# EUROPEAN PATENT APPLICATION

(11) **EP 3 716 274 A1**
(43) Date of publication of application: **30.09.2020**
(21) Application number: 20164165.1
(22) Date of filing: 19.03.2020
(51) Int. Cl.: G16C 20/10

(54) **CONTROL DEVICE, CONTROL METHOD, COMPUTER PROGRAM, AND METHOD FOR PRODUCING ORGANIC COMPOUND**

(30) Priority: 29.03.2019 JP 2019068866
(71) Applicant: AJINOMOTO CO., INC., Chuo-ku Tokyo 104-8315 (JP)
(72) Inventor: SHIMODAIRA, Yoshiki, Kawasaki-shi, Kanagawa 210-8681 (JP); KOBAYASHI, Hiroki, Kawasaki-shi, Kanagawa 210-8681 (JP); TERAWAKI, Takahiro, Kawasaki-shi, Kanagawa 210-8681 (JP); KUSUNOSE, Yasuhiro, Kawasaki-shi, Kanagawa 210-8681 (JP)
(74) Representative: Mewburn Ellis LLP

(57) **Abstract**

It is an object of the embodiment of the present invention to provide a control device, a control method, and a computer program that can determine an optimum, readable culture condition in real time and control a production result in production of an organic compound by a fermentation method, and the like. In the embodiment, the control device executes processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate. The linear model and the target production amount are set for each time.

## Description

### TECHNICAL FIELD

The present invention relates to a control device, a control method, a computer program, and a method for producing an organic compound.

### BACKGROUND ART

Method A and Method B below are main methods for stabilizing amino acid production by a fermentation method.
Method A: A method that optimizes conditions on, for example, a sugar concentration and a nutritional source concentration before the start of culture based on information on, for example, raw materials, which is known before the start of culture.
Method B: A method that repeats culture on the same condition and optimizes conditions on, for example, culture temperature, pH, and the amount of aeration based on a tendency of a plurality of production results in a trial- and-error manner before the start of culture.

However, Method A and Method B require that the conditions be set before the start of culture and cannot thus perform real-time control of the conditions according to situations during culture. It is considered that, for real-time control of the conditions, a model that estimates a future culture result is required.

In general, Method C and Method D below are main methods for developing the model.

Method C: A method that corrects general formulae based on fermentation technology and metabolic technology such as the Monod expression.

Method D: A method using a black box-like learning model such as a genetic algorithm or a neural network.

Patent Document 1 describes a technique on production of glutamic acid by a fermentation method. Specifically, Patent Document 1 describes a technique that determines a glutamic acid concentration during fermentation from a history of pH, temperature, the amount of aeration, and the like and determines pH, temperature, the amount of aeration optimum for optimizing the glutamic acid concentration using a three-layer neural network learned by back propagation.

### PRIOR ART REFERENCES

### PATENT DOCUMENT

### Patent Document 1: CN-B-104616072

### SUMMARY OF INVENTION

### PROBLEM TO BE SOLVED BY THE INVENTION

However, in actual production, there are many factors such as raw materials, and an expression obtained by Method C, which does not consider the factors, cannot achieve real-time control of the conditions according to situations during culture. In Method D, because a black box-like learning model is used, nobody can know what factor has a large influence on a culture result. That is, Method D is not readable. Consequently, it is unclear how the conditions should be controlled based on a result obtained by Method D.

The present invention has been made in view of the problems, and an object thereof is to provide a control device, a control method, and a computer program that can determine an optimum, readable culture condition in real time and control a production result in production of an organic compound by a fermentation method and a method for producing an organic compound that can achieve stable production of an organic compound by a fermentation method.

### MEANS FOR SOLVING PROBLEM

To solve the problems and to achieve the object, a control device according to one aspect of the present invention is a control device that performs control of a culture condition in production of an organic compound by a fermentation method, the control device executing processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate, and the linear model and the target production amount being set for each time.

In the control device according to another aspect of the present invention, the future time may be a time when next time processing is executed. In the control device according to still another aspect of the present invention, a candidate corresponding to a production amount at the future time closest to the target production amount may be determined to be the optimum candidate. In the control device according to still another aspect of the present invention, a plurality of the linear models may be set for each time, and the optimum candidate may be determined out of the candidates using a sample statistical amount for each of the candidates based on a plurality of production amounts at the future time for each of the candidates and the target production amount. In the control device according to still another aspect of the present invention, the linear model may be a multiple regression formula. In the control device according to still another aspect of the present invention, the organic compound may be an amino acid. In the control device according to still another aspect of the present invention, the amino acid may be a basic amino acid. In the control device according to still another aspect of the present invention, the basic amino acid may be lysine or arginine. In the control device according to still another aspect of the present invention, the culture condition may include a condition on temperature, pH, or a phosphoric acid concentration.

A control method according to one aspect of the present invention is a control method executed by a control device that performs control of a culture condition in production of an organic compound by a fermentation method, the control device executing processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate, and the linear model and the target production amount being set for each time.

A computer program according to one aspect of the present invention is a computer program causing a control device that performs control of a culture condition in production of an organic compound by a fermentation method to execute, the computer program causing the control device to execute processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate, and the linear model and the target production amount being set for each time.

A method for producing an organic compound according to one aspect of the present invention includes a step of producing an organic compound with a production system having the control device.

In the production method according to another aspect of the present invention, the organic compound may be an amino acid. In the production method according to still another aspect of the present invention, the amino acid may be a basic amino acid. In the production method according to still another aspect of the present invention, the basic amino acid may be lysine or arginine.

### EFFECT OF THE INVENTION

The present invention produces an effect of making it possible to determine an optimum, readable culture condition in real time and control a production result in production of an organic compound by a fermentation method. The present invention also produces an effect of making it possible to achieve stable production of an organic compound by a fermentation method.

### BRIEF DESCRIPTION OF DRAWINGS

FIG. 1 is diagram of an exemplary configuration of a culture system 1;
FIG. 2 is a diagram of an exemplary configuration of a control device 14;
FIG. 3 is a diagram of an exemplary flowchart about control processing;
FIG. 4 is a diagram of exemplary operating conditions;
FIG. 5 is a diagram of exemplary arithmetic signs ("+" or "-") to be attached to variables of an operating condition contained in a linear model;
FIG. 6 is a diagram of an exemplary operating condition range;
FIG. 7 is a diagram of an exemplary Lys culture result;
FIG. 8 is a diagram of an exemplary graph of the Lys culture result;
FIG. 9 is a diagram of exemplary operating conditions;
FIG. 10 is a diagram of exemplary arithmetic signs ("+" or "-") to be attached to the variables of the operating condition contained in the linear model;
FIG. 11 is a diagram of an exemplary operating condition range;
FIG. 12 is a diagram of an exemplary Lys culture result;
FIG. 13 is a diagram of an exemplary graph of Lys culture results;
FIG. 14 is a diagram of an exemplary graph of Lys culture results;
FIG. 15 is a diagram of an exemplary graph of Lys culture results;
FIG. 16 is a diagram of exemplary operating conditions;
FIG. 17 is a diagram of exemplary arithmetic signs ("+" or "-") to be attached to the variables of the operating condition contained in the linear model;
FIG. 18 is a diagram of an exemplary operating condition range;
FIG. 19 is a diagram of an exemplary Arg culture result; and
FIG. 20 is a diagram of an exemplary graph of the Arg culture result.

### EMBODIMENTS FOR CARRYING OUT THE INVENTION

The following explains an embodiment of a control device, a control method, a computer program, and a method for producing an organic compound according to the present invention in detail based on the accompanying drawings.

The present embodiment does not limit the present invention.

### 1. Configuration

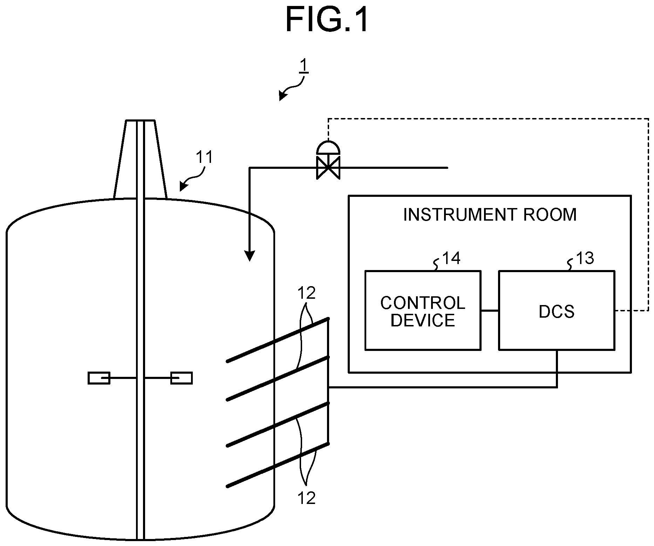

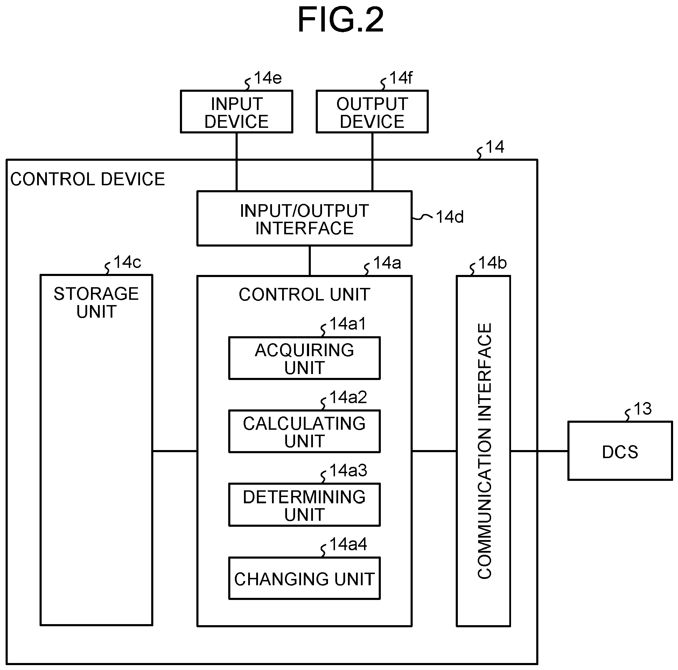

The following explains a configuration of a culture system 1 (corresponding to an exemplary production system) according to the present embodiment in detail with reference to FIG. 1 and FIG. 2. FIG. 1 is a diagram of an exemplary configuration of the culture system 1 according to the present embodiment. FIG. 2 is diagram of an exemplary configuration of a control device 14.

The culture system 1 mainly includes a culture tank 11, sensors 12, a distributed control system (DCS) 13, and the control device 14.

The culture tank 11 and the DCS 13 are included in a general culture device (specifically, a jar fermenter). The sensors 12 are for acquiring culture data and are included in a general culture device as a standard or added thereto. The culture data includes operation data (temperature, pH, or sugar supply amount, for example), near-infrared reflectance (NIR) data on a near infrared spectrum of a fermentation liquid, or property values (refractive index, conductivity, viscosity, or capacitance, for example), for example. The culture data may include pH, temperature, turbidity, a dissolved oxygen concentration, an ammonia nitrogen concentration, a sulfuric acid ion concentration, a nitrogen source concentration, a lactic acid concentration, a phosphorous source concentration, an ammonium sulfate concentration, an ammonium sulfate supply amount, a sugar-ammonium sulfate mixed liquid supply amount, an extracting liquid amount, an extracting cell amount, an amino acid concentration, amino acid yield, amino acid productivity, a phosphoric acid concentration, a phosphoric acid ion concentration, a phosphoric acid ion addition amount, a phosphoric acid ion consumption amount, a cell concentration, cell yield, a cell amount, a sugar concentration, a sugar consumption rate, a sugar supply amount, a specific multiplication rate, a specific sugar consumption rate, a specific production rate, an emission gas concentration, an oxygen consumption amount, oxygen demand, a carbon dioxide generation amount, respiratory quotient, rab, or a sugar liquid amount, for example.

The culture data may include values themselves acquired from the operation data or the like (pH, temperature, and a dissolved oxygen concentration, for example), analytical values obtained using other analyzers that are not included in the culture device such as HPLC (amino acid concentration and lactic acid concentration, for example), values obtained by performing arithmetic operations based on the values contained in the culture data (specific multiplication rate, amino acid productivity, and respiratory quotient, for example), or accumulated values of respective values from the start of culture to each culture interval calculated based on the values contained in the culture data (accumulated oxygen consumption amount, culture temperature accumulated value, specific multiplication rate accumulated value, and the like). Furthermore, the culture data may be acquired by soft sensors, for example. A statistical analytical method of soft sensors may be Partial Least Squares (PLS), Random Forest (RF), Extremely Randomized Trees (ERT), Least Absolute Shrinkage and Selection Operator (LASSO), Ridge, Elastic Net (EN), Linear Support Vector Regression (LSVR), Non-Linear Support Vector Regression (NLSVR), Gaussian Process (GP), Gene Algorithm-based Partial Least Squares (GA_PLS), Adaptive Boosting (AdaBoost), extreme Gradient Boosting (XGBoost), or Deep Neural Network (DNN), for example. Examples of technical literature on soft sensing include CN-A-107391851, WO 2018/007394, CN-A-107653274, CN-A-106444377, and CN-A-106022532.

Examples of a carbon source include carbohydrates such as starch, various sugars such as glucose and sucrose, and various organic acids. According to the type of anabolism of a selected microorganism, alcohol including ethanol and glycerol may be used.

Examples used as a nitrogen supply source include ammonia and various ammonium salts such as ammonium sulfate, other nitrogen compounds such as amine, and natural nitrogen sources such as peptone, soybean hydrolysates, and fermentable microorganism-digested objects.

Examples used as a phosphorous supply source include phosphoric acid, phosphates such as dipotassium monohydrogen phosphate and potassium dihydrogen phosphate, and phosphorous compounds such as myo-inositol.

The organic compound by a fermentation method is a product of a fermentation-and-culture process and is specifically selected from the group consisting of amino acids, organic acids, polysaccharides, proteins, antibiotics, alcohols, and microbial cells.

Examples of amino acids include glycine, alanine, valine, leucine, isoleucine, serine, threonine, cysteine, cystine, methionine, phenylalanine, tyrosine, tryptophan, proline, hydroxy proline, asparagine, glutamine, aspartic acid, glutamic acid, lysine, histidine, and arginine. The amino acids can be classified into any of an acidic amino acid, a basic amino acid, and a neutral amino acid depending on difference in their side chain. Specifically, glutamic acid and aspartic acid can be classified into the acidic amino acid, lysine, arginine, and histidine can be classified into the basic amino acid, and glycine, alanine, threonine, methionine, cysteine, serine, valine, leucine, isoleucine, tryptophan, phenylalanine, tyrosine, proline, glutamine, and asparagine can be classified into the neutral amino acid.

Examples of organic acids include acetic acid, lactic acid, pelvic acid, succinic acid, malic acid, itaconic acid, citric acid, acrylic acid, propionic acid, and fumaric acid. Examples of polysaccharides include xanthan, dextran, alginates, hyaluronic acid, curdlan, gellan, scleroglucan, and pullulan. Examples of proteins include hormones, lymphokine, interferon, enzymes (such as amylase, glucoamylase, invertase, lactase, protease, and lipase). Examples of antibiotics include antimicrobial agents (such as β-lactam, macrolide, ansamycin, tetracycline, chloramphenicol, peptide antibiotics, and aminoglycoside), antimold agents (such as polyoxin B, griseofulvin, and polyene macrolide), anticancer agents (such as daunomycin, adriamycin, dactinomycin, mithramycin, and bleomycin), protease/peptidase inhibitors (such as leupeptin, antipain, and pepstatin), and cholesterol biosynthesis inhibitors (such as compactin, lovastatin, and pravastatin). Examples of alcohols include ethanol, isopropanol (2-propanol), glycerin, propylene glycol, trimethylene glycol, 1-butanol, and sorbite. Examples of other organic compounds produced by a fermentation method include organic compounds such as acrylamide, diene compounds (such as isoprene), and pentanediamine. Techniques that produce the organic compounds by culturing microorganisms having organic compound productivity are widely known. The present embodiment can widely be applied to such microbial fermentation techniques. In microbial fermentation, microorganisms multiply themselves using a carbon source, a nitrogen source, or the like. In such a meaning, in the present embodiment, a fermentation product also includes microbial cells. Examples of microbial cells include any microorganisms having organic compound productivity.

Microorganisms having organic compound productivity include both 1) microorganisms that originally have organic compound productivity and 2) microorganisms that do not have or do not substantially have organic compound productivity originally but have had organic compound productivity a posteriori owing to an organic compound producing gene introduced by genetic modification. As to microorganisms having organic compound productivity, various kinds of microorganisms are known depending on the kind of organic compounds; in the present embodiment, these known microorganisms may widely be used. As far as ammonia can be used as a nitrogen source or a pH regulator in culture, the present embodiment can widely also be applied to microorganisms to be developed in the future.

As microorganisms, which are not limited to particular microorganisms so long as they have organic compound productivity, bacteria or fungi are preferred. Examples of bacteria include bacteria belonging to the genus Escherichia, bacteria belonging to the genus Pantoea, bacteria belonging to the genus Corynebacterium, bacteria belonging to the genus Enterobacter, bacteria belonging to the genus Clostridium, bacteria belonging to the genus Bacillus, bacteria belonging to the genus Lactobacillus, bacteria belonging to the genus Streptomyces, bacteria belonging to the genus Streptococcus, and bacteria belonging to the genus Pseudomonas. Examples of fungi include fungi belonging to the genus Saccharomyces, fungi belonging to the genus Schizosaccharomyces, fungi belonging to the genus Yarrowia, fungi belonging to the genus Trichoderma, fungi belonging to the genus Aspergillus, fungi belonging to the genus Fusarium, and fungi belonging to the genus Mucor.

Examples of bacteria belonging to the genus Escherichia include Escherichia coli. Examples of bacteria belonging to the genus Pantoea include Pantoea ananatis. Examples of bacteria belonging to the genus Corynebacterium include Corynebacterium glutamicum and Corynebacterium ammoniagenes. Examples of bacteria belonging to the genus Enterobacter include Enterobacter aerogenes. Examples of bacteria belonging to the genus Clostridium include Clostridium acetobutylicum. Examples of bacteria belonging to the genus Bacillus include Bacillus subtilis and Bacillus amyloliquefaciens. Examples of bacteria belonging to the genus Lactobacillus include Lactobacillus yamanashiensis, Lactobacillus animalis, Lactobacillus hilgardii, and Lactobacillus brevis. Examples of bacteria belonging to the genus Streptomyces include Streptomyces clavuligerus, Streptomyces venezuelae, and Streptomyces peucetius. Examples of bacteria belonging to the genus Streptococcus include Streptococcus equi and Streptococcus mutans. Examples of bacteria belonging to the genus Pseudomonas include Pseudomonas fluorescens, Pseudomonas aeruginosa, Pseudomonas elodea, and Pseudomonas putida. Examples of fungi include fungi belonging to the genus Saccharomyces include Saccharomyces cerevisiae. Examples of fungi belonging to the genus Schizosaccharomyces include Schizosaccharomyces pombe. Examples of fungi belonging to the genus Yarrowia include Yarrowia lipolytica. Examples of fungi belonging to the genus Trichoderma include Trichoderma reesei. Examples of fungi belonging to the genus Aspergillus include Aspergullus terreus and Aspergillus oryzae. Examples of fungi belonging to the genus Fusarium include Fusarium hetereosporum. Examples of fungi belonging to the genus Mucor include Mucor javanicus.

When an amino acid is produced in the present embodiment, examples of microorganisms that can suitably be used include the following. For example, when a target substance is L-lysine, examples include Escherichia coli AJ11442 (NRRL B-12185, FERM BP-1543) (refer to US-B-4,346,170), Brevibacterium lactofermentum AJ3990 (ATCC31269) (refer to US-B-4,066,501), and Lys-producing bacteria WC196LC/pCABD2 (WO 2010/061890). WC196AcadAAldc is a strain constructed by destroying cadA and ldcC genes, which encode lysine decarboxylase, from the WC196 strain. WC196ΔcadAΔldc/pCABD2 is a strain constructed by introducing the plasmid pCABD2 containing lysine biosynthesis genes (US-B-6,040,160) into WC196ΔcadAΔldc. WC196ΔcadAΔldc was designated AJ110692 and was deposited at the national research and development agency, National Institute of Advanced Industrial Science and Technology, International Patent Organism Depositary (currently the independent administrative agency, National Institute of Technology and Evaluation, International Patent Organism Depositary, #120, 2-5-8 Kazusakamatari, Kisarazu-shi, Chiba, 292-0818, Japan) as an accession number of FERM BP-11027 on Oct. 7, 2008. In the case of L-threonine, examples include Escherichia coli VKPM B-3996 (RIA 1867, VKPM B-3996) (refer to US-B-5,175,107) and Corynebacterium acetoacidophilum AJ12318 (FERM BP-1172) (refer to US-B-5,188,949). In the case of L-phenylalanine, examples include Escherichia coli AJ12604 (FERM BP-3579) (refer to EP-A-488424) and Brevibacterium lactofermentum AJ12637 (FERM BP-4160) (refer to FR-A-2686898). In the case of L-glutamic acid, examples include Escherichia coli AJ12624 (FERM BP-3853) (refer to FR-A-2680178), Brevibacterium lactofermentum AJ12475 (FERM BP-2922) (refer to US-B-5,272,067), and 2256ΔldhAΔsucAyggB* created with Corynebacterium glutamicum ATCC13869 as a mother strain (WO 2014/185430). In the case of L-leucine, examples include Escherichia coli AJ11478 (FERM P-5274) (refer to JP-B-62-34397) and Brevibacterium lactofermentum AJ3718 (FERM P-2516) (refer to US-B-3,970,519). In the case of L-isoleucine, examples include Escherichia coli KX141 (VKPM B-4781) (refer to EP-A-519113) and Brevibacterium flavum AJ12149 (FERM BP-759) (refer to US-B-4,656,135). In the case of L-valine, examples include Escherichia coli VL1970 (VKPM B-4411) (refer to EP-A-519113) and Brevibacterium lactofermentum AJ12341 (FERM BP-1763) (refer to US-B-5,188,948). fIn the case of L-arginine, examples include strains belonging to the genus Escherichia such as Escherichia coli 237 strain (VKPM B-7925) (US-A1-2002/058315), a derivative strain thereof holding modified N-acetylglutamate synthase (RU-A-2001112869), Escherichia coli 382 strain (VKPM B-7926) (EP-A1-1170358), and an arginine-producing strain into which the argA gene encoding N-acetylglutamate synthase is introduced (EP-A1-1170361); all of these examples are not limiting.

When an organic acid is produced in the present embodiment, examples of microorganisms that can suitably be used include the following. For example, when a target substance is L-lactic acid, examples include Lactobacillus yamanashiensis, Lactobacillus animalis, and Saccharomyces cerevisiae. In the case of pyruvic acid, examples include Escherichia coli and Pseudomonas fluorescens. In the case of succinic acid, examples include Escherichia coli and Pantoea ananatis. In the case of itaconic acid, examples include Aspergillus terreus. In the case of citric acid, examples include Escherichia coli (refer to WO 2007/097260 and JP-A-2010-187542, for example).

When polysaccharides are produced in the present embodiment, examples of microorganisms that can suitably be used include the following. For example, when a target substance is dextran, examples include Lactobacillus hilgardii and Streptococcus mutans. In the case of alginates, examples include Pseudomonas aeruginosa. In the case of hyaluronic acid, examples include Streptococcus equi and Streptococcus mutans. In the case of gellan, examples include Pseudomonas elodea (refer to JP-A-2011-116825 and JP-A-2007-9092, for example).

When a protein is produced in the present embodiment, examples of microorganisms that can suitably be used include the following. For example, when a target substance is various kinds of hormones or interferon, examples include Saccharomyces cerevisiae. In the case of amylase, glucoamylase, protease, or lipase, examples include Bacillus subtilis and Aspergillus oryzae. In the case of invertase and lactase, examples include Saccharomyces cerevisiae and Aspergillus oryzae (refer to WO 2006/67511 and JP-A-2003-153696, for example).

When an antibiotic is produced in the present embodiment, examples of microorganisms that can suitably be used include the following. For example, when a target substance is β-lactam such as penicillin, examples include Pseudomonas putida and Streptomyces clavuligerus. In the case of macrolide such as erythromycin and azithromycin, examples include Streptomyces venezuelae. In the case of daunomycin, examples include Streptomyces peucetius. In the case of pravastatin, examples include Streptomyces clavuligerus (refer to WO 96/10084, JP-A-2002-53589, WO 2005/54265, and WO 2007/147827, for example).

When an alcohol is produced in the present embodiment, examples of microorganisms that can suitably be used include the following. For example, when a target substance is ethanol, examples include Saccharomyces cerevisiae, Schizosaccharomyces pombe, and Lactobacillus brevis. In the case of trimethylene glycol, examples include Escherichia coli (refer to WO 2007/97260, for example).

The sensors 12 and the DCS 13 are connected to each other in a communicable manner via a wired or wireless communication line. The DCS 13 and the control device 14 are connected to each other in a communicable manner via a wired or wireless communication line. The number of the sensors 12 is not limited to four as depicted. The control device 14 may be present as a separate casing from the DCS 13 as depicted or present as the same casing as the DCS 13.

The control device 14 performs control of a culture condition in production of an organic compound by a fermentation method. The control device 14 executes processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate. In the control device 14, the linear model and the target production amount are set for each time. A time interval between a time when an nth (n is an integer of equal to or more than 1) processing is executed and a time when an (n+1)th processing is executed may be any time interval.

The control device 14 includes a control unit 14a, such as CPU (Central Processing Unit), that integrally controls the device, a communication interface 14b that connects the device to the DCS 13 in a communicable manner via a wired or wireless communication line, a storage unit 14c that stores various databases, tables, files and others, and an input/output interface 14d connected to an input device 14e and an output device 14f. These units included in the control device 14 are connected to each other in a communicable manner via any communication channel.

A keyboard, a mouse, a microphone, a monitor functioning as a pointing device together with a mouse, a touch screen, or the like may be used as the input device 14d. A monitor, a speaker, a printer, or the like may be used as the output device 14f.

The storage unit 14c is a storage means. A memory device such as RAM (Random Access Memory) and ROM (Read Only Memory), a fixed disk drive such as a hard disk, a flexible disk, an optical disk, or the like may be used as the storage unit 14c. The storage unit 14c may store computer programs giving instructions to the CPU for various processings, together with OS (Operating System).

The control unit 14a has an internal memory storing control programs such as OS, programs for various processing procedures, and needed data, and performs various information processings according to these programs.

The control unit 14a functionally conceptually includes an acquiring unit 14a1, a calculating unit 14a2, a determining unit 14a3, and a changing unit 14a4. The control unit 14a operates the acquiring unit 14a1, the calculating unit 14a2, the determining unit 14a3, and the changing unit 14a4 a plurality of times.

The acquiring unit 14a1 is an information processing unit that acquires the culture data. The acquiring unit 14a1 may acquire the culture data measured by the sensors 12 acquired by, for example, the DCS 13 from the DCS 13.

The calculating unit 14a2 is an information processing unit that calculates, using the culture data acquired by the acquiring unit 14a1, a plurality of candidates for a culture condition set in advance, and a linear model set in advance that outputs a production amount of an organic compound at a future time, the production amount at the future time for each of the candidates. The future time may be a time when next time processing is executed, for example. A plurality of the linear models may be set for each time, for example. The linear model may be a multiple regression formula, for example. The candidates for the culture condition may be configured by culture conditions for respective times (a first culture condition, a second culture condition, ...), for example. The culture condition may be an operating condition of a general culture device, for example. The culture condition may be a condition on temperature, pH, or a phosphoric acid concentration, for example.

The determining unit 14a3 is an information processing unit that determines an optimum candidate out of the candidates for the culture condition using the production amount at the future time for each of the candidates calculated by the calculating unit 14a2 and a target production amount of the organic compound at the future time set in advance. When the linear models are set for each time, the determining unit 14a3 may determine the optimum candidate out of the candidates using a sample statistical amount (an average, for example) for each of the candidates based on a plurality of production amounts at the future time for each of the candidates and the target production amount, for example. The determining unit 14a3 may determine a candidate corresponding to a production amount at the future time closest to the target production amount or the sample statistical amount to be the optimum candidate, for example. The sample statistical amount refers to a value representing a characteristic of data such as an average.

The changing unit 14a4 is an information processing unit that changes the culture condition to the candidate determined by the determining unit 14a3. When the culture condition is an operating condition of the culture device, the changing unit 14a4 may change the operating condition set in the DCS 13 to the candidate determined by the determining unit 14a3, for example.

### 2. Processing

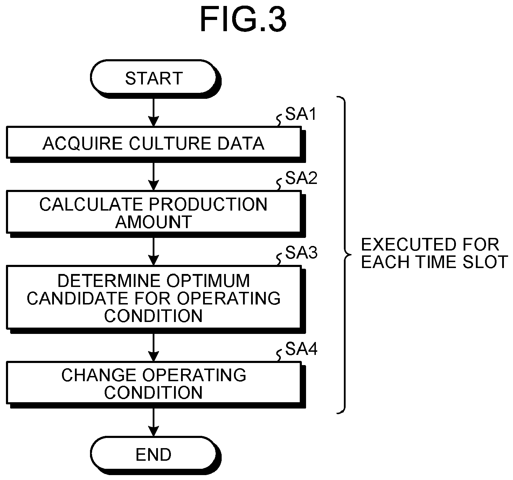

The following explains exemplary control processing executed by the control device 14 with reference to FIG. 3. It is premised in the present explanation that a time schedule based on a time elapsed from the start of culture (culture start N₁ time, culture start N₂ (N₁<N₂) time, culture start N₃ (N₂<N₃) time, ..., for example) is set. It is also premised in the present explanation that each of the candidates for the operating condition of the culture device includes the operating condition for each time set in the time schedule.

First, upon a first time in the time schedule, the control unit 14a causes the acquiring unit 14a1 to operate, and the acquiring unit 14a1 acquires culture data corresponding to the first time from the DCS 13 (Step SA1).

Next, the calculating unit 14a2 inputs the culture data acquired at Step SA1 and a plurality of candidates for the operating condition to a linear model that outputs a production amount of an organic compound at a second time in the time schedule to calculate a production amount of the organic compound at the second time for each of the candidates (Step SA2).

Next, the determining unit 14a3 compares the production amount for each of the candidates calculated at Step SA2 and a target production amount at the second time in the time schedule with each other in magnitude and determines a candidate corresponding to a production amount closest to the target production amount to be an optimum candidate (Step SA3).

Next, the changing unit 14a4 changes the operating condition set in the DCS 13 to the candidate for the operating condition determined at Step SA3 (Step SA4).

Then, the control unit 14a repeatedly executes the processing from Step SA1 to Step SA4 according to the time schedule.

### 3. Summary of Present Embodiment

As explained in the foregoing, the present embodiment repeatedly executes a series of processing including (1) acquisition of the culture data, (2) calculation of the future production amount for each of the candidates using the linear model, (3) determination of the optimum candidate for the culture condition, and (4) change of the culture condition according to, for example, the time schedule during culture and can determine an optimum, readable culture condition in real time and control a production result in production of an organic compound by a fermentation method. The present embodiment can achieve real-time control of the culture condition based on high estimation accuracy. The present embodiment can optimize a production amount of the organic compound by, for example, optimizing the operating condition in real time during culture operation, and by extension, enables stable production of the organic compound throughout the year in a plant.

The linear model used in the present embodiment is created based on the data during culture (is created by executing Processing 1 through Processing 6 below, for example), and the present embodiment can cope with various production situations that cannot be represented by general formulae based on fermentation technology and metabolic technology such as the Monod expression. That is, the present embodiment enables not only control on a laboratory level but also enables control on a bench plant scale, a pilot plant scale, or an actual device scale.

The linear model used in the present embodiment can be created by executing Processing 1 through Processing 6 below, for example.
[Processing 1] Data during culture is collected. As to the data to be collected, not only variables indicating a fermentation state (amino acid concentration, sugar concentration, cell concentration, or the like) but also data on infinitesimal components in a liquid (other amino acids or organic acids) is acquired. Specifically, NIR data, operation data, or property value data is acquired. For the operating condition, which is not the same operating condition, data is acquired on various conditions (variable temperature, pH, or the like). When an experimental condition is determined, thought is given to make variables close to a normal distribution.
[Processing 2] A response variable to be modeled (amino acid production amount) and explanatory variables to be used in the model are determined.
[Processing 3] Coefficients of correlation of the response variable and the explanatory variables are calculated to select explanatory variables with a high coefficient of correlation.
[Processing 4] Using Akaike's Information Criterion (AIC), the explanatory variables are narrowed down by the stepwise method.
[Processing 5] Based on the narrowed down explanatory variables, models are created by the variable coverage method.
[Processing 6] Models with a high coefficient of correlation (top 1,000 models or models with a coefficient of correlation of equal to or more than 0.7, for example) are extracted.

### 4. Other embodiments

In addition to the embodiment described above, the present invention can be practiced in various different embodiments within the technological scope of the claims.

Of the processings described in the embodiment, all or a part of the processings described as automatically performed ones may be manually performed, or all or a part of the processings described as manually performed ones may be also automatically performed by known methods.

The components of the devices shown in the figures are functionally conceptual and therefore not be physically configured as shown in the figures. A specific configuration of dispersion or integration of the system is not limited to the shown one. The system can be configured by functionally or physically dispersing or integrating the devices in arbitrary units.

For the operational functions provided in the control device 14, in particular, for the operational functions performed in the control unit 14a, all or part thereof may be implemented by the CPU and programs interpreted and executed in the CPU. The program is recorded in a non-transitory tangible computer-readable recording medium including programmed instructions for making an information processing apparatus execute the control method according to the present invention, and is mechanically read as needed by the control device 14. More specifically, computer programs to give instructions to the CPU in cooperation with the OS to perform various processes are recorded in the storage unit 14c such as ROM or a HDD (hard disk drive). The computer programs are executed by being loaded to RAM, and form the control unit in cooperation with the CPU.

The control program according to the present invention may be stored in the non-transitory tangible computer-readable recording medium, or can be configured as a program product. The "recording medium" mentioned here includes any "portable physical medium" such as a memory card, a USB (universal serial bus) memory, an SD (secure digital) card, a flexible disk, a magneto-optical disc, ROM, EPROM (erasable programmable read only memory), EEPROM (registered trademark) (electronically erasable and programmable read only memory), CD-ROM (compact disk read only memory), MO (magneto-optical disk), DVD (digital versatile disk), and Blu-ray (registered trademark) Disc.

### Example 1

In Example 1, the operating condition was optimized using a linear model (specifically, a multiple regression formula) on a lysine production amount, whereby control of the lysine production amount was performed. The linear model used in Example 1 was created by a statistical method explained in the present embodiment based on experimental data to model a lysine fermentation process.

### 1. Control Method

A culture time from the start of culture to the end of culture was divided into six intervals (Interval 1, Interval 2, Interval 3, Interval 4, Interval 5, and Interval 6), and control of the operating condition indicated by (1) through (4) below was performed. Before the start of culture, a lysine production amount as a target production amount was set at the end of each interval (at the end of the current interval, for example). Before the start of culture, candidates (about eight candidates) for the operating condition were determined for each interval. In the examples described in the present specification, the length of a time of each interval may be any time; the length of the time is preferably 2 hours or more and 12 hours or less, and more preferably 4 hours or more and 8 hours or less.
(1) Measurement or analysis of pH, temperature, a phosphoric acid concentration, a sulfuric acid concentration, a nitrogen source concentration, a sugar supply amount, a sampling amount, an extracting liquid amount, a lysine concentration, turbidity (OD), a sugar concentration, an ammonium sulfate supply amount, an emission gas concentration, and a dissolved oxygen concentration is performed at the beginning of the current interval (corresponding to the processing performed by the acquiring unit 14a1 described in the embodiment). Using the obtained measurement result or analysis result, all items as variables of the linear model are calculated.
(2) Using the obtained analysis data, data on the current operating condition, and a linear model that predicts a lysine production amount at the end of the current interval, the lysine production amount at the end is predicted (corresponding to the processing performed by the calculating unit 14a2 described in the embodiment). The linear model incorporates the operating condition as a variable, and the predicted value of the lysine production amount varies for each of the candidates for the operating condition. The linear model is set for each interval.
(3) The operating condition on which the predicted value is the closest to the lysine production amount as the target production amount at the end is selected (corresponding to the processing performed by the determining unit 14a3 described in the embodiment).
(4) The operating condition is changed to the selected operating condition (corresponding to the processing performed by the changing unit 14a4 described in the embodiment).

### 2. Experimental Conditions

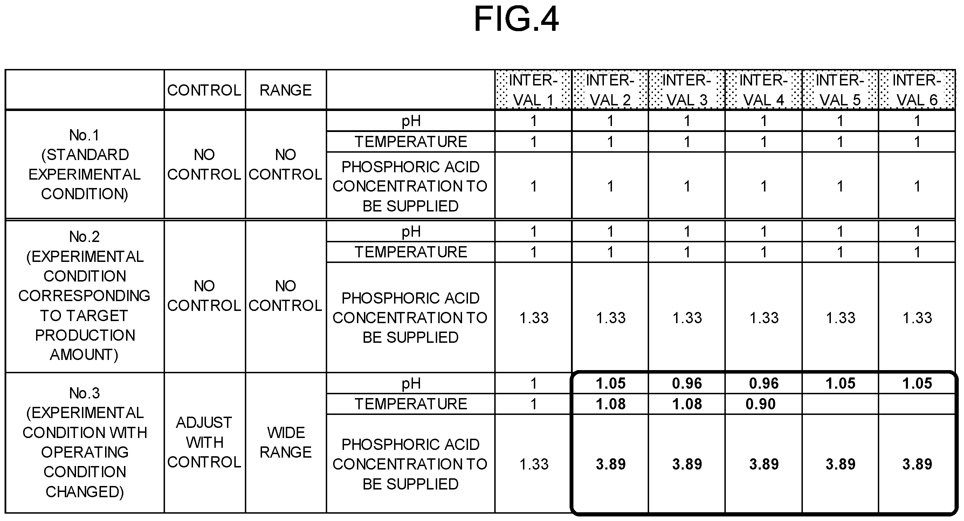

FIG. 4 depicts experimental conditions. The No.1 row shows a standard experimental condition. The No.2 row shows an experimental condition corresponding to the target production amount. In FIG. 4, the values of the operating condition are represented as relative values to the values of the No.1 operating condition.

The No.3 row shows an experimental condition with the operating condition changed from Interval 2 with a fermentation result obtained on the No.2 experimental condition as the target production amount. By comparing the No.2 and No.3 results with each other, the effect of optimization of the operating condition is confirmed.

### 3. Used Linear Model

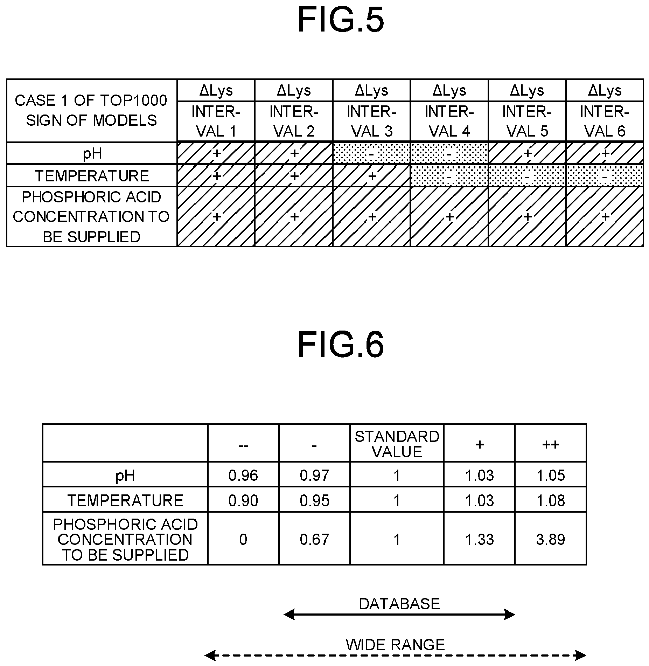

FIG. 5 depicts arithmetic signs ("+" or "-") to be attached to control variables (specifically, pH, temperature, and phosphoric acid concentration to be supplied) contained in a used linear model. The used linear model maximizes estimation performance explored by multiple regression analysis (the variable coverage method based on AIC minimum criterion) and was evaluated by an adjusted coefficient of determination with leave-one-out cross-validation performed and a coefficient of correlation with leave-one-out cross-validation performed.

In a linear model that predicts a lysine production amount at the end of Interval 1 (the amount of lysine produced in the culture time of Interval 1) based on data at the beginning of Interval 1 (top 1,000 expressions with a high adjusted coefficient of determination with leave-one-out cross-validation performed and a high coefficient of correlation with leave-one-out cross-validation performed), the arithmetic sign of the median of the coefficient of each control variable was "+." The top 1,000 expressions are described in [11. Linear Model that Predicts Lysine Production Amount in Interval 1].

In a linear model that predicts a lysine production amount at the end of Interval 2 (the amount of lysine produced in the culture time of Interval 2) based on data at the beginning of Interval 2 (top 1,000 expressions with a high adjusted coefficient of determination with leave-one-out cross-validation performed and a high coefficient of correlation with leave-one-out cross-validation performed), the arithmetic sign of the median of the coefficient of each control variable was "+." The top 1,000 expressions are described in [12. Linear Model that Predicts Lysine Production Amount in Interval 2].

In a linear model that predicts a lysine production amount at the end of Interval 3 (the amount of lysine produced in the culture time of Interval 3) based on data at the beginning of Interval 3 (top 1,000 expressions with a high adjusted coefficient of determination with leave-one-out cross-validation performed and a high coefficient of correlation with leave-one-out cross-validation performed), the arithmetic sign of the median of the coefficient of pH as a control variable was "-," whereas the arithmetic sign of the median of the coefficients of temperature and a phosphoric acid concentration to be supplied as control variables was "+." A list of the top 1,000 expressions is described in [13. Linear Model that Predicts Lysine Production Amount in Interval 3].

In a linear model that predicts a lysine production amount at the end of Interval 4 (the amount of lysine produced in the culture time of Interval 4) based on data at the beginning of Interval 4 (top 1,000 expressions with a high adjusted coefficient of determination with leave-one-out cross-validation performed and a high coefficient of correlation with leave-one-out cross-validation performed), the arithmetic sign of the median of the coefficients of pH and temperature as control variables was "-," whereas the arithmetic sign of the median of the coefficient of a phosphoric acid concentration to be supplied as a control variable was "+." A list of the top 1,000 expressions is described in [14. Linear Model that Predicts Lysine Production Amount in Interval 4].

In a linear model that predicts a lysine production amount at the end of Interval 5 (the amount of lysine produced in the culture time of Interval 5) based on data at the beginning of Interval 5 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "-," whereas the arithmetic sign of the median of the coefficients of pH and a phosphoric acid concentration to be supplied as control variables was "+." A list of the top 1,000 expressions is described in [15. Linear Model that Predicts Lysine Production Amount in Interval 5].

In a linear model that predicts a lysine production amount at the end of Interval 6 (the amount of lysine produced in the culture time of Interval 6) based on data at the beginning of Interval 6 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "-," whereas the arithmetic sign of the median of the coefficients of pH and a phosphoric acid concentration to be supplied as control variables was "+." A list of the top 1,000 expressions is described in [16. Linear Model that Predicts Lysine Production Amount in Interval 6].

### 4. Operating Condition Range

The control variables changed during fermentation operation were pH, temperature, and a phosphoric acid concentration to be supplied. FIG. 6 depicts an operating condition range. For the creation of the linear model, data acquired within a range denoted as "DATABASE" in FIG. 6 was used. In view of the prediction accuracy of the linear model, the data acquired within this range is preferably used, but data acquired outside this range (one by extrapolation) may also be used. By increasing the change range of the operating condition, the effect of a change of the operating condition on fermentation may be able to be increased, and in Example 1 the operating condition was changed within a range denoted as "WIDE RANGE" in FIG. 6 (a range wider than the range denoted as "DATABASE" in FIG. 6), which is considered to be hard to affect fermentation from the past knowledge in fermentation process development. In FIG. 6, the values of the operating condition are represented as relative values to the values of the No.1 operating condition depicted in FIG. 4.

### 5. Lysine Concentration Analysis

As a lysine concentration, one measured by a biosensor was used. To prevent difference in an analytical system from having influence, as a fermentation result as a target also, an analytical value of the biosensor was used. For the creation of the linear model, a lysine concentration measured by high performance liquid chromatography (HPLC) was used.

### 6. Results and Discussion

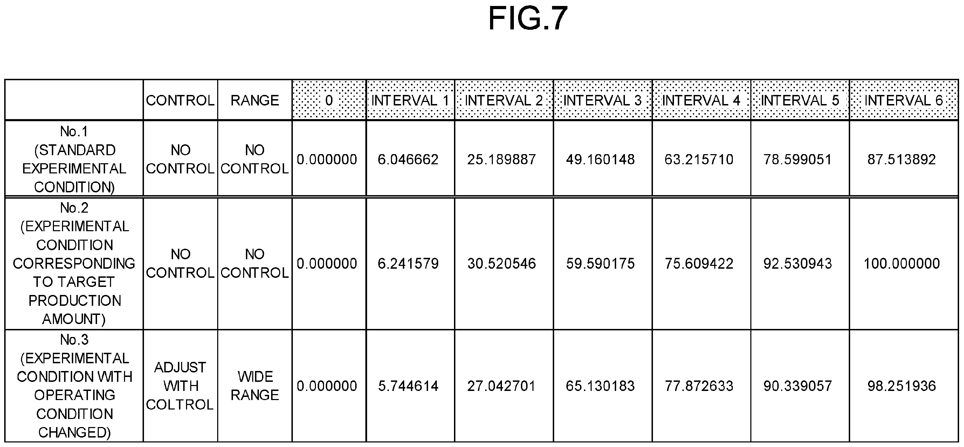

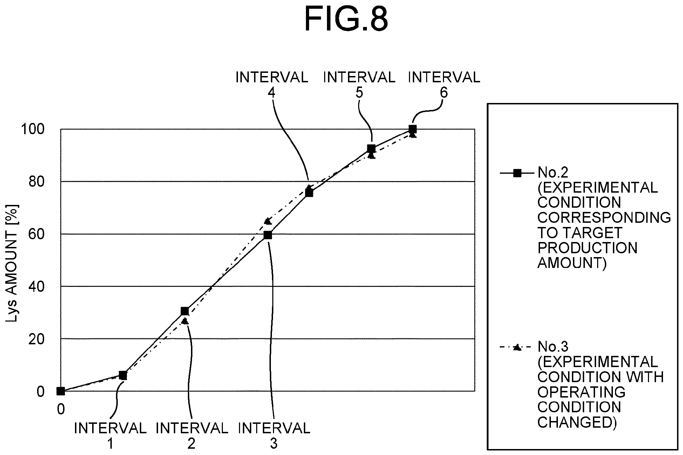

FIG. 7 and FIG. 8 depict culture results by the respective experimental conditions.

Optimization of the No.3 operating condition was performed from Interval 2 with the lysine production amount obtained on the No.2 operating condition as the target production amount. Consequently, the No.3 operating condition was changed to increase the lysine production amount from Interval 2 to Interval 6 (refer to the solid line frame in FIG. 4).

The lysine production amount obtained on the No. 3 experimental condition changed with substantially the same values as the lysine production amount obtained on the No.2 experimental condition. It is considered that this result means that controlling the lysine production amount to a value close to the target production amount succeeded by performing optimization of the operating condition using the linear model from Interval 2.

### Example 2

In Example 2, the operating condition was optimized using the linear model of Example 1 to renew control of the lysine production amount.

### 1. Control Method

The details are explained in Example 1 and are omitted.

### 2. Experimental Conditions

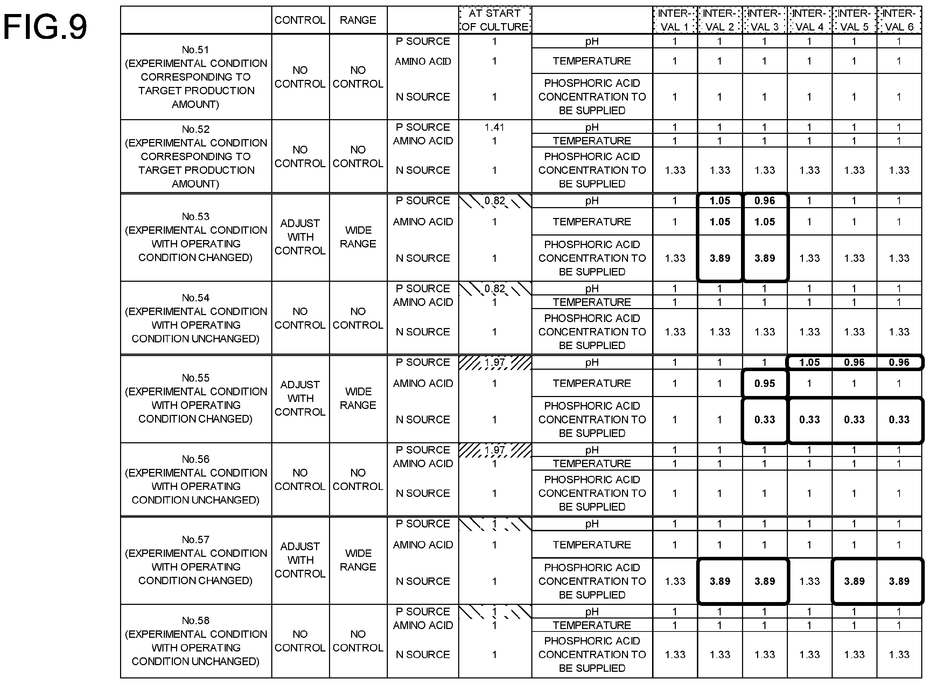

FIG. 9 depicts experimental conditions. The No. 51 row shows an experimental condition corresponding to the target production amount. The No. 52 row shows an experimental condition corresponding to the target production amount (the same as the No.2 experimental condition in FIG. 4), which was the largest in the lysine production amount in the experiment with the culture device (the jar fermentor) described in Example 1. In FIG. 9, the values of the operating condition are represented as relative values to the values of the No. 51 operating condition.

The No. 53 row shows an experimental condition with the operating condition changed from Interval 2 with a fermentation result obtained on the No. 52 experimental condition (the same as the No.2 experimental condition in FIG. 4) as the target production amount. The No. 54 row shows an experimental condition with the operating condition in Interval 1 the same as that of No. 53 and with the operating condition unchanged from Interval 2 on. The No. 54 experimental condition is one with which the No. 53 experimental condition is compared. The phosphoric acid concentration at the start of culture contained in the No. 53 experimental condition is lower than that contained in the No. 52 experimental condition (refer to FIG. 9). For this reason, the lysine production amount at the early state of culture on the No. 53 experimental condition is expected to be smaller than that on the No. 52 experimental condition. Given this, whether a lysine production amount comparable to the lysine production amount obtained on the No. 52 experimental condition can be obtained on the No. 53 experimental condition is confirmed by performing optimization of the operating condition from a state in which the lysine production amount is small.

The No. 55 row shows an experimental condition with the operating condition changed from Interval 2 with a fermentation result obtained on the No. 51 experimental condition as the target production amount. The No. 56 row shows an experimental condition with the operating condition in Interval 1 the same as that of No. 55 and with the operating condition unchanged from Interval 2 on. The No. 56 experimental condition is one with which the No. 55 experimental condition is compared. The phosphoric acid concentration at the start of culture contained in the No. 55 experimental condition is higher than that contained in the No. 51 experimental condition (refer to FIG. 9). For this reason, the lysine production amount at the early state of culture on the No. 55 experimental condition is expected to be larger than that on the No. 51 experimental condition. Given this, whether the lysine production amount can be reduced on the No. 55 experimental condition, which changes the operating condition, is confirmed.

The No. 57 row shows an experimental condition with the operating condition changed from Interval 2 with the fermentation result obtained on the No. 52 experimental condition as the target production amount. The No. 58 row shows an experimental condition with the operating condition in Interval 1 the same as that of No. 57 and with the operating condition unchanged from Interval 2 on. The No. 58 experimental condition is one with which the No. 57 experimental condition is compared. The No. 53 experimental condition and the No. 57 experimental condition are different from each other in that the No. 53 experimental condition simultaneously changes all the control variables (pH, temperature, and phosphoric acid concentration to be supplied), whereas the No. 57 experimental condition changes only the phosphoric acid concentration to be supplied.

### 3. Used Linear Model

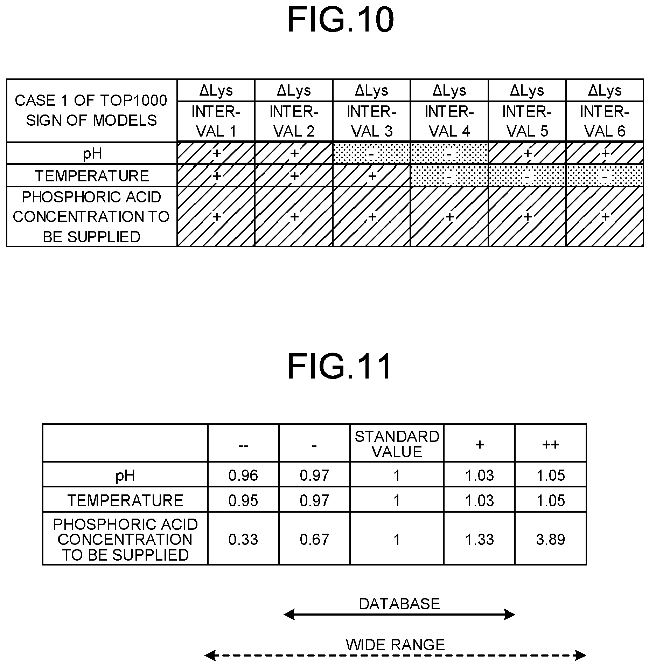

FIG. 10 depicts arithmetic signs ("+" or "-") to be attached to control variables contained in a used linear model. The details are explained in Example 1 and are omitted.

### 4. Operating Condition Range

The control variables changed during fermentation operation were pH, temperature, and a phosphoric acid concentration to be supplied. FIG. 11 depicts an operating condition range. For the creation of the linear model, data acquired within a range denoted as "DATABASE" in FIG. 11 was used. In view of the prediction accuracy of the linear model, the data acquired within this range is preferably used, but data acquired outside this range (one by extrapolation) may also be used. By increasing the change range of the operating condition, the effect of a change of the operating condition on fermentation may be able to be increased, and in Example 2 the operating condition was changed within a range denoted as "WIDE RANGE" in FIG. 11 (a range wider than the range denoted as "DATABASE" in FIG. 11), which is considered to be hard to affect fermentation from the past knowledge in fermentation process development. In FIG. 11, the values of the operating condition are represented as relative values to the values of the No. 51 operating condition depicted in FIG. 9.

### 5. Lysine Concentration Analysis

The details are explained in Example 1 and are omitted.

### 6. Results and Discussion

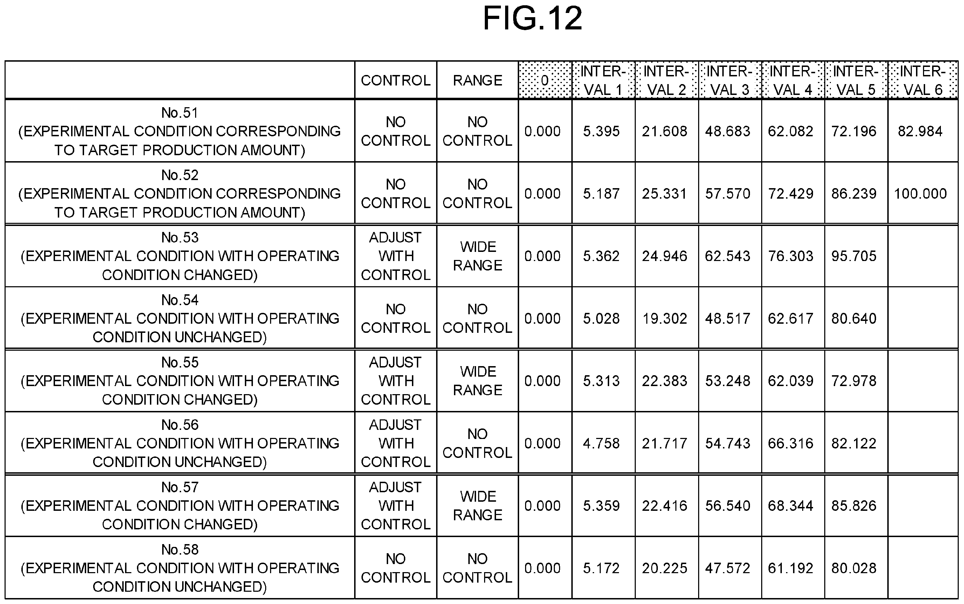

FIGS. 12 to 15 depict culture results by the respective experimental conditions.

### 6-1. Results and Discussion of No. 53 and No. 54

Optimization of the No. 53 operating condition was performed from Interval 2 with a lysine production amount obtained on the No. 52 operating condition as the target production amount. Consequently, the No. 53 operating condition was changed to increase the lysine production amount in Interval 2 and Interval 3 (refer to the solid line frame in FIG. 9).

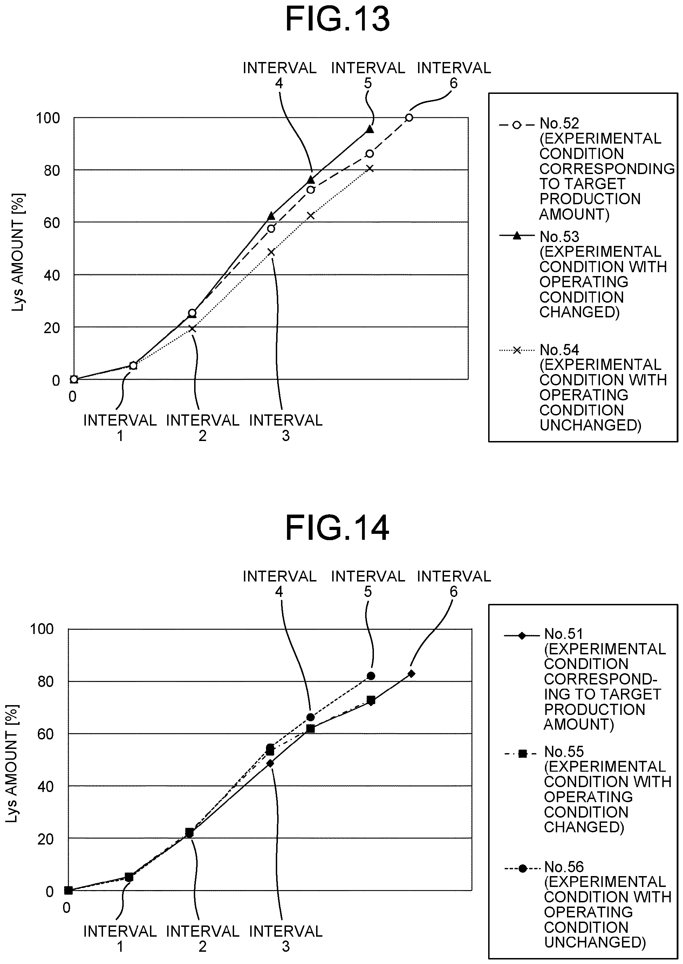

FIG. 12 and FIG. 13 depict culture results. The lysine production amount obtained on the No. 54 experimental condition (without any change of the operating condition) was smaller than the lysine production amount obtained on the No. 52 experimental condition. It is considered that this result is caused by intentionally decreasing the phosphoric acid concentration at the start of culture. On the other hand, the lysine production amount obtained on the No. 53 experimental condition, which was the same as the No. 54 experimental condition in the phosphoric acid concentration at the start of culture, was larger than the lysine production amount obtained on the No. 52 experimental condition from the middle stage of culture on. It is considered that this result means that increasing the lysine production amount succeeded by performing optimization of the operating condition using the linear model from Interval 2.

### 6-2. Results and Discussion of No. 55 and No. 56

Optimization of the No. 55 operating condition was performed from Interval 2 with a lysine production amount obtained on the No. 51 operating condition as the target production amount. Consequently, the No. 55 operating condition was changed to decrease the lysine production amount from Interval 3 on (refer to the solid line frame in FIG. 9).

FIG. 12 and FIG. 14 depict culture results. The lysine production amount obtained on the No. 56 experimental condition (without any change of the operating condition) was smaller than the lysine production amount obtained on the No. 51 experimental condition. It is considered that this result is caused by increasing the phosphoric acid concentration at the start of culture. On the other hand, the lysine production amount obtained on the No. 55 experimental condition, which was the same as the No. 56 experimental condition in the phosphoric acid concentration at the start of culture, was comparable to the lysine production amount obtained on the No. 51 experimental condition from the middle stage of culture on. It is considered that this result means that decreasing the lysine production amount succeeded by changing the operating condition using the linear model from Interval 2. That is, it is confirmed that the present embodiment can both increase and decrease the lysine production amount.

### 6-3. Results and Discussion of No. 57 and No. 58

Optimization of the No. 57 operating condition was performed from Interval 2 with the lysine production amount obtained on the No. 52 operating condition as the target production amount. Consequently, the No. 57 operating condition was changed to increase the lysine production amount in Interval 2, Interval 3, Interval 5, and Interval 6 (refer to the solid line frame in FIG. 9).

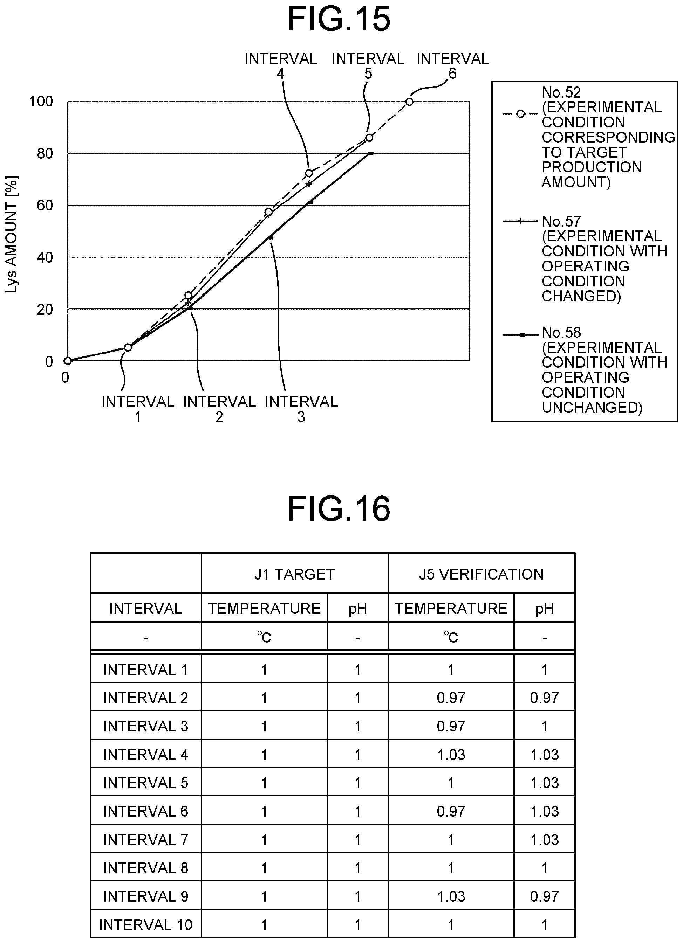

FIG. 12 and FIG. 15 depict culture results. The lysine production amount obtained on the No. 58 experimental condition (without any change of the operating condition) was smaller than the lysine production amount obtained on the No. 52 experimental condition. It is considered that this result is caused by decreasing the phosphoric acid concentration at the start of culture. On the other hand, the lysine production amount obtained on the No. 57 experimental condition, which was the same as the No. 58 experimental condition in the phosphoric acid concentration at the start of culture, was comparable to the lysine production amount obtained on the No. 52 experimental condition from the middle stage of culture on. It is considered that this result means that increasing the lysine production amount succeeded by performing optimization of the operating condition using the linear model from Interval 2.

### Example 3

In Example 3, the operating condition was optimized using the linear model (specifically, the multiple regression formula) on an arginine production amount, whereby control of the arginine production amount was performed. The linear model used in Example 3 is created by the statistical method explained in the present embodiment based on experimental data to model an arginine fermentation process.

### 1. Control Method

A culture time from the start of culture to the end of culture was divided into ten intervals (Interval 1, Interval 2, Interval 3, Interval 4, Interval 5, Interval 6, Interval 7, Interval 8, Interval 9, and Interval 10), and control of the operating condition indicated by (1) through (4) below was performed. Before the start of culture, an arginine production amount as a target production amount was set at the end of each interval (at the end of the current interval, for example). Before the start of culture, candidates (about eight candidates) for the operating condition were determined for each interval.
(1) Measurement or analysis of pH, temperature, a sugar supply amount, an ammonium sulfate supply amount, a sampling amount, an arginine concentration, turbidity (OD), a sugar concentration, and an ammonium sulfate concentration is performed at the beginning of the current interval (corresponding to the processing performed by the acquiring unit 14a1 described in the embodiment). Using the obtained measurement result or analysis result, all items as variables of the linear model (specific multiplication rate, specific production rate, sugar consumption rate, and the like) are calculated.
(2) Using the obtained analysis data, data on the current operating condition, and a linear model that predicts an arginine production amount at the end of the current interval, the arginine production amount at the end is predicted (corresponding to the processing performed by the calculating unit 14a2 described in the embodiment). The linear model incorporates the operating condition as a variable, and the predicted value of the arginine production amount varies for each of the candidates for the operating condition. The linear model is set for each interval.
(3) The operating condition on which the predicted value is the closest to the arginine production amount as the target production amount at the end is selected (corresponding to the processing performed by the determining unit 14a3 described in the embodiment). Specifically, culture was started on the same condition as that of the target, that the arginine production amount increases or decreases relative to the target based on the predicted value of the linear model was confirmed, and that the arginine production amount comparable to the target was given in the end was verified.
(4) The operating condition is changed to the selected operating condition (corresponding to the processing performed by the changing unit 14a4 described in the embodiment).

### 2. Experimental Conditions

FIG. 16 depicts experimental conditions. The row denoted as J1 target shows an experimental condition corresponding to the target production amount. The row denoted as J5 verification shows an experimental condition with the operating condition changed from Interval 2 with a fermentation result obtained on the experimental condition of J1 target as the target production amount. By comparing the results of J1 target and J5 verification with each other, the effect of optimization of the operating condition is confirmed. In FIG. 16, the values of the operating condition are represented as relative values to the values of the operating condition of J1 target.

### 3. Used Linear Model

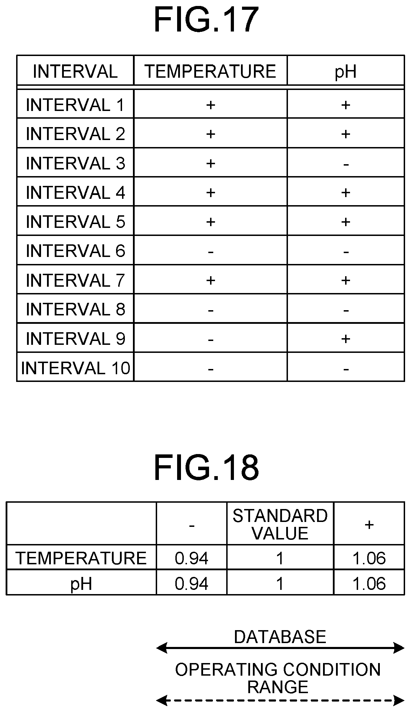

FIG. 17 depicts arithmetic signs ("+" or "-") to be attached to control variables (specifically, pH and temperature) contained in a used linear model. The used linear model maximizes estimation performance explored by multiple regression analysis (the variable coverage method based on AIC minimum criterion) and was evaluated by an adjusted coefficient of determination with leave-one-out cross-validation performed and a coefficient of correlation with leave-one-out cross-validation performed.

In a linear model that predicts an arginine production amount at the end of Interval 1 (the amount of arginine produced in the culture time of Interval 1) based on data at the beginning of Interval 1 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "+," and the arithmetic sign of the median of the coefficient of pH as a control variable was "+." The top 1,000 expressions are described in [201. Linear Model that Predicts Arginine Production Amount in Interval 1].

In a linear model that predicts an arginine production amount at the end of Interval 2 (the amount of arginine produced in the culture time of Interval 2) based on data at the beginning of Interval 2 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "+," and the arithmetic sign of the median of the coefficient of pH as a control variable was "+." The top 1,000 expressions are described in [202. Linear Model that Predicts Arginine Production Amount in Interval 2].

In a linear model that predicts an arginine production amount at the end of Interval 3 (the amount of arginine produced in the culture time of Interval 3) based on data at the beginning of Interval 3 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "+," whereas the arithmetic sign of the median of the coefficient of pH as a control variable was "-." A list of the top 1,000 expressions is described in [203. Linear Model that Predicts Arginine Production Amount in Interval 3].

In a linear model that predicts an arginine production amount at the end of Interval 4 (the amount of arginine produced in the culture time of Interval 4) based on data at the beginning of Interval 4 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "+," and the arithmetic sign of the median of the coefficient of pH as a control variable was "+." A list of the top 1,000 expressions is described in [204. Linear Model that Predicts Arginine Production Amount in Interval 4].

In a linear model that predicts an arginine production amount at the end of Interval 5 (the amount of arginine produced in the culture time of Interval 5) based on data at the beginning of Interval 5 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "+," and the arithmetic sign of the median of the coefficient of pH as a control variable was "+." A list of the top 1,000 expressions is described in [205. Linear Model that Predicts Arginine Production Amount in Interval 5].

In a linear model that predicts an arginine production amount at the end of Interval 6 (the amount of arginine produced in the culture time of Interval 6) based on data at the beginning of Interval 6 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "-," and the arithmetic sign of the median of the coefficient of pH as a control variable was "-." A list of the top 1,000 expressions is described in [206. Linear Model that Predicts Arginine Production Amount in Interval 6].

In a linear model that predicts an arginine production amount at the end of Interval 7 (the amount of arginine produced in the culture time of Interval 7) based on data at the beginning of Interval 7 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "+," and the arithmetic sign of the median of the coefficient of pH as a control variable was "+." A list of the top 1,000 expressions is described in [207. Linear Model that Predicts Arginine Production Amount in Interval 7].

In a linear model that predicts an arginine production amount at the end of Interval 8 (the amount of arginine produced in the culture time of Interval 8) based on data at the beginning of Interval 8 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "-," and the arithmetic sign of the median of the coefficient of pH as a control variable was "-." A list of the top 1,000 expressions is described in [208. Linear Model that Predicts Arginine Production Amount in Interval 8].

In a linear model that predicts an arginine production amount at the end of Interval 9 (the amount of arginine produced in the culture time of Interval 9) based on data at the beginning of Interval 9 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "-," whereas the arithmetic sign of the median of the coefficient of pH as a control variable was "+." A list of the top 1,000 expressions is described in [209. Linear Model that Predicts Arginine Production Amount in Interval 9].

In a linear model that predicts an arginine production amount at the end of Interval 10 (the amount of arginine produced in the culture time of Interval 10) based on data at the beginning of Interval 10 (top 1,000 expressions with a high adjusted coefficient of determination with leave-one-out cross-validation performed and a high coefficient of correlation with leave-one-out cross-validation performed), the arithmetic sign of the median of the coefficient of temperature as a control variable was "-," and the arithmetic sign of the median of the coefficient of pH as a control variable was "-." A list of the top 1,000 expressions is described in [210. Linear Model that Predicts Arginine Production Amount in Interval 10].

### 4. Operating Condition Range

The control variables changed during fermentation operation were pH and temperature. FIG. 18 depicts an operating condition range. For the creation of the linear model, data acquired within a range denoted as "DATABASE" in FIG. 18 was used. In Example 3, the operating condition was changed within a range denoted as "OPERATING CONDITION RANGE" in FIG. 18 (the same range as the range denoted as "DATABASE" in FIG. 18). In FIG. 18, the values of the operating condition are represented as relative values to the value of the operating condition of J1 target depicted in FIG. 16.

### 5. Arginine Concentration Analysis

During culture control, an arginine concentration measured by the biosensor was used. Then, in the stage of determining a final result of the fermentation result, recalculation was performed using an arginine concentration measured by HPLC. For the creation of the linear model, the arginine concentration measured by HPLC was used.

### 6. Results and Discussion

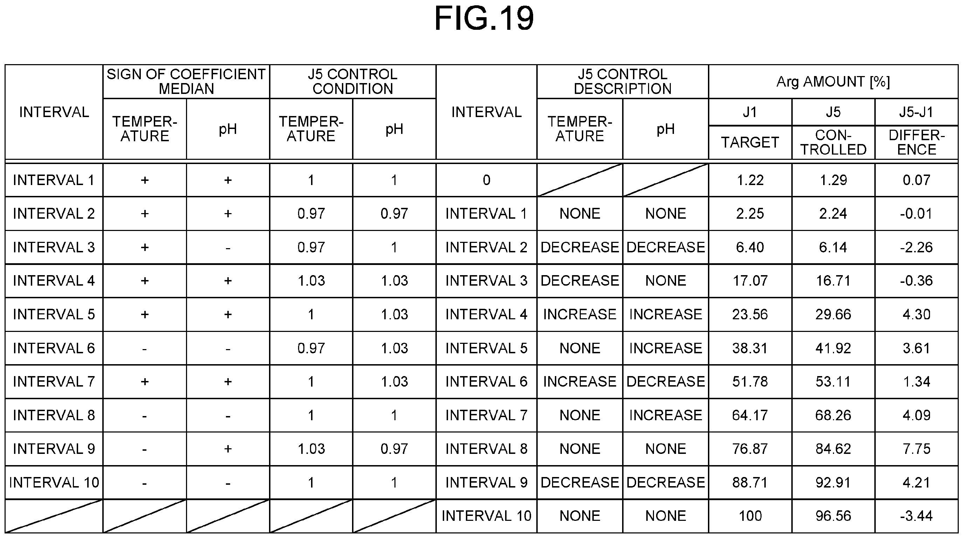

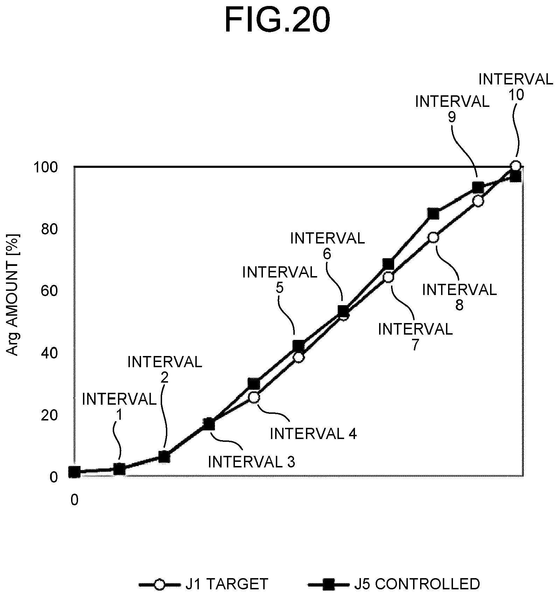

FIG. 19 and FIG. 20 depict culture results by the respective experimental conditions.

FIG. 19 depicts the sign of the median of the coefficients of the control variables of the linear model described in FIG. 17, J5 control condition, which performed control, described in FIG. 16, J5 control description, arginine amounts of J1, which did not perform control, and arginine amounts of J5, which performed control.

An arithmetic sign "+" described in the column of the sign of the coefficient median in FIG. 19 means that the arginine production amount in the next interval can be increased by making temperature higher or pH higher than that of the standard condition in FIG. 18. An arithmetic sign "-" described means that the arginine production amount in the next interval can be increased by making temperature lower or pH lower than that of the standard condition in FIG. 18.

By comparing J5 control condition in FIG. 19 and J1 standard condition in FIG. 16 with each other, the control performed by J5 can be classified into three control descriptions including increasing the arginine production amount, decreasing it, and having no influence on J1. The column of J5 control description in FIG. 19 describes "INCREASE" in the case of a control description of increasing the arginine production amount compared with J1, "DECREASE" in the case of a control description of decreasing the arginine production amount compared with J1, and "NONE" in the case of a control description making the arginine production amount comparable to J1 (the control description of Interval 1 has an influence on the fermentation result of Interval 1, and FIG. 19 is represented with the part up to the fifth column from the left and the part from the sixth column on from the left staggered by one row).

To confirm the control result, the results of J1 and J5 were compared with each other. In the column of difference of J5-J1 in FIG. 19, when the control description is "DECREASE," a negative value is given, whereas when the control description is "INCREASE," a positive value is given, and it is considered that control as estimated can be achieved using the linear model.

### INDUSTRIAL APPLICABILITY

The present invention may be practiced in particular in production of an organic compound by a fermentation method.

### EXPLANATIONS OF LETTERS OR NUMERALS

- 1: Culture system
- 11: Culture tank
- 12: Sensors
- 13: DCS
- 14: Control device
- 14a: Control unit
- 14a1: Acquiring unit
- 14a2: Calculating unit
- 14a3: Determining unit
- 14a4: Changing unit
- 14b: Communication interface
- 14c: Storage unit
- 14d: Input/output interface
- 14e: Input device
- 14f: Output device

In the present specification, various kinds of explanatory variables are mainly denoted by abbreviations; their formal names are as follows.

### (Abbreviation of explanatory variable) (Formal name)

tmp: Culture medium temperature
tmp_cumulo: Culture medium temperature accumulated value
pH: Culture medium pH
pH_cumulo: Culture medium pH accumulated value
PL: Dissolved oxygen concentration in culture medium
PL_cumulo: Accumulated value of dissolved oxygen concentration in culture medium
AN: Ammonia nitrogen concentration in culture medium
AN_cumulo: Accumulated value of ammonia nitrogen concentration in culture medium
interval_yield: Interval arginine yield
interval_Pdt.: Interval arginine productivity
OD: Culture medium turbidity
RS: Sugar concentration in culture medium
Feed_Vol: Sugar-ammonium sulfate mixed liquid feed amount
mu_cumulo: Specific multiplication rate accumulated value
nu_cumulo: Specific sugar consumption rate accumulated value
rho_cumulo: Specific production rate accumulated value
nu_per_rho_cumulo: Specific multiplication rate/specific production rate accumulated value
RS_cumulo: Accumulated value of sugar concentration in culture medium
Sugar_consuming_rate_cumulo: Sugar consumption rate accumulated value
Arg_conc._cumulo: Accumulated value of arginine concentration in culture medium
cell_yield_cumulo: Cell yield accumulated value
Cell_conc._cumulo: Cell concentration accumulated value
Cell_conc._interval: Cell concentration difference between intervals
rab_integral: Average oxygen demand
RQ_integral: Average respiratory quotient
Accum._Yield: Accumulated yield
Arg_conc.: Arginine concentration in culture medium
AS_Vol.: Ammonium sulfate feed amount (added amount)
emission_O2: Oxygen concentration in emission gas
emission_CO2: Carbon dioxide concentration in emission gas
consum_O2: Oxygen consumption amount
interval_O2: Accumulated oxygen consumption amount
generate_CO2: Carbon dioxide generation amount
interval_CO2: Accumulated carbon dioxide generation amount
RQ: Respiratory quotient
rab: Oxygen demand
Main.P-Source.Conc.: Phosphorous source concentration in culture medium at the start of culture
P-Source.Feed.conc.: Phosphorous source concentration in culture fed-batch liquid
Main.Thr.Conc.: Threonine concentration in culture medium at the start of culture
Nitrogen.Conc.: Nitrogen source concentration in culture medium at the start of culture
Lys_conc.: Lysine concentration in culture medium
feed.Sugar: Fed-batch sugar liquid amount
X.F.Sugar: Fed-batch sugar liquid amount per interval
Cell: Cell amount in culture medium
X.VXdt: Integrated cell amount
total.Vol: Total culture medium amount
total.cell: Total cell amount
total.sugar: Total sugar consumption amount
yield: Product yield
Thr: Threonine concentration in culture medium
His: Histidine concentration in culture medium
lactate: Lactic acid concentration in culture medium
SO4.ferm.: Sulfuric acid ion concentration in culture medium
PO4.ferm.: Phosphoric acid ion concentration in culture medium
PO4.apporte: Phosphoric acid ion added amount during culture
PO4.utilise: Phosphoric acid ion consumption amount during culture

### [11. Linear Model that Predicts Lysine Production Amount in Interval 1]

0.647, 0.828, pH+tmp+Main.P-Source.Conc.+Nitrogen.Conc.+Thr +lactate+PO4.apporte; 0.652, 0.827, tmp+Main.P-Source.Conc. +Nitrogen.Conc.+Thr+lactate+PO4.apporte; 0.646, 0.827, tmp+ Main.P-Source.Conc.+P-Source.Feed.conc.+Nitrogen.Conc.+Thr+ lactate+PO4.apporte; 0.636, 0.825, pH+tmp+Main.P-Source.Con c.+Nitrogen.Conc.+total.Vol+Thr+lactate+PO4.apporte; 0.636, 0.825, pH+tmp+Main.P-Source.Conc.+Nitrogen.Conc.+emission_ O2+Thr+lactate+PO4.apporte; 0.636, 0.825, pH+tmp+Main.P-Sou rce.Conc.+P-Source.Feed.conc.+Nitrogen.Conc.+Thr+lactate+PO 4.apporte; 0.635, 0.825, pH+tmp+Main.P-Source.Conc.+Nitroge n.Conc.+PL+Thr+lactate+PO4.apporte; 0.634, 0.824, tmp+Main. P-Source.Conc.+P-Source.Feed.conc.+Nitrogen.Conc.+emission_ O2+Thr+lactate+PO4.apporte; 0.641, 0.824, tmp+Main.P-Source. Conc.+Nitrogen.Conc.+emission_O2+Thr+lactate+PO4.apporte; 0. 634, 0.824, pH+tmp+Main.P-Source.Conc.+Nitrogen.Conc.+emiss ion_CO2+Thr+lactate+PO4.apporte; 0.640, 0.824, tmp+Main.P-S ource.Conc.+Nitrogen.Conc.+PL+Thr+lactate+PO4.apporte; 0.63 8, 0.823, tmp+Main.P-Source.Conc.+Main.Thr.Conc.+Nitrogen.C onc.+Thr+lactate+PO4.apporte; 0.625, 0.823, pH+tmp+Main.P-S ource.Conc.+Nitrogen.Conc.+emission_O2+emission_CO2+Thr+lac tate+PO4.apporte; 0.638, 0.823, tmp+Main.P-Source.Conc.+Nit rogen.Conc.+Thr+His+lactate+PO4.apporte; 0.625, 0.823, pH+t mp+Main.P-Source.Conc.+Nitrogen.Conc.+total.Vol+emission_O2 +Thr+lactate+PO4.apporte; 0.631, 0.823, tmp+Main.P-Source.C onc.+P-Source.Feed.conc.+Nitrogen.Conc.+PL+Thr+lactate+PO4. apporte; 0.638, 0.823, tmp+Main.P-Source.Conc.+Nitrogen.Con c.+total.Vol+Thr+lactate+PO4.apporte; 0.631, 0.823, pH+tmp+ Main.P-Source.Conc.+Nitrogen.Conc.+Thr+His+lactate+PO4.appo rte; 0.631, 0.823, pH+tmp+Main.P-Source.Conc.+Main.Thr.Conc. +Nitrogen.Conc.+Thr+lactate+PO4.apporte; 0.631, 0.822, tmp+ Main.P-Source.Conc.+P-Source.Feed.conc.+Nitrogen.Conc.+emis sion_CO2+Thr+lactate+PO4.apporte; 0.624, 0.822, pH+tmp+Main. P-Source.Conc.+P-Source.Feed.conc.+Nitrogen.Conc.+emission_ O2+Thr+lactate+PO4.apporte; 0.624, 0.822, pH+tmp+Main.P-Sou rce.Conc.+Nitrogen.Conc.+PL+emission_O2+Thr+lactate+PO4.app orte; 0.637, 0.822, tmp+Main.P-Source.Conc.+Nitrogen.Conc.+ total.sugar+Thr+lactate+PO4.apporte; 0.624, 0.822, pH+tmp+M ain.P-Source.Conc.+Nitrogen.Conc.+total.Vol+PL+Thr+lactate+ PO4.apporte; 0.630, 0.822, pH+tmp+Main.P-Source.Conc.+Nitro gen.Conc.+total.sugar+Thr+lactate+PO4.apporte; 0.637, 0.822, tmp+Main.P-Source.Conc.+Nitrogen.Conc.+emission_CO2+Thr+la ctate+PO4.apporte; 0.624, 0.822, pH+tmp+Main.P-Source.Conc. +Nitrogen.Conc.+total.Vol+emission_CO2+Thr+lactate+PO4.appo rte; 0.623, 0.822, tmp+Main.P-Source.Conc.+P-Source.Feed.co nc.+Nitrogen.Conc.+emission_O2+emission_CO2+Thr+lactate+PO4. apporte; 0.630, 0.822, tmp+Main.P-Source.Conc.+P-Source.Fee d.conc.+Main.Thr.Conc.+Nitrogen.Conc.+Thr+lactate+PO4.appor te; 0.629, 0.822, tmp+Main.P-Source.Conc.+P-Source.Feed.con c.+Nitrogen.Conc.+Thr+His+lactate+PO4.apporte; 0.616, 0.822, pH+tmp+Main.P-Source.Conc.+Nitrogen.Conc.+total.Vol+emissi on_O2+emission_CO2+Thr+lactate+PO4.apporte; 0.629, 0.821, t mp+Main.P-Source.Conc.+P-Source.Feed.conc.+Nitrogen.Conc.+t otal.Vol+Thr+lactate+PO4.apporte; 0.622, 0.821, pH+tmp+Main. P-Source.Conc.+P-Source.Feed.conc.+Nitrogen.Conc.+emission_ CO2+Thr+lactate+PO4.apporte; 0.622, 0.821, pH+tmp+Main.P-So urce.Conc.+P-Source.Feed.conc.+Nitrogen.Conc.+total.Vol+Thr +lactate+PO4.apporte; 0.622, 0.821, pH+tmp+Main.P-Source.Co nc.+Nitrogen.Conc.+PL+emission_CO2+Thr+lactate+PO4.apporte; 0.622, 0.821, pH+tmp+Main.P-Source.Conc.+P-Source.Feed.con c.+Nitrogen.Conc.+PL+Thr+lactate+PO4.apporte; 0.628, 0.821, tmp+Main.P-Source.Conc.+Nitrogen.Conc.+emission_O2+emissio n_CO2+Thr+lactate+PO4.apporte; 0.614, 0.821, pH+tmp+Main.P-Source.Conc.+P-Source.Feed.conc.+Nitrogen.Conc.+emission_O2 +emission_CO2+Thr+lactate+PO4.apporte; 0.628, 0.821, tmp+Ma in.P-Source.Conc.+Nitrogen.Conc.+PL+emission_O2+Thr+lactate +PO4.apporte; 0.621, 0.821, tmp+Main.P-Source.Conc.+P-Sourc e.Feed.conc.+Nitrogen.Conc.+emission_CO2+rab+Thr+lactate+PO 4.apporte; 0.621, 0.820, pH+tmp+Main.P-Source.Conc.+Main.Th r.Conc.+Nitrogen.Conc.+total.Vol+Thr+lactate+PO4.apporte; 0. 627, 0.820, tmp+Main.P-Source.Conc.+P-Source.Feed.conc.+Nit rogen.Conc.+total.sugar+Thr+lactate+PO4.apporte; 0.613, 0.8 20, pH+tmp+Main.P-Source.Conc.+Nitrogen.Conc.+PL+emission_O 2+emission-CO2+Thr+lactate+PO4.apporte; 0.627, 0.820, tmp+M ain.P-Source.Conc.+Nitrogen.Conc.+emission_CO2+rab+Thr+lact ate+PO4.apporte; 0.620, 0.820, pH+tmp+Main.P-Source.Conc.+N itrogen.Conc.+emission_O2+Thr+His+lactate+PO4.apporte; 0.62 0, 0.820, pH+tmp+Main.P-Source.Conc.+Nitrogen.Conc.+total.V ol+Thr+His+lactate+PO4.apporte; 0.620, 0.820, pH+tmp+Main.P -Source.Conc.+Main.Thr.Conc.+Nitrogen.Conc.+emission_O2+Thr +lactate+PO4.apporte; 0.626, 0.820, tmp+Main.P-Source.Conc. +Main.Thr.Conc.+Nitrogen.Conc.+emission_O2+Thr+lactate+PO4. apporte; 0.619, 0.820, tmp+Main.P-Source.Conc.+P-Source.Fee d.conc.+Nitrogen.Conc.+PL+emission_O2+Thr+lactate+PO4.appor te; 0.619, 0.820, pH+tmp+Main.P-Source.Conc.+P-Source.Feed. conc.+Main.Thr.Conc.+Nitrogen.Conc.+Thr+lactate+PO4.apport e; 0.626, 0.820, tmp+Main.P-Source.Conc.+Nitrogen.Conc.+emi ssion_O2+Thr+His+lactate+PO4.apporte; 0.612, 0.819, pH+tmp+ Main.P-Source.Conc.+Nitrogen.Conc.+total.Vol+emission_CO2+r ab+Thr+lactate+PO4.apporte; 0.625, 0.819, tmp+Main.P-Source. Conc.+Nitrogen.Conc.+total.Vol+emission_O2+Thr+lactate+PO4. apporte; 0.618, 0.819, pH+tmp+Main.P-Source.Conc.+Main.Thr. Conc.+Nitrogen.Conc.+PL+Thr+lactate+PO4.apporte; 0.625, 0.8 19, tmp+Main.P-Source.Conc.+Main.Thr.Conc.+Nitrogen.Conc.+P L+Thr+lactate+PO4.apporte; 0.611, 0.819, pH+tmp+Main.P-Sour ce.Conc.+Nitrogen.Conc.+total.Vol+PL+emission_O2+Thr+lactat e+PO4.apporte; 0.618, 0.819, pH+tmp+Main.P-Source.Conc.+P-S ource.Feed.conc.+Nitrogen.Conc.+Thr+His+lactate+PO4.apport e; 0.618, 0.819, pH+tmp+Main.P-Source.Conc.+Nitrogen.Conc.+ total.sugar+emission_O2+Thr+lactate+PO4.apporte; 0.618, 0.8 19, pH+tmp+Main.P-Source.Conc.+Nitrogen.Conc.+PL+Thr+His+la ctate+PO4.apporte; 0.618, 0.819, pH+tmp+Main.P-Source.Conc. +Nitrogen.Conc.+total.sugar+PL+Thr+lactate+PO4.apporte; 0.6 18, 0.819, pH+tmp+Main.P-Source.Conc.+Main.Thr.Conc.+Nitrog en.Conc.+total.sugar+Thr+lactate+PO4.apporte; 0.618, 0.819, pH+tmp+Main.P-Source.Conc.+Main.Thr.Conc.+Nitrogen.Conc.+e mission_CO2+Thr+lactate+PO4.apporte; 0.618, 0.819, tmp+Main. P-Source.Conc.+P-Source.Feed.conc.+Nitrogen.Conc.+emission_ O2+Thr+His+lactate+PO4.apporte; 0.618, 0.819, tmp+Main.P-So urce.Conc.+P-Source.Feed.conc.+Main.Thr.Conc.+Nitrogen.Conc. +emission_O2+Thr+lactate+PO4.apporte; 0.617, 0.819, pH+tmp+ Main.P-Source.Conc.+Nitrogen.Conc.+PL+rab+Thr+lactate+PO4.a pporte; 0.624, 0.819, pH+tmp+Main.P-Source.Conc.+Nitrogen.C onc.+rab+Thr+lactate+PO4.apporte; 0.617, 0.819, pH+tmp+Main. P-Source.Conc.+Nitrogen.Conc.+emission_CO2+Thr+His+lactate+ PO4.apporte; 0.610, 0.819, pH+tmp+Main.P-Source.Conc.+Nitro gen.Conc.+total.Vol+PL+emission_CO2+Thr+lactate+PO4.apport e; 0.624, 0.819, tmp+Main.P-Source.Conc.+Nitrogen.Conc.+PL+ emission_CO2+Thr+lactate+PO4.apporte; 0.624, 0.819, tmp+Mai n.P-Source.Conc.+Nitrogen.Conc.+PL+Thr+His+lactate+PO4.appo rte; 0.624, 0.819, tmp+Main.P-Source.Conc.+Nitrogen.Conc.+t otal.sugar+emission_O2+Thr+lactate+PO4.apporte; 0.624, 0.81 9, tmp+Main.P-Source.Conc.+Nitrogen.Conc.+total.Vol+PL+Thr+ lactate+PO4.apporte; 0.624, 0.819, tmp+Main.P-Source.Conc.+ Nitrogen.Conc.+total.sugar+PL+Thr+lactate+PO4.apporte; 0.62 4, 0.818, tmp+Main.P-Source.Conc.+Main.Thr.Conc.+Nitrogen.C onc.+total.sugar+Thr+lactate+PO4.apporte; 0.624, 0.818, tmp +Main.P-Source.Conc.+Main.Thr.Conc.+Nitrogen.Conc.+total.Vo l+Thr+lactate+PO4.apporte; 0.617, 0.818, tmp+Main.P-Source. Conc.+P-Source.Feed.conc.+Nitrogen.Conc.+total.Vol+emission _O2+Thr+lactate+PO4.apporte; 0.617, 0.818, pH+tmp+Main.P-So urce.Conc.+P-Source.Feed.conc.+Nitrogen.Conc.+total.sugar+T hr+lactate+PO4.apporte; 0.610, 0.818, pH+tmp+Main.P-Source. Conc.+P-Source.Feed.conc.+Nitrogen.Conc.+PL+emission_O2+Thr +lactate+PO4.apporte; 0.609, 0.818, pH+tmp+Main.P-Source.Co nc.+P-Source.Feed.conc.+Nitrogen.Conc.+total.Vol+emission_O 2+Thr+lactate+PO4.apporte; 0.616, 0.818, tmp+Main.P-Source. Conc.+Nitrogen.Conc.+PL+emission_CO2+rab+Thr+lactate+PO4.ap porte; 0.623, 0.818, tmp+Main.P-Source.Conc.+Nitrogen.Conc. +total.sugar+Thr+His+lactate+PO4.apporte; 0.623, 0.818, tmp +Main.P-Source.Conc.+Main.Thr.Conc.+Nitrogen.Conc.+Thr+His+ lactate+PO4.apporte; 0.616, 0.818, pH+tmp+Main.P-Source.Con c.+Nitrogen.Conc.+emission_CO2+rab+Thr+lactate+PO4.apporte; 0.616, 0.818, tmp+Main.P-Source.Conc.+P-Source.Feed.conc.+ Nitrogen.Conc.+PL+emission_CO2+Thr+lactate+PO4.apporte; 0.6 16, 0.818, pH+tmp+Main.P-Source.Conc.+Nitrogen.Conc.+total. Vol+total.sugar+Thr+lactate+PO4.apporte; 0.609, 0.818, pH+t mp+Main.P-Source.Conc.+P-Source.Feed.conc.+Nitrogen.Conc.+t otal.Vol+emission_CO2+Thr+lactate+PO4.apporte; 0.616, 0.818, tmp+Main.P-Source.Conc.+Nitrogen.Conc.+PL+emission_O2+emis sion_CO2+Thr+lactate+PO4.apporte; 0.622, 0.818, tmp+Main.P-Source.Conc.+Nitrogen.Conc.+total.Vol+Thr+His+lactate+PO4.a pporte; 0.609, 0.818, pH+tmp+Main.P-Source.Conc.+Main.Thr.C onc.+Nitrogen.Conc.+emission_O2+emission_CO2+Thr+lactate+PO 4.apporte; 0.609, 0.818, pH+tmp+Main.P-Source.Conc.+Main.Th r.Conc.+Nitrogen.Conc.+total.Vol+emission_O2+Thr+lactate+PO 4.apporte; 0.615, 0.818, pH+tmp+Main.P-Source.Conc.+Nitroge n.Conc.+total.sugar+Thr+His+lactate+PO4.apporte; 0.608, 0.8 18, pH+tmp+Main.P-Source.Conc.+Nitrogen.Conc.+emission_O2+e mission_CO2+Thr+His+lactate+PO4.apporte; 0.615, 0.818, pH+t mp+Main.P-Source.Conc.+Main.Thr.Conc.+Nitrogen.Conc.+Thr+Hi s+lactate+PO4.apporte; 0.608, 0.818, pH+tmp+Main.P-Source.C onc.+Nitrogen.Conc.+total.Vol+emission_O2+Thr+His+lactate+P O4.apporte; 0.622, 0.817, tmp+Main.P-Source.Conc.+Main.Thr. Conc.+Nitrogen.Conc.+emission_CO2+Thr+lactate+PO4.apporte; 0.601, 0.817, pH+tmp+Main.P-Source.Conc.+Nitrogen.Conc.+tot al.Vol+PL+emission_O2+emission_CO2+Thr+lactate+PO4.apporte; 0.601, 0.817, pH+tmp+Main.P-Source.Conc.+P-Source.Feed.con c.+Nitrogen.Conc.+total.Vol+emission_O2+emission_CO2+Thr+la ctate+PO4.apporte; 0.608, 0.817, pH+tmp+Main.P-Source.Conc. +P-Source.Feed.conc.+Nitrogen.Conc.+total.Vol+PL+Thr+lact at e+PO4.apporte; 0.608, 0.817, pH+tmp+Main.P-Source.Conc.+P-S ource.Feed.conc.+Nitrogen.Conc.+PL+emission_CO2+Thr+lactate +PO4.apporte; 0.608, 0.817, tmp+Main.P-Source.Conc.+P-Sourc e.Feed.conc.+Nitrogen.Conc.+PL+emission_O2+emission_CO2+Thr +lactate+PO4.apporte; 0.628, 0.817, tmp+Main.P-Source.Conc. +Nitrogen.Conc.+rab+Thr+lactate+PO4.apporte; 0.615, 0.817, tmp+Main.P-Source.Conc.+P-Source.Feed.conc.+Nitrogen.Conc.+ total.sugar+emission_O2+Thr+lactate+PO4.apporte; 0.621, 0.8 17, tmp+Main.P-Source.Conc.+Nitrogen.Conc.+total.Vol+emissi on_CO2+Thr+lactate+PO4.apporte; 0.607, 0.817, pH+tmp+Main.P -Source.Cone.+Main.Thr.Cone.+Nitrogen.Cone.+total.Vol+emiss ion_CO2+Thr+lactate+PO4.apporte; 0.621, 0.817, tmp+Main.P-S ource.Conc.+Nitrogen.Conc.+PL+rab+Thr+lactate+PO4.apporte; 0.607, 0.817, pH+tmp+Main.P-Source.Conc.+Nitrogen.Conc.+PL+ emission_CO2+rab+Thr+lactate+PO4.apporte; 0.621, 0.817, tmp +Main.P-Source.Conc.+Nitrogen.Conc.+emission CO2+Thr+His+la ctate+PO4.apporte; 0.607, 0.817, pH+tmp+Main.P-Source.Conc. +Main.Thr.Conc.+Nitrogen.Conc.+total.Vol+PL+Thr+lactate+PO4. apporte; 0.614, 0.817, tmp+Main.P-Source.Conc.+P-Source.Fee d.conc.+Main.Thr.Conc.+Nitrogen.Conc.+emission CO2+Thr+lact ate+PO4.apporte; 0.607, 0.817, pH+tmp+Main.P-Source.Conc.+P -Source.Feed.conc.+Main.Thr.Conc.+Nitrogen.Conc.+emission_O 2+Thr+lactate+PO4.apporte; 0.614, 0.817, tmp+Main.P-Source. Conc.+P-Source.Feed.conc.+Nitrogen.Conc.+emission_CO2+Thr+H is+lactate+PO4.apporte; 0.614, 0.817, tmp+Main.P-Source.Con c.+P-Source.Feed.conc.+Main.Thr.Conc.+Nitrogen.Conc.+PL+Thr +lactate+PO4.apporte; 0.614, 0.817, tmp+Main.P-Source.Conc. +P-Source.Feed.conc.+Nitrogen.Conc.+total.Vol+emission_CO2+ Thr+lactate+PO4.apporte; 0.607, 0.817, tmp+Main.P-Source.Co nc.+P-Source.Feed.conc.+Nitrogen.Conc.+PL+emission_CO2+rab+ Thr+lactate+PO4.apporte; 0.607, 0.817, pH+tmp+Main.P-Source. Conc.+P-Source.Feed.conc.+Nitrogen.Conc.+emission_CO2+rab+T hr+lactate+PO4.apporte; 0.607, 0.817, pH+tmp+Main.P-Source. Conc.+Main.Thr.Conc.+Nitrogen.Conc.+PL+emission_O2+Thr+lact ate+PO4.apporte; 0.607, 0.817, pH+tmp+Main.P-Source.Conc.+N itrogen.Conc.+total.Vol+emission_CO2+Thr+His+lactate+PO4.ap porte; 0.607, 0.817, pH+tmp+Main.P-Source.Conc.+P-Source.Fe ed.conc.+Nitrogen.Conc.+emission_O2+Thr+His+lactate+PO4.app orte; 0.614, 0.817, pH+tmp+Main.P-Source.Conc.+Nitrogen.Con c.+total.sugar+emission_CO2+Thr+lactate+PO4.apporte; 0.607, 0.817, pH+tmp+Main.P-Source.Conc.+Nitrogen.Conc.+PL+emissi on_O2+Thr+His+lactate+PO4.apporte; 0.599, 0.817, pH+tmp+Mai n.P-Source.Conc.+Main.Thr.Conc.+Nitrogen.Conc.+total.Vol+em ission_O2+emission_CO2+Thr+lactate+PO4.apporte; 0.599, 0.81 7, pH+tmp+Main.P-Source.Conc.+P-Source.Feed.conc.+Nitrogen. Conc.+PL+emission_O2+emission_CO2+Thr+lactate+PO4.apporte; 0.613, 0.817, tmp+Main.P-Source.Conc.+P-Source.Feed.conc.+N itrogen.Conc.+PL+Thr+His+lactate+PO4.apporte; 0.613, 0.817, tmp+Main.P-Source.Conc.+P-Source.Feed.conc.+Nitrogen.Conc. +total.Vol+PL+Thr+lactate+PO4.apporte; 0.620, 0.817, tmp+Ma in.P-Source.Conc.+P-Source.Feed.conc.+Nitrogen.Conc.+rab+Th r+lactate+PO4.apporte; 0.613, 0.817, tmp+Main.P-Source.Conc. +Nitrogen.Conc.+total.Vol+emission_O2+emission_CO2+Thr+lact ate+PO4.apporte; 0.606, 0.817, tmp+Main.P-Source.Conc.+P-So urce.Feed.conc.+Main.Thr.Conc.+Nitrogen.Conc.+emission_O2+e mission_CO2+Thr+lactate+PO4.apporte; 0.606, 0.816, tmp+Main. P-Source.Conc.+P-Source.Feed.conc.+Nitrogen.Conc.+emission_ O2+emission_CO2+Thr+His+lactate+PO4.apporte; 0.613, 0.816, tmp+Main.P-Source.Conc.+Nitrogen.Conc.+total.Vol+emission_C O2+rab+Thr+lactate+PO4.apporte; 0.613, 0.816, tmp+Main.P-So urce.Conc.+P-Source.Feed.conc.+Main.Thr.Conc.+Nitrogen.Conc. +Thr+His+lactate+PO4.apporte; 0.606, 0.816, pH+tmp+Main.P-S ource.Conc.+Nitrogen.Conc.+total.Vol+PL+Thr+His+lactate+PO4. apporte; 0.613, 0.816, tmp+Main.P-Source.Conc.+Main.Thr.Con c.+Nitrogen.Conc.+emission_O2+emission_CO2+Thr+lactate+PO4. apporte; 0.606, 0.816, tmp+Main.P-Source.Conc.+P-Source.Fee d.conc.+Nitrogen.Conc.+total.Vol+emission_O2+emission_CO2+T hr+lactate+PO4.apporte; 0.598, 0.816, pH+tmp+Main.P-Source. Conc.+Nitrogen.Conc.+total.Vol+emission_O2+emission_CO2+Thr +His+lactate+PO4.apporte; 0.613, 0.816, tmp+Main.P-Source.C onc.+P-Source.Feed.conc.+Main.Thr.Conc.+Nitrogen.Conc.+tota l.Vol+Thr+lactate+PO4.apporte; 0.613, 0.816, pH+tmp+Main.P-Source.Conc.+P-Source.Feed.conc.+Nitrogen.Conc.+rab+Thr+lac tate+PO4.apporte; 0.619, 0.816, tmp+Main.P-Source.Conc.+Nit rogen.Conc.+total.Vol+total.sugar+Thr+lactate+PO4.apporte; 0.612, 0.816, tmp+Main.P-Source.Conc.+Main.Thr.Conc.+Nitrog en.Conc.+PL+emission_O2+Thr+lactate+PO4.apporte; 0.612, 0.8 16, tmp+Main.P-Source.Conc.+P-Source.Feed.conc.+Nitrogen.Co nc.+total.sugar+Thr+His+lactate+PO4.apporte; 0.612, 0.816, tmp+Main.P-Source.Conc.+P-Source.Feed.conc.+Main.Thr.Conc.+ Nitrogen.Conc.+total.sugar+Thr+lactate+PO4.apporte; 0.605, 0.816, pH+tmp+Main.P-Source.Conc.+P-Source.Feed.conc.+Main. Thr.Conc.+Nitrogen.Conc.+emission_CO2+Thr+lactate+PO4.appor te; 0.605, 0.816, pH+tmp+Main.P-Source.Conc.+Nitrogen.Conc. +total.sugar+PL+emission_O2+Thr+lactate+PO4.apporte; 0.598, 0.816, pH+tmp+Main.P-Source.Conc.+Nitrogen.Conc.+total.Vol +PL+emission_CO2+rab+Thr+lactate+PO4.apporte; 0.612, 0.816, tmp+Main.P-Source.Conc.+Nitrogen.Conc.+emission_O2+emissio n_CO2+Thr+His+lactate+PO4.apporte; 0.605, 0.816, pH+tmp+Mai n.P-Source.Conc.+P-Source.Feed.conc.+Main.Thr.Conc.+Nitroge n.Conc.+total.Vol+Thr+lactate+PO4.apporte; 0.605, 0.816, pH +tmp+Main.P-Source.Conc.+Main.Thr.Conc.+Nitrogen.Conc.+tota 1.sugar+emission_O2+Thr+lactate+PO4.apporte; 0.612, 0.816, tmp+Main.P-Source.Conc.+P-Source.Feed.conc.+Nitrogen.Conc.+ total.Vol+Thr+His+lactate+PO4.apporte; 0.605, 0.816, pH+tmp +Main.P-Source.Conc.+Nitrogen.Conc.+total.sugar+emission_O2 +emission_CO2+Thr+lactate+PO4.apporte; 0.604, 0.816, pH+tmp +Main.P-Source.Conc.+Main.Thr.Conc.+Nitrogen.Conc.+PL+emiss ion_CO2+Thr+lactate+PO4.apporte; 0.604, 0.816, pH+tmp+Main. P-Source.Conc.+P-Source.Feed.conc.+Nitrogen.Conc.+emission_ CO2+Thr+His+lactate+PO4.apporte; 0.611, 0.816, tmp+Main.P-S ource.Conc.+Nitrogen.Conc.+PL+emission_O2+Thr+His+lactate+P O4.apporte; 0.604, 0.816, pH+tmp+Main.P-Source.Conc.+P-Sour ce.Feed.conc.+Nitrogen.Conc.+total.sugar+emission_O2+Thr+la ctate+PO4.apporte; 0.611, 0.816, tmp+Main.P-Source.Conc.+P-Source.Feed.conc.+Nitrogen.Conc.+total.sugar+PL+Thr+lactate +PO4.apporte; 0.597, 0.816, pH+tmp+Main.P-Source.Conc.+P-So urce.Feed.conc.+Main.Thr.Conc.+Nitrogen.Conc.+emission_O2+e mission_CO2+Thr+lactate+PO4.apporte; 0.604, 0.815, pH+tmp+M ain.P-Source.Conc.+P-Source.Feed.conc.+Nitrogen.Conc.+total. Vol+Thr+His+lactate+PO4.apporte; 0.604, 0.815, pH+tmp+Main. P-Source.Conc.+Main.Thr.Conc.+Nitrogen.Conc.+total.sugar+PL +Thr+lactate+PO4.apporte; 0.611, 0.815, tmp+Main.P-Source.C onc.+Main.Thr.Conc.+Nitrogen.Conc.+emission_CO2+rab+Thr+lac tate+PO4.apporte; 0.604, 0.815, pH+tmp+Main.P-Source.Conc.+ Nitrogen.Conc.+PL+emission_CO2+Thr+His+lactate+PO4.apporte; 0.596, 0.815, pH+tmp+Main.P-Source.Conc.+P-Source.Feed.con c.+Nitrogen.Conc.+total.Vol+emission_CO2+rab+Thr+lactate+PO 4.apporte; 0.604, 0.815, pH+tmp+Main.P-Source.Conc.+Main.Th r.Conc.+Nitrogen.Conc.+total.Vol+Thr+His+lactate+PO4.apport e; 0.611, 0.815, tmp+Main.P-Source.Conc.+Nitrogen.Conc.+tot al.Vol+PL+emission_O2+Thr+lactate+PO4.apporte; 0.604, 0.815, tmp+Main.P-Source.Conc.+P-Source.Feed.conc.+Nitrogen.Conc. +total.Vol+emission_CO2+rab+Thr+lactate+PO4.apporte; 0.604, 0.815, pH+tmp+Main.P-Source.Conc.+P-Source.Feed.conc.+Main. Thr.Conc.+Nitrogen.Conc.+PL+Thr+lactate+PO4.apporte; 0.604, 0.815, pH+tmp+Main.P-Source.Conc.+Main.Thr.Conc.+Nitrogen. Conc.+emission_O2+Thr+His+lactate+PO4.apporte; 0.611, 0.815, tmp+Main.P-Source.Conc.+Main.Thr.Conc.+Nitrogen.Conc.+tota l.Vol+emission_O2+Thr+lactate+PO4.apporte; 0.596, 0.815, pH +tmp+Main.P-Source.Conc.+P-Source.Feed.conc.+Nitrogen.Conc. +emission_O2+emission_CO2+Thr+His+lactate+PO4.apporte; 0.60 3, 0.815, tmp+Main.P-Source.Conc.+P-Source.Feed.conc.+Main. Thr.Conc.+Nitrogen.Conc.+emission_CO2+rab+Thr+lactate+PO4.a pporte; 0.603, 0.815, pH+tmp+Main.P-Source.Conc.+Nitrogen.C onc.+total.Vol+total.sugar+emission_O2+Thr+lactate+PO4.appo rte; 0.617, 0.815, tmp+Main.P-Source.Conc.+Nitrogen.Conc.+t otal.sugar+emission_CO2+Thr+lactate+PO4.apporte; 0.610, 0.8 15, tmp+Main.P-Source.Conc.+P-Source.Feed.conc.+Nitrogen.Co nc.+PL+rab+Thr+lactate+PO4.apporte; 0.610, 0.815, tmp+Main. P-Source.Conc.+Nitrogen.Conc.+total.sugar+PL+emission_O2+Th r+lactate+PO4.apporte; 0.603, 0.815, pH+tmp+Main.P-Source.C onc.+Nitrogen.Conc.+total.sugar+emission_O2+Thr+His+lactate +PO4.apporte; 0.596, 0.815, pH+tmp+Main.P-Source.Conc.+Main. Thr.Conc.+Nitrogen.Conc.+PL+emission_O2+emission_CO2+Thr+la ctate+PO4.apporte; 0.610, 0.815, tmp+Main.P-Source.Conc.+Ma in.Thr.Conc.+Nitrogen.Conc.+emission_O2+Thr+His+lactate+PO4. apporte; 0.603, 0.815, pH+tmp+Main.P-Source.Conc.+P-Source. Feed.conc.+Main.Thr.Conc.+Nitrogen.Conc.+total.sugar+Thr+la ctate+PO4.apporte; 0.610, 0.815, tmp+Main.P-Source.Conc.+Ma in.Thr.Conc.+Nitrogen.Conc.+total.sugar+emission_O2+Thr+lac tate+PO4.apporte; 0.603, 0.815, pH+tmp+Main.P-Source.Conc.+ P-Source.Feed.conc.+Nitrogen.Conc.+PL+rab+Thr+lactate+PO4.a pporte; 0.603, 0.815, pH+tmp+Main.P-Source.Conc.+P-Source.F eed.conc.+Nitrogen.Conc.+PL+Thr+His+lactate+PO4.apporte; 0. 595, 0.815, pH+tmp+Main.P-Source.Conc.+Nitrogen.Conc.+PL+em ission_O2+emission_CO2+Thr+His+lactate+PO4.apporte; 0.610, 0.815, tmp+Main.P-Source.Conc.+Nitrogen.Conc.+total.Vol+emi ssion_O2+Thr+His+lactate+PO4.apporte; 0.609, 0.815, tmp+Mai n.P-Source.Conc.+Main.Thr.Conc.+Nitrogen.Conc.+total.sugar+ PL+Thr+lactate+PO4.apporte; 0.602, 0.814, pH+tmp+Main.P-Sou rce.Conc.+Nitrogen.Conc.+total.Vol+total.sugar+PL+Thr+lacta te+PO4.apporte; 0.609, 0.814, tmp+Main.P-Source.Conc.+Nitro gen.Conc.+total.sugar+emission_O2+Thr+His+lactate+PO4.appor te; 0.609, 0.814, tmp+Main.P-Source.Conc.+P-Source.Feed.con c.+Nitrogen.Conc.+total.sugar+emission_CO2+Thr+lactate+PO4. apporte; 0.609, 0.814, tmp+Main.P-Source.Conc.+Main.Thr.Con c.+Nitrogen.Conc.+total.Vol+PL+Thr+lactate+PO4.apporte; 0.6 02, 0.814, pH+tmp+Main.P-Source.Conc.+Main.Thr.Conc.+Nitrog en.Conc.+total.Vol+total.sugar+Thr+lactate+PO4.apporte; 0.6 02, 0.814, tmp+Main.P-Source.Conc.+P-Source.Feed.conc.+Nitr ogen.Conc.+emission_CO2+rab+Thr+His+lactate+PO4.apporte; 0. 595, 0.814, pH+tmp+Main.P-Source.Conc.+Main.Thr.Conc.+Nitro gen.Conc.+total.Vol+emission_CO2+rab+Thr+lactate+PO4.apport e; 0.602, 0.814, pH+tmp+Main.P-Source.Conc.+Main.Thr.Conc.+ Nitrogen.Conc.+total.sugar+Thr+His+lactate+PO4.apporte; 0.6 01, 0.814, pH+tmp+Main.P-Source.Conc.+P-Source.Feed.conc.+M ain.Thr.Conc.+Nitrogen.Conc.+Thr+His+lactate+PO4.apporte; 0. 594, 0.814, pH+tmp+Main.P-Source.Conc.+P-Source.Feed.conc.+ Nitrogen.Conc.+total.Vol+PL+emission_O2+Thr+lactate+PO4.app orte; 0.594, 0.814, pH+tmp+Main.P-Source.Conc.+Main.Thr.Con c.+Nitrogen.Conc.+total.Vol+PL+emission_O2+Thr+lactate+PO4. apporte; 0.608, 0.814, tmp+Main.P-Source.Conc.+Nitrogen.Con c.+emission_CO2+rab+Thr+His+lactate+PO4.apporte; 0.601, 0.8 14, pH+tmp+Main.P-Source.Conc.+P-Source.Feed.conc.+Nitrogen. Conc.+total.sugar+PL+Thr+lactate+PO4.apporte; 0.594, 0.814, pH+tmp+Main.P-Source.Conc.+P-Source.Feed.conc.+Nitrogen.Co nc.+PL+emission_CO2+rab+Thr+lactate+PO4.apporte; 0.601, 0.8 14, tmp+Main.P-Source.Conc.+P-Source.Feed.conc.+Main.Thr.Co nc.+Nitrogen.Conc.+PL+emission_O2+Thr+lactate+PO4.apporte; 0.601, 0.814, tmp+Main.P-Source.Conc.+P-Source.Feed.conc.+N itrogen.Conc.+PL+emission_O2+Thr+His+lactate+PO4.apporte; 0. 608, 0.814, tmp+Main.P-Source.Conc.+Nitrogen.Conc.+total.su gar+emission_O2+emission_CO2+Thr+lactate+PO4.apporte; 0.601, 0.814, tmp+Main.P-Source.Conc.+P-Source.Feed.conc.+Nitroge n.Conc.+total.sugar+emission_O2+emission_CO2+Thr+lactate+PO 4.apporte; 0.601, 0.814, pH+tmp+Main.P-Source.Conc.+Main.Th r.Conc.+Nitrogen.Conc.+emission CO2+Thr+His+lactate+PO4.app orte; 0.594, 0.814, pH+tmp+Main.P-Source.Conc.+P-Source.Fee d.conc.+Nitrogen.Conc.+total.Vol+PL+emission_CO2+Thr+lactat e+PO4.apporte; 0.608, 0.814, tmp+Main.P-Source.Conc.+Main.T hr.Conc.+Nitrogen.Conc.+PL+emission_CO2+Thr+lactate+PO4.app orte; 0.608, 0.814, tmp+Main.P-Source.Conc.+P-Source.Feed.c onc.+Nitrogen.Conc.+total.Vol+total.sugar+Thr+lactate+PO4.a pporte; 0.601, 0.814, pH+tmp+Main.P-Source.Conc.+P-Source.F eed.conc.+Nitrogen.Conc.+total.sugar+Thr+His+lactate+PO4.ap porte; 0.608, 0.814, tmp+Main.P-Source.Conc.+Main.Thr.Conc. +Nitrogen.Conc.+total.sugar+Thr+His+lactate+PO4.apporte; 0. 601, 0.814, pH+tmp+Main.P-Source.Conc.+Nitrogen.Conc.+total. sugar+PL+Thr+His+lactate+PO4.apporte; 0.608, 0.814, pH+tmp+ Main.P-Source.Conc.+Main.Thr.Conc.+Nitrogen.Conc.+rab+Thr+l actate+PO4.apporte; 0.601, 0.814, tmp+Main.P-Source.Conc.+P -Source.Feed.conc.+Main.Thr.Conc.+Nitrogen.Conc.+emission_O 2+Thr+His+lactate+PO4.apporte; 0.593, 0.814, pH+tmp+Main.P-Source.Conc.+Main.Thr.Conc.+Nitrogen.Conc.+total.Vol+PL+emi ssion_CO2+Thr+lactate+PO4.apporte; 0.601, 0.814, pH+tmp+Mai n.P-Source.Conc.+Nitrogen.Conc.+total.sugar+PL+emission_CO2 +Thr+lactate+PO4.apporte; 0.601, 0.814, pH+tmp+Main.P-Sourc e.Conc.+Nitrogen.Conc.+total.Vol+total.sugar+emission-CO2+T hr+lactate+PO4.apporte; 0.600, 0.814, pH+tmp+Main.P-Source. Conc.+P-Source.Feed.conc.+Nitrogen.Conc.+total.sugar+emissi on_CO2+Thr+lactate+PO4.apporte; 0.593, 0.814, pH+tmp+Main.P -Source.Conc.+Nitrogen.Conc.+total.Vol+PL+emission_O2+Thr+H is+lactate+PO4.apporte; 0.608, 0.814, tmp+Main.P-Source.Con c.+Nitrogen.Conc.+total.sugar+PL+Thr+His+lactate+PO4.apport e; 0.600, 0.814, pH+tmp+Main.P-Source.Conc.+Nitrogen.Conc.+ total.Vol+total.sugar+Thr+His+lactate+PO4.apporte; 0.608, 0. 814, tmp+Main.P-Source.Conc.+Main.Thr.Conc.+Nitrogen.Conc.+ PL+Thr+His+lactate+PO4.apporte; 0.600, 0.814, pH+tmp+Main.P -Source.Conc.+P-Source.Feed.conc.+Nitrogen.Conc.+total.Vol+ total.sugar+Thr+lactate+PO4.apporte; 0.593, 0.813, pH+tmp+M ain.P-Source.Conc.+Nitrogen.Conc.+total.Vol+emission_CO2+ra b+Thr+His+lactate+PO4.apporte; 0.600, 0.813, tmp+Main.P-Sou rce.Conc.+P-Source.Feed.conc.+Nitrogen.Conc.+total.Vol+PL+e mission_O2+Thr+lactate+PO4.apporte; 0.600, 0.813, pH+tmp+Ma in.P-Source.Conc.+Main.Thr.Conc.+Nitrogen.Conc.+total.sugar +emission_CO2+Thr+lactate+PO4.apporte; 0.600, 0.813, pH+tmp +Main.P-Source.Conc.+Main.Thr.Conc.+Nitrogen.Conc.+PL+Thr+H is+lactate+PO4.apporte; 0.607, 0.813, tmp+Main.P-Source.Con c.+Main.Thr.Conc.+Nitrogen.Conc.+total.Vol+Thr+His+lactate+ PO4.apporte; 0.607, 0.813, tmp+Main.P-Source.Conc.+Nitrogen. Conc.+total.Vol+PL+emission_CO2+Thr+lactate+PO4.apporte; 0. 600, 0.813, tmp+Main.P-Source.Conc.+Main.Thr.Conc.+Nitrogen. Conc.+PL+emission_CO2+rab+Thr+lactate+PO4.apporte; 0.607, 0. 813, tmp+Main.P-Source.Conc.+Nitrogen.Conc.+PL+emission_CO2 +Thr+His+lactate+PO4.apporte; 0.607, 0.813, tmp+Main.P-Sour ce.Conc.+Nitrogen.Conc.+total.Vol+PL+Thr+His+lactate+PO4.ap porte; 0.600, 0.813, pH+tmp+Main.P-Source.Conc.+Main.Thr.Co nc.+Nitrogen.Conc.+PL+rab+Thr+lactate+PO4.apporte; 0.600, 0. 813, tmp+Main.P-Source.Conc.+P-Source.Feed.conc.+Main.Thr.C onc.+Nitrogen.Conc.+total.Vol+emission_O2+Thr+lactate+PO4.a pporte; 0.592, 0.813, pH+tmp+Main.P-Source.Conc.+Nitrogen.C onc.+total.Vol+total.sugar+emission_O2+emission_CO2+Thr+lac tate+PO4.apporte; 0.600, 0.813, tmp+Main.P-Source.Conc.+P-S ource.Feed.conc.+Nitrogen.Conc.+total.sugar+emission_O2+Thr +His+lactate+PO4.apporte; 0.592, 0.813, pH+tmp+Main.P-Sourc e.Conc.+P-Source.Feed.conc.+Main.Thr.Conc.+Nitrogen.Conc.+t otal.Vol+emission_O2+Thr+lactate+PO4.apporte; 0.592, 0.813, pH+tmp+Main.P-Source.Conc.+P-Source.Feed.conc.+Nitrogen.Co nc.+total.sugar+emission_O2+emission_CO2+Thr+lactate+PO4.ap porte; 0.599, 0.813, tmp+Main.P-Source.Conc.+Main.Thr.Conc. +Nitrogen.Conc.+PL+emission_O2+emission_CO2+Thr+lactate+PO4. apporte; 0.607, 0.813, tmp+Main.P-Source.Conc.+Main.Thr.Con c.+Nitrogen.Conc.+total.Vol+emission_CO2+Thr+lactate+PO4.ap porte; 0.599, 0.813, tmp+Main.P-Source.Conc.+P-Source.Feed. conc.+Nitrogen.Conc.+total.Vol+emission_O2+Thr+His+lactate+ PO4.apporte; 0.592, 0.813, pH+tmp+Main.P-Source.Conc.+Nitro gen.Conc.+total.sugar+PL+emission_O2+emission_CO2+Thr+lacta te+PO4.apporte; 0.592, 0.813, pH+tmp+Main.P-Source.Conc.+Ni trogen.Conc.+total.Vol+PL+emission_CO2+Thr+His+lactate+PO4. apporte; 0.599, 0.813, tmp+Main.P-Source.Conc.+P-Source.Fee d.conc.+Main.Thr.Conc.+Nitrogen.Conc.+total.sugar+emission_ O2+Thr+lactate+PO4.apporte; 0.592, 0.813, pH+tmp+Main.P-Sou rce.Conc.+P-Source.Feed.conc.+Main.Thr.Conc.+Nitrogen.Conc. +total.Vol+emission_CO2+Thr+lactate+PO4.apporte; 0.592, 0.8 13, pH+tmp+Main.P-Source.Conc.+Main.Thr.Conc.+Nitrogen.Conc. +emission_O2+emission_CO2+Thr+His+lactate+PO4.apporte; 0.59 9, 0.813, pH+tmp+Main.P-Source.Conc.+Main.Thr.Conc.+Nitroge n.Conc.+emission_CO2+rab+Thr+lactate+PO4.apporte; 0.591, 0. 813, pH+tmp+Main.P-Source.Conc.+Main.Thr.Conc.+Nitrogen.Con c.+total.Vol+emission_O2+Thr+His+lactate+PO4.apporte; 0.599, 0.813, pH+tmp+Main.P-Source.Conc.+Nitrogen.Conc.+total.Vol +PL+rab+Thr+lactate+PO4.apporte; 0.591, 0.813, pH+tmp+Main. P-Source.Conc.+P-Source.Feed.conc.+Nitrogen.Conc.+total.Vol +emission_O2+Thr+His+lactate+PO4.apporte; 0.599, 0.813, pH+ tmp+Main.P-Source.Conc.+Nitrogen.Conc.+total.sugar+emission _CO2+Thr+His+lactate+PO4.apporte; 0.606, 0.813, tmp+Main.P-Source.Conc.+Nitrogen.Conc.+total.Vol+total.sugar+emission_ O2+Thr+lactate+PO4.apporte; 0.591, 0.813, pH+tmp+Main.P-Sou rce.Conc.+P-Source.Feed.conc.+Main.Thr.Conc.+Nitrogen.Conc. +PL+emission_O2+Thr+lactate+PO4.apporte; 0.598, 0.812, tmp+ Main.P-Source.Conc.+Main.Thr.Conc.+Nitrogen.Conc.+total.Vol +emission_CO2+rab+Thr+lactate+PO4.apporte; 0.583, 0.812, pH +tmp+Main.P-Source.Conc.+Main.Thr.Conc.+Nitrogen.Conc.+tota l.Vol+PL+emission_O2+emission_CO2+Thr+lactate+PO4.apporte; 0.605, 0.812, tmp+Main.P-Source.Conc.+Main.Thr.Conc.+Nitrog en.Conc.+emission_CO2+Thr+His+lactate+PO4.apporte; 0.598, 0. 812, tmp+Main.P-Source.Conc.+Nitrogen.Conc.+PL+emission_O2+ emission_CO2+Thr+His+lactate+PO4.apporte; 0.591, 0.812, pH+ tmp+Main.P-Source.Conc.+Main.Thr.Conc.+Nitrogen.Conc.+total. sugar+PL+emission_O2+Thr+lactate+PO4.apporte; 0.605, 0.812, tmp+Main.P-Source.Conc.+Nitrogen.Conc.+total.Vol+emission_ CO2+Thr+His+lactate+PO4.apporte; 0.598, 0.812, tmp+Main.P-S ource.Conc.+Main.Thr.Conc.+Nitrogen.Conc.+total.Vol+emissio n_O2+emission_CO2+Thr+lactate+PO4.apporte; 0.583, 0.812, pH +tmp+Main.P-Source.Conc.+P-Source.Feed.conc.+Nitrogen.Conc. +total.Vol+PL+emission_O2+emission_CO2+Thr+lactate+PO4.appo rte; 0.583, 0.812, pH+tmp+Main.P-Source.Conc.+P-Source.Feed. conc.+Main.Thr.Conc.+Nitrogen.Conc.+total.Vol+emission_O2+e mission_CO2+Thr+lactate+PO4.apporte; 0.591, 0.812, pH+tmp+M ain.P-Source.Conc.+Main.Thr.Conc.+Nitrogen.Conc.+total.suga r+emission_O2+emission_CO2+Thr+lactate+PO4.apporte; 0.591, 0.812, pH+tmp+Main.P-Source.Conc.+P-Source.Feed.conc.+Nitro gen.Conc.+total.Vol+emission_CO2+Thr+His+lactate+PO4.apport e; 0.598, 0.812, tmp+Main.P-Source.Conc.+Nitrogen.Conc.+tot al.Vol+PL+emission_O2+emission_CO2+Thr+lactate+PO4.apporte; 0.598, 0.812, tmp+Main.P-Source.Conc.+P-Source.Feed.conc.+ Nitrogen.Conc.+total.sugar+PL+emission_O2+Thr+lactate+PO4.a pporte; 0.598, 0.812, tmp+Main.P-Source.Conc.+P-Source.Feed. conc.+Main.Thr.Conc.+Nitrogen.Conc.+PL+emission_CO2+Thr+lac tate+PO4.apporte; 0.605, 0.812, pH+tmp+Main.P-Source.Conc.+ Nitrogen.Conc.+total.Vol+rab+Thr+lactate+PO4.apporte; 0.590, 0.812, pH+tmp+Main.P-Source.Conc.+P-Source.Feed.conc.+Nitr ogen.Conc.+PL+emission_O2+Thr+His+lactate+PO4.apporte; 0.59 8, 0.812, tmp+Main.P-Source.Conc.+P-Source.Feed.conc.+Nitro gen.Conc.+PL+emission_CO2+Thr+His+lactate+PO4.apporte; 0.59 0, 0.812, pH+tmp+Main.P-Source.Conc.+Main.Thr.Conc.+Nitroge n.Conc.+total.Vol+emission_CO2+Thr+His+lactate+PO4.apporte; 0.605, 0.812, tmp+Main.P-Source.Conc.+Main.Thr.Conc.+Nitro gen.Conc.+total.Vol+total.sugar+Thr+lactate+PO4.apporte; 0. 598, 0.812, tmp+Main.P-Source.Conc.+P-Source.Feed.conc.+Nit rogen.Conc.+total.Vol+PL+emission_CO2+Thr+lactate+PO4.appor te; 0.590, 0.812, pH+tmp+Main.P-Source.Conc.+P-Source.Feed. conc.+Main.Thr.Conc.+Nitrogen.Conc.+total.Vol+PL+Thr+lactat e+PO4.apporte; 0.582, 0.812, pH+tmp+Main.P-Source.Conc.+P-S ource.Feed.conc.+Nitrogen.Conc.+total.Vol+emission_O2+emiss ion_CO2+Thr+His+lactate+PO4.apporte; 0.597, 0.812, tmp+Main. P-Source.Conc.+Nitrogen.Conc.+PL+emission_CO2+rab+Thr+His+l actate+PO4.apporte; 0.597, 0.812, tmp+Main.P-Source.Conc.+P -Source.Feed.conc.+Main.Thr.Conc.+Nitrogen.Conc.+emission_C O2+Thr+His+lactate+PO4.apporte; 0.590, 0.812, pH+tmp+Main.P -Source.Conc.+P-Source.Feed.conc.+Main.Thr.Conc.+Nitrogen.C onc.+total.sugar+emission_O2+Thr+lactate+PO4.apporte; 0.582, 0.812, pH+tmp+Main.P-Source.Conc.+Nitrogen.Conc.+total.Vol +PL+emission_O2+emission_CO2+Thr+His+lactate+PO4.apporte; 0. 604, 0.812, tmp+Main.P-Source.Conc.+Nitrogen.Conc.+total.Vo l+total.sugar+Thr+His+lactate+PO4.apporte; 0.597, 0.812, tm p+Main.P-Source.Conc.+Nitrogen.Conc.+total.Vol+PL+emission_ CO2+rab+Thr+lactate+PO4.apporte; 0.597, 0.812, tmp+Main.P-S ource.Conc.+Nitrogen.Conc.+total.Vol+emission_O2+emission_C O2+Thr+His+lactate+PO4.apporte; 0.597, 0.812, tmp+Main.P-So urce.Conc.+P-Source.Feed.conc.+Main.Thr.Conc.+Nitrogen.Conc. +total.Vol+emission_CO2+Thr+lactate+PO4.apporte; 0.582, 0.8 12, pH+tmp+Main.P-Source.Conc.+Main.Thr.Conc.+Nitrogen.Conc. +total.Vol+emission_O2+emission_CO2+Thr+His+lactate+PO4.app orte; 0.589, 0.812, pH+tmp+Main.P-Source.Conc.+P-Source.Fee d.conc.+Main.Thr.Conc.+Nitrogen.Conc.+emission_O2+Thr+His+l actate+PO4.apporte; 0.604, 0.812, tmp+Main.P-Source.Conc.+N itrogen.Conc.+total.Vol+total.sugar+PL+Thr+lactate+PO4.appo rte; 0.597, 0.812, tmp+Main.P-Source.Conc.+Nitrogen.Conc.+t otal.Vol+emission_CO2+rab+Thr+His+lactate+PO4.apporte; 0.58 9, 0.812, pH+tmp+Main.P-Source.Conc.+P-Source.Feed.conc.+Ma in.Thr.Conc.+Nitrogen.Conc.+PL+emission_CO2+Thr+lactate+PO4. apporte; 0.589, 0.812, pH+tmp+Main.P-Source.Conc.+P-Source. Feed.conc.+Main.Thr.Conc.+Nitrogen.Conc.+emission_CO2+rab+T hr+lactate+PO4.apporte; 0.597, 0.812, pH+tmp+Main.P-Source. Conc.+P-Source.Feed.conc.+Main.Thr.Conc.+Nitrogen.Conc.+rab +Thr+lactate+PO4.apporte; 0.597, 0.812, tmp+Main.P-Source.C onc.+P-Source.Feed.conc.+Main.Thr.Conc.+Nitrogen.Conc.+tota l.sugar+Thr+His+lactate+PO4.apporte; 0.589, 0.812, pH+tmp+M ain.P-Source.Conc.+Nitrogen.Conc.+total.sugar+emission_O2+e mission_CO2+Thr+His+lactate+PO4.apporte; 0.589, 0.812, tmp+ Main.P-Source.Conc.+P-Source.Feed.conc.+Main.Thr.Conc.+Nitr ogen.Conc.+PL+emission_O2+emission_CO2+Thr+lactate+PO4.appo rte; 0.604, 0.812, tmp+Main.P-Source.Conc.+Nitrogen.Conc.+t otal.sugar+PL+emission_CO2+Thr+lactate+PO4.apporte; 0.589, 0.812, pH+tmp+Main.P-Source.Conc.+Main.Thr.Conc.+Nitrogen.C onc.+PL+emission_CO2+rab+Thr+lactate+PO4.apporte; 0.589, 0. 811, pH+tmp+Main.P-Source.Conc.+Main.Thr.Conc.+Nitrogen.Con c.+total.Vol+total.sugar+emission_O2+Thr+lactate+PO4.apport e; 0.596, 0.811, tmp+Main.P-Source.Conc.+P-Source.Feed.conc. +Main.Thr.Conc.+Nitrogen.Conc.+total.Vol+PL+Thr+lactate+PO4. apporte; 0.596, 0.811, tmp+Main.P-Source.Conc.+P-Source.Fee d.conc.+Nitrogen.Conc.+total.Vol+emission_CO2+Thr+His+lacta te+PO4.apporte; 0.589, 0.811, tmp+Main.P-Source.Conc.+P-Sou rce.Feed.conc.+Nitrogen.Conc.+PL+emission_O2+emission_CO2+T hr+His+lactate+PO4.apporte; 0.589, 0.811, tmp+Main.P-Source. Conc.+P-Source.Feed.conc.+Main.Thr.Conc.+Nitrogen.Conc.+emi ssion_O2+emission_CO2+Thr+His+lactate+PO4.apporte; 0.589, 0. 811, pH+tmp+Main.P-Source.Conc.+Main.Thr.Conc.+Nitrogen.Con c.+total.sugar+emission_O2+Thr+His+lactate+PO4.apporte; 0.5 96, 0.811, tmp+Main.P-Source.Conc.+P-Source.Feed.conc.+Nitr ogen.Conc.+total.sugar+emission_CO2+rab+Thr+lactate+PO4.app orte; 0.589, 0.811, pH+tmp+Main.P-Source.Conc.+Nitrogen.Con c.+total.Vol+total.sugar+PL+emission_O2+Thr+lactate+PO4.app orte; 0.596, 0.811, tmp+Main.P-Source.Conc.+Main.Thr.Conc.+ Nitrogen.Conc.+emission_O2+emission_CO2+Thr+His+lactate+PO4. apporte; 0.596, 0.811, tmp+Main.P-Source.Conc.+P-Source.Fee d.conc.+Main.Thr.Conc.+Nitrogen.Conc.+PL+Thr+His+lactate+PO 4.apporte; 0.610, 0.811, tmp+Main.P-Source.Conc.+Main.Thr.C onc.+Nitrogen.Conc.+rab+Thr+lactate+PO4.apporte; 0.603, 0.8 11, tmp+Main.P-Source.Conc.+Main.Thr.Conc.+Nitrogen.Conc.+t otal.sugar+emission_CO2+Thr+lactate+PO4.apporte; 0.596, 0.8 11, tmp+Main.P-Source.Conc.+P-Source.Feed.conc.+Main.Thr.Co nc.+Nitrogen.Conc.+total.sugar+PL+Thr+lactate+PO4.apporte; 0.588, 0.811, pH+tmp+Main.P-Source.Conc.+P-Source.Feed.conc. +Nitrogen.Conc.+total.sugar+emission_O2+Thr+His+lactate+PO4. apporte; 0.580, 0.811, pH+tmp+Main.P-Source.Conc.+P-Source. Feed.conc.+Nitrogen.Conc.+total.Vol+PL+emission_CO2+rab+Thr +lactate+PO4.apporte; 0.588, 0.811, tmp+Main.P-Source.Conc. +P-Source.Feed.conc.+Main.Thr.Conc.+Nitrogen.Conc.+total.Vo l+emission_O2+emission_CO2+Thr+lactate+PO4.apporte; 0.588, 0.811, pH+tmp+Main.P-Source.Conc.+P-Source.Feed.conc.+Nitro gen.Conc.+total.sugar+PL+emission_O2+Thr+lactate+PO4.apport e; 0.588, 0.811, pH+tmp+Main.P-Source.Conc.+Main.Thr.Conc.+ Nitrogen.Conc.+total.Vol+PL+Thr+His+lactate+PO4.apporte; 0. 588, 0.811, pH+tmp+Main.P-Source.Conc.+P-Source.Feed.conc.+ Nitrogen.Conc.+PL+emission_CO2+Thr+His+lactate+PO4.apporte; 0.588, 0.811, tmp+Main.P-Source.Conc.+P-Source.Feed.conc.+ Main.Thr.Conc.+Nitrogen.Conc.+PL+emission_CO2+rab+Thr+lacta te+PO4.apporte; 0.588, 0.811, pH+tmp+Main.P-Source.Conc.+P-Source.Feed.conc.+Nitrogen.Conc.+total.Vol+PL+Thr+His+lacta te+PO4.apporte; 0.588, 0.811, pH+tmp+Main.P-Source.Conc.+Ni trogen.Conc.+total.sugar+PL+emission_O2+Thr+His+lactate+PO4. apporte; 0.588, 0.811, pH+tmp+Main.P-Source.Conc.+Main.Thr. Conc.+Nitrogen.Conc.+PL+emission_O2+Thr+His+lactate+PO4.app orte; 0.595, 0.811, tmp+Main.P-Source.Conc.+P-Source.Feed.c onc.+Main.Thr.Conc.+Nitrogen.Conc.+total.Vol+Thr+His+lactat e+PO4.apporte; 0.603, 0.811, tmp+Main.P-Source.Conc.+Nitrog en.Conc.+total.sugar+emission_CO2+Thr+His+lactate+PO4.appor te; 0.603, 0.811, tmp+Main.P-Source.Conc.+Main.Thr.Conc.+Ni trogen.Conc.+PL+rab+Thr+lactate+PO4.apporte; 0.595, 0.811, tmp+Main.P-Source.Conc.+Main.Thr.Conc.+Nitrogen.Conc.+total. Vol+PL+emission_O2+Thr+lactate+PO4.apporte; 0.580, 0.811, p H+tmp+Main.P-Source.Conc.+P-Source.Feed.conc.+Main.Thr.Conc. +Nitrogen.Conc.+PL+emission_O2+emission_CO2+Thr+lactate+PO4. apporte; 0.588, 0.811, tmp+Main.P-Source.Conc.+P-Source.Fee d.conc.+Nitrogen.Conc.+total.Vol+emission_O2+emission_CO2+T hr+His+lactate+PO4.apporte; 0.595, 0.811, tmp+Main.P-Source. Conc.+Main.Thr.Conc.+Nitrogen.Conc.+total.sugar+PL+emission O2+Thr+lactate+PO4.apporte; 0.580, 0.811, pH+tmp+Main.P-So urce.Conc.+Main.Thr.Conc.+Nitrogen.Conc.+total.Vol+PL+emiss ion_CO2+rab+Thr+lactate+PO4.apporte; 0.588, 0.811, pH+tmp+M ain.P-Source.Conc.+Nitrogen.Conc.+total.Vol+total.sugar+emi ssion_O2+Thr+His+lactate+PO4.apporte; 0.588, 0.811, pH+tmp+ Main.P-Source.Conc.+Main.Thr.Conc.+Nitrogen.Conc.+total.Vol +total.sugar+PL+Thr+lactate+PO4.apporte; 0.595, 0.811, pH+t mp+Main.P-Source.Conc.+Nitrogen.Conc.+emission_CO2+rab+Thr+ His+lactate+PO4.apporte; 0.595, 0.811, tmp+Main.P-Source.Co nc.+P-Source.Feed.conc.+Nitrogen.Conc.+total.sugar+PL+Thr+H is+lactate+PO4.apporte; 0.587, 0.811, tmp+Main.P-Source.Con c.+P-Source.Feed.conc.+Nitrogen.Conc.+total.Vol+PL+emission _O2+emission_CO2+Thr+lactate+PO4.apporte; 0.595, 0.811, tmp +Main.P-Source.Conc.+P-Source.Feed.conc.+Nitrogen.Conc.+tot al.Vol+PL+Thr+His+lactate+PO4.apporte; 0.595, 0.811, tmp+Ma in.P-Source.Conc.+Nitrogen.Conc.+total.sugar+PL+emission_O2 +emission_CO2+Thr+lactate+PO4.apporte; 0.587, 0.811, pH+tmp +Main.P-Source.Conc.+P-Source.Feed.conc.+Main.Thr.Conc.+Nit rogen.Conc.+emission_CO2+Thr+His+lactate+PO4.apporte; 0.602, 0.811, tmp+Main.P-Source.Conc.+Nitrogen.Conc.+total.sugar+ emission_CO2+rab+Thr+lactate+PO4.apporte; 0.595, 0.811, tmp +Main.P-Source.Conc.+P-Source.Feed.conc.+Nitrogen.Conc.+tot al.Vol+total.sugar+emission_O2+Thr+lactate+PO4.apporte; 0.5 87, 0.811, pH+tmp+Main.P-Source.Conc.+P-Source.Feed.conc.+M ain.Thr.Conc.+Nitrogen.Conc.+total.Vol+Thr+His+lactate+PO4. apporte; 0.587, 0.811, pH+tmp+Main.P-Source.Conc.+P-Source. Feed.conc.+Nitrogen.Conc.+total.Vol+total.sugar+emission_O2 +Thr+lactate+PO4.apporte; 0.594, 0.810, tmp+Main.P-Source.C onc.+Main.Thr.Conc.+Nitrogen.Conc.+PL+emission_O2+Thr+His+l actate+PO4.apporte; 0.602, 0.810, tmp+Main.P-Source.Conc.+P -Source.Feed.conc.+Main.Thr.Conc.+Nitrogen.Conc.+rab+Thr+la ctate+PO4.apporte; 0.579, 0.810, pH+tmp+Main.P-Source.Conc. +P-Source.Feed.conc.+Nitrogen.Conc.+PL+emission_O2+emission _CO2+Thr+His+lactate+PO4.apporte; 0.594, 0.810, tmp+Main.P-Source.Conc.+P-Source.Feed.conc.+Nitrogen.Conc.+total.sugar +emission_CO2+Thr+His+lactate+PO4.apporte; 0.579, 0.810, pH +tmp+Main.P-Source.Conc.+Nitrogen.Conc.+total.Vol+PL+emissi on_CO2+rab+Thr+His+lactate+PO4.apporte; 0.594, 0.810, tmp+M ain.P-Source.Conc.+Main.Thr.Conc.+Nitrogen.Conc.+total.suga r+emission_O2+Thr+His+lactate+PO4.apporte; 0.594, 0.810, pH +tmp+Main.P-Source.Conc.+Nitrogen.Conc.+PL+rab+Thr+His+lact ate+PO4.apporte; 0.586, 0.810, pH+tmp+Main.P-Source.Conc.+P -Source.Feed.conc.+Main.Thr.Conc.+Nitrogen.Conc.+total.suga r+PL+Thr+lactate+PO4.apporte; 0.579, 0.810, pH+tmp+Main.P-S ource.Conc.+P-Source.Feed.conc.+Main.Thr.Conc.+Nitrogen.Con c.+emission_O2+emission_CO2+Thr+His+lactate+PO4.apporte; 0. 586, 0.810, tmp+Main.P-Source.Conc.+P-Source.Feed.conc.+Nit rogen.Conc.+PL+emission CO2+rab+Thr+His+lactate+PO4.apport e; 0.594, 0.810, tmp+Main.P-Source.Conc.+P-Source.Feed.conc. +Main.Thr.Conc.+Nitrogen.Conc.+total.sugar+emission_CO2+Thr +lactate+PO4.apporte; 0.586, 0.810, pH+tmp+Main.P-Source.Co nc.+Nitrogen.Conc.+total.Vol+total.sugar+emission_CO2+rab+T hr+lactate+PO4.apporte; 0.594, 0.810, tmp+Main.P-Source.Con c.+Main.Thr.Conc.+Nitrogen.Conc.+total.sugar+emission_O2+em ission_CO2+Thr+lactate+PO4.apporte; 0.594, 0.810, tmp+Main. P-Source.Conc.+Nitrogen.Conc.+total.sugar+PL+emission_O2+Th r+His+lactate+PO4.apporte; 0.594, 0.810, tmp+Main.P-Source. Conc.+Main.Thr.Conc.+Nitrogen.Conc.+total.Vol+emission_O2+T hr+His+lactate+PO4.apporte; 0.586, 0.810, tmp+Main.P-Source. Conc.+P-Source.Feed.conc.+Main.Thr.Conc.+Nitrogen.Conc.+tot al.Vol+emission_CO2+rab+Thr+lactate+PO4.apporte; 0.578, 0.8 10, pH+tmp+Main.P-Source.Conc.+P-Source.Feed.conc.+Main.Thr. Conc.+Nitrogen.Conc.+total.Vol+emission_CO2+rab+Thr+lactate +PO4.apporte; 0.586, 0.810, pH+tmp+Main.P-Source.Conc.+Nitr ogen.Conc.+total.Vol+total.sugar+PL+emission_CO2+Thr+lactat e+PO4.apporte; 0.586, 0.810, pH+tmp+Main.P-Source.Conc.+P-S ource.Feed.conc.+Nitrogen.Conc.+emission_CO2+rab+Thr+His+la ctate+PO4.apporte; 0.586, 0.810, pH+tmp+Main.P-Source.Conc. +Nitrogen.Conc.+PL+emission_CO2+rab+Thr+His+lactate+PO4.app orte; 0.594, 0.810, tmp+Main.P-Source.Conc.+Nitrogen.Conc.+ total.Vol+PL+emission_O2+Thr+His+lactate+PO4.apporte; 0.586, 0.810, pH+tmp+Main.P-Source.Conc.+Main.Thr.Conc.+Nitrogen. Conc.+total.sugar+PL+emission_CO2+Thr+lactate+PO4.apporte; 0.586, 0.810, pH+tmp+Main.P-Source.Conc.+Main.Thr.Conc.+Nit rogen.Conc.+total.Vol+total.sugar+emission_CO2+Thr+lactate+ PO4.apporte; 0.593, 0.810, pH+tmp+Main.P-Source.Conc.+Nitro gen.Conc.+total.sugar+PL+rab+Thr+lactate+PO4.apporte; 0.586, 0.810, pH+tmp+Main.P-Source.Conc.+P-Source.Feed.conc.+Main. Thr.Conc.+Nitrogen.Conc.+total.sugar+Thr+His+lactate+PO4.ap porte; 0.593, 0.810, tmp+Main.P-Source.Conc.+Nitrogen.Conc. +total.sugar+emission_O2+emission_CO2+Thr+His+lactate+PO4.a pporte; 0.586, 0.810, pH+tmp+Main.P-Source.Conc.+P-Source.F eed.conc.+Main.Thr.Conc.+Nitrogen.Conc.+total.sugar+emissio n_CO2+Thr+lactate+PO4.apporte; 0.586, 0.810, tmp+Main.P-Sou rce.Conc.+P-Source.Feed.conc.+Nitrogen.Conc.+total.sugar+em ission_O2+emission_CO2+Thr+His+lactate+PO4.apporte; 0.586, 0.810, tmp+Main.P-Source.Conc.+P-Source.Feed.conc.+Nitrogen. Conc.+total.Vol+PL+emission_CO2+rab+Thr+lactate+PO4.apport e; 0.585, 0.810, pH+tmp+Main.P-Source.Conc.+Main.Thr.Conc.+ Nitrogen.Conc.+PL+emission_CO2+Thr+His+lactate+PO4.apporte; 0.585, 0.810, pH+tmp+Main.P-Source.Conc.+P-Source.Feed.con c.+Main.Thr.Conc.+Nitrogen.Conc.+total.Vol+total.sugar+Thr+ lactate+PO4.apporte; 0.585, 0.810, pH+tmp+Main.P-Source.Con c.+Main.Thr.Conc.+Nitrogen.Conc.+total.sugar+PL+Thr+His+lac tate+PO4.apporte; 0.593, 0.810, tmp+Main.P-Source.Conc.+P-S ource.Feed.conc.+Nitrogen.Conc.+total.sugar+PL+emission_CO2 +Thr+lactate+PO4.apporte; 0.600, 0.810, pH+tmp+Main.P-Sourc e.Conc.+Nitrogen.Conc.+rab+Thr+His+lactate+PO4.apporte; 0.6 00, 0.810, tmp+Main.P-Source.Conc.+P-Source.Feed.conc.+Nitr ogen.Conc.+rab+Thr+His+lactate+PO4.apporte; 0.585, 0.810, t mp+Main.P-Source.Conc.+P-Source.Feed.conc.+Main.Thr.Conc.+N itrogen.Conc.+total.sugar+emission_O2+emission_CO2+Thr+lact ate+PO4.apporte; 0.593, 0.810, tmp+Main.P-Source.Conc.+P-So urce.Feed.conc.+Main.Thr.Conc.+Nitrogen.Conc.+total.Vol+tot al.sugar+Thr+lactate+PO4.apporte; 0.593, 0.810, tmp+Main.P-Source.Conc.+P-Source.Feed.conc.+Nitrogen.Conc.+total.Vol+t otal.sugar+Thr+His+lactate+PO4.apporte; 0.585, 0.810, pH+tm p+Main.P-Source.Conc.+Main.Thr.Conc.+Nitrogen.Conc.+total.V ol+total.sugar+Thr+His+lactate+PO4.apporte; 0.585, 0.809, p H+tmp+Main.P-Source.Conc.+P-Source.Feed.conc.+Nitrogen.Conc. +total.Vol+total.sugar+emission_CO2+Thr+lactate+PO4.apport e; 0.585, 0.809, pH+tmp+Main.P-Source.Conc.+Nitrogen.Conc.+ total.Vol+total.sugar+emission_CO2+Thr+His+lactate+PO4.appo rte; 0.600, 0.809, pH+tmp+Main.P-Source.Conc.+Nitrogen.Conc. +total.sugar+rab+Thr+lactate+PO4.apporte; 0.577, 0.809, pH+ tmp+Main.P-Source.Conc.+Main.Thr.Conc.+Nitrogen.Conc.+total. Vol+total.sugar+emission_O2+emission_CO2+Thr+lactate+PO4.ap porte; 0.600, 0.809, tmp+Main.P-Source.Conc.+Nitrogen.Conc. +total.Vol+total.sugar+emission_CO2+Thr+lactate+PO4.apport e; 0.585, 0.809, tmp+Main.P-Source.Conc.+P-Source.Feed.conc. +Nitrogen.Conc.+total.Vol+emission_CO2+rab+Thr+His+lactate+ PO4.apporte; 0.592, 0.809, tmp+Main.P-Source.Conc.+Main.Thr. Conc.+Nitrogen.Conc.+emission_CO2+rab+Thr+His+lactate+PO4.a pporte; 0.585, 0.809, pH+tmp+Main.P-Source.Conc.+P-Source.F eed.conc.+Main.Thr.Conc.+Nitrogen.Conc.+PL+rab+Thr+lactate+ PO4.apporte; 0.584, 0.809, tmp+Main.P-Source.Conc.+P-Source. Feed.conc.+Main.Thr.Conc.+Nitrogen.Conc.+emission_CO2+rab+T hr+His+lactate+PO4.apporte; 0.613, 0.809, pH+tmp+Main.P-Sou rce.Conc.+Nitrogen.Conc.+total.sugar+Thr+PO4.apporte; 0.577, 0.809, pH+tmp+Main.P-Source.Conc.+P-Source.Feed.conc.+Main. Thr.Conc.+Nitrogen.Conc.+total.sugar+emission_O2+emission_C O2+Thr+lactate+PO4.apporte; 0.584, 0.809, pH+tmp+Main.P-Sou rce.Conc.+P-Source.Feed.conc.+Nitrogen.Conc.+total.sugar+PL +emission_CO2+Thr+lactate+PO4.apporte; 0.584, 0.809, pH+tmp +Main.P-Source.Conc.+P-Source.Feed.conc.+Nitrogen.Conc.+tot al.sugar+emission_CO2+Thr+His+lactate+PO4.apporte; 0.577, 0. 809, pH+tmp+Main.P-Source.Conc.+Main.Thr.Conc.+Nitrogen.Con c.+total.sugar+PL+emission_O2+emission_CO2+Thr+lactate+PO4. apporte; 0.584, 0.809, pH+tmp+Main.P-Source.Conc.+P-Source. Feed.conc.+Nitrogen.Conc.+total.Vol+total.sugar+PL+Thr+lact ate+PO4.apporte; 0.576, 0.809, pH+tmp+Main.P-Source.Conc.+P -Source.Feed.conc.+Nitrogen.Conc.+total.Vol+emission_CO2+ra b+Thr+His+lactate+PO4.apporte; 0.592, 0.809, tmp+Main.P-Sou rce.Conc.+Nitrogen.Conc.+total.sugar+PL+emission_CO2+rab+Th r+lactate+PO4.apporte; 0.592, 0.809, tmp+Main.P-Source.Conc. +Main.Thr.Conc.+Nitrogen.Conc.+total.sugar+PL+Thr+His+lacta te+PO4.apporte; 0.592, 0.809, pH+tmp+Main.P-Source.Conc.+P-Source.Feed.conc.+Nitrogen.Conc.+total.Vol+rab+Thr+lactate+ PO4.apporte; 0.576, 0.809, pH+tmp+Main.P-Source.Conc.+Nitro gen.Conc.+total.Vol+total.sugar+emission_O2+emission_CO2+Th r+His+lactate+PO4.apporte; 0.576, 0.809, pH+tmp+Main.P-Sour ce.Conc.+P-Source.Feed.conc.+Nitrogen.Conc.+total.Vol+total. sugar+emission_O2+emission_CO2+Thr+lactate+PO4.apporte; 0.5 92, 0.809, tmp+Main.P-Source.Conc.+Main.Thr.Conc.+Nitrogen. Conc.+total.Vol+PL+emission_CO2+Thr+lactate+PO4.apporte; 0. 576, 0.809, pH+tmp+Main.P-Source.Conc.+Main.Thr.Conc.+Nitro gen.Conc.+PL+emission_O2+emission_CO2+Thr+His+lactate+PO4.a pporte; 0.591, 0.809, pH+tmp+Main.P-Source.Conc.+P-Source.F eed.conc.+Nitrogen.Conc.+rab+Thr+His+lactate+PO4.apporte; 0. 606, 0.809, tmp+Main.P-Source.Conc.+Nitrogen.Conc.+total.Vo l+rab+Thr+lactate+PO4.apporte; 0.584, 0.809, pH+tmp+Main.P-Source.Conc.+P-Source.Feed.conc.+Nitrogen.Conc.+total.Vol+t otal.sugar+Thr+His+lactate+PO4.apporte; 0.584, 0.809, pH+tm p+Main.P-Source.Conc.+Main.Thr.Conc.+Nitrogen.Conc.+total.s ugar+emission_CO2+Thr+His+lactate+PO4.apporte; 0.584, 0.809, tmp+Main.P-Source.Conc.+P-Source.Feed.conc.+Nitrogen.Conc. +total.sugar+PL+emission_O2+emission_CO2+Thr+lactate+PO4.ap porte; 0.584, 0.809, pH+tmp+Main.P-Source.Conc.+Nitrogen.Co nc.+total.Vol+total.sugar+PL+Thr+His+lactate+PO4.apporte; 0. 584, 0.809, pH+tmp+Main.P-Source.Conc.+P-Source.Feed.conc.+ Main.Thr.Conc.+Nitrogen.Conc.+PL+Thr+His+lactate+PO4.apport e; 0.599, 0.809, tmp+Main.P-Source.Conc.+Nitrogen.Conc.+tot al.Vol+PL+rab+Thr+lactate+PO4.apporte; 0.576, 0.809, pH+tmp +Main.P-Source.Conc.+Nitrogen.Conc.+total.Vol+total.sugar+P L+emission_O2+emission_CO2+Thr+lactate+PO4.apporte; 0.584, 0.809, pH+tmp+Main.P-Source.Conc.+P-Source.Feed.conc.+Nitro gen.Conc.+total.sugar+PL+Thr+His+lactate+PO4.apporte; 0.584, 0.809, pH+tmp+Main.P-Source.Conc.+Nitrogen.Conc.+total.sug ar+PL+emission_CO2+Thr+His+lactate+PO4.apporte; 0.591, 0.80 9, tmp+Main.P-Source.Conc.+Nitrogen.Conc.+total.Vol+total.s ugar+emission_O2+emission_CO2+Thr+lactate+PO4.apporte; 0.57 6, 0.809, pH+tmp+Main.P-Source.Conc.+P-Source.Feed.conc.+Ma in.Thr.Conc.+Nitrogen.Conc.+total.Vol+PL+emission_CO2+Thr+l actate+PO4.apporte; 0.576, 0.809, pH+tmp+Main.P-Source.Conc. +P-Source.Feed.conc.+Main.Thr.Conc.+Nitrogen.Conc.+total.Vo l+PL+emission_O2+Thr+lactate+PO4.apporte; 0.576, 0.809, pH+ tmp+Main.P-Source.Conc.+P-Source.Feed.conc.+Nitrogen.Conc.+ total.sugar+emission_O2+emission_CO2+Thr+His+lactate+PO4.ap porte; 0.576, 0.809, pH+tmp+Main.P-Source.Conc.+Main.Thr.Co nc.+Nitrogen.Conc.+total.Vol+emission_CO2+rab+Thr+His+lacta te+PO4.apporte; 0.591, 0.809, tmp+Main.P-Source.Conc.+Main. Thr.Conc.+Nitrogen.Conc.+total.Vol+PL+Thr+His+lactate+PO4.a pporte; 0.575, 0.809, pH+tmp+Main.P-Source.Conc.+P-Source.F eed.conc.+Nitrogen.Conc.+total.sugar+PL+emission_O2+emissio n_CO2+Thr+lactate+PO4.apporte; 0.591, 0.809, tmp+Main.P-Sou rce.Conc.+Main.Thr.Conc.+Nitrogen.Conc.+total.Vol+total.sug ar+emission_O2+Thr+lactate+PO4.apporte; 0.591, 0.809, tmp+M ain.P-Source.Conc.+P-Source.Feed.conc.+Nitrogen.Conc.+total. Vol+total.sugar+PL+Thr+lactate+PO4.apporte; 0.583, 0.808, t mp+Main.P-Source.Conc.+P-Source.Feed.conc.+Main.Thr.Conc.+N itrogen.Conc.+total.sugar+emission_O2+Thr+His+lactate+PO4.a pporte; 0.583, 0.808, tmp+Main.P-Source.Conc.+P-Source.Feed. conc.+Main.Thr.Conc.+Nitrogen.Conc.+PL+emission_O2+Thr+His+ lactate+PO4.apporte; 0.598, 0.808, tmp+Main.P-Source.Conc.+ P-Source.Feed.conc.+Nitrogen.Conc.+total.Vol+rab+Thr+lactat e+PO4.apporte; 0.590, 0.808, tmp+Main.P-Source.Conc.+P-Sour ce.Feed.conc.+Nitrogen.Conc.+total.Vol+total.sugar+emission CO2+Thr+lactate+PO4.apporte; 0.590, 0.808, tmp+Main.P-Sour ce.Conc.+P-Source.Feed.conc.+Main.Thr.Conc.+Nitrogen.Conc.+ PL+rab+Thr+lactate+PO4.apporte; 0.590, 0.808, tmp+Main.P-So urce.Conc.+Nitrogen.Conc.+total.Vol+total.sugar+emission_O2 +Thr+His+lactate+PO4.apporte; 0.583, 0.808, pH+tmp+Main.P-S ource.Conc.+P-Source.Feed.conc.+Nitrogen.Conc.+total.Vol+PL +rab+Thr+lactate+PO4.apporte; 0.598, 0.808, tmp+Main.P-Sour ce.Conc.+Nitrogen.Conc.+PL+rab+Thr+His+lactate+PO4.apporte; 0.575, 0.808, pH+tmp+Main.P-Source.Conc.+Main.Thr.Conc.+Ni trogen.Conc.+total.Vol+PL+emission_O2+Thr+His+lactate+PO4.a pporte; 0.575, 0.808, pH+tmp+Main.P-Source.Conc.+P-Source.F eed.conc.+Main.Thr.Conc.+Nitrogen.Conc.+PL+emission_CO2+rab +Thr+lactate+PO4.apporte; 0.582, 0.808, tmp+Main.P-Source.C onc.+Main.Thr.Conc.+Nitrogen.Conc.+total.Vol+PL+emission_CO 2+rab+Thr+lactate+PO4.apporte; 0.590, 0.808, tmp+Main.P-Sou rce.Conc.+Main.Thr.Conc.+Nitrogen.Conc.+PL+emission_CO2+Thr +His+lactate+PO4.apporte; 0.590, 0.808, tmp+Main.P-Source.C onc.+Nitrogen.Conc.+total.Vol+total.sugar+emission_CO2+rab+ Thr+lactate+PO4.apporte; 0.582, 0.808, tmp+Main.P-Source.Co nc.+P-Source.Feed.conc.+Main.Thr.Conc.+Nitrogen.Conc.+total. Vol+PL+emission_O2+Thr+lactate+PO4.apporte; 0.590, 0.808, t mp+Main.P-Source.Conc.+Nitrogen.Conc.+total.Vol+total.sugar +PL+emission_O2+Thr+lactate+PO4.apporte; 0.574, 0.808, pH+t mp+Main.P-Source.Cone.+Nitrogen.Conc.+total.sugar+PL+emissi on_O2+emission_CO2+Thr+His+lactate+PO4.apporte; 0.582, 0.80 8, tmp+Main.P-Source.Conc.+P-Source.Feed.conc.+Main.Thr.Con c.+Nitrogen.Conc.+total.Vol+emission_O2+Thr+His+lactate+PO4. apporte; 0.574, 0.808, pH+tmp+Main.P-Source.Conc.+P-Source. Feed.conc.+Nitrogen.Conc.+total.Vol+PL+emission_O2+Thr+His+ lactate+PO4.apporte; 0.582, 0.808, tmp+Main.P-Source.Conc.+ Main.Thr.Conc.+Nitrogen.Conc.+total.Vol+PL+emission_O2+emis sion_CO2+Thr+lactate+PO4.apporte; 0.590, 0.808, tmp+Main.P-Source.Conc.+Nitrogen.Conc.+total.Vol+PL+emission_CO2+Thr+H is+lactate+PO4.apporte; 0.590, 0.808, tmp+Main.P-Source.Con c.+Main.Thr.Conc.+Nitrogen.Conc.+total.Vol+emission_CO2+Thr +His+lactate+PO4.apporte; 0.582, 0.808, tmp+Main.P-Source.C onc.+P-Source.Feed.conc.+Main.Thr.Conc.+Nitrogen.Conc.+tota l.sugar+PL+emission_O2+Thr+lactate+PO4.apporte; 0.574, 0.80 8, pH+tmp+Main.P-Source.Conc.+P-Source.Feed.conc.+Main.Thr. Conc.+Nitrogen.Conc.+total.Vol+emission_O2+Thr+His+lactate+ PO4.apporte; 0.604, 0.808, tmp+Main.P-Source.Conc.+Nitrogen. Conc.+rab+Thr+His+lactate+PO4.apporte; 0.574, 0.808, pH+tmp +Main.P-Source.Conc.+Main.Thr.Conc.+Nitrogen.Conc.+total.su gar+emission_O2+emission_CO2+Thr+His+lactate+PO4.apporte; 0. 597, 0.808, tmp+Main.P-Source.Conc.+Nitrogen.Conc.+total.su gar+PL+rab+Thr+lactate+PO4.apporte; 0.589, 0.808, tmp+Main. P-Source.Conc.+Main.Thr.Conc.+Nitrogen.Conc.+total.Vol+tota l.sugar+PL+Thr+lactate+PO4.apporte; 0.582, 0.808, tmp+Main. P-Source.Conc.+P-Source.Feed.conc.+Nitrogen.Conc.+total.Vol +total.sugar+emission_O2+emission_CO2+Thr+lactate+PO4.appor te; 0.574, 0.808, pH+tmp+Main.P-Source.Conc.+Main.Thr.Conc. +Nitrogen.Conc.+total.Vol+PL+emission_CO2+Thr+His+lactate+P O4.apporte; 0.597, 0.808, tmp+Main.P-Source.Conc.+P-Source. Feed.conc.+Nitrogen.Conc.+total.sugar+rab+Thr+lactate+PO4.a pporte; 0.589, 0.808, pH+tmp+Main.P-Source.Conc.+P-Source.F eed.conc.+Nitrogen.Conc.+total.sugar+rab+Thr+lactate+PO4.ap porte; 0.589, 0.808, tmp+Main.P-Source.Conc.+P-Source.Feed. conc.+Nitrogen.Conc.+PL+rab+Thr+His+lactate+PO4.apporte; 0. 574, 0.808, pH+tmp+Main.P-Source.Conc.+P-Source.Feed.conc.+ Nitrogen.Conc.+total.Vol+PL+emission_CO2+Thr+His+lactate+PO 4.apporte; 0.581, 0.808, tmp+Main.P-Source.Conc.+P-Source.F eed.conc.+Nitrogen.Conc.+total.sugar+PL+emission_O2+Thr+His +lactate+PO4.apporte; 0.573, 0.808, pH+tmp+Main.P-Source.Co nc.+Main.Thr.Conc.+Nitrogen.Conc.+total.Vol+total.sugar+PL+ emission_O2+Thr+lactate+PO4.apporte; 0.581, 0.808, tmp+Main. P-Source.Conc.+P-Source.Feed.conc.+Nitrogen.Conc.+total.Vol +PL+emission_O2+Thr+His+lactate+PO4.apporte; 0.589, 0.808, pH+tmp+Main.P-Source.Conc.+Nitrogen.Conc.+total.sugar+emiss ion_CO2+rab+Thr+lactate+PO4.apporte; 0.573, 0.808, pH+tmp+M ain.P-Source.Conc.+P-Source.Feed.conc.+Main.Thr.Conc.+Nitro gen.Conc.+total.Vol+emission_CO2+Thr+His+lactate+PO4.apport e; 0.603, 0.807, tmp+Main.P-Source.Conc.+Nitrogen.Conc.+tot al.sugar+rab+Thr+lactate+PO4.apporte; 0.589, 0.807, tmp+Mai n.P-Source.Conc.+Main.Thr.Conc.+Nitrogen.Conc.+total.sugar+ PL+emission_CO2+Thr+lactate+PO4.apporte; 0.603, 0.807, pH+t mp+Main.P-Source.Conc.+Main.Thr.Conc.+Nitrogen.Conc.+total. sugar+Thr+PO4.apporte; 0.581, 0.807, tmp+Main.P-Source.Conc. +P-Source.Feed.conc.+Nitrogen.Conc.+total.sugar+PL+emission CO2+rab+Thr+lactate+PO4.apporte; 0.588, 0.807, pH+tmp+Main. P-Source.Conc.+Main.Thr.Conc.+Nitrogen.Conc.+total.sugar+ra b+Thr+lactate+PO4.apporte; 0.581, 0.807, tmp+Main.P-Source. Conc.+Main.Thr.Conc.+Nitrogen.Conc.+PL+emission_O2+emission _CO2+Thr+His+lactate+PO4.apporte; 0.581, 0.807, pH+tmp+Main. P-Source.Conc.+Nitrogen.Conc.+total.sugar+PL+emission_CO2+r ab+Thr+lactate+PO4.apporte; 0.588, 0.807, tmp+Main.P-Source. Conc.+Main.Thr.Conc.+Nitrogen.Conc.+total.Vol+total.sugar+T hr+His+lactate+PO4.apporte; 0.581, 0.807, tmp+Main.P-Source. Conc.+Main.Thr.Conc.+Nitrogen.Conc.+total.Vol+emission_O2+e mission_CO2+Thr+His+lactate+PO4.apporte; 0.581, 0.807, pH+t mp+Main.P-Source.Conc.+P-Source.Feed.conc.+Nitrogen.Conc.+t otal.sugar+emission_CO2+rab+Thr+lactate+PO4.apporte; 0.581, 0.807, pH+tmp+Main.P-Source.Conc.+P-Source.Feed.conc.+Nitr ogen.Conc.+PL+rab+Thr+His+lactate+PO4.apporte; 0.581, 0.807, tmp+Main.P-Source.Conc.+Main.Thr.Conc.+Nitrogen.Conc.+tota l.Vol+emission_CO2+rab+Thr+His+lactate+PO4.apporte; 0.573, 0.807, pH+tmp+Main.P-Source.Conc.+Nitrogen.Conc.+total.Vol+ total.sugar+PL+emission_CO2+rab+Thr+lactate+PO4.apporte; 0. 564, 0.807, pH+tmp+Main.P-Source.Conc.+P-Source.Feed.conc.+ Main.Thr.Conc.+Nitrogen.Conc.+total.Vol+PL+emission_O2+emis sion_CO2+Thr+lactate+PO4.apporte; 0.564, 0.807, pH+tmp+Main. P-Source.Conc.+P-Source.Feed.conc.+Main.Thr.Conc.+Nitrogen. Conc.+total.Vol+emission_O2+emission_CO2+Thr+His+lactate+PO 4.apporte; 0.573, 0.807, pH+tmp+Main.P-Source.Conc.+P-Sourc e.Feed.conc.+Main.Thr.Conc.+Nitrogen.Conc.+total.sugar+PL+e mission_O2+Thr+lactate+PO4.apporte; 0.572, 0.807, pH+tmp+Ma in.P-Source.Conc.+P-Source.Feed.conc.+Main.Thr.Conc.+Nitrog en.Conc.+total.sugar+emission_O2+Thr+His+lactate+PO4.apport e; 0.580, 0.807, tmp+Main.P-Source.Conc.+P-Source.Feed.conc. +Main.Thr.Conc.+Nitrogen.Conc.+total.sugar+emission CO2+rab +Thr+lactate+PO4.apporte; 0.580, 0.807, tmp+Main.P-Source.C onc.+Nitrogen.Conc.+total.Vol+PL+emission_CO2+rab+Thr+His+l actate+PO4.apporte; 0.580, 0.807, tmp+Main.P-Source.Conc.+N itrogen.Conc.+total.Vol+PL+emission_O2+emission_CO2+Thr+His +lactate+PO4.apporte; 0.588, 0.807, tmp+Main.P-Source.Conc. +Main.Thr.Conc.+Nitrogen.Conc.+total.sugar+emission_CO2+rab +Thr+lactate+PO4.apporte; 0.580, 0.807, pH+tmp+Main.P-Sourc e.Conc.+Main.Thr.Conc.+Nitrogen.Conc.+total.sugar+PL+rab+Th r+lactate+PO4.apporte; 0.572, 0.807, pH+tmp+Main.P-Source.C onc.+P-Source.Feed.conc.+Nitrogen.Conc.+PL+emission_CO2+rab +Thr+His+lactate+PO4.apporte; 0.580, 0.807, tmp+Main.P-Sour ce.Conc.+Main.Thr.Conc.+Nitrogen.Conc.+PL+emission_CO2+rab+ Thr+His+lactate+PO4.apporte; 0.572, 0.807, pH+tmp+Main.P-So urce.Conc.+Main.Thr.Conc.+Nitrogen.Conc.+total.sugar+PL+emi ssion_O2+Thr+His+lactate+PO4.apporte; 0.572, 0.807, pH+tmp+ Main.P-Source.Conc.+Main.Thr.Conc.+Nitrogen.Conc.+total.Vol +total.sugar+emission_O2+Thr+His+lactate+PO4.apporte;0.580, 0.807, tmp+Main.P-Source.Conc.+P-Source.Feed.conc.+Main.Th r.Conc.+Nitrogen.Conc.+total.Vol+PL+emission_CO2+Thr+lactat e+PO4.apporte; 0.587, 0.807, tmp+Main.P-Source.Conc.+P-Sour ce.Feed.conc.+Nitrogen.Conc.+total.Vol+PL+rab+Thr+lactate+P O4.apporte; 0.587, 0.807, tmp+Main.P-Source.Conc.+Nitrogen. Conc.+total.sugar+PL+emission_CO2+Thr+His+lactate+PO4.appor te; 0.580, 0.807, tmp+Main.P-Source.Conc.+P-Source.Feed.con c.+Nitrogen.Conc.+total.sugar+emission_CO2+rab+Thr+His+lact ate+PO4.apporte; 0.615, 0.807, tmp+Main.P-Source.Conc.+Nitr ogen.Conc.+total.sugar+Thr+PO4.apporte; 0.572, 0.807, pH+tm p+Main.P-Source.Conc.+P-Source.Feed.conc.+Main.Thr.Conc.+Ni trogen.Conc.+total.Vol+total.sugar+emission_O2+Thr+lactate+ PO4.apporte; 0.587, 0.807, tmp+Main.P-Source.Conc.+Main.Thr. Conc.+Nitrogen.Conc.+total.sugar+emission_CO2+Thr+His+lacta te+PO4.apporte; 0.580, 0.807, tmp+Main.P-Source.Conc.+P-Sou rce.Feed.conc.+Main.Thr.Conc.+Nitrogen.Conc.+PL+emission_CO 2+Thr+His+lactate+PO4.apporte; 0.563, 0.807, pH+tmp+Main.P-Source.Conc.+Main.Thr.Conc.+Nitrogen.Conc.+total.Vol+PL+emi ssion_O2+emission_CO2+Thr+His+lactate+PO4.apporte; 0.579, 0. 807, tmp+Main.P-Source.Conc.+Main.Thr.Conc.+Nitrogen.Conc.+ total.sugar+PL+emission_O2+emission_CO2+Thr+lactate+PO4.app orte; 0.587, 0.807, pH+tmp+Main.P-Source.Conc.+Main.Thr.Con c.+Nitrogen.Conc.+total.Vol+rab+Thr+lactate+PO4.apporte; 0. 579, 0.807, pH+tmp+Main.P-Source.Conc.+Main.Thr.Conc.+Nitro gen.Conc.+total.Vol+PL+rab+Thr+lactate+PO4.apporte; 0.594, 0.807, pH+tmp+Main.P-Source.Conc.+Main.Thr.Conc.+Nitrogen.C onc.+total.Vol+total.sugar+lactate+PO4.apporte; 0.608, 0.80 6, tmp+Main.P-Source.Conc.+P-Source.Feed.conc.+Nitrogen.Con c.+His+lactate+PO4.apporte; 0.579, 0.806, tmp+Main.P-Source. Conc.+P-Source.Feed.conc.+Main.Thr.Conc.+Nitrogen.Conc.+tot al.Vol+emission_CO2+Thr+His+lactate+PO4.apporte; 0.571, 0.8 06, pH+tmp+Main.P-Source.Conc.+Main.Thr.Conc.+Nitrogen.Conc. +total.Vol+total.sugar+emission_CO2+rab+Thr+lactate+PO4.app orte; 0.587, 0.806, tmp+Main.P-Source.Conc.+Nitrogen.Conc.+ total.Vol+total.sugar+PL+Thr+His+lactate+PO4.apporte; 0.571, 0.806, pH+tmp+Main.P-Source.Conc.+Main.Thr.Conc.+Nitrogen. Conc.+total.Vol+total.sugar+PL+emission_CO2+Thr+lactate+PO4. apporte; 0.571, 0.806, pH+tmp+Main.P-Source.Conc.+P-Source. Feed.conc.+Main.Thr.Conc.+Nitrogen.Conc.+PL+emission_O2+Thr +His+lactate+PO4.apporte; 0.579, 0.806, tmp+Main.P-Source.C onc.+P-Source.Feed.conc.+Nitrogen.Conc.+total.Vol+total.sug ar+emission_O2+Thr+His+lactate+PO4.apporte; 0.571, 0.806, p H+tmp+Main.P-Source.Conc.+P-Source.Feed.conc.+Nitrogen.Conc. +total.Vol+total.sugar+emission_CO2+rab+Thr+lactate+PO4.app orte; 0.563, 0.806, pH+tmp+Main.P-Source.Conc.+P-Source.Fee d.conc.+Nitrogen.Conc.+total.Vol+PL+emission_O2+emission_CO 2+Thr+His+lactate+PO4.apporte; 0.579, 0.806, tmp+Main.P-Sou rce.Conc.+P-Source.Feed.conc.+Main.Thr.Conc.+Nitrogen.Conc. +total.Vol+total.sugar+emission_O2+Thr+lactate+PO4.apporte; 0.601, 0.806, pH+tmp+Main.P-Source.Conc.+Nitrogen.Conc.+to tal.Vol+total.sugar+lactate+PO4.apporte; 0.579, 0.806, tmp+ Main.P-Source.Conc.+P-Source.Feed.conc.+Nitrogen.Conc.+tota l.Vol+total.sugar+emission_CO2+rab+Thr+lactate+PO4.apporte; 0.579, 0.806, tmp+Main.P-Source.Conc.+P-Source.Feed.conc.+ Nitrogen.Conc.+total.Vol+PL+emission_CO2+Thr+His+lactate+PO 4.apporte; 0.586, 0.806, tmp+Main.P-Source.Conc.+Nitrogen.C onc.+total.sugar+emission_CO2+rab+Thr+His+lactate+PO4.appor te; 0.578, 0.806, pH+tmp+Main.P-Source.Conc.+Main.Thr.Conc. +Nitrogen.Conc.+emission_CO2+rab+Thr+His+lactate+PO4.apport e; 0.578, 0.806, pH+tmp+Main.P-Source.Conc.+P-Source.Feed.c onc.+Nitrogen.Conc.+total.sugar+PL+rab+Thr+lactate+PO4.appo rte; 0.570, 0.806, pH+tmp+Main.P-Source.Conc.+P-Source.Feed. conc.+Nitrogen.Conc.+total.Vol+total.sugar+emission_O2+Thr+ His+lactate+PO4.apporte; 0.578, 0.806, tmp+Main.P-Source.Co nc.+P-Source.Feed.conc.+Main.Thr.Conc.+Nitrogen.Conc.+total. sugar+PL+Thr+His+lactate+PO4.apporte; 0.570, 0.806, pH+tmp+ Main.P-Source.Conc.+P-Source.Feed.conc.+Nitrogen.Conc.+tota l.sugar+PL+emission_O2+Thr+His+lactate+PO4.apporte; 0.570, 0.806, tmp+Main.P-Source.Conc.+P-Source.Feed.conc.+Main.Thr. Conc.+Nitrogen.Conc.+total.Vol+emission_O2+emission_CO2+Thr +His+lactate+PO4.apporte; 0.570, 0.806, tmp+Main.P-Source.C onc.+P-Source.Feed.conc.+Main.Thr.Conc.+Nitrogen.Conc.+PL+e mission_O2+emission_CO2+Thr+His+lactate+PO4.apporte; 0.570, 0.806, pH+tmp+Main.P-Source.Conc.+Nitrogen.Conc.+total.Vol +total.sugar+PL+emission_O2+Thr+His+lactate+PO4.apporte; 0. 586, 0.806, pH+tmp+Main.P-Source.Conc.+Main.Thr.Conc.+Nitro gen.Conc.+total.Vol+total.sugar+emission_CO2+lactate+PO4.ap porte; 0.578, 0.806, tmp+Main.P-Source.Conc.+Nitrogen.Conc. +total.sugar+PL+emission_O2+emission_CO2+Thr+His+lactate+PO 4.apporte; 0.586, 0.806, tmp+Main.P-Source.Conc.+P-Source.F eed.conc.+Nitrogen.Conc.+total.sugar+PL+rab+Thr+lactate+PO4. apporte; 0.570, 0.806, pH+tmp+Main.P-Source.Conc.+P-Source. Feed.conc.+Nitrogen.Conc.+total.Vol+total.sugar+PL+emission O2+Thr+lactate+PO4.apporte; 0.561, 0.806, pH+tmp+Main.P-So urce.Conc.+P-Source.Feed.conc.+Main.Thr.Conc.+Nitrogen.Conc. +total.Vol+PL+emission_CO2+rab+Thr+lactate+PO4.apporte; 0.6 07, 0.806, pH+tmp+Main.P-Source.Conc.+Nitrogen.Conc.+His+la ctate+PO4.apporte; 0.578, 0.806, tmp+Main.P-Source.Conc.+P-Source.Feed.conc.+Main.Thr.Conc.+Nitrogen.Conc.+total.sugar +emission_CO2+Thr+His+lactate+PO4.apporte; 0.570, 0.806, pH +tmp+Main.P-Source.Conc.+Nitrogen.Conc.+total.Vol+total.sug ar+emission_CO2+rab+Thr+His+lactate+PO4.apporte; 0.600, 0.8 06, pH+tmp+Main.P-Source.Conc.+P-Source.Feed.conc.+Nitrogen. Conc.+His+lactate+PO4.apporte; 0.600, 0.806, tmp+Main.P-Sou rce.Conc.+P-Source.Feed.conc.+Main.Thr.Conc.+Nitrogen.Conc. +total.sugar+lactate+PO4.apporte; 0.569, 0.806, pH+tmp+Main. P-Source.Conc.+P-Source.Feed.conc.+Main.Thr.Conc.+Nitrogen. Conc.+total.Vol+total.sugar+emission CO2+Thr+lactate+PO4.ap porte; 0.577, 0.806, tmp+Main.P-Source.Conc.+P-Source.Feed. conc.+Main.Thr.Conc.+Nitrogen.Conc.+total.Vol+PL+Thr+His+la ctate+PO4.apporte; 0.577, 0.806, pH+tmp+Main.P-Source.Conc. +Main.Thr.Conc.+Nitrogen.Conc.+total.Vol+total.sugar+emissi on_O2+emission_CO2+lactate+PO4.apporte; 0.577, 0.806, tmp+M ain.P-Source.Conc.+Main.Thr.Conc.+Nitrogen.Conc.+total.suga r+PL+emission_O2+Thr+His+lactate+PO4.apporte; 0.569, 0.806, pH+tmp+Main.P-Source.Conc.+P-Source.Feed.conc.+Main.Thr.Co nc.+Nitrogen.Conc.+total.Vol+PL+Thr+His+lactate+PO4.apport e; 0.577, 0.806, tmp+Main.P-Source.Conc.+Main.Thr.Conc.+Nit rogen.Conc.+total.sugar+emission_O2+emission_CO2+Thr+His+la ctate+PO4.apporte; 0.600, 0.805, tmp+Main.P-Source.Conc.+P-Source.Feed.conc.+Nitrogen.Conc.+total.sugar+His+lactate+PO 4.apporte; 0.592, 0.805, pH+tmp+Main.P-Source.Conc.+Nitroge n.Conc.+total.Vol+total.sugar+emission_CO2+lactate+PO4.appo rte; 0.585, 0.805, pH+tmp+Main.P-Source.Conc.+Main.Thr.Conc. +Nitrogen.Conc.+rab+Thr+His+lactate+PO4.apporte; 0.585, 0.8 05, tmp+Main.P-Source.Conc.+Main.Thr.Conc.+Nitrogen.Conc.+t otal.Vol+total.sugar+emission_CO2+Thr+lactate+PO4.apporte; 0.577, 0.805, tmp+Main.P-Source.Conc.+Main.Thr.Conc.+Nitrog en.Conc.+total.sugar+PL+emission_CO2+rab+Thr+lactate+PO4.ap porte; 0.569, 0.805, pH+tmp+Main.P-Source.Conc.+P-Source.Fe ed.conc.+Main.Thr.Conc.+Nitrogen.Conc.+PL+emission_CO2+Thr+ His+lactate+PO4.apporte; 0.569, 0.805, tmp+Main.P-Source.Co nc.+P-Source.Feed.conc.+Main.Thr.Conc.+Nitrogen.Conc.+total. Vol+PL+emission_O2+emission_CO2+Thr+lactate+PO4.apporte; 0. 606, 0.805, pH+tmp+Main.P-Source.Conc.+Nitrogen.Conc.+total. sugar+lactate+PO4.apporte; 0.599, 0.805, tmp+Main.P-Source. Conc.+P-Source.Feed.conc.+Nitrogen.Conc.+rab+His+lactate+PO 4.apporte; 0.569, 0.805, pH+tmp+Main.P-Source.Conc.+P-Sourc e.Feed.conc.+Main.Thr.Conc.+Nitrogen.Conc.+total.Vol+total. sugar+PL+Thr+lactate+PO4.apporte; 0.569, 0.805, pH+tmp+Main. P-Source.Conc.+Main.Thr.Conc.+Nitrogen.Conc.+total.Vol+tota l.sugar+emission_CO2+Thr+His+lactate+PO4.apporte; 0.577, 0. 805, tmp+Main.P-Source.Conc.+Main.Thr.Conc.+Nitrogen.Conc.+ total.Vol+PL+emission_O2+Thr+His+lactate+PO4.apporte; 0.584, 0.805, tmp+Main.P-Source.Conc.+Nitrogen.Conc.+total.Vol+to tal.sugar+emission_CO2+Thr+His+lactate+PO4.apporte; 0.577, 0.805, tmp+Main.P-Source.Conc.+P-Source.Feed.conc.+Main.Thr. Conc.+Nitrogen.Conc.+total.sugar+PL+emission_CO2+Thr+lactat e+PO4.apporte; 0.569, 0.805, pH+tmp+Main.P-Source.Conc.+P-S ource.Feed.conc.+Main.Thr.Conc.+Nitrogen.Conc.+emission_CO2 +rab+Thr+His+lactate+PO4.apporte; 0.584, 0.805, pH+tmp+Main. P-Source.Conc.+Nitrogen.Conc.+total.Vol+total.sugar+emissio n_O2+emission_CO2+lactate+PO4.apporte; 0.560, 0.805, pH+tmp +Main.P-Source.Conc.+Main.Thr.Conc.+Nitrogen.Conc.+total.Vo l+total.sugar+PL+emission_O2+emission_CO2+Thr+lactate+PO4.a pporte; 0.569, 0.805, pH+tmp+Main.P-Source.Conc.+P-Source.F eed.conc.+Main.Thr.Conc.+Nitrogen.Conc.+total.sugar+PL+emis sion_CO2+Thr+lactate+PO4.apporte; 0.606, 0.805, tmp+Main.P-Source.Conc.+P-Source.Feed.conc.+Nitrogen.Conc.+total.sugar +lactate+PO4.apporte; 0.560, 0.805, pH+tmp+Main.P-Source.Co nc.+P-Source.Feed.conc.+Main.Thr.Conc.+Nitrogen.Conc.+total. Vol+total.sugar+emission_O2+emission_CO2+Thr+lactate+PO4.ap porte; 0.569, 0.805, tmp+Main.P-Source.Conc.+P-Source.Feed. conc.+Main.Thr.Conc.+Nitrogen.Conc.+total.sugar+emission_O2 +emission_CO2+Thr+His+lactate+PO4.apporte; 0.576, 0.805, pH +tmp+Main.P-Source.Conc.+P-Source.Feed.conc.+Main.Thr.Conc. +Nitrogen.Conc.+total.sugar+rab+Thr+lactate+PO4.apporte; 0. 568, 0.805, pH+tmp+Main.P-Source.Conc.+P-Source.Feed.conc.+ Nitrogen.Conc.+total.Vol+total.sugar+emission_CO2+Thr+His+l actate+PO4.apporte; 0.584, 0.805, tmp+Main.P-Source.Conc.+N itrogen.Conc.+total.Vol+total.sugar+PL+emission_CO2+Thr+lac tate+PO4.apporte; 0.568, 0.805, pH+tmp+Main.P-Source.Conc.+ P-Source.Feed.conc.+Main.Thr.Conc.+Nitrogen.Conc.+total.sug ar+emission_CO2+Thr+His+lactate+PO4.apporte; 0.576, 0.805, tmp+Main.P-Source.Conc.+P-Source.Feed.conc.+Nitrogen.Conc.+ total.Vol+total.sugar+PL+emission_O2+Thr+lactate+PO4.apport e; 0.599, 0.805, pH+tmp+Main.P-Source.Conc.+P-Source.Feed.c onc.+Nitrogen.Conc.+total.sugar+Thr+PO4.apporte; 0.599, 0.8 05, pH+tmp+Main.P-Source.Conc.+Nitrogen.Conc.+total.Vol+His +lactate+PO4.apporte; 0.568, 0.805, pH+tmp+Main.P-Source.Co nc.+P-Source.Feed.conc.+Nitrogen.Conc.+total.Vol+total.suga r+PL+emission_CO2+Thr+lactate+PO4.apporte; 0.599, 0.805, tm p+Main.P-Source.Conc.+P-Source.Feed.conc.+Nitrogen.Conc.+to tal.sugar+rab+lactate+PO4.apporte; 0.576, 0.805, tmp+Main.P -Source.Conc.+P-Source.Feed.conc.+Nitrogen.Conc.+total.suga r+PL+emission_CO2+Thr+His+lactate+PO4.apporte; 0.560, 0.805, pH+tmp+Main.P-Source.Conc.+Main.Thr.Conc.+Nitrogen.Conc.+t otal.Vol+PL+emission_CO2+rab+Thr+His+lactate+PO4.apporte; 0. 576, 0.805, pH+tmp+Main.P-Source.Conc.+Main.Thr.Conc.+Nitro gen.Conc.+PL+rab+Thr+His+lactate+PO4.apporte; 0.598, 0.805, pH+tmp+Main.P-Source.Conc.+Nitrogen.Conc.+total.sugar+emis sion_O2+Thr+PO4.apporte; 0.598, 0.805, tmp+Main.P-Source.Co nc.+P-Source.Feed.conc.+Main.Thr.Conc.+Nitrogen.Conc.+His+1 actate+PO4.apporte; 0.612, 0.805, pH+tmp+Main.P-Source.Conc. +Nitrogen.Conc.+Thr+PO4.apporte; 0.560, 0.805, pH+tmp+Main. P-Source.Conc.+Main.Thr.Conc.+Nitrogen.Conc.+total.Vol+tota l.sugar+emission_O2+emission_CO2+Thr+His+lactate+PO4.apport e; 0.568, 0.805, tmp+Main.P-Source.Conc.+P-Source.Feed.conc. +Nitrogen.Conc.+total.Vol+PL+emission_O2+emission_CO2+Thr+H is+lactate+PO4.apporte; 0.576, 0.805, tmp+Main.P-Source.Con c.+P-Source.Feed.conc.+Main.Thr.Conc.+Nitrogen.Conc.+total. Vol+total.sugar+Thr+His+lactate+PO4.apporte;0.576, 0.805, tmp+Main.P-Source.Conc.+Main.Thr.Conc.+Nitrogen.Conc.+total. Vol+total.sugar+emission_O2+emission_CO2+Thr+lactate+PO4.ap porte; 0.559, 0.805, pH+tmp+Main.P-Source.Conc.+P-Source.Fe ed.conc.+Main.Thr.Conc.+Nitrogen.Conc.+PL+emission_O2+emiss ion_CO2+Thr+His+lactate+PO4.apporte; 0.568, 0.805, pH+tmp+M ain.P-Source.Conc.+Nitrogen.Conc.+total.Vol+total.sugar+PL+ emission_CO2+Thr+His+lactate+PO4.apporte; 0.568, 0.805, tmp +Main.P-Source.Conc.+P-Source.Feed.conc.+Main.Thr.Conc.+Nit rogen.Conc.+total.Vol+PL+emission_CO2+rab+Thr+lactate+PO4.a pporte; 0.568, 0.805, pH+tmp+Main.P-Source.Conc.+Main.Thr.C onc.+Nitrogen.Conc.+total.Vol+total.sugar+PL+Thr+His+lactat e+PO4.apporte; 0.598, 0.805, tmp+Main.P-Source.Conc.+P-Sour ce.Feed.conc.+Nitrogen.Conc.+emission_O2+His+lactate+PO4.ap porte; 0.568, 0.805, pH+tmp+Main.P-Source.Conc.+P-Source.Fe ed.conc.+Main.Thr.Conc.+Nitrogen.Conc.+total.Vol+total.suga r+Thr+His+lactate+PO4.apporte; 0.559, 0.805, pH+tmp+Main.P-Source.Conc.+P-Source.Feed.conc.+Nitrogen.Conc.+total.Vol+t otal.sugar+emission_O2+emission_CO2+Thr+His+lactate+PO4.app orte; 0.591, 0.805, tmp+Main.P-Source.Conc.+P-Source.Feed.c onc.+Main.Thr.Conc.+Nitrogen.Conc.+total.sugar+His+lactate+ PO4.apporte; 0.568, 0.805, tmp+Main.P-Source.Conc.+P-Source. Feed.conc.+Main.Thr.Conc.+Nitrogen.Conc.+PL+emission_CO2+ra b+Thr+His+lactate+PO4.apporte; 0.583, 0.805, pH+tmp+Main.P-Source.Conc.+Main.Thr.Conc.+Nitrogen.Conc.+total.Vol+total. sugar+emission_O2+lactate+PO4.apporte; 0.598, 0.805, pH+tmp +Main.P-Source.Conc.+P-Source.Feed.conc.+Nitrogen.Conc.+tot al.sugar+lactate+PO4.apporte; 0.575, 0.805, pH+tmp+Main.P-S ource.Conc.+P-Source.Feed.conc.+Main.Thr.Conc.+Nitrogen.Con c.+rab+Thr+His+lactate+PO4.apporte; 0.575, 0.805, tmp+Main. P-Source.Conc.+Nitrogen.Conc.+total.Vol+total.sugar+emissio n_O2+emission_CO2+Thr+His+lactate+PO4.apporte; 0.559, 0.804, pH+tmp+Main.P-Source.Conc.+P-Source.Feed.conc.+Nitrogen.Co nc.+total.Vol+PL+emission_CO2+rab+Thr+His+lactate+PO4.appor te; 0.559, 0.804, pH+tmp+Main.P-Source.Conc.+P-Source.Feed. conc.+Main.Thr.Conc.+Nitrogen.Conc.+total.sugar+emission_O2 +emission_CO2+Thr+His+lactate+PO4.apporte; 0.567, 0.804, pH +tmp+Main.P-Source.Conc.+Main.Thr.Conc.+Nitrogen.Conc.+tota l.sugar+PL+emission_CO2+Thr+His+lactate+PO4.apporte; 0.567, 0.804, pH+tmp+Main.P-Source.Conc.+Main.Thr.Conc.+Nitrogen. Conc.+PL+emission_CO2+rab+Thr+His+lactate+PO4.apporte; 0.59 0, 0.804, tmp+Main.P-Source.Conc.+P-Source.Feed.conc.+Nitro gen.Conc.+total.sugar+rab+His+lactate+PO4.apporte; 0.567, 0. 804, tmp+Main.P-Source.Conc.+P-Source.Feed.conc.+Main.Thr.C onc.+Nitrogen.Conc.+total.sugar+PL+emission_O2+emission_CO2 +Thr+lactate+PO4.apporte; 0.559, 0.804, pH+tmp+Main.P-Sourc e.Conc.+P-Source.Feed.conc.+Main.Thr.Conc.+Nitrogen.Conc.+t otal.sugar+PL+emission_O2+emission_CO2+Thr+lactate+PO4.appo rte; 0.590, 0.804, pH+tmp+Main.P-Source.Conc.+Nitrogen.Conc. +total.Vol+total.sugar+emission_O2+lactate+PO4.apporte; 0.5 90, 0.804, pH+tmp+Main.P-Source.Conc.+Nitrogen.Conc.+total. Vol+total.sugar+rab+lactate+PO4.apporte; 0.575, 0.804, tmp+ Main.P-Source.Conc.+Main.Thr.Conc.+Nitrogen.Conc.+total.Vol +total.sugar+emission_CO2+rab+Thr+lactate+PO4.apporte; 0.57 5, 0.804, tmp+Main.P-Source.Conc.+P-Source.Feed.conc.+Main. Thr.Conc.+Nitrogen.Conc.+total.Vol+total.sugar+PL+Thr+lacta te+PO4.apporte; 0.567, 0.804, pH+tmp+Main.P-Source.Conc.+P-Source.Feed.conc.+Nitrogen.Conc.+total.sugar+PL+emission_CO 2+rab+Thr+lactate+PO4.apporte; 0.567, 0.804, tmp+Main.P-Sou rce.Conc.+P-Source.Feed.conc.+Main.Thr.Conc.+Nitrogen.Conc. +total.Vol+emission_CO2+rab+Thr+His+lactate+PO4.apporte; 0. 567, 0.804, pH+tmp+Main.P-Source.Conc.+P-Source.Feed.conc.+ Main.Thr.Conc.+Nitrogen.Conc.+total.sugar+PL+Thr+His+lactat e+PO4.apporte; 0.604, 0.804, tmp+Main.P-Source.Conc.+Main.T hr.Conc.+Nitrogen.Conc.+total.sugar+lactate+PO4.apporte; 0. 604, 0.804, pH+tmp+Main.P-Source.Conc.+Nitrogen.Conc.+Thr+H is+PO4.apporte; 0.597, 0.804, pH+tmp+Main.P-Source.Conc.+Ni trogen.Conc.+total.sugar+rab+lactate+PO4.apporte; 0.597, 0. 804, tmp+Main.P-Source.Conc.+P-Source.Feed.conc.+Nitrogen.C onc.+emission_CO2+His+lactate+PO4.apporte; 0.582, 0.804, tm p+Main.P-Source.Conc.+P-Source.Feed.conc.+Main.Thr.Conc.+Ni trogen.Conc.+rab+Thr+His+lactate+PO4.apporte; 0.558, 0.804, pH+tmp+Main.P-Source.Conc.+P-Source.Feed.conc.+Main.Thr.Co nc.+Nitrogen.Conc.+total.Vol+emission_CO2+rab+Thr+His+lacta te+PO4.apporte; 0.597, 0.804, pH+tmp+Main.P-Source.Conc.+Ni trogen.Conc.+total.sugar+Thr+His+PO4.apporte; 0.567, 0.804, tmp+Main.P-Source.Conc.+P-Source.Feed.conc.+Nitrogen.Conc. +total.sugar+PL+emission_O2+emission_CO2+Thr+His+lactate+PO 4.apporte; 0.575, 0.804, tmp+Main.P-Source.Conc.+Main.Thr.C onc.+Nitrogen.Conc.+total.Vol+total.sugar+PL+emission_O2+Th r+lactate+PO4.apporte; 0.590, 0.804, pH+tmp+Main.P-Source.C onc.+P-Source.Feed.conc.+Nitrogen.Conc.+total.sugar+His+lac tate+PO4.apporte; 0.597, 0.804, pH+tmp+Main.P-Source.Conc.+ Nitrogen.Conc.+rab+His+lactate+PO4.apporte; 0.574, 0.804, t mp+Main.P-Source.Conc.+Nitrogen.Conc.+total.Vol+total.sugar +PL+emission_O2+emission_CO2+Thr+lactate+PO4.apporte; 0.566, 0.804, pH+tmp+Main.P-Source.Conc.+P-Source.Feed.conc.+Nitr ogen.Conc.+total.sugar+PL+emission_CO2+Thr+His+lactate+PO4. apporte; 0.574, 0.804, tmp+Main.P-Source.Conc.+P-Source.Fee d.conc.+Main.Thr.Conc.+Nitrogen.Conc.+total.Vol+total.sugar +emission_CO2+Thr+lactate+PO4.apporte; 0.574, 0.804, tmp+Ma in.P-Source.Conc.+P-Source.Feed.conc.+Nitrogen.Conc.+total. Vol+total.sugar+emission_CO2+Thr+His+lactate+PO4.apporte; 0. 574, 0.804, pH+tmp+Main.P-Source.Conc.+Main.Thr.Conc.+Nitro gen.Conc.+total.sugar+emission_CO2+rab+Thr+lactate+PO4.appo rte; 0.597, 0.804, pH+tmp+Main.P-Source.Conc.+Main.Thr.Conc. +Nitrogen.Conc.+total.sugar+lactate+PO4.apporte; 0.590, 0.8 04, tmp+Main.P-Source.Conc.+P-Source.Feed.conc.+Nitrogen.Co nc.+total.sugar+emission_O2+His+lactate+PO4.apporte; 0.597, 0.804, pH+tmp+Main.P-Source.Conc.+Nitrogen.Conc.+total.sug ar+emission_CO2+lactate+PO4.apporte; 0.589, 0.804, tmp+Main. P-Source.Conc.+P-Source.Feed.conc.+Main.Thr.Conc.+Nitrogen. Conc.+total.sugar+emission_O2+lactate+PO4.apporte; 0.589, 0. 804, tmp+Main.P-Source.Conc.+P-Source.Feed.conc.+Main.Thr.C onc.+Nitrogen.Conc.+rab+His+lactate+PO4.apporte; 0.589, 0.8 04, tmp+Main.P-Source.Conc.+P-Source.Feed.conc.+Main.Thr.Co nc.+Nitrogen.Conc.+total.sugar+rab+lactate+PO4.apporte; 0.5 89, 0.804, pH+tmp+Main.P-Source.Conc.+P-Source.Feed.conc.+N itrogen.Conc.+rab+His+lactate+PO4.apporte; 0.574, 0.804, tm p+Main.P-Source.Conc.+Nitrogen.Conc.+total.Vol+total.sugar+ PL+emission_CO2+rab+Thr+lactate+PO4.apporte; 0.589, 0.804, tmp+Main.P-Source.Conc.+Main.Thr.Conc.+Nitrogen.Conc.+total. sugar+rab+Thr+lactate+PO4.apporte; 0.596, 0.804, pH+tmp+Mai n.P-Source.Conc.+Nitrogen.Conc.+emission_O2+emission_CO2+Th r+PO4.apporte; 0.574, 0.804, pH+tmp+Main.P-Source.Conc.+P-S ource.Feed.conc.+Main.Thr.Conc.+Nitrogen.Conc.+total.Vol+ra b+Thr+lactate+PO4.apporte; 0.582, 0.804, tmp+Main.P-Source. Conc.+Main.Thr.Conc.+Nitrogen.Conc.+total.sugar+PL+rab+Thr+ lactate+PO4.apporte; 0.589, 0.804, pH+tmp+Main.P-Source.Con c.+Nitrogen.Conc.+total.Vol+rab+His+lactate+PO4.apporte; 0. 596, 0.804, pH+tmp+Main.P-Source.Conc.+Nitrogen.Conc.+total. sugar+His+lactate+PO4.apporte; 0.589, 0.804, tmp+Main.P-Sou rce.Conc.+P-Source.Feed.conc.+Main.Thr.Conc.+Nitrogen.Conc. +total.sugar+emission_CO2+lactate+PO4.apporte; 0.596, 0.804, pH+tmp+Main.P-Source.Conc.+Nitrogen.Conc.+emission_O2+His+ lactate+PO4.apporte; 0.596, 0.804, pH+tmp+Main.P-Source.Con c.+Nitrogen.Conc.+total.sugar+emission_O2+lactate+PO4.appor te; 0.574, 0.804, tmp+Main.P-Source.Conc.+Nitrogen.Conc.+to tal.sugar+PL+emission_CO2+rab+Thr+His+lactate+PO4.apporte; 0.574, 0.804, tmp+Main.P-Source.Conc.+Main.Thr.Conc.+Nitrog en.Conc.+total.Vol+total.sugar+emission_O2+Thr+His+lactate+ PO4.apporte; 0.589, 0.804, pH+tmp+Main.P-Source.Conc.+Nitro gen.Conc.+total.Vol+total.sugar+His+lactate+PO4.apporte; 0. 596, 0.804, tmp+Main.P-Source.Conc.+P-Source.Feed.conc.+Nit rogen.Conc.+total.sugar+emission_O2+lactate+PO4.apporte; 0. 574, 0.804, pH+tmp+Main.P-Source.Conc.+Nitrogen.Conc.+total. Vol+PL+rab+Thr+His+lactate+PO4.apporte; 0.574, 0.804, tmp+M ain.P-Source.Conc.+Nitrogen.Conc.+total.Vol+total.sugar+emi ssion_CO2+rab+Thr+His+lactate+PO4.apporte; 0.596, 0.804, pH +tmp+Main.P-Source.Conc.+Nitrogen.Conc.+emission_CO2+His+la ctate+PO4.apporte; 0.557, 0.804, pH+tmp+Main.P-Source.Conc. +Main.Thr.Cone.+Nitrogen.Conc.+total.sugar+PL+emission_O2+e mission_CO2+Thr+His+lactate+PO4.apporte; 0.574, 0.804, pH+t mp+Main.P-Source.Conc.+Nitrogen.Conc.+total.sugar+PL+rab+Th r+His+lactate+PO4.apporte; 0.589, 0.804, tmp+Main.P-Source. Conc.+P-Source.Feed.conc.+Nitrogen.Conc.+total.sugar+emissi on_CO2+His+lactate+PO4.apporte; 0.581, 0.804, tmp+Main.P-So urce.Conc.+P-Source.Feed.conc.+Main.Thr.Conc.+Nitrogen.Conc. +total.sugar+rab+Thr+lactate+PO4.apporte; 0.596, 0.804, tmp +Main.P-Source.Conc.+P-Source.Feed.conc.+Nitrogen.Conc.+tot al.sugar+emission_CO2+lactate+PO4.apporte; 0.589, 0.804, pH +tmp+Main.P-Source.Conc.+P-Source.Feed.conc.+Nitrogen.Conc. +emission_O2+His+lactate+PO4.apporte; 0.557, 0.804, pH+tmp+ Main.P-Source.Conc.+Nitrogen.Conc.+total.Vol+total.sugar+PL +emission_O2+emission_CO2+Thr+His+lactate+PO4.apporte; 0.55 7, 0.803, pH+tmp+Main.P-Source.Conc.+Main.Thr.Conc.+Nitroge n.Conc.+total.Vol+total.sugar+PL+emission_CO2+rab+Thr+lacta te+PO4.apporte; 0.589, 0.803, pH+tmp+Main.P-Source.Conc.+Ni trogen.Conc.+total.Vol+emission_CO2+His+lactate+PO4.apport e; 0.565, 0.803, tmp+Main.P-Source.Conc.+P-Source.Feed.conc. +Nitrogen.Conc.+total.Vol+total.sugar+emission_O2+emission CO2+Thr+His+lactate+PO4.apporte; 0.565, 0.803, tmp+Main.P-S ource.Conc.+P-Source.Feed.conc.+Main.Thr.Conc.+Nitrogen.Con c.+total.Vol+total.sugar+emission_O2+emission_CO2+Thr+lacta te+PO4.apporte; 0.557, 0.803, pH+tmp+Main.P-Source.Conc.+P-Source.Feed.conc.+Nitrogen.Conc.+total.Vol+total.sugar+PL+e mission_O2+emission_CO2+Thr+lactate+PO4.apporte; 0.588, 0.8 03, pH+tmp+Main.P-Source.Conc.+P-Source.Feed.conc.+Nitrogen. Conc.+emission_CO2+His+lactate+PO4.apporte; 0.565, 0.803, p H+tmp+Main.P-Source.Conc.+Main.Thr.Conc.+Nitrogen.Conc.+tot al.sugar+PL+emission_CO2+rab+Thr+lactate+PO4.apporte; 0.588, 0.803, pH+tmp+Main.P-Source.Conc.+P-Source.Feed.conc.+Nitr ogen.Conc.+total.sugar+rab+lactate+PO4.apporte; 0.603, 0.80 3, tmp+Main.P-Source.Conc.+Main.Thr.Conc.+Nitrogen.Conc.+to tal.sugar+Thr+PO4.apporte; 0.581, 0.803, tmp+Main.P-Source. Conc.+P-Source.Feed.conc.+Main.Thr.Conc.+Nitrogen.Conc.+tot al.sugar+rab+His+lactate+PO4.apporte; 0.581, 0.803, pH+tmp+ Main.P-Source.Conc.+Nitrogen.Conc.+total.sugar+rab+Thr+His+ lactate+PO4.apporte; 0.557, 0.803, pH+tmp+Main.P-Source.Con c.+P-Source.Feed.conc.+Nitrogen.Conc.+total.sugar+PL+emissi on_O2+emission_CO2+Thr+His+lactate+PO4.apporte; 0.573, 0.80 3, pH+tmp+Main.P-Source.Conc.+Nitrogen.Conc.+total.Vol+tota l.sugar+PL+rab+Thr+lactate+PO4.apporte; 0.573, 0.803, tmp+M ain.P-Source.Conc.+P-Source.Feed.conc.+Nitrogen.Conc.+total. Vol+total.sugar+PL+Thr+His+lactate+PO4.apporte; 0.581, 0.80 3, tmp+Main.P-Source.Conc.+P-Source.Feed.conc.+Main.Thr.Con c.+Nitrogen.Conc.+total.sugar+emission_O2+emission_CO2+lact ate+PO4.apporte; 0.588, 0.803, tmp+Main.P-Source.Conc.+P-So urce.Feed.conc.+Nitrogen.Conc.+emission_O2+emission_CO2+His +lactate+PO4.apporte; 0.565, 0.803, tmp+Main.P-Source.Conc. +P-Source.Feed.conc.+Nitrogen.Conc.+total.Vol+PL+emission_C O2+rab+Thr+His+lactate+PO4.apporte; 0.565, 0.803, pH+tmp+Ma in.P-Source.Conc.+P-Source.Feed.conc.+Main.Thr.Conc.+Nitrog en.Conc.+total.sugar+emission_CO2+rab+Thr+lactate+PO4.appor te; 0.565, 0.803, pH+tmp+Main.P-Source.Conc.+P-Source.Feed. conc.+Nitrogen.Conc.+total.Vol+total.sugar+PL+Thr+His+lacta te+PO4.apporte; 0.588, 0.803, pH+tmp+Main.P-Source.Conc.+Ni trogen.Conc.+total.sugar+emission_O2+emission_CO2+Thr+PO4.a pporte; 0.573, 0.803, tmp+Main.P-Source.Conc.+Main.Thr.Conc. +Nitrogen.Conc.+total.Vol+PL+emission_CO2+Thr+His+lactate+P O4.apporte; 0.588, 0.803, pH+tmp+Main.P-Source.Conc.+Main.T hr.Conc.+Nitrogen.Conc.+total.Vol+His+lactate+PO4.apporte; 0.588, 0.803, pH+tmp+Main.P-Source.Conc.+P-Source.Feed.conc. +Nitrogen.Conc.+total.sugar+emission_CO2+lactate+PO4.apport e; 0.580, 0.803, pH+tmp+Main.P-Source.Conc.+Main.Thr.Conc.+ Nitrogen.Conc.+total.Vol+total.sugar+His+lactate+PO4.apport e; 0.595, 0.803, pH+tmp+Main.P-Source.Conc.+Main.Thr.Conc.+ Nitrogen.Conc.+His+lactate+PO4.apporte; 0.565, 0.803, tmp+M ain.P-Source.Conc.+P-Source.Feed.conc.+Main.Thr.Conc.+Nitro gen.Conc.+total.sugar+PL+emission_CO2+rab+Thr+lactate+PO4.a pporte; 0.588, 0.803, tmp+Main.P-Source.Conc.+P-Source.Feed. conc.+Nitrogen.Conc.+total.sugar+emission_O2+emission_CO2+l actate+PO4.apporte; 0.602, 0.803, pH+tmp+Main.P-Source.Conc. +Nitrogen.Conc.+rab+Thr+PO4.apporte; 0.580, 0.803, tmp+Main. P-Source.Conc.+P-Source.Feed.conc.+Nitrogen.Conc.+total.sug ar+rab+Thr+His+lactate+PO4.apporte; 0.588, 0.803, pH+tmp+Ma in.P-Source.Conc.+P-Source.Feed.conc.+Nitrogen.Conc.+total. sugar+emission_O2+lactate+PO4.apporte; 0.602, 0.803, tmp+Ma in.P-Source.Conc.+P-Source.Feed.conc.+Nitrogen.Conc.+total. sugar+Thr+PO4.apporte; 0.595, 0.803, tmp+Main.P-Source.Conc. +Main.Thr.Conc.+Nitrogen.Conc.+total.Vol+total.sugar+lact at e+PO4.apporte; 0.580, 0.803, tmp+Main.P-Source.Conc.+P-Sour ce.Feed.conc.+Nitrogen.Conc.+total.sugar+emission_O2+emissi on_CO2+His+lactate+PO4.apporte; 0.572, 0.803, pH+tmp+Main.P -Source.Conc.+Nitrogen.Conc.+total.sugar+emission_CO2+rab+T hr+His+lactate+PO4.apporte; 0.587, 0.803, pH+tmp+Main.P-Sou rce.Conc.+P-Source.Feed.conc.+Main.Thr.Conc.+Nitrogen.Conc. +total.sugar+Thr+PO4.apporte; 0.564, 0.803, tmp+Main.P-Sour ce.Conc.+P-Source.Feed.conc.+Main.Thr.Conc.+Nitrogen.Conc.+ total.sugar+PL+emission_O2+Thr+His+lactate+PO4.apporte; 0.5 80, 0.803, tmp+Main.P-Source.Conc.+P-Source.Feed.conc.+Main. Thr.Conc.+Nitrogen.Conc.+total.sugar+emission_O2+His+lactat e+PO4.apporte; 0.587, 0.803, pH+tmp+Main.P-Source.Conc.+P-S ource.Feed.conc.+Main.Thr.Conc.+Nitrogen.Conc.+total.sugar+ lactate+PO4.apporte; 0.587, 0.803, pH+tmp+Main.P-Source.Con c.+P-Source.Feed.conc.+Nitrogen.Conc.+total.Vol+total.sugar +lactate+PO4.apporte; 0.572, 0.803, tmp+Main.P-Source.Conc. +P-Source.Feed.conc.+Nitrogen.Conc.+total.Vol+total.sugar+P L+emission_CO2+Thr+lactate+PO4.apporte; 0.580, 0.803, pH+tm p+Main.P-Source.Conc.+Nitrogen.Conc.+total.Vol+emission_O2+ emission_CO2+His+lactate+PO4.apporte; 0.572, 0.803, tmp+Mai n.P-Source.Conc.+Nitrogen.Conc.+total.Vol+total.sugar+PL+em ission_O2+Thr+His+lactate+PO4.apporte; 0.580, 0.803, pH+tmp +Main.P-Source.Conc.+Nitrogen.Conc.+total.Vol+total.sugar+r ab+Thr+lactate+PO4.apporte; 0.580, 0.803, pH+tmp+Main.P-Sou rce.Conc.+Nitrogen.Conc.+total.Vol+total.sugar+emission_CO2 +His+lactate+PO4.apporte; 0.587, 0.803, pH+tmp+Main.P-Sourc e.Conc.+Main.Thr.Conc.+Nitrogen.Conc.+total.sugar+emission_ O2+Thr+PO4.apporte; 0.587, 0.803, tmp+Main.P-Source.Conc.+M ain.Thr.Conc.+Nitrogen.Conc.+rab+Thr+His+lactate+PO4.apport e; 0.595, 0.803, tmp+Main.P-Source.Conc.+Main.Thr.Conc.+Nit rogen.Conc.+total.sugar+rab+lactate+P04.apporte; 0.587, 0.8 03, pH+tmp+Main.P-Source.Conc.+Nitrogen.Conc.+total.Vol+emi ssion_O2+His+lactate+PO4.apporte; 0.580, 0.803, tmp+Main.P-Source.Conc.+Main.Thr.Conc.+Nitrogen.Conc.+PL+rab+Thr+His+l actate+PO4.apporte; 0.564, 0.803, pH+tmp+Main.P-Source.Conc. +P-Source.Feed.conc.+Main.Thr.Conc.+Nitrogen.Conc.+total.su gar+PL+rab+Thr+lactate+PO4.apporte; 0.572, 0.803, pH+tmp+Ma in.P-Source.Conc.+P-Source.Feed.conc.+Nitrogen.Conc.+total. sugar+rab+Thr+His+lactate+PO4.apporte; 0.580, 0.803, pH+tmp +Main.P-Source.Conc.+P-Source.Feed.conc.+Main.Thr.Conc.+Nit rogen.Conc.+total.Vol+total.sugar+lactate+PO4.apporte; 0.58 0, 0.803, pH+tmp+Main.P-Source.Conc.+P-Source.Feed.conc.+Ni trogen.Conc.+emission_O2+emission_CO2+His+lactate+PO4.appor te; 0.580, 0.803, pH+tmp+Main.P-Source.Conc.+P-Source.Feed. conc.+Nitrogen.Conc.+total.sugar+emission_O2+emission_CO2+l actate+PO4.apporte; 0.587, 0.803, tmp+Main.P-Source.Conc.+P -Source.Feed.conc.+Main.Thr.Conc.+Nitrogen.Conc.+emission_O 2+His+lactate+PO4.apporte; 0.587, 0.803, pH+tmp+Main.P-Sour ce.Conc.+P-Source.Feed.conc.+Main.Thr.Conc.+Nitrogen.Conc.+ His+lactate+PO4.apporte; 0.564, 0.803, tmp+Main.P-Source.Co nc.+Main.Thr.Conc.+Nitrogen.Conc.+total.Vol+PL+emission_CO2 +rab+Thr+His+lactate+PO4.apporte; 0.587, 0.803, pH+tmp+Main. P-Source.Conc.+Nitrogen.Conc.+total.sugar+emission_O2+emiss ion_CO2+lactate+PO4.apporte; 0.563, 0.803, pH+tmp+Main.P-So urce.Conc.+P-Source.Feed.conc.+Nitrogen.Conc.+total.sugar+e mission_CO2+rab+Thr+His+lactate+PO4.apporte; 0.587, 0.803, pH+tmp+Main.P-Source.Conc.+P-Source.Feed.conc.+Nitrogen.Con c.+total.Vol+His+lactate+PO4.apporte; 0.587, 0.802, pH+tmp+ Main.P-Source.Conc.+Main.Thr.Conc.+Nitrogen.Conc.+total.sug ar+emission_CO2+lactate+PO4.apporte; 0.555, 0.802, pH+tmp+M ain.P-Source.Conc.+P-Source.Feed.conc.+Main.Thr.Conc.+Nitro gen.Conc.+total.Vol+PL+emission_O2+Thr+His+lactate+PO4.appo rte; 0.587, 0.802, pH+tmp+Main.P-Source.Conc.+Nitrogen.Conc. +emission_O2+emission_CO2+His+lactate+PO4.apporte; 0.579, 0. 802, pH+tmp+Main.P-Source.Conc.+Nitrogen.Conc.+total.Vol+ra b+Thr+His+lactate+PO4.apporte; 0.555, 0.802, pH+tmp+Main.P-Source.Conc.+P-Source.Feed.conc.+Main.Thr.Conc.+Nitrogen.Co nc.+total.Vol+total.sugar+emission CO2+rab+Thr+lactate+PO4. apporte; 0.601, 0.802, tmp+Main.P-Source.Conc.+Main.Thr.Con c.+Nitrogen.Conc.+His+lactate+PO4.apporte; 0.579, 0.802, tm p+Main.P-Source.Conc.+P-Source.Feed.conc.+Main.Thr.Conc.+Ni trogen.Conc.+total.sugar+emission_CO2+His+lactate+PO4.appor te; 0.601, 0.802, pH+tmp+Main.P-Source.Conc.+Nitrogen.Conc. +emission_O2+Thr+PO4.apporte; 0.579, 0.802, pH+tmp+Main.P-S ource.Conc.+P-Source.Feed.conc.+Nitrogen.Conc.+total.sugar+ emission_O2+His+lactate+PO4.apporte; 0.563, 0.802, pH+tmp+M ain.P-Source.Conc.+P-Source.Feed.conc.+Main.Thr.Conc.+Nitro gen.Conc.+total.Vol+PL+rab+Thr+lactate+PO4.apporte; 0.555, 0.802, pH+tmp+Main.P-Source.Conc.+P-Source.Feed.conc.+Main. Thr.Conc.+Nitrogen.Conc.+total.Vol+PL+emission_CO2+Thr+His+ lactate+PO4.apporte; 0.586, 0.802, pH+tmp+Main.P-Source.Con c.+Main.Thr.Conc.+Nitrogen.Conc.+total.sugar+emission_O2+la ctate+PO4.apporte; 0.563, 0.802, tmp+Main.P-Source.Conc.+P-Source.Feed.conc.+Main.Thr.Conc.+Nitrogen.Conc.+total.Vol+P L+emission_O2+Thr+His+lactate+PO4.apporte; 0.571, 0.802, pH +tmp+Main.P-Source.Conc.+Nitrogen.Conc.+total.Vol+total.sug ar+emission_O2+emission_CO2+His+lactate+PO4.apporte; 0.586, 0.802, tmp+Main.P-Source.Conc.+P-Source.Feed.conc.+Main.Th r.Conc.+Nitrogen.Conc.+total.Vol+total.sugar+lactate+PO4.ap porte; 0.579, 0.802, pH+tmp+Main.P-Source.Conc.+P-Source.Fe ed.conc.+Nitrogen.Conc.+total.sugar+emission_CO2+His+lactat e+PO4.apporte; 0.601, 0.802, tmp+Main.P-Source.Conc.+Nitrog en.Conc.+total.sugar+rab+lactate+PO4.apporte; 0.579, 0.802, pH+tmp+Main.P-Source.Conc.+Nitrogen.Conc.+total.Vol+total. sugar+rab+His+lactate+PO4.apporte; 0.563, 0.802, tmp+Main.P -Source.Conc.+Main.Thr.Conc.+Nitrogen.Conc.+total.Vol+PL+em ission_O2+emission_CO2+Thr+His+lactate+PO4.apporte; 0.579, 0.802, pH+tmp+Main.P-Source.Conc.+P-Source.Feed.conc.+Nitro gen.Conc.+total.sugar+rab+His+lactate+PO4.apporte; 0.586, 0. 802, pH+tmp+Main.P-Source.Conc.+Main.Thr.Conc.+Nitrogen.Con c.+total.sugar+rab+lactate+PO4.apporte; 0.586, 0.802, pH+tm p+Main.P-Source.Conc.+Nitrogen.Conc.+total.sugar+rab+His+la ctate+PO4.apporte; 0.571, 0.802, tmp+Main.P-Source.Conc.+Ma in.Thr.Conc.+Nitrogen.Conc.+total.sugar+PL+emission_CO2+Thr +His+lactate+PO4.apporte; 0.586, 0.802, pH+tmp+Main.P-Sourc e.Conc.+Nitrogen.Conc.+total.sugar+emission_CO2+His+lactate +PO4.apporte; 0.586, 0.802, pH+tmp+Main.P-Source.Conc.+Nitr ogen.Conc.+emission_O2+emission_CO2+Thr+His+PO4.apporte; 0. 586, 0.802, pH+tmp+Main.P-Source.Conc.+Nitrogen.Conc.+total. sugar+emission_O2+His+lactate+PO4.apporte; 0.607, 0.802, tm p+Main.P-Source.Conc.+Nitrogen.Conc.+His+lactate+PO4.apport e; 0.571, 0.802, pH+tmp+Main.P-Source.Conc.+Main.Thr.Conc.+ Nitrogen.Cone.+total.Vol+total.sugar+emission_CO2+His+lacta te+PO4.apporte; 0.586, 0.802, tmp+Main.P-Source.Conc.+P-Sou rce.Feed.conc.+Main.Thr.Conc.+Nitrogen.Conc.+emission_CO2+H is+lactate+PO4.apporte; 0.600, 0.802, tmp+Main.P-Source.Con c.+Nitrogen.Conc.+total.sugar+emission_O2+Thr+PO4.apporte; 0.571, 0.802, pH+tmp+Main.P-Source.Conc.+P-Source.Feed.conc. +Main.Thr.Conc.+Nitrogen.Conc.+total.Vol+total.sugar+emissi on_CO2+lactate+PO4.apporte; 0.562, 0.802, tmp+Main.P-Source. Conc.+P-Source.Feed.conc.+Main.Thr.Conc.+Nitrogen.Conc.+tot al.sugar+emission_CO2+rab+Thr+His+lactate+PO4.apporte; 0.57 1, 0.802, tmp+Main.P-Source.Conc.+Main.Thr.Conc.+Nitrogen.C onc.+total.Vol+total.sugar+PL+Thr+His+lactate+PO4.apporte; 0.593, 0.802, tmp+Main.P-Source.Conc.+Main.Thr.Conc.+Nitrog en.Conc.+total.sugar+emission_O2+lactate+PO4.apporte; 0.578, 0.802, pH+tmp+Main.P-Source.Conc.+P-Source.Feed.conc.+Nitr ogen.Conc.+total.Vol+total.sugar+emission_CO2+lactate+PO4.a pporte; 0.562, 0.802, tmp+Main.P-Source.Conc.+P-Source.Feed. conc.+Nitrogen.Conc.+total.sugar+PL+emission_CO2+rab+Thr+Hi s+lactate+PO4.apporte; 0.593, 0.802, tmp+Main.P-Source.Conc. +Main.Thr.Conc.+Nitrogen.Conc.+total.sugar+His+lactate+PO4. apporte; 0.562, 0.802, tmp+Main.P-Source.Conc.+P-Source.Fee d.conc.+Main.Thr.Conc.+Nitrogen.Conc.+total.Vol+total.sugar +emission_CO2+rab+Thr+lactate+PO4.apporte; 0.578, 0.802, pH +tmp+Main.P-Source.Conc.+Nitrogen.Conc.+total.Vol+total.sug ar+emission_O2+His+lactate+PO4.apporte; 0.554, 0.802, pH+tm p+Main.P-Source.Conc.+P-Source.Feed.conc.+Main.Thr.Conc.+Ni trogen.Conc.+total.Vol+total.sugar+PL+emission_O2+Thr+lacta te+PO4.apporte; 0.578, 0.802, pH+tmp+Main.P-Source.Conc.+P-Source.Feed.conc.+Nitrogen.Conc.+total.Vol+rab+His+lactate+ PO4.apporte; 0.570, 0.802, tmp+Main.P-Source.Conc.+P-Source. Feed.conc.+Main.Thr.Conc.+Nitrogen.Conc.+PL+rab+Thr+His+lac tate+PO4.apporte; 0.562, 0.802, pH+tmp+Main.P-Source.Conc.+ Nitrogen.Conc.+total.sugar+PL+emission_CO2+rab+Thr+His+lact ate+PO4.apporte; 0.570, 0.802, tmp+Main.P-Source.Conc.+Main. Thr.Conc.+Nitrogen.Conc.+total.sugar+emission_CO2+rab+Thr+H is+lactate+PO4.apporte; 0.586, 0.802, pH+tmp+Main.P-Source. Conc.+Main.Thr.Conc.+Nitrogen.Conc.+rab+His+lactate+PO4.app orte; 0.554, 0.802, pH+tmp+Main.P-Source.Conc.+P-Source.Fee d.conc.+Nitrogen.Conc.+total.Vol+total.sugar+PL+emission_CO 2+rab+Thr+lactate+PO4.apporte; 0.562, 0.802, pH+tmp+Main.P-Source.Conc.+P-Source.Feed.conc.+Main.Thr.Conc.+Nitrogen.Co nc.+PL+rab+Thr+His+lactate+PO4.apporte; 0.570, 0.802, tmp+M ain.P-Source.Conc.+P-Source.Feed.conc.+Main.Thr.Conc.+Nitro gen.Conc.+total.sugar+emission_O2+emission_CO2+His+lactate+ PO4.apporte; 0.554, 0.802, pH+tmp+Main.P-Source.Conc.+P-Sou rce.Feed.conc.+Main.Thr.Conc.+Nitrogen.Conc.+total.Vol+tota l.sugar+emission_O2+Thr+His+lactate+PO4.apporte; 0.606, 0.8 02, tmp+Main.P-Source.Conc.+Nitrogen.Conc.+total.sugar+lact ate+PO4.apporte; 0.570, 0.802, pH+tmp+Main.P-Source.Conc.+P -Source.Feed.conc.+Nitrogen.Conc.+total.sugar+emission_O2+e mission_CO2+His+lactate+PO4.apporte; 0.553, 0.802, pH+tmp+M ain.P-Source.Conc.+Main.Thr.Conc.+Nitrogen.Conc.+total.Vol+ total.sugar+PL+emission_O2+Thr+His+lactate+PO4.apporte; 0.5 62, 0.802, pH+tmp+Main.P-Source.Conc.+Main.Thr.Conc.+Nitrog en.Conc.+total.Vol+total.sugar+emission_O2+emission_CO2+His +lactate+PO4.apporte; 0.592, 0.802, tmp+Main.P-Source.Conc. +Main.Thr.Conc.+Nitrogen.Conc.+rab+His+lactate+PO4.apporte; 0.599, 0.802, tmp+Main.P-Source.Conc.+Nitrogen.Conc.+rab+H is+lactate+PO4.apporte; 0.585, 0.802, tmp+Main.P-Source.Con c.+Main.Thr.Conc.+Nitrogen.Conc.+total.Vol+rab+Thr+lactate+ PO4.apporte; 0.562, 0.802, tmp+Main.P-Source.Conc.+P-Source. Feed.conc.+Main.Thr.Conc.+Nitrogen.Conc.+total.Vol+total.su gar+emission_O2+Thr+His+lactate+PO4.apporte; 0.585, 0.802, pH+tmp+Main.P-Source.Conc.+Main.Thr.Conc.+Nitrogen.Conc.+to tal.sugar+Thr+His+PO4.apporte; 0.599, 0.802, tmp+Main.P-Sou rce.Conc.+Nitrogen.Conc.+total.sugar+Thr+His+PO4.apporte; 0. 562, 0.802, tmp+Main.P-Source.Conc.+P-Source.Feed.conc.+Nit rogen.Conc.+total.Vol+total.sugar+PL+emission_O2+emission_C O2+Thr+lactate+PO4.apporte; 0.577, 0.802, pH+tmp+Main.P-Sou rce.Conc.+P-Source.Feed.conc.+Main.Thr.Conc.+Nitrogen.Conc. +total.sugar+emission_CO2+lactate+PO4.apporte; 0.577, 0.802, tmp+Main.P-Source.Conc.+Main.Thr.Conc.+Nitrogen.Conc.+tota l.Vol+PL+rab+Thr+lactate+PO4.apporte; 0.577, 0.802, pH+tmp+ Main.P-Source.Conc.+Main.Thr.Conc.+Nitrogen.Conc.+total.Vol +emission_CO2+His+lactate+PO4.apporte; 0.585, 0.802, pH+tmp +Main.P-Source.Conc.+Main.Thr.Conc.+Nitrogen.Conc.+total.su gar+His+lactate+PO4.apporte; 0.561, 0.801, pH+tmp+Main.P-So urce.Conc.+P-Source.Feed.conc.+Main.Thr.Conc.+Nitrogen.Conc. +total.Vol+total.sugar+emission_O2+emission_CO2+lactate+PO4. apporte; 0.577, 0.801, pH+tmp+Main.P-Source.Conc.+P-Source. Feed.conc.+Main.Thr.Conc.+Nitrogen.Conc.+rab+His+lactate+PO 4.apporte; 0.577, 0.801, pH+tmp+Main.P-Source.Conc.+P-Sourc e.Feed.conc.+Nitrogen.Conc.+total.Vol+total.sugar+rab+lacta te+PO4.apporte; 0.592, 0.801, pH+tmp+Main.P-Source.Conc.+Ni trogen.Conc.+total.sugar+PL+Thr+PO4.apporte; 0.553, 0.801, pH+tmp+Main.P-Source.Conc.+Nitrogen.Conc.+total.Vol+total.s ugar+PL+emission_CO2+rab+Thr+His+lactate+PO4.apporte; 0.577, 0.801, pH+tmp+Main.P-Source.Conc.+P-Source.Feed.conc.+Main. Thr.Conc.+Nitrogen.Conc.+total.sugar+His+lactate+PO4.apport e; 0.553, 0.801, pH+tmp+Main.P-Source.Conc.+P-Source.Feed.c onc.+Nitrogen.Conc.+total.Vol+total.sugar+emission_CO2+rab+ Thr+His+lactate+PO4.apporte; 0.569, 0.801, tmp+Main.P-Sourc e.Conc.+P-Source.Feed.conc.+Main.Thr.Conc.+Nitrogen.Conc.+t otal.sugar+PL+rab+Thr+lactate+PO4.apporte; 0.599, 0.801, pH +tmp+Main.P-Source.Conc.+P-Source.Feed.conc.+Nitrogen.Conc. +Thr+PO4.apporte; 0.553, 0.801, pH+tmp+Main.P-Source.Conc.+ P-Source.Feed.conc.+Main.Thr.Conc.+Nitrogen.Conc.+total.sug ar+PL+emission_O2+Thr+His+lactate+PO4.apporte; 0.553, 0.801, pH+tmp+Main.P-Source.Conc.+Main.Thr.Conc.+Nitrogen.Conc.+t otal.Vol+total.sugar+emission_CO2+rab+Thr+His+lactate+PO4.a pporte; 0.592, 0.801, pH+tmp+Main.P-Source.Conc.+Nitrogen.C onc.+emission_O2+Thr+His+PO4.apporte; 0.569, 0.801, pH+tmp+ Main.P-Source.Conc.+P-Source.Feed.conc.+Nitrogen.Conc.+tota l.Vol+total.sugar+emission_O2+emission_CO2+lactate+PO4.appo rte; 0.561, 0.801, tmp+Main.P-Source.Conc.+P-Source.Feed.co nc.+Nitrogen.Conc.+total.Vol+total.sugar+emission_CO2+rab+T hr+His+lactate+PO4.apporte; 0.577, 0.801, tmp+Main.P-Source. Conc.+Nitrogen.Conc.+total.sugar+PL+rab+Thr+His+lactate+PO4. apporte; 0.577, 0.801, tmp+Main.P-Source.Conc.+P-Source.Fee d.conc.+Main.Thr.Conc.+Nitrogen.Conc.+emission_O2+emission CO2+His+lactate+PO4.apporte; 0.569, 0.801, tmp+Main.P-Sourc e.Conc.+Main.Thr.Conc.+Nitrogen.Conc.+total.Vol+total.sugar +PL+emission_CO2+Thr+lactate+PO4.apporte; 0.584, 0.801, tmp +Main.P-Source.Conc.+Nitrogen.Conc.+total.sugar+rab+Thr+His +lactate+PO4.apporte; 0.577, 0.801, pH+tmp+Main.P-Source.Co nc.+P-Source.Feed.conc.+Nitrogen.Conc.+total.Vol+total.suga r+His+lactate+PO4.apporte; 0.577, 0.801, pH+tmp+Main.P-Sour ce.Conc.+P-Source.Feed.conc.+Main.Thr.Conc.+Nitrogen.Conc.+ total.sugar+emission_O2+lactate+PO4.apporte; 0.561, 0.801, tmp+Main.P-Source.Conc.+Main.Thr.Conc.+Nitrogen.Conc.+total. sugar+PL+emission_O2+emission_CO2+Thr+His+lactate+PO4.appor te; 0.584, 0.801, pH+tmp+Main.P-Source.Conc.+P-Source.Feed. conc.+Nitrogen.Conc.+emission_O2+emission_CO2+Thr+PO4.appor te; 0.592, 0.801, tmp+Main.P-Source.Conc.+Main.Thr.Conc.+Ni trogen.Conc.+total.sugar+emission_CO2+lactate+PO4.apporte; 0.577, 0.801, pH+tmp+Main.P-Source.Conc.+P-Source.Feed.conc. +Main.Thr.Conc.+Nitrogen.Conc.+total.sugar+rab+lactate+PO4. apporte; 0.592, 0.801, pH+tmp+Main.P-Source.Conc.+Nitrogen. Conc.+total.sugar+rab+Thr+PO4.apporte; 0.577, 0.801, tmp+Ma in.P-Source.Conc.+P-Source.Feed.conc.+Main.Thr.Conc.+Nitrog en.Conc.+total.Vol+rab+Thr+lactate+PO4.apporte; 0.552, 0.80 1, pH+tmp+Main.P-Source.Conc.+P-Source.Feed.conc.+Main.Thr. Conc.+Nitrogen.Conc.+PL+emission_CO2+rab+Thr+His+lactate+PO 4.apporte; 0.599, 0.801, pH+tmp+Main.P-Source.Conc.+Nitroge n.Conc.+emission_CO2+Thr+PO4.apporte; 0.569, 0.801, pH+tmp+ Main.P-Source.Conc.+Main.Thr.Conc.+Nitrogen.Conc.+total.Vol +total.sugar+emission_O2+His+lactate+PO4.apporte; 0.577, 0. 801, pH+tmp+Main.P-Source.Conc.+Main.Thr.Conc.+Nitrogen.Con c.+total.sugar+emission_O2+emission_CO2+lactate+PO4.apport e; 0.552, 0.801, pH+tmp+Main.P-Source.Conc.+P-Source.Feed.c onc.+Main.Thr.Conc.+Nitrogen.Conc.+total.Vol+total.sugar+PL +emission_CO2+Thr+lactate+PO4.apporte; 0.591, 0.801, pH+tmp +Main.P-Source.Conc.+Nitrogen.Conc.+total.sugar+emission_CO 2+Thr+PO4.apporte; 0.569, 0.801, pH+tmp+Main.P-Source.Conc. +P-Source.Feed.conc.+Main.Thr.Conc.+Nitrogen.Conc.+total.su gar+emission_O2+emission_CO2+lactate+PO4.apporte; 0.612, 0. 801, pH+tmp+Main.P-Source.Conc.+Nitrogen.Conc.+PO4.apporte; 0.598, 0.801, pH+tmp+Main.P-Source.Conc.+Main.Thr.Conc.+Ni trogen.Conc.+Thr+PO4.apporte; 0.605, 0.801, tmp+Main.P-Sour ce.Conc.+Nitrogen.Conc.+rab+Thr+PO4.apporte; 0.584, 0.801, pH+tmp+Main.P-Source.Conc.+Main.Thr.Conc.+Nitrogen.Conc.+em ission_O2+His+lactate+PO4.apporte; 0.560, 0.801, tmp+Main.P -Source.Conc.+P-Source.Feed.conc.+Main.Thr.Conc.+Nitrogen.C onc.+total.Vol+PL+emission_CO2+Thr+His+lactate+PO4.apporte; 0.569, 0.801, pH+tmp+Main.P-Source.Conc.+Main.Thr.Conc.+Ni trogen.Conc.+total.Vol+emission_O2+emission_CO2+His+lactate +PO4.apporte; 0.576, 0.801, pH+tmp+Main.P-Source.Conc.+Main. Thr.Conc.+Nitrogen.Conc.+total.Vol+total.sugar+PL+lactate+P O4.apporte; 0.543, 0.801, pH+tmp+Main.P-Source.Conc.+P-Sour ce.Feed.conc.+Main.Thr.Conc.+Nitrogen.Conc.+total.Vol+PL+em ission_O2+emission_CO2+Thr+His+lactate+PO4.apporte; 0.591, 0.801, tmp+Main.P-Source.Conc.+P-Source.Feed.conc.+Nitrogen. Conc.+total.Vol+His+lactate+PO4.apporte; 0.568, 0.801, pH+t mp+Main.P-Source.Conc.+P-Source.Feed.conc.+Nitrogen.Conc.+t otal.Vol+rab+Thr+His+lactate+PO4.apporte; 0.605, 0.801, tmp +Main.P-Source.Conc.+Nitrogen.Conc.+Thr+His+PO4.apporte; 0. 576, 0.801, pH+tmp+Main.P-Source.Conc.+Main.Thr.Conc.+Nitro gen.Conc.+total.sugar+emission_O2+emission_CO2+Thr+PO4.appo rte; 0.611, 0.801, tmp+Main.P-Source.Conc.+Nitrogen.Conc.+T hr+PO4.apporte; 0.560, 0.801, tmp+Main.P-Source.Conc.+P-Sou rce.Feed.conc.+Main.Thr.Conc.+Nitrogen.Conc.+total.Vol+tota l.sugar+PL+emission_O2+Thr+lactate+PO4.apporte; 0.576, 0.80 1, pH+tmp+Main.P-Source.Conc.+Nitrogen.Conc.+total.sugar+em ission_O2+emission_CO2+His+lactate+PO4.apporte; 0.568, 0.80 1, pH+tmp+Main.P-Source.Conc.+Main.Thr.Conc.+Nitrogen.Conc. +total.sugar+rab+Thr+His+lactate+P04.apporte; 0.584, 0.801, pH+tmp+Main.P-Source.Conc.+Main.Thr.Conc.+Nitrogen.Conc.+e mission_CO2+His+lactate+PO4.apporte; 0.598, 0.801, pH+tmp+M ain.P-Source.Conc.+Nitrogen.Conc.+emission_O2+emission_CO2+ PO4.apporte; 0.584, 0.801, pH+tmp+Main.P-Source.Conc.+Main. Thr.Conc.+Nitrogen.Conc.+total.sugar+rab+Thr+PO4.apporte; 0. 576, 0.801, pH+tmp+Main.P-Source.Conc.+Main.Thr.Conc.+Nitro gen.Conc.+total.Vol+emission_O2+His+lactate+PO4.apporte; 0. 598, 0.801, tmp+Main.P-Source.Conc.+Nitrogen.Conc.+total.su gar+His+lactate+PO4.apporte; 0.583, 0.801, tmp+Main.P-Sourc e.Conc.+Main.Thr.Conc.+Nitrogen.Conc.+total.sugar+rab+His+l actate+PO4.apporte; 0.576, 0.801, pH+tmp+Main.P-Source.Conc. +P-Source.Feed.conc.+Nitrogen.Conc.+total.Vol+emission_CO2+ His+lactate+PO4.apporte; 0.576, 0.801, pH+tmp+Main.P-Source. Conc.+P-Source.Feed.conc.+Nitrogen.Conc.+total.Vol+total.su gar+emission_O2+lactate+PO4.apporte; 0.551, 0.801, pH+tmp+M ain.P-Source.Conc.+P-Source.Feed.conc.+Main.Thr.Conc.+Nitro gen.Conc.+total.Vol+total.sugar+emission_CO2+Thr+His+lactat e+PO4.apporte; 0.568, 0.801, tmp+Main.P-Source.Conc.+Main.T hr.Conc.+Nitrogen.Conc.+total.Vol+total.sugar+emission_CO2+ Thr+His+lactate+PO4.apporte; 0.576, 0.801, tmp+Main.P-Sourc e.Conc.+P-Source.Feed.conc.+Nitrogen.Conc.+total.Vol+rab+Th r+His+lactate+PO4.apporte; 0.576, 0.801, tmp+Main.P-Source. Conc.+P-Source.Feed.conc.+Main.Thr.Conc.+Nitrogen.Conc.+tot al.Vol+total.sugar+emission_CO2+lactate+PO4.apporte; 0.591, 0.801, tmp+Main.P-Source.Conc.+P-Source.Feed.conc.+Nitroge n.Conc.+PL+His+lactate+PO4.apporte; 0.568, 0.801, tmp+Main. P-Source.Conc.+P-Source.Feed.conc.+Nitrogen.Conc.+total.sug ar+PL+rab+Thr+His+lactate+PO4.apporte; 0.590, 0.801, pH+tmp +Main.P-Source.Conc.+Nitrogen.Conc.+total.Vol+total.sugar+T hr+PO4.apporte; 0.590, 0.801, tmp+Main.P-Source.Conc.+Nitro gen.Conc.+total.sugar+rab+His+lactate+PO4.apporte; 0.568, 0. 800, pH+tmp+Main.P-Source.Conc.+P-Source.Feed.conc.+Nitroge n.Conc.+total.Vol+total.sugar+rab+Thr+lactate+PO4.apporte; 0.551, 0.800, pH+tmp+Main.P-Source.Conc.+Main.Thr.Conc.+Nit rogen.Conc.+total.Vol+total.sugar+PL+emission_CO2+Thr+His+l actate+PO4.apporte; 0.559, 0.800, pH+tmp+Main.P-Source.Conc. +P-Source.Feed.conc.+Nitrogen.Conc.+total.sugar+PL+rab+Thr+ His+lactate+PO4.apporte; 0.568, 0.800, pH+tmp+Main.P-Source. Conc.+P-Source.Feed.conc.+Main.Thr.Conc.+Nitrogen.Conc.+tot al.Vol+total.sugar+emission_O2+lactate+PO4.apporte; 0.604, 0.800, pH+tmp+Main.P-Source.Conc.+Nitrogen.Conc.+lactate+PO 4.apporte; 0.559, 0.800, tmp+Main.P-Source.Conc.+P-Source.F eed.conc.+Nitrogen.Conc.+total.Vol+total.sugar+PL+emission_ CO2+rab+Thr+lactate+PO4.apporte; 0.583, 0.800, tmp+Main.P-S ource.Conc.+Main.Thr.Conc.+Nitrogen.Conc.+total.Vol+total.s ugar+emission_O2+lactate+PO4.apporte; 0.575, 0.800, tmp+Mai n.P-Source.Conc.+P-Source.Feed.conc.+Main.Thr.Conc.+Nitroge n.Conc.+total.Vol+total.sugar+His+lactate+PO4.apporte; 0.57 5, 0.800, pH+tmp+Main.P-Source.Conc.+P-Source.Feed.conc.+Ma in.Thr.Conc.+Nitrogen.Conc.+emission_O2+His+lactate+PO4.app orte; 0.583, 0.800, pH+tmp+Main.P-Source.Conc.+P-Source.Fee d.conc.+Nitrogen.Conc.+total.sugar+emission_O2+Thr+PO4.appo rte; 0.575, 0.800, pH+tmp+Main.P-Source.Conc.+P-Source.Feed. conc.+Main.Thr.Conc.+Nitrogen.Conc.+emission_CO2+His+lactat e+PO4.apporte; 0.542, 0.800, pH+tmp+Main.P-Source.Conc.+P-S ource.Feed.conc.+Main.Thr.Conc.+Nitrogen.Conc.+total.Vol+to tal.sugar+emission_O2+emission_CO2+Thr+His+lactate+PO4.appo rte; 0.567, 0.800, pH+tmp+Main.P-Source.Conc.+P-Source.Feed. conc.+Nitrogen.Conc.+total.Vol+total.sugar+rab+His+lactate+ PO4.apporte; 0.550, 0.800, pH+tmp+Main.P-Source.Conc.+P-Sou rce.Feed.conc.+Main.Thr.Conc.+Nitrogen.Conc.+total.sugar+PL +emission_CO2+rab+Thr+lactate+PO4.apporte; 0.559, 0.800, tm p+Main.P-Source.Conc.+Main.Thr.Conc.+Nitrogen.Conc.+total.V ol+total.sugar+PL+emission_O2+emission_CO2+Thr+lactate+PO4. apporte; 0.567, 0.800, pH+tmp+Main.P-Source.Conc.+Main.Thr. Conc.+Nitrogen.Conc.+total.Vol+total.sugar+PL+emission_CO2+ lactate+PO4.apporte; 0.575, 0.800, pH+tmp+Main.P-Source.Con c.+P-Source.Feed.conc.+Main.Thr.Conc.+Nitrogen.Conc.+total. Vol+His+lactate+PO4.apporte; 0.582, 0.800, tmp+Main.P-Sourc e.Conc.+P-Source.Feed.conc.+Nitrogen.Conc.+total.Vol+rab+Hi s+lactate+PO4.apporte; 0.559, 0.800, tmp+Main.P-Source.Conc. +P-Source.Feed.conc.+Main.Thr.Conc.+Nitrogen.Conc.+total.su gar+PL+emission_CO2+Thr+His+lactate+PO4.apporte; 0.582, 0.8 00, tmp+Main.P-Source.Conc.+Main.Thr.Conc.+Nitrogen.Conc.+t otal.Vol+total.sugar+emission_CO2+lactate+PO4.apporte; 0.58 2, 0.800, pH+tmp+Main.P-Source.Conc.+Nitrogen.Conc.+total.s ugar+emission_O2+Thr+His+PO4.apporte; 0.550, 0.800, pH+tmp+ Main.P-Source.Conc.+P-Source.Feed.conc.+Nitrogen.Conc.+tota l.Vol+total.sugar+PL+emission_O2+Thr+His+lactate+PO4.apport e; 0.590, 0.800, pH+tmp+Main.P-Source.Conc.+Nitrogen.Conc.+ emission_CO2+Thr+His+PO4.apporte; 0.567, 0.800, pH+tmp+Main. P-Source.Conc.+P-Source.Feed.conc.+Main.Thr.Conc.+Nitrogen. Conc.+total.sugar+rab+His+lactate+PO4.apporte; 0.559, 0.800, tmp+Main.P-Source.Conc.+Main.Thr.Conc.+Nitrogen.Conc.+tota l.Vol+total.sugar+PL+emission_CO2+rab+Thr+lactate+PO4.appor te; 0.567, 0.800, pH+tmp+Main.P-Source.Conc.+P-Source.Feed. conc.+Main.Thr.Conc.+Nitrogen.Conc.+total.Vol+total.sugar+H is+lactate+PO4.apporte; 0.590, 0.800, tmp+Main.P-Source.Con c.+P-Source.Feed.conc.+Nitrogen.Conc.+total.Vol+total.sugar +lactate+PO4.apporte; 0.590, 0.800, pH+tmp+Main.P-Source.Co nc.+P-Source.Feed.conc.+Nitrogen.Conc.+Thr+His+PO4.apporte; 0.567, 0.800, pH+tmp+Main.P-Source.Conc.+P-Source.Feed.con c.+Nitrogen.Conc.+total.Vol+total.sugar+emission CO2+His+la ctate+PO4.apporte; 0.567, 0.800, tmp+Main.P-Source.Conc.+P-Source.Feed.conc.+Main.Thr.Conc.+Nitrogen.Conc.+total.Vol+t otal.sugar+emission_O2+emission_CO2+lactate+PO4.apporte; 0. 575, 0.800, tmp+Main.P-Source.Conc.+P-Source.Feed.conc.+Mai n.Thr.Conc.+Nitrogen.Conc.+total.Vol+total.sugar+emission_O 2+lactate+PO4.apporte; 0.558, 0.800, tmp+Main.P-Source.Conc. +Main.Thr.Conc.+Nitrogen.Conc.+total.Vol+total.sugar+emissi on_O2+emission_CO2+Thr+His+lactate+PO4.apporte; 0.558, 0.80 0, pH+tmp+Main.P-Source.Conc.+Main.Thr.Conc.+Nitrogen.Conc. +total.sugar+PL+rab+Thr+His+lactate+PO4.apporte; 0.582, 0.8 00, tmp+Main.P-Source.Conc.+P-Source.Feed.conc.+Main.Thr.Co nc.+Nitrogen.Conc.+total.Vol+His+lactate+PO4.apporte; 0.558, 0.800, tmp+Main.P-Source.Conc.+P-Source.Feed.conc.+Nitroge n.Conc.+total.Vol+total.sugar+PL+emission_O2+Thr+His+lactat e+PO4.apporte; 0.558, 0.800, pH+tmp+Main.P-Source.Conc.+P-S ource.Feed.conc.+Nitrogen.Conc.+total.Vol+PL+rab+Thr+His+la ctate+PO4.apporte; 0.566, 0.800, tmp+Main.P-Source.Conc.+Ni trogen.Conc.+total.Vol+total.sugar+PL+emission_CO2+Thr+His+ lactate+PO4.apporte; 0.582, 0.800, pH+tmp+Main.P-Source.Con c.+Main.Thr.Conc.+Nitrogen.Conc.+emission_O2+emission_CO2+T hr+PO4.apporte; 0.574, 0.800, pH+tmp+Main.P-Source.Conc.+Ma in.Thr.Conc.+Nitrogen.Conc.+total.sugar+rab+His+lactate+PO4. apporte; 0.582, 0.800, tmp+Main.P-Source.Conc.+Main.Thr.Con c.+Nitrogen.Conc.+total.sugar+emission_O2+emission_CO2+lact ate+PO4.apporte; 0.603, 0.800, pH+tmp+Main.P-Source.Conc.+N itrogen.Conc.+rab+PO4.apporte; 0.574, 0.800, pH+tmp+Main.P-Source.Conc.+P-Source.Feed.conc.+Nitrogen.Conc.+total.Vol+e mission_O2+His+lactate+PO4.apporte; 0.574, 0.800, tmp+Main. P-Source.Conc.+Main.Thr.Conc.+Nitrogen.Conc.+total.Vol+tota l.sugar+emission_O2+emission_CO2+lactate+PO4.apporte; 0.558, 0.800, pH+tmp+Main.P-Source.Conc.+Main.Thr.Conc.+Nitrogen. Conc.+total.Vol+total.sugar+PL+rab+Thr+lactate+PO4.apporte; 0.574, 0.800, pH+tmp+Main.P-Source.Conc.+Main.Thr.Conc.+Ni trogen.Conc.+total.Vol+total.sugar+rab+lactate+PO4.apporte; 0.589, 0.800, tmp+Main.P-Source.Conc.+Main.Thr.Conc.+Nitro gen.Conc.+emission_O2+His+lactate+PO4.apporte; 0.582, 0.800, tmp+Main.P-Source.Conc.+P-Source.Feed.conc.+Nitrogen.Conc. +total.Vol+total.sugar+His+lactate+PO4.apporte; 0.574, 0.80 0, pH+tmp+Main.P-Source.Conc.+Main.Thr.Conc.+Nitrogen.Conc. +total.sugar+emission_O2+His+lactate+PO4.apporte; 0.582, 0. 800, tmp+Main.P-Source.Conc.+Main.Thr.Conc.+Nitrogen.Conc.+ total.Vol+total.sugar+His+lactate+PO4.apporte; 0.582, 0.800, pH+tmp+Main.P-Source.Conc.+P-Source.Feed.conc.+Nitrogen.Co nc.+total.sugar+Thr+His+PO4.apporte; 0.566, 0.800, pH+tmp+M ain.P-Source.Conc.+P-Source.Feed.conc.+Main.Thr.Conc.+Nitro gen.Conc.+total.sugar+emission_CO2+His+lactate+PO4.apporte; 0.566, 0.800, pH+tmp+Main.P-Source.Conc.+P-Source.Feed.con c.+Main.Thr.Conc.+Nitrogen.Conc.+total.sugar+emission-O2+Hi s+lactate+PO4.apporte; 0.589, 0.800, pH+tmp+Main.P-Source.C onc.+Main.Thr.Conc.+Nitrogen.Conc.+rab+Thr+PO4.apporte; 0.5 96, 0.800, tmp+Main.P-Source.Conc.+Nitrogen.Conc.+emission_ O2+His+lactate+PO4.apporte; 0.574, 0.800, pH+tmp+Main.P-Sou rce.Conc.+Main.Thr.Conc.+Nitrogen.Conc.+emission_O2+emissio n_CO2+His+lactate+PO4.apporte; 0.566, 0.800, pH+tmp+Main.P-Source.Conc.+P-Source.Feed.conc.+Main.Thr.Conc.+Nitrogen.Co nc.+emission_O2+emission_CO2+His+lactate+PO4.apporte; 0.574, 0.800, pH+tmp+Main.P-Source.Conc.+Main.Thr.Conc.+Nitrogen. Conc.+total.sugar+emission_CO2+His+lactate+PO4.apporte; 0.5 66, 0.800, pH+tmp+Main.P-Source.Conc.+Main.Thr.Conc.+Nitrog en.Conc.+total.Vol+total.sugar+rab+Thr+lactate+PO4.apporte; 0.540, 0.800, pH+tmp+Main.P-Source.Conc.+P-Source.Feed.con c.+Main.Thr.Cone.+Nitrogen.Cone.+total.Vol+total.sugar+PL+e mission_O2+emission_CO2+Thr+lactate+PO4.apporte; 0.574, 0.8 00, pH+tmp+Main.P-Source.Conc.+P-Source.Feed.conc.+Nitrogen. Conc.+total.sugar+emission_O2+emission_CO2+Thr+PO4.apporte; 0.581, 0.800, pH+tmp+Main.P-Source.Conc.+Main.Thr.Conc.+Ni trogen.Conc.+total.sugar+PL+Thr+PO4.apporte; 0.581, 0.800, tmp+Main.P-Source.Conc.+Main.Thr.Conc.+Nitrogen.Conc.+total. sugar+emission_O2+His+lactate+PO4.apporte; 0.589, 0.800, pH +tmp+Main.P-Source.Conc.+P-Source.Feed.conc.+Nitrogen.Conc. +rab+Thr+PO4.apporte; 0.581, 0.800, tmp+Main.P-Source.Conc. +P-Source.Feed.conc.+Nitrogen.Conc.+total.sugar+PL+His+lact ate+PO4.apporte; 0.549, 0.800, tmp+Main.P-Source.Conc.+P-So urce.Feed.conc.+Main.Thr.Conc.+Nitrogen.Conc.+total.Vol+PL+ emission_O2+emission_CO2+Thr+His+lactate+PO4.apporte; 0.574, 0.800, tmp+Main.P-Source.Conc.+Nitrogen.Conc.+total.Vol+PL +rab+Thr+His+lactate+PO4.apporte; 0.558, 0.800, pH+tmp+Main. P-Source.Conc.+P-Source.Feed.conc.+Main.Thr.Conc.+Nitrogen. Conc.+total.sugar+rab+Thr+His+lactate+PO4.apporte; 0.581, 0. 799, pH+tmp+Main.P-Source.Conc.+P-Source.Feed.conc.+Nitroge n.Conc.+PL+His+lactate+PO4.apporte; 0.596, 0.799, tmp+Main. P-Source.Conc.+Nitrogen.Conc.+total.sugar+emission_O2+lacta te+PO4.apporte; 0.581, 0.799, tmp+Main.P-Source.Conc.+P-Sou rce.Feed.conc.+Main.Thr.Conc.+Nitrogen.Conc.+total.sugar+PL +lactate+PO4.apporte; 0.581, 0.799, tmp+Main.P-Source.Conc. +P-Source.Feed.conc.+Nitrogen.Conc.+PL+rab+His+lactate+PO4. apporte; 0.566, 0.799, pH+tmp+Main.P-Source.Conc.+P-Source. Feed.conc.+Nitrogen.Conc.+total.Vol+emission_O2+emission_CO 2+His+lactate+PO4.apporte; 0.549, 0.799, pH+tmp+Main.P-Sour ce.Conc.+P-Source.Feed.conc.+Main.Thr.Conc.+Nitrogen.Conc.+ total.sugar+PL+emission_CO2+Thr+His+lactate+PO4.apporte; 0. 588, 0.799, tmp+Main.P-Source.Conc.+P-Source.Feed.conc.+Mai n.Thr.Conc.+Nitrogen.Conc.+total.sugar+Thr+PO4.apporte; 0.6 02, 0.799, pH+tmp+Main.P-Source.Conc.+Nitrogen.Conc.+emissi on_CO2+PO4.apporte; 0.557, 0.799, tmp+Main.P-Source.Conc.+M ain.Thr.Conc.+Nitrogen.Conc.+total.sugar+PL+emission_CO2+ra b+Thr+His+lactate+PO4.apporte; 0.549, 0.799, tmp+Main.P-Sou rce.Conc.+P-Source.Feed.conc.+Main.Thr.Conc.+Nitrogen.Conc. +total.sugar+PL+emission_O2+emission_CO2+Thr+His+lactate+PO 4.apporte; 0.573, 0.799, pH+tmp+Main.P-Source.Conc.+Main.Th r.Conc.+Nitrogen.Conc.+total.Vol+rab+His+lactate+PO4.apport e; 0.557, 0.799, tmp+Main.P-Source.Conc.+P-Source.Feed.conc. +Main.Thr.Conc.+Nitrogen.Conc.+total.Vol+total.sugar+emissi on_CO2+Thr+His+lactate+PO4.apporte; 0.573, 0.799, tmp+Main. P-Source.Conc.+P-Source.Feed.conc.+Nitrogen.Conc.+total.Vol +total.sugar+rab+Thr+lactate+PO4.apporte; 0.581, 0.799, tmp +Main.P-Source.Conc.+P-Source.Feed.conc.+Nitrogen.Conc.+tot al.Vol+total.sugar+rab+lactate+PO4.apporte; 0.595, 0.799, p H+tmp+Main.P-Source.Conc.+Nitrogen.Conc.+emission_CO2+lacta te+PO4.apporte; 0.549, 0.799, pH+tmp+Main.P-Source.Conc.+P-Source.Feed.conc.+Nitrogen.Conc.+total.Vol+total.sugar+PL+e mission_CO2+Thr+His+lactate+PO4.apporte; 0.602, 0.799, pH+t mp+Main.P-Source.Conc.+Main.Thr.Conc.+Nitrogen.Conc.+PO4.ap porte; 0.540, 0.799, pH+tmp+Main.P-Source.Conc.+Main.Thr.Co nc.+Nitrogen.Conc.+total.Vol+total.sugar+PL+emission_O2+emi ssion_CO2+Thr+His+lactate+PO4.apporte; 0.557, 0.799, pH+tmp +Main.P-Source.Conc.+Main.Thr.Conc.+Nitrogen.Conc.+total.Vo l+total.sugar+PL+emission_O2+emission_CO2+lactate+PO4.appor te; 0.557, 0.799, tmp+Main.P-Source.Conc.+Main.Thr.Conc.+Ni trogen.Conc.+total.Vol+total.sugar+emission_CO2+rab+Thr+His +lactate+PO4.apporte; 0.602, 0.799, pH+tmp+Main.P-Source.Co nc.+Nitrogen.Conc.+emission_O2+PO4.apporte; 0.588, 0.799, p H+tmp+Main.P-Source.Conc.+Nitrogen.Conc.+PL+His+lactate+PO4. apporte; 0.565, 0.799, tmp+Main.P-Source.Conc.+P-Source.Fee d.conc.+Main.Thr.Conc.+Nitrogen.Conc.+total.Vol+PL+rab+Thr+ lactate+PO4.apporte; 0.557, 0.799, pH+tmp+Main.P-Source.Con c.+P-Source.Feed.conc.+Nitrogen.Conc.+total.Vol+total.sugar +PL+rab+Thr+lactate+PO4.apporte; 0.602, 0.799, tmp+Main.P-S ource.Conc.+P-Source.Feed.conc.+Nitrogen.Conc.+Thr+PO4.appo rte; 0.557, 0.799, pH+tmp+Main.P-Source.Conc.+P-Source.Feed. conc.+Main.Thr.Conc.+Nitrogen.Conc.+total.sugar+emission_O2 +emission_CO2+His+lactate+PO4.apporte; 0.580, 0.799, tmp+Ma in.P-Source.Conc.+Nitrogen.Conc.+total.Vol+total.sugar+rab+ Thr+lactate+PO4.apporte; 0.573, 0.799, tmp+Main.P-Source.Co nc.+Nitrogen.Conc.+total.Vol+total.sugar+PL+rab+Thr+lactate +PO4.apporte; 0.557, 0.799, pH+tmp+Main.P-Source.Conc.+P-So urce.Feed.conc.+Nitrogen.Conc.+total.Vol+total.sugar+emissi on_O2+emission_CO2+His+lactate+PO4.apporte; 0.588, 0.799, t mp+Main.P-Source.Conc.+Main.Thr.Conc.+Nitrogen.Conc.+total. Vol+His+lactate+PO4.apporte; 0.565, 0.799, pH+tmp+Main.P-So urce.Conc.+P-Source.Feed.conc.+Nitrogen.Conc.+total.Vol+tot al.sugar+emission_O2+His+lactate+PO4.apporte; 0.573, 0.799, tmp+Main.P-Source.Conc.+P-Source.Feed.conc.+Nitrogen.Conc. +total.Vol+total.sugar+rab+His+lactate+PO4.apporte; 0.539, 0.799, pH+tmp+Main.P-Source.Conc.+P-Source.Feed.conc.+Main. Thr.Conc.+Nitrogen.Conc.+total.Vol+PL+emission_CO2+rab+Thr+ His+lactate+PO4.apporte; 0.556, 0.799, pH+tmp+Main.P-Source. Conc.+P-Source.Feed.conc.+Main.Thr.Conc.+Nitrogen.Conc.+tot al.Vol+total.sugar+emission_CO2+His+lactate+PO4.apporte; 0. 580, 0.799, pH+tmp+Main.P-Source.Conc.+Nitrogen.Conc.+total. Vol+total.sugar+PL+lactate+PO4.apporte; 0.580, 0.799, tmp+M ain.P-Source.Conc.+Nitrogen.Conc.+total.Vol+rab+Thr+His+lac tate+PO4.apporte; 0.548, 0.799, pH+tmp+Main.P-Source.Conc.+ P-Source.Feed.conc.+Main.Thr.Conc.+Nitrogen.Conc.+total.Vol +total.sugar+PL+Thr+His+lactate+PO4.apporte; 0.573, 0.799, pH+tmp+Main.P-Source.Conc.+Nitrogen.Conc.+total.sugar+emiss ion_O2+emission_CO2+Thr+His+PO4.apporte; 0.556, 0.799, tmp+ Main.P-Source.Conc.+Nitrogen.Conc.+total.Vol+total.sugar+PL +emission_O2+emission_CO2+Thr+His+lactate+PO4.apporte; 0.58 0, 0.799, pH+tmp+Main.P-Source.Conc.+Main.Thr.Conc.+Nitroge n.Conc.+total.sugar+emission_CO2+Thr+PO4.apporte; 0.564, 0. 799, tmp+Main.P-Source.Conc.+P-Source.Feed.conc.+Nitrogen.C onc.+total.Vol+PL+rab+Thr+His+lactate+PO4.apporte; 0.572, 0. 799, tmp+Main.P-Source.Conc.+P-Source.Feed.conc.+Main.Thr.C onc.+Nitrogen.Conc.+total.sugar+PL+His+lactate+PO4.apporte; 0.548, 0.799, pH+tmp+Main.P-Source.Conc.+P-Source.Feed.con c.+Nitrogen.Conc.+total.sugar+PL+emission_CO2+rab+Thr+His+l actate+PO4.apporte; 0.556, 0.799, tmp+Main.P-Source.Conc.+P -Source.Feed.conc.+Main.Thr.Conc.+Nitrogen.Conc.+total.Vol+ total.sugar+PL+Thr+His+lactate+PO4.apporte; 0.587, 0.799, p H+tmp+Main.P-Source.Conc.+Main.Thr.Conc.+Nitrogen.Conc.+emi ssion_O2+emission_CO2+PO4.apporte; 0.556, 0.799, pH+tmp+Mai n.P-Source.Conc.+Main.Thr.Conc.+Nitrogen.Conc.+total.sugar+ emission_CO2+rab+Thr+His+lactate+PO4.apporte; 0.556, 0.799, tmp+Main.P-Source.Conc.+P-Source.Feed.conc.+Main.Thr.Conc. +Nitrogen.Conc.+total.Vol+total.sugar+PL+emission_CO2+Thr+l actate+PO4.apporte; 0.564, 0.799, pH+tmp+Main.P-Source.Conc. +Main.Thr.Conc.+Nitrogen.Conc.+total.Vol+total.sugar+PL+emi ssion_O2+lactate+PO4.apporte; 0.594, 0.799, tmp+Main.P-Sour ce.Conc.+P-Source.Feed.conc.+Nitrogen.Conc.+rab+Thr+PO4.app orte; 0.587, 0.799, tmp+Main.P-Source.Conc.+Nitrogen.Conc.+ total.sugar+emission_O2+emission_CO2+Thr+PO4.apporte; 0.548, 0.799, tmp+Main.P-Source.Conc.+P-Source.Feed.conc.+Main.Th r.Conc.+Nitrogen.Conc.+total.Vol+total.sugar+emission_O2+em ission_CO2+Thr+His+lactate+PO4.apporte; 0.594, 0.799, tmp+M ain.P-Source.Conc.+Nitrogen.Conc.+total.sugar+emission_CO2+ lactate+PO4.apporte; 0.539, 0.799, pH+tmp+Main.P-Source.Con c.+P-Source.Feed.conc.+Main.Thr.Conc.+Nitrogen.Conc.+total. sugar+PL+emission_O2+emission_CO2+Thr+His+lactate+PO4.appor te; 0.594, 0.799, pH+tmp+Main.P-Source.Conc.+Nitrogen.Conc. +emission_O2+lactate+PO4.apporte; 0.587, 0.799, tmp+Main.P-Source.Conc.+P-Source.Feed.conc.+Nitrogen.Conc.+total.sugar +PL+lactate+PO4.apporte; 0.580, 0.799, tmp+Main.P-Source.Co nc.+P-Source.Feed.conc.+Nitrogen.Conc.+PL+emission_O2+His+l actate+PO4.apporte; 0.587, 0.799, pH+tmp+Main.P-Source.Conc. +P-Source.Feed.conc.+Nitrogen.Conc.+emission O2+Thr+PO4.app orte; 0.587, 0.799, tmp+Main.P-Source.Conc.+Main.Thr.Conc.+ Nitrogen.Conc.+total.sugar+emission_O2+Thr+PO4.apporte; 0.5 80, 0.799, tmp+Main.P-Source.Conc.+P-Source.Feed.conc.+Nitr ogen.Conc.+total.Vol+emission_CO2+His+lactate+PO4.apporte; 0.580, 0.799, tmp+Main.P-Source.Conc.+P-Source.Feed.conc.+N itrogen.Conc.+total.Vol+total.sugar+emission_CO2+lactate+PO 4.apporte; 0.594, 0.799, tmp+Main.P-Source.Conc.+Nitrogen.C onc.+emission_CO2+His+lactate+PO4.apporte; 0.579, 0.799, tm p+Main.P-Source.Conc.+P-Source.Feed.conc.+Nitrogen.Conc.+to tal.Vol+emission_O2+His+lactate+PO4.apporte; 0.594, 0.799, tmp+Main.P-Source.Conc.+Main.Thr.Conc.+Nitrogen.Conc.+rab+T hr+PO4.apporte; 0.564, 0.799, tmp+Main.P-Source.Conc.+P-Sou rce.Feed.conc.+Main.Thr.Conc.+Nitrogen.Conc.+total.sugar+ra b+Thr+His+lactate+PO4.apporte; 0.579, 0.799, tmp+Main.P-Sou rce.Conc.+P-Source.Feed.conc.+Main.Thr.Conc.+Nitrogen.Conc. +PL+His+lactate+PO4.apporte; 0.594, 0.799, tmp+Main.P-Sourc e.Conc.+Nitrogen.Conc.+total.sugar+PL+Thr+PO4.apporte; 0.60 1, 0.799, tmp+Main.P-Source.Conc.+Main.Thr.Conc.+Nitrogen.C onc.+Thr+PO4.apporte; 0.579, 0.799, tmp+Main.P-Source.Conc. +Main.Thr.Conc.+Nitrogen.Conc.+total.sugar+emission_CO2+His +lactate+PO4.apporte; 0.587, 0.798, tmp+Main.P-Source.Conc. +Main.Thr.Conc.+Nitrogen.Conc.+emission_CO2+His+lactate+PO4. apporte; 0.579, 0.798, tmp+Main.P-Source.Conc.+P-Source.Fee d.conc.+Nitrogen.Conc.+total.sugar+PL+rab+lactate+PO4.appor te; 0.587, 0.798, tmp+Main.P-Source.Conc.+P-Source.Feed.con c.+Nitrogen.Conc.+emission_O2+emission_CO2+Thr+PO4.apporte; 0.587, 0.798, pH+tmp+Main.P-Source.Conc.+Nitrogen.Conc.+to tal.sugar+PL+lactate+PO4.apporte; 0.555, 0.798, tmp+Main.P-Source.Conc.+Main.Thr.Conc.+Nitrogen.Conc.+total.Vol+total. sugar+PL+emission_O2+Thr+His+lactate+PO4.apporte; 0.587, 0. 798, tmp+Main.P-Source.Conc.+Nitrogen.Conc.+total.sugar+emi ssion_O2+His+lactate+PO4.apporte; 0.601, 0.798, tmp+Main.P-Source.Conc.+Nitrogen.Conc.+emission_O2+Thr+PO4.apporte; 0. 572, 0.798, pH+tmp+Main.P-Source.Conc.+P-Source.Feed.conc.+ Nitrogen.Conc.+emission_O2+emission_CO2+Thr+His+PO4.apport e; 0.564, 0.798, pH+tmp+Main.P-Source.Conc.+P-Source.Feed.c onc.+Main.Thr.Conc.+Nitrogen.Conc.+total.Vol+emission_CO2+H is+lactate+PO4.apporte; 0.587, 0.798, tmp+Main.P-Source.Con c.+P-Source.Feed.conc.+Nitrogen.Conc.+total.sugar+emission_ O2+Thr+PO4.apporte; 0.594, 0.798, tmp+Main.P-Source.Conc.+N itrogen.Conc.+emission_O2+emission_CO2+Thr+PO4.apporte; 0.5 87, 0.798, pH+tmp+Main.P-Source.Conc.+Nitrogen.Conc.+emissi on_O2+emission_CO2+lactate+PO4.apporte; 0.594, 0.798, pH+tm p+Main.P-Source.Conc.+Nitrogen.Conc.+total.Vol+lactate+PO4. apporte; 0.555, 0.798, tmp+Main.P-Source.Conc.+Nitrogen.Con c.+total.Vol+total.sugar+PL+emission_CO2+rab+Thr+His+lactat e+PO4.apporte; 0.563, 0.798, pH+tmp+Main.P-Source.Conc.+Mai n.Thr.Conc.+Nitrogen.Conc.+total.sugar+emission_O2+emission _CO2+His+lactate+PO4.apporte; 0.594, 0.798, pH+tmp+Main.P-S ource.Conc.+Nitrogen.Conc.+rab+lactate+PO4.apporte; 0.571, 0.798, tmp+Main.P-Source.Conc.+P-Source.Feed.conc.+Nitrogen. Conc.+total.sugar+PL+rab+His+lactate+PO4.apporte; 0.586, 0. 798, pH+tmp+Main.P-Source.Conc.+Main.Thr.Conc.+Nitrogen.Con c.+emission_O2+Thr+PO4.apporte; 0.571, 0.798, tmp+Main.P-So urce.Conc.+P-Source.Feed.conc.+Main.Thr.Conc.+Nitrogen.Conc. +total.sugar+PL+rab+lactate+PO4.apporte; 0.547, 0.798, tmp+ Main.P-Source.Conc.+P-Source.Feed.conc.+Main.Thr.Conc.+Nitr ogen.Conc.+total.Vol+PL+emission_CO2+rab+Thr+His+lactate+PO 4.apporte; 0.555, 0.798, pH+tmp+Main.P-Source.Conc.+Main.Th r.Conc.+Nitrogen.Conc.+total.Vol+PL+rab+Thr+His+lactate+PO4. apporte; 0.563, 0.798, tmp+Main.P-Source.Conc.+P-Source.Fee d.conc.+Main.Thr.Conc.+Nitrogen.Conc.+total.Vol+total.sugar +emission_CO2+His+lactate+PO4.apporte; 0.586, 0.798, pH+tmp +Main.P-Source.Conc.+Main.Thr.Conc.+Nitrogen.Conc.+Thr+His+ PO4.apporte; 0.593, 0.798, tmp+Main.P-Source.Conc.+Nitrogen. Conc.+total.Vol+total.sugar+lactate+PO4.apporte; 0.579, 0.7 98, pH+tmp+Main.P-Source.Conc.+Main.Thr.Conc.+Nitrogen.Conc. +total.Vol+total.sugar+Thr+PO4.apporte; 0.579, 0.798, tmp+M ain.P-Source.Conc.+P-Source.Feed.conc.+Nitrogen.Conc.+PL+em ission_CO2+His+lactate+PO4.apporte; 0.579, 0.798, tmp+Main. P-Source.Conc.+P-Source.Feed.conc.+Nitrogen.Conc.+total.Vol +total.sugar+emission_O2+lactate+PO4.apporte; 0.586, 0.798, tmp+Main.P-Source.Conc.+Main.Thr.Conc.+Nitrogen.Conc.+tota l.sugar+PL+lactate+PO4.apporte; 0.546, 0.798, pH+tmp+Main.P -Source.Conc.+P-Source.Feed.conc.+Main.Thr.Conc.+Nitrogen.C onc.+total.sugar+emission_CO2+rab+Thr+His+lactate+PO4.appor te; 0.546, 0.798, pH+tmp+Main.P-Source.Conc.+P-Source.Feed. conc.+Main.Thr.Conc.+Nitrogen.Conc.+total.Vol+total.sugar+e mission_O2+emission_CO2+His+lactate+PO4.apporte; 0.571, 0.7 98, pH+tmp+Main.P-Source.Conc.+Nitrogen.Conc.+total.Vol+tot al.sugar+PL+emission_CO2+lactate+PO4.apporte; 0.563, 0.798, tmp+Main.P-Source.Conc.+P-Source.Feed.conc.+Main.Thr.Conc. +Nitrogen.Conc.+total.Vol+total.sugar+emission_O2+His+lacta te+PO4.apporte; 0.579, 0.798, pH+tmp+Main.P-Source.Conc.+Ni trogen.Conc.+total.Vol+emission_O2+emission_CO2+lactate+PO4. apporte

### [12. Linear Model that Predicts Lysine Production Amount in Interval 2]

0.775, 0.900, tmp+Main.P-Source.Conc.+Nitrogen.Conc.+total. Vol+PL+emission_CO2+His+lactate+PO4.ferm.+PO4.apporte; 0.77 0, 0.900, tmp+Main.P-Source.Conc.+Nitrogen.Conc.+total.Vol+ PL+emission_O2+emission_CO2+His+lactate+PO4.ferm.+PO4.appor te; 0.774, 0.899, tmp+Main.P-Source.Conc.+Nitrogen.Conc.+to tal.Vol+PL+emission_CO2+His+lactate+PO4.apporte+PO4.utilis e; 0.766, 0.899, tmp+Main.P-Source.Conc.+Nitrogen.Conc.+tot al.Vol+PL+emission_O2+emission_CO2+Thr+His+lactate+PO4.ferm. +PO4.apporte; 0.770, 0.899, tmp+Main.P-Source.Conc.+Nitroge n.Conc.+total.Vol+PL+emission_CO2+rab+His+lactate+PO4.ferm. +PO4.apporte; 0.770, 0.899, tmp+Main.P-Source.Conc.+Nitroge n.Conc.+total.Vol+PL+emission_O2+emission_CO2+His+lactate+P O4.apporte+PO4.utilise; 0.778, 0.899, Main.P-Source.Cone.+N itrogen.Conc.+total.Vol+PL+emission_CO2+His+lactate+PO4.fer m.+PO4.apporte; 0.769, 0.899, tmp+Main.P-Source.Conc.+Nitro gen.Conc.+total.Vol+PL+emission_CO2+Thr+His+lactate+PO4.fer m.+PO4.apporte; 0.778, 0.899, Main.P-Source.Cone.+Nitrogen. Conc.+total.Vol+PL+emission_CO2+His+lactate+PO4.apporte+PO4. utilise; 0.765, 0.899, tmp+Main.P-Source.Conc.+Nitrogen.Con c.+total.Vol+PL+emission_O2+emission_CO2+Thr+His+lactate+PO 4.apporte+PO4.utilise; 0.769, 0.899, tmp+Main.P-Source.Conc. +Nitrogen.Conc.+total.Vol+PL+emission_CO2+rab+His+lactate+P O4.apporte+PO4.utilise; 0.769, 0.899, tmp+Main.P-Source.Con c.+Nitrogen.Conc.+total.Vol+PL+emission_CO2+Thr+His+lactate +PO4.apporte+PO4.utilise; 0.781, 0.899, Main.P-Source.Conc. +Nitrogen.Conc.+total.Vol+PL+emission_CO2+His+lactate+PO4.a pporte; 0.764, 0.899, tmp+Main.P-Source.Conc.+Nitrogen.Conc. +total.Vol+PL+emission_CO2+rab+Thr+His+lactate+PO4.ferm.+PO 4.apporte; 0.764, 0.898, tmp+Main.P-Source.Conc.+Nitrogen.C onc.+total.Vol+PL+emission_CO2+rab+Thr+His+lactate+PO4.appo rte+PO4.utilise; 0.775, 0.898, tmp+Main.P-Source.Conc.+Nitr ogen.Conc.+total.Vol+PL+emission_CO2+His+lactate+PO4.apport e; 0.766, 0.898, pH+tmp+Main.P-Source.Conc.+Nitrogen.Conc.+ total.Vol+PL+emission_CO2+His+lactate+PO4.ferm.+PO4.apport e; 0.770, 0.898, tmp+Main.P-Source.Conc.+Nitrogen.Conc.+tot al.Vol+emission_CO2+rab+His+lactate+PO4.ferm.+PO4.apporte; 0.770, 0.898, tmp+Main.P-Source.Conc.+Nitrogen.Conc.+total. Vol+emission_O2+emission_CO2+His+lactate+PO4.ferm.+PO4.appo rte; 0.774, 0.897, pH+Main.P-Source.Cone.+Nitrogen.Cone.+to tal.Vol+PL+emission_CO2+His+lactate+PO4.apporte; 0.766, 0.8 97, pH+tmp+Main.P-Source.Conc.+Nitrogen.Conc.+total.Vol+PL+ emission_CO2+His+lactate+PO4.apporte+PO4.utilise; 0.761, 0. 897, pH+tmp+Main.P-Source.Conc.+Nitrogen.Conc.+total.Vol+PL +emission_O2+emission_CO2+His+lactate+PO4.ferm.+PO4.apport e; 0.756, 0.897, tmp+Main.P-Source.Conc.+P-Source.Feed.conc. +Nitrogen.Conc.+total.Vol+PL+emission_O2+emission_CO2+Thr+H is+lactate+PO4.ferm.+PO4.apporte; 0.765, 0.897, tmp+Main.P-Source.Conc.+Nitrogen.Conc.+OD+total.Vol+PL+emission_CO2+Hi s+lactate+PO4.ferm.+PO4.apporte; 0.769, 0.897, tmp+Main.P-S ource.Conc.+Nitrogen.Conc.+total.Vol+emission_O2+emission_C O2+His+lactate+PO4.apporte+PO4.utilise; 0.769, 0.897, tmp+M ain.P-Source.Conc.+Nitrogen.Conc.+total.Vol+emission_CO2+ra b+His+lactate+PO4.apporte+PO4.utilise; 0.773, 0.897, Main.P -Source.Conc.+Nitrogen.Conc.+OD+total.Vol+PL+emission-CO2+H is+lactate+PO4.apporte; 0.769, 0.897, pH+Main.P-Source.Conc. +Nitrogen.Conc.+total.Vol+PL+emission_CO2+His+lactate+PO4.f erm.+PO4.apporte; 0.760, 0.897, pH+tmp+Main.P-Source.Conc.+ Nitrogen.Conc.+total.Vol+PL+emission_CO2+rab+His+lactate+PO 4.ferm.+PO4.apporte; 0.765, 0.897, tmp+Main.P-Source.Conc.+ Nitrogen.Conc.+total.Vol+PL+emission_O2+emission_CO2+Thr+la ctate+PO4.ferm.+PO4.apporte; 0.769, 0.897, pH+Main.P-Source. Conc.+Nitrogen.Conc.+total.Vol+PL+emission_CO2+His+lactate+ PO4.apporte+PO4.utilise; 0.773, 0.897, tmp+P-Source.Feed.co nc.+PL+emission_CO2+Thr+His+lactate+PO4.apporte+PO4.utilis e; 0.756, 0.897, tmp+Main.P-Source.Conc.+P-Source.Feed.conc. +Nitrogen.Conc.+total.Vol+PL+emission_O2+emission_CO2+Thr+H is+lactate+PO4.apporte+PO4.utilise; 0.760, 0.897, pH+tmp+Ma in.P-Source.Conc.+Nitrogen.Conc.+total.Vol+PL+emission_O2+e mission_CO2+His+lactate+PO4.apporte+PO4.utilise; 0.760, 0.8 97, pH+tmp+Main.P-Source.Conc.+Nitrogen.Conc.+total.Vol+PL+ emission_CO2+Thr+His+lactate+PO4.ferm.+PO4.apporte; 0.764, 0.897, tmp+Main.P-Source.Conc.+Nitrogen.Conc.+OD+total.Vol+ PL+emission_CO2+His+lactate+PO4.apporte+PO4.utilise; 0.773, 0.897, tmp+Main.P-Source.Conc.+Nitrogen.Conc.+total.Vol+em ission_O2+emission_CO2+lactate+PO4.ferm.+PO4.apporte; 0.773, 0.897, tmp+Main.P-Source.Conc.+Nitrogen.Conc.+total.Vol+em ission_CO2+rab+lactate+PO4.ferm.+PO4.apporte; 0.760, 0.897, tmp+Main.P-Source.Conc.+Nitrogen.Conc.+OD+total.Vol+PL+emi ssion_O2+emission_CO2+His+lactate+PO4.ferm.+PO4.apporte; 0. 769, 0.897, tmp+Main.P-Source.Conc.+Nitrogen.Conc.+OD+PL+em ission_CO2+His+lactate+PO4.ferm.+PO4.apporte; 0.773, 0.897, tmp+P-Source.Feed.conc.+PL+emission CO2+Thr+His+lactate+PO 4.ferm.+PO4.apporte; 0.760, 0.897, pH+tmp+Main.P-Source.Con c.+Nitrogen.Conc.+total.Vol+PL+emission_CO2+rab+His+lactate +PO4.apporte+PO4.utilise; 0.768, 0.897, tmp+Main.P-Source.C onc.+Nitrogen.Conc.+total.Vol+PL+emission_CO2+Thr+His+lacta te+PO4.apporte; 0.773, 0.897, tmp+Main.P-Source.Conc.+Nitro gen.Conc.+total.Vol+emission_CO2+His+lactate+PO4.ferm.+PO4. apporte; 0.760, 0.897, pH+tmp+Main.P-Source.Conc.+Nitrogen. Conc.+total.Vol+PL+emission_CO2+Thr+His+lactate+PO4.apporte +PO4.utilise; 0.764, 0.897, tmp+Main.P-Source.Conc.+Nitroge n.Conc.+total.Vol+PL+emission_O2+emission_CO2+Thr+lactate+P O4.apporte+PO4.utilise; 0.755, 0.897, pH+tmp+Main.P-Source. Conc.+Nitrogen.Conc.+total.Vol+PL+emission_O2+emission_CO2+ Thr+His+lactate+PO4.ferm.+PO4.apporte; 0.768, 0.897, Main.P -Source.Conc.+Nitrogen.Conc.+OD+total.Vol+PL+emission-CO2+H is+lactate+PO4.ferm.+PO4.apporte; 0.764, 0.897, tmp+Main.P-Source.Conc.+Nitrogen.Conc.+total.Vol+yield+PL+emission_CO2 +His+lactate+PO4.ferm.+PO4.apporte; 0.768, 0.897, tmp+Main. P-Source.Conc.+Nitrogen.Conc.+OD+PL+emission_CO2+His+lactat e+PO4.apporte+PO4.utilise; 0.759, 0.897, tmp+Main.P-Source. Conc.+P-Source.Feed.conc.+Nitrogen.Conc.+total.Vol+PL+emiss ion_CO2+Thr+His+lactate+PO4.ferm.+PO4.apporte; 0.772, 0.897, tmp+Main.P-Source.Conc.+Nitrogen.Conc.+OD+PL+emission_CO2+ His+lactate+PO4.apporte; 0.776, 0.897, tmp+P-Source.Feed.co nc.+PL+emission_CO2+Thr+His+lactate+PO4.apporte; 0.764, 0.8 97, tmp+Main.P-Source.Conc.+Nitrogen.Conc.+total.Vol+yield+ PL+emission_CO2+His+lactate+PO4.apporte+PO4.utilise; 0.768, 0.897, Main.P-Source.Conc.+Nitrogen.Conc.+OD+total.Vol+PL+ emission_CO2+His+lactate+PO4.apporte+PO4.utilise; 0.776, 0. 897, Main.P-Source.Conc.+Nitrogen.Conc.+OD+PL+emission_CO2+ His+lactate+PO4.apporte; 0.759, 0.897, tmp+Main.P-Source.Co nc.+P-Source.Feed.conc.+Nitrogen.Conc.+total.Vol+PL+emissio n_CO2+Thr+His+lactate+PO4.apporte+PO4.utilise; 0.768, 0.896, pH+tmp+Main.P-Source.Conc.+Nitrogen.Conc.+total.Vol+PL+emi ssion_CO2+His+lactate+PO4.apporte; 0.755, 0.896, tmp+Main.P -Source.Conc.+P-Source.Feed.conc.+Nitrogen.Conc.+total.Vol+ PL+emission_CO2+rab+Thr+His+lactate+PO4.ferm.+PO4.apporte; 0.772, 0.896, tmp+P-Source.Feed.conc.+PL+emission_CO2+Thr+H is+lactate+PO4.ferm.+PO4.utilise; 0.759, 0.896, tmp+Main.P-Source.Conc.+Nitrogen.Conc.+OD+total.Vol+PL+emission_O2+emi ssion_CO2+His+lactate+PO4.apporte+PO4.utilise; 0.759, 0.896, tmp+Main.P-Source.Conc.+Nitrogen.Conc.+OD+total.Vol+PL+emi ssion_CO2+rab+His+lactate+PO4.ferm.+PO4.apporte; 0.772, 0.8 96, tmp+Main.P-Source.Conc.+Nitrogen.Conc.+total.Vol+emissi on_O2+emission_CO2+lactate+PO4.apporte+PO4.utilise; 0.754, 0.896, pH+tmp+Main.P-Source.Conc.+Nitrogen.Conc.+total.Vol+ PL+emission_O2+emission_CO2+Thr+His+lactate+PO4.apporte+PO4. utilise; 0.763, 0.896, tmp+Main.P-Source.Conc.+Main.Thr.Con c.+Nitrogen.Conc.+total.Vol+emission_O2+emission_CO2+His+la ctate+PO4.ferm.+PO4.apporte; 0.772, 0.896, tmp+Main.P-Sourc e.Conc.+Nitrogen.Conc.+total.Vol+emission_CO2+rab+lactate+P O4.apporte+PO4.utilise; 0.772, 0.896, tmp+Main.P-Source.Con c.+Nitrogen.Conc.+total.Vol+emission_CO2+His+lactate+PO4.ap porte+PO4.utilise; 0.768, 0.896, tmp+Main.P-Source.Conc.+Ma in.Thr.Conc.+Nitrogen.Conc.+total.Vol+emission_O2+emission CO2+lactate+PO4.ferm.+PO4.apporte; 0.763, 0.896, tmp+Main.P -Source.Conc.+Nitrogen.Conc.+total.Vol+PL+emission_CO2+rab+ Thr+lactate+PO4.ferm.+PO4.apporte; 0.772, 0.896, Main.P-Sou rce.Conc.+Nitrogen.Conc.+OD+yield+PL+emission_CO2+His+lacta te+PO4.apporte; 0.754, 0.896, tmp+Main.P-Source.Conc.+P-Sou rce.Feed.conc.+Nitrogen.Conc.+total.Vol+PL+emission_CO2+rab +Thr+His+lactate+PO4.apporte+PO4.utilise; 0.768, 0.896, Mai n.P-Source.Conc.+Nitrogen.Conc.+total.Vol+yield+PL+emission _CO2+His+lactate+PO4.ferm.+PO4.apporte; 0.763, 0.896, tmp+M ain.P-Source.Conc.+Main.Thr.Conc.+Nitrogen.Conc.+total.Vol+ emission_CO2+rab+His+lactate+PO4.ferm.+PO4.apporte; 0.768, 0.896, Main.P-Source.Conc.+Nitrogen.Conc.+total.Vol+yield+P L+emission_CO2+His+lactate+PO4.apporte+PO4.utilise; 0.767, 0.896, tmp+Main.P-Source.Conc.+Main.Thr.Conc.+Nitrogen.Conc. +total.Vol+emission_CO2+rab+lactate+PO4.ferm.+PO4.apporte; 0.759, 0.896, tmp+Main.P-Source.Conc.+Nitrogen.Conc.+total. Vol+yield+PL+emission_O2+emission_CO2+His+lactate+PO4.appor te+PO4.utilise; 0.759, 0.896, tmp+Main.P-Source.Conc.+Nitro gen.Conc.+total.Vol+yield+PL+emission_O2+emission_CO2+His+l actate+PO4.ferm.+PO4.apporte; 0.767, 0.896, tmp+Main.P-Sour ce.Conc.+Nitrogen.Conc.+OD+yield+PL+emission_CO2+His+lactat e+PO4.apporte; 0.767, 0.896, Main.P-Source.Conc.+Nitrogen.C onc.+total.Vol+PL+emission_CO2+Thr+His+lactate+PO4.ferm.+PO 4.apporte; 0.763, 0.896, tmp+Main.P-Source.Conc.+Nitrogen.C onc.+OD+yield+PL+emission_CO2+His+lactate+PO4.apporte+PO4.u tilise; 0.767, 0.896, tmp+Main.P-Source.Conc.+Main.Thr.Conc. +Nitrogen.Conc.+total.Vol+emission_O2+emission_CO2+lactate+ PO4.apporte+PO4.utilise; 0.763, 0.896, tmp+Main.P-Source.Co nc.+Nitrogen.Conc.+OD+yield+PL+emission_CO2+His+lactate+PO4. ferm.+PO4.apporte; 0.767, 0.896, Main.P-Source.Conc.+P-Sour ce.Feed.conc.+Nitrogen.Conc.+total.Vol+PL+emission_CO2+His+ lactate+PO4.ferm.+PO4.apporte; 0.763, 0.896, tmp+Main.P-Sou rce.Conc.+Main.Thr.Conc.+Nitrogen.Conc.+total.Vol+emission_ O2+emission_CO2+His+lactate+PO4.apporte+PO4.utilise; 0.758, 0.896, tmp+Main.P-Source.Conc.+P-Source.Feed.conc.+Nitroge n.Conc.+PL+emission_O2+emission_CO2+Thr+His+lactate+PO4.app orte+PO4.utilise; 0.758, 0.896, tmp+Main.P-Source.Conc.+Nit rogen.Conc.+OD+total.Vol+PL+emission_CO2+Thr+His+lactate+PO 4.ferm.+PO4.apporte; 0.754, 0.896, pH+tmp+Main.P-Source.Con c.+Nitrogen.Conc.+total.Vol+PL+emission_CO2+rab+Thr+His+lac tate+PO4.ferm.+PO4.apporte; 0.767, 0.896, tmp+Main.P-Source. Conc.+Nitrogen.Conc.+total.Vol+PL+emission_CO2+His+lactate+ PO4.ferm.+PO4.utilise; 0.767, 0.896, Main.P-Source.Cone.+Ni trogen.Conc.+total.Vol+PL+emission_CO2+Thr+His+lactate+PO4. apporte+PO4.utilise; 0.758, 0.896, tmp+Main.P-Source.Conc.+ Nitrogen.Conc.+OD+total.Vol+PL+emission-CO2+rab+His+lactate +PO4.apporte+PO4.utilise; 0.767, 0.896, tmp+Main.P-Source.C onc.+Nitrogen.Conc.+OD+total.Vol+PL+emission_CO2+His+lactat e+PO4.apporte; 0.758, 0.896, tmp+Main.P-Source.Conc.+P-Sour ce.Feed.conc.+Nitrogen.Conc.+PL+emission_O2+emission_CO2+Th r+His+lactate+PO4.ferm.+PO4.apporte; 0.767, 0.896, tmp+Main. P-Source.Conc.+Main.Thr.Conc.+Nitrogen.Conc.+total.Vol+emis sion_CO2+rab+lactate+PO4.apporte+PO4.utilise; 0.767, 0.896, tmp+Main.P-Source.Conc.+Nitrogen.Conc.+total.Vol+emission_ O2+emission_CO2+Thr+lactate+PO4.ferm.+PO4.apporte; 0.758, 0. 896, tmp+Main.P-Source.Conc.+Nitrogen.Conc.+total.Vol+yield +PL+emission_CO2+Thr+His+lactate+PO4.ferm.+PO4.apporte; 0.7 63, 0.896, tmp+Main.P-Source.Conc.+Main.Thr.Conc.+Nitrogen. Conc.+total.Vol+emission_CO2+rab+His+lactate+PO4.apporte+PO 4.utilise; 0.754, 0.896, tmp+Main.P-Source.Conc.+Nitrogen.C onc.+OD+total.Vol+PL+emission_O2+emission_CO2+Thr+His+lacta te+PO4.ferm.+PO4.apporte; 0.767, 0.896, tmp+Main.P-Source.C onc.+Nitrogen.Conc.+total.Vol+PL+emission_O2+emission_CO2+l actate+PO4.ferm.+PO4.apporte; 0.763, 0.896, tmp+Main.P-Sour ce.Conc.+Nitrogen.Conc.+total.Vol+PL+emission_CO2+rab+Thr+l actate+PO4.apporte+PO4.utilise; 0.758, 0.896, tmp+Main.P-So urce.Conc.+P-Source.Feed.conc.+Nitrogen.Conc.+PL+emission_C O2+rab+Thr+His+lactate+PO4.apporte+PO4.utilise; 0.758, 0.89 6, tmp+Main.P-Source.Conc.+Nitrogen.Conc.+total.Vol+yield+P L+emission_CO2+Thr+His+lactate+PO4.apporte+PO4.utilise; 0.7 67, 0.896, Main.P-Source.Conc.+P-Source.Feed.conc.+Nitrogen. Conc.+total.Vol+PL+emission CO2+His+lactate+PO4.apporte+PO4. utilise; 0.758, 0.896, tmp+Main.P-Source.Conc.+Main.Thr.Con c.+Nitrogen.Conc.+total.Vol+PL+emission_O2+emission_CO2+His +lactate+PO4.ferm.+PO4.apporte; 0.758, 0.896, tmp+Main.P-So urce.Conc.+P-Source.Feed.conc.+Nitrogen.Conc.+PL+emission_C O2+rab+Thr+His+lactate+PO4.ferm.+PO4.apporte; 0.758, 0.896, tmp+Main.P-Source.Conc.+Nitrogen.Conc.+total.Vol+yield+PL+ emission_CO2+rab+His+lactate+PO4.apporte+PO4.utilise; 0.758, 0.896, tmp+Main.P-Source.Conc.+Nitrogen.Conc.+total.Vol+yi eld+PL+emission_CO2+rab+His+lactate+PO4.ferm.+PO4.apporte; 0.758, 0.896, tmp+Main.P-Source.Conc.+Nitrogen.Conc.+OD+tot al.Vol+PL+emission_CO2+Thr+His+lactate+PO4.apporte+PO4.util ise; 0.758, 0.896, tmp+Main.P-Source.Conc.+P-Source.Feed.co nc.+Nitrogen.Conc.+total.Vol+PL+emission_O2+emission_CO2+Hi s+lactate+PO4.ferm.+PO4.apporte; 0.753, 0.896, tmp+Main.P-S ource.Conc.+Nitrogen.Conc.+total.Vol+PL+emission_O2+emissio n_CO2+rab+Thr+His+lactate+PO4.ferm.+PO4.apporte; 0.762, 0.8 96, tmp+Main.P-Source.Conc.+P-Source.Feed.conc.+Nitrogen.Co nc.+PL+emission_CO2+Thr+His+lactate+PO4.apporte+PO4.utilis e; 0.758, 0.896, tmp+Main.P-Source.Conc.+Nitrogen.Conc.+Lys _conc.+total.Vol+PL+emission_O2+emission_CO2+His+lactate+PO 4.ferm.+PO4.apporte; 0.767, 0.896, Main.P-Source.Conc.+Nitr ogen.Conc.+total.Vol+PL+emission_O2+emission_CO2+His+lactat e+PO4.ferm.+PO4.apporte; 0.775, 0.896, tmp+Main.P-Source.Co nc.+Nitrogen.Conc.+total.Vol+emission_CO2+His+lactate+PO4.a pporte; 0.758, 0.896, tmp+Main.P-Source.Conc.+Main.Thr.Conc. +Nitrogen.Conc.+total.Vol+PL+emission_O2+emission_CO2+His+l actate+PO4.apporte+PO4.utilise; 0.753, 0.896, pH+tmp+Main.P -Source.Conc.+Nitrogen.Conc.+total.Vol+PL+emission_CO2+rab+ Thr+His+lactate+PO4.apporte+PO4.utilise; 0.767, 0.896, tmp+ Main.P-Source.Conc.+Nitrogen.Conc.+total.Vol+PL+emission_CO 2+rab+lactate+PO4.ferm.+PO4.apporte; 0.758, 0.896, tmp+Main. P-Source.Conc.+Nitrogen.Conc.+total.Vol+PL+emission_O2+emis sion_CO2+His+lactate+SO4.ferm.+PO4.ferm.+PO4.apporte; 0.762, 0.896, tmp+Main.P-Source.Conc.+P-Source.Feed.conc.+Nitroge n.Conc.+total.Vol+PL+emission_CO2+His+lactate+PO4.ferm.+PO4. apporte; 0.762, 0.896, tmp+Main.P-Source.Conc.+P-Source.Fee d.conc.+Nitrogen.Conc.+PL+emission_CO2+Thr+His+lactate+PO4. ferm.+PO4.apporte; 0.767, 0.896, tmp+P-Source.Feed.conc.+PL +emission_O2+emission_CO2+Thr+His+lactate+PO4.apporte+PO4.u tilise; 0.753, 0.896, tmp+Main.P-Source.Conc.+Nitrogen.Conc. +Lys_conc.+total.Vol+PL+emission_O2+emission_CO2+Thr+His+la ctate+PO4.ferm.+PO4.apporte; 0.758, 0.896, tmp+Main.P-Sourc e.Conc.+P-Source.Feed.conc.+Nitrogen.Conc.+total.Vol+PL+emi ssion_CO2+rab+His+lactate+PO4.ferm.+PO4.apporte; 0.762, 0.8 96, tmp+Main.P-Source.Conc.+Nitrogen.Conc.+total.Vol+emissi on_O2+emission_CO2+Thr+His+lactate+PO4.ferm.+PO4.apporte; 0. 753, 0.896, tmp+Main.P-Source.Conc.+Nitrogen.Conc.+OD+total. Vol+PL+emission_O2+emission_CO2+Thr+His+lactate+PO4.apporte +PO4.utilise; 0.766, 0.896, Main.P-Source.Conc.+Nitrogen.Co nc.+total.Vol+PL+emission_O2+emission_CO2+His+lactate+PO4.a pporte+PO4.utilise; 0.770, 0.896, Main.P-Source.Conc.+Nitro gen.Conc.+total.Vol+yield+PL+emission_CO2+His+lactate+PO4.a pporte; 0.757, 0.896, tmp+Main.P-Source.Conc.+Nitrogen.Conc. +total.Vol+PL+emission_CO2+rab+His+lactate+SO4.ferm.+PO4.fe rm.+PO4.apporte; 0.753, 0.896, tmp+Main.P-Source.Conc.+Nitr ogen.Conc.+total.Vol+PL+emission_O2+emission_CO2+rab+Thr+Hi s+lactate+PO4.apporte+PO4.utilise; 0.766, 0.896, tmp+Main.P -Source.Conc.+Nitrogen.Conc.+total.Vol+PL+emission_O2+emiss ion_CO2+lactate+PO4.apporte+PO4.utilise; 0.757, 0.896, tmp+ Main.P-Source.Conc.+Main.Thr.Conc.+Nitrogen.Conc.+total.Vol +PL+emission_CO2+rab+His+lactate+PO4.ferm.+PO4.apporte; 0.7 57, 0.896, tmp+Main.P-Source.Conc.+P-Source.Feed.conc.+Nitr ogen.Conc.+total.Vol+PL+emission_O2+emission_CO2+His+lactat e+PO4.apporte+PO4.utilise; 0.766, 0.896, pH+Main.P-Source.C onc.+Nitrogen.Conc.+OD+total.Vol+PL+emission_CO2+His+lactat e+PO4.apporte; 0.753, 0.896, tmp+Main.P-Source.Conc.+Nitrog en.Conc.+total.Vol+PL+emission_O2+emission_CO2+Thr+His+lact ate+SO4.ferm.+PO4.ferm.+PO4.apporte; 0.762, 0.896, tmp+Main. P-Source.Conc.+P-Source.Feed.conc.+Nitrogen.Conc.+total.Vol +PL+emission_CO2+His+lactate+PO4.apporte+PO4.utilise; 0.762, 0.896, tmp+Main.P-Source.Conc.+Nitrogen.Conc.+total.Vol+PL +emission_CO2+His+lactate+SO4.ferm.+PO4.ferm.+PO4.apporte; 0.766, 0.896, tmp+Main.P-Source.Conc.+Nitrogen.Conc.+total. Vol+emission_CO2+rab+Thr+lactate+PO4.ferm.+PO4.apporte; 0.7 66, 0.896, tmp+Main.P-Source.Conc.+Nitrogen.Conc.+total.Vol +emission_O2+emission_CO2+Thr+lactate+PO4.apporte+PO4.utili se; 0.753, 0.896, tmp+Main.P-Source.Conc.+Nitrogen.Conc.+to tal.Vol+yield+PL+emission_O2+emission_CO2+Thr+His+lactate+P O4.apporte+PO4.utilise; 0.762, 0.896, tmp+Main.P-Source.Con c.+Nitrogen.Conc.+OD+PL+emission_O2+emission_CO2+His+lactat e+PO4.ferm.+PO4.apporte; 0.757, 0.896, tmp+Main.P-Source.Co nc.+Nitrogen.Conc.+total.Vol+PL+emission_O2+emission_CO2+Hi s+lactate+SO4.ferm.+PO4.apporte+PO4.utilise; 0.757, 0.896, tmp+Main.P-Source.Conc.+Nitrogen.Conc.+OD+yield+PL+emission _O2+emission_CO2+His+lactate+PO4.apporte+PO4.utilise; 0.757, 0.896, tmp+Main.P-Source.Conc.+Main.Thr.Conc.+Nitrogen.Con c.+total.Vol+PL+emission CO2+rab+His+lactate+PO4.apporte+PO 4.utilise; 0.766, 0.895, tmp+Main.P-Source.Conc.+Nitrogen.C onc.+total.Vol+PL+emission_CO2+rab+lactate+PO4.apporte+PO4. utilise; 0.757, 0.895, tmp+Main.P-Source.Conc.+P-Source.Fee d.conc.+Nitrogen.Conc.+total.Vol+PL+emission_CO2+rab+His+la ctate+PO4.apporte+PO4.utilise; 0.761, 0.895, tmp+Main.P-Sou rce.Conc.+Nitrogen.Conc.+OD+PL+emission_CO2+Thr+His+lactate +PO4.ferm.+PO4.apporte; 0.761, 0.895, tmp+Main.P-Source.Con c.+Nitrogen.Conc.+OD+PL+emission_CO2+rab+His+lactate+PO4.fe rm.+PO4.apporte; 0.766, 0.895, tmp+P-Source.Feed.conc.+PL+e mission_O2+emission_CO2+Thr+His+lactate+PO4.ferm.+PO4.appor te; 0.761, 0.895, tmp+Main.P-Source.Conc.+Nitrogen.Conc.+to tal.Vol+PL+emission_CO2+His+lactate+SO4.ferm.+PO4.apporte+P O4.utilise; 0.766, 0.895, tmp+Main.P-Source.Conc.+Nitrogen. Conc.+total.Vol+PL+emission_CO2+Thr+lactate+PO4.ferm.+PO4.a pporte; 0.766, 0.895, tmp+Main.P-Source.Conc.+Nitrogen.Conc. +total.Vol+PL+emission_O2+emission_CO2+His+lactate+PO4.appo rte; 0.752, 0.895, tmp+Main.P-Source.Conc.+Nitrogen.Conc.+t otal.Vol+yield+PL+emission_O2+emission_CO2+Thr+His+lactate+ PO4.ferm.+PO4.apporte; 0.757, 0.895, tmp+Main.P-Source.Conc. +Nitrogen.Conc.+Lys_conc.+total.Vol+PL+emission_O2+emission _CO2+His+lactate+PO4.apporte+PO4.utilise; 0.757, 0.895, tmp +Main.P-Source.Conc.+Nitrogen.Conc.+total.Vol+PL+emission_C O2+rab+His+lactate+SO4.ferm.+PO4.apporte+PO4.utilise; 0.766, 0.895, Main.P-Source.Conc.+Nitrogen.Conc.+total.Vol+PL+emi ssion_CO2+rab+His+lactate+PO4.ferm.+PO4.apporte; 0.752, 0.8 95, tmp+Main.P-Source.Conc.+Nitrogen.Conc.+total.Vol+PL+emi ssion_O2+emission_CO2+Thr+His+lactate+SO4.ferm.+PO4.apporte +PO4.utilise; 0.757, 0.895, tmp+Main.P-Source.Conc.+Nitroge n.Conc.+OD+yield+PL+emission_O2+emission_CO2+His+lactate+PO 4.ferm.+PO4.apporte; 0.766, 0.895, tmp+Main.P-Source.Conc.+ Nitrogen.Conc.+total.Vol+PL+emission_CO2+rab+His+lactate+PO 4.apporte; 0.752, 0.895, tmp+Main.P-Source.Conc.+Nitrogen.C onc.+Lys_conc.+total.Vol+PL+emission_O2+emission_CO2+Thr+Hi s+lactate+PO4.apporte+PO4.utilise; 0.761, 0.895, tmp+Main.P -Source.Conc.+Nitrogen.Conc.+total.Vol+emission_CO2+rab+Thr +His+lactate+PO4.ferm.+PO4.apporte; 0.766, 0.895, pH+tmp+Ma in.P-Source.Conc.+Nitrogen.Conc.+OD+PL+emission-CO2+His+lac tate+PO4.apporte; 0.757, 0.895, tmp+Main.P-Source.Conc.+Nit rogen.Conc.+OD+yield+PL+emission_CO2+rab+His+lactate+PO4.ap porte+PO4.utilise; 0.761, 0.895, tmp+Main.P-Source.Conc.+Ni trogen.Conc.+total.Vol+PL+emission_O2+emission_CO2+His+lact ate+PO4.ferm.+PO4.utilise; 0.757, 0.895, tmp+Main.P-Source. Conc.+Nitrogen.Conc.+Lys_conc.+total.Vol+PL+emission_CO2+ra b+His+lactate+PO4.ferm.+PO4.apporte; 0.770, 0.895, Main.P-S ource.Conc.+Nitrogen.Conc.+total.Vol+PL+emission_CO2+Thr+Hi s+lactate+PO4.apporte; 0.761, 0.895, tmp+Main.P-Source.Conc. +Nitrogen.Conc.+OD+PL+emission_CO2+Thr+His+lactate+PO4.appo rte+PO4.utilise; 0.757, 0.895, tmp+Main.P-Source.Conc.+Nitr ogen.Conc.+total.Vol+PL+emission_O2+emission_CO2+rab+His+la ctate+PO4.ferm.+PO4.apporte; 0.778, 0.895, Main.P-Source.Co nc.+Nitrogen.Conc.+total.Vol+emission_CO2+His+lactate+PO4.a pporte; 0.761, 0.895, tmp+Main.P-Source.Conc.+Nitrogen.Conc. +OD+PL+emission_O2+emission_CO2+His+lactate+PO4.apporte+PO4. utilise; 0.757, 0.895, pH+tmp+Main.P-Source.Conc.+Nitrogen. Conc.+OD+total.Vol+PL+emission_CO2+His+lactate+PO4.ferm.+PO 4.apporte; 0.752, 0.895, tmp+Main.P-Source.Conc.+Nitrogen.C onc.+OD+total.Vol+PL+emission_CO2+rab+Thr+His+lactate+PO4.f erm.+PO4.apporte; 0.765, 0.895, tmp+P-Source.Feed.conc.+PL+ emission_CO2+rab+Thr+His+lactate+PO4.apporte+PO4.utilise; 0. 761, 0.895, tmp+Main.P-Source.Conc.+Nitrogen.Conc.+total.Vo l+PL+emission_CO2+rab+His+lactate+PO4.ferm.+PO4.utilise; 0. 765, 0.895, Main.P-Source.Cone.+Nitrogen.Cone.+total.Vol+PL +emission_CO2+rab+His+lactate+PO4.apporte+PO4.utilise; 0.77 7, 0.895, tmp+Main.P-Source.Conc.+Nitrogen.Conc.+total.Vol+ emission_CO2+lactate+PO4.apporte; 0.761, 0.895, tmp+Main.P-Source.Conc.+Nitrogen.Conc.+total.Vol+emission_O2+emission CO2+Thr+His+lactate+PO4.apporte+PO4.utilise; 0.765, 0.895, tmp+Main.P-Source.Conc.+Nitrogen.Conc.+total.Vol+PL+emissio n_CO2+Thr+lactate+PO4.apporte+PO4.utilise; 0.770, 0.895, Ma in.P-Source.Conc.+Nitrogen.Conc.+OD+PL+emission-CO2+His+lac tate+PO4.ferm.+PO4.apporte; 0.756, 0.895, tmp+Main.P-Source. Conc.+Nitrogen.Conc.+OD+yield+PL+emission_CO2+rab+His+lacta te+PO4.ferm.+PO4.apporte; 0.770, 0.895, tmp+Main.P-Source.C onc.+Main.Thr.Conc.+Nitrogen.Conc.+emission_CO2+rab+lactate +PO4.apporte+PO4.utilise; 0.761, 0.895, tmp+Main.P-Source.C onc.+Nitrogen.Conc.+OD+total.Vol+emission_CO2+rab+His+lacta te+PO4.ferm.+PO4.apporte; 0.761, 0.895, tmp+Main.P-Source.C onc.+Nitrogen.Conc.+OD+total.Vol+emission_O2+emission_CO2+H is+lactate+PO4.ferm.+PO4.apporte; 0.761, 0.895, tmp+Main.P-Source.Conc.+Nitrogen.Conc.+OD+PL+emission_CO2+rab+His+lact ate+PO4.apporte+PO4.utilise; 0.769, 0.895, Main.P-Source.Co nc.+Nitrogen.Conc.+total.Vol+PL+emission_O2+emission_CO2+Hi s+lactate+PO4.apporte; 0.769, 0.895, tmp+Main.P-Source.Conc. +Main.Thr.Conc.+Nitrogen.Conc.+emission_CO2+rab+lactate+PO4. ferm.+PO4.apporte; 0.756, 0.895, tmp+Main.P-Source.Conc.+Ni trogen.Conc.+total.Vol+PL+emission_CO2+Thr+His+lactate+SO4. ferm.+PO4.ferm.+PO4.apporte; 0.769, 0.895, Main.P-Source.Co nc.+Nitrogen.Conc.+OD+PL+emission_CO2+His+lactate+PO4.appor te+PO4.utilise; 0.765, 0.895, Main.P-Source.Conc.+Main.Thr. Conc.+Nitrogen.Conc.+total.Vol+PL+emission_CO2+His+lactate+ PO4.ferm.+PO4.apporte; 0.765, 0.895, tmp+Main.P-Source.Conc. +Nitrogen.Conc.+total.Vol+emission_CO2+rab+Thr+lactate+PO4. apporte+PO4.utilise; 0.777, 0.895, Main.P-Source.Conc.+Nitr ogen.Conc.+total.Vol+PL+emission_CO2+lactate+PO4.apporte; 0. 773, 0.895, tmp+Main.P-Source.Conc.+Nitrogen.Conc.+total.Vo l+emission_CO2+lactate+PO4.ferm.+PO4.apporte; 0.769, 0.895, pH+Main.P-Source.Conc.+Nitrogen.Conc.+OD+PL+emission-CO2+H is+lactate+PO4.apporte; 0.761, 0.895, pH+tmp+Main.P-Source. Conc.+Nitrogen.Conc.+OD+yield+PL+emission_CO2+His+lactate+P O4.apporte; 0.769, 0.895, Main.P-Source.Conc.+P-Source.Feed. conc.+Nitrogen.Conc.+total.Vol+PL+emission_CO2+His+lactate+ PO4.apporte; 0.756, 0.895, pH+tmp+Main.P-Source.Conc.+Nitro gen.Conc.+OD+total.Vol+PL+emission_CO2+His+lactate+PO4.appo rte+PO4.utilise; 0.765, 0.895, tmp+Main.P-Source.Conc.+P-So urce.Feed.conc.+Nitrogen.Conc.+PL+emission_C02+Thr+His+lact ate+PO4.apporte; 0.761, 0.895, pH+tmp+Main.P-Source.Conc.+N itrogen.Conc.+OD+PL+emission_CO2+His+lactate+PO4.ferm.+PO4. apporte; 0.781, 0.895, Main.P-Source.Conc.+Nitrogen.Conc.+t otal.Vol+emission_CO2+lactate+PO4.apporte; 0.751, 0.895, tm p+Main.P-Source.Conc.+Nitrogen.Conc.+total.Vol+PL+emission-CO2+rab+Thr+His+lactate+SO4.ferm.+PO4.ferm.+PO4.apporte; 0. 765, 0.895, tmp+P-Source.Feed.conc.+PL+emission_O2+emission _CO2+Thr+His+lactate+PO4.ferm.+PO4.utilise; 0.765, 0.895, M ain.P-Source.Conc.+Main.Thr.Conc.+Nitrogen.Conc.+total.Vol+ PL+emission_CO2+His+lactate+PO4.apporte+PO4.utilise; 0.756, 0.895, tmp+Main.P-Source.Conc.+Nitrogen.Conc.+total.Vol+PL +emission_O2+emission_CO2+rab+His+lactate+PO4.apporte+PO4.u tilise; 0.756, 0.895, tmp+Main.P-Source.Conc.+Nitrogen.Conc. +total.Vol+PL+emission_CO2+Thr+His+lactate+SO4.ferm.+PO4.ap porte+PO4.utilise; 0.751, 0.895, tmp+Main.P-Source.Conc.+Ni trogen.Conc.+OD+total.Vol+PL+emission_CO2+rab+Thr+His+lacta te+PO4.apporte+PO4.utilise; 0.769, 0.895, Main.P-Source.Con c.+Nitrogen.Conc.+total.Vol+PL+emission_CO2+His+lactate+PO4. ferm.+PO4.utilise; 0.765, 0.895, Main.P-Source.Conc.+Nitrog en.Conc.+OD+yield+PL+emission-CO2+His+lactate+PO4.apporte+P O4.utilise; 0.751, 0.895, tmp+Main.P-Source.Conc.+Nitrogen. Conc.+total.Vol+yield+PL+emission_CO2+rab+Thr+His+lactate+P O4.apporte+PO4.utilise; 0.765, 0.895, tmp+P-Source.Feed.con c.+PL+emission_CO2+rab+Thr+His+lactate+PO4.ferm.+PO4.apport e; 0.765, 0.895, Main.P-Source.Conc.+Nitrogen.Conc.+OD+yiel d+PL+emission_CO2+His+lactate+PO4.ferm.+PO4.apporte; 0.760, 0.895, pH+tmp+Main.P-Source.Conc.+Nitrogen.Conc.+total.Vol +PL+emission_CO2+Thr+His+lactate+PO4.apporte; 0.765, 0.895, pH+Main.P-Source.Conc.+Nitrogen.Conc.+OD+yield+PL+emission _CO2+His+lactate+PO4.apporte; 0.760, 0.895, pH+tmp+Main.P-S ource.Conc.+Nitrogen.Conc.+OD+PL+emission_CO2+His+lactate+P O4.apporte+PO4.utilise; 0.751, 0.895, tmp+Main.P-Source.Con c.+Nitrogen.Conc.+total.Vol+yield+PL+emission_CO2+rab+Thr+H is+lactate+PO4.ferm.+PO4.apporte; 0.760, 0.895, tmp+Main.P-Source.Conc.+Nitrogen.Conc.+total.Vol+emission_CO2+rab+Thr+ His+lactate+PO4.apporte+PO4.utilise; 0.756, 0.895, tmp+Main. P-Source.Conc.+Nitrogen.Conc.+Lys-conc.+total.Vol+PL+emissi on_CO2+rab+His+lactate+PO4.apporte+PO4.utilise; 0.760, 0.89 5, pH+tmp+Main.P-Source.Conc.+Nitrogen.Conc.+total.Vol+emis sion_CO2+rab+His+lactate+PO4.ferm.+PO4.apporte; 0.760, 0.89 5, tmp+Main.P-Source.Conc.+Nitrogen.Conc.+OD+total.Vol+emis sion_CO2+rab+His+lactate+PO4.apporte+PO4.utilise; 0.773, 0. 895, tmp+Main.P-Source.Conc.+Nitrogen.Conc.+total.Vol+emiss ion_CO2+lactate+PO4.apporte+PO4.utilise; 0.760, 0.895, pH+t mp+Main.P-Source.Cone.+Nitrogen.Cone.+OD+total.Vol+PL+emiss ion_CO2+His+lactate+PO4.apporte; 0.760, 0.895, tmp+Main.P-S ource.Conc.+Nitrogen.Conc.+OD+total.Vol+emission_O2+emissio n_CO2+His+lactate+PO4.apporte+PO4.utilise; 0.751, 0.895, tm p+Main.P-Source.Conc.+Nitrogen.Conc.+Lys_conc.+total.Vol+PL +emission_CO2+rab+Thr+His+lactate+PO4.ferm.+PO4.apporte; 0. 751, 0.895, tmp+Main.P-Source.Conc.+Nitrogen.Conc.+total.Vo l+PL+emission_CO2+rab+Thr+His+lactate+SO4.ferm.+PO4.apporte +PO4.utilise; 0.760, 0.895, pH+Main.P-Source.Conc.+Nitrogen. Conc.+OD+total.Vol+PL+emission_CO2+His+lactate+PO4.ferm.+PO 4.apporte; 0.764, 0.895, tmp+Main.P-Source.Conc.+Nitrogen.C onc.+OD+PL+emission-CO2+Thr+His+lactate+PO4.apporte; 0.760, 0.895, tmp+Main.P-Source.Conc.+Nitrogen.Conc.+total.Vol+PL +emission_CO2+Thr+His+lactate+PO4.ferm.+PO4.utilise; 0.769, 0.895, Main.P-Source.Conc.+Nitrogen.Conc.+total.Vol+PL+emi ssion_CO2+rab+His+lactate+PO4.apporte; 0.760, 0.895, pH+tmp +Main.P-Source.Conc.+Nitrogen.Conc.+total.Vol+emission_O2+e mission_CO2+His+lactate+PO4.ferm.+PO4.apporte; 0.768, 0.895, tmp+Main.P-Source.Conc.+Nitrogen.Conc.+total.Vol+PL+emissi on_CO2+lactate+PO4.ferm.+PO4.apporte; 0.768, 0.895, pH+tmp+ P-Source.Feed.conc.+PL+emission_CO2+Thr+His+lactate+PO4.app orte; 0.760, 0.895, tmp+Main.P-Source.Conc.+Nitrogen.Conc.+ total.Vol+PL+emission_O2+rab+His+lactate+PO4.ferm.+PO4.appo rte; 0.760, 0.895, tmp+Main.P-Source.Conc.+P-Source.Feed.co nc.+Nitrogen.Conc.+total.Vol+PL+emission_CO2+Thr+His+lactat e+PO4.apporte; 0.768, 0.895, tmp+Main.P-Source.Conc.+Main.T hr.Conc.+Nitrogen.Conc.+emission_O2+emission CO2+lactate+PO 4.apporte+PO4.utilise; 0.755, 0.895, pH+tmp+Main.P-Source.C onc.+Nitrogen.Conc.+total.Vol+yield+PL+emission_CO2+His+lac tate+PO4.ferm.+PO4.apporte; 0.764, 0.895, Main.P-Source.Con c.+Nitrogen.Conc.+OD+total.Vol+yield+PL+emission_CO2+His+la ctate+PO4.apporte; 0.760, 0.895, tmp+Main.P-Source.Conc.+Ma in.Thr.Conc.+Nitrogen.Conc.+total.Vol+PL+emission_CO2+His+l actate+PO4.ferm.+PO4.apporte; 0.760, 0.895, pH+Main.P-Sourc e.Conc.+Nitrogen.Conc.+OD+total.Vol+PL+emission_CO2+His+lac tate+PO4.apporte+PO4.utilise; 0.772, 0.895, Main.P-Source.C onc.+Nitrogen.Conc.+total.Vol+emission_CO2+His+lactate+PO4. ferm.+PO4.apporte; 0.768, 0.895, tmp+Main.P-Source.Conc.+Ni trogen.Conc.+total.Vol+emission_CO2+His+lactate+PO4.ferm.+P O4.utilise; 0.768, 0.895, tmp+Main.P-Source.Conc.+Main.Thr. Conc.+Nitrogen.Conc.+emission_O2+emission_CO2+lactate+PO4.f erm.+PO4.apporte; 0.750, 0.895, pH+tmp+Main.P-Source.Conc.+ Nitrogen.Cone.+OD+total.Vol+PL+emission_02+emission_C02+His +lactate+PO4.ferm.+PO4.apporte; 0.764, 0.895, tmp+P-Source. Feed.conc.+PL+emission_CO2+rab+Thr+His+lactate+PO4.ferm.+PO 4.utilise; 0.760, 0.895, tmp+Main.P-Source.Conc.+Nitrogen.C onc.+Lys_conc.+total.Vol+PL+emission_CO2+His+lactate+PO4.fe rm.+PO4.apporte; 0.750, 0.895, tmp+Main.P-Source.Conc.+Nitr ogen.Conc.+OD+Lys_conc.+total.Vol+PL+emission_O2+emission_C O2+His+lactate+PO4.ferm.+PO4.apporte; 0.755, 0.895, pH+tmp+ Main.P-Source.Conc.+Nitrogen.Conc.+total.Vol+yield+PL+emiss ion_CO2+His+lactate+PO4.apporte+PO4.utilise; 0.768, 0.895, tmp+Main.P-Source.Conc.+Nitrogen.Conc.+total.Vol+PL+emissio n_CO2+lactate+PO4.apporte+PO4.utilise; 0.772, 0.895, tmp+Ma in.P-Source.Conc.+Nitrogen.Conc.+total.Vol+emission_CO2+rab +lactate+PO4.apporte; 0.750, 0.895, tmp+Main.P-Source.Conc. +Main.Thr.Conc.+Nitrogen.Conc.+total.Vol+PL+emission_O2+emi ssion_CO2+Thr+His+lactate+PO4.ferm.+PO4.apporte; 0.764, 0.8 95, tmp+Main.P-Source.Conc.+Nitrogen.Conc.+OD+total.Vol+emi ssion_CO2+rab+lactate+PO4.ferm.+PO4.apporte; 0.764, 0.894, tmp+Main.P-Source.Cone.+Nitrogen.Cone.+total.Vol+emission_C O2+Thr+His+lactate+PO4.ferm.+PO4.apporte; 0.764, 0.894, tmp +Main.P-Source.Conc.+Nitrogen.Conc.+total.Vol+yield+PL+emis sion_CO2+His+lactate+PO4.apporte; 0.764, 0.894, tmp+Main.P-Source.Conc.+Nitrogen.Conc.+OD+total.Vol+emission_O2+emissi on_CO2+lactate+PO4.ferm.+PO4.apporte; 0.764, 0.894, tmp+Mai n.P-Source.Conc.+Nitrogen.Conc.+total.Vol+emission_CO2+rab+ His+lactate+PO4.ferm.+PO4.utilise; 0.759, 0.894, tmp+Main.P -Source.Conc.+Nitrogen.Conc.+total.Vol+PL+emission_O2+emiss ion_CO2+Thr+His+lactate+PO4.apporte; 0.759, 0.894, tmp+Main. P-Source.Conc.+Nitrogen.Conc.+OD+total.Vol+PL+emission_CO2+ Thr+His+lactate+PO4.apporte; 0.759, 0.894, tmp+Main.P-Sourc e.Conc.+Main.Thr.Conc.+Nitrogen.Conc.+total.Vol+PL+emission _CO2+His+lactate+PO4.apporte+PO4.utilise; 0.759, 0.894, tmp +Main.P-Source.Conc.+Nitrogen.Conc.+total.Vol+PL+emission_O 2+rab+His+lactate+PO4.apporte+PO4.utilise; 0.755, 0.894, tm p+Main.P-Source.Conc.+Nitrogen.Conc.+OD+total.Vol+yield+PL+ emission_CO2+His+lactate+PO4.ferm.+PO4.apporte; 0.772, 0.89 4, tmp+Main.P-Source.Conc.+Nitrogen.Conc.+total.Vol+emissio n_O2+emission_CO2+lactate+PO4.apporte; 0.764, 0.894, tmp+P-Source.Feed.conc.+total.Vol+PL+emission_CO2+Thr+His+lactate +PO4.apporte+PO4.utilise; 0.755, 0.894, tmp+Main.P-Source.C onc.+Nitrogen.Conc.+OD+PL+emission_O2+emission_CO2+Thr+His+ lactate+PO4.ferm.+PO4.apporte; 0.763, 0.894, tmp+Main.P-Sou rce.Conc.+Nitrogen.Conc.+total.Vol+emission_O2+emission_CO2 +His+lactate+PO4.ferm.+PO4.utilise; 0.750, 0.894, tmp+Main. P-Source.Conc.+Nitrogen.Conc.+Lys_conc.+total.Vol+PL+emissi on_CO2+rab+Thr+His+lactate+PO4.apporte+PO4.utilise; 0.755, 0.894, tmp+Main.P-Source.Conc.+Nitrogen.Conc.+OD+total.Vol+ yield+PL+emission_CO2+His+lactate+PO4.apporte+PO4.utilise; 0.755, 0.894, tmp+Main.P-Source.Conc.+Nitrogen.Conc.+OD+yie ld+PL+emission CO2+Thr+His+lactate+PO4.apporte+PO4.utilise; 0.755, 0.894, tmp+Main.P-Source.Conc.+Main.Thr.Conc.+Nitro gen.Conc.+total.Vol+PL+emission_CO2+Thr+His+lactate+PO4.fer m.+PO4.apporte; 0.772, 0.894, Main.P-Source.Conc.+Nitrogen. Conc.+total.Vol+emission CO2+His+lactate+PO4.apporte+PO4.ut ilise; 0.759, 0.894, pH+tmp+Main.P-Source.Conc.+Nitrogen.Co nc.+total.Vol+emission_CO2+rab+His+lactate+PO4.apporte+PO4. utilise; 0.755, 0.894, pH+tmp+Main.P-Source.Conc.+Nitrogen. Conc.+OD+yield+PL+emission_CO2+His+lactate+PO4.apporte+PO4. utilise; 0.755, 0.894, pH+tmp+Main.P-Source.Conc.+Nitrogen. Conc.+OD+yield+PL+emission_CO2+His+lactate+PO4.ferm.+PO4.ap porte; 0.755, 0.894, pH+tmp+Main.P-Source.Conc.+Nitrogen.Co nc.+total.Vol+PL+emission_O2+emission_CO2+Thr+lactate+PO4.f erm.+PO4.apporte; 0.750, 0.894, tmp+Main.P-Source.Conc.+Mai n.Thr.Conc.+Nitrogen.Conc.+total.Vol+PL+emission_O2+emissio n_CO2+Thr+His+lactate+PO4.apporte+PO4.utilise; 0.768, 0.894, tmp+Main.P-Source.Conc.+Nitrogen.Conc.+total.Vol+PL+emissi on_CO2+Thr+lactate+PO4.apporte; 0.772, 0.894, Main.P-Source. Conc.+Nitrogen.Conc.+total.Vol+PL+emission_CO2+lactate+PO4. ferm.+PO4.apporte; 0.750, 0.894, pH+tmp+Main.P-Source.Conc. +Nitrogen.Conc.+OD+total.Vol+PL+emission_CO2+rab+His+lactat e+PO4.ferm.+PO4.apporte; 0.754, 0.894, tmp+Main.P-Source.Co nc.+Nitrogen.Conc.+OD+yield+PL+emission CO2+Thr+His+lactate +PO4.ferm.+PO4.apporte; 0.759, 0.894, pH+tmp+Main.P-Source. Conc.+Nitrogen.Conc.+total.Vol+PL+emission_O2+emission_CO2+ His+lactate+PO4.apporte; 0.754, 0.894, tmp+Main.P-Source.Co nc.+Nitrogen.Conc.+Lys_conc.+total.Vol+PL+emission_CO2+Thr+ His+lactate+PO4.ferm.+PO4.apporte; 0.768, 0.894, tmp+P-Sour ce.Feed.conc.+PL+emission_O2+emission_CO2+Thr+His+lactate+P O4.apporte; 0.759, 0.894, tmp+Main.P-Source.Conc.+Nitrogen. Conc.+Lys_conc.+total.Vol+PL+emission_CO2+His+lactate+PO4.a pporte+PO4.utilise; 0.759, 0.894, pH+tmp+Main.P-Source.Conc. +Nitrogen.Conc.+total.Vol+emission_O2+emission_CO2+His+lact ate+PO4.apporte+PO4.utilise; 0.759, 0.894, tmp+Main.P-Sourc e.Conc.+Nitrogen.Conc.+total.Vol+yield+emission_O2+emission _CO2+His+lactate+PO4.ferm.+PO4.apporte; 0.772, 0.894, tmp+M ain.P-Source.Conc.+Nitrogen.Conc.+total.Vol+PL+emission_CO2 +lactate+PO4.apporte; 0.759, 0.894, tmp+Main.P-Source.Conc. +Nitrogen.Conc.+total.Vol+yield+emission_CO2+rab+His+lactat e+PO4.ferm.+PO4.apporte; 0.759, 0.894, pH+tmp+Main.P-Source. Conc.+Nitrogen.Conc.+total.Vol+PL+emission_CO2+rab+His+lact ate+PO4.apporte; 0.763, 0.894, pH+tmp+Main.P-Source.Conc.+N itrogen.Conc.+total.Vol+emission_CO2+His+lactate+PO4.ferm.+ PO4.apporte; 0.750, 0.894, pH+tmp+Main.P-Source.Conc.+Nitro gen.Conc.+OD+total.Vol+PL+emission_O2+emission_CO2+His+lact ate+PO4.apporte+PO4.utilise; 0.763, 0.894, pH+Main.P-Source. Conc.+Nitrogen.Conc.+total.Vol+yield+PL+emission_CO2+His+la ctate+PO4.apporte; 0.745, 0.894, pH+tmp+Main.P-Source.Conc. +P-Source.Feed.conc.+Nitrogen.Conc.+total.Vol+PL+emission_O 2+emission_CO2+Thr+His+lactate+PO4.ferm.+PO4.apporte; 0.759, 0.894, pH+Main.P-Source.Conc.+Nitrogen.Conc.+total.Vol+yie ld+PL+emission_CO2+His+lactate+PO4.ferm.+PO4.apporte; 0.750, 0.894, pH+tmp+Main.P-Source.Conc.+P-Source.Feed.conc.+Nitr ogen.Conc.+total.Vol+PL+emission_CO2+Thr+His+lactate+PO4.fe rm.+PO4.apporte; 0.763, 0.894, tmp+P-Source.Feed.conc.+tota l.Vol+PL+emission_CO2+Thr+His+lactate+PO4.ferm.+PO4.apport e; 0.763, 0.894, pH+tmp+Main.P-Source.Conc.+Nitrogen.Conc.+ total.Vol+emission_CO2+rab+lactate+PO4.ferm.+PO4.apporte; 0. 771, 0.894, Main.P-Source.Conc.+Nitrogen.Conc.+total.Vol+PL +emission_CO2+lactate+PO4.apporte+PO4.utilise; 0.759, 0.894, pH+Main.P-Source.Conc.+Nitrogen.Conc.+total.Vol+yield+PL+e mission_CO2+His+lactate+PO4.apporte+PO4.utilise; 0.767, 0.8 94, pH+tmp+Main.P-Source.Conc.+Nitrogen.Conc.+total.Vol+emi ssion_CO2+His+lactate+PO4.apporte; 0.749, 0.894, pH+tmp+Mai n.P-Source.Conc.+Nitrogen.Conc.+OD+total.Vol+PL+emission_CO 2+Thr+His+lactate+PO4.ferm.+PO4.apporte; 0.763, 0.894, pH+t mp+P-Source.Feed.conc.+PL+emission_CO2+Thr+His+lactate+PO4. apporte+PO4.utilise; 0.749, 0.894, tmp+Main.P-Source.Conc.+ Nitrogen.Conc.+OD+total.Vol+yield+PL+emission_O2+emission_C O2+His+lactate+PO4.apporte+PO4.utilise; 0.763, 0.894, pH+tm p+Main.P-Source.Conc.+Nitrogen.Conc.+total.Vol+emission_O2+ emission_CO2+lactate+PO4.ferm.+PO4.apporte; 0.763, 0.894, t mp+Main.P-Source.Conc.+Nitrogen.Conc.+OD+total.Vol+emission _CO2+rab+lactate+PO4.apporte+PO4.utilise; 0.754, 0.894, tmp +Main.P-Source.Conc.+Nitrogen.Conc.+OD+PL+emission_O2+emiss ion_CO2+Thr+His+lactate+PO4.apporte+PO4.utilise; 0.754, 0.8 94, tmp+Main.P-Source.Conc.+Nitrogen.Conc.+total.Vol+PL+emi ssion_O2+emission_CO2+Thr+His+lactate+PO4.ferm.+PO4.utilis e; 0.759, 0.894, tmp+Main.P-Source.Conc.+Nitrogen.Conc.+tot al.Vol+PL+emission_CO2+rab+Thr+His+lactate+PO4.apporte; 0.7 63, 0.894, tmp+Main.P-Source.Conc.+Nitrogen.Conc.+OD+total. Vol+emission_O2+emission_CO2+lactate+PO4.apporte+PO4.utilis e; 0.749, 0.894, pH+tmp+Main.P-Source.Conc.+P-Source.Feed.c onc.+Nitrogen.Conc.+total.Vol+PL+emission_CO2+Thr+His+lacta te+PO4.apporte+PO4.utilise; 0.754, 0.894, tmp+Main.P-Source. Conc.+Main.Thr.Conc.+Nitrogen.Conc.+total.Vol+PL+emission_C O2+Thr+His+lactate+PO4.apporte+PO4.utilise; 0.763, 0.894, t mp+Main.P-Source.Conc.+Nitrogen.Conc.+total.Vol+emission_CO 2+Thr+His+lactate+PO4.apporte+PO4.utilise; 0.749, 0.894, tm p+Main.P-Source.Conc.+Nitrogen.Conc.+OD+total.Vol+yield+PL+ emission_O2+emission_CO2+His+lactate+PO4.ferm.+PO4.apporte; 0.759, 0.894, tmp+Main.P-Source.Conc.+Nitrogen.Conc.+total. Vol+yield+emission_O2+emission_CO2+His+lactate+PO4.apporte+ PO4.utilise; 0.767, 0.894, Main.P-Source.Conc.+Main.Thr.Con c.+Nitrogen.Conc.+total.Vol+PL+emission_CO2+His+lactate+PO4. apporte; 0.758, 0.894, tmp+Main.P-Source.Conc.+Nitrogen.Con c.+total.Vol+yield+emission_CO2+rab+His+lactate+PO4.apporte +PO4.utilise; 0.758, 0.894, Main.P-Source.Conc.+Nitrogen.Co nc.+OD+total.Vol+yield+PL+emission_CO2+His+lactate+PO4.ferm. +PO4.apporte; 0.767, 0.894, tmp+Main.P-Source.Conc.+Nitroge n.Conc.+total.Vol+emission_CO2+rab+His+lactate+PO4.apporte; 0.744, 0.894, pH+tmp+Main.P-Source.Conc.+P-Source.Feed.con c.+Nitrogen.Conc.+total.Vol+PL+emission_O2+emission_CO2+Thr +His+lactate+PO4.apporte+PO4.utilise; 0.758, 0.894, Main.P-Source.Conc.+Nitrogen.Conc.+OD+total.Vol+yield+PL+emission-CO2+His+lactate+PO4.apporte+PO4.utilise; 0.754, 0.894, tmp+ Main.P-Source.Conc.+Nitrogen.Conc.+Lys_conc.+total.Vol+PL+e mission_CO2+Thr+His+lactate+PO4.apporte+PO4.utilise; 0.763, 0.894, tmp+Main.P-Source.Conc.+Main.Thr.Conc.+Nitrogen.Con c.+emission_CO2+rab+His+lactate+PO4.apporte+PO4.utilise; 0. 754, 0.894, pH+tmp+Main.P-Source.Conc.+Nitrogen.Conc.+total. Vol+PL+emission_O2+emission_CO2+Thr+lactate+PO4.apporte+PO4. utilise; 0.767, 0.894, tmp+P-Source.Feed.conc.+total.Vol+PL +emission_CO2+Thr+His+lactate+PO4.apporte; 0.763, 0.894, tm p+Main.P-Source.Conc.+Nitrogen.Conc.+OD+total.Vol+emission_ CO2+His+lactate+PO4.ferm.+PO4.apporte; 0.758, 0.894, pH+tmp +Main.P-Source.Conc.+Nitrogen.Conc.+total.Vol+PL+emission_C O2+His+lactate+PO4.ferm.+PO4.utilise; 0.763, 0.894, tmp+Mai n.P-Source.Conc.+P-Source.Feed.conc.+Nitrogen.Conc.+total.V ol+PL+emission_CO2+His+lactate+PO4.apporte; 0.749, 0.894, t mp+Main.P-Source.Conc.+Nitrogen.Conc.+OD+Lys_conc.+total.Vo l+PL+emission_O2+emission_CO2+His+lactate+PO4.apporte+PO4.u tilise; 0.771, 0.894, tmp+Main.P-Source.Conc.+Nitrogen.Conc. +emission_CO2+rab+lactate+PO4.ferm.+PO4.apporte; 0.758, 0.8 94, tmp+Main.P-Source.Conc.+Nitrogen.Conc.+OD+total.Vol+emi ssion_O2+emission_CO2+Thr+lactate+PO4.ferm.+PO4.apporte; 0. 763, 0.894, tmp+Main.P-Source.Conc.+Main.Thr.Conc.+Nitrogen. Conc.+emission_CO2+rab+His+lactate+PO4.ferm.+PO4.apporte; 0. 767, 0.894, tmp+P-Source.Feed.conc.+PL+emission_CO2+rab+Thr +His+lactate+PO4.apporte; 0.758, 0.894, pH+Main.P-Source.Co nc.+P-Source.Feed.conc.+Nitrogen.Conc.+total.Vol+PL+emissio n_CO2+His+lactate+PO4.ferm.+PO4.apporte; 0.763, 0.894, pH+t mp+P-Source.Feed.conc.+PL+emission_CO2+Thr+His+lactate+PO4. ferm.+PO4.apporte; 0.767, 0.894, tmp+Main.P-Source.Conc.+Ni trogen.Conc.+total.Vol+emission_O2+emission_CO2+His+lactate +PO4.apporte; 0.749, 0.894, pH+tmp+Main.P-Source.Conc.+Nitr ogen.Conc.+OD+total.Vol+PL+emission_CO2+Thr+His+lactate+PO4. apporte+PO4.utilise; 0.767, 0.894, tmp+Main.P-Source.Conc.+ Nitrogen.Conc.+total.Vol+emission_CO2+Thr+lactate+PO4.ferm. +PO4.apporte; 0.763, 0.894, tmp+P-Source.Feed.conc.+total.V ol+PL+emission_CO2+Thr+His+lactate+PO4.ferm.+PO4.utilise; 0. 758, 0.894, tmp+Main.P-Source.Conc.+P-Source.Feed.conc.+Nit rogen.Conc.+total.Vol+emission_CO2+rab+His+lactate+PO4.ferm. +PO4.apporte; 0.763, 0.894, Main.P-Source.Conc.+Nitrogen.Co nc.+total.Vol+PL+emission_CO2+His+lactate+SO4.ferm.+PO4.fer m.+PO4.apporte; 0.749, 0.894, pH+tmp+Main.P-Source.Conc.+Ni trogen.Conc.+total.Vol+yield+PL+emission_CO2+Thr+His+lactat e+PO4.ferm.+PO4.apporte; 0.749, 0.894, pH+tmp+Main.P-Source. Conc.+Nitrogen.Conc.+Lys_conc.+total.Vol+PL+emission_O2+emi ssion_CO2+His+lactate+PO4.ferm.+PO4.apporte; 0.749, 0.894, pH+tmp+Main.P-Source.Conc.+Nitrogen.Conc.+OD+total.Vol+PL+e mission_CO2+rab+His+lactate+PO4.apporte+PO4.utilise; 0.754, 0.894, tmp+Main.P-Source.Conc.+P-Source.Feed.conc.+Nitroge n.Conc.+total.Vol+PL+emission_O2+emission_CO2+Thr+lactate+P O4.ferm.+PO4.apporte; 0.754, 0.894, tmp+Main.P-Source.Conc. +Nitrogen.Conc.+OD+PL+emission_CO2+rab+Thr+His+lactate+PO4. ferm.+PO4.apporte; 0.754, 0.894, tmp+Main.P-Source.Conc.+P-Source.Feed.conc.+Nitrogen.Conc.+OD+PL+emission CO2+Thr+His +lactate+P04.apporte+P04.utilise; 0.754, 0.894, tmp+Main.P-Source.Conc.+P-Source.Feed.conc.+Nitrogen.Conc.+OD+PL+emiss ion_CO2+Thr+His+lactate+PO4.ferm.+PO4.apporte; 0.758, 0.894, tmp+Main.P-Source.Conc.+P-Source.Feed.conc.+Nitrogen.Conc. +total.Vol+emission_O2+emission_CO2+His+lactate+PO4.ferm.+P O4.apporte; 0.749, 0.894, pH+tmp+Main.P-Source.Conc.+Nitrog en.Conc.+total.Vol+yield+PL+emission_CO2+Thr+His+lactate+PO 4.apporte+PO4.utilise; 0.762, 0.894, pH+tmp+Main.P-Source.C onc.+Nitrogen.Conc.+total.Vol+emission_CO2+His+lactate+PO4. apporte+PO4.utilise; 0.762, 0.894, tmp+Main.P-Source.Conc.+ Nitrogen.Conc.+OD+PL+emission_O2+emission_CO2+His+lactate+P O4.apporte; 0.762, 0.894, tmp+Main.P-Source.Conc.+Nitrogen. Conc.+OD+PL+emission_CO2+rab+His+lactate+PO4.apporte; 0.758, 0.894, tmp+Main.P-Source.Conc.+Nitrogen.Conc.+OD+yield+PL+ emission_CO2+Thr+His+lactate+PO4.apporte; 0.762, 0.894, Mai n.P-Source.Conc.+Nitrogen.Conc.+total.Vol+PL+emission_CO2+H is+lactate+SO4.ferm.+PO4.apporte+PO4.utilise; 0.749, 0.894, tmp+Main.P-Source.Conc.+Main.Thr.Conc.+Nitrogen.Conc.+tota l.Vol+PL+emission_CO2+rab+Thr+His+lactate+PO4.ferm.+PO4.app orte; 0.758, 0.894, pH+Main.P-Source.Conc.+P-Source.Feed.co nc.+Nitrogen.Conc.+total.Vol+PL+emission_CO2+His+lactate+PO 4.apporte+PO4.utilise; 0.749, 0.894, tmp+Main.P-Source.Conc. +Nitrogen.Conc.+OD+Lys-conc.+total.Vol+PL+emission_CO2+rab+ His+lactate+PO4.ferm.+PO4.apporte; 0.762, 0.894, pH+tmp+Mai n.P-Source.Conc.+Nitrogen.Conc.+total.Vol+emission_CO2+rab+ lactate+PO4.apporte+PO4.utilise; 0.758, 0.894, pH+Main.P-So urce.Conc.+Nitrogen.Conc.+total.Vol+PL+emission_CO2+Thr+His +lactate+PO4.ferm.+PO4.apporte; 0.749, 0.894, pH+tmp+Main.P -Source.Conc.+Nitrogen.Conc.+total.Vol+yield+PL+emission_O2 +emission_CO2+His+lactate+PO4.apporte+PO4.utilise; 0.753, 0. 894, pH+tmp+Main.P-Source.Conc.+Nitrogen.Conc.+OD+PL+emissi on_CO2+Thr+His+lactate+PO4.ferm.+PO4.apporte; 0.748, 0.894, tmp+Main.P-Source.Conc.+Nitrogen.Conc.+OD+total.Vol+yield+ PL+emission_CO2+rab+His+lactate+PO4.apporte+PO4.utilise; 0. 748, 0.894, pH+tmp+Main.P-Source.Conc.+Nitrogen.Conc.+total. Vol+yield+PL+emission_O2+emission_CO2+His+lactate+PO4.ferm. +PO4.apporte; 0.758, 0.894, tmp+Main.P-Source.Conc.+Nitroge n.Conc.+total.Vol+emission_O2+emission_CO2+rab+His+lactate+ PO4.ferm.+PO4.apporte; 0.753, 0.894, pH+tmp+Main.P-Source.C onc.+Nitrogen.Conc.+total.Vol+PL+emission_CO2+rab+Thr+lacta te+PO4.ferm.+PO4.apporte; 0.753, 0.894, tmp+Main.P-Source.C onc.+Nitrogen.Conc.+total.Vol+PL+emission_CO2+rab+Thr+His+l actate+PO4.ferm.+PO4.utilise; 0.771, 0.894, tmp+Main.P-Sour ce.Conc.+Nitrogen.Conc.+emission_CO2+rab+lactate+PO4.apport e+PO4.utilise; 0.748, 0.894, tmp+Main.P-Source.Conc.+Nitrog en.Conc.+OD+total.Vol+yield+PL+emission_CO2+rab+His+lactate +PO4.ferm.+PO4.apporte; 0.744, 0.894, tmp+Main.P-Source.Con c.+Nitrogen.Conc.+OD+Lys_conc.+total.Vol+PL+emission_O2+emi ssion CO2+Thr+His+lactate+PO4.ferm.+PO4.apporte; 0.762, 0.8 94, pH+tmp+Main.P-Source.Conc.+Nitrogen.Conc.+total.Vol+emi ssion_O2+emission_CO2+lactate+PO4.apporte+PO4.utilise; 0.76 2, 0.894, tmp+Main.P-Source.Conc.+Nitrogen.Conc.+total.Vol+ yield+emission CO2+His+lactate+PO4.ferm.+PO4.apporte; 0.758, 0.894, pH+tmp+Main.P-Source.Conc.+Main.Thr.Conc.+Nitrogen. Conc.+total.Vol+emission_O2+emission_CO2+lactate+PO4.ferm.+ PO4.apporte; 0.758, 0.894, tmp+Main.P-Source.Conc.+Nitrogen. Conc.+OD+yield+PL+emission_O2+emission_CO2+His+lactate+PO4. apporte; 0.753, 0.894, tmp+Main.P-Source.Conc.+P-Source.Fee d.conc.+Nitrogen.Conc.+total.Vol+PL+emission_O2+emission_CO 2+Thr+lactate+PO4.apporte+PO4.utilise; 0.758, 0.894, tmp+Ma in.P-Source.Conc.+Main.Thr.Conc.+Nitrogen.Conc.+total.Vol+e mission_O2+emission_CO2+Thr+lactate+PO4.ferm.+PO4.apporte; 0.762, 0.894, pH+tmp+P-Source.Feed.conc.+PL+emission_CO2+Th r+His+lactate+PO4.ferm.+PO4.utilise; 0.758, 0.894, pH+tmp+M ain.P-Source.Conc.+Main.Thr.Conc.+Nitrogen.Conc.+total.Vol+ emission_CO2+rab+lactate+PO4.ferm.+PO4.apporte; 0.762, 0.89 4, tmp+Main.P-Source.Conc.+Nitrogen.Conc.+OD+total.Vol+emis sion_CO2+His+lactate+PO4.apporte+PO4.utilise; 0.743, 0.894, tmp+Main.P-Source.Conc.+P-Source.Feed.conc.+Nitrogen.Conc. +total.Vol+PL+emission_O2+emission_CO2+rab+Thr+His+lactate+ PO4.ferm.+PO4.apporte; 0.753, 0.894, tmp+Main.P-Source.Conc. +Nitrogen.Conc.+OD+PL+emission CO2+rab+Thr+His+lactate+PO4. apporte+PO4.utilise; 0.753, 0.894, tmp+Main.P-Source.Conc.+ Main.Thr.Conc.+Nitrogen.Conc.+OD+total.Vol+emission_O2+emis sion_CO2+His+lactate+PO4.ferm.+PO4.apporte; 0.758, 0.894, p H+Main.P-Source.Conc.+Nitrogen.Conc.+total.Vol+PL+emission_ CO2+Thr+His+lactate+PO4.apporte+PO4.utilise; 0.743, 0.894, pH+tmp+Main.P-Source.Conc.+P-Source.Feed.conc.+Nitrogen.Con c.+total.Vol+PL+emission_CO2+rab+Thr+His+lactate+PO4.ferm.+ PO4.apporte; 0.758, 0.894, tmp+Main.P-Source.Conc.+P-Source. Feed.conc.+Nitrogen.Conc.+PL+emission O2+emission CO2+Thr+1 actate+PO4.ferm.+PO4.apporte; 0.748, 0.894, tmp+Main.P-Sour ce.Conc.+Main.Thr.Conc.+Nitrogen.Conc.+total.Vol+PL+emissio n_O2+emission_CO2+His+lactate+SO4.ferm.+PO4.apporte+PO4.uti lise; 0.758, 0.894, tmp+Main.P-Source.Conc.+Nitrogen.Conc.+ OD+total.Vol+emission_O2+emission_CO2+Thr+lactate+PO4.appor te+PO4.utilise; 0.770, 0.894, tmp+Main.P-Source.Conc.+Nitro gen.Conc.+total.Vol+emission_CO2+Thr+lactate+PO4.apporte; 0. 753, 0.894, pH+tmp+Main.P-Source.Conc.+Main.Thr.Conc.+Nitro gen.Conc.+total.Vol+emission_O2+emission_CO2+His+lactate+PO 4.ferm.+PO4.apporte; 0.753, 0.894, tmp+Main.P-Source.Conc.+ Nitrogen.Conc.+OD+total.Vol+emission_O2+emission_CO2+Thr+Hi s+lactate+PO4.ferm.+PO4.apporte; 0.766, 0.894, tmp+Main.P-S ource.Conc.+Nitrogen.Conc.+total.Vol+emission_CO2+Thr+lacta te+PO4.apporte+PO4.utilise; 0.758, 0.894, tmp+Main.P-Source. Conc.+P-Source.Feed.conc.+Nitrogen.Conc.+PL+emission_O2+emi ssion_CO2+Thr+lactate+PO4.apporte+PO4.utilise; 0.758, 0.894, tmp+Main.P-Source.Conc.+Nitrogen.Conc.+OD+total.Vol+yield+ PL+emission_CO2+His+lactate+PO4.apporte; 0.753, 0.894, pH+t mp+Main.P-Source.Conc.+Nitrogen.Conc.+OD+PL+emission_CO2+Th r+His+lactate+PO4.apporte+PO4.utilise; 0.758, 0.894, tmp+Ma in.P-Source.Conc.+Nitrogen.Conc.+OD+yield+PL+emission_CO2+r ab+His+lactate+PO4.apporte; 0.753, 0.894, pH+tmp+Main.P-Sou rce.Conc.+P-Source.Feed.conc.+Nitrogen.Conc.+total.Vol+PL+e mission_CO2+His+lactate+PO4.ferm.+PO4.apporte; 0.757, 0.894, tmp+Main.P-Source.Conc.+P-Source.Feed.conc.+Nitrogen.Conc. +PL+emission_CO2+rab+His+lactate+PO4.ferm.+PO4.apporte; 0.7 53, 0.894, tmp+Main.P-Source.Conc.+Nitrogen.Conc.+total.Vol +PL+emission_O2+emission_CO2+Thr+lactate+SO4.ferm.+PO4.ferm. +PO4.apporte; 0.748, 0.894, tmp+Main.P-Source.Conc.+Main.Th r.Conc.+Nitrogen.Conc.+total.Vol+PL+emission_O2+emission_CO 2+His+lactate+SO4.ferm.+PO4.ferm.+PO4.apporte; 0.766, 0.894, tmp+Main.P-Source.Conc.+P-Source.Feed.conc.+PL+emission_CO 2+Thr+His+lactate+PO4.ferm.; 0.753, 0.894, pH+tmp+Main.P-So urce.Conc.+Main.Thr.Conc.+Nitrogen.Conc.+total.Vol+emission _CO2+rab+His+lactate+PO4.ferm.+PO4.apporte; 0.757, 0.894, p H+tmp+Main.P-Source.Conc.+Nitrogen.Conc.+total.Vol+PL+emiss ion_O2+emission_CO2+lactate+PO4.ferm.+PO4.apporte; 0.753, 0. 894, pH+tmp+Main.P-Source.Conc.+Nitrogen.Conc.+OD+PL+emissi on_O2+emission_CO2+His+lactate+PO4.ferm.+PO4.apporte; 0.757, 0.894, tmp+Main.P-Source.Conc.+P-Source.Feed.conc.+Nitroge n.Conc.+PL+emission_CO2+rab+His+lactate+PO4.apporte+PO4.uti lise; 0.748, 0.894, tmp+Main.P-Source.Conc.+Main.Thr.Conc.+ Nitrogen.Conc.+total.Vol+PL+emission_CO2+rab+Thr+His+lactat e+PO4.apporte+PO4.utilise; 0.743, 0.894, tmp+Main.P-Source. Conc.+P-Source.Feed.conc.+Nitrogen.Conc.+total.Vol+PL+emiss ion_O2+emission_CO2+rab+Thr+His+lactate+PO4.apporte+PO4.uti lise; 0.757, 0.894, tmp+Main.P-Source.Conc.+Main.Thr.Conc.+ Nitrogen.Conc.+OD+total.Vol+emission_O2+emission_CO2+lactat e+PO4.ferm.+PO4.apporte; 0.757, 0.894, tmp+Main.P-Source.Co nc.+Nitrogen.Conc.+OD+total.Vol+emission_CO2+rab+Thr+lactat e+PO4.ferm.+PO4.apporte; 0.753, 0.894, tmp+Main.P-Source.Co nc.+Main.Thr.Conc.+Nitrogen.Conc.+OD+total.Vol+emission_CO2 +rab+His+lactate+PO4.ferm.+PO4.apporte; 0.753, 0.894, tmp+M ain.P-Source.Conc.+Nitrogen.Conc.+Lys_conc.+total.Vol+PL+em ission_O2+emission_CO2+Thr+lactate+PO4.ferm.+PO4.apporte; 0. 748, 0.894, pH+tmp+Main.P-Source.Conc.+Nitrogen.Conc.+total. Vol+yield+PL+emission_CO2+rab+His+lactate+PO4.ferm.+PO4.app orte; 0.762, 0.894, tmp+P-Source.Feed.conc.+OD+PL+emission_ CO2+Thr+His+lactate+PO4.apporte+PO4.utilise; 0.757, 0.894, tmp+Main.P-Source.Conc.+P-Source.Feed.conc.+Nitrogen.Conc.+ PL+emission_CO2+rab+Thr+lactate+PO4.ferm.+PO4.apporte; 0.77 0, 0.894, pH+tmp+Main.P-Source.Conc.+Nitrogen.Conc.+total.V ol+emission_CO2+lactate+PO4.apporte; 0.757, 0.894, pH+tmp+M ain.P-Source.Conc.+Main.Thr.Conc.+Nitrogen.Conc.+total.Vol+ emission_O2+emission_CO2+lactate+PO4.apporte+PO4.utilise; 0. 748, 0.894, pH+tmp+Main.P-Source.Conc.+Nitrogen.Conc.+total. Vol+yield+PL+emission_CO2+rab+His+lactate+PO4.apporte+PO4.u tilise; 0.762, 0.894, tmp+Main.P-Source.Conc.+P-Source.Feed. conc.+Nitrogen.Conc.+total.Vol+emission_O2+emission_CO2+lac tate+PO4.ferm.+PO4.apporte; 0.753, 0.894, pH+tmp+Main.P-Sou rce.Conc.+Nitrogen.Conc.+OD+PL+emission_CO2+rab+His+lactate +PO4.ferm.+PO4.apporte; 0.743, 0.894, tmp+Main.P-Source.Con c.+P-Source.Feed.conc.+Nitrogen.Conc.+OD+total.Vol+PL+emiss ion_O2+emission_CO2+Thr+His+lactate+PO4.ferm.+PO4.apporte; 0.753, 0.894, tmp+Main.P-Source.Conc.+Nitrogen.Conc.+OD+tot al.Vol+PL+emission_O2+emission_CO2+Thr+lactate+PO4.ferm.+PO 4.apporte; 0.743, 0.893, pH+tmp+Main.P-Source.Conc.+Nitroge n.Conc.+OD+total.Vol+PL+emission_O2+emission_CO2+Thr+His+la ctate+PO4.ferm.+PO4.apporte; 0.762, 0.893, tmp+P-Source.Fee d.conc.+yield+PL+emission_CO2+Thr+His+lactate+PO4.apporte+P O4.utilise; 0.770, 0.893, pH+Main.P-Source.Conc.+Nitrogen.C onc.+total.Vol+PL+emission_CO2+lactate+PO4.apporte; 0.743, 0.893, pH+tmp+Main.P-Source.Conc.+P-Source.Feed.conc.+Nitro gen.Conc.+total.Vol+PL+emission_CO2+rab+Thr+His+lactate+PO4. apporte+PO4.utilise; 0.757, 0.893, tmp+Main.P-Source.Conc.+ P-Source.Feed.conc.+Nitrogen.Conc.+total.Vol+emission_CO2+r ab+His+lactate+PO4.apporte+PO4.utilise; 0.762, 0.893, pH+Ma in.P-Source.Conc.+Nitrogen.Conc.+total.Vol+PL+emission_CO2+ Thr+His+lactate+PO4.apporte; 0.774, 0.893, Main.P-Source.Co nc.+Nitrogen.Conc.+total.Vol+emission_CO2+lactate+PO4.ferm. +PO4.apporte; 0.762, 0.893, pH+Main.P-Source.Conc.+P-Source. Feed.conc.+Nitrogen.Conc.+total.Vol+PL+emission_CO2+His+lac tate+PO4.apporte; 0.757, 0.893, pH+tmp+Main.P-Source.Conc.+ Main.Thr.Conc.+Nitrogen.Conc.+total.Vol+emission_CO2+rab+la ctate+PO4.apporte+PO4.utilise; 0.757, 0.893, tmp+Main.P-Sou rce.Conc.+Main.Thr.Conc.+Nitrogen.Conc.+OD+total.Vol+emissi on_CO2+rab+lactate+PO4.ferm.+PO4.apporte; 0.762, 0.893, pH+ Main.P-Source.Conc.+Nitrogen.Conc.+total.Vol+PL+emission_O2 +emission_CO2+His+lactate+PO4.apporte; 0.753, 0.893, tmp+Ma in.P-Source.Conc.+Main.Thr.Conc.+Nitrogen.Conc.+OD+total.Vo l+emission_O2+emission_CO2+His+lactate+PO4.apporte+PO4.util ise; 0.748, 0.893, tmp+Main.P-Source.Conc.+P-Source.Feed.co nc.+Nitrogen.Conc.+total.Vol+yield+PL+emission_CO2+Thr+His+ lactate+PO4.apporte+PO4.utilise; 0.762, 0.893, tmp+P-Source. Feed.conc.+Main.Thr.Conc.+PL+emission_CO2+Thr+His+lactate+P O4.apporte+PO4.utilise; 0.766, 0.893, tmp+Main.P-Source.Con c.+Main.Thr.Conc.+Nitrogen.Conc.+total.Vol+emission_CO2+rab +lactate+PO4.apporte; 0.757, 0.893, tmp+Main.P-Source.Conc. +Nitrogen.Conc.+OD+total.Vol+PL+emission_CO2+His+lactate+PO 4.ferm.+PO4.utilise; 0.757, 0.893, tmp+Main.P-Source.Conc.+ P-Source.Feed.conc.+Nitrogen.Conc.+PL+emission_CO2+rab+Thr+ lactate+PO4.apporte+PO4.utilise; 0.757, 0.893, pH+tmp+Main. P-Source.Conc.+Nitrogen.Conc.+total.Vol+PL+emission_CO2+rab +lactate+PO4.ferm.+PO4.apporte; 0.762, 0.893, tmp+Main.P-So urce.Conc.+P-Source.Feed.conc.+Nitrogen.Conc.+total.Vol+emi ssion_CO2+rab+lactate+PO4.ferm.+PO4.apporte; 0.748, 0.893, tmp+Main.P-Source.Conc.+P-Source.Feed.conc.+Nitrogen.Conc.+ total.Vol+yield+PL+emission_CO2+Thr+His+lactate+PO4.ferm.+P O4.apporte; 0.757, 0.893, tmp+Main.P-Source.Conc.+Main.Thr. Conc.+Nitrogen.Conc.+total.Vol+PL+emission_O2+emission_CO2+ lactate+PO4.ferm.+PO4.apporte; 0.748, 0.893, pH+tmp+Main.P-Source.Conc.+Main.Thr.Conc.+Nitrogen.Conc.+total.Vol+PL+emi ssion_O2+emission_CO2+His+lactate+PO4.ferm.+PO4.apporte; 0. 757, 0.893, tmp+Main.P-Source.Conc.+P-Source.Feed.conc.+Nit rogen.Conc.+OD+PL+emission CO2+Thr+His+lactate+PO4.apporte; 0.757, 0.893, tmp+Main.P-Source.Conc.+Nitrogen.Conc.+OD+to tal.Vol+PL+emission_O2+emission_CO2+His+lactate+PO4.apport e; 0.748, 0.893, tmp+Main.P-Source.Conc.+P-Source.Feed.conc. +Nitrogen.Conc.+OD+PL+emission_O2+emission_CO2+Thr+His+lact ate+PO4.apporte+PO4.utilise; 0.748, 0.893, tmp+Main.P-Sourc e.Conc.+P-Source.Feed.conc.+Nitrogen.Conc.+OD+PL+emission_O 2+emission_CO2+Thr+His+lactate+PO4.ferm.+PO4.apporte; 0.753, 0.893, pH+tmp+Main.P-Source.Conc.+Nitrogen.Conc.+total.Vol +PL+emission CO2+rab+Thr+lactate+PO4.apporte+PO4.utilise; 0. 757, 0.893, tmp+Main.P-Source.Conc.+Main.Thr.Conc.+Nitrogen. Conc.+OD+total.Vol+emission_O2+emission_CO2+lactate+PO4.app orte+PO4.utilise; 0.757, 0.893, tmp+Main.P-Source.Conc.+P-S ource.Feed.conc.+Nitrogen.Conc.+total.Vol+emission_O2+emiss ion_CO2+His+lactate+PO4.apporte+PO4.utilise; 0.753, 0.893, pH+tmp+Main.P-Source.Conc.+P-Source.Feed.conc.+Nitrogen.Con c.+total.Vol+PL+emission_CO2+His+lactate+PO4.apporte+PO4.ut ilise; 0.770, 0.893, pH+Main.P-Source.Conc.+Nitrogen.Conc.+ total.Vol+emission_CO2+His+lactate+PO4.apporte; 0.748, 0.89 3, pH+tmp+Main.P-Source.Conc.+P-Source.Feed.conc.+Nitrogen. Conc.+total.Vol+PL+emission_O2+emission_CO2+His+lactate+PO4. ferm.+PO4.apporte; 0.757, 0.893, tmp+Main.P-Source.Conc.+Ma in.Thr.Conc.+Nitrogen.Conc.+total.Vol+emission_O2+emission CO2+Thr+lactate+PO4.apporte+PO4.utilise; 0.761, 0.893, tmp+ Main.P-Source.Conc.+Nitrogen.Conc.+total.Vol+yield+emission _CO2+His+lactate+PO4.apporte+PO4.utilise; 0.757, 0.893, tmp +P-Source.Feed.conc.+PL+emission_O2+emission_CO2+rab+Thr+Hi s+lactate+PO4.apporte+PO4.utilise; 0.752, 0.893, pH+tmp+Mai n.P-Source.Conc.+Main.Thr.Conc.+Nitrogen.Conc.+total.Vol+em ission_O2+emission_CO2+His+lactate+PO4.apporte+PO4.utilise; 0.748, 0.893, pH+tmp+Main.P-Source.Conc.+Nitrogen.Conc.+Ly s_conc.+total.Vol+PL+emission_O2+emission_CO2+His+lactate+P O4.apporte+PO4.utilise; 0.743, 0.893, tmp+Main.P-Source.Con c.+P-Source.Feed.conc.+Nitrogen.Conc.+OD+total.Vol+PL+emiss ion_O2+emission_CO2+Thr+His+lactate+PO4.apporte+PO4.utilis e; 0.748, 0.893, pH+tmp+Main.P-Source.Conc.+Nitrogen.Conc.+ OD+yield+PL+emission_O2+emission_CO2+His+lactate+PO4.apport e+PO4.utilise; 0.766, 0.893, tmp+Main.P-Source.Conc.+Main.T hr.Conc.+Nitrogen.Conc.+total.Vol+emission_O2+emission_CO2+ lactate+PO4.apporte; 0.748, 0.893, tmp+Main.P-Source.Conc.+ P-Source.Feed.conc.+Nitrogen.Conc.+PL+emission_O2+emission_ CO2+rab+Thr+His+lactate+PO4.apporte+PO4.utilise; 0.757, 0.8 93, pH+Main.P-Source.Conc.+Nitrogen.Conc.+total.Vol+PL+emis sion_O2+emission_CO2+His+lactate+PO4.ferm.+PO4.apporte; 0.7 61, 0.893, tmp+Main.P-Source.Conc.+Main.Thr.Conc.+Nitrogen. Conc.+emission_O2+emission_CO2+His+lactate+PO4.apporte+PO4. utilise; 0.757, 0.893, Main.P-Source.Conc.+P-Source.Feed.co nc.+Nitrogen.Conc.+OD+total.Vol+PL+emission_CO2+His+lactate +PO4.ferm.+PO4.apporte; 0.748, 0.893, tmp+Main.P-Source.Con c.+Nitrogen.Conc.+OD+yield+PL+emission_O2+emission_CO2+Thr+ His+lactate+PO4.apporte+PO4.utilise; 0.748, 0.893, pH+tmp+M ain.P-Source.Conc.+Nitrogen.Conc.+Lys_conc.+total.Vol+PL+em ission_CO2+rab+His+lactate+PO4.ferm.+PO4.apporte; 0.752, 0. 893, tmp+Main.P-Source.Conc.+Nitrogen.Conc.+total.Vol+PL+em ission_O2+emission_CO2+rab+Thr+lactate+PO4.ferm.+PO4.apport e; 0.770, 0.893, tmp+Main.P-Source.Conc.+Nitrogen.Conc.+emi ssion_O2+emission_CO2+lactate+PO4.ferm.+PO4.apporte; 0.743, 0.893, tmp+Main.P-Source.Conc.+P-Source.Feed.conc.+Nitroge n.Conc.+total.Vol+PL+emission_O2+emission_CO2+Thr+His+lacta te+SO4.ferm.+PO4.ferm.+PO4.apporte; 0.757, 0.893, Main.P-So urce.Conc.+P-Source.Feed.conc.+Nitrogen.Conc.+total.Vol+yie ld+PL+emission_CO2+His+lactate+PO4.ferm.+PO4.apporte; 0.752, 0.893, pH+tmp+Main.P-Source.Conc.+Nitrogen.Conc.+total.Vol +PL+emission_CO2+His+lactate+SO4.ferm.+PO4.ferm.+PO4.apport e; 0.752, 0.893, tmp+Main.P-Source.Conc.+Main.Thr.Conc.+Nit rogen.Conc.+OD+total.Vol+emission_CO2+rab+His+lactate+PO4.a pporte+PO4.utilise; 0.752, 0.893, pH+tmp+Main.P-Source.Conc. +Main.Thr.Conc.+Nitrogen.Conc.+total.Vol+emission_CO2+rab+H is+lactate+PO4.apporte+PO4.utilise; 0.752, 0.893, tmp+Main. P-Source.Conc.+Nitrogen.Conc.+total.Vol+PL+emission_O2+emis sion_CO2+Thr+lactate+SO4.ferm.+PO4.apporte+PO4.utilise; 0.7 57, 0.893, tmp+Main.P-Source.Conc.+Main.Thr.Conc.+Nitrogen. Conc.+OD+total.Vol+emission_CO2+rab+lactate+PO4.apporte+PO4. utilise; 0.748, 0.893, pH+tmp+Main.P-Source.Conc.+P-Source. Feed.conc.+Nitrogen.Conc.+total.Vol+PL+emission_CO2+rab+His +lactate+PO4.ferm.+PO4.apporte; 0.757, 0.893, tmp+Main.P-So urce.Conc.+Main.Thr.Conc.+Nitrogen.Conc.+total.Vol+emission _O2+emission_CO2+lactate+SO4.ferm.+PO4.ferm.+PO4.apporte; 0. 757, 0.893, tmp+Main.P-Source.Conc.+Main.Thr.Conc.+Nitrogen. Conc.+total.Vol+PL+emission_O2+emission_CO2+lactate+PO4.app orte+PO4.utilise; 0.752, 0.893, pH+tmp+Main.P-Source.Conc.+ Nitrogen.Conc.+OD+PL+emission_O2+emission_CO2+His+lactate+P O4.apporte+PO4.utilise; 0.747, 0.893, pH+tmp+Main.P-Source. Conc.+Main.Thr.Conc.+Nitrogen.Conc.+total.Vol+PL+emission_O 2+emission_CO2+His+lactate+PO4.apporte+PO4.utilise; 0.747, 0.893, tmp+Main.P-Source.Conc.+Nitrogen.Conc.+OD+total.Vol+ yield+PL+emission_CO2+Thr+His+lactate+PO4.ferm.+PO4.apport e; 0.757, 0.893, pH+tmp+Main.P-Source.Conc.+Nitrogen.Conc.+ OD+PL+emission_CO2+Thr+His+lactate+PO4.apporte; 0.761, 0.89 3, pH+Main.P-Source.Conc.+Nitrogen.Conc.+OD+PL+emission_CO2 +His+lactate+PO4.ferm.+PO4.apporte; 0.757, 0.893, tmp+Main. P-Source.Conc.+Nitrogen.Conc.+total.Vol+yield+PL+emission_C O2+Thr+His+lactate+PO4.apporte; 0.765, 0.893, tmp+Main.P-So urce.Conc.+Nitrogen.Conc.+total.Vol+emission_CO2+Thr+His+la ctate+PO4.apporte; 0.757, 0.893, tmp+P-Source.Feed.conc.+to tal.Vol+PL+emission_O2+emission_CO2+Thr+His+lactate+PO4.app orte+PO4.utilise; 0.747, 0.893, tmp+Main.P-Source.Conc.+P-S ource.Feed.conc.+Nitrogen.Conc.+OD+total.Vol+PL+emission_CO 2+Thr+His+lactate+PO4.ferm.+PO4.apporte; 0.747, 0.893, pH+t mp+Main.P-Source.Conc.+Nitrogen.Conc.+OD+yield+PL+emission O2+emission_CO2+His+lactate+PO4.ferm.+PO4.apporte; 0.757, 0. 893, tmp+Main.P-Source.Conc.+Nitrogen.Conc.+OD+total.Vol+PL +emission_CO2+rab+His+lactate+PO4.apporte; 0.742, 0.893, tm p+Main.P-Source.Conc.+Nitrogen.Conc.+OD+Lys-conc.+total.Vol +PL+emission_O2+emission_CO2+Thr+His+lactate+PO4.apporte+PO 4.utilise; 0.761, 0.893, tmp+P-Source.Feed.conc.+Main.Thr.C onc.+PL+emission_CO2+Thr+His+lactate+PO4.ferm.+PO4.apporte; 0.774, 0.893, Main.P-Source.Conc.+Nitrogen.Conc.+total.Vol +emission_CO2+lactate+PO4.apporte+PO4.utilise; 0.747, 0.893, tmp+Main.P-Source.Conc.+P-Source.Feed.conc.+Nitrogen.Conc. +PL+emission_O2+emission_CO2+rab+Thr+His+lactate+PO4.ferm.+ PO4.apporte; 0.747, 0.893, pH+tmp+Main.P-Source.Conc.+Nitro gen.Conc.+OD+yield+PL+emission_CO2+rab+His+lactate+PO4.appo rte+PO4.utilise; 0.761, 0.893, tmp+P-Source.Feed.conc.+OD+P L+emission_CO2+Thr+His+lactate+PO4.ferm.+PO4.apporte; 0.752, 0.893, tmp+Main.P-Source.Conc.+P-Source.Feed.conc.+Nitroge n.Conc.+total.Vol+PL+emission_CO2+rab+Thr+lactate+PO4.ferm. +PO4.apporte; 0.752, 0.893, tmp+Main.P-Source.Conc.+Nitroge n.Conc.+OD+total.Vol+emission_CO2+rab+Thr+His+lactate+PO4.f erm.+PO4.apporte; 0.747, 0.893, tmp+Main.P-Source.Conc.+Nit rogen.Conc.+OD+total.Vol+yield+PL+emission_CO2+Thr+His+lact ate+PO4.apporte+PO4.utilise; 0.761, 0.893, tmp+Main.P-Sourc e.Conc.+Nitrogen.Conc.+total.Vol+PL+emission_O2+emission_CO 2+Thr+lactate+PO4.apporte; 0.757, 0.893, pH+tmp+Main.P-Sour ce.Conc.+Nitrogen.Conc.+total.Vol+PL+emission_CO2+Thr+lacta te+PO4.ferm.+PO4.apporte; 0.752, 0.893, tmp+Main.P-Source.C onc.+Nitrogen.Conc.+OD+total.Vol+emission_O2+emission_CO2+T hr+His+lactate+PO4.apporte+PO4.utilise; 0.757, 0.893, pH+tm p+Main.P-Source.Conc.+Nitrogen.Conc.+total.Vol+yield+PL+emi ssion_CO2+His+lactate+PO4.apporte; 0.757, 0.893, Main.P-Sou rce.Conc.+P-Source.Feed.conc.+Nitrogen.Conc.+total.Vol+yiel d+PL+emission_CO2+His+lactate+PO4.apporte+PO4.utilise; 0.75 7, 0.893, Main.P-Source.Conc.+Nitrogen.Conc.+OD+total.Vol+P L+emission_CO2+Thr+His+lactate+PO4.ferm.+PO4.apporte; 0.752, 0.893, tmp+Main.P-Source.Conc.+Nitrogen.Conc.+OD+total.Vol +PL+emission_O2+emission_CO2+Thr+lactate+PO4.apporte+PO4.ut ilise; 0.757, 0.893, pH+tmp+Main.P-Source.Conc.+Nitrogen.Co nc.+total.Vol+PL+emission_O2+emission_CO2+lactate+PO4.appor te+PO4.utilise; 0.747, 0.893, tmp+Main.P-Source.Conc.+Nitro gen.Conc.+OD+Lys_conc.+total.Vol+PL+emission_CO2+rab+His+la ctate+PO4.apporte+PO4.utilise; 0.761, 0.893, tmp+Main.P-Sou rce.Conc.+Main.Thr.Conc.+Nitrogen.Conc.+emission_O2+emissio n_CO2+His+lactate+PO4.ferm.+PO4.apporte; 0.757, 0.893, tmp+ Main.P-Source.Conc.+Nitrogen.Conc.+total.Vol+emission-O2+em ission_CO2+rab+His+lactate+PO4.apporte+PO4.utilise; 0.752, 0.893, pH+tmp+Main.P-Source.Conc.+Nitrogen.Conc.+total.Vol+ PL+emission_O2+emission_CO2+His+lactate+PO4.ferm.+PO4.utili se; 0.742, 0.893, tmp+Main.P-Source.Conc.+P-Source.Feed.con c.+Nitrogen.Conc.+total.Vol+PL+emission O2+emission CO2+Thr +His+lactate+SO4.ferm.+PO4.apporte+PO4.utilise; 0.757, 0.89 3, tmp+Main.P-Source.Conc.+Nitrogen.Conc.+OD+total.Vol+emis sion_CO2+rab+Thr+lactate+PO4.apporte+PO4.utilise; 0.757, 0. 893, pH+Main.P-Source.Conc.+Nitrogen.Conc.+total.Vol+PL+emi ssion_O2+emission_CO2+His+lactate+PO4.apporte+PO4.utilise; 0.752, 0.893, pH+tmp+Main.P-Source.Conc.+Nitrogen.Conc.+OD+ PL+emission_CO2+rab+His+lactate+PO4.apporte+PO4.utilise; 0. 765, 0.893, tmp+Main.P-Source.Conc.+Nitrogen.Conc.+total.Vo l+emission_CO2+rab+lactate+PO4.ferm.+PO4.utilise; 0.747, 0. 893, pH+tmp+Main.P-Source.Conc.+P-Source.Feed.conc.+Nitroge n.Conc.+PL+emission_O2+emission_CO2+Thr+His+lactate+PO4.app orte+PO4.utilise; 0.765, 0.893, Main.P-Source.Conc.+Nitroge n.Conc.+total.Vol+PL+emission_CO2+His+lactate+SO4.ferm.+PO4. apporte; 0.757, 0.893, tmp+Main.P-Source.Conc.+Main.Thr.Con c.+Nitrogen.Conc.+total.Vol+emission_O2+emission_CO2+lactat e+SO4.ferm.+PO4.apporte+PO4.utilise; 0.752, 0.893, pH+tmp+M ain.P-Source.Conc.+P-Source.Feed.conc.+Nitrogen.Conc.+PL+em ission_CO2+Thr+His+lactate+PO4.apporte+PO4.utilise; 0.747, 0.893, tmp+Main.P-Source.Conc.+Main.Thr.Conc.+Nitrogen.Conc. +OD+total.Vol+PL+emission_O2+emission_CO2+His+lactate+PO4.f erm.+PO4.apporte; 0.747, 0.893, pH+tmp+Main.P-Source.Conc.+ Nitrogen.Conc.+total.Vol+PL+emission_O2+emission_CO2+His+la ctate+SO4.ferm.+PO4.ferm.+PO4.apporte; 0.747, 0.893, pH+tmp +Main.P-Source.Conc.+Main.Thr.Conc.+Nitrogen.Conc.+total.Vo l+PL+emission_CO2+rab+His+lactate+PO4.ferm.+PO4.apporte; 0. 757, 0.893, Main.P-Source.Conc.+Nitrogen.Conc.+total.Vol+yi eld+PL+emission_CO2+Thr+His+lactate+PO4.ferm.+PO4.apporte; 0.761, 0.893, pH+Main.P-Source.Conc.+Nitrogen.Conc.+OD+PL+e mission_CO2+His+lactate+PO4.apporte+PO4.utilise; 0.757, 0.8 93, tmp+Main.P-Source.Conc.+Main.Thr.Conc.+Nitrogen.Conc.+t otal.Vol+PL+emission_CO2+rab+lactate+PO4.ferm.+PO4.apporte; 0.752, 0.893, pH+tmp+Main.P-Source.Conc.+Nitrogen.Conc.+to tal.Vol+PL+emission_CO2+rab+His+lactate+PO4.ferm.+PO4.utili se; 0.752, 0.893, tmp+Main.P-Source.Conc.+Nitrogen.Conc.+OD +Lys_conc.+total.Vol+PL+emission_CO2+His+lactate+PO4.ferm.+ PO4.apporte; 0.742, 0.893, pH+tmp+Main.P-Source.Conc.+Nitro gen.Conc.+OD+total.Vol+PL+emission_O2+emission_CO2+Thr+His+ lactate+PO4.apporte+PO4.utilise; 0.747, 0.893, tmp+Main.P-S ource.Conc.+P-Source.Feed.conc.+Nitrogen.Conc.+OD+total.Vol +PL+emission_CO2+Thr+His+lactate+PO4.apporte+PO4.utilise; 0. 757, 0.893, Main.P-Source.Conc.+Nitrogen.Conc.+total.Vol+yi eld+PL+emission_CO2+Thr+His+lactate+PO4.apporte+PO4.utilis e; 0.765, 0.893, tmp+P-Source.Feed.conc.+OD+PL+emission_CO2 +Thr+His+lactate+PO4.apporte; 0.761, 0.893, tmp+P-Source.Fe ed.conc.+yield+PL+emission_CO2+Thr+His+lactate+PO4.ferm.+PO 4.apporte; 0.757, 0.893, pH+Main.P-Source.Conc.+Nitrogen.Co nc.+OD+total.Vol+yield+PL+emission_CO2+His+lactate+PO4.appo rte; 0.761, 0.893, Main.P-Source.Conc.+Nitrogen.Conc.+OD+to tal.Vol+PL+emission_CO2+Thr+His+lactate+PO4.apporte; 0.747, 0.893, pH+tmp+Main.P-Source.Conc.+Nitrogen.Conc.+OD+yield+ PL+emission_CO2+rab+His+lactate+PO4.ferm.+PO4.apporte; 0.74 2, 0.893, tmp+Main.P-Source.Conc.+P-Source.Feed.conc.+Nitro gen.Conc.+total.Vol+yield+PL+emission_O2+emission_CO2+Thr+H is+lactate+PO4.apporte+PO4.utilise; 0.757, 0.893, tmp+Main. P-Source.Conc.+Main.Thr.Conc.+Nitrogen.Conc.+total.Vol+emis sion_CO2+rab+Thr+lactate+PO4.ferm.+PO4.apporte; 0.747, 0.89 3, pH+tmp+Main.P-Source.Conc.+Nitrogen.Conc.+total.Vol+PL+e mission_CO2+rab+His+lactate+SO4.ferm.+PO4.ferm.+PO4.apport e; 0.765, 0.893, tmp+P-Source.Feed.conc.+Lys_conc.+PL+emiss ion_CO2+Thr+His+lactate+PO4.apporte; 0.747, 0.893, pH+tmp+M ain.P-Source.Conc.+P-Source.Feed.conc.+Nitrogen.Conc.+PL+em ission_O2+emission_CO2+Thr+His+lactate+PO4.ferm.+PO4.apport e; 0.761, 0.893, tmp+Main.P-Source.Conc.+P-Source.Feed.conc. +Nitrogen.Conc.+PL+emission_CO2+His+lactate+PO4.apporte+PO4. utilise; 0.773, 0.893, pH+Main.P-Source.Conc.+Nitrogen.Conc. +total.Vol+emission_CO2+lactate+PO4.apporte; 0.761, 0.893, tmp+Main.P-Source.Conc.+Nitrogen.Conc.+total.Vol+yield+emis sion_O2+emission_CO2+lactate+PO4.ferm.+PO4.apporte; 0.757, 0.893, pH+tmp+Main.P-Source.Conc.+Nitrogen.Conc.+total.Vol+ emission_O2+emission_CO2+Thr+lactate+PO4.ferm.+PO4.apporte; 0.761, 0.893, tmp+P-Source.Feed.conc.+Lys_conc.+PL+emissio n_CO2+Thr+His+lactate+PO4.apporte+PO4.utilise; 0.757, 0.893, pH+tmp+Main.P-Source.Conc.+P-Source.Feed.conc.+Nitrogen.Co nc.+PL+emission_CO2+Thr+His+lactate+PO4.apporte; 0.761, 0.8 93, tmp+P-Source.Feed.conc.+Main.Thr.Conc.+PL+emission_CO2+ Thr+His+lactate+PO4.ferm.+PO4.utilise; 0.757, 0.893, Main.P -Source.Conc.+P-Source.Feed.conc.+Nitrogen.Conc.+OD+total.V ol+PL+emission_CO2+His+lactate+PO4.apporte+PO4.utilise; 0.7 47, 0.893, pH+tmp+Main.P-Source.Conc.+P-Source.Feed.conc.+N itrogen.Conc.+PL+emission_CO2+rab+Thr+His+lactate+PO4.appor te+PO4.utilise; 0.752, 0.893, pH+tmp+Main.P-Source.Conc.+Ni trogen.Conc.+total.Vol+PL+emission_CO2+His+lactate+SO4.ferm. +PO4.apporte+PO4.utilise; 0.761, 0.893, Main.P-Source.Conc. +Nitrogen.Conc.+OD+total.Vol+PL+emission_O2+emission_CO2+Hi s+lactate+PO4.apporte; 0.765, 0.893, tmp+Main.P-Source.Conc. +Nitrogen.Conc.+total.Vol+PL+emission_O2+emission_CO2+lacta te+PO4.apporte; 0.769, 0.893, tmp+Main.P-Source.Conc.+Nitro gen.Conc.+emission_O2+emission_CO2+lactate+PO4.apporte+PO4. utilise; 0.761, 0.893, tmp+Main.P-Source.Conc.+P-Source.Fee d.cone.+Nitrogen.Cone.+PL+emission_CO2+His+lactate+PO4.ferm. +PO4.apporte; 0.747, 0.893, tmp+Main.P-Source.Conc.+Nitroge n.Conc.+OD+yield+PL+emission_O2+emission_CO2+Thr+His+lactat e+PO4.ferm.+PO4.apporte; 0.747, 0.893, pH+tmp+Main.P-Source. Conc.+P-Source.Feed.conc.+Nitrogen.Conc.+total.Vol+PL+emiss ion_O2+emission_CO2+His+lactate+PO4.apporte+PO4.utilise; 0. 752, 0.893, pH+tmp+Main.P-Source.Conc.+P-Source.Feed.conc.+ Nitrogen.Conc.+PL+emission_CO2+Thr+His+lactate+PO4.ferm.+PO 4.apporte; 0.761, 0.893, tmp+Main.P-Source.Conc.+P-Source.F eed.conc.+Main.Thr.Conc.+Nitrogen.Conc.+emission_CO2+rab+la ctate+PO4.apporte+PO4.utilise; 0.765, 0.893, tmp+Main.P-Sou rce.Conc.+Nitrogen.Conc.+total.Vol+PL+emission_CO2+rab+lact ate+PO4.apporte; 0.747, 0.893, pH+tmp+Main.P-Source.Conc.+P -Source.Feed.conc.+Nitrogen.Conc.+PL+emission_CO2+rab+Thr+H is+lactate+PO4.ferm.+PO4.apporte; 0.742, 0.893, pH+tmp+Main. P-Source.Conc.+Nitrogen.Conc.+total.Vol+PL+emission_O2+emis sion_CO2+rab+Thr+His+lactate+PO4.ferm.+PO4.apporte; 0.756, 0.893, pH+tmp+Main.P-Source.Conc.+Nitrogen.Conc.+total.Vol+ PL+emission_CO2+rab+lactate+PO4.apporte+PO4.utilise; 0.761, 0.893, tmp+Main.P-Source.Conc.+Nitrogen.Conc.+total.Vol+yi eld+emission_CO2+rab+lactate+PO4.ferm.+PO4.apporte; 0.765, 0.893, tmp+Main.P-Source.Conc.+Nitrogen.Conc.+total.Vol+emi ssion_O2+emission_CO2+lactate+PO4.ferm.+PO4.utilise; 0.756, 0.893, Main.P-Source.Conc.+Nitrogen.Conc.+OD+total.Vol+PL+ emission_CO2+Thr+His+lactate+PO4.apporte+PO4.utilise; 0.765, 0.893, tmp+Main.P-Source.Conc.+Nitrogen.Conc.+PL+emission_ CO2+His+lactate+PO4.apporte+PO4.utilise; 0.761, 0.893, tmp+ Main.P-Source.Conc.+Nitrogen.Conc.+OD+PL+emission_CO2+His+1 actate+PO4.ferm.+PO4.utilise; 0.765, 0.893, tmp+Main.P-Sour ce.Conc.+Nitrogen.Conc.+PL+emission_CO2+His+lactate+PO4.fer m.+PO4.apporte; 0.756, 0.893, tmp+Main.P-Source.Conc.+P-Sou rce.Feed.conc.+Nitrogen.Conc.+PL+emission_O2+emission_CO2+T hr+His+lactate+PO4.apporte; 0.761, 0.893, tmp+Main.P-Source. Conc.+P-Source.Feed.conc.+Main.Thr.Conc.+Nitrogen.Conc.+emi ssion_CO2+rab+lactate+PO4.ferm.+PO4.apporte; 0.756, 0.893, tmp+Main.P-Source.Conc.+P-Source.Feed.conc.+Nitrogen.Conc.+ PL+emission_O2+emission_CO2+His+lactate+PO4.ferm.+PO4.appor te; 0.761, 0.893, tmp+Main.P-Source.Conc.+Nitrogen.Conc.+PL +emission_CO2+rab+His+lactate+PO4.ferm.+PO4.apporte; 0.752, 0.893, tmp+Main.P-Source.Conc.+Nitrogen.Conc.+total.Vol+PL +emission_O2+emission_CO2+rab+Thr+lactate+PO4.apporte+PO4.u tilise; 0.752, 0.893, tmp+Main.P-Source.Conc.+P-Source.Feed. conc.+Nitrogen.Conc.+total.Vol+PL+emission_CO2+Thr+His+lact ate+PO4.ferm.+PO4.utilise; 0.761, 0.893, tmp+Main.P-Source. Conc.+Nitrogen.Conc.+total.Vol+yield+emission_O2+emission_C O2+lactate+PO4.apporte+PO4.utilise; 0.761, 0.893, pH+Main.P -Source.Conc.+Nitrogen.Conc.+total.Vol+PL+emission_CO2+rab+ His+lactate+PO4.apporte; 0.756, 0.893, tmp+Main.P-Source.Co nc.+P-Source.Feed.conc.+Nitrogen.Conc.+PL+emission_O2+emiss ion_CO2+His+lactate+PO4.apporte+PO4.utilise; 0.742, 0.893, pH+tmp+Main.P-Source.Conc.+Nitrogen.Conc.+Lys_conc.+total.V ol+PL+emission_O2+emission_CO2+Thr+His+lactate+PO4.ferm.+PO 4.apporte; 0.752, 0.893, tmp+Main.P-Source.Conc.+Nitrogen.C onc.+Lys_conc.+total.Vol+PL+emission_O2+emission_CO2+Thr+la ctate+PO4.apporte+PO4.utilise; 0.761, 0.893, tmp+Main.P-Sou rce.Conc.+P-Source.Feed.conc.+Nitrogen.Conc.+total.Vol+emis sion_O2+emission_CO2+lactate+PO4.apporte+PO4.utilise; 0.756, 0.893, tmp+Main.P-Source.Conc.+P-Source.Feed.conc.+Nitroge n.Conc.+PL+emission_CO2+rab+Thr+His+lactate+PO4.apporte; 0. 756, 0.893, tmp+Main.P-Source.Conc.+P-Source.Feed.conc.+Mai n.Thr.Conc.+Nitrogen.Conc.+total.Vol+emission_O2+emission_C O2+lactate+PO4.ferm.+PO4.apporte; 0.765, 0.893, pH+tmp+Main. P-Source.Conc.+Nitrogen.Conc.+total.Vol+emission_CO2+rab+la ctate+PO4.apporte; 0.747, 0.893, tmp+Main.P-Source.Conc.+Ma in.Thr.Conc.+Nitrogen.Conc.+OD+total.Vol+PL+emission_O2+emi ssion_CO2+His+lactate+PO4.apporte+PO4.utilise; 0.756, 0.893, pH+tmp+Main.P-Source.Conc.+Nitrogen.Conc.+total.Vol+PL+emi ssion_CO2+Thr+lactate+PO4.apporte+PO4.utilise; 0.747, 0.893, pH+tmp+Main.P-Source.Conc.+Main.Thr.Conc.+Nitrogen.Conc.+t otal.Vol+PL+emission_CO2+rab+His+lactate+PO4.apporte+PO4.ut ilise; 0.765, 0.893, tmp+Main.P-Source.Conc.+Nitrogen.Conc. +OD+total.Vol+emission_CO2+His+lactate+PO4.apporte; 0.756, 0.893, tmp+Main.P-Source.Conc.+Main.Thr.Conc.+Nitrogen.Conc. +total.Vol+PL+emission_CO2+rab+lactate+PO4.apporte+PO4.util ise; 0.752, 0.893, tmp+Main.P-Source.Conc.+Main.Thr.Conc.+N itrogen.Conc.+total.Vol+emission_O2+emission_CO2+Thr+His+la ctate+PO4.ferm.+PO4.apporte; 0.761, 0.893, Main.P-Source.Co nc.+P-Source.Feed.conc.+Nitrogen.Conc.+OD+total.Vol+PL+emis sion_CO2+His+lactate+PO4.apporte; 0.747, 0.893, pH+tmp+Main. P-Source.Conc.+P-Source.Feed.conc.+Nitrogen.Conc.+total.Vol +PL+emission_CO2+rab+His+lactate+PO4.apporte+PO4.utilise; 0. 752, 0.893, tmp+Main.P-Source.Conc.+P-Source.Feed.conc.+Nit rogen.Conc.+total.Vol+PL+emission_CO2+rab+Thr+lactate+PO4.a pporte+PO4.utilise; 0.747, 0.893, tmp+Main.P-Source.Conc.+M ain.Thr.Conc.+Nitrogen.Conc.+total.Vol+PL+emission_CO2+rab+ His+lactate+SO4.ferm.+PO4.ferm.+PO4.apporte; 0.761, 0.893, tmp+Main.P-Source.Conc.+Nitrogen.Conc.+total.Vol+emission_O 2+rab+His+lactate+PO4.ferm.+PO4.apporte; 0.761, 0.893, tmp+ Main.P-Source.Conc.+P-Source.Feed.conc.+Nitrogen.Conc.+tota l.Vol+emission_CO2+rab+lactate+PO4.apporte+PO4.utilise; 0.7 61, 0.893, tmp+Main.P-Source.Conc.+Nitrogen.Conc.+total.Vol +yield+emission_CO2+rab+lactate+PO4.apporte+PO4.utilise; 0. 747, 0.893, tmp+Main.P-Source.Conc.+Main.Thr.Conc.+Nitrogen. Conc.+total.Vol+PL+emission_CO2+rab+His+lactate+SO4.ferm.+P O4.apporte+PO4.utilise; 0.756, 0.893, tmp+P-Source.Feed.con c.+PL+emission_O2+emission_O2+rab+Thr+His+lactate+PO4.ferm. +PO4.apporte; 0.761, 0.893, tmp+Main.P-Source.Conc.+Nitroge n.Conc.+PL+emission_CO2+rab+His+lactate+PO4.apporte+PO4.uti lise; 0.752, 0.893, tmp+Main.P-Source.Conc.+Nitrogen.Conc.+ total.Vol+PL+emission_CO2+rab+Thr+lactate+SO4.ferm.+PO4.fer m.+PO4.apporte; 0.747, 0.893, tmp+Main.P-Source.Conc.+P-Sou rce.Feed.conc.+Nitrogen.Conc.+OD+PL+emission CO2+rab+Thr+Hi s+lactate+PO4.ferm.+PO4.apporte; 0.752, 0.893, tmp+Main.P-S ource.Conc.+P-Source.Feed.conc.+Nitrogen.Conc.+OD+total.Vol +PL+emission_CO2+His+lactate+PO4.ferm.+PO4.apporte; 0.761, 0.893, Main.P-Source.Conc.+Nitrogen.Conc.+Lys_conc.+total.V ol+PL+emission_CO2+His+lactate+PO4.ferm.+PO4.apporte; 0.747, 0.893, tmp+Main.P-Source.Conc.+P-Source.Feed.conc.+Nitroge n.Conc.+OD+PL+emission CO2+rab+Thr+His+lactate+PO4.apporte+ PO4.utilise; 0.742, 0.893, tmp+Main.P-Source.Conc.+P-Source. Feed.conc.+Nitrogen.Conc.+total.Vol+yield+PL+emission_O2+em ission_CO2+Thr+His+lactate+PO4.ferm.+PO4.apporte; 0.777, 0. 893, tmp+Main.P-Source.Conc.+Nitrogen.Conc.+emission_CO2+la ctate+PO4.apporte; 0.761, 0.893, tmp+Main.P-Source.Conc.+Ni trogen.Conc.+total.Vol+PL+emission_CO2+rab+Thr+lactate+PO4. apporte; 0.756, 0.893, Main.P-Source.Conc.+P-Source.Feed.co nc.+Nitrogen.Conc.+total.Vol+PL+emission_CO2+Thr+His+lactat e+PO4.ferm.+PO4.apporte; 0.756, 0.893, pH+Main.P-Source.Con c.+Nitrogen.Conc.+total.Vol+PL+emission_CO2+rab+His+lactate +PO4.ferm.+PO4.apporte; 0.761, 0.893, tmp+P-Source.Feed.con c.+Lys_conc.+PL+emission_CO2+Thr+His+lactate+PO4.ferm.+PO4. apporte; 0.756, 0.893, tmp+P-Source.Feed.conc.+total.Vol+PL +emission_O2+emission_CO2+Thr+His+lactate+PO4.ferm.+PO4.app orte; 0.765, 0.893, tmp+Main.P-Source.Conc.+Nitrogen.Conc.+ total.Vol+emission_O2+emission_CO2+Thr+lactate+PO4.apporte; 0.747, 0.893, tmp+Main.P-Source.Conc.+P-Source.Feed.conc.+ Nitrogen.Conc.+total.Vol+PL+emission_O2+emission_CO2+Thr+Hi s+lactate+PO4.ferm.+PO4.utilise; 0.747, 0.893, tmp+Main.P-S ource.Conc.+Nitrogen.Conc.+OD+yield+PL+emission_CO2+rab+Thr +His+lactate+PO4.apporte+PO4.utilise; 0.760, 0.893, tmp+Mai n.P-Source.Conc.+Nitrogen.Conc.+total.Vol+PL+emission_CO2+H is+lactate+SO4.ferm.+PO4.apporte; 0.756, 0.893, Main.P-Sour ce.Conc.+P-Source.Feed.conc.+Nitrogen.Conc.+total.Vol+PL+em ission_O2+emission_CO2+His+lactate+PO4.ferm.+PO4.apporte; 0. 765, 0.893, pH+tmp+Main.P-Source.Conc.+Nitrogen.Conc.+total. Vol+emission_O2+emission_CO2+lactate+PO4.apporte; 0.747, 0. 893, tmp+Main.P-Source.Conc.+P-Source.Feed.conc.+Nitrogen.C onc.+OD+total.Vol+PL+emission_O2+emission_CO2+His+lactate+P O4.ferm.+PO4.apporte; 0.756, 0.893, tmp+Main.P-Source.Conc. +P-Source.Feed.conc.+Main.Thr.Conc.+Nitrogen.Conc.+total.Vo l+emission_CO2+rab+lactate+PO4.ferm.+PO4.apporte; 0.756, 0. 893, Main.P-Source.Conc.+Nitrogen.Conc.+OD+total.Vol+PL+emi ssion_O2+emission_CO2+His+lactate+PO4.ferm.+PO4.apporte; 0. 747, 0.893, pH+tmp+Main.P-Source.Conc.+Nitrogen.Conc.+total. Vol+PL+emission_O2+emission_CO2+His+lactate+SO4.ferm.+PO4.a pporte+PO4.utilise; 0.756, 0.893, tmp+Main.P-Source.Conc.+M ain.Thr.Conc.+Nitrogen.Conc.+total.Vol+emission_CO2+rab+lac tate+SO4.ferm.+PO4.ferm.+PO4.apporte; 0.760, 0.893, tmp+Mai n.P-Source.Conc.+P-Source.Feed.conc.+Nitrogen.Conc.+total.V ol+emission_CO2+His+lactate+PO4.ferm.+PO4.apporte; 0.756, 0. 893, pH+Main.P-Source.Conc.+Nitrogen.Conc.+OD+yield+PL+emis sion_CO2+His+lactate+PO4.apporte+PO4.utilise; 0.756, 0.893, tmp+Main.P-Source.Conc.+Nitrogen.Conc.+total.Vol+PL+emissi on_O2+emission_CO2+lactate+SO4.ferm.+PO4.ferm.+PO4.apporte; 0.742, 0.893, pH+tmp+Main.P-Source.Conc.+Nitrogen.Conc.+to tal.Vol+PL+emission_O2+emission_CO2+rab+Thr+His+lactate+PO4. apporte+PO4.utilise; 0.751, 0.893, tmp+Main.P-Source.Conc.+ Nitrogen.Conc.+OD+total.Vol+emission_CO2+rab+Thr+His+lactat e+PO4.apporte+PO4.utilise; 0.747, 0.893, pH+tmp+Main.P-Sour ce.Conc.+Nitrogen.Conc.+total.Vol+PL+emission_CO2+rab+His+l actate+SO4.ferm.+PO4.apporte+PO4.utilise; 0.756, 0.893, tmp +Main.P-Source.Conc.+Nitrogen.Conc.+total.Vol+PL+emission_C O2+His+lactate+PO4.ferm.+PO4.apporte+PO4.utilise; 0.756, 0. 893, tmp+Main.P-Source.Conc.+Main.Thr.Conc.+Nitrogen.Conc.+ total.Vol+emission_CO2+rab+Thr+lactate+PO4.apporte+PO4.util ise; 0.751, 0.893, tmp+Main.P-Source.Conc.+Nitrogen.Conc.+O D+Lys_conc.+total.Vol+PL+emission_CO2+His+lactate+PO4.appor te+PO4.utilise; 0.756, 0.893, tmp+Main.P-Source.Conc.+P-Sou rce.Feed.conc.+Main.Thr.Conc.+Nitrogen.Conc.+total.Vol+emis sion_O2+emission_CO2+lactate+PO4.apporte+PO4.utilise; 0.756, 0.893, pH+Main.P-Source.Conc.+Nitrogen.Conc.+OD+yield+PL+e mission_CO2+His+lactate+PO4.ferm.+PO4.apporte; 0.751, 0.893, tmp+Main.P-Source.Conc.+Nitrogen.Conc.+total.Vol+PL+emissi on_O2+rab+Thr+His+lactate+PO4.ferm.+PO4.apporte; 0.741, 0.8 93, pH+tmp+Main.P-Source.Conc.+Nitrogen.Conc.+OD+total.Vol+ PL+emission_CO2+rab+Thr+His+lactate+PO4.ferm.+PO4.apporte; 0.751, 0.893, pH+tmp+Main.P-Source.Conc.+Nitrogen.Conc.+OD+ total.Vol+PL+emission_CO2+Thr+His+lactate+PO4.apporte; 0.76 0, 0.893, Main.P-Source.Conc.+Nitrogen.Conc.+Lys_conc.+tota l.Vol+PL+emission_CO2+His+lactate+PO4.apporte+PO4.utilise; 0.760, 0.893, tmp+Main.P-Source.Conc.+Nitrogen.Conc.+PL+emi ssion_CO2+Thr+His+lactate+PO4.apporte+PO4.utilise; 0.756, 0. 893, tmp+Main.P-Source.Conc.+P-Source.Feed.conc.+Nitrogen.C onc.+OD+PL+emission CO2+His+lactate+PO4.ferm.+PO4.apporte; 0.741, 0.893, tmp+Main.P-Source.Conc.+P-Source.Feed.conc.+N itrogen.Conc.+Lys_conc.+total.Vol+PL+emission_O2+emission_C O2+Thr+His+lactate+PO4.ferm.+PO4.apporte; 0.756, 0.893, pH+ Main.P-Source.Conc.+Nitrogen.Conc.+total.Vol+PL+emission_CO 2+rab+His+lactate+PO4.apporte+PO4.utilise; 0.741, 0.893, tm p+Main.P-Source.Conc.+Nitrogen.Conc.+OD+total.Vol+yield+PL+ emission_O2+emission_CO2+Thr+His+lactate+PO4.apporte+PO4.ut ilise; 0.746, 0.893, pH+tmp+Main.P-Source.Conc.+Nitrogen.Co nc.+Lys_conc.+total.Vol+PL+emission_CO2+rab+His+lactate+PO4. apporte+PO4.utilise; 0.769, 0.893, tmp+Main.P-Source.Conc.+ Main.Thr.Conc.+Nitrogen.Conc.+total.Vol+emission_CO2+lactat e+PO4.apporte; 0.756, 0.893, Main.P-Source.Conc.+P-Source.F eed.conc.+Nitrogen.Conc.+total.Vol+PL+emission_CO2+Thr+His+ lactate+PO4.apporte+PO4.utilise; 0.760, 0.893, tmp+Main.P-S ource.Conc.+P-Source.Feed.conc.+Nitrogen.Conc.+OD+PL+emissi on_CO2+His+lactate+PO4.apporte; 0.756, 0.893, tmp+Main.P-So urce.Conc.+Nitrogen.Conc.+OD+total.Vol+PL+emission_O2+emiss ion_CO2+lactate+PO4.ferm.+PO4.apporte; 0.741, 0.893, tmp+Ma in.P-Source.Conc.+P-Source.Feed.conc.+Nitrogen.Conc.+OD+tot al.Vol+PL+emission_CO2+rab+Thr+His+lactate+PO4.ferm.+PO4.ap porte; 0.756, 0.893, pH+tmp+Main.P-Source.Conc.+Nitrogen.Co nc.+total.Vol+emission_CO2+rab+Thr+lactate+PO4.ferm.+PO4.ap porte; 0.751, 0.893, tmp+Main.P-Source.Conc.+P-Source.Feed. conc.+Nitrogen.Conc.+total.Vol+yield+PL+emission_CO2+His+la ctate+PO4.ferm.+PO4.apporte; 0.746, 0.893, pH+tmp+Main.P-So urce.Conc.+Nitrogen.Conc.+total.Vol+PL+emission_O2+emission _CO2+rab+His+lactate+PO4.ferm.+PO4.apporte; 0.764, 0.893, M ain.P-Source.Conc.+P-Source.Feed.conc.+Nitrogen.Conc.+total. Vol+PL+emission_CO2+lactate+PO4.ferm.+PO4.apporte; 0.751, 0. 893, tmp+Main.P-Source.Conc.+P-Source.Feed.conc.+Nitrogen.C onc.+OD+total.Vol+PL+emission_CO2+His+lactate+PO4.apporte+P O4.utilise; 0.751, 0.893, tmp+Main.P-Source.Conc.+Nitrogen. Conc.+OD+total.Vol+PL+emission_CO2+rab+Thr+lactate+PO4.ferm. +PO4.apporte; 0.764, 0.893, tmp+P-Source.Feed.conc.+Main.Th r.Conc.+PL+emission_CO2+Thr+His+lactate+PO4.apporte; 0.760, 0.893, tmp+Main.P-Source.Conc.+Nitrogen.Conc.+PL+emission_ CO2+Thr+His+lactate+PO4.ferm.+PO4.apporte; 0.756, 0.893, pH +tmp+P-Source.Feed.conc.+PL+emission_O2+emission_CO2+Thr+Hi s+lactate+PO4.apporte+PO4.utilise; 0.756, 0.893, pH+Main.P-Source.Conc.+Main.Thr.Conc.+Nitrogen.Conc.+total.Vol+PL+emi ssion_CO2+His+lactate+PO4.ferm.+PO4.apporte; 0.773, 0.893, tmp+P-Source.Feed.conc.+PL+emission_CO2+His+lactate+PO4.app orte; 0.741, 0.893, pH+tmp+Main.P-Source.Conc.+Nitrogen.Con c.+total.Vol+yield+PL+emission_O2+emission_CO2+Thr+His+lact ate+PO4.apporte+PO4.utilise; 0.769, 0.893, tmp+Main.P-Sourc e.Conc.+Main.Thr.Conc.+Nitrogen.Conc.+emission_CO2+rab+lact ate+PO4.apporte; 0.756, 0.893, tmp+Main.P-Source.Conc.+Main. Thr.Conc.+Nitrogen.Conc.+total.Vol+emission_CO2+rab+lactate +SO4.ferm.+PO4.apporte+PO4.utilise; 0.756, 0.893, tmp+Main. P-Source.Conc.+Nitrogen.Conc.+total.Vol+PL+emission_CO2+rab +lactate+SO4.ferm.+PO4.ferm.+PO4.apporte; 0.764, 0.893, tmp +Main.P-Source.Conc.+Nitrogen.Conc.+total.Vol+emission_CO2+ rab+Thr+lactate+PO4.apporte; 0.760, 0.893, tmp+P-Source.Fee d.conc.+OD+PL+emission_CO2+Thr+His+lactate+PO4.ferm.+PO4.ut ilise; 0.746, 0.893, tmp+Main.P-Source.Conc.+Nitrogen.Conc. +OD+yield+PL+emission_CO2+rab+Thr+His+lactate+PO4.ferm.+PO4. apporte; 0.746, 0.893, tmp+Main.P-Source.Conc.+Main.Thr.Con c.+Nitrogen.Conc.+OD+total.Vol+PL+emission_CO2+rab+His+lact ate+PO4.ferm.+PO4.apporte; 0.764, 0.893, tmp+P-Source.Feed. conc.+yield+PL+emission_CO2+Thr+His+lactate+PO4.apporte; 0. 760, 0.893, pH+Main.P-Source.Conc.+Nitrogen.Conc.+total.Vol +PL+emission_CO2+His+lactate+PO4.ferm.+PO4.utilise; 0.760, 0.893, tmp+Main.P-Source.Conc.+Main.Thr.Conc.+Nitrogen.Conc. +OD+emission_CO2+rab+lactate+PO4.ferm.+PO4.apporte; 0.756, 0.893, tmp+Main.P-Source.Conc.+P-Source.Feed.conc.+Nitrogen. Conc.+OD+PL+emission CO2+His+lactate+PO4.apporte+PO4.utilis e; 0.764, 0.893, tmp+Main.P-Source.Conc.+P-Source.Feed.conc. +Nitrogen.Conc.+PL+emission_CO2+His+lactate+PO4.apporte; 0. 760, 0.893, tmp+P-Source.Feed.conc.+Lys_conc.+PL+emission_C O2+Thr+His+lactate+PO4.ferm.+PO4.utilise; 0.746, 0.893, pH+ tmp+Main.P-Source.Conc.+Nitrogen.Conc.+total.Vol+PL+emissio n_CO2+Thr+His+lactate+SO4.ferm.+PO4.ferm.+PO4.apporte; 0.75 6, 0.893, Main.P-Source.Conc.+Nitrogen.Conc.+OD+total.Vol+P L+emission_O2+emission_CO2+His+lactate+PO4.apporte+PO4.util ise; 0.764, 0.893, pH+tmp+Main.P-Source.Conc.+Nitrogen.Conc. +total.Vol+PL+emission_CO2+lactate+PO4.apporte; 0.751, 0.89 3, tmp+Main.P-Source.Conc.+Main.Thr.Conc.+Nitrogen.Conc.+to tal.Vol+emission_O2+emission_CO2+Thr+His+lactate+PO4.apport e+PO4.utilise; 0.751, 0.893, pH+tmp+Main.P-Source.Conc.+Nit rogen.Conc.+total.Vol+emission_O2+emission_CO2+Thr+His+lact ate+PO4.ferm.+PO4.apporte; 0.764, 0.893, pH+tmp+Main.P-Sour ce.Conc.+Nitrogen.Conc.+total.Vol+emission_CO2+lactate+PO4. ferm.+PO4.apporte; 0.756, 0.893, pH+tmp+Main.P-Source.Conc. +Nitrogen.Conc.+total.Vol+emission_O2+emission_CO2+Thr+lact ate+PO4.apporte+PO4.utilise; 0.751, 0.893, tmp+Main.P-Sourc e.Conc.+Nitrogen.Conc.+total.Vol+PL+emission_CO2+rab+Thr+la ctate+SO4.ferm.+PO4.apporte+PO4.utilise; 0.756, 0.893, tmp+ Main.P-Source.Conc.+P-Source.Feed.conc.+Main.Thr.Conc.+Nitr ogen.Conc.+total.Vol+emission_CO2+rab+lactate+PO4.apporte+P O4.utilise; 0.756, 0.893, tmp+Main.P-Source.Conc.+P-Source. Feed.conc.+Nitrogen.Conc.+total.Vol+PL+emission_O2+emission _CO2+lactate+PO4.ferm.+PO4.apporte; 0.741, 0.893, tmp+Main. P-Source.Conc.+Nitrogen.Conc.+OD+total.Vol+yield+PL+emissio n_O2+emission_CO2+Thr+His+lactate+PO4.ferm.+PO4.apporte; 0. 741, 0.893, tmp+Main.P-Source.Conc.+P-Source.Feed.conc.+Nit rogen.Conc.+total.Vol+PL+emission_CO2+rab+Thr+His+lactate+S O4.ferm.+PO4.ferm.+PO4.apporte; 0.756, 0.893, Main.P-Source. Conc.+Main.Thr.Conc.+Nitrogen.Conc.+OD+total.Vol+PL+emissio n_CO2+His+lactate+PO4.ferm.+PO4.apporte; 0.751, 0.893, tmp+ Main.P-Source.Conc.+P-Source.Feed.conc.+Nitrogen.Conc.+tota l.Vol+yield+PL+emission_CO2+His+lactate+PO4.apporte+PO4.uti lise; 0.760, 0.893, tmp+Main.P-Source.Conc.+Main.Thr.Conc.+ Nitrogen.Conc.+OD+emission_CO2+rab+lactate+PO4.apporte+PO4. utilise; 0.751, 0.893, tmp+Main.P-Source.Conc.+P-Source.Fee d.conc.+Nitrogen.Conc.+total.Vol+PL+emission_O2+emission_CO 2+Thr+His+lactate+PO4.apporte; 0.741, 0.893, pH+tmp+Main.P-Source.Conc.+Nitrogen.Conc.+OD+Lys_conc.+total.Vol+PL+emiss ion_O2+emission_CO2+His+lactate+PO4.ferm.+PO4.apporte; 0.76 0, 0.893, Main.P-Source.Conc.+Nitrogen.Conc.+OD+total.Vol+P L+emission_CO2+rab+His+lactate+PO4.apporte; 0.760, 0.893, t mp+Main.P-Source.Conc.+Nitrogen.Conc.+total.Vol+PL+emission _O2+rab+His+lactate+PO4.apporte; 0.764, 0.893, tmp+Main.P-S ource.Conc.+Nitrogen.Conc.+total.Vol+yield+emission_CO2+His +lactate+PO4.apporte; 0.755, 0.893, Main.P-Source.Conc.+P-S ource.Feed.conc.+Nitrogen.Conc.+total.Vol+PL+emission_O2+em ission_CO2+His+lactate+PO4.apporte+PO4.utilise; 0.741, 0.89 3, tmp+Main.P-Source.Conc.+P-Source.Feed.conc.+Nitrogen.Con c.+OD+total.Vol+PL+emission_CO2+rab+Thr+His+lactate+PO4.app orte+PO4.utilise; 0.751, 0.893, pH+tmp+Main.P-Source.Conc.+ P-Source.Feed.conc.+Nitrogen.Conc.+total.Vol+PL+emission_CO 2+Thr+His+lactate+PO4.apporte; 0.741, 0.893, pH+tmp+Main.P-Source.Conc.+Nitrogen.Conc.+total.Vol+yield+PL+emission_O2+ emission_CO2+Thr+His+lactate+PO4.ferm.+PO4.apporte; 0.751, 0.893, pH+tmp+Main.P-Source.Conc.+Nitrogen.Conc.+total.Vol+ PL+emission_O2+emission_CO2+Thr+His+lactate+PO4.apporte; 0. 760, 0.893, pH+tmp+Main.P-Source.Conc.+Nitrogen.Conc.+total. Vol+emission_CO2+rab+His+lactate+PO4.apporte; 0.760, 0.893, tmp+Main.P-Source.Conc.+Nitrogen.Conc.+total.Vol+emission_ O2+emission_CO2+rab+lactate+PO4.ferm.+PO4.apporte; 0.760, 0. 893, pH+tmp+Main.P-Source.Conc.+Main.Thr.Conc.+Nitrogen.Con c.+emission_CO2+rab+lactate+PO4.apporte+PO4.utilise; 0.751, 0.893, tmp+Main.P-Source.Conc.+Main.Thr.Conc.+Nitrogen.Con c.+total.Vol+emission_O2+emission_CO2+His+lactate+SO4.ferm. +PO4.ferm.+PO4.apporte; 0.746, 0.893, tmp+Main.P-Source.Con c.+P-Source.Feed.conc.+Nitrogen.Conc.+OD+total.Vol+PL+emiss ion_CO2+rab+His+lactate+PO4.ferm.+PO4.apporte; 0.755, 0.893, pH+Main.P-Source.Conc.+Main.Thr.Conc.+Nitrogen.Conc.+total. Vol+PL+emission_CO2+His+lactate+PO4.apporte+PO4.utilise; 0. 760, 0.893, tmp+P-Source.Feed.conc.+yield+PL+emission_CO2+T hr+His+lactate+PO4.ferm.+PO4.utilise; 0.751, 0.893, pH+tmp+ Main.P-Source.Conc.+Nitrogen.Conc.+OD+yield+PL+emission_O2+ emission_CO2+His+lactate+PO4.apporte; 0.760, 0.893, pH+tmp+ Main.P-Source.Conc.+Main.Thr.Conc.+Nitrogen.Conc.+emission_ CO2+rab+lactate+PO4.ferm.+PO4.apporte; 0.764, 0.893, Main.P -Source.Conc.+Nitrogen.Conc.+OD+PL+emission_O2+emission_CO2 +His+lactate+PO4.apporte; 0.746, 0.893, tmp+Main.P-Source.C onc.+P-Source.Feed.conc.+Nitrogen.Conc.+OD+total.Vol+PL+emi ssion_O2+emission_CO2+His+lactate+PO4.apporte+PO4.utilise; 0.751, 0.893, tmp+Main.P-Source.Conc.+Main.Thr.Conc.+Nitrog en.Conc.+total.Vol+PL+emission_O2+emission_CO2+Thr+lactate+ PO4.ferm.+PO4.apporte; 0.741, 0.893, pH+tmp+Main.P-Source.C onc.+Nitrogen.Conc.+total.Vol+PL+emission_O2+emission_CO2+T hr+His+lactate+SO4.ferm.+PO4.ferm.+PO4.apporte; 0.751, 0.89 3, tmp+Main.P-Source.Conc.+Nitrogen.Conc.+total.Vol+yield+P L+emission_O2+emission_CO2+Thr+lactate+PO4.apporte+PO4.util ise; 0.751, 0.893, tmp+Main.P-Source.Conc.+Nitrogen.Conc.+t otal.Vol+PL+emission_O2+rab+Thr+His+lactate+PO4.apporte+PO4. utilise; 0.746, 0.893, tmp+Main.P-Source.Conc.+P-Source.Fee d.conc.+Nitrogen.Conc.+total.Vol+yield+PL+emission_O2+emiss ion_CO2+His+lactate+PO4.apporte+PO4.utilise; 0.741, 0.893, pH+tmp+Main.P-Source.Conc.+Nitrogen.Conc.+Lys_conc.+total.V ol+PL+emission_O2+emission_CO2+Thr+His+lactate+PO4.apporte+ PO4.utilise; 0.746, 0.893, tmp+Main.P-Source.Conc.+P-Source. Feed.conc.+Nitrogen.Conc.+total.Vol+yield+PL+emission_O2+em ission_CO2+His+lactate+PO4.ferm.+PO4.apporte; 0.741, 0.893, tmp+Main.P-Source.Conc.+Nitrogen.Conc.+OD+Lys_conc.+total. Vol+PL+emission_CO2+rab+Thr+His+lactate+PO4.ferm.+PO4.appor te; 0.751, 0.893, tmp+Main.P-Source.Conc.+Nitrogen.Conc.+to tal.Vol+yield+PL+emission_CO2+His+lactate+SO4.ferm.+PO4.fer m.+PO4.apporte; 0.760, 0.893, tmp+Main.P-Source.Conc.+P-Sou rce.Feed.conc.+Main.Thr.Conc.+Nitrogen.Conc.+emission_O2+em ission_CO2+lactate+PO4.apporte+PO4.utilise; 0.768, 0.893, M ain.P-Source.Conc.+Nitrogen.Conc.+OD+total.Vol+emission_CO2 +His+lactate+PO4.apporte; 0.755, 0.893, tmp+Main.P-Source.C onc.+Nitrogen.Conc.+PL+emission_CO2+rab+Thr+His+lactate+PO4. ferm.+PO4.apporte; 0.755, 0.893, tmp+P-Source.Feed.conc.+to tal.Vol+PL+emission_CO2+rab+Thr+His+lactate+PO4.apporte+PO4. utilise; 0.760, 0.893, tmp+Main.P-Source.Conc.+Nitrogen.Con c.+total.Vol+emission_O2+rab+His+lactate+PO4.apporte+PO4.ut ilise; 0.764, 0.893, tmp+Main.P-Source.Conc.+Nitrogen.Conc. +emission_CO2+rab+His+lactate+PO4.ferm.+PO4.apporte; 0.760, 0.893, tmp+Main.P-Source.Conc.+Nitrogen.Conc.+PL+emission_ O2+emission_CO2+His+lactate+PO4.ferm.+PO4.apporte; 0.755, 0. 893, tmp+P-Source.Feed.conc.+PL+emission_O2+emission_CO2+ra b+Thr+His+lactate+PO4.ferm.+PO4.utilise; 0.741, 0.893, tmp+ Main.P-Source.Conc.+P-Source.Feed.conc.+Nitrogen.Conc.+tota l.Vol+yield+PL+emission_CO2+rab+Thr+His+lactate+PO4.apporte +PO4.utilise; 0.755, 0.893, pH+tmp+Main.P-Source.Conc.+P-So urce.Feed.conc.+Nitrogen.Conc.+total.Vol+PL+emission_CO2+Hi s+lactate+PO4.apporte; 0.755, 0.893, pH+tmp+Main.P-Source.C onc.+Nitrogen.Conc.+OD+PL+emission_O2+emission_CO2+His+lact ate+PO4.apporte; 0.746, 0.893, pH+tmp+Main.P-Source.Conc.+N itrogen.Conc.+total.Vol+PL+emission_CO2+Thr+His+lactate+SO4. ferm.+PO4.apporte+PO4.utilise; 0.755, 0.893, tmp+Main.P-Sou rce.Conc.+Nitrogen.Conc.+total.Vol+PL+emission_O2+emission CO2+lactate+SO4.ferm.+PO4.apporte+PO4.utilise; 0.751, 0.893, pH+tmp+Main.P-Source.Conc.+Nitrogen.Conc.+OD+yield+PL+emis sion_CO2+rab+His+lactate+PO4.apporte; 0.741, 0.893, tmp+Mai n.P-Source.Conc.+P-Source.Feed.conc.+Nitrogen.Conc.+total.V ol+PL+emission_CO2+rab+Thr+His+lactate+SO4.ferm.+PO4.apport e+PO4.utilise; 0.746, 0.893, tmp+Main.P-Source.Conc.+Main.T hr.Conc.+Nitrogen.Conc.+OD+total.Vol+PL+emission_CO2+rab+Hi s+lactate+PO4.apporte+PO4.utilise; 0.764, 0.893, Main.P-Sou rce.Conc.+P-Source.Feed.conc.+Nitrogen.Conc.+total.Vol+PL+e mission_CO2+lactate+PO4.apporte+PO4.utilise; 0.746, 0.893, pH+tmp+Main.P-Source.Conc.+Nitrogen.Conc.+OD+total.Vol+yiel d+PL+emission_CO2+His+lactate+PO4.ferm.+PO4.apporte; 0.751, 0.893, tmp+Main.P-Source.Conc.+Main.Thr.Conc.+Nitrogen.Con c.+total.Vol+emission_CO2+rab+Thr+His+lactate+PO4.ferm.+PO4. apporte; 0.760, 0.893, pH+tmp+Main.P-Source.Conc.+Nitrogen. Conc.+total.Vol+PL+emission_CO2+lactate+PO4.ferm.+PO4.appor te; 0.755, 0.893, tmp+Main.P-Source.Conc.+Nitrogen.Conc.+OD +total.Vol+PL+emission_CO2+rab+lactate+PO4.ferm.+PO4.apport e; 0.755, 0.893, pH+tmp+Main.P-Source.Conc.+Nitrogen.Conc.+ OD+PL+emission_CO2+rab+His+lactate+PO4.apporte; 0.760, 0.89 3, Main.P-Source.Conc.+Nitrogen.Conc.+total.Vol+PL+emission _CO2+His+lactate+PO4.ferm.+PO4.apporte+PO4.utilise; 0.755, 0.893, tmp+Main.P-Source.Cone.+Nitrogen.Cone.+PL+emission_C O2+rab+Thr+His+lactate+PO4.apporte+PO4.utilise; 0.760, 0.89 3, tmp+Main.P-Source.Conc.+P-Source.Feed.conc.+Nitrogen.Con c.+total.Vol+emission_CO2+His+lactate+PO4.apporte+PO4.utili se; 0.751, 0.893, pH+tmp+Main.P-Source.Conc.+Nitrogen.Conc. +OD+total.Vol+emission_CO2+rab+His+lactate+PO4.ferm.+PO4.ap porte; 0.760, 0.893, Main.P-Source.Conc.+Main.Thr.Conc.+Nit rogen.Conc.+OD+total.Vol+PL+emission_CO2+His+lactate+PO4.ap porte; 0.755, 0.893, tmp+P-Source.Feed.conc.+total.Vol+PL+e mission_O2+emission_CO2+Thr+His+lactate+PO4.ferm.+PO4.utili se; 0.741, 0.893, pH+tmp+Main.P-Source.Conc.+Nitrogen.Conc. +OD+total.Vol+PL+emission_CO2+rab+Thr+His+lactate+PO4.appor te+PO4.utilise; 0.755, 0.893, Main.P-Source.Conc.+Main.Thr. Conc.+Nitrogen.Conc.+OD+total.Vol+PL+emission_CO2+His+lacta te+PO4.apporte+PO4.utilise; 0.746, 0.893, tmp+Main.P-Source. Conc.+Nitrogen.Conc.+total.Vol+yield+PL+emission_O2+emissio n-C02+His+lactate+SO4.ferm.+PO4.apporte+PO4.utilise; 0.746, 0.893, tmp+Main.P-Source.Conc.+P-Source.Feed.conc.+Nitroge n.Conc.+total.Vol+PL+emission_CO2+Thr+His+lactate+SO4.ferm. +PO4.ferm.+PO4.apporte; 0.746, 0.893, tmp+Main.P-Source.Con c.+P-Source.Feed.conc.+Nitrogen.Conc.+total.Vol+PL+emission _CO2+rab+Thr+His+lactate+PO4.ferm.+PO4.utilise; 0.760, 0.89 3, tmp+Main.P-Source.Conc.+Nitrogen.Conc.+PL+emission_O2+em ission_CO2+His+lactate+PO4.apporte+PO4.utilise; 0.755, 0.89 3, tmp+Main.P-Source.Conc.+P-Source.Feed.conc.+Nitrogen.Con c.+total.Vol+emission_O2+emission_CO2+Thr+lactate+PO4.ferm. +PO4.apporte; 0.760, 0.893, tmp+Main.P-Source.Conc.+P-Sourc e.Feed.conc.+Main.Thr.Conc.+Nitrogen.Conc.+emission_O2+emis sion_CO2+lactate+PO4.ferm.+PO4.apporte; 0.751, 0.893, tmp+M ain.P-Source.Conc.+Nitrogen.Conc.+total.Vol+yield+PL+emissi on_CO2+His+lactate+SO4.ferm.+PO4.apporte+PO4.utilise; 0.751, 0.893, tmp+Main.P-Source.Conc.+Nitrogen.Conc.+OD+total.Vol +PL+emission_CO2+rab+Thr+lactate+PO4.apporte+PO4.utilise; 0. 760, 0.893, pH+tmp+P-Source.Feed.conc.+PL+emission_O2+emiss ion_CO2+Thr+His+lactate+PO4.apporte; 0.760, 0.892, pH+tmp+M ain.P-Source.Conc.+Nitrogen.Conc.+total.Vol+emission_O2+emi ssion_CO2+His+lactate+PO4.apporte; 0.760, 0.892, tmp+Main.P -Source.Conc.+Main.Thr.Conc.+Nitrogen.Conc.+total.Vol+emiss ion_CO2+His+lactate+PO4.ferm.+PO4.apporte; 0.760, 0.892, tm p+Main.P-Source.Conc.+Main.Thr.Conc.+Nitrogen.Conc.+OD+emis sion_O2+emission_CO2+lactate+PO4.ferm.+PO4.apporte; 0.750, 0.892, tmp+Main.P-Source.Conc.+Nitrogen.Conc.+Lys_conc.+tot al.Vol+PL+emission_CO2+rab+Thr+lactate+PO4.ferm.+PO4.apport e; 0.746, 0.892, tmp+Main.P-Source.Conc.+Nitrogen.Conc.+Lys _conc.+total.Vol+yield+PL+emission_O2+emission_CO2+His+lact ate+PO4.ferm.+PO4.apporte; 0.746, 0.892, pH+tmp+Main.P-Sour ce.Conc.+Nitrogen.Conc.+OD+yield+PL+emission_CO2+Thr+His+la ctate+PO4.apporte+PO4.utilise; 0.750, 0.892, tmp+Main.P-Sou rce.Conc.+P-Source.Feed.conc.+Nitrogen.Conc.+total.Vol+emis sion_O2+emission_CO2+Thr+His+lactate+PO4.ferm.+PO4.apporte; 0.741, 0.892, tmp+Main.P-Source.Conc.+P-Source.Feed.conc.+ Nitrogen.Conc.+Lys_conc.+total.Vol+PL+emission_O2+emission_ CO2+Thr+His+lactate+PO4.apporte+PO4.utilise; 0.755, 0.892, Main.P-Source.Conc.+Nitrogen.Conc.+total.Vol+yield+PL+emiss ion_O2+emission_CO2+His+lactate+PO4.ferm.+PO4.apporte; 0.75 0, 0.892, tmp+Main.P-Source.Conc.+P-Source.Feed.conc.+Nitro gen.Conc.+yield+PL+emission_CO2+Thr+His+lactate+PO4.apporte +PO4.utilise; 0.755, 0.892, tmp+Main.P-Source.Conc.+Nitroge n.Conc.+total.Vol+emission_CO2+rab+His+lactate+SO4.ferm.+PO 4.ferm.+PO4.apporte; 0.755, 0.892, Main.P-Source.Conc.+Nitr ogen.Conc.+total.Vol+PL+emission_O2+emission_CO2+Thr+His+la ctate+PO4.ferm.+PO4.apporte; 0.746, 0.892, pH+tmp+Main.P-So urce.Conc.+Nitrogen.Conc.+OD+total.Vol+yield+PL+emission_CO 2+His+lactate+PO4.apporte+PO4.utilise; 0.755, 0.892, tmp+Ma in.P-Source.Conc.+Nitrogen.Conc.+PL+emission_O2+emission_CO 2+Thr+His+lactate+PO4.ferm.+PO4.apporte; 0.750, 0.892, pH+t mp+Main.P-Source.Conc.+Nitrogen.Conc.+OD+yield+PL+emission_ CO2+Thr+His+lactate+PO4.apporte; 0.750, 0.892, tmp+Main.P-S ource.Conc.+Main.Thr.Conc.+Nitrogen.Conc.+total.Vol+emissio n_O2+emission_CO2+His+lactate+SO4.ferm.+PO4.apporte+PO4.uti lise; 0.755, 0.892, tmp+Main.P-Source.Conc.+P-Source.Feed.c onc.+Nitrogen.Conc.+total.Vol+PL+emission_CO2+rab+lactate+P O4.ferm.+PO4.apporte; 0.760, 0.892, tmp+Main.P-Source.Conc. +Main.Thr.Conc.+Nitrogen.Conc.+OD+emission_O2+emission_CO2+ lactate+PO4.apporte+PO4.utilise; 0.755, 0.892, tmp+Main.P-S ource.Conc.+Nitrogen.Conc.+OD+total.Vol+PL+emission_O2+emis sion_CO2+lactate+PO4.apporte+PO4.utilise; 0.746, 0.892, pH+ tmp+Main.P-Source.Conc.+Nitrogen.Conc.+total.Vol+PL+emissio n_O2+emission_CO2+rab+His+lactate+PO4.apporte+PO4.utilise; 0.755, 0.892, tmp+Main.P-Source.Conc.+Nitrogen.Conc.+PL+emi ssion_O2+emission_CO2+Thr+His+lactate+PO4.apporte+PO4.utili se; 0.746, 0.892, tmp+Main.P-Source.Conc.+P-Source.Feed.con c.+Nitrogen.Conc.+total.Vol+PL+emission_CO2+Thr+His+lactate +SO4.ferm.+PO4.apporte+PO4.utilise; 0.755, 0.892, pH+tmp+P-Source.Feed.conc.+PL+emission_O2+emission_CO2+Thr+His+lacta te+PO4.ferm.+PO4.apporte; 0.750, 0.892, tmp+Main.P-Source.C onc.+Nitrogen.Conc.+total.Vol+yield+PL+emission_O2+emission _CO2+Thr+lactate+PO4.ferm.+PO4.apporte; 0.755, 0.892, Main. P-Source.Conc.+Nitrogen.Conc.+total.Vol+yield+PL+emission_O 2+emission_CO2+His+lactate+PO4.apporte+PO4.utilise; 0.750, 0.892, tmp+Main.P-Source.Conc.+Main.Thr.Conc.+Nitrogen.Conc. +total.Vol+emission_O2+emission_CO2+rab+His+lactate+PO4.fer m.+PO4.apporte; 0.746, 0.892, tmp+Main.P-Source.Conc.+Nitro gen.Conc.+total.Vol+yield+PL+emission_O2+emission_CO2+His+l actate+SO4.ferm.+PO4.ferm.+PO4.apporte; 0.746, 0.892, pH+tm p+Main.P-Source.Conc.+Nitrogen.Conc.+OD+yield+PL+emission_C O2+Thr+His+lactate+PO4.ferm.+PO4.apporte; 0.759, 0.892, pH+ tmp+Main.P-Source.Conc.+Nitrogen.Conc.+total.Vol+PL+emissio n_CO2+Thr+lactate+PO4.apporte; 0.750, 0.892, tmp+Main.P-Sou rce.Conc.+Main.Thr.Conc.+Nitrogen.Conc.+OD+PL+emission_O2+e mission_CO2+His+lactate+PO4.ferm.+PO4.apporte; 0.755, 0.892, tmp+Main.P-Source.Conc.+Nitrogen.Conc.+total.Vol+PL+emissi on_CO2+rab+lactate+SO4.ferm.+PO4.apporte+PO4.utilise; 0.759, 0.892, tmp+Main.P-Source.Conc.+Nitrogen.Conc.+PL+emission CO2+rab+Thr+lactate+PO4.ferm.+PO4.apporte; 0.755, 0.892, Ma in.P-Source.Conc.+P-Source.Feed.conc.+Nitrogen.Conc.+total. Vol+PL+emission_CO2+rab+His+lactate+PO4.ferm.+PO4.apporte; 0.750, 0.892, pH+tmp+Main.P-Source.Conc.+Nitrogen.Conc.+OD+ total.Vol+yield+PL+emission_CO2+His+lactate+PO4.apporte; 0. 764, 0.892, Main.P-Source.Conc.+P-Source.Feed.conc.+Nitroge n.Conc.+total.Vol+emission_CO2+His+lactate+PO4.ferm.+PO4.ap porte; 0.750, 0.892, pH+tmp+Main.P-Source.Conc.+Nitrogen.Co nc.+Lys_conc.+total.Vol+PL+emission_CO2+His+lactate+PO4.fer m.+PO4.apporte; 0.759, 0.892, pH+Main.P-Source.Conc.+Main.T hr.Conc.+Nitrogen.Conc.+total.Vol+PL+emission_CO2+His+lacta te+PO4.apporte; 0.741, 0.892, tmp+Main.P-Source.Conc.+P-Sou rce.Feed.conc.+Nitrogen.Conc.+total.Vol+yield+PL+emission_C O2+rab+Thr+His+lactate+PO4.ferm.+PO4.apporte; 0.750, 0.892, tmp+Main.P-Source.Conc.+P-Source.Feed.conc.+Main.Thr.Conc. +Nitrogen.Conc.+total.Vol+emission_O2+emission_CO2+His+lact ate+PO4.ferm.+PO4.apporte; 0.750, 0.892, pH+tmp+Main.P-Sour ce.Conc.+Nitrogen.Conc.+OD+total.Vol+emission_O2+emission_C O2+His+lactate+PO4.ferm.+PO4.apporte; 0.750, 0.892, tmp+Mai n.P-Source.Conc.+Main.Thr.Conc.+Nitrogen.Conc.+OD+PL+emissi on_O2+emission_CO2+His+lactate+PO4.apporte+PO4.utilise; 0.7 68, 0.892, Main.P-Source.Conc.+P-Source.Feed.conc.+Nitrogen. Conc.+total.Vol+emission_CO2+lactate+PO4.ferm.+PO4.apporte; 0.750, 0.892, tmp+Main.P-Source.Conc.+Main.Thr.Conc.+Nitro gen.Conc.+total.Vol+PL+emission_O2+emission_CO2+Thr+lactate +PO4.apporte+PO4.utilise; 0.764, 0.892, pH+tmp+Main.P-Sourc e.Conc.+Nitrogen.Conc.+total.Vol+emission_CO2+lactate+PO4.a pporte+PO4.utilise; 0.745, 0.892, tmp+Main.P-Source.Conc.+N itrogen.Conc.+OD+Lys_conc.+total.Vol+PL+emission_CO2+Thr+Hi s+lactate+PO4.ferm.+PO4.apporte; 0.772, 0.892, tmp+Main.P-S ource.Conc.+Nitrogen.Conc.+emission_CO2+rab+lactate+PO4.app orte; 0.764, 0.892, tmp+Main.P-Source.Conc.+Nitrogen.Conc.+ PL+emission_CO2+rab+lactate+PO4.ferm.+PO4.apporte; 0.750, 0. 892, pH+tmp+Main.P-Source.Conc.+Nitrogen.Conc.+total.Vol+PL +emission_O2+rab+His+lactate+PO4.ferm.+PO4.apporte; 0.759, 0.892, Main.P-Source.Conc.+Nitrogen.Conc.+OD+total.Vol+PL+e mission_CO2+His+lactate+PO4.ferm.+PO4.utilise; 0.755, 0.892, tmp+Main.P-Source.Conc.+P-Source.Feed.conc.+Nitrogen.Conc. +PL+emission_CO2+Thr+His+lactate+PO4.ferm.+PO4.utilise; 0.7 50, 0.892, pH+tmp+Main.P-Source.Conc.+Nitrogen.Conc.+total. Vol+emission_CO2+rab+Thr+His+lactate+PO4.ferm.+PO4.apporte; 0.755, 0.892, Main.P-Source.Conc.+Nitrogen.Conc.+OD+total. Vol+PL+emission_CO2+rab+His+lactate+PO4.ferm.+PO4.apporte; 0.759, 0.892, tmp+Main.P-Source.Conc.+Nitrogen.Conc.+PL+emi ssion_O2+emission_CO2+Thr+lactate+PO4.ferm.+PO4.apporte; 0. 755, 0.892, tmp+Main.P-Source.Conc.+Nitrogen.Conc.+Lys_conc. +total.Vol+PL+emission_O2+emission_CO2+lactate+PO4.ferm.+PO 4.apporte; 0.750, 0.892, tmp+Main.P-Source.Conc.+Main.Thr.C onc.+Nitrogen.Conc.+total.Vol+yield+emission_O2+emission_CO 2+His+lactate+PO4.apporte+PO4.utilise; 0.745, 0.892, tmp+Ma in.P-Source.Conc.+P-Source.Feed.conc.+Main.Thr.Conc.+Nitrog en.Conc.+total.Vol+PL+emission_CO2+Thr+His+lactate+PO4.ferm. +PO4.apporte; 0.764, 0.892, tmp+Main.P-Source.Conc.+Nitroge n.Conc.+emission_CO2+rab+Thr+lactate+PO4.ferm.+PO4.apporte; 0.755, 0.892, tmp+Main.P-Source.Conc.+Nitrogen.Conc.+total. Vol+emission_O2+emission_CO2+His+lactate+SO4.ferm.+PO4.ferm. +PO4.apporte; 0.740, 0.892, tmp+Main.P-Source.Conc.+Nitroge n.Conc.+Lys_conc.+total.Vol+PL+emission_O2+emission_CO2+rab +Thr+His+lactate+PO4.ferm.+PO4.apporte; 0.750, 0.892, tmp+M ain.P-Source.Conc.+P-Source.Feed.conc.+Main.Thr.Conc.+Nitro gen.Conc.+total.Vol+emission CO2+rab+His+lactate+PO4.ferm.+ PO4.apporte; 0.768, 0.892, tmp+Main.P-Source.Conc.+Nitrogen. Conc.+total.Vol+emission_CO2+lactate+PO4.ferm.+PO4.utilise; 0.740, 0.892, pH+tmp+Main.P-Source.Conc.+Nitrogen.Conc.+to tal.Vol+PL+emission_O2+emission_CO2+Thr+His+lactate+SO4.fer m.+PO4.apporte+PO4.utilise; 0.750, 0.892, tmp+Main.P-Source. Conc.+P-Source.Feed.conc.+Nitrogen.Conc.+total.Vol+PL+emiss ion_CO2+rab+Thr+His+lactate+PO4.apporte; 0.750, 0.892, pH+t mp+Main.P-Source.Conc.+Nitrogen.Conc.+total.Vol+PL+emission _CO2+rab+Thr+His+lactate+PO4.apporte; 0.772, 0.892, Main.P-Source.Conc.+P-Source.Feed.conc.+Nitrogen.Conc.+total.Vol+e mission_CO2+lactate+PO4.apporte; 0.755, 0.892, Main.P-Sourc e.Conc.+Nitrogen.Conc.+total.Vol+PL+emission_O2+emission_CO 2+Thr+His+lactate+PO4.apporte+PO4.utilise; 0.759, 0.892, pH +tmp+Main.P-Source.Conc.+Nitrogen.Conc.+total.Vol+PL+emissi on_CO2+lactate+PO4.apporte+PO4.utilise; 0.745, 0.892, tmp+M ain.P-Source.Conc.+P-Source.Feed.conc.+Nitrogen.Conc.+total. Vol+yield+PL+emission_CO2+rab+His+lactate+PO4.ferm.+PO4.app orte; 0.755, 0.892, pH+tmp+Main.P-Source.Conc.+Nitrogen.Con c.+total.Vol+emission_CO2+rab+Thr+lactate+PO4.apporte+PO4.u tilise; 0.745, 0.892, tmp+Main.P-Source.Conc.+P-Source.Feed. conc.+Nitrogen.Conc.+total.Vol+yield+PL+emission_CO2+rab+Hi s+lactate+PO4.apporte+PO4.utilise; 0.764, 0.892, tmp+Main.P -Source.Conc.+Nitrogen.Conc.+emission_CO2+rab+His+lactate+P O4.apporte+PO4.utilise; 0.745, 0.892, tmp+Main.P-Source.Con c.+P-Source.Feed.conc.+Nitrogen.Conc.+OD+total.Vol+PL+emiss ion_CO2+rab+His+lactate+PO4.apporte+PO4.utilise; 0.755, 0.8 92, tmp+Main.P-Source.Conc.+P-Source.Feed.conc.+Nitrogen.Co nc.+total.Vol+PL+emission_O2+emission_CO2+lactate+PO4.appor te+PO4.utilise; 0.759, 0.892, tmp+Main.P-Source.Conc.+Nitro gen.Conc.+PL+emission_CO2+rab+Thr+lactate+PO4.apporte+PO4.u tilise; 0.745, 0.892, tmp+Main.P-Source.Conc.+Nitrogen.Conc. +Lys_conc.+total.Vol+PL+emission_O2+emission_CO2+His+lactat e+SO4.ferm.+PO4.ferm.+PO4.apporte; 0.755, 0.892, tmp+Main.P -Source.Conc.+P-Source.Feed.conc.+Nitrogen.Conc.+total.Vol+ PL+emission_CO2+His+lactate+PO4.ferm.+PO4.utilise; 0.745, 0. 892, tmp+Main.P-Source.Conc.+Nitrogen.Conc.+total.Vol+yield +PL+emission_CO2+rab+His+lactate+SO4.ferm.+PO4.apporte+PO4. utilise; 0.755, 0.892, tmp+Main.P-Source.Conc.+Nitrogen.Con c.+total.Vol+emission_O2+emission_CO2+rab+Thr+lactate+PO4.f erm.+PO4.apporte; 0.759, 0.892, tmp+Main.P-Source.Conc.+Mai n.Thr.Conc.+Nitrogen.Conc.+total.Vol+PL+emission_CO2+His+la ctate+PO4.apporte; 0.759, 0.892, tmp+Main.P-Source.Conc.+Ni trogen.Conc.+PL+emission_O2+emission_CO2+Thr+lactate+PO4.ap porte+PO4.utilise; 0.740, 0.892, tmp+Main.P-Source.Conc.+P-Source.Feed.conc.+Main.Thr.Conc.+Nitrogen.Conc.+total.Vol+P L+emission_O2+emission_CO2+Thr+His+lactate+PO4.ferm.+PO4.ap porte; 0.750, 0.892, tmp+Main.P-Source.Conc.+Main.Thr.Conc. +Nitrogen.Conc.+total.Vol+yield+emission_CO2+rab+His+lactat e+PO4.apporte+PO4.utilise; 0.745, 0.892, tmp+Main.P-Source. Conc.+Main.Thr.Conc.+Nitrogen.Conc.+total.Vol+yield+PL+emis sion_O2+emission_CO2+His+lactate+PO4.apporte+PO4.utilise; 0. 755, 0.892, tmp+P-Source.Feed.conc.+OD+PL+emission_O2+emiss ion_CO2+Thr+His+lactate+PO4.apporte+PO4.utilise; 0.750, 0.8 92, pH+tmp+Main.P-Source.Conc.+Main.Thr.Conc.+Nitrogen.Conc. +total.Vol+PL+emission_CO2+His+lactate+PO4.ferm.+PO4.apport e; 0.763, 0.892, tmp+Main.P-Source.Conc.+Main.Thr.Conc.+Nit rogen.Conc.+total.Vol+emission_CO2+lactate+PO4.ferm.+PO4.ap porte; 0.750, 0.892, tmp+Main.P-Source.Conc.+P-Source.Feed. conc.+Nitrogen.Conc.+yield+PL+emission_CO2+Thr+His+lactate+ PO4.ferm.+PO4.apporte; 0.755, 0.892, pH+tmp+P-Source.Feed.c onc.+PL+emission_CO2+rab+Thr+His+lactate+PO4.apporte+PO4.ut ilise; 0.745, 0.892, tmp+Main.P-Source.Conc.+P-Source.Feed. conc.+Nitrogen.Conc.+total.Vol+PL+emission_O2+emission_CO2+ His+lactate+SO4.ferm.+PO4.ferm.+PO4.apporte; 0.745, 0.892, tmp+Main.P-Source.Conc.+Nitrogen.Conc.+total.Vol+yield+PL+e mission_CO2+rab+His+lactate+SO4.ferm.+PO4.ferm.+PO4.apport e; 0.772, 0.892, tmp+Main.P-Source.Conc.+Nitrogen.Conc.+emi ssion_CO2+lactate+PO4.ferm.+PO4.apporte; 0.755, 0.892, tmp+ P-Source.Feed.conc.+total.Vol+PL+emission_CO2+rab+Thr+His+l actate+PO4.ferm.+PO4.apporte; 0.763, 0.892, Main.P-Source.C onc.+Nitrogen.Conc.+OD+PL+emission CO2+rab+His+lactate+PO4. apporte; 0.745, 0.892, pH+tmp+Main.P-Source.Conc.+Nitrogen. Conc.+OD+PL+emission_O2+emission_CO2+Thr+His+lactate+PO4.fe rm.+PO4.apporte; 0.750, 0.892, tmp+Main.P-Source.Conc.+Main. Thr.Conc.+Nitrogen.Conc.+total.Vol+emission_CO2+rab+His+lac tate+SO4.ferm.+PO4.ferm.+PO4.apporte; 0.763, 0.892, tmp+Mai n.P-Source.Conc.+Nitrogen.Conc.+PL+emission_CO2+rab+lactate +PO4.apporte+PO4.utilise; 0.745, 0.892, tmp+Main.P-Source.C onc.+Nitrogen.Conc.+Lys_conc.+total.Vol+yield+PL+emission_O 2+emission_CO2+His+lactate+PO4.apporte+PO4.utilise; 0.750, 0.892, tmp+Main.P-Source.Conc.+Main.Thr.Conc.+Nitrogen.Conc. +total.Vol+yield+emission_O2+emission_CO2+His+lactate+PO4.f erm.+PO4.apporte; 0.740, 0.892, pH+tmp+Main.P-Source.Conc.+ Nitrogen.Conc.+total.Vol+yield+PL+emission_CO2+rab+Thr+His+ lactate+PO4.apporte+PO4.utilise; 0.759, 0.892, tmp+Main.P-S ource.Conc.+P-Source.Feed.conc.+Nitrogen.Conc.+emission_CO2 +rab+Thr+lactate+PO4.ferm.+PO4.apporte; 0.763, 0.892, tmp+M ain.P-Source.Conc.+P-Source.Feed.conc.+Nitrogen.Conc.+emiss ion_CO2+rab+lactate+PO4.ferm.+PO4.apporte; 0.750, 0.892, pH +tmp+Main.P-Source.Conc.+Nitrogen.Conc.+total.Vol+PL+emissi on_CO2+Thr+His+lactate+PO4.ferm.+PO4.utilise; 0.759, 0.892, tmp+Main.P-Source.Conc.+Nitrogen.Conc.+total.Vol+emission_ CO2+rab+lactate+SO4.ferm.+PO4.ferm.+PO4.apporte; 0.759, 0.8 92, Main.P-Source.Conc.+P-Source.Feed.conc.+Nitrogen.Conc.+ OD+yield+PL+emission_CO2+His+lactate+PO4.apporte; 0.767, 0. 892, tmp+Main.P-Source.Conc.+Main.Thr.Conc.+Nitrogen.Conc.+ emission_O2+emission_CO2+lactate+PO4.apporte; 0.750, 0.892, tmp+Main.P-Source.Conc.+Main.Thr.Conc.+Nitrogen.Conc.+tota l.Vol+emission_CO2+rab+Thr+His+lactate+PO4.apporte+PO4.util ise; 0.745, 0.892, tmp+Main.P-Source.Conc.+P-Source.Feed.co nc.+Main.Thr.Conc.+Nitrogen.Conc.+total.Vol+PL+emission_CO2 +Thr+His+lactate+PO4.apporte+PO4.utilise; 0.750, 0.892, pH+ tmp+Main.P-Source.Conc.+Nitrogen.Conc.+total.Vol+emission_O 2+emission CO2+Thr+His+lactate+PO4.apporte+PO4.utilise; 0.7 50, 0.892, tmp+Main.P-Source.Conc.+Main.Thr.Conc.+Nitrogen. Conc.+total.Vol+emission_O2+emission_CO2+rab+His+lactate+PO 4.apporte+PO4.utilise; 0.750, 0.892, tmp+Main.P-Source.Conc. +Main.Thr.Conc.+Nitrogen.Conc.+total.Vol+yield+emission_CO2 +rab+His+lactate+PO4.ferm.+PO4.apporte; 0.754, 0.892, tmp+M ain.P-Source.Conc.+Nitrogen.Conc.+OD+total.Vol+PL+emission_ CO2+rab+lactate+PO4.apporte+PO4.utilise; 0.754, 0.892, tmp+ Main.P-Source.Conc.+P-Source.Feed.conc.+Nitrogen.Conc.+tota l.Vol+PL+emission_CO2+Thr+lactate+PO4.ferm.+PO4.apporte; 0. 767, 0.892, Main.P-Source.Conc.+P-Source.Feed.conc.+Nitroge n.Conc.+total.Vol+PL+emission_CO2+lactate+PO4.apporte; 0.76 3, 0.892, tmp+Main.P-Source.Conc.+P-Source.Feed.conc.+Nitro gen.Conc.+PL+emission_CO2+Thr+lactate+PO4.apporte; 0.767, 0. 892, Main.P-Source.Conc.+Nitrogen.Conc.+total.Vol+yield+emi ssion_CO2+His+lactate+PO4.apporte; 0.750, 0.892, tmp+Main.P -Source.Conc.+Nitrogen.Conc.+total.Vol+emission_O2+emission _CO2+rab+Thr+His+lactate+PO4.ferm.+PO4.apporte; 0.750, 0.89 2, tmp+Main.P-Source.Conc.+Main.Thr.Conc.+Nitrogen.Conc.+OD +PL+emission_CO2+rab+His+lactate+PO4.ferm.+PO4.apporte; 0.7 54, 0.892, Main.P-Source.Conc.+Main.Thr.Conc.+Nitrogen.Conc. +total.Vol+yield+PL+emission_CO2+His+lactate+PO4.ferm.+PO4. apporte; 0.745, 0.892, tmp+Main.P-Source.Conc.+Main.Thr.Con c.+Nitrogen.Conc.+Lys_conc.+total.Vol+PL+emission_O2+emissi on_CO2+His+lactate+PO4.ferm.+PO4.apporte; 0.767, 0.892, tmp +P-Source.Feed.conc.+PL+emission_CO2+His+lactate+PO4.apport e+PO4.utilise; 0.754, 0.892, Main.P-Source.Conc.+Nitrogen.C onc.+OD+total.Vol+PL+emission_CO2+rab+His+lactate+PO4.appor te+PO4.utilise; 0.750, 0.892, tmp+Main.P-Source.Conc.+P-Sou rce.Feed.conc.+Main.Thr.Conc.+Nitrogen.Conc.+total.Vol+emis sion_O2+emission_CO2+His+lactate+PO4.apporte+PO4.utilise; 0. 750, 0.892, pH+tmp+Main.P-Source.Conc.+Nitrogen.Conc.+OD+to tal.Vol+PL+emission_O2+emission_CO2+His+lactate+PO4.apport e; 0.759, 0.892, tmp+Main.P-Source.Conc.+Nitrogen.Conc.+tot al.Vol+emission_O2+emission_CO2+rab+lactate+PO4.apporte+PO4. utilise; 0.754, 0.892, tmp+Main.P-Source.Conc.+Nitrogen.Con c.+total.Vol+yield+PL+emission_O2+emission_CO2+lactate+PO4. apporte+PO4.utilise; 0.740, 0.892, tmp+Main.P-Source.Conc.+ P-Source.Feed.conc.+Main.Thr.Conc.+Nitrogen.Conc.+total.Vol +PL+emission_O2+emission_CO2+Thr+His+lactate+PO4.apporte+PO 4.utilise; 0.763, 0.892, tmp+Main.P-Source.Conc.+Nitrogen.C onc.+OD+total.Vol+emission_CO2+lactate+PO4.ferm.+PO4.apport e; 0.754, 0.892, tmp+Main.P-Source.Conc.+Nitrogen.Conc.+Lys _conc.+total.Vol+emission_O2+emission_CO2+His+lactate+PO4.f erm.+PO4.apporte; 0.759, 0.892, tmp+Main.P-Source.Conc.+Mai n.Thr.Conc.+Nitrogen.Conc.+total.Vol+emission_CO2+His+lacta te+PO4.apporte+PO4.utilise; 0.745, 0.892, tmp+Main.P-Source. Conc.+P-Source.Feed.conc.+Nitrogen.Conc.+total.Vol+PL+emiss ion_CO2+rab+His+lactate+SO4.ferm.+PO4.ferm.+PO4.apporte; 0. 740, 0.892, pH+tmp+Main.P-Source.Conc.+Nitrogen.Conc.+total. Vol+yield+PL+emission_CO2+rab+Thr+His+lactate+PO4.ferm.+PO4. apporte; 0.759, 0.892, pH+tmp+P-Source.Feed.conc.+PL+emissi on_CO2+rab+Thr+His+lactate+PO4.apporte; 0.754, 0.892, tmp+M ain.P-Source.Conc.+Nitrogen.Conc.+total.Vol+yield+PL+emissi on_O2+emission_CO2+lactate+PO4.ferm.+PO4.apporte; 0.759, 0. 892, Main.P-Source.Conc.+Nitrogen.Conc.+OD+yield+PL+emissio n_O2+emission_CO2+His+lactate+PO4.apporte; 0.754, 0.892, Ma in.P-Source.Conc.+P-Source.Feed.conc.+Nitrogen.Conc.+total. Vol+PL+emission_CO2+rab+His+lactate+PO4.apporte+PO4.utilis e; 0.750, 0.892, tmp+Main.P-Source.Conc.+Main.Thr.Conc.+Nit rogen.Conc.+OD+PL+emission_CO2+rab+His+lactate+PO4.apporte+ PO4.utilise; 0.750, 0.892, tmp+Main.P-Source.Conc.+P-Source. Feed.conc.+Nitrogen.Conc.+OD+PL+emission_CO2+rab+His+lactat e+PO4.ferm.+PO4.apporte; 0.750, 0.892, tmp+Main.P-Source.Co nc.+P-Source.Feed.conc.+Nitrogen.Conc.+total.Vol+emission_C O2+rab+Thr+His+lactate+PO4.ferm.+PO4.apporte; 0.740, 0.892, tmp+Main.P-Source.Conc.+Nitrogen.Conc.+OD+Lys_conc.+total. Vol+PL+emission_CO2+rab+Thr+His+lactate+PO4.apporte+PO4.uti lise; 0.754, 0.892, Main.P-Source.Conc.+Main.Thr.Conc.+Nitr ogen.Conc.+total.Vol+yield+PL+emission_CO2+His+lactate+PO4. apporte+PO4.utilise; 0.759, 0.892, tmp+Main.P-Source.Conc.+ Nitrogen.Conc.+total.Vol+emission_O2+emission_CO2+lactate+S O4.ferm.+PO4.ferm.+PO4.apporte; 0.750, 0.892, tmp+Main.P-So urce.Conc.+P-Source.Feed.conc.+Nitrogen.Conc.+OD+PL+emissio n_O2+emission_CO2+His+lactate+PO4.ferm.+PO4.apporte; 0.750, 0.892, pH+tmp+Main.P-Source.Conc.+Nitrogen.Conc.+Lys_conc. +total.Vol+PL+emission_CO2+His+lactate+PO4.apporte+PO4.util ise; 0.745, 0.892, tmp+Main.P-Source.Conc.+Nitrogen.Conc.+O D+Lys_conc.+total.Vol+PL+emission_CO2+Thr+His+lactate+PO4.a pporte+PO4.utilise; 0.759, 0.892, pH+tmp+Main.P-Source.Conc. +Nitrogen.Conc.+total.Vol+emission_CO2+His+lactate+PO4.ferm. +PO4.utilise; 0.750, 0.892, pH+tmp+Main.P-Source.Conc.+Nitr ogen.Conc.+OD+total.Vol+emission_CO2+rab+His+lactate+PO4.ap porte+PO4.utilise; 0.740, 0.892, tmp+Main.P-Source.Conc.+Ni trogen.Conc.+OD+total.Vol+yield+PL+emission_CO2+rab+Thr+His +lactate+PO4.apporte+PO4.utilise; 0.759, 0.892, tmp+Main.P-Source.Conc.+P-Source.Feed.conc.+Nitrogen.Conc.+emission_O2 +emission_CO2+Thr+lactate+PO4.ferm.+PO4.apporte; 0.750, 0.8 92, tmp+Main.P-Source.Conc.+P-Source.Feed.conc.+Nitrogen.Co nc.+PL+emission_CO2+rab+Thr+His+lactate+PO4.ferm.+PO4.utili se; 0.750, 0.892, pH+tmp+Main.P-Source.Conc.+Nitrogen.Conc. +total.Vol+PL+emission_O2+rab+His+lactate+PO4.apporte+PO4.u tilise; 0.763, 0.892, tmp+Main.P-Source.Conc.+Nitrogen.Conc. +emission_O2+emission_CO2+Thr+lactate+PO4.ferm.+PO4.apport e; 0.771, 0.892, Main.P-Source.Conc.+Nitrogen.Conc.+OD+tota l.Vol+emission_CO2+lactate+PO4.apporte; 0.750, 0.892, tmp+M ain.P-Source.Conc.+P-Source.Feed.conc.+Main.Thr.Conc.+Nitro gen.Conc.+total.Vol+emission_CO2+rab+His+lactate+PO4.apport e+PO4.utilise; 0.763, 0.892, tmp+Main.P-Source.Conc.+Nitrog en.Conc.+emission_CO2+rab+Thr+lactate+PO4.apporte+PO4.utili se; 0.740, 0.892, pH+tmp+Main.P-Source.Conc.+Nitrogen.Conc. +total.Vol+PL+emission_CO2+rab+Thr+His+lactate+SO4.ferm.+PO 4.ferm.+PO4.apporte; 0.771, 0.892, tmp+Main.P-Source.Conc.+ Nitrogen.Conc.+emission_CO2+lactate+PO4.apporte+PO4.utilis e; 0.754, 0.892, tmp+Main.P-Source.Conc.+P-Source.Feed.conc. +Nitrogen.Conc.+total.Vol+emission_O2+emission_CO2+Thr+lact ate+PO4.apporte+PO4.utilise; 0.750, 0.892, pH+tmp+Main.P-So urce.Conc.+Main.Thr.Conc.+Nitrogen.Conc.+total.Vol+PL+emiss ion_CO2+His+lactate+PO4.apporte+PO4.utilise; 0.754, 0.892, tmp+Main.P-Source.Conc.+P-Source.Feed.conc.+Nitrogen.Conc.+ total.Vol+PL+emission_CO2+rab+lactate+PO4.apporte+PO4.utili se; 0.750, 0.892, tmp+Main.P-Source.Conc.+P-Source.Feed.con c.+Nitrogen.Conc.+PL+emission_O2+emission_CO2+Thr+His+lacta te+PO4.ferm.+PO4.utilise; 0.767, 0.892, Main.P-Source.Conc. +Nitrogen.Conc.+OD+total.Vol+PL+emission_CO2+lactate+PO4.ap porte; 0.763, 0.892, Main.P-Source.Conc.+Nitrogen.Conc.+OD+ PL+emission_CO2+Thr+His+lactate+PO4.apporte; 0.767, 0.892, tmp+Main.P-Source.Conc.+Nitrogen.Conc.+OD+total.Vol+emissio n_CO2+lactate+PO4.apporte; 0.759, 0.892, Main.P-Source.Conc. +Nitrogen.Conc.+total.Vol+yield+PL+emission_CO2+Thr+His+lac tate+PO4.apporte; 0.754, 0.892, tmp+Main.P-Source.Conc.+Mai n.Thr.Conc.+Nitrogen.Conc.+total.Vol+emission_O2+emission_C O2+rab+lactate+PO4.ferm.+PO4.apporte; 0.767, 0.892, Main.P-Source.Conc.+Nitrogen.Conc.+total.Vol+PL+emission_O2+emissi on_CO2+lactate+PO4.apporte; 0.750, 0.892, tmp+Main.P-Source. Conc.+Nitrogen.Conc.+OD+total.Vol+PL+emission_O2+emission_C O2+His+lactate+PO4.ferm.+PO4.utilise; 0.767, 0.892, Main.P-Source.Conc.+P-Source.Feed.conc.+Nitrogen.Conc.+total.Vol+e mission_CO2+lactate+PO4.apporte+PO4.utilise; 0.754, 0.892, Main.P-Source.Conc.+Nitrogen.Conc.+total.Vol+PL+emission_CO 2+rab+Thr+His+lactate+PO4.ferm.+PO4.apporte; 0.740, 0.892, pH+tmp+Main.P-Source.Conc.+Nitrogen.Conc.+OD+Lys_conc.+tota l.Vol+PL+emission_O2+emission_CO2+His+lactate+PO4.apporte+P O4.utilise; 0.763, 0.892, Main.P-Source.Conc.+P-Source.Feed. conc.+Nitrogen.Conc.+total.Vol+emission_CO2+His+lactate+PO4. apporte+PO4.utilise; 0.754, 0.892, tmp+Main.P-Source.Conc.+ Nitrogen.Conc.+OD+total.Vol+emission_CO2+Thr+His+lactate+PO 4.ferm.+PO4.apporte; 0.740, 0.892, tmp+Main.P-Source.Conc.+ Nitrogen.Conc.+Lys_conc.+total.Vol+PL+emission_O2+emission CO2+Thr+His+lactate+SO4.ferm.+PO4.ferm.+PO4.apporte; 0.754, 0.892, tmp+Main.P-Source.Conc.+P-Source.Feed.conc.+Nitroge n.Conc.+total.Vol+emission_CO2+rab+Thr+lactate+PO4.ferm.+PO 4.apporte; 0.759, 0.892, pH+tmp+P-Source.Feed.conc.+total.V ol+PL+emission_CO2+Thr+His+lactate+PO4.apporte; 0.763, 0.89 2, pH+Main.P-Source.Conc.+Nitrogen.Conc.+total.Vol+PL+emiss ion_CO2+lactate+PO4.ferm.+PO4.apporte; 0.767, 0.892, Main.P -Source.Conc.+Nitrogen.Conc.+total.Vol+PL+emission_CO2+Thr+ lactate+PO4.apporte; 0.745, 0.892, tmp+Main.P-Source.Conc.+ Main.Thr.Conc.+Nitrogen.Conc.+total.Vol+yield+PL+emission_O 2+emission_CO2+His+lactate+PO4.ferm.+PO4.apporte; 0.750, 0. 892, pH+Main.P-Source.Conc.+Nitrogen.Conc.+OD+total.Vol+yie ld+PL+emission_CO2+His+lactate+PO4.ferm.+PO4.apporte; 0.754, 0.892, pH+tmp+Main.P-Source.Conc.+Nitrogen.Conc.+total.Vol +emission_CO2+rab+His+lactate+PO4.ferm.+PO4.utilise; 0.740, 0.892, tmp+Main.P-Source.Conc.+Nitrogen.Conc.+OD+total.Vol +yield+PL+emission_CO2+rab+Thr+His+lactate+PO4.ferm.+PO4.ap porte; 0.754, 0.892, pH+tmp+P-Source.Feed.conc.+PL+emission _O2+emission_CO2+Thr+His+lactate+PO4.ferm.+PO4.utilise; 0.7 59, 0.892, Main.P-Source.Conc.+P-Source.Feed.conc.+Nitrogen. Conc.+total.Vol+yield+PL+emission_CO2+His+lactate+PO4.appor te; 0.754, 0.892, tmp+Main.P-Source.Conc.+P-Source.Feed.con c.+Nitrogen.Conc.+total.Vol+PL+emission_CO2+Thr+lactate+PO4. apporte+PO4.utilise; 0.750, 0.892, tmp+Main.P-Source.Conc.+ Main.Thr.Conc.+Nitrogen.Conc.+OD+total.Vol+PL+emission_CO2+ His+lactate+PO4.ferm.+PO4.apporte; 0.763, 0.892, tmp+Main.P -Source.Conc.+Main.Thr.Conc.+Nitrogen.Conc.+total.Vol+emiss ion_CO2+lactate+PO4.apporte+PO4.utilise; 0.749, 0.892, tmp+ Main.P-Source.Conc.+P-Source.Feed.conc.+Nitrogen.Conc.+tota l.Vol+emission_O2+emission_CO2+Thr+His+lactate+PO4.apporte+ PO4.utilise; 0.740, 0.892, pH+tmp+Main.P-Source.Conc.+Nitro gen.Conc.+Lys_conc.+total.Vol+PL+emission_CO2+rab+Thr+His+l actate+PO4.ferm.+PO4.apporte; 0.754, 0.892, tmp+Main.P-Sour ce.Conc.+Main.Thr.Conc.+Nitrogen.Conc.+OD+emission_CO2+rab+ His+lactate+PO4.ferm.+PO4.apporte; 0.749, 0.892, pH+tmp+Mai n.P-Source.Conc.+Nitrogen.Conc.+OD+total.Vol+PL+emission_CO 2+rab+His+lactate+PO4.apporte; 0.749, 0.892, pH+Main.P-Sour ce.Conc.+Nitrogen.Conc.+OD+total.Vol+yield+PL+emission_CO2+ His+lactate+PO4.apporte+PO4.utilise; 0.749, 0.892, tmp+Main. P-Source.Conc.+Main.Thr.Conc.+Nitrogen.Conc.+total.Vol+emis sion_CO2+rab+His+lactate+SO4.ferm.+PO4.apporte+PO4.utilise; 0.759, 0.892, pH+tmp+Main.P-Source.Conc.+Main.Thr.Conc.+Ni trogen.Conc.+emission_O2+emission_CO2+lactate+PO4.apporte+P O4.utilise; 0.745, 0.892, pH+tmp+Main.P-Source.Conc.+Nitrog en.Conc.+OD+PL+emission_O2+emission_CO2+Thr+His+lactate+PO4. apporte+PO4.utilise; 0.759, 0.892, tmp+Main.P-Source.Conc.+ P-Source.Feed.conc.+Nitrogen.Conc.+PL+emission_CO2+Thr+lact ate+PO4.ferm.+PO4.apporte; 0.740, 0.892, tmp+Main.P-Source. Conc.+Nitrogen.Conc.+OD+total.Vol+PL+emission_O2+emission_C O2+rab+Thr+His+lactate+PO4.ferm.+PO4.apporte; 0.745, 0.892, tmp+Main.P-Source.Conc.+P-Source.Feed.conc.+Nitrogen.Conc. +total.Vol+PL+emission_O2+emission_CO2+His+lactate+SO4.ferm. +PO4.apporte+PO4.utilise; 0.763, 0.892, tmp+Main.P-Source.C onc.+P-Source.Feed.conc.+Nitrogen.Conc.+emission_CO2+rab+la ctate+PO4.apporte+PO4.utilise; 0.759, 0.892, tmp+Main.P-Sou rce.Conc.+P-Source.Feed.conc.+Nitrogen.Conc.+PL+emission_CO 2+Thr+lactate+PO4.apporte+PO4.utilise; 0.759, 0.892, tmp+Ma in.P-Source.Conc.+Nitrogen.Conc.+total.Vol+emission_CO2+His +lactate+SO4.ferm.+PO4.ferm.+PO4.apporte; 0.759, 0.892, tmp +Main.P-Source.Conc.+P-Source.Feed.conc.+Nitrogen.Conc.+emi ssion_CO2+rab+Thr+lactate+PO4.apporte+PO4.utilise; 0.749, 0. 892, tmp+Main.P-Source.Conc.+Nitrogen.Conc.+Lys_conc.+total. Vol+PL+emission_CO2+rab+Thr+lactate+PO4.apporte+PO4.utilis e; 0.740, 0.892, tmp+Main.P-Source.Conc.+Nitrogen.Conc.+Lys _conc.+total.Vol+PL+emission_O2+emission_CO2+rab+Thr+His+la ctate+PO4.apporte+PO4.utilise; 0.754, 0.892, tmp+Main.P-Sou rce.Conc.+Nitrogen.Conc.+total.Vol+PL+emission_CO2+Thr+His+ lactate+SO4.ferm.+PO4.apporte; 0.754, 0.892, pH+tmp+Main.P-Source.Conc.+Nitrogen.Conc.+total.Vol+emission_CO2+Thr+His+ lactate+PO4.ferm.+PO4.apporte; 0.749, 0.892, pH+tmp+Main.P-Source.Conc.+Nitrogen.Conc.+OD+total.Vol+emission_O2+emissi on_CO2+His+lactate+PO4.apporte+PO4.utilise; 0.745, 0.892, p H+tmp+Main.P-Source.Conc.+P-Source.Feed.conc.+Nitrogen.Conc. +OD+PL+emission_CO2+Thr+His+lactate+PO4.apporte+PO4.utilis e; 0.754, 0.892, tmp+Main.P-Source.Conc.+Nitrogen.Conc.+tot al.Vol+yield+PL+emission_CO2+Thr+lactate+PO4.ferm.+PO4.appo rte; 0.745, 0.892, tmp+Main.P-Source.Conc.+Nitrogen.Conc.+t otal.Vol+yield+PL+emission_CO2+Thr+His+lactate+SO4.ferm.+PO 4.ferm.+PO4.apporte; 0.745, 0.892, tmp+Main.P-Source.Conc.+ Main.Thr.Conc.+Nitrogen.Conc.+total.Vol+yield+PL+emission_C O2+rab+His+lactate+PO4.apporte+PO4.utilise; 0.745, 0.892, p H+tmp+Main.P-Source.Conc.+P-Source.Feed.conc.+Nitrogen.Conc. +OD+PL+emission_CO2+Thr+His+lactate+PO4.ferm.+PO4.apporte; 0.754, 0.892, tmp+Main.P-Source.Conc.+Nitrogen.Conc.+OD+PL+ emission_O2+emission_CO2+Thr+His+lactate+PO4.apporte; 0.754, 0.892, tmp+Main.P-Source.Conc.+Main.Thr.Conc.+Nitrogen.Con c.+total.Vol+yield+emission_O2+emission_CO2+lactate+PO4.app orte+PO4.utilise; 0.749, 0.892, tmp+Main.P-Source.Conc.+Nit rogen.Conc.+total.Vol+yield+PL+emission_CO2+rab+Thr+lactate +PO4.apporte+PO4.utilise; 0.754, 0.892, pH+tmp+P-Source.Fee d.conc.+PL+emission_CO2+rab+Thr+His+lactate+PO4.ferm.+PO4.a pporte; 0.767, 0.892, tmp+Main.P-Source.Conc.+Nitrogen.Conc. +OD+PL+emission_CO2+lactate+PO4.apporte; 0.749, 0.892, tmp+ Main.P-Source.Conc.+P-Source.Feed.conc.+Nitrogen.Conc.+OD+P L+emission_CO2+rab+His+lactate+PO4.apporte+PO4.utilise; 0.7 45, 0.892, tmp+Main.P-Source.Conc.+Nitrogen.Conc.+OD+total. Vol+PL+emission_O2+emission_CO2+His+lactate+SO4.ferm.+PO4.f erm.+PO4.apporte; 0.758, 0.892, tmp+Main.P-Source.Conc.+P-S ource.Feed.conc.+PL+emission_O2+emission_CO2+Thr+His+lactat e+PO4.ferm.; 0.749, 0.892, tmp+Main.P-Source.Conc.+P-Source. Feed.conc.+Nitrogen.Conc.+OD+PL+emission_O2+emission_CO2+Hi s+lactate+PO4.apporte+PO4.utilise; 0.763, 0.892, Main.P-Sou rce.Conc.+P-Source.Feed.conc.+Nitrogen.Conc.+OD+PL+emission _CO2+His+lactate+Po4.apporte; 0.758, 0.892, pH+tmp+Main.P-S ource.Conc.+Main.Thr.Conc.+Nitrogen.Conc.+total.Vol+emissio n_CO2+rab+lactate+PO4.apporte; 0.758, 0.892, pH+tmp+Main.P-Source.Conc.+Main.Thr.Conc.+Nitrogen.Conc.+emission_O2+emis sion_CO2+lactate+PO4.ferm.+PO4.apporte; 0.745, 0.892, tmp+M ain.P-Source.Conc.+Nitrogen.Conc.+total.Vol+yield+PL+emissi on_CO2+Thr+His+lactate+SO4.ferm.+PO4.apporte+PO4.utilise; 0. 754, 0.892, tmp+Main.P-Source.Conc.+Main.Thr.Conc.+Nitrogen. Conc.+OD+emission_CO2+rab+His+lactate+PO4.apporte+PO4.utili se; 0.754, 0.892, tmp+Main.P-Source.Conc.+P-Source.Feed.con c.+Main.Thr.Conc.+Nitrogen.Conc.+emission_CO2+rab+His+lacta te+PO4.apporte+PO4.utilise; 0.744, 0.892, pH+tmp+Main.P-Sou rce.Conc.+Main.Thr.Conc.+Nitrogen.Conc.+total.Vol+PL+emissi on_CO2+Thr+His+lactate+PO4.ferm.+PO4.apporte; 0.754, 0.892, tmp+Main.P-Source.Conc.+Nitrogen.Conc.+total.Vol+emission_ CO2+rab+His+lactate+SO4.ferm.+PO4.apporte+PO4.utilise; 0.76 3, 0.892, pH+Main.P-Source.Conc.+Nitrogen.Conc.+total.Vol+e mission_CO2+His+lactate+PO4.ferm.+PO4.apporte; 0.763, 0.892, pH+Main.P-Source.Conc.+Nitrogen.Conc.+total.Vol+PL+emissio n_CO2+lactate+PO4.apporte+PO4.utilise; 0.749, 0.892, tmp+Ma in.P-Source.Conc.+Nitrogen.Conc.+OD+total.Vol+PL+emission_C O2+His+lactate+SO4.ferm.+PO4.ferm.+PO4.apporte; 0.771, 0.89 2, Main.P-Source.Conc.+Nitrogen.Conc.+total.Vol+emission_O2 +emission_CO2+lactate+PO4.apporte; 0.754, 0.892, Main.P-Sou rce.Conc.+Nitrogen.Conc.+total.Vol+PL+emission_CO2+rab+Thr+ His+lactate+PO4.apporte+PO4.utilise; 0.744, 0.892, tmp+Main. P-Source.Conc.+Main.Thr.Conc.+Nitrogen.Conc.+Lys_conc.+tota l.Vol+PL+emission_O2+emission_CO2+His+lactate+PO4.apporte+P O4.utilise; 0.767, 0.892, tmp+P-Source.Feed.conc.+PL+emissi on_CO2+His+lactate+PO4.ferm.+PO4.apporte; 0.763, 0.892, tmp +Main.P-Source.Conc.+Nitrogen.Conc.+PL+emission_O2+emission _CO2+lactate+PO4.ferm.+PO4.apporte; 0.763, 0.892, tmp+Main. P-Source.Conc.+P-Source.Feed.conc.+Nitrogen.Conc.+total.Vol +emission_CO2+His+lactate+PO4.apporte; 0.758, 0.892, tmp+Ma in.P-Source.Conc.+P-Source.Feed.conc.+Nitrogen.Conc.+emissi on_CO2+rab+His+lactate+PO4.ferm.+PO4.apporte; 0.744, 0.892, pH+tmp+Main.P-Source.Conc.+Nitrogen.Conc.+Lys_conc.+total. Vol+PL+emission_CO2+Thr+His+lactate+PO4.ferm.+PO4.apporte; 0.767, 0.892, tmp+Main.P-Source.Conc.+Nitrogen.Conc.+PL+emi ssion_CO2+His+lactate+PO4.apporte; 0.754, 0.892, tmp+Main.P -Source.Conc.+Nitrogen.Conc.+total.Vol+yield+PL+emission_CO 2+Thr+lactate+PO4.apporte+PO4.utilise; 0.771, 0.892, Main.P -Source.Conc.+Main.Thr.Conc.+Nitrogen.Conc.+total.Vol+emiss ion_CO2+lactate+PO4.apporte; 0.754, 0.892, tmp+Main.P-Sourc e.Conc.+Main.Thr.Conc.+Nitrogen.Conc.+total.Vol+yield+emiss ion_CO2+rab+lactate+PO4.apporte+PO4.utilise; 0.754, 0.892, tmp+Main.P-Source.Conc.+Nitrogen.Conc.+total.Vol+yield+PL+e mission_CO2+rab+lactate+PO4.ferm.+PO4.apporte; 0.754, 0.892, tmp+Main.P-Source.Conc.+Nitrogen.Conc.+Lys_conc.+total.Vol +emission_CO2+rab+His+lactate+PO4.ferm.+PO4.apporte; 0.754, 0.892, pH+tmp+Main.P-Source.Conc.+Nitrogen.Conc.+OD+total. Vol+emission_CO2+rab+lactate+PO4.ferm.+PO4.apporte; 0.744, 0.892, tmp+Main.P-Source.Conc.+P-Source.Feed.conc.+Nitrogen. Conc.+total.Vol+PL+emission_CO2+rab+His+lactate+SO4.ferm.+P O4.apporte+PO4.utilise; 0.754, 0.892, Main.P-Source.Conc.+N itrogen.Conc.+total.Vol+yield+PL+emission_CO2+rab+His+lacta te+PO4.ferm.+PO4.apporte; 0.758, 0.892, tmp+Main.P-Source.C onc.+P-Source.Feed.conc.+Nitrogen.Conc.+emission_O2+emissio n_CO2+Thr+lactate+PO4.apporte+PO4.utilise; 0.754, 0.892, tm p+Main.P-Source.Conc.+Nitrogen.Conc.+total.Vol+yield+PL+emi ssion_CO2+rab+lactate+PO4.apporte+PO4.utilise

### [13. Linear Model that Predicts Lysine Production Amount in Interval 3]

0.474, 0.728, tmp+P-Source.Feed.conc.+Main.Thr.Conc.+Nitrog en.Conc.+Lys_conc.+SO4.ferm.+PO4.apporte; 0.482, 0.728, tmp +P-Source.Feed.conc.+Main.Thr.Conc.+Lys_conc.+SO4.ferm.+PO4. apporte; 0.459, 0.725, pH+tmp+P-Source.Feed.conc.+Main.Thr. Conc.+Nitrogen.Conc.+Lys_conc.+SO4.ferm.+PO4.apporte; 0.467, 0.724, pH+tmp+P-Source.Feed.conc.+Main.Thr.Conc.+Lys_conc. +SO4.ferm.+PO4.apporte; 0.467, 0.724, tmp+P-Source.Feed.con c.+Main.Thr.Conc.+Lys_conc.+lactate+SO4.ferm.+PO4.apporte; 0.456, 0.723, tmp+P-Source.Feed.conc.+Main.Thr.Conc.+Nitrog en.Conc.+Lys_conc.+lactate+SO4.ferm.+PO4.apporte; 0.464, 0. 722, tmp+P-Source.Feed.conc.+Main.Thr.Conc.+Lys_conc.+yield +SO4.ferm.+PO4.apporte; 0.454, 0.722, tmp+Main.P-Source.Con c.+P-Source.Feed.conc.+Main.Thr.Conc.+Nitrogen.Conc.+Lys_co nc.+SO4.ferm.+PO4.apporte; 0.454, 0.722, tmp+P-Source.Feed. conc.+Main.Thr.Conc.+Nitrogen.Conc.+Lys conc.+yield+SO4.fer m.+PO4.apporte; 0.451, 0.720, tmp+P-Source.Feed.conc.+Main. Thr.Conc.+Nitrogen.Conc.+Lys conc.+His+SO4.ferm.+PO4.apport e; 0.460, 0.720, tmp+P-Source.Feed.conc.+Main.Thr.Conc.+Lys _conc.+PL+SO4.ferm.+PO4.apporte; 0.450, 0.720, pH+tmp+P-Sou rce.Feed.conc.+Main.Thr.Conc.+Lys_conc.+lactate+SO4.ferm.+P O4.apporte; 0.450, 0.720, tmp+P-Source.Feed.conc.+Main.Thr. Conc.+Nitrogen.Conc.+Lys_conc.+PL+SO4.ferm.+PO4.apporte; 0. 459, 0.720, tmp+P-Source.Feed.conc.+Main.Thr.Conc.+OD+Lys_c onc.+SO4.ferm.+PO4.apporte; 0.440, 0.719, pH+tmp+Main.P-Sou rce.Conc.+P-Source.Feed.conc.+Main.Thr.Conc.+Nitrogen.Conc. +Lys_conc.+SO4.ferm.+PO4.apporte; 0.439, 0.719, pH+tmp+P-So urce.Feed.conc.+Main.Thr.Conc.+Nitrogen.Conc.+Lys_conc.+lac tate+SO4.ferm.+PO4.apporte; 0.449, 0.719, tmp+P-Source.Feed. conc.+Main.Thr.Conc.+Nitrogen.Conc.+OD+Lys conc.+SO4.ferm.+ PO4.apporte; 0.458, 0.718, tmp+Main.P-Source.Conc.+P-Source. Feed.conc.+Main.Thr.Conc.+Lys_conc.+SO4.ferm.+PO4.apporte; 0.447, 0.718, tmp+P-Source.Feed.conc.+Main.Thr.Conc.+Lys_co nc.+yield+lactate+SO4.ferm.+PO4.apporte; 0.457, 0.718, tmp+ P-Source.Feed.conc.+Main.Thr.Conc.+Lys conc.+His+SO4.ferm.+ PO4.apporte; 0.447, 0.718, pH+tmp+P-Source.Feed.conc.+Main. Thr.Conc.+Lys_conc.+yield+SO4.ferm.+PO4.apporte; 0.437, 0.7 17, pH+tmp+P-Source.Feed.conc.+Main.Thr.Conc.+Nitrogen.Conc. +Lys_conc.+yield+SO4.ferm.+PO4.apporte; 0.436, 0.717, tmp+P -Source.Feed.conc.+Main.Thr.Conc.+Nitrogen.Conc.+Lys_conc.+ yield+lactate+SO4.ferm.+PO4.apporte; 0.445, 0.717, pH+tmp+P -Source.Feed.conc.+Main.Thr.Conc.+OD+Lys conc.+SO4.ferm.+PO 4.apporte; 0.445, 0.717, tmp+P-Source.Feed.conc.+Main.Thr.C onc.+Lys_conc.+PL+lactate+SO4.ferm.+PO4.apporte; 0.435, 0.7 16, tmp+Main.P-Source.Conc.+P-Source.Feed.conc.+Main.Thr.Co nc.+Nitrogen.Conc.+Lys conc.+lactate+SO4.ferm.+PO4.apporte; 0.434, 0.716, pH+tmp+P-Source.Feed.conc.+Main.Thr.Conc.+Ni trogen.Conc.+Lys_conc.+His+SO4.ferm.+PO4.apporte; 0.443, 0. 716, tmp+P-Source.Feed.conc.+Main.Thr.Conc.+OD+Lys_conc.+yi eld+SO4.ferm.+PO4.apporte; 0.433, 0.715, pH+tmp+P-Source.Fe ed.conc.+Main.Thr.Conc.+Nitrogen.Conc.+OD+Lys_conc.+SO4.fer m.+PO4.apporte; 0.443, 0.715, tmp+P-Source.Feed.conc.+Main. Thr.Conc.+OD+Lys_conc.+lactate+SO4.ferm.+PO4.apporte; 0.443, 0.715, pH+tmp+P-Source.Feed.conc.+Main.Thr.Conc.+Lys_conc. +PL+SO4.ferm.+PO4.apporte; 0.433, 0.715, tmp+Main.P-Source. Conc.+P-Source.Feed.conc.+Main.Thr.Conc.+Nitrogen.Conc.+Lys conc.+yield+SO4.ferm.+PO4.apporte; 0.452, 0.715, tmp+P-Sou rce.Feed.conc.+Main.Thr.Conc.+Lys_conc.+SO4.ferm.+PO4.appor te+PO4.utilise; 0.432, 0.715, pH+tmp+P-Source.Feed.conc.+Ma in.Thr.Conc.+Nitrogen.Conc.+Lys_conc.+PL+SO4.ferm.+PO4.appo rte; 0.442, 0.714, pH+tmp+Main.P-Source.Conc.+P-Source.Feed. conc.+Main.Thr.Conc.+Lys_conc.+SO4.ferm.+PO4.apporte; 0.451, 0.714, tmp+P-Source.Feed.conc.+Main.Thr.Conc.+Lys_conc.+SO 4.ferm.+PO4.ferm.+PO4.apporte; 0.432, 0.714, tmp+Main.P-Sou rce.Conc.+P-Source.Feed.conc.+Main.Thr.Conc.+Nitrogen.Conc. +Lys_conc.+His+SO4.ferm.+PO4.apporte; 0.432, 0.714, tmp+P-S ource.Feed.conc.+Main.Thr.Conc.+Nitrogen.Conc.+Lys_conc.+PL +lactate+SO4.ferm.+PO4.apporte; 0.441, 0.714, tmp+P-Source. Feed.conc.+Main.Thr.Conc.+Nitrogen.Conc.+Lys_conc.+SO4.ferm. +PO4.apporte+PO4.utilise; 0.441, 0.714, tmp+Main.P-Source.C onc.+P-Source.Feed.conc.+Main.Thr.Conc.+Lys_conc.+lactate+S O4.ferm.+PO4.apporte; 0.451, 0.714, tmp+P-Source.Feed.conc. +Main.Thr.Conc.+Lys_conc.+SO4.ferm.+PO4.ferm.+PO 0.441, 0.714, tmp+P-Source.Feed.conc.+Main.Thr.Conc.+Nitrog en.Conc.+Lys_conc.+SO4.ferm.+PO4.ferm.+PO4.apporte; 0.440, 0.714, tmp+P-Source.Feed.conc.+Main.Thr.Conc.+OD+Lys_conc.+ His+SO4.ferm.+PO4.apporte; 0.440, 0.714, tmp+P-Source.Feed. conc.+Main.Thr.Conc.+Lys conc.+yield+PL+SO4.ferm.+PO4.appor te; 0.430, 0.713, tmp+P-Source.Feed.conc.+Main.Thr.Conc.+Ni trogen.Conc.+OD+Lys conc.+lactate+SO4.ferm.+PO4.apporte; 0. 440, 0.713, pH+tmp+P-Source.Feed.conc.+Main.Thr.Conc.+Lys_c onc.+His+SO4.ferm.+PO4.apporte; 0.440, 0.713, tmp+P-Source. Feed.conc.+Main.Thr.Conc.+Nitrogen.Conc.+Lys_conc.+SO4.ferm. +PO4.ferm.+PO4.utilise; 0.419, 0.713, pH+tmp+Main.P-Source. Conc.+P-Source.Feed.conc.+Main.Thr.Conc.+Nitrogen.Conc.+Lys conc.+lactate+SO4.ferm.+PO4.apporte; 0.429, 0.713, pH+tmp+ P-Source.Feed.conc.+Main.Thr.Conc.+Lys_conc.+yield+lactate+ SO4.ferm.+PO4.apporte; 0.429, 0.713, tmp+Main.P-Source.Conc. +P-Source.Feed.conc.+Main.Thr.Conc.+Nitrogen.Conc.+Lys_conc. +PL+SO4.ferm.+PO4.apporte; 0.429, 0.713, tmp+P-Source.Feed. conc.+Main.Thr.Conc.+Nitrogen.Conc.+Lys conc.+yield+PL+SO4. ferm.+PO4.apporte; 0.428, 0.712, tmp+P-Source.Feed.conc.+Ma in.Thr.Conc.+Nitrogen.Conc.+OD+Lys_conc.+His+SO4.ferm.+PO4. apporte; 0.438, 0.712, tmp+Main.P-Source.Conc.+P-Source.Fee d.conc.+Main.Thr.Conc.+Lys_conc.+yield+SO4.ferm.+PO4.apport e; 0.428, 0.712, tmp+P-Source.Feed.conc.+Main.Thr.Conc.+Nit rogen.Conc.+OD+Lys_conc.+yield+SO4.ferm.+PO4.apporte; 0.457, 0.712, tmp+P-Source.Feed.conc.+Main.Thr.Conc.+Nitrogen.Con c.+Lys_conc.+PO4.apporte; 0.417, 0.712, pH+tmp+P-Source.Fee d.conc.+Main.Thr.Conc.+Nitrogen.Conc.+Lys_conc.+yield+lacta te+SO4.ferm.+PO4.apporte; 0.427, 0.712, pH+tmp+P-Source.Fee d.conc.+Main.Thr.Conc.+OD+Lys_conc.+yield+SO4.ferm.+PO4.app orte; 0.437, 0.712, tmp+P-Source.Feed.conc.+Main.Thr.Conc.+ OD+Lys_conc.+PL+SO4.ferm.+PO4.apporte; 0.416, 0.712, pH+tmp +Main.P-Source.Conc.+P-Source.Feed.conc.+Main.Thr.Conc.+Nit rogen.Conc.+Lys_conc.+yield+SO4.ferm.+PO4.apporte; 0.427, 0. 711, tmp+P-Source.Feed.conc.+Main.Thr.Conc.+Nitrogen.Conc.+ Lys_conc.+PL+His+SO4.ferm.+PO4.apporte; 0.427, 0.711, pH+tm p+P-Source.Feed.conc.+Main.Thr.Conc.+OD+Lys_conc.+lactate+S O4.ferm.+PO4.apporte; 0.446, 0.711, pH+tmp+P-Source.Feed.co nc.+Main.Thr.Conc.+Nitrogen.Conc.+Lys_conc.+PO4.apporte; 0. 426, 0.711, tmp+Main.P-Source.Conc.+P-Source.Feed.conc.+Mai n.Thr.Conc.+Nitrogen.Conc.+OD+Lys conc.+SO4.ferm.+PO4.appor te; 0.416, 0.711, pH+tmp+Main.P-Source.Conc.+P-Source.Feed. conc.+Main.Thr.Conc.+Nitrogen.Conc.+Lys_conc.+His+SO4.ferm. +PO4.apporte; 0.436, 0.711, tmp+P-Source.Feed.conc.+Main.Th r.Conc.+Lys_conc.+lactate+SO4.ferm.+PO4.apporte+PO4.utilis e; 0.436, 0.711, pH+tmp+P-Source.Feed.conc.+Main.Thr.Conc.+ Lys_conc.+SO4.ferm.+PO4.apporte+PO4.utilise; 0.426, 0.711, pH+tmp+P-Source.Feed.conc.+Main.Thr.Conc.+Lys_conc.+PL+lact ate+SO4.ferm.+PO4.apporte; 0.436, 0.711, tmp+P-Source.Feed. conc.+Main.Thr.Conc.+Lys_conc.+lactate+SO4.ferm.+PO4.ferm.+ PO4.apporte; 0.436, 0.711, pH+tmp+P-Source.Feed.conc.+Main. Thr.Conc.+Lys_conc.+SO4.ferm.+PO4.ferm.+PO4.apporte; 0.425, 0.711, pH+tmp+P-Source.Feed.conc.+Main.Thr.Conc.+OD+Lys_co nc.+His+SO4.ferm.+PO4.apporte; 0.425, 0.710, pH+tmp+P-Sourc e.Feed.conc.+Main.Thr.Conc.+Nitrogen.Conc.+Lys_conc.+SO4.fe rm.+PO4.apporte+PO4.utilise; 0.435, 0.710, tmp+P-Source.Fee d.conc.+Main.Thr.Conc.+Lys_conc.+lactate+SO4.ferm.+PO4.ferm. +PO4.utilise; 0.435, 0.710, pH+tmp+P-Source.Feed.conc.+Main. Thr.Conc.+Lys_conc.+SO4.ferm.+PO4.ferm.+PO4.utilise; 0.435, 0.710, tmp+P-Source.Feed.conc.+Main.Thr.Conc.+Lys_conc.+PL +His+SO4.ferm.+PO4.apporte; 0.425, 0.710, pH+tmp+P-Source.F eed.conc.+Main.Thr.Conc.+Nitrogen.Conc.+Lys_conc.+SO4.ferm. +PO4.ferm.+PO4.apporte; 0.425, 0.710, tmp+P-Source.Feed.con c.+Main.Thr.Conc.+OD+Lys_conc.+yield+lactate+SO4.ferm.+PO4. apporte; 0.435, 0.710, tmp+Main.P-Source.Conc.+P-Source.Fee d.conc.+Main.Thr.Conc.+Lys_conc.+PL+SO4.ferm.+PO4.apporte; 0.425, 0.710, tmp+P-Source.Feed.conc.+Main.Thr.Conc.+Nitrog en.Conc.+Lys_conc.+SO4.ferm.+PO4.ferm.+PO4.apporte+PO4.util ise; 0.414, 0.710, tmp+Main.P-Source.Conc.+P-Source.Feed.co nc.+Main.Thr.Conc.+Nitrogen.Conc.+Lys_conc.+yield+lactate+S O4.ferm.+PO4.apporte; 0.424, 0.710, tmp+P-Source.Feed.conc. +Main.Thr.Conc.+Lys conc.+yield+PL+lactate+SO4.ferm.+PO4.ap porte; 0.424, 0.710, pH+tmp+P-Source.Feed.conc.+Main.Thr.Co nc.+Nitrogen.Conc.+Lys conc.+SO4.ferm.+PO4.ferm.+PO4.utilis e; 0.423, 0.709, pH+tmp+Main.P-Source.Conc.+P-Source.Feed.c onc.+Main.Thr.Conc.+Lys_conc.+lactate+SO4.ferm.+PO4.apport e; 0.433, 0.709, tmp+P-Source.Feed.conc.+Main.Thr.Conc.+Lys conc.+SO4.ferm.+PO4.ferm.+PO4.apporte+PO4.utilise; 0.423, 0.709, tmp+P-Source.Feed.conc.+Main.Thr.Conc.+Nitrogen.Conc. +OD+Lys_conc.+PL+SO4.ferm.+PO4.apporte; 0.412, 0.709, pH+tm p+P-Source.Feed.conc.+Main.Thr.Conc.+Nitrogen.Conc.+OD+Lys_ conc.+lactate+SO4.ferm.+PO4.apporte; 0.433, 0.709, tmp+Main. P-Source.Conc.+P-Source.Feed.conc.+Main.Thr.Conc.+OD+Lys_co nc.+SO4.ferm.+PO4.apporte; 0.412, 0.709, pH+tmp+P-Source.Fe ed.conc.+Main.Thr.Conc.+Nitrogen.Conc.+OD+Lys conc.+His+SO4. ferm.+PO4.apporte; 0.423, 0.709, tmp+P-Source.Feed.conc.+Ma in.Thr.Conc.+Nitrogen.Conc.+Lys_conc.+lactate+SO4.ferm.+PO4. apporte+PO4.utilise; 0.422, 0.709, tmp+Main.P-Source.Conc.+ P-Source.Feed.conc.+Main.Thr.Conc.+Nitrogen.Conc.+Lys_conc. +SO4.ferm.+PO4.apporte+PO4.utilise; 0.422, 0.709, tmp+Main. P-Source.Conc.+P-Source.Feed.conc.+Main.Thr.Conc.+Nitrogen. Conc.+Lys_conc.+SO4.ferm.+PO4.ferm.+PO4.apporte; 0.412, 0.7 09, pH+tmp+P-Source.Feed.conc.+Main.Thr.Conc.+Nitrogen.Conc. +Lys_conc.+PL+lactate+SO4.ferm.+PO4.apporte; 0.422, 0.709, tmp+P-Source.Feed.conc.+Main.Thr.Conc.+Nitrogen.Conc.+Lys_c onc.+lactate+SO4.ferm.+PO4.ferm.+PO4.apporte; 0.411, 0.708, pH+tmp+Main.P-Source.Conc.+P-Source.Feed.conc.+Main.Thr.Co nc.+Nitrogen.Conc.+Lys_conc.+PL+SO4.ferm.+PO4.apporte; 0.43 2, 0.708, tmp+Main.P-Source.Conc.+P-Source.Feed.conc.+Main. Thr.Conc.+Lys_conc.+His+SO4.ferm.+PO4.apporte; 0.411, 0.708, pH+tmp+Main.P-Source.Conc.+P-Source.Feed.conc.+Main.Thr.Co nc.+Nitrogen.Conc.+OD+Lys_conc.+SO4.ferm.+PO4.apporte; 0.43 2, 0.708, tmp+P-Source.Feed.conc.+Main.Thr.Conc.+Lys_conc.+ yield+SO4.ferm.+PO4.apporte+PO4.utilise; 0.432, 0.708, tmp+ P-Source.Feed.conc.+Main.Thr.Conc.+Lys conc.+yield+SO4.ferm. +PO4.ferm.+PO4.apporte; 0.431, 0.708, tmp+P-Source.Feed.con c.+Main.Thr.Conc.+Lys_conc.+yield+SO4.ferm.+PO4.ferm.+PO4.u tilise; 0.421, 0.708, tmp+P-Source.Feed.conc.+Main.Thr.Conc. +Nitrogen.Conc.+Lys conc.+lactate+SO4.ferm.+PO4.ferm.+PO4.u tilise; 0.411, 0.708, pH+tmp+P-Source.Feed.conc.+Main.Thr.C onc.+Nitrogen.Conc.+OD+Lys_conc.+yield+SO4.ferm.+PO4.apport e; 0.431, 0.708, pH+tmp+Main.P-Source.Conc.+P-Source.Feed.c onc.+Main.Thr.Conc.+Nitrogen.Conc.+Lys_conc.+SO4.ferm.; 0.4 41, 0.708, tmp+Main.P-Source.Conc.+P-Source.Feed.conc.+Main. Thr.Conc.+Nitrogen.Conc.+Lys_conc.+SO4.ferm.; 0.421, 0.708, pH+tmp+P-Source.Feed.conc.+Main.Thr.Conc.+Lys_conc.+yield+ PL+SO4.ferm.+PO4.apporte; 0.410, 0.708, tmp+P-Source.Feed.c onc.+Main.Thr.Conc.+Nitrogen.Conc.+Lys_conc.+yield+PL+lacta te+SO4.ferm.+PO4.apporte; 0.421, 0.708, tmp+Main.P-Source.C onc.+P-Source.Feed.conc.+Main.Thr.Conc.+Lys_conc.+yield+lac tate+SO4.ferm.+PO4.apporte; 0.421, 0.708, tmp+Main.P-Source. Conc.+P-Source.Feed.conc.+Main.Thr.Conc.+Nitrogen.Conc.+Lys conc.+SO4.ferm.+PO4.ferm.+PO4.utilise; 0.440, 0.707, tmp+P -Source.Feed.conc.+Main.Thr.Conc.+Nitrogen.Conc.+Lys_conc.+ yield+PO4.apporte; 0.420, 0.707, tmp+P-Source.Feed.conc.+Ma in.Thr.Conc.+OD+Lys conc.+PL+lactate+SO4.ferm.+PO4.apporte; 0.420, 0.707, pH+tmp+Main.P-Source.Conc.+P-Source.Feed.con c.+Main.Thr.Conc.+Lys_conc.+yield+SO4.ferm.+PO4.apporte; 0. 410, 0.707, tmp+Main.P-Source.Conc.+P-Source.Feed.conc.+Mai n.Thr.Conc.+Nitrogen.Conc.+Lys_conc.+SO4.ferm.+PO4.ferm.+PO 4.apporte+PO4.utilise; 0.420, 0.707, pH+tmp+P-Source.Feed.c onc.+Main.Thr.Conc.+OD+Lys conc.+PL+SO4.ferm.+PO4.apporte; 0.420, 0.707, tmp+P-Source.Feed.conc.+Main.Thr.Conc.+Nitrog en.Conc.+Lys_conc.+His+lactate+SO4.ferm.+PO4.apporte; 0.409, 0.707, tmp+Main.P-Source.Conc.+P-Source.Feed.conc.+Main.Th r.Conc.+Nitrogen.Conc.+Lys_conc.+PL+lactate+SO4.ferm.+PO4.a pporte; 0.409, 0.707, pH+tmp+P-Source.Feed.conc.+Main.Thr.C onc.+Nitrogen.Conc.+Lys_conc.+yield+PL+SO4.ferm.+PO4.apport e; 0.419, 0.707, tmp+P-Source.Feed.conc.+Main.Thr.Conc.+Nit rogen.Conc.+Lys conc.+yield+SO4.ferm.+PO4.apporte+PO4.utili se; 0.429, 0.707, tmp+P-Source.Feed.conc.+Main.Thr.Conc.+OD +Lys_conc.+His+SO4.ferm.+PO4.utilise; 0.419, 0.707, tmp+P-S ource.Feed.conc.+Main.Thr.Conc.+OD+Lys conc.+yield+PL+SO4.f erm.+PO4.apporte; 0.429, 0.707, tmp+P-Source.Feed.conc.+Mai n.Thr.Conc.+Lys_conc.+PL+SO4.ferm.+PO4.apporte+PO4.utilise; 0.409, 0.707, tmp+P-Source.Feed.conc.+Main.Thr.Conc.+Nitro gen.Conc.+OD+Lys_conc.+yield+lactate+SO4.ferm.+PO4.apporte; 0.419, 0.707, tmp+P-Source.Feed.conc.+Main.Thr.Conc.+Nitro gen.Conc.+Lys_conc.+yield+SO4.ferm.+PO4.ferm.+PO4.apporte; 0.419, 0.707, tmp+P-Source.Feed.conc.+Main.Thr.Conc.+OD+Lys conc.+PL+His+SO4.ferm.+PO4.apporte; 0.429, 0.707, tmp+P-So urce.Feed.conc.+Main.Thr.Conc.+Lys_conc.+PL+SO4.ferm.+PO4.f erm.+PO4.apporte; 0.419, 0.707, pH+tmp+P-Source.Feed.conc.+ Main.Thr.Conc.+Lys_conc.+lactate+SO4.ferm.+PO4.apporte+PO4. utilise; 0.429, 0.706, tmp+Main.P-Source.Conc.+P-Source.Fee d.conc.+Main.Thr.Conc.+Lys_conc.+SO4.ferm.+PO4.apporte+PO4. utilise; 0.418, 0.706, pH+tmp+P-Source.Feed.conc.+Main.Thr. Conc.+Lys_conc.+lactate+SO4.ferm.+PO4.ferm.+PO4.apporte; 0. 429, 0.706, tmp+Main.P-Source.Conc.+P-Source.Feed.conc.+Mai n.Thr.Conc.+Lys_conc.+SO4.ferm.+PO4.ferm.+PO4.apporte; 0.42 9, 0.706, tmp+P-Source.Feed.conc.+Main.Thr.Conc.+Lys_conc.+ PL+SO4.ferm.+PO4.ferm.+PO4.utilise; 0.418, 0.706, tmp+P-Sou rce.Feed.conc.+Main.Thr.Conc.+Nitrogen.Conc.+Lys_conc.+yiel d+SO4.ferm.+PO4.ferm.+PO4.utilise; 0.438, 0.706, tmp+P-Sour ce.Feed.conc.+Main.Thr.Conc.+Nitrogen.Conc.+Lys_conc.+lacta te+PO4.apporte; 0.418, 0.706, pH+tmp+P-Source.Feed.conc.+Ma in.Thr.Conc.+Lys_conc.+lactate+SO4.ferm.+PO4.ferm.+PO4.util ise; 0.407, 0.706, pH+tmp+Main.P-Source.Conc.+P-Source.Feed. conc.+Main.Thr.Conc.+Nitrogen.Conc.+Lys_conc.+SO4.ferm.+PO4. apporte+PO4.utilise; 0.407, 0.706, pH+tmp+Main.P-Source.Con c.+P-Source.Feed.conc.+Main.Thr.Cone.+Nitrogen.Cone.+Lys_co nc.+SO4.ferm.+PO4.ferm.+PO4.apporte; 0.418, 0.706, tmp+Main. P-Source.Conc.+P-Source.Feed.conc.+Main.Thr.Conc.+Lys_conc. +PL+lactate+SO4.ferm.+PO4.apporte; 0.396, 0.706, pH+tmp+Mai n.P-Source.Conc.+P-Source.Feed.conc.+Main.Thr.Conc.+Nitroge n.Conc.+Lys_conc.+yield+lactate+SO4.ferm.+PO4.apporte; 0.41 8, 0.706, pH+tmp+Main.P-Source.Conc.+P-Source.Feed.conc.+Ma in.Thr.Conc.+OD+Lys-conc.+SO4.ferm.+PO4.apporte; 0.428, 0.7 06, tmp+Main.P-Source.Conc.+P-Source.Feed.conc.+Main.Thr.Co nc.+Lys_conc.+SO4.ferm.+PO4.ferm.+PO4.utilise; 0.407, 0.706, pH+tmp+P-Source.Feed.conc.+Main.Thr.Conc.+OD+Lys_conc.+yie ld+lactate+SO4.ferm.+PO4.apporte; 0.407, 0.706, pH+tmp+P-So urce.Feed.conc.+Main.Thr.Conc.+Nitrogen.Conc.+Lys_conc.+SO4. ferm.+PO4.ferm.+PO4.apporte+PO4.utilise; 0.406, 0.705, tmp+ Main.P-Source.Conc.+P-Source.Feed.conc.+Main.Thr.Conc.+Nitr ogen.Conc.+Lys_conc.+PL+His+SO4.ferm.+PO4.apporte; 0.427, 0. 705, tmp+P-Source.Feed.conc.+Main.Thr.Conc.+OD+Lys_conc.+yi eld+SO4.ferm.+PO4.utilise; 0.456, 0.705, tmp+P-Source.Feed. conc.+Main.Thr.Conc.+Lys_conc.+PO4.apporte; 0.406, 0.705, t mp+Main.P-Source.Conc.+P-Source.Feed.conc.+Main.Thr.Conc.+N itrogen.Conc.+OD+Lys_conc.+lactate+SO4.ferm.+PO4.apporte; 0. 406, 0.705, pH+tmp+P-Source.Feed.conc.+Main.Thr.Conc.+Nitro gen.Conc.+Lys_conc.+PL+His+SO4.ferm.+PO4.apporte; 0.406, 0. 705, tmp+Main.P-Source.Conc.+P-Source.Feed.conc.+Main.Thr.C onc.+Nitrogen.Conc.+Lys conc.+yield+PL+SO4.ferm.+PO4.apport e; 0.416, 0.705, tmp+P-Source.Feed.conc.+Main.Thr.Conc.+Lys conc.+lactate+SO4.ferm.+PO4.ferm.+PO4.apporte+PO4.utilise; 0.416, 0.705, pH+tmp+P-Source.Feed.conc.+Main.Thr.Conc.+Ly s_conc.+SO4.ferm.+PO4.ferm.+PO4.apporte+PO4.utilise; 0.406, 0.705, pH+tmp+Main.P-Source.Conc.+P-Source.Feed.conc.+Main. Thr.Conc.+Nitrogen.Conc.+Lys_conc.+SO4.ferm.+PO4.ferm.+PO4. utilise; 0.416, 0.705, tmp+P-Source.Feed.conc.+Main.Thr.Con c.+Nitrogen.Conc.+Lys conc.+PL+SO4.ferm.+PO4.apporte+PO4.ut ilise; 0.416, 0.705, pH+tmp+Main.P-Source.Conc.+P-Source.Fe ed.conc.+Main.Thr.Conc.+Lys_conc.+PL+SO4.ferm.+PO4.apporte; 0.405, 0.705, pH+tmp+P-Source.Feed.conc.+Main.Thr.Conc.+Ni trogen.Conc.+Lys_conc.+lactate+SO4.ferm.+PO4.apporte+PO4.ut ilise; 0.416, 0.705, tmp+P-Source.Feed.conc.+Main.Thr.Conc. +Nitrogen.Conc.+Lys_conc.+His+SO4.ferm.+PO4.ferm.+PO4.appor te; 0.416, 0.705, tmp+P-Source.Feed.conc.+Main.Thr.Conc.+Ni trogen.Conc.+Lys_conc.+His+SO4.ferm.+PO4.ferm.+PO4.utilise; 0.416, 0.705, tmp+P-Source.Feed.conc.+Main.Thr.Conc.+Nitro gen.Conc.+Lys_conc.+PL+SO4.ferm.+PO4.ferm.+PO4.apporte; 0.4 26, 0.705, tmp+Main.P-Source.Conc.+P-Source.Feed.conc.+Main. Thr.Conc.+Nitrogen.Conc.+Lys conc.+yield+SO4.ferm.; 0.416, 0.705, tmp+P-Source.Feed.conc.+Main.Thr.Conc.+Nitrogen.Conc. +Lys_conc.+His+SO4.ferm.+PO4.apporte+PO4.utilise; 0.426, 0. 705, tmp+P-Source.Feed.conc.+Main.Thr.Conc.+Lys_conc.+His+S O4.ferm.+PO4.ferm.+PO4.utilise; 0.445, 0.705, pH+tmp+P-Sour ce.Feed.conc.+Main.Thr.Conc.+Lys_conc.+PO4.apporte; 0.416, 0.705, tmp+Main.P-Source.Conc.+P-Source.Feed.conc.+Main.Thr. Conc.+OD+Lys_conc.+yield+SO4.ferm.+PO4.apporte; 0.405, 0.70 5, pH+tmp+P-Source.Feed.conc.+Main.Thr.Conc.+Nitrogen.Conc. +Lys_conc.+lactate+SO4.ferm.+PO4.ferm.+PO4.apporte; 0.405, 0.705, pH+tmp+P-Source.Feed.conc.+Main.Thr.Conc.+Nitrogen.C onc.+OD+Lys_conc.+PL+SO4.ferm.+PO4.apporte; 0.426, 0.705, t mp+P-Source.Feed.conc.+Main.Thr.Conc.+Lys_conc.+His+lactate +SO4.ferm.+PO4.apporte; 0.426, 0.705, tmp+P-Source.Feed.con c.+Main.Thr.Conc.+OD+Lys conc.+SO4.ferm.+PO4.apporte+PO4.ut ilise; 0.415, 0.705, tmp+P-Source.Feed.conc.+Main.Thr.Conc. +Nitrogen.Conc.+Lys_conc.+yield+His+SO4.ferm.+PO4.apporte; 0.415, 0.704, tmp+P-Source.Feed.conc.+Main.Thr.Conc.+Lys_co nc.+yield+lactate+SO4.ferm.+PO4.apporte+PO4.utilise; 0.415, 0.704, tmp+P-Source.Feed.conc.+Main.Thr.Conc.+Lys_conc.+yi eld+lactate+SO4.ferm.+PO4.ferm.+PO4.apporte; 0.415, 0.704, tmp+P-Source.Feed.conc.+Main.Thr.Conc.+Lys_conc.+yield+lact ate+SO4.ferm.+PO4.ferm.+PO4.utilise; 0.393, 0.704, pH+tmp+M ain.P-Source.Conc.+P-Source.Feed.conc.+Main.Thr.Conc.+Nitro gen.Conc.+Lys conc.+SO4.ferm.+PO4.ferm.+PO4.apporte+PO4.uti lise; 0.425, 0.704, pH+tmp+P-Source.Feed.conc.+Main.Thr.Con c.+Nitrogen.Conc.+Lys_conc.+lactate+PO4.apporte; 0.425, 0.7 04, pH+tmp+P-Source.Feed.conc.+Main.Thr.Conc.+Nitrogen.Conc. +Lys_conc.+yield+PO4.apporte; 0.425, 0.704, tmp+P-Source.Fe ed.conc.+Main.Thr.Conc.+Lys_conc.+His+SO4.ferm.+PO4.ferm.+P O4.apporte; 0.425, 0.704, tmp+P-Source.Feed.conc.+Main.Thr. Conc.+Lys_conc.+His+SO4.ferm.+PO4.apporte+PO4.utilise; 0.42 5, 0.704, tmp+P-Source.Feed.conc.+Main.Thr.Conc.+OD+Lys_con c.+SO4.ferm.+PO4.ferm.+PO4.apporte; 0.445, 0.704, tmp+P-Sou rce.Feed.conc.+Main.Thr.Conc.+Lys_conc.+yield+PO4.apporte; 0.415, 0.704, tmp+P-Source.Feed.conc.+Main.Thr.Conc.+Nitrog en.Conc.+Lys_conc.+PL+SO4.ferm.+PO4.ferm.+PO4.utilise; 0.41 5, 0.704, pH+tmp+P-Source.Feed.conc.+Main.Thr.Conc.+Lys_con c.+PL+His+SO4.ferm.+PO4.apporte; 0.404, 0.704, pH+tmp+P-Sou rce.Feed.conc.+Main.Thr.Conc.+Nitrogen.Conc.+Lys_conc.+lact ate+SO4.ferm.+PO4.ferm.+PO4.utilise; 0.404, 0.704, tmp+P-So urce.Feed.conc.+Main.Thr.Conc.+Nitrogen.Conc.+Lys_conc.+lac tate+SO4.ferm.+PO4.ferm.+PO4.apporte+PO4.utilise; 0.414, 0. 704, tmp+Main.P-Source.Conc.+P-Source.Feed.conc.+Main.Thr.C onc.+OD+Lys-conc.+lactate+SO4.ferm.+PO4.apporte; 0.404, 0.7 04, tmp+Main.P-Source.Conc.+P-Source.Feed.conc.+Main.Thr.Co nc.+Nitrogen.Conc.+Lys_conc.+lactate+SO4.ferm.+PO4.ferm.+PO 4.apporte; 0.403, 0.704, tmp+Main.P-Source.Conc.+P-Source.F eed.conc.+Main.Thr.Conc.+Nitrogen.Conc.+Lys_conc.+lactate+S O4.ferm.+PO4.apporte+PO4.utilise; 0.403, 0.704, tmp+Main.P-Source.Conc.+P-Source.Feed.conc.+Main.Thr.Conc.+Nitrogen.Co nc.+OD+Lys_conc.+His+SO4.ferm.+PO4.apporte; 0.414, 0.704, p H+tmp+Main.P-Source.Conc.+P-Source.Feed.conc.+Main.Thr.Conc. +Lys_conc.+His+SO4.ferm.+PO4.apporte; 0.403, 0.704, tmp+Mai n.P-Source.Conc.+P-Source.Feed.conc.+Main.Thr.Conc.+Nitroge n.Conc.+OD+Lys_conc.+yield+SO4.ferm.+PO4.apporte; 0.403, 0. 704, tmp+P-Source.Feed.conc.+Main.Thr.Conc.+Nitrogen.Conc.+ OD+Lys-conc.+PL+lactate+SO4.ferm.+PO4.apporte; 0.414, 0.704, pH+tmp+P-Source.Feed.conc.+Main.Thr.Conc.+Lys_conc.+yield+ SO4.ferm.+PO4.apporte+PO4.utilise; 0.424, 0.703, tmp+P-Sour ce.Feed.conc.+Main.Thr.Conc.+OD+Lys conc.+SO4.ferm.+PO4.fer m.+PO4.utilise; 0.414, 0.703, pH+tmp+P-Source.Feed.conc.+Ma in.Thr.Conc.+Lys conc.+yield+SO4.ferm.+PO4.ferm.+PO4.apport e; 0.403, 0.703, pH+tmp+P-Source.Feed.conc.+Main.Thr.Conc.+ Lys_conc.+yield+PL+lactate+SO4.ferm.+PO4.apporte; 0.414, 0. 703, pH+tmp+P-Source.Feed.conc.+Main.Thr.Conc.+Lys_conc.+yi eld+SO4.ferm.+PO4.ferm.+PO4.utilise; 0.424, 0.703, tmp+Main. P-Source.Conc.+P-Source.Feed.conc.+Main.Thr.Conc.+Nitrogen. Conc.+Lys_conc.+SO4.ferm.+PO4.utilise; 0.413, 0.703, pH+tmp +P-Source.Feed.conc.+Main.Thr.Conc.+OD+Lys conc.+His+SO4.fe rm.+PO4.utilise; 0.413, 0.703, tmp+Main.P-Source.Conc.+P-So urce.Feed.conc.+Main.Thr.Conc.+Lys_conc.+yield+PL+SO4.ferm. +PO4.apporte; 0.413, 0.703, tmp+Main.P-Source.Conc.+P-Sourc e.Feed.conc.+Main.Thr.Conc.+OD+Lys_conc.+His+SO4.ferm.+PO4. apporte; 0.413, 0.703, tmp+P-Source.Feed.conc.+Main.Thr.Con c.+Lys_conc.+PL+lactate+SO4.ferm.+PO4.apporte+PO4.utilise; 0.403, 0.703, tmp+P-Source.Feed.conc.+Main.Thr.Conc.+Nitrog en.Conc.+OD+Lys_conc.+PL+His+SO4.ferm.+PO4.apporte; 0.413, 0.703, tmp+P-Source.Feed.conc.+Main.Thr.Conc.+Nitrogen.Conc. +OD+Lys_conc.+SO4.ferm.+PO4.apporte+PO4.utilise; 0.433, 0.7 03, tmp+Main.P-Source.Conc.+P-Source.Feed.conc.+Main.Thr.Co nc.+Nitrogen.Conc.+Lys_conc.+PO4.apporte; 0.413, 0.703, tmp +P-Source.Feed.conc.+Main.Thr.Conc.+Lys_conc.+PL+lactate+SO 4.ferm.+PO4.ferm.+PO4.apporte; 0.413, 0.703, tmp+P-Source.F eed.conc.+Main.Thr.Conc.+Lys_conc.+PL+lactate+SO4.ferm.+PO4. ferm.+PO4.utilise; 0.413, 0.703, tmp+P-Source.Feed.conc.+Ma in.Thr.Conc.+Nitrogen.Conc.+OD+Lys_conc.+SO4.ferm.+PO4.ferm. +PO4.apporte; 0.433, 0.703, tmp+P-Source.Feed.conc.+Main.Th r.Conc.+Nitrogen.Conc.+Lys_conc.+His+PO4.apporte; 0.402, 0. 703, tmp+Main.P-Source.Conc.+P-Source.Feed.conc.+Main.Thr.C onc.+Nitrogen.Conc.+Lys conc.+lactate+SO4.ferm.+PO4.ferm.+P O4.utilise; 0.412, 0.703, tmp+Main.P-Source.Conc.+P-Source. Feed.conc.+Main.Thr.Conc.+Lys_conc.+lactate+SO4.ferm.+PO4.a pporte+PO4.utilise; 0.412, 0.703, tmp+Main.P-Source.Conc.+P -Source.Feed.conc.+Main.Thr.Conc.+Lys conc.+lactate+SO4.fer m.+PO4.ferm.+PO4.apporte; 0.401, 0.702, pH+tmp+P-Source.Fee d.conc.+Main.Thr.Conc.+Nitrogen.Conc.+Lys_conc.+yield+SO4.f erm.+PO4.apporte+PO4.utilise; 0.401, 0.702, pH+tmp+P-Source. Feed.conc.+Main.Thr.Conc.+Nitrogen.Conc.+Lys_conc.+His+lact ate+SO4.ferm.+PO4.apporte; 0.412, 0.702, pH+tmp+Main.P-Sour ce.Conc.+P-Source.Feed.conc.+Main.Thr.Conc.+Lys_conc.+SO4.f erm.+PO4.apporte+PO4.utilise; 0.401, 0.702, pH+tmp+P-Source. Feed.conc.+Main.Thr.Conc.+Nitrogen.Conc.+Lys_conc.+yield+SO 4.ferm.+PO4.ferm.+PO4.apporte; 0.412, 0.702, pH+tmp+Main.P-Source.Conc.+P-Source.Feed.conc.+Main.Thr.Conc.+Nitrogen.Co nc.+Lys_conc.+yield+SO4.ferm.; 0.412, 0.702, pH+tmp+Main.P-Source.Conc.+P-Source.Feed.conc.+Main.Thr.Conc.+Lys_conc.+S O4.ferm.+PO4.ferm.+PO4.aporte; 0.412, 0.702, tmp+P-Source. Feed.conc.+Main.Thr.Conc.+Lys conc.+yield+SO4.ferm.+PO4.fer m.+PO4.apporte+PO4.utilise; 0.401, 0.702, pH+tmp+Main.P-Sou rce.Conc.+P-Source.Feed.conc.+Main.Thr.Conc.+Lys_conc.+yiel d+lactate+SO4.ferm.+PO4.apporte; 0.401, 0.702, pH+tmp+P-Sou rce.Feed.conc.+Main.Thr.Conc.+OD+Lys conc.+PL+lactate+SO4.f erm.+PO4.apporte; 0.432, 0.702, tmp+P-Source.Feed.conc.+Mai n.Thr.Conc.+Nitrogen.Conc.+Lys_conc.+PL+PO4.apporte; 0.401, 0.702, tmp+P-Source.Feed.conc.+Main.Thr.Conc.+Nitrogen.Con c.+Lys conc.+yield+lactate+SO4.ferm.+PO4.apporte+PO4.utilis e; 0.401, 0.702, tmp+P-Source.Feed.conc.+Main.Thr.Conc.+Nit rogen.Conc.+Lys_conc.+yield+lactate+SO4.ferm.+PO4.ferm.+PO4. apporte; 0.390, 0.702, pH+tmp+Main.P-Source.Conc.+P-Source. Feed.conc.+Main.Thr.Cone.+Nitrogen.Conc.+Lys_conc.+PL+lacta te+SO4.ferm.+PO4.apporte; 0.422, 0.702, tmp+P-Source.Feed.c onc.+Main.Thr.Conc.+Nitrogen.Conc.+Lys_conc.+yield+lactate+ PO4.apporte; 0.390, 0.702, pH+tmp+P-Source.Feed.conc.+Main. Thr.Conc.+Nitrogen.Conc.+OD+Lys_conc.+yield+lactate+SO4.fer m.+PO4.apporte; 0.401, 0.702, tmp+P-Source.Feed.conc.+Main. Thr.Conc.+Nitrogen.Conc.+OD+Lys_conc.+yield+PL+SO4.ferm.+PO 4.apporte; 0.411, 0.702, tmp+Main.P-Source.Conc.+P-Source.F eed.conc.+Main.Thr.Conc.+Lys_conc.+lactate+SO4.ferm.+PO4.fe rm.+PO4.utilise; 0.411, 0.702, tmp+P-Source.Feed.conc.+Main. Thr.Conc.+Nitrogen.Conc.+OD+Lys_conc.+SO4.ferm.+PO4.ferm.+P O4.utilise; 0.401, 0.702, pH+tmp+P-Source.Feed.conc.+Main.T hr.Conc.+OD+Lys_conc.+PL+His+SO4.ferm.+PO4.apporte; 0.400, 0.702, pH+tmp+P-Source.Feed.conc.+Main.Thr.Conc.+Nitrogen.C one.+Lys_conc.+yield+SO4.ferm.+PO4.ferm.+PO4.utilise; 0.400, 0.702, tmp+P-Source.Feed.conc.+Main.Thr.Conc.+OD+Lys_conc. +yield+PL+lactate+SO4.ferm.+PO4.apporte; 0.411, 0.702, pH+t mp+Main.P-Source.Conc.+P-Source.Feed.conc.+Main.Thr.Conc.+L ys_conc.+SO4.ferm.+PO4.ferm.+PO4.utilise; 0.400, 0.702, pH+ tmp+P-Source.Feed.conc.+Main.Thr.Conc.+OD+Lys_conc.+yield+P L+SO4.ferm.+PO4.apporte; 0.389, 0.702, pH+tmp+Main.P-Source. Conc.+P-Source.Feed.conc.+Main.Thr.Conc.+Nitrogen.Conc.+OD+ Lys_conc.+lactate+SO4.ferm.+PO4.apporte; 0.400, 0.702, tmp+ P-Source.Feed.conc.+Main.Thr.Conc.+Nitrogen.Conc.+Lys_conc. +yield+lactate+SO4.ferm.+PO4.ferm.+PO4.utilise; 0.411, 0.70 2, pH+tmp+P-Source.Feed.conc.+Main.Thr.Conc.+OD+Lys_conc.+S O4.ferm.+PO4.apporte+PO4.utilise; 0.421, 0.702, pH+tmp+Main. P-Source.Conc.+P-Source.Feed.conc.+Main.Thr.Conc.+Nitrogen. Conc.+Lys_conc.+PO4.apporte; 0.400, 0.702, tmp+Main.P-Sourc e.Conc.+P-Source.Feed.conc.+Main.Thr.Conc.+Nitrogen.Conc.+L ys_conc.+yield+SO4.ferm.+PO4.ferm.+PO4.apporte; 0.431, 0.70 2, tmp+P-Source.Feed.conc.+Main.Thr.Conc.+Nitrogen.Conc.+OD +Lys_conc.+PO4.apporte; 0.400, 0.702, tmp+Main.P-Source.Con c.+P-Source.Feed.conc.+Main.Thr.Cone.+Nitrogen.Cone.+Lys_co nc.+yield+SO4.ferm.+PO4.apporte+PO4.utilise; 0.389, 0.702, pH+tmp+P-Source.Feed.conc.+Main.Thr.Conc.+Nitrogen.Conc.+Ly s_conc.+yield+PL+lactate+SO4.ferm.+PO4.apporte; 0.411, 0.70 2, pH+tmp+P-Source.Feed.conc.+Main.Thr.Conc.+Lys_conc.+PL+S O4.ferm.+PO4.apporte+PO4.utilise; 0.400, 0.702, tmp+Main.P-Source.Conc.+P-Source.Feed.conc.+Main.Thr.Conc.+Nitrogen.Co nc.+Lys_conc.+His+lactate+SO4.ferm.+PO4.apporte; 0.410, 0.7 01, pH+tmp+P-Source.Feed.conc.+Main.Thr.Conc.+Lys_conc.+PL+ SO4.ferm.+PO4.ferm.+PO4.apporte; 0.421, 0.701, tmp+Main.P-S ource.Conc.+P-Source.Feed.conc.+Main.Thr.Conc.+Nitrogen.Con c.+Lys_conc.+lactate+SO4.ferm.; 0.431, 0.701, tmp+P-Source. Feed.conc.+Main.Thr.Conc.+OD+Lys_conc.+yield+PO4.apporte; 0. 421, 0.701, pH+tmp+P-Source.Feed.conc.+Main.Thr.Conc.+Nitro gen.Conc.+OD+Lys_conc.+PO4.apporte; 0.410, 0.701, pH+tmp+Ma in.P-Source.Conc.+P-Source.Feed.conc.+Main.Thr.Conc.+Nitrog en.Conc.+Lys_conc.+SO4.ferm.+PO4.utilise; 0.410, 0.701, tmp +P-Source.Feed.conc.+Main.Thr.Conc.+Nitrogen.Conc.+OD+Lys_c onc.+His+SO4.ferm.+PO4.utilise; 0.410, 0.701, tmp+P-Source. Feed.conc.+Main.Thr.Conc.+Lys_conc.+PL+SO4.ferm.+PO4.ferm.+ PO4.apporte+PO4.utilise; 0.410, 0.701, pH+tmp+P-Source.Feed. conc.+Main.Thr.Conc.+OD+Lys_conc.+SO4.ferm.+PO4.ferm.+PO4.a pporte; 0.399, 0.701, tmp+P-Source.Feed.conc.+Main.Thr.Conc. +Nitrogen.Conc.+Lys_conc.+yield+SO4.ferm.+PO4.ferm.+PO4.app orte+PO4.utilise; 0.410, 0.701, pH+tmp+P-Source.Feed.conc.+ Main.Thr.Conc.+Lys_conc.+PL+SO4.ferm.+PO4.ferm.+PO4.utilis e; 0.388, 0.701, tmp+Main.P-Source.Conc.+P-Source.Feed.conc. +Main.Thr.Conc.+Nitrogen.Conc.+Lys_conc.+lactate+SO4.ferm.+ PO4.ferm.+PO4.apporte+PO4.utilise; 0.399, 0.701, tmp+Main.P -Source.Conc.+P-Source.Feed.conc.+Main.Thr.Conc.+Nitrogen.C onc.+Lys_conc.+His+SO4.ferm.+PO4.ferm.+PO4.apporte; 0.420, 0.701, pH+tmp+P-Source.Feed.conc.+Main.Thr.Conc.+Nitrogen.C onc.+Lys_conc.+His+PO4.apporte; 0.388, 0.701, pH+tmp+Main.P -Source.Conc.+P-Source.Feed.conc.+Main.Thr.Conc.+Nitrogen.C onc.+OD+Lys_conc.+His+SO4.ferm.+PO4.apporte; 0.399, 0.701, tmp+Main.P-Source.Conc.+P-Source.Feed.conc.+Main.Thr.Conc.+ Nitrogen.Conc.+Lys_conc.+His+SO4.ferm.+PO4.apporte+PO4.util ise; 0.439, 0.701, tmp+P-Source.Feed.conc.+Main.Thr.Conc.+L ys_conc.+lactate+PO4.apporte; 0.398, 0.701, tmp+Main.P-Sour ce.Conc.+P-Source.Feed.conc.+Main.Thr.Conc.+Nitrogen.Conc.+ Lys_conc.+yield+SO4.ferm.+PO4.ferm.+PO4.utilise; 0.430, 0.7 01, pH+tmp+P-Source.Feed.conc.+Main.Thr.Conc.+Lys_conc.+yie ld+PO4.apporte; 0.409, 0.701, pH+tmp+Main.P-Source.Conc.+P-Source.Feed.conc.+Main.Thr.Conc.+Nitrogen.Conc.+Lys conc.+l actate+SO4.ferm.; 0.409, 0.701, pH+tmp+P-Source.Feed.conc.+ Main.Thr.Conc.+OD+Lys_conc.+yield+SO4.ferm.+PO4.utilise; 0. 409, 0.701, pH+tmp+P-Source.Feed.conc.+Main.Thr.Conc.+OD+Ly s_conc.+SO4.ferm.+PO4.ferm.+PO4.utilise; 0.398, 0.701, tmp+ Main.P-Source.Conc.+P-Source.Feed.conc.+Main.Thr.Conc.+Nitr ogen.Conc.+OD+Lys_conc.+PL+SO4.ferm.+PO4.apporte; 0.448, 0. 701, tmp+Main.Thr.Conc.+Lys_conc.+SO4.ferm.+PO4.apporte; 0. 398, 0.701, pH+tmp+Main.P-Source.Conc.+P-Source.Feed.conc.+ Main.Thr.Conc.+OD+Lys_conc.+yield+SO4.ferm.+PO4.apporte; 0. 409, 0.701, tmp+Main.P-Source.Conc.+P-Source.Feed.conc.+Mai n.Thr.Conc.+Lys_conc.+PL+His+SO4.ferm.+PO4.apporte; 0.398, 0.701, tmp+P-Source.Feed.conc.+Main.Thr.Conc.+Nitrogen.Conc. +Lys conc.+PL+SO4.ferm.+PO4.ferm.+PO4.apporte+PO4.utilise; 0.409, 0.700, tmp+Main.P-Source.Conc.+P-Source.Feed.conc.+M ain.Thr.Conc.+OD+Lys_conc.+PL+SO4.ferm.+PO4.apporte; 0.387, 0.700, pH+tmp+Main.P-Source.Conc.+P-Source.Feed.conc.+Main. Thr.Conc.+Nitrogen.Conc.+Lys_conc.+yield+PL+SO4.ferm.+PO4.a pporte; 0.398, 0.700, tmp+Main.P-Source.Conc.+P-Source.Feed. conc.+Main.Thr.Conc.+Nitrogen.Conc.+Lys_conc.+His+SO4.ferm. +PO4.ferm.+PO4.utilise; 0.387, 0.700, pH+tmp+Main.P-Source. Conc.+P-Source.Feed.conc.+Main.Thr.Conc.+Nitrogen.Conc.+Lys _conc.+lactate+SO4.ferm.+PO4.ferm.+PO4.apporte; 0.387, 0.70 0, pH+tmp+Main.P-Source.Conc.+P-Source.Feed.conc.+Main.Thr. Conc.+Nitrogen.Conc.+Lys_conc.+lactate+SO4.ferm.+PO4.apport e+PO4.utilise; 0.387, 0.700, pH+tmp+Main.P-Source.Conc.+P-S ource.Feed.conc.+Main.Thr.Conc.+Nitrogen.Conc.+OD+Lys_conc. +yield+SO4.ferm.+PO4.apporte; 0.387, 0.700, tmp+Main.P-Sour ce.Conc.+P-Source.Feed.conc.+Main.Thr.Conc.+Nitrogen.Conc.+ Lys_conc.+yield+PL+lactate+SO4.ferm.+PO4.apporte; 0.419, 0. 700, tmp+P-Source.Feed.conc.+Main.Thr.Conc.+Nitrogen.Conc.+ Lys_conc.+PO4.ferm.+PO4.apporte+PO4.utilise; 0.408, 0.700, tmp+P-Source.Feed.conc.+Main.Thr.Conc.+OD+Lys_conc.+PL+His+ SO4.ferm.+PO4.utilise; 0.408, 0.700, tmp+P-Source.Feed.conc. +Main.Thr.Conc.+OD+Lys_conc.+lactate+SO4.ferm.+PO4.apporte+ PO4.utilise; 0.408, 0.700, tmp+P-Source.Feed.conc.+Main.Thr. Conc.+OD+Lys_conc.+yield+lactate+SO4.ferm.+PO4.utilise; 0.3 86, 0.700, pH+tmp+Main.P-Source.Conc.+P-Source.Feed.conc.+M ain.Thr.Conc.+Nitrogen.Conc.+Lys_conc.+PL+His+SO4.ferm.+PO4. apporte; 0.419, 0.700, pH+tmp+P-Source.Feed.conc.+Main.Thr. Conc.+Nitrogen.Conc.+Lys_conc.+PL+PO4.apporte; 0.428, 0.700, tmp+P-Source.Feed.conc.+Main.Thr.Conc.+OD+Lys-conc.+SO4.fe rm.+PO4.utilise; 0.418, 0.700, tmp+P-Source.Feed.conc.+Main. Thr.Conc.+Lys_conc.+yield+His+SO4.ferm.+PO4.apporte; 0.397, 0.700, pH+tmp+P-Source.Feed.conc.+Main.Thr.Conc.+Nitrogen. Conc.+Lys_conc.+yield+His+SO4.ferm.+PO4.apporte; 0.397, 0.7 00, pH+tmp+P-Source.Feed.conc.+Main.Thr.Conc.+Nitrogen.Conc. +Lys_conc.+His+SO4.ferm.+PO4.ferm.+PO4.utilise; 0.408, 0.70 0, tmp+P-Source.Feed.conc.+Main.Thr.Conc.+OD+Lys_conc.+lact ate+SO4.ferm.+PO4.ferm.+PO4.apporte; 0.397, 0.700, pH+tmp+P -Source.Feed.conc.+Main.Thr.Conc.+Lys_conc.+lactate+SO4.fer m.+PO4.ferm.+PO4.apporte+PO4.utilise; 0.408, 0.700, tmp+Mai n.P-Source.Conc.+P-Source.Feed.conc.+Main.Thr.Conc.+Nitroge n.Conc.+Lys_conc.+lactate+SO4.ferm.+PO4.utilise; 0.408, 0.7 00, tmp+Main.P-Source.Conc.+P-Source.Feed.conc.+Main.Thr.Co nc.+Lys_conc.+yield+SO4.ferm.+PO4.apporte+PO4.utilise; 0.39 7, 0.700, pH+tmp+Main.P-Source.Conc.+P-Source.Feed.conc.+Ma in.Thr.Conc.+OD+Lys-conc.+His+SO4.ferm.+PO4.apporte; 0.408, 0.700, tmp+Main.P-Source.Conc.+P-Source.Feed.conc.+Main.Th r.Conc.+Lys_conc.+yield+SO4.ferm.+PO4.ferm.+PO4.apporte; 0. 397, 0.700, pH+tmp+Main.P-Source.Conc.+P-Source.Feed.conc.+ Main.Thr.Conc.+Lys_conc.+PL+lactate+SO4.ferm.+PO4.apporte; 0.397, 0.700, pH+tmp+P-Source.Feed.conc.+Main.Thr.Conc.+Nit rogen.Conc.+Lys_conc.+His+SO4.ferm.+PO4.ferm.+PO4.apporte; 0.397, 0.700, pH+tmp+P-Source.Feed.conc.+Main.Thr.Conc.+Nit rogen.Conc.+Lys_conc.+His+SO4.ferm.+PO4.apporte+PO4.utilis e; 0.397, 0.700, pH+tmp+P-Source.Feed.conc.+Main.Thr.Conc.+ Nitrogen.Conc.+Lys_conc.+PL+SO4.ferm.+PO4.apporte+PO4.utili se; 0.397, 0.700, pH+tmp+Main.P-Source.Conc.+P-Source.Feed. conc.+Main.Thr.Conc.+OD+Lys conc.+lactate+SO4.ferm.+PO4.app orte; 0.408, 0.700, tmp+P-Source.Feed.conc.+Main.Thr.Conc.+ Lys_conc.+yield+PL+SO4.ferm.+PO4.apporte+PO4.utilise; 0.408, 0.700, pH+tmp+P-Source.Feed.conc.+Main.Thr.Conc.+Lys_conc. +His+SO4.ferm.+PO4.ferm.+PO4.utilise; 0.397, 0.700, pH+tmp+ P-Source.Feed.conc.+Main.Thr.Conc.+Nitrogen.Conc.+OD+Lys_co nc.+SO4.ferm.+PO4.apporte+PO4.utilise; 0.407, 0.700, tmp+P-Source.Feed.conc.+Main.Thr.Conc.+Lys_conc.+yield+PL+SO4.fer m.+PO4.ferm.+PO4.apporte; 0.407, 0.700, tmp+P-Source.Feed.c onc.+Main.Thr.Conc.+Lys_conc.+yield+PL+SO4.ferm.+PO4.ferm.+ PO4.utilise; 0.397, 0.700, tmp+P-Source.Feed.conc.+Main.Thr. Conc.+Nitrogen.Conc.+Lys conc.+PL+lactate+SO4.ferm.+PO4.app orte+PO4.utilise; 0.407, 0.700, pH+tmp+P-Source.Feed.conc.+ Main.Thr.Conc.+Lys_conc.+His+lactate+SO4.ferm.+PO4.apporte; 0.397, 0.700, pH+tmp+P-Source.Feed.conc.+Main.Thr.Conc.+Ni trogen.Conc.+Lys_conc.+PL+SO4.ferm.+PO4.ferm.+PO4.apporte; 0.407, 0.700, tmp+Main.P-Source.Conc.+P-Source.Feed.conc.+M ain.Thr.Conc.+Lys_conc.+yield+SO4.ferm.+PO4.ferm.+PO4.utili se; 0.407, 0.699, tmp+Main.P-Source.Conc.+P-Source.Feed.con c.+Main.Thr.Conc.+Nitrogen.Conc.+Lys_conc.+His+SO4.ferm.+PO 4.utilise; 0.396, 0.699, tmp+P-Source.Feed.conc.+Main.Thr.C onc.+Nitrogen.Conc.+Lys_conc.+PL+lactate+SO4.ferm.+PO4.ferm. +PO4.apporte; 0.407, 0.699, tmp+P-Source.Feed.conc.+Main.Th r.Conc.+OD+Lys_conc.+yield+His+SO4.ferm.+PO4.apporte; 0.385, 0.699, pH+tmp+Main.P-Source.Conc.+P-Source.Feed.conc.+Main. Thr.Conc.+Nitrogen.Conc.+Lys_conc.+lactate+SO4.ferm.+PO4.fe rm.+PO4.utilise; 0.407, 0.699, tmp+P-Source.Feed.conc.+Main. Thr.Conc.+OD+Lys conc.+lactate+SO4.ferm.+PO4.ferm.+PO4.util ise; 0.396, 0.699, pH+tmp+P-Source.Feed.conc.+Main.Thr.Conc. +Lys_conc.+yield+lactate+SO4.ferm.+PO4.apporte+PO4.utilise; 0.407, 0.699, tmp+Main.P-Source.Conc.+P-Source.Feed.conc.+ Main.Thr.Conc.+Lys_conc.+SO4.ferm.+PO4.ferm.+PO4.apporte+PO 4.utilise; 0.396, 0.699, pH+tmp+P-Source.Feed.conc.+Main.Th r.Conc.+Nitrogen.Conc.+OD+Lys_conc.+SO4.ferm.+PO4.ferm.+PO4. apporte; 0.417, 0.699, pH+tmp+P-Source.Feed.conc.+Main.Thr. Conc.+OD+Lys conc.+yield+PO4.apporte; 0.396, 0.699, pH+tmp+ P-Source.Feed.conc.+Main.Thr.Conc.+Lys_conc.+yield+lactate+ SO4.ferm.+PO4.ferm.+PO4.apporte; 0.407, 0.699, pH+tmp+P-Sou rce.Feed.conc.+Main.Thr.Conc.+Lys_conc.+His+SO4.ferm.+PO4.f erm.+PO4.apporte; 0.407, 0.699, tmp+P-Source.Feed.conc.+Mai n.Thr.Conc.+OD+Lys_conc.+yield+SO4.ferm.+PO4.apporte+PO4.ut ilise; 0.396, 0.699, pH+tmp+P-Source.Feed.conc.+Main.Thr.Co nc.+Lys_conc.+yield+lactate+SO4.ferm.+PO4.ferm.+PO4.utilis e; 0.407, 0.699, pH+tmp+P-Source.Feed.conc.+Main.Thr.Conc.+ Lys_conc.+His+SO4.ferm.+PO4.apporte+PO4.utilise; 0.396, 0.6 99, tmp+Main.P-Source.Conc.+P-Source.Feed.conc.+Main.Thr.Co nc.+Lys_conc.+yield+PL+lactate+SO4.ferm.+PO4.apporte; 0.396, 0.699, pH+tmp+P-Source.Feed.conc.+Main.Thr.Conc.+Nitrogen. Conc.+Lys_conc.+PL+SO4.ferm.+PO4.ferm.+PO4.utilise; 0.407, 0.699, tmp+P-Source.Feed.conc.+Main.Thr.Conc.+OD+Lys_conc.+ His+SO4.ferm.+PO4.apporte+PO4.utilise; 0.406, 0.699, tmp+P-Source.Feed.conc.+Main.Thr.Conc.+OD+Lys conc.+His+SO4.ferm. +PO4.ferm.+PO4.apporte; 0.396, 0.699, tmp+Main.P-Source.Con c.+P-Source.Feed.conc.+Main.Thr.Cone.+Nitrogen.Cone.+Lys_co nc.+PL+SO4.ferm.+PO4.apporte+PO4.utilise; 0.396, 0.699, tmp +Main.P-Source.Conc.+P-Source.Feed.conc.+Main.Thr.Conc.+Nit rogen.Conc.+Lys_conc.+PL+SO4.ferm.+PO4.ferm.+PO4.apporte; 0. 427, 0.699, pH+tmp+P-Source.Feed.conc.+Main.Thr.Conc.+Lys_c onc.+lactate+PO4.apporte; 0.406, 0.699, tmp+P-Source.Feed.c onc.+Main.Thr.Conc.+OD+Lys_conc.+yield+SO4.ferm.+PO4.ferm.+ PO4.apporte; 0.395, 0.699, tmp+P-Source.Feed.conc.+Main.Thr. Conc.+Nitrogen.Conc.+Lys_conc.+PL+lactate+SO4.ferm.+PO4.fer m.+PO4.utilise; 0.384, 0.699, pH+tmp+P-Source.Feed.conc.+Ma in.Thr.Conc.+Nitrogen.Conc.+Lys_conc.+lactate+SO4.ferm.+PO4. ferm.+PO4.apporte+PO4.utilise; 0.406, 0.699, tmp+P-Source.F eed.conc.+Main.Thr.Conc.+OD+Lys_conc.+His+SO4.ferm.+PO4.fer m.+PO4.utilise; 0.406, 0.699, tmp+P-Source.Feed.conc.+Main. Thr.Conc.+OD+Lys_conc.+His+lactate+SO4.ferm.+PO4.utilise; 0. 395, 0.699, tmp+P-Source.Feed.conc.+Main.Thr.Conc.+Nitrogen. Conc.+Lys_conc.+PL+His+lactate+SO4.ferm.+PO4.apporte; 0.416, 0.699, tmp+Main.P-Source.Conc.+P-Source.Feed.conc.+Main.Th r.Conc.+Nitrogen.Conc.+Lys_conc.+PL+SO4.ferm.; 0.427, 0.699, tmp+P-Source.Feed.conc.+Main.Thr.Conc.+Lys_conc.+yield+lac tate+PO4.apporte; 0.395, 0.699, tmp+Main.P-Source.Conc.+P-S ource.Feed.conc.+Main.Thr.Conc.+OD+Lys_conc.+yield+lactate+ SO4.ferm.+PO4.apporte; 0.406, 0.699, tmp+P-Source.Feed.conc. +Main.Thr.Conc.+OD+Lys conc.+His+lactate+SO4.ferm.+PO4.appo rte; 0.406, 0.699, tmp+P-Source.Feed.conc.+Main.Thr.Conc.+O D+Lys_conc.+SO4.ferm.+PO4.ferm.+PO4.apporte+PO4.utilise; 0. 406, 0.699, tmp+P-Source.Feed.conc.+Main.Thr.Conc.+OD+Lys_c onc.+yield+SO4.ferm.+PO4.ferm.+PO4.utilise; 0.426, 0.699, p H+tmp+P-Source.Feed.conc.+Main.Thr.Conc.+OD+Lys_conc.+PO4.a pporte; 0.395, 0.699, pH+tmp+P-Source.Feed.conc.+Main.Thr.C onc.+Nitrogen.Conc.+OD+Lys_conc.+SO4.ferm.+PO4.ferm.+PO4.ut ilise; 0.406, 0.698, tmp+P-Source.Feed.conc.+Main.Thr.Conc. +OD+Lys-conc.+yield+His+SO4.ferm.+PO4.utilise; 0.426, 0.698, tmp+P-Source.Feed.conc.+Main.Thr.Conc.+Nitrogen.Conc.+Lys_ conc.+SO4.ferm.+PO4.utilise; 0.405, 0.698, pH+tmp+P-Source. Feed.conc.+Main.Thr.Conc.+Nitrogen.Conc.+Lys_conc.+yield+la ctate+PO4.apporte; 0.383, 0.698, pH+tmp+P-Source.Feed.conc. +Main.Thr.Conc.+Nitrogen.Conc.+OD+Lys_conc.+PL+His+SO4.ferm. +PO4.apporte; 0.394, 0.698, tmp+P-Source.Feed.conc.+Main.Th r.Conc.+Nitrogen.Conc.+OD+Lys_conc.+SO4.ferm.+PO4.ferm.+PO4. apporte+PO4.utilise; 0.416, 0.698, tmp+P-Source.Feed.conc.+ Main.Thr.Conc.+Nitrogen.Conc.+OD+Lys_conc.+yield+PO4.apport e; 0.405, 0.698, tmp+Main.P-Source.Conc.+P-Source.Feed.conc. +Main.Thr.Conc.+Nitrogen.Conc.+Lys_conc.+yield+lactate+SO4. ferm.; 0.383, 0.698, pH+tmp+P-Source.Feed.conc.+Main.Thr.Co nc.+Nitrogen.Conc.+OD+Lys_conc.+PL+lactate+SO4.ferm.+PO4.ap porte; 0.415, 0.698, tmp+P-Source.Feed.conc.+Main.Thr.Conc. +OD+Lys_conc.+lactate+SO4.ferm.+PO4.utilise; 0.383, 0.698, tmp+Main.P-Source.Conc.+P-Source.Feed.conc.+Main.Thr.Conc.+ Nitrogen.Conc.+OD+Lys_conc.+yield+lactate+SO4.ferm.+PO4.app orte; 0.383, 0.698, pH+tmp+Main.P-Source.Conc.+P-Source.Fee d.conc.+Main.Thr.Conc.+Nitrogen.Conc.+Lys_conc.+yield+SO4.f erm.+PO4.ferm.+PO4.apporte; 0.383, 0.698, pH+tmp+Main.P-Sou rce.Conc.+P-Source.Feed.conc.+Main.Thr.Conc.+Nitrogen.Conc. +Lys_conc.+yield+SO4.ferm.+PO4.apporte+PO4.utilise; 0.394, 0.698, tmp+Main.P-Source.Conc.+P-Source.Feed.conc.+Main.Thr. Conc.+Nitrogen.Conc.+Lys_conc.+PL+SO4.ferm.+PO4.ferm.+PO4.u tilise; 0.405, 0.698, tmp+Main.P-Source.Conc.+P-Source.Feed. conc.+Main.Thr.Conc.+Lys_conc.+PL+SO4.ferm.+PO4.apporte+PO4. utilise; 0.394, 0.698, tmp+P-Source.Feed.conc.+Main.Thr.Con c.+Lys_conc.+yield+lactate+SO4.ferm.+PO4.ferm.+PO4.apporte+ PO4.utilise; 0.405, 0.698, tmp+Main.P-Source.Conc.+P-Source. Feed.conc.+Main.Thr.Conc.+Lys_conc.+PL+SO4.ferm.+PO4.ferm.+ PO4.apporte; 0.405, 0.698, tmp+Main.P-Source.Conc.+P-Source. Feed.conc.+Main.Thr.Conc.+Nitrogen.Conc.+Lys_conc.+yield+SO 4.ferm.+PO4.utilise; 0.394, 0.698, pH+tmp+Main.P-Source.Con c.+P-Source.Feed.conc.+Main.Thr.Conc.+Lys_conc.+lactate+SO4. ferm.+PO4.apporte+PO4.utilise; 0.394, 0.698, tmp+P-Source.F eed.conc.+Main.Thr.Conc.+Nitrogen.Conc.+OD+Lys_conc.+yield+ His+SO4.ferm.+PO4.apporte; 0.394, 0.698, pH+tmp+Main.P-Sour ce.Conc.+P-Source.Feed.conc.+Main.Thr.Conc.+Lys_conc.+lacta te+SO4.ferm.+PO4.ferm.+PO4.apporte; 0.415, 0.698, tmp+Main. P-Source.Conc.+P-Source.Feed.conc.+Main.Thr.Conc.+Nitrogen. Conc.+Lys_conc.+yield+PO4.apporte; 0.404, 0.698, tmp+P-Sour ce.Feed.conc.+Main.Thr.Conc.+Nitrogen.Conc.+OD+Lys_conc.+yi eld+SO4.ferm.+PO4.utilise; 0.404, 0.698, tmp+P-Source.Feed. conc.+Main.Thr.Conc.+Lys_conc.+PL+His+lactate+SO4.ferm.+PO4. apporte; 0.414, 0.698, tmp+Main.P-Source.Conc.+P-Source.Fee d.conc.+Main.Thr.Conc.+Nitrogen.Conc.+Lys_conc.+His+SO4.fer m.; 0.434, 0.698, tmp+P-Source.Feed.conc.+Main.Thr.Conc.+OD +Lys_conc.+PO4.apporte; 0.393, 0.698, tmp+Main.P-Source.Con c.+P-Source.Feed.conc.+Main.Thr.Conc.+Nitrogen.Conc.+Lys_co nc.+yield+His+SO4.ferm.+PO4.apporte; 0.382, 0.698, pH+tmp+M ain.P-Source.Conc.+P-Source.Feed.conc.+Main.Thr.Conc.+Nitro gen.Conc.+Lys_conc.+His+lactate+SO4.ferm.+PO4.apporte; 0.38 2, 0.697, pH+tmp+P-Source.Feed.conc.+Main.Thr.Conc.+Nitroge n.Conc.+Lys_conc.+yield+lactate+SO4.ferm.+PO4.apporte+PO4.u tilise; 0.393, 0.697, tmp+P-Source.Feed.conc.+Main.Thr.Conc. +Nitrogen.Conc.+OD+Lys_conc.+His+lactate+SO4.ferm.+PO4.appo rte; 0.434, 0.697, tmp+Main.P-Source.Conc.+P-Source.Feed.co nc.+Main.Thr.Conc.+Lys_conc.+PO4.apporte; 0.414, 0.697, tmp +P-Source.Feed.conc.+Main.Thr.Conc.+Nitrogen.Conc.+Lys_conc. +yield+PL+PO4.apporte; 0.404, 0.697, tmp+Main.P-Source.Conc. +P-Source.Feed.conc.+Main.Thr.Conc.+Lys_conc.+PL+SO4.ferm.+ PO4.ferm.+PO4.utilise; 0.393, 0.697, tmp+P-Source.Feed.conc. +Main.Thr.Conc.+Nitrogen.Conc.+Lys_conc.+yield+PL+SO4.ferm. +PO4.apporte+PO4.utilise; 0.393, 0.697, pH+tmp+P-Source.Fee d.conc.+Main.Thr.Conc.+Lys_conc.+PL+lactate+SO4.ferm.+PO4.a pporte+PO4.utilise; 0.382, 0.697, pH+tmp+P-Source.Feed.conc. +Main.Thr.Conc.+Nitrogen.Conc.+Lys_conc.+yield+lactate+SO4. ferm.+PO4.ferm.+PO4.apporte; 0.370, 0.697, pH+tmp+Main.P-So urce.Conc.+P-Source.Feed.conc.+Main.Thr.Conc.+Nitrogen.Conc. +Lys_conc.+lactate+SO4.ferm.+PO4.ferm.+PO4.apporte+PO4.util ise; 0.393, 0.697, pH+tmp+P-Source.Feed.conc.+Main.Thr.Conc. +Nitrogen.Conc.+OD+Lys_conc.+His+SO4.ferm.+PO4.utilise; 0.3 93, 0.697, pH+tmp+P-Source.Feed.conc.+Main.Thr.Conc.+Lys_co nc.+yield+SO4.ferm.+PO4.ferm.+PO4.apporte+PO4.utilise; 0.38 2, 0.697, pH+tmp+Main.P-Source.Conc.+P-Source.Feed.conc.+Ma in.Thr.Conc.+Nitrogen.Conc.+Lys conc.+His+SO4.ferm.+PO4.fer m.+PO4.apporte; 0.393, 0.697, tmp+P-Source.Feed.conc.+Main. Thr.Conc.+Nitrogen.Conc.+OD+Lys_conc.+lactate+SO4.ferm.+PO4. apporte+PO4.utilise; 0.414, 0.697, tmp+P-Source.Feed.conc.+ Main.Thr.Conc.+Nitrogen.Conc.+Lys_conc.+rab+SO4.ferm.+PO4.a pporte; 0.404, 0.697, tmp+P-Source.Feed.conc.+Main.Thr.Conc. +Lys_conc.+PL+His+SO4.ferm.+PO4.ferm.+PO4.utilise; 0.393, 0. 697, pH+tmp+Main.P-Source.Conc.+P-Source.Feed.conc.+Main.Th r.Conc.+Lys_conc.+lactate+SO4.ferm.+PO4.ferm.+PO4.utilise; 0.393, 0.697, tmp+P-Source.Feed.conc.+Main.Thr.Conc.+Nitrog en.Conc.+Lys_conc.+yield+PL+SO4.ferm.+PO4.ferm.+PO4.apport e; 0.393, 0.697, tmp+P-Source.Feed.conc.+Main.Thr.Conc.+Lys _conc.+PL+lactate+SO4.ferm.+PO4.ferm.+PO4.apporte+PO4.utili se; 0.393, 0.697, pH+tmp+Main.P-Source.Conc.+P-Source.Feed. conc.+Main.Thr.Conc.+Lys_conc.+yield+PL+SO4.ferm.+PO4.appor te; 0.393, 0.697, pH+tmp+P-Source.Feed.conc.+Main.Thr.Conc. +Lys_conc.+PL+lactate+SO4.ferm.+PO4.ferm.+PO4.apporte; 0.38 1, 0.697, pH+tmp+Main.P-Source.Conc.+P-Source.Feed.conc.+Ma in.Thr.Conc.+Nitrogen.Conc.+Lys_conc.+yield+SO4.ferm.+PO4.f erm.+PO4.utilise; 0.381, 0.697, tmp+Main.P-Source.Conc.+P-S ource.Feed.conc.+Main.Thr.Conc.+Nitrogen.Conc.+Lys_conc.+yi eld+lactate+SO4.ferm.+PO4.ferm.+PO4.apporte; 0.392, 0.697, pH+tmp+P-Source.Feed.conc.+Main.Thr.Conc.+Lys_conc.+PL+lact ate+SO4.ferm.+PO4.ferm.+PO4.utilise; 0.381, 0.697, tmp+Main. P-Source.Conc.+P-Source.Feed.conc.+Main.Thr.Conc.+Nitrogen. Conc.+Lys conc.+yield+lactate+SO4.ferm.+PO4.apporte+PO4.uti lise; 0.381, 0.697, tmp+Main.P-Source.Conc.+P-Source.Feed.c onc.+Main.Thr.Conc.+Nitrogen.Conc.+Lys_conc.+yield+SO4.ferm. +PO4.ferm.+PO4.apporte+PO4.utilise; 0.403, 0.697, pH+tmp+Ma in.P-Source.Conc.+P-Source.Feed.conc.+Main.Thr.Conc.+Nitrog en.Conc.+Lys_conc.+PL+SO4.ferm.; 0.403, 0.697, tmp+P-Source. Feed.conc.+Main.Thr.Conc.+OD+Lys_conc.+yield+PL+SO4.ferm.+P O4.utilise; 0.381, 0.697, pH+tmp+Main.P-Source.Conc.+P-Sour ce.Feed.conc.+Main.Thr.Conc.+Nitrogen.Conc.+Lys_conc.+His+S O4.ferm.+PO4.apporte+PO4.utilise; 0.414, 0.697, tmp+Main.P-Source.Conc.+P-Source.Feed.conc.+Main.Thr.Conc.+Nitrogen.Co nc.+Lys_conc.+lactate+PO4.apporte; 0.403, 0.697, pH+tmp+Mai n.P-Source.Conc.+P-Source.Feed.conc.+Main.Thr.Conc.+Nitroge n.Conc.+OD+Lys_conc.+SO4.ferm.; 0.392, 0.697, tmp+P-Source. Feed.conc.+Main.Thr.Conc.+Nitrogen.Conc.+OD+Lys_conc.+lacta te+SO4.ferm.+PO4.ferm.+PO4.apporte; 0.424, 0.697, tmp+P-Sou rce.Feed.conc.+Main.Thr.Conc.+Nitrogen.Conc.+Lys_conc.+PO4. apporte+PO4.utilise; 0.381, 0.697, pH+tmp+P-Source.Feed.con c.+Main.Thr.Conc.+Nitrogen.Conc.+Lys_conc.+yield+lactate+SO 4.ferm.+PO4.ferm.+PO4.utilise; 0.381, 0.697, tmp+P-Source.F eed.conc.+Main.Thr.Conc.+Nitrogen.Conc.+OD+Lys_conc.+yield+ PL+lactate+SO4.ferm.+PO4.apporte; 0.403, 0.697, pH+tmp+Main. P-Source.Conc.+P-Source.Feed.conc.+Main.Thr.Conc.+Nitrogen. Conc.+Lys_conc.+His+SO4.ferm.; 0.381, 0.697, pH+tmp+P-Sourc e.Feed.conc.+Main.Thr.Conc.+Nitrogen.Conc.+OD+Lys_conc.+yie ld+PL+SO4.ferm.+PO4.apporte; 0.392, 0.697, tmp+P-Source.Fee d.conc.+Main.Thr.Conc.+Nitrogen.Conc.+Lys_conc.+yield+PL+SO 4.ferm.+PO4.ferm.+PO4.utilise; 0.381, 0.697, tmp+Main.P-Sou rce.Conc.+P-Source.Feed.conc.+Main.Thr.Conc.+Nitrogen.Conc. +Lys_conc.+PL+SO4.ferm.+PO4.ferm.+PO4.apporte+PO4.utilise; 0.413, 0.697, pH+tmp+P-Source.Feed.conc.+Main.Thr.Conc.+OD+ Lys_conc.+SO4.ferm.+PO4.utilise; 0.381, 0.697, pH+tmp+Main. P-Source.Conc.+P-Source.Feed.conc.+Main.Thr.Conc.+Nitrogen. Conc.+OD+Lys-conc.+PL+SO4.ferm.+PO4.apporte; 0.403, 0.697, tmp+P-Source.Feed.conc.+Main.Thr.Conc.+Lys_conc.+PL+His+SO4. ferm.+PO4.ferm.+PO4.apporte; 0.403, 0.697, tmp+Main.P-Sourc e.Conc.+P-Source.Feed.conc.+Main.Thr.Conc.+Lys_conc.+His+SO 4.ferm.+PO4.ferm.+PO4.utilise; 0.403, 0.697, tmp+P-Source.F eed.conc.+Main.Thr.Conc.+Lys_conc.+PL+His+SO4.ferm.+PO4.app orte+PO4.utilise; 0.413, 0.697, tmp+Main.P-Source.Conc.+P-S ource.Feed.conc.+Main.Thr.Conc.+Nitrogen.Conc.+OD+Lys_conc. +SO4.ferm.; 0.403, 0.697, tmp+Main.P-Source.Conc.+P-Source. Feed.conc.+Main.Thr.Conc.+Lys_conc.+His+SO4.ferm.+PO4.ferm. +PO4.apporte; 0.392, 0.697, pH+tmp+Main.P-Source.Conc.+P-So urce.Feed.conc.+Main.Thr.Conc.+Nitrogen.Conc.+Lys_conc.+lac tate+SO4.ferm.+PO4.utilise; 0.380, 0.697, pH+tmp+Main.P-Sou rce.Conc.+P-Source.Feed.conc.+Main.Thr.Conc.+Nitrogen.Conc. +Lys_conc.+His+SO4.ferm.+PO4.ferm.+PO4.utilise; 0.423, 0.69 7, tmp+P-Source.Feed.conc.+Main.Thr.Conc.+Lys_conc.+yield+S O4.ferm.+PO4.utilise; 0.413, 0.697, tmp+P-Source.Feed.conc. +Main.Thr.Conc.+Nitrogen.Conc.+OD+Lys_conc.+SO4.ferm.+PO4.u tilise; 0.423, 0.697, tmp+P-Source.Feed.conc.+Main.Thr.Conc. +Lys_conc.+lactate+SO4.ferm.+PO4.utilise; 0.392, 0.697, pH+ tmp+P-Source.Feed.conc.+Main.Thr.Conc.+OD+Lys_conc.+lactate +SO4.ferm.+PO4.apporte+PO4.utilise; 0.402, 0.697, tmp+Main. P-Source.Conc.+P-Source.Feed.conc.+Main.Thr.Conc.+Lys_conc. +His+SO4.ferm.+PO4.apporte+PO4.utilise; 0.402, 0.696, pH+tm p+P-Source.Feed.conc.+Main.Thr.Conc.+Nitrogen.Conc.+Lys_con c.+PO4.ferm.+PO4.apporte+PO4.utilise; 0.433, 0.696, tmp+P-S ource.Feed.conc.+Main.Thr.Conc.+Lys_conc.+SO4.ferm.+PO4.uti lise; 0.380, 0.696, pH+tmp+P-Source.Feed.conc.+Main.Thr.Con c.+Nitrogen.Conc.+Lys_conc.+yield+SO4.ferm.+PO4.ferm.+PO4.a pporte+PO4.utilise; 0.413, 0.696, pH+tmp+P-Source.Feed.conc. +Main.Thr.Conc.+Nitrogen.Conc.+Lys_conc.+PO4.apporte+PO4.ut ilise; 0.402, 0.696, tmp+P-Source.Feed.conc.+Main.Thr.Conc. +OD+Lys_conc.+PL+SO4.ferm.+PO4.apporte+PO4.utilise; 0.423, 0.696, tmp+P-Source.Feed.conc.+Main.Thr.Conc.+Nitrogen.Conc. +Lys_conc.+PO4.ferm.+PO4.apporte; 0.391, 0.696, pH+tmp+P-So urce.Feed.conc.+Main.Thr.Conc.+OD+Lys_conc.+lactate+SO4.fer m.+PO4.ferm.+PO4.apporte; 0.380, 0.696, tmp+Main.P-Source.C onc.+P-Source.Feed.conc.+Main.Thr.Conc.+Nitrogen.Conc.+Lys_ conc.+yield+lactate+SO4.ferm.+PO4.ferm.+PO4.utilise; 0.391, 0.696, pH+tmp+Main.P-Source.Conc.+P-Source.Feed.conc.+Main. Thr.Conc.+Nitrogen.Conc.+Lys_conc.+His+SO4.ferm.+PO4.utilis e; 0.391, 0.696, tmp+P-Source.Feed.conc.+Main.Thr.Conc.+Lys _conc.+yield+PL+lactate+SO4.ferm.+PO4.apporte+PO4.utilise; 0.412, 0.696, tmp+P-Source.Feed.conc.+Main.Thr.Conc.+Nitrog en.Conc.+Lys_conc.+PL+lactate+PO4.apporte; 0.391, 0.696, tm p+P-Source.Feed.conc.+Main.Thr.Conc.+Nitrogen.Conc.+OD+Lys_ conc.+lactate+SO4.ferm.+PO4.ferm.+PO4.utilise; 0.391, 0.696, tmp+P-Source.Feed.conc.+Main.Thr.Conc.+Lys_conc.+yield+PL+ lactate+SO4.ferm.+PO4.ferm.+PO4.utilise; 0.391, 0.696, tmp+ P-Source.Feed.conc.+Main.Thr.Conc.+Lys_conc.+yield+PL+lacta te+SO4.ferm.+PO4.ferm.+PO4.apporte; 0.412, 0.696, tmp+P-Sou rce.Feed.conc.+Main.Thr.Conc.+Nitrogen.Conc.+Lys_conc.+yiel d+SO4.ferm.+PO4.utilise; 0.379, 0.696, pH+tmp+P-Source.Feed. conc.+Main.Thr.Conc.+OD+Lys_conc.+yield+PL+lactate+SO4.ferm. +PO4.apporte; 0.391, 0.696, pH+tmp+P-Source.Feed.conc.+Main. Thr.Conc.+OD+Lys_conc.+yield+His+SO4.ferm.+PO4.apporte; 0.4 02, 0.696, tmp+Main.P-Source.Conc.+P-Source.Feed.conc.+Main. Thr.Conc.+Nitrogen.Conc.+Lys_conc.+PO4.ferm.+PO4.apporte+PO 4.utilise; 0.402, 0.696, tmp+P-Source.Feed.conc.+Main.Thr.C one.+OD+Lys_conc.+PL+SO4.ferm.+PO4.ferm.+PO4.apporte; 0.391, 0.696, pH+tmp+Main.P-Source.Conc.+P-Source.Feed.conc.+Main. Thr.Conc.+OD+Lys_conc.+PL+SO4.ferm.+PO4.apporte; 0.391, 0.6 96, tmp+Main.P-Source.Conc.+P-Source.Feed.conc.+Main.Thr.Co nc.+Lys conc.+yield+lactate+SO4.ferm.+PO4.apporte+PO4.utili se; 0.432, 0.696, tmp+Main.P-Source.Conc.+Main.Thr.Conc.+Ly s_conc.+SO4.ferm.+PO4.apporte; 0.391, 0.696, tmp+Main.P-Sou rce.Conc.+P-Source.Feed.conc.+Main.Thr.Conc.+Lys_conc.+yiel d+lactate+SO4.ferm.+PO4.ferm.+PO4.apporte; 0.401, 0.696, pH +tmp+P-Source.Feed.conc.+Main.Thr.Conc.+Nitrogen.Conc.+OD+L ys_conc.+yield+PO4.apporte; 0.390, 0.696, pH+tmp+P-Source.F eed.conc.+Main.Thr.Conc.+OD+Lys conc.+His+lactate+SO4.ferm. +PO4.apporte; 0.390, 0.696, tmp+P-Source.Feed.conc.+Main.Th r.Conc.+Nitrogen.Conc.+Lys_conc.+PL+His+SO4.ferm.+PO4.ferm. +PO4.utilise; 0.390, 0.696, pH+tmp+P-Source.Feed.conc.+Main. Thr.Conc.+OD+Lys_conc.+His+SO4.ferm.+PO4.apporte+PO4.utilis e; 0.412, 0.696, pH+tmp+P-Source.Feed.conc.+Main.Thr.Conc.+ Nitrogen.Conc.+Lys_conc.+PO4.ferm.+PO4.apporte; 0.390, 0.69 6, pH+tmp+P-Source.Feed.conc.+Main.Thr.Conc.+OD+Lys_conc.+H is+SO4.ferm.+PO4.ferm.+PO4.apporte; 0.390, 0.696, tmp+Main. P-Source.Conc.+P-Source.Feed.conc.+Main.Thr.Conc.+OD+Lys_co nc.+PL+His+SO4.ferm.+PO4.apporte; 0.390, 0.696, tmp+P-Sourc e.Feed.conc.+Main.Thr.Conc.+Nitrogen.Conc.+Lys_conc.+PL+His +SO4.ferm.+PO4.ferm.+PO4.apporte; 0.390, 0.696, tmp+Main.P-Source.Conc.+P-Source.Feed.conc.+Main.Thr.Conc.+Nitrogen.Co nc.+OD+Lys-conc.+SO4.ferm.+PO4.apporte+PO4.utilise; 0.432, 0.696, tmp+P-Source.Feed.conc.+Main.Thr.Conc.+Lys_conc.+PL+ PO4.apporte; 0.379, 0.696, tmp+P-Source.Feed.conc.+Main.Thr. Conc.+Nitrogen.Conc.+Lys_conc.+yield+lactate+SO4.ferm.+PO4. ferm.+PO4.apporte+PO4.utilise; 0.390, 0.696, tmp+P-Source.F eed.conc.+Main.Thr.Conc.+Nitrogen.Conc.+Lys_conc.+PL+His+SO 4.ferm.+PO4.apporte+PO4.utilise; 0.412, 0.696, tmp+P-Source. Feed.conc.+Main.Thr.Conc.+Nitrogen.Conc.+Lys_conc.+lactate+ SO4.ferm.+PO4.utilise; 0.390, 0.696, tmp+Main.P-Source.Conc. +P-Source.Feed.conc.+Main.Thr.Conc.+Lys_conc.+yield+lactate +SO4.ferm.+PO4.ferm.+PO4.utilise; 0.390, 0.696, tmp+Main.P-Source.Conc.+P-Source.Feed.conc.+Main.Thr.Conc.+OD+Lys_conc. +PL+lactate+SO4.ferm.+PO4.apporte; 0.390, 0.696, pH+tmp+P-S ource.Feed.conc.+Main.Thr.Conc.+OD+Lys_conc.+lactate+SO4.fe rm.+PO4.ferm.+PO4.utilise; 0.390, 0.696, pH+tmp+P-Source.Fe ed.conc.+Main.Thr.Conc.+OD+Lys_conc.+His+SO4.ferm.+PO4.ferm. +PO4.utilise; 0.390, 0.696, tmp+Main.P-Source.Conc.+P-Sourc e.Feed.conc.+Main.Thr.Conc.+Nitrogen.Conc.+OD+Lys conc.+SO4. ferm.+PO4.ferm.+PO4.apporte; 0.390, 0.696, pH+tmp+P-Source. Feed.conc.+Main.Thr.Conc.+OD+Lys_conc.+PL+His+SO4.ferm.+PO4. utilise; 0.411, 0.696, tmp+P-Source.Feed.conc.+Main.Thr.Con c.+OD+Lys_conc.+yield+lactate+PO4.apporte; 0.390, 0.696, pH +tmp+P-Source.Feed.conc.+Main.Thr.Conc.+Lys_conc.+PL+SO4.fe rm.+PO4.ferm.+PO4.apporte+PO4.utilise; 0.411, 0.695, tmp+P-Source.Feed.conc.+Main.Thr.Conc.+Nitrogen.Conc.+Lys_conc.+e mission_O2+SO4.ferm.+PO4.apporte; 0.390, 0.695, tmp+Main.P-Source.Conc.+P-Source.Feed.conc.+Main.Thr.Conc.+OD+Lys_conc. +yield+PL+SO4.ferm.+PO4.apporte; 0.431, 0.695, tmp+Main.Thr. Conc.+Nitrogen.Conc.+Lys_conc.+SO4.ferm.+PO4.apporte; 0.390, 0.695, pH+tmp+Main.P-Source.Conc.+P-Source.Feed.conc.+Main. Thr.Conc.+Nitrogen.Conc.+Lys_conc.+yield+lactate+SO4.ferm.; 0.431, 0.695, tmp+P-Source.Feed.conc.+Main.Thr.Conc.+Lys_c onc.+Thr+PO4.apporte; 0.411, 0.695, tmp+P-Source.Feed.conc. +Main.Thr.Conc.+Nitrogen.Conc.+OD+Lys_conc.+lactate+PO4.app orte; 0.401, 0.695, tmp+P-Source.Feed.conc.+Main.Thr.Conc.+ OD+Lys_conc.+PL+SO4.ferm.+PO4.ferm.+PO4.utilise; 0.390, 0.6 95, pH+tmp+P-Source.Feed.conc.+Main.Thr.Conc.+OD+Lys_conc.+ SO4.ferm.+PO4.ferm.+PO4.apporte+PO4.utilise; 0.400, 0.695, pH+tmp+P-Source.Feed.conc.+Main.Thr.Conc.+Lys_conc.+yield+H is+SO4.ferm.+PO4.apporte; 0.400, 0.695, tmp+P-Source.Feed.c onc.+Main.Thr.Conc.+Nitrogen.Conc.+Lys_conc.+lactate+PO4.fe rm.+PO4.apporte+PO4.utilise; 0.389, 0.695, pH+tmp+P-Source. Feed.conc.+Main.Thr.Conc.+OD+Lys_conc.+yield+SO4.ferm.+PO4. apporte+PO4.utilise; 0.389, 0.695, tmp+Main.P-Source.Conc.+ P-Source.Feed.conc.+Main.Thr.Conc.+Lys_conc.+lactate+SO4.fe rm.+PO4.ferm.+PO4.apporte+PO4.utilise; 0.389, 0.695, tmp+P-Source .Feed.conc.+Main.Thr.Conc.+Nitrogen.Conc.+Lys_conc.+H is+SO4.ferm.+PO4.ferm.+PO4.apporte+PO4.utilise; 0.421, 0.69 5, tmp+P-Source.Feed.conc.+Main.Thr.Conc.+Lys_conc.+rab+SO4. ferm.+PO4.apporte; 0.400, 0.695, tmp+Main.P-Source.Conc.+P-Source.Feed.conc.+Main.Thr.Conc.+OD+Lys_conc.+SO4.ferm.+PO4. apporte+PO4.utilise; 0.389, 0.695, pH+tmp+P-Source.Feed.con c.+Main.Thr.Conc.+OD+Lys_conc.+yield+lactate+SO4.ferm.+PO4. utilise; 0.400, 0.695, tmp+Main.P-Source.Conc.+P-Source.Fee d.conc.+Main.Thr.Conc.+OD+Lys_conc.+His+SO4.ferm.+PO4.utili se; 0.389, 0.695, pH+tmp+P-Source.Feed.conc.+Main.Thr.Conc. +OD+Lys_conc.+yield+SO4.ferm.+PO4.ferm.+PO4.apporte; 0.400, 0.695, tmp+Main.P-Source.Conc.+P-Source.Feed.conc.+Main.Th r.Conc.+OD+Lys_conc.+SO4.ferm.+PO4.ferm.+PO4.apporte; 0.389, 0.695, pH+tmp+Main.P-Source.Conc.+P-Source.Feed.conc.+Main. Thr.Conc.+Lys_conc.+yield+SO4.ferm.+PO4.apporte+PO4.utilis e; 0.389, 0.695, pH+tmp+Main.P-Source.Conc.+P-Source.Feed.c onc.+Main.Thr.Conc.+Lys_conc.+SO4.ferm.+PO4.ferm.+PO4.appor te+PO4.utilise; 0.389, 0.695, pH+tmp+Main.P-Source.Conc.+P-Source.Feed.conc.+Main.Thr.Conc.+Lys_conc.+yield+SO4.ferm.+ PO4.ferm.+PO4.apporte; 0.400, 0.695, tmp+P-Source.Feed.conc. +Main.Thr.Conc.+Lys_conc.+His+SO4.ferm.+PO4.ferm.+PO4.appor te+PO4.utilise; 0.389, 0.695, pH+tmp+P-Source.Feed.conc.+Ma in.Thr.Conc.+OD+Lys_conc.+His+lactate+SO4.ferm.+PO4.utilis e; 0.377, 0.695, pH+tmp+Main.P-Source.Conc.+P-Source.Feed.c onc.+Main.Thr.Conc.+Nitrogen.Conc.+Lys_conc.+PL+SO4.ferm.+P 04.apporte+PO4.utilise; 0.400, 0.695, tmp+Main.P-Source.Con c.+P-Source.Feed.conc.+Main.Thr.Conc.+Nitrogen.Conc.+Lys_co nc.+yield+PL+SO4.ferm.; 0.377, 0.695, pH+tmp+Main.P-Source. Conc.+P-Source.Feed.conc.+Main.Thr.Conc.+Nitrogen.Conc.+Lys conc.+PL+SO4.ferm.+PO4.ferm.+PO4.apporte; 0.366, 0.695, pH +tmp+Main.P-Source.Conc.+P-Source.Feed.conc.+Main.Thr.Conc. +Nitrogen.Conc.+Lys_conc.+yield+PL+lactate+SO4.ferm.+PO4.ap porte; 0.377, 0.695, tmp+Main.P-Source.Conc.+P-Source.Feed. conc.+Main.Thr.Conc.+Nitrogen.Conc.+OD+Lys_conc.+PL+lactate +SO4.ferm.+PO4.apporte; 0.399, 0.695, tmp+Main.P-Source.Con c.+P-Source.Feed.conc.+Main.Thr.Conc.+Lys_conc.+His+lactate +SO4.ferm.+PO4.apporte; 0.389, 0.695, pH+tmp+P-Source.Feed. conc.+Main.Thr.Conc.+OD+Lys_conc.+yield+SO4.ferm.+PO4.ferm. +PO4.utilise; 0.377, 0.695, tmp+Main.P-Source.Conc.+P-Sourc e.Feed.conc.+Main.Thr.Conc.+Nitrogen.Conc.+OD+Lys_conc.+SO4. ferm.+PO4.ferm.+PO4.apporte+PO4.utilise; 0.399, 0.695, pH+t mp+Main.P-Source.Conc.+P-Source.Feed.conc.+Main.Thr.Conc.+N itrogen.Conc.+Lys_conc.+SO4.ferm.+PO4.ferm.; 0.399, 0.695, pH+tmp+Main.P-Source.Conc.+P-Source.Feed.conc.+Main.Thr.Con c.+Nitrogen.Conc.+Lys_conc.+yield+PO4.apporte; 0.410, 0.695, pH+tmp+P-Source.Feed.conc.+Main.Thr.Conc.+Lys_conc.+yield+ lactate+PO4.apporte; 0.410, 0.695, tmp+Main.P-Source.Conc.+ P-Source.Feed.conc.+Main.Thr.Conc.+Nitrogen.Conc.+Lys_conc. +SO4.ferm.+PO4.ferm.; 0.388, 0.695, pH+tmp+Main.P-Source.Co nc.+P-Source.Feed.conc.+Main.Thr.Conc.+Lys_conc.+yield+SO4. ferm.+PO4.ferm.+PO4.utilise; 0.388, 0.695, tmp+Main.P-Sourc e.Conc.+P-Source.Feed.conc.+Main.Thr.Conc.+Nitrogen.Conc.+O D+Lys_conc.+SO4.ferm.+PO4.ferm.+PO4.utilise; 0.377, 0.695, pH+tmp+P-Source.Feed.conc.+Main.Thr.Conc.+Nitrogen.Conc.+Ly s_conc.+PL+SO4.ferm.+PO4.ferm.+PO4.apporte+PO4.utilise; 0.3 88, 0.694, tmp+P-Source.Feed.conc.+Main.Thr.Conc.+OD+Lys_co nc.+yield+lactate+SO4.ferm.+PO4.apporte+PO4.utilise; 0.399, 0.694, pH+tmp+Main.P-Source.Conc.+P-Source.Feed.conc.+Main. Thr.Conc.+Nitrogen.Conc.+Lys_conc.+lactate+PO4.apporte; 0.3 88, 0.694, tmp+P-Source.Feed.conc.+Main.Thr.Conc.+OD+Lys_co nc.+PL+His+lactate+SO4.ferm.+PO4.utilise; 0.388, 0.694, pH+ tmp+Main.P-Source.Conc.+P-Source.Feed.conc.+Main.Thr.Conc.+ Nitrogen.Conc.+Lys_conc.+yield+SO4.ferm.+PO4.utilise; 0.388, 0.694, tmp+Main.P-Source.Conc.+P-Source.Feed.conc.+Main.Th r.Conc.+Lys_conc.+PL+lactate+SO4.ferm.+PO4.apporte+PO4.util ise; 0.419, 0.694, pH+tmp+Main.P-Source.Conc.+P-Source.Feed. conc.+Main.Thr.Conc.+Lys_conc.+PO4.apporte; 0.388, 0.694, t mp+Main.P-Source.Conc.+P-Source.Feed.conc.+Main.Thr.Conc.+L ys_conc.+PL+lactate+SO4.ferm.+PO4.ferm.+PO4.apporte; 0.388, 0.694, tmp+P-Source.Feed.conc.+Main.Thr.Conc.+OD+Lys_conc. +yield+lactate+SO4.ferm.+PO4.ferm.+PO4.apporte; 0.388, 0.69 4, tmp+P-Source.Feed.conc.+Main.Thr.Conc.+Nitrogen.Conc.+OD +Lys_conc.+yield+SO4.ferm.+PO4.apporte+PO4.utilise; 0.376, 0.694, tmp+P-Source.Feed.conc.+Main.Thr.Conc.+Nitrogen.Conc. +Lys conc.+PL+lactate+SO4.ferm.+PO4.ferm.+PO4.apporte+PO4.u tilise; 0.399, 0.694, pH+tmp+P-Source.Feed.conc.+Main.Thr.C onc.+Nitrogen.Conc.+OD+Lys_conc.+lactate+PO4.apporte; 0.399, 0.694, tmp+Main.P-Source.Conc.+P-Source.Feed.conc.+Main.Th r.Conc.+OD+Lys_conc.+SO4.ferm.+PO4.ferm.+PO4.utilise; 0.376, 0.694, pH+tmp+P-Source.Feed.conc.+Main.Thr.Conc.+Nitrogen. Conc.+OD+Lys_conc.+His+lactate+SO4.ferm.+PO4.apporte; 0.376, 0.694, pH+tmp+P-Source.Feed.conc.+Main.Thr.Conc.+Nitrogen. Conc.+OD+Lys-conc.+yield+His+SO4.ferm.+PO4.apporte; 0.388, 0.694, pH+tmp+P-Source.Feed.conc.+Main.Thr.Conc.+OD+Lys_con c.+yield+His+SO4.ferm.+PO4.utilise; 0.376, 0.694, pH+tmp+P-Source.Feed.conc.+Main.Thr.Conc.+Nitrogen.Conc.+OD+Lys_conc. +SO4.ferm.+PO4.ferm.+PO4.apporte+PO4.utilise; 0.398, 0.694, tmp+Main.P-Source.Conc.+P-Source.Feed.conc.+Main.Thr.Conc. +Nitrogen.Conc.+OD+Lys_conc.+yield+SO4.ferm.; 0.409, 0.694, tmp+Main.P-Source.Conc.+P-Source.Feed.conc.+Main.Thr.Conc. +Nitrogen.Conc.+Lys_conc.+His+PO4.apporte; 0.364, 0.694, pH +tmp+Main.P-Source.Conc.+P-Source.Feed.conc.+Main.Thr.Conc. +Nitrogen.Conc.+OD+Lys_conc.+yield+lactate+SO4.ferm.+PO4.ap porte; 0.376, 0.694, pH+tmp+Main.P-Source.Conc.+P-Source.Fe ed.conc.+Main.Thr.Conc.+OD+Lys_conc.+yield+lactate+SO4.ferm. +PO4.apporte; 0.387, 0.694, pH+tmp+Main.P-Source.Conc.+P-So urce.Feed.conc.+Main.Thr.Conc.+Lys_conc.+PL+His+SO4.ferm.+P O4.apporte; 0.387, 0.694, tmp+Main.P-Source.Conc.+P-Source. Feed.conc.+Main.Thr.Conc.+Nitrogen.Conc.+Lys_conc.+yield+la ctate+SO4.ferm.+PO4.utilise; 0.376, 0.694, tmp+Main.P-Sourc e.Conc.+P-Source.Feed.conc.+Main.Thr.Conc.+Nitrogen.Conc.+L ys_conc.+PL+lactate+SO4.ferm.+PO4.ferm.+PO4.apporte; 0.387, 0.694, tmp+P-Source.Feed.conc.+Main.Thr.Conc.+Nitrogen.Con c.+Lys_conc.+yield+PL+His+SO4.ferm.+PO4.apporte; 0.419, 0.6 94, tmp+Main.P-Source.Conc.+P-Source.Feed.conc.+Main.Thr.Co nc.+Lys_conc.+SO4.ferm.+PO4.utilise; 0.387, 0.694, tmp+P-So urce.Feed.conc.+Main.Thr.Conc.+Nitrogen.Conc.+OD+Lys_conc.+ yield+SO4.ferm.+PO4.ferm.+PO4.apporte; 0.419, 0.694, tmp+P-Source.Feed.conc.+Main.Thr.Conc.+Lys_conc.+yield+PL+PO4.app orte; 0.387, 0.694, tmp+P-Source.Feed.conc.+Main.Thr.Conc.+ OD+Lys_conc.+yield+lactate+SO4.ferm.+PO4.ferm.+PO4.utilise; 0.376, 0.694, pH+tmp+P-Source.Feed.conc.+Main.Thr.Conc.+Ni trogen.Conc.+Lys conc.+PL+lactate+SO4.ferm.+PO4.apporte+PO4. utilise; 0.409, 0.694, tmp+P-Source.Feed.conc.+Main.Thr.Con c.+Nitrogen.Conc.+OD+Lys_conc.+His+PO4.apporte; 0.376, 0.69 4, tmp+Main.P-Source.Conc.+P-Source.Feed.conc.+Main.Thr.Con c.+Nitrogen.Conc.+Lys_conc.+PL+lactate+SO4.ferm.+PO4.apport e+PO4.utilise; 0.419, 0.694, tmp+Main.P-Source.Conc.+P-Sour ce.Feed.conc.+Main.Thr.Conc.+Lys_conc.+yield+PO4.apporte; 0. 376, 0.694, pH+tmp+Main.P-Source.Conc.+P-Source.Feed.conc.+ Main.Thr.Conc.+Nitrogen.Conc.+Lys_conc.+yield+His+SO4.ferm. +PO4.apporte; 0.398, 0.694, pH+tmp+P-Source.Feed.conc.+Main. Thr.Conc.+Nitrogen.Conc.+OD+Lys_conc.+His+PO4.apporte; 0.42 9, 0.694, tmp+Main.Thr.Conc.+Lys_conc.+PL+SO4.ferm.+PO4.app orte; 0.376, 0.694, pH+tmp+Main.P-Source.Conc.+P-Source.Fee d.conc.+Main.Thr.Conc.+Nitrogen.Conc.+Lys_conc.+PL+SO4.ferm. +PO4.ferm.+PO4.utilise; 0.387, 0.694, tmp+Main.P-Source.Con c.+P-Source.Feed.conc.+Main.Thr.Conc.+Lys_conc.+PL+lactate+ SO4.ferm.+PO4.ferm.+PO4.utilise; 0.376, 0.694, tmp+Main.P-S ource.Conc.+P-Source.Feed.conc.+Main.Thr.Conc.+Nitrogen.Con c.+OD+Lys_conc.+PL+His+SO4.ferm.+PO4.apporte; 0.376, 0.694, pH+tmp+P-Source.Feed.conc.+Main.Thr.Conc.+Nitrogen.Conc.+L ys_conc.+PL+lactate+SO4.ferm.+PO4.ferm.+PO4.apporte; 0.387, 0.694, tmp+P-Source.Feed.conc.+Main.Thr.Conc.+Nitrogen.Con c.+OD+Lys_conc.+His+SO4.ferm.+PO4.ferm.+PO4.apporte; 0.419, 0.694, tmp+P-Source.Feed.conc.+Main.Thr.Conc.+Nitrogen.Con c.+Lys_conc.+PO4.ferm.+PO4.utilise; 0.398, 0.694, tmp+Main. P-Source.Conc.+P-Source.Feed.conc.+Main.Thr.Conc.+Nitrogen. Conc.+Lys_conc.+PL+SO4.ferm.+PO4.utilise; 0.398, 0.694, pH+ tmp+P-Source.Feed.conc.+Main.Thr.Conc.+OD+Lys_conc.+lactate +SO4.ferm.+PO4.utilise; 0.387, 0.694, tmp+P-Source.Feed.con c.+Main.Thr.Conc.+Nitrogen.Conc.+OD+Lys_conc.+His+SO4.ferm. +PO4.apporte+PO4.utilise; 0.387, 0.694, pH+tmp+P-Source.Fee d.conc.+Main.Thr.Conc.+Lys_conc.+yield+PL+SO4.ferm.+PO4.app orte+PO4.utilise; 0.387, 0.694, pH+tmp+Main.P-Source.Conc.+ P-Source.Feed.conc.+Main.Thr.Conc.+Nitrogen.Conc.+Lys_conc. +PO4.ferm.+PO4.apporte+PO4.utilise; 0.408, 0.694, tmp+P-Sou rce.Feed.conc.+Main.Thr.Conc.+OD+Lys_conc.+PL+SO4.ferm.+PO4. utilise; 0.408, 0.694, tmp+P-Source.Feed.conc.+Main.Thr.Con c.+Lys_conc.+yield+lactate+SO4.ferm.+PO4.utilise; 0.419, 0. 694, pH+tmp+P-Source.Feed.conc.+Main.Thr.Conc.+Lys_conc.+PL +PO4.apporte; 0.387, 0.694, pH+tmp+P-Source.Feed.conc.+Main. Thr.Conc.+Lys_conc.+yield+PL+SO4.ferm.+PO4.ferm.+PO4.utilis e; 0.387, 0.694, pH+tmp+P-Source.Feed.conc.+Main.Thr.Conc.+ Lys_conc.+yield+PL+SO4.ferm.+PO4.ferm.+PO4.apporte; 0.364, 0.694, pH+tmp+Main.P-Source.Conc.+P-Source.Feed.conc.+Main. Thr.Conc.+Nitrogen.Conc.+Lys_conc.+yield+SO4.ferm.+PO4.ferm. +PO4.apporte+PO4.utilise; 0.387, 0.694, tmp+P-Source.Feed.c onc.+Main.Thr.Conc.+Nitrogen.Conc.+OD+Lys_conc.+His+SO4.fer m.+PO4.ferm.+PO4.utilise; 0.408, 0.694, tmp+Main.P-Source.C onc.+P-Source.Feed.conc.+Main.Thr.Conc.+Lys_conc.+lactate+S O4.ferm.+PO4.utilise; 0.408, 0.694, pH+tmp+P-Source.Feed.co nc.+Main.Thr.Conc.+Nitrogen.Conc.+Lys_conc.+PO4.ferm.+PO4.u tilise; 0.398, 0.694, pH+tmp+P-Source.Feed.conc.+Main.Thr.C onc.+Nitrogen.Conc.+Lys_conc.+rab+SO4.ferm.+PO4.apporte; 0. 428, 0.694, pH+tmp+Main.Thr.Conc.+Lys_conc.+SO4.ferm.+PO4.a pporte; 0.387, 0.694, tmp+P-Source.Feed.conc.+Main.Thr.Conc. +Lys_conc.+yield+PL+SO4.ferm.+PO4.ferm.+PO4.apporte+PO4.uti lise; 0.387, 0.693, tmp+P-Source.Feed.conc.+Main.Thr.Conc.+ Nitrogen.Conc.+OD+Lys_conc.+yield+SO4.ferm.+PO4.ferm.+PO4.u tilise; 0.418, 0.693, tmp+P-Source.Feed.conc.+Main.Thr.Conc. +Lys_conc.+emission_O2+SO4.ferm.+PO4.apporte; 0.386, 0.693, tmp+P-Source.Feed.conc.+Main.Thr.Conc.+OD+Lys_conc.+lactat e+SO4.ferm.+PO4.ferm.+PO4.apporte+PO4.utilise; 0.408, 0.693, tmp+P-Source.Feed.conc.+Main.Thr.Conc.+Nitrogen.Conc.+Lys_ conc.+His+SO4.ferm.+PO4.utilise; 0.428, 0.693, tmp+Main.Thr. Conc.+Lys_conc.+lactate+SO4.ferm.+PO4.apporte; 0.375, 0.693, pH+tmp+P-Source.Feed.conc.+Main.Thr.Conc.+Nitrogen.Conc.+L ys_conc.+PL+lactate+SO4.ferm.+PO4.ferm.+PO4.utilise; 0.375, 0.693, pH+tmp+P-Source.Feed.conc.+Main.Thr.Conc.+Nitrogen. Conc.+OD+Lys_conc.+lactate+SO4.ferm.+PO4.apporte+PO4.utilis e; 0.397, 0.693, pH+tmp+P-Source.Feed.conc.+Main.Thr.Conc.+ Nitrogen.Conc.+Lys_conc.+yield+PL+PO4.apporte; 0.428, 0.693, tmp+P-Source.Feed.conc.+Main.Thr.Conc.+Lys_conc.+His+PO4.a pporte; 0.397, 0.693, pH+tmp+P-Source.Feed.conc.+Main.Thr.C onc.+Nitrogen.Conc.+Lys_conc.+PL+lactate+PO4.apporte; 0.374, 0.693, pH+tmp+Main.P-Source.Conc.+P-Source.Feed.conc.+Main. Thr.Conc.+Nitrogen.Conc.+OD+Lys_conc.+SO4.ferm.+PO4.apporte +PO4.utilise; 0.374, 0.693, tmp+Main.P-Source.Conc.+P-Sourc e.Feed.conc.+Main.Thr.Conc.+Nitrogen.Conc.+Lys_conc.+PL+lac tate+SO4.ferm.+PO4.ferm.+PO4.utilise; 0.363, 0.693, pH+tmp+ Main.P-Source.Conc.+P-Source.Feed.conc.+Main.Thr.Conc.+Nitr ogen.Conc.+Lys_conc.+yield+lactate+SO4.ferm.+PO4.ferm.+PO4. apporte; 0.386, 0.693, tmp+P-Source.Feed.conc.+Main.Thr.Con c.+Nitrogen.Conc.+OD+Lys_conc.+PL+His+SO4.ferm.+PO4.utilis e; 0.407, 0.693, tmp+Main.P-Source.Conc.+P-Source.Feed.conc. +Main.Thr.Conc.+Nitrogen.Conc.+Lys_conc.+PL+PO4.apporte; 0. 362, 0.693, pH+tmp+Main.P-Source.Conc.+P-Source.Feed.conc.+ Main.Thr.Conc.+Nitrogen.Conc.+Lys_conc.+yield+lactate+SO4.f erm.+PO4.apporte+PO4.utilise; 0.374, 0.693, tmp+Main.P-Sour ce.Conc.+P-Source.Feed.conc.+Main.Thr.Conc.+Nitrogen.Conc.+ OD+Lys_conc.+yield+PL+SO4.ferm.+PO4.apporte; 0.386, 0.693, tmp+P-Source.Feed.conc.+Main.Thr.Conc.+Nitrogen.Conc.+Lys_c onc.+His+lactate+SO4.ferm.+PO4.ferm.+PO4.utilise; 0.374, 0. 693, pH+tmp+Main.P-Source.Conc.+P-Source.Feed.conc.+Main.Th r.Conc.+Nitrogen.Conc.+OD+Lys_conc.+SO4.ferm.+PO4.ferm.+PO4. apporte; 0.427, 0.693, tmp+Main.Thr.Conc.+Lys_conc.+yield+S O4.ferm.+PO4.apporte; 0.374, 0.693, pH+tmp+P-Source.Feed.co nc.+Main.Thr.Conc.+Nitrogen.Conc.+OD+Lys_conc.+lactate+SO4. ferm.+PO4.ferm.+PO4.apporte; 0.397, 0.693, pH+tmp+P-Source. Feed.conc.+Main.Thr.Conc.+OD+Lys_conc.+yield+lactate+PO4.ap porte; 0.385, 0.693, tmp+P-Source.Feed.conc.+Main.Thr.Conc. +Nitrogen.Conc.+Lys_conc.+His+lactate+SO4.ferm.+PO4.ferm.+P O4.apporte; 0.385, 0.693, tmp+P-Source.Feed.conc.+Main.Thr. Conc.+Nitrogen.Conc.+Lys_conc.+His+lactate+SO4.ferm.+PO4.ap porte+PO4.utilise; 0.385, 0.693, tmp+P-Source.Feed.conc.+Ma in.Thr.Conc.+Nitrogen.Conc.+OD+Lys_conc.+PL+SO4.ferm.+PO4.a pporte+PO4.utilise; 0.374, 0.693, tmp+Main.P-Source.Conc.+P -Source.Feed.conc.+Main.Thr.Conc.+Nitrogen.Conc.+Lys_conc.+ PL+His+lactate+SO4.ferm.+PO4.apporte; 0.385, 0.693, tmp+P-S ource.Feed.conc.+Main.Thr.Conc.+OD+Lys_conc.+yield+SO4.ferm. +PO4.ferm.+PO4.apporte+PO4.utilise; 0.385, 0.693, pH+tmp+P-Source.Feed.conc.+Main.Thr.Conc.+Nitrogen.Conc.+OD+Lys_conc. +yield+SO4.ferm.+PO4.utilise; 0.396, 0.693, pH+tmp+P-Source. Feed.conc.+Main.Thr.Conc.+Nitrogen.Conc.+OD+Lys_conc.+SO4.f erm.+PO4.utilise; 0.396, 0.693, tmp+P-Source.Feed.conc.+Mai n.Thr.Conc.+Lys_conc.+His+lactate+SO4.ferm.+PO4.ferm.+PO4.u tilise; 0.374, 0.693, tmp+P-Source.Feed.conc.+Main.Thr.Conc. +Nitrogen.Conc.+Lys_conc.+yield+PL+lactate+SO4.ferm.+PO4.ap porte+PO4.utilise; 0.396, 0.693, tmp+P-Source.Feed.conc.+Ma in.Thr.Conc.+Nitrogen.Conc.+OD+Lys conc.+yield+lactate+PO4. apporte; 0.407, 0.693, pH+tmp+P-Source.Feed.conc.+Main.Thr. Conc.+Nitrogen.Conc.+Lys_conc.+SO4.ferm.+PO4.utilise; 0.385, 0.693, tmp+P-Source.Feed.conc.+Main.Thr.Conc.+Nitrogen.Con c.+Lys_conc.+yield+His+lactate+SO4.ferm.+PO4.apporte; 0.417, 0.693, tmp+P-Source.Feed.conc.+Main.Thr.Conc.+OD+Lys_conc. +lactate+PO4.apporte; 0.374, 0.693, tmp+P-Source.Feed.conc. +Main.Thr.Conc.+Nitrogen.Conc.+Lys_conc.+yield+PL+lactate+S O4.ferm.+PO4.ferm.+PO4.apporte; 0.396, 0.692, tmp+P-Source. Feed.conc.+Main.Thr.Conc.+Nitrogen.Conc.+OD+Lys_conc.+lacta te+SO4.ferm.+PO4.utilise; 0.385, 0.692, tmp+P-Source.Feed.c onc.+Main.Thr.Conc.+OD+Lys_conc.+PL+His+lactate+SO4.ferm.+P O4.apporte; 0.406, 0.692, pH+tmp+P-Source.Feed.conc.+Main.T hr.Conc.+OD+Lys_conc.+lactate+PO4.apporte; 0.385, 0.692, tm p+P-Source.Feed.conc.+Main.Thr.Conc.+OD+Lys_conc.+yield+PL+ lactate+SO4.ferm.+PO4.utilise; 0.385, 0.692, tmp+P-Source.F eed.conc.+Main.Thr.Conc.+Nitrogen.Conc.+OD+Lys_conc.+PL+SO4. ferm.+PO4.ferm.+PO4.apporte; 0.385, 0.692, pH+tmp+Main.P-So urce.Conc.+P-Source.Feed.conc.+Main.Thr.Conc.+Lys_conc.+PL+ SO4.ferm.+PO4.apporte+PO4.utilise; 0.396, 0.692, tmp+Main.P -Source.Conc.+P-Source.Feed.conc.+Main.Thr.Conc.+Nitrogen.C onc.+Lys_conc.+yield+lactate+PO4.apporte; 0.396, 0.692, tmp +Main.P-Source.Conc.+P-Source.Feed.conc.+Main.Thr.Conc.+Nit rogen.Conc.+Lys_conc.+PL+lactate+SO4.ferm.; 0.385, 0.692, p H+tmp+Main.P-Source.Conc.+P-Source.Feed.conc.+Main.Thr.Conc. +Lys_conc.+PL+SO4.ferm.+PO4.ferm.+PO4.apporte; 0.373, 0.692, pH+tmp+Main.P-Source.Conc.+P-Source.Feed.conc.+Main.Thr.Co nc.+Lys_conc.+yield+PL+lactate+SO4.ferm.+PO4.apporte; 0.396, 0.692, tmp+Main.P-Source.Conc.+P-Source.Feed.conc.+Main.Th r.Conc.+OD+Lys_conc.+yield+SO4.ferm.+PO4.utilise; 0.385, 0. 692, tmp+P-Source.Feed.conc.+Main.Thr.Conc.+OD+Lys_conc.+PL +lactate+SO4.ferm.+PO4.apporte+PO4.utilise; 0.396, 0.692, t mp+P-Source.Feed.conc.+Main.Thr.Conc.+OD+Lys_conc.+PL+lacta te+SO4.ferm.+PO4.utilise; 0.385, 0.692, pH+tmp+Main.P-Sourc e.Conc.+P-Source.Feed.conc.+Main.Thr.Conc.+Nitrogen.Conc.+O D+Lys_conc.+yield+SO4.ferm.; 0.373, 0.692, pH+tmp+P-Source. Feed.conc.+Main.Thr.Conc.+Lys_conc.+yield+lactate+SO4.ferm. +PO4.ferm.+PO4.apporte+PO4.utilise; 0.395, 0.692, tmp+Main. P-Source.Conc.+P-Source.Feed.conc.+Main.Thr.Conc.+Nitrogen. Conc.+Lys_conc.+rab+SO4.ferm.+PO4.apporte; 0.384, 0.692, pH +tmp+Main.P-Source.Conc.+P-Source.Feed.conc.+Main.Thr.Conc. +OD+Lys_conc.+His+SO4.ferm.+PO4.utilise; 0.373, 0.692, pH+t mp+P-Source.Feed.conc.+Main.Thr.Conc.+Nitrogen.Conc.+Lys_co nc.+PL+His+lactate+SO4.ferm.+PO4.apporte; 0.406, 0.692, tmp +P-Source.Feed.conc.+Main.Thr.Conc.+Nitrogen.Conc.+Lys_conc. +PL+His+PO4.apporte; 0.373, 0.692, tmp+P-Source.Feed.conc.+ Main.Thr.Conc.+Nitrogen.Conc.+Lys_conc.+yield+PL+lactate+SO 4.ferm.+PO4.ferm.+PO4.utilise; 0.395, 0.692, tmp+P-Source.F eed.conc.+Main.Thr.Conc.+Lys_conc.+His+lactate+SO4.ferm.+PO 4.apporte+PO4.utilise; 0.416, 0.692, tmp+Main.P-Source.Conc. +P-Source.Feed.conc.+Main.Thr.Conc.+Lys_conc.+lactate+PO4.a pporte; 0.373, 0.692, pH+tmp+P-Source.Feed.conc.+Main.Thr.C onc.+Nitrogen.Conc.+OD+Lys_conc.+lactate+SO4.ferm.+PO4.ferm. +PO4.utilise; 0.384, 0.692, tmp+P-Source.Feed.conc.+Main.Th r.Conc.+OD+Lys_conc.+PL+His+SO4.ferm.+PO4.apporte+PO4.utili se; 0.395, 0.692, tmp+P-Source.Feed.conc.+Main.Thr.Conc.+Ly s_conc.+His+lactate+SO4.ferm.+PO4.ferm.+PO4.apporte; 0.361, 0.692, pH+tmp+Main.P-Source.Conc.+P-Source.Feed.conc.+Main. Thr.Conc.+Nitrogen.Conc.+Lys_conc.+yield+lactate+SO4.ferm.+ PO4.ferm.+PO4.utilise; 0.361, 0.692, pH+tmp+Main.P-Source.C onc.+P-Source.Feed.conc.+Main.Thr.Conc.+Nitrogen.Conc.+OD+L ys_conc.+SO4.ferm.+PO4.ferm.+PO4.apporte+PO4.utilise; 0.384, 0.692, tmp+P-Source.Feed.conc.+Main.Thr.Conc.+OD+Lys_conc. +PL+His+SO4.ferm.+PO4.ferm.+PO4.apporte; 0.395, 0.692, pH+t mp+Main.P-Source.Conc.+P-Source.Feed.conc.+Main.Thr.Conc.+N itrogen.Conc.+Lys_conc.+His+PO4.apporte; 0.384, 0.692, tmp+ P-Source.Feed.conc.+Main.Thr.Conc.+OD+Lys_conc.+PL+lactate+ SO4.ferm.+PO4.ferm.+PO4.apporte; 0.384, 0.692, pH+tmp+P-Sou rce.Feed.conc.+Main.Thr.Conc.+OD+Lys_conc.+PL+SO4.ferm.+PO4. apporte+PO4.utilise; 0.361, 0.692, pH+tmp+Main.P-Source.Con c.+P-Source.Feed.conc.+Main.Thr.Conc.+Nitrogen.Conc.+Lys_co nc.+PL+SO4.ferm.+PO4.ferm.+PO4.apporte+PO4.utilise; 0.384, 0.692, tmp+P-Source.Feed.conc.+Main.Thr.Conc.+Nitrogen.Conc. +OD+Lys-conc.+yield+lactate+SO4.ferm.+PO4.utilise; 0.384, 0. 692, tmp+P-Source.Feed.conc.+Main.Thr.Conc.+OD+Lys_conc.+PL +His+SO4.ferm.+PO4.ferm.+PO4.utilise; 0.384, 0.692, pH+tmp+ Main.P-Source.Conc.+P-Source.Feed.conc.+Main.Thr.Conc.+OD+L ys_conc.+SO4.ferm.+PO4.apporte+PO4.utilise; 0.372, 0.692, p H+tmp+Main.P-Source.Conc.+P-Source.Feed.conc.+Main.Thr.Conc. +Nitrogen.Conc.+OD+Lys_conc.+SO4.ferm.+PO4.ferm.+PO4.utilis e; 0.384, 0.692, pH+tmp+Main.P-Source.Conc.+P-Source.Feed.c onc.+Main.Thr.Conc.+Lys conc.+PL+SO4.ferm.+PO4.ferm.+PO4.ut ilise; 0.384, 0.692, tmp+P-Source.Feed.conc.+Main.Thr.Conc. +Nitrogen.Conc.+OD+Lys_conc.+His+lactate+SO4.ferm.+PO4.util ise; 0.395, 0.692, tmp+P-Source.Feed.conc.+Main.Thr.Conc.+N itrogen.Conc.+Lys_conc.+yield+lactate+SO4.ferm.+PO4.utilis e; 0.372, 0.692, tmp+Main.P-Source.Conc.+P-Source.Feed.conc. +Main.Thr.Conc.+Nitrogen.Conc.+Lys_conc.+PL+His+SO4.ferm.+P O4.ferm.+PO4.apporte; 0.416, 0.692, tmp+P-Source.Feed.conc. +Main.Thr.Conc.+Lys_conc.+PO4.ferm.+PO4.apporte+PO4.utilis e; 0.405, 0.692, tmp+P-Source.Feed.conc.+Main.Thr.Conc.+Nit rogen.Conc.+Lys_conc.+lactate+PO4.apporte+PO4.utilise; 0.40 5, 0.692, tmp+Main.P-Source.Conc.+P-Source.Feed.conc.+Main. Thr.Conc.+Lys_conc.+yield+SO4.ferm.+PO4.utilise; 0.384, 0.6 92, pH+tmp+Main.P-Source.Conc.+P-Source.Feed.conc.+Main.Thr. Conc.+OD+Lys_conc.+SO4.ferm.+PO4.ferm.+PO4.apporte; 0.395, 0.692, tmp+P-Source.Feed.conc.+Main.Thr.Conc.+Nitrogen.Conc. +Lys_conc.+yield+PL+lactate+PO4.apporte; 0.405, 0.692, tmp+ Main.P-Source.Conc.+P-Source.Feed.conc.+Main.Thr.Conc.+OD+L ys_conc.+yield+PO4.apporte; 0.384, 0.692, tmp+P-Source.Feed. conc.+Main.Thr.Conc.+Nitrogen.Conc.+OD+Lys_conc.+PL+SO4.fer m.+PO4.ferm.+PO4.utilise; 0.372, 0.692, pH+tmp+P-Source.Fee d.conc.+Main.Thr.Conc.+Nitrogen.Conc.+Lys_conc.+yield+PL+SO 4.ferm.+PO4.apporte+PO4.utilise; 0.395, 0.692, tmp+Main.P-S ource.Conc.+P-Source.Feed.conc.+Main.Thr.Conc.+Nitrogen.Con c.+OD+Lys_conc.+SO4.ferm.+PO4.utilise; 0.383, 0.692, pH+tmp +P-Source.Feed.conc.+Main.Thr.Conc.+OD+Lys-conc.+PL+SO4.fer m.+PO4.ferm.+PO4.apporte; 0.372, 0.692, tmp+Main.P-Source.C onc.+P-Source.Feed.conc.+Main.Thr.Conc.+Nitrogen.Conc.+Lys_ conc.+yield+PL+SO4.ferm.+PO4.ferm.+PO4.apporte; 0.372, 0.69 2, tmp+Main.P-Source.Conc.+P-Source.Feed.conc.+Main.Thr.Con c.+Nitrogen.Conc.+Lys_conc.+yield+PL+SO4.ferm.+PO4.apporte+ PO4.utilise; 0.360, 0.692, tmp+Main.P-Source.Conc.+P-Source. Feed.conc.+Main.Thr.Conc.+Nitrogen.Conc.+Lys_conc.+yield+la ctate+SO4.ferm.+PO4.ferm.+PO4.apporte+PO4.utilise; 0.415, 0. 692, pH+tmp+P-Source.Feed.conc.+Main.Thr.Conc.+Lys_conc.+Hi s+PO4.apporte; 0.394, 0.692, pH+tmp+P-Source.Feed.conc.+Mai n.Thr.Conc.+Nitrogen.Conc.+Lys_conc.+emission_O2+SO4.ferm.+ PO4.apporte; 0.405, 0.692, tmp+P-Source.Feed.conc.+Main.Thr. Conc.+Nitrogen.Conc.+Lys_conc.+yield+PO4.apporte+PO4.utilis e; 0.372, 0.692, pH+tmp+P-Source.Feed.conc.+Main.Thr.Conc.+ Nitrogen.Conc.+Lys_conc.+yield+PL+SO4.ferm.+PO4.ferm.+PO4.a pporte; 0.372, 0.691, tmp+Main.P-Source.Conc.+P-Source.Feed. conc.+Main.Thr.Conc.+Nitrogen.Conc.+Lys_conc.+PL+His+SO4.fe rm.+PO4.apporte+PO4.utilise; 0.405, 0.691, tmp+Main.P-Sourc e.Conc.+P-Source.Feed.conc.+Main.Thr.Conc.+Nitrogen.Conc.+O D+Lys_conc.+PO4.apporte; 0.383, 0.691, tmp+Main.P-Source.Co nc.+P-Source.Feed.conc.+Main.Thr.Conc.+Lys_conc.+yield+SO4. ferm.+PO4.ferm.+PO4.apporte+PO4.utilise; 0.383, 0.691, tmp+ P-Source.Feed.conc.+Main.Thr.Conc.+OD+Lys_conc.+PL+lactate+ SO4.ferm.+PO4.ferm.+PO4.utilise; 0.383, 0.691, pH+tmp+Main. P-Source.Conc.+P-Source.Feed.conc.+Main.Thr.Conc.+Lys_conc. +His+SO4.ferm.+PO4.ferm.+PO4.utilise; 0.425, 0.691, tmp+Mai n.Thr.Conc.+OD+Lys_conc.+SO4.ferm.+PO4.apporte; 0.383, 0.69 1, pH+tmp+P-Source.Feed.conc.+Main.Thr.Conc.+OD+Lys_conc.+y ield+PL+SO4.ferm.+PO4.utilise; 0.372, 0.691, tmp+P-Source.F eed.conc.+Main.Thr.Conc.+Nitrogen.Conc.+Lys_conc.+yield+PL+ SO4.ferm.+PO4.ferm.+PO4.apporte+PO4.utilise; 0.415, 0.691, pH+tmp+P-Source.Feed.conc.+Main.Thr.Conc.+Lys_conc.+SO4.fer m.+PO4.utilise; 0.383, 0.691, pH+tmp+Main.P-Source.Conc.+P-Source.Feed.conc.+Main.Thr.Conc.+Lys_conc.+His+SO4.ferm.+PO 4.ferm.+PO4.apporte; 0.372, 0.691, tmp+P-Source.Feed.conc.+ Main.Thr.Conc.+Nitrogen.Conc.+OD+Lys_conc.+lactate+SO4.ferm. +PO4.ferm.+PO4.apporte+PO4.utilise; 0.394, 0.691, tmp+P-Sou rce.Feed.conc.+Main.Thr.Conc.+Nitrogen.Conc.+Lys_conc.+yiel d+PO4.ferm.+PO4.apporte+PO4.utilise; 0.405, 0.691, tmp+P-So urce.Feed.conc.+Main.Thr.Conc.+Nitrogen.Conc.+Lys_conc.+lac tate+PO4.ferm.+PO4.apporte; 0.415, 0.691, tmp+P-Source.Feed. conc.+Main.Thr.Conc.+Lys_conc.+PL+lactate+PO4.apporte; 0.40 4, 0.691, pH+tmp+P-Source.Feed.conc.+Main.Thr.Conc.+Lys_con c.+rab+SO4.ferm.+PO4.apporte; 0.383, 0.691, pH+tmp+Main.P-S ource.Conc.+P-Source.Feed.conc.+Main.Thr.Conc.+Lys_conc.+Hi s+SO4.ferm.+PO4.apporte+PO4.utilise; 0.371, 0.691, tmp+Main. P-Source.Conc.+P-Source.Feed.conc.+Main.Thr.Conc.+Nitrogen. Conc.+Lys_conc.+PL+His+SO4.ferm.+PO4.ferm.+PO4.utilise; 0.4 04, 0.691, tmp+P-Source.Feed.conc.+Main.Thr.Conc.+Nitrogen. Conc.+Lys_conc.+yield+PO4.ferm.+PO4.apporte; 0.415, 0.691, tmp+P-Source.Feed.conc.+Main.Thr.Conc.+Lys_conc.+His+SO4.fe rm.+PO4.utilise; 0.383, 0.691, tmp+P-Source.Feed.conc.+Main. Thr.Conc.+OD+Lys_conc.+yield+PL+His+SO4.ferm.+PO4.utilise; 0.383, 0.691, pH+tmp+Main.P-Source.Conc.+P-Source.Feed.conc. +Main.Thr.Conc.+Nitrogen.Conc.+Lys_conc.+yield+PL+SO4.fer m.; 0.371, 0.691, pH+tmp+P-Source.Feed.conc.+Main.Thr.Conc. +Nitrogen.Conc.+Lys_conc.+yield+PL+SO4.ferm.+PO4.ferm.+PO4. utilise; 0.404, 0.691, pH+tmp+P-Source.Feed.conc.+Main.Thr. Conc.+Lys_conc.+lactate+SO4.ferm.+PO4.utilise; 0.383, 0.691, tmp+Main.P-Source.Conc.+P-Source.Feed.conc.+Main.Thr.Conc. +Nitrogen.Conc.+Lys_conc.+lactate+PO4.ferm.+PO4.apporte+PO4. utilise; 0.383, 0.691, pH+tmp+P-Source.Feed.conc.+Main.Thr. Conc.+Nitrogen.Conc.+Lys_conc.+lactate+PO4.ferm.+PO4.apport e+PO4.utilise; 0.383, 0.691, pH+tmp+P-Source.Feed.conc.+Mai n.Thr.Conc.+OD+Lys_conc.+PL+SO4.ferm.+PO4.ferm.+PO4.utilis e; 0.393, 0.691, tmp+P-Source.Feed.conc.+Main.Thr.Conc.+Nit rogen.Conc.+Lys_conc.+emission_O2+rab+SO4.ferm.+PO4.apport e; 0.359, 0.691, pH+tmp+P-Source.Feed.conc.+Main.Thr.Conc.+ Nitrogen.Conc.+OD+Lys_conc.+yield+PL+lactate+SO4.ferm.+PO4. apporte; 0.382, 0.691, pH+tmp+Main.P-Source.Conc.+P-Source. Feed.conc.+Main.Thr.Conc.+OD+Lys_conc.+SO4.ferm.+PO4.ferm.+ PO4.utilise; 0.382, 0.691, tmp+Main.P-Source.Conc.+P-Source. Feed.conc.+Main.Thr.Conc.+Lys_conc.+yield+PL+SO4.ferm.+PO4. apporte+PO4.utilise; 0.382, 0.691, tmp+Main.P-Source.Conc.+ P-Source.Feed.conc.+Main.Thr.Conc.+Nitrogen.Conc.+OD+Lys_co nc.+His+SO4.ferm.+PO4.utilise; 0.382, 0.691, tmp+Main.P-Sou rce.Conc.+P-Source.Feed.conc.+Main.Thr.Conc.+Lys_conc.+yiel d+PL+SO4.ferm.+PO4.ferm.+PO4.apporte; 0.382, 0.691, tmp+P-S ource.Feed.conc.+Main.Thr.Conc.+OD+Lys_conc.+His+SO4.ferm.+ PO4.ferm.+PO4.apporte+PO4.utilise; 0.382, 0.691, pH+tmp+P-S ource.Feed.conc.+Main.Thr.Conc.+Lys_conc.+PL+His+lactate+SO 4.ferm.+PO4.apporte; 0.404, 0.691, tmp+P-Source.Feed.conc.+ Main.Thr.Conc.+OD+Lys_conc.+yield+PL+PO4.apporte; 0.404, 0. 691, pH+tmp+P-Source.Feed.conc.+Main.Thr.Conc.+OD+Lys_conc. +His+PO4.apporte; 0.371, 0.691, tmp+Main.P-Source.Conc.+P-S ource.Feed.conc.+Main.Thr.Conc.+Nitrogen.Conc.+Lys_conc.+yi eld+PL+SO4.ferm.+PO4.ferm.+PO4.utilise; 0.393, 0.691, pH+tm p+P-Source.Feed.conc.+Main.Thr.Conc.+Nitrogen.Conc.+Lys_con c.+lactate+PO4.apporte+PO4.utilise; 0.393, 0.691, pH+tmp+Ma in.P-Source.Conc.+P-Source.Feed.conc.+Main.Thr.Conc.+Nitrog en.Conc.+OD+Lys_conc.+PO4.apporte; 0.414, 0.691, tmp+P-Sour ce.Feed.conc.+Main.Thr.Conc.+OD+Lys_conc.+yield+PO4.utilis e; 0.393, 0.691, tmp+Main.P-Source.Conc.+P-Source.Feed.conc. +Main.Thr.Conc.+Nitrogen.Conc.+Lys_conc.+yield+SO4.ferm.+PO 4.ferm.; 0.370, 0.691, pH+tmp+Main.P-Source.Conc.+P-Source. Feed.conc.+Main.Thr.Conc.+OD+Lys_conc.+PL+His+SO4.ferm.+PO4. apporte; 0.382, 0.691, tmp+Main.P-Source.Conc.+P-Source.Fee d.conc.+Main.Thr.Conc.+Lys_conc.+yield+PL+SO4.ferm.+PO4.fer m.+PO4.utilise; 0.382, 0.691, tmp+P-Source.Feed.conc.+Main. Thr.Conc.+OD+Lys_conc.+yield+PL+His+SO4.ferm.+PO4.apporte; 0.382, 0.691, pH+tmp+P-Source.Feed.conc.+Main.Thr.Conc.+Lys _conc.+PL+His+SO4.ferm.+PO4.ferm.+PO4.utilise; 0.382, 0.691, tmp+Main.P-Source.Conc.+P-Source.Feed.conc.+Main.Thr.Conc. +OD+Lys-conc.+lactate+SO4.ferm.+PO4.apporte+PO4.utilise; 0. 393, 0.691, tmp+Main.P-Source.Conc.+P-Source.Feed.conc.+Mai n.Thr.Conc.+Nitrogen.Conc.+Lys_conc.+emission_O2+SO4.ferm.+ PO4.apporte; 0.382, 0.690, tmp+Main.P-Source.Conc.+P-Source. Feed.conc.+Main.Thr.Conc.+Nitrogen.Conc.+Lys_conc.+PL+lacta te+SO4.ferm.+PO4.utilise; 0.382, 0.690, pH+tmp+Main.P-Sourc e.Conc.+P-Source.Feed.conc.+Main.Thr.Conc.+Nitrogen.Conc.+L ys_conc.+PL+SO4.ferm.+PO4.utilise; 0.424, 0.690, tmp+P-Sour ce.Feed.conc.+Main.Thr.Conc.+Lys_conc.+PO4.apporte+PO4.util ise; 0.403, 0.690, pH+tmp+Main.P-Source.Conc.+P-Source.Feed. conc.+Main.Thr.Conc.+Lys_conc.+SO4.ferm.+PO4.utilise; 0.382, 0.690, tmp+Main.P-Source.Conc.+P-Source.Feed.conc.+Main.Th r.Conc.+OD+Lys_conc.+lactate+SO4.ferm.+PO4.ferm.+PO4.apport e; 0.382, 0.690, tmp+Main.P-Source.Conc.+P-Source.Feed.conc. +Main.Thr.Conc.+Nitrogen.Conc.+Lys_conc.+PL+His+SO4.ferm.+P O4.utilise; 0.403, 0.690, tmp+P-Source.Feed.conc.+Main.Thr. Conc.+Nitrogen.Conc.+OD+Lys_conc.+PL+PO4.apporte; 0.382, 0. 690, tmp+Main.P-Source.Conc.+P-Source.Feed.conc.+Main.Thr.C onc.+Lys_conc.+PL+SO4.ferm.+PO4.ferm.+PO4.apporte+PO4.utili se; 0.403, 0.690, pH+tmp+P-Source.Feed.conc.+Main.Thr.Conc. +Lys_conc.+yield+SO4.ferm.+PO4.utilise; 0.370, 0.690, pH+tm p+P-Source.Feed.conc.+Main.Thr.Conc.+Lys_conc.+PL+lactate+S O4.ferm.+PO4.ferm.+PO4.apporte+PO4.utilise; 0.393, 0.690, t mp+P-Source.Feed.conc.+Main.Thr.Conc.+Nitrogen.Conc.+Lys_co nc.+rab+lactate+SO4.ferm.+PO4.apporte; 0.370, 0.690, pH+tmp +Main.P-Source.Conc.+P-Source.Feed.conc.+Main.Thr.Conc.+Lys _conc.+yield+lactate+SO4.ferm.+PO4.apporte+PO4.utilise; 0.3 70, 0.690, pH+tmp+Main.P-Source.Conc.+P-Source.Feed.conc.+M ain.Thr.Conc.+Lys_conc.+yield+lactate+SO4.ferm.+PO4.ferm.+P O4.apporte; 0.358, 0.690, tmp+Main.P-Source.Conc.+P-Source. Feed.conc.+Main.Thr.Conc.+Nitrogen.Conc.+Lys_conc.+PL+lacta te+SO4.ferm.+PO4.ferm.+PO4.apporte+PO4.utilise; 0.358, 0.69 0, pH+tmp+P-Source.Feed.conc.+Main.Thr.Conc.+Nitrogen.Conc. +Lys_conc.+yield+lactate+SO4.ferm.+PO4.ferm.+PO4.apporte+PO 4.utilise; 0.413, 0.690, tmp+Main.P-Source.Conc.+P-Source.F eed.conc.+Main.Thr.Conc.+OD+Lys_conc.+PO4.apporte; 0.381, 0. 690, tmp+P-Source.Feed.conc.+Main.Thr.Conc.+OD+Lys_conc.+yi eld+PL+SO4.ferm.+PO4.apporte+PO4.utilise; 0.413, 0.690, tmp +P-Source.Feed.conc.+Main.Thr.Conc.+OD+Lys_conc.+Thr+PO4.ap porte; 0.381, 0.690, tmp+P-Source.Feed.conc.+Main.Thr.Conc. +OD+Lys_conc.+PL+SO4.ferm.+PO4.ferm.+PO4.apporte+PO4.utilis e; 0.392, 0.690, tmp+P-Source.Feed.conc.+Main.Thr.Conc.+Lys _conc.+yield+PL+His+SO4.ferm.+PO4.apporte; 0.392, 0.690, pH +tmp+Main.P-Source.Conc.+P-Source.Feed.conc.+Main.Thr.Conc. +Nitrogen.Conc.+Lys_conc.+PL+PO4.apporte; 0.392, 0.690, pH+ tmp+P-Source.Feed.conc.+Main.Thr.Conc.+Nitrogen.Conc.+Lys_c onc.+lactate+PO4.ferm.+PO4.apporte; 0.370, 0.690, tmp+Main. P-Source.Conc.+P-Source.Feed.conc.+Main.Thr.Conc.+Nitrogen. Conc.+OD+Lys_conc.+lactate+SO4.ferm.+PO4.apporte+PO4.utilis e; 0.381, 0.690, tmp+P-Source.Feed.conc.+Main.Thr.Conc.+Nit rogen.Conc.+OD+Lys_conc.+yield+His+SO4.ferm.+PO4.utilise; 0. 381, 0.690, pH+tmp+Main.P-Source.Conc.+P-Source.Feed.conc.+ Main.Thr.Conc.+Nitrogen.Conc.+OD+Lys_conc.+SO4.ferm.+PO4.ut ilise; 0.413, 0.690, pH+tmp+P-Source.Feed.conc.+Main.Thr.Co nc.+Lys_conc.+PO4.apporte+PO4.utilise; 0.370, 0.690, tmp+Ma in.P-Source.Conc.+P-Source.Feed.conc.+Main.Thr.Conc.+Nitrog en.Conc.+OD+Lys_conc.+lactate+SO4.ferm.+PO4.ferm.+PO4.appor te; 0.381, 0.690, tmp+Main.P-Source.Conc.+P-Source.Feed.con c.+Main.Thr.Conc.+OD+Lys conc.+His+SO4.ferm.+PO4.ferm.+PO4. apporte; 0.370, 0.690, pH+tmp+P-Source.Feed.conc.+Main.Thr. Conc.+Nitrogen.Conc.+OD+Lys_conc.+His+SO4.ferm.+PO4.ferm.+P O4.apporte; 0.381, 0.690, tmp+P-Source.Feed.conc.+Main.Thr. Conc.+OD+Lys_conc.+yield+PL+SO4.ferm.+PO4.ferm.+PO4.apport e; 0.370, 0.690, pH+tmp+P-Source.Feed.conc.+Main.Thr.Conc.+ Nitrogen.Conc.+OD+Lys_conc.+His+SO4.ferm.+PO4.apporte+PO4.u tilise; 0.370, 0.690, pH+tmp+Main.P-Source.Conc.+P-Source.F eed.conc.+Main.Thr.Conc.+OD+Lys_conc.+yield+PL+SO4.ferm.+PO 4.apporte; 0.369, 0.690, pH+tmp+Main.P-Source.Conc.+P-Sourc e.Feed.conc.+Main.Thr.Conc.+Lys_conc.+yield+lactate+SO4.fer m.+PO4.ferm.+PO4.utilise; 0.369, 0.690, pH+tmp+Main.P-Sourc e.Conc.+P-Source.Feed.conc.+Main.Thr.Conc.+OD+Lys_conc.+PL+ lactate+SO4.ferm.+PO4.apporte; 0.403, 0.690, tmp+Main.P-Sou rce.Conc.+P-Source.Feed.conc.+Main.Thr.Conc.+OD+Lys_conc.+S O4.ferm.+PO4.utilise; 0.423, 0.690, tmp+P-Source.Feed.conc. +Main.Thr.Conc.+Lys_conc.+PO4.ferm.+PO4.apporte; 0.369, 0.6 90, pH+tmp+P-Source.Feed.conc.+Main.Thr.Conc.+Nitrogen.Conc. +OD+Lys_conc.+yield+SO4.ferm.+PO4.apporte+PO4.utilise; 0.36 9, 0.690, pH+tmp+P-Source.Feed.conc.+Main.Thr.Conc.+OD+Lys_ conc.+yield+lactate+SO4.ferm.+PO4.apporte+PO4.utilise; 0.38 1, 0.690, tmp+Main.P-Source.Conc.+P-Source.Feed.conc.+Main. Thr.Conc.+OD+Lys_conc.+His+SO4.ferm.+PO4.apporte+PO4.utilis e; 0.357, 0.690, pH+tmp+Main.P-Source.Conc.+P-Source.Feed.c onc.+Main.Thr.Conc.+Nitrogen.Conc.+OD+Lys_conc.+PL+lactate+ SO4.ferm.+PO4.apporte; 0.381, 0.690, pH+tmp+P-Source.Feed.c onc.+Main.Thr.Conc.+Lys conc.+PL+His+SO4.ferm.+PO4.ferm.+PO 4.apporte; 0.369, 0.690, pH+tmp+P-Source.Feed.conc.+Main.Th r.Conc.+Nitrogen.Conc.+OD+Lys_conc.+His+SO4.ferm.+PO4.ferm. +PO4.utilise; 0.381, 0.690, pH+tmp+P-Source.Feed.conc.+Main. Thr.Conc.+Lys_conc.+PL+His+SO4.ferm.+PO4.apporte+PO4.utilis e; 0.369, 0.690, pH+tmp+P-Source.Feed.conc.+Main.Thr.Conc.+ Nitrogen.Conc.+Lys_conc.+His+SO4.ferm.+PO4.ferm.+PO4.apport e+PO4.utilise; 0.381, 0.690, tmp+P-Source.Feed.conc.+Main.T hr.Conc.+OD+Lys_conc.+yield+PL+SO4.ferm.+PO4.ferm.+PO4.util ise; 0.369, 0.690, tmp+Main.P-Source.Conc.+P-Source.Feed.co nc.+Main.Thr.Conc.+OD+Lys_conc.+yield+PL+lactate+SO4.ferm.+ PO4.apporte; 0.381, 0.690, tmp+Main.P-Source.Conc.+P-Source. Feed.conc.+Main.Thr.Conc.+OD+Lys_conc.+His+SO4.ferm.+PO4.fe rm.+PO4.utilise; 0.392, 0.690, pH+tmp+P-Source.Feed.conc.+M ain.Thr.Conc.+Nitrogen.Conc.+Lys_conc.+lactate+SO4.ferm.+PO 4.utilise; 0.392, 0.690, tmp+P-Source.Feed.conc.+Main.Thr.C onc.+Nitrogen.Conc.+OD+Lys_conc.+PO4.ferm.+PO4.apporte+PO4. utilise; 0.369, 0.690, pH+tmp+Main.P-Source.Conc.+P-Source. Feed.conc.+Main.Thr.Conc.+Nitrogen.Conc.+Lys_conc.+yield+la ctate+SO4.ferm.+PO4.utilise; 0.369, 0.690, pH+tmp+P-Source. Feed.conc.+Main.Thr.Conc.+Nitrogen.Conc.+OD+Lys_conc.+yield +SO4.ferm.+PO4.ferm.+PO4.apporte; 0.381, 0.690, pH+tmp+P-So urce.Feed.conc.+Main.Thr.Conc.+Nitrogen.Conc.+OD+Lys_conc.+ yield+lactate+PO4.apporte; 0.369, 0.690, pH+tmp+P-Source.Fe ed.conc.+Main.Thr.Conc.+OD+Lys_conc.+yield+lactate+SO4.ferm. +PO4.ferm.+PO4.apporte; 0.380, 0.690, tmp+Main.P-Source.Con c.+P-Source.Feed.conc.+Main.Thr.Conc.+OD+Lys_conc.+yield+SO 4.ferm.+PO4.apporte+PO4.utilise; 0.432, 0.690, tmp+Main.P-S ource.Conc.+Main.Thr.Conc.+Lys_conc.+PO4.apporte; 0.380, 0. 690, tmp+Main.P-Source.Conc.+P-Source.Feed.conc.+Main.Thr.C onc.+OD+Lys_conc.+lactate+SO4.ferm.+PO4.ferm.+PO4.utilise; 0.402, 0.690, tmp+P-Source.Feed.conc.+Main.Thr.Conc.+Lys_co nc.+PL+lactate+SO4.ferm.+PO4.utilise; 0.402, 0.690, tmp+P-S ource.Feed.conc.+Main.Thr.Conc.+Lys_conc.+rab+lactate+SO4.f erm.+PO4.apporte; 0.380, 0.690, pH+tmp+Main.P-Source.Conc.+ P-Source.Feed.conc.+Main.Thr.Conc.+Nitrogen.Conc.+Lys-conc. +PL+lactate+SO4.ferm.; 0.380, 0.690, pH+tmp+P-Source.Feed.c onc.+Main.Thr.Conc.+Lys_conc.+His+SO4.ferm.+PO4.ferm.+PO4.a pporte+PO4.utilise; 0.412, 0.690, pH+tmp+P-Source.Feed.conc. +Main.Thr.Conc.+Lys_conc.+PO4.ferm.+PO4.apporte; 0.402, 0.6 90, tmp+P-Source.Feed.conc.+Main.Thr.Conc.+Nitrogen.Conc.+L ys_conc.+PL+SO4.ferm.+PO4.utilise; 0.391, 0.690, tmp+Main.P -Source.Conc.+P-Source.Feed.conc.+Main.Thr.Conc.+Nitrogen.C onc.+Lys-conc.+PL+His+SO4.ferm.; 0.412, 0.690, pH+tmp+P-Sou rce.Feed.conc.+Main.Thr.Conc.+Lys_conc.+Thr+PO4.apporte; 0. 380, 0.690, tmp+Main.P-Source.Conc.+P-Source.Feed.conc.+Mai n.Thr.Conc.+OD+Lys_conc.+yield+SO4.ferm.+PO4.ferm.+PO4.appo rte; 0.369, 0.690, pH+tmp+P-Source.Feed.conc.+Main.Thr.Conc. +Lys conc.+yield+PL+lactate+SO4.ferm.+PO4.apporte+PO4.utili se; 0.391, 0.690, tmp+P-Source.Feed.conc.+Main.Thr.Conc.+Ni trogen.Conc.+Lys_conc.+PL+PO4.ferm.+PO4.apporte+PO4.utilis e; 0.391, 0.690, tmp+Main.P-Source.Conc.+P-Source.Feed.conc. +Main.Thr.Conc.+Nitrogen.Conc.+OD+Lys_conc.+lactate+SO4.fer m.; 0.402, 0.690, tmp+P-Source.Feed.conc.+Main.Thr.Conc.+Ni trogen.Conc.+Lys_conc.+yield+PO4.ferm.+PO4.utilise; 0.369, 0.690, pH+tmp+P-Source.Feed.conc.+Main.Thr.Conc.+Lys_conc.+ yield+PL+lactate+SO4.ferm.+PO4.ferm.+PO4.utilise; 0.369, 0. 690, tmp+Main.P-Source.Conc.+P-Source.Feed.conc.+Main.Thr.C onc.+Nitrogen.Conc.+Lys_conc.+His+lactate+SO4.ferm.+PO4.fer m.+PO4.apporte; 0.369, 0.690, pH+tmp+P-Source.Feed.conc.+Ma in.Thr.Conc.+Lys_conc.+yield+PL+lactate+SO4.ferm.+PO4.ferm. +PO4.apporte; 0.369, 0.690, pH+tmp+Main.P-Source.Conc.+P-So urce.Feed.conc.+Main.Thr.Conc.+Lys_conc.+lactate+SO4.ferm.+ PO4.ferm.+PO4.apporte+PO4.utilise; 0.412, 0.690, tmp+P-Sour ce.Feed.conc.+Main.Thr.Conc.+OD+Lys_conc.+His+PO4.apporte; 0.391, 0.690, pH+tmp+P-Source.Feed.conc.+Main.Thr.Conc.+Nit rogen.Conc.+Lys_conc.+yield+SO4.ferm.+PO4.utilise; 0.391, 0. 690, tmp+Main.P-Source.Conc.+P-Source.Feed.conc.+Main.Thr.C onc.+Lys_conc.+yield+His+SO4.ferm.+PO4.apporte; 0.369, 0.69 0, pH+tmp+P-Source.Feed.conc.+Main.Thr.Conc.+OD+Lys_conc.+y ield+lactate+SO4.ferm.+PO4.ferm.+PO4.utilise; 0.357, 0.689, pH+tmp+Main.P-Source.Conc.+P-Source.Feed.conc.+Main.Thr.Co nc.+Nitrogen.Conc.+OD+Lys_conc.+PL+His+SO4.ferm.+PO4.apport e; 0.402, 0.689, pH+tmp+P-Source.Feed.conc.+Main.Thr.Conc.+ Lys_conc.+emission_O2+SO4.ferm.+PO4.apporte; 0.368, 0.689, tmp+P-Source.Feed.conc.+Main.Thr.Conc.+Lys_conc.+yield+PL+l actate+SO4.ferm.+PO4.ferm.+PO4.apporte+PO4.utilise; 0.368, 0.689, pH+tmp+P-Source.Feed.conc.+Main.Thr.Conc.+Nitrogen.C onc.+Lys_conc.+PL+His+SO4.ferm.+PO4.ferm.+PO4.utilise; 0.38 0, 0.689, tmp+Main.P-Source.Conc.+P-Source.Feed.conc.+Main. Thr.Conc.+OD+Lys conc.+yield+SO4.ferm.+PO4.ferm.+PO4.utilis e; 0.380, 0.689, pH+tmp+Main.P-Source.Conc.+P-Source.Feed.c onc.+Main.Thr.Conc.+Lys_conc.+His+lactate+SO4.ferm.+PO4.app orte; 0.368, 0.689, tmp+Main.P-Source.Conc.+P-Source.Feed.c onc.+Main.Thr.Conc.+Nitrogen.Conc.+Lys_conc.+His+lactate+SO 4.ferm.+PO4.apporte+PO4.utilise; 0.401, 0.689, pH+tmp+P-Sou rce.Feed.conc.+Main.Thr.Conc.+Lys_conc.+yield+PL+PO4.apport e; 0.368, 0.689, pH+tmp+P-Source.Feed.conc.+Main.Thr.Conc.+ Nitrogen.Conc.+OD+Lys_conc.+yield+SO4.ferm.+PO4.ferm.+PO4.u tilise; 0.391, 0.689, pH+tmp+P-Source.Feed.conc.+Main.Thr.C onc.+OD+Lys_conc.+PL+SO4.ferm.+PO4.utilise; 0.412, 0.689, t mp+Main.P-Source.Conc.+Main.Thr.Conc.+Lys_conc.+lactate+SO4. ferm.+PO4.apporte; 0.391, 0.689, tmp+P-Source.Feed.conc.+Ma in.Thr.Conc.+Nitrogen.Conc.+Lys_conc.+PL+rab+SO4.ferm.+PO4. apporte; 0.401, 0.689, tmp+Main.P-Source.Conc.+P-Source.Fee d.conc.+Main.Thr.Conc.+Lys conc.+His+SO4.ferm.+PO4.utilise; 0.368, 0.689, pH+tmp+P-Source.Feed.conc.+Main.Thr.Conc.+Ni trogen.Conc.+Lys_conc.+PL+His+SO4.ferm.+PO4.ferm.+PO4.appor te; 0.368, 0.689, pH+tmp+P-Source.Feed.conc.+Main.Thr.Conc. +OD+Lys_conc.+PL+His+lactate+SO4.ferm.+PO4.utilise; 0.401, 0.689, pH+tmp+Main.P-Source.Conc.+P-Source.Feed.conc.+Main. Thr.Conc.+Lys_conc.+yield+PO4.apporte; 0.368, 0.689, tmp+P-Source.Feed.conc.+Main.Thr.Conc.+Nitrogen.Conc.+OD+Lys_conc. +yield+lactate+SO4.ferm.+PO4.apporte+PO4.utilise; 0.390, 0. 689, pH+tmp+P-Source.Feed.conc.+Main.Thr.Conc.+Nitrogen.Con c.+OD+Lys_conc.+PL+PO4.apporte; 0.411, 0.689, tmp+P-Source. Feed.conc.+Main.Thr.Conc.+Lys_conc.+PL+SO4.ferm.+PO4.utilis e; 0.368, 0.689, tmp+Main.P-Source.Conc.+P-Source.Feed.conc. +Main.Thr.Conc.+Nitrogen.Conc.+OD+Lys_conc.+lactate+SO4.fer m.+PO4.ferm.+PO4.utilise; 0.356, 0.689, pH+tmp+Main.P-Sourc e.Conc.+P-Source.Feed.conc.+Main.Thr.Conc.+Nitrogen.Conc.+L ys_conc.+PL+lactate+SO4.ferm.+PO4.ferm.+PO4.apporte; 0.368, 0.689, pH+tmp+P-Source.Feed.conc.+Main.Thr.Conc.+OD+Lys_co nc.+lactate+SO4.ferm.+PO4.ferm.+PO4.apporte+PO4.utilise; 0. 368, 0.689, pH+tmp+P-Source.Feed.conc.+Main.Thr.Conc.+Nitro gen.Conc.+Lys_conc.+PL+His+SO4.ferm.+PO4.apporte+PO4.utilis e; 0.379, 0.689, pH+tmp+Main.P-Source.Conc.+P-Source.Feed.c onc.+Main.Thr.Conc.+Nitrogen.Conc.+OD+Lys conc.+lactate+SO4. ferm.; 0.356, 0.689, pH+tmp+Main.P-Source.Conc.+P-Source.Fe ed.conc.+Main.Thr.Conc.+Nitrogen.Conc.+Lys_conc.+PL+lactate +SO4.ferm.+PO4.apporte+PO4.utilise; 0.401, 0.689, tmp+P-Sou rce.Feed.conc.+Main.Thr.Conc.+Lys_conc.+emission_O2+rab+SO4. ferm.+PO4.apporte; 0.379, 0.689, pH+tmp+Main.P-Source.Conc. +P-Source.Feed.conc.+Main.Thr.Conc.+Nitrogen.Conc.+Lys_conc. +rab+SO4.ferm.+PO4.apporte; 0.390, 0.689, tmp+P-Source.Feed. conc.+Main.Thr.Conc.+Nitrogen.Conc.+Lys_conc.+emission_O2+l actate+SO4.ferm.+PO4.apporte; 0.368, 0.689, tmp+P-Source.Fe ed.conc.+Main.Thr.Conc.+Nitrogen.Conc.+OD+Lys_conc.+yield+l actate+SO4.ferm.+PO4.ferm.+PO4.apporte; 0.368, 0.689, tmp+M ain.P-Source.Conc.+P-Source.Feed.conc.+Main.Thr.Conc.+Nitro gen.Conc.+Lys_conc.+His+lactate+SO4.ferm.+PO4.ferm.+PO4.uti lise; 0.390, 0.689, tmp+Main.P-Source.Conc.+P-Source.Feed.c onc.+Main.Thr.Conc.+Lys_conc.+yield+lactate+SO4.ferm.+PO4.u tilise; 0.367, 0.689, pH+tmp+Main.P-Source.Conc.+P-Source.F eed.conc.+Main.Thr.Conc.+Nitrogen.Conc.+OD+Lys_conc.+His+SO 4.ferm.+PO4.utilise; 0.390, 0.689, pH+tmp+Main.P-Source.Con c.+P-Source.Feed.conc.+Main.Thr.Conc.+Lys_conc.+lactate+SO4. ferm.+PO4.utilise; 0.411, 0.689, tmp+P-Source.Feed.conc.+Ma in.Thr.Conc.+Lys_conc.+yield+PO4.apporte+PO4.utilise; 0.379, 0.689, tmp+Main.P-Source.Conc.+P-Source.Feed.conc.+Main.Th r.Conc.+Lys_conc.+PL+His+SO4.ferm.+PO4.ferm.+PO4.utilise; 0. 411, 0.689, tmp+Main.P-Source.Conc.+Main.Thr.Conc.+Lys_conc. +yield+SO4.ferm.+PO4.apporte; 0.401, 0.689, tmp+Main.P-Sour ce.Conc.+P-Source.Feed.conc.+Main.Thr.Conc.+Nitrogen.Conc.+ Lys_conc.+PO4.apporte+PO4.utilise; 0.390, 0.689, pH+tmp+P-S ource.Feed.conc.+Main.Thr.Conc.+Nitrogen.Conc.+Lys_conc.+PL +His+PO4.apporte; 0.401, 0.689, pH+tmp+Main.P-Source.Conc.+ P-Source.Feed.conc.+Main.Thr.Conc.+OD+Lys conc.+PO4.apport e; 0.401, 0.689, tmp+Main.P-Source.Conc.+P-Source.Feed.conc. +Main.Thr.Conc.+Lys_conc.+rab+SO4.ferm.+PO4.apporte; 0.390, 0.689, tmp+Main.P-Source.Conc.+P-Source.Feed.conc.+Main.Th r.Conc.+Nitrogen.Conc.+Lys_conc.+lactate+SO4.ferm.+PO4.fer m.; 0.390, 0.689, pH+tmp+P-Source.Feed.conc.+Main.Thr.Conc. +Nitrogen.Conc.+Lys_conc.+yield+PO4.apporte+PO4.utilise; 0. 400, 0.689, tmp+P-Source.Feed.conc.+Main.Thr.Conc.+Lys_conc. +PL+rab+SO4.ferm.+PO4.apporte; 0.400, 0.689, tmp+P-Source.F eed.conc.+Main.Thr.Conc.+Nitrogen.Conc.+Lys_conc.+lactate+P O4.ferm.+PO4.utilise; 0.379, 0.689, tmp+Main.P-Source.Conc. +P-Source.Feed.conc.+Main.Thr.Conc.+Lys_conc.+PL+His+SO4.fe rm.+PO4.ferm.+PO4.apporte; 0.379, 0.689, tmp+Main.P-Source. Conc.+P-Source.Feed.conc.+Main.Thr.Conc.+OD+Lys_conc.+SO4.f erm.+PO4.ferm.+PO4.apporte+PO4.utilise; 0.400, 0.689, tmp+P -Source.Feed.conc.+Main.Thr.Conc.+Lys_conc.+emission_O2+lac tate+SO4.ferm.+PO4.apporte; 0.411, 0.689, tmp+P-Source.Feed. conc.+Main.Thr.Conc.+Lys conc.+yield+PO4.ferm.+PO4.apporte; 0.400, 0.689, pH+tmp+P-Source.Feed.conc.+Main.Thr.Conc.+Ly s_conc.+PO4.ferm.+PO4.apporte+PO4.utilise; 0.367, 0.689, pH +tmp+Main.P-Source.Conc.+P-Source.Feed.conc.+Main.Thr.Conc. +Nitrogen.Conc.+Lys_conc.+lactate+PO4.ferm.+PO4.apporte+PO4. utilise; 0.400, 0.689, tmp+Main.P-Source.Conc.+P-Source.Fee d.conc.+Main.Thr.Conc.+Lys_conc.+yield+lactate+PO4.apporte; 0.367, 0.689, pH+tmp+P-Source.Feed.conc.+Main.Thr.Conc.+OD +Lys_conc.+yield+SO4.ferm.+PO4.ferm.+PO4.apporte+PO4.utilis e; 0.378, 0.688, tmp+Main.P-Source.Conc.+P-Source.Feed.conc. +Main.Thr.Conc.+Lys_conc.+PL+His+SO4.ferm.+PO4.apporte+PO4. utilise; 0.378, 0.688, pH+tmp+Main.P-Source.Conc.+P-Source. Feed.conc.+Main.Thr.Conc.+Nitrogen.Conc.+Lys_conc.+yield+la ctate+PO4.apporte; 0.378, 0.688, pH+tmp+Main.P-Source.Conc. +P-Source.Feed.conc.+Main.Thr.Conc.+OD+Lys_conc.+yield+SO4. ferm.+PO4.utilise; 0.355, 0.688, pH+tmp+Main.P-Source.Conc. +P-Source.Feed.conc.+Main.Thr.Conc.+Nitrogen.Conc.+OD+Lys_c onc.+yield+PL+SO4.ferm.+PO4.apporte; 0.400, 0.688, tmp+Main. P-Source.Conc.+P-Source.Feed.conc.+Main.Thr.Conc.+Nitrogen. Conc.+Lys_conc.+PO4.ferm.+PO4.apporte; 0.367, 0.688, tmp+P-Source.Feed.conc.+Main.Thr.Conc.+Nitrogen.Conc.+OD+Lys_conc. +yield+SO4.ferm.+PO4.ferm.+PO4.apporte+PO4.utilise; 0.367, 0.688, tmp+P-Source.Feed.conc.+Main.Thr.Conc.+Nitrogen.Conc. +OD+Lys_conc.+yield+lactate+SO4.ferm.+PO4.ferm.+PO4.utilis e; 0.367, 0.688, tmp+P-Source.Feed.conc.+Main.Thr.Conc.+Nit rogen.Conc.+OD+Lys_conc.+PL+His+lactate+SO4.ferm.+PO4.appor te; 0.400, 0.688, tmp+P-Source.Feed.conc.+Main.Thr.Conc.+Ly s_conc.+yield+PL+lactate+PO4.apporte; 0.410, 0.688, tmp+Mai n.P-Source.Conc.+Main.Thr.Conc.+Lys_conc.+PL+SO4.ferm.+PO4. apporte; 0.389, 0.688, pH+tmp+P-Source.Feed.conc.+Main.Thr. Conc.+Nitrogen.Conc.+Lys_conc.+yield+PO4.ferm.+PO4.apporte; 0.410, 0.688, pH+tmp+Main.Thr.Conc.+Nitrogen.Conc.+Lys_con c.+SO4.ferm.+PO4.apporte; 0.378, 0.688, tmp+Main.P-Source.C onc.+P-Source.Feed.conc.+Main.Thr.Conc.+Nitrogen.Conc.+Lys_ conc.+yield+PL+lactate+SO4.ferm.; 0.366, 0.688, tmp+Main.P-Source.Conc.+P-Source.Feed.conc.+Main.Thr.Conc.+Nitrogen.Co nc.+Lys_conc.+His+SO4.ferm.+PO4.ferm.+PO4.apporte+PO4.utili se; 0.378, 0.688, pH+tmp+Main.P-Source.Conc.+P-Source.Feed. conc.+Main.Thr.Conc.+Nitrogen.Conc.+Lys_conc.+yield+SO4.fer m.+PO4.ferm.; 0.389, 0.688, tmp+P-Source.Feed.conc.+Main.Th r.Conc.+Nitrogen.Conc.+OD+Lys_conc.+PL+SO4.ferm.+PO4.utilis e; 0.389, 0.688, tmp+P-Source.Feed.conc.+Main.Thr.Conc.+Nit rogen.Conc.+Lys_conc.+yield+rab+SO4.ferm.+PO4.apporte; 0.38 9, 0.688, pH+tmp+Main.P-Source.Conc.+P-Source.Feed.conc.+Ma in.Thr.Conc.+OD+Lys_conc.+yield+PO4.apporte; 0.378, 0.688, tmp+Main.P-Source.Conc.+P-Source.Feed.conc.+Main.Thr.Conc.+ OD+Lys_conc.+PL+His+SO4.ferm.+PO4.utilise; 0.378, 0.688, tm p+P-Source.Feed.conc.+Main.Thr.Conc.+Nitrogen.Conc.+OD+Lys_ conc.+yield+PL+SO4.ferm.+PO4.utilise; 0.378, 0.688, tmp+Mai n.P-Source.Conc.+P-Source.Feed.conc.+Main.Thr.Conc.+OD+Lys_ conc.+yield+His+SO4.ferm.+PO4.apporte; 0.354, 0.688, pH+tmp +Main.P-Source.Conc.+P-Source.Feed.conc.+Main.Thr.Conc.+Nit rogen.Conc.+Lys_conc.+PL+lactate+SO4.ferm.+PO4.ferm.+PO4.ut ilise; 0.378, 0.688, pH+tmp+Main.P-Source.Conc.+P-Source.Fe ed.conc.+Main.Thr.Conc.+Nitrogen.Conc.+Lys_conc.+lactate+SO 4.ferm.+PO4.ferm.; 0.399, 0.688, pH+tmp+P-Source.Feed.conc. +Main.Thr.Conc.+Lys_conc.+PL+lactate+PO4.apporte; 0.366, 0. 688, pH+tmp+P-Source.Feed.conc.+Main.Thr.Conc.+Nitrogen.Con c.+OD+Lys_conc.+PL+His+SO4.ferm.+PO4.utilise; 0.399, 0.688, pH+tmp+Main.P-Source.Conc.+P-Source.Feed.conc.+Main.Thr.Co nc.+Lys_conc.+lactate+PO4.apporte; 0.366, 0.688, pH+tmp+P-S ource.Feed.conc.+Main.Thr.Conc.+Nitrogen.Conc.+Lys_conc.+Hi s+lactate+SO4.ferm.+PO4.ferm.+PO4.utilise; 0.366, 0.688, pH +tmp+Main.P-Source.Conc.+P-Source.Feed.conc.+Main.Thr.Conc. +Lys_conc.+PL+lactate+SO4.ferm.+PO4.apporte+PO4.utilise; 0. 389, 0.688, pH+tmp+Main.P-Source.Conc.+P-Source.Feed.conc.+ Main.Thr.Conc.+OD+Lys-conc.+SO4.ferm.+PO4.utilise; 0.366, 0. 688, pH+tmp+Main.P-Source.Conc.+P-Source.Feed.conc.+Main.Th r.Conc.+Lys_conc.+PL+lactate+SO4.ferm.+PO4.ferm.+PO4.apport e; 0.366, 0.688, pH+tmp+P-Source.Feed.conc.+Main.Thr.Conc.+ Nitrogen.Conc.+OD+Lys_conc.+PL+SO4.ferm.+PO4.apporte+PO4.ut ilise; 0.410, 0.688, pH+tmp+Main.P-Source.Conc.+Main.Thr.Co nc.+Lys_conc.+SO4.ferm.+PO4.apporte; 0.439, 0.688, tmp+Main. Thr.Conc.+Lys_conc.+PO4.apporte; 0.366, 0.688, pH+tmp+P-Sou rce.Feed.conc.+Main.Thr.Conc.+Nitrogen.Conc.+Lys_conc.+yiel d+His+lactate+SO4.ferm.+PO4.apporte; 0.366, 0.688, pH+tmp+P -Source.Feed.conc.+Main.Thr.Conc.+Nitrogen.Conc.+Lys_conc.+ His+lactate+SO4.ferm.+PO4.ferm.+PO4.apporte; 0.354, 0.688, tmp+Main.P-Source.Conc.+P-Source.Feed.conc.+Main.Thr.Conc.+ Nitrogen.Conc.+OD+Lys_conc.+lactate+SO4.ferm.+PO4.ferm.+PO4. apporte+PO4.utilise; 0.399, 0.688, tmp+P-Source.Feed.conc.+ Main.Thr.Conc.+Lys_conc.+yield+PL+SO4.ferm.+PO4.utilise; 0. 366, 0.688, pH+tmp+P-Source.Feed.conc.+Main.Thr.Conc.+Nitro gen.Conc.+Lys_conc.+His+lactate+SO4.ferm.+PO4.apporte+PO4.u tilise; 0.388, 0.688, pH+tmp+P-Source.Feed.conc.+Main.Thr.C onc.+Nitrogen.Conc.+Lys_conc.+lactate+PO4.ferm.+PO4.utilis e; 0.388, 0.688, tmp+P-Source.Feed.conc.+Main.Thr.Conc.+Nit rogen.Conc.+Lys_conc.+PL+emission_O2+SO4.ferm.+PO4.apporte; 0.377, 0.688, pH+tmp+P-Source.Feed.conc.+Main.Thr.Conc.+Ni trogen.Conc.+OD+Lys_conc.+lactate+SO4.ferm.+PO4.utilise; 0. 429, 0.688, tmp+Main.Thr.Conc.+Nitrogen.Conc.+Lys_conc.+PO4. apporte; 0.388, 0.688, tmp+Main.P-Source.Conc.+P-Source.Fee d.conc.+Main.Thr.Conc.+OD+Lys_conc.+lactate+SO4.ferm.+PO4.u tilise; 0.366, 0.688, pH+tmp+P-Source.Feed.conc.+Main.Thr.C onc.+Nitrogen.Conc.+Lys_conc.+yield+PL+His+SO4.ferm.+PO4.ap porte; 0.366, 0.688, tmp+Main.P-Source.Conc.+P-Source.Feed. conc.+Main.Thr.Conc.+Nitrogen.Conc.+OD+Lys_conc.+yield+His+ SO4.ferm.+PO4.apporte; 0.377, 0.688, tmp+Main.P-Source.Conc. +P-Source.Feed.conc.+Main.Thr.Conc.+Nitrogen.Conc.+Lys_conc. +yield+PL+SO4.ferm.+PO4.utilise; 0.377, 0.688, tmp+P-Source. Feed.conc.+Main.Thr.Conc.+Nitrogen.Conc.+Lys_conc.+yield+Hi s+SO4.ferm.+PO4.ferm.+PO4.apporte; 0.366, 0.688, pH+tmp+P-S ource.Feed.conc.+Main.Thr.Conc.+OD+Lys_conc.+PL+His+lactate +SO4.ferm.+PO4.apporte; 0.409, 0.688, tmp+Main.Thr.Conc.+Ni trogen.Conc.+Lys_conc.+yield+SO4.ferm.+PO4.apporte; 0.399, 0.688, tmp+Main.P-Source.Conc.+P-Source.Feed.conc.+Main.Thr. Conc.+OD+Lys_conc.+yield+SO4.ferm.; 0.377, 0.688, tmp+Main. P-Source.Conc.+P-Source.Feed.conc.+Main.Thr.Conc.+Nitrogen. Conc.+Lys_conc.+His+lactate+SO4.ferm.+PO4.utilise; 0.399, 0. 688, pH+tmp+P-Source.Feed.conc.+Main.Thr.Conc.+OD+Lys_conc. +yield+PO4.utilise; 0.377, 0.688, pH+tmp+Main.P-Source.Conc. +P-Source.Feed.conc.+Main.Thr.Conc.+Nitrogen.Conc.+OD+Lys_c onc.+His+SO4.ferm.; 0.388, 0.688, tmp+P-Source.Feed.conc.+M ain.Thr.Conc.+Nitrogen.Conc.+Lys_conc.+yield+emission_O2+SO 4.ferm.+PO4.apporte; 0.377, 0.688, tmp+P-Source.Feed.conc.+ Main.Thr.Conc.+Nitrogen.Conc.+Lys_conc.+yield+His+SO4.ferm. +PO4.apporte+PO4.utilise; 0.409, 0.688, tmp+P-Source.Feed.c onc.+Main.Thr.Conc.+OD+Lys_conc.+PL+PO4.apporte; 0.388, 0.6 88, pH+tmp+P-Source.Feed.conc.+Main.Thr.Conc.+Nitrogen.Conc. +Lys_conc.+His+SO4.ferm.+PO4.utilise; 0.341, 0.688, pH+tmp+ Main.P-Source.Conc.+P-Source.Feed.conc.+Main.Thr.Conc.+Nitr ogen.Conc.+Lys_conc.+yield+lactate+SO4.ferm.+PO4.ferm.+PO4. apporte+PO4.utilise; 0.388, 0.688, pH+tmp+P-Source.Feed.con c.+Main.Thr.Conc.+Lys_conc.+yield+lactate+SO4.ferm.+PO4.uti lise; 0.365, 0.688, pH+tmp+P-Source.Feed.conc.+Main.Thr.Con c.+Nitrogen.Conc.+OD+Lys_conc.+His+lactate+SO4.ferm.+PO4.ut ilise; 0.365, 0.688, tmp+P-Source.Feed.conc.+Main.Thr.Conc. +Nitrogen.Conc.+OD+Lys_conc.+PL+SO4.ferm.+PO4.ferm.+PO4.app orte+PO4.utilise; 0.365, 0.688, pH+tmp+P-Source.Feed.conc.+ Main.Thr.Conc.+Nitrogen.Conc.+OD+Lys_conc.+PL+SO4.ferm.+PO4. ferm.+PO4.apporte; 0.377, 0.688, tmp+Main.P-Source.Conc.+P-Source.Feed.conc.+Main.Thr.Conc.+Nitrogen.Conc.+OD+Lys_conc. +lactate+SO4.ferm.+PO4.utilise; 0.388, 0.688, tmp+Main.P-So urce.Conc.+P-Source.Feed.conc.+Main.Thr.Conc.+Nitrogen.Conc. +OD+Lys_conc.+yield+PO4.apporte; 0.365, 0.688, pH+tmp+Main. P-Source.Conc.+P-Source.Feed.conc.+Main.Thr.Conc.+Lys-conc. +PL+lactate+SO4.ferm.+PO4.ferm.+PO4.utilise; 0.419, 0.688, tmp+Main.Thr.Conc.+Lys_conc.+SO4.ferm.+PO4.ferm.+PO4.utilis e; 0.353, 0.688, tmp+Main.P-Source.Conc.+P-Source.Feed.conc. +Main.Thr.Conc.+Nitrogen.Conc.+OD+Lys_conc.+yield+PL+lactat e+SO4.ferm.+PO4.apporte; 0.353, 0.687, pH+tmp+P-Source.Feed. conc.+Main.Thr.Conc.+Nitrogen.Conc.+Lys_conc.+PL+lactate+SO 4.ferm.+PO4.ferm.+PO4.apporte+PO4.utilise; 0.388, 0.687, tm p+P-Source.Feed.conc.+Main.Thr.Conc.+Nitrogen.Conc.+Lys_con c.+PL+lactate+SO4.ferm.+PO4.utilise; 0.388, 0.687, tmp+P-So urce.Feed.conc.+Main.Thr.Conc.+Lys_conc.+yield+His+lactate+ SO4.ferm.+PO4.apporte; 0.377, 0.687, tmp+Main.P-Source.Conc. +P-Source.Feed.conc.+Main.Thr.Conc.+Lys_conc.+PL+His+lactat e+SO4.ferm.+PO4.apporte; 0.377, 0.687, tmp+P-Source.Feed.co nc.+Main.Thr.Conc.+Nitrogen.Conc.+Lys_conc.+yield+His+SO4.f erm.+PO4.ferm.+PO4.utilise; 0.388, 0.687, pH+tmp+Main.P-Sou rce.Conc.+P-Source.Feed.conc.+Main.Thr.Conc.+Nitrogen.Conc. +Lys_conc.+PO4.apporte+PO4.utilise; 0.409, 0.687, tmp+Main. P-Source.Conc.+P-Source.Feed.conc.+Main.Thr.Conc.+Lys_conc. +PL+PO4.apporte; 0.399, 0.687, tmp+P-Source.Feed.conc.+Main. Thr.Conc.+Lys_conc.+PL+emission_O2+SO4.ferm.+PO4.apporte; 0. 377, 0.687, pH+tmp+P-Source.Feed.conc.+Main.Thr.Conc.+Lys_c onc.+His+lactate+SO4.ferm.+PO4.ferm.+PO4.utilise; 0.388, 0. 687, pH+tmp+P-Source.Feed.conc.+Main.Thr.Conc.+OD+Lys_conc. +yield+PL+PO4.apporte; 0.365, 0.687, pH+tmp+P-Source.Feed.c onc.+Main.Thr.Conc.+OD+Lys_conc.+His+SO4.ferm.+PO4.ferm.+PO 4.apporte+PO4.utilise; 0.419, 0.687, tmp+P-Source.Feed.conc. +Main.Thr.Conc.+Lys_conc.+PO4.ferm.+PO4.utilise; 0.409, 0.6 87, pH+tmp+P-Source.Feed.conc.+Main.Thr.Conc.+Lys_conc.+PO4. ferm.+PO4.utilise; 0.409, 0.687, tmp+P-Source.Feed.conc.+Ma in.Thr.Conc.+Lys_conc.+yield+PO4.ferm.+PO4.utilise; 0.377, 0.687, tmp+Main.P-Source.Conc.+P-Source.Feed.conc.+Main.Thr. Conc.+Nitrogen.Conc.+OD+Lys-conc.+yield+SO4.ferm.+PO4.utili se; 0.409, 0.687, tmp+Main.P-Source.Conc.+Main.Thr.Conc.+Ni trogen.Conc.+Lys_conc.+SO4.ferm.+PO4.apporte; 0.388, 0.687, tmp+P-Source.Feed.conc.+Main.Thr.Conc.+Nitrogen.Conc.+Lys_ conc.+rab+SO4.ferm.+PO4.ferm.+PO4.apporte; 0.365, 0.687, tm p+P-Source.Feed.conc.+Main.Thr.Conc.+OD+Lys_conc.+yield+lac tate+SO4.ferm.+PO4.ferm.+PO4.apporte+PO4.utilise; 0.376, 0. 687, pH+tmp+P-Source.Feed.conc.+Main.Thr.Conc.+Nitrogen.Con c.+Lys_conc.+yield+PO4.ferm.+PO4.apporte+PO4.utilise; 0.388, 0.687, tmp+Main.P-Source.Conc.+P-Source.Feed.conc.+Main.Th r.Conc.+Nitrogen.Conc.+Lys_conc.+yield+PL+PO4.apporte; 0.39 8, 0.687, pH+tmp+P-Source.Feed.conc.+Main.Thr.Conc.+OD+Lys_ conc.+PL+PO4.apporte; 0.365, 0.687, pH+tmp+P-Source.Feed.co nc.+Main.Thr.Conc.+Lys_conc.+yield+PL+SO4.ferm.+PO4.ferm.+P O4.apporte+PO4.utilise; 0.365, 0.687, tmp+P-Source.Feed.con c.+Main.Thr.Conc.+Nitrogen.Conc.+OD+Lys_conc.+yield+PL+His+ SO4.ferm.+PO4.apporte; 0.387, 0.687, tmp+Main.P-Source.Conc. +P-Source.Feed.conc.+Main.Thr.Conc.+Nitrogen.Conc.+OD+Lys_c onc.+His+SO4.ferm.; 0.387, 0.687, tmp+P-Source.Feed.conc.+M ain.Thr.Conc.+Nitrogen.Conc.+Lys_conc.+yield+lactate+PO4.ap porte+PO4.utilise; 0.376, 0.687, tmp+Main.P-Source.Conc.+P-Source.Feed.conc.+Main.Thr.Conc.+OD+Lys_conc.+yield+lactate +SO4.ferm.+PO4.utilise; 0.387, 0.687, tmp+P-Source.Feed.con c.+Main.Thr.Conc.+Nitrogen.Conc.+Lys_conc.+rab+SO4.ferm.+PO 4.apporte+PO4.utilise; 0.376, 0.687, pH+tmp+Main.P-Source.C onc.+P-Source.Feed.conc.+Main.Thr.Conc.+Nitrogen.Conc.+Lys_ conc.+emission_O2+SO4.ferm.+PO4.apporte; 0.376, 0.687, pH+t mp+P-Source.Feed.conc.+Main.Thr.Conc.+Nitrogen.Conc.+Lys_co nc.+emission_O2+rab+SO4.ferm.+PO4.apporte; 0.376, 0.687, tm p+P-Source.Feed.conc.+Main.Thr.Conc.+Lys_conc.+PL+His+SO4.f erm.+PO4.ferm.+PO4.apporte+PO4.utilise; 0.376, 0.687, tmp+M ain.P-Source.Conc.+P-Source.Feed.conc.+Main.Thr.Conc.+OD+Ly s_conc.+His+lactate+SO4.ferm.+PO4.apporte; 0.376, 0.687, tm p+P-Source.Feed.conc.+Main.Thr.Conc.+OD+Lys_conc.+yield+His +lactate+SO4.ferm.+PO4.utilise; 0.365, 0.687, tmp+Main.P-So urce.Conc.+P-Source.Feed.conc.+Main.Thr.Conc.+Lys_conc.+yie ld+PL+lactate+SO4.ferm.+PO4.apporte+PO4.utilise; 0.408, 0.6 87, tmp+Main.Thr.Conc.+Nitrogen.Conc.+Lys_conc.+PL+SO4.ferm. +PO4.apporte; 0.365, 0.687, tmp+Main.P-Source.Conc.+P-Sourc e.Feed.conc.+Main.Thr.Conc.+Lys_conc.+yield+lactate+SO4.fer m.+PO4.ferm.+PO4.apporte+PO4.utilise; 0.365, 0.687, tmp+Mai n.P-Source.Conc.+P-Source.Feed.conc.+Main.Thr.Conc.+Lys_con c.+yield+PL+lactate+SO4.ferm.+PO4.ferm.+PO4.apporte; 0.365, 0.687, pH+tmp+P-Source.Feed.conc.+Main.Thr.Conc.+OD+Lys_co nc.+PL+lactate+SO4.ferm.+PO4.apporte+PO4.utilise; 0.353, 0. 687, tmp+Main.P-Source.Conc.+P-Source.Feed.conc.+Main.Thr.C onc.+Nitrogen.Conc.+Lys_conc.+yield+PL+lactate+SO4.ferm.+PO 4.ferm.+PO4.apporte; 0.418, 0.687, tmp+Main.Thr.Conc.+Lys_c onc.+SO4.ferm.+PO4.apporte+PO4.utilise; 0.387, 0.687, pH+tm p+Main.P-Source.Conc.+P-Source.Feed.conc.+Main.Thr.Conc.+Ni trogen.Conc.+Lys_conc.+PO4.ferm.+PO4.apporte; 0.352, 0.687, tmp+Main.P-Source.Conc.+P-Source.Feed.conc.+Main.Thr.Conc. +Nitrogen.Conc.+Lysconc.+yield+PL+lactate+SO4.ferm.+PO4.ap porte+PO4.utilise; 0.364, 0.687, pH+tmp+P-Source.Feed.conc. +Main.Thr.Conc.+OD+Lys_conc.+PL+His+SO4.ferm.+PO4.apporte+P O4.utilise; 0.408, 0.687, tmp+Main.Thr.Conc.+Nitrogen.Conc. +Lys_conc.+lactate+SO4.ferm.+PO4.apporte; 0.408, 0.687, tmp +Main.Thr.Conc.+Lys_conc.+PL+lactate+SO4.ferm.+PO4.apporte; 0.418, 0.687, tmp+Main.Thr.Conc.+Lys_conc.+SO4.ferm.+PO4.f erm.+PO4.apporte; 0.364, 0.687, pH+tmp+P-Source.Feed.conc.+ Main.Thr.Conc.+OD+Lys_conc.+PL+His+SO4.ferm.+PO4.ferm.+PO4. apporte; 0.376, 0.687, pH+tmp+P-Source.Feed.conc.+Main.Thr. Conc.+Nitrogen.Conc.+OD+Lys_conc.+PO4.ferm.+PO4.apporte+PO4. utilise; 0.408, 0.687, tmp+Main.P-Source.Conc.+P-Source.Fee d.conc.+Main.Thr.Conc.+Lys conc.+yield+SO4.ferm.; 0.376, 0. 687, pH+tmp+Main.P-Source.Conc.+P-Source.Feed.conc.+Main.Th r.Conc.+Nitrogen.Conc.+Lys_conc.+PL+His+SO4.ferm.; 0.364, 0. 687, pH+tmp+P-Source.Feed.conc.+Main.Thr.Conc.+OD+Lys_conc. +PL+His+SO4.ferm.+PO4.ferm.+PO4.utilise; 0.364, 0.687, tmp+ Main.P-Source.Conc.+P-Source.Feed.conc.+Main.Thr.Conc.+Lys_ conc.+yield+PL+lactate+SO4.ferm.+PO4.ferm.+PO4.utilise; 0.3 98, 0.687, tmp+P-Source.Feed.conc.+Main.Thr.Conc.+Lys_conc. +yield+rab+SO4.ferm.+PO4.apporte; 0.376, 0.687, pH+tmp+P-So urce.Feed.conc.+Main.Thr.Conc.+OD+Lys conc.+PL+lactate+SO4. ferm.+PO4.utilise; 0.398, 0.687, tmp+P-Source.Feed.conc.+Ma in.Thr.Conc.+Lys_conc.+lactate+PO4.ferm.+PO4.apporte+PO4.ut ilise; 0.364, 0.687, pH+tmp+P-Source.Feed.conc.+Main.Thr.Co nc.+Nitrogen.Conc.+OD+Lys_conc.+PL+SO4.ferm.+PO4.ferm.+PO4. utilise; 0.387, 0.687, tmp+P-Source.Feed.conc.+Main.Thr.Con c.+Nitrogen.Conc.+Lys_conc.+yield+lactate+PO4.ferm.+PO4.app orte; 0.398, 0.687, tmp+Main.P-Source.Conc.+P-Source.Feed.c onc.+Main.Thr.Conc.+Lys_conc.+emission_O2+SO4.ferm.+PO4.app orte; 0.387, 0.687, tmp+P-Source.Feed.conc.+Main.Thr.Conc.+ Nitrogen.Conc.+OD+Lys_conc.+yield+PL+PO4.apporte; 0.398, 0. 687, tmp+P-Source.Feed.conc.+Main.Thr.Conc.+Nitrogen.Conc.+ Lys_conc.+His+lactate+PO4.apporte; 0.398, 0.687, tmp+P-Sour ce.Feed.conc.+Main.Thr.Conc.+Lys_conc.+yield+emission_O2+SO 4.ferm.+PO4.apporte; 0.376, 0.687, tmp+P-Source.Feed.conc.+ Main.Thr.Conc.+Nitrogen.Conc.+Lys_conc.+yield+lactate+PO4.f erm.+PO4.apporte+PO4.utilise; 0.352, 0.687, pH+tmp+Main.P-S ource.Conc.+P-Source.Feed.conc.+Main.Thr.Conc.+Nitrogen.Con c.+Lys_conc.+PL+His+lactate+SO4.ferm.+PO4.apporte; 0.376, 0. 687, pH+tmp+P-Source.Feed.conc.+Main.Thr.Conc.+Lys_conc.+Hi s+lactate+SO4.ferm.+PO4.apporte+PO4.utilise; 0.352, 0.687, pH+tmp+Main.P-Source.Conc.+P-Source.Feed.conc.+Main.Thr.Con c.+Nitrogen.Conc.+OD+Lys_conc.+lactate+SO4.ferm.+PO4.apport e+PO4.utilise; 0.364, 0.687, tmp+P-Source.Feed.conc.+Main.T hr.Conc.+Nitrogen.Conc.+OD+Lys_conc.+PL+lactate+SO4.ferm.+P O4.apporte+PO4.utilise; 0.387, 0.687, tmp+Main.P-Source.Con c.+P-Source.Feed.conc.+Main.Thr.Conc.+Nitrogen.Conc.+OD+Lys _conc.+PL+SO4.ferm.; 0.398, 0.687, pH+tmp+P-Source.Feed.con c.+Main.Thr.Conc.+OD+Lys_conc.+Thr+PO4.apporte; 0.364, 0.68 7, pH+tmp+P-Source.Feed.conc.+Main.Thr.Conc.+OD+Lys_conc.+P L+lactate+SO4.ferm.+PO4.ferm.+PO4.apporte; 0.387, 0.687, pH +tmp+P-Source.Feed.conc.+Main.Thr.Conc.+Nitrogen.Conc.+Lys_ conc.+yield+PO4.ferm.+PO4.utilise; 0.364, 0.687, tmp+Main.P -Source.Conc.+P-Source.Feed.conc.+Main.Thr.Conc.+Nitrogen.C onc.+Lys_conc.+yield+PL+His+SO4.ferm.+PO4.apporte; 0.352, 0. 687, pH+tmp+Main.P-Source.Conc.+P-Source.Feed.conc.+Main.Th r.Conc.+Nitrogen.Conc.+OD+Lys conc.+lactate+SO4.ferm.+PO4.f erm.+PO4.apporte; 0.376, 0.687, pH+tmp+P-Source.Feed.conc.+ Main.Thr.Conc.+Nitrogen.Conc.+Lys_conc.+yield+PL+lactate+PO 4.apporte; 0.397, 0.687, tmp+P-Source.Feed.conc.+Main.Thr.C onc.+Nitrogen.Conc.+Lys_conc.+PL+PO4.apporte+PO4.utilise; 0. 418, 0.687, tmp+Main.Thr.Conc.+Lys_conc.+His+SO4.ferm.+PO4. apporte; 0.408, 0.687, tmp+Main.Thr.Conc.+Nitrogen.Conc.+OD +Lys_conc.+SO4.ferm.+PO4.apporte; 0.376, 0.687, pH+tmp+P-So urce.Feed.conc.+Main.Thr.Conc.+Lys_conc.+His+lactate+SO4.fe rm.+PO4.ferm.+PO4.apporte; 0.387, 0.687, pH+tmp+Main.P-Sour ce.Conc.+P-Source.Feed.conc.+Main.Thr.Conc.+Lys_conc.+yield +SO4.ferm.+PO4.utilise; 0.387, 0.687, tmp+P-Source.Feed.con c.+Main.Thr.Conc.+Nitrogen.Conc.+OD+Lys_conc.+rab+SO4.ferm. +PO4.apporte; 0.387, 0.687, tmp+P-Source.Feed.conc.+Main.Th r.Conc.+Nitrogen.Conc.+Lys_conc.+yield+PL+SO4.ferm.+PO4.uti lise; 0.352, 0.687, pH+tmp+Main.P-Source.Conc.+P-Source.Fee d.conc.+Main.Thr.Conc.+Nitrogen.Conc.+Lys_conc.+yield+PL+SO 4.ferm.+PO4.ferm.+PO4.apporte; 0.352, 0.687, pH+tmp+Main.P-Source.Conc.+P-Source.Feed.conc.+Main.Thr.Conc.+Nitrogen.Co nc.+Lys_conc.+yield+PL+SO4.ferm.+PO4.apporte+PO4.utilise; 0. 408, 0.687, tmp+P-Source.Feed.conc.+Main.Thr.Conc.+Lys_conc. +lactate+PO4.apporte+PO4.utilise; 0.375, 0.687, tmp+Main.P-Source.Conc.+P-Source.Feed.conc.+Main.Thr.Conc.+Nitrogen.Co nc.+OD+Lys_conc.+yield+lactate+SO4.ferm.; 0.364, 0.687, pH+ tmp+P-Source.Feed.conc.+Main.Thr.Conc.+Nitrogen.Conc.+OD+Ly s_conc.+yield+lactate+SO4.ferm.+PO4.utilise; 0.364, 0.687, pH+tmp+Main.P-Source.Conc.+P-Source.Feed.conc.+Main.Thr.Con c.+OD+Lys-conc.+His+SO4.ferm.+PO4.ferm.+PO4.apporte; 0.352, 0.687, pH+tmp+P-Source.Feed.conc.+Main.Thr.Conc.+Nitrogen. Conc.+OD+Lys conc.+lactate+SO4.ferm.+PO4.ferm.+PO4.apporte+ PO4.utilise; 0.364, 0.687, tmp+Main.P-Source.Conc.+P-Source. Feed.conc.+Main.Thr.Conc.+Nitrogen.Conc.+OD+Lys_conc.+yield +SO4.ferm.+PO4.ferm.+PO4.apporte; 0.352, 0.687, pH+tmp+P-So urce.Feed.conc.+Main.Thr.Conc.+Nitrogen.Conc.+Lys_conc.+yie ld+PL+lactate+SO4.ferm.+PO4.apporte+PO4.utilise; 0.364, 0.6 87, pH+tmp+Main.P-Source.Conc.+P-Source.Feed.conc.+Main.Thr. Conc.+OD+Lys conc.+lactate+SO4.ferm.+PO4.apporte+PO4.utilis e; 0.386, 0.687, tmp+Main.P-Source.Conc.+P-Source.Feed.conc. +Main.Thr.Conc.+Nitrogen.Conc.+Lys_conc.+PL+lactate+PO4.app orte; 0.364, 0.687, tmp+Main.P-Source.Conc.+P-Source.Feed.c onc.+Main.Thr.Conc.+Nitrogen.Conc.+OD+Lys_conc.+His+lactate +SO4.ferm.+PO4.apporte; 0.364, 0.686, tmp+P-Source.Feed.con c.+Main.Thr.Conc.+Nitrogen.Conc.+OD+Lys_conc.+PL+lactate+SO 4.ferm.+PO4.ferm.+PO4.apporte; 0.352, 0.686, pH+tmp+P-Sourc e.Feed.conc.+Main.Thr.Conc.+Nitrogen.Conc.+Lys_conc.+yield+ PL+lactate+SO4.ferm.+PO4.ferm.+PO4.apporte; 0.364, 0.686, t mp+Main.P-Source.Conc.+P-Source.Feed.conc.+Main.Thr.Conc.+N itrogen.Conc.+OD+Lys_conc.+yield+SO4.ferm.+PO4.apporte+PO4. utilise; 0.363, 0.686, pH+tmp+Main.P-Source.Conc.+P-Source. Feed.conc.+Main.Thr.Conc.+OD+Lys_conc.+His+SO4.ferm.+PO4.ap porte+PO4.utilise; 0.407, 0.686, tmp+P-Source.Feed.conc.+Ma in.Thr.Conc.+OD+Lys_conc.+yield+SO4.ferm.; 0.363, 0.686, pH +tmp+Main.P-Source.Conc.+P-Source.Feed.conc.+Main.Thr.Conc. +OD+Lys_conc.+lactate+SO4.ferm.+PO4.ferm.+PO4.apporte; 0.36 3, 0.686, pH+tmp+P-Source.Feed.conc.+Main.Thr.Conc.+OD+Lys_ conc.+yield+PL+lactate+SO4.ferm.+PO4.utilise; 0.407, 0.686, tmp+Main.P-Source.Conc.+Main.Thr.Conc.+OD+Lys-conc.+SO4.fe rm.+PO4.apporte; 0.397, 0.686, tmp+P-Source.Feed.conc.+Main. Thr.Conc.+Lys_conc.+rab+SO4.ferm.+PO4.ferm.+PO4.apporte; 0. 363, 0.686, tmp+Main.P-Source.Conc.+P-Source.Feed.conc.+Mai n.Thr.Conc.+Lys conc.+PL+lactate+SO4.ferm.+PO4.ferm.+PO4.ap porte+PO4.utilise; 0.407, 0.686, tmp+P-Source.Feed.conc.+Ma in.Thr.Conc.+Lys_conc.+lactate+PO4.ferm.+PO4.apporte; 0.363, 0.686, pH+tmp+P-Source.Feed.conc.+Main.Thr.Conc.+OD+Lys_co nc.+PL+lactate+SO4.ferm.+PO4.ferm.+PO4.utilise; 0.363, 0.68 6, pH+tmp+Main.P-Source.Conc.+P-Source.Feed.conc.+Main.Thr. Conc.+OD+Lys_conc.+His+SO4.ferm.+PO4.ferm.+PO4.utilise; 0.3 75, 0.686, tmp+Main.P-Source.Conc.+P-Source.Feed.conc.+Main. Thr.Conc.+OD+Lys_conc.+PL+SO4.ferm.+PO4.apporte+PO4.utilis e; 0.351, 0.686, tmp+Main.P-Source.Conc.+P-Source.Feed.conc. +Main.Thr.Conc.+Nitrogen.Conc.+Lys_conc.+yield+PL+lactate+S O4.ferm.+PO4.ferm.+PO4.utilise; 0.397, 0.686, tmp+P-Source. Feed.conc.+Main.Thr.Conc.+Nitrogen.Conc.+Lys_conc.+PL+PO4.f erm.+PO4.apporte; 0.397, 0.686, tmp+Main.P-Source.Conc.+P-S ource.Feed.conc.+Main.Thr.Conc.+Lys_conc.+PL+SO4.ferm.+PO4. utilise; 0.407, 0.686, tmp+Main.Thr.Conc.+Lys_conc.+yield+P L+SO4.ferm.+PO4.apporte

### [14. Linear Model that Predicts Lysine Production Amount in Interval 4]

0.343, 0.643, Main.P-Source.Conc.+P-Source.Feed.conc.+X.F.S uc+PL+rab+SO4.ferm.+PO4.utilise; 0.329, 0.642, pH+Main.P-So urce.Conc.+P-Source.Feed.conc.+X.F.Suc+PL+rab+SO4.ferm.+PO4. utilise; 0.340, 0.641, Main.P-Source.Conc.+P-Source.Feed.co nc.+X.F.Suc+PL+emission_O2+SO4.ferm.+PO4.utilise; 0.326, 0. 639, Main.P-Source.Conc.+P-Source.Feed.conc.+X.F.Suc+PL+rab +Thr+SO4.ferm.+PO4.utilise; 0.325, 0.639, pH+Main.P-Source. Conc.+P-Source.Feed.conc.+X.F.Suc+PL+emission_O2+SO4.ferm.+ PO4.utilise; 0.311, 0.637, pH+Main.P-Source.Conc.+P-Source. Feed.conc.+X.F.Suc+PL+rab+Thr+SO4.ferm.+PO4.utilise; 0.321, 0.636, Main.P-Source.Conc.+P-Source.Feed.conc.+X.F.Suc+PL+ emission_O2+Thr+SO4.ferm.+PO4.utilise; 0.320, 0.635, Main.P -Source.Conc.+P-Source.Feed.conc.+X.F.Suc+PL+rab+His+SO4.fe rm.+PO4.utilise; 0.307, 0.634, pH+Main.P-Source.Conc.+P-Sou rce.Feed.conc.+X.F.Suc+X.VXdt+PL+rab+SO4.ferm.+PO4.utilise; 0.341, 0.634, Main.P-Source.Conc.+P-Source.Feed.conc.+X.F. Suc+rab+SO4.ferm.+PO4.utilise; 0.318, 0.634, Main.P-Source. Conc.+P-Source.Feed.conc.+X.F.Suc+X.VXdt+PL+rab+SO4.ferm.+P O4.utilise; 0.305, 0.634, pH+Main.P-Source.Conc.+P-Source.F eed.conc.+X.F.Suc+PL+rab+His+SO4.ferm.+PO4.utilise; 0.305, 0.633, pH+Main.P-Source.Conc.+P-Source.Feed.conc.+X.F.Suc+P L+emission_O2+Thr+SO4.ferm.+PO4.utilise; 0.317, 0.633, Main. P-Source.Conc.+P-Source.Feed.conc.+X.F.Suc+PL+emission_O2+H is+SO4.ferm.+PO4.utilise; 0.317, 0.633, tmp+Main.P-Source.C onc.+P-Source.Feed.conc.+X.F.Suc+PL+rab+SO4.ferm.+PO4.utili se; 0.316, 0.632, Main.P-Source.Conc.+P-Source.Feed.conc.+X. F.Suc+total.Vol+PL+rab+SO4.ferm.+PO4.utilise; 0.339, 0.632, Main.P-Source.Conc.+P-Source.Feed.conc.+X.F.Suc+emission_O 2+SO4.ferm.+PO4.utilise; 0.315, 0.632, Main.P-Source.Conc.+ P-Source.Feed.conc.+X.F.Suc+PL+rab+SO4.ferm.+PO4.ferm.+PO4. utilise; 0.302, 0.632, Main.P-Source.Conc.+P-Source.Feed.co nc.+X.F.Suc+PL+rab+Thr+His+SO4.ferm.+PO4.utilise; 0.302, 0. 632, tmp+Main.P-Source.Conc.+P-Source.Feed.conc.+X.F.Suc+PL +rab+Thr+SO4.ferm.+PO4.utilise; 0.302, 0.631, pH+Main.P-Sou rce.Conc.+P-Source.Feed.conc.+X.F.Suc+PL+emission_O2+His+SO 4.ferm.+PO4.utilise; 0.302, 0.631, pH+Main.P-Source.Conc.+P -Source.Feed.conc.+X.F.Suc+PL+rab+SO4.ferm.+PO4.ferm.+PO4.u tilise; 0.289, 0.631, pH+Main.P-Source.Conc.+P-Source.Feed. conc.+X.F.Suc+X.VXdt+total.Vol+PL+rab+SO4.ferm.+PO4.utilis e; 0.301, 0.631, pH+tmp+Main.P-Source.Conc.+P-Source.Feed.c onc.+X.F.Suc+PL+rab+SO4.ferm.+PO4.utilise; 0.301, 0.631, pH +Main.P-Source.Conc.+P-Source.Feed.conc.+X.F.Suc+total.Vol+ PL+rab+SO4.ferm.+PO4.utilise; 0.325, 0.631, tmp+Main.P-Sour ce.Conc.+P-Source.Feed.conc.+X.F.Suc+PL+SO4.ferm.+PO4.utili se; 0.313, 0.631, pH+tmp+Main.P-Source.Conc.+P-Source.Feed. conc.+X.F.Suc+PL+SO4.ferm.+PO4.utilise; 0.325, 0.631, Main. P-Source.Conc.+P-Source.Feed.conc.+X.F.Suc+rab+Thr+SO4.ferm. +PO4.utilise; 0.312, 0.630, tmp+Main.P-Source.Conc.+P-Sourc e.Feed.conc.+X.F.Suc+PL+emission_O2+SO4.ferm.+PO4.utilise; 0.286, 0.629, pH+tmp+Main.P-Source.Conc.+P-Source.Feed.conc. +X.F.Suc+X.VXdt+PL+rab+SO4.ferm.+PO4.utilise; 0.311, 0.629, Main.P-Source.Conc.+P-Source.Feed.conc.+X.F.Suc+total.Vol+ PL+emission_O2+SO4.ferm.+PO4.utilise; 0.299, 0.629, pH+Main. P-Source.Conc.+P-Source.Feed.conc.+X.F.Suc+X.VXdt+PL+emissi on_O2+SO4.ferm.+PO4.utilise; 0.311, 0.629, Main.P-Source.Co nc.+P-Source.Feed.conc.+X.F.Suc+X.VXdt+PL+emission_O2+SO4.f erm.+PO4.utilise; 0.286, 0.629, pH+Main.P-Source.Conc.+P-So urce.Feed.conc.+X.F.Suc+PL+rab+Thr+His+SO4.ferm.+PO4.utilis e; 0.285, 0.629, pH+tmp+Main.P-Source.Conc.+P-Source.Feed.c onc.+X.F.Suc+PL+rab+Thr+SO4.ferm.+PO4.utilise; 0.310, 0.629, Main.P-Source.Conc.+P-Source.Feed.conc.+X.F.Suc+PL+emissio n_O2+SO4.ferm.+PO4.ferm.+PO4.utilise; 0.310, 0.629, tmp+Mai n.P-Source.Conc.+P-Source.Feed.conc.+X.F.Suc+PL+Thr+SO4.fer m.+PO4.utilise; 0.298, 0.629, tmp+Main.P-Source.Conc.+P-Sou rce.Feed.conc.+X.F.Suc+X.VXdt+PL+rab+SO4.ferm.+PO4.utilise; 0.298, 0.628, Main.P-Source.Conc.+P-Source.Feed.conc.+X.F. Suc+total.Vol+PL+rab+Thr+SO4.ferm.+PO4.utilise; 0.297, 0.62 8, Main.P-Source.Conc.+P-Source.Feed.conc.+X.F.Suc+X.VXdt+t otal.Vol+PL+rab+SO4.ferm.+PO4.utilise; 0.297, 0.628, tmp+Ma in.P-Source.Conc.+P-Source.Feed.conc.+X.F.Suc+PL+emission_O 2+Thr+SO4.ferm.+PO4.utilise; 0.297, 0.628, Main.P-Source.Co nc.+P-Source.Feed.conc.+X.F.Suc+PL+emission_O2+Thr+His+SO4. ferm.+PO4.utilise; 0.297, 0.628, pH+tmp+Main.P-Source.Conc. +P-Source.Feed.conc.+X.F.Suc+PL+emission_O2+SO4.ferm.+PO4.u tilise; 0.321, 0.628, Main.P-Source.Conc.+P-Source.Feed.con c.+X.F.Suc+rab+His+SO4.ferm.+PO4.utilise; 0.297, 0.628, pH+ Main.P-Source.Conc.+P-Source.Feed.conc.+X.F.Suc+total.Vol+P L+emission_O2+SO4.ferm.+PO4.utilise; 0.321, 0.628, pH+Main. P-Source.Conc.+P-Source.Feed.conc.+X.F.Suc+rab+SO4.ferm.+PO 4.utilise; 0.320, 0.627, Main.P-Source.Conc.+P-Source.Feed. conc.+X.F.Suc+emission_O2+Thr+SO4.ferm.+PO4.utilise; 0.296, 0.627, pH+Main.P-Source.Conc.+P-Source.Feed.conc.+X.F.Suc+ PL+emission_O2+SO4.ferm.+PO4.ferm.+PO4.utilise; 0.296, 0.62 7, pH+tmp+Main.P-Source.Conc.+P-Source.Feed.conc.+X.F.Suc+P L+Thr+SO4.ferm.+PO4.utilise; 0.283, 0.627, pH+Main.P-Source. Conc.+P-Source.Feed.conc.+X.F.Suc+X.VXdt+total.Vol+PL+emiss ion_O2+SO4.ferm.+PO4.utilise; 0.282, 0.627, pH+Main.P-Sourc e.Conc.+P-Source.Feed.conc.+X.F.Suc+X.VXdt+PL+rab+His+SO4.f erm.+PO4.utilise; 0.295, 0.626, tmp+Main.P-Source.Conc.+P-S ource.Feed.conc.+X.F.Suc+total.Vol+PL+rab+SO4.ferm.+PO4.uti lise; 0.295, 0.626, Main.P-Source.Conc.+P-Source.Feed.conc. +X.F.Suc+X.VXdt+PL+rab+His+SO4.ferm.+PO4.utilise; 0.294, 0. 626, pH+tmp+Main.P-Source.Conc.+P-Source.Feed.conc.+X.F.Suc +X.VXdt+PL+SO4.ferm.+PO4.utilise; 0.318, 0.626, Main.P-Sour ce.Conc.+P-Source.Feed.conc.+X.F.Suc+emission_O2+His+SO4.fe rm.+PO4.utilise; 0.318, 0.626, pH+Main.P-Source.Conc.+P-Sou rce.Feed.conc.+X.F.Suc+emission_O2+SO4.ferm.+PO4.utilise; 0. 294, 0.626, tmp+Main.P-Source.Conc.+P-Source.Feed.conc.+X.F. Suc+PL+rab+His+SO4.ferm.+PO4.utilise; 0.281, 0.626, pH+Main. P-Source.Conc.+P-Source.Feed.conc.+X.F.Suc+total.Vol+PL+rab +Thr+SO4.ferm.+PO4.utilise; 0.280, 0.625, pH+tmp+Main.P-Sou rce.Conc.+P-Source.Feed.conc.+X.F.Suc+PL+emission_O2+Thr+SO 4.ferm.+PO4.utilise; 0.280, 0.625, pH+Main.P-Source.Conc.+P -Source.Feed.conc.+X.F.Suc+PL+emission_O2+Thr+His+SO4.ferm. +PO4.utilise; 0.280, 0.625, pH+tmp+Main.P-Source.Conc.+P-So urce.Feed.conc.+X.F.Suc+X.VXdt+PL+emission_O2+SO4.ferm.+PO4. utilise; 0.279, 0.625, pH+Main.P-Source.Conc.+P-Source.Feed. conc.+X.F.Suc+X.VXdt+PL+rab+SO4.ferm.+PO4.ferm.+PO4.utilis e; 0.305, 0.625, Main.P-Source.Conc.+P-Source.Feed.conc.+Ni trogen.Conc.+X.F.Suc+PL+rab+SO4.ferm.+PO4.utilise; 0.279, 0. 625, pH+tmp+Main.P-Source.Conc.+P-Source.Feed.conc.+X.F.Suc +total.Vol+PL+rab+SO4.ferm.+PO4.utilise; 0.292, 0.624, Main. P-Source.Conc.+P-Source.Feed.conc.+X.F.Suc+X.VXdt+total.Vol +PL+emission_O2+SO4.ferm.+PO4.utilise; 0.292, 0.624, Main.P -Source.Conc.+P-Source.Feed.conc.+X.F.Suc+total.Vol+PL+rab+ His+SO4.ferm.+PO4.utilise; 0.328, 0.624, tmp+Main.P-Source. Conc.+P-Source.Feed.conc.+X.F.Suc+SO4.ferm.+PO4.utilise; 0. 292, 0.624, Main.P-Source.Conc.+P-Source.Feed.conc.+X.F.Suc +total.Vol+PL+emission_O2+Thr+SO4.ferm.+PO4.utilise; 0.291, 0.624, tmp+Main.P-Source.Conc.+P-Source.Feed.conc.+X.F.Suc +PL+rab+SO4.ferm.+PO4.ferm.+PO4.utilise; 0.291, 0.624, tmp+ Main.P-Source.Conc.+P-Source.Feed.conc.+X.F.Suc+X.VXdt+PL+e mission_O2+SO4.ferm.+PO4.utilise; 0.278, 0.624, tmp+Main.P-Source.Conc.+P-Source.Feed.conc.+X.F.Suc+PL+rab+Thr+His+SO4. ferm.+PO4.utilise; 0.315, 0.624, Main.P-Source.Conc.+P-Sour ce.Feed.conc.+X.F.Suc+total.Vol+rab+SO4.ferm.+PO4.utilise; 0.303, 0.624, pH+Main.P-Source.Conc.+P-Source.Feed.conc.+X. F.Suc+rab+Thr+SO4.ferm.+PO4.utilise; 0.303, 0.624, tmp+Main. P-Source.Conc.+P-Source.Feed.conc.+X.F.Suc+PL+His+SO4.ferm. +PO4.utilise; 0.291, 0.624, pH+Main.P-Source.Conc.+P-Source. Feed.conc.+Nitrogen.Conc.+X.F.Suc+PL+rab+SO4.ferm.+PO4.util ise; 0.303, 0.624, Main.P-Source.Conc.+P-Source.Feed.conc.+ X.F.Suc+rab+Thr+His+SO4.ferm.+PO4.utilise; 0.291, 0.624, Ma in.P-Source.Conc.+P-Source.Feed.conc.+X.F.Suc+X.VXdt+PL+rab +SO4.ferm.+PO4.ferm.+PO4.utilise; 0.264, 0.623, pH+Main.P-S ource.Conc.+P-Source.Feed.conc.+X.F.Suc+X.VXdt+total.Vol+PL +rab+His+SO4.ferm.+PO4.utilise; 0.315, 0.623, tmp+Main.P-So urce.Conc.+P-Source.Feed.conc.+X.F.Suc+rab+SO4.ferm.+PO4.ut ilise; 0.303, 0.623, tmp+Main.P-Source.Conc.+P-Source.Feed. conc.+X.F.Suc+X.VXdt+PL+SO4.ferm.+PO4.utilise; 0.290, 0.623, pH+tmp+Main.P-Source.Conc.+P-Source.Feed.conc.+X.F.Suc+tot al.Vol+PL+SO4.ferm.+PO4.utilise; 0.277, 0.623, tmp+Main.P-S ource.Conc.+P-Source.Feed.conc.+X.F.Suc+total.Vol+PL+rab+Th r+SO4.ferm.+PO4.utilise; 0.277, 0.623, pH+tmp+Main.P-Source. Conc.+P-Source.Feed.conc.+X.F.Suc+PL+rab+His+SO4.ferm.+PO4. utilise; 0.303, 0.623, tmp+Main.P-Source.Conc.+P-Source.Fee d.conc.+X.F.Suc+total.Vol+PL+SO4.ferm.+PO4.utilise; 0.290, 0.623, pH+tmp+Main.P-Source.Conc.+P-Source.Feed.conc.+X.F.S uc+PL+His+SO4.ferm.+PO4.utilise; 0.290, 0.623, tmp+Main.P-S ource.Conc.+P-Source.Feed.conc.+X.F.Suc+total.Vol+PL+emissi on_O2+SO4.ferm.+PO4.utilise; 0.290, 0.623, Main.P-Source.Co nc.+P-Source.Feed.conc.+X.F.Suc+PL+rab+Thr+SO4.ferm.+PO4.fe rm.+PO4.utilise; 0.277, 0.623, pH+tmp+Main.P-Source.Conc.+P -Source.Feed.conc.+X.F.Suc+PL+rab+SO4.ferm.+PO4.ferm.+PO4.u tilise; 0.289, 0.623, Main.P-Source.Conc.+P-Source.Feed.con c.+X.F.Suc+PL+rab+His+SO4.ferm.+PO4.ferm.+PO4.utilise; 0.28 9, 0.623, tmp+Main.P-Source.Conc.+P-Source.Feed.conc.+X.F.S uc+PL+emission_O2+His+SO4.ferm.+PO4.utilise; 0.276, 0.623, pH+Main.P-Source.Conc.+P-Source.Feed.conc.+X.F.Suc+total.Vo l+PL+rab+His+SO4.ferm.+PO4.utilise; 0.289, 0.622, pH+tmp+Ma in.P-Source.Conc.+P-Source.Feed.conc.+X.F.Suc+PL+SO4.ferm.+ PO4.ferm.+PO4.utilise; 0.301, 0.622, Main.P-Source.Conc.+P-Source.Feed.conc.+Nitrogen.Conc.+X.F.Suc+PL+emission_O2+SO4. ferm.+PO4.utilise; 0.262, 0.622, pH+tmp+Main.P-Source.Conc. +P-Source.Feed.conc.+X.F.Suc+X.VXdt+PL+rab+His+SO4.ferm.+PO 4.utilise; 0.262, 0.622, pH+Main.P-Source.Conc.+P-Source.Fe ed.conc.+OD+X.F.Suc+total.Vol+total.cell+PL+rab+SO4.ferm.+P O4.utilise; 0.300, 0.622, tmp+Main.P-Source.Conc.+P-Source. Feed.conc.+X.F.Suc+PL+SO4.ferm.+PO4.ferm.+PO4.utilise; 0.28 8, 0.622, Main.P-Source.Conc.+P-Source.Feed.conc.+X.F.Suc+t otal.Vol+PL+rab+SO4.ferm.+PO4.ferm.+PO4.utilise; 0.275, 0.6 22, pH+Main.P-Source.Conc.+P-Source.Feed.conc.+X.F.Suc+PL+r ab+His+SO4.ferm.+PO4.ferm.+PO4.utilise; 0.275, 0.621, tmp+M ain.P-Source.Conc.+P-Source.Feed.conc.+X.F.Suc+X.VXdt+PL+ra b+His+SO4.ferm.+PO4.utilise; 0.287, 0.621, Main.P-Source.Co nc.+P-Source.Feed.conc.+X.F.Suc+X.VXdt+PL+emission_O2+His+S O4.ferm.+PO4.utilise; 0.274, 0.621, pH+tmp+Main.P-Source.Co nc.+P-Source.Feed.conc.+X.F.Suc+total.Vol+PL+emission_O2+SO 4.ferm.+PO4.utilise; 0.312, 0.621, Main.P-Source.Conc.+P-So urce.Feed.conc.+X.F.Suc+total.Vol+emission_O2+SO4.ferm.+PO4. utilise; 0.300, 0.621, tmp+Main.P-Source.Conc.+P-Source.Fee d.conc.+X.F.Suc+rab+Thr+SO4.ferm.+PO4.utilise; 0.274, 0.621, pH+Main.P-Source.Conc.+P-Source.Feed.conc.+X.F.Suc+total.V ol+PL+rab+SO4.ferm.+PO4.ferm.+PO4.utilise; 0.274, 0.621, pH +Main.P-Source.Conc.+P-Source.Feed.conc.+X.F.Suc+X.VXdt+PL+ emission_O2+His+SO4.ferm.+PO4.utilise; 0.287, 0.621, Main.P -Source.Conc.+P-Source.Feed.conc.+X.F.Suc+total.Vol+PL+emis sion_O2+His+SO4.ferm.+PO4.utilise; 0.300, 0.621, pH+Main.P-Source.Conc.+P-Source.Feed.conc.+X.F.Suc+rab+His+SO4.ferm.+ PO4.utilise; 0.287, 0.621, tmp+Main.P-Source.Conc.+P-Source. Feed.conc.+X.F.Suc+PL+Thr+His+SO4.ferm.+PO4.utilise; 0.312, 0.621, tmp+Main.P-Source.Conc.+P-Source.Feed.conc.+X.F.Suc +Thr+SO4.ferm.+PO4.utilise; 0.274, 0.621, pH+Main.P-Source. Conc.+P-Source.Feed.conc.+X.F.Suc+total.Vol+PL+emission_O2+ Thr+SO4.ferm.+PO4.utilise; 0.274, 0.621, Main.P-Source.Conc. +P-Source.Feed.conc.+X.F.Suc+X.VXdt+total.Vol+PL+rab+His+SO 4.ferm.+PO4.utilise; 0.287, 0.621, pH+Main.P-Source.Conc.+P -Source.Feed.conc.+Nitrogen.Conc.+X.F.Suc+PL+emission_O2+SO 4.ferm.+PO4.utilise; 0.274, 0.621, tmp+Main.P-Source.Conc.+ P-Source.Feed.conc.+X.F.Suc+PL+emission_O2+Thr+His+SO4.ferm. +PO4.utilise; 0.260, 0.621, pH+tmp+Main.P-Source.Conc.+P-So urce.Feed.conc.+X.F.Suc+PL+rab+Thr+His+SO4.ferm.+PO4.utilis e; 0.299, 0.621, pH+Main.P-Source.Conc.+P-Source.Feed.conc. +X.F.Suc+emission_O2+Thr+SO4.ferm.+PO4.utilise; 0.286, 0.62 1, tmp+Main.P-Source.Conc.+P-Source.Feed.conc.+X.F.Suc+PL+e mission_O2+SO4.ferm.+PO4.ferm.+PO4.utilise; 0.299, 0.620, M ain.P-Source.Conc.+P-Source.Feed.conc.+X.F.Suc+total.cell+P L+rab+SO4.ferm.+PO4.utilise; 0.311, 0.620, Main.P-Source.Co nc.+P-Source.Feed.conc.+X.F.Suc+X.VXdt+rab+SO4.ferm.+PO4.ut ilise; 0.298, 0.620, Main.P-Source.Conc.+P-Source.Feed.conc. +X.F.Suc+emission_O2+Thr+His+SO4.ferm.+PO4.utilise; 0.259, 0.620, pH+tmp+Main.P-Source.Conc.+P-Source.Feed.conc.+X.F.S uc+total.Vol+PL+rab+Thr+SO4.ferm.+PO4.utilise; 0.273, 0.620, pH+tmp+Main.P-Source.Conc.+P-Source.Feed.conc.+X.F.Suc+PL+ emission_O2+His+SO4.ferm.+PO4.utilise; 0.286, 0.620, pH+Mai n.P-Source.Conc.+P-Source.Feed.conc.+X.F.Suc+total.cell+PL+ rab+SO4.ferm.+PO4.utilise; 0.272, 0.620, pH+Main.P-Source.C onc.+P-Source.Feed.conc.+X.F.Suc+PL+rab+Thr+SO4.ferm.+PO4.f erm.+PO4.utilise; 0.272, 0.620, Main.P-Source.Conc.+P-Sourc e.Feed.conc.+X.F.Suc+total.Vol+PL+rab+Thr+His+SO4.ferm.+PO4. utilise; 0.285, 0.620, Main.P-Source.Conc.+P-Source.Feed.co nc.+Nitrogen.Conc.+X.F.Suc+PL+rab+Thr+SO4.ferm.+PO4.utilis e; 0.298, 0.620, Main.P-Source.Conc.+P-Source.Feed.conc.+X. F.Suc+total.Vol+rab+Thr+SO4.ferm.+PO4.utilise; 0.310, 0.620, tmp+Main.P-Source.Conc.+P-Source.Feed.conc.+X.F.Suc+emissi on_O2+SO4.ferm.+PO4.utilise; 0.272, 0.620, pH+Main.P-Source. Conc.+P-Source.Feed.conc.+X.F.Suc+X.VXdt+PL+emission_O2+SO4. ferm.+PO4.ferm.+PO4.utilise; 0.272, 0.620, tmp+Main.P-Sourc e.Conc.+P-Source.Feed.conc.+X.F.Suc+total.Vol+PL+emission_O 2+Thr+SO4.ferm.+PO4.utilise; 0.297, 0.619, pH+Main.P-Source. Conc.+P-Source.Feed.conc.+X.F.Suc+emission_O2+His+SO4.ferm. +PO4.utilise; 0.309, 0.619, Main.P-Source.Conc.+P-Source.Fe ed.conc.+X.F.Suc+rab+SO4.ferm.+PO4.ferm.+PO4.utilise; 0.272, 0.619, pH+tmp+Main.P-Source.Conc.+P-Source.Feed.conc.+X.F. Suc+PL+emission_O2+SO4.ferm.+PO4.ferm.+PO4.utilise; 0.271, 0.619, tmp+Main.P-Source.Conc.+P-Source.Feed.conc.+X.F.Suc+ total.Vol+PL+rab+His+SO4.ferm.+PO4.utilise; 0.258, 0.619, p H+Main.P-Source.Conc.+P-Source.Feed.conc.+X.F.Suc+X.VXdt+to tal.Vol+PL+emission_O2+His+SO4.ferm.+PO4.utilise; 0.284, 0. 619, Main.P-Source.Conc.+P-Source.Feed.conc.+X.F.Suc+X.VXdt +PL+emission_O2+SO4.ferm.+PO4.ferm.+PO4.utilise; 0.258, 0.6 19, pH+Main.P-Source.Conc.+P-Source.Feed.conc.+X.F.Suc+X.VX dt+total.Vol+PL+rab+SO4.ferm.+PO4.ferm.+PO4.utilise; 0.271, 0.619, pH+tmp+Main.P-Source.Conc.+P-Source.Feed.conc.+X.F. Suc+PL+Thr+His+SO4.ferm.+PO4.utilise; 0.258, 0.619, pH+tmp+ Main.P-Source.Conc.+P-Source.Feed.conc.+X.F.Suc+X.VXdt+PL+r ab+SO4.ferm.+PO4.ferm.+PO4.utilise; 0.271, 0.619, pH+Main.P -Source.Conc.+P-Source.Feed.conc.+X.F.Suc+total.Vol+PL+emis sion_O2+His+SO4.ferm.+PO4.utilise; 0.271, 0.619, pH+tmp+Mai n.P-Source.Conc.+P-Source.Feed.conc.+X.F.Suc+X.VXdt+PL+His+ SO4.ferm.+PO4.utilise; 0.284, 0.619, Main.P-Source.Conc.+P-Source.Feed.conc.+X.F.Suc+total.Vol+PL+emission_O2+SO4.ferm. +PO4.ferm.+PO4.utilise; 0.284, 0.619, Main.P-Source.Conc.+P -Source.Feed.conc.+X.F.Suc+PL+emission_O2+His+SO4.ferm.+PO4. ferm.+PO4.utilise; 0.284, 0.619, tmp+Main.P-Source.Conc.+P-Source.Feed.conc.+X.F.Suc+total.Vol+PL+Thr+SO4.ferm.+PO4.ut ilise; 0.284, 0.619, Main.P-Source.Conc.+P-Source.Feed.conc. +X.F.Suc+X.VXdt+PL+rab+Thr+SO4.ferm.+PO4.utilise; 0.296, 0. 619, Main.P-Source.Conc.+P-Source.Feed.conc.+Main.Thr.Conc. +X.F.Suc+PL+rab+SO4.ferm.+PO4.utilise; 0.308, 0.619, tmp+Ma in.P-Source.Conc.+P-Source.Feed.conc.+X.F.Suc+His+SO4.ferm. +PO4.utilise; 0.296, 0.619, Main.P-Source.Conc.+P-Source.Fe ed.conc.+Lys_conc.+X.F.Suc+PL+emission_O2+SO4.ferm.+PO4.uti lise; 0.308, 0.618, pH+tmp+Main.P-Source.Conc.+P-Source.Fee d.conc.+X.F.Suc+SO4.ferm.+PO4.utilise; 0.296, 0.618, Main.P -Source.Conc.+P-Source.Feed.conc.+X.F.Suc+Cell+PL+rab+SO4.f erm.+PO4.utilise; 0.270, 0.618, pH+Main.P-Source.Conc.+P-So urce.Feed.conc.+Nitrogen.Conc.+X.F.Suc+PL+rab+Thr+SO4.ferm. +PO4.utilise; 0.270, 0.618, pH+Main.P-Source.Conc.+P-Source. Feed.conc.+X.F.Suc+total.Vol+PL+emission_O2+SO4.ferm.+PO4.f erm.+PO4.utilise; 0.283, 0.618, pH+Main.P-Source.Conc.+P-So urce.Feed.conc.+X.F.Suc+Cell+PL+rab+SO4.ferm.+PO4.utilise; 0.295, 0.618, Main.P-Source.Conc.+P-Source.Feed.conc.+Main. Thr.Conc.+X.F.Suc+PL+emission_O2+SO4.ferm.+PO4.utilise; 0.2 83, 0.618, Main.P-Source.Conc.+P-Source.Feed.conc.+X.F.Suc+ PL+emission_O2+Thr+SO4.ferm.+PO4.ferm.+PO4.utilise; 0.283, 0.618, pH+Main.P-Source.Conc.+P-Source.Feed.conc.+Main.Thr. Conc.+X.F.Suc+PL+rab+SO4.ferm.+PO4.utilise; 0.269, 0.618, t mp+Main.P-Source.Conc.+P-Source.Feed.conc.+X.F.Suc+X.VXdt+P L+rab+SO4.ferm.+PO4.ferm.+PO4.utilise; 0.269, 0.618, tmp+Ma in.P-Source.Conc.+P-Source.Feed.conc.+X.F.Suc+PL+rab+Thr+SO 4.ferm.+PO4.ferm.+PO4.utilise; 0.256, 0.618, pH+tmp+Main.P-Source.Conc.+P-Source.Feed.conc.+X.F.Suc+X.VXdt+PL+emission _O2+His+SO4.ferm.+PO4.utilise; 0.269, 0.618, Main.P-Source. Conc.+P-Source.Feed.conc.+OD+X.F.Suc+total.Vol+total.cell+P L+rab+SO4.ferm.+PO4.utilise; 0.295, 0.618, Main.P-Source.Co nc.+P-Source.Feed.conc.+Lys_conc.+X.F.Suc+PL+rab+SO4.ferm.+ PO4.utilise; 0.295, 0.618, tmp+Main.P-Source.Conc.+P-Source. Feed.conc.+X.F.Suc+emission_O2+Thr+SO4.ferm.+PO4.utilise; 0. 307, 0.617, Main.P-Source.Conc.+P-Source.Feed.conc.+X.F.Suc +emission_O2+SO4.ferm.+PO4.ferm.+PO4.utilise; 0.282, 0.617, pH+Main.P-Source.Conc.+P-Source.Feed.conc.+Main.Thr.Conc.+ X.F.Suc+PL+emission_O2+SO4.ferm.+PO4.utilise; 0.269, 0.617, pH+Main.P-Source.Conc.+P-Source.Feed.conc.+Nitrogen.Conc.+ X.F.Suc+X.VXdt+PL+rab+SO4.ferm.+PO4.utilise; 0.269, 0.617, pH+Main.P-Source.Conc.+P-Source.Feed.conc.+X.F.Suc+PL+emiss ion_O2+His+SO4.ferm.+PO4.ferm.+PO4.utilise; 0.294, 0.617, p H+Main.P-Source.Conc.+P-Source.Feed.conc.+X.F.Suc+total.Vol +rab+SO4.ferm.+PO4.utilise; 0.255, 0.617, pH+tmp+Main.P-Sou rce.Conc.+P-Source.Feed.conc.+X.F.Suc+PL+emission_O2+Thr+Hi s+SO4.ferm.+PO4.utilise; 0.269, 0.617, pH+Main.P-Source.Con c.+P-Source.Feed.conc.+X.F.Suc+X.VXdt+total.cell+PL+rab+SO4. ferm.+PO4.utilise; 0.294, 0.617, tmp+Main.P-Source.Conc.+P-Source.Feed.conc.+X.F.Suc+rab+His+SO4.ferm.+PO4.utilise; 0. 268, 0.617, pH+tmp+Main.P-Source.Conc.+P-Source.Feed.conc.+ X.F.Suc+total.Vol+PL+Thr+SO4.ferm.+PO4.utilise; 0.268, 0.61 7, tmp+Main.P-Source.Conc.+P-Source.Feed.conc.+X.F.Suc+X.VX dt+PL+emission_O2+His+SO4.ferm.+PO4.utilise; 0.255, 0.617, pH+tmp+Main.P-Source.Conc.+P-Source.Feed.conc.+X.F.Suc+tota l.Vol+PL+rab+His+SO4.ferm.+PO4.utilise; 0.268, 0.617, tmp+M ain.P-Source.Conc.+P-Source.Feed.conc.+X.F.Suc+X.VXdt+PL+ra b+Thr+SO4.ferm.+PO4.utilise; 0.281, 0.617, pH+Main.P-Source. Conc.+P-Source.Feed.conc.+Lys_conc.+X.F.Suc+PL+emission_O2+ SO4.ferm.+PO4.utilise; 0.281, 0.617, pH+Main.P-Source.Conc. +P-Source.Feed.conc.+X.F.Suc+PL+rab+lactate+SO4.ferm.+PO4.u tilise; 0.281, 0.617, tmp+Main.P-Source.Conc.+P-Source.Feed. conc.+X.F.Suc+total.Vol+PL+His+SO4.ferm.+PO4.utilise; 0.306, 0.617, Main.P-Source.Conc.+P-Source.Feed.conc.+X.F.Suc+X.V Xdt+emission_O2+SO4.ferm.+PO4.utilise; 0.268, 0.617, Main.P -Source.Conc.+P-Source.Feed.conc.+X.F.Suc+X.VXdt+total.Vol+ PL+emission_O2+His+SO4.ferm.+PO4.utilise; 0.294, 0.617, Mai n.P-Source.Conc.+P-Source.Feed.conc.+X.F.Suc+total.Vol+rab+ His+SO4.ferm.+PO4.utilise; 0.281, 0.617, tmp+Main.P-Source. Conc.+P-Source.Feed.conc.+X.F.Suc+X.VXdt+PL+His+SO4.ferm.+P O4.utilise; 0.281, 0.617, pH+Main.P-Source.Conc.+P-Source.F eed.conc.+X.F.Suc+rab+Thr+His+SO4.ferm.+PO4.utilise; 0.281, 0.617, pH+Main.P-Source.Conc.+P-Source.Feed.conc.+Lys_conc. +X.F.Suc+PL+rab+SO4.ferm.+PO4.utilise; 0.254, 0.617, pH+Mai n.P-Source.Conc.+P-Source.Feed.conc.+X.F.Suc+total.Vol+PL+r ab+Thr+His+SO4.ferm.+PO4.utilise; 0.281, 0.617, Main.P-Sour ce.Conc.+P-Source.Feed.conc.+Main.Thr.Conc.+X.F.Suc+PL+rab+ Thr+SO4.ferm.+PO4.utilise; 0.254, 0.617, pH+Main.P-Source.C onc.+P-Source.Feed.conc.+X.F.Suc+X.VXdt+PL+rab+Thr+lactate+ SO4.ferm.+PO4.utilise; 0.254, 0.617, pH+tmp+Main.P-Source.C onc.+P-Source.Feed.conc.+Nitrogen.Conc.+X.F.Suc+X.VXdt+PL+r ab+SO4.ferm.+PO4.utilise; 0.267, 0.616, pH+tmp+Main.P-Sourc e.Conc.+P-Source.Feed.conc.+X.F.Suc+total.Vol+PL+His+SO4.fe rm.+PO4.utilise; 0.254, 0.616, pH+tmp+Main.P-Source.Conc.+P -Source.Feed.conc.+X.F.Suc+total.Vol+PL+emission_O2+Thr+SO4. ferm.+PO4.utilise; 0.254, 0.616, pH+Main.P-Source.Conc.+P-S ource.Feed.conc.+Nitrogen.Conc.+X.F.Suc+X.VXdt+total.Vol+PL +rab+SO4.ferm.+PO4.utilise; 0.280, 0.616, Main.P-Source.Con c.+P-Source.Feed.conc.+Nitrogen.Conc.+X.F.Suc+X.VXdt+PL+rab +SO4.ferm.+PO4.utilise; 0.254, 0.616, pH+Main.P-Source.Conc. +P-Source.Feed.conc.+OD+X.F.Suc+total.Vol+total.cell+PL+emi ssion_O2+SO4.ferm.+PO4.utilise; 0.293, 0.616, Main.P-Source. Conc.+P-Source.Feed.conc.+X.F.Suc+total.Vol+emission_O2+Thr +SO4.ferm.+PO4.utilise; 0.267, 0.616, tmp+Main.P-Source.Con c.+P-Source.Feed.conc.+X.F.Suc+total.Vol+PL+emission_O2+His +SO4.ferm.+PO4.utilise; 0.267, 0.616, Main.P-Source.Conc.+P -Source.Feed.conc.+X.F.Suc+X.VXdt+total.Vol+PL+rab+SO4.ferm. +PO4.ferm.+PO4.utilise; 0.253, 0.616, pH+tmp+Main.P-Source. Conc.+P-Source.Feed.conc.+X.F.Suc+X.VXdt+total.Vol+PL+rab+S O4.ferm.+PO4.utilise; 0.267, 0.616, pH+Main.P-Source.Conc.+ P-Source.Feed.conc.+Main.Thr.Conc.+X.F.Suc+X.VXdt+PL+rab+SO 4.ferm.+PO4.utilise; 0.280, 0.616, Main.P-Source.Conc.+P-So urce.Feed.conc.+Nitrogen.Conc.+X.F.Suc+PL+emission_O2+Thr+S O4.ferm.+PO4.utilise; 0.292, 0.616, pH+tmp+Main.P-Source.Co nc.+P-Source.Feed.conc.+X.F.Suc+rab+SO4.ferm.+PO4.utilise; 0.266, 0.616, pH+Main.P-Source.Conc.+P-Source.Feed.conc.+X. F.Suc+X.VXdt+PL+rab+lactate+SO4.ferm.+PO4.utilise; 0.279, 0. 616, Main.P-Source.Conc.+P-Source.Feed.conc.+X.F.Suc+X.VXdt +total.cell+PL+rab+SO4.ferm.+PO4.utilise; 0.266, 0.616, pH+ Main.P-Source.Conc.+P-Source.Feed.conc.+Main.Thr.Conc.+X.F. Suc+PL+rab+Thr+SO4.ferm.+PO4.utilise; 0.266, 0.616, Main.P-Source.Conc.+P-Source.Feed.conc.+X.F.Suc+total.Vol+PL+emiss ion_O2+Thr+His+SO4.ferm.+PO4.utilise; 0.253, 0.616, tmp+Mai n.P-Source.Conc.+P-Source.Feed.conc.+X.F.Suc+total.Vol+PL+r ab+Thr+His+SO4.ferm.+PO4.utilise; 0.304, 0.616, tmp+Main.P-Source.Conc.+P-Source.Feed.conc.+X.F.Suc+total.Vol+SO4.ferm. +PO4.utilise; 0.252, 0.616, pH+Main.P-Source.Conc.+P-Source. Feed.conc.+X.F.Suc+X.VXdt+total.Vol+PL+emission_O2+SO4.ferm. +PO4.ferm.+PO4.utilise; 0.266, 0.616, tmp+Main.P-Source.Con c.+P-Source.Feed.conc.+X.F.Suc+PL+rab+His+SO4.ferm.+PO4.fer m.+PO4.utilise; 0.292, 0.615, Main.P-Source.Conc.+P-Source. Feed.conc.+X.F.Suc+total.cell+PL+emission_O2+SO4.ferm.+PO4. utilise; 0.279, 0.615, tmp+Main.P-Source.Conc.+P-Source.Fee d.conc.+Nitrogen.Conc.+X.F.Suc+PL+rab+SO4.ferm.+PO4.utilis e; 0.252, 0.615, pH+Main.P-Source.Conc.+P-Source.Feed.conc. +X.F.Suc+X.VXdt+PL+rab+His+SO4.ferm.+PO4.ferm.+PO4.utilise; 0.266, 0.615, tmp+Main.P-Source.Conc.+P-Source.Feed.conc.+ Nitrogen.Conc.+X.F.Suc+X.VXdt+PL+rab+SO4.ferm.+PO4.utilise; 0.266, 0.615, pH+tmp+Main.P-Source.Conc.+P-Source.Feed.con c.+X.F.Suc+X.VXdt+PL+SO4.ferm.+PO4.ferm.+PO4.utilise; 0.291, 0.615, tmp+Main.P-Source.Conc.+P-Source.Feed.conc.+X.F.Suc +total.Vol+rab+SO4.ferm.+PO4.utilise; 0.252, 0.615, pH+Main. P-Source.Conc.+P-Source.Feed.conc.+Main.Thr.Conc.+X.F.Suc+X. VXdt+total.Vol+PL+rab+SO4.ferm.+PO4.utilise; 0.252, 0.615, pH+tmp+Main.P-Source.Conc.+P-Source.Feed.conc.+X.F.Suc+X.VX dt+PL+emission_O2+SO4.ferm.+PO4.ferm.+PO4.utilise; 0.278, 0. 615, Main.P-Source.Conc.+P-Source.Feed.conc.+Main.Thr.Conc. +X.F.Suc+PL+emission_O2+Thr+SO4.ferm.+PO4.utilise; 0.291, 0. 615, Main.P-Source.Conc.+P-Source.Feed.conc.+X.F.Suc+PL+rab +lactate+SO4.ferm.+PO4.utilise; 0.265, 0.615, tmp+Main.P-So urce.Conc.+P-Source.Feed.conc.+X.F.Suc+X.VXdt+total.Vol+PL+ rab+SO4.ferm.+PO4.utilise; 0.291, 0.615, pH+Main.P-Source.C onc.+P-Source.Feed.conc.+X.F.Suc+total.Vol+emission_O2+SO4. ferm.+PO4.utilise; 0.265, 0.615, pH+Main.P-Source.Conc.+P-S ource.Feed.conc.+X.F.Suc+PL+rab+Thr+lactate+SO4.ferm.+PO4.u tilise; 0.291, 0.615, Main.P-Source.Conc.+P-Source.Feed.con c.+yield+PL+rab+SO4.ferm.+PO4.ferm.+PO4.utilise; 0.291, 0.6 15, tmp+Main.P-Source.Conc.+P-Source.Feed.conc.+yield+PL+SO 4.ferm.+PO4.ferm.+PO4.utilise; 0.278, 0.615, Main.P-Source. Conc.+P-Source.Feed.conc.+Lys_conc.+X.F.Suc+PL+rab+Thr+SO4. ferm.+PO4.utilise; 0.278, 0.615, pH+Main.P-Source.Conc.+P-S ource.Feed.conc.+X.F.Suc+total.cell+PL+emission_O2+SO4.ferm. +PO4.utilise; 0.278, 0.615, pH+tmp+Main.P-Source.Conc.+P-So urce.Feed.conc.+yield+PL+SO4.ferm.+PO4.ferm.+PO4.utilise; 0. 290, 0.615, tmp+Main.P-Source.Conc.+P-Source.Feed.conc.+X.F. Suc+Thr+His+SO4.ferm.+PO4.utilise; 0.278, 0.615, tmp+Main.P -Source.Conc.+P-Source.Feed.conc.+X.F.Suc+rab+Thr+His+SO4.f erm.+PO4.utilise; 0.264, 0.614, Main.P-Source.Conc.+P-Sourc e.Feed.conc.+X.F.Suc+X.VXdt+total.Vol+PL+rab+Thr+SO4.ferm.+ PO4.utilise; 0.264, 0.614, pH+Main.P-Source.Conc.+P-Source. Feed.conc.+X.F.Suc+PL+emission_O2+Thr+SO4.ferm.+PO4.ferm.+P O4.utilise; 0.264, 0.614, Main.P-Source.Conc.+P-Source.Feed. conc.+X.F.Suc+X.VXdt+PL+rab+His+SO4.ferm.+PO4.ferm.+PO4.uti lise; 0.251, 0.614, pH+tmp+Main.P-Source.Conc.+P-Source.Fee d.conc.+X.F.Suc+PL+rab+Thr+SO4.ferm.+PO4.ferm.+PO4.utilise; 0.290, 0.614, Main.P-Source.Conc.+P-Source.Feed.conc.+X.F. Suc+Cell+PL+emission_O2+SO4.ferm.+PO4.utilise; 0.264, 0.614, tmp+Main.P-Source.Conc.+P-Source.Feed.conc.+X.F.Suc+PL+emi ssion_O2+Thr+SO4.ferm.+PO4.ferm.+PO4.utilise; 0.264, 0.614, pH+tmp+Main.P-Source.Conc.+P-Source.Feed.conc.+X.F.Suc+PL+ His+SO4.ferm.+PO4.ferm.+PO4.utilise; 0.290, 0.614, Main.P-S ource.Conc.+P-Source.Feed.conc.+X.F.Suc+total.Vol+emission O2+His+SO4.ferm.+PO4.utilise; 0.290, 0.614, pH+tmp+Main.P-S ource.Conc.+P-Source.Feed.conc.+X.F.Suc+Thr+SO4.ferm.+PO4.u tilise; 0.277, 0.614, Main.P-Source.Conc.+P-Source.Feed.con c.+Lys_conc.+X.F.Suc+PL+emission_O2+Thr+SO4.ferm.+PO4.utili se; 0.250, 0.614, pH+tmp+Main.P-Source.Conc.+P-Source.Feed. conc.+X.F.Suc+total.Vol+PL+emission_O2+His+SO4.ferm.+PO4.ut ilise; 0.277, 0.614, tmp+Main.P-Source.Conc.+P-Source.Feed. conc.+X.F.Suc+PL+His+SO4.ferm.+PO4.ferm.+PO4.utilise; 0.290, 0.614, pH+Main.P-Source.Conc.+P-Source.Feed.conc.+X.F.Suc+ X.VXdt+rab+SO4.ferm.+PO4.utilise; 0.290, 0.614, tmp+Main.P-Source.Conc.+P-Source.Feed.conc.+X.F.Suc+emission_O2+His+SO 4.ferm.+PO4.utilise; 0.264, 0.614, pH+Main.P-Source.Conc.+P -Source.Feed.conc.+X.F.Suc+PL+emission_O2+Thr+lactate+SO4.f erm.+PO4.utilise; 0.289, 0.614, Main.P-Source.Conc.+P-Sourc e.Feed.conc.+OD+X.F.Suc+PL+rab+SO4.ferm.+PO4.utilise; 0.277, 0.614, pH+Main.P-Source.Conc.+P-Source.Feed.conc.+X.F.Suc+ PL+emission_O2+lactate+SO4.ferm.+PO4.utilise; 0.250, 0.614, pH+tmp+Main.P-Source.Conc.+P-Source.Feed.conc.+X.F.Suc+PL+ rab+His+SO4.ferm.+PO4.ferm.+PO4.utilise; 0.264, 0.614, pH+M ain.P-Source.Conc.+P-Source.Feed.conc.+Main.Thr.Conc.+X.F.S uc+PL+emission_O2+Thr+SO4.ferm.+PO4.utilise; 0.264, 0.614, tmp+Main.P-Source.Conc.+P-Source.Feed.conc.+X.F.Suc+X.VXdt+ PL+emission_O2+SO4.ferm.+PO4.ferm.+PO4.utilise; 0.264, 0.61 4, pH+Main.P-Source.Conc.+P-Source.Feed.conc.+Nitrogen.Conc. +X.F.Suc+PL+emission_O2+Thr+SO4.ferm.+PO4.utilise; 0.277, 0. 614, pH+Main.P-Source.Conc.+P-Source.Feed.conc.+X.F.Suc+Cel l+PL+emission_O2+SO4.ferm.+PO4.utilise; 0.277, 0.614, Main. P-Source.Conc.+P-Source.Feed.conc.+Nitrogen.Conc.+X.F.Suc+t otal.Vol+PL+rab+SO4.ferm.+PO4.utilise; 0.301, 0.614, Main.P -Source.Conc.+P-Source.Feed.conc.+Nitrogen.Conc.+X.F.Suc+ra b+SO4.ferm.+PO4.utilise; 0.289, 0.614, Main.P-Source.Conc.+ P-Source.Feed.conc.+X.F.Suc+X.VXdt+rab+His+SO4.ferm.+PO4.ut ilise; 0.301, 0.614, Main.P-Source.Conc.+P-Source.Feed.conc. +yield+PL+rab+SO4.ferm.+PO4.utilise; 0.249, 0.614, pH+Main. P-Source.Conc.+P-Source.Feed.conc.+X.F.Suc+X.VXdt+total.Vol +total.cell+PL+rab+SO4.ferm.+PO4.utilise; 0.276, 0.613, pH+ Main.P-Source.Conc.+P-Source.Feed.conc.+OD+X.F.Suc+PL+rab+S O4.ferm.+PO4.utilise; 0.263, 0.613, pH+tmp+Main.P-Source.Co nc.+P-Source.Feed.conc.+Nitrogen.Conc.+X.F.Suc+PL+rab+SO4.f erm.+PO4.utilise; 0.276, 0.613, pH+Main.P-Source.Conc.+P-So urce.Feed.conc.+X.F.Suc+emission_O2+Thr+His+SO4.ferm.+PO4.u tilise; 0.249, 0.613, pH+Main.P-Source.Conc.+P-Source.Feed. conc.+Main.Thr.Conc.+X.F.Suc+X.VXdt+total.Vol+PL+emission_O 2+SO4.ferm.+PO4.utilise; 0.276, 0.613, Main.P-Source.Conc.+ P-Source.Feed.conc.+Nitrogen.Conc.+X.F.Suc+PL+rab+His+SO4.f erm.+PO4.utilise; 0.263, 0.613, Main.P-Source.Conc.+P-Sourc e.Feed.conc.+X.F.Suc+total.Vol+PL+rab+Thr+SO4.ferm.+PO4.fer m.+PO4.utilise; 0.263, 0.613, pH+Main.P-Source.Conc.+P-Sour ce.Feed.conc.+Lys_conc.+X.F.Suc+PL+rab+Thr+SO4.ferm.+PO4.ut ilise; 0.263, 0.613, pH+tmp+Main.P-Source.Conc.+P-Source.Fe ed.conc.+Nitrogen.Conc.+X.F.Suc+X.VXdt+PL+SO4.ferm.+PO4.uti lise; 0.262, 0.613, pH+Main.P-Source.Conc.+P-Source.Feed.co nc.+Main.Thr.Conc.+X.F.Suc+X.VXdt+PL+emission_O2+SO4.ferm.+ PO4.utilise; 0.276, 0.613, pH+tmp+Main.P-Source.Conc.+P-Sou rce.Feed.conc.+X.F.Suc+rab+Thr+SO4.ferm.+PO4.utilise; 0.300, 0.613, Main.P-Source.Conc.+P-Source.Feed.conc.+yield+PL+em ission_O2+SO4.ferm.+PO4.utilise; 0.288, 0.613, pH+Main.P-So urce.Conc.+P-Source.Feed.conc.+X.F.Suc+rab+SO4.ferm.+PO4.fe rm.+PO4.utilise; 0.249, 0.613, pH+Main.P-Source.Conc.+P-Sou rce.Feed.conc.+OD+X.F.Suc+X.VXdt+total.Vol+PL+rab+SO4.ferm. +PO4.utilise; 0.262, 0.613, Main.P-Source.Conc.+P-Source.Fe ed.conc.+X.F.Suc+X.VXdt+total.Vol+PL+emission_O2+SO4.ferm.+ PO4.ferm.+PO4.utilise; 0.275, 0.613, tmp+Main.P-Source.Conc. +P-Source.Feed.conc.+X.F.Suc+PL+Thr+SO4.ferm.+PO4.ferm.+PO4. utilise; 0.275, 0.613, Main.P-Source.Conc.+P-Source.Feed.co nc.+Main.Thr.Conc.+X.F.Suc+X.VXdt+PL+rab+SO4.ferm.+PO4.util ise; 0.262, 0.613, pH+Main.P-Source.Conc.+P-Source.Feed.con c.+Nitrogen.Conc.+X.F.Suc+total.Vol+PL+rab+SO4.ferm.+PO4.ut ilise; 0.288, 0.613, Main.P-Source.Conc.+P-Source.Feed.conc. +X.F.Suc+rab+Thr+SO4.ferm.+PO4.ferm.+PO4.utilise; 0.234, 0. 613, pH+Main.P-Source.Conc.+P-Source.Feed.conc.+X.F.Suc+X.V Xdt+total.Vol+PL+rab+Thr+lactate+SO4.ferm.+PO4.utilise; 0.2 88, 0.613, tmp+Main.P-Source.Conc.+P-Source.Feed.conc.+Nitr ogen.Conc.+X.F.Suc+PL+SO4.ferm.+PO4.utilise; 0.262, 0.613, Main.P-Source.Conc.+P-Source.Feed.conc.+OD+X.F.Suc+total.Vo l+total.cell+PL+emission_O2+SO4.ferm.+PO4.utilise; 0.275, 0. 613, pH+tmp+Main.P-Source.Conc.+P-Source.Feed.conc.+Nitroge n.Conc.+X.F.Suc+PL+SO4.ferm.+PO4.utilise; 0.275, 0.613, pH+ Main.P-Source.Conc.+P-Source.Feed.conc.+X.F.Suc+total.Vol+r ab+Thr+SO4.ferm.+PO4.utilise; 0.288, 0.613, pH+tmp+Main.P-S ource.Conc.+P-Source.Feed.conc.+X.F.Suc+emission_O2+SO4.fer m.+PO4.utilise; 0.288, 0.613, pH+tmp+Main.P-Source.Conc.+P-Source.Feed.conc.+X.F.Suc+His+SO4.ferm.+PO4.utilise; 0.275, 0.613, tmp+Main.P-Source.Conc.+P-Source.Feed.conc.+X.F.Suc +total.cell+PL+rab+SO4.ferm.+PO4.utilise; 0.288, 0.613, tmp +Main.P-Source.Conc.+P-Source.Feed.conc.+X.F.Suc+X.VXdt+rab +SO4.ferm.+PO4.utilise; 0.262, 0.613, Main.P-Source.Conc.+P -Source.Feed.conc.+Nitrogen.Conc.+X.F.Suc+X.VXdt+total.Vol+ PL+rab+SO4.ferm.+PO4.utilise; 0.300, 0.613, tmp+Main.P-Sour ce.Conc.+P-Source.Feed.conc.+X.F.Suc+X.VXdt+SO4.ferm.+PO4.u tilise; 0.262, 0.613, tmp+Main.P-Source.Conc.+P-Source.Feed. conc.+Nitrogen.Conc.+X.F.Suc+PL+rab+Thr+SO4.ferm.+PO4.utili se; 0.262, 0.613, pH+Main.P-Source.Conc.+P-Source.Feed.conc. +X.F.Suc+PL+rab+lactate+SO4.ferm.+PO4.ferm.+PO4.utilise; 0. 287, 0.613, Main.P-Source.Conc.+P-Source.Feed.conc.+X.F.Suc +PL+emission_O2+lactate+SO4.ferm.+PO4.utilise; 0.248, 0.612, pH+Main.P-Source.Conc.+P-Source.Feed.conc.+X.F.Suc+PL+rab+ Thr+lactate+SO4.ferm.+PO4.ferm.+PO4.utilise; 0.261, 0.612, Main.P-Source.Conc.+P-Source.Feed.conc.+X.F.Suc+PL+rab+Thr+ His+SO4.ferm.+PO4.ferm.+PO4.utilise; 0.275, 0.612, pH+Main. P-Source.Conc.+P-Source.Feed.conc.+yield+PL+rab+SO4.ferm.+P O4.ferm.+PO4.utilise; 0.323, 0.612, Main.P-Source.Conc.+P-S ource.Feed.conc.+yield+SO4.ferm.+PO4.utilise; 0.274, 0.612, tmp+Main.P-Source.Conc.+P-Source.Feed.conc.+X.F.Suc+X.VXdt +PL+SO4.ferm.+PO4.ferm.+PO4.utilise; 0.261, 0.612, Main.P-S ource.Conc.+P-Source.Feed.conc.+X.F.Suc+total.Vol+PL+rab+Hi s+SO4.ferm.+PO4.ferm.+PO4.utilise; 0.248, 0.612, tmp+Main.P -Source.Conc.+P-Source.Feed.conc.+X.F.Suc+total.Vol+PL+emis sion_O2+Thr+His+SO4.ferm.+PO4.utilise; 0.287, 0.612, Main.P -Source.Conc.+P-Source.Feed.conc.+X.F.Suc+X.VXdt+total.Vol+ rab+SO4.ferm.+PO4.utilise; 0.247, 0.612, pH+tmp+Main.P-Sour ce.Conc.+P-Source.Feed.conc.+Nitrogen.Conc.+X.F.Suc+X.VXdt+ PL+emission_O2+SO4.ferm.+PO4.utilise; 0.261, 0.612, pH+Main. P-Source.Conc.+P-Source.Feed.conc.+Nitrogen.Conc.+X.F.Suc+P L+rab+His+SO4.ferm.+PO4.utilise; 0.287, 0.612, Main.P-Sourc e.Conc.+P-Source.Feed.conc.+yield+PL+emission_O2+SO4.ferm.+ PO4.ferm.+PO4.utilise; 0.274, 0.612, Main.P-Source.Conc.+P-Source.Feed.conc.+Nitrogen.Conc.+X.F.Suc+PL+rab+SO4.ferm.+P O4.ferm.+PO4.utilise; 0.274, 0.612, Main.P-Source.Conc.+P-S ource.Feed.conc.+X.F.Suc+total.Vol+rab+Thr+His+SO4.ferm.+PO 4.utilise; 0.261, 0.612, tmp+Main.P-Source.Conc.+P-Source.F eed.conc.+X.F.Suc+PL+emission_O2+His+SO4.ferm.+PO4.ferm.+PO 4.utilise; 0.233, 0.612, pH+tmp+Main.P-Source.Conc.+P-Sourc e.Feed.conc.+X.F.Suc+X.VXdt+PL+rab+Thr+lactate+SO4.ferm.+PO 4.utilise; 0.261, 0.612, pH+Main.P-Source.Conc.+P-Source.Fe ed.conc.+X.F.Suc+X.VXdt+PL+rab+Thr+SO4.ferm.+PO4.utilise; 0. 233, 0.612, pH+tmp+Main.P-Source.Conc.+P-Source.Feed.conc.+ X.F.Suc+total.Vol+PL+rab+Thr+His+SO4.ferm.+PO4.utilise; 0.2 74, 0.612, tmp+Main.P-Source.Conc.+P-Source.Feed.conc.+X.F. Suc+total.Vol+rab+Thr+SO4.ferm.+PO4.utilise; 0.261, 0.612, tmp+Main.P-Source.Conc.+P-Source.Feed.conc.+X.F.Suc+X.VXdt+ PL+emission_O2+Thr+SO4.ferm.+PO4.utilise; 0.261, 0.612, Mai n.P-Source.Conc.+P-Source.Feed.conc.+X.F.Suc+X.VXdt+PL+rab+ Thr+lactate+SO4.ferm.+PO4.utilise; 0.233, 0.612, pH+Main.P-Source.Conc.+P-Source.Feed.conc.+OD+X.F.Suc+X.VXdt+total.Vo l+total.cell+PL+rab+SO4.ferm.+PO4.utilise; 0.247, 0.612, pH +Main.P-Source.Conc.+P-Source.Feed.conc.+X.F.Suc+total.Vol+ PL+emission_O2+Thr+His+SO4.ferm.+PO4.utilise; 0.274, 0.612, tmp+Main.P-Source.Conc.+P-Source.Feed.conc.+Nitrogen.Conc. +X.F.Suc+PL+emission_O2+SO4.ferm.+PO4.utilise; 0.261, 0.612, pH+tmp+Main.P-Source.Conc.+P-Source.Feed.conc.+X.F.Suc+tot al.cell+PL+rab+SO4.ferm.+PO4.utilise; 0.247, 0.612, pH+Main. P-Source.Conc.+P-Source.Feed.conc.+X.F.Suc+X.VXdt+total.Vol +PL+rab+lactate+SO4.ferm.+PO4.utilise; 0.260, 0.612, pH+Mai n.P-Source.Conc.+P-Source.Feed.conc.+OD+X.F.Suc+X.VXdt+PL+r ab+SO4.ferm.+PO4.utilise; 0.286, 0.612, tmp+Main.P-Source.C onc.+P-Source.Feed.conc.+X.F.Suc+total.Vol+emission_O2+SO4. ferm.+PO4.utilise; 0.260, 0.612, pH+Main.P-Source.Conc.+P-S ource.Feed.conc.+Lys_conc.+X.F.Suc+PL+emission_O2+Thr+SO4.f erm.+PO4.utilise; 0.247, 0.612, pH+tmp+Main.P-Source.Conc.+ P-Source.Feed.conc.+Main.Thr.Conc.+X.F.Suc+X.VXdt+PL+rab+SO 4.ferm.+PO4.utilise; 0.286, 0.612, tmp+Main.P-Source.Conc.+ P-Source.Feed.conc.+total.Vol+yield+PL+SO4.ferm.+PO4.utilis e; 0.260, 0.612, tmp+Main.P-Source.Conc.+P-Source.Feed.conc. +X.F.Suc+total.Vol+PL+Thr+His+SO4.ferm.+PO4.utilise; 0.273, 0.612, pH+tmp+Main.P-Source.Conc.+P-Source.Feed.conc.+tota l.Vol+yield+PL+SO4.ferm.+PO4.utilise; 0.260, 0.612, pH+tmp+ Main.P-Source.Conc.+P-Source.Feed.conc.+X.F.Suc+X.VXdt+tota l.Vol+PL+SO4.ferm.+PO4.utilise; 0.247, 0.612, pH+Main.P-Sou rce.Conc.+P-Source.Feed.conc.+X.F.Suc+total.Vol+PL+rab+His+ SO4.ferm.+PO4.ferm.+PO4.utilise; 0.298, 0.612, Main.P-Sourc e.Conc.+P-Source.Feed.conc.+Nitrogen.Conc.+X.F.Suc+emission _O2+SO4.ferm.+PO4.utilise; 0.246, 0.612, pH+Main.P-Source.C onc.+P-Source.Feed.conc.+Nitrogen.Conc.+X.F.Suc+X.VXdt+tota l.Vol+PL+emission_O2+SO4.ferm.+PO4.utilise; 0.260, 0.612, p H+Main.P-Source.Conc.+P-Source.Feed.conc.+Nitrogen.Conc.+X. F.Suc+PL+rab+SO4.ferm.+PO4.ferm.+PO4.utilise; 0.273, 0.612, Main.P-Source.Conc.+P-Source.Feed.conc.+X.F.Suc+X.VXdt+PL+ emission_O2+Thr+SO4.ferm.+PO4.utilise; 0.310, 0.612, tmp+Ma in.P-Source.Conc.+P-Source.Feed.conc.+yield+SO4.ferm.+PO4.u tilise; 0.246, 0.611, pH+tmp+Main.P-Source.Conc.+P-Source.F eed.conc.+X.F.Suc+X.VXdt+PL+rab+lactate+SO4.ferm.+PO4.utili se; 0.232, 0.611, pH+Main.P-Source.Conc.+P-Source.Feed.conc. +OD+X.F.Suc+total.Vol+total.cell+PL+rab+SO4.ferm.+PO4.ferm. +PO4.utilise; 0.273, 0.611, tmp+Main.P-Source.Conc.+P-Sourc e.Feed.conc.+Main.Thr.Conc.+X.F.Suc+PL+rab+SO4.ferm.+PO4.ut ilise; 0.310, 0.611, Main.P-Source.Conc.+P-Source.Feed.conc. +yield+rab+SO4.ferm.+PO4.utilise; 0.273, 0.611, Main.P-Sour ce.Conc.+P-Source.Feed.conc.+X.F.Suc+total.cell+PL+rab+SO4. ferm.+PO4.ferm.+PO4.utilise; 0.273, 0.611, tmp+Main.P-Sourc e.Conc.+P-Source.Feed.conc.+yield+PL+His+SO4.ferm.+PO4.ferm. +PO4.utilise; 0.246, 0.611, pH+Main.P-Source.Conc.+P-Source. Feed.conc.+X.F.Suc+X.VXdt+PL+emission_O2+Thr+lactate+SO4.fe rm.+PO4.utilise; 0.246, 0.611, pH+tmp+Main.P-Source.Conc.+P -Source.Feed.conc.+X.F.Suc+X.VXdt+total.cell+PL+rab+SO4.fer m.+PO4.utilise; 0.260, 0.611, pH+Main.P-Source.Conc.+P-Sour ce.Feed.conc.+X.F.Suc+X.VXdt+total.cell+PL+emission_O2+SO4. ferm.+PO4.utilise; 0.202, 0.611, pH+Main.P-Source.Conc.+P-S ource.Feed.conc.+Main.Thr.Conc.+OD+Lys_conc.+Cell+total.Vol +total.cell+yield+PL+rab+SO4.ferm.+PO4.utilise; 0.286, 0.61 1, pH+Main.P-Source.Conc.+P-Source.Feed.conc.+X.F.Suc+emiss ion_O2+SO4.ferm.+PO4.ferm.+PO4.utilise; 0.273, 0.611, tmp+M ain.P-Source.Conc.+P-Source.Feed.conc.+X.F.Suc+emission_O2+ Thr+His+SO4.ferm.+PO4.utilise; 0.259, 0.611, pH+Main.P-Sour ce.Conc.+P-Source.Feed.conc.+X.F.Suc+total.cell+PL+rab+SO4. ferm.+PO4.ferm.+PO4.utilise; 0.259, 0.611, tmp+Main.P-Sourc e.Conc.+P-Source.Feed.conc.+Nitrogen.Conc.+X.F.Suc+X.VXdt+P L+emission_O2+SO4.ferm.+PO4.utilise; 0.298, 0.611, tmp+Main. P-Source.Conc.+P-Source.Feed.conc.+total.Vol+yield+S04.ferm. +PO4.utilise; 0.272, 0.611, Main.P-Source.Conc.+P-Source.Fe ed.conc.+X.F.Suc+total.cell+PL+rab+Thr+SO4.ferm.+PO4.utilis e; 0.272, 0.611, Main.P-Source.Conc.+P-Source.Feed.conc.+X. F.Suc+X.VXdt+PL+rab+lactate+SO4.ferm.+PO4.utilise; 0.259, 0. 611, pH+Main.P-Source.Conc.+P-Source.Feed.conc.+Nitrogen.Co nc.+X.F.Suc+X.VXdt+PL+emission_O2+SO4.ferm.+PO4.utilise; 0. 272, 0.611, Main.P-Source.Conc.+P-Source.Feed.conc.+Nitroge n.Conc.+X.F.Suc+PL+emission_O2+His+SO4.ferm.+PO4.utilise; 0. 272, 0.611, tmp+Main.P-Source.Conc.+P-Source.Feed.conc.+Nit rogen.Conc.+yield+PL+SO4.ferm.+PO4.ferm.+PO4.utilise; 0.272, 0.611, Main.P-Source.Conc.+P-Source.Feed.conc.+Lys_conc.+X. F.Suc+PL+emission_O2+His+SO4.ferm.+PO4.utilise; 0.245, 0.61 1, pH+tmp+Main.P-Source.Conc.+P-Source.Feed.conc.+X.F.Suc+X. VXdt+total.Vol+PL+emission_O2+SO4.ferm.+PO4.utilise; 0.245, 0.611, pH+Main.P-Source.Conc.+P-Source.Feed.conc.+X.F.Suc+ total.Vol+PL+rab+Thr+SO4.ferm.+PO4.ferm.+PO4.utilise; 0.245, 0.611, pH+tmp+Main.P-Source.Conc.+P-Source.Feed.conc.+X.F. Suc+X.VXdt+PL+rab+Thr+SO4.ferm.+PO4.utilise; 0.259, 0.611, pH+tmp+Main.P-Source.Conc.+P-Source.Feed.conc.+yield+PL+His +SO4.ferm.+PO4.ferm.+PO4.utilise; 0.297, 0.611, tmp+Main.P-Source.Conc.+P-Source.Feed.conc.+X.F.Suc+SO4.ferm.+PO4.ferm. +PO4.utilise; 0.272, 0.611, tmp+Main.P-Source.Conc.+P-Sourc e.Feed.conc.+Main.Thr.Conc.+X.F.Suc+PL+emission_O2+SO4.ferm. +PO4.utilise; 0.285, 0.611, tmp+Main.P-Source.Conc.+P-Sourc e.Feed.conc.+X.F.Suc+total.Vol+Thr+SO4.ferm.+PO4.utilise; 0. 309, 0.611, Main.P-Source.Conc.+P-Source.Feed.conc.+yield+e mission_O2+SO4.ferm.+PO4.utilise; 0.285, 0.611, Main.P-Sour ce.Conc.+P-Source.Feed.conc.+X.F.Suc+rab+His+SO4.ferm.+PO4. ferm.+PO4.utilise; 0.285, 0.611, pH+Main.P-Source.Conc.+P-S ource.Feed.conc.+X.F.Suc+X.VXdt+emission_O2+SO4.ferm.+PO4.u tilise; 0.245, 0.611, pH+tmp+Main.P-Source.Conc.+P-Source.F eed.conc.+X.F.Suc+PL+emission_O2+Thr+SO4.ferm.+PO4.ferm.+PO 4.utilise; 0.245, 0.611, pH+tmp+Main.P-Source.Conc.+P-Sourc e.Feed.conc.+X.F.Suc+PL+emission_O2+His+SO4.ferm.+PO4.ferm. +PO4.utilise; 0.259, 0.611, pH+Main.P-Source.Conc.+P-Source. Feed.conc.+Main.Thr.Conc.+X.F.Suc+PL+rab+SO4.ferm.+PO4.ferm. +PO4.utilise; 0.272, 0.611, Main.P-Source.Conc.+P-Source.Fe ed.conc.+OD+X.F.Suc+X.VXdt+PL+rab+SO4.ferm.+PO4.utilise; 0. 259, 0.610, pH+tmp+Main.P-Source.Conc.+P-Source.Feed.conc.+ X.F.Suc+PL+Thr+SO4.ferm.+PO4.ferm.+PO4.utilise; 0.320, 0.61 0, Main.P-Source.Conc.+P-Source.Feed.conc.+X.F.Suc+SO4.ferm. +PO4.utilise; 0.272, 0.610, pH+Main.P-Source.Conc.+P-Source. Feed.conc.+X.F.Suc+total.Vol+rab+His+SO4.ferm.+PO4.utilise; 0.272, 0.610, Main.P-Source.Conc.+P-Source.Feed.conc.+Nitr ogen.Conc.+X.F.Suc+total.Vol+PL+emission_O2+SO4.ferm.+PO4.u tilise; 0.272, 0.610, tmp+Main.P-Source.Conc.+P-Source.Feed. conc.+Nitrogen.Conc.+X.F.Suc+X.VXdt+PL+SO4.ferm.+PO4.utilis e; 0.309, 0.610, Main.P-Source.Conc.+P-Source.Feed.conc.+X. F.Suc+PL+SO4.ferm.+PO4.utilise; 0.258, 0.610, tmp+Main.P-So urce.Conc.+P-Source.Feed.conc.+Main.Thr.Conc.+X.F.Suc+PL+ra b+Thr+SO4.ferm.+PO4.utilise; 0.230, 0.610, pH+tmp+Main.P-So urce.Conc.+P-Source.Feed.conc.+X.F.Suc+X.VXdt+PL+rab+His+SO 4.ferm.+PO4.ferm.+PO4.utilise; 0.245, 0.610, pH+Main.P-Sour ce.Conc.+P-Source.Feed.conc.+X.F.Suc+X.VXdt+PL+emission_O2+ His+SO4.ferm.+PO4.ferm.+PO4.utilise; 0.245, 0.610, pH+Main. P-Source.Conc.+P-Source.Feed.conc.+X.F.Suc+X.VXdt+total.Vol +PL+rab+Thr+SO4.ferm.+PO4.utilise; 0.272, 0.610, Main.P-Sou rce.Conc.+P-Source.Feed.conc.+Nitrogen.Conc.+X.F.Suc+X.VXdt +PL+emission_O2+SO4.ferm.+PO4.utilise; 0.258, 0.610, Main.P -Source.Conc.+P-Source.Feed.conc.+X.F.Suc+X.VXdt+PL+rab+Thr +His+SO4.ferm.+PO4.utilise; 0.244, 0.610, pH+tmp+Main.P-Sou rce.Conc.+P-Source.Feed.conc.+Main.Thr.Conc.+X.F.Suc+X.VXdt +PL+emission_O2+SO4.ferm.+PO4.utilise; 0.271, 0.610, Main.P -Source.Conc.+P-Source.Feed.conc.+X.F.Suc+total.cell+PL+rab +His+SO4.ferm.+PO4.utilise; 0.284, 0.610, pH+Main.P-Source. Conc.+P-Source.Feed.conc.+yield+PL+rab+SO4.ferm.+PO4.utilis e; 0.284, 0.610, Main.P-Source.Conc.+P-Source.Feed.conc.+OD +X.F.Suc+PL+emission_O2+SO4.ferm.+PO4.utilise; 0.271, 0.610, Main.P-Source.Conc.+P-Source.Feed.conc.+X.F.Suc+X.VXdt+tot al.cell+PL+emission_O2+SO4.ferm.+PO4.utilise; 0.271, 0.610, Main.P-Source.Conc.+P-Source.Feed.conc.+X.F.Suc+PL+rab+Thr +lactate+SO4.ferm.+PO4.utilise; 0.284, 0.610, tmp+Main.P-So urce.Conc.+P-Source.Feed.conc.+X.F.Suc+total.Vol+His+SO4.fe rm.+PO4.utilise; 0.271, 0.610, Main.P-Source.Conc.+P-Source. Feed.conc.+Main.Thr.Conc.+X.F.Suc+X.VXdt+PL+emission_O2+SO4. ferm.+PO4.utilise; 0.271, 0.610, Main.P-Source.Conc.+P-Sour ce.Feed.conc.+X.F.Suc+PL+emission_O2+Thr+lactate+SO4.ferm.+ PO4.utilise; 0.258, 0.610, pH+tmp+Main.P-Source.Conc.+P-Sou rce.Feed.conc.+Main.Thr.Conc.+X.F.Suc+PL+rab+SO4.ferm.+PO4. utilise; 0.230, 0.610, pH+Main.P-Source.Conc.+P-Source.Feed. conc.+X.F.Suc+X.VXdt+total.Vol+PL+rab+His+SO4.ferm.+PO4.fer m.+PO4.utilise; 0.284, 0.610, tmp+Main.P-Source.Conc.+P-Sou rce.Feed.conc.+X.F.Suc+rab+SO4.ferm.+PO4.ferm.+PO4.utilise; 0.258, 0.610, pH+Main.P-Source.Conc.+P-Source.Feed.conc.+X. F.Suc+X.VXdt+PL+emission_O2+lactate+SO4.ferm.+PO4.utilise; 0.258, 0.610, pH+tmp+Main.P-Source.Conc.+P-Source.Feed.conc. +Nitrogen.Conc.+X.F.Suc+PL+emission_O2+SO4.ferm.+PO4.utilis e; 0.271, 0.610, tmp+Main.P-Source.Conc.+P-Source.Feed.conc. +X.F.Suc+X.VXdt+PL+Thr+SO4.ferm.+PO4.utilise; 0.271, 0.610, Main.P-Source.Conc.+P-Source.Feed.conc.+Lys_conc.+X.F.Suc+ PL+rab+His+SO4.ferm.+PO4.utilise; 0.296, 0.610, tmp+Main.P-Source.Conc.+P-Source.Feed.conc.+yield+PL+SO4.ferm.+PO4.uti lise; 0.284, 0.610, Main.P-Source.Conc.+P-Source.Feed.conc. +X.F.Suc+X.VXdt+emission_O2+His+SO4.ferm.+PO4.utilise; 0.25 8, 0.610, Main.P-Source.Conc.+P-Source.Feed.conc.+X.F.Suc+X. VXdt+PL+emission_O2+His+SO4.ferm.+PO4.ferm.+PO4.utilise; 0. 271, 0.610, Main.P-Source.Conc.+P-Source.Feed.conc.+Main.Th r.Conc.+X.F.Suc+PL+rab+SO4.ferm.+PO4.ferm.+PO4.utilise; 0.2 96, 0.610, Main.P-Source.Conc.+P-Source.Feed.conc.+Lys_conc. +X.F.Suc+emission_O2+SO4.ferm.+PO4.utilise; 0.244, 0.610, p H+tmp+Main.P-Source.Conc.+P-Source.Feed.conc.+Nitrogen.Conc. +X.F.Suc+PL+rab+Thr+SO4.ferm.+PO4.utilise; 0.284, 0.610, Ma in.P-Source.Conc.+P-Source.Feed.conc.+X.F.Suc+emission_O2+T hr+SO4.ferm.+PO4.ferm.+PO4.utilise; 0.284, 0.610, pH+tmp+Ma in.P-Source.Conc.+P-Source.Feed.conc.+X.F.Suc+total.Vol+SO4. ferm.+PO4.utilise; 0.271, 0.610, pH+tmp+Main.P-Source.Conc. +P-Source.Feed.conc.+X.F.Suc+rab+His+SO4.ferm.+PO4.utilise; 0.284, 0.610, Main.P-Source.Conc.+P-Source.Feed.conc.+X.F. Suc+X.VXdt+total.Vol+emission_O2+SO4.ferm.+PO4.utilise; 0.2 71, 0.610, Main.P-Source.Conc.+P-Source.Feed.conc.+X.F.Suc+ PL+rab+lactate+SO4.ferm.+PO4.ferm.+PO4.utilise; 0.257, 0.61 0, pH+tmp+Main.P-Source.Conc.+P-Source.Feed.conc.+Main.Thr. Conc.+X.F.Suc+PL+emission_O2+SO4.ferm.+PO4.utilise; 0.257, 0.610, pH+tmp+Main.P-Source.Conc.+P-Source.Feed.conc.+X.F.S uc+X.VXdt+total.cell+PL+SO4.ferm.+PO4.utilise; 0.296, 0.610, Main.P-Source.Conc.+P-Source.Feed.conc.+X.F.Suc+rab+lactat e+SO4.ferm.+PO4.utilise; 0.257, 0.610, tmp+Main.P-Source.Co nc.+P-Source.Feed.conc.+Main.Thr.Conc.+X.F.Suc+PL+emission O2+Thr+SO4.ferm.+PO4.utilise; 0.271, 0.610, pH+tmp+Main.P-S ource.Conc.+P-Source.Feed.conc.+X.F.Suc+total.cell+PL+SO4.f erm.+PO4.utilise; 0.257, 0.610, tmp+Main.P-Source.Conc.+P-S ource.Feed.conc.+X.F.Suc+X.VXdt+total.Vol+PL+emission_O2+SO 4.ferm.+PO4.utilise; 0.271, 0.610, Main.P-Source.Conc.+P-So urce.Feed.conc.+yield+PL+rab+His+SO4.ferm.+PO4.ferm.+PO4.ut ilise; 0.257, 0.610, pH+tmp+Main.P-Source.Conc.+P-Source.Fe ed.conc.+Nitrogen.Conc.+yield+PL+SO4.ferm.+PO4.ferm.+PO4.ut ilise; 0.257, 0.610, Main.P-Source.Conc.+P-Source.Feed.conc. +X.F.Suc+X.VXdt+total.Vol+total.cell+PL+rab+SO4.ferm.+PO4.u tilise; 0.257, 0.610, pH+Main.P-Source.Conc.+P-Source.Feed. conc.+X.F.Suc+total.cell+PL+rab+His+SO4.ferm.+PO4.utilise; 0.270, 0.609, pH+tmp+Main.P-Source.Conc.+P-Source.Feed.conc. +X.F.Suc+emission_O2+Thr+SO4.ferm.+PO4.utilise; 0.257, 0.60 9, pH+Main.P-Source.Conc.+P-Source.Feed.conc.+X.F.Suc+Cell+ X.VXdt+PL+rab+SO4.ferm.+PO4.utilise; 0.270, 0.609, tmp+Main. P-Source.Conc.+P-Source.Feed.conc.+Nitrogen.Conc.+X.F.Suc+P L+Thr+SO4.ferm.+PO4.utilise; 0.257, 0.609, Main.P-Source.Co nc.+P-Source.Feed.conc.+X.F.Suc+total.Vol+PL+emission_O2+Th r+SO4.ferm.+PO4.ferm.+PO4.utilise; 0.296, 0.609, pH+Main.P-Source.Conc.+P-Source.Feed.conc.+X.F.Suc+PL+SO4.ferm.+PO4.u tilise; 0.257, 0.609, tmp+Main.P-Source.Conc.+P-Source.Feed. conc.+X.F.Suc+X.VXdt+total.cell+PL+rab+SO4.ferm.+PO4.utilis e; 0.283, 0.609, Main.P-Source.Conc.+P-Source.Feed.conc.+to tal.Vol+yield+PL+rab+SO4.ferm.+PO4.utilise; 0.257, 0.609, p H+Main.P-Source.Conc.+P-Source.Feed.conc.+Nitrogen.Conc.+X. F.Suc+PL+emission_O2+His+SO4.ferm.+PO4.utilise; 0.257, 0.60 9, Main.P-Source.Conc.+P-Source.Feed.conc.+X.F.Suc+total.Vo l+PL+emission_O2+His+SO4.ferm.+PO4.ferm.+PO4.utilise; 0.229, 0.609, pH+tmp+Main.P-Source.Conc.+P-Source.Feed.conc.+X.F. Suc+X.VXdt+total.Vol+PL+rab+His+SO4.ferm.+PO4.utilise; 0.27 0, 0.609, pH+Main.P-Source.Conc.+P-Source.Feed.conc.+yield+ PL+emission_O2+SO4.ferm.+PO4.ferm.+PO4.utilise; 0.307, 0.60 9, Main.P-Source.Conc.+P-Source.Feed.conc.+yield+His+SO4.fe rm.+PO4.utilise; 0.243, 0.609, tmp+Main.P-Source.Conc.+P-So urce.Feed.conc.+X.F.Suc+X.VXdt+PL+rab+His+SO4.ferm.+PO4.fer m.+PO4.utilise; 0.270, 0.609, pH+Main.P-Source.Conc.+P-Sour ce.Feed.conc.+OD+X.F.Suc+PL+emission_O2+SO4.ferm.+PO4.utili se; 0.257, 0.609, Main.P-Source.Conc.+P-Source.Feed.conc.+X. F.Suc+X.VXdt+PL+rab+Thr+SO4.ferm.+PO4.ferm.+PO4.utilise; 0. 283, 0.609, pH+Main.P-Source.Conc.+P-Source.Feed.conc.+yiel d+PL+emission_O2+SO4.ferm.+PO4.utilise; 0.283, 0.609, tmp+M ain.P-Source.Conc.+P-Source.Feed.conc.+total.Vol+yield+His+ SO4.ferm.+PO4.utilise; 0.283, 0.609, Main.P-Source.Conc.+P-Source.Feed.conc.+X.F.Suc+X.VXdt+rab+SO4.ferm.+PO4.ferm.+PO 4.utilise; 0.270, 0.609, pH+tmp+Main.P-Source.Conc.+P-Sourc e.Feed.conc.+Main.Thr.Conc.+X.F.Suc+PL+SO4.ferm.+PO4.utilis e; 0.257, 0.609, pH+Main.P-Source.Conc.+P-Source.Feed.conc. +Nitrogen.Conc.+X.F.Suc+total.Vol+PL+emission_O2+SO4.ferm.+ PO4.utilise; 0.270, 0.609, tmp+Main.P-Source.Conc.+P-Source. Feed.conc.+X.F.Suc+total.Vol+rab+His+SO4.ferm.+PO4.utilise; 0.283, 0.609, tmp+Main.P-Source.Conc.+P-Source.Feed.conc.+ Main.Thr.Conc.+X.F.Suc+PL+SO4.ferm.+PO4.utilise; 0.243, 0.6 09, pH+tmp+Main.P-Source.Conc.+P-Source.Feed.conc.+X.F.Suc+ total.Vol+PL+Thr+His+SO4.ferm.+PO4.utilise; 0.243, 0.609, t mp+Main.P-Source.Conc.+P-Source.Feed.conc.+X.F.Suc+X.VXdt+P L+rab+Thr+His+SO4.ferm.+PO4.utilise; 0.283, 0.609, Main.P-S ource.Conc.+P-Source.Feed.conc.+Nitrogen.Conc.+X.F.Suc+rab+ Thr+SO4.ferm.+PO4.utilise; 0.295, 0.609, Main.P-Source.Conc. +P-Source.Feed.conc.+Main.Thr.Conc.+X.F.Suc+rab+SO4.ferm.+P O4.utilise; 0.283, 0.609, Main.P-Source.Conc.+P-Source.Feed. conc.+total.Vol+yield+PL+emission_O2+SO4.ferm.+PO4.utilise; 0.270, 0.609, pH+Main.P-Source.Conc.+P-Source.Feed.conc.+X. F.Suc+total.Vol+emission_O2+Thr+SO4.ferm.+PO4.utilise; 0.22 9, 0.609, pH+tmp+Main.P-Source.Conc.+P-Source.Feed.conc.+X. F.Suc+X.VXdt+PL+emission_O2+Thr+lactate+SO4.ferm.+PO4.utili se; 0.257, 0.609, pH+Main.P-Source.Conc.+P-Source.Feed.conc. +X.F.Suc+PL+rab+His+lactate+SO4.ferm.+PO4.utilise; 0.295, 0. 609, tmp+Main.P-Source.Conc.+P-Source.Feed.conc.+yield+SO4. ferm.+PO4.ferm.+PO4.utilise; 0.256, 0.609, tmp+Main.P-Sourc e.Conc.+P-Source.Feed.conc.+Nitrogen.Conc.+X.F.Suc+PL+emiss ion_O2+Thr+SO4.ferm.+PO4.utilise; 0.283, 0.609, pH+tmp+Main. P-Source.Conc.+P-Source.Feed.conc.+yield+PL+SO4.ferm.+PO4.u tilise; 0.295, 0.609, Main.P-Source.Conc.+P-Source.Feed.con c.+Main.Thr.Conc.+X.F.Suc+emission_O2+SO4.ferm.+PO4.utilis e; 0.256, 0.609, pH+Main.P-Source.Conc.+P-Source.Feed.conc. +Lys_conc.+X.F.Suc+PL+emission_O2+His+SO4.ferm.+PO4.utilis e; 0.243, 0.609, pH+Main.P-Source.Conc.+P-Source.Feed.conc. +X.F.Suc+X.VXdt+total.Vol+total.cell+PL+emission_O2+SO4.fer m.+PO4.utilise; 0.228, 0.609, pH+Main.P-Source.Conc.+P-Sour ce.Feed.conc.+X.F.Suc+X.VXdt+PL+rab+Thr+His+lactate+SO4.fer m.+PO4.utilise; 0.243, 0.609, pH+Main.P-Source.Conc.+P-Sour ce.Feed.conc.+X.F.Suc+PL+rab+Thr+His+SO4.ferm.+PO4.ferm.+PO 4.utilise; 0.270, 0.609, pH+Main.P-Source.Conc.+P-Source.Fe ed.conc.+X.F.Suc+total.Vol+PL+SO4.ferm.+PO4.ferm.+PO4.utili se; 0.242, 0.609, pH+Main.P-Source.Conc.+P-Source.Feed.conc. +X.F.Suc+PL+emission_O2+Thr+lactate+SO4.ferm.+PO4.ferm.+PO4. utilise; 0.256, 0.609, tmp+Main.P-Source.Conc.+P-Source.Fee d.conc.+Nitrogen.Conc.+X.F.Suc+total.Vol+PL+rab+SO4.ferm.+P O4.utilise; 0.256, 0.609, tmp+Main.P-Source.Conc.+P-Source. Feed.conc.+X.F.Suc+total.Vol+PL+rab+SO4.ferm.+PO4.ferm.+PO4. utilise; 0.228, 0.609, pH+Main.P-Source.Conc.+P-Source.Feed. conc.+X.F.Suc+X.VXdt+total.Vol+PL+emission_O2+Thr+lactate+S O4.ferm.+PO4.utilise; 0.228, 0.609, pH+Main.P-Source.Conc.+ P-Source.Feed.conc.+OD+X.F.Suc+total.Vol+total.cell+PL+rab+ lactate+SO4.ferm.+PO4.utilise; 0.282, 0.609, tmp+Main.P-Sou rce.Conc.+P-Source.Feed.conc.+X.F.Suc+total.cell+PL+SO4.fer m.+PO4.utilise; 0.282, 0.609, tmp+Main.P-Source.Conc.+P-Sou rce.Feed.conc.+X.F.Suc+X.VXdt+emission_O2+SO4.ferm.+PO4.uti lise; 0.256, 0.609, Main.P-Source.Conc.+P-Source.Feed.conc. +OD+X.F.Suc+X.VXdt+total.Vol+PL+rab+SO4.ferm.+PO4.utilise; 0.256, 0.609, pH+Main.P-Source.Conc.+P-Source.Feed.conc.+X. F.Suc+total.cell+PL+rab+Thr+SO4.ferm.+PO4.utilise; 0.256, 0. 609, pH+Main.P-Source.Conc.+P-Source.Feed.conc.+Lys_conc.+X. F.Suc+PL+rab+His+SO4.ferm.+PO4.utilise; 0.242, 0.609, tmp+M ain.P-Source.Conc.+P-Source.Feed.conc.+X.F.Suc+X.VXdt+total. Vol+PL+rab+His+SO4.ferm.+PO4.utilise; 0.269, 0.609, tmp+Mai n.P-Source.Conc.+P-Source.Feed.conc.+X.F.Suc+X.VXdt+total.V ol+PL+SO4.ferm.+PO4.utilise; 0.256, 0.609, Main.P-Source.Co nc.+P-Source.Feed.conc.+Nitrogen.Conc.+X.F.Suc+total.Vol+PL +rab+Thr+SO4.ferm.+PO4.utilise; 0.256, 0.609, Main.P-Source. Conc.+P-Source.Feed.conc.+X.F.Suc+X.VXdt+total.Vol+PL+emiss ion_O2+Thr+SO4.ferm.+PO4.utilise; 0.256, 0.609, tmp+Main.P-Source.Conc.+P-Source.Feed.conc.+Main.Thr.Conc.+X.F.Suc+X.V Xdt+PL+rab+SO4.ferm.+PO4.utilise; 0.228, 0.609, pH+tmp+Main. P-Source.Conc.+P-Source.Feed.conc.+X.F.Suc+total.Vol+PL+emi ssion_O2+Thr+His+SO4.ferm.+PO4.utilise; 0.242, 0.608, pH+Ma in.P-Source.Conc.+P-Source.Feed.conc.+X.F.Suc+X.VXdt+PL+rab +His+lactate+SO4.ferm.+PO4.utilise; 0.242, 0.608, pH+tmp+Ma in.P-Source.Conc.+P-Source.Feed.conc.+Main.Thr.Conc.+X.F.Su c+PL+rab+Thr+SO4.ferm.+PO4.utilise; 0.282, 0.608, Main.P-So urce.Conc.+P-Source.Feed.conc.+X.F.Suc+emission_O2+His+SO4. ferm.+PO4.ferm.+PO4.utilise; 0.269, 0.608, pH+Main.P-Source. Conc.+P-Source.Feed.conc.+X.F.Suc+X.VXdt+total.Vol+PL+SO4.f erm.+PO4.utilise; 0.294, 0.608, Main.P-Source.Conc.+P-Sourc e.Feed.conc.+Lys_conc.+X.F.Suc+rab+SO4.ferm.+PO4.utilise; 0. 256, 0.608, pH+Main.P-Source.Conc.+P-Source.Feed.conc.+X.F. Suc+total.Vol+total.cell+PL+rab+SO4.ferm.+PO4.utilise; 0.26 9, 0.608, Main.P-Source.Conc.+P-Source.Feed.conc.+X.F.Suc+t otal.Vol+emission_O2+Thr+His+SO4.ferm.+PO4.utilise; 0.294, 0.608, tmp+Main.P-Source.Conc.+P-Source.Feed.conc.+yield+Hi s+SO4.ferm.+PO4.utilise; 0.256, 0.608, pH+Main.P-Source.Con c.+P-Source.Feed.conc.+Main.Thr.Conc.+X.F.Suc+PL+emission_O 2+SO4.ferm.+PO4.ferm.+PO4.utilise; 0.242, 0.608, pH+Main.P-Source.Conc.+P-Source.Feed.conc.+X.F.Suc+total.Vol+PL+emiss ion_O2+His+SO4.ferm.+PO4.ferm.+PO4.utilise; 0.269, 0.608, t mp+Main.P-Source.Conc.+P-Source.Feed.conc.+X.F.Suc+total.Vo l+emission_O2+Thr+SO4.ferm.+PO4.utilise; 0.269, 0.608, Main. P-Source.Conc.+P-Source.Feed.conc.+X.F.Suc+total.Vol+total. cell+PL+rab+SO4.ferm.+PO4.utilise; 0.269, 0.608, tmp+Main.P -Source.Conc.+P-Source.Feed.conc.+total.Vol+yield+PL+His+SO 4.ferm.+PO4.utilise; 0.255, 0.608, pH+tmp+Main.P-Source.Con c.+P-Source.Feed.conc.+Nitrogen.Conc.+X.F.Suc+PL+Thr+SO4.fe rm.+PO4.utilise; 0.269, 0.608, Main.P-Source.Conc.+P-Source. Feed.conc.+X.F.Suc+Cell+total.Vol+PL+rab+SO4.ferm.+PO4.util ise; 0.269, 0.608, Main.P-Source.Conc.+P-Source.Feed.conc.+ X.F.Suc+Cell+X.VXdt+PL+rab+SO4.ferm.+PO4.utilise; 0.241, 0. 608, tmp+Main.P-Source.Conc.+P-Source.Feed.conc.+X.F.Suc+PL +rab+Thr+His+SO4.ferm.+PO4.ferm.+PO4.utilise; 0.281, 0.608, Main.P-Source.Conc.+P-Source.Feed.conc.+X.F.Suc+X.VXdt+rab +Thr+SO4.ferm.+PO4.utilise; 0.294, 0.608, Main.P-Source.Con c.+P-Source.Feed.conc.+X.F.Suc+total.cell+rab+SO4.ferm.+PO4. utilise; 0.241, 0.608, pH+Main.P-Source.Conc.+P-Source.Feed. conc.+OD+X.F.Suc+X.VXdt+total.Vol+PL+emission_O2+SO4.ferm.+ PO4.utilise; 0.268, 0.608, Main.P-Source.Conc.+P-Source.Fee d.conc.+Nitrogen.Conc.+X.F.Suc+PL+emission_O2+SO4.ferm.+PO4. ferm.+PO4.utilise; 0.281, 0.608, Main.P-Source.Conc.+P-Sour ce.Feed.conc.+X.F.Suc+total.Vol+rab+SO4.ferm.+PO4.ferm.+PO4. utilise; 0.255, 0.608, Main.P-Source.Conc.+P-Source.Feed.co nc.+Main.Thr.Conc.+X.F.Suc+X.VXdt+total.Vol+PL+rab+SO4.ferm. +PO4.utilise; 0.255, 0.608, pH+Main.P-Source.Conc.+P-Source. Feed.conc.+X.F.Suc+Cell+total.Vol+PL+rab+SO4.ferm.+PO4.util ise; 0.212, 0.608, pH+Main.P-Source.Conc.+P-Source.Feed.con c.+OD+X.F.Suc+total.Vol+total.cell+PL+rab+Thr+lactate+SO4.f erm.+PO4.utilise; 0.241, 0.608, pH+tmp+Main.P-Source.Conc.+ P-Source.Feed.conc.+X.F.Suc+X.VXdt+PL+emission_O2+lactate+S O4.ferm.+PO4.utilise; 0.255, 0.608, Main.P-Source.Conc.+P-S ource.Feed.conc.+Nitrogen.Conc.+X.F.Suc+X.VXdt+total.Vol+PL +emission_O2+SO4.ferm.+PO4.utilise; 0.281, 0.608, Main.P-So urce.Conc.+P-Source.Feed.conc.+Lys_conc.+yield+PL+emission_ O2+SO4.ferm.+PO4.utilise; 0.255, 0.608, Main.P-Source.Conc. +P-Source.Feed.conc.+X.F.Suc+X.VXdt+PL+emission_O2+Thr+lact ate+SO4.ferm.+PO4.utilise; 0.268, 0.608, Main.P-Source.Conc. +P-Source.Feed.conc.+Main.Thr.Conc.+X.F.Suc+PL+emission_O2+ SO4.ferm.+PO4.ferm.+PO4.utilise; 0.268, 0.608, pH+Main.P-So urce.Conc.+P-Source.Feed.conc.+X.F.Suc+total.Vol+emission_O 2+His+SO4.ferm.+PO4.utilise; 0.241, 0.608, pH+tmp+Main.P-So urce.Conc.+P-Source.Feed.conc.+Main.Thr.Conc.+X.F.Suc+PL+em ission_O2+Thr+SO4.ferm.+PO4.utilise; 0.268, 0.608, pH+tmp+M ain.P-Source.Conc.+P-Source.Feed.conc.+X.F.Suc+total.Vol+ra b+SO4.ferm.+PO4.utilise; 0.293, 0.608, Main.P-Source.Conc.+ P-Source.Feed.conc.+X.F.Suc+emission_O2+lactate+SO4.ferm.+P O4.utilise; 0.305, 0.608, Main.P-Source.Conc.+P-Source.Feed. conc.+yield+PL+SO4.ferm.+PO4.utilise; 0.255, 0.608, pH+tmp+ Main.P-Source.Conc.+P-Source.Feed.conc.+total.Vol+yield+PL+ His+SO4.ferm.+PO4.utilise; 0.293, 0.608, Main.P-Source.Conc. +P-Source.Feed.conc.+yield+rab+His+SO4.ferm.+PO4.utilise; 0. 255, 0.608, Main.P-Source.Conc.+P-Source.Feed.conc.+Nitroge n.Conc.+X.F.Suc+PL+rab+Thr+His+SO4.ferm.+PO4.utilise; 0.241, 0.608, pH+tmp+Main.P-Source.Conc.+P-Source.Feed.conc.+X.F. Suc+total.Vol+PL+rab+SO4.ferm.+PO4.ferm.+PO4.utilise; 0.255, 0.608, Main.P-Source.Conc.+P-Source.Feed.conc.+X.F.Suc+PL+ rab+Thr+lactate+SO4.ferm.+PO4.ferm.+PO4.utilise; 0.268, 0.6 08, tmp+Main.P-Source.Conc.+P-Source.Feed.conc.+X.F.Suc+tot al.cell+PL+emission_O2+SO4.ferm.+PO4.utilise; 0.196, 0.608, pH+Main.P-Source.Conc.+P-Source.Feed.conc.+Main.Thr.Conc.+ OD+Lys_conc.+Cell+total.Vol+total.cell+yield+PL+emission_O2 +SO4.ferm.+PO4.utilise; 0.241, 0.608, pH+Main.P-Source.Conc. +P-Source.Feed.conc.+X.F.Suc+Cell+X.VXdt+total.Vol+PL+rab+S O4.ferm.+PO4.utilise; 0.293, 0.608, Main.P-Source.Conc.+P-S ource.Feed.conc.+total.Vol+yield+rab+SO4.ferm.+PO4.utilise; 0.268, 0.608, Main.P-Source.Conc.+P-Source.Feed.conc.+X.F. Suc+Cell+PL+rab+His+SO4.ferm.+PO4.utilise; 0.254, 0.608, pH +Main.P-Source.Conc.+P-Source.Feed.conc.+X.F.Suc+PL+emissio n_O2+lactate+SO4.ferm.+PO4.ferm.+PO4.utilise; 0.281, 0.608, Main.P-Source.Conc.+P-Source.Feed.conc.+X.F.Suc+total.Vol+ PL+SO4.ferm.+PO4.ferm.+PO4.utilise; 0.241, 0.608, Main.P-So urce.Conc.+P-Source.Feed.conc.+OD+X.F.Suc+total.Vol+total.c ell+PL+rab+SO4.ferm.+PO4.ferm.+PO4.utilise; 0.281, 0.608, M ain.P-Source.Conc.+P-Source.Feed.conc.+yield+PL+rab+His+SO4. ferm.+PO4.utilise; 0.281, 0.608, Main.P-Source.Conc.+P-Sour ce.Feed.conc.+Lys_conc.+yield+PL+rab+SO4.ferm.+PO4.utilise; 0.268, 0.608, Main.P-Source.Conc.+P-Source.Feed.conc.+Main. Thr.Conc.+X.F.Suc+PL+rab+His+SO4.ferm.+PO4.utilise; 0.226, 0.608, pH+Main.P-Source.Conc.+P-Source.Feed.conc.+OD+X.F.Su c+total.Vol+total.cell+PL+rab+His+SO4.ferm.+PO4.utilise; 0. 305, 0.607, Main.P-Source.Conc.+P-Source.Feed.conc.+yield+S O4.ferm.+PO4.ferm.+PO4.utilise; 0.268, 0.607, pH+tmp+Main.P -Source.Conc.+P-Source.Feed.conc.+X.F.Suc+Thr+His+SO4.ferm. +PO4.utilise; 0.240, 0.607, pH+Main.P-Source.Conc.+P-Source. Feed.conc.+Nitrogen.Conc.+X.F.Suc+X.VXdt+PL+rab+SO4.ferm.+P O4.ferm.+PO4.utilise; 0.293, 0.607, Main.P-Source.Conc.+P-S ource.Feed.conc.+total.Vol+yield+emission_O2+SO4.ferm.+PO4. utilise; 0.268, 0.607, Main.P-Source.Conc.+P-Source.Feed.co nc.+X.F.Suc+PL+rab+His+lactate+SO4.ferm.+PO4.utilise; 0.226, 0.607, pH+Main.P-Source.Conc.+P-Source.Feed.conc.+X.F.Suc+ X.VXdt+PL+rab+Thr+lactate+SO4.ferm.+PO4.ferm.+PO4.utilise; 0.240, 0.607, pH+Main.P-Source.Conc.+P-Source.Feed.conc.+X. F.Suc+X.VXdt+total.cell+PL+rab+SO4.ferm.+PO4.ferm.+PO4.util ise; 0.254, 0.607, Main.P-Source.Conc.+P-Source.Feed.conc.+ X.F.Suc+PL+emission_O2+Thr+His+SO4.ferm.+PO4.ferm.+PO4.util ise; 0.240, 0.607, pH+Main.P-Source.Conc.+P-Source.Feed.con c.+X.F.Suc+PL+rab+Thr+His+lactate+SO4.ferm.+PO4.utilise; 0. 293, 0.607, Main.P-Source.Conc.+P-Source.Feed.conc.+yield+P L+SO4.ferm.+PO4.ferm.+PO4.utilise; 0.280, 0.607, tmp+Main.P -Source.Conc.+P-Source.Feed.conc.+X.F.Suc+emission_O2+SO4.f erm.+PO4.ferm.+PO4.utilise; 0.240, 0.607, pH+tmp+Main.P-Sou rce.Conc.+P-Source.Feed.conc.+X.F.Suc+X.VXdt+PL+His+SO4.fer m.+PO4.ferm.+PO4.utilise; 0.240, 0.607, pH+Main.P-Source.Co nc.+P-Source.Feed.conc.+X.F.Suc+X.VXdt+total.Vol+PL+emissio n_O2+lactate+SO4.ferm.+PO4.utilise; 0.280, 0.607, Main.P-So urce.Conc.+P-Source.Feed.conc.+Main.Thr.Conc.+X.F.Suc+rab+T hr+SO4.ferm.+PO4.utilise; 0.267, 0.607, tmp+Main.P-Source.C onc.+P-Source.Feed.conc.+Main.Thr.Conc.+X.F.Suc+PL+Thr+SO4. ferm.+PO4.utilise; 0.254, 0.607, pH+Main.P-Source.Conc.+P-S ource.Feed.conc.+X.F.Suc+Cell+PL+rab+SO4.ferm.+PO4.ferm.+PO 4.utilise; 0.254, 0.607, pH+Main.P-Source.Conc.+P-Source.Fe ed.conc.+X.F.Suc+Cell+PL+rab+His+SO4.ferm.+PO4.utilise; 0.2 80, 0.607, pH+Main.P-Source.Conc.+P-Source.Feed.conc.+Nitro gen.Conc.+X.F.Suc+rab+SO4.ferm.+PO4.utilise; 0.240, 0.607, Main.P-Source.Conc.+P-Source.Feed.conc.+X.F.Suc+X.VXdt+tota l.Vol+PL+rab+His+SO4.ferm.+PO4.ferm.+PO4.utilise; 0.254, 0. 607, pH+tmp+Main.P-Source.Conc.+P-Source.Feed.conc.+X.F.Suc +total.Vol+PL+SO4.ferm.+PO4.ferm.+PO4.utilise; 0.240, 0.607, tmp+Main.P-Source.Conc.+P-Source.Feed.conc.+X.F.Suc+X.VXdt +PL+rab+Thr+SO4.ferm.+PO4.ferm.+PO4.utilise; 0.267, 0.607, pH+Main.P-Source.Conc.+P-Source.Feed.conc.+X.F.Suc+X.VXdt+r ab+His+SO4.ferm.+PO4.utilise; 0.226, 0.607, pH+tmp+Main.P-S ource.Conc.+P-Source.Feed.conc.+OD+X.F.Suc+total.Vol+total. cell+PL+rab+SO4.ferm.+PO4.utilise; 0.280, 0.607, Main.P-Sou rce.Conc.+P-Source.Feed.conc.+X.F.Suc+emission_O2+Thr+lacta te+SO4.ferm.+PO4.utilise; 0.267, 0.607, Main.P-Source.Conc. +P-Source.Feed.conc.+Main.Thr.Conc.+X.F.Suc+total.Vol+PL+ra b+SO4.ferm.+PO4.utilise; 0.254, 0.607, pH+Main.P-Source.Con c.+P-Source.Feed.conc.+Nitrogen.Conc.+X.F.Suc+PL+emission_O 2+SO4.ferm.+PO4.ferm.+PO4.utilise; 0.292, 0.607, tmp+Main.P -Source.Conc.+P-Source.Feed.conc.+total.Vol+PL+SO4.ferm.+PO 4.utilise; 0.240, 0.607, pH+Main.P-Source.Conc.+P-Source.Fe ed.conc.+X.F.Suc+X.VXdt+total.cell+PL+rab+His+SO4.ferm.+PO4. utilise; 0.267, 0.607, tmp+Main.P-Source.Conc.+P-Source.Fee d.conc.+total.Vol+yield+PL+rab+SO4.ferm.+PO4.utilise; 0.280, 0.607, Main.P-Source.Conc.+P-Source.Feed.conc.+X.F.Suc+tot al.Vol+emission_O2+SO4.ferm.+PO4.ferm.+PO4.utilise; 0.280, 0.607, pH+tmp+Main.P-Source.Conc.+P-Source.Feed.conc.+total. Vol+PL+SO4.ferm.+PO4.utilise; 0.292, 0.607, Main.P-Source.C onc.+P-Source.Feed.conc.+yield+emission_O2+His+SO4.ferm.+PO 4.utilise; 0.280, 0.607, pH+tmp+Main.P-Source.Conc.+P-Sourc e.Feed.conc.+X.F.Suc+X.VXdt+SO4.ferm.+PO4.utilise; 0.254, 0. 607, pH+tmp+Main.P-Source.Conc.+P-Source.Feed.conc.+X.F.Suc +total.cell+PL+emission_O2+SO4.ferm.+PO4.utilise; 0.240, 0. 607, pH+tmp+Main.P-Source.Conc.+P-Source.Feed.conc.+Nitroge n.Conc.+X.F.Suc+total.Vol+PL+rab+SO4.ferm.+PO4.utilise; 0.2 80, 0.607, Main.P-Source.Conc.+P-Source.Feed.conc.+yield+PL +emission_O2+His+SO4.ferm.+PO4.utilise; 0.254, 0.607, pH+Ma in.P-Source.Conc.+P-Source.Feed.conc.+X.F.Suc+total.Vol+PL+ rab+lactate+SO4.ferm.+PO4.utilise; 0.254, 0.607, pH+tmp+Mai n.P-Source.Conc.+P-Source.Feed.conc.+X.F.Suc+PL+rab+lactate +SO4.ferm.+PO4.utilise; 0.267, 0.607, Main.P-Source.Conc.+P -Source.Feed.conc.+X.F.Suc+Cell+PL+rab+SO4.ferm.+PO4.ferm.+ PO4.utilise; 0.240, 0.607, pH+Main.P-Source.Conc.+P-Source. Feed.conc.+Nitrogen.Conc.+X.F.Suc+X.VXdt+PL+rab+His+SO4.fer m.+PO4.utilise; 0.280, 0.607, tmp+Main.P-Source.Conc.+P-Sou rce.Feed.conc.+yield+His+SO4.ferm.+PO4.ferm.+PO4.utilise; 0. 267, 0.607, Main.P-Source.Conc.+P-Source.Feed.conc.+OD+X.F. Suc+PL+rab+SO4.ferm.+PO4.ferm.+PO4.utilise; 0.253, 0.607, M ain.P-Source.Conc.+P-Source.Feed.conc.+Lys_conc.+X.F.Suc+PL +rab+Thr+His+SO4.ferm.+PO4.utilise; 0.253, 0.607, tmp+Main. P-Source.Conc.+P-Source.Feed.conc.+Main.Thr.Conc.+X.F.Suc+X. VXdt+PL+emission_O2+SO4.ferm.+PO4.utilise; 0.253, 0.607, pH +Main.P-Source.Conc.+P-Source.Feed.conc.+yield+PL+rab+His+S O4.ferm.+PO4.ferm.+PO4.utilise; 0.267, 0.607, pH+tmp+Main.P -Source.Conc.+P-Source.Feed.conc.+X.F.Suc+emission_O2+His+S O4.ferm.+PO4.utilise; 0.267, 0.607, tmp+Main.P-Source.Conc. +P-Source.Feed.conc.+yield+PL+rab+SO4.ferm.+PO4.ferm.+PO4.u tilise; 0.225, 0.607, pH+Main.P-Source.Conc.+P-Source.Feed. conc.+Nitrogen.Conc.+X.F.Suc+X.VXdt+total.Vol+PL+rab+His+SO 4.ferm.+PO4.utilise; 0.279, 0.607, Main.P-Source.Conc.+P-So urce.Feed.conc.+X.F.Suc+rab+Thr+lactate+SO4.ferm.+PO4.utili se; 0.239, 0.607, Main.P-Source.Conc.+P-Source.Feed.conc.+O D+X.F.Suc+X.VXdt+total.Vol+total.cell+PL+rab+SO4.ferm.+PO4. utilise; 0.279, 0.607, tmp+Main.P-Source.Conc.+P-Source.Fee d.conc.+X.F.Suc+X.VXdt+His+SO4.ferm.+PO4.utilise; 0.253, 0. 607, pH+Main.P-Source.Conc.+P-Source.Feed.conc.+Main.Thr.Co nc.+X.F.Suc+total.Vol+PL+rab+SO4.ferm.+PO4.utilise; 0.292, 0.607, Main.P-Source.Conc.+P-Source.Feed.conc.+Lys_conc.+yi eld+emission_O2+SO4.ferm.+PO4.utilise; 0.253, 0.607, tmp+Ma in.P-Source.Conc.+P-Source.Feed.conc.+Nitrogen.Conc.+X.F.Su c+PL+rab+SO4.ferm.+PO4.ferm.+PO4.utilise; 0.266, 0.607, Mai n.P-Source.Conc.+P-Source.Feed.conc.+yield+PL+emission_O2+H is+SO4.ferm.+PO4.ferm.+PO4.utilise; 0.266, 0.607, pH+Main.P -Source.Conc.+P-Source.Feed.conc.+X.F.Suc+X.VXdt+total.Vol+ rab+SO4.ferm.+PO4.utilise; 0.279, 0.607, pH+Main.P-Source.C onc.+P-Source.Feed.conc.+X.F.Suc+rab+lactate+SO4.ferm.+PO4. utilise; 0.225, 0.607, pH+Main.P-Source.Conc.+P-Source.Feed. conc.+OD+X.F.Suc+total.Vol+total.cell+PL+emission_O2+SO4.fe rm.+PO4.ferm.+PO4.utilise; 0.266, 0.607, tmp+Main.P-Source. Conc.+P-Source.Feed.conc.+X.F.Suc+total.Vol+PL+SO4.ferm.+PO 4.ferm.+PO4.utilise; 0.210, 0.606, Main.P-Source.Cone.+P-So urce.Feed.conc.+Main.Thr.Conc.+OD+Lys_conc.+Cell+total.Vol+ total.cell+yield+PL+rab+SO4.ferm.+PO4.utilise; 0.225, 0.606, pH+tmp+Main.P-Source.Conc.+P-Source.Feed.conc.+X.F.Suc+X.V Xdt+PL+emission_O2+His+SO4.ferm.+PO4.ferm.+PO4.utilise; 0.2 66, 0.606, pH+Main.P-Source.Conc.+P-Source.Feed.conc.+total. Vol+yield+PL+rab+SO4.ferm.+PO4.utilise; 0.253, 0.606, pH+Ma in.P-Source.Conc.+P-Source.Feed.conc.+OD+X.F.Suc+PL+rab+SO4. ferm.+PO4.ferm.+PO4.utilise; 0.253, 0.606, tmp+Main.P-Sourc e.Conc.+P-Source.Feed.conc.+X.F.Suc+X.VXdt+PL+rab+lactate+S O4.ferm.+PO4.utilise; 0.266, 0.606, Main.P-Source.Conc.+P-S ource.Feed.conc.+Nitrogen.Conc.+yield+PL+rab+SO4.ferm.+PO4. ferm.+PO4.utilise; 0.279, 0.606, Main.P-Source.Conc.+P-Sour ce.Feed.conc.+X.F.Suc+X.VXdt+emission_O2+SO4.ferm.+PO4.ferm. +PO4.utilise; 0.239, 0.606, Main.P-Source.Conc.+P-Source.Fe ed.conc.+X.F.Suc+X.VXdt+total.Vol+PL+rab+Thr+His+SO4.ferm.+ PO4.utilise; 0.266, 0.606, Main.P-Source.Conc.+P-Source.Fee d.conc.+OD+X.F.Suc+total.cell+PL+rab+SO4.ferm.+PO4.utilise; 0.266, 0.606, Main.P-Source.Conc.+P-Source.Feed.conc.+X.F. Suc+total.cell+PL+emission_O2+SO4.ferm.+PO4.ferm.+PO4.utili se; 0.266, 0.606, Main.P-Source.Conc.+P-Source.Feed.conc.+X. F.Suc+Cell+PL+rab+Thr+SO4.ferm.+PO4.utilise; 0.239, 0.606, pH+Main.P-Source.Conc.+P-Source.Feed.conc.+X.F.Suc+X.VXdt+P L+rab+lactate+SO4.ferm.+PO4.ferm.+PO4.utilise; 0.239, 0.606, pH+Main.P-Source.Conc.+P-Source.Feed.conc.+X.F.Suc+total.V ol+PL+emission_O2+Thr+SO4.ferm.+PO4.ferm.+PO4.utilise; 0.29 1, 0.606, Main.P-Source.Conc.+P-Source.Feed.conc.+total.Vol +PL+rab+SO4.ferm.+PO4.utilise; 0.239, 0.606, pH+Main.P-Sour ce.Conc.+P-Source.Feed.conc.+X.F.Suc+PL+emission_O2+Thr+His +lactate+SO4.ferm.+PO4.utilise; 0.253, 0.606, pH+tmp+Main.P -Source.Conc.+P-Source.Feed.conc.+Main.Thr.Conc.+X.F.Suc+X. VXdt+PL+SO4.ferm.+PO4.utilise; 0.303, 0.606, Main.P-Source. Conc.+P-Source.Feed.conc.+X.F.Suc+total.Vol+SO4.ferm.+PO4.u tilise; 0.266, 0.606, Main.P-Source.Cone.+P-Source.Feed.con c.+Main.Thr.Conc.+X.F.Suc+PL+emission_O2+His+SO4.ferm.+PO4. utilise; 0.224, 0.606, pH+Main.P-Source.Conc.+P-Source.Feed. conc.+OD+X.F.Suc+X.VXdt+total.Vol+total.cell+PL+emission_O2 +SO4.ferm.+PO4.utilise; 0.239, 0.606, pH+Main.P-Source.Conc. +P-Source.Feed.conc.+Nitrogen.Conc.+X.F.Suc+total.Vol+PL+ra b+Thr+SO4.ferm.+PO4.utilise; 0.252, 0.606, Main.P-Source.Co nc.+P-Source.Feed.conc.+Main.Thr.Conc.+X.F.Suc+X.VXdt+total. Vol+PL+emission_O2+SO4.ferm.+PO4.utilise; 0.252, 0.606, pH+ tmp+Main.P-Source.Conc.+P-Source.Feed.conc.+Main.Thr.Conc.+ X.F.Suc+PL+Thr+SO4.ferm.+PO4.utilise; 0.252, 0.606, pH+tmp+ Main.P-Source.Conc.+P-Source.Feed.conc.+X.F.Suc+rab+Thr+His +SO4.ferm.+PO4.utilise; 0.252, 0.606, pH+Main.P-Source.Conc. +P-Source.Feed.conc.+OD+X.F.Suc+X.VXdt+PL+emission_O2+SO4.f erm.+PO4.utilise; 0.266, 0.606, tmp+Main.P-Source.Conc.+P-S ource.Feed.conc.+X.F.Suc+X.VXdt+rab+His+SO4.ferm.+PO4.utili se; 0.224, 0.606, pH+Main.P-Source.Conc.+P-Source.Feed.conc. +X.F.Suc+X.VXdt+total.Vol+PL+emission_O2+His+SO4.ferm.+PO4. ferm.+PO4.utilise; 0.279, 0.606, Main.P-Source.Conc.+P-Sour ce.Feed.conc.+Main.Thr.Conc.+X.F.Suc+emission_O2+Thr+SO4.fe rm.+PO4.utilise; 0.239, 0.606, pH+tmp+Main.P-Source.Conc.+P -Source.Feed.conc.+Nitrogen.Conc.+X.F.Suc+PL+emission_O2+Th r+SO4.ferm.+PO4.utilise; 0.291, 0.606, Main.P-Source.Conc.+ P-Source.Feed.conc.+X.F.Suc+total.Vol+PL+SO4.ferm.+PO4.util ise; 0.266, 0.606, pH+Main.P-Source.Conc.+P-Source.Feed.con c.+X.F.Suc+rab+Thr+lactate+SO4.ferm.+PO4.utilise; 0.266, 0. 606, pH+Main.P-Source.Conc.+P-Source.Feed.conc.+X.F.Suc+emi ssion_O2+Thr+lactate+SO4.ferm.+PO4.utilise; 0.252, 0.606, M ain.P-Source.Conc.+P-Source.Feed.conc.+Lys_conc.+X.F.Suc+PL +emission_O2+Thr+His+SO4.ferm.+PO4.utilise; 0.224, 0.606, p H+tmp+Main.P-Source.Conc.+P-Source.Feed.conc.+Nitrogen.Conc. +X.F.Suc+X.VXdt+PL+rab+SO4.ferm.+PO4.ferm.+PO4.utilise; 0.2 52, 0.606, pH+Main.P-Source.Conc.+P-Source.Feed.conc.+X.F.S uc+PL+emission_O2+His+lactate+SO4.ferm.+PO4.utilise; 0.238, 0.606, pH+tmp+Main.P-Source.Conc.+P-Source.Feed.conc.+X.F. Suc+X.VXdt+total.cell+PL+emission_O2+SO4.ferm.+PO4.utilise; 0.266, 0.606, tmp+Main.P-Source.Conc.+P-Source.Feed.conc.+ X.F.Suc+PL+rab+lactate+SO4.ferm.+PO4.utilise; 0.238, 0.606, pH+Main.P-Source.Conc.+P-Source.Feed.conc.+Main.Thr.Conc.+ X.F.Suc+X.VXdt+PL+rab+SO4.ferm.+PO4.ferm.+PO4.utilise; 0.26 6, 0.606, tmp+Main.P-Source.Conc.+P-Source.Feed.conc.+X.F.S uc+X.VXdt+total.cell+PL+SO4.ferm.+PO4.utilise; 0.252, 0.606, pH+Main.P-Source.Conc.+P-Source.Feed.conc.+Main.Thr.Conc.+ X.F.Suc+PL+rab+His+SO4.ferm.+PO4.utilise; 0.252, 0.606, Mai n.P-Source.Conc.+P-Source.Feed.conc.+Nitrogen.Conc.+X.F.Suc +X.VXdt+PL+rab+His+SO4.ferm.+PO4.utilise; 0.238, 0.606, tmp +Main.P-Source.Conc.+P-Source.Feed.conc.+X.F.Suc+X.VXdt+PL+ rab+Thr+lactate+SO4.ferm.+PO4.utilise; 0.265, 0.606, tmp+Ma in.P-Source.Conc.+P-Source.Feed.conc.+X.F.Suc+total.Vol+emi ssion_O2+His+SO4.ferm.+PO4.utilise; 0.238, 0.606, pH+Main.P -Source.Conc.+P-Source.Feed.conc.+Nitrogen.Conc.+X.F.Suc+PL +rab+Thr+His+SO4.ferm.+PO4.utilise; 0.265, 0.606, pH+Main.P -Source.Conc.+P-Source.Feed.conc.+X.F.Suc+rab+Thr+SO4.ferm. +PO4.ferm.+PO4.utilise; 0.278, 0.606, Main.P-Source.Conc.+P -Source.Feed.conc.+Lys conc.+X.F.Suc+rab+Thr+SO4.ferm.+PO4. utilise; 0.278, 0.606, tmp+Main.P-Source.Conc.+P-Source.Fee d.conc.+total.Vol+PL+rab+SO4.ferm.+PO4.utilise; 0.265, 0.60 6, Main.P-Source.Conc.+P-Source.Feed.conc.+X.F.Suc+X.VXdt+P L+emission_O2+lactate+SO4.ferm.+PO4.utilise; 0.265, 0.606, Main.P-Source.Conc.+P-Source.Feed.conc.+Main.Thr.Conc.+X.F. Suc+total.Vol+PL+emission_O2+SO4.ferm.+PO4.utilise; 0.291, 0.606, Main.P-Source.Conc.+P-Source.Feed.conc.+Lys_conc.+yi eld+rab+SO4.ferm.+PO4.utilise; 0.252, 0.606, pH+Main.P-Sour ce.Conc.+P-Source.Feed.conc.+OD+X.F.Suc+total.cell+PL+rab+S O4.ferm.+PO4.utilise; 0.303, 0.606, pH+Main.P-Source.Conc.+ P-Source.Feed.conc.+X.F.Suc+SO4.ferm.+PO4.utilise; 0.265, 0. 606, tmp+Main.P-Source.Conc.+P-Source.Feed.conc.+Nitrogen.C onc.+X.F.Suc+total.Vol+PL+SO4.ferm.+PO4.utilise; 0.224, 0.6 06, pH+tmp+Main.P-Source.Conc.+P-Source.Feed.conc.+Nitrogen. Conc.+X.F.Suc+X.VXdt+PL+rab+His+SO4.ferm.+PO4.utilise; 0.27 8, 0.606, Main.P-Source.Conc.+P-Source.Feed.conc.+Nitrogen. Conc.+X.F.Suc+emission_O2+Thr+SO4.ferm.+PO4.utilise; 0.265, 0.606, pH+Main.P-Source.Conc.+P-Source.Feed.conc.+total.Vo l+yield+PL+emission_O2+SO4.ferm.+PO4.utilise; 0.252, 0.606, pH+Main.P-Source.Conc.+P-Source.Feed.conc.+X.F.Suc+total.c ell+PL+emission_O2+SO4.ferm.+PO4.ferm.+PO4.utilise; 0.252, 0.606, pH+tmp+Main.P-Source.Conc.+P-Source.Feed.conc.+Nitro gen.Conc.+X.F.Suc+total.Vol+PL+SO4.ferm.+PO4.utilise; 0.278, 0.606, Main.P-Source.Conc.+P-Source.Feed.conc.+Lys_conc.+X. F.Suc+emission_O2+Thr+SO4.ferm.+PO4.utilise; 0.278, 0.606, pH+Main.P-Source.Conc.+P-Source.Feed.conc.+X.F.Suc+total.Vo l+PL+SO4.ferm.+PO4.utilise; 0.238, 0.606, tmp+Main.P-Source. Conc.+P-Source.Feed.conc.+X.F.Suc+X.VXdt+PL+emission_O2+His +SO4.ferm.+PO4.ferm.+PO4.utilise; 0.265, 0.606, Main.P-Sour ce.Conc.+P-Source.Feed.conc.+OD+X.F.Suc+X.VXdt+PL+emission_ O2+SO4.ferm.+PO4.utilise; 0.265, 0.606, Main.P-Source.Conc. +P-Source.Feed.conc.+X.F.Suc+X.VXdt+total.Vol+rab+His+SO4.f erm.+PO4.utilise; 0.238, 0.606, pH+Main.P-Source.Conc.+P-So urce.Feed.conc.+X.F.Suc+total.Vol+PL+rab+Thr+lactate+SO4.fe rm.+PO4.utilise; 0.251, 0.606, Main.P-Source.Conc.+P-Source. Feed.conc.+X.F.Suc+X.VXdt+total.cell+PL+rab+His+SO4.ferm.+P O4.utilise; 0.302, 0.606, pH+Main.P-Source.Conc.+P-Source.F eed.conc.+yield+SO4.ferm.+PO4.utilise; 0.208, 0.605, pH+Mai n.P-Source.Conc.+P-Source.Feed.conc.+X.F.Suc+X.VXdt+total.V ol+PL+rab+Thr+His+lactate+SO4.ferm.+PO4.utilise; 0.238, 0.6 05, pH+tmp+Main.P-Source.Conc.+P-Source.Feed.conc.+X.F.Suc+ X.VXdt+PL+emission_O2+Thr+SO4.ferm.+PO4.utilise; 0.251, 0.6 05, Main.P-Source.Conc.+P-Source.Feed.conc.+Main.Thr.Conc.+ X.F.Suc+PL+rab+Thr+His+SO4.ferm.+PO4.utilise; 0.251, 0.605, Main.P-Source.Conc.+P-Source.Feed.conc.+Nitrogen.Conc.+X.F. Suc+X.VXdt+PL+rab+SO4.ferm.+PO4.ferm.+PO4.utilise; 0.251, 0. 605, tmp+Main.P-Source.Conc.+P-Source.Feed.conc.+X.F.Suc+to tal.Vol+PL+emission_O2+SO4.ferm.+PO4.ferm.+PO4.utilise; 0.2 65, 0.605, pH+tmp+Main.P-Source.Conc.+P-Source.Feed.conc.+X. F.Suc+X.VXdt+rab+SO4.ferm.+PO4.utilise; 0.290, 0.605, Main. P-Source.Conc.+P-Source.Feed.conc.+yield+rab+SO4.ferm.+PO4. ferm.+PO4.utilise; 0.251, 0.605, pH+Main.P-Source.Conc.+P-S ource.Feed.conc.+X.F.Suc+Cell+PL+rab+Thr+SO4.ferm.+PO4.util ise; 0.265, 0.605, Main.P-Source.Conc.+P-Source.Feed.conc.+ X.F.Suc+PL+emission_O2+lactate+SO4.ferm.+PO4.ferm.+PO4.util ise; 0.251, 0.605, tmp+Main.P-Source.Conc.+P-Source.Feed.co nc.+Nitrogen.Conc.+X.F.Suc+total.Vol+PL+emission_O2+SO4.fer m.+PO4.utilise; 0.277, 0.605, pH+Main.P-Source.Conc.+P-Sour ce.Feed.conc.+yield+PL+SO4.ferm.+PO4.ferm.+PO4.utilise; 0.2 77, 0.605, tmp+Main.P-Source.Conc.+P-Source.Feed.conc.+Lys_ conc.+yield+PL+SO4.ferm.+PO4.utilise; 0.265, 0.605, pH+tmp+ Main.P-Source.Conc.+P-Source.Feed.conc.+Lys_conc.+yield+PL+ SO4.ferm.+PO4.utilise; 0.251, 0.605, pH+Main.P-Source.Conc. +P-Source.Feed.conc.+Main.Thr.Conc.+X.F.Suc+total.Vol+PL+em ission_O2+SO4.ferm.+PO4.utilise; 0.223, 0.605, pH+Main.P-So urce.Conc.+P-Source.Feed.conc.+OD+X.F.Suc+total.Vol+total.c ell+PL+rab+Thr+SO4.ferm.+PO4.utilise; 0.290, 0.605, tmp+Mai n.P-Source.Conc.+P-Source.Feed.conc.+Lys conc.+yield+SO4.fe rm.+PO4.utilise; 0.251, 0.605, Main.P-Source.Conc.+P-Source. Feed.conc.+X.F.Suc+X.VXdt+total.cell+PL+rab+SO4.ferm.+PO4.f erm.+PO4.utilise; 0.237, 0.605, pH+tmp+Main.P-Source.Conc.+ P-Source.Feed.conc.+OD+X.F.Suc+total.Vol+total.cell+PL+SO4. ferm.+PO4.utilise; 0.237, 0.605, pH+tmp+Main.P-Source.Conc. +P-Source.Feed.conc.+Nitrogen.Conc.+X.F.Suc+PL+rab+SO4.ferm. +PO4.ferm.+PO4.utilise; 0.251, 0.605, Main.P-Source.Conc.+P -Source.Feed.conc.+X.F.Suc+X.VXdt+total.Vol+total.cell+PL+e mission_O2+SO4.ferm.+PO4.utilise; 0.237, 0.605, pH+tmp+Main. P-Source.Conc.+P-Source.Feed.conc.+X.F.Suc+PL+rab+lactate+S O4.ferm.+PO4.ferm.+PO4.utilise; 0.264, 0.605, Main.P-Source. Conc.+P-Source.Feed.conc.+X.F.Suc+total.cell+PL+emission_O2 +His+SO4.ferm.+PO4.utilise; 0.237, 0.605, pH+tmp+Main.P-Sou rce.Conc.+P-Source.Feed.conc.+X.F.Suc+X.VXdt+total.Vol+PL+H is+SO4.ferm.+PO4.utilise; 0.277, 0.605, pH+Main.P-Source.Co nc.+P-Source.Feed.conc.+Nitrogen.Conc.+X.F.Suc+emission_O2+ SO4.ferm.+PO4.utilise; 0.290, 0.605, pH+Main.P-Source.Conc. +P-Source.Feed.conc.+yield+PL+SO4.ferm.+PO4.utilise; 0.223, 0.605, Main.P-Source.Conc.+P-Source.Feed.conc.+OD+Lys_conc. +Cell+total.Vol+total.cell+yield+PL+rab+SO4.ferm.+PO4.utili se; 0.264, 0.605, Main.P-Source.Conc.+P-Source.Feed.conc.+X. F.Suc+total.Vol+PL+rab+lactate+SO4.ferm.+PO4.utilise; 0.251, 0.605, tmp+Main.P-Source.Conc.+P-Source.Feed.conc.+X.F.Suc +total.cell+PL+rab+Thr+SO4.ferm.+PO4.utilise; 0.251, 0.605, pH+tmp+Main.P-Source.Conc.+P-Source.Feed.conc.+Nitrogen.Co nc.+X.F.Suc+PL+SO4.ferm.+PO4.ferm.+PO4.utilise; 0.264, 0.60 5, tmp+Main.P-Source.Conc.+P-Source.Feed.conc.+X.F.Suc+Cell +PL+rab+SO4.ferm.+PO4.utilise; 0.290, 0.605, Main.P-Source. Conc.+P-Source.Feed.conc.+yield+His+SO4.ferm.+PO4.ferm.+PO4. utilise; 0.277, 0.605, pH+tmp+Main.P-Source.Conc.+P-Source. Feed.conc.+X.F.Suc+SO4.ferm.+PO4.ferm.+PO4.utilise; 0.264, 0.605, Main.P-Source.Conc.+P-Source.Feed.conc.+X.F.Suc+PL+e mission_O2+His+lactate+SO4.ferm.+PO4.utilise; 0.237, 0.605, tmp+Main.P-Source.Conc.+P-Source.Feed.conc.+OD+X.F.Suc+tot al.Vol+total.cell+PL+rab+SO4.ferm.+PO4.utilise; 0.301, 0.60 5, Main.P-Source.Conc.+P-Source.Feed.conc.+X.F.Suc+His+SO4. ferm.+PO4.utilise; 0.264, 0.605, tmp+Main.P-Source.Conc.+P-Source.Feed.conc.+X.F.Suc+rab+Thr+SO4.ferm.+PO4.ferm.+PO4.u tilise; 0.277, 0.605, tmp+Main.P-Source.Conc.+P-Source.Feed. conc.+total.Vol+PL+His+SO4.ferm.+PO4.utilise; 0.207, 0.605, pH+tmp+Main.P-Source.Conc.+P-Source.Feed.conc.+X.F.Suc+X.V Xdt+PL+rab+Thr+His+lactate+SO4.ferm.+PO4.utilise; 0.251, 0. 605, tmp+Main.P-Source.Conc.+P-Source.Feed.conc.+X.F.Suc+to tal.Vol+rab+Thr+His+SO4.ferm.+PO4.utilise; 0.237, 0.605, Ma in.P-Source.Conc.+P-Source.Feed.conc.+OD+X.F.Suc+total.Vol+ total.cell+PL+rab+Thr+SO4.ferm.+PO4.utilise; 0.251, 0.605, Main.P-Source.Conc.+P-Source.Feed.conc.+X.F.Suc+PL+emission _O2+Thr+lactate+SO4.ferm.+PO4.ferm.+PO4.utilise; 0.250, 0.6 05, tmp+Main.P-Source.Conc.+P-Source.Feed.conc.+X.F.Suc+X.V Xdt+PL+His+SO4.ferm.+PO4.ferm.+PO4.utilise; 0.250, 0.605, p H+tmp+Main.P-Source.Conc.+P-Source.Feed.conc.+total.Vol+yie ld+PL+rab+SO4.ferm.+PO4.utilise; 0.222, 0.605, pH+Main.P-So urce.Conc.+P-Source.Feed.conc.+X.F.Suc+X.VXdt+total.Vol+PL+ rab+His+lactate+SO4.ferm.+PO4.utilise; 0.237, 0.605, Main.P -Source.Conc.+P-Source.Feed.conc.+X.F.Suc+X.VXdt+total.Vol+ PL+rab+Thr+lactate+SO4.ferm.+PO4.utilise; 0.277, 0.605, tmp +Main.P-Source.Conc.+P-Source.Feed.conc.+yield+PL+His+SO4.f erm.+PO4.utilise; 0.264, 0.605, tmp+Main.P-Source.Conc.+P-S ource.Feed.conc.+Nitrogen.Conc.+X.F.Suc+PL+SO4.ferm.+PO4.fe rm.+PO4.utilise; 0.236, 0.605, pH+Main.P-Source.Conc.+P-Sou rce.Feed.conc.+Lys conc.+X.F.Suc+PL+rab+Thr+His+SO4.ferm.+P O4.utilise; 0.236, 0.605, pH+tmp+Main.P-Source.Conc.+P-Sour ce.Feed.conc.+OD+X.F.Suc+X.VXdt+PL+rab+SO4.ferm.+PO4.utilis e; 0.236, 0.605, Main.P-Source.Conc.+P-Source.Feed.conc.+X. F.Suc+X.VXdt+PL+rab+Thr+His+lactate+SO4.ferm.+PO4.utilise; 0.236, 0.605, tmp+Main.P-Source.Conc.+P-Source.Feed.conc.+X. F.Suc+PL+emission_O2+Thr+His+SO4.ferm.+PO4.ferm.+PO4.utilis e; 0.250, 0.605, pH+Main.P-Source.Conc.+P-Source.Feed.conc. +X.F.Suc+total.Vol+rab+Thr+His+SO4.ferm.+PO4.utilise; 0.236, 0.605, tmp+Main.P-Source.Conc.+P-Source.Feed.conc.+Nitroge n.Conc.+X.F.Suc+X.VXdt+PL+rab+His+SO4.ferm.+PO4.utilise; 0. 250, 0.605, Main.P-Source.Conc.+P-Source.Feed.conc.+Main.Th r.Conc.+X.F.Suc+total.Vol+PL+rab+Thr+SO4.ferm.+PO4.utilise; 0.222, 0.605, pH+tmp+Main.P-Source.Conc.+P-Source.Feed.con c.+Main.Thr.Conc.+X.F.Suc+X.VXdt+total.Vol+PL+rab+SO4.ferm. +PO4.utilise; 0.236, 0.605, tmp+Main.P-Source.Conc.+P-Sourc e.Feed.conc.+Nitrogen.Conc.+X.F.Suc+total.Vol+PL+rab+Thr+SO 4.ferm.+PO4.utilise; 0.277, 0.605, Main.P-Source.Conc.+P-So urce.Feed.conc.+total.Vol+yield+rab+His+SO4.ferm.+PO4.utili se; 0.277, 0.605, pH+Main.P-Source.Conc.+P-Source.Feed.conc. +X.F.Suc+emission_O2+lactate+SO4.ferm.+PO4.utilise; 0.264, 0.605, pH+tmp+Main.P-Source.Conc.+P-Source.Feed.conc.+X.F.S uc+total.Vol+emission_O2+SO4.ferm.+PO4.utilise; 0.175, 0.60 5, pH+Main.P-Source.Conc.+P-Source.Feed.conc.+Main.Thr.Conc. +OD+Lys conc.+Cell+total.Vol+total.cell+yield+PL+rab+SO4.fe rm.+PO4.ferm.+PO4.utilise; 0.250, 0.605, pH+Main.P-Source.C onc.+P-Source.Feed.conc.+Main.Thr.Conc.+X.F.Suc+PL+emission _O2+His+SO4.ferm.+PO4.utilise; 0.250, 0.605, pH+tmp+Main.P-Source.Conc.+P-Source.Feed.conc.+X.F.Suc+PL+emission_O2+lac tate+SO4.ferm.+PO4.utilise; 0.263, 0.605, Main.P-Source.Con c.+P-Source.Feed.conc.+X.F.Suc+total.cell+PL+emission_O2+Th r+SO4.ferm.+PO4.utilise; 0.250, 0.604, tmp+Main.P-Source.Co nc.+P-Source.Feed.conc.+Main.Thr.Conc.+X.F.Suc+total.Vol+PL +rab+SO4.ferm.+PO4.utilise; 0.222, 0.604, pH+tmp+Main.P-Sou rce.Conc.+P-Source.Feed.conc.+X.F.Suc+X.VXdt+PL+rab+His+lac tate+SO4.ferm.+PO4.utilise; 0.276, 0.604, pH+Main.P-Source. Conc.+P-Source.Feed.conc.+total.Vol+PL+rab+SO4.ferm.+PO4.ut ilise; 0.263, 0.604, Main.P-Source.Conc.+P-Source.Feed.conc. +X.F.Suc+total.Vol+total.cell+PL+emission_O2+SO4.ferm.+PO4. utilise; 0.250, 0.604, pH+Main.P-Source.Conc.+P-Source.Feed. conc.+X.F.Suc+Cell+X.VXdt+PL+emission_O2+SO4.ferm.+PO4.util ise; 0.263, 0.604, pH+Main.P-Source.Conc.+P-Source.Feed.con c.+Lys_conc.+yield+PL+rab+SO4.ferm.+PO4.utilise; 0.276, 0.6 04, Main.P-Source.Conc.+P-Source.Feed.conc.+Nitrogen.Conc.+ yield+PL+rab+SO4.ferm.+PO4.utilise; 0.236, 0.604, pH+tmp+Ma in.P-Source.Conc.+P-Source.Feed.conc.+X.F.Suc+total.Vol+PL+ emission_O2+SO4.ferm.+PO4.ferm.+PO4.utilise; 0.263, 0.604, pH+Main.P-Source.Conc.+P-Source.Feed.conc.+X.F.Suc+X.VXdt+t otal.Vol+emission_O2+SO4.ferm.+PO4.utilise; 0.250, 0.604, p H+tmp+Main.P-Source.Conc.+P-Source.Feed.conc.+X.F.Suc+X.VXd t+PL+Thr+SO4.ferm.+PO4.utilise; 0.289, 0.604, Main.P-Source. Conc.+P-Source.Feed.conc.+X.F.Suc+total.cell+emission_O2+SO 4.ferm.+PO4.utilise; 0.289, 0.604, tmp+Main.P-Source.Conc.+ P-Source.Feed.conc.+Nitrogen.Conc.+X.F.Suc+SO4.ferm.+PO4.ut ilise; 0.221, 0.604, pH+tmp+Main.P-Source.Conc.+P-Source.Fe ed.conc.+X.F.Suc+X.VXdt+total.Vol+PL+emission_O2+His+SO4.fe rm.+PO4.utilise; 0.276, 0.604, Main.P-Source.Conc.+P-Source. Feed.conc.+total.Vol+yield+emission_O2+His+SO4.ferm.+PO4.ut ilise; 0.236, 0.604, tmp+Main.P-Source.Conc.+P-Source.Feed. conc.+X.F.Suc+X.VXdt+PL+emission_O2+Thr+His+SO4.ferm.+PO4.u tilise; 0.263, 0.604, tmp+Main.P-Source.Conc.+P-Source.Feed. conc.+total.Vol+yield+PL+emission_O2+SO4.ferm.+PO4.utilise; 0.236, 0.604, tmp+Main.P-Source.Conc.+P-Source.Feed.conc.+ X.F.Suc+X.VXdt+total.Vol+PL+rab+Thr+SO4.ferm.+PO4.utilise; 0.250, 0.604, pH+Main.P-Source.Conc.+P-Source.Feed.conc.+X. F.Suc+total.Vol+total.cell+PL+emission_O2+SO4.ferm.+PO4.uti lise; 0.263, 0.604, pH+Main.P-Source.Conc.+P-Source.Feed.co nc.+Lys_conc.+yield+PL+emission_O2+SO4.ferm.+PO4.utilise; 0. 263, 0.604, pH+Main.P-Source.Conc.+P-Source.Feed.conc.+X.F. Suc+rab+His+SO4.ferm.+PO4.ferm.+PO4.utilise; 0.250, 0.604, Main.P-Source.Conc.+P-Source.Feed.conc.+X.F.Suc+X.VXdt+tota l.Vol+PL+rab+lactate+SO4.ferm.+PO4.utilise; 0.263, 0.604, p H+tmp+Main.P-Source.Conc.+P-Source.Feed.conc.+X.F.Suc+total. Vol+His+SO4.ferm.+PO4.utilise; 0.263, 0.604, Main.P-Source. Conc.+P-Source.Feed.conc.+X.F.Suc+Cell+total.Vol+PL+emissio n_O2+SO4.ferm.+PO4.utilise; 0.263, 0.604, pH+tmp+Main.P-Sou rce.Conc.+P-Source.Feed.conc.+total.Vol+PL+His+SO4.ferm.+PO 4.utilise; 0.250, 0.604, pH+tmp+Main.P-Source.Conc.+P-Sourc e.Feed.conc.+yield+PL+rab+SO4.ferm.+PO4.ferm.+PO4.utilise; 0.263, 0.604, Main.P-Source.Conc.+P-Source.Feed.conc.+total. Vol+yield+PL+rab+His+SO4.ferm.+PO4.utilise; 0.263, 0.604, M ain.P-Source.Cone.+P-Source.Feed.conc.+X.F.Suc+X.VXdt+rab+T hr+lactate+SO4.ferm.+PO4.utilise; 0.236, 0.604, pH+Main.P-S ource.Conc.+P-Source.Feed.conc.+X.F.Suc+total.Vol+PL+emissi on_O2+Thr+lactate+SO4.ferm.+PO4.utilise; 0.249, 0.604, Main. P-Source.Conc.+P-Source.Feed.conc.+OD+X.F.Suc+X.VXdt+total. Vol+PL+emission_O2+SO4.ferm.+PO4.utilise; 0.249, 0.604, tmp +Main.P-Source.Conc.+P-Source.Feed.conc.+X.F.Suc+X.VXdt+tot al.cell+PL+emission_O2+SO4.ferm.+PO4.utilise; 0.249, 0.604, pH+Main.P-Source.Conc.+P-Source.Feed.conc.+X.F.Suc+total.c ell+PL+emission_O2+His+SO4.ferm.+PO4.utilise; 0.236, 0.604, tmp+Main.P-Source.Conc.+P-Source.Feed.conc.+Nitrogen.Conc. +X.F.Suc+X.VXdt+PL+rab+SO4.ferm.+PO4.ferm.+PO4.utilise; 0.2 49, 0.604, Main.P-Source.Conc.+P-Source.Feed.conc.+Nitrogen. Conc.+X.F.Suc+total.Vol+PL+emission_O2+Thr+SO4.ferm.+PO4.ut ilise; 0.249, 0.604, pH+Main.P-Source.Conc.+P-Source.Feed.c onc.+X.F.Suc+X.VXdt+PL+emission_O2+Thr+SO4.ferm.+PO4.utilis e; 0.221, 0.604, pH+tmp+Main.P-Source.Conc.+P-Source.Feed.c onc.+X.F.Suc+PL+rab+Thr+His+SO4.ferm.+PO4.ferm.+PO4.utilis e; 0.235, 0.604, pH+Main.P-Source.Conc.+P-Source.Feed.conc. +Main.Thr.Conc.+X.F.Suc+X.VXdt+PL+rab+His+SO4.ferm.+PO4.uti lise; 0.221, 0.604, pH+Main.P-Source.Conc.+P-Source.Feed.co nc.+X.F.Suc+PL+rab+Thr+His+lactate+SO4.ferm.+PO4.ferm.+PO4. utilise; 0.288, 0.604, pH+tmp+Main.P-Source.Conc.+P-Source. Feed.conc.+yield+SO4.ferm.+PO4.utilise; 0.276, 0.604, tmp+M ain.P-Source.Conc.+P-Source.Feed.conc.+Lys_conc.+total.Vol+ PL+SO4.ferm.+PO4.utilise; 0.235, 0.604, pH+Main.P-Source.Co nc.+P-Source.Feed.conc.+X.F.Suc+PL+rab+His+lactate+SO4.ferm. +PO4.ferm.+PO4.utilise; 0.249, 0.604, pH+tmp+Main.P-Source. Conc.+P-Source.Feed.conc.+X.F.Suc+Cell+PL+rab+SO4.ferm.+PO4. utilise; 0.249, 0.604, Main.P-Source.Conc.+P-Source.Feed.co nc.+Nitrogen.Conc.+X.F.Suc+PL+emission_O2+Thr+His+SO4.ferm. +PO4.utilise; 0.288, 0.604, Main.P-Source.Conc.+P-Source.Fe ed.conc.+X.F.Suc+PL+His+SO4.ferm.+PO4.utilise; 0.276, 0.604, pH+tmp+Main.P-Source.Conc.+P-Source.Feed.conc.+total.Vol+y ield+SO4.ferm.+PO4.utilise; 0.249, 0.604, pH+tmp+Main.P-Sou rce.Conc.+P-Source.Feed.conc.+X.F.Suc+total.Vol+rab+Thr+SO4. ferm.+PO4.utilise; 0.249, 0.604, pH+tmp+Main.P-Source.Conc. +P-Source.Feed.conc.+Nitrogen.Conc.+X.VXdt+yield+PL+SO4.fer m.+PO4.utilise; 0.275, 0.604, Main.P-Source.Conc.+P-Source. Feed.conc.+yield+PL+His+SO4.ferm.+PO4.ferm.+PO4.utilise; 0. 249, 0.604, tmp+Main.P-Source.Conc.+P-Source.Feed.conc.+Mai n.Thr.Conc.+X.F.Suc+total.Vol+PL+emission_O2+SO4.ferm.+PO4. utilise; 0.262, 0.604, tmp+Main.P-Source.Conc.+P-Source.Fee d.conc.+X.F.Suc+PL+emission_O2+lactate+SO4.ferm.+PO4.utilis e; 0.249, 0.604, tmp+Main.P-Source.Conc.+P-Source.Feed.conc. +Nitrogen.Conc.+X.F.Suc+PL+rab+His+SO4.ferm.+PO4.utilise; 0. 262, 0.604, tmp+Main.P-Source.Conc.+P-Source.Feed.conc.+X.F. Suc+total.Vol+Thr+His+SO4.ferm.+PO4.utilise; 0.235, 0.604, pH+Main.P-Source.Conc.+P-Source.Feed.conc.+X.F.Suc+X.VXdt+t otal.Vol+PL+emission_O2+Thr+SO4.ferm.+PO4.utilise; 0.235, 0. 604, Main.P-Source.Conc.+P-Source.Feed.conc.+X.F.Suc+X.VXdt +total.Vol+PL+emission_O2+His+SO4.ferm.+PO4.ferm.+PO4.utili se; 0.288, 0.604, Main.P-Source.Conc.+P-Source.Feed.conc.+y ield+emission_O2+SO4.ferm.+PO4.ferm.+PO4.utilise; 0.262, 0. 604, pH+Main.P-Source.Conc.+P-Source.Feed.conc.+yield+PL+ra b+His+SO4.ferm.+PO4.utilise; 0.249, 0.604, pH+Main.P-Source. Conc.+P-Source.Feed.conc.+X.F.Suc+Cell+total.Vol+PL+emissio n_O2+SO4.ferm.+PO4.utilise; 0.235, 0.604, pH+Main.P-Source. Conc.+P-Source.Feed.conc.+X.F.Suc+X.VXdt+PL+rab+Thr+SO4.fer m.+PO4.ferm.+PO4.utilise; 0.221, 0.604, pH+Main.P-Source.Co nc.+P-Source.Feed.conc.+X.F.Suc+X.VXdt+total.Vol+total.cell +PL+rab+His+SO4.ferm.+PO4.utilise; 0.262, 0.604, Main.P-Sou rce.Conc.+P-Source.Feed.conc.+OD+X.F.Suc+PL+rab+Thr+SO4.fer m.+PO4.utilise; 0.262, 0.604, pH+tmp+Main.P-Source.Conc.+P-Source.Feed.conc.+Lys conc.+total.Vol+PL+SO4.ferm.+PO4.util ise; 0.262, 0.604, Main.P-Source.Conc.+P-Source.Feed.conc.+ X.F.Suc+Cell+X.VXdt+PL+emission_O2+SO4.ferm.+PO4.utilise; 0. 249, 0.604, pH+Main.P-Source.Conc.+P-Source.Feed.conc.+X.F. Suc+total.Vol+PL+emission_O2+lactate+SO4.ferm.+PO4.utilise; 0.249, 0.604, Main.P-Source.Conc.+P-Source.Feed.conc.+X.F. Suc+X.VXdt+PL+rab+His+lactate+SO4.ferm.+PO4.utilise; 0.275, 0.604, pH+Main.P-Source.Conc.+P-Source.Feed.conc.+Lys_conc. +X.F.Suc+emission_O2+SO4.ferm.+PO4.utilise; 0.262, 0.604, p H+tmp+Main.P-Source.Conc.+P-Source.Feed.conc.+X.VXdt+yield+ PL+SO4.ferm.+PO4.utilise; 0.262, 0.604, pH+tmp+Main.P-Sourc e.Conc.+P-Source.Feed.conc.+X.F.Suc+total.Vol+Thr+SO4.ferm. +PO4.utilise; 0.262, 0.604, pH+Main.P-Source.Conc.+P-Source. Feed.conc.+X.F.Suc+X.VXdt+emission_O2+His+SO4.ferm.+PO4.uti lise; 0.275, 0.604, Main.P-Source.Conc.+P-Source.Feed.conc. +X.F.Suc+X.VXdt+total.Vol+PL+SO4.ferm.+PO4.utilise; 0.220, 0.604, pH+Main.P-Source.Conc.+P-Source.Feed.conc.+OD+X.F.Su c+Cell+total.Vol+total.cell+PL+rab+SO4.ferm.+PO4.utilise; 0. 249, 0.604, tmp+Main.P-Source.Conc.+P-Source.Feed.conc.+X.F. Suc+PL+Thr+His+SO4.ferm.+PO4.ferm.+PO4.utilise; 0.262, 0.60 4, Main.P-Source.Conc.+P-Source.Feed.conc.+X.F.Suc+Cell+PL+ emission_O2+His+SO4.ferm.+PO4.utilise; 0.262, 0.604, Main.P -Source.Conc.+P-Source.Feed.conc.+total.Vol+yield+PL+emissi on_O2+His+SO4.ferm.+PO4.utilise; 0.235, 0.604, pH+Main.P-So urce.Conc.+P-Source.Feed.conc.+Main.Thr.Conc.+X.F.Suc+PL+ra b+Thr+His+SO4.ferm.+PO4.utilise; 0.275, 0.604, Main.P-Sourc e.Conc.+P-Source.Feed.conc.+Nitrogen.Conc.+yield+PL+emissio n_O2+SO4.ferm.+PO4.utilise; 0.220, 0.604, pH+Main.P-Source. Conc.+P-Source.Feed.conc.+Nitrogen.Conc.+X.F.Suc+X.VXdt+tot al.Vol+PL+rab+SO4.ferm.+PO4.ferm.+PO4.utilise; 0.235, 0.604, pH+tmp+Main.P-Source.Conc.+P-Source.Feed.conc.+Nitrogen.Co nc.+X.F.Suc+total.Vol+PL+emission_O2+SO4.ferm.+PO4.utilise; 0.262, 0.604, Main.P-Source.Conc.+P-Source.Feed.conc.+OD+X. F.Suc+total.Vol+PL+rab+SO4.ferm.+PO4.utilise; 0.235, 0.604, pH+Main.P-Source.Conc.+P-Source.Feed.conc.+Main.Thr.Conc.+ X.F.Suc+X.VXdt+PL+emission_O2+SO4.ferm.+PO4.ferm.+PO4.utili se; 0.220, 0.604, pH+Main.P-Source.Conc.+P-Source.Feed.conc. +X.F.Suc+X.VXdt+PL+emission_O2+Thr+His+lactate+SO4.ferm.+PO 4.utilise; 0.220, 0.603, pH+Main.P-Source.Conc.+P-Source.Fe ed.conc.+Main.Thr.Conc.+OD+X.F.Suc+total.Vol+total.cell+PL+ rab+SO4.ferm.+PO4.utilise; 0.275, 0.603, tmp+Main.P-Source. Conc.+P-Source.Feed.conc.+X.F.Suc+His+SO4.ferm.+PO4.ferm.+P O4.utilise; 0.249, 0.603, pH+Main.P-Source.Conc.+P-Source.F eed.conc.+yield+PL+emission_O2+His+SO4.ferm.+PO4.ferm.+PO4. utilise; 0.275, 0.603, tmp+Main.P-Source.Conc.+P-Source.Fee d.conc.+Nitrogen.Conc.+X.F.Suc+rab+SO4.ferm.+PO4.utilise; 0. 262, 0.603, pH+tmp+Main.P-Source.Conc.+P-Source.Feed.conc.+ total.Vol+PL+rab+SO4.ferm.+PO4.utilise; 0.235, 0.603, tmp+M ain.P-Source.Conc.+P-Source.Feed.conc.+X.F.Suc+X.VXdt+total. Vol+PL+emission_O2+His+SO4.ferm.+PO4.utilise; 0.235, 0.603, pH+tmp+Main.P-Source.Conc.+P-Source.Feed.conc.+Main.Thr.Co nc.+X.F.Suc+PL+rab+SO4.ferm.+PO4.ferm.+PO4.utilise; 0.248, 0.603, pH+Main.P-Source.Conc.+P-Source.Feed.conc.+Nitrogen. Conc.+yield+PL+rab+SO4.ferm.+PO4.ferm.+PO4.utilise; 0.248, 0.603, Main.P-Source.Conc.+P-Source.Feed.conc.+Main.Thr.Con c.+X.F.Suc+PL+emission_O2+Thr+His+SO4.ferm.+PO4.utilise; 0. 205, 0.603, pH+Main.P-Source.Conc.+P-Source.Feed.conc.+OD+X. F.Suc+total.Vol+total.cell+PL+emission_O2+Thr+lactate+SO4.f erm.+PO4.utilise; 0.235, 0.603, tmp+Main.P-Source.Conc.+P-S ource.Feed.conc.+X.F.Suc+X.VXdt+PL+emission_O2+Thr+lactate+ SO4.ferm.+PO4.utilise; 0.248, 0.603, tmp+Main.P-Source.Conc. +P-Source.Feed.conc.+OD+X.F.Suc+X.VXdt+PL+rab+SO4.ferm.+PO4. utilise; 0.248, 0.603, pH+tmp+Main.P-Source.Conc.+P-Source. Feed.conc.+OD+X.F.Suc+X.VXdt+PL+SO4.ferm.+PO4.utilise; 0.20 5, 0.603, Main.P-Source.Conc.+P-Source.Feed.conc.+Main.Thr. Conc.+OD+Lys_conc.+Cell+total.Vol+total.cell+yield+PL+emiss ion_O2+SO4.ferm.+PO4.utilise; 0.234, 0.603, pH+tmp+Main.P-S ource.Conc.+P-Source.Feed.conc.+Main.Thr.Conc.+X.F.Suc+tota l.Vol+PL+rab+SO4.ferm.+PO4.utilise; 0.275, 0.603, Main.P-So urce.Conc.+P-Source.Feed.conc.+Nitrogen.Conc.+X.F.Suc+total. Vol+rab+SO4.ferm.+PO4.utilise; 0.220, 0.603, pH+Main.P-Sour ce.Conc.+P-Source.Feed.conc.+Main.Thr.Conc.+X.F.Suc+X.VXdt+ total.Vol+PL+rab+His+SO4.ferm.+PO4.utilise; 0.275, 0.603, M ain.P-Source.Conc.+P-Source.Feed.conc.+Lys_conc.+X.F.Suc+em ission_O2+His+SO4.ferm.+PO4.utilise; 0.220, 0.603, pH+tmp+M ain.P-Source.Conc.+P-Source.Feed.conc.+Nitrogen.Conc.+X.F.S uc+X.VXdt+total.Vol+PL+rab+SO4.ferm.+PO4.utilise; 0.220, 0. 603, pH+tmp+Main.P-Source.Conc.+P-Source.Feed.conc.+X.F.Suc +PL+rab+Thr+lactate+SO4.ferm.+PO4.ferm.+PO4.utilise; 0.234, 0.603, pH+Main.P-Source.Conc.+P-Source.Feed.conc.+Lys_conc. +X.F.Suc+PL+emission_O2+Thr+His+SO4.ferm.+PO4.utilise; 0.22 0, 0.603, pH+Main.P-Source.Conc.+P-Source.Feed.conc.+OD+X.F. Suc+total.Vol+total.cell+PL+emission_O2+lactate+SO4.ferm.+P O4.utilise; 0.262, 0.603, pH+tmp+Main.P-Source.Conc.+P-Sour ce.Feed.conc.+yield+PL+His+SO4.ferm.+PO4.utilise; 0.287, 0. 603, Main.P-Source.Conc.+P-Source.Feed.conc.+yield+PL+His+S O4.ferm.+PO4.utilise; 0.262, 0.603, Main.P-Source.Conc.+P-S ource.Feed.conc.+Nitrogen.Conc.+yield+PL+emission_O2+SO4.fe rm.+PO4.ferm.+PO4.utilise; 0.262, 0.603, Main.P-Source.Conc. +P-Source.Feed.conc.+X.F.Suc+Cell+PL+emission_O2+SO4.ferm.+ PO4.ferm.+PO4.utilise; 0.262, 0.603, pH+Main.P-Source.Conc. +P-Source.Feed.conc.+X.F.Suc+X.VXdt+rab+SO4.ferm.+PO4.ferm. +PO4.utilise; 0.275, 0.603, Main.P-Source.Conc.+P-Source.Fe ed.conc.+Lys_conc.+yield+emission_O2+His+SO4.ferm.+PO4.util ise; 0.234, 0.603, pH+Main.P-Source.Conc.+P-Source.Feed.con c.+Main.Thr.Conc.+X.F.Suc+total.Vol+PL+rab+Thr+SO4.ferm.+PO 4.utilise; 0.248, 0.603, tmp+Main.P-Source.Conc.+P-Source.F eed.conc.+Main.Thr.Conc.+X.F.Suc+PL+rab+SO4.ferm.+PO4.ferm. +PO4.utilise; 0.275, 0.603, pH+Main.P-Source.Conc.+P-Source. Feed.conc.+Main.Thr.Conc.+X.F.Suc+rab+SO4.ferm.+PO4.utilis e; 0.248, 0.603, Main.P-Source.Conc.+P-Source.Feed.conc.+X. F.Suc+X.VXdt+PL+emission_O2+Thr+SO4.ferm.+PO4.ferm.+PO4.uti lise; 0.234, 0.603, Main.P-Source.Conc.+P-Source.Feed.conc. +OD+X.F.Suc+total.Vol+total.cell+PL+emission_O2+SO4.ferm.+P O4.ferm.+PO4.utilise; 0.220, 0.603, pH+tmp+Main.P-Source.Co nc.+P-Source.Feed.conc.+X.F.Suc+X.VXdt+total.Vol+PL+rab+SO4. ferm.+PO4.ferm.+PO4.utilise; 0.274, 0.603, tmp+Main.P-Sourc e.Conc.+P-Source.Feed.conc.+total.Vol+yield+rab+SO4.ferm.+P O4.utilise; 0.205, 0.603, pH+Main.P-Source.Conc.+P-Source.F eed.conc.+OD+Lys_conc.+Cell+total.Vol+total.cell+yield+PL+r ab+SO4.ferm.+PO4.utilise; 0.234, 0.603, pH+Main.P-Source.Co nc.+P-Source.Feed.conc.+X.F.Suc+PL+emission_O2+Thr+His+SO4. ferm.+PO4.ferm.+PO4.utilise; 0.248, 0.603, tmp+Main.P-Sourc e.Conc.+P-Source.Feed.conc.+X.F.Suc+X.VXdt+PL+emission_O2+l actate+SO4.ferm.+PO4.utilise; 0.248, 0.603, pH+Main.P-Sourc e.Conc.+P-Source.Feed.conc.+X.F.Suc+X.VXdt+rab+Thr+lactate+ SO4.ferm.+PO4.utilise; 0.248, 0.603, tmp+Main.P-Source.Conc. +P-Source.Feed.conc.+X.F.Suc+total.cell+PL+rab+SO4.ferm.+PO 4.ferm.+PO4.utilise; 0.274, 0.603, pH+Main.P-Source.Conc.+P -Source.Feed.conc.+Main.Thr.Conc.+X.F.Suc+emission_O2+SO4.f erm.+PO4.utilise; 0.234, 0.603, pH+Main.P-Source.Conc.+P-So urce.Feed.conc.+X.F.Suc+X.VXdt+PL+rab+Thr+His+SO4.ferm.+PO4. utilise; 0.248, 0.603, Main.P-Source.Conc.+P-Source.Feed.co nc.+X.F.Suc+Cell+X.VXdt+total.Vol+PL+rab+SO4.ferm.+PO4.util ise; 0.248, 0.603, pH+Main.P-Source.Conc.+P-Source.Feed.con c.+OD+X.F.Suc+total.Vol+PL+rab+SO4.ferm.+PO4.utilise; 0.274, 0.603, tmp+Main.P-Source.Conc.+P-Source.Feed.conc.+Lys_con c.+yield+His+SO4.ferm.+PO4.utilise; 0.248, 0.603, Main.P-So urce.Cone.+P-Source.Feed.cone.+X.F.Suc+X.VXdt+PL+emission_O 2+Thr+His+SO4.ferm.+PO4.utilise; 0.248, 0.603, tmp+Main.P-S ource.Conc.+P-Source.Feed.conc.+total.Vol+yield+PL+rab+His+ SO4.ferm.+PO4.utilise; 0.274, 0.603, Main.P-Source.Conc.+P-Source.Feed.conc.+Nitrogen.Conc.+X.F.Suc+rab+His+SO4.ferm.+ PO4.utilise; 0.274, 0.603, tmp+Main.P-Source.Conc.+P-Source. Feed.conc.+Nitrogen.Conc.+yield+SO4.ferm.+PO4.ferm.+PO4.uti lise; 0.248, 0.603, Main.P-Source.Conc.+P-Source.Feed.conc. +X.F.Suc+PL+rab+Thr+His+lactate+SO4.ferm.+PO4.utilise; 0.24 8, 0.603, tmp+Main.P-Source.Conc.+P-Source.Feed.conc.+Nitro gen.Conc.+X.F.Suc+PL+emission_O2+SO4.ferm.+PO4.ferm.+PO4.ut ilise; 0.248, 0.603, pH+Main.P-Source.Conc.+P-Source.Feed.c onc.+X.F.Suc+Cell+PL+emission_O2+SO4.ferm.+PO4.ferm.+PO4.ut ilise; 0.248, 0.603, tmp+Main.P-Source.Conc.+P-Source.Feed. conc.+X.F.Suc+PL+rab+lactate+SO4.ferm.+PO4.ferm.+PO4.utilis e; 0.234, 0.603, pH+tmp+Main.P-Source.Conc.+P-Source.Feed.c onc.+Main.Thr.Conc.+X.F.Suc+total.Vol+PL+emission_O2+SO4.fe rm.+PO4.utilise; 0.261, 0.603, Main.P-Source.Conc.+P-Source. Feed.conc.+X.F.Suc+X.VXdt+total.Vol+emission_O2+His+SO4.fer m.+PO4.utilise; 0.248, 0.603, pH+tmp+Main.P-Source.Conc.+P-Source.Feed.conc.+X.F.Suc+emission_O2+Thr+His+SO4.ferm.+PO4. utilise; 0.234, 0.603, tmp+Main.P-Source.Conc.+P-Source.Fee d.conc.+X.F.Suc+total.Vol+PL+rab+Thr+SO4.ferm.+PO4.ferm.+PO 4.utilise; 0.248, 0.603, pH+Main.P-Source.Conc.+P-Source.Fe ed.conc.+X.F.Suc+Cell+PL+emission_O2+His+SO4.ferm.+PO4.util ise; 0.248, 0.603, pH+tmp+Main.P-Source.Conc.+P-Source.Feed. conc.+X.F.Suc+total.cell+PL+SO4.ferm.+PO4.ferm.+PO4.utilis e; 0.234, 0.603, pH+Main.P-Source.Conc.+P-Source.Feed.conc. +X.F.Suc+Cell+X.VXdt+total.Vol+PL+emission_O2+SO4.ferm.+PO4. utilise; 0.248, 0.603, Main.P-Source.Conc.+P-Source.Feed.co nc.+X.F.Suc+PL+emission_O2+Thr+His+lactate+SO4.ferm.+PO4.ut ilise; 0.261, 0.603, tmp+Main.P-Source.Conc.+P-Source.Feed. conc.+yield+PL+emission_O2+SO4.ferm.+PO4.ferm.+PO4.utilise; 0.261, 0.603, pH+Main.P-Source.Conc.+P-Source.Feed.conc.+X. F.Suc+emission_O2+Thr+SO4.ferm.+PO4.ferm.+PO4.utilise; 0.27 4, 0.603, Main.P-Source.Conc.+P-Source.Feed.conc.+X.F.Suc+r ab+His+lactate+SO4.ferm.+PO4.utilise; 0.234, 0.603, tmp+Mai n.P-Source.Conc.+P-Source.Feed.conc.+X.F.Suc+X.VXdt+PL+emis sion_O2+Thr+SO4.ferm.+PO4.ferm.+PO4.utilise; 0.234, 0.603, Main.P-Source.Conc.+P-Source.Feed.conc.+X.F.Suc+total.Vol+P L+rab+Thr+His+SO4.ferm.+PO4.ferm.+PO4.utilise; 0.248, 0.603, tmp+Main.P-Source.Conc.+P-Source.Feed.conc.+X.F.Suc+total. cell+PL+rab+His+SO4.ferm.+PO4.utilise; 0.261, 0.603, pH+Mai n.P-Source.Conc.+P-Source.Feed.conc.+yield+PL+emission_O2+H is+SO4.ferm.+PO4.utilise; 0.274, 0.603, tmp+Main.P-Source.C onc.+P-Source.Feed.conc.+X.F.Suc+Thr+SO4.ferm.+PO4.ferm.+PO 4.utilise; 0.234, 0.603, Main.P-Source.Conc.+P-Source.Feed. conc.+Nitrogen.Conc.+X.F.Suc+X.VXdt+total.Vol+PL+rab+His+SO 4.ferm.+PO4.utilise; 0.219, 0.603, pH+Main.P-Source.Conc.+P -Source.Feed.conc.+Main.Thr.Conc.+X.F.Suc+X.VXdt+total.Vol+ PL+rab+SO4.ferm.+PO4.ferm.+PO4.utilise; 0.234, 0.603, pH+Ma in.P-Source.Conc.+P-Source.Feed.conc.+OD+X.F.Suc+X.VXdt+tot al.cell+PL+rab+SO4.ferm.+PO4.utilise; 0.274, 0.603, Main.P-Source.Conc.+P-Source.Feed.conc.+X.F.Suc+X.VXdt+emission_O2 +Thr+SO4.ferm.+PO4.utilise; 0.219, 0.603, pH+Main.P-Source. Conc.+P-Source.Feed.conc.+X.F.Suc+X.VXdt+PL+emission_O2+Thr +lactate+SO4.ferm.+PO4.ferm.+PO4.utilise; 0.261, 0.603, Mai n.P-Source.Conc.+P-Source.Feed.conc.+OD+X.F.Suc+PL+emission _O2+SO4.ferm.+PO4.ferm.+PO4.utilise; 0.234, 0.603, Main.P-S ource.Conc.+P-Source.Feed.conc.+X.F.Suc+X.VXdt+total.Vol+PL +rab+Thr+SO4.ferm.+PO4.ferm.+PO4.utilise; 0.219, 0.603, pH+ Main.P-Source.Conc.+P-Source.Feed.conc.+X.F.Suc+X.VXdt+tota l.Vol+total.cell+PL+rab+SO4.ferm.+PO4.ferm.+PO4.utilise; 0. 261, 0.603, Main.P-Source.Conc.+P-Source.Feed.conc.+X.F.Suc +rab+Thr+His+SO4.ferm.+PO4.ferm.+PO4.utilise; 0.286, 0.603, Main.P-Source.Conc.+P-Source.Feed.conc.+X.F.Suc+Cell+rab+S O4.ferm.+PO4.utilise; 0.247, 0.603, Main.P-Source.Conc.+P-S ource.Feed.conc.+Main.Thr.Conc.+X.F.Suc+total.Vol+PL+emissi on_O2+Thr+SO4.ferm.+PO4.utilise; 0.247, 0.603, tmp+Main.P-S ource.Conc.+P-Source.Feed.conc.+X.F.Suc+X.VXdt+total.Vol+PL +His+SO4.ferm.+PO4.utilise; 0.274, 0.603, tmp+Main.P-Source. Conc.+P-Source.Feed.conc.+yield+PL+rab+SO4.ferm.+PO4.utilis e; 0.274, 0.603, Main.P-Source.Conc.+P-Source.Feed.conc.+Ly s_conc.+yield+rab+His+SO4.ferm.+PO4.utilise; 0.261, 0.603, pH+tmp+Main.P-Source.Conc.+P-Source.Feed.conc.+X.F.Suc+rab+ SO4.ferm.+PO4.ferm.+PO4.utilise; 0.233, 0.603, pH+Main.P-So urce.Cone.+P-Source.Feed.cone.+X.F.Suc+X.VXdt+PL+emission_O 2+His+lactate+SO4.ferm.+PO4.utilise; 0.261, 0.603, tmp+Main. P-Source.Conc.+P-Source.Feed.conc.+Main.Thr.Conc.+X.F.Suc+X. VXdt+PL+SO4.ferm.+PO4.utilise; 0.286, 0.603, tmp+Main.P-Sou rce.Conc.+P-Source.Feed.conc.+Nitrogen.Conc.+yield+SO4.ferm. +PO4.utilise; 0.286, 0.603, Main.P-Source.Conc.+P-Source.Fe ed.conc.+total.Vol+PL+emission_O2+SO4.ferm.+PO4.utilise; 0. 233, 0.603, Main.P-Source.Conc.+P-Source.Feed.conc.+OD+X.F. Suc+total.Vol+total.cell+PL+rab+His+SO4.ferm.+PO4.utilise; 0.286, 0.603, Main.P-Source.Conc.+P-Source.Feed.conc.+PL+ra b+SO4.ferm.+PO4.ferm.+PO4.utilise; 0.286, 0.602, pH+Main.P-Source.Conc.+P-Source.Feed.conc.+yield+His+SO4.ferm.+PO4.ut ilise; 0.261, 0.602, tmp+Main.P-Source.Conc.+P-Source.Feed. conc.+X.F.Suc+X.VXdt+emission_O2+His+SO4.ferm.+PO4.utilise; 0.261, 0.602, tmp+Main.P-Source.Conc.+P-Source.Feed.conc.+ Lys_conc.+yield+PL+rab+SO4.ferm.+PO4.utilise; 0.233, 0.602, pH+tmp+Main.P-Source.Conc.+P-Source.Feed.conc.+X.F.Suc+tot al.cell+PL+rab+Thr+SO4.ferm.+PO4.utilise; 0.247, 0.602, Mai n.P-Source.Conc.+P-Source.Feed.conc.+Main.Thr.Conc.+X.F.Suc +PL+rab+Thr+SO4.ferm.+PO4.ferm.+PO4.utilise; 0.204, 0.602, pH+tmp+Main.P-Source.Conc.+P-Source.Feed.conc.+X.F.Suc+X.VX dt+PL+rab+Thr+lactate+SO4.ferm.+PO4.ferm.+PO4.utilise; 0.26 0, 0.602, tmp+Main.P-Source.Conc.+P-Source.Feed.conc.+OD+X. F.Suc+PL+rab+SO4.ferm.+PO4.utilise; 0.260, 0.602, tmp+Main. P-Source.Conc.+P-Source.Feed.conc.+total.Vol+PL+rab+His+SO4. ferm.+PO4.utilise; 0.233, 0.602, pH+tmp+Main.P-Source.Conc. +P-Source.Feed.conc.+X.F.Suc+total.cell+PL+rab+SO4.ferm.+PO 4.ferm.+PO4.utilise; 0.260, 0.602, tmp+Main.P-Source.Conc.+ P-Source.Feed.conc.+X.F.Suc+X.VXdt+rab+Thr+SO4.ferm.+PO4.ut ilise; 0.247, 0.602, Main.P-Source.Conc.+P-Source.Feed.conc. +Main.Thr.Conc.+X.F.Suc+X.VXdt+PL+rab+SO4.ferm.+PO4.ferm.+P O4.utilise; 0.260, 0.602, pH+tmp+Main.P-Source.Conc.+P-Sour ce.Feed.conc.+total.Vol+yield+His+SO4.ferm.+PO4.utilise; 0. 273, 0.602, pH+Main.P-Source.Conc.+P-Source.Feed.conc.+X.F. Suc+PL+His+SO4.ferm.+PO4.utilise; 0.260, 0.602, pH+Main.P-S ource.Conc.+P-Source.Feed.conc.+Nitrogen.Conc.+X.F.Suc+rab+ Thr+SO4.ferm.+PO4.utilise; 0.233, 0.602, pH+tmp+Main.P-Sour ce.Conc.+P-Source.Feed.conc.+Main.Thr.Conc.+X.F.Suc+PL+emis sion_O2+SO4.ferm.+PO4.ferm.+PO4.utilise; 0.260, 0.602, Main. P-Source.Conc.+P-Source.Feed.conc.+X.F.Suc+total.Vol+PL+emi ssion_O2+lactate+SO4.ferm.+PO4.utilise; 0.260, 0.602, Main. P-Source.Conc.+P-Source.Feed.conc.+Lys_conc.+yield+PL+emiss ion_O2+His+SO4.ferm.+PO4.utilise; 0.247, 0.602, tmp+Main.P-Source.Conc.+P-Source.Feed.conc.+yield+PL+rab+His+SO4.ferm. +PO4.ferm.+PO4.utilise; 0.260, 0.602, tmp+Main.P-Source.Con c.+P-Source.Feed.conc.+X.F.Suc+emission_O2+Thr+SO4.ferm.+PO 4.ferm.+PO4.utilise; 0.247, 0.602, pH+tmp+Main.P-Source.Con c.+P-Source.Feed.conc.+total.Vol+yield+PL+emission_O2+SO4.f erm.+PO4.utilise; 0.247, 0.602, pH+Main.P-Source.Conc.+P-So urce.Feed.conc.+OD+X.F.Suc+PL+rab+Thr+SO4.ferm.+PO4.utilis e; 0.273, 0.602, pH+Main.P-Source.Conc.+P-Source.Feed.conc. +Lys_conc.+X.F.Suc+rab+SO4.ferm.+PO4.utilise; 0.260, 0.602, tmp+Main.P-Source.Conc.+P-Source.Feed.conc.+Nitrogen.Conc. +total.Vol+yield+PL+SO4.ferm.+PO4.utilise; 0.218, 0.602, pH +Main.P-Source.Conc.+P-Source.Feed.conc.+OD+X.F.Suc+total.V ol+total.cell+PL+emission_O2+His+SO4.ferm.+PO4.utilise; 0.2 18, 0.602, pH+tmp+Main.P-Source.Conc.+P-Source.Feed.conc.+X. F.Suc+X.VXdt+PL+rab+Thr+His+SO4.ferm.+PO4.utilise; 0.233, 0. 602, Main.P-Source.Conc.+P-Source.Feed.conc.+X.F.Suc+X.VXdt +PL+rab+Thr+lactate+SO4.ferm.+PO4.ferm.+PO4.utilise; 0.260, 0.602, Main.P-Source.Conc.+P-Source.Feed.conc.+Lys_conc.+y ield+PL+rab+His+SO4.ferm.+PO4.utilise; 0.218, 0.602, pH+Mai n.P-Source.Conc.+P-Source.Feed.conc.+Nitrogen.Conc.+OD+X.F. Suc+total.Vol+total.cell+PL+rab+SO4.ferm.+PO4.utilise; 0.24 7, 0.602, tmp+Main.P-Source.Conc.+P-Source.Feed.conc.+Nitro gen.Conc.+yield+PL+His+SO4.ferm.+PO4.ferm.+PO4.utilise; 0.2 60, 0.602, Main.P-Source.Conc.+P-Source.Feed.conc.+OD+X.F.S uc+total.cell+PL+emission_O2+SO4.ferm.+PO4.utilise; 0.260, 0.602, tmp+Main.P-Source.Conc.+P-Source.Feed.conc.+X.F.Suc+ total.cell+PL+SO4.ferm.+PO4.ferm.+PO4.utilise; 0.273, 0.602, Main.P-Source.Conc.+P-Source.Feed.conc.+X.F.Suc+total.cell +rab+Thr+SO4.ferm.+PO4.utilise; 0.233, 0.602, pH+tmp+Main.P -Source.Conc.+P-Source.Feed.conc.+Nitrogen.Conc.+X.F.Suc+X. VXdt+PL+His+SO4.ferm.+PO4.utilise; 0.260, 0.602, pH+Main.P-Source.Conc.+P-Source.Feed.conc.+X.F.Suc+emission_O2+His+SO 4.ferm.+PO4.ferm.+PO4.utilise; 0.218, 0.602, pH+tmp+Main.P-Source.Conc.+P-Source.Feed.conc.+Nitrogen.Conc.+X.F.Suc+X.V Xdt+PL+emission_O2+SO4.ferm.+PO4.ferm.+PO4.utilise; 0.171, 0.602, pH+Main.P-Source.Conc.+P-Source.Feed.conc.+Main.Thr. Conc.+OD+Lys_conc.+Cell+total.Vol+total.cell+yield+PL+emiss ion_O2+SO4.ferm.+PO4.ferm.+PO4.utilise; 0.218, 0.602, pH+Ma in.P-Source.Conc.+P-Source.Feed.conc.+OD+X.F.Suc+X.VXdt+tot al.Vol+PL+rab+SO4.ferm.+PO4.ferm.+PO4.utilise; 0.273, 0.602, Main.P-Source.Conc.+P-Source.Feed.conc.+yield+rab+His+SO4. ferm.+PO4.ferm.+PO4.utilise; 0.273, 0.602, Main.P-Source.Co nc.+P-Source.Feed.conc.+Lys_conc.+X.F.Suc+rab+His+SO4.ferm. +PO4.utilise; 0.260, 0.602, Main.P-Source.Conc.+P-Source.Fe ed.conc.+X.F.Suc+X.VXdt+emission_O2+Thr+lactate+SO4.ferm.+P O4.utilise; 0.285, 0.602, pH+Main.P-Source.Conc.+P-Source.F eed.conc.+yield+rab+SO4.ferm.+PO4.utilise; 0.260, 0.602, tm p+Main.P-Source.Conc.+P-Source.Feed.conc.+Main.Thr.Conc.+X. F.Suc+total.Vol+PL+SO4.ferm.+PO4.utilise; 0.273, 0.602, Mai n.P-Source.Conc.+P-Source.Feed.conc.+X.F.Suc+X.VXdt+rab+lac tate+SO4.ferm.+PO4.utilise; 0.246, 0.602, pH+tmp+Main.P-Sou rce.Conc.+P-Source.Feed.conc.+Main.Thr.Conc.+X.F.Suc+total. Vol+PL+SO4.ferm.+PO4.utilise; 0.246, 0.602, tmp+Main.P-Sour ce.Cone.+P-Source.Feed.conc.+X.F.Suc+X.VXdt+PL+Thr+His+SO4. ferm.+PO4.utilise; 0.285, 0.602, pH+Main.P-Source.Conc.+P-S ource.Feed.conc.+X.F.Suc+total.Vol+SO4.ferm.+PO4.utilise; 0. 203, 0.602, pH+tmp+Main.P-Source.Conc.+P-Source.Feed.conc.+ X.F.Suc+X.VXdt+PL+emission_O2+Thr+His+lactate+SO4.ferm.+PO4. utilise; 0.246, 0.602, Main.P-Source.Conc.+P-Source.Feed.co nc.+Nitrogen.Conc.+X.F.Suc+total.Vol+PL+rab+His+SO4.ferm.+P O4.utilise; 0.273, 0.602, tmp+Main.P-Source.Conc.+P-Source. Feed.conc.+Nitrogen.Conc.+yield+PL+SO4.ferm.+PO4.utilise; 0. 232, 0.602, Main.P-Source.Conc.+P-Source.Feed.conc.+X.F.Suc +X.VXdt+total.Vol+PL+emission_O2+Thr+lactate+SO4.ferm.+PO4. utilise; 0.246, 0.602, tmp+Main.P-Source.Conc.+P-Source.Fee d.conc.+Main.Thr.Conc.+X.F.Suc+PL+emission_O2+SO4.ferm.+PO4. ferm.+PO4.utilise; 0.232, 0.602, pH+tmp+Main.P-Source.Conc. +P-Source.Feed.conc.+X.F.Suc+PL+rab+Thr+lactate+SO4.ferm.+P O4.utilise; 0.218, 0.602, pH+Main.P-Source.Conc.+P-Source.F eed.conc.+Nitrogen.Conc.+X.F.Suc+X.VXdt+total.Vol+PL+emissi on_O2+His+SO4.ferm.+PO4.utilise; 0.232, 0.602, pH+tmp+Main. P-Source.Conc.+P-Source.Feed.conc.+X.F.Suc+total.Vol+PL+rab +lactate+SO4.ferm.+PO4.utilise; 0.218, 0.602, pH+tmp+Main.P -Source.Conc.+P-Source.Feed.conc.+Main.Thr.Conc.+X.F.Suc+X. VXdt+total.Vol+PL+emission_O2+SO4.ferm.+PO4.utilise; 0.246, 0.602, pH+Main.P-Source.Conc.+P-Source.Feed.conc.+OD+X.F.S uc+PL+emission_O2+SO4.ferm.+PO4.ferm.+PO4.utilise; 0.232, 0. 602, pH+Main.P-Source.Conc.+P-Source.Feed.conc.+OD+X.F.Suc+ X.VXdt+PL+rab+SO4.ferm.+PO4.ferm.+PO4.utilise; 0.232, 0.602, pH+tmp+Main.P-Source.Conc.+P-Source.Feed.conc.+X.F.Suc+PL+ emission_O2+lactate+SO4.ferm.+PO4.ferm.+PO4.utilise; 0.260, 0.602, pH+tmp+Main.P-Source.Conc.+P-Source.Feed.conc.+X.F. Suc+X.VXdt+emission_O2+SO4.ferm.+PO4.utilise; 0.246, 0.602, pH+tmp+Main.P-Source.Conc.+P-Source.Feed.conc.+Main.Thr.Co nc.+X.F.Suc+PL+SO4.ferm.+PO4.ferm.+PO4.utilise; 0.260, 0.60 2, Main.P-Source.Conc.+P-Source.Feed.conc.+X.F.Suc+Cell+tot al.cell+PL+rab+SO4.ferm.+PO4.utilise; 0.232, 0.602, tmp+Mai n.P-Source.Conc.+P-Source.Feed.conc.+Main.Thr.Conc.+X.F.Suc +total.Vol+PL+rab+Thr+SO4.ferm.+PO4.utilise; 0.260, 0.602, tmp+Main.P-Source.Conc.+P-Source.Feed.conc.+X.F.Suc+rab+His +SO4.ferm.+PO4.ferm.+PO4.utilise; 0.218, 0.602, pH+Main.P-S ource.Conc.+P-Source.Feed.conc.+X.F.Suc+X.VXdt+total.Vol+PL +rab+Thr+His+SO4.ferm.+PO4.utilise; 0.273, 0.602, pH+Main.P -Source.Conc.+P-Source.Feed.conc.+X.F.Suc+total.cell+rab+SO 4.ferm.+PO4.utilise; 0.260, 0.602, Main.P-Source.Conc.+P-So urce.Feed.conc.+OD+X.F.Suc+PL+rab+His+SO4.ferm.+PO4.utilis e; 0.246, 0.602, pH+Main.P-Source.Conc.+P-Source.Feed.conc. +X.F.Suc+total.cell+PL+emission_O2+Thr+SO4.ferm.+PO4.utilis e; 0.246, 0.602, Main.P-Source.Conc.+P-Source.Feed.conc.+Ma in.Thr.Conc.+X.F.Suc+X.VXdt+PL+rab+His+SO4.ferm.+PO4.utilis e; 0.246, 0.602, Main.P-Source.Conc.+P-Source.Feed.conc.+Ni trogen.Conc.+X.F.Suc+PL+rab+Thr+SO4.ferm.+PO4.ferm.+PO4.uti lise; 0.272, 0.602, Main.P-Source.Conc.+P-Source.Feed.conc. +X.F.Suc+X.VXdt+total.cell+rab+SO4.ferm.+PO4.utilise; 0.259, 0.602, Main.P-Source.Conc.+P-Source.Feed.conc.+X.F.Suc+tot al.Vol+rab+Thr+SO4.ferm.+PO4.ferm.+PO4.utilise; 0.232, 0.60 2, pH+tmp+Main.P-Source.Conc.+P-Source.Feed.conc.+X.F.Suc+t otal.cell+PL+rab+His+SO4.ferm.+PO4.utilise; 0.218, 0.602, p H+tmp+Main.P-Source.Conc.+P-Source.Feed.conc.+X.F.Suc+X.VXd t+PL+rab+Thr+SO4.ferm.+PO4.ferm.+PO4.utilise; 0.232, 0.602, pH+Main.P-Source.Conc.+P-Source.Feed.conc.+X.F.Suc+total.V ol+PL+rab+lactate+SO4.ferm.+PO4.ferm.+PO4.utilise; 0.218, 0. 602, pH+tmp+Main.P-Source.Conc.+P-Source.Feed.conc.+Nitroge n.Conc.+X.F.Suc+total.Vol+PL+rab+Thr+SO4.ferm.+PO4.utilise; 0.232, 0.602, pH+tmp+Main.P-Source.Conc.+P-Source.Feed.con c.+Nitrogen.Conc.+X.F.Suc+PL+emission_O2+SO4.ferm.+PO4.ferm. +PO4.utilise; 0.246, 0.602, pH+tmp+Main.P-Source.Conc.+P-So urce.Feed.conc.+X.F.Suc+total.Vol+rab+His+SO4.ferm.+PO4.uti lise; 0.285, 0.602, Main.P-Source.Conc.+P-Source.Feed.conc. +Nitrogen.Conc.+yield+rab+SO4.ferm.+PO4.utilise; 0.217, 0.6 02, pH+tmp+Main.P-Source.Conc.+P-Source.Feed.conc.+X.F.Suc+ X.VXdt+PL+rab+lactate+SO4.ferm.+PO4.ferm.+PO4.utilise; 0.21 7, 0.602, pH+tmp+Main.P-Source.Conc.+P-Source.Feed.conc.+Ni trogen.Conc.+X.F.Suc+X.VXdt+PL+emission_O2+His+SO4.ferm.+PO 4.utilise; 0.232, 0.602, pH+tmp+Main.P-Source.Conc.+P-Sourc e.Feed.conc.+Nitrogen.Conc.+X.F.Suc+PL+rab+His+SO4.ferm.+PO 4.utilise; 0.259, 0.602, pH+Main.P-Source.Conc.+P-Source.Fe ed.conc.+X.F.Suc+total.Vol+rab+SO4.ferm.+PO4.ferm.+PO4.util ise; 0.217, 0.602, pH+Main.P-Source.Conc.+P-Source.Feed.con c.+OD+X.F.Suc+X.VXdt+total.Vol+PL+rab+His+SO4.ferm.+PO4.uti lise; 0.232, 0.602, pH+tmp+Main.P-Source.Conc.+P-Source.Fee d.conc.+Nitrogen.Conc.+X.F.Suc+X.VXdt+PL+SO4.ferm.+PO4.ferm. +PO4.utilise; 0.285, 0.602, Main.P-Source.Conc.+P-Source.Fe ed.conc.+OD+X.F.Suc+rab+SO4.ferm.+PO4.utilise; 0.259, 0.602, tmp+Main.P-Source.Conc.+P-Source.Feed.conc.+Lys_conc.+tota l.Vol+PL+His+SO4.ferm.+PO4.utilise; 0.232, 0.602, pH+Main.P -Source.Conc.+P-Source.Feed.conc.+Nitrogen.Conc.+X.F.Suc+PL +emission_O2+Thr+His+SO4.ferm.+PO4.utilise; 0.272, 0.602, p H+Main.P-Source.Conc.+P-Source.Feed.conc.+PL+rab+SO4.ferm.+ PO4.ferm.+PO4.utilise; 0.232, 0.602, pH+Main.P-Source.Conc. +P-Source.Feed.conc.+Nitrogen.Conc.+X.F.Suc+X.VXdt+PL+emiss ion_O2+SO4.ferm.+PO4.ferm.+PO4.utilise; 0.246, 0.602, pH+Ma in.P-Source.Conc.+P-Source.Feed.conc.+X.F.Suc+X.VXdt+PL+emi ssion_O2+rab+SO4.ferm.+PO4.utilise; 0.232, 0.602, pH+tmp+Ma in.P-Source.Conc.+P-Source.Feed.conc.+X.F.Suc+X.VXdt+PL+Thr +lactate+SO4.ferm.+PO4.utilise; 0.232, 0.601, pH+Main.P-Sou rce.Conc.+P-Source.Feed.conc.+X.F.Suc+X.VXdt+total.cell+PL+ emission_O2+SO4.ferm.+PO4.ferm.+PO4.utilise; 0.202, 0.601, pH+Main.P-Source.Conc.+P-Source.Feed.conc.+X.F.Suc+X.VXdt+t otal.Vol+PL+rab+Thr+lactate+SO4.ferm.+PO4.ferm.+PO4.utilis e; 0.246, 0.601, tmp+Main.P-Source.Conc.+P-Source.Feed.conc. +Nitrogen.Conc.+yield+PL+rab+SO4.ferm.+PO4.ferm.+PO4.utilis e; 0.246, 0.601, pH+Main.P-Source.Conc.+P-Source.Feed.conc. +X.F.Suc+PL+emission_O2+rab+SO4.ferm.+PO4.ferm.+PO4.utilis e; 0.246, 0.601, pH+tmp+Main.P-Source.Conc.+P-Source.Feed.c onc.+Nitrogen.Conc.+total.Vol+yield+PL+SO4.ferm.+PO4.utilis e; 0.232, 0.601, tmp+Main.P-Source.Conc.+P-Source.Feed.conc. +X.F.Suc+X.VXdt+total.Vol+PL+rab+SO4.ferm.+PO4.ferm.+PO4.ut ilise; 0.246, 0.601, pH+tmp+Main.P-Source.Conc.+P-Source.Fe ed.conc.+X.F.Suc+X.VXdt+PL+lactate+SO4.ferm.+PO4.utilise; 0. 217, 0.601, pH+tmp+Main.P-Source.Conc.+P-Source.Feed.conc.+ X.F.Suc+X.VXdt+total.cell+PL+rab+His+SO4.ferm.+PO4.utilise; 0.285, 0.601, pH+Main.P-Source.Conc.+P-Source.Feed.conc.+y ield+emission_O2+SO4.ferm.+PO4.utilise; 0.259, 0.601, tmp+M ain.P-Source.Conc.+P-Source.Feed.conc.+Lys_conc.+yield+PL+H is+SO4.ferm.+PO4.utilise; 0.246, 0.601, tmp+Main.P-Source.C onc.+P-Source.Feed.conc.+X.F.Suc+total.Vol+emission_O2+Thr+ His+SO4.ferm.+PO4.utilise; 0.202, 0.601, pH+Main.P-Source.C onc.+P-Source.Feed.conc.+X.F.Suc+X.VXdt+total.Vol+PL+emissi on_O2+Thr+His+lactate+SO4.ferm.+PO4.utilise; 0.217, 0.601, pH+tmp+Main.P-Source.Conc.+P-Source.Feed.conc.+Main.Thr.Con c.+X.F.Suc+X.VXdt+PL+rab+SO4.ferm.+PO4.ferm.+PO4.utilise; 0. 246, 0.601, Main.P-Source.Conc.+P-Source.Feed.conc.+X.F.Suc +total.cell+PL+rab+His+SO4.ferm.+PO4.ferm.+PO4.utilise; 0.2 72, 0.601, pH+tmp+Main.P-Source.Conc.+P-Source.Feed.conc.+y ield+SO4.ferm.+PO4.ferm.+PO4.utilise; 0.246, 0.601, pH+Main. P-Source.Conc.+P-Source.Feed.conc.+X.F.Suc+Cell+total.cell+ PL+rab+SO4.ferm.+PO4.utilise; 0.232, 0.601, pH+Main.P-Sourc e.Conc.+P-Source.Feed.conc.+Main.Thr.Conc.+X.F.Suc+PL+rab+T hr+SO4.ferm.+PO4.ferm.+PO4.utilise; 0.232, 0.601, tmp+Main. P-Source.Conc.+P-Source.Feed.conc.+Nitrogen.Conc.+X.F.Suc+X. VXdt+total.Vol+PL+rab+SO4.ferm.+PO4.utilise; 0.245, 0.601, Main.P-Source.Conc.+P-Source.Feed.conc.+X.F.Suc+PL+rab+His+ lactate+SO4.ferm.+PO4.ferm.+PO4.utilise; 0.259, 0.601, tmp+ Main.P-Source.Conc.+P-Source.Feed.conc.+X.F.Suc+total.cell+ PL+Thr+SO4.ferm.+PO4.utilise; 0.259, 0.601, pH+Main.P-Sourc e.Conc.+P-Source.Feed.conc.+Main.Thr.Conc.+X.F.Suc+rab+Thr+ SO4.ferm.+PO4.utilise; 0.231, 0.601, pH+Main.P-Source.Conc. +P-Source.Feed.conc.+Main.Thr.Conc.+X.F.Suc+PL+emission_O2+ Thr+His+SO4.ferm.+PO4.utilise; 0.259, 0.601, pH+Main.P-Sour ce.Conc.+P-Source.Feed.conc.+yield+PL+His+SO4.ferm.+PO4.fer m.+PO4.utilise; 0.272, 0.601, Main.P-Source.Conc.+P-Source. Feed.conc.+total.Vol+PL+rab+His+SO4.ferm.+PO4.utilise; 0.28 4, 0.601, tmp+Main.P-Source.Conc.+P-Source.Feed.conc.+Main. Thr.Conc.+X.F.Suc+SO4.ferm.+PO4.utilise; 0.259, 0.601, tmp+ Main.P-Source.Conc.+P-Source.Feed.conc.+total.Vol+yield+rab +His+SO4.ferm.+PO4.utilise; 0.231, 0.601, pH+Main.P-Source. Conc.+P-Source.Feed.conc.+Nitrogen.Conc.+X.F.Suc+total.Vol+ PL+emission_O2+Thr+SO4.ferm.+PO4.utilise; 0.245, 0.601, Mai n.P-Source.Conc.+P-Source.Feed.conc.+X.F.Suc+X.VXdt+PL+rab+ lactate+SO4.ferm.+PO4.ferm.+PO4.utilise; 0.272, 0.601, tmp+ Main.P-Source.Conc.+P-Source.Feed.conc.+total.Vol+PL+emissi on_O2+SO4.ferm.+PO4.utilise; 0.245, 0.601, Main.P-Source.Co nc.+P-Source.Feed.conc.+Nitrogen.Conc.+X.F.Suc+total.Vol+PL +rab+SO4.ferm.+PO4.ferm.+PO4.utilise; 0.259, 0.601, Main.P-Source.Conc.+P-Source.Feed.conc.+X.F.Suc+X.VXdt+total.Vol+r ab+Thr+SO4.ferm.+PO4.utilise; 0.245, 0.601, tmp+Main.P-Sour ce.Conc.+P-Source.Feed.conc.+Main.Thr.Conc.+X.F.Suc+PL+rab+ His+SO4.ferm.+PO4.utilise; 0.231, 0.601, Main.P-Source.Conc. +P-Source.Feed.conc.+OD+X.F.Suc+X.VXdt+total.Vol+total.cell +PL+emission_O2+SO4.ferm.+PO4.utilise; 0.284, 0.601, tmp+Ma in.P-Source.Conc.+P-Source.Feed.conc.+yield+rab+SO4.ferm.+P O4.utilise; 0.217, 0.601, pH+tmp+Main.P-Source.Conc.+P-Sour ce.Feed.conc.+X.F.Suc+X.VXdt+PL+emission_O2+His+lactate+SO4. ferm.+PO4.utilise; 0.259, 0.601, pH+tmp+Main.P-Source.Conc. +P-Source.Feed.conc.+X.F.Suc+Cell+PL+SO4.ferm.+PO4.utilise; 0.217, 0.601, pH+Main.P-Source.Conc.+P-Source.Feed.conc.+M ain.Thr.Conc.+X.F.Suc+X.VXdt+total.Vol+PL+emission_O2+SO4.f erm.+PO4.ferm.+PO4.utilise; 0.272, 0.601, Main.P-Source.Con c.+P-Source.Feed.conc.+X.F.Suc+rab+lactate+SO4.ferm.+PO4.fe rm.+PO4.utilise

### [15. Linear Model that Predicts Lysine Production Amount in Interval 5]

0.610, 0.826, pH+tmp+Nitrogen.Conc.+OD+Lys_conc.+X.VXdt+tot al.cell+emission_O2+rab+Thr+lactate+PO4.ferm.; 0.600, 0.825, pH+tmp+Nitrogen.Conc.+OD+Lys_conc.+X.VXdt+total.cell+PL+em ission_O2+rab+Thr+lactate+PO4.ferm.; 0.598, 0.824, pH+tmp+N itrogen.Conc.+OD+Lys_conc.+X.F.Suc+X.VXdt+total.cell+emissi on_O2+rab+Thr+lactate+PO4.ferm.; 0.612, 0.823, tmp+Nitrogen. Conc.+OD+Lys_conc.+X.VXdt+total.cell+emission_O2+rab+Thr+la ctate+PO4.ferm.; 0.588, 0.822, pH+tmp+Nitrogen.Conc.+OD+Lys conc.+X.VXdt+total.Vol+total.cell+PL+emission_O2+rab+Thr+l actate+PO4.ferm.; 0.595, 0.822, pH+tmp+Nitrogen.Conc.+Lys_c onc.+X.VXdt+total.Vol+total.cell+PL+emission_O2+rab+Thr+lac tate+PO4.ferm.; 0.595, 0.822, pH+tmp+Nitrogen.Conc.+OD+Lys_ conc.+X.VXdt+total.Vol+total.cell+emission_O2+rab+Thr+lacta te+PO4.ferm.; 0.617, 0.822, pH+tmp+Nitrogen.Conc.+Lys_conc. +X.VXdt+total.Vol+emission_O2+rab+lactate+PO4.ferm.; 0.609, 0.822, pH+tmp+P-Source.Feed.conc.+Nitrogen.Conc.+Lys_conc. +X.VXdt+total.Vol+emission_O2+rab+lactate+PO4.ferm.; 0.601, 0.821, pH+tmp+OD+Lys_conc.+X.VXdt+total.cell+PL+emission_O 2+rab+Thr+lactate+PO4.ferm.; 0.601, 0.821, pH+tmp+Nitrogen. Conc.+Lys_conc.+X.VXdt+total.Vol+total.cell+emission_O2+rab +Thr+lactate+PO4.ferm.; 0.593, 0.821, pH+tmp+Nitrogen.Conc. +OD+Lys_conc.+X.VXdt+total.cell+emission_O2+rab+Thr+His+lac tate+PO4.ferm.; 0.600, 0.821, pH+tmp+Nitrogen.Conc.+Lys_con c.+X.VXdt+total.Vol+PL+emission_O2+rab+Thr+lactate+PO4.fer m.; 0.608, 0.821, pH+tmp+Nitrogen.Conc.+Lys_conc.+X.VXdt+to tal.Vol+emission_O2+rab+Thr+lactate+PO4.ferm.; 0.613, 0.820, tmp+OD+Lys_conc.+X.VXdt+total.cell+emission_O2+rab+Thr+lac tate+PO4.ferm.; 0.606, 0.820, pH+tmp+Nitrogen.Conc.+Lys_con c.+X.F.Suc+X.VXdt+total.Vol+emission_O2+rab+lactate+PO4.fer m.; 0.599, 0.820, tmp+Nitrogen.Conc.+OD+Lys_conc.+X.F.Suc+X. VXdt+total.cell+emission_O2+rab+Thr+lactate+PO4.ferm.; 0.60 6, 0.820, pH+tmp+Nitrogen.Conc.+Lys_conc.+X.VXdt+total.Vol+ emission_O2+rab+His+lactate+PO4.ferm.; 0.582, 0.820, tmp+Ni trogen.Conc.+Lys_conc.+X.VXdt+total.Vol+yield+PL+emission_O 2+rab+Thr+lactate+PO4.ferm.+PO4.apporte+PO4.utilise; 0.598, 0.820, pH+tmp+P-Source.Feed.conc.+Nitrogen.Cone.+Lys_conc. +X.VXdt+total.Vol+emission_O2+rab+His+lactate+PO4.ferm.; 0. 597, 0.820, Nitrogen.Conc.+Lys_conc.+X.VXdt+total.Vol+yield +emission_O2+rab+Thr+lactate+PO4.ferm.+PO4.apporte+PO4.util ise; 0.589, 0.819, pH+tmp+Nitrogen.Conc.+OD+Lys_conc.+X.VXd t+total.Vol+PL+emission_O2+rab+Thr+lactate+PO4.ferm.; 0.604, 0.819, pH+tmp+OD+Lys_conc.+X.VXdt+total.cell+emission_O2+r ab+Thr+lactate+PO4.ferm.; 0.581, 0.819, pH+tmp+Nitrogen.Con c.+OD+Lys_conc.+X.VXdt+total.cell+PL+emission_O2+rab+Thr+Hi s+lactate+PO4.ferm.; 0.604, 0.819, pH+tmp+OD+Lys_conc.+X.VX dt+total.cell+PL+emission_O2+rab+Thr+PO4.ferm.; 0.581, 0.81 9, pH+tmp+Nitrogen.Conc.+OD+Lys_conc.+X.F.Suc+X.VXdt+total. cell+emission_O2+rab+Thr+His+lactate+PO4.ferm.; 0.589, 0.81 9, Nitrogen.Conc.+Lys_conc.+X.VXdt+total.Vol+yield+PL+emiss ion_O2+rab+Thr+lactate+PO4.ferm.+PO4.apporte+PO4.utilise; 0. 604, 0.819, Nitrogen.Conc.+Lys_conc.+X.VXdt+total.Vol+yield +emission_O2+rab+Thr+lactate+PO4.ferm.+PO4.apporte; 0.617, 0.818, tmp+Nitrogen.Conc.+OD+Lys_conc.+X.VXdt+total.cell+Th r+lactate+PO4.ferm.; 0.595, 0.818, tmp+Nitrogen.Conc.+OD+Ly s_conc.+X.VXdt+total.cell+emission_O2+rab+Thr+His+lactate+P O4.ferm.; 0.595, 0.818, pH+tmp+Nitrogen.Conc.+OD+Lys_conc.+ X.VXdt+total.Vol+emission_O2+rab+Thr+lactate+PO4.ferm.; 0.5 95, 0.818, tmp+Nitrogen.Conc.+OD+Lys conc.+X.VXdt+total.cel l+PL+emission_O2+rab+Thr+lactate+PO4.ferm.; 0.602, 0.818, t mp+OD+Lys_conc.+X.VXdt+total.cell+PL+emission_O2+rab+Thr+la ctate+PO4.ferm.; 0.602, 0.818, tmp+Nitrogen.Conc.+Lys_conc. +X.VXdt+total.Vol+total.cell+emission_O2+rab+Thr+lactate+PO 4.ferm.; 0.586, 0.818, pH+tmp+Main.Thr.Conc.+Nitrogen.Conc. +OD+Lys_conc.+X.VXdt+total.cell+emission_O2+rab+Thr+lactate +PO4.ferm.; 0.578, 0.818, pH+tmp+Nitrogen.Conc.+OD+Lys_conc. +X.F.Suc+X.VXdt+total.cell+PL+emission_O2+rab+Thr+lactate+P O4.ferm.; 0.578, 0.818, pH+tmp+Nitrogen.Conc.+OD+Lys_conc.+ X.VXdt+total.Vol+total.cell+emission_O2+rab+Thr+His+lactate +PO4.ferm.; 0.594, 0.818, pH+tmp+Nitrogen.Conc.+OD+Lys_conc. +X.F.Suc+X.VXdt+total.cell+emission_O2+rab+lactate+PO4.fer m.; 0.601, 0.818, pH+tmp+Nitrogen.Conc.+Lys_conc.+X.VXdt+to tal.Vol+total.cell+emission_O2+rab+lactate+PO4.ferm.; 0.569, 0.818, pH+tmp+Nitrogen.Conc.+OD+Lys_conc.+X.VXdt+total.Vol +total.cell+PL+emission_O2+rab+Thr+His+lactate+PO4.ferm.; 0. 593, 0.818, tmp+Nitrogen.Conc.+OD+Lys_conc.+X.VXdt+total.Vo l+total.cell+emission_O2+rab+Thr+lactate+PO4.ferm.; 0.577, 0.818, pH+tmp+Nitrogen.Conc.+Lys_conc.+X.VXdt+total.Vol+tot al.cell+PL+emission_O2+rab+Thr+His+lactate+PO4.ferm.; 0.608, 0.818, tmp+Nitrogen.Conc.+OD+Lys_conc.+X.F.Suc+X.VXdt+tota l.cell+Thr+lactate+PO4.ferm.; 0.593, 0.818, pH+tmp+Nitrogen. Conc.+Lys_conc.+X.F.Suc+X.VXdt+total.Vol+emission_O2+rab+Hi s+lactate+PO4.ferm.; 0.608, 0.817, tmp+Nitrogen.Conc.+Lys_c onc.+X.VXdt+total.Vol+emission_O2+rab+Thr+lactate+PO4.fer m.; 0.593, 0.817, pH+tmp+P-Source.Feed.conc.+Nitrogen.Conc. +Lys_conc.+X.VXdt+total.Vol+emission_O2+rab+Thr+lactate+PO4. ferm.; 0.585, 0.817, tmp+Nitrogen.Conc.+Lys_conc.+X.VXdt+to tal.Vol+yield+emission_O2+rab+Thr+lactate+PO4.ferm.+PO4.app orte+PO4.utilise; 0.593, 0.817, pH+tmp+Main.Thr.Cone.+Nitro gen.Conc.+Lys_conc.+X.VXdt+total.Vol+emission_O2+rab+Thr+la ctate+PO4.ferm.; 0.615, 0.817, tmp+OD+Lys_conc.+X.VXdt+tota l.cell+PL+Thr+lactate+PO4.ferm.; 0.585, 0.817, pH+tmp+Nitro gen.Conc.+Lys conc.+X.VXdt+total.Vol+total.cell+emission_O2 +rab+Thr+His+lactate+PO4.ferm.; 0.607, 0.817, pH+tmp+OD+Lys conc.+X.VXdt+total.cell+emission_O2+rab+Thr+PO4.ferm.; 0.5 76, 0.817, pH+tmp+Nitrogen.Conc.+OD+Lys_conc.+X.F.Suc+X.VXd t+total.Vol+total.cell+emission_O2+rab+Thr+lactate+PO4.fer m.; 0.584, 0.817, pH+tmp+P-Source.Feed.conc.+Nitrogen.Conc. +OD+Lys_conc.+X.VXdt+total.cell+emission_O2+rab+Thr+lactate +PO4.ferm.; 0.600, 0.817, tmp+P-Source.Feed.conc.+Nitrogen. Conc.+Lys_conc.+X.VXdt+total.Vol+emission_O2+rab+Thr+lactat e+PO4.ferm.; 0.576, 0.817, pH+tmp+Main.Thr.Conc.+Nitrogen.C onc.+OD+Lys_conc.+X.VXdt+total.cell+PL+emission_O2+rab+Thr+ lactate+PO4.ferm.; 0.607, 0.817, tmp+P-Source.Feed.conc.+Ni trogen.Conc.+Lys_conc.+X.VXdt+total.Vol+emission_O2+rab+lac tate+PO4.ferm.; 0.592, 0.817, tmp+P-Source.Feed.conc.+Nitro gen.Conc.+OD+Lys_conc.+X.VXdt+total.cell+emission_O2+rab+Th r+lactate+PO4.ferm.; 0.606, 0.817, tmp+Nitrogen.Conc.+OD+Ly s_conc.+X.VXdt+total.cell+PL+Thr+lactate+PO4.ferm.; 0.599, 0.817, pH+tmp+Nitrogen.Conc.+OD+Lys_conc.+X.VXdt+total.Vol+ emission_O2+rab+lactate+PO4.ferm.; 0.599, 0.817, tmp+Nitrog en.Conc.+Lys_conc.+X.VXdt+total.Vol+emission_O2+rab+Thr+lac tate+PO4.ferm.+PO4.apporte; 0.591, 0.816, pH+tmp+Nitrogen.C onc.+OD+Lys_conc.+X.VXdt+total.cell+PL+emission_O2+rab+Thr+ PO4.ferm.; 0.591, 0.816, pH+tmp+Nitrogen.Conc.+Lys_conc.+X. VXdt+total.Vol+emission_O2+rab+Thr+His+lactate+PO4.ferm.; 0. 583, 0.816, pH+tmp+Main.Thr.Conc.+Nitrogen.Conc.+Lys_conc.+ X.VXdt+total.Vol+PL+emission_O2+rab+Thr+lactate+PO4.ferm.; 0.591, 0.816, pH+tmp+Nitrogen.Conc.+Lys_conc.+X.VXdt+total. Vol+total.cell+emission_O2+rab+His+lactate+PO4.ferm.; 0.591, 0.816, pH+tmp+Nitrogen.Conc.+Lys_conc.+X.F.Suc+X.VXdt+tota l.Vol+total.cell+emission_O2+rab+lactate+PO4.ferm.; 0.566, 0.816, tmp+Nitrogen.Conc.+Lys_conc.+X.VXdt+total.Vol+yield+ PL+emission_O2+rab+Thr+His+lactate+PO4.ferm.+PO4.apporte+PO 4.utilise; 0.574, 0.816, pH+tmp+Nitrogen.Conc.+OD+Lys_conc. +X.VXdt+total.cell+PL+emission_O2+rab+Thr+lactate+SO4.ferm. +PO4.ferm.; 0.598, 0.816, pH+tmp+Nitrogen.Conc.+OD+Lys_conc. +X.VXdt+total.cell+emission_O2+rab+Thr+PO4.ferm.; 0.612, 0. 816, tmp+OD+Lys_conc.+X.VXdt+total.cell+emission_O2+rab+Thr +PO4.ferm.; 0.619, 0.816, tmp+OD+Lys_conc.+X.VXdt+total.cel l+Thr+lactate+PO4.ferm.; 0.582, 0.816, pH+tmp+Nitrogen.Conc. +OD+Lys_conc.+X.F.Suc+X.VXdt+total.Vol+total.cell+emission_ O2+rab+lactate+PO4.ferm.; 0.590, 0.816, pH+tmp+P-Source.Fee d.conc.+Nitrogen.Cone.+Lys_conc.+X.VXdt+total.Vol+total.cel l+emission_O2+rab+lactate+PO4.ferm.; 0.582, 0.816, pH+tmp+M ain.Thr.Conc.+OD+Lys_conc.+X.VXdt+total.cell+PL+emission_O2 +rab+Thr+lactate+PO4.ferm.; 0.590, 0.816, pH+tmp+Nitrogen.C onc.+OD+Lys_conc.+X.VXdt+total.Vol+total.cell+emission_O2+r ab+lactate+PO4.ferm.; 0.574, 0.816, pH+tmp+Nitrogen.Conc.+L ys_conc.+X.VXdt+total.Vol+total.cell+PL+emission_O2+rab+Thr +lactate+SO4.ferm.+PO4.ferm.; 0.590, 0.816, tmp+Nitrogen.Co nc.+Lys_conc.+X.VXdt+total.Vol+yield+emission_O2+rab+Thr+la ctate+PO4.ferm.+PO4.apporte; 0.582, 0.816, pH+tmp+Nitrogen. Conc.+Lys_conc.+X.VXdt+total.Vol+PL+emission_O2+rab+Thr+His +lactate+PO4.ferm.; 0.604, 0.816, tmp+Main.Thr.Conc.+Nitrog en.Conc.+OD+Lys conc.+X.VXdt+total.cell+Thr+lactate+PO4.fer m.; 0.582, 0.816, Nitrogen.Conc.+Lys_conc.+X.VXdt+total.Vol +yield+emission_O2+rab+Thr+His+lactate+PO4.ferm.+PO4.apport e+PO4.utilise; 0.581, 0.815, pH+tmp+Nitrogen.Conc.+OD+Lys_c onc.+X.F.Suc+X.VXdt+total.cell+emission_O2+rab+His+lactate+ PO4.ferm.; 0.573, 0.815, pH+tmp+P-Source.Feed.conc.+Nitroge n.Conc.+OD+Lys_conc.+X.VXdt+total.Vol+total.cell+emission_O 2+rab+Thr+lactate+PO4.ferm.; 0.597, 0.815, pH+tmp+Main.Thr. Conc.+Nitrogen.Conc.+Lys_conc.+X.VXdt+total.Vol+emission_O2 +rab+lactate+PO4.ferm.; 0.581, 0.815, pH+tmp+Nitrogen.Conc. +OD+Lys_conc.+X.VXdt+total.cell+emission_O2+rab+Thr+lactate +SO4.ferm.+PO4.ferm.; 0.581, 0.815, pH+tmp+Main.P-Source.Co nc.+Nitrogen.Conc.+OD+Lys_conc.+X.VXdt+total.cell+emission_ O2+rab+Thr+lactate+PO4.ferm.; 0.589, 0.815, pH+tmp+Main.P-S ource.Conc.+P-Source.Feed.conc.+Nitrogen.Conc.+Lys_conc.+X. VXdt+total.Vol+emission_O2+rab+lactate+PO4.ferm.; 0.581, 0. 815, Nitrogen.Cone.+Lys_conc.+X.F.Suc+X.VXdt+total.Vol+yiel d+emission_O2+rab+Thr+lactate+PO4.ferm.+PO4.apporte+PO4.uti lise; 0.573, 0.815, Nitrogen.Conc.+Lys_conc.+X.VXdt+total.V ol+yield+PL+emission_O2+rab+Thr+His+lactate+PO4.ferm.+PO4.a pporte+PO4.utilise; 0.581, 0.815, tmp+Nitrogen.Conc.+OD+Lys conc.+X.F.Suc+X.VXdt+total.cell+emission_O2+rab+Thr+His+la ctate+PO4.ferm.; 0.573, 0.815, pH+tmp+Main.Thr.Conc.+Nitrog en.Conc.+Lys_conc.+X.VXdt+total.Vol+total.cell+PL+emission_ O2+rab+Thr+lactate+PO4.ferm.; 0.589, 0.815, pH+tmp+Nitrogen. Conc.+Lys_conc.+X.F.Suc+X.VXdt+total.Vol+emission_O2+rab+Th r+lactate+PO4.ferm.; 0.604, 0.815, Nitrogen.Conc.+Lys_conc. +X.VXdt+total.Vol+yield+emission_O2+rab+Thr+lactate+PO4.uti lise; 0.596, 0.815, pH+tmp+Nitrogen.Conc.+Lys_conc.+X.VXdt+ total.Vol+PL+emission_O2+rab+lactate+PO4.ferm.; 0.589, 0.81 5, pH+tmp+P-Source.Feed.conc.+Nitrogen.Conc.+OD+Lys_conc.+X. VXdt+total.Vol+emission_O2+rab+lactate+PO4.ferm.; 0.564, 0. 815, pH+tmp+Main.Thr.Conc.+Nitrogen.Conc.+OD+Lys_conc.+X.VX dt+total.Vol+total.cell+PL+emission_O2+rab+Thr+lactate+PO4. ferm.; 0.580, 0.815, pH+tmp+P-Source.Feed.conc.+Nitrogen.Co nc.+Lys_conc.+X.VXdt+total.Vol+PL+emission_O2+rab+Thr+lacta te+PO4.ferm.; 0.564, 0.815, tmp+Main.Thr.Conc.+Nitrogen.Con c.+Lys_conc.+X.VXdt+total.Vol+yield+PL+emission_O2+rab+Thr+ lactate+PO4.ferm.+PO4.apporte+PO4.utilise; 0.572, 0.815, pH +tmp+Main.Thr.Conc.+Nitrogen.Conc.+OD+Lys_conc.+X.VXdt+tota l.Vol+total.cell+emission_O2+rab+Thr+lactate+PO4.ferm.; 0.5 96, 0.815, tmp+Nitrogen.Conc.+OD+Lys_conc.+X.VXdt+total.Vol +emission_O2+rab+Thr+lactate+PO4.ferm.; 0.588, 0.815, pH+tm p+Nitrogen.Conc.+OD+Lys_conc.+X.VXdt+total.Vol+emission_O2+ rab+His+lactate+PO4.ferm.; 0.580, 0.815, pH+tmp+Main.Thr.Co nc.+Nitrogen.Conc.+Lys_conc.+X.VXdt+total.Vol+total.cell+em ission_O2+rab+Thr+lactate+PO4.ferm.; 0.580, 0.815, pH+tmp+N itrogen.Conc.+Lys_conc.+X.F.Suc+X.VXdt+total.Vol+total.cell +emission_O2+rab+Thr+lactate+PO4.ferm.; 0.580, 0.815, pH+tm p+OD+Lys_conc.+X.VXdt+total.Vol+total.cell+PL+emission_O2+r ab+Thr+lactate+PO4.ferm.; 0.603, 0.815, tmp+Nitrogen.Conc.+ OD+Lys_conc.+X.VXdt+total.cell+Thr+His+lactate+PO4.ferm.; 0. 588, 0.815, pH+tmp+P-Source.Feed.conc.+Main.Thr.Conc.+Nitro gen.Conc.+Lys_conc.+X.VXdt+total.Vol+emission_O2+rab+lactat e+PO4.ferm.; 0.588, 0.815, pH+tmp+Main.Thr.Conc.+OD+Lys_con c.+X.VXdt+total.cell+emission_O2+rab+Thr+lactate+PO4.ferm.; 0.563, 0.815, pH+tmp+Nitrogen.Conc.+OD+Lys_conc.+X.VXdt+to tal.Vol+total.cell+PL+emission_O2+rab+Thr+lactate+SO4.ferm. +PO4.ferm.; 0.588, 0.815, Nitrogen.Conc.+Lys_conc.+X.VXdt+t otal.Vol+yield+emission_O2+rab+Thr+His+lactate+PO4.ferm.+PO 4.apporte; 0.595, 0.815, tmp+OD+Lys_conc.+X.VXdt+total.cell +emission_O2+rab+Thr+His+lactate+PO4.ferm.; 0.579, 0.815, M ain.Thr.Conc.+Nitrogen.Conc.+Lys_conc.+X.VXdt+total.Vol+yie ld+emission_O2+rab+Thr+lactate+PO4.ferm.+PO4.apporte+PO4.ut ilise; 0.579, 0.815, Nitrogen.Conc.+Lys_conc.+X.VXdt+total. Vol+yield+emission_O2+rab+Thr+lactate+SO4.ferm.+PO4.ferm.+P O4.apporte+PO4.utilise; 0.571, 0.815, tmp+Main.Thr.Conc.+Ni trogen.Conc.+Lys_conc.+X.VXdt+total.Vol+yield+emission_O2+r ab+Thr+lactate+PO4.ferm.+PO4.apporte+PO4.utilise; 0.587, 0. 815, pH+tmp+Nitrogen.Conc.+OD+Lys_conc.+X.F.Suc+X.VXdt+tota l.Vol+emission_O2+rab+lactate+PO4.ferm.; 0.587, 0.815, tmp+ Nitrogen.Conc.+Lys_conc.+X.VXdt+total.Vol+emission_O2+rab+T hr+lactate+PO4.ferm.+PO4.apporte+PO4.utilise; 0.587, 0.814, pH+tmp+Lys_conc.+X.VXdt+total.Vol+total.cell+PL+emission_O 2+rab+Thr+lactate+PO4.ferm.; 0.579, 0.814, pH+tmp+Nitrogen. Conc.+Lys_conc.+X.VXdt+total.Vol+PL+emission_O2+rab+Thr+lac tate+SO4.ferm.+PO4.ferm.; 0.579, 0.814, tmp+P-Source.Feed.c onc.+Nitrogen.Conc.+OD+Lys_conc.+X.VXdt+total.Vol+total.cel l+emission_O2+rab+Thr+lactate+PO4.ferm.; 0.616, 0.814, tmp+ OD+Lys_conc.+X.VXdt+total.cell+PL+Thr+PO4.ferm.; 0.587, 0.8 14, P-Source.Feed.conc.+Nitrogen.Conc.+Lys_conc.+X.VXdt+tot al.Vol+yield+emission_O2+rab+Thr+lactate+PO4.ferm.+PO4.appo rte; 0.579, 0.814, pH+tmp+P-Source.Feed.conc.+Nitrogen.Conc. +Lys_conc.+X.VXdt+total.Vol+total.cell+emission_O2+rab+Thr+ lactate+PO4.ferm.; 0.571, 0.814, pH+tmp+Nitrogen.Conc.+OD+L ys_conc.+X.VXdt+total.Vol+PL+emission_O2+rab+Thr+His+lactat e+PO4.ferm.; 0.595, 0.814, pH+tmp+Main.P-Source.Conc.+Nitro gen.Conc.+Lys_conc.+X.VXdt+total.Vol+emission_O2+rab+lactat e+PO4.ferm.; 0.587, 0.814, tmp+Main.Thr.Conc.+Nitrogen.Conc. +OD+Lys_conc.+X.VXdt+total.cell+emission_O2+rab+Thr+lactate +PO4.ferm.; 0.587, 0.814, tmp+Nitrogen.Conc.+Lys_conc.+X.VX dt+total.Vol+total.cell+PL+emission_O2+rab+Thr+lactate+PO4. ferm.; 0.594, 0.814, tmp+P-Source.Feed.conc.+Nitrogen.Conc. +Lys_conc.+X.VXdt+total.Vol+emission_O2+rab+His+lactate+PO4. ferm.; 0.609, 0.814, tmp+Main.Thr.Conc.+OD+Lys_conc.+X.VXdt +total.cell+Thr+lactate+PO4.ferm.; 0.579, 0.814, pH+tmp+Nit rogen.Conc.+Lys_conc.+X.VXdt+total.Vol+PL+emission_O2+rab+T hr+lactate+PO4.ferm.+PO4.apporte; 0.587, 0.814, Nitrogen.Co nc.+Lys_conc.+X.VXdt+total.Vol+yield+PL+emission_O2+rab+Thr +lactate+PO4.ferm.+PO4.apporte; 0.587, 0.814, tmp+Main.P-So urce.Conc.+Nitrogen.Conc.+OD+Lys_conc.+X.VXdt+total.cell+em ission_O2+rab+Thr+lactate+PO4.ferm.; 0.586, 0.814, pH+tmp+N itrogen.Conc.+Lys_conc.+X.VXdt+total.Vol+emission_O2+rab+Th r+lactate+PO4.ferm.+PO4.apporte; 0.570, 0.814, pH+tmp+Main. Thr.Conc.+Nitrogen.Conc.+OD+Lys_conc.+X.F.Suc+X.VXdt+total. cell+emission_O2+rab+Thr+lactate+PO4.ferm.; 0.594, 0.814, t mp+Nitrogen.Conc.+OD+Lys_conc.+X.F.Suc+X.VXdt+total.cell+Th r+His+lactate+PO4.ferm.; 0.579, 0.814, pH+tmp+OD+Lys_conc.+ X.VXdt+total.cell+PL+emission_O2+rab+Thr+His+lactate+PO4.fe rm.; 0.586, 0.814, Nitrogen.Conc.+Lys_conc.+X.F.Suc+X.VXdt+ total.Vol+yield+emission_O2+rab+Thr+lactate+PO4.ferm.+PO4.a pporte; 0.586, 0.814, tmp+P-Source.Feed.conc.+Nitrogen.Conc. +Lys_conc.+X.VXdt+total.Vol+total.cell+emission_O2+rab+Thr+ lactate+PO4.ferm.; 0.594, 0.814, tmp+Nitrogen.Conc.+Lys_con c.+X.VXdt+total.Vol+emission_O2+rab+Thr+lactate+PO4.ferm.+P O4.utilise; 0.594, 0.814, tmp+Main.Thr.Conc.+OD+Lys_conc.+X. VXdt+total.cell+emission_O2+rab+Thr+lactate+PO4.ferm.; 0.57 8, 0.814, pH+tmp+Nitrogen.Conc.+OD+Lys_conc.+X.VXdt+total.V ol+emission_O2+rab+Thr+His+lactate+PO4.ferm.; 0.578, 0.814, pH+tmp+P-Source.Feed.conc.+Nitrogen.Cone.+Lys_conc.+X.VXdt +total.Vol+total.cell+emission_O2+rab+His+lactate+PO4.fer m.; 0.578, 0.814, pH+tmp+Nitrogen.Conc.+OD+Lys_conc.+X.VXdt +total.Vol+total.cell+emission_O2+rab+His+lactate+PO4.fer m.; 0.570, 0.814, pH+tmp+Nitrogen.Conc.+OD+Lys_conc.+X.F.Su c+X.VXdt+total.cell+emission_O2+rab+Thr+lactate+SO4.ferm.+P O4.ferm.; 0.594, 0.814, tmp+OD+Lys_conc.+X.F.Suc+X.VXdt+tot al.cell+emission_O2+rab+Thr+lactate+PO4.ferm.; 0.601, 0.814, tmp+Main.Thr.Conc.+OD+Lys_conc.+X.VXdt+total.cell+PL+Thr+l actate+PO4.ferm.; 0.578, 0.814, pH+tmp+Nitrogen.Conc.+Lys_c onc.+X.VXdt+total.Vol+total.cell+emission_O2+rab+Thr+lactat e+SO4.ferm.+PO4.ferm.; 0.586, 0.814, tmp+Nitrogen.Conc.+Lys conc.+X.VXdt+total.Vol+total.cell+emission_O2+rab+Thr+His+ lactate+PO4.ferm.; 0.578, 0.814, pH+tmp+Nitrogen.Conc.+Lys_ conc.+X.F.Suc+X.VXdt+total.Vol+total.cell+emission_O2+rab+H is+lactate+PO4.ferm.; 0.586, 0.814, pH+tmp+OD+Lys_conc.+X.V Xdt+total.cell+PL+emission_O2+rab+Thr+His+PO4.ferm.; 0.569, 0.814, pH+tmp+Main.P-Source.Conc.+Nitrogen.Conc.+OD+Lys_co nc.+X.VXdt+total.cell+PL+emission_O2+rab+Thr+lactate+PO4.fe rm.; 0.561, 0.814, pH+tmp+Nitrogen.Conc.+Lys_conc.+X.VXdt+t otal.Vol+yield+PL+emission_O2+rab+Thr+lactate+PO4.ferm.+PO4. apporte+PO4.utilise; 0.577, 0.814, tmp+Nitrogen.Conc.+OD+Ly s_conc.+X.VXdt+total.Vol+total.cell+PL+emission_O2+rab+Thr+ lactate+PO4.ferm.; 0.569, 0.814, tmp+Nitrogen.Conc.+Lys_con c.+X.VXdt+total.Vol+yield+emission_O2+rab+Thr+His+lactate+P O4.ferm.+PO4.apporte+PO4.utilise; 0.585, 0.814, Main.Thr.Co nc.+Nitrogen.Conc.+Lys_conc.+X.VXdt+total.Vol+yield+emissio n_O2+rab+Thr+lactate+PO4.ferm.+PO4.apporte; 0.569, 0.813, p H+tmp+Nitrogen.Conc.+OD+Lys_conc.+X.F.Suc+X.VXdt+total.Vol+ total.cell+emission_O2+rab+His+lactate+PO4.ferm.; 0.585, 0. 813, pH+tmp+P-Source.Feed.conc.+Nitrogen.Cone.+Lys_conc.+X. F.Suc+X.VXdt+total.Vol+emission_O2+rab+lactate+PO4.ferm.; 0. 569, 0.813, pH+tmp+Nitrogen.Conc.+OD+Lys_conc.+X.VXdt+total. Vol+total.cell+emission_O2+rab+Thr+lactate+SO4.ferm.+PO4.fe rm.; 0.577, 0.813, pH+tmp+OD+Lys_conc.+X.VXdt+total.cell+PL +emission_O2+rab+Thr+lactate+SO4.ferm.+PO4.ferm.; 0.577, 0. 813, pH+tmp+P-Source.Feed.conc.+Nitrogen.Conc.+OD+Lys_conc. +X.VXdt+total.Vol+total.cell+emission_O2+rab+lactate+PO4.fe rm.; 0.600, 0.813, tmp+OD+Lys_conc.+X.VXdt+total.cell+PL+Th r+His+lactate+PO4.ferm.; 0.585, 0.813, tmp+Main.P-Source.Co nc.+P-Source.Feed.conc.+Nitrogen.Conc.+Lys_conc.+X.VXdt+tot al.Vol+emission_O2+rab+Thr+lactate+PO4.ferm.; 0.621, 0.813, tmp+OD+Lys_conc.+X.VXdt+total.cell+Thr+PO4.ferm.; 0.585, 0. 813, pH+tmp+OD+Lys_conc.+X.F.Suc+X.VXdt+total.cell+emission _O2+rab+Thr+lactate+PO4.ferm.; 0.577, 0.813, pH+tmp+Main.P-Source.Conc.+P-Source.Feed.conc.+Nitrogen.Conc.+Lys_conc.+X. VXdt+total.Vol+emission_O2+rab+His+lactate+PO4.ferm.; 0.577, 0.813, pH+tmp+P-Source.Feed.conc.+Nitrogen.Conc.+OD+Lys_co nc.+X.VXdt+total.Vol+emission_O2+rab+His+lactate+PO4.ferm.; 0.560, 0.813, tmp+Nitrogen.Conc.+Lys_conc.+X.VXdt+total.Vo l+yield+PL+emission_O2+rab+Thr+lactate+SO4.ferm.+PO4.ferm.+ PO4.apporte+PO4.utilise; 0.577, 0.813, pH+tmp+P-Source.Feed. conc.+Main.Thr.Conc.+Nitrogen.Conc.+Lys_conc.+X.VXdt+total. Vol+emission_O2+rab+Thr+lactate+PO4.ferm.; 0.568, 0.813, pH +tmp+P-Source.Feed.conc.+Nitrogen.Conc.+OD+Lys_conc.+X.VXdt +total.cell+PL+emission_O2+rab+Thr+lactate+PO4.ferm.; 0.577, 0.813, tmp+Nitrogen.Conc.+OD+Lys_conc.+X.VXdt+total.Vol+to tal.cell+emission_O2+rab+Thr+His+lactate+PO4.ferm.; 0.576, 0.813, tmp+Nitrogen.Conc.+OD+Lys_conc.+X.VXdt+total.cell+PL +emission_O2+rab+Thr+His+lactate+PO4.ferm.; 0.568, 0.813, p H+tmp+Nitrogen.Conc.+Lys_conc.+X.VXdt+total.Vol+PL+emission _O2+rab+Thr+lactate+PO4.ferm.+PO4.apporte+PO4.utilise; 0.59 9, 0.813, tmp+Nitrogen.Cone.+OD+Lys_conc.+X.VXdt+total.cell +emission_O2+rab+Thr+PO4.ferm.; 0.592, 0.813, tmp+Nitrogen. Conc.+OD+Lys conc.+X.VXdt+total.cell+PL+Thr+His+lactate+PO4. ferm.; 0.592, 0.813, tmp+Main.Thr.Cone.+Nitrogen.Cone.+OD+L ys_conc.+X.F.Suc+X.VXdt+total.cell+Thr+lactate+PO4.ferm.; 0. 568, 0.813, Main.Thr.Cone.+Nitrogen.Cone.+Lys_conc.+X.VXdt+ total.Vol+yield+PL+emission_O2+rab+Thr+lactate+PO4.ferm.+PO 4.apporte+PO4.utilise; 0.568, 0.813, Nitrogen.Conc.+Lys_con c.+X.VXdt+total.Vol+yield+PL+emission_O2+rab+Thr+lactate+SO 4.ferm.+PO4.ferm.+PO4.apporte+PO4.utilise; 0.568, 0.813, pH +tmp+Nitrogen.Conc.+OD+Lys_conc.+X.VXdt+total.Vol+PL+emissi on_O2+rab+Thr+lactate+SO4.ferm.+PO4.ferm.; 0.584, 0.813, pH +tmp+Nitrogen.Cone.+Lys_conc.+X.VXdt+total.Vol+PL+emission_ O2+rab+His+lactate+PO4.ferm.; 0.599, 0.813, tmp+OD+Lys_conc. +X.VXdt+total.cell+PL+emission_O2+rab+Thr+PO4.ferm.; 0.568, 0.813, pH+tmp+Main.P-Source.Conc.+Nitrogen.Conc.+OD+Lys_co nc.+X.F.Suc+X.VXdt+total.cell+emission_O2+rab+Thr+lactate+P O4.ferm.; 0.584, 0.813, pH+tmp+OD+Lys_conc.+X.VXdt+total.ce ll+emission_O2+rab+Thr+His+lactate+PO4.ferm.; 0.592, 0.813, tmp+Main.Thr.Conc.+Nitrogen.Conc.+OD+Lys_conc.+X.VXdt+tota l.cell+PL+Thr+lactate+PO4.ferm.; 0.599, 0.813, pH+tmp+Nitro gen.Conc.+OD+Lys_conc.+X.F.Suc+X.VXdt+total.cell+lactate+PO 4.ferm.; 0.584, 0.813, pH+tmp+Nitrogen.Conc.+Lys_conc.+X.VX dt+total.Vol+emission_O2+rab+Thr+lactate+SO4.ferm.+PO4.fer m.; 0.606, 0.813, tmp+Nitrogen.Conc.+Lys_conc.+X.VXdt+total. Vol+emission_O2+rab+lactate+PO4.ferm.; 0.606, 0.813, tmp+Ni trogen.Conc.+OD+Lys_conc.+X.F.Suc+X.VXdt+total.cell+lactate +PO4.ferm.; 0.576, 0.813, pH+tmp+P-Source.Feed.conc.+Nitrog en.Conc.+Lys_conc.+X.VXdt+total.Vol+emission_O2+rab+Thr+His +lactate+PO4.ferm.; 0.591, 0.813, tmp+Nitrogen.Conc.+Lys_co nc.+X.VXdt+total.Vol+PL+emission_O2+rab+Thr+lactate+PO4.fer m.; 0.567, 0.813, Nitrogen.Conc.+Lys_conc.+X.F.Suc+X.VXdt+t otal.Vol+yield+PL+emission_O2+rab+Thr+lactate+PO4.ferm.+PO4. apporte+PO4.utilise; 0.606, 0.813, tmp+OD+Lys_conc.+X.VXdt+ total.cell+Thr+His+lactate+PO4.ferm.; 0.583, 0.813, pH+tmp+ Main.P-Source.Conc.+Nitrogen.Conc.+Lys_conc.+X.VXdt+total.V ol+emission_O2+rab+His+lactate+PO4.ferm.; 0.567, 0.813, pH+ tmp+P-Source.Feed.conc.+Nitrogen.Conc.+OD+Lys_conc.+X.VXdt+ total.cell+emission_O2+rab+Thr+His+lactate+PO4.ferm.; 0.583, 0.813, pH+tmp+Main.P-Source.Conc.+Nitrogen.Conc.+Lys_conc. +X.VXdt+total.Vol+emission_O2+rab+Thr+lactate+PO4.ferm.; 0. 583, 0.813, pH+tmp+Main.P-Source.Conc.+Nitrogen.Conc.+Lys_c onc.+X.F.Suc+X.VXdt+total.Vol+emission_O2+rab+lactate+PO4.f erm.; 0.591, 0.813, tmp+Nitrogen.Conc.+Lys_conc.+X.VXdt+tot al.Vol+emission_O2+rab+Thr+His+lactate+PO4.ferm.; 0.575, 0. 813, pH+tmp+Nitrogen.Conc.+Lys_conc.+X.VXdt+total.Vol+PL+em ission_O2+rab+Thr+lactate+PO4.ferm.+PO4.utilise; 0.558, 0.8 13, pH+tmp+P-Source.Feed.conc.+Nitrogen.Conc.+OD+Lys_conc.+ X.VXdt+total.Vol+total.cell+PL+emission_O2+rab+Thr+lactate+ PO4.ferm.; 0.567, 0.813, pH+tmp+Main.Thr.Conc.+Nitrogen.Con c.+OD+Lys_conc.+X.VXdt+total.Vol+PL+emission_O2+rab+Thr+lac tate+PO4.ferm.; 0.558, 0.813, pH+tmp+Nitrogen.Conc.+OD+Lys_ conc.+X.F.Suc+X.VXdt+total.Vol+total.cell+emission_O2+rab+T hr+His+lactate+PO4.ferm.; 0.575, 0.813, tmp+P-Source.Feed.c onc.+Nitrogen.Conc.+OD+Lys_conc.+X.VXdt+total.cell+emission _O2+rab+Thr+His+lactate+PO4.ferm.; 0.567, 0.812, pH+tmp+P-S ource.Feed.conc.+Nitrogen.Conc.+OD+Lys_conc.+X.F.Suc+X.VXdt +total.cell+emission_O2+rab+Thr+lactate+PO4.ferm.; 0.575, 0. 812, pH+tmp+Main.Thr.Conc.+Nitrogen.Conc.+OD+Lys_conc.+X.VX dt+total.Vol+emission_O2+rab+Thr+lactate+PO4.ferm.; 0.575, 0.812, tmp+Nitrogen.Conc.+Lys_conc.+X.VXdt+total.Vol+PL+emi ssion_O2+rab+Thr+lactate+PO4.ferm.+PO4.apporte+PO4.utilise; 0.591, 0.812, pH+tmp+Nitrogen.Conc.+OD+Lys_conc.+X.F.Suc+X. VXdt+total.cell+Thr+lactate+PO4.ferm.; 0.583, 0.812, tmp+P-Source.Feed.conc.+Nitrogen.Conc.+Lys_conc.+X.VXdt+tota1.Vol +emission_O2+rab+Thr+His+lactate+PO4.ferm.; 0.575, 0.812, p H+tmp+OD+Lys_conc.+X.F.Suc+X.VXdt+total.cell+PL+emission_O2 +rab+Thr+lactate+PO4.ferm.; 0.583, 0.812, pH+tmp+P-Source.F eed.conc.+Nitrogen.Cone.+Lys_conc.+X.VXdt+total.Vol+PL+emis sion_O2+rab+lactate+PO4.ferm.; 0.583, 0.812, pH+tmp+Nitroge n.Conc.+OD+Lys_conc.+X.F.Suc+X.VXdt+total.cell+emission_O2+ rab+Thr+PO4.ferm.; 0.566, 0.812, pH+tmp+Main.Thr.Conc.+Nitr ogen.Conc.+Lys_conc.+X.VXdt+total.Vol+PL+emission_O2+rab+Th r+lactate+SO4.ferm.+PO4.ferm.; 0.566, 0.812, pH+tmp+Main.P-Source.Conc.+Nitrogen.Conc.+Lys_conc.+X.VXdt+total.Vol+tota l.cell+PL+emission_O2+rab+Thr+lactate+PO4.ferm.; 0.583, 0.8 12, pH+tmp+Nitrogen.Conc.+Lys_conc.+X.VXdt+total.Vol+emissi on_O2+rab+Thr+lactate+PO4.ferm.+PO4.utilise; 0.566, 0.812, tmp+Nitrogen.Conc.+Lys_conc.+X.VXdt+total.Vol+yield+emissio n_O2+rab+Thr+lactate+SO4.ferm.+PO4.ferm.+PO4.apporte+PO4.ut ilise; 0.590, 0.812, Nitrogen.Conc.+Lys_conc.+X.VXdt+total. Vol+yield+emission_O2+rab+Thr+His+lactate+PO4.utilise; 0.57 4, 0.812, pH+tmp+Main.P-Source.Conc.+Nitrogen.Conc.+Lys_con c.+X.VXdt+total.Vol+total.cell+emission_O2+rab+Thr+lactate+ PO4.ferm.; 0.582, 0.812, tmp+OD+Lys_conc.+X.VXdt+total.cell +PL+emission_O2+rab+Thr+His+lactate+PO4.ferm.; 0.566, 0.812, pH+tmp+P-Source.Feed.conc.+Nitrogen.Conc.+Lys_conc.+X.VXdt +total.Vol+total.cell+PL+emission_O2+rab+Thr+lactate+PO4.fe rm.; 0.574, 0.812, pH+tmp+P-Source.Feed.conc.+Main.Thr.Conc. +Nitrogen.Conc.+Lys_conc.+X.VXdt+total.Vol+emission_O2+rab+ His+lactate+PO4.ferm.; 0.590, 0.812, tmp+Main.Thr.Conc.+Nit rogen.Conc.+OD+Lys_conc.+X.VXdt+total.cell+Thr+His+lactate+ PO4.ferm.; 0.582, 0.812, pH+tmp+Main.Thr.Conc.+Nitrogen.Con c.+Lys_conc.+X.VXdt+total.Vol+emission_O2+rab+His+lactate+P O4.ferm.; 0.574, 0.812, pH+tmp+Nitrogen.Conc.+OD+Lys_conc.+ X.F.Suc+X.VXdt+total.Vol+emission_O2+rab+His+lactate+PO4.fe rm.; 0.566, 0.812, pH+tmp+Main.Thr.Conc.+Nitrogen.Conc.+OD+ Lys_conc.+X.VXdt+total.cell+emission_O2+rab+Thr+His+lactate +PO4.ferm.; 0.557, 0.812, pH+tmp+Nitrogen.Conc.+OD+Lys_conc. +X.F.Suc+X.VXdt+total.cell+PL+emission_O2+rab+Thr+His+lacta te+PO4.ferm.; 0.597, 0.812, tmp+Nitrogen.Conc.+OD+Lys_conc. +X.VXdt+total.cell+Thr+lactate+SO4.ferm.+PO4.ferm.; 0.574, 0.812, tmp+Nitrogen.Conc.+Lys_conc.+X.VXdt+total.Vol+yield+ PL+emission_O2+rab+Thr+lactate+PO4.ferm.+PO4.apporte; 0.582, 0.812, tmp+Nitrogen.Conc.+OD+Lys_conc.+X.VXdt+total.cell+e mission_O2+rab+Thr+lactate+SO4.ferm.+PO4.ferm.; 0.590, 0.81 2, pH+tmp+Nitrogen.Conc.+OD+Lys_conc.+X.VXdt+total.cell+emi ssion_O2+rab+lactate+PO4.ferm.; 0.582, 0.812, tmp+Nitrogen. Conc.+Lys_conc.+X.VXdt+total.Vol+emission_O2+rab+Thr+His+la ctate+PO4.ferm.+PO4.apporte; 0.589, 0.812, tmp+Main.P-Sourc e.Conc.+P-Source.Feed.conc.+Nitrogen.Conc.+Lys_conc.+X.VXdt +total.Vol+emission_O2+rab+lactate+PO4.ferm.; 0.574, 0.812, pH+tmp+P-Source.Feed.conc.+Nitrogen.Conc.+OD+Lys_conc.+X.V Xdt+total.Vol+emission_O2+rab+Thr+lactate+PO4.ferm.; 0.582, 0.812, pH+tmp+Main.Thr.Conc.+OD+Lys_conc.+X.VXdt+total.cel l+PL+emission_O2+rab+Thr+PO4.ferm.; 0.573, 0.812, pH+tmp+Ni trogen.Conc.+OD+Lys_conc.+X.F.Suc+X.VXdt+total.Vol+emission _O2+rab+Thr+lactate+PO4.ferm.; 0.573, 0.812, tmp+Nitrogen.C onc.+OD+Lys_conc.+X.F.Suc+X.VXdt+total.Vol+total.cell+emiss ion_O2+rab+Thr+lactate+PO4.ferm.; 0.581, 0.812, pH+tmp+Nitr ogen.Conc.+Lys_conc.+X.VXdt+total.Vol+total.cell+PL+emissio n_O2+rab+lactate+PO4.ferm.; 0.573, 0.812, tmp+Main.Thr.Conc. +Nitrogen.Conc.+Lys_conc.+X.VXdt+total.Vol+yield+emission_O 2+rab+Thr+lactate+PO4.ferm.+PO4.apporte; 0.589, 0.812, P-So urce.Feed.conc.+Nitrogen.Conc.+OD+Lys_conc.+X.VXdt+total.ce ll+emission_O2+rab+Thr+lactate+PO4.ferm.; 0.581, 0.812, tmp +P-Source.Feed.conc.+Main.Thr.Conc.+Nitrogen.Conc.+Lys_cone. +X.VXdt+total.Vol+emission_O2+rab+Thr+lactate+PO4.ferm.; 0. 589, 0.812, pH+tmp+OD+Lys_conc.+X.F.Suc+X.VXdt+total.cell+e mission_O2+rab+Thr+PO4.ferm.; 0.565, 0.811, pH+tmp+P-Source. Feed.conc.+Nitrogen.Conc.+OD+Lys_conc.+X.VXdt+total.Vol+tot al.cell+emission_O2+rab+His+lactate+PO4.ferm.; 0.596, 0.811, pH+tmp+Nitrogen.Conc.+OD+Lys_conc.+X.VXdt+total.cell+Thr+1 actate+PO4.ferm.; 0.573, 0.811, pH+tmp+Main.P-Source.Conc.+ P-Source.Feed.conc.+Nitrogen.Cone.+Lys_conc.+X.VXdt+total.V ol+emission_O2+rab+Thr+lactate+PO4.ferm.; 0.589, 0.811, pH+ tmp+OD+Lys_conc.+X.VXdt+total.cell+emission_O2+rab+Thr+His+ PO4.ferm.; 0.581, 0.811, tmp+P-Source.Feed.conc.+Nitrogen.C onc.+OD+Lys_conc.+X.VXdt+total.Vol+emission_O2+rab+Thr+lact ate+PO4.ferm.; 0.573, 0.811, tmp+Nitrogen.Conc.+Lys_conc.+X. VXdt+total.Vol+yield+emission_O2+rab+Thr+His+lactate+PO4.fe rm.+PO4.apporte; 0.589, 0.811, tmp+Main.P-Source.Conc.+OD+L ys_conc.+X.VXdt+total.cell+emission_O2+rab+Thr+lactate+PO4. ferm.; 0.589, 0.811, Nitrogen.Conc.+Lys_conc.+X.VXdt+total. Vol+yield+emission_O2+rab+Thr+lactate+PO4.ferm.+PO4.utilis e; 0.610, 0.811, tmp+Nitrogen.Conc.+OD+Lys_conc.+X.VXdt+tot al.cell+Thr+PO4.ferm.; 0.556, 0.811, pH+tmp+Nitrogen.Conc.+ OD+Lys_conc.+X.F.Suc+X.VXdt+total.Vol+total.cell+PL+emissio n_O2+rab+Thr+lactate+PO4.ferm.; 0.556, 0.811, pH+tmp+Main.T hr.Conc.+Nitrogen.Conc.+OD+Lys_conc.+X.VXdt+total.cell+PL+e mission_O2+rab+Thr+His+lactate+PO4.ferm.; 0.596, 0.811, tmp +Main.Thr.Conc.+OD+Lys_conc.+X.VXdt+total.cell+Thr+His+lact ate+PO4.ferm.; 0.588, 0.811, tmp+Nitrogen.Conc.+OD+Lys_conc. +X.F.Suc+X.VXdt+total.cell+PL+Thr+lactate+PO4.ferm.; 0.581, 0.811, tmp+Main.Thr.Conc.+OD+Lys_conc.+X.VXdt+total.cell+P L+emission_O2+rab+Thr+lactate+PO4.ferm.; 0.581, 0.811, tmp+ Nitrogen.Conc.+OD+Lys_conc.+X.VXdt+total.Vol+PL+emission_O2 +rab+Thr+lactate+PO4.ferm.; 0.588, 0.811, tmp+Nitrogen.Conc. +OD+Lys_conc.+X.F.Suc+X.VXdt+total.cell+Thr+lactate+SO4.fer m.+PO4.ferm.; 0.555, 0.811, pH+tmp+P-Source.Feed.conc.+Nitr ogen.Conc.+OD+Lys_conc.+X.VXdt+total.Vol+total.cell+emissio n_O2+rab+Thr+His+lactate+PO4.ferm.; 0.596, 0.811, tmp+OD+Ly s_conc.+X.VXdt+total.cell+PL+Thr+lactate+SO4.ferm.+PO4.fer m.; 0.572, 0.811, tmp+Nitrogen.Conc.+OD+Lys_conc.+X.F.Suc+X. VXdt+total.cell+PL+emission_O2+rab+Thr+lactate+PO4.ferm.; 0. 603, 0.811, tmp+Nitrogen.Conc.+OD+Lys_conc.+X.VXdt+total.ce ll+PL+Thr+PO4.ferm.; 0.555, 0.811, pH+tmp+Nitrogen.Conc.+Ly s_conc.+X.VXdt+total.Vol+total.cell+PL+emission_O2+rab+Thr+ His+lactate+SO4.ferm.+PO4.ferm.; 0.555, 0.811, pH+tmp+Nitro gen.Conc.+OD+Lys_conc.+X.VXdt+total.cell+PL+emission_O2+rab +Thr+His+lactate+SO4.ferm.+PO4.ferm.; 0.588, 0.811, tmp+Mai n.Thr.Conc.+Nitrogen.Conc.+Lys_conc.+X.VXdt+total.Vol+emiss ion_O2+rab+Thr+lactate+PO4.ferm.; 0.572, 0.811, pH+tmp+Nitr ogen.Conc.+OD+Lys_conc.+X.VXdt+total.cell+PL+emission_O2+ra b+Thr+His+PO4.ferm.; 0.564, 0.811, Nitrogen.Cone.+Lys_conc. +X.F.Suc+X.VXdt+total.Vol+yield+emission_O2+rab+Thr+His+lac tate+PO4.ferm.+PO4.apporte+PO4.utilise; 0.572, 0.811, pH+tm p+Main.P-Source.Conc.+Nitrogen.Conc.+Lys_conc.+X.VXdt+total. Vol+PL+emission_O2+rab+Thr+lactate+PO4.ferm.; 0.588, 0.811, tmp+Lys_conc.+X.VXdt+total.Vol+total.cell+PL+emission_O2+r ab+Thr+lactate+PO4.ferm.; 0.572, 0.811, pH+tmp+Nitrogen.Con c.+OD+Lys_conc.+X.VXdt+total.Vol+emission_O2+rab+Thr+lactat e+SO4.ferm.+PO4.ferm.; 0.572, 0.811, pH+tmp+Main.P-Source.C onc.+OD+Lys_conc.+X.VXdt+total.cell+PL+emission_O2+rab+Thr+ lactate+PO4.ferm.; 0.572, 0.811, pH+tmp+Main.Thr.Conc.+Nitr ogen.Conc.+Lys_conc.+X.VXdt+total.Vol+emission_O2+rab+Thr+l actate+SO4.ferm.+PO4.ferm.; 0.580, 0.811, tmp+Nitrogen.Conc. +Lys_conc.+X.VXdt+total.Vol+PL+emission_O2+rab+Thr+lactate+ PO4.ferm.+PO4.apporte; 0.602, 0.811, tmp+OD+Lys_conc.+X.F.S uc+X.VXdt+total.cell+Thr+lactate+PO4.ferm.; 0.546, 0.811, t mp+Main.Thr.Conc.+Nitrogen.Conc.+Lys_conc.+X.VXdt+tota1.Vol +yield+PL+emission_O2+rab+Thr+His+lactate+PO4.ferm.+PO4.app orte+PO4.utilise; 0.602, 0.811, tmp+OD+Lys_conc.+X.VXdt+tot al.cell+PL+Thr+His+PO4.ferm.; 0.580, 0.811, pH+tmp+Nitrogen. Conc.+OD+Lys_conc.+X.VXdt+total.cell+emission_O2+rab+Thr+Hi s+PO4.ferm.; 0.572, 0.811, P-Source.Feed.conc.+Main.Thr.Con c.+Nitrogen.Conc.+Lys_conc.+X.VXdt+total.Vol+yield+emission _O2+rab+Thr+lactate+PO4.ferm.+PO4.apporte; 0.563, 0.811, pH +tmp+Nitrogen.Conc.+OD+Lys_conc.+X.VXdt+total.cell+emission _O2+rab+Thr+His+lactate+SO4.ferm.+PO4.ferm.; 0.587, 0.811, tmp+OD+Lys_conc.+X.VXdt+total.Vol+total.cell+emission_O2+ra b+Thr+lactate+PO4.ferm.; 0.572, 0.811, tmp+P-Source.Feed.co nc.+Nitrogen.Conc.+OD+Lys_conc.+X.F.Suc+X.VXdt+total.cell+e mission_O2+rab+Thr+lactate+PO4.ferm.; 0.587, 0.811, tmp+Nit rogen.Conc.+OD+Lys_conc.+X.VXdt+total.cell+PL+Thr+lactate+S O4.ferm.+PO4.ferm.; 0.580, 0.811, pH+tmp+OD+Lys_conc.+X.F.S uc+X.VXdt+total.cell+PL+emission_O2+rab+Thr+PO4.ferm.; 0.58 7, 0.811, tmp+P-Source.Feed.conc.+Nitrogen.Cone.+Lys_conc.+ X.VXdt+total.Vol+total.cell+emission_O2+rab+lactate+PO4.fer m.; 0.571, 0.811, pH+tmp+P-Source.Feed.conc.+Nitrogen.Conc. +Lys_conc.+X.F.Suc+X.VXdt+total.Vol+emission_O2+rab+His+lac tate+PO4.ferm.; 0.563, 0.811, pH+tmp+P-Source.Feed.conc.+Ma in.Thr.Conc.+Nitrogen.Conc.+Lys_conc.+X.VXdt+total.Vol+PL+e mission_O2+rab+Thr+lactate+PO4.ferm.; 0.595, 0.811, tmp+OD+ Lys_conc.+X.VXdt+total.cell+emission_O2+rab+Thr+His+PO4.fer m.; 0.563, 0.811, Nitrogen.Conc.+Lys_conc.+X.VXdt+total.Vol +yield+emission_O2+rab+Thr+His+lactate+SO4.ferm.+PO4.ferm.+ PO4.apporte+PO4.utilise; 0.579, 0.811, tmp+P-Source.Feed.co nc.+Nitrogen.Conc.+Lys_conc.+X.VXdt+total.Vol+PL+emission_O 2+rab+Thr+lactate+PO4.ferm.; 0.571, 0.811, pH+tmp+Main.Thr. Conc.+Nitrogen.Conc.+Lys_conc.+X.VXdt+total.Vol+emission_O2 +rab+Thr+His+lactate+PO4.ferm.; 0.554, 0.811, pH+tmp+Main.P -Source.Conc.+Nitrogen.Conc.+OD+Lys_conc.+X.VXdt+total.Vol+ total.cell+PL+emission_O2+rab+Thr+lactate+PO4.ferm.; 0.595, 0.811, tmp+Lys_conc.+X.VXdt+total.Vol+total.cell+emission_ O2+rab+Thr+lactate+PO4.ferm.; 0.563, 0.810, pH+tmp+Main.P-S ource.Conc.+Nitrogen.Conc.+OD+Lys_conc.+X.VXdt+total.cell+e mission_O2+rab+Thr+His+lactate+PO4.ferm.; 0.571, 0.810, tmp +Main.P-Source.Conc.+Nitrogen.Conc.+OD+Lys_conc.+X.F.Suc+X. VXdt+total.cell+emission_O2+rab+Thr+lactate+PO4.ferm.; 0.57 9, 0.810, tmp+Nitrogen.Conc.+OD+Lys_conc.+X.VXdt+total.Vol+ emission_O2+rab+Thr+His+lactate+PO4.ferm.; 0.579, 0.810, pH +tmp+Nitrogen.Conc.+OD+Lys_conc.+X.VXdt+total.Vol+PL+emissi on_O2+rab+lactate+PO4.ferm.; 0.594, 0.810, tmp+Nitrogen.Con c.+Lys_conc.+X.F.Suc+X.VXdt+total.Vol+emission_O2+rab+lacta te+PO4.ferm.; 0.579, 0.810, pH+Nitrogen.Conc.+Lys_conc.+X.V Xdt+total.Vol+yield+emission_O2+rab+Thr+lactate+PO4.ferm.+P O4.apporte; 0.554, 0.810, pH+tmp+Main.Thr.Conc.+Nitrogen.Co nc.+OD+Lys_conc.+X.VXdt+total.cell+PL+emission_O2+rab+Thr+l actate+SO4.ferm.+PO4.ferm.; 0.563, 0.810, pH+tmp+Main.P-Sou rce.Conc.+Nitrogen.Conc.+OD+Lys_conc.+X.VXdt+total.Vol+tota l.cell+emission_O2+rab+Thr+lactate+PO4.ferm.; 0.594, 0.810, tmp+Main.Thr.Conc.+OD+Lys_conc.+X.VXdt+total.cell+emission _O2+Thr+lactate+PO4.ferm.; 0.571, 0.810, pH+tmp+Nitrogen.Co nc.+Lys_conc.+X.VXdt+total.Vol+emission_O2+rab+Thr+lactate+ PO4.ferm.+PO4.apporte+PO4.utilise; 0.545, 0.810, pH+tmp+Nit rogen.Conc.+OD+Lys_conc.+X.VXdt+total.Vol+total.cell+PL+emi ssion_O2+rab+Thr+His+lactate+SO4.ferm.+PO4.ferm.; 0.579, 0. 810, pH+tmp+OD+Lys_conc.+X.VXdt+total.Vol+PL+emission_O2+ra b+Thr+lactate+PO4.ferm.; 0.609, 0.810, tmp+Main.Thr.Conc.+O D+Lys_conc.+X.VXdt+total.cell+Thr+PO4.ferm.; 0.579, 0.810, tmp+Nitrogen.Conc.+Lys_conc.+X.F.Suc+X.VXdt+total.Vol+total. cell+emission_O2+rab+Thr+lactate+PO4.ferm.; 0.587, 0.810, t mp+Nitrogen.Conc.+Lys_conc.+X.F.Suc+X.VXdt+total.Vol+emissi on_O2+rab+Thr+lactate+PO4.ferm.; 0.571, 0.810, P-Source.Fee d.conc.+Nitrogen.Cone.+Lys_conc.+X.VXdt+total.Vol+yield+emi ssion_O2+rab+Thr+His+lactate+PO4.ferm.+PO4.apporte; 0.587, 0.810, pH+tmp+Nitrogen.Conc.+OD+Lys_conc.+X.F.Suc+X.VXdt+to tal.cell+His+lactate+PO4.ferm.; 0.571, 0.810, P-Source.Feed. conc.+Nitrogen.Conc.+Lys_conc.+X.VXdt+total.Vol+yield+PL+em ission_O2+rab+Thr+lactate+PO4.ferm.+PO4.apporte; 0.579, 0.8 10, pH+tmp+OD+Lys_conc.+X.VXdt+total.Vol+total.cell+emissio n_O2+rab+Thr+lactate+PO4.ferm.; 0.571, 0.810, tmp+Main.P-So urce.Cone.+P-Source.Feed.conc.+Nitrogen.Cone.+OD+Lys_conc.+ X.VXdt+total.cell+emission_O2+rab+Thr+lactate+PO4.ferm.; 0. 579, 0.810, tmp+OD+Lys_conc.+X.VXdt+total.Vol+total.cell+PL +emission_O2+rab+Thr+lactate+PO4.ferm.; 0.562, 0.810, pH+tm p+Main.Thr.Conc.+Nitrogen.Conc.+Lys_conc.+X.VXdt+total.Vol+ PL+emission_O2+rab+Thr+His+lactate+PO4.ferm.; 0.562, 0.810, pH+tmp+Nitrogen.Conc.+Lys_conc.+X.F.Suc+X.VXdt+total.Vol+t otal.cell+emission_O2+rab+Thr+His+lactate+PO4.ferm.; 0.562, 0.810, pH+tmp+Nitrogen.Conc.+Lys_conc.+X.VXdt+total.Vol+yi eld+PL+emission_O2+rab+Thr+lactate+PO4.ferm.+PO4.apporte; 0. 601, 0.810, tmp+Main.Thr.Conc.+OD+Lys_conc.+X.VXdt+total.ce ll+PL+Thr+PO4.ferm.; 0.586, 0.810, tmp+Main.P-Source.Conc.+ Nitrogen.Conc.+Lys_conc.+X.VXdt+total.Vol+emission_O2+rab+T hr+lactate+PO4.ferm.; 0.594, 0.810, tmp+Nitrogen.Conc.+OD+L ys_conc.+X.F.Suc+X.VXdt+total.cell+His+lactate+PO4.ferm.; 0. 579, 0.810, tmp+Main.Thr.Conc.+Nitrogen.Conc.+Lys_conc.+X.V Xdt+total.Vol+total.cell+emission_O2+rab+Thr+lactate+PO4.fe rm.; 0.578, 0.810, pH+tmp+Main.Thr.Conc.+Nitrogen.Conc.+Lys _conc.+X.VXdt+total.Vol+total.cell+emission_O2+rab+lactate+ PO4.ferm.; 0.562, 0.810, Main.Thr.Conc.+Nitrogen.Conc.+Lys_ conc.+X.F.Suc+X.VXdt+total.Vol+yield+emission_O2+rab+Thr+la ctate+PO4.ferm.+PO4.apporte+PO4.utilise; 0.578, 0.810, tmp+ Main.Thr.Conc.+Nitrogen.Conc.+Lys_conc.+X.VXdt+total.Vol+em ission_O2+rab+Thr+lactate+PO4.ferm.+PO4.apporte; 0.562, 0.8 10, tmp+P-Source.Feed.conc.+Nitrogen.Conc.+OD+Lys_conc.+X.V Xdt+total.Vol+total.cell+emission_O2+rab+Thr+His+lactate+PO 4.ferm.; 0.586, 0.810, tmp+P-Source.Feed.conc.+Nitrogen.Con c.+OD+Lys_conc.+X.VXdt+total.Vol+emission_O2+rab+lactate+PO 4.ferm.; 0.562, 0.810, pH+tmp+P-Source.Feed.conc.+Nitrogen. Conc.+Lys_conc.+X.VXdt+total.Vol+total.cell+emission_O2+rab +Thr+His+lactate+PO4.ferm.; 0.562, 0.810, pH+tmp+Main.Thr.C onc.+Nitrogen.Conc.+OD+Lys_conc.+X.VXdt+total.cell+emission _O2+rab+Thr+lactate+SO4.ferm.+PO4.ferm.; 0.578, 0.810, pH+P -Source.Feed.conc.+Nitrogen.Conc.+OD+Lys_conc.+X.VXdt+total. cell+emission_O2+rab+Thr+lactate+PO4.ferm.; 0.553, 0.810, p H+tmp+Main.Thr.Conc.+Nitrogen.Conc.+Lys_conc.+X.VXdt+total. Vol+total.cell+PL+emission_O2+rab+Thr+lactate+SO4.ferm.+PO4. ferm.; 0.594, 0.810, tmp+OD+Lys_conc.+X.VXdt+total.cell+PL+ emission_O2+Thr+lactate+PO4.ferm.; 0.570, 0.810, pH+tmp+P-S ource.Feed.conc.+Nitrogen.Conc.+Lys_conc.+X.VXdt+total.Vol+ PL+emission_O2+rab+His+lactate+PO4.ferm.; 0.544, 0.810, pH+ tmp+Main.Thr.Conc.+Nitrogen.Conc.+Lys_conc.+X.VXdt+total.Vo l+yield+PL+emission_O2+rab+Thr+lactate+PO4.ferm.+PO4.apport e+PO4.utilise; 0.586, 0.810, tmp+Main.Thr.Conc.+OD+Lys_conc. +X.VXdt+total.cell+PL+Thr+His+lactate+PO4.ferm.; 0.562, 0.8 10, pH+tmp+P-Source.Feed.conc.+Nitrogen.Conc.+Lys_conc.+X.V Xdt+total.Vol+PL+emission_O2+rab+Thr+His+lactate+PO4.ferm.; 0.570, 0.810, pH+tmp+Nitrogen.Conc.+Lys_conc.+X.F.Suc+X.VX dt+total.Vol+emission_O2+rab+Thr+His+lactate+PO4.ferm.; 0.5 70, 0.810, pH+tmp+Main.P-Source.Conc.+Nitrogen.Conc.+Lys_co nc.+X.F.Suc+X.VXdt+total.Vol+emission_O2+rab+His+lactate+PO 4.ferm.; 0.586, 0.810, pH+tmp+Nitrogen.Conc.+Lys_conc.+X.VX dt+total.Vol+emission_O2+rab+lactate+SO4.ferm.+PO4.ferm.; 0. 586, 0.810, Main.Thr.Conc.+Nitrogen.Conc.+Lys_conc.+X.VXdt+ total.Vol+yield+emission_O2+rab+Thr+lactate+PO4.utilise; 0. 562, 0.810, pH+Nitrogen.Conc.+Lys_conc.+X.VXdt+total.Vol+yi eld+PL+emission_O2+rab+Thr+lactate+PO4.ferm.+PO4.apporte+PO 4.utilise; 0.561, 0.810, pH+tmp+Nitrogen.Conc.+Lys_conc.+X. F.Suc+X.VXdt+total.Vol+total.cell+PL+emission_O2+rab+Thr+la ctate+PO4.ferm.; 0.601, 0.810, pH+tmp+Nitrogen.Conc.+Lys_co nc.+X.F.Suc+X.VXdt+total.Vol+lactate+PO4.ferm.; 0.578, 0.81 0, tmp+Nitrogen.Conc.+Lys_conc.+X.VXdt+total.Vol+yield+emis sion_O2+rab+Thr+lactate+PO4.ferm.+PO4.utilise; 0.561, 0.810, pH+tmp+Nitrogen.Conc.+Lys_conc.+X.VXdt+total.Vol+PL+emissi on_O2+rab+Thr+His+lactate+SO4.ferm.+PO4.ferm.; 0.608, 0.810, tmp+OD+Lys_conc.+X.VXdt+total.cell+Thr+His+PO4.ferm.; 0.58 6, 0.810, pH+tmp+Main.Thr.Conc.+OD+Lys_conc.+X.VXdt+total.c ell+emission_O2+rab+Thr+PO4.ferm.; 0.570, 0.810, tmp+Main.T hr.Conc.+Nitrogen.Conc.+OD+Lys_conc.+X.VXdt+total.cell+PL+e mission_O2+rab+Thr+lactate+PO4.ferm.; 0.570, 0.810, Nitroge n.Conc.+Lys_conc.+X.VXdt+total.Vol+yield+PL+emission_O2+rab +Thr+His+lactate+PO4.ferm.+PO4.apporte; 0.561, 0.810, pH+tm p+P-Source.Feed.conc.+Nitrogen.Conc.+OD+Lys_conc.+X.VXdt+to tal.Vol+PL+emission_O2+rab+Thr+lactate+PO4.ferm.; 0.593, 0. 810, tmp+Nitrogen.Conc.+Lys_conc.+X.VXdt+total.Vol+emission _O2+rab+His+lactate+PO4.ferm.; 0.593, 0.810, tmp+OD+Lys_con c.+X.F.Suc+X.VXdt+total.cell+emission_O2+rab+Thr+PO4.ferm.; 0.578, 0.810, pH+tmp+Main.P-Source.Conc.+OD+Lys_conc.+X.VX dt+total.cell+emission_O2+rab+Thr+lactate+PO4.ferm.; 0.570, 0.810, tmp+P-Source.Feed.conc.+Nitrogen.Conc.+Lys_conc.+X. VXdt+total.Vol+total.cell+emission_O2+rab+Thr+His+lactate+P O4.ferm.; 0.570, 0.810, tmp+Nitrogen.Conc.+Lys_conc.+X.VXdt +total.Vol+emission_O2+rab+Thr+His+lactate+PO4.ferm.+PO4.ap porte+PO4.utilise; 0.570, 0.810, pH+tmp+Main.Thr.Conc.+Lys_ conc.+X.VXdt+total.Vol+total.cell+PL+emission_O2+rab+Thr+la ctate+PO4.ferm.; 0.586, 0.810, pH+tmp+Nitrogen.Conc.+OD+Lys _conc.+X.VXdt+total.cell+PL+Thr+lactate+PO4.ferm.; 0.543, 0. 810, pH+tmp+Main.Thr.Conc.+Nitrogen.Conc.+OD+Lys_conc.+X.VX dt+total.Vol+total.cell+PL+emission_O2+rab+Thr+His+lactate+ PO4.ferm.; 0.585, 0.810, Nitrogen.Conc.+Lys_conc.+X.VXdt+to tal.Vol+yield+PL+emission_O2+rab+Thr+lactate+PO4.utilise; 0. 578, 0.810, tmp+Main.P-Source.Conc.+Nitrogen.Conc.+Lys_conc. +X.VXdt+total.Vol+total.cell+emission_O2+rab+Thr+lactate+PO 4.ferm.; 0.569, 0.810, pH+Nitrogen.Conc.+Lys_conc.+X.VXdt+t otal.Vol+yield+emission_O2+rab+Thr+lactate+PO4.ferm.+PO4.ap porte+PO4.utilise; 0.578, 0.810, pH+tmp+Main.Thr.Conc.+Lys_ conc.+X.VXdt+total.Vol+PL+emission_O2+rab+Thr+lactate+PO4.f erm.; 0.552, 0.810, pH+tmp+Main.Thr.Conc.+Nitrogen.Conc.+Ly s_conc.+X.VXdt+total.Vol+total.cell+PL+emission_O2+rab+Thr+ His+lactate+PO4.ferm.; 0.561, 0.810, pH+tmp+Main.Thr.Conc.+ OD+Lys_conc.+X.VXdt+total.cell+PL+emission_O2+rab+Thr+lacta te+SO4.ferm.+PO4.ferm.; 0.607, 0.810, tmp+Nitrogen.Conc.+Ly s_conc.+X.F.Suc+X.VXdt+total.Vol+lactate+PO4.ferm.; 0.569, 0.810, tmp+Nitrogen.Conc.+Lys_conc.+X.VXdt+total.Vol+total. cell+PL+emission_O2+rab+Thr+His+lactate+PO4.ferm.; 0.585, 0. 810, pH+tmp+Lys_conc.+X.VXdt+total.Vol+PL+emission_O2+rab+T hr+lactate+PO4.ferm.; 0.577, 0.810, pH+tmp+Main.P-Source.Co nc.+Nitrogen.Conc.+Lys_conc.+X.VXdt+total.Vol+total.cell+em ission_O2+rab+lactate+PO4.ferm.; 0.577, 0.810, pH+tmp+P-Sou rce.Feed.conc.+Nitrogen.Cone.+Lys_conc.+X.VXdt+total.Vol+yi eld+emission_O2+rab+lactate+PO4.ferm.; 0.577, 0.810, tmp+Ma in.Thr.Conc.+Nitrogen.Conc.+OD+Lys_conc.+X.F.Suc+X.VXdt+tot al.cell+Thr+His+lactate+PO4.ferm.; 0.561, 0.810, pH+tmp+Nit rogen.Conc.+Lys_conc.+X.VXdt+total.Vol+total.cell+emission-O2+rab+Thr+His+lactate+SO4.ferm.+PO4.ferm.; 0.569, 0.810, p H+tmp+Nitrogen.Conc.+Lys_conc.+X.VXdt+total.Vol+total.cell+ PL+emission_O2+rab+His+lactate+PO4.ferm.; 0.577, 0.810, pH+ tmp+OD+Lys_conc.+X.VXdt+total.cell+PL+emission_O2+rab+Thr+S O4.ferm.+PO4.ferm.; 0.569, 0.809, pH+tmp+Nitrogen.Conc.+OD+ Lys_conc.+X.VXdt+total.Vol+total.cell+PL+emission_O2+rab+la ctate+PO4.ferm.; 0.543, 0.809, tmp+Nitrogen.Conc.+Lys_conc. +X.VXdt+total.Vol+yield+PL+emission_O2+rab+Thr+His+lactate+ SO4.ferm.+PO4.ferm.+PO4.apporte+PO4.utilise; 0.561, 0.809, Main.Thr.Conc.+Nitrogen.Conc.+Lys_conc.+X.VXdt+total.Vol+yi eld+emission_O2+rab+Thr+His+lactate+PO4.ferm.+PO4.apporte+P O4.utilise; 0.585, 0.809, tmp+OD+Lys_conc.+X.VXdt+total.cel l+emission_O2+rab+Thr+lactate+SO4.ferm.+PO4.ferm.; 0.543, 0. 809, pH+tmp+Nitrogen.Conc.+Lys_conc.+X.VXdt+total.Vol+yield +PL+emission_O2+rab+Thr+His+lactate+PO4.ferm.+PO4.apporte+P O4.utilise; 0.585, 0.809, Nitrogen.Conc.+Lys_conc.+X.VXdt+t otal.Vol+yield+emission_O2+rab+Thr+lactate+PO4.apporte+PO4. utilise; 0.561, 0.809, pH+tmp+Main.Thr.Conc.+OD+Lys_conc.+X. VXdt+total.Vol+total.cell+PL+emission_O2+rab+Thr+lactate+PO 4.ferm.; 0.592, 0.809, tmp+Nitrogen.Conc.+OD+Lys_conc.+X.VX dt+total.cell+emission_O2+Thr+lactate+PO4.ferm.; 0.585, 0.8 09, tmp+Main.Thr.Conc.+Nitrogen.Conc.+OD+Lys_conc.+X.VXdt+t otal.cell+Thr+lactate+SO4.ferm.+PO4.ferm.; 0.569, 0.809, pH +tmp+Nitrogen.Conc.+Lys_conc.+X.VXdt+total.Vol+emission_O2+ rab+Thr+His+lactate+PO4.ferm.+PO4.apporte; 0.577, 0.809, pH +tmp+Main.Thr.Conc.+Nitrogen.Conc.+OD+Lys_conc.+X.VXdt+tota l.Vol+emission_O2+rab+lactate+PO4.ferm.; 0.585, 0.809, Nitr ogen.Conc.+Lys_conc.+X.F.Suc+X.VXdt+total.Vol+yield+emissio n_O2+rab+Thr+lactate+PO4.utilise; 0.592, 0.809, tmp+OD+Lys_ conc.+X.F.Suc+X.VXdt+total.cell+PL+Thr+lactate+PO4.ferm.; 0. 569, 0.809, pH+tmp+P-Source.Feed.conc.+Main.Thr.Conc.+Nitro gen.Conc.+Lys_conc.+X.VXdt+total.Vol+yield+emission_O2+rab+ lactate+PO4.ferm.; 0.569, 0.809, tmp+P-Source.Feed.conc.+Ni trogen.Conc.+OD+Lys_conc.+X.VXdt+total.cell+PL+emission_O2+ rab+Thr+lactate+PO4.ferm.; 0.577, 0.809, P-Source.Feed.conc. +Nitrogen.Conc.+Lys_conc.+X.VXdt+total.Vol+yield+emission_O 2+rab+Thr+lactate+PO4.ferm.+PO4.utilise; 0.577, 0.809, tmp+ Nitrogen.Conc.+Lys_conc.+X.VXdt+total.Vol+total.cell+emissi on_O2+rab+Thr+lactate+SO4.ferm.+PO4.ferm.; 0.551, 0.809, pH +tmp+Nitrogen.Conc.+OD+Lys_conc.+X.VXdt+total.Vol+total.cel l+emission_O2+rab+Thr+His+lactate+SO4.ferm.+PO4.ferm.; 0.56 9, 0.809, tmp+Main.Thr.Conc.+Nitrogen.Conc.+OD+Lys_conc.+X. VXdt+total.Vol+total.cell+emission_O2+rab+Thr+lactate+PO4.f erm.; 0.577, 0.809, pH+tmp+Nitrogen.Conc.+OD+Lys_conc.+X.VX dt+total.cell+emission_O2+rab+His+lactate+PO4.ferm.; 0.551, 0.809, pH+tmp+Main.Thr.Conc.+Nitrogen.Conc.+OD+Lys_conc.+X. VXdt+total.Vol+total.cell+emission_O2+rab+Thr+His+lactate+P O4.ferm.; 0.577, 0.809, tmp+Nitrogen.Conc.+Lys_conc.+X.VXdt +total.Vol+emission_O2+rab+Thr+His+lactate+PO4.ferm.+PO4.ut ilise; 0.569, 0.809, Nitrogen.Conc.+Lys_conc.+X.F.Suc+X.VXd t+total.Vol+yield+emission_O2+rab+Thr+His+lactate+PO4.ferm. +PO4.apporte; 0.592, 0.809, tmp+Main.P-Source.Conc.+Nitroge n.Conc.+OD+Lys_conc.+X.VXdt+total.cell+Thr+lactate+PO4.fer m.; 0.569, 0.809, tmp+P-Source.Feed.conc.+Nitrogen.Conc.+Ly s_conc.+X.VXdt+total.Vol+yield+emission_O2+rab+Thr+lactate+ PO4.ferm.+PO4.apporte; 0.592, 0.809, pH+tmp+OD+Lys_conc.+X. VXdt+total.cell+PL+Thr+lactate+PO4.ferm.; 0.551, 0.809, tmp +Main.Thr.Conc.+Nitrogen.Conc.+Lys_conc.+X.VXdt+total.Vol+y ield+emission_O2+rab+Thr+His+lactate+PO4.ferm.+PO4.apporte+ PO4.utilise; 0.577, 0.809, tmp+Main.Thr.Conc.+Nitrogen.Conc. +OD+Lys_conc.+X.VXdt+total.cell+PL+Thr+His+lactate+PO4.fer m.; 0.560, 0.809, pH+tmp+Main.P-Source.Conc.+Nitrogen.Conc. +OD+Lys_conc.+X.VXdt+total.Vol+PL+emission_O2+rab+Thr+lacta te+PO4.ferm.; 0.568, 0.809, tmp+Main.P-Source.Conc.+Nitroge n.Conc.+OD+Lys_conc.+X.VXdt+total.cell+emission_O2+rab+Thr+ His+lactate+PO4.ferm.; 0.577, 0.809, pH+tmp+OD+Lys_conc.+X. VXdt+total.cell+emission_O2+rab+Thr+lactate+SO4.ferm.+PO4.f erm.; 0.568, 0.809, pH+tmp+Nitrogen.Conc.+Lys conc.+X.F.Suc +X.VXdt+total.Vol+PL+emission_O2+rab+Thr+lactate+PO4.ferm.; 0.584, 0.809, tmp+Main.Thr.Conc.+OD+Lys_conc.+X.VXdt+total. cell+PL+emission_O2+Thr+lactate+PO4.ferm.; 0.551, 0.809, pH +tmp+Nitrogen.Conc.+OD+Lys_conc.+X.F.Suc+X.VXdt+total.cell+ PL+emission_O2+rab+Thr+lactate+SO4.ferm.+PO4.ferm.; 0.584, 0.809, pH+tmp+Main.Thr.Conc.+Nitrogen.Conc.+OD+Lys_conc.+X. VXdt+total.cell+Thr+lactate+PO4.ferm.; 0.542, 0.809, pH+tmp +Main.Thr.Conc.+Nitrogen.Conc.+OD+Lys_conc.+X.VXdt+total.Vo l+total.cell+PL+emission_O2+rab+Thr+lactate+SO4.ferm.+PO4.f erm.; 0.568, 0.809, pH+tmp+Nitrogen.Conc.+Lys_conc.+X.VXdt+ total.Vol+yield+emission_O2+rab+Thr+lactate+PO4.ferm.+PO4.a pporte; 0.576, 0.809, pH+tmp+Main.Thr.Conc.+Nitrogen.Conc.+ Lys_conc.+X.F.Suc+X.VXdt+total.Vol+emission_O2+rab+lactate+ PO4.ferm.; 0.560, 0.809, pH+tmp+Nitrogen.Conc.+Lys_conc.+X. VXdt+total.Vol+PL+emission_O2+rab+Thr+His+lactate+PO4.ferm. +PO4.apporte; 0.568, 0.809, tmp+Main.Thr.Conc.+Nitrogen.Con c.+OD+Lys_conc.+X.F.Suc+X.VXdt+total.cell+emission_O2+rab+T hr+lactate+PO4.ferm.; 0.568, 0.809, pH+tmp+Main.P-Source.Co nc.+Nitrogen.Conc.+OD+Lys_conc.+X.VXdt+total.Vol+emission_O 2+rab+Thr+lactate+PO4.ferm.; 0.560, 0.809, pH+tmp+P-Source. Feed.conc.+Main.Thr.Conc.+Nitrogen.Conc.+OD+Lys_conc.+X.VXd t+total.cell+emission_O2+rab+Thr+lactate+PO4.ferm.; 0.576, 0.809, pH+tmp+Nitrogen.Conc.+Lys_conc.+X.VXdt+total.Vol+emi ssion_O2+rab+His+lactate+SO4.ferm.+PO4.ferm.; 0.551, 0.809, pH+tmp+Nitrogen.Conc.+OD+Lys_conc.+X.F.Suc+X.VXdt+total.ce ll+emission_O2+rab+Thr+His+lactate+SO4.ferm.+PO4.ferm.; 0.5 99, 0.809, tmp+OD+Lys_conc.+X.VXdt+total.cell+Thr+lactate+S O4.ferm.+PO4.ferm.; 0.576, 0.809, tmp+Main.P-Source.Conc.+P -Source.Feed.conc.+Nitrogen.Conc.+Lys_conc.+X.VXdt+total.Vo l+emission_O2+rab+His+lactate+PO4.ferm.; 0.568, 0.809, tmp+ Lys_conc.+X.VXdt+total.Vol+yield+PL+emission_O2+rab+Thr+lac tate+PO4.ferm.+PO4.apporte+PO4.utilise; 0.576, 0.809, tmp+P -Source.Feed.conc.+Nitrogen.Conc.+Lys_conc.+X.VXdt+total.Vo l+emission_O2+rab+Thr+lactate+PO4.ferm.+PO4.apporte; 0.551, 0.809, Nitrogen.Conc.+Lys_conc.+X.VXdt+total.Vol+yield+PL+ emission_O2+rab+Thr+His+lactate+SO4.ferm.+PO4.ferm.+PO4.app orte+PO4.utilise; 0.584, 0.809, tmp+Nitrogen.Conc.+Lys_conc. +X.VXdt+total.Vol+emission_O2+rab+Thr+lactate+PO4.apporte+P O4.utilise; 0.568, 0.809, pH+tmp+Nitrogen.Conc.+OD+Lys_conc. +X.F.Suc+X.VXdt+total.cell+PL+emission_O2+rab+Thr+PO4.fer m.; 0.560, 0.809, P-Source.Feed.conc.+Nitrogen.Conc.+Lys_co nc.+X.VXdt+total.Vol+yield+PL+emission_O2+rab+Thr+lactate+P O4.ferm.+PO4.apporte+PO4.utilise; 0.584, 0.809, tmp+Nitroge n.Conc.+OD+Lys conc.+X.VXdt+total.cell+Thr+His+lactate+SO4. ferm.+PO4.ferm.; 0.568, 0.809, pH+tmp+P-Source.Feed.conc.+N itrogen.Conc.+Lys_conc.+X.F.Suc+X.VXdt+total.Vol+emission_O 2+rab+Thr+lactate+PO4.ferm.; 0.576, 0.809, tmp+OD+Lys_conc. +X.VXdt+total.cell+PL+emission_O2+rab+Thr+lactate+SO4.ferm. +PO4.ferm.; 0.576, 0.809, pH+tmp+Nitrogen.Conc.+Lys_conc.+X. F.Suc+X.VXdt+total.Vol+emission_O2+rab+lactate+SO4.ferm.+PO 4.ferm.; 0.559, 0.809, tmp+Nitrogen.Conc.+OD+Lys_conc.+X.VX dt+total.Vol+total.cell+PL+emission_O2+rab+Thr+His+lactate+ PO4.ferm.; 0.568, 0.809, tmp+Nitrogen.Conc.+OD+Lys_conc.+X. F.Suc+X.VXdt+total.cell+emission_O2+rab+Thr+lactate+SO4.fer m.+PO4.ferm.; 0.568, 0.809, P-Source.Feed.conc.+Nitrogen.Co nc.+Lys_conc.+X.VXdt+total.Vol+yield+emission_O2+rab+Thr+la ctate+PO4.ferm.+PO4.apporte+PO4.utilise; 0.576, 0.809, tmp+ OD+Lys_conc.+X.F.Suc+X.VXdt+total.cell+PL+emission_O2+rab+T hr+lactate+PO4.ferm.; 0.584, 0.809, tmp+Nitrogen.Conc.+OD+L ys_conc.+X.VXdt+total.cell+PL+emission_O2+rab+Thr+PO4.fer m.; 0.584, 0.809, tmp+P-Source.Feed.conc.+Main.Thr.Conc.+Ni trogen.Conc.+Lys_conc.+X.VXdt+total.Vol+emission_O2+rab+lac tate+PO4.ferm.; 0.612, 0.809, tmp+Nitrogen.Conc.+Lys_conc.+ X.VXdt+total.Vol+lactate+PO4.ferm.; 0.576, 0.809, pH+tmp+Ma in.Thr.Conc.+Nitrogen.Conc.+Lys_conc.+X.VXdt+total.Vol+PL+e mission_O2+rab+lactate+PO4.ferm.; 0.550, 0.809, Main.Thr.Co nc.+Nitrogen.Conc.+Lys_conc.+X.VXdt+total.Vol+yield+PL+emis sion_O2+rab+Thr+His+lactate+PO4.ferm.+PO4.apporte+PO4.utili se; 0.584, 0.809, tmp+Nitrogen.Conc.+OD+Lys_conc.+X.F.Suc+X. VXdt+total.cell+emission_O2+Thr+lactate+PO4.ferm.; 0.559, 0. 809, pH+tmp+Main.Thr.Conc.+Nitrogen.Conc.+Lys_conc.+X.VXdt+ total.Vol+total.cell+emission_O2+rab+Thr+His+lactate+PO4.fe rm.; 0.567, 0.809, pH+P-Source.Feed.conc.+Nitrogen.Conc.+OD +Lys_conc.+X.F.Suc+X.VXdt+total.cell+emission_O2+rab+Thr+la ctate+PO4.ferm.; 0.567, 0.809, pH+tmp+Nitrogen.Conc.+Lys_co nc.+X.VXdt+total.Vol+emission_O2+rab+Thr+His+lactate+SO4.fe rm.+PO4.ferm.; 0.567, 0.809, tmp+Main.P-Source.Conc.+P-Sour ce.Feed.conc.+Nitrogen.Conc.+Lys_conc.+X.VXdt+total.Vol+emi ssion_O2+rab+Thr+His+lactate+PO4.ferm.; 0.576, 0.809, tmp+O D+Lys_conc.+X.F.Suc+X.VXdt+total.cell+emission_O2+rab+Thr+H is+lactate+PO4.ferm.; 0.583, 0.809, pH+tmp+Lys_conc.+X.VXdt +total.Vol+total.cell+emission_O2+rab+Thr+lactate+PO4.fer m.; 0.567, 0.809, Main.Thr.Conc.+Nitrogen.Conc.+Lys conc.+X. VXdt+total.Vol+yield+emission_O2+rab+Thr+His+lactate+PO4.fe rm.+PO4.apporte; 0.575, 0.809, pH+tmp+Nitrogen.Conc.+OD+Lys _conc.+X.F.Suc+X.VXdt+total.cell+Thr+His+lactate+PO4.ferm.; 0.559, 0.809, tmp+Nitrogen.Conc.+Lys_conc.+X.F.Suc+X.VXdt+ total.Vol+yield+emission_O2+rab+Thr+lactate+PO4.ferm.+PO4.a pporte+PO4.utilise; 0.598, 0.809, pH+tmp+OD+Lys_conc.+X.VXd t+total.cell+PL+Thr+PO4.ferm.; 0.583, 0.809, tmp+Nitrogen.C onc.+Lys_conc.+X.VXdt+total.Vol+emission_O2+rab+Thr+lactate +SO4.ferm.+PO4.ferm.; 0.567, 0.809, tmp+P-Source.Feed.conc. +Main.Thr.Conc.+Nitrogen.Conc.+OD+Lys_conc.+X.VXdt+total.ce ll+emission_O2+rab+Thr+lactate+PO4.ferm.; 0.559, 0.809, pH+ tmp+Main.Thr.Conc.+Nitrogen.Conc.+Lys_conc.+X.VXdt+total.Vo l+total.cell+emission_O2+rab+Thr+lactate+SO4.ferm.+PO4.fer m.; 0.559, 0.808, pH+tmp+Main.Thr.Conc.+Nitrogen.Conc.+Lys_ conc.+X.VXdt+total.Vol+PL+emission_O2+rab+Thr+lactate+PO4.f erm.+PO4.apporte; 0.591, 0.808, tmp+OD+Lys_conc.+X.F.Suc+X. VXdt+total.cell+Thr+His+lactate+PO4.ferm.; 0.583, 0.808, tm p+Main.Thr.Conc.+Nitrogen.Conc.+OD+Lys_conc.+X.VXdt+total.c ell+emission_O2+Thr+lactate+PO4.ferm.; 0.583, 0.808, pH+tmp +Nitrogen.Conc.+OD+Lys_conc.+X.F.Suc+X.VXdt+total.cell+emis sion_O2+rab+PO4.ferm.; 0.567, 0.808, pH+tmp+Main.P-Source.C onc.+P-Source.Feed.conc.+Main.Thr.Conc.+Nitrogen.Conc.+Lys_ conc.+X.VXdt+total.Vol+emission_O2+rab+lactate+PO4.ferm.; 0. 567, 0.808, tmp+Nitrogen.Conc.+OD+Lys_conc.+X.VXdt+total.ce ll+PL+emission_O2+rab+Thr+lactate+SO4.ferm.+PO4.ferm.; 0.57 5, 0.808, pH+tmp+Main.P-Source.Conc.+Main.Thr.Conc.+Nitroge n.Conc.+Lys_conc.+X.VXdt+total.Vol+emission_O2+rab+lactate+ PO4.ferm.; 0.567, 0.808, pH+tmp+Main.Thr.Conc.+Nitrogen.Con c.+Lys_conc.+X.VXdt+total.Vol+emission_O2+rab+Thr+lactate+P O4.ferm.+PO4.apporte; 0.575, 0.808, pH+tmp+Main.P-Source.Co nc.+Nitrogen.Conc.+OD+Lys_conc.+X.VXdt+total.Vol+emission_O 2+rab+lactate+PO4.ferm.; 0.567, 0.808, tmp+Main.P-Source.Co nc.+Nitrogen.Conc.+OD+Lys_conc.+X.VXdt+total.cell+PL+emissi on_O2+rab+Thr+lactate+PO4.ferm.; 0.575, 0.808, P-Source.Fee d.conc.+Nitrogen.Cone.+OD+Lys_conc.+X.F.Suc+X.VXdt+total.ce ll+emission_O2+rab+Thr+lactate+PO4.ferm.; 0.567, 0.808, pH+ tmp+Main.P-Source.Conc.+Main.Thr.Conc.+Nitrogen.Conc.+Lys_c onc.+X.VXdt+total.Vol+emission_O2+rab+Thr+lactate+PO4.fer m.; 0.605, 0.808, tmp+OD+Lys_conc.+X.F.Suc+X.VXdt+total.cel l+Thr+PO4.ferm.; 0.567, 0.808, pH+tmp+Main.P-Source.Conc.+P -Source.Feed.conc.+Nitrogen.Conc.+Lys_conc.+X.VXdt+total.Vo l+total.cell+emission_O2+rab+lactate+PO4.ferm.; 0.567, 0.80 8, tmp+Main.Thr.Conc.+Nitrogen.Conc.+OD+Lys_conc.+X.VXdt+to tal.cell+emission_O2+rab+Thr+His+lactate+PO4.ferm.; 0.575, 0.808, tmp+Nitrogen.Conc.+Lys_conc.+X.VXdt+total.Vol+PL+emi ssion_O2+rab+Thr+lactate+PO4.ferm.+PO4.utilise; 0.549, 0.80 8, pH+tmp+P-Source.Feed.conc.+Nitrogen.Conc.+OD+Lys_conc.+X. VXdt+total.cell+PL+emission_O2+rab+Thr+His+lactate+PO4.fer m.; 0.549, 0.808, tmp+Nitrogen.Conc.+Lys_conc.+X.VXdt+total. Vol+yield+emission_O2+rab+Thr+His+lactate+SO4.ferm.+PO4.fer m.+PO4.apporte+PO4.utilise; 0.583, 0.808, tmp+Nitrogen.Conc. +OD+Lys_conc.+X.F.Suc+X.VXdt+total.cell+emission_O2+rab+Thr +PO4.ferm.; 0.575, 0.808, pH+tmp+OD+Lys_conc.+X.VXdt+total. Vol+total.cell+PL+emission_O2+rab+Thr+PO4.ferm.; 0.567, 0.8 08, pH+tmp+Nitrogen.Conc.+OD+Lys_conc.+X.VXdt+total.Vol+PL+ emission_O2+rab+His+lactate+PO4.ferm.; 0.558, 0.808, tmp+Ma in.P-Source.Conc.+P-Source.Feed.conc.+Nitrogen.Conc.+OD+Lys _conc.+X.VXdt+total.Vol+total.cell+emission_O2+rab+Thr+lact ate+PO4.ferm.; 0.590, 0.808, tmp+Nitrogen.Conc.+Lys_conc.+X. VXdt+total.Vol+total.cell+emission_O2+rab+lactate+PO4.fer m.; 0.575, 0.808, pH+tmp+Main.P-Source.Conc.+OD+Lys_conc.+X. VXdt+total.cell+PL+emission_O2+rab+Thr+PO4.ferm.; 0.558, 0. 808, pH+tmp+Main.Thr.Conc.+OD+Lys_conc.+X.VXdt+total.cell+P L+emission_O2+rab+Thr+His+lactate+PO4.ferm.; 0.575, 0.808, pH+tmp+Nitrogen.Conc.+Lys_conc.+X.VXdt+total.Vol+emission_O 2+rab+Thr+lactate+PO4.apporte+PO4.utilise; 0.558, 0.808, pH +tmp+Main.P-Source.Conc.+P-Source.Feed.conc.+Nitrogen.Conc. +OD+Lys_conc.+X.VXdt+total.cell+emission_O2+rab+Thr+lactate +PO4.ferm.; 0.583, 0.808, tmp+Nitrogen.Conc.+OD+Lys_conc.+X. VXdt+total.cell+PL+emission_O2+Thr+lactate+PO4.ferm.; 0.549, 0.808, pH+tmp+Nitrogen.Conc.+OD+Lys_conc.+X.VXdt+total.Vol +PL+emission_O2+rab+Thr+His+lactate+SO4.ferm.+PO4.ferm.; 0. 575, 0.808, tmp+Nitrogen.Conc.+OD+Lys_conc.+X.F.Suc+X.VXdt+ total.cell+Thr+His+lactate+SO4.ferm.+PO4.ferm.; 0.558, 0.80 8, pH+tmp+Nitrogen.Conc.+Lys_conc.+X.VXdt+total.Vol+yield+e mission_O2+rab+Thr+lactate+PO4.ferm.+PO4.apporte+PO4.utilis e; 0.567, 0.808, Main.Thr.Conc.+Nitrogen.Conc.+Lys_conc.+X. F.Suc+X.VXdt+total.Vol+yield+emission_O2+rab+Thr+lactate+PO 4.ferm.+PO4.apporte; 0.549, 0.808, pH+tmp+Main.Thr.Conc.+Ni trogen.Conc.+OD+Lys_conc.+X.F.Suc+X.VXdt+total.cell+PL+emis sion_O2+rab+Thr+lactate+PO4.ferm.; 0.582, 0.808, tmp+Nitrog en.Conc.+OD+Lys_conc.+X.F.Suc+X.VXdt+total.cell+emission_O2 +rab+lactate+PO4.ferm.; 0.549, 0.808, pH+tmp+Nitrogen.Conc. +Lys_conc.+X.VXdt+total.Vol+PL+emission_O2+rab+Thr+His+lact ate+PO4.ferm.+PO4.apporte+PO4.utilise; 0.566, 0.808, tmp+Ma in.P-Source.Conc.+P-Source.Feed.conc.+Nitrogen.Conc.+Lys_co nc.+X.VXdt+total.Vol+total.cell+emission_O2+rab+Thr+lactate +PO4.ferm.; 0.549, 0.808, pH+tmp+Main.Thr.Conc.+Nitrogen.Co nc.+OD+Lys_conc.+X.VXdt+total.Vol+total.cell+emission_O2+ra b+Thr+lactate+SO4.ferm.+PO4.ferm.; 0.558, 0.808, pH+tmp+Mai n.P-Source.Conc.+P-Source.Feed.conc.+Nitrogen.Conc.+Lys_con c.+X.VXdt+total.Vol+PL+emission_O2+rab+Thr+lactate+PO4.fer m.; 0.549, 0.808, pH+tmp+Main.P-Source.Conc.+Nitrogen.Conc. +OD+Lys_conc.+X.F.Suc+X.VXdt+total.cell+emission_O2+rab+Thr +His+lactate+PO4.ferm.; 0.582, 0.808, tmp+Main.Thr.Conc.+OD +Lys conc.+X.VXdt+total.cell+PL+Thr+lactate+SO4.ferm.+PO4.f erm.; 0.549, 0.808, Nitrogen.Conc.+Lys_conc.+X.F.Suc+X.VXdt +total.Vol+yield+PL+emission_O2+rab+Thr+His+lactate+PO4.fer m.+PO4.apporte+PO4.utilise; 0.574, 0.808, tmp+Main.P-Source. Conc.+OD+Lys_conc.+X.VXdt+total.cell+PL+emission_O2+rab+Thr +lactate+PO4.ferm.; 0.549, 0.808, pH+tmp+Main.P-Source.Conc. +Nitrogen.Conc.+OD+Lys_conc.+X.VXdt+total.cell+PL+emission_ O2+rab+Thr+His+lactate+PO4.ferm.; 0.566, 0.808, Main.Thr.Co nc.+Nitrogen.Conc.+Lys_conc.+X.VXdt+total.Vol+yield+PL+emis sion_O2+rab+Thr+lactate+PO4.ferm.+PO4.apporte; 0.566, 0.808, pH+tmp+Main.P-Source.Cone.+Nitrogen.Cone.+Lys_conc.+X.VXdt +total.Vol+total.cell+emission_O2+rab+His+lactate+PO4.fer m.; 0.574, 0.808, tmp+P-Source.Feed.conc.+Nitrogen.Conc.+Ly s_conc.+X.VXdt+total.Vol+total.cell+emission_O2+rab+His+lac tate+PO4.ferm.; 0.590, 0.808, tmp+Main.P-Source.Conc.+OD+Ly s_conc.+X.VXdt+total.cell+emission_O2+rab+Thr+PO4.ferm.; 0. 597, 0.808, tmp+OD+Lys_conc.+X.VXdt+total.cell+emission_O2+ Thr+lactate+PO4.ferm.; 0.566, 0.808, pH+tmp+P-Source.Feed.c onc.+Nitrogen.Conc.+Lys_conc.+X.VXdt+total.Vol+emission_O2+ rab+Thr+lactate+SO4.ferm.+PO4.ferm.; 0.574, 0.808, pH+tmp+M ain.Thr.Conc.+Nitrogen.Conc.+OD+Lys_conc.+X.F.Suc+X.VXdt+to tal.cell+Thr+lactate+PO4.ferm.; 0.574, 0.808, Nitrogen.Conc. +Lys_conc.+X.VXdt+total.Vol+yield+PL+emission_O2+Thr+lactat e+PO4.ferm.+PO4.apporte+PO4.utilise; 0.574, 0.808, Nitrogen. Conc.+Lys_conc.+X.VXdt+total.Vol+yield+emission_O2+rab+Thr+ lactate+SO4.ferm.+PO4.ferm.+PO4.apporte; 0.597, 0.808, tmp+ Main.Thr.Conc.+Nitrogen.Conc.+OD+Lys_conc.+X.VXdt+total.cel l+Thr+PO4.ferm.; 0.582, 0.808, pH+tmp+Nitrogen.Conc.+Lys_co nc.+X.VXdt+total.Vol+emission_O2+rab+lactate+PO4.ferm.+PO4. apporte; 0.582, 0.808, tmp+Main.P-Source.Conc.+Nitrogen.Con c.+OD+Lys_conc.+X.F.Suc+X.VXdt+total.cell+Thr+lactate+PO4.f erm.; 0.566, 0.808, pH+tmp+Lys_conc.+X.VXdt+total.Vol+total. cell+PL+emission_O2+rab+Thr+His+lactate+PO4.ferm.; 0.539, 0. 808, pH+tmp+P-Source.Feed.conc.+Nitrogen.Conc.+OD+Lys_conc. +X.VXdt+total.Vol+total.cell+PL+emission_O2+rab+Thr+His+lac tate+PO4.ferm.; 0.566, 0.808, pH+tmp+P-Source.Feed.conc.+Ni trogen.Conc.+Lys_conc.+X.VXdt+total.Vol+yield+emission_O2+r ab+His+lactate+PO4.ferm.; 0.566, 0.808, pH+tmp+Main.Thr.Con c.+Nitrogen.Conc.+OD+Lys_conc.+X.VXdt+total.cell+PL+emissio n_O2+rab+Thr+PO4.ferm.; 0.566, 0.808, pH+tmp+P-Source.Feed. conc.+Main.Thr.Conc.+Nitrogen.Conc.+Lys_conc.+X.VXdt+total. Vol+total.cell+emission_O2+rab+lactate+PO4.ferm.; 0.548, 0. 808, pH+tmp+P-Source.Feed.conc.+Nitrogen.Conc.+OD+Lys_conc. +X.F.Suc+X.VXdt+total.cell+emission_O2+rab+Thr+His+lactate+ PO4.ferm.; 0.566, 0.808, tmp+Nitrogen.Conc.+OD+Lys_conc.+X. VXdt+total.Vol+total.cell+emission_O2+rab+Thr+lactate+SO4.f erm.+PO4.ferm.; 0.597, 0.808, tmp+Nitrogen.Conc.+OD+Lys_con c.+X.F.Suc+X.VXdt+total.cell+Thr+PO4.ferm.; 0.574, 0.808, t mp+Main.Thr.Conc.+OD+Lys_conc.+X.VXdt+total.cell+emission_O 2+rab+Thr+His+lactate+PO4.ferm.; 0.582, 0.808, pH+Nitrogen. Conc.+Lys_conc.+X.VXdt+total.Vol+yield+emission_O2+rab+Thr+ lactate+PO4.utilise; 0.557, 0.808, pH+tmp+OD+Lys_conc.+X.VX dt+total.Vol+total.cell+PL+emission_O2+rab+Thr+His+lactate+ PO4.ferm.; 0.548, 0.808, pH+tmp+Main.Thr.Conc.+Nitrogen.Con c.+OD+Lys_conc.+X.F.Suc+X.VXdt+total.cell+emission_O2+rab+T hr+His+lactate+PO4.ferm.; 0.557, 0.808, pH+tmp+Main.P-Sourc e.Conc.+Nitrogen.Conc.+Lys_conc.+X.VXdt+total.Vol+total.cel l+emission_O2+rab+Thr+His+lactate+PO4.ferm.; 0.597, 0.808, pH+tmp+OD+Lys conc.+X.VXdt+total.cell+Thr+lactate+PO4.fer m.; 0.566, 0.808, pH+tmp+Lys_conc.+X.VXdt+total.Vol+total.c ell+PL+emission_O2+rab+Thr+lactate+SO4.ferm.+PO4.ferm.; 0.5 74, 0.808, pH+tmp+P-Source.Feed.conc.+Nitrogen.Conc.+Lys_co nc.+X.VXdt+total.Vol+emission_O2+rab+lactate+SO4.ferm.+PO4. ferm.; 0.597, 0.808, tmp+Nitrogen.Conc.+OD+Lys_conc.+X.VXdt +total.cell+Thr+His+PO4.ferm.; 0.582, 0.808, pH+tmp+Main.Th r.Conc.+Nitrogen.Conc.+OD+Lys_conc.+X.F.Suc+X.VXdt+total.ce ll+lactate+PO4.ferm.; 0.557, 0.808, Nitrogen.Conc.+Lys_conc. +X.F.Suc+X.VXdt+total.Vol+yield+emission_O2+rab+Thr+lactate +SO4.ferm.+PO4.ferm.+PO4.apporte+PO4.utilise; 0.557, 0.808, tmp+Main.Thr.Conc.+Lys_conc.+X.VXdt+total.Vol+yield+PL+emi ssion_O2+rab+Thr+lactate+PO4.ferm.+PO4.apporte+PO4.utilise; 0.565, 0.808, tmp+Main.P-Source.Conc.+Nitrogen.Conc.+OD+Ly s_conc.+X.VXdt+total.Vol+total.cell+emission_O2+rab+Thr+lac tate+PO4.ferm.; 0.565, 0.808, tmp+Main.Thr.Conc.+Nitrogen.C onc.+Lys_conc.+X.VXdt+total.Vol+emission_O2+rab+Thr+lactate +PO4.ferm.+PO4.apporte+PO4.utilise; 0.565, 0.808, pH+tmp+Ma in.P-Source.Conc.+Nitrogen.Conc.+Lys_conc.+X.VXdt+total.Vol +emission_O2+rab+Thr+His+lactate+PO4.ferm.; 0.574, 0.808, t mp+Main.Thr.Conc.+Nitrogen.Conc.+Lys_conc.+X.VXdt+total.Vol +emission_O2+rab+Thr+lactate+PO4.ferm.+PO4.utilise; 0.548, 0.808, pH+tmp+Main.Thr.Conc.+Nitrogen.Conc.+OD+Lys_conc.+X. VXdt+total.Vol+PL+emission_O2+rab+Thr+lactate+SO4.ferm.+PO4. ferm.; 0.581, 0.808, tmp+OD+Lys_conc.+X.VXdt+total.cell+PL+ Thr+His+lactate+SO4.ferm.+PO4.ferm.; 0.565, 0.808, pH+tmp+M ain.P-Source.Conc.+P-Source.Feed.conc.+Nitrogen.Conc.+OD+Ly s_conc.+X.VXdt+total.Vol+emission_O2+rab+lactate+PO4.ferm.; 0.574, 0.808, pH+tmp+Main.Thr.Conc.+Nitrogen.Conc.+Lys_con c.+X.VXdt+total.Vol+emission_O2+rab+lactate+SO4.ferm.+PO4.f erm.; 0.557, 0.808, tmp+Nitrogen.Conc.+Lys_conc.+X.VXdt+tot al.Vol+yield+PL+emission_O2+rab+Thr+His+lactate+PO4.ferm.+P O4.apporte; 0.581, 0.808, tmp+OD+Lys_conc.+X.VXdt+total.cel l+PL+emission_O2+rab+Thr+His+PO4.ferm.; 0.548, 0.808, pH+tm p+P-Source.Feed.conc.+Main.Thr.Conc.+Nitrogen.Conc.+OD+Lys_ conc.+X.VXdt+total.Vol+total.cell+emission_O2+rab+Thr+lacta te+PO4.ferm.; 0.589, 0.808, tmp+Nitrogen.Conc.+OD+Lys_conc. +X.VXdt+total.cell+PL+Thr+His+PO4.ferm.; 0.589, 0.808, tmp+ Main.Thr.Conc.+OD+Lys_conc.+X.F.Suc+X.VXdt+total.cell+Thr+l actate+PO4.ferm.; 0.574, 0.808, tmp+Nitrogen.Conc.+Lys_conc. +X.VXdt+total.Vol+yield+emission_O2+rab+lactate+PO4.ferm.+P O4.apporte+PO4.utilise; 0.565, 0.808, pH+tmp+Main.Thr.Conc. +OD+Lys_conc.+X.VXdt+total.cell+emission_O2+rab+Thr+His+lac tate+PO4.ferm.; 0.557, 0.808, tmp+Nitrogen.Conc.+Lys_conc.+ X.VXdt+total.Vol+PL+emission_O2+rab+Thr+His+lactate+PO4.fer m.+PO4.apporte+PO4.utilise; 0.565, 0.808, pH+tmp+Main.P-Sou rce.Conc.+Nitrogen.Conc.+Lys_conc.+X.F.Suc+X.VXdt+total.Vol +total.cell+emission_O2+rab+lactate+PO4.ferm.; 0.548, 0.808, pH+tmp+Nitrogen.Conc.+OD+Lys_conc.+X.F.Suc+X.VXdt+total.Vo l+total.cell+emission_O2+rab+Thr+lactate+SO4.ferm.+PO4.fer m.; 0.565, 0.808, pH+Nitrogen.Conc.+Lys_conc.+X.VXdt+total. Vol+yield+PL+emission_O2+rab+Thr+lactate+PO4.ferm.+PO4.appo rte; 0.565, 0.808, pH+tmp+Main.Thr.Conc.+Lys_conc.+X.VXdt+t otal.Vol+PL+emission_O2+rab+Thr+lactate+SO4.ferm.+PO4.fer m.; 0.589, 0.808, tmp+Main.Thr.Conc.+Nitrogen.Conc.+OD+Lys_ conc.+X.F.Suc+X.VXdt+total.cell+lactate+PO4.ferm.; 0.581, 0. 808, tmp+Nitrogen.Conc.+OD+Lys_conc.+X.VXdt+total.cell+emis sion_O2+rab+Thr+His+PO4.ferm.; 0.573, 0.808, tmp+Nitrogen.C onc.+OD+Lys_conc.+X.F.Suc+X.VXdt+total.cell+PL+Thr+His+lact ate+PO4.ferm.; 0.589, 0.807, tmp+Main.Thr.Conc.+OD+Lys_conc. +X.VXdt+total.cell+Thr+lactate+SO4.ferm.+PO4.ferm.; 0.556, 0.807, pH+tmp+Main.P-Source.Conc.+Main.Thr.Conc.+Nitrogen.C onc.+OD+Lys_conc.+X.VXdt+total.cell+emission_O2+rab+Thr+lac tate+PO4.ferm.; 0.556, 0.807, pH+tmp+Nitrogen.Conc.+Lys_con c.+X.VXdt+total.Vol+PL+emission_O2+rab+Thr+His+lactate+PO4. ferm.+PO4.utilise; 0.565, 0.807, pH+tmp+Nitrogen.Conc.+Lys_ conc.+X.VXdt+total.Vol+PL+emission_O2+rab+Thr+lactate+PO4.a pporte+PO4.utilise; 0.573, 0.807, tmp+Nitrogen.Conc.+Lys_co nc.+X.VXdt+total.Vol+PL+emission_O2+rab+Thr+His+lactate+PO4. ferm.; 0.556, 0.807, pH+tmp+P-Source.Feed.conc.+Main.Thr.Co nc.+Nitrogen.Conc.+Lys_conc.+X.VXdt+total.Vol+emission_O2+r ab+Thr+His+lactate+PO4.ferm.; 0.565, 0.807, pH+tmp+P-Source. Feed.conc.+Main.Thr.Conc.+Nitrogen.Conc.+OD+Lys_conc.+X.VXd t+total.Vol+emission_O2+rab+lactate+PO4.ferm.; 0.556, 0.807, pH+tmp+Nitrogen.Conc.+OD+Lys_conc.+X.VXdt+total.Vol+total. cell+PL+emission_O2+rab+His+lactate+PO4.ferm.; 0.556, 0.807, tmp+P-Source.Feed.conc.+Nitrogen.Conc.+OD+Lys_conc.+X.VXdt +total.Vol+total.cell+PL+emission_O2+rab+Thr+lactate+PO4.fe rm.; 0.565, 0.807, pH+tmp+Nitrogen.Conc.+Lys_conc.+X.VXdt+t otal.Vol+emission_O2+rab+Thr+His+lactate+PO4.ferm.+PO4.util ise; 0.581, 0.807, pH+tmp+Nitrogen.Conc.+OD+Lys_conc.+X.VXd t+total.cell+Thr+His+lactate+PO4.ferm.; 0.596, 0.807, pH+tm p+OD+Lys_conc.+X.VXdt+total.cell+emission_O2+rab+PO4.ferm.; 0.573, 0.807, tmp+P-Source.Feed.conc.+Nitrogen.Conc.+OD+Ly s_conc.+X.VXdt+total.Vol+emission_O2+rab+His+lactate+PO4.fe rm.; 0.565, 0.807, pH+tmp+P-Source.Feed.conc.+Nitrogen.Conc. +Lys_conc.+X.F.Suc+X.VXdt+total.Vol+total.cell+emission_O2+ rab+lactate+PO4.ferm.; 0.547, 0.807, pH+tmp+P-Source.Feed.c onc.+Nitrogen.Conc.+Lys_conc.+X.VXdt+total.Vol+total.cell+P L+emission_O2+rab+Thr+His+lactate+PO4.ferm.; 0.581, 0.807, pH+tmp+Main.Thr.Conc.+Lys_conc.+X.VXdt+total.Vol+emission_O 2+rab+Thr+lactate+PO4.ferm.; 0.573, 0.807, tmp+Nitrogen.Con c.+Lys_conc.+X.F.Suc+X.VXdt+total.Vol+emission_O2+rab+Thr+l actate+PO4.ferm.+PO4.apporte; 0.556, 0.807, pH+tmp+P-Source. Feed.conc.+Nitrogen.Conc.+Lys_conc.+X.VXdt+total.Vol+PL+emi ssion_O2+rab+Thr+lactate+SO4.ferm.+PO4.ferm.; 0.565, 0.807, tmp+P-Source.Feed.conc.+Nitrogen.Conc.+Lys_conc.+X.VXdt+to tal.Vol+total.cell+PL+emission_O2+rab+Thr+lactate+PO4.fer m.; 0.565, 0.807, pH+tmp+Main.P-Source.Conc.+Nitrogen.Conc. +OD+Lys_conc.+X.F.Suc+X.VXdt+total.cell+emission_O2+rab+lac tate+PO4.ferm.; 0.573, 0.807, tmp+Nitrogen.Conc.+OD+Lys_con c.+X.VXdt+total.cell+PL+Thr+His+lactate+SO4.ferm.+PO4.fer m.; 0.573, 0.807, pH+tmp+Nitrogen.Conc.+Lys_conc.+X.F.Suc+X. VXdt+total.Vol+PL+emission_O2+rab+lactate+PO4.ferm.; 0.573, 0.807, tmp+Main.Thr.Conc.+Nitrogen.Conc.+OD+Lys_conc.+X.VX dt+total.Vol+emission_O2+rab+Thr+lactate+PO4.ferm.; 0.565, 0.807, pH+tmp+Main.Thr.Conc.+Nitrogen.Conc.+OD+Lys_conc.+X. VXdt+total.Vol+total.cell+emission_O2+rab+lactate+PO4.fer m.; 0.565, 0.807, pH+tmp+OD+Lys_conc.+X.F.Suc+X.VXdt+total. cell+emission_O2+rab+Thr+His+lactate+PO4.ferm.; 0.556, 0.80 7, pH+tmp+P-Source.Feed.conc.+Nitrogen.Conc.+OD+Lys_conc.+X. VXdt+total.Vol+emission_O2+rab+Thr+His+lactate+PO4.ferm.; 0. 547, 0.807, pH+tmp+Main.Thr.Conc.+Nitrogen.Conc.+Lys_conc.+ X.VXdt+total.Vol+PL+emission_O2+rab+Thr+His+lactate+SO4.fer m.+PO4.ferm.; 0.596, 0.807, tmp+OD+Lys_conc.+X.F.Suc+X.VXdt +total.cell+PL+Thr+PO4.ferm.; 0.556, 0.807, pH+tmp+OD+Lys_c onc.+X.VXdt+total.Vol+total.cell+PL+emission_O2+rab+Thr+lac tate+SO4.ferm.+PO4.ferm.; 0.573, 0.807, tmp+P-Source.Feed.c onc.+Nitrogen.Conc.+OD+Lys_conc.+X.VXdt+total.Vol+total.cel l+emission_O2+rab+lactate+PO4.ferm.; 0.573, 0.807, tmp+Main. Thr.Conc.+Nitrogen.Conc.+OD+Lys_conc.+X.F.Suc+X.VXdt+total. cell+Thr+lactate+SO4.ferm.+PO4.ferm.; 0.588, 0.807, tmp+Nit rogen.Conc.+OD+Lys_conc.+X.VXdt+total.Vol+emission_O2+rab+l actate+PO4.ferm.; 0.580, 0.807, tmp+P-Source.Feed.conc.+OD+ Lys_conc.+X.VXdt+total.cell+emission_O2+rab+Thr+lactate+PO4. ferm.; 0.580, 0.807, pH+tmp+Main.P-Source.Conc.+OD+Lys_conc. +X.VXdt+total.cell+emission_O2+rab+Thr+PO4.ferm.; 0.573, 0. 807, tmp+Main.Thr.Conc.+Nitrogen.Conc.+OD+Lys_conc.+X.VXdt+ total.cell+PL+Thr+lactate+SO4.ferm.+PO4.ferm.; 0.616, 0.807, tmp+OD+Lys_conc.+X.VXdt+total.cell+PO4.ferm.; 0.564, 0.807, tmp+Main.P-Source.Conc.+P-Source.Feed.conc.+Main.Thr.Conc. +Nitrogen.Conc.+Lys_conc.+X.VXdt+total.Vol+emission_O2+rab+ Thr+lactate+PO4.ferm.; 0.580, 0.807, pH+tmp+Lys_conc.+X.VXd t+total.Vol+total.cell+PL+emission_O2+rab+Thr+PO4.ferm.; 0. 572, 0.807, tmp+P-Source.Feed.conc.+Nitrogen.Conc.+Lys_conc. +X.F.Suc+X.VXdt+total.Vol+emission_O2+rab+Thr+lactate+PO4.f erm.; 0.537, 0.807, pH+tmp+Nitrogen.Conc.+Lys_conc.+X.VXdt+ total.Vol+yield+PL+emission_O2+rab+Thr+lactate+SO4.ferm.+PO 4.ferm.+PO4.apporte+PO4.utilise; 0.595, 0.807, pH+tmp+Nitro gen.Conc.+Lys_conc.+X.VXdt+total.Vol+emission_O2+rab+PO4.fe rm.; 0.564, 0.807, pH+tmp+Main.Thr.Conc.+Nitrogen.Conc.+Lys _conc.+X.VXdt+total.Vol+total.cell+emission_O2+rab+His+lact ate+PO4.ferm.; 0.602, 0.807, pH+tmp+Nitrogen.Conc.+Lys_conc. +X.VXdt+total.Vol+lactate+PO4.ferm.; 0.588, 0.807, pH+tmp+N itrogen.Conc.+Lys_conc.+X.F.Suc+X.VXdt+total.Vol+His+lactat e+PO4.ferm.; 0.588, 0.807, tmp+OD+Lys_conc.+X.VXdt+total.ce ll+Thr+His+lactate+SO4.ferm.+PO4.ferm.; 0.572, 0.807, pH+tm p+Main.Thr.Conc.+Nitrogen.Conc.+OD+Lys_conc.+X.VXdt+total.c ell+emission_O2+rab+Thr+PO4.ferm.; 0.555, 0.807, Main.Thr.C onc.+Nitrogen.Conc.+Lys_conc.+X.VXdt+total.Vol+yield+emissi on_O2+rab+Thr+lactate+SO4.ferm.+PO4.ferm.+PO4.apporte+PO4.u tilise; 0.564, 0.807, tmp+P-Source.Feed.conc.+Nitrogen.Conc. +OD+Lys_conc.+X.VXdt+total.Vol+emission_O2+rab+Thr+His+lact ate+PO4.ferm.; 0.564, 0.807, pH+P-Source.Feed.conc.+Nitroge n.Conc.+Lys_conc.+X.VXdt+total.Vol+yield+emission_O2+rab+Th r+lactate+PO4.ferm.+PO4.apporte; 0.555, 0.807, pH+tmp+P-Sou rce.Feed.conc.+Main.Thr.Conc.+Nitrogen.Conc.+Lys_conc.+X.VX dt+total.Vol+total.cell+emission_O2+rab+Thr+lactate+PO4.fer m.; 0.547, 0.807, tmp+P-Source.Feed.conc.+Nitrogen.Conc.+Ly s_conc.+X.VXdt+total.Vol+yield+PL+emission_O2+rab+Thr+lacta te+PO4.ferm.+PO4.apporte+PO4.utilise; 0.580, 0.807, tmp+OD+ Lys_conc.+X.VXdt+total.Vol+PL+emission_O2+rab+Thr+lactate+P O4.ferm.; 0.555, 0.807, pH+tmp+Main.Thr.Conc.+Nitrogen.Conc. +Lys_conc.+X.VXdt+total.Vol+PL+emission_O2+rab+Thr+lactate+ PO4.ferm.+PO4.utilise; 0.588, 0.807, pH+tmp+Nitrogen.Conc.+ Lys_conc.+X.VXdt+total.Vol+emission_O2+rab+His+lactate; 0.5 55, 0.807, pH+tmp+P-Source.Feed.conc.+Main.Thr.Conc.+Nitrog en.Conc.+Lys_conc.+X.VXdt+total.Vol+yield+emission_O2+rab+H is+lactate+PO4.ferm.; 0.572, 0.807, tmp+Nitrogen.Conc.+OD+L ys_conc.+X.F.Suc+X.VXdt+total.Vol+emission_O2+rab+Thr+lacta te+PO4.ferm.; 0.555, 0.807, tmp+P-Source.Feed.conc.+Main.Th r.Conc.+Nitrogen.Conc.+Lys_conc.+X.VXdt+total.Vol+yield+emi ssion_O2+rab+Thr+lactate+PO4.ferm.+PO4.apporte; 0.572, 0.80 7, pH+tmp+Main.P-Source.Conc.+Nitrogen.Conc.+Lys_conc.+X.VX dt+total.Vol+PL+emission_O2+rab+lactate+PO4.ferm.; 0.546, 0. 807, pH+tmp+P-Source.Feed.conc.+Nitrogen.Conc.+OD+Lys_conc. +X.F.Suc+X.VXdt+total.Vol+total.cell+emission_O2+rab+Thr+la ctate+PO4.ferm.; 0.572, 0.807, pH+tmp+Nitrogen.Conc.+Lys_co nc.+X.VXdt+total.Vol+PL+emission_O2+rab+lactate+SO4.ferm.+P O4.ferm.; 0.546, 0.807, pH+tmp+Main.P-Source.Conc.+Nitrogen. Conc.+Lys_conc.+X.VXdt+total.Vol+total.cell+PL+emission_O2+ rab+Thr+His+lactate+PO4.ferm.; 0.602, 0.807, tmp+P-Source.F eed.conc.+Nitrogen.Conc.+Lys_conc.+X.VXdt+total.Vol+lactate +PO4.ferm.; 0.595, 0.807, tmp+Main.P-Source.Conc.+OD+Lys_co nc.+X.VXdt+total.cell+Thr+lactate+PO4.ferm.; 0.546, 0.807, pH+tmp+Main.P-Source.Conc.+P-Source.Feed.conc.+Nitrogen.Con c.+OD+Lys_conc.+X.VXdt+total.Vol+total.cell+emission_O2+rab +Thr+lactate+PO4.ferm.; 0.564, 0.807, pH+tmp+Nitrogen.Conc. +Lys_conc.+X.F.Suc+X.VXdt+total.Vol+emission_O2+rab+His+lac tate+SO4.ferm.+PO4.ferm.; 0.572, 0.807, P-Source.Feed.conc. +Nitrogen.Conc.+OD+Lys_conc.+X.VXdt+total.cell+emission_O2+ rab+Thr+His+lactate+PO4.ferm.; 0.564, 0.807, pH+tmp+Nitroge n.Conc.+OD+Lys_conc.+X.F.Suc+X.VXdt+total.cell+emission_O2+ rab+Thr+His+PO4.ferm.; 0.555, 0.807, tmp+Main.Thr.Conc.+Nit rogen.Conc.+Lys_conc.+X.VXdt+total.Vol+yield+PL+emission_O2 +Thr+lactate+PO4.ferm.+PO4.apporte+PO4.utilise; 0.564, 0.80 7, tmp+Nitrogen.Conc.+OD+Lys_onc.+X.VXdt+total.cell+emissi on_O2+rab+Thr+His+lactate+SO4.ferm.+PO4.ferm.; 0.572, 0.807, tmp+P-Source.Feed.conc.+Nitrogen.Conc.+Lys_conc.+X.VXdt+to tal.Vol+emission_O2+rab+Thr+lactate+SO4.ferm.+PO4.ferm.; 0. 564, 0.807, pH+tmp+Main.Thr.Conc.+Nitrogen.Conc.+OD+Lys_con c.+X.F.Suc+X.VXdt+total.cell+emission_2+rab+lactate+PO4.fe rm.; 0.572, 0.807, tmp+P-Source.Feed.conc.+Nitrogen.Conc.+L ys_conc.+X.VXdt+total.Vol+emission_O2+rab+Thr+lactate+PO4.f erm.+PO4.utilise; 0.595, 0.807, tmp+Main.Thr.Conc.+OD+Lys_c onc.+X.VXdt+total.cell+Thr+His+PO4.ferm.; 0.580, 0.807, tmp +P-Source.Feed.conc.+Nitrogen.Conc.+Lys_conc.+X.F.Suc+X.VXd t+total.Vol+emission_O2+rab+lactate+PO4.ferm.; 0.564, 0.807, Nitrogen.Conc.+Lys_conc.+X.F.Suc+X.VXdt+total.Vol+yield+PL +emission_O2+rab+Thr+lactate+PO4.ferm.+PO4.apporte; 0.572, 0.807, Nitrogen.Conc.+Lys_conc.+X.VXdt+total.Vol+yield+emis sion_O2+rab+Thr+His+lactate+PO4.ferm.+PO4.utilise; 0.546, 0. 807, pH+tmp+Main.Thr.Conc.+Nitrogen.Conc.+Lys_conc.+X.VXdt+ total.Vol+PL+emission_O2+rab+Thr+lactate+PO4.ferm.+PO4.appo rte+PO4.utilise; 0.587, 0.807, tmp+Main.Thr.Conc.+OD+Lys_co nc.+X.VXdt+total.cell+emission_O2+rab+Thr+PO4.ferm.; 0.572, 0.807, tmp+Main.Thr.Conc.+Nitrogen.Conc.+OD+Lys_conc.+X.F. Suc+X.VXdt+total.cell+PL+Thr+lactate+PO4.ferm.; 0.555, 0.80 7, pH+tmp+Main.P-Source.Conc.+P-Source.Feed.conc.+Nitrogen. Conc.+Lys_conc.+X.VXdt+total.Vol+total.cell+emission_O2+rab +Thr+lactate+PO4.ferm.; 0.595, 0.807, tmp+Nitrogen.Conc.+Ly s_conc.+X.F.Suc+X.VXdt+total.Vol+His+lactate+PO.ferm.; 0.5 72, 0.807, tmp+Main.P-Source.Conc.+Nitrogen.Conc.+OD+Lys_co nc.+X.VXdt+total.Vol+emission_O2+rab+Thr+lactate+PO4.ferm.; 0.587, 0.807, pH+tmp+Main.Thr.Conc.+OD+Lys_conc.+X.VXdt+to tal.cell+Thr+lactate+PO4.ferm.; 0.563, 0.807, pH+Nitrogen.C onc.+Lys_conc.+X.F.Suc+X.VXdt+total.Vol+yield+emission_O2+r ab+Thr+lactate+PO4.ferm.+PO4.apporte; 0.587, 0.807, pH+tmp+ OD+Lys_conc.+X.F.Suc+X.VXdt+total.cell+emission_O2+rab+PO4. ferm.; 0.563, 0.807, pH+tmp+Main.Thr.Conc.+OD+Lys_conc.+X.V Xdt+total.Vol+total.cell+emission_O2+rab+Thr+lactate+PO4.fe rm.; 0.563, 0.807, pH+tmp+P-Source.Feed.conc.+Nitrogen.Conc. +Lys_conc.+X.VXdt+total.Vol+total.cell+PL+emission_O2+rab+l actate+PO4.ferm.; 0.555, 0.807, pH+tmp+Nitrogen.Conc.+OD+Ly s_conc.+X.F.Suc+X.VXdt+total.Vol+emission_O2+rab+Thr+His+la ctate+PO4.ferm.; 0.555, 0.807, pH+tmp+Nitrogen.Conc.+OD+Lys _conc.+X.VXdt+total.Vol+emission_O2+rab+Thr+His+lactate+SO4. ferm.+PO4.ferm.; 0.546, 0.807, pH+tmp+Main.Thr.Conc.+Nitrog en.Conc.+OD+Lys_conc.+X.VXdt+total.Vol+PL+emission_O2+rab+T hr+His+lactate+PO4.ferm.; 0.587, 0.807, pH+tmp+Main.Thr.Con c.+Lys_conc.+X.VXdt+total.Vol+emission_O2+rab+lactate+PO4.f erm.; 0.572, 0.807, Nitrogen.Conc.+Lys_conc.+X.VXdt+total.V ol+yield+PL+emission_O2+rab+Thr+His+lactate+PO4.utilise; 0. 580, 0.807, tmp+Nitrogen.Conc.+Lys_conc.+X.F.Suc+X.VXdt+tot al.Vol+emission_O2+rab+His+lactate+PO4.ferm.; 0.555, 0.807, pH+tmp+Nitrogen.Conc.+Lys_conc.+X.F.Suc+X.VXdt+total.Vol+t otal.cell+emission_O2+rab+Thr+lactate+SO4.ferm.+PO4.ferm.; 0.563, 0.807, pH+tmp+Main.P-Source.Conc.+Nitrogen.Conc.+OD+ Lys_conc.+X.VXdt+total.Vol+emission_O2+rab+His+lactate+PO4. ferm.; 0.563, 0.807, pH+tmp+Main.Thr.Conc.+Nitrogen.Conc.+L ys_conc.+X.VXdt+total.Vol+emission_O2+rab+Thr+lactate+PO4.f erm.+PO4.utilise; 0.587, 0.807, tmp+Main.Thr.Conc.+Nitrogen. Conc.+OD+Lys_conc.+X.VXdt+total.cell+PL+Thr+PO4.ferm.; 0.56 3, 0.807, pH+tmp+Main.P-Source.Conc.+P-Source.Feed.conc.+Ni trogen.Conc.+Lys_conc.+X.F.Suc+X.VXdt+total.Vol+emission_O2 +rab+lactate+PO4.ferm.; 0.563, 0.807, pH+tmp+P-Source.Feed. conc.+Nitrogen.Conc.+Lys_conc.+X.VXdt+total.Vol+emission_O2 +rab+Thr+lactate+PO4.ferm.+PO4.apporte; 0.555, 0.807, tmp+M ain.Thr.Conc.+Nitrogen.Conc.+Lys_conc.+X.VXdt+total.Vol+yie ld+emission_O2+rab+Thr+His+lactate+PO4.ferm.+PO4.apporte; 0. 594, 0.807, tmp+OD+Lys_conc.+X.VXdt+total.cell+PL+Thr+SO4.f erm.+PO4.ferm.; 0.571, 0.807, Main.Thr.Conc.+Nitrogen.Conc. +Lys_conc.+X.VXdt+total.Vol+yield+emission_O2+rab+Thr+His+l actate+PO4.utilise; 0.563, 0.807, tmp+Nitrogen.Conc.+Lys_co nc.+X.VXdt+total.Vol+yield+PL+emission_O2+rab+Thr+PO4.ferm. +PO4.apporte+PO4.utilise; 0.563, 0.807, pH+tmp+Main.P-Sourc e.Conc.+Main.Thr.Conc.+OD+Lys_conc.+X.VXdt+total.cell+emiss ion_O2+rab+Thr+lactate+PO4.ferm.; 0.602, 0.807, tmp+Nitroge n.Conc.+OD+Lys_conc.+X.F.Suc+X.VXdt+total.cell+PO4.ferm.; 0. 587, 0.807, tmp+Main.P-Source.Conc.+OD+Lys_conc.+X.VXdt+tot al.cell+PL+Thr+lactate+PO4.ferm.; 0.563, 0.807, pH+tmp+Main. Thr.Conc.+OD+Lys_conc.+X.F.Suc+X.VXdt+total.cell+emission_O 2+rab+Thr+lactate+PO4.ferm.; 0.555, 0.807, pH+tmp+Main.Thr. Conc.+Nitrogen.Conc.+Lys_conc.+X.VXdt+total.Vol+yield+emiss ion_O2+rab+Thr+lactate+PO4.ferm.+PO4.apporte; 0.594, 0.807, tmp+OD+Lys_conc.+X.VXdt+total.cell+PL+emission_O2+Thr+PO4. ferm.; 0.555, 0.807, pH+tmp+Main.P-Source.Conc.+P-Source.Fe ed.conc.+Nitrogen.Conc.+Lys_conc.+X.VXdt+total.Vol+emission _O2+rab+Thr+His+lactate+PO4.ferm.; 0.555, 0.807, tmp+Main.T hr.Conc.+Nitrogen.Conc.+Lys_conc.+X.VXdt+total.Vol+yield+PL +emission_O2+rab+Thr+lactate+PO4.ferm.+PO4.apporte; 0.579, 0.807, pH+tmp+Main.Thr.Conc.+OD+Lys_conc.+X.VXdt+total.cell +PL+Thr+lactate+PO4.ferm.; 0.555, 0.807, pH+tmp+Nitrogen.Co nc.+OD+Lys_conc.+X.F.Suc+X.VXdt+total.Vol+PL+emission_O2+ra b+Thr+lactate+PO4.ferm.; 0.546, 0.807, pH+tmp+Main.Thr.Conc. +Nitrogen.Conc.+Lys_conc.+X.VXdt+total.Vol+yield+emission_O 2+rab+Thr+lactate+PO4.ferm.+PO4.apporte+PO4.utilise; 0.571, 0.807, tmp+Main.Thr.Conc.+Nitrogen.Conc.+OD+Lys_conc.+X.VX dt+total.cell+Thr+His+lactate+SO4.ferm.+PO4.ferm.; 0.563, 0. 807, pH+tmp+Main.P-Source.Conc.+Nitrogen.Conc.+Lys_conc.+X. F.Suc+X.VXdt+total.Vol+emission_O2+rab+Thr+lactate+PO4.fer m.; 0.555, 0.807, pH+tmp+OD+Lys_conc.+X.VXdt+total.cell+PL+ emission_O2+rab+Thr+His+lactate+SO4.ferm.+PO4.ferm.; 0.555, 0.807, tmp+Nitrogen.Conc.+OD+Lys_conc.+X.F.Suc+X.VXdt+tota l.Vol+total.cell+emission_O2+rab+Thr+His+lactate+PO4.ferm.; 0.563, 0.806, tmp+Nitrogen.Conc.+Lys_conc.+X.VXdt+total.Vo l+total.cell+PL+emission_O2+rab+Thr+lactate+SO4.ferm.+PO4.f erm.; 0.546, 0.806, pH+tmp+Main.P-Source.Conc.+Nitrogen.Con c.+OD+Lys_conc.+X.F.Suc+X.VXdt+total.cell+PL+emission_O2+ra b+Thr+lactate+PO4.ferm.; 0.571, 0.806, tmp+Nitrogen.Conc.+O D+Lys_conc.+X.VXdt+total.Vol+emission_O2+rab+Thr+lactate+SO 4.ferm.+PO4.ferm.; 0.571, 0.806, pH+tmp+Main.Thr.Conc.+Lys_ conc.+X.VXdt+total.Vol+total.cell+emission_O2+rab+Thr+lacta te+PO4.ferm.; 0.563, 0.806, pH+tmp+Nitrogen.Conc.+OD+Lys_co nc.+X.VXdt+total.Vol+total.cell+PL+emission_O2+rab+Thr+PO4. ferm.; 0.587, 0.806, pH+tmp+Nitrogen.Conc.+OD+Lys_conc.+X.V Xdt+total.cell+emission_O2+rab+PO4.ferm.; 0.587, 0.806, tmp +Lys_conc.+X.VXdt+total.Vol+PL+emission_O2+rab+Thr+lactate+ PO4.ferm.; 0.608, 0.806, tmp+OD+Lys_conc.+X.F.Suc+X.VXdt+to tal.cell+PO4.ferm.; 0.545, 0.806, tmp+Nitrogen.Conc.+Lys_co nc.+X.F.Suc+X.VXdt+total.Vol+yield+PL+emission_O2+rab+Thr+l actate+PO4.ferm.+PO4.apporte+PO4.utilise; 0.587, 0.806, tmp +Main.Thr.Conc.+OD+Lys_conc.+X.VXdt+total.cell+PL+Thr+His+P O4.ferm.; 0.563, 0.806, pH+tmp+Main.Thr.Conc.+OD+Lys_conc.+ X.VXdt+total.Vol+PL+emission_O2+rab+Thr+lactate+PO4.ferm.; 0.554, 0.806, pH+tmp+Main.P-Source.Conc.+P-Source.Feed.conc. +Nitrogen.Conc.+Lys_conc.+X.VXdt+total.Vol+total.cell+emiss ion_O2+rab+His+lactate+PO4.ferm.; 0.554, 0.806, pH+tmp+Main. P-Source.Conc.+Main.Thr.Conc.+Nitrogen.Conc.+Lys_conc.+X.VX dt+total.Vol+total.cell+emission_O2+rab+Thr+lactate+PO4.fer m.; 0.571, 0.806, pH+tmp+Main.P-Source.Conc.+Nitrogen.Conc. +OD+Lys_conc.+X.VXdt+total.cell+emission_O2+rab+Thr+PO4.fer m.; 0.563, 0.806, tmp+Nitrogen.Conc.+Lys_conc.+X.VXdt+total. Vol+yield+emission_O2+rab+His+lactate+PO4.ferm.+PO4.apporte +PO4.utilise; 0.563, 0.806, tmp+Nitrogen.Conc.+Lys_conc.+X. VXdt+total.Vol+yield+PL+emission_O2+Thr+lactate+PO4.ferm.+P O4.apporte+PO4.utilise; 0.571, 0.806, pH+tmp+Main.Thr.Conc. +Nitrogen.Conc.+OD+Lys_conc.+X.VXdt+total.cell+PL+Thr+lacta te+PO4.ferm.; 0.563, 0.806, P-Source.Feed.conc.+Nitrogen.Co nc.+Lys_conc.+X.F.Suc+X.VXdt+total.Vol+yield+emission_O2+ra b+Thr+lactate+PO4.ferm.+PO4.apporte; 0.545, 0.806, Main.Thr. Conc.+Nitrogen.Conc.+Lys_conc.+X.F.Suc+X.VXdt+total.Vol+yie ld+PL+emission_O2+rab+Thr+lactate+PO4.ferm.+PO4.apporte+PO4. utilise; 0.563, 0.806, tmp+Nitrogen.Conc.+OD+Lys_conc.+X.VX dt+total.Vol+PL+emission_O2+rab+Thr+His+lactate+PO4.ferm.; 0.563, 0.806, pH+tmp+Nitrogen.Conc.+Lys_conc.+X.F.Suc+X.VXd t+total.Vol+emission_O2+rab+Thr+lactate+SO4.ferm.+PO4.fer m.; 0.563, 0.806, pH+tmp+P-Source.Feed.conc.+Nitrogen.Conc. +Lys_conc.+X.VXdt+total.Vol+emission_O2+rab+His+lactate+SO4. ferm.+PO4.ferm.; 0.545, 0.806, pH+tmp+Main.Thr.Conc.+Nitrog en.Conc.+OD+Lys_conc.+X.F.Suc+X.VXdt+total.cell+emission_O2 +rab+Thr+lactate+SO4.ferm.+PO4.ferm.; 0.594, 0.806, pH+tmp+ Lys_conc.+X.VXdt+total.Vol+emission_O2+rab+lactate+PO4.fer m.; 0.563, 0.806, pH+tmp+P-Source.Feed.conc.+Nitrogen.Conc. +OD+Lys_conc.+X.F.Suc+X.VXdt+total.Vol+emission_O2+rab+lact ate+PO4.ferm.; 0.545, 0.806, pH+tmp+P-Source.Feed.conc.+Nit rogen.Conc.+OD+Lys_conc.+X.VXdt+total.Vol+total.cell+emissi on_O2+rab+Thr+lactate+SO4.ferm.+PO4.ferm.; 0.563, 0.806, pH +tmp+P-Source.Feed.conc.+Main.Thr.Conc.+Nitrogen.Conc.+Lys_ conc.+X.VXdt+total.Vol+PL+emission_O2+rab+lactate+PO4.fer m.; 0.545, 0.806, pH+tmp+P-Source.Feed.conc.+Main.Thr.Conc. +Nitrogen.Conc.+OD+Lys_conc.+X.VXdt+total.cell+PL+emission_ O2+rab+Thr+lactate+PO4.ferm.; 0.554, 0.806, pH+tmp+P-Source. Feed.conc.+Nitrogen.Conc.+OD+Lys_conc.+X.VXdt+total.cell+em ission_O2+rab+Thr+lactate+SO4.ferm.+PO4.ferm.; 0.536, 0.806, tmp+Main.Thr.Conc.+Nitrogen.Conc.+Lys_conc.+X.VXdt+total.V ol+yield+PL+emission_O2+rab+Thr+lactate+SO4.ferm.+PO4.ferm. +PO4.apporte+PO4.utilise; 0.562, 0.806, pH+tmp+Main.Thr.Con c.+OD+Lys_conc.+X.VXdt+total.cell+emission_O2+rab+Thr+lacta te+SO4.ferm.+PO4.ferm.; 0.601, 0.806, pH+tmp+OD+Lys_conc.+X. VXdt+total.cell+Thr+PO4.ferm.; 0.562, 0.806, tmp+Main.Thr.C onc.+Nitrogen.Conc.+OD+Lys_conc.+X.VXdt+total.cell+emission _O2+rab+Thr+lactate+SO4.ferm.+PO4.ferm.; 0.554, 0.806, pH+t mp+P-Source.Feed.conc.+Main.Thr.Conc.+Nitrogen.Conc.+Lys_co nc.+X.VXdt+total.Vol+yield+emission_O2+rab+Thr+lactate+PO4. ferm.; 0.586, 0.806, tmp+P-Source.Feed.conc.+OD+Lys_conc.+X. VXdt+total.cell+emission_O2+rab+Thr+PO4.ferm.; 0.562, 0.806, pH+tmp+Nitrogen.Conc.+Lys_conc.+X.VXdt+total.Vol+PL+emissi on_O2+rab+His+lactate+SO4.ferm.+PO4.ferm.; 0.545, 0.806, pH +tmp+Main.Thr.Conc.+Nitrogen.Conc.+Lys_conc.+X.VXdt+total.V ol+yield+PL+emission_O2+rab+Thr+lactate+PO4.ferm.+PO4.appor te; 0.554, 0.806, pH+Nitrogen.Conc.+Lys_conc.+X.F.Suc+X.VXd t+total.Vol+yield+emission_O2+rab+Thr+lactate+PO4.ferm.+PO4. apporte+PO4.utilise; 0.554, 0.806, P-Source.Feed.conc.+Nitr ogen.Conc.+Lys_conc.+X.VXdt+total.Vol+yield+PL+emission_O2+ rab+Thr+His+lactate+PO4.ferm.+PO4.apporte; 0.579, 0.806, tm p+Main.Thr.Conc.+Lys_conc.+X.VXdt+total.Vol+total.cell+emis sion_O2+rab+Thr+lactate+PO4.ferm.; 0.562, 0.806, pH+tmp+Nit rogen.Conc.+Lys_conc.+X.F.Suc+X.VXdt+total.Vol+emission_O2+ rab+Thr+lactate+PO4.ferm.+PO4.apporte; 0.594, 0.806, pH+tmp +Nitrogen.Conc.+OD+Lys_conc.+X.F.Suc+X.VXdt+total.cell+PO4. ferm.; 0.554, 0.806, pH+tmp+P-Source.Feed.conc.+Nitrogen.Co nc.+OD+Lys_conc.+X.F.Suc+X.VXdt+total.Vol+total.cell+emissi on_O2+rab+lactate+PO4.ferm.; 0.579, 0.806, tmp+Nitrogen.Con c.+Lys_conc.+X.F.Suc+X.VXdt+total.Vol+total.cell+emission_O 2+rab+lactate+PO4.ferm.; 0.601, 0.806, tmp+Nitrogen.Conc.+O D+Lys_conc.+X.VXdt+total.cell+lactate+PO4.ferm.; 0.571, 0.8 06, pH+tmp+P-Source.Feed.conc.+Nitrogen.Conc.+Lys_conc.+X.V Xdt+total.Vol+emission_O2+rab+lactate+PO4.ferm.+PO4.apport e; 0.562, 0.806, pH+Nitrogen.Conc.+Lys_conc.+X.VXdt+total.V ol+yield+emission_O2+rab+Thr+His+lactate+PO4.ferm.+PO4.appo rte; 0.554, 0.806, pH+tmp+Main.P-Source.Conc.+P-Source.Feed. conc.+Main.Thr.Conc.+Nitrogen.Conc.+Lys_conc.+X.VXdt+total. Vol+emission_O2+rab+Thr+lactate+PO4.ferm.; 0.562, 0.806, pH +tmp+Main.Thr.Conc.+Nitrogen.Conc.+OD+Lys_conc.+X.VXdt+tota l.Vol+emission_O2+rab+His+lactate+PO4.ferm.; 0.554, 0.806, pH+tmp+P-Source.Feed.conc.+Nitrogen.Conc.+Lys_conc.+X.VXdt+ total.Vol+PL+emission_O2+rab+Thr+lactate+PO4.ferm.+PO4.appo rte; 0.562, 0.806, pH+tmp+Main.Thr.Conc.+Nitrogen.Conc.+Lys conc.+X.VXdt+total.Vol+PL+emission_O2+rab+His+lactate+PO4. ferm.; 0.554, 0.806, P-Source.Feed.conc.+Main.Thr.Conc.+Nit rogen.Conc.+Lys_conc.+X.VXdt+total.Vol+yield+emission_O2+ra b+Thr+His+lactate+PO4.ferm.+PO4.apporte; 0.562, 0.806, tmp+ Main.Thr.Conc.+Nitrogen.Conc.+Lys_conc.+X.VXdt+total.Vol+to tal.cell+PL+emission_O2+rab+Thr+lactate+PO4.ferm.; 0.562, 0. 806, pH+tmp+P-Source.Feed.conc.+Nitrogen.Conc.+OD+Lys_conc. +X.VXdt+total.Vol+PL+emission_O2+rab+lactate+PO4.ferm.; 0.5 70, 0.806, Nitrogen.Cone.+Lys_conc.+X.F.Suc+X.VXdt+total.Vo l+yield+emission_O2+rab+Thr+lactate+PO4.ferm.+PO4.utilise; 0.601, 0.806, tmp+Nitrogen.Conc.+Lys_conc.+X.VXdt+total.Vol +His+lactate+PO4.ferm.; 0.554, 0.806, pH+tmp+Main.Thr.Conc. +Nitrogen.Conc.+OD+Lys_conc.+X.VXdt+total.Vol+emission_O2+r ab+Thr+lactate+SO4.ferm.+PO4.ferm.; 0.562, 0.806, tmp+Nitro gen.Cone.+Lys_conc.+X.F.Suc+X.VXdt+total.Vol+yield+emission _O2+rab+Thr+lactate+PO4.ferm.+PO4.apporte; 0.570, 0.806, pH +tmp+P-Source.Feed.conc.+OD+Lys_conc.+X.VXdt+total.cell+PL+ emission_O2+rab+Thr+PO4.ferm.; 0.578, 0.806, pH+tmp+Nitroge n.Conc.+Lys_conc.+X.VXdt+total.Vol+emission_O2+rab+lactate+ PO4.ferm.+PO4.utilise; 0.570, 0.806, tmp+Lys_conc.+X.VXdt+t otal.Vol+PL+emission_O2+rab+Thr+lactate+PO4.ferm.+PO4.appor te+PO4.utilise; 0.578, 0.806, tmp+Main.P-Source.Conc.+Nitro gen.Conc.+OD+Lys_conc.+X.VXdt+total.cell+PL+Thr+lactate+PO4. ferm.; 0.570, 0.806, pH+tmp+P-Source.Feed.conc.+Nitrogen.Co nc.+OD+Lys_conc.+X.VXdt+total.cell+emission_O2+rab+Thr+PO4. ferm.; 0.553, 0.806, tmp+P-Source.Feed.conc.+Main.Thr.Conc. +Nitrogen.Conc.+OD+Lys_conc.+X.VXdt+total.Vol+total.cell+em ission_O2+rab+Thr+lactate+PO4.ferm.; 0.570, 0.806, pH+tmp+O D+Lys_conc.+X.F.Suc+X.VXdt+total.cell+emission_O2+rab+Thr+H is+PO4.ferm.; 0.545, 0.806, pH+tmp+Main.P-Source.Conc.+Nitr ogen.Conc.+OD+Lys_conc.+X.VXdt+total.Vol+total.cell+emissio n_O2+rab+Thr+His+lactate+PO4.ferm.; 0.562, 0.806, tmp+P-Sou rce.Feed.conc.+Main.Thr.Conc.+Nitrogen.Conc.+Lys_conc.+X.VX dt+total.Vol+emission_O2+rab+Thr+His+lactate+PO4.ferm.; 0.5 62, 0.806, tmp+P-Source.Feed.conc.+Main.Thr.Conc.+Nitrogen. Conc.+Lys_conc.+X.VXdt+total.Vol+total.cell+emission_O2+rab +Thr+lactate+PO4.ferm.; 0.562, 0.806, pH+tmp+Main.P-Source. Conc.+Nitrogen.Conc.+OD+Lys_conc.+X.VXdt+total.cell+PL+emis sion_O2+rab+Thr+PO4.ferm.; 0.562, 0.806, tmp+Nitrogen.Conc. +Lys_conc.+X.VXdt+total.Vol+PL+emission_O2+rab+Thr+His+lact ate+PO4.ferm.+PO4.apporte; 0.570, 0.806, pH+tmp+Nitrogen.Co nc.+Lys_conc.+X.VXdt+total.Vol+emission_O2+rab+His+lactate+ PO4.ferm.+PO4.apporte; 0.553, 0.806, pH+tmp+Nitrogen.Conc.+ Lys_conc.+X.VXdt+total.Vol+yield+PL+emission_O2+rab+Thr+lac tate+PO4.ferm.+PO4.utilise; 0.570, 0.806, tmp+Main.Thr.Conc. +Nitrogen.Conc.+Lys_conc.+X.VXdt+total.Vol+PL+emission_O2+r ab+Thr+lactate+PO4.ferm.; 0.570, 0.806, Nitrogen.Cone.+Lys_ conc.+X.F.Suc+X.VXdt+total.Vol+yield+emission_O2+rab+Thr+Hi s+lactate+PO4.utilise; 0.553, 0.806, tmp+P-Source.Feed.conc. +Nitrogen.Conc.+OD+Lys_conc.+X.F.Suc+X.VXdt+total.cell+emis sion_O2+rab+Thr+His+lactate+PO4.ferm.; 0.586, 0.806, tmp+Ma in.Thr.Conc.+Lys_conc.+X.VXdt+total.Vol+emission_O2+rab+Thr +lactate+PO4.ferm.; 0.586, 0.806, pH+tmp+Lys_conc.+X.VXdt+t otal.Vol+PL+emission_O2+rab+Thr+PO4.ferm.; 0.553, 0.806, tm p+Main.Thr.Conc.+Nitrogen.Conc.+Lys_conc.+X.VXdt+total.Vol+ PL+emission_O2+rab+Thr+lactate+PO4.ferm.+PO4.apporte+PO4.ut ilise; 0.544, 0.806, tmp+Main.Thr.Conc.+Nitrogen.Conc.+Lys_ conc.+X.VXdt+total.Vol+yield+emission_O2+rab+Thr+lactate+SO 4.ferm.+PO4.ferm.+PO4.apporte+PO4.utilise; 0.553, 0.806, pH +tmp+Main.Thr.Conc.+Nitrogen.Conc.+OD+Lys_conc.+X.VXdt+tota l.Vol+emission_O2+rab+Thr+His+lactate+PO4.ferm.; 0.562, 0.8 06, pH+tmp+Main.P-Source.Conc.+Nitrogen.Conc.+OD+Lys_conc.+ X.F.Suc+X.VXdt+total.Vol+emission_O2+rab+lactate+PO4.ferm.; 0.544, 0.806, pH+Nitrogen.Conc.+Lys_conc.+X.VXdt+total.Vol +yield+PL+emission_O2+rab+Thr+His+lactate+PO4.ferm.+PO4.app orte+PO4.utilise; 0.562, 0.806, pH+tmp+Nitrogen.Conc.+OD+Ly s_conc.+X.VXdt+total.cell+PL+emission_O2+rab+Thr+SO4.ferm.+ PO4.ferm.; 0.553, 0.806, pH+tmp+P-Source.Feed.conc.+Nitroge n.Conc.+Lys_conc.+X.VXdt+total.Vol+total.cell+emission_O2+r ab+Thr+lactate+SO4.ferm.+PO4.ferm.; 0.586, 0.806, tmp+OD+Ly s_conc.+X.VXdt+total.Vol+emission_O2+rab+Thr+lactate+PO4.fe rm.; 0.570, 0.806, tmp+Main.P-Source.Conc.+Main.Thr.Conc.+O D+Lys_conc.+X.VXdt+total.cell+emission_O2+rab+Thr+lactate+P O4.ferm.; 0.553, 0.806, tmp+Main.P-Source.Conc.+P-Source.Fe ed.conc.+Nitrogen.Conc.+OD+Lys_conc.+X.VXdt+total.cell+emis sion_O2+rab+Thr+His+lactate+PO4.ferm.; 0.562, 0.806, tmp+P-Source.Feed.conc.+Nitrogen.Conc.+Lys_conc.+X.VXdt+total.Vol +PL+emission_O2+rab+Thr+His+lactate+PO4.ferm.; 0.578, 0.806, pH+tmp+OD+Lys_conc.+X.F.Suc+X.VXdt+total.cell+emission_O2+ rab+lactate+PO4.ferm.; 0.553, 0.806, pH+tmp+Nitrogen.Conc.+ Lys_conc.+X.VXdt+total.Vol+emission_O2+rab+Thr+His+lactate+ PO4.ferm.+PO4.apporte+PO4.utilise; 0.578, 0.806, tmp+OD+Lys _conc.+X.F.Suc+X.VXdt+total.cell+PL+Thr+His+lactate+PO4.fer m.; 0.553, 0.806, pH+Nitrogen.Conc.+Lys_conc.+X.VXdt+total. Vol+yield+emission_O2+rab+Thr+His+lactate+PO4.ferm.+PO4.app orte+PO4.utilise; 0.570, 0.806, tmp+Nitrogen.Conc.+Lys_conc. +X.VXdt+total.Vol+yield+emission_O2+rab+Thr+lactate+PO4.app orte+PO4.utilise; 0.585, 0.806, tmp+OD+Lys_conc.+X.VXdt+tot al.cell+PL+rab+Thr+lactate+PO4.ferm.; 0.561, 0.806, tmp+Mai n.Thr.Conc.+Nitrogen.Conc.+Lys_conc.+X.VXdt+total.Vol+yield +emission_O2+rab+Thr+lactate+PO4.ferm.+PO4.utilise; 0.578, 0.806, tmp+Lys_conc.+X.VXdt+total.Vol+total.cell+emission_O 2+rab+Thr+His+lactate+PO4.ferm.; 0.570, 0.806, tmp+Lys_conc. +X.VXdt+total.Vol+total.cell+PL+emission_O2+rab+Thr+His+lac tate+PO4.ferm.; 0.553, 0.806, pH+tmp+Main.P-Source.Conc.+P-Source.Feed.conc.+Main.Thr.Conc.+Nitrogen.Conc.+Lys_conc.+X. VXdt+total.Vol+emission_O2+rab+His+lactate+PO4.ferm.; 0.578, 0.806, tmp+Main.P-Source.Conc.+Nitrogen.Conc.+OD+Lys_conc. +X.VXdt+total.cell+emission_O2+rab+Thr+PO4.ferm.; 0.570, 0. 806, P-Source.Feed.conc.+Nitrogen.Conc.+Lys_conc.+X.VXdt+to tal.Vol+yield+emission_O2+rab+Thr+lactate+PO4.apporte+PO4.u tilise; 0.561, 0.806, tmp+Main.P-Source.Conc.+P-Source.Feed. conc.+Nitrogen.Conc.+Lys_conc.+X.VXdt+total.Vol+PL+emission _O2+rab+Thr+lactate+PO4.ferm.; 0.561, 0.806, tmp+Nitrogen.C onc.+Lys_conc.+X.VXdt+total.Vol+yield+PL+emission_O2+rab+Th r+lactate+PO4.ferm.+PO4.utilise; 0.561, 0.806, tmp+Main.P-S ource.Conc.+P-Source.Feed.conc.+Nitrogen.Conc.+OD+Lys_conc. +X.VXdt+total.Vol+emission_O2+rab+Thr+lactate+PO4.ferm.; 0. 570, 0.806, Nitrogen.Conc.+Lys_conc.+X.VXdt+total.Vol+yield +PL+emission_O2+rab+Thr+lactate+PO4.ferm.+PO4.utilise; 0.55 3, 0.806, pH+tmp+Main.P-Source.Conc.+P-Source.Feed.conc.+Ni trogen.Conc.+OD+Lys_conc.+X.VXdt+total.Vol+emission_O2+rab+ His+lactate+PO4.ferm.; 0.561, 0.806, pH+tmp+Main.Thr.Conc.+ Nitrogen.Conc.+Lys_conc.+X.F.Suc+X.VXdt+total.Vol+emission_ O2+rab+Thr+lactate+PO4.ferm.; 0.561, 0.806, Main.Thr.Conc.+ Nitrogen.Conc.+Lys_conc.+X.VXdt+total.Vol+yield+PL+emission _O2+Thr+lactate+PO4.ferm.+PO4.apporte+PO4.utilise; 0.525, 0. 806, pH+tmp+Main.Thr.Conc.+Nitrogen.Conc.+Lys_conc.+X.VXdt+ total.Vol+yield+PL+emission_O2+rab+Thr+His+lactate+PO4.ferm. +PO4.apporte+PO4.utilise; 0.544, 0.806, pH+tmp+Nitrogen.Con c.+Lys_conc.+X.VXdt+total.Vol+yield+PL+emission_O2+rab+Thr+ His+lactate+PO4.ferm.+PO4.apporte; 0.577, 0.806, tmp+Main.T hr.Conc.+OD+Lys_conc.+X.F.Suc+X.VXdt+total.cell+PL+Thr+lact ate+PO4.ferm.; 0.561, 0.806, pH+P-Source.Feed.conc.+Nitroge n.Conc.+OD+Lys_conc.+X.VXdt+total.cell+emission_O2+rab+Thr+ His+lactate+PO4.ferm.; 0.592, 0.805, tmp+Lys_conc.+X.VXdt+t otal.Vol+emission_O2+rab+Thr+lactate+PO4.ferm.; 0.577, 0.80 5, tmp+Nitrogen.Conc.+OD+Lys_conc.+X.VXdt+total.Vol+total.c ell+PL+Thr+lactate+PO4.ferm.; 0.543, 0.805, P-Source.Feed.c onc.+Nitrogen.Conc.+Lys_conc.+X.VXdt+total.Vol+yield+PL+emi ssion_O2+rab+Thr+His+lactate+PO4.ferm.+PO4.apporte+PO4.util ise; 0.577, 0.805, tmp+Nitrogen.Conc.+Lys_conc.+X.VXdt+tota l.Vol+total.cell+emission_O2+rab+His+lactate+PO4.ferm.; 0.5 52, 0.805, pH+tmp+Main.P-Source.Conc.+Main.Thr.Conc.+OD+Lys _conc.+X.VXdt+total.cell+PL+emission_O2+rab+Thr+lactate+PO4. ferm.; 0.585, 0.805, pH+tmp+Nitrogen.Conc.+OD+Lys_conc.+X.V Xdt+total.cell+PL+Thr+PO4.ferm.; 0.577, 0.805, tmp+P-Source. Feed.conc.+Nitrogen.Conc.+OD+Lys_conc.+X.VXdt+total.cell+em ission_O2+rab+Thr+PO4.ferm.; 0.552, 0.805, tmp+P-Source.Fee d.conc.+Nitrogen.Conc.+Lys_conc.+X.VXdt+total.Vol+yield+PL+ emission_O2+rab+Thr+lactate+PO4.ferm.+PO4.apporte; 0.561, 0. 805, pH+tmp+Main.P-Source.Conc.+P-Source.Feed.conc.+Nitroge n.Conc.+Lys_conc.+X.VXdt+total.Vol+PL+emission_O2+rab+lacta te+PO4.ferm.; 0.561, 0.805, tmp+Nitrogen.Conc.+Lys_conc.+X. VXdt+total.Vol+yield+emission_O2+rab+Thr+His+lactate+PO4.fe rm.+PO4.utilise; 0.569, 0.805, tmp+Nitrogen.Conc.+Lys_conc. +X.VXdt+total.Vol+PL+emission_O2+rab+Thr+lactate+SO4.ferm.+ PO4.ferm.; 0.561, 0.805, P-Source.Feed.conc.+Main.Thr.Conc. +Nitrogen.Conc.+Lys_conc.+X.VXdt+total.Vol+yield+emission_O 2+rab+Thr+lactate+PO4.ferm.+PO4.utilise; 0.552, 0.805, tmp+ Main.P-Source.Conc.+Nitrogen.Conc.+OD+Lys_conc.+X.F.Suc+X.V Xdt+total.cell+emission_O2+rab+Thr+His+lactate+PO4.ferm.; 0. 534, 0.805, pH+tmp+P-Source.Feed.conc.+Main.Thr.Conc.+Nitro gen.Conc.+OD+Lys_conc.+X.VXdt+total.Vol+total.cell+PL+emiss ion_O2+rab+Thr+lactate+PO4.ferm.; 0.606, 0.805, tmp+Nitroge n.Conc.+OD+Lys_conc.+X.VXdt+total.cell+PO4.ferm.; 0.577, 0. 805, tmp+Main.Thr.Conc.+OD+Lys_conc.+X.VXdt+total.cell+Thr+ His+lactate+SO4.ferm.+PO4.ferm.; 0.569, 0.805, tmp+Main.Thr. Conc.+Nitrogen.Conc.+OD+Lys_conc.+X.VXdt+total.cell+PL+emis sion_O2+Thr+lactate+PO4.ferm.; 0.569, 0.805, tmp+Main.Thr.C onc.+OD+Lys_conc.+X.VXdt+total.cell+emission_O2+rab+Thr+lac tate+SO4.ferm.+PO4.ferm.; 0.577, 0.805, tmp+Main.P-Source.C onc.+Nitrogen.Conc.+OD+Lys_conc.+X.VXdt+total.cell+Thr+His+ lactate+PO4.ferm.; 0.569, 0.805, pH+tmp+Nitrogen.Conc.+OD+L ys_conc.+X.F.Suc+X.VXdt+total.cell+PL+Thr+lactate+PO4.fer m.; 0.592, 0.805, tmp+OD+Lys_conc.+X.F.Suc+X.VXdt+total.cel l+Thr+His+PO4.ferm.; 0.599, 0.805, pH+tmp+Lys_conc.+X.VXdt+ total.Vol+emission_O2+rab+PO4.ferm.; 0.577, 0.805, tmp+P-So urce.Feed.conc.+Nitrogen.Conc.+Lys_conc.+X.VXdt+total.Vol+y ield+emission_O2+rab+lactate+PO4.ferm.; 0.561, 0.805, pH+tm p+Main.Thr.Conc.+OD+Lys_conc.+X.VXdt+total.cell+PL+emission _O2+rab+Thr+His+PO4.ferm.; 0.569, 0.805, tmp+Lys_conc.+X.VX dt+total.Vol+yield+PL+emission_O2+rab+Thr+PO4.ferm.+PO4.app orte+PO4.utilise; 0.561, 0.805, tmp+Nitrogen.Conc.+Lys_conc. +X.VXdt+total.Vol+yield+emission_O2+rab+Thr+lactate+SO4.fer m.+PO4.ferm.+PO4.apporte; 0.569, 0.805, pH+tmp+Nitrogen.Con c.+Lys_conc.+X.VXdt+total.Vol+total.cell+emission_O2+rab+la ctate+SO4.ferm.+PO4.ferm.; 0.577, 0.805, tmp+Main.Thr.Conc. +OD+Lys_conc.+X.F.Suc+X.VXdt+total.cell+Thr+His+lactate+PO4. ferm.; 0.561, 0.805, tmp+P-Source.Feed.conc.+Nitrogen.Conc. +Lys_conc.+X.VXdt+total.Vol+yield+emission_O2+rab+Thr+lacta te+PO4.ferm.+PO4.utilise; 0.569, 0.805, tmp+P-Source.Feed.c onc.+Main.Thr.Conc.+Nitrogen.Conc.+Lys_conc.+X.VXdt+total.V ol+emission_O2+rab+His+lactate+PO4.ferm.; 0.552, 0.805, P-S ource.Feed.conc.+Main.Thr.Conc.+Nitrogen.Conc.+Lys_conc.+X. VXdt+total.Vol+yield+emission_O2+rab+Thr+lactate+PO4.ferm.+ PO4.apporte+PO4.utilise; 0.569, 0.805, tmp+Nitrogen.Conc.+O D+Lys_conc.+X.F.Suc+X.VXdt+total.cell+PL+Thr+lactate+SO4.fe rm.+PO4.ferm.; 0.552, 0.805, tmp+Nitrogen.Conc.+OD+Lys_conc. +X.F.Suc+X.VXdt+total.cell+PL+emission_O2+rab+Thr+His+lacta te+PO4.ferm.; 0.561, 0.805, pH+tmp+Main.Thr.Conc.+Nitrogen. Conc.+Lys_conc.+X.VXdt+total.Vol+emission_O2+rab+His+lactat e+SO4.ferm.+PO4.ferm.; 0.585, 0.805, tmp+Main.P-Source.Conc. +Main.Thr.Conc.+OD+Lys_conc.+X.VXdt+total.cell+Thr+lactate+ PO4.ferm.; 0.599, 0.805, tmp+Main.Thr.Conc.+Nitrogen.Conc.+ Lys_conc.+X.VXdt+total.Vol+lactate+PO4.ferm.; 0.552, 0.805, P-Source.Feed.conc.+Main.Thr.Conc.+Nitrogen.Conc.+Lys_conc. +X.VXdt+total.Vol+yield+PL+emission_O2+rab+Thr+lactate+PO4. ferm.+PO4.apporte; 0.552, 0.805, pH+tmp+Main.P-Source.Conc. +Main.Thr.Conc.+Nitrogen.Conc.+Lys_conc.+X.VXdt+total.Vol+P L+emission_O2+rab+Thr+lactate+PO4.ferm.; 0.561, 0.805, tmp+ Nitrogen.Conc.+Lys_conc.+X.F.Suc+X.VXdt+total.Vol+total.cel l+emission_O2+rab+Thr+His+lactate+PO4.ferm.; 0.577, 0.805, pH+tmp+P-Source.Feed.conc.+OD+Lys_conc.+X.VXdt+total.cell+e mission_O2+rab+Thr+PO4.ferm.; 0.561, 0.805, pH+tmp+Main.Thr. Conc.+OD+Lys_conc.+X.VXdt+total.cell+PL+emission_O2+rab+Thr +SO4.ferm.+PO4.ferm.; 0.569, 0.805, tmp+Main.Thr.Conc.+OD+L ys_conc.+X.VXdt+total.Vol+total.cell+emission_O2+rab+Thr+la ctate+PO4.ferm.; 0.569, 0.805, pH+tmp+Nitrogen.Cone.+Lys_co nc.+X.VXdt+total.Vol+emission_O2+rab+lactate+PO4.ferm.+PO4. apporte+PO4.utilise; 0.552, 0.805, P-Source.Feed.conc.+Nitr ogen.Conc.+Lys_conc.+X.VXdt+total.Vol+yield+emission_O2+rab +Thr+His+lactate+PO4.ferm.+PO4.apporte+PO4.utilise; 0.585, 0.805, pH+tmp+OD+Lys_conc.+X.VXdt+total.cell+emission_O2+ra b+lactate+PO4.ferm.; 0.585, 0.805, tmp+Nitrogen.Conc.+OD+Ly s_conc.+X.VXdt+total.cell+rab+Thr+lactate+PO4.ferm.; 0.569, 0.805, Main.Thr.Conc.+Nitrogen.Conc.+Lys_conc.+X.VXdt+tota l.Vol+yield+emission_O2+rab+Thr+lactate+PO4.ferm.+PO4.utili se; 0.561, 0.805, pH+Main.Thr.Conc.+Nitrogen.Conc.+Lys_conc. +X.VXdt+total.Vol+yield+emission_O2+rab+Thr+lactate+PO4.fer m.+PO4.apporte; 0.534, 0.805, pH+tmp+Main.Thr.Conc.+Nitroge n.Conc.+OD+Lys_conc.+X.VXdt+total.cell+PL+emission_O2+rab+T hr+His+lactate+SO4.ferm.+PO4.ferm.; 0.577, 0.805, tmp+Nitro gen.Conc.+OD+Lys_conc.+X.F.Suc+X.VXdt+total.cell+rab+Thr+la ctate+PO4.ferm.; 0.569, 0.805, Main.P-Source.Conc.+P-Source. Feed.conc.+Nitrogen.Conc.+OD+Lys_conc.+X.VXdt+total.cell+em ission_O2+rab+Thr+lactate+PO4.ferm.; 0.534, 0.805, pH+tmp+M ain.P-Source.Conc.+Nitrogen.Conc.+OD+Lys_conc.+X.VXdt+total. Vol+total.cell+PL+emission_O2+rab+Thr+His+lactate+PO4.fer m.; 0.552, 0.805, pH+tmp+Nitrogen.Conc.+Lys_conc.+X.VXdt+to tal.Vol+PL+emission_O2+rab+Thr+lactate+SO4.ferm.+PO4.ferm.+ PO4.apporte; 0.561, 0.805, tmp+P-Source.Feed.conc.+Nitrogen. Conc.+OD+Lys_conc.+X.VXdt+total.cell+emission_O2+rab+Thr+la ctate+SO4.ferm.+PO4.ferm.; 0.552, 0.805, tmp+P-Source.Feed. conc.+Nitrogen.Conc.+Lys_conc.+X.VXdt+total.Vol+yield+emiss ion_O2+rab+Thr+His+lactate+PO4.ferm.+PO4.apporte; 0.599, 0. 805, tmp+OD+Lys_conc.+X.F.Suc+X.VXdt+total.cell+lactate+PO4. ferm.; 0.552, 0.805, tmp+Main.Thr.Conc.+Nitrogen.Conc.+OD+L ys_conc.+X.VXdt+total.Vol+total.cell+PL+emission_O2+rab+Thr +lactate+PO4.ferm.; 0.552, 0.805, pH+tmp+Main.P-Source.Conc. +Nitrogen.Conc.+Lys_conc.+X.F.Suc+X.VXdt+total.Vol+total.ce ll+emission_O2+rab+His+lactate+PO4.ferm.; 0.569, 0.805, pH+ tmp+Main.Thr.Conc.+Nitrogen.Conc.+OD+Lys_conc.+X.F.Suc+X.VX dt+total.cell+His+lactate+PO4.ferm.; 0.569, 0.805, tmp+OD+L ys_conc.+X.VXdt+total.Vol+total.cell+emission_O2+rab+Thr+Hi s+lactate+PO4.ferm.; 0.560, 0.805, tmp+Main.P-Source.Conc.+ Main.Thr.Conc.+Nitrogen.Conc.+OD+Lys_conc.+X.VXdt+total.cel l+emission_O2+rab+Thr+lactate+PO4.ferm.; 0.569, 0.805, pH+t mp+Nitrogen.Conc.+OD+Lys_conc.+X.VXdt+total.cell+PL+Thr+His +lactate+PO4.ferm.; 0.560, 0.805, tmp+Main.P-Source.Conc.+N itrogen.Conc.+Lys_conc.+X.VXdt+total.Vol+total.cell+emissio n_O2+rab+Thr+His+lactate+PO4.ferm.; 0.569, 0.805, pH+tmp+P-Source.Feed.conc.+Nitrogen.Cone.+OD+Lys_conc.+X.VXdt+total. cell+emission_O2+rab+lactate+PO4.ferm.; 0.577, 0.805, tmp+N itrogen.Conc.+Lys_conc.+X.VXdt+total.Vol+yield+emission_O2+ rab+Thr+lactate+PO4.utilise; 0.560, 0.805, tmp+P-Source.Fee d.conc.+Main.Thr.Conc.+Nitrogen.Conc.+Lys_conc.+X.VXdt+tota l.Vol+PL+emission_O2+rab+Thr+lactate+PO4.ferm.; 0.552, 0.80 5, pH+tmp+P-Source.Feed.conc.+Main.Thr.Conc.+Nitrogen.Conc. +Lys_conc.+X.VXdt+total.Vol+total.cell+emission_O2+rab+His+ lactate+PO4.ferm.; 0.569, 0.805, Nitrogen.Conc.+Lys_conc.+X. VXdt+total.Vol+yield+PL+emission_O2+rab+Thr+PO4.ferm.+PO4.a pporte+PO4.utilise; 0.569, 0.805, tmp+Main.P-Source.Conc.+N itrogen.Conc.+Lys_conc.+X.VXdt+total.Vol+emission_O2+rab+Th r+His+lactate+PO4.ferm.; 0.560, 0.805, P-Source.Feed.conc.+ Nitrogen.Conc.+Lys_conc.+X.VXdt+total.Vol+yield+emission_O2 +rab+Thr+His+lactate+PO4.ferm.+PO4.utilise; 0.569, 0.805, t mp+Main.P-Source.Conc.+OD+Lys_conc.+X.VXdt+total.cell+emiss ion_O2+rab+Thr+His+lactate+PO4.ferm.; 0.543, 0.805, pH+tmp+ P-Source.Feed.conc.+Main.Thr.Conc.+Nitrogen.Conc.+Lys_conc. +X.VXdt+total.Vol+total.cell+PL+emission_O2+rab+Thr+lactate +PO4.ferm.; 0.569, 0.805, pH+Nitrogen.Conc.+Lys_conc.+X.VXd t+total.Vol+yield+PL+emission_O2+rab+Thr+lactate+PO4.utilis e; 0.543, 0.805, pH+tmp+Main.P-Source.Conc.+Nitrogen.Conc.+ Lys_conc.+X.VXdt+total.Vol+total.cell+PL+emission_O2+rab+Th r+lactate+SO4.ferm.+PO4.ferm.; 0.569, 0.805, pH+tmp+Nitroge n.Conc.+OD+Lys_conc.+X.F.Suc+X.VXdt+total.cell+emission_O2+ rab+His+PO4.ferm.; 0.552, 0.805, pH+tmp+Main.P-Source.Conc. +Nitrogen.Cone.+Lys_conc.+X.F.Suc+X.VXdt+total.Vol+total.ce ll+emission_O2+rab+Thr+lactate+PO4.ferm.; 0.560, 0.805, pH+ tmp+P-Source.Feed.conc.+OD+Lys_conc.+X.VXdt+total.cell+PL+e mission_O2+rab+Thr+lactate+PO4.ferm.; 0.569, 0.805, tmp+Mai n.Thr.Conc.+OD+Lys_conc.+X.F.Suc+X.VXdt+total.cell+emission _O2+rab+Thr+lactate+PO4.ferm.; 0.584, 0.805, tmp+Main.P-Sou rce.Conc.+Nitrogen.Conc.+Lys_conc.+X.VXdt+total.Vol+emissio n_O2+rab+lactate+PO4.ferm.; 0.552, 0.805, pH+tmp+P-Source.F eed.conc.+Main.Thr.Conc.+Nitrogen.Conc.+Lys_conc.+X.VXdt+to tal.Vol+emission_O2+rab+Thr+lactate+SO4.ferm.+PO4.ferm.; 0. 552, 0.805, pH+tmp+Main.P-Source.Conc.+Nitrogen.Conc.+Lys_c onc.+X.VXdt+total.Vol+PL+emission_O2+rab+Thr+His+lactate+PO 4.ferm.; 0.543, 0.805, pH+tmp+P-Source.Feed.conc.+Main.Thr. Conc.+Nitrogen.Conc.+Lys_conc.+X.VXdt+total.Vol+PL+emission _O2+rab+Thr+His+lactate+PO4.ferm.; 0.560, 0.805, tmp+Nitrog en.Conc.+Lys_conc.+X.F.Suc+X.VXdt+total.Vol+emission_O2+rab +Thr+lactate+PO4.ferm.+PO4.apporte+PO4.utilise; 0.569, 0.80 5, pH+tmp+Main.Thr.Conc.+Nitrogen.Conc.+OD+Lys_conc.+X.VXdt +total.cell+Thr+His+lactate+PO4.ferm.; 0.569, 0.805, pH+Nit rogen.Conc.+Lys_conc.+X.VXdt+total.Vol+yield+emission_O2+ra b+Thr+His+lactate+PO4.utilise; 0.569, 0.805, pH+tmp+Nitroge n.Conc.+OD+Lys_conc.+X.F.Suc+X.VXdt+total.cell+Thr+lactate+ SO4.ferm.+PO4.ferm.; 0.543, 0.805, pH+tmp+Main.P-Source.Con c.+Nitrogen.Conc.+OD+Lys_conc.+X.F.Suc+X.VXdt+total.Vol+tot al.cell+emission_O2+rab+Thr+lactate+PO4.ferm.; 0.533, 0.805, pH+tmp+Nitrogen.Conc.+OD+Lys_conc.+X.F.Suc+X.VXdt+total.Vo l+total.cell+PL+emission_O2+rab+Thr+His+lactate+PO4.ferm.; 0.576, 0.805, pH+tmp+Nitrogen.Conc.+Lys_conc.+X.VXdt+total. Vol+PL+emission_O2+rab+Thr+PO4.ferm.; 0.576, 0.805, tmp+Mai n.Thr.Conc.+Nitrogen.Conc.+OD+Lys_conc.+X.F.Suc+X.VXdt+tota l.cell+His+lactate+PO4.ferm.; 0.551, 0.805, tmp+P-Source.Fe ed.conc.+Nitrogen.Cone.+Lys_conc.+X.VXdt+total.Vol+yield+em ission_O2+rab+Thr+lactate+PO4.ferm.+PO4.apporte+PO4.utilis e; 0.568, 0.805, Nitrogen.Conc.+Lys_conc.+X.VXdt+total.Vol+ yield+emission_O2+rab+Thr+His+lactate+PO4.apporte+PO4.utili se; 0.542, 0.805, pH+tmp+Main.P-Source.Conc.+Main.Thr.Conc. +Nitrogen.Conc.+OD+Lys_conc.+X.VXdt+total.cell+PL+emission_ O2+rab+Thr+lactate+PO4.ferm.; 0.560, 0.805, tmp+P-Source.Fe ed.conc.+Nitrogen.Conc.+OD+Lys_conc.+X.VXdt+total.Vol+PL+em ission_O2+rab+Thr+lactate+PO4.ferm.; 0.551, 0.805, pH+tmp+P -Source.Feed.conc.+Main.Thr.Conc.+Nitrogen.Conc.+OD+Lys_con c.+X.VXdt+total.Vol+total.cell+emission_O2+rab+lactate+PO4. ferm.; 0.560, 0.805, pH+tmp+Main.P-Source.Conc.+Lys_conc.+X. VXdt+total.Vol+total.cell+PL+emission_O2+rab+Thr+lactate+PO 4.ferm.; 0.599, 0.805, pH+tmp+OD+Lys_conc.+X.F.Suc+X.VXdt+t otal.cell+PO4.ferm.; 0.551, 0.805, pH+tmp+Main.Thr.Conc.+Ly s_conc.+X.VXdt+total.Vol+total.cell+PL+emission_O2+rab+Thr+ lactate+SO4.ferm.+PO4.ferm.; 0.533, 0.805, pH+tmp+Main.Thr. Conc.+Nitrogen.Conc.+Lys_conc.+X.VXdt+total.Vol+total.cell+ PL+emission_O2+rab+Thr+His+lactate+SO4.ferm.+PO4.ferm.; 0.5 84, 0.805, tmp+Nitrogen.Conc.+OD+Lys_conc.+X.VXdt+total.Vol +total.cell+Thr+lactate+PO4.ferm.; 0.576, 0.805, tmp+Main.P -Source.Conc.+Main.Thr.Conc.+Nitrogen.Conc.+OD+Lys_conc.+X. VXdt+total.cell+Thr+lactate+PO4.ferm.; 0.542, 0.805, pH+tmp +P-Source.Feed.conc.+Nitrogen.Conc.+OD+Lys_conc.+X.VXdt+tot al.Vol+PL+emission_O2+rab+Thr+His+lactate+PO4.ferm.; 0.560, 0.805, pH+tmp+Main.P-Source.Conc.+Nitrogen.Conc.+OD+Lys_co nc.+X.VXdt+total.Vol+total.cell+emission_O2+rab+lactate+PO4. ferm.; 0.542, 0.805, pH+tmp+Main.Thr.Conc.+Nitrogen.Conc.+O D+Lys_conc.+X.F.Suc+X.VXdt+total.Vol+total.cell+emission_O2 +rab+Thr+lactate+PO4.ferm.; 0.560, 0.805, tmp+Main.Thr.Conc. +OD+Lys_conc.+X.VXdt+total.cell+PL+emission_O2+rab+Thr+His+ lactate+PO4.ferm.; 0.560, 0.805, Main.P-Source.Conc.+Nitrog en.Conc.+Lys_conc.+X.VXdt+total.Vol+yield+emission_O2+rab+T hr+lactate+PO4.ferm.+PO4.apporte+PO4.utilise; 0.584, 0.805, pH+tmp+Nitrogen.Conc.+Lys_conc.+X.F.Suc+X.VXdt+total.Vol+e mission_O2+rab+PO4.ferm.; 0.584, 0.805, pH+tmp+Main.Thr.Con c.+OD+Lys_conc.+X.VXdt+total.cell+PL+Thr+PO4.ferm.; 0.551, 0.805, pH+tmp+Main.P-Source.Conc.+Nitrogen.Conc.+OD+Lys_con c.+X.VXdt+total.cell+emission_O2+rab+Thr+lactate+SO4.ferm.+ PO4.ferm.; 0.551, 0.805, pH+tmp+Main.Thr.Conc.+Nitrogen.Con c.+Lys_conc.+X.VXdt+total.Vol+emission_O2+rab+Thr+His+lacta te+SO4.ferm.+PO4.ferm.; 0.551, 0.805, pH+Main.Thr.Conc.+Nit rogen.Conc.+Lys_conc.+X.VXdt+total.Vol+yield+emission_O2+ra b+Thr+lactate+PO4.ferm.+PO4.apporte+PO4.utilise; 0.560, 0.8 05, P-Source.Feed.conc.+Nitrogen.Cone.+Lys_conc.+X.VXdt+tot al.Vol+yield+PL+emission_O2+rab+Thr+lactate+PO4.ferm.+PO4.u tilise; 0.598, 0.805, tmp+OD+Lys_conc.+X.VXdt+total.cell+em ission_O2+Thr+PO4.ferm.; 0.560, 0.805, tmp+Main.Thr.Conc.+N itrogen.Conc.+Lys_conc.+X.VXdt+total.Vol+PL+emission_O2+rab +Thr+lactate+PO4.ferm.+PO4.apporte; 0.551, 0.805, pH+tmp+P-Source.Feed.conc.+Main.Thr.Conc.+Nitrogen.Conc.+OD+Lys_conc. +X.VXdt+total.Vol+emission_O2+rab+Thr+lactate+PO4.ferm.; 0. 551, 0.805, pH+tmp+Main.P-Source.Conc.+Nitrogen.Conc.+OD+Ly s_conc.+X.F.Suc+X.VXdt+total.Vol+total.cell+emission_O2+rab +lactate+PO4.ferm.; 0.551, 0.805, pH+tmp+P-Source.Feed.conc. +Nitrogen.Conc.+Lys_conc.+X.F.Suc+X.VXdt+total.Vol+total.ce ll+emission_O2+rab+Thr+lactate+PO4.ferm.; 0.551, 0.805, pH+ tmp+Main.P-Source.Conc.+Nitrogen.Conc.+OD+Lys_conc.+X.F.Suc +X.VXdt+total.cell+emission_O2+rab+His+lactate+PO4.ferm.; 0. 542, 0.805, pH+tmp+P-Source.Feed.conc.+Nitrogen.Conc.+Lys_c onc.+X.VXdt+total.Vol+total.cell+PL+emission_O2+rab+Thr+lac tate+SO4.ferm.+PO4.ferm.; 0.542, 0.805, Main.Thr.Conc.+Nitr ogen.Conc.+Lys_conc.+X.VXdt+total.Vol+yield+PL+emission_O2+ rab+Thr+lactate+SO4.ferm.+PO4.ferm.+PO4.apporte+PO4.utilis e; 0.560, 0.805, tmp+Main.Thr.Conc.+Nitrogen.Conc.+Lys_conc. +X.VXdt+total.Vol+emission_O2+rab+Thr+His+lactate+PO4.ferm. +PO4.apporte; 0.591, 0.805, pH+tmp+Main.Thr.Conc.+Nitrogen. Conc.+Lys_conc.+X.VXdt+total.Vol+lactate+PO4.ferm.; 0.551, 0.805, pH+tmp+P-Source.Feed.conc.+Nitrogen.Conc.+Lys conc.+ X.F.Suc+X.VXdt+total.Vol+total.cell+emission_O2+rab+His+lac tate+PO4.ferm.; 0.560, 0.805, pH+tmp+Main.P-Source.Conc.+Ma in.Thr.Conc.+Nitrogen.Conc.+Lys_conc.+X.VXdt+total.Vol+emis sion_O2+rab+His+lactate+PO4.ferm.; 0.560, 0.805, tmp+Main.P -Source.Conc.+Nitrogen.Conc.+Lys_conc.+X.VXdt+total.Vol+tot al.cell+PL+emission_O2+rab+Thr+lactate+PO4.ferm.; 0.551, 0. 805, pH+tmp+Main.P-Source.Conc.+P-Source.Feed.conc.+Nitroge n.Conc.+OD+Lys_conc.+X.VXdt+total.Vol+total.cell+emission_O 2+rab+lactate+PO4.ferm.; 0.584, 0.805, Nitrogen.Conc.+Lys_c onc.+X.VXdt+total.Vol+emission_O2+rab+Thr+lactate+PO4.ferm. +PO4.apporte; 0.584, 0.805, pH+tmp+Lys_conc.+X.VXdt+total.V ol+total.cell+emission_O2+rab+lactate+PO4.ferm.; 0.551, 0.8 05, tmp+P-Source.Feed.conc.+Nitrogen.Conc.+OD+Lys_conc.+X.V Xdt+total.cell+PL+emission_O2+rab+Thr+His+lactate+PO4.fer m.; 0.551, 0.805, pH+tmp+Main.P-Source.Conc.+Nitrogen.Conc. +Lys_conc.+X.VXdt+total.Vol+total.cell+emission_O2+rab+Thr+ lactate+SO4.ferm.+PO4.ferm.; 0.568, 0.805, tmp+Nitrogen.Con c.+Lys_conc.+X.F.Suc+X.VXdt+total.Vol+emission_O2+rab+Thr+H is+lactate+PO4.ferm.; 0.551, 0.805, tmp+Nitrogen.Conc.+OD+L ys_conc.+X.VXdt+total.Vol+total.cell+PL+emission_O2+rab+Thr +lactate+SO4.ferm.+PO4.ferm.; 0.532, 0.805, pH+tmp+P-Source. Feed.conc.+Nitrogen.Conc.+OD+Lys_conc.+X.VXdt+total.Vol+tot al.cell+PL+emission_O2+rab+Thr+lactate+SO4.ferm.+PO4.ferm.; 0.605, 0.805, pH+tmp+OD+Lys_conc.+X.VXdt+total.cell+PO4.fe rm.; 0.551, 0.805, pH+P-Source.Feed.conc.+Nitrogen.Conc.+Ly s_conc.+X.VXdt+total.Vol+yield+PL+emission_O2+rab+Thr+lacta te+PO4.ferm.+PO4.apporte; 0.559, 0.805, pH+tmp+Nitrogen.Con c.+Lys_conc.+X.F.Suc+X.VXdt+total.Vol+total.cell+emission_O 2+rab+lactate+SO4.ferm.+PO4.ferm.; 0.559, 0.805, pH+tmp+P-S ource.Feed.conc.+Nitrogen.Conc.+Lys_conc.+X.VXdt+total.Vol+ emission_O2+rab+Thr+lactate+PO4.ferm.+PO4.utilise; 0.551, 0. 805, pH+tmp+Nitrogen.Conc.+Lys_conc.+X.VXdt+total.Vol+yield +emission_O2+rab+Thr+His+lactate+PO4.ferm.+PO4.apporte; 0.5 59, 0.805, pH+tmp+Nitrogen.Conc.+OD+Lys_conc.+X.F.Suc+X.VXd t+total.cell+emission_O2+rab+lactate+SO4.ferm.+PO4.ferm.; 0. 551, 0.805, pH+tmp+P-Source.Feed.conc.+Main.Thr.Conc.+Nitro gen.Conc.+OD+Lys_conc.+X.VXdt+total.Vol+emission_O2+rab+His +lactate+PO4.ferm.; 0.576, 0.805, tmp+Main.Thr.Conc.+OD+Lys conc.+X.VXdt+total.cell+emission_O2+Thr+His+lactate+PO4.fe rm.; 0.542, 0.805, pH+tmp+Main.P-Source.Conc.+Nitrogen.Conc. +OD+Lys_conc.+X.VXdt+total.cell+PL+emission_O2+rab+Thr+lact ate+SO4.ferm.+PO4.ferm.; 0.559, 0.805, pH+tmp+P-Source.Feed. conc.+Nitrogen.Conc.+OD+Lys_conc.+X.VXdt+total.cell+PL+emis sion_O2+rab+Thr+PO4.ferm.; 0.542, 0.805, pH+tmp+Main.P-Sour ce.Conc.+Main.Thr.Conc.+Nitrogen.Conc.+Lys_conc.+X.VXdt+tot al.Vol+total.cell+PL+emission_O2+rab+Thr+lactate+PO4.ferm.; 0.568, 0.805, pH+tmp+P-Source.Feed.conc.+OD+Lys_conc.+X.VX dt+total.cell+emission_O2+rab+Thr+lactate+PO4.ferm.; 0.568, 0.805, tmp+Nitrogen.Conc.+Lys_conc.+X.F.Suc+X.VXdt+total.V ol+emission_O2+rab+Thr+lactate+PO4.ferm.+PO4.utilise; 0.583, 0.805, pH+tmp+OD+Lys_conc.+X.VXdt+total.cell+PL+Thr+His+PO 4.ferm.; 0.568, 0.805, tmp+OD+Lys_conc.+X.VXdt+total.cell+e mission_O2+rab+Thr+His+lactate+SO4.ferm.+PO4.ferm.; 0.576, 0.805, tmp+Nitrogen.Conc.+OD+Lys_conc.+X.VXdt+total.Vol+tot al.cell+emission_O2+rab+lactate+PO4.ferm.; 0.559, 0.805, pH +tmp+Main.P-Source.Conc.+Nitrogen.Conc.+Lys_conc.+X.VXdt+to tal.Vol+PL+emission_O2+rab+His+lactate+PO4.ferm.; 0.568, 0. 805, tmp+Main.Thr.Conc.+Lys_conc.+X.VXdt+total.Vol+total.ce ll+PL+emission_O2+rab+Thr+lactate+PO4.ferm.; 0.559, 0.805, tmp+Nitrogen.Conc.+Lys_conc.+X.VXdt+total.Vol+total.cell+em ission_O2+rab+Thr+His+lactate+SO4.ferm.+PO4.ferm.; 0.591, 0. 805, tmp+Main.Thr.Conc.+OD+Lys_conc.+X.VXdt+total.cell+emis sion_O2+Thr+PO4.ferm.; 0.551, 0.805, pH+tmp+P-Source.Feed.c onc.+Nitrogen.Conc.+Lys_conc.+X.VXdt+total.Vol+PL+emission-O2+rab+Thr+lactate+PO4.ferm.+PO4.utilise; 0.542, 0.805, Mai n.Thr.Conc.+Nitrogen.Conc.+Lys conc.+X.F.Suc+X.VXdt+total.V ol+yield+emission_O2+rab+Thr+His+lactate+PO4.ferm.+PO4.appo rte+PO4.utilise; 0.551, 0.805, pH+tmp+P-Source.Feed.conc.+N itrogen.Conc.+Lys_conc.+X.VXdt+total.Vol+total.cell+PL+emis sion_O2+rab+His+lactate+PO4.ferm.; 0.542, 0.805, pH+tmp+Mai n.Thr.Conc.+Nitrogen.Conc.+OD+Lys_conc.+X.VXdt+total.cell+e mission_O2+rab+Thr+His+lactate+SO4.ferm.+PO4.ferm.; 0.567, 0.805, tmp+Nitrogen.Conc.+OD+Lys_conc.+X.F.Suc+X.VXdt+total. cell+emission_O2+rab+His+lactate+PO4.ferm.; 0.541, 0.805, p H+tmp+P-Source.Feed.conc.+Nitrogen.Conc.+OD+Lys_conc.+X.VXd t+total.cell+PL+emission_O2+rab+Thr+lactate+SO4.ferm.+PO4.f erm.; 0.559, 0.805, pH+tmp+Nitrogen.Conc.+Lys_conc.+X.VXdt+ total.Vol+yield+emission_O2+rab+Thr+lactate+PO4.ferm.+PO4.u tilise; 0.575, 0.805, tmp+OD+Lys_conc.+X.F.Suc+X.VXdt+total. cell+emission_O2+rab+Thr+His+PO4.ferm.; 0.550, 0.805, pH+tm p+Main.Thr.Conc.+Nitrogen.Conc.+OD+Lys_conc.+X.VXdt+total.V ol+total.cell+emission_O2+rab+His+lactate+PO4.ferm.; 0.567, 0.805, tmp+Main.Thr.Conc.+Nitrogen.Conc.+Lys_conc.+X.VXdt+ total.Vol+emission_O2+rab+Thr+lactate+SO4.ferm.+PO4.ferm.; 0.575, 0.804, tmp+Nitrogen.Conc.+OD+Lys_conc.+X.VXdt+total. cell+emission_O2+Thr+His+lactate+PO4.ferm.; 0.559, 0.804, t mp+P-Source.Feed.conc.+Main.Thr.Conc.+Nitrogen.Conc.+Lys_co nc.+X.VXdt+total.Vol+yield+emission_O2+rab+Thr+lactate+PO4. ferm.; 0.522, 0.804, pH+tmp+Main.Thr.Conc.+Nitrogen.Conc.+O D+Lys_conc.+X.VXdt+total.Vol+total.cell+PL+emission_O2+rab+ Thr+His+lactate+SO4.ferm.+PO4.ferm.; 0.559, 0.804, pH+tmp+N itrogen.Conc.+Lys_conc.+X.VXdt+total.Vol+total.cell+emissio n_O2+rab+His+lactate+SO4.ferm.+PO4.ferm.; 0.559, 0.804, tmp +P-Source.Feed.conc.+Nitrogen.Conc.+Lys_conc.+X.VXdt+total. Vol+emission_O2+rab+Thr+His+lactate+PO4.ferm.+PO4.apporte; 0.583, 0.804, pH+tmp+P-Source.Feed.conc.+Nitrogen.Conc.+Lys _conc.+X.VXdt+total.Vol+emission_O2+rab+PO4.ferm.; 0.575, 0. 804, pH+tmp+OD+Lys_conc.+X.VXdt+total.cell+PL+Thr+His+lacta te+PO4.ferm.; 0.590, 0.804, pH+tmp+Nitrogen.Conc.+OD+Lys_co nc.+X.VXdt+total.cell+Thr+PO4.ferm.; 0.590, 0.804, pH+tmp+N itrogen.Conc.+OD+Lys_conc.+X.VXdt+total.cell+lactate+PO4.fe rm.; 0.567, 0.804, tmp+Nitrogen.Conc.+OD+Lys_conc.+X.F.Suc+ X.VXdt+total.Vol+total.cell+emission_O2+rab+lactate+PO4.fer m.; 0.583, 0.804, pH+tmp+Main.Thr.Conc.+Nitrogen.Conc.+Lys_ conc.+X.F.Suc+X.VXdt+total.Vol+lactate+PO4.ferm.; 0.550, 0. 804, tmp+Lys_conc.+X.VXdt+total.Vol+yield+PL+emission_O2+ra b+Thr+His+lactate+PO4.ferm.+PO4.apporte+PO4.utilise; 0.550, 0.804, pH+tmp+Main.P-Source.Conc.+Nitrogen.Conc.+OD+Lys_co nc.+X.VXdt+total.Vol+emission_O2+rab+Thr+His+lactate+PO4.fe rm.; 0.575, 0.804, tmp+Nitrogen.Conc.+OD+Lys_conc.+X.F.Suc+ X.VXdt+total.Vol+emission_O2+rab+lactate+PO4.ferm.; 0.567, 0.804, pH+tmp+Nitrogen.Conc.+Lys_conc.+X.VXdt+total.Vol+tot al.cell+PL+emission_O2+rab+Thr+PO4.ferm.; 0.567, 0.804, tmp +Main.Thr.Conc.+OD+Lys_conc.+X.VXdt+total.cell+PL+Thr+His+l actate+SO4.ferm.+PO4.ferm.; 0.550, 0.804, pH+tmp+Main.Thr.C onc.+OD+Lys_conc.+X.F.Suc+X.VXdt+total.cell+PL+emission_O2+ rab+Thr+lactate+PO4.ferm.; 0.559, 0.804, tmp+OD+Lys_conc.+X. VXdt+total.Vol+total.cell+PL+emission_O2+rab+Thr+His+lactat e+PO4.ferm.; 0.559, 0.804, Nitrogen.Conc.+Lys_conc.+X.F.Suc +X.VXdt+total.Vol+yield+PL+emission_O2+Thr+lactate+PO4.ferm. +PO4.apporte+PO4.utilise; 0.550, 0.804, pH+Nitrogen.Conc.+L ys_conc.+X.VXdt+total.Vol+yield+emission_O2+rab+Thr+lactate +SO4.ferm.+PO4.ferm.+PO4.apporte+PO4.utilise; 0.559, 0.804, tmp+P-Source.Feed.conc.+Nitrogen.Conc.+OD+Lys_conc.+X.VXdt +total.Vol+total.cell+emission_O2+rab+His+lactate+PO4.fer m.; 0.550, 0.804, pH+tmp+P-Source.Feed.conc.+Nitrogen.Conc. +OD+Lys_conc.+X.VXdt+total.Vol+total.cell+PL+emission_O2+ra b+lactate+PO4.ferm.; 0.567, 0.804, pH+tmp+Nitrogen.Conc.+OD +Lys_conc.+X.VXdt+total.Vol+emission_O2+rab+lactate+SO4.fer m.+PO4.ferm.; 0.550, 0.804, pH+tmp+OD+Lys_conc.+X.F.Suc+X.V Xdt+total.cell+PL+emission_O2+rab+Thr+His+lactate+PO4.fer m.; 0.559, 0.804, pH+tmp+OD+Lys_conc.+X.F.Suc+X.VXdt+total. cell+PL+emission_O2+rab+Thr+His+PO4.ferm.; 0.583, 0.804, tm p+OD+Lys_conc.+X.VXdt+total.Vol+total.cell+PL+Thr+lactate+P O4.ferm.; 0.583, 0.804, tmp+Nitrogen.Conc.+OD+Lys_conc.+X.F. Suc+X.VXdt+total.cell+PL+Thr+PO4.ferm.; 0.550, 0.804, tmp+M ain.Thr.Conc.+Nitrogen.Conc.+OD+Lys_conc.+X.VXdt+total.cell +PL+emission_O2+rab+Thr+His+lactate+PO4.ferm.; 0.567, 0.804, pH+tmp+P-Source.Feed.conc.+Nitrogen.Conc.+Lys_conc.+X.VXdt +total.Vol+emission_O2+rab+lactate+PO4.ferm.+PO4.utilise; 0. 575, 0.804, tmp+Nitrogen.Conc.+Lys_conc.+X.VXdt+total.Vol+e mission_O2+rab+lactate+PO4.ferm.+PO4.apporte+PO4.utilise; 0. 590, 0.804, pH+tmp+Nitrogen.Conc.+Lys_conc.+X.VXdt+total.Vo l+His+lactate+PO4.ferm.; 0.559, 0.804, tmp+P-Source.Feed.co nc.+Nitrogen.Conc.+Lys_conc.+X.VXdt+total.Vol+total.cell+em ission_O2+rab+Thr+lactate+SO4.ferm.+PO4.ferm.; 0.559, 0.804, pH+tmp+Nitrogen.Conc.+OD+Lys_conc.+X.F.Suc+X.VXdt+total.ce ll+PL+emission_O2+rab+lactate+PO4.ferm.; 0.559, 0.804, pH+t mp+Main.Thr.Conc.+Nitrogen.Conc.+Lys_conc.+X.VXdt+total.Vol +PL+emission_O2+rab+lactate+SO4.ferm.+PO4.ferm.; 0.559, 0.8 04, tmp+Nitrogen.Conc.+Lys_conc.+X.F.Suc+X.VXdt+total.Vol+y ield+emission_O2+rab+lactate+PO4.ferm.+PO4.apporte+PO4.util ise; 0.550, 0.804, pH+tmp+Main.Thr.Conc.+Nitrogen.Conc.+Lys conc.+X.VXdt+total.Vol+emission_O2+rab+Thr+lactate+PO4.fer m.+PO4.apporte+PO4.utilise; 0.567, 0.804, tmp+Main.P-Source. Conc.+Nitrogen.Conc.+OD+Lys_conc.+X.F.Suc+X.VXdt+total.cell +Thr+His+lactate+PO4.ferm.; 0.567, 0.804, tmp+Nitrogen.Conc. +Lys_conc.+X.VXdt+total.Vol+emission_O2+rab+Thr+lactate+SO4. ferm.+PO4.ferm.+PO4.apporte; 0.559, 0.804, pH+tmp+P-Source. Feed.conc.+Main.Thr.Conc.+Nitrogen.Conc.+Lys_conc.+X.VXdt+t otal.Vol+emission_O2+rab+lactate+SO4.ferm.+PO4.ferm.; 0.597, 0.804, tmp+Main.P-Source.Conc.+OD+Lys_conc.+X.VXdt+total.c ell+Thr+PO4.ferm.; 0.567, 0.804, tmp+Main.Thr.Conc.+Nitroge n.Conc.+Lys_conc.+X.VXdt+total.Vol+emission_O2+rab+Thr+His+ lactate+PO4.ferm.; 0.550, 0.804, pH+tmp+P-Source.Feed.conc. +Nitrogen.Conc.+Lys_conc.+X.VXdt+total.Vol+yield+PL+emissio n_O2+rab+Thr+lactate+PO4.ferm.; 0.550, 0.804, pH+tmp+Nitrog en.Conc.+Lys_conc.+X.VXdt+total.Vol+PL+emission_O2+rab+Thr+ lactate+SO4.ferm.+PO4.ferm.+PO4.utilise; 0.575, 0.804, tmp+ Nitrogen.Conc.+OD+Lys_conc.+X.VXdt+total.Vol+emission_O2+ra b+His+lactate+PO4.ferm.; 0.567, 0.804, tmp+Main.P-Source.Co nc.+OD+Lys_conc.+X.F.Suc+X.VXdt+total.cell+emission_O2+rab+ Thr+lactate+PO4.ferm.; 0.590, 0.804, tmp+Main.Thr.Conc.+OD+ Lys_conc.+X.F.Suc+X.VXdt+total.cell+Thr+PO4.ferm.; 0.559, 0. 804, tmp+Main.Thr.Conc.+Nitrogen.Conc.+OD+Lys conc.+X.F.Suc +X.VXdt+total.cell+Thr+His+lactate+SO4.ferm.+PO4.ferm.; 0.5 41, 0.804, pH+tmp+P-Source.Feed.conc.+Main.Thr.Conc.+Nitrog en.Conc.+Lys_conc.+X.VXdt+total.Vol+PL+emission_O2+rab+Thr+ lactate+SO4.ferm.+PO4.ferm.; 0.567, 0.804, tmp+Main.Thr.Con c.+OD+Lys_conc.+X.VXdt+total.cell+PL+emission_O2+Thr+lactat e+SO4.ferm.+PO4.ferm.; 0.575, 0.804, pH+tmp+Main.P-Source.C onc.+Main.Thr.Conc.+Lys_conc.+X.VXdt+total.Vol+emission_O2+ rab+lactate+PO4.ferm.; 0.583, 0.804, tmp+Nitrogen.Conc.+OD+ Lys_conc.+X.F.Suc+X.VXdt+total.cell+Thr+His+PO4.ferm.; 0.55 0, 0.804, tmp+P-Source.Feed.conc.+Nitrogen.Conc.+OD+Lys_con c.+X.VXdt+total.Vol+total.cell+emission_O2+rab+Thr+lactate+ SO4.ferm.+PO4.ferm.; 0.541, 0.804, pH+tmp+Nitrogen.Conc.+Ly s_conc.+X.VXdt+total.Vol+yield+emission_O2+rab+Thr+His+lact ate+PO4.ferm.+PO4.apporte+PO4.utilise; 0.550, 0.804, pH+tmp +Main.P-Source.Conc.+P-Source.Feed.conc.+Nitrogen.Conc.+OD+ Lys_conc.+X.VXdt+total.Vol+emission_O2+rab+Thr+lactate+PO4. ferm.; 0.558, 0.804, tmp+Main.Thr.Conc.+OD+Lys_conc.+X.VXdt +total.cell+PL+emission_O2+rab+Thr+lactate+SO4.ferm.+PO4.fe rm.; 0.558, 0.804, pH+tmp+Nitrogen.Conc.+Lys_conc.+X.F.Suc+ X.VXdt+total.Vol+emission_O2+rab+Thr+lactate+PO4.ferm.+PO4. utilise; 0.567, 0.804, Main.Thr.Conc.+Nitrogen.Conc.+Lys_co nc.+X.VXdt+total.Vol+yield+emission_O2+Thr+lactate+PO4.ferm. +PO4.apporte+PO4.utilise; 0.541, 0.804, pH+tmp+Main.P-Sourc e.Conc.+P-Source.Feed.conc.+Nitrogen.Conc.+OD+Lys_conc.+X.V Xdt+total.cell+PL+emission_O2+rab+Thr+lactate+PO4.ferm.; 0. 590, 0.804, tmp+P-Source.Feed.conc.+Nitrogen.Conc.+Lys_conc. +X.VXdt+total.Vol+His+lactate+PO4.ferm.; 0.575, 0.804, tmp+ OD+Lys_conc.+X.VXdt+total.cell+PL+emission_O2+Thr+His+lacta te+PO4.ferm.; 0.590, 0.804, pH+tmp+Lys_conc.+X.VXdt+total.V ol+PL+emission_O2+rab+PO4.ferm.; 0.567, 0.804, tmp+Main.P-S ource.Conc.+P-Source.Feed.conc.+Nitrogen.Conc.+Lys_conc.+X. VXdt+total.Vol+total.cell+emission_O2+rab+lactate+PO4.fer m.; 0.582, 0.804, pH+tmp+OD+Lys_conc.+X.VXdt+total.cell+Thr +His+lactate+PO4.ferm.; 0.558, 0.804, pH+tmp+OD+Lys_conc.+X. VXdt+total.cell+PL+emission_O2+rab+Thr+His+SO4.ferm.+PO4.fe rm.; 0.582, 0.804, tmp+Main.Thr.Conc.+Nitrogen.Conc.+OD+Lys conc.+X.VXdt+total.cell+Thr+His+PO4.ferm.; 0.558, 0.804, p H+tmp+P-Source.Feed.conc.+Nitrogen.Conc.+Lys_conc.+X.VXdt+t otal.Vol+emission_O2+rab+His+lactate+PO4.ferm.+PO4.apporte; 0.550, 0.804, tmp+P-Source.Feed.conc.+Nitrogen.Conc.+OD+Ly s_conc.+X.F.Suc+X.VXdt+total.Vol+total.cell+emission_O2+rab +Thr+lactate+PO4.ferm.; 0.590, 0.804, tmp+Nitrogen.Conc.+OD +Lys_conc.+X.F.Suc+X.VXdt+total.cell+His+PO4.ferm.

### [16. Linear Model that Predicts Lysine Production Amount in Interval 6]

0.463, 0.744, pH+Nitrogen.Conc.+OD+X.F.Suc+X.VXdt+yield+rab +Thr+PO4.ferm.+PO4.apporte+PO4.utilise; 0.459, 0.742, pH+Ni trogen.Conc.+OD+X.F.Suc+X.VXdt+yield+emission_O2+Thr+PO4.fe rm.+PO4.apporte+PO4.utilise; 0.446, 0.740, pH+Nitrogen.Conc. +OD+X.F.Suc+X.VXdt+yield+rab+Thr+His+PO4.ferm.+PO4.apporte+ PO4.utilise; 0.445, 0.740, pH+Nitrogen.Conc.+OD+X.F.Suc+X.V Xdt+yield+PL+rab+Thr+PO4.ferm.+PO4.apporte+PO4.utilise; 0.4 43, 0.739, pH+Nitrogen.Conc.+OD+X.F.Suc+X.VXdt+yield+emissi on_O2+Thr+His+PO4.ferm.+PO4.apporte+PO4.utilise; 0.462, 0.7 38, pH+Nitrogen.Conc.+X.F.Suc+X.VXdt+yield+rab+Thr+PO4.ferm. +PO4.apporte+PO4.utilise; 0.440, 0.737, pH+Nitrogen.Conc.+O D+X.F.Suc+X.VXdt+yield+PL+emission_O2+Thr+PO4.ferm.+PO4.app orte+PO4.utilise; 0.470, 0.737, pH+Nitrogen.Conc.+X.F.Suc+X. VXdt+yield+rab+PO4.ferm.+PO4.apporte+PO4.utilise; 0.440, 0. 737, pH+Main.P-Source.Conc.+Nitrogen.Conc.+OD+X.F.Suc+X.VXd t+yield+rab+Thr+PO4.ferm.+PO4.apporte+PO4.utilise; 0.439, 0. 736, pH+Nitrogen.Conc.+OD+X.F.Suc+X.VXdt+yield+rab+Thr+lact ate+PO4.ferm.+PO4.apporte+PO4.utilise; 0.439, 0.736, pH+Nit rogen.Conc.+OD+X.F.Suc+X.VXdt+yield+emission_O2+rab+Thr+PO4. ferm.+PO4.apporte+PO4.utilise; 0.427, 0.736, pH+Nitrogen.Co nc.+OD+X.F.Suc+X.VXdt+yield+PL+rab+Thr+His+PO4.ferm.+PO4.ap porte+PO4.utilise; 0.437, 0.736, pH+Nitrogen.Conc.+OD+X.F.S uc+X.VXdt+yield+emission_CO2+rab+Thr+PO4.ferm.+PO4.apporte+ PO4.utilise; 0.448, 0.736, pH+Nitrogen.Conc.+X.F.Suc+X.VXdt +total.cell+yield+rab+Thr+PO4.ferm.+PO4.apporte+PO4.utilis e; 0.435, 0.735, pH+Nitrogen.Conc.+OD+X.F.Suc+X.VXdt+yield+ emission_O2+emission_CO2+Thr+PO4.ferm.+PO4.apporte+PO4.util ise; 0.424, 0.734, pH+tmp+Nitrogen.Conc.+X.F.Suc+X.VXdt+tot al.cell+yield+emission_O2+Thr+His+PO4.ferm.+PO4.apporte+PO4. utilise; 0.435, 0.734, pH+Nitrogen.Conc.+OD+X.F.Suc+X.VXdt+ yield+emission_O2+Thr+lactate+PO4.ferm.+PO4.apporte+PO4.uti lise; 0.424, 0.734, pH+Nitrogen.Conc.+OD+X.F.Suc+X.VXdt+yie ld+PL+emission_O2+Thr+His+PO4.ferm.+PO4.apporte+PO4.utilis e; 0.455, 0.734, pH+Nitrogen.Conc.+OD+X.F.Suc+X.VXdt+yield+ rab+PO4.ferm.+PO4.apporte+PO4.utilise; 0.445, 0.734, pH+Nit rogen.Conc.+X.F.Suc+X.VXdt+yield+PL+rab+Thr+PO4.ferm.+PO4.a pporte+PO4.utilise; 0.445, 0.734, pH+Nitrogen.Conc.+X.F.Suc +X.VXdt+yield+rab+Thr+His+PO4.ferm.+PO4.apporte+PO4.utilis e; 0.434, 0.734, pH+Main.P-Source.Conc.+Nitrogen.Conc.+OD+X. F.Suc+X.VXdt+yield+emission_O2+Thr+PO4.ferm.+PO4.apporte+PO 4.utilise; 0.444, 0.734, pH+Nitrogen.Conc.+X.F.Suc+X.VXdt+y ield+rab+Thr+lactate+PO4.ferm.+PO4.apporte+PO4.utilise; 0.4 33, 0.733, pH+tmp+Nitrogen.Conc.+X.F.Suc+X.VXdt+total.cell+ yield+emission_O2+Thr+PO4.ferm.+PO4.apporte+PO4.utilise; 0. 453, 0.733, pH+Nitrogen.Conc.+X.F.Suc+X.VXdt+yield+PL+rab+P O4.ferm.+PO4.apporte+PO4.utilise; 0.453, 0.733, pH+Nitrogen. Conc.+X.F.Suc+X.VXdt+yield+emission_O2+Thr+PO4.ferm.+PO4.ap porte+PO4.utilise; 0.432, 0.733, pH+tmp+Nitrogen.Conc.+X.F. Suc+X.VXdt+total.cell+yield+rab+Thr+PO4.ferm.+PO4.apporte+P O4.utilise; 0.453, 0.733, pH+Nitrogen.Conc.+X.F.Suc+X.VXdt+ yield+rab+His+PO4.ferm.+PO4.apporte+PO4.utilise; 0.421, 0.7 32, pH+Nitrogen.Conc.+OD+X.F.Suc+X.VXdt+yield+emission_O2+r ab+Thr+His+PO4.ferm.+PO4.apporte+PO4.utilise; 0.409, 0.732, pH+tmp+Nitrogen.Conc.+X.F.Suc+X.VXdt+total.cell+yield+PL+e mission_O2+Thr+His+PO4.ferm.+PO4.apporte+PO4.utilise; 0.452, 0.732, pH+Nitrogen.Cone.+X.F.Suc+X.VXdt+total.cell+yield+r ab+PO4.ferm.+PO4.apporte+PO4.utilise; 0.421, 0.732, pH+tmp+ Nitrogen.Conc.+X.F.Suc+X.VXdt+total.cell+yield+rab+Thr+His+ PO4.ferm.+PO4.apporte+PO4.utilise; 0.420, 0.732, pH+Nitroge n.Conc.+OD+X.F.Suc+X.VXdt+yield+PL+rab+Thr+lactate+PO4.ferm. +PO4.apporte+PO4.utilise; 0.452, 0.732, pH+tmp+Nitrogen.Con c.+X.F.Suc+X.VXdt+yield+rab+PO4.ferm.+PO4.apporte+PO4.utili se; 0.420, 0.732, pH+Main.P-Source.Conc.+Nitrogen.Conc.+OD+ X.F.Suc+X.VXdt+yield+PL+rab+Thr+PO4.ferm.+PO4.apporte+PO4.u tilise; 0.442, 0.732, pH+tmp+Nitrogen.Conc.+X.F.Suc+X.VXdt+ total.cell+yield+rab+PO4.ferm.+PO4.apporte+PO4.utilise; 0.4 51, 0.732, pH+tmp+Nitrogen.Conc.+X.F.Suc+X.VXdt+yield+emiss ion_O2+PO4.ferm.+PO4.apporte+PO4.utilise; 0.451, 0.732, pH+ Nitrogen.Conc.+X.F.Suc+X.VXdt+yield+rab+lactate+PO4.ferm.+P O4.apporte+PO4.utilise; 0.420, 0.732, pH+Nitrogen.Conc.+OD+ X.F.Suc+X.VXdt+yield+PL+emission_O2+rab+Thr+PO4.ferm.+PO4.a pporte+PO4.utilise; 0.419, 0.732, pH+Nitrogen.Conc.+OD+X.F. Suc+X.VXdt+yield+emission_CO2+rab+Thr+His+PO4.ferm.+PO4.app orte+PO4.utilise; 0.441, 0.732, pH+Main.P-Source.Conc.+Nitr ogen.Conc.+X.F.Suc+X.VXdt+yield+rab+Thr+PO4.ferm.+PO4.appor te+PO4.utilise; 0.419, 0.732, pH+Nitrogen.Conc.+OD+X.F.Suc+ X.VXdt+yield+rab+Thr+His+lactate+PO4.ferm.+PO4.apporte+PO4. utilise; 0.441, 0.732, pH+tmp+Nitrogen.Conc.+X.F.Suc+X.VXdt +yield+emission_O2+His+PO4.ferm.+PO4.apporte+PO4.utilise; 0. 430, 0.731, pH+Nitrogen.Conc.+X.F.Suc+X.VXdt+total.cell+yie ld+PL+rab+Thr+PO4.ferm.+PO4.apporte+PO4.utilise; 0.419, 0.7 31, pH+tmp+Nitrogen.Conc.+X.F.Suc+X.VXdt+total.cell+yield+e mission_CO2+Thr+His+PO4.ferm.+PO4.apporte+PO4.utilise; 0.41 9, 0.731, pH+Main.P-Source.Conc.+Nitrogen.Conc.+OD+X.F.Suc+ X.VXdt+yield+rab+Thr+His+PO4.ferm.+PO4.apporte+PO4.utilise; 0.429, 0.731, pH+P-Source.Feed.conc.+Nitrogen.Conc.+OD+X.F. Suc+X.VXdt+yield+rab+Thr+PO4.ferm.+PO4.apporte+PO4.utilise; 0.429, 0.731, pH+Nitrogen.Conc.+X.F.Suc+X.VXdt+total.cell+ yield+rab+Thr+His+PO4.ferm.+PO4.apporte+PO4.utilise; 0.429, 0.731, pH+Main.Thr.Conc.+Nitrogen.Conc.+OD+X.F.Suc+X.VXdt+ yield+rab+Thr+PO4.ferm.+PO4.apporte+PO4.utilise; 0.418, 0.7 31, pH+Nitrogen.Conc.+OD+X.F.Suc+X.VXdt+yield+PL+emission-C O2+rab+Thr+PO4.ferm.+PO4.apporte+PO4.utilise; 0.418, 0.731, pH+Nitrogen.Conc.+OD+X.F.Suc+X.VXdt+yield+emission_O2+Thr+ His+lactate+PO4.ferm.+PO4.apporte+PO4.utilise; 0.417, 0.731, pH+tmp+Nitrogen.Conc.+X.F.Suc+X.VXdt+total.cell+yield+PL+e mission_O2+Thr+PO4.ferm.+PO4.apporte+PO4.utilise; 0.439, 0. 731, pH+tmp+Nitrogen.Conc.+X.F.Suc+X.VXdt+total.cell+yield+ emission_O2+PO4.ferm.+PO4.apporte+PO4.utilise; 0.428, 0.731, pH+tmp+Nitrogen.Conc.+X.F.Suc+X.VXdt+total.cell+yield+emis sion_O2+His+PO4.ferm.+PO4.apporte+PO4.utilise; 0.428, 0.731, pH+tmp+Nitrogen.Conc.+X.F.Suc+X.VXdt+total.cell+yield+rab+ His+PO4.ferm.+PO4.apporte+PO4.utilise; 0.406, 0.731, pH+tmp +Nitrogen.Conc.+X.F.Suc+X.VXdt+total.cell+yield+PL+rab+Thr+ His+PO4.ferm.+PO4.apporte+PO4.utilise; 0.439, 0.731, pH+tmp +Nitrogen.Conc.+X.F.Suc+X.VXdt+yield+rab+His+PO4.ferm.+PO4. apporte+PO4.utilise; 0.417, 0.730, pH+Nitrogen.Conc.+OD+X.F. Suc+X.VXdt+yield+emission_O2+emission_CO2+Thr+His+PO4.ferm. +PO4.apporte+PO4.utilise; 0.417, 0.730, pH+tmp+Nitrogen.Con c.+X.F.Suc+X.VXdt+total.cell+yield+PL+rab+Thr+PO4.ferm.+PO4. apporte+PO4.utilise; 0.448, 0.730, pH+Main.P-Source.Conc.+N itrogen.Conc.+X.F.Suc+X.VXdt+yield+rab+PO4.ferm.+PO4.apport e+PO4.utilise; 0.458, 0.730, pH+Nitrogen.Conc.+X.F.Suc+X.VX dt+yield+emission_O2+PO4.ferm.+PO4.apporte+PO4.utilise; 0.4 27, 0.730, pH+Nitrogen.Conc.+X.F.Suc+X.VXdt+yield+PL+rab+Th r+His+PO4.ferm.+PO4.apporte+PO4.utilise; 0.438, 0.730, pH+N itrogen.Conc.+OD+X.F.Suc+X.VXdt+yield+rab+His+PO4.ferm.+PO4. apporte+PO4.utilise; 0.416, 0.730, pH+Nitrogen.Conc.+OD+X.F. Suc+X.VXdt+yield+PL+emission_O2+Thr+lactate+PO4.ferm.+PO4.a pporte+PO4.utilise; 0.438, 0.730, pH+Nitrogen.Conc.+OD+X.F. Suc+X.VXdt+yield+PL+rab+PO4.ferm.+PO4.apporte+PO4.utilise; 0.427, 0.730, pH+P-Source.Feed.conc.+Nitrogen.Conc.+OD+X.F. Suc+X.VXdt+yield+emission_O2+Thr+PO4.ferm.+PO4.apporte+PO4. utilise; 0.438, 0.730, pH+Nitrogen.Conc.+X.F.Suc+X.VXdt+yie ld+emission_O2+Thr+His+PO4.ferm.+PO4.apporte+PO4.utilise; 0. 448, 0.730, pH+Nitrogen.Conc.+X.F.Suc+X.VXdt+yield+emission _O2+rab+PO4.ferm.+PO4.apporte+PO4.utilise; 0.438, 0.730, pH +Nitrogen.Conc.+X.F.Suc+X.VXdt+yield+emission_O2+rab+Thr+PO 4.ferm.+PO4.apporte+PO4.utilise; 0.416, 0.730, pH+Nitrogen. Conc.+OD+X.F.Suc+X.VXdt+yield+PL+emission_O2+emission_CO2+T hr+PO4.ferm.+PO4.apporte+PO4.utilise; 0.416, 0.730, pH+Nitr ogen.Conc.+OD+X.F.Suc+X.VXdt+yield+emission_O2+emission_CO2 +rab+Thr+PO4.ferm.+PO4.apporte+PO4.utilise; 0.426, 0.729, p H+Nitrogen.Conc.+X.F.Suc+X.VXdt+yield+PL+rab+Thr+lactate+PO 4.ferm.+PO4.apporte+PO4.utilise; 0.426, 0.729, pH+tmp+Nitro gen.Conc.+X.F.Suc+X.VXdt+yield+emission_O2+Thr+His+PO4.ferm. +PO4.apporte+PO4.utilise; 0.403, 0.729, pH+tmp+Nitrogen.Con c.+X.F.Suc+X.VXdt+total.cell+yield+PL+emission_CO2+Thr+His+ PO4.ferm.+PO4.apporte+PO4.utilise; 0.415, 0.729, pH+Main.P-Source.Conc.+Nitrogen.Conc.+OD+X.F.Suc+X.VXdt+yield+PL+emis sion_O2+Thr+PO4.ferm.+PO4.apporte+PO4.utilise; 0.415, 0.729, pH+Main.P-Source.Conc.+Nitrogen.Conc.+OD+X.F.Suc+X.VXdt+yi eld+emission_O2+Thr+His+PO4.ferm.+PO4.apporte+PO4.utilise; 0.436, 0.729, pH+Nitrogen.Conc.+X.F.Suc+X.VXdt+yield+PL+emi ssion_O2+Thr+PO4.ferm.+PO4.apporte+PO4.utilise; 0.426, 0.72 9, pH+P-Source.Feed.conc.+Nitrogen.Conc.+X.F.Suc+X.VXdt+tot al.cell+yield+rab+Thr+PO4.ferm.+PO4.apporte+PO4.utilise; 0. 425, 0.729, pH+tmp+Nitrogen.Conc.+X.F.Suc+X.VXdt+total.cell +yield+PL+rab+PO4.ferm.+PO4.apporte+PO4.utilise; 0.425, 0.7 29, pH+Nitrogen.Conc.+X.F.Suc+X.VXdt+total.cell+yield+emiss ion_O2+rab+Thr+PO4.ferm.+PO4.apporte+PO4.utilise; 0.436, 0. 729, pH+Nitrogen.Conc.+X.F.Suc+X.VXdt+yield+emission_CO2+ra b+Thr+PO4.ferm.+PO4.apporte+PO4.utilise; 0.425, 0.729, pH+M ain.P-Source.Conc.+Nitrogen.Conc.+X.F.Suc+X.VXdt+total.cell +yield+rab+Thr+PO4.ferm.+PO4.apporte+PO4.utilise; 0.436, 0. 729, pH+Nitrogen.Conc.+X.F.Suc+X.VXdt+yield+PL+rab+His+PO4. ferm.+PO4.apporte+PO4.utilise; 0.435, 0.729, pH+P-Source.Fe ed.conc.+Nitrogen.Conc.+X.F.Suc+X.VXdt+yield+rab+Thr+PO4.fe rm.+PO4.apporte+PO4.utilise; 0.446, 0.729, pH+Nitrogen.Conc. +OD+X.F.Suc+X.VXdt+yield+emission_O2+PO4.ferm.+PO4.apporte+ PO4.utilise; 0.435, 0.729, pH+tmp+Nitrogen.Conc.+X.F.Suc+X. VXdt+yield+PL+rab+PO4.ferm.+PO4.apporte+PO4.utilise; 0.446, 0.729, pH+Nitrogen.Conc.+X.F.Suc+X.VXdt+yield+emission_CO2 +rab+PO4.ferm.+PO4.apporte+PO4.utilise; 0.424, 0.729, pH+Ma in.Thr.Conc.+Nitrogen.Conc.+OD+X.F.Suc+X.VXdt+yield+emissio n_O2+Thr+PO4.ferm.+PO4.apporte+PO4.utilise; 0.424, 0.728, p H+tmp+Nitrogen.Conc.+X.F.Suc+X.VXdt+yield+PL+emission-O2+Hi s+PO4.ferm.+PO4.apporte+PO4.utilise; 0.435, 0.728, pH+Nitro gen.Conc.+X.F.Suc+X.VXdt+total.cell+yield+PL+rab+PO4.ferm.+ PO4.apporte+PO4.utilise; 0.435, 0.728, pH+tmp+Nitrogen.Conc. +X.F.Suc+X.VXdt+yield+emission_O2+Thr+PO4.ferm.+PO4.apporte +PO4.utilise; 0.435, 0.728, pH+Nitrogen.Conc.+X.F.Suc+X.VXd t+yield+emission_O2+emission_CO2+Thr+PO4.ferm.+PO4.apporte+ PO4.utilise;0.424, 0.728, pH+Nitrogen.Conc.+X.F.Suc+X.VXdt+ yield+rab+Thr+His+lactate+PO4.ferm.+PO4.apporte+PO4.utilis e; 0.435, 0.728, pH+Nitrogen.Conc.+X.F.Suc+X.VXdt+yield+PL+ rab+lactate+PO4.ferm.+PO4.apporte+PO4.utilise; 0.435, 0.728, pH+tmp+Nitrogen.Conc.+X.F.Suc+X.VXdt+yield+PL+emission_O2+ PO4.ferm.+PO4.apporte+PO4.utilise; 0.413, 0.728, pH+Main.P-Source.Conc.+Nitrogen.Conc.+OD+X.F.Suc+X.VXdt+yield+rab+Thr +lactate+PO4.ferm.+PO4.apporte+PO4.utilise; 0.424, 0.728, p H+Nitrogen.Conc.+X.F.Suc+X.VXdt+total.cell+yield+rab+Thr+la ctate+PO4.ferm.+PO4.apporte+PO4.utilise; 0.401, 0.728, pH+N itrogen.Conc.+OD+X.F.Suc+X.VXdt+yield+PL+emission_O2+rab+Th r+His+PO4.ferm.+PO4.apporte+PO4.utilise; 0.434, 0.728, pH+N itrogen.Conc.+X.F.Suc+X.VXdt+yield+emission_O2+Thr+lactate+ PO4.ferm.+PO4.apporte+PO4.utilise; 0.434, 0.728, pH+Nitroge n.Conc.+X.F.Suc+X.VXdt+total.cell+yield+emission_O2+Thr+PO4. ferm.+PO4.apporte+PO4.utilise; 0.423, 0.728, pH+tmp+Nitroge n.Conc.+X.F.Suc+X.VXdt+yield+emission_CO2+Thr+His+PO4.ferm. +PO4.apporte+PO4.utilise; 0.434, 0.728, pH+Nitrogen.Conc.+X. F.Suc+X.VXdt+total.cell+yield+rab+His+PO4.ferm.+PO4.apporte +PO4.utilise; 0.454, 0.728, pH+tmp+Nitrogen.Conc.+X.VXdt+to tal.cell+yield+emission_CO2+Thr+His; 0.412, 0.728, pH+tmp+N itrogen.Conc.+X.F.Suc+X.VXdt+total.cell+yield+PL+emission_O 2+His+PO4.ferm.+PO4.apporte+PO4.utilise; 0.412, 0.728, pH+t mp+Nitrogen.Conc.+X.F.Suc+X.VXdt+total.cell+yield+PL+rab+Hi s+PO4.ferm.+PO4.apporte+PO4.utilise; 0.412, 0.728, pH+Main. P-Source.Conc.+Nitrogen.Conc.+OD+X.F.Suc+X.VXdt+yield+emiss ion_O2+rab+Thr+PO4.ferm.+PO4.apporte+PO4.utilise; 0.400, 0. 728, pH+Nitrogen.Conc.+OD+X.F.Suc+X.VXdt+yield+PL+rab+Thr+H is+lactate+PO4.ferm.+PO4.apporte+PO4.utilise; 0.434, 0.728, pH+Main.Thr.Conc.+Nitrogen.Conc.+X.F.Suc+X.VXdt+yield+rab+ Thr+PO4.ferm.+PO4.apporte+PO4.utilise; 0.434, 0.728, pH+Nit rogen.Conc.+OD+X.F.Suc+X.VXdt+yield+emission_O2+rab+PO4.fer m.+PO4.apporte+PO4.utilise; 0.444, 0.728, pH+Nitrogen.Conc. +X.F.Suc+X.VXdt+yield+emission_O2+emission_CO2+PO4.ferm.+PO 4.apporte+PO4.utilise; 0.400, 0.728, pH+Nitrogen.Conc.+OD+X. F.Suc+X.VXdt+yield+PL+emission_CO2+rab+Thr+His+PO4.ferm.+PO 4.apporte+PO4.utilise; 0.423, 0.728, pH+Main.P-Source.Conc. +Nitrogen.Conc.+X.F.Suc+X.VXdt+yield+PL+rab+Thr+PO4.ferm.+P O4.apporte+PO4.utilise; 0.423, 0.727, pH+Nitrogen.Conc.+X.F. Suc+X.VXdt+total.cell+yield+emission_CO2+rab+Thr+PO4.ferm.+ PO4.apporte+PO4.utilise; 0.422, 0.727, pH+tmp+Nitrogen.Conc. +X.F.Suc+X.VXdt+total.cell+yield+PL+emission_O2+PO4.ferm.+P O4.apporte+PO4.utilise; 0.411, 0.727, pH+Main.Thr.Conc.+Nit rogen.Conc.+OD+X.F.Suc+X.VXdt+yield+rab+Thr+His+PO4.ferm.+P O4.apporte+PO4.utilise; 0.443, 0.727, pH+Nitrogen.Conc.+X.F. Suc+X.VXdt+yield+emission_O2+His+PO4.ferm.+PO4.apporte+PO4. utilise; 0.399, 0.727, pH+Nitrogen.Conc.+OD+X.F.Suc+X.VXdt+ yield+emission_O2+emission_CO2+rab+Thr+His+PO4.ferm.+PO4.ap porte+PO4.utilise; 0.411, 0.727, pH+P-Source.Feed.cone.+Nit rogen.Conc.+OD+X.F.Suc+X.VXdt+yield+rab+Thr+His+PO4.ferm.+P O4.apporte+PO4.utilise; 0.422, 0.727, pH+tmp+Nitrogen.Conc. +X.F.Suc+X.VXdt+yield+PL+rab+His+PO4.ferm.+PO4.apporte+PO4. utilise; 0.411, 0.727, pH+Nitrogen.Conc.+X.F.Suc+X.VXdt+tot al.cell+yield+PL+rab+Thr+His+PO4.ferm.+PO4.apporte+PO4.util ise; 0.411, 0.727, pH+Nitrogen.Conc.+OD+X.F.Suc+X.VXdt+yiel d+emission_O2+rab+Thr+lactate+PO4.ferm.+PO4.apporte+PO4.uti lise; 0.410, 0.727, pH+Main.P-Source.Conc.+Nitrogen.Conc.+O D+X.F.Suc+X.VXdt+yield+emission_CO2+rab+Thr+PO4.ferm.+PO4.a pporte+PO4.utilise; 0.410, 0.727, pH+P-Source.Feed.conc.+Ni trogen.Conc.+OD+X.F.Suc+X.VXdt+yield+emission_O2+Thr+His+PO 4.ferm.+PO4.apporte+PO4.utilise; 0.399, 0.727, pH+Main.P-So urce.Conc.+Nitrogen.Conc.+OD+X.F.Suc+X.VXdt+yield+PL+rab+Th r+His+PO4.ferm.+PO4.apporte+PO4.utilise; 0.399, 0.727, pH+N itrogen.Conc.+OD+X.F.Suc+X.VXdt+yield+PL+emission_O2+Thr+Hi s+lactate+PO4.ferm.+PO4.apporte+PO4.utilise; 0.432, 0.727, pH+Nitrogen.Conc.+X.F.Suc+X.VXdt+total.cell+yield+emission_ O2+rab+PO4.ferm.+PO4.apporte+PO4.utilise; 0.442, 0.727, pH+ Nitrogen.Conc.+X.F.Suc+X.VXdt+yield+PL+emission_O2+PO4.ferm. +PO4.apporte+PO4.utilise; 0.421, 0.726, pH+Main.P-Source.Co nc.+Nitrogen.Conc.+X.F.Suc+X.VXdt+yield+rab+Thr+His+PO4.fer m.+PO4.apporte+PO4.utilise; 0.410, 0.726, pH+P-Source.Feed. conc.+Nitrogen.Conc.+OD+X.F.Suc+X.VXdt+yield+PL+rab+Thr+PO4. ferm.+PO4.apporte+PO4.utilise; 0.431, 0.726, pH+Main.P-Sour ce.Conc.+Nitrogen.Conc.+OD+X.F.Suc+X.VXdt+yield+rab+PO4.fer m.+PO4.apporte+PO4.utilise; 0.431, 0.726, pH+tmp+Nitrogen.C onc.+X.F.Suc+X.VXdt+yield+rab+Thr+PO4.ferm.+PO4.apporte+PO4. utilise; 0.431, 0.726, pH+Nitrogen.Conc.+OD+X.F.Suc+X.VXdt+ yield+rab+lactate+PO4.ferm.+PO4.apporte+PO4.utilise; 0.409, 0.726, pH+tmp+Nitrogen.Conc.+OD+X.F.Suc+X.VXdt+yield+emiss ion_CO2+Thr+His+PO4.ferm.+PO4.apporte+PO4.utilise; 0.421, 0. 726, pH+Nitrogen.Conc.+X.F.Suc+X.VXdt+yield+PL+emission_O2+ Thr+His+PO4.ferm.+PO4.apporte+PO4.utilise; 0.431, 0.726, pH +Nitrogen.Conc.+OD+X.F.Suc+X.VXdt+yield+emission_CO2+rab+PO 4.ferm.+PO4.apporte+PO4.utilise; 0.409, 0.726, pH+tmp+Nitro gen.Conc.+X.F.Suc+X.VXdt+yield+PL+emission_O2+Thr+His+PO4.f erm.+PO4.apporte+PO4.utilise; 0.431, 0.726, pH+Nitrogen.Con c.+X.F.Suc+X.VXdt+yield+rab+His+lactate+PO4.ferm.+PO4.appor te+PO4.utilise; 0.409, 0.726, pH+Nitrogen.Conc.+OD+X.F.Suc+ X.VXdt+yield+emission_CO2+rab+Thr+lactate+PO4.ferm.+PO4.app orte+PO4.utilise; 0.431, 0.726, pH+Main.P-Source.Conc.+Nitr ogen.Conc.+X.F.Suc+X.VXdt+yield+PL+rab+PO4.ferm.+PO4.apport e+PO4.utilise; 0.420, 0.726, pH+Nitrogen.Conc.+X.F.Suc+X.VX dt+total.cell+yield+emission_O2+emission_CO2+Thr+PO4.ferm.+ PO4.apporte+PO4.utilise; 0.397, 0.726, pH+Nitrogen.Conc.+OD +X.F.Suc+X.VXdt+yield+PL+emission_O2+emission_CO2+Thr+His+P O4.ferm.+PO4.apporte+PO4.utilise; 0.430, 0.726, pH+Nitrogen. Conc.+X.F.Suc+X.VXdt+yield+PL+emission_O2+rab+PO4.ferm.+PO4. apporte+PO4.utilise; 0.420, 0.726, pH+Nitrogen.Conc.+OD+X.F. Suc+X.VXdt+yield+PL+rab+His+PO4.ferm.+PO4.apporte+PO4.utili se; 0.408, 0.726, pH+Main.P-Source.Conc.+Nitrogen.Conc.+OD+ X.F.Suc+X.VXdt+yield+emission_O2+Thr+lactate+PO4.ferm.+PO4. apporte+PO4.utilise; 0.420, 0.726, pH+tmp+Nitrogen.Conc.+X. F.Suc+X.VXdt+yield+rab+Thr+His+PO4.ferm.+PO4.apporte+PO4.ut ilise; 0.408, 0.726, pH+Main.P-Source.Conc.+Nitrogen.Conc.+ OD+X.F.Suc+X.VXdt+yield+emission_O2+emission_CO2+Thr+PO4.fe rm.+PO4.apporte+PO4.utilise; 0.441, 0.726, pH+tmp+Nitrogen. Conc.+X.VXdt+total.cell+yield+PL+emission_CO2+Thr+His; 0.43 0, 0.726, pH+P-Source.Feed.conc.+Nitrogen.Conc.+X.F.Suc+X.V Xdt+yield+emission_O2+Thr+PO4.ferm.+PO4.apporte+PO4.utilis e; 0.408, 0.726, pH+Main.Thr.Conc.+Nitrogen.Conc.+OD+X.F.Su c+X.VXdt+yield+PL+rab+Thr+PO4.ferm.+PO4.apporte+PO4.utilis e; 0.419, 0.726, pH+Nitrogen.Conc.+X.F.Suc+X.VXdt+yield+PL+ emission_O2+rab+Thr+PO4.ferm.+PO4.apporte+PO4.utilise; 0.43 0, 0.726, pH+Nitrogen.Conc.+OD+X.F.Suc+X.VXdt+yield+emissio n O2+His+PO4.ferm.+PO4.apporte+PO4.utilise; 0.430, 0.725, p H+Main.P-Source.Conc.+Nitrogen.Conc.+X.F.Suc+X.VXdt+yield+e mission_O2+Thr+PO4.ferm.+PO4.apporte+PO4.utilise; 0.408, 0. 725, pH+tmp+Nitrogen.Cone.+OD+X.F.Suc+X.VXdt+yield+emission O2+Thr+His+PO4.ferm.+PO4.apporte+PO4.utilise; 0.419, 0.725, pH+Nitrogen.Conc.+X.F.Suc+X.VXdt+yield+emission_O2+rab+Thr +His+PO4.ferm.+PO4.apporte+PO4.utilise; 0.396, 0.725, pH+Ni trogen.Conc.+OD+X.F.Suc+X.VXdt+yield+PL+emission_O2+emissio n_CO2+rab+Thr+PO4.ferm.+PO4.apporte+PO4.utilise; 0.408, 0.7 25, pH+Main.Thr.Cone.+Nitrogen.Cone.+OD+X.F.Suc+X.VXdt+yiel d+emission_O2+Thr+His+PO4.ferm.+PO4.apporte+PO4.utilise; 0. 396, 0.725, pH+tmp+Nitrogen.Conc.+X.F.Suc+X.VXdt+total.cell +yield+emission_O2+rab+Thr+His+PO4.ferm.+PO4.apporte+PO4.ut ilise; 0.429, 0.725, pH+Nitrogen.Conc.+X.F.Suc+X.VXdt+total. cell+yield+rab+lactate+PO4.ferm.+PO4.apporte+PO4.utilise; 0. 407, 0.725, pH+Nitrogen.Conc.+OD+X.F.Suc+X.VXdt+yield+emiss ion_O2+emission_CO2+Thr+lactate+PO4.ferm.+PO4.apporte+PO4.u tilise; 0.429, 0.725, pH+Main.P-Source.Conc.+Nitrogen.Conc. +X.F.Suc+X.VXdt+total.cell+yield+rab+PO4.ferm.+PO4.apporte+ PO4.utilise; 0.440, 0.725, pH+P-Source.Feed.conc.+Nitrogen. Conc.+X.F.Suc+X.VXdt+yield+rab+PO4.ferm.+PO4.apporte+PO4.ut ilise; 0.407, 0.725, pH+P-Source.Feed.conc.+Nitrogen.Conc.+ OD+X.F.Suc+X.VXdt+yield+PL+emission_O2+Thr+PO4.ferm.+PO4.ap porte+PO4.utilise; 0.418, 0.725, pH+Main.Thr.Conc.+Nitrogen. Conc.+X.F.Suc+X.VXdt+yield+rab+Thr+His+PO4.ferm.+PO4.apport e+PO4.utilise; 0.418, 0.725, pH+Nitrogen.Conc.+OD+X.F.Suc+X. VXdt+yield+rab+Thr+SO4.ferm.+PO4.ferm.+PO4.apporte+PO4.util ise; 0.407, 0.725, pH+tmp+Nitrogen.Conc.+X.F.Suc+X.VXdt+yie ld+PL+emission_CO2+Thr+His+PO4.ferm.+PO4.apporte+PO4.utilis e; 0.429, 0.725, pH+Nitrogen.Conc.+OD+X.F.Suc+X.VXdt+yield+ emission_O2+emission_CO2+PO4.ferm.+PO4.apporte+PO4.utilise; 0.429, 0.725, pH+Nitrogen.Conc.+X.F.Suc+X.VXdt+total.cell+ yield+emission_CO2+rab+PO4.ferm.+PO4.apporte+PO4.utilise; 0. 395, 0.725, pH+Main.P-Source.Conc.+Nitrogen.Conc.+OD+X.F.Su c+X.VXdt+yield+PL+emission_O2+Thr+His+PO4.ferm.+PO4.apporte +PO4.utilise; 0.395, 0.725, pH+tmp+Nitrogen.Conc.+X.F.Suc+X. VXdt+total.cell+yield+emission_CO2+rab+Thr+His+PO4.ferm.+PO 4.apporte+PO4.utilise; 0.429, 0.725, pH+Nitrogen.Conc.+X.F. Suc+X.VXdt+yield+emission_O2+rab+His+PO4.ferm.+PO4.apporte+ PO4.utilise; 0.418, 0.725, pH+tmp+Nitrogen.Conc.+X.F.Suc+X. VXdt+yield+PL+emission_O2+Thr+PO4.ferm.+PO4.apporte+PO4.uti lise; 0.407, 0.725, pH+P-Source.Feed.conc.+Nitrogen.Conc.+X. F.Suc+X.VXdt+total.cell+yield+PL+rab+Thr+PO4.ferm.+PO4.appo rte+PO4.utilise; 0.428, 0.725, pH+tmp+Nitrogen.Conc.+X.F.Su c+X.VXdt+yield+emission_CO2+His+PO4.ferm.+PO4.apporte+PO4.u tilise; 0.418, 0.725, pH+Nitrogen.Conc.+X.F.Suc+X.VXdt+tota l.cell+yield+emission_O2+Thr+His+PO4.ferm.+PO4.apporte+PO4. utilise; 0.428, 0.725, pH+Main.P-Source.Conc.+Nitrogen.Conc. +X.F.Suc+X.VXdt+yield+rab+His+PO4.ferm.+PO4.apporte+PO4.uti lise; 0.418, 0.725, pH+tmp+Nitrogen.Conc.+OD+X.F.Suc+X.VXdt +yield+emission_O2+Thr+PO4.ferm.+PO4.apporte+PO4.utilise; 0. 406, 0.725, pH+Nitrogen.Conc.+X.F.Suc+X.VXdt+total.cell+yie ld+PL+emission_O2+rab+Thr+PO4.ferm.+PO4.apporte+PO4.utilis e; 0.418, 0.725, pH+Main.Thr.Conc.+Nitrogen.Conc.+X.F.Suc+X. VXdt+total.cell+yield+rab+Thr+PO4.ferm.+PO4.apporte+PO4.uti lise; 0.428, 0.725, pH+Nitrogen.Conc.+X.F.Suc+X.VXdt+yield+ PL+emission_CO2+rab+PO4.ferm.+PO4.apporte+PO4.utilise; 0.41 7, 0.725, pH+Nitrogen.Conc.+X.F.Suc+X.VXdt+yield+PL+emissio n_CO2+rab+Thr+PO4.ferm.+PO4.apporte+PO4.utilise; 0.417, 0.7 25, pH+Main.P-Source.Conc.+Nitrogen.Conc.+X.F.Suc+X.VXdt+yi eld+rab+Thr+lactate+PO4.ferm.+PO4.apporte+PO4.utilise; 0.40 6, 0.724, pH+Main.P-Source.Conc.+Nitrogen.Conc.+X.F.Suc+X.V Xdt+total.cell+yield+PL+rab+Thr+PO4.ferm.+PO4.apporte+PO4.u tilise; 0.406, 0.724, pH+P-Source.Feed.conc.+Nitrogen.Conc. +X.F.Suc+X.VXdt+total.cell+yield+rab+Thr+His+PO4.ferm.+PO4. apporte+PO4.utilise; 0.428, 0.724, pH+Nitrogen.Conc.+OD+X.F. Suc+X.VXdt+yield+PL+emission_O2+PO4.ferm.+PO4.apporte+PO4.u tilise; 0.417, 0.724, pH+P-Source.Feed.conc.+Nitrogen.Conc. +X.F.Suc+X.VXdt+yield+rab+Thr+His+PO4.ferm.+PO4.apporte+PO4. utilise; 0.417, 0.724, pH+Nitrogen.Conc.+X.F.Suc+X.VXdt+yie ld+PL+emission_O2+Thr+lactate+PO4.ferm.+PO4.apporte+PO4.uti lise; 0.417, 0.724, pH+tmp+Nitrogen.Conc.+X.F.Suc+X.VXdt+to tal.cell+yield+emission_CO2+Thr+PO4.ferm.+PO4.apporte+PO4.u tilise; 0.417, 0.724, pH+Nitrogen.Conc.+X.F.Suc+X.VXdt+tota l.cell+yield+PL+emission_O2+Thr+PO4.ferm.+PO4.apporte+PO4.u tilise; 0.428, 0.724, pH+Nitrogen.Conc.+X.F.Suc+X.VXdt+yiel d+emission_O2+rab+lactate+PO4.ferm.+PO4.apporte+PO4.utilis e; 0.438, 0.724, pH+Nitrogen.Conc.+X.F.Suc+X.VXdt+yield+emi ssion_O2+lactate+PO4.ferm.+PO4.apporte+PO4.utilise; 0.406, 0.724, pH+tmp+Nitrogen.Conc.+X.F.Suc+X.VXdt+total.cell+yiel d+emission_O2+rab+Thr+PO4.ferm.+PO4.apporte+PO4.utilise; 0. 417, 0.724, pH+Nitrogen.Conc.+X.F.Suc+X.VXdt+yield+emission CO2+rab+Thr+His+PO4.ferm.+PO4.apporte+PO4.utilise; 0.405, 0.724, pH+Nitrogen.Conc.+X.F.Suc+X.VXdt+yield+PL+rab+Thr+Hi s+lactate+PO4.ferm.+PO4.apporte+PO4.utilise; 0.417, 0.724, pH+P-Source.Feed.conc.+Nitrogen.Conc.+X.F.Suc+X.VXdt+yield+ PL+rab+Thr+PO4.ferm.+PO4.apporte+PO4.utilise; 0.394, 0.724, pH+tmp+Nitrogen.Conc.+OD+X.F.Suc+X.VXdt+yield+PL+emission_ CO2+Thr+His+PO4.ferm.+PO4.apporte+PO4.utilise; 0.405, 0.724, pH+Nitrogen.Conc.+X.F.Suc+X.VXdt+total.cell+yield+PL+rab+T hr+lactate+PO4.ferm.+PO4.apporte+PO4.utilise; 0.438, 0.724, pH+Nitrogen.Conc.+X.F.Suc+X.VXdt+total.cell+yield+emission O2+PO4.ferm.+PO4.apporte+PO4.utilise; 0.416, 0.724, pH+P-S ource.Feed.conc.+Nitrogen.Conc.+X.F.Suc+X.VXdt+total.cell+y ield+emission_O2+Thr+PO4.ferm.+PO4.apporte+PO4.utilise; 0.4 16, 0.724, pH+Nitrogen.Conc.+X.F.Suc+X.VXdt+yield+PL+emissi on_O2+emission_CO2+Thr+PO4.ferm.+PO4.apporte+PO4.utilise; 0. 448, 0.724, pH+tmp+Nitrogen.Conc.+X.VXdt+total.cell+yield+e mission_O2+Thr+His; 0.416, 0.724, pH+Nitrogen.Conc.+X.F.Suc +X.VXdt+total.cell+yield+PL+rab+His+PO4.ferm.+PO4.apporte+P O4.utilise; 0.427, 0.724, pH+tmp+Nitrogen.Conc.+X.F.Suc+X.V Xdt+yield+emission_O2+rab+PO4.ferm.+PO4.apporte+PO4.utilis e; 0.393, 0.724, pH+tmp+Nitrogen.Conc.+X.F.Suc+X.VXdt+total. cell+yield+emission_O2+emission_CO2+Thr+His+PO4.ferm.+PO4.a pporte+PO4.utilise; 0.393, 0.724, pH+Main.P-Source.Conc.+Ni trogen.Conc.+OD+X.F.Suc+X.VXdt+yield+PL+rab+Thr+lactate+PO4. ferm.+PO4.apporte+PO4.utilise; 0.416, 0.724, pH+Nitrogen.Co nc.+X.F.Suc+X.VXdt+yield+emission_O2+Thr+His+lactate+PO4.fe rm.+PO4.apporte+PO4.utilise; 0.427, 0.724, pH+Nitrogen.Conc. +X.F.Suc+X.VXdt+yield+PL+emission_O2+His+PO4.ferm.+PO4.appo rte+PO4.utilise; 0.457, 0.724, pH+tmp+X.VXdt+total.cell+yie ld+emission_CO2+Thr+His; 0.416, 0.724, pH+Nitrogen.Conc.+X. F.Suc+X.VXdt+yield+emission_O2+rab+Thr+lactate+PO4.ferm.+PO 4.apporte+PO4.utilise; 0.426, 0.723, pH+Nitrogen.Conc.+X.F. Suc+X.VXdt+yield+PL+emission_O2+emission_CO2+PO4.ferm.+PO4. apporte+PO4.utilise; 0.437, 0.723, pH+Main.Thr.Conc.+Nitrog en.Conc.+X.F.Suc+X.VXdt+yield+rab+PO4.ferm.+PO4.apporte+PO4. utilise; 0.380, 0.723, pH+tmp+Nitrogen.Conc.+X.F.Suc+X.VXdt +total.cell+yield+PL+emission_O2+rab+Thr+His+PO4.ferm.+PO4. apporte+PO4.utilise; 0.404, 0.723, pH+Main.P-Source.Conc.+M ain.Thr.Conc.+Nitrogen.Conc.+OD+X.F.Suc+X.VXdt+yield+rab+Th r+PO4.ferm.+PO4.apporte+PO4.utilise; 0.404, 0.723, pH+Nitro gen.Conc.+X.F.Suc+X.VXdt+total.cell+yield+emission_O2+rab+T hr+His+PO4.ferm.+PO4.apporte+PO4.utilise; 0.404, 0.723, pH+ Nitrogen.Conc.+X.F.Suc+X.VXdt+total.cell+yield+rab+Thr+His+ lactate+PO4.ferm.+PO4.apporte+PO4.utilise; 0.426, 0.723, pH +tmp+Nitrogen.Conc.+X.F.Suc+yield+emission_CO2+Thr+His+PO4. ferm.+PO4.apporte+PO4.utilise; 0.380, 0.723, pH+Nitrogen.Co nc.+OD+X.F.Suc+X.VXdt+yield+PL+emission_O2+emission_CO2+rab +Thr+His+PO4.ferm.+PO4.apporte+PO4.utilise; 0.426, 0.723, p H+Nitrogen.Conc.+X.F.Suc+X.VXdt+total.cell+yield+emission_O 2+emission-CO2+PO4.ferm.+PO4.apporte+PO4.utilise; 0.415, 0. 723, pH+Nitrogen.Conc.+OD+X.F.Suc+X.VXdt+yield+PL+emission_ O2+rab+PO4.ferm.+PO4.apporte+PO4.utilise; 0.426, 0.723, pH+ Nitrogen.Conc.+X.F.Suc+X.VXdt+yield+emission_CO2+rab+His+PO 4.ferm.+PO4.apporte+PO4.utilise; 0.426, 0.723, pH+tmp+Main. P-Source.Conc.+Nitrogen.Conc.+X.F.Suc+X.VXdt+yield+rab+P04. ferm.+PO4.apporte+PO4.utilise; 0.404, 0.723, pH+Nitrogen.Co nc.+X.F.Suc+X.VXdt+total.cell+yield+PL+emission_CO2+rab+Thr +PO4.ferm.+PO4.apporte+PO4.utilise; 0.426, 0.723, pH+Main.T hr.Conc.+Nitrogen.Conc.+X.F.Suc+X.VXdt+yield+emission_O2+Th r+PO4.ferm.+PO4.apporte+PO4.utilise; 0.415, 0.723, pH+Nitro gen.Conc.+X.F.Suc+X.VXdt+yield+emission_O2+emission_CO2+Thr +His+PO4.ferm.+PO4.apporte+PO4.utilise; 0.392, 0.723, pH+Ma in.P-Source.Conc.+Nitrogen.Conc.+OD+X.F.Suc+X.VXdt+yield+PL +emission_O2+rab+Thr+PO4.ferm.+PO4.apporte+PO4.utilise; 0.4 26, 0.723, pH+Main.P-Source.Conc.+Nitrogen.Conc.+X.F.Suc+X. VXdt+yield+emission_O2+rab+PO4.ferm.+PO4.apporte+PO4.utilis e; 0.415, 0.723, pH+tmp+Nitrogen.Conc.+X.F.Suc+X.VXdt+yield +emission_O2+rab+His+PO4.ferm.+PO4.apporte+PO4.utilise; 0.4 03, 0.723, pH+tmp+Nitrogen.Conc.+X.F.Suc+X.VXdt+total.cell+ yield+emission_CO2+rab+Thr+PO4.ferm.+PO4.apporte+PO4.utilis e; 0.415, 0.723, pH+Main.P-Source.Conc.+Main.Thr.Conc.+Nitr ogen.Conc.+X.F.Suc+X.VXdt+yield+rab+Thr+PO4.ferm.+PO4.appor te+PO4.utilise; 0.379, 0.723, pH+tmp+Nitrogen.Conc.+X.F.Suc +X.VXdt+total.cell+yield+PL+emission_CO2+rab+Thr+His+PO4.fe rm.+PO4.apporte+PO4.utilise; 0.425, 0.723, pH+Main.P-Source. Conc.+Nitrogen.Conc.+X.F.Suc+X.VXdt+yield+rab+lactate+PO4.f erm.+PO4.apporte+PO4.utilise; 0.403, 0.723, pH+Main.Thr.Con c.+Nitrogen.Conc.+OD+X.F.Suc+X.VXdt+yield+PL+emission_O2+Th r+PO4.ferm.+PO4.apporte+PO4.utilise; 0.403, 0.723, pH+Main. P-Source.Cone.+Nitrogen.Cone.+X.F.Suc+X.VXdt+total.cell+yie ld+rab+Thr+His+PO4.ferm.+PO4.apporte+PO4.utilise; 0.415, 0. 723, pH+tmp+Nitrogen.Conc.+X.F.Suc+X.VXdt+total.cell+yield+ emission_O2+rab+PO4.ferm.+PO4.apporte+PO4.utilise; 0.392, 0. 723, pH+tmp+Nitrogen.Conc.+OD+X.F.Suc+X.VXdt+yield+PL+emiss ion_O2+Thr+His+PO4.ferm.+PO4.apporte+PO4.utilise; 0.414, 0. 723, pH+tmp+Nitrogen.Cone.+X.F.Suc+X.VXdt+yield+PL+rab+Thr+ PO4.ferm.+PO4.apporte+PO4.utilise; 0.391, 0.723, pH+Nitroge n.Conc.+OD+X.F.Suc+X.VXdt+yield+PL+emission_O2+rab+Thr+lact ate+PO4.ferm.+PO4.apporte+PO4.utilise; 0.391, 0.723, pH+Mai n.P-Source.Conc.+Nitrogen.Conc.+OD+X.F.Suc+X.VXdt+yield+rab +Thr+His+lactate+PO4.ferm.+PO4.apporte+PO4.utilise; 0.425, 0.723, pH+Nitrogen.Conc.+X.F.Suc+X.VXdt+yield+emission_CO2+ rab+lactate+PO4.ferm.+PO4.apporte+PO4.utilise; 0.403, 0.723, pH+P-Source.Feed.conc.+Nitrogen.Conc.+OD+X.F.Suc+X.VXdt+yi eld+emission_O2+rab+Thr+PO4.ferm.+PO4.apporte+PO4.utilise; 0.425, 0.723, pH+tmp+Nitrogen.Conc.+X.F.Suc+X.VXdt+yield+em ission_CO2+rab+PO4.ferm.+PO4.apporte+PO4.utilise; 0.414, 0. 723, pH+Nitrogen.Conc.+OD+X.F.Suc+X.VXdt+yield+emission_O2+ rab+His+PO4.ferm.+PO4.apporte+PO4.utilise; 0.414, 0.723, pH +tmp+Main.P-Source.Conc.+Nitrogen.Conc.+X.F.Suc+X.VXdt+tota l.cell+yield+rab+PO4.ferm.+PO4.apporte+PO4.utilise; 0.435, 0.723, pH+tmp+Nitrogen.Conc.+X.VXdt+total.cell+yield+PL+emi ssion_O2+Thr+His; 0.414, 0.723, pH+Main.P-Source.Conc.+Nitr ogen.Conc.+X.F.Suc+X.VXdt+yield+emission_O2+rab+Thr+PO4.fer m.+PO4.apporte+PO4.utilise; 0.414, 0.723, pH+Main.Thr.Conc. +Nitrogen.Conc.+X.F.Suc+X.VXdt+yield+rab+Thr+lactate+PO4.fe rm.+PO4.apporte+PO4.utilise; 0.391, 0.723, pH+P-Source.Feed. onc.+Nitrogen.Conc.+OD+X.F.Suc+X.VXdt+yield+PL+rab+Thr+His +PO4.ferm.+PO4.apporte+PO4.utilise; 0.403, 0.723, pH+tmp+Ni trogen.Conc.+X.F.Suc+X.VXdt+yield+PL+rab+Thr+His+PO4.ferm.+ PO4.apporte+PO4.utilise; 0.403, 0.723, pH+Main.Thr.Conc.+Ni trogen.Conc.+OD+X.F.Suc+X.VXdt+yield+emission_O2+rab+Thr+PO 4.ferm.+PO4.apporte+PO4.utilise; 0.414, 0.723, pH+P-Source. Feed.conc.+Nitrogen.Conc.+X.F.Suc+X.VXdt+yield+emission_O2+ Thr+His+PO4.ferm.+PO4.apporte+PO4.utilise; 0.435, 0.723, pH +tmp+X.F.Suc+yield+emission_CO2+Thr+His+PO4.ferm.+PO4.appor te+PO4.utilise; 0.414, 0.723, pH+Nitrogen.Conc.+X.F.Suc+X.V Xdt+yield+emission_CO2+rab+Thr+lactate+PO4.ferm.+PO4.apport e+PO4.utilise; 0.403, 0.723, pH+Main.Thr.Conc.+Nitrogen.Con c.+OD+X.F.Suc+X.VXdt+yield+rab+Thr+lactate+PO4.ferm.+PO4.ap porte+PO4.utilise; 0.414, 0.723, pH+tmp+Nitrogen.Conc.+OD+X. F.Suc+X.VXdt+yield+rab+Thr+PO4.ferm.+PO4.apporte+PO4.utilis e; 0.414, 0.722, pH+Nitrogen.Conc.+X.F.Suc+X.VXdt+yield+PL+ rab+His+lactate+PO4.ferm.+PO4.apporte+PO4.utilise; 0.414, 0. 722, pH+Nitrogen.Conc.+X.F.Suc+X.VXdt+total.cell+yield+PL+e mission_O2+rab+PO4.ferm.+PO4.apporte+PO4.utilise; 0.414, 0. 722, pH+Nitrogen.Conc.+OD+X.F.Suc+X.VXdt+yield+PL+rab+lacta te+PO4.ferm.+PO4.apporte+PO4.utilise; 0.391, 0.722, pH+P-So urce.Feed.conc.+Nitrogen.Conc.+OD+X.F.Suc+X.VXdt+yield+PL+e mission_O2+Thr+His+PO4.ferm.+PO4.apporte+PO4.utilise; 0.391, 0.722, pH+Main.P-Source.Conc.+Nitrogen.Conc.+OD+X.F.Suc+X. VXdt+yield+emission_O2+rab+Thr+His+PO4.ferm.+PO4.apporte+PO 4.utilise; 0.402, 0.722, pH+Main.P-Source.Cone.+Nitrogen.Co nc.+X.F.Suc+X.VXdt+yield+PL+rab+Thr+His+PO4.ferm.+PO4.appor te+PO4.utilise; 0.413, 0.722, pH+tmp+Nitrogen.Conc.+X.F.Suc +X.VXdt+yield+emission_CO2+rab+His+PO4.ferm.+PO4.apporte+PO 4.utilise; 0.424, 0.722, pH+tmp+Main.P-Source.Conc.+Nitroge n.Conc.+X.F.Suc+X.VXdt+yield+emission_O2+PO4.ferm.+PO4.appo rte+PO4.utilise; 0.390, 0.722, pH+Main.Thr.Conc.+Nitrogen.C onc.+OD+X.F.Suc+X.VXdt+yield+PL+rab+Thr+His+PO4.ferm.+PO4.a pporte+PO4.utilise; 0.390, 0.722, pH+Main.P-Source.Conc.+Ni trogen.Conc.+OD+X.F.Suc+X.VXdt+yield+PL+emission_CO2+rab+Th r+PO4.ferm.+PO4.apporte+PO4.utilise; 0.402, 0.722, pH+tmp+N itrogen.Conc.+X.F.Suc+X.VXdt+total.cell+yield+emission_O2+e mission_CO2+Thr+PO4.ferm.+PO4.apporte+PO4.utilise; 0.413, 0. 722, pH+Main.Thr.Conc.+Nitrogen.Conc.+X.F.Suc+X.VXdt+yield+ PL+rab+Thr+PO4.ferm.+PO4.apporte+PO4.utilise; 0.390, 0.722, pH+Nitrogen.Conc.+OD+X.F.Suc+X.VXdt+yield+PL+emission_CO2+ rab+Thr+lactate+PO4.ferm.+PO4.apporte+PO4.utilise; 0.390, 0. 722, pH+Nitrogen.Conc.+OD+X.F.Suc+X.VXdt+yield+emission_O2+ rab+Thr+His+lactate+PO4.ferm.+PO4.apporte+PO4.utilise; 0.42 4, 0.722, pH+P-Source.Feed.conc.+Nitrogen.Conc.+X.F.Suc+X.V Xdt+total.cell+yield+rab+PO4.ferm.+PO4.apporte+PO4.utilise; 0.424, 0.722, pH+tmp+Nitrogen.Conc.+X.F.Suc+X.VXdt+yield+e mission_O2+emission_CO2+PO4.ferm.+PO4.apporte+PO4.utilise; 0.413, 0.722, pH+Main.P-Source.Conc.+Nitrogen.Conc.+OD+X.F. Suc+X.VXdt+yield+PL+rab+PO4.ferm.+PO4.apporte+PO4.utilise; 0.413, 0.722, pH+Nitrogen.Conc.+OD+X.F.Suc+X.VXdt+yield+PL+ emission_CO2+rab+PO4.ferm.+PO4.apporte+PO4.utilise; 0.413, 0.722, pH+tmp+Nitrogen.Conc.+X.F.Suc+X.VXdt+total.cell+yiel d+emission_CO2+His+PO4.ferm.+PO4.apporte+PO4.utilise; 0.413, 0.722, pH+tmp+Nitrogen.Conc.+X.F.Suc+yield+PL+emission_CO2 +Thr+His+PO4.ferm.+PO4.apporte+PO4.utilise; 0.378, 0.722, p H+tmp+Nitrogen.Conc.+X.F.Suc+X.VXdt+total.cell+yield+PL+emi ssion_O2+emission_CO2+Thr+His+PO4.ferm.+PO4.apporte+PO4.uti lise; 0.413, 0.722, pH+Nitrogen.Conc.+X.F.Suc+X.VXdt+yield+ emission_O2+emission_CO2+Thr+lactate+PO4.ferm.+PO4.apporte+ PO4.utilise; 0.423, 0.722, pH+Nitrogen.Conc.+X.F.Suc+X.VXdt +yield+emission_O2+emission_CO2+His+PO4.ferm.+PO4.apporte+P O4.utilise; 0.401, 0.722, pH+Nitrogen.Conc.+X.F.Suc+X.VXdt+ total.cell+yield+PL+emission_O2+emission_CO2+Thr+PO4.ferm.+ PO4.apporte+PO4.utilise; 0.401, 0.722, pH+Main.P-Source.Con c.+Nitrogen.Conc.+X.F.Suc+X.VXdt+total.cell+yield+emission_ O2+rab+Thr+PO4.ferm.+PO4.apporte+PO4.utilise; 0.401, 0.722, pH+tmp+Nitrogen.Conc.+OD+X.F.Suc+X.VXdt+yield+rab+Thr+His+ PO4.ferm.+PO4.apporte+PO4.utilise; 0.434, 0.722, pH+Main.P-Source.Conc.+Nitrogen.Conc.+X.F.Suc+X.VXdt+yield+emission_O 2+PO4.ferm.+PO4.apporte+PO4.utilise; 0.423, 0.722, pH+Main. P-Source.Conc.+Nitrogen.Conc.+X.F.Suc+X.VXdt+yield+emission CO2+rab+PO4.ferm.+PO4.apporte+PO4.utilise; 0.401, 0.722, p H+Main.Thr.Conc.+Nitrogen.Conc.+OD+X.F.Suc+X.VXdt+yield+emi ssion_CO2+rab+Thr+PO4.ferm.+PO4.apporte+PO4.utilise; 0.401, 0.722, pH+Nitrogen.Conc.+X.F.Suc+X.VXdt+total.cell+yield+e mission_CO2+rab+Thr+His+PO4.ferm.+PO4.apporte+PO4.utilise; 0.401, 0.722, pH+tmp+Nitrogen.Conc.+X.F.Suc+X.VXdt+yield+em ission_O2+rab+Thr+His+PO4.ferm.+PO4.apporte+PO4.utilise; 0. 389, 0.722, pH+tmp+Nitrogen.Conc.+X.F.Suc+X.VXdt+total.cell +yield+PL+emission_O2+rab+Thr+PO4.ferm.+PO4.apporte+PO4.uti lise; 0.401, 0.722, pH+P-Source.Feed.conc.+Nitrogen.Conc.+O D+X.F.Suc+X.VXdt+yield+emission_CO2+rab+Thr+PO4.ferm.+PO4.a pporte+PO4.utilise; 0.401, 0.722, pH+Nitrogen.Conc.+X.F.Suc +X.VXdt+total.cell+yield+emission_O2+emission_CO2+rab+Thr+P O4.ferm.+PO4.apporte+PO4.utilise; 0.412, 0.722, pH+Main.Thr. Conc.+Nitrogen.Conc.+X.F.Suc+X.VXdt+yield+emission_O2+Thr+H is+PO4.ferm.+PO4.apporte+PO4.utilise; 0.389, 0.722, pH+Main. P-Source.Conc.+Nitrogen.Conc.+OD+X.F.Suc+X.VXdt+yield+emiss ion_CO2+rab+Thr+His+PO4.ferm.+PO4.apporte+PO4.utilise; 0.41 2, 0.722, pH+tmp+Nitrogen.Conc.+X.F.Suc+X.VXdt+yield+PL+emi ssion_CO2+His+PO4.ferm.+PO4.apporte+PO4.utilise; 0.412, 0.7 22, pH+tmp+Nitrogen.Conc.+X.F.Suc+X.VXdt+total.cell+yield+e mission_CO2+rab+PO4.ferm.+PO4.apporte+PO4.utilise; 0.401, 0. 722, pH+Nitrogen.Conc.+OD+X.F.Suc+X.VXdt+yield+rab+Thr+His+ SO4.ferm.+PO4.ferm.+PO4.apporte+PO4.utilise; 0.412, 0.722, pH+Main.P-Source.Conc.+Nitrogen.Conc.+X.F.Suc+X.VXdt+yield+ emission_CO2+rab+Thr+PO4.ferm.+PO4.apporte+PO4.utilise; 0.4 12, 0.722, pH+Main.P-Source.Conc.+Nitrogen.Conc.+X.F.Suc+X. VXdt+yield+PL+emission_O2+Thr+PO4.ferm.+PO4.apporte+PO4.uti lise; 0.389, 0.722, pH+Main.P-Source.Conc.+Nitrogen.Conc.+O D+X.F.Suc+X.VXdt+yield+emission_O2+Thr+His+lactate+PO4.ferm. +PO4.apporte+PO4.utilise; 0.389, 0.722, pH+Nitrogen.Conc.+O D+X.F.Suc+X.VXdt+yield+emission_CO2+rab+Thr+His+lactate+PO4. ferm.+PO4.apporte+PO4.utilise; 0.412, 0.721, pH+Nitrogen.Co nc.+OD+X.F.Suc+X.VXdt+yield+emission_CO2+rab+His+PO4.ferm.+ PO4.apporte+PO4.utilise; 0.401, 0.721, pH+tmp+Nitrogen.Conc. +OD+X.F.Suc+X.VXdt+yield+PL+emission_O2+Thr+PO4.ferm.+PO4.a pporte+PO4.utilise; 0.412, 0.721, pH+P-Source.Feed.cone.+Ni trogen.Conc.+X.F.Suc+X.VXdt+yield+PL+emission_O2+Thr+PO4.fe rm.+PO4.apporte+PO4.utilise; 0.412, 0.721, pH+Nitrogen.Conc. +X.F.Suc+X.VXdt+total.cell+yield+PL+rab+lactate+PO4.ferm.+P O4.apporte+PO4.utilise; 0.412, 0.721, pH+Nitrogen.Conc.+OD+ X.F.Suc+X.VXdt+yield+PL+emission_O2+His+PO4.ferm.+PO4.appor te+PO4.utilise; 0.401, 0.721, pH+Main.Thr.Conc.+Nitrogen.Co nc.+X.F.Suc+X.VXdt+total.cell+yield+rab+Thr+His+PO4.ferm.+P O4.apporte+PO4.utilise; 0.423, 0.721, pH+Nitrogen.Conc.+X.F. Suc+X.VXdt+yield+emission_O2+emission_CO2+lactate+PO4.ferm. +PO4.apporte+PO4.utilise; 0.400, 0.721, pH+tmp+Nitrogen.Con c.+X.F.Suc+X.VXdt+total.cell+yield+emission_O2+rab+His+PO4. ferm.+PO4.apporte+PO4.utilise; 0.389, 0.721, pH+tmp+Main.P-Source.Conc.+Nitrogen.Conc.+X.F.Suc+X.VXdt+total.cell+yield +emission_O2+Thr+His+PO4.ferm.+PO4.apporte+PO4.utilise; 0.4 12, 0.721, pH+Main.P-Source.Conc.+Nitrogen.Conc.+X.F.Suc+X. VXdt+yield+emission_O2+Thr+His+PO4.ferm.+PO4.apporte+PO4.ut ilise; 0.423, 0.721, pH+tmp+X.F.Suc+yield+PL+emission_CO2+T hr+His+PO4.ferm.+PO4.apporte+PO4.utilise; 0.443, 0.721, pH+ tmp+X.VXdt+total.cell+yield+PL+emission_CO2+Thr+His; 0.412, 0.721, pH+tmp+Nitrogen.Conc.+X.F.Suc+X.VXdt+yield+emission _O2+emission_CO2+His+PO4.ferm.+PO4.apporte+PO4.utilise; 0.4 12, 0.721, pH+tmp+Main.P-Source.Conc.+Nitrogen.Conc.+X.F.Su c+X.VXdt+yield+emission_O2+His+PO4.ferm.+PO4.apporte+PO4.ut ilise; 0.389, 0.721, pH+Main.P-Source.Conc.+Nitrogen.Conc.+ OD+X.F.Suc+X.VXdt+yield+PL+emission_O2+Thr+lactate+PO4.ferm. +PO4.apporte+PO4.utilise; 0.400, 0.721, pH+Nitrogen.Conc.+X. F.Suc+X.VXdt+yield+PL+emission_O2+rab+Thr+His+PO4.ferm.+PO4. apporte+PO4.utilise; 0.400, 0.721, pH+Main.P-Source.Conc.+M ain.Thr.Conc.+Nitrogen.Conc.+OD+X.F.Suc+X.VXdt+yield+emissi on_O2+Thr+PO4.ferm.+PO4.apporte+PO4.utilise; 0.400, 0.721, pH+tmp+Main.P-Source.Conc.+Nitrogen.Conc.+X.F.Suc+X.VXdt+to tal.cell+yield+emission_O2+Thr+PO4.ferm.+PO4.apporte+PO4.ut ilise; 0.400, 0.721, pH+tmp+Nitrogen.Conc.+X.F.Suc+X.VXdt+t otal.cell+yield+PL+emission_CO2+Thr+PO4.ferm.+PO4.apporte+P O4.utilise; 0.411, 0.721, pH+Nitrogen.Conc.+OD+X.F.Suc+X.VX dt+yield+emission_O2+emission_CO2+rab+PO4.ferm.+PO4.apporte +PO4.utilise; 0.411, 0.721, pH+Nitrogen.Conc.+X.F.Suc+X.VXd t+yield+emission_O2+emission_CO2+rab+Thr+PO4.ferm.+PO4.appo rte+PO4.utilise; 0.411, 0.721, pH+Main.P-Source.Conc.+Nitro gen.Conc.+X.F.Suc+X.VXdt+total.cell+yield+PL+rab+PO4.ferm.+ PO4.apporte+PO4.utilise; 0.400, 0.721, pH+Nitrogen.Conc.+X. F.Suc+X.VXdt+total.cell+yield+PL+emission_O2+Thr+His+PO4.fe rm.+PO4.apporte+PO4.utilise; 0.411, 0.721, pH+Nitrogen.Conc. +OD+X.F.Suc+X.VXdt+yield+rab+His+lactate+PO4.ferm.+PO4.appo rte+PO4.utilise; 0.443, 0.721, pH+tmp+Nitrogen.Conc.+X.VXdt +total.cell+yield+rab+Thr+His; 0.411, 0.721, pH+Main.P-Sour ce.Conc.+Nitrogen.Conc.+X.F.Suc+X.VXdt+yield+emission_O2+em ission_CO2+Thr+PO4.ferm.+PO4.apporte+PO4.utilise; 0.422, 0. 721, pH+Nitrogen.Conc.+X.F.Suc+X.VXdt+yield+emission_O2+emi ssion_CO2+rab+PO4.ferm.+PO4.apporte+PO4.utilise; 0.388, 0.7 21, pH+Nitrogen.Conc.+OD+X.F.Suc+X.VXdt+yield+PL+emission-O 2+emission_CO2+Thr+lactate+PO4.ferm.+PO4.apporte+PO4.utilis e; 0.422, 0.721, pH+Nitrogen.Conc.+X.F.Suc+X.VXdt+total.cel l+yield+emission_O2+His+PO4.ferm.+PO4.apporte+PO4.utilise; 0.400, 0.721, pH+Main.Thr.Conc.+Nitrogen.Conc.+OD+X.F.Suc+X. VXdt+yield+emission_O2+emission_CO2+Thr+PO4.ferm.+PO4.appor te+PO4.utilise; 0.422, 0.721, pH+P-Source.Feed.cone.+Nitrog en.Conc.+X.F.Suc+X.VXdt+yield+PL+rab+PO4.ferm.+PO4.apporte+ PO4.utilise; 0.411, 0.721, pH+Nitrogen.Conc.+X.F.Suc+X.VXdt +yield+PL+emission_O2+rab+His+PO4.ferm.+PO4.apporte+PO4.uti lise; 0.422, 0.721, pH+Nitrogen.Conc.+X.F.Suc+X.VXdt+yield+ PL+emission_O2+lactate+PO4.ferm.+PO4.apporte+PO4.utilise; 0. 422, 0.721, pH+tmp+Nitrogen.Conc.+OD+X.F.Suc+X.VXdt+yield+r ab+PO4.ferm.+PO4.apporte+PO4.utilise; 0.399, 0.721, pH+Main. P-Source.Conc.+P-Source.Feed.conc.+Nitrogen.Conc.+OD+X.F.Su c+X.VXdt+yield+rab+Thr+PO4.ferm.+PO4.apporte+PO4.utilise; 0. 388, 0.721, pH+Main.P-Source.Conc.+Nitrogen.Conc.+OD+X.F.Su c+X.VXdt+yield+PL+emission_O2+emission_CO2+Thr+PO4.ferm.+PO 4.apporte+PO4.utilise; 0.411, 0.721, pH+Nitrogen.Conc.+X.F. Suc+X.VXdt+total.cell+yield+emission_O2+Thr+lactate+PO4.fer m.+PO4.apporte+PO4.utilise; 0.399, 0.721, pH+tmp+Nitrogen.C onc.+X.F.Suc+X.VXdt+yield+emission_CO2+rab+Thr+His+PO4.ferm. +PO4.apporte+PO4.utilise; 0.388, 0.721, pH+tmp+Nitrogen.Con c.+X.F.Suc+X.VXdt+total.cell+yield+rab+Thr+His+SO4.ferm.+PO 4.ferm.+PO4.apporte+PO4.utilise; 0.411, 0.721, pH+Main.P-So urce.Conc.+Nitrogen.Conc.+OD+X.F.Suc+X.VXdt+yield+rab+His+P O4.ferm.+PO4.apporte+PO4.utilise; 0.411, 0.721, pH+Nitrogen. Conc.+X.F.Suc+X.VXdt+total.cell+yield+PL+emission_CO2+rab+P O4.ferm.+PO4.apporte+PO4.utilise; 0.399, 0.721, pH+tmp+Main. P-Source.Conc.+Nitrogen.Conc.+X.F.Suc+X.VXdt+total.cell+yie ld+rab+Thr+PO4.ferm.+PO4.apporte+PO4.utilise; 0.411, 0.721, pH+Main.P-Source.Conc.+Nitrogen.Conc.+X.F.Suc+X.VXdt+yield +PL+rab+His+PO4.ferm.+PO4.apporte+PO4.utilise; 0.399, 0.721, pH+P-Source.Feed.conc.+Nitrogen.Conc.+OD+X.F.Suc+X.VXdt+yi eld+emission_O2+emission_CO2+Thr+PO4.ferm.+PO4.apporte+PO4. utilise; 0.421, 0.721, pH+tmp+Nitrogen.Conc.+X.VXdt+total.c ell+yield+emission_O2+emission_CO2+rab+Thr+His; 0.410, 0.72 1, pH+Nitrogen.Conc.+X.F.Suc+X.VXdt+yield+PL+emission_O2+ra b+lactate+PO4.ferm.+PO4.apporte+PO4.utilise; 0.421, 0.721, pH+Main.P-Source.Conc.+Nitrogen.Conc.+X.F.Suc+X.VXdt+yield+ emission_O2+emission_CO2+PO4.ferm.+PO4.apporte+PO4.utilise; 0.421, 0.721, pH+P-Source.Feed.conc.+Nitrogen.Conc.+X.F.Su c+X.VXdt+yield+rab+His+PO4.ferm.+PO4.apporte+PO4.utilise; 0. 410, 0.721, pH+Nitrogen.Conc.+X.F.Suc+X.VXdt+total.cell+yie ld+emission_O2+rab+His+PO4.ferm.+PO4.apporte+PO4.utilise; 0. 410, 0.721, pH+tmp+Main.P-Source.Conc.+Nitrogen.Conc.+X.F.S uc+X.VXdt+yield+rab+His+PO4.ferm.+PO4.apporte+PO4.utilise; 0.421, 0.721, pH+Nitrogen.Conc.+OD+X.F.Suc+X.VXdt+yield+emi ssion_CO2+Thr+PO4.ferm.+PO4.apporte+PO4.utilise; 0.399, 0.7 21, pH+tmp+Nitrogen.Conc.+X.F.Suc+X.VXdt+total.cell+yield+e mission_CO2+rab+His+PO4.ferm.+PO4.apporte+PO4.utilise; 0.41 0, 0.721, pH+Nitrogen.Conc.+OD+X.F.Suc+X.VXdt+yield+emissio n_O2+Thr+SO4.ferm.+PO4.ferm.+PO4.apporte+PO4.utilise; 0.387, 0.721, pH+tmp+Nitrogen.Conc.+X.F.Suc+X.VXdt+total.cell+yie ld+PL+emission_CO2+rab+Thr+PO4.ferm.+PO4.apporte+PO4.utilis e; 0.421, 0.720, pH+Main.Thr.Conc.+Nitrogen.Conc.+X.F.Suc+X. VXdt+yield+rab+His+PO4.ferm.+PO4.apporte+PO4.utilise; 0.375, 0.720, pH+tmp+Nitrogen.Conc.+X.F.Suc+X.VXdt+total.cell+yie ld+emission_O2+emission_CO2+rab+Thr+His+PO4.ferm.+PO4.appor te+PO4.utilise; 0.421, 0.720, pH+tmp+Nitrogen.Conc.+X.VXdt+ total.cell+yield+PL+emission_O2+Thr+His+PO4.utilise; 0.410, 0.720, pH+tmp+Nitrogen.Conc.+X.F.Suc+X.VXdt+total.cell+yie ld+emission_O2+emission_CO2+PO4.ferm.+PO4.apporte+PO4.utili se; 0.387, 0.720, pH+Nitrogen.Conc.+OD+X.F.Suc+X.VXdt+yield +emission_O2+emission_CO2+Thr+His+lactate+PO4.ferm.+PO4.app orte+PO4.utilise; 0.399, 0.720, pH+Main.Thr.Conc.+Nitrogen. Conc.+OD+X.F.Suc+X.VXdt+yield+emission_O2+Thr+lactate+PO4.f erm.+PO4.apporte+PO4.utilise; 0.410, 0.720, pH+Nitrogen.Con c.+OD+X.F.Suc+X.VXdt+yield+PL+emission_O2+emission_CO2+PO4. ferm.+PO4.apporte+PO4.utilise; 0.410, 0.720, pH+tmp+Main.P-Source.Conc.+Nitrogen.Conc.+X.F.Suc+X.VXdt+total.cell+yield +emission_O2+PO4.ferm.+PO4.apporte+PO4.utilise; 0.421, 0.72 0, pH+tmp+Nitrogen.Conc.+X.VXdt+total.cell+yield+PL+emissio n_CO2+Thr+His+PO4.utilise; 0.410, 0.720, pH+tmp+Nitrogen.Co nc.+OD+X.F.Suc+X.VXdt+yield+emission_O2+His+PO4.ferm.+PO4.a pporte+PO4.utilise; 0.399, 0.720, pH+P-Source.Feed.conc.+Ni trogen.Conc.+X.F.Suc+X.VXdt+total.cell+yield+emission-O2+Th r+His+PO4.ferm.+PO4.apporte+PO4.utilise; 0.399, 0.720, pH+N itrogen.Conc.+X.F.Suc+X.VXdt+total.cell+yield+emission_O2+r ab+Thr+lactate+PO4.ferm.+PO4.apporte+PO4.utilise; 0.421, 0. 720, pH+Nitrogen.Conc.+OD+X.F.Suc+X.VXdt+yield+emission_O2+ lactate+PO4.ferm.+PO4.apporte+PO4.utilise; 0.399, 0.720, pH +Main.P-Source.Conc.+Nitrogen.Conc.+X.F.Suc+X.VXdt+total.ce ll+yield+emission-CO2+rab+Thr+PO4.ferm.+PO4.apporte+PO4.uti lise; 0.410, 0.720, pH+Nitrogen.Conc.+OD+X.F.Suc+X.VXdt+yie ld+emission_CO2+Thr+His+PO4.ferm.+PO4.apporte+PO4.utilise; 0.421, 0.720, pH+tmp+Nitrogen.Conc.+OD+X.F.Suc+X.VXdt+yield +emission_O2+PO4.ferm.+PO4.apporte+PO4.utilise; 0.421, 0.72 0, pH+Nitrogen.Conc.+X.F.Suc+X.VXdt+total.cell+yield+PL+emi ssion_O2+PO4.ferm.+PO4.apporte+PO4.utilise; 0.410, 0.720, p H+tmp+Nitrogen.Conc.+X.F.Suc+X.VXdt+yield+PL+emission_O2+ra b+PO4.ferm.+PO4.apporte+PO4.utilise; 0.387, 0.720, pH+Main. Thr.Conc.+Nitrogen.Conc.+OD+X.F.Suc+X.VXdt+yield+PL+emissio n_O2+Thr+His+PO4.ferm.+PO4.apporte+PO4.utilise; 0.387, 0.72 0, pH+Main.P-Source.Conc.+Nitrogen.Conc.+OD+X.F.Suc+X.VXdt+ yield+emission_O2+emission_CO2+rab+Thr+PO4.ferm.+PO4.apport e+PO4.utilise; 0.398, 0.720, pH+Main.P-Source.Conc.+Nitroge n.Conc.+X.F.Suc+X.VXdt+yield+PL+rab+Thr+lactate+PO4.ferm.+P O4.apporte+PO4.utilise; 0.398, 0.720, pH+P-Source.Feed.conc. +Nitrogen.Conc.+X.F.Suc+X.VXdt+total.cell+yield+emission_O2 +rab+Thr+PO4.ferm.+PO4.apporte+PO4.utilise; 0.386, 0.720, p H+Main.P-Source.Conc.+Nitrogen.Conc.+OD+X.F.Suc+X.VXdt+yiel d+emission_O2+emission_CO2+Thr+His+PO4.ferm.+PO4.apporte+PO 4.utilise; 0.398, 0.720, pH+Nitrogen.Conc.+OD+X.F.Suc+X.VXd t+yield+PL+rab+Thr+SO4.ferm.+PO4.ferm.+PO4.apporte+PO4.util ise; 0.398, 0.720, pH+Main.P-Source.Conc.+Main.Thr.Conc.+Ni trogen.Conc.+X.F.Suc+X.VXdt+yield+rab+Thr+His+PO4.ferm.+PO4. apporte+PO4.utilise; 0.420, 0.720, pH+tmp+Nitrogen.Conc.+X. F.Suc+X.VXdt+yield+emission_CO2+Thr+PO4.ferm.+PO4.apporte+P O4.utilise; 0.398, 0.720, pH+Nitrogen.Conc.+X.F.Suc+X.VXdt+ total.cell+yield+emission_O2+emission_CO2+Thr+His+PO4.ferm. +PO4.apporte+PO4.utilise; 0.398, 0.720, pH+Nitrogen.Conc.+X. F.Suc+X.VXdt+yield+PL+emission O2+Thr+His+lactate+PO4.ferm. +PO4.apporte+PO4.utilise; 0.398, 0.720, pH+Nitrogen.Conc.+X. F.Suc+X.VXdt+yield+PL+emission_CO2+rab+Thr+His+PO4.ferm.+PO 4.apporte+PO4.utilise; 0.398, 0.720, pH+tmp+Nitrogen.Conc.+ X.F.Suc+X.VXdt+yield+emission_O2+emission_CO2+Thr+His+PO4.f erm.+PO4.apporte+PO4.utilise; 0.386, 0.720, pH+P-Source.Fee d.conc.+Nitrogen.Conc.+X.F.Suc+X.VXdt+total.cell+yield+PL+r ab+Thr+His+PO4.ferm.+PO4.apporte+PO4.utilise; 0.420, 0.720, pH+tmp+Nitrogen.Conc.+X.VXdt+total.cell+yield+PL+rab+Thr+H is+PO4.utilise; 0.398, 0.720, pH+tmp+Main.P-Source.Conc.+Ni trogen.Conc.+X.F.Suc+X.VXdt+total.cell+yield+rab+His+PO4.fe rm.+PO4.apporte+PO4.utilise; 0.386, 0.720, pH+tmp+Nitrogen. Conc.+X.F.Suc+X.VXdt+total.cell+yield+emission_O2+Thr+His+S O4.ferm.+PO4.ferm.+PO4.apporte+PO4.utilise; 0.420, 0.720, p H+Nitrogen.Conc.+X.F.Suc+X.VXdt+yield+emission_O2+His+lacta te+PO4.ferm.+PO4.apporte+PO4.utilise; 0.398, 0.720, pH+P-So urce.Feed.conc.+Nitrogen.Conc.+X.F.Suc+X.VXdt+total.cell+yi eld+PL+emission_O2+Thr+PO4.ferm.+PO4.apporte+PO4.utilise; 0. 409, 0.720, pH+tmp+Nitrogen.Conc.+OD+X.F.Suc+X.VXdt+yield+e mission_CO2+Thr+PO4.ferm.+PO4.apporte+PO4.utilise; 0.409, 0. 720, pH+Nitrogen.Conc.+X.F.Suc+X.VXdt+total.cell+yield+rab+ His+lactate+PO4.ferm.+PO4.apporte+PO4.utilise; 0.398, 0.720, pH+P-Source.Feed.conc.+Nitrogen.Conc.+X.F.Suc+X.VXdt+yield +PL+rab+Thr+His+PO4.ferm.+PO4.apporte+PO4.utilise; 0.398, 0. 720, pH+Main.Thr.Conc.+Nitrogen.Conc.+X.F.Suc+X.VXdt+yield+ PL+rab+Thr+His+PO4.ferm.+PO4.apporte+PO4.utilise; 0.398, 0. 720, pH+tmp+Nitrogen.Conc.+X.F.Suc+X.VXdt+yield+PL+emission _O2+rab+His+PO4.ferm.+PO4.apporte+PO4.utilise; 0.398, 0.720, pH+tmp+Nitrogen.Conc.+X.F.Suc+X.VXdt+total.cell+yield+PL+e mission_O2+rab+PO4.ferm.+PO4.apporte+PO4.utilise; 0.431, 0. 720, pH+tmp+Nitrogen.Conc.+X.VXdt+total.cell+yield+PL+rab+T hr+His; 0.409, 0.720, pH+Main.P-Source.Conc.+Nitrogen.Conc. +X.F.Suc+X.VXdt+total.cell+yield+emission_O2+Thr+PO4.ferm.+ PO4.apporte+PO4.utilise; 0.430, 0.720, pH+tmp+Nitrogen.Conc. +X.VXdt+total.cell+yield+emission_CO2+Thr+His+PO4.utilise; 0.398, 0.720, pH+Main.P-Source.Conc.+Main.Thr.Conc.+Nitroge n.Conc.+X.F.Suc+X.VXdt+total.cell+yield+rab+Thr+PO4.ferm.+P O4.apporte+PO4.utilise; 0.386, 0.720, pH+Nitrogen.Conc.+OD+ X.F.Suc+X.VXdt+yield+emission_O2+emission_CO2+rab+Thr+lacta te+PO4.ferm.+PO4.apporte+PO4.utilise; 0.409, 0.720, pH+Nitr ogen.Conc.+OD+X.F.Suc+X.VXdt+yield+emission_O2+emission_CO2 +His+PO4.ferm.+PO4.apporte+PO4.utilise; 0.409, 0.720, pH+Ma in.P-Source.Conc.+Nitrogen.Conc.+X.F.Suc+X.VXdt+total.cell+ yield+emission_O2+rab+PO4.ferm.+PO4.apporte+PO4.utilise; 0. 397, 0.720, pH+P-Source.Feed.conc.+Nitrogen.Conc.+OD+X.F.Su c+X.VXdt+yield+rab+Thr+lactate+PO4.ferm.+PO4.apporte+PO4.ut ilise; 0.409, 0.720, pH+tmp+Nitrogen.Conc.+X.F.Suc+X.VXdt+y ield+emission_O2+rab+Thr+PO4.ferm.+PO4.apporte+PO4.utilise; 0.409, 0.720, pH+P-Source.Feed.conc.+Nitrogen.Conc.+X.F.Su c+X.VXdt+yield+rab+Thr+lactate+PO4.ferm.+PO4.apporte+PO4.ut ilise; 0.397, 0.720, pH+P-Source.Feed.conc.+Nitrogen.Conc.+ OD+X.F.Suc+X.VXdt+yield+emission_O2+Thr+lactate+PO4.ferm.+P O4.apporte+PO4.utilise; 0.385, 0.720, pH+tmp+Nitrogen.Conc. +X.F.Suc+X.VXdt+total.cell+yield+PL+emission_O2+emission_CO 2+Thr+PO4.ferm.+PO4.apporte+PO4.utilise; 0.419, 0.719, pH+M ain.P-Source.Conc.+Nitrogen.Conc.+OD+X.F.Suc+X.VXdt+yield+e mission_O2+PO4.ferm.+PO4.apporte+PO4.utilise; 0.397, 0.719, pH+Main.P-Source.Conc.+Nitrogen.Conc.+X.F.Suc+X.VXdt+total. cell+yield+rab+Thr+lactate+PO4.ferm.+PO4.apporte+PO4.utilis e; 0.397, 0.719, pH+tmp+Nitrogen.Conc.+OD+X.F.Suc+X.VXdt+yi eld+PL+rab+Thr+PO4.ferm.+PO4.apporte+PO4.utilise; 0.397, 0. 719, pH+Nitrogen.Conc.+X.F.Suc+X.VXdt+yield+PL+emission_O2+ rab+Thr+lactate+PO4.ferm.+PO4.apporte+PO4.utilise; 0.408, 0. 719, pH+Main.P-Source.Conc.+Nitrogen.Conc.+OD+X.F.Suc+X.VXd t+yield+emission_O2+rab+PO4.ferm.+PO4.apporte+PO4.utilise; 0.408, 0.719, pH+Nitrogen.Conc.+X.F.Suc+X.VXdt+total.cell+y ield+emission_O2+emission_CO2+rab+PO4.ferm.+PO4.apporte+PO4. utilise; 0.373, 0.719, pH+tmp+Main.P-Source.Conc.+Nitrogen. Conc.+X.F.Suc+X.VXdt+total.cell+yield+PL+emission_O2+Thr+Hi s+PO4.ferm.+PO4.apporte+PO4.utilise; 0.397, 0.719, pH+tmp+M ain.P-Source.Conc.+Nitrogen.Conc.+X.F.Suc+X.VXdt+total.cell +yield+PL+rab+PO4.ferm.+PO4.apporte+PO4.utilise; 0.397, 0.7 19, pH+tmp+Main.P-Source.Conc.+Nitrogen.Conc.+X.F.Suc+X.VXd t+total.cell+yield+emission_O2+His+PO4.ferm.+PO4.apporte+PO 4.utilise; 0.430, 0.719, pH+tmp+P-Source.Feed.conc.+Nitroge n.Conc.+X.VXdt+total.cell+yield+emission_CO2+Thr+His; 0.408, 0.719, pH+Nitrogen.Conc.+X.F.Suc+X.VXdt+yield+PL+emission_ CO2+rab+His+PO4.ferm.+PO4.apporte+PO4.utilise; 0.408, 0.719, pH+Main.P-Source.Conc.+Nitrogen.Conc.+X.F.Suc+X.VXdt+total. cell+yield+rab+His+PO4.ferm.+PO4.apporte+PO4.utilise; 0.385, 0.719, pH+tmp+Main.P-Source.Conc.+Nitrogen.Conc.+X.F.Suc+X. VXdt+total.cell+yield+rab+Thr+His+PO4.ferm.+PO4.apporte+PO4. utilise; 0.408, 0.719, pH+tmp+Main.P-Source.Conc.+Nitrogen. Conc.+X.F.Suc+X.VXdt+yield+PL+rab+PO4.ferm.+PO4.apporte+PO4. utilise; 0.385, 0.719, pH+tmp+Nitrogen.Conc.+OD+X.F.Suc+X.V Xdt+yield+PL+rab+Thr+His+PO4.ferm.+PO4.apporte+PO4.utilise; 0.397, 0.719, pH+tmp+Nitrogen.Conc.+X.F.Suc+X.VXdt+total.c ell+yield+PL+emission_CO2+His+PO4.ferm.+PO4.apporte+PO4.uti lise; 0.397, 0.719, pH+tmp+Nitrogen.Conc.+X.F.Suc+X.VXdt+to tal.cell+yield+emission_O2+emission_CO2+His+PO4.ferm.+PO4.a pporte+PO4.utilise; 0.408, 0.719, pH+tmp+Nitrogen.Conc.+OD+ X.F.Suc+X.VXdt+yield+rab+His+PO4.ferm.+PO4.apporte+PO4.util ise; 0.385, 0.719, pH+Nitrogen.Conc.+X.F.Suc+X.VXdt+total.c ell+yield+PL+emission_O2+rab+Thr+His+PO4.ferm.+PO4.apporte+ PO4.utilise; 0.408, 0.719, pH+P-Source.Feed.conc.+Nitrogen. Conc.+X.F.Suc+X.VXdt+yield+emission_O2+rab+Thr+PO4.ferm.+PO 4.apporte+PO4.utilise; 0.385, 0.719, pH+Nitrogen.Conc.+X.F. Suc+X.VXdt+total.cell+yield+PL+rab+Thr+His+lactate+PO4.ferm. +PO4.apporte+PO4.utilise; 0.408, 0.719, pH+Nitrogen.Conc.+X. F.Suc+X.VXdt+yield+PL+emission_CO2+rab+lactate+PO4.ferm.+PO 4.apporte+PO4.utilise; 0.408, 0.719, pH+tmp+Nitrogen.Conc.+ X.F.Suc+X.VXdt+yield+PL+emission_CO2+rab+PO4.ferm.+PO4.appo rte+PO4.utilise; 0.408, 0.719, pH+Main.P-Source.Conc.+Nitro gen.Conc.+X.F.Suc+X.VXdt+yield+PL+rab+lactate+PO4.ferm.+PO4. apporte+PO4.utilise; 0.429, 0.719, pH+P-Source.Feed.conc.+N itrogen.Conc.+X.F.Suc+X.VXdt+yield+emission_O2+PO4.ferm.+PO 4.apporte+PO4.utilise; 0.396, 0.719, pH+Main.P-Source.Conc. +P-Source.Feed.conc.+Nitrogen.Conc.+OD+X.F.Suc+X.VXdt+yield +emission_O2+Thr+PO4.ferm.+PO4.apporte+PO4.utilise; 0.396, 0.719, pH+tmp+Nitrogen.Conc.+X.F.Suc+X.VXdt+yield+PL+emissi on_CO2+rab+His+PO4.ferm.+PO4.apporte+PO4.utilise; 0.408, 0. 719, pH+Nitrogen.Conc.+X.F.Suc+X.VXdt+total.cell+yield+PL+e mission_O2+emission_CO2+PO4.ferm.+PO4.apporte+PO4.utilise; 0.396, 0.719, pH+Main.Thr.Conc.+Nitrogen.Conc.+X.F.Suc+X.VX dt+total.cell+yield+PL+rab+Thr+PO4.ferm.+PO4.apporte+PO4.ut ilise; 0.396, 0.719, pH+Nitrogen.Conc.+X.F.Suc+X.VXdt+yield +PL+emission_O2+emission_CO2+Thr+His+PO4.ferm.+PO4.apporte+ PO4.utilise; 0.408, 0.719, pH+Main.P-Source.Conc.+Nitrogen. Conc.+X.F.Suc+X.VXdt+yield+PL+emission_O2+rab+PO4.ferm.+PO4. apporte+PO4.utilise; 0.408, 0.719, pH+Nitrogen.Conc.+X.F.Su c+X.VXdt+total.cell+yield+emission_O2+rab+lactate+PO4.ferm. +PO4.apporte+PO4.utilise; 0.418, 0.719, pH+Main.Thr.Conc.+N itrogen.Conc.+OD+X.F.Suc+X.VXdt+yield+rab+PO4.ferm.+PO4.app orte+PO4.utilise; 0.384, 0.719, pH+tmp+Main.P-Source.Conc.+ Nitrogen.Conc.+X.F.Suc+X.VXdt+total.cell+yield+emission_CO2 +Thr+His+PO4.ferm.+PO4.apporte+PO4.utilise; 0.396, 0.719, p H+Main.P-Source.Cone.+Nitrogen.Conc.+X.F.Suc+X.VXdt+total.c ell+yield+emission_O2+emission_CO2+Thr+PO4.ferm.+PO4.apport e+PO4.utilise; 0.407, 0.719, pH+Main.Thr.Conc.+Nitrogen.Con c.+X.F.Suc+X.VXdt+yield+emission_O2+rab+Thr+PO4.ferm.+PO4.a pporte+PO4.utilise; 0.384, 0.719, pH+Main.Thr.Conc.+Nitroge n.Conc.+OD+X.F.Suc+X.VXdt+yield+emission_O2+rab+Thr+His+PO4. ferm.+PO4.apporte+PO4.utilise; 0.359, 0.719, pH+tmp+Nitroge n.Conc.+X.F.Suc+X.VXdt+total.cell+yield+PL+emission_O2+emis sion_CO2+rab+Thr+His+PO4.ferm.+PO4.apporte+PO4.utilise; 0.3 96, 0.719, pH+Nitrogen.Conc.+X.F.Suc+X.VXdt+total.cell+yiel d+emission_CO2+rab+Thr+lactate+PO4.ferm.+PO4.apporte+PO4.ut ilise; 0.407, 0.719, pH+Nitrogen.Conc.+OD+X.F.Suc+X.VXdt+yi eld+emission_O2+rab+lactate+PO4.ferm.+PO4.apporte+PO4.utili se; 0.407, 0.719, pH+Main.P-Source.Conc.+Main.Thr.Conc.+Nit rogen.Conc.+X.F.Suc+X.VXdt+yield+emission_O2+Thr+PO4.ferm.+ PO4.apporte+PO4.utilise; 0.384, 0.719, pH+Main.P-Source.Con c.+Nitrogen.Conc.+X.F.Suc+X.VXdt+total.cell+yield+PL+rab+Th r+His+PO4.ferm.+PO4.apporte+PO4.utilise; 0.429, 0.719, pH+t mp+Nitrogen.Conc.+X.VXdt+total.cell+yield+emission_CO2+Thr+ His+lactate; 0.384, 0.719, pH+tmp+Nitrogen.Conc.+X.F.Suc+X. VXdt+yield+PL+emission_O2+rab+Thr+His+PO4.ferm.+PO4.apporte +PO4.utilise; 0.407, 0.719, pH+tmp+X.F.Suc+X.VXdt+total.cel l+yield+emission_CO2+Thr+His+PO4.ferm.+PO4.apporte+PO4.util ise; 0.384, 0.719, pH+P-Source.Feed.conc.+Nitrogen.Conc.+OD +X.F.Suc+X.VXdt+yield+emission_O2+rab+Thr+His+PO4.ferm.+PO4. apporte+PO4.utilise; 0.384, 0.719, pH+tmp+Nitrogen.Conc.+X. F.Suc+X.VXdt+total.cell+yield+PL+emission_O2+rab+His+PO4.fe rm.+PO4.apporte+PO4.utilise; 0.384, 0.719, pH+Main.P-Source. Conc.+Main.Thr.Conc.+Nitrogen.Conc.+OD+X.F.Suc+X.VXdt+yield +rab+Thr+His+PO4.ferm.+PO4.apporte+PO4.utilise; 0.429, 0.71 9, pH+tmp+Nitrogen.Conc.+X.F.Suc+X.VXdt+yield+emission_CO2+ PO4.ferm.+PO4.apporte+PO4.utilise; 0.418, 0.719, pH+Main.Th r.Conc.+Nitrogen.Conc.+X.F.Suc+X.VXdt+yield+PL+rab+PO4.ferm. +PO4.apporte+PO4.utilise; 0.395, 0.719, pH+P-Source.Feed.co nc.+Nitrogen.Conc.+X.F.Suc+X.VXdt+yield+PL+emission_O2+Thr+ His+PO4.ferm.+PO4.apporte+PO4.utilise; 0.428, 0.719, pH+tmp +Main.P-Source.Conc.+Nitrogen.Conc.+X.VXdt+total.cell+yield +emission_CO2+Thr+His; 0.371, 0.719, pH+Main.P-Source.Conc. +Nitrogen.Conc.+OD+X.F.Suc+X.VXdt+yield+PL+rab+Thr+His+lact ate+PO4.ferm.+PO4.apporte+PO4.utilise; 0.407, 0.719, pH+Nit rogen.Conc.+X.F.Suc+X.VXdt+total.cell+yield+emission_CO2+ra b+His+PO4.ferm.+PO4.apporte+PO4.utilise; 0.407, 0.719, pH+M ain.Thr.Conc.+Nitrogen.Conc.+X.F.Suc+X.VXdt+total.cell+yiel d+emission_O2+Thr+PO4.ferm.+PO4.apporte+PO4.utilise; 0.395, 0.718, pH+Nitrogen.Conc.+OD+X.F.Suc+X.VXdt+yield+PL+emissi on_O2+rab+His+PO4.ferm.+PO4.apporte+PO4.utilise; 0.395, 0.7 18, pH+P-Source.Feed.conc.+Nitrogen.Conc.+X.F.Suc+X.VXdt+to tal.cell+yield+emission_CO2+rab+Thr+PO4.ferm.+PO4.apporte+P O4.utilise; 0.395, 0.718, pH+tmp+Nitrogen.Conc.+X.F.Suc+X.V Xdt+total.cell+yield+PL+emission_CO2+rab+PO4.ferm.+PO4.appo rte+PO4.utilise; 0.395, 0.718, pH+Nitrogen.Conc.+X.F.Suc+X. VXdt+yield+PL+emission_CO2+rab+Thr+lactate+PO4.ferm.+PO4.ap porte+PO4.utilise; 0.418, 0.718, pH+Main.Thr.Conc.+Nitrogen. Conc.+X.F.Suc+X.VXdt+total.cell+yield+rab+PO4.ferm.+PO4.app orte+PO4.utilise; 0.383, 0.718, pH+tmp+Main.P-Source.Conc.+ Nitrogen.Conc.+X.F.Suc+X.VXdt+total.cell+yield+PL+emission_ O2+Thr+PO4.ferm.+PO4.apporte+PO4.utilise; 0.395, 0.718, pH+ Main.P-Source.Conc.+Nitrogen.Conc.+X.F.Suc+X.VXdt+yield+rab +Thr+His+lactate+PO4.ferm.+PO4.apporte+PO4.utilise; 0.406, 0.718, pH+Main.Thr.Conc.+Nitrogen.Conc.+X.F.Suc+X.VXdt+yiel d+emission_O2+Thr+lactate+PO4.ferm.+PO4.apporte+PO4.utilis e; 0.428, 0.718, pH+tmp+Main.P-Source.Conc.+Nitrogen.Conc.+ X.VXdt+total.cell+yield+emission_O2+Thr+His; 0.406, 0.718, pH+tmp+Nitrogen.Conc.+X.F.Suc+X.VXdt+yield+PL+emission_O2+e mission_CO2+PO4.ferm.+PO4.apporte+PO4.utilise; 0.395, 0.718, pH+Main.Thr.Conc.+Nitrogen.Conc.+X.F.Suc+X.VXdt+yield+rab+ Thr+His+lactate+PO4.ferm.+PO4.apporte+PO4.utilise; 0.406, 0. 718, pH+tmp+Main.P-Source.Conc.+Nitrogen.Conc.+X.F.Suc+X.VX dt+yield+PL+emission_O2+PO4.ferm.+PO4.apporte+PO4.utilise; 0.395, 0.718, pH+Nitrogen.Conc.+OD+X.F.Suc+X.VXdt+yield+rab +Thr+lactate+SO4.ferm.+PO4.ferm.+PO4.apporte+PO4.utilise; 0. 395, 0.718, pH+tmp+Nitrogen.Conc.+X.F.Suc+X.VXdt+total.cell +yield+rab+Thr+SO4.ferm.+PO4.ferm.+PO4.apporte+PO4.utilise; 0.417, 0.718, pH+P-Source.Feed.conc.+Nitrogen.Conc.+OD+X.F. Suc+X.VXdt+yield+rab+PO4.ferm.+PO4.apporte+PO4.utilise; 0.4 06, 0.718, pH+tmp+Nitrogen.Conc.+X.F.Suc+X.VXdt+yield+emiss ion_CO2+rab+Thr+PO4.ferm.+PO4.apporte+PO4.utilise; 0.428, 0. 718, pH+tmp+Nitrogen.Conc.+X.VXdt+total.cell+yield+emission _O2+Thr+His+PO4.utilise; 0.383, 0.718, pH+tmp+Nitrogen.Conc. +OD+X.F.Suc+X.VXdt+yield+emission_CO2+rab+Thr+His+PO4.ferm. +PO4.apporte+PO4.utilise; 0.406, 0.718, pH+Main.P-Source.Co nc.+Nitrogen.Conc.+X.F.Suc+X.VXdt+yield+emission_O2+Thr+lac tate+PO4.ferm.+PO4.apporte+PO4.utilise; 0.395, 0.718, pH+Ma in.P-Source.Conc.+Nitrogen.Conc.+X.F.Suc+X.VXdt+yield+PL+em ission_O2+rab+Thr+PO4.ferm.+PO4.apporte+PO4.utilise; 0.406, 0.718, pH+tmp+Nitrogen.Conc.+X.VXdt+total.cell+yield+PL+em ission_O2+emission_CO2+rab+Thr+His; 0.383, 0.718, pH+tmp+Ma in.P-Source.Conc.+Nitrogen.Conc.+X.F.Suc+X.VXdt+total.cell+ yield+PL+rab+Thr+PO4.ferm.+PO4.apporte+PO4.utilise; 0.417, 0.718, pH+Main.P-Source.Conc.+Nitrogen.Conc.+X.F.Suc+X.VXdt +yield+PL+emission_O2+PO4.ferm.+PO4.apporte+PO4.utilise; 0. 383, 0.718, pH+tmp+Nitrogen.Conc.+OD+X.F.Suc+X.VXdt+yield+e mission_O2+rab+Thr+His+PO4.ferm.+PO4.apporte+PO4.utilise; 0. 406, 0.718, pH+Main.P-Source.Conc.+Nitrogen.Conc.+OD+X.F.Su c+X.VXdt+yield+emission_CO2+rab+PO4.ferm.+PO4.apporte+PO4.u tilise; 0.417, 0.718, pH+Main.Thr.Conc.+Nitrogen.Conc.+X.F. Suc+X.VXdt+yield+rab+lactate+PO4.ferm.+PO4.apporte+PO4.util ise; 0.395, 0.718, pH+P-Source.Feed.conc.+Nitrogen.Conc.+X. F.Suc+X.VXdt+total.cell+yield+rab+Thr+lactate+PO4.ferm.+PO4. apporte+PO4.utilise; 0.394, 0.718, pH+tmp+Nitrogen.Conc.+X. F.Suc+X.VXdt+yield+PL+emission_O2+emission_CO2+His+PO4.ferm. +PO4.apporte+PO4.utilise; 0.383, 0.718, pH+Main.Thr.Conc.+N itrogen.Conc.+OD+X.F.Suc+X.VXdt+yield+emission_CO2+rab+Thr+ His+PO4.ferm.+PO4.apporte+PO4.utilise; 0.370, 0.718, pH+Nit rogen.Conc.+OD+X.F.Suc+X.VXdt+yield+PL+emission_O2+rab+Thr+ His+lactate+PO4.ferm.+PO4.apporte+PO4.utilise; 0.383, 0.718, pH+P-Source.Feed.conc.+Nitrogen.Conc.+OD+X.F.Suc+X.VXdt+yi eld+PL+emission_O2+rab+Thr+PO4.ferm.+PO4.apporte+PO4.utilis e; 0.394, 0.718, pH+Nitrogen.Conc.+OD+X.F.Suc+X.VXdt+yield+ emission_O2+Thr+His+SO4.ferm.+PO4.ferm.+PO4.apporte+PO4.uti lise; 0.406, 0.718, pH+Main.Thr.Conc.+Nitrogen.Conc.+X.F.Su c+X.VXdt+yield+emission_CO2+rab+Thr+PO4.ferm.+PO4.apporte+P O4.utilise; 0.406, 0.718, pH+Nitrogen.Conc.+X.F.Suc+X.VXdt+ yield+PL+emission_O2+emission_CO2+His+PO4.ferm.+PO4.apporte +PO4.utilise; 0.382, 0.718, pH+tmp+Nitrogen.Conc.+X.F.Suc+X. VXdt+total.cell+yield+PL+emission_CO2+rab+His+PO4.ferm.+PO4. apporte+PO4.utilise; 0.394, 0.718, pH+Main.P-Source.Conc.+P -Source.Feed.conc.+Nitrogen.Conc.+X.F.Suc+X.VXdt+total.cell +yield+rab+Thr+PO4.ferm.+PO4.apporte+PO4.utilise; 0.370, 0. 718, pH+Main.P-Source.Conc.+Nitrogen.Conc.+OD+X.F.Suc+X.VXd t+yield+PL+emission_O2+rab+Thr+His+PO4.ferm.+PO4.apporte+PO 4.utilise; 0.406, 0.718, pH+Main.P-Source.Conc.+Nitrogen.Co nc.+X.F.Suc+X.VXdt+total.cell+yield+emission_CO2+rab+PO4.fe rm.+PO4.apporte+PO4.utilise; 0.406, 0.718, pH+Main.Thr.Conc. +Nitrogen.Conc.+X.F.Suc+X.VXdt+yield+PL+emission_O2+Thr+PO4. ferm.+PO4.apporte+PO4.utilise; 0.382, 0.718, pH+tmp+Nitroge n.Conc.+X.F.Suc+X.VXdt+yield+PL+emission_CO2+rab+Thr+His+PO 4.ferm.+PO4.apporte+PO4.utilise; 0.370, 0.718, pH+tmp+Nitro gen.Conc.+X.F.Suc+X.VXdt+total.cell+yield+PL+rab+Thr+His+SO 4.ferm.+PO4.ferm.+PO4.apporte+PO4.utilise; 0.427, 0.718, pH +tmp+Nitrogen.Conc.+X.VXdt+total.cell+yield+emission_CO2+ra b+Thr+His; 0.394, 0.718, pH+tmp+X.F.Suc+X.VXdt+total.cell+y ield+PL+emission_CO2+Thr+His+PO4.ferm.+PO4.apporte+PO4.util ise; 0.406, 0.718, pH+tmp+Nitrogen.Conc.+X.F.Suc+X.VXdt+yie ld+emission_O2+emission_CO2+Thr+PO4.ferm.+PO4.apporte+PO4.u tilise; 0.382, 0.718, pH+Main.P-Source.Conc.+Main.Thr.Conc. +Nitrogen.Conc.+OD+X.F.Suc+X.VXdt+yield+PL+rab+Thr+PO4.ferm. +PO4.apporte+PO4.utilise; 0.416, 0.718, pH+tmp+Main.P-Sourc e.Conc.+Nitrogen.Conc.+X.VXdt+total.cell+yield+PL+emission-O2+Thr+His; 0.382, 0.718, pH+P-Source.Feed.conc.+Nitrogen.C onc.+OD+X.F.Suc+X.VXdt+yield+emission_CO2+rab+Thr+His+PO4.f erm.+PO4.apporte+PO4.utilise; 0.394, 0.718, pH+tmp+Main.P-S ource.Conc.+Nitrogen.Conc.+X.F.Suc+X.VXdt+yield+PL+emission _O2+His+PO4.ferm.+PO4.apporte+PO4.utilise; 0.405, 0.718, pH +Nitrogen.Conc.+X.F.Suc+X.VXdt+yield+PL+emission_O2+emissio n_CO2+lactate+PO4.ferm.+PO4.apporte+PO4.utilise; 0.416, 0.7 18, pH+Main.P-Source.Conc.+Nitrogen.Conc.+X.F.Suc+X.VXdt+yi eld+emission_O2+His+PO4.ferm.+PO4.apporte+PO4.utilise; 0.44 7, 0.718, pH+tmp+Nitrogen.Conc.+X.VXdt+total.cell+yield+emi ssion_O2+Thr; 0.405, 0.718, pH+P-Source.Feed.conc.+Nitrogen. Conc.+X.F.Suc+X.VXdt+yield+emission_CO2+rab+Thr+PO4.ferm.+P O4.apporte+PO4.utilise; 0.405, 0.718, pH+Main.P-Source.Conc. +Nitrogen.Conc.+OD+X.F.Suc+X.VXdt+yield+rab+lactate+PO4.fer m.+PO4.apporte+PO4.utilise; 0.405, 0.718, pH+Main.P-Source. Conc.+Nitrogen.Conc.+X.F.Suc+X.VXdt+yield+PL+emission_CO2+r ab+PO4.ferm.+PO4.apporte+PO4.utilise; 0.394, 0.718, pH+Nitr ogen.Conc.+X.F.Suc+X.VXdt+yield+PL+emission_O2+emission_CO2 +Thr+lactate+PO4.ferm.+PO4.apporte+PO4.utilise; 0.394, 0.71 8, pH+Main.P-Source.Conc.+Nitrogen.Conc.+X.F.Suc+X.VXdt+yie ld+PL+emission_O2+Thr+His+PO4.ferm.+PO4.apporte+PO4.utilis e; 0.382, 0.718, pH+Main.Thr.Conc.+Nitrogen.Conc.+OD+X.F.Su c+X.VXdt+yield+rab+Thr+His+lactate+PO4.ferm.+PO4.apporte+PO 4.utilise; 0.405, 0.718, pH+P-Source.Feed.conc.+Nitrogen.Co nc.+X.F.Suc+X.VXdt+total.cell+yield+PL+rab+PO4.ferm.+PO4.ap porte+PO4.utilise; 0.416, 0.718, pH+tmp+Nitrogen.Conc.+X.F. Suc+yield+emission_O2+Thr+His+PO4.ferm.+PO4.apporte+PO4.uti lise; 0.416, 0.718, pH+tmp+Nitrogen.Conc.+X.F.Suc+X.VXdt+yi eld+rab+lactate+PO4.ferm.+PO4.apporte+PO4.utilise; 0.437, 0. 718, pH+tmp+Main.P-Source.Conc.+X.VXdt+total.cell+yield+emi ssion_CO2+Thr+His; 0.394, 0.718, pH+Nitrogen.Conc.+OD+X.F.S uc+X.VXdt+yield+emission_O2+rab+Thr+SO4.ferm.+PO4.ferm.+PO4. apporte+PO4.utilise; 0.405, 0.718, pH+Main.Thr.Conc.+Nitrog en.Conc.+X.F.Suc+X.VXdt+yield+emission_O2+emission_CO2+Thr+ PO4.ferm.+PO4.apporte+PO4.utilise; 0.382, 0.718, pH+Nitroge n.Conc.+X.F.Suc+X.VXdt+total.cell+yield+PL+emission_CO2+rab +Thr+His+PO4.ferm.+PO4.apporte+PO4.utilise; 0.405, 0.718, p H+tmp+Nitrogen.Conc.+X.F.Suc+X.VXdt+total.cell+yield+rab+la ctate+PO4.ferm.+PO4.apporte+PO4.utilise; 0.405, 0.718, pH+M ain.P-Source.Conc.+P-Source.Feed.conc.+Nitrogen.Conc.+X.F.S uc+X.VXdt+yield+rab+Thr+PO4.ferm.+PO4.apporte+PO4.utilise; 0.394, 0.718, pH+Nitrogen.Conc.+OD+X.F.Suc+X.VXdt+yield+emi ssion_O2+emission_CO2+rab+His+PO4.ferm.+PO4.apporte+PO4.uti lise; 0.369, 0.718, pH+Nitrogen.Conc.+OD+X.F.Suc+X.VXdt+yie ld+PL+emission_CO2+rab+Thr+His+lactate+PO4.ferm.+PO4.apport e+PO4.utilise; 0.437, 0.718, pH+tmp+X.F.Suc+yield+emission_ CO2+His+PO4.ferm.+PO4.apporte+PO4.utilise; 0.394, 0.718, pH +Main.Thr.Conc.+Nitrogen.Conc.+X.F.Suc+X.VXdt+total.cell+yi eld+emission_O2+rab+Thr+PO4.ferm.+PO4.apporte+PO4.utilise; 0.427, 0.718, pH+tmp+Nitrogen.Conc.+X.F.Suc+yield+emission_ CO2+His+PO4.ferm.+PO4.apporte+PO4.utilise; 0.427, 0.718, pH +tmp+Nitrogen.Conc.+X.F.Suc+yield+emission_O2+His+PO4.ferm. +PO4.apporte+PO4.utilise; 0.405, 0.717, pH+Nitrogen.Conc.+O D+X.F.Suc+X.VXdt+yield+emission_CO2+rab+lactate+PO4.ferm.+P O4.apporte+PO4.utilise; 0.405, 0.717, pH+P-Source.Feed.conc. +Nitrogen.Conc.+X.F.Suc+X.VXdt+yield+emission_O2+Thr+lactat e+PO4.ferm.+PO4.apporte+PO4.utilise; 0.369, 0.717, pH+Main. P-Source.Conc.+Nitrogen.Conc.+OD+X.F.Suc+X.VXdt+yield+PL+em ission_O2+Thr+His+lactate+PO4.ferm.+PO4.apporte+PO4.utilis e; 0.381, 0.717, pH+Main.Thr.Conc.+Nitrogen.Conc.+OD+X.F.Su c+X.VXdt+yield+PL+rab+Thr+lactate+PO4.ferm.+PO4.apporte+PO4. utilise; 0.393, 0.717, pH+Nitrogen.Conc.+X.F.Suc+X.VXdt+tot al.cell+yield+emission_O2+emission_CO2+Thr+lactate+PO4.ferm. +PO4.apporte+PO4.utilise; 0.381, 0.717, pH+tmp+Nitrogen.Con c.+OD+X.F.Suc+X.VXdt+yield+emission_O2+emission_CO2+Thr+His +PO4.ferm.+PO4.apporte+PO4.utilise; 0.426, 0.717, pH+tmp+Ni trogen.Conc.+X.VXdt+total.cell+yield+rab+Thr+His+PO4.utilis e; 0.369, 0.717, pH+tmp+Main.P-Source.Conc.+Nitrogen.Conc.+ X.F.Suc+X.VXdt+total.cell+yield+PL+rab+Thr+His+PO4.ferm.+PO 4.apporte+PO4.utilise; 0.393, 0.717, pH+Main.P-Source.Conc. +Main.Thr.Conc.+Nitrogen.Conc.+X.F.Suc+X.VXdt+yield+PL+rab+ Thr+PO4.ferm.+PO4.apporte+PO4.utilise; 0.381, 0.717, pH+Nit rogen.Conc.+X.F.Suc+X.VXdt+total.cell+yield+PL+emission_O2+ emission_CO2+rab+Thr+PO4.ferm.+PO4.apporte+PO4.utilise; 0.4 16, 0.717, pH+Main.P-Source.Conc.+Main.Thr.Conc.+Nitrogen.C onc.+X.F.Suc+X.VXdt+yield+rab+PO4.ferm.+PO4.apporte+PO4.uti lise; 0.381, 0.717, pH+Main.P-Source.Conc.+Nitrogen.Conc.+O D+X.F.Suc+X.VXdt+yield+emission_O2+rab+Thr+lactate+PO4.ferm. +PO4.apporte+PO4.utilise; 0.381, 0.717, pH+Main.P-Source.Co nc.+Nitrogen.Conc.+X.F.Suc+X.VXdt+total.cell+yield+PL+emiss ion_O2+rab+Thr+PO4.ferm.+PO4.apporte+PO4.utilise; 0.393, 0. 717, pH+Main.Thr.Conc.+Nitrogen.Conc.+X.F.Suc+X.VXdt+yield+ PL+rab+Thr+lactate+PO4.ferm.+PO4.apporte+PO4.utilise; 0.426, 0.717, pH+tmp+X.VXdt+total.cell+yield+PL+emission_CO2+Thr+ His+PO4.utilise; 0.393, 0.717, pH+Nitrogen.Conc.+OD+X.F.Suc +X.VXdt+yield+PL+emission_CO2+rab+His+PO4.ferm.+PO4.apporte +PO4.utilise; 0.416, 0.717, pH+P-Source.Feed.conc.+Nitrogen. Conc.+X.F.Suc+X.VXdt+yield+emission_O2+rab+PO4.ferm.+PO4.ap porte+PO4.utilise; 0.404, 0.717, pH+Nitrogen.Conc.+X.F.Suc+ X.VXdt+total.cell+yield+PL+emission_O2+His+PO4.ferm.+PO4.ap porte+PO4.utilise; 0.381, 0.717, pH+Main.Thr.Conc.+Nitrogen. Conc.+OD+X.F.Suc+X.VXdt+yield+PL+emission O2+rab+Thr+PO4.fe rm.+PO4.apporte+PO4.utilise; 0.415, 0.717, pH+tmp+P-Source. Feed.conc.+Nitrogen.Conc.+X.VXdt+total.cell+yield+PL+emissi on_CO2+Thr+His; 0.393, 0.717, pH+Nitrogen.Conc.+OD+X.F.Suc+ X.VXdt+yield+PL+rab+His+lactate+PO4.ferm.+PO4.apporte+PO4.u tilise; 0.404, 0.717, pH+P-Source.Feed.conc.+Nitrogen.Conc. +X.F.Suc+X.VXdt+total.cell+yield+rab+His+PO4.ferm.+PO4.appo rte+PO4.utilise; 0.393, 0.717, pH+tmp+Nitrogen.Conc.+X.F.Su c+X.VXdt+total.cell+yield+PL+emission_O2+emission_CO2+PO4.f erm.+PO4.apporte+PO4.utilise; 0.393, 0.717, pH+P-Source.Fee d.conc.+Nitrogen.Conc.+X.F.Suc+X.VXdt+total.cell+yield+emis sion_O2+emission_CO2+Thr+PO4.ferm.+PO4.apporte+PO4.utilise; 0.381, 0.717, pH+tmp+Nitrogen.Conc.+X.F.Suc+X.VXdt+total.c ell+yield+emission_O2+emission_CO2+rab+Thr+PO4.ferm.+PO4.ap porte+PO4.utilise; 0.369, 0.717, pH+Main.P-Source.Conc.+Nit rogen.Conc.+OD+X.F.Suc+X.VXdt+yield+PL+emission_CO2+rab+Thr +His+PO4.ferm.+PO4.apporte+PO4.utilise; 0.393, 0.717, pH+Ma in.P-Source.Conc.+Nitrogen.Conc.+X.F.Suc+X.VXdt+yield+PL+em ission_CO2+rab+Thr+PO4.ferm.+PO4.apporte+PO4.utilise; 0.393, 0.717, pH+Main.P-Source.Conc.+Nitrogen.Conc.+X.F.Suc+X.VXd t+yield+emission_O2+rab+Thr+His+PO4.ferm.+PO4.apporte+PO4.u tilise; 0.381, 0.717, pH+tmp+Nitrogen.Conc.+X.F.Suc+X.VXdt+ yield+PL+emission_O2+emission_CO2+Thr+His+PO4.ferm.+PO4.app orte+PO4.utilise; 0.381, 0.717, pH+tmp+Main.P-Source.Conc.+ Nitrogen.Conc.+X.F.Suc+X.VXdt+total.cell+yield+PL+rab+His+P O4.ferm.+PO4.apporte+PO4.utilise; 0.404, 0.717, pH+P-Source. Feed.conc.+Nitrogen.Conc.+X.F.Suc+X.VXdt+yield+emission_O2+ emission_CO2+Thr+PO4.ferm.+PO4.apporte+PO4.utilise; 0.393, 0.717, pH+P-Source.Feed.conc.+Main.Thr.Conc.+Nitrogen.Conc. +OD+X.F.Suc+X.VXdt+yield+rab+Thr+PO4.ferm.+PO4.apporte+PO4. utilise; 0.404, 0.717, pH+tmp+Nitrogen.Conc.+OD+X.F.Suc+X.V Xdt+yield+PL+rab+PO4.ferm.+PO4.apporte+PO4.utilise; 0.381, 0.717, pH+tmp+Nitrogen.Conc.+X.F.Suc+X.VXdt+total.cell+yiel d+emission_O2+emission_CO2+rab+His+PO4.ferm.+PO4.apporte+PO 4.utilise; 0.381, 0.717, pH+Main.P-Source.Conc.+Main.Thr.Co nc.+Nitrogen.Conc.+OD+X.F.Suc+X.VXdt+yield+emission_O2+Thr+ His+PO4.ferm.+PO4.apporte+PO4.utilise; 0.404, 0.717, pH+Nit rogen.Conc.+X.F.Suc+X.VXdt+total.cell+yield+emission_CO2+ra b+lactate+PO4.ferm.+PO4.apporte+PO4.utilise; 0.404, 0.717, p+Nitrogen.Conc.+X.F.Suc+X.VXdt+yield+emission_O2+rab+His+ lactate+PO4.ferm.+PO4.apporte+PO4.utilise; 0.393, 0.717, pH +tmp+Nitrogen.Conc.+OD+X.F.Suc+X.VXdt+yield+PL+emission_O2+ His+PO4.ferm.+PO4.apporte+PO4.utilise; 0.368, 0.717, pH+tmp +Nitrogen.Conc.+X.F.Suc+X.VXdt+total.cell+yield+PL+emission _O2+Thr+His+SO4.ferm.+PO4.ferm.+PO4.apporte+PO4.utilise; 0. 381, 0.717, pH+Main.Thr.Conc.+Nitrogen.Conc.+OD+X.F.Suc+X.V Xdt+yield+emission_O2+emission_CO2+Thr+His+PO4.ferm.+PO4.ap porte+PO4.utilise; 0.393, 0.717, pH+tmp+Main.P-Source.Conc. +Nitrogen.Conc.+X.F.Suc+X.VXdt+total.cell+yield+PL+emission _O2+PO4.ferm.+PO4.apporte+PO4.utilise; 0.393, 0.717, pH+Nit rogen.Conc.+X.F.Suc+X.VXdt+total.cell+yield+PL+emission_O2+ Thr+lactate+PO4.ferm.+PO4.apporte+PO4.utilise; 0.393, 0.717, pH+Nitrogen.Conc.+X.F.Suc+X.VXdt+total.cell+yield+emission _O2+Thr+His+lactate+PO4.ferm.+PO4.apporte+PO4.utilise; 0.39 2, 0.717, pH+tmp+Main.P-Source.Conc.+Nitrogen.Conc.+X.F.Suc +X.VXdt+yield+PL+rab+His+PO4.ferm.+PO4.apporte+PO4.utilise; 0.381, 0.717, pH+Nitrogen.Conc.+OD+X.F.Suc+X.VXdt+yield+PL +rab+Thr+His+SO4.ferm.+PO4.ferm.+PO4.apporte+PO4.utilise; 0. 392, 0.717, pH+Nitrogen.Conc.+X.F.Suc+X.VXdt+yield+emission _O2+rab+Thr+His+lactate+PO4.ferm.+PO4.apporte+PO4.utilise; 0.426, 0.717, Nitrogen.Conc.+OD+X.F.Suc+X.VXdt+yield+emissi on_O2+Thr+PO4.ferm.+PO4.apporte+PO4.utilise; 0.380, 0.717, pH+P-Source.Feed.conc.+Nitrogen.Conc.+OD+X.F.Suc+X.VXdt+yie ld+PL+emission_CO2+rab+Thr+PO4.ferm.+PO4.apporte+PO4.utilis e; 0.392, 0.717, pH+Nitrogen.Conc.+OD+X.F.Suc+X.VXdt+yield+ PL+emission_O2+emission_CO2+rab+PO4.ferm.+PO4.apporte+PO4.u tilise; 0.368, 0.717, pH+tmp+Main.P-Source.Conc.+Nitrogen.C onc.+X.F.Suc+X.VXdt+total.cell+yield+PL+emission_CO2+Thr+Hi s+PO4.ferm.+PO4.apporte+PO4.utilise; 0.392, 0.717, pH+Main. P-Source.Conc.+Main.Thr.Conc.+Nitrogen.Conc.+X.F.Suc+X.VXdt +yield+emission_O2+Thr+His+PO4.ferm.+PO4.apporte+PO4.utilis e; 0.404, 0.717, pH+Main.P-Source.Conc.+Nitrogen.Conc.+X.F. Suc+X.VXdt+yield+emission_O2+rab+His+PO4.ferm.+PO4.apporte+ PO4.utilise; 0.404, 0.717, pH+Nitrogen.Conc.+X.F.Suc+X.VXdt +yield+PL+emission_O2+emission_CO2+rab+PO4.ferm.+PO4.apport e+PO4.utilise; 0.415, 0.717, pH+tmp+Main.P-Source.Conc.+Nit rogen.Conc.+X.VXdt+total.cell+yield+PL+emission_CO2+Thr+Hi s; 0.404, 0.717, pH+tmp+Nitrogen.Conc.+X.F.Suc+yield+PL+emi ssion_O2+Thr+His+PO4.ferm.+PO4.apporte+PO4.utilise; 0.425, 0.717, pH+tmp+Nitrogen.Conc.+X.VXdt+total.cell+yield+emissi on_O2+emission_CO2+Thr+His; 0.425, 0.717, pH+tmp+Main.P-Sou rce.Conc.+Nitrogen.Conc.+X.VXdt+total.cell+yield+rab+Thr+Hi s; 0.392, 0.717, pH+tmp+Main.P-Source.Conc.+Nitrogen.Conc.+ X.F.Suc+X.VXdt+yield+emission_O2+Thr+His+PO4.ferm.+PO4.appo rte+PO4.utilise; 0.415, 0.717, pH+tmp+Nitrogen.Conc.+X.F.Su c+X.VXdt+yield+emission_O2+lactate+PO4.ferm.+PO4.apporte+PO 4.utilise; 0.392, 0.717, pH+Main.Thr.Conc.+Nitrogen.Conc.+X. F.Suc+X.VXdt+yield+PL+emission_O2+Thr+His+PO4.ferm.+PO4.app orte+PO4.utilise; 0.403, 0.717, pH+Nitrogen.Conc.+X.F.Suc+X. VXdt+total.cell+yield+emission_O2+emission CO2+His+PO4.ferm. +PO4.apporte+PO4.utilise; 0.392, 0.717, pH+Nitrogen.Conc.+X. F.Suc+X.VXdt+yield+PL+emission_O2+emission CO2+rab+Thr+PO4. ferm.+PO4.apporte+PO4.utilise; 0.415, 0.717, pH+Main.Thr.Co nc.+Nitrogen.Conc.+X.F.Suc+X.VXdt+yield+emission_O2+rab+PO4. ferm.+PO4.apporte+PO4.utilise; 0.392, 0.717, pH+P-Source.Fe ed.conc.+Main.Thr.Conc.+Nitrogen.Conc.+X.F.Suc+X.VXdt+total. cell+yield+rab+Thr+PO4.ferm.+PO4.apporte+PO4.utilise; 0.380, 0.717, pH+Main.P-Source.Conc.+Nitrogen.Conc.+OD+X.F.Suc+X. VXdt+yield+emission_CO2+rab+Thr+lactate+PO4.ferm.+PO4.appor te+PO4.utilise; 0.445, 0.717, pH+tmp+X.VXdt+total.cell+yiel d+emission_O2+Thr+His; 0.403, 0.717, pH+Nitrogen.Conc.+OD+X. F.Suc+X.VXdt+yield+emission_O2+His+lactate+PO4.ferm.+PO4.ap porte+PO4.utilise; 0.403, 0.717, pH+Nitrogen.Conc.+X.F.Suc+ X.VXdt+yield+PL+emission_O2+His+lactate+PO4.ferm.+PO4.appor te+PO4.utilise; 0.380, 0.717, pH+Nitrogen.Conc.+X.F.Suc+X.V Xdt+total.cell+yield+emission_O2+emission_CO2+rab+Thr+His+P O4.ferm.+PO4.apporte+PO4.utilise; 0.392, 0.717, pH+Nitrogen. Conc.+X.F.Suc+X.VXdt+total.cell+yield+PL+emission_O2+rab+Hi s+PO4.ferm.+PO4.apporte+PO4.utilise; 0.435, 0.717, pH+tmp+N itrogen.Conc.+X.F.Suc+yield+emission_O2+PO4.ferm.+PO4.appor te+PO4.utilise; 0.403, 0.717, pH+Main.P-Source.Conc.+Nitrog en.Conc.+X.F.Suc+X.VXdt+yield+PL+emission_O2+emission_CO2+P O4.ferm.+PO4.apporte+PO4.utilise; 0.425, 0.717, pH+tmp+Nitr ogen.Conc.+X.VXdt+total.cell+yield+emission_CO2+Thr+His+PO4. apporte; 0.403, 0.717, pH+tmp+Nitrogen.Conc.+OD+X.F.Suc+X.V Xdt+yield+PL+emission_O2+PO4.ferm.+PO4.apporte+PO4.utilise; 0.435, 0.717, pH+tmp+X.VXdt+total.cell+yield+emission_CO2+ Thr+His+PO4.utilise; 0.403, 0.717, pH+Main.P-Source.Conc.+N itrogen.Conc.+X.F.Suc+X.VXdt+yield+rab+His+lactate+PO4.ferm. +PO4.apporte+PO4.utilise; 0.380, 0.717, pH+tmp+Nitrogen.Con c.+X.F.Suc+X.VXdt+total.cell+yield+PL+emission_O2+emission_ CO2+His+PO4.ferm.+PO4.apporte+PO4.utilise; 0.445, 0.716, pH +tmp+Nitrogen.Conc.+X.VXdt+total.cell+yield+rab+Thr; 0.380, 0.716, pH+P-Source.Feed.conc.+Nitrogen.Conc.+OD+X.F.Suc+X. VXdt+yield+emission_O2+emission_CO2+Thr+His+PO4.ferm.+PO4.a pporte+PO4.utilise; 0.392, 0.716, pH+Main.P-Source.Conc.+Ni trogen.Conc.+X.F.Suc+X.VXdt+yield+PL+emission_O2+emission_C O2+Thr+PO4.ferm.+PO4.apporte+PO4.utilise; 0.380, 0.716, pH+ tmp+Main.P-Source.Conc.+Nitrogen.Conc.+X.F.Suc+X.VXdt+total. cell+yield+PL+emission_O2+His+PO4.ferm.+PO4.apporte+PO4.uti lise; 0.380, 0.716, pH+Main.Thr.Conc.+Nitrogen.Conc.+OD+X.F. Suc+X.VXdt+yield+emission_O2+Thr+His+lactate+PO4.ferm.+PO4. apporte+PO4.utilise; 0.392, 0.716, pH+tmp+Nitrogen.Conc.+OD +X.F.Suc+X.VXdt+yield+PL+emission_CO2+Thr+PO4.ferm.+PO4.app orte+PO4.utilise; 0.380, 0.716, pH+P-Source.Feed.conc.+Nitr ogen.Conc.+X.F.Suc+X.VXdt+total.cell+yield+PL+emission_O2+T hr+His+PO4.ferm.+PO4.apporte+PO4.utilise; 0.425, 0.716, Nit rogen.Conc.+OD+X.F.Suc+X.VXdt+yield+rab+Thr+PO4.ferm.+PO4.a pporte+PO4.utilise; 0.403, 0.716, pH+Nitrogen.Conc.+OD+X.F. Suc+X.VXdt+yield+PL+emission_O2+lactate+PO4.ferm.+PO4.appor te+PO4.utilise; 0.367, 0.716, pH+Nitrogen.Conc.+OD+X.F.Suc+ X.VXdt+yield+emission_O2+emission_CO2+rab+Thr+His+lactate+P O4.ferm.+PO4.apporte+PO4.utilise; 0.403, 0.716, pH+Main.P-S ource.Conc.+Nitrogen.Conc.+OD+X.F.Suc+X.VXdt+yield+emission O2+emission CO2+PO4.ferm.+PO4.apporte+PO4.utilise; 0.403, 0.716, pH+tmp+Nitrogen.Conc.+X.F.Suc+yield+emission_O2+rab+ Thr+His+PO4.ferm.+PO4.apporte+PO4.utilise; 0.445, 0.716, pH +tmp+Nitrogen.Conc.+X.VXdt+total.cell+yield+emission_CO2+Th r; 0.392, 0.716, pH+Main.P-Source.Conc.+Nitrogen.Conc.+OD+X. F.Suc+X.VXdt+yield+PL+rab+His+PO4.ferm.+PO4.apporte+PO4.uti lise; 0.414, 0.716, pH+Nitrogen.Conc.+X.F.Suc+X.VXdt+total. cell+yield+emission_O2+lactate+PO4.ferm.+PO4.apporte+PO4.ut ilise; 0.403, 0.716, pH+tmp+Main.P-Source.Conc.+Nitrogen.Co nc.+X.F.Suc+X.VXdt+yield+emission_O2+Thr+PO4.ferm.+PO4.appo rte+PO4.utilise; 0.391, 0.716, pH+Nitrogen.Conc.+OD+X.F.Suc +X.VXdt+yield+PL+emission_CO2+Thr+His+PO4.ferm.+PO4.apporte +PO4.utilise; 0.380, 0.716, pH+P-Source.Feed.conc.+Nitrogen. Conc.+OD+X.F.Suc+X.VXdt+yield+emission_O2+Thr+His+lactate+P O4.ferm.+PO4.apporte+PO4.utilise; 0.425, 0.716, pH+Main.Thr. Conc.+Nitrogen.Conc.+X.F.Suc+X.VXdt+yield+emission_O2+PO4.f erm.+PO4.apporte+PO4.utilise; 0.391, 0.716, pH+Main.Thr.Con c.+Nitrogen.Conc.+X.F.Suc+X.VXdt+total.cell+yield+rab+Thr+l actate+PO4.ferm.+PO4.apporte+PO4.utilise; 0.391, 0.716, pH+ Main.Thr.Conc.+Nitrogen.Conc.+X.F.Suc+X.VXdt+total.cell+yie ld+emission_O2+Thr+His+PO4.ferm.+PO4.apporte+PO4.utilise; 0. 403, 0.716, pH+P-Source.Feed.conc.+Nitrogen.Conc.+X.F.Suc+X. VXdt+yield+PL+rab+His+PO4.ferm.+PO4.apporte+PO4.utilise; 0. 391, 0.716, pH+tmp+Nitrogen.Conc.+X.F.Suc+X.VXdt+total.cell +yield+emission_O2+emission_CO2+rab+PO4.ferm.+PO4.apporte+P O4.utilise; 0.379, 0.716, pH+Main.Thr.Conc.+Nitrogen.Conc.+ OD+X.F.Suc+X.VXdt+yield+PL+emission_CO2+rab+Thr+PO4.ferm.+P O4.apporte+PO4.utilise; 0.367, 0.716, pH+Main.P-Source.Conc. +Nitrogen.Conc.+OD+X.F.Suc+X.VXdt+yield+emission_O2+emissio n_CO2+rab+Thr+His+PO4.ferm.+PO4.apporte+PO4.utilise; 0.391, 0.716, pH+Nitrogen.Conc.+X.F.Suc+X.VXdt+yield+emission_O2+ emission_CO2+rab+Thr+His+PO4.ferm.+PO4.apporte+PO4.utilise; 0.403, 0.716, pH+Main.P-Source.Conc.+Nitrogen.Conc.+X.F.Su c+X.VXdt+total.cell+yield+rab+lactate+PO4.ferm.+PO4.apporte +PO4.utilise; 0.403, 0.716, pH+tmp+Nitrogen.Conc.+X.F.Suc+X. VXdt+yield+PL+emission_CO2+Thr+PO4.ferm.+PO4.apporte+PO4.ut ilise; 0.414, 0.716, pH+tmp+Main.P-Source.Conc.+Nitrogen.Co nc.+X.VXdt+total.cell+yield+PL+rab+Thr+His; 0.414, 0.716, p H+tmp+Nitrogen.Conc.+X.VXdt+total.cell+yield+PL+emission_CO 2+rab+Thr+His; 0.391, 0.716, pH+Main.Thr.Conc.+Nitrogen.Con c.+X.F.Suc+X.VXdt+total.cell+yield+emission_CO2+rab+Thr+PO4. ferm.+PO4.apporte+PO4.utilise; 0.403, 0.716, pH+P-Source.Fe ed.conc.+Main.Thr.Conc.+Nitrogen.Conc.+X.F.Suc+X.VXdt+yield +rab+Thr+PO4.ferm.+PO4.apporte+PO4.utilise; 0.367, 0.716, p H+Nitrogen.Conc.+OD+X.F.Suc+X.VXdt+yield+PL+emission_O2+emi ssion_CO2+Thr+His+lactate+PO4.ferm.+PO4.apporte+PO4.utilis e; 0.403, 0.716, pH+Nitrogen.Conc.+OD+X.F.Suc+X.VXdt+yield+ PL+emission_CO2+Thr+PO4.ferm.+PO4.apporte+PO4.utilise; 0.39 1, 0.716, pH+tmp+Nitrogen.Conc.+OD+X.F.Suc+X.VXdt+yield+emi ssion_O2+rab+Thr+PO4.ferm.+PO4.apporte+PO4.utilise; 0.379, 0.716, pH+Main.Thr.Conc.+Nitrogen.Conc.+X.F.Suc+X.VXdt+tota l.cell+yield+PL+rab+Thr+His+PO4.ferm.+PO4.apporte+PO4.utili se; 0.414, 0.716, pH+tmp+Nitrogen.Conc.+X.F.Suc+X.VXdt+tota l.cell+yield+emission_CO2+PO4.ferm.+PO4.apporte+PO4.utilis e; 0.379, 0.716, pH+Nitrogen.Conc.+X.F.Suc+X.VXdt+total.cel l+yield+PL+emission_O2+rab+Thr+lactate+PO4.ferm.+PO4.apport e+PO4.utilise; 0.391, 0.716, pH+Nitrogen.Conc.+X.F.Suc+X.VX dt+total.cell+yield+PL+rab+His+lactate+PO4.ferm.+PO4.apport e+PO4.utilise; 0.391, 0.716, pH+Main.Thr.Conc.+Nitrogen.Con c.+X.F.Suc+X.VXdt+yield+emission_O2+rab+Thr+His+PO4.ferm.+P O4.apporte+PO4.utilise; 0.402, 0.716, pH+Main.P-Source.Conc. +Nitrogen.Conc.+X.F.Suc+X.VXdt+total.cell+yield+emission_O2 +emission_CO2+PO4.ferm.+PO4.apporte+PO4.utilise; 0.391, 0.7 16, pH+tmp+Nitrogen.Cone.+X.F.Suc+X.VXdt+total.cell+yield+e mission_O2+His+lactate+PO4.ferm.+PO4.apporte+PO4.utilise; 0. 391, 0.716, pH+tmp+Nitrogen.Conc.+X.F.Suc+X.VXdt+yield+PL+e mission_O2+rab+Thr+PO4.ferm.+PO4.apporte+PO4.utilise; 0.391, 0.716, pH+Nitrogen.Conc.+OD+X.F.Suc+X.VXdt+yield+emission_ CO2+rab+Thr+SO4.ferm.+PO4.ferm.+PO4.apporte+PO4.utilise; 0. 391, 0.716, pH+tmp+Main.P-Source.Conc.+Nitrogen.Conc.+X.F.S uc+X.VXdt+yield+emission_CO2+Thr+His+PO4.ferm.+PO4.apporte+ PO4.utilise; 0.413, 0.716, pH+P-Source.Feed.conc.+Nitrogen. Conc.+X.F.Suc+X.VXdt+yield+emission_O2+His+PO4.ferm.+PO4.ap porte+PO4.utilise; 0.391, 0.716, pH+Main.P-Source.Conc.+Mai n.Thr.Conc.+Nitrogen.Conc.+X.F.Suc+X.VXdt+yield+rab+Thr+lac tate+PO4.ferm.+PO4.apporte+PO4.utilise; 0.413, 0.716, pH+P-Source.Feed.conc.+Nitrogen.Conc.+X.F.Suc+X.VXdt+yield+rab+1 actate+PO4.ferm.+PO4.apporte+PO4.utilise; 0.366, 0.716, pH+ tmp+Nitrogen.Conc.+OD+X.F.Suc+X.VXdt+yield+PL+emission_CO2+ rab+Thr+His+PO4.ferm.+PO4.apporte+PO4.utilise; 0.391, 0.716, pH+Main.P-Source.Conc.+Nitrogen.Conc.+X.F.Suc+X.VXdt+total. cell+yield+PL+emission_O2+Thr+PO4.ferm.+PO4.apporte+PO4.uti lise; 0.434, 0.716, pH+tmp+Main.P-Source.Conc.+X.VXdt+total. cell+yield+emission_O2+Thr+His; 0.402, 0.716, pH+tmp+Nitrog en.Conc.+X.F.Suc+X.VXdt+total.cell+yield+emission_O2+lactat e+PO4.ferm.+PO4.apporte+PO4.utilise; 0.379, 0.716, pH+Nitro gen.Conc.+X.F.Suc+X.VXdt+total.cell+yield+PL+emission_O2+em ission_CO2+Thr+His+PO4.ferm.+PO4.apporte+PO4.utilise; 0.379, 0.716, pH+Main.P-Source.Conc.+Main.Thr.Conc.+Nitrogen.Conc. +X.F.Suc+X.VXdt+total.cell+yield+rab+Thr+His+PO4.ferm.+PO4. apporte+PO4.utilise; 0.391, 0.716, pH+tmp+Nitrogen.Conc.+OD +X.F.Suc+X.VXdt+yield+PL+rab+His+PO4.ferm.+PO4.apporte+PO4. utilise; 0.391, 0.716, pH+tmp+Nitrogen.Conc.+X.F.Suc+X.VXdt +total.cell+yield+rab+His+lactate+PO4.ferm.+PO4.apporte+PO4. utilise; 0.379, 0.716, pH+tmp+Nitrogen.Conc.+X.F.Suc+X.VXdt +total.cell+yield+emission_CO2+Thr+His+lactate+PO4.ferm.+PO 4.apporte+PO4.utilise; 0.366, 0.716, pH+Nitrogen.Conc.+OD+X. F.Suc+X.VXdt+yield+PL+emission_O2+emission_CO2+rab+Thr+lact ate+PO4.ferm.+PO4.apporte+PO4.utilise; 0.402, 0.716, pH+Nit rogen.Conc.+OD+X.F.Suc+X.VXdt+yield+emission_O2+emission_CO 2+lactate+PO4.ferm.+PO4.apporte+PO4.utilise; 0.391, 0.716, pH+Main.P-Source.Conc.+Nitrogen.Conc.+X.F.Suc+X.VXdt+yield+ emission_CO2+rab+Thr+His+PO4.ferm.+PO4.apporte+PO4.utilise; 0.391, 0.716, pH+tmp+Nitrogen.Conc.+X.F.Suc+X.VXdt+total.c ell+yield+emission_O2+Thr+SO4.ferm.+PO4.ferm.+PO4.apporte+P O4.utilise; 0.379, 0.716, pH+Main.P-Source.Conc.+Nitrogen.C onc.+X.F.Suc+X.VXdt+total.cell+yield+PL+emission_CO2+rab+Th r+PO4.ferm.+PO4.apporte+PO4.utilise; 0.402, 0.716, pH+Nitro gen.Conc.+X.F.Suc+X.VXdt+yield+emission_O2+emission_CO2+rab +lactate+PO4.ferm.+PO4.apporte+PO4.utilise; 0.366, 0.716, p H+tmp+Nitrogen.Conc.+OD+X.F.Suc+X.VXdt+yield+PL+emission_O2 +rab+Thr+His+PO4.ferm.+PO4.apporte+PO4.utilise; 0.378, 0.71 6, pH+P-Source.Feed.conc.+Nitrogen.Conc.+X.F.Suc+X.VXdt+tot al.cell+yield+PL+emission_O2+rab+Thr+PO4.ferm.+PO4.apporte+ PO4.utilise; 0.390, 0.716, pH+P-Source.Feed.conc.+Nitrogen. Conc.+OD+X.F.Suc+X.VXdt+yield+rab+Thr+SO4.ferm.+PO4.ferm.+P O4.apporte+PO4.utilise; 0.378, 0.716, pH+P-Source.Feed.conc. +Nitrogen.Conc.+OD+X.F.Suc+X.VXdt+yield+PL+emission_O2+emis sion_C02+Thr+P04.ferm.+P04.apporte+P04.utilise; 0.402, 0.71 6, pH+Main.Thr.Conc.+Nitrogen.Conc.+X.F.Suc+X.VXdt+yield+PL +rab+His+PO4.ferm.+PO4.apporte+PO4.utilise; 0.402, 0.716, p H+Nitrogen.Conc.+X.F.Suc+X.VXdt+yield+emission_O2+emission CO2+rab+His+PO4.ferm.+PO4.apporte+PO4.utilise; 0.413, 0.716, pH+tmp+X.F.Suc+yield+emission O2+rab+Thr+His+PO4.ferm.+PO4. apporte+PO4.utilise; 0.434, 0.716, pH+tmp+Nitrogen.Conc.+X. VXdt+total.cell+yield+PL+emission_O2+Thr; 0.378, 0.716, pH+ Main.P-Source.Conc.+P-Source.Feed.conc.+Nitrogen.Conc.+OD+X. F.Suc+X.VXdt+yield+PL+rab+Thr+PO4.ferm.+PO4.apporte+PO4.uti lise; 0.423, 0.716, pH+tmp+Main.P-Source.Conc.+X.VXdt+total. cell+yield+PL+emission_CO2+Thr+His; 0.390, 0.716, pH+P-Sour ce.Feed.conc.+Main.Thr.Conc.+Nitrogen.Conc.+OD+X.F.Suc+X.VX dt+yield+emission_O2+Thr+PO4.ferm.+PO4.apporte+PO4.utilise; 0.413, 0.716, pH+tmp+Nitrogen.Conc.+OD+X.VXdt+total.cell+y ield+rab+Thr+His+PO4.utilise; 0.378, 0.716, pH+Main.P-Sourc e.Conc.+P-Source.Feed.conc.+Nitrogen.Conc.+OD+X.F.Suc+X.VXd t+yield+rab+Thr+His+PO4.ferm.+PO4.apporte+PO4.utilise; 0.39 0, 0.716, pH+Nitrogen.Conc.+X.F.Suc+X.VXdt+yield+emission_C O2+rab+Thr+His+lactate+PO4.ferm.+PO4.apporte+PO4.utilise; 0. 378, 0.716, pH+tmp+Nitrogen.Conc.+OD+X.F.Suc+X.VXdt+total.c ell+yield+emission_O2+Thr+His+PO4.ferm.+PO4.apporte+PO4.uti lise; 0.390, 0.716, pH+Main.P-Source.Conc.+Nitrogen.Conc.+O D+X.F.Suc+X.VXdt+yield+rab+Thr+SO4.ferm.+PO4.ferm.+PO4.appo rte+PO4.utilise; 0.390, 0.716, pH+Main.Thr.Conc.+Nitrogen.C onc.+OD+X.F.Suc+X.VXdt+yield+rab+Thr+SO4.ferm.+PO4.ferm.+PO 4.apporte+PO4.utilise; 0.402, 0.716, pH+tmp+Nitrogen.Conc.+ OD+X.VXdt+total.cell+yield+PL+rab+Thr+His+PO4.utilise; 0.41 3, 0.716, pH+P-Source.Feed.conc.+Nitrogen.Conc.+X.F.Suc+X.V Xdt+yield+emission_CO2+rab+PO4.ferm.+PO4.apporte+PO4.utilis e; 0.378, 0.716, pH+Main.P-Source.Conc.+Nitrogen.Conc.+OD+X. F.Suc+X.VXdt+yield+emission_O2+emission_CO2+Thr+lactate+PO4. ferm.+PO4.apporte+PO4.utilise; 0.366, 0.716, pH+Main.P-Sour ce.Conc.+Nitrogen.Conc.+OD+X.F.Suc+X.VXdt+yield+PL+emission _O2+emission-CO2+Thr+His+PO4.ferm.+PO4.apporte+PO4.utilise; 0.378, 0.716, pH+tmp+Nitrogen.Conc.+OD+X.F.Suc+X.VXdt+tota l.cell+yield+rab+Thr+His+PO4.ferm.+PO4.apporte+PO4.utilise; 0.378, 0.715, pH+P-Source.Feed.conc.+Nitrogen.Conc.+OD+X.F. Suc+X.VXdt+yield+PL+rab+Thr+lactate+PO4.ferm.+PO4.apporte+P O4.utilise; 0.378, 0.715, pH+Main.P-Source.Conc.+Main.Thr.C onc.+Nitrogen.Conc.+OD+X.F.Suc+X.VXdt+yield+PL+emission_O2+ Thr+PO4.ferm.+PO4.apporte+PO4.utilise; 0.390, 0.715, pH+Nit rogen.Conc.+OD+X.F.Suc+X.VXdt+yield+PL+emission_O2+Thr+SO4. ferm.+PO4.ferm.+PO4.apporte+PO4.utilise; 0.423, 0.715, pH+t mp+Nitrogen.Conc.+X.VXdt+total.cell+yield+emission_O2+rab+T hr+His; 0.390, 0.715, pH+Main.P-Source.Conc.+Nitrogen.Conc. +X.F.Suc+X.VXdt+total.cell+yield+PL+emission_O2+rab+PO4.fer m.+PO4.apporte+PO4.utilise; 0.423, 0.715, pH+tmp+Nitrogen.C onc.+X.F.Suc+yield+emission_O2+Thr+PO4.ferm.+PO4.apporte+PO 4.utilise; 0.365, 0.715, pH+Main.P-Source.Conc.+Nitrogen.Co nc.+OD+X.F.Suc+X.VXdt+yield+PL+emission_O2+emission_CO2+rab +Thr+PO4.ferm.+PO4.apporte+PO4.utilise; 0.401, 0.715, pH+tm p+Nitrogen.Conc.+X.F.Suc+X.VXdt+yield+emission_O2+His+lacta te+PO4.ferm.+PO4.apporte+PO4.utilise; 0.378, 0.715, pH+Main. Thr.Conc.+Nitrogen.Conc.+OD+X.F.Suc+X.VXdt+yield+emission_O 2+emission_CO2+rab+Thr+PO4.ferm.+PO4.apporte+PO4.utilise; 0. 390, 0.715, pH+tmp+Nitrogen.Conc.+X.VXdt+total.cell+yield+P L+emission_O2+emission_CO2+rab+Thr+His+PO4.utilise; 0.390, 0.715, pH+Nitrogen.Conc.+OD+X.F.Suc+X.VXdt+yield+PL+emissio n_O2+emission_CO2+His+PO4.ferm.+PO4.apporte+PO4.utilise; 0. 412, 0.715, pH+tmp+Nitrogen.Conc.+X.F.Suc+yield+PL+emission _O2+His+PO4.ferm.+PO4.apporte+PO4.utilise; 0.390, 0.715, pH +tmp+Nitrogen.Conc.+OD+X.F.Suc+X.VXdt+yield+emission_CO2+ra b+Thr+PO4.ferm.+PO4.apporte+PO4.utilise; 0.412, 0.715, pH+M ain.Thr.Conc.+Nitrogen.Conc.+X.F.Suc+X.VXdt+yield+emission CO2+rab+PO4.ferm.+PO4.apporte+PO4.utilise; 0.378, 0.715, pH +tmp+Nitrogen.Conc.+X.F.Suc+X.VXdt+total.cell+yield+emissio n_O2+Thr+His+lactate+PO4.ferm.+PO4.apporte+PO4.utilise; 0.3 90, 0.715, pH+tmp+Nitrogen.Conc.+X.F.Suc+yield+PL+emission_ O2+rab+Thr+His+PO4.ferm.+PO4.apporte+PO4.utilise; 0.433, 0. 715, pH+tmp+P-Source.Feed.conc.+X.VXdt+total.cell+yield+emi ssion_CO2+Thr+His; 0.412, 0.715, pH+tmp+Nitrogen.Conc.+X.VX dt+total.cell+yield+PL+emission_CO2+Thr+His+PO4.apporte; 0. 390, 0.715, pH+Nitrogen.Conc.+X.F.Suc+X.VXdt+total.cell+yie ld+PL+emission_O2+emission_CO2+rab+PO4.ferm.+PO4.apporte+PO 4.utilise; 0.378, 0.715, pH+tmp+Main.Thr.Conc.+Nitrogen.Con c.+X.F.Suc+X.VXdt+total.cell+yield+emission_O2+Thr+His+PO4. ferm.+PO4.apporte+PO4.utilise; 0.378, 0.715, pH+Main.Thr.Co nc.+Nitrogen.Conc.+OD+X.F.Suc+X.VXdt+yield+PL+emission_O2+e mission_CO2+Thr+PO4.ferm.+PO4.apporte+PO4.utilise; 0.389, 0. 715, pH+Main.P-Source.Conc.+Nitrogen.Conc.+X.F.Suc+X.VXdt+t otal.cell+yield+emission_O2+Thr+His+PO4.ferm.+PO4.apporte+P O4.utilise; 0.389, 0.715, pH+Main.Thr.Conc.+Nitrogen.Conc.+ X.F.Suc+X.VXdt+total.cell+yield+emission_O2+emission_CO2+Th r+PO4.ferm.+PO4.apporte+PO4.utilise; 0.401, 0.715, pH+Nitro gen.Conc.+X.F.Suc+X.VXdt+yield+emission_CO2+rab+His+lactate +PO4.ferm.+PO4.apporte+PO4.utilise; 0.401, 0.715, pH+Main.P -Source.Conc.+Nitrogen.Conc.+X.F.Suc+X.VXdt+yield+emission_ CO2+rab+His+PO4.ferm.+PO4.apporte+PO4.utilise; 0.377, 0.715, pH+P-Source.Feed.conc.+Nitrogen.Conc.+OD+X.F.Suc+X.VXdt+yi eld+PL+emission_O2+Thr+lactate+PO4.ferm.+PO4.apporte+PO4.ut ilise; 0.423, 0.715, pH+tmp+Main.P-Source.Conc.+X.VXdt+tota l.cell+yield+PL+emission_O2+Thr+His; 0.389, 0.715, pH+Main. P-Source.Conc.+Nitrogen.Conc.+X.F.Suc+X.VXdt+total.cell+yie ld+PL+rab+His+PO4.ferm.+PO4.apporte+PO4.utilise; 0.389, 0.7 15, pH+Nitrogen.Conc.+X.F.Suc+X.VXdt+total.cell+yield+PL+em ission-O2+rab+lactate+PO4.ferm.+PO4.apporte+PO4.utilise; 0. 401, 0.715, pH+Nitrogen.Conc.+X.F.Suc+X.VXdt+total.cell+yie ld+emission_O2+emission_CO2+lactate+PO4.ferm.+PO4.apporte+P O4.utilise; 0.412, 0.715, pH+P-Source.Feed.conc.+Nitrogen.C onc.+X.F.Suc+X.VXdt+yield+PL+emission_O2+PO4.ferm.+PO4.appo rte+PO4.utilise; 0.377, 0.715, pH+Main.P-Source.Conc.+Nitro gen.Conc.+X.F.Suc+X.VXdt+total.cell+yield+PL+rab+Thr+lact at e+PO4.ferm.+PO4.apporte+PO4.utilise; 0.365, 0.715, pH+tmp+N itrogen.Conc.+OD+X.F.Suc+X.VXdt+yield+PL+emission_O2+emissi on_CO2+Thr+His+PO4.ferm.+PO4.apporte+PO4.utilise; 0.389, 0. 715, pH+P-Source.Feed.conc.+Nitrogen.Conc.+X.F.Suc+X.VXdt+y ield+PL+rab+Thr+lactate+PO4.ferm.+PO4.apporte+PO4.utilise; 0.423, 0.715, pH+tmp+X.F.Suc+yield+PL+emission_CO2+His+PO4. ferm.+PO4.apporte+PO4.utilise; 0.377, 0.715, pH+Main.P-Sour ce.Conc.+Main.Thr.Conc.+Nitrogen.Conc.+OD+X.F.Suc+X.VXdt+yi eld+rab+Thr+lactate+PO4.ferm.+PO4.apporte+PO4.utilise; 0.40 1, 0.715, pH+Main.P-Source.Conc.+Nitrogen.Conc.+OD+X.F.Suc+ X.VXdt+yield+emission_O2+His+PO4.ferm.+PO4.apporte+PO4.util ise; 0.433, 0.715, pH+tmp+X.VXdt+total.cell+yield+PL+emissi on_O2+Thr+His; 0.401, 0.715, pH+Main.P-Source.Conc.+Nitroge n.Conc.+OD+X.F.Suc+X.VXdt+yield+PL+emission_O2+PO4.ferm.+PO 4.apporte+PO4.utilise; 0.412, 0.715, pH+tmp+Nitrogen.Conc.+ X.F.Suc+X.VXdt+yield+PL+emission_CO2+PO4.ferm.+PO4.apporte+ PO4.utilise; 0.412, 0.715, pH+tmp+Nitrogen.Conc.+X.F.Suc+yi eld+PL+emission_CO2+His+PO4.ferm.+PO4.apporte+PO4.utilise; 0.412, 0.715, pH+tmp+Nitrogen.Conc.+X.VXdt+total.cell+yield +PL+emission_O2+emission_CO2+Thr+His; 0.401, 0.715, pH+Main. P-Source.Conc.+Nitrogen.Conc.+X.F.Suc+X.VXdt+yield+emission _O2+rab+lactate+PO4.ferm.+PO4.apporte+PO4.utilise; 0.377, 0. 715, pH+Main.Thr.Conc.+Nitrogen.Conc.+OD+X.F.Suc+X.VXdt+yie ld+PL+emission_O2+Thr+lactate+PO4.ferm.+PO4.apporte+PO4.uti lise; 0.389, 0.715, pH+Nitrogen.Conc.+X.F.Suc+X.VXdt+yield+ emission_O2+emission_CO2+rab+Thr+lactate+PO4.ferm.+PO4.appo rte+PO4.utilise; 0.389, 0.715, pH+tmp+Nitrogen.Conc.+X.F.Su c+X.VXdt+yield+emission_O2+emission_CO2+rab+His+PO4.ferm.+P O4.apporte+PO4.utilise; 0.412, 0.715, pH+tmp+Nitrogen.Conc. +X.VXdt+total.cell+yield+rab+Thr+PO4.ferm.+PO4.apporte+PO4. utilise; 0.422, 0.715, pH+tmp+X.VXdt+total.cell+yield+PL+em ission_O2+Thr+His+PO4.utilise; 0.389, 0.715, pH+tmp+Nitroge n.Conc.+OD+X.F.Suc+X.VXdt+total.cell+yield+rab+His+PO4.ferm. +PO4.apporte+PO4.utilise; 0.389, 0.715, pH+Main.Thr.Conc.+N itrogen.Conc.+X.F.Suc+X.VXdt+yield+emission_O2+Thr+His+lact ate+PO4.ferm.+PO4.apporte+PO4.utilise; 0.389, 0.715, pH+Nit rogen.Conc.+OD+X.F.Suc+X.VXdt+yield+PL+emission_O2+rab+lact ate+PO4.ferm.+PO4.apporte+PO4.utilise; 0.377, 0.715, pH+Mai n.P-Source.Conc.+Nitrogen.Conc.+X.F.Suc+X.VXdt+total.cell+y ield+emission_O2+rab+Thr+His+PO4.ferm.+PO4.apporte+PO4.util ise; 0.389, 0.715, pH+Main.P-Source.Conc.+Nitrogen.Conc.+OD +X.F.Suc+X.VXdt+yield+PL+emission_O2+rab+PO4.ferm.+PO4.appo rte+PO4.utilise; 0.389, 0.715, pH+Main.Thr.Conc.+Nitrogen.C onc.+X.F.Suc+X.VXdt+yield+emission_CO2+rab+Thr+His+PO4.ferm. +PO4.apporte+PO4.utilise; 0.377, 0.715, pH+P-Source.Feed.co nc.+Nitrogen.Conc.+OD+X.F.Suc+X.VXdt+yield+emission_O2+emis sion_CO2+rab+Thr+PO4.ferm.+PO4.apporte+PO4.utilise; 0.377, 0.715, pH+Main.P-Source.Conc.+P-Source.Feed.conc.+Nitrogen. Conc.+OD+X.F.Suc+X.VXdt+yield+emission_O2+Thr+His+PO4.ferm. +PO4.apporte+PO4.utilise; 0.411, 0.715, pH+Main.P-Source.Co nc.+Nitrogen.Conc.+X.F.Suc+X.VXdt+total.cell+yield+emission _O2+PO4.ferm.+PO4.apporte+PO4.utilise; 0.377, 0.715, pH+P-S ource.Feed.conc.+Nitrogen.Conc.+OD+X.F.Suc+X.VXdt+yield+rab +Thr+His+lactate+PO4.ferm.+PO4.apporte+PO4.utilise; 0.400, 0.715, pH+Main.Thr.Conc.+Nitrogen.Conc.+X.F.Suc+X.VXdt+tota l.cell+yield+rab+His+PO4.ferm.+PO4.apporte+PO4.utilise; 0.3 77, 0.715, pH+tmp+Nitrogen.Conc.+X.F.Suc+X.VXdt+total.cell+ yield+PL+rab+Thr+SO4.ferm.+PO4.ferm.+PO4.apporte+PO4.utilis e; 0.422, 0.715, pH+tmp+P-Source.Feed.conc.+Nitrogen.Conc.+ X.VXdt+total.cell+yield+emission_O2+Thr+His; 0.400, 0.715, pH+tmp+Nitrogen.Conc.+X.F.Suc+X.VXdt+yield+emission_O2+emis sion_CO2+rab+PO4.ferm.+PO4.apporte+PO4.utilise; 0.364, 0.71 5, pH+tmp+Nitrogen.Conc.+X.F.Suc+X.VXdt+total.cell+yield+PL +emission_O2+emission_CO2+rab+Thr+PO4.ferm.+PO4.apporte+PO4. utilise; 0.389, 0.715, pH+Main.P-Source.Conc.+Nitrogen.Conc. +X.F.Suc+X.VXdt+yield+emission_O2+emission_CO2+Thr+His+PO4. ferm.+PO4.apporte+PO4.utilise; 0.411, 0.715, pH+tmp+Nitroge n.Conc.+X.VXdt+total.cell+yield+emission_O2+Thr+PO4.ferm.+P O4.apporte+PO4.utilise; 0.389, 0.715, pH+P-Source.Feed.conc. +Nitrogen.Conc.+X.F.Suc+X.VXdt+yield+PL+emission_O2+rab+Thr +PO4.ferm.+PO4.apporte+PO4.utilise; 0.389, 0.715, pH+tmp+Ni trogen.Conc.+X.F.Suc+X.VXdt+yield+PL+emission_CO2+rab+Thr+P O4.ferm.+PO4.apporte+PO4.utilise; 0.432, 0.715, pH+tmp+Nitr ogen.Conc.+X.VXdt+total.cell+yield+PL+rab+Thr; 0.400, 0.715, pH+Main.Thr.Conc.+Nitrogen.Conc.+OD+X.F.Suc+X.VXdt+yield+r ab+His+PO4.ferm.+PO4.apporte+PO4.utilise; 0.411, 0.715, pH+ tmp+Main.P-Source.Conc.+Nitrogen.Conc.+X.VXdt+total.cell+yi eld+emission_CO2+Thr+His+lactate; 0.400, 0.715, pH+P-Source. Feed.conc.+Nitrogen.Conc.+X.F.Suc+X.VXdt+total.cell+yield+e mission_O2+rab+PO4.ferm.+PO4.apporte+PO4.utilise; 0.364, 0. 715, pH+tmp+Nitrogen.Conc.+X.F.Suc+X.VXdt+total.cell+yield+ PL+emission_O2+emission_CO2+rab+His+PO4.ferm.+PO4.apporte+P O4.utilise; 0.388, 0.715, pH+tmp+Nitrogen.Conc.+OD+X.F.Suc+ X.VXdt+yield+emission_O2+emission_CO2+Thr+PO4.ferm.+PO4.app orte+PO4.utilise; 0.400, 0.715, pH+tmp+X.F.Suc+yield+PL+emi ssion_O2+rab+Thr+His+PO4.ferm.+PO4.apporte+PO4.utilise; 0.3 76, 0.715, pH+Main.P-Source.Conc.+Main.Thr.Conc.+Nitrogen.C onc.+X.F.Suc+X.VXdt+yield+PL+rab+Thr+His+PO4.ferm.+PO4.appo rte+PO4.utilise; 0.376, 0.715, pH+Nitrogen.Conc.+X.F.Suc+X. VXdt+total.cell+yield+PL+emission_CO2+rab+Thr+lactate+PO4.f erm.+PO4.apporte+PO4.utilise; 0.388, 0.715, pH+Nitrogen.Con c.+X.F.Suc+X.VXdt+total.cell+yield+PL+emission_CO2+rab+His+ PO4.ferm.+PO4.apporte+PO4.utilise; 0.388, 0.715, pH+Nitroge n.Conc.+X.F.Suc+X.VXdt+yield+emission_O2+emission_CO2+Thr+H is+lactate+PO4.ferm.+PO4.apporte+PO4.utilise; 0.411, 0.715, pH+Main.Thr.Conc.+Nitrogen.Conc.+X.F.Suc+X.VXdt+yield+emis sion_O2+His+PO4.ferm.+PO4.apporte+PO4.utilise; 0.400, 0.715, pH+tmp+Nitrogen.Conc.+X.F.Suc+X.VXdt+yield+rab+His+lactate +PO4.ferm.+PO4.apporte+PO4.utilise; 0.400, 0.715, pH+tmp+X. F.Suc+X.VXdt+total.cell+yield+emission_O2+Thr+His+PO4.ferm. +PO4.apporte+PO4.utilise; 0.388, 0.715, pH+Nitrogen.Conc.+O D+X.F.Suc+X.VXdt+yield+emission_O2+emission_CO2+Thr+SO4.fer m.+PO4.ferm.+PO4.apporte+PO4.utilise; 0.411, 0.715, pH+Main. P-Source.Conc.+P-Source.Feed.conc.+Nitrogen.Conc.+X.F.Suc+X. VXdt+yield+rab+PO4.ferm.+PO4.apporte+PO4.utilise; 0.388, 0. 715, pH+P-Source.Feed.conc.+Nitrogen.Conc.+X.F.Suc+X.VXdt+y ield+emission_O2+rab+Thr+His+PO4.ferm.+PO4.apporte+PO4.util ise; 0.411, 0.714, pH+Main.Thr.Conc.+Nitrogen.Conc.+X.F.Suc +X.VXdt+yield+emission_O2+emission_CO2+PO4.ferm.+PO4.apport e+PO4.utilise; 0.411, 0.714, pH+P-Source.Feed.conc.+Nitroge n.Conc.+X.F.Suc+X.VXdt+yield+emission_O2+emission_CO2+PO4.f erm.+PO4.apporte+PO4.utilise; 0.376, 0.714, pH+Main.P-Sourc e.Conc.+Main.Thr.Conc.+Nitrogen.Conc.+OD+X.F.Suc+X.VXdt+yie ld+rab+Thr+SO4.ferm.+PO4.ferm.+PO4.apporte+PO4.utilise; 0.3 88, 0.714, pH+tmp+Nitrogen.Conc.+X.F.Suc+X.VXdt+total.cell+ yield+rab+His+SO4.ferm.+PO4.ferm.+PO4.apporte+PO4.utilise; 0.376, 0.714, pH+Main.P-Source.Conc.+Nitrogen.Conc.+X.F.Suc +X.VXdt+total.cell+yield+PL+emission_O2+emission_CO2+Thr+PO 4.ferm.+PO4.apporte+PO4.utilise; 0.399, 0.714, pH+tmp+Main. P-Source.Conc.+Nitrogen.Conc.+X.F.Suc+X.VXdt+yield+emission _O2+rab+PO4.ferm.+PO4.apporte+PO4.utilise; 0.399, 0.714, pH +tmp+P-Source.Feed.conc.+Nitrogen.Conc.+X.VXdt+total.cell+y ield+PL+emission_O2+Thr+His+PO4.utilise; 0.388, 0.714, pH+M ain.Thr.Conc.+Nitrogen.Conc.+X.F.Suc+X.VXdt+yield+emission_ O2+emission CO2+Thr+His+PO4.ferm.+PO4.apporte+PO4.utilise; 0.363, 0.714, pH+P-Source.Feed.conc.+Nitrogen.Conc.+OD+X.F. Suc+X.VXdt+yield+PL+emission_O2+rab+Thr+His+PO4.ferm.+PO4.a pporte+PO4.utilise; 0.388, 0.714, pH+tmp+Nitrogen.Conc.+X.F. Suc+X.VXdt+yield+PL+emission_O2+emission_CO2+Thr+PO4.ferm.+ PO4.apporte+PO4.utilise; 0.421, 0.714, pH+tmp+Nitrogen.Conc. +X.VXdt+total.cell+yield+emission_CO2+Thr+His+PO4.ferm.; 0. 410, 0.714, pH+P-Source.Feed.conc.+Nitrogen.Conc.+X.F.Suc+X. VXdt+total.cell+yield+emission_O2+PO4.ferm.+PO4.apporte+PO4. utilise; 0.410, 0.714, Nitrogen.Conc.+OD+X.F.Suc+X.VXdt+yie ld+PL+emission_O2+Thr+PO4.ferm.+PO4.apporte+PO4.utilise; 0. 431, 0.714, pH+tmp+X.F.Suc+yield+emission_O2+His+PO4.ferm.+ PO4.apporte+PO4.utilise; 0.399, 0.714, pH+tmp+Main.P-Source. Conc.+Nitrogen.Conc.+X.VXdt+total.cell+yield+emission_O2+em ission_CO2+rab+Thr+His; 0.376, 0.714, pH+P-Source.Feed.conc. +Nitrogen.Conc.+X.F.Suc+X.VXdt+total.cell+yield+emission_O2 +rab+Thr+His+PO4.ferm.+PO4.apporte+PO4.utilise; 0.399, 0.71 4, pH+tmp+Nitrogen.Conc.+X.F.Suc+X.VXdt+total.cell+yield+ra b+SO4.ferm.+PO4.ferm.+PO4.apporte+PO4.utilise; 0.410, 0.714, pH+Nitrogen.Conc.+X.F.Suc+X.VXdt+yield+rab+Thr+SO4.ferm.+P O4.ferm.+PO4.apporte+PO4.utilise; 0.375, 0.714, pH+P-Source. Feed.conc.+Nitrogen.Conc.+X.F.Suc+X.VXdt+total.cell+yield+P L+emission_CO2+rab+Thr+PO4.ferm.+PO4.apporte+PO4.utilise; 0. 399, 0.714, pH+tmp+Main.P-Source.Conc.+Nitrogen.Conc.+X.F.S uc+X.VXdt+yield+rab+Thr+PO4.ferm.+PO4.apporte+PO4.utilise; 0.387, 0.714, pH+Main.P-Source.Conc.+Nitrogen.Conc.+X.F.Suc +X.VXdt+yield+PL+emission_O2+Thr+lactate+PO4.ferm.+PO4.appo rte+PO4.utilise; 0.399, 0.714, pH+Main.P-Source.Conc.+Nitro gen.Conc.+X.F.Suc+X.VXdt+yield+PL+emission_O2+His+PO4.ferm. +PO4.apporte+PO4.utilise; 0.399, 0.714, pH+tmp+Nitrogen.Con c.+X.VXdt+total.cell+yield+emission_O2+emission_CO2+rab+Thr +His+PO4.utilise; 0.410, 0.714, pH+tmp+Nitrogen.Conc.+X.VXd t+total.cell+yield+PL+emission_O2+rab+Thr+His; 0.410, 0.714, pH+tmp+Nitrogen.Conc.+X.F.Suc+yield+PL+emission_O2+Thr+PO4. ferm.+PO4.apporte+PO4.utilise; 0.375, 0.714, pH+Main.P-Sour ce.Conc.+Nitrogen.Conc.+X.F.Suc+X.VXdt+yield+PL+rab+Thr+His +lactate+PO4.ferm.+PO4.apporte+PO4.utilise; 0.375, 0.714, p H+Main.P-Source.Conc.+Main.Thr.Conc.+Nitrogen.Conc.+X.F.Suc +X.VXdt+total.cell+yield+PL+rab+Thr+PO4.ferm.+PO4.apporte+P O4.utilise; 0.387, 0.714, pH+Nitrogen.Conc.+X.F.Suc+X.VXdt+ total.cell+yield+emission_O2+emission_CO2+rab+His+PO4.ferm. +PO4.apporte+PO4.utilise; 0.399, 0.714, pH+P-Source.Feed.co nc.+Nitrogen.Conc.+OD+X.F.Suc+X.VXdt+yield+rab+His+PO4.ferm. +PO4.apporte+PO4.utilise; 0.410, 0.714, pH+tmp+Nitrogen.Con c.+X.VXdt+total.cell+yield+emission_CO2+Thr+His+lactate+PO4. utilise; 0.421, 0.714, pH+tmp+Nitrogen.Conc.+X.F.Suc+yield+ PL+emission_O2+PO4.ferm.+PO4.apporte+PO4.utilise; 0.387, 0. 714, pH+tmp+X.F.Suc+X.VXdt+total.cell+yield+PL+emission_O2+ Thr+His+PO4.ferm.+PO4.apporte+PO4.utilise; 0.399, 0.714, pH +Main.Thr.Conc.+Nitrogen.Conc.+OD+X.F.Suc+X.VXdt+yield+PL+r ab+PO4.ferm.+PO4.apporte+PO4.utilise; 0.410, 0.714, pH+tmp+ P-Source.Feed.conc.+Nitrogen.Conc.+X.VXdt+total.cell+yield+ emission_CO2+Thr+His+PO4.utilise; 0.410, 0.714, pH+tmp+P-So urce.Feed.conc.+Nitrogen.Conc.+X.VXdt+total.cell+yield+PL+e mission_O2+Thr+His; 0.421, 0.714, pH+tmp+Nitrogen.Conc.+X.F. Suc+yield+emission_CO2+Thr+PO4.ferm.+PO4.apporte+PO4.utilis e; 0.387, 0.714, pH+tmp+Nitrogen.Conc.+X.F.Suc+X.VXdt+total. cell+yield+PL+rab+lactate+PO4.ferm.+PO4.apporte+PO4.utilis e; 0.387, 0.714, pH+Nitrogen.Conc.+OD+X.F.Suc+X.VXdt+yield+ emission_CO2+Thr+His+lactate+PO4.ferm.+PO4.apporte+PO4.util ise; 0.375, 0.714, pH+tmp+Main.Thr.Conc.+Nitrogen.Conc.+X.F. Suc+X.VXdt+total.cell+yield+rab+Thr+His+PO4.ferm.+PO4.appor te+PO4.utilise; 0.399, 0.714, pH+Main.P-Source.Conc.+P-Sour ce.Feed.conc.+Nitrogen.Conc.+X.F.Suc+X.VXdt+yield+emission_ O2+Thr+PO4.ferm.+PO4.apporte+PO4.utilise; 0.375, 0.714, pH+ Nitrogen.Conc.+X.F.Suc+X.VXdt+total.cell+yield+emission_O2+ rab+Thr+His+lactate+PO4.ferm.+PO4.apporte+PO4.utilise; 0.42 0, 0.714, pH+tmp+X.VXdt+total.cell+yield+PL+emission_CO2+Th r+His+PO4.apporte; 0.399, 0.714, pH+tmp+P-Source.Feed.conc. +Nitrogen.Conc.+X.VXdt+total.cell+yield+PL+rab+Thr+His+PO4. utilise; 0.399, 0.714, pH+tmp+P-Source.Feed.conc.+Nitrogen. Conc.+X.VXdt+total.cell+yield+PL+emission_CO2+Thr+His+PO4.u tilise; 0.375, 0.714, pH+Main.P-Source.Conc.+Nitrogen.Conc. +X.F.Suc+X.VXdt+total.cell+yield+emission_O2+emission_CO2+r ab+Thr+PO4.ferm.+PO4.apporte+PO4.utilise; 0.375, 0.714, pH+ Main.P-Source.Conc.+P-Source.Feed.conc.+Nitrogen.Conc.+OD+X. F.Suc+X.VXdt+yield+PL+emission_O2+Thr+PO4.ferm.+PO4.apporte +PO4.utilise; 0.387, 0.714, pH+P-Source.Feed.conc.+Nitrogen. Conc.+X.F.Suc+X.VXdt+total.cell+yield+emission_O2+Thr+lacta te+PO4.ferm.+PO4.apporte+PO4.utilise; 0.398, 0.714, pH+tmp+ Nitrogen.Conc.+X.F.Suc+X.VXdt+yield+PL+rab+lactate+PO4.ferm. +PO4.apporte+PO4.utilise; 0.410, 0.714, pH+Main.P-Source.Co nc.+Nitrogen.Conc.+X.F.Suc+X.VXdt+yield+emission_O2+lactate +PO4.ferm.+PO4.apporte+PO4.utilise; 0.362, 0.714, pH+Main.T hr.Conc.+Nitrogen.Conc.+OD+X.F.Suc+X.VXdt+yield+PL+emission _O2+rab+Thr+His+PO4.ferm.+PO4.apporte+PO4.utilise; 0.375, 0. 714, pH+P-Source.Feed.conc.+Nitrogen.Conc.+X.F.Suc+X.VXdt+t otal.cell+yield+PL+rab+Thr+lactate+PO4.ferm.+PO4.apporte+PO 4.utilise; 0.375, 0.714, pH+Main.P-Source.Conc.+Nitrogen.Co nc.+X.F.Suc+X.VXdt+total.cell+yield+rab+Thr+His+lactate+PO4. ferm.+PO4.apporte+PO4.utilise; 0.387, 0.714, pH+P-Source.Fe ed.conc.+Nitrogen.Conc.+X.F.Suc+X.VXdt+yield+rab+Thr+His+la ctate+PO4.ferm.+PO4.apporte+PO4.utilise; 0.398, 0.714, pH+t mp+Nitrogen.Conc.+OD+X.F.Suc+X.VXdt+yield+emission_CO2+His+ PO4.ferm.+PO4.apporte+PO4.utilise; 0.410, 0.714, pH+Nitroge n.Conc.+OD+X.F.Suc+X.VXdt+yield+rab+SO4.ferm.+PO4.ferm.+PO4. apporte+PO4.utilise; 0.398, 0.714, pH+Main.P-Source.Conc.+M ain.Thr.Conc.+Nitrogen.Conc.+X.F.Suc+X.VXdt+yield+rab+His+P O4.ferm.+PO4.apporte+PO4.utilise; 0.431, 0.714, pH+tmp+X.VX dt+total.cell+yield+emission_CO2+Thr+His+PO4.apporte; 0.420, 0.714, pH+Nitrogen.Conc.+X.F.Suc+X.VXdt+yield+rab+SO4.ferm. +PO4.ferm.+PO4.apporte+PO4.utilise; 0.398, 0.714, pH+P-Sour ce.Feed.conc.+Nitrogen.Conc.+OD+X.F.Suc+X.VXdt+yield+PL+rab +PO4.ferm.+PO4.apporte+PO4.utilise; 0.375, 0.714, pH+Nitrog en.Conc.+OD+X.F.Suc+X.VXdt+yield+PL+rab+Thr+lactate+SO4.fer m.+PO4.ferm.+PO4.apporte+PO4.utilise; 0.387, 0.714, pH+Main. P-Source.Conc.+Nitrogen.Conc.+X.F.Suc+X.VXdt+total.cell+yie ld+PL+emission_CO2+rab+PO4.ferm.+PO4.apporte+PO4.utilise; 0. 387, 0.714, pH+P-Source.Feed.conc.+Main.Thr.Conc.+Nitrogen. Conc.+X.F.Suc+X.VXdt+yield+rab+Thr+His+PO4.ferm.+PO4.apport e+PO4.utilise; 0.375, 0.714, pH+Main.P-Source.Conc.+Main.Th r.Conc.+Nitrogen.Conc.+OD+X.F.Suc+X.VXdt+yield+emission_O2+ Thr+lactate+PO4.ferm.+PO4.apporte+PO4.utilise; 0.387, 0.714, pH+Main.P-Source.Conc.+Nitrogen.Conc.+OD+X.F.Suc+X.VXdt+yi eld+PL+rab+lactate+PO4.ferm.+PO4.apporte+PO4.utilise; 0.398, 0.714, pH+Main.P-Source.Conc.+Nitrogen.Conc.+X.F.Suc+X.VXd t+yield+emission_O2+emission_CO2+His+PO4.ferm.+PO4.apporte+ PO4.utilise; 0.362, 0.714, pH+tmp+Main.Thr.Conc.+Nitrogen.C onc.+X.F.Suc+X.VXdt+total.cell+yield+rab+Thr+His+SO4.ferm.+ PO4.ferm.+PO4.apporte+PO4.utilise; 0.398, 0.714, pH+Main.P-Source.Conc.+Nitrogen.Conc.+X.F.Suc+X.VXdt+yield+emission_C O2+rab+lactate+PO4.ferm.+PO4.apporte+PO4.utilise; 0.398, 0. 714, pH+tmp+Nitrogen.Conc.+OD+X.F.Suc+X.VXdt+total.cell+yie ld+rab+PO4.ferm.+PO4.apporte+PO4.utilise; 0.387, 0.714, pH+ Nitrogen.Conc.+OD+X.F.Suc+X.VXdt+yield+PL+emission_CO2+rab+ lactate+PO4.ferm.+PO4.apporte+PO4.utilise; 0.375, 0.714, pH +Nitrogen.Conc.+OD+X.F.Suc+X.VXdt+yield+rab+Thr+His+lactate +SO4.ferm.+PO4.ferm.+PO4.apporte+PO4.utilise; 0.398, 0.714, pH+Main.P-Source.Conc.+Nitrogen.Conc.+X.F.Suc+X.VXdt+yield +emission_O2+emission_CO2+rab+PO4.ferm.+PO4.apporte+PO4.uti lise; 0.387, 0.714, pH+tmp+Main.Thr.Conc.+Nitrogen.Conc.+X. F.Suc+X.VXdt+total.cell+yield+emission_O2+Thr+PO4.ferm.+PO4. apporte+PO4.utilise; 0.386, 0.714, pH+Main.P-Source.Conc.+N itrogen.Conc.+OD+X.F.Suc+X.VXdt+yield+PL+emission_CO2+rab+P O4.ferm.+PO4.apporte+PO4.utilise; 0.430, 0.714, pH+tmp+Main. P-Source.Conc.+Nitrogen.Conc.+X.VXdt+total.cell+yield+rab+T hr; 0.398, 0.714, pH+Nitrogen.Conc.+X.F.Suc+X.VXdt+yield+em ission_O2+emission_CO2+His+lactate+PO4.ferm.+PO4.apporte+PO 4.utilise; 0.374, 0.714, pH+Nitrogen.Conc.+OD+X.F.Suc+X.VXd t+yield+PL+emission_O2+emission_CO2+rab+His+PO4.ferm.+PO4.a pporte+PO4.utilise; 0.420, 0.714, pH+tmp+X.VXdt+total.cell+ yield+PL+rab+Thr+His+PO4.utilise; 0.362, 0.714, pH+tmp+Nitr ogen.Conc.+OD+X.F.Suc+X.VXdt+yield+emission_O2+emission_CO2 +rab+Thr+His+PO4.ferm.+PO4.apporte+PO4.utilise; 0.398, 0.71 4, pH+P-Source.Feed.conc.+Main.Thr.Conc.+Nitrogen.Conc.+X.F. Suc+X.VXdt+yield+emission_O2+Thr+PO4.ferm.+PO4.apporte+PO4. utilise; 0.374, 0.714, pH+tmp+Nitrogen.Conc.+OD+X.F.Suc+X.V Xdt+yield+emission_O2+Thr+His+SO4.ferm.+PO4.ferm.+PO4.appor te+PO4.utilise; 0.398, 0.714, pH+Main.Thr.Conc.+Nitrogen.Co nc.+X.F.Suc+X.VXdt+yield+PL+rab+lactate+PO4.ferm.+PO4.appor te+PO4.utilise; 0.386, 0.714, pH+tmp+Main.Thr.Conc.+Nitroge n.Conc.+X.F.Suc+X.VXdt+total.cell+yield+rab+Thr+PO4.ferm.+P O4.apporte+PO4.utilise; 0.386, 0.713, pH+Main.P-Source.Conc. +Nitrogen.Conc.+X.F.Suc+X.VXdt+yield+emission_O2+rab+Thr+la ctate+PO4.ferm.+PO4.apporte+PO4.utilise; 0.398, 0.713, pH+M ain.Thr.Conc.+Nitrogen.Conc.+X.F.Suc+X.VXdt+total.cell+yiel d+PL+rab+PO4.ferm.+PO4.apporte+PO4.utilise; 0.420, 0.713, p H+tmp+X.F.Suc+yield+emission_O2+Thr+His+PO4.ferm.+PO4.appor te+PO4.utilise; 0.386, 0.713, pH+Main.P-Source.Conc.+Nitrog en.Conc.+X.F.Suc+X.VXdt+yield+emission_O2+emission_CO2+rab+ Thr+PO4.ferm.+PO4.apporte+PO4.utilise; 0.374, 0.713, pH+tmp +Main.P-Source.Conc.+Nitrogen.Conc.+X.F.Suc+X.VXdt+yield+PL +emission_O2+Thr+His+PO4.ferm.+PO4.apporte+PO4.utilise; 0.3 98, 0.713, pH+tmp+Nitrogen.Conc.+X.F.Suc+yield+emission_CO2 +rab+Thr+His+PO4.ferm.+PO4.apporte+PO4.utilise; 0.420, 0.71 3, pH+tmp+Nitrogen.Conc.+X.VXdt+total.cell+yield+PL+rab+Thr +PO4.utilise; 0.430, 0.713, pH+tmp+Main.P-Source.Conc.+X.VX dt+total.cell+yield+rab+Thr+His; 0.386, 0.713, pH+Nitrogen. Conc.+OD+X.F.Suc+X.VXdt+yield+emission_O2+Thr+lactate+SO4.f erm.+PO4.ferm.+PO4.apporte+PO4.utilise; 0.362, 0.713, pH+Ma in.P-Source.Conc.+Main.Thr.Conc.+Nitrogen.Conc.+OD+X.F.Suc+ X.VXdt+yield+PL+rab+Thr+His+PO4.ferm.+PO4.apporte+PO4.utili se; 0.386, 0.713, pH+Nitrogen.Conc.+X.F.Suc+X.VXdt+yield+PL +emission_O2+rab+His+lactate+PO4.ferm.+PO4.apporte+PO4.util ise; 0.386, 0.713, pH+Main.P-Source.Conc.+Main.Thr.Conc.+Ni trogen.Conc.+X.F.Suc+X.VXdt+total.cell+yield+emission_O2+Th r+PO4.ferm.+PO4.apporte+PO4.utilise; 0.386, 0.713, pH+Main. Thr.Conc.+Nitrogen.Conc.+X.F.Suc+X.VXdt+yield+PL+emission_O 2+rab+Thr+PO4.ferm.+PO4.apporte+PO4.utilise; 0.362, 0.713, pH+P-Source.Feed.conc.+Nitrogen.Conc.+OD+X.F.Suc+X.VXdt+yie ld+PL+emission_CO2+rab+Thr+His+PO4.ferm.+PO4.apporte+PO4.ut ilise; 0.374, 0.713, pH+tmp+Nitrogen.Conc.+X.F.Suc+X.VXdt+y ield+emission_O2+emission_CO2+rab+Thr+His+PO4.ferm.+PO4.app orte+PO4.utilise; 0.398, 0.713, pH+tmp+Nitrogen.Conc.+X.VXd t+total.cell+yield+emission_O2+Thr+His+PO4.ferm.+PO4.apport e+PO4.utilise; 0.386, 0.713, pH+P-Source.Feed.conc.+Nitroge n.Conc.+X.F.Suc+X.VXdt+yield+PL+emission_CO2+rab+Thr+PO4.fe rm.+PO4.apporte+PO4.utilise; 0.374, 0.713, pH+tmp+Nitrogen. Conc.+X.F.Suc+X.VXdt+total.cell+yield+PL+emission_O2+emissi on_CO2+rab+PO4.ferm.+PO4.apporte+PO4.utilise; 0.374, 0.713, pH+tmp+Main.Thr.Conc.+Nitrogen.Conc.+X.F.Suc+X.VXdt+total. ell+yield+emission_CO2+Thr+His+PO4.ferm.+PO4.apporte+PO4.u tilise; 0.386, 0.713, pH+tmp+Nitrogen.Conc.+OD+X.F.Suc+X.VX dt+total.cell+yield+rab+Thr+PO4.ferm.+PO4.apporte+PO4.utili se; 0.374, 0.713, pH+Main.Thr.Conc.+Nitrogen.Conc.+X.F.Suc+ X.VXdt+total.cell+yield+emission_O2+rab+Thr+His+PO4.ferm.+P O4.apporte+PO4.utilise; 0.386, 0.713, pH+Main.P-Source.Conc. +Main.Thr.Conc.+Nitrogen.Conc.+X.F.Suc+X.VXdt+yield+PL+emis sion_O2+Thr+PO4.ferm.+PO4.apporte+PO4.utilise; 0.374, 0.713, pH+Nitrogen.Conc.+X.F.Suc+X.VXdt+total.cell+yield+PL+emiss ion_O2+Thr+His+lactate+PO4.ferm.+PO4.apporte+PO4.utilise; 0. 420, 0.713, pH+tmp+X.VXdt+total.cell+yield+emission_O2+emis sion_CO2+rab+Thr+His; 0.386, 0.713, pH+P-Source.Feed.conc.+ Nitrogen.Conc.+X.F.Suc+X.VXdt+yield+PL+emission_O2+Thr+lact ate+PO4.ferm.+PO4.apporte+PO4.utilise; 0.386, 0.713, pH+Nit rogen.Conc.+X.F.Suc+X.VXdt+total.cell+yield+PL+emission_CO2 +rab+lactate+PO4.ferm.+PO4.apporte+PO4.utilise; 0.374, 0.71 3, pH+Main.P-Source.Conc.+Nitrogen.Conc.+X.F.Suc+X.VXdt+tot al.cell+yield+emission_CO2+rab+Thr+His+PO4.ferm.+PO4.apport e+PO4.utilise; 0.386, 0.713, pH+P-Source.Feed.conc.+Nitroge n.Conc.+X.F.Suc+X.VXdt+yield+emission_O2+Thr+His+lactate+PO 4.ferm.+PO4.apporte+PO4.utilise; 0.374, 0.713, pH+Nitrogen. Conc.+OD+X.F.Suc+X.VXdt+yield+emission_O2+rab+Thr+His+SO4.f erm.+PO4.ferm.+PO4.apporte+PO4.utilise; 0.374, 0.713, pH+Ni trogen.Conc.+OD+X.F.Suc+X.VXdt+yield+PL+emission_O2+Thr+His +SO4.ferm.+PO4.ferm.+PO4.apporte+PO4.utilise; 0.374, 0.713, pH+P-Source.Feed.conc.+Main.Thr.Conc.+Nitrogen.Conc.+OD+X. F.Suc+X.VXdt+yield+rab+Thr+His+PO4.ferm.+PO4.apporte+PO4.ut ilise; 0.397, 0.713, pH+Main.Thr.Conc.+Nitrogen.Conc.+OD+X. F.Suc+X.VXdt+yield+emission_CO2+Thr+PO4.ferm.+PO4.apporte+P O4.utilise; 0.409, 0.713, Nitrogen.Conc.+OD+X.F.Suc+X.VXdt+ yield+PL+rab+Thr+PO4.ferm.+PO4.apporte+PO4.utilise; 0.386, 0.713, pH+tmp+Main.P-Source.Conc.+Nitrogen.Conc.+X.F.Suc+X. VXdt+total.cell+yield+emission_O2+rab+PO4.ferm.+PO4.apporte +PO4.utilise; 0.386, 0.713, pH+Main.P-Source.Conc.+Nitrogen. Conc.+OD+X.F.Suc+X.VXdt+yield+emission_O2+rab+His+PO4.ferm. +PO4.apporte+PO4.utilise; 0.419, 0.713, pH+tmp+P-Source.Fee d.conc.+X.VXdt+total.cell+yield+PL+emission_CO2+Thr+His; 0. 397, 0.713, pH+Nitrogen.Conc.+OD+X.F.Suc+X.VXdt+total.cell+ yield+rab+Thr+PO4.ferm.+PO4.apporte+PO4.utilise; 0.430, 0.7 13, pH+tmp+Nitrogen.Conc.+X.VXdt+total.cell+yield+PL+emissi on_CO2+Thr; 0.419, 0.713, pH+tmp+Nitrogen.Conc.+X.VXdt+tota l.cell+yield+rab+PO4.ferm.+PO4.apporte+PO4.utilise; 0.408, 0.713, pH+tmp+Nitrogen.Conc.+X.VXdt+total.cell+yield+PL+emi ssion_CO2+Thr+His+lactate; 0.374, 0.713, pH+tmp+Nitrogen.Co nc.+OD+X.F.Suc+X.VXdt+yield+PL+emission_O2+rab+Thr+PO4.ferm. +PO4.apporte+PO4.utilise; 0.386, 0.713, pH+Main.P-Source.Co nc.+Nitrogen.Conc.+X.F.Suc+X.VXdt+yield+emission_O2+Thr+His +lactate+PO4.ferm.+PO4.apporte+PO4.utilise; 0.374, 0.713, p H+Nitrogen.Conc.+X.F.Suc+X.VXdt+total.cell+yield+PL+emissio n_O2+emission_CO2+Thr+lactate+PO4.ferm.+PO4.apporte+PO4.uti lise; 0.397, 0.713, pH+tmp+Main.P-Source.Conc.+Nitrogen.Con c.+X.F.Suc+X.VXdt+yield+emission_CO2+His+PO4.ferm.+PO4.appo rte+PO4.utilise; 0.397, 0.713, pH+tmp+Main.P-Source.Conc.+N itrogen.Conc.+X.F.Suc+X.VXdt+yield+emission_CO2+rab+PO4.fer m.+PO4.apporte+PO4.utilise; 0.361, 0.713, pH+tmp+Nitrogen.C onc.+OD+X.F.Suc+X.VXdt+total.cell+yield+PL+emission_O2+Thr+ His+PO4.ferm.+PO4.apporte+PO4.utilise; 0.397, 0.713, pH+P-S ource.Feed.conc.+Nitrogen.Conc.+X.F.Suc+X.VXdt+yield+PL+emi ssion_O2+rab+PO4.ferm.+PO4.apporte+PO4.utilise; 0.408, 0.71 3, pH+tmp+X.F.Suc+yield+emission_CO2+rab+Thr+His+PO4.ferm.+ PO4.apporte+PO4.utilise; 0.374, 0.713, pH+P-Source.Feed.con c.+Nitrogen.Conc.+X.F.Suc+X.VXdt+total.cell+yield+rab+Thr+H is+lactate+PO4.ferm.+PO4.apporte+PO4.utilise; 0.386, 0.713, pH+Main.Thr.Conc.+Nitrogen.Conc.+X.F.Suc+X.VXdt+total.cell +yield+PL+emission_O2+Thr+PO4.ferm.+PO4.apporte+PO4.utilis e; 0.374, 0.713, pH+Main.Thr.Conc.+Nitrogen.Conc.+X.F.Suc+X. VXdt+yield+PL+rab+Thr+His+lactate+PO4.ferm.+PO4.apporte+PO4. utilise; 0.361, 0.713, pH+tmp+Nitrogen.Conc.+OD+X.F.Suc+X.V Xdt+total.cell+yield+PL+rab+Thr+His+PO4.ferm.+PO4.apporte+P O4.utilise; 0.373, 0.713, pH+P-Source.Feed.conc.+Main.Thr.C onc.+Nitrogen.Conc.+X.F.Suc+X.VXdt+total.cell+yield+rab+Thr +His+PO4.ferm.+PO4.apporte+PO4.utilise; 0.397, 0.713, pH+Ma in.Thr.Conc.+Nitrogen.Conc.+X.F.Suc+X.VXdt+total.cell+yield +emission_O2+rab+PO4.ferm.+PO4.apporte+PO4.utilise; 0.385, 0.713, pH+Main.Thr.Conc.+Nitrogen.Conc.+X.F.Suc+X.VXdt+yiel d+PL+emission_O2+Thr+lactate+PO4.ferm.+PO4.apporte+PO4.util ise; 0.397, 0.713, pH+Main.Thr.Conc.+Nitrogen.Conc.+X.F.Suc +X.VXdt+yield+rab+His+lactate+PO4.ferm.+PO4.apporte+PO4.uti lise; 0.361, 0.713, pH+Main.Thr.Conc.+Nitrogen.Conc.+OD+X.F. Suc+X.VXdt+yield+PL+emission_CO2+rab+Thr+His+PO4.ferm.+PO4. apporte+PO4.utilise; 0.429, 0.713, pH+tmp+Main.P-Source.Con c.+Nitrogen.Conc.+X.VXdt+total.cell+yield+emission_O2+Thr; 0.373, 0.713, pH+Main.P-Source.Conc.+P-Source.Feed.conc.+Ni trogen.Conc.+X.F.Suc+X.VXdt+total.cell+yield+PL+rab+Thr+PO4. ferm.+PO4.apporte+PO4.utilise; 0.373, 0.713, pH+Nitrogen.Co nc.+X.F.Suc+X.VXdt+total.cell+yield+emission_O2+emission_CO 2+rab+Thr+lactate+PO4.ferm.+PO4.apporte+PO4.utilise; 0.373, 0.713, pH+Main.P-Source.Conc.+Main.Thr.Conc.+Nitrogen.Conc. +OD+X.F.Suc+X.VXdt+yield+emission_O2+rab+Thr+PO4.ferm.+PO4. apporte+PO4.utilise; 0.361, 0.713, pH+Main.P-Source.Conc.+N itrogen.Conc.+OD+X.F.Suc+X.VXdt+yield+PL+emission_O2+rab+Th r+lactate+PO4.ferm.+PO4.apporte+PO4.utilise; 0.397, 0.713, pH+tmp+Nitrogen.Conc.+X.F.Suc+yield+emission_O2+emission_CO 2+Thr+His+PO4.ferm.+PO4.apporte+PO4.utilise; 0.397, 0.713, pH+Nitrogen.Conc.+OD+X.F.Suc+X.VXdt+yield+emission_CO2+Thr+ lactate+PO4.ferm.+PO4.apporte+PO4.utilise; 0.397, 0.713, pH +Nitrogen.Conc.+X.F.Suc+X.VXdt+total.cell+yield+PL+emission _O2+lactate+PO4.ferm.+PO4.apporte+PO4.utilise; 0.397, 0.713, pH+tmp+Nitrogen.Conc.+X.F.Suc+X.VXdt+total.cell+yield+PL+e mission_CO2+PO4.ferm.+PO4.apporte+PO4.utilise; 0.361, 0.713, pH+tmp+Nitrogen.Conc.+X.F.Suc+X.VXdt+total.cell+yield+PL+e mission_CO2+Thr+His+lactate+PO4.ferm.+PO4.apporte+PO4.utili se; 0.373, 0.713, pH+Nitrogen.Conc.+X.F.Suc+X.VXdt+yield+PL +emission_O2+rab+Thr+His+lactate+PO4.ferm.+PO4.apporte+PO4. utilise; 0.385, 0.713, pH+Nitrogen.Conc.+OD+X.F.Suc+X.VXdt+ yield+PL+emission_O2+His+lactate+PO4.ferm.+PO4.apporte+PO4. utilise; 0.385, 0.713, pH+P-Source.Feed.conc.+Nitrogen.Conc. +X.F.Suc+X.VXdt+total.cell+yield+PL+rab+His+PO4.ferm.+PO4.a pporte+PO4.utilise; 0.385, 0.713, pH+P-Source.Feed.conc.+Ni trogen.Conc.+X.F.Suc+X.VXdt+yield+emission_CO2+rab+Thr+His+ PO4.ferm.+PO4.apporte+PO4.utilise; 0.373, 0.713, pH+tmp+Mai n.P-Source.Conc.+Nitrogen.Conc.+X.F.Suc+X.VXdt+yield+PL+emi ssion_CO2+Thr+His+PO4.ferm.+PO4.apporte+PO4.utilise; 0.373, 0.713, pH+Main.P-Source.Conc.+Nitrogen.Conc.+X.F.Suc+X.VXd t+yield+PL+emission_O2+rab+Thr+His+PO4.ferm.+PO4.apporte+PO 4.utilise; 0.397, 0.713, pH+tmp+Nitrogen.Conc.+X.F.Suc+X.VX dt+yield+PL+emission_O2+lactate+PO4.ferm.+PO4.apporte+PO4.u tilise; 0.385, 0.713, pH+Main.P-Source.Conc.+Nitrogen.Conc. +X.F.Suc+X.VXdt+yield+PL+emission_O2+rab+His+PO4.ferm.+PO4. apporte+PO4.utilise; 0.360, 0.713, pH+tmp+Main.Thr.Conc.+Ni trogen.Conc.+X.F.Suc+X.VXdt+total.cell+yield+PL+emission_O2 +Thr+His+PO4.ferm.+PO4.apporte+PO4.utilise; 0.385, 0.713, p H+tmp+Main.P-Source.Conc.+Nitrogen.Conc.+X.F.Suc+X.VXdt+yie ld+rab+Thr+His+PO4.ferm.+PO4.apporte+PO4.utilise; 0.373, 0. 713, pH+Main.Thr.Conc.+Nitrogen.Conc.+OD+X.F.Suc+X.VXdt+yie ld+rab+Thr+His+SO4.ferm.+PO4.ferm.+PO4.apporte+PO4.utilise; 0.429, 0.713, pH+tmp+Nitrogen.Conc.+X.F.Suc+yield+rab+PO4. ferm.+PO4.apporte+PO4.utilise; 0.360, 0.713, pH+Main.Thr.Co nc.+Nitrogen.Conc.+OD+X.F.Suc+X.VXdt+yield+PL+rab+Thr+His+l actate+PO4.ferm.+PO4.apporte+PO4.utilise; 0.347, 0.713, pH+ Nitrogen.Conc.+OD+X.F.Suc+X.VXdt+yield+PL+emission_O2+emiss ion_CO2+rab+Thr+His+lactate+PO4.ferm.+PO4.apporte+PO4.utili se; 0.373, 0.713, pH+P-Source.Feed.conc.+Nitrogen.Conc.+X.F. Suc+X.VXdt+total.cell+yield+PL+emission_O2+emission_CO2+Thr +PO4.ferm.+PO4.apporte+PO4.utilise; 0.373, 0.713, pH+Nitrog en.Conc.+OD+X.F.Suc+X.VXdt+yield+PL+emission_O2+rab+Thr+SO4. ferm.+PO4.ferm.+PO4.apporte+PO4.utilise; 0.385, 0.713, pH+P -Source.Feed.conc.+Nitrogen.Conc.+X.F.Suc+X.VXdt+yield+PL+e mission_O2+emission-CO2+Thr+PO4.ferm.+PO4.apporte+PO4.utili se; 0.397, 0.713, pH+tmp+Nitrogen.Conc.+X.VXdt+total.cell+y ield+rab+Thr+His+PO4.ferm.+PO4.apporte+PO4.utilise; 0.360, 0.713, pH+Main.Thr.Conc.+Nitrogen.Conc.+OD+X.F.Suc+X.VXdt+y ield+emission_O2+emission_CO2+rab+Thr+His+PO4.ferm.+PO4.app orte+PO4.utilise; 0.396, 0.713, pH+Main.Thr.Conc.+Nitrogen. Conc.+X.F.Suc+X.VXdt+yield+emission_O2+rab+His+PO4.ferm.+PO 4.apporte+PO4.utilise; 0.429, 0.713, pH+tmp+X.VXdt+total.ce ll+yield+emission_O2+Thr+His+PO4.utilise; 0.408, 0.713, pH+ tmp+P-Source.Feed.conc.+Nitrogen.Conc.+X.VXdt+total.cell+yi eld+emission_O2+Thr+His+PO4.utilise; 0.396, 0.713, pH+P-Sou rce.Feed.conc.+Nitrogen.Conc.+X.F.Suc+X.VXdt+total.cell+yie ld+emission_CO2+rab+PO4.ferm.+PO4.apporte+PO4.utilise; 0.38 5, 0.713, pH+Main.P-Source.Conc.+Nitrogen.Conc.+X.F.Suc+X.V Xdt+yield+PL+rab+His+lactate+PO4.ferm.+PO4.apporte+PO4.util ise; 0.418, 0.713, pH+tmp+Main.P-Source.Conc.+X.VXdt+total. cell+yield+PL+rab+Thr+His; 0.385, 0.713, pH+Nitrogen.Conc.+ X.F.Suc+X.VXdt+total.cell+yield+PL+emission_O2+emission_CO2 +His+PO4.ferm.+PO4.apporte+PO4.utilise; 0.373, 0.713, pH+tm p+Main.P-Source.Conc.+Nitrogen.Conc.+OD+X.F.Suc+X.VXdt+yiel d+emission_CO2+Thr+His+PO4.ferm.+PO4.apporte+PO4.utilise; 0. 373, 0.713, pH+P-Source.Feed.conc.+Nitrogen.Conc.+OD+X.F.Su c+X.VXdt+yield+rab+Thr+His+SO4.ferm.+PO4.ferm.+PO4.apporte+ PO4.utilise; 0.360, 0.713, pH+tmp+Main.Thr.Conc.+Nitrogen.C onc.+X.F.Suc+X.VXdt+total.cell+yield+emission_O2+Thr+His+SO 4.ferm.+PO4.ferm.+PO4.apporte+PO4.utilise; 0.429, 0.713, pH +tmp+X.VXdt+total.cell+yield+emission_CO2+rab+Thr+His; 0.40 8, 0.713, pH+tmp+X.F.Suc+yield+PL+emission_O2+Thr+His+PO4.f erm.+PO4.apporte+PO4.utilise; 0.373, 0.713, pH+tmp+Nitrogen. Conc.+OD+X.F.Suc+X.VXdt+yield+rab+Thr+His+SO4.ferm.+PO4.fer m.+PO4.apporte+PO4.utilise; 0.418, 0.713, pH+tmp+Main.P-Sou rce.Conc.+Nitrogen.Conc.+X.VXdt+total.cell+yield+PL+rab+Th r; 0.385, 0.713, pH+Main.P-Source.Conc.+Nitrogen.Conc.+X.F. Suc+X.VXdt+yield+emission_CO2+rab+Thr+lactate+PO4.ferm.+PO4. apporte+PO4.utilise; 0.407, 0.713, pH+Main.Thr.Conc.+Nitrog en.Conc.+OD+X.F.Suc+X.VXdt+yield+emission_O2+PO4.ferm.+PO4. apporte+PO4.utilise; 0.396, 0.713, pH+tmp+P-Source.Feed.con c.+Nitrogen.Conc.+X.VXdt+total.cell+yield+emission_O2+emiss ion_CO2+rab+Thr+His; 0.385, 0.713, pH+Main.P-Source.Cone.+P _Source.Feed.conc.+Nitrogen.Conc.+X.F.Suc+X.VXdt+yield+PL+r ab+Thr+PO4.ferm.+PO4.apporte+PO4.utilise; 0.396, 0.713, pH+ tmp+Nitrogen.Conc.+X.VXdt+total.cell+yield+PL+rab+Thr+PO4.f erm.+PO4.apporte+PO4.utilise; 0.385, 0.713, pH+Main.P-Sourc e.Conc.+Main.Thr.Conc.+Nitrogen.Conc.+X.F.Suc+X.VXdt+yield+ emission_O2+rab+Thr+PO4.ferm.+PO4.apporte+PO4.utilise; 0.36 0, 0.713, pH+Nitrogen.Conc.+X.F.Suc+X.VXdt+total.cell+yield +PL+emission_O2+emission_CO2+rab+Thr+His+PO4.ferm.+PO4.appo rte+PO4.utilise; 0.439, 0.713, pH+tmp+X.VXdt+total.cell+yie ld+rab+Thr+His; 0.407, 0.712, pH+P-Source.Feed.conc.+Nitrog en.Conc.+OD+X.F.Suc+X.VXdt+yield+emission_O2+PO4.ferm.+PO4. apporte+PO4.utilise; 0.372, 0.712, pH+tmp+Nitrogen.Conc.+X. F.Suc+X.VXdt+total.cell+yield+PL+emission_O2+His+lactate+PO 4.ferm.+PO4.apporte+PO4.utilise; 0.385, 0.712, pH+tmp+Main. P-Source.Conc.+Nitrogen.Conc.+X.F.Suc+X.VXdt+yield+PL+emiss ion_O2+Thr+PO4.ferm.+PO4.apporte+PO4.utilise; 0.396, 0.712, pH+Nitrogen.Conc.+X.F.Suc+X.VXdt+total.cell+yield+emission _O2+His+lactate+PO4.ferm.+PO4.apporte+PO4.utilise; 0.385, 0. 712, pH+Main.Thr.Conc.+Nitrogen.Conc.+X.F.Suc+X.VXdt+yield+ PL+emission_CO2+rab+Thr+PO4.ferm.+PO4.apporte+PO4.utilise; 0.396, 0.712, pH+Main.P-Source.Conc.+Main.Thr.Conc.+Nitroge n.Conc.+X.F.Suc+X.VXdt+yield+PL+rab+PO4.ferm.+PO4.apporte+P O4.utilise; 0.385, 0.712, pH+tmp+Main.P-Source.Conc.+Nitrog en.Conc.+X.F.Suc+X.VXdt+yield+emission_O2+rab+His+PO4.ferm. +PO4.apporte+PO4.utilise; 0.407, 0.712, pH+tmp+X.F.Suc+yiel d+emission_O2+emission_CO2+Thr+His+PO4.ferm.+PO4.apporte+PO 4.utilise; 0.384, 0.712, pH+Main.P-Source.Conc.+P-Source.Fe ed.conc.+Nitrogen.Conc.+X.F.Suc+X.VXdt+yield+rab+Thr+His+PO 4.ferm.+PO4.apporte+PO4.utilise; 0.372, 0.712, pH+P-Source. Feed.conc.+Main.Thr.Conc.+Nitrogen.Conc.+OD+X.F.Suc+X.VXdt+ yield+emission_O2+Thr+His+PO4.ferm.+PO4.apporte+PO4.utilis e; 0.396, 0.712, pH+tmp+Main.P-Source.Conc.+Nitrogen.Conc.+ X.VXdt+total.cell+yield+PL+rab+Thr+His+PO4.utilise; 0.372, 0.712, pH+Main.Thr.Conc.+Nitrogen.Conc.+OD+X.F.Suc+X.VXdt+y ield+emission_O2+rab+Thr+lactate+PO4.ferm.+PO4.apporte+PO4. utilise; 0.384, 0.712, pH+tmp+Nitrogen.Conc.+X.F.Suc+yield+ PL+emission_CO2+rab+Thr+His+PO4.ferm.+PO4.apporte+PO4.utili se; 0.384, 0.712, pH+Main.P-Source.Conc.+Nitrogen.Conc.+X.F. Suc+X.VXdt+total.cell+yield+PL+rab+lactate+PO4.ferm.+PO4.ap porte+PO4.utilise; 0.396, 0.712, pH+Nitrogen.Conc.+OD+X.F.S uc+X.VXdt+total.cell+yield+emission_O2+Thr+PO4.ferm.+PO4.ap porte+PO4.utilise; 0.384, 0.712, pH+tmp+Nitrogen.Conc.+OD+X. F.Suc+X.VXdt+total.cell+yield+emission_O2+His+PO4.ferm.+PO4. apporte+PO4.utilise; 0.384, 0.712, pH+Main.P-Source.Conc.+N itrogen.Conc.+X.F.Suc+X.VXdt+total.cell+yield+emission_O2+r ab+His+PO4.ferm.+PO4.apporte+PO4.utilise; 0.384, 0.712, pH+ Nitrogen.Conc.+OD+X.F.Suc+X.VXdt+yield+PL+emission_O2+emiss ion_CO2+rab+Thr+His+PO4.ferm.; 0.372, 0.712, pH+tmp+Nitroge n.Conc.+OD+X.F.Suc+X.VXdt+yield+PL+emission_CO2+rab+Thr+PO4. ferm.+PO4.apporte+PO4.utilise; 0.407, 0.712, pH+tmp+Nitroge n.Conc.+OD+X.VXdt+total.cell+yield+emission_O2+Thr+His+PO4. utilise; 0.372, 0.712, pH+Main.Thr.Conc.+Nitrogen.Conc.+X.F. Suc+X.VXdt+total.cell+yield+rab+Thr+His+lactate+PO4.ferm.+P O4.apporte+PO4.utilise; 0.372, 0.712, pH+tmp+Nitrogen.Conc. +X.F.Suc+X.VXdt+total.cell+yield+PL+rab+His+lactate+PO4.fer m.+PO4.apporte+PO4.utilise; 0.360, 0.712, pH+P-Source.Feed. conc.+Nitrogen.Conc.+OD+X.F.Suc+X.VXdt+yield+emission_O2+em ission_CO2+rab+Thr+His+PO4.ferm.+PO4.apporte+PO4.utilise; 0. 372, 0.712, pH+P-Source.Feed.conc.+Nitrogen.Conc.+X.F.Suc+X. VXdt+total.cell+yield+emission_O2+emission_CO2+rab+Thr+PO4. ferm.+PO4.apporte+PO4.utilise; 0.418, 0.712, pH+tmp+Nitroge n.Conc.+X.VXdt+total.cell+yield+emission_O2+Thr+His+PO4.app orte; 0.372, 0.712, pH+P-Source.Feed.conc.+Nitrogen.Conc.+X. F.Suc+X.VXdt+total.cell+yield+emission_CO2+rab+Thr+His+PO4. ferm.+PO4.apporte+PO4.utilise; 0.396, 0.712, pH+Main.P-Sour ce.Conc.+Nitrogen.Conc.+X.F.Suc+X.VXdt+yield+emission_O2+em ission_CO2+lactate+PO4.ferm.+PO4.apporte+PO4.utilise; 0.418, 0.712, pH+tmp+Main.P-Source.Conc.+Nitrogen.Conc.+X.F.Suc+y ield+emission_O2+PO4.ferm.+PO4.apporte+PO4.utilise; 0.372, 0.712, pH+tmp+Nitrogen.Conc.+X.F.Suc+X.VXdt+total.cell+yiel d+PL+emission_O2+Thr+SO4.ferm.+PO4.ferm.+PO4.apporte+PO4.ut ilise; 0.396, 0.712, pH+tmp+Main.P-Source.Conc.+Nitrogen.Co nc.+X.F.Suc+X.VXdt+yield+emission_O2+emission_CO2+PO4.ferm. +PO4.apporte+PO4.utilise; 0.359, 0.712, pH+P-Source.Feed.co nc.+Nitrogen.Conc.+OD+X.F.Suc+X.VXdt+yield+PL+emission_O2+T hr+His+lactate+PO4.ferm.+PO4.apporte+PO4.utilise; 0.384, 0. 712, pH+Main.P-Source.Conc.+Main.Thr.Conc.+Nitrogen.Conc.+X. F.Suc+X.VXdt+yield+emission_O2+Thr+lactate+PO4.ferm.+PO4.ap porte+PO4.utilise; 0.359, 0.712, pH+Main.P-Source.Conc.+Nit rogen.Conc.+OD+X.F.Suc+X.VXdt+yield+PL+emission_CO2+rab+Thr +lactate+PO4.ferm.+PO4.apporte+PO4.utilise

### [201. Linear Model that Predicts Arginine Product ion Amount in Interval 1]

0.526, 0.765, tmp+pH+PL+OD+Arg_conc._cumulo+rab_integral+em ission_O2+emission_CO2+RQ; 0.518, 0.765, tmp+pH+PL+OD+Arg_c onc._cumulo+cell_yield_cumulo+rab_integral+emission_O2+emis sion_CO2+RQ; 0.533, 0.765, tmp+pH+PL+OD+Arg_conc._cumulo+ce ll_yield_cumulo+rab_integral+emission_O2; 0.540, 0.764, tmp +pH+PL+OD+Arg_conc._cumulo+rab_integral+emission_O2; 0.524, 0.764, tmp+pH+PL+OD+Arg_conc._cumulo+cell_yield_cumulo+rab _integral+emission_O2+RQ; 0.531, 0.763, tmp+pH+PL+OD+Arg_co nc._cumulo+rab_integral+emission_O2+emission_CO2; 0.530, 0. 763, tmp+pH+PL+OD+Arg_conc._cumulo+rab_integral+emission_O2 +RQ; 0.523, 0.763, tmp+pH+PL+OD+Arg_conc._cumulo+cell_yield _cumulo+rab_integral+emission_O2+emission_CO2; 0.535, 0.762, tmp+pH+PL+Arg_conc._cumulo+rab_integral+emission_O2+emissi on_CO2; 0.527, 0.761, tmp+pH+PL+OD+Arg_conc._cumulo+rab_int egral+emission_O2+rab; 0.512, 0.761, tmp+pH+PL+OD+Arg_conc. _cumulo+rab_integral+emission_O2+emission_CO2+RQ+rab; 0.519, 0.761, tmp+pH+PL+OD+Arg_conc._cumulo+cell_yield_cumulo+rab _integral+emission_O2+rab; 0.541, 0.761, tmp+pH+PL+OD+rab_i ntegral+emission_O2; 0.503, 0.761, tmp+pH+PL+OD+Arg_conc._c umulo+cell_yield_cumulo+rab_integral+emission_O2+emission_C O2+RQ+rab; 0.526, 0.761, tmp+pH+PL+Arg_conc._cumulo+rab_int egral+emission_O2+emission_CO2+RQ; 0.526, 0.761, tmp+pH+PL+ OD+Sugar_consuming_rate_cumulo+Arg_conc._cumulo+rab_integra l+emission_O2; 0.518, 0.761, tmp+pH+PL+OD+Arg_conc._cumulo+ rab_integral+emission_O2+RQ+rab; 0.511, 0.761, tmp+pH+PL+AN +OD+Arg_conc._cumulo+rab_integral+emission_O2+emission_CO2+ RQ; 0.526, 0.760, tmp+pH+PL+AN+OD+Arg_conc._cumulo+rab_inte gral+emission_O2; 0.518, 0.760, tmp+pH+PL+OD+RS+Arg_conc._c umulo+cell_yield_cumulo+emission_CO2+RQ; 0.509, 0.760, tmp+ pH+PL+OD+Arg_conc._cumulo+cell_yield_cumulo+rab_integral+em ission_O2+RQ+rab; 0.509, 0.760, tmp+pH+PL+OD+Sugar_consumin g_rate_cumulo+Arg_conc._cumulo+rab_integral+emission_O2+emi ssion_CO2+RQ; 0.517, 0.760, tmp+pH+PL+OD+Arg_conc._cumulo+r ab_integral+emission_O2+emission_CO2+rab; 0.517, 0.760, tmp +pH+PL+AN+OD+Arg conc._cumulo+rab_integral+emission_O2+emis sion_CO2; 0.509, 0.759, tmp+pH+PL+OD+Arg_conc._cumulo+cell_ yield_cumulo+rab_integral+emission_O2+emission_CO2+rab; 0.5 16, 0.759, tmp+pH+PL+OD+Sugar_consuming_rate_cumulo+Arg_con c._cumulo+rab_integral+emission_O2+RQ; 0.538, 0.759, tmp+pH +PL+Arg_conc._cumulo+rab_integral+emission_O2; 0.516, 0.759, tmp+pH+PL+AN+OD+Arg_conc._cumulo+cell_yield_cumulo+rab-int egral+emission_O2; 0.515, 0.759, tmp+pH+PL+AN+OD+Arg_conc._ cumulo+rab_integral+emission_O2+RQ; 0.507, 0.759, tmp+pH+PL +AN+OD+RS+Arg_conc._cumulo+cell_yield_cumulo+emission_CO2+R Q; 0.499, 0.759, tmp+pH+PL+AN+OD+Arg_conc._cumulo+cell_yiel d_cumulo+rab_integral+emission_O2+emission_CO2+RQ; 0.499, 0. 759, tmp+pH+PL+OD+RS+Arg_conc._cumulo+cell_yield_cumulo+rab _integral+emission_O2+emission_CO2+RQ; 0.515, 0.759, tmp+pH +PL+AN+OD+RS+Arg_conc._cumulo+emission_CO2+RQ; 0.515, 0.758, tmp+pH+PL+OD+Sugar_consuming_rate_cumulo+Arg_conc._cumulo+ rab_integral+emission_O2+emission_CO2; 0.522, 0.758, tmp+pH + PL+Arg_conc._cumulo+rab_integral+emission_O2+emission_CO2+ rab; 0.514, 0.758, tmp+pH+PL+AN+OD+Arg_conc._cumulo+rab_int egral+emission_O2+rab; 0.528, 0.758, tmp+pH+PL+OD+rab_integ ral+emission_O2+rab; 0.505, 0.758, tmp+pH+PL+AN+OD+Arg_conc. _cumulo+cell_yield_cumulo+rab_integral+emission_O2+RQ; 0.50 5, 0.757, tmp+pH+PL+AN+OD+Arg_conc._cumulo+cell_yield_cumul o+rab_integral+emission_O2+emission_CO2; 0.528, 0.757, tmp+ pH+PL+OD+rab_integral+emission_O2+RQ; 0.520, 0.757, tmp+pH+ PL+AN+Arg_conc._cumulo+rab_integral+emission_O2+emission_CO 2; 0.504, 0.757, tmp+pH+PL+OD+RS+Arg_conc._cumulo+rab_integ ral+emission_O2+emission CO2+RQ; 0.512, 0.757, tmp+pH+PL+OD +Sugar_consuming_rate_cumulo+Arg_conc._cumulo+rab_integral+ emission_O2+rab; 0.512, 0.757, tmp+pH+PL+Arg_conc._cumulo+r ab_integral+emission_O2+emission_CO2+RQ+rab; 0.519, 0.757, tmp+pH+PL+OD+RS+Arg_conc._cumulo+emission_CO2+RQ; 0.512, 0. 757, tmp+pH+PL+AN+OD+RS+Arg_conc._cumulo+cell_yield_cumulo+ emission_CO2; 0.504, 0.757, tmp+pH+PL+OD+RS+Arg_conc._cumul o+cell_yield_cumulo+rab_integral+emission_O2+emission-CO2; 0.495, 0.757, tmp+pH+PL+AN+OD+Arg_conc._cumulo+rab_integral +emission_O2+emission_CO2+RQ+rab; 0.504, 0.757, tmp+pH+PL+O D+RS+Arg_conc._cumulo+cell_yield_cumulo+emission_CO2+RQ+ra b; 0.526, 0.757, tmp+pH+PL+Arg_conc._cumulo+rab_integral+em ission_O2+RQ; 0.519, 0.757, tmp+pH+PL+AN+OD+RS+Arg_conc._cu mulo+emission_CO2; 0.503, 0.757, tmp+pH+PL+AN+OD+Arg_conc._ cumulo+rab_integral+emission_O2+RQ+rab; 0.519, 0.756, tmp+p H+PL+Sugar_consuming_rate_cumulo+Arg_conc._cumulo+rab_integ ral+emission_O2+emission_CO2; 0.511, 0.756, tmp+pH+PL+OD+RS +Arg_conc._cumulo+cell_yield_cumulo+rab_integral+emission_O 2; 0.518, 0.756, tmp+pH+PL+OD+RS+Arg_conc._cumulo+cell_yiel d_cumulo+emission_CO2; 0.502, 0.756, tmp+pH+PL+OD+Sugar_con suming_rate_cumulo+Arg_conc._cumulo+rab_integral+emission_O 2+RQ+rab; 0.525, 0.756, tmp+pH+PL+Arg_conc._cumulo+rab_inte gral+emission_O2+rab; 0.510, 0.756, tmp+pH+PL+AN+OD+Sugar_c onsuming_rate_cumulo+Arg_conc._cumulo+rab_integral+emission _O2; 0.493, 0.756, tmp+pH+PL+OD+Sugar_consuming_rate_cumulo +Arg_conc._cumulo+rab_integral+emission_O2+emission_CO2+RQ+ rab; 0.509, 0.755, tmp+pH+PL+AN+Arg_conc._cumulo+rab_integr al+emission_O2+emission_CO2+RQ; 0.509, 0.755, tmp+pH+PL+Sug ar_consuming_rate_cumulo+Arg_conc._cumulo+rab_integral+emis sion_O2+emission_CO2+RQ; 0.509, 0.755, tmp+pH+PL+OD+RS+Arg_ conc._cumulo+rab_integral+emission_O2+emission_CO2; 0.501, 0.755, tmp+pH+PL+AN+OD+Arg_conc._cumulo+rab_integral+emissi on_O2+emission_CO2+rab; 0.501, 0.755, tmp+pH+PL+OD+RS+Arg_c onc._cumulo+cell_yield_cumulo+rab_integral+emission_O2+RQ; 0.524, 0.755, tmp+pH+PL+Sugar_consuming_rate_cumulo+Arg_con c._cumulo+rab_integral+emission_O2; 0.501, 0.755, tmp+pH+PL +AN+OD+Arg_conc._cumulo+cell_yield_cumulo+rab_integral+emis sion_O2+rab; 0.516, 0.755, tmp+pH+PL+OD+RS+Arg_conc._cumulo +rab_integral+emission_O2; 0.501, 0.755, tmp+pH+PL+OD+RS+Su gar_consuming_rate_cumulo+Arg_conc._cumulo+cell_yield_cumul o+emission_CO2+RQ; 0.523, 0.755, tmp+pH+PL+OD+RS+rab_integr al+emission_O2; 0.508, 0.754, tmp+pH+PL+OD+RS+Sugar_consumi ng_rate_cumulo+Arg_conc._cumulo+emission_CO2+RQ; 0.500, 0.7 54, tmp+pH+PL+OD+Sugar_consuming_rate_cumulo+Arg_conc._cumu lo+rab_integral+emission_O2+emission_CO2+rab; 0.522, 0.754, tmp+pH+PL+OD+rab_integral+emission_O2+emission_CO2; 0.522, 0.754, tmp+pH+PL+AN+Arg_conc._cumulo+rab_integral+emission _O2; 0.491, 0.754, tmp+pH+PL+AN+OD+Sugar_consuming_rate_cum ulo+Arg_conc._cumulo+rab_integral+emission_O2+emission_CO2+ RQ; 0.482, 0.754, tmp+pH+PL+OD+RS+Arg_conc._cumulo+cell_yie ld_cumulo+rab_integral+emission_O2+emission_CO2+RQ+rab; 0.5 15, 0.754, tmp+pH+PL+OD+rab_integral+emission_O2+RQ+rab; 0. 507, 0.754, tmp+pH+PL+OD+RS+Arg_conc._cumulo+cell_yield_cum ulo+emission_CO2+rab; 0.499, 0.754, tmp+pH+PL+AN+OD+Sugar_c onsuming_rate_cumulo+Arg_conc._cumulo+rab_integral+emission _O2+RQ; 0.498, 0.754, tmp+pH+PL+AN+OD+RS+Sugar_consuming_ra te_cumulo+Arg_conc._cumulo+emission_CO2+RQ; 0.490, 0.754, t mp+pH+PL+AN+OD+RS+Arg_conc._cumulo+cell_yield_cumulo+emissi on_CO2+RQ+rab; 0.482, 0.754, tmp+pH+PL+AN+OD+Arg_conc._cumu lo+cell_yield_cumulo+rab_integral+emission_O2+emission_CO2+ RQ+rab; 0.490, 0.753, tmp+pH+PL+AN+OD+Arg_conc._cumulo+cell yield_cumulo+rab_integral+emission_O2+RQ+rab; 0.521, 0.753, tmp+pH+PL+AN+OD+rab_integral+emission_O2; 0.506, 0.753, tm p+pH+PL+OD+RS+Arg_conc._cumulo+rab_integral+emission_O2+RQ; 0.490, 0.753, tmp+pH+PL+AN+OD+RS+Arg_conc._cumulo+rab_inte gral+emission_O2+emission_CO2+RQ; 0.498, 0.753, tmp+pH+PL+A N+OD+Sugar_consuming_rate_cumulo+Arg_conc._cumulo+rab_integ ral+emission_O2+emission_CO2; 0.506, 0.753, tmp+pH+PL+Sugar _consuming_rate_cumulo+Arg_conc._cumulo+rab_integral+emissi on_O2+emission_CO2+rab; 0.481, 0.753, tmp+pH+PL+AN+OD+RS+Ar g_conc._cumulo+cell_yield_cumulo+rab_integral+emission_O2+e mission_CO2+RQ; 0.497, 0.753, tmp+pH+PL+AN+OD+RS+Arg_conc._ cumulo+emission_CO2+RQ+rab; 0.497, 0.753, tmp+pH+PL+OD+RS+A rg_conc._cumulo+cell_yield_cumulo+rab_integral+emission_CO2 +RQ; 0.489, 0.753, tmp+pH+PL+OD+RS+Sugar_consuming_rate_cum ulo+Arg_conc._cumulo+rab_integral+emission_O2+emission_CO2+ RQ; 0.489, 0.753, tmp+pH+PL+OD+RS+Arg_conc._cumulo+cell_yie ld_cumulo+rab_integral+emission_O2+emission_CO2+rab; 0.513, 0.753, tmp+pH+PL+Arg_conc._cumulo+rab_integral+emission_O2 +RQ+rab; 0.520, 0.753, tmp+pH+PL+OD+Sugar_consuming_rate_cu mulo+rab_integral+emission_O2; 0.512, 0.753, tmp+pH+PL+Suga r_consuming_rate_cumulo+Arg_conc._cumulo+rab_integral+emiss ion_O2+RQ; 0.488, 0.752, tmp+pH+PL+AN+OD+Arg_conc._cumulo+c ell_yield_cumulo+rab_integral+emission_O2+emission_CO2+rab; 0.496, 0.752, tmp+pH+PL+AN+OD+RS+Arg_conc._cumulo+rab_inte gral+emission_O2+emission_CO2; 0.496, 0.752, tmp+pH+PL+OD+R S+Arg_conc._cumulo+cell_yield_cumulo+rab_integral+emission_ O2+rab; 0.519, 0.752, tmp+pH+PL+OD+RS+Arg_conc._cumulo+emis sion_CO2; 0.488, 0.752, tmp+pH+PL+OD+RS+Arg_conc._cumulo+ra b_integral+emission_O2+emission_CO2+RQ+rab; 0.512, 0.752, t mp+pH+PL+Sugar_consuming_rate_cumulo+Arg_conc._cumulo+cell_ yield_cumulo+rab_integral+emission_O2; 0.496, 0.752, tmp+pH +PL+AN+OD+RS+Arg_conc._cumulo+cell_yield_cumulo+emission_CO 2+rab; 0.504, 0.752, tmp+pH+PL+AN+Arg_conc._cumulo+rab_inte gral+emission_O2+emission_CO2+rab; 0.504, 0.752, tmp+pH+PL+ OD+RS+Sugar_consuming_rate_cumulo+Arg_conc._cumulo+rab_inte gral+emission_O2; 0.503, 0.752, tmp+pH+PL+AN+OD+RS+Sugar_co nsuming_rate_cumulo+Arg_conc._cumulo+emission_CO2; 0.487, 0. 752, tmp+pH+PL+AN+OD+RS+Arg_conc._cumulo+cell_yield_cumulo+ rab_integral+emission_O2+emission_CO2; 0.503, 0.752, tmp+pH +PL+OD+RS+Arg_conc._cumulo+emission_CO2+RQ+rab; 0.495, 0.75 2, tmp+pH+PL+Sugar_consuming_rate_cumulo+Arg_conc._cumulo+r ab_integral+emission_O2+emission_CO2+RQ+rab; 0.495, 0.752, tmp+pH+PL+AN+OD+Sugar_consuming_rate_cumulo+Arg_conc._cumul o+rab_integral+emission_O2+rab; 0.518, 0.752, tmp+pH+PL+OD+ RS+Arg_conc._cumulo+cell_yield_cumulo; 0.511, 0.752, tmp+pH +PL+OD+RS+rab_integral+emission_O2+rab; 0.486, 0.752, tmp+p H+PL+AN+OD+RS+Arg_conc._cumulo+cell_yield_cumulo+rab_integr al+emission_CO2+RQ; 0.510, 0.752, tmp+pH+PL+Arg_conc._cumul o+cell_yield_cumulo+rab_integral+emission_O2+emission_CO2; 0.510, 0.751, tmp+pH+PL+Sugar_consuming_rate_cumulo+Arg_con c._cumulo+rab_integral+emission_O2+rab; 0.502, 0.751, tmp+p H+PL+AN+OD+RS+Arg_conc._cumulo+rab_integral+emission_O2; 0. 494, 0.751, tmp+pH+PL+OD+RS+Arg_conc._cumulo+cell_yield_cum ulo+emission_O2+emission_CO2+RQ; 0.510, 0.751, tmp+pH+PL+RS +Arg_conc._cumulo+rab_integral+emission_O2+emission_CO2; 0. 494, 0.751, tmp+pH+PL+AN+OD+RS+Arg_conc._cumulo+cell_yield_ cumulo+rab_integral+emission_O2; 0.502, 0.751, tmp+pH+PL+Su gar_consuming_rate_cumulo+Arg_conc._cumulo+cell_yield_cumul o+rab_integral+emission_O2+emission_CO2; 0.510, 0.751, tmp+ pH+PL+AN+OD+RS+Arg_conc._cumulo+cell_yield_cumulo; 0.494, 0. 751, tmp+pH+PL+OD+RS+Sugar_consuming_rate_cumulo+Arg_conc.-cumulo+rab_integral+emission_O2+emission_CO2; 0.510, 0.751, tmp+pH+PL+OD+RS+rab_integral+emission_O2+RQ; 0.502, 0.751, tmp+pH+PL+OD+RS+Arg_conc._cumulo+rab_integral+emission_O2+ rab; 0.510, 0.751, tmp+pH+PL+OD+Arg_conc._cumulo+cell_yield _cumulo+emission_CO2+RQ; 0.502, 0.751, tmp+pH+PL+AN+OD+RS+A rg_conc._cumulo+emission_CO2+rab; 0.510, 0.751, tmp+pH+PL+A N+Arg_conc._cumulo+rab_integral+emission_O2+RQ; 0.517, 0.75 1, tmp+pH+PL+AN+OD+RS+Arg_conc._cumulo; 0.517, 0.751, tmp+p H+PL+OD+cell_yield_cumulo+rab_integral+emission_O2; 0.509, 0.751, tmp+pH+PL+OD+RS+Arg_conc._cumulo+cell_yield_cumulo+R Q; 0.501, 0.751, tmp+pH+PL+AN+Sugar_consuming_rate_cumulo+A rg_conc. _cumulo+rab_integral+emission_O2+emission_CO2; 0.50 9, 0.751, tmp+pH+PL+OD+rab_integral+emission_O2+emission_CO 2+rab; 0.493, 0.751, tmp+pH+PL+Sugar_consuming_rate_cumulo+ Arg_conc._cumulo+cell_yield_cumulo+rab_integral+emission_O2 +emission_CO2+RQ; 0.509, 0.751, tmp+pH+PL+OD+RS+Sugar_consu ming_rate_cumulo+Arg_conc._cumulo+emission_CO2; 0.509, 0.75 1, tmp+pH+PL+OD+rab_integral+emission_O2+emission_CO2+RQ; 0. 516, 0.751, tmp+pH+PL+Arg_conc._cumulo+cell_yield_cumulo+ra b_integral+emission_O2; 0.493, 0.751, tmp+pH+PL+OD+RS+Sugar _consuming_rate_cumulo+Arg_conc._cumulo+rab_integral+emissi on_O2+RQ; 0.493, 0.751, tmp+pH+PL+OD+RS+Arg_conc._cumulo+ra b_integral+emission_O2+emission_CO2+rab; 0.484, 0.750, tmp+ pH+PL+OD+RS+Arg_conc._cumulo+cell_yield_cumulo+rab_integral +emission_O2+RQ+rab; 0.501, 0.750, tmp+pH+PL+Arg_conc._cumu lo+cell_yield_cumulo+rab_integral+emission_O2+emission_CO2+ RQ; 0.508, 0.750, tmp+pH+PL+AN+Arg_conc._cumulo+rab_integra l+emission_O2+rab; 0.508, 0.750, tmp+pH+PL+AN+OD+rab_integr al+emission_O2+RQ; 0.492, 0.750, tmp+pH+PL+AN+Arg_conc._cum ulo+rab_integral+emission_O2+emission_CO2+RQ+rab; 0.484, 0. 750, tmp+pH+PL+OD+RS+Sugar_consuming_rate_cumulo+Arg_conc._ cumulo+cell_yield_cumulo+emission_CO2+RQ+rab; 0.484, 0.750, tmp+pH+PL+AN+OD+Sugar_consuming_rate_cumulo+Arg_conc._cumu lo+rab_integral+emission_O2+RQ+rab; 0.492, 0.750, tmp+pH+PL +OD+RS+Sugar_consuming_rate_cumulo+Arg_conc._cumulo+emissio n_CO2+RQ+rab; 0.500, 0.750, tmp+pH+PL+RS+Arg_conc._cumulo+r ab_integral+emission_O2+emission_CO2+RQ; 0.508, 0.750, tmp+ pH+PL+OD+Sugar_consuming_rate_cumulo+Arg_conc._cumulo+emiss ion_CO2+RQ; 0.508, 0.750, tmp+pH+PL+AN+OD+rab_integral+emis sion_O2+rab; 0.491, 0.750, tmp+pH+PL+OD+RS+Arg_conc._cumulo +rab_integral+emission_O2+RQ+rab; 0.507, 0.750, tmp+pH+PL+O D+Sugar_consuming_rate_cumulo+rab_integral+emission_O2+rab; 0.507, 0.750, tmp+pH+PL+OD+RS+rab_integral+emission_O2+emi ssion_CO2; 0.483, 0.750, tmp+pH+PL+AN+OD+RS+Arg_conc._cumul o+cell_yield_cumulo+emission_O2+emission_CO2+RQ; 0.491, 0.7 50, tmp+pH+PL+AN+OD+RS+Arg_conc._cumulo+cell_yield_cumulo+r ab_integral+emission_CO2; 0.491, 0.749, tmp+pH+PL+OD+RS+Sug ar_consuming_rate_cumulo+Arg_conc._cumulo+rab_integral+emis sion_CO2+RQ; 0.499, 0.749, tmp+pH+PL+Sugar_consuming_rate_c umulo+Arg_conc._cumulo+cell_yield_cumulo+rab_integral+emiss ion_O2+RQ; 0.483, 0.749, tmp+pH+PL+OD+RS+Arg_conc._cumulo+c ell_yield_cumulo+rab_integral+emission_CO2+RQ+rab; 0.482, 0. 749, tmp+pH+PL+AN+OD+RS+Arg_conc._cumulo+cell_yield_cumulo+ rab_integral+emission_O2+RQ; 0.491, 0.749, tmp+pH+PL+AN+OD+ RS+Arg_conc._cumulo+rab_integral+emission_CO2+RQ; 0.506, 0. 749, tmp+pH+PL+AN+Sugar_consuming_rate_cumulo+Arg_conc._cum ulo+rab_integral+emission_O2; 0.490, 0.749, tmp+pH+PL+AN+OD +RS+Arg_conc._cumulo+rab_integral+emission_O2+RQ; 0.498, 0. 749, tmp+pH+PL+AN+OD+RS+Arg_conc._cumulo+cell_yield_cumulo+ RQ; 0.490, 0.749, tmp+pH+PL+AN+OD+RS+Arg_conc._cumulo+emiss ion_O2+emission_CO2+RQ; 0.473, 0.749, tmp+pH+PL+AN+OD+Sugar _consuming_rate_cumulo+Arg_conc._cumulo+rab_integral+emissi on_O2+emission_CO2+RQ+rab; 0.506, 0.749, tmp+pH+PL+OD+RS+Su gar_consuming_rate_cumulo+rab_integral+emission_O2; 0.490, 0.749, tmp+pH+PL+AN+Sugar_consuming_rate_cumulo+Arg_conc._c umulo+rab_integral+emission_O2+emission_CO2+RQ; 0.498, 0.74 9, tmp+pH+PL+OD+RS+Arg_conc._cumulo+emission_O2+emission_CO 2+RQ; 0.473, 0.749, tmp+pH+PL+AN+OD+RS+Arg_conc._cumulo+rab _integral+emission_O2+emission_CO2+RQ+rab; 0.498, 0.749, tm p+pH+PL+OD+RS+Sugar_consuming_rate_cumulo+Arg_conc._cumulo+ cell_yield_cumulo+emission_CO2; 0.498, 0.749, tmp+pH+PL+Sug ar_consuming_rate_cumulo+Arg_conc._cumulo+rab_integral+emis sion_O2+RQ+rab; 0.506, 0.749, tmp+pH+PL+AN+OD+RS+Arg_conc._ cumulo+RQ; 0.521, 0.749, tmp+pH+PL+OD+RS+Arg_conc._cumulo; 0.528, 0.749, tmp+pH+PL+OD+RS; 0.498, 0.749, tmp+pH+PL+OD+R S+Arg_conc._cumulo+cell_yield_cumulo+rab_integral+emission_ CO2; 0.505, 0.749, tmp+pH+PL+OD+Sugar_consuming_rate_cumulo +rab_integral+emission_O2+RQ; 0.498, 0.749, tmp+pH+PL+Sugar _consuming_rate_cumulo+Arg_conc._cumulo+cell_yield_cumulo+r ab_integral+emission_O2+rab; 0.513, 0.749, tmp+pH+PL+RS+Arg _conc._cumulo+rab_integral+emission_O2; 0.489, 0.748, tmp+p H+PL+AN+OD+RS+Arg_conc._cumulo+rab_integral+emission_O2+ra b; 0.463, 0.748, tmp+pH+PL+AN+OD+RS+Arg_conc._cumulo+cell_y ield_cumulo+rab_integral+emission_O2+emission_CO2+RQ+rab; 0. 505, 0.748, tmp+pH+PL+OD+RS+cell_yield_cumulo+rab_integral+ emission_O2; 0.480, 0.748, tmp+pH+PL+AN+OD+RS+Sugar_consumi ng_rate_cumulo+Arg_conc._cumulo+rab_integral+emission_CO2+R Q; 0.505, 0.748, tmp+pH+PL+AN+OD+RS+rab_integral+emission_O 2; 0.480, 0.748, tmp+pH+PL+AN+OD+Sugar_consuming_rate_cumul o+Arg_conc._cumulo+rab_integral+emission_O2+emission_CO2+ra b; 0.512, 0.748, tmp+pH+PL+Sugar_consuming_rate_cumulo+cell _yield_cumulo+rab_integral+emission_O2; 0.512, 0.748, tmp+p H+PL+OD+RS+Arg_conc._cumulo+RQ; 0.480, 0.748, tmp+pH+PL+AN+ OD+RS+Sugar_consuming_rate_cumulo+Arg_conc._cumulo+emission _CO2+RQ+rab; 0.471, 0.748, tmp+pH+PL+OD+RS+Sugar_consuming_ rate_cumulo+Arg_conc._cumulo+rab_integral+emission_O2+emiss ion_CO2+RQ+rab; 0.505, 0.748, tmp+pH+PL+OD+RS+Arg_conc._cum ulo+emission_CO2+rab; 0.488, 0.748, tmp+pH+PL+OD+RS+Sugar_c onsuming_rate_cumulo+Arg_conc._cumulo+cell_yield_cumulo+emi ssion_CO2+rab; 0.488, 0.748, tmp+pH+PL+OD+Arg_conc._cumulo+ rab_integral+RQ_integral+emission_O2+emission_CO2+RQ; 0.471, 0.748, tmp+pH+PL+AN+OD+RS+Sugar_consuming_rate_cumulo+Arg_ conc._cumulo+rab_integral+emission_O2+emission_CO2+RQ; 0.48 8, 0.748, tmp+pH+PL+OD+RS+Sugar_consuming_rate_cumulo+Arg_c onc._cumulo+rab_integral+emission_O2+rab; 0.512, 0.748, tmp +pH+PL+OD+Arg_conc._cumulo+emission_CO2+RQ; 0.504, 0.748, t mp+pH+PL+OD+RS+Arg_conc._cumulo+cell_yield_cumulo+rab; 0.49 6, 0.748, tmp+pH+PL+OD+RS+Arg_conc._cumulo+rab_integral+emi ssion_CO2+RQ; 0.504, 0.748, tmp+pH+PL+OD+Arg_conc._cumulo+e mission_O2+emission_CO2+RQ; 0.504, 0.748, tmp+pH+PL+OD+cell _yield_cumulo+rab_integral+emission_O2+rab; 0.496, 0.748, t mp+pH+PL+OD+RS+rab_integral+emission_O2+RQ+rab; 0.479, 0.74 8, tmp+pH+PL+AN+OD+RS+Arg_conc._cumulo+rab_integral+emissio n_O2+emission_CO2+rab; 0.504, 0.748, tmp+pH+PL+AN+OD+Arg_co nc._cumulo+emission_CO2+RQ; 0.488, 0.748, tmp+pH+PL+Sugar_c onsuming_rate_cumulo+Arg_conc._cumulo+cell_yield_cumulo+rab _integral+emission_O2+emission_CO2+rab; 0.496, 0.748, tmp+p H+PL+AN+OD+Arg_conc._cumulo+cell_yield_cumulo+emission_CO2+ RQ; 0.511, 0.748, tmp+pH+PL+OD+RS+Sugar_consuming_rate_cumu lo+Arg_conc._cumulo; 0.496, 0.748, tmp+pH+PL+Arg_conc._cumu lo+cell_yield_cumulo+rab_integral+emission_O2+emission_CO2+ rab; 0.479, 0.747, tmp+pH+PL+OD+RS+Arg_conc._cumulo+cell_yi eld_cumulo+emission_O2+emission_CO2+RQ+rab; 0.470, 0.747, t mp+pH+PL+AN+OD+RS+Arg_conc._cumulo+cell_yield_cumulo+rab_in tegral+emission_O2+emission_CO2+rab; 0.488, 0.747, tmp+pH+P L+AN+OD+RS+Arg_conc._cumulo+cell_yield_cumulo+emission_O2+e mission_CO2; 0.487, 0.747, tmp+pH+PL+OD+RS+Sugar_consuming_ rate_cumulo+Arg_conc._cumulo+cell_yield_cumulo+rab_integral +emission_O2; 0.503, 0.747, tmp+pH+PL+AN+OD+RS+Sugar_consum ing_rate_cumulo+Arg_conc._cumulo; 0.487, 0.747, tmp+pH+PL+A N+OD+RS+Sugar_consuming_rate_cumulo+Arg_conc._cumulo+rab_in tegral+emission_O2; 0.487, 0.747, tmp+pH+PL+OD+RS+Sugar_con suming_rate_cumulo+Arg_conc._cumulo+emission_O2+emission_CO 2+RQ; 0.479, 0.747, tmp+pH+PL+AN+OD+RS+Sugar_consuming_rate _cumulo+Arg_conc._cumulo+cell_yield_cumulo+emission_CO2+RQ; 0.503, 0.747, tmp+pH+PL+Arg_conc._cumulo+cell_yield_cumulo +rab_integral+emission_O2+RQ; 0.495, 0.747, tmp+pH+PL+AN+OD +RS+Arg_conc._cumulo+rab_integral+emission_CO2; 0.479, 0.74 7, tmp+pH+PL+AN+OD+RS+Arg_conc._cumulo+cell_yield_cumulo+ra b_integral+emission_O2+rab; 0.478, 0.747, tmp+pH+PL+AN+OD+R S+Sugar_consuming_rate_cumulo+Arg_conc._cumulo+rab_integral +emission_O2+emission_CO2; 0.503, 0.747, tmp+pH+PL+OD+cell_ yield_cumulo+rab_integral+emission_O2+RQ; 0.495, 0.747, tmp +pH+PL+OD+RS+Arg_conc._cumulo+cell_yield_cumulo+emission_O2 +emission_CO2; 0.495, 0.747, tmp+pH+PL+OD+RS+Sugar_consumin g_rate_cumulo+Arg_conc._cumulo+emission_CO2+rab; 0.510, 0.7 47, tmp+pH+PL+OD+Arg_conc._cumulo+cell_yield_cumulo+RQ; 0.4 95, 0.747, tmp+pH+PL+AN+OD+Sugar_consuming_rate_cumulo+Arg_ conc._cumulo+emission_CO2+RQ; 0.495, 0.747, tmp+pH+PL+AN+OD +RS+Arg_conc._cumulo+emission_O2+emission_CO2; 0.518, 0.747, tmp+pH+PL+cell_yield_cumulo+rab_integral+emission_O2; 0.49 5, 0.747, tmp+pH+PL+OD+rab_integral+emission_O2+emission_CO 2+RQ+rab; 0.469, 0.747, tmp+pH+PL+AN+OD+RS+Arg_conc._cumulo +cell_yield_cumulo+rab_integral+emission_CO2+RQ+rab; 0.495, 0.747, tmp+pH+PL+AN+Arg_conc._cumulo+rab_integral+emission _O2+RQ+rab; 0.518, 0.747, tmp+pH+PL+OD+Sugar_consuming_rate _cumulo+Arg_conc._cumulo; 0.518, 0.747, tmp+pH+PL+AN+OD+RS; 0.478, 0.747, tmp+pH+PL+OD+RS+Sugar_consuming_rate_cumulo+ Arg_conc._cumulo+rab_integral+emission_O2+emission_CO2+rab; 0.486, 0.747, tmp+pH+PL+AN+OD+RS+Arg_conc._cumulo+RQ_integ ral+emission_CO2+RQ; 0.486, 0.747, tmp+pH+PL+AN+OD+RS+Sugar _consuming_rate_cumulo+Arg_conc._cumulo+rab_integral+emissi on_CO2; 0.502, 0.747, tmp+pH+PL+AN+OD+RS+Arg_conc._cumulo+r ab; 0.494, 0.747, tmp+pH+PL+RS+Arg_conc._cumulo+rab_integra 1+emission_O2+emission_CO2+rab; 0.502, 0.747, tmp+pH+PL+AN+ OD+rab_integral+emission_O2+emission_CO2; 0.486, 0.747, tmp +pH+PL+AN+OD+RS+Sugar_consuming_rate_cumulo+Arg_conc._cumul o+emission_CO2+rab; 0.478, 0.747, tmp+pH+PL+Sugar_consuming _rate_cumulo+Arg_conc._cumulo+cell_yield_cumulo+rab_integra l+emission_O2+emission_CO2+RQ+rab; 0.486, 0.747, tmp+pH+PL+ OD+RS+Arg_conc._cumulo+cell_yield_cumulo+rab_integral+emiss ion_CO2+rab; 0.510, 0.747, tmp+pH+PL+OD+Sugar_consuming_rat e_cumulo+Arg_conc._cumulo+RQ; 0.502, 0.746, tmp+pH+PL+OD+RS +Sugar_consuming_rate_cumulo+Arg_conc._cumulo+RQ; 0.494, 0. 746, tmp+pH+PL+OD+RS+rab_integral+emission_O2+emission_CO2+ RQ; 0.502, 0.746, tmp+pH+PL+Arg_conc._cumulo+cell_yield_cum ulo+rab_integral+emission_O2+rab; 0.517, 0.746, tmp+pH+PL+O D+Arg_conc._cumulo+cell_yield_cumulo; 0.494, 0.746, tmp+pH+ PL+OD+RS+Sugar_consuming_rate_cumulo+rab_integral+emission_ O2+rab; 0.494, 0.746, tmp+pH+PL+AN+OD+RS+Arg_conc._cumulo+c ell_yield_cumulo+rab; 0.477, 0.746, tmp+pH+PL+AN+OD+RS+Arg_ conc._cumulo+rab_integral+emission_O2+RQ+rab; 0.517, 0.746, tmp+pH+PL+OD+RS+emission_CO2; 0.494, 0.746, tmp+pH+PL+RS+S ugar_consuming_rate_cumulo+Arg_conc._cumulo+rab_integral+em ission_O2+emission_CO2; 0.494, 0.746, tmp+pH+PL+AN+OD+rab_i ntegral+emission_O2+RQ+rab; 0.494, 0.746, tmp+pH+PL+OD+Arg_ conc._cumulo+cell_yield_cumulo+emission_O2+emission_CO2+RQ; 0.477, 0.746, tmp+pH+PL+OD+RS+Sugar_consuming_rate_cumulo+ Arg_conc._cumulo+cell_yield_cumulo+rab_integral+emission_O2 +emission_CO2; 0.494, 0.746, tmp+pH+PL+OD+RS+Sugar_consumin g_rate_cumulo+Arg_conc._cumulo+rab_integral+emission_CO2; 0. 494, 0.746, tmp+pH+PL+AN+Sugar_consuming_rate_cumulo+Arg_co nc._cumulo+rab_integral+emission_O2+RQ; 0.493, 0.746, tmp+p H+PL+OD+RS+rab_integral+emission_O2+emission_CO2+rab; 0.493, 0.746, tmp+pH+PL+AN+Sugar_consuming_rate_cumulo+Arg_conc._ cumulo+cell_yield_cumulo+rab_integral+emission_O2; 0.477, 0. 746, tmp+pH+PL+OD+RS+Sugar_consuming_rate_cumulo+Arg_conc._ cumulo+rab_integral+emission_O2+RQ+rab; 0.493, 0.746, tmp+p H+PL+OD+RS+Arg_conc._cumulo+cell_yield_cumulo+RQ+rab; 0.509, 0.746, tmp+pH+PL+OD+Arg_conc._cumulo+cell_yield_cumulo+emi ssion_CO2; 0.509, 0.746, tmp+pH+PL+OD+Sugar_consuming_rate_ cumulo+Arg_conc._cumulo+emission_CO2; 0.493, 0.746, tmp+pH+ PL+AN+RS+Arg_conc._cumulo+rab_integral+emission_O2+emission _CO2; 0.476, 0.746, tmp+pH+PL+AN+OD+RS+Sugar_consuming_rate cumulo+Arg_conc._cumulo+emission_O2+emission_CO2+RQ; 0.516, 0.746, tmp+pH+PL+OD+RS+RQ; 0.485, 0.746, tmp+pH+PL+Arg_con c._cumulo+cell_yield_cumulo+rab_integral+emission_O2+emissi on_CO2+RQ+rab; 0.516, 0.746, tmp+pH+PL+OD+RS+rab; 0.485, 0. 746, tmp+pH+PL+AN+Sugar_consuming_rate_cumulo+Arg_conc._cum ulo+rab_integral+emission_O2+emission_CO2+rab; 0.493, 0.746, tmp+pH+PL+OD+Arg_conc._cumulo+cell_yield_cumulo+emission_C O2+RQ+rab; 0.493, 0.746, tmp+pH+PL+OD+RS+cell_yield_cumulo+ rab_integral+emission_O2+rab; 0.492, 0.746, tmp+pH+PL+AN+OD +Arg_conc._cumulo+emission_O2+emission_CO2+RQ; 0.508, 0.746, tmp+pH+PL+AN+OD+RS+emission_CO2; 0.484, 0.745, tmp+pH+PL+S ugar_consuming_rate_cumulo+Arg_conc._cumulo+cell_yield_cumu lo+rab_integral+emission_O2+RQ+rab; 0.500, 0.745, tmp+pH+PL +RS+Arg_conc._cumulo+rab_integral+emission_O2+RQ; 0.475, 0. 745, tmp+pH+PL+AN+OD+RS+Arg_conc._cumulo+cell_yield_cumulo+ rab_integral+emission_CO2+rab; 0.507, 0.745, tmp+pH+PL+AN+O D+Sugar_consuming_rate_cumulo+Arg_conc._cumulo; 0.492, 0.74 5, tmp+pH+PL+OD+Sugar_consuming_rate_cumulo+rab_integral+em ission_O2+RQ+rab; 0.515, 0.745, tmp+pH+PL+OD+RS+Sugar_consu ming_rate_cumulo; 0.475, 0.745, tmp+pH+PL+AN+OD+RS+Sugar_co nsuming_rate_cumulo+Arg_conc._cumulo+rab_integral+emission-O2+RQ; 0.499, 0.745, tmp+pH+PL+AN+OD+Sugar_consuming_rate_c umulo+Arg_conc._cumulo+emission_CO2; 0.491, 0.745, tmp+pH+P L+OD+RS+Sugar_consuming_rate_cumulo+rab_integral+emission_O 2+RQ; 0.475, 0.745, tmp+pH+PL+OD+RS+Sugar_consuming_rate_cu mulo+Arg_conc._cumulo+rab_integral+emission_CO2+RQ+rab; 0.4 91, 0.745, tmp+pH+PL+AN+OD+RS+Sugar_consuming_rate_cumulo+A rg_conc._cumulo+RQ; 0.491, 0.745, tmp+pH+PL+AN+Sugar_consum ing_rate_cumulo+Arg_conc._cumulo+rab_integral+emission_O2+r ab; 0.507, 0.745, tmp+pH+PL+Arg_conc._cumulo+emission_O2+em ission_CO2+RQ; 0.466, 0.745, tmp+pH+PL+AN+OD+RS+Arg_conc._c umulo+cell_yield_cumulo+rab_integral+emission_O2+RQ+rab; 0. 474, 0.745, tmp+pH+PL+AN+OD+Arg_conc._cumulo+rab_integral+R Q_integral+emission_O2+emission_CO2+RQ; 0.507, 0.745, tmp+p H+PL+OD+RS+emission_CO2+RQ; 0.483, 0.745, tmp+pH+PL+RS+Suga r_consuming_rate_cumulo+Arg_conc._cumulo+rab_integral+emiss ion_O2+emission_CO2+RQ; 0.483, 0.745, tmp+pH+PL+RS+Arg_conc. cumulo+rab_integral+emission_O2+emission_CO2+RQ+rab; 0.499, 0.745, tmp+pH+PL+Sugar_consuming_rate_cumulo+cell_yield_cu mulo+rab_integral+emission_O2+rab; 0.491, 0.745, tmp+pH+PL+ AN+OD+RS+rab_integral+emission_O2+rab; 0.514, 0.745, tmp+pH +PL+rab_integral+emission_O2+rab; 0.483, 0.745, tmp+pH+PL+A N+Sugar_consuming_rate_cumulo+Arg_conc._cumulo+cell_yield_c umulo+rab_integral+emission_O2+emission_CO2; 0.483, 0.745, tmp+pH+PL+OD+RS+Arg_conc._cumulo+cell_yield_cumulo+emission _O2+emission_CO2+rab; 0.506, 0.745, tmp+pH+PL+AN+OD+Arg_con c._cumulo+cell_yield_cumulo; 0.499, 0.744, tmp+pH+PL+AN+OD+ Arg_conc._cumulo+cell_yield_cumulo+emission_CO2; 0.474, 0.7 44, tmp+pH+PL+OD+Arg_conc._cumulo+cell_yield_cumulo+rab_int egral+RQ_integral+emission_O2+emission_CO2+RQ; 0.490, 0.744, tmp+pH+PL+AN+OD+RS+Arg_conc._cumulo+RQ+rab; 0.506, 0.744, tmp+pH+PL+AN+OD+Arg_conc._cumulo+emission_CO2; 0.498, 0.744, tmp+pH+PL+RS+Sugar_consuming_rate_cumulo+Arg_conc._cumulo+ rab_integral+emission_O2; 0.490, 0.744, tmp+pH+PL+AN+OD+RS+ rab_integral+emission_O2+RQ; 0.465, 0.744, tmp+pH+PL+AN+OD+ RS+Arg_conc._cumulo+cell_yield_cumulo+emission_O2+emission-CO2+RQ+rab; 0.514, 0.744, tmp+pH+PL+OD+Arg_conc._cumulo+RQ; 0.498, 0.744, tmp+pH+PL+OD+RS+Arg_conc._cumulo+emission_O2 +emission_CO2; 0.498, 0.744, tmp+pH+PL+AN+OD+Sugar_consumin g_rate_cumulo+rab_integral+emission_O2; 0.482, 0.744, tmp+p H+PL+AN+OD+RS+Sugar_consuming_rate_cumulo+Arg_conc._cumulo+ emission_O2+emission_CO2; 0.513, 0.744, tmp+pH+PL+OD+RS+cel l_yield_cumulo; 0.490, 0.744, tmp+pH+PL+OD+RS+cell_yield_cu mulo+rab_integral+emission_O2+RQ; 0.490, 0.744, tmp+pH+PL+O D+Sugar_consuming_rate_cumulo+Arg_conc._cumulo+emission_O2+ emission_CO2+RQ; 0.506, 0.744, tmp+pH+PL+OD+RS+Arg_conc._cu mulo+rab; 0.506, 0.744, tmp+pH+PL+OD+RS+emission_CO2+rab; 0. 498, 0.744, tmp+pH+PL+OD+Sugar_consuming_rate_cumulo+rab_in tegral+emission_O2+emission_CO2; 0.490, 0.744, tmp+pH+PL+AN +Arg_conc._cumulo+cell_yield_cumulo+rab_integral+emission_O 2+emission_CO2; 0.506, 0.744, tmp+pH+PL+Sugar_consuming_rat e_cumulo+Arg_conc. _cumulo+emission_CO2+RQ; 0.473, 0.744, tm p+pH+PL+AN+OD+RS+Arg_conc._cumulo+rab_integral+emission_CO2 +RQ+rab; 0.473, 0.744, tmp+pH+PL+AN+OD+RS+Sugar_consuming_r ate_cumulo+Arg_conc._cumulo+cell_yield_cumulo+rab_integral+ emission_O2; 0.490, 0.744, tmp+pH+PL+OD+Sugar_consuming_rat e_cumulo+Arg_conc._cumulo+emission_CO2+RQ+rab; 0.498, 0.744, tmp+pH+PL+AN+Arg_conc._cumulo+cell_yield_cumulo+rab_integr al+emission_O2; 0.513, 0.744, tmp+pH+PL+Sugar_consuming_rat e_cumulo+Arg_conc._cumulo+emission_CO2; 0.513, 0.744, tmp+p H+PL+AN+OD+Arg_conc._cumulo; 0.464, 0.744, tmp+pH+PL+AN+OD+ RS+Sugar_consuming_rate_cumulo+Arg_conc._cumulo+cell_yield_ cumulo+rab_integral+emission_O2+emission_CO2; 0.497, 0.744, tmp+pH+PL+Sugar_consuming_rate_cumulo+cell_yield_cumulo+ra b_integral+emission_O2+RQ; 0.505, 0.744, tmp+pH+PL+AN+Arg_c onc._cumulo+emission_O2+emission_CO2; 0.497, 0.744, tmp+pH+ PL+RS+Arg_conc._cumulo+rab_integral+emission_O2+rab; 0.497, 0.744, tmp+pH+PL+OD+RS+Arg_conc._cumulo+cell_yield_cumulo+ rab_integral; 0.513, 0.744, tmp+pH+PL+Arg_conc._cumulo+emis sion_O2+emission_CO2; 0.473, 0.744, tmp+pH+PL+OD+Arg_conc._ cumulo+rab_integral+RQ _integral+emission_O2+emission_CO2+RQ +rab; 0.473, 0.744, tmp+pH+PL+AN+Sugar_consuming_rate_cumul o+Arg_conc._cumulo+rab_integral+emission_O2+emission-CO2+RQ +rab; 0.481, 0.744, tmp+pH+PL+AN+OD+RS+Arg_conc._cumulo+cel l_yield_cumulo+RQ+rab; 0.497, 0.744, tmp+pH+PL+OD+RS+Sugar_ consuming_rate_cumulo+Arg_conc._cumulo+rab_integral; 0.481, 0.744, tmp+pH+PL+OD+RS+Arg_conc._cumulo+emission_O2+emissi on_CO2+RQ+rab; 0.497, 0.744, tmp+pH+PL+AN+OD+Arg_conc._cumu lo+cell_yield_cumulo+RQ; 0.472, 0.744, tmp+pH+PL+AN+OD+RS+A rg_conc._cumulo+emission_O2+emission_CO2+RQ+rab; 0.489, 0.7 44, tmp+pH+PL+AN+OD+RS+Arg_conc._cumulo+cell_yield_cumulo+r ab_integral; 0.489, 0.744, tmp+pH+PL+OD+RS+Sugar_consuming_ rate _cumulo+Arg_conc. _cumulo+emission_O2+emission_CO2; 0.47 2, 0.744, tmp+pH+PL+AN+Sugar_consuming_rate_cumulo+Arg_conc. _cumulo+cell_yield_cumulo+rab_integral+emission_O2+emission CO2+RQ; 0.497, 0.744, tmp+pH+PL+AN+OD+Sugar_consuming_rate _cumulo+Arg_conc._cumulo+RQ; 0.481, 0.744, tmp+pH+PL+AN+RS+ Arg_conc._cumulo+rab_integral+emission_O2+emission_CO2+RQ; 0.505, 0.743, tmp+pH+PL+OD+RS+cell_yield_cumulo+rab; 0.472, 0.743, tmp+pH+PL+AN+OD+RS+Sugar_consuming_rate_cumulo+Arg_ conc._cumulo+rab_integral+emission_O2+rab; 0.504, 0.743, tm p+pH+PL+AN+OD+Arg_conc._cumulo+RQ; 0.489, 0.743, tmp+pH+PL+ AN+OD+rab_integral+emission_O2+emission_CO2+RQ; 0.489, 0.74 3, tmp+pH+PL+OD+RS+Arg_conc._cumulo+cell_yield_cumulo+rab_i ntegral+RQ; 0.496, 0.743, tmp+pH+PL+OD+RS+Arg_conc._cumulo+ RQ+rab; 0.488, 0.743, tmp+pH+PL+AN+OD+RS+rab_integral+emiss ion_O2+emission_CO2; 0.519, 0.743, tmp+pH+PL+OD+Arg_conc._c umulo; 0.496, 0.743, tmp+pH+PL+OD+cell_yield_cumulo+rab_int egral+emission_O2+emission_CO2; 0.472, 0.743, tmp+pH+PL+AN+ OD+RS+Arg_conc._cumulo+cell_yield_cumulo+RQ_integral+emissi on_CO2+RQ; 0.496, 0.743, tmp+pH+PL+OD+RS+Arg_conc._cumulo+r ab_integral+emission_CO2; 0.504, 0.743, tmp+pH+PL+AN+OD+RS+ RQ; 0.488, 0.743, tmp+pH+PL+OD+cell_yield_cumulo+rab_integr al+emission_O2+RQ+rab; 0.504, 0.743, tmp+pH+PL+AN+OD+RS+ra b; 0.496, 0.743, tmp+pH+PL+AN+RS+Arg_conc._cumulo+rab_integ ral+emission_O2; 0.480, 0.743, tmp+pH+PL+AN+Sugar_consuming _rate_cumulo+Arg_conc._cumulo+cell_yield_cumulo+rab_integra l+emission_O2+RQ; 0.463, 0.743, tmp+pH+PL+AN+OD+RS+Sugar_co nsuming_rate_cumulo+Arg_conc._cumulo+rab_integral+emission_ CO2+RQ+rab; 0.488, 0.743, tmp+pH+PL+AN+OD+RS+Sugar_consumin g_rate_cumulo+Arg_conc._cumulo+rab_integral; 0.488, 0.743, tmp+pH+PL+OD+Arg_conc._cumulo+rab_integral+RQ_integral+emis sion_O2+RQ; 0.504, 0.743, tmp+pH+PL+OD+RS+Sugar_consuming_r ate_cumulo+rab; 0.462, 0.743, tmp+pH+PL+AN+OD+RS+Sugar_cons uming_rate_cumulo+Arg_conc._cumulo+cell_yield_cumulo+emissi on_CO2+RQ+rab; 0.496, 0.743, tmp+pH+PL+OD+RS+Sugar_consumin g_rate_cumulo+Arg_conc._cumulo+rab; 0.504, 0.743, tmp+pH+PL +RS+Sugar_consuming_rate_cumulo+Arg_conc._cumulo+emission_C 02; 0.453, 0.743, tmp+pH+PL+AN+OD+RS+Sugar_consuming_rate_c umulo+Arg_conc._cumulo+rab_integral+emission-O2+emission-CO 2+RQ+rab; 0.496, 0.743, tmp+pH+PL+AN+Arg_conc._cumulo+emiss ion_O2+emission_CO2+RQ; 0.479, 0.743, tmp+pH+PL+OD+RS+Arg_c onc._cumulo+rab_integral+emission_CO2+RQ+rab; 0.503, 0.743, tmp+pH+PL+OD+Arg_conc. _cumulo+emission_O2+emission_CO2; 0. 471, 0.743, tmp+pH+PL+AN+OD+RS+Arg_conc._cumulo+cell_yield_ cumulo+emission_O2+emission_CO2+rab; 0.496, 0.743, tmp+pH+P L+AN+OD+RS+emission_CO2+RQ; 0.479, 0.743, tmp+pH+PL+OD+RS+r ab_integral+emission_O2+emission_CO2+RQ+rab; 0.488, 0.743, tmp+pH+PL+AN+OD+rab_integral+emission_O2+emission_CO2+rab; 0.503, 0.743, tmp+pH+PL+cell_yield_cumulo+rab_integral+emis sion_O2+rab; 0.495, 0.743, tmp+pH+PL+AN+OD+Arg_conc._cumulo +emission_O2+emission_CO2; 0.479, 0.743, tmp+pH+PL+AN+Arg_c onc._cumulo+cell_yield_cumulo+rab_integral+emission_O2+emis sion_CO2+RQ; 0.487, 0.743, tmp+pH+PL+Arg_conc._cumulo+cell_ yield_cumulo+rab_integral+emission_O2+RQ+rab; 0.518, 0.743, tmp+pH+PL+rab_integral+emission_O2; 0.503, 0.743, tmp+pH+P L+AN+RS+Arg_conc._cumulo+emission_CO2; 0.479, 0.743, tmp+pH +PL+RS+Sugar_consuming_rate_cumulo+Arg_conc._cumulo+rab_int egral+emission_O2+emission_CO2+rab; 0.470, 0.742, tmp+pH+PL +OD+RS+Sugar_consuming_rate_cumulo+Arg_conc._cumulo+emissio n_O2+emission_CO2+RQ+rab; 0.495, 0.742, tmp+pH+PL+RS+Sugar_ consuming_rate_cumulo+Arg_conc._cumulo+emission_CO2+RQ; 0.5 03, 0.742, tmp+pH+PL+rab_integral+emission_O2+RQ+rab; 0.479, 0.742, tmp+pH+PL+OD+RS+Sugar_consuming_rate_cumulo+Arg_con c._cumulo+rab_integral+emission_CO2+rab; 0.487, 0.742, tmp+ pH+PL+OD+RS+Sugar_consuming_rate_cumulo+Arg_conc._cumulo+ra b_integral+RQ; 0.510, 0.742, tmp+pH+PL+OD+Arg_conc._cumulo+ emission_CO2; 0.503, 0.742, tmp+pH+PL+OD+RS+RQ+rab; 0.495, 0.742, tmp+pH+PL+AN+OD+cell_yield_cumulo+rab_integral+emiss ion_O2; 0.487, 0.742, tmp+pH+PL+AN+OD+RS+Sugar_consuming_ra te_cumulo+Arg_conc._cumulo+rab; 0.495, 0.742, tmp+pH+PL+AN+ OD+RS+emission CO2+rab; 0.503, 0.742, tmp+pH+PL+OD+Arg_conc. _cumulo+emission_O2+RQ; 0.503, 0.742, tmp+pH+PL+AN+Sugar_co nsuming_rate_cumulo+Arg_conc._cumulo+emission_CO2; 0.510, 0. 742, tmp+pH+PL+RS+Arg_conc._cumulo+emission_CO2; 0.487, 0.7 42, tmp+pH+PL+OD+RS+Arg_conc._cumulo+RQ_integral+emission_C O2+RQ; 0.487, 0.742, tmp+pH+PL+OD+Sugar_consuming_rate_cumu lo+Arg_conc._cumulo+rab_integral+emission_CO2+RQ; 0.487, 0. 742, tmp+pH+PL+OD+RS+cell_yield_cumulo+rab_integral+emissio n_O2+emission_CO2; 0.495, 0.742, tmp+pH+PL+OD+Arg_conc._cum ulo+emission_CO2+RQ+rab; 0.495, 0.742, tmp+pH+PL+OD+RS+cell _yield_cumulo+emission_CO2+rab; 0.461, 0.742, tmp+pH+PL+AN+ OD+RS+Sugar_consuming_rate_cumulo+Arg_conc._cumulo+rab_inte gral+emission_O2+emission_CO2+rab; 0.495, 0.742, tmp+pH+PL+ OD+RS+emission_CO2+RQ+rab; 0.486, 0.742, tmp+pH+PL+OD+RS+Su gar_consuming_rate_cumulo+rab_integral+emission_O2+emission _CO2; 0.502, 0.742, tmp+pH+PL+cell_yield_cumulo+rab_integra l+emission_O2+RQ; 0.486, 0.742, tmp+pH+PL+OD+Sugar_consumin g_rate_cumulo+Arg_conc._cumulo+cell_yield_cumulo+rab_integr al+emission_O2; 0.478, 0.742, tmp+pH+PL+OD+RS+Sugar_consumi ng_rate_cumulo+rab_integral+emission_O2+RQ+rab; 0.486, 0.74 2, tmp+pH+PL+OD+Arg_conc. _cumulo+emission_O2+emission_CO2+R Q+rab; 0.502, 0.742, tmp+pH+PL+RS+Arg_conc._cumulo+emission CO2+RQ; 0.509, 0.742, tmp+pH+PL+OD+Arg_conc._cumulo+emissi on_O2; 0.478, 0.742, tmp+pH+PL+AN+OD+RS+Arg_conc._cumulo+ra b_integral+emission_CO2+rab; 0.478, 0.742, tmp+pH+PL+AN+Sug ar_consuming_rate_cumulo+Arg_conc._cumulo+rab_integral+emis sion_O2+RQ+rab; 0.478, 0.742, tmp+pH+PL+AN+Sugar_consuming_ rate_cumulo+Arg_conc._cumulo+cell_yield_cumulo+rab_integral +emission_O2+rab; 0.494, 0.742, tmp+pH+PL+AN+OD+RS+Arg_conc. _cumulo+rab_integral; 0.478, 0.742, tmp+pH+PL+AN+OD+Arg_con c._cumulo+cell_yield_cumulo+emission_O2+emission_CO2+RQ; 0. 502, 0.742, tmp+pH+PL+OD+RS+Sugar_consuming_rate_cumulo+emi ssion_CO2; 0.469, 0.742, tmp+pH+PL+AN+OD+RS+Sugar_consuming _rate_cumulo+Arg_conc._cumulo+rab_integral+emission_CO2+ra b; 0.502, 0.742, tmp+pH+PL+OD+RS+Sugar_consuming_rate_cumul o+RQ; 0.478, 0.742, tmp+pH+PL+AN+OD+RS+Arg_conc._cumulo+cel l_yield_cumulo+rab_integral+RQ; 0.502, 0.742, tmp+pH+PL+AN+ OD+Arg_conc._cumulo+emission_O2; 0.486, 0.742, tmp+pH+PL+OD +Arg_conc._cumulo+rab_integral+RQ_integral+emission_O2+emis sion_CO2; 0.494, 0.742, tmp+pH+PL+AN+OD+RS+Arg_conc._cumulo +emission_O2; 0.494, 0.742, tmp+pH+PL+OD+Arg_conc._cumulo+c ell_yield_cumulo+emission_CO2+rab; 0.501, 0.741, tmp+pH+PL+ OD+Sugar_consuming_rate_cumulo+Arg_conc._cumulo+rab; 0.477, 0.741, tmp+pH+PL+AN+OD+RS+Arg_conc._cumulo+emission_O2+emi ssion_CO2+rab; 0.485, 0.741, tmp+pH+PL+RS+Sugar_consuming_r ate_cumulo+Arg_conc._cumulo+rab_integral+emission_O2+RQ; 0. 493, 0.741, tmp+pH+PL+OD+Arg_conc._cumulo+rab_integral+RQ_i ntegral+emission_O2; 0.493, 0.741, tmp+pH+PL+OD+RS+Sugar_co nsuming_rate_cumulo+emission_CO2+rab; 0.493, 0.741, tmp+pH+ PL+OD+RS+Arg_conc._cumulo+cell_yield_cumulo+emission_O2; 0. 493, 0.741, tmp+pH+PL+OD+Arg_conc._cumulo+cell_yield_cumulo +RQ+rab; 0.477, 0.741, tmp+pH+PL+RS+Sugar_consuming_rate_cu mulo+Arg_conc._cumulo+cell_yield_cumulo+rab_integral+emissi on_O2+emission_CO2; 0.485, 0.741, tmp+pH+PL+OD+RS+Sugar_con suming_rate_cumulo+Arg_conc._cumulo+RQ+rab; 0.493, 0.741, t mp+pH+PL+AN+RS+Sugar_consuming_rate_cumulo+Arg_conc._cumulo +emission_CO2; 0.468, 0.741, tmp+pH+PL+AN+OD+RS+Arg_conc._c umulo+RQ_integral+emission_CO2+RQ+rab; 0.501, 0.741, tmp+pH +PL+OD+Arg_conc._cumulo+cell_yield_cumulo+rab; 0.493, 0.741, tmp+pH+PL+OD+Arg_conc._cumulo+cell_yield_cumulo+emission_O 2+emission_CO2; 0.485, 0.741, tmp+pH+PL+OD+Sugar_consuming_ rate_cumulo+rab_integral+emission_O2+emission_CO2+rab; 0.48 5, 0.741, tmp+pH+PL+RS+Sugar_consuming_rate_cumulo+Arg_conc. _cumulo+cell_yield_cumulo+rab_integral+emission_O2; 0.477, 0.741, tmp+pH+PL+OD+RS+Arg_conc._cumulo+cell_yield_cumulo+R Q_integral+emission_CO2+RQ; 0.516, 0.741, tmp+pH+PL+Sugar_c onsuming_rate_cumulo+Arg_conc._cumulo; 0.493, 0.741, tmp+pH +PL+RS+Sugar_consuming_rate_cumulo+Arg_conc._cumulo+emissio n_CO2+rab; 0.485, 0.741, tmp+pH+PL+AN+OD+Arg_conc._cumulo+e mission_CO2+RQ+rab; 0.485, 0.741, tmp+pH+PL+AN+OD+RS+Sugar_ consuming_rate_cumulo+rab_integral+emission_O2; 0.459, 0.74 1, tmp+pH+PL+AN+OD+RS+Sugar_consuming_rate_cumulo+Arg_conc. _cumulo+rab_integral+emission_O2+RQ+rab; 0.485, 0.741, tmp+ pH+PL+AN+OD+RS+Arg_conc._cumulo+RQ_integral+emission_CO2; 0. 501, 0.741, tmp+pH+PL+OD+RS+cell_yield_cumulo+RQ; 0.485, 0. 741, tmp+pH+PL+OD+Arg_conc._cumulo+cell_yield_cumulo+rab_in tegral+emission_CO2+RQ; 0.476, 0.741, tmp+pH+PL+OD+RS+cell_ yield_cumulo+rab_integral+emission_O2+RQ+rab; 0.500, 0.741, tmp+pH+PL+AN+OD+RS+Sugar_consuming_rate_cumulo; 0.485, 0.7 41, tmp+pH+PL+RS+Sugar_consuming_rate_cumulo+Arg_conc._cumu lo+cell_yield_cumulo+emission_CO2+RQ; 0.493, 0.741, tmp+pH+ PL+OD+Sugar_consuming_rate_cumulo+Arg_conc._cumulo+RQ+rab; 0.500, 0.741, tmp+pH+PL+OD+RS+cell_yield_cumulo+emission_CO 2; 0.508, 0.741, tmp+pH+PL+Arg_conc._cumulo+emission_CO2+R Q; 0.508, 0.741, tmp+pH+PL+rab_integral+emission_O2+RQ; 0.4 92, 0.741, tmp+pH+PL+OD+RS+Sugar_consuming_rate_cumulo+cell _yield_cumulo+emission_CO2; 0.500, 0.741, tmp+pH+PL+cell_yi eld_cumulo+rab_integral+emission_O2+emission_CO2; 0.484, 0. 741, tmp+pH+PL+OD+RS+Arg_conc._cumulo+cell_yield_cumulo+emi ssion_O2+RQ; 0.484, 0.741, tmp+pH+PL+AN+OD+Sugar_consuming_ rate_cumulo+rab_integral+emission_O2+rab; 0.492, 0.741, tmp +pH+PL+AN+Sugar_consuming_rate_cumulo+Arg_conc._cumulo+emis sion_CO2+RQ; 0.492, 0.741, tmp+pH+PL+OD+Sugar_consuming_rat e_cumulo+Arg_conc._cumulo+emission_CO2+rab; 0.484, 0.741, t mp+pH+PL+AN+OD+RS+Arg_conc._cumulo+cell_yield_cumulo+emissi on_O2; 0.476, 0.741, tmp+pH+PL+AN+OD+RS+rab_integral+emissi on_O2+RQ+rab; 0.484, 0.741, tmp+pH+PL+AN+OD+Sugar_consuming _rate_cumulo+rab_integral+emission_O2+RQ; 0.476, 0.741, tmp +pH+PL+AN+OD+RS+Sugar_consuming_rate_cumulo+Arg_conc._cumul o+rab_integral+RQ; 0.458, 0.741, tmp+pH+PL+AN+OD+RS+Sugar_c onsuming_rate_cumulo+Arg_conc._cumulo+cell_yield_cumulo+rab _integral+emission_O2+rab; 0.484, 0.741, tmp+pH+PL+AN+Arg_c onc._cumulo+cell_yield_cumulo+rab_integral+emission_O2+RQ; 0.476, 0.741, tmp+pH+PL+OD+Arg_conc._cumulo+cell_yield_cumu lo+emission_O2+emission_CO2+RQ+rab; 0.458, 0.741, tmp+pH+PL +AN+OD+Arg_conc._cumulo+rab_integral+RQ_integral+emission_O 2+emission_CO2+RQ+rab; 0.492, 0.741, tmp+pH+PL+Sugar_consum ing_rate_cumulo+cell_yield_cumulo+rab_integral+emission_O2+ emission_CO2; 0.467, 0.741, tmp+pH+PL+RS+Sugar_consuming_ra te_cumulo+Arg_conc._cumulo+cell_yield_cumulo+rab_integral+e mission_O2+emission_CO2+RQ; 0.492, 0.741, tmp+pH+PL+AN+OD+A rg_conc._cumulo+emission_O2+RQ; 0.467, 0.740, tmp+pH+PL+RS+ Sugar_consuming_rate_cumulo+Arg_conc._cumulo+rab_integral+e mission_O2+emission_CO2+RQ+rab; 0.475, 0.740, tmp+pH+PL+AN+ OD+Sugar_consuming_rate_cumulo+Arg_conc._cumulo+emission_O2 +emission_CO2+RQ; 0.492, 0.740, tmp+pH+PL+OD+Sugar_consumin g_rate_cumulo+Arg_conc._cumulo+emission_O2+emission_CO2; 0. 458, 0.740, tmp+pH+PL+AN+OD+RS+Sugar_consuming_rate_cumulo+ Arg_conc._cumulo+emission_O2+emission_CO2+RQ+rab; 0.475, 0. 740, tmp+pH+PL+OD+RS+cell_yield_cumulo+rab_integral+emissio n_O2+emission_CO2+rab; 0.499, 0.740, tmp+pH+PL+Sugar_consum ing_rate_cumulo+Arg_conc._cumulo+emission_CO2+rab; 0.475, 0. 740, tmp+pH+PL+OD+Arg_conc._cumulo+cell_yield_cumulo+rab_in tegral+RQ_integral+emission_O2+RQ; 0.475, 0.740, tmp+pH+PL+ AN+OD+Arg_conc._cumulo+cell_yield_cumulo+emission_CO2+RQ+ra b; 0.483, 0.740, tmp+pH+PL+RS+Arg_conc._cumulo+cell_yield_c umulo+rab_integral+emission_O2+emission_CO2; 0.491, 0.740, tmp+pH+PL+AN+RS+Arg_conc._cumulo+emission_CO2+RQ; 0.491, 0. 740, tmp+pH+PL+Sugar_consuming_rate_cumulo+Arg_conc._cumulo +emission_O2+emission_CO2+RQ; 0.491, 0.740, tmp+pH+PL+Sugar _consuming_rate_cumulo+Arg_conc._cumulo+cell_yield_cumulo+e mission_CO2+RQ; 0.499, 0.740, tmp+pH+PL+AN+OD+RS+cell_yield _cumulo; 0.491, 0.740, tmp+pH+PL+OD+Arg_conc._cumulo+rab_in tegral+emission_CO2+RQ; 0.483, 0.740, tmp+pH+PL+RS+Arg_conc. _cumulo+rab_integral+emission_O2+RQ+rab; 0.483, 0.740, tmp+ pH+PL+AN+OD+RS+cell_yield_cumulo+rab_integral+emission_O2; 0.507, 0.740, tmp+pH+PL+AN+Arg_conc._cumulo+emission_CO2; 0. 483, 0.740, tmp+pH+PL+OD+RS+Arg_conc._cumulo+cell_yield_cum ulo+rab_integral+rab; 0.507, 0.740, tmp+pH+PL+OD+RS+emissio n_O2; 0.483, 0.740, tmp+pH+PL+OD+cell_yield_cumulo+rab_inte gral+emission_O2+emission_CO2+rab; 0.499, 0.740, tmp+pH+PL+ OD+RS+Arg_conc._cumulo+emission_O2; 0.483, 0.740, tmp+pH+PL +RS+Sugar_consuming_rate_cumulo+Arg_conc._cumulo+rab_integr al+emission_O2+rab; 0.483, 0.740, tmp+pH+PL+Sugar_consuming _rate_cumulo+cell_yield_cumulo+rab_integral+emission_O2+RQ+ rab; 0.491, 0.740, tmp+pH+PL+RS+Sugar_consuming_rate_cumulo +cell_yield_cumulo+rab_integral+emission_O2; 0.491, 0.740, tmp+pH+PL+Sugar_consuming_rate_cumulo+Arg_conc._cumulo+emis sion_CO2+RQ+rab; 0.475, 0.740, tmp+pH+PL+AN+OD+RS+rab_integ ral+emission_O2+emission_CO2+RQ; 0.475, 0.740, tmp+pH+PL+OD +RS+Sugar_consuming_rate_cumulo+rab_integral+emission_02+em ission_CO2+rab; 0.474, 0.740, tmp+pH+PL+AN+RS+Arg_conc._cum ulo+rab_integral+emission_O2+emission_CO2+rab; 0.483, 0.740, tmp+pH+PL+RS+Sugar_consuming_rate_cumulo+Arg_conc._cumulo+ emission_CO2+RQ+rab; 0.499, 0.740, tmp+pH+PL+OD+Sugar_consu ming_rate_cumulo+Arg_conc._cumulo+emission_O2; 0.491, 0.740, tmp+pH+PL+RS+Sugar_consuming_rate_cumulo+Arg_conc._cumulo+ cell_yield_cumulo+emission_CO2; 0.491, 0.740, tmp+pH+PL+OD+ RS+Sugar_consuming_rate_cumulo+Arg_conc._cumulo+emission_O 2; 0.466, 0.740, tmp+pH+PL+OD+RS+Sugar_consuming_rate_cumul o+Arg_conc._cumulo+cell_yield_cumulo+rab_integral+emission_ O2+rab; 0.457, 0.740, tmp+pH+PL+OD+Arg_conc._cumulo+cell_yi eld_cumulo+rab_integral+RQ_integral+emission_O2+emission_CO 2+RQ+rab; 0.457, 0.740, tmp+pH+PL+AN+OD+Arg_conc._cumulo+ce ll_yield_cumulo+rab_integral+RQ_integral+emission_O2+emissi on_CO2+RQ; 0.483, 0.740, tmp+pH+PL+OD+RS+Arg_conc._cumulo+e mission_O2+emission_CO2+rab; 0.499, 0.740, tmp+pH+PL+Sugar_ consuming_rate_cumulo+Arg_conc._cumulo+emission_O2+emission _CO2; 0.474, 0.740, tmp+pH+PL+AN+OD+Arg_conc._cumulo+rab_in tegral+RQ_integral+emission_O2+emission_CO2; 0.513, 0.740, tmp+pH+PL+Arg_conc._cumulo+emission_CO2; 0.483, 0.740, tmp+ pH+PL+OD+Sugar_consuming_rate_cumulo+rab_integral+emission-O2+emission_CO2+RQ; 0.491, 0.740, tmp+pH+PL+AN+Sugar_consum ing rate cumulo+cell yield cumulo+rab integral+emission O2; 0.474, 0.740, tmp+pH+PL+AN+OD+Sugar_consuming_rate_cumulo+ Arg_conc._cumulo+emission_CO2+RQ+rab; 0.498, 0.740, tmp+pH+ PL+OD+RS+Sugar_consuming_rate_cumulo+rab_integral; 0.498, 0. 740, tmp+pH+PL+OD+Sugar_consuming_rate_cumulo+Arg_conc._cum ulo+rab_integral; 0.474, 0.740, tmp+pH+PL+OD+RS+Sugar_consu ming_rate_cumulo+Arg_conc._cumulo+emission_O2+emission_CO2+ rab; 0.498, 0.740, tmp+pH+PL+RS+Arg_conc._cumulo+emission_C O2+rab; 0.465, 0.740, tmp+pH+PL+OD+RS+Arg_conc._cumulo+rab_ integral+RQ_integral+emission_O2+emission_CO2+RQ; 0.474, 0. 740, tmp+pH+PL+OD+Arg_conc._cumulo+rab_integral+RQ_integral +emission_O2+RQ+rab; 0.474, 0.740, tmp+pH+PL+AN+OD+RS+Sugar _consuming_rate_cumulo+Arg_conc._cumulo+RQ+rab; 0.474, 0.74 0, tmp+pH+PL+AN+RS+Sugar_consuming_rate_cumulo+Arg_conc._cu mulo+rab_integral+emission_O2+emission_CO2; 0.491, 0.740, t mp+pH+PL+Arg_conc._cumulo+emission_O2+emission_CO2+RQ+rab; 0.482, 0.740, tmp+pH+PL+AN+OD+RS+Arg_conc._cumulo+rab_integ ral+RQ; 0.498, 0.740, tmp+pH+PL+rab_integral+emission_O2+em ission_CO2+rab; 0.498, 0.740, tmp+pH+PL+OD+Arg_conc._cumulo +RQ+rab; 0.474, 0.740, tmp+pH+PL+AN+OD+RS+rab_integral+emis sion_O2+emission_CO2+rab; 0.490, 0.740, tmp+pH+PL+OD+RS+Sug ar_consuming_rate_cumulo+emission_CO2+RQ; 0.490, 0.739, tmp +pH+PL+OD+Arg_conc._cumulo+cell_yield_cumulo+emission_O2+R Q; 0.490, 0.739, tmp+pH+PL+OD+RS+Arg_conc._cumulo+emission_ O2+RQ; 0.498, 0.739, tmp+pH+PL+RS+Arg_conc._cumulo+emission _O2+emission_CO2; 0.490, 0.739, tmp+pH+PL+OD+Sugar_consumin g_rate_cumulo+Arg_conc._cumulo+emission_O2+RQ; 0.490, 0.739, tmp+pH+PL+RS+Arg_conc._cumulo+emission_O2+emission_CO2+RQ; 0.465, 0.739, tmp+pH+PL+AN+Sugar_consuming_rate_cumulo+Arg _conc._cumulo+cell_yield_cumulo+rab_integral+emission_O2+em ission_CO2+rab; 0.482, 0.739, tmp+pH+PL+AN+OD+RS+Arg_conc._ cumulo+emission_O2+RQ; 0.482, 0.739, tmp+pH+PL+AN+RS+Sugar_ consuming_rate_cumulo+Arg_conc._cumulo+emission_CO2+RQ; 0.4 82, 0.739, tmp+pH+PL+AN+OD+RS+Sugar_consuming_rate_cumulo+A rg_conc._cumulo+emission_O2; 0.482, 0.739, tmp+pH+PL+AN+RS+ Arg_conc._cumulo+rab_integral+emission_O2+RQ; 0.473, 0.739, tmp+pH+PL+AN+OD+rab_integral+emission_O2+emission_CO2+RQ+r ab; 0.482, 0.739, tmp+pH+PL+OD+RS+Sugar_consuming_rate_cumu lo+Arg_conc._cumulo+rab_integral+rab; 0.482, 0.739, tmp+pH+ PL+AN+OD+RS+emission_CO2+RQ+rab; 0.482, 0.739, tmp+pH+PL+AN +OD+Arg_conc._cumulo+rab_integral+emission_CO2+RQ; 0.490, 0. 739, tmp+pH+PL+OD+Sugar_consuming_rate_cumulo+Arg_conc._cum ulo+rab_integral+RQ; 0.505, 0.739, tmp+pH+PL+OD+RS+rab_inte gral; 0.490, 0.739, tmp+pH+PL+OD+RS+cell_yield_cumulo+RQ+ra b; 0.473, 0.739, tmp+pH+PL+AN+OD+RS+Arg_conc._cumulo+cell_y ield_cumulo+rab_integral+rab; 0.490, 0.739, tmp+pH+PL+AN+RS +Arg_conc._cumulo+emission_O2+emission_CO2; 0.481, 0.739, t mp+pH+PL+OD+Arg_conc._cumulo+cell_yield_cumulo+rab_integral +RQ_integral+emission_O2; 0.490, 0.739, tmp+pH+PL+AN+OD+Sug ar_consuming_rate_cumulo+Arg_conc._cumulo+rab; 0.490, 0.739, tmp+pH+PL+AN+OD+RS+RQ+rab; 0.497, 0.739, tmp+pH+PL+OD+Arg_ conc._cumulo+cell_yield_cumulo+emission_O2; 0.497, 0.739, t mp+pH+PL+OD+RS+Arg_conc._cumulo+rab_integral; 0.489, 0.739, tmp+pH+PL+OD+RS+Sugar_consuming_rate_cumulo+RQ+rab; 0.497, 0.739, tmp+pH+PL+AN+Arg_conc._cumulo+emission_CO2+RQ; 0.49 7, 0.739, tmp+pH+PL+AN+OD+Arg_conc._cumulo+rab; 0.489, 0.73 9, tmp+pH+PL+AN+OD+Arg_conc._cumulo+RQ+rab; 0.464, 0.739, t mp+pH+PL+AN+OD+RS+Sugar_consuming_rate_cumulo+Arg_conc._cum ulo+emission_O2+emission_CO2+rab; 0.481, 0.739, tmp+pH+PL+A N+Arg_conc._cumulo+cell_yield_cumulo+rab_integral+emission_ O2+rab; 0.481, 0.739, tmp+pH+PL+AN+OD+cell_yield_cumulo+rab _integral+emission_O2+RQ; 0.497, 0.739, tmp+pH+PL+Arg_conc. _cumulo+emission_O2+emission_CO2+rab; 0.481, 0.739, tmp+pH+ PL+OD+RS+cell_yield_cumulo+emission_CO2+RQ+rab; 0.473, 0.73 9, tmp+pH+PL+OD+Arg_conc._cumulo+cell_yield_cumulo+rab_inte gral+RQ_integral+emission_O2+emission_CO2; 0.473, 0.739, tm p+pH+PL+RS+Arg_conc._cumulo+cell_yield_cumulo+rab_integral+ emission_O2+emission_CO2+RQ; 0.481, 0.739, tmp+pH+PL+OD+cel l_yield_cumulo+rab_integral+emission_O2+emission_CO2+RQ; 0. 473, 0.739, tmp+pH+PL+AN+OD+Arg_conc._cumulo+rab_integral+R Q_integral+emission_O2+RQ; 0.489, 0.739, tmp+pH+PL+OD+Sugar _consuming_rate_cumulo+Arg_conc._cumulo+rab_integral+emissi on_CO2; 0.472, 0.739, tmp+pH+PL+OD+RS+Arg_conc._cumulo+cell _yield_cumulo+rab_integral+RQ+rab; 0.481, 0.739, tmp+pH+PL+ OD+RS+Sugar_consuming_rate_cumulo+Arg_conc._cumulo+emission _O2+RQ; 0.481, 0.739, tmp+pH+PL+OD+RS+Arg_conc._cumulo+rab_ integral+emission_CO2+rab; 0.481, 0.739, tmp+pH+PL+AN+OD+Ar g_conc._cumulo+cell_yield_cumulo+emission_O2+emission_CO2; 0.472, 0.739, tmp+pH+PL+AN+OD+RS+Sugar_consuming_rate_cumul o+Arg_conc._cumulo+rab_integral+rab; 0.472, 0.739, tmp+pH+P L+AN+OD+RS+Arg_conc._cumulo+cell_yield_cumulo+emission_O2+R Q; 0.497, 0.739, tmp+pH+PL+Sugar_consuming_rate_cumulo+Arg_ conc._cumulo+cell_yield_cumulo+emission_CO2; 0.472, 0.739, tmp+pH+PL+AN+OD+Arg_conc._cumulo+emission_O2+emission_CO2+R Q+rab; 0.504, 0.739, tmp+pH+PL+Sugar_consuming_rate_cumulo+ Arg_conc._cumulo+cell_yield_cumulo; 0.489, 0.739, tmp+pH+PL +RS+Arg_conc._cumulo+cell_yield_cumulo+rab_integral+emissio n_O2; 0.489, 0.739, tmp+pH+PL+OD+RS+Arg_conc._cumulo+rab_in tegral+RQ; 0.489, 0.739, tmp+pH+PL+OD+RS+cell_yield_cumulo+ emission_CO2+RQ; 0.463, 0.739, tmp+pH+PL+AN+Sugar_consuming _rate_cumulo+Arg_conc._cumulo+cell_yield_cumulo+rab_integra l+emission_O2+RQ+rab; 0.489, 0.739, tmp+pH+PL+RS+Arg_conc._ cumulo+emission_CO2+RQ+rab; 0.489, 0.739, tmp+pH+PL+AN+OD+R S+Sugar_consuming_rate_cumulo+emission_CO2; 0.481, 0.739, t mp+pH+PL+AN+OD+Arg_conc._cumulo+RQ_integral+emission_CO2+R Q; 0.472, 0.739, tmp+pH+PL+AN+OD+Sugar_consuming_rate_cumul o+Arg_conc._cumulo+rab_integral+emission_CO2+RQ; 0.504, 0.7 38, tmp+pH+PL+Sugar_consuming_rate_cumulo+Arg_conc._cumulo+ RQ; 0.480, 0.738, tmp+pH+PL+OD+RS+Sugar_consuming_rate_cumu lo+emission_CO2+RQ+rab; 0.480, 0.738, tmp+pH+PL+AN+RS+Sugar _consuming_rate_cumulo+Arg_conc._cumulo+cell_yield_cumulo+e mission_CO2; 0.480, 0.738, tmp+pH+PL+AN+OD+Sugar_consuming_ rate_cumulo+Arg_conc._cumulo+emission_O2+emission_CO2; 0.47 2, 0.738, tmp+pH+PL+OD+RS+cell_yield_cumulo+rab_integral+em ission_O2+emission_CO2+RQ; 0.480, 0.738, tmp+pH+PL+AN+OD+Ar g_conc._cumulo+rab_integral+RQ_integral+emission_O2; 0.480, 0.738, tmp+pH+PL+OD+RS+Sugar_consuming_rate_cumulo+cell_yi eld_cumulo+emission_CO2+rab; 0.472, 0.738, tmp+pH+PL+OD+RS+ Sugar_consuming_rate_cumulo+rab_integral+emission_O2+emissi on_CO2+RQ; 0.496, 0.738, tmp+pH+PL+OD+RS+emission_O2+emissi on_CO2; 0.463, 0.738, tmp+pH+PL+AN+OD+Sugar_consuming_rate_ cumulo+Arg_conc._cumulo+cell_yield_cumulo+rab_integral+emis sion_O2+rab; 0.472, 0.738, tmp+pH+PL+OD+Sugar_consuming_rat e_cumulo+Arg_conc._cumulo+emission_O2+emission_CO2+RQ+rab; 0.480, 0.738, tmp+pH+PL+AN+OD+cell_yield_cumulo+rab_integra l+emission_O2+rab; 0.472, 0.738, tmp+pH+PL+AN+Arg_conc._cum ulo+cell_yield_cumulo+rab_integral+emission_O2+emission_CO2 +rab; 0.480, 0.738, tmp+pH+PL+AN+RS+Sugar_consuming_rate_cu mulo+Arg_conc._cumulo+rab_integral+emission_O2; 0.480, 0.73 8, tmp+pH+PL+AN+OD+RS+Arg_conc._cumulo+rab_integral+rab; 0. 503, 0.738, tmp+pH+PL+OD+Arg_conc._cumulo+rab; 0.488, 0.738, tmp+pH+PL+AN+OD+Arg_conc._cumulo+cell_yield_cumulo+rab; 0. 454, 0.738, tmp+pH+PL+AN+Sugar_consuming_rate_cumulo+Arg_co nc._cumulo+cell_yield_cumulo+rab_integral+emission_O2+emiss ion_CO2+RQ+rab; 0.480, 0.738, tmp+pH+PL+OD+RS+Sugar_consumi ng_rate_cumulo+cell_yield_cumulo+emission_CO2+RQ; 0.454, 0. 738, tmp+pH+PL+AN+OD+RS+Arg_conc._cumulo+cell_yield_cumulo+ RQ_integral+emission_CO2+RQ+rab; 0.488, 0.738, tmp+pH+PL+AN +Sugar_consuming_rate_cumulo+Arg_conc._cumulo+emission_O2+e mission_CO2; 0.480, 0.738, tmp+pH+PL+AN+RS+Arg_conc._cumulo +rab_integral+emission_O2+rab; 0.471, 0.738, tmp+pH+PL+RS+S ugar_consuming_rate_cumulo+Arg_conc._cumulo+cell_yield_cumu lo+rab_integral+emission_O2+RQ; 0.496, 0.738, tmp+pH+PL+AN+ cell_yield_cumulo+rab_integral+emission_O2; 0.471, 0.738, t mp+pH+PL+AN+OD+RS+Sugar_consuming_rate_cumulo+rab_integral+ emission_O2+rab; 0.462, 0.738, tmp+pH+PL+OD+RS+Arg_conc._cu mulo+cell_yield_cumulo+RQ_integral+emission_CO2+RQ+rab; 0.4 53, 0.738, tmp+pH+PL+AN+OD+RS+Arg_conc._cumulo+rab_integral +RQ_integral+emission_O2+emission_CO2+RQ; 0.471, 0.738, tmp +pH+PL+AN+OD+RS+Arg_conc._cumulo+cell_yield_cumulo+RQ_integ ral+emission_CO2; 0.510, 0.738, tmp+pH+PL+Arg_conc._cumulo+ emission_O2; 0.462, 0.738, tmp+pH+PL+AN+OD+RS+Arg_conc._cum ulo+rab_integral+RQ_integral+emission_CO2+RQ; 0.495, 0.738, tmp+pH+PL+AN+rab_integral+emission_O2+rab; 0.487, 0.738, t mp+pH+PL+OD+RS+Sugar_consuming_rate_cumulo+cell_yield_cumul o+rab; 0.487, 0.738, tmp+pH+PL+AN+OD+RS+Sugar_consuming_rat e_cumulo+rab; 0.487, 0.738, tmp+pH+PL+AN+OD+Sugar_consuming _rate_cumulo+Arg_conc._cumulo+emission_O2; 0.487, 0.738, tm p+pH+PL+OD+RS+Sugar_consuming_rate_cumulo+rab_integral+rab; 0.487, 0.738, tmp+pH+PL+AN+OD+RS+cell_yield_cumulo+emissio n_CO2; 0.471, 0.738, tmp+pH+PL+AN+RS+Sugar_consuming_rate_c umulo+Arg_conc._cumulo+cell_yield_cumulo+emission_CO2+RQ; 0. 471, 0.738, tmp+pH+PL+OD+RS+Sugar_consuming_rate_cumulo+Arg _conc._cumulo+rab_integral+RQ+rab; 0.470, 0.738, tmp+pH+PL+ OD+RS+Arg_conc._cumulo+RQ_integral+emission_CO2+RQ+rab; 0.4 79, 0.738, tmp+pH+PL+AN+OD+Sugar_consuming_rate_cumulo+Arg_ conc._cumulo+emission_CO2+rab; 0.495, 0.738, tmp+pH+PL+AN+O D+RS+rab_integral; 0.479, 0.737, tmp+pH+PL+AN+OD+Sugar_cons uming_rate_cumulo+Arg_conc._cumulo+RQ+rab; 0.479, 0.737, tm p+pH+PL+AN+OD+RS+Arg_conc._cumulo+emission_O2+rab; 0.495, 0. 737, tmp+pH+PL+AN+OD+RS+emission_O2; 0.487, 0.737, tmp+pH+P L+AN+OD+Sugar_consuming_rate_cumulo+Arg_conc._cumulo+rab_in tegral; 0.453, 0.737, tmp+pH+PL+OD+RS+Arg_conc._cumulo+cell _yield_cumulo+rab_integral+RQ_integral+emission_O2+emission CO2+RQ; 0.479, 0.737, tmp+pH+PL+Sugar_consuming_rate_cumul o+cell_yield_cumulo+rab_integral+emission_O2+emission_CO2+r ab; 0.479, 0.737, tmp+pH+PL+OD+Arg_conc._cumulo+rab_integra l+RQ_integral+emission_O2+rab; 0.461, 0.737, tmp+pH+PL+RS+S ugar_consuming_rate_cumulo+Arg_conc._cumulo+cell_yield_cumu lo+rab_integral+emission_O2+emission_CO2+rab; 0.461, 0.737, tmp+pH+PL+AN+RS+Sugar_consuming_rate_cumulo+Arg_conc._cumu lo+rab_integral+emission_O2+emission_CO2+RQ; 0.461, 0.737, tmp+pH+PL+OD+RS+Sugar_consuming_rate_cumulo+Arg_conc._cumul o+cell_yield_cumulo+rab_integral+emission_O2+RQ; 0.470, 0.7 37, tmp+pH+PL+AN+OD+Sugar_consuming_rate_cumulo+Arg_conc._c umulo+cell_yield_cumulo+rab_integral+emission_O2; 0.502, 0. 737, tmp+pH+PL+AN+Sugar_consuming_rate_cumulo+Arg_conc._cum ulo; 0.487, 0.737, tmp+pH+PL+AN+OD+RS+cell_yield_cumulo+ra b; 0.494, 0.737, tmp+pH+PL+OD+RS+emission_O2+RQ; 0.478, 0.7 37, tmp+pH+PL+AN+OD+Arg_conc._cumulo+cell_yield_cumulo+emis sion_CO2+rab; 0.486, 0.737, tmp+pH+PL+AN+OD+RS+Sugar_consum ing_rate_cumulo+RQ; 0.486, 0.737, tmp+pH+PL+OD+Arg_conc._cu mulo+emission_O2+RQ+rab; 0.470, 0.737, tmp+pH+PL+AN+OD+RS+S ugar_consuming_rate_cumulo+rab_integral+emission_O2+RQ 0.4 61, 0.737, tmp+pH+PL+AN+RS+Arg_conc._cumulo+rab_integral+em ission_O2+emission_CO2+RQ+rab; 0.494, 0.737, tmp+pH+PL+OD+R S+rab integral+emission CO2; 0.478, 0.737, tmp+pH+PL+AN+OD+ Arg_conc._cumulo+cell_yield_cumulo+RQ+rab; 0.486, 0.737, tm p+pH+PL+cell_yield_cumulo+rab_integral+emission_O2+RQ+rab; 0.478, 0.737, tmp+pH+PL+OD+RS+Arg_conc._cumulo+cell_yield_c umulo+emission_O2+rab; 0.470, 0.737, tmp+pH+PL+AN+OD+Arg_co nc._cumulo+cell_yield_cumulo+rab_integral+emission_CO2+RQ; 0.486, 0.737, tmp+pH+PL+AN+OD+Arg_conc._cumulo+emission_CO2 +rab; 0.486, 0.737, tmp+pH+PL+OD+Arg_conc._cumulo+emission_ O2+emission_CO2+rab; 0.452, 0.737, tmp+pH+PL+AN+OD+RS+Sugar _consuming_rate_cumulo+Arg_conc._cumulo+cell_yield_cumulo+r ab_integral+emission_O2+RQ; 0.470, 0.737, tmp+pH+PL+RS+Suga r_consuming_rate_cumulo+Arg_conc._cumulo+cell_yield_cumulo+ rab_integral+emission_O2+rab; 0.478, 0.737, tmp+pH+PL+AN+RS +Arg_conc. _cumulo+emission_O2+emission_CO2+RQ; 0.470, 0.737, tmp+pH+PL+OD+Arg_conc._cumulo+rab_integral+RQ_integral+emi ssion_O2+emission_CO2+rab; 0.469, 0.737, tmp+pH+PL+AN+OD+RS +Sugar_consuming_rate_cumulo+Arg_conc._cumulo+cell_yield_cu mulo+emission_CO2; 0.469, 0.737, tmp+pH+PL+RS+Sugar_consumi ng_rate_cumulo+Arg_conc._cumulo+cell_yield_cumulo+emission-CO2+RQ+rab; 0.478, 0.737, tmp+pH+PL+RS+Sugar_consuming_rate _cumulo+Arg_conc._cumulo+cell_yield_cumulo+emission_CO2+ra b; 0.442, 0.737, tmp+pH+PL+AN+OD+RS+Sugar_consuming_rate_cu mulo+Arg_conc._cumulo+cell_yield_cumulo+rab_integral+emissi on_O2+emission_CO2+rab; 0.461, 0.737, tmp+pH+PL+AN+OD+RS+Ar g_conc._cumulo+cell_yield_cumulo+rab_integral+RQ+rab; 0.460, 0.737, tmp+pH+PL+AN+OD+RS+Arg_conc._cumulo+RQ_integral+emi ssion_O2+emission_CO2+RQ; 0.478, 0.737, tmp+pH+PL+AN+Sugar_ consuming_rate_cumulo+Arg_conc._cumulo+emission_O2+emission CO2+RQ; 0.469, 0.737, tmp+pH+PL+AN+OD+RS+Sugar_consuming_r ate_cumulo+Arg_conc._cumulo+emission_O2+RQ; 0.469, 0.737, t mp+pH+PL+OD+Sugar_consuming_rate_cumulo+Arg_conc._cumulo+ce ll_yield_cumulo+rab_integral+emission_O2+rab; 0.477, 0.737, tmp+pH+PL+AN+OD+RS+Sugar_consuming_rate_cumulo+emission_CO 2+rab; 0.516, 0.737, tmp+pH+PL+Arg_conc._cumulo; 0.486, 0.7 37, tmp+pH+PL+cell_yield_cumulo+rab_integral+emission_O2+em ission_CO2+rab; 0.477, 0.737, tmp+pH+PL+AN+OD+RS+cell_yield _cumulo+emission_CO2+rab; 0.469, 0.737, tmp+pH+PL+AN+OD+Sug ar_consuming_rate_cumulo+rab_integral+emission_O2+RQ+rab; 0. 486, 0.737, tmp+pH+PL+OD+Arg_conc._cumulo+RQ_integral+emiss ion_CO2+RQ; 0.486, 0.737, tmp+pH+PL+OD+RS+emission_O2+emiss ion_CO2+RQ; 0.477, 0.737, tmp+pH+PL+RS+Sugar_consuming_rate _cumulo+cell_yield_cumulo+rab_integral+emission_O2+rab; 0.4 69, 0.737, tmp+pH+PL+AN+OD+RS+cell_yield_cumulo+rab_integra l+emission_O2+rab; 0.493, 0.737, tmp+pH+PL+RS+cell_yield_cu mulo+rab_integral+emission_O2; 0.486, 0.737, tmp+pH+PL+AN+O D+Sugar_consuming_rate_cumulo+Arg_conc._cumulo+cell_yield_c umulo; 0.509, 0.737, tmp+pH+PL+OD+emission_O2; 0.493, 0.737, tmp+pH+PL+OD+Arg_conc._cumulo+emission_CO2+rab; 0.477, 0.7 37, tmp+pH+PL+AN+OD+Sugar_consuming_rate_cumulo+Arg_conc._c umulo+rab_integral+emission_CO2; 0.469, 0.737, tmp+pH+PL+OD +Sugar_consuming_rate_cumulo+Arg_conc._cumulo+rab_integral+ emission_CO2+RQ+rab; 0.493, 0.737, tmp+pH+PL+OD+RS+emission _O2+rab; 0.501, 0.737, tmp+pH+PL+Sugar_consuming_rate_cumul o+Arg_conc._cumulo+rab; 0.485, 0.737, tmp+pH+PL+AN+OD+Arg_c onc._cumulo+cell_yield_cumulo+emission_O2; 0.493, 0.737, tm p+pH+PL+OD+Arg_conc._cumulo+emission_O2+rab; 0.493, 0.737, tmp+pH+PL+OD+RS+Sugar_consuming_rate_cumulo+cell_yield_cumu lo; 0.469, 0.737, tmp+pH+PL+RS+Sugar_consuming_rate_cumulo+ Arg_conc._cumulo+rab_integral+emission_O2+RQ+rab; 0.485, 0. 736, tmp+pH+PL+AN+OD+RS+emission_O2+emission_CO2; 0.477, 0. 736, tmp+pH+PL+OD+Arg_conc._cumulo+cell_yield_cumulo+emissi on_O2+emission_CO2+rab; 0.469, 0.736, tmp+pH+PL+OD+Sugar_co nsuming_rate_cumulo+rab_integral+emission_O2+emission-CO2+R Q+rab; 0.493, 0.736, tmp+pH+PL+Sugar_consuming_rate_cumulo+ Arg_conc._cumulo+cell_yield_cumulo+RQ; 0.485, 0.736, tmp+pH +PL+AN+OD+Arg_conc._cumulo+emission_O2+rab; 0.485, 0.736, t mp+pH+PL+AN+OD+RS+cell_yield_cumulo+RQ; 0.501, 0.736, tmp+p H+PL+RS+Sugar_consuming_rate_cumulo+Arg_conc._cumulo; 0.493, 0.736, tmp+pH+PL+OD+RS+rab_integral+RQ; 0.485, 0.736, tmp+ pH+PL+AN+OD+RS+rab_integral+emission_CO2; 0.485, 0.736, tmp +pH+PL+AN+RS+Arg_conc._cumulo+emission_CO2+rab; 0.460, 0.73 6, tmp+pH+PL+AN+Arg_conc._cumulo+cell_yield_cumulo+rab_inte gral+emission_O2+emission_CO2+RQ+rab; 0.493, 0.736, tmp+pH+ PL+OD+RS+Sugar_consuming_rate_cumulo+emission_O2; 0.468, 0. 736, tmp+pH+PL+AN+OD+RS+cell_yield_cumulo+rab_integral+emis sion_O2+RQ; 0.485, 0.736, tmp+pH+PL+RS+Sugar_consuming_rate _cumulo+Arg_conc._cumulo+emission_O2+emission_CO2; 0.485, 0. 736, tmp+pH+PL+rab_integral+emission_O2+emission_O2+RQ+ra b; 0.459, 0.736, tmp+pH+PL+AN+OD+Arg_conc._cumulo+rab_integ ral+RQ_integral+emission_O2+RQ+rab; 0.459, 0.736, tmp+pH+PL +AN+OD+RS+Sugar_consuming_rate_cumulo+Arg_conc._cumulo+rab_ integral+RQ+rab; 0.500, 0.736, tmp+pH+PL+rab_integral+emiss ion_O2+emission_CO2; 0.468, 0.736, tmp+pH+PL+AN+OD+RS+Arg_c onc._cumulo+rab_integral+RQ+rab; 0.493, 0.736, tmp+pH+PL+Su gar_consuming_rate_cumulo+rab_integral+emission_O2+rab; 0.4 92, 0.736, tmp+pH+PL+RS+Sugar_consuming_rate_cumulo+Arg_con c._cumulo+cell_yield_cumulo; 0.459, 0.736, tmp+pH+PL+AN+OD+ RS+rab_integral+emission_O2+emission_CO2+RQ+rab; 0.484, 0.7 36, tmp+pH+PL+OD+RS+Sugar_consuming_rate_cumulo+rab_integra 1+RQ; 0.476, 0.736, tmp+pH+PL+AN+RS+Sugar_consuming_rate_cu mulo+Arg_conc._cumulo+emission_CO2+rab; 0.459, 0.736, tmp+p H+PL+OD+Arg_conc._cumulo+cell_yield_cumulo+rab_integral+RQ_ integral+emission_O2+RQ+rab; 0.492, 0.736, tmp+pH+PL+Arg_co nc._cumulo+emission_CO2+RQ+rab; 0.450, 0.736, tmp+pH+PL+RS+ Sugar_consuming_rate_cumulo+Arg_conc._cumulo+cell_yield_cum ulo+rab_integral+emission_O2+emission_CO2+RQ+rab; 0.476, 0. 736, tmp+pH+PL+AN+Sugar_consuming_rate_cumulo+cell_yield_cu mulo+rab_integral+emission_O2+RQ; 0.476, 0.736, tmp+pH+PL+A N+OD+Sugar_consuming_rate_cumulo+Arg_conc._cumulo+emission_ O2+RQ; 0.492, 0.736, tmp+pH+PL+OD+RS+rab_integral+rab; 0.47 6, 0.736, tmp+pH+PL+AN+OD+Arg_conc._cumulo+emission_O2+RQ+r ab; 0.459, 0.736, tmp+pH+PL+OD+RS+cell_yield_cumulo+rab_int egral+emission_O2+emission_CO2+RQ+rab; 0.459, 0.736, tmp+pH +PL+OD+RS+Sugar_consuming_rate_cumulo+rab_integral+emission _O2+emission_CO2+RQ+rab; 0.484, 0.736, tmp+pH+PL+Sugar_cons uming_rate_cumulo+Arg_conc._cumulo+emission_O2+emission_CO2 +rab; 0.484, 0.736, tmp+pH+PL+OD+RS+emission_O2+emission_CO 2+rab; 0.484, 0.736, tmp+pH+PL+AN+Sugar_consuming_rate_cumu lo+Arg_conc._cumulo+cell_yield_cumulo+emission_CO2; 0.450, 0.736, tmp+pH+PL+AN+OD+RS+Arg_conc._cumulo+cell_yield_cumul o+rab_integral+RQ_integral+emission_CO2+RQ; 0.484, 0.736, t mp+pH+PL+AN+OD+Arg_conc._cumulo+rab_integral+emission_CO2; 0.476, 0.736, tmp+pH+PL+RS+Sugar_consuming_rate_cumulo+Arg_ conc. _cumulo+emission_O2+emission_CO2+RQ; 0.476, 0.736, tmp +pH+PL+Sugar_consuming_rate_cumulo+Arg_conc._cumulo+emissio n_O2+emission_CO2+RQ+rab; 0.476, 0.736, tmp+pH+PL+Sugar_con suming_rate_cumulo+cell_yield_cumulo+rab_integral+emission_ O2+emission_CO2+RQ; 0.467, 0.736, tmp+pH+PL+AN+OD+RS+Arg_co nc._cumulo+cell_yield_cumulo+emission_O2+rab; 0.467, 0.736, tmp+pH+PL+RS+Arg_conc._cumulo+cell_yield_cumulo+rab_integr al+emission_O2+emission_CO2+rab; 0.467, 0.736, tmp+pH+PL+OD +Sugar_consuming_rate_cumulo+Arg_conc._cumulo+cell_yield_cu mulo+rab_integral+emission_O2+emission_CO2; 0.476, 0.736, t mp+pH+PL+AN+Sugar_consuming_rate_cumulo+Arg_conc._cumulo+ce ll_yield_cumulo+emission_CO2+RQ; 0.492, 0.736, tmp+pH+PL+Su gar_consuming_rate_cumulo+Arg_conc._cumulo+rab_integral+emi ssion_CO2; 0.476, 0.736, tmp+pH+PL+Arg_conc._cumulo+rab_int egral+RQ_integral+emission_O2+emission_CO2+RQ; 0.476, 0.736, tmp+pH+PL+AN+OD+Sugar_consuming_rate_cumulo+Arg_conc._cumu lo+rab_integral+RQ; 0.484, 0.736, tmp+pH+PL+Sugar_consuming _rate_cumulo+Arg_conc._cumulo+rab_integral+emission_CO2+RQ; 0.467, 0.736, tmp+pH+PL+OD+RS+Arg_conc._cumulo+cell_yield_ cumulo+emission_O2+RQ+rab; 0.484, 0.736, tmp+pH+PL+OD+RS+ra b_integral+emission_CO2+RQ; 0.484, 0.736, tmp+pH+PL+OD+Arg_ conc._cumulo+cell_yield_cumulo+rab_integral+RQ; 0.484, 0.73 6, tmp+pH+PL+OD+Arg_conc._cumulo+cell_yield_cumulo+rab_inte gral+emission_CO2; 0.484, 0.736, tmp+pH+PL+AN+Arg_conc._cum ulo+emission_O2+emission_CO2+rab; 0.476, 0.736, tmp+pH+PL+A N+OD+Arg_conc._cumulo+cell_yield_cumulo+emission_O2+RQ; 0.4 84, 0.736, tmp+pH+PL+RS+Arg_conc._cumulo+emission_O2+emissi on_CO2+rab; 0.484, 0.736, tmp+pH+PL+OD+RS+Sugar_consuming_r ate_cumulo+rab_integral+emission_CO2; 0.467, 0.735, tmp+pH+ PL+AN+OD+RS+Arg_conc._cumulo+RQ_integral+emission_CO2+rab; 0.491, 0.735, tmp+pH+PL+OD+Arg_conc._cumulo+rab_integral+R Q; 0.467, 0.735, tmp+pH+PL+OD+Arg_conc._cumulo+cell_yield_c umulo+rab_integral+emission_CO2+RQ+rab; 0.475, 0.735, tmp+p H+PL+AN+OD+RS+Sugar_consuming_rate_cumulo+emission_CO2+RQ; 0.475, 0.735, tmp+pH+PL+AN+Sugar_consuming_rate_cumulo+cell _yield_cumulo+rab_integral+emission_O2+rab; 0.491, 0.735, t mp+pH+PL+AN+Sugar_consuming_rate_cumulo+Arg_conc._cumulo+ce ll_yield_cumulo; 0.475, 0.735, tmp+pH+PL+RS+Sugar_consuming _rate_cumulo+cell_yield_cumulo+rab_integral+emission_O2+RQ; 0.499, 0.735, tmp+pH+PL+Arg_conc._cumulo+emission_O2+RQ; 0. 491, 0.735, tmp+pH+PL+AN+OD+Arg_conc._cumulo+rab_integral; 0.483, 0.735, tmp+pH+PL+RS+Sugar_consuming_rate_cumulo+Arg_ conc._cumulo+rab_integral+emission_CO2; 0.458, 0.735, tmp+p H+PL+AN+OD+RS+Sugar_consuming_rate_cumulo+Arg_conc._cumulo+ cell_yield_cumulo+emission_CO2+rab; 0.475, 0.735, tmp+pH+PL +Sugar_consuming_rate_cumulo+Arg_conc._cumulo+cell_yield_cu mulo+emission_O2+emission_CO2+RQ; 0.475, 0.735, tmp+pH+PL+A NOD+Sugar_consuming_rate_cumulo+rab_integral+emission_O2+e mission_CO2; 0.467, 0.735, tmp+pH+PL+AN+OD+RS+Arg_conc._cum ulo+emission_O2+RQ+rab; 0.483, 0.735, tmp+pH+PL+cell_yield_ cumulo+rab_integral+emission_O2+emission_CO2+RQ; 0.466, 0.7 35, tmp+pH+PL+AN+OD+RS+Sugar_consuming_rate_cumulo+cell_yie ld_cumulo+emission_CO2+rab; 0.483, 0.735, tmp+pH+PL+OD+RS+A rg_conc._cumulo+emission_O2+rab; 0.499, 0.735, tmp+pH+PL+Ar g_conc._cumulo+emission_CO2+rab; 0.458, 0.735, tmp+pH+PL+AN +OD+Arg_conc._cumulo+cell_yield_cumulo+rab_integral+RQ_inte gral+emission_O2+emission_CO2; 0.506, 0.735, tmp+pH+PL+OD+S ugar_consuming_rate_cumulo; 0.466, 0.735, tmp+pH+PL+OD+cell _yield_cumulo+rab_integral+emission_O2+emission_CO2+RQ+rab; 0.483, 0.735, tmp+pH+PL+AN+rab_integral+emission_O2+RQ+ra b; 0.499, 0.735, tmp+pH+PL+AN+Arg_conc._cumulo+emission_O2; 0.475, 0.735, tmp+pH+PL+AN+OD+Arg_conc._cumulo+emission_O2 +emission_CO2+rab; 0.475, 0.735, tmp+pH+PL+OD+RS+Sugar_cons uming_rate_cumulo+cell_yield_cumulo+rab_integral+emission_O 2; 0.498, 0.735, tmp+pH+PL+RS+Sugar_consuming_rate_cumulo+c ell_yield_cumulo; 0.466, 0.735, tmp+pH+PL+AN+OD+Arg_conc._c umulo+rab_integral+RQ_integral+emission_O2+rab; 0.457, 0.73 5, tmp+pH+PL+AN+OD+Arg_conc._cumulo+cell_yield_cumulo+rab_i ntegral+RQ_integral+emission_O2+RQ; 0.475, 0.735, tmp+pH+PL +OD+RS+Sugar_consuming_rate_cumulo+Arg_conc._cumulo+emissio n_O2+rab; 0.466, 0.735, tmp+pH+PL+AN+Arg_conc._cumulo+cell_ yield_cumulo+rab_integral+emission_O2+RQ+rab; 0.483, 0.735, tmp+pH+PL+AN+Sugar_consuming_rate_cumulo+Arg_conc._cumulo+ emission_CO2+rab; 0.474, 0.735, tmp+pH+PL+RS+Arg_conc._cumu lo+cell_yield_cumulo+rab_integral+emission_O2+RQ; 0.474, 0. 735, tmp+pH+PL+OD+RS+Sugar_consuming_rate_cumulo+rab_integr al+emission_CO2+rab; 0.474, 0.735, tmp+pH+PL+OD+Sugar_consu ming_rate_cumulo+Arg_conc._cumulo+emission_O2+emission_CO2+ rab; 0.474, 0.735, tmp+pH+PL+Sugar_consuming_rate_cumulo+Ar g_conc._cumulo+cell_yield_cumulo+emission_CO2+RQ+rab; 0.466, 0.735, tmp+pH+PL+OD+Arg_conc._cumulo+cell_yield_cumulo+rab _integral+RQ_integral+emission_O2+rab; 0.483, 0.735, tmp+pH +PL+OD+RS+Sugar_consuming_rate_cumulo+cell_yield_cumulo+RQ; 0.457, 0.735, tmp+pH+PL+AN+OD+Arg_conc._cumulo+rab_integra 1+RQ_integral+emission_O2+emission_CO2+rab; 0.474, 0.735, t mp+pH+PL+AN+OD+RS+Sugar_consuming_rate_cumulo+cell_yield_cu mulo+emission_CO2; 0.466, 0.735, tmp+pH+PL+AN+RS+Sugar_cons uming_rate_cumulo+Arg_conc._cumulo+cell_yield_cumulo+rab_in tegral+emission_O2; 0.483, 0.735, tmp+pH+PL+Arg_conc._cumul o+rab_integral+RQ_integral+emission_O2+emission_CO2; 0.498, 0.735, tmp+pH+PL+Sugar_consuming_rate_cumulo+Arg_conc._cum ulo+emission_O2; 0.482, 0.735, tmp+pH+PL+AN+OD+RS+Sugar_con suming_rate_cumulo+rab_integral; 0.498, 0.735, tmp+pH+PL+AN +rab_integral+emission_O2; 0.482, 0.735, tmp+pH+PL+AN+OD+Ar g_conc._cumulo+rab_integral+RQ; 0.505, 0.735, tmp+pH+PL+Arg _conc._cumulo+RQ; 0.490, 0.735, tmp+pH+PL+OD+Arg_conc._cumu lo+cell_yield_cumulo+rab_integral; 0.482, 0.735, tmp+pH+PL+ AN+RS+Arg_conc._cumulo+rab_integral+emission_CO2; 0.474, 0. 735, tmp+pH+PL+RS+Arg_conc._cumulo+emission_O2+emission_CO2 +RQ+rab; 0.465, 0.735, tmp+pH+PL+AN+OD+Arg_conc._cumulo+cel l_yield_cumulo+rab_integral+RQ_integral+emission_O2; 0.505, 0.735, tmp+pH+PL+RS+Arg_conc._cumulo; 0.482, 0.735, tmp+pH +PL+OD+RS+rab_integral+emission_CO2+rab; 0.457, 0.735, tmp+ pH+PL+OD+Arg_conc._cumulo+cell_yield_cumulo+rab_integral+RQ _integral+emission_O2+emission_CO2+rab; 0.448, 0.735, tmp+p H+PL+OD+RS+Arg_conc._cumulo+rab_integral+RQ_integral+emissi on_O2+emission_CO2+RQ+rab; 0.457, 0.735, tmp+pH+PL+AN+OD+RS +Sugar_consuming_rate_cumulo+Arg_conc._cumulo+RQ_integral+e mission_CO2+RQ; 0.438, 0.735, tmp+pH+PL+AN+OD+Arg_conc._cum ulo+cell_yield_cumulo+rab_integral+RQ_integral+emission_O2+ emission_CO2+RQ+rab; 0.505, 0.735, tmp+pH+PL+AN+Arg_conc._c umulo; 0.465, 0.735, tmp+pH+PL+AN+RS+Sugar_consuming_rate_c umulo+Arg_conc._cumulo+rab_integral+emission_O2+RQ; 0.482, 0.735, tmp+pH+PL+OD+RS+Arg_conc._cumulo+rab_integral+rab; 0. 474, 0.735, tmp+pH+PL+AN+Arg_conc._cumulo+emission_O2+emiss ion_CO2+RQ+rab; 0.474, 0.735, tmp+pH+PL+RS+Sugar_consuming_ rate_cumulo+Arg_conc._cumulo+rab_integral+emission_CO2+RQ; 0.482, 0.735, tmp+pH+OD+RS+Sugar_consuming_rate_cumulo+Arg_ conc._cumulo+emission_CO2+RQ; 0.456, 0.735, tmp+pH+PL+AN+RS +Sugar_consuming_rate_cumulo+Arg_conc._cumulo+cell_yield_cu mulo+rab_integral+emission_O2+emission_CO2; 0.474, 0.735, t mp+pH+PL+OD+Arg_conc._cumulo+cell_yield_cumulo+RQ_integral+ emission_CO2+RQ; 0.474, 0.735, tmp+pH+PL+AN+OD+RS+cell_yiel d_cumulo+emission_CO2+RQ; 0.474, 0.735, tmp+pH+PL+AN+RS+Sug ar_consuming_rate_cumulo+Arg_conc._cumulo+emission_O2+emiss ion_CO2; 0.456, 0.735, tmp+pH+PL+OD+Sugar_consuming_rate_cu mulo+Arg_conc._cumulo+rab_integral+RQ_integral+emission_O2+ emission_CO2+RQ; 0.482, 0.735, tmp+pH+PL+Sugar_consuming_ra te_cumulo+rab_integral+emission_O2+RQ+rab; 0.438, 0.734, tm p+pH+PL+AN+OD+RS+Sugar_consuming_rate_cumulo+Arg_conc._cumu lo+cell_yield_cumulo+rab_integral+emission_O2+RQ+rab; 0.490, 0.734, tmp+pH+PL+OD+Arg_conc._cumulo+rab_integral+emission _CO2; 0.456, 0.734, tmp+pH+PL+AN+RS+Sugar_consuming_rate_cu mulo+Arg_conc._cumulo+rab_integral+emission_O2+emission_CO2 +rab; 0.512, 0.734, tmp+pH+PL+OD; 0.482, 0.734, tmp+pH+PL+O D+Sugar_consuming_rate_cumulo+Arg_conc._cumulo+rab_integral +rab; 0.497, 0.734, tmp+pH+PL+OD+Arg_conc._cumulo+rab_integ ral; 0.474, 0.734, tmp+pH+PL+OD+RS+Arg_conc._cumulo+emissio n_O2+RQ+rab; 0.490, 0.734, tmp+pH+PL+Arg_conc._cumulo+rab_i ntegral+emission_CO2+RQ; 0.465, 0.734, tmp+pH+PL+AN+OD+RS+S ugar_consuming_rate_cumulo+Arg_conc._cumulo+emission_O2+ra b; 0.465, 0.734, tmp+pH+PL+AN+OD+Arg_conc._cumulo+RQ_integr al+emission_O2+emission_CO2+RQ; 0.438, 0.734, tmp+pH+PL+AN+ OD+RS+Arg_conc._cumulo+cell_yield_cumulo+rab_integral+RQ_in tegral+emission_O2+emission_CO2+RQ; 0.497, 0.734, tmp+pH+PL +OD+emission O2+RQ; 0.473, 0.734, tmp+pH+PL+OD+Arg_conc._cu mulo+rab_integral+emission_CO2+RQ+rab; 0.473, 0.734, tmp+pH +PL+OD+RS+Sugar_consuming_rate_cumulo+cell_yield_cumulo+RQ+ rab; 0.465, 0.734, tmp+pH+PL+AN+OD+cell_yield_cumulo+rab_in tegral+emission_O2+RQ+rab; 0.481, 0.734, tmp+pH+PL+OD+Sugar _consuming_rate_cumulo+Arg_conc._cumulo+emission_O2+rab; 0. 473, 0.734, tmp+pH+PL+OD+Arg_conc._cumulo+RQ_integral+emiss ion_O2+emission_CO2+RQ; 0.456, 0.734, tmp+pH+PL+AN+OD+Arg_c onc._cumulo+cell_yield_cumulo+emission_O2+emission_CO2+RQ+r ab; 0.489, 0.734, tmp+pH+PL+AN+Sugar_consuming_rate_cumulo+ Arg_conc._cumulo+RQ; 0.473, 0.734, tmp+pH+PL+RS+Sugar_consu ming_rate_cumulo+cell_yield_cumulo+rab_integral+emission_O2 +emission_CO2; 0.473, 0.734, tmp+pH+PL+AN+OD+cell_yield_cum ulo+rab_integral+emission_O2+emission_CO2; 0.489, 0.734, tm p+pH+OD+RS+Arg_conc._cumulo+emission_CO2+RQ; 0.464, 0.734, tmp+pH+PL+AN+OD+RS+Sugar_consuming_rate_cumulo+rab_integral +emission_O2+emission_CO2; 0.481, 0.734, tmp+pH+PL+Sugar_co nsuming_rate_cumulo+Arg_conc._cumulo+cell_yield_cumulo+emis sion_CO2+rab; 0.489, 0.734, tmp+pH+PL+AN+Arg_conc._cumulo+r ab_integral+emission_CO2; 0.473, 0.734, tmp+pH+PL+OD+Sugar_ consuming_rate_cumulo+Arg_conc._cumulo+rab_integral+RQ+rab; 0.481, 0.734, tmp+pH+PL+OD+RS+Sugar_consuming_rate_cumulo+ emission_O2+rab; 0.473, 0.734, tmp+pH+PL+RS+Sugar_consuming _rate_cumulo+Arg_conc._cumulo+emission_O2+emission_CO2+rab; 0.473, 0.734, tmp+pH+PL+OD+RS+Arg_conc._cumulo+rab_integra 1+RQ+rab; 0.489, 0.734, tmp+pH+PL+RS+Arg_conc._cumulo+rab_i ntegral+emission_CO2; 0.464, 0.734, tmp+pH+PL+AN+OD+RS+cell _yield_cumulo+rab_integral+emission_O2+emission_CO2; 0.481, 0.734, tmp+pH+PL+AN+RS+Sugar_consuming_rate_cumulo+Arg_con c._cumulo+cell_yield_cumulo; 0.464, 0.734, tmp+pH+PL+AN+RS+ Arg_conc._cumulo+rab_integral+emission_O2+RQ+rab; 0.446, 0. 734, tmp+pH+PL+AN+OD+RS+Arg_conc._cumulo+cell_yield_cumulo+ RQ_integral+emission_O2+emission_CO2+RQ; 0.481, 0.734, tmp+ pH+PL+AN+OD+RS+emission_O2+RQ; 0.473, 0.734, tmp+pH+PL+RS+A rg_conc._cumulo+cell_yield_cumulo+rab_integral+emission_O2+ rab; 0.481, 0.734, tmp+pH+PL+AN+OD+RS+rab_integral+RQ; 0.48 1, 0.734, tmp+pH+PL+Sugar_consuming_rate_cumulo+Arg_conc._c umulo+cell_yield_cumulo+emission_O2+emission_CO2; 0.472, 0. 734, tmp+pH+PL+AN+OD+RS+emission_O2+emission_CO2+RQ; 0.472, 0.734, tmp+pH+PL+AN+OD+Arg_conc._cumulo+cell_yield_cumulo+ rab_integral+emission_CO2; 0.464, 0.734, tmp+pH+PL+AN+OD+Ar g_conc._cumulo+cell_yield_cumulo+RQ_integral+emission_CO2+R Q; 0.455, 0.734, tmp+pH+PL+AN+OD+RS+Sugar_consuming_rate_cu mulo+rab_integral+emission_O2+RQ+rab; 0.481, 0.734, tmp+pH+ PL+RS+Arg_conc._cumulo+rab_integral+emission_CO2+RQ; 0.481, 0.734, tmp+pH+PL+AN+cell_yield_cumulo+rab_integral+emissio n_O2+RQ; 0.455, 0.734, tmp+pH+PL+OD+RS+Arg_conc._cumulo+cel l_yield_cumulo+rab_integral+RQ_integral+emission_CO2+RQ; 0. 489, 0.734, tmp+pH+PL+Sugar_consuming_rate_cumulo+Arg_conc. _cumulo+RQ+rab; 0.472, 0.734, tmp+pH+PL+OD+RS+emission_O2+e mission_CO2+RQ+rab; 0.472, 0.734, tmp+pH+PL+AN+RS+Arg_conc. _cumulo+emission_CO2+RQ+rab; 0.489, 0.734, tmp+pH+PL+RS+rab _integral+emission_O2+rab; 0.481, 0.734, tmp+pH+PL+OD+Arg_c onc._cumulo+cell_yield_cumulo+emission_O2+rab; 0.464, 0.734, tmp+pH+PL+OD+RS+Arg_conc._cumulo+RQ_integral+emission_O2+e mission_CO2+RQ; 0.488, 0.734, tmp+pH+PL+rab_integral+emissi on_O2+emission_CO2+RQ; 0.464, 0.734, tmp+pH+PL+AN+OD+RS+Sug ar_consuming_rate_cumulo+cell_yield_cumulo+emission_CO2+RQ; 0.480, 0.734, tmp+pH+PL+AN+Sugar_consuming_rate_cumulo+Arg _conc._cumulo+rab_integral+emission_CO2; 0.480, 0.734, tmp+ pH+PL+AN+OD+RS+rab_integral+rab; 0.464, 0.734, tmp+pH+PL+AN +RS+Sugar_consuming_rate_cumulo+Arg_conc._cumulo+emission_C O2+RQ+rab; 0.455, 0.734, tmp+pH+PL+RS+Arg_conc._cumulo+cell _yield_cumulo+rab_integral+emission_O2+emission_CO2+RQ+rab; 0.472, 0.734, tmp+pH+PL+OD+Arg_conc._cumulo+cell_yield_cum ulo+emission_O2+RQ+rab; 0.472, 0.734, tmp+pH+PL+AN+OD+RS+Su gar_consuming_rate_cumulo+RQ+rab; 0.472, 0.734, tmp+pH+PL+A N+OD+RS+rab_integral+emission_CO2+RQ; 0.472, 0.734, tmp+pH+ PL+AN+OD+Sugar_consuming_rate_cumulo+Arg_conc._cumulo+cell_ yield_cumulo+RQ; 0.472, 0.733, tmp+pH+PL+OD+Sugar_consuming _rate_cumulo+Arg_conc._cumulo+emission_O2+RQ+rab; 0.488, 0. 733, tmp+pH+PL+Sugar_consuming_rate_cumulo+Arg_conc._cumulo +cell_yield_cumulo+rab; 0.472, 0.733, tmp+pH+PL+AN+RS+Sugar _consuming_rate_cumulo+Arg_conc._cumulo+rab_integral+emissi on_CO2; 0.480, 0.733, tmp+pH+PL+AN+OD+RS+emission_O2+rab; 0. 455, 0.733, tmp+pH+PL+AN+OD+RS+Arg_conc._cumulo+cell_yield_ cumulo+RQ_integral+emission_CO2+rab; 0.472, 0.733, tmp+pH+P L+OD+RS+Sugar_consuming_rate_cumulo+rab_integral+RQ+rab; 0. 463, 0.733, tmp+pH+PL+OD+RS+Sugar_consuming_rate_cumulo+Arg _conc._cumulo+emission_O2+RQ+rab; 0.463, 0.733, tmp+pH+PL+A N+OD+RS+Sugar_consuming_rate_cumulo+emission_CO2+RQ+rab; 0. 454, 0.733, tmp+pH+PL+RS+Sugar_consuming_rate_cumulo+Arg_co nc._cumulo+cell_yield_cumulo+rab_integral+emission_O2+RQ+ra b; 0.488, 0.733, tmp+pH+PL+RS+Sugar_consuming_rate_cumulo+A rg_conc._cumulo+RQ; 0.472, 0.733, tmp+pH+PL+OD+Sugar_consum ing_rate_cumulo+Arg_conc._cumulo+rab_integral+emission_CO2+ rab; 0.480, 0.733, tmp+pH+PL+OD+RS+emission_O2+RQ+rab; 0.47 2, 0.733, tmp+pH+PL+AN+Sugar_consuming_rate_cumulo+Arg_conc. _cumulo+emission_CO2+RQ+rab; 0.463, 0.733, tmp+pH+PL+AN+RS+ Sugar_consuming_rate_cumulo+Arg_conc._cumulo+rab_integral+e mission_O2+rab; 0.454, 0.733, tmp+pH+PL+AN+OD+RS+Arg_conc._ cumulo+cell_yield_cumulo+emission_O2+RQ+rab; 0.463, 0.733, tmp+pH+PL+OD+RS+Arg_conc._cumulo+rab_integral+RQ_integral+e mission_CO2+RQ; 0.480, 0.733, tmp+pH+PL+RS+Sugar_consuming_ rate_cumulo+Arg_conc._cumulo+cell_yield_cumulo+RQ; 0.480, 0. 733, tmp+pH+PL+OD+RS+Sugar_consuming_rate_cumulo+emission_O 2+emission_CO2; 0.472, 0.733, tmp+pH+PL+RS+Sugar_consuming_ rate_cumulo+Arg_conc._cumulo+rab_integral+emission_CO2+rab; 0.436, 0.733, tmp+pH+PL+AN+OD+RS+Sugar_consuming_rate_cumu lo+Arg_conc._cumulo+cell_yield_cumulo+rab_integral+emission _O2+emission_CO2+RQ; 0.445, 0.733, tmp+pH+PL+OD+RS+Sugar_co nsuming_rate_cumulo+Arg_conc._cumulo+cell_yield_cumulo+rab_ integral+emission_O2+emission_CO2+rab; 0.454, 0.733, tmp+pH +PL+AN+OD+RS+Arg_conc._cumulo+rab_integral+RQ_integral+emis sion_O2+emission_CO2; 0.436, 0.733, tmp+pH+PL+AN+OD+RS+Arg_ conc._cumulo+rab_integral+RQ_integral+emission_O2+emission_ CO2+RQ+rab; 0.471, 0.733, tmp+pH+PL+AN+OD+RS+emission_O2+em ission_CO2+rab; 0.445, 0.733, tmp+pH+PL+AN+RS+Sugar_consumi ng_rate_cumulo+Arg_conc._cumulo+cell_yield_cumulo+rab_integ ral+emission_O2+emission_CO2+RQ; 0.480, 0.733, tmp+pH+PL+AN +Arg_conc._cumulo+rab_integral+emission_CO2+RQ; 0.463, 0.73 3, tmp+pH+PL+AN+OD+RS+cell_yield_cumulo+emission_CO2+RQ+ra b; 0.463, 0.733, tmp+pH+PL+AN+RS+Sugar_consuming_rate_cumul o+Arg_conc._cumulo+cell_yield_cumulo+emission_CO2+rab; 0.46 3, 0.733, tmp+pH+PL+RS+Sugar_consuming_rate_cumulo+Arg_conc. _cumulo+cell_yield_cumulo+rab_integral+emission_CO2+RQ; 0.4 95, 0.733, tmp+pH+PL+Arg_conc._cumulo+rab_integral+emission _CO2; 0.463, 0.733, tmp+pH+PL+OD+RS+Arg_conc._cumulo+rab_in tegral+RQ_integral+emission_O2+emission_CO2; 0.471, 0.733, tmp+pH+PL+AN+OD+RS+cell_yield_cumulo+RQ+rab; 0.471, 0.733, tmp+pH+PL+AN+OD+Sugar_consuming_rate_cumulo+Arg_conc._cumul o+cell_yield_cumulo+rab; 0.471, 0.733, tmp+pH+PL+AN+OD+RS+r ab_integral+emission_CO2+rab; 0.462, 0.733, tmp+pH+PL+RS+Su gar_consuming_rate_cumulo+Arg_conc._cumulo+cell_yield-cumul o+emission_O2+emission_CO2+RQ; 0.453, 0.733, tmp+pH+PL+AN+O D+Sugar_consuming_rate_cumulo+Arg_conc._cumulo+emission_O2+ emission_CO2+RQ+rab; 0.479, 0.733, tmp+pH+PL+OD+RS+Sugar_co nsuming_rate_cumulo+emission_O2+RQ; 0.479, 0.733, tmp+pH+PL +AN+OD+Arg_conc._cumulo+cell_yield_cumulo+rab_integral; 0.4 62, 0.733, tmp+pH+PL+OD+RS+Sugar_consuming_rate_cumulo+cell _yield_cumulo+emission_CO2+RQ+rab; 0.471, 0.733, tmp+pH+PL+ OD+RS+Sugar_consuming_rate_cumulo+emission_O2+emission_CO2+ rab; 0.471, 0.733, tmp+pH+PL+RS+Sugar_consuming_rate_cumulo +Arg_conc._cumulo+cell_yield_cumulo+rab_integral+emission_C 02; 0.487, 0.733, tmp+pH+PL+AN+rab_integral+emission_O2+RQ; 0.487, 0.733, tmp+pH+PL+RS+Sugar_consuming_rate_cumulo+Arg _conc._cumulo+rab; 0.471, 0.733, tmp+pH+PL+OD+RS+rab_integr al+emission_CO2+RQ+rab; 0.435, 0.733, tmp+pH+PL+OD+RS+Arg_c onc._cumulo+cell_yield_cumulo+rab_integral+RQ_integral+emis sion_O2+emission_CO2+RQ+rab; 0.487, 0.733, tmp+pH+PL+AN+RS+ Sugar_consuming_rate_cumulo+Arg_conc._cumulo; 0.462, 0.733, tmp+pH+PL+RS+Sugar_consuming_rate_cumulo+Arg_conc._cumulo+ emission_O2+emission_CO2+RQ+rab; 0.479, 0.733, tmp+pH+PL+OD +RS+rab_integral+RQ+rab; 0.495, 0.733, tmp+pH+Arg_conc._cum ulo+emission_O2+emission_CO2+RQ; 0.462, 0.733, tmp+pH+PL+OD +RS+Arg_conc._cumulo+rab_integral+RQ_integral+emission_O2+R Q; 0.479, 0.733, tmp+pH+PL+Arg_conc._cumulo+cell_yield_cumu lo+emission_O2+emission CO2+RQ; 0.444, 0.733, tmp+pH+PL+AN+ OD+RS+Arg_conc._cumulo+rab_integral+RQ_integral+emission_CO 2+RQ+rab; 0.470, 0.733, tmp+pH+PL+OD+RS+Sugar_consuming_rat e_cumulo+rab_integral+emission_CO2+RQ; 0.479, 0.733, tmp+pH +PL+AN+rab_integral+emission_O2+emission_CO2 + rab; 0.502, 0. 732, tmp+pH+PL+OD+RQ; 0.487, 0.732, tmp+pH+PL+RS+Sugar_cons uming_rate_cumulo+cell_yield_cumulo+rab; 0.462, 0.732, tmp+ pH+PL+AN+OD+Arg_conc._cumulo+rab_integral+emission_CO2+RQ+r ab; 0.478, 0.732, tmp+pH+PL+AN+cell_yield_cumulo+rab_integr al+emission_O2+rab; 0.470, 0.732, tmp+pH+PL+AN+RS+Arg_conc. _cumulo+rab_integral+emission_CO2+RQ; 0.462, 0.732, tmp+pH+ PL+AN+RS+Sugar_consuming_rate_cumulo+Arg_conc._cumulo+emiss ion_O2+emission_CO2+RQ; 0.486, 0.732, tmp+pH+PL+RS+Sugar_co nsuming_rate_cumulo+cell_yield_cumulo+emission_CO2; 0.494, 0.732, tmp+pH+PL+Arg_conc._cumulo+emission_O2+rab; 0.478, 0. 732, tmp+pH+PL+AN+Sugar_consuming_rate_cumulo+Arg_conc._cum ulo+cell_yield_cumulo+RQ; 0.494, 0.732, tmp+pH+PL+AN+RS+Arg _conc._cumulo; 0.453, 0.732, tmp+pH+PL+AN+OD+RS+cell_yield_ cumulo+rab_integral+emission_O2+RQ+rab; 0.461, 0.732, tmp+p H+PL+Sugar_consuming_rate_cumulo+cell_yield_cumulo+rab_inte gral+emission_O2+emission_CO2+RQ+rab; 0.461, 0.732, tmp+pH+ PL+AN+RS+Arg_conc._cumulo+cell_yield_cumulo+rab_integral+em ission_O2+emission_CO2; 0.478, 0.732, tmp+pH+PL+RS+cell_yie ld_cumulo+rab_integral+emission_O2+rab; 0.486, 0.732, tmp+p H+PL+Sugar_consuming_rate_cumulo+Arg_conc._cumulo+emission_ O2+RQ; 0.452, 0.732, tmp+pH+PL+OD+RS+Arg_conc._cumulo+cell_ yield_cumulo+RQ_integral+emission_O2+emission_CO2+RQ; 0.494, 0.732, tmp+pH+PL+Sugar_consuming_rate_cumulo+Arg_conc._cum ulo+rab_integral; 0.486, 0.732, tmp+pH+PL+OD+rab_integral+R Q_integral+emission_O2; 0.470, 0.732, tmp+pH+PL+AN+OD+Arg_c onc._cumulo+cell_yield_cumulo+rab_integral+RQ; 0.470, 0.732, tmp+pH+PL+RS+Sugar_consuming_rate_cumulo+Arg_conc._cumulo+ cell_yield_cumulo+emission_O2+emission_CO2; 0.486, 0.732, t mp+pH+PL+AN+Arg_conc._cumulo+emission_O2+RQ; 0.470, 0.732, tmp+pH+PL+AN+Sugar_consuming_rate_cumulo+Arg_conc._cumulo+r ab_integral+emission_CO2+RQ; 0.494, 0.732, tmp+pH+PL+Sugar_ consuming_rate_cumulo+rab_integral+emission_O2; 0.486, 0.73 2, tmp+pH+PL+AN+Arg_conc._cumulo+emission_CO2+rab; 0.469, 0. 732, tmp+pH+PL+AN+RS+Arg_conc._cumulo+emission_O2+emission_ CO2+rab; 0.461, 0.732, tmp+pH+PL+Arg_conc._cumulo+rab_integ ral+RQ_integral+emission_O2+emission_CO2+RQ+rab; 0.469, 0.7 32, tmp+pH+PL+AN+RS+Sugar_consuming_rate_cumulo+cell_yield_ cumulo+rab_integral+emission_O2; 0.461, 0.732, tmp+pH+PL+AN +OD+Sugar_consuming_rate_cumulo+rab_integral+emission_O2+em ission_CO2+rab; 0.452, 0.732, tmp+pH+PL+AN+RS+Sugar_consumi ng_rate_cumulo+Arg_conc._cumulo+cell_yield_cumulo+emission_ CO2+RQ+rab; 0.493, 0.732, tmp+pH+PL+RS+rab_integral+emissio n_O2; 0.478, 0.732, tmp+pH+PL+RS+cell_yield_cumulo+rab_inte gral+emission_O2+emission_CO2; 0.486, 0.732, tmp+pH+PL+Suga r_consuming_rate_cumulo+Arg_conc._cumulo+cell_yield_cumulo+ emission_O2; 0.469, 0.732, tmp+pH+PL+AN+OD+Sugar_consuming_ rate_cumulo+Arg_conc._cumulo+rab_integral+rab; 0.443, 0.732, tmp+pH+PL+AN+RS+Sugar_consuming_rate_cumulo+Arg_conc._cumu lo+rab_integral+emission_O2+emission_CO2+RQ+rab; 0.501, 0.7 32, tmp+pH+PL+Arg_conc._cumulo+rab; 0.469, 0.732, tmp+pH+PL +AN+Sugar_consuming_rate_cumulo+cell_yield_cumulo+rab_integ ral+emission_O2+emission_CO2; 0.469, 0.732, tmp+pH+PL+Sugar _consuming_rate_cumulo+Arg_conc._cumulo+cell_yield_cumulo+r ab_integral+emission_CO2+RQ; 0.477, 0.732, tmp+pH+PL+RS+Sug ar_consuming_rate_cumulo+Arg_conc._cumulo+cell_yield_cumulo +rab; 0.485, 0.732, tmp+pH+PL+OD+RS+cell_yield_cumulo+rab_i ntegral; 0.452, 0.732, tmp+pH+PL+AN+OD+RS+Sugar_consuming_r ate_cumulo+Arg_conc._cumulo+emission_O2+RQ+rab; 0.493, 0.73 2, tmp+pH+PL+OD+emission_O2+rab; 0.477, 0.732, tmp+pH+PL+OD +RS+Arg_conc._cumulo+RQ_integral+emission_CO2; 0.460, 0.732, tmp+pH+PL+RS+Sugar_consuming_rate_cumulo+cell_yield_cumulo +rab_integral+emission_O2+RQ+rab; 0.469, 0.732, tmp+pH+PL+O D+RS+Arg_conc._cumulo+cell_yield_cumulo+RQ_integral+emissio n_CO2; 0.469, 0.732, tmp+pH+PL+AN+OD+RS+Sugar_consuming_rat e_cumulo+rab_integral+rab; 0.485, 0.732, tmp+pH+PL+OD+RS+ce ll_yield_cumulo+emission_O2; 0.469, 0.732, tmp+pH+PL+AN+RS+ Arg_conc._cumulo+cell_yield_cumulo+rab_integral+emission_O 2; 0.460, 0.732, tmp+pH+PL+AN+OD+RS+Arg_conc._cumulo+rab_in tegral+RQ_integral+emission_CO2; 0.460, 0.732, tmp+pH+PL+AN +OD+Arg_conc._cumulo+RQ_integral+emission_CO2+RQ+rab; 0.493, 0.732, tmp+pH+PL+OD+Sugar_consuming_rate_cumulo+RQ; 0.460, 0.732, tmp+pH+PL+RS+Sugar_consuming_rate_cumulo+Arg_conc._ cumulo+rab_integral+emission_CO2+RQ+rab; 0.451, 0.732, tmp+ pH+PL+AN+OD+Sugar_consuming_rate_cumulo+Arg_conc._cumulo+ra b_integral+emission_CO2+RQ+rab; 0.451, 0.732, tmp+pH+PL+AN+ OD+RS+Sugar_consuming_rate_cumulo+cell_yield_cumulo+emissio n_CO2+RQ+rab; 0.493, 0.732, tmp+pH+PL+AN+Arg_conc._cumulo+R Q; 0.460, 0.732, tmp+pH+PL+OD+RS+Sugar_consuming_rate_cumul o+rab_integral+emission_CO2+RQ+rab; 0.451, 0.732, tmp+pH+PL +AN+OD+RS+Sugar_consuming_rate_cumulo+rab_integral+emission _O2+emission_CO2+rab; 0.477, 0.732, tmp+pH+PL+AN+cell_yield _cumulo+rab_integral+emission_O2+emission_CO2; 0.442, 0.731, tmp+pH+PL+AN+OD+RS+Arg_conc._cumulo+RQ_integral+emission_O 2+emission_CO2+RQ+rab; 0.460, 0.731, tmp+pH+PL+AN+OD+Sugar_ consuming_rate_cumulo+rab_integral+emission_O2+emission_CO2 +RQ; 0.477, 0.731, tmp+pH+PL+AN+OD+RS+Arg_conc._cumulo+RQ_i ntegral; 0.469, 0.731, tmp+pH+PL+OD+RS+Arg_conc._cumulo+rab _integral+RQ_integral+emission_O2; 0.493, 0.731, tmp+pH+PL+ OD+Sugar_consuming_rate_cumulo+rab; 0.477, 0.731, tmp+pH+PL +RS+Sugar_consuming_rate_cumulo+cell_yield_cumulo+emission-CO2+rab; 0.451, 0.731, tmp+pH+PL+OD+RS+Arg_conc._cumulo+cel l_yield_cumulo+rab_integral+RQ_integral+emission_O2+emissio n_CO2; 0.493, 0.731, tmp+pH+PL+RS+Arg_conc._cumulo+RQ; 0.46 9, 0.731, tmp+pH+PL+Arg_conc._cumulo+rab_integral+RQ_integr al+emission_O2+emission_CO2+rab; 0.477, 0.731, tmp+pH+PL+Su gar_consuming_rate_cumulo+Arg_conc._cumulo+rab_integral+emi ssion_CO2+rab; 0.477, 0.731, tmp+pH+PL+RS+cell_yield_cumulo +rab_integral+emission_O2+RQ; 0.451, 0.731, tmp+pH+PL+AN+OD +RS+cell_yield_cumulo+rab_integral+emission_O2+emission_CO2 +rab; 0.460, 0.731, tmp+pH+PL+RS+Sugar_consuming_rate_cumul o+cell_yield_cumulo+rab_integral+emission_O2+emission_CO2+r ab; 0.468, 0.731, tmp+pH+PL+AN+Arg_conc._cumulo+rab_integra 1+RQ_integral+emission_O2+emission_CO2; 0.493, 0.731, tmp+p H+PL+AN+OD+emission_O2; 0.485, 0.731, tmp+pH+PL+Arg_conc._c umulo+cell_yield_cumulo+emission_O2+emission_CO2; 0.477, 0. 731, tmp+pH+PL+RS+rab_integral+emission_O2+RQ+rab; 0.477, 0. 731, tmp+pH+PL+AN+OD+RS+Sugar_consuming_rate_cumulo+emissio n_O2; 0.451, 0.731, tmp+pH+PL+AN+RS+Sugar_consuming_rate_cu mulo+Arg_conc._cumulo+cell_yield_cumulo+rab_integral+emissi on_O2+RQ; 0.460, 0.731, tmp+pH+PL+AN+Sugar_consuming_rate_c umulo+cell_yield_cumulo+rab_integral+emission_O2+RQ+rab; 0. 468, 0.731, tmp+pH+PL+AN+OD+Sugar_consuming_rate_cumulo+Arg _conc._cumulo+emission_O2+rab; 0.476, 0.731, tmp+pH+PL+OD+R S+cell_yield_cumulo+rab_integral+rab; 0.492, 0.731, tmp+pH+ PL+OD+emission_O2+emission_CO2; 0.459, 0.731, tmp+pH+PL+AN+ RS+Sugar_consuming_rate_cumulo+Arg_conc._cumulo+rab_integra l+emission_CO2+RQ; 0.459, 0.731, tmp+pH+PL+OD+RS+Sugar_cons uming_rate_cumulo+Arg_conc._cumulo+RQ_integral+emission_CO2 +RQ; 0.476, 0.731, tmp+pH+PL+AN+OD+Arg_conc._cumulo+RQ_inte gral+emission_CO2; 0.459, 0.731, tmp+pH+PL+AN+OD+Arg_conc._ cumulo+cell_yield_cumulo+emission_O2+emission_CO2+rab; 0.43 2, 0.731, tmp+pH+PL+AN+OD+RS+Arg_conc._cumulo+cell_yield_cu mulo+rab_integral+RQ_integral+emission_CO2+RQ+rab; 0.468, 0. 731, tmp+pH+PL+Sugar_consuming_rate_cumulo+Arg_conc._cumulo +rab_integral+emission_CO2+RQ+rab; 0.468, 0.731, tmp+pH+PL+ AN+OD+RS+Sugar_consuming_rate_cumulo+rab_integral+emission CO2; 0.484, 0.731, tmp+pH+PL+AN+Sugar_consuming_rate_cumulo +Arg_conc._cumulo+rab

### [202. Linear Model that Predicts Arginine Product ion Amount in Interval 2]

0.791, 0.914, tmp+PL+PL_cumulo+interval_Pdt.+OD+nu_cumulo+r ho_cumulo+nu_per_rho_cumulo+Arg_conc._cumulo+Accum._Yield+e mission_O2+emission_CO2+consum_O2+generate_CO2; 0.801, 0.91 3, tmp+PL+AN+interval_Pdt.+mu_cumulo+nu_cumulo+rho_cumulo+n u_per_rho_cumulo+emission_CO2+consum_O2+generate_CO2; 0.801, 0.913, tmp+PL+interval_Pdt.+mu_cumulo+nu_cumulo+rho_cumulo +nu_per_rho_cumulo+rab_integral+emission_CO2+consum_O2+gene rate_CO2; 0.797, 0.913, tmp+PL+AN+interval_Pdt.+mu_cumulo+n u_cumulo+rho_cumulo+nu_per_rho_cumulo+Accum._Yield+emission _CO2+consum_O2+generate_CO2; 0.784, 0.913, tmp+PL+PL_cumulo +AN+interval_Pdt.+OD+nu_cumulo+rho_cumulo+nu_per_rho_cumulo +Arg_conc._cumulo+Accum._Yield+emission_O2+emission_CO2+con sum_O2+generate_CO2; 0.792, 0.913, tmp+PL+interval_Pdt.+mu_ cumulo+nu_cumulo+rho_cumulo+nu_per_rho_cumulo+rab_integral+ RQ_integral+Accum._Yield+emission_CO2+consum_O2+generate_CO 2; 0.787, 0.913, tmp+PL+PL_cumulo+AN+interval_Pdt.+mu_cumul o+nu_cumulo+rho_cumulo+nu_per_rho_cumulo+rab_integral+Accum. _Yield+emission_CO2+consum_O2+generate_CO2; 0.795, 0.913, t mp+PL+interval_Pdt.+mu_cumulo+nu_cumulo+rho_cumulo+nu_per_r ho_cumulo+cell_yield_cumulo+rab_integral+emission_CO2+consu m_O2+generate_CO2; 0.783, 0.913, tmp+PL+PL_cumulo+interval_ Pdt.+OD+nu_cumulo+rho_cumulo+nu_per_rho_cumulo+Arg_conc._cu mulo+RQ_integral+Accum._Yield+emission_O2+emission_CO2+cons um_O2+generate_CO2; 0.795, 0.913, tmp+PL+interval_Pdt.+mu_c umulo+nu_cumulo+rho_cumulo+nu_per_rho_cumulo+rab_integral+R Q_integral+emission_CO2+consum_O2+generate_CO2; 0.782, 0.91 2, tmp+PL+PL_cumulo+AN+interval_Pdt.+OD+nu_cumulo+rho_cumul o+nu_per_rho_cumulo+Arg_conc._cumulo+rab_integral+Accum._Yi eld+emission_CO2+consum_O2+generate_CO2; 0.791, 0.912, tmp+ PL+PL_cumulo+AN+interval_Pdt.+mu_cumulo+nu_cumulo+rho_cumul o+nu_per_rho_cumulo+rab_integral+emission_CO2+consum_O2+gen erate_CO2; 0.790, 0.912, tmp+PL+interval_Pdt.+OD+nu_cumulo+ rho_cumulo+nu_per_rho_cumulo+Arg_conc._cumulo+rab_integral+ Accum._Yield+emission_CO2+consum_O2+generate_CO2; 0.794, 0. 912, tmp+PL+interval_Pdt.+mu_cumulo+nu_cumulo+rho_cumulo+nu _per_rho_cumulo+rab_integral+Accum._Yield+emission_CO2+cons um_O2+generate_CO2; 0.794, 0.912, tmp+PL+AN+interval_Pdt.+m u_cumulo+nu_cumulo+rho_cumulo+nu_per_rho_cumulo+emission_CO 2+consum_O2+interval_O2+generate_CO2; 0.794, 0.912, tmp+PL+ PL_cumulo+interval_Pdt.+mu_cumulo+nu_cumulo+rho_cumulo+nu_p er_rho_cumulo+rab_integral+emission_CO2+consum_O2+generate-CO2; 0.782, 0.912, tmp+PL+PL_cumulo+AN+interval_Pdt.+mu_cum ulo+nu_cumulo+rho_cumulo+nu_per_rho_cumulo+Accum._Yield+emi ssion_O2+emission_CO2+consum_O2+interval_O2+generate_CO2; 0. 781, 0.912, tmp+PL+PL_cumulo+interval_Pdt.+OD+nu_cumulo+rho _cumulo+nu_per_rho_cumulo+Arg_conc._cumulo+Accum._Yield+emi ssion_O2+emission_CO2+consum_O2+interval_O2+generate_CO2; 0. 789, 0.912, tmp+PL+AN+interval_Pdt.+mu_cumulo+nu_cumulo+rho _cumulo+nu_per_rho_cumulo+rab_integral+emission_CO2+consum_ O2+interval_O2+generate_CO2; 0.785, 0.912, tmp+PL+PL_cumulo +interval_Pdt.+OD+nu_cumulo+rho_cumulo+nu_per_rho_cumulo+Ar g_conc._cumulo+rab_integral+Accum._Yield+emission_CO2+consu m_O2+generate_CO2; 0.785, 0.912, tmp+PL+PL_cumulo+AN+interv al_Pdt.+mu_cumulo+nu_cumulo+rho_cumulo+nu_per_rho_cumulo+ra b_integral+emission_CO2+consum_O2+interval_O2+generate_CO2; 0.785, 0.912, tmp+PL+PL_cumulo+AN+interval_Pdt.+mu_cumulo+ nu_cumulo+rho_cumulo+nu_per_rho_cumulo+emission_O2+emission _CO2+consum_O2+interval_O2+generate_CO2; 0.793, 0.912, tmp+ PL+AN+interval_Pdt.+mu_cumulo+nu_cumulo+rho_cumulo+nu_per_r ho_cumulo+rab_integral+emission_CO2+consuni_O2+generate_CO2 ; 0.789, 0.912, tmp+PL+interval_Pdt.+mu_cumulo+nu_cumulo+rho _cumulo+nu_per_rho_cumulo+cell_yield_cumulo+rab_integral+Ac cum._Yield+emission_CO2+consum_O2+generate_CO2; 0.789, 0.91 2, tmp+PL+AN+interval_Pdt.+mu_cumulo+nu_cumulo+rho_cumulo+n u_per_rho_cumulo+rab_integral+Accum._Yield+emission_CO2+con sum_O2+generate_CO2; 0.780, 0.912, tmp+PL+PL_cumulo+AN+inte rval_Pdt.+mu_cumulo+nu_cumulo+rho_cumulo+nu_per_rho_cumulo+ rab_integral+Accum._Yield+emission_CO2+consum_O2+interval_O 2+generate_CO2; 0.780, 0.912, tmp+PL+PL_cumulo+interval_Pdt. +OD+nu_cumulo+rho_cumulo+nu_per_rho_cumulo+Arg_conc._cumulo +rab_integral+Accum._Yield+emission_O2+emission_CO2+consum_ O2+generate_CO2; 0.789, 0.912, tmp+PL+AN+interval_Pdt.+mu_c umulo+nu_cumulo+rho_cumulo+nu_per_rho_cumulo+Accum._Yield+e mission_CO2+consum_O2+interval_O2+generate_CO2; 0.793, 0.91 2, tmp+PL+interval_Pdt.+RS+mu_cumulo+nu_cumulo+rho_cumulo+n u_per_rho_cumulo+rab_integral+emission_CO2+consum_O2+genera te_CO2; 0.789, 0.912, tmp+PL+interval_Pdt.+mu_cumulo+nu_cum ulo+rho_cumulo+nu_per_rho_cumulo+RQ_integral+Accum._Yield+e mission_O2+emission_CO2+consum_O2+generate_CO2; 0.789, 0.91 2, tmp+PL+AN+interval_Pdt.+mu_cumulo+nu_cumulo+rho_cumulo+n u_per_rho_cumulo+cell_yield_cumulo+rab_integral+emission_CO 2+consum_O2+generate_CO2; 0.784, 0.911, tmp+PL+PL_cumulo+in terval_Pdt.+mu_cumulo+nu_cumulo+rho_cumulo+nu_per_rho_cumul o+RQ_integral+Accum._Yield+emission_O2+emission_CO2+consum O2+generate_CO2; 0.780, 0.911, tmp+PL+PL_cumulo+AN+interval _Pdt.+mu_cumulo+nu_cumulo+rho_cumulo+nu_per_rho_cumulo+Arg_ conc._cumulo+rab_integral+Accum._Yield+emission_CO2+consum_ O2+generate_CO2; 0.792, 0.911, tmp+PL+interval_Pdt.+OD+mu_c umulo+nu_cumulo+rho_cumulo+nu_per_rho_cumulo+rab_integral+e mission_CO2+consum_O2+generate_CO2; 0.784, 0.911, tmp+PL+in terval_Pdt.+mu_cumulo+nu_cumulo+rho_cumulo+nu_per_rho_cumul o+cell_yield_cumulo+RQ_integral+Accum._Yield+emission_O2+em ission_CO2+consum_O2+generate_CO2; 0.788, 0.911, tmp+PL+PL_ cumulo+interval_Pdt.+mu_cumulo+nu_cumulo+rho_cumulo+nu_per_ rho_cumulo+rab_integral+Accum._Yield+emission_CO2+consum_O2 +generate_CO2; 0.788, 0.911, tmp+PL+interval_Pdt.+OD+nu_cum ulo+rho_cumulo+nu_per_rho_cumulo+Arg_conc._cumulo+Accum._Yi eld+emission_O2+emission_CO2+consum_O2+generate_CO2; 0.775, 0.911, tmp+PL+PL_cumulo+AN+interval_Pdt.+OD+nu_cumulo+rho_ cumulo+nu_per_rho_cumulo+Arg_conc._cumulo+Accum._Yield+emis sion_O2+emission_CO2+consum_O2+interval_O2+generate_CO2; 0. 784, 0.911, tmp+PL+AN+interval_Pdt.+mu_cumulo+nu_cumulo+rho _cumulo+nu_per_rho_cumulo+cell_yield_cumulo+rab_integral+Ac cum._Yield+emission_CO2+consum_O2+generate_CO2; 0.784, 0.91 1, tmp+PL+interval_Pdt.+mu_cumulo+nu_cumulo+rho_cumulo+nu_p er_rho_cumulo+cell_yield_cumulo+rab_integral+RQ_integral+Ac cum._Yield+emission_CO2+consum_O2+generate_CO2; 0.784, 0.91 1, tmp+PL+PL_cumulo+AN+interval_Pdt.+mu_cumulo+nu_cumulo+rh o_cumulo+nu_per_rho_cumulo+Accum._Yield+emission_O2+emissio n_CO2+consum_O2+generate_CO2; 0.795, 0.911, tmp+PL+interval _Pdt.+mu_cumulo+nu_cumulo+rho_cumulo+nu_per_rho_cumulo+emis sion_O2+emission_CO2+consum_O2+generate_CO2; 0.783, 0.911, tmp+PL+AN+interval_Pdt.+mu_cumulo+nu_cumulo+rho_cumulo+nu_p er_rho_cumulo+rab_integral+Accum._Yield+emission_CO2+consum _O2+interval_O2+generate_CO2; 0.788, 0.911, tmp+PL+PL_cumul o+OD+nu_cumulo+rho_cumulo+nu_per_rho_cumulo+Arg_conc._cumul o+Accum._Yield+emission_O2+emission_CO2+consum_O2+generate CO2; 0.799, 0.911, tmp+PL+interval_Pdt.+mu_cumulo+nu_cumulo +rho_cumulo+nu_per_rho_cumulo+emission_CO2+consum_O2+genera te_CO2; 0.787, 0.911, tmp+PL+AN+interval_Pdt.+mu_cumulo+nu_ cumulo+rho_cumulo+nu_per_rho_cumulo+Arg_conc._cumulo+Accum. _Yield+emission_CO2+consum_O2+generate_CO2; 0.783, 0.911, t mp+PL+AN+interval_Pdt.+mu_cumulo+nu_cumulo+rho_cumulo+nu_pe r_rho_cumulo+rab_integral+RQ_integral+Accum._Yield+emission _CO2+consum_O2+generate_CO2; 0.779, 0.911, tmp+PL+PL_cumulo +interval_Pdt.+OD+RS+nu_cumulo+rho_cumulo+nu_per_rho_cumulo +Arg_conc._cumulo+Accum._Yield+emission_O2+emission_CO2+con sum_O2+generate_CO2; 0.791, 0.911, tmp+PL+PL_cumulo+interva l_Pdt.+mu_cumulo+nu_cumulo+rho_cumulo+nu_per_rho_cumulo+emi ssion_O2+emission_CO2+consum_O2+generate_CO2; 0.783, 0.911, tmp+PL+AN+interval_Pdt.+OD+nu_cumulo+rho_cumulo+nu_per_rho _cumulo+Arg_conc._cumulo+rab_integral+Accum._Yield+emission _CO2+consum_O2+generate_CO2; 0.787, 0.911, tmp+PL+interval_ Pdt.+mu_cumulo+nu_cumulo+rho_cumulo+nu_per_rho_cumulo+cell_ yield_cumulo+rab_integral+RQ_integral+emission_CO2+consuni_O 2+generate_CO2; 0.791, 0.911, tmp+PL+interval_Pdt.+mu_cumul o+nu_cumulo+rho_cumulo+nu_per_rho_cumulo+rab_integral+emiss ion_CO2+consum_O2+interval_O2+generate_CO2; 0.791, 0.911, t mp+PL+AN+interval_Pdt.+mu_cumulo+nu_cumulo+rho_cumulo+nu_pe r_rho_cumulo+Arg_conc._cumulo+emission_CO2+consum_O2+genera te_CO2; 0.787, 0.911, tmp+PL+PL_cumulo+interval_Pdt.+mu_cum ulo+nu_cumulo+rho_cumulo+nu_per_rho_cumulo+Accum._Yield+emi ssion_O2+emission_CO2+consum_O2+generate_CO2; 0.791, 0.911, tmp+PL+AN+interval_Pdt.+mu_cumulo+nu_cumulo+rho_cumulo+nu_ per_rho_cumulo+cell_yield_cumulo+emission_CO2+consuni_O2+gen erate_CO2; 0.791, 0.911, tmp+PL+interval_Pdt.+mu_cumulo+nu_ cumulo+rho_cumulo+nu_per_rho_cumulo+cell_yield_cumulo+emiss ion_O2+emission_CO2+consum_O2+generate_CO2; 0.787, 0.911, t mp+PL+AN+interval_Pdt.+mu_cumulo+nu_cumulo+rho_cumulo+nu_pe r_rho_cumulo+cell_yield_cumulo+Accum._Yield+emission_CO2+co nsum_O2+generate_CO2; 0.798, 0.911, tmp+PL+AN+interval_Pdt. +mu_cumulo+rho_cumulo+Accum._Yield+emission_CO2+consum_O2+g enerate_CO2; 0.774, 0.911, tmp+PL+PL_cumulo+AN+interval_Pdt. +OD+nu_cumulo+rho_cumulo+nu_per_rho_cumulo+Arg_conc._cumulo +rab_integral+Accum._Yield+emission_O2+emission_CO2+consum_ O2+generate_CO2; 0.791, 0.911, tmp+PL+interval_Pdt.+mu_cumu lo+nu_cumulo+rho_cumulo+nu_per_rho_cumulo+Arg_conc._cumulo+ rab_integral+emission_CO2+consum_O2+generate_CO2; 0.773, 0. 911, tmp+PL+PL_cumulo+interval_Pdt.+OD+nu_cumulo+rho_cumulo +nu_per_rho_cumulo+Arg_conc._cumulo+RQ_integral+Accum._Yiel d+emission_O2+emission_CO2+consum_O2+interval_O2+generate_C 02; 0.782, 0.911, tmp+PL+interval_Pdt.+OD+nu_cumulo+rho_cum ulo+nu_per_rho_cumulo+Arg_conc._cumulo+rab_integral+RQ_inte gral+Accum._Yield+emission_CO2+consum_O2+generate_CO2; 0.79 0, 0.911, tmp+PL+AN+interval_Pdt.+RS+mu_cumulo+nu_cumulo+rh o_cumulo+nu_per_rho_cumulo+emission_CO2+consum_O2+generate_ CO2; 0.782, 0.910, tmp+PL+interval_Pdt.+RS+mu_cumulo+nu_cum ulo+rho_cumulo+nu_per_rho_cumulo+rab_integral+RQ_integral+A ccum._Yield+emission_CO2+consum_O2+generate_CO2; 0.786, 0.9 10, tmp+PL+AN+interval_Pdt.+mu_cumulo+nu_cumulo+rho_cumulo+ nu_per_rho_cumulo+emission_O2+emission_CO2+consum_O2+interv al_O2+generate_CO2; 0.782, 0.910, tmp+PL+interval_Pdt.+OD+n u_cumulo+rho_cumulo+nu_per_rho_cumulo+Arg_conc._cumulo+RQ_i ntegral+Accum._Yield+emission_O2+emission_CO2+consum_O2+gen erate_CO2; 0.798, 0.910, tmp+PL_cumulo+interval_Pdt.+mu_cum ulo+nu_cumulo+rho_cumulo+nu_per_rho_cumulo+emission_CO2+con sum_O2+generate_CO2; 0.786, 0.910, tmp+PL+interval_Pdt.+RS+ mu_cumulo+nu_cumulo+rho_cumulo+nu_per_rho_cumulo+rab_integr al+RQ_integral+emission_CO2+consum_O2+generate_CO2; 0.786, 0.910, tmp+PL+AN+interval_Pdt.+RS+mu_cumulo+nu_cumulo+rho_c umulo+nu_per_rho_cumulo+Accum._Yield+emission_CO2+consum_O2 +generate_CO2; 0.786, 0.910, tmp+PL+interval_Pdt.+RS+mu_cum ulo+nu_cumulo+rho_cumulo+nu_per_rho_cumulo+rab_integral+Acc um._Yield+emission_CO2+consum_O2+generate_CO2; 0.782, 0.910, tmp+PL+AN+interval_Pdt.+mu_cumulo+nu_cumulo+rho_cumulo+nu_ per_rho_cumulo+cell_yield_cumulo+rab_integral+emission_CO2+ consum_O2+interval_O2+generate_CO2; 0.790, 0.910, tmp+PL+AN +interval_Pdt.+OD+mu_cumulo+nu_cumulo+rho_cumulo+nu_per_rho _cumulo+emission_CO2+consum_O2+generate_CO2; 0.786, 0.910, tmp+PL+interval_Pdt.+RS+mu_cumulo+nu_cumulo+rho_cumulo+nu_p er_rho_cumulo+cell_yield_cumulo+rab_integral+emission_CO2+c onsum_O2+generate_CO2; 0.794, 0.910, tmp+PL+AN+interval_Pdt. +mu_cumulo+rho_cumulo+Accum._Yield+emission_CO2+consum_O2+i nterval_O2+generate_CO2; 0.786, 0.910, tmp+PL+interval_Pdt. +mu_cumulo+nu_cumulo+rho_cumulo+nu_per_rho_cumulo+Arg_conc. _cumulo+cell_yield_cumulo+rab_integral+emission_CO2+consum_ O2+generate_CO2; 0.786, 0.910, tmp+PL+PL_cumulo+interval_Pd t.+mu_cumulo+nu_cumulo+rho_cumulo+nu_per_rho_cumulo+emissio n_O2+emission_CO2+consum_O2+interval_O2+generate_CO2; 0.773, 0.910, tmp+PL+PL_cumulo+interval_Pdt.+OD+nu_cumulo+rho_cum ulo+nu_per_rho_cumulo+Arg_conc._cumulo+rab_integral+RQ_inte gral+Accum._Yield+emission_O2+emission_CO2+consum_O2+genera te_CO2; 0.790, 0.910, tmp+PL+interval_Pdt.+mu_cumulo+nu_cum ulo+rho_cumulo+nu_per_rho_cumulo+Accum._Yield+emission_O2+e mission_CO2+consum_O2+generate_CO2; 0.786, 0.910, tmp+PL+AN +interval_Pdt.+OD+nu_cumulo+rho_cumulo+nu_per_rho_cumulo+Ar g_conc._cumulo+Accum._Yield+emission_CO2+consum_O2+generate _CO2; 0.790, 0.910, tmp+PL+interval_Pdt.+OD+nu_cumulo+rho_c umulo+nu_per_rho_cumulo+Arg_conc._cumulo+rab_integral+emiss ion_CO2+consum_O2+generate_CO2; 0.782, 0.910, tmp+PL+interv al_Pdt.+OD+mu_cumulo+nu_cumulo+rho_cumulo+nu_per_rho_cumulo +Arg_conc._cumulo+rab_integral+Accum._Yield+emission_CO2+co nsum_O2+generate_CO2; 0.786, 0.910, tmp+PL+AN+interval_Pdt. +OD+mu_cumulo+nu_cumulo+rho_cumulo+nu_per_rho_cumulo+Accum. _Yield+emission_CO2+consum_O2+generate_CO2; 0.786, 0.910, t mp+PL+AN+interval_Pdt.+RS+mu_cumulo+nu_cumulo+rho_cumulo+nu _per_rho_cumulo+rab_integral + emission_CO2+consum_O2+generat e_CO2; 0.777, 0.910, tmp+PL+PL_cumulo+interval_Pdt.+mu_cumu lo+nu_cumulo+rho_cumulo+nu_per_rho_cumulo+RQ_integral+Accum. _Yield+emission_O2+emission_CO2+consum_O2+interval_O2+gener ate_CO2; 0.786, 0.910, tmp+PL+AN+interval_Pdt.+OD+mu_cumulo +nu_cumulo+rho_cumulo+nu_per_rho_cumulo+rab_integral+emissi on-CO2+consum-O2+generate-CO2; 0.790, 0.910, tmp+PL_cumulo+ interval_Pdt.+mu_cumulo+nu_cumulo+rho_cumulo+nu_per_rho_cum ulo+cell_yield_cumulo+rab_integral+emission_CO2+consum_O2+g enerate_CO2; 0.790, 0.910, tmp+PL+OD+nu_cumulo+rho_cumulo+n u_per_rho_cumulo+Arg_conc._cumulo+Accum._Yield+emission_O2+ emission_CO2+consum_O2+generate_CO2; 0.781, 0.910, tmp+PL+i nterval_Pdt.+mu_cumulo+nu_cumulo+rho_cumulo+nu_per_rho_cumu lo+Arg_conc._cumulo+rab_integral+RQ_integral+Accum._Yield+e mission_CO2+consum_O2+generate_CO2; 0.777, 0.910, tmp+pH+PL +PL_cumulo+interval_Pdt.+OD+nu_cumulo+rho_cumulo+nu_per_rho _cumulo+Arg_conc._cumulo+Accum._Yield+emission_O2+emission-CO2+consum_O2+generate_CO2; 0.785, 0.910, tmp+PL+interval_P dt.+OD+mu_cumulo+nu_cumulo+rho_cumulo+nu_per_rho_cumulo+rab _integral+Accum._Yield+emission_CO2+consum_O2+generate_CO2; 0.781, 0.910, tmp+PL+interval_Pdt.+OD+RS+nu_cumulo+rho_cum ulo+nu_per_rho_cumulo+Arg_conc._cumulo+rab_integral+Accum.-Yield+emission_CO2+consum_O2+generate_CO2; 0.789, 0.910, tm p+PL+OD+nu_cumulo+rho_cumulo+nu_per_rho_cumulo+Arg_conc._cu mulo+rab_integral+Accum._Yield+emission_CO2+consum_O2+gener ate_CO2; 0.785, 0.910, tmp+PL+PL_cumulo+AN+interval_Pdt.+mu _cumulo+nu_cumulo+rho_cumulo+nu_per_rho_cumulo+emission_O2+ emission_CO2+consum_O2+generate_CO2; 0.785, 0.910, tmp+PL+i nterval_Pdt.+mu_cumulo+nu_cumulo+rho_cumulo+nu_per_rho_cumu lo+cell_yield_cumulo+Accum._Yield+emission_O2+emission_CO2+ consum_O2+generate_CO2; 0.785, 0.910, tmp+PL+interval_Pdt.+ OD+mu_cumulo+nu_cumulo+rho_cumulo+nu_per_rho_cumulo+cell_yi eld_cumulo+rab_integral+emission_CO2+consum_O2+generate_CO 2; 0.781, 0.910, tmp+PL+PL_cumulo+AN+interval_yield+interva l_Pdt.+mu_cumulo+nu_cumulo+rho_cumulo+nu_per_rho_cumulo+rab _integral+emission_CO2+consum_O2+generate_CO2; 0.781, 0.910, tmp+PL+interval_Pdt.+mu_cumulo+nu_cumulo+rho_cumulo+nu_per _rho_cumulo+Arg_conc._cumulo+cell_yield_cumulo+rab_integral +Accum._Yield+emission_CO2+consum_O2+generate_CO2; 0.781, 0. 910, tmp+PL+AN+interval_Pdt.+RS+mu_cumulo+nu_cumulo+rho_cum ulo+nu_per_rho_cumulo+rab_integral+Accum._Yield+emission_CO 2+consum_O2+generate_CO2; 0.772, 0.910, tmp+PL+PL_cumulo+AN +interval_Pdt.+mu_cumulo+nu_cumulo+rho_cumulo+nu_per_rho_cu mulo+rab_integral+Accum._Yield+emission_O2+emission_CO2+con sum_O2+interval_O2+generate_CO2; 0.781, 0.910, tmp+PL+inter val_Pdt.+OD+mu_cumulo+nu_cumulo+rho_cumulo+nu_per_rho_cumul o+rab_integral+RQ_integral+Accum._Yield+emission_CO2+consum _O2+generate_CO2; 0.789, 0.910, tmp+pH+PL+interval_Pdt.+mu_ cumulo+nu_cumulo+rho_cumulo+nu_per_rho_cumulo+rab_integral+ emission_CO2+consum_O2+generate_CO2; 0.772, 0.910, tmp+PL+P L_cumulo+AN+interval_Pdt.+mu_cumulo+nu_cumulo+rho_cumulo+nu _per_rho_cumulo+Arg_conc._cumulo+Accum._Yield+emission_O2+e mission_CO2+consum_O2+interval_O2+generate_CO2; 0.781, 0.91 0, tmp+PL+interval_Pdt.+mu_cumulo+nu_cumulo+rho_cumulo+nu_p er_rho_cumulo+rab_integral+RQ_integral+Accum._Yield+emissio n_O2+emission_CO2+consum_O2+generate_CO2; 0.789, 0.910, tmp +PL+interval_Pdt.+OD+mu_cumulo+nu_cumulo+rho_cumulo+nu_per_ rho_cumulo+emission_O2+emission_CO2+consum_O2+generate_CO2; 0.789, 0.910, tmp+PL+PL_cumulo+AN+interval_Pdt.+mu_cumulo+ nu_cumulo+rho_cumulo+nu_per_rho_cumulo+emission_CO2+consum O2+generate_CO2; 0.780, 0.910, tmp+PL+interval_Pdt.+OD+mu_c umulo+nu_cumulo+rho_cumulo+nu_per_rho_cumulo+RQ_integral+Ac cum._Yield+emission_O2+emission_CO2+consum_O2+generate_CO2 ; 0.780, 0.910, tmp+PL+interval_Pdt.+mu_cumulo+nu_cumulo+rho _cumulo+nu_per_rho_cumulo+rab_integral+RQ_integral+Accum._Y ield+emission_CO2+consum_O2+interval_O2+generate_CO2; 0.785, 0.910, tmp+PL+interval_Pdt.+mu_cumulo+nu_cumulo+rho_cumulo +nu_per_rho_cumulo+rab_integral+RQ_integral+emission_CO2+co nsum_O2+interval_O2+generate_CO2; 0.776, 0.910, tmp+PL+PL_c umulo+interval_Pdt.+mu_cumulo+nu_cumulo+rho_cumulo+nu_per_r ho_cumulo+rab_integral+RQ_integral+Accum._Yield+emission_O2 +emission_CO2+consum_O2+generate_CO2; 0.780, 0.910, tmp+PL+ interval_Pdt.+mu_cumulo+nu_cumulo+rho_cumulo+nu_per_rho_cum ulo+RQ_integral+Accum._Yield+emission_O2+emission_CO2+consu m_O2+interval_O2+generate_CO2; 0.785, 0.910, tmp+PL+PL_cumu lo+interval_Pdt.+mu_cumulo+nu_cumulo+rho_cumulo+nu_per_rho_ cumulo+rab_integral+RQ_integral+emission_CO2+consum_O2+gene rate_CO2; 0.780, 0.910, tmp+PL+PL_cumulo+interval_Pdt.+mu_c umulo+nu_cumulo+rho_cumulo+nu_per_rho_cumulo+Accum._Yield+e mission_O2+emission_CO2+consum_O2+interval_O2+generate_CO2; 0.771, 0.910, tmp+PL+PL_cumulo+AN+interval_Pdt.+mu_cumulo+ nu_cumulo+rho_cumulo+nu_per_rho_cumulo+RQ_integral+Accum._Y ield+emission_O2+emission_CO2+consum_O2+interval_O2+generat e_CO2; 0.789, 0.910, tmp+PL+interval_Pdt.+mu_cumulo+nu_cumu lo+rho_cumulo+nu_per_rho_cumulo+emission_O2+emission_CO2+co nsum_O2+interval_O2+generate_CO2; 0.780, 0.910, tmp+PL+AN+i nterval_Pdt.+mu_cumulo+nu_cumulo+rho_cumulo+nu_per_rho_cumu lo+Accum._Yield+emission_O2+emission_CO2+consum_O2+interval _O2+generate_CO2; 0.780, 0.910, tmp+PL+interval_Pdt.+OD+nu_ cumulo+rho_cumulo+nu_per_rho_cumulo+Arg_conc._cumulo+rab_in tegral+Accum._Yield+emission_CO2+consum_O2+interval_O2+gene rate_CO2; 0.789, 0.910, tmp+PL+AN+interval_Pdt.+OD+nu_cumul o+rho_cumulo+nu_per_rho_cumulo+rab_integral+emission_CO2+co nsum_O2+generate_CO2; 0.771, 0.910, tmp+PL+PL_cumulo+AN+int erval_Pdt.+OD+RS+nu_cumulo+rho_cumulo+nu_per_rho_cumulo+Arg _conc._cumulo+Accum._Yield+emission_O2+emission_CO2+consum_ O2+generate_CO2; 0.792, 0.910, tmp+PL+interval_Pdt.+OD+nu_c umulo+rho_cumulo+nu_per_rho_cumulo+rab_integral+emission_CO 2+consum_O2+generate_CO2; 0.788, 0.910, tmp+PL+interval_Pdt. +mu_cumulo+nu_cumulo+rho_cumulo+nu_per_rho_cumulo+RQ_integr al+emission_O2+emission_CO2+consum_O2+generate_CO2; 0.776, 0.910, tmp+PL+PL_cumulo+interval_Pdt.+OD+mu_cumulo+nu_cumul o+rho_cumulo+nu_per_rho_cumulo+Arg_conc._cumulo+Accum._Yiel d+emission_O2+emission_CO2+consum_O2+generate_CO2; 0.776, 0. 910, tmp+PL+AN+interval_Pdt.+mu_cumulo+nu_cumulo+rho_cumulo +nu_per_rho_cumulo+cell_yield_cumulo+rab_integral+Accum._Yi eld+emission_CO2+consum_O2+interval_O2+generate_CO2; 0.780, 0.910, tmp+PL+PL_cumulo+interval_Pdt.+mu_cumulo+nu_cumulo+ rho_cumulo+nu_per_rho_cumulo+Arg_conc._cumulo+rab_integral+ Accum._Yield+emission_CO2+consum_O2+generate_CO2; 0.780, 0. 910, tmp+PL+AN+interval_Pdt.+OD+mu_cumulo+nu_cumulo+rho_cum ulo+nu_per_rho_cumulo+rab_integral+Accum._Yield+emission_CO 2+consum_O2+generate_CO2; 0.784, 0.910, tmp+PL+interval_Pdt. +mu_cumulo+nu_cumulo+rho_cumulo+nu_per_rho_cumulo+cell_yiel d_cumulo+rab_integral+emission_CO2+consum_O2+interval-O2+ge nerate_CO2; 0.780, 0.910, tmp+PL+interval_Pdt.+RS+mu_cumulo +nu_cumulo+rho_cumulo+nu_per_rho_cumulo+RQ_integral+Accum._ Yield+emission_O2+emission_CO2+consum_O2+generate_CO2; 0.78 0, 0.910, tmp+PL+AN+interval_Pdt.+OD+mu_cumulo+nu_cumulo+rh o_cumulo+nu_per_rho_cumulo+rab_integral+emission_CO2+consum _O2+interval_O2+generate_CO2; 0.792, 0.910, tmp+PL_cumulo+i nterval_Pdt.+mu_cumulo+nu_cumulo+rho_cumulo+nu_per_rho_cumu lo+rab_integral+emission_CO2+consum_O2+generate_CO2; 0.780, 0.910, tmp+pH+PL+interval_Pdt.+mu_cumulo+nu_cumulo+rho_cum ulo+nu_per_rho_cumulo+rab_integral+RQ_integral+Accum._Yield +emission_CO2+consum_O2+generate_CO2; 0.771, 0.910, tmp+PL+ PL_cumulo+AN+interval _Pdt.+OD+mu_cumulo+nu_cumulo+rho_cumul o+nu_per_rho_cumulo+Arg_conc._cumulo+rab_integral+Accum._Yi eld+emission_CO2+consum_O2+generate_CO2; 0.780, 0.910, tmp+ PL+AN+interval_Pdt.+mu_cumulo+nu_cumulo+rho_cumulo+nu_per_r ho_cumulo+Arg_conc._cumulo+rab_integral+emission_CO2+consum _O2+interval_O2+generate_CO2; 0.771, 0.910, tmp+PL+PL_cumul o+AN+interval_Pdt.+OD+RS+nu_cumulo+rho_cumulo+nu_per_rho_cu mulo+Arg_conc._cumulo+rab_integral+Accum._Yield+emission_CO 2+consum_O2+generate_CO2; 0.780, 0.910, tmp+PL+PL_cumulo+in terval_Pdt.+mu_cumulo+nu_cumulo+rho_cumulo+nu_per_rho_cumul o+rab_integral+RQ_integral+Accum._Yield+emission_CO2+consum _O2+generate_CO2; 0.784, 0.910, tmp+PL+interval_Pdt.+mu_cum ulo+nu_cumulo+rho_cumulo+nu_per_rho_cumulo+rab_integral+Acc um._Yield+emission_CO2+consum_O2+interval_O2+generate_CO2; 0.784, 0.910, tmp+PL+interval_Pdt.+OD+mu_cumulo+nu_cumulo+r ho_cumulo+nu_per_rho_cumulo+rab_integral+RQ_integral+emissi on_CO2+consum_O2+generate_CO2; 0.784, 0.910, tmp+PL+PL_cumu lo+AN+interval_Pdt.+mu_cumulo+nu_cumulo+rho_cumulo+nu_per_r ho_cumulo+Accum._Yield+emission_CO2+consum_O2+generate_CO2; 0.784, 0.910, tmp+PL+AN+interval_Pdt.+mu_cumulo+nu_cumulo+ rho_cumulo+nu_per_rho_cumulo+Arg_conc._cumulo+rab_integral+ emission_CO2+consum_O2+generate_CO2; 0.775, 0.910, tmp+PL+P L_cumulo+AN+interval_Pdt.+mu_cumulo+nu_cumulo+rho_cumulo+nu _per_rho_cumulo+rab_integral+RQ_integral+Accum._Yield+emiss ion_CO2+consum_O2+generate_CO2; 0.792, 0.910, tmp+PL+interv al_Pdt.+mu_cumulo+nu_cumulo+rho_cumulo+nu_per_rho_cumulo+Ac cum._Yield+emission_CO2+consum_O2+generate_CO2; 0.784, 0.91 0, tmp+PL+AN+interval_Pdt.+mu_cumulo+nu_cumulo+rho_cumulo+n u_per_rho_cumulo+Arg_conc._cumulo+emission_CO2+consum_O2+in terval_O2+generate_CO2; 0.784, 0.910, tmp+PL+PL_cumulo+inte rval_Pdt.+mu_cumulo+nu_cumulo+rho_cumulo+nu_per_rho_cumulo+ cell_yield_cumulo+rab_integral+emission_CO2+consum_O2+gener ate_CO2; 0.775, 0.910, tmp+PL+PL_cumulo+AN+interval_Pdt.+mu _cumulo+nu_cumulo+rho_cumulo+nu_per_rho_cumulo+rab_integral +Accum._Yield+emission_O2+emission_CO2+consum_O2+generate_C 02; 0.775, 0.910, tmp+PL+AN+interval_Pdt.+mu_cumulo+nu_cumu lo+rho_cumulo+nu_per_rho_cumulo+cell_yield_cumulo+rab_integ ral+RQ_integral+Accum._Yield+emission_CO2+consum_O2+generat e_CO2; 0.784, 0.910, tmp+PL+interval_Pdt.+mu_cumulo+nu_cumu lo+rho_cumulo+nu_per_rho_cumulo+Arg_conc._cumulo+rab_integr al+Accum._Yield+emission_CO2+consum_O2+generate_CO2; 0.784, 0.910, tmp+PL+OD+RS+nu_cumulo+rho_cumulo+nu_per_rho_cumulo +Arg_conc._cumulo+rab_integral+Accum._Yield+emission_CO2+co nsum_O2+generate_CO2; 0.780, 0.909, tmp+PL+AN+interval_Pdt. +OD+mu_cumulo+nu_cumulo+rho_cumulo+nu_per_rho_cumulo+Arg_co nc._cumulo+Accum._Yield+emission_CO2+consum_O2+generate_CO 2; 0.780, 0.909, tmp+PL+AN+interval_Pdt.+RS+mu_cumulo+nu_cu mulo+rho_cumulo+nu_per_rho_cumulo+cell_yield_cumulo+rab_int egral+emission_CO2+consum_O2+generate_CO2; 0.775, 0.909, tm p+PL+PL_cumulo+AN+interval_Pdt.+RS+mu_cumulo+nu_cumulo+rho_ cumulo+nu_per_rho_cumulo+rab_integral+Accum._Yield+emission _CO2+consum_O2+generate_CO2; 0.784, 0.909, tmp+PL+AN+interv al_Pdt.+mu_cumulo+nu_cumulo+rho_cumulo+nu_per_rho_cumulo+Ac cum._Yield+emission_O2+emission_CO2+consum_O2+generate_CO2; 0.771, 0.909, tmp+PL+PL_cumulo+interval_Pdt.+OD+RS+nu_cumu lo+rho_cumulo+nu_per_rho_cumulo+Arg_conc._cumulo+RQ_integra l+Accum._Yield+emission_O2+emission_CO2+consum_O2+generate_ CO2; 0.771, 0.909, tmp+PL+PL_cumulo+AN+interval_Pdt.+mu_cum ulo+nu_cumulo+rho_cumulo+nu_per_rho_cumulo+Arg_conc._cumulo +rab_integral+Accum._Yield+emission_CO2+consum_O2+interval-O2+generate_CO2; 0.792, 0.909, tmp+PL+OD+nu_cumulo+rho_cumu lo+nu_per_rho_cumulo+Arg_conc._cumulo+rab_integral+emission _CO2+consum_O2+generate_CO2; 0.780, 0.909, tmp+PL+PL_cumulo +AN+interval_Pdt.+mu_cumulo+nu_cumulo+rho_cumulo+nu_per_rho _cumulo+cell_yield_cumulo+rab_integral+emission_CO2+consum_ O2+generate_CO2; 0.771, 0.909, tmp+PL+PL_cumulo+AN+interval _Pdt.+RS+mu_cumulo+nu_cumulo+rho_cumulo+nu_per_rho_cumulo+A rg_conc._cumulo+rab_integral+Accum._Yield+emission_CO2+cons um_O2+generate_CO2; 0.784, 0.909, tmp+pH+PL+interval_Pdt.+m u_cumulo+nu_cumulo+rho_cumulo+nu_per_rho_cumulo+rab_integra l+RQ_integral+emission_CO2+consum_O2+generate_CO2; 0.775, 0. 909, tmp+PL+PL_cumulo+AN+interval_Pdt.+mu_cumulo+nu_cumulo+ rho_cumulo+nu_per_rho_cumulo+rab_integral+emission_O2+emiss ion_CO2+consum_O2+interval_O2+generate_CO2; 0.788, 0.909, t mp+PL+AN+interval_Pdt.+mu_cumulo+nu_cumulo+rho_cumulo+nu_pe r_rho_cumulo+emission_O2+emission_CO2+consum_O2+generate_CO 2; 0.780, 0.909, tmp+PL+PL_cumulo+OD+RS+nu_cumulo+rho_cumul o+nu_per_rho_cumulo+Arg_conc._cumulo+Accum._Yield+emission_ O2+emission_CO2+consum_O2+generate_CO2; 0.770, 0.909, tmp+P L+PL_cumulo+AN+interval_Pdt.+OD+nu_cumulo+rho_cumulo+nu_per _rho_cumulo+Arg_conc._cumulo+RQ_integral+Accum._Yield+emiss ion_O2+emission_CO2+consum_O2+generate_CO2; 0.770, 0.909, t mp+PL+PL_cumulo+AN+interval_Pdt.+OD+nu_cumulo+rho_cumulo+nu _per_rho_cumulo+Arg_conc._cumulo+rab_integral+Accum._Yield+ emission_CO2+consum_O2+interval_O2+generate_CO2; 0.779, 0.9 09, tmp+PL+AN+interval_Pdt.+mu_cumulo+nu_cumulo+rho_cumulo+ nu_per_rho_cumulo+Arg_conc._cumulo+cell_yield_cumulo+rab_in tegral+emission_CO2+consum_O2+generate_CO2; 0.792, 0.909, t mp+PL_cumulo+interval_Pdt.+mu_cumulo+nu_cumulo+rho_cumulo+n u_per_rho_cumulo+cell_yield_cumulo+emission_CO2+consum_O2+g enerate_CO2; 0.784, 0.909, tmp+PL_cumulo+interval_Pdt.+mu_c umulo+nu_cumulo+rho_cumulo+nu_per_rho_cumulo+rab_integral+R Q_integral+Accum._Yield+emission_CO2+consum_O2+generate_CO 2; 0.775, 0.909, tmp+PL+PL_cumulo+interval_Pdt.+mu_cumulo+n u_cumulo+rho_cumulo+nu_per_rho_cumulo+Arg_conc._cumulo+RQ_i ntegral+Accum._Yield+emission_O2+emission_CO2+consum_O2+gen erate_CO2; 0.775, 0.909, tmp+PL+PL_cumulo+AN+interval_Pdt.+ mu_cumulo+nu_cumulo+rho_cumulo+nu_per_rho_cumulo+Arg_conc._ cumulo+Accum._Yield+emission_O2+emission_CO2+consum_O2+gene rate_CO2; 0.784, 0.909, tmp+PL+AN+interval_Pdt.+mu_cumulo+n u_cumulo+rho_cumulo+nu_per_rho_cumulo+rab_integral+RQ_integ ral+emission_CO2+consum_O2+generate_CO2; 0.784, 0.909, tmp+ PL+PL_cumulo+interval_Pdt.+mu_cumulo+nu_cumulo+rho_cumulo+n u_per_rho_cumulo+rab_integral+emission_CO2+consum_O2+interv al_O2+generate_CO2; 0.775, 0.909, tmp+PL+interval_Pdt.+mu_c umulo+nu_cumulo+rho_cumulo+nu_per_rho_cumulo+Arg_conc._cumu lo+cell_yield_cumulo+rab_integral+RQ_integral+Accum._Yield+ emission_CO2+consum_O2+generate_CO2; 0.784, 0.909, tmp+PL+i nterval_Pdt.+OD+mu_cumulo+nu_cumulo+rho_cumulo+nu_per_rho_c umulo+Arg_conc._cumulo+rab_integral+emission_CO2+consum_O2+ generate_CO2; 0.779, 0.909, tmp+PL+PL_cumulo+AN+interval_Pd t.+mu_cumulo+nu_cumulo+rho_cumulo+nu_per_rho_cumulo+Arg_con c._cumulo+rab_integral+emission_CO2+consum_O2+generate_CO2; 0.791, 0.909, tmp+PL+OD+nu_cumulo+rho_cumulo+nu_per_rho_cu mulo+Arg_conc._cumulo+emission_O2+emission_CO2+consum_O2+ge nerate_CO2; 0.779, 0.909, tmp+PL+AN+interval_Pdt.+mu_cumulo +nu_cumulo+rho_cumulo+nu_per_rho_cumulo+cell_yield_cumulo+e mission_O2+emission_CO2+consum_O2+interval_O2+generate_CO2 ; 0.779, 0.909, tmp+PL+interval_Pdt.+RS+mu_cumulo+nu_cumulo+ rho_cumulo+nu_per_rho_cumulo+cell_yield_cumulo+rab_integral +Accum._Yield+emission_CO2+consum_O2+generate_CO2; 0.775, 0. 909, tmp+PL+interval_Pdt.+RS+mu_cumulo+nu_cumulo+rho_cumulo +nu_per_rho_cumulo+Arg_conc._cumulo+rab_integral+RQ_integra l+Accum._Yield+emission_CO2+consum_O2+generate_CO2; 0.775, 0.909, tmp+PL+interval_Pdt.+mu_cumulo+nu_cumulo+rho_cumulo+ nu_per_rho_cumulo+cell_yield_cumulo+RQ_integral+Accum._Yiel d+emission_O2+emission_CO2+consum_O2+interval_O2+generate_C 02; 0.788, 0.909, tmp+PL+interval_Pdt.+OD+nu_cumulo+rho_cum ulo+nu_per_rho_cumulo+Arg_conc._cumulo+emission_O2+emission _CO2+consum_O2+generate_CO2; 0.783, 0.909, tmp+PL_cumulo+in terval_Pdt.+mu_cumulo+nu_cumulo+rho_cumulo+nu_per_rho_cumul o+cell_yield_cumulo+rab_integral+Accum._Yield+emission_CO2+ consum_O2+generate_CO2; 0.775, 0.909, tmp+PL+AN+interval_Pd t.+mu_cumulo+nu_cumulo+rho_cumulo+nu_per_rho_cumulo+Arg_con c._cumulo+cell_yield_cumulo+rab_integral+Accum._Yield+emiss ion_CO2+consum_O2+generate_CO2; 0.779, 0.909, tmp+PL+PL_cum ulo+AN+interval_Pdt.+RS+mu_cumulo+nu_cumulo+rho_cumulo+nu_p er_rho_cumulo+rab_integral+emission_CO2+consum_O2+generate_ CO2; 0.775, 0.909, tmp+PL+PL_cumulo+AN+interval_Pdt.+mu_cum ulo+nu_cumulo+rho_cumulo+nu_per_rho_cumulo+cell_yield_cumul o+rab_integral+Accum._Yield+emission_CO2+consum_O2+generate _CO2; 0.783, 0.909, tmp+PL+interval_Pdt.+mu_cumulo+nu_cumul o+rho_cumulo+nu_per_rho_cumulo+cell_yield_cumulo+RQ_integra l+emission_O2+emission_CO2+consum_O2+generate_CO2; 0.779, 0. 909, tmp+PL+PL_cumulo+interval_Pdt.+mu_cumulo+nu_cumulo+rho _cumulo+nu_per_rho_cumulo+Arg_conc._cumulo+Accum._Yield+emi ssion_O2+emission_CO2+consum_O2+generate_CO2; 0.775, 0.909, tmp+PL+AN+interval_Pdt.+mu_cumulo+nu_cumulo+rho_cumulo+nu_ per_rho_cumulo+rab_integral+RQ_integral+Accum._Yield+emissi on_CO2+consum_O2+interval_O2+generate_CO2; 0.783, 0.909, tm p+PL+interval_Pdt.+mu_cumulo+nu_cumulo+rho_cumulo+nu_per_rh o_cumulo+Arg_conc._cumulo+rab_integral+RQ_integral+emission _CO2+consum_O2+generate_CO2; 0.783, 0.909, tmp+PL+AN+interv al_Pdt.+mu_cumulo+nu_cumulo+rho_cumulo+nu_per_rho_cumulo+ce ll_yield_cumulo+emission_O2+emission_CO2+consum_O2+generate _CO2; 0.775, 0.909, tmp+PL+interval_Pdt.+mu_cumulo+nu_cumul o+rho_cumulo+nu_per_rho_cumulo+Arg_conc._cumulo+cell_yield_ cumulo+RQ_integral+Accum._Yield+emission_O2+emission_CO2+co nsum_O2+generate_CO2; 0.795, 0.909, tmp+interval_Pdt.+mu_cu mulo+nu_cumulo+rho_cumulo+nu_per_rho_cumulo+rab_integral+em ission_CO2+consum_O2+generate_CO2; 0.783, 0.909, tmp+PL+int erval_Pdt.+RS+mu_cumulo+nu_cumulo+rho_cumulo+nu_per_rho_cum ulo+Arg_conc._cumulo+rab_integral+emission_CO2+consum-O2+ge nerate_CO2; 0.787, 0.909, tmp+PL+interval_Pdt.+OD+nu_cumulo +rho_cumulo+nu_per_rho_cumulo+rab_integral+RQ_integral+emis sion_CO2+consum_O2+generate_CO2; 0.791, 0.909, tmp+PL_cumul o+interval_Pdt.+OD+mu_cumulo+nu_cumulo+rho_cumulo+nu_per_rh o_cumulo+emission_CO2+consum_O2+generate_CO2; 0.787, 0.909, tmp+PL+AN+interval_Pdt.+mu_cumulo+rho_cumulo+rab_integral+ Accum._Yield+emission_CO2+consum_O2+interval_O2+generate_CO 2; 0.774, 0.909, tmp+PL+PL_cumulo+AN+interval_Pdt.+mu_cumul o+nu_cumulo+rho_cumulo+nu_per_rho_cumulo+RQ_integral+Accum. _Yield+emission_O2+emission_CO2+consum_O2+generate_CO2; 0.7 70, 0.909, tmp+PL+PL_cumulo+interval_Pdt.+mu_cumulo+nu_cumu lo+rho_cumulo+nu_per_rho_cumulo+Arg_conc._cumulo+rab_integr al+RQ_integral+Accum._Yield+emission_O2+emission_CO2+consum _O2+generate_CO2; 0.774, 0.909, tmp+PL+PL_cumulo+interval_P dt.+OD+nu_cumulo+rho_cumulo+nu_per_rho_cumulo+Arg_conc._cum ulo+rab_integral+Accum._Yield+emission_CO2+consum_O2+interv al_O2+generate_CO2; 0.770, 0.909, tmp+PL+PL_cumulo+interval _Pdt.+OD+nu_cumulo+rho_cumulo+nu_per_rho_cumulo+Arg_conc._c umulo+rab_integral+Accum._Yield+emission_O2+emission_CO2+co nsum_O2+interval_O2+generate_CO2; 0.779, 0.909, tmp+PL+AN+i nterval_Pdt.+OD+nu_cumulo+rho_cumulo+nu_per_rho_cumulo+Arg_ conc._cumulo+Accum._Yield+emission_O2+emission_CO2+consum_O 2+generate_CO2; 0.774, 0.909, tmp+PL+PL_cumulo+interval_Pdt. +OD+mu_cumulo+nu_cumulo+rho_cumulo+nu_per_rho_cumulo+Arg_co nc._cumulo+rab_integral+Accum._Yield+emission_CO2+consum_O2 +generate_CO2; 0.774, 0.909, tmp+PL+interval_Pdt.+OD+mu_cum ulo+nu_cumulo+rho_cumulo+nu_per_rho_cumulo+Arg_conc._cumulo +rab_integral+RQ_integral+Accum._Yield+emission_CO2+consum_ O2+generate_CO2; 0.779, 0.909, tmp+PL+AN+interval_Pdt.+mu_c umulo+nu_cumulo+rho_cumulo+nu_per_rho_cumulo+cell_yield_cum ulo+Accum._Yield+emission_O2+emission_CO2+consum_O2+generat e_CO2; 0.783, 0.909, tmp+PL+AN+interval_Pdt.+mu_cumulo+nu_c umulo+rho_cumulo+nu_per_rho_cumulo+cell_yield_cumulo+emissi on_CO2+consum_O2+interval_O2+generate_CO2; 0.787, 0.909, tm p+pH+PL+AN+interval_Pdt.+mu_cumulo+nu_cumulo+rho_cumulo+nu_ per_rho_cumulo+emission_CO2+consum_O2+generate_CO2; 0.779, 0.909, tmp+PL+AN+interval_Pdt.+RS+mu_cumulo+nu_cumulo+rho_c umulo+nu_per_rho_cumulo+rab_integral+emission_CO2+consum_O2 +interval_O2+generate_CO2; 0.783, 0.909, tmp+PL+interval_Pd t.+mu_cumulo+nu_cumulo+rho_cumulo+nu_per_rho_cumulo+cell_yi eld_cumulo+emission_O2+emission_CO2+consum_O2+interval_O2+g enerate_CO2; 0.783, 0.909, tmp+PL+PL_cumulo+interval_Pdt.+m u_cumulo+nu_cumulo+rho_cumulo+nu_per_rho_cumulo+Arg_conc._c umulo+rab_integral+emission_CO2+consum_O2+generate_CO2; 0.7 83, 0.909, tmp+PL+OD+RS+nu_cumulo+rho_cumulo+nu_per_rho_cum ulo+Arg_conc._cumulo+Accum._Yield+emission_O2+emission_CO2+ consum_O2+generate_CO2; 0.779, 0.909, tmp+PL+AN+interval_Pd t.+mu_cumulo+nu_cumulo+rho_cumulo+nu_per_rho_cumulo+Arg_con c._cumulo+cell_yield_cumulo+Accum._Yield+emission_CO2+consu m_O2+generate_CO2; 0.774, 0.909, tmp+PL+AN+interval_Pdt.+RS +mu_cumulo+nu_cumulo+rho_cumulo+nu_per_rho_cumulo+rab_integ ral+RQ_integral+Accum._Yield+emission_CO2+consum_O2+generat e_CO2; 0.783, 0.909, tmp+pH+PL+AN+interval_Pdt.+mu_cumulo+n u_cumulo+rho_cumulo+nu_per_rho_cumulo+Accum._Yield+emission _CO2+consum_O2+generate_CO2; 0.779, 0.909, tmp+PL_cumulo+in terval_Pdt.+mu_cumulo+nu_cumulo+rho_cumulo+nu_per_rho_cumul o+cell_yield_cumulo+rab_integral+RQ_integral+Accum._Yield+e mission_CO2+consum_O2+generate_CO2; 0.783, 0.909, tmp+PL+in terval_Pdt.+OD+RS+mu_cumulo+nu_cumulo+rho_cumulo+nu_per_rho _cumulo+rab_integral+emission_CO2+consum_O2+generate_CO2; 0. 787, 0.909, tmp+PL+interval_Pdt.+RS+mu_cumulo+nu_cumulo+rho _cumulo+nu_per_rho_cumulo+emission_O2+emission_CO2+consum_O 2+generate_CO2; 0.779, 0.909, tmp+PL+AN+interval_Pdt.+RS+mu _cumulo+nu_cumulo+rho_cumulo+nu_per_rho_cumulo+Arg_conc._cu mulo+Accum._Yield+emission_CO2+consum_O2+generate_CO2; 0.77 4, 0.909, tmp+PL+PL_cumulo+interval_Pdt.+OD+RS+nu_cumulo+rh o_cumulo+nu_per_rho_cumulo+Arg_conc._cumulo+rab_integral+Ac cum._Yield+emission_CO2+consum_O2+generate_CO2; 0.769, 0.90 9, tmp+pH+PL+PL_cumulo+AN+interval_Pdt.+OD+nu_cumulo+rho_cu mulo+nu_per_rho_cumulo+Arg_conc._cumulo+Accum._Yield+emissi on_O2+emission_CO2+consum_O2+generate_CO2; 0.787, 0.909, tm p+PL+AN+interval_Pdt.+mu_cumulo+nu_cumulo+rho_cumulo+nu_per _rho_cumulo+RQ_integral+emission_CO2+consum_O2+generate_CO 2; 0.774, 0.909, tmp+PL+AN+interval_Pdt.+RS+mu_cumulo+nu_cu mulo+rho_cumulo+nu_per_rho_cumulo+cell_yield_cumulo+rab_int egral+Accum._Yield+emission_CO2+consum_O2+generate_CO2 0.7 78, 0.909, tmp+PL+interval_Pdt.+OD+mu_cumulo+nu_cumulo+rho_ cumulo+nu_per_rho_cumulo+cell_yield_cumulo+rab_integral+Acc um._Yield+emission_CO2+consum_O2+generate_CO2; 0.791, 0.909, tmp+interval_Pdt.+mu_cumulo+nu_cumulo+rho_cumulo+nu_per_rh o_cumulo+rab_integral+Accum._Yield+emission_CO2+consum_O2+g enerate_CO2; 0.774, 0.909, tmp+PL+interval_Pdt.+RS+mu_cumul o+nu_cumulo+rho_cumulo+nu_per_rho_cumulo+cell_yield_cumulo+ RQ_integral+Accum._Yield+emission_O2+emission_CO2+consum_O2 +generate_CO2; 0.778, 0.909, tmp+PL+AN+interval_Pdt.+mu_cum ulo+nu_cumulo+rho_cumulo+nu_per_rho_cumulo+Arg_conc._cumulo +Accum._Yield+emission_CO2+consum_O2+interval_O2+generate_C 02; 0.769, 0.909, tmp+pH+PL+PL_cumulo+interval_Pdt.+OD+nu_c umulo+rho_cumulo+nu_per_rho_cumulo+Arg_conc._cumulo+RQ_inte gral+Accum._Yield+emission_O2+emission_CO2+consum_O2+genera te_CO2; 0.783, 0.909, tmp+PL+PL_cumulo+interval_Pdt.+RS+mu_ cumulo+nu_cumulo+rho_cumulo+nu_per_rho_cumulo+rab_integral+ emission_CO2+consum_O2+generate_CO2; 0.778, 0.909, tmp+PL+i nterval_Pdt.+OD+nu_cumulo+rho_cumulo+nu_per_rho_cumulo+Arg_ conc._cumulo+Accum._Yield+emission_O2+emission_CO2+consum_O 2+interval_O2+generate_CO2; 0.774, 0.909, tmp+PL+interval_P dt.+OD+RS+mu_cumulo+nu_cumulo+rho_cumulo+nu_per_rho_cumulo+ Arg_conc._cumulo+rab_integral+Accum._Yield+emission_CO2+con sum_O2+generate_CO2; 0.769, 0.909, tmp+PL+PL_cumulo+AN+inte rval_Pdt.+mu_cumulo+nu_cumulo+rho_cumulo+nu_per_rho_cumulo+ Arg_conc._cumulo+rab_integral+Accum._Yield+emission_O2+emis sion_CO2+consum_O2+generate_CO2; 0.782, 0.909, tmp+PL+AN+in terval_Pdt.+OD+mu_cumulo+nu_cumulo+rho_cumulo+nu_per_rho_cu mulo+emission_CO2+consum_O2+interval_O2+generate_CO2; 0.782, 0.909, tmp+PL+PL_cumulo+interval_Pdt.+mu_cumulo+nu_cumulo+ rho_cumulo+nu_per_rho_cumulo+rab_integral+emission_O2+emiss ion_CO2+consum_O2+generate_CO2; 0.794, 0.909, tmp+PL+AN+int erval_Pdt.+mu_cumulo+rho_cumulo+nu_per_rho_cumulo+emission_ CO2+consum_O2+generate_CO2; 0.774, 0.909, tmp+PL+PL_cumulo+ interval_Pdt.+mu_cumulo+nu_cumulo+rho_cumulo+nu_per_rho_cum ulo+cell_yield_cumulo+RQ_integral+Accum._Yield+emission_O2+ emission_CO2+consum_O2+generate_CO2; 0.787, 0.909, tmp+PL+i nterval_Pdt.+mu_cumulo+nu_cumulo+rho_cumulo+nu_per_rho_cumu lo+rab_integral+emission_O2+emission_CO2+consum_O2+generate _CO2; 0.782, 0.909, tmp+PL+AN+interval_Pdt.+RS+mu_cumulo+nu _cumulo+rho_cumulo+nu_per_rho_cumulo+emission_CO2+consum_O2 +interval_O2+generate_CO2; 0.778, 0.909, tmp+PL+AN+interval _Pdt.+OD+mu_cumulo+nu_cumulo+rho_cumulo+nu_per_rho_cumulo+c ell_yield_cumulo+rab_integral+emission_CO2+consum_O2+genera te_CO2; 0.787, 0.909, tmp+PL_cumulo+interval_Pdt.+mu_cumulo +nu_cumulo+rho_cumulo+nu_per_rho_cumulo+rab_integral+RQ_int egral+emission_CO2+consum_O2+generate_CO2; 0.774, 0.909, tm p+PL+interval_Pdt.+OD+mu_cumulo+nu_cumulo+rho_cumulo+nu_per _rho_cumulo+cell_yield_cumulo+RQ_integral+Accum._Yield+emis sion_O2+emission_CO2+consum_O2+generate_CO2; 0.782, 0.909, tmp+PL+AN+interval_Pdt.+OD+nu_cumulo+rho_cumulo+nu_per_rho_ cumulo+Arg_conc._cumulo+rab_integral+emission_CO2+consum_O2 +generate_CO2; 0.790, 0.909, tmp+PL+interval_Pdt.+OD+nu_cum ulo+rho_cumulo+nu_per_rho_cumulo+emission_O2+emission_CO2+c onsum_O2+generate_CO2; 0.778, 0.909, tmp+PL+interval_Pdt.+O D+RS+nu_cumulo+rho_cumulo+nu_per_rho_cumulo+Arg_conc._cumul o+Accum._Yield+emission_O2+emission_CO2+consum_O2+generate-CO2; 0.782, 0.909, tmp+PL+interval_Pdt.+OD+mu_cumulo+nu_cum ulo+rho_cumulo+nu_per_rho_cumulo+Accum._Yield+emission_O2+e mission_CO2+consum_O2+generate_CO2; 0.782, 0.909, tmp+pH+PL +interval_Pdt.+mu_cumulo+nu_cumulo+rho_cumulo+nu_per_rho_cu mulo+rab_integral+Accum._Yield+emission_CO2+consum_O2+gener ate_CO2; 0.774, 0.909, tmp+PL+AN+interval_Pdt.+OD+mu_cumulo +nu_cumulo+rho_cumulo+nu_per_rho_cumulo+Arg_conc._cumulo+ra b_integral+Accum._Yield+emission_CO2+consum_O2+generate_CO 2; 0.778, 0.909, tmp+PL+PL_cumulo+interval_Pdt.+mu_cumulo+n u_cumulo+rho_cumulo+nu_per_rho_cumulo+rab_integral+Accum._Y ield+emission_O2+emission_CO2+consum_O2+generate_CO2; 0.778, 0.909, tmp+PL+AN+interval_Pdt.+mu_cumulo+nu_cumulo+rho_cum ulo+nu_per_rho_cumulo+rab_integral+RQ_integral+emission_CO2 +consum_O2+interval_O2+generate_CO2; 0.764, 0.909, tmp+PL+P L_cumulo+AN+interval_Pdt.+mu_cumulo+nu_cumulo+rho_cumulo+nu _per_rho_cumulo+Arg_conc._cumulo+rab_integral+Accum._Yield+ emission_O2+emission_CO2+consum_O2+interval_O2+generate_CO 2; 0.782, 0.909, tmp+PL_cumulo+interval_Pdt.+RS+mu_cumulo+n u_cumulo+rho_cumulo+nu_per_rho_cumulo+cell_yield_cumulo+rab _integral+emission_CO2+consum_O2+generate_CO2; 0.786, 0.909, tmp+PL_cumulo+interval_Pdt.+OD+mu_cumulo+nu_cumulo+rho_cum ulo+nu_per_rho_cumulo+rab_integral+emission_CO2+consum_O2+g enerate_CO2; 0.778, 0.909, tmp+PL+AN+interval_Pdt.+OD+mu_cu mulo+nu_cumulo+rho_cumulo+nu_per_rho_cumulo+emission_O2+emi ssion_CO2+consum_O2+interval_O2+generate_CO2; 0.769, 0.909, tmp+pH+PL+PL_cumulo+AN+interval_Pdt.+OD+nu_cumulo+rho_cumu lo+nu_per_rho_cumulo+Arg_conc._cumulo+rab_integral+Accum._Y ield+emission_CO2+consum_O2+generate_CO2; 0.778, 0.909, tmp +pH+PL+interval_Pdt.+OD+nu_cumulo+rho_cumulo+nu_per_rho_cum ulo+Arg_conc._cumulo+rab_integral+Accum._Yield+emission_CO2 +consum_O2+generate_CO2; 0.773, 0.909, tmp+PL+PL_cumulo+AN+ interval_Pdt.+mu_cumulo+nu_cumulo+rho_cumulo+nu_per_rho_cum ulo+Arg_conc._cumulo+rab_integral+emission_CO2+consum_O2+in terval_O2+generate_CO2; 0.790, 0.909, tmp+PL_cumulo+interva l_Pdt.+mu_cumulo+nu_cumulo+rho_cumulo+nu_per_rho_cumulo+Acc um._Yield+emission_CO2+consum_O2+generate_CO2; 0.782, 0.909, tmp+PL+PL_cumulo+interval_Pdt.+mu_cumulo+nu_cumulo+rho_cum ulo+nu_per_rho_cumulo+RQ_integral+emission_O2+emission_CO2+ consum_O2+generate_CO2; 0.790, 0.909, tmp+PL+AN+interval_Pd t.+mu_cumulo+rho_cumulo+nu_per_rho_cumulo+emission_CO2+cons um_O2+interval_O2+generate_CO2; 0.782, 0.909, tmp+pH+PL+int erval_Pdt.+mu_cumulo+nu_cumulo+rho_cumulo+nu_per_rho_cumulo +cell_yield_cumulo+rab_integral+emission_CO2+consum_O2+gene rate_CO2; 0.782, 0.909, tmp+PL+interval_Pdt.+OD+mu_cumulo+n u_cumulo+rho_cumulo+nu_per_rho_cumulo+rab_integral+emission _CO2+consum_O2+interval_O2+generate_CO2; 0.773, 0.909, tmp+ PL+AN+interval_Pdt.+OD+RS+nu_cumulo+rho_cumulo+nu_per_rho_c umulo+Arg_conc._cumulo+rab_integral+Accum._Yield+emission_C O2+consum_O2+generate_CO2; 0.786, 0.909, tmp+PL+interval_Pd t.+OD+nu_cumulo+rho_cumulo+nu_per_rho_cumulo+RQ_integral+em ission_O2+emission_CO2+consum_O2+generate_CO2; 0.773, 0.909, tmp+PL+AN+interval_Pdt.+OD+mu_cumulo+nu_cumulo+rho_cumulo+ nu_per_rho_cumulo+rab_integral+Accum._Yield+emission_CO2+co nsum_O2+interval_O2+generate_CO2; 0.782, 0.909, tmp+PL_cumu lo+interval_Pdt.+mu_cumulo+nu_cumulo+rho_cumulo+nu_per_rho_ cumulo+cell_yield_cumulo+rab_integral+RQ_integral+emission_ CO2+consum_O2+generate_CO2; 0.782, 0.909, tmp+PL+interval_P dt.+mu_cumulo+nu_cumulo+rho_cumulo+nu_per_rho_cumulo+RQ_int egral+emission_O2+emission_CO2+consum_O2+interval_O2+genera te_CO2; 0.790, 0.909, tmp+pH+interval_Pdt.+mu_cumulo+nu_cum ulo+rho_cumulo+nu_per_rho_cumulo+rab_integral+emission_CO2+ consum_O2+generate_CO2; 0.773, 0.909, tmp+PL+interval_Pdt.+ mu_cumulo+nu_cumulo+rho_cumulo+nu_per_rho_cumulo+cell_yield _cumulo+rab_integral+RQ_integral+Accum._Yield+emission_O2+e mission_CO2+consum_O2+generate_CO2; 0.782, 0.909, tmp+PL+in terval_Pdt.+mu_cumulo+nu_cumulo+rho_cumulo+nu_per_rho_cumul o+Arg_conc._cumulo+cell_yield_cumulo+emission_O2+emission_C O2+consum_O2+generate_CO2; 0.773, 0.909, tmp+PL+interval_Pd t.+RS+mu_cumulo+nu_cumulo+rho_cumulo+nu_per_rho_cumulo+Arg_ conc._cumulo+cell_yield_cumulo+rab_integral+Accum._Yield+em ission_CO2+consum_O2+generate_CO2; 0.778, 0.909, tmp+PL+AN+ interval_Pdt.+mu_cumulo+nu_cumulo+rho_cumulo+nu_per_rho_cum ulo+Arg_conc._cumulo+rab_integral+Accum._Yield+emission_CO2 +consum_O2+generate_CO2; 0.773, 0.909, tmp+PL+PL_cumulo+AN+ interval_Pdt.+mu_cumulo+nu_cumulo+rho_cumulo+nu_per_rho_cum ulo+Arg_conc._cumulo+emission_O2+emission_CO2+consum_O2+int erval_O2+generate_CO2; 0.782, 0.909, tmp+PL+PL_cumulo+AN+in terval_Pdt.+mu_cumulo+nu_cumulo+rho_cumulo+nu_per_rho_cumul o+emission_CO2+consum_O2+interval_O2+generate_CO2; 0.778, 0. 909, tmp+PL+interval_Pdt.+OD+mu_cumulo+nu_cumulo+rho_cumulo +nu_per_rho_cumulo+Arg_conc._cumulo+Accum._Yield+emission_O 2+emission_CO2+consum_O2+generate_CO2; 0.778, 0.909, tmp+PL +interval_Pdt.+RS+mu_cumulo+nu_cumulo+rho_cumulo+nu_per_rho _cumulo+Arg_conc._cumulo+rab_integral+Accum._Yield+emission _CO2+consum_O2+generate_CO2; 0.769, 0.909, tmp+PL+PL_cumulo +AN+interval_Pdt.+OD+mu_cumulo+nu_cumulo+rho_cumulo+nu_per_ rho_cumulo+Arg_conc._cumulo+Accum._Yield+emission_O2+emissi on_CO2+consum_O2+generate_CO2; 0.773, 0.909, tmp+PL+AN+inte rval_Pdt.+mu_cumulo+nu_cumulo+rho_cumulo+nu_per_rho_cumulo+ cell_yield_cumulo+Accum._Yield+emission_O2+emission_CO2+con sum_O2+interval_O2+generate_CO2; 0.786, 0.909, tmp+PL+inter val_Pdt.+mu_cumulo+nu_cumulo+rho_cumulo+nu_per_rho_cumulo+A rg_conc._cumulo+emission_O2+emission_CO2+consum_O2+generate _CO2; 0.790, 0.909, tmp+PL+AN+interval_Pdt.+RS+mu_cumulo+rh o_cumulo+Accum._Yield+emission_CO2+consum_O2+generate_CO2; 0.786, 0.909, tmp+PL_cumulo+interval_Pdt.+RS+mu_cumulo+nu_c umulo+rho_cumulo+nu_per_rho_cumulo+rab_integral+emission_CO 2+consum_O2+generate_CO2; 0.782, 0.909, tmp+PL+interval_Pdt. +OD+nu_cumulo+rho_cumulo+nu_per_rho_cumulo+RQ_integral+Accu m._Yield+emission_O2+emission_CO2+consum_O2+generate_CO2; 0. 782, 0.909, tmp+PL+AN+interval_Pdt.+mu_cumulo+nu_cumulo+rho _cumulo+nu_per_rho_cumulo+RQ_integral+Accum._Yield+emission _CO2+consum_O2+generate_CO2; 0.773, 0.909, tmp+PL+interval_ Pdt.+OD+mu_cumulo+nu_cumulo+rho_cumulo+nu_per_rho_cumulo+ce ll_yield_cumulo+rab_integral+RQ_integral+Accum._Yield+emiss ion_CO2+consum_O2+generate_CO2; 0.782, 0.909, tmp+PL+PL_cum ulo+interval_Pdt.+mu_cumulo+nu_cumulo+rho_cumulo+nu_per_rho _cumulo+cell_yield_cumulo+emission_O2+emission_CO2+consum_O 2+generate_CO2; 0.782, 0.908, tmp+PL+PL_cumulo+interval_yie ld+interval_Pdt.+mu_cumulo+nu_cumulo+rho_cumulo+nu_per_rho_ cumulo+rab_integral+emission_CO2+consum_O2+generate_CO2; 0. 782, 0.908, tmp+PL+interval_Pdt.+mu_cumulo+nu_cumulo+rho_cu mulo+nu_per_rho_cumulo+Accum._Yield+emission_O2+emission_CO 2+consum_O2+interval_O2+generate_CO2; 0.782, 0.908, tmp+PL+ interval_Pdt.+OD+mu_cumulo+nu_cumulo+rho_cumulo+nu_per_rho_ cumulo+cell_yield_cumulo+emission_O2+emission_CO2+consum_O2 +generate_CO2; 0.782, 0.908, tmp+PL+interval_Pdt.+RS+mu_cum ulo+nu_cumulo+rho_cumulo+nu_per_rho_cumulo+cell_yield_cumul o+emission_O2+emission_CO2+consum_O2+generate_CO2; 0.786, 0. 908, tmp+PL_cumulo+interval_Pdt.+mu_cumulo+nu_cumulo+rho_cu mulo+nu_per_rho_cumulo+rab_integral+Accum._Yield+emission_C O2+consum_O2+generate_CO2; 0.777, 0.908, tmp+PL+PL_cumulo+A N+interval_Pdt.+mu_cumulo+nu_cumulo+rho_cumulo+nu_per_rho_c umulo+rab_integral+emission_O2+emission_CO2+consum_O2+gener ate_CO2; 0.782, 0.908, tmp+PL+AN+interval_Pdt.+mu_cumulo+nu _cumulo+rho_cumulo+nu_per_rho_cumulo+Arg_conc._cumulo+cell_ yield_cumulo+emission_CO2+consum_O2+generate_CO2; 0.773, 0. 908, tmp+PL+interval_Pdt.+OD+nu_cumulo+rho_cumulo+nu_per_rh o_cumulo+Arg_conc._cumulo+RQ_integral+Accum._Yield+emission _O2+emission_CO2+consum_O2+interval_O2+generate_CO2; 0.777, 0.908, tmp+pH+PL+PL_cumulo+OD+nu_cumulo+rho_cumulo+nu_per_ rho_cumulo+Arg_conc._cumulo+Accum._Yield+emission_O2+emissi on_CO2+consum_O2+generate_CO2; 0.777, 0.908, tmp+PL+interva l_Pdt.+mu_cumulo+nu_cumulo+rho_cumulo+nu_per_rho_cumulo+cel l_yield_cumulo+rab_integral+Accum._Yield+emission_CO2+consu m_O2+interval_O2+generate_CO2; 0.777, 0.908, tmp+PL+AN+inte rval_Pdt.+mu_cumulo+nu_cumulo+rho_cumulo+nu_per_rho_cumulo+ cell_yield_cumulo+Accum._Yield+emission_CO2+consum_O2+inter val_O2+generate_CO2; 0.773, 0.908, tmp+PL+PL_cumulo+interva l_Pdt.+mu_cumulo+nu_cumulo+rho_cumulo+nu_per_rho_cumulo+Arg _conc._cumulo+rab_integral+Accum._Yield+emission_O2+emissio n_CO2+consum_O2+generate_CO2; 0.781, 0.908, tmp+PL+AN+inter val_Pdt.+OD+mu_cumulo+nu_cumulo+rho_cumulo+nu_per_rho_cumul o+emission_O2+emission_CO2+consum_O2+generate_CO2; 0.768, 0. 908, tmp+PL+PL_cumulo+interval_Pdt.+OD+mu_cumulo+nu_cumulo+ rho_cumulo+nu_per_rho_cumulo+Arg_conc._cumulo+RQ_integral+A ccum._Yield+emission_O2+emission_CO2+consum_O2+generate_CO 2; 0.777, 0.908, tmp+PL+AN+interval_Pdt.+mu_cumulo+nu_cumul o+rho_cumulo+nu_per_rho_cumulo+Arg_conc._cumulo+emission_02 +emission_CO2+consum_O2+interval_O2+generate_CO2; 0.777, 0. 908, tmp+PL+PL_cumulo+interval_Pdt.+mu_cumulo+nu_cumulo+rho _cumulo+nu_per_rho_cumulo+cell_yield_cumulo+rab_integral+Ac cum._Yield+emission_CO2+consum_O2+generate_CO2; 0.777, 0.90 8, tmp+PL+interval_Pdt.+RS+mu_cumulo+nu_cumulo+rho_cumulo+n u_per_rho_cumulo+Arg_conc._cumulo+cell_yield_cumulo+rab_int egral+emission_CO2+consum_O2+generate_CO2; 0.763, 0.908, tm p+PL+PL_cumulo+AN+interval_Pdt.+OD+nu_cumulo+rho_cumulo+nu_ per_rho_cumulo+Arg_conc._cumulo+rab_integral+Accum._Yield+e mission_O2+emission_CO2+consum_O2+interval_O2+generate_CO2; 0.777, 0.908, tmp+PL+AN+interval_Pdt.+OD+mu_cumulo+nu_cumu lo+rho_cumulo+nu_per_rho_cumulo+Accum._Yield+emission_CO28+c onsum_O2+interval_O2+generate_CO2; 0.781, 0.908, tmp+PL+AN+ interval_Pdt.+OD+nu_cumulo+rho_cumulo+nu_per_rho_cumulo+rab integral+emission_CO2+consum_O2+interval_O2+generate_CO2; 0.773, 0.908, tmp+PL+AN+interval_Pdt.+RS+mu_cumulo+nu_cumul o+rho_cumulo+nu_per_rho_cumulo+rab_integral+Accum._Yield+em ission_CO2+consum_O2+interval_O2+generate_CO2; 0.773, 0.908, tmp+PL+interval_Pdt.+OD+RS+nu_cumulo+rho_cumulo+nu_per_rho _cumulo+Arg_conc._cumulo+RQ_integral+Accum._Yield+eniission_ O2+emission_CO2+consum_O2+generate_CO2; 0.768, 0.908, tmp+P L+PL_cumulo+interval_Pdt.+OD+RS+nu_cumulo+rho_cumulo+nu_per _rho_cumulo+Arg_conc._cumulo+Accum._Yield+emission_O2+emiss ion_CO2+consum_O2+interval_O2+generate_CO2; 0.773, 0.908, t mp+PL+AN+interval_Pdt.+OD+mu_cumulo+nu_cumulo+rho_cumulo+nu _per_rho_cumulo+cell_yield_cumulo+rab_integral+Accum._Yield +emission_CO2+consum_O2+generate_CO2; 0.777, 0.908, tmp+PL+ interval_Pdt.+RS+mu_cumulo+nu_cumulo+rho_cumulo+nu_per_rho_ cumulo+cell_yield_cumulo+rab_integral+RQ_integral+emission_ CO2+consum_O2+generate_CO2; 0.777, 0.908, tmp+PL+interval_P dt.+mu_cumulo+nu_cumulo+rho_cumulo+nu_per_rho_cumulo+Arg_co nc._cumulo+RQ_integral+Accum._Yield+emission_O2+emission_CO 2+consum_O2+generate_CO2; 0.772, 0.908, tmp+PL+interval_Pdt. +OD+nu_cumulo+rho_cumulo+nu_per_rho_cumulo+Arg_conc._cumulo +rab_integral+RQ_integral+Accum._Yield+emission_CO2+consum-O2+interval_O2+generate_CO2; 0.781, 0.908, tmp+PL+interval_ Pdt.+OD+mu_cumulo+nu_cumulo+rho_cumulo+nu_per_rho_cumulo+RQ _integral+emission_O2+emission_CO2+consum_O2+generate_CO2; 0.772, 0.908, tmp+pH+PL+PL_cumulo+AN+interval_Pdt.+mu_cumul o+nu_cumulo+rho_cumulo+nu_per_rho_cumulo+rab_integral+Accum. _Yield+emission_CO2+consum_O2+generate_CO2; 0.772, 0.908, t mp+PL+PL_cumulo+AN+interval_Pdt.+mu_cumulo+nu_cumulo+rho_cu mulo+nu_per_rho_cumulo+cell_yield_cumulo+emission_O2+emissi on_CO2+consum_O2+interval_O2+generate_CO2; 0.789, 0.908, tm p+PL+interval_Pdt.+mu_cumulo+nu_cumulo+rho_cumulo+nu_per_rh o_cumulo+emission_CO2+consum_O2+interval_O2+generate_CO2; 0. 789, 0.908, tmp+PL+AN+interval_Pdt.+mu_cumulo+rho_cumulo+nu _per_rho_cumulo+Accum._Yield+emission_CO2+consum_O2+generat e_CO2; 0.772, 0.908, tmp+PL+interval_Pdt.+RS+mu_cumulo+nu_c umulo+rho_cumulo+nu_per_rho_cumulo+cell_yield_cumulo+rab_in tegral+RQ_integral+Accum._Yield+emission_CO2+consum_O2+gene rate_CO2; 0.785, 0.908, tmp+interval_Pdt.+mu_cumulo+nu_cumu lo+rho_cumulo+nu_per_rho_cumulo+rab_integral+RQ_integral+Ac cum._Yield+emission_CO2+consum _O2+generate_CO2; 0.777, 0.90 8, tmp+PL+AN+interval_Pdt.+RS+mu_cumulo+nu_cumulo+rho_cumul o+nu_per_rho_cumulo+Accum._Yield+emission_CO2+consum_O2+int erval_O2+generate_CO2; 0.772, 0.908, tmp+PL+PL_cumulo+AN+in terval_Pdt.+mu_cumulo+nu_cumulo+rho_cumulo+nu_per_rho_cumul o+cell_yield_cumulo+rab_integral+emission_CO2+consum_O2+int erval_O2+generate_CO2; 0.777, 0.908, tmp+PL+PL_cumulo+AN+in terval_Pdt.+mu_cumulo+nu_cumulo+rho_cumulo+nu_per_rho_cumul o+rab_integral+RQ_integral+emission_CO2+consum_O2+generate_ CO2; 0.777, 0.908, tmp+PL+AN+interval_Pdt.+mu_cumulo+nu_cum ulo+rho_cumulo+nu_per_rho_cumulo+cell_yield_cumulo+rab_inte gral+RQ_integral+emission_CO2+consum_O2+generate_CO2; 0.781, 0.908, tmp+PL+interval_Pdt.+RS+mu_cumulo+nu_cumulo+rho_cum ulo+nu_per_rho_cumulo+rab_integral+emission_CO2+consum_O2+i nterval_O2+generate_CO2; 0.772, 0.908, tmp+PL+interval_Pdt. +OD+mu_cumulo+nu_cumulo+rho_cumulo+nu_per_rho_cumulo+Arg_co nc._cumulo+RQ_integral+Accum._Yield+eniission_O2+emission_Co 2+consum_O2+generate_CO2; 0.768, 0.908, tmp+PL+PL_cumulo+AN +interval_Pdt.+RS+mu_cumulo+nu_cumulo+rho_cumulo+nu_per_rho _cumulo+Accum._Yield+emission_O2+emission_CO2+consuni_O2+int erval_O2+generate_CO2; 0.781, 0.908, tmp+PL+interval_Pdt.+O D+mu_cumulo+nu_cumulo+rho_cumulo+nu_per_rho_cumulo+emission _O2+emission_CO2+consum_O2+interval_O2+generate_CO2; 0.772, 0.908, tmp+PL+AN+interval_Pdt.+mu_cumulo+nu_cumulo+rho_cum ulo+nu_per_rho_cumulo+Arg_conc._cumulo+cell_yield_cumulo+ra b_integral+emission_CO2+consum_O2+interval_O2+generate_CO2; 0.772, 0.908, tmp+PL+AN+interval_Pdt.+OD+mu_cumulo+nu_cumu lo+rho_cumulo+nu_per_rho_cumulo+rab_integral+RQ_integral+Ac cum._Yield+emission_CO2+consum_O2+generate_CO2; 0.781, 0.90 8, tmp+PL+AN+interval_Pdt.+RS+mu_cumulo+nu_cumulo+rho_cumul o+nu_per_rho_cumulo+Arg_conc._cumulo+emission_CO2+consum_O2 +generate_CO2; 0.777, 0.908, tmp+PL+PL_cumulo+interval_Pdt. +mu_cumulo+nu_cumulo+rho_cumulo+nu_per_rho_cumulo+rab_integ ral+Accum._Yield+emission_CO2+consum_O2+interval_O2+generat e_CO2; 0.772, 0.908, tmp+pH+PL+PL_cumulo+interval_Pdt.+OD+n u_cumulo+rho_cumulo+nu_per_rho_cumulo+Arg_conc._cumulo+rab__ integral+Accum._Yield+emission_CO2+consum_O2+generate_CO2; 0.781, 0.908, tmp+pH+PL+PL_cumulo+interval_Pdt.+mu_cumulo+n u_cumulo+rho_cumulo+nu_per_rho_cumulo+rab_integral+emission _CO2+consum_O2+generate_CO2; 0.789, 0.908, tmp+PL+AN+interv al_yield+interval_Pdt.+mu_cumulo+rho_cumulo+Accum._Yield+em ission_CO2+consum_O2+generate_CO2; 0.781, 0.908, tmp+PL+int erval_Pdt.+RS+mu_cumulo+nu_cumulo+rho_cumulo+nu_per_rho_cum ulo+Accum._Yield+emission_O2+emission_CO2+consum_O2+generat e_CO2; 0.785, 0.908, tmp+PL+AN+interval_Pdt.+OD+nu_cumulo+r ho_cumulo+nu_per_rho_cumulo+emission_O2+emission_CO2+consum _O2+generate_CO2; 0.789, 0.908, tmp+interval_Pdt.+mu_cumulo +nu_cumulo+rho_cumulo+nu_per_rho_cumulo+cell_yield_cumulo+r ab_integral+emission_CO2+consum_O2+generate_CO2; 0.768, 0.9 08, tmp+pH+PL+PL_cumulo+AN+interval_Pdt.+mu_cumulo+nu_cumul o+rho_cumulo+nu_per_rho_cumulo+Arg_conc._cumulo+rab_integra l+Accum._Yield+emission_CO2+consum_O2+generate_CO2; 0.777, 0.908, tmp+PL+PL_cumulo+interval_Pdt.+mu_cumulo+nu_cumulo+r ho_cumulo+nu_per_rho_cumulo+RQ_integral+emission_O2+emissio n_CO2+consum_O2+interval_O2+generate_CO2; 0.768, 0.908, tmp +PL+PL_cumulo+AN+interval_Pdt.+mu_cumulo+nu_cumulo+rho_cumu lo+nu_per_rho_cumulo+Arg_conc._cumulo+cell_yield_cumulo+rab _integral+Accum._Yield+emission_CO2+consum_O2+generate_CO2; 0.781, 0.908, tmp+PL+interval_yield+interval_Pdt.+mu_cumul o+nu_cumulo+rho_cumulo+nu_per_rho_cumulo+cell_yield_cumulo+ rab_integral+emission_CO2+consum_O2+generate_CO2; 0.781, 0. 908, tmp+pH+interval_Pdt.+mu_cumulo+nu_cumulo+rho_cumulo+nu _per_rho_cumulo+rab_integral+RQ_integral+Accum._Yield+emiss ion_CO2+consum_O2+generate_CO2; 0.768, 0.908, tmp+PL+PL_cum ulo+AN+interval_Pdt.+mu_cumulo+nu_cumulo+rho_cumulo+nu_per_ rho_cumulo+cell_yield_cumulo+Accum._Yield+emission_O2+emiss ion_CO2+consum_O2+interval_O2+generate_CO2; 0.772, 0.908, t mp+PL+interval_Pdt.+OD+RS+nu_cumulo+rho_cumulo+nu_per_rho_c umulo+Arg_conc._cumulo+rab_integral+RQ_integral+Accum._Yiel d+emission_CO2+consum_O2+generate_CO2; 0.763, 0.908, tmp+PL +PL_cumulo+interval_Pdt.+OD+nu_cumulo+rho_cumulo+nu_per_rho _cumulo+Arg_conc._cumulo+rab_integral+RQ_integral+Accum._Yi eld+emission_O2+emission_CO2+consum_O2+interval_O2+generate _CO2; 0.781, 0.908, tmp+PL+interval_yield+interval_Pdt.+mu_ cumulo+nu_cumulo+rho_cumulo+nu_per_rho_cumulo+rab_integral+ RQ_integral+emission_CO2+consum_O2+generate_CO2; 0.781, 0.9 08, tmp+pH+PL+AN+interval_Pdt.+mu_cumulo+nu_cumulo+rho_cumu lo+nu_per_rho_cumulo+rab_integral+emission_CO2+consum_O2+ge nerate_CO2; 0.789, 0.908, tmp+PL_cumulo+interval_Pdt.+RS+mu _cumulo+nu_cumulo+rho_cumulo+nu_per_rho_cumulo+emission_CO2 +consum_O2+generate_CO2; 0.767, 0.908, tmp+PL+PL_cumulo+int erval_Pdt.+mu_cumulo+nu_cumulo+rho_cumulo+nu_per_rho_cumulo +rab_integral+RQ_integral+Accum._Yield+emission_O2+emission _CO2+consum_O2+interval_O2+generate_CO2; 0.781, 0.908, tmp+ PL+AN+interval_Pdt.+OD+mu_cumulo+nu_cumulo+rho_cumulo+nu_pe r_rho_cumulo+cell_yield_cumulo+emission_CO2+consum_O2+gener ate_CO2; 0.777, 0.908, tmp+PL_cumulo+interval_Pdt.+RS+mu_cu mulo+nu_cumulo+rho_cumulo+nu_per_rho_cumulo+rab_integral+RQ _integral+Accum._Yield+emission_CO2+consum_O2+generate_CO2; 0.776, 0.908, tmp+PL+interval_Pdt.+mu_cumulo+nu_cumulo+rho _cumulo+nu_per_rho_cumulo+Arg_conc._cumulo+cell_yield_cumul o+Accum._Yield+emission_O2+emission_CO2+consum_O2+generate-CO2; 0.781, 0.908, tmp+pH+PL+OD+nu_cumulo+rho_cumulo+nu_per _rho_cumulo+Arg_conc._cumulo+rab_integral+Accum._Yield+emis sion_CO2+consum_O2+generate_CO2; 0.772, 0.908, tmp+PL+AN+in terval_Pdt.+OD+nu_cumulo+rho_cumulo+nu_per_rho_cumulo+Arg_c onc._cumulo+rab_integral+Accum._Yield+emission_CO2+consum_O 2+interval_O2+generate_CO2; 0.785, 0.908, tmp+PL+AN+interva l_Pdt.+mu_cumulo+rho_cumulo+Accum._Yield+emission_O2+emissi on_CO2+consum_O2+interval_O2+generate_CO2; 0.781, 0.908, tm p+PL+interval_Pdt.+mu_cumulo+nu_cumulo+rho_cumulo+nu_per_rh o_cumulo+rab_integral+Accum._Yield+emission_O2+emission_CO2 +consum_O2+generate_CO2; 0.776, 0.908, tmp+PL+PL_cumulo+int erval_Pdt.+RS+mu_cumulo+nu_cumulo+rho_cumulo+nu_per_rho_cum ulo+rab_integral+Accum._Yield+emission_CO2+consum_O2+genera te_CO2; 0.781, 0.908, tmp+PL_cumulo+interval_Pdt.+RS+mu_cum ulo+nu_cumulo+rho_cumulo+nu_per_rho_cumulo+rab_integral+RQ_ integral+emission_CO2+consum_O2+generate_CO2; 0.776, 0.908, tmp+pH+PL+AN+interval_Pdt.+mu_cumulo+nu_cumulo+rho_cumulo+ nu_per_rho_cumulo+rab_integral+Accum._Yield+emission_CO2+co nsum_O2+generate_CO2; 0.785, 0.908, tmp+pH+interval_Pdt.+mu _cumulo+nu_cumulo+rho_cumulo+nu_per_rho_cumulo+rab_integral +Accum._Yield+emission_CO2+consum_O2+generate_CO2; 0.776, 0. 908, tmp+PL+interval_Pdt.+mu_cumulo+nu_cumulo+rho_cumulo+nu _per_rho_cumulo+cell_yield_cumulo+Accum._Yield+emission_O2+ emission_CO2+consum_O2+interval_O2+generate_CO2; 0.776, 0.9 08, tmp+PL+interval_Pdt.+OD+mu_cumulo+nu_cumulo+rho_cumulo+ nu_per_rho_cumulo+cell_yield_cumulo+rab_integral+RQ_integra 1+emission_CO2+consum_O2+generate_CO2; 0.776, 0.908, tmp+PL +interval_yield+interval_Pdt.+mu_cumulo+nu_cumulo+rho_cumul o+nu_per_rho_cumulo+cell_yield_cumulo+rab_integral+RQ_integ ral+emission_CO2+consum_O2+generate_CO2; 0.767, 0.908, tmp+ PL+AN+interval_Pdt.+RS+mu_cumulo+nu_cumulo+rho_cumulo+nu_pe r_rho_cumulo+Arg_conc._cumulo+cell_yield_cumulo+rab_integra 1+Accum._Yield+emission_CO2+consum_O2+generate_CO2; 0.781, 0.908, tmp+PL+interval_Pdt.+mu_cumulo+nu_cumulo+rho_cumulo+ nu_per_rho_cumulo+Arg_conc._cumulo+rab_integral+emission_CO 2+consum_O2+interval_O2+generate_CO2; 0.789, 0.908, tmp+PL_ cumulo+AN+interval_Pdt.+mu_cumulo+nu_cumulo+rho_cumulo+nu_p er_rho_cumulo+emission_CO2+consum_O2+generate_CO2; 0.781, 0. 908, tmp+PL+interval_Pdt.+OD+nu_cumulo+rho_cumulo+nu_per_rh o_cumulo+Arg_conc._cumulo+rab_integral+emission_CO2+consum O2+interval_O2+generate_CO2; 0.781, 0.908, tmp+PL+interval_ Pdt.+OD+nu_cumulo+rho_cumulo+nu_per_rho_cumulo+rab_integral +RQ_integral+Accum._Yield+emission_CO2+consum_O2+generate_C O2; 0.789, 0.908, tmp+PL_cumulo+OD+nu_cumulo+rho_cumulo+nu_ per_rho_cumulo+Arg_conc._cumulo+rab_integral+emission_CO2+c onsum_O2+generate_CO2; 0.789, 0.908, tmp+PL+AN+interval_Pdt. +OD+nu_cumulo+rho_cumulo+nu_per_rho_cumulo+emission_CO2+con sum_O2+generate_CO2; 0.776, 0.908, tmp+pH+PL+PL_cumulo+AN+i nterval_Pdt.+mu_cumulo+nu_cumulo+rho_cumulo+nu_per_rho_cumu lo+rab_integral+emission_CO2+consum_O2+generate_CO2; 0.767, 0.908, tmp+PL+PL_cumulo+interval_Pdt.+OD+RS+nu_cumulo+rho_ cumulo+nu_per_rho_cumulo+Arg_conc._cumulo+rab_integral+Accu m._Yield+emission_O2+emission_CO2+consum_O2+generate_CO2; 0. 781, 0.908, tmp+PL+PL_cumulo+interval_Pdt.+OD+mu_cumulo+nu_ cumulo+rho_cumulo+nu_per_rho_cumulo+rab_integral+emission_C O2+consum_O2+generate_CO2; 0.789, 0.908, tmp+PL+AN+interval _Pdt.+mu_cumulo+nu_cumulo+rho_cumulo+Accum._Yield+emission_ CO2+consum_O2+generate_CO2; 0.780, 0.908, tmp+PL_cumulo+int erval_Pdt.+mu_cumulo+nu_cumulo+rho_cumulo+nu_per_rho_cumulo +Arg_conc._cumulo+cell_yield_cumulo+rab_integral+emission_C O2+consum_O2+generate_CO2; 0.767, 0.908, tmp+PL+interval_Pd t.+RS+mu_cumulo+nu_cumulo+rho_cumulo+nu_per_rho_cumulo+Arg_ conc._cumulo+cell_yield_cumulo+rab_integral+RQ_integral+Acc um._Yield+emission_CO2+consum_O2+generate_CO2; 0.789, 0.908, tmp+PL_cumulo+interval_Pdt.+mu_cumulo+nu_cumulo+rho_cumulo +nu_per_rho_cumulo+emission_CO2+consum_O2+interval_O2+gener ate_CO2; 0.785, 0.908, tmp+PL+interval_Pdt.+OD+nu_cumulo+rh o_cumulo+nu_per_rho_cumulo+rab_integral+Accum._Yield+emissi on_CO2+consum_O2+generate_CO2; 0.772, 0.908, tmp+PL+AN+inte rval_Pdt.+OD+nu_cumulo+rho_cumulo+nu_per_rho_cumulo+Arg_con c._cumulo+rab_integral+RQ_integral+Accum._Yield+emission_CO 2+consum_O2+generate_CO2; 0.772, 0.908, tmp+PL+AN+interval_ Pdt.+mu_cumulo+nu_cumulo+rho_cumulo+nu_per_rho_cumulo+Arg_c onc._cumulo+rab_integral+RQ_integral+Accum._Yield+emission_ CO2+consum_O2+generate_CO2; 0.772, 0.908, tmp+PL+interval_P dt.+OD+mu_cumulo+nu_cumulo+rho_cumulo+nu_per_rho_cumulo+RQ_ integral+Accum._Yield+emission_O2+emission_CO2+consum_O2+in terval_O2+generate_CO2; 0.776, 0.908, tmp+PL+PL_cumulo+AN+i nterval_Pdt.+mu_cumulo+nu_cumulo+rho_cumulo+nu_per_rho_cumu lo+Arg_conc._cumulo+Accum._Yield+emission_CO2+consum_O2+gen erate_CO2; 0.776, 0.908, tmp+PL+AN+interval_Pdt.+OD+mu_cumu lo+nu_cumulo+rho_cumulo+nu_per_rho_cumulo+cell_yield_cumulo +Accum._Yield+emission_CO2+consum_O2+generate_CO2; 0.772, 0. 908, tmp+PL+PL_cumulo+interval_Pdt.+RS+mu_cumulo+nu_cumulo+ rho_cumulo+nu_per_rho_cumulo+RQ_integral+Accum._Yield+emiss ion_O2+emission_CO2+consum_O2+generate_CO2; 0.776, 0.908, t mp+PL+AN+interval_Pdt.+OD+mu_cumulo+nu_cumulo+rho_cumulo+nu _per_rho_cumulo+Accum._Yield+emission_O2+emission_CO2+consu m_O2+generate_CO2; 0.789, 0.908, tmp+PL+AN+interval_Pdt.+mu _cumulo+rho_cumulo+rab_integral+Accum._Yield+emission_CO2+c onsum_O2+generate_CO2; 0.776, 0.908, tmp+PL+PL_cumulo+AN+in terval_Pdt.+OD+mu_cumulo+nu_cumulo+rho_cumulo+nu_per_rho_cu mulo+rab_integral+emission_CO2+consum_O2+generate_CO2; 0.78 0, 0.908, tmp+PL+AN+interval_Pdt.+OD+nu_cumulo+rho_cumulo+n _per_rho_cumulo+rab_integral+Accum._Yield+emission_CO2+con sum_O2+generate_CO2; 0.784, 0.908, tmp+interval_Pdt.+mu_cum ulo+nu_cumulo+rho_cumulo+nu_per_rho_cumulo+cell_yield_cumul o+rab_integral+Accum._Yield+emission_CO2+consum_O2+generate _CO2; 0.776, 0.908, tmp+PL+AN+interval_Pdt.+mu_cumulo+nu_cu mulo+rho_cumulo+nu_per_rho_cumulo+RQ_integral+Accum._Yield+ emission_O2+emission_CO2+consum_O2+generate_CO2; 0.772, 0.9 08, tmp+PL+interval_Pdt.+mu_cumulo+nu_cumulo+rho_cumulo+nu_ per_rho_cumulo+cell_yield_cumulo+rab_integral+RQ_integral+A ccum._Yield+emission_CO2+consum_O2+interval_O2+generate_CO 2; 0.776, 0.908, tmp+PL+AN+interval_Pdt.+mu_cumulo+nu_cumul o+rho_cumulo+nu_per_rho_cumulo+rab_integral+emission_O2+emi ssion_CO2+consum_O2+interval_O2+generate_CO2; 0.772, 0.908, tmp+PL+PL_cumulo+AN+interval_Pdt.+RS+mu_cumulo+nu_cumulo+r ho_cumulo+nu_per_rho_cumulo+rab_integral+emission_CO2+consu m_O2+interval_O2+generate_CO2; 0.772, 0.908, tmp+PL+PL_cumu lo+AN+interval_yield+interval_Pdt.+mu_cumulo+nu_cumulo+rho_ cumulo+nu_per_rho_cumulo+cell_yield_cumulo+rab_integral+emi ssion_CO2+consum_O2+generate_CO2; 0.788, 0.908, tmp+PL+inte rval_Pdt.+mu_cumulo+nu_cumulo+rho_cumulo+nu_per_rho_cumulo+ Arg_conc._cumulo+emission_CO2+consum_O2+generate_CO2; 0.776, 0.908, tmp+PL+AN+interval_Pdt.+RS+mu_cumulo+nu_cumulo+rho_ cumulo+nu_per_rho_cumulo+Arg_conc._cumulo+rab_integral+emis sion_CO2+consum_O2+generate_CO2; 0.772, 0.908, tmp+PL+PL_cu mulo+AN+interval_Pdt.+OD+mu_cumulo+nu_cumulo+rho_cumulo+nu_ per_rho_cumulo+rab_integral+Accum._Yield+emission_CO2+consu m_O2+generate_CO2; 0.788, 0.908, tmp+PL+interval_Pdt.+RS+mu _cumulo+nu_cumulo+rho_cumulo+nu_per_rho_cumulo+emission_CO2 +consum_O2+generate_CO2; 0.784, 0.908, tmp+PL_cumulo+interv al_Pdt.+mu_cumulo+nu_cumulo+rho_cumulo+nu_per_rho_cumulo+ce ll_yield_cumulo+Accum._Yield+emission_CO2+consum_O2+generat e_CO2; 0.780, 0.908, tmp+PL+AN+interval_Pdt.+mu_cumulo+nu_c umulo+rho_cumulo+nu_per_rho_cumulo+RQ_integral+emission_CO2 +consum_O2+interval_O2+generate_CO2; 0.767, 0.908, tmp+PL+P L_cumulo+AN+interval_Pdt.+RS+mu_cumulo+nu_cumulo+rho_cumulo +nu_per_rho_cumulo+rab_integral+Accum._Yield+emission_CO2+c onsum_O2+interval_O2+generate_CO2; 0.771, 0.908, tmp+PL+PL_ cumulo+interval_Pdt.+OD+nu_cumulo+rho_cumulo+nu_per_rho_cum ulo+Arg_conc._cumulo+rab_integral+RQ_integral+Accum._Yield+ emission_CO2+consum_O2+generate_CO2; 0.767, 0.908, tmp+PL+i nterval_Pdt.+RS+mu_cumulo+nu_cumulo+rho_cumulo+nu_per_rho_c umulo+Arg_conc._cumulo+cell_yield_cumulo+RQ_integral+Accum. _Yield+emission_O2+emission_CO2+consum_O2+generate_CO2; 0.7 76, 0.908, tmp+pH+PL_cumulo+interval_Pdt.+mu_cumulo+nu_cumu lo+rho_cumulo+nu_per_rho_cumulo+rab_integral+RQ_integral+Ac cum._Yield+emission_CO2+consum_O2+generate_CO2; 0.780, 0.90 8, tmp+PL_cumulo+interval_Pdt.+OD+mu_cumulo+nu_cumulo+rho_c umulo+nu_per_rho_cumulo+cell_yield_cumulo+rab_integral+emis sion_CO2+consum_O2+generate_CO2; 0.776, 0.908, tmp+PL+PL_cu mulo+interval_Pdt.+mu_cumulo+nu_cumulo+rho_cumulo+nu_per_rh o_cumulo+cell_yield_cumulo+Accum._Yield+emission_O2+emissio n_CO2+consum_O2+generate_CO2; 0.767, 0.908, tmp+PL+PL_cumul o+AN+interval_Pdt.+mu_cumulo+nu_cumulo+rho_cumulo+nu_per_rh o_cumulo+cell_yield_cumulo+rab_integral+Accum._Yield+emissi on_CO2+consum_O2+interval_O2+generate_CO2; 0.788, 0.908, tm p+PL+AN+interval_yield+interval_Pdt.+mu_cumulo+rho_cumulo+e mission_CO2+consum_O2+interval_O2+generate_CO2; 0.784, 0.90 8, tmp+PL_cumulo+AN+interval_Pdt.+mu_cumulo+nu_cumulo+rho_c umulo+nu_per_rho_cumulo+cell_yield_cumulo+emission_CO2+cons um_O2+generate_CO2; 0.776, 0.908, tmp+PL+interval_Pdt.+mu_c umulo+nu_cumulo+rho_cumulo+nu_per_rho_cumulo+cell_yield_cum ulo+rab_integral+RQ_integral+emission_CO2+consum_O2+interva l_O2+generate_CO2; 0.776, 0.908, tmp+pH+PL+interval_Pdt.+mu _cumulo+nu_cumulo+rho_cumulo+nu_per_rho_cumulo+RQ_integral+ Accum._Yield+emission_O2+emission_CO2+consum_O2+generate_CO 2; 0.780, 0.908, tmp+PL+interval_Pdt.+RS+mu_cumulo+nu_cumul o+rho_cumulo+nu_per_rho_cumulo+RQ_integral+emission_O2+emis sion_CO2+consum_O2+generate_CO2; 0.771, 0.908, tmp+PL+PL_cu mulo+interval_Pdt.+OD+mu_cumulo+nu_cumulo+rho_cumulo+nu_per _rho_cumulo+RQ_integral+Accum._Yield+emission_O2+emission_C O2+consum_O2+generate_CO2; 0.788, 0.908, tmp+PL+AN+interval _Pdt.+mu_cumulo+rho_cumulo+Arg_conc._cumulo+Accum._Yield+em ission_CO2+consum_O2+generate_CO2; 0.788, 0.908, tmp+PL_cum ulo+interval_Pdt.+mu_cumulo+nu_cumulo+rho_cumulo+nu_per_rho _cumulo+Arg_conc._cumulo+emission_CO2+consum_O2+generate_CO 2; 0.767, 0.908, tmp+pH+PL+PL_cumulo+interval_Pdt.+OD+nu_cu mulo+rho_cumulo+nu_per_rho_cumulo+Arg_conc._cumulo+Accum._Y ield+emission_O2+emission_CO2+consum_O2+interval_O2+generat e_CO2; 0.776, 0.908, tmp+PL+interval_Pdt.+OD+mu_cumulo+nu_c umulo+rho_cumulo+nu_per_rho_cumulo+Arg_conc._cumulo+cell_yi eld_cumulo+rab_integral+emission_CO2+consum_O2+enerate_CO 2; 0.784, 0.908, tmp+PL+interval_Pdt.+OD+nu_cumulo+rho_cumu lo+nu_per_rho_cumulo+rab_integral+emission_CO2+consum_O2+in terval_O2+generate_CO2; 0.776, 0.908, tmp+PL+interval_Pdt.+ mu_cumulo+nu_cumulo+rho_cumulo+nu_per_rho_cumulo+cell_yield _cumulo+RQ_integral+emission_O2+emission_CO2+consum_O2+inte rval_O2+generate_CO2; 0.776, 0.908, tmp+PL+interval_Pdt.+OD +RS+mu_cumulo+nu_cumulo+rho_cumulo+nu_per_rho_cumulo+cell_y ield_cumulo+rab_integral+emission_CO2+consum_O2+generate_CO 2; 0.784, 0.908, tmp+PL+AN+interval_yield+interval_Pdt.+mu_ cumulo+rho_cumulo+Accum._Yield+emission_CO2+consum_O2+inter val_O2+generate_CO2; 0.788, 0.908, tmp+PL+PL_cumulo+interva l_Pdt.+mu_cumulo+nu_cumulo+rho_cumulo+nu_per_rho_cumulo+emi ssion_CO2+consum_O2+generate_CO2; 0.776, 0.908, tmp+PL+inte rval_Pdt.+OD+RS+mu_cumulo+nu_cumulo+rho_cumulo+nu_per_rho_c umulo+rab_integral+Accum._Yield+emission_CO2+consum_O2+gene rate_CO2; 0.780, 0.908, tmp+PL+PL_cumulo+AN+interval_Pdt.+m u_cumulo+rho_cumulo+Accum._Yield+emission_O2+emission_CO2+c onsum_O2+interval_O2+generate_CO2; 0.780, 0.908, tmp+PL+PL_ cumulo+interval_Pdt.+mu_cumulo+nu_cumulo+rho_cumulo+nu_per_ rho_cumulo+Arg_conc._cumulo+emission_O2+emission_CO2+consum _O2+generate_CO2; 0.776, 0.908, tmp+PL+PL_cumulo+AN+OD+nu_c umulo+rho_cumulo+nu_per_rho_cumulo+Arg_conc._cumulo+Accum._ Yield+emission_O2+emission_CO2+consum _O2+generate_CO2; 0.77 1, 0.908, tmp+PL+AN+interval_Pdt.+mu_cumulo+nu_cumulo+rho_c umulo+nu_per_rho_cumulo+RQ_integral+Accum._Yield+emission_O 2+emission_CO2+consum_O2+interval_O2+generate_CO2; 0.771, 0. 908, tmp+PL+AN+interval_Pdt.+mu_cumulo+nu_cumulo+rho_cumulo +nu_per_rho_cumulo+Arg_conc._cumulo+rab_integral+Accum._Yie ld+emission_CO2+consum_O2+interval_O2+generate_CO2; 0.766, 0.908, tmp+PL+PL_cumulo+AN+interval_Pdt.+mu_cumulo+nu_cumul o+rho_cumulo+nu_per_rho_cumulo+rab_integral+RQ_integral+Acc um._Yield+emission_CO2+consum_O2+interval_O2+generate-CO2; 0.771, 0.908, tmp+PL+AN+interval_Pdt.+RS+mu_cumulo+nu_cumul o+rho_cumulo+nu_per_rho_cumulo+Arg_conc._cumulo+rab_integra l+Accum._Yield+eniission_CO2+consum_O2+generate_CO2; 0.771, 0.908, tmp+PL+interval_Pdt.+mu_cumulo+nu_cumulo+rho_cumulo+ nu_per_rho_cumulo+rab_integral+RQ_integral+Accum._Yield+emi ssion_O2+emission_CO2+consum_O2+interval_O2+generate_CO2; 0. 771, 0.908, tmp+PL+PL_cumulo+AN+interval_Pdt.+mu_cumulo+nu_ cumulo+rho_cumulo+nu_per_rho_cumulo+RQ_integral+emission_O2 +emission_CO2+consum_O2+interval_O2+generate_CO2; 0.771, 0. 908, tmp+pH+PL+AN+interval_Pdt.+mu_cumulo+nu_cumulo+rho_cum ulo+nu_per_rho_cumulo+rab_integral+RQ_integral+Accum._Yield +emission_CO2+consum_O2+generate_CO2; 0.766, 0.908, tmp+PL+ PL_cumulo+AN+interval_Pdt.+mu_cumulo+nu_cumulo+rho_cumulo+n u_per_rho_cumulo+rab_integral+RQ_integral+Accum._Yield+emis sion_O2+emission_CO2+consum_O2+generate_CO2; 0.771, 0.908, tmp+PL+AN+interval_Pdt.+OD+mu_cumulo+nu_cumulo+rho_cumulo+n u_per_rho_cumulo+Accum._Yield+emission_O2+emission_CO2+cons um_O2+interval_O2+generate_CO2; 0.766, 0.908, tmp+PL+interv al_Pdt.+OD+RS+mu_cumulo+nu_cumulo+rho_cumulo+nu_per_rho_cum ulo+Arg_conc._cumulo+rab_integral+RQ_integral+Accum._Yield+ emission_CO2+consum_O2+generate_CO2; 0.780, 0.908, tmp+PL+A N+interval_Pdt.+OD+mu_cumulo+nu_cumulo+rho_cumulo+nu_per_rh o_cumulo+Arg_conc.cumulo+emission_CO2+consum_O2+generate_C O2; 0.780, 0.908, tmp+PL+interval_Pdt.+mu_cumulo+nu_cumulo+ rho_cumulo+nu_per_rho_cumulo+rab_integral+RQ_integral+emiss ion_O2+emission_CO2+consum_O2+generate_CO2; 0.771, 0.908, t mp+PL+AN+interval_Pdt.+OD+mu_cumulo+nu_cumulo+rho_cumulo+nu _per_rho_cumulo+cell_yield_cumulo+rab_integral+emission_CO2 +consum_O2+interval_O2+generate_CO2; 0.780, 0.908, tmp+PL+A N+interval_Pdt.+RS+mu_cumulo+nu_cumulo+rho_cumulo+nu_per_rh o_cumulo+cell_yield_cumulo+emission_CO2+consum_O2+generate-CO2; 0.766, 0.908, tmp+PL+PL_cumulo+interval_Pdt.+OD+mu_cum ulo+nu_cumulo+rho_cumulo+nu_per_rho_cumulo+Arg_conc._cumulo +rab_integral+Accum._Yield+emission_O2+emission_CO2+consum_ O2+generate_CO2; 0.776, 0.908, tmp+PL+AN+interval_Pdt.+mu_c umulo+nu_cumulo+rho_cumulo+nu_per_rho_cumulo+rab_integral+A ccum._Yield+emission_O2+emission_CO2+consum_O2+generate_CO 2; 0.776, 0.908, tmp+PL+AN+interval_Pdt.+OD+mu_cumulo+nu_cu mulo+rho_cumulo+nu_per_rho_cumulo+Arg_conc._cumulo+rab_inte gral+emission_CO2+consum_O2+generate_CO2; 0.788, 0.908, tmp +PL+AN+interval_Pdt.+mu_cumulo+rho_cumulo+nu_per_rho_cumulo +Arg_conc._cumulo+emission_CO2+consum_O2+generate_CO2; 0.78 0, 0.908, tmp+PL+interval_Pdt.+OD+mu_cumulo+nu_cumulo+rho_c umulo+nu_per_rho_cumulo+Arg_conc._cumulo+emission_O2+emissi on_CO2+consum_O2+generate_CO2; 0.780, 0.908, tmp+PL+interva l_Pdt.+OD+nu_cumulo+rho_cumulo+nu_per_rho_cumulo+Arg_conc._ cumulo+emission_O2+emission_CO2+consum_O2+interval_O2+gener ate_CO2; 0.784, 0.908, tmp+pH+PL_cumulo+interval_Pdt.+mu_cu mulo+nu_cumulo+rho_cumulo+nu_per_rho_cumulo+rab_integral+em ission_CO2+consum_O2+generate_CO2; 0.788, 0.908, tmp+interv al_Pdt.+RS+mu_cumulo+nu_cumulo+rho_cumulo+nu_per_rho_cumulo +rab_integral+emission_CO2+consum_O2+generate_CO2; 0.784, 0. 908, tmp+pH+OD+nu_cumulo+rho_cumulo+nu_per_rho_cumulo+Arg_c onc._cumulo+rab_integral+Accum._Yield+emission_CO2+consum_O 2+generate_CO2; 0.784, 0.908, tmp+PL_cumulo+OD+nu_cumulo+rh o_cumulo+nu_per_rho_cumulo+Arg_conc._cumulo+rab_integral+Ac cum._Yield+emission_CO2+consum_O2+generate_CO2; 0.788, 0.90 8, tmp+PL+interval_Pdt.+mu_cumulo+nu_cumulo+rho_cumulo+nu_p r_rho_cumulo+cell_yield_cumulo+emission_CO2+consuni_O2+gene rate_CO2; 0.784, 0.908, tmp+PL_cumulo+AN+interval_Pdt.+OD+m u_cumulo+nu_cumulo+rho_cumulo+nu_per_rho_cumulo+emission_CO 2+consum_O2+generate_CO2; 0.780, 0.908, tmp+PL+PL_cumulo+AN +interval_Pdt.+mu_cumulo+rho_cumulo+rab_integral+Accum._Yie ld+emission_CO2+consum_O2+interval_O2+generate_CO2; 0.775, 0.908, tmp+PL+AN+interval_Pdt.+RS+mu_cumulo+nu_cumulo+rho_c umulo+nu_per_rho_cumulo+rab_integral+RQ_integral+emission_C O2+consum_O2+generate_CO2; 0.775, 0.908, tmp+PL+interval_Pd t.+mu_cumulo+nu_cumulo+rho_cumulo+nu_per_rho_cumulo+Arg_con c._cumulo+cell_yield_cumulo+rab_integral+RQ_integral+emissi on_CO2+consum_O2+generate_CO2; 0.775, 0.908, tmp+PL+AN+inte rval_Pdt.+OD+RS+mu_cumulo+nu_cumulo+rho_cumulo+nu_per_rho_c umulo+rab_integral+emission_CO2+consum_O2+generate_CO2 0.7 71, 0.908, tmp+PL+PL_cumulo+AN+interval_Pdt.+RS+mu_cumulo+n u_cumulo+rho_cumulo+nu_per_rho_cumulo+emission_O2+emission CO2+consum_O2+interval_O2+generate_CO2; 0.775, 0.908, tmp+P L+AN+interval_Pdt.+RS+mu_cumulo+nu_cumulo+rho_cumulo+nu_per _rho_cumulo+emission_O2+emission_CO2+consum_O2+interval_O2+ generate_CO2; 0.761, 0.908, tmp+PL+PL_cumulo+AN+interval_Pd t.+mu_cumulo+nu_cumulo+rho_cumulo+nu_per_rho_cumulo+rab_int egral+RQ_integral+Accum._Yield+emission_02+emission_CO2+con sum_O2+interval_O2+generate_CO2; 0.775, 0.908, tmp+pH+PL+in terval_Pdt.+mu_cumulo+nu_cumulo+rho_cumulo+nu_per_rho_cumul o+cell_yield_cumulo+rab_integral+Accum._Yield+eniission_CO2+ consum_O2+generate_CO2; 0.780, 0.908, tmp+PL+AN+interval_Pd t.+mu_cumulo+nu_cumulo+rho_cumulo+nu_per_rho_cumulo+rab_int egral+emission_O2+emission_CO2+consum_O2+generate_CO2; 0.77 5, 0.908, tmp+PL_cumulo+interval_Pdt.+RS+mu_cumulo+nu_cumul o+rho_cumulo+nu_per_rho_cumulo+cell_yield_cumulo+rab_integr al+Accum._Yield+emission_CO2+consum_O2+generate_CO2; 0.775, 0.908, tmp+PL+PL_cumulo+AN+interval_Pdt.+mu_cumulo+nu_cumu lo+rho_cumulo+nu_per_rho_cumulo+Accum._Yield+emission_Co2+c onsum_O2+interval_O2+generate_CO2; 0.771, 0.908, tmp+pH+PL+ interval_Pdt.+mu_cumulo+nu_cumulo+rho_cumulo+nu_per_rho_cum ulo+Arg_conc._cumulo+rab_integral+RQ_integral+Accum._Yield+ emission_CO2+consum_O2+generate_CO2; 0.775, 0.908, tmp+PL+i nterval_Pdt.+RS+mu_cumulo+nu_cumulo+rho_cumulo+nu_per_rho_c umulo+cell_yield_cumulo+Accum._Yield+emission_O2+emission_C O2+consum_O2+generate_CO2; 0.771, 0.908, tmp+PL+AN+interval _Pdt.+mu_cumulo+nu_cumulo+rho_cumulo+nu_per_rho_cumulo+cell _yield_cumulo+RQ_integral+Accum._Yield+eniission_O2+emission _CO2+consum_O2+generate_CO2; 0.780, 0.908, tmp+PL+interval_ Pdt.+mu_cumulo+nu_cumulo+rho_cumulo+nu_per_rho_cumulo+cell_ yield_cumulo+rab_integral+emission_O2+emission_CO2+consum_O 2+generate_CO2; 0.784, 0.908, tmp+PL_cumulo+interval_Pdt.+m u_cumulo+nu_cumulo+rho_cumulo+nu_per_rho_cumulo+cell_yield_ cumulo+emission_O2+emission_CO2+consum_O2+generate_CO2; 0.7 75, 0.908, tmp+PL+PL_cumulo+interval_Pdt.+mu_cumulo+nu_cumu lo+rho_cumulo+nu_per_rho_cumulo+rab_integral+emission_O2+em ission_CO2+consum_O2+interval_O2+generate_CO2; 0.784, 0.908, mp+PL+AN+interval_Pdt.+mu_cumulo+rho_cumulo+nu_per_rho_cu mulo+Accum._Yield+emission_CO2+consum_O2+interval_O2+genera te_CO2; 0.775, 0.908, tmp+PL+AN+interval_Pdt.+OD+nu_cumulo+ rho_cumulo+nu_per_rho_cumulo+Arg_conc._cumulo+Accum._Yield+ emission_CO2+consum_O2+interval_O2+generate_CO2; 0.766, 0.9 08, tmp+PL+PL_cumulo+AN+interval_Pdt.+OD+mu_cumulo+nu_cumul o+rho_cumulo+nu_per_rho_cumulo+Accum._Yield+emission_O2+emi ssion_CO2+consum_O2+interval_O2+generate_CO2; 0.771, 0.908, tmp+PL+PL_cumulo+AN+interval_Pdt.+RS+mu_cumulo+nu_cumulo+r ho_cumulo+nu_per_rho_cumulo+Accum._Yield+emission_O2+emissi on_CO2+consum_O2+generate_CO2; 0.771, 0.908, tmp+PL+PL_cumu lo+interval_Pdt.+RS+mu_cumulo+nu_cumulo+rho_cumulo+nu_per_r ho_cumulo+Arg_conc._cumulo+rab_integral+Accum._Yield+emissi on_CO2+consum_O2+generate_CO2; 0.784, 0.908, tmp+PL+interva l_Pdt.+mu_cumulo+nu_cumulo+rho_cumulo+nu_per_rho_cumulo+Arg _conc._cumulo+Accum._Yield+emission_CO2+consum_O2+generate_ CO2; 0.788, 0.908, tmp+PL+AN+interval_Pdt.+mu_cumulo+rho_cu mulo+cell_yield_cumulo+Accum._Yield+emission_CO2+consum_O2+ generate_CO2; 0.784, 0.908, tmp+PL_cumulo+interval_Pdt.+mu_ cumulo+nu_cumulo+rho_cumulo+nu_per_rho_cumulo+rab_integral+ emission_CO2+consum_O2+interval_O2+generate_CO2; 0.784, 0.9 08, tmp+PL+AN+interval_Pdt.+RS+mu_cumulo+rho_cumulo+Accum._ Yield+emission_CO2+consum_O2+interval_O2+generate_CO2; 0.79 1, 0.907, tmp+PL+AN+interval_yield+interval_Pdt.+mu_cumulo+ rho_cumulo+emission_CO2+consum_O2+generate_CO2; 0.784, 0.90 7, tmp+PL+AN+interval_yield+interval_Pdt.+mu_cumulo+nu_cumu lo+rho_cumulo+nu_per_rho_cumulo+emission_CO2+consum_O2+gene rate_CO2; 0.775, 0.907, tmp+PL+AN+interval_Pdt.+RS+mu_cumul o+nu_cumulo+rho_cumulo+nu_per_rho_cumulo+cell_yield_cumulo+ Accum._Yield+emission_CO2+consum_O2+generate_CO2; 0.771, 0. 907, tmp+PL+PL_cumulo+AN+interval_Pdt.+OD+mu_cumulo+nu_cumu lo+rho_cumulo+nu_per_rho_cumulo+emission_O2+emission_CO2+co nsum_O2+interval_O2+generate_CO2; 0.775, 0.907, tmp+PL+inte rval_Pdt.+OD+mu_cumulo+nu_cumulo+rho_cumulo+nu_per_rho_cumu lo+cell_yield_cumulo+Accum._Yield+emission_O2+emission_CO2+ consum_O2+generate_CO2; 0.779, 0.907, tmp+pH+PL_cumulo+inte rval_Pdt.+mu_cumulo+nu_cumulo+rho_cumulo+nu_per_rho_cumulo+ rab_integral+RQ_integral+emission_CO2+consum_O2+generate_CO 2; 0.791, 0.907, tmp+PL+AN+interval_Pdt.+mu_cumulo+rho_cumu lo+emission_CO2+consum_O2+interval_O2+generate_CO2; 0.771, 0.907, tmp+PL+PL_cumulo+AN+interval_Pdt.+mu_cumulo+nu_cumul o+rho_cumulo+nu_per_rho_cumulo+cell_yield_cumulo+Accum._Yie ld+emission_O2+emission_CO2+consum_O2+generate_CO2; 0.771, 0.907, tmp+PL+PL_cumulo+AN+interval_Pdt.+mu_cumulo+nu_cumul o+rho_cumulo+nu_per_rho_cumulo+rab_integral+RQ_integral+emi ssion_CO2+consum_O2+interval_O2+generate_CO2; 0.779, 0.907, tmp+PL+PL_cumulo+interval_Pdt.+RS+mu_cumulo+nu_cumulo+rho_ cumulo+nu_per_rho_cumulo+emission_O2+emission_CO2+consum_O2 +generate_CO2; 0.775, 0.907, tmp+pH+PL+AN+interval_Pdt.+mu_ cumulo+nu_cumulo+rho_cumulo+nu_per_rho_cumulo+rab_integral+ emission_CO2+consum_O2+interval_O2+generate_CO2; 0.775, 0.9 07, tmp+PL+PL_cumulo+interval_Pdt.+RS+mu_cumulo+nu_cumulo+r ho_cumulo+nu_per_rho_cumulo+Accum._Yield+emission_O2+emissi on_CO2+consum_O2+generate_CO2; 0.779, 0.907, tmp+PL+AN+inte rval_Pdt.+RS+mu_cumulo+nu_cumulo+rho_cumulo+nu_per_rho_cumu lo+emission_O2+emission_CO2+consum_O2+generate_CO2; 0.779, 0.907, tmp+PL+interval_Pdt.+mu_cumulo+nu_cumulo+rho_cumulo+ nu_per_rho_cumulo+Arg_conc._cumulo+RQ_integral+emission_O2+ emission_CO2+consum_O2+generate_CO2; 0.775, 0.907, tmp+PL+A N+interval_Pdt.+RS+mu_cumulo+nu_cumulo+rho_cumulo+nu_per_rh o_cumulo+Accum._Yield+emission_O2+emission_CO2+consum-O2+ge nerate_CO2; 0.783, 0.907, tmp+PL+AN+interval_Pdt.+mu_cumulo +rho_cumulo+Arg_conc._cumulo+Accum._Yield+emission_CO2+cons um_O2+interval_O2+generate_CO2; 0.766, 0.907, tmp+PL+PL_cum ulo+interval_Pdt.+mu_cumulo+nu_cumulo+rho_cumulo+nu_per_rho _cumulo+cell_yield_cumulo+RQ_integral+Accum._Yield+emission _O2+emission_CO2+consum_O2+interval_O2+generate_CO2; 0.779, 0.907, tmp+PL_cumulo+interval_Pdt.+OD+mu_cumulo+nu_cumulo+ rho_cumulo+nu_per_rho_cumulo+rab_integral+Accum._Yield+emis sion_CO2+consum_O2+generate_CO2; 0.779, 0.907, tmp+PL+AN+in terval_Pdt.+mu_cumulo+nu_cumulo+rho_cumulo+nu_per_rho_cumul o+Arg_conc._cumulo+emission_O2+emission_CO2+consum_O2+gener ate_CO2; 0.783, 0.907, tmp+PL+AN+interval_yield+interval_Pd t.+mu_cumulo+rho_cumulo+nu_per_rho_cumulo+Accum._Yield+emis sion_CO2+consum_O2+generate_CO2; 0.775, 0.907, tmp+PL+PL_cu mulo+interval_Pdt.+mu_cumulo+nu_cumulo+rho_cumulo+nu_per_rh o_cumulo+cell_yield_cumulo+emission_O2+emission_CO2+consum_ O2+interval_O2+generate_CO2; 0.787, 0.907, tmp+PL+interval_ Pdt.+OD+mu_cumulo+nu_cumulo+rho_cumulo+nu_per_rho_cumulo+em ission_CO2+consum_O2+generate_CO2; 0.775, 0.907, tmp+PL_cum ulo+interval_Pdt.+OD+mu_cumulo+nu_cumulo+rho_cumulo+nu_per_ rho_cumulo+rab_integral+RQ_integral+Accum._Yield+emission_C O2+consum_O2+generate_CO2; 0.761, 0.907, tmp+PL+PL_cumulo+A N+interval_Pdt.+OD+RS+mu_cumulo+nu_cumulo+rho_cumulo+nu_per _rho_cumulo+Arg_conc._cumulo+rab_integral+Accum._Yield+emis sion_CO2+consum_O2+generate_CO2; 0.775, 0.907, tmp+PL+PL_cu mulo+OD+nu_cumulo+rho_cumulo+nu_per_rho_cumulo+Arg_conc._cu mulo+Accum._Yield+emission_O2+emission_CO2+consum_O2+interv al_O2+generate_CO2; 0.783, 0.907, tmp+PL+AN+interval_Pdt.+m u_cumulo+rho_cumulo+nu_per_rho_cumulo+rab_integral+emission _CO2+consum_O2+interval_O2+generate_CO2; 0.783, 0.907, tmp+ PL_cumulo+interval_Pdt.+OD+mu_cumulo+nu_cumulo+rho_cumulo+n u_per_rho_cumulo+Accum._Yield+emission_CO2+consum_O2+genera te_CO2; 0.779, 0.907, tmp+PL+interval_Pdt.+OD+nu_cumulo+rho _cumulo+nu_per_rho_cumulo+RQ_integral+emission_O2+emission-CO2+consum_O2+interval_O2+generate_CO2; 0.783, 0.907, tmp+i nterval_Pdt.+RS+mu_cumulo+nu_cumulo+rho_cumulo+nu_per_rho_c umulo+rab_integral+Accum._Yield+emission_CO2+consum_O2+gene rate_CO2; 0.787, 0.907, tmp+PL+interval_Pdt.+mu_cumulo+rho_ cumulo+rab_integral+RQ_integral+Accum._Yield+emission_CO2+c onsum_O2+generate_CO2; 0.783, 0.907, tmp+PL_cumulo+AN+inter val_Pdt.+mu_cumulo+nu_cumulo+rho_cumulo+nu_per_rho_cumulo+e mission_CO2+consum_O2+interval_O2+generate_CO2; 0.779, 0.90 7, tmp+PL+interval_Pdt.+OD+RS+mu_cumulo+nu_cumulo+rho_cumul o+nu_per_rho_cumulo+emission_O2+emission_CO2+consum_O2+gene rate_CO2; 0.770, 0.907, tmp+PL+interval_Pdt.+RS+mu_cumulo+n u_cumulo+rho_cumulo+nu_per_rho_cumulo+RQ_integral+Accum._Yi eld+emission_O2+emission_CO2+consum_O2+interval_O2+generate _CO2; 0.779, 0.907, tmp+PL+AN+interval_Pdt.+OD+nu_cumulo+rh o_cumulo+nu_per_rho_cumulo+emission_O2+emission_CO2+consum_ O2+interval_O2+generate_CO2; 0.783, 0.907, tmp+PL+interval_ Pdt.+OD+nu_cumulo+rho_cumulo+nu_per_rho_cumulo+emission_O2+ emission_CO2+consum_O2+interval_O2+generate_CO2; 0.770, 0.9 07, tmp+pH+PL+AN+interval_Pdt.+OD+nu_cumulo+rho_cumulo+nu_p er_rho_cumulo+Arg_conc._cumulo+rab_integral+Accum._Yield+eni ission_CO2+consum_O2+generate_CO2; 0.783, 0.907, tmp+PL+AN+ interval_Pdt.+mu_cumulo+nu_cumulo+rho_cumulo+Accum._Yield+e mission_CO2+consum_O2+interval_O2+generate_CO2; 0.783, 0.90 7, tmp+pH+interval_Pdt.+mu_cumulo+nu_cumulo+rho_cumulo+nu_p er_rho_cumulo+rab_integral+RQ_integral+emission_CO2+consum_ O2+generate_CO2; 0.779, 0.907, tmp+PL+PL_cumulo+interval_Pd t.+OD+mu_cumulo+nu_cumulo+rho_cumulo+nu_per_rho_cumulo+emis sion_O2+emission_CO2+consum_O2+generate_CO2; 0.770, 0.907, tmp+PL+AN+interval_Pdt.+mu_cumulo+nu_cumulo+rho_cumulo+nu_p er_rho_cumulo+rab_integral+Accum._Yield+emission_O2+emissio n_CO2+consum_O2+interval_O2+generate_CO2; 0.770, 0.907, tmp +PL+interval_Pdt.+RS+mu_cumulo+nu_cumulo+rho_cumulo+nu_per_ rho_cumulo+Arg_conc._cumulo+RQ_integral+Accum._Yield+emissi on_O2+emission_CO2+consum_O2+generate_CO2; 0.770, 0.907, tm p+PL+AN+interval_Pdt.+RS+mu_cumulo+nu_cumulo+rho_cumulo+nu_ per_rho_cumulo+cell_yield_cumulo+rab_integral+emission_CO2+ consum_O2+interval_O2+generate_CO2; 0.783, 0.907, tmp+PL_cu mulo+interval_Pdt.+OD+mu_cumulo+nu_cumulo+rho_cumulo+nu_per _rho_cumulo+cell_yield_cumulo+emission_CO2+consum_O2+genera te_CO2; 0.775, 0.907, tmp+PL+interval_Pdt.+RS+mu_cumulo+nu_ cumulo+rho_cumulo+nu_per_rho_cumulo+Arg_conc._cumulo+rab_in tegral+RQ_integral+emission_CO2+consum_O2+generate_CO2; 0.7 79, 0.907, tmp+PL_cumulo+interval_Pdt.+mu_cumulo+nu_cumulo+ rho_cumulo+nu_per_rho_cumulo+cell_yield_cumulo+rab_integral +emission_CO2+consum_O2+interval_O2+generate_CO2; 0.775, 0. 907, tmp+PL+PL_cumulo+AN+interval_Pdt.+mu_cumulo+nu_cumulo+ rho_cumulo+nu_per_rho_cumulo+cell_yield_cumulo+emission_O2+ emission_CO2+consum_O2+generate_CO2; 0.765, 0.907, tmp+pH+P L+PL_cumulo+interval_Pdt.+OD+nu_cumulo+rho_cumulo+nu_per_rh o_cumulo+Arg_conc._cumulo+rab_integral+Accum._Yield+emissio n_O2+emission_CO2+consum_O2+generate_CO2; 0.779, 0.907, tmp +PL_cumulo+interval_Pdt.+RS+mu_cumulo+nu_cumulo+rho_cumulo+ nu_per_rho_cumulo+rab_integral+Accum._Yield+eniission_CO2+co nsum_O2+generate_CO2; 0.779, 0.907, tmp+PL_cumulo+interval_ Pdt.+OD+nu_cumulo+rho_cumulo+nu_per_rho_cumulo+Arg_conc._cu mulo+rab_integral+Accum._Yield+emission_CO2+consum_O2+gener ate_CO2; 0.775, 0.907, tmp+pH+PL+AN+interval_Pdt.+mu_cumulo +nu_cumulo+rho_cumulo+nu_per_rho_cumulo+Arg_conc._cumulo+Ac cum._Yield+emission_CO2+consum_O2+generate_CO2; 0.765, 0.90 7, tmp+PL+AN+interval_Pdt.+mu_cumulo+nu_cumulo+rho_cumulo+n u_per_rho_cumulo+Arg_conc._cumulo+cell_yield_cumulo+rab_int egral+RQ_integral+Accum._Yield+emission_CO2+consum_O2+gener ate_CO2; 0.770, 0.907, tmp+PL+AN+interval_Pdt.+mu_cumulo+nu _cumulo+rho_cumulo+nu_per_rho_cumulo+Arg_conc._cumulo+cell_ yield_cumulo+emission_O2+emission_CO2+consum_O2+interval_O2 +generate_CO2; 0.779, 0.907, tmp+PL+PL_cumulo+AN+interval_P dt.+mu_cumulo+nu_cumulo+rho_cumulo+nu_per_rho_cumulo+Arg_co nc._cumulo+emission_CO2+consum_O2+generate_CO2; 0.770, 0.90 7, tmp+PL+AN+interval_Pdt.+RS+mu_cumulo+nu_cumulo+rho_cumul o+nu_per_rho_cumulo+Arg_conc._cumulo+cell_yield_cumulo+rab_ integral+emission_CO2+consum_O2+generate_CO2; 0.779, 0.907, tmp+PL+AN+interval_Pdt.+OD+RS+mu_cumulo+nu_cumulo+rho_cumu lo+nu_per_rho_cumulo+emission_CO2+consum_O2+generate_CO2; 0. 779, 0.907, tmp+PL+AN+interval_Pdt.+OD+nu_cumulo+rho_cumulo +nu_per_rho_cumulo+Arg_conc._cumulo+emission_O2+emission_CO 2+consum_O2+generate_CO2; 0.779, 0.907, tmp+PL+interval_Pdt. +OD+RS+nu_cumulo+rho_cumulo+nu_per_rho_cumulo+Arg_conc._cum ulo+rab_integral+emission_CO2+consum_O2+generate_CO2; 0.779, 0.907, tmp+pH+PL+OD+nu_cumulo+rho_cumulo+nu_per_rho_cumulo +Arg_conc._cumulo+Accum._Yield+emission_O2+emission_CO2+con sum_O2+generate_CO2; 0.783, 0.907, tmp+PL+PL_cumulo+AN+inte rval_Pdt.+mu_cumulo+rho_cumulo+rab_integral+Accum._Yield+em ission_CO2+consum_O2+generate_CO2; 0.783, 0.907, tmp+PL_cum ulo+OD+RS+nu_cumulo+rho_cumulo+nu_per_rho_cumulo+Arg_conc._ cumulo+rab_integral+Accum._Yield+emission_CO2+generate_CO2; 0.774, 0.907, tmp+PL_cumulo+interval_Pdt.+RS+mu_cumulo+nu_ cumulo+rho_cumulo+nu_per_rho_cumulo+cell_yield_cumulo+rab_i ntegral+RQ_integral+emission_CO2+consum_O2+generate_CO2; 0. 774, 0.907, tmp+pH+PL+AN+interval_Pdt.+mu_cumulo+nu_cumulo+ rho_cumulo+nu_per_rho_cumulo+cell_yield_cumulo+rab_integral +emission_CO2+consum_O2+generate_CO2; 0.783, 0.907, tmp+PL+ OD+nu_cumulo+rho_cumulo+nu_per_rho_cumulo+Arg_conc._cumulo+ emission_O2+emission_CO2+consum_O2+interval_O2+generate_CO 2; 0.783, 0.907, tmp+PL+AN+interval_Pdt.+RS+mu_cumulo+rho_c umulo+rab_integral+Accum._Yield+emission_CO2+consum_O2+gene rate_CO2; 0.765, 0.907, tmp+PL+PL_cumulo+interval_Pdt.+mu_c umulo+nu_cumulo+rho_cumulo+nu_per_rho_cumulo+Arg_conc._cumu lo+RQ_integral+Accum._Yield+emission_O2+emission_CO2+consum _O2+interval_O2+generate_CO2; 0.770, 0.907, tmp+PL_cumulo+i nterval_Pdt.+RS+mu_cumulo+nu_cumulo+rho_cumulo+nu_per_rho_c umulo+cell_yield_cumulo+rab_integral+RQ_integral+Accum._Yie ld+emission_CO2+consum_O2+generate_CO2; 0.779, 0.907, tmp+p H+PL+AN+interval_Pdt.+mu_cumulo+nu_cumulo+rho_cumulo+nu_per rho_cumulo+emission_CO2+consum_O2+interval_O2+generate_CO 2; 0.765, 0.907, tmp+PL+AN+interval_Pdt.+OD+RS+mu_cumulo+nu _cumulo+rho_cumulo+nu_per_rho_cumulo+Arg_conc._cumulo+rab_i ntegral+Accum._Yield+emission_CO2+consum_O2+generate_CO2; 0. 774, 0.907, tmp+PL_cumulo+interval_Pdt.+mu_cumulo+nu_cumulo +rho_cumulo+nu_per_rho_cumulo+cell_yield_cumulo+RQ_integral +Accum._Yield+emission_O2+emission_CO2+consum_O2+generate_C O2; 0.770, 0.907, tmp+PL+interval_Pdt.+OD+RS+mu_cumulo+nu_c umulo+rho_cumulo+nu_per_rho_cumulo+rab_integral+RQ_integral +Accum._Yield+emission_CO2+consum_O2+generate_CO2; 0.774, 0. 907, tmp+pH+PL+PL_cumulo+interval_Pdt.+mu_cumulo+nu_cumulo+ rho_cumulo+nu_per_rho_cumulo+rab_integral+Accum._Yield+emis sion_CO2+consum_O2+generate_CO2; 0.779, 0.907, tmp+PL+AN+in terval_Pdt.+RS+mu_cumulo+rho_cumulo+rab_integral+Accum._Yie ld+emission_CO2+consum_O2+interval_O2+generate_CO2; 0.770, 0.907, tmp+PL+PL_cumulo+AN+interval_Pdt.+OD+mu_cumulo+nu_cu mulo+rho_cumulo+nu_per_rho_cumulo+rab_integral+emission_CO2 +consum_O2+interval_O2+generate_CO2; 0.774, 0.907, tmp+PL+i nterval_Pdt.+OD+mu_cumulo+nu_cumulo+rho_cumulo+nu_per_rho_c umulo+rab_integral+Accum._Yield+emission_CO2+consum_O2+inte rval_O2+generate_CO2; 0.774, 0.907, tmp+PL+PL_cumulo+OD+nu_ cumulo+rho_cumulo+nu_per_rho_cumulo+Arg_conc._cumulo+rab_in tegral+Accum._Yield+emission_O2+emission_CO2+consum_O2+gene rate_CO2; 0.787, 0.907, tmp+PL_cumulo+OD+nu_cumulo+rho_cumu lo+nu_per_rho_cumulo+Arg_conc._cumulo+rab_integral+Accum._Y ield+emission_CO2+generate_CO2; 0.774, 0.907, tmp+PL+AN+int erval_Pdt.+mu_cumulo+nu_cumulo+rho_cumulo+nu_per_rho_cumulo +Arg_conc._cumulo+cell_yield_cumulo+emission_O2+emission_CO 2+consum_O2+generate_CO2; 0.760, 0.907, tmp+PL+PL_cumulo+AN +interval_Pdt.+OD+RS+nu_cumulo+rho_cumulo+nu_per_rho_cumulo +Arg_conc._cumulo+Accum._Yield+emission_O2+emission_CO2+con sum_O2+interval_O2+generate_CO2; 0.774, 0.907, tmp+PL_cumul o+interval_Pdt.+mu_cumulo+nu_cumulo+rho_cumulo+nu_per_rho_c umulo+Arg_conc._cumulo+cell_yield_cumulo+rab_integral+Accum. _Yield+emission_CO2+consum_O2+generate_CO2; 0.779, 0.907, t mp+PL+PL_cumulo+OD+nu_cumulo+rho_cumulo+nu_per_rho_cumulo+A rg_conc._cumulo+rab_integral+Accum._Yield+emission_CO2+cons um_O2+generate_CO2; 0.779, 0.907, tmp+PL_cumulo+interval_Pd t.+OD+mu_cumulo+nu_cumulo+rho_cumulo+nu_per_rho_cumulo+rab_ integral+RQ_integral+emission_CO2+consum _O2+generate_CO2; 0. 760, 0.907, tmp+PL+PL_cumulo+AN+interval_Pdt.+OD+mu_cumulo+ nu_cumulo+rho_cumulo+nu_per_rho_cumulo+Arg_conc._cumulo+Acc um._Yield+emission_O2+emission_CO2+consum_O2+interval_O2+ge nerate_CO2; 0.770, 0.907, tmp+PL+PL_cumulo+interval_Pdt.+mu _cumulo+nu_cumulo+rho_cumulo+nu_per_rho_cumulo+Arg_conc._cu mulo+Accum._Yield+emission-O2+emission_CO2+consum_O2+interv al_O2+generate_CO2; 0.774, 0.907, tmp+pH+PL+interval_Pdt.+O D+nu_cumulo+rho_cumulo+nu_per_rho_cumulo+Arg_conc._cumulo+A ccum._Yield+emission_O2+emission_CO2+consum_O2+generate_CO 2; 0.770, 0.907, tmp+PL+interval_Pdt.+OD+RS+mu_cumulo+nu_cu mulo+rho_cumulo+nu_per_rho_cumulo+RQ_integral+Accum._Yield+ emission_O2+emission_CO2+consum_O2+generate_CO2; 0.774, 0.9 07, tmp+PL+interval_Pdt.+OD+RS+mu_cumulo+nu_cumulo+rho_cumu lo+nu_per_rho_cumulo+Arg_conc._cumulo+rab_integral+emission _CO2+consum_O2+generate_CO2; 0.783, 0.907, tmp+PL+AN+interv al_Pdt.+mu_cumulo+rho_cumulo+nu_per_rho_cumulo+Arg_conc._cu mulo+emission_CO2+consum_O2+interval_O2+generate_CO2; 0.774, 0.907, tmp+PL+AN+interval_yield+interval_Pdt.+mu_cumulo+nu _cumulo+rho_cumulo+nu_per_rho_cumulo+cell_yield_cumulo+rab_ integral+emission_CO2+consum_O2+generate_CO2; 0.770, 0.907, tmp+PL+PL_cumulo+interval_Pdt.+mu_cumulo+nu_cumulo+rho_cum ulo+nu_per_rho_cumulo+rab_integral+Accum._Yield+emission_O2 +emission_CO2+consum_O2+interval_O2+generate_CO2; 0.774, 0. 907, tmp+PL+AN+interval_Pdt.+OD+nu_cumulo+rho_cumulo+nu_per _rho_cumulo+Arg_conc._cumulo+rab_integral+emission_CO2+cons um_O2+interval_O2+generate_CO2; 0.783, 0.907, tmp+PL_cumulo +interval_Pdt.+mu_cumulo+nu_cumulo+rho_cumulo+nu_per_rho_cu mulo+Arg_conc._cumulo+rab_integral+emission_CO2+consum_O2+g enerate_CO2; 0.765, 0.907, tmp+PL+PL_cumulo+AN+interval_Pdt. +OD+nu_cumulo+rho_cumulo+nu_per_rho_cumulo+Arg_conc._cumulo +rab_integral+RQ_integral+Accum._Yield+emission_CO2+consum-O2+generate_CO2; 0.765, 0.907, tmp+PL+PL_cumulo+interval_Pd t.+RS+mu_cumulo+nu_cumulo+rho_cumulo+nu_per_rho_cumulo+Arg_ conc._cumulo+RQ_integral+Accum._Yield+emission_O2+emission-CO2+consum_O2+generate_CO2; 0.783, 0.907, tmp+PL_cumulo+AN+ interval_Pdt.+mu_cumulo+nu_cumulo+rho_cumulo+nu_per_rho_cum ulo+Accum._Yield+emission_CO2+consum_O2+generate_CO2; 0.770, 0.907, tmp+pH+PL+interval_Pdt.+OD+mu_cumulo+nu_cumulo+rho_ cumulo+nu_per_rho_cumulo+Arg_conc._cumulo+rab_integral+Accu m._Yield+emission_CO2+consum_O2+generate_CO2; 0.787, 0.907, tmp+interval_Pdt.+mu_cumulo+nu_cumulo+rho_cumulo+nu_per_rh o_cumulo+rab_integral+emission_CO2+consum_O2+interval_O2+ge nerate_CO2; 0.779, 0.907, tmp+PL+AN+interval_Pdt.+OD+nu_cum ulo+rho_cumulo+nu_per_rho_cumulo+rab_integral+RQ_integral+e mission_CO2+consum_O2+generate_CO2; 0.787, 0.907, tmp+PL+AN +interval_Pdt.+RS+mu_cumulo+rho_cumulo+nu_per_rho_cumulo+em ission_CO2+consum_O2+generate_CO2; 0.765, 0.907, tmp+PL+PL_ cumulo+interval_Pdt.+mu_cumulo+nu_cumulo+rho_cumulo+nu_per_ rho_cumulo+Arg_conc._cumulo+cell_yield_cumulo+RQ_integral+A ccum._Yield+emission_O2+emission_CO2+consum_O2+generate_CO 2; 0.774, 0.907, tmp+PL+interval_Pdt.+OD+RS+mu_cumulo+nu_cu mulo+rho_cumulo+nu_per_rho_cumulo+rab_integral+RQ_integral+ emission_CO2+consum_O2+generate_CO2; 0.770, 0.907, tmp+PL+i nterval_Pdt.+RS+mu_cumulo+nu_cumulo+rho_cumulo+nu_per_rho_c umulo+rab_integral+RQ_integral+Accum._Yield+emission_O2+emi ssion_CO2+consum_O2+generate_CO2; 0.783, 0.907, tmp+PL+OD+R S+nu_cumulo+rho_cumulo+nu_per_rho_cumulo+Arg_conc._cumulo+r ab_integral+emission_CO2+consum_O2+generate_CO2; 0.765, 0.9 07, tmp+PL+AN+interval_Pdt.+mu_cumulo+nu_cumulo+rho_cumulo+ nu_per_rho_cumulo+Arg_conc._cumulo+cell_yield_cumulo+rab_in tegral+Accum._Yield+emission_CO2+consum_O2+interval_O2+gene rate_CO2; 0.770, 0.907, tmp+pH+PL+interval_Pdt.+mu_cumulo+n u_cumulo+rho_cumulo+nu_per_rho_cumulo+cell_yield_cumulo+rab _integral+RQ_integral+Accum._Yield+emission_CO2+consum_O2+g enerate_CO2; 0.778, 0.907, tmp+PL+interval_Pdt.+mu_cumulo+n u_cumulo+rho_cumulo+nu_per_rho_cumulo+Arg_conc._cumulo+Accu m._Yield+emission_O2+emission_CO2+consum_O2+generate_CO2; 0. 778, 0.907, tmp+PL+interval_Pdt.+OD+nu_cumulo+rho_cumulo+nu _per_rho_cumulo+Arg_conc._cumulo+cell_yield_cumulo+rab_inte gral+emission_CO2+consum_O2+generate_CO2; 0.770, 0.907, tmp +PL+interval_Pdt.+OD+RS+nu_cumulo+rho_cumulo+nu_per_rho_cum ulo+Arg_conc._cumulo+rab_integral+Accum._Yield+emission_CO2 +consum_O2+interval_O2+generate_CO2; 0.774, 0.907, tmp+PL+i nterval_Pdt.+mu_cumulo+nu_cumulo+rho_cumulo+nu_per_rho_cumu lo+Arg_conc._cumulo+cell_yield_cumulo+rab_integral+emission _CO2+consum_O2+interval_O2+generate_CO2; 0.778, 0.907, tmp+ PL+PL_cumulo+interval_Pdt.+OD+nu_cumulo+rho_cumulo+nu_per_r ho_cumulo+Arg_conc._cumulo+rab_integral+emission_CO2+consum _O2+generate_CO2; 0.778, 0.907, tmp+PL+AN+interval_Pdt.+RS+ mu_cumulo+rho_cumulo+rab_integral+RQ_integral+Accum._Yield+ emission_CO2+consum_O2+generate_CO2; 0.760, 0.907, tmp+PL+P L_cumulo+interval_Pdt.+OD+RS+nu_cumulo+rho_cumulo+nu_per_rh o_cumulo+Arg_conc._cumulo+RQ_integral+Accum._Yield+emission _O2+emission_CO2+consum_O2+interval_O2+generate_CO2; 0.778, 0.907, tmp+PL+interval_Pdt.+mu_cumulo+nu_cumulo+rho_cumulo +nu_per_rho_cumulo+Arg_conc._cumulo+emission_O2+emission_CO 2+consum_O2+interval_O2+generate_CO2; 0.765, 0.907, tmp+PL+ AN+interval_Pdt.+mu_cumulo+nu_cumulo+rho_cumulo+nu_per_rho_ cumulo+cell_yield_cumulo+rab_integral+RQ_integral+Accum._Yi eld+emission_CO2+consum_O2+interval_O2+generate_CO2; 0.778, 0.907, tmp+PL+AN+interval_Pdt.+mu_cumulo+rho_cumulo+rab_in tegral+RQ_integral+Accum._Yield+emission_CO2+consum_O2+inte rval_O2+generate_CO2; 0.783, 0.907, tmp+PL+AN+interval_Pdt. +mu_cumulo+rho_cumulo+rab_integral+RQ_integral+Accum._Yield +emission_CO2+consum_O2+generate_CO2; 0.765, 0.907, tmp+PL+ AN+interval_Pdt.+RS+mu_cumulo+nu_cumulo+rho_cumulo+nu_per_r ho_cumulo+Arg_conc._cumulo+rab_integral+RQ_integral+Accum._ Yield+emission_CO2+consum_O2+generate_CO2; 0.778, 0.907, tm p+pH+PL+interval_Pdt.+OD+mu_cumulo+nu_cumulo+rho_cumulo+nu_ per_rho_cumulo+rab_integral+emission_CO2+consum_O2+generate _CO2; 0.778, 0.907, tmp+PL+interval_Pdt.+RS+mu_cumulo+nu_cu mulo+rho_cumulo+nu_per_rho_cumulo+emission_O2+emission_CO2+ consum_O2+interval_O2+generate_CO2; 0.774, 0.907, tmp+PL+AN +interval_Pdt.+OD+RS+mu_cumulo+nu_cumulo+rho_cumulo+nu_per_ rho_cumulo+Accum._Yield+emission_CO2+consum_O2+generate_CO 2; 0.774, 0.907, tmp+PL+PL_cumulo+interval_Pdt.+mu_cumulo+n u_cumulo+rho_cumulo+nu_per_rho_cumulo+cell_yield_cumulo+rab _integral+RQ_integral+emission_CO2+consum_O2+generate_CO2; 0.765, 0.907, tmp+PL+interval_Pdt.+mu_cumulo+nu_cumulo+rho_ cumulo+nu_per_rho_cumulo+Arg_conc._cumulo+cell_yield_cumulo +rab_integral+RQ_integral+Accum._Yield+emission_O2+emission _CO2+consum_O2+generate_CO2; 0.769, 0.907, tmp+PL+interval_ Pdt.+mu_cumulo+nu_cumulo+rho_cumulo+nu_per_rho_cumulo+Arg_c onc._cumulo+rab_integral+RQ_integral+Accum._Yield+emission_ O2+emission_CO2+consum_O2+generate_CO2; 0.760, 0.907, tmp+P L+PL_cumulo+interval_Pdt.+OD+mu_cumulo+nu_cumulo+rho_cumulo +nu_per_rho_cumulo+Arg_conc._cumulo+rab_integral+RQ_integra l+Accum._Yield+emission_O2+emission_CO2+consum_O2+generate-CO2; 0.765, 0.907, tmp+pH+PL+PL_cumulo+AN+interval_Pdt.+mu_ cumulo+nu_cumulo+rho_cumulo+nu_per_rho_cumulo+Accum._Yield+ emission_O2+emission_CO2+consum_O2+interval_O2+generate_CO 2; 0.774, 0.907, tmp+PL+interval_Pdt.+mu_cumulo+nu_cumulo+r ho_cumulo+nu_per_rho_cumulo+Arg_conc._cumulo+cell_yield_cum ulo+RQ_integral+emission_O2+emission_CO2+consum_O2+generate _CO2; 0.778, 0.907, tmp+PL+PL_cumulo+AN+interval_Pdt.+OD+nu _cumulo+rho_cumulo+nu_per_rho_cumulo+rab_integral+emission_ CO2+consum_O2+generate_CO2; 0.778, 0.907, tmp+PL+OD+nu_cumu lo+rho_cumulo+nu_per_rho_cumulo+Arg_conc._cumulo+Accum._Yie ld+emission_O2+emission_CO2+consum_O2+interval_O2+generate CO2; 0.769, 0.907, tmp+pH+PL+interval_Pdt.+OD+nu_cumulo+rho _cumulo+nu_per_rho_cumulo+Arg_conc._cumulo+rab_integral+RQ_ integral+Accum._Yield+emission_CO2+consum_O2+generate-CO2; 0.769, 0.907, tmp+pH+PL+AN+interval_Pdt.+mu_cumulo+nu_cumul o+rho_cumulo+nu_per_rho_cumulo+cell_yield_cumulo+rab_integr al+Accum._Yield+emission_CO2+consum_O2+generate_CO2; 0.760, 0.907, tmp+PL+PL_cumulo+AN+interval_Pdt.+OD+nu_cumulo+rho_ cumulo+nu_per_rho_cumulo+Arg_conc._cumulo+RQ_integral+Accum. _Yield+emission_O2+emission_CO2+consum_O2+interval_O2+gener ate_CO2; 0.760, 0.907, tmp+PL+PL_cumulo+AN+interval_Pdt.+OD +nu_cumulo+rho_cumulo+nu_per_rho_cumulo+Arg_conc._cumulo+ra b_integral+RQ_integral+Accum._Yield+emission_O2+emission_CO 2+consum_O2+generate_CO2; 0.786, 0.907, tmp+PL+PL_cumulo+AN +interval_Pdt.+mu_cumulo+rho_cumulo+Accum._Yield+emission_C O2+consum_O2+generate_CO2; 0.774, 0.907, tmp+PL+interval_Pd t.+RS+mu_cumulo+nu_cumulo+rho_cumulo+nu_per_rho_cumulo+cell _yield_cumulo+RQ_integral+emission_O2+emission_CO2+consum_O 2+generate_CO2; 0.782, 0.907, tmp+PL_cumulo+interval_Pdt.+m u_cumulo+nu_cumulo+rho_cumulo+nu_per_rho_cumulo+Arg_conc._c umulo+cell_yield_cumulo+emission_CO2+consum_O2+generate_CO 2; 0.774, 0.907, tmp+PL+PL_cumulo+interval_yield+interval_P dt.+mu_cumulo+nu_cumulo+rho_cumulo+nu_per_rho_cumulo+rab_in tegral+RQ_integral+emission_CO2+consum_O2+generate_CO2 0.7 69, 0.907, tmp+PL+AN+interval_Pdt.+OD+RS+mu_cumulo+nu_cumul o+rho_cumulo+nu_per_rho_cumulo+rab_integral+Accum._Yield+em ission_CO2+consum_O2+generate_CO2; 0.774, 0.907, tmp+PL+AN+ interval_Pdt.+mu_cumulo+nu_cumulo+rho_cumulo+nu_per_rho_cum ulo+Arg_conc._cumulo+rab_integral+RQ_integral+emission_CO2+ consum_O2+generate_CO2; 0.760, 0.907, tmp+PL+PL_cumulo+inte rval_Pdt.+RS+mu_cumulo+nu_cumulo+rho_cumulo+nu_per_rho_cumu lo+Arg_conc._cumulo+rab_integral+RQ_integral+Accum._Yield+e mission_O2+emission_CO2+consum_O2+generate_CO2; 0.782, 0.90 7, tmp+pH+PL+interval_Pdt.+mu_cumulo+nu_cumulo+rho_cumulo+n u_per_rho_cumulo+emission_O2+emission_CO2+consum_O2+generat e_CO2; 0.778, 0.907, tmp+PL+interval_Pdt.+OD+nu_cumulo+rho_ cumulo+nu_per_rho_cumulo+rab_integral+RQ_integral+emission_ CO2+consum_O2+interval_O2+generate_CO2; 0.774, 0.907, tmp+P L+interval_Pdt.+OD+mu_cumulo+nu_cumulo+rho_cumulo+nu_per_rh o_cumulo+RQ_integral+emission_O2+emission_CO2+consum_O2+int erval_O2+generate_CO2; 0.790, 0.907, tmp+PL_cumulo+OD+nu_cu mulo+rho_cumulo+nu_per_rho_cumulo+Arg_conc._cumulo+rab_inte gral+emission_CO2+generate_CO2; 0.786, 0.907, tmp+OD+nu_cum ulo+rho_cumulo+nu_per_rho_cumulo+Arg_conc._cumulo+rab-integ ral+Accum._Yield+emission_CO2+consum_O2+generate_CO2; 0.769, 0.907, tmp+PL+AN+interval_Pdt.+RS+mu_cumulo+nu_cumulo+rho_ cmulo+nu_per_rho_cumulo+Accum._Yield+emission_O2+emission CO2+consum_O2+interval_O2+generate_CO2; 0.769, 0.907, tmp+P L+AN+interval_Pdt.+OD+mu_cumulo+nu_cumulo+rho_cumulo+nu_per _rho_cumulo+Arg_conc._cumulo+rab_integral+emission_CO2+cons um_O2+interval_O2+generate_CO2; 0.778, 0.907, tmp+PL_cumulo +OD+RS+nu_cumulo+rho_cumulo+nu_per_rho_cumulo+Arg_conc._cum ulo+rab_integral+Accum._Yield+emission_CO2+consum_O2+genera te_CO2; 0.774, 0.907, tmp+PL+interval_Pdt.+RS+mu_cumulo+nu_ cumulo+rho_cumulo+nu_per_rho_cumulo+rab_integral+Accum._Yie ld+emission_CO2+consum_O2+interval_O2+generate_CO2; 0.782, 0.907, tmp+PL_cumulo+RS+mu_cumulo+nu_cumulo+rho_cumulo+nu_p er_rho_cumulo+cell_yield_cumulo+rab_integral+emission_CO2+c onsum_O2+generate_CO2; 0.778, 0.907, tmp+PL+interval_Pdt.+m u_cumulo+nu_cumulo+rho_cumulo+nu_per_rho_cumulo+rab_integra l+emission_O2+emission_CO2+consum_O2+interval_O2+generate_C 02; 0.769, 0.907, tmp+pH+PL+interval_Pdt.+mu_cumulo+nu_cumu lo+rho_cumulo+nu_per_rho_cumulo+cell_yield_cumulo+RQ_integr al+Accum._Yield+emission_O2+emission_CO2+consum_O2+generate _CO2; 0.774, 0.907, tmp+PL+interval_Pdt.+OD+nu_cumulo+rho_c umulo+nu_per_rho_cumulo+RQ_integral+Accum._Yield+emission_O 2+emission_CO2+consum_O2+interval_O2+generate_CO2; 0.769, 0. 907, tmp+PL+PL_cumulo+interval_Pdt.+mu_cumulo+nu_cumulo+rho _cumulo+nu_per_rho_cumulo+Arg_conc._cumulo+cell_yield_cumul o+rab_integral+Accum._Yield+emission_CO2+consum_O2+generate _CO2; 0.782, 0.907, tmp+PL+AN+interval_Pdt.+OD+nu_cumulo+rh o_cumulo+nu_per_rho_cumulo+Arg_conc._cumulo+emission_CO2+co nsum_O2+generate_CO2; 0.778, 0.907, tmp+pH+PL+interval_Pdt. +RS+mu_cumulo+nu_cumulo+rho_cumulo+nu_per_rho_cumulo+rab_in tegral+emission_CO2+consum_O2+generate_CO2; 0.782, 0.907, t mp+PL+interval_Pdt.+OD+nu_cumulo+rho_cumulo+nu_per_rho_cumu lo+Accum._Yield+emission_O2+emission_CO2+consum_O2+generate _CO2; 0.769, 0.907, tmp+PL+AN+interval_Pdt.+OD+RS+mu_cumulo +nu_cumulo+rho_cumulo+nu_per_rho_cumulo+Arg_conc._cumulo+Ac cum._Yield+emission_CO2+consum_O2+generate_CO2; 0.778, 0.90 7, tmp+PL+AN+OD+nu_cumulo+rho_cumulo+nu_per_rho_cumulo+Arg_ conc._cumulo+rab_integral+Accum._Yield+emission_CO2+consum_ O2+generate_CO2; 0.765, 0.907, tmp+PL+AN+interval_Pdt.+RS+m u_cumulo+nu_cumulo+rho_cumulo+nu_per_rho_cumulo+cell_yield_ cumulo+rab_integral+RQ_integral+Accum._Yield+emission_CO2+c onsum_O2+generate_CO2; 0.778, 0.907, tmp+pH+OD+mu_cumulo+nu _cumulo+rho_cumulo+nu_per_rho_cumulo+Arg_conc._cumulo+rab_i ntegral+Accum._Yield+emission_CO2+consum_O2+generate_CO2; 0. 774, 0.907, tmp+PL+PL_cumulo+interval_Pdt.+mu_cumulo+nu_cum ulo+rho_cumulo+nu_per_rho_cumulo+Arg_conc._cumulo+emission_ O2+emission_CO2+consum _O2+interval_O2+generate_CO2; 0.782, 0.907, tmp+PL+interval_Pdt.+OD+nu_cumulo+rho_cumulo+nu_per_ rho_cumulo+Arg_conc._cumulo+Accum._Yield+emission_CO2+consu m_O2+generate_CO2; 0.774, 0.907, tmp+PL+AN+interval_Pdt.+mu _cumulo+nu_cumulo+rho_cumulo+nu_per_rho_cumulo+RQ_integral+ emission_O2+emission_CO2+consum_O2+interval_O2+generate_CO 2; 0.786, 0.907, tmp+PL+interval_Pdt.+mu_cumulo+nu_cumulo+r ho_cumulo+nu_per_rho_cumulo+RQ_integral+emission_CO2+consum _O2+generate_CO2; 0.786, 0.907, tmp+interval_Pdt.+mu_cumulo +nu_cumulo+rho_cumulo+nu_per_rho_cumulo+rab_integral+RQ_int egral+emission_CO2+consum _O2+generate_CO2; 0.769, 0.907, tm p+pH+PL+PL_cumulo+interval_Pdt.+mu_cumulo+nu_cumulo+rho_cum ulo+nu_per_rho_cumulo+RQ_integral+Accum._Yield+emission_O2+ emission_CO2+consum_O2+generate_CO2; 0.774, 0.907, tmp+PL+P L_cumulo+interval_Pdt.+OD+mu_cumulo+nu_cumulo+rho_cumulo+nu _per_rho_cumulo+rab_integral+Accum._Yield+emission_CO2+cons um_O2+generate_CO2; 0.774, 0.907, tmp+PL+AN+interval Pdt.+O D+mu_cumulo+nu_cumulo+rho_cumulo+nu_per_rho_cumulo+rab_inte gral+RQ_integral+emission_CO2+consum_O2+generate_CO2; 0.760, 0.907, tmp+PL+PL_cumulo+AN+interval_Pdt.+OD+RS+nu_cumulo+r ho_cumulo+nu_per_rho_cumulo+Arg_conc._cumulo+rab_integral+A ccum._Yield+emission_O2+emission_CO2+consum_O2+generate_CO 2; 0.774, 0.907, tmp+PL+interval_Pdt.+OD+mu_cumulo+nu_cumul o+rho_cumulo+nu_per_rho_cumulo+cell_yield_cumulo+rab_integr al+emission_CO2+consum_O2+interval_O2+generate_CO2; 0.786, 0.907, tmp+pH+PL+AN+interval_Pdt.+mu_cumulo+rho_cumulo+Accu m._Yield+emission_CO2+consum_O2+generate_CO2; 0.774, 0.907, tmp+PL+PL_cumulo+AN+interval_Pdt.+mu_cumulo+nu_cumulo+rho_ cumulo+nu_per_rho_cumulo+Arg_conc._cumulo+emission_O2+emiss ion_CO2+consum_O2+generate_CO2; 0.782, 0.907, tmp+PL_cumulo +OD+mu_cumulo+nu_cumulo+rho_cumulo+nu_per_rho_cumulo+cell_y ield_cumulo+rab_integral+emission_CO2+consum_O2+generate_CO 2; 0.778, 0.907, tmp+PL+PL_cumulo+interval_Pdt.+OD+nu_cumul o+rho_cumulo+nu_per_rho_cumulo+Arg_conc._cumulo+emission_O2 +emission_CO2+consum_O2+generate_CO2; 0.778, 0.907, tmp+PL_ cumulo+AN+interval_Pdt.+mu_cumulo+nu_cumulo+rho_cumulo+nu_p er_rho_cumulo+cell_yield_cumulo+Accum._Yield+emission_CO2+c onsum_O2+generate_CO2; 0.760, 0.907, tmp+PL+PL_cumulo+AN+in terval_Pdt.+OD+mu_cumulo+nu_cumulo+rho_cumulo+nu_per_rho_cu mulo+Arg_conc._cumulo+rab_integral+Accum._Yield+emission_O2 +emission_CO2+consum_O2+generate_CO2; 0.782, 0.907, tmp+PL+ OD+nu_cumulo+rho_cumulo+nu_per_rho_cumulo+Arg_conc._cumulo+ cell_yield_cumulo+rab_integral+emission_CO2+consum_O2+gener ate_CO2; 0.778, 0.907, tmp+pH+PL+interval_Pdt.+mu_cumulo+nu _cumulo+rho_cumulo+nu_per_rho_cumulo+Arg_conc._cumulo+rab_i ntegral+emission_CO2+consum_O2+generate_CO2; 0.774, 0.907, tmp+PL+interval_Pdt.+OD+mu_cumulo+nu_cumulo+rho_cumulo+nu_p er_rho_cumulo+cell_yield_cumulo+RQ_integral+emission_O2+emi ssion_CO2+consum_O2+generate_CO2; 0.782, 0.907, tmp+PL_cumu lo+interval_Pdt.+OD+mu_cumulo+nu_cumulo+rho_cumulo+nu_per_r ho_cumulo+Arg_conc._cumulo+emission_CO2+consum_O2+generate-CO2; 0.778, 0.907, tmp+PL_cumulo+interval_Pdt.+OD+RS+mu_cum ulo+nu_cumulo+rho_cumulo+nu_per_rho_cumulo+rab_integral+emi ssion_CO2+consum_O2+generate_CO2; 0.774, 0.907, tmp+PL+AN+i nterval_Pdt.+RS+mu_cumulo+nu_cumulo+rho_cumulo+nu_per_rho_c umulo+cell_yield_cumulo+emission_O2+emission_CO2+consum_O2+ generate_CO2; 0.774, 0.907, tmp+pH+PL+interval_Pdt.+mu_cumu lo+nu_cumulo+rho_cumulo+nu_per_rho_cumulo+cell_yield_cumulo +rab_integral+RQ_integral+emission_CO2+consum_O2+generate_C 02; 0.774, 0.907, tmp+PL+AN+interval_Pdt.+OD+mu_cumulo+nu_c umulo+rho_cumulo+nu_per_rho_cumulo+cell_yield_cumulo+emissi on_O2+emission_CO2+consum_O2+generate_CO2; 0.764, 0.907, tm p+PL+PL_cumulo+interval_Pdt.+OD+RS+mu_cumulo+nu_cumulo+rho_ cumulo+nu_per_rho_cumulo+Arg_conc._cumulo+Accum._Yield+emis sion_O2+emission_CO2+consum_O2+generate_CO2; 0.764, 0.907, tmp+PL+PL_cumulo+AN+interval_Pdt.+OD+mu_cumulo+nu_cumulo+rh o_cumulo+nu_per_rho_cumulo+rab_integral+Accum._Yield+emissi on_CO2+consum_O2+interval_O2+generate_CO2; 0.782, 0.907, tm p+PL+interval_Pdt.+OD+nu_cumulo+rho_cumulo+nu_per_rho_cumul o+Arg_conc._cumulo+rab_integral+Accum._Yield+emission_CO2+g enerate_CO2; 0.778, 0.907, tmp+pH+PL_cumulo+interval_Pdt.+m u_cumulo+nu_cumulo+rho_cumulo+nu_per_rho_cumulo+cell_yield_ cumulo+rab_integral+emission_CO2+consum_O2+generate_CO2; 0. 759, 0.907, tmp+PL+PL_cumulo+interval_Pdt.+OD+RS+nu_cumulo+ rho_cumulo+nu_per_rho_cumulo+Arg_conc._cumulo+rab_integral+ RQ_integral+Accum._Yield+emission_O2+emission_CO2+consum_O2 +generate_CO2; 0.764, 0.907, tmp+PL+PL_cumulo+interval_Pdt. +OD+RS+mu_cumulo+nu_cumulo+rho_cumulo+nu_per_rho_cumulo+Arg _conc._cumulo+rab_integral+Accum._Yield+emission_CO2+consum _O2+generate_CO2; 0.774, 0.907, tmp+PL+PL_cumulo+interval_P dt.+mu_cumulo+nu_cumulo+rho_cumulo+nu_per_rho_cumulo+rab_in tegral+RQ_integral+emission_CO2+consum_O2+interval_O2+gener ate_CO2; 0.774, 0.907, tmp+PL+interval_Pdt.+mu_cumulo+nu_cu mulo+rho_cumulo+nu_per_rho_cumulo+cell_yield_cumulo+rab_int egral+Accum._Yield+emission_O2+emission_CO2+consum_O2+gener ate_CO2; 0.769, 0.907, tmp+PL+interval_Pdt.+OD+mu_cumulo+nu _cumulo+rho_cumulo+nu_per_rho_cumulo+rab_integral+RQ_integr al+Accum._Yield+emission_CO2+consum_O2+interval_O2+generate _CO2; 0.764, 0.907, tmp+PL+interval_Pdt.+OD+RS+mu_cumulo+nu _cumulo+rho_cumulo+nu_per_rho_cumulo+Arg_conc._cumulo+RQ_in tegral+Accum._Yield+emission_O2+emission_CO2+consum_O2+gene rate_CO2; 0.769, 0.907, tmp+PL+interval_Pdt.+RS+mu_cumulo+n u_cumulo+rho_cumulo+nu_per_rho_cumulo+rab_integral+RQ_integ ral+Accum._Yield+emission_CO2+consum_O2+interval_O2+generat e_CO2; 0.786, 0.907, tmp+PL+AN+interval_Pdt.+mu_cumulo+rho_ cumulo+RQ_integral+Accum._Yield+emission_CO2+consum_O2+gene rate_CO2; 0.782, 0.907, tmp+PL+interval_yield+interval_Pdt. +mu_cumulo+nu_cumulo+rho_cumulo+nu_per_rho_cumulo+rab_integ ral+emission_CO2+consum_O2+generate_CO2; 0.786, 0.907, tmp+ pH+PL_cumulo+interval_Pdt.+mu_cumulo+nu_cumulo+rho_cumulo+n u_per_rho_cumulo+emission_CO2+consum_O2+generate_CO2; 0.769, 0.907, tmp+PL+PL_cumulo+AN+interval_yield+interval_Pdt.+mu _cumulo+nu_cumulo+rho_cumulo+nu_per_rho_cumulo+rab_integral +emission_CO2+consum_O2+interval_O2+generate_CO2; 0.769, 0. 907, tmp+pH+PL+interval_Pdt.+OD+nu_cumulo+rho_cumulo+nu_per _rho_cumulo+Arg_conc._cumulo+RQ_integral+Accum._Yield+emiss ion_O2+emission_CO2+consum_O2+generate_CO2; 0.782, 0.907, t mp+PL_cumulo+interval_Pdt.+RS+mu_cumulo+nu_cumulo+rho_cumul o+nu_per_rho_cumulo+cell_yield_cumulo+emission_CO2+consuni_O 2+generate_CO2; 0.790, 0.907, tmp+PL+interval_Pdt.+mu_cumul o+rho_cumulo+rab_integral+Accum._Yield+emission_CO2+consum_ O2+generate_CO2; 0.778, 0.907, tmp+PL_cumulo+interval_Pdt.+ mu_cumulo+nu_cumulo+rho_cumulo+nu_per_rho_cumulo+cell_yield _cumulo+Accum._Yield+emission_O2+emission_CO2+consum_O2+gen erate_CO2; 0.769, 0.907, tmp+PL+AN+interval_Pdt.+OD+mu_cumu lo+nu_cumulo+rho_cumulo+nu_per_rho_cumulo+cell_yield_cumulo +emission_O2+emission_CO2+consum_O2+interval_O2+generate_CO 2; 0.773, 0.907, tmp+PL+interval_Pdt.+OD+nu_cumulo+rho_cumu lo+nu_per_rho_cumulo+Arg_conc._cumulo+rab_integral+Accum._Y ield+emission_O2+emission_CO2+consum_O2+generate_CO2; 0.764, 0.907, tmp+PL+AN+interval_Pdt.+OD+mu_cumulo+nu_cumulo+rho_ cumulo+nu_per_rho_cumulo+cell_yield_cumulo+rab_integral+Acc um._Yield+emission_CO2+consum_O2+interval_O2+generate_CO2; 0.773, 0.907, tmp+PL+OD+RS+mu_cumulo+nu_cumulo+rho_cumulo+n u_per_rho_cumulo+Arg_conc._cumulo+rab_integral+Accum.-Yield +emission_CO2+consum_O2+generate_CO2; 0.769, 0.907, tmp+PL_ cumulo+interval_Pdt.+mu_cumulo+nu_cumulo+rho_cumulo+nu_per_ rho_cumulo+Arg_conc._cumulo+cell_yield_cumulo+rab_integral+ RQ_integral+Accum._Yield+emission_CO2+consum_O2+generate_CO 2; 0.769, 0.907, tmp+PL+AN+interval_Pdt.+mu_cumulo+nu_cumul o+rho_cumulo+nu_per_rho_cumulo+Arg_conc._cumulo+cell_yield_ cumulo+Accum._Yield+emission_O2+emission_CO2+consum_O2+gene rate_CO2; 0.778, 0.907, tmp+pH+PL+interval_Pdt.+mu_cumulo+n u_cumulo+rho_cumulo+nu_per_rho_cumulo+rab_integral+emission _CO2+consum_O2+interval_O2+generate_CO2; 0.764, 0.907, tmp+ PL+PL_cumulo+AN+interval_Pdt.+mu_cumulo+nu_cumulo+rho_cumul o+nu_per_rho_cumulo+Arg_conc._cumulo+rab_integral+RQ_integr al+Accum._Yield+emission_CO2+consum_O2+generate_CO2; 0.769, 0.907, tmp+PL+PL_cumulo+interval_Pdt.+mu_cumulo+nu_cumulo+ rho_cumulo+nu_per_rho_cumulo+cell_yield_cumulo+rab_integral +RQ_integral+Accum._Yield+emission_CO2+consum_O2+generate_C 02; 0.790, 0.907, tmp+PL+OD+nu_cumulo+rho_cumulo+nu_per_rho _cumulo+emission_O2+emission_CO2+consum_O2+generate_CO2; 0. 782, 0.907, tmp+pH+interval_Pdt.+mu_cumulo+nu_cumulo+rho_cu mulo+nu_per_rho_cumulo+cell_yield_cumulo+rab_integral+emiss ion_CO2+consum_O2+generate_CO2; 0.773, 0.907, tmp+PL+interv al_Pdt.+OD+mu_cumulo+nu_cumulo+rho_cumulo+nu_per_rho_cumulo +Accum._Yield+emission_O2+emission_CO2+consum _O2+interval_O 2+generate_CO2; 0.773, 0.907, tmp+PL+interval_Pdt.+RS+mu_cu mulo+nu_cumulo+rho_cumulo+nu_per_rho_cumulo+rab_integral+RQ _integral+emission_CO2+consum_O2+interval_O2+generate_CO2; 0.773, 0.907, tmp+PL+AN+interval_Pdt.+OD+RS+nu_cumulo+rho_c umulo+nu_per_rho_cumulo+Arg_conc._cumulo+Accum._Yield+emiss ion_CO2+consum_O2+generate_CO2; 0.773, 0.907, tmp+PL_cumulo +interval_Pdt.+OD+mu_cumulo+nu_cumulo+rho_cumulo+nu_per_rho _cumulo+cell_yield_cumulo+rab_integral+Accum._Yield+emissio n_CO2+consum_O2+generate_CO2; 0.782, 0.907, tmp+PL+interval _Pdt.+OD+nu_cumulo+rho_cumulo+nu_per_rho_cumulo+cell_yield_ cumulo+rab_integral+emission_CO2+consum_O2+generate_CO2; 0. 764, 0.907, tmp+PL+PL_cumulo+interval_Pdt.+OD+mu_cumulo+nu_ cumulo+rho_cumulo+nu_per_rho_cumulo+Arg_conc._cumulo+Accum. _Yield+emission_O2+emission_CO2+consum_O2+interval_O2+gener ate_CO2; 0.790, 0.907, tmp+OD+nu_cumulo+rho_cumulo+nu_per_r ho_cumulo+Arg_conc._cumulo+rab_integral+Accum._Yield+emissi on_CO2+generate_CO2; 0.782, 0.907, tmp+PL+interval_Pdt.+OD+ RS+nu_cumulo+rho_cumulo+nu_per_rho_cumulo+rab_integral+emis sion_CO2+consum_O2+generate_CO2; 0.786, 0.907, tmp+PL+AN+in terval_yield+interval_Pdt.+mu_cumulo+rho_cumulo+nu_per_rho_ cumulo+emission_CO2+consum_O2+generate_CO2; 0.782, 0.907, t mp+PL+AN+interval_yield+interval_Pdt.+mu_cumulo+rho_cumulo+ nu_per_rho_cumulo+emission_CO2+consum_O2+interval_O2+genera te_CO2; 0.769, 0.907, tmp+pH+PL+AN+interval_Pdt.+mu_cumulo+ nu_cumulo+rho_cumulo+nu_per_rho_cumulo+rab_integral+Accum._ Yield+emission_CO2+consum_O2+interval_O2+generate_CO2; 0.75 9, 0.907, tmp+pH+PL+PL_cumulo+AN+interval_Pdt.+OD+nu_cumulo +rho_cumulo+nu_per_rho_cumulo+Arg_conc._cumulo+Accum._Yield +emission_O2+emission_CO2+consum_O2+interval_O2+generate_CO 2; 0.773, 0.907, tmp+PL+interval_Pdt.+OD+mu_cumulo+nu_cumul o+rho_cumulo+nu_per_rho_cumulo+rab_integral+RQ_integral+emi ssion_CO2+consum_O2+interval_O2+generate_CO2; 0.764, 0.907, tmp+PL+interval_Pdt.+OD+mu_cumulo+nu_cumulo+rho_cumulo+nu_ per_rho_cumulo+cell_yield_cumulo+RQ_integral+Accum._Yield+e mission_O2+emission_CO2+consum_O2+interval_O2+generate_CO2; 0.786, 0.907, tmp+PL_cumulo+interval_Pdt.+mu_cumulo+nu_cum ulo+rho_cumulo+nu_per_rho_cumulo+emission_O2+emission_CO2+c onsum_O2+generate_CO2; 0.759, 0.907, tmp+pH+PL+PL_cumulo+in terval_Pdt.+OD+nu_cumulo+rho_cumulo+nu_per_rho_cumulo+Arg_c onc._cumulo+RQ_integral+Accum._Yield+emission_O2+emission_C O2+consum_O2+interval_O2+generate_CO2; 0.773, 0.907, tmp+PL +PL_cumulo+interval_Pdt.+mu_cumulo+nu_cumulo+rho_cumulo+nu_ per_rho_cumulo+rab_integral+RQ_integral+emission_O2+emissio n_CO2+consum_O2+generate_CO2; 0.769, 0.907, tmp+PL+interval _Pdt.+OD+RS+mu_cumulo+nu_cumulo+rho_cumulo+nu_per_rho_cumul o+Arg_conc._cumulo+Accum._Yield+emission_O2+emission_CO2+co nsum_O2+generate_CO2; 0.782, 0.907, tmp+PL+interval_Pdt.+mu _cumulo+nu_cumulo+rho_cumulo+nu_per_rho_cumulo+Accum._Yield +emission_CO2+consum _O2+interval_O2+generate_CO2; 0.778, 0. 907, tmp+PL+PL_cumulo+AN+interval_Pdt.+mu_cumulo+nu_cumulo+ rho_cumulo+nu_per_rho_cumulo+cell_yield_cumulo+emission_CO2 +consum_O2+generate_CO2; 0.769, 0.907, tmp+PL+PL_cumulo+int erval_Pdt.+mu_cumulo+nu_cumulo+rho_cumulo+nu_per_rho_cumulo +Arg_conc._cumulo+rab_integral+RQ_integral+Accum._Yield+emi ssion_CO2+consum_O2+generate_CO2; 0.769, 0.907, tmp+PL+PL_c umulo+AN+interval_yield+interval_Pdt.+mu_cumulo+nu_cumulo+r ho_cumulo+nu_per_rho_cumulo+Arg_conc._cumulo+rab_integral+e mission_CO2+consum_O2+generate_CO2; 0.773, 0.907, tmp+PL+in terval_Pdt.+mu_cumulo+nu_cumulo+rho_cumulo+nu_per_rho_cumul o+Arg_conc._cumulo+cell_yield_cumulo+emission_O2+emission_C O2+consum_O2+interval_O2+generate_CO2; 0.769, 0.907, tmp+PL +interval_Pdt.+mu_cumulo+nu_cumulo+rho_cumulo+nu_per_rho_cu mulo+Arg_conc._cumulo+rab_integral+RQ_integral+Accum.-Yield +emission_CO2+consum_O2+interval_O2+generate_CO2; 0.773, 0. 907, tmp+pH+PL+AN+interval_Pdt.+mu_cumulo+nu_cumulo+rho_cum ulo+nu_per_rho_cumulo+Accum._Yield+emission_CO2+consum_O2+i nterval_O2+generate_CO2; 0.782, 0.907, tmp+PL_cumulo+interv al_Pdt.+OD+nu_cumulo+rho_cumulo+nu_per_rho_cumulo+Arg-conc. _cumulo+rab_integral+Accum._Yield+emission_CO2+generate_CO 2; 0.786, 0.907, tmp+pH+mu_cumulo+nu_cumulo+rho_cumulo+nu_p er_rho_cumulo+cell_yield_cumulo+rab_integral+emission_CO2+c onsum_O2+generate_CO2; 0.778, 0.907, tmp+PL_cumulo+interval _Pdt.+OD+mu_cumulo+nu_cumulo+rho_cumulo+nu_per_rho_cumulo+A rg_conc._cumulo+rab_integral+emission_CO2+consum_O2+generat e_CO2; 0.778, 0.907, tmp+PL+interval_Pdt.+OD+nu_cumulo+rho_ cumulo+nu_per_rho_cumulo+Arg_conc._cumulo+rab_integral+RQ_i ntegral+emission_CO2+consum_O2+generate_CO2; 0.769, 0.907, tmp+PL+PL_cumulo+AN+interval_yield+interval_Pdt.+RS+mu_cumu lo+nu_cumulo+rho_cumulo+nu_per_rho_cumulo+rab_integral+emis sion_CO2+consum_O2+generate_CO2; 0.773, 0.907, tmp+pH+inter val_Pdt.+OD+mu_cumulo+nu_cumulo+rho_cumulo+nu_per_rho_cumul o+Arg_conc._cumulo+rab_integral+Accum._Yield+emission_CO2+c onsum_O2+generate_CO2; 0.790, 0.907, tmp+PL+interval_Pdt.+m u_cumulo+rho_cumulo+nu_per_rho_cumulo+rab_integral+emission _CO2+consum_O2+generate_CO2; 0.773, 0.907, tmp+PL_cumulo+in terval_Pdt.+RS+mu_cumulo+nu_cumulo+rho_cumulo+nu_per_rho_cu mulo+Arg_conc._cumulo+cell_yield_cumulo+rab_integral+emissi on_CO2+consum_O2+generate_CO2; 0.773, 0.907, tmp+PL+PL_cumu lo+interval_Pdt.+OD+mu_cumulo+nu_cumulo+rho_cumulo+nu_per_r ho_cumulo+Accum._Yield+emission_O2+emission_CO2+consum_O2+g enerate_CO2; 0.778, 0.907, tmp+PL_cumulo+interval_Pdt.+mu_c umulo+nu_cumulo+rho_cumulo+nu_per_rho_cumulo+Arg_conc._cumu lo+rab_integral+RQ_integral+emission_CO2+consum_O2+generate _CO2; 0.764, 0.907, tmp+PL+PL_cumulo+AN+interval_Pdt.+RS+mu _cumulo+nu_cumulo+rho_cumulo+nu_per_rho_cumulo+Arg_conc._cu mulo+Accum._Yield+emission_O2+emission_CO2+consum_O2+genera te_CO2; 0.769, 0.907, tmp+PL+interval_Pdt.+OD+nu_cumulo+rho _cumulo+nu_per_rho_cumulo+Arg_conc._cumulo+rab_integral+RQ_ integral+Accum._Yield+emission_O2+emission_CO2+consum-O2+ge nerate_CO2; 0.769, 0.907, tmp+PL+PL_cumulo+AN+interval_yiel d+interval_Pdt.+mu_cumulo+nu_cumulo+rho_cumulo+nu_per_rho_c umulo+rab_integral+Accum._Yield+emission_CO2+consum_O2+gene rate_CO2; 0.773, 0.907, tmp+pH+interval_Pdt.+mu_cumulo+nu_c umulo+rho_cumulo+nu_per_rho_cumulo+Arg_conc._cumulo+rab_int egral+RQ_integral+Accum._Yield+emission_CO2+consum_O2+gener ate_CO2; 0.773, 0.907, tmp+PL+interval_Pdt.+RS+mu_cumulo+nu _cumulo+rho_cumulo+nu_per_rho_cumulo+cell_yield_cumulo+rab_ integral+emission_CO2+consum_O2+interval_O2+generate_CO2; 0. 769, 0.907, tmp+PL+AN+interval_Pdt.+mu_cumulo+nu_cumulo+rho _cumulo+nu_per_rho_cumulo+cell_yield_cumulo+rab_integral+RQ _integral+emission_CO2+consum_O2+interval_O2+generate_CO2; 0.764, 0.907, tmp+PL+AN+interval_Pdt.+OD+mu_cumulo+nu_cumul o+rho_cumulo+nu_per_rho_cumulo+cell_yield_cumulo+rab_integr al+RQ_integral+Accum._Yield+emission_CO2+consum_O2+generate _CO2; 0.769, 0.907, tmp+PL+interval_Pdt.+OD+mu_cumulo+nu_cu mulo+rho_cumulo+nu_per_rho_cumulo+rab_integral+RQ_integral+ Accum._Yield+emission_O2+emission_CO2+consum_O2+generate_CO 2; 0.769, 0.907, tmp+PL+AN+interval_Pdt.+OD+nu_cumulo+rho_c umulo+nu_per_rho_cumulo+Arg_conc._cumulo+Accum._Yield+emiss ion_O2+emission_CO2+consum_O2+interval_O2+generate_CO2 0.7 82, 0.907, tmp+PL+AN+interval_Pdt.+mu_cumulo+rho_cumulo+cel l_yield_cumulo+Accum._Yield+emission_CO2+consum_O2+interval _O2+generate_CO2; 0.769, 0.907, tmp+pH+PL+PL_cumulo+AN+inte rval_Pdt.+mu_cumulo+nu_cumulo+rho_cumulo+nu_per_rho_cumulo+ rab_integral+emission_CO2+consum_O2+interval_O2+generate_CO 2; 0.773, 0.907, tmp+PL+AN+interval_Pdt.+mu_cumulo+nu_cumul o+rho_cumulo+nu_per_rho_cumulo+RQ_integral+Accum._Yield+emi ssion_CO2+consum_O2+interval_O2+generate_CO2; 0.773, 0.907, tmp+PL+interval_yield+interval_Pdt.+RS+mu_cumulo+nu_cumulo +rho_cumulo+nu_per_rho_cumulo+rab_integral+RQ_integral+emis sion_CO2+consum_O2+generate_CO2; 0.777, 0.907, tmp+PL+PL_cu mulo+AN+interval_Pdt.+RS+mu_cumulo+nu_cumulo+rho_cumulo+nu_ per_rho_cumulo+emission_CO2+consum_O2+generate_CO2; 0.777, 0.907, tmp+PL+interval_Pdt.+RS+mu_cumulo+nu_cumulo+rho_cumu lo+nu_per_rho_cumulo+Arg_conc._cumulo+emission_O2+emission-CO2+consum_O2+generate_CO2; 0.782, 0.907, tmp+PL+interval_P dt.+RS+mu_cumulo+nu_cumulo+rho_cumulo+nu_per_rho_cumulo+Acc um._Yield+emission_CO2+consum_O2+generate_CO2; 0.768, 0.907, tmp+pH+PL+PL_cumulo+interval_Pdt.+mu_cumulo+nu_cumulo+rho_ cumulo+nu_per_rho_cumulo+Arg_conc._cumulo+rab_integral+Accu m._Yield+emission_CO2+consum_O2+generate_CO2; 0.786, 0.907, tmp+PL+AN+interval_Pdt.+mu_cumulo+rho_cumulo+rab_integral+ emission_CO2+consum_O2+nterval_O+generate_CO; 0.782, 0.9 07, tmp+PL+AN+interval_Pdt.+RS+mu_cumulo+rho_cumulo+nu_per_ rho_cumulo+Accum._Yield+emission_CO+consum_O+generate_CO 2; 0.768, 0.907, tmp+PL+PL_cumulo+interval_Pdt.+RS+mu_cumul o+nu_cumulo+rho_cumulo+nu_per_rho_cumulo+Arg_conc._cumulo+A ccum._Yield+emission_O2+emission_CO2+consum_O2+generate_CO 2; 0.759, 0.907, tmp+PL+PL_cumulo+interval_Pdt.+mu_cumulo+n u_cumulo+rho_cumulo+nu_per_rho_cumulo+Arg_conc._cumulo+rab-integral+RQ_integral+Accum._Yield+emission_O+emission_CO+ consum_O2+interval_O2+generate_CO2; 0.773, 0.907, tmp+PL+in terval_Pdt.+OD+mu_cumulo+nu_cumulo+rho_cumulo+nu_per_rho_cu mulo+cell_yield_cumulo+emission_O+emission_CO+consum_O+i nterval_O2+generate_CO2; 0.782, 0.907, tmp+PL_cumulo+AN+int erval_Pdt.+RS+mu_cumulo+nu_cumulo+rho_cumulo+nu_per_rho_cum ulo+emission_CO+consum_O+generate_CO; 0.768, 0.907, tmp+ PL+interval_Pdt.+OD+mu_cumulo+nu_cumulo+rho_cumulo+nu_per_r ho_cumulo+Arg_conc._cumulo+rab_integral+Accum._Yield+emissi on_CO2+consum_O2+interval_O2+generate_CO2; 0.759, 0.907, tm p+PL+PL_cumulo+AN+interval_Pdt.+OD+mu_cumulo+nu_cumulo+rho_ cumulo+nu_per_rho_cumulo+Arg_conc._cumulo+rab_integral+Accu m._Yield+emission_CO2+consum_O2+interval_O2+generate_CO2; 0. 781, 0.907, tmp+PL+AN+OD+nu_cumulo+rho_cumulo+nu_per_rho_cu mulo+Arg_conc._cumulo+rab_integral+emission_CO2+consum_O2+g enerate_CO2; 0.773, 0.907, tmp+pH+PL+interval_Pdt.+mu_cumul o+nu_cumulo+rho_cumulo+nu_per_rho_cumulo+Arg_conc._cumulo+r ab_integral+Accum._Yield+emission_CO2+consum_O2+generate_CO 2; 0.773, 0.907, tmp+PL_cumulo+interval_Pdt.+mu_cumulo+nu_c umulo+rho_cumulo+nu_per_rho_cumulo+Arg_conc._cumulo+rab_int egral+RQ_integral+Accum._Yield+emission_CO2+consum_O2+gener ate_CO2; 0.773, 0.907, tmp+PL+PL_cumulo+interval_Pdt.+mu_cu mulo+nu_cumulo+rho_cumulo+nu_per_rho_cumulo+Arg_conc.-cumul o+cell_yield_cumulo+rab_integral+emission_CO2+consum_O2+gen erate_CO2; 0.764, 0.907, tmp+PL+AN+interval_Pdt.+RS+mu_cumu lo+nu_cumulo+rho_cumulo+nu_per_rho_cumulo+cell_yield_cumulo +rab_integral+Accum._Yield+emission_CO2+consum_O2+interval_ O2+generate_CO2; 0.781, 0.907, tmp+interval_Pdt.+mu_cumulo+ nu_cumulo+rho_cumulo+nu_per_rho_cumulo+rab_integral+Accum._ Yield+emission_CO2+consum_O2+interval_O2+generate_CO2; 0.76 8, 0.907, tmp+PL_cumulo+interval_Pdt.+OD+mu_cumulo+nu_cumul o+rho_cumulo+nu_per_rho_cumulo+cell_yield_cumulo+rab_integr al+RQ_integral+Accum._Yield+emission_CO2+consum_O2+generate _CO2; 0.768, 0.907, tmp+PL+PL_cumulo+interval_Pdt.+mu_cumul o+nu_cumulo+rho_cumulo+nu_per_rho_cumulo+cell_yield_cumulo+ Accum._Yield+emission_O2+emission_CO2+consum_O2+interval_O2 +generate_CO2; 0.773, 0.907, tmp+PL+interval_Pdt.+OD+mu_cum ulo+nu_cumulo+rho_cumulo+nu_per_rho_cumulo+Arg_conc._cumulo +rab_integral+RQ_integral+emission_CO2+consum_O2+generate_C 02; 0.764, 0.907, tmp+PL+AN+interval_Pdt.+mu_cumulo+nu_cumu lo+rho_cumulo+nu_per_rho_cumulo+cell_yield_cumulo+RQ_integr al+Accum._Yield+emission_O2+emission_CO2+consum_O2+interval _O2+generate_CO2; 0.768, 0.907, tmp+PL+PL_cumulo+interval_P dt.+mu_cumulo+nu_cumulo+rho_cumulo+nu_per_rho_cumulo+Arg_co nc._cumulo+cell_yield_cumulo+Accum._Yield+emission_O2+emiss ion_CO2+consum_O2+generate_CO2; 0.777, 0.907, tmp+pH+PL_cum ulo+interval_Pdt.+mu_cumulo+nu_cumulo+rho_cumulo+nu_per_rho _cumulo+rab_integral+Accum._Yield+emission_CO2+consum_O2+ge nerate_CO2; 0.764, 0.907, tmp+PL+PL_cumulo+AN+interval_Pdt. +mu_cumulo+nu_cumulo+rho_cumulo+nu_per_rho_cumulo+Arg-conc. _cumulo+RQ_integral+Accum._Yield+emission_O2+emission-CO2+c onsum_O2+generate_CO2; 0.768, 0.907, tmp+PL+PL_cumulo+AN+in terval_Pdt.+OD+mu_cumulo+nu_cumulo+rho_cumulo+nu_per_rho_cu mulo+Accum._Yield+emission_O2+emission_CO2+consum_O2+genera te_CO2; 0.785, 0.907, tmp+PL+AN+interval_yield+interval_Pdt. +mu_cumulo+rho_cumulo+Arg_conc._cumulo+emission_CO2+consum_ O2+generate_CO2; 0.768, 0.907, tmp+PL+AN+interval_Pdt.+OD+m u_cumulo+nu_cumulo+rho_cumulo+nu_per_rho_cumulo+Arg_conc._c umulo+Accum._Yield+emission_O2+emission_CO2+consum_O2+gener ate_CO2; 0.777, 0.907, tmp+PL+AN+OD+nu_cumulo+rho_cumulo+nu _per_rho_cumulo+Arg_conc._cumulo+Accum._Yield+emission_O2+e mission_CO2+consum_O2+generate_CO2; 0.768, 0.907, tmp+PL+AN +interval_Pdt.+RS+mu_cumulo+nu_cumulo+rho_cumulo+nu_per_rho _cumulo+cell_yield_cumulo+Accum._Yield+emission_O2+emission _CO2+consum_O2+generate_CO2; 0.768, 0.907, tmp+PL+AN+interv al_Pdt.+RS+mu_cumulo+nu_cumulo+rho_cumulo+nu_per_rho_cumulo +Arg_conc._cumulo+cell_yield_cumulo+Accum._Yield+emission_C O2+consum_O2+generate_CO2; 0.777, 0.907, tmp+PL+AN+interval _yield+interval_Pdt.+mu_cumulo+nu_cumulo+rho_cumulo+nu_per_ rho_cumulo+cell_yield_cumulo+emission_CO2+consu_O2+generat e_CO2; 0.768, 0.906, tmp+PL+AN+interval_Pdt.+RS+mu_cumulo+n u_cumulo+rho_cumulo+nu_per_rho_cumulo+Arg_conc._cumulo+rab_ integral+emission_CO2+consum_O2+interval_O2+generate_CO2; 0. 781, 0.906, tmp+PL+PL_cumulo+OD+nu_cumulo+rho_cumulo+nu_per _rho_cumulo+Arg_conc._cumulo+emission_O2+emission_CO2+consu m_O2+generate_CO2; 0.781, 0.906, tmp+PL_cumulo+interval_Pdt. +mu_cumulo+nu_cumulo+rho_cumulo+nu_per_rho_cumulo+cell_yiel d_cumulo+emission_CO2+consum _O2+interval_O2+generate_CO2; 0. 768, 0.906, tmp+PL+interval_Pdt.+OD+RS+mu_cumulo+nu_cumulo+ rho_cumulo+nu_per_rho_cumulo+cell_yield_cumulo+rab_integral +Accum._Yield+emission_CO2+consum_O2+generate_CO2; 0.768, 0. 906, tmp+PL_cumulo+interval_Pdt.+RS+mu_cumulo+nu_cumulo+rho _cumulo+nu_per_rho_cumulo+Arg_conc._cumulo+cell_yield_cumul o+rab_integral+Accum._Yield+emission_CO2+consum_O2+generate _CO2; 0.773, 0.906, tmp+PL+AN+interval_Pdt.+mu_cumulo+nu_cu mulo+rho_cumulo+nu_per_rho_cumulo+Arg_conc._cumulo+cell_yie ld_cumulo+emission_CO2+consum_O2+interval_O2+generate_CO2; 0.781, 0.906, tmp+PL_cumulo+interval_Pdt.+OD+nu_cumulo+rho_ cumulo+nu_per_rho_cumulo+Arg_conc._cumulo+rab_integral+emis sion_CO2+consum_O2+generate_CO2; 0.773, 0.906, tmp+PL+AN+in terval_Pdt.+OD+nu_cumulo+rho_cumulo+nu_per_rho_cumulo+rab_i ntegral+RQ_integral+Accum._Yield+emission_CO2+consum_O2+gen erate_CO2; 0.763, 0.906, tmp+PL+AN+interval_Pdt.+OD+mu_cumu lo+nu_cumulo+rho_cumulo+nu_per_rho_cumulo+Arg_conc._cumulo+ rab_integral+RQ_integral+Accum._Yield+emission_CO2+consum_O 2+generate_CO2; 0.777, 0.906, tmp+PL_cumulo+AN+interval_Pdt. +OD+mu_cumulo+nu_cumulo+rho_cumulo+nu_per_rho_cumulo+Accum. _Yield+emission_CO2+consum_O2+generate_CO2; 0.777, 0.906, t mp+PL+PL_cumulo+AN+interval_Pdt.+OD+mu_cumulo+nu_cumulo+rho _cumulo+nu_per_rho_cumulo+emission_CO2+consum_O2+generate_C 02; 0.763, 0.906, tmp+PL+interval_Pdt.+RS+mu_cumulo+nu_cumu lo+rho_cumulo+nu_per_rho_cumulo+cell_yield_cumulo+RQ_integr al+Accum._Yield+emission_O2+emission_CO2+consum_O2+interval _O2+generate_CO2; 0.768, 0.906, tmp+PL+PL_cumulo+AN+interva l_Pdt.+RS+mu_cumulo+nu_cumulo+rho_cumulo+nu_per_rho_cumulo+ Arg_conc._cumulo+rab_integral+emission_CO2+consum_O2+genera te_CO2; 0.785, 0.906, tmp+PL+AN+interval_Pdt.+mu_cumulo+nu_ cumulo+rho_cumulo+emission_CO2+consum_O2+interval_O2+genera te_CO2; 0.763, 0.906, tmp+pH+PL+PL_cumulo+AN+interval_Pdt.+ mu_cumulo+nu_cumulo+rho_cumulo+nu_per_rho_cumulo+rab_integr al+Accum._Yield+emission_CO2+consum_O2+interval_O2+generate _CO2; 0.768, 0.906, tmp+pH+PL+PL_cumulo+AN+interval_Pdt.+mu _cumulo+nu_cumulo+rho_cumulo+nu_per_rho_cumulo+emission_O2+ emission_CO2+consum _O2+interval_O2+generate_CO2; 0.781, 0.9 06, tmp+PL_cumulo+interval_Pdt.+OD+mu_cumulo+nu_cumulo+rho_ cumulo+nu_per_rho_cumulo+emission_O2+emission_CO2+consum_O2 +generate_CO2; 0.758, 0.906, tmp+PL+PL_cumulo+AN+interval_P dt.+mu_cumulo+nu_cumulo+rho_cumulo+nu_per_rho_cumulo+Arg_co nc._cumulo+RQ_integral+Accum._Yield+emission_O2+emission_CO 2+consum_O2+interval_O2+generate_CO2; 0.785, 0.906, tmp+PL_ cumulo+interval_Pdt.+mu_cumulo+nu_cumulo+rho_cumulo+nu_per_ rho_cumulo+RQ_integral+emission_CO2+consum_O2+generate_CO2; 0.773, 0.906, tmp+PL_cumulo+interval_Pdt.+mu_cumulo+nu_cum ulo+rho_cumulo+nu_per_rho_cumulo+rab_integral+RQ_integral+A ccum._Yield+emission_CO2+consum_O2+interval_O2+generate_CO 2; 0.773, 0.906, tmp+PL+PL_cumulo+interval_yield+interval_P dt.+mu_cumulo+nu_cumulo+rho_cumulo+nu_per_rho_cumulo+cell_y ield_cumulo+rab_integral+emission_CO2+consum_O2+generate_CO 2; 0.768, 0.906, tmp+PL+AN+interval_Pdt.+OD+mu_cumulo+nu_cu mulo+rho_cumulo+nu_per_rho_cumulo+Arg_conc._cumulo+Accum._Y ield+emission_CO2+consum_O2+interval_O2+generate_CO2; 0.781, 0.906, tmp+PL_cumulo+interval_Pdt.+OD+mu_cumulo+nu_cumulo+ rho_cumulo+nu_per_rho_cumulo+emission_CO2+consum_O2+interva l_O2+generate_CO2; 0.773, 0.906, tmp+PL+PL_cumulo+AN+interv al_Pdt.+RS+mu_cumulo+nu_cumulo+rho_cumulo+nu_per_rho_cumulo +emission_O2+emission_CO2+consum _O2+generate_CO2; 0.777, 0. 906, tmp+PL_cumulo+interval_Pdt.+mu_cumulo+nu_cumulo+rho_cu mulo+nu_per_rho_cumulo+rab_integral+RQ_integral+emission_CO 2+consum_O2+interval_O2+generate_CO2; 0.785, 0.906, tmp+PL_ cumulo+mu_cumulo+nu_cumulo+rho_cumulo+nu_per_rho_cumulo+cel l_yield_cumulo+rab_integral+emission_CO2+consum_O2+generate _CO2; 0.773, 0.906, tmp+PL+AN+OD+RS+nu_cumulo+rho_cumulo+nu _per_rho_cumulo+Arg_conc._cumulo+rab_integral+Accum._Yield+ emission_CO2+consum_O2+generate_CO2; 0.777, 0.906, tmp+pH+P L+interval_Pdt.+mu_cumulo+nu_cumulo+rho_cumulo+nu_per_rho_c umulo+cell_yield_cumulo+emission_O2+emission_CO2+consum_O2+ generate_CO2; 0.781, 0.906, tmp+PL+PL_cumulo+AN+interval_Pd t.+mu_cumulo+rho_cumulo+Accum._Yield+emission_CO2+consum_O2 +interval_O2+generate_CO2; 0.777, 0.906, tmp+PL+AN+interval _yield+interval_Pdt.+mu_cumulo+rho_cumulo+nu_per_rho_cumulo +Accum._Yield+emission_CO2+consum_O2+interval_O2+generate_C 02; 0.781, 0.906, tmp+PL_cumulo+interval_Pdt.+OD+RS+mu_cumu lo+nu_cumulo+rho_cumulo+nu_per_rho_cumulo+emission_CO2+cons um_O2+generate_CO2; 0.777, 0.906, tmp+PL+OD+mu_cumulo+nu_cu mulo+rho_cumulo+nu_per_rho_cumulo+Arg_conc._cumulo+rab_inte gral+Accum._Yield+emission_CO2+consum_O2+generate_CO2; 0.77 7, 0.906, tmp+PL+AN+interval_yield+interval_Pdt.+mu_cumulo+ rho_cumulo+rab_integral+Accum._Yield+emission_CO2+consum_O2 +interval_O2+generate_CO2; 0.768, 0.906, tmp+PL+PL_cumulo+i nterval_Pdt.+mu_cumulo+nu_cumulo+rho_cumulo+nu_per_rho_cumu lo+rab_integral+RQ_integral+Accum._Yield+emission_CO2+consu m_O2+interval_O2+generate_CO2; 0.789, 0.906, tmp+PL_cumulo+ interval_Pdt.+OD+nu_cumulo+rho_cumulo+nu_per_rho_cumulo+emi ssion_CO2+consum_O2+generate_CO2; 0.763, 0.906, tmp+pH+PL+P L_cumulo+interval_Pdt.+OD+RS+nu_cumulo+rho_cumulo+nu_per_rh o_cumulo+Arg_conc._cumulo+Accum._Yield+emission_O2+emission _CO2+consum_O2+generate_CO2; 0.785, 0.906, tmp+PL_cumulo+in terval_Pdt.+OD+nu_cumulo+rho_cumulo+nu_per_rho_cumulo+Arg_c onc._cumulo+emission_CO2+consum _O2+generate_CO2; 0.789, 0.9 06, tmp+PL+OD+nu_cumulo+rho_cumulo+nu_per_rho_cumulo+rab_in tegral+emission_CO2+consum _O2+generate_CO2; 0.763, 0.906, t mp+PL+PL_cumulo+interval_Pdt.+mu_cumulo+nu_cumulo+rho_cumul o+nu_per_rho_cumulo+cell_yield_cumulo+rab_integral+RQ_integ ral+Accum._Yield+emission_O2+emission_CO2+consum_O2+generat e_CO2; 0.773, 0.906, tmp+PL+AN+interval_Pdt.+OD+nu_cumulo+r ho_cumulo+nu_per_rho_cumulo+rab_integral+Accum._Yield+emiss ion_CO2+consum_O2+interval_O2+generate_CO2; 0.768, 0.906, t mp+PL+AN+interval_Pdt.+OD+RS+nu_cumulo+rho_cumulo+nu_per_rh o_cumulo+Arg_conc._cumulo+Accum._Yield+emission_O2+emission _CO2+consum_O2+generate_CO2; 0.773, 0.906, tmp+PL+PL_cumulo +AN+interval_Pdt.+RS+mu_cumulo+nu_cumulo+rho_cumulo+nu_per_ rho_cumulo+Accum._Yield+emission_CO2+consum_O2+generate_CO 2; 0.768, 0.906, tmp+PL+AN+interval_Pdt.+OD+mu_cumulo+nu_cu mulo+rho_cumulo+nu_per_rho_cumulo+RQ_integral+Accum._Yield+ emission_O2+emission_CO2+consum_O2+generate_CO2; 0.773, 0.9 06, tmp+PL+PL_cumulo+OD+RS+nu_cumulo+rho_cumulo+nu_per_rho_ cumulo+Arg_conc._cumulo+rab_integral+Accum._Yield+emission-CO2+consum_O2+generate_CO2; 0.768, 0.906, tmp+pH+PL+PL_cumu lo+AN+interval_Pdt.+mu_cumulo+nu_cumulo+rho_cumulo+nu_per_r ho_cumulo+Accum._Yield+emission_O2+emission_CO2+consum_O2+g enerate_CO2; 0.785, 0.906, tmp+interval_Pdt.+OD+mu_cumulo+n u_cumulo+rho_cumulo+nu_per_rho_cumulo+rab_integral+emission _CO2+consum_O2+generate_CO2; 0.785, 0.906, tmp+pH+PL+interv al_Pdt.+mu_cumulo+nu_cumulo+rho_cumulo+nu_per_rho_cumulo+em ission_CO2+consum_O2+generate_CO2; 0.773, 0.906, tmp+PL+int erval_Pdt.+OD+mu_cumulo+nu_cumulo+rho_cumulo+nu_per_rho_cum ulo+Arg_conc._cumulo+rab_integral+emission_CO2+consum_O2+in terval_O2+generate_CO2; 0.768, 0.906, tmp+pH+PL+interval_Pd t.+RS+mu_cumulo+nu_cumulo+rho_cumulo+nu_per_rho_cumulo+rab_ integral+RQ_integral+Accum._Yield+emission_CO2+consum_O2+ge nerate_CO2; 0.758, 0.906, tmp+pH+PL+PL_cumulo+AN+interval_P dt.+OD+nu_cumulo+rho_cumulo+nu_per_rho_cumulo+Arg_conc._cum ulo+rab_integral+Accum._Yield+emission_O2+emission_CO2+cons um_O2+generate_CO2; 0.773, 0.906, tmp+PL_cumulo+interval_Pd t.+OD+mu_cumulo+nu_cumulo+rho_cumulo+nu_per_rho_cumulo+Arg_ conc._cumulo+rab_integral+Accum._Yield+emission_CO2+consum_ O2+generate_CO2; 0.768, 0.906, tmp+PL_cumulo+interval_Pdt.+ RS+mu_cumulo+nu_cumulo+rho_cumulo+nu_per_rho_cumulo+Arg_con c._cumulo+rab_integral+RQ_integral+Accum._Yield+emission_CO 2+consum_O2+generate_CO2; 0.777, 0.906, tmp+PL+AN+interval_ Pdt.+OD+nu_cumulo+rho_cumulo+nu_per_rho_cumulo+cell_yield_c umulo+rab_integral+emission_CO2+consum _O2+generate_CO2; 0.7 81, 0.906, tmp+PL+OD+RS+nu_cumulo+rho_cumulo+nu_per_rho_cum ulo+Arg_conc._cumulo+rab_integral+Accum._Yield+emission_CO2 +generate_CO2; 0.773, 0.906, tmp+PL+PL_cumulo+AN+interval_P dt.+OD+nu_cumulo+rho_cumulo+nu_per_rho_cumulo+Arg_conc._cum ulo+Accum._Yield+emission_CO2+consum_O2+generate_CO2; 0.781, 0.906, tmp+interval_Pdt.+RS+mu_cumulo+nu_cumulo+rho_cumulo +nu_per_rho_cumulo+cell_yield_cumulo+rab_integral+emission_ CO2+consum_O2+generate_CO2; 0.773, 0.906, tmp+PL+PL_cumulo+ interval_Pdt.+mu_cumulo+nu_cumulo+rho_cumulo+nu_per_rho_cum ulo+cell_yield_cumulo+rab_integral+emission_CO2+consum_O2+i nterval_O2+generate_CO2; 0.772, 0.906, tmp+PL+interval_Pdt. +mu_cumulo+nu_cumulo+rho_cumulo+nu_per_rho_cumulo+Arg_conc. _cumulo+RQ_integral+emission_O2+emission_CO2+consum_O2+inte rval_O2+generate_CO2; 0.768, 0.906, tmp+PL+AN+interval_Pdt. +OD+mu_cumulo+nu_cumulo+rho_cumulo+nu_per_rho_cumulo+cell_y ield_cumulo+Accum._Yield+emission_O2+emission_CO2+consum_O2 +generate_CO2; 0.772, 0.906, tmp+PL+PL_cumulo+interval_Pdt. +RS+mu_cumulo+nu_cumulo+rho_cumulo+nu_per_rho_cumulo+Arg_co nc._cumulo+rab_integral+emission_CO2+consum_O2+generate_CO 2; 0.768, 0.906, tmp+PL+AN+interval_Pdt.+OD+RS+mu_cumulo+nu _cumulo+rho_cumulo+nu_per_rho_cumulo+cell_yield_cumulo+rab_ integral+emission_CO2+consum_O2+generate_CO2; 0.789, 0.906, tmp+PL_cumulo+OD+nu_cumulo+rho_cumulo+nu_per_rho_cumulo+Ar g_conc._cumulo+emission_CO2+consum_O2+generate_CO2; 0.777, 0.906, tmp+PL+interval_Pdt.+OD+nu_cumulo+rho_cumulo+nu_per_ rho_cumulo+Arg_conc._cumulo+RQ_integral+emission_O2+emissio n_CO2+consum_O2+generate_CO2; 0.772, 0.906, tmp+pH+PL+PL_cu mulo+interval_Pdt.+mu_cumulo+nu_cumulo+rho_cumulo+nu_per_rh o_cumulo+Accum._Yield+emission_O2+emission_CO2+consum_O2+ge nerate_CO2; 0.781, 0.906, tmp+PL+AN+interval_yield+interval _Pdt.+mu_cumulo+rho_cumulo+Arg_conc._cumulo+emission_CO2+co nsum_O2+interval_O2+generate_CO2; 0.768, 0.906, tmp+PL+AN+i nterval_Pdt.+mu_cumulo+nu_cumulo+rho_cumulo+nu_per_rho_cumu lo+cell_yield_cumulo+rab_integral+Accum._Yield+emission_O2+ emission_CO2+consum_O2+generate_CO2; 0.781, 0.906, tmp+PL_c umulo+interval_Pdt.+RS+mu_cumulo+nu_cumulo+rho_cumulo+nu_pe r_rho_cumulo+Accum._Yield+emission_CO2+consum_O2+generate_C 02; 0.772, 0.906, tmp+PL+AN+interval_Pdt.+mu_cumulo+nu_cumu lo+rho_cumulo+nu_per_rho_cumulo+cell_yield_cumulo+rab_integ ral+emission_O2+emission_CO2+consum _O2+generate_CO2; 0.777, 0.906, tmp+pH+PL_cumulo+OD+nu_cumulo+rho_cumulo+nu_per_rho _cumulo+Arg_conc._cumulo+rab_integral+Accum._Yield+emission _CO2+consum_O2+generate_CO2; 0.777,0.906, tmp+pH+PL+PL_cumu lo+interval_Pdt.+mu_cumulo+nu_cumulo+rho_cumulo+nu_per_rho_ cumulo+emission_O2+emission_CO2+consum_O2+generate_CO2; 0.7 72, 0.906, tmp+PL+PL_cumulo+interval_Pdt.+RS+mu_cumulo+nu_c umulo+rho_cumulo+nu_per_rho_cumulo+emission_O2+emission_CO2 +consum_O2+interval_O2+generate_CO2; 0.785, 0.906, tmp+PL_c umulo+interval_Pdt.+OD+nu_cumulo+rho_cumulo+nu_per_rho_cumu lo+rab_integral+emission_CO2+consum_O2+generate_CO2; 0.777, 0.906, tmp+PL+AN+interval_Pdt.+OD+RS+nu_cumulo+rho_cumulo+ nu_per_rho_cumulo+rab_integral+emission_CO2+consum_O2+gener ate_CO2; 0.768, 0.906, tmp+pH+PL+interval_Pdt.+mu_cumulo+nu _cumulo+rho_cumulo+nu_per_rho_cumulo+Arg_conc._cumulo+cell_ yield_cumulo+rab_integral+Accum._Yield+emission_CO2+consum_ O2+generate_CO2; 0.781, 0.906, tmp+PL+AN+interval_Pdt.+OD+n u_cumulo+rho_cumulo+nu_per_rho_cumulo+Accum._Yield+emission _CO2+consum_O2+generate_CO2; 0.768, 0.906, tmp+PL+PL_cumulo +AN+interval_Pdt.+mu_cumulo+nu_cumulo+rho_cumulo+nu_per_rho _cumulo+Arg_conc._cumulo+cell_yield_cumulo+rab_integral+emi ssion_CO2+consum_O2+generate_CO2; 0.772, 0.906, tmp+PL+AN+i nterval_Pdt.+RS+mu_cumulo+nu_cumulo+rho_cumulo+nu_per_rho_c umulo+Arg_conc._cumulo+emission_CO2+consum_O2+interval_O2+g enerate_CO2; 0.763, 0.906, tmp+PL+interval_Pdt.+mu_cumulo+n u_cumulo+rho_cumulo+nu_per_rho_cumulo+Arg_conc._cumulo+cell _yield_cumulo+RQ_integral+Accum._Yield+emission_O2+emission _CO2+consum_O2+interval_O2+generate_CO2; 0.777, 0.906, tmp+ pH+interval_Pdt.+mu_cumulo+nu_cumulo+rho_cumulo+nu_per_rho_ cumulo+Arg_conc._cumulo+rab_integral+Accum._Yield+emission_ CO2+consum_O2+generate_CO2; 0.777, 0.906, tmp+PL+OD+nu_cumu lo+rho_cumulo+nu_per_rho_cumulo+Arg_conc._cumulo+rab_integr al+Accum._Yield+emission_CO2+consum_O2+interval_O2+generate _CO2; 0.781, 0.906, tmp+PL+AN+interval_yield+interval_Pdt.+ mu_cumulo+rho_cumulo+rab_integral+emission_CO2+consum_O2+in terval_O2+generate_CO2; 0.763, 0.906, tmp+PL+PL_cumulo+inte rval_Pdt.+OD+mu_cumulo+nu_cumulo+rho_cumulo+nu_per_rho_cumu lo+RQ_integral+Accum._Yield+emission_O2+emission_CO2+consum _O2+interval_O2+generate_CO2; 0.785, 0.906, tmp+interval_Pd t.+mu_cumulo+nu_cumulo+rho_cumulo+nu_per_rho_cumulo+rab_int egral+RQ_integral+Accum._Yield+emission_CO2+consum_O2; 0.76 8, 0.906, tmp+PL+AN+interval_Pdt.+mu_cumulo+nu_cumulo+rho_c umulo+nu_per_rho_cumulo+rab_integral+RQ_integral+Accum._Yie ld+emission_O2+emission_CO2+consum _O2+generate_CO2; 0.777, 0.906, tmp+PL+AN+interval_Pdt.+OD+nu_cumulo+rho_cumulo+nu_p er_rho_cumulo+Accum._Yield+emission_O2+emission_CO2+consum_ O2+generate_CO2; 0.785, 0.906, tmp+PL+AN+interval_Pdt.+OD+m u_cumulo+rho_cumulo+Accum._Yield+emission_CO2+consum_O2+gen erate_CO2; 0.772, 0.906, tmp+PL+interval_Pdt.+mu_cumulo+nu_ cumulo+rho_cumulo+nu_per_rho_cumulo+Arg_conc._cumulo+rab_in tegral+RQ_integral+emission_CO2+consum_O2+interval_O2+gener ate_CO2; 0.781, 0.906, tmp+PL+OD+mu_cumulo+nu_cumulo+rho_cu mulo+nu_per_rho_cumulo+cell_yield_cumulo+emission_O2+emissi on_CO2+consum_O2+generate_CO2; 0.772, 0.906, tmp+PL+PL_cumu lo+interval_Pdt.+mu_cumulo+nu_cumulo+rho_cumulo+nu_per_rho_ cumulo+cell_yield_cumulo+RQ_integral+emission_O2+emission_C O2+consum_O2+generate_CO2; 0.781, 0.906, tmp+PL+OD+nu_cumul o+rho_cumulo+nu_per_rho_cumulo+Arg_conc._cumulo+cell_yield_ cumulo+emission_O2+emission_CO2+consum_O2+generate_CO2 0.7 63, 0.906, tmp+PL+interval_Pdt.+OD+RS+mu_cumulo+nu_cumulo+r ho_cumulo+nu_per_rho_cumulo+cell_yield_cumulo+RQ_integral+A ccum._Yield+emission_O2+emission_CO2+consum_O2+generate_CO 2; 0.772, 0.906, tmp+PL+interval_yield+interval_Pdt.+mu_cum ulo+nu_cumulo+rho_cumulo+nu_per_rho_cumulo+Arg_conc._cumulo +cell_yield_cumulo+rab_integral+emission_CO2+consum_O2+gene rate_CO2; 0.763, 0.906, tmp+pH+PL+PL_cumulo+interval_Pdt.+O D+mu_cumulo+nu_cumulo+rho_cumulo+nu_per_rho_cumulo+Arg_conc. _cumulo+Accum._Yield+emission_O2+emission_CO2+consum_O2+gen erate_CO2; 0.781, 0.906, tmp+PL+OD+nu_cumulo+rho_cumulo+nu_ per_rho_cumulo+Arg_conc._cumulo+rab_integral+emission_CO2+c onsum_O2+interval_O2+generate_CO2; 0.781, 0.906, tmp+PL+AN+ interval_Pdt.+RS+mu_cumulo+rho_cumulo+Arg_conc._cumulo+Accu m._Yield+emission_CO2+consum_O2+generate_CO2; 0.763, 0.906, tmp+PL+interval_Pdt.+mu_cumulo+nu_cumulo+rho_cumulo+nu_per _rho_cumulo+cell_yield_cumulo+rab_integral+RQ_integral+Accu m._Yield+emission_O2+emission_CO2+consum_O2+interval_O2+gen erate_CO2; 0.781, 0.906, tmp+PL+AN+interval_Pdt.+OD+mu_cumu lo+rho_cumulo+Accum._Yield+emission_CO2+consum_O2+interval O2+generate_CO2; 0.758, 0.906, tmp+PL+PL_cumulo+AN+interval _Pdt.+RS+mu_cumulo+nu_cumulo+rho_cumulo+nu_per_rho_cumulo+A rg_conc._cumulo+rab_integral+Accum._Yield+emission_O2+emiss ion_CO2+consum_O2+generate_CO2; 0.772, 0.906, tmp+pH+PL+AN+ interval_Pdt.+mu_cumulo+nu_cumulo+rho_cumulo+nu_per_rho_cum ulo+rab_integral+RQ_integral+emission_CO2+consum_O2+generat e_CO2; 0.758, 0.906, tmp+PL+PL_cumulo+AN+interval_Pdt.+mu_c umulo+nu_cumulo+rho_cumulo+nu_per_rho_cumulo+Arg_conc._cumu lo+rab_integral+RQ_integral+Accum._Yield+emission_O2+emissi on_CO2+consum_O2+generate_CO2; 0.781, 0.906, tmp+PL+OD+mu_c umulo+nu_cumulo+rho_cumulo+nu_per_rho_cumulo+cell_yield_cum ulo+rab_integral+emission_CO2+consum_O2+generate_CO2; 0.758, 0.906, tmp+PL+PL_cumulo+AN+interval_Pdt.+RS+mu_cumulo+nu_c umulo+rho_cumulo+nu_per_rho_cumulo+Arg_conc._cumulo+cell_yi eld_cumulo+rab_integral+Accum._Yield+emission_CO2+consum_O2 +generate_CO2; 0.777, 0.906, tmp+PL+AN+interval_Pdt.+mu_cum ulo+nu_cumulo+rho_cumulo+nu_per_rho_cumulo+cell_yield_cumul o+RQ_integral+emission_CO2+consum_O2+generate_CO2; 0.781, 0. 906, tmp+PL+interval_Pdt.+mu_cumulo+nu_cumulo+rho_cumulo+nu _per_rho_cumulo+cell_yield_cumulo+Accum._Yield+emission_CO2 +consum_O2+generate_CO2; 0.772, 0.906, tmp+PL+PL_cumulo+AN+ interval_Pdt.+OD+nu_cumulo+rho_cumulo+nu_per_rho_cumulo+Arg _conc._cumulo+rab_integral+emission_CO2+consum_O2+generate_ CO2; 0.772, 0.906, tmp+PL+AN+interval_Pdt.+OD+nu_cumulo+rho _cumulo+nu_per_rho_cumulo+Arg_conc._cumulo+emission_O2+emis sion_CO2+consum_O2+interval_O2+generate_CO2; 0.772, 0.906, tmp+PL+AN+interval_Pdt.+OD+mu_cumulo+nu_cumulo+rho_cumulo+n u_per_rho_cumulo+cell_yield_cumulo+emission_CO2+consum_O2+i nterval_O2+generate_CO2; 0.758, 0.906, tmp+pH+PL+PL_cumulo+ AN+interval_Pdt.+OD+mu_cumulo+nu_cumulo+rho_cumulo+nu_per_r ho_cumulo+Arg_conc._cumulo+rab_integral+Accum._Yield+emissi on_CO2+consum_O2+generate_CO2; 0.768, 0.906, tmp+PL+AN+inte rval_Pdt.+OD+RS+mu_cumulo+nu_cumulo+rho_cumulo+nu_per_rho_c umulo+rab_integral+emission_CO2+consum_O2+interval_O2+gener ate_CO2; 0.781, 0.906, tmp+PL+OD+RS+nu_cumulo+rho_cumulo+nu _per_rho_cumulo+Arg_conc._cumulo+emission_O2+emission_CO2+c onsum_O2+generate_CO2; 0.793, 0.906, tmp+PL+AN+interval_Pdt. +mu_cumulo+rho_cumulo+emission_CO2+consum_O2+generate_CO2; 0.772, 0.906, tmp+PL+interval_Pdt.+RS+mu_cumulo+nu_cumulo+r ho_cumulo+nu_per_rho_cumulo+Arg_conc._cumulo+cell_yield_cum ulo+emission_O2+emission_CO2+consum_O2+generate_CO2; 0.777, 0.906, tmp+pH+PL+interval_Pdt.+mu_cumulo+nu_cumulo+rho_cum ulo+nu_per_rho_cumulo+Accum._Yield+emission_O2+emission_CO2 +consum_O2+generate_CO2; 0.772, 0.906, tmp+PL+interval_Pdt. +mu_cumulo+nu_cumulo+rho_cumulo+nu_per_rho_cumulo+Arg_conc. _cumulo+rab_integral+Accum._Yield+emission_CO2+consum_O2+in terval_O2+generate_CO2; 0.777, 0.906, tmp+interval_Pdt.+RS+ mu_cumulo+nu_cumulo+rho_cumulo+nu_per_rho_cumulo+rab_integr al+RQ_integral+Accum._Yield+emission_CO2+consum_O2+generate _CO2; 0.785, 0.906, tmp+PL+AN+interval_Pdt.+mu_cumulo+rho_c umulo+Accum._Yield+emission_O2+emission_CO2+consum_O2+gener ate_CO2; 0.763, 0.906, tmp+PL_cumulo+interval_Pdt.+RS+mu_cu mulo+nu_cumulo+rho_cumulo+nu_per_rho_cumulo+Arg_conc._cumul o+cell_yield_cumulo+rab_integral+RQ_integral+Accum._Yield+e mission_CO2+consum_O2+generate_CO2; 0.768, 0.906, tmp+PL+PL _cumulo+AN+interval_yield+interval_Pdt.+mu_cumulo+nu_cumulo +rho_cumulo+nu_per_rho_cumulo+rab_integral+RQ_integral+emis sion_CO2+consum_O2+generate_CO2; 0.772, 0.906, tmp+PL+PL_cu mulo+AN+interval_Pdt.+OD+mu_cumulo+nu_cumulo+rho_cumulo+nu_ per_rho_cumulo+emission_O2+emission_CO2+consum_O2+generate_ CO2; 0.777, 0.906, tmp+PL_cumulo+interval_Pdt.+OD+mu_cumulo +nu_cumulo+rho_cumulo+nu_per_rho_cumulo+rab_integral+emissi on_CO2+consum_O2+interval_O2+generate_CO2; 0.772, 0.906, tm p+PL+PL_cumulo+interval_Pdt.+OD+mu_cumulo+nu_cumulo+rho_cum ulo+nu_per_rho_cumulo+emission_O2+emission_CO2+consum_O2+in terval_O2+generate_CO2; 0.763, 0.906, tmp+PL+PL_cumulo+inte rval_Pdt.+RS+mu_cumulo+nu_cumulo+rho_cumulo+nu_per_rho_cumu lo+RQ_integral+Accum._Yield+emission_O2+emission_CO2+consum _O2+interval_O2+generate_CO2; 0.772, 0.906, tmp+PL+PL_cumul o+AN+interval_Pdt.+mu_cumulo+nu_cumulo+rho_cumulo+nu_per_rh o_cumulo+cell_yield_cumulo+Accum._Yield+emission_CO2+consum _O2+generate_CO2; 0.763, 0.906, tmp+PL+PL_cumulo+AN+interva l_Pdt.+mu_cumulo+nu_cumulo+rho_cumulo+nu_per_rho_cumulo+Arg _conc._cumulo+cell_yield_cumulo+Accum._Yield+emission_O2+em ission_CO2+consum_O2+generate_CO2; 0.785, 0.906, tmp+PL+mu_ cumulo+nu_cumulo+rho_cumulo+nu_per_rho_cumulo+cell_yield_cu mulo+emission_O2+emission_CO2+consum_O2+generate_CO2; 0.758, 0.906, tmp+PL+PL_cumulo+interval_Pdt.+mu_cumulo+nu_cumulo+ rho_cumulo+nu_per_rho_cumulo+Arg_conc._cumulo+cell_yield_cu mulo+rab_integral+RQ_integral+Accum._Yield+emission_O2+emis sion_CO2+consum_O2+generate_CO2; 0.777, 0.906, tmp+PL_cumul o+interval_Pdt.+mu_cumulo+nu_cumulo+rho_cumulo+nu_per_rho_c umulo+RQ_integral+Accum._Yield+emission_O2+emission_CO2+con sum_O2+generate_CO2; 0.781, 0.906, tmp+PL+PL_cumulo+interva l_Pdt.+OD+nu_cumulo+rho_cumulo+nu_per_rho_cumulo+emission_O 2+emission_CO2+consum_O2+generate_CO2; 0.777, 0.906, tmp+in terval_Pdt.+mu_cumulo+nu_cumulo+rho_cumulo+nu_per_rho_cumul o+Arg_conc._cumulo+cell_yield_cumulo+rab_integral+Accum._Yi eld+emission_CO2+consum_O2+generate_CO2; 0.781, 0.906, tmp+ pH+PL+AN+interval_Pdt.+mu_cumulo+rho_cumulo+Accum. Yield+em ission_CO2+consum_O2+interval_O2+generate_CO2; 0.777, 0.906, tmp+PL_cumulo+AN+interval_Pdt.+mu_cumulo+nu_cumulo+rho cum ulo+nu_per_rho_cumulo+cell_yield_cumulo+rab_integral+emissi on_CO2+consum_O2+generate_CO2; 0.763, 0.906, tmp+PL+AN+inte rval_Pdt.+OD+mu_cumulo+nu_cumulo+rho_cumulo+nu_per_rho_cumu lo+rab_integral+RQ_integral+Accum._Yield+emission_CO2+consu m_O2+interval_O2+generate_CO2; 0.776, 0.906, tmp+PL+AN+inte rval_Pdt.+mu_cumulo+rho_cumulo+nu_per_rho_cumulo+Arg_conc._ cumulo+rab_integral+emission_CO2+consum_O2+interval_O2+gene rate_CO2; 0.768, 0.906, tmp+PL+interval_Pdt.+RS+mu_cumulo+n u_cumulo+rho_cumulo+nu_per_rho_cumulo+Arg_conc._cumulo+cell _yield_cumulo+Accum._Yield+emission_O2+emission_CO2+consum_ O2+generate_CO2; 0.776, 0.906, tmp+pH+interval_Pdt.+mu_cumu lo+nu_cumulo+rho_cumulo+nu_per_rho_cumulo+cell_yield_cumulo +rab_integral+Accum._Yield+emission_CO2+consum_O2+generate_ CO2; 0.772, 0.906, tmp+PL+PL_cumulo+interval_Pdt.+RS+mu_cum ulo+nu_cumulo+rho_cumulo+nu_per_rho_cumulo+rab_integral+RQ_ integral+emission_CO2+consum_O2+generate_CO2; 0.781, 0.906, tmp+interval_Pdt.+OD+mu_cumulo+nu_cumulo+rho_cumulo+nu_per _rho_cumulo+rab_integral+Accum._Yield+emission_CO2+consum_O 2+generate_CO2; 0.776, 0.906, tmp+interval_Pdt.+mu_cumulo+n u_cumulo+rho_cumulo+nu_per_rho_cumulo+cell_yield_cumulo+rab _integral+RQ_integral+Accum._Yield+emission_CO2+consum_O2+g enerate_CO2; 0.776, 0.906, tmp+interval_Pdt.+OD+mu_cumulo+n u_cumulo+rho_cumulo+nu_per_rho_cumulo+Arg_conc._cumulo+rab_ integral+Accum._Yield+emission_CO2+consum_O2+generate_CO2; 0.792, 0.906, tmp+PL+interval_Pdt.+mu_cumulo+rho_cumulo+nu_ per_rho_cumulo+emission_CO2+consum_O2+generate_CO2; 0.763, 0.906, tmp+PL+AN+interval_Pdt.+OD+mu_cumulo+nu_cumulo+rho_c umulo+nu_per_rho_cumulo+Arg_conc._cumulo+rab_integral+Accum. _Yield+emission_CO2+consum_O2+interval_O2+generate_CO2; 0.7 81, 0.906, tmp+PL_cumulo+AN+interval_Pdt.+mu_cumulo+nu_cumu lo+rho_cumulo+nu_per_rho_cumulo+Arg_conc._cumulo+emission_C O2+consum_O2+generate_CO2; 0.767, 0.906, tmp+PL+AN+interval Pdt.+OD+mu cumulo+nu cumulo+rho cumulo+nu per rho cumulo+A rg_conc._cumulo+cell_yield_cumulo+rab_integral+emission_CO2 +consum_O2+generate_CO2; 0.781, 0.906, tmp+PL+AN+interval_P dt.+mu_cumulo+rho_cumulo+RQ_integral+Accum._Yield+emission_ CO2+consum_O2+interval_O2+generate_CO2; 0.789, 0.906, tmp+P L_cumulo+OD+nu_cumulo+rho_cumulo+nu_per_rho_cumulo+rab_inte gral+emission_CO2+consum_O2+generate_CO2; 0.772, 0.906, tmp +PL_cumulo+interval_Pdt.+mu_cumulo+nu_cumulo+rho_cumulo+nu_ per_rho_cumulo+Arg_conc._cumulo+cell_yield_cumulo+rab_integ ral+RQ_integral+emission_CO2+consum_O2+generate_CO2; 0.785, 0.906, tmp+OD+RS+nu_cumulo+rho_cumulo+nu_per_rho_cumulo+Ar g_conc._cumulo+rab_integral+Accum._Yield+emission_CO2+gener ate_CO2; 0.772, 0.906, tmp+PL+AN+interval_Pdt.+mu_cumulo+nu _cumulo+rho_cumulo+nu_per_rho_cumulo+Arg_conc._cumulo+Accum. _Yield+emission_O2+emission_CO2+consum_O2+generate_CO2; 0.7 85, 0.906, tmp+PL_cumulo+OD+nu_cumulo+rho_cumulo+nu_per_rho _cumulo+Arg_conc._cumulo+emission_O2+emission_CO2+consum_O2 +generate_CO2; 0.785, 0.906, tmp+interval_Pdt.+mu_cumulo+nu _cumulo+rho_cumulo+nu_per_rho_cumulo+Arg_conc._cumulo+rab_i ntegral+emission_CO2+consum_02+generate_CO2; 0.776, 0.906, tmp+PL_cumulo+interval_Pdt.+RS+mu_cumulo+nu_cumulo+rho_cumu lo+nu_per_rho_cumulo+Arg_conc._cumulo+rab_integral+emission _CO2+consum_O2+generate_CO2; 0.767, 0.906, tmp+PL+AN+interv al_Pdt.+RS+mu_cumulo+nu_cumulo+rho_cumulo+nu_per_rho_cumulo +cell_yield_cumulo+emission_O2+emission_CO2+consum_O2+inter val_O2+generate_CO2; 0.781, 0.906, tmp+PL+AN+interval_Pdt.+ mu_cumulo+rho_cumulo+nu_per_rho_cumulo+emission_O2+emission _CO2+consum_O2+interval_O2+generate_CO2; 0.763, 0.906, tmp+ pH+PL+interval_Pdt.+OD+mu_cumulo+nu_cumulo+rho_cumulo+nu_pe r_rho_cumulo+Arg_conc._cumulo+rab_integral+RQ_integral+Accu m._Yield+emission_CO2+consum_O2+generate_CO2; 0.772, 0.906, tmp+PL+interval_Pdt.+OD+RS+mu_cumulo+nu_cumulo+rho_cumulo+ nu_per_rho_cumulo+Accum._Yield+emission_O2+emission_CO2+con sum-O2+generate-CO2; 0.767, 0.906, tmp+PL+PL_cumulo+AN+inte rval_Pdt.+RS+mu_cumulo+nu_cumulo+rho_cumulo+nu_per_rho_cumu lo+cell_yield_cumulo+rab_integral+emission_CO2+consum_O2+ge nerate_CO2; 0.767, 0.906, tmp+PL+AN+interval_Pdt.+mu_cumulo +nu_cumulo+rho_cumulo+nu_per_rho_cumulo+Arg_conc._cumulo+ra b_integral+RQ_integral+emission_CO2+consum_O2+interval_O2+g enerate_CO2; 0.785, 0.906, tmp+PL_cumulo+OD+nu_cumulo+rho_c umulo+nu_per_rho_cumulo+Arg_conc._cumulo+rab_integral+emiss ion_CO2+interval_O2+generate_CO2; 0.763, 0.906, tmp+PL+PL_c umulo+AN+interval_Pdt.+mu_cumulo+nu_cumulo+rho_cumulo+nu_pe r_rho_cumulo+cell_yield_cumulo+rab_integral+RQ_integral+Acc um._Yield+emission_CO2+consum_O2+generate_CO2; 0.776, 0.906, tmp+PL_cumulo+AN+interval_Pdt.+OD+mu_cumulo+nu_cumulo+rho_ cumulo+nu_per_rho_cumulo+emission_CO2+consum_O2+interval_O2 +generate_CO2; 0.772, 0.906, tmp+PL+interval_Pdt.+RS+mu_cum ulo+nu_cumulo+rho_cumulo+nu_per_rho_cumulo+cell_yield_cumul o+emission_O2+emission_CO2+consum_O2+interval_O2+generate_C 02; 0.781, 0.906, tmp+PL_cumulo+interval_Pdt.+OD+nu_cumulo+ rho_cumulo+nu_per_rho_cumulo+rab_integral+RQ_integral+emiss ion_CO2+consum_O2+generate_CO2; 0.781, 0.906, tmp+PL+AN+OD+ nu_cumulo+rho_cumulo+nu_per_rho_cumulo+Arg_conc._cumulo+emi ssion_O2+emission_CO2+consum_O2+generate_CO2; 0.776, 0.906, tmp+PL+interval_Pdt.+OD+mu_cumulo+nu_cumulo+rho_cumulo+nu_ per_rho_cumulo+Arg_conc._cumulo+Accum._Yield+emission_CO2+c onsum_O2+generate_CO2; 0.758, 0.906, tmp+PL+PL_cumulo+AN+in terval_Pdt.+mu_cumulo+nu_cumulo+rho_cumulo+nu_per_rho_cumul o+Arg_conc._cumulo+cell_yield_cumulo+Accum._Yield+emission_ O2+emission_CO2+consum_O2+interval_O2+generate_CO2; 0.792, 0.906, tmp+PL_cumulo+interval_Pdt.+mu_cumulo+rho_cumulo+nu_ per_rho_cumulo+emission_CO2+consum_O2+generate_CO2; 0.767, 0.906, tmp+PL+interval_Pdt.+mu_cumulo+nu_cumulo+rho_cumulo+ nu_per_rho_cumulo+Arg_conc._cumulo+cell_yield_cumulo+rab_in tegral+Accum._Yield+emission_CO2+consum_O2+interval_O2+gene rate_CO2; 0.772, 0.906, tmp+pH+PL+interval_Pdt.+mu_cumulo+n u_cumulo+rho_cumulo+nu_per_rho_cumulo+Arg_conc._cumulo+cell _yield_cumulo+rab_integral+emission_CO2+consum_O2+generate_ CO2; 0.792, 0.906, tmp+PL+interval_Pdt.+mu_cumulo+rho_cumul o+Accum._Yield+emission_CO2+consum_O2+generate_CO2; 0.767, 0.906, tmp+PL+PL_cumulo+interval_Pdt.+RS+mu_cumulo+nu_cumul o+rho_cumulo+nu_per_rho_cumulo+Accum._Yield+emission_O2+emi ssion_CO2+consum_O2+interval_O2+generate_CO2; 0.772, 0.906, tmp+PL+interval Pdt.+mu_cumulo+nu_cumulo+rho_cumulo+nu_per _rho_cumulo+cell_yield_cumulo+rab_integral+RQ_integral+emis sion_O2+emission_CO2+consum_O2+generate_CO2; 0.781, 0.906, tmp+PL+PL_cumulo+interval_Pdt.+OD+nu_cumulo+rho_cumulo+nu_p er_rho_cumulo+rab_integral+emission_CO2+consum_O2+generate_ CO2; 0.780, 0.906, tmp+PL+AN+interval_Pdt.+RS+mu_cumulo+rho _cumulo+nu_per_rho_cumulo+rab_integral+emission_CO2+consum_ O2+generate_CO2; 0.780, 0.906, tmp+PL+AN+interval_yield+int erval_Pdt.+RS+mu_cumulo+rho_cumulo+Accum._Yield+emission_CO 2+consum_O2+generate_CO2; 0.772, 0.906, tmp+PL_cumulo+inter val_Pdt.+OD+RS+mu_cumulo+nu_cumulo+rho_cumulo+nu_per_rho_cu mulo+cell_yield_cumulo+rab_integral+emission_CO2+consum_O2+ generate_CO2; 0.776, 0.906, tmp+pH+PL+AN+interval_Pdt.+mu_c umulo+nu_cumulo+rho_cumulo+nu_per_rho_cumulo+Arg_conc._cumu lo+emission_CO2+consum_O2+generate_CO2; 0.772, 0.906, tmp+P L+interval_yield+interval_Pdt.+RS+mu_cumulo+nu_cumulo+rho_c umulo+nu_per_rho_cumulo+cell_yield_cumulo+rab_integral+emis sion_CO2+consum_O2+generate_CO2; 0.776, 0.906, tmp+PL+AN+in terval_yield+interval_Pdt.+mu_cumulo+rho_cumulo+nu_per_rho_ cumulo+rab_integral+emission_CO2+consum_O2+interval_O2+gene rate_CO2; 0.767, 0.906, tmp+PL+AN+interval_Pdt.+mu_cumulo+n u_cumulo+rho_cumulo+nu_per_rho_cumulo+Arg_conc._cumulo+cell _yield_umulo+Accum._Yield+emission_CO2+consum_O2+interval O2+generate_CO2; 0.763, 0.906, tmp+PL+AN+interval_Pdt.+RS+m u_cumulo+nu_cumulo+rho_cumulo+nu_per_rho_cumulo+rab_integra 1+RQ_integral+Accum._Yield+emission_CO2+consum_O2+interval_ O2+generate_CO2; 0.772, 0.906, tmp+PL_cumulo+interval_Pdt.+ mu_cumulo+nu_cumulo+rho_cumulo+nu_per_rho_cumulo+cell_yield _cumulo+rab_integral+Accum._Yield+emission_CO2+consum_O2+in terval_O2+generate_CO2; 0.763, 0.906, tmp+PL+PL_cumulo+AN+i nterval_Pdt.+RS+mu_cumulo+nu_cumulo+rho_cumulo+nu_per_rho_c umulo+rab_integral+RQ_integral+Accum._Yield+emission_CO2+co nsum_O2+generate_CO2; 0.780, 0.906, tmp+PL+interval_Pdt.+RS +mu_cumulo+rho_cumulo+rab_integral+RQ_integral+Accum._Yield +emission_CO2+consum_O2+generate_CO2; 0.785, 0.906, tmp+PL+ OD+nu_cumulo+rho_cumulo+nu_per_rho_cumulo+Arg_conc._cumulo+ rab_integral+Accum._Yield+emission_CO2+generate_CO2; 0.772, 0.906, tmp+PL+PL_cumulo+interval_Pdt.+RS+mu_cumulo+nu_cumu lo+rho_cumulo+nu_per_rho_cumulo+cell_yield_cumulo+rab_integ ral+emission_CO2+consum_O2+generate_CO2; 0.776, 0.906, tmp+ PL+interval_Pdt.+RS+mu_cumulo+nu_cumulo+rho_cumulo+nu_per_r ho_cumulo+rab_integral+emission_O2+emission_CO2+consum_O2+g enerate_CO2; 0.767, 0.906, tmp+PL+PL_cumulo+interval_Pdt.+R S+mu_cumulo+nu_cumulo+rho_cumulo+nu_per_rho_cumulo+rab_inte gral+RQ_integral+Accum._Yield+emission_CO2+consum_O2+genera te_CO2; 0.762, 0.906, tmp+pH+PL+PL_cumulo+interval_Pdt.+mu_ cumulo+nu_cumulo+rho_cumulo+nu_per_rho_cumulo+Arg_conc._cum ulo+RQ_integral+Accum._Yield+emission_O2+emission_CO2+consu m_O2+generate_CO2; 0.788, 0.906, tmp+mu_cumulo+nu_cumulo+rh o_cumulo+nu_per_rho_cumulo+cell_yield_cumulo+rab_integral+e mission_CO2+consum_O2+generate_CO2; 0.776, 0.906, tmp+PL+in terval_Pdt.+mu_cumulo+nu_cumulo+rho_cumulo+nu_per_rho_cumul o+Arg_conc._cumulo+rab_integral+emission_O2+emission_CO2+co nsum_O2+generate_CO2; 0.776, 0.906, tmp+PL+AN+interval_Pdt. +mu_cumulo+rho_cumulo+Arg_conc._cumulo+rab_integral+Accum._ Yield+emission_CO2+consum_O2+interval_O2+generate_CO2; 0.77 2, 0.906, tmp+PL_cumulo+interval_Pdt.+OD+mu_cumulo+nu_cumul o+rho_cumulo+nu_per_rho_cumulo+cell_yield_cumulo+rab_integr al+RQ_integral+emission_CO2+consum_O2+generate_CO2; 0.776, 0.906, tmp+PL+interval_Pdt.+OD+nu_cumulo+rho_cumulo+nu_per_ rho_cumulo+cell_yield_cumulo+rab_integral+RQ_integral+emiss ion_CO2+consum_O2+generate_CO2; 0.757, 0.906, tmp+PL+PL_cum ulo+AN+interval_Pdt.+mu_cumulo+nu_cumulo+rho_cumulo+nu_per_ rho_cumulo+cell_yield_cumulo+RQ_integral+Accum._Yield+emiss ion_O2+emission_CO2+consum_O2+interval_O2+generate_CO2; 0.7 76, 0.906, tmp+PL_cumulo+AN+interval_Pdt.+RS+mu_cumulo+nu_c umulo+rho_cumulo+nu_per_rho_cumulo+cell_yield_cumulo+emissi on-CO2+consum-O2+generate-CO2; 0.776, 0.906, tmp+PL+AN+inte rval_Pdt.+mu_cumulo+rho_cumulo+nu_per_rho_cumulo+rab_integr al+Accum._Yield+emission_CO2+consum_O2+interval_O2+generate _CO2; 0.776, 0.906, tmp+PL_cumulo+AN+interval_Pdt.+mu_cumul o+nu_cumulo+rho_cumulo+nu_per_rho_cumulo+cell_yield_cumulo+ emission_CO2+consum_O2+interval_O2+generate_CO2; 0.762, 0.9 06, tmp+PL+PL_cumulo+AN+interval_Pdt.+mu_cumulo+nu_cumulo+r ho_cumulo+nu_per_rho_cumulo+Arg_conc._cumulo+rab_integral+e mission_O2+emission_CO2+consum_O2+interval_O2+generate_CO2; 0.772, 0.906, tmp+PL+PL_cumulo+AN+interval_yield+interval_ Pdt.+mu_cumulo+nu_cumulo+rho_cumulo+nu_per_rho_cumulo+emiss ion_02+emission_CO2+consum_O2+generate_CO2; 0.757, 0.906, t mp+PL+PL_cumulo+AN+interval_Pdt.+RS+mu_cumulo+nu_cumulo+rho _cumulo+nu-per-rho-cumulo+Arg-conc._cumulo+Accum._Yield+emi ssion_O2+emission_CO2+consum_O2+interval_O2+generate_CO2; 0. 780, 0.906, tmp+interval_Pdt.+mu_cumulo+nu_cumulo+rho_cumul o+nu_per_rho_cumulo+Arg_conc._cumulo+rab_integral+Accum._Yi eld+emission_CO2+consum_O2+generate_CO2; 0.780, 0.906, tmp+ PL+AN+interval_Pdt.+mu_cumulo+rho_cumulo+nu_per_rho_cumulo+ Arg_conc._cumulo+Accum._Yield+emission_CO2+consum_O2+genera te_CO2; 0.780, 0.906, tmp+PL+OD+mu_cumulo+nu_cumulo+rho_cum ulo+nu_per_rho_cumulo+Arg_conc._cumulo+rab_integral+emissio n_CO2+consum_O2+generate_CO2; 0.772, 0.906, tmp+PL+AN+inter val_Pdt.+mu_cumulo+nu_cumulo+rho_cumulo+nu_per_rho_cumulo+A rg_conc._cumulo+RQ_integral+Accum._Yield+emission_CO2+consu m_O2+generate_CO2; 0.788, 0.906, tmp+PL+AN+interval_Pdt.+mu _cumulo+nu_cumulo+rho_cumulo+emission_CO2+consum_O2+generat e_CO2; 0.767, 0.906, tmp+PL+interval_Pdt.+OD+RS+nu_cumulo+r ho_cumulo+nu_per_rho_cumulo+Arg_conc._cumulo+Accum._Yield+e mission_O2+emission_CO2+consum_O2+interval_O2+generate_CO2; 0.772, 0.906, tmp+pH+PL+interval_Pdt.+RS+mu_cumulo+nu_cumu lo+rho_cumulo+nu_per_rho_cumulo+rab_integral+RQ_integral+em ission_CO2+consum_O2+generate_CO2; 0.776, 0.906, tmp+PL+AN+ interval_Pdt.+RS+mu_cumulo+nu_cumulo+rho_cumulo+nu_per_rho_ cumulo+RQ_integral+emission_CO2+consum_O2+generate_CO2; 0.7 67, 0.906, tmp+PL+AN+interval_Pdt.+RS+mu_cumulo+nu_cumulo+r ho_cumulo+nu_per_rho_cumulo+RQ_integral+Accum._Yield+emissi on_O2+emission_CO2+consum_O2+generate_CO2; 0.784, 0.906, tm p+PL+interval_Pdt.+mu_cumulo+rho_cumulo+RQ_integral+Accum._ Yield+emission_O2+emission_CO2+consum_O2+generate_CO2; 0.77 2, 0.906, tmp+PL+interval_Pdt.+mu_cumulo+nu_cumulo+rho_cumu lo+nu_per_rho_cumulo+rab_integral+RQ_integral+emission_O2+e mission_CO2+consum_O2+interval_O2+generate_CO2; 0.776, 0.90 6, tmp+PL+AN+interval_Pdt.+RS+mu_cumulo+rho_cumulo+Accum._Y ield+emission_O2+emission_CO2+consum_O2+interval_O2+generat e_CO2; 0.772, 0.906, tmp+PL+PL_cumulo+interval_Pdt.+mu_cumu lo+nu_cumulo+rho_cumulo+nu_per_rho_cumulo+Arg_conc._cumulo+ rab_integral+emission_CO2+consum_O2+interval_O2+generate_CO 2; 0.780, 0.906, tmp+PL+interval_Pdt.+OD+mu_cumulo+nu_cumul o+rho_cumulo+nu_per_rho_cumulo+Accum._Yield+emission_CO2+co nsum_O2+generate_CO2; 0.762, 0.906, tmp+PL+interval_Pdt.+OD +RS+nu_cumulo+rho_cumulo+nu_per_rho_cumulo+Arg_conc._cumulo +RQ_integral+Accum._Yield+emission_O2+emission_CO2+consum_O 2+interval_O2+generate_CO2; 0.792, 0.906, tmp+PL_cumulo+OD+ nu_cumulo+rho_cumulo+nu_per_rho_cumulo+Arg_conc._cumulo+emi ssion_CO2+generate_CO2; 0.780, 0.906, tmp+PL_cumulo+OD+nu_c umulo+rho_cumulo+nu_per_rho_cumulo+Arg_conc._cumulo+Accum._ Yield+emission_O2+emission_CO2+consum_O2+generate_CO2; 0.77 2, 0.906, tmp+PL+AN+interval_Pdt.+OD+mu_cumulo+nu_cumulo+rh o_cumulo+nu_per_rho_cumulo+Arg_conc._cumulo+emission_CO2+co nsum_O2+interval_O2+generate_CO2; 0.757, 0.906, tmp+pH+PL+P L_cumulo+interval_Pdt.+OD+nu_cumulo+rho_cumulo+nu_per_rho_c umulo+Arg_conc._cumulo+rab_integral+RQ_integral+Accum._Yiel d+emission_O2+emission_CO2+consum_O2+generate_CO2; 0.762, 0. 906, tmp+PL+interval_Pdt.+RS+mu_cumulo+nu_cumulo+rho_cumulo +nu_per_rho_cumulo+Arg_conc._cumulo+rab_integral+RQ_integra l+Accum._Yield+emission_O2+emission_CO2+consum_O2+generate_ CO2; 0.776, 0.906, tmp+PL+interval_yield+interval_Pdt.+OD+n u_cumulo+rho_cumulo+nu_per_rho_cumulo+Arg_conc._cumulo+rab_ integral+emission_CO2+consum_O2+generate_CO2; 0.757, 0.906, tmp+PL+AN+interval_Pdt.+RS+mu_cumulo+nu_cumulo+rho_cumulo+ nu_per_rho_cumulo+Arg_conc._cumulo+cell_yield_cumulo+rab_in tegral+RQ_integral+Accum._Yield+emission_CO2+consum_O2+gene rate_CO2; 0.784, 0.906, tmp+PL+interval_Pdt.+RS+mu_cumulo+r ho_cumulo+nu_per_rho_cumulo+rab_integral+emission_CO2+consu m_O2+generate_CO2; 0.762, 0.906, tmp+PL+PL_cumulo+AN+interv al_Pdt.+mu_cumulo+nu_cumulo+rho_cumulo+nu_per_rho_cumulo+ce 11-yield-cumulo+RQ-integral+Accum._Yield+emission_O2+emissi on-CO2+consum-O2+generate-CO2; 0.772, 0.906, tmp+PL+interva l_Pdt.+OD+mu_cumulo+nu_cumulo+rho_cumulo+nu_per_rho_cumulo+ Arg_conc._cumulo+cell_yield_cumulo+emission_O2+emission_CO2 +consum_O2+generate_CO2; 0.776, 0.906, tmp+PL_cumulo+AN+int erval_Pdt.+mu_cumulo+nu_cumulo+rho_cumulo+nu_per_rho_cumulo +Arg_conc._cumulo+cell_yield_cumulo+emission_CO2+consum_O2+ generate_CO2; 0.776, 0.906, tmp+PL+AN+interval_Pdt.+OD+mu_c umulo+nu_cumulo+rho_cumulo+nu_per_rho_cumulo+RQ_integral+em ission_CO2+consum_O2+generate_CO2; 0.757, 0.906, tmp+PL+PL_ cumulo+AN+interval_Pdt.+RS+mu_cumulo+nu_cumulo+rho_cumulo+n u_per_rho_cumulo+Arg_conc._cumulo+rab_integral+Accum._Yield +emission_CO2+consum_O2+interval_O2+generate_CO2; 0.776, 0. 906, tmp+PL+interval_yield+interval_Pdt.+OD+nu_cumulo+rho_c umulo+nu_per_rho_cumulo+rab_integral+RQ_integral+emission_C O2+consum_O2+generate_CO2; 0.772, 0.906, tmp+pH+PL+interval _Pdt.+RS+mu_cumulo+nu_cumulo+rho_cumulo+nu_per_rho_cumulo+r ab_integral+Accum._Yield+emission_CO2+consum_O2+generate_CO 2; 0.780, 0.906, tmp+PL+AN+interval_Pdt.+OD+nu_cumulo+rho_c umulo+nu_per_rho_cumulo+emission_CO2+consum_O2+interval_O2+ generate_CO2; 0.772, 0.906, tmp+PL+PL_cumulo+interval_Pdt.+ OD+mu_cumulo+nu_cumulo+rho_cumulo+nu_per_rho_cumulo+cell_yi eld_cumulo+rab_integral+emission_CO2+consum_O2+generate_CO 2; 0.772, 0.906, tmp+PL+interval_Pdt.+RS+mu_cumulo+nu_cumul o+rho_cumulo+nu_per_rho_cumulo+RQ_integral+emission_O2+emis sion_CO2+consum_O2+interval_O2+generate_CO2; 0.784, 0.906, tmp+PL_cumulo+interval_Pdt.+OD+nu_cumulo+rho_cumulo+nu_per_ rho_cumulo+Arg_conc._cumulo+Accum._Yield+emission_CO2+gener ate_CO2; 0.772, 0.906, tmp+PL+PL_cumulo+AN+interval_Pdt.+OD +mu_cumulo+nu_cumulo+rho_cumulo+nu_per_rho_cumulo+Accum._Yi eld+emission_CO2+consum_O2+generate_CO2; 0.772, 0.906, tmp+ PL+PL_cumulo+interval_Pdt.+OD+nu_cumulo+rho_cumulo+nu_per_r ho_cumulo+RQ_integral+Accum._Yield+emission_O2+emission_CO2 +consum_O2+generate_CO2; 0.772, 0.906, tmp+pH+PL+OD+RS+nu_c umulo+rho_cumulo+nu_per_rho_cumulo+Arg_conc._cumulo+rab_int egral+Accum._Yield+emission CO2+consum_O2+generate_CO2

### [203. Linear Model that Predicts Arginine Product ion Amount in Interval 3]

0.711, 0.864, tmp+AN_cumulo+interval_Pdt.+RS+Feed_Vol+mu_cu mulo+Sugar_consuming_rate_cumulo+rab_integral+AS_Vol.; 0.70 5, 0.864, tmp+AN_cumulo+interval_Pdt.+RS+Feed_Vol+mu_cumulo +Sugar_consuming_rate_cumulo+rab_integral+AS_Vol.+emission_ 02; 0.699, 0.863, tmp+AN_cumulo+RS+Feed_Vol+mu_cumulo+Sugar _consuming_rate_cumulo+RQ_integral+AS_Vol.+emission_O2+emis sion_CO2+generate_CO2; 0.698, 0.862, tmp+AN_cumulo+interval _yield+interval_Pdt.+RS+Feed_Vol+mu_cumulo+Sugar_consuming_ rate_cumulo+rab_integral+AS_Vol.+emission_O2; 0.702, 0.862, tmp+RS+Feed_Vol+mu_cumulo+Sugar_consuming_rate_cumulo+RQ_i ntegral+AS_Vol.+emission_O2+emission_CO2+generate_CO2; 0.70 2, 0.862, tmp+AN_cumulo+interval_Pdt.+RS+Feed_Vol+mu_cumulo +nu_per_rho_cumulo+Sugar_consuming_rate_cumulo+rab_integral +AS_Vol.; 0.710, 0.861, tmp+AN_cumulo+RS+Feed_Vol+mu_cumulo +Sugar_consuming_rate_cumulo+rab_integral+AS_Vol.; 0.701, 0. 861, tmp+AN_cumulo+interval_yield+interval_Pdt.+RS+Feed_Vol +mu_cumulo+Sugar_consuming_rate_cumulo+rab_integral+AS_Vo l.; 0.696, 0.861, tmp+AN_cumulo+interval_Pdt.+RS+Feed_Vol+m u_cumulo+nu_per_rho_cumulo+Sugar_consuming_rate_cumulo+rab_ integral+AS_Vol.+emission_O2; 0.691, 0.861, tmp+PL+interval _Pdt.+RS+Feed_Vol+mu_cumulo+nu_per_rho_cumulo+Sugar_consumi ng_rate_cumulo+RQ_integral+AS_Vol.+emission_O2+emission_CO 2; 0.696, 0.861, tmp+interval_Pdt.+RS+Feed_Vol+mu_cumulo+Su gar_consuming_rate_cumulo+RQ_integral+AS_Vol.+emission_O2+e mission_CO2+generate_CO2; 0.690, 0.861, tmp+AN_cumulo+inter val_yield+interval_Pdt.+RS+Feed_Vol+mu_cumulo+Sugar_consumi ng_rate_cumulo+rab_integral+AS_Vol.+emission_O2+emission_CO 2; 0.690, 0.861, tmp+AN_cumulo+RS+Feed_Vol+mu_cumulo+nu_per rho_cumulo+Sugar_consuming_rate_cumulo+RQ_integral+AS_Vol. +emission_O2+emission_CO2+generate_CO2; 0.695, 0.861, tmp+R S+Feed_Vol+mu_cumulo+rho_cumulo+Sugar_consuming_rate_cumulo +RQ_integral+AS_Vol.+emission_O2+emission_CO2+generate_CO2; 0.690, 0.861, tmp+interval_Pdt.+RS+Feed_Vol+mu_cumulo+nu_p er_rho_cumulo+Sugar_consuming_rate_cumulo+RQ_integral+AS_Vo l.+emission_O2+emission_CO2+generate_CO2; 0.695, 0.861, tmp +RS+Feed_Vol+mu_cumulo+nu_per_rho_cumulo+Sugar_consuming_ra te_cumulo+RQ_integral+AS_Vol.+emission_O2+emission_CO2+gene rate_CO2; 0.704, 0.860, tmp+AN_cumulo+interval_Pdt.+RS+Feed _Vol+mu_cumulo+Sugar_consuming_rate_cumulo+rab_integral+emi ssion_O2; 0.708, 0.860, tmp+interval_Pdt.+RS+Feed_Vol+mu_cu mulo+Sugar_consuming_rate_cumulo+rab_integral+AS_Vol.; 0.68 4, 0.860, tmp+PL+interval_Pdt.+OD+RS+Feed_Vol+mu_cumulo+rho _cumulo+Sugar_consuming_rate_cumulo+RQ_integral+AS_Vol.+emi ssion_O2+emission_CO2; 0.689, 0.860, tmp+PL+interval_Pdt.+R S+Feed_Vol+mu_cumulo+rho_cumulo+Sugar_consuming_rate_cumulo +RQ_integral+AS_Vol.+emission_O2+emission_CO2; 0.699, 0.860, tmp+AN_cumulo+RS+Feed_Vol+mu_cumulo+Sugar_consuming_rate_c umulo+RQ_integral+AS_Vol.+emission_CO2+generate_CO2; 0.708, 0.860, tmp+RS+Feed_Vol+mu_cumulo+Sugar_consuming_rate_cumu lo+AS_Vol.+emission_CO2+generate_CO2; 0.694, 0.860, tmp+PL+ AN_cumulo+interval_Pdt.+RS+Feed_Vol+mu_cumulo+Sugar_consumi ng_rate_cumulo+rab_integral+AS_Vol.+emission_O2; 0.703, 0.8 60, tmp+AN_cumulo+interval_Pdt.+RS+Feed_Vol+mu_cumulo+Sugar _consuming_rate_cumulo+RQ_integral+AS_Vol.; 0.703, 0.860, t mp+AN_cumulo+interval_yield+RS+Feed_Vol+mu_cumulo+Sugar_con suming_rate_cumulo+rab_integral+AS_Vol.; 0.698, 0.860, tmp+ PL+AN_cumulo+interval_Pdt.+RS+Feed_Vol+mu_cumulo+Sugar_cons uming_rate_cumulo+rab_integral+AS_Vol.; 0.703, 0.860, tmp+A N_cumulo+RS+Feed_Vol+mu_cumulo+Sugar_consuming_rate_cumulo+ rab_integral+AS_Vol.+emission_CO2; 0.703, 0.860, tmp+interv al_Pdt.+RS+Feed_Vol+mu_cumulo+Sugar_consuming_rate_cumulo+A S_Vol.+emission_CO2+generate_CO2; 0.693, 0.860, tmp+interva l_Pdt.+RS+Feed_Vol+mu_cumulo+nu_per_rho_cumulo+Sugar_consum ing_rate_cumulo+rab_integral+AS_Vol.+emission_O2+emission_C 02; 0.693, 0.860, tmp+interval_Pdt.+RS+Feed_Vol+mu_cumulo+r ho_cumulo+Sugar_consuming_rate_cumulo+rab_integral+AS_Vol.+ emission_O2+emission_CO2; 0.683, 0.860, tmp+PL+interval_Pdt. +RS+Feed_Vol+mu_cumulo+nu_per_rho_cumulo+Sugar_consuming_ra te_cumulo+RQ_integral+AS_Vol.+emission_O2+emission_CO2+gene rate_CO2; 0.688, 0.860, tmp+AN_cumulo+RS+Feed_Vol+mu_cumulo +rho_cumulo+Sugar_consuming_rate_cumulo+RQ_integral+AS_Vol. +emission_O2+emission_CO2+generate_CO2; 0.703, 0.860, tmp+i nterval_Pdt.+OD+RS+Feed_Vol+mu_cumulo+rho_cumulo+Sugar_cons uming_rate_cumulo+AS_Vol.; 0.683, 0.860, tmp+PL+AN+interval _Pdt.+RS+Feed_Vol+mu_cumulo+nu_per_rho_cumulo+Sugar_consumi ng_rate_cumulo+RQ_integral+AS_Vol. +emission_O2+emission_CO 2; 0.702, 0.860, tmp+AN_cumulo+RS+Feed_Vol+mu_cumulo+Sugar_ consuming_rate_cumulo+RQ_integral+AS_Vol.+emission_CO2; 0.6 93, 0.860, tmp+PL+RS+Feed_Vol+mu_cumulo+rho_cumulo+Sugar_co nsuming_rate_cumulo+RQ_integral+AS_Vol.+emission_O2+emissio n_CO2; 0.698, 0.860, tmp+AN_cumulo+RS+Feed_Vol+mu_cumulo+Su gar_consuming_rate_cumulo+RQ_integral+AS_Vol.+emission_O2+e mission_CO2; 0.688, 0.860, tmp+AN_cumulo+interval_yield+int erval_Pdt.+OD+RS+Feed_Vol+mu_cumulo+Sugar_consuming_rate_cu mulo+rab_integral+AS_Vol.+emission_O2; 0.688, 0.860, tmp+PL +AN_cumulo+interval_yield+interval_Pdt.+RS+Feed_Vol+mu-cumu lo+Sugar_consuming_rate_cumulo+rab_integral+AS_Vol.+emissio n_O2; 0.697, 0.860, tmp+AN_cumulo+interval_Pdt.+RS+Feed_Vol +mu_cumulo+rho_cumulo+Sugar_consuming_rate_cumulo+rab_integ ral+AS_Vol.; 0.688, 0.860, tmp+AN_cumulo+interval_Pdt.+RS+F eed_Vol+mu_cumulo+nu_per_rho_cumulo+Sugar_consuming_rate_cu mulo+rab_integral+AS_Vol.+emission_O2+emission_CO2; 0.687, 0.860, tmp+PL+AN_cumulo+RS+Feed_Vol+mu_cumulo+Sugar_consumi ng_rate_cumulo+RQ_integral+AS_Vol. +emission_O2+emission_CO2 +generate_CO2; 0.697, 0.860, tmp+AN_cumulo+interval_Pdt.+RS +Feed_Vol+mu_cumulo+Sugar_consuming_rate_cumulo+rab_integra l+AS_Vol.+generate_CO2; 0.682, 0.859, tmp+PL+interval_Pdt.+ RS+Feed_Vol+mu_cumulo+rho_cumulo+Sugar_consuming_rate_cumul o+RQ_integral+AS-Vol.+emission-O2+emission-CO2+generate-CO 2; 0.706, 0.859, tmp+AN_cumulo+RS+Feed_Vol+mu_cumulo+Sugar_ consuming_rate_cumulo+RQ_integral+AS_Vol.; 0.706, 0.859, tm p+AN_cumulo+interval_Pdt.+RS+Feed_Vol+mu_cumulo+Sugar_consu ming_rate_cumulo+AS_Vol.; 0.692, 0.859, tmp+AN_cumulo+inter val_Pdt.+RS+Feed_Vol+mu_cumulo+Sugar_consuming_rate_cumulo+ rab_integral+AS_Vol.+emission_O2+emission_CO2; 0.687, 0.859, tmp+AN_cumulo+interval_Pdt.+RS+Feed_Vol+mu_cumulo+Sugar_co nsuming_rate_cumulo+RQ_integral+AS_Vol.+emission_O2+emissio n_CO2+generate_CO2; 0.697, 0.859, tmp+interval_Pdt.+OD+RS+F eed_Vol+mu_cumulo+rho_cumulo+Sugar_consuming_rate_cumulo+ra b_integral+AS_Vol.; 0.697, 0.859, tmp+AN+AN_cumulo+interval _Pdt.+RS+Feed_Vol+mu_cumulo+Sugar_consuming_rate_cumulo+rab _integral+AS_Vol.; 0.682, 0.859, tmp+PL+AN+interval_Pdt.+RS +Feed_Vol+mu_cumulo+rho_cumulo+Sugar_consuming_rate_cumulo+ RQ_integral+AS_Vol.+emission_O2+emission_CO2; 0.701, 0.859, tmp+interval_Pdt.+RS+Feed_Vol+mu_cumulo+nu_per_rho_cumulo+ Sugar_consuming_rate_cumulo+rab_integral+AS_Vol.; 0.692, 0. 859, tmp+AN+RS+Feed_Vol+mu_cumulo+Sugar_consuming_rate_cumu lo+RQ_integral+AS_Vol.+emission_O2+emission_CO2+generate_CO 2; 0.701, 0.859, tmp+RS+Feed_Vol+mu_cumulo+Sugar_consuming_ rate_cumulo+RQ_integral+AS_Vol.+emission_O2+emission_CO2; 0. 701, 0.859, tmp+AN_cumulo+interval_Pdt.+RS+Feed_Vol+mu_cumu lo+Sugar_consuming_rate_cumulo+AS_Vol.+generate_CO2; 0.686, 0.859, tmp+interval_Pdt.+RS+Feed_Vol+mu_cumulo+rho_cumulo+ Sugar_consuming_rate_cumulo+RQ_integral+AS_Vol.+emission_O2 +emission_CO2+generate_CO2; 0.701, 0.859, tmp+AN+interval_P dt.+RS+Feed_Vol+mu_cumulo+Sugar_consuming_rate_cumulo+rab_i ntegral+AS_Vol.; 0.701, 0.859, tmp+AN_cumulo+RS+Feed_Vol+mu _cumulo+nu_per_rho_cumulo+Sugar_consuming_rate_cumulo+rab_i ntegral+AS_Vol.; 0.701, 0.859, tmp+AN_cumulo+RS+Feed_Vol+mu _cumulo+Sugar_consuming_rate_cumulo+AS_Vol.+emission_CO2+ge nerate_CO2; 0.681, 0.859, tmp+PL+AN_cumulo+interval_Pdt.+RS +Feed_Vol+mu_cumulo+nu_per_rho_cumulo+Sugar_consuming_rate_ cumulo+RQ_integral+ASVol.+emission_O2+emission_CO2; 0.686, 0.859, tmp+PL+RS+Feed_Vol+mu_cumulo+rho_cumulo+Sugar_consu ming_rate_cumulo+RQ_integral+AS_Vol.+emission_O2+emission_C O2+generate_CO2; 0.686, 0.859, tmp+AN+AN_cumulo+RS+Feed_Vol +mu_cumulo+Sugar_consuming_rate_cumulo+RQ_integral+AS_Vol.+ emission_O2+emission_CO2+generate_CO2; 0.696, 0.859, tmp+in terval_Pdt.+OD+RS+Feed_Vol+mu_cumulo+rho_cumulo+Sugar_consu ming_rate_cumulo+AS_Vol.+consum_O2; 0.696, 0.859, tmp+AN_cu mulo+interval_yield+interval_Pdt.+RS+Feed_Vol+mu_cumulo+Sug ar_consuming_rate_cumulo+rab_integral+emission_O2; 0.675, 0. 859, tmp+PL+AN+interval_Pdt.+OD+RS+Feed_Vol+mu_cumulo+rho_c umulo+Sugar_consuming_rate_cumulo+RQ_integral+AS_Vol.+emiss ion_O2+emission_CO2; 0.691, 0.859, tmp+interval_yield+inter val_Pdt.+RS+Feed_Vol+mu_cumulo+Sugar_consuming_rate_cumulo+ rab_integral+AS_Vol.+emission_02+emission_CO2; 0.696, 0.859, tmp+AN_cumulo+interval_Pdt.+RS+Feed_Vol+mu_cumulo+Sugar_co nsuming_rate_cumulo+rab_integral+AS_Vol.+emission_CO2; 0.69 1, 0.859, tmp+AN_cumulo+interval_Pdt.+RS+Feed_Vol+mu_cumulo +Sugar_consuming_rate_cumulo+rab_integral+RQ_integral+AS_Vo l.+emission_O2; 0.686, 0.859, tmp+AN_cumulo+interval_yield+ interval_Pdt.+RS+Feed_Vol+mu_cumulo+nu_per_rho_cumulo+Sugar _consuming_rate_cumulo+rab_integral+AS_Vol.+emission_O2; 0. 675, 0.859, tmp+PL+AN_cumulo+interval_Pdt.+OD+RS+Feed_Vol+m u_cumulo+rho_cumulo+Sugar_consuming_rate_cumulo+RQ_integral +AS_Vol.+emission_O2+emission_CO2; 0.701, 0.859, tmp+RS+Fee d_Vol+mu_cumulo+Sugar_consuming_rate_cumulo+RQ_integral+AS_ Vol.+emission_CO2+generate_CO2; 0.686, 0.859, tmp+PL+interv al_Pdt.+RS+Feed_Vol+mu_cumulo+Sugar_consuming_rate_cumulo+R Q_integral+AS_Vol.+emission_O2+emission_CO2+generate_CO2; 0. 691, 0.859, tmp+AN+AN_cumulo+interval_Pdt.+RS+Feed_Vol+mu_c umulo+Sugar_consuming_rate_cumulo+rab_integral+AS_Vol.+emis sion_O2; 0.709, 0.859, tmp+interval_Pdt.+RS+Feed_Vol+mu_cum ulo+Sugar_consuming_rate_cumulo+AS_Vol.; 0.696, 0.859, tmp+ AN_cumulo+interval_Pdt.+RS+Feed_Vol+mu_cumulo+Sugar_consumi ng_rate_cumulo+rab_integral+RQ_integral+AS_Vol.; 0.696, 0.8 59, tmp+interval_Pdt.+RS+Feed_Vol+mu_cumulo+Sugar_consuming _rate_cumulo+rab_integral+AS_Vol.+emission_O2+emission_CO2; 0.691, 0.859, tmp+AN_cumulo+interval_yield+interval_Pdt.+R S+Feed_Vol+mu_cumulo+nu_per_rho_cumulo+Sugar_consuming_rate _cumulo+rab_integral+AS_Vol.; 0.686, 0.859, tmp+PL+AN_cumul o+interval_Pdt.+RS+Feed_Vol+mu_cumulo+nu_per_rho_cumulo+Sug ar_consuming_rate_cumulo+rab_integral+AS_Vol.+emission_O2; 0.691, 0.859, tmp+PL+AN_cumulo+RS+Feed_Vol+mu_cumulo+Sugar_ consuming_rate cumulo+RQ_integral+AS_Vol.+emission_O2+emiss ion_CO2; 0.695, 0.859, tmp+AN_cumulo+interval_yield+RS+Feed Vol+mu_cumulo+nu_per_rho_cumulo+Sugar_consuming_rate_cumul o+rab_integral+AS_Vol.; 0.690, 0.859, tmp+PL+RS+Feed_Vol+mu _cumulo+Sugar_consuming_rate_cumulo+RQ_integral+AS_Vol.+emi ssion_O2+emission_CO2+generate_CO2; 0.685, 0.859, tmp+AN+in terval_Pdt.+RS+Feed_Vol+mu_cumulo+rho_cumulo+Sugar_consumin g_rate_cumulo+rab_integral+AS_Vol.+emission_O2+emission_CO 2; 0.700, 0.858, tmp+AN_cumulo+interval_yield+RS+Feed_Vol+m u_cumulo+Sugar_consuming_rate_cumulo+RQ_integral+AS_Vol.; 0. 695, 0.858, tmp+AN_cumulo+interval_Pdt.+RS+Feed_Vol+mu_cumu lo+Sugar_consuming_rate_cumulo+rab_integral+RQ_integral+emi ssion_O2; 0.690, 0.858, tmp+AN_cumulo+interval_Pdt.+RS+Feed _Vol+mu_cumulo+rho_cumulo+Sugar_consuming_rate_cumulo+rab_i ntegral+AS_Vol.+emission_O2; 0.685, 0.858, tmp+AN+interval_ Pdt.+RS+Feed_Vol+mu_cumulo+nu_per_rho_cumulo+Sugar_consumin grate_cumulo+rab_integral+AS_Vol.+emission_O2+emission_CO 2; 0.695, 0.858, tmp+AN+AN_cumulo+interval_Pdt.+RS+Feed_Vol +mu_cumulo+Sugar_consuming_rate_cumulo+rab_integral+emissio n_O2; 0.695, 0.858, tmp+AN_cumulo+interval_Pdt.+RS+Feed_Vol +mu_cumulo+nu_per_rho_cumulo+Sugar_consuming_rate_cumulo+ra b_integral+emission_O2; 0.685, 0.858, tmp+AN+interval_yield +interval_Pdt.+RS+Feed_Vol+mu_cumulo+Sugar_consuming_rate_c umulo+rab_integral+ASVol.+emission_O2+emission_CO2; 0.690, 0.858, tmp+AN_cumulo+RS+Feed_Vol+mu_cumulo+nu_per_rho_cumu lo+Sugar_consuming_rate_cumulo+RQ_integral+AS_Vol.+emission _CO2+generate_CO2; 0.695, 0.858, tmp+interval_Pdt.+RS+Feed_ Vol+mu_cumulo+nu_per_rho_cumulo+Sugar_consuming_rate_cumulo +AS_Vol.+emission_CO2+generate_CO2; 0.680, 0.858, tmp+AN_cu mulo+interval_Pdt.+RS+Feed_Vol+mu_cumulo+nu_per_rho_cumulo+ Sugar_consuming_rate_cumulo+RQ_integral+AS_Vol.+emission_O2 +emission_CO2+generate_CO2; 0.700, 0.858, tmp+interval_Pdt. +RS+Feed_Vol+mu_cumulo+rho_cumulo+Sugar_consuming_rate_cumu lo+rab_integral+AS_Vol.; 0.695, 0.858, tmp+RS+Feed_Vol+mu_c umulo+nu_per_rho_cumulo+Sugar_consuming_rate_cumulo+RQ_inte gral+AS_Vol.+emission_O2+emission_CO2; 0.704, 0.858, tmp+RS +Feed_Vol+mu_cumulo+Sugar_consuming_rate_cumulo+rab_integra l+AS_Vol.+emission_CO2; 0.685, 0.858, tmp+AN_cumulo+interva l_Pdt.+OD+RS+Feed_Vol+mu_cumulo+rho_cumulo+Sugar_consuming_ rate_cumulo+RQ_integral+AS_Vol.+emission_O2; 0.690, 0.858, tmp+AN_cumulo+RS+Feed_Vol+mu_cumulo+nu_per_rho_cumulo+Sugar consuming_rate_cumulo+RQ_integral+AS_Vol.+emission_O2+emis sion_CO2; 0.695, 0.858, tmp+AN_cumulo+RS+Feed_Vol+mu_cumulo +nu_per_rho_cumulo+Sugar_consuming_rate_cumulo+rab_integral +AS_Vol.+emission_CO2; 0.699, 0.858, tmp+RS+Feed_Vol+mu_cum ulo+nu_per_rho_cumulo+Sugar_consuming_rate_cumulo+AS_Vol.+e mission_CO2+generate_CO2; 0.690, 0.858, tmp+OD+RS+Feed_Vol+ mu_cumulo+Sugar_consuming_rate_cumulo+RQ_integral+AS_Vol.+e mission_O2+emission_CO2+generate_CO2; 0.679, 0.858, tmp+AN_ cumulo+interval_yield+interval_Pdt.+OD+RS+Feed_Vol+mu_cumul o+Sugar_consuming_rate_cumulo+rab_integral+AS_Vol.+emission _O2+emission_CO2; 0.674, 0.858, tmp+PL+AN_cumulo+interval_P dt.+RS+Feed_Vol+mu_cumulo+nu_per_rho_cumulo+Sugar_consuming rate_cumulo+RQ_integral+AS_Vol.+emission_O2+emission_CO2+g enerate_CO2; 0.689, 0.858, tmp+PL+interval_Pdt.+RS+Feed_Vol +mu_cumulo+Sugar_consuming_rate_cumulo+RQ_integral+AS_Vol.+ emission_O2+emission_CO2; 0.684, 0.858, tmp+interval_Pdt.+O D+RS+Feed_Vol+mu_cumulo+rho_cumulo+Sugar_consuming_rate_cum ulo+rab_integral+AS_Vol.+emission_O2+emission_CO2; 0.684, 0. 858, tmp+AN_cumulo+interval_Pdt.+OD+RS+Feed_Vol+mu_cumulo+r ho_cumulo+Sugar_consuming_rate_cumulo+rab_integral+AS_Vol.+ emission_O2; 0.684, 0.858, tmp+AN+interval_Pdt.+RS+Feed_Vol +mu_cumulo+Sugar_consuming_rate_cumulo+RQ_integral+AS_Vol.+ emission_O2+emission_CO2+generate_CO2; 0.689, 0.858, tmp+PL +AN_cumulo+interval_Pdt.+RS+Feed_Vol+mu_cumulo+nu_per_rho_c umulo+Sugar_consuming_rate_cumulo+rab_integral+AS_Vol.; 0.6 79, 0.858, tmp+AN_cumulo+interval_yield+interval_Pdt.+RS+Fe ed_Vol+mu_cumulo+nu_per_rho_cumulo+Sugar_consuming_rate_cum ulo+rab_integral+AS_Vol.+emission_O2+emission_CO2; 0.689, 0. 858, tmp+RS+Feed_Vol+mu_cumulo+nu_cumulo+Sugar_consuming_ra te_cumulo+RQ_integral+AS_Vol.+emission_O2+emission_CO2+gene rate_CO2; 0.684, 0.858, tmp+AN+RS+Feed_Vol+mu_cumulo+rho_cu mulo+Sugar_consuming_rate_cumulo+RQ_integral+AS_Vol.+emissi on_O2+emission_CO2+generate_CO2; 0.679, 0.858, tmp+PL+inter val_Pdt.+RS+Feed_Vol+mu_cumulo+rho_cumulo+Sugar_consuming_r ate_cumulo+rab_integral+RQ_integral+AS_Vol.+emission_O2+emi ssion_CO2; 0.689, 0.858, tmp+AN_cumulo+interval_Pdt.+OD+RS+ Feed_Vol+mu_cumulo+rho_cumulo+Sugar_consuming_rate_cumulo+R Q_integral+AS_Vol.; 0.689, 0.858, tmp+interval_Pdt.+RS+Feed _Vol+mu_cumulo+nu_per_rho_cumulo+Sugar_consuming_rate_cumul o+RQ_integral+AS_Vol.+emission_O2+emission_CO2; 0.684, 0.85 8, tmp+AN_cumulo+interval_Pdt.+RS+Feed_Vol+mu_cumulo+rho_cu mulo+Sugar_consuming_rate_cumulo+rab_integral+AS_Vol.+emiss ion-O2+emission-CO2; 0.679, 0.858, tmp+AN_cumulo+interval_y ield+interval_Pdt.+RS+Feed_Vol+mu_cumulo+Sugar_consuming_ra te_cumulo+rab_integral+AS_Vol.+emission_O2+emission_CO2+gen erate_CO2; 0.684, 0.858, tmp+PL+AN_cumulo+RS+Feed_Vol+mu_cu mulo+rho_cumulo+Sugar_consuming_rate_cumulo+RQ_integral+AS_ Vol.+emission_O2+emission_CO2; 0.694, 0.858, tmp+PL+RS+Feed Vol+mu_cumulo+Sugar_consuming_rate_cumulo+RQ_integral+AS_V ol.+emission_O2+emission_CO2; 0.694, 0.858, tmp+AN_cumulo+R S+Feed_Vol+mu_cumulo+nu_per_rho_cumulo+Sugar_consuming_rate _cumulo+RQ_integral+AS_Vol.+emission_CO2; 0.694, 0.858, tmp +AN_cumulo+interval_Pdt.+RS+Feed_Vol+mu_cumulo+Sugar_consum ing_rate_cumulo+AS_Vol.+emission_CO2+generate_CO2; 0.679, 0. 858, tmp+PL+AN_cumulo+RS+Feed_Vol+mu_cumulo+nu_per_rho_cumu lo+Sugar_consuming_rate_cumulo+RQ_integral+AS_Vol.+emission _O2+emission_CO2+generate_CO2; 0.684, 0.858, tmp+interval_y ield+interval_Pdt.+RS+Feed_Vol+mu_cumulo+Sugar_consuming_ra te_cumulo+RQ_integral+AS_Vol.+emission_O2+emission_CO2+gene rate_CO2; 0.689, 0.858, tmp+PL+AN_cumulo+interval_yield+int erval_Pdt.+RS+Feed_Vol+mu_cumulo+Sugar_consuming_rate_cumul o+rab_integral+AS_Vol.; 0.679, 0.858, tmp+PL+interval_Pdt.+ RS+Feed_Vol+mu_cumulo+nu_per_rho_cumulo+Sugar_consuming_rat e_cumulo+rab_integral+RQ_integral+AS_Vol.+emission_O2+emiss ion_CO2; 0.679, 0.858, tmp+PL+AN_cumulo+interval_yield+inte rval_Pdt.+RS+Feed_Vol+mu_cumulo+Sugar_consuming_rate_cumulo +rab_integral+AS_Vol.+emission_O2+emission_CO2; 0.699, 0.85 8, tmp+RS+Feed_Vol+mu_cumulo+rho_cumulo+Sugar_consuming_rat e_cumulo+rab_integral+AS_Vol.+emission_CO2; 0.694, 0.858, t mp+AN_cumulo+interval_Pdt.+RS+Feed_Vol+mu_cumulo+nu_cumulo+ Sugar_consuming_rate_cumulo+rab_integral+AS_Vol.; 0.679, 0. 858, tmp+PL+AN_cumulo+interval_Pdt.+OD+RS+Feed_Vol+mu_cumul o+rho_cumulo+Sugar_consuming_rate_cumulo+RQ_integral+AS_Vol. +emission_O2; 0.694, 0.858, tmp+RS+Feed_Vol+mu_cumulo+rho_c umulo+Sugar_consuming_rate_cumulo+RQ_integral+AS_Vol.+emiss ion_O2+emission_CO2; 0.699, 0.858, tmp+AN_cumulo+RS+Feed_Vo l+mu_cumulo+rho_cumulo+Sugar_consuming_rate_cumulo+rab_inte gral+AS_Vol.; 0.699, 0.858, tmp+AN_cumulo+interval_Pdt.+RS+ Feed_Vol+mu_cumulo+Sugar_consuming_rate_cumulo+RQ_integral+ emission_O2; 0.684, 0.858, tmp+PL+AN+RS+Feed_Vol+mu_cumulo+ rho_cumulo+Sugar_consuming_rate_cumulo+RQ_integral+AS_Vol.+ emission_O2+emission_CO2; 0.699, 0.858, tmp+AN+RS+Feed_Vol+ mu_cumulo+Sugar_consuming_rate_cumulo+AS_Vol.+emission_CO2+ generate_CO2; 0.694, 0.858, tmp+AN_cumulo+interval_Pdt.+RS+ Feed_Vol+mu_cumulo+nu_per_rho_cumulo+Sugar_consuming_rate_c umulo+RQ_integral+AS_Vol.; 0.678, 0.858, tmp+PL+AN_cumulo+i nterval_Pdt.+RS+Feed_Vol+mu_cumulo+Sugar_consuming_rate_cum ulo+RQ_integral+AS_Vol.+emission_O2+emission_CO2+generate_C 02; 0.694, 0.858, tmp+AN_cumulo+RS+Feed_Vol+mu_cumulo+Sugar _consuming_rate_cumulo+rab_integral+AS_Vol.+emission_CO2+ge nerate_CO2; 0.678, 0.858, tmp+AN+interval_Pdt.+RS+Feed_Vol+ mu_cumulo+nu_per_rho_cumulo+Sugar_consuming_rate_cumulo+RQ_ integral+AS_Vol.+emission_O2+emission_CO2+generate_CO2 0.6 94, 0.858, tmp+AN+interval_Pdt.+RS+Feed_Vol+mu_cumulo+nu_pe r_rho_cumulo+Sugar_consuming_rate_cumulo+rab_integral+AS_Vo l.; 0.684, 0.858, tmp+PL+interval_yield+RS+Feed_Vol+mu_cumu lo+rho_cumulo+Sugar_consuming_rate_cumulo+RQ_integral+AS_Vo l.+emission_O2+emission_CO2; 0.689, 0.858, tmp+interval_yie ld+RS+Feed_Vol+mu_cumulo+Sugar_consuming_rate_cumulo+RQ_int egral+AS_Vol.+emission_O2+emission_CO2+generate_CO2; 0.689, 0.858, tmp+AN_cumulo+interval_Pdt.+RS+Feed_Vol+mu_cumulo+n u_per_rho_cumulo+Sugar_consuming_rate_cumulo+rab_integral+A S_Vol.+emission_CO2; 0.698, 0.858, tmp+AN+AN_cumulo+RS+Feed _Vol+mu_cumulo+Sugar_consuming_rate_cumulo+rab_integral+AS_ Vol.; 0.689, 0.858, tmp+PL+AN_cumulo+interval_Pdt.+RS+Feed_ Vol+mu_cumulo+Sugar_consuming_rate_cumulo+RQ_integral+AS_Vo l.+emission_O2; 0.684, 0.858, tmp+AN+RS+Feed_Vol+mu_cumulo+ nu_per_rho_cumulo+Sugar_consuming_rate_cumulo+RQ_integral+A S_Vol.+emission_O2+emission_CO2+generate_CO2; 0.694, 0.858, tmp+AN_cumulo+RS+Feed_Vol+mu_cumulo+rho_cumulo+Sugar_consu ming_rate_cumulo+rab_integral+AS_Vol.+emission_CO2; 0.694, 0.858, tmp+AN+interval_Pdt.+RS+Feed_Vol+mu_cumulo+Sugar_con suming_rate_cumulo+AS_Vol.+emission_CO2+generate_CO2; 0.693, 0.858, tmp+AN+interval_Pdt.+OD+RS+Feed_Vol+mu_cumulo+rho_c umulo+Sugar_consuming_rate_cumulo+AS_Vol.; 0.689, 0.858, tm p+AN_cumulo+interval_Pdt.+RS+Feed_Vol+mu_cumulo+nu_per_rho_ cumulo+Sugar_consuming_rate_cumulo+rab_integral+AS_Vol.+gen erate_CO2; 0.698, 0.858, tmp+interval_yield+RS+Feed_Vol+mu_ cumulo+Sugar_consuming_rate_cumulo+AS_Vol.+emission_CO2+gen erate_CO2; 0.693, 0.858, tmp+AN_cumulo+interval_Pdt.+OD+RS+ Feed_Vol+mu_cumulo+rho_cumulo+Sugar_consuming_rate_cumulo+A S_Vol.; 0.678, 0.858, tmp+PL+AN_cumulo+RS+Feed_Vol+mu_cumul o+rho_cumulo+Sugar_consuming_rate_cumulo+RQ_integral+AS_Vol. +emission_O2+emission_CO2+generate_CO2; 0.683, 0.858, tmp+P L+AN_cumulo+RS+Feed_Vol+mu_cumulo+nu_per_rho_cumulo+Sugar_c onsuming_rate_cumulo+RQ_integral+AS_Vol.+emission_02+emissi on_CO2; 0.678, 0.858, tmp+AN_cumulo+interval_yield+interval _Pdt.+OD+RS+Feed_Vol+mu_cumulo+rho_cumulo+Sugar_consuming_r ate_cumulo+rab_integral+AS_Vol.+emission_O2; 0.688, 0.858, tmp+AN+AN_cumulo+interval_Pdt.+RS+Feed_Vol+mu_cumulo+nu_per _rho_cumulo+Sugar_consuming_rate_cumulo+rab_integral+AS_Vo l.; 0.698, 0.858, tmp+PL+AN_cumulo+RS+Feed_Vol+mu_cumulo+Su gar_consuming_rate_cumulo+rab_integral+AS_Vol.; 0.688, 0.85 7, tmp+AN_cumulo+interval_Pdt.+OD+RS+Feed_Vol+mu_cumulo+Sug ar_consuming_rate_cumulo+RQ_integral+AS_Vol.+emission_O2; 0. 698, 0.857, tmp+RS+Feed_Vol+mu_cumulo+Sugar_consuming_rate_ cumulo+rab_integral+AS_Vol.+emission_CO2+generate_CO2; 0.68 3, 0.857, tmp+interval_yield+RS+Feed_Vol+mu_cumulo+nu_per_r ho_cumulo+Sugar_consuming_rate_cumulo+RQ_integral+AS_Vol.+e mission_O2+emission_CO2+generate_CO2; 0.688, 0.857, tmp+PL+ RS+Feed_Vol+mu_cumulo+nu_per_rho_cumulo+Sugar_consuming_rat e_cumulo+RQ_integral+AS_Vol.+emission_O2+emission_CO2; 0.69 8, 0.857, tmp+AN_cumulo+interval_Pdt.+RS+Feed_Vol+mu_cumulo +nu_per_rho_cumulo+Sugar_consuming_rate_cumulo+AS_Vol.; 0.6 98, 0.857, tmp+interval_yield+interval_Pdt.+RS+Feed_Vol+mu_ cumulo+Sugar_consuming_rate_cumulo+rab_integral+AS_Vol.; 0. 693, 0.857, tmp+AN_cumulo+interval_Pdt.+OD+RS+Feed_Vol+mu_c umulo+Sugar_consuming_rate_cumulo+AS_Vol.+generate_CO2; 0.6 98, 0.857, tmp+RS+Feed_Vol+mu_cumulo+rho_cumulo+Sugar_consu ming_rate_cumulo+AS_Vol.+emission_CO2+generate_CO2; 0.698, 0.857, tmp+interval_Pdt.+RS+Feed_Vol+mu_cumulo+Sugar_consum ing_rate_cumulo+rab_integral+AS_Vol.+emission_CO2; 0.703, 0. 857, tmp+AN+interval_Pdt.+RS+Feed_Vol+mu_cumulo+Sugar_consu ming_rate_cumulo+AS_Vol.; 0.698, 0.857, tmp+AN_cumulo+RS+Fe ed_Vol+mu_cumulo+Sugar_consuming_rate_cumulo+rab_integral+R Q_integral+AS_Vol.; 0.693, 0.857, tmp+interval_Pdt.+RS+Feed _Vol+mu_cumulo+nu_per_rho_cumulo+Sugar_consuming_rate_cumul o+rab_integral+AS_Vol.+emission_CO2; 0.702, 0.857, tmp+inte rval_Pdt.+RS+Feed_Vol+mu_cumulo+nu_per_rho_cumulo+Sugar_con suming_rate_cumulo+AS_Vol.; 0.683, 0.857, tmp+AN_cumulo+RS+ Feed_Vol+mu_cumulo+nu_cumulo+Sugar_consuming_rate_cumulo+RQ _integral+AS_Vol.+emission_O2+emission_CO2+generate_CO2; 0. 688, 0.857, tmp+AN+AN_cumulo+RS+Feed_Vol+mu_cumulo+Sugar_co nsuming_rate_cumulo+RQ_integral+AS_Vol.+emission_CO2+genera te_CO2; 0.688, 0.857, tmp+interval_Pdt.+RS+Feed_Vol+mu_cumu lo+Sugar_consuming_rate_cumulo+rab_integral+AS_Vol.+emissio n_O2+emission_CO2+generate_CO2; 0.683, 0.857, tmp+interval_ Pdt.+RS+Feed_Vol+mu_cumulo+nu_per_rho_cumulo+Sugar_consumin g_rate_cumulo+rab_integral+RQ_integral+AS_Vol.+emission_O2+ emission_CO2; 0.693, 0.857, tmp+AN_cumulo+interval_Pdt.+OD+ RS+Feed_Vol+mu_cumulo+Sugar_consuming_rate_cumulo+RQ_integr al+AS_Vol.; 0.688, 0.857, tmp+AN+AN_cumulo+interval_yield+i nterval_Pdt.+RS+Feed_Vol+mu_cumulo+Sugar_consuming_rate_cum ulo+rab_integral+emission_O2; 0.672, 0.857, tmp+PL+AN+inter val_Pdt.+RS+Feed_Vol+mu_cumulo+nu_per_rho_cumulo+Sugar_cons uming_rate_cumulo+RQ_integral+AS_Vol.+emission_O2+emission_ CO2+generate_CO2; 0.693, 0.857, tmp+AN_cumulo+interval_Pdt. +RS+Feed_Vol+mu_cumulo+Sugar_consuming_rate_cumulo+RQ_integ ral+AS_Vol.+emission_O2; 0.683, 0.857, tmp+PL+interval_Pdt. +RS+Feed_Vol+mu_cumulo+rho_cumulo+Sugar_consuming_rate_cumu lo+rab_integral+AS_Vol.+emission_O2+emission_CO2; 0.672, 0. 857, tmp+PL+interval_Pdt.+OD+RS+Feed_Vol+mu_cumulo+rho_cumu lo+Sugar_consuming_rate_cumulo+RQ_integral+AS_Vol.+emission _O2+emission_CO2+generate_CO2; 0.682, 0.857, tmp+PL+RS+Feed _Vol+mu_cumulo+nu_per_rho_cumulo+Sugar_consuming_rate_cumul o+RQ_integral+AS_Vol.+emission_O2+emission_CO2+generate_CO 2; 0.682, 0.857, tmp+interval_Pdt.+RS+Feed_Vol+mu_cumulo+rh o_cumulo+Sugar_consuming_rate_cumulo+rab_integral+AS_Vol.+e mission_O2+emission_CO2+generate_CO2; 0.682, 0.857, tmp+AN_ cumulo+OD+RS+Feed_Vol+mu_cumulo+Sugar_consuming_rate_cumulo +RQ_integral+AS_Vol.+emission_O2+emission_CO2+generate_CO2; 0.682, 0.857, tmp+interval_Pdt.+RS+Feed_Vol+mu_cumulo+nu_p er_rho_cumulo+Sugar_consuming_rate_cumulo+rab_integral+AS_V ol.+emission_O2+emission_CO2+generate_CO2; 0.688, 0.857, tm p+AN+interval_Pdt.+RS+Feed_Vol+mu_cumulo+Sugar_consuming_ra te_cumulo+rab_integral+AS_Vol.+emission_O2+emission_CO2; 0. 682, 0.857, tmp+interval_yield+interval_Pdt.+RS+Feed_Vol+mu _cumulo+Sugar_consuming_rate_cumulo+rab_integral+AS_Vol.+em ission_O2+emission_CO2+generate_CO2; 0.682, 0.857, tmp+AN+A N_cumulo+interval_yield+interval_Pdt.+RS+Feed_Vol+mu_cumulo +Sugar_consuming_rate_cumulo+rab_integral+AS_Vol.+emission_ 02; 0.682, 0.857, tmp+RS+Feed_Vol+mu_cumulo+rho_cumulo+nu_p er_rho_cumulo+Sugar_consuming_rate_cumulo+RQ_integral+AS_Vo l.+emission_O2+emission_CO2+generate_CO2; 0.677, 0.857, tmp +AN_cumulo+interval_yield+interval_Pdt.+RS+Feed_Vol+mu_cumu lo+rho_cumulo+Sugar_consuming_rate_cumulo+rab_integral+AS_V ol.+emission_O2+emission_CO2; 0.672, 0.857, tmp+PL+AN+inter val_Pdt.+RS+Feed_Vol+mu_cumulo+rho_cumulo+Sugar_consuming_r ate_cumulo+RQ_integral+AS_Vol.+emission_O2+emission_CO2+gen erate_CO2; 0.687, 0.857, tmp+AN_cumulo+RS+Feed_Vol+mu_cumul o+rho_cumulo+Sugar_consuming_rate_cumulo+RQ_integral+AS_Vol. +emission_CO2+generate_CO2; 0.677, 0.857, tmp+PL+interval_y ield+interval_Pdt.+RS+Feed_Vol+mu_cumulo+nu_per_rho_cumulo+ Sugar_consuming_rate_cumulo+RQ_integral+AS_Vol.+emission_O2 +emission_CO2; 0.682, 0.857, tmp+PL+interval_yield+RS+Feed_ Vol+mu_cumulo+nu_per_rho_cumulo+Sugar_consuming_rate_cumulo +RQ_integral+AS_Vol.+emission_O2+emission_CO2; 0.682, 0.857, tmp+PL+AN_cumulo+interval_Pdt.+RS+Feed_Vol+mu_cumulo+Sugar _consuming_rate_cumulo+RQ_integral+AS_Vol.+emission_O2+emis sion_CO2; 0.687, 0.857, tmp+AN_cumulo+RS+Feed_Vol+mu_cumulo +Sugar_consuming_rate_cumulo+rab_integral+RQ_integral+AS_Vo l.+emission_O2+emission_CO2; 0.687, 0.857, tmp+AN+interval_ Pdt.+OD+RS+Feed_Vol+mu_cumulo+rho_cumulo+Sugar_consuming_ra te_cumulo+rab_integral+AS_Vol.; 0.692, 0.857, tmp+RS+Feed_V ol+mu_cumulo+nu_per_rho_cumulo+Sugar_consuming_rate_cumulo+ RQ_integral+AS_Vol.+emission_CO2+generate_CO2; 0.697, 0.857, tmp+pH+AN_cumulo+RS+Feed_Vol+mu_cumulo+Sugar_consuming_rat e_cumulo+rab_integral+AS_Vol.; 0.687, 0.857, tmp+AN_cumulo+ interval_yield+interval_Pdt.+RS+Feed_Vol+mu_cumulo+Sugar_co nsuming_rate_cumulo+rab_integral+AS_Vol.+generate_CO2; 0.69 2, 0.857, tmp+interval_Pdt.+RS+Feed_Vol+mu_cumulo+Sugar_con suming_rate_cumulo+RQ_integral+AS_Vol.+emission_O2+emission _CO2; 0.692, 0.857, tmp+AN_cumulo+RS+Feed_Vol+mu_cumulo+Sug ar_consuming_rate_cumulo+rab_integral+RQ_integral+AS_Vol.+e mission_CO2; 0.671, 0.857, tmp+PL+AN+interval_Pdt.+RS+Feed_ Vol+mu_cumulo+rho_cumulo+Sugar_consuming_rate_cumulo+rab_in tegral+RQ_integral+AS_Vol.+emission_O2+emission_CO2; 0.692, 0.857, tmp+AN+interval_Pdt.+RS+Feed_Vol+mu_cumulo+rho_cumu lo+Sugar_consuming_rate_cumulo+rab_integral+AS_Vol.; 0.692, 0.857, tmp+PL+AN_cumulo+interval_Pdt.+RS+Feed_Vol+mu_cumul o+Sugar_consuming_rate_cumulo+RQ_integral+AS_Vol.; 0.677, 0. 857, tmp+AN+AN_cumulo+RS+Feed_Vol+mu_cumulo+nu_per_rho_cumu lo+Sugar_consuming_rate_cumulo+RQ_integral+AS_Vol.+emission O2+emission_CO2+generate_CO2; 0.677, 0.857, tmp+PL+interva l_yield+interval_Pdt.+RS+Feed_Vol+mu_cumulo+Sugar_consuming rate_cumulo+RQ_integral+AS_Vol.+emission_O2+emission_CO2+g enerate_CO2; 0.692, 0.857, tmp+AN+RS+Feed_Vol+mu_cumulo+Sug ar_consuming_rate_cumulo+RQ_integral+AS_Vol.+emission_O2+em ission_CO2; 0.687, 0.857, tmp+AN+AN_cumulo+interval_yield+i nterval_Pdt.+RS+Feed_Vol+mu_cumulo+Sugar_consuming_rate_cum ulo+rab_integral+AS_Vol.; 0.677, 0.857, tmp+PL+interval_Pdt. +RS+Feed_Vol+mu_cumulo+rho_cumulo+nu_per_rho_cumulo+Sugar_c onsuming_rate_cumulo+RQ_integral+AS_Vol.+emission_O2+emissi on_CO2; 0.692, 0.857, tmp+AN_cumulo+RS+Feed_Vol+mu_cumulo+n u_per_rho_cumulo+Sugar_consuming_rate_cumulo+AS_Vol.+emissi on-CO2+generate-CO2; 0.692, 0.857, tmp+AN_cumulo+interval_y ield+RS+Feed_Vol+mu_cumulo+nu_per_rho_cumulo+Sugar_consumin g_rate_cumulo+RQ_integral+AS_Vol.; 0.687, 0.857, tmp+AN_cum ulo+RS+Feed_Vol+mu_cumulo+rho_cumulo+Sugar_consuming_rate_c umulo+RQ_integral+AS_Vol.+emission_O2+emission_CO2; 0.682, 0.857, tmp+OD+RS+Feed_Vol+mu_cumulo+rho_cumulo+Sugar_consum ing_rate_cumulo+RQ_integral+AS_Vol.+emission_O2+emission_CO 2+generate_CO2; 0.697, 0.857, tmp+RS+Feed_Vol+mu_cumulo+nu_ per_rho_cumulo+Sugar_consuming_rate_cumulo+rab_integral+AS_ Vol.+emission_CO2; 0.682, 0.857, tmp+AN+AN_cumulo+interval_ Pdt.+RS+Feed_Vol+mu_cumulo+nu_per_rho_cumulo+Sugar_consumin g_rate_cumulo+rab_integral+AS_Vol.+emission_O2; 0.701, 0.85 7, tmp+AN_cumulo+interval_yield+RS+Feed_Vol+mu_cumulo+Sugar consuming_rate_cumulo+AS_Vol.; 0.687, 0.857, tmp+AN_cumulo +interval_yield+interval_Pdt.+RS+Feed_Vol+mu_cumulo+rho_cum ulo+Sugar_consuming_rate_cumulo+rab_integral+AS_Vol.; 0.682, 0.857, tmp+AN_cumulo+interval_yield+interval_Pdt.+RS+Feed_ Vol+mu_cumulo+rho_cumulo+Sugar_consuming_rate_cumulo+rab_in tegral+AS_Vol.+emission_O2; 0.687, 0.857, tmp+AN_cumulo+int erval_Pdt.+RS+Feed_Vol+mu_cumulo+Sugar_consuming_rate_cumul o+rab_integral+AS_Vol.+emission_O2+generate_CO2; 0.687, 0.8 57, tmp+AN_cumulo+interval_yield+interval_Pdt.+RS+Feed_Vol+ mu_cumulo+Sugar_consuming_rate_cumulo+rab_integral+AS_Vol.+ emission_CO2; 0.676, 0.857, tmp+interval_Pdt.+OD+RS+Feed_Vo l+mu_cumulo+rho_cumulo+Sugar_consuming_rate_cumulo+RQ-integ ral+AS_Vol.+emission_O2+emission_CO2+generate_CO2; 0.682, 0. 857, tmp+interval_yield+interval_Pdt.+RS+Feed_Vol+mu_cumulo +nu_per_rho_cumulo+Sugar_consuming_rate_cumulo+rab_integral +AS_Vol.+emission_O2+emission_CO2; 0.692, 0.857, tmp+PL+AN_ cumulo+interval_Pdt.+RS+Feed_Vol+mu_cumulo+Sugar_consuming_ rate_cumulo+rab_integral+emission_O2; 0.692, 0.857, tmp+RS+ Feed_Vol+mu_cumulo+rho_cumulo+Sugar_consuming_rate_cumulo+R Q_integral+AS_Vol.+emission_CO2+generate_CO2; 0.687, 0.857, tmp+RS+Feed_Vol+mu_cumulo+rho_cumulo+Sugar_consuming_rate_ cumulo+rab_integral+RQ_integral+AS_Vol.+emission_O2+emissio n_CO2; 0.701, 0.857, tmp+RS+Feed_Vol+mu_cumulo+Sugar_consum ing_rate_cumulo+RQ_integral+AS_Vol.+emission_CO2; 0.701, 0. 857, tmp+interval_yield+RS+Feed_Vol+mu_cumulo+nu_per_rho_cu mulo+Sugar_consuming_rate_cumulo+AS_Vol.; 0.692, 0.857, tmp +interval_Pdt.+RS+Feed_Vol+mu_cumulo+Sugar_consuming_rate_c umulo+ASVol.+emission_O2+emission_CO2+generate_CO2; 0.696, 0.857, tmp+AN_cumulo+RS+Feed_Vol+mu_cumulo+nu_per_rho_cumu lo+Sugar_consuming_rate_cumulo+RQ_integral+AS_Vol.; 0.687, 0.857, tmp+AN_cumulo+interval_Pdt.+RS+Feed_Vol+mu_cumulo+nu _cumulo+Sugar_consuming_rate_cumulo+rab_integral+AS_Vol.+em ission_O2; 0.687, 0.857, tmp+AN_cumulo+interval_Pdt.+OD+RS+ Feed_Vol+mu_cumulo+rho_cumulo+Sugar_consuming_rate_cumulo+r ab_integral+AS_Vol.; 0.692, 0.857, tmp+RS+Feed_Vol+mu_cumul o+Sugar_consuming_rate_cumulo+rab_integral+RQ_integral+AS_V ol.+emission_O2+emission_CO2; 0.681, 0.857, tmp+AN_cumulo+i nterval_yield+interval_Pdt.+OD+RS+Feed_Vol+mu_cumulo+Sugar_ consuming_rate_cumulo+RQ_integral+AS_Vol.+emission_O2; 0.68 1, 0.857, tmp+interval_yield+RS+Feed_Vol+mu_cumulo+rho_cumu lo+Sugar_consuming_rate_cumulo+RQ_integral+AS_Vol.+emission _O2+emission_CO2+generate_CO2; 0.692, 0.857, tmp+interval_P dt.+RS+Feed_Vol+mu_cumulo+rho_cumulo+Sugar_consuming_rate_c umulo+rab_integral+AS_Vol.+emission_CO2; 0.681, 0.857, tmp+ PL+interval_Pdt.+RS+Feed_Vol+mu_cumulo+nu_per_rho_cumulo+Su gar_consuming_rate_cumulo+rab_integral+AS_Vol.+emission_O2+ emission_CO2; 0.681, 0.857, tmp+PL+AN_cumulo+interval_Pdt.+ RS+Feed_Vol+mu_cumulo+Sugar_consuming_rate_cumulo+rab_integ ral+RQ_integral+AS_Vol.+emission_O2; 0.681, 0.857, tmp+AN_c umulo+interval_yield+interval_Pdt.+RS+Feed_Vol+mu_cumulo+Su gar_consuming_rate_cumulo+rab_integral+RQ_integral+AS_Vol.+ emission_O2; 0.681, 0.857, tmp+interval_yield+interval_Pdt. +RS+Feed_Vol+mu_cumulo+rho_cumulo+Sugar_consuming_rate_cumu lo+rab_integral+AS_Vol.+emission_O2+emission_CO2; 0.691, 0. 857, tmp+interval_Pdt.+RS+Feed_Vol+mu_cumulo+rho_cumulo+Sug ar_consuming_rate_cumulo+AS_Vol.+emission_CO2+generate_CO2; 0.686, 0.857, tmp+interval_Pdt.+OD+RS+Feed_Vol+mu_cumulo+r ho_cumulo+Sugar_consuming_rate_cumulo+rab_integral+AS_Vol.+ consum-O2; 0.691, 0.857, tmp+AN_cumulo+interval_Pdt.+RS+Fee d_Vol+mu_cumulo+Sugar_consuming_rate_cumulo+RQ_integral+AS_ Vol.+generate_CO2; 0.691, 0.857, tmp+AN_cumulo+RS+Feed_Vol+ mu_cumulo+rho_cumulo+Sugar_consuming_rate_cumulo+RQ_integra l+AS_Vol.+emission_CO2; 0.681, 0.857, tmp+PL+AN+interval_Pd t.+RS+Feed_Vol+mu_cumulo+Sugar_consuming_rate_cumulo+RQ_int egral+AS_Vol.+emission_O2+emission_CO2; 0.676, 0.857, tmp+A N_cumulo+interval_yield+interval_Pdt.+RS+Feed_Vol+mu_cumulo +Sugar_consuming_rate_cumulo+RQ_integral+AS_Vol.+emission_O 2+emission_CO2+generate_CO2; 0.681, 0.857, tmp+interval_Pdt. +OD+RS+Feed_Vol+mu_cumulo+Sugar_consuming_rate_cumulo+RQ_in tegral+AS_Vol.+emission_O2+emission_CO2+generate_CO2; 0.681, 0.857, tmp+AN_cumulo+interval_Pdt.+RS+Feed_Vol+mu_cumulo+S ugar_consuming_rate_cumulo+rab_integral+AS_Vol.+emission_O2 +emission_CO2+generate_CO2; 0.670, 0.857, tmp+PL+AN+interva l_Pdt.+RS+Feed_Vol+mu_cumulo+nu_per_rho_cumulo+Sugar_consum ing_rate_cumulo+rab_integral+RQ_integral+AS_Vol.+emission_O 2+emission_CO2; 0.696, 0.856, tmp+AN_cumulo+RS+Feed_Vol+mu_ cumulo+Sugar_consuming_rate_cumulo+rab_integral+AS_Vol.+gen erate_CO2; 0.670, 0.856, tmp+PL+AN_cumulo+interval_yield+in terval_Pdt.+RS+Feed_Vol+mu_cumulo+Sugar_consuming_rate_cumu lo+RQ_integral+AS_Vol.+emission_O2+emission_CO2+generate_CO 2; 0.686, 0.856, tmp+interval_yield+RS+Feed_Vol+mu_cumulo+n u_per_rho_cumulo+Sugar_consuming_rate_cumulo+RQ_integral+AS _Vol.+emission_O2+emission_CO2; 0.676, 0.856, tmp+PL+interv al_Pdt.+OD+RS+Feed_Vol+mu_cumulo+nu_per_rho_cumulo+Sugar_co nsuming_rate_cumulo+RQ_integral+AS_Vol.+emission_O2+emissio n_CO2; 0.686, 0.856, tmp+AN_cumulo+interval_Pdt.+OD+RS+Feed _Vol+mu_cumulo+rho_cumulo+Sugar_consuming_rate_cumulo+AS_Vo l.+generate_CO2; 0.696, 0.856, tmp+AN_cumulo+OD+RS+Feed_Vol +mu_cumulo+Sugar_consuming_rate_cumulo+RQ_integral+AS_Vol.; 0.681, 0.856, tmp+RS+Feed_Vol+mu_cumulo+nu_cumulo+nu_per_r ho_cumulo+Sugar_consuming_rate_cumulo+RQ_integral+AS_Vol.+e mission_O2+emission_CO2+generate_CO2; 0.681, 0.856, tmp+int erval_Pdt.+OD+RS+Feed_Vol+mu_cumulo+rho_cumulo+Sugar_consum ing_rate_cumulo+RQ_integral+AS_Vol.+emission_O2+emission_CO 2; 0.676, 0.856, tmp+AN_cumulo+interval_yield+interval_Pdt. +OD+RS+Feed_Vol+mu_cumulo+nu_cumulo+Sugar_consuming_rate_cu mulo+rab_integral+AS_Vol.+emission_O2; 0.681, 0.856, tmp+PL +RS+Feed_Vol+mu_cumulo+rho_cumulo+Sugar_consuming_rate_cumu lo+rab_integral+RQ_integral+AS_Vol.+emission_O2+emission_CO 2; 0.691, 0.856, tmp+AN+interval_yield+interval_Pdt.+RS+Fee d_Vol+mu_cumulo+Sugar_consuming_rate_cumulo+rab_integral+AS _Vol.; 0.691, 0.856, tmp+interval_yield+RS+Feed_Vol+mu_cumu lo+nu_per_rho_cumulo+Sugar_consuming_rate_cumulo+AS_Vol.+em ission_CO2+generate_CO2; 0.670, 0.856, tmp+PL+interval_Pdt. +OD+RS+Feed_Vol+mu_cumulo+rho_cumulo+Sugar_consuming_rate_c umulo+rab_integral+RQ_integral+AS_Vol.+emission_O2+emission _CO2; 0.686, 0.856, tmp+AN+interval_Pdt.+OD+RS+Feed_Vol+mu_ cumulo+rho_cumulo+Sugar_consuming_rate_cumulo+AS_Vol.+consu m_O2; 0.691, 0.856, tmp+interval_Pdt.+OD+RS+Feed_Vol+mu_cum ulo+rho_cumulo+Sugar_consuming_rate_cumulo+AS_Vol.+generate _CO2; 0.691, 0.856, tmp+AN_cumulo+interval_Pdt.+RS+Feed_Vol +mu_cumulo+nu_per_rho_cumulo+Sugar_consuming_rate_cumulo+AS _Vol.+generate_CO2; 0.686, 0.856, tmp+AN_cumulo+interval_Pd t.+RS+Feed_Vol+mu_cumulo+nu_per_rho_cumulo+Sugar_consuming_ rate_cumulo+rab_integral+RQ_integral+AS_Vol.; 0.691, 0.856, tmp+PL+AN_cumulo+interval_Pdt.+RS+Feed_Vol+mu_cumulo+Sugar _consuming_rate_cumulo+RQ_integral+emission_O2; 0.696, 0.85 6, tmp+AN_cumulo+interval_Pdt.+OD+RS+Feed_Vol+mu_cumulo+Sug ar_consuming_rate_cumulo+AS_Vol.; 0.681, 0.856, tmp+PL+OD+R S+Feed_Vol+mu_cumulo+rho_cumulo+Sugar_consuming_rate_cumulo +RQ_integral+AS_Vol.+emission_O2+emission_CO2; 0.696, 0.856, tmp+AN+RS+Feed_Vol+mu_cumulo+Sugar_consuming_rate_cumulo+r ab_integral+AS_Vol.+emission_CO2; 0.691, 0.856, tmp+AN_cumu lo+interval_yield+RS+Feed_Vol+mu_cumulo+Sugar_consuming_rat e_cumulo+rab_integral+AS_Vol.+emission_CO2; 0.696, 0.856, t mp+PL+AN_cumulo+RS+Feed_Vol+mu_cumulo+Sugar_consuming_rate_ cumulo+RQ_integral+AS_Vol.; 0.681, 0.856, tmp+OD+RS+Feed_Vo l+mu_cumulo+nu_per_rho_cumulo+Sugar_consuming_rate_cumulo+R Q_integral+AS_Vol.+emission_O2+emission_CO2+generate_CO2; 0. 686, 0.856, tmp+AN_cumulo+interval_Pdt.+RS+Feed_Vol+mu_cumu lo+rho_cumulo+nu_per_rho cumulo+Sugar_consuming_rate_cumulo +rab_integral+AS_Vol.; 0.696, 0.856, tmp+interval_yield+RS+ Feed_Vol+mu_cumulo+nu_per_rho_cumulo+Sugar_consuming_rate_c umulo+rab_integral+AS_Vol.; 0.675, 0.856, tmp+AN_cumulo+int erval_Pdt.+OD+RS+Feed_Vol+mu_cumulo+rho_cumulo+Sugar_consum ing_rate_cumulo+RQ_integral+AS_Vol.+emission_O2+emission_CO 2; 0.681, 0.856, tmp+AN_cumulo+RS+Feed_Vol+mu_cumulo+Sugar_ consuming_rate_cumulo+rab_integral+RQ_integral+AS_Vol.+emis sion_O2+emission_CO2+generate_CO2; 0.675, 0.856, tmp+PL+AN+ interval_Pdt.+RS+Feed_Vol+mu_cumulo+rho_cumulo+Sugar_consum ing_rate_cumulo+rab_integral+AS_Vol.+emission_O2+emission_C 02; 0.675, 0.856, tmp+PL+interval_Pdt.+RS+Feed_Vol+mu_cumul o+nu_cumulo+nu_per_rho_cumulo+Sugar_consuming_rate_cumulo+R Q_integral+AS_Vol.+emission_O2+emission_CO2; 0.691, 0.856, tmp+AN+interval_Pdt.+RS+Feed_Vol+mu_cumulo+Sugar_consuming_ rate_cumulo+rab_integral+AS_Vol.+emission_CO2; 0.675, 0.856, tmp+PL+AN+RS+Feed_Vol+mu_cumulo+rho_cumulo+Sugar_consuming _rate_cumulo+RQ_integral+AS_Vol.+emission_O2+emission_CO2+g enerate_CO2; 0.700, 0.856, tmp+interval_yield+RS+Feed_Vol+m u_cumulo+Sugar_consuming_rate_cumulo+rab_integral+AS_Vol.; 0.681, 0.856, tmp+RS+Feed_Vol+mu_cumulo+nu_cumulo+rho_cumul o+Sugar_consuming_rate_cumulo+RQ_integral+AS_Vol.+emission_ O2+emission_CO2+generate_CO2; 0.675, 0.856, tmp+PL+AN_cumul o+interval_Pdt.+RS+Feed_Vol+mu_cumulo+rho_cumulo+Sugar_cons uming_rate_cumulo+RQ_integral+AS_Vol.+emission_O2+emission-CO2; 0.670, 0.856, tmp+AN_cumulo+interval_yield+interval_Pd t.+OD+RS+Feed_Vol+mu_cumulo+rho_cumulo+Sugar_consuming_rate _cumulo+rab_integral+AS_Vol.+emission_O2+emission_CO2; 0.67 5, 0.856, tmp+AN_cumulo+interval_Pdt.+OD+RS+Feed_Vol+mu_cum ulo+rho_cumulo+Sugar_consuming_rate_cumulo+rab_integral+AS_ Vol.+emission_O2+emission_CO2; 0.686, 0.856, tmp+AN+AN_cumu lo+interval_Pdt.+RS+Feed_Vol+mu_cumulo+nu_per_rho_cumulo+Su gar_consuming_rate_cumulo+rab_integral+emission_O2; 0.686, 0.856, tmp+AN+interval_Pdt.+RS+Feed_Vol+mu_cumulo+nu_per_rh o_cumulo+Sugar_consuming_rate_cumulo+rab_integral+AS_Vol.+e mission CO2; 0.675, 0.856, tmp+interval_Pdt.+OD+RS+Feed_Vol +mu_cumulo+nu_per_rho_cumulo+Sugar_consuming_rate_cumulo+RQ _integral+AS_Vol.+emission_O2+emission_CO2+generate_CO2; 0. 691, 0.856, tmp+AN_cumulo+interval_Pdt.+OD+RS+Feed_Vol+mu_c umulo+Sugar_consuming_rate_cumulo+RQ_integral+emission_O2; 0.675, 0.856, tmp+AN+interval_Pdt.+OD+RS+Feed_Vol+mu_cumulo +rho_cumulo+Sugar_consuming_rate_cumulo+rab_integral+AS_Vol. +emission_O2+emission_CO2; 0.695, 0.856, tmp+AN_cumulo+inte rval_yield+RS+Feed_Vol+mu_cumulo+nu_per_rho_cumulo+Sugar_co nsuming_rate_cumulo+AS_Vol.; 0.690, 0.856, tmp+interval_Pdt. +OD+RS+Feed_Vol+mu_cumulo+rho_cumulo+Sugar_consuming_rate_c umulo+RQ_integral+AS_Vol.; 0.675, 0.856, tmp+PL+AN_cumulo+i nterval_yield+interval_Pdt.+RS+Feed_Vol+mu_cumulo+nu_per_rh o_cumulo+Sugar_consuming_rate_cumulo+rab_integral+AS_Vol.+e mission_O2; 0.690, 0.856, tmp+AN+RS+Feed_Vol+mu_cumulo+Suga r_consuming_rate_cumulo+RQ_integral+AS_Vol.+emission_CO2+ge nerate_CO2; 0.695, 0.856, tmp+PL+interval_Pdt.+RS+Feed_Vol+ mu_cumulo+Sugar_consuming_rate_cumulo+rab_integral+AS_Vol.; 0.700, 0.856, tmp+interval_Pdt.+OD+RS+Feed_Vol+mu_cumulo+S ugar_consuming_rate_cumulo+AS_Vol.; 0.685, 0.856, tmp+inter val_Pdt.+RS+Feed_Vol+mu_cumulo+Sugar_consuming_rate_cumulo+ rab_integral+RQ_integral+AS_Vol.+emission_O2+emission_CO2; 0.680, 0.856, tmp+AN_cumulo+interval_yield+RS+Feed_Vol+mu_c umulo+Sugar_consuming_rate_cumulo+RQ_integral+AS_Vol.+emiss ion_O2+emission_CO2+generate_CO2; 0.690, 0.856, tmp+PL+AN_c umulo+interval_yield+RS+Feed_Vol+mu_cumulo+Sugar_consuming_ rate_cumulo+rab_integral+AS_Vol.; 0.680, 0.856, tmp+AN_cumu lo+interval_yield+interval_Pdt.+RS+Feed_Vol+mu_cumulo+nu_cu mulo+Sugar_consuming_rate_cumulo+rab_integral+AS_Vol.+emiss ion_O2; 0.690, 0.856, tmp+AN+RS+Feed_Vol+mu_cumulo+rho_cumu lo+Sugar_consuming_rate_cumulo+rab_integral+AS_Vol.+emissio n_CO2; 0.675, 0.856, tmp+PL+interval_Pdt.+RS+Feed_Vol+mu_cu mulo+nu_cumulo+rho_cumulo+Sugar_consuming_rate_cumulo+RQ_in tegral+AS_Vol.+emission_O2+emission_CO2; 0.675, 0.856, tmp+ AN+interval_Pdt.+RS+Feed_Vol+mu_cumulo+rho_cumulo+Sugar_con suming_rate_cumulo+RQ_integral+AS_Vol.+emission_O2+emission _CO2+generate_CO2; 0.690, 0.856, tmp+AN_cumulo+interval_yie Id+RS+Feed_Vol+mu_cumulo+Sugar_consuming_rate_cumulo+AS_Vol. +emission_CO2+generate_CO2; 0.690, 0.856, tmp+interval_Pdt. +RS+Feed_Vol+mu_cumulo+Sugar_consuming_rate_cumulo+rab_inte gral+AS_Vol.+emission_CO2+generate_CO2; 0.685, 0.856, tmp+A N_cumulo+interval_yield+interval_Pdt.+RS+Feed_Vol+mu_cumulo +nu_cumulo+Sugar_consuming_rate_cumulo+rab_integral+AS_Vo l.; 0.680, 0.856, tmp+AN_cumulo+interval_Pdt.+RS+Feed_Vol+m u_cumulo+nu_per_rho_cumulo+Sugar_consuming_rate_cumulo+rab_ integral+RQ_integral+AS_Vol.+emission_O2; 0.680, 0.856, tmp +PL+interval_yield+interval_Pdt.+RS+Feed_Vol+mu_cumulo+Suga r_consuming_rate_cumulo+rab_integral+AS_Vol.+emission_O2+em ission_CO2; 0.680, 0.856, tmp+PL+AN+interval_Pdt.+RS+Feed_V ol+mu_cumulo+rho_cumulo+Sugar_consuming_rate_cumulo+RQ_inte gral+emission_O2+emission_CO2; 0.675, 0.856, tmp+PL+AN_cumu lo+interval_yield+interval_Pdt.+OD+RS+Feed_Vol+mu_cumulo+Su gar_consuming_rate_cumulo+rab_integral+AS_Vol.+emission_O2; 0.685, 0.856, tmp+AN+RS+Feed_Vol+mu_cumulo+nu_per_rho_cumu lo+Sugar_consuming_rate_cumulo+RQ_integral+AS_Vol.+emission _O2+emission_CO2; 0.675, 0.856, tmp+PL+AN+interval_Pdt.+RS+ Feed_Vol+mu_cumulo+Sugar_consuming_rate_cumulo+RQ_integral+ AS_Vol.+emission_O2+emission_CO2+generate_CO2; 0.690, 0.856, tmp+interval_yield+interval_Pdt.+RS+Feed_Vol+mu_cumulo+Sug ar_consuming_rate_cumulo+AS_Vol.+emission_CO2+generate_CO2; 0.685, 0.856, tmp+AN_cumulo+interval_Pdt.+RS+Feed_Vol+mu_c umulo+nu_cumulo+nu_per_rho_cumulo+Sugar_consuming_rate_cumu lo+rab_integral+AS_Vol.; 0.690, 0.856, tmp+pH+AN_cumulo+int erval_Pdt.+RS+Feed_Vol+mu_cumulo+Sugar_consuming_rate_cumul o+rab_integral+AS_Vol.; 0.680, 0.856, tmp+PL+interval_yield +interval_Pdt.+RS+Feed_Vol+mu_cumulo+Sugar_consuming_rate_c umulo+RQ_integral+AS_Vol.+emission_O2+emission_CO2; 0.680, 0.856, tmp+AN_cumulo+interval_yield+interval_Pdt.+RS+Feed_V ol+mu_cumulo+Sugar_consuming_rate_cumulo+rab_integral+AS_Vo l.+emission_O2+generate_CO2; 0.695, 0.856, tmp+AN_cumulo+RS +Feed_Vol+mu_cumulo+nu_cumulo+Sugar_consuming_rate_cumulo+r ab_integral+AS_Vol.; 0.675, 0.856, tmp+AN+AN_cumulo+RS+Feed _Vol+mu_cumulo+rho_cumulo+Sugar_consuming_rate_cumulo+RQ_in tegral+AS_Vol.+emission_O2+emission_CO2+generate_CO2; 0.690, 0.856, tmp+AN_cumulo+interval_yield+interval_Pdt.+RS+Feed_ Vol+mu_cumulo+Sugar_consuming_rate_cumulo+RQ_integral+AS_Vo l.; 0.685, 0.856, tmp+PL+AN_cumulo+RS+Feed_Vol+mu_cumulo+Su gar_consuming_rate_cumulo+RQ_integral+AS_Vol.+emission_CO2+ generate_CO2; 0.685, 0.856, tmp+AN_cumulo+interval_Pdt.+OD+ RS+Feed_Vol+mu_cumulo+nu_per_rho_cumulo+Sugar_consuming_rat e_cumulo+RQ_integral+AS_Vol.; 0.690, 0.856, tmp+AN+AN_cumul o+interval_yield+RS+Feed_Vol+mu_cumulo+Sugar_consuming_rate _cumulo+rab_integral+AS_Vol.; 0.695, 0.856, tmp+AN+interval _Pdt.+RS+Feed_Vol+mu_cumulo+nu_per_rho_cumulo+Sugar_consumi ng_rate_cumulo+AS_Vol.; 0.690, 0.856, tmp+AN+AN_cumulo+RS+F eed_Vol+mu_cumulo+Sugar_consuming_rate_cumulo+AS_Vol.+emiss ion_CO2+generate_CO2; 0.685, 0.856, tmp+interval_yield+inte rval_Pdt.+OD+RS+Feed_Vol+mu_cumulo+rho_cumulo+Sugar_consumi ng_rate_cumulo+AS_Vol.+consum_O2; 0.675, 0.856, tmp+interva l_yield+interval_Pdt.+RS+Feed_Vol+mu_cumulo+nu_per_rho_cumu lo+Sugar_consuming_rate_cumulo+RQ_integral+AS_Vol.+emission _O2+emission_CO2+generate_CO2; 0.685, 0.856, tmp+AN_cumulo+ interval_Pdt.+OD+RS+Feed_Vol+mu_cumulo+Sugar_consuming_rate _cumulo+rab_integral+AS_Vol.+emission_O2; 0.690, 0.856, tmp +interval_Pdt.+OD+RS+Feed_Vol+mu_cumulo+nu_per_rho_cumulo+S ugar_consuming_rate_cumulo+AS_Vol.+consum_O2; 0.675, 0.856, tmp+AN_cumulo+RS+Feed_Vol+mu_cumulo+rho_cumulo+nu_per_rho_ cumulo+Sugar_consuming_rate_cumulo+RQ_integral+AS_Vol.+emis sion_O2+emission_CO2+generate_CO2; 0.685, 0.856, tmp+AN+int erval_Pdt.+RS+Feed_Vol+mu_cumulo+nu_per_rho_cumulo+Sugar_co nsuming_rate_cumulo+AS_Vol.+emission_CO2+generate_CO2; 0.67 5, 0.856, tmp+PL+AN+interval_yield+interval_Pdt.+RS+Feed_Vo l+mu_cumulo+Sugar_consuming_rate_cumulo+rab_integral+AS_Vol. +emission_O2+emission_CO2; 0.690, 0.856, tmp+AN_cumulo+inte rval_Pdt.+OD+RS+Feed_Vol+mu_cumulo+nu_per_rho_cumulo+Sugar_ consuming_rate_cumulo+AS_Vol.; 0.674, 0.856, tmp+AN+AN_cumu lo+interval_yield+interval_Pdt.+OD+RS+Feed_Vol+mu_cumulo+Su gar_consuming_rate_cumulo+rab_integral+AS_Vol.+emission_O2; 0.674, 0.856, tmp+PL+interval_yield+RS+Feed_Vol+mu_cumulo+ rho_cumulo+Sugar_consuming_rate_cumulo+RQ_integral+AS_Vol.+ emission_O2+emission_CO2+generate_CO2; 0.674, 0.856, tmp+PL +AN_cumulo+interval_Pdt.+RS+Feed_Vol+mu_cumulo+nu_per_rho_c umulo+Sugar_consuming_rate_cumulo+rab_integral+AS_Vol.+emis sion_O2+emission_CO2; 0.690, 0.856, tmp+AN_cumulo+RS+Feed_V ol+mu_cumulo+rho_cumulo+Sugar_consuming_rate_cumulo+AS_Vol. +emission_CO2+generate_CO2; 0.690, 0.856, tmp+RS+Feed_Vol+m u_cumulo+rho_cumulo+Sugar_consuming_rate_cumulo+rab_integra l+AS_Vol.+emission_CO2+generate_CO2; 0.685, 0.856, tmp+AN_c umulo+interval_Pdt.+RS+Feed_Vol+mu_cumulo+nu_cumulo+rho_cum ulo+Sugar_consuming_rate_cumulo+rab_integral+AS_Vol.; 0.674, 0.856, tmp+AN_cumulo+interval_Pdt.+RS+Feed_Vol+mu_cumulo+n u_per_rho_cumulo+Sugar_consuming_rate_cumulo+rab_integral+A S_Vol.+emission_O2+emission_CO2+generate_CO2; 0.695, 0.856, tmp+AN+AN_cumulo+RS+Feed_Vol+mu_cumulo+Sugar_consuming_rat e_cumulo+RQ_integral+AS_Vol.; 0.695, 0.856, tmp+interval_Pd t.+OD+RS+Feed_Vol+mu_cumulo+nu_per_rho_cumulo+Sugar_consumi ng_rate_cumulo+AS_Vol.; 0.690, 0.856, tmp+PL+AN_cumulo+RS+F eed_Vol+mu_cumulo+Sugar_consuming_rate_cumulo+rab_integral+ AS_Vol.+emission_CO2; 0.685, 0.856, tmp+PL+AN+AN_cumulo+int erval_Pdt.+RS+Feed_Vol+mu_cumulo+Sugar_consuming_rate_cumul o+rab_integral+AS_Vol.; 0.695, 0.856, tmp+AN_cumulo+interva l_yield+RS+Feed_Vol+mu_cumulo+Sugar_consuming_rate_cumulo+A S_Vol.+generate_CO2; 0.685, 0.856, tmp+PL+AN+RS+Feed_Vol+mu _cumulo+Sugar_consuming_rate_cumulo+RQ_integral+AS_Vol.+emi ssion_O2+emission_CO2; 0.695, 0.856, tmp+PL+AN_cumulo+inter val_Pdt.+RS+Feed_Vol+mu_cumulo+Sugar_consuming_rate_cumulo+ AS_Vol.; 0.690, 0.856, tmp+AN+AN_cumulo+RS+Feed_Vol+mu_cumu lo+Sugar_consuming_rate_cumulo+rab_integral+AS_Vol.+emissio n_CO2; 0.690, 0.856, tmp+AN_cumulo+interval_yield+RS+Feed_V ol+mu_cumulo+Sugar_consuming_rate_cumulo+rab_integral+AS_Vo l.+generate_CO2; 0.685, 0.856, tmp+AN_cumulo+RS+Feed_Vol+mu _cumulo+nu_per_rho_cumulo+Sugar_consuming_rate_cumulo+rab_i ntegral+AS_Vol.+emission_CO2+generate_CO2; 0.680, 0.856, tm p+AN+interval_Pdt.+RS+Feed_Vol+mu_cumulo+nu_per_rho_cumulo+ Sugar_consuming_rate_cumulo+RQ_integral+AS_Vol.+emission_O2 +emission_CO2; 0.685, 0.856, tmp+OD+RS+Feed_Vol+mu_cumulo+r ho_cumulo+Sugar_consuming_rate_cumulo+RQ_integral+AS_Vol.+e mission_CO2+consum_O2; 0.674, 0.856, tmp+AN_cumulo+interval _Pdt.+OD+RS+Feed_Vol+mu_cumulo+Sugar_consuming_rate_cumulo+ RQ_integral+AS_Vol.+emission_O2+emission_CO2+generate_CO2; 0.699, 0.856, tmp+PL+interval_Pdt.+RS+Feed_Vol+mu_cumulo+Su gar_consuming_rate_cumulo+AS_Vol.; 0.690, 0.856, tmp+AN_cum ulo+interval_yield+RS+Feed_Vol+mu_cumulo+Sugar_consuming_ra te_cumulo+rab_integral+RQ_integral+AS_Vol.; 0.685, 0.856, t mp+AN_cumulo+interval_Pdt.+RS+Feed_Vol+mu_cumulo+rho_cumulo +Sugar_consuming_rate_cumulo+rab_integral+AS_Vol.+emission_ CO2; 0.690, 0.856, tmp+AN+AN_cumulo+RS+Feed_Vol+mu_cumulo+S ugar_consuming_rate_cumulo+RQ_integral+AS_Vol.+emission_CO 2; 0.680, 0.856, tmp+PL+AN_cumulo+interval_Pdt.+RS+Feed_Vol +mu_cumulo+rho_cumulo+Sugar_consuming_rate_cumulo+rab_integ ral+AS_Vol.+emission_O2; 0.674, 0.856, tmp+AN+AN_cumulo+int erval_Pdt.+OD+RS+Feed_Vol+mu_cumulo+rho_cumulo+Sugar_consum ing_rate_cumulo+RQ_integral+AS_Vol.+emission_O2; 0.674, 0.8 56, tmp+AN+interval_yield+interval_Pdt.+RS+Feed_Vol+mu_cumu lo+rho_cumulo+Sugar_consuming_rate_cumulo+rab_integral+AS_V ol.+emission_O2+emission_CO2; 0.690, 0.856, tmp+PL+AN_cumul o+RS+Feed_Vol+mu_cumulo+Sugar_consuming_rate_cumulo+RQ_inte gral+AS_Vol.+emission_CO2; 0.685, 0.856, tmp+AN_cumulo+inte rval_yield+interval_Pdt.+OD+RS+Feed_Vol+mu_cumulo+Sugar_con suming_rate_cumulo+rab_integral+AS_Vol.; 0.669, 0.856, tmp+ PL+interval_yield+interval_Pdt.+OD+RS+Feed_Vol+mu_cumulo+rh o_cumulo+Sugar_consuming_rate_cumulo+RQ_integral+AS_Vol.+em ission_O2+emission_CO2; 0.680, 0.856, tmp+interval_Pdt.+RS+ Feed_Vol+mu_cumulo+rho_cumulo+nu_per_rho_cumulo+Sugar_consu ming_rate_cumulo+rab_integral+AS_Vol.+emission_O2+emission-CO2; 0.685, 0.856, tmp+AN_cumulo+interval_yield+interval_Pd t.+OD+RS+Feed_Vol+mu_cumulo+Sugar_consuming_rate_cumulo+AS_ Vol.+generate_CO2; 0.690, 0.856, tmp+AN_cumulo+interval_yie ld+RS+Feed_Vol+mu_cumulo+rho_cumulo+Sugar_consuming_rate_cu mulo+rab_integral+AS_Vol.; 0.685, 0.856, tmp+AN_cumulo+inte rval_yield+interval_Pdt.+RS+Feed_Vol+mu_cumulo+nu_per_rho_c umulo+Sugar_consuming_rate_cumulo+rab_integral+emission_O2; 0.685, 0.856, tmp+AN_cumulo+interval_yield+interval_Pdt.+R S+Feed_Vol+mu_cumulo+Sugar_consuming_rate_cumulo+rab_integr al+RQ_integral+AS_Vol.; 0.674, 0.856, tmp+PL+interval_yield +interval_Pdt.+RS+Feed_Vol+mu_cumulo+rho_cumulo+Sugar_consu ming_rate_cumulo+RQ_integral+AS_Vol.+emission_O2+emission_C 02; 0.694, 0.856, tmp+AN_cumulo+RS+Feed_Vol+mu_cumulo+rho_c umulo+Sugar_consuming_rate_cumulo+RQ_integral+AS_Vol.; 0.69 4, 0.856, tmp+interval_yield+RS+Feed_Vol+mu_cumulo+rho_cumu lo+Sugar_consuming_rate_cumulo+rab_integral+AS_Vol.; 0.685, 0.856, tmp+RS+Feed_Vol+mu_cumulo+Sugar_consuming_rate_cumu lo+rab_integral+RQ_integral+AS_Vol.+emission_O2+eniission_CO 2+generate_CO2; 0.690, 0.856, tmp+AN_cumulo+interval_Pdt.+R S+Feed_Vol+mu_cumulo+rho_cumulo+Sugar_consuming_rate_cumulo +RQ_integral+AS_Vol.; 0.669, 0.856, tmp+AN_cumulo+interval_ yield+interval_Pdt.+OD+RS+Feed_Vol+mu_cumulo+Sugar_consumin g_rate_cumulo+RQ_integral+AS_Vol.+emission_O2+emission_CO2+ generate_CO2; 0.685, 0.856, tmp+interval_Pdt.+OD+RS+Feed_Vo l+mu_cumulo+rho_cumulo+Sugar_consuming_rate_cumulo+RQ_integ ral+AS_Vol.+consum_O2; 0.679, 0.856, tmp+interval_Pdt.+RS+F eed_Vol+mu_cumulo+rho_cumulo+Sugar_consuming_rate_cumulo+ra b_integral+RQ_integral+AS_Vol.+emission_O2+emission_CO2; 0. 704, 0.856, tmp+interval_yield+RS+Feed_Vol+mu_cumulo+Sugar_ consuming_rate_cumulo+AS_Vol.; 0.685, 0.856, tmp+AN_cumulo+ interval_Pdt.+OD+RS+Feed_Vol+mu_cumulo+rho_cumulo+Sugar_con suming_rate_cumulo+RQ_integral+emission_O2; 0.668, 0.856, t mp+pH+PL+interval_Pdt.+OD+RS+Feed_Vol+mu_cumulo+rho_cumulo+ Sugar_consuming_rate_cumulo+RQ_integral+AS_Vol.+emission_O2 +emission_CO2; 0.704, 0.856, tmp+AN_cumulo+RS+Feed_Vol+mu_c umulo+Sugar_consuming_rate_cumulo+AS_Vol.; 0.685, 0.856, tm p+OD+RS+Feed_Vol+mu_cumulo+rho_cumulo+Sugar_consuming_rate_ cumulo+RQ_integral+AS_Vol.+emission_O2+emission_CO2; 0.694, 0.856, tmp+PL+RS+Feed_Vol+mu_cumulo+Sugar_consuming_rate_c umulo+AS_Vol.+emission_CO2+generate_CO2; 0.690, 0.856, tmp+ AN+AN_cumulo+interval_Pdt.+RS+Feed_Vol+mu_cumulo+Sugar_cons uming_rate_cumulo+RQ_integral+AS_Vol.; 0.668, 0.856, tmp+PL +AN_cumulo+interval_Pdt.+OD+RS+Feed_Vol+mu_cumulo+nu_per_rh o_cumulo+Sugar_consuming_rate_cumulo+RQ_integral+AS_Vol.+em ission_O2+emission_CO2; 0.685, 0.856, tmp+AN+RS+Feed_Vol+mu _cumulo+rho_cumulo+Sugar_consuming_rate_cumulo+RQ_integral+ AS_Vol.+emission_O2+emission_CO2; 0.699, 0.856, tmp+AN+inte rval_Pdt.+RS+Feed_Vol+mu_cumulo+Sugar_consuming_rate_cumulo +rab_integral; 0.674, 0.856, tmp+pH+PL+interval_Pdt.+RS+Fee d_Vol+mu_cumulo+nu_per_rho_cumulo+Sugar_consuming_rate_cumu lo+RQ_integral+AS_Vol.+emission_O2+emission_CO2; 0.668, 0.8 56, tmp+PL+AN+interval_Pdt.+RS+Feed_Vol+mu_cumulo+rho_cumul o+nu_per_rho_cumulo+Sugar_consuming_rate_cumulo+RQ_integral +AS_Vol.+emission_O2+emission_CO2; 0.674, 0.856, tmp+AN+int erval_yield+interval_Pdt.+RS+Feed_Vol+mu_cumulo+nu_per_rho_ cumulo+Sugar_consuming_rate_cumulo+rab_integral+AS_Vol.+emi ssion_O2+emission_CO2; 0.679, 0.856, tmp+AN_cumulo+interval _Pdt.+OD+RS+Feed_Vol+mu_cumulo+nu_per_rho_cumulo+Sugar_cons uming_rate_cumulo+RQ_integral+AS_Vol.+emission_O2; 0.679, 0. 856, tmp+AN_cumulo+interval_Pdt.+RS+Feed_Vol+mu_cumulo+nu_p er_rho_cumulo+Sugar_consuming_rate_cumulo+rab_integral+AS_V ol.+emission_O2+generate_CO2; 0.679, 0.856, tmp+PL+AN+RS+Fe ed_Vol+mu_cumulo+Sugar_consuming_rate_cumulo+RQ_integral+AS _Vol.+emission_O2+emission_CO2+generate_CO2; 0.684, 0.856, tmp+AN_cumulo+interval_yield+interval_Pdt.+RS+Feed_Vol+mu_c umulo+Sugar_consuming_rate_cumulo+rab_integral+RQ_integral+ emission_O2; 0.679, 0.856, tmp+PL+RS+Feed_Vol+mu_cumulo+rho _cumulo+nu_per_rho_cumulo+Sugar_consuming_rate_cumulo+RQ_in tegral+AS_Vol.+emission_O2+emission_CO2; 0.684, 0.856, tmp+ RS+Feed_Vol+mu_cumulo+nu_per_rho_cumulo+Sugar_consuming_rat e_cumulo+rab_integral+RQ_integral+AS_Vol.+emission_O2+emiss ion_CO2; 0.679, 0.856, tmp+pH+AN_cumulo+RS+Feed_Vol+mu_cumu lo+Sugar_consuming_rate_cumulo+RQ_integral+AS_Vol.+emission _O2+emission_CO2+generate_CO2; 0.674, 0.856, tmp+AN_cumulo+ RS+Feed_Vol+mu_cumulo+nu_cumulo+nu_per_rho_cumulo+Sugar_con suming_rate_cumulo+RQ_integral+AS_Vol.+emission_O2+emission _CO2+generate_CO2; 0.689, 0.856, tmp+AN+RS+Feed_Vol+mu_cumu lo+nu_per_rho_cumulo+Sugar_consuming_rate_cumulo+AS_Vol.+em ission_CO2+generate_CO2; 0.674, 0.856, tmp+AN+interval_Pdt. +RS+Feed_Vol+mu_cumulo+nu_per_rho_cumulo+Sugar_consuming_ra te_cumulo+rab_integral+RQ_integral+AS_Vol.+emission_O2+emis sion_CO2; 0.689, 0.856, tmp+RS+Feed_Vol+mu_cumulo+nu_per_rh o_cumulo+Sugar_consuming_rate_cumulo+rab_integral+AS_Vol.+e mission_CO2+generate_CO2; 0.674, 0.856, tmp+AN_cumulo+inter val_yield+interval_Pdt.+RS+Feed_Vol+mu_cumulo+nu_cumulo+Sug ar_consuming_rate_cumulo+rab_integral+AS_Vol.+emission_O2+e mission_CO2; 0.684, 0.856, tmp+AN+AN_cumulo+interval_Pdt.+R S+Feed_Vol+mu_cumulo+Sugar_consuming_rate_cumulo+rab_integr al+RQ_integral+emission_O2; 0.668, 0.856, tmp+PL+AN+interva l_yield+interval_Pdt.+RS+Feed_Vol+mu_cumulo+nu_per_rho_cumu lo+Sugar_consuming_rate_cumulo+RQ_integral+AS_Vol.+emission _O2+emission_CO2; 0.694, 0.856, tmp+RS+Feed_Vol+mu_cumulo+n u_cumulo+Sugar_consuming_rate_cumulo+AS_Vol.+emission_CO2+g enerate_CO2; 0.668, 0.856, tmp+PL+interval_Pdt.+RS+Feed_Vol +mu_cumulo+rho_cumulo+nu_per_rho_cumulo+Sugar_consuming_rat e_cumulo+RQ_integral+AS_Vol.+emission_O2+emission_CO2+gener ate_CO2; 0.689, 0.855, tmp+interval_yield+interval_Pdt.+OD+ RS+Feed_Vol+mu_cumulo+Sugar_consuming_rate_cumulo+AS_Vol.+c onsum_O2; 0.684, 0.855, tmp+interval_Pdt.+OD+RS+Feed_Vol+mu _cumulo+rho_cumulo+Sugar_consuming_rate_cumulo+rab_integral +AS_Vol.+emission_O2; 0.684, 0.855, tmp+AN+interval_Pdt.+RS +Feed_Vol+mu_cumulo+rho_cumulo+Sugar_consuming_rate_cumulo+ rab_integral+AS_Vol.+emission_CO2; 0.684, 0.855, tmp+PL+AN_ cumulo+interval_Pdt.+RS+Feed_Vol+mu_cumulo+Sugar_consuming_ rate_cumulo+rab_integral+AS_Vol.+generate_CO2; 0.684, 0.855, tmp+AN_cumulo+interval_Pdt.+RS+Feed_Vol+mu_cumulo+nu_per_r ho_cumulo+Sugar_consuming_rate_cumulo+AS_Vol.+emission_CO2+ generate_CO2; 0.668, 0.855, tmp+PL+AN_cumulo+interval_Pdt.+ RS+Feed_Vol+mu_cumulo+rho_cumulo+Sugar_consuming_rate_cumul o+RQ_integral+AS_Vol.+emission_O2+emission_CO2+generate_CO 2; 0.679, 0.855, tmp+PL+AN_cumulo+interval_Pdt.+RS+Feed_Vol +mu_cumulo+Sugar_consuming_rate_cumulo+rab_integral+AS_Vol. +emission_O2+emission_CO2; 0.694, 0.855, tmp+RS+Feed_Vol+mu _cumulo+nu_per_rho_cumulo+Sugar_consuming_rate_cumulo+RQ_in tegral+AS_Vol.+emission_CO2; 0.674, 0.855, tmp+AN_cumulo+in terval_Pdt.+RS+Feed_Vol+mu_cumulo+rho_cumulo+Sugar_consumin g_rate_cumulo+RQ_integral+AS_Vol.+emission_O2+emission_CO2+ generate_CO2; 0.689, 0.855, tmp+PL+AN_cumulo+interval_Pdt.+ RS+Feed_Vol+mu_cumulo+Sugar_consuming_rate_cumulo+AS_Vol.+g enerate_CO2; 0.689, 0.855, tmp+AN_cumulo+interval_Pdt.+RS+F eed_Vol+mu_cumulo+Sugar_consuming_rate_cumulo+rab_integral+ AS_Vol.+consum_O2; 0.674, 0.855, tmp+PL+AN_cumulo+interval_ yield+interval_Pdt.+RS+Feed_Vol+mu_cumulo+Sugar_consuming_r ate_cumulo+RQ_integral+AS_Vol.+emission_O2+emission_CO2; 0. 689, 0.855, tmp+AN_cumulo+interval_Pdt.+RS+Feed_Vol+mu_cumu lo+nu_cumulo+Sugar_consuming_rate_cumulo+RQ_integral+AS_Vo l.; 0.679, 0.855, tmp+PL+AN+AN_cumulo+interval_Pdt.+RS+Feed _Vol+mu_cumulo+Sugar_consuming_rate_cumulo+rab_integral+AS_ Vol.+emission_O2; 0.674, 0.855, tmp+interval_Pdt.+RS+Feed_V ol+mu_cumulo+rho_cumulo+nu_per_rho_cumulo+Sugar_consuming_r ate_cumulo+RQ_integral+AS_Vol.+emission_O2+emission_CO2+gen erate_CO2; 0.679, 0.855, tmp+PL+AN_cumulo+interval_Pdt.+RS+ Feed_Vol+mu_cumulo+nu_per_rho_cumulo+Sugar_consuming_rate_c umulo+RQ_integral+AS_Vol.+emission_O2; 0.668, 0.855, tmp+PL +interval_yield+interval_Pdt.+RS+Feed_Vol+mu_cumulo+nu_per_ rho_cumulo+Sugar_consuming_rate_cumulo+RQ_integral+AS_Vol.+ emission_O2+emission_CO2+generate_CO2; 0.679, 0.855, tmp+AN _cumulo+RS+Feed_Vol+mu_cumulo+nu_per_rho_cumulo+Sugar_consu ming_rate_cumulo+rab_integral+RQ_integral+AS_Vol.+emission_ O2+emission_CO2; 0.694, 0.855, tmp+RS+Feed_Vol+mu_cumulo+Su gar_consuming_rate_cumulo+AS_Vol.+emission_CO2+consum-O2+ge nerate_CO2; 0.674, 0.855, tmp+PL+AN+interval_yield+RS+Feed_ Vol+mu_cumulo+rho_cumulo+Sugar_consuming_rate_cumulo+RQ_int egral+AS_Vol.+emission_O2+emission_CO2; 0.679, 0.855, tmp+P L+AN+RS+Feed_Vol+mu_cumulo+nu_per_rho_cumulo+Sugar_consumin g_rate_cumulo+RQ_integral+AS_Vol.+emission_O2+emission_CO2; 0.679, 0.855, tmp+interval_Pdt.+RS+Feed_Vol+mu_cumulo+nu_c umulo+Sugar_consuming_rate_cumulo+RQ_integral+AS_Vol.+emiss ion_O2+emission_CO2+generate_CO2; 0.684, 0.855, tmp+AN_cumu lo+interval_Pdt.+RS+Feed_Vol+mu_cumulo+rho_cumulo+Sugar_con suming_rate_cumulo+rab_integral+AS_Vol.+generate_CO2; 0.673, 0.855, tmp+PL+AN+AN_cumulo+RS+Feed_Vol+mu_cumulo+Sugar_con suming_rate_cumulo+RQ_integral+AS_Vol.+emission_O2+emission _CO2+generate_CO2; 0.684, 0.855, tmp+AN_cumulo+interval_Pdt. +RS+Feed_Vol+mu_cumulo+Sugar_consuming_rate_cumulo+rab_inte gral+AS_Vol.+emission_CO2+generate_CO2; 0.689, 0.855, tmp+i nterval_Pdt.+RS+Feed_Vol+mu_cumulo+Sugar_consuming_rate_cum ulo+RQ_integral+AS_Vol.+emission_CO2+generate_CO2; 0.694, 0. 855, tmp+pH+AN_cumulo+RS+Feed_Vol+mu_cumulo+Sugar_consuming _rate_cumulo+RQ_integral+AS_Vol.; 0.689, 0.855, tmp+RS+Feed _Vol+mu_cumulo+Sugar_consuming_rate_cumulo+RQ_integral+AS_V ol.+emission_CO2+consum_O2+generate_CO2; 0.703, 0.855, tmp+ interval_yield+RS+Feed_Vol+mu_cumulo+nu_per_rho_cumulo+AS_V ol.; 0.673, 0.855, tmp+PL+AN+interval_Pdt.+RS+Feed_Vol+mu_c umulo+nu_per_rho_cumulo+Sugar_consuming_rate_cumulo+rab_int egral+AS_Vol.+emission_O2+emission_CO2; 0.684, 0.855, tmp+A N_cumulo+interval_Pdt.+RS+Feed_Vol+mu_cumulo+nu_per_rho_cum ulo+Sugar_consuming_rate_cumulo+rab_integral+RQ_integral+em ission_O2; 0.694, 0.855, tmp+AN_cumulo+interval_yield+inter val_Pdt.+RS+Feed_Vol+mu_cumulo+Sugar_consuming_rate_cumulo+ AS_Vol.; 0.689, 0.855, tmp+interval_yield+interval_Pdt.+RS+ Feed_Vol+mu_cumulo+nu_per_rho_cumulo+Sugar_consuming_rate_c umulo+rab_integral+AS_Vol.; 0.673, 0.855, tmp+PL+interval_P dt.+OD+RS+Feed_Vol+mu_cumulo+rho_cumulo+Sugar_consuming_rat e_cumulo+rab_integral+AS_Vol.+emission_O2+emission_CO2; 0.6 79, 0.855, tmp+PL+RS+Feed_Vol+mu_cumulo+nu_cumulo+Sugar_con suming_rate_cumulo+RQ_integral+AS_Vol.+emission_O2+emission _CO2+generate_CO2; 0.673, 0.855, tmp+AN_cumulo+RS+Feed_Vol+ mu_cumulo+nu_cumulo+rho_cumulo+Sugar_consuming_rate_cumulo+ RQ_integral+AS_Vol.+emission_O2+emission_O2+generate_CO2; 0.689, 0.855, tmp+AN_cumulo+interval_Pdt.+RS+Feed_Vol+mu_cu mulo+rho_cumulo+Sugar_consuming_rate_cumulo+rab_integral+em ission_O2; 0.684, 0.855, tmp+AN_cumulo+interval_Pdt.+OD+RS+ Feed_Vol+mu_cumulo+nu_per_rho_cumulo+Sugar_consuming_rate_c umulo+AS_Vol.+generate_CO2; 0.684, 0.855, tmp+AN_cumulo+int erval_Pdt.+RS+Feed_Vol+mu_cumulo+Sugar_consuming_rate_cumul o+RQ_integral+AS_Vol.+emission_CO2+generate_CO2; 0.679, 0.8 55, tmp+AN+AN_cumulo+interval_Pdt.+OD+RS+Feed_Vol+mu_cumulo +rho_cumulo+Sugar_consuming_rate_cumulo+RQ_integral+AS_Vo l.; 0.679, 0.855, tmp+AN+AN_cumulo+RS+Feed_Vol+mu_cumulo+nu _per_rho_cumulo+Sugar_consuming_rate_cumulo+RQ_integral+AS_ Vol.+emission_CO2+generate_CO2; 0.684, 0.855, tmp+AN_cumulo +interval_Pdt.+RS+Feed_Vol+mu_cumulo+Sugar_consuming_rate_c umulo+RQ_integral+AS_Vol.+emission_O2+emission_CO2; 0.684, 0.855, tmp+PL+AN_cumulo+interval_yield+interval_Pdt.+RS+Fee d_Vol+mu_cumulo+Sugar_consuming_rate_cumulo+rab_integral+em ission_O2; 0.679, 0.855, tmp+interval_Pdt.+RS+Feed_Vol+mu_c umulo+nu_cumulo+nu_per_rho_cumulo+Sugar_consuming_rate_cumu lo+rab_integral+AS_Vol.+emission_O2+emission_CO2; 0.679, 0. 855, tmp+RS+Feed_Vol+mu_cumulo+rho_cumulo+Sugar_consuming_r ate_cumulo+rab_integral+RQ_integral+AS_Vol.+emission_O2+emi ssion_CO2+generate_CO2; 0.673, 0.855, tmp+AN_cumulo+interva l_yield+interval_Pdt.+OD+RS+Feed_Vol+mu_cumulo+nu_per_rho_c umulo+Sugar_consuming_rate_cumulo+rab_integral+AS_Vol.+emis sion_O2; 0.679, 0.855, tmp+interval_yield+interval_Pdt.+RS+ Feed_Vol+mu_cumulo+Sugar_consuming_rate_cumulo+rab_integral +RQ_integral+AS_Vol.+emission_O2+emission_CO2; 0.673, 0.855, tmp+PL+AN+interval_yield+interval_Pdt.+RS+Feed_Vol+mu_cumu lo+Sugar_consuming_rate_cumulo+RQ_integral+AS_Vol.+emission _O2+emission_CO2; 0.684, 0.855, tmp+interval_Pdt.+OD+RS+Fee d_Vol+mu_cumulo+rho_cumulo+Sugar_consuming_rate_cumulo+AS_V ol.+consum_O2+generate_CO2; 0.689, 0.855, tmp+PL+interval_P dt.+RS+Feed_Vol+mu_cumulo+nu_per_rho_cumulo+Sugar_consuming _rate_cumulo+rab_integral+AS_Vol.; 0.694, 0.855, tmp+interv al_Pdt.+RS+Feed_Vol+mu_cumulo+Sugar_consuming_rate_cumulo+r ab_integral+AS_Vol.+emission_O2; 0.678, 0.855, tmp+AN+AN_cu mulo+interval_Pdt.+OD+RS+Feed_Vol+mu_cumulo+Sugar_consuming _rate_cumulo+RQ_integral+AS_Vol.+emission_O2; 0.684, 0.855, tmp+PL+AN_cumulo+interval_Pdt.+RS+Feed_Vol+mu_cumulo+Sugar _consuming_rate_cumulo+rab_integral+RQ_integral+emission_O 2; 0.693, 0.855, tmp+PL+interval_Pdt.+RS+Feed_Vol+mu_cumulo +nu_per_rho_cumulo+Sugar_consuming_rate_cumulo+AS_Vol.; 0.6 73, 0.855, tmp+AN+interval_yield+interval_Pdt.+RS+Feed_Vol+ mu_cumulo+Sugar_consuming_rate_cumulo+RQ_integral+AS_Vol.+e mission_O2+emission_CO2+generate_CO2; 0.693, 0.855, tmp+int erval_Pdt.+RS+Feed_Vol+mu_cumulo+nu_cumulo+Sugar_consuming_ rate_cumulo+rab_integral+AS_Vol.; 0.693, 0.855, tmp+pH+RS+F eed_Vol+mu_cumulo+Sugar_consuming_rate_cumulo+AS_Vol.+emiss ion_CO2+generate_CO2; 0.689, 0.855, tmp+PL+interval_Pdt.+OD +RS+Feed_Vol+mu_cumulo+rho_cumulo+Sugar_consuming_rate_cumu lo+AS_Vol.; 0.678, 0.855, tmp+AN_cumulo+interval_Pdt.+RS+Fe ed_Vol+mu_cumulo+nu_per_rho_cumulo+Sugar_consuming_rate_cum ulo+RQ_integral+AS_Vol.+emission_O2+emission_CO2; 0.673, 0. 855, tmp+AN_cumulo+OD+RS+Feed_Vol+mu_cumulo+nu_per_rho_cumu lo+Sugar_consuming_rate_cumulo+RQ_integral+AS_Vol.+emission _O2+emission_CO2+generate_CO2; 0.684, 0.855, tmp+AN+AN_cumu lo+interval_Pdt.+RS+Feed_Vol+mu_cumulo+rho_cumulo+Sugar_con suming_rate_cumulo+rab_integral+AS_Vol.; 0.689, 0.855, tmp+ interval_yield+interval_Pdt.+OD+RS+Feed_Vol+mu_cumulo+rho_c umulo+Sugar_consuming_rate_cumulo+AS_Vol.; 0.698, 0.855, tm p+interval_Pdt.+RS+Feed_Vol+mu_cumulo+Sugar_consuming_rate_ cumulo+AS_Vol.+generate_CO2; 0.673, 0.855, tmp+PL+RS+Feed_V ol+mu_cumulo+rho_cumulo+nu_per_rho_cumulo+Sugar_consuming_r ate_cumulo+RQ_integral+AS_Vol.+emission_O2+emission_CO2+gen erate_CO2; 0.673, 0.855, tmp+AN_cumulo+OD+RS+Feed_Vol+mu_cu mulo+rho_cumulo+Sugar_consuming_rate_cumulo+RQ_integral+AS_ Vol.+emission_O2+emission_CO2+generate_CO2; 0.678, 0.855, t mp+AN+OD+RS+Feed_Vol+mu_cumulo+Sugar_consuming_rate_cumulo+ RQ_integral+AS_Vol.+emission_O2+emission_O2+generate_CO2; 0.688, 0.855, tmp+AN+AN_cumulo+RS+Feed_Vol+mu_cumulo+nu_per _rho_cumulo+Sugar_consuming_rate_cumulo+rab_integral+AS_Vo l.; 0.683, 0.855, tmp+interval_Pdt.+RS+Feed_Vol+mu_cumulo+r ho_cumulo+Sugar_consuming_rate_cumulo+RQ_integral+AS_Vol.+e mission_O2+emission_CO2; 0.683, 0.855, tmp+interval_Pdt.+RS +Feed_Vol+mu_cumulo+nu_per_rho_cumulo+Sugar_consuming_rate_ cumulo+AS_Vol.+emission_O2+emission_CO2+generate_CO2; 0.678, 0.855, tmp+AN_cumulo+OD+RS+Feed_Vol+mu_cumulo+rho_cumulo+S ugar_consuming_rate_cumulo+RQ_integral+AS_Vol.+emission_O2+ emission_CO2; 0.667, 0.855, tmp+PL+AN+interval_Pdt.+RS+Feed _Vol+mu_cumulo+nu_cumulo+nu_per_rho_cumulo+Sugar_consuming_ rate_cumulo+RQ_integral+AS_Vol.+emission_O2+emission_CO2; 0. 667, 0.855, tmp+AN_cumulo+interval_Pdt.+OD+RS+Feed_Vol+mu_c umulo+nu_per_rho_cumulo+Sugar_consuming_rate_cumulo+RQ_inte gral+AS_Vol.+emission_O2+emission_CO2+generate_CO2; 0.678, 0.855, tmp+AN+RS+Feed_Vol+mu_cumulo+nu_cumulo+Sugar_consumi ng_rate_cumulo+RQ_integral+AS_Vol.+emission_O2+emission_CO2 +generate_CO2; 0.683, 0.855, tmp+AN_cumulo+interval_yield+i nterval_Pdt.+OD+RS+Feed_Vol+mu_cumulo+Sugar_consuming_rate_ cumulo+RQ_integral+AS_Vol.; 0.667, 0.855, tmp+PL+interval_P dt.+RS+Feed_Vol+mu_cumulo+nu_cumulo+nu_per_rho_cumulo+Sugar _consuming_rate_cumulo+RQ_integral+AS_Vol.+emission_O2+emis sion_CO2+generate_CO2; 0.683, 0.855, tmp+AN_cumulo+OD+RS+Fe ed_Vol+mu_cumulo+Sugar_consuming_rate_cumulo+RQ_integral+AS _Vol. +emission_O2+emission_CO2; 0.683, 0.855, tmp+interval_ yield+interval_Pdt.+OD+RS+Feed_Vol+mu_cumulo+rho_cumulo+Sug ar_consuming_rate_cumulo+rab_integral+AS_Vol.; 0.673, 0.855, tmp+interval_Pdt.+RS+Feed_Vol+mu_cumulo+nu_cumulo+nu_per_r ho_cumulo+Sugar_consuming_rate_cumulo+RQ_integral+AS_Vol.+e mission_O2+emission_CO2+generate_CO2; 0.698, 0.855, tmp+AN_ cumulo+interval_Pdt.+RS+Feed_Vol+mu_cumulo+Sugar_consuming_ rate_cumulo+rab_integral; 0.678, 0.855, tmp+AN_cumulo+inter val_Pdt.+OD+RS+Feed_Vol+mu_cumulo+nu_per_rho_cumulo+Sugar_c onsuming_rate_cumulo+rab_integral+AS_Vol.+emission_O2; 0.68 8, 0.855, tmp+pH+AN_cumulo+RS+Feed_Vol+mu_cumulo+rho_cumulo +Sugar_consuming_rate_cumulo+rab_integral+AS_Vol.; 0.678, 0. 855, tmp+PL+interval_Pdt.+RS+Feed_Vol+mu_cumulo+Sugar_consu ming_rate_cumulo+rab_integral+RQ_integral+AS_Vol.+emission_ O2+emission_CO2; 0.683, 0.855, tmp+AN_cumulo+interval_yield +RS+Feed_Vol+mu_cumulo+nu_per_rho_cumulo+Sugar_consuming_ra te_cumulo+rab_integral+AS_Vol.+emission_CO2; 0.683, 0.855, tmp+AN+AN_cumulo+interval_Pdt.+OD+RS+Feed_Vol+mu_cumulo+Sug ar_consuming_rate_cumulo+AS_Vol.+generate_CO2; 0.683, 0.855, tmp+AN_cumulo+RS+Feed_Vol+mu_cumulo+nu_cumulo+Sugar_consum ing_rate_cumulo+RQ_integral+AS_Vol.+emission_CO2+generate_C 02; 0.683, 0.855, tmp+PL+AN_cumulo+interval_Pdt.+RS+Feed_Vo l+mu_cumulo+Sugar_consuming_rate_cumulo+rab_integral+RQ_int egral+AS_Vol.; 0.688, 0.855, tmp+PL+interval_Pdt.+RS+Feed_V ol+mu_cumulo+Sugar_consuming_rate_cumulo+AS_Vol.+emission_C O2+generate_CO2; 0.673, 0.855, tmp+interval_yield+interval_ Pdt.+OD+RS+Feed_Vol+mu_cumulo+rho_cumulo+Sugar_consuming_ra te_cumulo+rab_integral+AS_Vol. +emission_O2+emission_CO2; 0. 683, 0.855, tmp+AN_cumulo+RS+Feed_Vol+mu_cumulo+nu_per_rho_ cumulo+Sugar_consuming_rate_cumulo+rab_integral+RQ_integral +AS_Vol.+emission_CO2; 0.688, 0.855, tmp+PL+AN_cumulo+RS+Fe ed_Vol+mu_cumulo+nu_per_rho_cumulo+Sugar_consuming_rate_cum ulo+rab_integral+AS_Vol.; 0.688, 0.855, tmp+AN_cumulo+inter val_Pdt.+RS+Feed_Vol+mu_cumulo+nu_cumulo+Sugar_consuming_ra te_cumulo+AS_Vol.+generate_CO2; 0.667, 0.855, tmp+AN_cumulo +interval_Pdt.+OD+RS+Feed_Vol+mu_cumulo+rho_cumulo+Sugar_co nsuming_rate_cumulo+RQ_integral+AS_Vol.+emission_O2+emissio n_CO2+generate_CO2; 0.688, 0.855, tmp+interval_yield+RS+Fee d_Vol+mu_cumulo+nu_per_rho_cumulo+Sugar_consuming_rate_cumu lo+rab_integral+AS_Vol.+emission_CO2; 0.667, 0.855, tmp+PL+ interval_Pdt.+OD+RS+Feed_Vol+mu_cumulo+nu_per_rho_cumulo+Su gar_consuming_rate_cumulo+RQ_integral+AS_Vol.+emission_O2+e mission_CO2+generate_CO2; 0.698, 0.855, tmp+interval_Pdt.+R S+Feed_Vol+mu_cumulo+rho_cumulo+Sugar_consuming_rate_cumulo +AS_Vol.; 0.693, 0.855, tmp+interval_Pdt.+OD+RS+Feed_Vol+mu _cumulo+Sugar_consuming_rate_cumulo+AS_Vol.+consum_O2; 0.66 1, 0.855, tmp+PL+AN_cumulo+interval_Pdt.+OD+RS+Feed_Vol+mu_ cumulo+rho_cumulo+Sugar_consuming_rate_cumulo+RQ_integral+A S_Vol.+emission_O2+emission_CO2+generate_CO2; 0.661, 0.855, tmp+PL+AN_cumulo+interval_yield+interval_Pdt.+OD+RS+Feed_V ol+mu_cumulo+rho_cumulo+Sugar_consuming_rate_cumulo+RQ_inte gral+AS_Vol.+emission_O2+emission_CO2; 0.673, 0.855, tmp+PL +interval_yield+OD+RS+Feed_Vol+mu_cumulo+rho_cumulo+Sugar_c onsuming_rate_cumulo+RQ_integral+AS_Vol.+emission_O2+emissi on_CO2; 0.678, 0.855, tmp+interval_yield+interval_Pdt.+OD+R S+Feed_Vol+mu_cumulo+Sugar_consuming_rate_cumulo+rab_integr al+AS_Vol.+emission_O2+emission_CO2; 0.673, 0.855, tmp+AN_c umulo+interval_Pdt.+OD+RS+Feed_Vol+mu_cumulo+nu_per_rho_cum ulo+Sugar_consuming_rate_cumulo+RQ_integral+AS_Vol.+emissio n_O2+emission_CO2; 0.688, 0.855, tmp+AN_cumulo+OD+RS+Feed_V ol+mu_cumulo+nu_cumulo+Sugar_consuming_rate_cumulo+RQ_integ ral+AS_Vol.; 0.688, 0.855, tmp+AN_cumulo+OD+RS+Feed_Vol+mu_ cumulo+Sugar_consuming_rate_cumulo+RQ_integral+AS_Vol.+cons um_O2; 0.678, 0.855, tmp+PL+OD+RS+Feed_Vol+mu_cumulo+Sugar_ consuming_rate_cumulo+RQ_integral+AS_Vol.+emission_O2+emiss ion_CO2+generate_CO2; 0.688, 0.855, tmp+AN+RS+Feed_Vol+mu_c umulo+nu_per_rho_cumulo+Sugar_consuming_rate_cumulo+rab_int egral+AS_Vol.+emission_CO2; 0.688, 0.855, tmp+AN_cumulo+int erval_yield+interval_Pdt.+RS+Feed_Vol+mu_cumulo+Sugar_consu ming_rate_cumulo+AS_Vol.+generate_CO2; 0.683, 0.855, tmp+pH +RS+Feed_Vol+mu_cumulo+Sugar_consuming_rate_cumulo+RQ_integ ral+AS_Vol.+emission_O2+emission_CO2+generate_CO2; 0.688, 0. 855, tmp+interval_yield+interval_Pdt.+RS+Feed_Vol+mu_cumulo +Sugar_consuming_rate_cumulo+rab_integral+AS_Vol.+emission_ CO2; 0.688, 0.855, tmp+AN_cumulo+interval_Pdt.+OD+RS+Feed_V ol+mu_cumulo+Sugar_consuming_rate_cumulo+rab_integral+AS_Vo l.; 0.678, 0.855, tmp+AN_cumulo+RS+Feed_Vol+mu_cumulo+rho_c umulo+Sugar_consuming_rate_cumulo+rab_integral+RQ_integral+ AS_Vol.+emission_O2+emission_CO2; 0.693, 0.855, tmp+RS+Feed _Vol+mu_cumulo+rho_cumulo+Sugar_consuming_rate_cumulo+RQ_in tegral+AS_Vol.+emission_CO2; 0.683, 0.855, tmp+PL+interval_ Pdt.+RS+Feed_Vol+mu_cumulo+Sugar_consuming_rate_cumulo+rab_ integral+AS_Vol.+emission_O2+emission_CO2; 0.688, 0.855, tm p+interval_yield+RS+Feed_Vol+mu_cumulo+rho_cumulo+Sugar_con suming_rate_cumulo+rab_integral+AS_Vol.+emission_CO2; 0.667, 0.855, tmp+PL+AN_cumulo+interval_yield+interval_Pdt.+RS+Fe ed_Vol+mu_cumulo+nu_per_rho_cumulo+Sugar_consuming_rate_cum ulo+RQ_integral+AS_Vol.+emission_O2+emission_CO2; 0.688, 0. 855, tmp+AN_cumulo+interval_yield+interval_Pdt.+OD+RS+Feed_ Vol+mu_cumulo+Sugar_consuming_rate_cumulo+AS_Vol.; 0.693, 0. 855, tmp+interval_Pdt.+OD+RS+Feed_Vol+mu_cumulo+Sugar_consu ming_rate_cumulo+rab_integral+AS_Vol.; 0.678, 0.855, tmp+pH +interval_Pdt.+RS+Feed_Vol+mu_cumulo+rho_cumulo+Sugar_consu ming_rate_cumulo+rab_integral+AS_Vol.+emission_O2+emission_ CO2; 0.672, 0.855, tmp+PL+AN_cumulo+interval_Pdt.+OD+RS+Fee d_Vol+mu_cumulo+rho_cumulo+Sugar_consuming_rate_cumulo+rab_ integral+AS_Vol.+emission_O2; 0.693, 0.855, tmp+AN+RS+Feed_ Vol+mu_cumulo+Sugar_consuming_rate_cumulo+RQ_integral+AS_Vo l.+emission_CO2; 0.688, 0.855, tmp+AN+RS+Feed_Vol+mu_cumulo +rho_cumulo+Sugar_consuming_rate_cumulo+AS_Vol.+emission_CO 2+generate_CO2; 0.688, 0.855, tmp+AN_cumulo+interval_Pdt.+R S+Feed_Vol+mu_cumulo+rho_cumulo+Sugar_consuming_rate_cumulo +AS_Vol.+generate_CO2; 0.683, 0.855, tmp+AN+interval_Pdt.+R S+Feed_Vol+mu_cumulo+Sugar_consuming_rate_cumulo+RQ_integra l+AS_Vol.+emission-O2+emission_CO2; 0.698, 0.855, tmp+inter val_Pdt.+RS+Feed_Vol+mu_cumulo+Sugar_consuming_rate_cumulo+ RQ_integral+AS_Vol.; 0.672, 0.855, tmp+PL+interval_yield+in terval_Pdt.+RS+Feed_Vol+mu_cumulo+Sugar_consuming_rate_cumu lo+rab_integral+RQ_integral+AS_Vol.+emission_O2+emission_CO 2; 0.678, 0.855, tmp+interval_Pdt.+RS+Feed_Vol+mu_cumulo+nu _cumulo+rho_cumulo+Sugar_consuming_rate_cumulo+rab_integral +AS_Vol.+emission_O2+emission_CO2; 0.683, 0.855, tmp+PL+AN_ cumulo+interval_Pdt.+RS+Feed_Vol+mu_cumulo+rho_cumulo+Sugar _consuming_rate_cumulo+rab_integral+AS_Vol.; 0.693, 0.855, tmp+AN_cumulo+interval_Pdt.+RS+Feed_Vol+mu_cumulo+nu_cumulo +Sugar_consuming_rate_cumulo+AS_Vol.; 0.688, 0.855, tmp+int erval_yield+RS+Feed_Vol+mu_cumulo+Sugar_consuming_rate_cumu lo+RQ_integral+AS_Vol.+emission_O2+emission_CO2; 0.667, 0.8 55, tmp+PL+AN+interval_Pdt.+OD+RS+Feed_Vol+mu_cumulo+nu_per _rho_cumulo+Sugar_consuming_rate_cumulo+RQ_integral+AS_Vol. +emission_O2+emission_CO2; 0.672, 0.855, tmp+AN+interval_yi eld+interval_Pdt.+RS+Feed_Vol+mu_cumulo+Sugar_consuming_rat e_cumulo+rab_integral+AS_Vol.+emission_O2+emission_CO2+gene rate_CO2; 0.667, 0.855, tmp+PL+AN+interval_yield+interval_P dt.+RS+Feed_Vol+mu_cumulo+Sugar_consuming_rate_cumulo+RQ_in tegral+AS_Vol.+emission_O2+emission_CO2+generate_CO2; 0.683, 0.855, tmp+interval_Pdt.+RS+Feed_Vol+mu_cumulo+nu_per_rho_ cumulo+Sugar_consuming_rate_cumulo+rab_integral+AS_Vol.+emi ssion_CO2+generate_CO2; 0.678, 0.855, tmp+PL+RS+Feed_Vol+mu _cumulo+nu_cumulo+rho_cumulo+Sugar_consuming_rate_cumulo+RQ _integral+AS_Vol.+emission_O2+emission_CO2; 0.683, 0.855, t mp+AN_cumulo+interval_yield+RS+Feed_Vol+mu_cumulo+Sugar_con suming_rate_cumulo+RQ_integral+AS_Vol.+emission_CO2+generat e_CO2; 0.693, 0.855, tmp+pH+interval_Pdt.+RS+Feed_Vol+mu_cu mulo+Sugar_consuming_rate_cumulo+rab_integral+AS_Vol.; 0.67 8, 0.855, tmp+AN_cumulo+interval_Pdt.+RS+Feed_Vol+mu_cumulo +rho_cumulo+nu_per_rho_cumulo+Sugar_consuming_rate_cumulo+r ab_integral+AS_Vol.+emission_O2; 0.688, 0.855, tmp+AN+AN_cu mulo+OD+RS+Feed_Vol+mu_cumulo+Sugar_consuming_rate_cumulo+R Q_integral+AS_Vol.; 0.678, 0.855, tmp+PL+AN+interval_Pdt.+R S+Feed_Vol+mu_cumulo+nu_per_rho_cumulo+Sugar_consuming_rate _cumulo+RQ_integral+emission_O2+emission_CO2; 0.667, 0.855, tmp+PL+AN+interval_Pdt.+RS+Feed_Vol+mu_cumulo+nu_cumulo+rh o_cumulo+Sugar_consuming_rate_cumulo+RQ_integral+AS_Vol.+em ission_O2+emission_CO2; 0.702, 0.855, tmp+RS+Feed_Vol+mu_cu mulo+Sugar_consuming_rate_cumulo+rab_integral+AS_Vol.; 0.67 8, 0.855, tmp+interval_Pdt.+RS+Feed_Vol+mu_cumulo+Sugar_con suming_rate_cumulo+rab_integral+RQ_integral+AS_Vol.+emissio n_O2+emission_CO2+generate_CO2; 0.688, 0.855, tmp+AN_cumulo +RS+Feed_Vol+mu_cumulo+nu_per_rho_cumulo+Sugar_consuming_ra te_cumulo+rab_integral+RQ_integral+AS_Vol.; 0.688, 0.855, t mp+PL+AN+interval_Pdt.+RS+Feed_Vol+mu_cumulo+Sugar_consumin g_rate_cumulo+rab_integral+AS_Vol.; 0.683, 0.855, tmp+PL+AN +AN_cumulo+interval_Pdt.+RS+Feed_Vol+mu_cumulo+Sugar_consum ing_rate_cumulo+rab_integral+emission_O2; 0.688, 0.855, tmp +interval_Pdt.+RS+Feed_Vol+mu_cumulo+nu_cumulo+Sugar_consum ing_rate_cumulo+AS_Vol.+emission_CO2+generate_CO2; 0.688, 0. 855, tmp+PL+AN_cumulo+interval_yield+RS+Feed_Vol+mu_cumulo+ Sugar_consuming_rate_cumulo+RQ_integral+AS_Vol.; 0.667, 0.8 55, tmp+PL+AN+AN_cumulo+interval_Pdt.+OD+RS+Feed_Vol+mu_cum ulo+rho_cumulo+Sugar_consuming_rate_cumulo+RQ_integral+AS_V ol.+emission_O2; 0.672, 0.855, tmp+PL+AN_cumulo+interval_yi eld+interval_Pdt.+OD+RS+Feed_Vol+mu_cumulo+Sugar_consuming_ rate_cumulo+RQ_integral+AS_Vol.+emission_O2; 0.697, 0.855, tmp+interval_yield+interval_Pdt.+RS+Feed_Vol+mu_cumulo+Suga r_consuming_rate_cumulo+AS_Vol.; 0.688, 0.855, tmp+OD+RS+Fe ed_Vol+mu_cumulo+Sugar_consuming_rate_cumulo+RQ_integral+AS _Vol.+emission_O2+emission_CO2; 0.661, 0.855, tmp+PL+AN+int erval_Pdt.+OD+RS+Feed_Vol+mu_cumulo+rho_cumulo+Sugar_consum ing_rate_cumulo+RQ_integral+AS_Vol.+emission_O2+emission_CO 2+generate_CO2; 0.683, 0.855, tmp+AN+AN_cumulo+interval_Pdt. +RS+Feed_Vol+mu_cumulo+Sugar_consuming_rate_cumulo+rab_inte gral+AS_Vol.+generate_CO2; 0.697, 0.855, tmp+AN_cumulo+inte rval_Pdt.+RS+Feed_Vol+mu_cumulo+Sugar_consuming_rate_cumulo +RQ_integral; 0.693, 0.855, tmp+AN+AN_cumulo+interval_Pdt.+ RS+Feed_Vol+mu_cumulo+Sugar_consuming_rate_cumulo+AS_Vol.; 0.693, 0.855, tmp+interval_yield+RS+Feed_Vol+mu_cumulo+Suga r_consuming_rate_cumulo+rab_integral+AS_Vol.+emission_CO2; 0.661, 0.855, tmp+PL+AN+interval_Pdt.+OD+RS+Feed_Vol+mu_cum ulo+rho_cumulo+Sugar_consuming_rate_cumulo+rab_integral+RQ_ integral+AS_Vol.+emission_O2+emission_CO2; 0.683, 0.855, tm p+PL+AN_cumulo+interval_Pdt.+RS+Feed_Vol+mu_cumulo+nu_per_r ho_cumulo+Sugar_consuming_rate_cumulo+RQ_integral+AS_Vol.; 0.693, 0.855, tmp+PL+AN+interval_Pdt.+RS+Feed_Vol+mu_cumulo +Sugar_consuming_rate_cumulo+AS_Vol.; 0.688, 0.855, tmp+AN+ AN_cumulo+interval_Pdt.+RS+Feed_Vol+mu_cumulo+Sugar_consumi ng_rate_cumulo+RQ_integral+emission_O2; 0.677, 0.855, tmp+A N+RS+Feed_Vol+mu_cumulo+rho_cumulo+Sugar_consuming_rate_cum ulo+rab_integral+RQ_integral+AS_Vol.+emission_O2+emission_C 02; 0.693, 0.855, tmp+AN_cumulo+interval_Pdt.+RS+Feed_Vol+m u_cumulo+rho_cumulo+Sugar_consuming_rate_cumulo+AS_Vol.; 0. 688, 0.855, tmp+AN+interval_yield+RS+Feed_Vol+mu_cumulo+Sug ar_consuming_rate_cumulo+AS_Vol.+emission_CO2+generate_CO2; 0.688, 0.855, tmp+AN_cumulo+interval_yield+RS+Feed_Vol+mu_ cumulo+Sugar_consuming_rate_cumulo+RQ_integral+AS_Vol.+emis sion_CO2; 0.677, 0.855, tmp+AN_cumulo+interval_Pdt.+OD+RS+F eed_Vol+mu_cumulo+rho_cumulo+Sugar_consuming_rate_cumulo+RQ _integral+AS_Vol.+consum_O2; 0.688, 0.855, tmp+AN_cumulo+RS +Feed_Vol+mu_cumulo+nu_cumulo+Sugar_consuming_rate_cumulo+r ab_integral+AS_Vol.+emission_CO2; 0.666, 0.855, tmp+AN_cumu lo+interval_yield+interval_Pdt.+RS+Feed_Vol+mu_cumulo+nu_pe r_rho_cumulo+Sugar_consuming_rate_cumulo+rab_integral+AS_Vo l.+emission_O2+emission_CO2+generate_CO2; 0.672, 0.855, tmp +PL+OD+RS+Feed_Vol+mu_cumulo+rho_cumulo+Sugar_consuming_rat e_cumulo+RQ_integral+AS_Vol.+emission_O2+emission_CO2+gener ate_CO2; 0.688, 0.855, tmp+pH+AN_cumulo+RS+Feed_Vol+mu_cumu lo+nu_per_rho_cumulo+Sugar_consuming_rate_cumulo+rab_integr al+AS_Vol.; 0.672, 0.855, tmp+interval_Pdt.+RS+Feed_Vol+mu_ cumulo+nu_per_rho_cumulo+Sugar_consuming_rate_cumulo+rab_in tegral+RQ_integral+AS_Vol.+emission_O2+emission_CO2+generat e_CO2; 0.672, 0.855, tmp+PL+AN_cumulo+interval_yield+interv al_Pdt.+RS+Feed_Vol+mu_cumulo+Sugar_consuming_rate_cumulo+r ab_integral+RQ_integral+AS_Vol.+emission_O2; 0.672, 0.855, tmp+PL+AN_cumulo+OD+RS+Feed_Vol+mu_cumulo+rho_cumulo+Sugar_ consuming_rate_cumulo+RQ_integral+AS_Vol.+emission_O2+emiss ion_CO2; 0.682, 0.855, tmp+AN_cumulo+RS+Feed_Vol+mu_cumulo+ rho_cumulo+Sugar_consuming_rate_cumulo+rab_integral+AS_Vol. +emission_CO2+generate_CO2; 0.677, 0.855, tmp+PL+AN_cumulo+ interval_Pdt.+OD+RS+Feed_Vol+mu_cumulo+Sugar_consuming_rate _cumulo+RQ_integral+AS_Vol.+emission_O2; 0.688, 0.855, tmp+ pH+AN_cumulo+RS+Feed_Vol+mu_cumulo+Sugar_consuming_rate_cum ulo+AS_Vol.+emission_CO2+generate_CO2; 0.677, 0.855, tmp+PL +AN_cumulo+interval_yield+interval_Pdt.+RS+Feed_Vol+mu_cumu lo+nu_per_rho_cumulo+Sugar_consuming_rate_cumulo+rab_integr al+AS_Vol.; 0.666, 0.855, tmp+PL+interval_Pdt.+RS+Feed_Vol+ mu_cumulo+nu_cumulo+rho_cumulo+Sugar_consuming_rate_cumulo+ RQ_integral+AS_Vol.+emission_O2+emission_O2+generate_CO2; 0.687, 0.855, tmp+interval_yield+RS+Feed_Vol+mu_cumulo+rho_ cumulo+Sugar_consuming_rate_cumulo+AS_Vol.+emission_CO2+gen erate_CO2; 0.677, 0.855, tmp+pH+RS+Feed_Vol+mu_cumulo+rho_c umulo+Sugar_consuming_rate_cumulo+RQ_integral+AS_Vol.+emiss ion_O2+emission_CO2+generate_CO2; 0.692, 0.855, tmp+RS+Feed _Vol+mu_cumulo+nu_cumulo+Sugar_consuming_rate_cumulo+rab_in tegral+AS_Vol.+emission_CO2; 0.672, 0.855, tmp+PL+interval_ yield+RS+Feed_Vol+mu_cumulo+nu_per_rho_cumulo+Sugar_consumi ng_rate_cumulo+RQ_integral+AS_Vol.+emission_O2+emission_CO2 +generate_CO2; 0.666, 0.855, tmp+PL+interval_yield+interval _Pdt.+RS+Feed_Vol+mu_cumulo+rho_cumulo+Sugar_consuming_rate _cumulo+RQ_integral+AS_Vol.+emission_O2+emission_CO2+genera te_CO2; 0.672, 0.855, tmp+PL+AN_cumulo+interval_Pdt.+RS+Fee d_Vol+mu_cumulo+rho_cumulo+Sugar_consuming_rate_cumulo+rab_ integral+AS_Vol.+emission_O2+emission_CO2; 0.672, 0.855, tm p+PL+AN+AN_cumulo+interval_yield+interval_Pdt.+RS+Feed_Vol+ mu_cumulo+Sugar_consuming_rate_cumulo+rab_integral+AS_Vol.+ emission_O2; 0.666, 0.855, tmp+PL+interval_Pdt.+OD+RS+Feed_ Vol+mu_cumulo+rho_cumulo+nu_per_rho_cumulo+Sugar_consuming_ rate_cumulo+RQ_integral+AS_Vol.+emission_O2+emission_CO2; 0. 687, 0.855, tmp+AN_cumulo+OD+RS+Feed_Vol+mu_cumulo+rho_cumu lo+Sugar_consuming_rate_cumulo+RQ_integral+AS_Vol.; 0.677, 0.855, tmp+interval_Pdt.+OD+RS+Feed_Vol+mu_cumulo+nu_per_rh o_cumulo+Sugar_consuming_rate_cumulo+RQ_integral+AS_Vol.+em ission_O2+emission_CO2; 0.687, 0.855, tmp+AN_cumulo+RS+Feed _Vol+mu_cumulo+nu_cumulo+Sugar_consuming_rate_cumulo+RQ_int egral+AS_Vol.+emission_CO2; 0.672, 0.855, tmp+AN_cumulo+int erval_yield+interval_Pdt.+OD+RS+Feed_Vol+mu_cumulo+rho_cumu lo+Sugar_consuming_rate_cumulo+RQ_integral+AS_Vol.+emission _O2; 0.687, 0.855, tmp+AN_cumulo+interval_Pdt.+RS+Feed_Vol+ mu_cumulo+Sugar_consuming_rate_cumulo+RQ_integral+AS_Vol.+e mission_CO2; 0.666, 0.855, tmp+PL+AN+interval_yield+interva l_Pdt.+RS+Feed_Vol+mu_cumulo+Sugar_consuming_rate_cumulo+ra b_integral+RQ_integral+AS_Vol. +emission_O2+emission_CO2; 0. 677, 0.855, tmp+AN_cumulo+interval_Pdt.+RS+Feed_Vol+mu_cumu lo+nu_cumulo+nu_per_rho_cumulo+Sugar_consuming_rate_cumulo+ rab_integral+AS_Vol.+emission_O2; 0.687, 0.855, tmp+AN+RS+F eed_Vol+mu_cumulo+Sugar_consuming_rate_cumulo+rab_integral+ AS_Vol.+emission_CO2+generate_CO2; 0.682, 0.855, tmp+PL+AN_ cumulo+interval_Pdt.+RS+Feed_Vol+mu_cumulo+nu_per_rho_cumul o+Sugar_consuming_rate_cumulo+rab_integral+emission_O2; 0.6 72, 0.854, tmp+interval_Pdt.+RS+Feed_Vol+mu_cumulo+nu_cumul o+rho_cumulo+Sugar_consuming_rate_cumulo+RQ_integral+AS_Vol. +emission_O2+emission_CO2+generate_CO2; 0.672, 0.854, tmp+P L+AN+RS+Feed_Vol+mu_cumulo+rho_cumulo+Sugar_consuming_rate_ cumulo+rab_integral+RQ_integral+AS_Vol.+emission_O2+emissio n_CO2; 0.682, 0.854, tmp+AN+RS+Feed_Vol+mu_cumulo+Sugar_con suming_rate_cumulo+rab_integral+RQ_integral+AS_Vol.+emissio n_O2+emission_CO2; 0.692, 0.854, tmp+AN_cumulo+RS+Feed_Vol+ mu_cumulo+Sugar_consuming_rate_cumulo+RQ_integral+AS_Vol.+g enerate_CO2; 0.697, 0.854, tmp+AN_cumulo+interval_Pdt.+RS+F eed_Vol+mu_cumulo+Sugar_consuming_rate_cumulo+generate_CO2; 0.672, 0.854, tmp+interval_yield+interval_Pdt.+RS+Feed_Vol +mu_cumulo+rho_cumulo+Sugar_consuming_rate_cumulo+RQ_integr al+AS_Vol.+emission_O2+emission_CO2+generate_CO2; 0.666, 0. 854, tmp+PL+AN+interval_yield+interval_Pdt.+RS+Feed_Vol+mu_ cumulo+rho_cumulo+Sugar_consuming_rate_cumulo+RQ_integral+A S_Vol.+emission_O2+emission_CO2; 0.672, 0.854, tmp+AN+AN_cu mulo+interval_Pdt.+OD+RS+Feed_Vol+mu_cumulo+rho_cumulo+Suga r_consuming_rate_cumulo+rab_integral+AS_Vol.+emission_O2; 0. 682, 0.854, tmp+AN+AN_cumulo+interval_Pdt.+OD+RS+Feed_Vol+m u_cumulo+rho_cumulo+Sugar_consuming_rate_cumulo+AS_Vol.; 0. 687, 0.854, tmp+interval_Pdt.+OD+RS+Feed_Vol+mu_cumulo+nu_c umulo+nu_per_rho_cumulo+Sugar_consuming_rate_cumulo+AS_Vo l.; 0.682, 0.854, tmp+AN+AN_cumulo+interval_Pdt.+OD+RS+Feed _Vol+mu_cumulo+Sugar_consuming_rate_cumulo+RQ_integral+AS_V ol.; 0.682, 0.854, tmp+PL+RS+Feed_Vol+mu_cumulo+nu_cumulo+S ugar_consuming_rate_cumulo+RQ_integral+AS_Vol.+emission_O2+ emission_CO2; 0.682, 0.854, tmp+interval_Pdt.+OD+RS+Feed_Vo l+mu_cumulo+rho_cumulo+Sugar_consuming_rate_cumulo+AS_Vol.+ emission_CO2+generate_CO2; 0.687, 0.854, tmp+pH+interval_Pd t.+RS+Feed_Vol+mu_cumulo+rho_cumulo+Sugar_consuming_rate_cu mulo+rab_integral+AS_Vol.; 0.666, 0.854, tmp+PL+AN_cumulo+i nterval_yield+interval_Pdt.+RS+Feed_Vol+mu_cumulo+nu_per_rh o_cumulo+Sugar_consuming_rate_cumulo+rab_integral+AS_Vol.+e mission_O2+emission_CO2; 0.687, 0.854, tmp+AN_cumulo+RS+Fee d_Vol+mu_cumulo+nu_per_rho_cumulo+Sugar_consuming_rate_cumu lo+rab_integral+AS_Vol.+generate_CO2; 0.682, 0.854, tmp+AN_ cumulo+RS+Feed_Vol+mu_cumulo+rho_cumulo+Sugar_consuming_rat e_cumulo+rab_integral+RQ_integral+AS_Vol.+emission_CO2; 0.6 82, 0.854, tmp+AN+interval_Pdt.+RS+Feed_Vol+mu_cumulo+rho_c umulo+Sugar_consuming_rate_cumulo+rab_integral+emission_O2+ emission_CO2; 0.671, 0.854, tmp+pH+PL+interval_Pdt.+RS+Feed _Vol+mu_cumulo+rho_cumulo+Sugar_consuming_rate_cumulo+RQ_in tegral+AS_Vol.+emission_O2+emission_CO2; 0.687, 0.854, tmp+ AN_cumulo+interval_Pdt.+RS+Feed_Vol+mu_cumulo+nu_per_rho_cu mulo+Sugar_consuming_rate_cumulo+RQ_integral+emission_O2; 0. 677, 0.854, tmp+AN_cumulo+interval_yield+interval_Pdt.+OD+R S+Feed_Vol+mu_cumulo+rho_cumulo+Sugar_consuming_rate_cumulo +rab_integral+AS_Vol.; 0.677, 0.854, tmp+pH+interval_Pdt.+R S+Feed_Vol+mu_cumulo+Sugar_consuming_rate_cumulo+RQ_integra l+AS-Vol.+emission_O2+emission_CO2+generate_CO2; 0.671, 0.8 54, tmp+AN_cumulo+interval_yield+RS+Feed_Vol+mu_cumulo+nu_p er_rho_cumulo+Sugar_consuming_rate_cumulo+RQ_integral+AS_Vo l.+emission_O2+emission_CO2+generate_CO2; 0.687, 0.854, tmp +interval_Pdt.+OD+RS+Feed_Vol+mu_cumulo+rho_cumulo+Sugar_co nsuming_rate_cumulo+AS_Vol.+emission_CO2; 0.660, 0.854, tmp +pH+PL+AN_cumulo+interval_Pdt.+OD+RS+Feed_Vol+mu_cumulo+rho _cumulo+Sugar_consuming_rate_cumulo+RQ_integral+AS_Vol.+emi ssion_O2+emission_CO2; 0.677, 0.854, tmp+interval_Pdt.+OD+R S+Feed_Vol+mu_cumulo+nu_per_rho_cumulo+Sugar_consuming_rate _cumulo+rab_integral+AS_Vol. +emission_O2+emission_CO2; 0.67 1, 0.854, tmp+AN_cumulo+RS+Feed_Vol+mu_cumulo+nu_per_rho_cu mulo+Sugar_consuming_rate_cumulo+rab_integral+RQ_integral+A S_Vol.+emission_O2+emission_CO2+generate_CO2; 0.682, 0.854, tmp+interval_Pdt.+OD+RS+Feed_Vol+mu_cumulo+rho_cumulo+Suga r_consuming_rate_cumulo+AS_Vol.+emission_CO2+consum_O2; 0.6 71, 0.854, tmp+PL+AN+interval_yield+RS+Feed_Vol+mu_cumulo+n u_per_rho_cumulo+Sugar_consuming_rate_cumulo+RQ_integral+AS _Vol. +emission_O2+emission_CO2; 0.682, 0.854, tmp+AN_cumulo +interval_Pdt.+OD+RS+Feed_Vol+mu_cumulo+Sugar_consuming_rat e_cumulo+AS_Vol.+emission_CO2+generate_CO2; 0.666, 0.854, t mp+AN_cumulo+interval_yield+interval_Pdt.+OD+RS+Feed_Vol+mu _cumulo+Sugar_consuming_rate_cumulo+rab_integral+AS_Vol.+em ission_O2+emission_CO2+generate_CO2; 0.682, 0.854, tmp+AN_c umulo+interval_Pdt.+OD+RS+Feed_Vol+mu_cumulo+rho_cumulo+Sug ar_consuming_rate_cumulo+AS_Vol.+consum_O2; 0.692, 0.854, t mp+interval_yield+OD+RS+Feed_Vol+mu_cumulo+rho_cumulo+Sugar _consuming_rate_cumulo+AS_Vol.; 0.687, 0.854, tmp+interval_ Pdt.+OD+RS+Feed_Vol+mu_cumulo+Sugar_consuming_rate_cumulo+A S_Vol.+emission_CO2+generate_CO2; 0.687, 0.854, tmp+AN_cumu lo+OD+RS+Feed_Vol+mu_cumulo+Sugar_consuming_rate_cumulo+RQ_ integral+AS_Vol.+emission_CO2; 0.682, 0.854, tmp+AN_cumulo+ interval_Pdt.+OD+RS+Feed_Vol+mu_cumulo+rho_cumulo+Sugar_con suming_rate_cumulo+AS_Vol.+emission_CO2; 0.687, 0.854, tmp+ PL+AN_cumulo+RS+Feed_Vol+mu_cumulo+Sugar_consuming_rate_cum ulo+AS_Vol.+emission_CO2+generate_CO2; 0.671, 0.854, tmp+AN +interval_yield+interval_Pdt.+RS+Feed_Vol+mu_cumulo+Sugar_c onsuming_rate_cumulo+rab_integral+RQ_integral+AS_Vol.+emiss ion_O2+emission_CO2; 0.677, 0.854, tmp+PL+RS+Feed_Vol+mu_cu mulo+nu_cumulo+nu_per_rho_cumulo+Sugar_consuming_rate_cumul o+RQ_integral+AS_Vol.+emission_O2+emission_CO2; 0.687, 0.85 4, tmp+RS+Feed_Vol+mu_cumulo+nu_cumulo+Sugar_consuming_rate _cumulo+RQ_integral+AS_Vol.+emission_CO2+generate_CO2; 0.67 1, 0.854, tmp+interval_Pdt.+OD+RS+Feed_Vol+mu_cumulo+rho_cu mulo+Sugar_consuming_rate_cumulo+rab_integral+RQ_integral+A S_Vol.+emission_O2+emission_CO2; 0.671, 0.854, tmp+AN_cumul o+interval_Pdt.+RS+Feed_Vol+mu_cumulo+rho_cumulo+nu_per_rho _cumulo+Sugar_consuming_rate_cumulo+rab_integral+AS_Vol.+em ission_O2+emission_CO2; 0.677, 0.854, tmp+AN+interval_Pdt.+ RS+Feed_Vol+mu_cumulo+Sugar_consuming_rate_cumulo+rab_integ ral+AS_Vol.+emission_O2+emission_CO2+generate_CO2; 0.666, 0. 854, tmp+PL+AN_cumulo+interval_yield+interval_Pdt.+OD+RS+Fe ed_Vol+mu_cumulo+rho_cumulo+Sugar_consuming_rate_cumulo+rab _integral+AS_Vol.+emission_O2; 0.687, 0.854, tmp+pH+AN_cumu lo+RS+Feed_Vol+mu_cumulo+Sugar_consuming_rate_cumulo+rab_in tegral+AS_Vol.+emission_CO2; 0.687, 0.854, tmp+interval_yie ld+interval_Pdt.+RS+Feed_Vol+mu_cumulo+rho_cumulo+Sugar_con suming_rate_cumulo+rab_integral+AS_Vol.; 0.692, 0.854, tmp+ OD+RS+Feed_Vol+mu_cumulo+Sugar_consuming_rate_cumulo+AS_Vol. +emission_CO2+generate_CO2; 0.677, 0.854, tmp+AN+interval_P dt.+RS+Feed_Vol+mu_cumulo+Sugar_consuming_rate_cumulo+rab_i ntegral+RQ_integral+AS_Vol.+emission_O2+emission_CO2; 0.682, 0.854, tmp+AN+RS+Feed_Vol+mu_cumulo+nu_per_rho_cumulo+Suga r_consuming_rate_cumulo+RQ_integral+AS_Vol.+emission_CO2+ge nerate_CO2; 0.671, 0.854, tmp+interval_yield+interval_Pdt.+ OD+RS+Feed_Vol+mu_cumulo+Sugar_consuming_rate_cumulo+RQ_int egral+AS_Vol.+emission_O2+emission_CO2+generate_CO2; 0.677, 0.854, tmp+AN_cumulo+interval_Pdt.+OD+RS+Feed_Vol+mu_cumul o+Sugar_consuming_rate_cumulo+RQ_integral+AS_Vol.+emission_ O2+emission_CO2; 0.687, 0.854, tmp+AN_cumulo+interval_yield +RS+Feed_Vol+mu_cumulo+Sugar_consuming_rate_cumulo+RQ_integ ral+AS_Vol.+generate_CO2; 0.666, 0.854, tmp+PL+AN_cumulo+in terval_yield+interval_Pdt.+RS+Feed_Vol+mu_cumulo+Sugar_cons uming rate cumulo+rab integral+AS Vol.+emission O2+emission _CO2+generate_CO2; 0.677, 0.854, tmp+OD+RS+Feed_Vol+mu_cumu lo+nu_cumulo+Sugar_consuming_rate_cumulo+RQ_integral+AS_Vol. +emission_O2+emission_CO2+generate_CO2; 0.671, 0.854, tmp+P L+AN_cumulo+RS+Feed_Vol+mu_cumulo+nu_cumulo+Sugar_consuming _rate_cumulo+RQ_integral+AS_Vol.+emission_O2+emission_CO2+g enerate_CO2; 0.666, 0.854, tmp+AN_cumulo+interval_yield+int erval_Pdt.+OD+RS+Feed_Vol+mu_cumulo+nu_per_rho_cumulo+Sugar _consuming_rate_cumulo+rab_integral+AS_Vol.+emission_O2+emi ssion_CO2; 0.682, 0.854, tmp+AN_cumulo+interval_yield+inter val_Pdt.+OD+RS+Feed_Vol+mu_cumulo+Sugar_consuming_rate_cumu lo+AS_Vol.+consum_O2; 0.682, 0.854, tmp+AN_cumulo+interval_ Pdt.+OD+RS+Feed_Vol+mu_cumulo+Sugar_consuming_rate_cumulo+R Q_integral+AS_Vol.+generate_CO2; 0.692, 0.854, tmp+AN_cumul o+RS+Feed_Vol+mu_cumulo+nu_cumulo+Sugar_consuming_rate_cumu lo+RQ_integral+AS_Vol.; 0.671, 0.854, tmp+interval_yield+in terval_Pdt.+RS+Feed_Vol+mu_cumulo+nu_per_rho_cumulo+Sugar_c onsuming_rate_cumulo+rab_integral+AS_Vol.+emission_O2+emiss ion_CO2+generate_CO2; 0.671, 0.854, tmp+PL+interval_yield+R S+Feed_Vol+mu_cumulo+rho_cumulo+nu_per_rho_cumulo+Sugar_con suming_rate_cumulo+RQ_integral+AS_Vol.+emission_O2+emission _CO2; 0.671, 0.854, tmp+AN+interval_Pdt.+RS+Feed_Vol+mu_cum ulo+rho_cumulo+Sugar_consuming_rate_cumulo+rab_integral+AS_ Vol.+emission_O2+emission_CO2+generate_CO2; 0.682, 0.854, t mp+AN+AN_cumulo+interval_yield+RS+Feed_Vol+mu_cumulo+nu_per _rho_cumulo+Sugar_consuming_rate_cumulo+rab_integral+AS_Vo l.; 0.671, 0.854, tmp+PL+AN+RS+Feed_Vol+mu_cumulo+nu_per_rh o_cumulo+Sugar_consuming_rate_cumulo+RQ_integral+AS_Vol.+em ission_O2+emission_CO2+generate_CO2; 0.671, 0.854, tmp+AN+i nterval_Pdt.+RS+Feed_Vol+mu_cumulo+rho_cumulo+nu_per_rho_cu mulo+Sugar_consuming_rate_cumulo+rab_integral+AS_Vol.+emiss ion_O2+emission_CO2; 0.671, 0.854, tmp+PL+AN_cumulo+interva l_Pdt.+RS+Feed_Vol+mu_cumulo+nu_per_rho_cumulo+Sugar_consum ing_rate_cumulo+rab_integral+RQ_integral+AS_Vol.+emission_O 2; 0.676, 0.854, tmp+AN+interval_yield+RS+Feed_Vol+mu_cumul o+Sugar_consuming_rate_cumulo+RQ_integral+AS_Vol.+emission_ O2+emission_CO2+generate_CO2; 0.660, 0.854, tmp+PL+AN+inter val_yield+interval_Pdt.+OD+RS+Feed_Vol+mu_cumulo+rho_cumulo +Sugar-consuming-rate-cumulo+RQ-integral+AS-Vol.+emission-O 2+emission_CO2; 0.687, 0.854, tmp+AN_cumulo+interval_yield+ RS+Feed_Vol+mu_cumulo+nu_per_rho_cumulo+Sugar_consuming_rat e_cumulo+AS_Vol.+generate_CO2; 0.671, 0.854, tmp+AN+interva l_yield+interval_Pdt.+OD+RS+Feed_Vol+mu_cumulo+Sugar_consum ing_rate_cumulo+rab_integral+AS_Vol.+emission_O2+emission_C 02; 0.692, 0.854, tmp+interval_Pdt.+RS+Feed_Vol+mu_cumulo+S ugar_consuming_rate_cumulo+rab_integral+AS_Vol.+generate_CO 2; 0.687, 0.854, tmp+PL+RS+Feed_Vol+mu_cumulo+Sugar_consumi ng_rate_cumulo+RQ_integral+AS_Vol.+emission_CO2+generate_CO 2; 0.687, 0.854, tmp+pH+interval_Pdt.+OD+RS+Feed_Vol+mu_cum ulo+rho_cumulo+Sugar_consuming_rate_cumulo+AS_Vol.; 0.682, 0.854, tmp+AN+AN_cumulo+RS+Feed_Vol+mu_cumulo+Sugar_consumi ng_rate_cumulo+rab_integral+AS_Vol.+emission_CO2+generate_C 02; 0.687, 0.854, tmp+AN+AN_cumulo+interval_Pdt.+RS+Feed_Vo l+mu_cumulo+Sugar_consuming_rate_cumulo+AS_Vol.+generate_CO 2; 0.671, 0.854, tmp+AN+AN_cumulo+interval_yield+interval_P dt.+RS+Feed_Vol+mu_cumulo+Sugar_consuming_rate_cumulo+rab_i ntegral+AS_Vol.+emission_O2+emission_CO2; 0.671, 0.854, tmp +pH+interval_Pdt.+RS+Feed_Vol+mu_cumulo+nu_per_rho_cumulo+S ugar_consuming_rate_cumulo+RQ_integral+AS_Vol.+emission_O2+ emission_CO2+generate_CO2; 0.671, 0.854, tmp+interval_yield +interval_Pdt.+RS+Feed_Vol+mu_cumulo+rho_cumulo+Sugar_consu ming_rate_cumulo+rab_integral+AS_Vol.+emission_O2+emission_ CO2+generate_CO2; 0.676, 0.854, tmp+AN_cumulo+interval_yiel d+interval_Pdt.+RS+Feed_Vol+mu_cumulo+nu_per_rho_cumulo+Sug ar_consuming_rate_cumulo+rab_integral+AS_Vol.+generate_CO2; 0.671, 0.854, tmp+PL+RS+Feed_Vol+mu_cumulo+nu_cumulo+nu_pe r_rho_cumulo+Sugar_consuming_rate_cumulo+RQ_integral+AS_Vol. +emission_O2+emission_CO2+generate_CO2; 0.682, 0.854, tmp+P L+AN_cumulo+interval_yield+RS+Feed_Vol+mu_cumulo+nu_per_rho _cumulo+Sugar_consuming_rate_cumulo+rab_integral+AS_Vol.; 0. 682, 0.854, tmp+interval_Pdt.+OD+RS+Feed_Vol+mu_cumulo+rho_ cumulo+Sugar_consuming_rate_cumulo+rab_integral+AS_Vol.+emi ssion_CO2; 0.687, 0.854, tmp+interval_Pdt.+RS+Feed_Vol+mu_c umulo+rho_cumulo+nu_per_rho_cumulo+Sugar_consuming_rate_cum ulo+rab_integral+AS_Vol.; 0.671, 0.854, tmp+PL+RS+Feed_Vol+ mu_cumulo+nu_cumulo+rho_cumulo+Sugar_consuming_rate_cumulo+ RQ_integral+AS_Vol.+emission_O2+emission_O2+generate_CO2; 0.692, 0.854, tmp+AN+AN_cumulo+interval_Pdt.+RS+Feed_Vol+mu _cumulo+Sugar_consuming_rate_cumulo+rab_integral; 0.682, 0. 854, tmp+AN+interval_yield+interval_Pdt.+RS+Feed_Vol+mu_cum ulo+Sugar_consuming_rate_cumulo+rab_integral+AS_Vol.+emissi on_CO2; 0.682, 0.854, tmp+AN+interval_Pdt.+RS+Feed_Vol+mu_c umulo+rho_cumulo+Sugar_consuming_rate_cumulo+AS_Vol.+emissi on_CO2+generate_CO2; 0.687, 0.854, tmp+PL+AN_cumulo+interva l_Pdt.+RS+Feed_Vol+mu_cumulo+nu_per_rho_cumulo+Sugar_consum ing_rate_cumulo+AS_Vol.; 0.676, 0.854, tmp+PL+AN+AN_cumulo+ interval_yield+interval_Pdt.+RS+Feed_Vol+mu_cumulo+Sugar_co nsuming_rate_cumulo+rab_integral+emission_O2; 0.676, 0.854, tmp+PL+AN_cumulo+interval_yield+interval_Pdt.+RS+Feed_Vol+ mu_cumulo+Sugar_consuming_rate_cumulo+RQ_integral+AS_Vol.+e mission_O2; 0.687, 0.854, tmp+AN+AN_cumulo+interval_yield+R S+Feed_Vol+mu_cumulo+Sugar_consuming_rate_cumulo+RQ_integra l+AS_Vol.; 0.671, 0.854, tmp+AN+RS+Feed_Vol+mu_cumulo+rho_c umulo+nu_per_rho_cumulo+Sugar_consuming_rate_cumulo+RQ_inte gral+AS_Vol. +emission_O2+emission_CO2+generate_CO2; 0.665, 0.854, tmp+PL+AN_cumulo+interval_yield+interval_Pdt.+OD+RS+ Feed_Vol+mu_cumulo+rho_cumulo+Sugar_consuming_rate_cumulo+R Q_integral+AS_Vol.+emission_O2; 0.682, 0.854, tmp+AN+interv al_Pdt.+RS+Feed_Vol+mu_cumulo+Sugar_consuming_rate_cumulo+A S_Vol.+emission_O2+emission_CO2+generate_CO2; 0.682, 0.854, tmp+PL+AN_cumulo+interval_Pdt.+RS+Feed_Vol+mu_cumulo+Sugar _consuming_rate_cumulo+rab_integral+AS_Vol.+emission_CO2; 0. 682, 0.854, tmp+pH+AN_cumulo+interval_Pdt.+RS+Feed_Vol+mu_c umulo+Sugar_consuming_rate_cumulo+rab_integral+AS_Vol.+emis sion_O2; 0.687, 0.854, tmp+interval_Pdt.+OD+RS+Feed_Vol+mu_ cumulo+nu_per_rho_cumulo+Sugar_consuming_rate_cumulo+rab_in tegral+AS_Vol.; 0.696, 0.854, tmp+interval_Pdt.+RS+Feed_Vol +mu_cumulo+Sugar_consuming_rate_cumulo+AS_Vol.+emission_CO 2; 0.687, 0.854, tmp+AN_cumulo+interval_Pdt.+RS+Feed_Vol+mu _cumulo+Sugar_consuming_rate_cumulo+rab_integral+emission_O 2+generate_CO2; 0.681, 0.854, tmp+PL+RS+Feed_Vol+mu_cumulo+ Sugar_consuming_rate_cumulo+rab_integral+RQ_integral+AS_Vol. +emission_O2+emission_CO2; 0.671, 0.854, tmp+pH+AN_cumulo+i nterval_yield+interval_Pdt.+RS+Feed_Vol+mu_cumulo+Sugar_con suming_rate_cumulo+rab_integral+AS_Vol.+emission_O2+emissio n_CO2; 0.691, 0.854, tmp+RS+Feed_Vol+mu_cumulo+Sugar_consum ing_rate_cumulo+RQ_integral+AS_Vol.+emission_CO2+consum_O2; 0.665, 0.854, tmp+PL+AN_cumulo+interval_yield+interval_Pdt. +OD+RS+Feed_Vol+mu_cumulo+Sugar_consuming_rate_cumulo+RQ_in tegral+AS_Vol.+emission_O2+emission_CO2; 0.681, 0.854, tmp+ AN_cumulo+RS+Feed_Vol+mu_cumulo+nu_cumulo+Sugar_consuming_r ate_cumulo+RQ_integral+AS_Vol.+emission_O2+emission_CO2; 0. 681, 0.854, tmp+AN_cumulo+interval_yield+RS+Feed_Vol+mu_cum ulo+nu_per_rho_cumulo+Sugar_consuming_rate_cumulo+rab_integ ral+AS_Vol.+generate_CO2; 0.676, 0.854, tmp+AN_cumulo+inter val_yield+interval_Pdt.+OD+RS+Feed_Vol+mu_cumulo+Sugar_cons uming_rate_cumulo+RQ_integral+AS_Vol.+consum_O2; 0.665, 0.8 54, tmp+PL+AN_cumulo+interval_yield+interval_Pdt.+OD+RS+Fee d_Vol+mu_cumulo+Sugar_consuming_rate_cumulo+rab_integral+AS _Vol.+emission_O2+emission_CO2; 0.687, 0.854, tmp+PL+AN+AN_ cumulo+RS+Feed_Vol+mu_cumulo+Sugar_consuming_rate_cumulo+ra b_integral+AS_Vol.; 0.681, 0.854, tmp+pH+AN_cumulo+interval _Pdt.+RS+Feed_Vol+mu_cumulo+nu_per_rho_cumulo+Sugar_consumi ng_rate_cumulo+rab_integral+AS_Vol.; 0.687, 0.854, tmp+RS+F eed_Vol+mu_cumulo+nu_cumulo+Sugar_consuming_rate_cumulo+RQ_ integral+AS_Vol.+emission_O2+emission_CO2; 0.676, 0.854, tm p+AN+AN_cumulo+RS+Feed_Vol+mu_cumulo+rho_cumulo+Sugar_consu ming_rate_cumulo+RQ_integral+AS_Vol.+emission_CO2+generate CO2; 0.676, 0.854, tmp+pH+interval_Pdt.+RS+Feed_Vol+mu_cumu lo+nu_per_rho_cumulo+Sugar_consuming_rate_cumulo+rab_integr al+AS_Vol.+emission_O2+emission_CO2; 0.671, 0.854, tmp+AN+i nterval_Pdt.+RS+Feed_Vol+mu_cumulo+nu_per_rho_cumulo+Sugar_ consuming_rate_cumulo+rab_integral+AS_Vol.+emission_O2+emis sion_CO2+generate_CO2; 0.671, 0.854, tmp+AN+interval_Pdt.+O D+RS+Feed_Vol+mu_cumulo+rho_cumulo+Sugar_consuming_rate_cum ulo+RQ_integral+AS_Vol.+emission_O2+emission_CO2; 0.681, 0. 854, tmp+AN_cumulo+interval_Pdt.+RS+Feed_Vol+mu_cumulo+nu_p er_rho_cumulo+Sugar_consuming_rate_cumulo+RQ_integral+AS_Vo l.+generate_CO2; 0.665, 0.854, tmp+pH+PL+AN+interval_Pdt.+R S+Feed_Vol+mu_cumulo+nu_per_rho_cumulo+Sugar_consuming_rate _cumulo+RQ_integral+AS_Vol.+emission_O2+emission_CO2; 0.676, 0.854, tmp+AN_cumulo+interval_yield+interval_Pdt.+RS+Feed_ Vol+mu_cumulo+nu_per_rho_cumulo+Sugar_consuming_rate_cumulo +rab_integral+AS_Vol.+emission_CO2; 0.665, 0.854, tmp+PL+in terval_Pdt.+OD+RS+Feed_Vol+mu_cumulo+nu_cumulo+nu_per_rho_c umulo+Sugar_consuming_rate_cumulo+RQ_integral+AS_Vol.+emiss ion_O2+emission_CO2; 0.671, 0.854, tmp+PL+AN+OD+RS+Feed_Vol +mu_cumulo+rho_cumulo+Sugar_consuming_rate_cumulo+RQ_integr al+AS_Vol.+emission_O2+emission_CO2; 0.691, 0.854, tmp+AN+i nterval_Pdt.+OD+RS+Feed_Vol+mu_cumulo+Sugar_consuming_rate_ cumulo+AS_Vol.; 0.676, 0.854, tmp+PL+interval_yield+RS+Feed _Vol+mu_cumulo+Sugar_consuming_rate_cumulo+RQ_integral+AS_V ol.+emission_O2+emission_CO2+generate_CO2; 0.681, 0.854, tm p+interval_Pdt.+RS+Feed_Vol+mu_cumulo+nu_per_rho_cumulo+Sug ar_consuming_rate_cumulo+RQ_integral+AS_Vol.+emission_CO2+g enerate_CO2; 0.671, 0.854, tmp+AN_cumulo+interval_Pdt.+RS+F eed_Vol+mu_cumulo+rho_cumulo+Sugar_consuming_rate_cumulo+ra b_integral+AS_Vol.+emission_O2+emission_CO2+generate_CO2; 0. 686, 0.854, tmp+PL+AN+interval_Pdt.+RS+Feed_Vol+mu_cumulo+n u_per_rho_cumulo+Sugar_consuming_rate_cumulo+AS_Vol.; 0.671, 0.854, tmp+AN+AN_cumulo+OD+RS+Feed_Vol+mu_cumulo+Sugar_con suming_rate_cumulo+RQ_integral+AS_Vol.+emission_O2+emission _CO2+generate_CO2; 0.681, 0.854, tmp+AN+interval_yield+inte rval_Pdt.+RS+Feed_Vol+mu_cumulo+Sugar_consuming_rate_cumulo +rab_integral+emission_O2+emission_CO2; 0.681, 0.854, tmp+A N+AN_cumulo+interval_Pdt.+RS+Feed_Vol+mu_cumulo+Sugar_consu ming_rate_cumulo+rab_integral+RQ_integral+AS_Vol.; 0.681, 0. 854, tmp+AN+RS+Feed_Vol+mu_cumulo+rho_cumulo+Sugar_consumin g_rate_cumulo+RQ_integral+AS_Vol.+emission_CO2+generate_CO 2; 0.681, 0.854, tmp+AN_cumulo+interval_Pdt.+OD+RS+Feed_Vol +mu_cumulo+nu_per_rho_cumulo+Sugar_consuming_rate_cumulo+ra b_integral+AS_Vol.; 0.671, 0.854, tmp+AN_cumulo+interval_Pd t.+RS+Feed_Vol+mu_cumulo+nu_cumulo+nu_per_rho_cumulo+Sugar_ consuming_rate_cumulo+rab_integral+AS_Vol.+emission_O2+emis sion_CO2; 0.681, 0.854, tmp+AN+interval_yield+interval_Pdt. +OD+RS+Feed_Vol+mu_cumulo+Sugar_consuming_rate_cumulo+AS_Vo l.+consum_O2; 0.676, 0.854, tmp+AN+interval_Pdt.+OD+RS+Feed _Vol+mu_cumulo+rho_cumulo+Sugar_consuming_rate_cumulo+rab_i ntegral+AS_Vol.+consum_O2; 0.676, 0.854, tmp+RS+Feed_Vol+mu _cumulo+nu_per_rho_cumulo+Sugar_consuming_rate_cumulo+rab_i ntegral+RQ_integral+AS_Vol.+emission_O2+emission_CO2+genera te_CO2; 0.671, 0.854, tmp+PL+AN+interval_Pdt.+OD+RS+Feed_Vo l+mu_cumulo+rho_cumulo+Sugar_consuming_rate_cumulo+RQ_integ ral+emission_O2+emission_CO2; 0.681, 0.854, tmp+AN_cumulo+i nterval_Pdt.+RS+Feed_Vol+mu_cumulo+nu_per_rho_cumulo+Sugar_ consuming_rate_cumulo+RQ_integral+AS_Vol.+emission_O2; 0.67 1, 0.854, tmp+AN_cumulo+interval_yield+interval_Pdt.+OD+RS+ Feed_Vol+mu_cumulo+Sugar_consuming_rate_cumulo+RQ_integral+ AS_Vol.+emission_O2+emission_CO2; 0.691, 0.854, tmp+AN+inte rval_Pdt.+RS+Feed_Vol+mu_cumulo+rho_cumulo+Sugar_consuming_ rate_cumulo+rab_integral; 0.676, 0.854, tmp+AN_cumulo+inter val_Pdt.+RS+Feed_Vol+mu_cumulo+nu_cumulo+rho_cumulo+Sugar_c onsuming_rate_cumulo+rab_integral+AS_Vol.+emission_O2; 0.67 1, 0.854, tmp+PL+interval_Pdt.+OD+RS+Feed_Vol+mu_cumulo+Sug ar_consuming_rate_cumulo+RQ_integral+AS_Vol.+emission_O2+em ission_CO2+generate_CO2; 0.659, 0.854, tmp+PL+AN_cumulo+int erval_yield+interval_Pdt.+RS+Feed_Vol+mu_cumulo+nu_per_rho_ cumulo+Sugar_consuming_rate_cumulo+RQ_integral+AS_Vol.+emis sion_O2+emission_CO2+generate_CO2; 0.686, 0.854, tmp+RS+Fee d_Vol+mu_cumulo+rho_cumulo+nu_per_rho_cumulo+Sugar_consumin g_rate_cumulo+rab_integral+AS_Vol.+emission_CO2; 0.671, 0.8 54, tmp+AN+interval_Pdt.+RS+Feed_Vol+mu_cumulo+rho_cumulo+S ugar_consuming_rate_cumulo+rab_integral+RQ_integral+AS_Vol. +emission_O2+emission_CO2; 0.686, 0.854, tmp+pH+interval_Pd t.+RS+Feed_Vol+mu_cumulo+Sugar_consuming_rate_cumulo+AS_Vol. +emission_CO2+generate_CO2; 0.681, 0.854, tmp+PL+AN_cumulo+ RS+Feed_Vol+mu_cumulo+nu_per_rho_cumulo+Sugar_consuming_rat e_cumulo+RQ_integral+AS_Vol.+emission_CO2; 0.681, 0.854, tm p+interval_Pdt.+RS+Feed_Vol+mu_cumulo+nu_cumulo+Sugar_consu ming-rate-cumulo+rab-integral+AS-Vol.+emission-O2+emission_ CO2; 0.691, 0.854, tmp+RS+Feed_Vol+mu_cumulo+Sugar_consumin g_rate_cumulo+rab_integral+AS_Vol.+emission_CO2+consum_O2; 0.659, 0.854, tmp+pH+PL+AN+interval_Pdt.+OD+RS+Feed_Vol+mu_ cumulo+rho_cumulo+Sugar_consuming_rate_cumulo+RQ_integral+A S_Vol.+emission_O2+emission_CO2; 0.676, 0.854, tmp+PL+AN_cu mulo+RS+Feed_Vol+mu_cumulo+nu_per_rho_cumulo+Sugar_consumin g_rate_cumulo+RQ_integral+AS_Vol.+emission_CO2+generate_CO 2; 0.676, 0.854, tmp+AN+AN_cumulo+interval_Pdt.+RS+Feed_Vol +mu_cumulo+Sugar_consuming_rate_cumulo+rab_integral+RQ_inte gral+AS_Vol.+emission_O2; 0.691, 0.854, tmp+AN+interval_yie ld+RS+Feed_Vol+mu_cumulo+nu_per_rho_cumulo+Sugar_consuming_ rate_cumulo+AS_Vol.; 0.676, 0.854, tmp+AN_cumulo+interval_P dt.+OD+RS+Feed_Vol+mu_cumulo+rho_cumulo+Sugar_consuming_rat e_cumulo+RQ_integral+AS_Vol.+generate_CO2; 0.665, 0.854, tm p+pH+PL+interval_Pdt.+RS+Feed_Vol+mu_cumulo+nu_per_rho_cumu lo+Sugar_consuming_rate_cumulo+RQ_integral+AS_Vol.+emission _O2+emission_CO2+generate_CO2; 0.681, 0.854, tmp+AN_cumulo+ interval_Pdt.+RS+Feed_Vol+mu_cumulo+rho_cumulo+Sugar_consum ing_rate_cumulo+AS_Vol.+emission_CO2+generate_CO2; 0.686, 0. 854, tmp+AN+AN_cumulo+RS+Feed_Vol+mu_cumulo+rho_cumulo+Suga r_consuming_rate_cumulo+rab_integral+AS_Vol.; 0.676, 0.854, tmp+AN+interval_yield+interval_Pdt.+OD+RS+Feed_Vol+mu_cumu lo+rho_cumulo+Sugar_consuming_rate_cumulo+AS_Vol.+consum_O 2; 0.665, 0.854, tmp+PL+interval_yield+interval_Pdt.+RS+Fee d_Vol+mu_cumulo+nu_per_rho_cumulo+Sugar_consuming_rate_cumu lo+rab_integral+RQ_integral+AS_Vol.+emission_O2+emission_CO 2; 0.700, 0.854, tmp+AN+interval_Pdt.+RS+Feed_Vol+mu_cumulo +Sugar_consuming_rate_cumulo; 0.686, 0.854, tmp+interval_yi eld+RS+Feed_Vol+mu_cumulo+Sugar_consuming_rate_cumulo+RQ_in tegral+AS_Vol.+emission_CO2+generate_CO2; 0.670, 0.854, tmp +PL+AN+AN_cumulo+interval_Pdt.+RS+Feed_Vol+mu_cumulo+nu_per _rho_cumulo+Sugar_consuming_rate_cumulo+rab_integral+AS_Vol. +emission_O2; 0.670, 0.854, tmp+PL+AN_cumulo+interval_yield +interval_Pdt.+RS+Feed_Vol+mu_cumulo+rho_cumulo+Sugar_consu ming_rate_cumulo+rab_integral+AS_Vol.+emission_O2; 0.681, 0. 854, tmp+AN_cumulo+interval_Pdt.+RS+Feed_Vol+mu_cumulo+rho_ cumulo+Sugar_consuming_rate_cumulo+rab_integral+RQ_integral +AS_Vol.; 0.681, 0.854, tmp+PL+AN+AN_cumulo+interval_Pdt.+R S+Feed_Vol+mu_cumulo+Sugar_consuming_rate_cumulo+RQ_integra l+emission_O2; 0.681, 0.854, tmp+AN+AN_cumulo+RS+Feed_Vol+m u_cumulo+Sugar_consuming_rate_cumulo+RQ_integral+AS_Vol.+em ission_O2+emission_CO2; 0.681, 0.854, tmp+pH+interval_Pdt.+ OD+RS+Feed_Vol+mu_cumulo+rho_cumulo+Sugar_consuming_rate_cu mulo+rab_integral+AS_Vol.; 0.665, 0.854, tmp+PL+AN_cumulo+i nterval_Pdt.+RS+Feed_Vol+mu_cumulo+rho_cumulo+nu_per_rho_cu mulo+Sugar_consuming_rate_cumulo+RQ_integral+AS_Vol.+emissi on_O2+emission_CO2; 0.681, 0.854, tmp+AN+interval_Pdt.+OD+R S+Feed_Vol+mu_cumulo+rho_cumulo+Sugar_consuming_rate_cumulo +AS_Vol.+generate_CO2; 0.691, 0.854, tmp+interval_Pdt.+RS+F eed_Vol+mu_cumulo+Sugar_consuming_rate_cumulo+rab_integral+ RQ_integral+AS_Vol.; 0.686, 0.854, tmp+interval_Pdt.+OD+RS+ Feed_Vol+mu_cumulo+rho_cumulo+nu_per_rho_cumulo+Sugar_consu ming_rate_cumulo+AS_Vol.; 0.670, 0.854, tmp+AN+OD+RS+Feed_V ol+mu_cumulo+rho_cumulo+Sugar_consuming_rate_cumulo+RQ_inte gral+AS_Vol.+emission_O2+emission_CO2+generate_CO2; 0.670, 0.854, tmp+AN+interval_Pdt.+RS+Feed_Vol+mu_cumulo+nu_cumulo +nu_per_rho_cumulo+Sugar_consuming_rate_cumulo+rab_integral +AS_Vol.+emission_O2+emission_CO2; 0.665, 0.854, tmp+PL+int erval_Pdt.+RS+Feed_Vol+mu_cumulo+rho_cumulo+Sugar_consuming _rate_cumulo+rab_integral+RQ_integral+AS_Vol.+emission_O2+e mission_CO2+generate_CO2; 0.686, 0.854, tmp+interval_yield+ RS+Feed_Vol+mu_cumulo+Sugar_consuming_rate_cumulo+rab_integ ral+AS_Vol.+emission_CO2+generate_CO2; 0.681, 0.854, tmp+PL +AN_cumulo+RS+Feed_Vol+mu_cumulo+nu_per_rho_cumulo+Sugar_co nsuming_rate_cumulo+rab_integral+AS_Vol.+emission_CO2; 0.68 1, 0.854, tmp+interval_Pdt.+RS+Feed_Vol+mu_cumulo+rho_cumul o+Sugar_consuming_rate_cumulo+rab_integral+AS_Vol.+emission _CO2+generate_CO2; 0.670, 0.854, tmp+RS+Feed_Vol+mu_cumulo+ nu_cumulo+rho_cumulo+nu_per_rho_cumulo+Sugar_consuming_rate _cumulo+RQ_integral+AS_Vol.+emission_O2+emission_CO2+genera te_CO2; 0.681, 0.854, tmp+AN_cumulo+interval_yield+interval _Pdt.+RS+Feed_Vol+mu_cumulo+Sugar_consuming_rate_cumulo+AS_ Vol.+emission_CO2+generate_CO2; 0.681, 0.854, tmp+AN+AN_cum ulo+RS+Feed_Vol+mu_cumulo+nu_per_rho_cumulo+Sugar_consuming _rate_cumulo+rab_integral+AS_Vol.+emission_CO2; 0.676, 0.85 4, tmp+PL+AN_cumulo+RS+Feed_Vol+mu_cumulo+Sugar_consuming_r ate_cumulo+rab_integral+RQ_integral+AS_Vol.+emission_O2+emi ssion_CO2; 0.676, 0.854, tmp+AN+AN_cumulo+interval_yield+in terval_Pdt.+RS+Feed_Vol+mu_cumulo+nu_per_rho_cumulo+Sugar_c onsuming_rate_cumulo+rab_integral+AS_Vol.; 0.681, 0.854, tm p+RS+Feed_Vol+mu_cumulo+rho_cumulo+nu_per_rho_cumulo+Sugar_ consuming_rate_cumulo+RQ_integral+AS_Vol.+emission_O2+emiss ion_CO2; 0.676, 0.854, tmp+interval_yield+interval_Pdt.+RS+ Feed_Vol+mu_cumulo+nu_cumulo+Sugar_consuming_rate_cumulo+ra b_integral+AS_Vol.+emission_O2+emission_CO2; 0.681, 0.854, tmp+AN+AN_cumulo+RS+Feed_Vol+mu_cumulo+nu_per_rho_cumulo+Su gar_consuming_rate_cumulo+RQ_integral+AS_Vol.+emission_CO2; 0.691, 0.854, tmp+AN+interval_Pdt.+RS+Feed_Vol+mu_cumulo+n u_per_rho_cumulo+Sugar_consuming_rate_cumulo+rab_integral; 0.686, 0.854, tmp+OD+RS+Feed_Vol+mu_cumulo+Sugar_consuming_ rate_cumulo+RQ_integral+AS_Vol.+emission_CO2+consum_O2; 0.6 70, 0.854, tmp+PL+interval_yield+interval_Pdt.+RS+Feed_Vol+ mu_cumulo+rho_cumulo+Sugar_consuming_rate_cumulo+rab_integr al+AS_Vol.+emission_02+emission_CO2; 0.670, 0.854, tmp+inte rval_Pdt.+OD+RS+Feed_Vol+mu_cumulo+rho_cumulo+Sugar_consumi ng_rate_cumulo+rab_integral+AS_Vol.+emission_O2+emission_CO 2+generate_CO2; 0.676, 0.854, tmp+PL+AN+AN_cumulo+interval_ Pdt.+RS+Feed_Vol+mu_cumulo+nu_per_rho_cumulo+Sugar_consumin g_rate_cumulo+rab_integral+AS_Vol.; 0.686, 0.854, tmp+AN+in terval_Pdt.+RS+Feed_Vol+mu_cumulo+nu_cumulo+Sugar_consuming _rate_cumulo+rab_integral+AS_Vol.; 0.670, 0.854, tmp+AN+int erval_yield+RS+Feed_Vol+mu_cumulo+nu_per_rho_cumulo+Sugar_c onsuming_rate_cumulo+RQ_integral+AS_Vol.+emission_O2+emissi on_CO2+generate_CO2; 0.691, 0.854, tmp+interval_Pdt.+RS+Fee d_Vol+mu_cumulo+nu_per_rho_cumulo+Sugar_consuming_rate_cumu lo+AS_Vol.+emission_CO2; 0.659, 0.854, tmp+PL+AN_cumulo+int erval_yield+interval_Pdt.+OD+RS+Feed_Vol+mu_cumulo+Sugar_co nsuming_rate_cumulo+RQ_integral+AS_Vol.+emission_O2+emissio n_CO2+generate_CO2; 0.670, 0.854, tmp+AN_cumulo+interval_Pd t.+OD+RS+Feed_Vol+mu_cumulo+nu_per_rho_cumulo+Sugar_consumi ng_rate_cumulo+rab_integral+AS_Vol.+emission_O2+emission_CO 2; 0.665, 0.854, tmp+AN_cumulo+interval_yield+interval_Pdt. +OD+RS+Feed_Vol+mu_cumulo+nu_cumulo+Sugar_consuming_rate_cu mulo+rab_integral+AS_Vol.+emission_O2+emission_CO2; 0.686, 0.854, tmp+interval_Pdt.+RS+Feed_Vol+mu_cumulo+nu_cumulo+nu _per_rho_cumulo+Sugar_consuming_rate_cumulo+rab_integral+AS _Vol.; 0.686, 0.854, tmp+AN_cumulo+interval_yield+RS+Feed_V ol+mu_cumulo+nu_cumulo+Sugar_consuming_rate_cumulo+rab_inte gral+AS_Vol.; 0.681, 0.854, tmp+PL+AN+interval_Pdt.+RS+Feed _Vol+mu_cumulo+nu_per_rho_cumulo+Sugar_consuming_rate_cumul o+rab_integral+AS_Vol.; 0.670, 0.854, tmp+AN_cumulo+interva l_yield+interval_Pdt.+RS+Feed_Vol+mu_cumulo+Sugar_consuming _rate_cumulo+rab_integral+RQ_integral+AS_Vol.+emission_O2+e mission_CO2; 0.681, 0.854, tmp+AN+interval_Pdt.+OD+RS+Feed_ Vol+mu_cumulo+rho_cumulo+Sugar_consuming_rate_cumulo+RQ_int egral+AS_Vol.; 0.686, 0.854, tmp+AN_cumulo+RS+Feed_Vol+mu_c umulo+rho_cumulo+Sugar_consuming_rate_cumulo+rab_integral+R Q_integral+AS_Vol.; 0.681, 0.854, tmp+PL+interval_Pdt.+RS+F eed_Vol+mu_cumulo+nu_per_rho_cumulo+RQ_integral+AS_Vol.+emi ssion_O2+emission_CO2; 0.686, 0.854, tmp+AN_cumulo+RS+Feed_ Vol+mu_cumulo+rho_cumulo+nu_per_rho_cumulo+Sugar_consuming_ rate_cumulo+rab_integral+AS_Vol.; 0.676, 0.854, tmp+interva l_yield+interval_Pdt.+OD+RS+Feed_Vol+mu_cumulo+rho_cumulo+S ugar_consuming_rate_cumulo+rab_integral+AS_Vol.+consum_O2; 0.686, 0.854, tmp+pH+interval_Pdt.+RS+Feed_Vol+mu_cumulo+nu _per_rho_cumulo+Sugar_consuming_rate_cumulo+rab_integral+AS _Vol.; 0.686, 0.854, tmp+RS+Feed_Vol+mu_cumulo+rho_cumulo+n u_per_rho_cumulo+Sugar_consuming_rate_cumulo+AS_Vol.+emissi on_CO2+generate_CO2; 0.670, 0.854, tmp+AN+AN_cumulo+interva l_Pdt.+OD+RS+Feed_Vol+mu_cumulo+nu_per_rho_cumulo+Sugar_con suming_rate_cumulo+RQ_integral+AS_Vol.+emission_O2; 0.670, 0.854, tmp+pH+AN_cumulo+interval_yield+interval_Pdt.+OD+RS+ Feed_Vol+mu_cumulo+Sugar_consuming_rate_cumulo+rab_integral +AS_Vol.+emission_O2; 0.691, 0.854, tmp+AN_cumulo+interval_ Pdt.+RS+Feed_Vol+mu_cumulo+Sugar_consuming_rate_cumulo+AS_V ol.+emission_CO2; 0.686, 0.854, tmp+interval_Pdt.+OD+RS+Fee d_Vol+mu_cumulo+nu_cumulo+rho_cumulo+Sugar_consuming_rate_c umulo+AS_Vol.; 0.681, 0.854, tmp+pH+AN_cumulo+interval_yiel d+interval_Pdt.+RS+Feed_Vol+mu_cumulo+Sugar_consuming_rate_ cumulo+rab_integral+AS_Vol.; 0.681, 0.854, tmp+AN_cumulo+in terval_Pdt.+OD+RS+Feed_Vol+mu_cumulo+Sugar_consuming_rate_c umulo+RQ_integral+AS_Vol.+consum_O2; 0.686, 0.854, tmp+pH+A N_cumulo+interval_Pdt.+RS+Feed_Vol+mu_cumulo+Sugar_consumin g_rate_cumulo+AS_Vol.+generate_CO2; 0.681, 0.854, tmp+AN_cu mulo+interval_yield+interval_Pdt.+OD+RS+Feed_Vol+mu_cumulo+ Sugar_consuming_rate_cumulo+RQ_integral+emission_O2; 0.681, 0.854, tmp+AN_cumulo+interval_Pdt.+RS+Feed_Vol+mu_cumulo+S ugar_consuming_rate_cumulo+rab_integral+RQ_integral+AS_Vol. +generate_CO2; 0.681, 0.854, tmp+PL+interval_yield+RS+Feed_ Vol+mu_cumulo+Sugar_consuming_rate_cumulo+RQ_integral+AS_Vo l.+emission_O2+emission_CO2; 0.681, 0.854, tmp+OD+RS+Feed_V ol+mu_cumulo+nu_per_rho_cumulo+Sugar_consuming_rate_cumulo+ RQ_integral+AS_Vol.+emission_O2+emission_CO2; 0.681, 0.854, tmp+AN+interval_yield+interval_Pdt.+RS+Feed_Vol+mu_cumulo+ Sugar_consuming_rate_cumulo+AS_Vol.+emission_CO2+generate_C O2; 0.681, 0.854, tmp+AN_cumulo+interval_Pdt.+OD+RS+Feed_Vo l+mu_cumulo+nu_per_rho_cumulo+Sugar_consuming_rate_cumulo+R Q_integral+emission_O2; 0.691, 0.854, tmp+AN+interval_yield +RS+Feed_Vol+mu_cumulo+Sugar_consuming_rate_cumulo+rab_inte gral+AS_Vol.; 0.681, 0.854, tmp+AN_cumulo+OD+RS+Feed_Vol+mu _cumulo+Sugar_consuming_rate_cumulo+RQ_integral+AS_Vol.+emi ssion_CO2+generate_CO2; 0.664, 0.854, tmp+PL+interval_Pdt.+ OD+RS+Feed_Vol+mu_cumulo+nu_cumulo+rho_cumulo+Sugar_consumi ng_rate_cumulo+RQ_integral+AS_Vol.+emission_O2+emission_CO 2; 0.664, 0.854, tmp+AN_cumulo+interval_yield+interval_Pdt. +RS+Feed_Vol+mu_cumulo+nu_per_rho_cumulo+Sugar_consuming_ra te_cumulo+RQ_integral+AS_Vol.+emission_O2+emission_CO2+gene rate_CO2; 0.691, 0.854, tmp+interval_Pdt.+OD+RS+Feed_Vol+mu _cumulo+nu_cumulo+Sugar_consuming_rate_cumulo+AS_Vol.; 0.68 6, 0.854, tmp+interval_Pdt.+RS+Feed_Vol+mu_cumulo+nu_per_rh o_cumulo+Sugar_consuming_rate_cumulo+rab_integral+AS_Vol.+e mission_O2; 0.686, 0.854, tmp+PL+AN_cumulo+RS+Feed_Vol+mu_c umulo+Sugar_consuming_rate_cumulo+rab_integral+RQ_integral+ AS_Vol.; 0.691, 0.854, tmp+RS+Feed_Vol+mu_cumulo+Sugar_cons uming_rate_cumulo+rab_integral+RQ_integral+AS_Vol.+emission _CO2; 0.670, 0.854, tmp+AN_cumulo+interval_Pdt.+RS+Feed_Vol +mu_cumulo+nu_cumulo+Sugar_consuming_rate_cumulo+RQ_integra l+AS_Vol.+emission_O2+emission_CO2+generate-CO2; 0.670, 0.8 54, tmp+PL+interval_yield+interval_Pdt.+RS+Feed_Vol+mu_cumu lo+nu_per_rho_cumulo+Sugar_consuming_rate_cumulo+rab_integr al+AS_Vol.+emission_O2+emission_CO2; 0.670, 0.854, tmp+PL+A N+RS+Feed_Vol+mu_cumulo+rho_cumulo+nu_per_rho_cumulo+Sugar_ consuming_rate_cumulo+RQ_integral+AS_Vol.+emission_O2+emiss ion_CO2; 0.681, 0.854, tmp+PL+interval_Pdt.+OD+RS+Feed_Vol+ mu_cumulo+rho_cumulo+Sugar_consuming_rate_cumulo+rab_integr al+AS_Vol.; 0.664, 0.854, tmp+AN_cumulo+interval_yield+inte rval_Pdt.+RS+Feed_Vol+mu_cumulo+rho_cumulo+Sugar_consuming_ rate_cumulo+rab_integral+AS_Vol.+emission_O2+emission_CO2+g enerate_CO2; 0.675, 0.854, tmp+AN+AN_cumulo+interval_Pdt.+O D+RS+Feed_Vol+mu_cumulo+rho_cumulo+Sugar_consuming_rate_cum ulo+AS_Vol.+generate_CO2; 0.681, 0.854, tmp+AN_cumulo+inter val_yield+interval_Pdt.+OD+RS+Feed_Vol+mu_cumulo+rho_cumulo +Sugar_consuming_rate_cumulo+AS_Vol.; 0.681, 0.854, tmp+AN+ AN_cumulo+RS+Feed_Vol+mu_cumulo+nu_per_rho_cumulo+Sugar_con suming_rate_cumulo+AS_Vol.+emission_CO2+generate_CO2; 0.670, 0.854, tmp+AN+AN_cumulo+interval_yield+interval_Pdt.+OD+RS +Feed_Vol+mu_cumulo+Sugar_consuming_rate_cumulo+RQ_integral +AS_Vol.+emission_O2; 0.691, 0.854, tmp+interval_yield+inte rval_Pdt.+RS+Feed_Vol+mu_cumulo+nu_per_rho_cumulo+Sugar_con suming_rate_cumulo+AS_Vol.; 0.670, 0.854, tmp+PL+AN_cumulo+ RS+Feed_Vol+mu_cumulo+rho_cumulo+nu_per_rho_cumulo+Sugar_co nsuming_rate_cumulo+RQ_integral+AS_Vol.+emission_O2+emissio n_CO2; 0.670, 0.854, tmp+AN+AN_cumulo+interval_yield+interv al_Pdt.+RS+Feed_Vol+mu_cumulo+nu_per_rho_cumulo+Sugar_consu ming_rate_cumulo+rab_integral+AS_Vol.+emission_O2; 0.681, 0. 854, tmp+interval_Pdt.+OD+RS+Feed_Vol+mu_cumulo+nu_cumulo+n u_per_rho_cumulo+Sugar_consuming_rate_cumulo+rab_integral+A S_Vol.; 0.664, 0.854, tmp+PL+AN_cumulo+interval_Pdt.+RS+Fee d_Vol+mu_cumulo+nu_per_rho_cumulo+Sugar_consuming_rate_cumu lo+rab_integral+RQ_integral+AS_Vol.+emission_O2+emission_CO 2; 0.686, 0.854, tmp+AN+AN_cumulo+RS+Feed_Vol+mu_cumulo+Sug ar_consuming_rate_cumulo+rab_integral+RQ_integral+AS_Vol.; 0.681, 0.854, tmp+interval_yield+RS+Feed_Vol+mu_cumulo+rho_ cumulo+Sugar_consuming_rate_cumulo+RQ_integral+AS_Vol.+emis sion_O2+emission_CO2; 0.686, 0.854, tmp+AN_cumulo+interval_ yield+interval_Pdt.+RS+Feed_Vol+mu_cumulo+Sugar_consuming_r ate_cumulo+RQ_integral+emission_O2; 0.675, 0.854, tmp+AN+AN _cumulo+interval_yield+interval_Pdt.+RS+Feed_Vol+mu_cumulo+ nu_per_rho_cumulo+Sugar_consuming_rate_cumulo+rab_integral+ emission_O2; 0.675, 0.854, tmp+AN_cumulo+interval_yield+int erval_Pdt.+OD+RS+Feed_Vol+mu_cumulo+rho_cumulo+Sugar_consum ing_rate_cumulo+RQ_integral+AS_Vol.; 0.675, 0.854, tmp+AN_c umulo+interval_Pdt.+RS+Feed_Vol+mu_cumulo+nu_cumulo+Sugar_c onsuming_rate_cumulo+rab_integral+AS_Vol.+emission_O2+emiss ion_CO2; 0.664, 0.854, tmp+AN+interval_Pdt.+OD+RS+Feed_Vol+ mu_cumulo+rho_cumulo+Sugar_consuming_rate_cumulo+RQ_integra l+AS_Vol.+emission_O2+emission_CO2+generate_CO2; 0.681, 0.8 54, tmp+AN_cumulo+RS+Feed_Vol+mu_cumulo+Sugar_consuming_rat e_cumulo+RQ_integral+AS_Vol.+emission_CO2+consum_O2+generat e_CO2; 0.675, 0.854, tmp+AN_cumulo+interval_yield+interval_ Pdt.+OD+RS+Feed_Vol+mu_cumulo+Sugar_consuming_rate_cumulo+A S_Vol.+consum_O2+generate_CO2; 0.681, 0.854, tmp+AN_cumulo+ interval_yield+RS+Feed_Vol+mu_cumulo+nu_per_rho_cumulo+Suga r_consuming_rate_cumulo+AS_Vol.+emission_CO2+generate_CO2; 0.686, 0.854, tmp+AN_cumulo+RS+Feed_Vol+mu_cumulo+nu_cumulo +Sugar_consuming_rate_cumulo+AS_Vol.+emission_CO2+generate_ CO2; 0.675, 0.854, tmp+AN_cumulo+OD+RS+Feed_Vol+mu_cumulo+n u_per_rho_cumulo+Sugar_consuming_rate_cumulo+RQ_integral+AS _Vol.+emission_O2+emission_CO2; 0.686, 0.854, tmp+interval_ Pdt.+OD+RS+Feed_Vol+mu_cumulo+rho_cumulo+Sugar_consuming_ra te_cumulo+AS_Vol.+emission_O2; 0.664, 0.854, tmp+PL+interva l_Pdt.+RS+Feed_Vol+mu_cumulo+nu_per_rho_cumulo+Sugar_consum ing_rate_cumulo+rab_integral+RQ_integral+AS_Vol.+emission_O 2+emission_CO2+generate_CO2; 0.686, 0.854, tmp+AN+RS+Feed_V ol+mu_cumulo+nu_per_rho_cumulo+Sugar_consuming_rate_cumulo+ RQ_integral+AS_Vol.+emission_CO2; 0.670, 0.854, tmp+PL+inte rval_yield+RS+Feed_Vol+mu_cumulo+rho_cumulo+Sugar_consuming _rate_cumulo+rab_integral+RQ_integral+AS_Vol.+emission_O2+e mission_CO2; 0.695, 0.854, tmp+AN_cumulo+interval_yield+RS+ Feed_Vol+mu_cumulo+nu_per_rho_cumulo+AS_Vol.; 0.690, 0.854, tmp+AN+interval_Pdt.+RS+Feed_Vol+mu_cumulo+rho_cumulo+Suga r_consuming_rate_cumulo+AS_Vol.; 0.675, 0.854, tmp+AN+AN_cu mulo+interval_Pdt.+RS+Feed_Vol+mu_cumulo+rho_cumulo+Sugar_c onsuming_rate_cumulo+rab_integral+AS_Vol.+emission_O2; 0.68 0, 0.854, tmp+AN_cumulo+RS+Feed_Vol+mu_cumulo+Sugar_consumi ng_rate_cumulo+rab_integral+RQ_integral+AS_Vol.+emission_CO 2+generate_CO2; 0.680, 0.854, tmp+PL+AN_cumulo+RS+Feed_Vol+ mu_cumulo+Sugar_consuming_rate_cumulo+rab_integral+AS_Vol.+ emission_CO2+generate_CO2; 0.664, 0.854, tmp+PL+AN+interval _Pdt.+OD+RS+Feed_Vol+mu_cumulo+rho_cumulo+Sugar_consuming_r ate_cumulo+rab_integral+AS_Vol.+emission_O2+emission_CO2; 0. 670, 0.854, tmp+pH+AN_cumulo+RS+Feed_Vol+mu_cumulo+nu_per_r ho_cumulo+Sugar_consuming_rate_cumulo+RQ_integral+AS_Vol.+e mission_O2+emission_CO2+generate_CO2; 0.680, 0.854, tmp+AN_ cumulo+interval_yield+RS+Feed_Vol+mu_cumulo+Sugar_consuming _rate_cumulo+rab_integral+AS_Vol.+emission_CO2+generate_CO 2; 0.686, 0.854, tmp+interval_Pdt.+RS+Feed_Vol+mu_cumulo+nu _cumulo+rho_cumulo+Sugar_consuming_rate_cumulo+rab_integral +AS_Vol.; 0.670, 0.854, tmp+PL+AN_cumulo+OD+RS+Feed_Vol+mu_ cumulo+Sugar_consuming_rate_cumulo+RQ_integral+AS_Vol.+emis sion_O2+emission_CO2+generate_CO2; 0.670, 0.854, tmp+PL+AN_ cumulo+interval_yield+RS+Feed_Vol+mu_cumulo+nu_per_rho_cumu lo+Sugar_consuming_rate_cumulo+RQ_integral+AS_Vol.+emission _O2+emission_CO2; 0.664, 0.854, tmp+PL+AN_cumulo+interval_y ield+interval_Pdt.+OD+RS+Feed_Vol+mu_cumulo+nu_cumulo+Sugar _consuming_rate_cumulo+rab_integral+AS_Vol.+emission_O2; 0. 680, 0.854, tmp+AN_cumulo+interval_yield+RS+Feed_Vol+mu_cum ulo+nu_per_rho_cumulo+Sugar_consuming_rate_cumulo+rab_integ ral+RQ_integral+AS_Vol.; 0.675, 0.854, tmp+PL+AN_cumulo+RS+ Feed_Vol+mu_cumulo+nu_cumulo+Sugar_consuming_rate_cumulo+RQ _integral+AS_Vol.+emission_O2+emission_CO2; 0.664, 0.854, t mp+PL+AN_cumulo+interval_Pdt.+RS+Feed_Vol+mu_cumulo+nu_cumu lo+nu_per_rho_cumulo+Sugar_consuming_rate_cumulo+RQ_integra l+AS_Vol.+emission_O2+emission_CO2; 0.685, 0.854, tmp+AN+in terval_Pdt.+RS+Feed_Vol+mu_cumulo+Sugar_consuming_rate_cumu lo+rab_integral+AS_Vol.+emission_O2; 0.680, 0.854, tmp+AN+i nterval_yield+interval_Pdt.+RS+Feed_Vol+mu_cumulo+nu_per_rh o_cumulo+Sugar_consuming_rate_cumulo+rab_integral+AS_Vol.; 0.664, 0.854, tmp+PL+AN_cumulo+interval_yield+interval_Pdt. +RS+Feed_Vol+mu_cumulo+rho_cumulo+Sugar_consuming_rate_cumu lo+rab_integral+AS_Vol.+emission_O2+emission_CO2; 0.690, 0. 854, tmp+AN_cumulo+interval_Pdt.+RS+Feed_Vol+mu_cumulo+Suga r_consuming_rate_cumulo+rab_integral+generate_CO2; 0.675, 0. 854, tmp+pH+AN_cumulo+interval_yield+interval_Pdt.+RS+Feed_ Vol+mu_cumulo+Sugar_consuming_rate_cumulo+rab_integral+AS_V ol.+emission_O2; 0.670, 0.854, tmp+interval_Pdt.+RS+Feed_Vo l+mu_cumulo+rho_cumulo+Sugar_consuming_rate_cumulo+rab_inte gral+RQ_integral+AS_Vol.+emission_O2+emission_CO2+generate_ CO2; 0.664, 0.854, tmp+AN+AN_cumulo+interval_yield+interval _Pdt.+OD+RS+Feed_Vol+mu_cumulo+rho_cumulo+Sugar_consuming_r ate_cumulo+rab_integral+AS_Vol.+emission_O2; 0.670, 0.854, tmp+PL+AN+interval_Pdt.+RS+Feed_Vol+mu_cumulo+Sugar_consumi ng_rate_cumulo+rab_integral+RQ_integral+AS_Vol.+emission_O2 +emission_CO2; 0.664, 0.854, tmp+PL+interval_Pdt.+RS+Feed_V ol+mu_cumulo+rho_cumulo+nu_per_rho_cumulo+Sugar_consuming_r ate_cumulo+rab_integral+RQ_integral+AS_Vol.+emission_O2+emi ssion_CO2; 0.664, 0.854, tmp+AN+interval_yield+interval_Pdt. +OD+RS+Feed_Vol+mu_cumulo+rho_cumulo+Sugar_consuming_rate_c umulo+rab_integral+AS_Vol.+emission_O2+emission_CO2; 0.680, 0.854, tmp+AN_cumulo+interval_Pdt.+RS+Feed_Vol+mu_cumulo+n u_per_rho_cumulo+Sugar_consuming_rate_cumulo+rab_integral+A S_Vol.+consum_O2; 0.680, 0.854, tmp+interval_Pdt.+OD+RS+Fee d_Vol+mu_cumulo+rho_cumulo+Sugar_consuming_rate_cumulo+rab_ integral+AS_Vol.+generate_CO2; 0.685, 0.854, tmp+pH+AN_cumu lo+interval_yield+RS+Feed_Vol+mu_cumulo+Sugar_consuming_rat e_cumulo+rab_integral+AS_Vol.; 0.670, 0.854, tmp+AN+AN_cumu lo+RS+Feed_Vol+mu_cumulo+nu_cumulo+Sugar_consuming_rate_cum ulo+RQ_integral+AS_Vol.+emission_O2+emission_CO2+generate_C 02; 0.685, 0.854, tmp+PL+interval_yield+interval_Pdt.+RS+Fe ed_Vol+mu_cumulo+Sugar_consuming_rate_cumulo+rab_integral+A S_Vol.; 0.658, 0.854, tmp+PL+AN_cumulo+interval_Pdt.+OD+RS+ Feed_Vol+mu_cumulo+nu_per_rho_cumulo+Sugar_consuming_rate_c umulo+RQ_integral+AS_Vol.+emission_O2+emission_CO2+generate _CO2; 0.685, 0.854, tmp+AN+interval_Pdt.+OD+RS+Feed_Vol+mu_ cumulo+nu_per_rho_cumulo+Sugar_consuming_rate_cumulo+AS_Vo l.; 0.685, 0.854, tmp+interval_Pdt.+RS+Feed_Vol+mu_cumulo+S ugar_consuming_rate_cumulo+AS_Vol.+emission_CO2+consum_O2+g enerate_CO2; 0.690, 0.854, tmp+AN+interval_Pdt.+RS+Feed_Vol +mu_cumulo+Sugar_consuming_rate_cumulo+AS_Vol.+generate_CO 2; 0.695, 0.853, tmp+pH+interval_Pdt.+RS+Feed_Vol+mu_cumulo +Sugar_consuming_rate_cumulo+AS_Vol.; 0.670, 0.853, tmp+AN_ cumulo+interval_Pdt.+RS+Feed_Vol+mu_cumulo+nu_per_rho_cumul o+Sugar_consuming_rate_cumulo+rab_integral+RQ_integral+AS_V ol.+emission_O2+emission_CO2; 0.680, 0.853, tmp+PL+AN_cumul o+interval_Pdt.+RS+Feed_Vol+mu_cumulo+nu_per_rho_cumulo+Sug ar_consuming_rate_cumulo+RQ_integral+emission_O2; 0.670, 0. 853, tmp+PL+AN_cumulo+RS+Feed_Vol+mu_cumulo+rho_cumulo+Suga r_consuming_rate_cumulo+rab_integral+RQ_integral+AS_Vol.+em ission_O2+emission_CO2; 0.675, 0.853, tmp+PL+AN+AN_cumulo+i nterval_yield+interval_Pdt.+RS+Feed_Vol+mu_cumulo+Sugar_con suming_rate_cumulo+rab_integral+AS_Vol.; 0.675, 0.853, tmp+ PL+AN_cumulo+interval_Pdt.+RS+Feed_Vol+mu_cumulo+Sugar_cons uming_rate_cumulo+rab_integral+AS_Vol.+emission_O2+generate _CO2; 0.670, 0.853, tmp+AN+RS+Feed_Vol+mu_cumulo+nu_cumulo+ nu_per_rho_cumulo+Sugar_consuming_rate_cumulo+RQ_integral+A S_Vol.+emission_O2+emission_CO2+generate_CO2; 0.675, 0.853, tmp+PL+AN_cumulo+interval_Pdt.+RS+Feed_Vol+mu_cumulo+nu_cu mulo+Sugar_consuming_rate_cumulo+rab_integral+AS_Vol.+emiss ion_O2; 0.680, 0.853, tmp+AN_cumulo+OD+RS+Feed_Vol+mu_cumul o+rho_cumulo+Sugar_consuming_rate_cumulo+RQ_integral+AS_Vol. +emission_CO2; 0.670, 0.853, tmp+AN_cumulo+RS+Feed_Vol+mu_c umulo+rho_cumulo+Sugar_consuming_rate_cumulo+rab_integral+R Q_integral+AS_Vol.+emission_O2+emission_CO2+generate_CO2; 0. 675, 0.853, tmp+PL+AN_cumulo+interval_Pdt.+RS+Feed_Vol+mu_c umulo+nu_per_rho_cumulo+Sugar_consuming_rate_cumulo+rab_int egral+AS_Vol.+generate_CO2; 0.670, 0.853, tmp+PL+interval_P dt.+RS+Feed_Vol+mu_cumulo+nu_cumulo+Sugar_consuming_rate_cu mulo+RQ_integral+AS_Vol.+emission_O2+emission_CO2+generate_ CO2; 0.695, 0.853, tmp+interval_yield+RS+Feed_Vol+mu_cumulo +rho_cumulo+Sugar_consuming_rate_cumulo+AS_Vol.; 0.680, 0.8 53, tmp+AN+AN_cumulo+interval_Pdt.+RS+Feed_Vol+mu_cumulo+Su gar_consuming_rate_cumulo+rab_integral+AS_Vol.+emission_CO 2; 0.670, 0.853, tmp+PL+AN_cumulo+interval_yield+interval_P dt.+RS+Feed_Vol+mu_cumulo+Sugar_consuming_rate_cumulo+rab_i ntegral+AS_Vol.+emission_O2+generate_CO2; 0.664, 0.853, tmp +PL+AN+interval_yield+interval_Pdt.+RS+Feed_Vol+mu_cumulo+r ho_cumulo+Sugar_consuming_rate_cumulo+rab_integral+AS_Vol.+ emission_O2+emission_CO2; 0.680, 0.853, tmp+AN+AN_cumulo+in terval_Pdt.+RS+Feed_Vol+mu_cumulo+nu_cumulo+Sugar_consuming _rate_cumulo+rab_integral+AS_Vol.; 0.690, 0.853, tmp+interv al_yield+interval_Pdt.+OD+RS+Feed_Vol+mu_cumulo+Sugar_consu ming_rate_cumulo+AS_Vol.; 0.670, 0.853, tmp+PL+interval_yie ld+interval_Pdt.+RS+Feed_Vol+mu_cumulo+Sugar_consuming_rate _cumulo+rab_integral+AS_Vol.+emission_O2+emission_CO2+gener ate_CO2; 0.670, 0.853, tmp+interval_yield+interval_Pdt.+RS+ Feed_Vol+mu_cumulo+Sugar_consuming_rate_cumulo+rab_integral +RQ_integral+AS_Vol.+emission_O2+emission_CO2+generate_CO2; 0.675, 0.853, tmp+PL+AN+interval_Pdt.+RS+Feed_Vol+mu_cumul o+Sugar_consuming_rate_cumulo+rab_integral+AS_Vol.+emission _O2+emission_CO2; 0.690, 0.853, tmp+AN+interval_Pdt.+RS+Fee d_Vol+mu_cumulo+Sugar_consuming_rate_cumulo+RQ_integral+AS_ Vol.; 0.664, 0.853, tmp+PL+interval_yield+interval_Pdt.+RS+ Feed_Vol+mu_cumulo+rho_cumulo+Sugar_consuming_rate_cumulo+r ab_integral+RQ_integral+AS_Vol.+emission_O2+emission_CO2; 0. 675, 0.853, tmp+AN+interval_yield+RS+Feed_Vol+mu_cumulo+nu_ per_rho_cumulo+Sugar_consuming_rate_cumulo+RQ_integral+AS_V ol.+emission_O2+emission_CO2; 0.700, 0.853, tmp+AN_cumulo+i nterval_Pdt.+RS+Feed_Vol+mu_cumulo+AS_Vol.; 0.675, 0.853, t mp+AN+AN_cumulo+interval_Pdt.+OD+RS+Feed_Vol+mu_cumulo+nu_p er_rho_cumulo+Sugar_consuming_rate_cumulo+RQ_integral+AS_Vo l.; 0.670, 0.853, tmp+PL+interval_yield+RS+Feed_Vol+mu_cumu lo+nu_cumulo+rho_cumulo+Sugar_consuming_rate_cumulo+RQ_inte gral+AS_Vol.+emission_O2+emission_CO2; 0.685, 0.853, tmp+AN +interval_yield+RS+Feed_Vol+mu_cumulo+nu_per_rho_cumulo+Sug ar_consuming_rate_cumulo+rab_integral+AS_Vol.; 0.675, 0.853, tmp+AN_cumulo+interval_Pdt.+RS+Feed_Vol+mu_cumulo+nu_per_r ho_cumulo+Sugar_consuming_rate_cumulo+rab_integral+AS_Vol.+ emission_CO2+generate_CO2; 0.690, 0.853, tmp+PL+RS+Feed_Vol +mu_cumulo+Sugar_consuming_rate_cumulo+rab_integral+AS_Vol. +emission_CO2; 0.680, 0.853, tmp+RS+Feed_Vol+mu_cumulo+nu_p er_rho_cumulo+Sugar_consuming_rate_cumulo+RQ_integral+AS_Vo l.+emission_CO2+consum_O2+generate_CO2; 0.675, 0.853, tmp+A N+RS+Feed_Vol+mu_cumulo+nu_per_rho_cumulo+Sugar_consuming_r ate_cumulo+rab_integral+RQ_integral+AS_Vol.+emission_O2+emi ssion_CO2; 0.685, 0.853, tmp+AN_cumulo+interval_Pdt.+OD+RS+ Feed_Vol+mu_cumulo+Sugar_consuming_rate_cumulo+AS_Vol.+emis sion_CO2; 0.695, 0.853, tmp+pH+AN_cumulo+RS+Feed_Vol+mu_cum ulo+Sugar_consuming_rate_cumulo+AS_Vol.; 0.680, 0.853, tmp+ AN_cumulo+interval_yield+interval_Pdt.+RS+Feed_Vol+mu_cumul o+nu_per_rho_cumulo+Sugar_consuming_rate_cumulo+RQ_integral +AS_Vol.; 0.680, 0.853, tmp+AN_cumulo+RS+Feed_Vol+mu_cumulo +rho_cumulo+nu_per_rho_cumulo+Sugar_consuming_rate_cumulo+r ab_integral+AS_Vol.+emission_CO2; 0.685, 0.853, tmp+PL+inte rval_Pdt.+RS+Feed_Vol+mu_cumulo+rho_cumulo+Sugar_consuming_ _rate_cumulo+rab_integral+AS_Vol.; 0.669, 0.853, tmp+interva l_yield+interval_Pdt.+RS+Feed_Vol+mu_cumulo+nu_per_rho_cumu lo+Sugar_consuming_rate_cumulo+rab_integral+RQ_integral+AS_ Vol.+emission_O2+emission_CO2; 0.680, 0.853, tmp+interval_P dt.+RS+Feed_Vol+mu_cumulo+rho_cumulo+nu_per_rho_cumulo+Suga r_consuming_rate_cumulo+AS_Vol.+emission_CO2+generate_CO2; 0.669, 0.853, tmp+AN+AN_cumulo+interval_Pdt.+RS+Feed_Vol+mu _cumulo+nu_per_rho_cumulo+Sugar_consuming_rate_cumulo+rab_i ntegral+AS_Vol.+emission_O2+emission_CO2; 0.680, 0.853, tmp +PL+OD+RS+Feed_Vol+mu_cumulo+Sugar_consuming_rate_cumulo+RQ _integral+AS_Vol.+emission_O2+emission_CO2; 0.680, 0.853, t mp+AN+AN_cumulo+interval_Pdt.+RS+Feed_Vol+mu_cumulo+Sugar_c onsuming_rate_cumulo+AS_Vol.+emission_CO2+generate_CO2; 0.6 69, 0.853, tmp+AN_cumulo+interval_Pdt.+RS+Feed_Vol+mu_cumul o+nu_cumulo+rho_cumulo+Sugar_consuming_rate_cumulo+rab_inte gral+AS_Vol.+emission_O2+emission_CO2; 0.685, 0.853, tmp+AN _cumulo+interval_Pdt.+RS+Feed_Vol+mu_cumulo+rho_cumulo+nu_p er_rho_cumulo+Sugar_consuming_rate_cumulo+AS_Vol.; 0.669, 0. 853, tmp+AN+AN_cumulo+interval_Pdt.+RS+Feed_Vol+mu_cumulo+S ugar_consuming_rate_cumulo+RQ_integral+AS_Vol.+emission_O2+ emission_CO2+generate_CO2; 0.685, 0.853, tmp+PL+AN_cumulo+R S+Feed_Vol+mu_cumulo+nu_per_rho_cumulo+Sugar_consuming_rate _cumulo+RQ_integral+AS_Vol.; 0.680, 0.853, tmp+interval_yie ld+interval_Pdt.+RS+Feed_Vol+mu_cumulo+nu_per_rho_cumulo+Su gar_consuming_rate_cumulo+AS_Vol.+emission_CO2+generate_CO 2; 0.669, 0.853, tmp+PL+AN+interval_Pdt.+RS+Feed_Vol+mu_cum ulo+rho_cumulo+Sugar_consuming_rate_cumulo+rab_integral+RQ_ integral+emission_O2+emission_CO2; 0.685, 0.853, tmp+AN_cum ulo+RS+Feed_Vol+mu_cumulo+nu_cumulo+nu_per_rho_cumulo+Sugar _consuming_rate_cumulo+rab_integral+AS_Vol.; 0.685, 0.853, tmp+AN_cumulo+interval_yield+RS+Feed_Vol+mu_cumulo+rho_cumu lo+Sugar_consuming_rate_cumulo+RQ_integral+AS_Vol.; 0.690, 0.853, tmp+AN+interval_yield+interval_Pdt.+RS+Feed_Vol+mu_c umulo+Sugar_consuming_rate_cumulo+AS_Vol.; 0.675, 0.853, tm p+AN_cumulo+interval_Pdt.+RS+Feed_Vol+mu_cumulo+rho_cumulo+ Sugar_consuming_rate_cumulo+rab_integral+AS_Vol.+emission_O 2+generate_CO2; 0.690, 0.853, tmp+PL+interval_yield+RS+Feed _Vol+mu_cumulo+nu_per_rho_cumulo+Sugar_consuming_rate_cumul o+AS_Vol.; 0.680, 0.853, tmp+AN_cumulo+interval_Pdt.+RS+Fee d_Vol+mu_cumulo+nu_cumulo+Sugar_consuming_rate_cumulo+AS_Vo l.+emission_CO2+generate_CO2; 0.664, 0.853, tmp+PL+AN+AN_cu mulo+RS+Feed_Vol+mu_cumulo+nu_per_rho_cumulo+Sugar_consumin g_rate_cumulo+RQ_integral+AS_Vol.+emission_O2+emission_CO2+ generate_CO2; 0.680, 0.853, tmp+pH+AN_cumulo+RS+Feed_Vol+mu _cumulo+Sugar_consuming_rate_cumulo+RQ_integral+AS_Vol.+emi ssion_CO2+generate_CO2; 0.685, 0.853, tmp+AN_cumulo+interva l_yield+RS+Feed_Vol+mu_cumulo+Sugar_consuming_rate_cumulo+r ab_integral+AS_Vol.+emission_O2; 0.680, 0.853, tmp+AN_cumul o+interval_Pdt.+RS+Feed_Vol+mu_cumulo+nu_cumulo+Sugar_consu ming_rate_cumulo+rab_integral+AS_Vol.+generate_CO2; 0.680, 0.853, tmp+AN_cumulo+interval_yield+RS+Feed_Vol+mu_cumulo+n u_per_rho_cumulo+Sugar_consuming_rate_cumulo+RQ_integral+AS _Vol.+emission_CO2; 0.680, 0.853, tmp+AN+AN_cumulo+RS+Feed_ Vol+mu_cumulo+rho_cumulo+Sugar_consuming_rate_cumulo+rab_in tegral+AS_Vol.+emission_CO2; 0.669, 0.853, tmp+PL+AN_cumulo +interval_yield+interval_Pdt.+RS+Feed_Vol+mu_cumulo+nu_cumu lo+Sugar_consuming_rate_cumulo+rab_integral+AS_Vol.+emissio n_O2; 0.690, 0.853, tmp+AN_cumulo+RS+Feed_Vol+mu_cumulo+Sug ar_consuming_rate_cumulo+rab_integral+AS_Vol.+consum_O2; 0. 695, 0.853, tmp+AN_cumulo+interval_yield+RS+Feed_Vol+mu_cum ulo+Sugar_consuming_rate_cumulo+RQ_integral; 0.675, 0.853, tmp+PL+RS+Feed_Vol+mu_cumulo+nu_per_rho_cumulo+Sugar_consum ing_rate_cumulo+rab_integral+RQ_integral+AS_Vol.+emission_O 2+emission_CO2; 0.685, 0.853, tmp+RS+Feed_Vol+mu_cumulo+nu_ cumulo+Sugar_consuming_rate_cumulo+rab_integral+AS_Vol.+emi ssion_CO2+generate_CO2; 0.695, 0.853, tmp+interval_Pdt.+OD+ RS+Feed_Vol+mu_cumulo+nu_per_rho_cumulo+AS_Vol.; 0.680, 0.8 53, tmp+OD+RS+Feed_Vol+mu_cumulo+nu_per_rho_cumulo+Sugar_co nsuming_rate_cumulo+RQ_integral+AS_Vol.+emission_CO2+consum _O2; 0.664, 0.853, tmp+PL+AN_cumulo+RS+Feed_Vol+mu_cumulo+r ho_cumulo+nu_per_rho_cumulo+Sugar_consuming_rate_cumulo+RQ_ integral+AS_Vol.+emission_O2+emission_CO2+generate_CO2; 0.6 69, 0.853, tmp+AN+interval_Pdt.+RS+Feed_Vol+mu_cumulo+nu_cu mulo+rho_cumulo+Sugar_consuming_rate_cumulo+rab_integral+AS _Vol.+emission_O2+emission_CO2; 0.690, 0.853, tmp+AN_cumulo +interval_Pdt.+OD+RS+Feed_Vol+mu_cumulo+nu_per_rho_cumulo+A S_Vol.; 0.680, 0.853, tmp+AN_cumulo+interval_Pdt.+OD+RS+Fee d_Vol+mu_cumulo+Sugar_consuming_rate_cumulo+AS_Vol.+consum_ O2+generate_CO2; 0.685, 0.853, tmp+interval_yield+interval_ Pdt.+OD+RS+Feed_Vol+mu_cumulo+Sugar_consuming_rate_cumulo+r ab_integral+AS_Vol.; 0.704, 0.853, tmp+interval_Pdt.+RS+Fee d_Vol+mu_cumulo+AS_Vol.; 0.680, 0.853, tmp+PL+AN_cumulo+int erval_Pdt.+RS+Feed_Vol+mu_cumulo+nu_cumulo+Sugar_consuming_ _rate_cumulo+rab_integral+AS_Vol.; 0.685, 0.853, tmp+AN_cumu lo+OD+RS+Feed_Vol+mu_cumulo+nu_per_rho_cumulo+Sugar_consumi ng_rate_cumulo+RQ_integral+AS_Vol.; 0.669, 0.853, tmp+PL+OD +RS+Feed_Vol+mu_cumulo+nu_per_rho_cumulo+Sugar_consuming_ra te_cumulo+RQ_integral+AS_Vol.+emission_O2+emission_CO2+gene rate_CO2; 0.669, 0.853, tmp+PL+interval_yield+RS+Feed_Vol+m u_cumulo+nu_cumulo+nu_per_rho_cumulo+Sugar_consuming_rate_c umulo+RQ_integral+AS_Vol.+emission_O2+emission_CO2; 0.685, 0.853, tmp+AN_cumulo+RS+Feed_Vol+mu_cumulo+Sugar_consuming_ rate_cumulo+RQ_integral+AS_Vol.+emission_CO2+consum_O2; 0.6 75, 0.853, tmp+PL+AN_cumulo+interval_Pdt.+OD+RS+Feed_Vol+mu _cumulo+rho_cumulo+Sugar_consuming_rate_cumulo+RQ_integral+ emission_O2; 0.669, 0.853, tmp+PL+RS+Feed_Vol+mu_cumulo+rho _cumulo+Sugar_consuming_rate_cumulo+rab_integral+RQ_integra l+AS_Vol.+emission_O2+emission_CO2+generate_CO2; 0.699, 0.8 53, tmp+interval_Pdt.+RS+Feed_Vol+mu_cumulo+nu_per_rho_cumu lo+AS_Vol.; 0.675, 0.853, tmp+PL+interval_Pdt.+OD+RS+Feed_V ol+mu_cumulo+rho_cumulo+Sugar_consuming_rate_cumulo+rab_int egral+AS_Vol.+emission_O2; 0.685, 0.853, tmp+PL+RS+Feed_Vol +mu_cumulo+nu_per_rho_cumulo+Sugar_consuming_rate_cumulo+AS _Vol.+emission_CO2+generate_CO2; 0.685, 0.853, tmp+pH+RS+Fe ed_Vol+mu_cumulo+rho_cumulo+Sugar_consuming_rate_cumulo+AS_ Vol.+emission_CO2+generate_CO2; 0.680, 0.853, tmp+AN_cumulo +OD+RS+Feed_Vol+mu_cumulo+rho_cumulo+Sugar_consuming_rate_c umulo+RQ_integral+AS_Vol.+consum_O2; 0.685, 0.853, tmp+inte rval_Pdt.+OD+RS+Feed_Vol+mu_cumulo+nu_cumulo+Sugar_consumin g_rate_cumulo+rab_integral+AS_Vol.; 0.685, 0.853, tmp+AN+in terval_yield+RS+Feed_Vol+mu_cumulo+rho_cumulo+Sugar_consumi ng_rate_cumulo+rab_integral+AS_Vol.; 0.690, 0.853, tmp+inte rval_Pdt.+RS+Feed_Vol+mu_cumulo+Sugar_consuming_rate_cumulo +rab_integral+AS_Vol.+consum_O2; 0.669, 0.853, tmp+AN_cumul o+interval_yield+interval_Pdt.+OD+RS+Feed_Vol+mu_cumulo+Sug ar_consuming_rate_cumulo+rab_integral+AS_Vol.+emission_O2+g enerate_CO2; 0.675, 0.853, tmp+AN_cumulo+RS+Feed_Vol+mu_cum ulo+rho_cumulo+nu_per_rho_cumulo+Sugar_consuming_rate_cumul o+RQ_integral+AS_Vol.+emission_CO2+generate_CO2; 0.669, 0.8 53, tmp+AN+interval_Pdt.+OD+RS+Feed_Vol+mu_cumulo+Sugar_con suming_rate_cumulo+RQ_integral+AS_Vol.+emission_O2+emission _CO2+generate_CO2; 0.680, 0.853, tmp+pH+AN_cumulo+interval_ Pdt.+RS+Feed_Vol+mu_cumulo+rho_cumulo+Sugar_consuming_rate cumulo+rab_integral+AS_Vol.; 0.680, 0.853, tmp+AN_cumulo+in terval_Pdt.+RS+Feed_Vol+mu_cumulo+rho_cumulo+nu_per_rho_cum ulo+Sugar_consuming_rate_cumulo+RQ_integral+AS_Vol.; 0.690, 0.853, tmp+interval_Pdt.+OD+RS+Feed_Vol+mu_cumulo+Sugar_co nsuming_rate_cumulo+AS_Vol.+generate_CO2; 0.675, 0.853, tmp +AN+OD+RS+Feed_Vol+mu_cumulo+rho_cumulo+Sugar_consuming_rat e_cumulo+RQ_integral+AS_Vol.+emission_CO2+consum_O2; 0.680, 0.853, tmp+AN+interval_yield+RS+Feed_Vol+mu_cumulo+nu_per_ rho_cumulo+Sugar_consuming_rate_cumulo+AS_Vol.+emission_CO2 +generate_CO2; 0.680, 0.853, tmp+AN+interval_Pdt.+RS+Feed_V ol+mu_cumulo+Sugar_consuming_rate_cumulo+RQ_integral+emissi on_O2+emission_CO2+generate_CO2; 0.685, 0.853, tmp+interval _yield+OD+RS+Feed_Vol+mu_cumulo+rho_cumulo+Sugar_consuming_ rate_cumulo+rab_integral+AS_Vol.; 0.685, 0.853, tmp+pH+AN+A N_cumulo+RS+Feed_Vol+mu_cumulo+Sugar_consuming_rate_cumulo+ rab_integral+AS_Vol.; 0.685, 0.853, tmp+interval_Pdt.+RS+Fe ed_Vol+mu_cumulo+nu_per_rho_cumulo+Sugar_consuming_rate_cum ulo+rab_integral+AS_Vol.+generate_CO2; 0.680, 0.853, tmp+PL +AN+interval_Pdt.+RS+Feed_Vol+mu_cumulo+Sugar_consuming_rat e_cumulo+RQ_integral+emission_O2+emission_CO2; 0.669, 0.853, tmp+pH+AN_cumulo+RS+Feed_Vol+mu_cumulo+rho_cumulo+Sugar_co nsuming_rate_cumulo+RQ_integral+AS_Vol.+emission_O2+emissio n_CO2+generate_CO2; 0.685, 0.853, tmp+RS+Feed_Vol+mu_cumulo +nu_cumulo+nu_per_rho_cumulo+Sugar_consuming_rate_cumulo+AS _Vol.+emission_CO2+generate_CO2; 0.669, 0.853, tmp+pH+inter val_Pdt.+RS+Feed_Vol+mu_cumulo+rho_cumulo+Sugar_consuming_r ate_cumulo+RQ_integral+AS_Vol.+emission_O2+emission_CO2+gen erate_CO2; 0.690, 0.853, tmp+interval_yield+RS+Feed_Vol+mu_ cumulo+nu_per_rho_cumulo+Sugar_consuming_rate_cumulo+AS_Vol. +emission_CO2; 0.669, 0.853, tmp+pH+AN+interval_Pdt.+RS+Fee d_Vol+mu_cumulo+rho_cumulo+Sugar_consuming_rate_cumulo+rab_ integral+AS_Vol.+emission_O2+emission_CO2; 0.680, 0.853, tm p+interval_yield+interval_Pdt.+RS+Feed_Vol+mu_cumulo+Sugar_ consuming_rate_cumulo+RQ_integral+AS_Vol.+emission_O2+emiss ion_CO2; 0.669, 0.853, tmp+AN+OD+RS+Feed_Vol+mu_cumulo+nu_p er_rho_cumulo+Sugar_consuming_rate_cumulo+RQ_integral+AS_Vo l.+emission_O2+emission_CO2+generate_CO2; 0.675, 0.853, tmp +pH+RS+Feed_Vol+mu_cumulo+nu_per_rho_cumulo+Sugar_consuming _rate_cumulo+RQ_integral+AS_Vol.+emission_O2+emission_CO2+g enerate_CO2; 0.669, 0.853, tmp+PL+interval_Pdt.+RS+Feed_Vol +mu_cumulo+rho_cumulo+Sugar_consuming_rate_cumulo+rab_integ ral+AS_Vol.+emission_O2+emission_CO2+generate_CO2; 0.680, 0. 853, tmp+AN+interval_Pdt.+OD+RS+Feed_Vol+mu_cumulo+nu_per_r ho_cumulo+Sugar_consuming_rate_cumulo+AS_Vol.+consum_O2; 0. 680, 0.853, tmp+interval_Pdt.+OD+RS+Feed_Vol+mu_cumulo+rho_ cumulo+nu_per_rho_cumulo+Sugar_consuming_rate_cumulo+rab_in tegral+AS_Vol.; 0.690, 0.853, tmp+AN_cumulo+OD+RS+Feed_Vol+ mu_cumulo+Sugar_consuming_rate_cumulo+rab_integral+AS_Vol.; 0.658, 0.853, tmp+PL+AN+AN_cumulo+interval_Pdt.+OD+RS+Feed _Vol+mu_cumulo+rho_cumulo+Sugar_consuming_rate_cumulo+RQ_in tegral+AS_Vol.+emission_O2+emission_CO2; 0.669, 0.853, tmp+ AN+interval_yield+interval_Pdt.+RS+Feed_Vol+mu_cumulo+nu_cu mulo+Sugar_consuming_rate_cumulo+rab_integral+AS_Vol.+emiss ion_O2+emission_CO2; 0.690, 0.853, tmp+interval_Pdt.+RS+Fee d_Vol+mu_cumulo+nu_per_rho_cumulo+Sugar_consuming_rate_cumu lo+AS_Vol.+generate_CO2

### [204. Linear Model that Predicts Arginine Product ion Amount in Interval 4]

0.386, 0.697, tmp_cumulo+PL_cumulo+AN_cumulo+interval_yield +interval_Pdt.+Feed_Vol+RS_cumulo+rab_integral+RQ_integral+ AS_Vol.+emission_CO2+interval_O2; 0.405, 0.697, tmp_cumulo+ PL_cumulo+AN_cumulo+interval_yield+Feed_Vol+RS_cumulo+rab_i ntegral+RQ_integral+AS_Vol.+emission_CO2; 0.394, 0.696, tmp _cumulo+PL_cumulo+AN_cumulo+interval_yield+Feed_Vol+RS_cumu lo+rab_integral+RQ_integral+AS_Vol.+emission_CO2+interval_O 2; 0.413, 0.696, tmp_cumulo+AN_cumulo+interval_yield+Feed_V ol+RS_cumulo+rab_integral+RQ_integral+AS_Vol.+emission_CO2; 0.403, 0.695, PL_cumulo+AN_cumulo+interval_yield+interval_ Pdt.+RS_cumulo+rab_integral+RQ_integral+AS_Vol.+emission_CO 2+interval_O2; 0.383, 0.695, tmp_cumulo+PL_cumulo+AN_cumulo +interval_yield+interval_Pdt.+Feed_Vol+RS_cumulo+rab_integr al+RQ_integral+AS_Vol.+emission_CO2+consum_O2; 0.392, 0.695, tmp_cumulo+PL_cumulo+AN_cumulo+interval_yield+interval_Pdt. +Feed_Vol+RS_cumulo+rab_integral+RQ_integral+AS_Vol.+emissi on_CO2; 0.410, 0.694, PL_cumulo+AN_cumulo+interval_yield+in terval_Pdt.+RS_cumulo+rab_integral+RQ_integral+AS_Vol.+emis sion_CO2; 0.390, 0.693, tmp_cumulo+PL_cumulo+AN_cumulo+inte rval_yield+interval_Pdt.+RS_cumulo+rab_integral+RQ_integral +AS_Vol.+emission_CO2+interval_O2; 0.408, 0.693, tmp_cumulo +PL_cumulo+interval_yield+Feed_Vol+RS_cumulo+rab_integral+R Q_integral+AS_Vol.+emission_CO2; 0.399, 0.693, tmp_cumulo+A N_cumulo+interval_yield+interval_Pdt.+Feed_Vol+RS_cumulo+ra b_integral+RQ_integral+AS_Vol.+emission_CO2; 0.399, 0.693, tmp_cumulo+AN_cumulo+interval_yield+Feed_Vol+RS_cumulo+rab_ integral+RQ_integral+AS_Vol.+emission_CO2+interval_O2; 0.39 9, 0.693, tmp_cumulo+AN_cumulo+interval_yield+RS+Feed_Vol+R S_cumulo+rab_integral+RQ_integral+AS_Vol.+emission_CO2; 0.3 89, 0.693, tmp_cumulo+PL+PL_cumulo+AN_cumulo+interval_yield +Feed_Vol+RS_cumulo+rab_integral+RQ_integral+AS_Vol.+emissi on_CO2; 0.379, 0.692, tmp_cumulo+AN+AN_cumulo+interval_yiel d+RS+Feed_Vol+RS_cumulo+rab_integral+RQ_integral+AS_Vol.+em ission_CO2+consum_O2; 0.389, 0.692, tmp_cumulo+PL_cumulo+AN _cumulo+interval_yield+Feed_Vol+RS_cumulo+rab_integral+RQ_i ntegral+AS_Vol.+emission_CO2+consum_O2; 0.398, 0.692, O2+PL _cumulo+AN_cumulo+interval_yield+interval_Pdt.+RS_cumulo+ra b_integral+RQ_integral+AS_Vol.+emission_CO2; 0.407, 0.692, tmp_cumulo+PL_cumulo+interval_yield+interval_Pdt.+RS_cumulo +rab_integral+RQ_integral+AS_Vol.+emission_CO2; 0.397, 0.69 2, tmp_cumulo+PL_cumulo+AN_cumulo+interval_yield+interval_P dt.+RS_cumulo+rab_integral+RQ_integral+AS_Vol.+emission_CO 2; 0.397, 0.692, tmp_cumulo+AN+AN_cumulo+interval_yield+Fee d_Vol+RS_cumulo+rab_integral+RQ_integral+AS_Vol.+emission_C 02; 0.387, 0.691, tmp_cumulo+AN_cumulo+interval_yield+inter val_Pdt.+Feed_Vol+RS_cumulo+rab_integral+RQ_integral+AS_Vol. +emission_CO2+interval_O2; 0.387, 0.691, tmp_cumulo+AN_cumu lo+interval_yield+interval_Pdt.+Feed_Vol+RS_cumulo+rab_inte gral+RQ_integral+AS_Vol.+emission_CO2+consum_O2; 0.367, 0.6 91, tmp_cumulo+PL_cumulo+AN_cumulo+interval_yield+interval_ Pdt.+Feed_Vol+RS_cumulo+rab_integral+RQ_integral+AS_Vol.+em ission_CO2+consum_O2+interval_O2; 0.377, 0.691, tmp_cumulo+ PL_cumulo+AN+AN_cumulo+interval_yield+Feed_Vol+RS_cumulo+ra b_integral+RQ_integral+AS_Vol.+emission_CO2+interval_O2; 0. 387, 0.691, tmp_cumulo+AN_cumulo+interval_yield+RS+Feed_Vol +RS_cumulo+rab_integral+RQ_integral+AS_Vol.+emission_CO2+co nsum_O2; 0.396, 0.691, tmp_cumulo+PL_cumulo+interval_yield+ interval_Pdt.+Feed_Vol+RS_cumulo+rab_integral+RQ_integral+A S_Vol.+emission_CO2; 0.396, 0.691, tmp_cumulo+AN_cumulo+int erval_yield+RS+Feed_Vol+RS_cumulo+rab_integral+RQ_integral+ AS_Vol.+emission_O2; 0.376, 0.691, tmp_cumulo+PL_cumulo+AN+ AN_cumulo+interval_yield+Feed_Vol+RS_cumulo+rab_integral+RQ _integral+AS_Vol.+emission_CO2+consum_O2; 0.386, 0.691, tmp _cumulo+PL_cumulo+AN+AN_cumulo+interval_yield+Feed_Vol+RS_c umulo+rab_integral+RQ_integral+AS_Vol.+emission_CO2; 0.386, 0.691, tmp_cumulo+PL_cumulo+AN_cumulo+interval_yield+RS+Fe ed_Vol+RS_cumulo+rab_integral+RQ_integral+AS_Vol.+emission_ CO2; 0.395, 0.691, tmp_cumulo+AN_cumulo+interval_yield+Feed _Vol+RS_cumulo+rab_integral+RQ_integral+AS_Vol.+emission_CO 2+consum_O2; 0.386, 0.691, tmp_cumulo+AN+AN_cumulo+interval _yield+Feed_Vol+RS_cumulo+rab_integral+RQ_integral+AS_Vol.+ emission_CO2+consum_O2; 0.386, 0.691, tmp_cumulo+pH+PL_cumu lo+AN_cumulo+interval_yield+Feed_Vol+RS_cumulo+rab_integral +RQ_integral+AS_Vol.+emission_CO2; 0.376, 0.691, tmp_cumulo +PL_cumulo+AN_cumulo+interval_yield+RS+Feed_Vol+RS_cumulo+r ab_integral+RQ_integral+AS_Vol.+emission_CO2+interval_O2; 0. 385, 0.690, tmp_cumulo+AN_cumulo+interval_yield+RS+Feed_Vol +RS_cumulo+rab_integral+RQ_integral+AS_Vol.+emission_CO2+in terval_O2; 0.375, 0.690, tmp_cumulo+pH+PL_cumulo+AN_cumulo+ interval_yield+Feed_Vol+RS_cumulo+rab_integral+RQ_integral+ AS_Vol.+emission_CO2+interval_O2; 0.385, 0.690, tmp_cumulo+ PL_cumulo+AN_cumulo+interval_yield+RS+Feed_Vol+RS_cumulo+ra b_integral+RQ_integral+AS_Vol.+emission_O2; 0.375, 0.690, t mp_cumulo+PL_cumulo+AN_cumulo+interval_yield+RS+Feed_Vol+RS _cumulo+rab_integral+RQ_integral+AS_Vol.+emission_CO2+consu m_O2; 0.365, 0.690, tmp_cumulo+PL_cumulo+AN+AN_cumulo+inter val_yield+RS+Feed_Vol+RS_cumulo+rab_integral+RQ_integral+AS _Vol.+emission_CO2+consum_O2; 0.375, 0.690, tmp_cumulo+PL+P L_cumulo+AN_cumulo+interval_yield+interval_Pdt.+Feed_Vol+RS _cumulo+rab_integral+RQ_integral+AS_Vol.+emission_CO2; 0.38 4, 0.690, tmp_cumulo+PL_cumulo+AN_cumulo+interval_yield+int erval_Pdt.+Feed_Vol+RS_cumulo+rab_integral+RQ_integral+AS_V ol.+emission_O2; 0.364, 0.690, tmp_cumulo+pH+PL_cumulo+AN_c umulo+interval_yield+interval_Pdt.+Feed_Vol+RS_cumulo+rab_i ntegral+RQ_integral+AS_Vol.+emission_CO2+interval_O2; 0.394, 0.690, tmp_cumulo+pH+AN_cumulo+interval_yield+Feed_Vol+RS_ cumulo+rab_integral+RQ_integral+AS_Vol.+emission_CO2; 0.384, 0.690, tmp_cumulo+AN+AN_cumulo+interval_yield+Feed_Vol+RS_ cumulo+rab_integral+RQ_integral+AS_Vol.+emission_CO2+interv al_O2; 0.364, 0.689, tmp_cumulo+PL_cumulo+AN+AN_cumulo+inte rval_yield+interval_Pdt.+Feed_Vol+RS_cumulo+rab_integral+RQ _integral+AS_Vol.+emission_CO2+consum_O2; 0.393, 0.689, PL_ cumulo+AN_cumulo+interval_yield+interval_Pdt.+RS_cumulo+rab _integral+RQ_integral+AS_Vol.+emission_CO2+consum_O2; 0.383, 0.689, tmp+PL_cumulo+AN_cumulo+interval_yield+interval_Pdt. +RS_cumulo+rab_integral+RQ_integral+AS_Vol.+emission_CO2+in terval_O2; 0.373, 0.689, tmp_cumulo+PL_cumulo+AN+AN_cumulo+ interval_yield+interval_Pdt.+RS_cumulo+rab_integral+RQ_inte gral+AS_Vol.+emission_CO2+interval_O2; 0.383, 0.689, PL_cum ulo+AN+AN_cumulo+interval_yield+interval_Pdt.+RS_cumulo+rab _integral+RQ_integral+AS_Vol.+emission_CO2+interval_O2; 0.3 63, 0.689, tmp_cumulo+PL_cumulo+AN+AN_cumulo+interval_yield +interval_Pdt.+Feed_Vol+RS_cumulo+rab_integral+RQ_integral+ AS_Vol.+emission_CO2+interval_O2; 0.363, 0.689, tmp_cumulo+ PL_cumulo+AN_cumulo+interval_yield+interval_Pdt.+RS+Feed_Vo l+RS_cumulo+rab_integral+RQ_integral+AS_Vol.+emission_CO2+c onsum_O2; 0.373, 0.689, tmp_cumulo+PL_cumulo+AN_cumulo+inte rval_yield+Feed_Vol+RS_cumulo+rab_integral+RQ_integral+AS_V ol.+emission_CO2+consum_O2+interval_O2; 0.411, 0.689, PL_cu mulo+interval_yield+interval_Pdt.+RS_cumulo+rab_integral+RQ _integral+AS_Vol.+emission_CO2; 0.383, 0.689, tmp_cumulo+AN +AN_cumulo+interval_yield+RS+Feed_Vol+RS_cumulo+rab_integra l+RQ_integral+AS_Vol.+emission_CO2; 0.383, 0.689, PL_cumulo +AN_cumulo+interval_yield+interval_Pdt.+Feed_Vol+RS_cumulo+ rab_integral+RQ_integral+AS_Vol.+emission_CO2+interval_O2; 0.362, 0.688, tmp_cumulo+PL_cumulo+AN_cumulo+interval_yield +interval_Pdt.+RS+Feed_Vol+RS_cumulo+rab_integral+RQ_integr al+AS_Vol.+emission_CO2+interval_O2; 0.372, 0.688, tmp_cumu lo+pH+PL+PL_cumulo+AN_cumulo+interval_yield+Feed_Vol+RS_cum ulo+rab_integral+RQ_integral+AS_Vol.+emission_CO2; 0.372, 0. 688, tmp_cumulo+PL_cumulo+AN_cumulo+interval_yield+interval _Pdt.+RS+Feed_Vol+RS_cumulo+rab_integral+RQ_integral+AS_Vol. +emission_O2; 0.372, 0.688, tmp_cumulo+AN_cumulo+interval_y ield+interval_Pdt.+RS+Feed_Vol+RS_cumulo+rab_integral+RQ_in tegral+AS_Vol.+emission_CO2+consum_O2; 0.382, 0.688, O2+PL_ cumulo+AN_cumulo+interval_yield+interval_Pdt.+RS_cumulo+rab _integral+RQ_integral+AS_Vol.+emission_CO2+interval_O2; 0.3 82, 0.688, tmp_cumulo+PL_cumulo+AN_cumulo+interval_yield+in terval_Pdt.+RS_cumulo+rab_integral+RQ_integral+AS_Vol.+emis sion_CO2+consum_O2; 0.391, 0.688, O2+PL_cumulo+AN_cumulo+in terval_yield+Feed_Vol+RS_cumulo+rab_integral+RQ_integral+AS _Vol.+emission_CO2; 0.372, 0.688, tmp_cumulo+PL+PL_cumulo+A N_cumulo+interval_yield+Feed_Vol+RS_cumulo+rab_integral+RQ_ integral+AS_Vol.+emission_CO2+interval_O2; 0.381, 0.688, tm p_cumulo+pH_cumulo+PL_cumulo+AN_cumulo+interval_yield+Feed_ Vol+RS_cumulo+rab_integral+RQ_integral+AS_Vol.+emission_CO 2; 0.361, 0.688, tmp+tmp_cumulo+PL_cumulo+AN_cumulo+interva l_yield+interval_Pdt.+Feed_Vol+RS_cumulo+rab_integral+RQ_in tegral+AS_Vol.+emission_CO2+interval_O2; 0.371, 0.688, tmp_ cumulo+AN+AN_cumulo+interval_yield+interval_Pdt.+Feed_Vol+R S_cumulo+rab_integral+RQ_integral+AS_Vol.+emission_CO2+cons um_O2; 0.361, 0.688, tmp_cumulo+PL+PL_cumulo+AN_cumulo+inte rval_yield+interval_Pdt.+Feed_Vol+RS_cumulo+rab_integral+RQ _integral+AS_Vol.+emission_CO2+interval_O2; 0.371, 0.688, t mp_cumulo+PL+PL_cumulo+AN+AN_cumulo+interval_yield+Feed_Vol +RS_cumulo+rab_integral+RQ_integral+AS_Vol.+emission_CO2; 0. 390, 0.688, tmp+PL_cumulo+AN_cumulo+interval_yield+interval _Pdt.+RS_cumulo+rab_integral+RQ_integral+AS_Vol.+emission_C O2; 0.381, 0.688, tmp_cumulo+PL+PL_cumulo+AN_cumulo+interva l_yield+interval_Pdt.+RS_cumulo+rab_integral+RQ_integral+AS _Vol.+emission_CO2; 0.381, 0.688, tmp_cumulo+AN_cumulo+inte rval_yield+interval_Pdt.+RS+Feed_Vol+RS_cumulo+rab_integral +RQ_integral+AS_Vol.+emission_CO2; 0.371, 0.688, tmp_cumulo +pH+PL_cumulo+AN_cumulo+interval_yield+interval_Pdt.+Feed_V ol+RS_cumulo+rab_integral+RQ_integral+AS_Vol.+emission_CO2; 0.381, 0.688, pH+PL_cumulo+AN_cumulo+interval_yield+interv al_Pdt.+RS_cumulo+rab_integral+RQ_integral+AS_Vol.+emission _CO2+interval_O2; 0.390, 0.688, tmp_cumulo+PL_cumulo+interv al_yield+Feed_Vol+RS_cumulo+rab_integral+RQ_integral+AS_Vol. +emission_CO2+interval_O2; 0.371, 0.688, tmp_cumulo+pH_cumu lo+PL_cumulo+AN_cumulo+interval_yield+Feed_Vol+RS_cumulo+ra b_integral+RQ_integral+AS_Vol.+emission_CO2+interval_O2; 0. 400, 0.687, PL_cumulo+AN_cumulo+interval_yield+Feed_Vol+RS_ cumulo+rab_integral+RQ_integral+AS_Vol.+emission_CO2; 0.390, 0.687, tmp_cumulo+pH_cumulo+AN_cumulo+interval_yield+Feed_ Vol+RS_cumulo+rab_integral+RQ_integral+AS_Vol.+emission_CO 2; 0.360, 0.687, tmp_cumulo+pH+PL_cumulo+AN_cumulo+interval _yield+interval_Pdt.+Feed_Vol+RS_cumulo+rab_integral+RQ_int egral+AS_Vol.+emission_CO2+consum_O2; 0.370, 0.687, tmp_cum ulo+AN+AN_cumulo+interval_yield+RS+Feed_Vol+RS_cumulo+rab_i ntegral+RQ_integral+AS_Vol.+emission_CO2+interval_O2; 0.390, 0.687, PL_cumulo+AN+AN_cumulo+interval_yield+interval_Pdt. +RS_cumulo+rab_integral+RQ_integral+AS_Vol.+emission_CO2; 0. 390, 0.687, tmp_cumulo+pH+PL_cumulo+interval_yield+Feed_Vol +RS_cumulo+rab_integral+RQ_integral+AS_Vol.+emission_CO2; 0. 380, 0.687, tmp_cumulo+PL_cumulo+AN+AN_cumulo+interval_yiel d+interval_Pdt.+RS_cumulo+rab_integral+RQ_integral+AS_Vol.+ emission_CO2; 0.380, 0.687, tmp_cumulo+AN_cumulo+interval_y ield+interval_Pdt.+RS+Feed_Vol+RS_cumulo+rab_integral+RQ_in tegral+AS_Vol.+emission_O2; 0.390, 0.687, tmp_cumulo+PL+AN_ cumulo+interval_yield+Feed_Vol+RS_cumulo+rab_integral+RQ_in tegral+AS_Vol.+emission_CO2; 0.380, 0.687, O2+PL_cumulo+AN_ cumulo+interval_yield+interval_Pdt.+Feed_Vol+RS_cumulo+rab_ integral+RQ_integral+AS_Vol.+emission_CO2; 0.380, 0.687, tm p+tmp_cumulo+PL_cumulo+AN_cumulo+interval_yield+Feed_Vol+RS _cumulo+rab_integral+RQ_integral+AS_Vol.+emission_CO2; 0.36 0, 0.687, tmp_cumulo+AN+AN_cumulo+interval_yield+RS+Feed_Vo l+RS_cumulo+rab_integral+RQ_integral+AS_Vol.+emission_CO2+c onsum_O2+interval_O2; 0.380, 0.687, O2+PL_cumulo+AN+AN_cumu lo+interval_yield+interval_Pdt.+RS_cumulo+rab_integral+RQ_i ntegral+AS_Vol.+emission_CO2; 0.370, 0.687, tmp_cumulo+PL_c umulo+AN_cumulo+interval_yield+interval_Pdt.+OD+Feed_Vol+RS _cumulo+rab_integral+RQ_integral+AS_Vol.+emission_CO2; 0.37 0, 0.687, tmp_cumulo+AN_cumulo+interval_yield+RS+Feed_Vol+R S_cumulo+rab_integral+RQ_integral+AS_Vol.+emission_CO2+cons um_O2+interval_O2; 0.370, 0.687, tmp_cumulo+PL+PL_cumulo+AN _cumulo+interval_yield+RS+Feed_Vol+RS_cumulo+rab_integral+R Q_integral+AS_Vol.+emission_CO2; 0.380, 0.687, PL_cumulo+AN _cumulo+interval_yield+interval_Pdt.+RS_cumulo+rab_integral +RQ_integral+AS_Vol.+emission_CO2+consum_O2+interval_O2; 0. 389, 0.687, tmp_cumulo+PL_cumulo+interval_yield+Feed_Vol+RS _cumulo+rab_integral+RQ_integral+AS_Vol.+emission_O2+emissi on_CO2; 0.380, 0.687, tmp_cumulo+pH+AN_cumulo+interval_yiel d+Feed_Vol+RS_cumulo+rab_integral+RQ_integral+AS_Vol.+emiss ion-CO2+interval_O2; 0.359, 0.687, tmp_cumulo+pH_cumulo+PL_ cumulo+AN_cumulo+interval_yield+interval_Pdt.+Feed_Vol+RS_c umulo+rab_integral+RQ_integral+AS_Vol.+emission_CO2+interva l_O2; 0.389, 0.687, tmp_cumulo+PL+PL_cumulo+interval_yield+ Feed_Vol+RS_cumulo+rab_integral+RQ_integral+AS_Vol.+emissio n_CO2; 0.408, 0.687, tmp_cumulo+PL_cumulo+interval_yield+RS +Feed_Vol+RS_cumulo+AS_Vol.+emission_O2; 0.369, 0.687, tmp+ tmp_cumulo+PL_cumulo+AN_cumulo+interval_yield+interval_Pdt. +RS_cumulo+rab_integral+RQ_integral+AS_Vol.+emission_CO2+in terval_O2; 0.408, 0.687, tmp_cumulo+PL_cumulo+interval_yiel d+Feed_Vol+RS_cumulo+RQ_integral+AS_Vol.+emission_CO2; 0.41 7, 0.687, PL_cumulo+interval_yield+interval_Pdt.+RS_cumulo+ RQ_integral+AS_Vol.+emission_CO2; 0.389, 0.687, tmp_cumulo+ PL_cumulo+interval_yield+interval_Pdt.+RS_cumulo+rab_integr al+RQ_integral+AS_Vol.+emission_CO2+interval_O2; 0.389, 0.6 87, PL_cumulo+AN_cumulo+interval_yield+Feed_Vol+RS_cumulo+r ab_integral+RQ_integral+AS_Vol.+emission_CO2+interval_O2; 0. 398, 0.687, tmp_cumulo+PL_cumulo+interval_yield+RS+Feed_Vol +RS_cumulo+AS_Vol.+emission_O2+interval_O2; 0.359, 0.687, t mp_cumulo+PL+PL_cumulo+AN_cumulo+interval_yield+interval_Pd t.+Feed_Vol+RS_cumulo+rab_integral+RQ_integral+AS_Vol.+emis sion_CO2+consum_O2; 0.379, 0.687, tmp+PL+PL_cumulo+AN_cumul o+interval_yield+interval_Pdt.+RS_cumulo+rab_integral+RQ_in tegral+AS_Vol.+emission_CO2; 0.369, 0.687, tmp_cumulo+PL_cu mulo+AN+AN_cumulo+interval_yield+interval_Pdt.+RS_cumulo+ra b_integral+RQ_integral+AS_Vol.+emission_CO2+consum_O2; 0.35 9, 0.687, tmp_cumulo+AN+AN_cumulo+interval_yield+interval_P dt.+RS+Feed_Vol+RS_cumulo+rab_integral+RQ_integral+AS_Vol.+ emission_CO2+consum_O2; 0.389, 0.687, PL_cumulo+AN_cumulo+i nterval_yield+interval_Pdt.+Feed_Vol+RS_cumulo+rab_integral +RQ_integral+AS_Vol.+emission_CO2; 0.389, 0.686, tmp_cumulo +PL_cumulo+interval_yield+RS+Feed_Vol+RS_cumulo+rab_integra l+RQ_integral+AS_Vol.+emission_CO2; 0.389, 0.686, tmp+tmp_c umulo+AN_cumulo+interval_yield+Feed_Vol+RS_cumulo+rab_integ ral+RQ_integral+AS_Vol.+emission_CO2; 0.369, 0.686, tmp_cum ulo+AN_cumulo+interval_yield+interval_Pdt.+Feed_Vol+RS_cumu lo+rab_integral+RQ_integral+AS_Vol.+emission_CO2+consum_O2+ interval_O2; 0.379, 0.686, PL_cumulo+AN_cumulo+interval_yie ld+interval_Pdt.+RS+RS_cumulo+rab_integral+RQ_integral+AS_V ol.+emission_CO2+interval_O2; 0.369, 0.686, tmp+tmp_cumulo+ PL_cumulo+AN_cumulo+interval_yield+Feed_Vol+RS_cumulo+rab_i ntegral+RQ_integral+AS_Vol.+emission_CO2+interval_O2; 0.369, 0.686, tmp_cumulo+PL_cumulo+AN_cumulo+interval_yield+inter val_Pdt.+RS+Feed_Vol+RS_cumulo+rab_integral+RQ_integral+AS_ Vol.+emission_CO2; 0.379, 0.686, tmp_cumulo+AN_cumulo+inter val_yield+Feed_Vol+RS_cumulo+rab_integral+RQ_integral+AS_Vo l.+emission_CO2+consum_O2+interval_O2; 0.379, 0.686, tmp_cu mulo+AN+AN_cumulo+interval_yield+interval_Pdt.+Feed_Vol+RS_ cumulo+rab_integral+RQ_integral+AS_Vol.+emission_CO2; 0.358, 0.686, tmp_cumulo+PL_cumulo+AN_cumulo+interval_yield+inter val_Pdt.+OD+Feed_Vol+RS_cumulo+rab_integral+RQ_integral+AS_ Vol.+emission_CO2+interval_O2; 0.416, 0.686, O2+PL_cumulo+A N_cumulo+interval_yield+RS+Feed_Vol+AS_Vol.; 0.369, 0.686, tmp_cumulo+AN_cumulo+interval_yield+interval_Pdt.+RS+Feed_V ol+RS_cumulo+rab_integral+RQ_integral+AS_Vol.+emission_CO2+ interval_O2; 0.358, 0.686, tmp_cumulo+PL_cumulo+AN+AN_cumul o+interval_yield+Feed_Vol+RS_cumulo+rab_integral+RQ_integra l+AS_Vol.+emission_CO2+consum_O2+interval_O2; 0.388, 0.686, pH+PL_cumulo+AN_cumulo+interval_yield+interval_Pdt.+RS_cum ulo+rab_integral+RQ_integral+AS_Vol.+emission_CO2; 0.358, 0. 686, tmp_cumulo+PL_cumulo+AN_cumulo+interval_yield+RS+Feed_ Vol+RS_cumulo+rab_integral+RQ_integral+AS_Vol.+emission_CO2 +consum_O2+interval_O2; 0.358, 0.686, tmp_cumulo+PL_cumulo+ AN+AN_cumulo+interval_yield+RS+Feed_Vol+RS_cumulo+rab_integ ral+RQ_integral+AS_Vol.+emission_CO2+interval_O2; 0.388, 0. 686, tmp_cumulo+PL_cumulo+interval_yield+RS+Feed_Vol+RS_cum ulo+AS_Vol.+emission_O2+consum_O2+interval_O2; 0.388, 0.686, tmp_cumulo+PL+PL_cumulo+interval_yield+interval_Pdt.+RS_cu mulo+rab_integral+RQ_integral+AS_Vol.+emission_CO2; 0.368, 0.686, tmp_cumulo+PL_cumulo+AN+AN_cumulo+interval_yield+int erval_Pdt.+Feed_Vol+RS_cumulo+rab_integral+RQ_integral+AS_V ol.+emission_CO2; 0.378, 0.686, tmp_cumulo+pH+AN_cumulo+int erval_yield+RS+Feed_Vol+RS_cumulo+rab_integral+RQ_integral+ AS_Vol.+emission_CO2; 0.378, 0.686, tmp_cumulo+pH+AN_cumulo +interval_yield+interval_Pdt.+Feed_Vol+RS_cumulo+rab_integr al+RQ_integral+AS_Vol.+emission_CO2; 0.358, 0.686, tmp_cumu lo+PL_cumulo+AN_cumulo+interval_yield+interval_Pdt.+Feed_Vo l+RS_cumulo+rab_integral+RQ_integral+AS_Vol.+emission_O2+em ission_CO2+interval_O2; 0.378, 0.686, tmp_cumulo+PL_cumulo+ AN_cumulo+interval_yield+OD+Feed_Vol+RS_cumulo+rab_integral +RQ_integral+AS_Vol.+emission_CO2; 0.368, 0.686, tmp+tmp_cu mulo+PL_cumulo+AN_cumulo+interval_yield+interval_Pdt.+Feed_ Vol+RS_cumulo+rab_integral+RQ_integral+AS_Vol.+emission_CO 2; 0.406, 0.686, tmp_cumulo+interval_yield+Feed_Vol+RS_cumu lo+rab_integral+RQ_integral+AS_Vol.+emission_CO2; 0.357, 0. 686, tmp+tmp_cumulo+PL_cumulo+AN_cumulo+interval_yield+inte rval_Pdt.+Feed_Vol+RS_cumulo+rab_integral+RQ_integral+AS_Vo l.+emission_CO2+consum_O2; 0.378, 0.686, pH+PL+PL_cumulo+AN _cumulo+interval_yield+interval_Pdt.+RS_cumulo+rab_integral +RQ_integral+AS_Vol.+emission_CO2; 0.378, 0.686, tmp_cumulo +PL_cumulo+interval_yield+interval_Pdt.+Feed_Vol+RS_cumulo+ rab_integral+RQ_integral+AS_Vol.+emission_O2+emission_CO2; 0.378, 0.686, tmp_cumulo+PL_cumulo+AN_cumulo+interval_yield +Feed_Vol+RS_cumulo+rab_integral+RQ_integral+AS_Vol.+emissi on_O2+emission_CO2; 0.397, 0.686, tmp_cumulo+PL_cumulo+AN_c umulo+interval_yield+RS+Feed_Vol+RS_cumulo+AS_Vol.+emission _O2; 0.397, 0.686, O2+PL_cumulo+interval_yield+interval_Pdt. +RS_cumulo+rab_integral+RQ_integral+AS_Vol.+emission_CO2; 0. 368, 0.686, tmp_cumulo+PL_cumulo+AN_cumulo+interval_yield+i nterval_Pdt.+RS_cumulo+rab_integral+RQ_integral+AS_Vol.+emi ssion_CO2+consum_O2+interval_O2; 0.378, 0.686, PL_cumulo+AN _cumulo+interval_yield+interval_Pdt.+OD+RS_cumulo+rab_integ ral+RQ_integral+AS_Vol.+emission_CO2+interval_O2; 0.367, 0. 686, tmp_cumulo+AN+AN_cumulo+interval_yield+interval_Pdt.+F eed_Vol+RS_cumulo+rab_integral+RQ_integral+AS_Vol.+emission _CO2+interval_O2; 0.406, 0.686, tmp_cumulo+PL_cumulo+interv al_yield+interval_Pdt.+RS_cumulo+RQ_integral+AS_Vol.+emissi on_CO2; 0.387, 0.686, PL_cumulo+AN_cumulo+interval_yield+in terval_Pdt.+OD+RS_cumulo+rab_integral+RQ_integral+AS_Vol.+e mission_CO2; 0.377, 0.686, pH_cumulo+PL_cumulo+AN_cumulo+in terval_yield+interval_Pdt.+RS_cumulo+rab_integral+RQ_integr al+AS_Vol.+emission_CO2+interval_O2; 0.387, 0.686, tmp_cumu lo+AN_cumulo+interval_yield+interval_Pdt.+Feed_Vol+RS_cumul o+rab_integral+RQ_integral+AS_Vol.+emission_O2; 0.367, 0.68 5, tmp_cumulo+pH+PL_cumulo+AN_cumulo+interval_yield+Feed_Vo l+RS_cumulo+rab_integral+RQ_integral+AS_Vol.+emission_CO2+c onsum_O2; 0.396, 0.685, tmp_cumulo+AN_cumulo+interval_yield +interval_Pdt.+RS_cumulo+rab_integral+RQ_integral+AS_Vol.+e mission_CO2; 0.367, 0.685, tmp_cumulo+pH+PL_cumulo+AN_cumul o+interval_yield+interval_Pdt.+RS_cumulo+rab_integral+RQ_in tegral+AS_Vol.+emission_CO2+interval_O2; 0.377, 0.685, tmp+ tmp_cumulo+PL_cumulo+AN_cumulo+interval_yield+interval_Pdt. +RS_cumulo+rab_integral+RQ_integral+AS_Vol.+emission_CO2; 0. 346, 0.685, tmp_cumulo+PL_cumulo+AN+AN_cumulo+interval_yiel d+RS+Feed_Vol+RS_cumulo+rab_integral+RQ_integral+AS_Vol.+em ission_CO2+consum_O2+interval_O2; 0.367, 0.685, tmp_cumulo+ PL_cumulo+AN_cumulo+interval_yield+interval_Pdt.+RS+RS_cumu lo+rab_integral+RQ_integral+AS_Vol.+emission_CO2+interval_O 2; 0.387, 0.685, tmp_cumulo+AN_cumulo+interval_yield+OD+Fee d_Vol+RS_cumulo+rab_integral+RQ_integral+AS_Vol.+emission_C 02; 0.367, 0.685, tmp_cumulo+AN+AN_cumulo+interval_yield+Fe ed_Vol+RS_cumulo+rab_integral+RQ_integral+AS_Vol.+emission_ CO2+consum_O2+interval_O2; 0.356, 0.685, tmp_cumulo+PL_cumu lo+AN_cumulo+interval_yield+interval_Pdt.+Feed_Vol+RS_cumul o+rab_integral+RQ_integral+AS_Vol.+emission_O2+emission_CO2 +consum_O2; 0.367, 0.685, tmp_cumulo+PL_cumulo+AN+AN_cumulo +interval_yield+RS+Feed_Vol+RS_cumulo+rab_integral+RQ_integ ral+AS_Vol.+emission_CO2; 0.356, 0.685, tmp_cumulo+pH+AN+AN _cumulo+interval_yield+RS+Feed_Vol+RS_cumulo+rab_integral+R Q_integral+AS_Vol.+emission_CO2+consum_O2; 0.386, 0.685, PL _cumulo+AN_cumulo+interval_yield+interval_Pdt.+RS+RS_cumulo +rab_integral+RQ_integral+AS_Vol.+emission_CO2; 0.386, 0.68 5, tmp_cumulo+PL_cumulo+AN_cumulo+interval_yield+Feed_Vol+R S_cumulo+rab_integral+RQ_integral+AS_Vol.+emission_O2; 0.34 5, 0.685, tmp_cumulo+PL_cumulo+AN+AN_cumulo+interval_yield+ interval_Pdt.+Feed_Vol+RS_cumulo+rab_integral+RQ_integral+A S_Vol.+emission_CO2+consum_O2+interval_O2; 0.345, 0.685, tm p_cumulo+PL_cumulo+AN+AN_cumulo+interval_yield+interval_Pdt. +RS+Feed_Vol+RS_cumulo+rab_integral+RQ_integral+AS_Vol.+emi ssion_CO2+consum_O2; 0.356, 0.685, tmp_cumulo+PL_cumulo+AN_ cumulo+interval_yield+interval_Pdt.+OD+Feed_Vol+RS_cumulo+r ab_integral+RQ_integral+AS_Vol.+emission_CO2+consum_O2; 0.4 05, 0.685, PL_cumulo+AN_cumulo+interval_yield+RS+Feed_Vol+R S_cumulo+AS_Vol.+emission_O2; 0.366, 0.685, tmp_cumulo+PL_c umulo+AN_cumulo+interval_yield+Feed_Vol+RS_cumulo+rab_integ ral+RQ_integral+AS_Vol.+emission_O2+emission_CO2+interval_O 2; 0.366, 0.685, tmp_cumulo+pH_cumulo+PL_cumulo+AN_cumulo+i nterval_yield+interval_Pdt.+Feed_Vol+RS_cumulo+rab_integral +RQ_integral+AS_Vol.+emission_CO2; 0.405, 0.685, O2+PL_cumu lo+interval_yield+interval_Pdt.+RS_cumulo+RQ_integral+AS_Vo l.+emission_CO2; 0.376, 0.685, tmp_cumulo+PL_cumulo+interva l_yield+interval_Pdt.+Feed_Vol+RS_cumulo+rab_integral+RQ_in tegral+AS_Vol.+emission_CO2+interval_O2; 0.376, 0.685, tmp_ cumulo+pH+AN+AN_cumulo+interval_yield+Feed_Vol+RS_cumulo+ra b_integral+RQ_integral+AS_Vol.+emission_CO2; 0.356, 0.685, tmp_cumulo+pH+PL+PL_cumulo+AN_cumulo+interval_yield+interva l_Pdt.+Feed_Vol+RS_cumulo+rab_integral+RQ_integral+AS_Vol.+ emission_CO2; 0.386, 0.685, tmp+tmp_cumulo+PL_cumulo+interv al_yield+interval_Pdt.+RS_cumulo+rab_integral+RQ_integral+A S_Vol.+emission_CO2; 0.366, 0.685, tmp_cumulo+PL+PL_cumulo+ AN_cumulo+interval_yield+Feed_Vol+RS_cumulo+rab_integral+RQ _integral+AS_Vol.+emission_CO2+consum_O2; 0.366, 0.685, tmp _cumulo+PL_cumulo+AN_cumulo+interval_yield+interval_Pdt.+Fe ed_Vol+RS_cumulo+rab_integral+RQ_integral+AS_Vol.+emission_ O2+emission_CO2; 0.376, 0.685, tmp_cumulo+AN_cumulo+interva l_yield+interval_Pdt.+OD+Feed_Vol+RS_cumulo+rab_integral+RQ _integral+AS_Vol.+emission_CO2; 0.355, 0.685, tmp_cumulo+pH +PL_cumulo+AN+AN_cumulo+interval_yield+Feed_Vol+RS_cumulo+r ab_integral+RQ_integral+AS_Vol.+emission_CO2+interval_O2; 0. 376, 0.685, O2+PL_cumulo+AN_cumulo+interval_yield+interval_ Pdt.+RS+RS_cumulo+rab_integral+RQ_integral+AS_Vol.+emission _CO2; 0.376, 0.685, PL_cumulo+AN+AN_cumulo+interval_yield+i nterval_Pdt.+RS_cumulo+rab_integral+RQ_integral+AS_Vol.+emi ssion_CO2+consum_O2; 0.386, 0.685, tmp_cumulo+pH+PL_cumulo+ interval_yield+interval_Pdt.+RS_cumulo+rab_integral+RQ_inte gral+AS_Vol.+emission_CO2; 0.345, 0.684, tmp_cumulo+PL_cumu lo+AN_cumulo+interval_yield+interval_Pdt.+RS+Feed_Vol+RS_cu mulo+rab_integral+RQ_integral+AS_Vol.+emission_CO2+consuni_O 2+interval_O2; 0.376, 0.684, tmp_cumulo+PL+PL_cumulo+interv al_yield+interval_Pdt.+Feed_Vol+RS_cumulo+rab_integral+RQ_i ntegral+AS_Vol.+emission_CO2; 0.376, 0.684, tmp_cumulo+pH_c umulo+AN_cumulo+interval_yield+Feed_Vol+RS_cumulo+rab_integ ral+RQ_integral+AS_Vol.+emission_CO2+interval_O2; 0.365, 0. 684, tmp_cumulo+pH+AN_cumulo+interval_yield+interval_Pdt.+F eed_Vol+RS_cumulo+rab_integral+RQ_integral+AS_Vol.+emission _CO2+interval_O2; 0.376, 0.684, tmp_cumulo+AN+AN_cumulo+int erval_yield+RS+Feed_Vol+RS_cumulo+rab_integral+RQ_integral+ AS_Vol.+emission_O2; 0.365, 0.684, tmp_cumulo+PL+PL_cumulo+ AN_cumulo+interval_yield+interval_Pdt.+RS_cumulo+rab_integr al+RQ_integral+AS_Vol.+emission_CO2+interval_O2; 0.355, 0.6 84, tmp_cumulo+pH+PL_cumulo+AN_cumulo+interval_yield+RS+Fee d_Vol+RS_cumulo+rab_integral+RQ_integral+AS_Vol.+emission_C O2+interval_O2; 0.375, 0.684, tmp_cumulo+AN_cumulo+interval _yield+RS+Feed_Vol+RS_cumulo+rab_integral+RQ_integral+AS_Vo l.+emission_O2+consum_O2; 0.395, 0.684, O2+PL_cumulo+AN_cum ulo+interval_yield+RS+Feed_Vol+AS_Vol.+consum_O2+interval_O 2; 0.385, 0.684, tmp_cumulo+PL_cumulo+interval_yield+interv al_Pdt.+RS_cumulo+rab_integral+RQ_integral+AS_Vol.+emission _O2+emission_CO2; 0.365, 0.684, tmp_cumulo+pH+AN_cumulo+int erval_yield+RS+Feed_Vol+RS_cumulo+rab_integral+RQ_integral+ AS_Vol.+emission_CO2+consum_02; 0.404, 0.684, O2+PL_cumulo+ interval_yield+OD+RS+Feed_Vol+AS_Vol.+interval_O2; 0.385, 0. 684, tmp_cumulo+pH_cumulo+PL_cumulo+interval_yield+Feed_Vol +RS_cumulo+rab_integral+RQ_integral+AS_Vol.+emission_CO2; 0. 365, 0.684, tmp_cumulo+pH_cumulo+PL+PL_cumulo+AN_cumulo+int erval_yield+Feed_Vol+RS_cumulo+rab_integral+RQ_integral+AS_ Vol.+emission_CO2; 0.375, 0.684, tmp+tmp_cumulo+AN_cumulo+i nterval_yield+interval_Pdt.+Feed_Vol+RS_cumulo+rab_integral +RQ_integral+AS_Vol.+emission_CO2; 0.365, 0.684, tmp_cumulo +pH+PL_cumulo+AN_cumulo+interval_yield+RS+Feed_Vol+RS_cumul o+rab_integral+RQ_integral+AS_Vol.+emission_CO2; 0.394, 0.6 84, tmp_cumulo+AN_cumulo+interval_yield+Feed_Vol+RS_cumulo+ rab_integral+RQ_integral+AS_Vol.+emission_O2; 0.375, 0.684, tmp_cumulo+AN+AN_cumulo+interval_yield+RS+Feed_Vol+rab_int egral+RQ_integral+AS_Vol.+emission_CO2+consum_O2; 0.375, 0. 684, tmp_cumulo+pH+PL_cumulo+interval_yield+interval_Pdt.+F eed_Vol+RS_cumulo+rab_integral+RQ_integral+AS_Vol.+emission _CO2; 0.365, 0.684, tmp_cumulo+pH+AN_cumulo+interval_yield+ interval_Pdt.+Feed_Vol+RS_cumulo+rab_integral+RQ_integral+A S_Vol.+emission_CO2+consum_O2; 0.375, 0.684, tmp_cumulo+pH+ PL_cumulo+AN_cumulo+interval_yield+interval_Pdt.+RS_cumulo+ rab_integral+RQ_integral+AS_Vol.+emission_CO2; 0.365, 0.684, tmp_cumulo+PL_cumulo+AN_cumulo+interval_yield+OD+Feed_Vol+ RS_cumulo+rab_integral+RQ_integral+AS_Vol.+emission_CO2+int erval_O2; 0.385, 0.684, pH_cumulo+PL_cumulo+AN_cumulo+inter val_yield+interval_Pdt.+RS_cumulo+rab_integral+RQ_integral+ AS_Vol.+emission_CO2; 0.403, 0.684, PL_cumulo+interval_yiel d+OD+RS+Feed_Vol+AS_Vol.+emission_O2+interval_O2; 0.375, 0. 684, tmp_cumulo+AN_cumulo+interval_yield+RS+Feed_Vol+RS_cum ulo+rab_integral+RQ_integral+AS_Vol.+emission_O2+interval_O 2; 0.365, 0.684, tmp_cumulo+pH+PL_cumulo+AN+AN_cumulo+inter val_yield+Feed_Vol+RS_cumulo+rab_integral+RQ_integral+AS_Vo 1.+emission_CO2; 0.375, 0.684, tmp_cumulo+pH+AN_cumulo+inte rval_yield+Feed_Vol+RS_cumulo+rab_integral+RQ_integral+AS_V ol.+emission_CO2+consum_O2; 0.343, 0.684, tmp_cumulo+pH+PL_ cumulo+AN_cumulo+interval_yield+interval_Pdt.+FeedVol+RS_c umulo+rab_integral+RQ_integral+AS_Vol.+emission_CO2+consum_ O2+interval_O2; 0.354, 0.684, tmp_cumulo+pH_cumulo+PL_cumul o+AN_cumulo+interval_yield+interval_Pdt.+Feed_Vol+RS_cumulo +rab_integral+RQ_integral+AS_Vol.+emission_CO2+consum_O2; 0. 364, 0.684, tmp_cumulo+pH+AN_cumulo+interval_yield+RS+Feed_ Vol+RS_cumulo+rab_integral+RQ_integral+AS_Vol.+emission_CO2 +interval_O2; 0.375, 0.684, tmp+tmp_cumulo+AN_cumulo+interv al_yield+RS+Feed_Vol+RS_cumulo+rab_integral+RQ_integral+AS_ Vol.+emission_CO2; 0.384, 0.684, tmp_cumulo+PL_cumulo+inter val_yield+interval_Pdt.+Feed_Vol+RS_cumulo+rab_integral+RQ_ integral+AS_Vol.+emission_O2; 0.394, 0.684, O2+PL_cumulo+AN _cumulo+interval_yield+RS+Feed_Vol+RS_cumulo+AS_Vol.+emissi on_CO2; 0.394, 0.684, tmp+PL_cumulo+interval_yield+interval _Pdt.+RS_cumulo+rab_integral+RQ_integral+AS_Vol.+emission_C 02; 0.364, 0.684, O2+PL_cumulo+AN_cumulo+interval_yield+int erval_Pdt.+Feed_Vol+RS_cumulo+rab_integral+RQ_integral+AS_V ol.+emission_CO2+interval_O2; 0.374, 0.684, tmp_cumulo+PL_c umulo+AN_cumulo+interval_yield+interval_Pdt.+RS+RS_cumulo+r ab_integral+RQ_integral+AS_Vol.+emission_CO2; 0.354, 0.684, tmp_cumulo+PL_cumulo+AN_cumulo+interval_yield+interval_Pdt. +RS+Feed_Vol+RS_cumulo+rab_integral+RQ_integral+AS_Vol.+emi ssion_O2+consum_O2; 0.384, 0.684, tmp_cumulo+PL_cumulo+inte rval_yield+RS+Feed_Vol+RS_cumulo+rab_integral+RQ_integral+A S_Vol.+emission_O2; 0.364, 0.684, tmp_cumulo+PL+PL_cumulo+A N+AN_cumulo+interval_yield+interval_Pdt.+RS_cumulo+rab_inte gral+RQ_integral+AS_Vol.+emission_CO2; 0.364, 0.684, tmp_cu mulo+AN_cumulo+interval_yield+RS+Feed_Vol+RS_cumulo+rab_int egral+RQ_integral+AS_Vol.+emission_O2+consum_O2+interval_O 2; 0.364, 0.684, tmp_cumulo+PL_cumulo+AN_cumulo+interval_yi eld+Feed_Vol+RS_cumulo+rab_integral+RQ_integral+Accum._Yiel d+AS_Vol.+emission_CO2+interval_O2; 0.374, 0.684, O2+PL_cum ulo+AN_cumulo+interval_yield+RS+Feed_Vol+RS_cumulo+rab_inte gral+RQ_integral+AS_Vol.+emission_CO2; 0.374, 0.683, tmp+tm p_cumulo+AN_cumulo+interval_yield+Feed_Vol+RS_cumulo+rab_in tegral+RQ_integral+AS_Vol.+emission_CO2+interval_O2; 0.374, 0.683, tmp_cumulo+pH_cumulo+AN_cumulo+interval_yield+inter val_Pdt.+Feed_Vol+RS_cumulo+rab_integral+RQ_integral+AS_Vol. +emission_CO2; 0.374, 0.683, tmp_cumulo+PL+AN_cumulo+interv al_yield+RS+Feed_Vol+RS_cumulo+rab_integral+RQ_integral+AS_ Vol.+emission_CO2; 0.402, 0.683, O2+PL_cumulo+interval_yiel d+RS+Feed_Vol+AS_Vol.+consum_O2+interval_O2; 0.374, 0.683, O2+PL_cumulo+AN_cumulo+interval_yield+interval_Pdt.+RS_cumu lo+rab_integral+RQ_integral+AS_Vol.+emission_CO2+consum_O2; 0.411, 0.683, O2+PL_cumulo+interval_yield+RS+Feed_Vol+AS_V ol.+interval_O2; 0.374, 0.683, tmp+PL_cumulo+AN_cumulo+inte rval_yield+interval_Pdt.+RS_cumulo+rab_integral+RQ_integral +AS_Vol.+emission_CO2+consum_O2; 0.353, 0.683, tmp_cumulo+A N_cumulo+interval_yield+interval_Pdt.+RS+Feed_Vol+RS_cumulo +rab_integral+RQ_integral+AS_Vol.+emission_CO2+consum_O2+in terval_O2; 0.393, 0.683, PL_cumulo+interval_yield+interval_ Pdt.+RS_cumulo+rab_integral+RQ_integral+AS_Vol.+emission_CO 2+interval_O2; 0.353, 0.683, tmp_cumulo+pH+PL+PL_cumulo+AN_ cumulo+interval_yield+Feed_Vol+RS_cumulo+rab_integral+RQ_in tegral+AS_Vol.+emission_CO2+interval_O2; 0.393, 0.683, O2+P L_cumulo+AN_cumulo+interval_yield+interval_Pdt.+RS_cumulo+R Q_integral+AS_Vol.+emission_CO2; 0.393, 0.683, O2+PL_cumulo +interval_yield+OD+RS+Feed_Vol+AS_Vol.+consum_O2+interval_O 2; 0.364, 0.683, tmp+tmp_cumulo+PL+PL_cumulo+AN_cumulo+inte rval_yield+Feed_Vol+RS_cumulo+rab_integral+RQ_integral+AS_V ol.+emission_CO2; 0.374, 0.683, PL_cumulo+AN_cumulo+interva l_yield+interval_Pdt.+Feed_Vol+RS_cumulo+rab_integral+RQ_in tegral+AS_Vol.+emission_CO2+consum_O2; 0.393, 0.683, tmp_cu mulo+AN_cumulo+interval_yield+RS+Feed_Vol+rab_integral+RQ_i ntegral+AS_Vol.+emission_O2; 0.383, 0.683, pH_cumulo+PL+PL_ cumulo+interval_yield+OD+RS+Feed_Vol+AS_Vol.+consum_O2+inte rval_O2; 0.353, 0.683, tmp_cumulo+pH+PL_cumulo+AN+AN_cumulo +interval_yield+Feed_Vol+RS_cumulo+rab_integral+RQ_integral +AS_Vol.+emission_CO2+consum_O2; 0.383, 0.683, tmp_cumulo+P L_cumulo+interval_yield+interval_Pdt.+RS+RS_cumulo+rab_inte gral+RQ_integral+AS_Vol.+emission_CO2; 0.353, 0.683, tmp_cu mulo+PL+PL_cumulo+AN+AN_cumulo+interval_yield+Feed_Vol+RS_c umulo+rab_integral+RQ_integral+AS_Vol.+emission_CO2+interva l_O2; 0.374, 0.683, tmp_cumulo+PL+AN_cumulo+interval_yield+ Feed_Vol+RS_cumulo+rab_integral+RQ_integral+AS_Vol.+emissio n_CO2+interval_O2; 0.383, 0.683, tmp+tmp_cumulo+PL_cumulo+i nterval_yield+Feed_Vol+RS_cumulo+rab_integral+RQ_integral+A S_Vol.+emission_CO2; 0.402, 0.683, PL_cumulo+AN_cumulo+inte rval_yield+interval_Pdt.+RS_cumulo+RQ_integral+AS_Vol.+emis sion_CO2; 0.374, 0.683, tmp_cumulo+pH_cumulo+AN_cumulo+inte rval_yield+RS+Feed_Vol+RS_cumulo+rab_integral+RQ_integral+A S_Vol.+emission_CO2; 0.383, 0.683, tmp_cumulo+AN_cumulo+int erval_yield+interval_Pdt.+RS_cumulo+rab_integral+RQ_integra l+AS_Vol.+emission_CO2+interval_O2; 0.374, 0.683, pH+PL+PL_ cumulo+AN_cumulo+interval_yield+Feed_Vol+RS_cumulo+rab_inte gral+RQ_integral+AS_Vol.+emission_CO2; 0.363, 0.683, tmp_cu mulo+pH+AN+AN_cumulo+interval_yield+Feed_Vol+RS_cumulo+rab_ integral+RQ_integral+AS_Vol.+emission_CO2+consum_O2; 0.353, 0.683, tmp_cumulo+pH+PL_cumulo+AN_cumulo+interval_yield+RS +Feed_Vol+RS_cumulo+rab_integral+RQ_integral+AS_Vol.+emissi on_CO2+consum_O2; 0.383, 0.683, tmp_cumulo+AN+AN_cumulo+int erval_yield+interval_Pdt.+RS_cumulo+rab_integral+RQ_integra l+AS_Vol.+emission_CO2; 0.402, 0.683, O2+PL_cumulo+AN_cumul o+interval_yield+RS+Feed_Vol+RS_cumulo+AS_Vol.; 0.353, 0.68 3, tmp_cumulo+PL_cumulo+AN_cumulo+interval_yield+interval_P dt.+Feed_Vol+RS_cumulo+rab_integral+RQ_integral+Accum._Yiel d+AS_Vol.+emission_CO2+consum_O2; 0.353, 0.683, tmp+tmp_cum ulo+AN+AN_cumulo+interval_yield+RS+Feed_Vol+RS_cumulo+rab_i ntegral+RQ_integral+AS_Vol.+emission_CO2+consum_O2; 0.353, 0.683, tmp_cumulo+PL+PL_cumulo+AN+AN_cumulo+interval_yield+ Feed_Vol+RS_cumulo+rab_integral+RQ_integral+AS_Vol.+emissio n_CO2+consum_O2; 0.373, 0.683, tmp+PL+PL_cumulo+AN_cumulo+i nterval_yield+Feed_Vol+RS_cumulo+rab_integral+RQ_integral+A S_Vol.+emission_CO2; 0.373, 0.683, O2+PL_cumulo+AN_cumulo+i nterval_yield+interval_Pdt.+OD+RS_cumulo+rab_integral+RQ_in tegral+AS_Vol.+emission_CO2; 0.363, 0.683, tmp_cumulo+PL_cu mulo+AN_cumulo+interval_yield+interval_Pdt.+OD+RS_cumulo+ra b_integral+RQ_integral+AS_Vol.+emission_CO2+interval_O2; 0. 353, 0.683, tmp_cumulo+PL_cumulo+AN_cumulo+interval_yield+i nterval_Pdt.+Feed_Vol+RS_cumulo+rab_integral+RQ_integral+Ac cum._Yield+AS_Vol.+emission_CO2+interval_O2; 0.363, 0.683, tmp+PL_cumulo+AN_cumulo+interval_yield+interval_Pdt.+Feed_V ol+RS_cumulo+rab_integral+RQ_integral+AS_Vol.+emission_CO2+ interval_O2; 0.373, 0.683, tmp_cumulo+PL+AN+AN_cumulo+inter val_yield+Feed_Vol+RS_cumulo+rab_integral+RQ_integral+AS_Vo l.+emission_CO2; 0.373, 0.683, tmp_cumulo+PL+AN_cumulo+inte rval_yield+interval_Pdt.+Feed_Vol+RS_cumulo+rab_integral+RQ _integral+AS_Vol.+emission_CO2; 0.363, 0.683, tmp_cumulo+AN _cumulo+interval_yield+interval_Pdt.+RS+Feed_Vol+RS_cumulo+ rab_integral+RQ_integral+AS_Vol.+emission_O2+consum_O2; 0.3 63, 0.683, tmp_cumulo+PL_cumulo+AN_cumulo+interval_yield+RS +Feed_Vol+RS_cumulo+rab_integral+RQ_integral+AS_Vol.+emissi on_O2+interval_O2; 0.363, 0.683, tmp_cumulo+pH+AN+AN_cumulo +interval_yield+Feed_Vol+RS_cumulo+rab_integral+RQ_integral +AS_Vol.+emission_CO2+interval_O2; 0.373, 0.683, tmp_cumulo +AN_cumulo+interval_yield+OD+RS+Feed_Vol+RS_cumulo+rab_inte gral+RQ_integral+AS_Vol.+emission_CO2; 0.363, 0.683, tmp_cu mulo+PL_cumulo+AN_cumulo+interval_yield+RS+Feed_Vol+RS_cumu lo+rab_integral+RQ_integral+AS_Vol.+emission_O2+consum_O2; 0.383, 0.683, tmp_cumulo+PL_cumulo+interval_yield+interval_ Pdt.+RS_cumulo+rab_integral+RQ_integral+AS_Vol.+emission_CO 2+consum_O2; 0.363, 0.683, tmp+tmp_cumulo+AN_cumulo+interva l_yield+interval_Pdt.+Feed_Vol+RS_cumulo+rab_integral+RQ_in tegral+AS_Vol.+emission_CO2+interval_O2; 0.363, 0.683, O2+P L_cumulo+AN+AN_cumulo+interval_yield+interval_Pdt.+RS_cumul o+rab_integral+RQ_integral+AS_Vol.+emission_CO2+interval_O 2; 0.383, 0.683, tmp_cumulo+PL_cumulo+interval_yield+Feed_V ol+RS_cumulo+rab_integral+RQ_integral+AS_Vol.+emission_CO2+ consum_O2; 0.392, 0.683, O2+PL_cumulo+AN_cumulo+interval_yi eld+Feed_Vol+RS_cumulo+RQ_integral+AS_Vol.+emission_CO2; 0. 392, 0.683, tmp_cumulo+PL+PL_cumulo+interval_yield+Feed_Vol +RS_cumulo+RQ_integral+AS_Vol.+emission_CO2; 0.373, 0.683, tmp_cumulo+PL_cumulo+interval_yield+interval_Pdt.+Feed_Vol+ RS_cumulo+rab_integral+RQ_integral+AS_Vol.+emission_CO2+con sum_O2; 0.341, 0.683, tmp_cumulo+PL+PL_cumulo+AN_cumulo+int erval_yield+interval_Pdt.+Feed_Vol+RS_cumulo+rab_integral+R Q_integral+AS_Vol.+emission_CO2+consum_O2+interval_O2; 0.36 2, 0.683, tmp_cumulo+pH_cumulo+PL_cumulo+AN_cumulo+interval _yield+interval_Pdt.+RS_cumulo+rab_integral+RQ_integral+AS_ Vol.+emission_CO2+interval_O2; 0.362, 0.682, tmp+PL+PL_cumu lo+AN_cumulo+interval_yield+interval_Pdt.+RS_cumulo+rab_int egral+RQ_integral+AS_Vol.+emission_CO2+interval_O2; 0.372, 0.682, O2+PL_cumulo+AN_cumulo+interval_yield+Feed_Vol+RS_cu mulo+rab_integral+RQ_integral+AS_Vol.+emission_CO2+interval _O2; 0.352, 0.682, tmp_cumulo+PL_cumulo+AN+AN_cumulo+interv al_yield+interval_Pdt.+RS_cumulo+rab_integral+RQ_integral+A S_Vol.+emission_CO2+consum_O2+interval_02; 0.382, 0.682, PL _cumulo+AN_cumulo+interval_yield+interval_Pdt.+RS_cumulo+ra b_integral+RQ_integral+AS_Vol.+emission_CO2+generate_CO2; 0. 382, 0.682, tmp_cumulo+PL_cumulo+AN+AN_cumulo+interval_yiel d+interval_Pdt.+RS_cumulo+rab_integral+RQ_integral+emission _CO2; 0.352, 0.682, tmp_cumulo+PL+PL_cumulo+AN_cumulo+inter val_yield+RS+Feed_Vol+RS_cumulo+rab_integral+RQ_integral+AS _Vol.+emission_CO2+interval_O2; 0.352, 0.682, tmp_cumulo+pH +PL+PL_cumulo+AN+AN_cumulo+interval_yield+Feed_Vol+RS_cumul o+rab_integral+RQ_integral+AS_Vol.+emission_CO2; 0.341, 0.6 82, tmp_cumulo+pH+PL_cumulo+AN+AN_cumulo+interval_yield+RS+ Feed_Vol+RS_cumulo+rab_integral+RQ_integral+AS_Vol.+emissio n_CO2+consum_O2; 0.372, 0.682, tmp_cumulo+pH+AN_cumulo+inte rval_yield+RS+Feed_Vol+RS_cumulo+rab_integral+RQ_integral+A S_Vol.+emission_O2; 0.362, 0.682, tmp_cumulo+PL_cumulo+AN_c umulo+interval_yield+Feed_Vol+RS_cumulo+rab_integral+RQ_int egral+AS_Vol.+emission_O2+emission_CO2+consum_O2; 0.362, 0. 682, tmp+tmp_cumulo+PL_cumulo+AN_cumulo+interval_yield+Feed _Vol+RS_cumulo+rab_integral+RQ_integral+AS_Vol.+emission_CO 2+consum_O2; 0.382, 0.682, tmp_cumulo+PL_cumulo+AN+interval _yield+Feed_Vol+RS_cumulo+rab_integral+RQ_integral+AS_Vol.+ emission_CO2; 0.362, 0.682, tmp_cumulo+pH_cumulo+PL_cumulo+ AN_cumulo+interval_yield+interval_Pdt.+Feed_Vol+RS_cumulo+r ab_integral+RQ_integral+AS_Vol.+emission_O2; 0.352, 0.682, tmp_cumulo+pH+PL+PL_cumulo+AN_cumulo+interval_yield+RS+Feed _Vol+RS_cumulo+rab_integral+RQ_integral+AS_Vol.+emission_CO 2; 0.372, 0.682, tmp_cumulo+pH+PL+PL_cumulo+interval_yield+ Feed_Vol+RS_cumulo+rab_integral+RQ_integral+AS_Vol.+emissio n_CO2; 0.362, 0.682, tmp_cumulo+PL_cumulo+AN+AN_cumulo+inte rval_yield+RS+Feed_Vol+RS_cumulo+rab_integral+RQ_integral+A S_Vol.+emission_O2; 0.372, 0.682, tmp_cumulo+PL_cumulo+AN_c umulo+interval_yield+RS+Feed_Vol+RS_cumulo+AS_Vol.+emission _O2+consum_O2+interval_O2; 0.382, 0.682, tmp_cumulo+AN+AN_c umulo+interval_yield+RS+Feed_Vol+rab_integral+RQ_integral+A S_Vol.+emission_O2; 0.372, 0.682, tmp_cumulo+PL_cumulo+AN+i nterval_yield+interval_Pdt.+Feed_Vol+RS_cumulo+rab_integral +RQ_integral+AS_Vol.+emission_CO2; 0.372, 0.682, tmp_cumulo +PL_cumulo+interval_yield+interval_Pdt.+RS+Feed_Vol+RS_cumu lo+rab_integral+RQ_integral+AS_Vol.+emission_CO2; 0.372, 0. 682, pH_cumulo+PL+PL_cumulo+AN_cumulo+interval_yield+interv al_Pdt.+RS_cumulo+rab_integral+RQ_integral+AS_Vol.+emission _CO2; 0.362, 0.682, tmp+tmp_cumulo+AN_cumulo+interval_yield +interval_Pdt.+Feed_Vol+RS_cumulo+rab_integral+RQ_integral+ AS_Vol.+emission_CO2+consum_O2; 0.351, 0.682, tmp_cumulo+PL +PL_cumulo+AN+AN_cumulo+interval_yield+interval_Pdt.+Feed_V ol+RS_cumulo+rab_integral+RQ_integral+AS_Vol.+emission_CO2; 0.362, 0.682, tmp_cumulo+PL_cumulo+AN_cumulo+interval_yiel d+Feed_Vol+RS_cumulo+rab_integral+RQ_integral+Accum._Yield+ AS _Vol.+emission_CO2+consum_O2; 0.391, 0.682, tmp_cumulo+in terval_yield+interval_Pdt.+Feed_Vol+RS_cumulo+rab_integral+ RQ_integral+AS_Vol.+emission_CO2; 0.418, 0.682, PL_cumulo+i nterval_yield+RS_cumulo+RQ_integral+AS_Vol.+emission_CO2; 0. 351, 0.682, tmp_cumulo+PL+PL_cumulo+AN+AN_cumulo+interval_y ield+RS+Feed_Vol+RS_cumulo+rab_integral+RQ_integral+AS_Vol. +emission_CO2; 0.391, 0.682, tmp_cumulo+PL_cumulo+AN+AN_cum ulo+interval_yield+RS_cumulo+rab_integral+RQ_integral+emiss ion_CO2; 0.418, 0.682, tmp_cumulo+interval_yield+RS+Feed_Vo l+RS_cumulo+AS_Vol.; 0.409, 0.682, O2+PL_cumulo+interval_yi eld+RS+Feed_Vol+RS_cumulo+AS_Vol.; 0.351, 0.682, tmp_cumulo +PL+PL_cumulo+AN_cumulo+interval_yield+interval_Pdt.+RS+Fee d_Vol+RS_cumulo+rab_integral+RQ_integral+AS_Vol.+emission_C 02; 0.362, 0.682, tmp_cumulo+AN_cumulo+interval_yield+inter val_Pdt.+OD+Feed_Vol+RS_cumulo+rab_integral+RQ_integral+AS_ Vol.+emission_CO2+consum_O2; 0.391, 0.682, tmp_cumulo+PL_cu mulo+interval_yield+OD+RS+Feed_Vol+AS_Vol.+emission_O2+inte rval_O2; 0.372, 0.682, pH+PL_cumulo+AN_cumulo+interval_yiel d+interval_Pdt.+RS_cumulo+rab_integral+RQ_integral+AS_Vol.+ emission_CO2+consum_O2; 0.362, 0.682, tmp+tmp_cumulo+pH+PL_ cumulo+AN_cumulo+interval_yield+Feed_Vol+RS_cumulo+rab_inte gral+RQ_integral+AS_Vol.+emission_CO2; 0.351, 0.682, tmp_cu mulo+PL_cumulo+AN_cumulo+interval_yield+RS+Feed_Vol+RS_cumu lo+rab_integral+RQ_integral+AS_Vol.+emission_O2+consum_O2+i nterval_O2; 0.372, 0.682, tmp+tmp_cumulo+AN+AN_cumulo+inter val_yield+Feed_Vol+RS_cumulo+rab_integral+RQ_integral+AS_Vo l.+emission_CO2; 0.351, 0.682, tmp_cumulo+AN+AN_cumulo+inte rval_yield+OD+RS+Feed_Vol+RS_cumulo+rab_integral+RQ_integra l+AS_Vol.+emission_CO2+consum_O2; 0.372, 0.682, PL_cumulo+A N_cumulo+interval_yield+interval_Pdt.+RS_cumulo+rab_integra l+RQ_integral+AS_Vol.+emission_CO2+interval_O2+generate_CO 2; 0.340, 0.682, tmp_cumulo+pH+PL+PL_cumulo+AN_cumulo+inter val_yield+interval_Pdt.+Feed_Vol+RS_cumulo+rab_integral+RQ_ integral+AS_Vol.+emission_CO2+interval_O2; 0.351, 0.682, tm p_cumulo+AN+AN_cumulo+interval_yield+interval_Pdt.+Feed_Vol +RS_cumulo+rab_integral+RQ_integral+AS_Vol.+emission_CO2+co nsum_O2+interval_O2; 0.409, 0.682, tmp_cumulo+PL_cumulo+int erval_yield+RS+Feed_Vol+RS_cumulo+AS_Vol.; 0.381, 0.682, tm p_cumulo+PL_cumulo+AN_cumulo+interval_yield+RS+Feed_Vol+rab _integral+RQ_integral+AS_Vol.+emission_O2; 0.372, 0.682, tm p_cumulo+PL_cumulo+AN_cumulo+interval_yield+interval_Pdt.+O D+RS_cumulo+rab_integral+RQ_integral+AS_Vol.+emission_CO2; 0.340, 0.682, tmp+tmp_cumulo+PL_cumulo+AN_cumulo+interval_y ield+interval_Pdt.+Feed_Vol+RS_cumulo+rab_integral+RQ_integ ral+AS_Vol.+emission_CO2+consum_O2+interval_O2; 0.351, 0.68 2, tmp_cumulo+pH+PL_cumulo+AN_cumulo+interval_yield+Feed_Vo l+RS_cumulo+rab_integral+RQ_integral+AS_Vol.+emission_CO2+c onsum_O2+interval_O2; 0.361, 0.682, tmp+tmp_cumulo+AN_cumul o+interval_yield+RS+Feed_Vol+RS_cumulo+rab_integral+RQ_inte gral+AS_Vol.+emission_CO2+consum_O2; 0.361, 0.682, tmp+PL_c umulo+AN+AN_cumulo+interval_yield+interval_Pdt.+RS_cumulo+r ab_integral+RQ_integral+AS_Vol.+emission_CO2+interval_O2; 0. 361, 0.682, tmp_cumulo+pH_cumulo+AN_cumulo+interval_yield+i nterval_Pdt.+Feed_Vol+RS_cumulo+rab_integral+RQ_integral+AS _Vol.+emission_CO2+interval_O2; 0.371, 0.682, tmp_cumulo+pH +PL_cumulo+interval_yield+Feed_Vol+RS_cumulo+rab_integral+R Q_integral+AS_Vol.+emission_CO2+interval_O2; 0.371, 0.682, O2+PL_cumulo+AN+AN_cumulo+interval_yield+Feed_Vol+RS_cumulo +rab_integral+RQ_integral+AS_Vol.+emission_CO2; 0.391, 0.68 2, tmp_cumulo+interval_yield+RS+Feed_Vol+RS_cumulo+rab_inte gral+RQ_integral+AS_Vol.+emission_CO2; 0.400, 0.682, tmp_cu mulo+PL_cumulo+AN+AN_cumulo+interval_yield+RS_cumulo+RQ_int egral+emission_CO2; 0.400, 0.682, tmp_cumulo+PL+PL_cumulo+i nterval_yield+RS+Feed_Vol+RS_cumulo+AS_Vol.; 0.371, 0.682, tmp+tmp_cumulo+PL_cumulo+interval_yield+interval_Pdt.+Feed_ Vol+RS_cumulo+rab_integral+RQ_integral+AS_Vol.+emission_CO 2; 0.340, 0.682, tmp_cumulo+PL_cumulo+AN+AN_cumulo+interval _yield+RS+Feed_Vol+RS_cumulo+rab_integral+RQ_integral+AS_Vo l.+emission_O2+emission_CO2+consum_O2; 0.361, 0.682, pH+PL_ cumulo+AN_cumulo+interval_yield+interval_Pdt.+Feed_Vol+RS_c umulo+rab_integral+RQ_integral+AS_Vol.+emission_CO2+interva l_O2; 0.361, 0.682, tmp_cumulo+pH_cumulo+PL_cumulo+AN_cumul o+interval_yield+Feed_Vol+RS_cumulo+rab_integral+RQ_integra l+AS_Vol.+emission_CO2+consum_O2; 0.361, 0.682, tmp+PL+PL_c umulo+AN_cumulo+interval_yield+interval_Pdt.+Feed_Vol+RS_cu mulo+rab_integral+RQ_integral+AS_Vol.+emission_CO2; 0.371, 0.682, tmp_cumulo+PL_cumulo+AN_cumulo+interval_yield+Feed_V ol+RS_cumulo+rab_integral+RQ_integral+Accum._Yield+AS_Vol.+ emission_CO2; 0.371, 0.682, tmp_cumulo+pH+PL+AN_cumulo+inte rval_yield+Feed_Vol+RS_cumulo+rab_integral+RQ_integral+AS_V ol.+emission_CO2; 0.381, 0.682, tmp_cumulo+PL_cumulo+interv al_yield+interval_Pdt.+OD+RS_cumulo+rab_integral+RQ_integra l+AS_Vol.+emission_CO2; 0.340, 0.682, tmp_cumulo+pH+PL_cumu lo+AN+AN_cumulo+interval_yield+interval_Pdt.+Feed_Vol+RS_cu mulo+rab_integral+RQ_integral+AS_Vol.+emission_CO2+consum_O 2; 0.400, 0.682, O2+PL_cumulo+AN_cumulo+interval_yield+RS+F eed_Vol+AS_Vol.+interval_O2; 0.371, 0.682, tmp_cumulo+AN_cu mulo+interval_yield+OD+Feed_Vol+RS_cumulo+rab_integral+RQ_i ntegral+AS_Vol.+emission_CO2+interval_O2; 0.381, 0.682, tmp _cumulo+pH_cumulo+PL_cumulo+interval_yield+interval_Pdt.+RS _cumulo+rab_integral+RQ_integral+AS_Vol.+emission_CO2; 0.35 0, 0.682, tmp_cumulo+PL_cumulo+AN_cumulo+interval_yield+int erval_Pdt.+RS+Feed_Vol+RS_cumulo+rab_integral+RQ_integral+A S_Vol.+emission_O2+interval_O2; 0.409, 0.682, PL_cumulo+int erval_yield+Feed_Vol+RS_cumulo+RQ_integral+AS_Vol.+emission _CO2; 0.381, 0.682, PL_cumulo+AN_cumulo+interval_yield+RS+F eed_Vol+RS_cumulo+rab_integral+RQ_integral+AS_Vol.+emission _CO2; 0.371, 0.682, tmp_cumulo+PL_cumulo+AN+interval_yield+ interval_Pdt.+Feed_Vol+RS_cumulo+rab_integral+RQ_integral+A S_Vol.+emission_O2; 0.350, 0.682, tmp_cumulo+PL+PL_cumulo+A N_cumulo+interval_yield+RS+Feed_Vol+RS_cumulo+rab_integral+ RQ_integral+AS_Vol.+emission_CO2+consum_O2; 0.361, 0.681, t mp+tmp_cumulo+PL_cumulo+AN_cumulo+interval_yield+interval_P dt.+RS_cumulo+rab_integral+RQ_integral+AS_Vol.+emission_CO2 +consum_O2; 0.350, 0.681, tmp+tmp_cumulo+pH+PL_cumulo+AN_cu mulo+interval_yield+Feed_Vol+RS_cumulo+rab_integral+RQ_inte gral+AS_Vol.+emission_CO2+interval_O2; 0.350, 0.681, tmp+tm p_cumulo+PL_cumulo+AN+AN_cumulo+interval_yield+interval_Pdt. +RS_cumulo+rab_integral+RQ_integral+AS_Vol.+emission_CO2+in terval_O2; 0.350, 0.681, tmp_cumulo+AN+AN_cumulo+interval_y ield+RS+Feed_Vol+RS_cumulo+rab_integral+RQ_integral+AS_Vol. +emission_O2+emission_CO2+consum_O2; 0.390, 0.681, tmp_cumu lo+interval_yield+Feed_Vol+RS_cumulo+rab_integral+RQ_integr al+AS_Vol.+emission_CO2+interval_O2; 0.361, 0.681, tmp_cumu lo+AN_cumulo+interval_yield+interval_Pdt.+OD+Feed_Vol+RS_cu mulo+rab_integral+RQ_integral+AS_Vol.+emission_CO2+interval _O2; 0.371, 0.681, tmp+tmp_cumulo+pH+AN_cumulo+interval_yie ld+Feed_Vol+RS_cumulo+rab_integral+RQ_integral+AS_Vol.+emis sion_CO2; 0.381, 0.681, tmp+PL_cumulo+AN_cumulo+interval_yi eld+Feed_Vol+RS_cumulo+rab_integral+RQ_integral+AS_Vol.+emi ssion_CO2; 0.339, 0.681, tmp_cumulo+pH+PL_cumulo+AN+AN_cumu lo+interval_yield+interval_Pdt.+Feed_Vol+RS_cumulo+rab_inte gral+RQ_integral+AS_Vol.+emission_CO2+interval_O2; 0.371, 0. 681, tmp_cumulo+AN_cumulo+interval_yield+RS+Feed_Vol+RS_cum ulo+rab_integral+RQ_integral+AS_Vol.+consum_O2+generate_CO 2; 0.381, 0.681, tmp_cumulo+AN_cumulo+interval_yield+Feed_V ol+RS_cumulo+rab_integral+RQ_integral+AS_Vol.+emission_CO2+ generate_CO2; 0.350, 0.681, tmp_cumulo+PL_cumulo+AN_cumulo+ interval_yield+RS+Feed_Vol+RS_cumulo+rab_integral+RQ_integr al+AS_Vol.+emission_O2+emission_CO2+consum_02; 0.361, 0.681, tmp+tmp_cumulo+PL_cumulo+AN_cumulo+interval_yield+RS+Feed_ Vol+RS_cumulo+rab_integral+RQ_integral+AS_Vol.+emission_CO 2; 0.371, 0.681, PL_cumulo+AN_cumulo+interval_yield+RS+Feed _Vol+RS_cumulo+rab_integral+RQ_integral+AS_Vol.+emission_CO 2+interval_O2; 0.381, 0.681, tmp_cumulo+PL_cumulo+interval_ yield+OD+RS+Feed_Vol+RS_cumulo+AS_Vol.+emission_O2+interval _O2; 0.371, 0.681, PL_cumulo+AN_cumulo+interval_yield+inter val_Pdt.+RS_cumulo+rab_integral+RQ_integral+AS_Vol.+emissio n_O2+emission_CO2+interval_O2; 0.361, 0.681, tmp_cumulo+pH+ PL_cumulo+AN_cumulo+interval_yield+RS+Feed_Vol+RS_cumulo+ra b_integral+RQ_integral+AS_Vol.+emission_O2; 0.339, 0.681, t mp_cumulo+PL+PL_cumulo+AN+AN_cumulo+interval_yield+RS+Feed_ Vol+RS_cumulo+rab_integral+RQ_integral+AS_Vol.+emission_CO2 +consum_O2; 0.390, 0.681, PL_cumulo+AN+interval_yield+inter val_Pdt.+RS_cumulo+rab_integral+RQ_integral+AS_Vol.+emissio n_CO2; 0.399, 0.681, O2+PL_cumulo+interval_yield+RS+Feed_Vo l+RS_cumulo+AS_Vol.+interval_O2; 0.361, 0.681, pH+PL+PL_cum ulo+AN_cumulo+interval_yield+interval_Pdt.+Feed_Vol+RS_cumu lo+rab_integral+RQ_integral+AS_Vol.+emission_CO2; 0.380, 0. 681, tmp_cumulo+AN_cumulo+interval_yield+RS+Feed_Vol+RS_cum ulo+rab_integral+RQ_integral+AS_Vol.+consum_O2; 0.371, 0.68 1, tmp_cumulo+PL_cumulo+AN_cumulo+interval_yield+RS+Feed_Vo l+RS_cumulo+rab_integral+AS_Vol.+emission_O2+generate_CO2; 0.360, 0.681, tmp_cumulo+AN+AN_cumulo+interval_yield+interv al_Pdt.+RS+Feed_Vol+RS_cumulo+rab_integral+RQ_integral+AS_V ol.+emission_CO2; 0.371, 0.681, tmp_cumulo+AN_cumulo+interv al_yield+RS+Feed_Vol+RS_cumulo+rab_integral+AS_Vol.+emissio n_O2+consum_O2+interval_O2; 0.360, 0.681, tmp+tmp_cumulo+AN _cumulo+interval_yield+RS+Feed_Vol+RS_cumulo+rab_integral+R Q_integral+AS_Vol.+emission_CO2+interval_O2; 0.360, 0.681, tmp_cumulo+PL_cumulo+AN_cumulo+interval_yield+RS+Feed_Vol+R S_cumulo+rab_integral+RQ_integral+AS_Vol.+emission_02+emiss ion_CO2; 0.390, 0.681, O2+PL_cumulo+interval_yield+OD+RS+Fe ed_Vol+AS_Vol.+emission_O2+interval_O2; 0.370, 0.681, tmp_c umulo+pH_cumulo+AN+AN_cumulo+interval_yield+Feed_Vol+RS_cum ulo+rab_integral+RQ_integral+AS_Vol.+emission_CO2; 0.339, 0. 681, tmp_cumulo+pH+PL_cumulo+AN_cumulo+interval_yield+inter val_Pdt.+RS+Feed_Vol+RS_cumulo+rab_integral+RQ_integral+AS_ Vol.+emission_CO2+consum_O2; 0.370, 0.681, tmp_cumulo+pH_cu mulo+PL_cumulo+AN_cumulo+interval_yield+interval_Pdt.+RS_cu mulo+rab_integral+RQ_integral+AS_Vol.+emission_CO2; 0.360, 0.681, PL_cumulo+AN+AN_cumulo+interval_yield+interval_Pdt.+ RS_cumulo+rab_integral+RQ_integral+AS_Vol.+emission_CO2+con sum_O2+interval_O2; 0.390, 0.681, tmp_cumulo+PL_cumulo+inte rval_yield+Feed_Vol+RS_cumulo+RQ_integral+AS_Vol.+emission_ CO2+interval_O2; 0.360, 0.681, pH+PL+PL_cumulo+AN_cumulo+in terval_yield+interval_Pdt.+RS_cumulo+rab_integral+RQ_integr al+AS_Vol.+emission_CO2+interval_O2; 0.370, 0.681, tmp+tmp_ cumulo+AN_cumulo+interval_yield+RS+Feed_Vol+RS_cumulo+rab_i ntegral+RQ_integral+AS_Vol.+emission_O2; 0.350, 0.681, tmp_ cumulo+pH+AN_cumulo+interval_yield+interval_Pdt.+RS+Feed_Vo l+RS_cumulo+rab_integral+RQ_integral+AS_Vol.+emission_CO2+c onsum_O2; 0.370, 0.681, tmp_cumulo+AN+AN_cumulo+interval_yi eld+interval_Pdt.+RS_cumulo+rab_integral+RQ_integral+AS_Vol. +emission_CO2+interval_O2; 0.350, 0.681, tmp+tmp_cumulo+PL_ cumulo+AN+AN_cumulo+interval_yield+Feed_Vol+RS_cumulo+rab_i ntegral+RQ_integral+AS_Vol.+emission_CO2+interval_O2; 0.339, 0.681, tmp_cumulo+PL_cumulo+AN+AN_cumulo+interval_yield+in terval_Pdt.+RS+Feed_Vol+RS_cumulo+rab_integral+RQ_integral+ AS_Vol.+emission_CO2+interval_O2; 0.380, 0.681, tmp+PL+PL_c umulo+interval_yield+interval_Pdt.+RS_cumulo+rab_integral+R Q_integral+AS_Vol.+emission_CO2; 0.350, 0.681, tmp+tmp_cumu lo+PL_cumulo+AN_cumulo+interval_yield+RS+Feed_Vol+RS_cumulo +rab_integral+RQ_integral+AS_Vol.+emission_CO2+interval_O2; 0.350, 0.681, tmp_cumulo+PL+PL_cumulo+AN_cumulo+interval_y ield+interval_Pdt.+OD+Feed_Vol+RS_cumulo+rab_integral+RQ_in tegral+AS_Vol.+emission_CO2; 0.390, 0.681, tmp_cumulo+PL+PL _cumulo+interval_yield+interval_Pdt.+RS_cumulo+RQ_integral+ AS_Vol.+emission_CO2; 0.339, 0.681, tmp_cumulo+pH+PL_cumulo +AN_cumulo+interval_yield+interval_Pdt.+RS+Feed_Vol+RS_cumu lo+rab_integral+RQ_integral+AS_Vol.+emission_CO2+interval_O 2; 0.360, 0.681, tmp_cumulo+pH+AN+AN_cumulo+interval_yield+ RS+Feed_Vol+RS_cumulo+rab_integral+RQ_integral+AS_Vol.+emis sion_CO2; 0.370, 0.681, tmp+PL_cumulo+AN_cumulo+interval_yi eld+Feed_Vol+RS_cumulo+rab_integral+RQ_integral+AS_Vol.+emi ssion_CO2+interval_O2; 0.360, 0.681, tmp_cumulo+pH_cumulo+P L_cumulo+AN_cumulo+interval_yield+RS+Feed_Vol+RS_cumulo+rab _integral+RQ_integral+AS_Vol.+emission_CO2; 0.350, 0.681, t mp_cumulo+pH_cumulo+PL_cumulo+AN_cumulo+interval_yield+RS+F eed_Vol+RS_cumulo+rab_integral+RQ_integral+AS_Vol.+emission _CO2+interval_O2; 0.360, 0.681, pH+PL_cumulo+AN+AN_cumulo+i nterval_yield+interval_Pdt.+RS_cumulo+rab_integral+RQ_integ ral+AS_Vol.+emission_CO2+interval_O2; 0.339, 0.681, tmp_cum ulo+PL+PL_cumulo+AN+AN_cumulo+interval_yield+interval_Pdt.+ Feed_Vol+RS_cumulo+rab_integral+RQ_integral+AS_Vol.+emissio n_CO2+consum_O2; 0.389, 0.681, tmp_cumulo+pH+PL_cumulo+inte rval_yield+Feed_Vol+RS_cumulo+RQ_integral+AS_Vol.+emission_ CO2; 0.370, 0.681, tmp_cumulo+AN+AN_cumulo+interval_yield+R S+Feed_Vol+rab_integral+RQ_integral+AS_Vol.+emission_O2+con sum_O2; 0.339, 0.681, tmp_cumulo+PL_cumulo+AN_cumulo+interv al_yield+interval_Pdt.+RS+Feed_Vol+RS_cumulo+rab_integral+R Q_integral+AS_Vol.+emission_O2+consum_O2+interval_O2; 0.370, 0.681, tmp_cumulo+PL_cumulo+interval_yield+interval_Pdt.+R S+Feed_Vol+RS_cumulo+rab_integral+RQ_integral+AS_Vol.+emiss ion_O2; 0.380, 0.681, pH+PL_cumulo+AN_cumulo+interval_yield +Feed_Vol+RS_cumulo+rab_integral+RQ_integral+AS_Vol.+emissi on_CO2; 0.360, 0.681, tmp+PL_cumulo+AN_cumulo+interval_yiel d+interval_Pdt.+RS_cumulo+rab_integral+RQ_integral+AS_Vol.+ emission_CO2+consum_O2+interval_O2; 0.349, 0.681, tmp_cumul o+PL_cumulo+AN+AN_cumulo+interval_yield+Feed_Vol+RS_cumulo+ rab_integral+RQ_integral+AS_Vol.+emission_O2+emission-CO2+c onsum_O2; 0.370, 0.681, tmp_cumulo+pH+PL_cumulo+interval_yi eld+Feed_Vol+RS_cumulo+rab_integral+RQ_integral+AS_Vol.+emi ssion_O2+emission_CO2; 0.360, 0.681, tmp+PL_cumulo+AN_cumul o+interval_yield+interval_Pdt.+RS+RS_cumulo+rab_integral+RQ _integral+AS_Vol.+emission_CO2+interval_O2; 0.370, 0.681, t mp_cumulo+pH_cumulo+PL_cumulo+interval_yield+interval_Pdt.+ Feed_Vol+RS_cumulo+rab_integral+RQ_integral+AS_Vol.+emissio n_CO2; 0.349, 0.681, tmp_cumulo+AN+AN_cumulo+interval_yield +interval_Pdt.+RS+Feed_Vol+RS_cumulo+rab_integral+RQ_integr al+AS_Vol.+emission_CO2+interval_O2; 0.360, 0.681, tmp_cumu lo+pH+PL+PL_cumulo+AN_cumulo+interval_yield+interval_Pdt.+R S_cumulo+rab_integral+RQ_integral+AS_Vol.+emission_CO2; 0.3 70, 0.681, tmp_cumulo+PL_cumulo+interval_yield+RS+Feed_Vol+ RS_cumulo+rab_integral+RQ_integral+AS_Vol.+emission_O2+emis sion_CO2; 0.338, 0.681, tmp+tmp_cumulo+pH+PL_cumulo+AN_cumu lo+interval_yield+interval_Pdt.+Feed_Vol+RS_cumulo+rab_inte gral+RQ_integral+AS_Vol.+emission_CO2+interval_O2; 0.360, 0. 681, tmp_cumulo+PL_cumulo+AN_cumulo+interval_yield+OD+RS+Fe ed_Vol+RS_cumulo+rab_integral+RQ_integral+AS_Vol.+emission_ CO2; 0.370, 0.681, tmp_cumulo+PL+AN_cumulo+interval_yield+F eed_Vol+RS_cumulo+rab_integral+RQ_integral+AS_Vol.+emission _CO2+consum_O2; 0.370, 0.681, PL_cumulo+AN_cumulo+interval_ yield+interval_Pdt.+OD+Feed_Vol+RS_cumulo+rab_integral+RQ_i ntegral+AS_Vol.+emission_CO2; 0.360, 0.681, tmp_cumulo+AN_c umulo+interval_yield+OD+RS+Feed_Vol+RS_cumulo+rab_integral+ RQ_integral+AS_Vol.+emission_CO2+consum_O2; 0.360, 0.681, t mp_cumulo+PL+AN_cumulo+interval_yield+interval_Pdt.+Feed_Vo l+RS_cumulo+rab_integral+RQ_integral+AS_Vol.+emission_CO2+i nterval_O2; 0.349, 0.681, tmp_cumulo+pH+PL_cumulo+AN+AN_cum ulo+interval_yield+interval_Pdt.+RS_cumulo+rab_integral+RQ_ integral+AS_Vol.+emission_CO2+interval_O2; 0.349, 0.681, tm p+tmp_cumulo+PL+PL_cumulo+AN_cumulo+interval_yield+interval _Pdt.+Feed_Vol+RS_cumulo+rab_integral+RQ_integral+AS_Vol.+e mission_CO2; 0.398, 0.681, PL_cumulo+interval_yield+interva l_Pdt.+RS_cumulo+RQ_integral+AS_Vol.+emission_CO2+interval_ 02; 0.360, 0.681, tmp_cumulo+PL+PL_cumulo+AN_cumulo+interva l_yield+OD+Feed_Vol+RS_cumulo+rab_integral+RQ_integral+AS_V ol.+emission_CO2; 0.360, 0.681, tmp+tmp_cumulo+PL_cumulo+AN +AN_cumulo+interval_yield+Feed_Vol+RS_cumulo+rab_integral+R Q_integral+AS_Vol.+emission_CO2; 0.370, 0.681, PL_cumulo+AN _cumulo+interval_yield+interval_Pdt.+RS+RS_cumulo+rab_integ ral+RQ_integral+AS_Vol.+emission_CO2+consum_O2; 0.360, 0.68 1, tmp_cumulo+AN+AN_cumulo+interval_yield+OD+Feed_Vol+RS_cu mulo+rab_integral+RQ_integral+AS_Vol.+emission_CO2+consum_O 2; 0.389, 0.681, pH_cumulo+PL+PL_cumulo+interval_yield+OD+R S+Feed_Vol+AS_Vol.+interval_O2; 0.349, 0.681, tmp_cumulo+PL +PL_cumulo+AN_cumulo+interval_yield+Feed_Vol+RS_cumulo+rab_ integral+RQ_integral+AS_Vol.+emission_CO2+consum_O2+interva l_O2; 0.360, 0.681, tmp_cumulo+pH_cumulo+AN_cumulo+interval _yield+RS+Feed_Vol+RS_cumulo+rab_integral+RQ_integral+AS_Vo l.+emission_CO2+interval_O2; 0.370, 0.681, tmp_cumulo+pH_cu mulo+AN_cumulo+interval_yield+RS+Feed_Vol+RS_cumulo+rab_int egral+RQ_integral+AS_Vol.+emission_O2; 0.389, 0.681, tmp_cu mulo+PL_cumulo+interval_yield+RS+Feed_Vol+RS_cumulo+RQ_inte gral+AS_Vol.+emission_CO2; 0.359, 0.681, tmp_cumulo+PL_cumu lo+AN_cumulo+interval_yield+OD+Feed_Vol+RS_cumulo+rab_integ ral+RQ_integral+AS_Vol.+emission_CO2+consum_O2; 0.398, 0.68 1, O2+PL_cumulo+interval_yield+Feed_Vol+RS_cumulo+RQ_integr al+AS_Vol.+emission_CO2; 0.370, 0.681, tmp_cumulo+PL_cumulo +AN_cumulo+interval_yield+interval_Pdt.+RS_cumulo+rab_integ ral+RQ_integral+AS_Vol.+emission_CO2+generate_CO2; 0.359, 0. 681, tmp_cumulo+PL+AN_cumulo+interval_yield+interval_Pdt.+F eed_Vol+RS_cumulo+rab_integral+RQ_integral+AS_Vol.+emission _CO2+consum_O2; 0.370, 0.681, tmp+tmp_cumulo+AN_cumulo+inte rval_yield+Feed_Vol+RS_cumulo+rab_integral+RQ_integral+AS_V ol.+emission_CO2+consum_O2; 0.379, 0.681, tmp_cumulo+AN_cum ulo+interval_yield+Feed_Vol+RS_cumulo+rab_integral+RQ_integ ral+AS_Vol.+emission_O2+emission_CO2; 0.359, 0.681, tmp+tmp _cumulo+PL+PL_cumulo+AN_cumulo+interval_yield+interval_Pdt. +RS_cumulo+rab_integral+RQ_integral+AS_Vol.+emission_CO2; 0. 379, 0.681, tmp_cumulo+PL_cumulo+interval_yield+OD+RS+Feed_ Vol+AS_Vol.+emission_O2+consum_O2+interval_02; 0.379, 0.681, tmp_cumulo+PL_cumulo+interval_yield+interval_Pdt.+RS_cumul o+rab_integral+RQ_integral+AS_Vol.+emission_CO2+generate_CO 2; 0.349, 0.681, tmp_cumulo+pH_cumulo+PL_cumulo+AN+AN_cumul o+interval_yield+Feed_Vol+RS_cumulo+rab_integral+RQ_integra l+AS_Vol.+emission_CO2+interval_O2; 0.338, 0.681, tmp_cumul o+AN+AN_cumulo+interval_yield+interval_Pdt.+RS+Feed_Vol+RS_ cumulo+rab_integral+RQ_integral+AS_Vol.+emission_CO2+consum _O2+interval_O2; 0.369, 0.681, tmp_cumulo+PL_cumulo+AN_cumu lo+interval_yield+Feed_Vol+RS_cumulo+rab_integral+RQ_integr al+AS_Vol.+emission_CO2+generate_CO2; 0.359, 0.681, tmp_cum ulo+pH+PL_cumulo+AN_cumulo+interval_yield+interval_Pdt.+Fee d_Vol+RS_cumulo+rab_integral+RQ_integral+AS_Vol.+emission_O 2; 0.389, 0.681, tmp_cumulo+PL_cumulo+interval_yield+Feed_V ol+RS_cumulo+rab_integral+RQ_integral+AS_Vol.+emission_O2; 0.338, 0.681, tmp_cumulo+PL_cumulo+AN_cumulo+interval_yield +interval_Pdt.+Feed_Vol+RS_cumulo+rab_integral+RQ_integral+ AS_Vol.+emission_O2+emission_CO2+consum_O2+interval_02; 0.3 89, 0.681, pH+PL_cumulo+interval_yield+interval_Pdt.+RS_cum ulo+rab_integral+RQ_integral+AS_Vol.+emission_CO2; 0.416, 0. 681, O2+PL_cumulo+interval_yield+RS+Feed_Vol+AS_Vol.; 0.369, 0.680, tmp_cumulo+AN+AN_cumulo+interval_yield+OD+Feed_Vol+ RS_cumulo+rab_integral+RQ_integral+AS_Vol.+emission_CO2; 0. 379, 0.680, tmp_cumulo+PL_cumulo+interval_yield+OD+Feed_Vol +RS_cumulo+rab_integral+RQ_integral+AS_Vol.+emission_CO2; 0. 369, 0.680, tmp_cumulo+PL_cumulo+interval_yield+OD+RS+Feed_ Vol+RS_cumulo+AS_Vol.+emission_O2+consum _O2+interval_02; 0. 379, 0.680, tmp_cumulo+PL_cumulo+AN_cumulo+interval_yield+R S+Feed_Vol+RS_cumulo+AS_Vol.+emission_O2+interval_O2; 0.379, 0.680, O2+PL_cumulo+AN_cumulo+interval_yield+RS+Feed_Vol+r ab_integral+RQ_integral+AS_Vol.+emission_CO2; 0.369, 0.680, tmp_cumulo+PL_cumulo+interval_yield+interval_Pdt.+OD+Feed_ Vol+RS_cumulo+rab_integral+RQ_integral+AS_Vol.+emission_CO 2; 0.348, 0.680, tmp+tmp_cumulo+PL_cumulo+AN_cumulo+interva l_yield+RS+Feed_Vol+RS_cumulo+rab_integral+RQ_integral+AS_V ol.+emission_CO2+consum_02; 0.398, 0.680, O2+PL_cumulo+AN_c umulo+interval_yield+RS+Feed_Vol+AS_Vol.+emission_CO2; 0.34 8, 0.680, tmp_cumulo+PL_cumulo+AN+AN_cumulo+interval_yield+ interval_Pdt.+RS+RS_cumulo+rab_integral+RQ_integral+AS_Vol. +emission_CO2+interval_O2; 0.369, 0.680, pH+PL_cumulo+AN_cu mulo+interval_yield+Feed_Vol+RS_cumulo+rab_integral+RQ_inte gral+AS_Vol.+emission_CO2+interval_O2; 0.389, 0.680, tmp_cu mulo+PL_cumulo+interval_yield+interval_Pdt.+Feed_Vol+RS_cum ulo+RQ_integral+AS_Vol.+emission_CO2; 0.389, 0.680, PL_cumu lo+interval_yield+interval_Pdt.+RS_cumulo+rab_integral+RQ_i ntegral+AS_Vol.+emission_O2+emission_CO2; 0.369, 0.680, tmp _cumulo+pH+PL_cumulo+interval_yield+RS+Feed_Vol+RS_cumulo+r ab_integral+RQ_integral+AS_Vol.+emission_CO2; 0.348, 0.680, tmp_cumulo+PL+AN+AN_cumulo+interval_yield+RS+Feed_Vol+RS_c umulo+rab_integral+RQ_integral+AS_Vol.+emission_CO2+consum_ 02; 0.359, 0.680, tmp_cumulo+pH+PL_cumulo+AN_cumulo+interva l_yield+interval_Pdt.+RS_cumulo+rab_integral+RQ_integral+AS _Vol.+emission_CO2+consum_02; 0.369, 0.680, tmp_cumulo+PL+P L_cumulo+interval_yield+Feed_Vol+RS_cumulo+rab_integral+RQ_ integral+AS_Vol.+emission_CO2+interval_O2; 0.359, 0.680, tm p_cumulo+PL_cumulo+AN_cumulo+interval_yield+interval_Pdt.+R S_cumulo+rab_integral+RQ_integral+AS_Vol.+emission_CO2+inte rval_O2+generate_CO2; 0.348, 0.680, tmp+tmp_cumulo+PL_cumul o+AN+AN_cumulo+interval_yield+Feed_Vol+RS_cumulo+rab_integr al+RQ_integral+AS_Vol.+emission_CO2+consum_O2; 0.359, 0.680, tmp_cumulo+pH+PL_cumulo+AN_cumulo+interval_yield+OD+Feed_V ol+RS_cumulo+rab_integral+RQ_integral+AS_Vol.+emission_CO2; 0.398, 0.680, PL_cumulo+AN_cumulo+interval_yield+Feed_Vol+ RS_cumulo+RQ_integral+AS_Vol.+emission_CO2; 0.359, 0.680, t mp_cumulo+PL_cumulo+AN_cumulo+interval_yield+interval_Pdt.+ RS_cumulo+rab_integral+RQ_integral+AS_Vol.+emission_O2+emis sion_CO2+interval_O2; 0.359, 0.680, PL_cumulo+AN_cumulo+int erval_yield+interval_Pdt.+Feed_Vol+RS_cumulo+rab_integral+R Q_integral+AS_Vol.+emission_CO2+consum_O2+interval_O2; 0.35 9, 0.680, tmp_cumulo+PL_cumulo+AN+AN_cumulo+interval_yield+ RS+Feed_Vol+rab_integral+RQ_integral+AS_Vol.+emission_CO2+c onsum_O2; 0.348, 0.680, tmp_cumulo+PL_cumulo+AN+AN_cumulo+i nterval_yield+Feed_Vol+RS_cumulo+rab_integral+RQ_integral+A S_Vol.+emission_O2+emission_CO2+interval_02; 0.369, 0.680, tmp_cumulo+AN_cumulo+interval_yield+OD+Feed_Vol+RS_cumulo+r ab_integral+RQ_integral+AS_Vol.+emission_CO2+consum_O2; 0.3 48, 0.680, tmp_cumulo+AN_cumulo+interval_yield+interval_Pdt. +RS+Feed_Vol+RS_cumulo+rab_integral+RQ_integral+AS_Vol.+emi ssion_O2+consum_O2+interval_O2; 0.398, 0.680, O2+PL_cumulo+ AN_cumulo+interval_yield+RS+Feed_Vol+AS_Vol.+consum_O2; 0.3 48, 0.680, tmp_cumulo+pH_cumulo+PL+PL_cumulo+AN_cumulo+inte rval_yield+interval_Pdt.+Feed_Vol+RS_cumulo+rab_integral+RQ _integral+AS_Vol.+emission_CO2; 0.359, 0.680, tmp_cumulo+PL _cumulo+AN_cumulo+interval_yield+interval_Pdt.+Feed_Vol+RS_ cumulo+rab_integral+RQ_integral+AS_Vol.+emission_O2+interva l_O2; 0.359, 0.680, tmp+tmp_cumulo+AN+AN_cumulo+interval_yi eld+Feed_Vol+RS_cumulo+rab_integral+RQ_integral+AS_Vol.+emi ssion_CO2+consum_O2; 0.337, 0.680, tmp_cumulo+PL_cumulo+AN_ cumulo+interval_yield+interval_Pdt.+RS+Feed_Vol+RS_cumulo+r ab_integral+RQ_integral+AS_Vol.+emission_O2+emission_CO2+co nsum_O2; 0.348, 0.680, tmp_cumulo+PL_cumulo+AN+AN_cumulo+in terval_yield+Feed_Vol+RS_cumulo+rab_integral+RQ_integral+Ac cum._Yield+AS_Vol.+emission_CO2+consum_O2; 0.398, 0.680, PL _cumulo+interval_yield+interval_Pdt.+OD+RS_cumulo+RQ_integr al+AS_Vol.+emission_CO2; 0.359, 0.680, PL_cumulo+AN_cumulo+ interval_yield+interval_Pdt.+OD+Feed_Vol+RS_cumulo+rab_inte gral+RQ_integral+AS_Vol.+emission_CO2+interval_O2; 0.359, 0. 680, tmp+pH+PL_cumulo+AN_cumulo+interval_yield+interval_Pdt. +RS_cumulo+rab_integral+RQ_integral+AS_Vol.+emission_CO2+in terval_O2; 0.348, 0.680, tmp_cumulo+pH+AN+AN_cumulo+interva l_yield+interval_Pdt.+Feed_Vol+RS_cumulo+rab_integral+RQ_in tegral+AS_Vol.+emission_CO2+consum_O2; 0.359, 0.680, tmp_cu mulo+pH_cumulo+PL_cumulo+AN_cumulo+interval_yield+RS+Feed_V ol+RS_cumulo+rab_integral+RQ_integral+AS_Vol.+emission_O2; 0.379, 0.680, tmp_cumulo+AN_cumulo+interval_yield+Feed_Vol+ RS_cumulo+rab_integral+RQ_integral+Accum._Yield+AS_Vol.+emi ssion_CO2; 0.359, 0.680, tmp_cumulo+AN_cumulo+interval_yiel d+interval_Pdt.+RS+Feed_Vol+RS_cumulo+rab_integral+RQ_integ ral+AS_Vol.+emission_O2+interval_O2; 0.388, 0.680, tmp_cumu lo+PL_cumulo+interval_yield+Feed_Vol+RS_cumulo+RQ_integral+ AS_Vol.+emission_O2+emission_CO2; 0.369, 0.680, tmp_cumulo+ PL+PL_cumulo+interval_yield+RS+Feed_Vol+RS_cumulo+rab_integ ral+RQ_integral+AS_Vol.+emission_CO2; 0.348, 0.680, tmp_cum ulo+PL+PL_cumulo+AN+AN_cumulo+interval_yield+interval_Pdt.+ RS_cumulo+rab_integral+RQ_integral+AS_Vol.+emission_CO2+int erval_O2; 0.359, 0.680, tmp_cumulo+AN_cumulo+interval_yield +RS+Feed_Vol+RS_cumulo+rab_integral+RQ_integral+AS_Vol.+emi ssion_O2+emission_CO2+consum_O2; 0.369, 0.680, pH_cumulo+PL _cumulo+AN_cumulo+interval_yield+interval_Pdt.+RS_cumulo+ra b_integral+RQ_integral+AS_Vol.+emission_CO2+consum_O2; 0.36 9, 0.680, tmp_cumulo+AN_cumulo+interval_yield+RS+Feed_Vol+R S_cumulo+rab_integral+RQ_integral+AS_Vol.+emission_O2+gener ate_CO2; 0.369, 0.680, O2+PL_cumulo+AN_cumulo+interval_yiel d+interval_Pdt.+RS_cumulo+rab_integral+RQ_integral+AS_Vol.+ emission_CO2+generate_CO2; 0.369, 0.680, tmp+PL_cumulo+AN_c umulo+interval_yield+interval_Pdt.+Feed_Vol+RS_cumulo+rab_i ntegral+RQ_integral+AS_Vol.+emission_CO2; 0.337, 0.680, tmp _cumulo+PL_cumulo+AN_cumulo+interval_yield+interval_Pdt.+OD +Feed_Vol+RS_cumulo+rab_integral+RQ_integral+AS_Vol.+emissi on_CO2+consum_O2+interval_O2; 0.358, 0.680, tmp_cumulo+pH+P L_cumulo+AN_cumulo+interval_yield+Feed_Vol+RS_cumulo+rab_in tegral+RQ_integral+AS_Vol.+emission_O2+emission_CO2; 0.379, 0.680, tmp_cumulo+AN_cumulo+interval_yield+RS+Feed_Vol+rab _integral+RQ_integral+AS_Vol.+emission_O2+consum _O2; 0.337, 0.680, tmp+tmp_cumulo+PL_cumulo+AN+AN_cumulo+interval_yiel d+RS+Feed_Vol+RS_cumulo+rab_integral+RQ_integral+AS_Vol.+em ission_CO2+consum_O2; 0.358, 0.680, PL_cumulo+AN_cumulo+int erval_yield+interval_Pdt.+RS+Feed_Vol+RS_cumulo+rab_integra l+RQ_integral+AS_Vol.+emission_CO2+interval_O2; 0.358, 0.68 0, O2+PL_cumulo+AN_cumulo+interval_yield+interval_Pdt.+RS+R S_cumulo+rab_integral+RQ_integral+AS_Vol.+emission_CO2+inte rval_O2; 0.388, 0.680, tmp_cumulo+AN_cumulo+interval_yield+ RS+Feed_Vol+rab_integral+RQ_integral+AS_Vol.+consum_O2; 0.3 58, 0.680, tmp_cumulo+PL+AN_cumulo+interval_yield+RS+Feed_V ol+RS_cumulo+rab_integral+RQ_integral+AS_Vol.+emission_CO2+ consum_O2; 0.378, 0.680, tmp_cumulo+PL_cumulo+AN+AN_cumulo+ interval_yield+RS+RS_cumulo+rab_integral+RQ_integral+emissi on_CO2; 0.348, 0.680, tmp_cumulo+pH+AN_cumulo+interval_yiel d+RS+Feed_Vol+RS_cumulo+rab_integral+RQ_integral+AS_Vol.+em ission_CO2+consum_O2+interval_O2; 0.358, 0.680, tmp+PL+PL_c umulo+AN+AN_cumulo+interval_yield+interval_Pdt.+RS_cumulo+r ab_integral+RQ_integral+AS_Vol.+emission_CO2; 0.358, 0.680, tmp_cumulo+PL+PL_cumulo+AN_cumulo+interval_yield+interval_ Pdt.+RS_cumulo+rab_integral+RQ_integral+AS_Vol.+emission_CO 2+consum_O2; 0.369, 0.680, tmp_cumulo+PL_cumulo+interval_yi eld+RS+Feed_Vol+RS_cumulo+rab_integral+RQ_integral+AS_Vol.+ emission_CO2+interval_O2; 0.388, 0.680, tmp_cumulo+AN_cumul o+interval_yield+RS+Feed_Vol+RS_cumulo+rab_integral+RQ_inte gral+AS_Vol.; 0.388, 0.680, tmp_cumulo+PL_cumulo+AN_cumulo+ interval_yield+Feed_Vol+RS_cumulo+RQ_integral+AS_Vol.+emiss ion_CO2; 0.358, 0.680, PL_cumulo+AN+AN_cumulo+interval_yiel d+interval_Pdt.+RS+RS_cumulo+rab_integral+RQ_integral+AS_Vo l.+emission_CO2+interval_O2; 0.388, 0.680, PL_cumulo+AN_cum ulo+interval_yield+interval_Pdt.+RS_cumulo+RQ_integral+AS_V ol.+emission_CO2+interval_O2; 0.358, 0.680, tmp_cumulo+AN+A N_cumulo+interval_yield+RS+Feed_Vol+rab_integral+RQ_integra l+AS_Vol.+emission_O2+consum_O2+interval_02; 0.337, 0.680, tmp_cumulo+PL+PL_cumulo+AN+AN_cumulo+interval_yield+interva l_Pdt.+Feed_Vol+RS_cumulo+rab_integral+RQ_integral+AS_Vol.+ emission_CO2+interval_O2; 0.337, 0.680, tmp_cumulo+pH_cumul o+PL_cumulo+AN_cumulo+interval_yield+interval_Pdt.+Feed_Vol +RS_cumulo+rab_integral+RQ_integral+AS_Vol.+emission_CO2+co nsum_O2+interval_O2; 0.337, 0.680, tmp_cumulo+pH+PL_cumulo+ AN_cumulo+interval_yield+interval_Pdt.+OD+Feed_Vol+RS_cumul o+rab_integral+RQ_integral+AS_Vol.+emission_CO2+interval_O 2; 0.358, 0.680, tmp_cumulo+pH_cumulo+AN_cumulo+interval_yi eld+interval_Pdt.+Feed_Vol+RS_cumulo+rab_integral+RQ_integr al+AS_Vol.+emission_CO2+consum_O2; 0.358, 0.680, pH+PL+PL_c umulo+AN+AN_cumulo+interval_yield+interval_Pdt.+RS_cumulo+r ab_integral+RQ_integral+AS_Vol.+emission_CO2; 0.358, 0.680, tmp_cumulo+pH+AN_cumulo+interval_yield+interval_Pdt.+RS+Fe ed_Vol+RS_cumulo+rab_integral+RQ_integral+AS_Vol.+emission_ CO2; 0.368, 0.680, tmp_cumulo+PL_cumulo+AN+AN_cumulo+interv al_yield+RS+Feed_Vol+rab_integral+RQ_integral+AS_Vol.+emiss ion_O2; 0.397, 0.680, tmp_cumulo+interval_yield+RS+Feed_Vol +RS_cumulo+AS_Vol.+emission_O2+interval_O2; 0.358, 0.680, t mp_cumulo+PL_cumulo+AN+AN_cumulo+interval_yield+Feed_Vol+RS _cumulo+rab_integral+RQ_integral+AS_Vol.+emission_O2+emissi on_CO2; 0.348, 0.680, tmp_cumulo+pH+AN+AN_cumulo+interval_y ield+RS+Feed_Vol+RS_cumulo+rab_integral+RQ_integral+AS_Vol. +emission_CO2+interval_O2; 0.326, 0.680, tmp_cumulo+PL_cumu lo+AN+AN_cumulo+interval_yield+interval_Pdt.+RS+Feed_Vol+RS _cumulo+rab_integral+RQ_integral+AS_Vol.+emission_CO2+consu m_O2+interval_O2; 0.348, 0.680, tmp_cumulo+PL_cumulo+AN_cum ulo+interval_yield+RS+Feed_Vol+RS_cumulo+rab_integral+RQ_in tegral+AS_Vol.+emission_O2+emission_CO2+interval_O2; 0.348, 0.680, tmp_cumulo+pH+PL_cumulo+AN_cumulo+interval_yield+in terval_Pdt.+OD+Feed_Vol+RS_cumulo+rab_integral+RQ_integral+ AS_Vol.+emission_CO2; 0.388, 0.680, PL_cumulo+interval_yiel d+OD+RS+Feed_Vol+AS_Vol.+emission_O2+consum_O2+interval_O2; 0.358, 0.680, tmp+tmp_cumulo+PL_cumulo+AN_cumulo+interval_ yield+interval_Pdt.+Feed_Vol+RS_cumulo+rab_integral+RQ_inte gral+AS_Vol.+emission_O2; 0.358, 0.680, tmp_cumulo+pH_cumul o+PL_cumulo+AN+AN_cumulo+interval_yield+Feed_Vol+RS_cumulo+ rab_integral+RQ_integral+AS_Vol.+emission_CO2; 0.348, 0.680, tmp_cumulo+pH_cumulo+AN+AN_cumulo+interval_yield+RS+Feed_V ol+RS_cumulo+rab_integral+RQ_integral+AS_Vol.+emission_CO2+ consum_O2; 0.368, 0.680, tmp_cumulo+pH_cumulo+AN_cumulo+int erval_yield+Feed_Vol+RS_cumulo+rab_integral+RQ_integral+AS_ Vol.+emission_CO2+consum_O2; 0.358, 0.680, tmp_cumulo+PL_cu mulo+AN_cumulo+interval_yield+interval_Pdt.+RS+RS_cumulo+ra b_integral+RQ_integral+AS_Vol.+emission_CO2+consum_O2; 0.35 8, 0.680, tmp+tmp_cumulo+AN+AN_cumulo+interval_yield+Feed_V ol+RS_cumulo+rab_integral+RQ_integral+AS_Vol.+emission_CO2+ interval_O2; 0.358, 0.680, O2+PL_cumulo+AN_cumulo+interval_ yield+interval_Pdt.+OD+Feed_Vol+RS_cumulo+rab_integral+RQ_i ntegral+AS_Vol.+emission_CO2; 0.358, 0.680, tmp+tmp_cumulo+ PL_cumulo+AN_cumulo+interval_yield+RS+Feed_Vol+RS_cumulo+ra b_integral+RQ_integral+AS_Vol.+emission_O2; 0.368, 0.680, t mp_cumulo+AN_cumulo+interval_yield+Feed_Vol+RS_cumulo+rab_i ntegral+RQ_integral+Accum._Yield+AS_Vol.+emission_CO2+consu m_O2; 0.337, 0.680, tmp_cumulo+PL_cumulo+AN+AN_cumulo+inter val_yield+interval_Pdt.+Feed_Vol+RS_cumulo+rab_integral+RQ_ integral+AS_Vol.+emission_O2+emission_CO2+consum_O2; 0.358, 0.680, tmp+PL+PL_cumulo+AN_cumulo+interval_yield+RS+Feed_V ol+RS_cumulo+rab_integral+RQ_integral+AS_Vol.+emission_CO2; 0.337, 0.680, tmp_cumulo+pH+AN+AN_cumulo+interval_yield+RS +Feed_Vol+RS_cumulo+rab_integral+RQ_integral+AS_Vol.+emissi on_CO2+consum_O2+interval_O2; 0.337, 0.680, tmp_cumulo+PL+P L_cumulo+AN_cumulo+interval_yield+interval_Pdt.+RS+Feed_Vol +RS_cumulo+rab_integral+RQ_integral+AS_Vol.+emission_CO2+co nsum_O2; 0.358, 0.680, tmp_cumulo+PL+AN+AN_cumulo+interval_ yield+Feed_Vol+RS_cumulo+rab_integral+RQ_integral+AS_Vol.+e mission_CO2+consum_O2; 0.368, 0.680, PL_cumulo+AN+AN_cumulo +interval_yield+Feed_Vol+RS_cumulo+rab_integral+RQ_integral +AS_Vol.+emission_CO2+interval_O2; 0.388, 0.680, PL_cumulo+ interval_yield+interval_Pdt.+OD+RS_cumulo+rab_integral+RQ_i ntegral+AS_Vol.+emission_CO2; 0.388, 0.680, PL_cumulo+AN_cu mulo+interval_yield+RS+Feed_Vol+RS_cumulo+AS_Vol.+emission_ O2+emission_CO2; 0.397, 0.680, tmp_cumulo+AN_cumulo+interva l_yield+RS+Feed_Vol+RS_cumulo+AS_Vol.+emission_O2; 0.358, 0. 680, pH+PL_cumulo+AN_cumulo+interval_yield+interval_Pdt.+RS _cumulo+rab_integral+RQ_integral+AS_Vol.+emission_CO2+consu m_O2+interval_O2; 0.358, 0.680, tmp+pH_cumulo+PL_cumulo+AN_ cumulo+interval_yield+interval_Pdt.+RS_cumulo+rab_integral+ RQ_integral+AS_Vol.+emission_CO2+interval_O2; 0.378, 0.680, PL_cumulo+AN+AN_cumulo+interval_yield+Feed_Vol+RS_cumulo+r ab_integral+RQ_integral+AS_Vol.+emission_CO2; 0.397, 0.680, O2+PL_cumulo+interval_yield+RS_cumulo+RQ_integral+AS_Vol.+ emission_O2+emission_CO2; 0.358, 0.680, O2+PL_cumulo+AN_cum ulo+interval_yield+interval_Pdt.+RS+Feed_Vol+RS_cumulo+rab_ integral+RQ_integral+AS_Vol.+emission_CO2; 0.336, 0.680, tm p_cumulo+pH+PL+PL_cumulo+AN_cumulo+interval_yield+interval_ Pdt.+Feed_Vol+RS_cumulo+rab_integral+RQ_integral+AS_Vol.+em ission_CO2+consum_O2; 0.347, 0.680, tmp_cumulo+PL_cumulo+AN +AN_cumulo+interval_yield+OD+Feed_Vol+RS_cumulo+rab_integra 1+RQ-integral+AS-Vol.+emission-CO2+consum-O2; 0.387, 0.680, PL_cumulo+AN_cumulo+interval_yield+OD+RS+Feed_Vol+AS_Vol.+ emission_O2+interval_O2; 0.358, 0.680, tmp_cumulo+PL+PL_cum ulo+AN_cumulo+interval_yield+interval_Pdt.+RS+RS_cumulo+rab _integral+RQ_integral+AS_Vol.+emission_CO2; 0.397, 0.680, P L_cumulo+interval_yield+interval_Pdt.+RS_cumulo+RQ_integral +AS_Vol.+emission_O2+emission_CO2; 0.387, 0.680, tmp_cumulo +interval_yield+RS+Feed_Vol+RS_cumulo+rab_integral+RQ_integ ral+AS_Vol.+emission_O2; 0.358, 0.680, tmp_cumulo+AN_cumulo +interval_yield+interval_Pdt.+RS+Feed_Vol+RS_cumulo+rab_int egral+RQ_integral+AS_Vol.+consum_O2+generate_CO2; 0.368, 0. 680, tmp_cumulo+PL_cumulo+AN+AN_cumulo+interval_yield+inter val_Pdt.+RS_cumulo+rab_integral+RQ_integral+emission_CO2+in terval_O2; 0.368, 0.680, tmp+PL_cumulo+AN+AN_cumulo+interva l_yield+interval_Pdt.+RS_cumulo+rab_integral+RQ_integral+AS _Vol.+emission_CO2; 0.397, 0.680, tmp+PL_cumulo+interval_yi eld+interval_Pdt.+RS_cumulo+RQ_integral+AS_Vol.+emission_CO 2; 0.368, 0.680, PL_cumulo+AN_cumulo+interval_yield+interva l_Pdt.+OD+RS_cumulo+rab_integral+RQ_integral+AS_Vol.+emissi on_CO2+consum_O2; 0.336, 0.680, tmp_cumulo+PL+PL_cumulo+AN_ cumulo+interval_yield+interval_Pdt.+RS+Feed_Vol+RS_cumulo+r ab_integral+RQ_integral+AS_Vol.+emission_CO2+interval_O2; 0. 387, 0.680, tmp_cumulo+interval_yield+RS+Feed_Vol+RS_cumulo +AS_Vol.+emission_O2+consum_O2+interval_02; 0.336, 0.680, t mp+tmp_cumulo+PL_cumulo+AN+AN_cumulo+interval_yield+interva l_Pdt.+Feed_Vol+RS_cumulo+rab_integral+RQ_integral+AS_Vol.+ emission_CO2+consum_O2; 0.397, 0.680, tmp_cumulo+PL_cumulo+ interval_yield+Feed_Vol+RS_cumulo+AS_Vol.+emission_O2+inter val_O2; 0.378, 0.680, tmp_cumulo+AN_cumulo+interval_yield+i nterval_Pdt.+RS+RS_cumulo+rab_integral+RQ_integral+AS_Vol.+ emission_CO2; 0.358, 0.679, tmp_cumulo+PL_cumulo+AN+AN_cumu lo+interval_yield+OD+Feed_Vol+RS_cumulo+rab_integral+RQ_int egral+AS_Vol.+emission_CO2; 0.378, 0.679, PL_cumulo+AN_cumu lo+interval_yield+Feed_Vol+RS_cumulo+rab_integral+RQ_integr al+AS_Vol.+emission_CO2+consum_O2; 0.378, 0.679, O2+PL_cumu lo+AN_cumulo+interval_yield+OD+RS+Feed_Vol+AS_Vol.+consum_O 2+interval_O2; 0.396, 0.679, tmp_cumulo+interval_yield+inte rval_Pdt.+RS_cumulo+rab_integral+RQ_integral+AS_Vol.+emissi on_CO2; 0.357, 0.679, PL_cumulo+AN+AN_cumulo+interval_yield +interval_Pdt.+Feed_Vol+RS_cumulo+rab_integral+RQ_integral+ AS_Vol.+emission_CO2+interval_O2; 0.387, 0.679, tmp_cumulo+ pH+interval_yield+Feed_Vol+RS_cumulo+rab_integral+RQ_integr al+AS_Vol.+emission_CO2; 0.357, 0.679, tmp+tmp_cumulo+PL_cu mulo+AN+AN_cumulo+interval_yield+interval_Pdt.+RS_cumulo+ra b_integral+RQ_integral+AS_Vol.+emission_CO2; 0.357, 0.679, tmp+tmp_cumulo+AN+AN_cumulo+interval_yield+RS+Feed_Vol+RS_c umulo+rab_integral+RQ_integral+AS_Vol.+emission_CO2; 0.396, 0.679, tmp_cumulo+PL_cumulo+interval_yield+RS+Feed_Vol+RS_ cumulo+AS_Vol.+emission_CO2; 0.405, 0.679, PL_cumulo+AN_cum ulo+interval_yield+RS+Feed_Vol+AS_Vol.+emission_O2; 0.357, 0.679, tmp_cumulo+AN+AN_cumulo+interval_yield+Feed_Vol+RS_c umulo+rab_integral+RQ_integral+Accum._Yield+AS_Vol.+emissio n_CO2+consum_O2; 0.387, 0.679, tmp_cumulo+PL+PL_cumulo+AN+A N_cumulo+interval_yield+RS_cumulo+RQ_integral+emission_CO2; 0.357, 0.679, tmp_cumulo+pH_cumulo+AN_cumulo+interval_yiel d+RS+Feed_Vol+RS_cumulo+rab_integral+RQ_integral+AS_Vol.+em ission_CO2+consum_O2; 0.357, 0.679, tmp_cumulo+PL_cumulo+AN _cumulo+interval_yield+interval_Pdt.+Feed_Vol+RS_cumulo+rab _integral+RQ_integral+AS_Vol.+emission_O2+consum _O2; 0.336, 0.679, tmp+tmp_cumulo+PL_cumulo+AN+AN_cumulo+interval_yiel d+interval_Pdt.+Feed_Vol+RS_cumulo+rab_integral+RQ_integral +AS_Vol.+emission_CO2+interval_O2; 0.357, 0.679, tmp_cumulo +AN_cumulo+interval_yield+interval_Pdt.+OD+RS+Feed_Vol+RS_c umulo+rab_integral+RQ_integral+AS_Vol.+emission_CO2; 0.387, 0.679, tmp_cumulo+PL_cumulo+interval_yield+interval_Pdt.+R S_cumulo+RQ_integral+AS_Vol.+emission_CO2+interval_O2; 0.34 7, 0.679, tmp_cumulo+pH+PL_cumulo+AN_cumulo+interval_yield+ OD+Feed_Vol+RS_cumulo+rab_integral+RQ_integral+AS_Vol.+emis sion_CO2+interval_O2; 0.368, 0.679, tmp_cumulo+pH+AN_cumulo +interval_yield+OD+Feed_Vol+RS_cumulo+rab_integral+RQ_integ ral+AS_Vol.+emission_CO2; 0.357, 0.679, tmp_cumulo+PL+AN_cu mulo+interval_yield+RS+Feed_Vol+RS_cumulo+rab_integral+RQ_i ntegral+AS_Vol.+emission_CO2+interval_O2; 0.336, 0.679, tmp _cumulo+pH+PL_cumulo+AN_cumulo+interval_yield+RS+Feed_Vol+R S_cumulo+rab_integral+RQ_integral+AS_Vol.+emission_CO2+cons um_O2+interval_O2; 0.405, 0.679, tmp _cumulo+interval_yield+ RS+Feed_Vol+RS_cumulo+AS_Vol.+emission_O2; 0.336, 0.679, tm p_cumulo+PL_cumulo+AN_cumulo+interval_yield+interval_Pdt.+F eed_Vol+RS_cumulo+rab_integral+RQ_integral+Accum._Yield+AS_ Vol.+emission_CO2+consum_O2+interval_O2; 0.347, 0.679, tmp+ tmp_cumulo+AN_cumulo+interval_yield+interval_Pdt.+RS+Feed_V ol+RS_cumulo+rab_integral+RQ_integral+AS_Vol.+emission_CO2+ consum_O2; 0.387, 0.679, tmp_cumulo+interval_yield+Feed_Vol +RS_cumulo+rab_integral+RQ_integral+AS_Vol.+emission_O2+emi ssion_CO2; 0.387, 0.679, O2+PL_cumulo+interval_yield+RS+Fee d_Vol+RS_cumulo+AS_Vol.+consum_O2+interval_O2; 0.336, 0.679, tmp+tmp_cumulo+PL_cumulo+AN_cumulo+interval_yield+interval _Pdt.+RS+Feed_Vol+RS_cumulo+rab_integral+RQ_integral+AS_Vol. +emission_CO2+consum_O2; 0.396, 0.679, PL_cumulo+interval_y ield+Feed_Vol+RS_cumulo+rab_integral+RQ_integral+AS_Vol.+em ission_CO2; 0.357, 0.679, tmp_cumulo+pH+AN_cumulo+interval_ yield+Feed_Vol+RS_cumulo+rab_integral+RQ_integral+AS_Vol.+e mission_CO2+consum_O2+interval_O2; 0.347, 0.679, tmp_cumulo +pH+PL_cumulo+AN_cumulo+interval_yield+Feed_Vol+RS_cumulo+r ab_integral+RQ_integral+AS_Vol.+emission_O2+emission_CO2+in terval_O2; 0.336, 0.679, tmp+tmp_cumulo+PL_cumulo+AN_cumulo +interval_yield+interval_Pdt.+RS+Feed_Vol+RS_cumulo+rab_int egral+RQ_integral+AS_Vol.+emission_CO2+interval_O2; 0.387, 0.679, tmp_cumulo+PL_cumulo+interval_yield+interval_Pdt.+RS +Feed_Vol+RS_cumulo+AS_Vol.+emission_O2; 0.347, 0.679, tmp_ cumulo+pH_cumulo+PL+PL_cumulo+AN_cumulo+interval_yield+Feed _Vol+RS_cumulo+rab_integral+RQ_integral+AS_Vol.+emission_CO 2+interval_O2; 0.367, 0.679, pH+PL_cumulo+AN+AN_cumulo+inte rval_yield+interval_Pdt.+RS_cumulo+rab_integral+RQ_integral +AS_Vol.+emission_CO2; 0.357, 0.679, pH+pH_cumulo+PL_cumulo +AN_cumulo+interval_yield+interval_Pdt.+RS_cumulo+rab_integ ral+RQ_integral+AS_Vol.+emission_CO2+interval_O2; 0.347, 0. 679, tmp+tmp_cumulo+pH+PL+PL_cumulo+AN_cumulo+interval_yiel d+Feed_Vol+RS_cumulo+rab_integral+RQ_integral+AS_Vol.+emiss ion_CO2; 0.367, 0.679, tmp_cumulo+PL_cumulo+interval_yield+ RS+Feed_Vol+RS_cumulo+rab_integral+RQ_integral+AS_Vol.+emis sion_O2+interval_O2; 0.367, 0.679, tmp_cumulo+AN_cumulo+int erval_yield+Feed_Vol+RS_cumulo+rab_integral+RQ_integral+Acc um._Yield+AS_Vol.+emission_CO2+interval_O2; 0.367, 0.679, t mp_cumulo+AN+AN_cumulo+interval_yield+interval_Pdt.+RS_cumu lo+rab_integral+RQ_integral+AS_Vol.+emission_CO2+consum_O2; 0.367, 0.679, tmp_cumulo+AN_cumulo+interval_yield+Feed_Vol +RS_cumulo+rab_integral+RQ_integral+AS_Vol.+emission_O2+emi ssion_CO2+consum_O2; 0.357, 0.679, O2+PL_cumulo+AN_cumulo+i nterval_yield+interval_Pdt.+RS_cumulo+rab_integral+RQ_integ ral+AS_Vol.+emission_CO2+consum_O2+interval_O2; 0.357, 0.67 9, tmp_cumulo+pH_cumulo+AN+AN_cumulo+interval_yield+Feed_Vo l+RS_cumulo+rab_integral+RQ_integral+AS_Vol.+emission_CO2+i nterval_O2; 0.377, 0.679, tmp_cumulo+PL_cumulo+AN_cumulo+in terval_yield+RS+Feed_Vol+RS_cumulo+AS_Vol.+emission_O2+emis sion_CO2; 0.367, 0.679, pH+PL_cumulo+AN_cumulo+interval_yie ld+interval_Pdt.+Feed_Vol+RS_cumulo+rab_integral+RQ_integra l+AS_Vol.+emission_CO2; 0.367, 0.679, tmp_cumulo+pH+PL+PL_c umulo+interval_yield+interval_Pdt.+RS_cumulo+rab_integral+R Q_integral+AS_Vol.+emission_CO2; 0.387, 0.679, tmp_cumulo+P L_cumulo+interval_yield+OD+Feed_Vol+RS_cumulo+RQ_integral+A S_Vol.+emission_CO2; 0.357, 0.679, tmp+PL+PL_cumulo+AN_cumu lo+interval_yield+interval_Pdt.+RS+RS_cumulo+rab_integral+R Q_integral+AS_Vol.+emission_CO2; 0.367, 0.679, tmp+tmp_cumu lo+PL_cumulo+interval_yield+interval_Pdt.+RS_cumulo+rab_int egral+RQ_integral+AS_Vol.+emission_CO2+interval_O2; 0.377, 0.679, tmp_cumulo+PL_cumulo+AN+interval_yield+interval_Pdt. +RS_cumulo+rab_integral+RQ_integral+AS_Vol.+emission_CO2; 0. 387, 0.679, tmp_cumulo+PL_cumulo+AN+AN_cumulo+interval_yiel d+RS+RS_cumulo+RQ_integral+emission_CO2; 0.336, 0.679, tmp_ cumulo+pH+AN+AN_cumulo+interval_yield+interval_Pdt.+RS+Feed _Vol+RS_cumulo+rab_integral+RQ_integral+AS_Vol.+emission_CO 2+consum_O2; 0.387, 0.679, PL_cumulo+AN_cumulo+interval_yie ld+RS+Feed_Vol+RS_cumulo+AS_Vol.+emission_CO2+consum_O2; 0. 377, 0.679, O2+PL_cumulo+interval_yield+interval_Pdt.+RS_cu mulo+rab_integral+RQ_integral+AS_Vol.+emission_CO2+interval _O2; 0.367, 0.679, tmp_cumulo+AN_cumulo+interval_yield+RS+F eed_Vol+RS_cumulo+rab_integral+RQ_integral+AS_Vol.+emission _CO2+generate_CO2; 0.357, 0.679, pH_cumulo+PL_cumulo+AN_cum ulo+interval_yield+interval_Pdt.+Feed_Vol+RS_cumulo+rab_int egral+RQ_integral+AS_Vol.+emission_CO2+interval_O2; 0.396, 0.679, O2+PL_cumulo+AN_cumulo+interval_yield+OD+RS+Feed_Vol +AS_Vol.; 0.357, 0.679, tmp_cumulo+PL+AN+AN_cumulo+interval _yield+RS+Feed_Vol+RS_cumulo+rab_integral+RQ_integral+AS_Vo l.+emission_CO2; 0.367, 0.679, tmp_cumulo+PL_cumulo+AN+AN_c umulo+interval_yield+interval_Pdt.+RS+RS_cumulo+rab_integra l+RQ_integral+emission_CO2; 0.367, 0.679, tmp+PL_cumulo+AN_ cumulo+interval_yield+interval_Pdt.+RS+RS_cumulo+rab_integr al+RQ_integral+AS_Vol.+emission_CO2; 0.357, 0.679, tmp_cumu lo+AN+AN_cumulo+interval_yield+Feed_Vol+RS_cumulo+rab_integ ral+RQ_integral+AS_Vol.+emission_O2+emission_CO2+consum_O2; 0.346, 0.679, tmp_cumulo+pH_cumulo+PL_cumulo+AN+AN_cumulo+ interval_yield+Feed_Vol+RS_cumulo+rab_integral+RQ_integral+ AS_Vol.+emission_CO2+consum_O2; 0.367, 0.679, tmp_cumulo+AN _cumulo+interval_yield+Feed_Vol+RS_cumulo+rab_integral+RQ_i ntegral+AS_Vol.+emission_O2+emission_CO2+interval_O2; 0.357, 0.679, PL_cumulo+AN+AN_cumulo+interval_yield+interval_Pdt. +OD+RS_cumulo+rab_integral+RQ_integral+AS_Vol.+emission_CO2 +interval_O2; 0.346, 0.679, tmp_cumulo+PL_cumulo+AN+AN_cumu lo+interval_yield+interval_Pdt.+OD+RS_cumulo+rab_integral+R Q_integral+AS_Vol.+emission_CO2+interval_O2; 0.357, 0.679, tmp_cumulo+AN+AN_cumulo+interval_yield+RS+Feed_Vol+RS_cumul o+rab_integral+RQ_integral+AS_Vol.+emission_O2+consum_O2; 0. 346, 0.679, tmp_cumulo+pH+AN_cumulo+interval_yield+interval _Pdt.+Feed_Vol+RS_cumulo+rab_integral+RQ_integral+AS_Vol.+e mission_CO2+consum_O2+interval_O2; 0.357, 0.679, tmp_cumulo +PL_cumulo+AN+AN_cumulo+interval_yield+interval_Pdt.+Feed_V ol+RS_cumulo+rab_integral+RQ_integral+AS_Vol.+emission_O2; 0.377, 0.679, tmp_cumulo+AN_cumulo+interval_yield+RS+Feed_V ol+RS_cumulo+rab_integral+AS_Vol.+emission_O2+generate_CO2; 0.405, 0.679, PL_cumulo+interval_yield+RS+Feed_Vol+RS_cumu lo+AS_Vol.+emission_O2; 0.357, 0.679, O2+PL_cumulo+AN+AN_cu mulo+interval_yield+interval_Pdt.+RS+RS_cumulo+rab_integral +RQ_integral+AS_Vol.+emission_CO2; 0.357, 0.679, O2+PL_cumu lo+AN_cumulo+interval_yield+interval_Pdt.+Feed_Vol+RS_cumul o+rab_integral+RQ_integral+AS_Vol.+emission_CO2+consum_O2; 0.346, 0.679, tmp+tmp_cumulo+pH+PL_cumulo+AN_cumulo+interva l_yield+interval_Pdt.+Feed_Vol+RS_cumulo+rab_integral+RQ_in tegral+AS_Vol.+emission_CO2; 0.367, 0.679, pH_cumulo+PL+PL_ cumulo+AN_cumulo+interval_yield+OD+RS+Feed_Vol+AS_Vol.+cons um_O2+interval_O2; 0.386, 0.679, O2+PL_cumulo+AN_cumulo+int erval_yield+RS+Feed_Vol+RS_cumulo+AS_Vol.+emission_O2; 0.37 7, 0.679, tmp+tmp_cumulo+AN_cumulo+interval_yield+interval_ Pdt.+RS_cumulo+rab_integral+RQ_integral+AS_Vol.+emission_CO 2; 0.346, 0.679, tmp_cumulo+AN_cumulo+interval_yield+interv al_Pdt.+OD+RS+Feed_Vol+RS_cumulo+rab_integral+RQ_integral+A S_Vol.+emission_CO2+consum_O2; 0.335, 0.679, tmp+tmp_cumulo +PL+PL_cumulo+AN_cumulo+interval_yield+interval_Pdt.+Feed_V ol+RS_cumulo+rab_integral+RQ_integral+AS_Vol.+emission_CO2+ interval_O2; 0.335, 0.679, tmp_cumulo+pH+PL_cumulo+AN+AN_cu mulo+interval_yield+RS+Feed_Vol+RS_cumulo+rab_integral+RQ_i ntegral+AS_Vol.+emission_CO2+interval_O2; 0.367, 0.679, pH+ PL+PL_cumulo+AN_cumulo+interval_yield+RS+Feed_Vol+rab_integ ral+RQ_integral+AS_Vol.+emission_CO2; 0.357, 0.679, tmp_cum ulo+pH+PL_cumulo+AN+AN_cumulo+interval_yield+interval_Pdt.+ RS_cumulo+rab_integral+RQ_integral+AS_Vol.+emission_CO2; 0. 396, 0.679, pH+PL_cumulo+interval_yield+interval_Pdt.+RS_cu mulo+RQ_integral+AS_Vol.+emission_CO2; 0.357, 0.679, tmp_cu mulo+PL+AN+AN_cumulo+interval_yield+Feed_Vol+RS_cumulo+rab_ integral+RQ_integral+AS_Vol.+emission_CO2+interval_O2; 0.35 7, 0.679, tmp_cumulo+pH_cumulo+AN+AN_cumulo+interval_yield+ Feed_Vol+RS_cumulo+rab_integral+RQ_integral+AS_Vol.+emissio n_CO2+consum_O2; 0.377, 0.679, tmp_cumulo+PL+PL_cumulo+AN_c umulo+interval_yield+Feed_Vol+RS_cumulo+RQ_integral+AS_Vol. +emission_CO2; 0.367, 0.679, tmp_cumulo+pH+PL_cumulo+interv al_yield+interval_Pdt.+RS_cumulo+rab_integral+RQ_integral+A S_Vol.+emission_CO2+interval_O2; 0.346, 0.679, tmp_cumulo+p H+PL_cumulo+AN_cumulo+interval_yield+interval_Pdt.+RS+Feed_ Vol+RS_cumulo+rab_integral+RQ_integral+AS_Vol.+emission_O2; 0.377, 0.679, tmp_cumulo+AN_cumulo+interval_yield+interval _Pdt.+RS_cumulo+rab_integral+RQ_integral+AS_Vol.+emission_C O2+consum_O2; 0.357, 0.679, O2+PL_cumulo+AN+AN_cumulo+inter val_yield+interval_Pdt.+RS_cumulo+rab_integral+RQ_integral+ AS_Vol.+emission_CO2+consum_O2; 0.357, 0.679, pH_cumulo+PL_ cumulo+AN+AN_cumulo+interval_yield+interval_Pdt.+RS_cumulo+ rab_integral+RQ_integral+AS_Vol.+emission_CO2+interval_O2; 0.367, 0.679, tmp_cumulo+PL_cumulo+interval_yield+Feed_Vol+ RS_cumulo+rab_integral+RQ_integral+AS_Vol.+emission_CO2+con sum_O2+interval_O2; 0.346, 0.679, tmp+tmp_cumulo+PL_cumulo+ AN_cumulo+interval_yield+interval_Pdt.+RS_cumulo+rab_integr al+RQ_integral+AS_Vol.+emission_CO2+consum_O2+interval_O2; 0.377, 0.679, O2+PL_cumulo+AN+interval_yield+interval_Pdt.+ RS_cumulo+rab_integral+RQ_integral+AS_Vol.+emission_CO2; 0. 356, 0.679, tmp+PL_cumulo+AN_cumulo+interval_yield+interval _Pdt.+OD+RS_cumulo+rab_integral+RQ_integral+AS_Vol.+emissio n_CO2+interval_O2; 0.395, 0.679, PL_cumulo+interval_yield+R S+Feed_Vol+RS_cumulo+AS_Vol.+emission_O2+interval_O2; 0.346, 0.679, tmp_cumulo+PL_cumulo+AN_cumulo+interval_yield+inter val_Pdt.+OD+RS+Feed_Vol+RS_cumulo+rab_integral+RQ_integral+ AS_Vol.+emission_CO2; 0.356, 0.679, tmp_cumulo+pH+AN+AN_cum ulo+interval_yield+interval_Pdt.+Feed_Vol+RS_cumulo+rab_int egral+RQ_integral+AS_Vol.+emission_CO2; 0.356, 0.679, tmp_c umulo+AN_cumulo+interval_yield+interval_Pdt.+Feed_Vol+RS_cu mulo+rab_integral+RQ_integral+Accum._Yield+AS_Vol.+emission _CO2+consum_O2; 0.356, 0.679, tmp_cumulo+pH+PL+PL_cumulo+in terval_yield+interval_Pdt.+Feed_Vol+RS_cumulo+rab_integral+ RQ_integral+AS_Vol.+emission_CO2; 0.346, 0.679, tmp_cumulo+ pH+AN_cumulo+interval_yield+interval_Pdt.+RS+Feed_Vol+RS_cu mulo+rab_integral+RQ_integral+AS_Vol.+emission_CO2+interval _O2; 0.367, 0.679, O2+PL_cumulo+AN+AN_cumulo+interval_yield +RS+Feed_Vol+rab_integral+RQ_integral+AS_Vol.+emission_CO2; 0.346, 0.679, tmp_cumulo+PL_cumulo+AN_cumulo+interval_yiel d+OD+RS+Feed_Vol+RS_cumulo+rab_integral+RQ_integral+AS_Vol. +emission_CO2+consum_O2; 0.386, 0.679, PL_cumulo+AN_cumulo+ interval_yield+RS+Feed_Vol+rab_integral+RQ_integral+AS_Vol. +emission_O2; 0.367, 0.679, tmp_cumulo+PL+PL_cumulo+AN+AN_c umulo+interval_yield+interval_Pdt.+RS_cumulo+rab_integral+R Q_integral+emission_CO2; 0.346, 0.679, tmp_cumulo+PL_cumulo +AN+AN_cumulo+interval_yield+OD+Feed_Vol+RS_cumulo+rab_inte gral+RQ_integral+AS_Vol.+emission_CO2+interval_O2; 0.356, 0. 679, tmp+tmp_cumulo+AN_cumulo+interval_yield+interval_Pdt.+ RS+Feed_Vol+RS_cumulo+rab_integral+RQ_integral+AS_Vol.+emis sion_CO2; 0.395, 0.679, tmp_cumulo+interval_yield+RS+Feed_V ol+RS_cumulo+rab_integral+RQ_integral+AS_Vol.; 0.356, 0.679, tmp_cumulo+AN_cumulo+interval_yield+RS+Feed_Vol+RS_cumulo+ rab_integral+AS_Vol.+emission_O2+consum_O2+interval_O2+gene rate_CO2; 0.346, 0.679, tmp_cumulo+pH+PL_cumulo+AN_cumulo+i nterval_yield+interval_Pdt.+RS+Feed_Vol+RS_cumulo+rab_integ ral+RQ_integral+AS_Vol.+emission_CO2; 0.366, 0.679, tmp_cum ulo+pH_cumulo+PL+AN_cumulo+interval_yield+Feed_Vol+RS_cumul o+rab_integral+RQ_integral+AS_Vol.+emission_CO2; 0.335, 0.6 79, tmp_cumulo+pH+PL_cumulo+AN_cumulo+interval_yield+interv al_Pdt.+Feed_Vol+RS_cumulo+rab_integral+RQ_integral+AS_Vol. +emission_O2+emission_CO2+interval_O2; 0.386, 0.679, tmp_cu mulo+PL_cumulo+interval_yield+interval_Pdt.+RS_cumulo+RQ_in tegral+AS_Vol.+emission_O2+emission_CO2; 0.335, 0.679, tmp_ cumulo+PL_cumulo+AN_cumulo+interval_yield+interval_Pdt.+OD+ RS+Feed_Vol+RS_cumulo+rab_integral+RQ_integral+AS_Vol.+emis sion_CO2+consum_O2; 0.346, 0.679, tmp+tmp_cumulo+PL+PL_cumu lo+AN_cumulo+interval_yield+Feed_Vol+RS_cumulo+rab_integral +RQ_integral+AS_Vol.+emission_CO2+interval_O2; 0.376, 0.679, tmp_cumulo+AN_cumulo+interval_yield+RS+Feed_Vol+rab_integr al+RQ_integral+AS_Vol.+consum_O2+generate_CO2; 0.356, 0.679, tmp+pH+PL+PL_cumulo+AN_cumulo+interval_yield+interval_Pdt. +RS_cumulo+rab_integral+RQ_integral+AS_Vol.+emission_CO2; 0. 395, 0.679, tmp_cumulo+interval_yield+RS+Feed_Vol+RS_cumulo +AS_Vol.+consum_O2+interval_O2; 0.335, 0.679, tmp_cumulo+pH +PL_cumulo+AN+AN_cumulo+interval_yield+Feed_Vol+RS_cumulo+r ab_integral+RQ_integral+AS_Vol.+emission_CO2+consum_O2+inte rval_O2; 0.346, 0.679, tmp+tmp_cumulo+PL_cumulo+AN_cumulo+i nterval_yield+interval_Pdt.+OD+Feed_Vol+RS_cumulo+rab_integ ral+RQ_integral+AS_Vol.+emission_CO2; 0.345, 0.679, tmp_cum ulo+pH_cumulo+PL_cumulo+AN_cumulo+interval_yield+interval_P dt.+RS+Feed_Vol+RS_cumulo+rab_integral+RQ_integral+AS_Vol.+ emission_O2; 0.366, 0.679, tmp_cumulo+pH_cumulo+PL_cumulo+i nterval_yield+Feed_Vol+RS_cumulo+rab_integral+RQ_integral+A S_Vol.+emission_CO2+interval_O2; 0.386, 0.679, tmp_cumulo+p H+PL_cumulo+interval_yield+interval_Pdt.+RS_cumulo+RQ_integ ral+AS_Vol.+emission_CO2; 0.356, 0.679, tmp_cumulo+AN+AN_cu mulo+interval_yield+interval_Pdt.+RS+Feed_Vol+RS_cumulo+rab _integral+RQ_integral+AS_Vol.+emission_O2; 0.366, 0.679, tm p_cumulo+AN_cumulo+interval_yield+interval_Pdt.+Feed_Vol+RS _cumulo+rab_integral+RQ_integral+AS_Vol.+emission_CO2+gener ate_CO2; 0.366, 0.679, PL_cumulo+AN_cumulo+interval_yield+i nterval_Pdt.+RS_cumulo+rab_integral+RQ_integral+AS_Vol.+emi ssion_CO2+consum_O2+generate_CO2; 0.376, 0.679, PL_cumulo+A N_cumulo+interval_yield+OD+RS+Feed_Vol+AS_Vol.+emission_O2+ consum_O2+interval_O2; 0.356, 0.679, O2+PL_cumulo+AN_cumulo +interval_yield+interval_Pdt.+OD+RS_cumulo+rab_integral+RQ_ integral+AS_Vol.+emission_CO2+interval_O2; 0.356, 0.679, tm p_cumulo+PL_cumulo+AN+interval_yield+interval_Pdt.+Feed_Vol +RS_cumulo+rab_integral+RQ_integral+AS_Vol.+emission_CO2+in terval_O2; 0.345, 0.679, tmp+tmp_cumulo+PL_cumulo+AN_cumulo +interval_yield+Feed_Vol+RS_cumulo+rab_integral+RQ_integral +AS_Vol.+emission_CO2+consum_O2+interval_O2; 0.345, 0.679, tmp+tmp_cumulo+PL_cumulo+AN+AN_cumulo+interval_yield+interv al_Pdt.+RS_cumulo+rab_integral+RQ_integral+AS_Vol.+emission _CO2+consum_O2; 0.356, 0.679, pH+PL_cumulo+AN_cumulo+interv al_yield+interval_Pdt.+OD+RS_cumulo+rab_integral+RQ_integra l+AS_Vol.+emission_CO2+interval_O2; 0.334, 0.679, tmp_cumul o+PL_cumulo+AN+AN_cumulo+interval_yield+interval_Pdt.+OD+Fe ed_Vol+RS_cumulo+rab_integral+RQ_integral+AS_Vol.+emission_ CO2+consum_O2; 0.386, 0.678, PL_cumulo+AN+AN_cumulo+interva l_yield+RS_cumulo+rab_integral+RQ_integral+AS_Vol.+emission _CO2; 0.366, 0.678, pH_cumulo+PL+PL_cumulo+AN_cumulo+interv al_yield+Feed_Vol+RS_cumulo+rab_integral+RQ_integral+AS_Vol. +emission_CO2; 0.386, 0.678, O2+PL_cumulo+interval_yield+in terval_Pdt.+RS_cumulo+RQ_integral+AS_Vol.+emission_CO2+inte rval_O2; 0.356, 0.678, O2+PL_cumulo+AN+AN_cumulo+interval_y ield+interval_Pdt.+Feed_Vol+RS_cumulo+rab_integral+RQ-integ ral+AS_Vol.+emission_CO2; 0.345, 0.678, tmp_cumulo+pH_cumul o+PL_cumulo+AN_cumulo+interval_yield+RS+Feed_Vol+RS_cumulo+ rab_integral+RQ_integral+AS_Vol.+emission_CO2+consum_O2; 0. 345, 0.678, tmp_cumulo+pH+PL+PL_cumulo+AN_cumulo+interval_y ield+Feed_Vol+RS_cumulo+rab_integral+RQ_integral+AS_Vol.+em ission_CO2+consum_O2; 0.345, 0.678, tmp_cumulo+pH+PL_cumulo +AN+AN_cumulo+interval_yield+interval_Pdt.+RS_cumulo+rab_in tegral+RQ_integral+AS_Vol.+emission_CO2+consum_O2; 0.356, 0. 678, tmp+tmp_cumulo+pH+AN_cumulo+interval_yield+Feed_Vol+RS _cumulo+rab_integral+RQ_integral+AS_Vol.+emission_CO2+inter val_O2; 0.356, 0.678, tmp_cumulo+AN_cumulo+interval_yield+i nterval_Pdt.+Feed_Vol+RS_cumulo+rab_integral+RQ_integral+AS _Vol.+emission_O2+emission_CO2+consum_O2; 0.356, 0.678, pH+ PL+PL_cumulo+AN_cumulo+interval_yield+RS+Feed_Vol+RS_cumulo +rab_integral+RQ_integral+AS_Vol.+emission_CO2; 0.345, 0.67 8, tmp_cumulo+AN+AN_cumulo+interval_yield+RS+Feed_Vol+RS_cu mulo+rab_integral+RQ_integral+AS_Vol.+emission_O2+consum_O2 +interval_O2; 0.385, 0.678, PL_cumulo+interval_yield+interv al_Pdt.+Feed_Vol+RS_cumulo+rab_integral+RQ_integral+AS_Vol. +emission_CO2; 0.376, 0.678, PL_cumulo+AN_cumulo+interval_y ield+OD+Feed_Vol+RS_cumulo+rab_integral+RQ_integral+AS_Vol. +emission_CO2; 0.366, 0.678, tmp+pH+PL_cumulo+AN_cumulo+int erval_yield+interval_Pdt.+RS_cumulo+rab_integral+RQ_integra l+AS_Vol.+emission_CO2; 0.345, 0.678, tmp_cumulo+pH+PL_cumu lo+AN+AN_cumulo+interval_yield+interval_Pdt.+Feed_Vol+RS_cu mulo+rab_integral+RQ_integral+AS_Vol.+emission_CO2; 0.356, 0.678, pH+PL_cumulo+AN_cumulo+interval_yield+interval_Pdt.+ RS+RS_cumulo+rab_integral+RQ_integral+AS_Vol.+emission_CO2+ interval_O2; 0.395, 0.678, pH+PL+PL_cumulo+AN_cumulo+interv al_yield+RS+Feed_Vol+AS_Vol.; 0.366, 0.678, tmp+PL_cumulo+A N_cumulo+interval_yield+interval_Pdt.+OD+RS_cumulo+rab_inte gral+RQ_integral+AS_Vol.+emission_CO2; 0.356, 0.678, tmp_cu mulo+AN_cumulo+interval_yield+OD+RS+Feed_Vol+RS_cumulo+rab_ integral+RQ_integral+AS_Vol.+emission_CO2+interval_O2; 0.36 6, 0.678, PL_cumulo+AN+AN_cumulo+interval_yield+interval_Pd t.+OD+RS_cumulo+rab_integral+RQ_integral+AS_Vol.+emission_C 02; 0.356, 0.678, PL_cumulo+AN_cumulo+interval_yield+interv al_Pdt.+RS+RS_cumulo+rab_integral+RQ_integral+AS_Vol.+emiss ion_CO2+consum_O2+interval_O2; 0.385, 0.678, PL_cumulo+AN_c umulo+interval_yield+RS+Feed_Vol+RS_cumulo+AS_Vol.+emission _O2+interval_O2; 0.366, 0.678, tmp+tmp_cumulo+PL+PL_cumulo+ interval_yield+interval_Pdt.+RS_cumulo+rab_integral+RQ_inte gral+AS_Vol.+emission_CO2; 0.345, 0.678, tmp_cumulo+PL_cumu lo+AN+AN_cumulo+interval_yield+Feed_Vol+RS_cumulo+rab_integ ral+RQ_integral+Accum._Yield+AS_Vol.+emission_CO2+interval 02; 0.395, 0.678, PL_cumulo+AN_cumulo+interval_yield+OD+RS+ Feed_Vol+AS_Vol.+emission_O2; 0.356, 0.678, pH_cumulo+PL+PL _cumulo+AN_cumulo+interval_yield+interval_Pdt.+RS_cumulo+ra b_integral+RQ_integral+AS_Vol.+emission_CO2+interval_O2; 0. 385, 0.678, O2+PL_cumulo+AN+interval_yield+OD+RS+Feed_Vol+A S_Vol.+interval_O2; 0.334, 0.678, tmp_cumulo+PL_cumulo+AN+A N_cumulo+interval_yield+OD+RS+Feed_Vol+RS_cumulo+rab_integr al+RQ_integral+AS_Vol.+emission_CO2+consum_O2; 0.334, 0.678, tmp_cumulo+PL_cumulo+AN+AN_cumulo+interval_yield+interval_ Pdt.+Feed_Vol+RS_cumulo+rab_integral+RQ_integral+AS_Vol.+em ission_O2+emission_CO2+interval_O2; 0.345, 0.678, tmp+tmp_c umulo+PL_cumulo+AN_cumulo+interval_yield+interval_Pdt.+RS+F eed_Vol+RS_cumulo+rab_integral+RQ_integral+AS_Vol.+emission _O2; 0.376, 0.678, pH+pH_cumulo+PL+PL_cumulo+interval_yield +OD+RS+Feed_Vol+AS_Vol.+interval_O2; 0.376, 0.678, pH+PL+PL _cumulo+interval_yield+interval_Pdt.+RS_cumulo+rab_integral +RQ_integral+AS_Vol.+emission_CO2; 0.376, 0.678, tmp+PL_cum ulo+interval_yield+interval_Pdt.+RS_cumulo+rab_integral+RQ_ integral+AS_Vol.+emission_CO2+interval_O2; 0.356, 0.678, tm p_cumulo+PL_cumulo+AN+AN_cumulo+interval_yield+interval_Pdt. +RS+RS_cumulo+rab_integral+RQ_integral+AS_Vol.+emission_CO 2; 0.356, 0.678, tmp_cumulo+PL_cumulo+AN_cumulo+interval_yi eld+interval_Pdt.+Feed_Vol+RS_cumulo+rab_integral+RQ_integr al+AS_Vol.+emission_CO2+generate_CO2; 0.345, 0.678, tmp_cum ulo+PL_cumulo+AN_cumulo+interval_yield+interval_Pdt.+Feed_V ol+RS_cumulo+rab_integral+RQ_integral+AS_Vol.+emission_CO2+ consum_O2+generate_CO2; 0.366, 0.678, tmp_cumulo+PL_cumulo+ AN+AN_cumulo+interval_yield+interval_Pdt.+RS_cumulo+rab_int egral+RQ_integral+emission_CO2+consum_O2; 0.355, 0.678, tmp _cumulo+pH+pH_cumulo+PL_cumulo+AN_cumulo+interval_yield+Fee d_Vol+RS_cumulo+rab_integral+RQ_integral+AS_Vol.+emission_C 02; 0.334, 0.678, tmp_cumulo+pH+pH_cumulo+PL_cumulo+AN_cumu lo+interval_yield+interval_Pdt.+Feed_Vol+RS_cumulo+rab_inte gral+RQ_integral+AS_Vol.+emission_CO2+interval_O2; 0.345, 0. 678, tmp_cumulo+PL_cumulo+AN+AN_cumulo+interval_yield+inter val_Pdt.+RS+Feed_Vol+RS_cumulo+rab_integral+RQ_integral+AS_ Vol.+emission_O2; 0.355, 0.678, tmp_cumulo+pH+PL_cumulo+int erval_yield+interval_Pdt.+Feed_Vol+RS_cumulo+rab_integral+R Q_integral+AS_Vol.+emission_O2+emission_CO2; 0.366, 0.678, tmp_cumulo+AN_cumulo+interval_yield+RS+Feed_Vol+rab_integra l+RQ_integral+AS_Vol. +emission_O2+consum_O2+interval_O2; 0. 385, 0.678, tmp+PL+PL_cumulo+interval_yield+interval_Pdt.+R S_cumulo+RQ_integral+AS_Vol.+emission_CO2; 0.366, 0.678, PL _cumulo+AN+AN_cumulo+interval_yield+interval_Pdt.+RS+RS_cum ulo+rab_integral+RQ_integral+AS_Vol.+emission_CO2; 0.334, 0. 678, tmp_cumulo+PL+PL_cumulo+AN_cumulo+interval_yield+RS+Fe ed_Vol+RS_cumulo+rab_integral+RQ_integral+AS_Vol.+emission_ CO2+consum_O2+interval_O2; 0.366, 0.678, tmp_cumulo+PL_cumu lo+AN_cumulo+interval_yield+RS+Feed_Vol+rab_integral+RQ_int egral+AS_Vol. +emission_O2+consum_O2; 0.385, 0.678, PL_cumul o+interval_yield+interval_Pdt.+RS_cumulo+rab_integral+RQ_in tegral+AS_Vol.+emission_CO2+consum_O2; 0.334, 0.678, tmp_cu mulo+PL_cumulo+AN_cumulo+interval_yield+interval_Pdt.+RS+Fe ed_Vol+RS_cumulo+rab_integral+RQ_integral+AS_Vol.+emission_ O2+emission_CO2+interval_O2; 0.345, 0.678, tmp+tmp_cumulo+p H+PL_cumulo+AN_cumulo+interval_yield+interval_Pdt.+RS_cumul o+rab_integral+RQ_integral+AS_Vol.+emission_CO2+interval_O 2; 0.366, 0.678, tmp_cumulo+PL_cumulo+interval_yield+interv al_Pdt.+RS_cumulo+rab_integral+RQ_integral+AS_Vol.+emission _CO2+consum_O2+interval_O2; 0.385, 0.678, O2+PL_cumulo+AN+A N_cumulo+interval_yield+RS_cumulo+RQ_integral+AS_Vol.+emiss ion_CO2; 0.375, 0.678, tmp_cumulo+pH+AN_cumulo+interval_yie ld+RS+Feed_Vol+rab_integral+RQ_integral+AS_Vol.+emission_O 2; 0.394, 0.678, PL_cumulo+AN_cumulo+interval_yield+RS+Feed _Vol+RS_cumulo+AS_Vol.+emission_CO2; 0.345, 0.678, tmp_cumu lo+PL_cumulo+AN_cumulo+interval_yield+OD+RS+Feed_Vol+RS_cum ulo+rab_integral+RQ_integral+AS_Vol.+emission_CO2+interval_ 02; 0.385, 0.678, PL_cumulo+interval_yield+interval_Pdt.+RS +RS_cumulo+rab_integral+RQ_integral+AS_Vol.+emission_CO2; 0. 403, 0.678, PL_cumulo+interval_yield+RS_cumulo+RQ_integral+ AS_Vol.+emission_O2+emission_CO2; 0.345, 0.678, tmp_cumulo+ PL_cumulo+AN_cumulo+interval_yield+Feed_Vol+RS_cumulo+rab_i ntegral+RQ_integral+Accum._Yield+AS_Vol.+emission_CO2+consu m_O2+interval_O2; 0.345, 0.678, tmp_cumulo+PL_cumulo+AN_cum ulo+interval_yield+interval_Pdt.+Feed_Vol+RS_cumulo+rab_int egral+RQ_integral+AS_Vol.+emission_CO2+interval_O2+generate _CO2; 0.394, 0.678, O2+PL_cumulo+AN+AN_cumulo+interval_yiel d+RS+RQ_integral+emission_CO2; 0.355, 0.678, O2+PL_cumulo+A N_cumulo+interval_yield+RS+Feed_Vol+RS_cumulo+rab_integral+ RQ_integral+AS_Vol.+emission_CO2+interval_O2; 0.345, 0.678, tmp_cumulo+PL_cumulo+AN+AN_cumulo+interval_yield+interval_ Pdt.+RS+Feed_Vol+RS_cumulo+rab_integral+RQ_integral+AS_Vol. +emission_CO2; 0.355, 0.678, tmp+tmp_cumulo+pH_cumulo+PL_cu mulo+AN_cumulo+interval_yield+Feed_Vol+RS_cumulo+rab_integr al+RQ_integral+AS_Vol.+emission_CO2; 0.394, 0.678, O2+PL_cu mulo+AN_cumulo+interval_yield+Feed_Vol+RS_cumulo+AS_Vol.+em ission_CO2; 0.365, 0.678, pH+PL_cumulo+AN_cumulo+interval_y ield+interval_Pdt.+OD+RS_cumulo+rab_integral+RQ_integral+AS _Vol.+emission_CO2; 0.345, 0.678, tmp_cumulo+PL_cumulo+AN+A N_cumulo+interval_yield+interval_Pdt.+RS+RS_cumulo+rab_inte gral+RQ_integral+AS_Vol.+emission_CO2+consum_O2; 0.345, 0.6 78, tmp+PL+PL_cumulo+AN_cumulo+interval_yield+interval_Pdt. +Feed_Vol+RS_cumulo+rab_integral+RQ_integral+AS_Vol.+emissi on_CO2+interval_O2; 0.355, 0.678, tmp_cumulo+PL_cumulo+AN+A N_cumulo+interval_yield+RS+Feed_Vol+rab_integral+RQ_integra l+AS_Vol.+emission_O2+consum_O2; 0.345, 0.678, tmp_cumulo+A N+AN_cumulo+interval_yield+RS+Feed_Vol+RS_cumulo+rab_integr al+RQ_integral+AS_Vol.+emission_CO2+consum_O2+generate_CO2; 0.375, 0.678, tmp_cumulo+PL+PL_cumulo+AN+AN_cumulo+interva l_yield+RS_cumulo+rab_integral+RQ_integral+emission_CO2; 0. 375, 0.678, tmp_cumulo+pH+PL+PL_cumulo+interval_yield+Feed_ Vol+RS_cumulo+RQ_integral+AS_Vol.+emission_CO2; 0.375, 0.67 8, O2+PL_cumulo+interval_yield+OD+RS+Feed_Vol+AS_Vol.+emiss ion_O2+consum_O2+interval_O2; 0.345, 0.678, tmp_cumulo+pH+A N+AN_cumulo+interval_yield+interval_Pdt.+Feed_Vol+RS_cumulo +rab_integral+RQ_integral+AS_Vol.+emission_CO2+interval_O2; 0.375, 0.678, tmp_cumulo+PL_cumulo+interval_yield+interval _Pdt.+RS+Feed_Vol+RS_cumulo+AS_Vol.+emission_O2+interval_O 2; 0.375, 0.678, tmp_cumulo+PL+PL_cumulo+interval_yield+RS+ Feed_Vol+RS_cumulo+AS_Vol.+consum_O2+interval_O2; 0.355, 0. 678, tmp_cumulo+PL+PL_cumulo+AN_cumulo+interval_yield+RS+Fe ed_Vol+RS_cumulo+rab_integral+RQ_integral+AS_Vol.+emission_ 02; 0.375, 0.678, PL_cumulo+AN_cumulo+interval_yield+interv al_Pdt.+RS_cumulo+rab_integral+RQ_integral+AS_Vol.+emission _O2+emission_CO2; 0.375, 0.678, tmp_cumulo+AN_cumulo+interv al_yield+interval_Pdt.+RS_cumulo+rab_integral+RQ_integral+A S_Vol.+emission_CO2+generate_CO2; 0.345, 0.678, tmp+tmp_cum ulo+AN+AN_cumulo+interval_yield+RS+Feed_Vol+RS_cumulo+rab_i ntegral+RQ_integral+AS_Vol.+emission_CO2+interval_O2; 0.394, 0.678, tmp_cumulo+AN_cumulo+interval_yield+RS+Feed_Vol+rab _integral+RQ_integral+AS_Vol.; 0.344, 0.678, tmp+tmp_cumulo +AN+AN_cumulo+interval_yield+interval_Pdt.+Feed_Vol+RS_cumu lo+rab_integral+RQ_integral+AS_Vol.+emission_CO2+consum_O2; 0.344, 0.678, tmp_cumulo+pH+pH_cumulo+PL_cumulo+AN_cumulo+ interval_yield+Feed_Vol+RS_cumulo+rab_integral+RQ_integral+ AS_Vol.+emission_CO2+interval_O2; 0.334, 0.678, tmp_cumulo+ pH_cumulo+PL_cumulo+AN+AN_cumulo+interval_yield+interval_Pd t.+Feed_Vol+RS_cumulo+rab_integral+RQ_integral+AS_Vol.+emis sion_CO2+consum_O2; 0.394, 0.678, AN_cumulo+interval_yield+ interval_Pdt.+RS_cumulo+rab_integral+RQ_integral+AS_Vol.+em ission_CO2; 0.375, 0.678, O2+PL_cumulo+AN_cumulo+interval_y ield+RS+Feed_Vol+RS_cumulo+AS_Vol.+consum_O2+interval_O2; 0. 355, 0.678, tmp_cumulo+PL_cumulo+AN_cumulo+interval_yield+i nterval_Pdt.+RS_cumulo+rab_integral+RQ_integral+AS_Vol.+emi ssion_CO2+consum_O2+generate_CO2; 0.355, 0.678, tmp_cumulo+ pH+AN_cumulo+interval_yield+interval_Pdt.+RS+Feed_Vol+RS_cu mulo+rab_integral+RQ_integral+AS_Vol.+emission_O2; 0.365, 0. 678, tmp_cumulo+AN_cumulo+interval_yield+RS+Feed_Vol+RS_cum ulo+rab_integral+RQ_integral+AS_Vol.+emission_O2+emission_C 02; 0.344, 0.678, tmp_cumulo+pH+PL_cumulo+AN_cumulo+interva l_yield+interval_Pdt.+RS_cumulo+rab_integral+RQ_integral+AS _Vol.+emission_CO2+consum_O2+interval_O2; 0.344, 0.678, tmp _cumulo+PL+PL_cumulo+AN+AN_cumulo+interval_yield+interval_P dt.+RS_cumulo+rab_integral+RQ_integral+AS_Vol.+emission_CO2 +consum_O2; 0.385, 0.678, pH_cumulo+PL_cumulo+interval_yiel d+interval_Pdt.+RS_cumulo+rab_integral+RQ_integral+AS_Vol.+ emission_CO2; 0.355, 0.678, tmp_cumulo+pH+PL_cumulo+interva l_yield+interval_Pdt.+Feed_Vol+RS_cumulo+rab_integral+RQ_in tegral+AS_Vol.+emission_CO2+interval_O2; 0.365, 0.678, tmp+ tmp_cumulo+pH+PL_cumulo+interval_yield+Feed_Vol+RS_cumulo+r ab_integral+RQ_integral+AS_Vol.+emission_CO2; 0.385, 0.678, pH+PL+PL_cumulo+interval_yield+interval_Pdt.+RS_cumulo+RQ_ integral+AS_Vol.+emission_CO2; 0.385, 0.678, O2+PL_cumulo+A N_cumulo+interval_yield+RS+Feed_Vol+rab_integral+RQ_integra l+AS_Vol.; 0.355, 0.678, tmp_cumulo+PL_cumulo+AN_cumulo+int erval_yield+RS+Feed_Vol+RS_cumulo+rab_integral+RQ_integral+ AS_Vol.+emission_O2+generate_CO2; 0.333, 0.678, tmp_cumulo+ PL+PL_cumulo+AN+AN_cumulo+interval_yield+RS+Feed_Vol+RS_cum ulo+rab_integral+RQ_integral+AS_Vol.+emission_CO2+interval 02; 0.355, 0.678, tmp_cumulo+PL_cumulo+AN_cumulo+interval_y ield+RS+Feed Vol+RS cumulo+rab integral+AS Vol.+emission O2 +consum_O2+interval_O2; 0.394, 0.678, PL_cumulo+AN+AN_cumul o+interval_yield+RS_cumulo+RQ_integral+AS_Vol.+emission_CO 2; 0.333, 0.678, tmp_cumulo+PL+PL_cumulo+AN_cumulo+interval _yield+interval_Pdt.+OD+Feed_Vol+RS_cumulo+rab_integral+RQ_ integral+AS_Vol.+emission_CO2+interval_O2; 0.344, 0.678, tm p_cumulo+AN+AN_cumulo+interval_yield+interval_Pdt.+OD+Feed_ Vol+RS_cumulo+rab_integral+RQ_integral+AS_Vol.+emission_CO2 +consum_O2; 0.385, 0.678, O2+PL_cumulo+AN_cumulo+interval_y ield+OD+RS+Feed_Vol+AS_Vol.+interval_O2; 0.333, 0.678, tmp_ cumulo+pH_cumulo+PL+PL_cumulo+AN_cumulo+interval_yield+inte rval_Pdt.+Feed_Vol+RS_cumulo+rab_integral+RQ_integral+AS_Vo l.+emission_CO2+interval_O2; 0.365, 0.678, pH+pH_cumulo+PL+ PL_cumulo+interval_yield+OD+RS+Feed_Vol+AS_Vol.+consum_O2+i nterval_O2; 0.333, 0.678, tmp_cumulo+PL+PL_cumulo+AN+AN_cum ulo+interval_yield+Feed_Vol+RS_cumulo+rab_integral+RQ_integ ral+AS_Vol.+emission_CO2+consum_O2+interval_O2; 0.365, 0.67 8, tmp_cumulo+AN+AN_cumulo+interval_yield+Feed_Vol+RS_cumul o+rab_integral+RQ_integral+AS_Vol.+emission_CO2+generate_CO 2; 0.394, 0.678, O2+PL_cumulo+AN_cumulo+interval_yield+RS+F eed_Vol+AS_Vol.+emission_O2; 0.355, 0.678, tmp_cumulo+PL_cu mulo+AN_cumulo+interval_yield+interval_Pdt.+OD+RS_cumulo+ra b_integral+RQ_integral+AS_Vol.+emission_CO2+consum_O2; 0.36 5, 0.678, tmp_cumulo+pH_cumulo+PL+PL_cumulo+interval_yield+ Feed_Vol+RS_cumulo+rab_integral+RQ_integral+AS_Vol.+emissio n_CO2; 0.344, 0.678, tmp_cumulo+pH_cumulo+PL_cumulo+AN_cumu lo+interval_yield+Feed_Vol+RS_cumulo+rab_integral+RQ_integr al+AS_Vol.+emission_CO2+consum_O2+interval_O2; 0.322, 0.678, tmp_cumulo+pH+PL_cumulo+AN+AN_cumulo+interval_yield+RS+Fee d_Vol+RS_cumulo+rab_integral+RQ_integral+AS_Vol.+emission_C O2+consum_O2+interval_O2; 0.384, 0.678, PL_cumulo+AN_cumulo +interval_yield+Feed_Vol+RS_cumulo+RQ_integral+AS_Vol.+emis sion_CO2+interval_O2; 0.344, 0.678, tmp_cumulo+PL_cumulo+AN +AN_cumulo+interval_yield+RS+Feed_Vol+rab_integral+RQ_integ ral+AS_Vol.+emission_O2+consum_O2+interval_O2; 0.333, 0.678, tmp_cumulo+pH_cumulo+PL_cumulo+AN_cumulo+interval_yield+in terval_Pdt.+RS+Feed_Vol+RS_cumulo+rab_integral+RQ_integral+ AS_Vol.+emission_CO2+interval_O2; 0.375, 0.678, tmp_cumulo+ PL_cumulo+AN_cumulo+interval_yield+RS+Feed_Vol+RS_cumulo+AS _Vol. +emission_O2+consum_O2; 0.375, 0.678, tmp_cumulo+AN_cu mulo+interval_yield+RS+Feed_Vol+RS_cumulo+rab_integral+RQ_i ntegral+AS_Vol.+generate_CO2; 0.355, 0.678, tmp_cumulo+AN+A N_cumulo+interval_yield+OD+RS+Feed_Vol+RS_cumulo+rab_integr al+RQ_integral+AS_Vol.+emission_CO2; 0.344, 0.678, tmp+tmp_ cumulo+PL_cumulo+AN_cumulo+interval_yield+interval_Pdt.+RS+ RS_cumulo+rab_integral+RQ_integral+AS_Vol.+emission_CO2+int erval_O2; 0.365, 0.678, O2+PL_cumulo+AN_cumulo+interval_yie ld+RS+Feed_Vol+RS_cumulo+AS_Vol.+emission_CO2+consum_O2+int erval_O2; 0.355, 0.678, tmp_cumulo+PL+PL_cumulo+AN_cumulo+i nterval_yield+Feed_Vol+RS_cumulo+rab_integral+RQ_integral+A ccum._Yield+AS_Vol.+emission_CO2; 0.365, 0.678, PL_cumulo+A N_cumulo+interval_yield+interval_Pdt.+RS+Feed_Vol+RS_cumulo +rab_integral+RQ_integral+AS_Vol.+emission_CO2; 0.365, 0.67 8, tmp_cumulo+PL_cumulo+AN+interval_yield+Feed_Vol+RS_cumul o+rab_integral+RQ_integral+AS_Vol.+emission_CO2+interval_O 2; 0.394, 0.678, O2+PL_cumulo+AN+AN_cumulo+interval_yield+R S+Feed_Vol+AS_Vol.; 0.375, 0.678, O2+PL_cumulo+AN_cumulo+in terval_yield+RS+Feed_Vol+AS_Vol.+emission_CO2+consum_O2+int erval_O2; 0.365, 0.678, tmp_cumulo+PL_cumulo+interval_yield +Feed_Vol+RS_cumulo+rab_integral+RQ_integral+AS_Vol.+emissi on_O2+emission_CO2+interval_O2; 0.375, 0.678, tmp_cumulo+PL _cumulo+interval_yield+Feed_Vol+RS_cumulo+rab_integral+RQ_i ntegral+AS_Vol.+emission_CO2+generate_CO2; 0.365, 0.678, tm p_cumulo+PL+PL_cumulo+interval_yield+interval_Pdt.+RS_cumul o+rab_integral+RQ_integral+AS_Vol.+emission_CO2+interval_O 2; 0.375, 0.678, pH_cumulo+PL_cumulo+AN_cumulo+interval_yie ld+Feed_Vol+RS_cumulo+rab_integral+RQ_integral+AS_Vol.+emis sion_CO2; 0.375, 0.678, tmp_cumulo+pH_cumulo+AN_cumulo+inte rval_yield+RS+Feed_Vol+rab_integral+RQ_integral+AS_Vol.+emi ssion_O2; 0.394, 0.678, PL_cumulo+interval_yield+interval_P dt.+RS+RS_cumulo+RQ_integral+AS_Vol.+emission_CO2; 0.355, 0. 678, pH_cumulo+PL_cumulo+AN_cumulo+interval_yield+interval_ Pdt.+RS_cumulo+rab_integral+RQ_integral+AS_Vol.+emission_CO 2+consum_O2+interval_O2; 0.344, 0.678, tmp+tmp_cumulo+PL+PL _cumulo+AN_cumulo+interval_yield+interval_Pdt.+RS_cumulo+ra b_integral+RQ_integral+AS_Vol.+emission_CO2+interval_O2; 0. 375, 0.678, PL_cumulo+AN+interval_yield+interval_Pdt.+RS_cu mulo+rab_integral+RQ_integral+AS_Vol.+emission_CO2+interval _O2; 0.355, 0.678, tmp+tmp_cumulo+pH+AN_cumulo+interval_yie ld+interval_Pdt.+Feed_Vol+RS_cumulo+rab_integral+RQ_integra l+AS_Vol.+emission_CO2; 0.365, 0.678, tmp+tmp_cumulo+PL_cum ulo+interval_yield+Feed_Vol+RS_cumulo+rab_integral+RQ_integ ral+AS_Vol.+emission_CO2+interval_O2; 0.365, 0.678, tmp_cum ulo+PL_cumulo+interval_yield+interval_Pdt.+Feed_Vol+RS_cumu lo+rab_integral+RQ_integral+AS_Vol.+emission_O2+interval_O 2; 0.354, 0.678, pH+PL+PL_cumulo+AN_cumulo+interval_yield+i nterval_Pdt.+RS+RS_cumulo+rab_integral+RQ_integral+AS_Vol.+ emission_CO2; 0.384, 0.678, tmp_cumulo+AN+interval_yield+Fe ed_Vol+RS_cumulo+rab_integral+RQ_integral+AS_Vol.+emission_ CO2; 0.344, 0.678, tmp_cumulo+PL_cumulo+AN+AN_cumulo+interv al_yield+interval_Pdt.+OD+RS_cumulo+rab_integral+RQ_integra l+AS_Vol.+emission_CO2+consum_O2; 0.365, 0.678, tmp+pH_cumu lo+PL_cumulo+AN_cumulo+interval_yield+interval_Pdt.+RS_cumu lo+rab_integral+RQ_integral+AS_Vol.+emission_CO2; 0.354, 0. 678, tmp_cumulo+PL_cumulo+AN_cumulo+interval_yield+interval _Pdt.+Feed_Vol+RS_cumulo+rab_integral+RQ_integral+Accum._Yi eld+AS_Vol.+emission_CO2; 0.375, 0.678, tmp_cumulo+AN_cumul o+interval_yield+RS+Feed_Vol+rab_integral+RQ_integral+AS_Vo l.+emission_CO2+consum_O2; 0.333, 0.678, tmp+tmp_cumulo+PL_ cumulo+AN_cumulo+interval_yield+interval_Pdt.+OD+Feed_Vol+R S_cumulo+rab_integral+RQ_integral+AS_Vol.+emission_CO2+inte rval_O2; 0.354, 0.678, tmp+tmp_cumulo+AN_cumulo+interval_yi eld+interval_Pdt.+RS+Feed_Vol+RS_cumulo+rab_integral+RQ_int egral+AS_Vol.+emission_O2; 0.394, 0.678, pH+PL+PL_cumulo+in terval_yield+RS+Feed_Vol+AS_Vol.+interval_O2; 0.344, 0.678, tmp_cumulo+PL_cumulo+AN_cumulo+interval_yield+Feed_Vol+RS_ cumulo+rab_integral+RQ_integral+AS_Vol.+emission_O2+emissio n_CO2+consum_O2+interval_O2; 0.365, 0.678, tmp_cumulo+AN_cu mulo+interval_yield+interval_Pdt.+RS+Feed_Vol+RS_cumulo+rab _integral+RQ_integral+AS_Vol.+consum_O2; 0.365, 0.678, tmp_ cumulo+AN_cumulo+interval_yield+OD+RS+Feed_Vol+RS_cumulo+ra b_integral+RQ_integral+AS_Vol.+emission_O2; 0.384, 0.678, O 2+PL_cumulo+interval_yield+interval_Pdt.+RS_cumulo+RQ_integ ral+AS_Vol.+emission_O2+emission_CO2; 0.365, 0.678, tmp+tmp _cumulo+pH_cumulo+AN_cumulo+interval_yield+Feed_Vol+RS_cumu lo+rab_integral+RQ_integral+AS_Vol.+emission_CO2; 0.354, 0. 678, tmp_cumulo+PL_cumulo+AN+AN_cumulo+interval_yield+inter val_Pdt.+RS+RS_cumulo+rab_integral+RQ_integral+emission_CO2 +consum_O2; 0.354, 0.678, tmp+PL+PL_cumulo+AN_cumulo+interv al_yield+interval_Pdt.+RS_cumulo+rab_integral+RQ_integral+A S_Vol.+emission_CO2+consum_O2; 0.354, 0.678, tmp_cumulo+AN+ AN_cumulo+interval_yield+OD+Feed_Vol+RS_cumulo+rab_integral +RQ_integral+AS_Vol.+emission_CO2+interval_O2; 0.365, 0.678, O2+PL_cumulo+AN_cumulo+interval_yield+OD+Feed_Vol+RS_cumul o+rab_integral+RQ_integral+AS_Vol.+emission_CO2; 0.393, 0.6 78, O2+PL_cumulo+interval_yield+RS+Feed_Vol+RS_cumulo+AS_Vo l.+emission_CO2; 0.393, 0.678, O2+PL_cumulo+AN+interval_yie ld+RS+Feed_Vol+RS_cumulo+AS_Vol.; 0.354, 0.678, tmp_cumulo+ pH+AN_cumulo+interval_yield+interval_Pdt.+OD+Feed_Vol+RS_cu mulo+rab_integral+RQ_integral+AS_Vol.+emission_CO2; 0.344, 0.678, tmp_cumulo+PL_cumulo+AN_cumulo+interval_yield+RS+Fee d_Vol+RS_cumulo+rab_integral+AS_Vol.+emission_O2+consum_O2+ interval_O2+generate_CO2; 0.354, 0.678, tmp+PL_cumulo+AN_cu mulo+interval_yield+interval_Pdt.+Feed_Vol+RS_cumulo+rab_in tegral+RQ_integral+AS_Vol.+emission_CO2+consum_O2; 0.344, 0. 678, tmp+tmp_cumulo+PL+PL_cumulo+AN_cumulo+interval_yield+R S+Feed_Vol+RS_cumulo+rab_integral+RQ_integral+AS_Vol.+emiss ion_CO2; 0.365, 0.678, tmp_cumulo+PL_cumulo+interval_yield+ interval_Pdt.+RS+Feed_Vol+RS_cumulo+AS_Vol.+emission_O2+con sum_O2+interval_O2; 0.374, 0.678, tmp_cumulo+AN_cumulo+inte rval_yield+OD+RS+Feed_Vol+rab_integral+RQ_integral+AS_Vol.+ emission_O2; 0.384, 0.677, tmp+tmp_cumulo+PL_cumulo+interva l_yield+interval_Pdt.+RS_cumulo+RQ_integral+AS_Vol.+emissio n_CO2; 0.344, 0.677, tmp_cumulo+pH+PL_cumulo+AN_cumulo+inte rval_yield+interval_Pdt.+Feed_Vol+RS_cumulo+rab_integral+RQ _integral+AS_Vol.+emission_O2+emission_CO2; 0.374, 0.677, t mp_cumulo+PL_cumulo+AN+AN-cumulo+interval_yield+RS_cumulo+r ab_integral+RQ_integral+emission_CO2+interval_O2; 0.365, 0. 677, tmp_cumulo+pH_cumulo+AN_cumulo+interval_yield+interval _Pdt.+Feed_Vol+RS_cumulo+rab_integral+RQ_integral+AS_Vol.+e mission_O2; 0.333, 0.677, tmp_cumulo+PL_cumulo+AN+AN_cumulo +interval_yield+interval_Pdt.+Feed_Vol+RS_cumulo+rab_integr al+RQ_integral+Accum._Yield+AS_Vol.+emission_CO2+consum_O2; 0.333, 0.677, tmp_cumulo+pH+PL_cumulo+AN_cumulo+interval_y ield+interval_Pdt.+OD+Feed_Vol+RS_cumulo+rab_integral+RQ_in tegral+AS_Vol.+emission_CO2+consum_O2; 0.344, 0.677, tmp_cu mulo+PL_cumulo+AN_cumulo+interval_yield+interval_Pdt.+RS+Fe ed_Vol+RS_cumulo+rab_integral+RQ_integral+AS_Vol.+emission_ O2+emission_CO2; 0.354, 0.677, tmp_cumulo+AN+AN_cumulo+inte rval_yield+RS+Feed_Vol+rab_integral+RQ_integral+AS_Vol.+emi ssion_CO2+consum_O2+interval_O2; 0.393, 0.677, tmp_cumulo+i nterval_yield+OD+RS+Feed_Vol+AS_Vol.+emission_O2+interval_O 2; 0.374, 0.677, O2+PL_cumulo+AN_cumulo+interval_yield+RS+F eed_Vol+RS_cumulo+AS_Vol.+emission_CO2+interval_O2; 0.344, 0.677, tmp_cumulo+pH+AN+AN_cumulo+interval_yield+Feed_Vol+R S_cumulo+rab_integral+RQ_integral+AS_Vol.+emission_CO2+cons um_O2+interval_O2; 0.354, 0.677, tmp+PL_cumulo+AN+AN_cumulo +interval_yield+interval_Pdt.+RS_cumulo+rab_integral+RQ_int egral+AS_Vol.+emission_CO2+consum_O2; 0.384, 0.677, PL_cumu lo+AN+interval_yield+OD+RS+Feed_Vol+AS_Vol.+emission_O2+int erval_O2; 0.354, 0.677, pH+pH_cumulo+PL+PL_cumulo+AN_cumulo +interval_yield+interval_Pdt.+RS_cumulo+rab_integral+RQ_int egral+AS_Vol.+emission_CO2; 0.354, 0.677, O2+PL_cumulo+AN+A N_cumulo+interval_yield+RS+Feed_Vol+RS_cumulo+rab_integral+ RQ_integral+AS_Vol.+emission_CO2; 0.402, 0.677, tmp_cumulo+ interval_yield+RS+Feed_Vol+RS_cumulo+AS_Vol.+emission_CO2; 0.393, 0.677, pH_cumulo+PL+PL_cumulo+AN_cumulo+interval_yie ld+RS+Feed_Vol+AS_Vol.; 0.344, 0.677, tmp+tmp_cumulo+PL+PL_ cumulo+AN+AN_cumulo+interval_yield+Feed_Vol+RS_cumulo+rab_i ntegral+RQ_integral+AS_Vol.+emission_CO2; 0.344, 0.677, tmp +tmp_cumulo+AN_cumulo+interval_yield+interval_Pdt.+RS+Feed_ Vol+RS_cumulo+rab_integral+RQ_integral+AS_Vol.+emission_CO2 +interval_O2; 0.344, 0.677, tmp_cumulo+pH+PL_cumulo+AN+AN_c umulo+interval_yield+RS+Feed_Vol+RS_cumulo+rab_integral+RQ integral+AS_Vol.+emission_CO2; 0.374, 0.677, tmp_cumulo+PL_ cumulo+AN_cumulo+interval_yield+Feed_Vol+RS_cumulo+RQ_integ ral+AS_Vol.+emission_CO2+interval_O2; 0.374, 0.677, tmp_cum ulo+pH+AN_cumulo+interval_yield+interval_Pdt.+RS_cumulo+rab _integral+RQ_integral+AS_Vol.+emission_CO2; 0.354, 0.677, t mp_cumulo+AN_cumulo+interval_yield+RS+Feed_Vol+RS_cumulo+ra b_integral+RQ_integral+AS_Vol.+consum_O2+interval_O2+genera te_CO2; 0.384, 0.677, pH_cumulo+PL_cumulo+interval_yield+OD +RS+Feed_Vol+AS_Vol.+emission_O2+interval_O2; 0.384, 0.677, O2+PL_cumulo+interval_yield+Feed_Vol+RS_cumulo+rab_integra l+RQ_integral+AS_Vol.+emission_CO2; 0.364, 0.677, tmp_cumul o+pH_cumulo+PL_cumulo+interval_yield+Feed_Vol+RS_cumulo+rab _integral+RQ_integral+AS_Vol. +emission_O2+emission_CO2; 0.3 33, 0.677, tmp_cumulo+pH+PL+PL_cumulo+AN_cumulo+interval_yi eld+RS+Feed_Vol+RS_cumulo+rab_integral+RQ_integral+AS_Vol.+ emission_CO2+interval_O2; 0.364, 0.677, tmp_cumulo+AN_cumul o+interval_yield+interval_Pdt.+RS+RS_cumulo+rab_integral+RQ _integral+AS_Vol.+emission_CO2+interval_O2; 0.374, 0.677, t mp_cumulo+AN_cumulo+interval_yield+OD+RS+Feed_Vol+rab_integ ral+RQ_integral+AS_Vol.+emission_CO2; 0.402, 0.677, tmp_cum ulo+PL_cumulo+interval_yield+RS_cumulo+RQ_integral+AS_Vol.+ emission_CO2; 0.374, 0.677, tmp_cumulo+PL+AN_cumulo+interva l_yield+interval_Pdt.+RS_cumulo+rab_integral+RQ_integral+AS _Vol.+emission_CO2; 0.364, 0.677, tmp_cumulo+PL+AN_cumulo+i nterval_yield+RS+Feed_Vol+RS_cumulo+rab_integral+RQ_integra l+AS_Vol.+emission_O2; 0.333, 0.677, tmp_cumulo+pH_cumulo+P L_cumulo+AN+AN_cumulo+interval_yield+interval_Pdt.+Feed_Vol +RS_cumulo+rab_integral+RQ_integral+AS_Vol.+emission_CO2+in terval_O2; 0.333, 0.677, tmp+tmp_cumulo+pH_cumulo+PL_cumulo +AN_cumulo+interval_yield+interval_Pdt.+Feed_Vol+RS_cumulo+ rab_integral+RQ_integral+AS_Vol.+emission_CO2+interval_O2; 0.344, 0.677, tmp+tmp_cumulo+pH_cumulo+PL_cumulo+AN_cumulo+ interval_yield+Feed_Vol+RS_cumulo+rab_integral+RQ_integral+ AS_Vol.+emission_CO2+interval_O2; 0.374, 0.677, pH+PL+PL_cu mulo+AN_cumulo+interval_yield+Feed_Vol+RS_cumulo+RQ_integra l+AS_Vol.+emission_CO2; 0.364, 0.677, tmp+tmp_cumulo+PL+AN_ cumulo+interval_yield+Feed_Vol+RS_cumulo+rab_integral+RQ_in tegral+AS_Vol.+emission_CO2; 0.354, 0.677, tmp_cumulo+PL_cu mulo+AN_cumulo+interval_yield+interval_Pdt.+OD+Feed_Vol+RS_ cumulo+rab_integral+RQ_integral+AS_Vol.+emission_O2; 0.343, 0.677, tmp+tmp_cumulo+AN_cumulo+interval_yield+RS+Feed_Vol +RS_cumulo+rab_integral+RQ_integral+AS_Vol.+emission_CO2+co nsum_O2+interval_O2; 0.343, 0.677, pH+PL+PL_cumulo+AN_cumul o+interval_yield+interval_Pdt.+Feed_Vol+RS_cumulo+rab_integ ral+RQ_integral+AS_Vol.+emission_CO2+interval_O2; 0.343, 0. 677, tmp_cumulo+PL_cumulo+AN_cumulo+interval_yield+interval _Pdt.+RS+RS_cumulo+rab_integral+RQ_integral+AS_Vol.+emissio n_CO2+consum_O2+interval_O2; 0.384, 0.677, O2+PL_cumulo+AN_ cumulo+interval_yield+RS+Feed_Vol+RS_cumulo+AS_Vol.+interva l_O2; 0.354, 0.677, tmp+tmp_cumulo+pH+AN_cumulo+interval_yi eld+RS+Feed_Vol+RS_cumulo+rab_integral+RQ_integral+AS_Vol.+ emission_CO2; 0.332, 0.677, tmp+tmp_cumulo+AN+AN_cumulo+int erval_yield+RS+Feed_Vol+RS_cumulo+rab_integral+RQ_integral+ AS_Vol.+emission_CO2+consum_O2+interval_O2; 0.354, 0.677, t mp_cumulo+pH+PL+AN_cumulo+interval_yield+RS+Feed_Vol+RS_cum ulo+rab_integral+RQ_integral+AS_Vol.+emission_CO2; 0.354, 0. 677, tmp_cumulo+pH+AN+AN_cumulo+interval_yield+RS+Feed_Vol+ rab_integral+RQ_integral+AS_Vol.+emission_CO2+consum_O2; 0. 332, 0.677, tmp_cumulo+pH+PL_cumulo+AN_cumulo+interval_yiel d+interval_Pdt.+Feed_Vol+RS_cumulo+rab_integral+RQ_integral +AS_Vol.+emission_O2+emission_CO2+consum_O2; 0.354, 0.677, tmp_cumulo+pH+AN_cumulo+interval_yield+RS+Feed_Vol+RS_cumul o+rab_integral+RQ_integral+AS_Vol.+emission_O2+consum_O2; 0. 364, 0.677, tmp_cumulo+PL_cumulo+AN_cumulo+interval_yield+i nterval_Pdt.+RS_cumulo+rab_integral+RQ_integral+AS_Vol.+emi ssion_O2+emission_CO2; 0.343, 0.677, tmp_cumulo+pH_cumulo+P L_cumulo+AN+AN_cumulo+interval_yield+interval_Pdt.+RS_cumul o+rab_integral+RQ_integral+AS_Vol.+emission_CO2+interval_O 2; 0.332, 0.677, tmp_cumulo+pH+PL+PL_cumulo+AN+AN_cumulo+in terval_yield+Feed_Vol+RS_cumulo+rab_integral+RQ_integral+AS _Vol.+emission_CO2+interval_O2; 0.364, 0.677, tmp_cumulo+PL +PL_cumulo+AN+interval_yield+Feed_Vol+RS_cumulo+rab_integra l+RQ_integral+AS_Vol.+emission_CO2; 0.354, 0.677, tmp+PL_cu mulo+AN_cumulo+interval_yield+RS+Feed_Vol+RS_cumulo+rab_int egral+RQ_integral+AS_Vol.+emission_CO2+interval_O2; 0.354, 0.677, O2+PL_cumulo+AN+AN_cumulo+interval_yield+interval_Pd t.+OD+RS_cumulo+rab_integral+RQ_integral+AS_Vol.+emission_C 02; 0.332, 0.677, tmp_cumulo+pH_cumulo+PL_cumulo+AN+AN_cumu lo+interval_yield+RS+Feed_Vol+RS_cumulo+rab_integral+RQ_int egral+AS_Vol.+emission_CO2+consum_O2; 0.384, 0.677, tmp_cum ulo+pH_cumulo+PL_cumulo+interval_yield+Feed_Vol+RS_cumulo+R Q_integral+AS_Vol.+emission_CO2; 0.354, 0.677, pH+PL_cumulo +AN+AN_cumulo+interval_yield+interval_Pdt.+RS_cumulo+rab_in tegral+RQ_integral+AS_Vol.+emission_CO2+consum_O2; 0.384, 0. 677, tmp_cumulo+PL_cumulo+interval_yield+interval_Pdt.+RS+R S_cumulo+RQ_integral+AS_Vol.+emission_CO2; 0.384, 0.677, tm p_cumulo+PL_cumulo+AN+AN_cumulo+interval_yield+RS_cumulo+RQ _integral+emission_CO2+interval_O2; 0.354, 0.677, tmp_cumul o+PL_cumulo+interval_yield+interval_Pdt.+RS+Feed_Vol+RS_cum ulo+rab_integral+RQ_integral+AS_Vol.+emission_O2+emission_C 02; 0.321, 0.677, tmp_cumulo+pH+PL_cumulo+AN+AN_cumulo+inte rval_yield+interval_Pdt.+Feed_Vol+RS_cumulo+rab_integral+RQ _integral+AS_Vol.+emission_CO2+consum_O2+interval_O2; 0.332, 0.677, tmp_cumulo+PL_cumulo+AN+AN_cumulo+interval_yield+in terval_Pdt.+OD+Feed_Vol+RS_cumulo+rab_integral+RQ_integral+ AS_Vol.+emission_CO2+interval_O2; 0.411, 0.677, PL_cumulo+i nterval_yield+RS+Feed_Vol+RS_cumulo+AS_Vol.; 0.332, 0.677, tmp+tmp_cumulo+PL+PL_cumulo+AN_cumulo+interval_yield+interv al_Pdt.+Feed_Vol+RS_cumulo+rab_integral+RQ_integral+AS_Vol. +emission_CO2+consum_O2; 0.354, 0.677, tmp_cumulo+PL_cumulo +AN_cumulo+interval_yield+Feed_Vol+RS_cumulo+rab_integral+R Q_integral+AS_Vol.+emission_CO2+interval_O2+generate_CO2; 0. 374, 0.677, tmp_cumulo+PL_cumulo+AN_cumulo+interval_yield+i nterval_Pdt.+RS+Feed_Vol+RS_cumulo+AS_Vol.+emission_O2; 0.3 32, 0.677, tmp_cumulo+PL_cumulo+AN_cumulo+interval_yield+in terval_Pdt.+OD+RS+Feed_Vol+RS_cumulo+rab_integral+RQ_integr al+AS_Vol.+emission_CO2+interval_O2; 0.343, 0.677, tmp_cumu lo+PL_cumulo+AN+AN_cumulo+interval_yield+interval_Pdt.+RS_c umulo+rab_integral+RQ_integral+AS_Vol.+emission_O2+emission _CO2+interval_O2; 0.343, 0.677, tmp+tmp_cumulo+PL_cumulo+AN _cumulo+interval_yield+interval_Pdt.+RS+Feed_Vol+RS_cumulo+ rab_integral+RQ_integral+AS_Vol.+emission_CO2; 0.354, 0.677, tmp_cumulo+PL+PL_cumulo+AN_cumulo+interval_yield+interval_ Pdt.+Feed_Vol+RS_cumulo+rab_integral+RQ_integral+AS_Vol.+em ission_O2; 0.364, 0.677, tmp_cumulo+pH+PL_cumulo+AN_cumulo+ interval_yield+RS+Feed_Vol+rab_integral+RQ_integral+AS_Vol. +emission_O2; 0.364, 0.677, PL_cumulo+AN_cumulo+interval_yi eld+RS+Feed_Vol+RS_cumulo+rab_integral+RQ_integral+AS_Vol.+ emission_CO2+consum_O2; 0.374, 0.677, tmp_cumulo+pH+PL_cumu lo+interval_yield+RS+Feed_Vol+RS_cumulo+AS_Vol.+emission_O2 +interval_O2; 0.321, 0.677, tmp_cumulo+pH+PL_cumulo+AN+AN_c umulo+interval_yield+interval_Pdt.+RS+Feed_Vol+RS_cumulo+ra b_integral+RQ_integral+AS_Vol.+emission_CO2+consum_O2; 0.34 3, 0.677, tmp_cumulo+pH_cumulo+PL+PL_cumulo+AN_cumulo+inter val_yield+RS+Feed_Vol+RS_cumulo+rab_integral+RQ_integral+AS _Vol.+emission_CO2; 0.364, 0.677, pH_cumulo+PL_cumulo+AN_cu mulo+interval_yield+Feed_Vol+RS_cumulo+rab_integral+RQ_inte gral+AS_Vol.+emission_CO2+interval_O2; 0.354, 0.677, tmp_cu mulo+PL_cumulo+AN_cumulo+interval_yield+RS+Feed_Vol+rab_int egral+RQ_integral+AS_Vol.+emission_O2+consum_O2+interval_O 2; 0.364, 0.677, tmp_cumulo+PL+PL_cumulo+interval_yield+int erval_Pdt.+RS+RS_cumulo+rab_integral+RQ_integral+AS_Vol.+em ission_CO2; 0.354, 0.677, tmp_cumulo+PL_cumulo+interval_yie ld+interval_Pdt.+Feed_Vol+RS_cumulo+rab_integral+RQ_integra l+AS_Vol.+emission_CO2+consum_O2+interval_O2; 0.374, 0.677, O2+PL_cumulo+AN_cumulo+interval_yield+RS+Feed_Vol+RS_cumul o+RQ_integral+AS_Vol.+emission_CO2; 0.354, 0.677, tmp_cumul o+pH+PL+AN_cumulo+interval_yield+Feed_Vol+RS_cumulo+rab_int egral+RQ_integral+AS_Vol.+emission_CO2+interval_O2; 0.364, 0.677, tmp_cumulo+PL_cumulo+interval_yield+interval_Pdt.+RS +RS_cumulo+rab_integral+RQ_integral+AS_Vol.+emission_CO2+in terval_O2; 0.364, 0.677, pH+pH_cumulo+PL_cumulo+AN_cumulo+i nterval_yield+interval_Pdt.+RS_cumulo+rab_integral+RQ_integ ral+AS_Vol.+emission_CO2; 0.393, 0.677, tmp+PL+PL_cumulo+AN _cumulo+interval_yield+RS+Feed_Vol+AS_Vol.; 0.383, 0.677, t mp_cumulo+AN_cumulo+interval_yield+RS+Feed_Vol+rab_integral +RQ_integral+AS_Vol.+emission_CO2; 0.383, 0.677, tmp_cumulo +PL+PL_cumulo+interval_yield+RS+Feed_Vol+RS_cumulo+AS_Vol.+ interval_O2; 0.332, 0.677, tmp_cumulo+pH_cumulo+PL_cumulo+A N_cumulo+interval_yield+interval_Pdt.+RS+Feed_Vol+RS_cumulo +rab_integral+RQ_integral+AS_Vol.+emission_CO2+consum-O2; 0. 374, 0.677, tmp_cumulo+AN+AN_cumulo+interval_yield+RS+Feed_ Vol+rab_integral+RQ_integral+AS_Vol.+emission_CO2; 0.321, 0. 677, tmp_cumulo+pH+PL_cumulo+AN_cumulo+interval_yield+inter val_Pdt.+RS+Feed_Vol+RS_cumulo+rab_integral+RQ_integral+AS_ Vol.+emission_CO2+consum_O2+interval_O2; 0.354, 0.677, tmp_ cumulo+PL+AN_cumulo+interval_yield+interval_Pdt.+RS+Feed_Vo l+RS_cumulo+rab_integral+RQ_integral+AS_Vol.+emission_CO2; 0.353, 0.677, tmp+PL+PL_cumulo+AN_cumulo+interval_yield+Fee d_Vol+RS_cumulo+rab_integral+RQ_integral+AS_Vol.+emission_C O2+interval_O2; 0.343, 0.677, tmp_cumulo+pH+PL+PL_cumulo+AN _cumulo+interval_yield+interval_Pdt.+RS_cumulo+rab_integral +RQ_integral+AS_Vol.+emission_CO2+interval_O2; 0.374, 0.677, O2+PL_cumulo+AN_cumulo+interval_yield+RS+Feed_Vol+AS_Vol.+ emission_O2+consum_O2+interval_O2; 0.343, 0.677, tmp+tmp_cu mulo+AN_cumulo+interval_yield+interval_Pdt.+Feed_Vol+RS_cum ulo+rab_integral+RQ_integral+AS_Vol.+emission_CO2+consum_O2 +interval_O2; 0.383, 0.677, pH+PL+PL_cumulo+interval_yield+ RS_cumulo+RQ_integral+AS_Vol.+emission_O2+emission_CO2; 0.3 53, 0.677, tmp_cumulo+pH_cumulo+PL_cumulo+AN_cumulo+interva l_yield+interval_Pdt.+RS_cumulo+rab_integral+RQ_integral+AS _Vol. +emission_CO2+consum_O2; 0.364, 0.677, tmp+PL_cumulo+A N_cumulo+interval_yield+RS+Feed_Vol+RS_cumulo+rab_integral+ RQ_integral+AS_Vol.+emission_CO2; 0.374, 0.677, tmp_cumulo+ PL+PL_cumulo+interval_yield+RS+Feed_Vol+RS_cumulo+AS_Vol.+e mission_O2+interval_O2; 0.402, 0.677, O2+PL_cumulo+interval _yield+RS_cumulo+RQ_integral+AS_Vol.+emission_CO2; 0.393, 0. 677, O2+PL_cumulo+AN+AN_cumulo+interval_yield+RS_cumulo+RQ_ integral+emission_CO2; 0.343, 0.677, tmp_cumulo+pH+PL+PL_cu mulo+AN_cumulo+interval_yield+OD+Feed_Vol+RS_cumulo+rab_int egral+RQ_integral+AS_Vol.+emission_CO2; 0.353, 0.677, tmp+t mp_cumulo+AN+AN_cumulo+interval_yield+interval_Pdt.+FeedVo l+RS_cumulo+rab_integral+RQ_integral+AS_Vol.+emission_CO2; 0.353, 0.677, pH_cumulo+PL+PL_cumulo+AN_cumulo+interval_yie ld+interval_Pdt.+Feed_Vol+RS_cumulo+rab_integral+RQ_integra l+AS_Vol.+emission_CO2; 0.364, 0.677, tmp+tmp_cumulo+PL+PL_ cumulo+interval_yield+Feed_Vol+RS_cumulo+rab_integral+RQ_in tegral+AS_Vol.+emission_CO2; 0.332, 0.677, tmp+tmp_cumulo+A N+AN_cumulo+interval_yield+interval_Pdt.+RS+Feed_Vol+RS_cum ulo+rab_integral+RQ_integral+AS_Vol.+emission_CO2+consum_O 2; 0.374, 0.677, O2+PL_cumulo+AN_cumulo+interval_yield+RS+F eed_Vol+RS_cumulo+AS_Vol.+emission_CO2+consum_O2; 0.320, 0. 677, tmp_cumulo+PL+PL_cumulo+AN+AN_cumulo+interval_yield+RS +Feed_Vol+RS_cumulo+rab_integral+RQ_integral+AS_Vol.+emissi on_CO2+consum_O2+interval_O2; 0.374, 0.677, tmp_cumulo+PL+P L_cumulo+AN+AN_cumulo+interval_yield+RS+RS_cumulo+RQ_integr al+emission_CO2; 0.374, 0.677, tmp_cumulo+PL_cumulo+interva l_yield+RS+Feed_Vol+RS_cumulo+AS_Vol.+emission_O2+emission_ CO2+interval_O2; 0.343, 0.677, tmp_cumulo+pH_cumulo+PL+PL_c umulo+AN+AN_cumulo+interval_yield+Feed_Vol+RS_cumulo+rab_in tegral+RQ_integral+AS_Vol.+emission_CO2; 0.353, 0.677, tmp+ tmp_cumulo+pH+PL_cumulo+AN_cumulo+interval_yield+interval_P dt.+RS_cumulo+rab_integral+RQ_integral+AS_Vol.+emission_CO 2; 0.383, 0.677, tmp_cumulo+PL_cumulo+interval_yield+interv al_Pdt.+OD+RS_cumulo+RQ_integral+AS_Vol.+emission_CO2; 0.37 3, 0.677, pH+PL+PL_cumulo+AN_cumulo+interval_yield+interval _Pdt.+RS_cumulo+RQ_integral+AS_Vol.+emission_CO2; 0.364, 0. 677, pH+PL_cumulo+AN_cumulo+interval_yield+interval_Pdt.+RS +RS_cumulo+rab_integral+RQ_integral+AS_Vol.+emission_CO2; 0. 353, 0.677, tmp_cumulo+PL_cumulo+AN_cumulo+interval_yield+i nterval_Pdt.+Feed_Vol+RS_cumulo+rab_integral+RQ_integral+AS _Vol.+emission_O2+generate_CO2; 0.383, 0.677, PL_cumulo+AN_ cumulo+interval_yield+OD+Feed_Vol+RS_cumulo+RQ_integral+AS_ Vol.+emission_CO2; 0.343, 0.677, tmp_cumulo+PL_cumulo+AN+AN _cumulo+interval_yield+interval_Pdt.+OD+Feed_Vol+RS_cumulo+ rab_integral+RQ_integral+AS_Vol.+emission_CO2; 0.353, 0.677, tmp_cumulo+PL+PL_cumulo+AN+interval_yield+interval_Pdt.+Fe ed_Vol+RS_cumulo+rab_integral+RQ_integral+AS_Vol.+emission_ CO2; 0.383, 0.677, tmp_cumulo+pH_cumulo+interval_yield+Feed _Vol+RS_cumulo+rab_integral+RQ_integral+AS_Vol.+emission_CO 2; 0.363, 0.677, O2+PL_cumulo+AN_cumulo+interval_yield+OD+R S+Feed_Vol+rab_integral+RQ_integral+AS_Vol.+emission_CO2; 0. 373, 0.677, O2+PL_cumulo+AN+AN_cumulo+interval_yield+interv al_Pdt.+RS_cumulo+RQ_integral+AS_Vol.+emission_CO2; 0.363, 0.677, tmp_cumulo+pH+PL_cumulo+interval_yield+RS+Feed_Vol+R S_cumulo+AS_Vol. +emission_O2+consum_O2+interval_O2; 0.353, 0.677, tmp_cumulo+AN_cumulo+interval_yield+RS+Feed_Vol+RS_c umulo+rab_integral+RQ_integral+AS_Vol.+emission_CO2+consum_ O2+generate_CO2; 0.373, 0.677, tmp_cumulo+interval_yield+RS +Feed_Vol+RS_cumulo+rab_integral+RQ_integral+AS_Vol.+emissi on_CO2+interval_O2; 0.401, 0.677, PL_cumulo+AN_cumulo+inter val_yield+RS_cumulo+RQ_integral+AS_Vol.+emission_CO2; 0.383, 0.677, tmp+tmp_cumulo+PL_cumulo+interval_yield+Feed_Vol+RS _cumulo+RQ_integral+AS_Vol.+emission_CO2; 0.353, 0.677, tmp _cumulo+AN+AN_cumulo+interval_yield+interval_Pdt.+RS+Feed_V ol+rab_integral+RQ_integral+AS_Vol.+emission_CO2+consum_O2; 0.363, 0.677, tmp+tmp_cumulo+AN_cumulo+interval_yield+inte rval_Pdt.+RS_cumulo+rab_integral+RQ_integral+AS_Vol.+emissi on_CO2+interval_O2; 0.353, 0.677, pH+PL+PL_cumulo+AN_cumulo +interval_yield+Feed_Vol+RS_cumulo+rab_integral+RQ_integral +AS_Vol.+emission_CO2+interval_O2; 0.383, 0.677, O2+PL_cumu lo+AN+AN_cumulo+interval_yield+RS+RS_cumulo+RQ_integral+emi ssion_CO2; 0.373, 0.677, pH_cumulo+PL_cumulo+interval_yield +OD+RS+Feed_Vol+AS_Vol.+emission_O2+consum_O2+interval_O2; 0.353, 0.677, tmp_cumulo+pH_cumulo+AN_cumulo+interval_yield +interval_Pdt.+RS+Feed_Vol+RS_cumulo+rab_integral+RQ_integr al+AS_Vol.+emission_CO2; 0.353, 0.677, tmp_cumulo+PL+PL_cum ulo+AN_cumulo+interval_yield+Feed_Vol+RS_cumulo+rab_integra l+RQ_integral+AS_Vol.+emission_O2+emission_CO2; 0.392, 0.67 7, tmp_cumulo+PL_cumulo+AN_cumulo+interval_yield+Feed_Vol+R S_cumulo+AS_Vol.+emission_O2; 0.342, 0.677, tmp_cumulo+pH+P L_cumulo+AN_cumulo+interval_yield+interval_Pdt.+RS+RS_cumul o+rab_integral+RQ_integral+AS_Vol.+emission_CO2+interval_O 2; 0.363, 0.677, tmp_cumulo+PL_cumulo+interval_yield+interv al_Pdt.+OD+RS_cumulo+rab_integral+RQ_integral+AS_Vol.+emiss ion_CO2+interval_O2; 0.353, 0.677, tmp_cumulo+PL_cumulo+AN+ AN_cumulo+interval_yield+interval_Pdt.+OD+RS_cumulo+rab_int egral+RQ_integral+AS_Vol.+emission_CO2; 0.401, 0.677, tmp_c umulo+PL_cumulo+interval_yield+Feed_Vol+RS_cumulo+AS_Vol.+e mission_O2; 0.353, 0.677, tmp_cumulo+PL+AN_cumulo+interval_ yield+Feed_Vol+RS_cumulo+rab_integral+RQ_integral+AS_Vol.+e mission_CO2+consum_O2+interval_O2; 0.383, 0.677, O2+PL_cumu lo+interval_yield+OD+RS+Feed_Vol+AS_Vol.+emission_CO2+inter val_O2; 0.353, 0.677, tmp_cumulo+pH_cumulo+PL_cumulo+AN_cum ulo+interval_yield+Feed_Vol+RS_cumulo+rab_integral+RQ_integ ral+AS_Vol.+emission_O2+emission_CO2; 0.353, 0.677, pH+PL+P L_cumulo+AN_cumulo+interval_yield+interval_Pdt.+OD+RS_cumul o+rab_integral+RQ_integral+AS_Vol.+emission_CO2; 0.363, 0.6 77, tmp_cumulo+pH_cumulo+PL_cumulo+interval_yield+RS+Feed_V ol+RS_cumulo+rab_integral+RQ_integral+AS_Vol.+emission_CO2; 0.373, 0.677, tmp_cumulo+PL_cumulo+AN_cumulo+interval_yiel d+RS+Feed_Vol+RS_cumulo+AS_Vol. +emission_CO2+consum_O2; 0.3 63, 0.677, tmp_cumulo+pH_cumulo+PL_cumulo+AN_cumulo+interva l_yield+Feed_Vol+RS_cumulo+rab_integral+RQ_integral+AS_Vol. +emission_O2; 0.373, 0.677, O2+PL_cumulo+AN_cumulo+interval _yield+Feed_Vol+RS_cumulo+RQ_integral+AS_Vol.+emission_CO2+ interval_O2; 0.353, 0.677, tmp_cumulo+PL+PL_cumulo+AN_cumul o+interval_yield+interval_Pdt.+OD+RS_cumulo+rab_integral+RQ _integral+AS_Vol.+emission_CO2; 0.373, 0.677, PL_cumulo+AN_ cumulo+interval_yield+RS+Feed_Vol+RS_cumulo+AS_Vol.+emissio n_O2+consum_O2+interval_O2; 0.363, 0.677, tmp_cumulo+pH+PL_ cumulo+AN+interval_yield+Feed_Vol+RS_cumulo+rab_integral+RQ _integral+AS_Vol.+emission_CO2; 0.373, 0.677, O2+PL_cumulo+ interval_yield+interval_Pdt.+Feed_Vol+RS_cumulo+rab_integra l+RQ_integral+AS_Vol.+emission_CO2

### [205. Linear Model that Predicts Arginine Product ion Amount in Interval 5]

0.490, 0.743, tmp_cumulo+pH+pH_cumulo+AN_cumulo+interval_yi eld+RS+Feed_Vol+Cell_conc._interval+AS_Vol.; 0.497, 0.742, tmp_cumulo+pH+pH_cumulo+AN_cumulo+interval_yield+RS+Cell_co nc._interval+AS_Vol.; 0.485, 0.740, tmp_cumulo+pH+pH_cumulo +AN_cumulo+interval_yield+RS+Cell_conc._interval+rab_integr al+AS_Vol.; 0.476, 0.739, tmp_cumulo+pH+pH_cumulo+AN_cumulo +interval_yield+RS+Feed_Vol+Cell_conc._interval+AS_Vol.+gen erate_CO2; 0.482, 0.738, tmp_cumulo+pH+pH_cumulo+AN+AN_cumu lo+interval_yield+RS+Cell_conc._interval+AS_Vol.; 0.474, 0. 738, tmp_cumulo+pH+pH_cumulo+AN+AN_cumulo+interval_yield+RS +Feed_Vol+Cell_conc._interval+AS_Vol.; 0.481, 0.738, tmp_cu mulo+pH+pH_cumulo+AN_cumulo+interval_yield+RS+Cell_conc._in terval+AS_Vol.+generate_CO2; 0.473, 0.738, tmp_cumulo+pH+pH _cumulo+AN_cumulo+interval_yield+RS+Feed_Vol+Cell_conc._int erval+AS_Vol.+emission_CO2; 0.473, 0.738, tmp_cumulo+pH+pH_ cumulo+AN_cumulo+interval_yield+RS+Feed_Vol+Cell_conc._inte rval+rab_integral+AS_Vol.; 0.489, 0.738, tmp_cumulo+pH+pH_c umulo+AN+interval_yield+RS+Cell_conc._interval+AS_Vol.; 0.4 73, 0.738, tmp+tmp_cumulo+pH+pH_cumulo+AN_cumulo+interval_y ield+RS+Feed_Vol+Cell_conc._interval+AS_Vol.; 0.473, 0.737, tmp_cumulo+pH+pH_cumulo+AN_cumulo+interval_yield+interval_ Pdt.+RS+Feed_Vol+Cell_conc._interval+AS_Vol.; 0.472, 0.737, tmp_cumulo+pH+pH_cumulo+PL_cumulo+AN_cumulo+interval_yield +RS+Feed_Vol+Cell_conc._interval+AS_Vol.; 0.480, 0.737, tmp _cumulo+pH+pH_cumulo+AN_cumulo+interval_yield+RS+Cell_conc. _interval+AS_Vol.+emission_CO2; 0.480, 0.737, tmp_cumulo+pH +pH_cumulo+AN+interval_yield+RS+Feed_Vol+Cell_conc._interva l+AS_Vol.; 0.480, 0.737, tmp+tmp_cumulo+pH+pH_cumulo+AN_cum ulo+interval_yield+RS+Cell_conc._interval+AS_Vol.; 0.478, 0. 736, tmp_cumulo+pH+pH_cumulo+AN_cumulo+interval_yield+inter val_Pdt.+RS+Cell_conc._interval+AS_Vol.; 0.470, 0.736, tmp_ cumulo+pH+pH_cumulo+AN_cumulo+interval_yield+RS+Feed_Vol+Ce ll_conc._interval+RQ_integral+AS_Vol.; 0.461, 0.736, tmp_cu mulo+pH+pH_cumulo+AN_cumulo+interval_yield+RS+Feed_Vol+Cell _conc._interval+AS_Vol.+emission_CO2+generate_CO2; 0.469, 0. 735, tmp_cumulo+pH+pH_cumulo+AN_cumulo+interval_yield+RS+Ce ll_conc._interval+rab_integral+AS_Vol.+generate_CO2; 0.461, 0.735, tmp_cumulo+pH+pH_cumulo+AN+AN_cumulo+interval_yield +RS+Feed_Vol+Cell_conc._interval+AS_Vol.+generate_CO2; 0.47 7, 0.735, tmp_cumulo+pH+pH_cumulo+PL_cumulo+AN_cumulo+inter val_yield+RS+Cell_conc._interval+AS_Vol.; 0.469, 0.735, tmp _cumulo+pH+pH_cumulo+AN_cumulo+interval_yield+RS+Cell_conc. _interval+AS_Vol.+emission_CO2+generate_CO2; 0.468, 0.735, tmp_cumulo+pH+pH_cumulo+AN_cumulo+interval_yield+RS+Feed_Vo l+Cell_conc._interval+AS_Vol.+emission_O2; 0.476, 0.735, tm p_cumulo+pH+pH_cumulo+PL+AN_cumulo+interval_yield+RS+Cell_c onc._interval+AS_Vol.; 0.484, 0.735, tmp_cumulo+pH+pH_cumul o+interval_yield+RS+Feed_Vol+Cell_conc._interval+AS_Vol.; 0. 468, 0.735, tmp_cumulo+pH+pH_cumulo+PL+AN_cumulo+interval_y ield+RS+Cell_conc._interval+rab_integral+AS_Vol.; 0.468, 0. 735, tmp_cumulo+pH+pH_cumulo+PL_cumulo+AN_cumulo+interval_y ield+RS+Cell_conc._interval+rab_integral+AS_Vol.; 0.468, 0. 735, tmp_cumulo+pH+pH_cumulo+AN+AN_cumulo+interval_yield+RS +Cell_conc._interval+rab_integral+AS_Vol.; 0.492, 0.735, tm p_cumulo+pH+pH_cumulo+interval_yield+RS+Feed_Vol+Cell_conc. _interval; 0.459, 0.735, tmp_cumulo+pH+pH_cumulo+AN_cumulo+ interval_yield+RS+Feed_Vol+Cell_conc._interval+rab_integral +AS_Vol.+generate_CO2; 0.468, 0.735, tmp_cumulo+pH+pH_cumul o+AN_cumulo+interval_yield+RS+Feed_Vol+Cell_conc._interval+ AS_Vol.+consum_O2; 0.476, 0.734, tmp_cumulo+pH+pH_cumulo+AN _cumulo+interval_yield+RS+Cell_conc._interval+AS_Vol.+consu m_O2; 0.467, 0.734, tmp_cumulo+pH+pH_cumulo+AN+interval_yie ld+RS+Feed_Vol+Cell_conc._interval+AS_Vol.+generate_CO2; 0. 467, 0.734, tmp_cumulo+pH+pH_cumulo+AN_cumulo+interval_yiel d+RS+Cell_conc._interval+rab_integral+AS_Vol.+emission_CO2; 0.459, 0.734, tmp_cumulo+pH+pH_cumulo+PL+AN_cumulo+interva l_yield+RS+Cell_conc._interval+rab_integral+AS_Vol.+emissio n_CO2; 0.467, 0.734, tmp_cumulo+pH+pH_cumulo+AN+AN_cumulo+i nterval_yield+RS+Cell_conc._interval+AS_Vol.+generate_CO2; 0.475, 0.734, tmp_cumulo+pH+pH_cumulo+AN_cumulo+interval_yi eld+RS+RS_cumulo+Cell_conc._interval+AS_Vol.; 0.467, 0.734, tmp+tmp_cumulo+pH+pH_cumulo+AN_cumulo+interval_yield+RS+Ce ll_conc._interval+rab_integral+AS_Vol.; 0.467, 0.734, tmp_c umulo+pH+pH_cumulo+AN_cumulo+interval_yield+RS+Feed_Vol+RS_ cumulo+Cell_conc._interval+AS_Vol.; 0.475, 0.734, tmp_cumul o+pH+pH_cumulo+AN+interval_yield+RS+Cell_conc._interval+AS_ Vol.+generate_CO2; 0.475, 0.734, tmp_cumulo+pH+pH_cumulo+AN _cumulo+interval_yield+RS+Cell_conc._interval+RQ_integral+A S_Vol.; 0.458, 0.734, tmp+tmp_cumulo+pH+pH_cumulo+AN_cumulo +interval_yield+RS+Feed_Vol+Cell_conc._interval+AS_Vol.+gen erate_CO2; 0.482, 0.734, tmp_cumulo+pH+pH_cumulo+AN_cumulo+ interval_yield+RS+Feed_Vol+Cell_conc._interval; 0.474, 0.73 4, tmp_cumulo+pH+pH_cumulo+AN+interval_yield+RS+Cell_conc._ interval+rab_integral+AS_Vol.; 0.457, 0.733, tmp_cumulo+pH+ pH_cumulo+PL_cumulo+AN_cumulo+interval_yield+RS+FeedVol+Ce ll_conc._interval+AS_Vol.+generate_CO2; 0.474, 0.733, tmp_c umulo+pH+pH_cumulo+AN_cumulo+interval_yield+OD+RS+Cell_conc. _interval+AS_Vol.; 0.465, 0.733, tmp_cumulo+pH+pH_cumulo+AN _cumulo+interval_yield+interval_Pdt.+RS+Cell_conc._interval +rab_integral+AS_Vol.; 0.457, 0.733, tmp_cumulo+pH+pH_cumul o+PL_cumulo+AN_cumulo+interval_yield+RS+Feed_Vol+Cell_conc. _interval+rab_integral+AS_Vol.; 0.465, 0.733, tmp_cumulo+pH +pH_cumulo+PL+AN_cumulo+interval_yield+RS+Cell_conc._interv al+AS_Vol.+emission_CO2; 0.465, 0.733, tmp_cumulo+pH+pH_cum ulo+AN_cumulo+interval_yield+RS+Cell_conc._interval+rab_int egral+RQ_integral+AS_Vol.; 0.456, 0.733, tmp_cumulo+pH+pH_c umulo+AN+AN_cumulo+interval_yield+RS+Feed_Vol+Cell_conc._in terval+AS_Vol.+emission_CO2; 0.456, 0.733, tmp_cumulo+pH+pH _cumulo+AN_cumulo+interval_yield+RS+Feed_Vol+Cell_conc._int erval+rab_integral+RQ_integral+AS_Vol.; 0.456, 0.733, tmp+t mp_cumulo+pH+pH_cumulo+AN+AN_cumulo+interval_yield+RS+Feed_ Vol+Cell_conc._interval+AS_Vol.; 0.465, 0.733, tmp+tmp_cumu lo+pH+pH_cumulo+AN+AN_cumulo+interval_yield+RS+Cell_conc._i nterval+AS_Vol.; 0.456, 0.733, tmp_cumulo+pH+pH_cumulo+AN+A N_cumulo+interval_yield+RS+Feed_Vol+Cell_conc._interval+rab _integral+AS_Vol.; 0.456, 0.733, tmp_cumulo+pH+pH_cumulo+AN _cumulo+interval_yield+interval_Pdt.+RS+Feed_Vol+Cell_conc. _interval+rab_integral+AS_Vol.; 0.456, 0.733, tmp+tmp_cumul o+pH+pH_cumulo+AN_cumulo+interval_yield+RS+Feed_Vol+Cell_co nc._interval+AS_Vol.+emission_CO2; 0.456, 0.733, tmp_cumulo +pH+pH_cumulo+PL_cumulo+AN+AN_cumulo+interval_yield+RS+Feed _Vol+Cell_conc._interval+AS_Vol.; 0.456, 0.732, tmp_cumulo+ pH+pH_cumulo+AN_cumulo+interval_yield+RS+Feed_Vol+Cell_conc. _interval+rab_integral+AS_Vol.+emission_CO2; 0.455, 0.732, tmp+tmp_cumulo+pH+pH_cumulo+AN_cumulo+interval_yield+RS+Fee d_Vol+Cell_conc._interval+rab_integral+AS_Vol.; 0.455, 0.73 2, tmp_cumulo+pH+pH_cumulo+AN+AN_cumulo+interval_yield+inte rval_Pdt.+RS+Feed_Vol+Cell_conc._interval+AS_Vol.; 0.464, 0. 732, tmp_cumulo+pH+pH_cumulo+AN+AN_cumulo+interval_yield+RS +Cell_conc._interval+AS_Vol.+emission_CO2; 0.464, 0.732, tm p+tmp_cumulo+pH+pH_cumulo+AN_cumulo+interval_yield+RS+Cell_ conc._interval+AS_Vol.+generate_CO2; 0.472, 0.732, tmp+tmp_ cumulo+pH+pH_cumulo+AN+interval_yield+RS+Cell_conc._interva l+AS_Vol.; 0.455, 0.732, tmp_cumulo+pH+pH_cumulo+PL+AN_cumu lo+interval_yield+RS+Feed_Vol+Cell_conc._interval+AS_Vol.+i nterval_O2; 0.455, 0.732, tmp_cumulo+pH+pH_cumulo+AN_cumulo +interval_yield+interval_Pdt.+RS+Feed_Vol+Cell_conc._interv al+AS_Vol.+generate_CO2; 0.464, 0.732, tmp_cumulo+pH+pH_cum ulo+AN_cumulo+interval_yield+RS+Cell_conc._interval+rab_int egral+AS_Vol.+consum_O2; 0.455, 0.732, tmp_cumulo+pH+pH_cum ulo+AN_cumulo+interval_yield+interval_Pdt.+RS+Feed_Vol+Cell _conc._interval+AS_Vol.+emission_CO2; 0.455, 0.732, tmp_cum ulo+pH+pH_cumulo+AN_cumulo+interval_yield+RS+Cell_conc._int erval+rab_integral+AS_Vol.+emission_CO2+generate_CO2; 0.454, 0.732, tmp_cumulo+pH+pH_cumulo+AN+AN_cumulo+interval_yield +RS+Feed_Vol+Cell_conc._interval+RQ_integral+AS_Vol.; 0.463, 0.732, tmp_cumulo+pH+pH_cumulo+PL+AN+AN_cumulo+interval_yi eld+RS+Cell_conc._interval+AS_Vol.; 0.463, 0.732, tmp_cumul o+pH+pH_cumulo+PL+AN+interval_yield+RS+Cell_conc._interval+ AS_Vol.+interval_O2; 0.463, 0.732, tmp+tmp_cumulo+pH+pH_cum ulo+AN_cumulo+interval_yield+RS+Cell_conc._interval+AS_Vol. +emission_CO2; 0.463, 0.732, tmp+tmp_cumulo+pH+pH_cumulo+AN +interval_yield+RS+Feed_Vol+Cell_conc._interval+AS_Vol.; 0. 454, 0.732, tmp_cumulo+pH+pH_cumulo+PL_cumulo+AN_cumulo+int erval_yield+RS+Feed_Vol+Cell_conc._interval+RQ_integral+AS_ Vol.; 0.463, 0.732, tmp_cumulo+pH+pH_cumulo+AN_cumulo+inter val_yield+RS+RS_cumulo+Cell_conc._interval+rab_integral+AS_ Vol.; 0.487, 0.732, tmp_cumulo+pH+pH_cumulo+interval_yield+ RS+Cell_conc._interval+AS_Vol.; 0.454, 0.731, tmp+tmp_cumul o+pH+pH_cumulo+PL_cumulo+AN_cumulo+interval_yield+RS+Feed_V ol+Cell_conc._interval+AS_Vol.; 0.462, 0.731, tmp_cumulo+pH +pH_cumulo+AN+interval_yield+RS+Feed_Vol+Cell_conc._interva l+AS_Vol.+emission_CO2; 0.462, 0.731, tmp_cumulo+pH+pH_cumu lo+AN_cumulo+interval_yield+OD+RS+Feed_Vol+Cell_conc._inter val+AS_Vol.; 0.454, 0.731, tmp_cumulo+pH+pH_cumulo+PL+AN+in terval_yield+RS+Feed_Vol+Cell_conc._interval+AS_Vol.+interv al_O2; 0.454, 0.731, tmp_cumulo+pH+pH_cumulo+PL_cumulo+AN_c umulo+interval_yield+interval_Pdt.+RS+Feed_Vol+Cell_conc._i nterval+AS_Vol.; 0.462, 0.731, tmp_cumulo+pH+pH_cumulo+PL_c umulo+AN+AN_cumulo+interval_yield+RS+Cell_conc._interval+AS _Vol.; 0.454, 0.731, tmp_cumulo+pH+pH_cumulo+AN_cumulo+inte rval_yield+RS+Feed_Vol+Cell_conc._interval+RQ_integral+AS_V ol.+generate_CO2; 0.462, 0.731, tmp_cumulo+pH+pH_cumulo+AN+ interval_yield+RS+Feed_Vol+Cell_conc._interval+rab_integral +AS_Vol.; 0.462, 0.731, tmp_cumulo+pH+pH_cumulo+AN+interval _yield+interval_Pdt.+RS+Feed_Vol+Cell_conc._interval+AS_Vo l.; 0.453, 0.731, tmp+tmp_cumulo+pH+pH_cumulo+AN_cumulo+int erval_yield+interval_Pdt.+RS+Feed_Vol+Cell_conc._interval+A S_Vol.; 0.462, 0.731, tmp_cumulo+pH+pH_cumulo+PL+AN_cumulo+ interval_yield+RS+Feed_Vol+Cell_conc._interval+AS_Vol.; 0.4 44, 0.731, tmp_cumulo+pH+pH_cumulo+AN_cumulo+interval_yield +RS+Feed_Vol+Cell_conc._interval+rab_integral+AS_Vol.+emiss ion_CO2+generate_CO2; 0.470, 0.731, tmp_cumulo+pH+pH_cumulo +AN+interval_yield+RS+Cell_conc._interval+AS_Vol.+emission_ CO2; 0.453, 0.731, tmp_cumulo+pH+pH_cumulo+PL_cumulo+AN_cum ulo+interval_yield+RS+Feed_Vol+Cell_conc._interval+AS_Vol.+ emission_CO2; 0.461, 0.731, tmp_cumulo+pH+pH_cumulo+AN+AN_c umulo+interval_yield+interval_Pdt.+RS+Cell_conc._interval+A S_Vol.; 0.453, 0.731, tmp+tmp_cumulo+pH+pH_cumulo+AN_cumulo +interval_yield+RS+Feed_Vol+Cell_conc._interval+RQ_integral +AS_Vol.; 0.470, 0.731, tmp_cumulo+pH+pH_cumulo+AN_cumulo+i nterval_yield+RS+Cell_conc._interval+AS_Vol.+emission_O2; 0. 478, 0.731, tmp_cumulo+pH+pH_cumulo+interval_yield+RS+Feed_ Vol+Cell_conc._interval+rab_integral; 0.461, 0.731, tmp_cum ulo+pH+pH_cumulo+AN+interval_yield+RS+Feed_Vol+Cell_conc._i nterval+RQ_integral+AS_Vol.; 0.478, 0.731, tmp_cumulo+pH+pH _cumulo+interval_yield+RS+Feed_Vol+Cell_conc._interval+cons um_O2; 0.444, 0.731, tmp_cumulo+pH+pH_cumulo+AN+AN_cumulo+i nterval_yield+RS+Feed_Vol+Cell_conc._interval+AS_Vol.+emiss ion_CO2+generate_CO2; 0.453, 0.731, tmp_cumulo+pH+pH_cumulo +PL+AN_cumulo+interval_yield+RS+Cell_conc._interval+RQ_inte gral+AS_Vol.+emission_CO2; 0.461, 0.731, tmp_cumulo+pH+pH_c umulo+AN+interval_yield+RS+Feed_Vol+Cell_conc._interval+AS_ Vol.+emission_O2; 0.461, 0.731, tmp_cumulo+pH+pH_cumulo+AN+ AN_cumulo+interval_yield+RS+Cell_conc._interval+RQ_integral +AS_Vol.; 0.452, 0.731, tmp_cumulo+pH+pH_cumulo+AN+AN_cumul o+interval_yield+RS+Feed_Vol+Cell_conc._interval+AS_Vol.+em ission_O2; 0.477, 0.730, tmp_cumulo+pH+AN_cumulo+interval_y ield+RS+Feed_Vol+Cell_conc._interval+AS_Vol.; 0.461, 0.730, tmp_cumulo+pH+pH_cumulo+PL_cumulo+AN+interval_yield+RS+Fee d_Vol+Cell_conc._interval+AS_Vol.; 0.469, 0.730, tmp_cumulo +pH+pH_cumulo+AN+interval_yield+interval_Pdt.+RS+Cell_conc. _interval+AS_Vol.; 0.461, 0.730, tmp_cumulo+pH+pH_cumulo+AN +AN_cumulo+interval_yield+RS+Cell_conc._interval+AS_Vol.+co nsum_O2; 0.443, 0.730, tmp_cumulo+pH+pH_cumulo+PL_cumulo+AN _cumulo+interval_yield+RS+Feed_Vol+Cell_conc._interval+AS_V ol.+emission_CO2+generate_CO2; 0.452, 0.730, tmp_cumulo+pH+ pH_cumulo+AN_cumulo+interval_yield+RS+Feed_Vol+Cell_conc._i nterval+AS_Vol.+consum_O2+generate_CO2; 0.452, 0.730, tmp_c umulo+pH+pH_cumulo+PL+AN+AN_cumulo+interval_yield+RS+Cell_c onc._interval+rab_integral+AS_Vol.; 0.452, 0.730, tmp+tmp_c umulo+pH+pH_cumulo+AN_cumulo+interval_yield+RS+Feed_Vol+Cel l_conc._interval+AS_Vol.+emission_O2; 0.452, 0.730, tmp_cum ulo+pH+pH_cumulo+AN_cumulo+interval_yield+RS+Feed_Vol+RS_cu mulo+Cell_conc._interval+AS_Vol.+generate_CO2; 0.443, 0.730, tmp_cumulo+pH+pH_cumulo+AN+AN_cumulo+interval_yield+RS+Fee d_Vol+Cell_conc._interval+rab_integral+AS_Vol.+generate_CO 2; 0.460, 0.730, tmp+tmp_cumulo+pH+pH_cumulo+AN_cumulo+inte rval_yield+interval_Pdt.+RS+Cell_conc._interval+AS_Vol.; 0. 452, 0.730, tmp_cumulo+pH+pH_cumulo+AN+AN_cumulo+interval_y ield+RS+Cell_conc._interval+AS_Vol.+emission_CO2+generate_C O2; 0.460, 0.730, tmp_cumulo+pH+pH_cumulo+PL_cumulo+AN_cumu lo+interval_yield+RS+Cell_conc._interval+AS_Vol.+generate_C O2; 0.452, 0.730, tmp_cumulo+pH+pH_cumulo+PL_cumulo+AN+AN_c umulo+interval_yield+RS+Cell_conc._interval+rab_integral+AS _Vol.; 0.468, 0.730, tmp_cumulo+pH+pH_cumulo+AN+interval_yi eld+RS+Cell_conc._interval+RQ_integral+AS_Vol.; 0.468, 0.73 0, tmp_cumulo+pH+pH_cumulo+PL+AN+interval_yield+RS+Cell_con c._interval+AS_Vol.; 0.451, 0.730, tmp_cumulo+pH+pH_cumulo+ AN_cumulo+interval_yield+RS+Feed_Vol+Cell_conc._interval+ra b_integral+AS_Vol.+emission_O2; 0.468, 0.730, tmp_cumulo+pH +pH_cumulo+PL_cumulo+AN+interval_yield+RS+Cell_conc._interv al+AS_Vol.; 0.451, 0.730, tmp_cumulo+pH+pH_cumulo+PL_cumulo +AN_cumulo+interval_yield+RS+Cell_conc._interval+rab_integr al+AS_Vol.+generate_CO2; 0.460, 0.730, tmp_cumulo+pH+pH_cum ulo+AN_cumulo+interval_yield+interval_Pdt.+RS+Cell_conc._in terval+AS_Vol.+emission_CO2; 0.442, 0.730, tmp_cumulo+pH+pH _cumulo+PL_cumulo+AN+AN_cumulo+interval_yield+RS+Feed_Vol+C ell_conc._interval+AS_Vol.+generate_CO2; 0.476, 0.730, tmp_ cumulo+pH+pH_cumulo+interval_yield+RS+Feed_Vol+Cell_conc._i nterval+emission_CO2; 0.468, 0.730, tmp_cumulo+pH+pH_cumulo +AN_cumulo+interval_yield+RS+Feed_Vol+Cell_conc._interval+r ab_integral; 0.460, 0.730, tmp_cumulo+pH+pH_cumulo+AN+AN_cu mulo+interval_yield+OD+RS+Cell_conc._interval+AS_Vol.; 0.45 1, 0.730, tmp_cumulo+pH+pH_cumulo+AN+AN_cumulo+interval_yie ld+RS+Cell_conc._interval+rab_integral+AS_Vol.+generate_CO 2; 0.442, 0.730, tmp_cumulo+pH+pH_cumulo+PL+AN_cumulo+inter val_yield+RS+Cell_conc._interval+rab_integral+AS_Vol.+emiss ion_CO2+generate_CO2; 0.468, 0.730, tmp_cumulo+pH+pH_cumulo +AN+interval_yield+OD+RS+Cell_conc._interval+AS_Vol.; 0.468, 0.730, tmp_cumulo+pH+pH_cumulo+AN_cumulo+interval_yield+in terval_Pdt.+RS+Feed_Vol+Cell_conc._interval; 0.451, 0.730, tmp_cumulo+pH+pH_cumulo+AN_cumulo+interval_yield+interval_P dt.+RS+Feed_Vol+Cell_conc._interval+RQ_integral+AS_Vol.; 0. 468, 0.730, tmp_cumulo+pH+pH_cumulo+AN_cumulo+interval_yiel d+RS+Cell_conc._interval+Accum._Yield+AS_Vol.; 0.460, 0.730, tmp_cumulo+pH+pH_cumulo+AN+AN_cumulo+interval_yield+RS+RS_ cumulo+Cell_conc._interval+AS_Vol.; 0.468, 0.730, tmp_cumul o+pH+pH_cumulo+AN+interval_yield+RS+Cell_conc._interval+AS_ Vol.+consum_O2; 0.468, 0.730, tmp_cumulo+pH+pH_cumulo+inter val_yield+RS+Feed_Vol+Cell_conc._interval+AS_Vol.+emission_ CO2; 0.459, 0.730, tmp_cumulo+pH+pH_cumulo+AN_cumulo+interv al_yield+interval_Pdt.+RS+Cell_conc._interval+AS_Vol.+gener ate_CO2; 0.451, 0.730, tmp_cumulo+pH+pH_cumulo+AN_cumulo+in terval_yield+RS+Cell_conc._interval+RQ_integral+AS_Vol.+emi ssion_O2+emission_CO2; 0.459, 0.730, tmp_cumulo+pH+pH_cumul o+AN_cumulo+interval_yield+OD+RS+Cell_conc._interval+rab_in tegral+AS_Vol.; 0.451, 0.730, tmp_cumulo+pH+pH_cumulo+PL_cu mulo+AN_cumulo+interval_yield+RS+Cell_conc._interval+rab_in tegral+AS_Vol.+emission_CO2; 0.451, 0.730, tmp_cumulo+pH+pH _cumulo+AN+interval_yield+RS+Feed_Vol+Cell_conc._interval+r ab_integral+AS_Vol.+generate_CO2; 0.451, 0.730, tmp+tmp_cum ulo+pH+pH_cumulo+AN_cumulo+interval_yield+RS+Cell_conc._int erval+rab_integral+AS_Vol.+generate_CO2; 0.459, 0.730, tmp_ cumulo+pH+pH_cumulo+PL+interval_yield+RS+Feed_Vol+Cell_conc. _interval+AS_Vol.+interval_O2; 0.451, 0.730, tmp_cumulo+pH+ pH_cumulo+AN+AN_cumulo+interval_yield+RS+Feed_Vol+Cell_conc. _interval+AS_Vol.+consum_O2; 0.467, 0.730, tmp_cumulo+pH+pH _cumulo+AN_cumulo+interval_yield+RS+Feed_Vol+Cell_conc._int erval+generate_CO2; 0.475, 0.729, tmp_cumulo+pH+pH_cumulo+i nterval_yield+RS+Feed_Vol+Cell_conc._interval+generate_CO2; 0.450, 0.729, tmp_cumulo+pH+pH_cumulo+AN_cumulo+interval_y ield+RS+Feed_Vol+Cell_conc._interval+rab_integral+AS_Vol.+c onsum_O2; 0.467, 0.729, tmp_cumulo+pH+pH_cumulo+interval_yi eld+RS+Feed_Vol+Cell_conc._interval+AS_Vol.+generate_CO2; 0. 441, 0.729, tmp+tmp_cumulo+pH+pH_cumulo+AN_cumulo+interval_ yield+RS+Feed_Vol+Cell_conc._interval+AS_Vol.+emission_CO2+ generate_CO2; 0.441, 0.729, tmp_cumulo+pH+pH_cumulo+PL+AN+A N_cumulo+interval_yield+RS+Cell_conc._interval+rab_integral +AS_Vol.+emission_CO2; 0.450, 0.729, tmp_cumulo+pH+pH_cumul o+PL+AN+interval_yield+RS+Cell_conc._interval+rab_integral+ AS_Vol.+interval_O2; 0.450, 0.729, tmp_cumulo+pH+pH_cumulo+ PL+AN_cumulo+interval_yield+RS+Cell_conc._interval+AS_Vol.+ emission_CO2+generate_CO2; 0.459, 0.729, tmp_cumulo+pH+pH_c umulo+AN_cumulo+interval_yield+RS+Cell_conc._interval+rab_i ntegral+AS_Vol.+emission_O2; 0.441, 0.729, tmp+tmp_cumulo+p H+pH_cumulo+AN+AN_cumulo+interval_yield+RS+Feed_Vol+Cell_co nc._interval+AS_Vol.+generate_CO2; 0.459, 0.729, tmp_cumulo +pH+pH_cumulo+AN_cumulo+interval_yield+RS+RS_cumulo+Cell_co nc._interval+AS_Vol.+generate_CO2; 0.459, 0.729, tmp_cumulo +pH+pH_cumulo+PL_cumulo+AN_cumulo+interval_yield+RS+Cell_co nc._interval+AS_Vol.+emission_CO2; 0.475, 0.729, tmp_cumulo +pH+pH_cumulo+interval_yield+interval_Pdt.+RS+Feed_Vol+Cell _conc._interval; 0.441, 0.729, tmp_cumulo+pH+pH_cumulo+PL+A N_cumulo+interval_yield+RS+Cell_conc._interval+rab_integral +AS_Vol.+emission_CO2+interval_O2; 0.467, 0.729, tmp_cumulo +pH+pH_cumulo+AN+interval_yield+RS+RS_cumulo+Cell_conc._int erval+AS_Vol.; 0.459, 0.729, tmp+tmp_cumulo+pH+pH_cumulo+PL +AN_cumulo+interval_yield+RS+Cell_conc._interval+AS_Vol.; 0. 450, 0.729, tmp_cumulo+pH+pH_cumulo+AN+AN_cumulo+interval_y ield+RS+Feed_Vol+RS_cumulo+Cell_conc._interval+AS_Vol. ; 0.4 50, 0.729, tmp_cumulo+pH+pH_cumulo+AN_cumulo+interval_yield +RS+Feed_Vol+Cell_conc._interval+AS_Vol.+emission_O2+emissi on_CO2; 0.450, 0.729, tmp_cumulo+pH+pH_cumulo+AN_cumulo+int erval_yield+RS+Feed_Vol+Cell_conc._interval+AS_Vol.+emissio n_O2+generate_CO2; 0.467, 0.729, tmp_cumulo+pH+pH_cumulo+AN +interval_yield+RS+Cell_conc._interval+AS_Vol.+emission_O2; 0.441, 0.729, tmp_cumulo+pH+pH_cumulo+PL_cumulo+AN_cumulo+ interval_yield+RS+Feed_Vol+Cell_conc._interval+rab_integral +AS_Vol.+generate_CO2; 0.475, 0.729, tmp_cumulo+pH+pH_cumul o+AN+interval_yield+RS+Feed_Vol+Cell_conc._interval; 0.450, 0.729, tmp_cumulo+pH+pH_cumulo+AN+interval_yield+RS+Feed_V ol+Cell_conc._interval+AS_Vol.+emission_CO2+generate_CO2; 0. 458, 0.729, tmp_cumulo+pH+pH_cumulo+PL+AN_cumulo+interval_y ield+RS+Cell_conc._interval+AS_Vol.+interval_O2; 0.490, 0.7 29, tmp_cumulo+pH+interval_yield+RS+Feed_Vol+Cell_conc._int erval; 0.441, 0.729, tmp_cumulo+pH+pH_cumulo+AN_cumulo+inte rval_yield+RS+Feed_Vol+Cell_conc._interval+RQ_integral+AS_V ol.+emission_O2+emission_CO2; 0.475, 0.729, tmp_cumulo+pH+p H_cumulo+interval_yield+RS+Feed_Vol+Cell_conc._interval+int erval_O2; 0.450, 0.729, tmp_cumulo+pH+pH_cumulo+AN_cumulo+i nterval_yield+RS+Feed_Vol+Cell_conc._interval+AS_Vol.+emiss ion_CO2+consum_O2; 0.458, 0.729, tmp+tmp_cumulo+pH+pH_cumul o+PL_cumulo+AN_cumulo+interval_yield+RS+Cell_conc._interval +AS_Vol.; 0.458, 0.729, tmp_cumulo+pH+pH_cumulo+AN+interval _yield+RS+Cell_conc._interval+rab_integral+AS_Vol.+generate _CO2; 0.450, 0.729, tmp_cumulo+pH+pH_cumulo+PL+AN_cumulo+in terval_yield+RS+Cell_conc._interval+rab_integral+RQ_integra l+AS_Vol.; 0.458, 0.729, tmp+tmp_cumulo+pH+pH_cumulo+AN_cum ulo+interval_yield+RS+Cell_conc._interval+AS_Vol.+consum_O 2; 0.450, 0.729, tmp+tmp_cumulo+pH+pH_cumulo+AN_cumulo+inte rval_yield+RS+Feed_Vol+Cell_conc._interval+AS_Vol.+consum_O 2; 0.458, 0.729, tmp_cumulo+pH+pH_cumulo+AN+interval_yield+ RS+Cell_conc._interval+AS_Vol.+emission_CO2+generate_CO2; 0. 458, 0.729, tmp_cumulo+pH+pH_cumulo+PL+AN_cumulo+interval_y ield+interval_Pdt.+RS+Cell_conc._interval+AS_Vol.; 0.458, 0. 729, tmp_cumulo+pH+pH_cumulo+AN_cumulo+interval_yield+RS+Ce ll_conc._interval+AS_Vol.+consum_O2+generate_CO2; 0.450, 0. 729, tmp_cumulo+pH+pH_cumulo+PL+AN+AN_cumulo+interval_yield +RS+Cell_conc._interval+AS_Vol.+emission_CO2; 0.458, 0.729, tmp_cumulo+pH+pH_cumulo+AN+interval_yield+RS+Feed_Vol+Cell _conc._interval+AS_Vol.+consum_O2; 0.441, 0.729, tmp+tmp_cu mulo+pH+pH_cumulo+AN_cumulo+interval_yield+RS+Feed_Vol+Cell _conc._interval+rab_integral+AS_Vol.+generate_CO2; 0.449, 0. 729, tmp_cumulo+pH+pH_cumulo+PL_cumulo+AN_cumulo+interval_y ield+RS+Cell_conc._interval+AS_Vol.+emission_CO2+generate_C O2; 0.466, 0.729, tmp+tmp_cumulo+pH+pH_cumulo+interval_yiel d+RS+Feed_Vol+Cell_conc._interval+AS_Vol.; 0.440, 0.729, tm p_cumulo+pH+pH_cumulo+AN_cumulo+interval_yield+interval_Pdt. +RS+Feed_Vol+Cell_conc._interval+AS_Vol.+emission_CO2+gener ate_CO2; 0.466, 0.729, tmp_cumulo+pH+pH_cumulo+AN_cumulo+in terval_yield+RS+Feed_Vol+Cell_conc._interval+emission_CO2; 0.449, 0.729, tmp_cumulo+pH+pH_cumulo+AN_cumulo+interval_yi eld+RS+Feed_Vol+RS_cumulo+Cell_conc._interval+AS_Vol.+emiss ion_CO2; 0.449, 0.729, tmp+tmp_cumulo+pH+pH_cumulo+AN+AN_cu mulo+interval_yield+RS+Cell_conc._interval+rab_integral+AS_ Vol.; 0.449, 0.729, tmp_cumulo+pH+pH_cumulo+AN+AN_cumulo+in terval_yield+RS+Cell_conc._interval+rab_integral+RQ_integra l+AS_Vol.; 0.449, 0.729, tmp+tmp_cumulo+pH+pH_cumulo+AN_cum ulo+interval_yield+RS+Feed_Vol+RS_cumulo+Cell_conc._interva l+AS_Vol.; 0.458, 0.729, tmp_cumulo+pH+pH_cumulo+AN_cumulo+ interval_yield+RS+RS_cumulo+Cell_conc._interval+AS_Vol.+emi ssion_CO2; 0.458, 0.729, tmp+tmp_cumulo+pH+pH_cumulo+AN_cum ulo+interval_yield+RS+RS_cumulo+Cell_conc._interval+AS_Vo l.; 0.449, 0.729, tmp_cumulo+pH+pH_cumulo+PL+AN_cumulo+inte rval_yield+RS+Cell_conc._interval+rab_integral+AS_Vol.+inte rval_O2; 0.449, 0.729, tmp_cumulo+pH+pH_cumulo+AN_cumulo+in terval_yield+RS+Feed_Vol+RS_cumulo+Cell_conc._interval+rab_ integral+AS_Vol.; 0.449, 0.729, tmp+tmp_cumulo+pH+pH_cumulo +AN_cumulo+interval_yield+RS+Cell_conc._interval+AS_Vol.+em ission_CO2+generate_CO2; 0.466, 0.729, tmp_cumulo+pH+pH_cum ulo+interval_yield+RS+Feed_Vol+Cell_conc._interval+AS_Vol.+ consum_O2; 0.449, 0.729, tmp+tmp_cumulo+pH+pH_cumulo+AN_cum ulo+interval_yield+RS+Cell_conc._interval+rab_integral+AS_V ol.+emission_CO2; 0.458, 0.729, tmp_cumulo+pH+pH_cumulo+AN_ cumulo+interval_yield+RS+Cell_conc._interval+AS_Vol.+emissi on_CO2+consum_O2; 0.458, 0.729, tmp+tmp_cumulo+pH+pH_cumulo +AN_cumulo+interval_yield+RS+Cell_conc._interval+RQ_integra l+AS_Vol.; 0.458, 0.729, tmp_cumulo+pH+pH_cumulo+PL_cumulo+ AN_cumulo+interval_yield+interval_Pdt.+RS+Cell_conc._interv al+AS_Vol.; 0.440, 0.729, tmp_cumulo+pH+pH_cumulo+PL+AN_cum ulo+interval_yield+RS+Cell_conc._interval+rab_integral+RQ_i ntegral+AS_Vol.+emission_CO2; 0.474, 0.729, tmp_cumulo+pH+p H_cumulo+interval_yield+RS+Cell_conc._interval+rab_integral +AS_Vol.; 0.466, 0.729, tmp_cumulo+pH+pH_cumulo+interval_yi eld+RS+Feed_Vol+Cell_conc._interval+rab_integral+AS_Vol.; 0. 474, 0.729, tmp+pH+pH_cumulo+AN_cumulo+interval_yield+RS+Ce ll_conc._interval+AS_Vol.; 0.440, 0.729, tmp_cumulo+pH+pH_c umulo+AN_cumulo+interval_yield+RS+Feed_Vol+Cell_conc._inter val+rab_integral+RQ_integral+AS_Vol.+generate_CO2; 0.449, 0. 729, tmp+tmp_cumulo+pH+pH_cumulo+PL+AN_cumulo+interval_yiel d+RS+Cell_conc._interval+rab_integral+AS_Vol.; 0.440, 0.729, tmp_cumulo+pH+pH_cumulo+PL_cumulo+AN+interval_yield+RS+Fee d_Vol+Cell_conc._interval+AS_Vol.+interval_O2+generate_CO2; 0.457, 0.729, tmp_cumulo+pH+pH_cumulo+AN_cumulo+interval_y ield+OD+RS+Cell_conc._interval+AS_Vol.+generate_CO2; 0.449, 0.729, tmp_cumulo+pH+pH_cumulo+AN+AN_cumulo+interval_yield +RS+Cell_conc._interval+rab_integral+AS_Vol.+emission_CO2; 0.449, 0.728, tmp_cumulo+pH+pH_cumulo+PL+AN_cumulo+interval _yield+RS+Cell_conc._interval+rab_integral+AS_Vol.+generate _CO2; 0.449, 0.728, tmp_cumulo+pH+pH_cumulo+PL+AN_cumulo+in terval_yield+interval_Pdt.+RS+Cell_conc._interval+rab_integ ral+AS_Vol.; 0.440, 0.728, tmp_cumulo+pH+pH_cumulo+AN+AN_cu mulo+interval_yield+RS+Feed_Vol+Cell_conc._interval+rab_int egral+RQ_integral+AS_Vol.; 0.466, 0.728, tmp_cumulo+pH+pH_c umulo+AN+AN_cumulo+interval_yield+RS+Feed_Vol+Cell_conc._in terval; 0.449, 0.728, tmp_cumulo+pH+pH_cumulo+AN_cumulo+int erval_yield+RS+Cell_conc._interval+rab_integral+AS_Vol.+emi ssion_O2+emission_CO2; 0.474, 0.728, tmp_cumulo+pH+pH_cumul o+interval_yield+RS+Feed_Vol+Cell_conc._interval+RQ_integra 1; 0.440, 0.728, tmp_cumulo+pH+pH_cumulo+PL_cumulo+AN_cumul o+interval_yield+RS+Cell_conc._interval+rab_integral+AS_Vol. +emission_CO2+generate_CO2; 0.440, 0.728, tmp_cumulo+pH+pH_ cumulo+PL_cumulo+AN_cumulo+interval_yield+RS+Feed_Vol+Cell_ conc._interval+rab_integral+RQ_integral+AS_Vol.; 0.457, 0.7 28, tmp_cumulo+pH+pH_cumulo+PL+AN_cumulo+interval_yield+RS+ Cell_conc._interval+RQ_integral+AS_Vol.; 0.448, 0.728, tmp+ tmp_cumulo+pH+pH_cumulo+AN+interval_yield+RS+Feed_Vol+Cell_ conc._interval+AS_Vol.+generate_CO2; 0.448, 0.728, tmp_cumu lo+pH+pH_cumulo+AN_cumulo+interval_yield+RS+Feed_Vol+Cell_c onc._interval+RQ_integral+AS_Vol.+emission_CO2; 0.473, 0.72 8, tmp_cumulo+pH+pH_cumulo+interval_yield+RS+Feed_Vol+Cell_ conc._interval+emission_O2; 0.465, 0.728, tmp_cumulo+pH+pH_ cumulo+interval_yield+RS+Feed_Vol+Cell_conc._interval+inter val_O2+generate_CO2; 0.448, 0.728, tmp_cumulo+pH+pH_cumulo+ PL_cumulo+AN+interval_yield+RS+Feed_Vol+Cell_conc._interval +AS_Vol.+generate_CO2; 0.439, 0.728, tmp_cumulo+pH+pH_cumul o+AN_cumulo+interval_yield+RS+Cell_conc._interval+rab_integ ral+RQ_integral+AS_Vol.+emission_O2+emission_CO2; 0.465, 0. 728, tmp_cumulo+pH+pH_cumulo+AN_cumulo+interval_yield+RS+Fe ed_Vol+Cell_conc._interval+consum_O2; 0.465, 0.728, tmp_cum ulo+pH+pH_cumulo+interval_yield+RS+Feed_Vol+Cell_conc._inte rval+rab_integral+consum_O2; 0.448, 0.728, tmp_cumulo+pH+pH _cumulo+PL_cumulo+AN_cumulo+interval_yield+RS+Cell_conc._in terval+rab_integral+RQ_integral+AS_Vol.; 0.448, 0.728, tmp_ cumulo+pH+pH_cumulo+AN_cumulo+interval_yield+interval_Pdt.+ RS+Feed_Vol+Cell_conc._interval+AS_Vol.+consum_O2; 0.439, 0. 728, tmp_cumulo+pH+pH_cumulo+PL+AN_cumulo+interval_yield+RS +Feed_Vol+Cell_conc._interval+rab_integral+AS_Vol.+interval _O2; 0.448, 0.728, tmp+tmp_cumulo+pH+pH_cumulo+PL_cumulo+AN _cumulo+interval_yield+RS+Cell_conc._interval+rab_integral+ AS_Vol.; 0.457, 0.728, tmp_cumulo+pH+pH_cumulo+AN+AN_cumulo +interval_yield+RS+Cell_conc._interval+AS_Vol.+emission_O2; 0.448, 0.728, tmp_cumulo+pH+pH_cumulo+PL_cumulo+AN_cumulo+ interval_yield+interval_Pdt.+RS+Cell_conc._interval+rab_int egral+AS_Vol.; 0.439, 0.728, tmp_cumulo+pH+pH_cumulo+PL_cum ulo+AN+AN_cumulo+interval_yield+RS+Feed_Vol+Cell_conc._inte rval+rab_integral+AS_Vol.; 0.457, 0.728, tmp+tmp_cumulo+pH+ pH_cumulo+AN_cumulo+interval_yield+OD+RS+Cell_conc._interva l+AS_Vol.; 0.457, 0.728, tmp_cumulo+pH+pH_cumulo+AN+interva l_yield+RS+Feed_Vol+RS_cumulo+Cell_conc._interval+AS_Vol.; 0.448, 0.728, tmp+tmp_cumulo+pH+pH_cumulo+AN+AN_cumulo+inte rval_yield+RS+Cell_conc._interval+AS_Vol.+generate_CO2; 0.4 73, 0.728, tmp+tmp_cumulo+pH+pH_cumulo+interval_yield+RS+Fe ed_Vol+Cell_conc._interval; 0.465, 0.728, tmp_cumulo+pH+pH_ cumulo+interval_yield+interval_Pdt.+RS+Feed_Vol+Cell_conc._ interval+AS_Vol.; 0.448, 0.728, tmp_cumulo+pH+pH_cumulo+PL_ cumulo+AN_cumulo+interval_yield+RS+Feed_Vol+Cell_conc._inte rval+AS_Vol.+consum_O2; 0.439, 0.728, tmp_cumulo+pH+pH_cumu lo+AN+AN_cumulo+interval_yield+RS+Feed_Vol+Cell_conc._inter val+RQ_integral+AS_Vol.+generate_CO2; 0.448, 0.728, tmp_cum ulo+pH+pH_cumulo+AN_cumulo+interval_yield+RS+Feed_Vol+Cell_ conc._interval+RQ_integral+AS_Vol.+emission_O2; 0.465, 0.72 8, tmp_cumulo+pH+pH_cumulo+interval_yield+RS+Feed_Vol+Cell_ conc._interval+RQ_integral+AS_Vol.; 0.456, 0.728, tmp_cumul o+pH+pH_cumulo+AN_cumulo+interval_yield+OD+RS+Cell_conc._in terval+AS_Vol.+emission_CO2; 0.456, 0.728, tmp_cumulo+pH+pH _cumulo+PL+AN+interval_yield+RS+Cell_conc._interval+rab_int egral+AS_Vol.; 0.439, 0.728, tmp+tmp_cumulo+pH+pH_cumulo+PL +AN_cumulo+interval_yield+RS+Cell_conc._interval+rab_integr al+AS_Vol.+emission_CO2; 0.448, 0.728, tmp_cumulo+pH+pH_cum ulo+AN+interval_yield+RS+Feed_Vol+Cell_conc._interval+RQ_in tegral+AS_Vol.+generate_CO2; 0.439, 0.728, tmp_cumulo+pH+pH _cumulo+AN+AN_cumulo+interval_yield+interval_Pdt.+RS+Feed_V ol+Cell_conc._interval+AS_Vol.+generate_CO2; 0.448, 0.728, tmp_cumulo+pH+pH_cumulo+PL+AN_cumulo+interval_yield+RS+Feed _Vol+Cell_conc._interval+rab_integral+AS_Vol.; 0.456, 0.728, tmp_cumulo+pH+pH_cumulo+interval_yield+RS+Feed_Vol+Cell_co nc._interval+rab_integral+interval_O2+generate_CO2; 0.448, 0.728, tmp_cumulo+pH+pH_cumulo+AN_cumulo+interval_yield+RS+ Feed_Vol+Cell_conc._interval+RQ_integral+AS_Vol.+consum_O2; 0.456, 0.728, tmp_cumulo+pH+pH_cumulo+AN_cumulo+interval_y ield+RS+Feed_Vol+Cell_conc._interval+AS_Vol.+interval_O2; 0. 439, 0.728, tmp_cumulo+pH+pH_cumulo+AN_cumulo+interval_yiel d+RS+Feed_Vol+Cell_conc._interval+AS_Vol.+emission_O2+emiss ion_CO2+generate_CO2; 0.456, 0.728, tmp_cumulo+pH+pH_cumulo +AN_cumulo+interval_yield+RS+Feed_Vol+Cell_conc._interval+r ab_integral+generate_CO2; 0.447, 0.728, tmp_cumulo+pH+pH_cu mulo+PL+AN_cumulo+interval_yield+RS+Cell_conc._interval+AS_ Vol.+emission_CO2+interval_O2; 0.438, 0.728, tmp+tmp_cumulo +pH+pH_cumulo+AN+AN_cumulo+interval_yield+RS+Feed_Vol+Cell_ conc._interval+AS_Vol.+emission_CO2; 0.456, 0.728, tmp_cumu lo+pH+pH_cumulo+AN_cumulo+interval_yield+RS+Cell_conc._inte rval+AS_Vol.+emission_O2+emission_CO2; 0.456, 0.728, tmp_cu mulo+pH+pH_cumulo+PL_cumulo+AN_cumulo+interval_yield+RS+Cel l_conc._interval+RQ_integral+AS_Vol.; 0.456, 0.728, tmp+tmp _cumulo+pH+pH_cumulo+AN+interval_yield+RS+Cell_conc._interv al+AS_Vol.+generate_CO2; 0.456, 0.728, tmp_cumulo+pH+pH_cum ulo+AN+interval_yield+RS+Cell_conc._interval+rab_integral+R Q_integral+AS_Vol.; 0.438, 0.728, tmp+tmp_cumulo+pH+pH_cumu lo+AN_cumulo+interval_yield+RS+Feed_Vol+Cell_conc._interval +rab_integral+RQ_integral+AS_Vol.; 0.456, 0.728, tmp+tmp_cu mulo+pH+pH_cumulo+AN+interval_yield+RS+Cell_conc._interval+ rab_integral+AS_Vol.; 0.447, 0.728, tmp_cumulo+pH+pH_cumulo +PL_cumulo+AN_cumulo+interval_yield+RS+Feed_Vol+RS_cumulo+C ell_conc._interval+AS_Vol.; 0.447, 0.728, tmp_cumulo+pH+pH_ cumulo+AN_cumulo+interval_yield+interval_Pdt.+RS+Feed_Vol+C ell_conc._interval+AS_Vol.+emission_O2; 0.447, 0.728, tmp_c umulo+pH+pH_cumulo+PL_cumulo+AN+AN_cumulo+interval_yield+RS +Cell_conc._interval+AS_Vol.+generate_CO2; 0.438, 0.728, tm p_cumulo+pH+pH_cumulo+PL_cumulo+AN_cumulo+interval_yield+RS +Feed_Vol+Cell_conc._interval+AS_Vol.+interval_O2+generate_ CO2; 0.456, 0.728, tmp_cumulo+pH+pH_cumulo+PL_cumulo+AN+int erval_yield+RS+Cell_conc._interval+rab_integral+AS_Vol.; 0. 447, 0.728, tmp_cumulo+pH+pH_cumulo+PL+AN+interval_yield+RS +Cell_conc._interval+AS_Vol.+emission_CO2+interval_O2; 0.44 7, 0.728, tmp_cumulo+pH+pH_cumulo+AN+AN_cumulo+interval_yie ld+interval_Pdt.+RS+Cell_conc._interval+rab_integral+AS_Vo l.; 0.438, 0.728, tmp_cumulo+pH+pH_cumulo+PL_cumulo+AN_cumu lo+interval_yield+interval_Pdt.+RS+Feed_Vol+Cell_conc._inte rval+rab_integral+AS_Vol.; 0.447, 0.728, tmp_cumulo+pH+pH_c umulo+AN_cumulo+interval_yield+interval_Pdt.+RS+Cell_conc._ interval+rab_integral+AS_Vol.+emission_CO2; 0.447, 0.727, t mp_cumulo+pH+pH_cumulo+AN_cumulo+interval_yield+interval_Pd t.+RS+Feed_Vol+RS_cumulo+Cell_conc._interval+AS_Vol.; 0.438, 0.727, tmp_cumulo+pH+pH_cumulo+PL+AN_cumulo+interval_yield +interval_Pdt.+RS+Cell_conc._interval+rab_integral+AS_Vol.+ emission_CO2; 0.456, 0.727, tmp_cumulo+pH+pH_cumulo+AN_cumu lo+interval_yield+RS+Feed_Vol+Cell_conc._interval+Accum._Yi eld+AS_Vol.; 0.447, 0.727, tmp_cumulo+pH+pH_cumulo+PL_cumul o+AN_cumulo+interval_yield+RS+Feed_Vol+Cell_conc._interval+ AS_Vol.+interval_O2; 0.447, 0.727, tmp+tmp_cumulo+pH+pH_cum ulo+AN_cumulo+interval_yield+RS+Cell_conc._interval+rab_int egral+RQ_integral+AS_Vol.; 0.447, 0.727, tmp_cumulo+pH+pH_c umulo+AN+interval_yield+RS+Feed_Vol+Cell_conc._interval+rab _integral+RQ_integral+AS_Vol.; 0.464, 0.727, tmp_cumulo+pH+ pH_cumulo+AN_cumulo+interval_yield+RS+Feed_Vol+Cell_conc._i nterval+RQ_integral; 0.447, 0.727, tmp_cumulo+pH+pH_cumulo+ AN_cumulo+interval_yield+OD+RS+Feed_Vol+Cell_conc._interval +AS_Vol.+generate_CO2; 0.464, 0.727, tmp_cumulo+pH+pH_cumul o+interval_yield+RS+Feed_Vol+Cell_conc._interval+rab_integr al+RQ_integral; 0.464, 0.727, tmp+tmp_cumulo+pH+pH_cumulo+A N_cumulo+interval_yield+RS+Feed_Vol+Cell_conc._interval; 0. 438, 0.727, tmp+tmp_cumulo+pH+pH_cumulo+AN+AN_cumulo+interv al_yield+RS+Feed_Vol+Cell_conc._interval+rab_integral+AS_Vo l.; 0.438, 0.727, tmp+tmp_cumulo+pH+pH_cumulo+PL_cumulo+AN_ cumulo+interval_yield+RS+Feed_Vol+Cell_conc._interval+AS_Vo l.+generate_CO2; 0.447, 0.727, tmp_cumulo+pH+pH_cumulo+PL+A N+AN_cumulo+interval_yield+RS+Cell_conc._interval+AS_Vol.+i nterval_O2; 0.438, 0.727, tmp_cumulo+pH+pH_cumulo+PL_cumulo +AN+AN_cumulo+interval_yield+RS+Feed_Vol+Cell_conc._interva l+RQ_integral+AS_Vol.; 0.455, 0.727, tmp_cumulo+pH+pH_cumul o+AN_cumulo+interval_yield+interval_Pdt.+OD+RS+Cell_conc._i nterval+AS_Vol.; 0.447, 0.727, tmp_cumulo+pH+pH_cumulo+AN_c umulo+interval_yield+RS+Cell_conc._interval+AS_Vol.+emissio n_CO2+consum_O2+generate_CO2; 0.438, 0.727, tmp_cumulo+pH+p H_cumulo+AN+AN_cumulo+interval_yield+interval_Pdt.+RS+Feed_ Vol+Cell_conc._interval+rab_integral+AS_Vol.; 0.447, 0.727, tmp+tmp_cumulo+pH+pH_cumulo+PL+AN_cumulo+interval_yield+RS +Cell_conc._interval+AS_Vol.+emission_CO2; 0.472, 0.727, pH +pH_cumulo+PL_cumulo+AN_cumulo+interval_yield+RS+Cell_conc. _interval+AS_Vol.; 0.446, 0.727, tmp_cumulo+pH+pH_cumulo+AN _cumulo+interval_yield+interval_Pdt.+RS+Cell_conc._interval +AS_Vol.+emission_CO2+generate_CO2; 0.455, 0.727, tmp_cumul o+pH+pH_cumulo+AN_cumulo+interval_yield+interval_Pdt.+RS+RS _cumulo+Cell_conc._interval+AS_Vol.; 0.437, 0.727, tmp+tmp_ cumulo+pH+pH_cumulo+AN_cumulo+interval_yield+RS+Feed_Vol+Ce ll_conc._interval+rab_integral+AS_Vol.+emission_CO2; 0.446, 0.727, tmp_cumulo+pH+pH_cumulo+AN_cumulo+interval_yield+RS +Feed_Vol+Cell_conc._interval+AS_Vol.+interval_O2+generate CO2; 0.455, 0.727, tmp_cumulo+pH+pH_cumulo+interval_yield+R S+Feed_Vol+Cell_conc._interval+AS_Vol.+emission_CO2+generat e_CO2; 0.437, 0.727, tmp_cumulo+pH+pH_cumulo+AN+AN_cumulo+i nterval_yield+RS+Feed_Vol+Cell_conc._interval+rab_integral+ AS_Vol.+emission_CO2; 0.455, 0.727, tmp_cumulo+pH+pH_cumulo +AN_cumulo+interval_yield+RS+Cell_conc._interval+RQ_integra l+AS_Vol.+generate_CO2; 0.437, 0.727, tmp_cumulo+pH+pH_cumu lo+AN_cumulo+interval_yield+interval_Pdt.+RS+Feed_Vol+Cell_ conc._interval+rab_integral+AS_Vol.+generate_CO2; 0.446, 0. 727, tmp+tmp_cumulo+pH+pH_cumulo+AN_cumulo+interval_yield+i nterval_Pdt.+RS+Cell_conc._interval+rab_integral+AS_Vol.; 0. 463, 0.727, tmp_cumulo+pH+pH_cumulo+PL_cumulo+interval_yiel d+RS+Feed_Vol+Cell_conc._interval+AS_Vol.; 0.437, 0.727, tm p_cumulo+pH+pH_cumulo+AN_cumulo+interval_yield+interval_Pdt. +RS+Feed_Vol+Cell_conc._interval+rab_integral+RQ_integral+A S_Vol.; 0.446, 0.727, tmp_cumulo+pH+pH_cumulo+AN+AN_cumulo+ interval_yield+RS+Cell_conc._interval+rab_integral+AS_Vol.+ consum_O2; 0.463, 0.727, tmp_cumulo+pH+pH_cumulo+interval_y ield+RS+Feed_Vol+Cell_conc._interval+AS_Vol.+emission_O2; 0. 446, 0.727, tmp_cumulo+pH+pH_cumulo+AN+interval_yield+inter val_Pdt.+RS+Feed_Vol+Cell_conc._interval+AS_Vol.+generate_C O2; 0.437, 0.727, tmp+tmp_cumulo+pH+pH_cumulo+PL_cumulo+AN_ cumulo+interval_yield+RS+Feed_Vol+Cell_conc._interval+rab_i ntegral+AS_Vol.; 0.446, 0.727, tmp_cumulo+pH+pH_cumulo+AN_c umulo+interval_yield+RS+RS_cumulo+Cell_conc._interval+rab_i ntegral+AS_Vol.+generate_CO2; 0.437, 0.727, tmp_cumulo+pH+p H_cumulo+PL+AN+interval_yield+RS+Cell_conc._interval+rab_in tegral+AS_Vol.+emission_CO2+interval_O2; 0.455, 0.727, tmp_ cumulo+pH+pH_cumulo+PL+AN_cumulo+interval_yield+RS+Cell_con c._interval+AS_Vol.+generate_CO2; 0.437, 0.727, tmp_cumulo+ pH+pH_cumulo+PL+AN+interval_yield+RS+Feed_Vol+Cell_conc._in terval+rab_integral+AS_Vol.+interval_O2; 0.446, 0.727, tmp_ cumulo+pH+pH_cumulo+AN_cumulo+interval_yield+RS+Cell_conc._ interval+rab_integral+RQ_integral+AS_Vol.+generate_CO2; 0.4 63, 0.727, tmp_cumulo+pH+pH_cumulo+interval_yield+RS+Feed_V ol+Cell_conc._interval+emission_CO2+generate_CO2; 0.463, 0. 727, tmp_cumulo+pH+pH_cumulo+AN_cumulo+interval_yield+RS+Fe ed_Vol+Cell_conc._interval+emission_O2; 0.479, 0.727, tmp_c umulo+pH+AN_cumulo+interval_yield+RS+Feed_Vol+Cell_conc._in terval; 0.463, 0.727, tmp_cumulo+pH+pH_cumulo+interval_yiel d+RS+Feed_Vol+Cell_conc._interval+rab_integral+generate_CO 2; 0.446, 0.727, tmp_cumulo+pH+pH_cumulo+AN_cumulo+interval _yield+RS+RS_cumulo+Cell_conc._interval+AS_Vol.+emission_CO 2+generate_CO2; 0.479, 0.727, tmp_cumulo+pH+interval_yield+ RS+Feed_Vol+Cell_conc._interval+AS_Vol.; 0.437, 0.727, tmp+ tmp_cumulo+pH+pH_cumulo+PL+AN_cumulo+interval_yield+RS+Feed _Vol+Cell_conc._interval+AS_Vol.+interval_O2; 0.454, 0.727, tmp_cumulo+pH+pH_cumulo+AN_cumulo+interval_yield+RS+Feed_V ol+Cell_conc._interval+rab_integral+RQ_integral; 0.437, 0.7 27, tmp_cumulo+pH+pH_cumulo+AN_cumulo+interval_yield+RS+Fee d_Vol+Cell_conc._interval+RQ_integral+AS_Vol.+emission_CO2+ generate_CO2; 0.446, 0.727, tmp_cumulo+pH+pH_cumulo+AN_cumu lo+interval_yield+interval_Pdt.+RS+Cell_conc._interval+rab_ integral+AS_Vol.+generate_CO2; 0.437, 0.727, tmp_cumulo+pH+ pH_cumulo+PL+AN+AN_cumulo+interval_yield+RS+Feed_Vol+Cell_c onc._interval+AS_Vol.+interval_O2; 0.437, 0.727, tmp_cumulo +pH+pH_cumulo+AN_cumulo+interval_yield+interval_Pdt.+RS+Fee d_Vol+Cell_conc._interval+rab_integral+AS_Vol.+emission_CO 2; 0.437, 0.727, tmp_cumulo+pH+pH_cumulo+AN_cumulo+interval _yield+RS+Feed_Vol+RS_cumulo+Cell_conc._interval+AS_Vol.+em ission_CO2+generate_CO2; 0.446, 0.727, tmp_cumulo+pH+pH_cum ulo+PL_cumulo+AN_cumulo+interval_yield+RS+Cell_conc._interv al+rab_integral+AS_Vol.+consum_O2; 0.446, 0.727, tmp_cumulo +pH+pH_cumulo+AN+AN_cumulo+interval_yield+OD+RS+Feed_Vol+Ce ll_conc._interval+AS_Vol.; 0.437, 0.727, tmp_cumulo+pH+pH_c umulo+AN_cumulo+interval_yield+RS+Feed_Vol+Cell_conc._inter val+AS_Vol.+emission_CO2+consum_O2+generate_CO2; 0.454, 0.7 27, tmp_cumulo+pH+pH_cumulo+PL_cumulo+AN_cumulo+interval_yi eld+RS+Cell_conc._interval+AS_Vol.+consum_O2; 0.446, 0.727, tmp_cumulo+pH+pH_cumulo+AN_cumulo+interval_yield+RS+Feed_V ol+RS_cumulo+Cell_conc._interval+RQ_integral+AS_Vol.; 0.437, 0.727, tmp_cumulo+pH+pH_cumulo+AN+AN_cumulo+interval_yield +interval_Pdt.+RS+Feed_Vol+Cell_conc._interval+AS_Vol.+emis sion_CO2; 0.446, 0.727, tmp+tmp_cumulo+pH+pH_cumulo+AN+AN_c umulo+interval_yield+RS+Cell_conc._interval+AS_Vol.+emissio n_CO2; 0.454, 0.727, tmp_cumulo+pH+pH_cumulo+AN_cumulo+inte rval_yield+interval_Pdt.+RS+Cell_conc._interval+AS_Vol.+con sum_O2; 0.454, 0.727, tmp_cumulo+pH+pH_cumulo+AN_cumulo+int erval_yield+RS+Cell_conc._interval+RQ_integral+AS_Vol.+cons um_O2; 0.479, 0.727, tmp_cumulo+pH+interval_yield+RS+Feed_V ol+Cell_conc._interval+interval_O2; 0.437, 0.727, tmp+tmp_c umulo+pH+pH_cumulo+AN+AN_cumulo+interval_yield+RS+Feed_Vol+ Cell_conc._interval+RQ_integral+AS_Vol.; 0.454, 0.727, tmp_ cumulo+pH+pH_cumulo+AN+interval_yield+RS+Cell_conc._interva l+rab_integral+AS_Vol.+emission_CO2; 0.454, 0.727, tmp_cumu lo+pH+pH_cumulo+PL_cumulo+AN_cumulo+interval_yield+OD+RS+Ce ll_conc._interval+AS_Vol.; 0.471, 0.727, tmp_cumulo+pH+pH_c umulo+interval_yield+RS+Cell_conc._interval+AS_Vol.+emissio n_CO2; 0.454, 0.727, tmp_cumulo+pH+pH_cumulo+AN_cumulo+inte rval_yield+interval_Pdt.+RS+Cell_conc._interval+RQ_integral +AS_Vol.; 0.454, 0.727, tmp_cumulo+pH+pH_cumulo+PL_cumulo+A N+interval_yield+RS+Cell_conc._interval+AS_Vol.+generate_CO 2; 0.454, 0.726, tmp_cumulo+pH+pH_cumulo+AN+interval_yield+ interval_Pdt.+RS+Cell_conc._interval+rab_integral+AS_Vol.; 0.436, 0.726, tmp_cumulo+pH+pH_cumulo+PL+AN+interval_yield+ RS+Cell_conc._interval+AS_Vol.+emission_CO2+interval_O2+gen erate_CO2; 0.436, 0.726, tmp_cumulo+pH+pH_cumulo+PL_cumulo+ AN_cumulo+interval_yield+RS+Feed_Vol+Cell_conc._interval+ra b_integral+AS_Vol.+emission_CO2; 0.445, 0.726, tmp+tmp_cumu lo+pH+pH_cumulo+AN_cumulo+interval_yield+RS+Cell_conc._inte rval+rab_integral+AS_Vol.+consum_O2; 0.436, 0.726, tmp_cumu lo+pH+pH_cumulo+PL_cumulo+AN_cumulo+interval_yield+RS+Feed_ Vol+Cell_conc._interval+RQ_integral+AS_Vol.+generate_CO2; 0. 445, 0.726, tmp_cumulo+pH+pH_cumulo+AN_cumulo+interval_yiel d+RS+Cell_conc._interval+rab_integral+AS_Vol.+consum_O2+gen erate_CO2; 0.436, 0.726, tmp+tmp_cumulo+pH+pH_cumulo+AN_cum ulo+interval_yield+RS+Feed_Vol+Cell_conc._interval+RQ-integ ral+AS_Vol.+generate_CO2; 0.436, 0.726, tmp_cumulo+pH+pH_cu mulo+PL+AN+AN_cumulo+interval_yield+RS+Cell_conc._interval+ RQ_integral+AS_Vol.+emission_CO2; 0.445, 0.726, tmp_cumulo+ pH+pH_cumulo+AN_cumulo+interval_yield+RS+Cell_conc._interva l+AS_Vol.+emission_O2+emission_CO2+generate_CO2 ; 0.436, 0.7 26, tmp+tmp_cumulo+pH+pH_cumulo+PL_cumulo+AN+AN_cumulo+inte rval_yield+RS+Feed_Vol+Cell_conc._interval+AS_Vol.;0.445, 0. 726, tmp_cumulo+pH+pH_cumulo+PL_cumulo+AN_cumulo+interval_y ield+RS+Feed_Vol+Cell_conc._interval+AS_Vol.+emission_O2; 0. 436, 0.726, tmp_cumulo+pH+pH_cumulo+AN+AN_cumulo+interval_y ield+RS+Feed_Vol+RS_cumulo+Cell_conc._interval+AS_Vol.+gene rate_CO2; 0.436, 0.726, tmp_cumulo+pH+pH_cumulo+AN_cumulo+i nterval_yield+RS+Cell_conc._interval+rab_integral+AS_Vol.+e mission_O2+emission_CO2+generate_CO2; 0.445, 0.726, tmp_cum ulo+pH+pH_cumulo+AN_cumulo+interval_yield+RS+RS_cumulo+Cell _conc._interval+rab_integral+AS_Vol.+emission_CO2; 0.445, 0. 726, tmp_cumulo+pH+pH_cumulo+AN_cumulo+interval_yield+RS+Ce ll-conc._interval+rab_integral+AS_Vol.+emission_CO2+consum_ O2; 0.445, 0.726, tmp_cumulo+pH+pH_cumulo+AN+interval_yield +RS+Feed_Vol+Cell_conc._interval+AS_Vol.+interval_O2+genera te_CO2; 0.445, 0.726, tmp+tmp_cumulo+pH+pH_cumulo+AN+interv al_yield+RS+Feed_Vol+Cell_conc._interval+AS_Vol.+emission_C O2; 0.445, 0.726, tmp+tmp_cumulo+pH+pH_cumulo+AN_cumulo+int erval_yield+OD+RS+Feed_Vol+Cell_conc._interval+AS_Vol.; 0.4 45, 0.726, tmp_cumulo+pH+pH_cumulo+PL+AN_cumulo+interval_yi eld+RS+Feed_Vol+Cell_conc._interval+RQ_integral+AS_Vol.; 0. 436, 0.726, tmp_cumulo+pH+pH_cumulo+AN_cumulo+interval_yiel d+RS+Feed_Vol+Cell_conc._interval+rab_integral+AS_Vol.+emis sion_O2+emission_CO2; 0.453, 0.726, tmp_cumulo+pH+pH_cumulo +AN_cumulo+interval_yield+RS+Feed_Vol+Cell_conc._interval+e mission_CO2+generate_CO2; 0.436, 0.726, tmp_cumulo+pH+pH_cu mulo+AN+AN_cumulo+interval_yield+RS+Feed_Vol+Cell_conc._int erval+AS_Vol.+consum_O2+generate_CO2; 0.426, 0.726, tmp_cum ulo+pH+pH_cumulo+AN_cumulo+interval_yield+RS+Feed_Vol+Cell_ conc._interval+rab_integral+RQ_integral+AS_Vol.+emission_O2 +emission_CO2; 0.445, 0.726, tmp_cumulo+pH+pH_cumulo+AN_cum ulo+interval_yield+RS+Feed_Vol+RS_cumulo+Cell_conc._interva l+AS_Vol.+emission_O2; 0.436, 0.726, tmp_cumulo+pH+pH_cumul o+AN_cumulo+interval_yield+RS+Feed_Vol+Cell_conc._interval+ rab_integral+AS_Vol.+emission_O2+generate_CO2; 0.445, 0.726, tmp_cumulo+pH+pH_cumulo+AN+AN_cumulo+interval_yield+RS+RS_ cumulo+Cell_conc._interval+rab_integral+AS_Vol.; 0.426, 0.7 26, tmp_cumulo+pH+pH_cumulo+PL_cumulo+AN_cumulo+interval_yi eld+RS+Feed_Vol+Cell_conc._interval+rab_integral+AS_Vol.+em ission_CO2+generate_CO2; 0.453, 0.726, tmp_cumulo+pH+pH_cum ulo+AN+interval_yield+OD+RS+Feed_Vol+Cell_conc._interval+AS _Vol.; 0.453, 0.726, tmp_cumulo+pH+pH_cumulo+AN_cumulo+inte rval_yield+interval_Pdt.+RS+Feed_Vol+Cell_conc._interval+ra b_integral; 0.470, 0.726, tmp_cumulo+pH+pH_cumulo+PL_cumulo +interval_yield+RS+Feed_Vol+Cell_conc._interval; 0.445, 0.7 26, tmp_cumulo+pH+pH_cumulo+AN_cumulo+interval_yield+RS+Fee d_Vol+Cell_conc._interval+AS_Vol.+emission_O2+consum_O2; 0. 445, 0.726, tmp_cumulo+pH+pH_cumulo+AN_cumulo+interval_yiel d+OD+RS+Feed_Vol+Cell_conc._interval+rab_integral+AS_Vol.; 0.453, 0.726, tmp_cumulo+pH+pH_cumulo+PL+AN_cumulo+interval _yield+RS+Cell_conc._interval+AS_Vol.+consum_O2; 0.436, 0.7 26, tmp_cumulo+pH+pH_cumulo+PL_cumulo+AN+AN_cumulo+interval _yield+RS+Feed_Vol+Cell_conc._interval+AS_Vol.+emission_CO 2; 0.436, 0.726, tmp+tmp_cumulo+pH+pH_cumulo+AN_cumulo+inte rval_yield+interval_Pdt.+RS+Feed_Vol+Cell_conc._interval+ra b_integral+AS_Vol.; 0.445, 0.726, tmp_cumulo+pH+pH_cumulo+A N_cumulo+interval_yield+interval_Pdt.+OD+RS+Feed_Vol+Cell_c onc._interval+AS_Vol.; 0.436, 0.726, tmp_cumulo+pH+pH_cumul o+PL_cumulo+AN_cumulo+interval_yield+interval_Pdt.+RS+Feed_ Vol+Cell_conc._interval+AS_Vol.+generate_CO2; 0.436, 0.726, 2.3tmp+tmp_cumulo+pH+pH_cumulo+PL+AN+interval_yield+RS+Feed_V ol+Cell_conc._interval+AS_Vol.+interval_O2; 0.444, 0.726, t mp_cumulo+pH+pH_cumulo+AN+interval_yield+RS+Feed_Vol+Cell_c onc._interval+AS_Vol.+consum_O2+generate_CO2; 0.462, 0.726, tmp_cumulo+pH+AN_cumulo+interval_yield+RS+Feed_Vol+Cell_co nc._interval+AS_Vol.+generate_CO2; 0.444, 0.726, tmp_cumulo +pH+pH_cumulo+AN+interval_yield+RS+Feed_Vol+Cell_conc._inte rval+AS_Vol.+emission_O2+generate_CO2; 0.436, 0.726, tmp_cu mulo+pH+pH_cumulo+PL_cumulo+AN+AN_cumulo+interval_yield+int erval_Pdt.+RS+Feed_Vol+Cell_conc._interval+AS_Vol.; 0.444, 0.726, tmp_cumulo+pH+pH_cumulo+AN_cumulo+interval_yield+int erval_Pdt.+RS+Cell_conc._interval+rab_integral+RQ_integral+ AS_Vol.; 0.462, 0.726, tmp_cumulo+pH+pH_cumulo+AN+interval_ yield+RS+Cell_conc._interval+Accum._Yield+AS_Vol.; 0.453, 0. 726, tmp_cumulo+pH+pH_cumulo+PL_cumulo+AN_cumulo+interval_y ield+RS+RS_cumulo+Cell_conc._interval+AS_Vol.; 0.453, 0.726, tmp_cumulo+pH+pH_cumulo+AN+interval_yield+OD+RS+Cell_conc. _interval+AS_Vol.+generate_CO2; 0.461, 0.726, tmp+pH+pH_cum ulo+PL_cumulo+AN_cumulo+interval_yield+RS+Cell_conc._interv al+AS_Vol.; 0.461, 0.726, tmp_cumulo+pH+pH_cumulo+interval_ yield+RS+Feed_Vol+Cell_conc._interval+rab_integral+emission _CO2; 0.444, 0.726, tmp+tmp_cumulo+pH+pH_cumulo+AN_cumulo+i nterval_yield+RS+RS_cumulo+Cell_conc._interval+rab_integral +AS_Vol.; 0.444, 0.726, tmp_cumulo+pH+pH_cumulo+AN+AN_cumul o+interval_yield+RS+Cell_conc._interval+AS_Vol.+consum_O2+g enerate_CO2; 0.435, 0.726, tmp+tmp_cumulo+pH+pH_cumulo+AN+A N_cumulo+interval_yield+RS+Feed_Vol+Cell_conc._interval+AS_ Vol.+emission_O2; 0.426, 0.726, tmp_cumulo+pH+pH_cumulo+PL_ cumulo+AN+AN_cumulo+interval_yield+RS+Feed_Vol+Cell_conc._i nterval+AS_Vol.+emission_CO2+generate_CO2; 0.444, 0.726, tm p_cumulo+pH+pH_cumulo+PL_cumulo+AN_cumulo+interval_yield+RS +RS_cumulo+Cell_conc._interval+rab_integral+AS_Vol.; 0.453, 0.726, tmp_cumulo+pH+pH_cumulo+AN+interval_yield+RS+Cell_c onc._interval+rab_integral+AS_Vol.+emission_O2; 0.461, 0.72 6, tmp_cumulo+pH+pH_cumulo+interval_yield+RS+Feed_Vol+Cell_ conc._interval+rab_integral+interval_O2; 0.461, 0.726, tmp_ cumulo+pH+AN_cumulo+interval_yield+RS+Feed_Vol+Cell_conc._i nterval+AS_Vol.+emission_CO2; 0.444, 0.726, tmp+tmp_cumulo+ pH+pH_cumulo+PL+AN+interval_yield+RS+Cell_conc._interval+AS _Vol.+interval_O2; 0.444, 0.726, tmp_cumulo+pH+pH_cumulo+PL _cumulo+AN+interval_yield+RS+Feed_Vol+Cell_conc._interval+R Q_integral+AS_Vol.; 0.453, 0.726, tmp+tmp_cumulo+pH+pH_cumu lo+AN_cumulo+interval_yield+RS+Cell_conc._interval+AS_Vol.+ emission_O2; 0.435, 0.726, tmp_cumulo+pH+pH_cumulo+PL+AN_cu mulo+interval_yield+RS+Cell_conc._interval+AS_Vol.+emission CO2+interval_O2+generate_CO2; 0.444, 0.726, tmp_cumulo+pH+ pH_cumulo+AN_cumulo+interval_yield+OD+RS+Feed_Vol+Cell_conc. _interval+AS_Vol.+emission_CO2; 0.444, 0.726, tmp_cumulo+pH +pH_cumulo+PL+AN_cumulo+interval_yield+RS+Cell_conc._interv al+rab_integral+AS_Vol.+consum_O2; 0.444, 0.726, tmp_cumulo +pH+pH_cumulo+AN+AN_cumulo+interval_yield+OD+RS+Cell_conc._ interval+AS_Vol.+generate_CO2; 0.444, 0.726, tmp+tmp_cumulo +pH+pH_cumulo+AN+interval_yield+RS+Feed_Vol+Cell_conc._inte rval+AS_Vol.+emission_O2; 0.435, 0.726, tmp+tmp_cumulo+pH+p H_cumulo+AN_cumulo+interval_yield+interval_Pdt.+RS+Feed_Vol +Cell_conc._interval+AS_Vol.+generate_CO2; 0.435, 0.726, tm p+tmp_cumulo+pH+pH_cumulo+PL_cumulo+AN_cumulo+interval_yiel d+RS+Feed_Vol+Cell_conc._interval+RQ_integral+AS_Vol.; 0.43 5, 0.726, tmp+tmp_cumulo+pH+pH_cumulo+AN+AN_cumulo+interval _yield+interval_Pdt.+RS+Feed_Vol+Cell_conc._interval+AS_Vo l.; 0.444, 0.726, tmp_cumulo+pH+pH_cumulo+AN_cumulo+interva l_yield+OD+RS+Cell_conc._interval+AS_Vol.+emission_CO2+gene rate_CO2; 0.444, 0.726, tmp_cumulo+pH+pH_cumulo+AN+interval _yield+interval_Pdt.+RS+Feed_Vol+Cell_conc._interval+rab_in tegral+AS_Vol.; 0.453, 0.726, tmp_cumulo+pH+pH_cumulo+AN_cu mulo+interval_yield+RS+Feed_Vol+Cell_conc._interval+rab_int egral+consum_O2; 0.435, 0.726, tmp_cumulo+pH+pH_cumulo+AN+A N_cumulo+interval_yield+RS+Feed_Vol+Cell_conc._interval+AS_ Vol.+emission_O2+generate_CO2; 0.453, 0.726, tmp_cumulo+pH+ pH_cumulo+AN+interval_yield+RS+Cell_conc._interval+AS_Vol.+ consum_O2+generate_CO2; 0.435, 0.726, tmp_cumulo+pH+pH_cumu lo+PL+AN_cumulo+interval_yield+RS+Cell_conc._interval+rab_i ntegral+AS_Vol.+emission_CO2+consum_O2; 0.453, 0.726, tmp_c umulo+pH+pH_cumulo+AN_cumulo+interval_yield+RS+RS_cumulo+Ce ll_conc._interval+AS_Vol.+consum_O2; 0.444, 0.726, tmp+tmp_ cumulo+pH+pH_cumulo+AN+interval_yield+RS+Feed_Vol+Cell_conc. _interval+rab_integral+AS_Vol.; 0.444, 0.726, tmp+tmp_cumul o+pH+pH_cumulo+PL+AN+AN_cumulo+interval_yield+RS+Cell_conc. _interval+AS_Vol.; 0.444, 0.726, tmp_cumulo+pH+pH_cumulo+AN +interval_yield+RS+Feed_Vol+RS_cumulo+Cell_conc._interval+A S_Vol.+generate_CO2; 0.469, 0.726, tmp_cumulo+pH+pH_cumulo+ interval_yield+interval_Pdt.+RS+Cell_conc._interval+AS_Vo l.; 0.453, 0.726, tmp_cumulo+pH+pH_cumulo+AN+interval_yield +RS+Cell_conc._interval+rab_integral+AS_Vol.+consum_O2; 0.4 35, 0.726, tmp_cumulo+pH+pH_cumulo+AN_cumulo+interval_yield +RS+Feed_Vol+Cell_conc._interval+rab_integral+AS_Vol.+consu m_O2+generate_CO2; 0.426, 0.726, tmp_cumulo+pH+pH_cumulo+AN +AN_cumulo+interval_yield+RS+Feed_Vol+Cell_conc._interval+r ab_integral+AS_Vol.+emission_CO2+generate_CO2; 0.453, 0.726, tmp_cumulo+pH+pH_cumulo+AN+AN_cumulo+interval_yield+RS+Cel l_conc._interval+Accum._Yield+AS_Vol.; 0.435, 0.726, tmp+tm p_cumulo+pH+pH_cumulo+AN_cumulo+interval_yield+interval_Pdt. +RS+Feed_Vol+Cell_conc._interval+AS_Vol.+emission_CO2; 0.45 3, 0.726, tmp_cumulo+pH+pH_cumulo+PL+AN+interval_yield+RS+C ell_conc._interval+AS_Vol.+emission_CO2; 0.444, 0.726, tmp+ tmp_cumulo+pH+pH_cumulo+PL+AN_cumulo+interval_yield+RS+Feed _Vol+Cell_conc._interval+AS_Vol.; 0.453, 0.726, tmp_cumulo+ pH+pH_cumulo+AN+interval_yield+RS+Cell_conc._interval+RQ_in tegral+AS_Vol.+generate_CO2; 0.444, 0.726, tmp_cumulo+pH+pH _cumulo+AN_cumulo+interval_yield+RS+Cell_conc._interval+rab integral+RQ_integral+AS_Vol.+consum_O2; 0.444, 0.726, tmp_ cumulo+pH+pH_cumulo+PL_cumulo+AN_cumulo+interval_yield+OD+R S+Feed_Vol+Cell_conc._interval+AS_Vol.; 0.461, 0.726, tmp_c umulo+pH+pH_cumulo+interval_yield+RS+Feed_Vol+RS_cumulo+Cel l_conc._interval+AS_Vol.; 0.435, 0.726, tmp_cumulo+pH+pH_cu mulo+PL_cumulo+AN+AN_cumulo+interval_yield+RS+Cell_conc._in terval+rab_integral+AS_Vol.+generate_CO2; 0.425, 0.726, tmp _cumulo+pH+pH_cumulo+PL+AN_cumulo+interval_yield+RS+Cell_co nc._interval+rab_integral+AS_Vol.+emission_CO2+interval_O2+ generate_CO2; 0.444, 0.726, tmp_cumulo+pH+pH_cumulo+AN+AN_c umulo+interval_yield+interval_Pdt.+RS+Cell_conc._interval+A S_Vol.+generate_CO2; 0.444, 0.726, tmp_cumulo+pH+pH_cumulo+ AN+AN_cumulo+interval_yield+RS+Cell_conc._interval+rab_inte gral+AS_Vol.+emission_O2; 0.435, 0.725, tmp_cumulo+pH+pH_cu mulo+AN+AN_cumulo+interval_yield+RS+Cell_conc._interval+rab _integral+AS_Vol.+emission_CO2+generate_CO2; 0.444, 0.725, tmp_cumulo+pH+pH_cumulo+PL+AN+AN_cumulo+interval_yield+RS+C ell_conc._interval+RQ_integral+AS_Vol.; 0.444, 0.725, tmp_c umulo+pH+pH cumulo+AN cumulo+interval yield+RS+Feed Vol+RS cumulo+Cell_conc._interval+AS_Vol.+consum_02; 0.461, 0.725, tmp_cumulo+pH+pH_cumulo+interval_yield+interval_Pdt.+RS+Fe ed_Vol+Cell_conc._interval+rab_integral; 0.469, 0.725, tmp+ tmp_cumulo+pH+pH_cumulo+interval_yield+RS+Cell_conc._interv al+AS_Vol.; 0.444, 0.725, tmp_cumulo+pH+pH_cumulo+AN+AN_cum ulo+interval_yield+RS+RS_cumulo+Cell_conc._interval+AS_Vol. +generate_CO2; 0.461, 0.725, tmp_cumulo+pH+pH_cumulo+PL_cum ulo+AN_cumulo+interval_yield+RS+Feed_Vol+Cell_conc._interva 1; 0.452, 0.725, tmp+tmp_cumulo+pH+pH_cumulo+AN+interval_yi eld+RS+Cell_conc._interval+AS_Vol.+emission_CO2; 0.425, 0.7 25, tmp_cumulo+pH+pH_cumulo+PL_cumulo+AN+AN_cumulo+interval _yield+RS+Feed_Vol+Cell_conc._interval+AS_Vol.+interval_O2+ generate_CO2; 0.461, 0.725, tmp_cumulo+pH+pH_cumulo+interva l_yield+RS+Feed_Vol+Cell_conc._interval+consum_O2+generate_ CO2; 0.443, 0.725, tmp_cumulo+pH+pH_cumulo+AN+interval_yiel d+RS+Feed_Vol+Cell_conc._interval+rab_integral+AS_Vol.+emis sion_CO2; 0.434, 0.725, tmp_cumulo+pH+pH_cumulo+AN_cumulo+i nterval_yield+RS+Feed_Vol+RS_cumulo+Cell_conc._interval+rab _integral+AS_Vol.+generate_CO2; 0.452, 0.725, tmp_cumulo+pH +pH_cumulo+AN_cumulo+interval_yield+RS+Cell_conc._interval+ rab_integral+Accum._Yield+AS_Vol.; 0.461, 0.725, tmp_cumulo +pH+pH_cumulo+interval_yield+RS+Feed_Vol+Cell_conc._interva l+emission_CO2+consum_O2; 0.443, 0.725, tmp+tmp_cumulo+pH+p H_cumulo+AN+interval_yield+RS+Feed_Vol+Cell_conc._interval+ RQ_integral+AS_Vol.; 0.434, 0.725, tmp_cumulo+pH+pH_cumulo+ AN+AN_cumulo+interval_yield+RS+Feed_Vol+Cell_conc._interval +rab_integral+AS_Vol.+emission_O2; 0.443, 0.725, tmp_cumulo +pH+pH_cumulo+PL+AN+AN_cumulo+interval_yield+RS+Feed_Vol+Ce ll_conc._interval+AS_Vol.; 0.452, 0.725, tmp_cumulo+pH+pH_c umulo+AN+interval_yield+interval_Pdt.+RS+Cell_conc._interva l+AS_Vol.+generate_CO2; 0.443, 0.725, tmp_cumulo+pH+pH_cumu lo+PL+AN_cumulo+interval_yield+interval_Pdt.+RS+Cell_conc._ interval+AS_Vol.+emission_CO2; 0.452, 0.725, tmp_cumulo+pH+ pH_cumulo+AN_cumulo+interval_yield+interval_Pdt.+RS+Feed_Vo l+Cell_conc._interval+emission_CO2; 0.460, 0.725, tmp_cumul o+pH+pH_cumulo+AN+interval_yield+RS+Feed_Vol+Cell_conc._int erval+generate_CO2; 0.452, 0.725, tmp_cumulo+pH+pH_cumulo+A N+interval_yield+RS+RS_cumulo+Cell_conc._interval+AS_Vol.+g enerate_CO2; 0.443, 0.725, tmp+tmp_cumulo+pH+pH_cumulo+AN+A N_cumulo+interval_yield+RS+Cell_conc._interval+RQ_integral+ AS_Vol.; 0.443, 0.725, tmp_cumulo+pH+pH_cumulo+PL_cumulo+AN +interval_yield+RS+Feed_Vol+Cell_conc._interval+rab_integra l+AS_Vol.; 0.434, 0.725, tmp_cumulo+pH+pH_cumulo+PL+AN+AN_c umulo+interval_yield+RS+Cell_conc._interval+rab_integral+AS Vol.+interval O2; 0.434, 0.725, tmp+tmp_cumulo+pH+pH_cumul o+AN_cumulo+interval_yield+RS+Cell_conc._interval+rab_integ ral+AS_Vol.+emission_CO2+generate_CO2; 0.469, 0.725, tmp_cu mulo+pH+interval_yield+RS+Feed_Vol+Cell_conc._interval+inte rval_O2+generate_CO2; 0.476, 0.725, tmp_cumulo+pH+interval_ yield+RS+Feed_Vol+Cell_conc._interval+consum_O2; 0.434, 0.7 25, tmp+tmp_cumulo+pH+pH_cumulo+AN_cumulo+interval_yield+RS +Feed_Vol+Cell_conc._interval+rab_integral+AS_Vol.+emission _O2; 0.434, 0.725, tmp_cumulo+pH+pH_cumulo+PL+AN+AN_cumulo+ interval_yield+RS+Cell_conc._interval+AS_Vol.+emission_CO2+ generate_CO2; 0.443, 0.725, tmp_cumulo+pH+pH_cumulo+AN+inte rval_yield+RS+Feed_Vol+Cell_conc._interval+rab_integral+AS_ Vol.+emission_O2; 0.434, 0.725, tmp_cumulo+pH+pH_cumulo+PL_ cumulo+AN+AN_cumulo+interval_yield+RS+Cell_conc._interval+A S_Vol.+emission_CO2+generate_CO2; 0.434, 0.725, tmp_cumulo+ pH+pH_cumulo+AN+AN_cumulo+interval_yield+interval_Pdt.+RS+F eed_Vol+Cell_conc._interval+RQ_integral+AS_Vol.; 0.443, 0.7 25, tmp_cumulo+pH+pH_cumulo+AN+interval_yield+interval_Pdt. +RS+Feed_Vol+Cell_conc._interval+AS_Vol.+emission_CO2; 0.42 5, 0.725, tmp_cumulo+pH+pH_cumulo+PL_cumulo+AN+AN_cumulo+in terval_yield+RS+Feed_Vol+Cell_conc._interval+rab_integral+A S_Vol.+generate_CO2; 0.468, 0.725, tmp+pH+pH_cumulo+AN+inte rval_yield+RS+Cell_conc._interval+AS_Vol.; 0.452, 0.725, tm p_cumulo+pH+pH_cumulo+AN_cumulo+interval_yield+RS+Cell_conc. _interval+RQ_integral+AS_Vol.+emission_CO2; 0.443, 0.725, t mp_cumulo+pH+pH_cumulo+PL+AN_cumulo+interval_yield+interval _Pdt.+RS+Feed_Vol+Cell_conc._interval+AS_Vol.; 0.443, 0.725, tmp_cumulo+pH+pH_cumulo+AN_cumulo+interval_yield+RS+Cell_c onc._interval+rab_integral+AS_Vol.+emission_O2+generate_CO 2; 0.476, 0.725, tmp_cumulo+pH+interval_yield+RS+Feed_Vol+C ell_conc._interval+emission_CO2; 0.460, 0.725, tmp_cumulo+p H+pH_cumulo+AN+interval_yield+RS+Feed_Vol+Cell_conc._interv al+rab_integral; 0.443, 0.725, tmp_cumulo+pH+pH_cumulo+AN+A N_cumulo+interval_yield+OD+RS+Cell_conc._interval+rab-integ ral+AS_Vol.; 0.443, 0.725, tmp_cumulo+pH+pH_cumulo+PL+AN+in terval_yield+RS+Cell_conc._interval+AS_Vol.+interval_O2+gen erate_CO2; 0.460, 0.725, tmp_cumulo+pH+pH_cumulo+interval_y ield+RS+Feed_Vol+Cell_conc._interval+AS_Vol.+interval_O2; 0. 468, 0.725, tmp_cumulo+pH+pH_cumulo+interval_yield+RS+Cell_ conc._interval+AS_Vol.+generate_CO2; 0.452, 0.725, tmp_cumu lo+pH+pH_cumulo+AN_cumulo+interval_yield+RS+Feed_Vol+Cell_c onc._interval+rab_integral+emission_CO2; 0.443, 0.725, tmp_ cumulo+pH+pH_cumulo+AN_cumulo+interval_yield+OD+RS+Cell_con c._interval+rab_integral+AS_Vol.+generate_CO2; 0.443, 0.725, tmp_cumulo+pH+pH_cumulo+AN+AN_cumulo+interval_yield+RS+Cel l_conc._interval+RQ_integral+AS_Vol.+generate_CO2; 0.460, 0. 725, tmp_cumulo+pH+AN+AN_cumulo+interval_yield+RS+Feed_Vol+ Cell_conc._interval+AS_Vol.; 0.443, 0.725, tmp_cumulo+pH+pH _cumulo+PL_cumulo+AN_cumulo+interval_yield+OD+RS+Cell_conc. _interval+rab_integral+AS_Vol.; 0.434, 0.725, tmp_cumulo+pH +pH_cumulo+AN_cumulo+interval_yield+RS+Feed_Vol+Cell_conc._ interval+rab_integral+RQ_integral+AS_Vol.+emission_O2; 0.42 5, 0.725, tmp_cumulo+pH+pH_cumulo+AN+AN_cumulo+interval_yie ld+RS+Feed_Vol+Cell_conc._interval+rab_integral+RQ_integral +AS_Vol.+generate_CO2; 0.460, 0.725, tmp_cumulo+pH+pH_cumul o+interval_yield+interval_Pdt.+RS+Feed_Vol+Cell_conc._inter val+emission_CO2; 0.451, 0.725, tmp_cumulo+pH+pH_cumulo+AN+ interval_yield+RS+RS_cumulo+Cell_conc._interval+rab_integra l+AS_Vol.; 0.434, 0.725, tmp_cumulo+pH+pH_cumulo+AN_cumulo+ interval_yield+RS+Feed_Vol+Cell_conc._interval+rab_integral +RQ_integral+AS_Vol.+emission_CO2; 0.443, 0.725, tmp+tmp_cu mulo+pH+pH_cumulo+PL_cumulo+AN+AN_cumulo+interval_yield+RS+ Cell_conc._interval+AS_Vol.; 0.424, 0.725, tmp_cumulo+pH+pH _cumulo+AN_cumulo+interval_yield+RS+Feed_Vol+Cell_conc._int erval+rab_integral+AS_Vol.+emission_O2+emission_CO2+generat e_CO2; 0.434, 0.725, tmp+tmp_cumulo+pH+pH_cumulo+PL_cumulo+ AN_cumulo+interval_yield+RS+Feed_Vol+Cell_conc._interval+AS _Vol.+emission_CO2; 0.451, 0.725, tmp_cumulo+pH+AN_cumulo+i nterval_yield+RS+Feed_Vol+Cell_conc._interval+AS_Vol.+emiss ion_CO2+generate_CO2; 0.443, 0.725, tmp_cumulo+pH+pH_cumulo +AN_cumulo+interval_yield+RS+Feed_Vol+Cell_conc._interval+r ab_integral+emission_CO2+generate_CO2; 0.434, 0.725, tmp_cu mulo+pH+pH_cumulo+AN_cumulo+interval_yield+RS+Cell_conc._in terval+RQ_integral+AS_Vol.+emission_O2+emission_CO2+generat e_CO2; 0.434, 0.725, tmp_cumulo+pH+pH_cumulo+AN+AN_cumulo+i nterval_yield+RS+Cell_conc._interval+RQ_integral+AS_Vol.+em ission_O2+emission_CO2; 0.443, 0.725, tmp_cumulo+pH+pH_cumu lo+PL+AN_cumulo+interval_yield+RS+RS_cumulo+Cell_conc._inte rval+rab_integral+AS_Vol.; 0.443, 0.725, tmp_cumulo+pH+pH_c umulo+PL_cumulo+AN+AN_cumulo+interval_yield+RS+Cell_conc._i nterval+AS_Vol.+emission_CO2; 0.460, 0.725, tmp_cumulo+pH+p H_cumulo+interval_yield+RS+Feed_Vol+Cell_conc._interval+RQ_ integral+consum_O2; 0.451, 0.725, tmp_cumulo+pH+pH_cumulo+i nterval_yield+RS+Feed_Vol+Cell_conc._interval+rab_integral+ emission_CO2+generate_CO2; 0.451, 0.725, tmp_cumulo+pH+pH_c umulo+AN_cumulo+interval_yield+OD+RS+RS_cumulo+Cell_conc._i nterval+AS_Vol.; 0.434, 0.725, tmp_cumulo+pH+pH_cumulo+PL+A N_cumulo+interval_yield+RS+Feed_Vol+Cell_conc._interval+AS-Vol.+emission_CO2+interval_O2; 0.460, 0.725, tmp_cumulo+pH+ pH_cumulo+interval_yield+RS+Feed_Vol+Cell_conc._interval+ra b_integral+emission_O2; 0.433, 0.725, tmp_cumulo+pH+pH_cumu lo+PL_cumulo+AN_cumulo+interval_yield+interval_Pdt.+RS+Feed _Vol+Cell_conc._interval+AS_Vol.+emission_CO2; 0.433, 0.725, mp_cumulo+pH+pH_cumulo+PL_cumulo+AN_cumulo+interval_yield +interval_Pdt.+RS+Feed_Vol+Cell_conc._interval+RQ_integral+ AS_Vol.; 0.433, 0.725, tmp_cumulo+pH+pH_cumulo+AN+AN_cumulo +interval_yield+RS+Feed_Vol+Cell_conc._interval+AS_Vol.+emi ssion_O2+emission_CO2; 0.451, 0.725, tmp_cumulo+pH+pH_cumul o+AN_cumulo+interval_yield+RS+Cell_conc._interval+AS_Vol.+e mission_O2+generate_CO2; 0.442, 0.725, tmp_cumulo+pH+pH_cum ulo+PL+AN+interval_yield+RS+Cell_conc._interval+rab_integra l+AS_Vol.+emission_CO2; 0.460, 0.725, tmp_cumulo+pH+pH_cumu lo+interval_yield+RS+Feed_Vol+Cell_conc._interval+emission_ CO2+interval_O2; 0.442, 0.725, tmp_cumulo+pH+pH_cumulo+AN_c umulo+interval_yield+interval_Pdt.+RS+Cell_conc._interval+r ab_integral+AS_Vol.+consum_O2; 0.451, 0.725, tmp_cumulo+pH+ pH_cumulo+PL+AN_cumulo+interval_yield+RS+RS_cumulo+Cell_con c._interval+AS_Vol.; 0.451, 0.725, tmp_cumulo+pH+pH_cumulo+ AN_cumulo+interval_yield+RS+RS_cumulo+Cell_conc._interval+R Q_integral+AS_Vol.; 0.442, 0.725, tmp_cumulo+pH+pH_cumulo+P L_cumulo+AN+AN_cumulo+interval_yield+RS+Cell_conc._interval +RQ_integral+AS_Vol.; 0.451, 0.725, tmp_cumulo+pH+pH_cumulo +AN+interval_yield+OD+RS+Cell_conc._interval+rab_integral+A S_Vol.; 0.433, 0.725, tmp_cumulo+pH+pH_cumulo+AN_cumulo+int erval_yield+RS+Feed_Vol+Cell_conc._interval+rab_integral+RQ _integral+AS_Vol.+consum_O2; 0.433, 0.725, tmp+tmp_cumulo+p H+pH_cumulo+AN_cumulo+interval_yield+RS+Feed_Vol+RS_cumulo+ Cell_conc._interval+AS_Vol.+generate_CO2; 0.442, 0.725, tmp +tmp_cumulo+pH+pH_cumulo+AN+AN_cumulo+interval_yield+interv al_Pdt.+RS+Cell_conc._interval+AS_Vol.; 0.424, 0.725, tmp_c umulo+pH+pH_cumulo+PL+AN+AN_cumulo+interval_yield+RS+Cell_c one._interval+rab_integral+AS_Vol.+emission_CO2+generate_CO 2; 0.442, 0.725, tmp+tmp_cumulo+pH+pH_cumulo+AN+AN_cumulo+i nterval_yield+RS+Cell_conc._interval+AS_Vol.+consum_O2; 0.4 24, 0.725, tmp+tmp_cumulo+pH+pH_cumulo+AN_cumulo+interval_y ield+RS+Feed_Vol+Cell_conc._interval+rab_integral+AS_Vol.+e mission_CO2+generate_CO2; 0.433, 0.725, tmp+tmp_cumulo+pH+p H_cumulo+PL_cumulo+AN_cumulo+interval_yield+interval_Pdt.+R S+Feed_Vol+Cell_conc._interval+AS_Vol.; 0.442, 0.725, tmp_c umulo+pH+pH_cumulo+PL+AN+interval_yield+RS+Cell_conc._inter val+RQ_integral+AS_Vol.+emission_CO2; 0.451, 0.725, tmp_cum ulo+pH+pH_cumulo+interval_yield+RS+Feed_Vol+Cell_conc._inte rval+rab_integral+RQ_integral+AS_Vol.; 0.433, 0.725, tmp+tm p_cumulo+pH+pH_cumulo+AN_cumulo+interval_yield+RS+Feed_Vol+ Cell_conc._interval+AS_Vol.+consum_O2+generate_CO2; 0.459, 0.725, tmp_cumulo+pH+pH_cumulo+AN+interval_yield+RS+Feed_Vo l+Cell_conc._interval+consum_O2; 0.451, 0.725, tmp+tmp_cumu lo+pH+pH_cumulo+AN+interval_yield+RS+Cell_conc._interval+RQ _integral+AS_Vol.; 0.442, 0.725, tmp_cumulo+pH+pH_cumulo+AN +interval_yield+RS+Feed_Vol+Cell_conc._interval+RQ_integral +AS_Vol.+emission_O2; 0.451, 0.725, tmp_cumulo+pH+pH_cumulo +PL+AN+interval_yield+RS+Cell_conc._interval+RQ_integral+AS _Vol.; 0.442, 0.725, tmp_cumulo+pH+pH_cumulo+AN_cumulo+inte rval_yield+interval_Pdt.+RS+RS_cumulo+Cell_conc._interval+r ab_integral+AS_Vol.; 0.442, 0.725, tmp+tmp_cumulo+pH+pH_cum ulo+AN+interval_yield+interval_Pdt.+RS+Feed_Vol+Cell_conc._ interval+AS_Vol.; 0.475, 0.725, tmp+pH+pH_cumulo+interval_y ield+RS+Cell_conc._interval+AS_Vol.; 0.433, 0.725, tmp+tmp_ cumulo+pH+pH_cumulo+AN_cumulo+interval_yield+RS+Feed_Vol+Ce ll_conc._interval+ASVol.+emission_O2+emission_CO2; 0.451, 0.725, tmp+tmp_cumulo+pH+pH_cumulo+AN+interval_yield+OD+RS+ Cell_conc._interval+AS_Vol.; 0.424, 0.725, tmp_cumulo+pH+pH _cumulo+AN_cumulo+interval_yield+RS+Feed_Vol+Cell_conc._int erval+RQ_integral+AS_Vol.+emission_O2+emission_CO2+generate _CO2; 0.442, 0.725, tmp+tmp_cumulo+pH+pH_cumulo+AN+AN_cumul o+interval_yield+OD+RS+Cell_conc._interval+AS_Vol.; 0.433, 0.724, tmp_cumulo+pH+pH_cumulo+AN+interval_yield+RS+Feed_Vo l+Cell_conc._interval+rab_integral+RQ_integral+AS_Vol.+gene rate_CO2; 0.459, 0.724, tmp+tmp_cumulo+pH+AN_cumulo+interva l_yield+RS+Feed_Vol+Cell_conc._interval+AS_Vol.; 0.433, 0.7 24, tmp_cumulo+pH+pH_cumulo+PL+AN_cumulo+interval_yield+RS+ RS_cumulo+Cell_conc._interval+rab_integral+AS_Vol.+emission _CO2; 0.442, 0.724, tmp_cumulo+pH+pH_cumulo+PL+interval_yie ld+RS+Feed_Vol+Cell_conc._interval+rab_integral+AS_Vol.+int erval_O2; 0.475, 0.724, pH+pH_cumulo+AN_cumulo+interval_yie ld+RS+Cell_conc._interval+AS_Vol.; 0.451, 0.724, tmp_cumulo +pH+pH_cumulo+interval_yield+RS+Feed_Vol+Cell_conc._interva l+rab_integral+AS_Vol.+generate_CO2; 0.433, 0.724, tmp_cumu lo+pH+pH_cumulo+PL_cumulo+AN+AN_cumulo+interval_yield+RS+Ce ll_conc._interval+rab_integral+RQ_integral+AS_Vol.; 0.459, 0.724, tmp_cumulo+pH+pH_cumulo+AN+interval_yield+interval_P dt.+RS+Feed_Vol+Cell_conc._interval; 0.442, 0.724, tmp_cumu lo+pH+pH_cumulo+PL+AN+AN_cumulo+interval_yield+interval_Pdt. +RS+Cell_conc._interval+AS_Vol.; 0.450, 0.724, tmp_cumulo+p H+pH_cumulo+AN+AN_cumulo+interval_yield+RS+Feed_Vol+Cell_co nc._interval+generate_CO2; 0.442, 0.724, tmp_cumulo+pH+pH_c umulo+PL+AN_cumulo+interval_yield+RS+Cell_conc._interval+AS Vol.+emission_CO2+consum_O2; 0.423, 0.724, tmp_cumulo+pH+p H_cumulo+PL+AN+AN_cumulo+interval_yield+RS+Cell_conc._inter val+rab_integral+AS_Vol.+emission_CO2+interval_O2; 0.450, 0. 724, tmp_cumulo+pH+pH_cumulo+AN_cumulo+interval_yield+inter val_Pdt.+RS+Feed_Vol+Cell_conc._interval+generate_CO2; 0.44 2, 0.724, tmp_cumulo+pH+pH_cumulo+PL+AN+AN_cumulo+interval_ yield+RS+Cell_conc._interval+AS_Vol.+generate_CO2; 0.442, 0. 724, tmp_cumulo+pH+pH_cumulo+AN_cumulo+interval_yield+RS+RS _cumulo+Cell_conc._interval+rab_integral+RQ_integral+AS_Vo l.; 0.450, 0.724, tmp+tmp_cumulo+pH+pH_cumulo+AN_cumulo+int erval_yield+RS+Cell_conc._interval+Accum._Yield+AS_Vol.; 0. 433, 0.724, tmp_cumulo+pH+pH_cumulo+AN+AN_cumulo+interval_y ield+RS+Feed_Vol+Cell_conc._interval+RQ_integral+AS_Vol.+em ission_O2; 0.450, 0.724, tmp_cumulo+pH+pH_cumulo+AN_cumulo+ interval_yield+OD+RS+Cell_conc._interval+RQ_integral+AS_Vo 1.; 0.459, 0.724, tmp+tmp_cumulo+pH+pH_cumulo+interval_yiel d+RS+Feed_Vol+Cell_conc._interval+rab_integral; 0.442, 0.72 4, tmp_cumulo+pH+pH_cumulo+AN+AN_cumulo+interval_yield+inte rval_Pdt.+RS+Cell_conc._interval+AS_Vol.+emission_CO2; 0.43 3, 0.724, tmp_cumulo+pH+pH_cumulo+PL+AN+AN_cumulo+interval_ yield+RS+Cell_conc._interval+AS_Vol.+emission_CO2+interval_ 02; 0.433, 0.724, tmp_cumulo+pH+pH_cumulo+AN+interval_yield +RS+Feed_Vol+Cell_conc._interval+rab_integral+AS_Vol.+emiss ion_CO2+generate_CO2; 0.459, 0.724, tmp+tmp_cumulo+pH+pH_cu mulo+interval_yield+RS+Feed_Vol+Cell_conc._interval+consum_ 02; 0.433, 0.724, tmp_cumulo+pH+pH_cumulo+PL+AN+AN_cumulo+i nterval_yield+RS+Cell_conc._interval+rab_integral+AS_Vol.+g enerate_CO2; 0.450, 0.724, tmp_cumulo+pH+pH_cumulo+AN+AN_cu mulo+interval_yield+RS+Feed_Vol+Cell_conc._interval+rab_int egral; 0.459, 0.724, pH+pH_cumulo+PL_cumulo+AN_cumulo+inter val_yield+RS+Cell_conc._interval+rab_integral+AS_Vol.; 0.43 3, 0.724, tmp+tmp_cumulo+pH+pH_cumulo+AN_cumulo+interval_yi eld+RS+Feed_Vol+Cell_conc._interval+AS_Vol.+emission_O2+gen erate_CO2; 0.442, 0.724, tmp+tmp_cumulo+pH+pH_cumulo+AN+AN_ cumulo+interval_yield+RS+RS_cumulo+Cell_conc._interval+AS_V ol.; 0.433, 0.724, tmp_cumulo+pH+pH_cumulo+PL_cumulo+AN_cum ulo+interval_yield+RS+Feed_Vol+Cell_conc._interval+rab_inte gral+AS_Vol.+consum_O2; 0.441, 0.724, tmp+tmp_cumulo+pH+pH_ cumulo+AN_cumulo+interval_yield+OD+RS+Cell_conc._interval+r ab_integral+AS_Vol.; 0.450, 0.724, tmp+tmp_cumulo+pH+pH_cum ulo+AN+interval_yield+interval_Pdt.+RS+Cell_conc._interval+ AS_Vol.; 0.423, 0.724, tmp+tmp_cumulo+pH+pH_cumulo+AN+AN_cu mulo+interval_yield+RS+Feed_Vol+Cell_conc._interval+rab_int egral+AS_Vol.+generate_CO2; 0.459, 0.724, tmp_cumulo+pH+pH_ cumulo+PL_cumulo+interval_yield+RS+Feed_Vol+Cell_conc._inte rval+interval_O2; 0.483, 0.724, pH+pH_cumulo+interval_yield +RS+Cell_conc._interval+AS_Vol.; 0.441, 0.724, tmp_cumulo+p H+pH_cumulo+AN+interval_yield+interval_Pdt.+RS+Feed_Vol+Cel l_conc._interval+RQ_integral+AS_Vol.; 0.432, 0.724, tmp_cum ulo+pH+pH_cumulo+PL+AN+interval_yield+RS+Feed_Vol+Cell_conc. _interval+AS_Vol.+emission_CO2+interval_O2; 0.441, 0.724, t mp+tmp_cumulo+pH+pH_cumulo+PL_cumulo+AN+interval_yield+RS+F eed_Vol+Cell_conc._interval+AS_Vol.; 0.432, 0.724, tmp_cumu lo+pH+pH_cumulo+PL+AN+AN_cumulo+interval_yield+RS+Cell_conc. _interval+rab_integral+RQ_integral+AS_Vol.; 0.459, 0.724, t mp_cumulo+pH+pH_cumulo+AN+interval_yield+RS+Feed_Vol+Cell_c onc._interval+emission_CO2; 0.441, 0.724, tmp_cumulo+pH+pH_ cumulo+AN_cumulo+interval_yield+RS+Feed_Vol+Cell_conc._inte rval+rab_integral+interval_O2+generate_CO2; 0.441, 0.724, t mp_cumulo+pH+pH_cumulo+PL+AN_cumulo+interval_yield+RS+Feed_ Vol+Cell_conc._interval+AS_Vol.+consum_O2; 0.441, 0.724, tm p_cumulo+pH+pH_cumulo+PL+AN_cumulo+interval_yield+RS+Feed_V ol+Cell_conc._interval+AS_Vol.+emission_CO2; 0.423, 0.724, tmp_cumulo+pH+pH_cumulo+AN_cumulo+interval_yield+RS+Feed_Vo l+Cell_conc._interval+rab_integral+RQ_integral+AS_Vol.+emis sion_CO2+generate_CO2; 0.432, 0.724, tmp_cumulo+pH+pH_cumul o+AN+AN_cumulo+interval_yield+RS+Feed_Vol+Cell_conc._interv al+rab_integral+AS_Vol.+consum_O2; 0.450, 0.724, tmp_cumulo +pH+pH_cumulo+PL_cumulo+interval_yield+RS+Feed_Vol+Cell_con c._interval+interval_O2+generate_CO2; 0.432, 0.724, tmp_cum ulo+pH+pH_cumulo+PL+AN_cumulo+interval_yield+RS+Feed_Vol+Ce ll_conc._interval+rab_integral+RQ_integral+AS_Vol.; 0.441, 0.724, tmp_cumulo+pH+pH_cumulo+PL+AN+interval_yield+RS+Cell _conc._interval+Accum._Yield+AS_Vol.+interval_O2; 0.450, 0. 724, tmp_cumulo+pH+pH_cumulo+AN_cumulo+interval_yield+RS+Ce ll_conc._interval+Accum._Yield+AS_Vol.+generate_CO2; 0.441, 0.724, tmp_cumulo+pH+pH_cumulo+PL+interval_yield+RS+Cell_c onc._interval+rab_integral+AS_Vol.+emission_CO2+interval_O 2; 0.441, 0.724, tmp_cumulo+pH+pH_cumulo+PL_cumulo+AN+inter val_yield+RS+Feed_Vol+Cell_conc._interval+AS_Vol.+interval_ O2; 0.441, 0.724, tmp_cumulo+pH+pH_cumulo+AN+interval_yield +RS+Feed_Vol+Cell_conc._interval+AS_Vol.+emission_O2+emissi on_CO2; 0.423, 0.724, tmp+tmp_cumulo+pH+pH_cumulo+AN+AN_cum ulo+interval_yield+RS+Feed_Vol+Cell_conc._interval+AS_Vol.+ emission_CO2+generate_CO2; 0.441, 0.724, tmp_cumulo+pH+pH_c umulo+PL+AN+interval_yield+RS+Cell_conc._interval+AS_Vol.+c onsum_O2+interval_O2; 0.432, 0.724, tmp+tmp_cumulo+pH+pH_cu mulo+PL+AN_cumulo+interval_yield+RS+Cell_conc._interval+RQ_ integral+AS_Vol.+emission_CO2; 0.450, 0.724, tmp+tmp_cumulo +pH+pH_cumulo+AN+interval_yield+RS+Cell_conc._interval+AS_V ol.+consum_O2; 0.450, 0.724, tmp_cumulo+pH+pH_cumulo+AN+int erval_yield+RS+Feed_Vol+Cell_conc._interval+AS_Vol.+interva l_O2; 0.441, 0.724, tmp_cumulo+pH+pH_cumulo+PL_cumulo+AN+in terval_yield+interval_Pdt.+RS+Feed_Vol+Cell_conc._interval+ AS_Vol.; 0.432, 0.724, tmp_cumulo+pH+pH_cumulo+PL+AN+interv al_yield+RS+Feed_Vol+Cell_conc._interval+AS_Vol.+interval_O 2+generate_CO2; 0.432, 0.724, tmp_cumulo+pH+pH_cumulo+AN+AN _cumulo+interval_yield+RS+Feed_Vol+Cell_conc._interval+AS_V ol.+emission CO2+consum_O2; 0.450, 0.724, tmp+tmp_cumulo+pH +pH_cumulo+PL+AN+interval_yield+RS+Cell_conc._interval+AS_V ol.; 0.423, 0.724, tmp_cumulo+pH+pH_cumulo+PL_cumulo+AN_cum ulo+interval_yield+RS+Feed_Vol+Cell_conc._interval+AS_Vol.+ emission_CO2+interval_O2+generate_CO2; 0.441, 0.724, tmp+tm p_cumulo+pH+pH_cumulo+AN_cumulo+interval_yield+RS+Cell_conc. _interval+rab_integral+AS_Vol.+emission_O2; 0.441, 0.724, t mp+tmp_cumulo+pH+pH_cumulo+AN_cumulo+interval_yield+interva l_Pdt.+RS+Cell_conc._interval+AS_Vol.+emission_CO2; 0.458, 0.724, tmp_cumulo+pH+pH_cumulo+AN_cumulo+interval_yield+RS+ Feed_Vol+Cell_conc._interval+interval_O2; 0.441, 0.724, tmp _cumulo+pH+pH_cumulo+AN_cumulo+interval_yield+interval_Pdt. +OD+RS+Cell_conc._interval+rab_integral+AS_Vol.; 0.441, 0.7 24, tmp_cumulo+pH+pH_cumulo+AN_cumulo+interval_yield+OD+RS+ Cell_conc._interval+rab_integral+AS_Vol.+emission_CO2; 0.43 2, 0.724, tmp_cumulo+pH+pH_cumulo+PL_cumulo+AN+AN_cumulo+in terval_yield+RS+Cell_conc._interval+rab_integral+AS_Vol.+em ission_CO2; 0.458, 0.724, tmp_cumulo+pH+AN_cumulo+interval_ yield+RS+Feed_Vol+Cell_conc._interval+RQ_integral+AS_Vol.; 0.432, 0.724, tmp_cumulo+pH+pH_cumulo+AN_cumulo+interval_yi eld+RS+Feed_Vol+Cell_conc._interval+rab_integral+AS_Vol.+em ission_CO2+consum_O2; 0.450, 0.724, tmp+tmp_cumulo+pH+pH_cu mulo+AN+interval_yield+RS+Cell_conc._interval+AS_Vol.+emiss ion_O2; 0.441, 0.724, tmp_cumulo+pH+pH_cumulo+AN_cumulo+int erval_yield+RS+Cell_conc._interval+rab_integral+RQ_integral +AS_Vol.+emission_CO2; 0.423, 0.724, tmp_cumulo+pH+pH_cumul o+AN+AN_cumulo+interval_yield+RS+Feed_Vol+Cell_conc._interv al+RQ_integral+AS_Vol.+emission_O2+emission_CO2; 0.441, 0.7 24, tmp+tmp_cumulo+pH+pH_cumulo+PL_cumulo+AN_cumulo+interva l_yield+RS+Cell_conc._interval+AS_Vol.+generate_CO2; 0.441, 0.724, tmp+tmp_cumulo+pH+pH_cumulo+AN_cumulo+interval_yiel d+interval_Pdt.+RS+Cell_conc._interval+AS_Vol.+generate_CO 2; 0.423, 0.724, tmp_cumulo+pH+pH_cumulo+PL_cumulo+AN+AN_cu mulo+interval_yield+RS+Feed_Vol+Cell_conc._interval+rab_int egral+RQ_integral+AS_Vol.; 0.432, 0.724, tmp+tmp_cumulo+pH+ pH_cumulo+AN+AN_cumulo+interval_yield+RS+Feed_Vol+Cell_conc. _interval+AS_Vol.+consum_O2; 0.441, 0.724, tmp_cumulo+pH+pH _cumulo+AN+AN_cumulo+interval_yield+RS+Cell_conc._interval+ AS_Vol.+emission_CO2+consum_O2; 0.458, 0.724, tmp+pH+pH_cum ulo+AN_cumulo+interval_yield+RS+RS_cumulo+Cell_conc._interv al+AS_Vol.; 0.432, 0.724, tmp+tmp_cumulo+pH+pH_cumulo+AN+AN _cumulo+interval_yield+RS+Feed_Vol+RS_cumulo+Cell_conc._int erval+AS_Vol.; 0.441, 0.724, tmp+tmp_cumulo+pH+pH_cumulo+PL +interval_yield+RS+Feed_Vol+Cell_conc._interval+AS_Vol.+int erval_O2; 0.432, 0.724, tmp_cumulo+pH+pH_cumulo+PL+AN_cumul o+interval_yield+RS+Feed_Vol+Cell_conc._interval+AS_Vol.+co nsum_O2+interval_O2; 0.466, 0.724, tmp_cumulo+pH+pH_cumulo+ interval_yield+RS+Cell_conc._interval+AS_Vol.+consum_O2; 0. 458, 0.724, tmp_cumulo+pH+pH_cumulo+interval_yield+interval _Pdt.+RS+Feed_Vol+Cell_conc._interval+consum_O2; 0.432, 0.7 24, tmp_cumulo+pH+pH_cumulo+AN_cumulo+interval_yield+RS+Fee d_Vol+RS_cumulo+Cell_conc._interval+rab_integral+RQ_integra l+AS_Vol.; 0.441, 0.724, tmp_cumulo+pH+pH_cumulo+AN+AN_cumu lo+interval_yield+RS+Cell_conc._interval+AS_Vol.+emission_O 2+emission_CO2; 0.450, 0.724, tmp_cumulo+pH+pH_cumulo+AN_cu mulo+interval_yield+RS+Feed_Vol+Cell_conc._interval+consum-O2+generate_CO2; 0.432, 0.724, tmp+tmp_cumulo+pH+pH_cumulo+ AN_cumulo+interval_yield+RS+Feed_Vol+Cell_conc._interval+ra b_integral+AS_Vol.+consum_O2; 0.458, 0.724, tmp_cumulo+pH+p H_cumulo+interval_yield+RS+Feed_Vol+Cell_conc._interval+RQ_ integral+generate_CO2; 0.474, 0.724, tmp_cumulo+pH+interval _yield+RS+Feed_Vol+Cell_conc._interval+RQ_integral; 0.450, 0.724, tmp_cumulo+pH+pH_cumulo+AN_cumulo+interval_yield+RS+ Feed_Vol+Cell_conc._interval+RQ_integral+generate_CO2; 0.43 2, 0.724, tmp_cumulo+pH+pH_cumulo+AN_cumulo+interval_yield+ OD+RS+Feed_Vol+Cell_conc._interval+AS_Vol.+emission_CO2+gen erate_CO2; 0.441, 0.724, tmp_cumulo+pH+pH_cumulo+AN+interva l_yield+RS+Cell_conc._interval+rab_integral+AS_Vol.+emissio n_CO2+generate_CO2; 0.432, 0.724, tmp_cumulo+pH+pH_cumulo+A N+AN_cumulo+interval_yield+RS+Feed_Vol+RS_cumulo+Cell_conc. _interval+AS_Vol.+emission_CO2; 0.449, 0.724, tmp_cumulo+pH +pH_cumulo+interval_yield+RS+Feed_Vol+Cell_conc._interval+r ab_integral+consum_O2+generate_CO2; 0.458, 0.724, tmp_cumul o+pH+pH_cumulo+interval_yield+RS+Cell_conc._interval+rab_in tegral+AS_Vol.+emission_CO2; 0.449, 0.724, tmp_cumulo+pH+pH _cumulo+interval_yield+RS+Feed_Vol+Cell_conc._interval+emis sion_CO2+interval_O2+generate_CO2; 0.432, 0.724, tmp_cumulo +pH+pH_cumulo+AN+AN_cumulo+interval_yield+RS+Cell_conc._int erval+rab_integral+RQ_integral+AS_Vol.+generate_CO2; 0.458, 0.724, tmp_cumulo+pH+pH_cumulo+interval_yield+RS+Cell_conc. _interval+AS_Vol.+emission_CO2+generate_CO2; 0.441, 0.724, tmp_cumulo+pH+pH_cumulo+PL+AN+interval_yield+interval_Pdt.+ RS+Cell_conc._interval+AS_Vol.+interval_O2; 0.441, 0.724, t mp_cumulo+pH+pH_cumulo+PL_cumulo+AN+AN_cumulo+interval_yiel d+interval_Pdt.+RS+Cell_conc._interval+AS_Vol.; 0.432, 0.72 4, tmp+tmp_cumulo+pH+pH_cumulo+AN_cumulo+interval_yield+int erval_Pdt.+RS+Feed_Vol+Cell_conc._interval+RQ_integral+AS_V ol.; 0.449, 0.724, tmp_cumulo+pH+pH_cumulo+AN+interval_yiel d+RS+Feed_Vol+Cell_conc._interval+rab_integral+generate_CO 2; 0.449, 0.724, tmp_cumulo+pH+pH_cumulo+interval_yield+RS+ Feed_Vol+Cell_conc._interval+rab_integral+AS_Vol.+emission_ CO2; 0.449, 0.724, tmp+pH+pH_cumulo+PL_cumulo+AN_cumulo+int erval_yield+RS+Cell_conc._interval+rab_integral+AS_Vol.; 0. 449, 0.724, tmp_cumulo+pH+pH_cumulo+AN_cumulo+interval_yiel d+RS+Feed_Vol+Cell_conc._interval+interval_O2+generate_CO2; 0.432, 0.724, tmp+tmp_cumulo+pH+pH_cumulo+PL+AN+AN_cumulo+ interval_yield+RS+Cell_conc._interval+rab_integral+AS-Vol.; 0.449, 0.724, tmp_cumulo+pH+pH_cumulo+PL_cumulo+AN+interva l_yield+RS+Cell_conc._interval+RQ_integral+AS_Vol.; 0.449, 0.724, tmp+tmp_cumulo+pH+pH_cumulo+AN_cumulo+interval_yield +RS+Feed_Vol+Cell_conc._interval+rab_integral; 0.449, 0.724, tmp_cumulo+pH+pH_cumulo+interval_yield+RS+Feed_Vol+Cell_co nc._interval+AS_Vol.+interval_O2+generate_CO2; 0.432, 0.724, tmp_cumulo+pH+pH_cumulo+PL_cumulo+AN_cumulo+interval_yield +RS+Feed_Vol+RS_cumulo+Cell_conc._interval+rab_integral+AS_ Vol.; 0.441, 0.724, tmp+tmp_cumulo+pH+pH_cumulo+AN_cumulo+i nterval_yield+RS+RS_cumulo+Cell_conc._interval+AS_Vol.+gene rate_CO2; 0.432, 0.724, tmp_cumulo+pH+pH_cumulo+AN_cumulo+i nterval_yield+RS+Cell_conc._interval+rab_integral+AS_Vol.+e mission_CO2+consum_O2+generate_CO2; 0.449, 0.724, tmp+tmp_c umulo+pH+pH_cumulo+AN+interval_yield+RS+RS_cumulo+Cell_conc. _interval+AS_Vol.; 0.441, 0.724, tmp_cumulo+pH+pH_cumulo+in terval_yield+RS+Feed_Vol+Cell_conc._interval+rab_integral+e mission_CO2+interval_O2+generate_CO2; 0.422, 0.724, tmp_cum ulo+pH+pH_cumulo+PL_cumulo+AN+interval_yield+RS+Feed_Vol+Ce ll_conc._interval+AS_Vol.+emission_CO2+interval_O2+generate _CO2; 0.466, 0.724, tmp_cumulo+pH+pH_cumulo+interval_yield+ OD+RS+Feed_Vol+Cell_conc._interval; 0.449, 0.724, tmp_cumul o+pH+pH_cumulo+AN+AN_cumulo+interval_yield+RS+Feed_Vol+Cell _conc._interval+emission_CO2; 0.449, 0.724, tmp_cumulo+pH+p H_cumulo+AN+AN_cumulo+interval_yield+interval_Pdt.+RS+Feed_ Vol+Cell_conc._interval; 0.466, 0.724, tmp_cumulo+pH+pH_cum ulo+interval_yield+OD+RS+Cell_conc._interval+AS_Vol.; 0.431, 0.724, tmp_cumulo+pH+pH_cumulo+AN+AN_cumulo+interval_yield +RS+Feed_Vol+Cell_conc._interval+RQ_integral+AS_Vol.+emissi on_CO2; 0.449, 0.724, tmp_cumulo+pH+pH_cumulo+PL_cumulo+int erval_yield+RS+Feed_Vol+Cell_conc._interval+AS_Vol.+interva l_O2; 0.440, 0.724, tmp_cumulo+pH+pH_cumulo+AN+AN_cumulo+in terval_yield+OD+RS+Cell_conc._interval+AS_Vol.+emission_CO 2; 0.431, 0.724, tmp+tmp_cumulo+pH+pH_cumulo+AN_cumulo+inte rval_yield+RS+Feed_Vol+RS_cumulo+Cell_conc._interval+AS_Vol. +emission_CO2; 0.431, 0.724, tmp_cumulo+pH+pH_cumulo+AN+AN_ cumulo+interval_yield+RS+Feed_Vol+Cell_conc._interval+AS_Vo l.+interval_O2+generate_CO2; 0.431, 0.724, tmp_cumulo+pH+pH _cumulo+PL_cumulo+AN_cumulo+interval_yield+RS+Feed_Vol+RS_c umulo+Cell_conc._interval+AS_Vol.+generate_CO2; 0.466, 0.72 4, pH+pH_cumulo+PL_cumulo+AN+interval_yield+RS+Cell_conc._i nterval+AS_Vol.; 0.449, 0.724, tmp_cumulo+pH+pH_cumulo+AN_c umulo+interval_yield+OD+RS+Cell_conc._interval+AS_Vol.+cons um_O2; 0.431, 0.724, tmp+tmp_cumulo+pH+pH_cumulo+AN_cumulo+ interval_yield+RS+Feed_Vol+Cell_conc._interval+RQ_integral+ AS_Vol.+emission_O2; 0.431, 0.724, tmp_cumulo+pH+pH_cumulo+ PL_cumulo+AN_cumulo+interval_yield+RS+Feed_Vol+Cell_conc._i nterval+AS_Vol.+consum_O2+generate_CO2; 0.431, 0.724, tmp+t mp_cumulo+pH+pH_cumulo+AN_cumulo+interval_yield+RS+Feed_Vol +Cell_conc._interval+AS_Vol.+emission_CO2+consum_02; 0.422, 0.724, tmp_cumulo+pH+pH_cumulo+AN_cumulo+interval_yield+in terval_Pdt.+RS+Feed_Vol+Cell_conc._interval+rab_integral+AS _Vol.+emission_CO2+generate_CO2; 0.449, 0.724, tmp+tmp_cumu lo+pH+pH_cumulo+interval_yield+RS+Feed_Vol+Cell_conc._inter val+AS_Vol.+emission_CO2; 0.449, 0.724, tmp_cumulo+pH+pH_cu mulo+AN_cumulo+interval_yield+RS+Feed_Vol+Cell_conc._interv al+rab_integral+emission_O2; 0.458, 0.724, tmp+pH+pH_cumulo +AN+AN_cumulo+interval_yield+RS+Cell_conc._interval+AS_Vo 1.; 0.449, 0.724, tmp+tmp_cumulo+pH+pH_cumulo+PL_cumulo+AN+ interval_yield+RS+Cell_conc._interval+AS_Vol.; 0.431, 0.724, tmp_cumulo+pH+pH_cumulo+AN_cumulo+interval_yield+RS+RS_cum ulo+Cell_conc._interval+rab_integral+AS_Vol.+emission_CO2+g enerate_CO2; 0.440, 0.724, tmp_cumulo+pH+pH_cumulo+AN+AN_cu mulo+interval_yield+RS+RS_cumulo+Cell_conc._interval+AS_Vol. +emission_CO2; 0.440, 0.724, tmp_cumulo+pH+pH_cumulo+AN_cum ulo+interval_yield+RS+RS_cumulo+Cell_conc._interval+rab_int egral+AS_Vol.+consum_O2; 0.440, 0.724, tmp_cumulo+pH+pH_cum ulo+PL_cumulo+AN+interval_yield+RS+Feed_Vol+Cell_conc._inte rval+AS_Vol.+emission_CO2; 0.422, 0.724, tmp_cumulo+pH+pH_c umulo+PL+AN+interval_yield+RS+Cell_conc._interval+rab_integ ral+AS_Vol.+emission_CO2+interval_O2+generate_CO2; 0.440, 0. 723, tmp_cumulo+pH+pH_cumulo+AN+interval_yield+RS+Cell_conc. _interval+RQ_integral+AS_Vol.+emission_O2+emission_CO2; 0.4 40, 0.723, tmp_cumulo+pH+pH_cumulo+AN_cumulo+interval_yield +OD+RS+Feed_Vol+Cell_conc._interval+RQ_integral+AS_Vol.; 0. 422, 0.723, tmp_cumulo+pH+pH_cumulo+AN+AN_cumulo+interval_y ield+RS+Feed_Vol+Cell_conc._interval+AS_Vol.+emission_O2+em ission_CO2+generate_CO2; 0.457, 0.723, tmp_cumulo+pH+pH_cum ulo+interval_yield+RS+Cell_conc._interval+rab_integral+AS_V ol.+generate_CO2; 0.431, 0.723, tmp_cumulo+pH+pH_cumulo+AN+ AN_cumulo+interval_yield+OD+RS+Feed_Vol+Cell_conc._interval +AS_Vol.+generate_CO2; 0.431, 0.723, tmp_cumulo+pH+pH_cumul o+AN+AN_cumulo+interval_yield+RS+Feed_Vol+RS_cumulo+Cell_co nc._interval+rab_integral+AS_Vol.; 0.440, 0.723, tmp_cumulo +pH+pH_cumulo+PL_cumulo+AN+AN_cumulo+interval_yield+OD+RS+C ell_conc._interval+AS_Vol.; 0.440, 0.723, tmp_cumulo+pH+pH_ cumulo+AN+interval_yield+RS+Feed_Vol+Cell_conc._interval+ra b_integral+interval_O2+generate_CO2; 0.440, 0.723, tmp_cumu lo+pH+pH_cumulo+AN+interval_yield+interval_Pdt.+RS+Feed_Vol +Cell_conc._interval+AS_Vol.+emission_O2; 0.422, 0.723, tmp _cumulo+pH+pH_cumulo+AN+AN_cumulo+interval_yield+interval_P dt.+RS+Feed_Vol+Cell_conc._interval+AS_Vol.+emission_CO2+ge nerate_CO2; 0.431, 0.723, tmp_cumulo+pH+pH_cumulo+AN+AN_cum ulo+interval_yield+RS+Cell_conc._interval+rab_integral+AS_V ol.+emission O2+emission CO2; 0.431, 0.723, tmp+tmp_cumulo+ pH+pH_cumulo+AN_cumulo+interval_yield+RS+Feed_Vol+Cell_conc. _interval+RQ_integral+AS_Vol.+emission_CO2; 0.474, 0.723, t mp_cumulo+pH+interval_yield+RS+Feed_Vol+Cell_conc._interval +generate_CO2; 0.431, 0.723, tmp_cumulo+pH+pH_cumulo+AN_cum ulo+interval_yield+interval_Pdt.+RS+Cell_conc._interval+rab _integral+AS_Vol.+emission_CO2+generate_CO2; 0.431, 0.723, mp+tmp_cumulo+pH+pH_cumulo+AN_cumulo+interval_yield+RS+Cel l_conc._interval+RQ_integral+AS_Vol.+emission_O2+emission_C 02; 0.440, 0.723, tmp+tmp_cumulo+pH+pH_cumulo+PL+AN_cumulo+ interval_yield+RS+Cell_conc._interval+AS_Vol.+interval_O2; 0.431, 0.723, tmp+tmp_cumulo+pH+pH_cumulo+PL_cumulo+AN_cumu lo+interval_yield+RS+Cell_conc._interval+rab_integral+AS_Vo l.+generate_CO2; 0.449, 0.723, tmp_cumulo+pH+pH_cumulo+inte rval_yield+RS+Feed_Vol+Cell_conc._interval+rab_integral+RQ_ integral+consum_O2; 0.466, 0.723, tmp_cumulo+pH+pH_cumulo+i nterval_yield+RS+Feed_Vol+RS_cumulo+Cell_conc._interval; 0. 449, 0.723, tmp_cumulo+pH+pH_cumulo+AN+interval_yield+RS+Ce ll_conc._interval+AS_Vol.+emission_O2+generate_CO2; 0.431, 0.723, tmp+tmp_cumulo+pH+pH_cumulo+AN+interval_yield+RS+Fee d_Vol+Cell_conc._interval+rab_integral+AS_Vol.+generate_CO 2; 0.457, 0.723, tmp_cumulo+pH+pH_cumulo+interval_yield+OD+ RS+Feed_Vol+Cell_conc._interval+AS_Vol.; 0.422, 0.723, tmp_ cumulo+pH+pH_cumulo+AN_cumulo+interval_yield+RS+Cell_conc._ interval+rab_integral+RQ_integral+AS_Vol.+emission_O2+emiss ion_CO2+generate_CO2; 0.457, 0.723, tmp_cumulo+pH+pH_cumulo +interval_yield+RS+Feed_Vol+Cell_conc._interval+RQ_integral +emission_CO2; 0.431, 0.723, tmp+tmp_cumulo+pH+pH_cumulo+AN _cumulo+interval_yield+RS+Feed_Vol+RS_cumulo+Cell_conc._int erval+rab_integral+AS_Vol.; 0.431, 0.723, tmp_cumulo+pH+pH_ cumulo+AN_cumulo+interval_yield+interval_Pdt.+RS+Feed_Vol+C ell_conc._interval+rab_integral+AS_Vol.+consum_O2; 0.431, 0. 723, tmp_cumulo+pH+pH_cumulo+PL+AN_cumulo+interval_yield+RS +Cell_conc._interval+RQ_integral+AS_Vol.+emission_CO2+gener ate_CO2; 0.440, 0.723, tmp_cumulo+pH+pH_cumulo+AN_cumulo+in terval_yield+RS+Cell_conc._interval+RQ_integral+AS_Vol.+emi ssion_CO2+generate_CO2; 0.440, 0.723, tmp_cumulo+pH+pH_cumu lo+AN_cumulo+interval_yield+RS+Feed_Vol+Cell_conc._interval +AS_Vol.+emission_CO2+interval_O2; 0.457, 0.723, tmp_cumulo +pH+pH_cumulo+AN+interval_yield+RS+Feed_Vol+Cell_conc._inte rval+RQ_integral; 0.440, 0.723, tmp_cumulo+pH+pH_cumulo+AN+ interval_yield+RS+Feed_Vol+Cell_conc._interval+rab_integral +AS_Vol.+consum_O2; 0.440, 0.723, tmp_cumulo+pH+pH_cumulo+A N+interval_yield+RS+Cell_conc._interval+rab_integral+RQ_int egral+AS_Vol.+generate_CO2; 0.449, 0.723, tmp_cumulo+pH+pH_ cumulo+AN+interval_yield+RS+Feed_Vol+Cell_conc._interval+Ac cum._Yield+AS_Vol.; 0.449, 0.723, tmp_cumulo+pH+pH_cumulo+A N+interval_yield+interval_Pdt.+OD+RS+Cell_conc._interval+AS _Vol.; 0.431, 0.723, tmp_cumulo+pH+pH_cumulo+AN_cumulo+inte rval_yield+RS+Feed_Vol+RS_cumulo+Cell_conc._interval+rab_in tegral+AS_Vol.+emission_CO2; 0.440, 0.723, tmp+tmp_cumulo+p H+pH_cumulo+AN_cumulo+interval_yield+RS+Cell_conc._interval +AS_Vol.+consum_O2+generate_CO2; 0.440, 0.723, tmp+tmp_cumu lo+pH+pH_cumulo+AN+interval_yield+RS+Feed_Vol+Cell_conc._in terval+AS_Vol.+consum_O2; 0.431, 0.723, tmp+tmp_cumulo+pH+p H_cumulo+AN+AN_cumulo+interval_yield+RS+Cell_conc._interval +rab_integral+AS_Vol.+generate_CO2; 0.431, 0.723, tmp_cumul o+pH+pH_cumulo+PL+AN_cumulo+interval_yield+interval_Pdt.+RS +Feed_Vol+Cell_conc._interval+AS_Vol.+interval_O2; 0.422, 0. 723, tmp+tmp_cumulo+pH+pH_cumulo+PL_cumulo+AN+AN_cumulo+int erval_yield+RS+Feed_Vol+Cell_conc._interval+AS_Vol.+generat e_CO2; 0.440, 0.723, tmp_cumulo+pH+pH_cumulo+AN_cumulo+inte rval_yield+RS+Feed_Vol+Cell_conc._interval+rab_integral+RQ_ integral+generate_CO2; 0.431, 0.723, tmp_cumulo+pH+pH_cumul o+AN_cumulo+interval_yield+RS+Feed_Vol+Cell_conc._interval+ RQ_integral+AS_Vol.+emission_O2+generate_CO2; 0.440, 0.723, tmp_cumulo+pH+pH_cumulo+AN+interval_yield+RS+Feed_Vol+Cell _conc._interval+AS_Vol.+emission_CO2+consum_02; 0.421, 0.72 3, tmp+tmp_cumulo+pH+pH_cumulo+AN_cumulo+interval_yield+RS+ Feed_Vol+Cell_conc._interval+rab_integral+RQ_integral+AS_Vo l.+generate_CO2; 0.431, 0.723, tmp_cumulo+pH+pH_cumulo+PL+A N_cumulo+interval_yield+RS+Cell_conc._interval+rab_integral +AS_Vol.+interval_O2+generate_CO2; 0.449, 0.723, tmp_cumulo +pH+pH_cumulo+PL+AN+interval_yield+RS+Feed_Vol+Cell_conc._i nterval+AS_Vol.; 0.421, 0.723, tmp_cumulo+pH+pH_cumulo+PL_c umulo+AN_cumulo+interval_yield+RS+Feed_Vol+Cell_conc._inter val+rab_integral+RQ_integral+AS_Vol.+generate_CO2; 0.440, 0. 723, tmp+tmp_cumulo+pH+pH_cumulo+AN_cumulo+interval_yield+R S+RS_cumulo+Cell_conc._interval+AS_Vol.+emission_CO2; 0.457, 0.723, tmp+tmp_cumulo+pH+pH_cumulo+interval_yield+RS+Feed_ Vol+Cell_conc._interval+emission_CO2; 0.449, 0.723, tmp_cum ulo+pH+pH_cumulo+AN_cumulo+interval_yield+RS+Cell_conc._int erval+Accum._Yield+AS_Vol.+emission_CO2; 0.465, 0.723, tmp_ cumulo+pH+interval_yield+RS+Feed_Vol+Cell_conc._interval+em ission_CO2+generate_CO2; 0.440, 0.723, tmp+tmp_cumulo+pH+pH _cumulo+AN_cumulo+interval_yield+OD+RS+Cell_conc._interval+ AS_Vol.+generate_CO2; 0.440, 0.723, tmp_cumulo+pH+pH_cumulo +AN_cumulo+interval_yield+RS+Cell_conc._interval+rab_integr al+RQ_integral+AS_Vol.+emission_O2; 0.457, 0.723, tmp_cumul o+pH+AN_cumulo+interval_yield+RS+Feed_Vol+Cell_conc._interv al+rab_integral+AS_Vol.; 0.440, 0.723, tmp_cumulo+pH+pH_cum ulo+PL+AN+interval_yield+OD+RS+Cell_conc._interval+AS_Vol.+ interval_O2; 0.431, 0.723, tmp+tmp_cumulo+pH+pH_cumulo+AN+A N_cumulo+interval_yield+RS+Cell_conc._interval+AS_Vol.+emis sion_CO2+generate_CO2; 0.448, 0.723, tmp_cumulo+pH+pH_cumul o+AN+interval_yield+RS+Cell_conc._interval+AS_Vol.+emission _O2+emission_CO2; 0.448, 0.723, tmp_cumulo+pH+pH_cumulo+AN+ interval_yield+interval_Pdt.+RS+Cell_conc._interval+AS_Vol. +emission_CO2; 0.421, 0.723, tmp+tmp_cumulo+pH+pH_cumulo+AN _cumulo+interval_yield+RS+Feed_Vol+Cell_conc._interval+RQ_i ntegral+AS_Vol.+emission_O2+emission_CO2; 0.440, 0.723, tmp +tmp_cumulo+pH+pH_cumulo+PL+AN_cumulo+interval_yield+interv al_Pdt.+RS+Cell_conc._interval+AS_Vol.; 0.421, 0.723, tmp_c umulo+pH+pH_cumulo+PL_cumulo+AN_cumulo+interval_yield+inter val_Pdt.+RS+Feed_Vol+Cell_conc._interval+AS_Vol.+emission_C O2+generate_CO2; 0.431, 0.723, tmp_cumulo+pH+pH_cumulo+PL_c umulo+AN+interval_yield+RS+Feed_Vol+Cell_conc._interval+AS_ Vol.+emission_CO2+generate_CO2; 0.457, 0.723, tmp+pH+pH_cum ulo+AN_cumulo+interval_yield+RS+Cell_conc._interval+rab_int egral+AS_Vol.; 0.431, 0.723, tmp_cumulo+pH+pH_cumulo+AN_cum ulo+interval_yield+RS+Cell_conc._interval+RQ_integral+AS_Vo l.+emission_O2+emission_CO2+consum_O2; 0.431, 0.723, tmp_cu mulo+pH+pH_cumulo+PL+AN+AN_cumulo+interval_yield+interval_P dt.+RS+Cell_conc._interval+rab_integral+AS_Vol.; 0.440, 0.7 23, tmp_cumulo+pH+pH_cumulo+AN+interval_yield+RS+Feed_Vol+C ell_conc._interval+RQ_integral+AS_Vol.+emission_CO2; 0.421, 0.723, tmp_cumulo+pH+pH_cumulo+PL_cumulo+AN+AN_cumulo+inte rval_yield+RS+Feed_Vol+Cell_conc._interval+RQ_integral+AS_V ol.+generate_CO2; 0.431, 0.723, tmp+tmp_cumulo+pH+pH_cumulo +PL_cumulo+AN+AN_cumulo+interval_yield+RS+Cell_conc._interv al+rab_integral+AS_Vol.; 0.457, 0.723, tmp_cumulo+pH+pH_cum ulo+interval_yield+interval_Pdt.+RS+Feed_Vol+Cell_conc._int erval+generate_CO2; 0.440, 0.723, tmp_cumulo+pH+pH_cumulo+A N_cumulo+interval_yield+RS+Feed_Vol+Cell_conc._interval+rab _integral+consum_O2+generate_CO2; 0.448, 0.723, tmp_cumulo+ pH+pH_cumulo+AN_cumulo+interval_yield+RS+Feed_Vol+Cell_conc. O_interval+emission_CO2+consum_O2; 0.421, 0.723, tmp+tmp_cum ulo+pH+pH_cumulo+PL_cumulo+AN_cumulo+interval_yield+RS+Feed _Vol+Cell_conc._interval+AS_Vol.+emission_CO2+generate_CO2; 0.457, 0.723, tmp+tmp_cumulo+pH+pH_cumulo+interval_yield+R S+Feed_Vol+Cell_conc._interval+interval_O2; 0.440, 0.723, t mp_cumulo+pH+pH_cumulo+AN+AN_cumulo+interval_yield+RS+Feed_ Vol+Cell_conc._interval+Accum._Yield+AS_Vol.; 0.457, 0.723, tmp+pH+pH_cumulo+AN_cumulo+interval_yield+RS+Cell_conc._in terval+AS_Vol.+generate_CO2; 0.448, 0.723, tmp_cumulo+pH+pH _cumulo+AN+AN_cumulo+interval_yield+RS+Feed_Vol+Cell_conc._ interval+consum_O2; 0.440, 0.723, tmp+tmp_cumulo+pH+pH_cumu lo+AN_cumulo+interval_yield+RS+Cell_conc._interval+AS_Vol.+ emission_CO2+consum_O2; 0.440, 0.723, tmp_cumulo+pH+pH_cumu lo+PL_cumulo+AN+interval_yield+RS+Cell_conc._interval+rab_i ntegral+AS_Vol.+generate_CO2; 0.430, 0.723, tmp_cumulo+pH+p H_cumulo+PL_cumulo+AN_cumulo+interval_yield+RS+Feed_Vol+Cel l_conc._interval+RQ_integral+AS_Vol.+emission_CO2; 0.430, 0. 723, tmp+tmp_cumulo+pH+pH_cumulo+AN+AN_cumulo+interval_yiel d+RS+Cell_conc._interval+rab_integral+RQ_integral+AS_Vol.; 0.421, 0.723, tmp_cumulo+pH+pH_cumulo+PL+AN+AN_cumulo+inter val_yield+RS+Cell_conc._interval+rab_integral+RQ_integral+A S_Vol.+emission_CO2; 0.439, 0.723, tmp_cumulo+pH+pH_cumulo+ AN+interval_yield+OD+RS+Feed_Vol+Cell_conc._interval+AS_Vol. +generate_CO2; 0.421, 0.723, tmp_cumulo+pH+pH_cumulo+PL+AN+ AN_cumulo+interval_yield+RS+Cell_conc._interval+AS_Vol.+emi ssion_CO2+interval_O2+generate_CO2; 0.448, 0.723, tmp+tmp_c umulo+pH+pH_cumulo+interval_yield+RS+Feed_Vol+Cell_conc._in terval+AS_Vol.+generate_CO2; 0.473, 0.723, tmp_cumulo+pH+in terval_yield+RS+Feed_Vol+Cell_conc._interval+emission_O2; 0. 448, 0.723, tmp_cumulo+pH+pH_cumulo+PL+AN+interval_yield+in terval_Pdt.+RS+Cell_conc._interval+AS_Vol.; 0.430, 0.723, t mp_cumulo+pH+pH_cumulo+PL+AN+interval_yield+RS+Feed_Vol+Cel l_conc._interval+AS_Vol.+consum_O2+interval_O2; 0.439, 0.72 3, tmp+tmp_cumulo+pH+pH_cumulo+AN+AN_cumulo+interval_yield+ RS+Cell_conc._interval+AS_Vol.+emission_O2; 0.439, 0.723, t mp_cumulo+pH+pH_cumulo+AN+AN_cumulo+interval_yield+interval _Pdt.+OD+RS+Cell_conc._interval+AS_Vol.; 0.465, 0.723, tmp_ cumulo+pH+AN+interval_yield+RS+Feed_Vol+Cell_conc._interval +AS_Vol.; 0.439, 0.723, tmp+tmp_cumulo+pH+pH_cumulo+PL_cumu lo+AN_cumulo+interval_yield+RS+Cell_conc._interval+AS_Vol.+ emission_CO2; 0.430, 0.723, tmp_cumulo+pH+pH_cumulo+AN+AN_c umulo+interval_yield+RS+Feed_Vol+Cell_conc._interval+RQ_int egral+AS_Vol.+consum_O2; 0.448, 0.723, tmp_cumulo+pH+pH_cum ulo+AN+interval_yield+RS+Feed_Vol+Cell_conc._interval+rab_i ntegral+RQ_integral; 0.421, 0.723, tmp_cumulo+pH+pH_cumulo+ PL_cumulo+AN+AN_cumulo+interval_yield+RS+Cell_conc._interva l+rab_integral+AS_Vol.+emission_CO2+generate_CO2; 0.421, 0. 723, tmp+tmp_cumulo+pH+pH_cumulo+AN+AN_cumulo+interval_yiel d+RS+Feed_Vol+Cell_conc._interval+rab_integral+RQ_integral+ AS_Vol.; 0.430, 0.723, tmp+tmp_cumulo+pH+pH_cumulo+PL+AN+in terval_yield+RS+Cell_conc._interval+rab_integral+AS_Vol.+in terval_O2; 0.430, 0.723, tmp+tmp_cumulo+pH+pH_cumulo+AN_cum ulo+interval_yield+RS+Cell_conc._interval+rab_integral+AS_V ol.+emission_O2+emission_CO2; 0.430, 0.723, tmp_cumulo+pH+p H_cumulo+AN+AN_cumulo+interval_yield+RS+Cellconc. _interval +AS_Vol.+emission_CO2+consum_O2+generate_CO2; 0.473, 0.723, tmp_cumulo+pH+interval_yield+RS+Feed_Vol+Cell_conc._interv al+rab_integral; 0.448, 0.723, tmp_cumulo+pH+pH_cumulo+inte rval_yield+interval_Pdt.+RS+Feed_Vol+Cell_conc._interval+AS _Vol.+emission_CO2; 0.439, 0.723, tmp_cumulo+pH+pH_cumulo+A N+AN_cumulo+interval_yield+RS+Feed_Vol+Cell_conc._interval+ AS_Vol.+interval_O2; 0.448, 0.723, tmp_cumulo+pH+pH_cumulo+ interval_yield+RS+Feed_Vol+Cell_conc._interval+AS_Vol.+emis sion_CO2+consum_O2; 0.430, 0.723, tmp_cumulo+pH+pH_cumulo+A N+AN_cumulo+interval_yield+interval_Pdt.+RS+Feed_Vol+Cell_c onc._interval+AS_Vol.+emission_O2; 0.430, 0.723, tmp_cumulo +pH+pH_cumulo+PL_cumulo+AN+AN_cumulo+interval_yield+RS+Feed _Vol+Cell_conc._interval+AS_Vol.+consum_O2; 0.448, 0.723, t mp_cumulo+pH+pH_cumulo+AN+interval_yield+OD+RS+Cell_conc._i nterval+AS_Vol.+emission_CO2; 0.430, 0.723, tmp_cumulo+pH+p H_cumulo+PL+AN+interval_yield+RS+Cell_conc._interval+rab_in tegral+AS_Vol.+interval_O2+generate_CO2; 0.430, 0.723, tmp+ tmp_cumulo+pH+pH_cumulo+PL+AN_cumulo+interval_yield+RS+Cell _conc._interval+rab_integral+RQ_integral+AS_Vol.; 0.430, 0. 723, tmp_cumulo+pH+pH_cumulo+PL_cumulo+AN+interval_yield+RS +Feed_Vol+Cell_conc._interval+rab_integral+AS_Vol.+generate _CO2; 0.421, 0.723, tmp_cumulo+pH+pH_cumulo+AN_cumulo+inter val_yield+interval_Pdt.+RS+Feed_Vol+Cell_conc._interval+RQ_ integral+AS_Vol.+emission_O2+emission_CO2; 0.439, 0.723, tm p_cumulo+pH+pH_cumulo+PL+AN_cumulo+interval_yield+RS+RS_cum ulo+Cell_conc._interval+AS_Vol.+emission_CO2; 0.439, 0.723, tmp_cumulo+pH+pH_cumulo+AN+AN_cumulo+interval_yield+RS+Cel l_conc._interval+RQ_integral+AS_Vol.+consum_O2; 0.430, 0.72 3, tmp+tmp_cumulo+pH+pH_cumulo+PL_cumulo+AN_cumulo+interval _yield+RS+Cell_conc._interval+rab_integral+AS_Vol.+emission _CO2; 0.439, 0.723, tmp_cumulo+pH+pH_cumulo+AN_cumulo+inter val_yield+RS+Feed_Vol+Cell_conc._interval+Accum._Yield+AS_V ol.+generate_CO2; 0.439, 0.723, tmp_cumulo+pH+pH_cumulo+PL_ cumulo+interval_yield+RS+Feed_Vol+Cell_conc._interval+AS_Vo l.+interval_O2+generate_CO2; 0.430, 0.723, tmp_cumulo+pH+pH _cumulo+AN+AN_cumulo+interval_yield+RS+Cell_conc._interval+ AS_Vol.+emission_O2+emission_CO2+generate_CO2; 0.456, 0.723, tmp_cumulo+pH+interval_yield+RS+Feed_Vol+Cell_conc._interv al+emission_CO2+interval_O2+generate_CO2; 0.456, 0.723, pH+ pH_cumulo+PL_cumulo+AN+AN_cumulo+interval_yield+RS+Cell_con c._interval+AS_Vol.; 0.465, 0.723, tmp_cumulo+pH+pH_cumulo+ interval_yield+RS+RS_cumulo+Cell_conc._interval+AS_Vol.; 0. 430, 0.723, tmp_cumulo+pH+pH_cumulo+AN_cumulo+interval_yiel d+RS+Cell_conc._interval+rab_integral+RQ_integral+AS_Vol.+e mission_CO2+generate_CO2; 0.430, 0.723, tmp_cumulo+pH+pH_cu mulo+PL+AN_cumulo+interval_yield+RS+Cell_conc._interval+rab _integral+Accum._Yield+AS_Vol.+emission_CO2; 0.456, 0.723, tmp_cumulo+pH+pH_cumulo+AN+interval_yield+RS+Feed_Vol+Cell_ conc._interval+emission_O2; 0.439, 0.723, tmp_cumulo+pH+pH_ cumulo+interval_yield+RS+Feed_Vol+Cell_conc._interval+RQ_in tegral+AS_Vol.+emission_O2+emission_CO2; 0.430, 0.723, tmp+ tmp_cumulo+pH+pH_cumulo+PL+AN+AN_cumulo+interval_yield+RS+C ell_conc._interval+AS_Vol.+emission_CO2; 0.439, 0.723, tmp_ cumulo+pH+pH_cumulo+PL_cumulo+AN+interval_yield+RS+Cell_con c._interval+AS_Vol.+emission_CO2+generate_CO2; 0.448, 0.723, tmp_cumulo+pH+pH_cumulo+PL+AN_cumulo+interval_yield+RS+Cel l_conc._interval+Accum._Yield+AS_Vol.; 0.448, 0.723, tmp_cu mulo+pH+pH_cumulo+AN_cumulo+interval_yield+interval_Pdt.+RS +Feed_Vol+Cell_conc._interval+RQ_integral; 0.430, 0.723, tm p_cumulo+pH+pH_cumulo+AN_cumulo+interval_yield+interval_Pdt. +RS+Feed_Vol+Cell_conc._interval+RQ_integral+AS_Vol.+genera te_CO2; 0.439, 0.723, tmp+tmp_cumulo+pH+pH_cumulo+PL+AN_cum ulo+interval_yield+RS+Cell_conc._interval+RQ_integral+AS_Vo l.; 0.456, 0.723, tmp+pH+pH_cumulo+AN_cumulo+interval_yield +RS+Cell_conc._interval+AS_Vol.+emission_CO2; 0.456, 0.723, tmp_cumulo+pH+AN_cumulo+interval_yield+RS+Feed_Vol+Cell_co nc._interval+AS_Vol.+consum_O2; 0.439, 0.723, tmp_cumulo+pH +pH_cumulo+PL_cumulo+AN+AN_cumulo+interval_yield+RS+Cell_co nc._interval+AS_Vol.+consum_O2; 0.430, 0.723, tmp_cumulo+pH +pH_cumulo+AN+interval_yield+RS+Feed_Vol+Cell_conc._interva l+AS_Vol.+emission_O2+emission_CO2+generate_CO2; 0.421, 0.7 23, tmp+tmp_cumulo+pH+pH_cumulo+PL_cumulo+AN_cumulo+interva l_yield+RS+Feed_Vol+Cell_conc._interval+rab_integral+AS_Vol. +generate_CO2; 0.430, 0.723, tmp_cumulo+pH+pH_cumulo+AN_cum ulo+interval_yield+interval_Pdt.+RS+Feed_Vol+Cell_conc._int erval+rab_integral+AS_Vol.+emission_O2; 0.448, 0.723, tmp_c umulo+pH+pH_cumulo+interval_yield+RS+Feed_Vol+Cell_conc._in terval+rab_integral+emission_CO2+consum_O2; 0.430, 0.723, t mp_cumulo+pH+pH_cumulo+PL_cumulo+AN_cumulo+interval_yield+i nterval_Pdt.+RS+Cell_conc._interval+rab_integral+AS_Vol.+em ission_CO2; 0.448, 0.723, tmp+tmp_cumulo+pH+pH_cumulo+inter val_yield+RS+Feed_Vol+Cell_conc._interval+AS_Vol.+consum_O 2; 0.430, 0.723, tmp_cumulo+pH+pH_cumulo+PL+AN+interval_yie ld+RS+Feed_Vol+Cell_conc._interval+Accum._Yield+AS_Vol.+int erval_O2; 0.430, 0.723, tmp_cumulo+pH+pH_cumulo+PL_cumulo+A N+AN_cumulo+interval_yield+interval_Pdt.+RS+Cell_conc._inte rval+rab_integral+AS_Vol.; 0.439, 0.723, tmp+tmp_cumulo+pH+ pH_cumulo+AN_cumulo+interval_yield+RS+Feed_Vol+Cell_conc._i nterval+AS_Vol.+interval_O2; 0.430, 0.723, tmp_cumulo+pH+pH _cumulo+AN_cumulo+interval_yield+interval_Pdt.+RS+Feed_Vol+ RS_cumulo+Cell_conc._interval+rab_integral+AS_Vol.; 0.420, 0.723, tmp+tmp_cumulo+pH+pH_cumulo+PL+AN_cumulo+interval_yi eld+RS+Cell_conc._interval+rab_integral+AS_Vol.+emission_CO 2+generate_CO2; 0.420, 0.723, tmp+tmp_cumulo+pH+pH_cumulo+P L+AN_cumulo+interval_yield+RS+Cell_conc._interval+rab_integ ral+AS_Vol.+emission_CO2+interval_O2; 0.448, 0.723, tmp_cum ulo+pH+pH_cumulo+interval_yield+RS+Feed_Vol+Cell_conc._inte rval+AS_Vol.+consum_O2+generate_CO2; 0.439, 0.723, tmp_cumu lo+pH+pH_cumulo+PL+interval_yield+RS+Feed_Vol+Cell_conc._in terval+AS_Vol.+emission_CO2+interval_O2; 0.430, 0.723, tmp+ tmp_cumulo+pH+pH_cumulo+PL+AN_cumulo+interval_yield+RS+Cell _conc._interval+AS_Vol.+emission_CO2+generate_CO2; 0.464, 0. 723, tmp_cumulo+pH+interval_yield+RS+Feed_Vol+Cell_conc._in terval+AS_Vol.+emission_CO2; 0.439, 0.723, tmp+tmp_cumulo+p H+pH_cumulo+AN+interval_yield+RS+Feed_Vol+RS_cumulo+Cell_co nc._interval+AS_Vol.; 0.430, 0.723, tmp_cumulo+pH+pH_cumulo +AN_cumulo+interval_yield+RS+Feed_Vol+Cell_conc._interval+A S_Vol.+emission_CO2+interval_O2+generate_CO2; 0.448, 0.723, tmp+tmp_cumulo+pH+pH_cumulo+AN_cumulo+interval_yield+RS+Fe ed_Vol+Cell_conc._interval+generate_CO2; 0.420, 0.723, tmp+ tmp_cumulo+pH+pH_cumulo+AN+AN_cumulo+interval_yield+RS+Feed _Vol+Cell_conc._interval+RQ_integral+AS_Vol.+generate_CO2; 0.439, 0.723, tmp_cumulo+pH+pH_cumulo+PL_cumulo+interval_yi eld+RS+Feed_Vol+Cell_conc._interval+rab_integral+interval_O 2+generate_CO2; 0.448, 0.723, tmp_cumulo+pH+pH_cumulo+PL_cu mulo+AN+interval_yield+OD+RS+Cell_conc._interval+AS_Vol.; 0. 420, 0.723, tmp_cumulo+pH+pH_cumulo+PL_cumulo+AN+interval_y ield+RS+Feed_Vol+Cell_conc._interval+rab_integral+AS_Vol.+i nterval_O2+generate_CO2; 0.430, 0.723, tmp+tmp_cumulo+pH+pH _cumulo+PL+AN_cumulo+interval_yield+RS+Cell_conc._interval+ rab_integral+AS_Vol.+generate_CO2; 0.472, 0.723, tmp+tmp_cu mulo+pH+interval_yield+RS+Feed_Vol+Cell_conc._interval; 0.4 20, 0.723, tmp_cumulo+pH+pH_cumulo+AN+AN_cumulo+interval_yi eld+RS+Cell_conc._interval+rab_integral+RQ_integral+AS_Vol. +emission_O2+emission_CO2; 0.430, 0.723, tmp_cumulo+pH+pH_c umulo+AN+interval_yield+RS+Feed_Vol+Cell_conc._interval+RQ_ integral+AS_Vol.+emission_O2+emission_CO2; 0.439, 0.723, tm p+tmp_cumulo+pH+pH_cumulo+AN_cumulo+interval_yield+OD+RS+Ce ll_conc._interval+AS_Vol.+emission_CO2; 0.448, 0.723, tmp_c umulo+pH+pH_cumulo+AN+interval_yield+RS+Cell_conc._interval +AS_Vol.+emission_CO2+consum_O2; 0.464, 0.723, tmp_cumulo+p H+AN_cumulo+interval_yield+RS+Feed_Vol+Cell_conc._interval+ emission_CO2; 0.420, 0.723, tmp+tmp_cumulo+pH+pH_cumulo+PL+ AN+AN_cumulo+interval_yield+RS+Cell_conc._interval+rab_inte gral+AS_Vol.+emission_CO2; 0.430, 0.723, tmp_cumulo+pH+pH_c umulo+PL_cumulo+AN_cumulo+interval_yield+RS+Feed_Vol+Cell_c onc._interval+rab_integral+AS_Vol.+interval_O2; 0.456, 0.72 3, tmp+tmp_cumulo+pH+pH_cumulo+AN+interval_yield+RS+Feed_Vo l+Cell_conc._interval; 0.447, 0.723, tmp_cumulo+pH+pH_cumul o+AN_cumulo+interval_yield+RS+Cell_conc._interval+RQ_integr al+AS_Vol.+emission_O2; 0.439, 0.723, tmp_cumulo+pH+pH_cumu lo+PL+AN_cumulo+interval_yield+interval_Pdt.+RS+Cell_conc._ interval+AS_Vol.+interval_O2; 0.456, 0.723, tmp_cumulo+pH+A N_cumulo+interval_yield+OD+RS+Feed_Vol+Cell_conc._interval+ AS_Vol.; 0.420, 0.723, tmp_cumulo+pH+pH_cumulo+PL_cumulo+AN +interval_yield+RS+Feed_Vol+Cell_conc._interval+AS_Vol.+con sum_O2+interval_O2+generate_CO2; 0.447, 0.723, tmp_cumulo+p H+pH_cumulo+AN+AN_cumulo+interval_yield+RS+Feed_Vol+Cell_co nc._interval+RQ_integral; 0.439, 0.723, tmp_cumulo+pH+pH_cu mulo+AN+AN_cumulo+interval_yield+RS+Cell_conc._interval+AS_ Vol.+emission_O2+generate_CO2; 0.430, 0.723, tmp+tmp_cumulo +pH+pH_cumulo+AN_cumulo+interval_yield+RS+Feed_Vol+Cell_con c._interval+RQ_integral+AS_Vol.+consum_O2 ; 0.430, 0.723, tm p_cumulo+pH+pH_cumulo+PL_cumulo+AN+AN_cumulo+interval_yield +RS+Feed_Vol+Cell_conc._interval+AS_Vol.+interval_O2; 0.439, 0.723, tmp_cumulo+pH+pH_cumulo+AN+interval_yield+RS+Feed_V ol+RS_cumulo+Cell_conc._interval+AS_Vol.+emission_CO2; 0.43 0, 0.723, tmp_cumulo+pH+pH_cumulo+AN+AN_cumulo+interval_yie ld+interval_Pdt.+RS+Feed_Vol+Cell_conc._interval+AS_Vol.+co nsum_O2; 0.439, 0.723, tmp+tmp_cumulo+pH+pH_cumulo+AN_cumul o+interval_yield+RS+Cell_conc._interval+AS_Vol.+emission_O2 +emission_CO2; 0.420, 0.723, tmp_cumulo+pH+pH_cumulo+PL_cum ulo+AN_cumulo+interval_yield+RS+Feed_Vol+Cell_conc._interva l+rab_integral+AS_Vol.+interval_O2+generate_CO2; 0.447, 0.7 23, tmp+tmp_cumulo+pH+pH_cumulo+AN_cumulo+interval_yield+RS +Feed_Vol+Cell_conc._interval+emission_CO2; 0.447, 0.723, t mp_cumulo+pH+pH_cumulo+interval_yield+RS+Feed_Vol+Cell_conc. _interval+rab_integral+RQ_integral+generate_CO2; 0.447, 0.7 23, tmp+tmp_cumulo+pH+pH_cumulo+interval_yield+RS+Feed_Vol+ Cell_conc._interval+rab_integral+AS_Vol.; 0.430, 0.723, tmp _cumulo+pH+pH_cumulo+PL+AN+interval_yield+interval_Pdt.+RS+ Feed_Vol+Cell_conc._interval+AS_Vol.+interval_O2; 0.429, 0. 722, tmp+tmp_cumulo+pH+pH_cumulo+AN+AN_cumulo+interval_yiel d+RS+Cell_conc._interval+rab_integral+AS_Vol.+emission_CO2; 0.447, 0.722, tmp_cumulo+pH+pH_cumulo+AN_cumulo+interval_y ield+RS+Cell_conc._interval+AS_Vol.+emission_O2+consum_O2; 0.429, 0.722, tmp_cumulo+pH+pH_cumulo+PL+AN_cumulo+interval _yield+RS+Feed_Vol+Cell_conc._interval+AS_Vol.+interval_O2+ generate_CO2; 0.420, 0.722, tmp_cumulo+pH+pH_cumulo+PL_cumu lo+AN_cumulo+interval_yield+RS+Feed_Vol+Cell_conc._interval +RQ_integral+AS_Vol.+emission_CO2+generate_CO2; 0.420, 0.72 2, tmp+tmp_cumulo+pH+pH_cumulo+PL+AN_cumulo+interval_yield+ RS+Feed_Vol+Cell_conc._interval+rab_integral+AS_Vol.+interv al_O2; 0.429, 0.722, tmp_cumulo+pH+pH_cumulo+PL_cumulo+AN_c umulo+interval_yield+RS+Feed_Vol+Cell_conc._interval+RQ_int egral+AS_Vol.+consum_O2; 0.438, 0.722, tmp_cumulo+pH+pH_cum ulo+AN+AN_cumulo+interval_yield+RS+Feed_Vol+Cell_conc._inte rval+rab_integral+generate_CO2; 0.429, 0.722, tmp_cumulo+pH +pH_cumulo+PL+AN_cumulo+interval_yield+interval_Pdt.+RS+Cel l_conc._interval+RQ_integral+AS_Vol.+emission_CO2; 0.447, 0. 722, tmp_cumulo+pH+pH_cumulo+PL_cumulo+AN+interval_yield+in terval_Pdt.+RS+Cell_conc._interval+AS_Vol.; 0.429, 0.722, t mp+tmp_cumulo+pH+pH_cumulo+PL+AN_cumulo+interval_yield+RS+C ell_conc._interval+rab_integral+AS_Vol.+interval_O2; 0.447, 0.722, tmp_cumulo+pH+pH_cumulo+PL_cumulo+AN+interval_yield +RS+Cell_conc._interval+AS_Vol.+emission_CO2; 0.438, 0.722, tmp_cumulo+pH+pH_cumulo+AN_cumulo+interval_yield+OD+RS+Fee d_Vol+RS_cumulo+Cell_conc._interval+AS_Vol.; 0.447, 0.722, tmp_cumulo+pH+pH_cumulo+AN+interval_yield+OD+RS+Cell_conc._ interval+RQ_integral+AS_Vol.; 0.429, 0.722, tmp_cumulo+pH+p H_cumulo+AN_cumulo+interval_yield+interval_Pdt.+RS+Feed_Vol +Cell_conc._interval+AS_Vol.+emission_O2+emission_CO2; 0.42 9, 0.722, tmp_cumulo+pH+pH_cumulo+PL+AN_cumulo+interval_yie ld+RS+Cell_conc._interval+rab_integral+AS_Vol.+consum_O2+in terval_O2; 0.456, 0.722, tmp+tmp_cumulo+pH+pH_cumulo+interv al_yield+RS+Feed_Vol+Cell_conc._interval+generate_CO2; 0.44 7, 0.722, tmp+tmp_cumulo+pH+pH_cumulo+AN_cumulo+interval_yi eld+interval_Pdt.+RS+Feed_Vol+Cell_conc._interval; 0.438, 0. 722, tmp+tmp_cumulo+pH+pH_cumulo+PL_cumulo+AN_cumulo+interv al_yield+interval_Pdt.+RS+Cell_conc._interval+AS_Vol.; 0.42 9, 0.722, tmp_cumulo+pH+pH_cumulo+AN_cumulo+interval_yield+ RS+Feed_Vol+Cell_conc._interval+RQ_integral+AS_Vol.+consum_ O2+generate_CO2; 0.420, 0.722, tmp_cumulo+pH+pH_cumulo+PL+A N+AN_cumulo+interval_yield+RS+Feed_Vol+Cell_conc._interval+ rab_integral+AS_Vol.+interval_O2; 0.438, 0.722, tmp_cumulo+ pH+pH_cumulo+PL+AN+AN_cumulo+interval_yield+RS+Cell_conc._i nterval+AS_Vol.+consum_O2; 0.429, 0.722, tmp_cumulo+pH+pH_c umulo+AN+AN_cumulo+interval_yield+RS+Feed_Vol+RS_cumulo+Cel l_conc._interval+RQ_integral+AS_Vol.; 0.429, 0.722, tmp_cum ulo+pH+pH_cumulo+PL_cumulo+AN+AN_cumulo+interval_yield+RS+F eed_Vol+RS_cumulo+Cell_conc._interval+AS_Vol.; 0.472, 0.722, pH+pH_cumulo+AN+interval_yield+RS+Cell_conc._interval+AS_V ol.; 0.447, 0.722, tmp_cumulo+pH+pH_cumulo+AN_cumulo+interv al_yield+RS+Feed_Vol+Cell_conc._interval+RQ_integral+consum _O2; 0.420, 0.722, tmp_cumulo+pH+pH_cumulo+PL+AN+interval_y ield+RS+Feed_Vol+Cell_conc._interval+AS_Vol.+emission_CO2+i nterval_O2+generate_CO2; 0.429, 0.722, tmp_cumulo+pH+pH_cum ulo+AN_cumulo+interval_yield+interval_Pdt.+RS+Feed_Vol+Cell _conc._interval+AS_Vol.+consum_O2+generate_CO2; 0.429, 0.72 2, tmp+tmp_cumulo+pH+pH_cumulo+AN+interval_yield+RS+Feed_Vo l+Cell_conc._interval+AS_Vol.+emission_CO2+generate_CO2; 0. 438, 0.722, tmp_cumulo+pH+pH_cumulo+AN+AN_cumulo+interval_y ield+interval_Pdt.+RS+Cell_conc._interval+RQ_integral+AS_Vo 1.; 0.447, 0.722, tmp_cumulo+pH+pH_cumulo+interval_yield+RS +Feed_Vol+Cell_conc._interval+rab_integral+AS_Vol.+consum_O 2; 0.429, 0.722, tmp_cumulo+pH+pH_cumulo+PL+AN_cumulo+inter val_yield+RS+Feed_Vol+Cell_conc._interval+rab_integral+AS_V ol.+emission_CO2; 0.429, 0.722, tmp_cumulo+pH+pH_cumulo+AN_ cumulo+interval_yield+OD+RS+Feed_Vol+Cell_conc._interval+ra b_integral+AS_Vol.+generate_CO2; 0.438, 0.722, tmp_cumulo+p H+pH_cumulo+AN+interval_yield+RS+Feed_Vol+Cell_conc._interv al+AS_Vol.+emission_O2+consum_O2; 0.429, 0.722, tmp_cumulo+ pH+pH_cumulo+PL+AN_cumulo+interval_yield+interval_Pdt.+RS+C ell_conc._interval+rab_integral+RQ_integral+AS_Vol.; 0.456, 0.722, tmp_cumulo+pH+pH_cumulo+interval_yield+interval_Pdt. +RS+Feed_Vol+Cell_conc._interval+RQ_integral; 0.447, 0.722, tmp_cumulo+pH+pH_cumulo+AN+interval_yield+RS+RS_cumulo+Cel l_conc._interval+AS_Vol.+emission_CO2; 0.438, 0.722, tmp_cu mulo+pH+pH_cumulo+PL+AN_cumulo+interval_yield+RS+Cell_conc. _interval+AS_Vol.+consum_O2+interval_O2; 0.438, 0.722, tmp+ tmp_cumulo+pH+pH_cumulo+AN+interval_yield+RS+Cell_conc._int erval+rab_integral+AS_Vol.+generate_CO2; 0.455, 0.722, tmp_ cumulo+pH+pH_cumulo+PL_cumulo+interval_yield+RS+Feed_Vol+Ce ll_conc._interval+rab_integral; 0.438, 0.722, tmp_cumulo+pH +pH_cumulo+AN+interval_yield+RS+Cell_conc._interval+AS_Vol. +emission_O2+emission_CO2 + generate_CO2; 0.429, 0.722, tmp_c umulo+pH+pH_cumulo+AN+interval_yield+RS+Feed_Vol+Cell_conc. _interval+rab_integral+AS_Vol.+emission_O2+generate_CO2; O. 447, 0.722, tmp_cumulo+pH+pH_cumulo+interval_yield+RS+Feed_ Vol+Cell_conc._interval+RQ_integral+AS_Vol.+generate_CO2; 0. 447, 0.722, tmp_cumulo+pH+pH_cumulo+AN+interval_yield+RS+Fe ed_Vol+Cell_conc._interval+interval_O2+generate_CO2; 0.438, 0.722, tmp_cumulo+pH+pH_cumulo+PL_cumulo+AN+interval_yield +RS+Cell_conc._interval+rab_integral+RQ_integral+AS_Vol.; 0. 438, 0.722, tmp_cumulo+pH+pH_cumulo+PL+interval_yield+RS+Fe ed_Vol+Cell_conc._interval+Accum._Yield+AS_Vol.+interval_O 2; 0.420, 0.722, tmp_cumulo+pH+pH_cumulo+AN+AN_cumulo+inter val_yield+RS+Feed_Vol+Cell_conc._interval+rab_integral+AS_V ol.+emission_O2+generate_CO2; 0.429, 0.722, tmp_cumulo+pH+p H_cumulo+AN_cumulo+interval_yield+interval_Pdt.+RS+Cell_con c._interval+RQ_integral+AS_Vol.+emission_O2+emission_CO2; 0. 464, 0.722, tmp_cumulo+pH+AN_cumulo+interval_yield+RS+Feed_ Vol+Cell_conc._interval+generate_CO2; 0.438, 0.722, tmp_cum ulo+pH+pH_cumulo+PL+AN_cumulo+interval_yield+interval_Pdt.+ RS+Cell_conc._interval+RQ_integral+AS_Vol.; 0.420, 0.722, t mp_cumulo+pH+pH_cumulo+PL+AN_cumulo+interval_yield+RS+Feed_ Vol+Cell_conc._interval+rab_integral+AS_Vol.+emission_CO2+i nterval_O2; 0.447, 0.722, tmp_cumulo+pH+pH_cumulo+interval_ yield+RS+Feed_Vol+Cell_conc._interval+rab_integral+emission _CO2+interval_O2; 0.420, 0.722, tmp_cumulo+pH+pH_cumulo+AN+ AN_cumulo+interval_yield+interval_Pdt.+RS+Feed_Vol+Cell_con c._interval+rab_integral+AS_Vol.+generate_CO2; 0.420, 0.722, tmp_cumulo+pH+pH_cumulo+PL+AN_cumulo+interval_yield+RS+Fee d_Vol+Cell_conc._interval+AS_Vol.+emission_CO2+interval_O2+ generate_CO2; 0.447, 0.722, tmp_cumulo+pH+pH_cumulo+AN_cumu lo+interval_yield+interval_Pdt.+RS+Cell_conc._interval+AS_V ol.+emission_O2; 0.447, 0.722, tmp_cumulo+pH+pH_cumulo+PL+i nterval_yield+RS+Cell_conc._interval+AS_Vol.+emission_CO2+i nterval_O2; 0.464, 0.722, tmp_cumulo+pH+interval_yield+RS+F eed_Vol+Cell_conc._interval+emission_CO2+interval_O2; 0.438, 0.722, tmp_cumulo+pH+pH_cumulo+PL_cumulo+AN_cumulo+interva l_yield+interval_Pdt.+RS+Cell_conc._interval+AS_Vol.+genera te_CO2; 0.447, 0.722, tmp_cumulo+pH+pH_cumulo+AN_cumulo+int erval_yield+interval_Pdt.+RS+Feed_Vol+Cell_conc._interval+c onsum O2; 0.420, 0.722, tmp_cumulo+pH+pH_cumulo+AN+AN_cumul o+interval_yield+interval_Pdt.+RS+Feed_Vol+Cell_conc._inter val+rab_integral+RQ_integral+AS_Vol.; 0.455, 0.722, tmp+tmp _cumulo+pH+pH_cumulo+interval_yield+RS+Cell_conc._interval+ rab_integral+AS_Vol.; 0.438, 0.722, tmp_cumulo+pH+pH_cumulo +AN+interval_yield+RS+Feed_Vol+Cell_conc._interval+RQ_integ ral+AS_Vol.+consum_O2; 0.447, 0.722, tmp_cumulo+pH+pH_cumul o+PL+PL_cumulo+AN_cumulo+interval_yield+RS+Cell_conc._inter val+AS_Vol.; 0.455, 0.722, tmp_cumulo+pH+pH_cumulo+interval _yield+interval_Pdt.+RS+Cell_conc._interval+rab_integral+AS _Vol.; 0.419, 0.722, tmp+tmp_cumulo+pH+pH_cumulo+AN_cumulo+ interval_yield+RS+Feed_Vol+Cell_conc._interval+AS_Vol.+emis sion_O2+emission_CO2+generate_CO2; 0.429, 0.722, tmp_cumulo +pH+pH_cumulo+PL_cumulo+AN+interval_yield+RS+Feed_Vol+Cell_ conc._interval+RQ_integral+AS_Vol.+generate_CO2; 0.419, 0.7 22, tmp_cumulo+pH+pH_cumulo+PL_cumulo+AN+AN_cumulo+interval _yield+interval_Pdt.+RS+Feed_Vol+Cell_conc._interval+AS_Vol. +generate_CO2; 0.447, 0.722, tmp+tmp_cumulo+pH+pH_cumulo+in terval_yield+RS+Feed_Vol+Cell_conc._interval+RQ_integral+AS _Vol.; 0.447, 0.722, tmp_cumulo+pH+pH_cumulo+interval_yield +RS+Feed_Vol+Cell_conc._interval+AS_Vol.+emission_O2+emissi on_CO2; 0.455, 0.722, tmp_cumulo+pH+pH_cumulo+AN_cumulo+int erval_yield+OD+RS+Feed_Vol+Cell_conc._interval; 0.429, 0.72 2, tmp_cumulo+pH+pH_cumulo+PL_cumulo+AN+interval_yield+RS+F eed_Vol+Cell_conc._interval+rab_integral+RQ_integral+AS_Vo 1.; 0.429, 0.722, tmp_cumulo+pH+pH_cumulo+AN_cumulo+interva l_yield+interval_Pdt.+RS+Cell_conc._interval+rab_integral+A S_Vol.+emission_O2+emission_CO2; 0.429, 0.722, tmp_cumulo+p H+pH_cumulo+PL+AN+interval_yield+RS+Cell_conc._interval+rab _integral+Accum._Yield+AS_Vol.+interval_O2; 0.429, 0.722, t mp+tmp_cumulo+pH+pH_cumulo+AN_cumulo+interval_yield+interva l_Pdt.+RS+Feed_Vol+Cell_conc._interval+AS_Vol.+emission_O2; 0.429, 0.722, tmp_cumulo+pH+pH_cumulo+PL+AN+interval_yield +RS+Cell_conc._interval+rab_integral+AS_Vol.+consum_O2+inte rval_O2; 0.438, 0.722, tmp_cumulo+pH+pH_cumulo+PL_cumulo+AN _cumulo+interval_yield+interval_Pdt.+RS+Cell_conc._interval +AS_Vol.+emission_CO2; 0.429, 0.722, tmp+tmp_cumulo+pH+pH_c umulo+AN+interval_yield+RS+Feed_Vol+Cell_conc._interval+RQ_ integral+AS_Vol.+generate_CO2; 0.455, 0.722, tmp_cumulo+pH+ interval_yield+RS+Feed_Vol+Cell_conc._interval+rab_integral +interval_O2+generate_CO2; 0.447, 0.722, tmp_cumulo+pH+pH_c umulo+AN_cumulo+interval_yield+RS+RS_cumulo+Cell_conc._inte rval+AS_Vol.+emission_O2; 0.447, 0.722, tmp_cumulo+pH+pH_cu mulo+PL+AN+interval_yield+RS+Cell_conc._interval+AS_Vol.+ge nerate_CO2; 0.419, 0.722, tmp+tmp_cumulo+pH+pH_cumulo+PL_cu mulo+AN_cumulo+interval_yield+RS+Feed_Vol+Cell_conc._interv al+rab_integral+RQ_integral+AS_Vol.; 0.455, 0.722, tmp_cumu lo+pH+PL_cumulo+AN_cumulo+interval_yield+RS+Feed_Vol+Cell_c onc._interval+AS_Vol.; 0.438, 0.722, tmp_cumulo+pH+pH_cumul o+AN+interval_yield+RS+Feed_Vol+RS_cumulo+Cell_conc._interv al+rab_integral+AS_Vol.; 0.447, 0.722, tmp_cumulo+pH+pH_cum ulo+interval_yield+interval_Pdt.+RS+Feed_Vol+Cell_conc._int erval+rab_integral+AS_Vol.; 0.438, 0.722, tmp_cumulo+pH+pH_ cumulo+AN_cumulo+interval_yield+OD+RS+Feed_Vol+Cell_conc._i nterval+AS_Vol.+consum_O2; 0.429, 0.722, tmp+tmp_cumulo+pH+ pH_cumulo+PL+AN_cumulo+interval_yield+RS+Feed_Vol+Cell_conc. _interval+rab_integral+AS_Vol.; 0.429, 0.722, tmp_cumulo+pH +pH_cumulo+PL+AN_cumulo+interval_yield+interval_Pdt.+RS+Fee d_Vol+Cell_conc._interval+rab_integral+AS_Vol.; 0.455, 0.72 2, tmp_cumulo+pH+pH_cumulo+interval_yield+RS+Feed_Vol+Cell_ conc._interval+emission_O2+emission_CO2; 0.419, 0.722, tmp_ cumulo+pH+pH_cumulo+AN+AN_cumulo+interval_yield+RS+Feed_Vol +Cell_conc._interval+RQ_integral+AS_Vol.+emission_CO2+gener ate_CO2; 0.446, 0.722, tmp+tmp_cumulo+pH+pH_cumulo+AN+AN_cu mulo+interval_yield+RS+Feed_Vol+Cell_conc._interval; 0.438, 0.722, tmp_cumulo+pH+pH_cumulo+PL+AN+interval_yield+RS+Cel l_conc._interval+rab_integral+RQ_integral+AS_Vol.; 0.455, 0. 722, tmp+tmp_cumulo+pH+pH_cumulo+interval_yield+RS+Feed_Vol +Cell_conc._interval+emission_O2; 0.419, 0.722, tmp_cumulo+ pH+pH_cumulo+PL+AN+AN_cumulo+interval_yield+interval_Pdt.+R S+Cell_conc._interval+rab_integral+AS_Vol.+emission_CO2; 0. 455, 0.722, tmp+pH+pH_cumulo+AN_cumulo+interval_yield+RS+Fe ed_Vol+Cell_conc._interval+AS_Vol.; 0.438, 0.722, tmp_cumul o+pH+pH_cumulo+PL+AN_cumulo+interval_yield+RS+Cell_conc._in terval+Accum._Yield+AS_Vol.+emission_CO2; 0.419, 0.722, tmp _cumulo+pH+pH_cumulo+PL_cumulo+AN+AN_cumulo+interval_yield+ interval_Pdt.+RS+Feed_Vol+Cell_conc._interval+rab_integral+ AS_Vol.; 0.463, 0.722, tmp_cumulo+pH+interval_yield+RS+Feed _Vol+Cell_conc._interval+AS_Vol.+interval_O2; 0.438, 0.722, tmp_cumulo+pH+pH_cumulo+interval_yield+RS+Feed_Vol+Cell_co nc._interval+rab_integral+AS_Vol.+emission_CO2+generate_CO 2; 0.446, 0.722, tmp+tmp_cumulo+pH+pH_cumulo+interval_yield +RS+Feed_Vol+Cell_conc._interval+rab_integral+consum_O2; 0. 429, 0.722, tmp_cumulo+pH+pH_cumulo+AN+interval_yield+inter val_Pdt.+RS+Feed_Vol+Cell_conc._interval+AS_Vol.+emission_C O2+generate_CO2; 0.446, 0.722, tmp_cumulo+pH+pH_cumulo+inte rval_yield+RS+Feed_Vol+Cell_conc._interval+rab_integral+RQ_ integral+emission_CO2; 0.438, 0.722, tmp+tmp_cumulo+pH+pH_c umulo+AN_cumulo+interval_yield+RS+Feed_Vol+Cell_conc._inter val+Accum._Yield+AS_Vol.; 0.428, 0.722, tmp_cumulo+pH+pH_cu mulo+AN_cumulo+interval_yield+OD+RS+Cell_conc._interval+rab _integral+AS_Vol.+emission_CO2+generate_CO2; 0.428, 0.722, tmp_cumulo+pH+pH_cumulo+PL_cumulo+AN_cumulo+interval_yield+ RS+Cell_conc._interval+rab_integral+RQ_integral+AS_Vol.+gen erate_CO2; 0.446, 0.722, tmp_cumulo+pH+pH_cumulo+AN+interva l_yield+RS+Feed_Vol+Cell_conc._interval+rab_integral+consum _O2; 0.455, 0.722, tmp_cumulo+pH+pH_cumulo+interval_yield+i nterval_Pdt.+RS+Feed_Vol+Cell_conc._interval+interval_O2; 0. 455, 0.722, tmp+pH+pH_cumulo+AN_cumulo+interval_yield+inter val_Pdt.+RS+Cell_conc._interval+AS_Vol.; 0.428, 0.722, tmp_ cumulo+pH+pH_cumulo+AN+AN_cumulo+interval_yield+interval_Pd t.+RS+Feed_Vol+RS_cumulo+Cell_conc._interval+AS_Vol.; 0.419, 0.722, tmp_cumulo+pH+pH_cumulo+PL+AN+interval_yield+RS+Cel l_conc._interval+rab_integral+Accum._Yield+AS_Vol.+emission _CO2+interval_O2; 0.428, 0.722, tmp_cumulo+pH+pH_cumulo+PL+ AN_cumulo+interval_yield+RS+Cell_conc._interval+RQ_integral +AS_Vol.+emission_CO2+consum_O2; 0.446, 0.722, tmp_cumulo+p H+pH_cumulo+AN+interval_yield+RS+Cell_conc._interval+RQ_int egral+AS_Vol.+consum_O2; 0.419, 0.722, tmp_cumulo+pH+pH_cum ulo+AN_cumulo+interval_yield+RS+Feed_Vol+Cell_conc._interva 1+rab_integral+AS_Vol.+emission_CO2+consum_O2+generate_CO2; 0.428, 0.722, tmp_cumulo+pH+pH_cumulo+PL_cumulo+AN+AN_cumu lo+interval_yield+RS+Cell_conc._interval+rab_integral+AS_Vo l.+consum_O2; 0.446, 0.722, tmp_cumulo+pH+pH_cumulo+PL+AN+i nterval_yield+RS+Cell_conc._interval+AS_Vol.+consum_O2; 0.4 28, 0.722, tmp_cumulo+pH+pH_cumulo+AN_cumulo+interval_yield +interval_Pdt.+RS+Feed_Vol+RS_cumulo+Cell_conc._interval+AS _Vol.+emission_CO2; 0.455, 0.722, tmp+tmp_cumulo+pH+pH_cumu lo+interval_yield+RS+Feed_Vol+Cell_conc._interval+RQ_integr al; 0.437, 0.722, tmp+tmp_cumulo+pH+pH_cumulo+AN+interval_y ield+RS+Cell_conc._interval+AS_Vol.+emission_CO2+generate_C 02; 0.437, 0.722, tmp_cumulo+pH+pH_cumulo+PL+AN_cumulo+inte rval_yield+RS+Cell_conc._interval+AS_Vol.+interval_02+gener ate_CO2; 0.455, 0.722, pH+pH_cumulo+PL_cumulo+AN_cumulo+int erval_yield+RS+Cell_conc._interval+AS_Vol.+generate_CO2; 0. 437, 0.722, tmp_cumulo+pH+pH_cumulo+AN+AN_cumulo+interval_y ield+OD+RS+Cell_conc._interval+RQ_integral+AS_Vol.; 0.437, 0.722, tmp_cumulo+pH+pH_cumulo+AN_cumulo+interval_yield+OD+ RS+Cell_conc._interval+rab_integral+RQ_integral+AS_Vol.; 0. 428, 0.722, tmp_cumulo+pH+pH_cumulo+PL+AN+AN_cumulo+interva l_yield+RS+Feed_Vol+Cell_conc._interval+rab_integral+AS_Vo 1.; 0.463, 0.722, tmp_cumulo+pH+AN+AN_cumulo+interval_yield +RS+Feed_Vol+Cell_conc._interval; 0.428, 0.722, tmp_cumulo+ pH+pH_cumulo+PL_cumulo+AN_cumulo+interval_yield+RS+Feed_Vol +Cell_conc._interval+rab_integral+AS_Vol.+emission_O2; 0.44 6, 0.722, tmp_cumulo+pH+pH_cumulo+interval_yield+RS+Feed_Vo l+Cell_conc._interval+RQ_integral+AS_Vol.+emission_CO2; 0.4 28, 0.722, mp+tmp_cumulo+pH+pH_cumulo+PL_cumulo+AN_cumulo+ interval_yield+RS+Feed_Vol+RS_cumulo+Cell_conc._interval+AS _Vol.; 0.419, 0.722, tmp_cumulo+pH+pH_cumulo+PL_cumulo+AN_c umulo+interval_yield+interval_Pdt.+RS+Feed_Vol+Cell_conc._i nterval+rab_integral+RQ_integral+AS_Vol.; 0.446, 0.722, tmp +tmp_cumulo+pH+pH_cumulo+AN_cumulo+interval_yield+RS+Feed_V ol+Cell_conc._interval+consum_O2; 0.437, 0.722, tmp+tmp_cum ulo+pH+pH_cumulo+AN_cumulo+interval_yield+RS+Cell_conc._int erval+RQ_integral+AS_Vol.+generate_CO2; 0.437, 0.722, tmp_c umulo+pH+pH_cumulo+AN+interval_yield+interval_Pdt.+RS+Feed_ Vol+Cell_conc._interval+AS_Vol.+consum_O2; 0.428, 0.722, tm p_cumulo+pH+pH_cumulo+AN_cumulo+interval_yield+interval_Pdt. +RS+Feed_Vol+Cell_conc._interval+AS_Vol.+emission_CO2+consu m_O2; 0.455, 0.722, pH+pH_cumulo+PL_cumulo+AN_cumulo+interv al_yield+RS+RS_cumulo+Cell_conc._interval+AS_Vol.; 0.446, 0. 722, tmp_cumulo+pH+pH_cumulo+AN+interval_yield+interval_Pdt. +RS+Cell_conc._interval+RQ_integral+AS_Vol.; 0.437, 0.722, tmp_cumulo+pH+pH_cumulo+AN+interval_yield+RS+Feed_Vol+RS_cu mulo+Cell_conc._interval+RQ_integral+AS_Vol.; 0.455, 0.722, tmp_cumulo+pH+pH_cumulo+interval_yield+RS+Feed_Vol+Cell_co nc._interval+emission_O2+consum_O2; 0.428, 0.722, tmp+tmp_c umulo+pH+pH_cumulo+PL_cumulo+AN_cumulo+interval_yield+RS+Fe ed_Vol+Cell_conc._interval+AS_Vol.+consum_O2; 0.428, 0.722, tmp_cumulo+pH+pH_cumulo+AN+interval_yield+interval_Pdt.+RS +Feed_Vol+Cell_conc._interval+rab_integral+AS_Vol.+generate _CO2; 0.437, 0.722, tmp+tmp_cumulo+pH+pH_cumulo+PL_cumulo+A N_cumulo+interval_yield+RS+Cell_conc._interval+RQ_integral+ AS_Vol.; 0.463, 0.722, tmp_cumulo+pH+pH_cumulo+PL_cumulo+in terval_yield+RS+Cell_conc._interval+AS_Vol.; 0.419, 0.722, tmp_cumulo+pH+pH_cumulo+AN_cumulo+interval_yield+RS+Feed_Vo l+RS_cumulo+Cell_conc._interval+rab_integral+AS_Vol.+emissi on_CO2+generate_CO2; 0.446, 0.722, tmp_cumulo+pH+pH_cumulo+ AN+interval_yield+RS+Cell_conc._interval+RQ_integral+AS_Vol. +emission_O2; 0.428, 0.722, tmp+tmp_cumulo+pH+pH_cumulo+AN+ interval_yield+RS+Feed_Vol+Cell_conc._interval+rab_integral +RQ_integral+AS_Vol.; 0.428, 0.722, tmp+tmp_cumulo+pH+pH_cu mulo+AN_cumulo+interval_yield+OD+RS+Feed_Vol+Cell_conc._int erval+AS_Vol.+generate_CO2; 0.455, 0.722, tmp_cumulo+pH+pH_ cumulo+AN_cumulo+interval_yield+RS+Feed_Vol+RS_cumulo+Cell_ conc._interval; 0.428, 0.722, tmp_cumulo+pH+pH_cumulo+PL_cu mulo+AN+AN_cumulo+interval_yield+RS+Feed_Vol+Cell_conc._int erval+AS_Vol.+emission_O2; 0.428, 0.722, tmp+tmp_cumulo+pH+ pH_cumulo+AN_cumulo+interval_yield+interval_Pdt.+RS+Feed_Vo l+Cell_conc._interval+AS_Vol.+consum_O2; 0.437, 0.722, tmp_ cumulo+pH+pH_cumulo+PL+AN_cumulo+interval_yield+RS+Cell_con c._interval+rab_integral+Accum._Yield+AS_Vol.; 0.437, 0.722, tmp_cumulo+pH+pH_cumulo+PL_cumulo+AN+interval_yield+RS+Fee d_Vol+Cell_conc._interval+AS_Vol.+consum_O2 [0130] [206. Linear Model that Predicts Arginine Product ion Amount in Interval 6] 0.394, 0.678, tmp_cumulo+pH_cumulo+interval_yield+OD+RQ_int egral+AS_Vol.+consum_O2+generate_CO2; 0.384, 0.677, tmp_cum ulo+pH_cumulo+interval_yield+OD+RQ_integral+AS_Vol.+consum_ O2+interval_O2+generate_CO2; 0.379, 0.674, tmp_cumulo+pH_cu mulo+interval_yield+OD+rab_integral+RQ_integral+AS_Vol.+con sum_O2+generate_CO2; 0.379, 0.674, tmp_cumulo+pH_cumulo+int erval_yield+interval_Pdt.+OD+RQ_integral+AS_Vol.+consum_O2+ generate_CO2; 0.368, 0.674, tmp_cumulo+pH_cumulo+interval_y ield+OD+rab_integral+RQ_integral+AS_Vol.+consum_O2+interval _O2+generate CO2; 0.367, 0.673, tmp_cumulo+pH_cumulo+interv al_yield+interval_Pdt.+OD+RQ_integral+AS_Vol.+consum_O2+int erval_O2+generate_CO2; 0.386, 0.673, tmp_cumulo+interval_yi eld+interval_Pdt.+RS_cumulo+RQ_integral+emission_CO2+consum _O2+generate CO2; 0.366, 0.672, tmp_cumulo+pH_cumulo+interv al_yield+interval_Pdt.+OD+RQ_integral+AS_Vol.+emission_CO2+ consum_O2+generate_CO2; 0.375, 0.672, tmp_cumulo+interval_y ield+interval_Pdt.+RS_cumulo+RQ_integral+AS_Vol.+emission_C O2+consum_O2+generate_CO2; 0.374, 0.671, tmp_cumulo+pH_cumu lo+interval_yield+interval_Pdt.+RQ_integral+AS_Vol.+consum_ O2+interval_O2+generate_CO2; 0.374, 0.671, tmp_cumulo+pH_cu mulo+interval_yield+OD+RQ_integral+AS_Vol.+emission_CO2+con sum_O2+generate_CO2; 0.373, 0.670, tmp_cumulo+interval_yiel d+interval_Pdt.+RS_cumulo+rab_integral+RQ_integral+emission _CO2+consum_O2+generate_CO2; 0.373, 0.670, tmp_cumulo+pH_cu mulo+interval_yield+OD+RS_cumulo+RQ_integral+AS_Vol.+consum _O2+generate CO2; 0.373, 0.670, tmp_cumulo+pH_cumulo+AN+int erval_yield+OD+RQ_integral+AS_Vol.+consum_O2+generate_CO2; 0.363, 0.670, tmp_cumulo+pH_cumulo+AN+interval_yield+OD+RQ_ integral+AS_Vol.+consum_O2+interval_O2+generate_CO2; 0.352, 0.670, tmp_cumulo+pH_cumulo+interval_yield+interval_Pdt.+O D+rab_integral+RQ_integral+AS_Vol.+emission_CO2+consum_O2+g enerate_CO2; 0.362, 0.670, tmp_cumulo+pH_cumulo+interval_yi eld+interval_Pdt.+OD+rab_integral+RQ_integral+AS_Vol.+consu m_O2+generate_CO2; 0.372, 0.669, tmp_cumulo+pH_cumulo+inter val_yield+OD+Feed_Vol+RQ_integral+AS_Vol.+consum_O2+generat e_CO2; 0.362, 0.669, tmp_cumulo+pH_cumulo+interval_yield+OD +Feed_Vol+RQ_integral+AS_Vol.+consum_O2+interval_O2+generat e_CO2; 0.371, 0.669, tmp_cumulo+pH_cumulo+interval_yield+OD +RQ_integral+AS_Vol.+emission_O2+consum _O2+generate_CO2; 0. 361, 0.669, tmp_cumulo+pH_cumulo+interval_yield+OD+rab_inte gral+RQ_integral+AS_Vol.+emission_CO2+consum_O2+generate_CO 2; 0.371, 0.669, tmp_cumulo+pH_cumulo+interval_yield+OD+Cel l_conc._interval+RQ_integral+AS_Vol.+consum_O2+generate_CO 2; 0.390, 0.669, tmp_cumulo+interval_yield+interval_Pdt.+RS _cumulo+RQ_integral+consum_O2+generate_CO2; 0.371, 0.669, t mp_cumulo+pH_cumulo+AN_cumulo+interval_yield+OD+RQ_integral +AS_Vol.+consum_O2+generate_CO2; 0.361, 0.669, tmp_cumulo+p H_cumulo+AN_cumulo+interval_yield+OD+RQ_integral+AS_Vol.+co nsum_O2+interval_O2+generate_CO2; 0.371, 0.669, tmp_cumulo+ interval_yield+interval_Pdt.+OD+RS_cumulo+RQ_integral+emiss ion_CO2+consum_O2+generate_CO2; 0.380, 0.668, tmp_cumulo+pH _cumulo+interval_yield+OD+RS_cumulo+RQ_integral+consum_O2+g enerate_CO2; 0.360, 0.668, tmp_cumulo+pH_cumulo+interval_yi eld+OD+RQ_integral+AS_Vol.+emission_O2+consum_O2+interval_O 2+generate_CO2; 0.370, 0.668, tmp_cumulo+pH+pH_cumulo+inter val_yield+OD+RQ_integral+AS_Vol.+consum_O2+generate_CO2; 0. 360, 0.668, tmp_cumulo+interval_yield+interval_Pdt.+RS_cumu lo+rab_integral+RQ_integral+AS_Vol.+emission_CO2+consum_O2+ generate_CO2; 0.360, 0.668, tmp_cumulo+pH_cumulo+interval_y ield+OD+RQ_integral+AS_Vol.+emission_CO2+consum_O2+interval _O2+generate CO2; 0.379, 0.668, tmp+interval_yield+interval _Pdt.+RS_cumulo+RQ_integral+emission_CO2+consum_O2+generate _CO2; 0.370, 0.668, tmp_cumulo+pH_cumulo+PL_cumulo+interval _yield+OD+RQ_integral+AS_Vol.+consum_O2+generate_CO2; 0.360, 0.668, tmp_cumulo+pH+pH_cumulo+interval_yield+OD+RQ_integr al+AS_Vol.+consum_O2+interval_O2+generate_CO2; 0.360, 0.668, tmp_cumulo+pH_cumulo+interval_yield+OD+RS_cumulo+RQ_integr al+AS_Vol.+consum_O2+interval_O2+generate_CO2; 0.379, 0.668, tmp+interval_yield+interval_Pdt.+RQ_integral+AS_Vol.+emiss ion_CO2+consum_O2+generate_CO2; 0.349, 0.668, tmp_cumulo+pH _cumulo+interval_yield+interval_Pdt.+OD+Feed_Vol+RQ_integra l+AS_Vol.+consum_O2+interval_O2+generate_CO2; 0.359, 0.668, tmp_cumulo+pH_cumulo+interval_yield+interval_Pdt.+OD+Feed_ Vol+RQ_integral+AS_Vol.+consum_O2+generate_CO2; 0.369, 0.66 8, tmp_cumulo+pH_cumulo+interval_yield+interval_Pdt.+RQ_int egral+AS_Vol.+emission_CO2+consum_O2+generate_CO2; 0.359, 0. 668, tmp_cumulo+pH_cumulo+interval_yield+OD+Cell_conc._inte rval+RQ_integral+AS_Vol.+consum_O2+interval_O2+generate_CO 2; 0.359, 0.668, tmp_cumulo+pH_cumulo+interval_yield+interv al_Pdt.+OD+RQ_integral+AS_Vol.+emission_O2+consum_O2+genera te_CO2; 0.359, 0.667, tmp_cumulo+pH_cumulo+interval_yield+O D+Feed_Vol+rab_integral+RQ_integral+AS_Vol.+consum_O2+gener ate_CO2; 0.369, 0.667, tmp_cumulo+pH_cumulo+interval_yield+ interval_Pdt.+OD+RS_cumulo+RQ_integral+consum_O2+generate_C 02; 0.349, 0.667, tmp_cumulo+pH_cumulo+interval_yield+OD+Fe ed_Vol+rab_integral+RQ_integral+AS_Vol.+consum_O2+interval_ O2+generate_CO2; 0.348, 0.667, tmp_cumulo+pH_cumulo+interva l_yield+interval_Pdt.+OD+RQ_integral+AS_Vol.+emission_CO2+c onsum_O2+interval_O2+generate_CO2; 0.358, 0.667, tmp_cumulo +pH_cumulo+interval_yield+interval_Pdt.+OD+RS_cumulo+RQ_int egral+AS_Vol.+consum_O2+generate_CO2; 0.368, 0.667, tmp+tmp _cumulo+pH_cumulo+interval_yield+OD+RQ_integral+AS_Vol.+con sum_O2+generate_CO2; 0.358, 0.667, tmp_cumulo+pH_cumulo+PL_ cumulo+interval_yield+OD+RQ_integral+AS_Vol.+consum_O2+inte rval_O2+generate_CO2; 0.368, 0.667, tmp_cumulo+interval_yie ld+interval_Pdt.+RS_cumulo+RQ_integral+emission_O2+emission _CO2+consum_O2+generate_CO2; 0.347, 0.667, tmp_cumulo+pH_cu mulo+interval_yield+interval_Pdt.+OD+rab_integral+RQ_integr al+AS_Vol.+consum_O2+interval_O2+generate_CO2; 0.357, 0.667, tmp_cumulo+pH_cumulo+AN+interval_yield+OD+rab_integral+RQ_ integral+AS_Vol.+consum_O2+generate_CO2; 0.357, 0.666, tmp_ cumulo+pH_cumulo+interval_yield+interval_Pdt.+RQ_integral+A S_Vol.+emission_CO2+consum_O2+interval_O2+generate_CO2; 0.3 47, 0.666, tmp_cumulo+pH_cumulo+interval_yield+OD+rab_integ ral+RQ_integral+AS_Vol.+emission_C02+consum_02+interval_02+ generate_CO2; 0.357, 0.666, tmp_cumulo+pH_cumulo+interval_y ield+interval_Pdt.+rab_integral+RQ_integral+AS_Vol.+emissio n_CO2+consum_O2+generate_CO2; 0.357, 0.666, tmp_cumulo+inte rval_yield+interval_Pdt.+OD+RS_cumulo+RQ_integral+AS_Vol.+e mission_CO2+consum_O2+generate_CO2; 0.357, 0.666, tmp_cumul o+pH_cumulo+AN+interval_yield+interval_Pdt.+OD+RQ_integral+ AS_Vol.+consum_O2+generate_CO2; 0.376, 0.666, tmp_cumulo+pH _cumulo+interval_yield+interval_Pdt.+RQ_integral+AS_Vol.+co nsum_O2+generate_CO2; 0.357, 0.666, tmp_cumulo+pH_cumulo+in terval_yield+OD+RS_cumulo+rab_integral+RQ_integral+AS_Vol.+ consum_O2+generate_CO2; 0.347, 0.666, tmp_cumulo+pH_cumulo+ AN+interval_yield+OD+rab_integral+RQ_integral+AS_Vol.+consu m_O2+interval_O2+generate_CO2; 0.357, 0.666, tmp_cumulo+int erval_yield+interval_Pdt.+OD+RS_cumulo+rab_integral+RQ_inte gral+emission_CO2+consum_O2+generate_CO2; 0.357, 0.666, tmp +pH_cumulo+interval_yield+interval_Pdt.+OD+RQ_integral+AS_V ol.+emission_CO2+consum_O2+generate_CO2; 0.357, 0.666, tmp_ cumulo+pH_cumulo+interval_yield+OD+rab_integral+RQ_integral +AS_Vol.+emission_O2+consum_O2+generate_CO2; 0.346, 0.666, tmp_cumulo+pH_cumulo+interval_yield+interval_Pdt.+OD+RS_cum ulo+RQ_integral+AS_Vol.+emission_CO2+consum_O2+generate_CO 2; 0.346, 0.666, tmp+pH_cumulo+interval_yield+interval_Pdt. +OD+Feed_Vol+RQ_integral+AS_Vol.+consum_O2+interval_O2+gene rate_CO2; 0.356, 0.666, tmp+pH_cumulo+interval_yield+interv al_Pdt.+OD+Feed_Vol+RQ_integral+AS_Vol.+consum_O2+generate_ CO2; 0.356, 0.666, tmp_cumulo+pH_cumulo+AN_cumulo+interval_ yield+interval_Pdt.+OD+RQ_integral+AS_Vol.+consum_O2+genera te_CO2; 0.356, 0.666, tmp_cumulo+pH_cumulo+AN_cumulo+interv al_yield+OD+rab_integral+RQ_integral+AS_Vol.+consum_O2+gene rate_CO2; 0.356, 0.666, tmp_cumulo+pH_cumulo+interval_yield +interval_Pdt.+RS_cumulo+RQ_integral+AS_Vol.+emission_CO2+c onsum_O2+generate_CO2; 0.366, 0.665, tmp+pH_cumulo+interval _yield+interval_Pdt.+RQ_integral+AS_Vol.+emission_CO2+consu m_O2+generate_CO2; 0.356, 0.665, tmp+tmp_cumulo+pH_cumulo+i nterval_yield+OD+RQ_integral+AS_Vol.+consum_O2+interval_O2+ generate_CO2; 0.366, 0.665, tmp_cumulo+AN+interval_yield+in terval_Pdt.+RS_cumulo+RQ_integral+emission_CO2+consum_O2+ge nerate_CO2; 0.345, 0.665, tmp_cumulo+pH_cumulo+AN_cumulo+in terval_yield+OD+rab_integral+RQ_integral+AS_Vol.+consum_O2+ interval_O2+generate_CO2; 0.355, 0.665, tmp_cumulo+interval _yield+interval_Pdt.+RS_cumulo+rab_integral+RQ_integral+emi ssion_O2+emission_CO2+consum_O2+generate_CO2; 0.355, 0.665, tmp_cumulo+pH+pH_cumulo+interval_yield+OD+rab_integral+RQ_ integral+AS_Vol.+consum_O2+generate_CO2; 0.375, 0.665, tmp_ cumulo+interval_yield+interval_Pdt.+RQ_integral+AS_Vol.+emi ssion_CO2+consum_O2+generate_CO2; 0.355, 0.665, tmp_cumulo+ pH_cumulo+interval_yield+interval_Pdt.+OD+Cell_conc._interv al+RQ_integral+AS_Vol.+consum_O2+generate_CO2; 0.365, 0.665, tmp_cumulo+pH_cumulo+interval_yield+OD+RS_cumulo+rab_integ ral+RQ_integral+consum_O2+generate_CO2; 0.345, 0.665, tmp_c umulo+pH_cumulo+interval_yield+interval_Pdt.+OD+RQ_integral +AS_Vol.+emission_O2+consum_O2+interval_O2+generate_CO2; 0. 345, 0.665, tmp_cumulo+pH_cumulo+AN+interval_yield+interval Pdt.+OD+RQ_integral+AS_Vol.+consum_O2+interval_O2+generate _CO2; 0.365, 0.665, tmp+interval_yield+interval_Pdt.+RS_cum ulo+RQ_integral+AS_Vol.+emission_CO2+consum_O2+generate_CO 2; 0.365, 0.665, tmp+interval_yield+interval_Pdt.+RS_cumulo +rab_integral+RQ_integral+emission_CO2+consum_O2+generate_C 02; 0.345, 0.665, tmp_cumulo+pH_cumulo+interval_yield+OD+ra b_integral+RQ_integral+AS_Vol.+emission_O2+consum_O2+interv al_O2+generate_CO2; 0.355, 0.665, tmp_cumulo+pH_cumulo+inte rval_yield+OD+Cell_conc._interval+rab_integral+RQ_integral+ AS_Vol.+consum_O2+generate_CO2; 0.355, 0.665, tmp_cumulo+in terval_yield+interval_Pdt.+RS_cumulo+RQ_integral+AS_Vol.+em ission_O2+emission_CO2+consum_O2+generate_CO2; 0.344, 0.665, tmp_cumulo+pH+pH_cumulo+interval_yield+OD+rab_integral+RQ_ integral+AS_Vol.+consum_O2+interval_O2+generate_CO2; 0.374, 0.665, tmp_cumulo+interval_yield+interval_Pdt.+OD+RS_cumul o+RQ_integral+consum_O2+generate_CO2; 0.374, 0.664, tmp_cum ulo+interval_yield+interval_Pdt.+RS_cumulo+RQ_integral+AS_V ol.+consum_O2+generate_CO2; 0.344, 0.664, tmp_cumulo+pH_cum ulo+interval_yield+interval_Pdt.+OD+RQ_integral+AS_Vol.+emi ssion_O2+emission_CO2+consum_O2+generate_CO2; 0.383, 0.664, tmp_cumulo+pH_cumulo+interval_yield+OD+RQ_integral+consum_ O2+generate_CO2; 0.344, 0.664, tmp_cumulo+pH_cumulo+interva l_yield+interval_Pdt.+OD+Feed_Vol+rab_integral+RQ_integral+ AS_Vol.+consum_O2+generate_CO2; 0.354, 0.664, tmp_cumulo+pH _cumulo+PL_cumulo+interval_yield+interval_Pdt.+OD+RQ_integr al+AS_Vol.+consum_O2+generate_CO2; 0.354, 0.664, tmp_cumulo +pH_cumulo+interval_yield+interval_Pdt.+OD+RS_cumulo+RQ_int egral+emission_CO2+consum_O2+generate_CO2; 0.354, 0.664, tm p_cumulo+pH_cumulo+AN+interval_yield+interval_Pdt.+RQ_integ ral+AS_Vol.+consum_O2+interval_O2+generate_CO2; 0.354, 0.66 4, tmp_cumulo+pH_cumulo+interval_yield+interval_Pdt.+RQ_int egral+AS_Vol.+emission_O2+consum_O2+interval_O2+generate_CO 2; 0.364, 0.664, tmp_cumulo+AN_cumulo+interval_yield+interv al_Pdt.+RS_cumulo+RQ_integral+emission_CO2+consum_O2+genera te_CO2; 0.364, 0.664, tmp_cumulo+pH_cumulo+PL+interval_yiel d+OD+RQ_integral+AS_Vol.+consum_O2+generate_CO2; 0.373, 0.6 64, tmp_cumulo+interval_yield+interval_Pdt.+RS_cumulo+RQ_in tegral+emission_O2+consum_O2+generate_CO2; 0.353, 0.664, tm p_cumulo+pH_cumulo+PL_cumulo+interval_yield+OD+rab_integral +RQ_integral+AS_Vol.+consum_O2+generate_CO2; 0.353, 0.664, tmp+tmp_cumulo+pH_cumulo+interval_yield+interval_Pdt.+OD+RQ _integral+AS_Vol.+consum_O2+generate_CO2; 0.332, 0.664, tmp _cumulo+pH_cumulo+interval_yield+interval_Pdt.+OD+rab_integ ral+RQ_integral+AS_Vol.+emission_CO2+consum_O2+interval_O2+ generate_CO2; 0.363, 0.664, tmp+interval_yield+interval_Pdt. +OD+RS_cumulo+RQ_integral+emission_CO2+consum_O2+generate_C 02; 0.373, 0.664, tmp+pH_cumulo+interval_yield+interval_Pdt. +OD+RQ_integral+consum_O2+generate_CO2; 0.353, 0.664, tmp_c umulo+pH_cumulo+interval_yield+interval_Pdt.+Feed_Vol+RQ_in tegral+AS_Vol.+consum_O2+interval_O2+generate_CO2; 0.343, 0. 664, tmp_cumulo+pH_cumulo+AN_cumulo+interval_yield+interval _Pdt.+OD+RQ_ntegral+AS_Vol.+consum_O2+interval_O2+generate _CO2; 0.343, 0.664, tmp_cumulo+pH_cumulo+AN+interval_yield+ interval_Pdt.+OD+RQ_integral+AS_Vol.+emission_CO2+consum_O2 +generate_CO2; 0.353, 0.664, tmp_cumulo+pH_cumulo+PL+interv al_yield+OD+RQ_integral+AS_Vol.+consum_O2+interval_O2+gener ate_CO2; 0.363, 0.664, tmp+pH_cumulo+interval_yield+interva l_Pdt.+OD+RQ_integral+AS_Vol.+consum_O2+generate_CO2; 0.353, 0.664, tmp_cumulo+pH_cumulo+interval_yield+interval_Pdt.+r ab_integral+RQ_integral+AS_Vol.+consum_O2+interval_O2+gener ate_CO2; 0.332, 0.663, tmp_cumulo+pH_cumulo+interval_yield+ interval_Pdt.+OD+Feed_Vol+rab_integral+RQ_integral+AS_Vol.+ consum_O2+interval_O2+generate_CO2; 0.372, 0.663, tmp_cumul o+pH_cumulo+interval_yield+interval_Pdt.+OD+RQ_integral+con sum_O2+generate_CO2; 0.342, 0.663, tmp_cumulo+pH_cumulo+int erval_yield+OD+RS_cumulo+rab_integral+RQ_integral+AS_Vol.+c onsum_O2+interval_O2+generate_CO2; 0.363, 0.663, tmp+interv al_yield+interval_Pdt.+RS_cumulo+RQ_integral+emission_O2+em ission_CO2+consum_O2+generate_CO2; 0.353, 0.663, tmp_cumulo +interval_yield+interval_Pdt.+Feed_Vol+RS_cumulo+RQ_integra l+AS_Vol.+emission_CO2+consum_O2+generate_CO2; 0.362, 0.663, tmp+interval_yield+interval_Pdt.+rab_integral+RQ_integral+ AS_Vol.+emission_CO2+consum_O2+generate_CO2; 0.352, 0.663, tmp_cumulo+pH_cumulo+interval_yield+OD+RS_cumulo+RQ_integra l+AS_Vol.+emission_CO2+consum_O2+generate_CO2; 0.342, 0.663, tmp_cumulo+pH_cumulo+interval_yield+interval_Pdt.+OD+Feed_ Vol+RQ_integral+AS_Vol.+emission_CO2+consum_O2+generate_CO 2; 0.352, 0.663, tmp_cumulo+pH+pH_cumulo+interval_yield+int erval_Pdt.+OD+RQ_integral+AS_Vol.+consum_O2+generate_CO2; 0. 362, 0.663, tmp_cumulo+PL_cumulo+interval_yield+interval_Pd t.+RS_cumulo+RQ_integral+emission_CO2+consum_O2+generate_CO 2; 0.342, 0.663, tmp_cumulo+pH_cumulo+interval_yield+interv al_Pdt.+rab_integral+RQ_integral+AS_Vol.+emission_CO2+consu m_O2+interval_O2+generate_CO2; 0.352, 0.663, tmp_cumulo+int erval_yield+interval_Pdt.+OD+RS_cumulo+RQ_integral+emission _O2+emission_CO2+consum_O2+generate_CO2; 0.342, 0.663, tmp_ cumulo+pH_cumulo+AN_cumulo+interval_yield+interval_Pdt.+OD+ RQ_integral+AS_Vol.+emission_CO2+consum_O2+generate_CO2; 0. 342, 0.663, tmp_cumulo+pH_cumulo+AN+interval_yield+OD+Feed_ Vol+RQ_integral+AS_Vol.+consum_O2+interval_O2+generate_CO2; 0.372, 0.663, tmp_cumulo+interval_yield+interval_Pdt.+RS_c umulo+rab_integral+RQ_integral+consum_O2+generate_CO2; 0.35 2, 0.663, tmp_cumulo+pH_cumulo+interval_yield+interval_Pdt. +OD+RS_cumulo+rab_integral+RQ_integral+consum_O2+generate_C 02; 0.342, 0.663, tmp_cumulo+pH_cumulo+interval_yield+OD+Ce ll_conc._interval+rab_integral+RQ_integral+AS_Vol.+consum_O 2+interval_O2+generate_CO2; 0.331, 0.663, tmp_cumulo+pH_cum ulo+interval_yield+interval_Pdt.+OD+RS_cumulo+rab_integral+ RQ_integral+AS_Vol.+emission_CO2+consum_O2+generate_CO2; 0. 362, 0.663, tmp_cumulo+pH+interval_yield+interval_Pdt.+RS_c umulo+RQ_integral+emission_CO2+consum_O2+generate_CO2; 0.34 2, 0.663, tmp_cumulo+pH_cumulo+interval_yield+interval_Pdt. +RS_cumulo+rab_integral+RQ_integral+AS_Vol.+emission_CO2+co nsum_O2+generate_CO2; 0.352, 0.663, tmp+tmp_cumulo+pH_cumul o+interval_yield+OD+rab_integral+RQ_integral+AS_Vol.+consum _O2+generate_CO2; 0.342, 0.663, tmp_cumulo+pH_cumulo+interv al_yield+interval_Pdt.+OD+rab_integral+RQ_integral+AS_Vol.+ emission_O2+consum_O2+generate_CO2; 0.352, 0.663, tmp_cumul o+AN+interval_yield+interval_Pdt.+RS_cumulo+rab_integral+RQ _integral+emission_CO2+consum_O2+generate_CO2; 0.331, 0.663, tmp_cumulo+pH_cumulo+interval_yield+interval_Pdt.+OD+rab_i ntegral+RQ_integral+AS_Vol.+emission_O2+emission_CO2+consum _O2+generate_CO2; 0.362, 0.663, tmp_cumulo+pH_cumulo+interv al_yield+interval_Pdt.+RS_cumulo+RQ_integral+AS_Vol.+consum _O2+generate_CO2; 0.362, 0.663, tmp_cumulo+interval_yield+i nterval_Pdt.+Feed_Vol+RS_cumulo+RQ_integral+emission_CO2+co nsum_O2+generate_CO2; 0.352, 0.663, tmp_cumulo+pH+interval_ yield+interval_Pdt.+RS_cumulo+RQ_integral+AS_Vol.+emission_ CO2+consum_O2+generate_CO2; 0.362, 0.663, tmp+tmp_cumulo+in terval_yield+interval_Pdt.+RS_cumulo+RQ_integral+emission_C O2+consum_O2+generate_CO2; 0.362, 0.663, tmp+interval_yield +interval_Pdt.+OD+RQ_integral+AS_Vol.+emission_CO2+consum_O 2+generate_CO2; 0.352, 0.663, tmp_cumulo+pH_cumulo+interval _yield+OD+Cell_conc._interval+RQ_integral+AS_Vol.+emission_ CO2+consum_O2+generate_CO2; 0.341, 0.663, tmp_cumulo+pH_cum ulo+interval_yield+interval_Pdt.+OD+RS_cumulo+RQ_integral+A S_Vol.+consum_O2+interval_O2+generate_CO2; 0.341, 0.663, tm p_cumulo+pH_cumulo+interval_yield+interval_Pdt.+OD+Cell_con c._interval+RQ_integral+AS_Vol.+consum_O2+interval_O2+gener ate_CO2; 0.341, 0.663, tmp_cumulo+interval_yield+interval_P dt.+OD+RS_cumulo+rab_integral+RQ_integral+AS_Vol.+emission_ CO2+consum_O2+generate_CO2; 0.351, 0.663, tmp_cumulo+AN+int erval_yield+interval_Pdt.+RS_cumulo+RQ_integral+AS_Vol.+emi ssion_CO2+consum_O2+generate_CO2; 0.351, 0.663, tmp+pH_cumu lo+interval_yield+interval_Pdt.+rab_integral+RQ_integral+AS _Vol.+emission_CO2+consum_O2+generate_CO2; 0.361, 0.662, tm p_cumulo+pH_cumulo+interval_yield+interval_Pdt.+RQ_integral +AS_Vol.+emission_O2+consum_O2+generate_CO2; 0.341, 0.662, tmp_cumulo+pH_cumulo+interval_yield+interval_Pdt.+OD+Cell_c onc._interval+RQ_integral+AS_Vol.+emission_CO2+consum_O2+ge nerate_CO2; 0.341, 0.662, tmp_cumulo+pH_cumulo+PL_cumulo+in terval_yield+OD+rab_integral+RQ_integral+AS_Vol.+consum_O2+ interval_O2+generate_CO2; 0.351, 0.662, tmp_cumulo+pH_cumul o+AN+interval_yield+OD+RQ_integral+AS_Vol.+emission_CO2+con sum_O2+generate_CO2; 0.341, 0.662, tmp_cumulo+pH_cumulo+int erval_yield+interval_Pdt.+OD+Feed_Vol+RS_cumulo+RQ_integral +AS_Vol.+consum_O2+generate_CO2; 0.361, 0.662, tmp+interval _yield+interval_Pdt.+Cell_conc._interval+RQ_integral+AS_Vol. +emission_CO2+consum_O2+generate_CO2; 0.361, 0.662, tmp_cum ulo+pH_cumulo+interval_yield+OD+RS_cumulo+RQ_integral+consu m_O2+interval_O2+generate_CO2; 0.340, 0.662, tmp_cumulo+pH+ pH_cumulo+interval_yield+interval_Pdt.+OD+RQ_integral+AS_Vo l.+consum_O2+interval_O2+generate_CO2; 0.340, 0.662, tmp_cu mulo+interval_yield+interval_Pdt.+RS_cumulo+rab_integral+RQ _integral+AS_Vol.+emission_O2+emission_CO2+consum_O2+genera te_CO2; 0.351, 0.662, tmp_cumulo+pH_cumulo+interval_yield+i nterval_Pdt.+RS_cumulo+RQ_integral+AS_Vol.+consum_O2+interv al_O2+generate_CO2; 0.351, 0.662, tmp_cumulo+pH_cumulo+AN_c umulo+interval_yield+interval_Pdt.+RQ_integral+AS_Vol.+cons um_O2+interval_O2+generate_CO2; 0.351, 0.662, tmp_cumulo+pH _cumulo+AN+interval_yield+OD+Feed_Vol+RQ_integral+AS_Vol.+c onsum_O2+generate_CO2; 0.340, 0.662, tmp_cumulo+pH_cumulo+i nterval_yield+interval_Pdt.+OD+RS_cumulo+rab_integral+RQ_in tegral+emission_CO2+consum_O2+generate_CO2; 0.360, 0.662, t mp_cumulo+pH_cumulo+interval_yield+interval_Pdt.+RS_cumulo+ RQ_integral+emission_CO2+consum_O2+generate_CO2; 0.350, 0.6 62, tmp_cumulo+AN_cumulo+interval_yield+interval_Pdt.+RS_cu mulo+RQ_integral+AS_Vol.+emission_CO2+consum_O2+generate_CO 2; 0.340, 0.662, tmp+pH_cumulo+interval_yield+interval_Pdt. +OD+rab_integral+RQ_integral+AS_Vol.+emission_CO2+consum_O2 +generate_CO2; 0.350, 0.662, tmp_cumulo+pH_cumulo+interval_ yield+OD+RS_cumulo+RQ_integral+AS_Vol.+emission_O2+consum_O 2+generate_CO2; 0.360, 0.662, tmp_cumulo+pH_cumulo+interval _yield+OD+RQ_integral+Accum._Yield+AS_Vol.+consum_O2+genera te_CO2; 0.350, 0.662, tmp+tmp_cumulo+interval_yield+interva l_Pdt.+RS_cumulo+RQ_integral+AS_Vol.+emission_CO2+consum_O2 +generate_CO2; 0.329, 0.662, tmp_cumulo+pH_cumulo+interval_ yield+interval_Pdt.+OD+Feed_Vol+rab_integral+RQ_integral+AS _Vol.+emission_CO2+consum_O2+generate_CO2; 0.370, 0.662, tm p_cumulo+pH_cumulo+interval_yield+OD+Feed_Vol+RQ_integral+c onsum_O2+generate_CO2; 0.360, 0.662, tmp_cumulo+interval_yi eld+interval_Pdt.+rab_integral+RQ_integral+AS_Vol.+emission _CO2+consum_O2+generate_CO2; 0.350, 0.662, tmp_cumulo+pH_cu mulo+AN+interval_yield+OD+RS_cumulo+RQ_integral+AS_Vol.+con sum_O2+generate_CO2; 0.329, 0.662, tmp_cumulo+pH_cumulo+AN+ interval_yield+interval_Pdt.+OD+rab_integral+RQ_integral+AS _Vol.+emission_CO2+consum_O2+generate_CO2; 0.370, 0.662, tm p_cumulo+pH_cumulo+OD+RQ_integral+AS_Vol.+consum_O2+interva l_O2+generate_CO2; 0.360, 0.662, tmp_cumulo+pH+interval_yie ld+interval_Pdt.+RQ_integral+AS_Vol.+emission_CO2+consum_O2 +generate_CO2; 0.350, 0.662, tmp_cumulo+pH_cumulo+AN_cumulo +interval_yield+OD+RQ_integral+AS_Vol.+emission_CO2+consum O2+generate_CO2; 0.350, 0.662, tmp_cumulo+pH_cumulo+interva l_yield+OD+Feed_Vol+RS_cumulo+RQ_integral+AS_Vol.+consum_O2 +generate_CO2; 0.360, 0.662, tmp_cumulo+interval_yield+inte rval_Pdt.+RS_cumulo+RQ_integral+AS_Vol.+emission_O2+consum_ O2+generate_CO2; 0.350, 0.662, tmp_cumulo+AN_cumulo+interva l_yield+interval_Pdt.+RS_cumulo+rab_integral+RQ_integral+em ission_CO2+consum_O2+generate_CO2; 0.350, 0.661, tmp_cumulo +pH_cumulo+interval_yield+OD+RQ_integral+AS_Vol.+emission_O 2+emission_CO2+consum_O2+generate_CO2; 0.339, 0.661, tmp_cu mulo+pH_cumulo+PL_cumulo+interval_yield+interval_Pdt.+OD+RQ _integral+AS_Vol.+consum_O2+interval_O2+generate_CO2; 0.350, 0.661, tmp_cumulo+pH_cumulo+interval_yield+OD+Feed_Vol+RQ_ integral+AS_Vol.+emission_CO2+consum_O2+generate_CO2; 0.339, 0.661, tmp+pH_cumulo+interval_yield+interval_Pdt.+OD+Feed_ Vol+RQ_integral+AS_Vol.+emission_CO2+consum_O2+generate_CO 2; 0.360, 0.661, tmp+interval_yield+interval_Pdt.+RQ_integr al+AS_Vol.+emission_O2+emission_CO2+consum_O2+generate_CO2; 0.329, 0.661, tmp_cumulo+pH_cumulo+AN+interval_yield+OD+Fe ed_Vol+rab_integral+RQ_integral+AS_Vol.+consum_O2+interval O2+generate_CO2; 0.339, 0.661, tmp+tmp_cumulo+pH_cumulo+int erval_yield+interval_Pdt.+OD+RQ_integral+AS_Vol.+emission_C O2+consum_O2+generate_CO2; 0.369, 0.661, tmp_cumulo+pH_cumu lo+interval_yield+interval_Pdt.+RS_cumulo+RQ_integral+consu m_O2+generate_CO2; 0.339, 0.661, tmp_cumulo+pH_cumulo+AN+in terval_yield+interval_Pdt.+OD+rab_integral+RQ_integral+AS_V ol.+consum_O2+generate_CO2; 0.328, 0.661, tmp_cumulo+pH_cum ulo+AN+interval_yield+interval_Pdt.+OD+Feed_Vol+RQ_integral +AS_Vol.+consum_O2+interval_O2+generate_CO2; 0.349, 0.661, tmp_cumulo+pH_cumulo+AN+interval_yield+OD+RQ_integral+AS_Vo l.+emission_O2+consum_O2+generate_CO2; 0.339, 0.661, tmp+tm p_cumulo+pH_cumulo+interval_yield+interval_Pdt.+OD+RQ_integ ral+AS_Vol.+consum_O2+interval_O2+generate_CO2; 0.339, 0.66 1, tmp_cumulo+interval_yield+interval_Pdt.+OD+RS_cumulo+rab _integral+RQ_integral+emission_O2+emission_CO2+consum_O2+ge nerate_CO2; 0.359, 0.661, tmp_cumulo+pH_cumulo+AN+interval_ yield+OD+RS_cumulo+RQ_integral+consum_O2+generate_CO2; 0.34 9, 0.661, tmp_cumulo+interval_yield+interval_Pdt.+RS_cumulo +RQ_integral+AS_Vol.+emission_CO2+consum_O2+interval_O2+gen erate_CO2; 0.339, 0.661, tmp_cumulo+pH_cumulo+interval_yiel d+interval_Pdt.+OD+RS_cumulo+rab_integral+RQ_integral+AS_Vo l.+consum_O2+generate_CO2; 0.339, 0.661, tmp+tmp_cumulo+pH_ cumulo+interval_yield+OD+rab_integral+RQ_integral+AS_Vol.+c onsum_O2+interval_O2+generate_CO2; 0.378, 0.661, tmp+pH_cum ulo+interval_yield+OD+RQ_integral+consum_O2+generate_CO2; 0. 349, 0.661, tmp+interval_yield+interval_Pdt.+RS_cumulo+rab_ integral+RQ_integral+emission_O2+emission_CO2+consum_O2+gen erate_CO2; 0.349, 0.661, tmp_cumulo+pH_cumulo+interval_yiel d+interval_Pdt.+Feed_Vol+RQ_integral+AS_Vol.+emission_CO2+c onsum_O2+generate_CO2; 0.339, 0.661, tmp+pH_cumulo+interval _yield+interval_Pdt.+OD+Feed_Vol+rab_integral+RQ_integral+A S_Vol.+consum_O2+generate_CO2; 0.339, 0.661, tmp_cumulo+pH_ cumulo+interval_yield+OD+RS_cumulo+rab_integral+RQ_integral +AS_Vol.+emission_CO2+consum_O2+generate_C2 ; 0.359, 0.661, tmp+AN_cumulo+interval_yield+interval_Pdt.+RS_cumulo+RQ_in tegral+emission_CO2+consum_O2+generate_CO2; 0.349, 0.661, t mp_cumulo+pH_cumulo+interval_yield+OD+RQ_integral+Accum._Yi eld+AS_Vol.+consum_O2+interval_O2+generate_CO2; 0.339, 0.66 1, tmp_cumulo+pH_cumulo+interval_yield+OD+Cell_conc._interv al+rab_integral+RQ_integral+AS_Vol.+emission_CO2+consum_O2+ generate_CO2; 0.378, 0.661, tmp+interval_yield+interval_Pdt. +RS_cumulo+RQ_integral+consum_O2+generate_CO2; 0.369, 0.661, tmp+pH_cumulo+interval_yield+OD+RQ_integral+AS_Vol.+consum _O2+generate_CO2; 0.349, 0.661, tmp_cumulo+pH+interval_yiel d+interval_Pdt.+OD+RQ_integral+AS_Vol.+emission_CO2+consum O2+generate_CO2; 0.328, 0.661, tmp_cumulo+pH_cumulo+AN_cumu lo+interval_yield+interval_Pdt.+OD+rab_integral+RQ_integral +AS_Vol.+emission_CO2+consum_O2+generate_C2; 0.349, 0.661, tmp_cumulo+pH_cumulo+AN+interval_yield+interval_Pdt.+OD+RS _cumulo+RQ_integral+consum_O2+generate_CO2; 0.349, 0.661, t mp_cumulo+pH+pH_cumulo+interval_yield+OD+RQ_integral+AS_Vol. +emission_CO2+consum_O2+generate_CO2; 0.369, 0.661, tmp_cum ulo+interval_yield+interval_Pdt.+RS_cumulo+RQ_integral+cons um_O2+interval_O2+generate_CO2; 0.349, 0.661, tmp_cumulo+pH _cumulo+AN+interval_yield+OD+Cell_conc._interval+RQ_integra l+AS_Vol.+consum_O2+generate_CO2; 0.338, 0.661, tmp_cumulo+ pH_cumulo+AN_cumulo+interval_yield+OD+Feed_Vol+RQ_integral+ AS_Vol.+consum_O2+interval_O2+generate_CO2; 0.349, 0.661, t mp_cumulo+PL_cumulo+interval_yield+interval_Pdt.+RS_cumulo+ rab_integral+RQ_integral+emission_CO2+consuni_O2+generate_CO 2; 0.359, 0.661, tmp+AN+interval_yield+interval_Pdt.+RS_cum ulo+RQ_integral+emission_CO2+consum_O2+generate_CO2; 0.359, 0.661, tmp+pH_cumulo+interval_yield+interval_Pdt.+OD+RQ_in tegral+emission_CO2+consum_O2+generate_CO2; 0.338, 0.661, t mp_cumulo+pH_cumulo+AN+interval_yield+OD+rab_integral+RQ_in tegral+AS_Vol.+emission_CO2+consum_O2+generate_CO2; 0.368, 0.661, tmp_cumulo+pH_cumulo+interval_yield+OD+rab_integral+ RQ_integral+consum_O2+generate_CO2; 0.338, 0.661, tmp_cumul o+pH_cumulo+AN+interval_yield+OD+RQ_integral+AS_Vol.+emissi on_CO2+consum_O2+interval_O2+generate_CO2; 0.359, 0.661, tm p_cumulo+pH_cumulo+interval_yield+interval_Pdt.+rab_integra l+RQ_integral+AS_Vol.+consum_O2+generate_CO2; 0.338, 0.661, tmp_cumulo+pH_cumulo+AN+interval_yield+OD+RQ_integral+AS_V ol.+emission_O2+consum_O2+interval_O2+generate_CO2; 0.328, 0.661, tmp+pH_cumulo+AN+interval_yield+interval_Pdt.+OD+Fee d_Vol+RQ_integral+AS_Vol.+consum_O2+interval_O2+generate_CO 2; 0.349, 0.661, tmp_cumulo+pH_cumulo+interval_yield+OD+Cel l_conc._interval+RQ_integral+AS_Vol.+emission_O2+consum_O2+ generate_CO2; 0.359, 0.661, tmp+AN+interval_yield+interval_ Pdt.+RQ_integral+AS_Vol.+emission_CO2+consum_O2+generate_CO 2; 0.368, 0.661, tmp_cumulo+AN+interval_yield+interval_Pdt. +RS_cumulo+RQ_integral+consum_O2+generate_CO2; 0.358, 0.661, tmp+interval_yield+interval_Pdt.+Feed_Vol+RQ_integral+AS_V ol.+emission_CO2+consum_O2+generate_CO2; 0.338, 0.661, tmp_ cumulo+pH_cumulo+interval_yield+interval_Pdt.+OD+RS_cumulo+ RQ_integral+AS_Vol.+emission_O2+consum_O2+generate_CO2; 0.3 48, 0.661, tmp_cumulo+AN+interval_yield+interval_Pdt.+OD+RS _cumulo+RQ_integral+emission_CO2+consum_O2+generate_CO2; 0. 358, 0.661, tmp+pH+interval_yield+interval_Pdt.+RQ_integral +AS_Vol.+emission_CO2+consum_O2+generate_C2; 0.358, 0.661, tmp_cumulo+interval_yield+interval_Pdt.+RS_cumulo+Cell_con c._interval+RQ_integral+emission_CO2+consum_O2+generate_CO 2; 0.338, 0.661, tmp_cumulo+pH_cumulo+AN_cumulo+interval_yi eld+interval_Pdt.+OD+rab_integral+RQ_integral+AS_Vol.+consu m_O2+generate_CO2; 0.348, 0.661, tmp_cumulo+pH_cumulo+PL_cu mulo+interval_yield+OD+RS_cumulo+RQ_integral+AS_Vol.+consum _O2+generate_CO2; 0.348, 0.661, tmp_cumulo+pH+pH_cumulo+int erval_yield+OD+RS_cumulo+RQ_integral+AS_Vol.+consum_O2+gene rate_CO2; 0.348, 0.661, tmp_cumulo+pH+interval_yield+interv al_Pdt.+OD+RS_cumulo+RQ_integral+emission_CO2+consum_O2+gen erate_CO2; 0.348, 0.661, tmp_cumulo+pH+interval_yield+inter val_Pdt.+RS_cumulo+rab_integral+RQ_integral+emission_CO2+co nsum_O2+generate_CO2; 0.368, 0.661, tmp_cumulo+AN_cumulo+in terval_yield+interval_Pdt.+RS_cumulo+RQ_integral+consum_O2+ generate_CO2; 0.348, 0.661, tmp_cumulo+pH_cumulo+PL+interva l_yield+OD+rab_integral+RQ_integral+AS_Vol.+consum_O2+gener ate_CO2; 0.348, 0.661, tmp_cumulo+pH_cumulo+interval_yield+ OD+Feed_Vol+Cell_conc._interval+RQ_integral+AS_Vol.+consum_ O2+generate_CO2; 0.358, 0.660, tmp_cumulo+interval_yield+in terval_Pdt.+RQ_integral+AS_Vol.+emission_CO2+consum_O2+inte rval_O2+generate_CO2; 0.348, 0.660, tmp+tmp_cumulo+pH_cumul o+interval_yield+OD+RS_cumulo+RQ_integral+AS_Vol.+consum_O2 +generate_CO2; 0.348, 0.660, tmp_cumulo+pH_cumulo+AN_cumulo +interval_yield+OD+Feed_Vol+RQ_integral+AS_Vol.+consum_O2+g enerate_CO2; 0.377, 0.660, tmp_cumulo+pH_cumulo+OD+RQ_integ ral+AS_Vol.+consum_O2+generate_CO2; 0.358, 0.660, tmp+inter val_yield+interval_Pdt.+RQ_integral+AS_Vol.+emission_CO2+co nsum_O2+interval_O2+generate_CO2; 0.368, 0.660, tmp_cumulo+ interval_yield+interval_Pdt.+RQ_integral+AS_Vol.+consum_O2+ interval_O2+generate_CO2; 0.348, 0.660, tmp_cumulo+pH_cumul o+AN_cumulo+interval_yield+OD+RS_cumulo+RQ_integral+AS_Vol. +consum_O2+generate_CO2; 0.338, 0.660, tmp_cumulo+interval_ yield+interval_Pdt.+Feed_Vol+RS_cumulo+rab_integral+RQ_inte gral+AS_Vol.+emission_CO2+consum_O2+generate_CO2; 0.338, 0. 660, tmp_cumulo+pH_cumulo+AN+interval_yield+interval_Pdt.+R Q_integral+AS_Vol.+emission_CO2+consum_O2+interval_O2+gener ate_CO2; 0.348, 0.660, tmp_cumulo+pH_cumulo+AN_cumulo+inter val_yield+OD+RQ_integral+AS_Vol.+emission_O2+consuni_O2+gene rate_CO2; 0.338, 0.660, tmp_cumulo+pH_cumulo+interval_yield +OD+Feed_Vol+rab_integral+RQ_integral+AS_Vol.+emission_CO2+ consum_O2+generate_CO2; 0.358, 0.660, tmp+AN_cumulo+interva l_yield+interval_Pdt.+RQ_integral+AS_Vol.+emission_CO2+cons um_O2+generate_CO2; 0.338, 0.660, tmp_cumulo+pH_cumulo+inte rval_yield+OD+rab_integral+RQ_integral+AS_Vol.+emission_O2+ emission_CO2+consum_O2+generate_CO2; 0.348, 0.660, tmp+pH_c umulo+interval_yield+interval_Pdt.+Feed_Vol+RQ_integral+AS_ Vol.+consum_O2+interval_O2+generate_CO2; 0.348, 0.660, tmp_ cumulo+interval_yield+interval_Pdt.+Feed_Vol+RS_cumulo+rab_ integral+RQ_integral+emission_CO2+consum_O2+generate_CO2; 0. 358, 0.660, tmp_cumulo+pH+pH_cumulo+interval_yield+OD+RS_cu mulo+RQ_integral+consum_O2+generate_CO2; 0.348, 0.660, tmp+ tmp_cumulo+interval_yield+interval_Pdt.+RS_cumulo+rab_integ ral+RQ_integral+emission_CO2+consum_O2+generate_CO2; 0.348, 0.660, tmp_cumulo+pH+pH_cumulo+AN+interval_yield+OD+RQ_int egral+AS_Vol.+consum_O2+generate_CO2; 0.358, 0.660, tmp_cum ulo+interval_yield+interval_Pdt.+RS_cumulo+RQ_integral+AS_V ol.+consum+O2+interval+O2+generate_CO2; 0.327, 0.660, tmp+p H_cumulo+interval_yield+interval_Pdt.+OD+Feed_Vol+rab_integ ral+RQ_integral+AS_Vol.+consum_O2+interval_O2+generate_CO2; 0.327, 0.660, tmp_cumulo+pH_cumulo+interval_yield+interval _Pdt.+OD+Cell_conc._interval+rab_integral+RQ_integral+AS_Vo l.+emission_CO2+consum_O2+generate_CO2; 0.367, 0.660, tmp+p H_cumulo+interval_yield+interval_Pdt.+RQ_integral+AS_Vol.+c onsum_O2+generate_CO2; 0.348, 0.660, tmp_cumulo+pH_cumulo+i nterval_yield+interval_Pdt.+RQ_integral+AS_Vol.+emission_O2 +emission_CO2+consum_O2+generate_CO2; 0.367, 0.660, tmp_cum ulo+PL_cumulo+interval_yield+interval_Pdt.+RS_cumulo+RQ_int egral+consum_O2+generate_CO2; 0.358, 0.660, tmp_cumulo+pH_c umulo+interval_yield+OD+RS_cumulo+RQ_integral+emission_CO2+ consum_O2+generate_CO2; 0.337, 0.660, tmp_cumulo+pH+pH_cumu lo+AN+interval_yield+OD+RQ_integral+AS_Vol.+consum_O2+inter val_O2+generate_CO2; 0.348, 0.660, tmp_cumulo+pH_cumulo+AN+ interval_yield+interval_Pdt.+RQ_integral+AS_Vol.+emission_C O2+consum_O2+generate_CO2; 0.337, 0.660, tmp_cumulo+pH_cumu lo+AN+interval_yield+OD+Feed_Vol+rab_integral+RQ_integral+A S_Vol.+consum_O2+generate_CO2; 0.348, 0.660, tmp+interval_y ield+interval_Pdt.+RS_cumulo+rab_integral+RQ_integral+AS_Vo l.+emission_CO2+consum_O2+generate_CO2; 0.337, 0.660, tmp_c umulo+pH_cumulo+AN_cumulo+interval_yield+OD+RQ_integral+AS Vol.+emission_CO2+consum_O2+interval_O2+generate_CO2; 0.337, 0.660, tmp_cumulo+pH_cumulo+AN+interval_yield+interval_Pdt. +OD+Feed_Vol+RQ_integral+AS_Vol.+consum_O2+generate_CO2; 0. 337, 0.660, tmp_cumulo+pH_cumulo+PL_cumulo+interval_yield+i nterval_Pdt.+OD+RQ_integral+AS_Vol.+emission_CO2+consum_O2+ generate_CO2; 0.337, 0.660, tmp_cumulo+pH_cumulo+AN_cumulo+ interval_yield+OD+rab_integral+RQ_integral+AS_Vol.+emission _CO2+consum_O2+generate_CO2; 0.347, 0.660, tmp+tmp_cumulo+p H_cumulo+interval_yield+OD+Cell_conc._interval+RQ_integral+ AS_Vol.+consum_O2+generate_CO2; 0.337, 0.660, tmp_cumulo+pH _cumulo+AN+interval_yield+OD+Cell_conc._interval+RQ_integra l+AS_Vol.+consum_O2+interval_O2+generate_CO2; 0.357, 0.660, tmp_cumulo+pH+interval_yield+interval_Pdt.+RQ_integral+AS_ Vol.+consum_O2+interval_O2+generate_CO2; 0.347, 0.660, tmp+ interval_yield+interval_Pdt.+OD+RS_cumulo+rab_integral+RQ_i ntegral+emission_CO2+consum_O2+generate_CO2; 0.367, 0.660, tmp_cumulo+pH+interval_yield+interval_Pdt.+RS_cumulo+RQ_int egral+consum_O2+generate_CO2; 0.367, 0.660, tmp_cumulo+pH_c umulo+AN+interval_yield+OD+RQ_integral+consum_O2+generate_C 02; 0.347, 0.660, tmp_cumulo+pH_cumulo+interval_yield+inter val_Pdt.+Cell_conc._interval+RQ_integral+AS_Vol.+consum_O2+ interval_O2+generate_CO2; 0.347, 0.660, tmp_cumulo+pH+pH_cu mulo+interval_yield+OD+RQ_integral+AS_Vol.+emission_O2+cons um_O2+generate_CO2; 0.357, 0.660, tmp_cumulo+pH_cumulo+AN+i nterval_yield+interval_Pdt.+OD+RQ_integral+consum_O2+genera te_CO2; 0.347, 0.660, tmp+tmp_cumulo+pH_cumulo+interval_yie ld+OD+RQ integral+AS Vol.+emission_CO2+consum_O2+generate_C 02; 0.347, 0.660, tmp_cumulo+pH_cumulo+AN_cumulo+interval_y ield+OD+Cell_conc._interval+RQ_integral+AS_Vol.+consum_O2+g enerate_CO2; 0.337, 0.660, tmp_cumulo+pH_cumulo+PL+interval _yield+OD+rab_integral+RQ_integral+AS_Vol.+consum_O2+interv al_O2+generate_CO2; 0.337, 0.660, tmp_cumulo+pH+pH_cumulo+i nterval_yield+interval_Pdt.+OD+RQ_integral+AS_Vol.+emission _CO2+consum_O2+generate_CO2; 0.357, 0.660, tmp_cumulo+inter val_yield+interval_Pdt.+OD+RQ_integral+AS_Vol.+emission_CO2 +consum_O2+generate_CO2; 0.347, 0.660, tmp+interval_yield+i nterval_Pdt.+OD+RS_cumulo+RQ_integral+AS_Vol.+emission_CO2+ consum_O2+generate_CO2; 0.347, 0.660, tmp_cumulo+pH_cumulo+ interval_yield+interval_Pdt.+RS_cumulo+rab_integral+RQ_inte gral+emission_CO2+consum_O2+generate_CO2; 0.326, 0.660, tmp _cumulo+pH_cumulo+interval_yield+interval_Pdt.+OD+Feed_Vol+ RQ_integral+AS_Vol.+emission_CO2+consum_O2+interval_O2+gene rate_CO2; 0.367, 0.660, tmp_cumulo+pH_cumulo+interval_yield +OD+RS+RQ_integral+consum_O2+generate_CO2; 0.367, 0.660, tm p_cumulo+pH_cumulo+interval_yield+OD+RQ_integral+consum_O2+ interval_O2+generate_CO2; 0.357, 0.660, tmp_cumulo+pH+inter val_yield+interval_Pdt.+OD+RQ_integral+AS_Vol.+consum_O2+ge nerate_CO2; 0.347, 0.660, tmp_cumulo+AN_cumulo+interval_yie ld+interval_Pdt.+OD+RS_cumulo+RQ_integral+emission_CO2+cons um_O2+generate_CO2; 0.347, 0.660, tmp_cumulo+pH_cumulo+AN_c umulo+interval_yield+interval_Pdt.+RQ_integral+AS_Vol.+emis sion_CO2+consum_O2+generate_CO2; 0.347, 0.660, tmp+interval _yield+interval_Pdt.+Feed_Vol+RS_cumulo+RQ_integral+AS_Vol. +emission_CO2+consum_O2+generate_CO2; 0.336, 0.660, tmp_cum ulo+interval_yield+interval_Pdt.+OD+RS_cumulo+RQ_integral+A S_Vol.+emission_emission_CO2+consum_O2+generate_CO2; 0.3 36, 0.660, tmp_cumulo+pH_cumulo+AN_cumulo+interval_yield+OD +RQ integral+AS Vol.+emission O2+consum O2+interval O2+gene rate_CO2; 0.357, 0.660, tmp_cumulo+interval_yield+interval_ Pdt.+OD+RS cumulo+RQ integral+emission O2+consum O2+generat e CO2; 0.347, 0.660, tmp_cumulo+pH_cumulo+PL_cumulo+interva l_yield+interval_Pdt.+RQ_integral+AS_Vol.+consum_O2+interva l_O2+generate_CO2; 0.347, 0.660, tmp_cumulo+pH+pH_cumulo+in terval_yield+interval_Pdt.+RQ_integral+AS_Vol.+consum_O2+in terval_O2+generate_CO2; 0.347, 0.660, tmp_cumulo+pH_cumulo+ PL_cumulo+AN+interval_yield+OD+RQ_integral+AS_Vol.+consum_0 2+generate CO2; 0.347, 0.660, tmp_cumulo+interval_yield+int erval_Pdt.+RS_cumulo+Cell_conc._interval+RQ_integral+AS_Vol. +emission_CO2+consum_O2+generate_CO2; 0.336, 0.659, tmp_cum ulo+pH_cumulo+interval_yield+interval_Pdt.+OD+Feed_Vol+RQ_i ntegral+AS_Vol.+emission_O2+consum_O2+generate_CO2; 0.347, 0.659, tmp_cumulo+pH_cumulo+interval_yield+OD+Feed_Vol+RQ_i ntegral+AS_Vol.+emission-O2+consum -O2+generate-CO2; 0.336, 0.659, tmp_cumulo+pH_cumulo+AN+interval_yield+OD+RS_cumulo+ RQ_integral+AS_Vol.+consum_O2+interval_O2+generate_CO2; 0.3 36, 0.659, tmp_cumulo+pH+pH_cumulo+interval_yield+OD+RQ_int egral+AS Vol.+emission O2+consum O2+interval O2+generate CO 2; 0.347, 0.659, tmp_cumulo+pH_cumulo+PL_cumulo+interval_yi eld+OD+RQ integral+AS Vol.+emission CO2+consum O2+generate CO2; 0.366, 0.659, tmp_cumulo+pH_cumulo+interval_yield+RQ_i ntegral+AS_Vol.+consum_O2+interval_O2+generate_CO2; 0.336, 0.659, tmp_cumulo+pH_cumulo+AN+interval_yield+interval_Pdt. +OD+RQ_integral+AS_Vol.+emission_O2+consum_O2+generate_CO2; 0.357, 0.659, tmp_cumulo+pH+interval_yield+interval_Pdt.+0 D+RS_cumulo+RQ_integral+consum_O2+generate_CO2; 0.336, 0.65 9, tmp_cumulo+pH_cumulo+interval_yield+interval_Pdt.+Feed_V ol+rab integral+RQ integral+AS Vol.+emission CO2+consum O2+ generate_CO2; 0.357, 0.659, tmp+pH_cumulo+interval_yield+in terval_Pdt.+RQ_integral+AS_Vol.+consum_02+interval_02+gener ate_CO2; 0.336, 0.659, tmp_cumulo+pH_cumulo+interval_yield+ interval -Pdt.+OD+Cell-conc. -interval+rab-integral+RQ_integr al+AS_Vol.+consum_O2+generate_CO2; 0.336, 0.659, tmp_cumulo +pH_cumulo+interval_yield+OD+Feed_Vol+RQ_integral+AS_Vol.+e mission_CO2+consum_O2+interval_O2+generate_CO2; 0.336, 0.65 9, tmp_cumulo+pH_cumulo+interval_yield+OD+Feed_Vol+RS_cumul o+rab_integral+RQ_integral+AS_Vol.+consum_O2+generate_CO2; 0.346, 0.659, tmp cumulo+PL cumulo+interval yield+interval Pdt.+RS_cumulo+RQ_integral+AS_Vol.+emission_CO2+consum_O2+g enerate_CO2; 0.336, 0.659, tmp_cumulo+pH_cumulo+interval_yi eld+OD+Feed_Vol+Cell_conc._interval+RQ_integral+AS_Vol.+con sum_O2+interval_O2+generate_CO2; 0.336, 0.659, tmp_cumulo+p H+pH_cumulo+AN_cumulo+interval_yield+OD+RQ_integral+AS_Vol. +consum_O2+interval_O2+generate_CO2; 0.336, 0.659, tmp_cumu lo+pH+pH_cumulo+interval_yield+OD+rab_integral+RQ_integral+ AS_Vol.+emission_CO2+consum_O2+generate_CO2; 0.325, 0.659, tmp_cumulo+pH_cumulo+AN+interval_yield+interval Pdt.+OD+RQ integral+AS_Vol.+emission_CO2+consum_O2+interval_O2+generat e_CO2; 0.356, 0.659, tmp_cumulo+pH_cumulo+interval_yield+in terval_Pdt.+OD+rab_integral+RQ_integral+consum_O2+generate CO2; 0.356, 0.659, tmp_cumulo+interval_yield+interval_Pdt.+ OD+RS_cumulo+RQ_integral+AS_Vol.+consum_O2+generate_CO2; 0. 346, 0.659, tmp+tmp cumulo+interval yield+interval Pdt.+OD+ RS cumulo+RQ integral+emission CO2+consum O2+generate CO2; 0.336, 0.659, tmp_cumulo+pH_cumulo+interval_yield+interval Pdt.+rab integral+RQ integral+AS Vol.+emission O2+emission CO2+consum_O2+generate_CO2; 0.366, 0.659, tmp+pH_cumulo+int erval_yield+OD+Cell_conc._interval+RQ_integral+consum_02+ge nerate_CO2; 0.356, 0.659, tmp_cumulo+pH_cumulo+interval_yie ld+rab_integral+RQ_integral+AS_Vol.+consum_02+interval_02+g enerate_CO2; 0.336, 0.659, tmp_cumulo+AN+interval_yield+int erval_Pdt.+RS_cumulo+rab_integral+RQ_integral+AS_Vol.+emiss ion_CO2+consum_O2+generate_CO2; 0.346, 0.659, tmp_cumulo+pH _cumulo+AN_cumulo+interval_yield+interval_Pdt.+OD+RS_cumulo +RQ_integral+consum_O2+generate_CO2; 0.356, 0.659, tmp_cumu lo+pH+interval_yield+OD+RQ_integral+AS_Vol.+consum_O2+inter val_O2+generate_CO2; 0.336, 0.659, tmp_cumulo+pH_cumulo+int erval_yield+OD+Feed_Vol+RS_cumulo+RQ_integral+AS_Vol.+consu m_O2+interval_O2+generate_CO2; 0.346, 0.659, tmp_cumulo+pH+ pH_cumulo+interval_yield+OD+Feed_Vol+RQ_integral+AS_Vol.+co nsum_O2+generate_CO2; 0.336, 0.659, tmp_cumulo+pH_cumulo+in terval_yield+OD+RS_cumulo+RQ_integral+AS_Vol.+emission_O2+c onsum_O2+interval_O2+generate_CO2; 0.346, 0.659, tmp+pH_cum ulo+interval_yield+interval_Pdt.+RQ_integral+AS_Vol.+emissi on_CO2+consum_O2+interval_O2+generate_CO2; 0.336, 0.659, tm p_cumulo+pH+interval_yield+interval_Pdt.+RS_cumulo+rab_inte gral+RQ_integral+AS_Vol.+emission_CO2+consum_O2+generate_CO 2; 0.376, 0.659, tmp+interval_yield+interval_Pdt.+RQ_integr al+AS_Vol.+consum_O2+generate_CO2; 0.366, 0.659, tmp+tmp_cu mulo+interval_yield+interval_Pdt.+RS_cumulo+RQ_integral+con sum_O2+generate_CO2; 0.356, 0.659, tmp+pH_cumulo+AN_cumulo+ interval_yield+interval_Pdt.+OD+RQ_integral+consum_O2+gener ate_CO2; 0.346, 0.659, tmp+interval_yield+interval_Pdt.+OD+ RS_cumulo+RQ_integral+emission_O2+emission_CO2+consum_O2+ge nerate_CO2; 0.346, 0.659, tmp+tmp_cumulo+pH_cumulo+interval _yield+interval_Pdt.+RQ_integral+AS_Vol.+consum_O2+interval _O2+generate_CO2; 0.336, 0.659, tmp_cumulo+pH_cumulo+PL_cum ulo+AN+interval_yield+OD+RQ_integral+AS_Vol.+consum_O2+inte rval_O2+generate_CO2; 0.336, 0.659, tmp_cumulo+pH+interval_ yield+interval_Pdt.+OD+RS_cumulo+RQ_integral+AS_Vol.+emissi on_CO2+consum_O2+generate_CO2; 0.346, 0.659, tmp_cumulo+pH_ cumulo+interval_yield+OD+RS_cumulo+Cell_conc._interval+RQ_i ntegral+AS_Vol.+consum_O2+generate_CO2; 0.325, 0.659, tmp+p H_cumulo+interval_yield+interval_Pdt.+OD+Feed_Vol+rab_integ ral+RQ_integral+AS_Vol.+emission_CO2+consum_O2+generate_CO 2; 0.336, 0.659, tmp_cumulo+pH+pH_cumulo+interval_yield+OD+ Feed_Vol+RQ_integral+AS_Vol.+consum_O2+interval_O2+generate _CO2; 0.346, 0.659, tmp_cumulo+pH_cumulo+interval_yield+OD+ rab_integral+RQ_integral+Accum._Yield+AS_Vol.+consum_O2+gen erate_CO2; 0.356, 0.659, tmp_cumulo+pH_cumulo+AN_cumulo+int erval_yield+OD+RS_cumulo+RQ_integral+consum_O2+generate_CO 2; 0.346, 0.659, tmp_cumulo+pH+pH_cumulo+AN_cumulo+interval _yield+OD+RQ_integral+AS_Vol.+consum_O2+generate_CO2; 0.336, 0.659, tmp_cumulo+pH_cumulo+interval_yield+OD+Feed_Vol+RQ_ integral+AS_Vol.+emission_O2+consum_O2+interval_O2+generate _CO2; 0.346, 0.659, tmp_cumulo+pH_cumulo+interval_yield+int erval_Pdt.+RS_cumulo+RQ_integral+AS_Vol.+emission_O2+consum _O2+generate_CO2; 0.356, 0.659, tmp_cumulo+pH_cumulo+AN_cum ulo+interval_yield+interval_Pdt.+OD+RQ_integral+consum_O2+g enerate_CO2; 0.336, 0.659, tmp+pH_cumulo+interval_yield+int erval_Pdt.+OD+RQ_integral+AS_Vol.+emission_O2+emission_CO2+ consum_O2+generate_CO2; 0.335, 0.659, tmp_cumulo+pH_cumulo+ AN+interval_yield+interval_Pdt.+rab_integral+RQ_integral+AS _Vol.+emission_CO2+consum_O2+generate_CO2; 0.335, 0.659, tm p_cumulo+pH_cumulo+interval_yield+OD+Cell_conc._interval+RQ _integral+AS_Vol.+emission_CO2+consum_O2+interval_O2+genera te_CO2; 0.325, 0.659, tmp_cumulo+pH_cumulo+AN_cumulo+interv al_yield+OD+Feed_Vol+rab_integral+RQ_integral+AS_Vol.+consu m_O2+interval_O2+generate_CO2; 0.325, 0.659, tmp_cumulo+pH_ cumulo+interval_yield+interval_Pdt.+OD+Feed_Vol+RS_cumulo+R Q_integral+AS_Vol.+consum_O2+interval_O2+generate_CO2; 0.33 5, 0.659, tmp_cumulo+pH_cumulo+interval_yield+interval_Pdt. +Feed_Vol+RQ_integral+AS_Vol.+emission_CO2+consum_O2+interv al_O2+generate_CO2; 0.375, 0.659, tmp_cumulo+interval_yield +OD+RS_cumulo+RQ_integral+consum_O2+generate_CO2; 0.346, 0. 659, tmp+tmp_cumulo+pH_cumulo+AN+interval_yield+OD+RQ_integ ral+AS_Vol.+consum_O2+generate_CO2; 0.346, 0.659, tmp_cumul o+pH_cumulo+PL_cumulo+AN_cumulo+interval_yield+OD+RQ_integr al+AS_Vol.+consum_O2+generate_CO2; 0.335, 0.659, tmp_cumulo +pH_cumulo+interval_yield+OD+RS_cumulo+RQ_integral+AS_Vol.+ emission_CO2+consum_O2+interval_O2+generate_CO2; 0.325, 0.6 59, tmp_cumulo+pH_cumulo+AN+interval_yield+OD+rab_integral+ RQ_integral+AS_Vol.+emission_CO2+consum_O2+interval_O2+gene rate_CO2; 0.335, 0.659, tmp_cumulo+pH+pH_cumulo+interval_yi eld+OD+RQ_integral+AS_Vol.+emission_CO2+consum_O2+interval_ O2+generate_CO2; 0.325, 0.659, tmp_cumulo+pH_cumulo+interva l_yield+interval_Pdt.+OD+rab_integral+RQ_integral+AS_Vol.+e mission_O2+consum_O2+interval_O2+generate_CO2; 0.356, 0.659, tmp+tmp_cumulo+pH_cumulo+interval_yield+OD+RS_cumulo+RQ_in tegral+consum_O2+generate_CO2; 0.346, 0.659, tmp_cumulo+pH_ cumulo+interval_yield+OD+RS_cumulo+rab_integral+RQ_integral +consum_O2+interval_O2+generate_CO2; 0.335, 0.659, tmp_cumu lo+pH_cumulo+PL_cumulo+interval_yield+interval_Pdt.+OD+rab_ integral+RQ_integral+AS_Vol.+consum_O2+generate_CO2; 0.335, 0.659, tmp_cumulo+pH_cumulo+AN_cumulo+interval_yield+inter val_Pdt.+OD+RQ_integral+AS_Vol.+emission_O2+consum_O2+gener ate_CO2; 0.335, 0.659, tmp_cumulo+pH_cumulo+AN_cumulo+inter val_yield+interval_Pdt.+OD+Feed_Vol+RQ_integral+AS_Vol.+con sum_O2+generate_CO2; 0.346, 0.659, tmp+interval_yield+inter val_Pdt.+RS_cumulo+RQ_integral+AS_Vol.+emission_O2+emission _CO2+consum_O2+generate_CO2; 0.324, 0.659, tmp_cumulo+pH_cu mulo+interval_yield+interval_Pdt.+OD+Feed_Vol+RQ_integral+A S_Vol.+emission_O2+consum_O2+interval_O2+generate_CO2; 0.32 4, 0.659, tmp_cumulo+pH_cumulo+interval_yield+interval_Pdt. +OD+RS_cumulo+RQ_integral+AS_Vol.+emission_O2+emission_CO2+ consum_O2+generate_CO2; 0.346, 0.659, tmp+pH_cumulo+interva l_yield+interval_Pdt.+RQ_integral+AS_Vol.+emission_O2+emiss ion_CO2+consum_O2+generate_CO2; 0.324, 0.659, tmp+tmp_cumul o+pH_cumulo+interval_yield+interval_Pdt.+OD+rab_integral+RQ _integral+AS_Vol.+emission_CO2+consum_O2+generate_CO2; 0.34 6, 0.659, tmp_cumulo+pH_cumulo+interval_yield+interval_Pdt. +OD+RS_cumulo+RQ_integral+emission_O2+consum_O2+generate_CO 2; 0.345, 0.659, tmp_cumulo+pH+interval_yield+interval_Pdt. +rab_integral+RQ_integral+AS_Vol.+emission_CO2+consum_O2+ge nerate_CO2; 0.356, 0.659, tmp_cumulo+interval_yield+interva l_Pdt.+RS_cumulo+RQ_integral+emission_CO2+consum_O2+interva l_O2+generate_CO2; 0.345, 0.659, tmp_cumulo+pH_cumulo+PL+in terval_yield+interval_Pdt.+OD+RQ_integral+AS_Vol.+consum_O2 +generate_CO2; 0.324, 0.659, tmp_cumulo+pH_cumulo+AN_cumulo +interval_yield+interval_Pdt.+OD+Feed_Vol+RQ_integral+AS_Vo l.+consum_O2+interval_O2+generate_CO2; 0.356, 0.659, tmp+pH _cumulo+AN+interval_yield+interval_Pdt.+OD+RQ_integral+cons um_O2+generate_CO2; 0.335, 0.659, tmp_cumulo+pH_cumulo+inte rval_yield+OD+Cell_conc._interval+RQ_integral+AS_Vol.+emiss ion_O2+consum_O2+interval_O2+generate_CO2; 0.335, 0.659, tm p_cumulo+pH_cumulo+interval_yield+interval_Pdt.+RS_cumulo+R Q_integral+AS_Vol.+emission_CO2+consum_O2+interval_O2+gener ate_CO2; 0.324, 0.659, tmp_cumulo+pH_cumulo+interval_yield+ interval_Pdt.+OD+Feed_Vol+RS_cumulo+rab_integral+RQ_integra l+AS_Vol.+consum_O2+generate_CO2; 0.355, 0.659, tmp_cumulo+ interval_yield+interval_Pdt.+RS_cumulo+rab_integral+RQ_inte gral+emission_O2+consum_O2+generate_CO2; 0.355, 0.659, tmp_ cumulo+pH_cumulo+interval_yield+OD+RS_cumulo+RQ_integral+em ission_O2+consum_O2+generate_CO2; 0.335, 0.659, tmp_cumulo+ pH_cumulo+AN_cumulo+interval_yield+OD+Cell_conc._interval+R Q_integral+AS_Vol.+consum_O2+interval_O2+generate_CO2; 0.35 5, 0.659, tmp+pH_cumulo+interval_yield+OD+RQ_integral+AS_Vo l.+consum_O2+interval_O2+generate_CO2; 0.335, 0.659, tmp_cu mulo+pH+pH_cumulo+interval_yield+interval_Pdt.+OD+rab_integ ral+RQ_integral+AS_Vol.+consum_O2+generate_CO2; 0.355, 0.65 9, tmp+pH_cumulo+interval_yield+OD+Cell_conc._interval+RQ_i ntegral+AS_Vol.+consum_O2+generate_CO2; 0.335, 0.659, tmp_c umulo+pH_cumulo+AN+AN_cumulo+interval_yield+OD+RQ_integral+ AS_Vol.+consum_O2+interval_O2+generate_CO2; 0.345, 0.659, t mp_cumulo+pH+pH_cumulo+interval_yield+OD+Cell_conc._interva l+RQ_integral+AS_Vol.+consum_O2+generate_CO2; 0.355, 0.659, tmp+pH+interval_yield+interval_Pdt.+RS_cumulo+RQ_integral+ emission_CO2+consum_O2+generate_CO2; 0.345, 0.659, tmp+tmp_ cumulo+pH_cumulo+interval_yield+OD+RQ_integral+AS_Vol.+emis sion_O2+consum_O2+generate_CO2; 0.324, 0.659, tmp_cumulo+pH _cumulo+AN+interval_yield+interval_Pdt.+OD+rab_integral+RQ_ integral+AS_Vol.+consum_O2+interval_O2+generate_CO2; 0.345, 0.659, tmp_cumulo+pH_cumulo+PL_cumulo+interval_yield+OD+Fe ed_Vol+RQ_integral+AS_Vol.+consum_O2+generate_CO2; 0.335, 0. 659, tmp_cumulo+pH_cumulo+interval_yield+OD+RQ_integral+AS_ Vol.+emission_O2+emission_CO2+consum_O2+interval_O2+generat e_CO2; 0.355, 0.658, tmp+interval_yield+interval_Pdt.+RS_cu mulo+Cell_conc._interval+RQ_integral+emission_CO2+consum_O2 +generate_CO2; 0.324, 0.658, tmp_cumulo+pH_cumulo+interval_ yield+interval_Pdt.+OD+RQ_integral+AS_Vol.+emission_O2+emis sion_CO2+consum_O2+interval_O2+generate_CO2; 0.335, 0.658, tmp_cumulo+AN_cumulo+interval_yield+interval_Pdt.+RS_cumulo +rab_integral+RQ_integral+AS_Vol.+emission_CO2+consum_O2+ge nerate_CO2; 0.345, 0.658, tmp+pH_cumulo+interval_yield+inte rval_Pdt.+OD+RQ_integral+AS_Vol.+consum_O2+interval_O2+gene rate_CO2; 0.365, 0.658, tmp_cumulo+pH_cumulo+AN_cumulo+inte rval_yield+OD+RQ_integral+consum_O2+generate_CO2; 0.345, 0. 658, tmp_cumulo+pH_cumulo+AN+AN_cumulo+interval_yield+OD+RQ _integral+AS_Vol.+consum_O2+generate_CO2; 0.335, 0.658, tmp _cumulo+pH_cumulo+interval_yield+interval_Pdt.+RS_cumulo+RQ _integral+AS_Vol.+emission_O2+emission_CO2+consum_O2+genera te_CO2; 0.355, 0.658, tmp_cumulo+interval_yield+interval_Pd t.+Feed_Vol+RS_cumulo+RQ_integral+AS_Vol.+consum_O2+generat e_CO2; 0.365, 0.658, tmp_cumulo+interval_yield+interval_Pdt. +Feed_Vol+RS_cumulo+RQ_integral+consum_O2+generate_CO2; 0.3 35, 0.658, tmp_cumulo+pH_cumulo+AN_cumulo+interval_yield+in terval_Pdt.+rab_integral+RQ_integral+AS_Vol.+emission_CO2+c onsum_O2+generate_CO2; 0.335, 0.658, tmp_cumulo+pH_cumulo+P L_cumulo+AN_cumulo+interval_yield+OD+RQ_integral+AS_Vol.+co nsum_O2+interval_O2+generate_CO2; 0.345, 0.658, tmp_cumulo+ pH_cumulo+PL_cumulo+interval_yield+OD+Cell_conc._interval+R Q_integral+AS_Vol.+consum_O2+generate_CO2; 0.355, 0.658, tm p_cumulo+pH_cumulo+interval_yield+interval_Pdt.+Feed_Vol+RQ _integral+AS_Vol.+consum_O2+generate_CO2; 0.355, 0.658, tmp _cumulo+pH_cumulo+PL_cumulo+interval_yield+OD+RS_cumulo+RQ_ integral+consum_O2+generate_CO2; 0.335, 0.658, tmp_cumulo+p H_cumulo+AN_cumulo+interval_yield+OD+Feed_Vol+rab_integral+ RQ_integral+AS_Vol.+consum_O2+generate_CO2; 0.345, 0.658, t mp+pH_cumulo+interval_yield+interval_Pdt.+Feed_Vol+RQ_integ ral+AS_Vol.+emission_CO2+consum_O2+generate_CO2; 0.335, 0.6 58, tmp_cumulo+pH+pH_cumulo+interval_yield+OD+RS_cumulo+RQ_ integral+AS_Vol.+consum_O2+interval_O2+generate_CO2; 0.334, 0.658, tmp+tmp_cumulo+interval_yield+interval_Pdt.+RS_cumu lo+rab_integral+RQ_integral+AS_Vol.+emission_CO2+consum_O2+ generate_CO2; 0.334, 0.658, tmp_cumulo+pH_cumulo+interval_y ield+interval_Pdt.+OD+RS_cumulo+RQ_integral+emission_O2+emi ssion_CO2+consum_O2+generate_CO2; 0.355, 0.658, tmp_cumulo+ interval_yield+interval_Pdt.+Feed_Vol+RQ_integral+AS_Vol.+e mission_CO2+consum_O2+generate_CO2; 0.355, 0.658, tmp_cumul o+interval_yield+interval_Pdt.+OD+RS_cumulo+rab_integral+RQ _integral+consum_O2+generate_CO2; 0.355, 0.658, tmp+pH_cumu lo+interval_yield+interval_Pdt.+OD+rab_integral+RQ_integral +consum_O2+generate_CO2; 0.334, 0.658, tmp_cumulo+pH_cumulo +AN+interval_yield+interval_Pdt.+OD+RS_cumulo+RQ_integral+A S_Vol.+consum_O2+generate_CO2; 0.334, 0.658, tmp_cumulo+pH_ cumulo+AN_cumulo+interval_yield+OD+RS_cumulo+RQ_integral+AS Vol.+consum_O2+interval_O2+generate_CO2; 0.324, 0.658, tmp _cumulo+pH_cumulo+interval_yield+OD+Feed_Vol+rab_integral+R Q_integral+AS_Vol.+emission_CO2+consum_O2+interval_O2+gener ate_CO2; 0.334, 0.658, tmp+tmp_cumulo+pH_cumulo+interval_yi eld+interval_Pdt.+OD+rab_integral+RQ_integral+AS_Vol.+consu m_O2+generate_CO2; 0.334, 0.658, tmp+pH_cumulo+interval_yie ld+interval_Pdt.+OD+Cell_conc._interval+RQ_integral+AS_Vol. +emission_CO2+consum_O2+generate_CO2; 0.323, 0.658, tmp_cum ulo+pH_cumulo+AN_cumulo+interval_yield+OD+rab_integral+RQ_i ntegral+AS_Vol.+emission_CO2+consum_O2+interval_O2+generate _CO2; 0.345, 0.658, tmp+tmp_cumulo+pH_cumulo+interval_yield +OD+Feed_Vol+RQ_integral+AS_Vol.+consum_O2+generate_CO2; 0. 323, 0.658, tmp_cumulo+pH_cumulo+AN_cumulo+interval_yield+i nterval_Pdt.+OD+RQ_integral+AS_Vol.+emission_CO2+consum_O2+ interval_O2+generate_CO2; 0.334, 0.658, tmp_cumulo+pH_cumul o+PL_cumulo+interval_yield+OD+Feed_Vol+RQ_integral+AS_Vol.+ consum_O2+interval_O2+generate_CO2; 0.334, 0.658, tmp_cumul o+pH_cumulo+AN_cumulo+interval_yield+interval_Pdt.+RQ_integ ral+AS_Vol.+emission_CO2+consum_O2+interval_O2+generate_CO 2; 0.364, 0.658, tmp+interval_yield+interval_Pdt.+RQ_integr al+AS_Vol.+consum_O2+interval_O2+generate_CO2; 0.344, 0.658, tmp+tmp_cumulo+pH_cumulo+AN_cumulo+interval_yield+OD+RQ_in tegral+AS_Vol.+consum_O2+generate_CO2; 0.334, 0.658, tmp+pH _cumulo+AN+interval_yield+interval_Pdt.+OD+Feed_Vol+RQ_inte gral+AS_Vol.+consum_O2+generate_CO2; 0.334, 0.658, tmp_cumu lo+pH_cumulo+AN+interval_yield+OD+rab_integral+RQ_integral+ AS_Vol.+emission_O2+consum_O2+generate_CO2; 0.334, 0.658, t mp_cumulo+pH_cumulo+interval_yield+interval_Pdt.+OD+Cell_co nc._interval+RQ_integral+AS_Vol.+emission_O2+consum_O2+gene rate_CO2; 0.354, 0.658, tmp_cumulo+pH_cumulo+AN+interval_yi eld+interval_Pdt.+RQ_integral+AS_Vol.+consum_O2+generate_CO 2; 0.334, 0.658, tmp_cumulo+pH_cumulo+interval_yield+OD+rab _integral+RQ_integral+Accum._Yield+AS_Vol.+consum_O2+interv al_O2+generate_CO2; 0.334, 0.658, tmp_cumulo+pH_cumulo+AN+i nterval_yield+interval_Pdt.+Feed_Vol+RQ_integral+AS_Vol.+co nsum_O2+interval_O2+generate_CO2; 0.334, 0.658, tmp+tmp_cum ulo+pH_cumulo+AN+interval_yield+OD+RQ_integral+AS_Vol.+cons um_O2+interval_O2+generate_CO2; 0.364, 0.658, tmp_cumulo+pH +interval_yield+interval_Pdt.+RQ_integral+AS_Vol.+consum_O2 +generate_CO2; 0.344, 0.658, tmp_cumulo+pH_cumulo+interval_ yield+interval_Pdt.+rab_integral+RQ_integral+AS_Vol.+emissi on_O2+consum_O2+generate_CO2; 0.334, 0.658, tmp_cumulo+pH_c umulo+interval_yield+OD+Feed_Vol+Cell_conc._interval+rab_in tegral+RQ_integral+AS_Vol.+consum_O2+generate_CO2; 0.344, 0. 658, tmp+tmp_cumulo+pH_cumulo+interval_yield+interval_Pdt.+ OD+RS_cumulo+RQ_integral+consum_O2+generate_CO2; 0.344, 0.6 58, tmp+pH_cumulo+AN+interval_yield+interval_Pdt.+RQ_integr al+AS_Vol.+emission_CO2+consum_O2+generate_CO2; 0.334, 0.65 8, tmp_cumulo+pH_cumulo+interval_yield+interval_Pdt.+OD+Fee d_Vol+Cell_conc._interval+RQ_integral+AS_Vol.+consum_O2+gen erate_CO2; 0.323, 0.658, tmp_cumulo+pH+pH_cumulo+interval_y ield+interval_Pdt.+OD+rab_integral+RQ_integral+AS_Vol.+emis sion_CO2+consum_O2+generate_CO2; 0.344, 0.658, tmp+interval _yield+interval_Pdt.+rab_integral+RQ_integral+AS_Vol.+emiss ion_O2+emission_CO2+consum_O2+generate_CO2; 0.344, 0.658, t mp_cumulo+AN+interval_yield+interval_Pdt.+RS_cumulo+RQ_inte gral+emission_O2+emission_CO2+consum_O2+generate_CO2; 0.323, 0.658, tmp_cumulo+pH_cumulo+interval_yield+interval_Pdt.+O D+Feed_Vol+RS_cumulo+RQ_integral+AS_Vol.+emission_CO2+consu m_O2+generate_CO2; 0.344, 0.658, tmp+interval_yield+interva l_Pdt.+OD+Cell_conc._interval+RQ_integral+AS_Vol.+emission_ CO2+consum_O2+generate_CO2; 0.354, 0.658, tmp_cumulo+pH_cum ulo+AN_cumulo+interval_yield+interval_Pdt.+RQ_integral+AS_V ol.+consum_O2+generate_CO2; 0.334, 0.658, tmp_cumulo+pH_cum ulo+interval_yield+OD+RS_cumulo+rab_integral+RQ_integral+AS _Vol.+emission_O2+consum_O2+generate_CO2; 0.334, 0.658, tmp +tmp_cumulo+pH_cumulo+interval_yield+OD+rab_integral+RQ_int egral+AS_Vol.+emission_CO2+consum_O2+generate_CO2; 0.344, 0. 658, tmp+pH_cumulo+AN_cumulo+interval_yield+interval_Pdt.+R Q_integral+AS_Vol.+emission_CO2+consum_O2+generate_CO2; 0.3 64, 0.658, tmp+interval_yield+interval_Pdt.+OD+RQ_integral+ emission_CO2+consum_O2+generate_CO2; 0.334, 0.658, tmp+tmp_ cumulo+pH_cumulo+interval_yield+interval_Pdt.+OD+Feed_Vol+R Q_integral+AS_Vol.+consum_O2+generate_CO2; 0.364, 0.658, tm p_cumulo+pH+interval_yield+OD+RQ_integral+AS_Vol.+consum_O2 +generate_CO2; 0.334, 0.658, tmp+pH_cumulo+AN+interval_yiel d+interval_Pdt.+OD+RQ_integral+AS_Vol.+emission_CO2+consum O2+generate_CO2; 0.354, 0.658, tmp_cumulo+interval_yield+in terval_Pdt.+RQ_integral+AS_Vol.+emission_O2+emission_CO2+co nsum_O2+generate_CO2; 0.344, 0.658, tmp_cumulo+pH+pH_cumulo +interval_yield+interval_Pdt.+OD+RS_cumulo+RQ_integral+cons um_O2+generate_CO2; 0.334, 0.658, tmp+pH_cumulo+interval_yi eld+interval_Pdt.+OD+Feed_Vol+RS_cumulo+RQ_integral+AS_Vol. +consum_O2+generate_CO2; 0.354, 0.658, tmp_cumulo+pH_cumulo +interval_yield+OD+Feed_Vol+rab_integral+RQ_integral+consum _O2+generate_CO2; 0.354, 0.658, tmp_cumulo+pH_cumulo+interv al_yield+interval_Pdt.+OD+Feed_Vol+RQ_integral+consum_O2+ge nerate_CO2; 0.334, 0.658, tmp_cumulo+AN+interval_yield+inte rval_Pdt.+OD+RS_cumulo+rab_integral+RQ_integral+emission_CO 2+consum_O2+generate_CO2; 0.354, 0.658, tmp_cumulo+pH_cumul o+interval_yield+OD+RS_cumulo+Cell_conc._interval+RQ_integr al+consum_O2+generate_CO2; 0.333, 0.658, tmp+pH_cumulo+AN_c umulo+interval_yield+interval_Pdt.+OD+RQ_integral+AS_Vol.+e mission_CO2+consum_O2+generate_CO2; 0.354, 0.658, tmp+pH_cu mulo+interval_yield+OD+Feed_Vol+RQ_integral+AS_Vol.+consum_ O2+generate_CO2; 0.333, 0.658, tmp_cumulo+pH_cumulo+interva l_yield+interval_Pdt.+RQ_integral+AS_Vol.+emission_O2+emiss ion_CO2+consum_O2+interval_O2+generate_CO2; 0.344, 0.658, t mp_cumulo+pH+interval_yield+interval_Pdt.+RQ_integral+AS_Vo l.+emission_CO2+consum_O2+interval_O2+generate_CO2; 0.373, 0.658, tmp_cumulo+interval_yield+interval_Pdt.+RQ_integral+ AS_Vol.+consum_O2+generate_CO2; 0.323, 0.658, tmp+pH_cumulo +interval_yield+interval_Pdt.+OD+Feed_Vol+RQ_integral+AS_Vo l.+emission_CO2+consum_O2+interval_O2+generate_CO2; 0.333, 0.658, tmp+pH_cumulo+PL_cumulo+interval_yield+interval_Pdt. +OD+Feed_Vol+RQ_integral+AS_Vol.+consum_O2+generate_CO2; 0. 344, 0.658, tmp_cumulo+pH_cumulo+AN+interval_yield+OD+RS_cu mulo+rab_integral+RQ_integral+consum_O2+generate_CO2; 0.373, 0.658, tmp_cumulo+pH_cumulo+RQ_integral+AS_Vol.+consum_O2+ interval_O2+generate_CO2; 0.333, 0.658, tmp_cumulo+pH_cumul o+interval_yield+OD+Feed_Vol+rab_integral+RQ_integral+AS_Vo l.+emission_O2+consum_O2+generate_CO2; 0.333, 0.658, tmp_cu mulo+pH_cumulo+PL_cumulo+interval_yield+OD+RQ_integral+AS_V ol.+emission_CO2+consum_O2+interval_O2+generate_CO2; 0.333, 0.658, tmp_cumulo+pH+interval_yield+interval_Pdt.+OD+RS_cu mulo+rab_integral+RQ_integral+emission_CO2+consum_O2+genera te_CO2; 0.344, 0.658, tmp_cumulo+interval_yield+interval_Pd t.+OD+Feed_Vol+RS_cumulo+RQ_integral+emission_CO2+consum_O2 +generate_CO2; 0.364, 0.658, tmp+interval_yield+interval_Pd t.+Feed_Vol+RQ_integral+emission_CO2+consum_O2+generate_CO 2; 0.344, 0.658, tmp+pH_cumulo+PL_cumulo+interval_yield+int erval_Pdt.+OD+RQ_integral+AS_Vol.+consum_O2+generate_CO2; 0. 333, 0.658, tmp+tmp_cumulo+pH_cumulo+interval_yield+OD+RS_c umulo+RQ_integral+AS_Vol.+consum_O2+interval_O2+generate_CO 2; 0.344, 0.658, tmp_cumulo+pH_cumulo+interval_yield+interv al_Pdt.+Cell_conc._interval+RQ_integral+AS_Vol.+emission_CO 2+consum_O2+generate_CO2; 0.344, 0.658, tmp+pH+interval_yie ld+interval_Pdt.+OD+RQ_integral+AS_Vol.+emission_CO2+consum _O2+generate_CO2; 0.344, 0.658, tmp_cumulo+pH_cumulo+interv al_yield+OD+RS_cumulo+rab_integral+RQ_integral+emission_CO2 +consum_O2+generate_CO2; 0.333, 0.658, tmp_cumulo+pH_cumulo +AN+interval_yield+OD+RS_cumulo+rab_integral+RQ_integral+AS _Vol.+consum_O2+generate_CO2; 0.354, 0.658, tmp_cumulo+inte rval_yield+interval_Pdt.+Cell_conc._interval+RQ_integral+AS _Vol.+emission_CO2+consum_O2+generate_CO2; 0.333, 0.658, tm p_cumulo+pH_cumulo+interval_yield+interval_Pdt.+rab_integra 1+RQ_integral+AS_Vol.+emission_O2+consum_O2+interval_O2+gen erate_CO2; 0.344, 0.658, tmp_cumulo+pH_cumulo+interval_yiel d+interval_Pdt.+OD+RS_cumulo+RQ_integral+consum_O2+interval _O2+generate_CO2; 0.354, 0.658, tmp_cumulo+AN+interval_yiel d+interval_Pdt.+RQ_integral+AS_Vol.+emission_CO2+consum_O2+ generate_CO2; 0.344, 0.658, tmp_cumulo+interval_yield+inter val_Pdt.+RS_cumulo+Cell_conc._interval+rab_integral+RQ_inte gral+emission_CO2+consum_O2+generate_CO2; 0.333, 0.658, tmp +tmp_cumulo+pH_cumulo+interval_yield+interval_Pdt.+OD+RS_cu mulo+RQ_integral+AS_Vol.+consum_O2+generate_CO2; 0.363, 0.6 57, tmp_cumulo+pH_cumulo+interval_yield+OD+Cell_conc._inter val+RQ_integral+consum_O2+generate_CO2; 0.322, 0.657, tmp_c umulo+pH_cumulo+interval_yield+interval_Pdt.+OD+FeedVol+Ce ll_conc._interval+RQ_integral+AS_Vol.+consum_O2+interval_O2 +generate_CO2; 0.343, 0.657, tmp_cumulo+PL_cumulo+interval_ yield+interval_Pdt.+OD+RS_cumulo+RQ_integral+emission_CO2+c onsum_O2+generate_CO2; 0.333, 0.657, tmp_cumulo+interval_yi eld+interval_Pdt.+OD+Feed_Vol+RS_cumulo+RQ_integral+AS_Vol. +emission_CO2+consum_O2+generate_CO2; 0.322, 0.657, tmp+pH_ cumulo+AN_cumulo+interval_yield+interval_Pdt.+OD+Feed_Vol+R Q_integral+AS_Vol.+consum_O2+interval_O2+generate_CO2; 0.35 4, 0.657, tmp_cumulo+pH_cumulo+interval_yield+interval_Pdt. +OD+RQ_integral+emission_CO2+consum_O2+generate_CO2; 0.333, 0.657, tmp+tmp_cumulo+pH_cumulo+AN_cumulo+interval_yield+O D+RQ_integral+AS_Vol.+consum_O2+interval_O2+generate_CO2; 0. 343, 0.657, tmp_cumulo+pH+interval_yield+interval_Pdt.+OD+R Q_integral+AS_Vol.+consum_O2+interval_O2+generate_CO2; 0.33 3, 0.657, tmp_cumulo+pH+pH_cumulo+interval_yield+OD+Cell_co nc._interval+RQ_integral+AS_Vol.+consum_O2+interval_O2+gene rate_CO2; 0.322, 0.657, tmp_cumulo+pH_cumulo+interval_yield +interval_Pdt.+OD+RS_cumulo+RQ_integral+AS_Vol.+emission_CO 2+consum_O2+interval_O2+generate_CO2; 0.322, 0.657, tmp+tmp _cumulo+pH_cumulo+interval_yield+interval_Pdt.+OD+Feed_Vol+ RQ_integral+AS_Vol.+consum_O2+interval_O2+generate_CO2; 0.3 53, 0.657, tmp+interval_yield+interval_Pdt.+Feed_Vol+RQ_int egral+AS_Vol.+consum_O2+interval_O2+generate_CO2; 0.343, 0. 657, tmp+AN+interval_yield+interval_Pdt.+RS_cumulo+rab_inte gral+RQ_integral+emission_CO2+consum_O2+generate_CO2; 0.382, 0.657, tmp+interval_yield+interval_Pdt.+RQ_integral+consum _O2+generate_CO2; 0.322, 0.657, tmp_cumulo+pH_cumulo+AN_cum ulo+interval_yield+interval_Pdt.+OD+rab_integral+RQ_integra l+AS_Vol.+consum_O2+interval_O2+generate_CO2; 0.353, 0.657, tmp+tmp_cumulo+interval_yield+interval_Pdt.+RQ_integral+AS _Vol.+emission_CO2+consum_O2+generate_CO2; 0.333, 0.657, tm p+pH_cumulo+AN_cumulo+interval_yield+interval_Pdt.+OD+Feed_ Vol+RQ_integral+AS_Vol.+consum_O2+generate_CO2; 0.333, 0.65 7, tmp_cumulo+pH_cumulo+AN+interval_yield+interval_Pdt.+OD+ RS_cumulo+RQ_integral+emission_CO2+consum_O2+generate_CO2; 0.333, 0.657, tmp_cumulo+pH_cumulo+PL_cumulo+interval_yield +OD+RS_cumulo+RQ_integral+AS_Vol.+consum_O2+interval_O2+gen erate_CO2; 0.343, 0.657, tmp+AN_cumulo+interval_yield+inter val_Pdt.+RS_cumulo+rab_integral+RQ_integral+emission_CO2+co nsum_O2+generate_CO2; 0.333, 0.657, tmp_cumulo+pH_cumulo+in terval_yield+interval_Pdt.+Feed_Vol+rab_integral+RQ_integra l+AS_Vol.+consum_O2+interval_O2+generate_CO2; 0.333, 0.657, tmp+tmp_cumulo+pH_cumulo+interval_yield+interval_Pdt.+OD+R Q_integral+AS_Vol.+emission_O2+consum_O2+generate_CO2; 0.31 1, 0.657, tmp_cumulo+pH_cumulo+interval_yield+interval_Pdt. +OD+Feed_Vol+rab_integral+RQ_integral+AS_Vol.+emission_CO2+ consum_O2+interval_O2+generate_CO2; 0.333, 0.657, tmp+tmp_c umulo+pH_cumulo+interval_yield+OD+Cell_conc._interval+RQ_in tegral+AS_Vol.+consum_O2+interval_O2+generate_CO2; 0.333, 0. 657, tmp+pH_cumulo+interval_yield+interval_Pdt.+OD+RQ_integ ral+AS_Vol.+emission_CO2+consum_O2+interval_O2+generate_CO 2; 0.322, 0.657, tmp_cumulo+pH_cumulo+interval_yield+OD+Fee d_Vol+rab_integral+RQ_integral+AS_Vol.+emission_O2+consum_O 2+interval_O2+generate_CO2; 0.333, 0.657, tmp_cumulo+pH_cum ulo+AN_cumulo+interval_yield+interval_Pdt.+OD+RS_cumulo+RQ_ integral+AS_Vol.+consum_O2+generate_CO2; 0.373, 0.657, tmp+ interval_yield+interval_Pdt.+RQ_integral+emission_CO2+consu m_O2+generate_CO2; 0.343, 0.657, tmp+pH_cumulo+interval_yie ld+interval_Pdt.+OD+rab_integral+RQ_integral+emission_CO2+c onsum_O2+generate_CO2; 0.353, 0.657, tmp_cumulo+pH_cumulo+i nterval_yield+OD+Feed_Vol+RS_cumulo+RQ_integral+consum_O2+g enerate_CO2; 0.343, 0.657, tmp_cumulo+pH+pH_cumulo+interval _yield+OD+RS_cumulo+rab_integral+RQ_integral+consum_O2+gene rate_CO2; 0.333, 0.657, tmp_cumulo+pH_cumulo+PL_cumulo+inte rval_yield+OD+rab_integral+RQ_integral+AS_Vol.+emission_CO2 +consum_O2+generate_CO2; 0.343, 0.657, tmp+interval_yield+i nterval_Pdt.+OD+rab_integral+RQ_integral+AS_Vol.+emission_C O2+consum_O2+generate_CO2; 0.343, 0.657, tmp_cumulo+pH_cumu lo+PL+interval_yield+OD+RS_cumulo+RQ_integral+AS_Vol.+consu m_O2+generate_CO2; 0.322, 0.657, tmp_cumulo+pH_cumulo+PL_cu mulo+interval_yield+interval_Pdt.+OD+rab_integral+RQ_integr al+AS_Vol.+emission_CO2+consum_O2+generate_CO2; 0.343, 0.65 7, tmp+tmp_cumulo+pH_cumulo+interval_yield+interval_Pdt.+RQ _integral+AS_Vol.+emission_CO2+consum_O2+generate_CO2; 0.32 2, 0.657, tmp_cumulo+pH_cumulo+AN+interval_yield+OD+rab_int egral+RQ_integral+AS_Vol.+emission_O2+consum_O2+interval_O2 +generate_CO2; 0.333, 0.657, tmp_cumulo+pH_cumulo+AN+interv al_yield+interval_Pdt.+RQ_integral+AS_Vol.+emission_O2+cons um_O2+interval_O2+generate_CO2; 0.353, 0.657, tmp_cumulo+pH +interval_yield+interval_Pdt.+RS_cumulo+RQ_integral+AS_Vol. +consum_O2+generate_CO2; 0.353, 0.657, tmp_cumulo+pH_cumulo +interval_yield+OD+Feed_Vol+RQ_integral+consum_O2+interval O2+generate_CO2; 0.353, 0.657, tmp+interval_yield+interval_ Pdt.+RQ_integral+Accum._Yield+AS_Vol.+emission_CO2+consum_O 2+generate_CO2; 0.322, 0.657, tmp_cumulo+pH_cumulo+interval _yield+OD+Feed_Vol+RS_cumulo+rab_integral+RQ_integral+AS_Vo l.+consum_O2+interval_O2+generate_CO2; 0.343, 0.657, tmp+pH _cumulo+interval_yield+interval_Pdt.+Cell_conc._interval+RQ _integral+AS_Vol.+emission_CO2+consum_O2+generate_CO2; 0.34 3, 0.657, tmp_cumulo+pH_cumulo+interval_yield+interval_Pdt. +OD+RQ_integral+Accum._Yield+AS_Vol.+consum_O2+generate_CO 2; 0.343, 0.657, tmp_cumulo+interval_yield+interval_Pdt.+OD +RS_cumulo+Cell_conc._interval+RQ_integral+emission_CO2+con sum_O2+generate_CO2; 0.333, 0.657, tmp+tmp_cumulo+pH_cumulo +interval_yield+OD+Feed_Vol+RQ_integral+AS_Vol.+consum_O2+i nterval_O2+generate_CO2; 0.333, 0.657, tmp_cumulo+pH_cumulo +PL_cumulo+interval_yield+interval_Pdt.+RQ_integral+AS_Vol. +emission_CO2+consum_O2+interval_O2+generate_CO2; 0.322, 0. 657, tmp_cumulo+pH+pH_cumulo+interval_yield+OD+rab_integral +RQ_integral+AS_Vol.+emission_CO2+consum_O2+interval_O2+gen erate_CO2; 0.343, 0.657, tmp_cumulo+pH+pH_cumulo+PL_cumulo+ interval_yield+OD+RQ_integral+AS_Vol.+consum_O2+generate_CO 2; 0.343, 0.657, tmp+interval_yield+interval_Pdt.+Cell_conc. _interval+rab_integral+RQ_integral+AS_Vol.+emission_CO2+con sum_O2+generate_CO2; 0.332, 0.657, tmp_cumulo+pH+pH_cumulo+ AN+interval_yield+OD+rab_integral+RQ_integral+AS_Vol.+consu m_O2+generate_CO2; 0.363, 0.657, tmp_cumulo+interval_yield+ interval_Pdt.+Feed_Vol+RQ_integral+emission_CO2+consum_O2+g enerate_CO2; 0.343, 0.657, tmp_cumulo+pH_cumulo+PL_cumulo+i nterval_yield+OD+RQ_integral+AS_Vol.+emission_O2+consum_O2+ generate_CO2; 0.332, 0.657, tmp_cumulo+AN+interval_yield+in terval_Pdt.+OD+RS_cumulo+RQ_integral+AS_Vol.+emission_CO2+c onsum_O2+generate_CO2; 0.353, 0.657, tmp_cumulo+AN_cumulo+i nterval_yield+interval_Pdt.+RQ_integral+AS_Vol.+emission_CO 2+consum_O2+generate_CO2; 0.332, 0.657, tmp_cumulo+pH_cumul o+AN_cumulo+interval_yield+OD+rab_integral+RQ_integral+AS_V ol.+emission_O2+consum_O2+generate_CO2; 0.343, 0.657, tmp_c umulo+pH_cumulo+PL_cumulo+interval_yield+interval_Pdt.+OD+R S_cumulo+RQ_integral+consum_O2+generate_CO2; 0.332, 0.657, tmp_cumulo+pH+pH_cumulo+interval_yield+OD+Feed_Vol+rab_inte gral+RQ_integral+AS_Vol.+consum_O2+generate_CO2; 0.310, 0.6 57, tmp_cumulo+pH_cumulo+AN+interval_yield+interval_Pdt.+OD +Feed_Vol+rab_integral+RQ_integral+AS_Vol.+consum_O2+interv al_O2+generate_CO2; 0.322, 0.657, tmp_cumulo+pH_cumulo+AN+i nterval_yield+interval_Pdt.+rab_integral+RQ_integral+AS_Vol. +emission_CO2+consum_O2+interval_O2+generate_CO2; 0.332, 0. 657, tmp_cumulo+pH_cumulo+PL_cumulo+interval_yield+interval _Pdt.+OD+RS_cumulo+RQ_integral+AS_Vol.+consum_O2+generate_C 02; 0.353, 0.657, tmp_cumulo+pH_cumulo+AN+interval_yield+OD +Feed_Vol+RQ_integral+consum_O2+generate_CO2; 0.322, 0.657, tmp_cumulo+pH_cumulo+interval_yield+OD+Feed_Vol+Cell_conc. interval+rab_integral+RQ_integral+AS_Vol.+consum_O2+interv al_O2+generate_CO2; 0.343, 0.657, tmp+pH_cumulo+interval_yi eld+interval_Pdt.+OD+RS_cumulo+RQ_integral+emission_CO2+con sum_O2+generate_CO2; 0.322, 0.657, tmp_cumulo+pH_cumulo+int erval_yield+interval_Pdt.+OD+Cell_conc._interval+RQ_integra 1+AS_Vol.+emission_CO2+consum_O2+interval_O2+generate_CO2; 0.353, 0.657, tmp_cumulo+pH_cumulo+interval_yield+Feed_Vol+ RQ_integral+AS_Vol.+consum_O2+interval_O2+generate_CO2; 0.3 32, 0.657, tmp_cumulo+pH+interval_yield+interval_Pdt.+OD+ra b_integral+RQ_integral+AS_Vol.+emission_CO2+consum_O2+gener ate_CO2; 0.321, 0.657, tmp_cumulo+pH_cumulo+AN+interval_yie ld+interval_Pdt.+OD+RS_cumulo+RQ_integral+AS_Vol.+emission_ CO2+consum_O2+generate_CO2; 0.332, 0.657, tmp_cumulo+pH+pH_ cumulo+interval_yield+OD+rab_integral+RQ_integral+AS_Vol.+e mission_O2+consum_O2+generate_CO2; 0.353, 0.657, tmp+pH_cum ulo+interval_yield+interval_Pdt.+OD+Cell_conc._interval+RQ_ integral+consum_O2+generate_CO2; 0.363, 0.657, tmp+pH_cumul o+interval_yield+OD+RQ_integral+consum_O2+interval_O2+gener ate_CO2; 0.343, 0.657, tmp+tmp_cumulo+interval_yield+interv al_Pdt.+RS_cumulo+RQ_integral+emission_O2+emission_CO2+cons um_O2+generate_CO2; 0.332, 0.657, tmp_cumulo+pH_cumulo+AN+i nterval_yield+interval_Pdt.+RS_cumulo+RQ_integral+AS_Vol.+e mission_CO2+consum_O2+generate_CO2; 0.321, 0.657, tmp_cumul o+pH+pH_cumulo+interval_yield+OD+Feed_Vol+rab_integral+RQ_i ntegral+AS_Vol.+consum_O2+interval_O2+generate_CO2; 0.321, 0.657, tmp_cumulo+pH_cumulo+interval_yield+OD+rab_integral+ RQ_integral+AS_Vol.+emission_O2+emission_CO2+consum_O2+inte rval_O2+generate_CO2; 0.332, 0.657, tmp+tmp_cumulo+pH_cumul o+interval_yield+OD+RQ_integral+AS_Vol.+emission_O2+consum_ O2+interval_O2+generate_CO2; 0.343, 0.657, tmp+pH_cumulo+in terval_yield+interval_Pdt.+RS_cumulo+RQ_integral+AS_Vol.+em ission_CO2+consum_O2+generate_CO2; 0.332, 0.657, tmp_cumulo +pH_cumulo+interval_yield+interval_Pdt.+Feed_Vol+RQ_integra 1+AS_Vol.+emission_O2+consum_O2+interval_O2+generate_CO2; 0. 353, 0.657, tmp_cumulo+pH_cumulo+OD+rab_integral+RQ_integra l+AS_Vol.+consum_O2+interval_O2+generate_CO2; 0.353, 0.657, tmp+interval_yield+interval_Pdt.+Feed_Vol+RS_cumulo+RQ_int egral+emission_CO2+consum_O2+generate_CO2; 0.321, 0.657, tm p_cumulo+pH_cumulo+AN+interval_yield+interval_Pdt.+OD+RQ_in tegral+AS_Vol.+emission_O2+consum_O2+interval_O2+generate_C 02; 0.362, 0.657, tmp_cumulo+interval_yield+interval_Pdt.+R S_cumulo+Cell_conc._interval+RQ_integral+consum_O2+generate _CO2; 0.353, 0.657, tmp_cumulo+interval_yield+interval_Pdt. +RS_cumulo+rab_integral+RQ_integral+AS_Vol.+consum_O2+gener ate_CO2; 0.332, 0.657, tmp+tmp_cumulo+interval_yield+interv al_Pdt.+OD+RS_cumulo+RQ_integral+AS_Vol.+emission_CO2+consu m_O2+generate_CO2; 0.321, 0.657, tmp_cumulo+pH_cumulo+inter val_yield+interval_Pdt.+OD+RS_cumulo+rab_integral+RQ_integr al+emission_O2+emission_CO2+consum_O2+generate_CO2; 0.332, 0.657, tmp_cumulo+pH_cumulo+interval_yield+interval_Pdt.+Fe ed_Vol+RS_cumulo+RQ_integral+AS_Vol.+emission_CO2+consum_O2 +generate_CO2; 0.342, 0.657, tmp+tmp_cumulo+pH_cumulo+PL_cu mulo+interval_yield+OD+RQ_integral+AS_Vol.+consum_O2+genera te_CO2; 0.332, 0.657, tmp+pH_cumulo+interval_yield+interval _Pdt.+rab_integral+RQ_integral+AS_Vol.+emission_O2+emission _CO2+consum_O2+generate_CO2; 0.353, 0.657, tmp_cumulo+pH_cu mulo+interval_Pdt.+OD+RQ_integral+AS_Vol.+consum_O2+interva l_O2+generate_CO2; 0.321, 0.657, tmp_cumulo+pH_cumulo+AN+in terval_yield+interval_Pdt.+OD+Feed_Vol+rab_integral+RQ_inte gral+AS_Vol.+consum_O2+generate_CO2; 0.332, 0.657, tmp_cumu lo+pH_cumulo+AN+interval_yield+interval_Pdt.+rab_integral+R Q_integral+AS_Vol.+consum_O2+interval_O2+generate_CO2; 0.34 2, 0.657, tmp+pH_cumulo+interval_yield+OD+Feed_Vol+RQ_integ ral+AS_Vol.+consum_O2+interval_O2+generate_CO2; 0.321, 0.65 7, tmp_cumulo+pH_cumulo+interval_yield+OD+Cell_conc._interv al+rab_integral+RQ_integral+AS_Vol.+emission_CO2+consum_O2+ interval_O2+generate_CO2; 0.342, 0.657, tmp+AN_cumulo+inter val_yield+interval_Pdt.+RS_cumulo+RQ_integral+AS_Vol.+emiss ion_CO2+consum_O2+generate_CO2; 0.332, 0.657, tmp_cumulo+AN _cumulo+interval_yield+interval_Pdt.+OD+RS_cumulo+rab_integ ral+RQ_integral+emission_CO2+consum _O2+generate_CO2; 0.352, 0.657, tmp+pH_cumulo+PL_cumulo+interval_yield+OD+RQ_integr al+AS_Vol.+consum_O2+generate_CO2; 0.321, 0.657, tmp_cumulo +interval_yield+interval_Pdt.+OD+RS_cumulo+rab_integral+RQ_ integral+AS_Vol.+emission_O2+emission_CO2+consum_O2+generat e_CO2; 0.321, 0.657, tmp_cumulo+pH+pH_cumulo+AN+interval_yi eld+OD+rab_integral+RQ_integral+AS_Vol.+consum_O2+interval_ O2+generate_CO2; 0.332, 0.657, tmp_cumulo+pH_cumulo+PL_cumu lo+interval_yield+OD+Cell_conc._interval+RQ_integral+AS_Vol. +consum_O2+interval_O2+generate_CO2; 0.332, 0.657, tmp_cumu lo+pH_cumulo+PL_cumulo+interval_yield+OD+RQ_integral+AS_Vol. +emission_O2+consum _O2+interval_O2+generate_CO2; 0.332, 0.6 57, tmp_cumulo+pH_cumulo+interval_yield+OD+Cell_conc._inter val+rab_integral+RQ_integral+AS_Vol.+emission_O2+consum_O2+ generate_CO2; 0.332, 0.657, tmp_cumulo+pH_cumulo+AN+interva l_yield+OD+Cell_conc._interval+rab_integral+RQ_integral+AS_ Vol.+consum_O2+generate_CO2; 0.342, 0.657, tmp_cumulo+pH+in terval_yield+interval_Pdt.+RS_cumulo+RQ_integral+emission_O 2+emission_CO2+consum_O2+generate_CO2; 0.332, 0.657, tmp_cu mulo+pH_cumulo+PL_cumulo+interval_yield+OD+Feed_Vol+rab_int egral+RQ_integral+AS_Vol.+consum_O2+generate_CO2; 0.332, 0. 657, tmp+pH_cumulo+interval_yield+interval_Pdt.+OD+Feed_Vol +Cell_conc._interval+RQ_integral+AS_Vol.+consum_O2+generate _CO2; 0.342, 0.657, tmp_cumulo+interval_yield+interval_Pdt. +Feed_Vol+RS_cumulo+RQ_integral+AS_Vol.+consum_O2+interval_ O2+generate_CO2; 0.352, 0.657, tmp+pH_cumulo+interval_yield +interval_Pdt.+OD+RQ_integral+consum_O2+interval_O2+generat e_CO2; 0.342, 0.657, tmp+AN+interval_yield+interval_Pdt.+RS _cumulo+RQ_integral+AS_Vol.+emission_CO2+consum_O2+generate _CO2; 0.342, 0.657, tmp_cumulo+pH_cumulo+PL+interval_yield+ OD+Feed_Vol+RQ_integral+AS_Vol.+consum_O2+generate_CO2; 0.3 32, 0.656, tmp_cumulo+interval_yield+interval_Pdt.+Feed_Vol +RS_cumulo+RQ_integral+AS_Vol.+emission_O2+emission_CO2+con sum_O2+generate_CO2; 0.332, 0.656, tmp+tmp_cumulo+pH_cumulo +interval_yield+OD+RQ_integral+AS_Vol.+emission_CO2+consum_ O2+interval_O2+generate_CO2; 0.342, 0.656, tmp+AN+interval_ yield+interval_Pdt.+Cell_conc._interval+RQ_integral+AS_Vol. +emission_CO2+consum_O2+generate_CO2; 0.332, 0.656, tmp+tmp _cumulo+interval_yield+interval_Pdt.+OD+RS_cumulo+rab_integ ral+RQ_integral+emission_CO2+consum_O2+generate_CO2; 0.362, 0.656, tmp_cumulo+interval_yield+OD+RS_cumulo+RQ_integral+ consum_O2+interval_O2+generate_CO2; 0.332, 0.656, tmp_cumul o+pH_cumulo+PL_cumulo+interval_yield+interval_Pdt.+OD+Feed_ Vol+RQ_integral+AS_Vol.+consum_O2+generate_CO2; 0.332, 0.65 6, tmp_cumulo+pH+pH_cumulo+interval_yield+OD+RS_cumulo+rab_ integral+RQ_integral+AS_Vol.+consum_O2+generate_CO2; 0.332, 0.656, tmp_cumulo+interval_yield+interval_Pdt.+RS_cumulo+r ab_integral+RQ_integral+AS_Vol.+emission_CO2+consum_O2+inte rval_O2+generate_CO2; 0.342, 0.656, tmp+interval_yield+inte rval_Pdt.+OD+RQ_integral+AS_Vol.+emission_O2+emission_CO2+c onsum_O2+generate_CO2; 0.362, 0.656, tmp+pH_cumulo+interval _yield+OD+rab_integral+RQ_integral+consum_O2+generate_CO2; 0.332, 0.656, tmp_cumulo+pH_cumulo+AN+interval_yield+interv al_Pdt.+OD+RS_cumulo+rab_integral+RQ_integral+consum_O2+gen erate_CO2; 0.332, 0.656, tmp_cumulo+pH+pH_cumulo+PL_cumulo+ interval_yield+OD+RQ_integral+AS_Vol.+consum_O2+interval_O2 +generate_CO2; 0.362, 0.656, tmp_cumulo+pH_cumulo+OD+rab_in tegral+RQ_integral+AS_Vol.+consum_O2+generate_CO2; 0.321, 0. 656, tmp_cumulo+pH_cumulo+interval_yield+interval_Pdt.+OD+F eed_Vol+rab_integral+RQ_integral+AS_Vol.+emission_O2+consum _O2+generate_CO2; 0.310, 0.656, tmp_cumulo+pH_cumulo+interv al_yield+interval_Pdt.+OD+RS_cumulo+rab_integral+RQ_integra l+AS_Vol.+emission_O2+emission_CO2+consum_O2+generate_CO2; 0.331, 0.656, tmp+pH_cumulo+interval_yield+interval_Pdt.+OD +Feed_Vol+RQ_integral+AS_Vol.+emission_O2+consum_O2+generat e_CO2; 0.331, 0.656, tmp_cumulo+pH_cumulo+AN+interval_yield +interval_Pdt.+OD+Cell_conc._interval+RQ_integral+AS_Vol.+c onsum_O2+generate_CO2; 0.331, 0.656, tmp_cumulo+pH+pH_cumul o+interval_yield+interval_Pdt.+OD+RQ_integral+AS_Vol.+emiss ion_O2+consum_O2+generate_CO2; 0.321, 0.656, tmp_cumulo+pH_ cumulo+interval_yield+OD+RS_cumulo+rab_integral+RQ_integral +AS_Vol.+emission_CO2+consum_O2+interval_O2+generate_CO2; 0. 352, 0.656, tmp_cumulo+pH_cumulo+interval_yield+interval_Pd t.+OD+RQ_integral+consum_O2+interval_O2+generate_CO2; 0.321, 0.656, tmp_cumulo+pH+pH_cumulo+interval_yield+OD+rab_integ ral+RQ_integral+AS_Vol.+emission_O2+consum_O2+interval_O2+g enerate_CO2; 0.352, 0.656, tmp_cumulo+pH_cumulo+interval_yi eld+OD+rab_integral+RQ_integral+consum_O2+interval_O2+gener ate_CO2; 0.331, 0.656, tmp_cumulo+AN_cumulo+interval_yield+ interval_Pdt.+OD+RS_cumulo+RQ_integral+AS_Vol.+emission_CO2 +consum_O2+generate_CO2; 0.342, 0.656, tmp_cumulo+interval_ yield+interval_Pdt.+Feed_Vol+RS_cumulo+RQ_integral+emission _O2+emission_CO2+consum_O2+generate_CO2; 0.321, 0.656, tmp_ cumulo+pH_cumulo+interval_yield+interval_Pdt.+OD+Feed_Vol+R Q_integral+AS_Vol.+emission_O2+emission_CO2+consum_O2+gener ate_C02; 0.342, 0.656, tmp+AN_cumulo+interval_yield+interva l_Pdt.+OD+RS_cumulo+RQ_integral+emission_CO2+consum_O2+gene rate_CO2; 0.352, 0.656, tmp_cumulo+AN_cumulo+interval_yield +interval_Pdt.+RS_cumulo+RQ_integral+emission_O2+consum_O2+ generate_CO2; 0.352, 0.656, tmp+pH_cumulo+interval_yield+in terval_Pdt.+RS_cumulo+RQ_integral+emission_CO2+consum_O2+ge nerate_CO2; 0.321, 0.656, tmp_cumulo+pH+pH_cumulo+interval_ yield+interval_Pdt.+OD+rab_integral+RQ_integral+AS_Vol.+con sum_O2+interval_O2+generate_CO2; 0.331, 0.656, tmp_cumulo+p H_cumulo+interval_yield+interval_Pdt.+Cell_conc._interval+r ab_integral+RQ_integral+AS_Vol.+emission_CO2+consum_O2+gene rate_CO2; 0.321, 0.656, tmp_cumulo+pH_cumulo+interval_yield +interval_Pdt.+RS_cumulo+rab_integral+RQ_integral+AS_Vol.+e mission_O2+emission_CO2+consum_O2+generate_CO2; 0.331, 0.65 6, tmp+interval_yield+interval_Pdt.+OD+RS_cumulo+rab_integr al+RQ_integral+emission_O2+emission_CO2+consum_O2+generate_ CO2; 0.352, 0.656, tmp_cumulo+AN+interval_yield+interval_Pd t.+RS_cumulo+RQ_integral+emission_O2+consum_O2+generate_CO 2; 0.331, 0.656, tmp+pH_cumulo+AN+interval_yield+interval_P dt.+Feed_Vol+RQ_integral+AS_Vol.+consum_O2+interval_O2+gene rate_CO2; 0.331, 0.656, tmp_cumulo+pH_cumulo+AN_cumulo+inte rval_yield+OD+RS_cumulo+rab_integral+RQ_integral+AS_Vol.+co nsum_O2+generate_CO2; 0.320, 0.656, tmp_cumulo+pH_cumulo+PL _cumulo+interval_yield+interval_Pdt.+OD+RQ_integral+AS_Vol. +emission_CO2+consum_O2+interval_O2+generate_CO2; 0.352, 0. 656, tmp_cumulo+pH_cumulo+interval_yield+OD+RS+rab_integral +RQ_integral+consum_O2+generate_CO2; 0.342, 0.656, tmp_cumu lo+pH_cumulo+PL_cumulo+interval_yield+interval_Pdt.+RQ_inte gral+AS_Vol.+emission_CO2+consum_O2+generate_CO2; 0.352, 0. 656, tmp_cumulo+pH_cumulo+AN+interval_yield+OD+rab_integral +RQ_integral+consum_O2+generate_CO2; 0.342, 0.656, tmp+AN+i nterval_yield+interval_Pdt.+rab_integral+RQ_integral+AS_Vol. +emission_CO2+consum_O2+generate_CO2; 0.342, 0.656, tmp_cum ulo+pH_cumulo+interval_yield+interval_Pdt.+OD+Feed_Vol+RS_c umulo+RQ_integral+consum_O2+generate_CO2; 0.342, 0.656, tmp +pH+interval_yield+interval_Pdt.+rab_integral+RQ_integral+A S_Vol.+emission_CO2+consum_O2+generate_CO2; 0.352, 0.656, t mp_cumulo+pH_cumulo+AN+interval_yield+RQ_integral+AS_Vol.+c onsum_O2+interval_O2+generate_CO2; 0.331, 0.656, tmp_cumulo +pH+pH_cumulo+interval_yield+interval_Pdt.+OD+RS_cumulo+RQ_ integral+AS_Vol.+consum_O2+generate_CO2; 0.331, 0.656, tmp_ cumulo+pH_cumulo+AN_cumulo+interval_yield+interval_Pdt.+RS_ cumulo+RQ_integral+AS_Vol.+emission_CO2+consum_O2+generate_ CO2; 0.361, 0.656, tmp_cumulo+pH+interval_yield+OD+RS_cumul o+RQ_integral+consum_O2+generate_CO2; 0.331, 0.656, tmp_cum ulo+pH_cumulo+interval_yield+OD+RS_cumulo+Cell_conc._interv al+RQ_integral+AS_Vol.+consum_O2+interval_O2+generate_CO2; 0.331, 0.656, tmp_cumulo+AN+interval_yield+interval_Pdt.+RS _cumulo+rab_integral+RQ_integral+emission_O2+emission_CO2+c onsum_O2+generate_CO2; 0.331, 0.656, tmp+tmp_cumulo+pH_cumu lo+interval_yield+OD+Feed_Vol+rab_integral+RQ_integral+AS_V ol.+consum_O2+generate_CO2; 0.341, 0.656, tmp+AN+interval_y ield+interval_Pdt.+OD+RS_cumulo+RQ_integral+emission_CO2+co nsum_O2+generate_CO2; 0.331, 0.656, tmp_cumulo+pH_cumulo+PL +interval_yield+OD+Feed_Vol+RQ_integral+AS_Vol.+consum_O2+i nterval_O2+generate_CO2; 0.341, 0.656, tmp+pH_cumulo+interv al_yield+interval_Pdt.+OD+Cell_conc._interval+RQ_integral+A S_Vol.+consum_O2+generate_CO2; 0.341, 0.656, tmp+interval_y ield+interval_Pdt.+Feed_Vol+rab_integral+RQ_integral+AS_Vol. +emission_CO2+consum_O2+generate_CO2; 0.320, 0.656, tmp+pH_ cumulo+interval_yield+interval_Pdt.+OD+Feed_Vol+RQ_integral +AS_Vol.+emission_O2+consum_O2+interval_O2+generate_CO2; 0. 341, 0.656, tmp_cumulo+AN_cumulo+interval_yield+interval_Pd t.+RS_cumulo+RQ_integral+emission_O2+emission_CO2+consum_O2 +generate_CO2; 0.320, 0.656, tmp+tmp_cumulo+pH_cumulo+inter val_yield+interval_Pdt.+OD+RS_cumulo+RQ_integral+AS_Vol.+em ission_CO2+consum_O2+generate_CO2; 0.341, 0.656, tmp_cumulo +interval_yield+interval_Pdt.+OD+RS_cumulo+RQ_integral+AS_V ol.+emission_O2+consum_O2+generate_CO2; 0.320, 0.656, tmp+p H_cumulo+interval_yield+interval_Pdt.+OD+rab_integral+RQ_in tegral+AS_Vol.+emission_O2+emission_CO2+consum_O2+generate_ CO2; 0.351, 0.656, tmp+PL_cumulo+interval_yield+interval_Pd t.+RS_cumulo+RQ_integral+emission_CO2+consum_O2+generate_CO 2; 0.320, 0.656, tmp_cumulo+pH_cumulo+AN_cumulo+interval_yi eld+OD+rab_integral+RQ_integral+AS_Vol.+emission_O2+consum_ O2+interval_O2+generate_CO2; 0.320, 0.656, tmp_cumulo+pH_cu mulo+AN_cumulo+interval_yield+interval_Pdt.+OD+RS_cumulo+RQ _integral+AS_Vol.+emission_CO2+consum_O2+generate_CO2; 0.34 1, 0.656, tmp_cumulo+pH_cumulo+interval_yield+interval_Pdt. +RS_cumulo+rab_integral+RQ_integral+AS_Vol.+consum_O2+gener ate_CO2; 0.331, 0.656, tmp+tmp_cumulo+pH_cumulo+interval_yi eld+OD+RS_cumulo+rab_integral+RQ_integral+AS_Vol.+consum_O2 +generate_CO2; 0.361, 0.656, tmp+pH_cumulo+interval_yield+O D+RS+RQ_integral+consum_O2+generate_CO2; 0.309, 0.656, tmp_ cumulo+pH_cumulo+AN+interval_yield+interval_Pdt.+OD+rab_int egral+RQ_integral+AS_Vol.+emission_CO2+consuni_O2+interval_O 2+generate_CO2; 0.351, 0.656, tmp_cumulo+pH_cumulo+interval _yield+interval_Pdt.+Cell_conc._interval+RQ_integral+AS_Vol. +consum_O2+generate_CO2; 0.351, 0.656, tmp_cumulo+pH_cumulo +PL_cumulo+interval_yield+interval_Pdt.+RQ_integral+AS_Vol. +consum_O2+generate_CO2; 0.320, 0.656, tmp_cumulo+pH_cumulo +PL_cumulo+interval_yield+OD+Feed_Vol+rab_integral+RQ_integ ral+AS_Vol.+consum_O2+interval_O2+generate_CO2; 0.320, 0.65 6, tmp_cumulo+pH_cumulo+interval_yield+interval_Pdt.+OD+Cel l_conc._interval+rab_integral+RQ_integral+AS_Vol.+consum_O2 +interval_O2+generate_CO2; 0.351, 0.656, tmp_cumulo+pH_cumu lo+interval_yield+interval_Pdt.+RS_cumulo+rab_integral+RQ_i ntegral+consum_O2+generate_CO2; 0.341, 0.656, tmp_cumulo+pH _cumulo+interval_yield+interval_Pdt.+RS_cumulo+RQ_integral+ emission_O2+emission_CO2+consum _O2+generate_CO2; 0.361, 0.6 56, tmp+interval_yield+interval_Pdt.+OD+RS_cumulo+RQ_integr al+consum_O2+generate_CO2; 0.309, 0.656, tmp_cumulo+pH_cumu lo+interval_yield+interval_Pdt.+OD+Feed_Vol+RS_cumulo+rab_i ntegral+RQ_integral+AS_Vol.+emission_CO2+consum_O2+generate _CO2; 0.341, 0.656, tmp+pH_cumulo+AN_cumulo+interval_yield+ interval_Pdt.+OD+RQ_integral+AS_Vol.+consum_O2+generate_CO 2; 0.331, 0.656, tmp+pH_cumulo+interval_yield+interval_Pdt. +Feed_Vol+rab_integral+RQ_integral+AS_Vol.+emission_CO2+con sum_O2+generate_CO2; 0.320, 0.656, tmp_cumulo+pH+pH_cumulo+ AN_cumulo+interval_yield+OD+rab_integral+RQ_integral+AS_Vol. +consum_O2+interval_O2+generate_CO2; 0.351, 0.656, tmp+tmp_ cumulo+pH_cumulo+interval_yield+interval_Pdt.+RQ_integral+A S_Vol.+consum_O2+generate_CO2; 0.370, 0.656, tmp+interval_y ield+interval_Pdt.+OD+RQ_integral+consum_O2+generate_CO2; 0. 331, 0.656, tmp_cumulo+pH_cumulo+PL_cumulo+interval_yield+O D+RS_cumulo+rab_integral+RQ_integral+AS_Vol.+consum_O2+gene rate_CO2; 0.320, 0.656, tmp_cumulo+pH_cumulo+interval_yield +interval_Pdt.+Feed_Vol+rab_integral+RQ_integral+AS_Vol.+em ission_CO2+consum_O2+interval_O2+generate_CO2; 0.330, 0.656, tmp_cumulo+pH_cumulo+interval_yield+interval_Pdt.+Cell_con c._interval+RQ_integral+AS_Vol.+emission_CO2+consum_O2+inte rval_O2+generate_CO2; 0.320, 0.656, tmp_cumulo+pH_cumulo+AN +interval_yield+interval_Pdt.+OD+Feed_Vol+RQ_integral+AS_Vo l.+emission_CO2+consum_O2+generate_CO2; 0.330, 0.656, tmp_c umulo+pH+pH_cumulo+AN_cumulo+interval_yield+OD+rab_integral +RQ_integral+AS_Vol.+consum_O2+generate_CO2; 0.341, 0.656, tmp+AN_cumulo+interval_yield+interval_Pdt.+rab_integral+RQ_ integral+AS_Vol.+emission_CO2+consum_O2+generate_CO2; 0.330, 0.656, tmp+tmp_cumulo+pH_cumulo+interval_yield+interval_Pd t.+OD+Cell_conc._interval+RQ_integral+AS_Vol.+consum_O2+gen erate_CO2; 0.341, 0.656, tmp+interval_yield+interval_Pdt.+R S_cumulo+Cell_conc._interval+RQ_integral+AS_Vol.+emission_C O2+consum_O2+generate_CO2; 0.341, 0.656, tmp+pH_cumulo+AN+i nterval_yield+interval_Pdt.+OD+RQ_integral+emission_CO2+con sum_O2+generate_CO2; 0.361, 0.656, tmp+interval_yield+inter val_Pdt.+RQ_integral+AS_Vol.+emission_O2+consum_O2+generate _CO2; 0.330, 0.656, tmp+interval_yield+interval_Pdt.+Feed_V ol+RS_cumulo+rab_integral+RQ_integral+AS_Vol.+emission_CO2+ consum_O2+generate_CO2; 0.320, 0.656, tmp_cumulo+pH_cumulo+ PL_cumulo+interval_yield+interval_Pdt.+OD+Feed_Vol+RQ_integ ral+AS_Vol.+consum_O2+interval_O2+generate_CO2; 0.320, 0.65 6, tmp_cumulo+pH_cumulo+AN+interval_yield+interval_Pdt.+OD+ RQ_integral+AS_Vol.+emission_O2+emission_CO2+consum_O2+gene rate_CO2; 0.330, 0.656, tmp_cumulo+pH_cumulo+PL_cumulo+AN+i nterval_yield+interval_Pdt.+OD+RQ_integral+AS_Vol.+consum_O 2+generate_CO2; 0.341, 0.656, tmp_cumulo+interval_yield+int erval_Pdt.+OD+rab_integral+RQ_integral+AS_Vol.+emission_CO2 +consum_O2+generate_CO2; 0.330, 0.656, tmp+pH_cumulo+interv al_yield+interval_Pdt.+OD+RS_cumulo+RQ_integral+AS_Vol.+emi ssion_CO2+consum_O2+generate_CO2; 0.341, 0.656, tmp_cumulo+ pH+interval_yield+interval_Pdt.+OD+RS_cumulo+RQ_integral+AS _Vol.+consum_O2+generate_CO2; 0.351, 0.656, tmp+pH_cumulo+i nterval_yield+interval_Pdt.+OD+RS_cumulo+RQ_integral+consum _O2+generate_CO2; 0.330, 0.656, tmp+tmp_cumulo+pH_cumulo+AN +interval_yield+interval_Pdt.+OD+RQ_integral+AS_Vol.+consum _O2+generate_CO2; 0.330, 0.656, tmp_cumulo+pH_cumulo+PL_cum ulo+interval_yield+interval_Pdt.+RQ_integral+AS_Vol.+emissi on_O2+consum_O2+interval_O2+generate_CO2; 0.319, 0.656, tmp +tmp_cumulo+pH_cumulo+interval_yield+interval_Pdt.+OD+RQ_in tegral+AS_Vol.+emission_CO2+consum_O2+interval_O2+generate_ CO2; 0.341, 0.656, tmp_cumulo+pH_cumulo+interval_yield+inte rval_Pdt.+OD+RS_cumulo+Cell_conc._interval+RQ_integral+cons um_O2+generate_CO2; 0.341, 0.656, tmp+pH_cumulo+interval_yi eld+interval_Pdt.+OD+rab_integral+RQ_integral+AS_Vol.+consu m_O2+generate_CO2; 0.330, 0.656, tmp+interval_yield+interva l_Pdt.+OD+Feed_Vol+RS_cumulo+RQ_integral+AS_Vol.+emission_C O2+consum_O2+generate_CO2; 0.330, 0.656, tmp_cumulo+pH_cumu lo+AN_cumulo+interval_yield+interval_Pdt.+OD+Cell_conc._int erval+RQ_integral+AS_Vol.+consum_O2+generate_CO2; 0.319, 0. 656, tmp_cumulo+pH+pH_cumulo+interval_yield+interval_Pdt.+O D+RQ_integral+AS_Vol.+emission_CO2+consum_O2+interval_O2+ge nerate_CO2; 0.330, 0.656, tmp_cumulo+pH_cumulo+interval_yie ld+interval_Pdt.+Feed_Vol+RS_cumulo+RQ_integral+AS_Vol.+con sum_O2+interval_O2+generate_CO2; 0.341, 0.656, tmp_cumulo+P L_cumulo+AN+interval_yield+interval_Pdt.+RS_cumulo+RQ_integ ral+emission_CO2+consum _O2+generate_CO2; 0.330, 0.656, tmp_ cumulo+pH_cumulo+interval_yield+interval_Pdt.+RS_cumulo+RQ_ integral+AS_Vol.+emission_O2+consum_O2+interval_O2+generate _CO2; 0.351, 0.656, tmp_cumulo+AN+interval_yield+interval_P dt.+OD+RS_cumulo+RQ_integral+consum_O2+generate_CO2; 0.330, 0.656, tmp_cumulo+pH_cumulo+PL_cumulo+AN+interval_yield+OD +rab_integral+RQ_integral+AS_Vol.+consum_O2+generate_CO2; 0. 341, 0.656, tmp+pH_cumulo+AN_cumulo+interval_yield+interval _Pdt.+OD+RQ_integral+emission_CO2+consum_O2+generate_CO2; 0. 341, 0.656, tmp+pH_cumulo+interval_yield+interval_Pdt.+OD+R Q_integral+AS_Vol.+emission_O2+consum_O2+generate_CO2; 0.34 1, 0.656, tmp_cumulo+pH_cumulo+PL+AN+interval_yield+OD+RQ_i ntegral+AS_Vol.+consum_O2+generate_CO2; 0.330, 0.656, tmp_c umulo+pH_cumulo+PL+AN_cumulo+interval_yield+OD+RQ_integral+ AS_Vol.+consum_O2+interval_O2+generate_CO2; 0.330, 0.656, t mp_cumulo+pH_cumulo+AN_cumulo+interval_yield+OD+Cell_conc._ interval+rab_integral+RQ_integral+AS_Vol.+consum_O2+generat e_CO2; 0.330, 0.655, tmp_cumulo+pH_cumulo+interval_yield+in terval_Pdt.+OD+RS_cumulo+Cell_conc._interval+RQ_integral+AS _Vol.+consum_O2+generate_CO2; 0.341, 0.655, tmp_cumulo+pH_c umulo+PL+interval_yield+OD+RQ_integral+AS_Vol.+emission_CO2 +consum_O2+generate_CO2; 0.308, 0.655, tmp_cumulo+pH_cumulo +interval_yield+interval_Pdt.+OD+rab_integral+RQ_integral+A S_Vol.+emission_O2+emission_CO2+consum_O2+interval_O2+gener ate_CO2; 0.319, 0.655, tmp_cumulo+pH_cumulo+interval_yield+ interval_Pdt.+OD+RS_cumulo+rab_integral+RQ_integral+AS_Vol. +consum_O2+interval_O2+generate_CO2; 0.351, 0.655, tmp_cumu lo+pH_cumulo+interval_yield+OD+RS+RQ_integral+consum_O2+int erval_O2+generate_CO2; 0.341, 0.655, tmp_cumulo+pH_cumulo+A N+interval_yield+interval_Pdt.+OD+rab_integral+RQ_integral+ consum_O2+generate_CO2; 0.360, 0.655, tmp_cumulo+pH_cumulo+ interval_yield+OD+RQ_integral+emission_CO2+consum_O2+genera te_CO2; 0.330, 0.655, tmp_cumulo+pH+pH_cumulo+interval_yiel d+interval_Pdt.+OD+Feed_Vol+RQ_integral+AS_Vol.+consum_O2+g enerate_CO2; 0.340, 0.655, tmp_cumulo+pH+interval_yield+int erval_Pdt.+OD+Feed_Vol+RQ_integral+AS_Vol.+consum_O2+genera te_CO2; 0.330, 0.655, tmp_cumulo+interval_yield+interval_Pd t.+RS_cumulo+Cell_conc._interval+rab_integral+RQ_integral+A S_Vol.+emission_CO2+consum_O2+generate_CO2; 0.330, 0.655, t mp_cumulo+pH+interval_yield+interval_Pdt.+RS_cumulo+RQ_inte gral+AS_Vol.+emission_O2+emission_CO2+consum_O2+generate_CO 2; 0.351, 0.655, tmp_cumulo+AN_cumulo+interval_yield+interv al_Pdt.+OD+RS_cumulo+RQ_integral+consum_O2+generate_CO2; 0. 340, 0.655, tmp+interval_yield+interval_Pdt.+Feed_Vol+RQ_in tegral+AS_Vol.+emission_CO2+consum_O2+interval_O2+generate CO2; 0.360, 0.655, tmp_cumulo+interval_yield+RS_cumulo+RQ_i ntegral+AS_Vol.+consum_O2+interval_O2+generate_CO2; 0.340, 0.655, tmp_cumulo+interval_yield+interval_Pdt.+RS_cumulo+ra b_integral+RQ_integral+emission_CO2+consum_O2+interval_O2+g enerate_CO2; 0.330, 0.655, tmp_cumulo+pH_cumulo+PL+AN+inter val_yield+OD+RQ_integral+AS_Vol.+consum_O2+interval_O2+gene rate_CO2; 0.330, 0.655, tmp_cumulo+pH_cumulo+AN_cumulo+inte rval_yield+interval_Pdt.+OD+RS_cumulo+RQ_integral+emission_ CO2+consum_O2+generate_CO2; 0.330, 0.655, tmp+tmp_cumulo+pH _cumulo+interval_yield+interval_Pdt.+rab_integral+RQ_integr al+AS_Vol.+emission_CO2+consum_O2+generate_CO2; 0.340, 0.65 5, tmp_cumulo+pH_cumulo+PL+AN_cumulo+interval_yield+OD+RQ_i ntegral+AS_Vol.+consum_O2+generate_CO2; 0.319, 0.655, tmp_c umulo+pH_cumulo+AN_cumulo+interval_yield+interval_Pdt.+OD+F eed_Vol+rab_integral+RQ_integral+AS_Vol.+consum_O2+generate _CO2; 0.330, 0.655, tmp_cumulo+PL_cumulo+interval_yield+int erval_Pdt.+RS_cumulo+rab_integral+RQ_integral+AS_Vol.+emiss ion_CO2+consum_O2+generate_CO2; 0.319, 0.655, tmp_cumulo+pH +pH_cumulo+interval_yield+interval_Pdt.+OD+Feed_Vol+RQ_inte gral+AS_Vol.+consum_O2+interval_O2+generate_CO2; 0.340, 0.6 55, tmp_cumulo+pH_cumulo+AN_cumulo+interval_yield+OD+RS_cum ulo+rab_integral+RQ_integral+consum_O2+generate_CO2; 0.360, 0.655, tmp_cumulo+pH_cumulo+interval_Pdt.+OD+RQ_integral+A S_Vol.+consum_O2+generate_CO2; 0.350, 0.655, tmp_cumulo+int erval_yield+interval_Pdt.+OD+Feed_Vol+RS_cumulo+RQ_integral +consum_O2+generate_CO2; 0.340, 0.655, tmp+pH+interval_yiel d+interval_Pdt.+RS_cumulo+rab_integral+RQ_integral+emission _CO2+consum_O2+generate_CO2; 0.360, 0.655, tmp_cumulo+inter val_yield+interval_Pdt.+RQ_integral+AS_Vol.+emission_O2+con sum_O2+generate_CO2; 0.330, 0.655, tmp+pH_cumulo+interval_y ield+interval_Pdt.+Feed_Vol+RQ_integral+AS_Vol.+emission_CO 2+consum_O2+interval_O2+generate_CO2; 0.330, 0.655, tmp_cum ulo+pH_cumulo+PL+interval_yield+OD+RS_cumulo+RQ_integral+AS _Vol.+consum_O2+interval_O2+generate_CO2; 0.340, 0.655, tmp +tmp_cumulo+pH_cumulo+interval_yield+OD+RS_cumulo+rab_integ ral+RQ_integral+consum_O2+generate_CO2; 0.340, 0.655, tmp+p H_cumulo+AN+interval_yield+interval_Pdt.+OD+RQ_integral+AS_ Vol.+consum_O2+generate_CO2; 0.350, 0.655, tmp+PL_cumulo+in terval_yield+interval_Pdt.+RQ_integral+AS_Vol.+emission_CO2 +consum_O2+generate_CO2; 0.360, 0.655, tmp+interval_yield+i nterval_Pdt. +RS_cumulo+RQ_integral+emission_O2+consum_O2+ge nerate_CO2; 0.330, 0.655, tmp_cumulo+AN+interval_yield+inte rval_Pdt. +RS_cumulo+RQ_integral+AS_Vol.+emission_O2+emissio n_CO2+consum_O2+generate_CO2; 0.340, 0.655, tmp+interval_yi eld+interval_Pdt.+OD+RS_cumulo+Cell_conc._interval+RQ_integ ral+emission_CO2+consum_O2+generate_CO2; 0.330, 0.655, tmp_ cumulo+pH_cumulo+PL_cumulo+AN_cumulo+interval_yield+interva l_Pdt.+OD+RQ_integral+AS_Vol.+consum_O2+generate_CO2; 0.350, 0.655, tmp_cumulo+interval_yield+interval_Pdt.+RS_cumulo+r ab_integral+RQ_integral+consum_O2+interval_O2+generate_CO2; 0.319, 0.655, tmp+tmp_cumulo+pH_cumulo+interval_yield+OD+F eed_Vol+rab_integral+RQ_integral+AS_Vol.+consum_O2+interval _O2+generate_CO2; 0.330, 0.655, tmp+tmp_cumulo+pH_cumulo+in terval_yield+interval_Pdt.+RS_cumulo+RQ_integral+AS_Vol.+em ission_CO2+consum _O2+generate_CO2; 0.330, 0.655, tmp+tmp_cu mulo+interval_yield+interval_Pdt.+RS_cumulo+rab_integral+RQ integral+emission_O2+emission_CO2+consum_O2+generate_CO2; 0.340, 0.655, tmp_cumulo+pH+AN+interval_yield+interval_Pdt. +RS_cumulo+RQ_integral+emission_CO2+consum_O2+generate_CO2; 0.330, 0.655, tmp_cumulo+pH_cumulo+PL+interval_yield+inter val_Pdt.+OD+RQ_integral+AS_Vol.+consum_O2+interval_O2+gener ate_CO2; 0.340, 0.655, tmp+AN+interval_yield+interval_Pdt.+ OD+RQ_integral+AS_Vol.+emission_CO2+consum_O2+generate_CO2; 0.319, 0.655, tmp_cumulo+pH_cumulo+AN+interval_yield+OD+Ce ll_conc._interval+rab_integral+RQ_integral+AS_Vol.+consum_O 2+interval_O2+generate_CO2; 0.308, 0.655, tmp+pH_cumulo+AN+ interval_yield+interval_Pdt.+OD+Feed_Vol+rab_integral+RQ_in tegral+AS_Vol.+consum_O2+interval_O2+generate_CO2; 0.350, 0. 655, tmp+pH_cumulo+interval_yield+OD+RQ_integral+AS_Vol.+em ission_CO2+consum_O2+generate_CO2; 0.350, 0.655, tmp+tmp_cu mulo+interval_yield+interval_Pdt.+OD+RS_cumulo+RQ_integral+ consum_O2+generate_CO2; 0.340, 0.655, tmp_cumulo+interval_y ield+interval_Pdt.+rab_integral+RQ_integral+AS_Vol.+emissio n_O2+emission_CO2+consum _O2+generate_CO2; 0.319, 0.655, tmp _cumulo+pH+interval_yield+interval_Pdt.+OD+RS_cumulo+rab_in tegral+RQ_integral+AS_Vol.+emission_CO2+consum_O2+generate_ CO2; 0.340, 0.655, tmp_cumulo+pH_cumulo+AN+interval_yield+r ab_integral+RQ_integral+AS_Vol.+consum_O2+interval_O2+gener ate_CO2; 0.319, 0.655, tmp+pH_cumulo+interval_yield+interva l_Pdt.+OD+Feed_Vol+RS_cumulo+RQ_integral+AS_Vol.+consum_O2+ interval_O2+generate_CO2; 0.340, 0.655, tmp+interval_yield+ interval_Pdt.+Feed_Vol+Cell_conc._interval+RQ_integral+AS_V ol.+emission_CO2+consum_O2+generate_CO2; 0.329, 0.655, tmp_ cumulo+pH+interval_yield+interval_Pdt.+OD+Feed_Vol+RQ_integ ral+AS_Vol.+consum_O2+interval_O2+generate_CO2; 0.340, 0.65 5, tmp_cumulo+pH_cumulo+interval_yield+OD+RS_cumulo+rab_int egral+RQ_integral+emission_O2+consum_O2+generate_CO2; 0.319, 0.655, tmp_cumulo+pH_cumulo+AN_cumulo+interval_yield+inter val_Pdt.+OD+RQ_integral+AS_Vol.+emission_O2+consum_O2+inter val_O2+generate_CO2; 0.350, 0.655, tmp+pH_cumulo+interval_y ild+interval_Pdt.+OD+Feed_Vol+RQ_integral+consum_O2+genera te_CO2; 0.340, 0.655, tmp+pH_cumulo+interval_yield+OD+Feed_ Vol+rab_integral+RQ_integral+AS_Vol.+consum_O2+generate_CO 2; 0.340, 0.655, tmp+interval_yield+interval_Pdt.+OD+Feed_V ol+RQ integral+AS_Vol.+emission_CO2+consum_O2+generate_CO2; 0.350, 0.655, tmp+pH_cumulo+interval_yield+interval_Pdt.+R Q_integral+AS_Vol.+emission_O2+consum _O2+generate_CO2; 0.35 0, 0.655, tmp_cumulo+PL_cumulo+interval_yield+interval_Pdt. +OD+RS_cumulo+RQ_integral+consum_O2+generate_CO2; 0.329, 0. 655, tmp_cumulo+pH+pH_cumulo+AN+interval_yield+interval_Pdt. +OD+RQ_integral+AS_Vol.+consum_O2+generate_CO2; 0.340, 0.65 5, tmp+tmp_cumulo+AN+interval_yield+interval_Pdt.+RS_cumulo +RQ_integral+emission_CO2+consum _O2+generate_CO2; 0.360, 0. 655, tmp+tmp_cumulo+pH_cumulo+interval_yield+OD+RQ_integral +consum_O2+generate_CO2; 0.329, 0.655, tmp+interval_yield+i nterval_Pdt.+RS_cumulo+rab_integral+RQ_integral+AS_Vol.+emi ssion_O2+emission_CO2+consum _O2+generate_CO2; 0.319, 0.655, tmp_cumulo+pH_cumulo+interval_yield+interval_Pdt.+OD+RS_cu mulo+rab_integral+RQ_integral+AS_Vol.+emission_O2+consum_O2 +generate_CO2; 0.340, 0.655, tmp+pH+interval_yield+interval _Pdt.+RS_cumulo+RQ_integral+AS_Vol.+emission_CO2+consum_O2+ generate_CO2; 0.329, 0.655, tmp+pH_cumulo+AN+interval_yield +interval_Pdt.+rab_integral+RQ_integral+AS_Vol.+emission_CO 2+consum_O2+generate_CO2; 0.360, 0.655, tmp+pH_cumulo+AN_cu mulo+interval_yield+OD+RQ_integral+consum_O2+generate_CO2; 0.318, 0.655, tmp+pH_cumulo+interval_yield+interval_Pdt.+OD +Feed_Vol+Cell_conc._interval+RQ_integral+AS_Vol.+consum_O2 +interval_O2+generate_CO2; 0.318, 0.655, tmp_cumulo+pH_cumu lo+interval_yield+OD+RS_cumulo+rab_integral+RQ_integral+AS_ Vol.+emission_O2+consum_O2+interval_O2+generate_CO2; 0.340, 0.655, tmp_cumulo+AN+interval_yield+interval_Pdt.+RQ_integ ral+AS_Vol.+emission_CO2+consum_O2+interval_O2+generate_CO 2; 0.329, 0.655, tmp+pH_cumulo+interval_yield+interval_Pdt. +OD+Feed_Vol+RQ_integral+Accum._Yield+AS_Vol.+consum_O2+gen erate_CO2; 0.329, 0.655, tmp+tmp_cumulo+pH_cumulo+AN_cumulo +interval_yield+interval_Pdt.+OD+RQ_integral+AS_Vol.+consum _O2+generate_CO2; 0.350, 0.655, tmp_cumulo+pH_cumulo+interv al_yield+interval_Pdt.+OD+RS+RQ_integral+consum_O2+generate _CO2; 0.340, 0.655, tmp_cumulo+AN+interval_yield+interval_P dt.+Feed_Vol+RS_cumulo+RQ_integral+emission_CO2+consum_O2+g enerate_CO2; 0.340, 0.655, tmp+interval_yield+interval_Pdt. +Cell_conc._interval+RQ_integral+AS_Vol.+emission_O2+emissi on_CO2+consum_O2+generate_CO2; 0.329, 0.655, tmp_cumulo+pH_ cumulo+PL_cumulo+interval_yield+interval_Pdt.+OD+RQ_integra l+AS_Vol.+emission_O2+consum_O2+generate_CO2; 0.318, 0.655, tmp_cumulo+pH_cumulo+AN+interval_yield+OD+RS_cumulo+rab_in tegral+RQ_integral+AS_Vol.+consum_O2+interval_O2+generate_C 02; 0.329, 0.655, tmp+tmp_cumulo+interval_yield+interval_Pd t.+RS_cumulo+RQ_integral+AS_Vol.+emission_O2+emission_CO2+c onsum_O2+generate_CO2; 0.350, 0.655, tmp+pH_cumulo+interval _yield+interval_Pdt.+Feed_Vol+RQ_integral+AS_Vol.+consum_O2 +generate_CO2; 0.329, 0.655, tmp+tmp_cumulo+pH_cumulo+inter val_yield+interval_Pdt.+RQ_integral+AS_Vol.+emission_CO2+co nsum_O2+interval_O2+generate_CO2; 0.350, 0.655, tmp_cumulo+ pH_cumulo+PL+interval_yield+OD+RS_cumulo+RQ_integral+consum _O2+generate_CO2; 0.329, 0.655, tmp_cumulo+pH+interval_yiel d+interval_Pdt.+RS_cumulo+rab_integral+RQ_integral+emission _O2+emission_CO2+consum _O2+generate_CO2; 0.360, 0.655, tmp+ pH_cumulo+AN+interval_yield+OD+RQ_integral+consum_O2+genera te_CO2; 0.350, 0.655, tmp+pH_cumulo+PL_cumulo+interval_yiel d+interval_Pdt.+OD+RQ_integral+consum_O2+generate_CO2; 0.30 7, 0.655, tmp_cumulo+pH_cumulo+interval_yield+interval_Pdt. +OD+Feed_Vol+rab_integral+RQ_integral+AS_Vol.+emission_O2+e mission_CO2+consum_O2+generate_CO2; 0.340, 0.655, tmp_cumul o+pH_cumulo+AN+interval_yield+interval_Pdt.+RS_cumulo+RQ_in tegral+emission_CO2+consum _O2+generate_CO2; 0.329, 0.655, t mp_cumulo+interval_yield+interval_Pdt.+OD+Feed_Vol+RS_cumul o+rab_integral+RQ_integral+emission_CO2+consum_O2+generate_ CO2; 0.329, 0.655, tmp+tmp_cumulo+pH_cumulo+interval_yield+ OD+Cell_conc._interval+rab_integral+RQ_integral+AS_Vol.+con sum_O2+generate_CO2; 0.329, 0.655, tmp_cumulo+pH_cumulo+AN+ AN_cumulo+interval_yield+OD+rab_integral+RQ_integral+AS_Vol. +consum_O2+generate_CO2; 0.350, 0.655, tmp+tmp_cumulo+pH_cu mulo+interval_yield+interval_Pdt.+OD+RQ_integral+consum_O2+ generate_CO2; 0.329, 0.655, tmp_cumulo+pH_cumulo+PL+interva l_yield+OD+Feed_Vol+rab_integral+RQ_integral+AS_Vol.+consum _O2+generate_CO2; 0.350, 0.655, tmp+interval_yield+interval _Pdt.+Feed_Vol+RS_cumulo+RQ_integral+AS_Vol.+consum_O2+gene rate_CO2; 0.340, 0.655, tmp_cumulo+interval_yield+interval_ Pdt.+rab_integral+RQ_integral+AS_Vol.+emission_CO2+consum_O 2+interval_O2+generate_CO2; 0.318, 0.655, tmp_cumulo+pH_cum ulo+AN+interval_yield+interval_Pdt.+OD+rab_integral+RQ_inte gral+AS_Vol.+emission_O2+consum_O2+generate_CO2; 0.329, 0.6 55, tmp_cumulo+PL_cumulo+interval_yield+interval_Pdt.+OD+RS _cumulo+rab_integral+RQ_integral+emission_CO2+consum_O2+gen erate_CO2; 0.318, 0.655, tmp_cumulo+pH_cumulo+PL_cumulo+int erval_yield+OD+rab_integral+RQ_integral+AS_Vol.+emission_CO 2+consum_O2+interval_O2+generate_CO2; 0.329, 0.655, tmp+tmp _cumulo+pH_cumulo+AN+interval_yield+OD+rab_integral+RQ_inte gral+AS_Vol.+consum_O2+generate_CO2; 0.318, 0.655, tmp_cumu lo+pH_cumulo+AN+interval_yield+interval_Pdt.+OD+RS_cumulo+r ab_integral+RQ_integral+emission_CO2+consum_O2+generate_CO 2; 0.339, 0.655, tmp+pH_cumulo+interval_yield+interval_Pdt. +OD+RQ_integral+emission_O2+emission_CO2+consum_O2+generate _CO2; 0.339, 0.655, tmp+interval_yield+interval_Pdt.+Feed_V ol+RQ_integral+AS_Vol.+emission_O2+emission_CO2+consum_O2+g enerate_CO2; 0.329, 0.655, tmp_cumulo+pH+interval_yield+int erval_Pdt.+OD+RS_cumulo+RQ_integral+emission_O2+emission_CO 2+consum_O2+generate_CO2; 0.329, 0.655, tmp+pH_cumulo+AN_cu mulo+interval_yield+interval_Pdt.+rab_integral+RQ_integral+ AS_Vol.+emission_CO2+consum_O2+generate_CO2; 0.329, 0.655, tmp_cumulo+pH_cumulo+PL_cumulo+AN_cumulo+interval_yield+OD+ rab_integral+RQ_integral+AS_Vol.+consum_O2+generate_CO2; 0. 318, 0.655, tmp_cumulo+pH_cumulo+AN+AN_cumulo+interval_yiel d+OD+rab_integral+RQ_integral+AS_Vol.+consum_O2+interval_O2 +generate_CO2; 0.329, 0.655, tmp_cumulo+pH_cumulo+AN_cumulo +interval_yield+interval_Pdt.+Feed_Vol+RQ_integral+AS_Vol.+ consum_O2+interval_O2+generate_CO2; 0.359, 0.655, tmp_cumul o+pH_cumulo+rab_integral+RQ_integral+AS_Vol.+consum_O2+inte rval_O2+generate_CO2; 0.350, 0.655, tmp+tmp_cumulo+interval _yield+interval_Pdt.+RS_cumulo+RQ_integral+AS_Vol.+consum_O 2+generate_CO2; 0.359, 0.655, tmp+interval_yield+interval_P dt.+Cell_conc._interval+RQ_integral+AS_Vol.+consum_O2+gener ate_CO2; 0.350, 0.655, tmp_cumulo+PL_cumulo+interval_yield+ interval_Pdt.+RS_cumulo+RQ_integral+AS_Vol.+consum_O2+gener ate_CO2; 0.339, 0.655, tmp_cumulo+pH_cumulo+interval_yield+ interval_Pdt.+Feed_Vol+RS_cumulo+RQ_integral+AS_Vol.+consum _O2+generate_CO2; 0.350, 0.655, tmp_cumulo+AN+interval_yiel d+interval_Pdt.+RS_cumulo+rab_integral+RQ_integral+consum_O 2+generate_CO2; 0.329, 0.655, tmp_cumulo+pH_cumulo+interval _yield+interval_Pdt.+OD+RS_cumulo+rab_integral+RQ_integral+ emission _O2+consum _O2+generate_CO2; 0.350, 0.655, tmp+pH_cu mulo+interval_yield+OD+rab_integral+RQ_integral+AS_Vol.+con sum_O2+generate_CO2; 0.329, 0.655, tmp_cumulo+AN+interval_y ield+interval_Pdt.+Feed_Vol+RS_cumulo+RQ_integral+AS_Vol.+e mission_CO2+consum_O2+generate_CO2; 0.329, 0.655, tmp_cumul o+pH_cumulo+AN_cumulo+interval_yield+interval_Pdt.+rab_inte gral+RQ_integral+AS_Vol.+consum_O2+interval_O2+generate_CO 2; 0.339, 0.655, tmp_cumulo+pH_cumulo+interval_yield+interv al-Pdt.+OD+Feed_Vol+RQ_integral+emission_CO2+consum_O2+gene rate_CO2; 0.339, 0.655, tmp+AN_cumulo+interval_yield+interv al_Pdt.+OD+RQ_integral+AS_Vol.+emission_CO2+consum_O2+gener ate_CO2; 0.339, 0.655, tmp_cumulo+pH+interval_yield+interva l_Pdt. +Feed_Vol+RQ_integral+AS_Vol.+emission_CO2+consum_O2+ generate_CO2; 0.349, 0.655, tmp_cumulo+pH_cumulo+AN_cumulo+ interval_yield+OD+Feed_Vol+RQ_integral+consum_O2+generate_C 02; 0.318, 0.655, tmp+pH_cumulo+interval_yield+interval_Pdt. +OD+Feed_Vol+RQ_integral+AS_Vol.+emission_O2+emission_CO2+c onsum_O2+generate_CO2; 0.329, 0.655, tmp+pH_cumulo+PL_cumul o+interval_yield+interval_Pdt.+OD+RQ_integral+AS_Vol.+emiss ion_CO2+consum_O2+generate_CO2; 0.349, 0.655, tmp_cumulo+in terval_yield+interval_Pdt.+OD+RS_cumulo+RQ_integral+consum_ O2+interval_O2+generate_CO2; 0.329, 0.655, tmp_cumulo+inter val_yield+interval_Pdt.+Feed_Vol+RS_cumulo+rab_integral+RQ_ integral+emission_O2+emission_CO2+consum _O2+generate_CO2; 0. 329, 0.655, tmp+tmp_cumulo+pH_cumulo+interval_yield+interva l_Pdt.+OD+RS_cumulo+RQ_integral+emission_CO2+consum_O2+gene rate_CO2; 0.318, 0.655, tmp_cumulo+pH_cumulo+interval_yield +interval_Pdt.+RS_cumulo+rab_integral+RQ_integral+AS_Vol.+e mission_CO2+consum_O2+interval_O2+generate_CO2; 0.349, 0.65 5, tmp_cumulo+pH_cumulo+AN+interval_yield+OD+RS+RQ_integral +consum_O2+generate_CO2; 0.318, 0.655, tmp_cumulo+pH_cumulo +interval_yield+interval_Pdt.+rab_integral+RQ_integral+AS_V ol.+emission_O2+emission_CO2+consum_O2+interval_O2+generate _CO2; 0.329, 0.655, tmp+tmp_cumulo+pH_cumulo+interval_yield +OD+rab_integral+RQ_integral+AS_Vol.+emission_O2+consum_O2+ generate_CO2; 0.329, 0.655, tmp_cumulo+pH_cumulo+AN+AN_cumu lo+interval_yield+interval_Pdt.+OD+RQ_integral+AS_Vol.+cons um_O2+generate_CO2; 0.339, 0.655, tmp_cumulo+pH+pH_cumulo+i nterval_yield+interval_Pdt.+RQ_integral+AS_Vol.+emission_CO 2+consum_O2+generate_CO2; 0.329, 0.655, tmp_cumulo+pH_cumul o+AN_cumulo+interval_yield+interval_Pdt.+RQ_integral+AS_Vol. +emission_O2+consum_O2+interval_O2+generate_CO2; 0.349, 0.6 55, tmp_cumulo+interval_yield+OD+RS_cumulo+RQ_integral+AS_V ol.+consum_O2+interval_O2+generate_CO2; 0.359, 0.655, tmp_c umulo+pH+pH_cumulo+interval_yield+OD+RQ_integral+consum_O2+ generate_CO2; 0.318, 0.655, tmp_cumulo+pH_cumulo+AN_cumulo+ interval_yield+interval_Pdt.+OD+RQ_integral+AS_Vol.+emissio n_O2+emission_CO2+consum_O2+generate_CO2; 0.318, 0.655, tmp _cumulo+pH_cumulo+interval_yield+interval_Pdt.+OD+RS_cumulo +RQ_integral+AS_Vol.+emission_O2+consum_O2+interval_O2+gene rate_CO2; 0.339, 0.655, tmp_cumulo+pH_cumulo+interval_yield +interval_Pdt.+Feed_Vol+RQ_integral+AS_Vol.+emission_O2+con sum_O2+generate_CO2; 0.349, 0.655, tmp_cumulo+pH_cumulo+int erval_yield+OD+Feed_Vol+RQ_integral+emission_CO2+consum_O2+ generate_CO2; 0.318, 0.655, tmp_cumulo+pH_cumulo+AN_cumulo+ interval_yield+interval_Pdt.+rab_integral+RQ_integral+AS_Vo l.+emission_CO2+consum_O2+interval_O2+generate_CO2; 0.349, 0.655, tmp_cumulo+pH+pH_cumulo+OD+RQ_integral+AS_Vol.+consu m_O2+interval_O2+generate_CO2; 0.339, 0.655, tmp+AN+interva l_yield+interval_Pdt.+RQ_integral+AS_Vol.+emission_CO2+cons um_O2+interval_O2+generate_CO2; 0.359, 0.655, tmp+pH+pH_cum ulo+interval_yield+OD+RQ_integral+consum_O2+generate_CO2; 0. 329, 0.655, tmp_cumulo+pH+interval_yield+interval_Pdt.+Feed _Vol+RS_cumulo+RQ_integral+AS_Vol.+emission_CO2+consum_O2+g enerate_CO2; 0.339, 0.655, tmp_cumulo+pH+pH_cumulo+interval _yield+OD+RS_cumulo+RQ_integral+consum_O2+interval_O2+gener ate_CO2; 0.349, 0.654, tmp_cumulo+AN+interval_yield+interva l_Pdt.+RS_cumulo+RQ_integral+AS_Vol.+consum_O2+generate_CO 2; 0.329, 0.654, tmp_cumulo+pH+pH_cumulo+interval_yield+OD+ Cell_conc._interval+rab_integral+RQ_integral+AS_Vol.+consum _O2+generate_CO2; 0.318, 0.654, tmp_cumulo+pH_cumulo+interv al_yield+interval_Pdt.+OD+Cell_conc._interval+RQ_integral+A S_Vol.+emission_O2+consum_O2+interval_O2+generate_CO2; 0.33 9, 0.654, tmp_cumulo+pH_cumulo+interval_yield+interval_Pdt. +OD+rab_integral+RQ_integral+emission_CO2+consum_O2+generat e_CO2; 0.329, 0.654, tmp_cumulo+pH_cumulo+AN_cumulo+interva l_yield+interval_Pdt.+OD+RS_cumulo+rab_integral+RQ_integral +consum_O2+generate_CO2; 0.318, 0.654, tmp_cumulo+pH_cumulo +PL_cumulo+AN+interval_yield+OD+rab_integral+RQ_integral+AS _Vol.+consum_O2+interval_O2+generate_CO2; 0.307, 0.654, tmp _cumulo+pH_cumulo+interval_yield+interval_Pdt.+OD+Feed_Vol+ rab_integral+RQ_integral+AS_Vol.+emission_O2+consum_O2+inte rval_O2+generate_CO2; 0.339, 0.654, tmp+interval_yield+inte rval_Pdt.+rab_integral+RQ_integral+Accum._Yield+AS_Vol.+emi ssion_CO2+consum_O2+generate_CO2; 0.329, 0.654, tmp_cumulo+ interval_yield+interval_Pdt.+OD+RS_cumulo+Cell_conc._interv al+RQ_integral+AS_Vol.+emission_CO2+consum_O2+generate_CO2; 0.307, 0.654, tmp_cumulo+pH_cumulo+AN_cumulo+interval_yiel d+interval_Pdt.+OD+rab_integral+RQ_integral+AS_Vol.+emissio n_CO2+consum_O2+interval_O2+generate_CO2; 0.349, 0.654, tmp _cumulo+AN_cumulo+interval_yield+interval_Pdt.+RS_cumulo+RQ _integral+AS_Vol.+consum_O2+generate_CO2; 0.339, 0.654, tmp _cumulo+PL_cumulo+interval_yield+interval_Pdt.+RS_cumulo+RQ _integral+emission_O2+emission_CO2+consum_O2+generate_CO2; 0.318, 0.654, tmp_cumulo+pH_cumulo+PL+interval_yield+OD+Fee d_Vol+rab_integral+RQ_integral+AS_Vol.+consum_O2+interval_O 2+generate_CO2; 0.318, 0.654, tmp_cumulo+pH_cumulo+interval _yield+interval_Pdt.+Feed_Vol+RS_cumulo+rab_integral+RQ_int egral+AS_Vol.+emission_CO2+consum_O2+generate_CO2; 0.318, 0. 654, tmp_cumulo+pH_cumulo+interval_yield+interval_Pdt.+OD+C ell_conc._interval+RQ_integral+AS_Vol.+emission_O2+emission _CO2+consum_O2+generate_CO2; 0.318, 0.654, tmp_cumulo+pH+pH _cumulo+interval_yield+interval_Pdt.+OD+RS_cumulo+RQ_integr al+AS_Vol.+emission_CO2+consum_O2+generate_CO2; 0.349, 0.65 4, tmp_cumulo+pH_cumulo+interval_yield+interval_Pdt.+OD+Cel l_conc._interval+RQ_integral+consum_O2+generate_CO2; 0.318, 0.654, tmp_cumulo+pH+pH_cumulo+interval_yield+interval_Pdt. +OD+RQ_integral+AS_Vol.+emission_O2+consum_O2+interval_O2+g enerate_CO2; 0.328, 0.654, tmp_cumulo+pH+pH_cumulo+interval _yield+interval_Pdt.+RQ_integral+AS_Vol.+emission_CO2+consu m_O2+interval_O2+generate_CO2; 0.349, 0.654, tmp_cumulo+pH_ cumulo+AN_cumulo+interval_yield+RQ_integral+AS_Vol.+consum_ O2+interval_O2+generate_CO2; 0.339, 0.654, tmp_cumulo+inter val_yield+interval_Pdt.+Feed_Vol+RS_cumulo+RQ_integral+AS_V ol.+emission_O2+consum_O2+generate_CO2; 0.328, 0.654, tmp_c umulo+pH+interval_yield+interval_Pdt.+OD+RQ_integral+AS_Vol. +emission_CO2+consum _O2+interval_O2+generate_CO2; 0.339, 0. 654, tmp_cumulo+pH+interval_yield+interval_Pdt.+Feed_Vol+RQ _integral+AS_Vol.+consum_O2+interval_O2+generate_CO2; 0.339, 0.654, tmp_cumulo+pH_cumulo+PL_cumulo+interval_yield+OD+RS _cumulo+rab_integral+RQ_integral+consum_O2+generate_CO2; 0. 359, 0.654, tmp+AN_cumulo+interval_yield+interval_Pdt.+RS_c umulo+RQ_integral+consum_O2+generate_CO2; 0.339, 0.654, tmp +pH_cumulo+interval_yield+OD+Cell_conc._interval+RQ_integra l+AS_Vol.+consum_O2+interval_O2+generate_CO2; 0.328, 0.654, tmp_cumulo+pH_cumulo+interval_yield+interval_Pdt.+Feed_Vol +RQ_integral+AS_Vol.+emission_O2+emission_CO2+consum_O2+gen erate_CO2; 0.339, 0.654, tmp_cumulo+interval_yield+interval _Pdt.+Feed_Vol+rab_integral+RQ_integral+AS_Vol.+emission_CO 2+consum_O2+generate_CO2; 0.349, 0.654, tmp_cumulo+pH_cumul o+AN_cumulo+interval_yield+OD+rab_integral+RQ_integral+cons um_O2+generate_CO2; 0.359, 0.654, tmp+interval_yield+interv al_Pdt.+RS_cumulo+RQ_integral+AS_Vol.+consum_O2+generate_CO 2; 0.349, 0.654, tmp_cumulo+pH_cumulo+interval_yield+interv al_Pdt.+RS_cumulo+RQ_integral+emission_O2+consum_O2+generat e_CO2; 0.368, 0.654, tmp+interval_yield+interval_Pdt.+RQ_in tegral+consum_O2+interval_O2+generate_CO2; 0.349, 0.654, tm p+pH+pH_cumulo+interval_yield+interval_Pdt.+OD+RQ_integral+ consum_O2+generate_CO2; 0.339, 0.654, tmp+AN_cumulo+interva l_yield+interval_Pdt.+Cell_conc._interval+RQ_integral+AS_Vo 1.+emission_CO2+consum_O2+generate_CO2; 0.339, 0.654, tmp_c umulo+pH+AN+interval_yield+interval_Pdt.+RQ_integral+AS_Vol. +emission_CO2+consum _O2+generate_CO2; 0.328, 0.654, tmp_cum ulo+pH+pH_cumulo+interval_yield+interval_Pdt.+OD+RS_cumulo+ RQ_integral+emission_CO2+consum _O2+generate_CO2; 0.317, 0.6 54, tmp+tmp_cumulo+pH_cumulo+interval_yield+interval_Pdt.+O D+rab_integral+RQ_integral+AS_Vol.+consum_O2+interval_O2+ge nerate_CO2; 0.317, 0.654, tmp_cumulo+pH_cumulo+AN_cumulo+in terval_yield+interval_Pdt.+OD+Feed_Vol+RQ_integral+AS_Vol.+ emission _CO2+consum _O2+generate_CO2; 0.359, 0.654, tmp_cumu lo+interval_yield+OD+RS_cumulo+RQ_integral+AS_Vol.+consum_O 2+generate_CO2; 0.306, 0.654, tmp_cumulo+pH_cumulo+interval _yield+interval_Pdt.+OD+Feed_Vol+RS_cumulo+rab_integral+RQ_ integral+AS_Vol.+consum_O2+interval_O2+generate_CO2; 0.349, 0.654, tmp_cumulo+pH_cumulo+AN+interval_yield+interval_Pdt. +RS_cumulo+RQ_integral+consum_O2+generate_CO2; 0.317, 0.654, tmp+tmp_cumulo+pH_cumulo+interval_yield+interval_Pdt.+OD+F eed_Vol+rab_integral+RQ_integral+AS_Vol.+consum_O2+generate _CO2; 0.339, 0.654, tmp_cumulo+pH_cumulo+PL+interval_yield+ OD+Cell_conc._interval+RQ_integral+AS_Vol.+consum_O2+genera te_CO2; 0.317, 0.654, tmp_cumulo+pH_cumulo+AN+interval_yiel d+interval_Pdt.+RS_cumulo+rab_integral+RQ_integral+AS_Vol.+ emission_CO2+consum_O2+generate_CO2; 0.339, 0.654, tmp_cumu lo+pH_cumulo+AN_cumulo+interval_yield+interval_Pdt.+RQ_inte gral+AS_Vol.+emission_O2+consum_O2+generate_CO2; 0.317, 0.6 54, tmp_cumulo+pH_cumulo+PL_cumulo+interval_yield+interval_ Pdt.+OD+rab_integral+RQ_integral+AS_Vol.+consum_O2+interval _O2+generate_CO2; 0.328, 0.654, tmp_cumulo+pH_cumulo+interv al_yield+interval_Pdt.+RS_cumulo+rab_integral+RQ_integral+e mission_O2+emission_CO2+consum_O2+generate_CO2; 0.339, 0.65 4, tmp_cumulo+pH_cumulo+interval_yield+OD+RQ_integral+Accum. _Yield+AS_Vol.+emission_CO2+consum_O2+generate_CO2; 0.349, 0.654, tmp_cumulo+PL_cumulo+interval_yield+interval_Pdt.+RS _cumulo+rab_integral+RQ_integral+consum_O2+generate_CO2; 0. 328, 0.654, tmp_cumulo+pH_cumulo+AN+interval_yield+OD+RS_cu mulo+RQ_integral+AS_Vol.+emission_CO2+consum_O2+generate_CO 2; 0.339, 0.654, tmp_cumulo+pH+pH_cumulo+AN+interval_yield+ OD+RS_cumulo+RQ_integral+consum_O2+generate_CO2; 0.317, 0.6 54, tmp_cumulo+pH_cumulo+interval_yield+OD+Cell_conc._inter val+rab_integral+RQ_integral+AS_Vol.+emission_O2+consum_O2+ interval_O2+generate_CO2; 0.339, 0.654, tmp+pH+interval_yie ld+interval_Pdt.+OD+RS_cumulo+RQ_integral+emission_CO2+cons um_O2+generate_CO2; 0.339, 0.654, tmp_cumulo+pH_cumulo+AN+i nterval_yield+interval_Pdt.+RQ_integral+AS_Vol.+emission_O2 +consum_O2+generate_CO2; 0.339, 0.654, tmp_cumulo+interval_ yield+interval_Pdt.+OD+Feed_Vol+RS_cumulo+RQ_integral+AS_Vo l.+consum_O2+generate_CO2; 0.339, 0.654, tmp_cumulo+AN+AN_c umulo+interval_yield+interval_Pdt.+RS_cumulo+RQ_integral+em ission_CO2+consum_O2+generate_CO2; 0.328, 0.654, tmp+interv al_yield+interval_Pdt.+OD+RS_cumulo+rab_integral+RQ_integra l+AS_Vol.+emission_CO2+consum_O2+generate_CO2; 0.328, 0.654, tmp_cumulo+pH+interval_yield+interval_Pdt.+OD+Feed_Vol+RS_ cumulo+RQ_integral+AS_Vol.+consum_O2+generate_CO2; 0.339, 0. 654, tmp_cumulo+interval_yield+interval_Pdt.+RS_cumulo+rab_ integral+RQ_integral+AS_Vol.+emission_O2+consum_O2+generate _CO2; 0.317, 0.654, tmp_cumulo+pH_cumulo+AN+interval_yield+ interval_Pdt.+OD+Cell_conc._interval+RQ_integral+AS_Vol.+co nsum_O2+interval_O2+generate_CO2; 0.317, 0.654, tmp_cumulo+ pH_cumulo+interval_yield+interval_Pdt.+OD+Feed_Vol+Cell_con c._interval+rab_integral+RQ_integral+AS_Vol.+consum_O2+gene rate_CO2; 0.359, 0.654, tmp+interval_yield+interval_Pdt.+OD +RQ_integral+AS_Vol.+consum_O2+generate_CO2; 0.349, 0.654, tmp_cumulo+interval_yield+interval_Pdt.+RQ_integral+AS_Vol. +emission_O2+consum_O2+interval_O2+generate_CO2; 0.328, 0.6 54, tmp_cumulo+pH_cumulo+interval_yield+OD+RS_cumulo+RQ_int egral+AS_Vol.+emission_O2+emission_CO2+consum_O2+generate_C 02; 0.306, 0.654, tmp_cumulo+pH_cumulo+AN+interval_yield+in terval_Pdt.+OD+Feed_Vol+rab_integral+RQ_integral+AS_Vol.+em ission_CO2+consum_O2+generate_CO2; 0.339, 0.654, tmp+interv al_yield+interval_Pdt.+Cell_conc._interval+RQ_integral+AS_V ol.+emission_CO2+consum_O2+interval_O2+generate_CO2; 0.317, 0.654, tmp_cumulo+pH_cumulo+interval_yield+interval_Pdt.+O D+RS_cumulo+Cell_conc._interval+RQ_integral+AS_Vol.+emissio n_CO2+consum_O2+generate_CO2; 0.328, 0.654, tmp_cumulo+pH_c umulo+PL+interval_yield+OD+rab_integral+RQ_integral+AS_Vol. +emission_CO2+consum_O2+generate_CO2; 0.306, 0.654, tmp_cum ulo+pH_cumulo+AN_cumulo+interval_yield+interval_Pdt.+OD+Fee d_Vol+rab_integral+RQ_integral+AS_Vol.+consum_O2+interval_O 2+generate_CO2; 0.328, 0.654, tmp+pH_cumulo+interval_yield+ OD+Feed_Vol+rab_integral+RQ_integral+AS_Vol.+consum_O2+inte rval_O2+generate_CO2; 0.377, 0.654, tmp_cumulo+RQ_integral+ AS_Vol.+consum_O2+interval_O2+generate_CO2; 0.339, 0.654, t mp_cumulo+interval_yield+interval_Pdt.+RS_cumulo+Cell_conc. _interval+RQ_integral+emission_O2+emission_CO2+consum_O2+ge nerate_CO2; 0.328, 0.654, tmp_cumulo+pH_cumulo+AN+interval_ yield+OD+Cell_conc._interval+RQ_integral+AS_Vol.+emission_C O2+consum_O2+generate_CO2; 0.339, 0.654, tmp_cumulo+AN+inte rval_yield+interval_Pdt.+rab_integral+RQ_integral+AS_Vol.+e mission_CO2+consum_O2+generate_CO2; 0.349, 0.654, tmp_cumul o+pH_cumulo+OD+Feed_Vol+RQ_integral+AS_Vol.+consum_O2+inter val_O2+generate_CO2; 0.349, 0.654, tmp_cumulo+pH+interval_y ield+interval_Pdt.+RQ_integral+AS_Vol.+emission_O2+consum_O 2+generate_CO2; 0.328, 0.654, tmp_cumulo+AN_cumulo+interval _yield+interval_Pdt.+RS_cumulo+rab_integral+RQ_integral+emi ssion_O2+emission_CO2+consum_O2+generate_CO2; 0.339, 0.654, tmp_cumulo+pH_cumulo+interval_yield+Feed_Vol+rab_integral+ RQ_integral+AS_Vol.+consum_O2+interval_O2+generate_CO2; 0.3 17, 0.654, tmp+tmp_cumulo+pH_cumulo+interval_yield+OD+rab_i ntegral+RQ_integral+AS_Vol.+emission_CO2+consum_O2+interval _O2+generate_CO2; 0.349, 0.654, tmp_cumulo+AN_cumulo+interv al_yield+interval_Pdt.+RS_cumulo+rab_integral+RQ_integral+c onsum_O2+generate_CO2; 0.338, 0.654, tmp+pH_cumulo+interval _yield+OD+Feed_Vol+Cell_conc._interval+RQ_integral+AS_Vol.+ consum_O2+generate_CO2; 0.338, 0.654, tmp+AN+interval_yield +interval_Pdt.+RS_cumulo+RQ_integral+emission_O2+emission_C O2+consum_O2+generate_CO2; 0.317, 0.654, tmp_cumulo+pH_cumu lo+interval_yield+interval_Pdt.+OD+Feed_Vol+RS_cumulo+RQ_in tegral+AS_Vol.+emission_O2+consum_O2+generate_CO2; 0.338, 0. 654, tmp_cumulo+pH_cumulo+AN_cumulo+interval_yield+interval _Pdt.+OD+rab_integral+RQ_integral+consum_O2+generate_CO2; 0. 328, 0.654, tmp_cumulo+pH_cumulo+PL_cumulo+interval_yield+i nterval_Pdt.+OD+Cell_conc._interval+RQ_integral+AS_Vol.+con sum_O2+generate_CO2; 0.328, 0.654, tmp_cumulo+pH_cumulo+AN+ interval_yield+interval_Pdt.+RS_cumulo+RQ_integral+AS_Vol.+ consum_O2+interval_O2+generate_CO2; 0.306, 0.654, tmp_cumul o+pH_cumulo+AN+interval_yield+interval_Pdt.+OD+rab_integral +RQ_integral+AS_Vol.+emission_O2+emission_CO2+consum_O2+gen erate_CO2; 0.338, 0.654, tmp_cumulo+interval_yield+interval _Pdt.+RS_cumulo+RQ_integral+AS_Vol.+emission_O2+consum_O2+i nterval_O2+generate_CO2; 0.317, 0.654, tmp+tmp_cumulo+pH_cu mulo+interval_yield+interval_Pdt.+OD+RQ_integral+AS_Vol.+em ission_O2+emission_CO2+consum_O2+generate_CO2; 0.338, 0.654, tmp_cumulo+PL_cumulo+AN_cumulo+interval_yield+interval_Pdt. +RS_cumulo+RQ_integral+emission_CO2+consum_O2+generate_CO2; 0.317, 0.654, tmp+pH_cumulo+AN+interval_yield+interval_Pdt. +OD+Feed_Vol+RQ_integral+AS_Vol.+emission_CO2+consum_O2+gen erate_CO2; 0.328, 0.654, tmp_cumulo+pH+pH_cumulo+interval_y ield+interval_Pdt.+OD+RS_cumulo+rab_integral+RQ_integral+co nsum_O2+generate_CO2; 0.338, 0.654, tmp_cumulo+pH_cumulo+AN +interval_yield+OD+RS_cumulo+RQ_integral+consum_O2+interval _O2+generate_CO2; 0.358, 0.654, tmp_cumulo+interval_yield+O D+RS_cumulo+rab_integral+RQ_integral+consum_O2+generate_CO 2; 0.317, 0.654, tmp_cumulo+interval_yield+interval_Pdt.+OD +Feed_Vol+RS_cumulo+rab_integral+RQ_integral+AS_Vol.+emissi on_CO2+consum_O2+generate_CO2; 0.328, 0.654, tmp_cumulo+int erval_yield+interval_Pdt.+Feed_Vol+RS_cumulo+RQ_integral+AS _Vol.+emission_CO2+consum _O2+interval_O2+generate_CO2; 0.31 7, 0.654, tmp_cumulo+pH_cumulo+AN_cumulo+interval_yield+int erval_Pdt.+OD+rab_integral+RQ_integral+AS_Vol.+emission_O2+ consum_O2+generate_CO2; 0.349, 0.654, tmp+tmp_cumulo+interv al_yield+interval_Pdt.+RS_cumulo+RQ_integral+emission_O2+co nsum_O2+generate_CO2; 0.349, 0.654, tmp_cumulo+pH+interval_ yield+interval_Pdt.+RS_cumulo+RQ_integral+emission_O2+consu m_O2+generate_CO2; 0.306, 0.654, tmp+pH_cumulo+interval_yie ld+interval_Pdt.+OD+Feed_Vol+rab_integral+RQ_integral+AS_Vo l.+emission_CO2+consum _O2+interval_O2+generate_CO2; 0.306, 0.654, tmp_cumulo+pH_cumulo+AN+interval_yield+interval_Pdt. +OD+RS_cumulo+rab_integral+RQ_integral+AS_Vol.+emission_CO2 +consum_O2+generate_CO2; 0.328, 0.654, tmp_cumulo+pH+pH_cum ulo+AN_cumulo+interval_yield+interval_Pdt.+OD+RQ_integral+A S_Vol.+consum_O2+generate_CO2; 0.358, 0.654, tmp_cumulo+int erval_yield+OD+RS_cumulo+RQ_integral+emission_CO2+consum_O2 +generate_CO2; 0.328, 0.654, tmp_cumulo+pH_cumulo+interval_ yield+OD+RS_cumulo+Cell_conc._interval+rab_integral+RQ_inte gral+AS_Vol.+consum_O2+generate_CO2; 0.338, 0.654, tmp_cumu lo+pH+AN_cumulo+interval_yield+interval_Pdt.+RQ_integral+AS _Vol.+emission_CO2+consum_O2+generate_CO2; 0.328, 0.654, tm p+tmp_cumulo+pH_cumulo+AN_cumulo+interval_yield+OD+rab_inte gral+RQ_integral+AS_Vol.+consum_O2+generate_CO2; 0.317, 0.6 54, tmp_cumulo+pH+pH_cumulo+interval_yield+OD+RS_cumulo+rab _integral+RQ_integral+AS_Vol.+consum_O2+interval_O2+generat e_CO2; 0.338, 0.654, tmp_cumulo+interval_yield+interval_Pdt. +OD+RS_cumulo+rab_integral+RQ_integral+emission_O2+consum_O 2+generate_CO2; 0.338, 0.654, tmp+pH_cumulo+interval_yield+ interval_Pdt.+OD+RS+RQ_integral+emission_CO2+consum_O2+gene rate_CO2; 0.338, 0.654, tmp_cumulo+pH_cumulo+interval_yield +OD+Feed_Vol+RS_cumulo+rab_integral+RQ_integral+consum_O2+g enerate_CO2; 0.338, 0.654, tmp+pH_cumulo+interval_yield+OD+ Cell_conc._interval+RQ_integral+AS_Vol.+emission_CO2+consum _O2+generate_CO2; 0.328, 0.654, tmp_cumulo+interval_yield+i nterval_Pdt.+OD+RS_cumulo+RQ_integral+AS_Vol.+emission_CO2+ consum_O2+interval_O2+generate_CO2; 0.338, 0.654, tmp+inter val_yield+interval_Pdt.+OD+Feed_Vol+RS_cumulo+RQ_integral+e mission_CO2+consum_O2+generate_CO2; 0.338, 0.654, tmp_cumul o+pH+AN_cumulo+interval_yield+interval_Pdt.+RS_cumulo+RQ_in tegral+emission_CO2+consum_O2+generate_CO2; 0.328, 0.654, t mp+pH_cumulo+interval_yield+interval_Pdt.+Cell_conc._interv al+rab_integral+RQ_integral+AS_Vol.+emission_CO2+consum_O2+ generate_CO2; 0.338, 0.654, tmp+tmp_cumulo+AN_cumulo+interv al_yield+interval_Pdt.+RS_cumulo+RQ_integral+emission_CO2+c onsum_O2+generate_CO2; 0.338, 0.654, tmp+pH_cumulo+PL_cumul o+interval_yield+interval_Pdt.+RQ_integral+AS_Vol.+emission _CO2+consum_O2+generate_CO2; 0.358, 0.654, tmp_cumulo+pH_cu mulo+PL_cumulo+interval_yield+OD+RQ_integral+consum_O2+gene rate_CO2; 0.358, 0.654, tmp+interval_yield+interval_Pdt.+RQ _integral+Accum._Yield+AS_Vol.+consum_O2+generate_CO2; 0.32 8, 0.654, tmp_cumulo+pH+AN+interval_yield+interval_Pdt.+RS_ cumulo+RQ_integral+AS_Vol.+emission_CO2+consum_O2+generate_ CO2; 0.328, 0.654, tmp_cumulo+pH_cumulo+PL_cumulo+interval_ yield+interval_Pdt.+rab_integral+RQ_integral+AS_Vol.+emissi on_CO2+consum_O2+generate_CO2; 0.328, 0.654, tmp_cumulo+pH_ cumulo+PL_cumulo+interval_yield+interval_Pdt.+RS_cumulo+RQ_ integral+AS_Vol.+emission_CO2+consum_O2+generate_CO2; 0.317, 0.654, tmp_cumulo+interval_yield+interval_Pdt.+Feed_Vol+RS _cumulo+rab_integral+RQ_integral+AS_Vol.+emission_O2+emissi on_CO2+consum_O2+generate_CO2; 0.328, 0.654, tmp_cumulo+pH_ cumulo+interval_yield+interval_Pdt.+RS_cumulo+rab_integral+ RQ_integral+AS_Vol.+consum_O2+interval_O2+generate_CO2; 0.3 17, 0.654, tmp+pH_cumulo+PL_cumulo+interval_yield+interval_ Pdt.+OD+Feed_Vol+rab_integral+RQ_integral+AS_Vol.+consum_O2 +generate_CO2; 0.348, 0.654, tmp_cumulo+pH+pH_cumulo+interv al_yield+interval_Pdt.+RQ_integral+AS_Vol.+consum_O2+genera te_CO2; 0.328, 0.654, tmp_cumulo+AN_cumulo+interval_yield+i nterval_Pdt.+RS_cumulo+RQ_integral+AS_Vol.+emission_O2+emis sion_CO2+consum_O2+generate_CO2; 0.348, 0.654, tmp_cumulo+A N+interval_yield+interval_Pdt.+RQ_integral+AS_Vol.+consum_O 2+interval_O2+generate_CO2; 0.328, 0.654, tmp+tmp_cumulo+pH _cumulo+PL_cumulo+interval_yield+OD+RQ_integral+AS_Vol.+con sum_O2+interval_O2+generate_CO2; 0.338, 0.654, tmp_cumulo+p H+AN+interval_yield+interval_Pdt.+RQ_integral+AS_Vol.+consu m_O2+interval_O2+generate_CO2; 0.328, 0.654, tmp_cumulo+pH+ pH_cumulo+interval_yield+interval_Pdt.+RS_cumulo+RQ_integra l+AS_Vol.+emission_CO2+consum_O2+generate_CO2; 0.338, 0.654, tmp+interval_yield+interval_Pdt.+RS_cumulo+Cell_conc._inte rval+rab_integral+RQ_integral+emission_CO2+consum_O2+genera te_CO2; 0.338, 0.654, tmp+pH_cumulo+interval_yield+interval _Pdt.+OD+Cell_conc._interval+RQ_integral+emission_CO2+consu m_O2+generate_CO2; 0.328, 0.654, tmp+tmp_cumulo+pH_cumulo+P L_cumulo+interval_yield+interval_Pdt.+OD+RQ_integral+AS_Vol. +consum_O2+generate_CO2; 0.338, 0.654, tmp_cumulo+AN_cumulo +interval_yield+interval_Pdt.+Feed_Vol+RS_cumulo+RQ_integra 1+emission_CO2+consum _O2+generate_CO2; 0.328, 0.654, tmp+in terval_yield+interval_Pdt.+OD+RS_cumulo+RQ_integral+AS_Vol. +emission_O2+emission_CO2+consum_O2+generate_CO2; 0.348, 0. 654, tmp_cumulo+interval_yield+interval_Pdt.+RS_cumulo+RQ_i ntegral+Accum._Yield+emission_CO2+consum_O2+generate_CO2; 0. 338, 0.654, tmp_cumulo+pH+interval_yield+interval_Pdt.+OD+R Q_integral+AS_Vol.+emission_O2+consum_O2+generate_CO2; 0.32 8, 0.654, tmp_cumulo+pH_cumulo+AN+interval_yield+interval_P dt.+Feed_Vol+RQ_integral+AS_Vol.+emission_CO2+consum_O2+gen erate_CO2; 0.338, 0.654, tmp+AN+interval_yield+interval_Pdt. +RQ_integral+AS_Vol.+emission_O2+emission_CO2+consum_O2+gen erate_CO2; 0.338, 0.654, tmp_cumulo+pH+interval_yield+inter val_Pdt.+RS_cumulo+RQ_integral+AS_Vol.+consum_O2+interval_O 2+generate_CO2; 0.317, 0.654, tmp+tmp_cumulo+pH_cumulo+inte rval_yield+interval_Pdt.+OD+RQ_integral+AS_Vol.+emission_O2 +consum_O2+interval_O2+generate_CO2; 0.317, 0.654, tmp_cumu lo+pH_cumulo+PL_cumulo+interval_yield+interval_Pdt.+OD+RS_c umulo+RQ_integral+AS_Vol.+emission_CO2+consum_O2+generate_C 02; 0.348, 0.654, tmp_cumulo+pH+interval_yield+interval_Pdt. +RS_cumulo+rab_integral+RQ_integral+consum_O2+generate_CO2; 0.338, 0.654, tmp+tmp_cumulo+pH+pH_cumulo+interval_yield+O D+RQ_integral+AS_Vol.+consum_O2+generate_CO2; 0.338, 0.654, tmp+interval_yield+interval_Pdt.+rab_integral+RQ_integral+ AS_Vol.+emission_CO2+consum_O2+interval_O2+generate_CO2; 0. 317, 0.654, tmp_cumulo+pH_cumulo+AN_cumulo+interval_yield+O D+Cell_conc._interval+rab_integral+RQ_integral+AS_Vol.+cons um_O2+interval_O2+generate_CO2; 0.327, 0.654, tmp+pH_cumulo +interval_yield+interval_Pdt.+rab_integral+RQ_integral+AS_V ol.+emission_CO2+consum_O2+interval_O2+generate_CO2; 0.348, 0.654, tmp+interval_yield+interval_Pdt.+OD+Cell_conc._inte rval+RQ_integral+emission_CO2+consum_O2+generate_CO2; 0.338, 0.654, tmp+interval_yield+interval_Pdt.+Feed_Vol+RS_cumulo +RQ_integral+AS_Vol.+consum_O2+interval_O2+generate_CO2; 0. 327, 0.654, tmp_cumulo+pH_cumulo+AN+interval_yield+OD+Feed_ Vol+RQ_integral+AS_Vol.+emission_CO2+consum_O2+generate_CO 2; 0.317, 0.654, tmp_cumulo+pH+pH_cumulo+AN+interval_yield+ interval_Pdt.+OD+RQ_integral+AS_Vol.+consum_O2+interval_O2+ generate_CO2

### [207. Linear Model that Predicts Arginine Product ion Amount in Interval 7]

0.316, 0.661, pH_cumulo+PL+AN+interval_yield+OD+RS+Feed_Vol +RS_cumulo+Accum._Yield+AS_Vol.+emission_CO2+consum_O2+gene rate_CO2; 0.324, 0.658, pH_cumulo+PL+AN+interval_yield+OD+R S+Feed_Vol+RS_cumulo+Accum._Yield+AS_Vol.+consum_O2+generat e_CO2; 0.319, 0.656, pH_cumulo+PL+AN+interval_yield+OD+RS+R S_cumulo+Accum._Yield+AS_Vol.+emission_CO2+consum_O2+genera te_CO2; 0.330, 0.655, pH_cumulo+PL+AN+interval_yield+OD+RS+ RS_cumulo+Accum._Yield+AS_Vol.+consum_O2+generate_CO2; 0.32 9, 0.655, pH_cumulo+PL+AN+interval_yield+OD+RS+RS_cumulo+AS _Vol.+emission_CO2+consum_O2+generate_CO2; 0.305, 0.654, pH _cumulo+PL+AN+interval_yield+OD+RS+Feed_Vol+RS_cumulo+rab_i ntegral+Accum._Yield+AS_Vol.+consum_O2+generate_CO2; 0.314, 0.652, pH_cumulo+PL+AN+interval_yield+OD+RS+Feed_Vol+RS_cu mulo+AS_Vol.+emission_CO2+consum_O2+generate_CO2; 0.292, 0. 652, pH_cumulo+PL+AN+AN_cumulo+interval_yield+OD+RS+Feed_Vo l+RS_cumulo+Accum._Yield+AS_Vol.+emission_CO2+consum_O2+gen erate_CO2; 0.334, 0.651, pH_cumulo+PL+interval_yield+OD+RS+ RS_cumulo+AS_Vol.+emission_CO2+consum_O2+generate_CO2; 0.30 1, 0.651, pH_cumulo+PL+AN+AN_cumulo+interval_yield+OD+RS+Fe ed_Vol+RS_cumulo+Accum._Yield+AS_Vol.+consum_O2+generate_CO 2; 0.289, 0.651, pH_cumulo+PL+AN+interval_yield+OD+RS+Feed_ Vol+RS_cumulo+rab_integral+Accum._Yield+AS_Vol.+emission_CO 2+consum_O2+generate_CO2; 0.311, 0.650, pH_cumulo+PL+AN+int erval_yield+OD+RS+Feed_Vol+RS_cumulo+RQ_integral+Accum._Yie ld+AS_Vol.+consum_O2; 0.286, 0.649, pH+pH_cumulo+PL+AN+inte rval_yield+OD+RS+Feed_Vol+RS_cumulo+Accum._Yield+AS_Vol.+em ission_CO2+consum_O2+generate_CO2; 0.319, 0.648, pH_cumulo+ PL+AN_cumulo+interval_yield+OD+RS+RS_cumulo+AS_Vol.+emissio n_CO2+consum_O2+generate_CO2; 0.308, 0.648, pH_cumulo+PL+AN _cumulo+interval_yield+OD+RS+RS_cumulo+Accum._Yield+AS_Vol. +emission_CO2+consum _O2+generate_CO2; 0.306, 0.647, pH_cumu lo+PL+AN+interval_yield+OD+RS+RS_cumulo+rab_integral+Accum. _Yield+AS_Vol.+consum_O2+generate_CO2; 0.306, 0.647, pH_cum ulo+PL+AN+AN_cumulo+interval_yield+OD+RS+RS_cumulo+Accum._Y ield+AS_Vol.+consum_O2+generate_CO2; 0.294, 0.647, pH_cumul o+PL+AN+AN_cumulo+interval_yield+OD+RS+RS_cumulo+Accum._Yie ld+AS_Vol.+emission_CO2+consum_O2+generate_CO2; 0.294, 0.64 6, pH_cumulo+PL+AN+interval_yield+OD+RS+Feed_Vol+RS_cumulo+ Cell_conc._interval+Accum._Yield+AS_Vol.+consum_O2+generate _CO2; 0.294, 0.646, pH_cumulo+PL+AN+interval_yield+OD+RS+Fe ed_Vol+RS_cumulo+Accum._Yield+AS_Vol.+consum_O2+interval_O2 +generate_CO2; 0.305, 0.646, pH_cumulo+PL+AN+interval_yield +OD+RS+RS_cumulo+Cell_conc._interval+Accum._Yield+AS_Vol.+c onsum_O2+generate_CO2; 0.305, 0.646, pH_cumulo+PL+AN+interv al_yield+OD+RS+RS_cumulo+Accum._Yield+AS_Vol.+consum_O2+int erval_O2+generate_CO2; 0.281, 0.646, pH_cumulo+PL+AN+interv al_yield+OD+RS+Feed_Vol+RS_cumulo+Accum._Yield+AS_Vol.+emis sion_CO2+consum_O2+interval_O2+generate_CO2; 0.315, 0.646, pH_cumulo+PL+interval_yield+OD+RS+RS_cumulo+Accum._Yield+AS _Vol.+emission_CO2+consum_O2+generate_CO2; 0.293, 0.646, pH _cumulo+PL+AN_cumulo+interval_yield+OD+RS+Feed_Vol+RS_cumul o+Accum._Yield+AS_Vol.+emission_CO2+consum_O2+generate_CO2; 0.281, 0.646, tmp+pH_cumulo+PL+AN+interval_yield+OD+RS+Fee d_Vol+RS_cumulo+Accum._Yield+AS_Vol.+emission_CO2+consum_O2 +generate_CO2; 0.292, 0.646, pH+pH_cumulo+PL+AN+interval_yi eld+OD+RS+Feed_Vol+RS_cumulo+Accum._Yield+AS_Vol.+consum_O2 +generate_CO2; 0.280, 0.645, pH_cumulo+PL+AN+interval_yield +OD+RS+Feed_Vol+RS_cumulo+Cell_conc._interval+Accum._Yield+ AS_Vol.+emission_CO2+consum_O2+generate_CO2; 0.304, 0.645, pH_cumulo+PL+AN+OD+RS+Feed_Vol+RS_cumulo+Accum._Yield+AS_Vo l.+emission_CO2+consum_O2+generate_CO2; 0.315, 0.645, pH_cu mulo+PL+interval_yield+OD+RS+Feed_Vol+RS_cumulo+AS_Vol.+emi ssion_CO2+consum_O2+generate_CO2; 0.292, 0.645, pH_cumulo+P L+AN+interval_yield+interval_Pdt.+OD+RS+Feed_Vol+RS_cumulo+ Accum._Yield+AS_Vol.+consum_O2+generate_CO2; 0.280, 0.645, pH_cumulo+PL+AN+AN_cumulo+interval_yield+OD+RS+Feed_Vol+RS_ cumulo+rab_integral+Accum._Yield+AS_Vol.+consum_O2+generate _CO2; 0.314, 0.645, pH_cumulo+PL+AN+OD+RS+Feed_Vol+RS_cumul o+Accum._Yield+AS_Vol.+consum_O2+generate_CO2; 0.290, 0.644, tmp+pH_cumulo+PL+AN+interval_yield+OD+RS+Feed_Vol+RS_cumul o+Accum._Yield+AS_Vol.+consum_O2+generate_CO2; 0.290, 0.644, pH_cumulo+PL+AN+interval_yield+OD+RS+Feed_Vol+RS_cumulo+ra b_integral+RQ_integral+Accum._Yield+AS_Vol.+consum_O2; 0.30 2, 0.644, pH_cumulo+PL+AN+AN_cumulo+interval_yield+OD+RS+RS _cumulo+AS_Vol.+emission_CO2+consum _O2+generate_CO2; 0.290, 0.644, pH_cumulo+PL+AN+interval_yield+OD+RS+Feed_Vol+RS_cu mulo+Accum._Yield+AS_Vol.+emission_O2+consum_O2+generate_CO 2; 0.290, 0.644, pH_cumulo+PL+AN+AN_cumulo+interval_yield+O D+RS+Feed_Vol+RS_cumulo+RQ_integral+Accum._Yield+AS_Vol.+co nsum_O2; 0.290, 0.644, pH_cumulo+PL+AN+interval_yield+OD+RS +RS_cumulo+Accum._Yield+AS_Vol.+emission_CO2+consum_O2+inte rval_O2+generate_CO2; 0.323, 0.644, pH_cumulo+PL+AN+OD+RS+R S_cumulo+Accum._Yield+AS_Vol.+consum_O2+generate_CO2; 0.289, 0.644, pH_cumulo+PL+AN+interval_yield+OD+RS+RS_cumulo+rab_ integral+Accum._Yield+AS_Vol.+emission_CO2+consum_O2+genera te_CO2; 0.289, 0.643, pH_cumulo+PL+AN+interval_yield+OD+RS+ Feed_Vol+RS_cumulo+RQ_integral+Accum._Yield+AS_Vol.+emissio n_O2+consum_O2; 0.289, 0.643, pH_cumulo+PL+AN+AN_cumulo+OD+ RS+Feed_Vol+RS_cumulo+Accum._Yield+AS_Vol.+emission_CO2+con sum_O2+generate_CO2; 0.311, 0.643, pH_cumulo+PL+AN+interval _yield+OD+RS+RS_cumulo+RQ_integral+Accum._Yield+AS_Vol.+con sum_O2; 0.300, 0.643, pH_cumulo+PL+AN+interval_yield+OD+RS+ RS_cumulo+rab_integral+AS_Vol.+emission_CO2+consum_O2+gener ate_CO2; 0.300, 0.643, pH_cumulo+PL+AN_cumulo+interval_yiel d+OD+RS+Feed_Vol+RS_cumulo+AS_Vol.+emission_CO2+consum_O2+g enerate_CO2; 0.276, 0.643, tmp_cumulo+pH_cumulo+PL+AN+inter val_yield+OD+RS+Feed_Vol+RS_cumulo+Accum._Yield+AS_Vol.+emi ssion_CO2+consum_O2+generate_CO2; 0.310, 0.643, pH_cumulo+P L+AN+OD+RS+RS_cumulo+Accum._Yield+AS_Vol.+emission_CO2+cons um_O2+generate_CO2; 0.288, 0.642, pH_cumulo+PL+AN+AN_cumulo +interval_yield+OD+RS+Feed_Vol+RS_cumulo+AS_Vol.+emission_C O2+consum_O2+generate_CO2; 0.288, 0.642, tmp_cumulo+pH_cumu lo+PL+AN+interval_yield+OD+RS+Feed_Vol+RS_cumulo+Accum._Yie ld+AS_Vol.+consum_O2+generate_CO2; 0.287, 0.642, pH_cumulo+ PL+AN+interval_yield+OD+RS+Feed_Vol+RS_cumulo+rab_integral+ AS_Vol.+emission_CO2+consum_O2+generate_CO2; 0.310, 0.642, pH_cumulo+PL+AN+OD+RS+RS_cumulo+Accum._Yield+AS_Vol.+consum _O2+interval_O2+generate_CO2; 0.263, 0.642, pH_cumulo+PL+AN +AN_cumulo+interval_yield+OD+RS+Feed_Vol+RS_cumulo+rab_inte gral+Accum._Yield+AS_Vol.+emission_CO2+consum_O2+generate_C O2; 0.287, 0.642, pH_cumulo+PL+AN+interval_yield+OD+RS+RS_c umulo+Cell_conc._interval+Accum._Yield+AS _Vol.+emission_CO2 +consum_O2+generate_CO2; 0.298, 0.642, pH_cumulo+PL+AN+AN_c umulo+OD+RS+Feed_Vol+RS_cumulo+Accum._Yield+AS_Vol.+consum_ O2+generate_CO2; 0.298, 0.642, pH_cumulo+PL+AN+interval_yie ld+OD+RS+RS_cumulo+Accum._Yield+AS_Vol.+emission_O2+consum_ O2+generate_CO2; 0.309, 0.642, pH_cumulo+PL+AN+interval_yie ld+OD+RS+Feed_Vol+RS_cumulo+Accum._Yield+AS_Vol.+consum_O2; 0.274, 0.642, pH+pH_cumulo+PL+AN+interval_yield+OD+RS+Feed _Vol+RS_cumulo+rab_integral+Accum._Yield+AS_Vol.+consum_O2+ generate_CO2; 0.319, 0.641, pH_cumulo+PL+AN+interval_yield+ OD+RS+RS_cumulo+AS_Vol.+consum_O2+generate_CO2; 0.274, 0.64 1, pH_cumulo+PL+AN+interval_yield+OD+RS+Feed_Vol+RS_cumulo+ Cell_conc._interval+rab_integral+Accum._Yield+AS_Vol.+consu m_O2+generate_CO2; 0.319, 0.641, pH_cumulo+PL+AN+interval_y ield+OD+RS+RS_cumulo+RQ_integral+AS_Vol.+consum_O2; 0.297, 0.641, pH_cumulo+PL+interval_yield+OD+RS+Feed_Vol+RS_cumulo +Accum._Yield+AS_Vol.+emission_CO2+consum_O2+generate_CO2; 0.297, 0.641, pH_cumulo+PL+AN+OD+RS+Feed_Vol+RS_cumulo+Accu m._Yield+AS_Vol.+consum_O2+interval_O2+generate_CO2; 0.261, 0.641, pH+pH_cumulo+PL+AN+AN_cumulo+interval_yield+OD+RS+F eed_Vol+RS_cumulo+Accum._Yield+AS_Vol.+emission_CO2+consum_ O2+generate_CO2; 0.285, 0.641, pH+pH_cumulo+PL+AN+interval_ yield+OD+RS+RS_cumulo+Accum._Yield+AS_Vol.+emission_CO2+con sum_O2+generate_CO2; 0.328, 0.640, pH_cumulo+PL+interval_yi eld+OD+RS+RS_cumulo+emission_CO2+consum_O2+generate_CO2; 0. 296, 0.640, pH_cumulo+PL+AN+interval_yield+OD+RS+Feed_Vol+R S_cumulo+rab_integral+Accum._Yield+AS_Vol.+consum_O2; 0.296, 0.640, pH_cumulo+PL+AN+interval_yield+interval_Pdt.+OD+RS+ Feed_Vol+Accum._Yield+AS_Vol.+consum_O2+generate_CO2; 0.272, 0.640, pH_cumulo+PL+AN+interval_yield+OD+RS+Feed_Vol+RS_cu mulo+rab_integral+Accum._Yield+AS_Vol.+consum_O2+interval_O 2+generate_CO2; 0.295, 0.640, pH_cumulo+PL+AN+interval_yiel d+OD+RS+RS_cumulo+Cell_conc._interval+AS_Vol.+emission_CO2+ consum_O2+generate_CO2; 0.295, 0.640, tmp+pH_cumulo+PL+AN+i nterval_yield+OD+RS+RS_cumulo+Accum._Yield+AS_Vol.+consum_O 2+generate_CO2; 0.295, 0.640, pH_cumulo+PL+AN+interval_yiel d+OD+RS+RS_cumulo+AS_Vol.+emission_CO2+consum_O2+interval_O 2+generate_CO2; 0.295, 0.640, tmp+pH_cumulo+PL+AN+interval_ yield+OD+RS+RS_cumulo+AS_Vol.+emission_CO2+consum_O2+genera te_CO2; 0.306, 0.640, pH_cumulo+PL+interval_yield+OD+RS+RS_ cumulo+rab_integral+AS_Vol.+emission_CO2+consum_O2+generate _CO2; 0.283, 0.639, tmp+pH_cumulo+PL+AN+interval_yield+OD+R S+RS_cumulo+Accum._Yield+AS_Vol.+emission_CO2+consum_O2+gen erate_CO2; 0.271, 0.639, pH_cumulo+PL+AN+interval_yield+int erval_Pdt.+OD+RS+Feed_Vol+RS_cumulo+rab_integral+Accum._Yie ld+AS_Vol.+consum_O2+generate_CO2; 0.294, 0.639, pH_cumulo+ PL+AN+OD+RS+RS_cumulo+Accum._Yield+AS_Vol.+emission_CO2+con sum_O2+interval_O2+generate_CO2; 0.306, 0.639, pH_cumulo+PL +AN_cumulo+interval_yield+OD+RS+RS_cumulo+Accum._Yield+AS_V ol.+consum_O2+generate_CO2; 0.306, 0.639, pH_cumulo+PL+inte rval_yield+OD+RS+RS_cumulo+AS_Vol.+emission_CO2+consum_O2+i nterval_O2+generate_CO2; 0.294, 0.639, pH_cumulo+PL+AN+AN_c umulo+OD+RS+RS_cumulo+Accum._Yield+AS_Vol.+emission_CO2+con sum_O2+generate_CO2; 0.294, 0.639, tmp_cumulo+pH_cumulo+PL+ AN+interval_yield+OD+RS+RS_cumulo+Accum._Yield+AS_Vol.+cons um_O2+generate_CO2; 0.294, 0.639, pH+pH_cumulo+PL+AN+interv al_yield+OD+RS+RS_cumulo+AS_Vol.+emission_CO2+consum_O2+gen erate_CO2; 0.283, 0.639, pH_cumulo+PL+AN+OD+RS+Feed_Vol+RS_ cumulo+Accum._Yield+AS_Vol.+emission_CO2+consum_O2+interval _O2+generate_CO2; 0.305, 0.639, pH_cumulo+PL+AN+AN_cumulo+O D+RS+RS_cumulo+Accum._Yield+AS_Vol.+consum_O2+generate_CO2; 0.294, 0.639, pH+pH_cumulo+PL+AN+interval_yield+OD+RS+RS_c umulo+Accum._Yield+AS_Vol.+consum_O2+generate_CO2; 0.305, 0. 639, pH_cumulo+PL+AN_cumulo+OD+RS+RS_cumulo+Accum._Yield+AS _Vol.+emission_CO2+consum_O2+generate_CO2; 0.258, 0.639, pH +pH_cumulo+PL+AN+interval_yield+OD+RS+Feed_Vol+RS_cumulo+ra b_integral+Accum._Yield+AS_Vol.+emission_CO2+consum_O2+gene rate_CO2; 0.282, 0.639, tmp+pH_cumulo+PL+AN+interval_yield+ OD+RS+Feed_Vol+RS_cumulo+AS_Vol.+emission_CO2+consum_O2+gen erate_CO2; 0.270, 0.639, pH_cumulo+PL+AN+interval_yield+int erval_Pdt.+OD+RS+Feed_Vol+RS_cumulo+Accum._Yield+AS_Vol.+em ission_CO2+consum_O2+generate_CO2; 0.270, 0.639, pH_cumulo+ PL+AN+interval_yield+OD+RS+Feed_Vol+RS_cumulo+rab_integral+ Accum._Yield+AS_Vol.+emission_O2+consum_O2+generate_CO2; 0. 269, 0.639, tmp+pH_cumulo+PL+AN+interval_yield+OD+RS+Feed_V ol+RS_cumulo+rab_integral+Accum._Yield+AS_Vol.+consum_O2+ge nerate_CO2; 0.281, 0.638, pH+pH_cumulo+PL+AN+interval_yield +OD+RS+Feed_Vol+RS_cumulo+AS_Vol.+emission_CO2+consum_O2+ge nerate_CO2; 0.269, 0.638, pH+pH_cumulo+PL+AN+AN_cumulo+inte rval_yield+OD+RS+Feed_Vol+RS_cumulo+Accum._Yield+AS_Vol.+co nsum_O2+generate_CO2; 0.281, 0.638, pH_cumulo+PL+AN+interva l_yield+OD+RS+Feed_Vol+RS_cumulo+RQ_integral+Accum._Yield+A S_Vol.+emission_CO2+consum_O2; 0.269, 0.638, pH_cumulo+PL+A N+interval_yield+OD+RS+Feed_Vol+RS_cumulo+RQ_integral+Accum. _Yield+AS_Vol.+emission_O2+emission_CO2+consum_O2; 0.269, 0. 638, pH_cumulo+PL+AN+AN_cumulo+interval_yield+OD+RS+Feed_Vo l+RS_cumulo+Cell_conc._interval+Accum._Yield+AS_Vol.+consum _O2+generate_CO2; 0.281, 0.638, tmp_cumulo+pH_cumulo+PL+AN+ interval_yield+OD+RS+RS_cumulo+Accum._Yield+AS_Vol.+emissio n_CO2+consum_O2+generate_CO2; 0.292, 0.638, tmp_cumulo+pH_c umulo+PL+AN+interval_yield+OD+RS+RS_cumulo+AS_Vol.+emission _CO2+consum_O2+generate_CO2; 0.256, 0.638, tmp+pH_cumulo+PL +AN+AN_cumulo+interval_yield+OD+RS+Feed_Vol+RS_cumulo+Accum. _Yield+AS_Vol.+emission_CO2+consum_O2+generate_CO2; 0.292, 0.638, pH_cumulo+PL+AN+OD+RS+Feed_Vol+RS_cumulo+rab_integra l+Accum._Yield+AS_Vol.+consum_O2+generate_CO2; 0.303, 0.638, pH+pH_cumulo+PL+interval_yield+OD+RS+RS_cumulo+AS_Vol.+emi ssion_CO2+consum_O2+generate_CO2; 0.280, 0.638, pH_cumulo+P L+AN+interval_yield+OD+RS+RS_cumulo+Cell_conc._interval+rab _integral+Accum._Yield+AS_Vol.+consum_O2+generate_CO2; 0.28 0, 0.638, pH_cumulo+PL+AN+interval_yield+OD+RS+RS_cumulo+ra b_integral+Accum._Yield+AS_Vol.+consum_O2+interval_O2+gener ate_CO2; 0.268, 0.638, pH_cumulo+PL+PL_cumulo+AN+interval_y ield+OD+RS+Feed_Vol+RS_cumulo+Accum._Yield+AS_Vol.+emission _CO2+consum_O2+generate_CO2; 0.280, 0.638, pH_cumulo+PL+AN+ AN_cumulo+interval_yield+OD+RS+RS_cumulo+Cell_conc._interva l+Accum._Yield+AS_Vol.+consum_O2+generate_CO2; 0.292, 0.637, pH_cumulo+PL+AN+interval_yield+OD+RS+RS_cumulo+rab_integra l+RQ_integral+Accum._Yield+AS_Vol.+consum_O2; 0.267, 0.637, pH_cumulo+PL+AN+AN_cumulo+interval_yield+OD+RS+Feed_Vol+RS _cumulo+RQ_integral+Accum._Yield+AS_Vol.+emission_O2+consum _O2; 0.279, 0.637, pH_cumulo+PL+AN+AN_cumulo+interval_yield +OD+RS+RS_cumulo+rab_integral+Accum._Yield+AS_Vol.+consum_O 2+generate_CO2; 0.279, 0.637, pH_cumulo+PL+AN_cumulo+interv al_yield+OD+RS+RS_cumulo+rab_integral+Accum._Yield+AS_Vol.+ emission _CO2+consum _O2+generate_CO2; 0.255, 0.637, pH_cumul o+PL+AN+AN_cumulo+interval_yield+OD+RS+Feed_Vol+RS_cumulo+C ell_conc._interval+Accum._Yield+AS_Vol.+emission_CO2+consum _O2+generate_CO2; 0.267, 0.637, pH_cumulo+PL+AN+AN_cumulo+i nterval_yield+OD+RS+Feed_Vol+RS_cumulo+rab_integral+RQ_inte gral+Accum._Yield+AS_Vol.+consum_O2; 0.291, 0.637, pH_cumul o+PL+AN_cumulo+interval_yield+OD+RS+RS_cumulo+rab_integral+ AS_Vol.+emission_CO2+consum_O2+generate_CO2; 0.279, 0.637, pH+pH_cumulo+PL+AN+interval_yield+OD+RS+Feed_Vol+RS_cumulo+ RQ_integral+Accum._Yield+AS_Vol.+consum_O2; 0.267, 0.637, t mp+pH_cumulo+PL+AN+AN_cumulo+interval_yield+OD+RS+Feed_Vol+ RS_cumulo+Accum._Yield+AS_Vol.+consum_O2+generate_CO2; 0.29 1, 0.637, pH_cumulo+PL+AN+interval_yield+OD+RS+RS_cumulo+RQ _integral+Accum._Yield+AS_Vol.+emission_O2+consum_O2; 0.267, 0.637, pH_cumulo+PL+AN+AN_cumulo+interval_yield+OD+RS+Feed _Vol+RS_cumulo+Accum._Yield+AS_Vol.+consum_O2+interval_O2+g enerate_CO2; 0.267, 0.637, pH_cumulo+PL+AN+interval_yield+O D+RS+Feed_Vol+RS_cumulo+rab_integral+RQ_integral+Accum._Yie ld+AS_Vol.+emission_O2+consum_O2; 0.279, 0.637, tmp_cumulo+ pH_cumulo+PL+AN+interval_yield+OD+RS+Feed_Vol+RS_cumulo+AS_ Vol.+emission_CO2+consum_O2+generate_CO2; 0.313, 0.637, pH_ cumulo+PL+AN_cumulo+interval_yield+OD+RS+RS_cumulo+emission _CO2+consum_O2+generate_CO2; 0.279, 0.637, pH_cumulo+PL+AN+ interval_yield+OD+RS+Feed_Vol+RS_cumulo+Cell_conc._interval +AS_Vol.+emission_CO2+consum_O2+generate_CO2; 0.290, 0.636, pH_cumulo+PL+AN+AN_cumulo+interval_yield+OD+RS+Feed_Vol+RS _cumulo+Accum._Yield+AS_Vol.+consum_O2; 0.290, 0.636, pH_cu mulo+PL+AN+AN_cumulo+interval_yield+OD+RS+RS_cumulo+RQ_inte gral+Accum._Yield+AS_Vol.+consum_O2; 0.290, 0.636, pH_cumul o+PL+interval_yield+OD+RS+RS_cumulo+Accum._Yield+AS_Vol.+em ission_CO2+consum_O2+interval_O2+generate_CO2; 0.253, 0.636, pH_cumulo+PL+AN+AN_cumulo+interval_yield+OD+RS+Feed_Vol+RS _cumulo+Accum._Yield+AS_Vol.+emission_CO2+consum_O2+interva l_O2+generate_CO2; 0.301, 0.636, pH_cumulo+PL+interval_yiel d+OD+RS+RS_cumulo+Cell_conc._interval+AS_Vol.+emission_CO2+ consum_O2+generate_CO2; 0.253, 0.636, pH_cumulo+PL+AN+inter val_yield+OD+RS+Feed_Vol+RS_cumulo+rab_integral+Accum._Yiel d+AS_Vol.+emission_CO2+consum_O2+interval_O2+generate_CO2; 0.278, 0.636, pH+pH_cumulo+PL+AN+OD+RS+Feed_Vol+RS_cumulo+A ccum._Yield+AS_Vol.+emission_CO2+consum _O2+generate_CO2; 0. 300, 0.636, pH_cumulo+PL+AN+interval_yield+OD+RS+Feed_Vol+R S_cumulo+RQ_integral+AS_Vol.+consum_O2; 0.265, 0.636, tmp_c umulo+pH_cumulo+PL+AN+interval_yield+OD+RS+Feed_Vol+RS_cumu lo+rab_integral+Accum._Yield+AS_Vol.+consum_O2+generate_CO 2; 0.322, 0.636, pH_cumulo+PL+interval_yield+OD+RS+RS_cumul O+RQ_integral+AS_Vol.+consum_O2; 0.300, 0.636, pH_cumulo+PL +AN+OD+RS+RS_cumulo+Cell_conc._interval+Accum._Yield+AS_Vol. +consum_O2+generate_CO2; 0.265, 0.636, pH_cumulo+PL+AN+AN_c umulo+interval_yield+OD+RS+Feed_Vol+RS_cumulo+Accum._Yield+ AS_Vol.+emission_O2+consum_O2+generate_CO2; 0.277, 0.636, p H_cumulo+PL+PL_cumulo+AN+interval_yield+OD+RS+Feed_Vol+RS_c umulo+Accum._Yield+AS_Vol.+consum_O2+generate_CO2; 0.300, 0. 636, tmp+pH_cumulo+PL+interval_yield+OD+RS+RS_cumulo+AS_Vol. +emission_CO2+consum_O2+generate_CO2; 0.277, 0.636, pH_cumu lo+PL+AN+AN_cumulo+interval_yield+OD+RS+Feed_Vol+RS_cumulo+ rab_integral+Accum._Yield+AS_Vol.+consum_O2; 0.311, 0.636, pH_cumulo+PL+AN+interval_yield+OD+RS+RS_cumulo+emission_CO2 +consum_O2+generate_CO2; 0.300, 0.635, pH_cumulo+PL+AN+inte rval_yield+OD+RS+Feed_Vol+Accum._Yield+AS_Vol.+consum_O2+ge nerate_CO2; 0.252, 0.635, tmp_cumulo+pH_cumulo+PL+AN+AN_cum ulo+interval_yield+OD+RS+Feed_Vol+RS_cumulo+Accum._Yield+AS _Vol.+emission_CO2+consum_O2+generate_CO2; 0.264, 0.635, pH _cumulo+PL+AN+interval_yield+OD+RS+Feed_Vol+RS_cumulo+Accum. _Yield+AS_Vol.+emission_O2+emission_CO2+consum_O2+generate_ CO2; 0.288, 0.635, pH_cumulo+PL+AN+interval_yield+interval_ Pdt.+OD+RS+RS_cumulo+Accum._Yield+AS_Vol.+consum_O2+generat e_CO2; 0.252, 0.635, pH_cumulo+PL+AN+interval_yield+OD+RS+F eed_Vol+RS_cumulo+Cell_conc._interval+rab_integral+Accum._Y ield+AS_Vol.+emission_CO2+consum_O2+generate_CO2; 0.276, 0. 635, pH_cumulo+PL+AN+AN_cumulo+interval_yield+OD+RS+RS_cumu lo+Accum._Yield+AS_Vol.+consum_O2+interval_O2+generate_CO2; 0.299, 0.635, pH_cumulo+PL+AN+interval_yield+OD+RS+RS_cumu lo+rab_integral+RQ_integral+AS_Vol.+consum_O2; 0.310, 0.635, pH_cumulo+PL+interval_yield+OD+RS+RS_cumulo+RQ_integral+Ac cum._Yield+AS_Vol.+consum_O2; 0.276, 0.635, pH_cumulo+PL+AN +interval_yield+interval_Pdt.+OD+RS+Feed_Vol+RS_cumulo+RQ_i ntegral+Accum._Yield+AS_Vol.+consum_O2; 0.264, 0.635, tmp_c umulo+pH_cumulo+PL+AN+AN_cumulo+interval_yield+OD+RS+Feed_V ol+RS_cumulo+Accum._Yield+AS_Vol.+consum_O2+generate_CO2; 0. 252, 0.635, tmp+pH_cumulo+PL+AN+interval_yield+OD+RS+Feed_V ol+RS_cumulo+rab_integral+Accum._Yield+AS_Vol.+emission_CO2 +consum_O2+generate_CO2; 0.264, 0.635, pH+pH_cumulo+PL+AN+i nterval_yield+OD+RS+Feed_Vol+RS_cumulo+Cell_conc._interval+ Accum._Yield+AS_Vol.+consum_O2+generate_CO2; 0.276, 0.635, pH_cumulo+PL+AN+interval_yield+OD+RS+Feed_Vol+RS_cumulo+Cel l_conc._interval+RQ_integral+Accum._Yield+AS_Vol.+consum_O 2; 0.287, 0.635, pH+pH_cumulo+PL+AN+OD+RS+Feed_Vol+RS_cumul o+Accum._Yield+AS_Vol.+consum_O2+generate_CO2; 0.251, 0.635, tmp+tmp_cumulo+pH_cumulo+PL+AN+interval_yield+OD+RS+Feed_V ol+RS_cumulo+Accum._Yield+AS_Vol.+emission_CO2+consum_O2+ge nerate_CO2; 0.276, 0.635, pH_cumulo+PL+AN+interval_yield+OD +RS+RS_cumulo+Cell_conc._interval+Accum._Yield+AS_Vol.+cons um_O2+interval_O2+generate_CO2; 0.276, 0.635, pH_cumulo+PL+ AN+interval_yield+OD+RS+Feed_Vol+RS_cumulo+AS_Vol.+emission _CO2+consum_O2+interval_O2+generate_CO2; 0.287, 0.635, pH_c umulo+PL+AN+interval_yield+OD+RS+Feed_Vol+Accum._Yield+AS_V ol.+emission_CO2+consum_O2+generate_CO2; 0.287, 0.635, pH_c umulo+PL+AN+OD+RS+Feed_Vol+RS_cumulo+Cell_conc._interval+Ac cum._Yield+AS_Vol.+consum_O2+generate_CO2; 0.287, 0.635, pH _cumulo+PL+AN_cumulo+OD+RS+Feed_Vol+RS_cumulo+Accum._Yield+ AS_Vol.+emission_CO2+consum_O2+generate_CO2; 0.263, 0.634, pH_cumulo+PL+AN+AN_cumulo+interval_yield+OD+RS+RS_cumulo+ra b_integral+Accum._Yield+AS_Vol.+emission_CO2+consum_O2+gene rate_CO2; 0.298, 0.634, pH_cumulo+PL+AN+interval_yield+OD+R S+Feed_Vol+RS_cumulo+AS_Vol.+consum_O2+generate_CO2; 0.251, 0.634, pH+pH_cumulo+PL+AN+interval_yield+OD+RS+Feed_Vol+RS _cumulo+Cell_conc._interval+Accum._Yield+AS_Vol.+emission_C O2+consum_O2+generate_CO2; 0.263, 0.634, pH_cumulo+PL+AN_cu mulo+interval_yield+OD+RS+Feed_Vol+RS_cumulo+rab_integral+A ccum._Yield+AS_Vol.+emission_CO2+consum_O2+generate_CO2; 0. 275, 0.634, pH_cumulo+PL+PL_cumulo+AN+interval_yield+OD+RS+ Feed_Vol+RS_cumulo+RQ_integral+Accum._Yield+AS_Vol.+consum_ 02; 0.275, 0.634, pH+pH_cumulo+PL+AN_cumulo+interval_yield+ OD+RS+RS_cumulo+Accum._Yield+AS_Vol.+emission_CO2+consum_O2 +generate_CO2; 0.275, 0.634, pH_cumulo+PL+AN+AN_cumulo+OD+R S+Feed_Vol+RS_cumulo+Accum._Yield+AS_Vol.+consum_O2+interva l_O2+generate_CO2; 0.286, 0.634, pH_cumulo+PL+interval_yiel d+OD+RS+Feed_Vol+RS_cumulo+rab_integral+AS_Vol.+emission_CO 2+consum_O2+generate_CO2; 0.263, 0.634, pH+pH_cumulo+PL+AN+ AN_cumulo+OD+RS+Feed_Vol+RS_cumulo+Accum._Yield+AS_Vol.+emi ssion_CO2+consum_O2+generate_CO2; 0.286, 0.634, pH_cumulo+P L+interval_yield+OD+RS+RS_cumulo+rab_integral+Accum._Yield+ AS_Vol.+emission_CO2+consum_O2+generate_CO2; 0.250, 0.634, pH+pH_cumulo+PL+AN+interval_yield+OD+RS+Feed_Vol+RS_cumulo+ Accum._Yield+AS_Vol.+emission_CO2+consum_O2+interval_O2+gen erate_CO2; 0.286, 0.634, pH_cumulo+PL+AN+AN_cumulo+OD+RS+RS _cumulo+Accum._Yield+AS_Vol.+consum_O2+interval_O2+generate _CO2; 0.298, 0.634, pH_cumulo+PL+AN+OD+RS+RS_cumulo+rab_int egral+Accum._Yield+AS_Vol.+consum_O2+generate_CO2; 0.262, 0. 634, pH_cumulo+PL+AN+AN_cumulo+interval_yield+interval_Pdt. +OD+RS+Feed_Vol+RS_cumulo+Accum._Yield+AS_Vol.+consum_O2+ge nerate_CO2; 0.286, 0.634, pH+pH_cumulo+PL+AN_cumulo+interva l_yield+OD+RS+RS_cumulo+AS_Vol.+emission_CO2+consum_O2+gene rate_CO2; 0.308, 0.634, pH_cumulo+PL+interval_yield+OD+RS+R S_cumulo+Accum._Yield+emission_CO2+consum_O2+generate-CO2; 0.297, 0.634, pH_cumulo+PL+AN+interval_yield+OD+RS+RS_cumul o+AS_Vol.+emission_O2+consum_O2+generate_CO2; 0.297, 0.634, tmp_cumulo+pH_cumulo+PL+interval_yield+OD+RS+RS_cumulo+AS_ Vol.+emission_CO2+consum_O2+generate_CO2; 0.262, 0.634, tmp +tmp_cumulo+pH_cumulo+PL+AN+interval_yield+OD+RS+Feed_Vol+R S_cumulo+Accum._Yield+AS_Vol.+consum_O2+generate_CO2; 0.274, 0.634, pH_cumulo+PL+AN+OD+RS+Feed_Vol+RS_cumulo+rab_integr al+Accum._Yield+AS_Vol.+emission_CO2+consum_O2+generate_CO 2; 0.297, 0.634, pH_cumulo+PL+AN+interval_yield+OD+RS+RS_cu mulo+rab_integral+AS_Vol.+consum_O2+generate_CO2; 0.297, 0. 634, pH_cumulo+PL+AN+AN_cumulo+interval_yield+OD+RS+RS_cumu lo+RQ_integral+AS_Vol.+consum_O2; 0.262, 0.634, pH+pH_cumul o+PL+AN+interval_yield+OD+RS+Feed_Vol+RS_cumulo+Accum._Yiel d+AS_Vol.+consum_O2+interval_O2+generate_CO2; 0.297, 0.633, pH_cumulo+PL+AN+interval_Pdt.+OD+RS+RS_cumulo+RQ_integral+ Accum._Yield+AS_Vol.+consum_O2; 0.262, 0.633, pH_cumulo+PL+ AN+AN_cumulo+OD+RS+Feed_Vol+RS_cumulo+Accum._Yield+AS_Vol.+ emission_CO2+consum_O2+interval_O2+generate_CO2; 0.297, 0.6 33, pH_cumulo+PL+AN_cumulo+interval_yield+OD+RS+RS_cumulo+A ccum._Yield+emission_CO2+consum_O2+generate_CO2; 0.285, 0.6 33, pH+PL+AN+interval_yield+OD+RS+Feed_Vol+RS_cumulo+Accum. _Yield+AS_Vol.+consum_O2+generate_CO2; 0.249, 0.633, pH+pH_ cumulo+PL+AN+AN_cumulo+interval_yield+OD+RS+Feed_Vol+RS_cum ulo+rab_integral+Accum._Yield+AS_Vol.+consum_O2+generate_CO 2; 0.273, 0.633, pH_cumulo+PL+AN+interval_yield+OD+RS+RS_cu mulo+Accum._Yield+AS_Vol.+emission_O2+consum_O2+interval_O2 +generate_CO2; 0.261, 0.633, pH_cumulo+PL+AN+interval_yield +OD+RS+Feed_Vol+RS_cumulo+Cell_conc._interval+Accum._Yield+ AS_Vol.+consum_O2+interval_O2+generate_CO2; 0.296, 0.633, p H_cumulo+PL+AN_cumulo+interval_yield+OD+RS+RS_cumulo+RQ_int egral+Accum._Yield+AS_Vol.+consum_O2; 0.273, 0.633, pH_cumu lo+PL+AN_cumulo+interval_yield+OD+RS+RS_cumulo+Accum._Yield +AS_Vol.+emission_CO2+consum_O2+interval_O2+generate_CO2; 0. 273, 0.633, pH_cumulo+PL+AN_cumulo+interval_yield+OD+RS+RS_ cumulo+Cell_conc._interval+Accum._Yield+AS_Vol.+emission_CO 2+consum_O2+generate_CO2; 0.273, 0.633, pH_cumulo+PL+AN+int erval_yield+OD+RS+Feed_Vol+RS_cumulo+rab_integral+Accum._Yi eld+AS_Vol.+emission_CO2+consum_O2; 0.285, 0.633, pH_cumulo +PL+AN+interval_yield+OD+RS+Feed_Vol+RS_cumulo+Accum._Yield +AS_Vol.+emission_CO2+consum_O2; 0.261, 0.633, pH_cumulo+PL +AN+AN_cumulo+interval_yield+OD+RS+RS_cumulo+Accum._Yield+A S_Vol.+emission_CO2+consum_O2+interval_O2+generate_CO2; 0.2 96, 0.633, pH_cumulo+PL+interval_yield+OD+RS+RS_cumulo+RQ_i ntegral+AS_Vol.+emission_O2+emission_CO2+consum_02; 0.273, 0.633, pH_cumulo+PL+AN+AN_cumulo+OD+RS+Feed_Vol+RS_cumulo+r ab_integral+Accum._Yield+AS_Vol.+consum_O2+generate_CO2; 0. 261, 0.633, pH+pH_cumulo+PL+AN_cumulo+interval_yield+OD+RS+ Feed_Vol+RS_cumulo+Accum._Yield+AS_Vol.+emission_CO2+consum _O2+generate_CO2; 0.261, 0.633, pH_cumulo+PL+AN+AN_cumulo+i nterval_yield+OD+RS+RS_cumulo+Cell_conc._interval+Accum._Yi eld+AS_Vol.+emission_CO2+consum_O2+generate_CO2; 0.284, 0.6 33, pH_cumulo+PL+AN+OD+RS+RS_cumulo+rab_integral+Accum._Yie ld+AS_Vol.+consum_O2+interval_O2+generate_CO2; 0.284, 0.633, pH_cumulo+PL+AN_cumulo+interval_yield+OD+RS+RS_cumulo+Cell _conc._interval+AS_Vol.+emission_CO2+consum_O2+generate_CO 2; 0.284, 0.633, pH_cumulo+PL+AN_cumulo+interval_yield+OD+R S+Feed_Vol+RS_cumulo+Accum._Yield+AS_Vol.+consum_O2+generat e_CO2; 0.284, 0.633, tmp+pH_cumulo+PL+AN_cumulo+interval_yi eld+OD+RS+RS_cumulo+AS_Vol.+emission_CO2+consum_O2+generate _CO2; 0.272, 0.633, pH_cumulo+PL+AN+AN_cumulo+interval_yiel d+OD+RS+RS_cumulo+Accum._Yield+AS_Vol.+emission_O2+consum_O 2+generate_CO2; 0.260, 0.633, pH_cumulo+PL+AN+interval_yiel d+OD+RS+Feed_Vol+RS_cumulo+Accum._Yield+AS_Vol.+emission_O2 +consum_O2+interval_O2+generate_CO2; 0.260, 0.632, pH_cumul o+PL+AN+interval_yield+interval_Pdt.+OD+RS+Feed_Vol+RS_cumu lo+Accum._Yield+AS_Vol.+consum_O2+interval_O2+generate_CO2; 0.272, 0.632, pH_cumulo+PL+AN+OD+RS+Feed_Vol+RS_cumulo+rab _integral+Accum._Yield+AS_Vol.+consum_O2+interval_O2+genera te_CO2; 0.272, 0.632, tmp+pH_cumulo+PL+AN+interval_yield+OD +RS+Feed_Vol+RS_cumulo+RQ_integral+Accum._Yield+AS_Vol.+con sum_O2; 0.260, 0.632, pH_cumulo+PL+AN+interval_yield+OD+RS+ RS_cumulo+rab_integral+Accum._Yield+AS _Vol.+emission_CO2+co nsum_O2+interval_O2+generate_CO2; 0.284, 0.632, pH+pH_cumul o+PL+interval_yield+OD+RS+RS_cumulo+Accum._Yield+AS_Vol.+em ission_CO2+consum_O2+generate_CO2; 0.284, 0.632, pH_cumulo+ PL+AN+interval_yield+interval_Pdt.+OD+RS+RS_cumulo+RQ_integ ral+Accum._Yield+AS_Vol.+consum_O2; 0.272, 0.632, pH_cumulo +PL+AN+interval_yield+OD+RS+RS_cumulo+rab_integral+Accum._Y ield+AS_Vol.+emission_O2+consum_O2+generate_CO2; 0.284, 0.6 32, pH+pH_cumulo+PL+interval_yield+OD+RS+Feed_Vol+RS_cumulo +AS_Vol.+emission_CO2+consum_O2+generate_CO2; 0.260, 0.632, pH_cumulo+PL+AN+AN_cumulo+interval_yield+OD+RS+Feed_Vol+RS _cumulo+rab_integral+AS_Vol.+emission_CO2+consum_O2+generat e_CO2; 0.306, 0.632, pH_cumulo+PL+interval_yield+OD+RS+Feed _Vol+RS_cumulo+emission_CO2+consum_O2+generate_CO2; 0.272, 0.632, pH_cumulo+PL+AN+AN_cumulo+OD+RS+RS_cumulo+Accum._Yie ld+AS_Vol.+emission_CO2+consum_O2+interval_O2+generate_CO2; 0.272, 0.632, pH_cumulo+PL+AN+AN_cumulo+interval_yield+OD+ RS+RS_cumulo+rab_integral+AS_Vol.+emission_CO2+consum_O2+ge nerate_CO2; 0.272, 0.632, pH+pH_cumulo+PL+AN+interval_yield +OD+RS+RS_cumulo+Cell_conc._interval+Accum._Yield+AS_Vol.+c onsum_O2+generate_CO2; 0.272, 0.632, pH+pH_cumulo+PL+AN+AN_ cumulo+OD+RS+Feed_Vol+RS_cumulo+Accum._Yield+AS_Vol.+consum _O2+generate_CO2; 0.247, 0.632, tmp+pH+pH_cumulo+PL+AN+inte rval_yield+OD+RS+Feed_Vol+RS_cumulo+Accum._Yield+AS_Vol.+em ission_CO2+consum_O2+generate_CO2; 0.259, 0.632, pH+pH_cumu lo+PL+AN+AN_cumulo+interval_yield+OD+RS+RS_cumulo+Accum._Yi eld+AS_Vol.+emission_CO2+consum_O2+generate_CO2; 0.259, 0.6 32, pH_cumulo+PL+AN+AN_cumulo+interval_yield+OD+RS+Feed_Vol +RS_cumulo+RQ_integral+Accum._Yield+AS_Vol.+emission_CO2+co nsum_O2; 0.271, 0.632, pH+pH_cumulo+PL+AN+interval_yield+OD +RS+RS_cumulo+rab_integral+Accum._Yield+AS_Vol.+consum_O2+g enerate_CO2; 0.259, 0.632, pH+pH_cumulo+PL+AN+interval_yiel d+OD+RS+Feed_Vol+RS_cumulo+Accum._Yield+AS_Vol.+emission_O2 +consum_O2+generate_CO2; 0.271, 0.632, tmp+pH_cumulo+PL+AN_ cumulo+interval_yield+OD+RS+RS_cumulo+Accum._Yield+AS_Vol.+ emission_CO2+consum_O2+generate_CO2; 0.259, 0.632, pH_cumul o+PL+PL_cumulo+AN+interval_yield+OD+RS+Feed_Vol+RS_cumulo+r ab_integral+Accum._Yield+AS_Vol.+consum_O2+generate_CO2; 0. 271, 0.632, tmp_cumulo+pH_cumulo+PL+AN_cumulo+interval_yiel d+OD+RS+RS_cumulo+Accum._Yield+AS_Vol.+emission_CO2+consum_ O2+generate_CO2; 0.283, 0.632, pH_cumulo+PL+interval_yield+ OD+RS+Feed_Vol+RS_cumulo+AS_Vol.+emission_CO2+consum_O2+int erval_O2+generate_CO2; 0.246, 0.632, pH_cumulo+PL+AN+AN_cum ulo+interval_yield+OD+RS+Feed_Vol+RS_cumulo+RQ_integral+Acc um._Yield+AS_Vol.+emission_O2+emission_CO2+consum_02; 0.283, 0.632, pH_cumulo+PL+AN+AN_cumulo+interval_yield+OD+RS+Feed _Vol+Accum._Yield+AS_Vol.+consum_O2+generate_CO2; 0.294, 0. 632, pH_cumulo+PL+AN+interval_yield+OD+RS+RS_cumulo+RQ_inte gral+AS_Vol.+emission_O2+consum_O2; 0.246, 0.632, pH_cumulo +PL+AN+AN_cumulo+interval_yield+OD+RS+Feed_Vol+RS_cumulo+Ce ll_conc._interval+rab_integral+Accum._Yield+AS_Vol.+consum_ O2+generate_CO2; 0.283, 0.632, pH_cumulo+PL+AN+AN_cumulo+OD +RS+RS_cumulo+Cell_conc._interval+Accum._Yield+AS_Vol.+cons um_O2+generate_CO2; 0.259, 0.632, tmp+pH_cumulo+PL+AN+inter val_yield+interval_Pdt.+OD+RS+Feed_Vol+RS_cumulo+Accum._Yie ld+AS_Vol.+consum_O2+generate_CO2; 0.271, 0.632, pH_cumulo+ PL+AN_cumulo+interval_yield+OD+RS+Feed_Vol+RS_cumulo+rab_in tegral+AS_Vol.+emission_CO2+consum_O2+generate_CO2; 0.271, 0.632, tmp+tmp_cumulo+pH_cumulo+PL+AN+interval_yield+OD+RS+ RS_cumulo+Accum._Yield+AS_Vol.+consum_O2+generate_CO2; 0.29 4, 0.632, pH_cumulo+PL+AN+interval_yield+OD+RS+RS_cumulo+Ce ll_conc._interval+AS_Vol.+consum_O2+generate_CO2; 0.283, 0. 632, pH_cumulo+PL+AN+OD+RS+RS_cumulo+Cell_conc._interval+Ac cum._Yield+AS_Vol.+consum_O2+interval_O2+generate_CO2; 0.27 1, 0.632, pH_cumulo+PL+PL_cumulo+AN+interval_yield+OD+RS+RS _cumulo+Accum._Yield+AS_Vol.+emission_CO2+consum_O2+generat e_CO2; 0.258, 0.631, tmp+pH_cumulo+PL+AN+interval_yield+OD+ RS+Feed_Vol+RS_cumulo+Cell_conc._interval+Accum._Yield+AS_V ol.+consum_O2+generate_CO2; 0.246, 0.631, pH+pH_cumulo+PL+P L_cumulo+AN+interval_yield+OD+RS+Feed_Vol+RS_cumulo+Accum._ Yield+AS_Vol.+emission_CO2+consum_O2+generate_CO2; 0.271, 0. 631, tmp+pH_cumulo+PL+AN+AN_cumulo+interval_yield+OD+RS+RS_ cumulo+Accum._Yield+AS_Vol.+consum_O2+generate_CO2; 0.271, 0.631, pH_cumulo+PL+AN+AN_cumulo+interval_yield+interval_Pd t.+OD+RS+Feed_Vol+Accum._Yield+AS_Vol.+consum_O2+generate_C 02; 0.305, 0.631, pH_cumulo+PL+AN_cumulo+OD+RS+RS_cumulo+Ac cum._Yield+AS_Vol.+consum_O2+generate_CO2; 0.282, 0.631, pH _cumulo+PL+PL_cumulo+AN+interval_yield+OD+RS+RS_cumulo+Accu m._Yield+AS_Vol.+consum_O2+generate_CO2; 0.305, 0.631, pH_c umulo+PL+interval_yield+OD+RS+RS_cumulo+Accum._Yield+AS_Vol. +consum_O2+generate_CO2; 0.246, 0.631, tmp_cumulo+pH_cumulo +PL+AN+interval_yield+OD+RS+Feed_Vol+RS_cumulo+rab_integral +Accum._Yield+AS_Vol.+emission_CO2+consum_O2+generate-CO2; 0.258, 0.631, pH+pH_cumulo+PL+AN+interval_yield+interval_Pd t.+OD+RS+Feed_Vol+RS_cumulo+Accum._Yield+AS_Vol.+consum_O2+ generate_CO2; 0.270, 0.631, tmp_cumulo+pH_cumulo+PL+AN+AN_c umulo+interval_yield+OD+RS+RS_cumulo+Accum._Yield+AS_Vol.+c onsum_O2+generate_CO2; 0.270, 0.631, pH_cumulo+PL+AN+interv al_yield+OD+RS+RS_cumulo+rab_integral+RQ_integral+Accum._Yi eld+AS_Vol.+emission_O2+consum_O2; 0.304, 0.631, pH_cumulo+ PL+interval_yield+OD+RS+RS_cumulo+rab_integral+emission_CO2 +consum_O2+generate_CO2; 0.282, 0.631, tmp_cumulo+pH_cumulo +PL+AN_cumulo+interval_yield+OD+RS+RS_cumulo+AS_Vol.+emissi on_CO2+consum_O2+generate_CO2; 0.293, 0.631, pH_cumulo+PL+A N+interval_yield+OD+RS+RS_cumulo+Accum._Yield+emission_CO2+ consum_O2+generate_CO2; 0.270, 0.631, pH+pH_cumulo+PL+AN+OD +RS+Feed_Vol+RS_cumulo+Accum._Yield+AS_Vol.+consum_O2+inter val_O2+generate_CO2; 0.258, 0.631, pH_cumulo+PL+AN+interval _yield+interval_Pdt.+OD+RS+Feed_Vol+RS_cumulo+Accum._Yield+ AS_Vol.+emission_O2+consum_O2+generate_CO2; 0.282, 0.631, p H_cumulo+PL+interval_Pdt.+OD+RS+RS_cumulo+RQ_integral+Accum. _Yield+AS_Vol.+emission_O2+emission_CO2+consum_O2; 0.270, 0. 631, pH_cumulo+PL+AN+AN_cumulo+OD+RS+Feed_Vol+RS_cumulo+Cel l_conc._interval+Accum._Yield+AS_Vol.+consum_O2+generate_CO 2; 0.257, 0.631, pH+pH_cumulo+PL+AN+interval_yield+OD+RS+Fe ed_Vol+RS_cumulo+rab_integral+RQ_integral+Accum._Yield+AS_V ol.+consum_O2; 0.257, 0.631, tmp+pH_cumulo+PL+AN+AN_cumulo+ interval_yield+OD+RS+RS_cumulo+Accum._Yield+AS_Vol.+emissio n_CO2+consum_O2+generate_CO2; 0.257, 0.631, pH_cumulo+PL+AN +AN_cumulo+OD+RS+Feed_Vol+RS_cumulo+rab_integral+Accum._Yie ld+AS_Vol.+emission_CO2+consum_O2+generate_CO2; 0.269, 0.63 1, pH+pH_cumulo+PL+AN+AN_cumulo+interval_yield+OD+RS+RS_cum ulo+Accum._Yield+AS_Vol.+consum_O2+generate_CO2; 0.257, 0.6 31, pH+pH_cumulo+PL+AN+AN_cumulo+interval_yield+OD+RS+Feed_ Vol+RS_cumulo+RQ_integral+Accum._Yield+AS_Vol.+consum_O2; 0. 281, 0.631, pH_cumulo+PL+AN_cumulo+OD+RS+RS_cumulo+Accum._Y ield+AS_Vol.+emission_CO2+consum_O2+interval_O2+generate_CO 2; 0.269, 0.631, pH_cumulo+PL+AN+OD+RS+Feed_Vol+RS_cumulo+C ell_conc._interval+Accum._Yield+AS_Vol.+emission_CO2+consum _O2+generate_CO2; 0.304, 0.631, pH_cumulo+PL+interval_yield +OD+RS+RS_cumulo+emission_CO2+consum_O2+interval_O2+generat e_CO2; 0.245, 0.631, tmp_cumulo+pH+pH_cumulo+PL+AN+interval _yield+OD+RS+Feed_Vol+RS_cumulo+Accum._Yield+AS_Vol.+emissi on_CO2+consum_O2+generate_CO2; 0.269, 0.631, pH_cumulo+PL+A N+interval_yield+interval_Pdt.+OD+RS+RS_cumulo+Accum._Yield +AS_Vol.+emission_CO2+consum_O2+generate_CO2; 0.232, 0.631, pH+pH_cumulo+PL+AN+AN_cumulo+interval_yield+OD+RS+Feed_Vol +RS_cumulo+rab_integral+Accum._Yield+AS_Vol.+emission_CO2+c onsum_O2+generate_CO2; 0.269, 0.631, pH_cumulo+PL+AN+interv al_yield+interval_Pdt.+OD+RS+Feed_Vol+rab_integral+Accum._Y ield+AS_Vol.+consum_O2+generate_CO2; 0.269, 0.631, tmp+pH_c umulo+PL+AN+interval_yield+OD+RS+RS_cumulo+Cell_conc._inter val+Accum._Yield+AS_Vol.+consum_O2+generate_CO2; 0.257, 0.6 31, tmp+tmp_cumulo+pH_cumulo+PL+AN+interval_yield+OD+RS+RS_ cumulo+Accum._Yield+AS_Vol.+emission_CO2+consum_O2+generate _CO2; 0.257, 0.631, pH_cumulo+PL+AN+interval_yield+OD+RS+Fe ed_Vol+RS_cumulo+Cell_conc._interval+Accum._Yield+AS_Vol.+e mission_O2+consum_O2+generate_CO2; 0.269, 0.631, pH+PL+AN+i nterval_yield+OD+RS+Feed_Vol+RS_cumulo+Cell_conc._interval+ Accum._Yield+AS_Vol.+consum_O2+generate_CO2; 0.281, 0.630, pH_cumulo+PL+AN_cumulo+interval_yield+OD+RS+RS_cumulo+AS_Vo l.+emission_CO2+consum_O2+interval_O2+generate_CO2; 0.244, 0.630, pH_cumulo+PL+AN+interval_yield+OD+RS+Feed_Vol+RS_cum ulo+Cell_conc._interval+Accum._Yield+AS_Vol.+emission_CO2+c onsum_O2+interval_O2+generate_CO2; 0.281, 0.630, pH_cumulo+ PL+interval_yield+OD+RS+RS_cumulo+Cell_conc._interval+Accum. _Yield+AS_Vol.+emission_CO2+consum_O2+generate_CO2; 0.269, 0.630, pH+PL+AN+interval_yield+interval_Pdt.+OD+RS+Feed_Vol +RS_cumulo+Accum._Yield+AS_Vol.+consum_O2+generate_CO2; 0.2 44, 0.630, pH+pH_cumulo+PL+AN+interval_yield+OD+RS+Feed_Vol +RS_cumulo+Cell_conc._interval+rab_integral+Accum._Yield+AS _Vol.+consum_O2+generate_CO2; 0.303, 0.630, pH_cumulo+PL+AN _cumulo+interval_yield+OD+RS+RS_cumulo+RQ_integral+AS_Vol.+ consum_O2; 0.269, 0.630, tmp+pH_cumulo+PL+AN+interval_yield +OD+RS+RS_cumulo+rab_integral+Accum._Yield+AS_Vol.+consum_O 2+generate_CO2; 0.244, 0.630, tmp+pH_cumulo+PL+AN+AN_cumulo +interval_yield+OD+RS+Feed_Vol+RS_cumulo+rab_integral+Accum. _Yield+AS_Vol.+consum_O2+generate_CO2; 0.269, 0.630, pH_cum ulo+PL+AN+interval_yield+OD+RS+Feed_Vol+RS_cumulo+RQ_integr al+AS_Vol.+emission_O2+emission_CO2+consum_O2; 0.269, 0.630, pH+PL+AN+AN_cumulo+interval_yield+OD+RS+Feed_Vol+RS_cumulo +Accum._Yield+AS_Vol.+consum_O2+generate_CO2; 0.256, 0.630, tmp_cumulo+pH_cumulo+PL+AN+interval_yield+OD+RS+Feed_Vol+R S_cumulo+Cell_conc._interval+Accum._Yield+AS_Vol.+consum_O2 +generate_CO2; 0.280, 0.630, pH_cumulo+PL+interval_yield+OD +RS+Feed_Vol+RS_cumulo+Cell_conc._interval+AS_Vol.+emission _CO2+consum_O2+generate_CO2; 0.244, 0.630, pH_cumulo+PL+AN+ AN_cumulo+interval_yield+OD+RS+Feed_Vol+RS_cumulo+rab_integ ral+Accum._Yield+AS_Vol.+emission_O2+consum_O2+generate_CO 2; 0.244, 0.630, pH_cumulo+PL+AN+AN_cumulo+interval_yield+O D+RS+Feed_Vol+RS_cumulo+rab_integral+Accum._Yield+AS_Vol.+c onsum_O2+interval_O2+generate_CO2; 0.280, 0.630, tmp+pH_cum ulo+PL+interval_yield+OD+RS+Feed_Vol+RS_cumulo+AS_Vol.+emis sion_CO2+consum_O2+generate_CO2; 0.280, 0.630, pH_cumulo+PL +AN+OD+RS+Feed_Vol+RS_cumulo+Accum._Yield+AS_Vol.+emission_ O2+consum_O2+generate_CO2; 0.268, 0.630, pH_cumulo+PL+AN+AN _cumulo+interval_yield+OD+RS+RS_cumulo+rab_integral+RQ_inte gral+Accum._Yield+AS_Vol.+consum_O2; 0.268, 0.630, pH_cumul o+PL+AN+interval_yield+OD+RS+RS_cumulo+Cell_conc._interval+ Accum._Yield+AS_Vol.+emission_O2+consum_O2+generate_CO2; 0. 280, 0.630, tmp+pH_cumulo+PL+AN+OD+RS+Feed_Vol+RS_cumulo+Ac cum._Yield+AS_Vol.+consum_O2+generate_CO2; 0.256, 0.630, pH +pH_cumulo+PL+AN+OD+RS+Feed_Vol+RS_cumulo+Accum._Yield+AS_V ol.+emission_CO2+consum_O2+interval_O2+generate_CO2; 0.280, 0.630, pH_cumulo+PL+AN+OD+RS+RS_cumulo+Cell_conc._interval +Accum._Yield+AS_Vol.+emission_CO2+consum_O2+generate_CO2; 0.268, 0.630, pH+pH_cumulo+PL+AN+interval_yield+OD+RS+RS_cu mulo+Accum._Yield+AS_Vol.+consum_O2+interval_O2+generate_CO 2; 0.243, 0.630, pH_cumulo+PL+AN+interval_yield+OD+RS+Feed_ Vol+RS_cumulo+rab_integral+RQ_integral+Accum._Yield+AS_Vol. +emission_O2+emission_CO2+consum_O2; 0.280, 0.630, pH+pH_cu mulo+PL+AN+OD+RS+RS_cumulo+Accum._Yield+AS_Vol.+emission_CO 2+consum_O2+generate_CO2; 0.268, 0.630, tmp_cumulo+pH_cumul o+PL+AN+interval_yield+OD+RS+Feed_Vol+RS_cumulo+RQ_integral +Accum._Yield+AS_Vol.+consum_O2; 0.256, 0.630, tmp_cumulo+p H_cumulo+PL+AN+AN_cumulo+interval_yield+OD+RS+RS_cumulo+Acc um._Yield+AS_Vol.+emission_CO2+consum_O2+generate_CO2; 0.25 6, 0.630, pH+pH_cumulo+PL+AN+interval_yield+OD+RS+Feed_Vol+ RS_cumulo+RQ_integral+Accum._Yield+AS_Vol.+emission_O2+cons um_O2; 0.243, 0.630, tmp+pH_cumulo+PL+AN+interval_yield+OD+ RS+Feed_Vol+RS_cumulo+Cell_conc._interval+Accum._Yield+AS_V ol.+emission_CO2+consum_O2+generate_CO2; 0.291, 0.630, pH+p H_cumulo+PL+AN+OD+RS+RS_cumulo+Accum._Yield+AS_Vol.+consum_ O2+generate_CO2; 0.256, 0.630, pH_cumulo+PL+AN_cumulo+inter val_yield+OD+RS+Feed_Vol+RS_cumulo+Cell_conc._interval+Accu m._Yield+AS_Vol.+emission_CO2+consum_O2+generate_CO2; 0.280, 0.630, pH_cumulo+PL+AN+interval_Pdt.+OD+RS+RS_cumulo+Accum. _Yield+AS_Vol.+emission_CO2+consum_O2+generate_CO2; 0.268, 0.630, tmp+tmp_cumulo+pH_cumulo+PL+AN+interval_yield+OD+RS+ RS_cumulo+AS_Vol.+emission_CO2+consum_O2+generate_CO2; 0.26 8, 0.630, pH_cumulo+PL+interval_yield+OD+RS+Feed_Vol+RS_cum ulo+Accum._Yield+AS_Vol.+emission_CO2+consum_O2+interval_O2 +generate_CO2; 0.280, 0.630, pH_cumulo+PL+interval_yield+OD +RS+Feed_Vol+RS_cumulo+RQ_integral+AS_Vol.+emission_O2+emis sion_CO2+consum_O2; 0.280, 0.630, pH_cumulo+PL+AN+OD+RS+RS_ cumulo+rab_integral+Accum._Yield+AS_Vol.+emission_CO2+consu m_O2+generate_CO2; 0.256, 0.630, pH+pH_cumulo+PL+AN+interva l_yield+OD+RS+RS_cumulo+rab_integral+Accum._Yield+AS_Vol.+e mission_CO2+consum_O2+generate_CO2; 0.268, 0.630, pH_cumulo +PL+interval_yield+OD+RS+Feed_Vol+RS_cumulo+rab_integral+Ac cum._Yield+AS_Vol.+emission_CO2+consum_O2+generate_CO2; 0.2 56, 0.630, tmp+pH_cumulo+PL+AN_cumulo+interval_yield+OD+RS+ Feed_Vol+RS_cumulo+Accum._Yield+AS_Vol.+emission_CO2+consum _O2+generate_CO2; 0.268, 0.630, tmp_cumulo+pH_cumulo+PL+AN+ interval_yield+OD+RS+RS_cumulo+Cell_conc._interval+Accum._Y ield+AS_Vol.+consum_O2+generate_CO2; 0.243, 0.630, pH_cumul o+PL+PL_cumulo+AN+interval_yield+OD+RS+Feed_Vol+RS_cumulo+r ab_integral+Accum._Yield+AS_Vol.+emission_CO2+consum_O2+gen erate_CO2; 0.280, 0.630, pH_cumulo+PL+AN+interval_Pdt.+OD+R S+Feed_Vol+RS_cumulo+RQ_integral+Accum._Yield+AS_Vol.+consu m_O2; 0.243, 0.630, pH_cumulo+PL+AN+AN_cumulo+interval_yiel d+OD+RS+Feed_Vol+RS_cumulo+rab_integral+RQ_integral+Accum._ Yield+AS_Vol.+emission_O2+consum_O2; 0.268, 0.630, tmp+pH_c umulo+PL+AN+OD+RS+Feed_Vol+RS_cumulo+Accum._Yield+AS_Vol.+e mission_CO2+consum_O2+generate_CO2; 0.268, 0.630, pH_cumulo +PL+AN+AN_cumulo+interval_yield+OD+RS+RS_cumulo+RQ_integral +Accum._Yield+AS_Vol.+emission_O2+consum_O2; 0.280, 0.630, pH_cumulo+PL+AN_cumulo+interval_yield+OD+RS+RS_cumulo+Cell_ conc._interval+Accum._Yield+AS_Vol.+consum_O2+generate_CO2; 0.268, 0.630, pH_cumulo+PL+AN+AN_cumulo+interval_yield+OD+ RS+Feed_Vol+Accum._Yield+AS_Vol.+emission_CO2+consum_O2+gen erate_CO2; 0.268, 0.630, tmp_cumulo+pH_cumulo+PL+AN+interva l_yield+OD+RS+RS_cumulo+Accum._Yield+AS_Vol.+consum_O2+inte rval_O2+generate_CO2; 0.255, 0.630, pH_cumulo+PL+AN+interva l_yield+OD+RS+RS_cumulo+Cell_conc._interval+rab_integral+Ac cum._Yield+AS_Vol.+emission_CO2+consum_O2+generate_CO2 0.2 79, 0.629, pH_cumulo+PL+AN+AN_cumulo+interval_yield+OD+RS+F eed_Vol+RS_cumulo+RQ_integral+AS_Vol.+consum_O2; 0.268, 0.6 29, pH_cumulo+PL+AN+interval_Pdt.+OD+RS+Feed_Vol+RS_cumulo+ Accum._Yield+AS_Vol.+emission_CO2+consum_O2+generate_CO2; 0. 302, 0.629, pH_cumulo+PL+interval_yield+OD+RS+RS_cumulo+rab _integral+RQ_integral+AS_Vol.+consum_O2; 0.255, 0.629, tmp+ pH_cumulo+PL+AN+AN_cumulo+interval_yield+OD+RS+Feed_Vol+RS_ cumulo+AS_Vol.+emission_CO2+consum_O2+generate_CO2; 0.279, 0.629, pH_cumulo+PL+interval_yield+interval_Pdt.+OD+RS+RS_c umulo+RQ_integral+AS_Vol.+emission_O2+emission_CO2+consum_O 2; 0.279, 0.629, pH_cumulo+PL+AN+interval_yield+OD+RS+RS_cu mulo+RQ_integral+Accum._Yield+AS_Vol.+emission_CO2+consum_O 2; 0.255, 0.629, tmp+pH_cumulo+PL+AN+interval_yield+OD+RS+F eed_Vol+RS_cumulo+Accum._Yield+AS_Vol.+consum_O2+interval_O 2+generate_CO2; 0.279, 0.629, pH_cumulo+PL+AN+AN_cumulo+int erval_Pdt.+OD+RS+RS_cumulo+RQ_integral+Accum._Yield+AS_Vol. +consum_O2; 0.255, 0.629, pH_cumulo+PL+AN+interval_yield+OD +RS+RS_cumulo+Cell_conc._interval+Accum._Yield+AS_Vol.+emis sion_CO2+consum_O2+interval_O2+generate_CO2; 0.267, 0.629, tmp+pH_cumulo+PL+AN+interval_yield+interval_Pdt.+OD+RS+Feed _Vol+Accum._Yield+AS_Vol.+consum_O2+generate_CO2; 0.267, 0. 629, pH+pH_cumulo+PL+AN_cumulo+interval_yield+OD+RS+Feed_Vo l+RS_cumulo+ASVol.+emission_CO2+consum_O2+generate_CO2; 0. 279, 0.629, pH_cumulo+PL+AN+interval_yield+interval_Pdt.+OD +RS+RS_cumulo+AS_Vol.+emission_CO2+consum-O2+generate_CO2; 0.279, 0.629, pH_cumulo+PL+AN_cumulo+interval_yield+OD+RS+R S_cumulo+Accum._Yield+AS_Vol.+emission_O2+consum_O2+generat e_CO2; 0.267, 0.629, tmp+pH_cumulo+PL+AN+AN_cumulo+interval _yield+OD+RS+RS_cumulo+AS_Vol.+emission_CO2+consum_O2+gener ate_CO2; 0.290, 0.629, pH_cumulo+PL+AN+OD+RS+RS_cumulo+Accu m._Yield+AS_Vol.+emission_O2+consum_O2+generate_CO2; 0.279, 0.629, pH_cumulo+PL+AN_cumulo+interval_yield+OD+RS+RS_cumu lo+rab_integral+Accum._Yield+AS_Vol.+consum_O2+generate_CO 2; 0.255, 0.629, pH_cumulo+PL+AN+interval_yield+interval_Pd t.+OD+RS+Feed_Vol+RS_cumulo+Cell_conc._interval+Accum._Yiel d+AS_Vol.+consum_O2+generate_CO2; 0.255, 0.629, tmp_cumulo+ pH_cumulo+PL+AN+interval_yield+OD+RS+Feed_Vol+RS_cumulo+Acc um._Yield+AS_Vol.+consum_O2+interval_O2+generate_CO2; 0.267, 0.629, pH_cumulo+PL+AN+AN_cumulo+interval_yield+OD+RS+RS_c umulo+Cell_conc._interval+AS_Vol.+emission_CO2+consum-O2+ge nerate_CO2; 0.279, 0.629, pH_cumulo+PL+AN+interval_yield+OD +RS+Feed_Vol+RS_cumulo+rab_integral+AS_Vol.+consum_O2+gener ate_CO2; 0.279, 0.629, pH+pH_cumulo+PL+AN+OD+RS+RS_cumulo+A ccum._Yield+AS_Vol.+consum_O2+interval_O2+generate_CO2; 0.2 79, 0.629, tmp_cumulo+pH_cumulo+PL+AN+OD+RS+Feed_Vol+RS_cum ulo+Accum._Yield+AS_Vol.+consum_O2+generate_CO2; 0.279, 0.6 29, tmp+pH_cumulo+PL+interval_yield+OD+RS+RS_cumulo+Accum._ Yield+AS_Vol.+emission_CO2+consum_O2+generate_CO2; 0.279, 0. 629, pH+pH_cumulo+PL+AN+interval_yield+OD+RS+RS_cumulo+RQ_i ntegral+Accum._Yield+AS_Vol.+consum_O2; 0.267, 0.629, tmp_c umulo+pH_cumulo+PL+AN+interval_yield+OD+RS+RS_cumulo+rab_in tegral+Accum._Yield+AS_Vol.+consum_O2+generate_CO2; 0.267, 0.629, pH_cumulo+PL+AN+interval_yield+OD+RS+RS_cumulo+Accum. _Yield+AS_Vol.+emission_O2+emission_CO2+consum_O2+generate_ CO2; 0.255, 0.629, tmp_cumulo+pH_cumulo+PL+AN+interval_yiel d+interval_Pdt.+OD+RS+Feed_Vol+RS_cumulo+Accum._Yield+AS_Vo l.+consum_O2+generate_CO2; 0.279, 0.629, pH_cumulo+PL+PL_cu mulo+AN+interval_yield+OD+RS+RS_cumulo+AS_Vol.+emission_CO2 +consum_O2+generate_CO2; 0.290, 0.629, pH_cumulo+PL+interva l_yield+OD+RS+RS_cumulo+rab_integral+RQ_integral+Accum._Yie ld+AS_Vol.+consum_O2; 0.278, 0.629, pH_cumulo+PL+AN+OD+RS+R S_cumulo+Accum._Yield+AS_Vol.+emission_O2+consum_O2+interva l_O2+generate_CO2; 0.290, 0.629, pH_cumulo+PL+AN+AN_cumulo+ interval_yield+OD+RS+RS_cumulo+AS_Vol.+consum_O2+generate_C 02; 0.254, 0.629, pH_cumulo+PL+AN+interval_yield+OD+RS+Feed _Vol+RS_cumulo+Cell_conc._interval+rab_integral+RQ_integral +Accum._Yield+AS_Vol.+consum_O2; 0.278, 0.629, pH_cumulo+PL +AN_cumulo+interval_yield+OD+RS+Feed_Vol+RS_cumulo+RQ_integ ral+Accum._Yield+AS_Vol.+consum_O2; 0.278, 0.629, pH_cumulo +PL+AN+interval_yield+OD+RS+Feed_Vol+rab_integral+Accum._Yi eld+AS_Vol.+consum_O2+generate_CO2; 0.266, 0.629, pH_cumulo +PL+AN+interval_yield+interval_Pdt.+OD+RS+Feed_Vol+RS_cumul O+AS_Vol.+emission_CO2+consum_O2+generate_CO2; 0.301, 0.629, pH+pH_cumulo+PL+interval_yield+OD+RS+RS_cumulo+emission_CO 2+consum_O2+generate_CO2; 0.254, 0.629, pH_cumulo+PL+AN+AN_ cumulo+interval_yield+interval_Pdt.+OD+RS+Feed_Vol+RS_cumul o+RQ_integral+Accum._Yield+AS_Vol.+consum_O2; 0.266, 0.629, pH+pH_cumulo+PL+AN+AN_cumulo+interval_yield+OD+RS+RS_cumul o+AS_Vol.+emission_CO2+consum_O2+generate_CO2; 0.278, 0.629, pH_cumulo+PL+AN+AN_cumulo+OD+RS+RS_cumulo+rab_integral+Acc um._Yield+AS_Vol.+consum_O2+generate_CO2; 0.266, 0.629, pH+ pH_cumulo+PL+AN+interval_yield+OD+RS+RS_cumulo+rab_integral +AS_Vol.+emission_CO2+consum_O2+generate_CO2; 0.301, 0.629, pH_cumulo+PL+AN+interval_yield+OD+RS+RS_cumulo+Accum._Yiel d+AS_Vol.+consum_O2; 0.254, 0.629, pH+pH_cumulo+PL+AN+inter val_yield+OD+RS+RS_cumulo+Accum._Yield+AS_Vol.+emission_CO2 +consum_O2+interval_O2+generate_CO2; 0.266, 0.629, pH_cumul o+PL+AN+interval_yield+OD+RS+RS_cumulo+rab_integral+AS_Vol. +emission_CO2+consum_O2+interval_O2+generate_CO2; 0.241, 0. 629, tmp+pH_cumulo+PL+AN+interval_yield+OD+RS+Feed_Vol+RS_c umulo+Accum._Yield+AS_Vol.+emission_CO2+consum_O2+interval_ O2+generate_CO2; 0.254, 0.629, pH+pH_cumulo+PL+AN+interval_ yield+OD+RS+RS_cumulo+Cell_conc._interval+Accum._Yield+AS_V ol.+emission_CO2+consum_O2+generate_CO2; 0.289, 0.628, pH_c umulo+PL+AN_cumulo+interval_yield+OD+RS+Feed_Vol+RS_cumulo+ emission_CO2+consum_O2+generate_CO2; 0.266, 0.628, pH_cumul o+PL+AN+OD+RS+Feed_Vol+RS_cumulo+Cell_conc._interval+Accum. _Yield+AS_Vol.+consum_O2+interval_O2+generate_CO2; 0.254, 0. 628, pH_cumulo+PL+AN_cumulo+interval_yield+OD+RS+Feed_Vol+R S_cumulo+Accum._Yield+AS_Vol.+emission_CO2+consum_O2+interv al_O2+generate_CO2; 0.254, 0.628, pH_cumulo+PL+AN+interval_ yield+OD+RS+Feed_Vol+RS_cumulo+Cell_conc._interval+RQ_integ ral+Accum._Yield+AS_Vol.+emission_O2+consum_O2; 0.254, 0.62 8, tmp+pH+pH_cumulo+PL+AN+interval_yield+OD+RS+Feed_Vol+RS_ cumulo+Accum._Yield+AS_Vol.+consum_O2+generate_CO2; 0.254, 0.628, pH+pH_cumulo+PL+AN+interval_yield+OD+RS+Feed_Vol+RS_ cumulo+rab_integral+AS_Vol.+emission_CO2+consum_O2+generate _CO2; 0.254, 0.628, pH_cumulo+PL+AN+AN_cumulo+interval_yiel d+OD+RS+Feed_Vol+RS_cumulo+Cell_conc._interval+RQ_integral+ Accum._Yield+AS_Vol.+consum_O2; 0.289, 0.628, pH_cumulo+PL+ interval_yield+OD+RS+RS_cumulo+Accum._Yield+AS_Vol.+consum_ O2+interval_O2+generate_CO2; 0.254, 0.628, tmp_cumulo+pH+pH _cumulo+PL+AN+interval_yield+OD+RS+Feed_Vol+RS_cumulo+Accum. _Yield+AS_Vol.+consum_O2+generate_CO2; 0.254, 0.628, pH+pH_ cumulo+PL+AN+AN_cumulo+interval_yield+OD+RS+Feed_Vol+RS_cum ulo+AS_Vol.+emission_CO2+consum_O2+generate_CO2; 0.266, 0.6 28, tmp+pH_cumulo+PL+AN+interval_yield+OD+RS+RS_cumulo+Accu m._Yield+AS_Vol.+consum_O2+interval_O2+generate_CO2; 0.278, 0.628, pH_cumulo+PL+AN+interval_yield+OD+RS+Feed_Vol+RS_cu mulo+RQ_integral+AS_Vol.+emission_CO2+consum_O2; 0.278, 0.6 28, pH_cumulo+PL+interval_yield+OD+RS+RS_cumulo+rab_integra l+AS_Vol. +emission_CO2+consum_O2+interval_O2+generate_CO2; 0.253, 0.628, tmp+tmp_cumulo+pH_cumulo+PL+AN+interval_yield +OD+RS+Feed_Vol+RS_cumulo+AS_Vol.+emission_C0O+consum-O2+ge nerate_CO2; 0.289, 0.628, pH_cumulo+PL+AN+interval_yield+OD +RS+RS_cumulo+RQ_integral+AS_Vol.+emission_CO2+consum-O2; 0. 289, 0.628, pH_cumulo+PL+AN_cumulo+interval_yield+OD+RS+RS_ cumulo+rab_integral+emission_CO2+consum_O2+generate_CO2; 0. 266, 0.628, pH+pH_cumulo+PL+AN+OD+RS+Feed_Vol+RS_cumulo+rab _integral+Accum._Yield+AS_Vol.+consum_O2+generate_CO2; 0.25 3, 0.628, tmp_cumulo+pH_cumulo+PL+AN_cumulo+interval_yield+ OD+RS+Feed_Vol+RS_cumulo+Accum._Yield+AS_Vol.+emission_CO2+ consum_O2+generate_CO2; 0.300, 0.628, tmp+pH_cumulo+PL+inte rval_yield+OD+RS+RS_cumulo+emission_CO2+consum_O2+generate-CO2; 0.289, 0.628, tmp_cumulo+pH_cumulo+PL+AN+OD+RS+RS_cumu lo+Accum._Yield+AS_Vol.+consum_O2+generate_CO2; 0.277, 0.62 8, pH_cumulo+PL+PL_cumulo+AN+interval_yield+OD+RS+RS_cumulo +RQ_integral+Accum._Yield+AS_Vol.+consum_O2; 0.253, 0.628, pH_cumulo+PL+AN+AN_cumulo+OD+RS+Feed_Vol+RS_cumulo+Cell_con c._interval+Accum._Yield+AS_Vol.+emission_CO2+consum_O2+gen erate_CO2; 0.253, 0.628, pH_cumulo+PL+AN+interval_yield+OD+ RS+Feed_Vol+RS_cumulo+rab_integral+RQ_integral+Accum._Yield +AS_Vol.+emission_CO2+consum_O2; 0.265, 0.628, pH_cumulo+PL +AN+interval_yield+OD+RS+RS_cumulo+Cell_conc._interval+rab_ integral+AS_Vol.+emission_CO2+consum_O2+generate_CO2; 0.265, 0.628, pH+pH_cumulo+PL+interval_yield+OD+RS+Feed_Vol+RS_cu mulo+Accum._Yield+AS_Vol.+emission_CO2+consum_O2+generate_C 02; 0.253, 0.628, pH_cumulo+PL+AN+AN_cumulo+interval_yield+ OD+RS+RS_cumulo+Cell_conc._interval+rab_integral+Accum._Yie ld+AS_Vol.+consum_O2+generate_CO2; 0.277, 0.628, pH_cumulo+ PL+AN+interval_yield+OD+RS+RS_cumulo+RQ_integral+AS_Vol.+em ission_O2+emission_CO2+consum_O2; 0.253, 0.628, tmp_cumulo+ pH_cumulo+PL+AN+AN_cumulo+interval_yield+OD+RS+Feed_Vol+RS_ cumulo+AS_Vol.+emission_CO2+consum_O2+generate_CO2; 0.277, 0.628, pH_cumulo+PL+AN_cumulo+OD+RS+RS_cumulo+rab_integral+ Accum._Yield+AS_Vol.+emission_CO2+consum_O2+generate_CO2; 0. 265, 0.628, pH_cumulo+PL+AN+interval_yield+interval_Pdt.+OD +RS+Feed_Vol+Accum._Yield+AS_Vol.+emission_CO2+consum-O2+ge nerate_CO2; 0.240, 0.628, tmp_cumulo+pH_cumulo+PL+AN+AN_cum ulo+interval_yield+OD+RS+Feed_Vol+RS_cumulo+rab_integral+Ac cum._Yield+AS_Vol.+consum_O2+generate_CO2; 0.240, 0.628, pH _cumulo+PL+AN+AN_cumulo+interval_yield+interval_Pdt.+OD+RS+ Feed_Vol+RS_cumulo+rab_integral+Accum._Yield+AS_Vol.+consum _O2+generate_CO2; 0.240, 0.628, pH_cumulo+PL+PL_cumulo+AN+A N_cumulo+interval_yield+OD+RS+Feed_Vol+RS_cumulo+Accum._Yie ld+AS_Vol.+emission_CO2+consum_O2+generate_CO2; 0.277, 0.62 8, pH_cumulo+PL+interval_yield+OD+RS+RS_cumulo+RQ_integral+ Accum._Yield+AS_Vol.+emission_O2+emission_CO2+consum_O2; 0. 240, 0.628, pH_cumulo+PL+AN+interval_yield+OD+RS+Feed_Vol+R S_cumulo+rab_integral+Accum._Yield+AS_Vol.+emission_O2+emis sion_CO2+consum_O2+generate_CO2; 0.277, 0.628, tmp_cumulo+p H_cumulo+PL+interval_yield+OD+RS+Feed_Vol+RS_cumulo+AS_Vol. +emission_CO2+consum_O2+generate_CO2; 0.265, 0.628, tmp_cum ulo+pH_cumulo+PL+AN+OD+RS+Feed_Vol+RS_cumulo+Accum._Yield+A S_Vol.+emission_CO2+consum_O2+generate_CO2; 0.277, 0.628, p H_cumulo+PL+AN+interval_yield+OD+RS+Feed_Vol+RS_cumulo+rab_ integral+RQ_integral+AS_Vol.+consum_O2; 0.253, 0.628, tmp+p H_cumulo+PL+AN+interval_yield+OD+RS+Feed_Vol+RS_cumulo+rab_ integral+AS_Vol.+emission_CO2+consum_O2+generate_CO2; 0.253, 0.628, pH_cumulo+PL+PL_cumulo+AN+interval_yield+OD+RS+Feed _Vol+RS_cumulo+Accum._Yield+AS_Vol.+consum_O2+interval_O2+g enerate_CO2; 0.277, 0.628, pH_cumulo+PL+AN_cumulo+interval_ yield+OD+RS+RS_cumulo+rab_integral+RQ_integral+Accum._Yield +AS_Vol.+consum_O2; 0.240, 0.628, pH_cumulo+PL+AN+interval_ yield+interval_Pdt.+OD+RS+Feed_Vol+RS_cumulo+rab_integral+A ccum._Yield+AS Vol.+emission_CO2+consum_O2+generate_CO2; 0. 277, 0.628, pH_cumulo+PL+AN+interval_Pdt.+OD+RS+RS_cumulo+R Q_integral+Accum._Yield+AS_Vol.+emission_CO2+consum_O2; 0.2 77, 0.628, pH+PL+AN+interval_yield+OD+RS+RS_cumulo+Cell_con c._interval+Accum._Yield+AS_Vol.+consum_O2+generate_CO2; 0. 240, 0.628, pH+pH_cumulo+PL+AN+interval_yield+OD+RS+Feed_Vo l+RS_cumulo+rab_integral+Accum._Yield+AS_Vol.+consum_O2+int erval_O2+generate_CO2; 0.227, 0.628, tmp+tmp_cumulo+pH_cumu lo+PL+AN+AN_cumulo+interval_yield+OD+RS+Feed_Vol+RS_cumulo+ Accum._Yield+AS_Vol.+emission_CO2+consum_O2+generate_CO2; 0. 252, 0.628, pH_cumulo+PL+PL_cumulo+AN+AN_cumulo+interval_yi eld+OD+RS+Feed_Vol+RS_cumulo+RQ_integral+Accum._Yield+AS_Vo l.+consum_O2; 0.252, 0.628, tmp+pH_cumulo+PL+AN+interval_yi eld+OD+RS+Feed_Vol+RS_cumulo+Accum._Yield+AS_Vol.+emission_ O2+consum_O2+generate_CO2; 0.288, 0.628, tmp+pH_cumulo+PL+A N+OD+RS+RS_cumulo+Accum._Yield+AS_Vol.+consum_O2+generate_C 02; 0.265, 0.628, tmp+pH_cumulo+PL+AN_cumulo+interval_yield +OD+RS+Feed_Vol+RS_cumulo+AS_Vol.+emission_CO2+consum-O2+ge nerate_CO2; 0.265, 0.628, pH+pH_cumulo+PL+AN+interval_yield +OD+RS+Feed_Vol+RS_cumulo+rab_integral+Accum._Yield+AS_Vol. +consum_O2; 0.252, 0.627, tmp+pH_cumulo+PL+AN+AN_cumulo+OD+ RS+Feed_Vol+RS_cumulo+Accum._Yield+AS_Vol.+emission_CO2+con sum_O2+generate_CO2; 0.299, 0.627, pH_cumulo+PL+AN+interval _yield+OD+RS+Feed_Vol+RS_cumulo+AS_Vol.+consum_O2; 0.264, 0. 627, pH+PL+AN+interval_yield+OD+RS+Feed_Vol+RS_cumulo+Accum. _Yield+AS_Vol.+emission_CO2+consum_O2+generate_CO2; 0.264, 0.627, pH_cumulo+PL+AN+interval_yield+interval_Pdt.+OD+RS+R S_cumulo+rab_integral+Accum._Yield+AS_Vol.+consum_O2+genera te_CO2; 0.240, 0.627, tmp+tmp_cumulo+pH_cumulo+PL+AN+interv al_yield+OD+RS+Feed_Vol+RS_cumulo+rab_integral+Accum._Yield +AS_Vol.+consum_O2+generate_CO2; 0.264, 0.627, pH_cumulo+PL +AN+AN_cumulo+interval_yield+OD+RS+RS_cumulo+AS_Vol.+emissi on_CO2+consum_O2+interval_O2+generate_CO2; 0.252, 0.627, pH _cumulo+PL+AN+AN_cumulo+interval_yield+OD+RS+Feed_Vol+RS_cu mulo+rab_integral+Accum._Yield+AS_Vol.+emission_CO2+consum-02; 0.276, 0.627, pH_cumulo+PL+AN+interval_yield+OD+RS+RS_c umulo+Cell_conc._interval+RQ_integral+Accum._Yield+AS-Vol.+ consum_O2; 0.264, 0.627, tmp_cumulo+pH_cumulo+PL+AN+AN_cumu lo+interval_yield+OD+RS+RS_cumulo+AS_Vol.+emission_CO2+cons um_O2+generate_CO2; 0.239, 0.627, tmp_cumulo+pH_cumulo+PL+A N+interval_yield+OD+RS+Feed_Vol+RS_cumulo+Cell_conc._interv al+Accum._Yield+AS_Vol.+emission_CO2+consum_O2+generate_CO 2; 0.276, 0.627, pH_cumulo+PL+AN+interval_yield+OD+RS+RS_cu mulo+AS_Vol.+emission_O2+emission_CO2+consum_O2+generate_CO 2; 0.264, 0.627, tmp+pH_cumulo+PL+AN+interval_yield+OD+RS+R S_cumulo+rab_integral+AS_Vol.+emission_CO2+consum_O2+genera te_CO2; 0.264, 0.627, pH_cumulo+PL+PL_cumulo+AN+interval_yi eld+OD+RS+Feed_Vol+RS_cumulo+AS_Vol.+emission_CO2+consum_O2 +generate_CO2; 0.276, 0.627, pH_cumulo+PL+AN+interval_yield +OD+RS+Feed_Vol+RS_cumulo+AS_Vol.+emission_O2+consum_O2+gen erate_CO2; 0.276, 0.627, pH+pH_cumulo+PL+AN+interval_yield+ OD+RS+Feed_Vol+RS_cumulo+Accum._Yield+AS_Vol.+consum_O2; 0. 288, 0.627, pH_cumulo+PL+AN+interval_yield+OD+RS+Feed_Vol+R S_cumulo+emission_CO2+consum_O2+generate_CO2; 0.288, 0.627, tmp+pH_cumulo+PL+AN+interval_yield+OD+RS+RS_cumulo+AS_Vol. +consum_O2+generate_CO2; 0.264, 0.627, pH_cumulo+PL+AN+AN_c umulo+OD+RS+Feed_Vol+RS_cumulo+Accum._Yield+AS_Vol.+emissio n_O2+consum_O2+generate_CO2; 0.276, 0.627, pH+pH_cumulo+PL+ interval_yield+OD+RS+RS_cumulo+rab_integral+AS_Vol.+emissio n_CO2+consum_O2+generate_CO2; 0.239, 0.627, pH+pH_cumulo+PL +AN+AN_cumulo+interval_yield+OD+RS+Feed_Vol+RS_cumulo+Cell_ conc._interval+Accum._Yield+AS_Vol.+consum_O2+generate_CO2; 0.276, 0.627, tmp_cumulo+pH_cumulo+PL+interval_yield+OD+RS +RS_cumulo+Accum._Yield+AS_Vol.+emission_CO2+consum_O2+gene rate_CO2; 0.309, 0.627, pH_cumulo+PL+AN+interval_yield+OD+R S+RS_cumulo+AS_Vol.+consum_O2; 0.309, 0.627, pH_cumulo+PL+i nterval_yield+OD+RS+RS_cumulo+AS_Vol.+consum_O2+generate_CO 2; 0.252, 0.627, pH_cumulo+PL+AN+OD+RS+Feed_Vol+RS_cumulo+r ab_integral+Accum._Yield+AS_Vol.+emission_CO2+consum_O2+int erval_O2+generate_CO2; 0.276, 0.627, tmp+pH_cumulo+PL+AN+in terval_yield+OD+RS+Feed_Vol+RS_cumulo+Accum._Yield+AS_Vol.+ consum-O2; 0.276, 0.627, pH_cumulo+PL+AN+AN_cumulo+interval _yield+OD+RS+RS_cumulo+rab_integral+RQ_integral+AS_Vol.+con sum_O2; 0.264, 0.627, pH_cumulo+PL+interval_yield+OD+RS+Fee d_Vol+RS_cumulo+RQ_integral+Accum._Yield+AS_Vol.+emission_O 2+emission_CO2+consum_O2; 0.298, 0.627, pH_cumulo+PL+interv al_yield+OD+RS+RS_cumulo+RQ_integral+AS_Vol.+emission_CO2+c onsum_O2; 0.226, 0.627, tmp+pH_cumulo+PL+AN+AN_cumulo+inter val_yield+OD+RS+Feed_Vol+RS_cumulo+rab_integral+Accum._Yiel d+AS_Vol.+emission_CO2+consum_O2+generate_CO2; 0.252, 0.627, pH+pH_cumulo+PL+PL_cumulo+AN+interval_yield+OD+RS+Feed_Vol +RS_cumulo+Accum._Yield+AS_Vol.+consum_O2+generate_CO2; 0.2 64, 0.627, pH_cumulo+PL+AN+interval_yield+interval_Pdt.+OD+ RS+Feed_Vol+Accum._Yield+AS_Vol.+consum_O2+interval_O2+gene rate_CO2; 0.276, 0.627, pH+PL+AN+interval_yield+interval_Pd t.+OD+RS+Feed_Vol+Accum._Yield+AS_Vol.+consum_O2+generate_C O2; 0.264, 0.627, pH_cumulo+PL+AN_cumulo+interval_yield+OD+ RS+Feed_Vol+RS_cumulo+Cell_conc._interval+AS_Vol.+emission_ CO2+consum_O2+generate_CO2; 0.264, 0.627, pH_cumulo+PL+AN+O D+RS+RS_cumulo+rab_integral+Accum._Yield+AS_Vol.+emission_C O2+consum_O2+interval_O2+generate_CO2; 0.239, 0.627, tmp_cu mulo+pH_cumulo+PL+AN+interval_yield+OD+RS+Feed_Vol+RS_cumul o+Accum._Yield+AS_Vol.+emission_CO2+consum_O2+interval_O2+g enerate_CO2; 0.239, 0.627, pH_cumulo+PL+AN+AN_cumulo+interv al_yield+OD+RS+Feed_Vol+RS_cumulo+Accum._Yield+AS_Vol.+emis sion_O2+emission_CO2+consum_O2+generate_CO2; 0.264, 0.627, pH+pH_cumulo+PL+AN+OD+RS+RS_cumulo+Accum._Yield+AS_Vol.+emi ssion_CO2+consum_O2+interval_O2+generate_CO2; 0.239, 0.627, tmp+tmp_cumulo+pH_cumulo+PL+AN+AN_cumulo+interval_yield+OD +RS+Feed_Vol+RS_cumulo+Accum._Yield+AS_Vol.+consum_O2+gener ate_CO2; 0.264, 0.627, pH_cumulo+PL+AN+AN_cumulo+interval_y ield+OD+RS+Feed_Vol+RS_cumulo+Accum._Yield+AS_Vol.+emission _CO2+consum_O2; 0.275, 0.627, tmp_cumulo+pH_cumulo+PL+AN+OD +RS+RS_cumulo+Accum._Yield+AS_Vol.+consum_O2+interval_O2+ge nerate_CO2; 0.251, 0.627, pH_cumulo+PL+AN+interval_yield+in terval_Pdt.+OD+RS+Feed_Vol+RS_cumulo+RQ_integral+Accum._Yie ld+AS_Vol.+emission_O2+consum_O2; 0.264, 0.627, pH_cumulo+P L+AN+AN_cumulo+OD+RS+RS_cumulo+rab_integral+Accum._Yield+AS Vol.+emission_CO2+consum_O2+generate_CO2; 0.251, 0.627, tm p+pH_cumulo+PL+AN+interval_yield+OD+RS+RS_cumulo+rab_integr al+Accum._Yield+AS_Vol.+emission_CO2+consum_O2+generate_CO 2; 0.263, 0.627, tmp+pH_cumulo+PL+AN+AN_cumulo+OD+RS+Feed_V ol+RS_cumulo+Accum._Yield+AS_Vol.+consum_O2+generate_CO2; 0. 263, 0.627, pH+pH_cumulo+PL+AN+interval_yield+OD+RS+RS_cumu lo+Accum._Yield+AS_Vol.+emission_O2+consum_O2+generate_CO2; 0.263, 0.627, tmp_cumulo+pH_cumulo+PL+AN+AN_cumulo+OD+RS+F eed_Vol+RS_cumulo+Accum._Yield+AS_Vol.+consum_O2+generate_C 02; 0.263, 0.627, pH_cumulo+PL+PL_cumulo+AN+interval_yield+ OD+RS+RS_cumulo+Accum._Yield+AS_Vol.+consum_O2+interval_O2+ generate_CO2; 0.251, 0.627, pH+PL+AN+AN_cumulo+interval_yie ld+OD+RS+Feed_Vol+RS_cumulo+Cell_conc._interval+Accum._Yiel d+AS_Vol.+consum_O2+generate_CO2; 0.263, 0.627, pH_cumulo+P L+AN+OD+RS+Feed_Vol+RS_cumulo+Accum._Yield+AS_Vol.+emission _O2+consum_O2+interval_O2+generate_CO2; 0.251, 0.627, tmp_c umulo+pH_cumulo+PL+AN+AN_cumulo+OD+RS+Feed_Vol+RS_cumulo+Ac cum._Yield+AS_Vol.+emission_C02+consum_O2+generate_CO2 0.2 63, 0.627, pH_cumulo+PL+AN+OD+RS+Feed_Vol+RS_cumulo+Cell_co nc._interval+rab_integral+Accum._Yield+AS_Vol.+consum_O2+ge nerate_CO2; 0.287, 0.627, pH_cumulo+PL+OD+RS+RS_cumulo+Accu m._Yield+AS_Vol.+emission_CO2+consum_O2+interval_O2+generat e_CO2; 0.263, 0.627, pH+pH_cumulo+PL+AN+AN_cumulo+OD+RS+RS_ cumulo+Accum._Yield+AS_Vol.+emission_CO2+consum_O2+generate _CO2; 0.263, 0.627, pH_cumulo+PL+AN+AN_cumulo+OD+RS+RS_cumu lo+Cell_conc._interval+Accum._Yield+AS_Vol.+emission_CO2+co nsum_O2+generate_CO2; 0.275, 0.627, pH_cumulo+PL+AN+interva l_yield+OD+RS+Feed_Vol+RS_cumulo+RQ_integral+AS_Vol.+emissi on_O2+consum_O2; 0.238, 0.626, pH+pH_cumulo+PL+AN+interval_ yield+OD+RS+Feed_Vol+RS_cumulo+rab_integral+Accum._Yield+AS _Vol.+emission_O2+consum_O2+generate_CO2; 0.275, 0.626, pH_ cumulo+PL+AN_cumulo+interval_Pdt.+OD+RS+RS_cumulo+Accum._Yi eld+AS_Vol.+emission_CO2+consum_O2+generate_CO2; 0.286, 0.6 26, tmp_cumulo+pH_cumulo+PL+AN+interval_yield+OD+RS+RS_cumu lo+AS_Vol.+consum_O2+generate_CO2; 0.238, 0.626, pH_cumulo+ PL+AN+interval_yield+OD+RS+Feed_Vol+RS_cumulo+Cell_conc._in terval+rab_integral+Accum._Yield+AS_Vol.+consum_O2+interval _O2+generate_CO2; 0.225, 0.626, pH_cumulo+PL+AN+AN_cumulo+i nterval_yield+OD+RS+Feed_Vol+RS_cumulo+Cell_conc._interval+ rab_integral+Accum._Yield+AS_Vol.+emission_CO2+consum_O2+ge nerate_CO2; 0.251, 0.626, pH_cumulo+PL+AN+AN_cumulo+interva l_yield+OD+RS+Feed_Vol+RS_cumulo+Cell_conc._interval+AS_Vol. +emission_CO2+consum_O2+generate_CO2; 0.238, 0.626, pH_cumu lo+PL+AN+AN_cumulo+interval_yield+interval_Pdt.+OD+RS+Feed_ Vol+RS_cumulo+Accum._Yield+AS_Vol.+emission_CO2+consum_O2+g enerate_CO2; 0.238, 0.626, pH_cumulo+PL+AN+interval_yield+i nterval_Pdt.+OD+RS+Feed_Vol+RS_cumulo+RQ_integral+Accum._Yi eld+AS_Vol.+emission_O2+emission_CO2+consum_O2; 0.263, 0.62 6, pH_cumulo+PL+AN+interval_yield+OD+RS+RS_cumulo+RQ_integr al+Accum._Yield+AS _Vol. +emission_O2+emission_CO2+consum_O2; 0.275, 0.626, pH+pH_cumulo+PL+AN_cumulo+OD+RS+RS_cumulo+Ac cum._Yield+AS_Vol.+emission_CO2+consum_O2+generate_CO2; 0.2 86, 0.626, pH_cumulo+PL+AN+interval_yield+OD+RS+RS_cumulo+r ab_integral+emission_CO2+consum_O2+generate_CO2; 0.225, 0.6 26, pH+pH_cumulo+PL+AN+AN_cumulo+interval_yield+OD+RS+Feed_ Vol+RS_cumulo+Cell_conc._interval+Accum._Yield+AS_Vol.+emis sion_CO2+consum_O2+generate_CO2; 0.250, 0.626, pH_cumulo+PL +AN+interval_yield+OD+RS+Feed_Vol+RS_cumulo+Cell_conc._inte rval+rab_integral+AS_Vol.+emission_CO2+consum_O2+generate_C 02; 0.297, 0.626, tmp_cumulo+pH_cumulo+PL+interval_yield+OD +RS+RS_cumulo+emission_CO2+consum_O2+generate_CO2; 0.286, 0. 626, pH+pH_cumulo+PL+AN+interval_yield+OD+RS+RS_cumulo+RQ_i ntegral+AS_Vol.+consum_O2; 0.286, 0.626, pH_cumulo+PL+inter val_yield+OD+RS+Feed_Vol+RS_cumulo+RQ_integral+Accum._Yield +AS_Vol.+consum_O2; 0.274, 0.626, pH_cumulo+PL+AN+interval_ yield+OD+RS+RS_cumulo+rab_integral+AS_Vol.+emission_O2+cons um_O2+generate_CO2; 0.237, 0.626, pH+pH_cumulo+PL+AN+interv al_yield+interval_Pdt.+OD+RS+Feed_Vol+RS_cumulo+Accum._Yiel d+AS_Vol.+emission_CO2+consum_O2+generate_CO2; 0.262, 0.626, pH_cumulo+PL+interval_yield+interval_Pdt.+OD+RS+RS_cumulo+ RQ_integral+Accum._Yield+AS_Vol.+emission_O2+emission-CO2+c onsum_O2; 0.274, 0.626, pH_cumulo+PL+AN_cumulo+interval_yie ld+OD+RS+RS_cumulo+Accum._Yield+AS_Vol.+consum_O2+interval_ O2+generate_CO2; 0.286, 0.626, pH_cumulo+PL+interval_Pdt.+O D+RS+RS_cumulo+RQ_integral+AS_Vol.+emission_O2+emission_CO2 +consum_O2; 0.286, 0.626, pH_cumulo+PL+interval_yield+OD+RS +RS_cumulo+Accum_Yield+emission_CO2+consum_O2+interval_O2+ generate_CO2; 0.274, 0.626, tmp+tmp_cumulo+pH_cumulo+PL+int erval_yield+OD+RS+RS_cumulo+AS_Vol.+emission_CO2+consum_O2+ generate_CO2; 0.262, 0.626, pH_cumulo+PL+AN+interval_yield+ interval_Pdt.+OD+RS+RS_cumulo+Accum._Yield+AS_Vol.+consum_O 2+interval_O2+generate_CO2; 0.286, 0.626, pH_cumulo+PL+AN+i nterval_yield+interval_Pdt.+OD+RS+RS_cumulo+AS_Vol.+consum_ O2+generate_CO2; 0.274, 0.626, pH_cumulo+PL+interval_yield+ OD+RS+RS_cumulo+rab_integral+RQ_integral+AS_Vol.+emission_O 2+emission_CO2+consum_O2; 0.250, 0.626, pH_cumulo+PL+PL_cum ulo+AN+AN_cumulo+interval_yield+OD+RS+Feed_Vol+RS_cumulo+Ac cum._Yield+AS_Vol.+consum_O2+generate_CO2; 0.297, 0.626, pH _cumulo+PL+interval_yield+OD+RS+RS_cumulo+Cell_conc._interv al+emission_CO2+consum_O2+generate_CO2; 0.250, 0.626, pH_cu mulo+PL+AN+AN_cumulo+interval_yield+OD+RS+RS_cumulo+rab_int egral+Accum._Yield+AS_Vol.+consum_O2+interval_O2+generate_C 02; 0.262, 0.626, pH_cumulo+PL+AN+AN_cumulo+interval_yield+ interval_Pdt.+OD+RS+RS_cumulo+RQ_integral+Accum._Yield+AS_V ol.+consum_O2; 0.250, 0.626, pH_cumulo+PL+AN+interval_yield +OD+RS+RS_cumulo+Cell_conc._interval+rab_integral+Accum._Yi eld+AS_Vol.+consum_O2+interval_O2+generate_CO2; 0.250, 0.62 6, tmp+pH_cumulo+PL+AN+AN_cumulo+interval_yield+OD+RS+Feed_ Vol+RS_cumulo+RQ_integral+Accum._Yield+AS_Vol.+consum_O2; 0. 250, 0.626, tmp_cumulo+pH_cumulo+PL+AN+interval_yield+OD+RS +RS_cumulo+Accum._Yield+AS_Vol.+emission_CO2+consum_O2+inte rval_O2+generate_CO2; 0.274, 0.626, pH_cumulo+PL+AN_cumulo+ interval_yield+OD+RS+RS_cumulo+rab_integral+Accum._Yield+em ission_CO2+consum_O2+generate_CO2; 0.262, 0.626, pH_cumulo+ PL+interval_yield+OD+RS+Feed_Vol+RS_cumulo+Cell_conc._inter val+Accum._Yield+AS_Vol.+emission_CO2+consuni_O2+generate_CO 2; 0.274, 0.626, tmp_cumulo+pH_cumulo+PL+AN_cumulo+OD+RS+RS _cumulo+Accum._Yield+AS_Vol.+emission_CO2+consum_O2+generat e_CO2; 0.262, 0.626, pH_cumulo+PL+AN+interval_Pdt.+OD+RS+RS _cumulo+RQ_integral+Accum._Yield+AS_Vol.+emission_O2+emissi on_CO2+consum_O2; 0.250, 0.626, tmp+tmp_cumulo+pH_cumulo+PL +AN_cumulo+interval_yield+OD+RS+RS_cumulo+Accum._Yield+AS_V ol.+emission_CO2+consum_O2+generate_CO2; 0.274, 0.626, pH_c umulo+PL+AN+OD+RS+RS_cumulo+Cell_conc._interval+rab_integra l+Accum._Yield+AS_Vol.+consum_O2+generate_CO2; 0.262, 0.626, pH+pH_cumulo+PL+AN+OD+RS+Feed_Vol+RS_cumulo+Cell_conc._int erval+Accum._Yield+AS_Vol.+consum_O2+generate_CO2; 0.262, 0. 626, pH_cumulo+PL+AN+interval_yield+OD+RS+Feed_Vol+RS_cumul o+AS_Vol.+emission_O2+emission_CO2+consum_O2+generate-CO2; 0.237, 0.626, pH+pH_cumulo+PL+AN+interval_yield+interval_Pd t.+OD+RS+Feed_Vol+RS_cumulo+rab_integral+Accum._Yield+AS_Vo l.+consum_O2+generate_CO2; 0.224, 0.626, pH_cumulo+PL+AN+AN _cumulo+interval_yield+OD+RS+Feed_Vol+RS_cumulo+rab_integra l+Accum._Yield+AS_Vol.+emission_CO2+consum_O2+interval_O2+g enerate_CO2; 0.249, 0.626, pH_cumulo+PL+AN+interval_yield+i nterval_Pdt.+OD+RS+Feed_Vol+RS_cumulo+RQ_integral+Accum._Yi eld+AS_Vol.+emission_CO2+consum_O2; 0.274, 0.626, tmp+pH_cu mulo+PL+AN+OD+RS+RS_cumulo+Accum._Yield+AS_Vol.+emission_CO 2+consum_O2+generate_CO2; 0.249, 0.626, tmp+pH_cumulo+PL+AN +interval_yield+OD+RS+RS_cumulo+Accum._Yield+AS_Vol.+emissi on_CO2+consum_O2+interval_O2+generate_CO2; 0.285, 0.626, pH _cumulo+PL+AN+AN_cumulo+interval_yield+OD+RS+RS_cumulo+emis sion_CO2+consum_O2+generate_CO2; 0.274, 0.626, pH+pH_cumulo +PL+AN+AN_cumulo+OD+RS+RS_cumulo+Accum._Yield+AS_Vol.+consu m_O2+generate_CO2; 0.249, 0.626, pH_cumulo+PL+AN+AN_cumulo+ interval_Pdt.+OD+RS+Feed_Vol+RS_cumulo+Accum._Yield+AS_Vol. +emission_CO2+consum_O2+generate_CO2; 0.262, 0.626, pH_cumu lo+PL+AN+AN_cumulo+interval_Pdt.+OD+RS+Feed_Vol+RS_cumulo+R Q_integral+Accum._Yield+AS_Vol.+consum_O2; 0.249, 0.626, tm p+pH_cumulo+PL+AN+interval_yield+OD+RS+Feed_Vol+RS_cumulo+R Q_integral+Accum._Yield+AS_Vol.+emission_O2+consum_O2; 0.27 4, 0.626, tmp_cumulo+pH_cumulo+PL+AN+OD+RS+RS_cumulo+Accum. _Yield+AS_Vol.+emission_CO2+consum_O2+generate_CO2; 0.249, 0.626, tmp+pH_cumulo+PL+AN+interval_yield+OD+RS+Feed_Vol+RS _cumulo+rab_integral+RQ_integral+Accum._Yield+AS_Vol.+consu m_O2; 0.249, 0.626, tmp+pH_cumulo+PL+AN+interval_yield+OD+R S+RS_cumulo+Cell_conc._interval+Accum._Yield+AS_Vol.+emissi on_CO2+consum_O2+generate_CO2; 0.262, 0.626, pH+pH_cumulo+P L+AN+interval_yield+OD+RS+RS_cumulo+Cell_conc._interval+AS_ Vol.+emission_CO2+consum_O2+generate_CO2; 0.273, 0.626, tmp _cumulo+pH_cumulo+PL+AN+AN_cumulo+OD+RS+RS_cumulo+Accum._Yi eld+AS_Vol.+consum_O2+generate_CO2; 0.273, 0.625, pH_cumulo +PL+AN_cumulo+interval_yield+OD+RS+Feed_Vol+Accum._Yield+AS _Vol.+emission_CO2+consum_O2+generate_CO2; 0.273, 0.625, pH _cumulo+PL+AN+interval_yield+OD+RS+Feed_Vol+RS_cumulo+Cell_ conc._interval+Accum._Yield+AS_Vol.+consum_O2; 0.261, 0.625, pH_cumulo+PL+AN+AN_cumulo+interval_Pdt.+OD+RS+RS_cumulo+Ac cum._Yield+AS_Vol.+emission_C02+consum_O2+generate_CO2 0.2 73, 0.625, pH_cumulo+PL+AN+interval_yield+OD+RS+RS_cumulo+r ab_integral+RQ_integral+AS_Vol. +emission_O2+consum_O2; 0.27 3, 0.625, pH_cumulo+PL+AN+AN_cumulo+OD+RS+RS_cumulo+Accum._ Yield+AS_Vol.+emission_O2+consum_O2+generate_CO2; 0.249, 0. 625, pH_cumulo+PL+AN+interval_yield+interval_Pdt.+OD+RS+Fee d_Vol+RS_cumulo+rab_integral+RQ_integral+Accum._Yield+AS_Vo l.+consum_O2; 0.273, 0.625, pH_cumulo+PL+AN_cumulo+interval _yield+OD+RS+RS_cumulo+RQ_integral+Accum._Yield+AS_Vol.+emi ssion_O2+consum_O2; 0.285, 0.625, pH_cumulo+PL+AN+interval_ yield+OD+RS+RS_cumulo+Cell_conc._interval+RQ_integral+AS_Vo l.+consum_O2; 0.285, 0.625, pH_cumulo+PL+AN_cumulo+OD+RS+RS _cumulo+Accum._Yield+AS_Vol.+consum_O2+interval_O2+generate _CO2; 0.261, 0.625, tmp_cumulo+pH_cumulo+PL+AN_cumulo+inter val_yield+OD+RS+Feed_Vol+RS_cumulo+AS_Vol.+emission_CO2+con sum_O2+generate_CO2; 0.285, 0.625, pH_cumulo+AN+interval_yi eld+OD+RS+Feed_Vol+RS_cumulo+RQ_integral+Accum._Yield+AS_Vo l.+consum_O2; 0.236, 0.625, pH_cumulo+PL+AN+interval_yield+ interval_Pdt.+OD+RS+Feed_Vol+RS_cumulo+rab_integral+Accum._ Yield+AS_Vol.+consum_O2+interval_O2+generate_CO2; 0.296, 0. 625, pH_cumulo+PL+interval_yield+OD+RS+RS_cumulo+RQ_integra l+AS_Vol.+emission_O2+consum_O2; 0.284, 0.625, pH_cumulo+PL +interval_yield+OD+RS+RS_cumulo+RQ_integral+Accum._Yield+AS _Vol.+emission_O2+consum_O2; 0.236, 0.625, pH_cumulo+PL+AN+ interval_yield+OD+RS+Feed_Vol+RS_cumulo+rab_integral+Accum. _Yield+AS_Vol.+emission_O2+consum_O2+interval_O2+generate_C 02; 0.236, 0.625, pH_cumulo+PL+PL_cumulo+AN+interval_yield+ OD+RS+Feed_Vol+RS_cumulo+Accum._Yield+AS_Vol.+emission_CO2+ consum_O2+interval_O2+generate_CO2; 0.261, 0.625, pH_cumulo +PL+interval_yield+OD+RS+RS_cumulo+rab_integral+Accum._Yiel d+AS_Vol.+emission_CO2+consum_O2+interval_O2+generate_CO2; 0.261, 0.625, pH_cumulo+PL+AN+OD+RS+RS_cumulo+Cell_conc._in terval+Accum._Yield+AS_Vol.+emission_CO2+consum_O2+interval _O2+generate_CO2; 0.261, 0.625, tmp+pH_cumulo+PL+AN+interva l_yield+OD+RS+RS_cumulo+Cell_conc._interval+AS_Vol.+emissio n_CO2+consum_O2+generate_CO2; 0.273, 0.625, tmp_cumulo+pH_c umulo+PL+AN_cumulo+interval_yield+OD+RS+RS_cumulo+Accum._Yi eld+AS_Vol.+consum_O2+generate_CO2; 0.284, 0.625, pH_cumulo +PL+AN+interval_yield+OD+RS+RS_cumulo+AS_Vol.+consum_O2+int erval_O2+generate_CO2; 0.261, 0.625, tmp+pH_cumulo+PL+AN+in terval_yield+OD+RS+Feed_Vol+RS_cumulo+rab_integral+Accum._Y ield+AS_Vol.+consum_O2; 0.249, 0.625, pH_cumulo+PL+AN+inter val_yield+OD+RS+Feed_Vol+RS_cumulo+rab_integral+AS_Vol.+emi ssion_CO2+consum_O2+interval_O2+generate_CO2; 0.284, 0.625, pH_cumulo+PL+AN+interval_Pdt.+OD+RS+RS_cumulo+Accum._Yield +AS_Vol.+consum_O2+generate_CO2; 0.248, 0.625, tmp_cumulo+p H_cumulo+PL+AN+interval_yield+OD+RS+Feed_Vol+RS_cumulo+rab_ integral+AS_Vol.+emission_CO2+consum_O2+generate_CO2; 0.284, 0.625, pH_cumulo+PL+interval_yield+OD+RS+RS_cumulo+rab_int egral+Accum._Yield+emission_CO2+consum_O2+generate_CO2; 0.2 95, 0.625, pH_cumulo+PL+interval_yield+OD+RS+RS_cumulo+RQ_i ntegral+emission_O2+emission_CO2+consum_O2; 0.273, 0.625, t mp+pH_cumulo+PL+AN+OD+RS+RS_cumulo+Accum._Yield+AS_Vol.+con sum_O2+interval_O2+generate_CO2; 0.273, 0.625, pH_cumulo+PL +AN+interval_yield+OD+RS+RS_cumulo+Cell_conc._interval+rab_ integral+AS_Vol.+consum_O2+generate_CO2; 0.236, 0.625, tmp+ pH_cumulo+PL+AN+interval_yield+OD+RS+Feed_Vol+RS_cumulo+Cel l_conc._interval+rab_integral+Accum._Yield+AS_Vol.+consum_O 2+generate_CO2; 0.261, 0.625, tmp+pH_cumulo+PL+AN+interval_ yield+OD+RS+RS_cumulo+Accum._Yield+AS_Vol.+emission_O2+cons um_O2+generate_CO2; 0.272, 0.625, pH+pH_cumulo+PL+interval_ yield+OD+RS+RS_cumulo+AS_Vol.+emission_CO2+consum_O2+interv al_O2+generate_CO2; 0.272, 0.625, pH_cumulo+PL+AN+interval_ yield+interval_Pdt.+OD+RS+Feed_Vol+RS_cumulo+AS_Vol.+consum _O2+generate_CO2; 0.235, 0.625, tmp+pH_cumulo+PL+AN+interva l_yield+interval_Pdt.+OD+RS+Feed_Vol+RS_cumulo+Accum._Yield +AS_Vol.+emission_CO2+consum_O2+generate_CO2; 0.235, 0.625, pH_cumulo+PL+AN+interval_yield+OD+RS+Feed_Vol+RS_cumulo+Ce ll_conc._interval+rab_integral+Accum._Yield+AS_Vol.+emissio n_O2+consum_O2+generate_CO2; 0.248, 0.625, pH_cumulo+PL+AN+ AN_cumulo+OD+RS+Feed_Vol+RS_cumulo+rab_integral+Accum._Yiel d+AS_Vol.+consum_O2+interval_O2+generate_CO2; 0.272, 0.625, pH_cumulo+PL+PL_cumulo+AN+OD+RS+RS_cumulo+Accum._Yield+AS_ Vol.+consum_O2+interval_O2+generate_CO2; 0.260, 0.625, tmp_ cumulo+pH_cumulo+PL+AN+interval_yield+OD+RS+RS_cumulo+rab_i ntegral+AS_Vol.+emission_CO2+consum_O2+generate_CO2; 0.235, 0.625, pH+pH_cumulo+PL+AN+interval_yield+OD+RS+Feed_Vol+RS _cumulo+Accum._Yield+AS_Vol.+emission_O2+emission_CO2+consu m_O2+generate_CO2; 0.260, 0.625, tmp_cumulo+pH_cumulo+PL+AN +OD+RS+Feed_Vol+RS_cumulo+Accum._Yield+AS_Vol.+consum_O2+in terval_O2+generate_CO2; 0.272, 0.625, pH_cumulo+PL+AN+inter val_Pdt.+OD+RS+Feed_Vol+RS_cumulo+Accum._Yield+AS_Vol.+cons um_O2+generate_CO2; 0.248, 0.625, pH_cumulo+PL+AN+AN_cumulo +interval_yield+OD+RS+Feed_Vol+RS_cumulo+RQ_integral+AS_Vol. +emission_O2+emission_CO2+consum_O2; 0.235, 0.625, tmp+pH_c umulo+PL+AN+interval_yield+interval_Pdt.+OD+RS+Feed_Vol+RS_ cumulo+rab_integral+Accum._Yield+AS_Vol.+consum_O2+generate _CO2; 0.272, 0.625, tmp_cumulo+pH_cumulo+PL+AN+interval_yie ld+OD+RS+Feed_Vol+RS_cumulo+Accum._Yield+AS_Vol.+consum_O2; 0.284, 0.625, pH_cumulo+PL+interval_yield+OD+RS+Feed_Vol+R S_cumulo+Accum._Yield+emission_CO2+consum_O2+generate-CO2; 0.222, 0.625, tmp+pH+pH_cumulo+PL+AN+AN_cumulo+interval_yie ld+OD+RS+Feed_Vol+RS_cumulo+Accum._Yield+AS_Vol.+emission_C O2+consum_O2+generate_CO2; 0.248, 0.624, pH+pH_cumulo+PL+AN +interval_yield+OD+RS+RS_cumulo+Cell_conc._interval+rab_int egral+Accum._Yield+AS_Vol.+consum_O2+generate_CO2; 0.248, 0. 624, pH+pH_cumulo+PL+AN+AN_cumulo+OD+RS+Feed_Vol+RS_cumulo+ Accum._Yield+AS_Vol.+consum_O2+interval_O2+generate_CO2; 0. 272, 0.624, pH_cumulo+PL+AN+interval_yield+OD+RS+Feed_Vol+A ccum._Yield+AS_Vol.+consum_O2+interval_O2+generate_CO2; 0.2 72, 0.624, pH_cumulo+PL+AN_cumulo+OD+RS+RS_cumulo+Cell_conc. _interval+Accum._Yield+AS_Vol. +emission_CO2+consum_O2+gener ate_CO2; 0.235, 0.624, pH+pH_cumulo+PL+AN+interval_yield+OD +RS+Feed_Vol+RS_cumulo+RQ_integral+Accum._Yield+AS_Vol.+emi ssion_O2+emission_CO2+consum_O2; 0.283, 0.624, pH_cumulo+PL +interval_yield+OD+RS+RS_cumulo+AS_Vol.+emission_O2+emissio n_CO2+consum_O2+generate_CO2; 0.248, 0.624, tmp_cumulo+pH_c umulo+PL+AN+interval_yield+OD+RS+RS_cumulo+rab_integral+Acc um._Yield+AS_Vol.+emission_CO2+consum_O2+generate_CO2; 0.26 0, 0.624, tmp+pH_cumulo+PL+interval_yield+OD+RS+Feed_Vol+RS _cumulo+Accum._Yield+AS_Vol.+emission_CO2+consum_O2+generat e_CO2; 0.235, 0.624, pH+pH_cumulo+PL+AN+AN_cumulo+interval_ yield+OD+RS+Feed_Vol+RS_cumulo+Accum._Yield+AS_Vol.+consum_ O2+interval_O2+generate_CO2; 0.283, 0.624, pH+pH_cumulo+PL+ AN_cumulo+interval_yield+OD+RS+RS_cumulo+emission_CO2+consu m_O2+generate_CO2; 0.260, 0.624, pH_cumulo+PL+AN+interval_y ield+OD+RS+Feed_Vol+RS_cumulo+Cell_conc._interval+rab_integ ral+Accum._Yield+AS_Vol.+consum_O2; 0.283, 0.624, tmp+pH_cu mulo+PL+AN_cumulo+interval_yield+OD+RS+RS_cumulo+emission_C O2+consum_O2+generate_CO2; 0.221, 0.624, pH+pH_cumulo+PL+AN +AN_cumulo+interval_yield+OD+RS+Feed_Vol+RS_cumulo+Accum._Y ield+AS_Vol.+emission_CO2+consum_O2+interval_O2+generate_CO 2; 0.235, 0.624, pH+pH_cumulo+PL+AN+AN_cumulo+OD+RS+Feed_Vo l+RS_cumulo+Accum._Yield+AS_Vol.+emission_CO2+consum_O2+int erval_O2+generate_CO2; 0.247, 0.624, tmp+tmp_cumulo+pH_cumu lo+PL+AN+AN_cumulo+interval_yield+OD+RS+RS_cumulo+Accum._Yi eld+AS_Vol.+consum_O2+generate_CO2; 0.283, 0.624, pH_cumulo +PL+AN+interval_yield+OD+RS+RS_cumulo+emission_CO2+consum_O 2+interval_O2+generate_CO2; 0.260, 0.624, tmp+pH_cumulo+PL+ AN+OD+RS+Feed_Vol+RS_cumulo+Accum._Yield+AS_Vol.+consum_O2+ interval_O2+generate_CO2; 0.294, 0.624, pH_cumulo+PL+OD+RS+ RS_cumulo+Accum._Yield+AS_Vol.+emission_CO2+consum_O2+gener ate_CO2; 0.260, 0.624, pH_cumulo+PL+AN_cumulo+OD+RS+Feed_Vo l+RS_cumulo+Accum._Yield+AS Vol.+emission_CO2+consum_O2+int erval_O2+generate_CO2; 0.316, 0.624, pH_cumulo+PL+interval_ yield+OD+RS+RS_cumulo+AS_Vol.+consum_O2; 0.260, 0.624, pH_c umulo+PL+AN+interval_yield+interval_Pdt.+OD+RS+RS_cumulo+Ce ll_conc._interval+Accum._Yield+AS_Vol.+consum_O2+generate_C 02; 0.234, 0.624, pH+pH_cumulo+PL+PL_cumulo+AN+interval_yie ld+OD+RS+Feed_Vol+RS_cumulo+rab_integral+Accum._Yield+AS_Vo l.+consum_O2+generate_CO2; 0.271, 0.624, pH+pH_cumulo+PL+AN +OD+RS+RS_cumulo+Cell_conc._interval+Accum._Yield+AS_Vol.+c onsum_O2+generate_CO2; 0.271, 0.624, tmp+pH_cumulo+PL+AN+in terval_yield+OD+RS+RS_cumulo+RQ_integral+Accum._Yield+AS_Vo l.+consum_O2; 0.283, 0.624, tmp+pH_cumulo+PL+AN+interval_yi eld+OD+RS+RS_cumulo+emission_CO2+consum_O2+generate_CO2; 0. 260, 0.624, pH_cumulo+PL+AN+AN_cumulo+OD+RS+RS_cumulo+rab_i ntegral+Accum._Yield+AS_Vol.+consum_O2+interval_O2+generate _CO2; 0.283, 0.624, pH_cumulo+PL+AN+interval_yield+OD+RS+Fe ed_Vol+RS_cumulo+rab_integral+AS_Vol.+consum_O2; 0.259, 0.6 24, pH_cumulo+PL+AN+interval_yield+OD+RS+RS_cumulo+Cell_con c._interval+AS_Vol.+emission_CO2+consum_O2+interval_O2+gene rate_CO2; 0.271, 0.624, pH_cumulo+PL+interval_yield+OD+RS+R S_cumulo+Cell_conc._interval+rab_integral+AS_Vol.+emission_ CO2+consum_O2+generate_CO2; 0.283, 0.624, pH+PL+AN+interval _yield+OD+RS+RS_cumulo+Accum._Yield+AS_Vol.+consum_O2+gener ate_CO2; 0.259, 0.624, pH_cumulo+PL+PL_cumulo+AN+interval_y ield+OD+RS+RS_cumulo+rab_integral+Accum._Yield+AS_Vol.+cons um_O2+generate_CO2; 0.283, 0.624, pH_cumulo+PL+AN_cumulo+in terval_yield+OD+RS+RS_cumulo+rab_integral+RQ_integral+AS_Vo l.+consum_O2; 0.247, 0.624, tmp_cumulo+pH_cumulo+PL+AN+inte rval_yield+OD+RS+RS_cumulo+Cell_conc._interval+Accum._Yield +AS_Vol.+emission_CO2+consum_O2+generate_CO2; 0.221, 0.624, pH+pH_cumulo+PL+AN+interval_yield+OD+RS+Feed_Vol+RS_cumulo +Cell_conc._interval+rab_integral+Accum._Yield+AS_Vol.+emis sion_CO2+consum_O2+generate_CO2; 0.259, 0.624, tmp_cumulo+p H_cumulo+PL+AN+interval_yield+OD+RS+RS_cumulo+Cell_conc._in terval+AS_Vol.+emission_CO2+consum_O2+generate_CO2; 0.259, 0.624, tmp_cumulo+pH_cumulo+PL+AN+AN_cumulo+OD+RS+RS_cumulo +Accum._Yield+AS_Vol.+emission_CO2+consum_O2+generate_CO2; 0.294, 0.624, pH_cumulo+PL+interval_yield+OD+RS+Feed_Vol+RS _cumulo+RQ_integral+AS_Vol.+consum_O2; 0.283, 0.624, pH_cum ulo+PL+AN+interval_yield+OD+RS+Feed_Vol+AS_Vol.+emission_CO 2+consum_O2+generate_CO2; 0.247, 0.624, pH+pH_cumulo+PL+AN+ interval_yield+OD+RS+Feed_Vol+RS_cumulo+RQ_integral+Accum._ Yield+AS_Vol.+emission_CO2+consum_O2; 0.247, 0.624, pH+pH_c umulo+PL+AN_cumulo+interval_yield+OD+RS+RS_cumulo+rab_integ ral+Accum._Yield+AS_Vol.+emission_CO2+consum_O2+generate_CO 2; 0.247, 0.624, tmp_cumulo+pH_cumulo+PL+AN+interval_yield+ OD+RS+Feed_Vol+RS_cumulo+Accum._Yield+AS_Vol.+emission_O2+c onsum_O2+generate_CO2; 0.247, 0.624, pH+pH_cumulo+PL+AN+AN_ cumulo+interval_yield+OD+RS+RS_cumulo+Cell_conc._interval+A ccum._Yield+AS_Vol.+consum_O2+generate_CO2; 0.259, 0.624, p H_cumulo+PL+PL_cumulo+AN+OD+RS+Feed_Vol+RS_cumulo+Accum._Yi eld+AS_Vol.+emission_CO2+consum_O2+generate_CO2; 0.234, 0.6 24, pH+pH_cumulo+PL+AN+AN_cumulo+interval_yield+OD+RS+Feed_ Vol+RS_cumulo+Accum._Yield+AS_Vol.+emission_O2+consum-O2+ge nerate_CO2; 0.247, 0.624, tmp_cumulo+pH_cumulo+PL+AN+interv al_yield+OD+RS+Feed_Vol+RS_cumulo+RQ_integral+Accum._Yield+ AS_Vol.+emission_O2+consum_O2; 0.283, 0.624, pH_cumulo+PL+i nterval_yield+interval_Pdt.+OD+RS+RS_cumulo+AS_Vol.+emissio n_CO2+consum_O2+generate_CO2; 0.247, 0.624, pH+pH_cumulo+PL +AN+OD+RS+Feed_Vol+RS_cumulo+rab_integral+Accum._Yield+AS_V ol.+emission_CO2+consum_O2+generate_CO2; 0.271, 0.624, pH_c umulo+PL+AN_cumulo+interval_yield+OD+RS+Feed_Vol+RS_cumulo+ Accum._Yield+emission_CO2+consum_O2+generate_CO2; 0.247, 0. 624, pH_cumulo+PL+PL_cumulo+AN+interval_yield+OD+RS+Feed_Vo l+RS_cumulo+rab_integral+RQ_integral+Accum._Yield+AS_Vol.+c onsum_O2; 0.221, 0.624, pH+pH_cumulo+PL+AN+interval_yield+O D+RS+Feed_Vol+RS_cumulo+rab_integral+Accum._Yield+AS_Vol.+e mission_CO2+consum_O2+interval_O2+generate_CO2; 0.271, 0.62 4, pH_cumulo+PL+AN+interval_yield+OD+RS+Feed_Vol+RS_cumulo+ Accum._Yield+AS_Vol.+emission_O2+consum_O2; 0.259, 0.624, p H_cumulo+PL+AN+interval_yield+interval_Pdt.+OD+RS+Feed_Vol+ Accum._Yield+AS_Vol.+emission_O2+consum_O2+generate_CO2; 0. 282, 0.624, pH_cumulo+PL+PL_cumulo+interval_yield+OD+RS+RS_ cumulo+AS_Vol. +emission_CO2+consum_O2+generate_CO2; 0.259, 0.624, pH_cumulo+PL+AN+interval_Pdt.+OD+RS+Feed_Vol+RS_cumu lo+RQ_integral+Accum._Yield+AS_Vol. +emission_CO2+consum_O2; 0.271, 0.624, tmp_cumulo+pH_cumulo+PL+AN+interval_yield+OD +RS+RS_cumulo+RQ_integral+Accum._Yield+AS_Vol.+consum_O2; 0. 271, 0.624, pH_cumulo+PL+interval_yield+OD+RS+RS_cumulo+Cel l_conc._interval+AS_Vol.+emission_CO2+consum_O2+interval_O2 +generate_CO2; 0.234, 0.624, pH_cumulo+PL+AN+interval_yield +OD+RS+Feed_Vol+RS_cumulo+RQ_integral+Accum._Yield+AS_Vol.+ emission_O2+emission_CO2+consum_O2+interval_O2; 0.246, 0.62 4, pH_cumulo+PL+AN+interval_Pdt.+OD+RS+Feed_Vol+RS_cumulo+R Q_integral+Accum._Yield+AS_Vol.+emission_O2+emission_CO2+co nsum_O2; 0.220, 0.624, tmp_cumulo+pH_cumulo+PL+AN+AN_cumulo +interval_yield+OD+RS+Feed_Vol+RS_cumulo+rab_integral+Accum. _Yield+AS_Vol.+emission_CO2+consum_O2+generate_CO2; 0.246, 0.624, pH_cumulo+PL+AN+interval_yield+OD+RS+RS_cumulo+rab_i ntegral+Accum._Yield+AS_Vol.+emission_O2+consum_O2+interval _O2+generate_CO2; 0.246, 0.624, pH_cumulo+PL+AN+OD+RS+Feed_ _Vol+RS_cumulo+Cell_conc._interval+Accum._Yield+AS_Vol.+emis sion_CO2+consum_O2+interval_O2+generate_CO2; 0.259, 0.624, pH+pH_cumulo+PL+AN+interval_yield+OD+RS+RS_cumulo+AS_Vol.+e mission_CO2+consum_O2+interval_O2+generate_CO2; 0.234, 0.62 4, pH+pH_cumulo+PL+AN+AN_cumulo+interval_yield+OD+RS+Feed_V ol+RS_cumulo+rab_integral+RQ_integral+Accum._Yield+AS_Vol.+ consum_O2; 0.259, 0.624, pH_cumulo+PL+AN+interval_yield+int erval_Pdt.+OD+RS+RS_cumulo+RQ_integral+Accum._Yield+AS_Vol. +emission_CO2+consum_O2; 0.293, 0.624, pH_cumulo+PL+AN_cumu lo+interval_yield+OD+RS+RS_cumulo+AS_Vol.+consum_O2+generat e_CO2; 0.282, 0.624, pH_cumulo+PL+AN_cumulo+OD+RS+Feed_Vol+ RS_cumulo+Accum._Yield+AS_Vol.+consum_O2+generate_CO2; 0.23 4, 0.624, pH+pH_cumulo+PL+AN+AN_cumulo+interval_yield+OD+RS +Feed_Vol+RS_cumulo+RQ_integral+Accum._Yield+AS_Vol.+emissi on_O2+consum_O2; 0.259, 0.624, pH_cumulo+PL+AN+interval_yie ld+interval_Pdt.+OD+RS+RS_cumulo+Accum._Yield+AS_Vol.+emiss ion_O2+consum_O2+generate_CO2; 0.259, 0.624, pH+PL+AN+AN_cu mulo+interval_yield+OD+RS+RS_cumulo+Cell_conc._interval+Acc um._Yield+AS_Vol.+consum_O2+generate_CO2; 0.293, 0.624, pH_ cumulo+PL+interval_yield+OD+RS+RS_cumulo+AS_Vol.+emission_O 2+consum_O2+generate_CO2; 0.246, 0.624, pH+pH_cumulo+PL+AN+ AN_cumulo+OD+RS+Feed_Vol+RS_cumulo+rab_integral+Accum._Yiel d+AS_Vol.+consum_O2+generate_CO2; 0.233, 0.624, tmp+pH+pH_c umulo+PL+AN+interval_yield+OD+RS+Feed_Vol+RS_cumulo+rab_int egral+Accum._Yield+AS_Vol.+consum_O2+generate_CO2; 0.259, 0. 624, tmp+tmp_cumulo+pH_cumulo+PL+AN_cumulo+interval_yield+O D+RS+RS_cumulo+AS_Vol.+emission_CO2+consum_O2+generate_CO2; 0.233, 0.624, pH_cumulo+PL+AN+interval_yield+interval_Pdt. +OD+RS+Feed_Vol+RS_cumulo+rab_integral+Accum._Yield+AS_Vol. +emission_O2+consum_O2+generate_CO2; 0.259, 0.624, pH+pH_cu mulo+PL+AN_cumulo+interval_yield+OD+RS+RS_cumulo+rab_integr al+AS_Vol.+emission_CO2+consum_O2+generate_CO2; 0.246, 0.62 4, pH_cumulo+PL+AN+AN_cumulo+interval_yield+OD+RS+Feed_Vol+ RS_cumulo+AS_Vol.+emission_CO2+consum_O2+interval_O2+genera te_CO2; 0.259, 0.624, pH_cumulo+PL+AN+AN_cumulo+interval_yi eld+interval_Pdt.+OD+RS+RS_cumulo+Accum._Yield+AS_Vol.+cons um_O2+generate_CO2; 0.220, 0.624, pH+pH_cumulo+PL+PL_cumulo +AN+interval_yield+OD+RS+Feed_Vol+RS_cumulo+rab_integral+Ac cum._Yield+AS_Vol.+emission_CO2+consum_O2+generate_CO2 0.2 58, 0.624, pH_cumulo+PL+PL_cumulo+AN+OD+RS+Feed_Vol+RS_cumu lo+Accum._Yield+AS_Vol.+consum_O2+interval_O2+generate_CO2; 0.270, 0.623, pH_cumulo+PL+AN+interval_yield+OD+RS+Feed_Vo l+RS_cumulo+Cell_conc._interval+AS_Vol.+consum_O2+generate CO2; 0.282, 0.623, pH_cumulo+PL+interval_yield+OD+RS+RS_cum ulo+rab_integral+RQ_integral+emission_O2+emission_CO2+consu m_O2; 0.246, 0.623, pH_cumulo+PL+AN+interval_yield+OD+RS+Fe ed_Vol+RS_cumulo+Cell_conc._interval+RQ_integral+Accum._Yie ld+AS_Vol.+emission_CO2+consum_O2; 0.282, 0.623, pH_cumulo+ PL+PL_cumulo+AN+interval_yield+OD+RS+RS_cumulo+RQ_integral+ AS_Vol.+consum_O2; 0.258, 0.623, pH+pH_cumulo+PL+AN+interva l_yield+OD+RS+RS_cumulo+rab_integral+RQ_integral+Accum._Yie ld+AS_Vol.+consum_O2; 0.282, 0.623, pH_cumulo+PL+interval_y ield+OD+RS+RS_cumulo+rab_integral+Accum._Yield+AS_Vol.+cons um_O2+generate_CO2; 0.282, 0.623, tmp+pH_cumulo+PL+AN+inter val_yield+OD+RS+RS_cumulo+RQ_integral+AS_Vol.+consum_O2; 0. 282, 0.623, pH_cumulo+PL+interval_yield+OD+RS+Feed_Vol+RS_c umulo+rab_integral+emission_CO2+consum_O2+generate_CO2; 0.2 82, 0.623, pH_cumulo+PL+AN+interval_yield+OD+RS+Feed_Vol+RQ _integral+Accum._Yield+AS_Vol.+consum_O2; 0.270, 0.623, pH_ cumulo+PL+AN+AN_cumulo+interval_yield+OD+RS+RS_cumulo+RQ_in tegral+AS_Vol.+emission_O2+consum_O2; 0.258, 0.623, pH_cumu lo+PL+AN_cumulo+interval_Pdt.+OD+RS+RS_cumulo+RQ_integral+A ccum._Yield+AS_Vol. +emission_O2+emission_CO2+consum_O2; 0.2 46, 0.623, pH+pH_cumulo+PL+PL_cumulo+AN+interval_yield+OD+R S+Feed_Vol+RS_cumulo+RQ_integral+Accum._Yield+AS_Vol.+consu m_O2; 0.246, 0.623, pH_cumulo+PL+AN+AN_cumulo+interval_yiel d+OD+RS+RS_cumulo+Cell_conc._interval+Accum._Yield+AS_Vol.+ consum_O2+interval_O2+generate_CO2; 0.233, 0.623, tmp+pH_cu mulo+PL+AN+AN_cumulo+interval_yield+OD+RS+Feed_Vol+RS_cumul o+Cell_conc._interval+Accum._Yield+AS_Vol.+consum_O2+genera te_CO2; 0.258, 0.623, pH_cumulo+PL+AN_cumulo+interval_yield +OD+RS+Feed_Vol+RS_cumulo+AS_Vol.+emission_CO2+consum_O2+in terval_O2+generate_CO2; 0.233, 0.623, pH_cumulo+PL+AN+inter val_yield+OD+RS+Feed_Vol+RS_cumulo+Cell_conc._interval+RQ_i ntegral+Accum._Yield+AS_Vol.+emission_O2+emission_CO2+consu m_02; 0.220, 0.623, tmp+tmp_cumulo+pH_cumulo+PL+AN+interval _yield+OD+RS+Feed_Vol+RS_cumulo+rab_integral+Accum._Yield+A S_Vol.+emission_CO2+consum_O2+generate_CO2; 0.233, 0.623, t mp+pH_cumulo+PL+PL_cumulo+AN+interval_yield+OD+RS+Feed_Vol+ RS_cumulo+Accum._Yield+AS_Vol.+emission_CO2+consum_O2+gener ate_CO2; 0.270, 0.623, tmp+pH_cumulo+PL+interval_yield+OD+R S+RS_cumulo+rab_integral+AS_Vol.+emission_CO2+consum_O2+gen erate-CO2; 0.246, 0.623, tmp_cumulo+pH_cumulo+PL+AN+interva l_yield+OD+RS+Feed_Vol+RS_cumulo+rab_integral+RQ_integral+A ccum._Yield+AS_Vol.+consum_O2; 0.282, 0.623, pH_cumulo+PL+i nterval_yield+OD+RS+Feed_Vol+RS_cumulo+emission_CO2+consum_ O2+interval_O2+generate_CO2; 0.258, 0.623, pH+PL+AN+interva l_yield+OD+RS+Feed_Vol+RS_cumulo+rab_integral+Accum._Yield+ AS_Vol.+consum_O2+generate_CO2; 0.270, 0.623, pH_cumulo+PL+ AN_cumulo+interval_yield+OD+RS+RS_cumulo+RQ_integral+AS_Vol. +emission_O2+emission_CO2+consum_O2; 0.293, 0.623, pH+pH_cu mulo+PL+interval_yield+OD+RS+RS_cumulo+RQ_integral+AS_Vol.+ consum_O2; 0.270, 0.623, tmp+pH_cumulo+PL+AN+AN_cumulo+OD+R S+RS_cumulo+Accum._Yield+AS_Vol.+consum_O2+generate_CO2; 0. 233, 0.623, pH+pH_cumulo+PL+AN+interval_yield+OD+RS+Feed_Vo l+RS_cumulo+rab_integral+RQ_integral+Accum._Yield+AS_Vol.+e mission_O2+consum_O2; 0.246, 0.623, pH_cumulo+PL+AN+AN_cumu lo+interval_yield+OD+RS+RS_cumulo+rab_integral+RQ_integral+ Accum._Yield+AS_Vol.+emission_O2+consum_O2; 0.282, 0.623, p H+pH_cumulo+PL+AN+interval_yield+OD+RS+RS_cumulo+emission_C O2+consum_O2+generate_CO2; 0.270, 0.623, tmp+pH_cumulo+PL+A N_cumulo+interval_yield+OD+RS+RS_cumulo+Accum._Yield+AS_Vol. +consum_O2+generate_CO2; 0.258, 0.623, pH_cumulo+PL+AN+AN_c umulo+interval_yield+OD+RS+Feed_Vol+rab_integral+Accum._Yie ld+AS_Vol.+consum_O2+generate_CO2; 0.282, 0.623, pH_cumulo+ PL+AN_cumulo+OD+RS+RS_cumulo+Cell_conc._interval+Accum._Yie ld+AS_Vol.+consum_O2+generate_CO2; 0.220, 0.623, tmp_cumulo +pH+pH_cumulo+PL+AN+AN_cumulo+interval_yield+OD+RS+Feed_Vol +RS_cumulo+Accum._Yield+AS_Vol.+emission_CO2+consum_O2+gene rate_CO2; 0.258, 0.623, tmp_cumulo+pH_cumulo+PL+AN+interval yield+OD+RS+RS_cumulo+Accum._Yield+AS_Vol.+emission_O2+con sum-O2+generate_CO2; 0.246, 0.623, pH_cumulo+PL+PL_cumulo+A N+interval_yield+OD+RS+Feed_Vol+RS_cumulo+Cell_conc._interv al+Accum._Yield+AS_Vol.+consum_O2+generate_CO2; 0.246, 0.62 3, pH+PL+AN+AN_cumulo+interval_yield+interval_Pdt.+OD+RS+Fe ed_Vol+RS_cumulo+Accum._Yield+AS_Vol.+consum_O2+generate_CO 2; 0.258, 0.623, pH_cumulo+PL+AN+interval_yield+OD+RS+Feed_ Vol+RS_cumulo+rab_integral+Accum._Yield+AS_Vol.+emission_O2 +consum_O2; 0.246, 0.623, pH+pH_cumulo+PL+AN+interval_yield +OD+RS+Feed_Vol+RS_cumulo+Cell_conc._interval+AS_Vol.+emiss ion_CO2+consum_O2+generate_CO2; 0.233, 0.623, pH_cumulo+PL+ AN+interval_yield+interval_Pdt.+OD+RS+Feed_Vol+RS_cumulo+Ce ll_conc._interval+rab_integral+Accum._Yield+AS_Vol.+consum_ O2+generate_CO2; 0.246, 0.623, pH+pH_cumulo+PL+PL_cumulo+AN +OD+RS+Feed_Vol+RS_cumulo+Accum._Yield+AS_Vol.+emission_CO2 +consum_O2+generate_CO2; 0.258, 0.623, pH_cumulo+PL+AN+AN_c umulo+OD+RS+RS_cumulo+Cell_conc._interval+Accum._Yield+AS_V ol.+consum_O2+interval_O2+generate_CO2; 0.270, 0.623, pH+pH _cumulo+PL+interval_yield+OD+RS+RS_cumulo+Cell_conc._interv al+AS_Vol.+emission_CO2+consum_O2+generate_CO2; 0.270, 0.62 3, pH_cumulo+PL+AN_cumulo+interval_Pdt.+OD+RS+RS_cumulo+RQ_ integral+Accum._Yield+AS_Vol.+emission_CO2+consum_O2; 0.245, 0.623, pH_cumulo+PL+AN+AN_cumulo+interval_yield+OD+RS+RS_c umulo+rab_integral+Accum._Yield+AS_Vol.+emission_O2+consum_ O2+generate_CO2; 0.258, 0.623, tmp+pH_cumulo+PL+AN+interval _yield+OD+RS+RS_cumulo+AS_Vol.+emission_CO2+consum_O2+inter val_O2+generate_CO2; 0.258, 0.623, pH_cumulo+PL+AN+AN_cumul o+interval_Pdt.+OD+RS+RS_cumulo+RQ_integral+Accum._Yield+AS _Vol.+emission_CO2+consum_O2; 0.219, 0.623, pH_cumulo+PL+AN +AN_cumulo+interval_yield+OD+RS+Feed_Vol+RS_cumulo+rab_inte gral+RQ_integral+Accum._Yield+AS_Vol.+emission_O2+emission_ CO2+consum_O2; 0.245, 0.623, pH+pH_cumulo+PL+AN+AN_cumulo+O D+RS+Feed_Vol+RS_cumulo+Cell_conc._interval+Accum._Yield+AS _Vol.+consum_O2+generate_CO2; 0.245, 0.623, pH+pH_cumulo+PL +AN+AN_cumulo+interval_yield+OD+RS+Feed_Vol+RS_cumulo+rab_i ntegral+Accum._Yield+AS_Vol.+consum_O2; 0.281, 0.623, pH+pH _cumulo+PL+interval_yield+OD+RS+RS_cumulo+RQ_integral+Accum. _Yield+AS_Vol.+consum_O2; 0.245, 0.623, tmp+pH+pH_cumulo+PL +AN+interval_yield+OD+RS+RS_cumulo+Accum._Yield+AS_Vol.+emi ssion_CO2+consum_O2+generate_CO2; 0.258, 0.623, pH_cumulo+P L+AN+OD+RS+Feed_Vol+RS_cumulo+rab_integral+Accum._Yield+AS_ Vol.+emission_O2+consum_O2+generate_CO2; 0.232, 0.623, tmp+ tmp_cumulo+pH_cumulo+PL+AN+AN_cumulo+interval_yield+OD+RS+R S_cumulo+Accum._Yield+AS_Vol.+emission_CO2+consum_O2+genera te_CO2; 0.245, 0.623, tmp+pH+pH_cumulo+PL+AN+interval_yield +OD+RS+Feed_Vol+RS_cumulo+AS_Vol.+emission_CO2+consum_O2+ge nerate_CO2; 0.281, 0.623, pH_cumulo+PL+interval_yield+OD+RS +RS_cumulo+Accum._Yield+AS_Vol.+emission_O2+consum_O2+gener ate_CO2; 0.292, 0.623, pH_cumulo+PL+AN+interval_yield+OD+RS +Feed_Vol+Accum._Yield+AS_Vol.+consum_O2; 0.257, 0.623, tmp +pH+pH_cumulo+PL+AN+interval_yield+OD+RS+RS_cumulo+AS_Vol.+ emission_CO2+consum_O2+generate_CO2; 0.245, 0.623, pH_cumul o+PL+PL_cumulo+AN+interval_yield+OD+RS+RS_cumulo+Accum._Yie ld+AS_Vol.+emission_CO2+consum_O2+interval_O2+generate_CO2; 0.281, 0.623, pH_cumulo+PL+AN_cumulo+interval_Pdt.+OD+RS+R S_cumulo+RQ_integral+Accum._Yield+AS_Vol.+consum_O2; 0.245, 0.623, tmp_cumulo+pH+pH_cumulo+PL+AN+interval_yield+OD+RS+ RS_cumulo+Accum._Yield+AS_Vol._+emission_CO2+consum_O2+gener ate_CO2; 0.269, 0.623, pH+pH_cumulo+PL+AN_cumulo+interval_y ield+OD+RS+RS_cumulo+Accum._Yield+AS_Vol.+consum_O2+generat e_CO2; 0.257, 0.623, pH+pH_cumulo+PL+AN_cumulo+OD+RS+Feed_V ol+RS_cumulo+Accum._Yield+AS_Vol.+emission_CO2+consum_O2+ge nerate_CO2; 0.269, 0.623, pH_cumulo+PL+AN+AN_cumulo+interva l_yield+OD+RS+RS_cumulo+Accum._Yield+emission_CO2+consum_O2 +generate_CO2; 0.232, 0.623, tmp_cumulo+pH_cumulo+PL+AN+int erval_yield+OD+RS+Feed_Vol+RS_cumulo+Cell_conc._interval+ra b_integral+Accum._Yield+AS_Vol.+consum_O2+generate_CO2; 0.2 32, 0.623, tmp_cumulo+pH+pH_cumulo+PL+AN+interval_yield+OD+ RS+Feed_Vol+RS_cumulo+rab_integral+Accum._Yield+AS_Vol.+con sum_O2+generate_CO2; 0.257, 0.623, tmp_cumulo+pH_cumulo+PL+ AN+interval_yield+OD+RS+RS_cumulo+AS_Vol.+emission_CO2+cons um_O2+interval_O2+generate_CO2; 0.219, 0.623, pH+pH_cumulo+ PL+PL_cumulo+AN+AN_cumulo+interval_yield+OD+RS+Feed_Vol+RS_ cumulo+Accum._Yield+AS_Vol.+emission_CO2+consum_O2+generate _CO2; 0.257, 0.623, pH_cumulo+PL+AN+AN_cumulo+interval_yiel d+OD+RS+Feed_Vol+RS_cumulo+RQ_integral+AS_Vol.+emission_CO2 +consum_O2; 0.281, 0.623, pH_cumulo+PL+interval_yield+OD+RS +RS_cumulo+rab_integral+emission_CO2+consum_O2+interval_O2+ generate_CO2; 0.257, 0.623, pH_cumulo+PL+AN+interval_yield+ OD+RS+Feed_Vol+rab_integral+Accum._Yield+AS_Vol.+emission_C O2+consum_O2+generate_CO2; 0.232, 0.623, pH_cumulo+PL+AN+in terval_yield+interval_Pdt.+OD+RS+Feed_Vol+RS_cumulo+Cell_co nc._interval+Accum._Yield+AS_Vol.+emission_CO2+consum_O2+ge nerate_CO2; 0.281, 0.623, pH_cumulo+PL+AN+AN_cumulo+interva l_yield+OD+RS+Feed_Vol+RS_cumulo+AS_Vol.+consum_O2; 0.245, 0.623, tmp_cumulo+pH+pH_cumulo+PL+AN+interval_yield+OD+RS+F eed_Vol+RS_cumulo+AS_Vol.+emission_CO2+consum_O2+generate_C O2; 0.281, 0.623, tmp_cumulo+pH_cumulo+PL+AN_cumulo+interva l_yield+OD+RS+RS_cumulo+emission_CO2+consum_O2+generate_CO 2; 0.232, 0.623, pH_cumulo+PL+AN+AN_cumulo+interval_yield+O D+RS+Feed_Vol+RS_cumulo+Accum._Yield+AS_Vol.+emission_O2+co nsum_O2+interval_O2+generate_CO2; 0.303, 0.623, pH_cumulo+P L+interval_yield+OD+RS+RS_cumulo+Accum._Yield+AS_Vol.+consu m_O2; 0.257, 0.623, tmp+pH_cumulo+PL+AN+AN_cumulo+OD+RS+RS_ cumulo+Accum._Yield+AS_Vol.+emission_CO2+consum_O2+generate _CO2; 0.257, 0.623, pH_cumulo+PL+AN_cumulo+interval_yield+O D+RS+Feed_Vol+RS_cumulo+rab_integral+Accum._Yield+AS_Vol.+c onsum_O2+generate_CO2; 0.245, 0.623, pH_cumulo+PL+PL_cumulo +AN+interval_yield+interval_Pdt.+OD+RS+Feed_Vol+RS_cumulo+A ccum._Yield+AS_Vol.+consum_O2+generate_CO2; 0.257, 0.623, p H_cumulo+PL+AN+interval_yield+interval_Pdt.+OD+RS+RS_cumulo +RQ_integral+Accum._Yield+AS_Vol.+emission_O2+consum_O2; 0. 232, 0.623, pH_cumulo+PL+AN+AN_cumulo+interval_yield+OD+RS+ Feed_Vol+RS_cumulo+Cell_conc._interval+Accum._Yield+AS_Vol. +consum_O2+interval_O2+generate_CO2; 0.257, 0.622, tmp_cumu lo+pH_cumulo+PL+AN+OD+RS+RS_cumulo+Accum._Yield+AS_Vol.+emi ssion_CO2+consum_O2+interval_O2+generate_CO2; 0.257, 0.622, pH+pH_cumulo+PL+AN+interval_yield+interval_Pdt.+OD+RS+Feed _Vol+Accum._Yield+AS_Vol.+consum_O2+generate_CO2; 0.257, 0. 622, pH_cumulo+PL+interval_yield+OD+RS+Feed_Vol+RS_cumulo+r ab_integral+RQ_integral+AS_Vol.+emission_O2+emission_CO2+co nsum_O2; 0.244, 0.622, pH+pH_cumulo+PL+AN+AN_cumulo+interva l_yield+OD+RS+RS_cumulo+rab_integral+Accum._Yield+AS_Vol.+c onsum_O2+generate_CO2; 0.280, 0.622, pH_cumulo+PL+AN+interv al_yield+interval_Pdt.+OD+RS+RS_cumulo+RQ_integral+AS_Vol.+ consum_O2; 0.232, 0.622, tmp+pH_cumulo+PL+AN+interval_yield +OD+RS+Feed_Vol+RS_cumulo+rab_integral+Accum._Yield+AS_Vol. +consum_O2+interval_O2+generate_CO2; 0.244, 0.622, tmp+pH_c umulo+PL+AN+interval_yield+OD+RS+Feed_Vol+RS_cumulo+Cell_co nc._interval+AS_Vol.+emission_CO2+consum_O2+generate_CO2; 0. 269, 0.622, pH_cumulo+PL+AN+interval_Pdt.+OD+RS+RS_cumulo+r ab_integral+RQ_integral+Accum._Yield+AS_Vol.+consum_O2; 0.2 44, 0.622, pH+pH_cumulo+PL+AN+OD+RS+Feed_Vol+RS_cumulo+rab_ integral+Accum._Yield+AS_Vol.+consum_O2+interval_O2+generat e_CO2; 0.244, 0.622, pH+pH_cumulo+PL+AN+interval_yield+OD+R S+RS_cumulo+rab_integral+Accum._Yield+AS_Vol.+consum_O2+int erval_O2+generate_CO2; 0.244, 0.622, pH+PL+AN+AN_cumulo+int erval_yield+OD+RS+Feed_Vol+RS_cumulo+Accum._Yield+AS_Vol.+e mission_CO2+consum_O2+generate_CO2; 0.244, 0.622, pH_cumulo +PL+AN_cumulo+interval_yield+OD+RS+RS_cumulo+rab_integral+A ccum._Yield+AS_Vol.+emission_CO2+consum_O2+interval_O2+gene rate_CO2; 0.269, 0.622, tmp+pH_cumulo+PL+AN+interval_yield+ OD+RS+Feed_Vol+Accum._Yield+AS_Vol.+consum_O2+generate_CO2; 0.280, 0.622, tmp_cumulo+pH_cumulo+PL+AN+interval_yield+OD +RS+RS_cumulo+emission_CO2+consum_O2+generate_CO2; 0.291, 0. 622, pH_cumulo+AN+interval_yield+OD+RS+RS_cumulo+RQ_integra l+Accum._Yield+AS_Vol.+consum_O2; 0.269, 0.622, tmp+pH_cumu lo+PL+interval_yield+OD+RS+RS_cumulo+AS_Vol.+emission_CO2+c onsum_O2+interval_O2+generate_CO2; 0.231, 0.622, pH_cumulo+ PL+AN+interval_yield+interval_Pdt.+OD+RS+Feed_Vol+RS_cumulo +Accum._Yield+AS_Vol.+emission_CO2+consum_O2+interval_O2+ge nerate_CO2; 0.244, 0.622, pH+pH_cumulo+PL+AN+interval_yield +interval_Pdt.+OD+RS+Feed_Vol+RS_cumulo+RQ_integral+Accum._ Yield+AS_Vol.+consum_O2; 0.280, 0.622, pH_cumulo+PL+interva l_Pdt.+OD+RS+RS_cumulo+RQ_integral+Accum._Yield+AS_Vol.+emi ssion_CO2+consum_O2; 0.268, 0.622, pH_cumulo+PL+AN+AN_cumul o+interval_yield+OD+RS+Feed_Vol+RS_cumulo+AS_Vol.+consum_O2 +generate_CO2; 0.244, 0.622, pH_cumulo+PL+AN+AN_cumulo+inte rval_yield+OD+RS+RS_cumulo+Accum._Yield+AS_Vol.+emission_O2 +consum_O2+interval_O2+generate_CO2; 0.256, 0.622, pH_cumul o+PL+AN_cumulo+interval_yield+OD+RS+Feed_Vol+RS_cumulo+Accu m._Yield+AS_Vol.+emission_O2+consum_O2+generate_CO2; 0.256, 0.622, pH_cumulo+PL+AN_cumulo+OD+RS+Feed_Vol+RS_cumulo+rab _integral+Accum._Yield+AS_Vol.+emission_CO2+consum_O2+gener ate_CO2; 0.268, 0.622, pH_cumulo+PL+AN+interval_yield+OD+RS +RS_cumulo+Accum._Yield+emission_CO2+consum_O2+interval_O2+ generate_CO2; 0.256, 0.622, pH_cumulo+PL+AN+interval_yield+ OD+RS+RS_cumulo+Cell_conc._interval+RQ_integral+Accum._Yiel d+AS_Vol.+emission_O2+consum_O2; 0.244, 0.622, pH+pH_cumulo +PL+AN+interval_yield+OD+RS+Feed_Vol+RS_cumulo+Cell_conc._i nterval+RQ_integral+Accum._Yield+AS_Vol.+consum_O2; 0.280, 0.622, pH_cumulo+PL+AN+OD+RS+Feed_Vol+RS_cumulo+RQ_integral +Accum._Yield+AS_Vol.+consum_O2; 0.244, 0.622, pH_cumulo+PL +AN_cumulo+interval_yield+OD+RS+Feed_Vol+RS_cumulo+RQ_integ ral+Accum._Yield+AS_Vol.+emission_O2+emission_CO2+consum_O 2; 0.268, 0.622, pH_cumulo+PL+AN+interval_yield+OD+RS+RS_cu mulo+rab_integral+Accum._Yield+emission_CO2+consum_O2+gener ate_CO2; 0.268, 0.622, tmp+pH_cumulo+PL+AN_cumulo+OD+RS+RS_ cumulo+Accum._Yield+AS_Vol.+emission_CO2+consum_O2+generate _CO2; 0.256, 0.622, pH_cumulo+PL+AN+interval_yield+interval _Pdt.+OD+RS+Feed_Vol+Cell_conc._interval+Accum._Yield+AS_Vo l.+consum_O2+generate_CO2; 0.256, 0.622, tmp_cumulo+pH+pH_c umulo+PL+AN+interval_yield+OD+RS+RS_cumulo+AS_Vol.+emission _CO2+consum_O2+generate_CO2; 0.280, 0.622, pH_cumulo+PL+int erval_yield+OD+RS+RS_cumulo+RQ_integral+Accum._Yield+AS_Vol. +emission_CO2+consum_O2; 0.256, 0.622, pH+pH_cumulo+PL+AN+A N_cumulo+interval_yield+OD+RS+RS_cumulo+RQ_integral+Accum._ Yield+AS_Vol.+consum_O2; 0.244, 0.622, tmp+pH_cumulo+PL+AN+ AN_cumulo+interval_yield+OD+RS+RS_cumulo+Cell_conc._interva l+Accum._Yield+AS_Vol.+consum_O2+generate_CO2; 0.256, 0.622, tmp_cumulo+pH+pH_cumulo+PL+AN+interval_yield+OD+RS+RS_cumu lo+Accum._Yield+AS_Vol.+consum_O2+generate_CO2; 0.244, 0.62 2, tmp+pH_cumulo+PL+AN+OD+RS+Feed_Vol+RS_cumulo+Accum._Yiel d+AS_Vol.+emission_CO2+consum_O2+interval_O2+generate_CO2; 0.256, 0.622, pH+pH_cumulo+PL+AN+AN_cumulo+interval_yield+O D+RS+Feed_Vol+RS_cumulo+Accum._Yield+AS_Vol.+consum_O2; 0.2 31, 0.622, pH_cumulo+PL+AN+AN_cumulo+interval_yield+OD+RS+F eed_Vol+RS_cumulo+Cell_conc._interval+RQ_integral+Accum._Yi eld+AS_Vol.+emission_O2+consum_O2; 0.256, 0.622, tmp+pH_cum ulo+PL+AN+OD+RS+Feed_Vol+RS_cumulo+rab_integral+Accum._Yiel d+AS_Vol.+consum_O2+generate_CO2; 0.244, 0.622, pH+pH_cumul o+PL+PL_cumulo+AN+interval_yield+OD+RS+RS_cumulo+Accum._Yie ld+AS_Vol.+emission_CO2+consum_O2+generate_CO2; 0.256, 0.62 2, pH_cumulo+PL+AN+interval_yield+interval_Pdt.+OD+RS+RS_cu mulo+RQ_integral+AS_Vol.+emission_O2+emission_CO2+consum_O 2; 0.280, 0.622, pH+pH_cumulo+PL+interval_yield+OD+RS+RS_cu mulo+Accum._Yield+emission_CO2+consum_O2+generate_CO2; 0.28 0, 0.622, pH_cumulo+PL+AN+AN_cumulo+interval_yield+OD+RS+RS _cumulo+Accum._Yield+AS_Vol.+consum_O2; 0.280, 0.622, pH_cu mulo+PL+AN_cumulo+interval_yield+OD+RS+RS_cumulo+Cell_conc. _interval+emission_CO2+consum_O2+generate_CO2; 0.268, 0.622, pH_cumulo+PL+AN+interval_Pdt.+OD+RS+RS_cumulo+RQ_integral+ Accum._Yield+AS_Vol.+emission_O2+consum_O2; 0.280, 0.622, p H_cumulo+PL+AN+interval_yield+OD+RS+RS_cumulo+Accum._Yield+ AS_Vol.+emission_CO2+consum_O2; 0.256, 0.622, tmp+pH_cumulo +PL+AN+AN_cumulo+interval_yield+OD+RS+Feed_Vol+RS_cumulo+Ac cum._Yield+AS_Vol.+consum_O2; 0.244, 0.622, pH+pH_cumulo+PL +AN+OD+RS+Feed_Vol+RS_cumulo+Cell_conc._interval+Accum._Yie ld+AS_Vol.+emission_CO2+consum_O2+generate_CO2; 0.256, 0.62 2, pH+pH_cumulo+PL+AN+interval_yield+OD+RS+RS_cumulo+RQ_int egral+Accum._Yield+AS_Vol.+emission_O2+consum_O2; 0.231, 0. 622, tmp_cumulo+pH_cumulo+PL+AN+AN_cumulo+interval_yield+OD +RS+Feed_Vol+RS_cumulo+Cell_conc._interval+Accum._Yield+AS_ Vol.+consum_O2+generate_CO2; 0.256, 0.622, pH_cumulo+PL+AN_ cumulo+interval_yield+OD+RS+Feed_Vol+RS_cumulo+Cell_conc._i nterval+Accum._Yield+AS_Vol.+consum_O2+generate_CO2; 0.231, 0.622, pH+pH_cumulo+PL+AN_cumulo+interval_yield+OD+RS+Feed _Vol+RS_cumulo+rab_integral+Accum._Yield+AS_Vol.+emission_C O2+consum_O2+generate_CO2; 0.231, 0.622, pH+pH_cumulo+PL+AN +interval_yield+OD+RS+Feed_Vol+RS_cumulo+Cell_conc._interva l+Accum._Yield+AS_Vol.+consum_O2+interval_O2+generate_CO2; 0.256, 0.622, pH+PL+AN+AN_cumulo+OD+RS+Feed_Vol+RS_cumulo+C ell_conc._interval+Accum._Yield+AS_Vol.+consum_O2+generate_ CO2; 0.217, 0.622, tmp+pH_cumulo+PL+AN+AN_cumulo+interval_y ield+OD+RS+Feed_Vol+RS_cumulo+Cell_conc._interval+Accum._Yi eld+AS_Vol.+emission_CO2+consum_O2+generate_CO2; 0.256, 0.6 22, pH_cumulo+PL+AN_cumulo+interval_yield+OD+RS+RS_cumulo+A ccum._Yield+AS_Vol.+emission_O2+emission_CO2+consum_O2+gene rate_CO2; 0.256, 0.622, pH_cumulo+PL+PL_cumulo+AN+OD+RS+RS_ cumulo+Accum._Yield+AS_Vol.+emission_CO2+consum_O2+interval _O2+generate_CO2; 0.279, 0.622, pH_cumulo+PL+interval_yield +OD+RS+Feed_Vol+RS_cumulo+RQ_integral+AS_Vol.+emission_CO2+ consum_O2; 0.231, 0.622, tmp+tmp_cumulo+pH_cumulo+PL+AN_cum ulo+interval_yield+OD+RS+Feed_Vol+RS_cumulo+Accum._Yield+AS _Vol.+emission_CO2+consum_O2+generate_CO2; 0.279, 0.622, pH _cumulo+PL+AN+AN_cumulo+OD+RS+Feed_Vol+Accum._Yield+AS_Vol. +consum_O2+generate_CO2; 0.268, 0.622, tmp_cumulo+pH_cumulo +PL+interval_yield+OD+RS+RS_cumulo+AS_Vol.+emission_CO2+con sum_O2+interval_O2+generate_CO2; 0.231, 0.622, pH_cumulo+PL +AN+interval_yield+OD+RS+Feed_Vol+RS_cumulo+Cell_conc._inte rval+rab_integral+RQ_integral+Accum._Yield+AS_Vol.+emission O2+consum_O2; 0.268, 0.622, pH_cumulo+PL+AN+AN_cumulo+inte rval_yield+OD+RS+Feed_Vol+RS_cumulo+rab_integral+AS_Vol.+co nsum_O2; 0.256, 0.622, tmp_cumulo+pH_cumulo+PL+interval_yie ld+OD+RS+Feed_Vol+RS_cumulo+Accum._Yield+AS_Vol.+emission_C O2+consum_O2+generate_CO2; 0.256, 0.622, pH_cumulo+PL+AN+AN _cumulo+interval_yield+OD+RS+RS_cumulo+RQ_integral+Accum._Y ield+AS_Vol.+emission_CO2+consum_O2; 0.256, 0.622, tmp_cumu lo+pH_cumulo+PL+AN+interval_yield+OD+RS+Feed_Vol+RS_cumulo+ rab_integral+Accum._Yield+AS_Vol.+consum_O2; 0.256, 0.622, pH_cumulo+PL+AN+interval_yield+interval_Pdt.+OD+RS+RS_cumul o+rab_integral+RQ_integral+Accum._Yield+AS_Vol.+consum_O2; 0.256, 0.622, pH_cumulo+PL+AN+interval_yield+OD+RS+RS_cumul o+Cell_conc._interval+rab_integral+RQ_integral+Accum._Yield +AS_Vol.+consum_O2; 0.268, 0.622, pH_cumulo+PL+PL_cumulo+AN +OD+RS+Feed_Vol+RS_cumulo+Accum._Yield+AS_Vol.+consum_O2+ge nerate_CO2; 0.256, 0.622, pH+pH_cumulo+PL+interval_yield+OD +RS+RS_cumulo+rab_integral+Accum._Yield+AS_Vol.+emission_CO 2+consum_O2+generate_CO2; 0.230, 0.622, pH_cumulo+PL+PL_cum ulo+AN+AN_cumulo+interval_yield+OD+RS+Feed_Vol+RS_cumulo+ra b_integral+Accum._Yield+AS_Vol.+consum_O2+generate_CO2; 0.2 68, 0.622, pH_cumulo+PL+AN+AN_cumulo+interval_yield+OD+RS+R S_cumulo+AS_Vol.+emission_O2+consum_O2+generate_CO2; 0.268, 0.622, pH_cumulo+PL+AN+interval_Pdt.+OD+RS+RS_cumulo+Accum. _Yield+AS_Vol.+consum_O2+interval_O2+generate_CO2; 0.230, 0. 622, tmp+pH_cumulo+PL+AN+interval_yield+OD+RS+Feed_Vol+RS_c umulo+rab_integral+Accum._Yield+AS_Vol.+emission_O2+consum_ O2+generate_CO2; 0.256, 0.622, pH_cumulo+PL+AN+interval_yie ld+OD+RS+RS_cumulo+rab_integral+RQ_integral+Accum._Yield+AS _Vol.+emission_CO2+consum_O2; 0.243, 0.622, pH_cumulo+PL+AN +interval_yield+interval_Pdt.+OD+RS+RS_cumulo+RQ_integral+A ccum._Yield+AS_Vol.+emission_O2+emission_CO2+consum_O2; 0.2 43, 0.622, pH_cumulo+PL+PL_cumulo+AN+OD+RS+Feed_Vol+RS_cumu lo+Accum._Yield+AS_Vol.+emission_CO2+consum_O2+interval_O2+ generate_CO2; 0.243, 0.622, tmp_cumulo+pH_cumulo+PL+AN+AN_c umulo+interval_yield+OD+RS+Feed_Vol+RS_cumulo+RQ_integral+A ccum._Yield+AS_Vol.+consum_O2; 0.256, 0.622, pH+pH_cumulo+P L+interval_yield+OD+RS+Feed_Vol+RS_cumulo+rab_integral+AS_V ol.+emission_CO2+consum_O2+generate_CO2; 0.256, 0.622, pH+p H_cumulo+PL+interval_yield+OD+RS+RS_cumulo+Accum._Yield+AS_ Vol.+emission_CO2+consum_O2+interval_O2+generate_CO2; 0.290, 0.622, pH_cumulo+interval_Pdt.+OD+RS+RS_cumulo+RQ_integral +AS_Vol.+emission_O2+emission_CO2+consum_O2; 0.243, 0.622, pH_cumulo+PL+PL_cumulo+AN+interval_yield+OD+RS+RS_cumulo+ra b_integral+Accum._Yield+AS_Vol.+emission_CO2+consum_O2+gene rate_CO2; 0.230, 0.621, pH_cumulo+PL+AN+AN_cumulo+interval_ yield+OD+RS+Feed_Vol+RS_cumulo+Cell_conc._interval+Accum._Y ield+AS_Vol.+emission_O2+consum_O2+generate_CO2; 0.230, 0.6 21, pH_cumulo+PL+AN+AN_cumulo+interval_yield+OD+RS+Feed_Vol +RS_cumulo+Cell_conc._interval+rab_integral+RQ_integral+Acc um._Yield+AS_Vol.+consum_O2; 0.279, 0.621, pH_cumulo+PL+int erval_yield+OD+RS+Feed_Vol+RS_cumulo+Accum._Yield+AS_Vol.+c onsum_O2+generate_CO2; 0.243, 0.621, pH_cumulo+PL+AN_cumulo +interval_yield+OD+RS+RS_cumulo+Cell_conc._interval+rab_int egral+Accum._Yield+AS_Vol.+emission_CO2+consum_O2+generate_ CO2; 0.267, 0.621, tmp+pH_cumulo+PL+AN+interval_yield+OD+RS +Feed_Vol+RS_cumulo+AS_Vol.+consum_O2+generate_CO2; 0.243, 0.621, pH_cumulo+PL+AN+interval_yield+OD+RS+Feed_Vol+RS_cum ulo+rab_integral+RQ_integral+AS_Vol.+emission_O2+emission_C O2+consum_O2; 0.230, 0.621, tmp+pH+pH_cumulo+PL+AN+AN_cumul o+interval_yield+OD+RS+Feed_Vol+RS_cumulo+Accum._Yield+AS_V ol.+consum_O2+generate_CO2; 0.279, 0.621, pH_cumulo+PL+AN_c umulo+interval_yield+OD+RS+RS_cumulo+emission_CO2+consum_O2 +interval_O2+generate_CO2; 0.255, 0.621, pH_cumulo+PL+AN+OD +RS+RS_cumulo+Cell_conc._interval+rab_integral+Accum._Yield +AS_Vol.+consum_O2+interval_O2+generate_CO2; 0.217, 0.621, pH+pH_cumulo+PL+AN+AN_cumulo+interval_yield+OD+RS+Feed_Vol+ RS_cumulo+Cell_conc._interval+rab_integral+Accum._Yield+AS_ Vol.+consum_O2+generate_CO2; 0.267, 0.621, pH+pH_cumulo+PL+ AN+interval_Pdt.+OD+RS+RS_cumulo+RQ_integral+Accum._Yield+A S_Vol.+consum_O2; 0.279, 0.621, tmp_cumulo+pH_cumulo+PL+AN+ interval_yield+OD+RS+RS_cumulo+RQ_integral+AS_Vol.+consum_O 2; 0.243, 0.621, tmp+pH_cumulo+PL+PL_cumulo+AN+interval_yie ld+OD+RS+Feed_Vol+RS_cumulo+Accum._Yield+AS_Vol.+consum_O2+ generate_CO2; 0.255, 0.621, tmp+pH_cumulo+PL+AN+OD+RS+RS_cu mulo+Accum._Yield+AS_Vol.+emission_CO2+consum_O2+interval_O 2+generate_CO2; 0.243, 0.621, tmp_cumulo+pH_cumulo+PL+AN+OD +RS+Feed_Vol+RS_cumulo+Accum._Yield+AS_Vol.+emission_CO2+co nsum_O2+interval_O2+generate_CO2; 0.230, 0.621, tmp_cumulo+ pH_cumulo+PL+AN+interval_yield+interval_Pdt.+OD+RS+Feed_Vol +RS_cumulo+Accum._Yield+AS_Vol.+emission_CO2+consum_O2+gene rate_CO2; 0.255, 0.621, pH_cumulo+PL+AN_cumulo+interval_yie ld+OD+RS+Feed_Vol+RS_cumulo+rab_integral+RQ_integral+Accum. _Yield+AS_Vol.+consum_O2; 0.230, 0.621, tmp_cumulo+pH_cumul o+PL+AN+interval_yield+OD+RS+Feed_Vol+RS_cumulo+rab_integra l+Accum._Yield+AS_Vol.+consum_O2+interval_O2+generate_CO2; 0.267, 0.621, pH_cumulo+PL+AN_cumulo+interval_yield+OD+RS+R S_cumulo+AS_Vol.+emission_O2+emission_CO2+consum_O2+generat e_CO2; 0.255, 0.621, pH_cumulo+PL+AN+interval_yield+OD+RS+F eed_Vol+RQ_integral+Accum._Yield+AS_Vol.+emission_O2+emissi on_CO2+consum_O2; 0.243, 0.621, pH_cumulo+PL+AN+AN_cumulo+i nterval_yield+interval_Pdt.+OD+RS+Feed_Vol+rab_integral+Acc um._Yield+AS_Vol.+consum_O2+generate_CO2; 0.217, 0.621, tmp +tmp_cumulo+pH+pH_cumulo+PL+AN+interval_yield+OD+RS+Feed_Vo l+RS_cumulo+Accum._Yield+AS_Vol.+emission_CO2+consum_O2+gen erate_CO2; 0.230, 0.621, pH+pH_cumulo+PL+PL_cumulo+AN+OD+RS +Feed_Vol+RS_cumulo+Accum._Yield+AS_Vol.+emission_CO2+consu m_O2+interval_O2+generate_CO2; 0.243, 0.621, tmp_cumulo+pH_ cumulo+PL+AN+interval_yield+OD+RS+Feed_Vol+RS_cumulo+Cell_c onc._interval+AS_Vol.+emission_CO2+consum_O2+generate_CO2; 0.255, 0.621, pH_cumulo+PL+PL_cumulo+AN+interval_yield+OD+R S+RS_cumulo+Cell_conc._interval+Accum._Yield+AS_Vol.+consum _O2+generate_CO2; 0.279, 0.621, pH+pH_cumulo+PL+AN+interval _yield+OD+RS+RS_cumulo+AS_Vol.+consum_O2+generate_CO2; 0.26 7, 0.621, pH+PL+AN+interval_yield+OD+RS+Feed_Vol+RS_cumulo+ AS_Vol.+emission_CO2+consum_O2+generate_CO2; 0.255, 0.621, pH_cumulo+PL+AN+interval_yield+OD+RS+Feed_Vol+RS_cumulo+rab _integral+AS_Vol.+emission_O2+consum_O2+generate_CO2; 0.255, 0.621, pH_cumulo+PL+interval_yield+OD+RS+RS_cumulo+rab_int egral+RQ_integral+Accum._Yield+AS_Vol.+emission_O2+emission _CO2+consum_O2; 0.243, 0.621, tmp+tmp_cumulo+pH_cumulo+PL+A N+interval_yield+OD+RS+RS_cumulo+rab_integral+Accum._Yield+ AS_Vol.+consum_O2+generate_CO2; 0.217, 0.621, tmp+pH+pH_cum ulo+PL+AN+interval_yield+OD+RS+Feed_Vol+RS_cumulo+rab_integ ral+Accum.Yield+AS_Vol.+emission_CO2+consum_O2+generate_CO 2; 0.243, 0.621, pH_cumulo+PL+AN+AN_cumulo+OD+RS+Feed_Vol+R S_cumulo+Cell_conc._interval+rab_integral+Accum._Yield+AS_V ol.+consum_O2+generate_CO2; 0.255, 0.621, pH_cumulo+PL+inte rval_yield+OD+RS+Feed_Vol+RS_cumulo+rab_integral+AS_Vol.+em ission_CO2+consum_O2+interval_O2+generate_CO2; 0.230, 0.621, tmp_cumulo+pH+pH_cumulo+PL+AN+AN_cumulo+interval_yield+OD+ RS+Feed_Vol+RS_cumulo+Accum._Yield+AS_Vol.+consum_O2+genera te_CO2; 0.243, 0.621, tmp+tmp_cumulo+pH_cumulo+PL+AN+interv al_yield+OD+RS+RS_cumulo+Cell_conc._interval+Accum._Yield+A S_Vol.+consum_O2+generate_CO2; 0.255, 0.621, tmp_cumulo+pH_ cumulo+PL+AN+interval_yield+interval_Pdt.+OD+RS+Feed_Vol+Ac cum._Yield+AS_Vol.+consum_O2+generate_CO2; 0.230, 0.621, pH _cumulo+PL+AN+AN_cumulo+interval_yield+OD+RS+Feed_Vol+RS_cu mulo+rab_integral+RQ_integral+Accum._Yield+AS_Vol.+emission _CO2+consum_O2; 0.267, 0.621, tmp+pH+pH_cumulo+PL+interval_ yield+OD+RS+RS_cumulo+AS_Vol.+emission_CO2+consum_O2+genera te_CO2; 0.267, 0.621, pH_cumulo+PL+AN+interval_yield+OD+RS+ Feed_Vol+RS_cumulo+Accum._Yield+emission_CO2+consum_O2+gene rate_CO2; 0.255, 0.621, pH_cumulo+PL+AN_cumulo+interval_yie ld+OD+RS+RS_cumulo+Cell_conc._interval+rab_integral+AS_Vol. +emission_CO2+consum_O2+generate_CO2; 0.267, 0.621, pH_cumu lo+PL+AN+interval_yield+OD+RS+RS_cumulo+Cell_conc._interval +AS_Vol.+emission_O2+consum_O2+generate_CO2; 0.230, 0.621, tmp+pH_cumulo+PL+AN+AN_cumulo+interval_yield+interval_Pdt.+ OD+RS+Feed_Vol+RS_cumulo+Accum._Yield+AS_Vol.+consum_O2+gen erate_CO2; 0.242, 0.621, tmp_cumulo+pH_cumulo+PL+AN+AN_cumu lo+interval_yield+OD+RS+RS_cumulo+Cell_conc._interval+Accum. _Yield+AS_Vol.+consum_O2+generate_CO2; 0.255, 0.621, pH_cum ulo+PL+interval_yield+interval_Pdt.+OD+RS+Feed_Vol+RS_cumul o+RQ_integral+AS_Vol.+emission_O2+emission_CO2+consum_O2; 0. 255, 0.621, pH_cumulo+PL+AN+AN_cumulo+OD+RS+RS_cumulo+Accum. _Yield+AS_Vol.+emission_O2+consum_O2+interval_O2+generate_C 02; 0.230, 0.621, tmp+tmp_cumulo+pH_cumulo+PL+AN+interval_y ield+OD+RS+Feed_Vol+RS_cumulo+Cell_conc._interval+Accum._Yi eld+AS_Vol.+consum_O2+generate_CO2; 0.230, 0.621, pH_cumulo +PL+PL_cumulo+AN+interval_yield+OD+RS+Feed_Vol+RS_cumulo+ra b_integral+Accum._Yield+AS_Vol.+consum_O2+interval_O2+gener ate_CO2; 0.230, 0.621, pH_cumulo+PL+AN+AN_cumulo+interval_y ield+OD+RS+RS_cumulo+rab_integral+Accum._Yield+AS_Vol.+emis sion_CO2+consum_O2+interval_O2+generate_CO2; 0.242, 0.621, pH_cumulo+PL+PL_cumulo+AN+AN_cumulo+interval_yield+OD+RS+RS _cumulo+Accum._Yield+AS_Vol.+emission_CO2+consum_O2+generat e_CO2; 0.255, 0.621, pH_cumulo+PL+AN_cumulo+interval_yield+ OD+RS+RS_cumulo+rab_integral+Accum._Yield+AS_Vol.+emission_ O2+consum_O2+generate_CO2; 0.267, 0.621, tmp+tmp_cumulo+pH_ cumulo+PL+AN+OD+RS+RS_cumulo+Accum._Yield+AS_Vol.+consum_O2 +generate_CO2; 0.278, 0.621, pH_cumulo+PL+AN+interval_yield +OD+RS+RS_cumulo+Cell_conc._interval+emission_CO2+consum_O2 +generate_CO2; 0.278, 0.621, pH_cumulo+PL+interval_yield+in terval_Pdt.+OD+RS+RS_cumulo+RQ_integral+AS_Vol.+emission_CO 2+consum_O2; 0.229, 0.621, pH+pH_cumulo+PL+AN+AN_cumulo+OD+ RS+Feed_Vol+RS_cumulo+rab_integral+Accum._Yield+AS_Vol.+emi ssion_CO2+consum_O2+generate_CO2; 0.242, 0.621, tmp+pH_cumu lo+PL+AN+AN_cumulo+interval_yield+interval_Pdt.+OD+RS+Feed_ Vol+Accum._Yield+AS_Vol.+consum_O2+generate_CO2; 0.267, 0.6 21, tmp_cumulo+pH_cumulo+PL+AN+interval_yield+OD+RS+Feed_Vo l+RS_cumulo+AS_Vol.+consum_O2+generate_CO2; 0.278, 0.621, p H_cumulo+PL+AN+interval_yield+interval_Pdt.+OD+RS+Feed_Vol+ AS_Vol.+consum_O2+generate_CO2; 0.267, 0.621, pH+pH_cumulo+ PL+AN+OD+RS+RS_cumulo+rab_integral+Accum._Yield+AS_Vol.+con sum_O2+generate_CO2; 0.255, 0.621, tmp+pH+pH_cumulo+PL+AN+i nterval_yield+OD+RS+RS_cumulo+Accum._Yield+AS_Vol.+consum_O 2+generate_CO2; 0.289, 0.621, pH_cumulo+PL+OD+RS+RS_cumulo+ Accum._Yield+AS_Vol.+consum_O2+interval_O2+generate_CO2; 0. 255, 0.621, pH_cumulo+PL+AN_cumulo+interval_yield+OD+RS+Fee d_Vol+RS_cumulo+RQ_integral+AS_Vol.+emission_O2+emission_CO 2+consum_O2; 0.267, 0.621, pH+pH_cumulo+PL+interval_yield+O D+RS+RS_cumulo+RQ_integral+AS_Vol.+emission_O2+emission_CO2 +consum_O2; 0.267, 0.621, pH_cumulo+PL+AN+AN_cumulo+interva l_yield+OD+RS+RS_cumulo+rab_integral+AS_Vol.+consum_O2+gene rate_CO2; 0.267, 0.621, pH_cumulo+PL+AN+OD+RS+Feed_Vol+RS_c umulo+RQ_integral+Accum._Yield+AS_Vol.+emission_O2+consum_O 2; 0.255, 0.621, pH_cumulo+PL+AN+AN_cumulo+OD+RS+RS_cumulo+ Cell_conc._interval+rab_integral+Accum._Yield+AS_Vol.+consu m_O2+generate_CO2; 0.242, 0.621, pH+pH_cumulo+PL+PL_cumulo+ AN+OD+RS+Feed_Vol+RS_cumulo+Accum._Yield+AS_Vol.+consum_O2+ interval_O2+generate_CO2; 0.254, 0.621, tmp+tmp_cumulo+pH_c umulo+PL+AN+OD+RS+Feed_Vol+RS_cumulo+Accum._Yield+AS_Vol.+c onsum_O2+generate_CO2; 0.242, 0.621, pH_cumulo+PL+AN+AN_cum ulo+OD+RS+Feed_Vol+RS_cumulo+Cell_conc._interval+Accum._Yie ld+AS_Vol.+consum_O2+interval_O2+generate_CO2; 0.242, 0.621, pH+pH_cumulo+PL+AN+interval_yield+OD+RS+RS_cumulo+Cell_con c._interval+Accum._Yield+AS_Vol.+consum_O2+interval_O2+gene rate_CO2; 0.229, 0.621, pH+pH_cumulo+PL+AN+AN_cumulo+interv al_yield+OD+RS+RS_cumulo+rab_integral+Accum._Yield+AS_Vol.+ emission_CO2+consum_O2+generate_CO2; 0.242, 0.621, tmp+pH_c umulo+PL+AN+AN_cumulo+interval_yield+OD+RS+Feed_Vol+RS_cumu lo+rab_integral+Accum._Yield+AS_Vol.+consum_O2; 0.254, 0.62 1, pH+pH_cumulo+PL+AN+AN_cumulo+OD+RS+RS_cumulo+Accum._Yiel d+AS_Vol.+consum_O2+interval_O2+generate_CO2; 0.229, 0.621, tmp_cumulo+pH_cumulo+PL+AN+interval_yield+interval_Pdt.+OD +RS+Feed_Vol+RS_cumulo+rab_integral+Accum._Yield+AS_Vol.+co nsum_O2+generate_CO2; 0.242, 0.621, pH_cumulo+PL+AN+interva l_Pdt.+OD+RS+Feed_Vol+RS_cumulo+rab_integral+Accum._Yield+A S_Vol.+emission_CO2+consum_O2+generate_CO2; 0.254, 0.621, p H+pH_cumulo+PL+AN+OD+RS+Feed_Vol+RS_cumulo+Accum._Yield+AS_ Vol.+emission_O2+consum_O2+generate_CO2; 0.254, 0.621, pH_c umulo+PL+PL_cumulo+AN+AN_cumulo+interval_yield+OD+RS+RS_cum ulo+Accum._Yield+AS_Vol.+consum_O2+generate_CO2; 0.254, 0.6 21, pH_cumulo+PL+AN+interval_Pdt.+OD+RS+Feed_Vol+RS_cumulo+ rab_integral+Accum._Yield+AS_Vol.+consum_O2+generate_CO2; 0. 254, 0.621, tmp_cumulo+pH_cumulo+PL+AN+OD+RS+Feed_Vol+RS_cu mulo+rab_integral+Accum._Yield+AS_Vol.+consum_O2+generate_C 02; 0.242, 0.621, tmp+pH_cumulo+PL+AN+AN_cumulo+interval_yl eld+OD+RS+RS_cumulo+rab_integral+Accum._Yield+AS_Vol.+consu m_O2+generate_CO2; 0.266, 0.621, pH+pH_cumulo+PL+AN_cumulo+ interval_yield+OD+RS+RS_cumulo+Accum._Yield+emission_CO2+co nsum_O2+generate_CO2; 0.229, 0.621, tmp+tmp_cumulo+pH_cumul o+PL+AN+interval_yield+interval_Pdt.+OD+RS+Feed_Vol+RS_cumu lo+Accum._Yield+AS_Vol.+consum_O2+generate_CO2; 0.242, 0.62 1, tmp+pH_cumulo+PL+AN+interval_yield+OD+RS+RS_cumulo+Cell_ conc._interval+rab_integral+Accum._Yield+AS_Vol.+consum_O2+ generate_CO2; 0.229, 0.621, pH_cumulo+PL+AN+AN_cumulo+OD+RS +Feed_Vol+RS_cumulo+rab_integral+Accum._Yield+AS_Vol.+emiss ion_CO2+consum_O2+interval_O2+generate_CO2; 0.266, 0.621, t mp+pH_cumulo+PL+interval_yield+OD+RS+RS_cumulo+Cell_conc._i nterval+AS_Vol.+emission_CO2+consum_O2+generate_CO2; 0.311, 0.621, pH_cumulo+PL+interval_yield+OD+RS+emission_CO2+cons um_O2+generate_CO2; 0.242, 0.621, tmp+tmp_cumulo+pH_cumulo+ PL+AN+interval_yield+OD+RS+Feed_Vol+RS_cumulo+RQ_integral+A ccum._Yield+AS_Vol.+consum_O2; 0.278, 0.621, tmp_cumulo+pH_ cumulo+PL+AN_cumulo+OD+RS+RS_cumulo+Accum._Yield+AS_Vol.+co nsum_O2+generate_CO2; 0.242, 0.621, pH_cumulo+PL+AN+interva l_yield+OD+RS+RS_cumulo+Cell_conc._interval+rab_integral+Ac cum._Yield+AS_Vol.+emission_O2+consum_O2+generate_CO2; 0.25 4, 0.621, pH+pH_cumulo+PL+AN+AN_cumulo+OD+RS+RS_cumulo+Cell _conc._interval+Accum._Yield+AS_Vol.+consum_O2+generate_CO 2; 0.254, 0.621, pH_cumulo+PL+PL_cumulo+AN_cumulo+interval_ yield+OD+RS+RS_cumulo+Accum._Yield+AS_Vol.+emission_CO2+con sum_O2+generate_CO2; 0.266, 0.621, pH_cumulo+PL+AN_cumulo+i nterval_yield+OD+RS+Feed_Vol+RS_cumulo+rab_integral+emissio n_CO2+consum_O2+generate_CO2; 0.266, 0.621, pH+pH_cumulo+PL +AN+interval_yield+OD+RS+Feed_Vol+RS_cumulo+RQ_integral+AS_ Vol.+consum_O2; 0.254, 0.621, pH+pH_cumulo+PL+interval_Pdt. +OD+RS+RS_cumulo+RQ_integral+Accum._Yield+AS_Vol.+emission_ O2+emission_CO2+consum_O2; 0.254, 0.621, pH_cumulo+PL+AN+in terval_yield+OD+RS+Feed_Vol+Accum._Yield+AS_Vol.+emission_C O2+consum_O2+interval_O2+generate_CO2; 0.254, 0.621, tmp+pH _cumulo+PL+AN_cumulo+interval_yield+OD+RS+RS_cumulo+rab_int egral+AS_Vol.+emission_CO2+consum_O2+generate_CO2; 0.242, 0. 621, pH_cumulo+PL+PL_cumulo+AN+interval_yield+OD+RS+Feed_Vo l+RS_cumulo+Accum._Yield+AS_Vol.+emission_O2+consum_O2+gene rate_CO2; 0.242, 0.621, pH_cumulo+PL+AN+AN_cumulo+interval_ yield+OD+RS+RS_cumulo+Cell_conc._interval+Accum._Yield+AS_V ol.+emission_O2+consum_O2+generate_CO2; 0.266, 0.621, pH_cu mulo+PL+PL_cumulo+AN+OD+RS+RS_cumulo+Accum._Yield+AS_Vol.+e mission_CO2+consum_O2+generate_CO2; 0.289, 0.621, pH_cumulo +PL+AN+AN_cumulo+interval_yield+OD+RS+RS_cumulo+AS_Vol.+con sum_O2; 0.278, 0.621, pH_cumulo+PL+AN_cumulo+interval_yield +OD+RS+RS_cumulo+AS_Vol.+emission_O2+consum_O2+generate_CO 2; 0.278, 0.621, pH_cumulo+PL+AN_cumulo+OD+RS+RS_cumulo+rab integral+Accum._Yield+AS_Vol.+consum_O2+generate_CO2; 0.25 4, 0.621, pH_cumulo+PL+AN_cumulo+interval_yield+OD+RS+Feed_ Vol+RS_cumulo+RQ_integral+Accum._Yield+AS_Vol.+emission_O2+ consum_O2; 0.242, 0.621, pH+PL+PL_cumulo+AN+interval_yield+ OD+RS+Feed_Vol+RS_cumulo+Accum._Yield+AS_Vol.+emission_CO2+ consum_O2+generate_CO2; 0.266, 0.621, pH_cumulo+PL+AN+AN_cu mulo+interval_yield+OD+RS+RS_cumulo+RQ_integral+AS_Vol.+emi ssion_CO2+consum_O2; 0.266, 0.620, pH_cumulo+PL+AN_cumulo+i nterval_yield+OD+RS+RS_cumulo+Accum._Yield+emission_CO2+con sum_O2+interval_O2+generate_CO2; 0.254, 0.620, pH_cumulo+PL +interval_Pdt.+OD+RS+RS_cumulo+rab_integral+RQ_integral+Acc um._Yield+AS_Vol.+emission_O2+emission_CO2+consum_O2; 0.266, 0.620, pH_cumulo+PL+AN+interval_yield+interval_Pdt.+OD+RS+ RS_cumulo+RQ_integral+AS_Vol.+emission_CO2+consum_O2; 0.254, 0.620, pH_cumulo+PL+interval_yield+OD+RS+RS_cumulo+Cell_co nc._interval+Accum._Yield+AS_Vol.+emission_CO2+consum_O2+in terval_O2+generate_CO2; 0.266, 0.620, pH+PL+AN+AN_cumulo+in terval_yield+OD+RS+RS_cumulo+Accum._Yield+AS_Vol.+consum_O2 +generate_CO2; 0.229, 0.620, tmp+pH_cumulo+PL+AN+AN_cumulo+ interval_yield+OD+RS+Feed_Vol+RS_cumulo+Accum._Yield+AS_Vol. +consum_O2+interval_O2+generate_CO2; 0.229, 0.620, pH+pH_cu mulo+PL+AN+AN_cumulo+interval_yield+interval_Pdt.+OD+RS+Fee d_Vol+RS_cumulo+Accum._Yield+AS_Vol.+consum_O2+generate_CO 2; 0.229, 0.620, pH_cumulo+PL+AN+AN_cumulo+interval_yield+O D+RS+RS_cumulo+Cell_conc._interval+rab_integral+Accum._Yiel d+AS_Vol.+emission_CO2+consum_O2+generate_CO2; 0.277, 0.620, pH_cumulo+PL+AN+interval_yield+OD+RS+Feed_Vol+rab_integral +Accum._Yield+AS_Vol.+consum_O2; 0.228, 0.620, pH+pH_cumulo +PL+AN+interval_yield+OD+RS+Feed_Vol+RS_cumulo+Accum._Yield +AS_Vol.+emission_O2+consum_O2+interval_O2+generate_CO2; 0. 241, 0.620, pH_cumulo+PL+interval_yield+OD+RS+Feed_Vol+RS_c umulo+rab_integral+RQ_integral+Accum._Yield+AS_Vol.+emissio n_O2+emission_CO2+consum_O2; 0.254, 0.620, pH_cumulo+PL+PL_ cumulo+AN+AN_cumulo+interval_yield+OD+RS+RS_cumulo+RQ_integ ral+Accum._Yield+AS_Vol.+consum_O2; 0.254, 0.620, pH_cumulo +PL+AN+AN_cumulo+interval_yield+OD+RS+RS_cumulo+RQ_integral +AS_Vol.+emission_O2+emission_CO2+consum_O2; 0.228, 0.620, tmp_cumulo+pH_cumulo+PL+AN+AN_cumulo+interval_yield+OD+RS+F eed_Vol+RS_cumulo+Accum._Yield+AS_Vol.+consum_O2+interval_O 2+generate_CO2; 0.266, 0.620, tmp_cumulo+pH_cumulo+PL+inter val_yield+OD+RS+RS_cumulo+rab_integral+AS_Vol.+emission_CO2 +consum_O2+generate_CO2; 0.254, 0.620, pH_cumulo+PL+AN+inte rval_Pdt.+OD+RS+RS_cumulo+rab_integral+Accum._Yield+AS_Vol. +emission_CO2+consum_O2+generate_CO2; 0.266, 0.620, pH_cumu lo+PL+AN+interval_yield+OD+RS+Feed_Vol+Cell_conc._interval+ Accum._Yield+AS_Vol.+consum_O2+generate_CO2; 0.254, 0.620, pH_cumulo+PL+AN+AN_cumulo+interval_yield+OD+RS+Feed_Vol+RS_ cumulo+rab_integral+RQ_integral+AS_Vol.+consum_O2; 0.241, 0. 620, pH+pH_cumulo+PL+AN+interval_yield+OD+RS+Feed_Vol+RS_cu mulo+AS_Vol.+emission_CO2+consum_O2+interval_O2+generate_CO 2; 0.277, 0.620, pH_cumulo+PL+AN_cumulo+OD+RS+RS_cumulo+Acc um._Yield+AS_Vol.+emission_O2+consum_O2+generate_CO2; 0.254, 0.620, pH+pH_cumulo+PL+AN+OD+RS+RS_cumulo+Cell_conc._inter val+Accum._Yield+AS_Vol.+consum_O2+interval_O2+generate_CO 2; 0.254, 0.620, tmp+pH_cumulo+PL+AN+interval_yield+OD+RS+F eed_Vol+Accum._Yield+AS_Vol.+emission_CO2+consum_O2+generat e_CO2; 0.266, 0.620, pH_cumulo+PL+AN+interval_yield+OD+RS+R S_cumulo+AS_Vol.+emission_O2+consum_O2+interval_O2+generate _CO2; 0.266, 0.620, pH_cumulo+PL+AN_cumulo+interval_yield+i nterval_Pdt.+OD+RS+RS_cumulo+AS_Vol.+emission_CO2+consum_O2 +generate_CO2; 0.254, 0.620, pH_cumulo+PL+AN+AN_cumulo+inte rval_yield+OD+RS+RS_cumulo+Cell_conc._interval+RQ_integral+ Accum._Yield+AS_Vol.+consum_O2; 0.254, 0.620, pH_cumulo+PL+ AN_cumulo+OD+RS+RS_cumulo+rab_integral+Accum._Yield+AS_Vol. +emission_CO2+consum_O2+interval_O2+generate_CO2; 0.254, 0. 620, pH_cumulo+PL+AN+AN_cumulo+interval_Pdt.+OD+RS+Feed_Vol +RS_cumulo+Accum._Yield+AS_Vol.+consum_O2+generate_CO2; 0.2 66, 0.620, pH_cumulo+PL+interval_yield+OD+RS+RS_cumulo+Accu m._Yield+AS_Vol.+emission_O2+consum_O2+interval_O2+generate _CO2; 0.241, 0.620, tmp+tmp_cumulo+pH_cumulo+PL+AN+interval _yield+OD+RS+RS_cumulo+Accum._Yield+AS_Vol.+consum_O2+inter val_O2+generate_CO2; 0.253, 0.620, pH_cumulo+PL+AN+interval _yield+OD+RS+RS_cumulo+rab_integral+RQ_integral+AS_Vol.+emi ssion_O2+emission_CO2+consum_O2; 0.215, 0.620, pH_cumulo+PL +AN+AN_cumulo+interval_yield+OD+RS+Feed_Vol+RS_cumulo+Cell_ conc._interval+Accum._Yield+AS_Vol.+emission_CO2+consum_O2+ interval_O2+generate_CO2; 0.253, 0.620, tmp_cumulo+pH_cumul o+PL+AN+AN_cumulo+OD+RS+RS_cumulo+Accum._Yield+AS_Vol.+cons um_O2+interval_O2+generate_CO2; 0.265, 0.620, pH+pH_cumulo+ PL+AN+interval_yield+OD+RS+RS_cumulo+rab_integral+RQ_integr al+AS_Vol.+consum_O2; 0.241, 0.620, tmp+pH_cumulo+PL+AN_cum ulo+interval_yield+OD+RS+RS_cumulo+rab_integral+Accum._Yiel d+AS_Vol.+emission_CO2+consum_O2+generate_CO2; 0.253, 0.620, tmp_cumulo+pH_cumulo+PL+AN_cumulo+OD+RS+Feed_Vol+RS_cumulo +Accum._Yield+AS_Vol.+emission_CO2+consum_O2+generate_CO2; 0.241, 0.620, pH+PL+AN+interval_yield+interval_Pdt.+OD+RS+F eed_Vol+RS_cumulo+Accum._Yield+AS_Vol.+emission_CO2+consum_ O2+generate_CO2; 0.265, 0.620, pH_cumulo+PL+PL_cumulo+AN_cu mulo+interval_yield+OD+RS+RS_cumulo+AS_Vol.+emission_CO2+co nsum_O2+generate_CO2; 0.241, 0.620, tmp_cumulo+pH_cumulo+PL +AN+AN_cumulo+interval_yield+OD+RS+RS_cumulo+rab_integral+A ccum._Yield+AS_Vol.+consum_O2+generate_CO2; 0.265, 0.620, t mp_cumulo+pH+pH_cumulo+PL+interval_yield+OD+RS+RS_cumulo+AS _Vol.+emission_CO2+consum_O2+generate_CO2; 0.241, 0.620, pH cumulo+PL+AN+AN_cumulo+OD+RS+Feed_Vol+RS_cumulo+Accum._Yie ld+AS_Vol.+emission_O2+consum_O2+interval_O2+generate_CO2; 0.215, 0.620, pH_cumulo+PL+AN+interval_yield+OD+RS+Feed_Vol +RS_cumulo+Cell_conc._interval+rab_integral+Accum._Yield+AS _Vol.+emission_CO2+consum_O2+interval_O2+generate_CO2; 0.25 3, 0.620, pH+PL+AN+AN_cumulo+interval_yield+interval_Pdt.+O D+RS+Feed_Vol+Accum._Yield+AS_Vol.+consum_O2+generate_CO2; 0.265, 0.620, pH_cumulo+PL+PL_cumulo+AN+interval_yield+OD+R S+Feed_Vol+RS_cumulo+Accum._Yield+AS_Vol.+consum_O2; 0.241, 0.620, pH_cumulo+PL+AN+AN_cumulo+OD+RS+RS_cumulo+rab_integ ral+Accum._Yield+AS_Vol.+emission_CO2+consum_O2+interval_O2 +generate_CO2; 0.253, 0.620, pH+pH_cumulo+PL+AN+OD+RS+RS_cu mulo+rab_integral+Accum._Yield+AS_Vol.+consum_O2+interval_O 2+generate_CO2; 0.253, 0.620, pH_cumulo+PL+AN+AN_cumulo+int erval_yield+OD+RS+Feed_Vol+RS_cumulo+Cell_conc._interval+Ac cum._Yield+AS_Vol.+consum_O2; 0.277, 0.620, pH+pH_cumulo+PL +interval_yield+OD+RS+RS_cumulo+rab_integral+emission_CO2+c onsum_O2+generate_CO2; 0.265, 0.620, pH_cumulo+PL+AN+AN_cum ulo+interval_Pdt.+OD+RS+RS_cumulo+Accum._Yield+AS_Vol.+cons um_O2+generate_CO2; 0.288, 0.620, pH_cumulo+PL+interval_yie ld+OD+RS+RS_cumulo+Cell_conc._interval+RQ_integral+AS_Vol.+ consum_O2; 0.265, 0.620, pH_cumulo+PL+interval_yield+OD+RS+ RS_cumulo+rab_integral+Accum._Yield+AS_Vol.+consum_O2+inter val_O2+generate_CO2; 0.265, 0.620, pH_cumulo+PL+AN+interval _Pdt.+OD+RS+RS_cumulo+Cell_conc._interval+Accum._Yield+AS_V ol.+consum_O2+generate_CO2; 0.228, 0.620, pH+pH_cumulo+PL+P L_cumulo+AN+interval_yield+OD+RS+Feed_Vol+RS_cumulo+Accum._ Yield+AS_Vol.+consum_O2+interval_O2+generate_CO2; 0.228, 0. 620, pH+pH_cumulo+PL+AN+interval_yield+OD+RS+Feed_Vol+RS_cu mulo+Cell_conc._interval+Accum._Yield+AS_Vol.+emission_O2+c onsum_O2+generate_CO2; 0.288, 0.620, pH_cumulo+PL+AN+OD+RS+ Feed_Vol+Accum._Yield+AS_Vol.+consum_O2+generate_CO2; 0.241, 0.620, pH+PL+AN+interval_yield+OD+RS+Feed_Vol+RS_cumulo+Ce ll_conc._interval+rab_integral+Accum._Yield+AS_Vol.+consum_ O2+generate_CO2; 0.277, 0.620, tmp+pH_cumulo+PL+interval_yi eld+OD+RS+RS_cumulo+Accum._Yield+emission_CO2+consum_O2+gen erate_CO2; 0.228, 0.620, pH_cumulo+PL+AN+interval_yield+OD+ RS+Feed_Vol+RS_cumulo+Cell_conc._interval+Accum._Yield+AS_V ol.+emission_O2+emission_CO2+consum_O2+generate_CO2; 0.214, 0.620, pH+pH_cumulo+PL+PL_cumulo+AN+interval_yield+OD+RS+F eed_Vol+RS_cumulo+Accum._Yield+AS_Vol.+emission_CO2+consum_ O2+interval_O2+generate_CO2; 0.228, 0.620, pH_cumulo+PL+AN+ AN_cumulo+interval_yield+interval_Pdt.+OD+RS+Feed_Vol+RS_cu mulo+RQ_integral+Accum._Yield+AS_Vol.+emission_O2+consum_O 2; 0.288, 0.620, pH_cumulo+PL+interval_yield+OD+RS+RS_cumul o+rab_integral+AS_Vol.+consum_O2+generate_CO2; 0.265, 0.620, pH_cumulo+PL+AN+interval_yield+OD+RS+RS_cumulo+rab_integra l+RQ_integral+AS_Vol.+emission_CO2+consum_O2; 0.253, 0.620, tmp+tmp_cumulo+pH_cumulo+PL+AN_cumulo+OD+RS+RS_cumulo+Accu m._Yield+AS_Vol.+emission_CO2+consum_O2+generate_CO2; 0.253, 0.620, pH_cumulo+PL+AN_cumulo+interval_Pdt.+OD+RS+Feed_Vol +RS_cumulo+Accum._Yield+AS_Vol.+emission_CO2+consum_O2+gene rate_CO2; 0.253, 0.620, pH_cumulo+PL+AN_cumulo+interval_yie ld+OD+RS+RS_cumulo+RQ_integral+Accum._Yield+AS_Vol.+emissio n_O2+emission_CO2+consum_O2; 0.277, 0.620, pH_cumulo+PL+AN_ cumulo+interval_yield+OD+RS+RS_cumulo+RQ_integral+AS_Vol.+e mission_O2+consum_O2; 0.214, 0.620, tmp+tmp_cumulo+pH_cumul o+PL+AN+AN_cumulo+interval_yield+OD+RS+Feed_Vol+RS_cumulo+r ab_integral+Accum._Yield+AS_Vol.+consum_O2+generate_CO2; 0. 277, 0.620, pH+pH_cumulo+PL+interval_yield+OD+RS+Feed_Vol+R S_cumulo+emission_CO2+consum_O2+generate_CO2; 0.228, 0.620, tmp+pH_cumulo+PL+AN+interval_yield+OD+RS+Feed_Vol+RS_cumul o+Accum._Yield+AS_Vol.+emission_O2+emission_CO2+consum_O2+g enerate_CO2; 0.265, 0.620, pH_cumulo+PL+AN+interval_yield+O D+RS+Feed_Vol+RS_cumulo+Cell_conc._interval+RQ_integral+AS_ Vol.+consum_O2; 0.265, 0.620, pH+pH_cumulo+PL+AN_cumulo+int erval_yield+OD+RS+RS_cumulo+RQ_integral+Accum._Yield+AS_Vol. +consum_O2; 0.265, 0.620, pH+PL+AN+AN_cumulo+OD+RS+Feed_Vol +RS_cumulo+Accum._Yield+AS_Vol.+consum_O2+generate_CO2; 0.2 40, 0.620, pH+pH_cumulo+PL+AN_cumulo+interval_yield+OD+RS+R S_cumulo+Cell_conc._interval+Accum._Yield+AS_Vol.+emission_ CO2+consum_O2+generate_CO2; 0.240, 0.620, tmp+tmp_cumulo+pH _cumulo+PL+AN+OD+RS+Feed_Vol+RS_cumulo+Accum._Yield+AS_Vol. +emission_CO2+consum_O2+generate_CO2; 0.240, 0.620, pH+pH_c umulo+PL+AN+interval_Pdt.+OD+RS+Feed_Vol+RS_cumulo+Accum._Y ield+AS_Vol.+emission_CO2+consum_O2+generate_CO2; 0.253, 0. 620, pH_cumulo+PL+AN+OD+RS+Feed_Vol+RS_cumulo+Accum._Yield+ AS_Vol.+emission_O2+emission_CO2+consum_O2+generate_CO2; 0. 240, 0.620, pH+pH_cumulo+PL+AN+OD+RS+Feed_Vol+RS_cumulo+Cel l_conc._interval+Accum._Yield+AS_Vol.+consum_O2+interval_O2 +generate_CO2; 0.240, 0.620, pH_cumulo+PL+AN+AN_cumulo+inte rval_yield+OD+RS+Feed_Vol+RS_cumulo+Cell_conc._interval+rab _integral+Accum._Yield+AS_Vol.+consum_O2; 0.253, 0.620, tmp +tmp_cumulo+pH_cumulo+PL+interval_yield+OD+RS+Feed_Vol+RS_c umulo+AS_Vol.+emission_CO2+consum_O2+generate_CO2; 0.253, 0. 620, pH_cumulo+PL+AN+interval_yield+OD+RS+Feed_Vol+RS_cumul o+RQ_integral+Accum._Yield+AS_Vol.+consum_O2+interval_O2; 0. 277, 0.620, pH_cumulo+PL+interval_yield+OD+RS+RS_cumulo+Cel l_conc._interval+Accum._Yield+AS_Vol.+consum_O2+generate_CO 2; 0.277, 0.620, pH_cumulo+PL+AN_cumulo+interval_yield+OD+R S+Feed_Vol+RS_cumulo+RQ_integral+AS_Vol.+consum_O2; 0.240, 0.620, tmp+pH_cumulo+PL+AN+interval_yield+OD+RS+Feed_Vol+RS _cumulo+RQ_integral+Accum._Yield+AS_Vol.+emission_CO2+consu m_O2; 0.227, 0.620, pH+pH_cumulo+PL+AN+AN_cumulo+OD+RS+Feed _Vol+RS_cumulo+Cell_conc._interval+Accum._Yield+AS_Vol.+emi ssion_CO2+consum_O2+generate_CO2

### [208. Linear Model that Predicts Arginine Product ion Amount in Interval 8]

0.478, 0.783, pH_cumulo+PL+PL_cumulo+AN+AN_cumulo+interval_ yield+OD+Cell_conc._interval+rab_integral+RQ_integral+Arg_c onc.+emission_O2+consum_O2+interval_CO2; 0.477, 0.783, pH_c umulo+PL_cumulo+AN+AN_cumulo+interval_yield+OD+Cell_conc._i nterval+rab_integral+RQ_integral+Arg_conc.+emission_O2+cons um_O2+generate_CO2+interval_CO2; 0.459, 0.781, pH_cumulo+PL +PL_cumulo+AN+AN_cumulo+interval_yield+OD+Cell_conc._interv al+rab_integral+RQ_integral+Arg_conc.+emission_O2+consum_O2 +generate_CO2+interval_CO2; 0.472, 0.780, pH_cumulo+PL+PL_c umulo+AN+AN_cumulo+interval_yield+OD+Feed_Vol+Cell_conc._in terval+rab_integral+RQ_integral+Arg_conc.+emission_O2+consu m_O2; 0.485, 0.780, pH_cumulo+PL_cumulo+AN+AN_cumulo+interv al_yield+OD+Cell_conc._interval+rab_integral+RQ_integral+Ar g_conc.+emission_O2+consum_O2+interval_CO2; 0.472, 0.780, p H_cumulo+PL_cumulo+AN+AN_cumulo+interval_yield+OD+Feed_Vol+ Cell_conc._interval+rab_integral+RQ_integral+Arg_conc.+emis sion_O2+consum_O2+interval_O2; 0.484, 0.780, pH_cumulo+PL_c umulo+AN+AN_cumulo+interval_yield+OD+Feed_Vol+Cell_conc._in terval+rab_integral+RQ_integral+Arg_conc.+emission_O2+consu m_O2; 0.457, 0.780, pH_cumulo+PL+PL_cumulo+AN+AN_cumulo+int erval_yield+OD+RS_cumulo+Cell_conc._interval+rab_integral+R Q_integral+Arg_conc.+emission_O2+consum_O2+interval_CO2; 0. 456, 0.779, pH+pH_cumulo+PL+PL_cumulo+AN+AN_cumulo+interval _yield+OD+Cell_conc._interval+rab_integral+RQ_integral+Arg_ conc.+emission_O2+consum_O2+interval_CO2; 0.482, 0.779, pH_ cumulo+PL+AN+AN_cumulo+interval_yield+OD+Feed_Vol+Cell_conc. _interval+rab_integral+RQ_integral+Arg_conc.+emission_O2+co nsum_O2; 0.455, 0.779, pH+pH_cumulo+PL_cumulo+AN+AN_cumulo+ interval_yield+OD+Cell_conc._interval+rab_integral+RQ_integ ral+Arg_conc.+emission_O2+consum_O2+generate_CO2+interval_C O2; 0.468, 0.779, pH_cumulo+PL_cumulo+AN+AN_cumulo+interval _yield+OD+Cell_conc._interval+rab_integral+RQ_integral+Arg_ conc.+emission_O2+interval_O2+generate_CO2+interval_CO2; 0. 454, 0.778, pH_cumulo+PL+PL_cumulo+AN+AN_cumulo+interval_yi eld+OD+Cell_conc._interval+rab_integral+RQ_integral+Arg_con c.+emission_O2+consum_O2+interval_O2+interval_CO2; 0.454, 0. 778, pH_cumulo+PL+PL_cumulo+AN+AN_cumulo+interval_yield+OD+ Feed_Vol+Cell_conc._interval+rab_integral+RQ_integral+Arg_c onc.+emission_O2+consum_O2+interval_O2; 0.493, 0.778, pH_cu mulo+AN+AN_cumulo+interval_yield+OD+Feed_Vol+Cell_conc._int erval+rab_integral+RQ_integral+Arg_conc.+emission_O2+consum _O2; 0.453, 0.778, pH_cumulo+PL_cumulo+AN+AN_cumulo+interva l_yield+OD+Feed_Vol+Cell_conc._interval+rab_integral+RQ_int egral+Arg_conc.+emission_O2+consum_O2+generate_CO2+interval _CO2; 0.466, 0.778, pH_cumulo+PL_cumulo+AN+AN_cumulo+interv al_yield+OD+Cell_conc._interval+rab_integral+RQ_integral+Ar g_conc.+emission_O2+consum_O2+interval_O2+interval_CO2; 0.4 91, 0.777, pH_cumulo+PL_cumulo+AN+AN_cumulo+OD+Feed_Vol+Cel l_conc._interval+rab_integral+RQ_integral+Arg_conc.+emissio n_O2+consum_O2; 0.451, 0.777, pH+pH_cumulo+PL_cumulo+AN+AN_ cumulo+interval_yield+OD+Feed_Vol+Cell_conc._interval+rab_i ntegral+RQ_integral+Arg_conc.+emission_O2+consum_O2+interva l_O2; 0.502, 0.777, pH_cumulo+AN+AN_cumulo+OD+Feed_Vol+Cell _conc._interval+rab_integral+RQ_integral+Arg_conc.+emission _O2+consum_O2; 0.524, 0.777, pH_cumulo+AN+AN_cumulo+OD+Feed _Vol+Cell_conc._interval+Arg_conc.+emission_CO2+consum_O2; 0.450, 0.777, pH_cumulo+PL+PL_cumulo+AN+AN_cumulo+interval_ yield+OD+Feed_Vol+Cell_conc._interval+rab_integral+RQ_integ ral+Arg_conc.+emission_O2+consum_O2+interval_CO2; 0.463, 0. 776, pH+pH_cumulo+PL_cumulo+AN+AN_cumulo+interval_yield+OD+ Cell_conc._interval+rab_integral+RQ_integral+Arg_conc.+emis sion_O2+consum_O2+interval_CO2; 0.463, 0.776, pH+pH_cumulo+ PL_cumulo+AN+AN_cumulo+interval_yield+OD+Feed_Vol+Cell_conc. _interval+rab_integral+RQ_integral+Arg_conc.+emission_O2+co nsum_O2; 0.462, 0.776, pH_cumulo+PL_cumulo+AN+AN_cumulo+int erval_yield+OD+Feed_Vol+Cell_conc._interval+rab_integral+RQ _integral+Arg_conc.+emission_O2+consum_O2+interval_CO2; 0.4 48, 0.776, pH+pH_cumulo+PL+PL_cumulo+AN+AN_cumulo+interval_ yield+OD+Feed_Vol+Cell_conc._interval+rab_integral+RQ_integ ral+Arg_conc.+emission_O2+consum_O2; 0.434, 0.776, pH+pH_cu mulo+PL+PL_cumulo+AN+AN_cumulo+interval_yield+OD+Cell_conc. _interval+rab_integral+RQ_integral+Arg_conc.+emission_O2+co nsum_O2+generate_CO2+interval_CO2; 0.462, 0.776, pH_cumulo+ PL_cumulo+AN+AN_cumulo+interval_yield+OD+Feed_Vol+Cell_conc. _interval+rab_integral+RQ_integral+Arg_conc.+emission_O2+co nsum_O2+generate_CO2; 0.510, 0.775, pH_cumulo+PL+AN+AN_cumu lo+OD+Feed_Vol+Cell_conc._interval+Arg_conc.+emission_CO2+c onsum_O2; 0.475, 0.775, pH+pH_cumulo+AN+AN_cumulo+interval_ yield+OD+Feed_Vol+Cell_conc._interval+rab_integral+RQ_integ ral+Arg_conc.+emission_O2+consum_O2; 0.486, 0.775, pH_cumul o+PL+AN+AN_cumulo+OD+Feed_Vol+Cell_conc._interval+rab_integ ral+RQ_integral+Arg_conc.+emission_O2+consum_O2; 0.474, 0.7 75, pH_cumulo+PL+PL_cumulo+AN+AN_cumulo+OD+Feed_Vol+Cell_co nc._interval+rab_integral+RQ_integral+Arg_conc.+emission_O2 +consum_O2; 0.486, 0.775, pH+pH_cumulo+AN+AN_cumulo+OD+Feed _Vol+Cell_conc._interval+rab_integral+RQ_integral+Arg_conc. +emission_O2+consum_O2; 0.432, 0.775, pH+pH_cumulo+PL+PL_cu mulo+AN+AN_cumulo+interval_yield+OD+RS_cumulo+Cell_conc._in terval+rab_integral+RQ_integral+Arg_conc.+emission_O2+consu m_O2+interval_CO2; 0.460, 0.775, pH+pH_cumulo+PL+AN+AN_cumu lo+interval_yield+OD+Feed_Vol+Cell_conc._interval+rab_integ ral+RQ_integral+Arg_conc.+emission_O2+consum_O2; 0.446, 0.7 75, tmp_cumulo+pH_cumulo+PL_cumulo+AN+AN_cumulo+interval_yi eld+OD+Cell_conc._interval+rab_integral+RQ_integral+Arg_con c.+emission_O2+consum_O2+generate_CO2+interval_CO2; 0.446, 0.775, pH_cumulo+PL+PL_cumulo+AN+AN_cumulo+interval_yield+O D+Cell_conc._interval+rab_integral+RQ_integral+Arg_conc.+em ission_O2+interval_O2+generate_CO2+interval_CO2; 0.460, 0.7 75, pH_cumulo+PL+AN+AN_cumulo+interval_yield+OD+Feed_Vol+RS _cumulo+Cell_conc._interval+rab_integral+RQ_integral+Arg_co nc.+emission_O2+consum_O2; 0.446, 0.775, pH+pH_cumulo+PL_cu mulo+AN+AN_cumulo+interval_yield+OD+Cell_conc._interval+rab _integral+RQ_integral+Arg_conc.+emission_O2+interval_O2+gen erate_CO2+interval_CO2; 0.473, 0.774, pH+pH_cumulo+PL_cumul o+AN+AN_cumulo+OD+Feed_Vol+Cell_conc._interval+rab_integral +RQ_integral+Arg_conc.+emission_O2+consum_O2; 0.530, 0.774, pH_cumulo+AN_cumulo+OD+Feed_Vol+Cell_conc._interval+Arg_co nc.+emission_CO2+consum_O2; 0.445, 0.774, pH_cumulo+PL_cumu lo+AN+AN_cumulo+interval_yield+OD+Feed_Vol+Cell_conc._inter val+rab_integral+RQ_integral+Arg_conc.+emission_O2+consum_O 2+interval_O2+interval_CO2; 0.430, 0.774, pH_cumulo+PL+PL_c umulo+AN+AN_cumulo+interval_yield+OD+Feed_Vol+Cell_conc._in terval+rab_integral+RQ_integral+Arg_conc.+emission_O2+consu m_O2+generate_CO2+interval_CO2; 0.430, 0.774, pH+pH_cumulo+ PL_cumulo+AN+AN_cumulo+interval_yield+OD+Feed_Vol+Cell_conc. _interval+rab_integral+RQ_integral+Arg_conc.+emission_O2+co nsum_O2+generate_CO2+interval_CO2; 0.458, 0.774, tmp_cumulo +pH_cumulo+PL_cumulo+AN+AN_cumulo+interval_yield+OD+Cell_co nc._interval+rab_integral+RQ_integral+Arg_conc.+emission_O2 +consum_O2+interval_CO2; 0.444, 0.774, tmp_cumulo+pH_cumulo +PL+PL_cumulo+AN+AN_cumulo+interval_yield+OD+Cell_conc._int erval+rab_integral+RQ_integral+Arg_conc.+emission_O2+consum _O2+interval_CO2; 0.430, 0.774, pH+pH_cumulo+PL+PL_cumulo+A N+AN_cumulo+interval_yield+OD+Cell_conc._interval+rab_integ ral+RQ_integral+Arg_conc.+emission_O2+consum_O2+interval_O2 +interval_CO2; 0.458, 0.774, pH_cumulo+PL+AN+AN_cumulo+inte rval_yield+OD+RS_cumulo+Cell_conc._interval+rab_integral+RQ _integral+Arg_conc.+emission_O2+consum_O2+interval_CO2; 0.4 44, 0.774, tmp+pH_cumulo+PL+PL_cumulo+AN+AN_cumulo+interval _yield+OD+Cell_conc._interval+rab_integral+RQ_integral+Arg_ conc.+emission_O2+consum_O2+interval_CO2; 0.507, 0.774, pH_ cumulo+AN+AN_cumulo+OD+Feed_Vol+Cell_conc._interval+RQ_inte gral+Arg_conc.+emission_CO2+consum_O2; 0.429, 0.773, pH_cum ulo+PL+PL_cumulo+AN+AN_cumulo+interval_yield+OD+RS_cumulo+C ell_conc._interval+rab_integral+RQ_integral+Arg_conc.+emiss ion_O2+consum_O2+generate_CO2+interval_CO2; 0.443, 0.773, p H_cumulo+PL_cumulo+AN+AN_cumulo+interval_yield+OD+Cell_conc. _interval+rab_integral+RQ_integral+Arg_conc.+emission_O2+co nsum_O2+interval_O2+generate_CO2+interval_CO2; 0.507, 0.773, pH+pH_cumulo+AN+AN_cumulo+OD+Feed_Vol+Cell_conc._interval+ Arg_conc.+emission_CO2+consum_O2; 0.443, 0.773, pH_cumulo+P L+PL_cumulo+AN+AN_cumulo+interval_yield+OD+Feed_Vol+RS_cumu lo+Cell_conc._interval+rab_integral+RQ_integral+Arg_conc.+e mission_O2+consum_O2; 0.443, 0.773, pH+pH_cumulo+PL_cumulo+ AN+AN_cumulo+interval_yield+OD+Cell_conc._interval+rab_inte gral+RQ_integral+Arg_conc.+emission_O2+consum_O2+interval_O 2+interval_CO2; 0.470, 0.773, pH_cumulo+AN+AN_cumulo+interv al_yield+OD+Feed_Vol+Cell_conc._interval+rab_integral+RQ_in tegral+Arg_conc.+emission_O2+emission_CO2+consum_O2; 0.428, 0.773, pH+pH_cumulo+PL+PL_cumulo+AN+AN_cumulo+interval_yie ld+OD+Feed_Vol+Cell_conc._interval+rab_integral+RQ_integral +Arg_conc.+emission_O2+consum_O2+interval_O2; 0.443, 0.773, pH_cumulo+PL+PL_cumulo+AN+AN_cumulo+interval_yield+OD+Feed _Vol+Cell_conc._interval+rab_integral+RQ_integral+Arg_conc. +emission_O2+consum_O2+generate_CO2; 0.470, 0.773, pH_cumul o+PL+AN+AN_cumulo+interval_yield+OD+Cell_conc._interval+rab _integral+RQ_integral+Arg_conc.+emission_O2+consum_O2+inter val_CO2; 0.457, 0.773, pH_cumulo+PL+AN+AN_cumulo+interval_y ield+OD+Feed_Vol+Cell_conc._interval+rab_integral+RQ_integr al+Arg_conc.+emission_O2+emission_CO2+consum_O2; 0.443, 0.7 73, pH_cumulo+PL_cumulo+AN+AN_cumulo+interval_yield+OD+Cell _conc._interval+rab_integral+RQ_integral+Arg_conc.+emission _O2+emission_CO2+consum_O2+generate_CO2+interval_CO2; 0.443, 0.773, tmp+pH_cumulo+PL_cumulo+AN+AN_cumulo+interval_yield +OD+Cell_conc._interval+rab_integral+RQ_integral+Arg_conc.+ emission_O2+consum_O2+generate_CO2+interval_CO2; 0.482, 0.7 73, pH_cumulo+AN+AN_cumulo+OD+Feed_Vol+Cell_conc._interval+ rab_integral+RQ_integral+Arg_conc.+emission_O2+emission_CO2 +consum_O2; 0.469, 0.773, pH_cumulo+PL_cumulo+AN+AN_cumulo+ interval_yield+OD+Cell_conc._interval+rab_integral+RQ_integ ral+Arg_conc.+emission_CO2+generate_CO2+interval_CO2; 0.469, 0.773, pH_cumulo+AN+AN_cumulo+interval_yield+OD+Cell_conc. _interval+rab_integral+RQ_integral+Arg_conc.+emission_O2+in terval_O2+generate_CO2+interval_CO2; 0.442, 0.772, pH_cumul o+PL_cumulo+AN+AN_cumulo+interval_yield+OD+RS_cumulo+Cell_c one._interval+rab_integral+RQ_integral+Arg_conc.+emission_O 2+consum_O2+generate_CO2+interval_CO2; 0.427, 0.772, pH_cum ulo+PL+PL_cumulo+AN+AN_cumulo+interval_yield+OD+Feed_Vol+Ce ll_conc._interval+rab_integral+RQ_integral+Arg_conc.+emissi on_O2+consum_O2+interval_O2+interval_CO2; 0.427, 0.772, pH_ cumulo+PL+PL cumulo+AN+AN_cumulo+interval yield+OD+RS_cumul o+Cell_conc._interval+rab_integral+RQ_integral+Arg_conc.+em ission_O2+consum_O2+interval_O2+interval_CO2; 0.426, 0.772, pH_cumulo+PL+PL_cumulo+AN+AN_cumulo+interval_yield+OD+Feed _Vol+RS_cumulo+Cell_conc._interval+rab_integral+RQ_integral +Arg_conc.+emission_O2+consum_O2+interval_CO2; 0.504, 0.772, pH_cumulo+AN+AN_cumulo+OD+Cell_conc._interval+Arg_conc.+em ission_CO2+interval_O2+generate_CO2+interval_CO2; 0.426, 0. 772, pH+pH_cumulo+PL+PL_cumulo+AN+AN_cumulo+interval_yield+ OD+Feed_Vol+Cell_conc._interval+rab_integral+RQ_integral+Ar g_conc.+emission_O2+consum_O2+interval_CO2; 0.441, 0.772, p H_cumulo+PL+PL_cumulo+AN+AN_cumulo+interval_yield+OD+RS+Cel l_conc._interval+rab_integral+RQ_integral+Arg_conc.+emissio n_O2+consum_O2+interval_CO2; 0.515, 0.772, pH_cumulo+PL+AN_ cumulo+OD+Feed_Vol+Cell_conc._interval+Arg_conc.+emission_C O2+consum_O2; 0.441, 0.772, pH+pH_cumulo+PL_cumulo+AN+AN_cu mulo+interval_yield+OD+Feed_Vol+Cell_conc._interval+rab_int egral+RQ_ntegral+Arg_conc.+emission_O2+consum_O2+generate_ CO2; 0.426, 0.772, tmp_cumulo+pH+pH_cumulo+PL_cumulo+AN+AN_ cumulo+interval_yield+OD+Cell_conc._interval+rab_integral+R Q_integral+Arg_conc.+emission_O2+consum_O2+generate_CO2+int erval_CO2; 0.440, 0.772, tmp_cumulo+pH_cumulo+PL_cumulo+AN+ AN_cumulo+interval_yield+OD+Cell_conc._interval+rab_integra l+RQ_integral+Arg_conc.+emission_O2+consuni_O2+interval_O2+i nterval_CO2; 0.468, 0.772, pH_cumulo+PL_cumulo+AN+AN_cumulo +OD+Feed_Vol+Cell_conc._interval+rab_integral+RQ_integral+A rg_conc.+emission_O2+consum_O2+interval_CO2; 0.440, 0.772, pH_cumulo+PL_cumulo+AN+AN_cumulo+interval_yield+OD+Feed_Vol +Cell_conc._interval+rab_integral+RQ_integral+Arg_conc.+emi ssion_O2+interval_O2+generate_CO2+interval_CO2; 0.454, 0.77 2, tmp+pH_cumulo+PL_cumulo+AN+AN_cumulo+interval_yield+OD+C ell_conc._interval+rab_integral+RQ_integral+Arg_conc.+emiss ion_O2+consum_O2+interval_CO2; 0.492, 0.772, pH_cumulo+PL+A N+AN_cumulo+OD+Feed_Vol+Cell_conc._interval+RQ_integral+Arg _conc.+emission_CO2+consum_O2; 0.454, 0.772, pH_cumulo+PL_c umulo+AN+AN_cumulo+interval_yield+OD+RS_cumulo+Cell_conc._i nterval+rab_integral+RQ_integral+Arg_conc.+emission_O2+cons um_O2+interval_CO2; 0.440, 0.772, pH+pH_cumulo+PL_cumulo+AN +AN_cumulo+interval_yield+OD+Feed_Vol+Cell_conc._interval+r ab_integral+RQ_integral+Arg_conc.+emission_O2+consum_O2+int erval_CO2; 0.514, 0.771, pH_cumulo+AN+AN_cumulo+OD+Cell_con c._interval+Arg_conc.+emission_CO2+consum_O2+interval_CO2; 0.467, 0.771, pH+pH_cumulo+PL+AN+AN_cumulo+OD+Feed_Vol+Cell _conc._interval+rab_integral+RQ_integral+Arg_conc.+emission _O2+consum_O2; 0.453, 0.771, pH_cumulo+PL+AN+AN_cumulo+inte rval_yield+OD+Feed_Vol+Cell_conc._interval+rab_integral+RQ_ integral+Arg_conc.+emission_O2+consum_O2+interval_CO2; 0.49 1, 0.771, pH_cumulo+PL+AN+AN_cumulo+interval_yield+OD+Feed_ Vol+Cell_conc._interval+Arg_conc.+emission_CO2+consum_O2; 0. 491, 0.771, pH+pH_cumulo+PL+AN+AN_cumulo+OD+Feed_Vol+Cell_c onc._interval+Arg_conc.+emission_CO2+consum_O2; 0.503, 0.77 1, pH_cumulo+AN+AN_cumulo+OD+Feed_Vol+Cell_conc._interval+r ab_integral+Arg_conc.+emission_CO2+consum_O2; 0.439, 0.771, pH_cumulo+PL+PL_cumulo+AN+AN_cumulo+interval_yield+OD+Cell _conc._interval+rab_integral+RQ_integral+Arg_conc.+emission _O2+emission_CO2+consum_O2+interval_CO2; 0.466, 0.771, pH_c umulo+AN+AN_cumulo+interval_yield+OD+Feed_Vol+Cell_conc._in terval+rab_integral+RQ_integral+Arg_conc.+emission_O2+consu m_O2+generate_CO2; 0.424, 0.771, tmp_cumulo+pH_cumulo+PL+PL _cumulo+AN+AN_cumulo+interval_yield+OD+Cell_conc._interval+ rab_integral+RQ_integral+Arg_conc.+emission_O2+consum_O2+ge nerate_CO2+interval_CO2; 0.438, 0.771, pH_cumulo+PL_cumulo+ AN+AN_cumulo+interval_yield+OD+Feed_Vol+Cell_conc._interval +rab_integral+RQ_integral+Arg_conc.+emission_O2+emission_CO 2+consum_O2+interval_O2; 0.452, 0.771, pH_cumulo+PL_cumulo+ AN+AN_cumulo+interval_yield+OD+RS+Cell_conc._interval+rab_i ntegral+RQ_integral+Arg_conc.+emission_O2+consum_O2+interva l_CO2; 0.491, 0.771, pH_cumulo+AN+AN_cumulo+interval_yield+ OD+Cell_conc._interval+Arg_conc.+emission_CO2+interval_O2+g enerate_CO2+interval_CO2; 0.424, 0.771, tmp+pH_cumulo+PL+PL _cumulo+AN+AN_cumulo+interval_yield+OD+Cell_conc._interval+ rab_integral+RQ_integral+Arg_conc.+emission_O2+consum_O2+ge nerate_CO2+interval_CO2; 0.466, 0.771, tmp_cumulo+pH_cumulo +AN+AN_cumulo+interval_yield+OD+Cell_conc._interval+rab_int egral+RQ_integral+Arg_conc.+emission_O2+consum_O2+interval_ CO2; 0.452, 0.771, pH_cumulo+PL_cumulo+AN+AN_cumulo+interva l_yield+OD+Feed_Vol+RS_cumulo+Cell_conc._interval+rab-integ ral+RQ_integral+Arg_conc.+emission_O2+consum _O2; 0.452, 0.7 71, pH_cumulo+PL+AN+AN_cumulo+interval_yield+OD+Cell_conc._ interval+rab_integral+RQ_integral+Arg_conc.+emission_O2+int erval_O2+generate_CO2+interval_CO2; 0.452, 0.771, pH+pH_cum ulo+PL+PL_cumulo+AN+AN_cumulo+OD+Feed_Vol+Cell_conc._interv al+rab_integral+RQ_integral+Arg_conc.+emission_O2+consum_O 2; 0.465, 0.771, pH_cumulo+AN+AN_cumulo+interval_yield+OD+F eed_Vol+Cell_conc._interval+rab_integral+RQ_integral+Arg_co nc.+emission_O2+consum_O2+interval_CO2; 0.438, 0.771, tmp_c umulo+pH+pH_cumulo+PL_cumulo+AN+AN_cumulo+interval_yield+OD +Cell_conc._interval+rab_integral+RQ_integral+Arg_conc.+emi ssion_O2+consum_O2+interval_CO2; 0.490, 0.771, pH_cumulo+AN +AN_cumulo+OD+Feed_Vol+Cell_conc._interval+rab_integral+RQ_ integral+Arg_conc.+emission_CO2+consum_O2; 0.465, 0.771, pH _cumulo+AN+AN_cumulo+interval_yield+OD+Feed_Vol+Cell_conc._ interval+rab_integral+RQ_integral+Arg_conc.+emission_O2+con sum_O2+interval_O2; 0.438, 0.771, pH_cumulo+PL_cumulo+AN+AN _cumulo+interval_yield+OD+Feed_Vol+Cell_conc._interval+rab_ integral+RQ_integral+Arg_conc.+emission_O2+consum_O2+interv al_O2+generate_CO2; 0.502, 0.771, pH_cumulo+PL+AN+AN_cumulo +OD+Cell_conc._interval+Arg conc.+emission_CO2+consum_O2+in terval_CO2; 0.478, 0.771, pH_cumulo+PL_cumulo+AN+AN_cumulo+ interval_yield+OD+Cell_conc._interval+Arg_conc.+emission_CO 2+consum_O2+generate_CO2+interval_CO2; 0.490, 0.771, pH_cum ulo+AN+AN_cumulo+OD+Feed_Vol+Cell_conc._interval+RQ_integra l+Arg conc.+emission_O2+emission_CO2+consum_O2; 0.501, 0.77 1, pH_cumulo+AN+AN_cumulo+interval_yield+OD+Feed_Vol+Cell_c onc._interval+Arg_conc.+emission_CO2+consum_O2; 0.451, 0.77 1, pH_cumulo+PL_cumulo+AN+AN_cumulo+interval_yield+OD+Cell_ conc._interval+rab_integral+RQ_integral+Arg_conc.+emission_ O2+emission_CO2+consum_O2+interval_CO2; 0.512, 0.770, pH+pH _cumulo+AN_cumulo+OD+Feed_Vol+Cell_conc._interval+Arg_conc. +emission_CO2+consum_O2; 0.465, 0.770, pH_cumulo+PL+AN+AN_c umulo+OD+Feed_Vol+Cell_conc._interval+rab_integral+RQ-integ ral+Arg_conc.+emission_O2+emission_CO2+consum_O2; 0.490, 0. 770, pH_cumulo+AN+AN_cumulo+OD+Feed_Vol+Cell_conc._interval +RQ_integral+Arg_conc.+emission_CO2+consum_O2+generate_CO2; 0.451, 0.770, pH_cumulo+PL+AN+AN_cumulo+interval_yield+OD+ Feed_Vol+Cell_conc._interval+rab_integral+RQ_integral+Arg_c onc.+emission_O2+consum_O2+interval_O2; 0.501, 0.770, pH_cu mulo+AN+AN_cumulo+OD+Feed_Vol+Cell_conc._interval+Arg_conc. +emission_O2+emission_CO2+consum_O2; 0.501, 0.770, pH_cumul o+PL_cumulo+AN+AN_cumulo+OD+Feed_Vol+Cell_conc._interval+Ar g_conc.+emission_CO2+consum_O2; 0.437, 0.770, pH_cumulo+PL_ cumulo+AN+AN_cumulo+interval_yield+OD+Feed_Vol+RS_cumulo+Ce ll_conc._interval+rab_integral+RQ_integral+Arg_conc.+emissi on_O2+consum_O2+interval_O2; 0.477, 0.770, pH_cumulo+AN+AN_ cumulo+interval_yield+OD+Cell_conc._interval+rab_integral+R Q_integral+Arg_conc.+emission_O2+consum_O2+interval_CO2; 0. 422, 0.770, pH+pH_cumulo+PL_cumulo+AN+AN_cumulo+interval_yi eld+OD+Feed_Vol+Cell_conc._interval+rab_integral+RQ_integra 1+Arg conc.+emission_O2+consum_O2+interval_O2+interval_CO2; 0.451, 0.770, pH_cumulo+PL_cumulo+AN+AN_cumulo+interval_yi eld+OD+Feed_Vol+Cell_conc._interval+rab_integral+RQ_integra l+Arg conc.+emission_O2+emission_CO2+consum_O2; 0.437, 0.77 0, pH_cumulo+PL+AN+AN_cumulo+interval_yield+OD+Feed_Vol+RS_ cumulo+Cell_conc._interval+rab_integral+RQ_integral+Arg_con c.+emission_O2+consum_O2+interval_CO2; 0.451, 0.770, tmp_cu mulo+pH_cumulo+PL+AN+AN_cumulo+interval_yield+OD+Cell_conc. _interval+rab_integral+RQ_integral+Arg_conc.+emission_O2+co nsum_O2+interval_CO2; 0.500, 0.770, tmp_cumulo+pH_cumulo+AN +AN_cumulo+OD+Cell_conc._interval+Arg_conc.+emission_CO2+co nsum_O2+interval_CO2; 0.450, 0.770, pH_cumulo+PL+PL_cumulo+ AN+AN_cumulo+OD+Feed_Vol+Cell_conc._interval+rab_integral+R Q_integral+Arg_conc.+emission_O2+consum_O2+interval_CO2; 0. 421, 0.770, tmp+pH+pH_cumulo+PL+PL_cumulo+AN+AN_cumulo+inte rval_yield+OD+Cell_conc._interval+rab_integral+RQ_integral+ Arg_conc.+emission_O2+consum_O2+interval_CO2; 0.464, 0.770, pH_cumulo+AN+AN_cumulo+interval_yield+OD+Feed_Vol+RS_cumul o+Cell_conc._interval+rab_integral+RQ_integral+Arg_conc.+em ission_O2+consum_O2; 0.464, 0.770, pH+pH_cumulo+AN+AN_cumul o+OD+Feed_Vol+Cell_conc._interval+rab_integral+RQ_integral+ Arg_conc.+emission_O2+emission_CO2+consum_O2; 0.421, 0.770, tmp_cumulo+pH_cumulo+PL+PL_cumulo+AN+AN_cumulo+interval_yi eld+OD+Cell_conc._interval+rab_integral+RQ_integral+Arg_con c.+emission_O2+consum_O2+interval_O2+interval_CO2; 0.488, 0. 770, pH_cumulo+PL_cumulo+AN+AN_cumulo+interval_yield+OD+Cel l_conc._interval+Arg_conc.+emission_CO2+generate_CO2+interv al_CO2; 0.450, 0.770, pH_cumulo+PL+AN+AN_cumulo+interval_yi eld+OD+Feed_Vol+Cell_conc._interval+rab_integral+RQ_integra l+Arg_conc.+emission_O2+consum_O2+generate_CO2; 0.421, 0.77 0, tmp+pH+pH_cumulo+PL_cumulo+AN+AN_cumulo+interval_yield+O D+Cell_conc._interval+rab_integral+RQ_integral+Arg_conc.+em ission_O2+consum_O2+generate_CO2+interval_CO2; 0.488, 0.770, pH_cumulo+AN+AN_cumulo+OD+Feed_Vol+Cell_conc._interval+RQ_ integral+Arg_conc.+emission_CO2+consum_O2+interval_O2; 0.43 6, 0.770, pH+pH_cumulo+PL+AN+AN_cumulo+interval_yield+OD+Fe ed_Vol+RS_cumulo+Cell_conc._interval+rab_integral+RQ_integr al+Arg_conc.+emission_O2+consum_O2; 0.500, 0.770, pH_cumulo +AN+AN_cumulo+OD+Feed_Vol+Cell_conc._interval+Arg_conc.+emi ssion_CO2+consum_O2+interval_CO2; 0.476, 0.769, pH_cumulo+A N+AN_cumulo+OD+Feed_Vol+RS_cumulo+Cell_conc._interval+rab_i ntegral+RQ_integral+Arg_conc.+emission_O2+consum_O2; 0.449, 0.769, pH+pH_cumulo+AN+AN_cumulo+interval_yield+OD+Feed_Vo l+Cell_conc._interval+rab_integral+RQ_integral+Arg_conc.+em ission_O2+emission_CO2+consum_O2; 0.420, 0.769, tmp_cumulo+ pH+pH_cumulo+PL+PL_cumulo+AN+AN_cumulo+interval_yield+OD+Ce ll_conc._interval+rab_integral+RQ_integral+Arg_conc.+emissi on_O2+consum_O2+interval_CO2; 0.476, 0.769, pH_cumulo+AN+AN _cumulo+OD+Feed_Vol+Cell_conc._interval+rab_integral+RQ_int egral+Arg_conc.+emission_O2+consum_O2+interval_O2; 0.463, 0. 769, pH_cumulo+PL_cumulo+AN+AN_cumulo+OD+Feed_Vol+RS_cumulo +Cell_conc._interval+rab_integral+RQ_integral+Arg_conc.+emi ssion_O2+consum_O2; 0.488, 0.769, pH+pH_cumulo+AN+AN_cumulo +OD+Feed_Vol+Cell_conc._interval+RQ_integral+Arg_conc.+emis sion_CO2+consum_O2; 0.435, 0.769, pH_cumulo+PL_cumulo+AN+AN _cumulo+interval_yield+OD+RS+Cell_conc._interval+rab_integr al+RQ_integral+Arg_conc.+emission_O2+consum_O2+generate_CO2 +interval_CO2; 0.475, 0.769, pH_cumulo+AN+AN_cumulo+OD+Feed _Vol+Cell_conc._interval+rab_integral+RQ_integral+Arg_conc. +emission_O2+consum_O2+generate_CO2; 0.499, 0.769, pH_cumul o+AN+AN_cumulo+OD+Feed_Vol+Cell_conc._interval+Arg_conc.+em ission_CO2+consum_O2+generate_CO2; 0.487, 0.769, pH_cumulo+ PL+AN+AN_cumulo+OD+Feed_Vol+Cell_conc._interval+rab_integra l+Arg-conc.+emission_CO2+consum_O2; 0.420, 0.769, tmp_cumul o+pH+pH_cumulo+PL_cumulo+AN+AN_cumulo+interval_yield+OD+Cel l_conc._interval+rab_integral+RQ_integral+Arg_conc.+emissio n_O2+consum_O2+interval_O2+interval_CO2; 0.420, 0.769, pH+p H_cumulo+PL+PL_cumulo+AN+AN_cumulo+interval_yield+OD+Cell_c onc._interval+rab_integral+RQ_integral+Arg_conc.+emission_O 2+interval_O2+generate_CO2+interval_CO2; 0.499, 0.769, pH_c umulo+AN+AN_cumulo+OD+Feed_Vol+Cell_conc._interval+Arg_conc. +emission_CO2+consum_O2+interval_O2; 0.448, 0.769, pH+pH_cu mulo+AN+AN_cumulo+interval_yield+OD+Cell_conc._interval+rab _integral+RQ_integral+Arg_conc.+emission_O2+interval_O2+gen erate_CO2+interval_CO2; 0.419, 0.769, pH+pH_cumulo+PL_cumul o+AN+AN_cumulo+interval_yield+OD+Cell_conc._interval+rab_in tegral+RQ_integral+Arg_conc.+emission_O2+consum_O2+interval _O2+generate_CO2+interval_CO2; 0.510, 0.769, pH_cumulo+PL_c umulo+AN_cumulo+OD+Feed_Vol+Cell_conc._interval+Arg_conc.+e mission_CO2+consum_O2; 0.419, 0.769, pH_cumulo+PL+PL_cumulo +AN+AN_cumulo+interval_yield+OD+Cell_conc._interval+rab_int egral+RQ_integral+Arg_conc.+emission_O2+consum_O2+interval_ O2+generate_CO2+interval_CO2; 0.462, 0.769, pH_cumulo+PL_cu mulo+AN+AN_cumulo+OD+Feed_Vol+Cell_conc._interval+rab_integ ral+RQ_integral+Arg_conc.+emission_O2+consum_O2+generate_CO 2; 0.448, 0.769, tmp_cumulo+pH_cumulo+PL_cumulo+AN+AN_cumul o+interval_yield+OD+Cell_conc._interval+rab_integral+RQ_int egral+Arg_conc.+emission_CO2+generate_CO2+interval_CO2; 0.4 19, 0.769, tmp+pH_cumulo+PL+PL_cumulo+AN+AN_cumulo+interval _yield+OD+Cell_conc._interval+rab_integral+RQ_integral+Arg_ conc.+emission_O2+consum_O2+interval_O2+interval_CO2; 0.475, 0.769, pH_cumulo+AN+AN_cumulo+OD+Feed_Vol+Cell_conc._inter val+rab_integral+RQ_integral+Arg_conc.+emission_O2+consum_O 2+interval_CO2; 0.462, 0.769, pH_cumulo+PL_cumulo+AN+AN_cum ulo+OD+Feed_Vol+Cell_conc._interval+rab_integral+RQ_integra l+Arg_conc.+emission_O2+consum_O2+interval_O2; 0.487, 0.769, pH_cumulo+PL+AN+AN_cumulo+OD+Feed_Vol+Cell_conc._interval+ Arg_conc.+emission_O2+emission_CO2+consum_O2; 0.474, 0.769, pH_cumulo+PL+AN+AN_cumulo+OD+Feed_Vol+Cell_conc._interval+ rab_integral+RQ_integral+Arg_conc.+emission_CO2+consum_O2; 0.448, 0.769, pH+pH_cumulo+PL_cumulo+AN+AN_cumulo+OD+Feed_V ol+Cell_conc._interval+rab_integral+RQ_integral+Arg_conc.+e mission_O2+consum_O2+interval_CO2; 0.434, 0.769, tmp+pH_cum ulo+PL_cumulo+AN+AN_cumulo+interval_yield+OD+Cell_conc._int erval+rab_integral+RQ_integral+Arg_conc.+emission_O2+consum _O2+interval_O2+interval_CO2; 0.419, 0.769, pH_cumulo+PL+PL _cumulo+AN+AN_cumulo+interval_yield+OD+Feed_Vol+Cell_conc._ interval+rab integral+RQ_integral+Arg_conc.+emission_O2+con sum_O2+interval_O2+generate_CO2; 0.448, 0.769, tmp_cumulo+p H+pH_cumulo+AN+AN_cumulo+interval_yield+OD+Cell_conc._inter val+rab_integral+RQ_integral+Arg_conc.+emission_O2+consum_O 2+interval_CO2; 0.448, 0.769, pH+pH_cumulo+PL+AN+AN_cumulo+ interval_yield+OD+Cell_conc._interval+rab_integral+RQ_integ ral+Arg_conc.+emission_O2+consum_O2+interval_CO2; 0.498, 0. 769, pH_cumulo+AN+AN_cumulo+OD+Feed_Vol+RS_cumulo+Cell_conc. _interval+Arg_conc.+emission_CO2+consum_O2; 0.461, 0.769, p H_cumulo+PL+AN+AN_cumulo+OD+Feed_Vol+RS_cumulo+Cell_conc._i nterval+rab_integral+RQ_integral+Arg_conc.+emission_O2+cons um_O2; 0.419, 0.769, pH_cumulo+PL+PL_cumulo+AN+AN_cumulo+in terval_yield+OD+Cell_conc._interval+rab_integral+RQ_integra l+Arg_conc.+emission_O2+emission_CO2+consum_O2+generate_CO2 +interval_CO2; 0.448, 0.769, pH+pH_cumulo+AN+AN_cumulo+inte rval_yield+OD+Feed_Vol+Cell_conc._interval+rab_integral+RQ_ integral+Arg_conc.+emission_O2+consum_O2+generate_CO2; 0.46 1, 0.769, pH_cumulo+PL+AN+AN_cumulo+interval_yield+OD+Feed_ Vol+Cell_conc._interval+rab_integral+RQ_integral+Arg_conc.+ emission_CO2+consum_O2; 0.418, 0.769, pH_cumulo+PL+PL_cumul o+AN+AN_cumulo+interval_yield+OD+Feed_Vol+RS_cumulo+Cell_co nc._interval+rab_integral+RQ_integral+Arg_conc.+emission_O2 +consum_O2+interval_O2; 0.433, 0.769, pH+pH_cumulo+PL+AN+AN _cumulo+interval_yield+OD+Feed_Vol+Cell_conc._interval+rab_ integral+RQ_integral+Arg_conc.+emission_O2+emission_CO2+con sum_O2; 0.433, 0.769, pH_cumulo+PL+PL_cumulo+AN+AN_cumulo+i nterval_yield+OD+Feed_Vol+Cell_conc._interval+rab_integral+ RQ_integral+Arg_conc.+emission_O2+emission_CO2+consum_O2; 0. 447, 0.768, pH_cumulo+AN+AN_cumulo+interval_yield+OD+Feed_V ol+Cell_conc._interval+rab_integral+RQ_integral+Arg_conc.+e mission_O2+interval_O2+generate_CO2+interval_CO2; 0.418, 0. 768, pH+pH_cumulo+PL_cumulo+AN+AN_cumulo+interval_yield+OD+ Cell_conc._interval+rab_integral+RQ_integral+Arg_conc.+emis sion_O2+emission_CO2+consum_O2+generate_CO2+interval_CO2; 0. 402, 0.768, pH+pH_cumulo+PL+PL_cumulo+AN+AN_cumulo+interval _yield+OD+Feed_Vol+Cell_conc._interval+rab_integral+RQ_inte gral+Arg_conc.+emission_O2+consum_O2+generate_CO2+interval_ CO2; 0.447, 0.768, pH_cumulo+AN+AN_cumulo+interval_yield+OD +Cell_conc._interval+rab_integral+RQ_integral+Arg_conc.+emi ssion_O2+consum_O2+interval_O2+generate_CO2+interval_CO2; 0. 486, 0.768, pH_cumulo+PL+AN+AN_cumulo+OD+Feed_Vol+Cell_conc. interval+Arg_conc.+emission_CO2+consum_O2+interval_CO2; 0. 418, 0.768, pH_cumulo+PL+PL_cumulo+AN+AN_cumulo+interval_yi eld+OD+RS_cumulo+Cell_conc._interval+rab_integral+RQ_integr al+Arg conc.+emission_O2+emission_CO2+consum_O2+interval_CO 2; 0.473, 0.768, pH_cumulo+AN+AN_cumulo+interval_yield+OD+F eed_Vol+Cell_conc._interval+rab_integral+RQ_integral+Arg_co nc.+emission_CO2+consum_O2; 0.473, 0.768, pH_cumulo+PL_cumu lo+AN+AN_cumulo+interval_yield+OD+Cell_conc._interval+Arg_c onc.+emission_CO2+interval_O2+generate_CO2+interval_CO2; 0. 485, 0.768, tmp_cumulo+pH_cumulo+AN+AN_cumulo+OD+Cell_conc. _interval+Arg_conc.+emission_CO2+interval_O2+generate_CO2+i nterval_CO2; 0.447, 0.768, pH_cumulo+PL_cumulo+AN+AN_cumulo +interval_yield+OD+Cell_conc._interval+rab_integral+RQ_inte gral+Arg conc.+emission_CO2+consum_O2+generate CO2+interval _CO2; 0.433, 0.768, pH_cumulo+PL_cumulo+AN+AN_cumulo+interv al_yield+OD+RS_cumulo+Cell_conc._interval+rab_integral+RQ_i ntegral+Arg_conc.+emission_O2+interval_O2+generate_CO2+inte rval_CO2; 0.433, 0.768, pH_cumulo+PL+AN+AN_cumulo+interval_ yield+OD+Feed_Vol+RS_cumulo+Cell_conc._interval+rab_integra l+RQ_integral+Arg_conc.+emission_O2+emission_CO2+consum_O2; 0.433, 0.768, pH_cumulo+PL_cumulo+AN+AN_cumulo+interval_yi eld+OD+Cell_conc._interval+rab_integral+RQ_integral+Arg_con c.+emission_O2+emission_CO2+interval_O2+generate_CO2+interv al_CO2; 0.418, 0.768, pH_cumulo+PL_cumulo+AN+AN_cumulo+inte rval_yield+OD+Feed_Vol+Cell_conc._interval+rab_integral+RQ_ integral+Arg_conc.+emission_O2+emission_CO2+consum_O2+gener ate_CO2+interval_CO2; 0.485, 0.768, pH+pH_cumulo+AN+AN_cumu lo+OD+Cell_conc._interval+Arg_conc.+emission_CO2+interval_O 2+generate_CO2+interval_CO2; 0.432, 0.768, pH+pH_cumulo+PL+ AN+AN_cumulo+interval_yield+OD+RS_cumulo+Cell_conc._interva l+rab_integral+RQ_integral+Arg_conc.+emission_O2+consum_O2+ interval_CO2; 0.485, 0.768, tmp_cumulo+pH_cumulo+AN+AN_cumu lo+OD+Cell_conc._interval+Arg_conc.+emission_CO2+consum_O2+ generate_CO2+interval_CO2; 0.473, 0.768, pH_cumulo+PL+AN+AN _cumulo+OD+Feed_Vol+Cell_conc._interval+RQ_integral+Arg_con c.+emission_O2+emission_CO2+consum_O2; 0.485, 0.768, pH_cum ulo+PL_cumulo+AN_cumulo+OD+Feed_Vol+Cell_conc._interval+rab _integral+RQ_integral+Arg_conc.+emission_O2+consum_O2; 0.48 5, 0.768, tmp_cumulo+pH_cumulo+PL+AN+AN_cumulo+OD+Cell_conc. _interval+Arg_conc.+emission_CO2+consum_O2+interval_CO2; 0. 447, 0.768, pH_cumulo+PL+PL_cumulo+AN+AN_cumulo+interval_yi eld+OD+Cell_conc._interval+rab_integral+RQ_integral+Arg_con c.+emission_CO2+generate_CO2+interval_CO2; 0.432, 0.768, tm p+pH+pH_cumulo+PL_cumulo+AN+AN_cumulo+interval_yield+OD+Cel l_conc._interval+rab_integral+RQ_integral+Arg_conc.+emissio n_O2+consum_O2+interval_CO2; 0.432, 0.768, pH_cumulo+PL_cum ulo+AN+AN_cumulo+interval_yield+OD+Cell_conc._interval+rab_ integral+RQ_integral+Arg_conc.+emission_O2+emission_CO2+con sum_O2+interval_O2+interval_CO2; 0.460, 0.768, pH_cumulo+PL +AN+AN_cumulo+OD+Feed_Vol+Cell_conc._interval+rab_integral+ RQ_integral+Arg_conc.+emission_O2+consum_O2+interval_O2; 0. 460, 0.768, pH_cumulo+PL_cumulo+AN+AN_cumulo+OD+Feed_Vol+Ce ll_conc._interval+rab_integral+RQ_integral+Arg_conc.+emissi on_O2+emission_CO2+consum_O2; 0.446, 0.768, pH+pH_cumulo+AN +AN_cumulo+interval_yield+OD+Feed_Vol+Cell_conc._interval+r ab_integral+RQ_integral+Arg_conc.+emission_O2+consum_O2+int erval_O2; 0.446, 0.768, pH_cumulo+PL+AN+AN_cumulo+interval_ yield+OD+Feed_Vol+Cell_conc._interval+rab_integral+RQ_integ ral+Accum._Yield+Arg_conc.+emission_O2+consum_O2; 0.417, 0. 768, tmp+pH_cumulo+PL+PL_cumulo+AN+AN_cumulo+interval_yield +OD+RS_cumulo+Cell_conc._interval+rab_integral+RQ_integral+ Arg_conc.+emission_O2+consum_O2+interval_CO2; 0.496, 0.768, pH_cumulo+AN+AN_cumulo+OD+Cell_conc._interval+Arg_conc.+em ission_CO2+consum_O2+generate_CO2+interval_CO2; 0.417, 0.76 8, pH+pH_cumulo+PL+PL_cumulo+AN+AN_cumulo+interval_yield+OD +RS+Cell_conc._interval+rab_integral+RQ_integral+Arg_conc.+ emission_O2+consum_O2+interval_CO2; 0.401, 0.768, pH+pH_cum ulo+PL+PL_cumulo+AN+AN_cumulo+interval_yield+OD+RS_cumulo+C ell_conc._interval+rab_integral+RQ_integral+Arg_conc.+emiss ion_O2+consum_O2+generate_CO2+interval_CO2; 0.459, 0.768, t mp_cumulo+pH_cumulo+PL_cumulo+AN+AN_cumulo+OD+Cell_conc._in terval+rab_integral+RQ_integral+Arg_conc.+emission_O2+consu m_O2+interval_CO2; 0.496, 0.768, pH+pH_cumulo+PL+AN_cumulo+ OD+Feed Vol+Cell conc._interval+Arg_conc.+emission_CO2+cons um_O2; 0.432, 0.768, tmp+pH_cumulo+PL+PL_cumulo+AN+AN_cumul o+interval_yield+OD+Feed_Vol+Cell_conc._interval+rab_integr al+RQ_integral+Arg_conc.+emission_O2+consum_O2; 0.496, 0.76 8, pH+pH_cumulo+AN+AN_cumulo+OD+Cell_conc._interval+Arg_con c.+emission_CO2+consum_O2+interval_CO2; 0.417, 0.768, pH+pH _cumulo+PL_cumulo+AN+AN_cumulo+interval_yield+OD+RS_cumulo+ Cell_conc._interval+rab_integral+RQ_integral+Arg_conc.+emis sion_O2+consum_O2+generate_CO2+interval_CO2; 0.507, 0.768, pH_cumulo+AN_cumulo+interval_yield+OD+Feed_Vol+Cell_conc._i nterval+Arg_conc.+emission_CO2+consum_O2; 0.416, 0.768, pH_ cumulo+PL_cumulo+AN+AN_cumulo+interval_yield+OD+Feed_Vol+RS _cumulo+Cell_conc._interval+rab_integral+RQ_integral+Arg_co nc.+emission_O2+consum_O2+generate_CO2+interval_CO2; 0.484, 0.768, pH_cumulo+PL+AN+AN_cumulo+OD+Cell_conc._interval+Ar g_conc.+emission_CO2+interval_O2+generate_CO2+interval_CO2; 0.484, 0.768, pH_cumulo+PL+AN+AN_cumulo+OD+Feed_Vol+RS_cum ulo+Cell_conc._interval+Arg_conc.+emission_CO2+consum_O2; 0. 496, 0.768, pH_cumulo+AN+AN_cumulo+OD+Feed_Vol+Cell_conc._i nterval+RQ_integral+Arg_conc.+emission_O2+consum_O2; 0.431, 0.768, pH_cumulo+PL_cumulo+AN+AN_cumulo+interval_yield+OD+ RS_cumulo+Cell_conc._interval+rab_integral+RQ_integral+Arg_ conc.+emission_O2+consum_O2+interval_O2+interval_CO2; 0.416, 0.768, pH+pH_cumulo+PL_cumulo+AN+AN_cumulo+interval_yield+ OD+Feed_Vol+Cell_conc._interval+rab_integral+RQ_integral+Ar g_conc.+emission_O2+emission_CO2+consum_O2+interval_O2; 0.4 45, 0.768, pH_cumulo+PL+PL_cumulo+AN+AN_cumulo+OD+Feed_Vol+ RS_cumulo+Cell_conc._interval+rab_integral+RQ_integral+Arg_ conc.+emission_O2+consum_O2; 0.416, 0.768, pH+pH_cumulo+PL+ PL_cumulo+AN+AN_cumulo+interval_yield+OD+Feed_Vol+RS_cumulo +Cell_conc._interval+rab_integral+RQ_integral+Arg_conc.+emi ssion_O2+consum_O2; 0.507, 0.768, pH_cumulo+AN_cumulo+OD+Fe ed_Vol+Cell_conc._interval+Arg_conc.+emission_CO2+consum_O2 +interval_O2; 0.472, 0.768, pH_cumulo+PL_cumulo+AN_cumulo+i nterval_yield+OD+Feed_Vol+Cell_conc._interval+rab_integral+ RQ_integral+Arg_conc.+emission_O2+consum _O2; 0.431, 0.768, tmp_cumulo+pH_cumulo+PL_cumulo+AN+AN_cumulo+interval_yield+ OD+Cell_conc._interval+rab_integral+RQ_integral+Arg_conc.+e mission_O2+interval_O2+generate_CO2+interval_CO2; 0.445, 0. 768, pH+pH_cumulo+AN+AN_cumulo+interval_yield+OD+Feed_Vol+C ell_conc._interval+rab_integral+RQ_integral+Arg_conc.+emiss ion_O2+consum_O2+interval_CO2; 0.507, 0.767, pH_cumulo+AN_c umulo+OD+Feed_Vol+Cell_conc._interval+Arg_conc.+emission_CO 2+consum_O2+generate_CO2; 0.445, 0.767, pH_cumulo+AN+AN_cum ulo+interval_yield+OD+Cell_conc._interval+rab_integral+RQ_i ntegral+Arg conc.+emission_O2+emission_CO2+interval_O2+gene rate_CO2+interval_CO2; 0.445, 0.767, pH_cumulo+PL+AN+AN_cum ulo+interval_yield+OD+Cell_conc._interval+rab_integral+RQ_i ntegral+Arg_conc.+emission_O2+emission_CO2+consum_O2+interv al_CO2; 0.416, 0.767, pH+pH_cumulo+PL_cumulo+AN+AN_cumulo+i nterval_yield+OD+Feed_Vol+Cell_conc._interval+rab_integral+ RQ_integral+Arg_conc.+emission_O2+interval_O2+generate_CO2+ interval_CO2; 0.458, 0.767, pH_cumulo+PL+AN+AN_cumulo+OD+Fe ed_Vol+Cell_conc._interval+rab_integral+RQ_integral+Arg_con c.+emission_O2+consum_O2+generate_CO2; 0.416, 0.767, pH+pH_ cumulo+PL+PL_cumulo+AN+AN_cumulo+interval_yield+OD+Feed_Vol +Cell_conc._interval+rab_integral+RQ_integral+Arg_conc.+emi ssion_O2+consum_O2+generate_CO2; 0.484, 0.767, pH_cumulo+AN +AN_cumulo+interval_yield+OD+Cell_conc._interval+Arg_conc.+ emission_CO2+consum_O2+generate_CO2+interval_CO2; 0.471, 0. 767, pH+pH_cumulo+PL+AN+AN_cumulo+OD+Feed_Vol+Cell_conc._in terval+RQ_integral+Arg_conc.+emission_CO2+consum_O2; 0.431, 0.767, pH_cumulo+PL_cumulo+AN+AN_cumulo+interval_yield+OD+ Feed_Vol+RS_cumulo+Cell_conc._interval+rab_integral+RQ_inte gral+Arg_conc.+emission_O2+consum_O2+interval_CO2; 0.416, 0. 767, pH_cumulo+PL+PL_cumulo+AN+AN_cumulo+interval_yield+OD+ RS_cumulo+Cell_conc._interval+rab_integral+RQ_integral+Arg_ conc.+emission_O2+interval_O2+generate_CO2+interval_CO2; 0. 483, 0.767, pH_cumulo+PL+AN+AN_cumulo+OD+Feed_Vol+Cell_conc. _interval+Arg_conc.+emission_CO2+consum_O2+generate_CO2; 0. 495, 0.767, pH_cumulo+PL+AN_cumulo+interval_yield+OD+Feed_V ol+Cell_conc._interval+Arg_conc.+emission_CO2+consum_O2; 0. 445, 0.767, pH_cumulo+AN+AN_cumulo+interval_yield+OD+Feed_V ol+Cell_conc._interval+rab_integral+RQ_integral+Arg_conc.+e mission_O2+emission_CO2+consum_O2+generate_CO2; 0.483, 0.76 7, pH+pH_cumulo+PL+AN+AN_cumulo+OD+Cell_conc._interval+Arg_ conc.+emission_CO2+consum_O2+interval_CO2; 0.415, 0.767, pH +pH_cumulo+PL_cumulo+AN+AN_cumulo+interval_yield+OD+Feed_Vo l+Cell_conc._interval+rab_integral+RQ_integral+Arg_conc.+em ission_O2+consum_O2+interval_O2+generate_CO2; 0.483, 0.767, pH+pH_cumulo+AN+AN_cumulo+OD+Feed_Vol+Cell_conc._interval+ rab_integral+Arg_conc.+emission_CO2+consum_O2; 0.471, 0.767, pH_cumulo+AN+AN_cumulo+OD+Feed_Vol+Cell_conc._interval+RQ_ integral+Arg_conc.+emission_O2+emission_CO2+consum_O2+gener ate_CO2; 0.430, 0.767, tmp+pH_cumulo+PL_cumulo+AN+AN_cumulo +interval_yield+OD+Cell_conc._interval+rab_integral+RQ_inte gral+Arg_conc.+emission_O2+interval_O2+generate_CO2+interva l_CO2; 0.471, 0.767, tmp_cumulo+pH_cumulo+AN+AN_cumulo+OD+C ell_conc._interval+rab_integral+RQ_integral+Arg_conc.+emiss ion_CO2+consum_O2+interval_CO2; 0.399, 0.767, pH+pH_cumulo+ PL+PL_cumulo+AN+AN_cumulo+interval_yield+OD+Feed_Vol+Cell_c onc._interval+rab_integral+RQ_integral+Arg_conc.+emission_O 2+consum_O2+interval_O2+interval_CO2; 0.430, 0.767, pH_cumu lo+PL+PL_cumulo+AN+AN_cumulo+interval_yield+OD+Feed_Vol+Cel l_conc._interval+rab_integral+RQ_integral+Accum._Yield+Arg_ conc.+emission_O2+consum_O2; 0.495, 0.767, tmp+pH_cumulo+AN +AN_cumulo+OD+Feed_Vol+Cell_conc._interval+Arg_conc.+emissi on_CO2+consum_O2; 0.430, 0.767, pH+pH_cumulo+PL+AN+AN_cumul o+interval_yield+OD+Feed_Vol+Cell_conc._interval+rab_integr al+RQ integral+Arg_conc.+emission_O2+consumc_O2+interval_CO 2; 0.483, 0.767, pH_cumulo+PL+AN+AN_cumulo+OD+Feed_Vol+Cell _conc._interval+Arg_conc.+emission_CO2+consum_O2+interval_O 2; 0.483, 0.767, pH_cumulo+PL+PL_cumulo+AN+AN_cumulo+OD+Fee d_Vol+Cell_conc._interval+Arg_conc.+emission_CO2+consum_O2; 0.458, 0.767, pH_cumulo+PL+PL_cumulo+AN_cumulo+interval_yi eld+OD+Feed_Vol+Cell_conc._interval+rab_integral+RQ_integra l+Arg_conc.+emission_O2+consum_O2; 0.506, 0.767, pH_cumulo+ AN_cumulo+OD+Feed_Vol+Cell_conc._interval+Arg_conc.+emissio n_O2+emission_CO2+consum_O2; 0.399, 0.767, pH+pH_cumulo+PL+ PL_cumulo+AN+AN_cumulo+interval_yield+OD+RS_cumulo+Cell _con c._interval+rab_integral+RQ_integral+Arg_conc.+emission_O2+ consum_O2+interval_O2+interval_CO2; 0.430, 0.767, pH_cumulo +PL+AN+AN_cumulo+interval_yield+OD+RS_cumulo+Cell_conc._int erval+rab_integral+RQ_integral+Arg_conc.+emission_O2+interv al_O2+generate_CO2+interval_CO2; 0.471, 0.767, pH_cumulo+AN +AN_cumulo+interval_yield+OD+Feed_Vol+Cell_conc._interval+R Q_integral+Arg_conc.+emission_CO2+consum_O2+generate_CO2; 0. 471, 0.767, pH+pH_cumulo+AN+AN_cumulo+OD+Feed_Vol+Cell_conc. _interval+RQ_integral+Arg_conc.+emission_O2+emission_CO2+co nsum_O2; 0.430, 0.767, tmp+pH_cumulo+PL_cumulo+AN+AN_cumulo +interval_yield+OD+Feed_Vol+Cell_conc._interval+rab_integra l+RQ_integral+Arg_conc.+emission_O2+consum_O2+interval_O2; 0.458, 0.767, pH+pH_cumulo+AN+AN_cumulo+OD+Feed_Vol+RS_cumu lo+Cell_conc._interval+rab_integral+RQ_integral+Arg_conc.+e mission_O2+consum_O2; 0.458, 0.767, pH+pH_cumulo+AN+AN_cumu lo+OD+Feed_Vol+Cell_conc._interval+rab_integral+RQ_integral +Arg_conc.+emission_O2+consum_O2+interval_O2; 0.470, 0.767, pH_cumulo+PL_cumulo+AN+AN_cumulo+OD+Cell_conc._interval+ra b_integral+RQ_integral+Arg_conc.+emission_O2+consum_O2+inte rval_CO2; 0.444, 0.767, tmp+pH_cumulo+PL+AN+AN_cumulo+inter val_yield+OD+Feed_Vol+Cell_conc._interval+rab_integral+RQ_i ntegral+Arg_conc.+emission_O2+consum_O2; 0.470, 0.767, tmp+ pH_cumulo+AN+AN_cumulo+OD+Feed_Vol+Cell_conc._interval+rab_ integral+RQ_integral+Arg_conc.+emission_O2+consum_O2; 0.457, 0.767, pH_cumulo+PL+AN+AN_cumulo+OD+Feed_Vol+Cell_conc._in terval+rab_integral+RQ_integral+Arg_conc.+emission_O2+consu m_O2+interval_CO2; 0.444, 0.767, pH+pH_cumulo+AN+AN_cumulo+ interval_yield+OD+Feed_Vol+RS_cumulo+Cell_conc._interval+ra b_integral+RQ_integral+Arg_conc.+emission_O2+consum_O2; 0.4 57, 0.767, pH_cumulo+PL+PL_cumulo+AN+AN_cumulo+interval_yie ld+OD+Cell_conc._interval+rab_integral+RQ_integral+Arg_conc. +emission_O2+consum_O2; 0.494, 0.767, pH_cumulo+AN_cumulo+O D+Feed_Vol+Cell_conc._interval+rab_integral+RQ_integral+Arg _conc.+emission_O2+consum_O2; 0.470, 0.767, pH+pH_cumulo+AN +AN_cumulo+OD+Feed_Vol+Cell_conc._interval+rab_integral+RQ_ integral+Arg_conc.+emission_CO2+consum_O2; 0.399, 0.767, pH +pH cumulo+PL+PL cumulo+AN+AN_cumulo+interval_yield+OD+Feed _Vol+RS_cumulo+Cell_conc._interval+rab_integral+RQ_integral +Arg_conc.+emission_O2+consum_O2+interval_CO2; 0.414, 0.767, pH_cumulo+PL+PL_cumulo+AN+AN_cumulo+interval_yield+OD+Cell _conc._interval+rab_integral+RQ_integral+Arg_conc.+emission _O2+emission_CO2+consum_O2+interval_O2+interval_CO2; 0.482, 0.767, pH_cumulo+PL+AN+AN_cumulo+interval_yield+OD+Cell_co nc._interval+Arg_conc.+emission_CO2+consum_O2+interval_CO2; 0.429, 0.767, pH_cumulo+PL+AN+AN_cumulo+interval_yield+OD+ RS_cumulo+Cell_conc._interval+rab_integral+RQ_integral+Arg_ conc.+emission_O2+emission_CO2+consum_O2+interval_CO2; 0.42 9, 0.767, pH+pH_cumulo+PL_cumulo+AN+AN_cumulo+interval_yiel d+OD+RS_cumulo+Cell_conc._interval+rab_integral+RQ_integral +Arg_conc.+emission_02+consum_O2+interval_CO2; 0.457, 0.767, pH+pH_cumulo+AN+AN_cumulo+OD+Feed_Vol+Cell_conc._interval+ rab_integral+RQ_integral+Arg_conc.+emission_O2+consum_O2+ge nerate_CO2; 0.482, 0.767, pH+pH_cumulo+AN+AN_cumulo+OD+Feed _Vol+Cell_conc._interval+RQ_integral+Arg_conc.+emission_O2+ consum_O2; 0.505, 0.767, pH_cumulo+AN_cumulo+OD+Feed_Vol+RS _cumulo+Cell_conc._interval+Arg_conc.+emission_CO2+consum_O 2; 0.429, 0.767, pH_cumulo+PL+PL_cumulo+AN+AN_cumulo+interv al_yield+OD+Cell_conc._interval+rab_integral+RQ_integral+Ac cum._Yield+Arg_conc.+emission_O2+consum_O2+interval_CO2; 0. 443, 0.767, pH_cumulo+AN+AN_cumulo+interval_yield+OD+Feed_V ol+Cell_conc._interval+rab_integral+RQ_integral+Arg_conc.+e mission_O2+emission_CO2+consum_O2+interval_O2; 0.414, 0.767, pH_cumulo+PL+PL_cumulo+AN+AN_cumulo+interval_yield+OD+Feed _Vol+Cell_conc._interval+rab_integral+RQ_integral+Arg_conc. +emission_O2+emission_CO2+consum_O2+interval_O2; 0.470, 0.7 67, pH_cumulo+AN+AN_cumulo+OD+Feed_Vol+Cell_conc._interval+ RQ_integral+Arg_conc.+emission_O2+emission_CO2+consum_O2+in terval_O2; 0.470, 0.767, pH_cumulo+PL+AN+AN_cumulo+OD+Feed_ Vol+Cell_conc._interval+RQ_integral+Arg_conc.+emission_CO2+ consum_O2+generate_CO2; 0.482, 0.767, pH+pH_cumulo+PL_cumul o+AN+AN_cumulo+OD+Feed_Vol+Cell_conc._interval+Arg_conc.+em ission_CO2+consum_O2; 0.414, 0.767, pH_cumulo+PL+PL_cumulo+ AN+AN_cumulo+interval_yield+OD+Feed_Vol+Cell_conc._interval +rab_integral+RQ_integral+Arg_conc.+emission_O2+interval_O2 +generate_CO2+interval_CO2; 0.457, 0.767, pH+pH_cumulo+AN+A N_cumulo+OD+Feed_Vol+Cell_conc._interval+rab_integral+RQ_in tegral+Arg_conc.+emission_O2+consum_O2+interval_CO2; 0.414, 0.767, pH_cumulo+PL_cumulo+AN+AN_cumulo+interval yield+OD+ Feed_Vol+Cell_conc._interval+rab_integral+RQ_integral+Arg_c onc.+emission_O2+consum_O2+interval_O2+generate_CO2+interva l_CO2; 0.443, 0.767, pH_cumulo+PL+PL_cumulo+AN+AN_cumulo+OD +Feed_Vol+Cell_conc._interval+rab_integral+RQ_integral+Arg_ conc.+emission_O2+consum_O2+generate_CO2; 0.470, 0.767, pH_ cumulo+PL+AN+AN_cumulo+interval_yield+OD+Cell_conc._interva l+Arg_conc.+emission_CO2+interval_O2+generate_CO2+interval CO2; 0.414, 0.767, pH+pH_cumulo+PL+PL_cumulo+AN+AN_cumulo+i nterval_yield+OD+Cell_conc._interval+rab_integral+RQ_integr al+Arg_conc.+emission_O2+emission_CO2+consum_O2+interval_CO 2; 0.457, 0.766, tmp+pH_cumulo+PL_cumulo+AN+AN_cumulo+OD+Fe ed_Vol+Cell_conc._interval+rab_integral+RQ_integral+Arg_con c.+emission_O2+consum_O2; 0.443, 0.766, pH+pH_cumulo+PL_cum ulo+AN+AN_cumulo+interval_yield+OD+Cell_conc._interval+rab_ integral+RQ_integral+Arg_conc.+emission_CO2+generate_CO2+in terval_CO2; 0.457, 0.766, pH+pH_cumulo+AN+AN_cumulo+interva l_yield+OD+Cell_conc._interval+rab_integral+RQ_integral+Arg _conc.+emission_O2+consum_O2+interval_CO2; 0.443, 0.766, pH +pH_cumulo+PL+AN+AN_cumulo+OD+Feed_Vol+Cell_conc._interval+ rab_integral+RQ_integral+Arg_conc.+emission_O2+emission_CO2 +consum_O2; 0.470, 0.766, pH+pH_cumulo+PL+AN+AN_cumulo+inte rval_yield+OD+Feed_Vol+Cell_conc._interval+Arg_conc.+emissi on_CO2+consum_O2; 0.482, 0.766, pH+pH_cumulo+AN+AN_cumulo+i nterval_yield+OD+Feed_Vol+Cell_conc._interval+Arg_conc.+emi ssion-CO2+consum-O2; 0.482, 0.766, tmp_cumulo+pH+pH_cumulo+ AN+AN cumulo+OD+Cell conc._interval+Arg_conc.+emission_CO2+ consum_O2+interval_CO2; 0.470, 0.766, pH_cumulo+AN+AN_cumul o+interval_yield+OD+Feed_Vol+Cell_conc._interval+RQ_integra l+Arg_conc.+emission_CO2+consum_O2+interval_O2; 0.429, 0.76 6, pH+pH_cumulo+PL_cumulo+AN+AN_cumulo+interval_yield+OD+RS +Cell_conc._interval+rab_integral+RQ_integral+Arg_conc.+emi ssion_O2+consum_O2+interval_CO2; 0.443, 0.766, pH_cumulo+PL +PL_cumulo+AN+AN_cumulo+OD+Feed_Vol+Cell_conc._interval+rab _integral+RQ_integral+Arg_conc.+emission_O2+consum_O2+inter val_O2; 0.482, 0.766, pH_cumulo+AN+AN_cumulo+OD+Feed_Vol+Ce ll_conc._interval+Arg_conc.+emission_CO2+interval_O2+genera te_CO2+interval_CO2; 0.414, 0.766, pH+pH_cumulo+PL_cumulo+A N+AN_cumulo+interval_yield+OD+Feed_Vol+RS_cumulo+Cell_conc. _interval+rab_integral+RQ_integral+Arg_conc.+emission_O2+co _nsum_O2+interval_O2; 0.469, 0.766, pH_cumulo+PL+AN+AN_cumul o+OD+Feed_Vol+Cell_conc._interval+RQ_integral+Arg_conc.+emi ssion_CO2+consum_O2+interval_O2; 0.429, 0.766, pH+pH_cumulo +PL_cumulo+AN+AN_cumulo+interval_yield+OD+Feed_Vol+Cell_con c._interval+rab_integral+RQ_integral+Arg_conc.+emission_O2+ emission_CO2+consum_O2; 0.443, 0.766, tmp+pH_cumulo+PL_cumu lo+AN+AN_cumulo+interval_yield+OD+Feed_Vol+Cell_conc._inter val+rab integral+RQ integral+Arg_conc.+emission_O2+consum_O 2; 0.429, 0.766, pH_cumulo+PL_cumulo+AN+AN_cumulo+interval_ yield+OD+Feed_Vol+Cell_conc._interval+rab_integral+RQ_integ_ ral+Arg_conc.+emission_O2+emission_CO2+consum_O2+interval_C O2; 0.456, 0.766, pH_cumulo+PL+AN+AN_cumulo+interval_yield+ OD+Cell_conc._interval+rab_integral+RQ_integral+Arg_conc.+e mission_CO2+consum_O2+interval_CO2; 0.443, 0.766, pH+pH_cum ulo+PL_cumulo+AN+AN_cumulo+OD+Feed_Vol+Cell_conc._interval+ rab_integral+RQ_integral+Arg_conc.+emission_O2+consum_O2+in terval_O2; 0.428, 0.766, pH+pH_cumulo+PL_cumulo+AN+AN_cumul o+interval_yield+OD+Feed_Vol+RS_cumulo+Cell_conc._interval+ rab_integral+RQ_integral+Arg_conc.+emission_O2+consum_O2; 0. 469, 0.766, pH+pH_cumulo+AN+AN_cumulo+OD+Feed_Vol+Cell_conc. _interval+RQ_integral+Arg_conc.+emission_CO2+consum_O2+gene rate_CO2; 0.456, 0.766, pH_cumulo+AN+AN_cumulo+interval_yie ld+OD+Feed_Vol+Cell_conc._interval+rab_integral+RQ_integral +Accum._Yield+Arg_conc.+emission_O2+consum_O2; 0.482, 0.766, pH_cumulo+AN+AN_cumulo+OD+Feed_Vol+Cell_conc._interval+rab _integral+Arg_conc.+emission_CO2+consum_O2+generate_CO2; 0. 456, 0.766, pH_cumulo+PL+PL_cumulo+AN+AN_cumulo+OD+Cell_con c._interval+rab_integral+RQ_integral+Arg_conc.+emission_O2+ consum_O2+interval_CO2; 0.481, 0.766, pH_cumulo+PL_cumulo+A N+AN_cumulo+OD+Feed_Vol+Cell_conc._interval+RQ_integral+Arg _conc.+emission_CO2+consum_O2; 0.456, 0.766, tmp+pH_cumulo+ AN+AN_cumulo+interval_yield+OD+Feed_Vol+Cell_conc._interval +rab_integral+RQ_integral+Arg_conc.+emission_O2+consum_O2; 0.481, 0.766, pH+pH_cumulo+AN+AN_cumulo+OD+Feed_Vol+Cell_co nc._interval+Arg_conc.+emission_O2+emission_CO2+consum_O2; 0.428, 0.766, pH_cumulo+PL_cumulo+AN+AN_cumulo+interval_yie ld+OD+Feed_Vol+Cell_conc._interval+rab_integral+RQ_integral +Accum._Yield+Arg_conc.+emission_O2+consum_O2+interval_O2; 0.397, 0.766, tmp+pH+pH_cumulo+PL+PL_cumulo+AN+AN_cumulo+in terval_yield+OD+Cell_conc._interval+rab_integral+RQ_integra l+Arg conc.+emissio_O2+consum_O2+generate_CO2+interval_CO 2; 0.397, 0.766, tmp_cumulo+pH+pH_cumulo+PL+PL_cumulo+AN+AN _cumulo+interval_yield+OD+Cell_conc._interval+rab_integral+ RQ_integral+Arg_conc.+emission_O2+consum_O2+generate_CO2+in terval_CO2; 0.469, 0.766, pH+pH_cumulo+AN+AN_cumulo+interva l_yield+OD+Cell_conc._interval+Arg_conc.+emission_CO2+inter val_O2+generate_CO2+interval_CO2; 0.469, 0.766, pH_cumulo+P L+AN+AN_cumulo+interval_yield+OD+Feed_Vol+RS_cumulo+Cell_co nc._interval+Arg_conc.+emission_CO2+consum_O2; 0.413, 0.766, tmp+tmp_cumulo+pH_cumulo+PL+PL_cumulo+AN+AN_cumulo+interva l_yield+OD+Cell_conc._interval+rab_integral+RQ_integral+Arg _conc.+emission_O2+consum_O2+interval_CO2; 0.481, 0.766, pH _cumulo+AN+AN_cumulo+OD+Cell_conc._interval+Arg_conc.+emiss ion_CO2+consum_O2+interval_O2+generate_CO2+interval_CO2; 0. 428, 0.766, pH+pH_cumulo+PL+AN+AN_cumulo+interval_yield+OD+ Cell_conc._interval+rab_integral+RQ_integral+Arg_conc.+emis sion_O2+interval_O2+generate_CO2+interval_CO2; 0.469, 0.766, pH+pH_cumulo+AN+AN_cumulo+OD+Feed_Vol+Cell_conc._interval+ RQ_integral+Arg_conc.+emission_CO2+consum_O2+interval_O2; 0. 481, 0.766, pH_cumulo+AN+AN_cumulo+OD+Feed_Vol+Cell_conc._i nterval+rab_integral+Arg_conc.+emission_CO2+consum_O2+inter val_O2; 0.413, 0.766, tmp+pH_cumulo+PL+PL_cumulo+AN+AN_cumu lo+interval_yield+OD+Feed_Vol+Cell_conc._interval+rab_integ ral+RQ_integral+Arg_conc.+emission_O2+consum_O2+interval_O 2; 0.428, 0.766, pH+pH_cumulo+PL_cumulo+AN+AN_cumulo+interv al_yield+OD+Cell_conc._interval+rab_integral+RQ_integral+Ar g_conc.+emission_O2+emission_CO2+consum_O2+interval_CO2; 0. 442, 0.766, pH+pH_cumulo+PL_cumulo+AN+AN_cumulo+OD+Feed_Vol +Cell_conc._interval+rab_integral+RQ_integral+Arg_conc.+emi ssion_O2+consum_O2+generate_CO2; 0.428, 0.766, pH+pH_cumulo +PL+PL_cumulo+AN+AN_cumulo+OD+Feed_Vol+Cell_conc._interval+ rab_integral+RQ_integral+Arg_conc.+emission_O2+consum_O2+in terval_CO2; 0.397, 0.766, pH_cumulo+PL+PL_cumulo+AN+AN_cumu lo+interval_yield+OD+Feed_Vol+RS_cumulo+Cell_conc._interval +rab integral+RQ integral+Arg_conc.+emission_O2+consum_O2+g enerate_CO2+interval_CO2; 0.481, 0.766, pH+pH_cumulo+AN+AN_ cumulo+OD+Feed_Vol+Cell_conc._interval+Arg_conc.+emission_C O2+consum_O2+interval_CO2; 0.455, 0.766, pH_cumulo+PL_cumul o+AN+AN_cumulo+interval_yield+OD+Feed_Vol+Cell_conc._interv al+RQ_integral+Arg_conc.+emission_CO2+consum_O2+interval_O 2; 0.455, 0.766, pH_cumulo+AN+AN_cumulo+interval_yield+OD+C ell_conc._interval+rab_integral+RQ_integral+Arg_conc.+emiss ion_CO2+interval_O2+generate_CO2+interval_CO2; 0.442, 0.766, pH_cumulo+AN+AN_cumulo+interval_yield+OD+Feed_Vol+Cell_con c._interval+rab_integral+RQ_integral+Arg_conc.+emission_O2+ emission_CO2+consum_O2+interval_CO2; 0.455, 0.766, pH_cumul o+AN+AN_cumulo+interval_yield+OD+Cell_conc._interval+rab_in tegral+RQ_integral+Arg_conc.+emission_O2+emission_CO2+consu m_O2+interval_CO2; 0.481, 0.766, pH_cumulo+PL+AN+AN_cumulo+ OD+Feed_Vol+Cell_conc._interval+RQ_integral+Arg_conc.+emiss ion_O2+consum_O2; 0.427, 0.766, tmp_cumulo+pH+pH_cumulo+PL+ AN+AN_cumulo+interval_yield+OD+Cell_conc._interval+rab_inte gral+RQ_integral+Arg_conc.+emission_O2+consum_O2+interval_C O2; 0.427, 0.766, pH+pH_cumulo+PL+AN+AN_cumulo+interval_yie ld+OD+Feed_Vol+Cell_conc._interval+rab_integral+RQ_integral +Arg_conc.+emission_O2+consum_O2+interval_O2; 0.427, 0.766, pH_cumulo+PL+AN+AN_cumulo+interval_yield+OD+Feed_Vol+Cell_ conc._interval+rab_integral+RQ_integral+Arg_conc.+emission_ O2+emission_CO2+consum_O2+interval_CO2; 0.427, 0.766, pH_cu mulo+PL_cumulo+AN+AN_cumulo+interval_yield+OD+RS+Feed_Vol+C ell_conc._interval+rab_integral+RQ_integral+Arg_conc.+emiss ion_O2+consum_O2+interval_O2; 0.480, 0.766, tmp_cumulo+pH_c umulo+AN+AN_cumulo+OD+Cell_conc._interval+Arg_conc.+emissio n_CO2+consum_O2+interval_O2+interval_CO2; 0.492, 0.766, pH_ cumulo+PL+PL_cumulo+AN_cumulo+OD+Feed_Vol+Cell_conc._interv al+Arg_conc.+emission_CO2+consum_O2; 0.441, 0.766, pH+pH_cu mulo+PL_cumulo+AN+AN_cumulo+OD+Feed_Vol+RS_cumulo+Cell_conc. interval+rab_integral+RQ_integral+Arg_conc.+emission_O2+co nsum_O2; 0.412, 0.766, tmp+pH_cumulo+PL+PL_cumulo+AN+AN_cum ulo+interval_yield+OD+Feed_Vol+Cell_conc._interval+rab_inte gral+RQ_integral+Arg_conc.+emission_O2+consum_O2+interval_C O2; 0.492, 0.766, tmp_cumulo+pH_cumulo+AN+AN_cumulo+OD+Feed _Vol+Cell_conc._interval+Arg_conc.+emission_CO2+consum_O2; 0.427, 0.766, pH_cumulo+PL_cumulo+AN+AN_cumulo+interval_yie ld+OD+RS+Cell_conc._interval+rab_integral+RQ_integral+Arg_c onc.+emission_O2+consum_O2+interval_O2+interval_CO2; 0.480, 0.766, pH+pH_cumulo+AN+AN_cumulo+OD+Feed_Vol+Cell_conc._in terval+Arg_conc.+emission_CO2+consum _O2+interval_O2; 0.480, 0.765, tmp_cumulo+pH_cumulo+AN+AN_cumulo+OD+Feed_Vol+Cell_ conc._interval+Arg_conc.+emission_CO2+consum_O2+interval_CO 2; 0.455, 0.765, pH_cumulo+AN+AN_cumulo+OD+Feed_Vol+Cell_co nc._interval+rab_integral+RQ_integral+Arg_conc.+emission_O2 +emission_CO2+consum_O2+generate_CO2; 0.441, 0.765, pH_cumu lo+PL_cumulo+AN+AN_cumulo+interval_yield+OD+Feed_Vol+Cell_c onc._interval+rab_integral+RQ_integral+Arg_conc.+emission_C O2+consum_O2+interval_O2; 0.441, 0.765, tmp_cumulo+pH+pH_cu mulo+PL_cumulo+AN+AN_cumulo+OD+Cell_conc._interval+rab_inte gral+RQ_integral+Arg_conc.+emission_O2+consum_O2+interval_C O2; 0.427, 0.765, pH_cumulo+PL_cumulo+AN+AN_cumulo+interval _yield+OD+Feed_Vol+Cell_conc._interval+rab_integral+RQ_inte gral+Arg_conc.+emission_O2+eniission_CO2+consum_O2+generate_ CO2; 0.412, 0.765, tmp+pH_cumulo+PL_cumulo+AN+AN_cumulo+int erval_yield+OD+Feed_Vol+Cell_conc._interval+rab_integral+RQ _integral+Arg_conc.+emission_O2+consum_O2+generate_CO2+inte rval_CO2; 0.411, 0.765, tmp+pH+pH_cumulo+PL_cumulo+AN+AN_cu mulo+interval_yield+OD+Cell_conc._interval+rab_integral+RQ_ integral+Arg_conc.+emission_O2+consum_O2+interval_O2+interv al_CO2; 0.396, 0.765, pH_cumulo+PL+PL_cumulo+AN+AN_cumulo+i nterval_yield+OD+Feed_Vol+RS_cumulo+Cell_conc._interval+rab _integral+RQ_integral+Arg_conc.+emission_O2+consum_O2+inter val_O2+interval_CO2; 0.411, 0.765, tmp_cumulo+pH_cumulo+PL_ cumulo+AN+AN_cumulo+interval_yield+OD+Feed_Vol+Cell_conc._i nterval+rab_integral+RQ_integral+Arg_conc.+emission_O2+cons um_O2+generate_CO2+interval_CO2; 0.480, 0.765, pH_cumulo+AN +AN_cumulo+OD+Feed_Vol+Cell_conc._interval+rab_integral+Arg _conc.+emission_O2+emission_CO2+consum_O2; 0.441, 0.765, pH _cumulo+PL_cumulo+AN+AN_cumulo+interval_yield+OD+Feed_Vol+C ell_conc._interval+rab_integral+RQ_integral+Accum._Yield+Ar g_conc.+emission_O2+consum_O2; 0.467, 0.765, tmp_cumulo+pH_ cumulo+AN+AN_cumulo+interval_yield+OD+Cell_conc._interval+A rg_conc.+emission_CO2+interval_O2+generate_CO2+interval_CO 2; 0.480, 0.765, tmp+pH_cumulo+PL+AN+AN_cumulo+OD+Feed_Vol+ Cell_conc._interval+Arg_conc.+emission_CO2+consum_O2; 0.426, 0.765, pH_cumulo+PL+AN+AN_cumulo+interval_yield+OD+Feed_Vo l+Cell_conc._interval+rab_integral+RQ_integral+Arg_conc.+em ission_O2+interval_O2+generate_CO2+interval_CO2; 0.426, 0.7 65, pH_cumulo+PL+AN+AN_cumulo+interval_yield+OD+Feed_Vol+RS _cumulo+Cell_conc._interval+rab_integral+RQ_integral+Accum. _Yield+Arg_conc.+emission_O2+consum_O2; 0.441, 0.765, tmp_c umulo+pH_cumulo+AN+AN_cumulo+interval_yield+OD+Cell_conc._i nterval+rab_integral+RQ_integral+Arg_conc.+emission_O2+emis sion_CO2+consum_O2+interval_CO2; 0.491, 0.765, pH_cumulo+AN +AN_cumulo+OD+Cell_conc._interval+Arg_conc.+emission_CO2+co nsum_O2+interval_O2+interval_CO2; 0.411, 0.765, pH_cumulo+P L+PL_cumulo+AN+AN_cumulo+interval_yield+OD+Feed_Vol+Cell_co nc._interval+rab_integral+RQ_integral+Accum._Yield+Arg_conc. +emission_O2+consum_O2+interval_O2; 0.467, 0.765, pH_cumulo +PL+AN+AN_cumulo+interval_yield+OD+Feed_Vol+Cell_conc._inte rval+Arg_conc.+emission_O2+emission_CO2+consum_O2; 0.480, 0. 765, pH+pH_cumulo+AN+AN_cumulo+OD+Feed_Vol+Cell_conc._inter val+Arg_conc.+emission_CO2+consum_O2+generate_CO2; 0.411, 0. 765, pH_cumulo+PL+PL_cumulo+AN+AN_cumulo+interval_yield+OD+ Feed_Vol+Cell_conc._interval+rab_integral+RQ_integral+Arg_c onc.+emission_O2+emission_CO2+consum_O2+interval_CO2; 0.467, 0.765, tmp_cumulo+pH_cumulo+AN+AN_cumulo+OD+Cell_conc._int erval+rab_integral+RQ_integral+Arg_conc.+emission_O2+consum _O2+interval_CO2; 0.441, 0.765, pH_cumulo+PL_cumulo+AN+AN_c umulo+OD+Feed_Vol+Cell_conc._interval+rab_integral+RQ_integ ral+Arg_conc.+emission_O2+consum_O2+generate_CO2+interval_C O2; 0.454, 0.765, pH_cumulo+AN+AN_cumulo+OD+Feed_Vol+Cell_c onc._interval+rab_integral+RQ_integral+Arg_conc.+emission_O 2+emission_CO2+consum_O2+interval_CO2; 0.426, 0.765, pH_cum ulo+PL_cumulo+AN+AN_cumulo+interval_yield+OD+Feed_Vol+RS_cu mulo+Cell_conc._interval+rab_integral+RQ_integral+Arg_conc. +emission_O2+consum_O2+generate_CO2; 0.454, 0.765, pH_cumul o+AN+AN_cumulo+OD+Feed_Vol+RS_cumulo+Cell_conc._interval+ra b_integral+RQ_integral+Arg_conc.+emission_O2+emission_CO2+c onsum_O2; 0.454, 0.765, pH+pH_cumulo+PL_cumulo+AN+AN_cumulo +interval_yield+OD+Cell_conc._interval+Arg_conc.+emission_C O2+consum_O2+generate_CO2+interval_CO2; 0.479, 0.765, tmp+p H_cumulo+AN+AN_cumulo+OD+Cell_conc._interval+Arg_conc.+emis sion_CO2+interval_O2+generate_CO2+interval_CO2; 0.467, 0.76 5, pH_cumulo+PL+PL_cumulo+AN_cumulo+OD+Feed_Vol+Cell_conc._ interval+rab_integral+RQ_integral+Arg_conc.+emission_O2+con sum_O2; 0.479, 0.765, pH_cumulo+PL+AN+AN_cumulo+OD+Cell_con c._interval+Arg_conc.+emission_CO2+consum_O2+generate_CO2+i nterval_CO2; 0.454, 0.765, pH_cumulo+AN+AN_cumulo+OD+Feed_V ol+Cell_conc._interval+rab_integral+RQ_integral+Arg_conc.+e mission_O2+emission_CO2+consum_O2+interval_O2; 0.467, 0.765, tmp_cumulo+pH+pH_cumulo+AN+AN_cumulo+OD+Cell_conc._interva 1+Arg_conc.+emission_CO2+consum_O2+generate_CO2+interval_CO 2; 0.479, 0.765, pH_cumulo+AN+AN_cumulo+OD+Feed_Vol+Cell_co nc._interval+Arg_conc.+emission_CO2+consum_O2+generate_CO2+ interval_CO2; 0.479, 0.765, pH_cumulo+PL_cumulo+AN+AN_cumul o+OD+Cell_conc._interval+Arg_conc.+emission_CO2+interval_O2 +generate_CO2+interval_CO2; 0.454, 0.765, pH_cumulo+PL+PL_c umulo+AN+AN_cumulo+interval_yield+OD+Cell_conc._interval+Ar g_conc.+emission_CO2+consum_O2+generate_CO2+interval_CO2; 0. 440, 0.765, pH_cumulo+AN+AN_cumulo+interval_yield+OD+Feed_V ol+Cell_conc._interval+rab_integral+RQ_integral+Arg_conc.+e mission_O2+consum_O2+generate_CO2+interval_CO2; 0.440, 0.76 5, pH+pH_cumulo+PL_cumulo+AN+AN_cumulo+OD+Feed_Vol+Cell_con c._interval+rab_integral+RQ_integral+Arg_conc.+emission_O2+ emission_CO2+consum_O2; 0.411, 0.765, pH_cumulo+PL+PL_cumul o+AN+AN_cumulo+interval_yield+OD+RS+Cell_conc._interval+rab integral+RQ_integral+Arg_conc.+emission_O2+consum_O2+gener ate_CO2+interval_CO2; 0.411, 0.765, tmp+tmp_cumulo+pH_cumul o+PL_cumulo+AN+AN_cumulo+interval_yield+OD+Cell_conc._inter val+rab_integral+RQ_integral+Arg_conc.+emission_O2+consum_O 2+generate_CO2+interval_CO2; 0.426, 0.765, tmp+tmp_cumulo+p H_cumulo+PL_cumulo+AN+AN_cumulo+interval_yield+OD+Cell_conc. _interval+rab_integral+RQ_integral+Arg_conc.+emission_O2+co nsum_O2+interval_CO2; 0.479, 0.765, pH_cumulo+AN+AN_cumulo+ OD+Feed_Vol+RS_cumulo+Cell_conc._interval+RQ_integral+Arg_c onc.+emission_CO2+consum_O2; 0.426, 0.765, pH+pH_cumulo+AN+ AN_cumulo+interval_yield+OD+Feed_Vol+Cell_conc._interval+ra b_integral+RQ_integral+Arg_conc.+emission_O2+interval_O2+ge nerate_CO2+interval_CO2; 0.467, 0.765, pH_cumulo+AN+AN_cumu lo+interval_yield+OD+Cell_conc._interval+Arg_conc.+emission _CO2+consum_O2+interval_O2+generate_CO2+interval_CO2; 0.467, 0.765, tmp_cumulo+pH_cumulo+AN+AN_cumulo+OD+Feed_Vol+Cell_ conc._interval+rab_integral+RQ_integral+Arg_conc.+emission_ O2+consum_O2; 0.440, 0.765, tmp+pH_cumulo+PL_cumulo+AN+AN_c umulo+interval_yield+OD+Cell_conc._interval+rab_integral+RQ _integral+Arg_conc.+emission_CO2+generate_CO2+interval_CO2; 0.440, 0.765, pH_cumulo+PL_cumulo+AN+AN_cumulo+interval_yi eld+OD+RS+Feed_Vol+Cell_conc._interval+rab_integral+RQ_inte gral+Arg_conc.+emission_O2+consum_O2; 0.394, 0.765, tmp_cum ulo+pH+pH_cumulo+PL+PL_cumulo+AN+AN_cumulo+interval_yield+O D+Cell_conc._interval+rab_integral+RQ_integral+Arg_conc.+em ission_O2+consum_O2+interval_O2+interval_CO2; 0.491, 0.765, pH_cumulo+PL+AN_cumulo+OD+Feed_Vol+Cell_conc._interval+Arg conc.+emission_O2+emission_CO2+consum_O2; 0.491, 0.765, pH +pH_cumulo+PL_cumulo+AN_cumulo+OD+Feed_Vol+Cell_conc._inter val+Arg_conc.+emission_CO2+consum_O2; 0.491, 0.765, pH_cumu 10+PL_cumulo+AN+AN_cumulo+OD+Cell_conc._interval+Arg_conc.+ emission_CO2+consum_O2+interval_CO2; 0.467, 0.765, pH+pH_cu mulo+PL_cumulo+AN_cumulo+OD+Feed_Vol+Cell_conc._interval+ra b_integral+RQ_integral+Arg_conc.+emission_O2+consum_O2; 0.4 25, 0.765, pH_cumulo+PL_cumulo+AN+AN_cumulo+interval_yield+ OD+RS+Cell_conc._interval+rab_integral+RQ_integral+Arg_conc. +emission_O2+interval_O2+generate_CO2+interval_CO2; 0.491, 0.765, pH_cumulo+PL+AN_cumulo+OD+Feed_Vol+Cell_conc._interv al+Arg_conc.+emission_CO2+consum_O2+interval_O2; 0.466, 0.7 65, pH+pH_cumulo+PL+AN+AN_cumulo+OD+Feed_Vol+Cell_conc._int erval+rab_integral+Arg_conc.+emission_CO2+consum_O2; 0.453, 0.765, pH+pH_cumulo+PL+AN+AN_cumulo+OD+Feed_Vol+Cell_conc. _interval+rab_integral+RQ_integral+Arg_conc.+emission_CO2+c onsum_O2; 0.479, 0.765, pH_cumulo+AN+AN_cumulo+interval_yie ld+OD+Feed_Vol+Cell_conc._interval+Arg_conc.+emission_O2+em ission_CO2+consum_O2; 0.425, 0.765, tmp+tmp_cumulo+pH_cumul o+PL+AN+AN_cumulo+interval_yield+OD+Cell_conc._interval+rab _integral+RQ_integral+Arg_conc.+emission_O2+consum_O2+inter val_CO2; 0.453, 0.765, tmp+pH+pH_cumulo+AN+AN_cumulo+OD+Fee d_Vol+Cell_conc._interval+rab_integral+RQ_integral+Arg_conc. +emission_O2+consum_O2; 0.425, 0.765, pH_cumulo+PL+AN+AN_cu mulo+interval_yield+OD+Feed_Vol+Cell_conc._interval+rab_int egral+RQ_integral+Arg_conc.+emission_O2+emission_CO2+consum _O2+interval_O2; 0.453, 0.765, pH_cumulo+PL_cumulo+AN+AN_cu mulo+interval_yield+OD+Cell_conc._interval+rab_integral+RQ_ integral+Arg_conc.+emission_O2+generate_CO2+interval_CO2; 0. 466, 0.765, pH_cumulo+PL+AN+AN_cumulo+interval_yield+OD+Cel l_conc._interval+Arg_conc.+emission_CO2+consum_O2+generate_ CO2+interval_CO2; 0.440, 0.765, pH_cumulo+AN+AN_cumulo+inte rval_yield+OD+Feed_Vol+Cell_conc._interval+rab_integral+RQ_ integral+Arg_conc.+emission_O2+consum_O2+interval_O2+genera te_CO2; 0.440, 0.765, pH+pH_cumulo+PL+AN+AN_cumulo+OD+Feed_ Vol+RS_cumulo+Cell_conc._interval+rab_integral+RQ_integral+ Arg-conc.+emission_O2+consum _O2; 0.491, 0.765, pH_cumulo+AN +AN_cumulo+interval_yield+OD+Cell_conc._interval+Arg_conc.+ emission _CO2+consum _O2+interval_CO2; 0.440, 0.765, pH_cumul o+PL+AN+AN_cumulo+interval_yield+OD+Cell_conc._interval+rab _integral+RQ_integral+Arg_conc.+emission_O2+consum_O2+inter val_O2+interval_CO2; 0.440, 0.765, pH_cumulo+AN+AN_cumulo+i nterval_yield+OD+Feed_Vol+RS_cumulo+Cell_conc._interval+rab integral+RQ_integral+Arg_conc.+emission_O2+emission_CO2+co nsum_O2; 0.479, 0.765, pH_cumulo+AN+AN_cumulo+OD+Feed_Vol+C ell_conc._interval+RQ_integral+Arg_conc.+emission_CO2+consu m_O2+interval_CO2; 0.466, 0.765, tmp_cumulo+pH_cumulo+AN+AN _cumulo+interval_yield+OD+Cell_conc._interval+Arg_conc.+emi ssion_CO2+consum_O2+generate_CO2+interval_CO2; 0.425, 0.765, pH+pH_cumulo+PL+AN+AN_cumulo+interval_yield+OD+Feed_Vol+Ce ll_conc._interval+rab_integral+RQ_integral+Arg_conc.+emissi on_O2+consum_O2+generate_CO2; 0.453, 0.765, tmp_cumulo+pH_c umulo+AN+AN_cumulo+interval_yield+OD+Cell_conc._interval+ra b_integral+RQ_integral+Arg_conc.+emission_CO2+consum_O2+int erval_CO2; 0.410, 0.765, pH_cumulo+PL_cumulo+AN+AN_cumulo+i nterval_yield+OD+Feed_Vol+Cell_conc._interval+rab_integral+ RQ_integral+Arg_conc.+emission_O2+emission_CO2+consum_O2+in terval_O2+interval_CO2; 0.479, 0.765, pH+pH_cumulo+AN+AN_cu mulo+OD+Feed_Vol+RS_cumulo+Cell_conc._interval+Arg_conc.+em ission_CO2+consum_O2; 0.440, 0.765, pH_cumulo+PL_cumulo+AN+ AN_cumulo+interval_yield+OD+Cell_conc._interval+rab_integra l+RQ_integral+Arg_conc.+emission_CO2+interval_O2+generate_C O2+interval_CO2; 0.466, 0.765, pH_cumulo+PL+AN+AN_cumulo+in terval_yield+OD+Cell_conc._interval+rab_integral+RQ_integra 1+Arg_conc.+emission_O2+consum_O2; 0.425, 0.765, pH_cumulo+ PL+AN+AN_cumulo+interval_yield+OD+RS_cumulo+Cell_conc._inte rval+rab_integral+RQ_integral+Arg_conc.+emission_O2+consum_ O2+interval_O2+interval_CO2; 0.453, 0.765, tmp_cumulo+pH_cu mulo+PL_cumulo+AN+AN_cumulo+interval_yield+OD+Cell_conc._in terval+Arg_conc.+emission_CO2+consum_O2+generate_CO2+interv al_CO2; 0.410, 0.765, pH+pH_cumulo+PL_cumulo+AN+AN_cumulo+i nterval_yield+OD+RS+Cell_conc._interval+rab_integral+RQ_int egral+Arg_conc.+emission_O2+consum_O2+generate_CO2+interval _CO2; 0.466, 0.765, tmp_cumulo+pH+pH_cumulo+AN+AN_cumulo+OD +Cell_conc._interval+Arg_conc.+emission_CO2+interval_O2+gen erate_CO2+interval_CO2; 0.502, 0.765, tmp+pH_cumulo+AN_cumu lo+OD+Feed_Vol+Cell_conc._interval+Arg_conc.+emission_CO2+c onsum_O2; 0.425, 0.765, pH+pH_cumulo+AN+AN_cumulo+interval_ yield+OD+Cell_conc._interval+rab_integral+RQ_integral+Arg_c onc.+emission_O2+consum_O2+interval_O2+generate_CO2+interva l_CO2; 0.453, 0.765, pH_cumulo+AN+AN_cumulo+interval_yield+ OD+Feed_Vol+Cell_conc._interval+rab_integral+RQ_integral+Ar g_conc.+emission_CO2+consum_O2+generate_CO2; 0.410, 0.765, pH+pH_cumulo+PL+AN+AN_cumulo+interval_yield+OD+Feed_Vol+RS_ cumulo+Cell_conc._interval+rab_integral+RQ_integral+Arg_con c.+emission_O2+consum_O2+interval_CO2; 0.410, 0.765, tmp_cu mulo+pH_cumulo+PL_cumulo+AN+AN_cumulo+interval_yield+OD+Cel l_conc._interval+rab_integral+RQ_integral+Arg_conc.+emissio n_O2+emission_CO2+consum_O2+generate_CO2+interval_CO2; 0.45 3, 0.765, tmp+pH_cumulo+PL+AN+AN_cumulo+OD+Feed_Vol+Cell_co nc._interval+rab_integral+RQ_integral+Arg_conc.+emission_O2 +consum_O2; 0.466, 0.765, pH_cumulo+PL_cumulo+AN+AN_cumulo+ OD+Feed_Vol+Cell_conc._interval+rab_integral+RQ_integral+Ar g_conc.+emission_CO2+consum_O2; 0.466, 0.765, pH_cumulo+AN+ AN_cumulo+OD+Feed_Vol+Cell_conc._interval+RQ_integral+Arg_c onc.+emission_CO2+consum_O2+interval_O2+generate_CO2; 0.490, 0.765, pH_cumulo+PL+AN_cumulo+OD+Feed_Vol+Cell_conc._inter val+Arg_conc.+emission_CO2+consum_O2+generate_CO2; 0.502, 0. 765, pH_cumulo+AN_cumulo+OD+Feed_Vol+Cell_conc._interval+ra b integral+Arg_conc.+emission_CO2+consum_O2; 0.410, 0.765, pH_cumulo+PL+PL_cumulo+AN+AN_cumulo+interval_yield+OD+RS_cu mulo+Cell_conc._interval+rab_integral+RQ_integral+Accum._Yi eld+Arg_conc.+emission_O2+consum_O2+interval_CO2; 0.425, 0. 765, tmp_cumulo+pH_cumulo+PL_cumulo+AN+AN_cumulo+interval_y ield+OD+Feed_Vol+Cell_conc._interval+rab_integral+RQ_integr al+Arg_conc.+emission_O2+consum_O2+interval_CO2; 0.439, 0.7 65, pH_cumulo+PL+AN+AN_cumulo+interval_yield+OD+Cell_conc._ interval+rab_integral+RQ_integral+Arg_conc.+emission_O2+con sum_O2+generate_CO2+interval_CO2; 0.490, 0.765, tmp+pH_cumu lo+AN+AN_cumulo+OD+Cell_conc._interval+Arg_conc.+emission_C O2+consum_O2+interval_CO2; 0.425, 0.765, pH_cumulo+PL+AN+AN _cumulo+interval_yield+OD+Feed_Vol+RS_cumulo+Cell_conc._int erval+rab_integral+RQ_integral+Arg_conc.+emission_O2+consum _O2+interval_O2; 0.439, 0.765, tmp_cumulo+pH_cumulo+AN+AN_c umulo+interval_yield+OD+Cell_conc._interval+rab_integral+RQ _integral+Arg_conc.+emission_O2+interval_O2+generate_CO2+in terval_CO2; 0.439, 0.765, tmp_cumulo+pH_cumulo+PL+PL_cumulo +AN+AN_cumulo+OD+Cell_conc._interval+rab_integral+RQ_integr al+Arg_conc.+emission_O2+consum_O2+interval_CO2; 0.439, 0.7 65, tmp+pH_cumulo+PL+PL_cumulo+AN+AN_cumulo+OD+Feed_Vol+Cel l_conc._interval+rab_integral+RQ_integral+Arg_conc.+emissio n_O2+consum_O2; 0.466, 0.765, tmp_cumulo+pH_cumulo+PL+AN+AN _cumulo+OD+Cell_conc._interval+Arg_conc.+emission_CO2+consu m_O2+generate_CO2+interval_CO2; 0.425, 0.765, tmp+pH_cumulo +PL_cumulo+AN+AN_cumulo+interval_yield+OD+Feed_Vol+Cell_con c._interval+rab_integral+RQ_integral+Arg_conc.+emission_O2+ consum_O2+interval_CO2; 0.410, 0.765, pH_cumulo+PL_cumulo+A N+AN_cumulo+interval_yield+OD+Feed_Vol+RS_cumulo+Cell_conc. _interval+rab_integral+RQ_integral+Arg_conc.+emission_O2+co nsum_O2+interval_O2+interval_CO2; 0.478, 0.765, pH+pH_cumul o+AN_cumulo+OD+Feed_Vol+Cell_conc._interval+rab_integral+RQ _integral+Arg_conc.+emission_O2+consum_O2; 0.394, 0.764, tm p+pH+pH_cumulo+PL+PL_cumulo+AN+AN_cumulo+interval_yield+OD+ Cell_conc._interval+rab_integral+RQ_integral+Arg_conc.+emis sion_O2+consum_O2+interval_O2+interval_CO2; 0.501, 0.764, p H_cumulo+AN_cumulo+OD+Feed_Vol+Cell_conc._interval+RQ_integ ral+Arg_conc.+emission_O2+consum_O2; 0.439, 0.764, pH_cumul o+PL_cumulo+AN+AN_cumulo+OD+Feed_Vol+RS_cumulo+Cell_conc._i nterval+rab_integral+RQ_integral+Arg_conc.+emission_O2+cons um_O2+interval_CO2; 0.490, 0.764, pH_cumulo+PL+AN_cumulo+OD +Feed_Vol+RS_cumulo+Cell_conc._interval+Arg_conc.+emission_ CO2+consum_O2; 0.409, 0.764, tmp_cumulo+pH+pH_cumulo+PL_cum ulo+AN+AN_cumulo+interval_yield+OD+Cell_conc._interval+rab_ integral+RQ_integral+Arg_conc.+emission_O2+interval_O2+gene rate_CO2+interval_CO2; 0.466, 0.764, pH_cumulo+AN+AN_cumulo +interval_yield+OD+Cell_conc._interval+rab_integral+RQ_inte gral+Arg_conc.+emission_CO2+consum_O2+interval_CO2; 0.424, 0.764, pH_cumulo+PL+AN+AN_cumulo+interval_yield+OD+Cell_con c._interval+rab_integral+RQ_integral+Arg_conc.+emission_O2+ consum_O2+interval_O2+generate_CO2+interval_CO2; 0.409, 0.7 64, tmp+pH+pH_cumulo+PL_cumulo+AN+AN_cumulo+interval_yield+ OD+Feed_Vol+Cell_conc._interval+rab_integral+RQ_integral+Ar g_conc.+emission_O2+consum _O2+interval_O2; 0.424, 0.764, pH _cumulo+PL+AN+AN_cumulo+interval_yield+OD+Feed_Vol+Cell_con c._interval+rab_integral+RQ_integral+Arg_conc.+emission_O2+ emission_CO2+consum_O2+generate_CO2; 0.465, 0.764, pH+pH_cu mulo+PL+AN+AN_cumulo+OD+Feed_Vol+Cell_conc._interval+Arg_co nc.+emission_CO2+consum_O2+interval_CO2; 0.478, 0.764, pH_c umulo+PL_cumulo+AN+AN_cumulo+OD+Cell_conc._interval+Arg-con c.+emission_CO2+consum_O2+generate_CO2+interval_CO2; 0.465, 0.764, pH_cumulo+PL_cumulo+AN+AN_cumulo+OD+Feed_Vol+Cell_c onc._interval+RQ_integral+Arg_conc.+emission_CO2+consum_O2+ interval_O2; 0.452, 0.764, pH_cumulo+AN+AN_cumulo+interval_ yield+OD+Feed_Vol+Cell_conc._interval+rab_integral+RQ_integ ral+Arg_conc.+emission_O2+interval_O2+generate_CO2; 0.478, 0.764, pH+pH_cumulo+AN+AN_cumulo+OD+Cell_conc._interval+Arg conc.+emission_CO2+consum_O2+generate_CO2+interval_CO2; 0. 465, 0.764, pH_cumulo+PL_cumulo+AN+AN_cumulo+OD+Feed_Vol+Ce ll_conc._interval+RQ_integral+Arg_conc.+emission_CO2+consum _O2+generate_CO2; 0.439, 0.764, pH_cumulo+AN+AN_cumulo+inte rval_yield+OD+Feed_Vol+Cell_conc._interval+rab_integral+RQ_ integral+Accum._Yield+Arg_conc.+emission_O2+emission_CO2+co nsum_O2; 0.424, 0.764, pH_cumulo+PL+AN+AN_cumulo+interval_y ield+OD+Feed_Vol+Cell_conc._interval+rab_integral+RQ_integr al+Accum._Yield+Arg_conc.+emission_O2+emission_CO2+consum_O 2; 0.465, 0.764, pH_cumulo+AN+AN_cumulo+OD+Cell_conc._inter val+rab_integral+RQ_integral+Arg_conc.+emission_O2+interval _O2+generate_CO2+interval_CO2; 0.439, 0.764, tmp+pH_cumulo+ PL_cumulo+AN+AN_cumulo+OD+Feed_Vol+Cell_conc._interval+rab_ integral+RQ_integral+Arg_conc.+emission_O2+consum_O2+interv al_CO2; 0.501, 0.764, pH_cumulo+AN_cumulo+OD+Feed_Vol+Cell_ conc._interval+Arg_conc.+emission_CO2+interval_O2+generate_ CO2; 0.478, 0.764, pH_cumulo+AN_cumulo+interval_yield+OD+Fe ed_Vol+Cell_conc._interval+rab_integral+RQ_integral+Arg_con c.+emission_O2+consum_O2; 0.501, 0.764, pH_cumulo+AN_cumulo +OD+Feed_Vol+Cell_conc._interval+RQ_integral+Arg_conc.+emis sion_CO2+consum_O2; 0.452, 0.764, pH_cumulo+AN+AN_cumulo+OD +Feed_Vol+Cell_conc._interval+rab_integral+RQ_integral+Arg_ conc.+emission_O2+interval_O2+generate_CO2+interval_CO2; 0. 438, 0.764, pH_cumulo+AN+AN_cumulo+interval_yield+OD+RS_cum ulo+Cell_conc._interval+rab_integral+RQ_integral+Arg_conc.+ emission_O2+interval_O2+generate_CO2+interval_CO2; 0.438, 0. 764, tmp_cumulo+pH_cumulo+PL_cumulo+AN+AN_cumulo+OD+Feed_Vo l+Cell_conc._interval+rab_integral+RQ_integral+Arg_conc.+em ission_O2+consum_O2+interval_CO2; 0.424, 0.764, pH_cumulo+P L+AN+AN_cumulo+interval_yield+OD+Cell_conc._interval+rab_in tegral+RQ_integral+Arg_conc.+emission_O2+emission_CO2+inter val_O2+generate_CO2+interval_CO2; 0.409, 0.764, tmp_cumulo+ pH_cumulo+PL+PL_cumulo+AN+AN_cumulo+interval_yield+OD+Feed_ Vol+Cell_conc._interval+rab_integral+RQ_integral+Arg_conc.+ emission_O2+consum_O2+interval_CO2; 0.424, 0.764, pH_cumulo +PL_cumulo+AN+AN_cumulo+interval_yield+OD+RS+Feed_Vol+Cell_ conc._interval+rab_integral+RQ_integral+Arg_conc.+emission_ O2+consum_O2+interval_CO2; 0.452, 0.764, pH_cumulo+AN+AN_cu mulo+interval_yield+OD+Cell_conc._interval+rab_integral+RQ_ integral+Arg_conc.+emission_O2+consum_O2+generate_CO2+inter val_CO2; 0.438, 0.764, pH_cumulo+PL+PL_cumulo+AN+AN_cumulo+ OD+Feed_Vol+Cell_conc._interval+rab_integral+RQ_integral+Ar g_conc.+emission_O2+emission_CO2+consum_O2; 0.477, 0.764, p H_cumulo+PL_cumulo+AN+AN_cumulo+OD+Feed_Vol+Cell_conc._inte rval+Arg_conc.+emission_CO2+consum_O2+interval_O2; 0.452, 0. 764, tmp_cumulo+pH_cumulo+AN+AN_cumulo+OD+Feed_Vol+Cell_con c._interval+rab_integral+RQ_integral+Arg_conc.+emission_O2+ consum_O2+interval_CO2; 0.452, 0.764, pH_cumulo+AN+AN_cumul o+interval_yield+OD+Feed_Vol+Cell_conc._interval+rab_integr al+RQ_integral+Arg_conc.+emission_CO2+consum_O2+interval_O 2; 0.452, 0.764, tmp_cumulo+pH+pH_cumulo+AN+AN_cumulo+OD+Ce ll_conc._interval+rab_integral+RQ_integral+Arg_conc.+emissi on_CO2+consum_O2+interval_CO2; 0.424, 0.764, pH_cumulo+PL+P L_cumulo+AN+AN_cumulo+OD+Feed_Vol+RS_cumulo+Cell_conc._inte rval+rab_integral+RQ_integral+Arg_conc.+emission_O2+consum_ O2+interval_CO2; 0.477, 0.764, pH_cumulo+PL_cumulo+AN+AN_cu mulo+OD+Feed_Vol+Cell_conc._interval+Arg_conc.+emission_CO2 +consum_O2+generate_CO2; 0.438, 0.764, tmp_cumulo+pH_cumulo +PL+AN+AN_cumulo+interval_yield+OD+Feed_Vol+Cell_conc._inte rval+rab_integral+RQ_integral+Arg_conc.+emission_O2+consum_ O2; 0.393, 0.764, pH+pH_cumulo+PL_cumulo+AN+AN_cumulo+inter val_yield+OD+Feed_Vol+Cell_conc._interval+rab_integral+RQ_i ntegral+Arg_conc.+emission_O2+emission_CO2+consum_O2+genera te_CO2+interval_CO2; 0.438, 0.764, tmp_cumulo+pH_cumulo+AN+ AN_cumulo+interval_yield+OD+Feed_Vol+Cell_conc._interval+ra b_integral+RQ_integral+Arg_conc.+emission_O2+consum_O2+inte rval_CO2; 0.465, 0.764, pH+pH_cumulo+PL+AN+AN_cumulo+OD+Fee d_Vol+Cell_conc._interval+Arg_conc.+emission_02+emission_CO 2+consum_O2; 0.452, 0.764, pH+pH_cumulo+AN+AN_cumulo+OD+Fee d_Vol+Cell_conc._interval+RQ_integral+Arg_conc.+emission_O2 +emission_CO2+consum_O2+generate_CO2; 0.477, 0.764, tmp_cum ulo+pH_cumulo+AN+AN_cumulo+OD+Cell_conc._interval+rab_integ ral+Arg_conc.+emission_CO2+consum_O2+interval_CO2; 0.438, 0. 764, tmp_cumulo+pH_cumulo+AN+AN_cumulo+interval_yield+OD+Ce ll_conc._interval+rab_integral+RQ_integral+Arg_conc.+emissi on_O2+consum_O2+generate_CO2+interval_CO2; 0.452, 0.764, tm p_cumulo+pH_cumulo+PL+AN+AN_cumulo+OD+Cell_conc._interval+r ab_integral+RQ_integral+Arg_conc.+emission_CO2+consum_O2+in terval_CO2; 0.438, 0.764, tmp+tmp_cumulo+pH_cumulo+AN+AN_cu mulo+interval_yield+OD+Cell_conc._interval+rab_integral+RQ_ integral+Arg_conc.+emission_O2+consum_O2+interval_CO2; 0.46 5, 0.764, tmp_cumulo+pH+pH_cumulo+PL+AN+AN_cumulo+OD+Cell_c onc._interval+Arg_conc.+emission_CO2+consum_O2+interval_CO 2; 0.465, 0.764, pH+pH_cumulo+PL_cumulo+AN+AN_cumulo+interv al_yield+OD+Cell_conc._interval+Arg_conc.+emission_CO2+gene rate_CO2+interval_CO2; 0.477, 0.764, pH_cumulo+PL_cumulo+AN +AN_cumulo+OD+Feed_Vol+Cell_conc._interval+rab_integral+Arg _conc.+emission_CO2+consum_O2; 0.424, 0.764, tmp_cumulo+pH_ cumulo+PL+AN+AN_cumulo+interval_yield+OD+Cell_conc._interva 1+rab_integral+RQ_integral+Arg_conc.+emission_O2+emission_C O2+consum_O2+interval_CO2; 0.452, 0.764, pH+pH_cumulo+PL+AN +AN_cumulo+OD+Feed_Vol+Cell_conc._interval+RQ_integral+Arg_ conc.+emission_O2+emission_CO2+consum_O2; 0.423, 0.764, pH_ cumulo+PL_cumulo+AN+AN_cumulo+interval_yield+OD+Cell_conc._ interval+rab_integral+RQ_integral+Accum._Yield+Arg_conc.+em ission_O2+consum_O2+generate_CO2+interval_CO2; 0.465, 0.764, pH_cumulo+PL+AN+AN_cumulo+OD+Cell_conc._interval+rab_integ ral+RQ_integral+Arg_conc.+emission_O2+interval_O2+interval_ CO2; 0.423, 0.764, tmp_cumulo+pH+pH_cumulo+PL_cumulo+AN+AN_ cumulo+interval_yield+OD+Cell_conc._interval+rab_integral+R Q_integral+Arg_conc.+emission_CO2+generate_CO2+interval_CO 2; 0.438, 0.764, pH+pH_cumulo+PL+AN+AN_cumulo+interval_yiel d+OD+Feed_Vol+Cell_conc._interval+rab_integral+RQ_integral+ Arg_conc.+emission_CO2+consum_O2; 0.408, 0.764, tmp+pH+pH_c umulo+PL_cumulo+AN+AN_cumulo+interval_yield+OD+Cell_conc._i nterval+rab_integral+RQ_integral+Arg_conc.+emission_O2+inte rval_O2+generate_CO2+interval_CO2; 0.477, 0.764, tmp+tmp_cu mulo+pH_cumulo+AN+AN_cumulo+OD+Cell_conc._interval+Arg_conc. +emission_CO2+consum_O2+interval_CO2; 0.477, 0.764, pH_cumu lo+PL_cumulo+AN+AN_cumulo+OD+Feed_Vol+Cell_conc._interval+A rg_conc.+emission_CO2+consum_O2+interval_CO2; 0.423, 0.764, tmp_cumulo+pH_cumulo+PL_cumulo+AN+AN_cumulo+interval_yield +OD+Cell_conc._interval+rab_integral+RQ_integral+Arg_conc.+ emission_O2+emission_CO2+consum_O2+interval_CO2; 0.423, 0.7 64, pH_cumulo+PL+AN+AN_cumulo+interval_yield+OD+RS+Feed_Vol +RS_cumulo+Cell_conc._interval+rab_integral+RQ_integral+Arg _conc.+emission_O2+consum_O2; 0.438, 0.764, tmp+pH+pH_cumul o+PL_cumulo+AN+AN_cumulo+OD+Feed_Vol+Cell_conc._interval+ra b_integral+RQ_integral+Arg_conc.+emission_O2+consum_O2; 0.4 77, 0.764, tmp+pH+pH_cumulo+AN+AN_cumulo+OD+Feed_Vol+Cell_c onc._interval+Arg_conc.+emission_CO2+consum_O2; 0.408, 0.76 4, pH_cumulo+PL+PL_cumulo+AN+AN_cumulo+interval_yield+OD+Ce ll_conc._interval+rab_integral+RQ_integral+Accum._Yield+Arg _conc.+emission_O2+consum_O2+generate_CO2+interval_CO2; 0.4 38, 0.764, pH_cumulo+PL+PL_cumulo+AN+AN_cumulo+interval_yie ld+OD+Cell_conc._interval+rab_integral+RQ_integral+Arg_conc. +emission_O2+generate_CO2+interval_CO2; 0.464, 0.764, pH_cu mulo+AN+AN_cumulo+interval_yield+OD+Cell_conc._interval+RQ_ integral+Arg_conc.+emission_CO2+interval_O2+generate_CO2+in terval_CO2; 0.438, 0.764, tmp_cumulo+pH_cumulo+PL+AN+AN_cum ulo+interval_yield+OD+Cell_conc._interval+rab_integral+RQ_i ntegral+Arg_conc.+emission_CO2+consum_O2+interval_CO2; 0.40 8, 0.764, pH+pH_cumulo+PL_cumulo+AN+AN_cumulo+interval_yiel d+OD+Cell_conc._interval+rab_integral+RQ_integral+Arg_conc. +emission_O2+emission_CO2+interval_O2+generate_CO2+interval _CO2; 0.477, 0.764, pH_cumulo+AN+AN_cumulo+interval_yield+0 D+Feed_Vol+Cell_conc._interval+RQ_integral+Arg_conc.+emissi on_CO2+consum_O2; 0.489, 0.764, pH_cumulo+AN+AN_cumulo+OD+F eed_Vol+Cell_conc._interval+Arg_conc.+emission_CO2+interval _O2+generate_CO2; 0.477, 0.764, tmp_cumulo+pH_cumulo+PL+AN+ AN_cumulo+OD+Feed_Vol+Cell_conc._interval+Arg_conc.+emissio n_CO2+consum_O2; 0.392, 0.764, pH+pH_cumulo+PL+PL_cumulo+AN +AN_cumulo+interval_yield+OD+Cell_conc._interval+rab_integr al+RQ_integral+Arg_conc.+emission_O2+consum_O2+interval_O2+ generate_CO2+interval_CO2; 0.408, 0.764, tmp+pH_cumulo+PL+P L_cumulo+AN+AN_cumulo+interval_yield+OD+Cell_conc._interval +rab_integral+RQ_integral+Arg_conc.+emission_O2+interval_O2 +generate_CO2+interval_CO2; 0.408, 0.764, pH_cumulo+PL+PL_c umulo+AN+AN_cumulo+interval_yield+OD+RS+Feed_Vol+Cell_conc. _interval+rab_integral+RQ_integral+Arg_conc.+emission_O2+co nsum_O2+interval_CO2; 0.408, 0.764, pH_cumulo+PL+AN+AN_cumu lo+interval_yield+OD+Feed_Vol+RS_cumulo+Cell_conc._interval +rab_integral+RQ_integral+Arg_conc.+emission_O2+emission_CO 2+consum_O2+interval_CO2; 0.423, 0.764, pH_cumulo+PL+PL_cum ulo+AN+AN_cumulo+interval_yield+OD+RS+Feed_Vol+Cell_conc._i nterval+rab_integral+RQ_integral+Arg_conc.+emission_O2+cons um_O2; 0.464, 0.764, pH_cumulo+PL_cumulo+AN+AN_cumulo+OD+Fe ed_Vol+Cell_conc._interval+RQ_integral+Arg_conc.+emission_O 2+emission_CO2+consum_O2; 0.451, 0.764, pH_cumulo+PL_cumulo +AN+AN_cumulo+interval_yield+OD+Feed_Vol+Cell_conc._interva 1+Arg_conc.+emission_CO2+consum_O2+generate_CO2+interval_CO 2; 0.477, 0.764, pH_cumulo+AN+AN_cumulo+OD+Cell_conc._inter val+Arg_conc.+emission_O2+emission_CO2+interval_O2+generate _CO2+interval_CO2; 0.437, 0.764, pH+pH_cumulo+AN+AN_cumulo+ interval_yield+OD+Feed_Vol+Cell_conc._interval+rab_integral +RQ_integral+Accum._Yield+Arg_conc.+emission_O2+consum_O2; 0.423, 0.764, pH+pH_cumulo+PL+AN+AN_cumulo+interval_yield+O D+Feed_Vol+Cell_conc._interval+rab_integral+RQ_integral+Acc um._Yield+Arg_conc.+emission_O2+consum_O2; 0.408, 0.764, pH +pH_cumulo+PL_cumulo+AN+AN_cumulo+interval_yield+OD+RS_cumu lo+Cell_conc._interval+rab_integral+RQ_integral+Arg_conc.+e mission_O2+interval_O2+generate_CO2+interval_CO2; 0.408, 0. 764, tmp_cumulo+pH_cumulo+PL+PL_cumulo+AN+AN_cumulo+interva l_yield+OD+RS_cumulo+Cell_conc._interval+rab_integral+RQ_in tegral+Arg_conc.+emission_O2+consum_O2+interval_CO2; 0.437, 0.764, pH+pH_cumulo+PL+AN+AN_cumulo+OD+Feed_Vol+Cell_conc. _interval+rab_integral+RQ_integral+Arg_conc.+emission_O2+co nsum_O2+interval_O2; 0.451, 0.764, pH+pH_cumulo+PL_cumulo+A N+AN_cumulo+OD+Cell_conc._interval+rab_integral+RQ_integral +Arg_conc.+emission_O2+consum_O2+interval_CO2; 0.437, 0.764, tmp+pH+pH_cumulo+AN+AN_cumulo+interval_yield+OD+Feed_Vol+C ell_conc._interval+rab_integral+RQ_integral+Arg_conc.+emiss ion_O2+consum_O2; 0.476, 0.764, pH_cumulo+PL+AN_cumulo+OD+F eed_Vol+Cell_conc._interval+rab_integral+RQ_integral+Arg_co nc.+emission_O2+consum_O2; 0.408, 0.764, pH_cumulo+PL+PL_cu mulo+AN+AN_cumulo+interval_yield+OD+RS+Cell_conc._interval+ rab_integral+RQ_integral+Arg_conc.+emission_O2+consum_O2+in terval_O2+interval_CO2; 0.451, 0.764, pH_cumulo+AN+AN_cumul o+OD+Feed_Vol+Cell_conc._interval+rab_integral+RQ_integral+ Arg_conc.+emission_O2+consum_O2+interval_O2+generate_CO2; 0. 488, 0.764, pH+pH_cumulo+AN_cumulo+OD+Feed_Vol+Cell_conc._i nterval+Arg_conc.+emission_CO2+consum_O2+interval_O2; 0.423, 0.764, pH_cumulo+PL+AN+AN_cumulo+interval_yield+OD+Feed_Vo l+RS_cumulo+Cell_conc._interval+rab_integral+RQ_integral+Ar g_conc.+emission_O2+consum_O2+generate_CO2; 0.451, 0.764, t mp+pH_cumulo+PL_cumulo+AN+AN_cumulo+OD+Cell_conc._interval+ rab_integral+RQ_integral+Arg_conc.+emission_O2+consum_O2+in terval_CO2; 0.476, 0.764, pH_cumulo+PL+AN+AN_cumulo+OD+Cell _conc._interval+Arg_conc.+emission_CO2+consum_O2+interval_O 2+interval_CO2; 0.451, 0.764, pH+pH_cumulo+AN+AN_cumulo+OD+ Feed_Vol+Cell_conc._interval+RQ_integral+Arg_conc.+emission _O2+emission_CO2+consum_O2+interval_O2; 0.437, 0.764, pH_cu mulo+AN+AN_cumulo+interval_yield+OD+Feed_Vol+RS_cumulo+Cell _conc._interval+rab_integral+RQ_integral+Arg_conc.+emission _O2+consum_O2+interval_CO2; 0.451, 0.764, pH_cumulo+PL_cumu lo+AN+AN_cumulo+OD+RS+Feed_Vol+Cell_conc._interval+rab_inte gral+RQ_integral+Arg_conc.+emission_O2+consum_O2; 0.451, 0. 764, tmp_cumulo+pH_cumulo+AN+AN_cumulo+interval_yield+OD+Fe ed_Vol+Cell_conc._interval+rab_integral+RQ_integral+Arg_con c.+emission_O2+consum_O2; 0.464, 0.764, pH_cumulo+AN+AN_cum ulo+OD+Feed_Vol+Cell_conc._interval+rab_integral+RQ_integra 1+Arg_conc.+emission_CO2+consum_O2+generate_CO2; 0.423, 0.7 63, pH+pH_cumulo+AN+AN_cumulo+interval_yield+OD+Feed_Vol+Ce ll_conc._interval+rab_integral+RQ_integral+Arg_conc.+emissi on_O2+emission_CO2+consum_O2+generate_CO2; 0.451, 0.763, pH _cumulo+PL+AN+AN_cumulo+OD+RS_cumulo+Cell_conc._interval+ra b_integral+RQ_integral+Arg_conc.+emission_O2+interval_O2+in terval_CO2; 0.464, 0.763, pH_cumulo+AN+AN_cumulo+interval_y ield+OD+Feed_Vol+Cell_conc._interval+Arg_conc.+emission_CO2 +interval_O2+generate_CO2+interval_CO2; 0.451, 0.763, tmp_c umulo+pH+pH_cumulo+AN+AN_cumulo+OD+Cell_conc._interval+rab_ integral+RQ_integral+Arg_conc.+emission_O2+consum_O2+interv al_CO2; 0.464, 0.763, tmp_cumulo+pH_cumulo+PL+AN+AN_cumulo+ OD+Feed_Vol+Cell_conc._interval+Arg_conc.+emission_CO2+cons um_O2+interval_CO2; 0.476, 0.763, pH_cumulo+PL_cumulo+AN+AN _cumulo+OD+Feed_Vol+Cell_conc._interval+Arg_conc.+emission_ O2+emission_CO2+consum_O2; 0.464, 0.763, pH_cumulo+PL+AN+AN _cumulo+interval_yield+OD+Feed_Vol+Cell_conc._interval+RQ_i ntegral+Arg_conc.+emission_CO2+consum_O2; 0.488, 0.763, pH_ cumulo+AN_cumulo+OD+Feed_Vol+Cell_conc._interval+RQ_integra 1+Arg_conc.+emission_O2+emission_CO2+consum_O2; 0.488, 0.76 3, pH_cumulo+PL_cumulo+AN_cumulo+OD+Feed_Vol+Cell_conc._int erval+Arg_conc.+emission_CO2+consum_O2+generate_CO2; 0.464, 0.763, tmp_cumulo+pH_cumulo+PL+AN+AN_cumulo+OD+Cell_conc._ interval+Arg_conc.+emission_CO2+interval_O2+generate_CO2+in terval_CO2; 0.476, 0.763, tmp+pH_cumulo+PL+AN+AN_cumulo+OD+ Cell_conc._interval+Arg_conc.+emission_CO2+consum_O2+interv al_CO2; 0.407, 0.763, pH_cumulo+PL_cumulo+AN+AN_cumulo+inte rval_yield+OD+RS+Feed_Vol+Cell_conc._interval+rab_integral+ RQ_integral+Arg_conc.+emission_O2+consum_O2+generate_CO2+in terval_CO2; 0.450, 0.763, pH+pH_cumulo+PL_cumulo+AN_cumulo+ interval_yield+OD+Feed_Vol+Cell_conc._interval+rab_integral +RQ_integral+Arg_conc.+emission_O2+consum_O2; 0.437, 0.763, pH_cumulo+PL_cumulo+AN+AN_cumulo+OD+Feed_Vol+Cell_conc._in terval+rab_integral+RQ_integral+Arg_conc.+emission_O2+consu m_O2+interval_O2+interval_CO2; 0.476, 0.763, tmp+pH_cumulo+ AN+AN_cumulo+OD+Feed_Vol+Cell_conc._interval+RQ_integral+Ar g_conc.+emission_CO2+consum_O2; 0.463, 0.763, pH+pH_cumulo+ PL+AN+AN_cumulo+OD+Cell_conc._interval+Arg_conc.+emission_C O2+interval_O2+generate_CO2+interval_CO2; 0.463, 0.763, pH_ cumulo+PL+PL_cumulo+AN+AN_cumulo+interval_yield+OD+Cell_con c._interval+Arg_conc.+emission_CO2+generate_CO2+interval_CO 2; 0.407, 0.763, pH+pH_cumulo+PL+PL_cumulo+AN+AN_cumulo+int erval_yield+OD+Feed_Vol+Cell_conc._interval+rab_integral+RQ _integral+Arg_conc.+emission_O2+emission_CO2+consum_O2; 0.4 76, 0.763, pH_cumulo+PL_cumulo+AN+AN_cumulo+interval_yield+ OD+Feed_Vol+Cell_conc._interval+Arg_conc.+emission_CO2+cons um_O2; 0.407, 0.763, tmp+pH+pH_cumulo+PL+PL_cumulo+AN+AN_cu mulo+interval_yield+OD+Feed_Vol+Cell_conc._interval+rab_int egral+RQ_integral+Arg_conc.+emission_O2+consum_O2; 0.422, 0. 763, pH_cumulo+PL+AN+AN_cumulo+interval_yield+OD+RS_cumulo+ Cell_conc._interval+rab_integral+RQ_integral+Arg_conc.+emis sion_O2+consum_O2+generate_CO2+interval_CO2; 0.476, 0.763, pH_cumulo+AN+AN_cumulo+OD+Feed_Vol+Cell_conc._interval+RQ_i ntegral+Arg_conc.+emission_CO2+interval_O2+generate_CO2; 0. 407, 0.763, pH+pH_cumulo+PL_cumulo+AN+AN_cumulo+interval_yi eld+OD+Cell_conc._interval+rab_integral+RQ_integral+Arg_con c.+emission_O2+emission_CO2+consum_O2+interval_O2+interval CO2; 0.422, 0.763, pH_cumulo+PL+PL_cumulo+AN+AN_cumulo+inte rval_yield+OD+Cell_conc._interval+rab_integral+RQ_integral+ Arg_conc.+emission_CO2+consum_O2+generate_CO2+interval_CO2; 0.436, 0.763, pH_cumulo+PL+PL_cumulo+AN+AN_cumulo+interval _yield+OD+Cell_conc._interval+rab_integral+RQ_integral+Arg_ conc.+emission_CO2+consum_O2+interval_CO2; 0.422, 0.763, pH _cumulo+AN+AN_cumulo+interval_yield+OD+Feed_Vol+Cell_conc._ interval+rab_integral+RQ_integral+Arg_conc.+emission_O2+emi ssion_CO2+interval_O2+generate_CO2+interval_CO2; 0.422, 0.7 63, tmp_cumulo+pH_cumulo+PL_cumulo+AN+AN_cumulo+interval_yi eld+OD+Feed_Vol+Cell_conc._interval+rab_integral+RQ_integra l+Arg_conc.+emission_O2+consum_O2+interval_O2; 0.463, 0.763, pH+pH_cumulo+PL+AN+AN_cumulo+OD+Feed_Vol+RS_cumulo+Cell_co nc._interval+Arg_conc.+emission _CO2+consum _O2; 0.391, 0.763, pH+pH_cumulo+PL+PL_cumulo+AN+AN_cumulo+interval_yield+OD+F eed_Vol+Cell_conc._interval+rab_integral+RQ_integral+Arg_co nc.+emission_O2+consum_O2+interval_O2+generate_CO2; 0.476, 0.763, pH_cumulo+PL_cumulo+AN+AN_cumulo+OD+Feed_Vol+Cell_co nc._interval+RQ_integral+Arg_conc.+emission_O2+consum_O2; 0. 422, 0.763, pH_cumulo+PL+AN+AN_cumulo+interval_yield+OD+Fee d_Vol+Cell_conc._interval+rab_integral+RQ_integral+Arg_conc. +emission_O2+consum_O2+generate_CO2+interval_CO2; 0.436, 0. 763, pH_cumulo+PL_cumulo+AN+AN_cumulo+interval_yield+OD+Cel l_conc._interval+rab_integral+RQ_integral+Arg_conc.+emissio n_CO2+consum_O2+interval_O2+interval_CO2; 0.488, 0.763, pH_ umulo+PL_cumulo+AN_cumulo+OD+Feed_Vol+Cell_conc._interval+ Arg_conc.+emission_CO2+consum_O2+interval_O2; 0.463, 0.763, pH+pH_cumulo+PL+AN+AN_cumulo+OD+Feed_Vol+Cell_conc._interv al+RQ_integral+Arg_conc.+emission_O2+consum_O2; 0.436, 0.76 3, pH+pH_cumulo+PL+PL_cumulo+AN+AN_cumulo+interval_yield+OD +Cell_conc._interval+rab_integral+RQ_integral+Arg_conc.+emi ssion_O2+consum_O2; 0.463, 0.763, pH_cumulo+PL+AN+AN_cumulo +OD+Feed_Vol+Cell_conc._interval+RQ_integral+Arg_conc.+emis sion_CO2+consum_O2+interval_CO2; 0.487, 0.763, pH+pH_cumulo +AN_cumulo+interval_yield+OD+Feed_Vol+Cell_conc._interval+A rg_conc.+emission_CO2+consum_O2; 0.436, 0.763, pH+pH_cumulo +PL+AN+AN_cumulo+OD+Feed_Vol+Cell_conc._interval+rab_integr al+RQ_integral+Arg_conc.+emission_O2+consum_O2+interval_CO 2; 0.463, 0.763, pH_cumulo+PL+AN+AN_cumulo+OD+Feed_Vol+Cell _conc._interval+rab_integral+Arg_conc.+emission_O2+emission _CO2+consum_O2; 0.463, 0.763, pH_cumulo+AN+AN_cumulo+OD+Fee d_Vol+Cell_conc._interval+rab_integral+RQ_integral+Arg_conc. +emission_CO2+consum_O2+interval_O2; 0.487, 0.763, pH+pH_cu muIo+AN_cumulo+OD+Feed_Vol+Cell_conc._interval+Arg_conc.+em ission_CO2+consum_O2+generate_CO2; 0.450, 0.763, pH+pH_cumu lo+AN+AN_cumulo+interval_yield+OD+Feed_Vol+Cell_conc._inter val+rab_integral+RQ_integral+Arg_conc.+emission_CO2+consum_ O2; 0.390, 0.763, pH+pH_cumulo+PL_cumulo+AN+AN_cumulo+inter val_yield+OD+Feed_Vol+RS_cumulo+Cell_conc._interval+rab_int egral+RQ_integral+Arg_conc.+emission_O2+consum_O2+generate CO2+interval_CO2; 0.406, 0.763, pH+pH_cumulo+PL_cumulo+AN+A N_cumulo+interval_yield+OD+Feed_Vol+Cell_conc._interval+rab integral+RQ_integral+Accum._Yield+Arg_conc.+emission_O2+co nsum_O2+interval_O2; 0.450, 0.763, pH_cumulo+PL+AN+AN_cumul o+OD+Feed_Vol+Cell_conc._interval+RQ_integral+Arg_conc.+emi ssion_O2+emission_CO2+consum_O2+interval_02; 0.463, 0.763, pH_cumulo+PL+AN_cumulo+interval_yield+OD+FeedVol+Cell_conc. _interval+rab_integral+RQ_integral+Arg_conc.+emission_O2+co nsum_O2; 0.450, 0.763, pH_cumulo+PL_cumulo+AN+AN_cumulo+int erval_yield+OD+Cell_conc._interval+rab_integral+RQ_integral +Arg_conc.+emission_CO2+consum_O2+interval_CO2; 0.487, 0.76 3, pH_cumulo+AN+AN_cumulo+OD+RS+Feed_Vol+Cell_conc._interva 1+Arg conc.+emission_CO2+consum_O2; 0.487, 0.763, pH+pH_cum ulo+AN_cumulo+OD+Feed_Vol+Cell_conc._interval+Arg_conc.+emi ssion_O2+emission_CO2+consum_O2; 0.463, 0.763, pH_cumulo+AN +AN_cumulo+OD+Feed_Vol+Cell_conc._interval+rab_integral+RQ_ integral+Arg_conc.+emission_CO2+consum_O2+interval_CO2; 0.4 50, 0.763, pH_cumulo+PL+AN+AN_cumulo+interval_yield+OD+Feed _Vol+Cell_conc._interval+RQ_integral+Arg_conc.+emission_CO2 +consum_O2+interval_O2; 0.436, 0.763, pH_cumulo+PL+AN+AN_cu mulo+OD+Feed_Vol+RS_cumulo+Cell_conc._interval+rab_integral +RQ_integral+Arg_conc.+emission_O2+emission_CO2+consum_O2; 0.475, 0.763, pH_cumulo+AN+AN_cumulo+OD+Feed_Vol+Cell_conc. _interval+Arg_conc.+emission_O2+emission_CO2+consum_O2+gene rate_CO2; 0.463, 0.763, pH_cumulo+PL+AN+AN_cumulo+interval_ yield+OD+RS_cumulo+Cell_conc._interval+Arg_conc.+emission_C O2+consum_O2+interval_CO2; 0.487, 0.763, pH+pH_cumulo+AN_cu mulo+OD+Feed_Vol+Cell_conc._interval+RQ_integral+Arg_conc.+ emission _O2+consum _02; 0.463, 0.763, pH+pH_cumulo+AN+AN_cum ulo+interval_yield+OD+Cell_conc._interval+Arg_conc.+emissio n_CO2+consum_O2+generate_CO2+interval_CO2; 0.450, 0.763, pH _cumulo+PL+AN+AN_cumulo+OD+Feed_Vol+Cell_conc._interval+RQ_ integral+Arg_conc.+emission_O2+emission_CO2+consum_O2+gener ate_CO2; 0.390, 0.763, pH+pH_cumulo+PL+PL_cumulo+AN+AN_cumu lo+interval_yield+OD+Cell_conc._interval+rab_integral+RQ_in tegral+Arg conc.+emission_O2+emission_CO2+consum_O2+generat e_CO2+interval_CO2; 0.421, 0.763, tmp_cumulo+pH_cumulo+PL+A N+AN_cumulo+interval_yield+OD+Feed_Vol+Cell_conc._interval+ rab_integral+RQ_integral+Arg_conc.+emission_O2+consum_O2+in terval_CO2; 0.406, 0.763, pH_cumulo+PL+PL_cumulo+AN+AN_cumu lo+interval_yield+OD+Feed_Vol+RS_cumulo+Cell_conc._interval +rab_integral+RQ_integral+Arg_conc.+emission_O2+consum_O2+g enerate_CO2; 0.406, 0.763, pH+pH_cumulo+PL+AN+AN_cumulo+int erval_yield+OD+Feed_Vol+RS_cumulo+Cell_conc._interval+rab_i ntegral+RQ_integral+Arg_conc.+emission_O2+emission_CO2+cons um_O2; 0.475, 0.763, pH_cumulo+AN+AN_cumulo+OD+Feed_Vol+Cel l_conc._interval+RQ_integral+Arg_conc.+emission_O2+consum_O 2+generate_CO2; 0.390, 0.763, tmp+pH+pH_cumulo+PL+PL_cumulo +AN+AN_cumulo+interval_yield+OD+RS_cumulo+Cell_conc._interv al+rab_integral+RQ_integral+Arg_conc.+emission_O2+consum_O2 +interval_CO2; 0.421, 0.763, tmp_cumulo+pH_cumulo+PL+PL_cum ulo+AN+AN_cumulo+interval_yield+OD+Cell_conc._interval+rab_ integral+RQ_integral+Arg_conc.+emission_CO2+generate_CO2+in terval_CO2; 0.450, 0.763, pH_cumulo+PL_cumulo+AN+AN_cumulo+ OD+Feed_Vol+Cell_conc._interval+RQ_integral+Arg_conc.+emiss ion_O2+emission_CO2+consum_O2+interval_O2; 0.436, 0.763, pH _cumulo+PL_cumulo+AN+AN_cumulo+OD+Feed_Vol+Cell_conc._inter val+rab_integral+RQ_integral+Arg_conc.+emission_O2+emission _CO2+consum_O2+interval_CO2; 0.449, 0.763, pH_cumulo+AN+AN_ cumulo+interval_yield+OD+RS+Feed_Vol+Cell_conc._interval+ra b_integral+RQ_integral+Arg_conc.+emission_O2+consum_O2; 0.4 49, 0.763, pH_cumulo+AN+AN_cumulo+interval_yield+OD+RS_cumu lo+Cell_conc._interval+rab_integral+RQ_integral+Arg_conc.+e mission_O2+consum_O2+interval_CO2; 0.463, 0.763, pH_cumulo+ AN+AN_cumulo+OD+Feed_Vol+Cell_conc._interval+RQ_integral+Ar g_conc.+emission_CO2+consum_O2+generate_CO2+interval_CO2; 0. 421, 0.763, pH+pH_cumulo+AN+AN_cumulo+interval_yield+OD+Cel l_conc._interval+rab_integral+RQ_integral+Arg_conc.+emissio n_O2+emission_CO2+interval_O2+generate_CO2+interval_CO2; 0. 390, 0.763, pH+pH_cumulo+PL+PL_cumulo+AN+AN_cumulo+interval _yield+OD+Feed_Vol+RS_cumulo+Cell_conc._interval+rab_integr al+RQ_integral+Arg_conc.+emission_O2+consum_O2+interval_O2; 0.475, 0.763, pH_cumulo+AN+AN_cumulo+OD+Feed_Vol+Cell_conc. _interval+Arg_conc.+emission_CO2+consum_O2+interval_O2+inte rval_CO2; 0.449, 0.763, pH_cumulo+PL_cumulo+AN_cumulo+inter val_yield+OD+Feed_Vol+Cell_conc._interval+rab_integral+RQ_i ntegral+Arg_conc.+emission_O2+consum_O2+interval_O2; 0.462, 0.763, pH_cumulo+PL+AN+AN_cumulo+OD+Feed_Vol+RS_cumulo+Cel l_conc._interval+RQ_integral+Arg_conc.+emission_CO2+consum 02;0.406, 0.763, pH_cumulo+PL+PL_cumulo+AN+AN_cumulo+interv al_yield+OD+Feed_Vol+Cell_conc._interval+rab_integral+RQ_in tegral+Accum._Yield+Arg_conc.+emission_O2+consum_O2+interva l_CO2; 0.462, 0.763, pH_cumulo+AN+AN_cumulo+OD+RS+Feed_Vol+ Cell_conc._interval+rab_integral+RQ_integral+Arg_conc.+emis sion_O2+consum_O2; 0.475, 0.763, pH_cumulo+AN+AN_cumulo+OD+ Feed_Vol+RS_cumulo+Cell_conc._interval+rab_integral+Arg_con c.+emission_CO2+consum_O2; 0.406, 0.763, tmp_cumulo+pH_cumu lo+PL+PL_cumulo+AN+AN_cumulo+interval_yield+OD+Cell_conc._i nterval+rab_integral+RQ_integral+Arg_conc.+emission_O2+inte rval_O2+generate_CO2+interval_CO2; 0.421, 0.763, pH+pH_cumu lo+AN+AN_cumulo+interval_yield+OD+Feed_Vol+Cell_conc._inter val+rab_integral+RQ_integral+Arg_conc.+emission_O2+emission _CO2+consum_O2+interval_O2; 0.449, 0.763, tmp_cumulo+pH+pH_ cumulo+AN+AN_cumulo+OD+Feed_Vol+Cell_conc._interval+rab_int egral+RQ_integral+Arg_conc.+emission_O2+consum_O2; 0.421, 0. 763, pH+pH_cumulo+PL+AN+AN_cumulo+interval_yield+OD+Cell_co nc._interval+rab_integral+RQ_integral+Arg_conc.+emission_O2 +emission_CO2+consum_O2+interval_CO2; 0.421, 0.763, tmp+pH+ pH_cumulo+PL+AN+AN_cumulo+interval_yield+OD+Feed_Vol+Cell_c one._interval+rab_integral+RQ_integral+Arg_conc.+emission_0 2+consum_O2; 0.475, 0.763, pH_cumulo+AN+AN_cumulo+OD+Feed_V ol+Cell_conc._interval+rab_integral+Arg_conc.+emission_CO2+ consum_O2+interval_CO2; 0.462, 0.763, pH+pH_cumulo+AN+AN_cu mulo+OD+Feed_Vol+Cell_conc._interval+RQ_integral+Arg_conc.+ emission_O2+consum_O2+generate_CO2; 0.435, 0.763, pH_cumulo +PL+AN+AN_cumulo+OD+Feed_Vol+Cell_conc._interval+rab_integr al+RQ_integral+Arg_conc.+emission_O2+emission_CO2+consum_O2 +interval_CO2; 0.421, 0.763, tmp+pH+pH_cumulo+PL_cumulo+AN+ AN_cumulo+interval_yield+OD+Feed_Vol+Cell_conc._interval+ra b_integral+RQ_integral+Arg_conc.+emission_O2+consum_O2; 0.4 21, 0.763, tmp_cumulo+pH_cumulo+PL_cumulo+AN+AN_cumulo+inte rval_yield+OD+RS_cumulo+Cell_conc._interval+rab_integral+RQ _integral+Arg_conc.+emission_O2+consum_O2+interval_CO2; 0.4 21, 0.763, pH_cumulo+AN+AN_cumulo+interval_yield+OD+Feed_Vo l+Cell_conc._interval+rab_integral+RQ_integral+Arg_conc.+em ission_O2+consum_O2+interval_O2+generate_CO2+interval_CO2; 0.462, 0.763, pH_cumulo+AN+AN_cumulo+OD+Feed_Vol+RS_cumulo+ Cell_conc._interval+RQ_integral+Arg_conc.+emission_O2+emiss ion_CO2+consum_O2; 0.406, 0.763, pH_cumulo+PL_cumulo+AN+AN_ cumulo+interval_yield+OD+Cell_conc._interval+rab_integral+R Q_integral+Arg_conc.+emission_O2+emission_CO2+consum_O2+int erval_O2+generate_CO2+interval_CO2; 0.435, 0.763, tmp+pH_cu mulo+PL+AN+AN_cumulo+interval_yield+OD+Cell_conc._interval+ rab_integral+RQ_integral+Arg_conc.+emission_O2+consum_O2+in terval_CO2; 0.406, 0.763, tmp_cumulo+pH_cumulo+PL_cumulo+AN +AN_cumulo+interval_yield+OD+Feed_Vol+Cell_conc._interval+r ab_integral+RQ_integral+Arg_conc.+emission_O2+consum_O2+int erval_O2+interval_CO2; 0.462, 0.763, pH_cumulo+PL+AN+AN_cum ulo+OD+Feed_Vol+RS_cumulo+Cell_conc._interval+RQ_integral+A rg_conc.+emission_O2+consum_O2; 0.449, 0.763, pH+pH_cumulo+ PL_cumulo+AN+AN_cumulo+interval_yield+OD+Cell_conc._interva l+Arg_conc.+emission_CO2+interval_O2+generate_CO2+interval_ CO2; 0.449, 0.763, pH_cumulo+PL+AN+AN_cumulo+OD+Cell_conc._ interval+rab_integral+RQ_integral+Arg_conc.+emission_O2+int erval_O2+generate_CO2+interval_CO2; 0.435, 0.763, pH_cumulo +PL+AN+AN_cumulo+interval_yield+OD+Feed_Vol+Cell_conc._inte rval+rab_integral+RQ_integral+Arg_conc.+emission_O2+interva l_O2+generate_CO2; 0.421, 0.763, tmp_cumulo+pH+pH_cumulo+AN +AN_cumulo+interval_yield+OD+Cell_conc._interval+rab_integr al+RQ_integral+Arg_conc.+emission_O2+interval_O2+generate_C O2+interval_CO2; 0.405, 0.763, pH+pH_cumulo+PL_cumulo+AN+AN _cumulo+interval_yield+OD+RS_cumulo+Cell_conc._interval+rab _integral+RQ_integral+Arg_conc.+emission_O2+consum_O2+inter val_O2+interval_CO2; 0.405, 0.763, pH_cumulo+PL_cumulo+AN+A N_cumulo+interval_yield+OD+RS_cumulo+Cell_conc._interval+ra b_integral+RQ_integral+Arg_conc.+emission_O2+consum_O2+inte rval_O2+generate_CO2+interval_CO2; 0.435, 0.763, pH+pH_cumu lo+PL+PL_cumulo+AN+AN_cumulo+OD+Cell_conc._interval+rab_int egral+RQ_integral+Arg_conc.+emission_O2+consum_O2+interval_ CO2; 0.405, 0.763, pH+pH_cumulo+PL+PL_cumulo+AN+AN_cumulo+i nterval_yield+OD+Cell_conc._interval+rab_integral+RQ_integr al+Accum._Yield+Arg_conc.+emission_O2+consum_O2+interval_CO 2; 0.475, 0.763, pH_cumulo+AN+AN_cumulo+OD+RS_cumulo+Cell_c onc._interval+Arg_conc.+emission_CO2+interval_O2+generate_C O2+interval_CO2; 0.475, 0.763, pH_cumulo+PL+PL_cumulo+AN+AN cumulo+OD+Cell_conc._interval+Arg_conc.+emission_CO2+consu m_O2+interval_CO2; 0.421, 0.763, pH+pH_cumulo+PL+PL_cumulo+ AN+AN_cumulo+OD+Feed_Vol+RS_cumulo+Cell_conc._interval+rab_ integral+RQ_integral+Arg_conc.+emission_O2+consum_O2; 0.449, 0.763, tmp_cumulo+pH_cumulo+PL+AN+AN_cumulo+OD+Feed_Vol+Ce ll_conc._interval+rab_integral+RQ_integral+Arg_conc.+emissi on_O2+consum_O2; 0.405, 0.763, tmp_cumulo+pH_cumulo+PL_cumu lo+AN+AN_cumulo+interval_yield+OD+Cell_conc._interval+rab_i ntegral+RQ_integral+Arg_conc.+emission_O2+consum_O2+interva l_O2+generate_CO2+interval_CO2; 0.435, 0.763, pH+pH_cumulo+ PL+AN+AN_cumulo+interval_yield+OD+Cell_conc._interval+rab_i ntegral+RQ_integral+Arg_conc.+emission_CO2+consum_O2+interv al_CO2; 0.474, 0.763, pH_cumulo+AN+AN_cumulo+OD+Feed_Vol+Ce ll_conc._interval+Arg_conc.+emission_O2+emission_CO2+consum _O2+interval_O2; 0.435, 0.763, pH_cumulo+PL+AN+AN_cumulo+OD +Feed_Vol+Cell_conc._interval+rab_integral+RQ_integral+Arg_ conc.+emission_O2+emission_CO2+consum_O2+interval_02; 0.486, 0.763, pH_cumulo+AN+AN_cumulo+OD+RS_cumulo+Cell_conc._inte rval+Arg_conc.+emission_CO2+consum_O2+interval_CO2; 0.389, 0.763, tmp+pH_cumulo+PL+PL_cumulo+AN+AN_cumulo+interval_yie ld+OD+Feed_Vol+Cell_conc._interval+rab_integral+RQ_integral +Arg_conc.+emission_O2+consum_O2+generate_CO2+interval_CO2; 0.421, 0.763, pH_cumulo+PL+AN+AN_cumulo+interval_yield+OD+ Feed_Vol+Cell_conc._interval+rab_integral+RQ_integral+Arg_c onc.+emission_O2+consum_O2+interval_O2+generate_CO2; 0.462, 0.763, pH_cumulo+PL+AN+AN_cumulo+OD+Feed_Vol+Cell_conc._in terval+rab_integral+Arg_conc.+emission_CO2+consum_O2+interv al_O2; 0.449, 0.763, pH_cumulo+PL+AN+AN_cumulo+interval_yie ld+OD+Feed_Vol+Cell_conc._interval+RQ_integral+Arg_conc.+em ission_CO2+consum_O2+generate_CO2; 0.462, 0.763, pH+pH_cumu lo+PL+PL_cumulo+AN+AN_cumulo+OD+Feed_Vol+Cell_conc._interva l+Arg_conc.+emission_CO2+consum_O2; 0.420, 0.762, pH_cumulo +AN+AN_cumulo+interval_yield+OD+Cell_conc._interval+rab_int egral+RQ_integral+Arg_conc.+emission_O2+emission_CO2+consum _O2+interval_O2+generate_CO2+interval_CO2; 0.405, 0.762, tm p+tmp_cumulo+pH_cumulo+PL_cumulo+AN+AN_cumulo+interval_yiel d+OD+Cell_conc._interval+rab_integral+RQ_integral+Arg_conc. +emission_O2+consum_O2+interval_O2+interval_CO2; 0.474, 0.7 62, pH_cumulo+AN+AN_cumulo+interval_yield+OD+Feed_Vol+Cell_ conc._interval+Arg_conc.+emission_CO2+consum_O2+interval_O 2; 0.462, 0.762, pH_cumulo+PL+PL_cumulo+AN+AN_cumulo+OD+Fee d_Vol+Cell_conc._interval+RQ_integral+Arg_conc.+emission_CO 2+consum_O2; 0.462, 0.762, pH_cumulo+AN+AN_cumulo+OD+Feed_V ol+RS_cumulo+Cell_conc._interval+rab_integral+RQ_integral+A rg_conc.+emission_CO2+consum_O2; 0.420, 0.762, pH_cumulo+PL +PL_cumulo+AN+AN_cumulo+OD+Feed_Vol+Cell_conc._interval+rab _integral+RQ_integral+Arg_conc.+emission_O2+consum_O2+gener ate_CO2+interval_CO2; 0.462, 0.762, pH_cumulo+PL+AN+AN_cumu lo+OD+Feed_Vol+Cell_conc._interval+rab_integral+Arg_conc.+e mission_CO2+consum_O2+generate_CO2; 0.462, 0.762, tmp_cumul o+pH_cumulo+AN+AN_cumulo+OD+Feed_Vol+Cell_conc._interval+Ar g conc.+emission CO2+consum O2+generate CO2+interval CO2; 0. 435, 0.762, pH_cumulo+PL+AN+AN_cumulo+OD+Feed_Vol+Cell_conc. _interval+rab_integral+RQ_integral+Arg_conc.+emission_O2+em ission_CO2+consum_O2+generate_CO2; 0.462, 0.762, pH_cumulo+ AN+AN_cumulo+interval_yield+OD+Cell_conc._interval+Arg_conc. +emission_O2+emission_CO2+interval_O2+generate_CO2+interval _CO2; 0.462, 0.762, pH_cumulo+PL+AN+AN_cumulo+interval_yiel d+OD+Feed_Vol+Cell_conc._interval+Arg_conc.+emission_CO2+co nsum_O2+interval_CO2; 0.420, 0.762, tmp_cumulo+pH_cumulo+PL +PL_cumulo+AN+AN_cumulo+interval_yield+OD+Feed_Vol+Cell_con c._interval+rab_integral+RQ_integral+Arg_conc.+emission_O2+ consum_O2; 0.435, 0.762, pH_cumulo+PL_cumulo+AN+AN_cumulo+i nterval_yield+OD+Feed_Vol+Cell_conc._interval+RQ_integral+A rg_conc.+emission_O2+emission_CO2+consum_O2+interval_O2; 0. 462, 0.762, tmp_cumulo+pH_cumulo+PL_cumulo+AN+AN_cumulo+OD+ Cell_conc._interval+Arg_conc.+emission_CO2+consum_O2+genera te_CO2+interval_CO2; 0.462, 0.762, tmp_cumulo+pH_cumulo+PL+ AN+AN_cumulo+OD+Cell_conc._interval+Arg_conc.+emission_CO2+ consum_O2+interval_O2+interval_CO2; 0.449, 0.762, tmp+pH_cu mulo+AN+AN_cumulo+OD+Feed_Vol+Cell_conc._interval+rab_integ ral+RQ_integral+Arg_conc.+emission_O2+emission_CO2+consum_O 2; 0.405, 0.762, tmp+pH_cumulo+PL_cumulo+AN+AN_cumulo+inter val_yield+OD+Feed_Vol+Cell_conc._interval+rab_integral+RQ_i ntegral+Arg_conc.+emission_O2+consum_O2+interval_O2+interva l_CO2; 0.462, 0.762, pH_cumulo+PL+AN+AN_cumulo+OD+Feed_Vol+ Cell_conc._interval+Arg_conc.+emission_CO2+consum_O2+genera te_CO2+interval_CO2; 0.448, 0.762, tmp_cumulo+pH_cumulo+PL_ cumulo+AN+AN_cumulo+OD+Feed_Vol+Cell_conc._interval+rab_int egral+RQ_integral+Arg_conc.+emission_O2+consum_O2; 0.435, 0. 762, pH_cumulo+PL_cumulo+AN+AN_cumulo+interval_yield+OD+RS_ cumulo+Cell_conc._interval+rab_integral+RQ_integral+Arg_con c.+emission_CO2+generate_CO2+interval_CO2; 0.462, 0.762, tm p_cumulo+pH+pH_cumulo+AN+AN_cumulo+OD+Cell_conc._interval+A rg_conc.+emission_CO2+consum_O2+interval_O2+interval_CO2; 0. 435, 0.762, pH_cumulo+PL+AN+AN_cumulo+interval_yield+OD+RS_ cumuIo+Cell_conc._interval+rab_integral+RQ_integral+Arg_con c.+emission_O2+interval_O2+interval_CO2; 0.474, 0.762, pH_c umulo+AN+AN_cumulo+OD+Feed_Vol+Cell_conc._interval+RQ_integ ral+Arg_conc.+emission_O2+consum_O2+interval_O2; 0.474, 0.7 62, tmp_cumulo+pH_cumulo+PL_cumulo+AN+AN_cumulo+OD+Cell_con c._interval+Arg_conc.+emission_CO2+consum_O2+interval-CO2; 0.448, 0.762, tmp_cumulo+pH_cumulo+AN+AN_cumulo+OD+Cell_con c._interval+rab_integral+RQ_integral+Arg_conc.+emission_O2+ emission_CO2+consum _O2+interval_CO2; 0.474, 0.762, pH_cumul o+AN+AN_cumulo+OD+Feed_Vol+Cell_conc._interval+Arg_conc.+em ission_CO2+consum_O2+interval_O2+generate_CO2; 0.462, 0.762, tmp+tmp_cumulo+pH_cumulo+AN+AN_cumulo+OD+Cell_conc._interv al+Arg_conc.+emission_CO2+consum_O2+generate_CO2+interval_C 02; 0.405, 0.762, pH+pH_cumulo+PL_cumulo+AN+AN_cumulo+inter val_yield+OD+Feed_Vol+RS_cumulo+Cell_conc._interval+rab_int egral+RQ_integral+Arg_conc.+emission_O2+consum_O2+interval_ CO2; 0.462, 0.762, pH+pH_cumulo+AN+AN_cumulo+OD+Feed_Vol+Ce ll_conc._interval+rab_integral+Arg_conc.+emission_CO2+consu m_O2+interval_O2; 0.461, 0.762, pH_cumulo+PL_cumulo+AN+AN_c umulo+OD+Feed_Vol+Cell_conc._interval+Arg_conc.+emission_CO 2+consum_O2+generate_CO2+interval_CO2; 0.389, 0.762, tmp+pH +pH_cumulo+PL_cumulo+AN+AN_cumulo+interval_yield+OD+Feed_Vo l+Cell_conc._interval+rab_integral+RQ_integral+Arg_conc.+em ission_O2+consum_O2+generate_CO2+interval_CO2; 0.420, 0.762, pH_cumulo+PL+AN+AN_cumulo+interval_yield+OD+Feed_Vol+Cell_ conc._interval+rab_integral+RQ_integral+Arg_conc.+emission_ O2+consum_O2+interval_O2+interval_CO2; 0.435, 0.762, pH+pH_ cumulo+PL+AN+AN_cumulo+OD+Feed_Vol+Cell_conc._interval+rab_ integral+RQ_integral+Arg_conc.+emission_O2+consum_O2+genera te_CO2; 0.420, 0.762, tmp_cumulo+pH+pH_cumulo+AN+AN_cumulo+ interval_yield+OD+Cell_conc._interval+rab_integral+RQ_integ ral+Arg_conc.+emission_O2+consum_O2+generate_CO2+interval_C 02; 0.448, 0.762, tmp_cumuIo+pH_cumulo+PL_cumulo+AN+AN_cumu lo+OD+Cell_conc._interval+rab_integral+RQ_integral+Arg_conc. +emission_CO2+consum_O2+interval_CO2; 0.435, 0.762, pH_cumu lo+AN+AN_cumulo+interval_yield+OD+Feed_Vol+RS_cumulo+Cell_c onc._interval+rab_integral+RQ_integral+Arg_conc.+emission_O 2+consum_O2+interval_O2; 0.434, 0.762, pH_cumulo+PL+AN+AN_c umulo+OD+Feed_Vol+RS_cumulo+Cell_conc._interval+rab_integra l+RQ_integral+Arg_conc.+emission_O2+consum_O2+interval_CO2 ; 0.461, 0.762, pH_cumulo+AN+AN_cumulo+OD+Feed_Vol+Cell_conc. _interval+RQ_integral+Arg_conc.+emission_CO2+interval_O2+ge nerate_CO2+interval_CO2; 0.461, 0.762, pH+pH_cumulo+PL+AN+A N_cumulo+OD+Feed_Vol+Cell_conc._interval+Arg_conc.+emission _CO2+consum_O2+interval_O2; 0.420, 0.762, tmp_cumulo+pH+pH_ cumulo+AN+AN_cumulo+interval_yield+OD+Cell_conc._interval+r ab_integral+RQ_integral+Arg_conc.+emission_O2+emission_CO2+ consum_O2+interval_CO2; 0.434, 0.762, tmp_cumulo+pH+pH_cumu lo+AN+AN_cumulo+OD+Feed_Vol+Cell_conc._interval+rab_integra l+RQ_integral+Arg_conc.+emission_O2+consum_O2+interval_CO2; 0.389, 0.762, pH+pH_cumulo+PL_cumulo+AN+AN_cumulo+interval _yield+OD+Feed_Vol+Cell_conc._interval+rab_integral+RQ_inte gral+Arg_conc.+emission_O2+consum_O2+interval_O2+generate_C O2+interval_CO2; 0.461, 0.762, pH+pH_cumulo+AN+AN_cumulo+OD +Feed_Vol+Cell_conc._interval+rab_integral+Arg_conc.+emissi on_CO2+consum_O2+generate_CO2; 0.389, 0.762, tmp_cumulo+pH+ pH_cumulo+PL_cumulo+AN+AN_cumulo+interval_yield+OD+Feed_Vol +Cell_conc._interval+rab_integral+RQ_integral+Arg_conc.+emi ssion_O2+consum_O2+generate_CO2+interval_CO2; 0.474, 0.762, pH_cumulo+AN+AN_cumulo+interval_yield+OD+Feed_Vol+RS_cumul o+Cell_conc._interval+Arg_conc.+emission_CO2+consum_O2; 0.4 48, 0.762, pH_cumulo+PL_cumulo+AN+AN_cumulo+interval_yield+ OD+Feed_Vol+Cell_conc._interval+rab_integral+RQ_integral+Ar g_conc.+emission_CO2+consum_O2; 0.388, 0.762, pH_cumulo+PL+ PL_cumulo+AN+AN_cumulo+interval_yield+OD+Feed_Vol+Cell_conc. _interval+rab_integral+RQ_integral+Arg_conc.+emission_02+em ission_CO2+consum_O2+generate_CO2+interval_CO2; 0.434, 0.76 2, pH_cumulo+PL+AN+AN_cumulo+interval_yield+OD+Cell_conc._i nterval+rab_integral+RQ_integral+Arg_conc.+emission_CO2+int erval_O2+generate_CO2+interval_CO2; 0.420, 0.762, tmp+pH_cu mulo+PL+PL_cumulo+AN+AN_cumulo+OD+Feed_Vol+Cell_conc._inter val+rab_integral+RQ_integral+Arg_conc.+emission_O2+consum_O 2+interval_CO2; 0.405, 0.762, tmp_cumulo+pH_cumulo+PL_cumul o+AN+AN_cumulo+interval_yield+OD+Cell_conc._interval+rab_in tegral+RQ_integral+Arg_conc.+emission_O2+emission_CO2+consu m_O2+interval_O2+interval_CO2; 0.474, 0.762, pH+pH_cumulo+P L+AN_cumulo+interval_yield+OD+Feed_Vol+Cell_conc._interval+ Arg_conc.+emission_CO2+consum_O2; 0.420, 0.762, tmp_cumulo+ pH_cumulo+PL_cumulo+AN+AN_cumulo+interval_yield+OD+Cell_con c._interval+rab_integral+RQ_integral+Arg_conc.+emission_CO2 +consum_O2+generate_CO2+interval_CO2; 0.461, 0.762, pH+pH_c umulo+AN+AN_cumulo+OD+Feed_Vol+Cell_conc._interval+Arg_conc. +emission_CO2+interval_O2+generate_CO2+interval_CO2; 0.420, 0.762, pH+pH_cumulo+PL_cumulo+AN+AN_cumulo+interval_yield+ OD+Cell_conc._interval+rab_integral+RQ_integral+Arg_conc.+e mission_CO2+consum_O2+generate_CO2+interval_CO2; 0.461, 0.7 62, pH_cumulo+PL_cumulo+AN+AN_cumulo+OD+Feed_Vol+Cell_conc. _interval+RQ_integral+Arg_conc.+emission_O2+consum_O2+inter val_O2; 0.486, 0.762, pH+pH_cumulo+AN_cumulo+OD+Feed_Vol+RS _cumulo+Cell_conc._interval+Arg_conc.+emission_CO2+consum_O 2; 0.434, 0.762, pH+pH_cumulo+PL+PL_cumulo+AN_cumulo+interv al_yield+OD+Feed_Vol+Cell_conc._interval+rab_integral+RQ_in tegral+Arg_conc.+emission_O2+consum_O2; 0.474, 0.762, pH_cu mulo+AN+AN_cumulo+OD+Cell_conc._interval+rab_integral+Arg_c onc.+emission_CO2+interval_O2+generate_CO2+interval_CO2; 0. 448, 0.762, pH_cumulo+PL_cumulo+AN+AN_cumulo+OD+Feed_Vol+Ce ll_conc._interval+RQ_integral+Arg_conc.+emission_O2+emissio n_CO2+consum_O2+generate_CO2; 0.461, 0.762, pH+pH_cumulo+PL +AN+AN_cumulo+interval_yield+OD+Cell_conc._interval+Arg_con c.+emission_CO2+consum_O2+interval_CO2; 0.434, 0.762, pH_cu mulo+AN+AN_cumulo+interval_yield+OD+Feed_Vol+RS_cumulo+Cell _conc._interval+rab_integral+RQ_integral+Arg_conc.+emission _O2+consum_O2+generate_CO2; 0.486, 0.762, pH_cumulo+PL_cumu lo+AN_cumulo+OD+Feed_Vol+Cell_conc._interval+Arg_conc.+emis sion_O2+emission_CO2+consum_O2; 0.434, 0.762, pH_cumulo+AN+ AN_cumulo+interval_yield+OD+Feed_Vol+Cell_conc._interval+ra b_integral+RQ_integral+Arg_conc.+emission_O2+consum_O2+inte rval_O2+interval_CO2; 0.420, 0.762, pH+pH_cumulo+AN+AN_cumu lo+interval_yield+OD+Feed_Vol+Cell_conc._interval+rab_integ ral+RQ_integral+Arg_conc.+emission_O2+consuni_O2+interval_O2 +generate_CO2; 0.404, 0.762, tmp_cumulo+pH_cumulo+PL_cumulo +AN+AN_cumulo+interval_yield+OD+RS_cumulo+Cell_conc._interv al+rab_integral+RQ_integral+Arg_conc.+emission_O2+consum_O2 +generate_CO2+interval_CO2; 0.461, 0.762, pH_cumulo+AN+AN_c umulo+interval_yield+OD+RS_cumulo+Cell_conc._interval+Arg_c onc.+emission_CO2+interval_O2+generate_CO2+interval_CO2; 0. 461, 0.762, pH+pH_cumulo+AN+AN_cumulo+OD+Feed_Vol+Cell_conc. _interval+RQ_integral+Arg_conc.+emission_O2+consum_O2+inter val_O2; 0.388, 0.762, pH+pH_cumulo+PL+PL_cumulo+AN+AN_cumul o+interval_yield+OD+RS_cumulo+Cell_conc._interval+rab_integ ral+RQ_integral+Arg_conc.+emission_O2+emission_CO2+consum_O 2+interval_CO2; 0.388, 0.762, tmp+tmp_cumulo+pH_cumulo+PL+P L_cumulo+AN+AN_cumulo+interval_yield+OD+Cell_conc._interval +rab_integral+RQ_integral+Arg_conc.+emission_O2+consum_O2+g enerate_CO2+interval_CO2; 0.485, 0.762, tmp+pH_cumulo+PL+AN _cumulo+OD+Feed_Vol+Cell_conc._interval+Arg_conc.+emission_ CO2+consum_O2; 0.448, 0.762, pH_cumulo+PL_cumulo+AN+AN_cumu lo+OD+Cell_conc._interval+rab_integral+RQ_integral+Arg_conc. +emission_O2+interval_O2+generate_CO2+interval_CO2; 0.448, 0.762, pH_cumulo+AN+AN_cumulo+OD+Feed_Vol+RS_cumulo+Cell_co nc._interval+rab_integral+RQ_integral+Arg_conc.+emission_O2 +consum_O2+interval_CO2; 0.419, 0.762, pH+pH_cumulo+AN+AN_c umulo+interval_yield+OD+Feed_Vol+Cell_conc._interval+rab_in tegral+RQ_integral+Arg_conc.+emission_O2+consum_O2+generate _CO2+interval_CO2; 0.404, 0.762, pH_cumulo+PL+PL_cumulo+AN+ AN_cumulo+interval_yield+OD+Cell_conc._interval+rab_integra l+RQ_integral+Arg_conc.+emission_O2+emission_CO2+interval_O 2+generate_CO2+interval_CO2; 0.473, 0.762, pH_cumulo+AN+AN_ cumulo+OD+Feed_Vol+RS_cumulo+Cell_conc._interval+Arg_conc.+ emission_O2+emission_CO2+consum_O2; 0.434, 0.762, pH_cumulo +PL+AN+AN_cumulo+interval_yield+OD+Feed_Vol+Cell_conc._inte rval+rab_integral+RQ_integral+Accum._Yield+Arg_conc.+emissi on_CO2+consum_O2; 0.419, 0.762, pH+pH_cumulo+PL_cumulo+AN+A N_cumulo+OD+Feed_Vol+Cell_conc._interval+rab_integral+RQ_in tegral+Arg_conc.+emission_O2+consum_O2+generate_CO2+interva l_CO2; 0.419, 0.762, tmp+pH_cumulo+PL_cumulo+AN+AN_cumulo+i nterval_yield+OD+RS_cumulo+Cell_conc._interval+rab_integral +RQ_integral+Arg_conc.+emission_O2+consum_O2+interval_CO2 ; 0.473, 0.762, pH_cumulo+AN+AN_cumulo+interval_yield+OD+Feed _Vol+Cell_conc._interval+Arg_conc.+emission_CO2+consum_O2+i nterval_CO2; 0.448, 0.762, pH_cumulo+AN+AN_cumulo+interval_ yield+OD+Feed_Vol+Cell_conc._interval+RQ_integral+Arg-conc. +emission_O2+emission_CO2+consum_O2+generate_CO2; 0.404, 0. 762, pH+pH_cumulo+PL+PL_cumulo+AN+AN_cumulo+interval_yield+ OD+Feed_Vol+Cell_conc._interval+rab_integral+RQ_integral+Ac cum._Yield+Arg_conc.+emission_O2+consum_O2; 0.473, 0.762, t mp_cumulo+pH_cumulo+AN+AN_cumulo+OD+Cell_conc._interval+Arg _conc.+emission_O2+emission_CO2+consum_O2+interval_CO2; 0.4 34, 0.762, pH+pH_cumulo+AN+AN_cumulo+OD+Feed_Vol+Cell_conc. _interval+rab_integral+RQ_integral+Arg_conc.+emission_02+em ission_CO2+consum_O2+generate_CO2; 0.404, 0.762, tmp+pH_cum ulo+PL_cumulo+AN+AN_cumulo+interval_yield+OD+Cell_conc._int erval+rab_integral+RQ_integral+Arg_conc.+emission_O2+consum _O2+interval_O2+generate_CO2+interval_CO2; 0.404, 0.762, pH +pH_cumulo+PL_cumulo+AN+AN_cumulo+interval_yield+OD+Feed_Vo l+Cell_conc._interval+rab_integral+RQ_integral+Arg_conc.+em ission_O2+emission_CO2+consum_O2+interval_CO2; 0.448, 0.762, pH_cumulo+PL_cumulo+AN+AN_cumulo+interval_yield+OD+Cell_co nc._interval+RQ_integral+Arg_conc.+emission_CO2+consum_O2+g enerate_CO2+interval_CO2; 0.461, 0.762, pH_cumulo+AN+AN_cum ulo+OD+Feed_Vol+Cell_conc._interval+rab_integral+RQ_integra l+Arg_conc.+emission_O2+interval_O2+generate_CO2; 0.461, 0. 762, tmp_cumulo+pH_cumulo+AN+AN_cumulo+interval_yield+OD+ra b_integral+RQ_integral+Arg_conc.+emission_O2+consum_O2+inte rval_CO2; 0.448, 0.762, pH+pH_cumulo+AN+AN_cumulo+interval_ yield+OD+Feed_Vol+Cell_conc._interval+RQ_integral+Arg-conc. +emission_CO2+consum_O2+generate_CO2; 0.473, 0.762, tmp_cum ulo+pH+pH_cumulo+AN+AN_cumulo+OD+Feed_Vol+Cell_conc._interv al+Arg_conc.+emission_CO2+consum_O2; 0.461, 0.762, tmp_cumu lo+pH+pH_cumulo+AN+AN_cumulo+OD+Feed_Vol+Cell_conc._interva l+Arg_conc.+emission_CO2+consum_O2+interval_CO2; 0.448, 0.7 62, pH_cumulo+PL_cumulo+AN+AN_cumulo+interval_yield+OD+Feed _Vol+Cell_conc._interval+RQ_integral+Arg_conc.+emission_CO2 +consum_O2+generate_CO2; 0.461, 0.762, pH+pH_cumulo+PL_cumu lo+AN+AN_cumulo+OD+Feed_Vol+Cell_conc._interval+RQ_integral +Arg_conc.+emission_CO2+consum_O2; 0.404, 0.762, tmp_cumulo +pH_cumulo+PL+PL_cumulo+AN+AN_cumulo+interval_yield+OD+Cell _conc._interval+rab_integral+RQ_integral+Arg_conc.+emission _O2+emission_CO2+consum_O2+interval_CO2; 0.461, 0.762, pH+p H_cumulo+PL+AN+AN_cumulo+OD+Feed_Vol+Cell_conc._interval+Ar g_conc.+emission_CO2+consum_O2+generate_CO2; 0.448, 0.762, pH_cumulo+AN+AN_cumulo+interval_yield+OD+Cell_conc._interva l+rab_integral+RQ_integral+Arg_conc.+emission_CO2+consum_O2 +generate_CO2+interval_CO2; 0.434, 0.762, pH+pH_cumulo+AN+A N_cumulo+OD+Feed_Vol+Cell_conc._interval+rab_integral+RQ_in tegral+Arg_conc.+emission_O2+emission_CO2+consum_O2+interva l_O2; 0.473, 0.762, pH_cumulo+AN+AN_cumulo+OD+Feed_Vol+RS_c umulo+Cell_conc._interval+Arg_conc.+emission_CO2+consum_O2+ interval_CO2; 0.447, 0.762, pH_cumulo+AN+AN_cumulo+OD+Feed_ Vol+RS_cumulo+Cell_conc._interval+rab_integral+RQ_integral+ Arg_conc.+emission_O2+consum_O2+interval_O2; 0.473, 0.762, tmp+pH_cumulo+AN+AN_cumulo+OD+Feed_Vol+Cell_conc._interval+ Arg_conc.+emission_CO2+consum_O2+interval_CO2; 0.461, 0.762, tmp+tmp_cumulo+pH_cumulo+PL+AN+AN_cumulo+OD+Cell_conc._int erval+Arg_conc.+emission_CO2+consum_O2+interval_CO2; 0.460, 0.762, pH_cumulo+PL+AN+AN_cumulo+OD+Feed_Vol+RS_cumulo+Cel l_conc._interval+Arg_conc.+emission_CO2+consum_O2+interval_ CO2; 0.473, 0.762, pH_cumulo+AN+AN_cumulo+OD+Cell_conc._int erval+RQ_integral+Arg_conc.+emission_CO2+interval_O2+genera te_CO2+interval_CO2; 0.419, 0.762, pH+pH_cumulo+PL+PL_cumul o+AN+AN_cumulo+interval_yield+OD+Cell_conc._interval+rab_in tegral+RQ_integral+Arg_conc.+emission_CO2+generate_CO2+inte rval_CO2; 0.485, 0.762, pH_cumulo+PL+AN_cumulo+OD+Feed_Vol+ Cell_conc._interval+rab_integral+Arg_conc.+emission_CO2+con sum_O2; 0.447, 0.762, pH_cumulo+AN+AN_cumulo+interval_yield +OD+Feed_Vol+Cell_conc._interval+RQ_integral+Arg_conc.+emis sion_CO2+consum_O2+interval_O2+generate_CO2; 0.433, 0.762, pH_cumulo+PL+PL_cumulo+AN+AN_cumulo+interval_yield+OD+RS_cu mulo+rab_integral+RQ_integral+Arg_conc.+emission_O2+consum_ O2+interval_CO2; 0.460, 0.762, pH_cumulo+PL_cumulo+AN+AN_cu mulo+interval_yield+OD+Cell_conc._interval+rab_integral+Arg _conc.+emission_CO2+generate_CO2+interval_CO2; 0.404, 0.762, pH+pH_cumulo+PL_cumulo+AN+AN_cumulo+interval_yield+OD+RS+F eed_Vol+Cell_conc._interval+rab_integral+RQ_integral+Arg_co nc.+emission_O2+consum_O2+interval_O2; 0.447, 0.762, pH_cum ulo+AN+AN_cumulo+OD+Feed_Vol+Cell_conc._interval+rab_integr al+RQ_integral+Arg_conc.+emission_O2+consum_O2+interval_O2+ interval_CO2; 0.404, 0.762, pH_cumulo+PL+PL_cumulo+AN+AN_cu mulo+interval_yield+OD+Feed_Vol+RS_cumulo+Cell_conc._interv al+rab_integral+RQ_integral+Arg_conc.+emission_O2+emission_ CO2+consum_O2; 0.485, 0.762, pH_cumulo+PL_cumulo+AN_cumulo+ interval_yield+OD+Feed_Vol+Cell_conc._interval+Arg_conc.+em ission_CO2+consum_O2; 0.460, 0.762, pH+pH_cumulo+AN+AN_cumu lo+OD+Cell_conc._interval+Arg_conc.+emission_CO2+consum_O2+ interval_O2+generate_CO2+interval_CO2; 0.460, 0.762, pH_cum ulo+PL+AN+AN_cumulo+OD+Feed_Vol+Cell_conc._interval+Arg_con c.+emission_CO2+interval_O2+generate_CO2+interval_CO2; 0.44 7, 0.762, pH+pH_cumulo+PL+AN+AN_cumulo+OD+Cell_conc._interv al+rab_integral+RQ_integral+Arg_conc.+emission_O2+interval_ O2+interval_CO2; 0.447, 0.762, pH_cumulo+AN+AN_cumulo+inter val_yield+OD+Cell_conc._interval+rab_integral+RQ_integral+A rg_conc.+emission_O2+consum_O2+interval_O2+interval_CO2; 0. 447, 0.762, tmp_cumulo+pH+pH_cumulo+AN+AN_cumulo+interval_y ield+OD+Cell_conc._interval+Arg_conc.+emission_CO2+consum_O 2+generate_CO2+interval_CO2; 0.433, 0.762, pH_cumulo+PL+AN+ AN_cumulo+interval_yield+OD+Feed_Vol+Cell_conc._interval+ra b_integral+RQ_integral+Arg_conc.+emission_CO2+consum_O2+int erval_O2; 0.433, 0.762, tmp_cumulo+pH_cumulo+PL_cumulo+AN+A N_cumulo+interval_yield+OD+Cell_conc._interval+rab_integral +RQ_integral+Arg_conc.+emission_O2+generate_CO2+interval_CO 2; 0.433, 0.762, pH+pH_cumulo+AN+AN_cumulo+OD+Feed_Vol+Cell _conc._interval+rab_integral+RQ_integral+Arg_conc.+emission _O2+emission_CO2+consum_O2+interval_CO2; 0.419, 0.762, tmp_ cumulo+pH+pH_cumulo+AN+AN_cumulo+interval_yield+OD+Feed_Vol +Cell_conc._interval+rab_integral+RQ_integral+Arg_conc.+emi ssion_O2+consum_O2+interval_CO2; 0.460, 0.762, tmp+pH_cumul o+AN+AN_cumulo+OD+Feed_Vol+Cell_conc._interval+rab_integral +RQ_integral+Arg_conc.+emission_CO2+consum_O2; 0.460, 0.762, pH_cumulo+PL_cumulo+AN+AN_cumulo+interval_yield+OD+Cell_co nc._interval+Arg_conc.+emission_CO2+consum_O2+interval_O2+i nterval_CO2; 0.403, 0.762, pH+pH_cumulo+PL_cumulo+AN+AN_cum ulo+interval_yield+OD+Feed_Vol+Cell_conc._interval+rab_inte gral+RQ_integral+Arg_conc.+emission_O2+emission_CO2+consum_ O2+generate_CO2; 0.387, 0.762, pH_cumulo+PL+PL_cumulo+AN+AN _cumulo+interval_yield+OD+Feed_Vol+RS_cumulo+Cell_conc._int erval+rab_integral+RQ_integral+Arg_conc.+emission_O2+emissi on_CO2+consum_O2+interval_CO2; 0.387, 0.762, tmp+tmp_cumulo +pH+pH_cumulo+PL_cumulo+AN+AN_cumulo+interval_yield+OD+Cell _conc._interval+rab_integral+RQ_integral+Arg_conc.+emission _O2+consum_O2+generate_CO2+interval_CO2; 0.473, 0.762, pH_c umulo+PL+AN+AN_cumulo+OD+RS_cumulo+Cell_conc._interval+Arg_ conc.+emission_CO2+consum_O2+interval_CO2; 0.419, 0.762, pH +pH_cumulo+PL+PL_cumulo+AN+AN_cumulo+OD+Feed_Vol+Cell_conc. _interval+rab_integral+RQ_integral+Arg_conc.+emission_O2+co nsum_O2+interval_O2; 0.403, 0.762, pH_cumulo+PL+PL_cumulo+A N+AN_cumulo+interval_yield+OD+Cell_conc._interval+rab_integ ral+RQ_integral+Accum._Yield+Arg_conc.+emission_O2+consuni_O 2+interval_O2+interval_CO2; 0.419, 0.762, tmp+pH+pH_cumulo+ PL_cumulo+AN+AN_cumulo+OD+Feed_Vol+Cell_conc._interval+rab_ integral+RQ_integral+Arg_conc.+emission_O2+consum_O2+interv al_CO2; 0.447, 0.762, pH+pH_cumulo+AN+AN_cumulo+interval_yi eld+OD+Feed_Vol+Cell_conc._interval+RQ_integral+Arg_conc.+e mission_CO2+consum_O2+interval_02; 0.460, 0.762, pH_cumulo+ PL+PL_cumulo+AN+AN_cumulo+interval_yield+OD+Feed_Vol+Cell_c onc._interval+Arg_conc.+emission_CO2+consum_O2; 0.460, 0.76 2, tmp+pH+pH_cumulo+AN+AN_cumulo+OD+Cell_conc._interval+Arg _conc.+emission_CO2+interval_O2+generate_CO2+interval_CO2; 0.460, 0.762, pH_cumulo+PL+AN+AN_cumulo+interval_yield+OD+F eed_Vol+Cell_conc._interval+Arg_conc.+emission_CO2+consum_O 2+interval_O2; 0.433, 0.762, pH_cumulo+PL_cumulo+AN+AN_cumu lo+interval_yield+OD+Feed_Vol+Cell_conc._interval+rab_integ ral+RQ_integral+Arg_conc.+emission_CO2+generate_CO2+interva l_CO2; 0.447, 0.762, pH+pH_cumulo+PL+AN+AN_cumulo+OD+Feed_V ol+Cell_conc._interval+RQ_integral+Arg_conc.+emission_CO2+c onsum_O2+interval_O2; 0.485, 0.762, pH_cumulo+PL+AN_cumulo+ OD+Feed_Vol+Cell_conc._interval+RQ_integral+Arg_conc.+emiss ion_O2+consum_O2; 0.473, 0.762, pH_cumulo+PL+AN_cumulo+inte rval_yield+OD+Feed_Vol+RS_cumulo+Cell_conc._interval+Arg_co nc.+emission_CO2+consum_O2; 0.433, 0.762, pH_cumulo+PL_cumu lo+AN+AN_cumulo+interval_yield+OD+Cell_conc._interval+rab_i ntegral+RQ_integral+Accum._Yield+Arg_conc.+emission_O2+cons um_O2+interval_CO2; 0.433, 0.762, pH_cumulo+PL+AN+AN_cumulo +interval_yield+OD+Feed_Vol+Cell_conc._interval+rab_integra l+RQ_integral+Arg_conc.+emission_CO2+consum_O2+generate_CO 2; 0.433, 0.762, tmp_cumulo+pH_cumulo+AN+AN_cumulo+interval _yield+OD+Cell_conc._interval+rab_integral+RQ_integral+Arg_ conc.+emission_O2+consum_O2+interval_O2+interval_CO2; 0.403, 0.762, tmp_cumulo+pH_cumulo+PL+PL_cumulo+AN+AN_cumulo+inte rval_yield+OD+Feed_Vol+Cell_conc._interval+rab_integral+RQ_ integral+Arg_conc.+emission_O2+consum_O2+interval_O2; 0.418, 0.762, pH+pH_cumulo+PL_cumulo+AN+AN_cumulo+interval_yield+ OD+Feed_Vol+Cell_conc._interval+rab_integral+RQ_integral+Ac cum._Yield+Arg_conc.+emission_O2+consum_O2; 0.433, 0.762, t mp_cumulo+pH_cumulo+AN+AN_cumulo+interval_yield+OD+Cell_con c._interval+rab_integral+RQ_integral+Arg_conc.+emission_CO2 +consum_O2+generate_CO2+interval_CO2; 0.433, 0.762, tmp+pH_ cumulo+PL+PL_cumulo+AN+AN_cumulo+OD+Cell_conc._interval+rab _integral+RQ_integral+Arg_conc.+emission_O2+consum_O2+inter val_CO2; 0.447, 0.762, pH_cumulo+AN+AN_cumulo+OD+Feed_Vol+C ell_conc._interval+RQ_integral+Arg_conc.+emission_O2+emissi on_CO2+consum_O2+interval_O2+generate_CO2; 0.460, 0.762, pH _cumulo+AN+AN_cumulo+interval_yield+OD+Feed_Vol+Cell_conc._ interval+Arg_conc.+emission_CO2+consum_O2+generate_CO2+inte rval_CO2; 0.418, 0.762, tmp_cumulo+pH+pH_cumulo+PL_cumulo+A N+AN_cumulo+OD+Feed_Vol+Cell_conc._interval+rab_integral+RQ integral+Arg_conc.+emission_O2+consum_O2+interval_CO2; 0.4 33, 0.762, pH_cumulo+PL+AN+AN_cumulo+OD+Feed_Vol+RS_cumulo+ Cell_conc._interval+rab_integral+RQ_integral+Arg_conc.+emis sion_O2+consum_O2+interval_O2; 0.433, 0.762, pH_cumulo+PL+A N+AN_cumulo+interval_yield+OD+RS_cumulo+Cell_conc._interval +rab_integral+RQ_integral+Arg_conc.+emission_CO2+consum_O2+ interval_CO2; 0.447, 0.762, tmp+pH_cumulo+PL_cumulo+AN+AN_c umulo+interval_yield+OD+Cell_conc._interval+Arg_conc.+emiss ion_CO2+consum_O2+generate_CO2+interval_CO2; 0.433, 0.762, pH_cumulo+PL_cumulo+AN+AN_cumulo+interval_yield+OD+RS+Cell_ conc._interval+rab_integral+RQ_integral+Arg_conc.+emission_ CO2+generate_CO2+interval_CO2; 0.418, 0.762, pH_cumulo+AN+A N_cumulo+interval_yield+OD+Feed_Vol+Cell_conc._interval+rab _integral+RQ_integral+Arg_conc.+emission_O2+emission_CO2+co nsum_O2+generate_CO2+interval_CO2; 0.472, 0.762, tmp+pH_cum ulo+AN+AN_cumulo+OD+Feed_Vol+Cell_conc._interval+rab_integr al+Arg_conc.+emission_CO2+consum_O2; 0.447, 0.762, pH_cumul o+PL_cumulo+AN+AN_cumulo+interval_yield+OD+Cell_conc._inter val+Arg_conc.+emission_O2+emission_CO2+consum_O2+generate_C O2+interval_CO2; 0.460, 0.762, pH+pH_cumulo+PL_cumulo+AN+AN _cumulo+OD+Feed_Vol+Cell_conc._interval+RQ_integral+Arg_con c.+emission_O2+consum _O2; 0.387, 0.762, tmp_cumulo+pH+pH_cu mulo+PL_cumulo+AN+AN_cumulo+interval_yield+OD+Cell_conc._in terval+rab_integral+RQ_integral+Arg_conc.+emission_O2+emiss ion_CO2+consum_O2+generate_CO2+interval_CO2; 0.418, 0.762, tmp_cumulo+pH_cumulo+PL+AN+AN_cumulo+interval_yield+OD+Cell _conc._interval+rab_integral+RQ_integral+Arg_conc.+emission _O2+consum_O2+generate_CO2+interval_CO2; 0.403, 0.762, pH_c umulo+PL+PL_cumulo+AN+AN_cumulo+interval_yield+OD+RS+RS_cum ulo+Cell_conc._interval+rab_integral+RQ_integral+Arg_conc.+ emission_O2+consum_O2+interval_CO2; 0.484, 0.761, pH_cumulo +PL+AN_cumulo+OD+Feed_Vol+Cell_conc._interval+RQ_integral+A rg_conc.+emission_CO2+consum_O2; 0.418, 0.761, pH+pH_cumulo +AN+AN_cumulo+interval_yield+OD+Feed_Vol+Cell_conc._interva l+rab_integral+RQ_integral+Arg_conc.+emission_O2+emission_C O2+consum_O2+interval_CO2; 0.447, 0.761, pH+pH_cumulo+PL_cu mulo+AN+AN_cumulo+OD+Feed_Vol+Cell_conc._interval+RQ_integr al+Arg_conc.+emission_O2+consum_O2+interval_O2; 0.460, 0.76 1, pH_cumulo+PL+AN+AN_cumulo+interval_yield+OD+Cell_conc._i nterval+rab_integral+RQ_integral+Arg_conc.+emission_CO2+con sum_O2; 0.472, 0.761, tmp+pH_cumulo+AN+AN_cumulo+OD+Cell_co nc._interval+Arg_conc.+emission_CO2+consum_O2+generate_CO2+ interval_CO2; 0.418, 0.761, tmp+pH_cumulo+PL+AN+AN_cumulo+i nterval_yield+OD+Feed_Vol+RS_cumulo+Cell_conc._interval+rab _integral+RQ_integral+Arg_conc.+emission_O2+consum _O2; 0.41 8, 0.761, pH_cumulo+PL_cumulo+AN+AN_cumulo+interval_yield+O D+RS+Cell_conc._interval+rab_integral+RQ_integral+Arg_conc. +emission_O2+emission_CO2+consum_O2+interval_CO2; 0.472, 0. 761, pH_cumulo+PL_cumulo+AN+AN_cumulo+OD+Feed_Vol+RS_cumulo +Cell_conc._interval+Arg_conc.+emission_CO2+consum_O2; 0.41 8, 0.761, tmp_cumulo+pH_cumulo+PL+AN+AN_cumulo+interval_yie ld+OD+Cell_conc._interval+rab_integral+RQ_integral+Arg_conc. +emission_O2+interval_O2+generate_CO2+interval_CO2; 0.447, 0.761, tmp_cumulo+pH+pH_cumulo+AN+AN_cumulo+interval_yield+ OD+Cell_conc._interval+Arg_conc.+emission_CO2+interval_O2+g enerate_CO2+interval_CO2; 0.447, 0.761, tmp+pH_cumulo+AN+AN _cumulo+interval_yield+OD+Cell_conc._interval+rab_integral+ RQ_integral+Arg_conc.+emission_O2+consum_O2+interval_CO2; 0. 403, 0.761, pH_cumulo+PL_cumulo+AN+AN_cumulo+interval_yield +OD+Feed_Vol+Cell_conc._interval+rab_integral+RQ_integral+A ccum._Yield+Arg_conc.+emission_O2+consum_O2+generate_CO2+in terval_CO2; 0.403, 0.761, pH_cumulo+PL_cumulo+AN+AN_cumulo+ interval_yield+OD+Feed_Vol+Cell_conc._interval+rab_integral +RQ_integral+Arg_conc.+emission_O2+emission_CO2+interval_O2 +generate_CO2+interval_CO2; 0.472, 0.761, pH+pH_cumulo+AN+A N_cumulo+OD+Cell_conc._interval+Arg_conc.+emission_CO2+cons um_O2+interval_O2+interval_CO2; 0.433, 0.761, tmp+pH_cumulo +AN+AN_cumulo+interval_yield+OD+Cell_conc._interval+rab-int egral+RQ_integral+Arg_conc.+emission_O2+interval_O2+generat e_CO2+interval_CO2; 0.387, 0.761, tmp+tmp_cumulo+pH_cumulo+ PL+PL_cumulo+AN+AN_cumulo+interval_yield+OD+Cell_conc._inte rval+rab_integral+RQ_integral+Arg_conc.+emission_O2+consum_ O2+interval_O2+interval_CO2; 0.387, 0.761, pH+pH_cumulo+PL+ PL_cumulo+AN+AN_cumulo+interval_yield+OD+Cell_conc._interva l+rab_integral+RQ_integral+Arg_conc.+emission_O2+emission_C O2+consum_O2+interval_O2+interval_CO2; 0.460, 0.761, pH+pH_ cumulo+AN+AN_cumulo+OD+Feed_Vol+Cell_conc._interval+Arg_con c.+emission_CO2+consum_O2+generate_CO2+interval_CO2; 0.403, 0.761, pH_cumulo+PL_cumulo+AN+AN_cumulo+interval_yield+OD+ Feed_Vol+RS_cumulo+Cell_conc._interval+rab_integral+RQ_inte gral+Arg_conc.+emission_O2+interval_O2+generate_CO2+interva l_CO2; 0.446, 0.761, pH_cumulo+AN+AN_cumulo+interval_yield+ OD+Feed_Vol+Cell_conc._interval+RQ_integral+Arg_conc.+emiss ion_O2+emission_CO2+consum_O2+interval_O2; 0.403, 0.761, pH _cumulo+PL+PL_cumulo+AN+AN_cumulo+interval_yield+OD+Feed_Vo l+RS_cumulo+Cell_conc._interval+rab_integral+RQ_integral+Ac cum._Yield+Arg_conc.+emission_O2+consum_O2; 0.446, 0.761, t mp_cumulo+pH_cumulo+PL+AN+AN_cumulo+OD+Cell_conc._interval+ rab_integral+RQ_integral+Arg_conc.+emission_O2+consum_O2+in terval_CO2; 0.387, 0.761, pH_cumulo+PL+PL_cumulo+AN+AN_cumu lo+interval_yield+OD+Feed_Vol+Cell_conc._interval+rab_integ ral+RQ_integral+Arg_conc.+emission_O2+consum_O2+interval_O2 +generate_CO2+interval_CO2; 0.387, 0.761, tmp+pH+pH_cumulo+ PL+PL_cumulo+AN+AN_cumulo+interval_yield+OD+Feed_Vol+Cell_c onc._interval+rab_integral+RQ_integral+Arg_conc.+emission_O 2+consum_O2+interval_CO2; 0.472, 0.761, pH_cumulo+AN+AN_cum ulo+OD+Feed_Vol+RS_cumulo+Cell_conc._interval+RQ_integral+A rg_conc.+emission_O2+consum_O2; 0.446, 0.761, pH_cumulo+PL+ PL_cumulo+AN+AN_cumulo+OD+Feed_Vol+Cell_conc._interval+rab_ integral+RQ_integral+Arg_conc.+emission_CO2+consum_O2; 0.40 3, 0.761, tmp+pH_cumulo+PL_cumulo+AN+AN_cumulo+interval_yie ld+OD+RS_cumulo+Cell_conc._interval+rab_integral+RQ_integra l+Arg_conc.+emission_O2+consum_O2+generate_CO2+interval_CO 2; 0.418, 0.761, pH_cumulo+PL+PL_cumulo+AN+AN_cumulo+OD+Fee d_Vol+Cell_conc._interval+rab_integral+RQ_integral+Arg_conc. +emission_O2+consum_O2+interval_O2+interval_CO2; 0.446, 0.7 61, pH_cumulo+AN+AN_cumulo+OD+Feed_Vol+RS_cumulo+Cell_conc. _interval+rab_integral+RQ_integral+Arg_conc.+emission_O2+co nsum_O2+generate_CO2; 0.460, 0.761, pH_cumulo+PL_cumulo+AN+ AN_cumulo+interval_yield+OD+Feed_Vol+Cell_conc._interval+Ar g_conc.+emission_CO2+generate_CO2+interval_CO2; 0.472, 0.76 1, pH_cumulo+AN+AN_cumulo+interval_yield+OD+Feed_Vol+Cell_c onc._interval+Arg_conc.+emission_CO2+consum_O2+generate_CO 2; 0.387, 0.761, tmp_cumulo+pH_cumulo+PL+PL_cumulo+AN+AN_cu mulo+interval_yield+OD+Feed_Vol+Cell_conc._interval+rab_int egral+RQ_integral+Arg_conc.+emission_O2+consum_O2+generate_ CO2+interval_CO2; 0.460, 0.761, tmp_cumulo+pH_cumulo+AN+AN_ cumulo+OD+Cell_conc._interval+rab_integral+RQ_integral+Arg_ conc.+emission_CO2+interval_O2+interval_CO2; 0.460, 0.761, pH+pH_cumulo+AN_cumulo+interval_yield+OD+Feed_Vol+Cell_conc. _interval+rab_integral+RQ_integral+Arg_conc.+emission_O2+co nsum_O2; 0.386, 0.761, pH_cumulo+PL+PL_cumulo+AN+AN_cumulo+ interval_yield+OD+RS_cumulo+Cell_conc._interval+rab_integra l+RQ_integral+Arg_conc.+emission_O2+consum_O2+interval_O2+g enerate_CO2+interval_CO2; 0.433, 0.761, pH_cumulo+AN+AN_cum ulo+interval_yield+OD+Cell_conc._interval+rab_integral+RQ_i ntegral+Arg_conc.+emission_O2+emission_CO2+consum_O2+genera te_CO2+interval_CO2; 0.459, 0.761, pH_cumulo+AN+AN_cumulo+O D+Feed_Vol+Cell_conc._interval+rab_integral+RQ_integral+Acc um._Yield+Arg_conc.+emission_O2+consum_O2; 0.446, 0.761, pH +pH_cumulo+PL+AN+AN_cumulo+OD+Feed_Vol+Cell_conc._interval+ RQ_integral+Arg_conc.+emission_CO2+consum_O2+generate_CO2; 0.403, 0.761, pH+pH_cumulo+PL+AN+AN_cumulo+interval_yield+O D+RS_cumulo+Cell_conc._interval+rab_integral+RQ_integral+Ar g_conc.+emission_O2+interval_O2+generate_CO2+interval_CO2; 0.418, 0.761, tmp_cumulo+pH_cumulo+PL+PL_cumulo+AN+AN_cumul o+OD+Feed_Vol+Cell_conc._interval+rab_integral+RQ_integral+ Arg_conc.+emission_O2+consum_O2+interval_CO2; 0.459, 0.761, pH+pH_cumulo+AN+AN_cumulo+OD+Feed_Vol+Cell_conc._interval+ rab_integral+Arg_conc.+emission_O2+emission_CO2+consum_O2; 0.446, 0.761, pH_cumulo+AN+AN_cumulo+OD+Feed_Vol+Cell_conc. _interval+rab_integral+RQ_integral+Arg_conc.+emission_O2+co nsum_O2+generate_CO2+interval_CO2; 0.386, 0.761, pH+pH_cumu lo+PL+PL_cumulo+AN+AN_cumulo+interval_yield+OD+Feed_Vol+Cel l_conc._interval+rab_integral+RQ_integral+Arg_conc.+emissio n_O2+emission_CO2+consum_O2+interval_O2; 0.459, 0.761, pH_c umulo+AN+AN_cumulo+OD+Feed_Vol+RS_cumulo+Cell_conc._interva l+RQ_integral+Arg_conc.+emission_CO2+consum_O2+generate_CO 2; 0.386, 0.761, tmp+pH_cumulo+PL+PL_cumulo+AN+AN_cumulo+in terval_yield+OD+Feed_Vol+Cell_conc._interval+rab_integral+R Q_integral+Arg_conc.+emission_O2+consum_O2+interval_O2+inte rval_CO2; 0.472, 0.761, pH_cumulo+AN+AN_cumulo+OD+Cell_conc. _interval+rab_integral+RQ_integral+Arg_conc.+emission_CO2+c onsum_O2+interval_CO2; 0.446, 0.761, pH_cumulo+PL+PL_cumulo +AN+AN_cumulo+interval_yield+OD+Cell_conc._interval+Arg_con c.+emission_CO2+interval_O2+generate_CO2+interval_CO2; 0.38 6, 0.761, pH+pH_cumulo+PL+PL_cumulo+AN+AN_cumulo+interval_y ield+OD+RS_cumulo+Cell_conc._interval+rab_integral+RQ_integ ral+Arg_conc.+emission_O2+interval_O2+generate_CO2+interval _CO2; 0.446, 0.761, pH_cumulo+PL+AN+AN_cumulo+OD+Feed_Vol+C ell_conc._interval+rab_integral+RQ_integral+Arg_conc.+emiss ion_CO2+consum_O2+interval_CO2; 0.459, 0.761, tmp_cumulo+pH +pH_cumulo+AN+AN_cumulo+OD+Cell_conc._interval+rab_integral +Arg_conc.+emission_CO2+consum_O2+interval_CO2; 0.402, 0.76 1, tmp_cumulo+pH+pH_cumulo+PL_cumulo+AN+AN_cumulo+interval_ yield+OD+Feed_Vol+Cell_conc._interval+rab_integral+RQ_integ ral+Arg_conc.+emission_O2+consum_O2+interval_CO2; 0.459, 0. 761, pH_cumulo+PL_cumulo+AN+AN_cumulo+interval_yield+OD+Fee d_Vol+Cell_conc._interval+Arg_conc.+emission_CO2+consum_O2+ interval_O2; 0.484, 0.761, pH_cumulo+AN_cumulo+OD+Feed_Vol+ Cell_conc._interval+Arg_conc.+emission_CO2+consum_O2+interv al_O2+generate_CO2; 0.459, 0.761, pH_cumulo+PL+AN+AN_cumulo +OD+Feed_Vol+Cell_conc._interval+rab_integral+Arg_conc.+emi ssion_CO2+consum_O2+interval_CO2; 0.418, 0.761, tmp+pH_cumu lo+PL+AN+AN_cumulo+interval_yield+OD+RS_cumulo+Cell_conc._i nterval+rab_integral+RQ_integral+Arg_conc.+emission_O2+cons um_O2+interval_CO2; 0.418, 0.761, pH_cumulo+PL+PL_cumulo+AN +AN_cumulo+interval_yield+OD+Cell_conc._interval+rab_integr al+RQ_integral+Arg_conc.+emission_CO2+consum_O2+interval_O2 +interval_CO2; 0.459, 0.761, tmp_cumulo+pH_cumulo+AN+AN_cum ulo+OD+Cell_conc._interval+Arg_conc.+emission_O2+emission_C O2+interval_O2+generate_CO2+interval_CO2; 0.459, 0.761, tmp _cumulo+pH_cumulo+PL_cumulo+AN+AN_cumulo+interval_yield+OD+ Cell_conc._interval+Arg_conc.+emission_CO2+generate_CO2+int erval_CO2; 0.402, 0.761, tmp+tmp_cumulo+pH+pH_cumulo+PL_cum ulo+AN+AN_cumulo+interval_yield+OD+Cell_conc._interval+rab_ integral+RQ_integral+Arg_conc.+emission_O2+consum_O2+interv al_CO2; 0.484, 0.761, pH_cumulo+PL_cumulo+AN_cumulo+OD+Feed _Vol+Cell_conc._interval+RQ_integral+Arg_conc.+emission_O2+ consum_O2; 0.459, 0.761, pH_cumulo+PL_cumulo+AN+AN_cumulo+i nterval_yield+OD+Cell_conc._interval+Arg_conc.+emission_O2+ emission_CO2+generate_CO2+interval_CO2; 0.432, 0.761, tmp+p H+pH_cumulo+PL+AN+AN_cumulo+OD+Feed_Vol+Cell_conc._interval +rab_integral+RQ_integral+Arg_conc.+emission_O2+consum_O2 ; 0.459, 0.761, tmp+pH_cumulo+PL+AN+AN_cumulo+OD+Feed_Vol+Cel l_conc._interval+RQ_integral+Arg_conc.+emission_CO2+consum_ 02; 0.459, 0.761, pH_cumulo+AN+AN_cumulo+OD+Feed_Vol+RS_cum ulo+Cell_conc._interval+RQ_integral+Arg_conc.+emission_CO2+ consum_O2+interval_O2; 0.459, 0.761, tmp+pH+pH_cumulo+PL+AN +AN_cumulo+OD+Feed_Vol+Cell_conc._interval+Arg_conc.+emissi on_CO2+consum_O2; 0.446, 0.761, pH_cumulo+AN+AN_cumulo+inte rval_yield+OD+Feed_Vol+Cell_conc._interval+RQ_integral+Arg_ conc.+emission_CO2+consum_O2+generate_CO2+interval_CO2 0.4 46, 0.761, tmp_cumulo+pH_cumulo+PL+AN+AN_cumulo+OD+Cell_con c._interval+rab_integral+RQ_integral+Arg_conc.+emission_O2+ interval_O2+interval_CO2; 0.484, 0.761, pH_cumulo+AN+AN_cum ulo+OD+Cell_conc._interval+rab_integral+Arg_conc.+emission_ CO2+consum_O2+interval_CO2; 0.495, 0.761, tmp_cumulo+pH_cum ulo+AN_cumulo+OD+Feed_Vol+Cell_conc._interval+Arg_conc.+emi ssion_CO2+consum_O2; 0.402, 0.761, pH_cumulo+PL_cumulo+AN+A N_cumulo+interval_yield+OD+RS_cumulo+Cell_conc._interval+ra b_integral+RQ_integral+Arg_conc.+emission_O2+emission_CO2+c onsum_O2+generate_CO2+interval_CO2; 0.402, 0.761, pH_cumulo +PL+PL_cumulo+AN+AN_cumulo+interval_yield+OD+RS+Cell_conc._ interval+rab_integral+RQ_integral+Arg_conc.+emission_O2+emi ssion_CO2+consum_O2+interval_CO2; 0.459, 0.761, tmp_cumulo+ pH_cumulo+AN+AN_cumulo+OD+Cell_conc._interval+Arg_conc.+emi ssion_CO2+consum_O2+interval_O2+generate_CO2+interval_CO2; 0.432, 0.761, pH+pH_cumulo+AN+AN_cumulo+OD+Feed_Vol+RS_cumu lo+Cell_conc._interval+rab_integral+RQ_integral+Arg_conc.+e mission_O2+emission_CO2+consum_O2; 0.417, 0.761, pH+pH_cumu lo+PL+PL_cumulo+AN+AN_cumulo+OD+Feed_Vol+Cell_conc._interva l+rab_integral+RQ_integral+Arg_conc.+emission_O2+consum _02+ generate_CO2; 0.386, 0.761, pH_cumulo+PL+PL_cumulo+AN+AN_cu mulo+interval_yield+OD+Feed_Vol+Cell_conc._interval+rab_int egral+RQ_integral+Arg_conc.+emission_O2+emission_CO2+consum _O2+interval_O2+interval_CO2; 0.484, 0.761, pH_cumulo+AN_cu mulo+OD+Feed_Vol+Cell_conc._interval+rab_integral+Arg_conc. +emission_O2+emission_CO2+consum_O2; 0.386, 0.761, tmp+pH+p H_cumulo+PL+PL_cumulo+AN+AN_cumulo+interval_yield+OD+Feed_V ol+Cell_conc._interval+rab_integral+RQ_integral+Arg_conc.+e mission_O2+consum_O2+interval_O2; 0.472, 0.761, tmp+pH_cumu lo+AN+AN_cumulo+OD+Feed_Vol+Cell_conc._interval+Arg_conc.+e mission_O2+emission_CO2+consum_O2; 0.402, 0.761, pH_cumulo+ PL+AN+AN_cumulo+interval_yield+OD+Feed_Vol+RS_cumulo+Cell_c onc._interval+rab_integral+RQ_integral+Accum._Yield+Arg_con c.+emission_O2+consum _O2+interval_CO2; 0.472, 0.761, pH_cum ulo+AN_cumulo+OD+Feed_Vol+Cell_conc._interval+rab_integral+ RQ_integral+Arg_conc.+emission_O2+emission_CO2+consum _O2; 0. 446, 0.761, tmp_cumulo+pH_cumulo+AN+AN_cumulo+OD+Cell_conc. _interval+rab_integral+RQ_integral+Arg_conc.+emission_O2+in terval_O2+generate_CO2+interval_CO2; 0.483, 0.761, pH_cumul o+AN_cumulo+interval_yield+OD+Feed_Vol+Cell_conc._interval+ Arg_conc.+emission_O2+emission_CO2+consum_O2; 0.432, 0.761, tmp_cumulo+pH_cumulo+PL+AN+AN_cumulo+OD+Feed_Vol+Cell_conc. _interval+rab_integral+RQ_integral+Arg_conc.+emission_O2+co nsum_O2+interval_CO2; 0.483, 0.761, pH_cumulo+PL_cumulo+AN_ cumulo+OD+Feed_Vol+RS_cumulo+Cell_conc._interval+Arg_conc.+ emission_CO2+consum_O2; 0.432, 0.761, pH_cumulo+PL_cumulo+A N+AN_cumulo+OD+Feed_Vol+Cell_conc._interval+rab_integral+RQ _integral+Arg_conc.+emission_O2+consum_O2+interval_O2+gener ate_CO2; 0.446, 0.761, pH+pH_cumulo+PL+AN+AN_cumulo+interva l_yield+OD+Cell_conc._interval+Arg_conc.+emission_CO2+inter val_O2+generate_CO2+interval_CO2; 0.446, 0.761, pH_cumulo+P L+AN+AN_cumulo+OD+Feed_Vol+RS_cumulo+Cell_conc._interval+RQ _integral+Arg_conc.+emission_O2+emission_CO2+consum_02; 0.4 32, 0.761, tmp_cumulo+pH_cumulo+PL_cumulo+AN+AN_cumulo+inte rval_yield+OD+Feed_Vol+Cell_conc._interval+rab_integral+RQ_ integral+Arg_conc.+emission_O2+consum_O2; 0.471, 0.761, pH_ cumulo+AN+AN_cumulo+OD+Feed_Vol+RS_cumulo+Cell_conc._interv al+Arg_conc.+emission_CO2+consum_O2+generate_CO2; 0.402, 0. 761, tmp+pH+pH_cumulo+PL_cumulo+AN+AN_cumulo+interval_yield +OD+Feed_Vol+Cell_conc._interval+rab_integral+RQ_integral+A rg_conc.+emission_O2+consum_O2+interval_CO2; 0.386, 0.761, pH_cumulo+PL+PL_cumulo+AN+AN_cumulo+interval_yield+OD+Feed_ Vol+RS_cumulo+Cell_conc._interval+rab_integral+RQ_integral+ Accum._Yield+Arg_conc.+emission_O2+consum_O2+interval_CO2; 0.459, 0.761, pH+pH_cumulo+PL+AN+AN_cumulo+OD+Cell_conc._in terval+Arg_conc.+emission_CO2+consum_O2+generate_CO2+interv al_CO2; 0.417, 0.761, tmp+tmp_cumulo+pH+pH_cumulo+AN+AN_cum ulo+interval_yield+OD+Cell_conc._interval+rab_integral+RQ_i ntegral+Arg_conc.+emission_O2+consum _O2+interval_CO2; 0.459, 0.761, pH_cumulo+PL+AN+AN_cumulo+OD+Feed_Vol+RS_cumulo+Cel l_conc._interval+rab_integral+Arg_conc.+emission_CO2+consum _O2; 0.471, 0.761, pH_cumulo+AN+AN_cumulo+OD+Feed_Vol+RS_cu mulo+Cell_conc._interval+Arg_conc.+emission_CO2+consum_O2+i nterval_O2; 0.459, 0.761, pH_cumulo+PL+PL_cumulo+AN+AN_cumu lo+OD+Feed_Vol+Cell_conc._interval+RQ_integral+Arg_conc.+em ission_O2+consum_O2; 0.459, 0.761, tmp_cumulo+pH_cumulo+PL+ AN+AN_cumulo+OD+Cell_conc._interval+rab_integral+Arg_conc.+ emission_CO2+consum_O2+interval_CO2; 0.432, 0.761, pH_cumul o+PL+AN+AN_cumulo+OD+Feed_Vol+Cell_conc._interval+rab_integ ral+RQ_integral+Arg_conc.+emission_O2+consum_O2+interval_O2 +generate_CO2; 0.459, 0.761, pH_cumulo+PL+AN+AN_cumulo+OD+F eed_Vol+Cell_conc._interval+Arg_conc.+emission_O2+emission_ CO2+consum_O2+generate_CO2; 0.471, 0.761, pH+pH_cumulo+PL+P L_cumulo+AN_cumulo+OD+Feed_Vol+Cell_conc._interval+Arg_conc. +emission_CO2+consum_02; 0.432, 0.761, pH+pH_cumulo+AN+AN_c umulo+interval_yield+OD+Feed_Vol+Cell_conc._interval+rab_in tegral+RQ_integral+Arg_conc.+emission_O2+interval_O2+genera te_CO2; 0.432, 0.761, tmp_cumulo+pH+pH_cumulo+AN+AN_cumulo+ interval_yield+OD+Feed_Vol+Cell_conc._interval+rab_integral +RQ_integral+Arg_conc.+emission_O2+consum_O2; 0.446, 0.761, tmp_cumulo+pH_cumulo+PL+AN+AN_cumulo+interval_yield+OD+Cel l_conc._interval+Arg_conc.+emission_CO2+consum_O2+generate_ CO2+interval_CO2; 0.386, 0.761, pH_cumulo+PL+PL_cumulo+AN+A N_cumulo+interval_yield+OD+RS_cumulo+Cell_conc._interval+ra b_integral+RQ_integral+Arg_conc.+emission_O2+emission_CO2+c onsum_O2+generate_CO2+interval_CO2; 0.459, 0.761, pH+pH_cum ulo+PL_cumulo+AN+AN_cumulo+OD+Cell_conc._interval+Arg_conc. +emission_CO2+interval_O2+generate_CO2+interval_CO2; 0.483, 0.761, pH_cumulo+AN+AN_cumulo+OD+RS+Cell_conc._interval+Ar g_conc.+emission_CO2+consum_O2+interval_CO2; 0.471, 0.761, tmp+pH+pH_cumulo+AN+AN_cumulo+OD+Cell_conc._interval+Arg_co nc.+emission_CO2+consum_O2+interval_CO2; 0.459, 0.761, tmp_ cumulo+pH_cumulo+PL+AN+AN_cumulo+interval_yield+OD+Cell_con c._interval+Arg_conc.+emission_CO2+consum_O2+interval_CO2; 0.417, 0.761, pH_cumulo+PL+PL_cumulo+AN+AN_cumulo+interval_ yield+OD+Feed_Vol+Cell_conc._interval+rab_integral+RQ_integ ral+Arg_conc.+emission_CO2+consum_O2+interval_O2; 0.402, 0. 761, tmp+pH_cumulo+PL+PL_cumulo+AN+AN_cumulo+interval_yield +OD+RS+Cell_conc._interval+rab_integral+RQ_integral+Arg_con c.+emission_O2+consum_O2+interval_CO2; 0.471, 0.761, tmp+pH _cumulo+PL_cumulo+AN+AN_cumulo+OD+Feed_Vol+Cell_conc._inter val+Arg_conc.+emission_CO2+consum_O2; 0.432, 0.761, pH+pH_c umulo+AN+AN_cumulo+OD+Feed_Vol+Cell_conc._interval+rab_inte gral+RQ_integral+Arg_conc.+emission_O2+interval_O2+generate _CO2+interval_CO2; 0.459, 0.761, pH_cumulo+PL_cumulo+AN_cum ulo+OD+Feed_Vol+RS_cumulo+Cell_conc._interval+rab_integral+ RQ_integral+Arg_conc.+emission_O2+consum_O2; 0.459, 0.761, tmp+pH_cumulo+AN+AN_cumulo+interval_yield+OD+Cell_conc._int erval+Arg_conc.+emission_CO2+interval_O2+generate_CO2+inter val_CO2; 0.445, 0.761, pH_cumulo+PL+AN+AN_cumulo+interval_y ield+OD+RS_cumulo+rab_integral+RQ_integral+Arg_conc.+emissi on_O2+consum_O2+interval_CO2; 0.402, 0.761, tmp+pH_cumulo+P L_cumulo+AN+AN_cumulo+interval_yield+OD+Cell_conc._interval +rab_integral+RQ_integral+Arg_conc.+emission_O2+emission_CO 2+consum_O2+generate_CO2+interval_CO2; 0.445, 0.761, tmp_cu mulo+pH_cumulo+PL_cumulo+AN+AN_cumulo+interval_yield+OD+Cel l_conc._interval+Arg_conc.+emission_CO2+interval_O2+generat e_CO2+interval_CO2; 0.432, 0.761, tmp+pH+pH_cumulo+PL_cumul o+AN+AN_cumulo+OD+Cell_conc._interval+rab_integral+RQ_integ ral+Arg_conc.+emission_O2+consum_O2+interval_CO2; 0.459, 0. 761, pH_cumulo+PL+PL_cumulo+AN+AN_cumulo+OD+Feed_Vol+Cell_c onc._interval+Arg_conc.+emission_CO2+consum_O2+interval_CO 2; 0.432, 0.761, pH_cumulo+PL+AN+AN_cumulo+OD+Feed_Vol+Cell _conc._interval+rab_integral+RQ_integral+Arg_conc.+emission _O2+consum_O2+interval_O2+interval_CO2; 0.459, 0.761, tmp+p H_cumulo+PL+AN+AN_cumulo+OD+Cell_conc._interval+Arg_conc.+e mission_CO2+interval_O2+generate_CO2+interval_CO2; 0.445, 0. 761, pH_cumulo+AN+AN_cumulo+interval_yield+OD+Feed_Vol+Cell _conc._interval+RQ_integral+Arg_conc.+emission_CO2+interval _O2+generate_CO2+interval_CO2; 0.432, 0.761, pH_cumulo+PL+A N+AN_cumulo+OD+Feed_Vol+Cell_conc._interval+rab_integral+RQ _integral+Arg_conc.+emission_O2+interval_O2+generate_CO2+in terval_CO2; 0.432, 0.761, pH_cumulo+PL_cumulo+AN+AN_cumulo+ OD+Feed_Vol+RS_cumulo+Cell_conc._interval+rab_integral+RQ_i ntegral+Arg_conc.+emission_O2+consum_O2+interval_O2; 0.483, 0.761, tmp+pH+pH_cumulo+AN_cumulo+OD+Feed_Vol+Cell_conc._i nterval+Arg_conc.+emission_CO2+consum_O2; 0.459, 0.761, tmp +tmp_cumulo+pH_cumulo+AN+AN_cumulo+OD+Cell_conc._interval+A rg_conc.+emission_CO2+interval_O2+generate_CO2+interval_CO 2; 0.459, 0.761, pH_cumulo+PL_cumulo+AN+AN_cumulo+OD+Feed_V ol+Cell_conc._interval+RQ_integral+Arg_conc.+emission_O2+co nsum_O2+generate_CO2; 0.431, 0.761, pH_cumulo+PL+PL_cumulo+ AN+AN_cumulo+interval_yield+OD+Feed_Vol+Cell_conc._interval +RQ_integral+Arg_conc.+emission_CO2+consum_O2+interval_O2 ; 0.431, 0.761, tmp+pH_cumulo+AN+AN_cumulo+interval_yield+OD+ Feed_Vol+Cell_conc._interval+rab_integral+RQ_integral+Arg_c onc.+emission_O2+emission_CO2+consum_O2; 0.402, 0.761, pH+p H_cumulo+PL+AN+AN_cumulo+interval_yield+OD+Feed_Vol+Cell_co nc._interval+rab_integral+RQ_integral+Arg_conc.+emission_O2 +emission_CO2+consum_O2+interval_CO2; 0.431, 0.761, pH+pH_c umulo+AN+AN_cumulo+interval_yield+OD+Cell_conc._interval+ra b_integral+RQ_integral+Arg_conc.+emission_O2+emission_CO2+c onsum_O2+interval_CO2; 0.471, 0.761, tmp_cumulo+pH_cumulo+A N+AN_cumulo+OD+Feed_Vol+Cell_conc._interval+RQ_integral+Arg _conc.+emission_CO2+consum_O2; 0.385, 0.761, tmp+pH_cumulo+ PL+PL_cumulo+AN+AN_cumulo+interval_yield+OD+RS_cumulo+Cell_ conc._interval+rab_integral+RQ_integral+Arg_conc.+emission_ O2+consum_O2+generate_CO2+interval_CO2; 0.417, 0.761, tmp+p H_cumulo+PL_cumulo+AN+AN_cumulo+interval_yield+OD+Feed_Vol+ Cell_conc._interval+rab_integral+RQ_integral+Arg_conc.+emis sion_O2+consum_O2+generate_CO2; 0.445, 0.761, pH_cumulo+AN+ AN_cumulo+interval_yield+OD+Feed_Vol+Cell_conc._interval+ra b_integral+RQ_integral+Accum._Yield+Arg_conc.+emission_CO2+ consum_O2; 0.471, 0.761, pH+pH_cumulo+PL_cumulo+AN+AN_cumul o+OD+Cell_conc._interval+Arg_conc.+emission_CO2+consum_O2+i nterval_CO2; 0.417, 0.761, tmp+pH_cumulo+PL+AN+AN_cumulo+in terval_yield+OD+Feed_Vol+Cell_conc._interval+rab_integral+R Q_integral+Arg_conc.+emission_O2+emission_CO2+consum_O2; 0. 431, 0.761, pH+pH_cumulo+PL_cumulo+AN+AN_cumulo+interval_yi eld+OD+Feed_Vol+Cell_conc._interval+RQ_integral+Arg_conc.+e mission_CO2+consum_O2+interval_O2; 0.483, 0.761, pH_cumulo+ AN+AN_cumulo+OD+Feed_Vol+Cell_conc._interval+Accum._Yield+A rg_conc.+emission_CO2+consum_O2; 0.385, 0.761, tmp+tmp_cumu lo+pH+pH_cumulo+PL+PL_cumulo+AN+AN_cumulo+interval_yield+OD +Cell_conc._interval+rab_integral+RQ_integral+Arg_conc.+emi ssion_O2+consum_O2+interval_CO2; 0.385, 0.761, pH_cumulo+PL +PL_cumulo+AN+AN_cumulo+interval_yield+OD+RS_cumulo+Cell_co nc._interval+rab_integral+RQ_integral+Arg_conc.+emission_O2 +emission_CO2+consum_O2+interval_O2+interval_CO2; 0.458, 0. 761, pH_cumulo+PL+AN+AN_cumulo+OD+Feed_Vol+Cell_conc._inter val+Arg_conc.+emission_CO2+consum_O2+interval_O2+interval_C 02; 0.401, 0.761, pH_cumulo+PL_cumulo+AN+AN_cumulo+interval _yield+OD+Feed_Vol+Cell_conc._interval+rab_integral+RQ_inte gral+Arg_conc.+emission_O2+emission_CO2+consum_O2+interval_ O2+generate_CO2; 0.431, 0.761, pH_cumulo+PL_cumulo+AN+AN_cu mulo+interval_yield+OD+Feed_Vol+Cell_conc._interval+rab_int egral+RQ_integral+Arg_conc.+emission_CO2+consum_O2+generate _CO2; 0.445, 0.761, tmp_cumulo+pH_cumulo+PL+AN+AN_cumulo+in terval_yield+OD+rab_integral+RQ_integral+Arg_conc.+emission _O2+consum_O2+interval_CO2; 0.445, 0.761, pH+pH_cumulo+PL_c umulo+AN+AN_cumulo+OD+Feed_Vol+Cell_conc._interval+rab_inte gral+RQ_integral+Arg_conc.+emission_CO2+consum_O2; 0.431, 0. 761, pH+pH_cumulo+AN+AN_cumulo+OD+Feed_Vol+Cell_conc._inter val+rab_integral+RQ_integral+Arg_conc.+emission_O2+consum_O 2+interval_O2+generate_CO2; 0.401, 0.761, pH+pH_cumulo+PL_c umulo+AN+AN_cumulo+interval_yield+OD+RS+Cell_conc._interval +rab_integral+RQ_integral+Arg_conc.+emission_O2+consum_O2+i nterval_O2+interval_CO2; 0.445, 0.761, pH+pH_cumulo+AN+AN_c umulo+OD+Cell_conc._interval+rab_integral+RQ_integral+Arg_c onc.+emission_O2+interval_O2+generate_CO2+interval_CO2; 0.4 58, 0.761, pH+pH_cumulo+AN+AN_cumulo+OD+Feed_Vol+RS_cumulo+ Cell_conc._interval+RQ_integral+Arg_conc.+emission_O2+consu m_O2; 0.431, 0.761, pH_cumulo+PL+AN+AN_cumulo+interval_yiel d+OD+RS+Feed_Vol+Cell_conc._interval+rab_integral+RQ_integr al+Arg_conc.+emission_O2+consuni_O2; 0.401, 0.761, tmp_cumul o+pH+pH_cumulo+PL_cumulo+AN+AN_cumulo+interval_yield+OD+Cel l_conc._interval+rab_integral+RQ_integral+Arg_conc.+emissio n_O2+emission_CO2+consum_O2+interval_CO2; 0.401, 0.761, pH_ cumulo+PL_cumulo+AN+AN_cumulo+interval_yield+OD+RS+Feed_Vol +Cell_conc._interval+rab_integral+RQ_integral+Arg_conc.+emi ssion_O2+consum_O2+interval_O2+interval_CO2; 0.445, 0.761, pH_cumulo+PL_cumulo+AN _cumulo+interval_yield+OD+FeedVol+Ce ll_conc._interval+rab_integral+RQ_integral+Arg_conc.+emissi on_O2+consum_O2+generate_CO2; 0.401, 0.761, pH+pH_cumulo+PL _cumulo+AN+AN_cumulo+interval_yield+OD+Feed_Vol+RS_cumulo+C ell_conc._interval+rab_integral+RQ_integral+Arg_conc.+emiss ion_O2+consum_O2+generate_CO2; 0.431, 0.761, pH_cumulo+PL_c umulo+AN+AN_cumulo+OD+Feed _Vol+Cell_conc. _interval+rab_inte gral+RQ_integral+Arg_conc.+emission_O2+interval_O2+generate _CO2+interval_CO2; 0.401, 0.761, pH+pH_cumulo+PL+AN+AN_cumu lo+interval_yield+OD+RS_cumulo+Cell_conc._interval+rab_inte gral+RQ_integral+Arg_conc.+emission_O2+emission_CO2+consum_ O2+interval_CO2; 0.471, 0.761, pH_cumulo+PL+AN_cumulo+inter val_yield+OD+Feed_Vol+Cell_conc._interval+Arg_conc.+emissio n_O2+emission_CO2+consum_O2; 0.471, 0.761, tmp_cumulo+pH_cu mulo+AN+AN_cumulo+interval_yield+OD+Cell_conc._interval+Arg _conc.+emission_CO2+consum_O2+interval_CO2; 0.445, 0.761, t mp+pH_cumulo+AN+AN_cumulo+OD+Feed_Vol+Cell_conc._interval+r ab_integral+RQ_integral+Arg_conc.+emission_O2+consum_O2+int erval_CO2; 0.401, 0.761, pH_cumulo+PL+PL_cumulo+AN+AN_cumul o+interval_yield+OD+Feed_Vol+Cell_conc._interval+rab_integr al+RQ_integral+Arg_conc.+emission_O2+emission_CO2+consum_O2 +generate_CO2; 0.458, 0.761, pH_cumulo+PL+AN+AN_cumulo+OD+F eed_Vol+Cell_conc._interval+Arg_conc.+emission_O2+emission-CO2+consum_O2+interval_O2; 0.458, 0.761, tmp+pH_cumulo+PL+A N+AN_cumulo+interval_yield+OD+Feed_Vol+Cell_conc._interval+ Arg_conc.+emission_CO2+consum_O2; 0.445, 0.761, pH+pH_cumul o+PL+PL_cumulo+AN_cumulo+OD+Feed_Vol+Cell_conc._interval+ra b_integral+RQ_integral+Arg_conc.+emission_O2+consum_O2; 0.4 58, 0.761, pH_cumulo+PL_cumulo+AN+AN_cumulo+OD+Feed_Vol+Cel 1_conc. _interval+rab _integral+Arg_conc.+emission _CO2+consum _O2+interval_O2; 0.458, 0.761, pH_cumulo+PL+AN+AN_cumulo+OD +Feed_Vol+RS_cumulo+Cell_conc._interval+Arg_conc.+emission_ O2+emission_CO2+consum_O2; 0.458, 0.761, pH+pH_cumulo+AN+AN _cumulo+OD+Feed_Vol+Cell_conc._interval+RQ_integral+Arg_con c.+emission_CO2+consum_O2+interval_CO2; 0.385, 0.761, pH+pH _cumulo+PL_cumulo+AN+AN_cumulo+interval_yield+OD+Feed_Vol+C ell_conc._interval+rab_integral+RQ_integral+Arg_conc.+emiss ion _O2+emission_CO2+consum_O2+interval_O2+interval_CO2; 0.4 58, 0.761, tmp_cumulo+pH_cumulo+AN+AN_cumulo+OD+Cell_conc._ interval+rab_integral+RQ_integral+Arg_conc.+emission_O2+int erval_O2+interval_CO2; 0.431, 0.761, pH_cumulo+PL_cumulo+AN +AN_cumulo+interval_yield+OD+Feed_Vol+Cell_conc._interval+R Q_integral+Arg_conc.+emission_CO2+consum_O2+generate_CO2+in terval_CO2; 0.483, 0.761, pH+pH_cumulo+AN_cumulo+OD+Feed_Vo l+Cell_conc._interval+rab_integral+Arg_conc.+emission_CO2+c onsum_O2; 0.431, 0.761, pH+pH_cumulo+PL+AN+AN_cumulo+OD+RS_ cumulo+Cell_conc._interval+rab_integral+RQ_integral+Arg_con c.+emission_O2+interval_O2+interval_CO2; 0.458, 0.761, pH_c umulo+PL+PL_cumulo+AN+AN_cumulo+OD+Feed_Vol+Cell_conc._inte rval+Arg_conc.+emission_CO2+consum_O2+interval_O2; 0.431, 0. 761, tmp_cumulo+pH_cumulo+AN+AN_cumulo+interval_yield+OD+Ce ll_conc._interval+rab_integral+RQ_integral+Arg_conc.+emissi on_CO2+interval_O2+generate_CO2+interval_CO2; 0.458, 0.761, pH+pH_cumulo+PL_cumulo+AN+AN_cumulo+OD+Cell_conc._interval +Arg_conc.+emission_CO2+consum_O2+generate_CO2+interval_CO 2; 0.431, 0.761, tmp_cumulo+pH+pH_cumulo+AN+AN_cumulo+inter val_yield+OD+Cell_conc._interval+rab_integral+RQ_integral+A rg_conc.+emission_CO2+consum_O2+interval_CO2; 0.416, 0.761, pH_cumulo+PL_cumulo+AN+AN_cumulo+interval_yield+OD+RS_cumu lo+Cell_conc._interval+rab_integral+RQ_integral+Arg_conc.+e mission_O2+emission_CO2+consum_O2+interval_CO2; 0.445, 0.76 1, pH_cumulo+PL_cumulo+AN+AN_cumulo+interval_yield+OD+Cell_ conc. _interval+Arg_conc.+emission _O2+emission_CO2+interval_ O2+generate_CO2+interval_CO2; 0.445, 0.761, pH+pH_cumulo+PL _cumulo+AN+AN_cumulo+OD+Feed_Vol+Cell_conc._interval+RQ_int egral+Arg_conc.+emission_CO2+consum_O2+interval_O2; 0.431, 0.761, pH_cumulo+PL+AN+AN_cumulo+OD+Feed_Vol+RS_cumulo+Cell _conc._interval+rab_integral+RQ_integral+Arg_conc.+emission _O2+consum_O2+generate_CO2; 0.416, 0.761, pH+pH_cumulo+AN+A N_cumulo+interval_yield+OD+RS_cumulo+Cell_conc._interval+ra b_integral+RQ_integral+Arg_conc.+emission_O2+interval_O2+ge nerate_CO2+interval_CO2; 0.416, 0.761, tmp_cumulo+pH_cumulo +PL_cumulo+AN+AN_cumulo+interval_yield+OD+Cell_conc._interv al+rab_integral+RQ_integral+Arg_conc.+emission_CO2+consum_O 2+interval_O2+interval_CO2; 0.431, 0.760, tmp_cumulo+pH+pH_ cumulo+PL_cumulo+AN+AN_cumulo+interval_yield+OD+Cell_conc._ interval+Arg_conc.+emission_CO2+consum_O2+generate_CO2+inte rval_CO2; 0.431, 0.760, pH_cumulo+PL+PL_cumulo+AN+AN_cumulo +interval_yield+OD+Feed_Vol+Cell_conc._interval+rab_integra l+RQ_integral+Arg_conc.+emission_CO2+consum_O2; 0.458, 0.76 0, pH_cumulo+AN+AN_cumulo+interval_yield+OD+Cell_conc._inte rval+rab_integral+Arg_conc.+emission_CO2+interval_O2+genera te_CO2+interval_CO2; 0.431, 0.760, pH_cumulo+PL_cumulo+AN+A N_cumulo+OD+Feed_Vol+RS_cumulo+Cell_conc._interval+rab_inte gral+RQ_integral+Arg_conc.+emission_O2+consum_O2+generate_C 02; 0.431, 0.760, pH_cumulo+PL+PL_cumulo+AN_cumulo+interval _yield+OD+Feed_Vol+Cell_conc._interval+rab_integral+RQ_inte gral+Arg_conc.+emission_O2+consum_O2+interval_O2; 0.416, 0. 760, pH+pH_cumulo+PL_cumulo+AN+AN_cumulo+OD+Feed_Vol+RS_cum ulo+Cell_conc._interval+rab_integral+RQ_integral+Arg_conc.+ emission_O2+consum_O2+interval_CO2; 0.385, 0.760, pH+pH_cum ulo+PL+PL_cumulo+AN+AN_cumulo+interval_yield+OD+Feed_Vol+Ce ll_conc._interval+rab_integral+RQ_integral+Arg_conc.+emissi on_O2+interval_O2+generate_CO2+interval_CO2; 0.458, 0.760, tmp_cumulo+pH_cumulo+AN+AN_cumulo+OD+Feed_Vol+Cell_conc._in terval+Arg_conc.+emission _CO2+interval_O2+generate_CO2+inte rval_CO2; 0.445, 0.760, tmp+tmp_cumulo+pH_cumulo+AN+AN_cumu lo+OD+Cell_conc._interval+rab_integral+RQ_integral+Arg_conc. +emission_CO2+consum_O2+interval_CO2

### [209. Linear Model that Predicts Arginine Product ion Amount in Interval 9]

0.238, 0.604, tmp_cumulo+pH+PL_cumulo+RS_cumulo+Cell_conc._ interval+rab_integral+Arg_conc.+emission_CO2+interval_O2; 0. 233, 0.601, tmp+PL_cumulo+Feed_Vol+RS_cumulo+Cell_conc._int erval+ 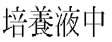 A 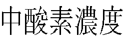 +interval_O2+generate_CO2 ; 0.233, 0. 600, tmp_cumulo+PL_cumulo+Feed_Vol+RS_cumulorg 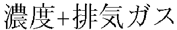 + Cell_conc._interval+RQ_integral+Arg_conc.+emission_CO2+inte rval_O2; 0.215, 0.600, tmp_cumulo+pH+PL_cumulo+Feed_Vol+RS_ cumulo+Cell_conc._interval+RQ_integral+Arg_conc.+emission_C O2+interval_O2; 0.232, 0.600, tmp+PL_cumulo+Feed_Vol+RS_cum ulo+Cell_conc._interval+Arg_conc.+emission_O2+consum_O2+gen erate_CO2; 0.231, 0.600, tmp_cumulo+pH_cumulo+PL_cumulo+RS_ cumulo+Cell_conc._interval+rab_integral+Arg_conc.+emission_ CO2+interval_O2; 0.212, 0.598, tmp+PL_cumulo+AN_cumulo+Feed _Vol+RS_cumulo+Cell_conc._interval+Arg_conc.+emission_O2+in terval_O2+generate_CO2; 0.212, 0.598, tmp_cumulo+pH+PL_cumu lo+AN_cumulo+RS_cumulo+Cell_conc._interval+rab_integral+Arg _conc.+emission_CO2+interval_O2; 0.210, 0.597, tmp_cumulo+p H+PL_cumulo+Feed_Vol+RS_cumulo+Cell_conc._interval+Arg_conc. +emission_O2+consum_O2+interval_O2; 0.192, 0.597, tmp_cumul o+pH+PL_cumulo+AN_cumulo+Feed_Vol+RS_cumulo+Cell_conc._inte rval+Arg_conc.+emission_O2+consum_O2+interval_O2; 0.227, 0. 597, tmp_cumulo+PL_cumulo+Feed_Vol+RS_cumulo+Cell_conc._int erval+Arg_conc.+emission_O2+interval_O2+generate_CO2; 0.210, 0.597, tmp_cumulo+pH+PL_cumulo+Feed_Vol+RS_cumulo+Cell_con c._interval+rab_integral+Arg_conc.+emission_CO2+interval_O 2; 0.207, 0.595, tmp_cumulo+pH_cumulo+PL_cumulo+Feed_Vol+RS _cumulo+Cell _conc. _interval+RQ _integral+Arg_conc.+emission_ CO2+interval_O2; 0.207, 0.595, tmp_cumulo+pH+PL_cumulo+RS+R S_cumulo+Cell_conc._interval+rab_integral+Arg_conc.+emissio n_CO2+interval_O2; 0.207, 0.595, tmp_cumulo+pH_cumulo+PL_cu mulo+Feed_Vol+RS_cumulo+Cell_conc._interval+rab_integral+Ar g_conc.+emission_CO2+interval_O2; 0.207, 0.595, tmp_cumulo+ pH_cumulo+PL_cumulo+Feed_Vol+RS_cumulo+Cell_conc._interval+ Arg_conc.+emission_O2+consum_O2+interval_O2; 0.206, 0.594, tmp_cumulo+pH+PL_cumulo+Feed_Vol+RS_cumulo+Cell_conc.-inter val+Arg_conc.+emission_O2+interval_O2+generate_CO2; 0.188, 0.594, tmp_cumulo+pH+PL_cumulo+AN_cumulo+Feed_Vol+RS_cumulo +Cell_conc._interval+rab_integral+Arg_conc.+emission_CO2+in terval_O2; 0.221, 0.593, tmp_cumulo+pH+PL_cumulo+Feed_Vol+R S_cumulo+Cell_conc._interval+Arg_conc.+emission_O2+interval _O2; 0.185, 0.592, tmp_cumulo+pH+PL_cumulo+AN_cumulo+Feed_V ol+RS_cumulo+Cell_conc._interval+RQ_integral+Arg_conc.+emis sion_CO2+interval_O2; 0.185, 0.592, tmp_cumulo+pH+PL_cumulo +AN_cumulo+RS+RS_cumulo+Cell_conc._interval+rab_integral+Ar g_conc.+emission_CO2+interval_O2; 0.203, 0.592, tmp_cumulo+ PL_cumulo+Feed_Vol+RS_cumulo+Cell_conc._interval+RQ_integra l+Arg_conc.+emission_CO2+interval_O2+generate_CO2; 0.202, 0. 592, tmp_cumulo+PL_cumulo+AN_cumulo+Feed_Vol+RS_cumulo+Cell _conc._interval+RQ_integral+Arg_conc.+emission_CO2+interval _O2; 0.202, 0.591, tmp+PL_cumulo+Feed_Vol+RS_cumulo+Cell_co nc._interval+Arg_conc.+emission_O2+emission_CO2+interval_O2 +generate_CO2; 0.202, 0.591, tmp+PL_cumulo+AN_cumulo+Feed_V ol+RS_cumulo+Cell_conc._interval+Arg_conc.+emission_O2+cons um_O2+generate_CO2; 0.220, 0.591, tmp_cumulo+pH+PL_cumulo+R S_cumulo+Cell _conc. _interval+RQ _integral+Arg_conc.+emission _CO2+interval_O2; 0.219, 0.591, tmp_cumulo+PL_cumulo+Feed_V ol+RS_cumulo+Cell_conc._interval+Arg_conc.+emission_O2+cons um_O2+generate_CO2; 0.201, 0.591, tmp_cumulo+pH_cumulo+PL_c umulo+Feed_Vol+RS_cumulo+Cell_conc._interval+Arg_conc.+emis sion_O2+interval_O2+generate_CO2; 0.201, 0.591, tmp_cumulo+ pH+PL_cumulo+RS_cumulo+Cell_conc._interval+rab_integral+Arg _conc.+emission_CO2+interval_O2+generate_CO2; 0.201, 0.591, tmp+pH+PL_cumulo+Feed_Vol+RS_cumulo+Cell_conc._interval+Ar g_conc.+emission_O2+interval_O2+generate_CO2; 0.200, 0.590, tmp+PL_cumulo+Feed_Vol+RS_cumulo+Cell_conc._interval+Arg_c onc.+emission_O2+emission_CO2+consum _O2+generate_CO2; 0.182, 0.590, tmp_cumulo+pH_cumulo+PL_cumulo+AN_cumulo+Feed_Vol+R S_cumulo+Cell_conc._interval+Arg_conc.+emission_O2+consum_O 2+interval_O2; 0.200, 0.590, tmp_cumulo+pH_cumulo+PL_cumulo +AN_cumulo+RS_cumulo+Cell_conc._interval+rab_integral+Arg_c onc.+emission_CO2+interval_O2; 0.199, 0.589, tmp_cumulo+PL+ PL_cumulo+Feed_Vol+RS_cumulo+Cell_conc._interval+RQ_integra l+Arg_conc.+emission_CO2+interval_O2; 0.180, 0.589, tmp+pH+ PL_cumulo+AN _cumulo+Feed _Vol+RS_cumulo+Cell _conc. _interval+ Arg_conc.+emission_O2+interval_O2+generate_CO2; 0.234, 0.58 9, tmp_cumulo+PL_cumulo+RS_cumulo+Cell_conc._interval+rab_i ntegral+Arg_conc.+emission_CO2+interval_O2; 0.199, 0.589, t mp_cumulo+PL_cumulo+AN_cumulo+Feed_Vol+RS_cumulo+Cell_conc. _interval+Arg_conc.+emission_O2+interval_O2+generate_CO2; 0. 198, 0.589, tmp+pH_cumulo+PL_cumulo+Feed_Vol+RS_cumulo+Cell _conc._interval+Arg_conc.+emission_O2+interval_O2+generate_ CO2; 0.180, 0.589, tmp_cumulo+pH+PL_cumulo+AN_cumulo+Feed_V ol+RS_cumulo+Cell_conc._interval+Arg_conc.+emission_O2+inte rval_O2+generate_CO2; 0.216, 0.589, tmp_cumulo+PL_cumulo+Fe ed_Vol+RS_cumulo+Cell_conc._interval+Arg_conc.+emission_O2+ consum_O2+interval_O2; 0.179, 0.589, tmp_cumulo+pH_cumulo+P L_cumulo+AN_cumulo+Feed_Vol+RS_cumulo+Cell_conc._interval+r ab_integral+Arg_conc.+emission_CO2+interval_O2; 0.179, 0.58 9, tmp+PL+PL_cumulo+AN_cumulo+Feed_Vol+RS_cumulo+Cell_conc. interval+Arg_conc.+emission_O2+interval_O2+generate_CO2; 0. 179, 0.588, tmp+pH+PL_cumulo+AN_cumulo+Feed_Vol+RS_cumulo+C ell_conc._interval+Arg_conc.+emission_O2+consum_O2+interval _O2; 0.179, 0.588, tmp+PL_cumulo+AN_cumulo+Feed_Vol+RS_cumu lo+Cell_conc._interval+Arg_conc.+emission_O2+emission_CO2+i nterval_O2+generate_CO2; 0.197, 0.588, tmp_cumulo+pH_cumulo +PL_cumulo+RS_cumulo+Cell_conc._interval+rab_integral+Arg_c onc.+emission_CO2+interval_O2+generate_CO2; 0.214, 0.587, t mp_cumulo+PL_cumulo+RS+RS_cumulo+Cell_conc._interval+rab_in tegral+Arg_conc.+emission_CO2+interval_O2; 0.196, 0.587, tm p_cumulo+pH_cumulo+PL_cumulo+RS+RS_cumulo+Cell_conc._interv al+rab_integral+Arg_conc.+emission_CO2+interval_O2; 0.196, 0.587, tmp_cumulo+pH+PL_cumulo+RS_cumulo+Cell_conc._interva l+rab_integral+Arg_conc.+emission_CO2+consum_O2+interval_O 2; 0.213, 0.587, tmp+PL_cumulo+RS+Feed_Vol+Cell_conc._inter val+Arg_conc.+emission_O2+consum_O2+generate_CO2; 0.195, 0. 587, tmp_cumulo+PL_cumulo+Feed_Vol+RS_cumulo+Cell_conc._int erval+RQ_integral+Arg_conc.+emission_CO2+consum_O2+interval _O2; 0.213, 0.587, tmp+PL_cumulo+Feed_Vol+RS_cumulo+Cell_co nc._interval+RQ_integral+Arg_conc.+emission_CO2+interval_O 2; 0.195, 0.587, tmp_cumulo+pH+PL+PL_cumulo+RS_cumulo+Cell_ conc._interval+rab_integral+Arg_conc.+emission_CO2+interval _O2; 0.176, 0.586, tmp_cumulo+PL_cumulo+AN_cumulo+Feed_Vol+ RS_cumulo+Cell_conc._interval+RQ_integral+Arg_conc.+emissio n_CO2+interval_O2+generate_CO2; 0.175, 0.586, tmp+pH_cumulo +PL_cumulo+AN_cumulo+Feed_Vol+RS_cumulo+Cell _conc. _interval +Arg_conc.+emission_O2+interval_O2+generate_CO2; 0.194, 0.5 86, tmp_cumulo+PL_cumulo+Feed_Vol+RS_cumulo+Cell_conc._inte rval+Arg_conc.+emission_O2+emission_CO2+interval_O2+generat e_CO2; 0.229, 0.586, tmp_cumulo+PL_cumulo+RS_cumulo+Cell_co nc._interval+RQ_integral+Arg_conc.+emission_CO2+interval_O 2; 0.194, 0.586, tmp_cumulo+PL_cumulo+Feed_Vol+RS_cumulo+Ce ll_conc._interval+RQ_integral+Arg_conc.+emission_O2+emissio n_CO2+interval_O2; 0.194, 0.586, tmp+PL_cumulo+AN_cumulo+Fe ed_Vol+RS_cumulo+Cell_conc._interval+Arg_conc.+emission_O2+ consum _O2+interval_02; 0.175, 0.586, tmp_cumulo+pH_cumulo+P L_cumulo+AN_cumulo+Feed_Vol+RS_cumulo+Cell_conc._interval+R Q_integral+Arg_conc.+emission_CO2+interval_O2; 0.193, 0.586, tmp+PL+PL_cumulo+Feed_Vol+RS_cumulo+Cell_conc._interval+Ar g_conc.+emission_O2+interval_O2+generate_CO2; 0.193, 0.585, tmp_cumulo+pH+pH_cumulo+PL_cumulo+RS_cumulo+Cell_conc._int erval+rab_integral+Arg_conc.+emission_CO2+interval_O2; 0.19 3, 0.585, tmp_cumulo+pH+PL_cumulo+AN+RS_cumulo+Cell_conc._i nterval+rab_integral+Arg_conc.+emission_CO2+interval_O2; 0. 193, 0.585, tmp+PL_cumulo+Feed_Vol+RS_cumulo+Cell_conc._int erval+rab_integral+Arg_conc.+emission_O2+interval_O2+genera te_CO2; 0.211, 0.585, tmp+PL_cumulo+Feed_Vol+RS_cumulo+Cell _conc._interval+Arg_conc.+emission_O2+consum_O2+interval_O 2; 0.174, 0.585, tmp_cumulo+pH+PL_cumulo+Feed_Vol+RS_cumulo +Cell_conc._interval+Arg_conc.+emission_O2+emission_CO2+con sum_O2+interval_O2; 0.211, 0.585, tmp_cumulo+PL_cumulo+Feed _Vol+RS_cumulo+Cell_conc._interval+rab_integral+Arg_conc.+e mission_CO2+interval_O2; 0.192, 0.585, tmp_cumulo+pH+PL_cum ulo+Feed_Vol+RS_cumulo+Cell_conc._interval+rab_integral+Arg conc.+emission_O2+interval_O2; 0.154, 0.585, tmp_cumulo+pH +PL_cumulo+AN_cumulo+Feed_Vol+RS_cumulo+Cell_conc._interval +Arg_conc.+emission_O2+emission_CO2+consum_O2+interval_O2; 0.173, 0.584, tmp+pH_cumulo+PL_cumulo+AN_cumulo+Feed_Vol+RS _cumulo+Cell_conc._interval+Arg_conc.+emission_O2+consum_O2 +interval_O2; 0.173, 0.584, tmp_cumulo+pH+PL+PL_cumulo+AN_c umulo+RS_cumulo+Cell_conc._interval+rab_integral+Arg_conc.+ emission_CO2+interval_O2; 0.192, 0.584, tmp_cumulo+PL_cumul o+interval_yield+Feed_Vol+RS_cumulo+Cell_conc._interval+Arg _conc.+emission_O2+interval_O2+generate_CO2; 0.191, 0.584, tmp+PL+PL_cumulo+Feed_Vol+RS_cumulo+Cell_conc._interval+Arg _conc.+emission_O2+consum_O2+generate_CO2; 0.191, 0.584, tm p+pH+PL_cumulo+Feed_Vol+RS_cumulo+Cell_conc._interval+Arg_c onc.+emission_O2+consum_O2+interval_O2; 0.172, 0.584, tmp_c umulo+pH+PL_cumulo+AN_cumulo+RS_cumulo+Cell_conc._interval+ rab_integral+Arg_conc.+emission_CO2+interval_O2+generate_CO 2; 0.191, 0.584, tmp_cumulo+pH+PL_cumulo+RS_cumulo+Cell_con c._cumulo+Cell_conc._interval+rab_integral+Arg_conc.+emissi on_CO2+interval_O2; 0.191, 0.584, tmp_cumulo+PL_cumulo+AN_c umulo+Feed_Vol+RS_cumulo+Cell_conc._interval+Arg_conc.+emis sion_O2+consum_O2+interval_O2; 0.226, 0.584, tmp_cumulo+pH+ PL_cumulo+RS_cumulo+Cell_conc._interval+Arg_conc.+emission_ O2+interval_O2; 0.209, 0.584, tmp_cumulo+pH_cumulo+PL_cumul o+Feed_Vol+RS_cumulo+Cell_conc._interval+Arg_conc.+emission _O2+interval_O2; 0.172, 0.584, tmp_cumulo+PL_cumulo+AN_cumu lo+Feed_Vol+RS_cumulo+Cell_conc._interval+RQ_integral+Arg_c onc.+emission_CO2+consum_O2+interval_O2; 0.171, 0.583, tmp+ PL_cumulo+AN _cumulo+Feed _Vol+RS_cumulo+Cell _conc. _interval+ rab_integral+Arg_conc.+emission_O2+interval_O2+generate_CO 2; 0.190, 0.583, tmp+PL_cumulo+Feed_Vol+RS_cumulo+Cell_conc. _interval+RQ_integral+Arg_conc.+emission_O2+interval_O2+gen erate_CO2; 0.190, 0.583, tmp+pH+PL_cumulo+Feed_Vol+RS_cumul o+Cell_conc._interval+Arg_conc.+emission_O2+consum_O2+gener ate_CO2; 0.171, 0.583, tmp_cumulo+PL+PL_cumulo+AN_cumulo+Fe ed_Vol+RS_cumulo+Cell_conc._interval+RQ_integral+Arg_conc.+ emission_CO2+interval_O2; 0.151, 0.583, tmp_cumulo+pH+PL_cu mulo+AN_cumulo+RS+Feed_Vol+RS_cumulo+Cell_conc._interval+Ar g_conc.+emission_O2+consum_O2+interval_O2; 0.190, 0.583, tm p_cumulo+pH_cumulo+PL_cumulo+Feed_Vol+RS_cumulo+Cell_conc._ interval+rab_integral+Arg_conc.+emission_O2+interval_O2; 0. 190, 0.583, tmp_cumulo+PL_cumulo+RS+Feed_Vol+RS_cumulo+Cell _conc._interval+Arg_conc.+emission_O2+interval_O2+generate_ CO2; 0.208, 0.583, tmp+pH+PL_cumulo+RS_cumulo+Cell_conc._in terval+rab_integral+Arg_conc.+emission_CO2+interval_O2; 0.1 71, 0.583, tmp_cumulo+pH+PL+PL_cumulo+Feed_Vol+RS_cumulo+Ce ll_conc._interval+RQ_integral+Arg_conc.+emission_CO2+interv al_O2; 0.190, 0.583, tmp+PL_cumulo+RS+Feed_Vol+RS_cumulo+Ce ll_conc._interval+Arg_conc.+emission_O2+interval_O2+generat e_CO2; 0.171, 0.583, tmp_cumulo+pH+PL_cumulo+RS+Feed_Vol+RS _cumulo+Cell_conc._interval+Arg_conc.+emission_O2+consum_O2 +interval_O2; 0.190, 0.583, tmp+pH_cumulo+PL_cumulo+Feed_Vo l+RS_cumulo+Cell_conc._interval+Arg_conc.+emission_O2+consu m_O2+interval_O2; 0.189, 0.583, tmp_cumulo+PL_cumulo+interv al_yield+Feed_Vol+RS_cumulo+Cell_conc._interval+RQ_integral +Arg_conc.+emission_CO2+interval_O2; 0.170, 0.583, tmp_cumu lo+pH+PL_cumulo+RS+Feed_Vol+RS_cumulo+Cell_conc._interval+r ab_integral+Arg_conc.+emission_CO2+interval_O2; 0.189, 0.58 2, tmp_cumulo+pH_cumulo+PL_cumulo+RS_cumulo+Cell_conc._inte rval+rab_integral+Arg_conc.+emission_CO2+consum_O2+interval _O2; 0.170, 0.582, tmp_cumulo+pH+PL_cumulo+Feed_Vol+RS_cumu lo+Cell_conc._interval+Arg_conc.+emission_O2+emission-CO2+i nterval_O2+generate_CO2; 0.170, 0.582, tmp_cumulo+pH+PL_cum ulo+interval_yield+Feed_Vol+RS_cumulo+Cell_conc._interval+R Q_integral+Arg_conc.+emission_CO2+interval_O2; 0.170, 0.582, tmp+PL_cumulo+AN_cumulo+Feed_Vol+RS_cumulo+Cell_conc._inte rval+RQ_integral+Arg_conc.+emission_O2+interval_O2+generate _CO2; 0.170, 0.582, tmp_cumulo+pH_cumulo+PL_cumulo+Feed_Vol +RS_cumulo+Cell_conc._interval+Arg_conc.+emission_O2+emissi on_CO2+consum_O2+interval_O2; 0.188, 0.582, tmp+PL_cumulo+F eed_Vol+RS_cumulo+Cell_conc._interval+rab_integral+Arg_conc. +emission_O2+consum_O2+generate_CO2; 0.188, 0.582, tmp_cumu lo+PL_cumulo+AN_cumulo+RS+RS_cumulo+Cell_conc._interval+rab _integral+Arg_conc.+emission_CO2+interval_O2; 0.169, 0.582, tmp_cumulo+pH_cumulo+PL_cumulo+AN_cumulo+Feed_Vol+RS_cumul o+Cell_conc._interval+Arg_conc.+emission_O2+interval_O2+gen erate_CO2; 0.188, 0.582, tmp_cumulo+pH+PL_cumulo+AN_cumulo+ Feed_Vol+RS_cumulo+Cell_conc._interval+Arg_conc.+emission_O 2+interval_O2; 0.169, 0.582, tmp+PL_cumulo+AN_cumulo+RS+Fee d_Vol+RS_cumulo+Cell_conc._interval+Arg_conc.+emission_O2+i nterval_O2+generate_CO2; 0.149, 0.582, tmp_cumulo+pH+PL_cum ulo+AN_cumulo+RS+Feed_Vol+RS_cumulo+Cell _conc. _interval+rab _integral+Arg_conc.+emission_CO2+interval_O2; 0.188, 0.582, tmp+PL_cumulo+Feed_Vol+RS_cumulo+Cell_conc._interval+RQ_in tegral+Arg_conc.+emission_CO2+interval_O2+generate_CO2; 0.1 69, 0.582, tmp+PL_cumulo+AN_cumulo+Feed_Vol+RS_cumulo+Cell_ conc._interval+Arg_conc.+emission_O2+emission_CO2+consum_O2 +generate_CO2; 0.187, 0.581, tmp_cumulo+PL_cumulo+Feed_Vol+ RS_cumulo+Cell_conc._interval+RQ_integral+Arg_conc.+emissio n_O2+interval_O2+generate_CO2; 0.187, 0.581, tmp+PL_cumulo+ RS+Feed_Vol+RS_cumulo+Cell_conc._interval+Arg_conc.+emissio n_O2+consum_O2+generate_CO2; 0.187, 0.581, tmp+PL_cumulo+in terval_yield+Feed_Vol+RS_cumulo+Cell_conc._interval+Arg_con c.+emission_O2+interval_O2+generate_CO2; 0.187, 0.581, tmp+ PL+PL_cumulo+Feed _Vol+RS_cumulo+Cell _conc. _interval+RQ_inte gral+Arg_conc.+emission_CO2+interval_O2; 0.168, 0.581, tmp_ cumulo+pH+pH_cumulo+PL_cumulo+Feed_Vol+RS_cumulo+Cell_conc. _interval+RQ_integral+Arg_conc.+emission_CO2+interval_O2; 0. 187, 0.581, tmp_cumulo+pH_cumulo+PL+PL_cumulo+RS_cumulo+Cel l_conc._interval+rab_integral+Arg_conc.+emission_CO2+interv al_O2; 0.205, 0.581, tmp+pH_cumulo+PL_cumulo+RS_cumulo+Cell _conc._interval+rab_integral+Arg_conc.+emission_CO2+interva l_O2; 0.187, 0.581, tmp+pH_cumulo+PL_cumulo+Feed_Vol+RS_cum ulo+Cell_conc._interval+Arg_conc.+emission_O2+consum_O2+gen erate_CO2; 0.187, 0.581, tmp_cumulo+pH+PL_cumulo+interval_y ield+RS_cumulo+Cell_conc._interval+rab_integral+Arg_conc.+e mission_CO2+interval_O2; 0.222, 0.581, tmp+PL_cumulo+RS_cum ulo+Cell_conc._interval+rab_integral+Arg_conc.+emission_CO2 +interval_O2; 0.205, 0.581, tmp_cumulo+pH_cumulo+PL_cumulo+ RS_cumulo+Cell_conc._interval+RQ_integral+Arg_conc.+emissio n_CO2+interval_O2; 0.205, 0.581, tmp+PL_cumulo+AN_cumulo+Fe ed_Vol+Cell_conc._interval+Arg_conc.+emission_O2+consum_O2+ generate_CO2; 0.186, 0.581, tmp_cumulo+PL_cumulo+Feed_Vol+R S_cumulo+Cell_conc._interval+rab_integral+Arg_conc.+emissio n_O2+interval_O2+generate_CO2; 0.186, 0.581, tmp+PL_cumulo+ interval_yield+Feed_Vol+RS_cumulo+Cell_conc._interval+Arg_c onc.+emission_O2+consum_O2+generate_CO2; 0.167, 0.580, tmp_ cumulo+pH+PL_cumulo+AN_cumulo+RS_cumulo+Cell_conc._interval +rab_integral+Arg_conc.+emission_CO2+consum_O2+interval_O2; 0.167, 0.580, tmp_cumulo+pH+PL_cumulo+Feed_Vol+RS_cumulo+C ell_conc._interval+RQ_integral+Arg_conc.+emission_CO2+consu m_O2+interval_O2; 0.186, 0.580, tmp_cumulo+PL_cumulo+Feed_V ol+RS_cumulo+Cell_conc._interval+Arg_conc.+emission_O2+emis sion_CO2+consum_O2+generate_CO2; 0.186, 0.580, tmp_cumulo+p H+PL_cumulo+Feed_Vol+RS_cumulo+Cell_conc._interval+RQ_integ ral+Arg_conc.+emission_O2+interval_O2; 0.221, 0.580, tmp+PL _cumulo+Feed_Vol+Cell_conc._interval+Arg_conc.+emission_O2+ consum_O2+generate_CO2; 0.186, 0.580, tmp+PL_cumulo+Feed_Vo l+RS_cumulo+Cell_conc._interval+RQ_integral+Arg_conc.+emiss ion_O2+consum_O2+generate_CO2; 0.167, 0.580, tmp_cumulo+pH_ cumulo+PL_cumulo+AN_cumulo+RS+RS_cumulo+Cell_conc._interval +rab_integral+Arg_conc.+emission_CO2+interval_O2; 0.186, 0. 580, tmp_cumulo+pH_cumulo+PL_cumulo+interval_yield+RS_cumul o+Cell_conc._interval+rab_integral+Arg_conc.+emission_CO2+i nterval_O2; 0.186, 0.580, tmp_cumulo+PL_cumulo+RS+Feed_Vol+ RS_cumulo+Cell_conc._interval+RQ_integral+Arg_conc.+emissio n_CO2+interval_O2; 0.147, 0.580, tmp_cumulo+pH+PL+PL_cumulo +AN_cumulo+Feed_Vol+RS_cumulo+Cell_conc._interval+Arg_conc. +emission_O2+consum_O2+interval_O2; 0.186, 0.580, tmp+PL_cu mulo+AN_cumulo+RS+Feed_Vol+Cell_conc._interval+Arg_conc.+em ission_O2+consum_O2+generate_CO2; 0.167, 0.580, tmp_cumulo+ pH+PL_cumulo+Feed_Vol+RS_cumulo+Cell_conc._interval+rab_int egral+Arg_conc.+emission_CO2+interval_O2+generate_CO2; 0.16 6, 0.580, tmp_cumulo+pH+PL_cumulo+RS+Feed_Vol+RS_cumulo+Cel l_conc._interval+RQ_integral+Arg_conc.+emission_CO2+interva l_O2; 0.146, 0.580, tmp_cumulo+pH+PL+PL_cumulo+AN_cumulo+Fe ed_Vol+RS_cumulo+Cell_conc._interval+rab_integral+Arg_conc. +emission_CO2+interval_O2; 0.185, 0.580, tmp+PL_cumulo+AN_c umulo+Feed_Vol+RS_cumulo+Cell_conc._interval+RQ_integral+Ar g_conc.+emission_CO2+interval_O2; 0.185, 0.580, tmp_cumulo+ PL_cumulo+Feed_Vol+RS_cumulo+Cell_conc._interval+RQ_integra l+Arg_conc.+emission_CO2+consum_O2+generate_CO2; 0.185, 0.5 80, tmp+PL_cumulo+Feed_Vol+RS_cumulo+Cell_conc._interval+Ar g_conc.+emission_O2+consum_O2+interval_O2+generate_CO2; 0.1 66, 0.580, tmp_cumulo+pH+PL_cumulo+RS+Feed_Vol+RS_cumulo+Ce ll_conc._interval+Arg_conc.+emission_O2+interval_O2+generat e_CO2; 0.203, 0.579, tmp_cumulo+pH+PL_cumulo+RS_cumulo+Cell _conc._interval+rab_integral+Arg_conc.+emission_O2+interval _O2; 0.185, 0.579, tmp+tmp_cumulo+pH+PL_cumulo+RS_cumulo+Ce ll_conc._interval+rab_integral+Arg_conc.+emission_CO2+inter val_O2; 0.203, 0.579, tmp_cumulo+PL_cumulo+RS_cumulo+Cell_c onc._interval+rab_integral+Arg_conc.+emission_CO2+interval_ O2+generate_CO2; 0.166, 0.579, tmp_cumulo+pH_cumulo+PL_cumu lo+interval_yield+Feed_Vol+RS_cumulo+Cell_conc._interval+Ar g_conc.+emission_O2+consum_O2+interval_O2; 0.146, 0.579, tm p_cumulo+pH+PL_cumulo+AN_cumulo+Feed_Vol+RS_cumulo+Cell_con c._interval+RQ_integral+Arg_conc.+emission_CO2+consum_O2+in terval_O2; 0.203, 0.579, tmp_cumulo+PL_cumulo+Feed_Vol+RS_c umulo+Cell_conc._interval+RQ_integral+Arg_conc.+emission_O2 +interval_O2; 0.166, 0.579, tmp+PL+PL_cumulo+AN_cumulo+Feed _Vol+RS_cumulo+Cell_conc._interval+Arg_conc.+emission_O2+co nsum_O2+generate_CO2; 0.166, 0.579, tmp+PL_cumulo+AN_cumulo +interval_yield+Feed_Vol+RS_cumulo+Cell_conc._interval+Arg_ conc.+emission_O2+interval_O2+generate_CO2; 0.165, 0.579, t mp+PL_cumulo+AN_cumulo+Feed_Vol+RS_cumulo+Cell_conc._interv al+RQ_integral+Arg_conc.+emission_CO2+interval_O2+generate_ CO2; 0.165, 0.579, tmp_cumulo+PL_cumulo+AN_cumulo+Feed_Vol+ RS_cumulo+Cell_conc._interval+Arg_conc.+emission_O2+emissio n_CO2+interval_O2+generate_CO2; 0.165, 0.579, tmp_cumulo+pH +PL_cumulo+AN+Feed_Vol+RS_cumulo+Cell_conc._interval+RQ_int egral+Arg_conc.+emission_CO2+interval_O2; 0.202, 0.579, tmp _cumulo+PL_cumulo+AN_cumulo+RS_cumulo+Cell_conc._interval+r ab_integral+Arg_conc.+emission_CO2+interval_O2; 0.184, 0.57 9, tmp_cumulo+pH+PL_cumulo+RS_cumulo+Cell_conc._interval+ra b_integral+Arg_conc.+emission_O2+emission_CO2+interval_O2; 0.165, 0.579, tmp+pH+PL_cumulo+Feed_Vol+RS_cumulo+Cell_conc. interval+Arg_conc.+emission_O2+emission_CO2+interval_O2+ge nerate_CO2; 0.145, 0.579, tmp_cumulo+pH+PL_cumulo+AN_cumulo +Feed_Vol+RS_cumulo+Cell_conc._interval+rab_integral+Arg_co nc.+emission_O2+consum_O2+interval_O2; 0.184, 0.579, tmp+PL _cumulo+RS+Feed_Vol+Cell_conc._interval+Arg_conc.+emission_ O2+emission_CO2+consum_O2+generate_CO2; 0.184, 0.579, tmp_c umulo+pH+PL_cumulo+Feed_Vol+RS_cumulo+Cell_conc._interval+A rg_conc.+emission _O2+emission_CO2 + interval_O2; 0.202, 0.579, tmp_cumulo+PL_cumulo+interval_yield+Feed_Vol+RS_cumulo+Cel l_conc._interval+RQ_integral+Arg_conc.+interval_O2; 0.184, 0.579, tmp_cumulo+PL_cumulo+RS+RS_cumulo+Cell_conc._interva l+rab_integral+Arg_conc.+emission_CO2+interval_O2+generate_ CO2; 0.165, 0.579, tmp_cumulo+pH+PL_cumulo+AN_cumulo+RS_cum ulo+Cell_conc._cumulo+Cell_conc._interval+rab_integral+Arg_ conc.+emission_CO2+interval_O2; 0.165, 0.579, tmp_cumulo+PL +PL_cumulo+Feed_Vol+RS_cumulo+Cell_conc._interval+RQ_integr al+Arg_conc.+emission_CO2+interval_O2+generate_CO2; 0.165, 0.579, tmp_cumulo+pH+pH_cumulo+PL_cumulo+Feed_Vol+RS_cumulo +Cell_conc._interval+rab_integral+Arg_conc.+emission_CO2+in terval_O2; 0.184, 0.579, tmp_cumulo+PL_cumulo+AN_cumulo+Fee d_Vol+RS_cumulo+Cell_conc._interval+Arg_conc.+emission_O2+c onsum_O2+generate_CO2; 0.145, 0.579, tmp+PL+PL_cumulo+AN_cu mulo+Feed_Vol+RS_cumulo+Cell_conc._interval+Arg_conc.+emiss ion_O2+emission_CO2+interval_O2+generate_CO2; 0.219, 0.579, tmp_cumulo+PL_cumulo+Feed_Vol+RS_cumulo+Cell_conc._interva l+RQ_integral+Arg_conc.+interval_O2; 0.183, 0.579, tmp+PL_c umulo+Feed_Vol+RS_cumulo+Cell_conc._interval+RQ_integral+Ar g_conc.+emission_CO2+consum_O2+generate_CO2; 0.164, 0.578, tmp_cumulo+pH_cumulo+PL_cumulo+interval_yield+Feed_Vol+RS_c umulo+Cell_conc._interval+rab_integral+Arg_conc.+emission_C O2+interval_O2; 0.183, 0.578, tmp_cumulo+pH+PL_cumulo+Feed_ Vol+RS_cumulo+Cell_conc._interval+Arg_conc.+emission_O2+con sum_O2+generate_CO2; 0.164, 0.578, tmp_cumulo+pH_cumulo+PL_ cumulo+interval_yield+Feed_Vol+RS_cumulo+Cell_conc._interva l+RQ_integral+Arg_conc.+emission_CO2+interval_O2; 0.164, 0. 578, tmp_cumulo+pH+PL_cumulo+RS+RS_cumulo+Cell_conc._interv al+rab_integral+Arg_conc.+emission_CO2+interval_O2+generate _CO2; 0.183, 0.578, tmp_cumulo+pH+PL_cumulo+RS+RS_cumulo+Ce ll_conc._interval+RQ_integral+Arg_conc.+emission_CO2+interv al_O2; 0.164, 0.578, tmp_cumulo+pH_cumulo+PL_cumulo+RS+Feed _Vol+RS_cumulo+Cell_conc._interval+Arg_conc.+emission_O2+co nsum_O2+interval_O2; 0.144, 0.578, tmp_cumulo+pH+PL_cumulo+ AN_cumulo+Feed_Vol+RS_cumulo+Cell_conc._interval+rab_integr al+Arg_conc.+emission_CO2+consum_O2+interval_O2; 0.144, 0.5 78, tmp_cumulo+pH_cumulo+PL_cumulo+AN_cumulo+Feed_Vol+RS_cu mulo+Cell_conc._interval+Arg_conc.+emission_O2+emission_CO2 +consum_O2+interval_O2; 0.164, 0.578, tmp_cumulo+pH_cumulo+ PL_cumulo+AN_cumulo+RS_cumulo+Cell_conc._interval+rab_integ ral+Arg_conc.+emission_CO2+interval_O2+generate_CO2; 0.201, 0.578, tmp_cumulo+pH+PL_cumulo+RS_cumulo+Cell_conc._interv al+Arg_conc.+emission_O2+consum_O2+interval_O2; 0.164, 0.57 8, tmp_cumulo+pH_cumulo+PL+PL_cumulo+Feed_Vol+RS_cumulo+Cel l_conc._interval+RQ_integral+Arg_conc.+emission_CO2+interva l_O2; 0.164, 0.578, tmp_cumulo+pH_cumulo+PL_cumulo+interval _yield+Feed_Vol+RS_cumulo+Cell_conc._interval+Arg_conc.+emi ssion_O2+interval_O2+generate_CO2; 0.164, 0.578, tmp_cumulo +pH+PL_cumulo+AN_cumulo+Feed_Vol+RS_cumulo+Cell_conc._inter val+rab_integral+Arg_conc.+emission_O2+interval_O2; 0.144, 0.578, tmp_cumulo+pH+PL+PL_cumulo+AN_cumulo+Feed_Vol+RS_cum ulo+Cell_conc._interval+RQ_integral+Arg_conc.+emission_CO2+ interval_O2; 0.183, 0.578, tmp+pH+PL_cumulo+AN_cumulo+RS_cu mulo+Cell_conc._interval+rab_integral+Arg_conc.+emission_CO 2+interval_O2; 0.164, 0.578, tmp_cumulo+PL_cumulo+AN_cumulo +Feed_Vol+RS_cumulo+Cell_conc._interval+RQ_integral+Arg_con c.+emission_O2+emission_CO2+interval_O2; 0.144, 0.578, tmp_ cumulo+pH+PL_cumulo+AN_cumulo+Feed_Vol+RS_cumulo+Cell_conc. _interval+RQ_integral+Arg_conc.+emission_O2+consum_O2+inter val_O2; 0.164, 0.578, tmp+PL+PL_cumulo+AN_cumulo+Feed_Vol+R S_cumulo+Cell_conc._interval+RQ_integral+Arg_conc.+emission _CO2+interval_O2; 0.164, 0.578, tmp_cumulo+pH_cumulo+PL_cum ulo+Feed_Vol+RS_cumulo+Cell_conc._interval+Arg_conc.+emissi on_O2+emission_CO2+interval_O2+generate_CO2; 0.163, 0.578, tmp_cumulo+PL_cumulo+AN_cumulo+interval_yield+Feed_Vol+RS_c umulo+Cell_conc._interval+Arg_conc.+emission_O2+interval_O2 +generate_CO2; 0.163, 0.578, tmp_cumulo+pH_cumulo+PL_cumulo +Feed_Vol+RS_cumulo+Cell_conc._interval+rab_integral+Arg_co nc.+emission_CO2+interval_O2+generate_CO2; 0.182, 0.578, tm p_cumulo+PL_cumulo+interval_yield+Feed_Vol+RS_cumulo+Cell_c onc._interval+Arg_conc.+emission_O2+consum_O2+generate_CO2; 0.182, 0.578, tmp+tmp_cumulo+PL_cumulo+Feed_Vol+RS_cumulo+ Cell_conc._interval+Arg_conc.+emission_O2+interval_O2+gener ate_CO2; 0.163, 0.578, tmp_cumulo+PL_cumulo+interval_yield+ Feed_Vol+RS_cumulo+Cell_conc._interval+RQ_integral+Arg_conc. +emission_CO2+interval_O2+generate_CO2; 0.143, 0.578, tmp_c umulo+pH+PL_cumulo+AN_cumulo+interval_yield+Feed_Vol+RS_cum ulo+Cell_conc._interval+Arg_conc.+emission_O2+consum_O2+int erval_O2; 0.163, 0.578, tmp_cumulo+PL_cumulo+AN_cumulo+RS+F eed_Vol+RS_cumulo+Cell_conc._interval+Arg_conc.+emission_O2 +interval_O2+generate_CO2; 0.163, 0.578, tmp_cumulo+pH+PL_c umulo+interval_yield+Feed_Vol+RS_cumulo+Cell_conc._interval +Arg_conc.+emission_O2+consum_O2+interval_O2; 0.163, 0.578, tmp_cumulo+pH_cumulo+PL_cumulo+Feed_Vol+RS_cumulo+Cell_con c._interval+RQ_integral+Arg_conc.+emission_CO2+consum_O2+in terval_O2; 0.143, 0.578, tmp_cumulo+pH+PL_cumulo+AN_cumulo+ Feed_Vol+RS_cumulo+Cell_conc._interval+rab_integral+Arg_con c.+emission_CO2+interval_O2+generate_CO2; 0.143, 0.578, tmp _cumulo+pH+PL+PL_cumulo+AN_cumulo+RS+RS_cumulo+Cell_conc._i nterval+rab_integral+Arg_conc.+emission_CO2+interval_O2; 0. 163, 0.578, tmp_cumulo+pH+PL_cumulo+Feed_Vol+RS_cumulo+Cell _conc._interval+rab_integral+Arg_conc.+emission_CO2+consum-O2+interval_O2; 0.163, 0.578, tmp_cumulo+pH+pH_cumulo+PL_cu mulo+AN_cumulo+RS_cumulo+Cell_conc._interval+rab_integral+A rg_conc.+emission_CO2+interval_O2; 0.182, 0.577, tmp_cumulo +PL_cumulo+AN_cumulo+Feed_Vol+RS_cumulo+Cell_conc._interval +rab_integral+Arg_conc.+emission_CO2+interval_O2; 0.163, 0. 577, tmp+pH_cumulo+PL_cumulo+Feed_Vol+RS_cumulo+Cell_conc._ interval+Arg_conc.+emission_O2+emission_CO2+interval_O2+gen erate_CO2; 0.182, 0.577, tmp_cumulo+PL_cumulo+Feed_Vol+RS_c umulo+Cell_conc._interval+rab_integral+Arg_conc.+emission_C O2+interval_O2+generate_CO2; 0.163, 0.577, tmp+PL+PL_cumulo +AN_cumulo+Feed_Vol+RS_cumulo+Cell_conc._interval+Arg_conc. +emission_O2+consum_O2+interval_O2; 0.163, 0.577, tmp_cumul o+pH+PL_cumulo+interval_yield+Feed_Vol+RS_cumulo+Cell-conc. _interval+Arg_conc.+emission_O2+interval_O2+generate_CO2; 0. 182, 0.577, tmp+pH+PL_cumulo+Feed_Vol+RS_cumulo+Cell_conc._ interval+RQ_integral+Arg_conc.+emission_CO2+interval_O2; 0. 163, 0.577, tmp_cumulo+pH+PL+PL_cumulo+Feed_Vol+RS_cumulo+C ell_conc._interval+rab_integral+Arg_conc.+emission_CO2+inte rval_O2; 0.182, 0.577, tmp_cumulo+PL_cumulo+RS+Feed_Vol+RS_ cumulo+Cell_conc._interval+Arg_conc.+emission_O2+consum_O2+ generate_CO2; 0.163, 0.577, tmp_cumulo+pH+PL_cumulo+Feed_Vo l+RS_cumulo+Cell_conc._interval+RQ_integral+Arg_conc.+emiss ion_O2+emission_CO2+interval_O2; 0.200, 0.577, tmp+pH+PL_cu mulo+Feed_Vol+RS_cumulo+Cell_conc._interval+Arg_conc.+emiss ion_O2+interval_O2; 0.182, 0.577, tmp_cumulo+pH+PL_cumulo+A N_cumulo+RS_cumulo+Cell_conc._interval+RQ_integral+Arg_conc. +emission_CO2+interval_02; 0.162, 0.577, tmp_cumulo+pH+PL_c umulo+Feed_Vol+RS_cumulo+Cell_conc._interval+rab_integral+A rg_conc.+emission_O2+consum_O2+interval_O2; 0.199, 0.577, t mp_cumulo+PL_cumulo+RS+RS_cumulo+Cell_conc._interval+RQ_int egral+Arg_conc.+emission_CO2+interval_O2; 0.162, 0.577, tmp _cumulo+pH+pH_cumulo+PL_cumulo+Feed_Vol+RS_cumulo+Cell_conc. _interval+Arg_conc.+emission_O2+consum_O2+interval_O2; 0.16 2, 0.577, tmp_cumulo+pH+PL_cumulo+Feed_Vol+RS_cumulo+Cell_c onc._interval+RQ_integral+Arg_conc.+emission_CO2+interval_O 2+generate_CO2; 0.181, 0.577, tmp+tmp_cumulo+pH_cumulo+PL_c umulo+RS_cumulo+Cell_conc._interval+rab_integral+Arg_conc.+ emission_CO2+interval_O2; 0.181, 0.577, tmp+tmp_cumulo+PL_c umulo+Feed_Vol+RS_cumulo+Cell_conc._interval+RQ_integral+Ar g_conc.+emission_CO2+interval_O2; 0.181, 0.577, tmp_cumulo+ PL_cumulo+Feed_Vol+RS_cumulo+Cell_conc._interval+Arg_conc.+ emission _O2+emission_CO2+consum_O2+interval_02; 0.142, 0.57 7, tmp_cumulo+pH+PL_cumulo+AN_cumulo+Feed_Vol+RS_cumulo+Cel l_conc._interval+Arg_conc.+emission_O2+emission_CO2+interva l_O2+generate_CO2; 0.181, 0.577, tmp_cumulo+PL_cumulo+RS+Fe ed_Vol+RS_cumulo+Cell_conc._interval+rab_integral+Arg_conc. +emission_CO2+interval_O2; 0.142, 0.577, tmp_cumulo+pH+pH_c umulo+PL_cumulo+AN_cumulo+Feed_Vol+RS_cumulo+Cell_conc._int erval+Arg_conc.+emission_O2+consum_O2+interval_O2; 0.180, 0. 576, tmp_cumulo+pH_cumulo+PL_cumulo+RS_cumulo+Cell_conc._cu mulo+Cell_conc._interval+rab_integral+Arg_conc.+emission_CO 2+interval_O2; 0.141, 0.576, tmp+PL+PL_cumulo+AN_cumulo+Fee d_Vol+RS_cumulo+Cell_conc._interval+RQ_integral+Arg_conc.+e mission_CO2+interval_O2+generate_CO2; 0.161, 0.576, tmp+PL+ PL_cumulo+Feed_Vol+RS_cumulo+Cell_conc._interval+Arg_conc.+ emission_O2+emission_CO2+interval_O2+generate_CO2; 0.180, 0. 576, tmp_cumulo+pH_cumulo+PL_cumulo+AN+RS_cumulo+Cell_conc. _interval+rab_integral+Arg_conc.+emission_CO2+interval_O2; 0.216, 0.576, tmp_cumulo+PL_cumulo+Feed_Vol+RS_cumulo+Cell_ conc._interval+Arg_conc.+emission_O2+interval_O2; 0.141, 0. 576, tmp_cumulo+pH_cumulo+PL_cumulo+AN_cumulo+interval_yiel d+Feed_Vol+RS_cumulo+Cell_conc._interval+Arg_conc.+emission _O2+consum_O2+interval_O2; 0.198, 0.576, tmp_cumulo+PL+PL_c umulo+RS_cumulo+Cell_conc._interval+RQ_integral+Arg_conc.+e mission_CO2+interval_O2; 0.161, 0.576, tmp_cumulo+pH+PL_cum ulo+AN+AN_cumulo+RS_cumulo+Cell_conc._interval+rab_integral +Arg_conc.+emission_CO2+interval_O2; 0.161, 0.576, tmp_cumu lo+pH+PL_cumulo+AN+Feed_Vol+RS_cumulo+Cell_conc._interval+r ab_integral+Arg_conc.+emission_CO2+interval_O2; 0.161, 0.57 6, tmp_cumulo+pH+PL_cumulo+RS+RS_cumulo+Cell_conc._interval +rab_integral+Arg_conc.+emission_CO2+consum_O2+interval_O2; 0.161, 0.576, tmp_cumulo+pH+PL_cumulo+Feed_Vol+RS_cumulo+C ell_conc._interval+rab_integral+Arg_conc.+emission_O2+emiss ion_CO2+interval_O2; 0.141, 0.576, tmp_cumulo+PL+PL_cumulo+ AN_cumulo+Feed_Vol+RS_cumulo+Cell_conc._interval+RQ_integra l+Arg_conc.+emission_CO2+interval_O2+generate_CO2; 0.180, 0. 576, tmp_cumulo+PL_cumulo+Feed_Vol+RS_cumulo+Cell_conc._int erval+Arg_conc.+emission_O2+consum_O2+interval_O2+generate_ CO2; 0.161, 0.576, tmp_cumulo+pH_cumulo+PL+PL_cumulo+AN_cum ulo+RS_cumulo+Cell_conc._interval+rab_integral+Arg_conc.+em ission_CO2+interval_O2; 0.180, 0.576, tmp+PL_cumulo+Feed_Vo l+RS_cumulo+Cell_conc._interval+rab_integral+Arg_conc.+emis sion_CO2+consum_O2+generate_CO2; 0.161, 0.576, tmp_cumulo+p H+PL+PL_cumulo+RS+RS_cumulo+Cell_conc._interval+rab_integra l+Arg_conc.+emission_CO2+interval_O2; 0.141, 0.576, tmp+pH+ PL_cumulo+AN_cumulo+Feed_Vol+RS_cumulo+Cell_conc._interval+ Arg_conc.+emission_O2+emission_CO2+interval_O2+generate_CO 2; 0.179, 0.576, tmp_cumulo+PL_cumulo+Feed_Vol+RS_cumulo+Ce ll_conc._interval+RQ_integral+Arg_conc.+emission_O2+consum_ O2+interval_O2; 0.198, 0.576, tmp_cumulo+PL+PL_cumulo+RS_cu mulo+Cell_conc._interval+rab_integral+Arg_conc.+emission_CO 2+interval_O2; 0.198, 0.576, tmp+PL_cumulo+Feed_Vol+RS_cumu lo+Cell_conc._interval+rab_integral+Arg_conc.+emission_CO2+ interval_O2; 0.160, 0.576, tmp_cumulo+pH+PL_cumulo+interval _yield+Feed_Vol+RS_cumulo+Cell_conc._interval+rab_integral+ Arg_conc.+emission_CO2+interval_O2; 0.179, 0.576, tmp+pH+PL _cumulo+Feed_Vol+RS_cumulo+Cell_conc._interval+rab_integral +Arg_conc.+emission_CO2+interval_O2; 0.160, 0.576, tmp_cumu lo+pH_cumulo+PL_cumulo+RS+Feed_Vol+RS_cumulo+Cell_conc._int erval+rab_integral+Arg_conc.+emission_CO2+interval_O2; 0.16 0, 0.576, tmp_cumulo+pH_cumulo+PL_cumulo+Feed_Vol+RS_cumulo +Cell_conc._interval+rab_integral+Arg_conc.+emission_CO2+co nsum_O2+interval_O2; 0.160, 0.576, tmp+PL_cumulo+AN_cumulo+ Feed_Vol+RS_cumulo+Cell_conc._interval+Arg_conc.+emission_O 2+emission_CO2+consum_O2+interval_O2; 0.179, 0.575, tmp_cum ulo+PL+PL_cumulo+Feed_Vol+RS_cumulo+Cell_conc._interval+Arg _conc.+emission_O2+interval_O2+generate_CO2; 0.160, 0.575, tmp_cumulo+PL_cumulo+AN_cumulo+Feed_Vol+RS_cumulo+Cell_conc. _interval+RQ_integral+Arg_conc.+emission_O2+interval_O2+gen erate_CO2; 0.160, 0.575, tmp+tmp_cumulo+pH+PL_cumulo+Feed_V ol+RS_cumulo+Cell_conc._interval+RQ_integral+Arg_conc.+emis sion_CO2+interval_O2; 0.160, 0.575, tmp_cumulo+pH+PL_cumulo +Feed_Vol+RS_cumulo+Cell_conc._interval+RQ_integral+Arg_con c.+emission_O2+consum_O2+interval_O2; 0.179, 0.575, tmp_cum ulo+pH+PL_cumulo+RS+Feed_Vol+RS_cumulo+Cell_conc._interval+ Arg_conc.+emission_O2+interval_O2; 0.160, 0.575, tmp_cumulo +pH+PL+PL_cumulo+Feed_Vol+RS_cumulo+Cell_conc._interval+Arg _conc.+emission_O2+consum_O2+interval_O2; 0.179, 0.575, tmp _cumulo+pH_cumulo+PL_cumulo+Feed_Vol+RS_cumulo+Cell_conc._i nterval+RQ_integral+Arg_conc.+emission_O2+interval_O2; 0.19 7, 0.575, tmp_cumulo+pH_cumulo+PL_cumulo+RS_cumulo+Cell_con c._interval+rab_integral+Arg_conc.+emission_O2+interval_O2; 0.160, 0.575, tmp_cumulo+pH+pH_cumulo+PL_cumulo+RS+RS_cumu lo+Cell_conc._interval+rab_integral+Arg_conc.+emission_CO2+ interval_O2; 0.160, 0.575, tmp+PL_cumulo+AN_cumulo+Feed_Vol +RS_cumulo+Cell_conc._interval+Arg_conc.+emission_O2+consum _O2+interval_O2+generate_CO2; 0.197, 0.575, tmp_cumulo+PL_c umulo+Feed_Vol+RS_cumulo+Cell_conc._interval+rab_integral+A rg_conc.+emission_O2+interval_O2; 0.140, 0.575, tmp_cumulo+ pH+PL_cumulo+AN_cumulo+RS+RS_cumulo+Cell_conc._interval+rab integral+Arg_conc.+emission_CO2+interval_O2+generate_CO2; 0.139, 0.575, tmp+PL+PL_cumulo+AN_cumulo+Feed_Vol+RS_cumulo +Cell_conc._interval+RQ_integral+Arg_conc.+emission_O2+inte rval_O2+generate_CO2; 0.139, 0.575, tmp+pH+PL_cumulo+AN_cum ulo+Feed_Vol+RS_cumulo+Cell_conc._interval+Arg_conc.+emissi on_O2+emission_CO2+consum_O2+interval_O2; 0.197, 0.575, tmp +PL_cumulo+RS+RS_cumulo+Cell_conc._interval+rab_integral+Ar g_conc.+emission_CO2+interval_O2; 0.139, 0.575, tmp_cumulo+ pH+pH_cumulo+PL_cumulo+AN_cumulo+Feed_Vol+RS_cumulo+Cell_co nc._interval+rab_integral+Arg_conc.+emission_CO2+interval_O 2; 0.178, 0.575, tmp+PL_cumulo+Feed_Vol+RS_cumulo+Cell_conc. _interval+Arg_conc.+emission_O2+emission_CO2+consum_O2+inte rval_O2; 0.159, 0.575, tmp_cumulo+pH_cumulo+PL_cumulo+Feed_ Vol+RS_cumulo+Cell_conc._interval+rab_integral+Arg_conc.+em ission_O2+consum_O2+interval_O2; 0.159, 0.575, tmp_cumulo+p H+PL_cumulo+AN_cumulo+interval_yield+RS_cumulo+Cell_conc._i nterval+rab_integral+Arg_conc.+emission_CO2+interval_O2; 0. 159, 0.575, tmp_cumulo+PL+PL_cumulo+Feed_Vol+RS_cumulo+Cell _conc._interval+RQ_integral+Arg_conc.+emission_O2+emission_ CO2+interval_O2; 0.178, 0.575, tmp_cumulo+PL_cumulo+RS+Feed _Vol+RS_cumulo+Cell_conc._interval+Arg_conc.+emission_O2+co nsum_O2+interval_O2; 0.159, 0.575, tmp_cumulo+PL_cumulo+Fee d_Vol+RS_cumulo+Cell_conc._interval+RQ_integral+Arg_conc.+e mission_O2+emission_CO2+interval_O2+generate_CO2; 0.196, 0. 575, tmp+PL_cumulo+RS+Feed_Vol+Cell_conc._interval+Arg_conc. +emission_O2+interval_O2+generate_CO2; 0.139, 0.575, tmp_cu mulo+pH+PL_cumulo+AN_cumulo+RS+Feed_Vol+RS_cumulo+Cell_conc. _interval+Arg_conc.+emission_O2+interval_O2+generate_CO2; 0. 139, 0.574, tmp+PL+PL_cumulo+AN_cumulo+Feed_Vol+RS_cumulo+C ell_conc. _interval+rab _integral+Arg_conc.+emission _O2+inter val_O2+generate_CO2; 0.159, 0.574, tmp_cumulo+PL+PL_cumulo+ Feed_Vol+RS_cumulo+Cell_conc._interval+RQ_integral+Arg_conc. +emission_CO2+consum_O2+interval_O2; 0.138, 0.574, tmp_cumu lo+pH_cumulo+PL_cumulo+AN_cumulo+Feed_Vol+RS_cumulo+Cell_co nc._interval+RQ_integral+Arg_conc.+emission_CO2+consum_O2+i nterval_O2; 0.177, 0.574, tmp+PL_cumulo+AN_cumulo+RS+Feed_V ol+Cell_conc._interval+Arg_conc.+emission_O2+interval_O2+ge nerate_CO2; 0.177, 0.574, tmp+pH_cumulo+PL_cumulo+RS+Feed_V ol+Cell_conc._interval+Arg_conc.+emission_O2+interval_O2+ge nerate_CO2; 0.138, 0.574, tmp_cumulo+PL+PL_cumulo+AN_cumulo +Feed_Vol+RS_cumulo+Cell_conc._interval+RQ_integral+Arg_con c.+emission_CO2+consum_O2+interval_O2; 0.158, 0.574, tmp+PL +PL_cumulo+Feed_Vol+RS_cumulo+Cell_conc._interval+Arg_conc. +emission_O2+emission_CO2+consum _O2+generate_CO2; 0.158, 0. 574, tmp+pH+PL_cumulo+AN_cumulo+Feed_Vol+RS_cumulo+Cell_con c._interval+rab_integral+Arg_conc.+emission_CO2+interval_O 2; 0.177, 0.574, tmp_cumulo+PL_cumulo+AN+Feed_Vol+RS_cumulo +Cell_conc._interval+RQ_integral+Arg_conc.+emission_CO2+int erval_O2; 0.177, 0.574, tmp_cumulo+PL_cumulo+Feed_Vol+RS_cu mulo+Cell_conc._interval+rab_integral+RQ_integral+Arg_conc. +emission_CO2+interval_O2; 0.158, 0.574, tmp+PL_cumulo+Feed _Vol+RS_cumulo+Cell_conc._interval+rab_integral+Arg_conc.+e mission_O2+emission_CO2+interval_O2+generate_CO2; 0.138, 0. 574, tmp_cumulo+pH+PL_cumulo+AN_cumulo+RS+RS_cumulo+Cell_co nc._interval+rab_integral+Arg_conc.+emission_CO2+consum_O2+ interval_O2; 0.138, 0.574, tmp_cumulo+pH_cumulo+PL_cumulo+A N_cumulo+RS+Feed_Vol+RS_cumulo+Cell_conc._interval+Arg_conc. +emission_O2+consum_O2+interval_O2; 0.177, 0.574, tmp+pH_cu mulo+PL_cumulo+Feed_Vol+RS_cumulo+Cell_conc._interval+rab_i ntegral+Arg_conc.+emission_CO2+interval_O2; 0.213, 0.574, t mp+PL_cumulo+RS_cumulo+Cell_conc._interval+RQ_integral+Arg_ conc.+emission _CO2+interval_02; 0.158, 0.574, tmp+PL_cumulo +AN_cumulo+Feed_Vol+RS_cumulo+Cell_conc._interval+RQ_integr al+Arg_conc.+emission_O2+consum_O2+generate_CO2; 0.158, 0.5 74, tmp_cumulo+pH+PL_cumulo+RS+RS_cumulo+Cell_conc._cumulo+ Cell_conc._interval+rab_integral+Arg_conc.+emission_CO2+int erval_O2; 0.138, 0.574, tmp_cumulo+pH+PL_cumulo+AN+AN_cumul o+Feed_Vol+RS_cumulo+Cell_conc._interval+Arg_conc.+emission _O2+consum_O2+interval_O2; 0.177, 0.574, tmp+PL_cumulo+RS+F eed_Vol+Cell_conc._interval+RQ_integral+Arg_conc.+emission_ O2+consum_O2+generate_CO2; 0.138, 0.574, tmp_cumulo+pH+PL_c umulo+AN_cumulo+Feed_Vol+RS_cumulo+Cell_conc._interval+rab_ integral+Arg_conc.+emission_O2+emission_CO2+interval_O2; 0. 158, 0.574, tmp+tmp_cumulo+pH+PL_cumulo+AN_cumulo+RS_cumulo +Cell_conc._interval+rab_integral+Arg_conc.+emission_CO2+in terval_O2; 0.177, 0.574, tmp+pH_cumulo+PL_cumulo+Feed_Vol+R S_cumulo+Cell_conc._interval+RQ_integral+Arg_conc.+emission _CO2+interval_O2; 0.158, 0.574, tmp+PL_cumulo+AN_cumulo+Fee d_Vol+RS_cumulo+Cell_conc._interval+rab_integral+Arg_conc.+ emission_O2+consum_O2+generate_CO2; 0.158, 0.574, tmp_cumul o+pH_cumulo+PL_cumulo+RS+Feed_Vol+RS_cumulo+Cell_conc._inte rval+Arg_conc.+emission_O2+interval_O2+generate_CO2; 0.195, 0.574, tmp_cumulo+PL_cumulo+RS_cumulo+Cell_conc._interval+ rab_integral+Arg_conc.+emission_CO2+consum_O2+interval_O2; 0.177, 0.574, tmp_cumulo+pH_cumulo+PL_cumulo+interval_yield +Feed_Vol+RS_cumulo+Cell_conc._interval+RQ_integral+Arg_con c.+interval_O2; 0.177, 0.574, tmp+pH_cumulo+PL_cumulo+AN_cu mulo+RS_cumulo+Cell_conc._interval+rab_integral+Arg_conc.+e mission_CO2+interval_O2; 0.158, 0.574, tmp_cumulo+pH+PL_cum ulo+Feed_Vol+RS_cumulo+Cell_conc._interval+rab_integral+Arg _conc.+emission_O2+interval_O2+generate_CO2; 0.138, 0.574, tmp_cumulo+pH+PL_cumulo+AN_cumulo+interval_yield+Feed_Vol+R S_cumulo+Cell_conc._interval+RQ_integral+Arg_conc.+emission _CO2+interval_O2; 0.177, 0.574, tmp+pH+PL_cumulo+RS+Feed_Vo l+Cell_conc._interval+Arg_conc.+emission_O2+consum_O2+gener ate_CO2; 0.157, 0.574, tmp_cumulo+pH_cumulo+PL_cumulo+AN_cu mulo+Feed_Vol+RS_cumulo+Cell_conc._interval+rab_integral+Ar g_conc.+emission_O2+interval_O2; 0.157, 0.574, tmp_cumulo+P L_cumulo+AN_cumulo+Feed_Vol+RS_cumulo+Cell_conc._interval+r ab_integral+Arg_conc.+emission_O2+interval_O2+generate_CO2; 0.137, 0.574, tmp_cumulo+pH+PL_cumulo+AN_cumulo+interval_y ield+Feed_Vol+RS_cumulo+Cell_conc._interval+rab_integral+Ar g_conc.+emission_CO2+interval_O2; 0.176, 0.574, tmp_cumulo+ pH_cumulo+PL_cumulo+Feed_Vol+RS_cumulo+Cell_conc._interval+ Arg_conc.+emission_O2+consum_O2+generate_CO2; 0.157, 0.574, tmp_cumulo+pH+PL+PL_cumulo+RS_cumulo+Cell_conc._interval+r ab_integral+Arg_conc.+emission_CO2+interval_O2+generate_CO 2; 0.157, 0.574, tmp+PL+PL_cumulo+Feed_Vol+RS_cumulo+Cell_c onc._interval+RQ_integral+Arg_conc.+emission_CO2+interval_O 2+generate_CO2; 0.195, 0.574, tmp+PL_cumulo+Feed_Vol+Cell_c onc._interval+Arg_conc.+emission_O2+emission_CO2+consum_O2+ generate_CO2; 0.157, 0.574, tmp_cumulo+pH+pH_cumulo+PL_cumu lo+Feed_Vol+RS_cumulo+Cell_conc._interval+Arg_conc.+emissio n_O2+interval-O2+generate-CO2; 0.137, 0.573, tmp+pH_cumulo+ PL_cumulo+AN_cumulo+Feed_Vol+RS_cumulo+Cell_conc._interval+ Arg_conc.+emission_O2+emission_CO2+interval_O2+generate_CO 2; 0.157, 0.573, tmp_cumulo+pH+PL_cumulo+AN+Feed_Vol+RS_cum ulo+Cell_conc._interval+Arg_conc.+emission_O2+consum_O2+int erval_O2; 0.157, 0.573, tmp_cumulo+PL_cumulo+AN_cumulo+Feed _Vol+RS_cumulo+Cell_conc._interval+Arg_conc.+emission_O2+em ission_CO2+consum_O2+interval_O2; 0.137, 0.573, tmp+tmp_cum ulo+pH+PL_cumulo+AN_cumulo+Feed_Vol+RS_cumulo+Cell_conc._in terval+Arg_conc.+emission_O2+consum_O2+interval_O2; 0.176, 0.573, tmp_cumulo+pH+PL+PL_cumulo+RS_cumulo+Cell_conc._inte rval+RQ_integral+Arg_conc.+emission_CO2+interval_O2; 0.137, 0.573, tmp_cumulo+PL_cumulo+AN_cumulo+interval_yield+Feed Vol+RS_cumulo+Cell_conc._interval+RQ_integral+Arg_conc.+emi ssion_CO2+interval_O2+generate_CO2; 0.157, 0.573, tmp_cumul o+pH+pH_cumulo+PL_cumulo+RS_cumulo+Cell_conc._interval+rab_ integral+Arg_conc.+emission_CO2+interval_O2+generate_CO2; 0. 157, 0.573, tmp_cumulo+pH_cumulo+PL_cumulo+Feed_Vol+RS_cumu lo+Cell_conc._interval+rab_integral+Arg_conc.+emission_O2+e mission_CO2+interval_O2; 0.157, 0.573, tmp+PL_cumulo+AN_cum ulo+RS+Feed_Vol+RS_cumulo+Cell_conc._interval+Arg_conc.+emi ssion_O2+consum_O2+generate_CO2; 0.157, 0.573, tmp_cumulo+p H_cumulo+PL_cumulo+Feed_Vol+RS_cumulo+Cell_conc._interval+A rg_conc.+emission_O2+consum_O2+interval_O2+generate_CO2; 0. 157, 0.573, tmp_cumulo+pH_cumulo+PL_cumulo+AN_cumulo+RS_cum ulo+Cell_conc._interval+rab_integral+Arg_conc.+emission_CO2 +consum_O2+interval_O2; 0.157, 0.573, tmp_cumulo+pH_cumulo+ PL_cumulo+Feed_Vol+RS_cumulo+Cell_conc._interval+RQ_integra l+Arg_conc.+emission_O2+consum_O2+interval_O2; 0.157, 0.573, tmp_cumulo+PL_cumulo+AN_cumulo+Feed_Vol+RS_cumulo+Cell_con c._interval+RQ_integral+Arg_conc.+emission_O2+consum_O2+int erval_O2; 0.137, 0.573, tmp_cumulo+pH+PL_cumulo+AN_cumulo+i nterval_yield+Feed_Vol+RS_cumulo+Cell_conc._interval+Arg_co nc.+emission_O2+interval_O2+generate_CO2; 0.137, 0.573, tmp _cumulo+pH+pH_cumulo+PL_cumulo+AN_cumulo+Feed_Vol+RS_cumulo +Cell_conc._interval+RQ_integral+Arg_conc.+emission_CO2+int erval_O2; 0.157, 0.573, tmp+pH+PL_cumulo+AN_cumulo+Feed_Vol +RS_cumulo+Cell_conc._interval+Arg_conc.+emission_O2+consum _O2+generate_CO2; 0.157, 0.573, tmp+pH+PL_cumulo+AN_cumulo+ RS+Feed_Vol+Cell_conc._interval+Arg_conc.+emission_O2+inter val_O2+generate_CO2; 0.194, 0.573, tmp_cumulo+PL_cumulo+AN_ cumulo+RS_cumulo+Cell_conc._interval+RQ_integral+Arg_conc.+ emission_CO2+interval_O2; 0.157, 0.573, tmp_cumulo+pH+PL_cu mulo+Feed_Vol+RS_cumulo+Cell_conc._interval+rab_integral+RQ _integral+Arg_conc.+emission_CO2+interval_O2; 0.157, 0.573, tmp+tmp_cumulo+PL_cumulo+AN_cumulo+Feed_Vol+RS_cumulo+Cell _conc._interval+Arg_conc.+emission_O2+interval_O2+generate_ CO2; 0.156, 0.573, tmp_cumulo+PL_cumulo+AN_cumulo+interval_ yield+Feed_Vol+RS_cumulo+Cell_conc._interval+RQ_integral+Ar g_conc.+emission_CO2+interval_O2; 0.176, 0.573, tmp+pH+PL_c umulo+RS+Feed_Vol+Cell_conc._interval+Arg_conc.+emission_O2 +interval_O2+generate_CO2; 0.175, 0.573, tmp_cumulo+pH+PL_c umulo+interval_yield+Feed_Vol+RS_cumulo+Cell_conc._interval +Arg_conc.+emission_O2+interval_O2; 0.136, 0.573, tmp_cumul o+pH+PL_cumulo+AN_cumulo+RS+RS_cumulo+Cell_conc._cumulo+Cel l_conc._interval+rab_integral+Arg_conc.+emission_CO2+interv al_O2; 0.136, 0.573, tmp+pH_cumulo+PL_cumulo+AN_cumulo+Feed _Vol+RS_cumulo+Cell_conc._interval+Arg_conc.+emission_O2+em ission_CO2+consum_O2+interval_O2; 0.156, 0.573, tmp_cumulo+ pH+PL_cumulo+AN+RS+RS_cumulo+Cell_conc._interval+rab_integr al+Arg_conc.+emission_CO2+interval_O2; 0.156, 0.573, tmp_cu mulo+PL_cumulo+AN_cumulo+RS+RS_cumulo+Cell_conc._interval+r ab_integral+Arg_conc.+emission_CO2+interval_O2+generate_CO 2; 0.175, 0.573, tmp+PL_cumulo+Feed_Vol+RS_cumulo+Cell_conc. _interval+rab_integral+Arg_conc.+emission_CO2+interval_O2+g enerate_CO2; 0.211, 0.573, tmp_cumulo+pH+PL_cumulo+RS_cumul o+Cell_conc._interval+Arg_conc.+emission_CO2+interval_O2; 0. 156, 0.573, tmp_cumulo+PL_cumulo+AN_cumulo+RS+Feed_Vol+RS_c umulo+Cell_conc._interval+rab_integral+Arg_conc.+emission_C O2+interval_O2; 0.156, 0.573, tmp_cumulo+pH_cumulo+PL+PL_cu mulo+Feed_Vol+RS_cumulo+Cell_conc._interval+rab_integral+Ar g_conc.+emission_CO2+interval_O2; 0.136, 0.573, tmp_cumulo+ pH_cumulo+PL_cumulo+AN_cumulo+Feed_Vol+RS_cumulo+Cell_conc. _interval+rab_integral+Arg_conc.+emission_O2+consum_O2+inte rval_O2; 0.175, 0.573, tmp+PL+PL_cumulo+RS+Feed_Vol+Cell_co nc._interval+Arg_conc.+emission_O2+consum_O2+generate_CO2; 0.194, 0.573, tmp+PL_cumulo+AN_cumulo+RS_cumulo+Cell_conc._ interval+rab_integral+Arg_conc.+emission_CO2+interval_O2; 0. 136, 0.573, tmp_cumulo+pH_cumulo+PL_cumulo+AN_cumulo+Feed_V ol+RS_cumulo+Cell_conc._interval+rab_integral+Arg_conc.+emi ssion_CO2+consum_O2+interval_O2; 0.175, 0.573, tmp+PL_cumul o+AN_cumulo+Feed_Vol+Cell_conc._interval+Arg_conc.+emission _O2+emission_CO2+consum_O2+generate_CO2; 0.136, 0.573, tmp_ cumulo+pH+PL_cumulo+AN+AN_cumulo+Feed_Vol+RS_cumulo+Cell_co nc._interval+rab_integral+Arg_conc.+emission_CO2+interval_O 2; 0.136, 0.573, tmp_cumulo+pH_cumulo+PL_cumulo+AN_cumulo+i nterval_yield+Feed_Vol+RS_cumulo+Cell_conc._interval+rab_in tegral+Arg_conc.+emission_CO2+interval_O2; 0.136, 0.573, tm p+pH+PL+PL_cumulo+AN_cumulo+Feed_Vol+RS_cumulo+Cell_conc._i nterval+Arg_conc.+emission_O2+consum_O2+interval_O2; 0.156, 0.573, tmp+tmp_cumulo+pH+PL_cumulo+Feed_Vol+RS_cumulo+Cell _conc._interval+rab_integral+Arg_conc.+emission_CO2+interva l_O2; 0.175, 0.573, tmp_cumulo+PL+PL_cumulo+RS+RS_cumulo+Ce ll_conc._interval+rab_integral+Arg_conc.+emission_CO2+inter val_O2; 0.156, 0.573, tmp+pH+PL_cumulo+Feed_Vol+RS_cumulo+C ell_conc._interval+Arg_conc.+emission_O2+emission_CO2+consu m_O2+interval_O2; 0.156, 0.573, tmp+PL_cumulo+AN_cumulo+int erval_yield+Feed_Vol+RS_cumulo+Cell_conc._interval+Arg_conc. +emission_O2+consum_O2+generate_CO2; 0.193, 0.573, tmp_cumu lo+PL_cumulo+RS_cumulo+Cell_conc._interval+RQ_integral+Arg_ conc.+emission_CO2+interval_O2+generate_CO2; 0.136, 0.572, tmp+PL_cumulo+AN_cumulo+Feed_Vol+RS_cumulo+Cell_conc._inter val+rab_integral+Arg_conc.+emission_O2+emission_CO2+interva l_O2+generate_CO2; 0.175, 0.572, tmp_cumulo+pH+PL_cumulo+RS _cumulo+Cell_conc._interval+rab_integral+RQ_integral+Arg_co nc.+emission_CO2+interval_O2; 0.156, 0.572, tmp_cumulo+pH+P L_cumulo+Feed_Vol+RS_cumulo+Cell_conc._interval+Arg_conc.+e mission_O2+consum_O2+interval_O2+generate_CO2; 0.156, 0.572, tmp_cumulo+PL_cumulo+AN_cumulo+RS+Feed_Vol+RS_cumulo+Cell_ conc._interval+Arg_conc.+emission_O2+consum_O2+interval_O2; 0.156, 0.572, tmp+pH+PL_cumulo+RS+Feed_Vol+RS_cumulo+Cell_ conc._interval+Arg_conc.+emission_O2+interval_O2+generate_C O2; 0.175, 0.572, tmp_cumulo+PL_cumulo+RS+RS_cumulo+Cell_co nc._interval+rab_integral+Arg_conc.+emission_CO2+consum_O2+ interval_O2; 0.156, 0.572, tmp_cumulo+PL+PL_cumulo+AN_cumul o+RS+RS_cumulo+Cell_conc._interval+rab_integral+Arg_conc.+e mission_CO2+interval_O2; 0.156, 0.572, tmp+pH_cumulo+PL_cum ulo+AN_cumulo+RS+Feed_Vol+Cell_conc._interval+Arg_conc.+emi ssion_O2+interval_O2+generate_CO2; 0.156, 0.572, tmp_cumulo +PL_cumulo+RS+Feed_Vol+RS_cumulo+Cell_conc._interval+RQ_int egral+Arg_conc.+emission_CO2+interval_O2+generate_CO2; 0.15 5, 0.572, tmp+pH_cumulo+PL_cumulo+Feed_Vol+RS_cumulo+Cell_c onc._interval+Arg_conc.+emission_O2+emission_CO2+consum_O2+ interval_O2; 0.175, 0.572, tmp_cumulo+pH_cumulo+PL_cumulo+R S_cumulo+Cell_conc._interval+rab_integral+Arg_conc.+emissio n_O2+emission_CO2+interval_O2; 0.155, 0.572, tmp_cumulo+pH_ cumulo+PL_cumulo+Feed_Vol+RS_cumulo+Cell_conc._interval+RQ_ integral+Arg_conc.+emission_CO2+interval_O2+generate_CO2; 0. 135, 0.572, tmp_cumulo+pH_cumulo+PL_cumulo+AN_cumulo+Feed_V ol+RS_cumulo+Cell_conc._interval+rab_integral+Arg_conc.+emi ssion_CO2+interval_O2+generate_CO2; 0.135, 0.572, tmp_cumul o+pH+PL_cumulo+AN_cumulo+Feed_Vol+RS_cumulo+Cell_conc._inte rval+RQ_integral+Arg_conc.+emission_CO2+interval_O2+generat e_CO2; 0.174, 0.572, tmp_cumulo+PL_cumulo+Feed_Vol+RS_cumul o+Cell_conc._interval+rab_integral+Arg_conc.+emission_O2+co nsum_O2+interval-O2; 0.135, 0.572, tmp_cumulo+pH_cumulo+PL+ PL_cumulo+AN_cumulo+Feed_Vol+RS_cumulo+Cell_conc._interval+ rab_integral+Arg_conc.+emission_CO2+interval_O2; 0.155, 0.5 72, tmp_cumulo+PL_cumulo+AN_cumulo+RS+Feed_Vol+RS_cumulo+Ce ll_conc._interval+RQ_integral+Arg_conc.+emission_CO2+interv al_O2; 0.174, 0.572, tmp_cumulo+PL_cumulo+interval_yield+Fe ed_Vol+RS_cumulo+Cell_conc._interval+Arg_conc.+emission_O2+ consum_O2+interval_O2; 0.135, 0.572, tmp_cumulo+pH+pH_cumul o+PL_cumulo+AN_cumulo+RS+RS_cumulo+Cell_conc._interval+rab_ integral+Arg_conc.+emission_CO2+interval_O2; 0.155, 0.572, tmp+PL+PL_cumulo+Feed_Vol+RS_cumulo+Cell_conc._interval+RQ_ integral+Arg_conc.+emission_O2+interval_O2+generate_CO2; 0. 155, 0.572, tmp+PL_cumulo+Feed_Vol+RS_cumulo+Cell_conc._int erval+rab_integral+Arg_conc.+emission_O2+emission_CO2+consu m_O2+generate_CO2; 0.135, 0.572, tmp+pH+PL+PL_cumulo+AN_cum ulo+Feed_Vol+RS_cumulo+Cell_conc._interval+Arg_conc.+emissi on_O2+interval_O2+generate_CO2; 0.174, 0.572, tmp_cumulo+PL _cumulo+Feed_Vol+RS_cumulo+Cell_conc._interval+rab_integral +Arg_conc.+emission_O2+consum_O2+generate_CO2; 0.135, 0.572, tmp_cumulo+pH_cumulo+PL_cumulo+AN_cumulo+Feed_Vol+RS_cumul o+Cell_conc._interval+RQ_integral+Arg_conc.+emission_O2+con sum_O2+interval_O2; 0.135, 0.572, tmp_cumulo+pH+PL_cumulo+A N_cumulo+RS+Feed_Vol+RS_cumulo+Cell_conc._interval+RQ_integ ral+Arg_conc.+emission_CO2+interval_O2; 0.155, 0.572, tmp_c umulo+pH_cumulo+PL_cumulo+Feed_Vol+RS_cumulo+Cell_conc._int erval+RQ_integral+Arg_conc.+emission_O2+emission_CO2+interv al_O2; 0.174, 0.572, tmp_cumulo+pH+PL_cumulo+RS_cumulo+Cell _conc._cumulo+Cell_conc._interval+RQ_integral+Arg_conc.+emi ssion_CO2+interval_O2; 0.155, 0.572, tmp_cumulo+PL_cumulo+i nterval_yield+Feed_Vol+RS_cumulo+Cell_conc._interval+Arg_co nc.+emission_O2+emission_CO2+interval_O2+generate_CO2; 0.13 5, 0.572, tmp_cumulo+pH_cumulo+PL+PL_cumulo+AN_cumulo+Feed_ Vol+RS_cumulo+Cell_conc._interval+RQ_integral+Arg_conc.+emi ssion_CO2+interval_O2; 0.135, 0.572, tmp+PL+PL_cumulo+AN_cu mulo+RS+Feed_Vol+RS_cumulo+Cell_conc._interval+Arg_conc.+em ission_O2+interval_O2+generate_CO2; 0.155, 0.572, tmp_cumul o+PL+PL_cumulo+AN_cumulo+Feed_Vol+RS_cumulo+Cell_conc._inte rval+Arg_conc.+emission_O2+interval_O2+generate_CO2; 0.192, 0.572, tmp+PL_cumulo+RS_cumulo+Cell_conc._interval+rab_int egral+Arg_conc.+emission_CO2+interval_O2+generate_CO2; 0.15 5, 0.572, tmp+pH+PL_cumulo+Feed_Vol+RS_cumulo+Cell_conc._in terval+Arg_conc.+emission_O2+emission_CO2+consum_O2+generat e_CO2; 0.155, 0.572, tmp+tmp_cumulo+pH+PL_cumulo+Feed_Vol+R S_cumulo+Cell_conc._interval+Arg_conc.+emission_O2+consum_O 2+interval_O2; 0.155, 0.572, tmp_cumulo+PL_cumulo+AN_cumulo +Feed_Vol+RS_cumulo+Cell_conc._interval+rab_integral+Arg_co nc.+emission_CO2+interval_O2+generate_CO2; 0.154, 0.572, tm p_cumulo+pH_cumulo+PL_cumulo+RS+Feed_Vol+RS_cumulo+Cell_con c._interval+RQ_integral+Arg_conc.+emission_CO2+interval_O2; 0.154, 0.572, tmp+PL_cumulo+AN_cumulo+Feed_Vol+RS_cumulo+C ell_conc._interval+RQ_integral+Arg_conc.+emission_O2+consum _O2+interval_O2; 0.154, 0.572, tmp+tmp_cumulo+pH_cumulo+PL_ cumulo+Feed_Vol+RS_cumulo+Cell_conc._interval+rab_integral+ Arg_conc.+emission_CO2+interval_O2; 0.154, 0.572, tmp_cumul o+PL+PL_cumulo+interval_yield+Feed_Vol+RS_cumulo+Cell_conc. _interval+RQ_integral+Arg_conc.+emission_CO2+interval_O2; 0. 154, 0.572, tmp_cumulo+pH+PL_cumulo+interval_yield+RS+RS_cu mulo+Cell_conc._interval+rab_integral+Arg_conc.+emission_CO 2+interval_O2; 0.174, 0.572, tmp_cumulo+PL_cumulo+Feed_Vol+ RS_cumulo+Cell_conc._interval+RQ_integral+Arg_conc.+emissio n_O2+consum_O2+generate_CO2; 0.174, 0.571, tmp_cumulo+PL_cu mulo+Feed_Vol+RS_cumulo+Cell_conc._interval+rab_integral+Ar g_conc.+emission_CO2+consum_O2+generate_CO2; 0.154, 0.571, tmp_cumulo+pH+PL_cumulo+AN+RS_cumulo+Cell_conc._interval+ra b_integral+Arg_conc.+emission_CO2+interval_O2+generate_CO2; 0.134, 0.571, tmp+pH_cumulo+PL+PL_cumulo+AN_cumulo+Feed_Vo l+RS_cumulo+Cell_conc._interval+Arg_conc.+emission_O2+inter val_O2+generate_CO2; 0.192, 0.571, tmp_cumulo+PL_cumulo+RS_ cumulo+Cell_conc._interval+RQ_integral+Arg_conc.+emission_O 2+emission_CO2+interval_O2; 0.154, 0.571, tmp_cumulo+pH+PL+ PL_cumulo+Feed_Vol+RS_cumulo+Cell_conc._interval+Arg_conc.+ emission_O2+interval_O2+generate_CO2; 0.154, 0.571, tmp+PL_ cumulo+AN_cumulo+RS+Feed_Vol+Cell_conc._interval+Arg_conc.+ emission_O2+emission_CO2+consum_O2+generate_CO2; 0.154, 0.5 71, tmp+PL+PL_cumulo+Feed_Vol+RS_cumulo+Cell_conc._interval +rab_integral+Arg_conc.+emission_O2+interval_O2+generate_CO 2; 0.173, 0.571, tmp+tmp_cumulo+PL_cumulo+Feed_Vol+RS_cumul o+Cell_conc._interval+Arg_conc.+emission_O2+consum_O2+gener ate_CO2; 0.192, 0.571, tmp+PL+PL_cumulo+RS_cumulo+Cell_conc. _interval+rab_integral+Arg_conc.+emission_CO2+interval_O2; 0.192, 0.571, tmp_cumulo+pH+PL_cumulo+RS_cumulo+Cell_conc._ interval+Arg_conc.+emission_O2+interval_O2+generate_CO2; 0. 154, 0.571, tmp+PL_cumulo+interval_yield+Feed_Vol+RS_cumulo +Cell_conc._interval+Arg_conc.+emission_O2+emission_CO2+int erval_O2+generate_CO2; 0.154, 0.571, tmp_cumulo+pH+PL_cumul o+AN_cumulo+RS_cumulo+Cell_conc._interval+rab_integral+Arg_ conc.+emission_O2+emission_CO2+interval_O2; 0.173, 0.571, t mp+PL_cumulo+AN_cumulo+RS+RS_cumulo+Cell_conc._interval+rab _integral+Arg_conc.+emission_CO2+interval_O2; 0.209, 0.571, tmp_cumulo+pH_cumulo+PL_cumulo+RS_cumulo+Cell_conc._interv al+Arg_conc.+emission_O2+interval_O2; 0.173, 0.571, tmp+pH+ PL_cumulo+RS+RS_cumulo+Cell_conc._interval+rab_integral+Arg _conc.+emission_CO2+interval_O2; 0.134, 0.571, tmp+pH+PL_cu mulo+AN_cumulo+RS+Feed_Vol+RS_cumulo+Cell_conc._interval+Ar g_conc.+emission_O2+interval_O2+generate_CO2; 0.173, 0.571, tmp+PL_cumulo+AN+Feed_Vol+RS_cumulo+Cell_conc._interval+Ar g_conc.+emission_O2+interval_O2+generate_CO2; 0.154, 0.571, tmp+tmp_cumulo+pH+PL_cumulo+Feed_Vol+RS_cumulo+Cell_conc._ interval+Arg_conc.+emission_O2+interval_O2+generate_CO2; 0. 134, 0.571, tmp+tmp_cumulo+pH+PL_cumulo+AN_cumulo+Feed_Vol+ RS_cumulo+Cell_conc._interval+rab_integral+Arg_conc.+emissi on_CO2+interval_O2; 0.209, 0.571, tmp+PL_cumulo+Feed_Vol+RS _cumulo+Cell_conc._interval+Arg_conc.+emission_O2+interval_ O2; 0.134, 0.571, tmp+pH_cumulo+PL+PL_cumulo+AN_cumulo+Feed _Vol+RS_cumulo+Cell_conc._interval+Arg_conc.+emission_O2+co nsum_O2+interval-O2; 0.173, 0.571, tmp_cumulo+pH+pH_cumulo+ PL_cumulo+RS_cumulo+Cell_conc._interval+RQ_integral+Arg_con c.+emission_CO2+interval_O2; 0.173, 0.571, tmp_cumulo+pH+PL +PL_cumulo+Feed_Vol+RS_cumulo+Cell_conc._interval+Arg-conc. +emission_O2+interval_O2; 0.154, 0.571, tmp+PL_cumulo+RS+Fe ed_Vol+RS_cumulo+Cell_conc._interval+Arg_conc.+emission_O2+ emission_CO2+interval_O2+generate_CO2; 0.154, 0.571, tmp_cu mulo+pH+PL_cumulo+AN_cumulo+Feed_Vol+RS_cumulo+Cell_conc._i nterval+RQ_integral+Arg_conc.+emission_O2+interval_O2; 0.17 3, 0.571, tmp+PL_cumulo+AN_cumulo+Feed_Vol+RS_cumulo+Cell_c onc._interval+rabintegral+Arg_conc.+emission_CO2+interval O2; 0.191, 0.571, tmp_cumulo+pH_cumulo+PL_cumulo+Feed_Vol+R S_cumulo+Cell_conc._interval+RQ_integral+Arg_conc.+interval _O2; 0.191, 0.571, tmp+PL_cumulo+Feed_Vol+RS_cumulo+Cell_co nc._interval+RQ_integral+Arg_conc.+emission_O2+interval_O2; 0.153, 0.571, tmp_cumulo+PL_cumulo+RS+Feed_Vol+RS_cumulo+C ell_conc._interval+Arg_conc.+emission_O2+emission_CO2+inter val_O2+generate_CO2; 0.153, 0.571, tmp+PL_cumulo+interval_y ield+Feed_Vol+RS_cumulo+Cell_conc._interval+Arg_conc.+emiss ion_O2+emission_CO2+consum_O2+generate_CO2; 0.133, 0.571, t mp_cumulo+pH_cumulo+PL_cumulo+AN_cumulo+interval_yield+Feed _Vol+RS_cumulo+Cell_conc._interval+Arg_conc.+emission_O2+in terval_O2+generate_CO2; 0.153, 0.571, tmp_cumulo+PL_cumulo+ RS+Feed_Vol+RS_cumulo+Cell_conc._interval+rab_integral+Arg_ conc.+emission_CO2+interval_O2+generate_CO2; 0.133, 0.571, tmp_cumulo+pH+PL_cumulo+AN_cumulo+Feed_Vol+RS_cumulo+Cell_c one._interval+rab_integral+RQ_integral+Arg_conc.+emission_C O2+interval_O2; 0.153, 0.571, tmp+PL_cumulo+Feed_Vol+RS_cum ulo+Cell_conc._interval+RQ_integral+Arg_conc.+emission_O2+e mission_CO2+interval_O2+generate_CO2; 0.153, 0.571, tmp+PL_ cumulo+AN_cumulo+Feed_Vol+RS_cumulo+Cell_conc._interval+RQ_ integral+Arg_conc.+emission_CO2+consum_O2+generate_CO2; 0.1 53, 0.571, tmp+tmp_cumulo+PL_cumulo+Feed_Vol+RS_cumulo+Cell _conc._interval+RQ_integral+Arg_conc.+emission_CO2+interval _O2+generate_CO2; 0.153, 0.571, tmp+PL_cumulo+AN_cumulo+Fee d_Vol+RS_cumulo+Cell_conc._interval+rab_integral+Arg_conc.+ emission_O2+consum_O2+interval_O2; 0.133, 0.571, tmp_cumulo +pH_cumulo+PL_cumulo+AN_cumulo+RS+Feed_Vol+RS_cumulo+Cell_c onc._interval+rab_integral+Arg_conc.+emission_CO2+interval_ O2; 0.153, 0.571, tmp+tmp_cumulo+pH_cumulo+PL_cumulo+Feed_V ol+RS_cumulo+Cell_conc._interval+RQ_integral+Arg_conc.+emis sion_CO2+interval_O2; 0.153, 0.571, tmp+pH_cumulo+PL_cumulo +AN_cumulo+Feed_Vol+RS_cumulo+Cell_conc._interval+rab_integ ral+Arg_conc.+emission_CO2+interval_O2; 0.153, 0.571, tmp_c umulo+PL_cumulo+Feed_Vol+RS_cumulo+Cell_conc._interval+rab_ integral+RQ integral+Arg_conc.+emission_CO2+consum_O2+inter val_O2; 0.172, 0.570, tmp+PL_cumulo+AN+Feed_Vol+RS_cumulo+C ell_conc._interval+Arg_conc.+emission_O2+consum_O2+generate _CO2; 0.153, 0.570, tmp_cumulo+PL_cumulo+Feed_Vol+RS_cumulo +Cell_conc._interval+RQ_integral+Arg_conc.+emission_O2+emis sion_CO2+consum_O2+interval_O2; 0.153, 0.570, tmp+PL_cumulo +AN_cumulo+Feed_Vol+RS_cumulo+Cell_conc._interval+rab_integ ral+Arg_conc.+emission_CO2+interval_O2+generate_CO2; 0.172, 0.570, tmp+PL+PL_cumulo+Feed_Vol+RS_cumulo+Cell_conc._inte rval+Arg_conc.+emission_O2+consum_O2+interval_O2; 0.153, 0. 570, tmp_cumulo+PL_cumulo+Feed_Vol+RS_cumulo+Cell_conc._int erval+RQ_integral+Arg_conc.+emission_CO2+consum_O2+interval _O2+generate_CO2; 0.133, 0.570, tmp+pH+PL_cumulo+AN_cumulo+ RS+Feed_Vol+RS_cumulo+Cell_conc._interval+Arg_conc.+emissio n_O2+consum_O2+interval_O2; 0.153, 0.570, tmp+pH_cumulo+PL_ cumulo+AN_cumulo+Feed_Vol+RS_cumulo+Cell_conc._interval+Arg _conc.+emission_O2+consum_O2+generate_CO2; 0.172, 0.570, tm p+pH_cumulo+PL_cumulo+RS+Feed_Vol+Cell_conc._interval+Arg_c onc.+emission_O2+consum_O2+generate_CO2; 0.172, 0.570, tmp+ PL_cumulo+RS+Feed_Vol+Cell_conc._interval+rab_integral+Arg_ conc.+emission_O2+consum_O2+generate_CO2; 0.153, 0.570, tmp _cumulo+PL_cumulo+interval_yield+Feed_Vol+RS_cumulo+Cell_co nc._interval+RQ_integral+Arg_conc.+emission_CO2+consum_O2+i nterval_O2; 0.172, 0.570, tmp_cumulo+pH_cumulo+PL_cumulo+AN _cumulo+Feed_Vol+RS_cumulo+Cell_conc._interval+Arg_conc.+em ission_O2+interval_O2; 0.172, 0.570, tmp_cumulo+pH+PL_cumul o+AN+RS_cumulo+Cell_conc._interval+RQ_integral+Arg_conc.+em ission_CO2+interval_O2; 0.172, 0.570, tmp+PL_cumulo+RS+Feed _Vol+Cell_conc._interval+Arg_conc.+emission_O2+emission_CO2 +interval_O2+generate_CO2; 0.152, 0.570, tmp+pH+PL_cumulo+A N_cumulo+Feed_Vol+RS_cumulo+Cell_conc._interval+RQ_integral +Arg_conc.+emission_CO2+interval_O2; 0.152, 0.570, tmp_cumu lo+pH_cumulo+PL+PL_cumulo+RS_cumulo+Cell_conc._interval+rab _integral+Arg_conc.+emission_CO2+interval_O2+generate_CO2; 0.132, 0.570, tmp_cumulo+pH_cumulo+PL+PL_cumulo+AN_cumulo+F eed_Vol+RS_cumulo+Cell_conc._interval+Arg_conc.+emission_O2 +consum_O2+interval_O2; 0.172, 0.570, tmp_cumulo+pH+PL_cumu lo+OD+RS_cumulo+Cell_conc._interval+rab_integral+Arg_conc.+ emission_CO2+interval_O2; 0.171, 0.570, tmp_cumulo+pH+pH_cu mulo+PL_cumulo+Feed_Vol+RS_cumulo+Cell_conc._interval+Arg_c onc.+emission_O2+interval_O2; 0.152, 0.570, tmp_cumulo+pH+P L_cumulo+AN+Feed_Vol+RS_cumulo+Cell_conc._interval+Arg-conc. +emission_O2+interval_O2+generate_CO2; 0.152, 0.570, tmp_cu mulo+PL_cumulo+interval_yield+RS+Feed_Vol+RS_cumulo+Cell_co nc._interval+Arg_conc.+emission_O2+interval_O2+generate_CO 2; 0.132, 0.570, tmp_cumulo+pH+PL_cumulo+AN_cumulo+Feed_Vol +RS_cumulo+Cell_conc._interval+RQ_integral+Arg_conc.+emissi on_O2+emission_CO2+interval_O2; 0.132, 0.570, tmp_cumulo+PL _cumulo+AN_cumulo+RS+Feed_Vol+RS_cumulo+Cell_conc._interval +rab_integral+Arg_conc.+emission_CO2+interval_O2+generate_C O2; 0.152, 0.570, tmp+PL_cumulo+Feed_Vol+RS_cumulo+Cell_con c._interval+Arg_conc.+emission_O2+emission_CO2+consum_O2+in terval_O2+generate_CO2; 0.190, 0.570, tmp+pH_cumulo+PL_cumu lo+Feed_Vol+RS_cumulo+Cell_conc._interval+Arg_conc.+emissio n_O2+interval_O2; 0.132, 0.570, tmp_cumulo+pH+PL+PL_cumulo+ AN_cumulo+RS_cumulo+Cell_conc._interval+rab_integral+Arg_co nc.+emission_CO2+interval_O2+generate_CO2; 0.190, 0.570, tm p_cumulo+pH_cumulo+PL_cumulo+RS_cumulo+Cell_conc._interval+ Arg_conc.+emission_O2+consum_O2+interval_O2; 0.171, 0.570, tmp_cumulo+pH_cumulo+PL_cumulo+interval_yield+Feed_Vol+RS_c umulo+Cell_conc._interval+rab_integral+Arg_conc.+interval_O 2; 0.132, 0.570, tmp_cumulo+PL+PL_cumulo+AN_cumulo+Feed_Vol +RS_cumulo+Cell_conc._interval+RQ_integral+Arg_conc.+emissi on_O2+emission_CO2+interval_O2; 0.152, 0.570, tmp_cumulo+pH _cumulo+PL_cumulo+AN+Feed_Vol+RS_cumulo+Cell_conc._interval +rab_integral+Arg_conc.+emission_CO2+interval_O2; 0.171, 0. 570, tmp_cumulo+PL+PL_cumulo+Feed_Vol+RS_cumulo+Cell_conc._ interval+Arg_conc.+emission_O2+consum_O2+generate_CO2; 0.15 2, 0.570, tmp_cumulo+pH+PL_cumulo+AN_cumulo+RS_cumulo+Cell conc._interval+rab_integral+RQ_integral+Arg_conc.+emission_ CO2+interval_O2; 0.152, 0.570, tmp_cumulo+PL_cumulo+interva l_yield+Feed_Vol+RS_cumulo+Cell_conc._interval+RQ_integral+ Arg_conc.+emission_O2+interval_O2+generate_CO2; 0.152, 0.57 0, tmp+pH_cumulo+PL_cumulo+RS+Feed_Vol+Cell_conc._interval+ Arg_conc.+emission_O2+emission_CO2+interval_O2+generate_CO 2; 0.132, 0.570, tmp_cumulo+pH+PL+PL_cumulo+AN_cumulo+Feed_ Vol+RS_cumulo+Cell_conc._interval+Arg_conc.+emission_O2+int erval_O2+generate_CO2; 0.152, 0.570, tmp+PL_cumulo+AN_cumul o+Feed_Vol+RS_cumulo+Cell_conc._interval+RQ_integral+Arg_co nc.+emission_CO2+consum_O2+interval_O2; 0.152, 0.570, tmp_c umulo+pH_cumulo+PL_cumulo+AN_cumulo+interval_yield+RS_cumul o+Cell_conc._interval+rab_integral+Arg_conc.+emission_CO2+i nterval_O2; 0.189, 0.570, tmp_cumulo+pH+PL_cumulo+RS+RS_cum ulo+Cell_conc._interval+Arg_conc.+emission_O2+interval_O2; 0.207, 0.570, tmp+pH+PL_cumulo+RS_cumulo+Cell_conc._interva l+Arg_conc.+emission_O2+interval_O2; 0.131, 0.570, tmp_cumu lo+pH_cumulo+PL_cumulo+AN_cumulo+Feed_Vol+RS_cumulo+Cell_co nc._interval+Arg_conc.+emission_O2+emission_CO2+interval_O2 +generate_CO2; 0.171, 0.570, tmp+PL_cumulo+Feed_Vol+RS_cumu lo+Cell_conc._interval+RQ_integral+Arg_conc.+emission_CO2+c onsum_O2+interval_O2; 0.131, 0.570, tmp+PL+PL_cumulo+AN_cum ulo+Feed_Vol+RS_cumulo+Cell_conc._interval+Arg_conc.+emissi on_O2+emission_CO2+consum_O2+generate_CO2; 0.152, 0.570, tm p+tmp_cumulo+PL+PL_cumulo+Feed_Vol+RS_cumulo+Cell_conc._int erval+RQ_integral+Arg_conc.+emission_CO2+interval_O2; 0.152, 0.570, tmp+PL_cumulo+RS+Feed_Vol+RS_cumulo+Cell_conc._inte rval+Arg_conc.+emission_O2+emission_CO2+consum_O2+generate CO2; 0.171, 0.570, tmp_cumulo+pH+PL_cumulo+RS_cumulo+Cell_c onc._interval+RQ_integral+Arg_conc.+emission_CO2+interval_O 2+generate_CO2; 0.189, 0.569, tmp+PL_cumulo+Feed_Vol+Cell_c onc._interval+RQ_integral+Arg_conc.+emission_O2+consum_O2+g enerate_CO2; 0.151, 0.569, tmp+pH_cumulo+PL_cumulo+Feed_Vol +RS_cumulo+Cell_conc._interval+Arg_conc.+emission_O2+emissi on_CO2+consum_O2+generate_CO2; 0.131, 0.569, tmp+PL_cumulo+ AN_cumulo+interval_yield+Feed_Vol+RS_cumulo+Cell_conc._inte rval+Arg_conc.+emission_O2+emission_CO2+interval_O2+generat e_CO2; 0.151, 0.569, tmp+PL_cumulo+AN_cumulo+RS+Feed_Vol+RS _cumulo+Cell_conc._interval+Arg_conc.+emission_O2+consum_O2 +interval_O2; 0.189, 0.569, tmp+PL_cumulo+AN_cumulo+Feed_Vo l+Cell_conc._interval+Arg_conc.+emission_O2+interval_O2+gen erate_CO2; 0.151, 0.569, tmp_cumulo+pH_cumulo+PL_cumulo+Fee d_Vol+RS_cumulo+Cell_conc._interval+rab_integral+Arg_conc.+ emission_O2+interval_O2+generate_CO2; 0.189, 0.569, tmp+PL_ cumulo+Feed_Vol+RS_cumulo+Cell_conc._interval+rab_integral+ Arg_conc.+emission_O2+interval_O2; 0.131, 0.569, tmp+PL_cum ulo+AN_cumulo+RS+Feed_Vol+RS_cumulo+Cell_conc._interval+Arg _conc.+emission_O2+emission_CO2+interval_O2+generate_CO2; 0. 110, 0.569, tmp_cumulo+pH+PL_cumulo+AN_cumulo+RS+Feed_Vol+R S_cumulo+Cell_conc._interval+Arg_conc.+emission_O2+emission _CO2+consum_O2+interval_O2; 0.189, 0.569, tmp_cumulo+PL_cum ulo+RS_cumulo+Cell_conc._interval+RQ_integral+Arg_conc.+emi ssion_CO2+consum_O2+interval_O2; 0.151, 0.569, tmp+tmp_cumu lo+pH_cumulo+PL_cumulo+Feed_Vol+RS_cumulo+Cell_conc._interv al+Arg_conc.+emission_O2+consum_O2+interval_O2; 0.151, 0.56 9, tmp+pH+PL_cumulo+interval_yield+Feed_Vol+RS_cumulo+Cell_ conc._interval+Arg_conc.+emission_O2+interval_O2+generate_C O2; 0.151, 0.569, tmp_cumulo+pH+PL+PL_cumulo+RS_cumulo+Cell _conc._interval+rab_integral+Arg_conc.+emission_CO2+consum_ O2+interval_O2; 0.151, 0.569, tmp_cumulo+PL_cumulo+AN_cumul o+Feed_Vol+RS_cumulo+Cell_conc._interval+RQ_integral+Arg_co nc.+emission_CO2+consum_O2+generate_CO2; 0.151, 0.569, tmp_ cumulo+pH+PL_cumulo+AN_cumulo+Feed_Vol+RS_cumulo+Cell_conc. _interval+Arg_conc.+emission_O2+emission_CO2+interval_O2; 0. 151, 0.569, tmp_cumulo+pH+PL_cumulo+Feed_Vol+RS_cumulo+Cell _conc._interval+RQ_integral+Arg_conc.+emission_O2+interval_ O2+generate_CO2; 0.131, 0.569, tmp_cumulo+PL_cumulo+AN_cumu lo+RS+Feed_Vol+RS_cumulo+Cell_conc._interval+RQ_integral+Ar g_conc.+emission_CO2+interval_O2+generate_CO2; 0.170, 0.569, tmp+PL_cumulo+Feed_Vol+RS_cumulo+Cell_conc._interval+RQ_in tegral+Arg_conc.+emission_O2+consum_O2+interval_O2; 0.170, 0.569, tmp_cumulo+pH+PL_cumulo+RS_cumulo+Cell_conc._interva 1+RQ_integral+Arg_conc.+emission_CO2+consum_O2+interval_O2; 0.131, 0.569, tmp+PL+PL_cumulo+AN_cumulo+interval_yield+Fe ed_Vol+RS_cumulo+Cell_conc._interval+Arg_conc.+emission_O2+ interval_O2+generate_CO2; 0.170, 0.569, tmp_cumulo+PL+PL_cu mulo+AN_cumulo+RS_cumulo+Cell_conc._interval+rab_integral+A rg_conc.+emission_CO2+interval_O2; 0.170, 0.569, tmp+PL+PL_ cumulo+AN_cumulo+RS_cumulo+Cell_conc._interval+rab_integral +Arg_conc.+emission_CO2+interval_O2; 0.151, 0.569, tmp_cumu lo+PL_cumulo+Feed_Vol+RS_cumulo+Cell_conc._interval+rab_int egral+RQ_integral+Arg_conc.+emission_CO2+interval_O2+genera te_CO2; 0.151, 0.569, tmp+PL+PL_cumulo+AN_cumulo+RS+Feed_Vo l+Cell_conc._interval+Arg_conc.+emission_O2+consum_O2+gener ate_CO2; 0.170, 0.569, tmp_cumulo+PL_cumulo+AN_cumulo+Feed_ Vol+RS_cumulo+Cell_conc._interval+RQ_integral+Arg_conc.+emi ssion_O2+interval_O2; 0.151, 0.569, tmp+pH_cumulo+PL_cumulo +interval_yield+Feed_Vol+RS_cumulo+Cell_conc._interval+Arg_ conc.+emission_O2+interval_O2+generate_CO2; 0.131, 0.569, t mp_cumulo+pH+PL_cumulo+AN_cumulo+Feed_Vol+RS_cumulo+Cell_co nc._interval+rab_integral+Arg_conc.+emission_O2+interval_O2 +generate_CO2; 0.131, 0.569, tmp_cumulo+pH+PL_cumulo+RS+Fee d_Vol+RS_cumulo+Cell_conc._interval+Arg_conc.+emission_O2+e mission_CO2+consum_O2+interval_O2; 0.151, 0.569, tmp_cumulo +pH_cumulo+PL_cumulo+AN+Feed_Vol+RS_cumulo+Cell_conc._inter val+RQ_integral+Arg_conc.+emission_CO2+interval_O2; 0.170, 0.569, tmp_cumulo+pH+PL_cumulo+interval_yield+RS_cumulo+Cel l_conc._interval+RQ_integral+Arg_conc.+emission_CO2+interva l_O2; 0.170, 0.569, tmp_cumulo+PL_cumulo+AN_cumulo+interval _yield+Feed_Vol+RS_cumulo+Cell_conc._interval+RQ_integral+A rg_conc.+interval_O2; 0.151, 0.569, tmp_cumulo+PL_cumulo+AN _cumulo+RS+RS_cumulo+Cell_conc._interval+rab_integral+Arg_c onc.+emission_CO2+consum_O2+interval_O2; 0.151, 0.569, tmp+ pH_cumulo+PL_cumulo+RS+Feed_Vol+RS_cumulo+Cell_conc._interv al+Arg_conc.+emission_O2+interval_O2+generate_CO2; 0.189, 0. 569, tmp_cumulo+PL_cumulo+interval_yield+RS_cumulo+Cell_con c._interval+rab_integral+Arg_conc.+emission_CO2+interval_O 2; 0.151, 0.569, tmp_cumulo+pH_cumulo+PL+PL_cumulo+Feed_Vol +RS_cumulo+Cell_conc._interval+Arg_conc.+emission_O2+consum _O2+interval_O2; 0.170, 0.569, tmp+PL_cumulo+AN_cumulo+Feed _Vol+Cell_conc._interval+RQ_integral+Arg_conc.+emission_O2+ consum_O2+generate_CO2; 0.170, 0.569, tmp+PL_cumulo+Feed_Vo l+RS_cumulo+Cell_conc._interval+rab_integral+Arg_conc.+emis sion_O2+consum_O2+interval_O2; 0.130, 0.569, tmp+pH+PL_cumu lo+AN_cumulo+Feed_Vol+RS_cumulo+Cell_conc._interval+rab_int egral+Arg_conc.+emission_O2+consum_O2+interval_O2; 0.151, 0. 569, tmp_cumulo+PL_cumulo+AN_cumulo+Feed_Vol+RS_cumulo+Cell _conc._interval+rab_integral+Arg_conc.+emission_O2+consum_O 2+interval_O2; 0.130, 0.569, tmp_cumulo+PL_cumulo+AN_cumulo +Feed_Vol+RS_cumulo+Cell_conc._interval+RQ_integral+Arg_con c.+emission_O2+emission_CO2+interval_O2+generate_CO2; 0.151, 0.569, tmp_cumulo+pH_cumulo+PL_cumulo+interval_yield+Feed_ Vol+RS_cumulo+Cell_conc._interval+rab_integral+Arg_conc.+em ission_O2+interval_O2; 0.130, 0.569, tmp+PL_cumulo+AN_cumul o+Feed_Vol+RS_cumulo+Cell_conc._interval+RQ_integral+Arg_co nc.+emission_O2+emission_CO2+interval_O2+generate_CO2; 0.15 0, 0.569, tmp_cumulo+PL_cumulo+AN_cumulo+Feed_Vol+RS_cumulo +Cell_conc._interval+Arg_conc.+emission_O2+consum_O2+interv al_O2+generate_CO2; 0.150, 0.569, tmp+pH+PL_cumulo+Feed_Vol +RS_cumulo+Cell_conc._interval+rab_integral+Arg_conc.+emiss ion_O2+interval_O2+generate_CO2; 0.150, 0.569, tmp+pH+PL_cu mulo+AN_cumulo+RS+RS_cumulo+Cell_conc._interval+rab_integra l+Arg_conc.+emission_CO2+interval_O2; 0.130, 0.569, tmp+pH+ PL_cumulo+AN_cumulo+interval_yield+Feed_Vol+RS_cumulo+Cell_ conc._interval+Arg_conc.+emission_O2+interval_O2+generate_C O2; 0.170, 0.569, tmp_cumulo+pH_cumulo+PL_cumulo+Feed_Vol+R S_cumulo+Cell_conc._interval+Arg_conc.+emission_O2+emission _CO2+interval_O2; 0.130, 0.569, tmp_cumulo+pH+PL_cumulo+AN_ cumulo+interval_yield+RS+RS_cumulo+Cell_conc._interval+rab_ integral+Arg_conc.+emission_CO2+interval_O2; 0.170, 0.569, tmp_cumulo+pH+PL_cumulo+RS+RS_cumulo+Cell_conc._interval+ra b_integral+Arg_conc.+emission_O2+interval_O2; 0.130, 0.569, tmp_cumulo+pH_cumulo+PL_cumulo+AN_cumulo+Feed_Vol+RS_cumul o+Cell_conc._interval+Arg_conc.+emission_O2+consum_O2+inter val_O2+generate_CO2; 0.170, 0.569, tmp_cumulo+pH+PL_cumulo+ AN+Feed_Vol+RS_cumulo+Cell_conc._interval+Arg_conc.+emissio n_O2+interval_O2; 0.150, 0.569, tmp_cumulo+pH+PL+PL_cumulo+ AN+RS_cumulo+Cell_conc._interval+rab_integral+Arg_conc.+emi ssion_CO2+interval_O2; 0.150, 0.569, tmp_cumulo+pH_cumulo+P L_cumulo+Feed_Vol+RS_cumulo+Cell_conc._interval+rab_integra l+RQ_integral+Arg_conc.+emission_CO2+interval_O2; 0.169, 0. 569, tmp+pH+PL_cumulo+AN_cumulo+Feed_Vol+RS_cumulo+Cell_con c._interval+Arg_conc.+emission_O2+interval_O2; 0.150, 0.569, tmp_cumulo+pH_cumulo+PL_cumulo+AN_cumulo+RS_cumulo+Cell_co nc._cumulo+Cell_conc._interval+rab_integral+Arg_conc.+emiss ion_CO2+interval_O2; 0.150, 0.569, tmp+PL+PL_cumulo+Feed_Vo l+RS_cumulo+Cell_conc._interval+RQ_integral+Arg_conc.+emiss ion_CO2+consum_O2+generate_CO2; 0.150, 0.569, tmp_cumulo+pH _cumulo+PL_cumulo+RS+RS_cumulo+Cell_conc._interval+rab_inte gral+Arg_conc.+emission_CO2+interval_O2+generate_CO2; 0.150, 0.569, tmp_cumulo+PL_cumulo+AN_cumulo+Feed_Vol+RS_cumulo+C ell_conc._interval+Arg_conc.+emission_O2+emission_CO2+consu m_O2+generate_CO2; 0.169, 0.569, tmp_cumulo+PL_cumulo+Feed_ Vol+RS_cumulo+Cell_conc._interval+rab_integral+Arg_conc.+em ission_CO2+consum_O2+interval_O2; 0.169, 0.568, tmp+pH+PL_c umulo+Feed_Vol+RS_cumulo+Cell_conc._interval+rab_integral+A rg_conc.+emission_O2+interval_O2; 0.150, 0.568, tmp_cumulo+ PL_cumulo+interval_yield+Feed_Vol+RS_cumulo+Cell_conc._inte rval+rab_integral+Arg_conc.+emission_O2+interval_O2+generat e_CO2; 0.150, 0.568, tmp_cumulo+PL+PL_cumulo+RS+Feed_Vol+RS _cumulo+Cell_conc._interval+RQ_integral+Arg_conc.+emission_ CO2+interval_O2; 0.130, 0.568, tmp+PL+PL_cumulo+AN_cumulo+F eed_Vol+RS_cumulo+Cell_conc._interval+RQ_integral+Arg_conc. +emission_CO2+consum_O2+interval_O2; 0.150, 0.568, tmp+PL_c umulo+AN_cumulo+RS+Feed_Vol+Cell_conc._interval+Arg_conc.+e mission_O2+emission_CO2+interval_O2+generate_CO2; 0.188, 0. 568, tmp+PL+PL_cumulo+RS_cumulo+Cell_conc._interval+RQ_inte gral+Arg_conc.+emission_CO2+interval_O2; 0.130, 0.568, tmp+ pH+pH_cumulo+PL_cumulo+AN_cumulo+Feed_Vol+RS_cumulo+Cell_co nc._interval+Arg_conc.+emission_O2+interval_O2+generate_CO 2; 0.150, 0.568, tmp+PL+PL_cumulo+AN_cumulo+RS+RS_cumulo+Ce ll_conc._interval+rab_integral+Arg_conc.+emission_CO2+inter val_O2; 0.150, 0.568, tmp+pH_cumulo+PL+PL_cumulo+Feed_Vol+R S_cumulo+Cell_conc._interval+Arg_conc.+emission_O2+interval _O2+generate_CO2; 0.169, 0.568, tmp_cumulo+pH_cumulo+PL_cum ulo+RS+Feed_Vol+Cell_conc._interval+Arg_conc.+emission_O2+c onsum_O2+interval_O2; 0.169, 0.568, tmp_cumulo+pH+PL_cumulo +RS_cumulo+Cell_conc._interval+rab_integral+Accum._Yield+Ar g_conc.+emission_CO2+interval_O2; 0.187, 0.568, tmp_cumulo+ PL_cumulo+RS_cumulo+Cell_conc._interval+rab_integral+Arg_co nc.+emission_O2+emission_CO2+interval_O2; 0.150, 0.568, tmp +pH+pH_cumulo+PL_cumulo+Feed_Vol+RS_cumulo+Cell_conc._inter val+Arg_conc.+emission_O2+interval_O2+generate_CO2; 0.150, 0.568, tmp_cumulo+pH+PL_cumulo+Feed_Vol+RS_cumulo+Cell_conc. _cumulo+Cell_conc._interval+RQ_integral+Arg_conc.+emission_ CO2+interval_O2; 0.187, 0.568, tmp+pH+PL_cumulo+RS_cumulo+C ell_conc._interval+RQ_integral+Arg_conc.+emission_CO2+inter val_O2; 0.129, 0.568, tmp_cumulo+pH_cumulo+PL_cumulo+AN_cum ulo+interval_yield+Feed_Vol+RS_cumulo+Cell_conc._interval+R Q_integral+Arg_conc.+emission_CO2+interval_O2; 0.187, 0.568, tmp_cumulo+PL_cumulo+Feed_Vol+RS_cumulo+Cell_conc._interva l+Arg_conc.+emission_CO2+interval_O2+generate_CO2; 0.129, 0. 568, tmp_cumulo+pH+pH_cumulo+PL_cumulo+AN_cumulo+Feed_Vol+R S_cumulo+Cell_conc._interval+Arg_conc.+emission_O2+interval _O2+generate_CO2; 0.169, 0.568, tmp+PL_cumulo+RS+RS_cumulo+ Cell_conc._interval+rab_integral+Arg_conc.+emission_CO2+int erval_O2+generate_CO2; 0.149, 0.568, tmp+tmp_cumulo+pH_cumu lo+PL_cumulo+Feed_Vol+RS_cumulo+Cell_conc._interval+Arg_con c.+emission_O2+interval_O2+generate_CO2; 0.149, 0.568, tmp_ cumulo+pH+pH_cumulo+PL_cumulo+RS_cumulo+Cell_conc._interval +rab_integral+Arg_conc.+emission_CO2+consum_O2+interval_O2; 0.149, 0.568, tmp+pH+PL+PL_cumulo+Feed_Vol+RS_cumulo+Cell_ conc._interval+Arg_conc.+emission_O2+interval_O2+generate_C 02; 0.169, 0.568, tmp+PL+PL_cumulo+AN_cumulo+Feed_Vol+Cell_ conc._interval+Arg_conc.+emission_O2+consum_O2+generate_CO 2; 0.149, 0.568, tmp_cumulo+pH+PL+PL_cumulo+RS_cumulo+Cell_ conc._cumulo+Cell_conc._interval+rab_integral+Arg_conc.+emi ssion_CO2+interval_O2; 0.149, 0.568, tmp_cumulo+pH+PL_cumul o+AN+RS_cumulo+Cell_conc._interval+rab_integral+Arg_conc.+e mission_CO2+consum_O2+interval_O2; 0.169, 0.568, tmp_cumulo +PL_cumulo+AN_cumulo+RS_cumulo+Cell_conc._interval+rab_inte gral+Arg_conc.+emission_CO2+interval_O2+generate_CO2; 0.149, 0.568, tmp_cumulo+pH+PL_cumulo+AN_cumulo+OD+RS_cumulo+Cell _conc._interval+rab_integral+Arg_conc.+emission_CO2+interva l_O2; 0.129, 0.568, tmp_cumulo+pH+PL_cumulo+AN_cumulo+OD+Fe ed_Vol+RS_cumulo+Cell_conc._interval+rab_integral+Arg_conc. +emission_CO2+interval_O2; 0.169, 0.568, tmp_cumulo+PL+PL_c umulo+Feed_Vol+RS_cumulo+Cell_conc._interval+Arg_conc.+emis sion_O2+consum_O2+interval_O2; 0.149, 0.568, tmp_cumulo+pH_ cumulo+PL_cumulo+interval_yield+RS_cumulo+Cell_conc._interv al+rab_integral+Arg_conc.+emission_CO2+interval_O2+generate _CO2; 0.168, 0.568, tmp+pH+PL_cumulo+RS_cumulo+Cell_conc._i nterval+rab_integral+Arg_conc.+emission_CO2+interval_O2+gen erate_CO2; 0.149, 0.568, tmp_cumulo+pH+PL_cumulo+RS+Feed_Vo l+RS_cumulo+Cell_conc._interval+rab_integral+Arg_conc.+emis sion_O2+interval_O2; 0.149, 0.568, tmp_cumulo+PL_cumulo+int erval_yield+Feed_Vol+RS_cumulo+Cell_conc._interval+RQ-integ ral+Arg_conc.+emission_O2+emission_CO2+interval_O2; 0.168, 0.568, tmp_cumulo+pH_cumulo+PL_cumulo+RS_cumulo+Cell_conc._ interval+rab_integral+RQ_integral+Arg_conc.+emission_CO2+in terval_O2; 0.149, 0.568, tmp_cumulo+PL_cumulo+Feed_Vol+RS_c umulo+Cell_conc._interval+rab_integral+Arg_conc.+emission_O 2+emission_CO2+interval_O2+generate_CO2; 0.129, 0.568, tmp_ cumulo+PL_cumulo+AN_cumulo+Feed_Vol+RS_cumulo+Cell_conc._in terval+RQ_integral+Arg_conc.+emission_O2+emission_CO2+consu m_O2+interval_O2; 0.149, 0.568, tmp_cumulo+pH+PL_cumulo+RS+ RS_cumulo+Cell_conc._interval+rab_integral+Arg_conc.+emissi on_O2+emission_CO2+interval_O2; 0.149, 0.568, tmp_cumulo+PL +PL_cumulo+AN_cumulo+Feed_Vol+RS_cumulo+Cell_conc._interval +Arg_conc.+emission_O2+consum_O2+interval_O2; 0.107, 0.568, tmp_cumulo+pH+PL_cumulo+AN_cumulo+Feed_Vol+RS_cumulo+Cell conc._interval+rab_integral+Arg_conc.+emission_O2+emission_ CO2+consum_O2+interval_O2; 0.129, 0.568, tmp+pH+PL_cumulo+A N_cumulo+Feed_Vol+RS_cumulo+Cell_conc._interval+rab_integra l+Arg_conc.+emission_O2+interval_O2+generate_CO2; 0.149, 0. 568, tmp+PL+PL_cumulo+Feed_Vol+RS_cumulo+Cell_conc._interva l+rab_integral+Arg_conc.+emission_O2+consum_O2+generate_CO 2; 0.129, 0.568, tmp+tmp_cumulo+pH+PL_cumulo+AN_cumulo+Feed _Vol+RS_cumulo+Cell_conc._interval+Arg_conc.+emission_O2+in terval_O2+generate_CO2; 0.168, 0.568, tmp+PL_cumulo+Feed_Vo l+RS_cumulo+Cell_conc._interval+RQ_integral+Arg_conc.+emiss ion_O2+emission_CO2+interval_O2; 0.149, 0.568, tmp_cumulo+p H_cumulo+PL+PL_cumulo+RS+RS_cumulo+Cell_conc._interval+rab_ integral+Arg_conc.+emission_CO2+interval_O2; 0.187, 0.568, tmp+PL_cumulo+RS_cumulo+Cell_conc._interval+rab_integral+Ar g_conc.+emission_CO2+consum_O2+generate_CO2; 0.168, 0.568, tmp_cumulo+PL+PL_cumulo+Feed_Vol+RS_cumulo+Cell_conc._inter val+rab_integral+Arg_conc.+emission_CO2+interval_O2; 0.149, 0.568, tmp_cumulo+PL_cumulo+AN_cumulo+interval_yield+Feed_ Vol+RS_cumulo+Cell_conc._interval+Arg_conc.+emission_O2+con sum_O2+interval_O2; 0.149, 0.568, tmp+PL_cumulo+RS+Feed_Vol +RS_cumulo+Cell_conc._interval+rab_integral+Arg_conc.+emiss ion_O2+interval_O2+generate_CO2; 0.187, 0.568, tmp+PL_cumul o+Feed_Vol+RS_cumulo+Cell_conc._interval+Arg_conc.+emission _CO2+consum_O2+generate_CO2; 0.128, 0.568, tmp_cumulo+PL_cu mulo+AN_cumulo+interval_yield+Feed_Vol+RS_cumulo+Cell_conc. _interval+RQ_integral+Arg_conc.+emission_CO2+consum_O2+inte rval_O2; 0.149, 0.568, tmp+PL_cumulo+AN_cumulo+RS+Feed_Vol+ Cell_conc._interval+RQ_integral+Arg_conc.+emission_O2+consu m_O2+generate_CO2; 0.128, 0.568, tmp_cumulo+pH_cumulo+PL_cu mulo+AN_cumulo+Feed_Vol+RS_cumulo+Cell_conc._interval+rab_i ntegral+Arg_conc.+emission_O2+emission_CO2+interval_O2; 0.1 68, 0.567, tmp+PL_cumulo+RS+Feed_Vol+RS_cumulo+Cell_conc._i nterval+Arg_conc.+emission_O2+consum_O2+interval_O2; 0.128, 0.567, tmp_cumulo+PL_cumulo+AN_cumulo+interval_yield+Feed_ Vol+RS_cumulo+Cell_conc._interval+Arg_conc.+emission_O2+emi ssion_CO2+interval_O2+generate_CO2; 0.149, 0.567, tmp_cumul o+pH_cumulo+PL_cumulo+RS_cumulo+Cell_conc._interval+rab_int egral+Arg_conc.+emission_CO2+consum_O2+interval_O2+generate _CO2; 0.149, 0.567, tmp+PL+PL_cumulo+Feed_Vol+RS_cumulo+Cel l_conc._interval+RQ_integral+Arg_conc.+emission_O2+consum_O 2+generate_CO2; 0.186, 0.567, tmp_cumulo+pH+PL_cumulo+RS_cu mulo+Cell_conc._interval+Arg_conc.+emission_O2+emission_CO2 +interval_O2; 0.168, 0.567, tmp+pH+PL_cumulo+AN_cumulo+Feed _Vol+Cell_conc._interval+Arg_conc.+emission_O2+interval_O2+ generate_CO2; 0.128, 0.567, tmp+PL+PL_cumulo+AN_cumulo+Feed _Vol+RS_cumulo+Cell_conc._interval+Arg_conc.+emission_O2+em ission_CO2+consum_O2+interval_O2; 0.128, 0.567, tmp_cumulo+ pH+PL_cumulo+AN+AN_cumulo+Feed_Vol+RS_cumulo+Cell_conc._int erval+RQ_integral+Arg_conc.+emission_CO2+interval_O2; 0.186, 0.567, tmp_cumulo+pH_cumulo+PL_cumulo+Feed_Vol+RS_cumulo+C ell_conc._interval+rab_integral+Arg_conc.+interval_O2; 0.14 8, 0.567, tmp+tmp_cumulo+PL_cumulo+AN_cumulo+Feed_Vol+RS_cu mulo+Cell_conc._interval+RQ_integral+Arg_conc.+emission_CO2 +interval_O2; 0.148, 0.567, tmp_cumulo+pH+PL_cumulo+Feed_Vo l+RS_cumulo+Cell_conc._cumulo+Cell_conc._interval+rab_integ ral+Arg_conc.+emission_CO2+interval_O2; 0.128, 0.567, tmp+t mp_cumulo+pH+PL_cumulo+AN_cumulo+Feed_Vol+RS_cumulo+Cell_co nc._interval+RQ_integral+Arg_conc.+emission_CO2+interval_O 2; 0.148, 0.567, tmp+PL_cumulo+Feed_Vol+RS_cumulo+Cell_conc. _interval+RQ_integral+Arg_conc.+emission_O2+emission_CO2+co nsum_O2+generate_CO2; 0.148, 0.567, tmp+tmp_cumulo+pH+PL_cu mulo+RS_cumulo+Cell_conc._interval+rab_integral+Arg_conc.+e mission_CO2+interval_O2+generate_CO2; 0.204, 0.567, tmp+PL_ cumulo+Feed_Vol+RS_cumulo+Cell_conc._interval+RQ_integral+A rg_conc.+interval_O2; 0.148, 0.567, tmp+tmp_cumulo+pH_cumul o+PL_cumulo+AN_cumulo+RS_cumulo+Cell_conc._interval+rab_int egral+Arg_conc.+emission_CO2+interval_O2; 0.148, 0.567, tmp _cumulo+pH_cumulo+PL_cumulo+interval_yield+RS+RS_cumulo+Cel l_conc._interval+rab_integral+Arg_conc.+emission_CO2+interv al_O2; 0.186, 0.567, tmp_cumulo+pH+PL_cumulo+Feed_Vol+RS_cu mulo+Cell_conc._interval+Arg_conc.+emission_CO2+interval_O 2; 0.148, 0.567, tmp_cumulo+pH_cumulo+PL_cumulo+RS+RS_cumul o+Cell_conc._interval+rab_integral+Arg_conc.+emission_CO2+c onsum_O2+interval_O2; 0.148, 0.567, tmp+pH_cumulo+PL_cumulo +AN_cumulo+RS+Feed_Vol+Cell_conc._interval+Arg_conc.+emissi on_O2+consum_O2+interval_O2; 0.128, 0.567, tmp+tmp_cumulo+p H_cumulo+PL_cumulo+AN_cumulo+Feed_Vol+RS_cumulo+Cell_conc._ interval+Arg_conc.+emission_O2+consum_O2+interval_O2; 0.128, 0.567, tmp_cumulo+PL_cumulo+AN_cumulo+Feed_Vol+RS_cumulo+C ell_conc._interval+rab_integral+RQ_integral+Arg_conc.+emiss ion_CO2+consum_O2+interval_O2; 0.167, 0.567, tmp_cumulo+pH_ cumulo+PL_cumulo+RS+RS_cumulo+Cell_conc._interval+RQ_integr al+Arg_conc.+emission_CO2+interval_O2; 0.148, 0.567, tmp+PL +PL_cumulo+RS+Feed_Vol+RS_cumulo+Cell_conc._interval+Arg_co nc.+emission_O2+interval_O2+generate_CO2; 0.128, 0.567, tmp _cumulo+pH+PL_cumulo+AN_cumulo+RS+RS_cumulo+Cell_conc._inte rval+rab_integral+RQ_integral+Arg_conc.+emission_CO2+interv al_O2; 0.148, 0.567, tmp+tmp_cumulo+pH+PL_cumulo+RS+RS_cumu lo+Cell_conc._interval+rab_integral+Arg_conc.+emission_CO2+ interval_O2; 0.148, 0.567, tmp+PL_cumulo+AN_cumulo+Feed_Vol +RS_cumulo+Cell_conc._interval+rab_integral+Arg_conc.+emiss ion_CO2+consum_O2+generate_CO2; 0.148, 0.567, tmp_cumulo+pH +PL+PL_cumulo+Feed_Vol+RS_cumulo+Cell_conc._interval+rab _in tegral+Arg_conc.+emission_O2+interval_O2; 0.167, 0.567, tmp _cumulo+PL_cumulo+interval_yield+RS+RS_cumulo+Cell_conc._in terval+rab_integral+Arg_conc.+emission_CO2+interval_O2; 0.1 48, 0.567, tmp_cumulo+PL_cumulo+AN_cumulo+Feed_Vol+RS_cumul o+Cell_conc._interval+rab_integral+RQ_integral+Arg_conc.+em ission_CO2+interval_O2; 0.186, 0.567, tmp_cumulo+PL_cumulo+ RS_cumulo+Cell_conc._interval+rab_integral+Arg_conc.+emissi on_CO2+consum_O2+generate_CO2; 0.148, 0.567, tmp_cumulo+pH+ PL_cumulo+AN+RS_cumulo+Cell_conc._cumulo+Cell_conc._interva l+rab_integral+Arg_conc.+emission_CO2+interval_O2; 0.167, 0. 567, tmp_cumulo+pH+PL_cumulo+interval_yield+Feed_Vol+RS_cum ulo+Cell_conc._interval+RQ_integral+Arg_conc.+interval_O2; 0.148, 0.567, tmp_cumulo+PL_cumulo+RS+Feed_Vol+RS_cumulo+Ce ll_conc._interval+rab_integral+Arg_conc.+emission_O2+interv al_O2+generate_CO2; 0.186, 0.567, tmp_cumulo+PL_cumulo+AN_c umulo+Feed_Vol+RS_cumulo+Cell_conc._interval+RQ_integral+Ar g_conc.+interval_O2; 0.127, 0.567, tmp_cumulo+pH+PL_cumulo+ AN_cumulo+Feed_Vol+RS_cumulo+Cell_conc._interval+Arg_conc.+ emission_O2+consum_O2+interval_O2+generate_CO2; 0.185, 0.56 7, tmp+PL_cumulo+Feed_Vol+Cell_conc._interval+RQ_integral+A rg_conc.+emission_CO2+consum_O2+generate_CO2; 0.127, 0.567, tmp_cumulo+PL_cumulo+AN_cumulo+RS+Feed_Vol+RS_cumulo+Cell_ conc._interval+Arg_conc.+emission_O2+emission_CO2+interval_ O2+generate_CO2; 0.127, 0.567, tmp+pH+pH_cumulo+PL_cumulo+A N_cumulo+Feed_Vol+RS_cumulo+Cell_conc._interval+Arg_conc.+e mission_O2+consum_O2+interval_O2; 0.127, 0.567, tmp+pH_cumu lo+PL_cumulo+AN_cumulo+interval_yield+Feed_Vol+RS_cumulo+Ce ll_conc._interval+Arg_conc.+emission_O2+interval_O2+generat e_CO2; 0.147, 0.567, tmp+pH+PL_cumulo+RS+Feed_Vol+Cell_conc. _interval+Arg_conc.+emission_O2+emission_CO2+interval_O2+ge nerate_CO2; 0.185, 0.567, tmp_cumulo+pH+PL_cumulo+Feed_Vol+ RS_cumulo+Cell_conc._interval+RQ_integral+Arg_conc.+interva l_O2; 0.167, 0.567, tmp_cumulo+PL+PL_cumulo+Feed_Vol+RS_cum ulo+Cell_conc._interval+RQ_integral+Arg_conc.+emission_O2+i nterval_O2; 0.147, 0.567, tmp+tmp_cumulo+PL_cumulo+Feed_Vol +RS_cumulo+Cell_conc._interval+Arg_conc.+emission_O2+emissi on_CO2+interval_O2+generate_CO2; 0.127, 0.567, tmp_cumulo+P L_cumulo+AN_cumulo+interval_yield+RS+Feed_Vol+RS_cumulo+Cel l_conc._interval+Arg_conc.+emission_O2+interval_O2+generate _CO2; 0.127, 0.567, tmp+PL+PL_cumulo+AN_cumulo+Feed_Vol+RS_ cumulo+Cell_conc._interval+RQ_integral+Arg_conc.+emission_O 2+consum_O2+interval_O2; 0.185, 0.567, tmp_cumulo+pH+PL_cum ulo+AN_cumulo+RS_cumulo+Cell_conc._interval+Arg_conc.+emiss ion_O2+interval_O2; 0.147, 0.567, tmp_cumulo+PL_cumulo+RS+F eed_Vol+RS_cumulo+Cell_conc._interval+RQ_integral+Arg_conc. +emission_CO2+consum_O2+interval_O2; 0.185, 0.567, tmp_cumu lo+PL_cumulo+RS_cumulo+Cell_conc._cumulo+Cell_conc._interva l+rab_integral+Arg_conc.+emission_CO2+interval_O2; 0.106, 0. 567, tmp_cumulo+pH+PL+PL_cumulo+AN_cumulo+Feed_Vol+RS_cumul o+Cell_conc._interval+Arg_conc.+emission_O2+emission_CO2+co nsum_O2+interval_O2; 0.167, 0.567, tmp_cumulo+pH+PL_cumulo+ RS_cumulo+Cell_conc._interval+RQ_integral+Arg_conc.+emissio n_O2+emission_CO2+interval_O2; 0.147, 0.567, tmp_cumulo+pH+ PL_cumulo+RS_cumulo+Cell_conc._cumulo+Cell_conc._interval+r ab_integral+Arg_conc.+emission_CO2+interval_O2+generate_CO 2; 0.167, 0.567, tmp+pH_cumulo+PL_cumulo+RS+RS_cumulo+Cell_ conc._interval+rab_integral+Arg_conc.+emission_CO2+interval _O2; 0.167, 0.567, tmp+PL_cumulo+RS+Feed_Vol+Cell_conc._int erval+RQ_integral+Arg_conc.+emission_CO2+consum_O2+generate _CO2; 0.167, 0.567, tmp_cumulo+PL_cumulo+AN+Feed_Vol+RS_cum ulo+Cell_conc._interval+Arg_conc.+emission_O2+interval_O2+g enerate_CO2; 0.127, 0.566, tmp_cumulo+pH_cumulo+PL_cumulo+A N_cumulo+Feed_Vol+RS_cumulo+Cell_conc._interval+RQ_integral +Arg_conc.+emission_CO2+interval_O2+generate_CO2; 0.147, 0. 566, tmp_cumulo+pH+PL_cumulo+Feed_Vol+RS_cumulo+Cell_conc._ interval+Arg_conc.+emission_O2+emission_CO2+consum_O2+gener ate_CO2; 0.127, 0.566, tmp_cumulo+PL_cumulo+AN_cumulo+RS+Fe ed_Vol+RS_cumulo+Cell_conc._interval+RQ_integral+Arg_conc.+ emission_CO2+consum_O2+interval_O2; 0.147, 0.566, tmp+pH_cu mulo+PL_cumulo+AN_cumulo+Feed_Vol+RS_cumulo+Cell_conc._inte rval+RQ_integral+Arg_conc.+emission_CO2+interval_O2; 0.185, 0.566, tmp+tmp_cumulo+PL_cumulo+RS_cumulo+Cell_conc._inter val+rab_integral+Arg_conc.+emission_CO2+interval_O2; 0.166, 0.566, tmp_cumulo+PL_cumulo+interval_yield+Feed_Vol+RS_cum ulo+Cell_conc._interval+RQ_integral+Arg_conc.+interval_O2+g enerate_CO2; 0.127, 0.566, tmp_cumulo+pH+PL+PL_cumulo+AN_cu mulo+RS_cumulo+Cell_conc._cumulo+Cell_conc._interval+rab_in tegral+Arg_conc.+emission_CO2+interval_O2; 0.127, 0.566, tm p_cumulo+pH_cumulo+PL_cumulo+interval_yield+Feed_Vol+RS_cum ulo+Cell_conc._interval+Arg_conc.+emission_O2+emission_CO2+ consum_O2+interval_O2; 0.147, 0.566, tmp+pH+PL_cumulo+RS+Fe ed_Vol+RS_cumulo+Cell_conc._interval+Arg_conc.+emission_O2+ consum_O2+interval_O2; 0.147, 0.566, tmp_cumulo+pH+PL_cumul o+interval_yield+RS_cumulo+Cell_conc._interval+rab_integral +Arg_conc.+emission_CO2+interval_O2+generate_CO2; 0.166, 0. 566, tmp+pH_cumulo+PL_cumulo+RS+Feed_Vol+Cell_conc._interva l+Arg_conc.+emission_O2+consum_O2+interval_O2; 0.127, 0.566, tmp_cumulo+pH+PL_cumulo+AN_cumulo+OD+Feed_Vol+RS_cumulo+Ce ll_conc._interval+Arg_conc.+emission_O2+consum_O2+interval_ 02; 0.166, 0.566, tmp_cumulo+pH+PL_cumulo+RS_cumulo+Cell_co nc._interval+rab_integral+Arg_conc.+emission_O2+consum_O2+i nterval_O2; 0.166, 0.566, tmp+tmp_cumulo+PL_cumulo+Feed_Vol +RS_cumulo+Cell_conc._interval+Arg_conc.+emission_O2+consum _O2+interval_O2; 0.166, 0.566, tmp_cumulo+pH_cumulo+PL_cumu lo+interval_yield+Feed_Vol+RS_cumulo+Cell_conc._interval+Ar g_conc.+emission_O2+interval_O2; 0.166, 0.566, tmp_cumulo+p H+PL_cumulo+RS+Feed_Vol+Cell_conc._interval+Arg_conc.+emiss ion_O2+interval_O2+generate_CO2; 0.166, 0.566, tmp_cumulo+P L_cumulo+RS+RS_cumulo+Cell_conc._interval+rab_integral+Arg_ conc.+emission_CO2+consum_O2+generate_CO2; 0.166, 0.566, tm p_cumulo+pH_cumulo+PL_cumulo+AN_cumulo+RS_cumulo+Cell_conc. _interval+RQ_integral+Arg_conc.+emission_CO2+interval_O2; 0. 202, 0.566, PL_cumulo+Feed _Vol+RS_cumulo+Cell_conc._interva l+Arg_conc.+emission_O2+consum_O2+generate_CO2; 0.126, 0.56 6, tmp_cumulo+pH+PL_cumulo+RS+Feed_Vol+RS_cumulo+Cell_conc. _interval+Arg_conc.+emission_O2+emission_CO2+interval_O2+ge nerate_CO2; 0.166, 0.566, tmp+pH_cumulo+PL_cumulo+Feed_Vol+ RS_cumulo+Cell_conc._interval+rab_integral+Arg_conc.+emissi on_O2+interval_O2; 0.185, 0.566, tmp+pH+PL_cumulo+Feed_Vol+ Cell_conc._interval+Arg_conc.+emission_O2+consum_O2+generat e_CO2; 0.126, 0.566, tmp+PL_cumulo+AN_cumulo+RS+Feed_Vol+RS _cumulo+Cell_conc._interval+rab_integral+Arg_conc.+emission _O2+interval_O2+generate_CO2; 0.166, 0.566, tmp_cumulo+pH_c umulo+PL_cumulo+RS+Feed_Vol+Cell_conc._interval+Arg_conc.+e mission_O2+interval_O2+generate_CO2; 0.126, 0.566, tmp+pH_c umulo+PL_cumulo+AN_cumulo+RS+Feed_Vol+RS_cumulo+Cell_conc._ interval+Arg_conc.+emission_O2+interval_O2+generate_CO2; 0. 185, 0.566, tmp_cumulo+PL_cumulo+Feed_Vol+RS_cumulo+Cell_co nc._interval+rab_integral+RQ_integral+Arg_conc.+interval_O 2; 0.147, 0.566, tmp_cumulo+PL_cumulo+AN_cumulo+RS+Feed_Vol +RS_cumulo+Cell_conc._interval+Arg_conc.+emission_O2+consum _O2+generate_CO2; 0.126, 0.566, tmp+tmp_cumulo+pH_cumulo+PL _cumulo+AN_cumulo+Feed_Vol+RS_cumulo+Cell_conc._interval+ra b_integral+Arg_conc.+emission_CO2+interval_O2; 0.126, 0.566, tmp_cumulo+pH_cumulo+PL+PL_cumulo+AN_cumulo+RS+RS_cumulo+C ell_conc._interval+rab_integral+Arg_conc.+emission_CO2+inte rval_O2; 0.126, 0.566, tmp_cumulo+pH+PL_cumulo+AN_cumulo+Fe ed_Vol+RS_cumulo+Cell_conc._interval+RQ_integral+Arg_conc.+ emission_O2+interval_O2+generate_CO2; 0.146, 0.566, tmp_cum ulo+pH+PL_cumulo+RS_cumulo+Cell_conc._cumulo+Cell_conc._int erval+rab_integral+Arg_conc.+emission_CO2+consum_O2+interva l_O2; 0.184, 0.566, tmp+pH+PL_cumulo+RS_cumulo+Cell_conc._i nterval+Arg_conc.+emission_O2+consum_O2+interval_O2; 0.105, 0.566, tmp_cumulo+pH+PL+PL_cumulo+AN_cumulo+RS+Feed_Vol+RS _cumulo+Cell_conc._interval+rab_integral+Arg_conc.+emission _CO2+interval_O2; 0.126, 0.566, tmp_cumulo+pH+PL+PL_cumulo+ AN_cumulo+RS_cumulo+Cell_conc._interval+rab_integral+Arg_co nc.+emission_CO2+consum_O2+interval_O2; 0.166, 0.566, tmp+p H_cumulo+PL_cumulo+RS_cumulo+Cell_conc._interval+rab_integr al+Arg_conc.+emission_CO2+interval_O2+generate_CO2; 0.126, 0.566, tmp_cumulo+PL_cumulo+AN_cumulo+Feed_Vol+RS_cumulo+Ce ll_conc._interval+RQ_integral+Arg_conc.+emission_CO2+consum _O2+interval_O2+generate_CO2; 0.166, 0.566, tmp_cumulo+PL+P L_cumulo+RS+RS_cumulo+Cell_conc._interval+RQ_integral+Arg_c onc.+emission_CO2+interval_O2; 0.146, 0.566, tmp+pH+PL_cumu lo+AN_cumulo+RS+Feed_Vol+Cell_conc._interval+Arg_conc.+emis sion_O2+consum_O2+interval_O2; 0.105, 0.566, tmp_cumulo+pH+ PL_cumulo+AN_cumulo+interval_yield+Feed_Vol+RS_cumulo+Cell_ conc._interval+Arg_conc.+emission_O2+emission_CO2+consum_O2 +interval_O2; 0.146, 0.566, tmp+tmp_cumulo+pH_cumulo+PL_cum ulo+RS_cumulo+Cell_conc._interval+rab_integral+Arg_conc.+em ission_CO2+interval_O2+generate_CO2; 0.126, 0.566, tmp+pH+P L_cumulo+AN_cumulo+Feed_Vol+RS_cumulo+Cell_conc._interval+R Q_integral+Arg_conc.+emission_O2+consum_O2+interval_O2; 0.1 26, 0.566, tmp_cumulo+pH_cumulo+PL_cumulo+AN_cumulo+RS+Feed _Vol+RS_cumulo+Cell_conc._interval+Arg_conc.+emission_O2+in terval_O2+generate_CO2; 0.165, 0.566, tmp+PL_cumulo+interva l_yield+RS+Feed_Vol+Cell_conc._interval+Arg_conc.+emission_ O2+consum_O2+generate_CO2; 0.165, 0.566, tmp+pH_cumulo+PL+P L_cumulo+RS_cumulo+Cell_conc._interval+rab_integral+Arg_con c.+emission_CO2+interval_O2; 0.146, 0.566, tmp_cumulo+PL_cu mulo+RS+Feed_Vol+RS_cumulo+Cell_conc._interval+Arg_conc.+em ission_O2+emission_CO2+consum_O2+generate_CO2; 0.146, 0.566, tmp+pH+PL+PL_cumulo+AN_cumulo+RS_cumulo+Cell_conc._interva l+rab_integral+Arg_conc.+emission_CO2+interval_O2; 0.165, 0. 566, tmp_cumulo+PL_cumulo+interval_yield+Feed_Vol+RS_cumulo +Cell_conc._interval+rab_integral+Arg_conc.+emission_CO2+in terval_O2; 0.146, 0.566, tmp+pH_cumulo+PL_cumulo+RS+Feed_Vo l+Cell_conc._interval+Arg_conc.+emission_O2+emission_CO2+co nsum_O2+interval_O2; 0.126, 0.566, tmp_cumulo+PL_cumulo+AN_ cumulo+interval_yield+Feed_Vol+RS_cumulo+Cell_conc._interva l+RQ_integral+Arg_conc.+emission_O2+interval_O2+generate_CO 2; 0.146, 0.566, tmp_cumulo+PL_cumulo+AN_cumulo+interval_yi eld+Feed_Vol+RS_cumulo+Cell_conc._interval+Arg_conc.+emissi on_O2+consum_O2+generate_CO2; 0.126, 0.566, tmp+tmp_cumulo+ PL_cumulo+AN_cumulo+Feed _Vol+RS_cumulo+Cell_conc._interval+ RQ_integral+Arg_conc.+emission_CO2+interval_O2+generate_CO 2; 0.146, 0.566, tmp_cumulo+pH_cumulo+PL_cumulo+AN+Feed_Vol +RS_cumulo+Cell_conc._interval+Arg_conc.+emission_O2+consum _O2+interval_O2; 0.165, 0.566, tmp+PL_cumulo+RS+Feed_Vol+Ce ll_conc._interval+RQ_integral+Arg_conc.+emission_O2+interva l_O2+generate_CO2; 0.165, 0.566, tmp+pH+PL_cumulo+AN_cumulo +Feed_Vol+Cell_conc._interval+Arg_conc.+emission_O2+consum_ O2+generate_CO2; 0.146, 0.566, tmp+PL+PL_cumulo+RS+Feed_Vol +Cell_conc._interval+Arg_conc.+emission_O2+emission_CO2+con sum_O2+generate_CO2; 0.126, 0.566, tmp_cumulo+pH+PL_cumulo+ interval_yield+Feed_Vol+RS_cumulo+Cell_conc._interval+Arg_c onc.+emission_O2+emission_CO2+consum_O2+interval_O2; 0.165, 0.566, tmp+pH+PL+PL_cumulo+RS_cumulo+Cell_conc._interval+r ab_integral+Arg_conc.+emission_CO2+interval_O2; 0.125, 0.56 6, tmp_cumulo+pH+PL_cumulo+interval_yield+Feed_Vol+RS_cumul o+Cell_conc._interval+Arg_conc.+emission_O2+emission_CO2+in terval_O2+generate_CO2; 0.146, 0.566, tmp_cumulo+PL+PL_cumu lo+Feed_Vol+RS_cumulo+Cell_conc._interval+RQ_integral+Arg_c onc.+emission_O2+interval_O2+generate_CO2; 0.146, 0.565, tm p_cumulo+PL_cumulo+interval_yield+Feed_Vol+RS_cumulo+Cell_c onc._interval+Arg_conc.+emission_O2+emission_CO2+consum_O2+ generate_CO2; 0.146, 0.565, tmp_cumulo+PL_cumulo+AN+Feed_Vo l+RS_cumulo+Cell_conc._interval+RQ_integral+Arg_conc.+emiss ion_CO2+interval_O2+generate_CO2; 0.146, 0.565, tmp_cumulo+ pH+PL_cumulo+Feed_Vol+RS_cumulo+Cell_conc._cumulo+Cell_conc. _interval+Arg_conc.+emission_O2+consum_O2+interval_O2; 0.14 6, 0.565, tmp_cumulo+pH_cumulo+PL_cumulo+RS+Feed_Vol+Cell_c onc._interval+Arg_conc.+emission_O2+emission_CO2+consum_O2+ interval_O2; 0.146, 0.565, tmp+PL+PL_cumulo+AN_cumulo+Feed_ Vol+RS_cumulo+Cell_conc._interval+rab_integral+Arg_conc.+em ission_CO2+interval_O2; 0.165, 0.565, tmp_cumulo+PL_cumulo+ Feed_Vol+RS_cumulo+Cell_conc._interval+rab_integral+Arg_con c.+emission_O2+emission_CO2+interval_O2; 0.125, 0.565, tmp_ cumulo+pH+PL_cumulo+AN_cumulo+Feed_Vol+RS_cumulo+Cell_conc. _cumulo+Cell_conc._interval+Arg_conc.+emission_O2+consum_O2 +interval_O2; 0.146, 0.565, tmp+PL_cumulo+AN+AN_cumulo+Feed _Vol+RS_cumulo+Cell_conc._interval+Arg_conc.+emission_O2+in terval_O2+generate_CO2; 0.165, 0.565, tmp_cumulo+PL_cumulo+ Feed _Vol+RS_cumulo+Cell_conc._cumulo+Cell_conc._interval+RQ _integral+Arg_conc.+emission_CO2+interval_O2; 0.146, 0.565, tmp_cumulo+PL_cumulo+RS+Feed_Vol+RS_cumulo+Cell_conc._inte rval+RQ_integral+Arg_conc.+emission_O2+emission_CO2+interva l_O2; 0.146, 0.565, tmp_cumulo+PL_cumulo+AN_cumulo+Feed_Vol +RS_cumulo+Cell_conc._interval+rab_integral+Arg_conc.+emiss ion_CO2+consum_O2+interval_O2; 0.146, 0.565, tmp+pH_cumulo+ PL+PL_cumulo+AN_cumulo+RS_cumulo+Cell_conc._interval+rab_in tegral+Arg_conc.+emission_CO2+interval_O2; 0.125, 0.565, tm p_cumulo+pH+PL_cumulo+AN+AN_cumulo+RS+RS_cumulo+Cell_conc._ interval+rab_integral+Arg_conc.+emission_CO2+interval_O2; 0. 201, 0.565, tmp_cumulo+PL_cumulo+RS_cumulo+Cell_conc._inter val+rab_integral+Arg_conc.+emission_O2+interval_O2; 0.145, 0.565, tmp_cumulo+pH+pH_cumulo+PL+PL_cumulo+RS_cumulo+Cell_ conc._interval+rab_integral+Arg_conc.+emission_CO2+interval _O2; 0.165, 0.565, tmp_cumulo+PL_cumulo+AN_cumulo+Feed_Vol+ RS_cumulo+Cell_conc._interval+rab_integral+Arg_conc.+emissi on_O2+interval_O2; 0.183, 0.565, tmp_cumulo+PL_cumulo+Feed_ Vol+RS_cumulo+Cell_conc._interval+Arg_conc.+emission_CO2+co nsum_O2+generate_CO2; 0.165, 0.565, tmp_cumulo+PL+PL_cumulo +RS_cumulo+Cell_conc._interval+rab_integral+Arg_conc.+emiss ion_CO2+interval_O2+generate_CO2; 0.145, 0.565, tmp_cumulo+ pH+PL_cumulo+interval_yield+Feed_Vol+RS_cumulo+Cell_conc._i nterval+rab_integral+Arg_conc.+emission_O2+interval_O2; 0.1 45, 0.565, tmp_cumulo+pH+PL_cumulo+OD+Feed_Vol+RS_cumulo+Ce ll_conc._interval+RQ_integral+Arg_conc.+emission_CO2+interv al_O2; 0.145, 0.565, tmp_cumulo+pH+PL_cumulo+RS_cumulo+Cell _conc._interval+rab_integral+Arg_conc.+emission_O2+emission _CO2+interval_O2+generate_CO2; 0.165, 0.565, tmp_cumulo+pH+ PL_cumulo+AN_cumulo+RS_cumulo+Cell_conc._interval+rab_integ ral+Arg_conc.+emission_O2+interval_O2; 0.201, 0.565, tmp+PL _cumulo+RS_cumulo+Cell_conc._interval+Arg_conc.+emission_O2 +interval_O2+generate_CO2; 0.145, 0.565, tmp+pH_cumulo+PL_c umulo+Feed_Vol+RS_cumulo+Cell_conc._interval+Arg_conc.+emis sion_O2+consum_O2+interval_O2+generate_CO2; 0.125, 0.565, t mp_cumulo+pH+PL_cumulo+AN_cumulo+Feed_Vol+RS_cumulo+Cell_co nc._cumulo+Cell_conc._interval+rab_integral+Arg_conc.+emiss ion_CO2+interval_O2; 0.145, 0.565, tmp_cumulo+PL_cumulo+Fee d_Vol+RS_cumulo+Cell_conc._interval+Arg_conc.+emission_O2+e mission_CO2+consum_O2+interval_O2+generate_CO2; 0.165, 0.56 5, tmp_cumulo+pH+PL_cumulo+RS+RS_cumulo+Cell_conc._interval +Arg_conc.+emission_O2+consum_O2+interval_O2; 0.125, 0.565, tmp+pH_cumulo+PL_cumulo+AN_cumulo+RS+Feed_Vol+Cell_conc._i nterval+Arg_conc.+emission_O2+emission_CO2+interval_O2+gene rate_CO2; 0.165, 0.565, tmp_cumulo+PL_cumulo+AN_cumulo+RS+R S_cumulo+Cell_conc._interval+RQ_integral+Arg_conc.+emission _CO2+interval_O2; 0.145, 0.565, tmp_cumulo+pH_cumulo+PL+PL_ cumulo+Feed_Vol+RS_cumulo+Cell_conc._interval+Arg_conc.+emi ssion_O2+interval_O2+generate_CO2; 0.145, 0.565, tmp_cumulo +PL+PL_cumulo+Feed_Vol+RS_cumulo+Cell_conc._interval+Arg_co nc.+emission_O2+emission_CO2+interval_O2+generate_CO2; 0.16 5, 0.565, tmp+PL_cumulo+AN_cumulo+Feed_Vol+Cell_conc._inter val+rab_integral+Arg_conc.+emission_O2+consum_O2+generate_C O2; 0.145, 0.565, tmp+PL+PL_cumulo+interval_yield+Feed_Vol+ RS_cumulo+Cell_conc._interval+Arg_conc.+emission_O2+interva l_O2+generate_CO2; 0.183, 0.565, tmp_cumulo+pH+PL+PL_cumulo +RS_cumulo+Cell_conc._interval+Arg_conc.+emission_O2+interv al_O2; 0.125, 0.565, tmp_cumulo+PL+PL_cumulo+AN_cumulo+inte rval_yield+Feed_Vol+RS_cumulo+Cell_conc._interval+RQ_integr al+Arg_conc.+emission_CO2+interval_O2; 0.103, 0.565, tmp_cu mulo+pH+PL_cumulo+AN_cumulo+RS+Feed_Vol+RS_cumulo+Cell_conc. _interval+rab_integral+Arg_conc.+emission_CO2+consum_O2+int erval_O2; 0.145, 0.565, tmp_cumulo+pH+PL_cumulo+AN_cumulo+R S+RS_cumulo+Cell_conc._interval+RQ_integral+Arg_conc.+emiss ion_CO2+interval_O2; 0.145, 0.565, tmp+PL+PL_cumulo+AN_cumu lo+RS+Feed_Vol+Cell_conc._interval+Arg_conc.+emission_O2+in terval_O2+generate_CO2; 0.145, 0.565, tmp_cumulo+pH+PL_cumu lo+AN_cumulo+Feed_Vol+RS_cumulo+Cell_conc._interval+Arg_con c.+emission_O2+consum_O2+generate_CO2; 0.125, 0.565, tmp_cu mulo+pH+PL_cumulo+Feed_Vol+RS_cumulo+Cell_conc._interval+ra b_integral+Arg_conc.+emission_O2+emission_CO2+consum_O2+int erval_O2; 0.183, 0.565, tmp_cumulo+PL_cumulo+interval_yield +RS_cumulo+Cell_conc._interval+RQ_integral+Arg_conc.+emissi on_CO2+interval_O2; 0.164, 0.565, tmp+PL_cumulo+AN_cumulo+F eed_Vol+Cell_conc._interval+Arg_conc.+emission_O2+emission-CO2+interval_O2+generate_CO2; 0.145, 0.565, tmp_cumulo+PL_c umulo+interval_yield+Feed_Vol+RS_cumulo+Cell_conc._interval +RQ_integral+Arg_conc.+emission_CO2+consum_O2+generate_CO2; 0.145, 0.565, tmp+pH+PL_cumulo+Feed_Vol+RS_cumulo+Cell_con c._interval+Arg_conc.+emission_O2+consum_O2+interval_O2+gen erate_CO2; 0.164, 0.565, tmp+pH_cumulo+PL_cumulo+AN_cumulo+ Feed_Vol+Cell_conc._interval+Arg_conc.+emission_O2+interval _O2+generate_CO2; 0.145, 0.565, tmp+PL+PL_cumulo+RS+Feed_Vo l+RS_cumulo+Cell_conc._interval+Arg_conc.+emission_O2+consu m_O2+generate_CO2; 0.125, 0.565, tmp_cumulo+pH+PL+PL_cumulo +interval_yield+Feed_Vol+RS_cumulo+Cell_conc._interval+RQ_i ntegral+Arg_conc.+emission_CO2+interval_O2; 0.164, 0.565, t mp+PL_cumulo+Feed_Vol+RS_cumulo+Cell_conc._cumulo+Cell_conc. _interval+Arg_conc.+emission_O2+interval_O2+generate_CO2; 0. 145, 0.565, tmp_cumulo+PL+PL_cumulo+Feed_Vol+RS_cumulo+Cell _conc._interval+rab_integral+RQ_integral+Arg_conc.+emission _CO2+interval_O2; 0.183, 0.565, tmp+pH+PL_cumulo+RS_cumulo+ Cell_conc._interval+rab_integral+Arg_conc.+emission_O2+inte rval_O2; 0.201, 0.565, tmp_cumulo+pH+PL_cumulo+RS_cumulo+Ce ll_conc._interval+Accum._Yield+emission_O2+interval_O2; 0.1 24, 0.565, tmp_cumulo+pH+pH_cumulo+PL_cumulo+AN_cumulo+RS_c umulo+Cell_conc._interval+rab_integral+Arg_conc.+emission_C O2+interval_O2+generate_CO2; 0.164, 0.565, tmp_cumulo+PL+PL _cumulo+AN_cumulo+RS_cumulo+Cell_conc._interval+RQ_integral +Arg_conc.+emission_CO2+interval_O2; 0.145, 0.565, tmp_cumu lo+pH+PL_cumulo+AN_cumulo+RS+Feed_Vol+Cell_conc._interval+A rg_conc.+emission_O2+consum_O2+interval_O2; 0.164, 0.565, t mp_cumulo+pH+PL_cumulo+RS_cumulo+Cell_conc._interval+rab_in tegral+Arg_conc.+emission_O2+interval_O2+generate_CO2; 0.14 5, 0.565, tmp_cumulo+pH+pH_cumulo+PL_cumulo+Feed_Vol+RS_cum ulo+Cell_conc._interval+rab_integral+Arg_conc.+emission_O2+ interval_O2; 0.200, 0.565, tmp_cumulo+PL_cumulo+RS_cumulo+C ell_conc._interval+Arg_conc.+emission_O2+consum_O2+interval _O2; 0.145, 0.565, tmp_cumulo+PL+PL_cumulo+AN_cumulo+Feed_V ol+RS_cumulo+Cell_conc._interval+rab_integral+Arg_conc.+emi ssion_CO2+interval_O2; 0.164, 0.565, tmp_cumulo+PL_cumulo+i nterval_yield+Feed_Vol+RS_cumulo+Cell_conc._interval+RQ_int egral+Arg_conc.+emission_O2+interval_O2; 0.183, 0.565, tmp+ PL_cumulo+Feed_Vol+Cell_conc._interval+rab_integral+Arg_con c.+emission_O2+consum_O2+generate_CO2; 0.103, 0.565, tmp_cu mulo+pH+PL+PL_cumulo+AN_cumulo+RS+Feed_Vol+RS_cumulo+Cell_c onc._interval+Arg_conc.+emission_O2+consum_O2+interval_O2; 0.124, 0.565, tmp_cumulo+pH_cumulo+PL_cumulo+interval_yield +Feed_Vol+RS_cumulo+Cell_conc._interval+Arg_conc.+emission_ O2+emission_CO2+interval_O2+generate_CO2; 0.124, 0.565, tmp +PL_cumulo+AN_cumulo+Feed_Vol+RS_cumulo+Cell_conc._interval +Arg_conc.+emission_O2+emission_CO2+consum_O2+interval_O2+g enerate_CO2; 0.144, 0.565, tmp+pH+PL_cumulo+AN_cumulo+RS+Fe ed_Vol+Cell_conc._interval+Arg_conc.+emission_O2+consum_O2+ generate_CO2; 0.144, 0.565, tmp_cumulo+pH+PL_cumulo+AN_cumu lo+RS+Feed_Vol+RS_cumulo+Cell_conc._interval+Arg_conc.+emis sion_O2+interval_O2; 0.164, 0.565, tmp_cumulo+pH_cumulo+PL_ cumulo+RS+Feed_Vol+Cell_conc._interval+RQ_integral+Arg_conc. +emission_CO2+interval_O2; 0.164, 0.565, tmp+tmp_cumulo+pH+ PL_cumulo+RS_cumulo+Cell_conc._interval+RQ_integral+Arg_con c.+emission_CO2+interval_O2; 0.144, 0.565, tmp_cumulo+PL+PL _cumulo+Feed_Vol+RS_cumulo+Cell_conc._interval+RQ_integral+ Arg_conc.+emission_CO2+consum_O2+generate_CO2; 0.164, 0.564, tmp+PL+PL_cumulo+RS+RS_cumulo+Cell_conc._interval+rab_inte gral+Arg_conc.+emission_CO2+interval_O2; 0.124, 0.564, tmp+ PL+PL_cumulo+AN_cumulo+Feed_Vol+RS_cumulo+Cell_conc._interv al+Arg_conc.+emission_O2+consum_O2+interval_O2+generate_CO 2; 0.103, 0.564, tmp+PL+PL_cumulo+AN_cumulo+Feed_Vol+RS_cum ulo+Cell_conc._interval+rab_integral+Arg_conc.+emission_O2+ emission_CO2+interval_O2+generate_CO2; 0.164, 0.564, tmp_cu mulo+pH_cumulo+PL+PL_cumulo+RS_cumulo+Cell_conc._interval+R Q_integral+Arg_conc.+emission_CO2+interval_O2; 0.144, 0.564, tmp+pH+PL_cumulo+RS+Feed_Vol+Cell_conc._interval+Arg_conc. +emission_O2+emission_CO2+consum_O2+generate_CO2; 0.103, 0. 564, tmp_cumulo+pH+PL_cumulo+AN_cumulo+Feed_Vol+RS_cumulo+C ell_conc._interval+RQ_integral+Arg_conc.+emission_O2+emissi on_CO2+consum_O2+interval_O2; 0.144, 0.564, tmp+PL_cumulo+A N_cumulo+RS+Feed_Vol+Cell_conc._interval+RQ_integral+Arg_co nc.+emission_O2+interval_O2+generate_CO2; 0.124, 0.564, tmp _cumulo+pH+pH_cumulo+PL_cumulo+Feed_Vol+RS_cumulo+Cell_conc. _interval+Arg_conc.+emission_O2+emission_CO2+consum_O2+inte rval_O2; 0.124, 0.564, tmp_cumulo+pH_cumulo+PL+PL_cumulo+AN _cumulo+RS_cumulo+Cell_conc._interval+rab_integral+Arg_conc. +emission_CO2+interval_O2+generate_CO2; 0.144, 0.564, tmp_c umulo+pH_cumulo+PL_cumulo+AN_cumulo+Feed_Vol+RS_cumulo+Cell _conc._interval+RQ_integral+Arg_conc.+emission_O2+interval_ 02; 0.164, 0.564, tmp_cumulo+PL_cumulo+AN_cumulo+RS_cumulo+ Cell_conc._interval+rab_integral+Arg_conc.+emission_CO2+con sum_O2+interval_O2; 0.102, 0.564, tmp_cumulo+pH_cumulo+PL_c umulo+AN_cumulo+interval_yield+Feed_Vol+RS_cumulo+Cell-conc. _interval+Arg_conc.+emission_O2+emission_CO2+consum_O2+inte rval_O2; 0.124, 0.564, tmp_cumulo+pH_cumulo+PL_cumulo+AN_cu mulo+Feed_Vol+RS_cumulo+Cell_conc._interval+rab_integral+RQ _integral+Arg_conc.+emission_CO2+interval_O2; 0.164, 0.564, tmp_cumulo+pH_cumulo+PL_cumulo+RS_cumulo+Cell_conc._interv al+rab_integral+RQ_integral+Arg_conc.+emission_O2+interval 02; 0.144, 0.564, tmp+PL_cumulo+interval_yield+Feed_Vol+RS_ cumulo+Cell_conc._interval+rab_integral+Arg_conc.+emission_ O2+interval_O2+generate_CO2; 0.124, 0.564, tmp_cumulo+pH+PL +PL_cumulo+AN+AN_cumulo+RS_cumulo+Cell_conc._interval+rab_i ntegral+Arg_conc.+emission_CO2+interval_O2; 0.124, 0.564, t mp+PL_cumulo+AN_cumulo+Feed_Vol+RS_cumulo+Cell_conc._interv al+rab_integral+Arg_conc.+emission_O2+emission_CO2+consum_O 2+generate_CO2; 0.124, 0.564, tmp_cumulo+pH+PL_cumulo+AN_cu mulo+RS+RS_cumulo+Cell_conc._interval+rab_integral+Arg_conc. +emission_O2+emission_CO2+interval_O2; 0.163, 0.564, tmp+tm p_cumulo+pH+PL_cumulo+Feed_Vol+RS_cumulo+Cell_conc._interva l+Arg_conc.+emission_O2+interval_O2; 0.144, 0.564, tmp+tmp_ cumulo+PL_cumulo+AN_cumulo+Feed_Vol+RS_cumulo+Cell_conc._in terval+Arg_conc.+emission_O2+consum_O2+interval_O2; 0.123, 0.564, tmp+pH_cumulo+PL_cumulo+AN_cumulo+RS+Feed_Vol+RS_cum ulo+Cell_conc._interval+Arg_conc.+emission_O2+consum_O2+int erval_O2; 0.144, 0.564, tmp_cumulo+pH_cumulo+PL_cumulo+AN_c umulo+interval_yield+Feed_Vol+RS_cumulo+Cell_conc._interval +rab_integral+Arg_conc.+interval_O2; 0.182, 0.564, PL_cumul o+RS+Feed_Vol+RS_cumulo+Cell_conc._interval+Arg_conc.+emiss ion_O2+consum_O2+generate_CO2; 0.163, 0.564, tmp_cumulo+pH+ PL_cumulo+RS+Feed_Vol+Cell_conc._interval+Arg_conc.+emissio n_O2+consum_O2+interval_O2; 0.182, 0.564, tmp_cumulo+PL_cum ulo+AN+RS_cumulo+Cell_conc._interval+rab_integral+Arg_conc. +emission_CO2+interval_O2; 0.144, 0.564, tmp_cumulo+pH_cumu lo+PL+PL_cumulo+RS_cumulo+Cell_conc._interval+rab_integral+ Arg_conc.+emission_CO2+consum_O2+interval_O2; 0.163, 0.564, tmp_cumulo+PL_cumulo+RS+RS_cumulo+Cell_conc._interval+rab_ integral+Arg_conc.+emission_O2+emission_CO2+interval_O2; 0. 163, 0.564, tmp+pH+PL_cumulo+Feed_Vol+RS_cumulo+Cell_conc._ interval+Arg_conc.+emission_O2+emission_CO2+interval_O2; 0. 102, 0.564, tmp_cumulo+pH+PL_cumulo+AN_cumulo+interval_yiel d+RS+Feed_Vol+RS_cumulo+Cell_conc._interval+Arg_conc.+emiss ion_O2+consum_O2+interval_O2; 0.144, 0.564, tmp_cumulo+pH_c umulo+PL_cumulo+Feed _Vol+RS_cumulo+Cell_conc._interval+RQ_i ntegral+Arg_conc.+emission_O2+interval_O2+generate_CO2; 0.1 44, 0.564, tmp+pH+PL_cumulo+AN+Feed_Vol+RS_cumulo+Cell_conc. _interval+Arg_conc.+emission_O2+interval_O2+generate_CO2; 0. 123, 0.564, tmp+PL+PL_cumulo+AN_cumulo+Feed_Vol+RS_cumulo+C ell_conc._interval+RQ_integral+Arg_conc.+emission_CO2+consu m_O2+generate_CO2; 0.102, 0.564, tmp_cumulo+pH+pH_cumulo+PL _cumulo+AN_cumulo+Feed_Vol+RS_cumulo+Cell_conc._interval+Ar g_conc.+emission_O2+emission_CO2+consum_O2+interval_O2; 0.1 23, 0.564, tmp+tmp_cumulo+pH+PL_cumulo+AN_cumulo+RS+RS_cumu lo+Cell_conc._interval+rab_integral+Arg_conc.+emission_CO2+ interval_O2; 0.144, 0.564, tmp_cumulo+pH+PL_cumulo+RS_cumul o+Cell_conc._interval+rab_integral+Arg_conc.+emission_CO2+c onsum_O2+interval_O2+generate_CO2; 0.123, 0.564, tmp_cumulo +PL_cumulo+AN_cumulo+Feed_Vol+RS_cumulo+Cell_conc._interval +rab_integral+RQ_integral+Arg_conc.+emission_CO2+interval_O 2+generate_CO2; 0.102, 0.564, tmp_cumulo+pH+PL+PL_cumulo+AN _cumulo+Feed_Vol+RS_cumulo+Cell_conc._interval+RQ_integral+ Arg_conc.+emission_CO2+consum_O2+interval_O2; 0.144, 0.564, tmp_cumulo+pH+PL+PL_cumulo+AN_cumulo+Feed_Vol+RS_cumulo+Ce ll_conc._interval+Arg_conc.+emission_O2+interval_O2; 0.144, 0.564, tmp_cumulo+pH+PL_cumulo+OD+Feed_Vol+RS_cumulo+Cell_ conc._interval+rab_integral+Arg_conc.+emission_CO2+interval _O2; 0.102, 0.564, tmp+PL+PL_cumulo+AN_cumulo+Feed_Vol+RS_c umulo+Cell_conc._interval+RQ_integral+Arg_conc.+emission_O2 +emission_CO2+interval_O2+generate_CO2; 0.163, 0.564, tmp+P L_cumulo+AN_cumulo+Feed_Vol+Cell_conc._interval+RQ_integral +Arg_conc.+emission_CO2+consum_O2+generate_CO2; 0.123, 0.56 4, tmp+PL+PL_cumulo+AN_cumulo+Feed_Vol+RS_cumulo+Cell_conc. _interval+rab_integral+Arg_conc.+emission_O2+consum_O2+inte rval_O2; 0.143, 0.564, tmp_cumulo+PL+PL_cumulo+AN+Feed_Vol+ RS_cumulo+Cell_conc._interval+RQ_integral+Arg_conc.+emissio n_CO2+interval_O2; 0.123, 0.564, tmp_cumulo+pH+PL_cumulo+RS +Feed_Vol+RS_cumulo+Cell_conc._interval+rab_integral+Arg_co nc.+emission_CO2+interval_O2+generate_CO2; 0.123, 0.564, tm p_cumulo+PL+PL_cumulo+interval_yield+Feed_Vol+RS_cumulo+Cel l_conc._interval+RQ_integral+Arg_conc.+emission_CO2+interva l_O2+generate_CO2; 0.143, 0.564, tmp+pH+PL_cumulo+AN_cumulo +Feed_Vol+RS_cumulo+Cell_conc._interval+rab_integral+Arg_co nc.+emission_O2+interval_O2; 0.123, 0.564, tmp+PL+PL_cumulo +AN_cumulo+Feed_Vol+RS_cumulo+Cell_conc._interval+RQ_integr al+Arg_conc.+emission_O2+consum_O2+generate_CO2; 0.123, 0.5 64, tmp_cumulo+pH_cumulo+PL_cumulo+RS+Feed_Vol+RS_cumulo+Ce ll_conc._interval+Arg_conc.+emission_O2+emission_CO2+consum _O2+interval_O2; 0.143, 0.564, tmp_cumulo+pH_cumulo+PL_cumu lo+AN_cumulo+RS+Feed_Vol+Cell_conc._interval+Arg_conc.+emis sion_O2+consum_O2+interval_O2; 0.143, 0.564, tmp_cumulo+pH+ PL_cumulo+RS_cumulo+Cell_conc._interval+rab_integral+Arg_co nc.+emission_O2+emission_CO2+consum_O2+interval_O2; 0.143, 0.564, tmp_cumulo+pH+PL_cumulo+OD+Feed_Vol+RS_cumulo+Cell_c onc._interval+Arg_conc.+emission_O2+consum_O2+interval_O2; 0.143, 0.564, tmp+tmp_cumulo+PL_cumulo+Feed_Vol+RS_cumulo+C ell_conc._interval+rab_integral+Arg_conc.+emission_O2+inter val_O2+generate_CO2; 0.163, 0.564, tmp_cumulo+pH+PL+PL_cumu lo+RS_cumulo+Cell_conc._interval+rab_integral+Arg_conc.+emi ssion_O2+interval_O2; 0.123, 0.564, tmp_cumulo+pH_cumulo+PL _cumulo+interval_yield+RS+Feed_Vol+RS_cumulo+Cell_conc._int erval+Arg_conc.+emission_O2+consum_O2+interval_O2; 0.123, 0. 564, tmp+pH_cumulo+PL_cumulo+AN_cumulo+RS+Feed_Vol+Cell_con c._interval+Arg_conc.+emission_O2+emission_CO2+consum_O2+in terval_O2; 0.143, 0.564, tmp+pH+PL_cumulo+RS+Feed_Vol+RS_cu mulo+Cell_conc._interval+Arg_conc.+emission_O2+consum_O2+ge nerate_CO2; 0.143, 0.564, tmp_cumulo+PL_cumulo+interval_yie ld+RS+Feed_Vol+RS_cumulo+Cell_conc._interval+Arg_conc.+emis sion_O2+consum_O2+generate_CO2; 0.123, 0.564, tmp+tmp_cumul o+PL+PL_cumulo+AN_cumulo+Feed_Vol+RS_cumulo+Cell_conc._inte rval+RQ_integral+Arg_conc.+emission_CO2+interval_O2; 0.163, 0.564, tmp_cumulo+pH_cumulo+PL_cumulo+RS+Feed_Vol+RS_cumul o+Cell_conc._interval+Arg_conc.+emission_O2+interval_O2; 0. 143, 0.564, tmp_cumulo+pH+PL_cumulo+RS+RS_cumulo+Cell_conc. _interval+rab_integral+RQ_integral+Arg_conc.+emission_CO2+i nterval_O2; 0.123, 0.564, tmp_cumulo+PL+PL_cumulo+AN_cumulo +RS+Feed_Vol+RS_cumulo+Cell_conc._interval+RQ_integral+Arg_ conc.+emission_CO2+interval_O2; 0.123, 0.564, tmp_cumulo+pH +PL_cumulo+interval_yield+Feed_Vol+RS_cumulo+Cell_conc._int erval+RQ_integral+Arg_conc.+emission_CO2+consum_O2+interval _O2; 0.143, 0.564, tmp+PL+PL_cumulo+interval_yield+Feed_Vol +RS_cumulo+Cell_conc._interval+Arg_conc.+emission_O2+consum _O2+generate_CO2; 0.181, 0.564, tmp+PL+PL_cumulo+Feed_Vol+C ell_conc._interval+Arg_conc.+emission_O2+consum_O2+generate _CO2; 0.163, 0.564, tmp+PL_cumulo+interval_yield+Feed_Vol+R S_cumulo+Cell_conc._interval+RQ_integral+Arg_conc.+emission _CO2+interval_O2; 0.101, 0.564, tmp_cumulo+pH+PL+PL_cumulo+ AN_cumulo+Feed_Vol+RS_cumulo+Cell_conc._interval+rab_integr al+Arg_conc.+emission_O2+consum_O2+interval_O2; 0.123, 0.56 4, tmp_cumulo+PL_cumulo+AN_cumulo+interval_yield+Feed_Vol+R S_cumulo+Cell_conc._interval+rab_integral+Arg_conc.+emissio n_O2+interval_O2+generate_CO2; 0.181, 0.564, tmp_cumulo+PL_ cumulo+RS+Feed_Vol+Cell_conc._interval+Arg_conc.+emission_O 2+consum_O2+generate_CO2; 0.123, 0.563, tmp_cumulo+pH+PL_cu mulo+Feed_Vol+RS_cumulo+Cell_conc._interval+RQ_integral+Arg _conc.+emission_O2+emission_CO2+consum_O2+interval_O2; 0.16 2, 0.563, tmp+PL+PL_cumulo+Feed_Vol+RS_cumulo+Cell_conc._in terval+rab_integral+Arg_conc.+emission_CO2+interval_O2; 0.1 22, 0.563, tmp_cumulo+pH+PL+PL_cumulo+AN_cumulo+Feed_Vol+RS _cumulo+Cell_conc._interval+rab_integral+Arg_conc.+emission _O2+interval_O2; 0.122, 0.563, tmp+pH_cumulo+PL_cumulo+AN_c umulo+Feed_Vol+RS_cumulo+Cell_conc._interval+rab_integral+A rg_conc.+emission_O2+consum_O2+interval_O2; 0.143, 0.563, t mp_cumulo+pH_cumulo+PL_cumulo+RS+RS_cumulo+Cell_conc.-cumul o+Cell_conc._interval+rab_integral+Arg_conc.+emission_CO2+i nterval_O2; 0.143, 0.563, tmp_cumulo+pH_cumulo+PL_cumulo+AN _cumulo+interval_yield+Feed_Vol+RS_cumulo+Cell_conc._interv al+RQ_integral+Arg_conc.+interval_O2; 0.181, 0.563, tmp+PL_ cumulo+RS_cumulo+Cell_conc._interval+rab_integral+Arg_conc. +emission_CO2+consum_O2+interval_O2; 0.122, 0.563, tmp+PL_c umulo+AN_cumulo+RS+Feed_Vol+RS_cumulo+Cell_conc._interval+r ab_integral+Arg_conc.+emission_CO2+interval_O2+generate_CO 2; 0.143, 0.563, tmp+PL_cumulo+interval_yield+Feed_Vol+RS_c umulo+Cell_conc._interval+RQ_integral+Arg_conc.+emission_O2 +interval_O2+generate_CO2; 0.181, 0.563, tmp_cumulo+PL_cumu lo+AN_cumulo+Feed_Vol+RS_cumulo+Cell_conc._interval+Arg_con c.+emission_O2+interval_O2; 0.181, 0.563, tmp_cumulo+pH+PL_ cumulo+interval_yield+RS_cumulo+Cell_conc._interval+Arg_con c.+emission_O2+interval_O2; 0.122, 0.563, tmp_cumulo+PL_cum ulo+AN_cumulo+RS+Feed_Vol+RS_cumulo+Cell_conc._interval+rab _integral+Arg_conc.+emission_CO2+consum_O2+interval_O2; 0.1 22, 0.563, tmp+pH+PL_cumulo+AN_cumulo+interval_yield+Feed_V ol+RS_cumulo+Cell_conc._interval+Arg_conc.+emission_O2+cons um_O2+interval_O2; 0.162, 0.563, tmp_cumulo+pH+PL_cumulo+RS _cumulo+Cell_conc._interval+rab_integral+Arg_conc.+emission _CO2+consum_O2+generate_CO2; 0.122, 0.563, tmp_cumulo+pH_cu mulo+PL_cumulo+AN_cumulo+Feed_Vol+RS_cumulo+Cell_conc._inte rval+RQ_integral+Arg_conc.+emission_O2+emission_CO2+interva l_O2; 0.162, 0.563, tmp+pH+PL_cumulo+Feed_Vol+RS_cumulo+Cel l_conc._interval+RQ_integral+Arg_conc.+emission_O2+interval _O2; 0.101, 0.563, tmp_cumulo+pH+PL_cumulo+AN_cumulo+RS+Fee d_Vol+RS_cumulo+Cell_conc._interval+rab_integral+Arg_conc.+ emission_O2+consum_O2+interval_O2; 0.143, 0.563, tmp+pH_cum ulo+PL_cumulo+Feed_Vol+RS_cumulo+Cell_conc._interval+rab_in tegral+Arg_conc.+emission_O2+interval_O2+generate_CO2; 0.14 3, 0.563, tmp_cumulo+pH_cumulo+PL+PL_cumulo+Feed_Vol+RS_cum ulo+Cell_conc._interval+rab_integral+Arg_conc.+emission_O2+ interval_O2; 0.122, 0.563, tmp+PL_cumulo+AN_cumulo+interval _yield+Feed_Vol+RS_cumulo+Cell_conc._interval+rab_integral+ Arg_conc.+emission_O2+interval_O2+generate_CO2; 0.143, 0.56 3, tmp_cumulo+pH+PL_cumulo+AN_cumulo+RS_cumulo+Cell_conc._i nterval+rab_integral+Accum._Yield+Arg_conc.+emission_CO2+in terval_O2; 0.162, 0.563, tmp+pH+PL_cumulo+RS_cumulo+Cell_co nc._cumulo+Cell_conc._interval+rab_integral+Arg_conc.+emiss ion_CO2+interval_O2; 0.162, 0.563, tmp_cumulo+PL_cumulo+RS+ RS_cumulo+Cell_conc._interval+RQ_integral+Arg_conc.+emissio n_CO2+interval_O2+generate_CO2; 0.143, 0.563, tmp+PL+PL_cum ulo+RS+Feed_Vol+Cell_conc._interval+RQ_integral+Arg_conc.+e mission_O2+consum_O2+generate_CO2; 0.143, 0.563, tmp+pH_cum ulo+PL_cumulo+RS+Feed_Vol+RS_cumulo+Cell_conc._interval+Arg _conc.+emission_O2+consum_O2+interval_O2; 0.143, 0.563, tmp +PL_cumulo+AN_cumulo+RS+Feed_Vol+Cell_conc._interval+rab_in tegral+Arg_conc.+emission_O2+consum_O2+generate_CO2; 0.143, 0.563, tmp+PL+PL_cumulo+Feed_Vol+RS_cumulo+Cell_conc._inte rval+Arg_conc.+emission_O2+consum_O2+interval_O2+generate_C 02; 0.143, 0.563, tmp+tmp_cumulo+pH+PL+PL_cumulo+RS_cumulo+ Cell_conc._interval+rab_integral+Arg_conc.+emission_CO2+int erval_O2; 0.143, 0.563, tmp_cumulo+pH+PL_cumulo+AN+Feed_Vol +RS_cumulo+Cell_conc._interval+rab_integral+Arg_conc.+emiss ion_O2+interval_O2; 0.122, 0.563, tmp+pH+PL_cumulo+AN_cumul o+RS+Feed_Vol+Cell_conc._interval+Arg_conc.+emission_O2+emi ssion_CO2+interval_O2+generate_CO2; 0.122, 0.563, tmp+pH+PL _cumulo+AN_cumulo+Feed_Vol+RS_cumulo+Cell_conc._interval+RQ _integral+Arg_conc.+emission_O2+interval_O2+generate_CO2; 0. 162, 0.563, tmp_cumulo+PL+PL_cumulo+interval_yield+Feed_Vol +RS_cumulo+Cell_conc._interval+RQ_integral+Arg_conc.+interv al_O2; 0.142, 0.563, tmp+PL_cumulo+RS+Feed_Vol+RS_cumulo+Ce ll_conc._interval+rab_integral+Arg_conc.+emission_O2+consum _O2+generate_CO2; 0.142, 0.563, tmp_cumulo+PL+PL_cumulo+RS+ RS_cumulo+Cell_conc._interval+rab_integral+Arg_conc.+emissi on_CO2+interval_O2+generate_CO2; 0.215, 0.563, tmp_cumulo+P L_cumulo+RS_cumulo+Cell_conc._interval+Arg_conc.+emission_O 2+interval_O2; 0.142, 0.563, tmp_cumulo+pH+PL_cumulo+interv al_yield+RS_cumulo+Cell_conc._interval+rab_integral+Arg_con c.+emission_CO2+consum_O2+interval_O2; 0.142, 0.563, tmp_cu mulo+pH_cumulo+PL_cumulo+AN+RS_cumulo+Cell_conc._interval+r ab_integral+Arg_conc.+emission_CO2+interval_O2+generate_CO 2; 0.181, 0.563, tmp+PL_cumulo+Feed_Vol+RS_cumulo+Cell_conc. _interval+Arg_conc.+emission_CO2+interval_O2+generate_CO2; 0.122, 0.563, tmp_cumulo+PL_cumulo+AN_cumulo+Feed_Vol+RS_cu mulo+Cell_conc._interval+rab_integral+Arg_conc.+emission_O2 +emission_CO2+interval_O2+generate_CO2; 0.142, 0.563, tmp_c umulo+pH_cumulo+PL_cumulo+AN_cumulo+RS_cumulo+Cell_conc._in terval+rab_integral+RQ_integral+Arg_conc.+emission_CO2+inte rval_O2; 0.162, 0.563, tmp_cumulo+pH+PL_cumulo+RS+Feed_Vol+ Cell_conc._interval+RQ_integral+Arg_conc.+emission_CO2+inte rval_O2; 0.142, 0.563, tmp_cumulo+pH+PL_cumulo+RS+Feed_Vol+ RS_cumulo+Cell_conc._interval+Arg_conc.+emission_O2+consum_ O2+generate_CO2; 0.101, 0.563, tmp_cumulo+PL+PL_cumulo+AN_c umulo+interval_yield+Feed_Vol+RS_cumulo+Cell_conc._interval +RQ_integral+Arg_conc.+emission_CO2+interval_O2+generate_CO 2; 0.142, 0.563, tmp+PL_cumulo+Feed_Vol+RS_cumulo+Cell_conc. _interval+rab_integral+RQ_integral+Arg_conc.+emission_O2+in terval_O2+generate_CO2; 0.122, 0.563, tmp+PL+PL_cumulo+AN_c umulo+Feed_Vol+RS_cumulo+Cell_conc._interval+rab_integral+A rg_conc.+emission_CO2+interval_O2+generate_CO2; 0.142, 0.56 3, tmp+pH+PL_cumulo+Feed_Vol+RS_cumulo+Cell_conc._interval+ rab_integral+Arg_conc.+emission_O2+consum_O2+interval_O2; 0. 142, 0.563, tmp+PL+PL_cumulo+Feed_Vol+RS_cumulo+Cell_conc._ interval+RQ_integral+Arg_conc.+emission_CO2+consum_O2+inter val_O2; 0.142, 0.563, tmp+tmp_cumulo+PL_cumulo+Feed_Vol+RS_ cumulo+Cell_conc._interval+RQ_integral+Arg_conc.+emission_O 2+interval_O2+generate_CO2; 0.122, 0.563, tmp_cumulo+pH+PL_ cumulo+interval_yield+RS+Feed_Vol+RS_cumulo+Cell_conc._inte rval+Arg_conc.+emission_O2+consum_O2+interval_O2; 0.180, 0. 563, tmp+pH+PL_cumulo+RS_cumulo+Cell_conc._interval+Arg_con c.+emission_O2+interval_O2+generate_CO2; 0.122, 0.563, tmp+ PL_cumulo+AN_cumulo+interval_yield+Feed_Vol+RS_cumulo+Cell_ conc._interval+Arg_conc.+emission_O2+emission_CO2+consum_O2 +generate_CO2; 0.122, 0.563, tmp_cumulo+pH_cumulo+PL_cumulo +AN_cumulo+RS_cumulo+Cell_conc._interval+rab_integral+Arg_c onc.+emission_CO2+consum_O2+interval_O2+generate_CO2; 0.162, 0.563, tmp+PL_cumulo+RS+Feed_Vol+RS_cumulo+Cell_conc._inte rval+RQ_integral+Arg_conc.+emission_CO2+interval_O2; 0.198, 0.563, tmp_cumulo+PL_cumulo+Feed_Vol+RS_cumulo+Cell_conc._ interval+rab_integral+Arg_conc.+interval_O2; 0.142, 0.563, tmp_cumulo+PL_cumulo+Feed_Vol+RS_cumulo+Cell_conc._interval +rab_integral+RQ_integral+Arg_conc.+emission_O2+emission_CO 2+interval_O2; 0.122, 0.563, tmp_cumulo+pH+PL+PL_cumulo+Fee d_Vol+RS_cumulo+Cell_conc._interval+Arg_conc.+emission_O2+e mission_CO2+consum_O2+interval_O2; 0.122, 0.563, tmp+PL_cum ulo+AN_cumulo+interval_yield+Feed_Vol+RS_cumulo+Cell_conc._ interval+RQ_integral+Arg_conc.+emission_O2+interval_O2+gene rate_CO2; 0.162, 0.563, tmp_cumulo+pH_cumulo+PL_cumulo+RS_c umulo+Cell_conc._interval+rab_integral+Accum._Yield+Arg_con c.+emission_CO2+interval_O2; 0.122, 0.563, tmp+PL_cumulo+AN _cumulo+interval_yield+RS+Feed_Vol+RS_cumulo+Cell_conc._int erval+Arg_conc.+emission_O2+interval_O2+generate_CO2; 0.100, 0.563, tmp_cumulo+pH+PL_cumulo+AN_cumulo+RS+Feed_Vol+RS_cu mulo+Cell_conc._interval+rab_integral+Arg_conc.+emission_CO 2+interval_O2+generate_CO2; 0.198, 0.563, tmp_cumulo+PL_cum ulo+RS_cumulo+Cell_conc._interval+Arg_conc.+emission_O2+int erval_O2+generate_CO2; 0.162, 0.563, tmp+PL+PL_cumulo+Feed_ Vol+RS_cumulo+Cell_conc._interval+RQ_integral+Arg_conc.+emi ssion_O2+interval_O2; 0.122, 0.563, tmp_cumulo+pH+PL_cumulo +AN+AN_cumulo+Feed_Vol+RS_cumulo+Cell_conc._interval+Arg_co nc.+emission_O2+interval_O2+generate_CO2; 0.162, 0.563, tmp _cumulo+pH+PL_cumulo+AN_cumulo+RS_cumulo+Cell_conc._interva l+Arg_conc.+emission_O2+consum_O2+interval_O2; 0.122, 0.563, tmp+PL+PL_cumulo+AN_cumulo+Feed_Vol+RS_cumulo+Cell_conc._i nterval+rab_integral+Arg_conc.+emission_O2+consum_O2+genera te_CO2; 0.142, 0.563, tmp_cumulo+pH+PL_cumulo+Feed_Vol+RS_c umulo+Cell_conc._interval+RQ_integral+Accum._Yield+Arg_conc. +emission_CO2+interval_O2; 0.142, 0.563, tmp_cumulo+pH+PL+P L_cumulo+Feed_Vol+RS_cumulo+Cell_conc._interval+RQ_integral +Arg_conc.+emission_O2+interval_O2; 0.162, 0.563, tmp+pH+PL _cumulo+RS_cumulo+Cell_conc._interval+rab_integral+Arg_conc. +emission_CO2+consum_O2+interval_O2; 0.122, 0.563, tmp_cumu lo+pH+PL_cumulo+Feed_Vol+RS_cumulo+Cell_conc._interval+rab_ integral+Arg_conc.+emission_O2+emission_CO2+interval_O2+gen erate_CO2; 0.161, 0.563, tmp+PL_cumulo+AN_cumulo+Feed_Vol+R S_cumulo+Cell_conc._interval+RQ_integral+Arg_conc.+emission _O2+interval_O2; 0.161, 0.563, tmp+PL_cumulo+RS+Feed_Vol+RS _cumulo+Cell_conc._interval+rab_integral+Arg_conc.+emission _CO2+interval_O2; 0.142, 0.563, tmp_cumulo+pH+PL+PL_cumulo+ interval_yield+RS_cumulo+Cell_conc._interval+rab_integral+A rg_conc.+emission_CO2+interval_O2; 0.122, 0.563, tmp_cumulo +pH_cumulo+PL_cumulo+interval_yield+Feed_Vol+RS_cumulo+Cell _conc._interval+RQ_integral+Arg_conc.+emission_CO2+consum_O 2+interval_02; 0.142, 0.563, tmp_cumulo+pH_cumulo+PL_cumulo +AN+AN_cumulo+RS_cumulo+Cell_conc._interval+rab_integral+Ar g_conc.+emission_CO2+interval_O2; 0.142, 0.563, tmp_cumulo+ pH+PL_cumulo+interval_yield+Feed_Vol+RS_cumulo+Cell_conc._i nterval+RQ_integral+Arg_conc.+emission_O2+interval_O2; 0.14 2, 0.563, tmp_cumulo+pH_cumulo+PL_cumulo+interval_yield+RS_ cumulo+Cell_conc._interval+rab_integral+Arg_conc.+emission_ CO2+consum_O2+interval_O2; 0.198, 0.563, tmp+PL_cumulo+RS_c umulo+Cell_conc._interval+Arg_conc.+emission_O2+consum_O2+i nterval_O2; 0.142, 0.563, tmp+PL_cumulo+AN_cumulo+RS+RS_cum ulo+Cell_conc._interval+rab_integral+Arg_conc.+emission_CO2 +interval_O2+generate_CO2; 0.161, 0.563, tmp_cumulo+PL_cumu lo+RS+Feed_Vol+RS_cumulo+Cell_conc._interval+Arg_conc.+emis sion_CO2+interval_O2+generate_CO2; 0.121, 0.563, tmp_cumulo +PL+PL_cumulo+AN_cumulo+RS+RS_cumulo+Cell_conc._interval+ra b_integral+Arg_conc.+emission_CO2+interval_O2+generate_CO2; 0.121, 0.563, tmp+PL_cumulo+AN_cumulo+Feed_Vol+RS_cumulo+C ell_conc._interval+rab_integral+RQ_integral+Arg_conc.+emiss ion_O2+interval_O2+generate_CO2; 0.180, 0.563, tmp+pH_cumul o+PL_cumulo+Feed_Vol+Cell_conc._interval+Arg_conc.+emission _O2+consum_O2+generate_CO2; 0.142, 0.563, tmp_cumulo+pH_cum ulo+PL_cumulo+RS+Feed_Vol+RS_cumulo+Cell_conc._interval+rab _integral+Arg_conc.+emission_O2+interval_O2; 0.142, 0.563, tmp_cumulo+pH+PL_cumulo+AN_cumulo+RS+Feed_Vol+Cell_conc._in terval+Arg_conc.+emission_O2+interval_O2+generate_CO2; 0.14 2, 0.563, tmp_cumulo+PL_cumulo+RS+Feed_Vol+RS_cumulo+Cell_c onc._interval+rab_integral+Arg_conc.+emission_CO2+consum_O2 +generate_CO2; 0.121, 0.563, tmp_cumulo+pH+PL+PL_cumulo+Fee d_Vol+RS_cumulo+Cell_conc._interval+RQ_integral+Arg_conc.+e mission_CO2+consum_O2+interval_O2; 0.121, 0.563, tmp_cumulo +pH+pH_cumulo+PL+PL_cumulo+Feed_Vol+RS_cumulo+Cell_conc._in terval+RQ_integral+Arg_conc.+emission_CO2+interval_O2; 0.16 1, 0.562, tmp+tmp_cumulo+PL_cumulo+Feed_Vol+RS_cumulo+Cell_ conc._interval+rab_integral+Arg_conc.+emission_CO2+interval _O2; 0.100, 0.562, tmp_cumulo+pH+PL_cumulo+AN_cumulo+interv al_yield+Feed_Vol+RS_cumulo+Cell_conc._interval+RQ_integral +Arg_conc.+emission_CO2+consum_O2+interval_O2; 0.161, 0.562, tmp_cumulo+PL_cumulo+RS+RS_cumulo+Cell_conc._interval+RQ_i ntegral+Arg_conc.+emission_O2+emission_CO2+interval_O2; 0.1 21, 0.562, tmp_cumulo+pH+pH_cumulo+PL_cumulo+RS+Feed_Vol+RS _cumulo+Cell_conc._interval+Arg_conc.+emission_O2+consum_O2 +interval_O2; 0.121, 0.562, tmp_cumulo+pH_cumulo+PL_cumulo+ AN+AN_cumulo+Feed_Vol+RS_cumulo+Cell_conc._interval+Arg_con c.+emission_O2+consum_O2+interval_O2; 0.141, 0.562, tmp+pH+ PL_cumulo+Feed_Vol+RS_cumulo+Cell_conc._interval+RQ_integra l+Arg_conc.+emission_O2+interval_O2+generate_CO2; 0.161, 0. 562, tmp_cumulo+PL+PL_cumulo+RS_cumulo+Cell_conc._interval+ RQ_integral+Arg_conc.+emission_CO2+interval_O2+generate_CO 2; 0.121, 0.562, tmp_cumulo+pH_cumulo+PL_cumulo+Feed_Vol+RS _cumulo+Cell_conc._interval+rab_integral+Arg_conc.+emission _O2+emission_CO2+consum_O2+interval_O2; 0.161, 0.562, tmp+P L_cumulo+RS+RS_cumulo+Cell_conc._interval+rab_integral+Arg_ conc.+emission_CO2+consum_O2+generate_CO2; 0.141, 0.562, tm p+PL_cumulo+Feed_Vol+RS_cumulo+Cell_conc._interval+rab_inte gral+Arg_conc.+emission_O2+consum_O2+interval_O2+generate_C 02; 0.141, 0.562, tmp+PL_cumulo+AN_cumulo+Feed_Vol+RS_cumul o+Cell_conc._cumulo+Cell_conc._interval+Arg_conc.+emission-O2+interval_O2+generate_CO2; 0.099, 0.562, tmp_cumulo+pH+PL +PL_cumulo+AN_cumulo+Feed_Vol+RS_cumulo+Cell_conc._interval +rab_integral+Arg_conc.+emission_CO2+consum_O2+interval_O2; 0.141, 0.562, tmp+pH+PL+PL_cumulo+Feed_Vol+RS_cumulo+Cell_ conc._interval+Arg_conc.+emission_O2+consum_O2+interval_O2; 0.141, 0.562, tmp+PL_cumulo+interval_yield+RS+Feed_Vol+RS_ cumulo+Cell_conc._interval+Arg_conc.+emission_O2+interval_O 2+generate_CO2; 0.141, 0.562, tmp_cumulo+PL_cumulo+RS+Feed_ Vol+RS_cumulo+Cell_conc._interval+Arg_conc.+emission_O2+con sum_O2+interval_O2+generate_CO2; 0.121, 0.562, tmp_cumulo+p H+PL_cumulo+interval_yield+RS+Feed_Vol+RS_cumulo+Cell_conc. interval+Arg_conc.+emission_O2+interval_O2+generate_CO2; 0. 099, 0.562, tmp_cumulo+pH+pH_cumulo+PL_cumulo+AN_cumulo+RS+ Feed_Vol+RS_cumulo+Cell_conc._interval+Arg_conc.+emission_O 2+consum_O2+interval_O2; 0.121, 0.562, tmp_cumulo+pH_cumulo +PL_cumulo+interval_yield+Feed_Vol+RS_cumulo+Cell_conc._int erval+rab_integral+Arg_conc.+emission_CO2+interval_O2+gener ate_CO2; 0.179, 0.562, tmp+pH_cumulo+PL_cumulo+RS_cumulo+Ce ll_conc._interval+rab_integral+Arg_conc.+emission_O2+interv al_O2; 0.141, 0.562, tmp+PL_cumulo+RS+Feed_Vol+RS_cumulo+Ce ll_conc._interval+rab_integral+Arg_conc.+emission_CO2+inter val_O2+generate_CO2; 0.197, 0.562, tmp_cumulo+PL_cumulo+Fee d_Vol+Cell_conc._interval+Arg_conc.+emission_O2+consum_O2+g enerate_CO2; 0.121, 0.562, tmp_cumulo+pH+pH_cumulo+PL_cumul o+Feed_Vol+RS_cumulo+Cell_conc._interval+rab_integral+Arg_c onc.+emission_CO2+interval_O2+generate_CO2; 0.121, 0.562, t mp+tmp_cumulo+PL+PL_cumulo+AN_cumulo+Feed_Vol+RS_cumulo+Cel l_conc._interval+Arg_conc.+emission_O2+interval_O2+generate _CO2; 0.121, 0.562, tmp_cumulo+pH_cumulo+PL_cumulo+AN_cumul o+Feed_Vol+RS_cumulo+Cell_conc._interval+rab_integral+Arg_c onc.+emission_O2+interval_O2+generate_CO2; 0.141, 0.562, tm p_cumulo+PL+PL_cumulo+interval_yield+Feed_Vol+RS_cumulo+Cel l_conc._interval+Arg_conc.+emission_O2+interval_O2+generate _CO2; 0.161, 0.562, tmp_cumulo+PL_cumulo+RS+RS_cumulo+Cell_ conc._cumulo+Cell_conc._interval+rab_integral+Arg_conc.+emi ssion_CO2+interval_O2; 0.141, 0.562, tmp+pH+PL_cumulo+inter val_yield+Feed_Vol+RS_cumulo+Cell_conc._interval+Arg_conc.+ emission_O2+consum_O2+generate_CO2; 0.121, 0.562, tmp_cumul o+pH_cumulo+PL_cumulo+AN+AN_cumulo+Feed_Vol+RS_cumulo+Cell_ conc._interval+rab_integral+Arg_conc.+emission_CO2+interval _O2; 0.121, 0.562, tmp_cumulo+pH_cumulo+PL_cumulo+AN_cumulo +RS+Feed_Vol+RS_cumulo+Cell_conc._interval+RQ_integral+Arg_ conc.+emission_CO2+interval_02; 0.141, 0.562, tmp+tmp_cumul o+PL_cumulo+Feed_Vol+RS_cumulo+Cell_conc._interval+RQ-integ ral+Arg_conc.+emission_CO2+consum_O2+interval_O2; 0.099, 0. 562, tmp_cumulo+pH+PL+PL_cumulo+AN_cumulo+Feed_Vol+RS_cumul o+Cell_conc._interval+RQ_integral+Arg_conc.+emission_O2+con sum_O2+interval_O2; 0.160, 0.562, tmp+PL_cumulo+AN_cumulo+F eed_Vol+RS_cumulo+Cell_conc._interval+rab_integral+Arg_conc. +emission_O2+interval_O2; 0.141, 0.562, tmp+pH_cumulo+PL+PL _cumulo+Feed_Vol+RS_cumulo+Cell_conc._interval+Arg_conc.+em ission_O2+consum_O2+interval_O2; 0.120, 0.562, tmp_cumulo+P L+PL_cumulo+AN_cumulo+Feed_Vol+RS_cumulo+Cell_conc._interva l+RQ_integral+Arg_conc.+emission_O2+interval_O2+generate_CO 2; 0.141, 0.562, tmp+pH_cumulo+PL+PL_cumulo+Feed_Vol+RS_cum ulo+Cell_conc._interval+RQ_integral+Arg_conc.+emission_CO2+ interval_O2; 0.160, 0.562, tmp+PL_cumulo+interval_yield+Fee d_Vol+RS_cumulo+Cell_conc._interval+Arg_conc.+emission_O2+c onsum_O2+interval_O2; 0.141, 0.562, tmp_cumulo+PL_cumulo+in terval_yield+RS+Feed_Vol+RS_cumulo+Cell_conc._interval+RQ_i ntegral+Arg_conc.+emission_CO2+interval_O2; 0.141, 0.562, t mp+PL_cumulo+interval_yield+Feed_Vol+RS_cumulo+Cell_conc._i nterval+rab_integral+Arg_conc.+emission_O2+consum_O2+genera te_CO2; 0.120, 0.562, tmp_cumulo+pH+PL+PL_cumulo+AN+Feed_Vo l+RS_cumulo+Cell_conc._interval+RQ_integral+Arg_conc.+emiss ion_CO2+interval_O2; 0.141, 0.562, tmp_cumulo+pH_cumulo+PL_ cumulo+AN_cumulo+RS_cumulo+Cell_conc._interval+rab_integral +Arg_conc.+emission_O2+emission_CO2+interval_O2; 0.160, 0.5 62, tmp_cumulo+PL_cumulo+interval_yield+Feed_Vol+RS_cumulo+ Cell_conc._interval+rab_integral+RQ_integral+Arg_conc.+inte rval_O2; 0.120, 0.562, tmp_cumulo+pH+PL_cumulo+interval_yie ld+RS+Feed_Vol+RS_cumulo+Cell_conc._interval+RQ_integral+Ar g_conc.+emission_CO2+interval_O2; 0.179, 0.562, tmp+pH_cumu lo+PL_cumulo+RS_cumulo+Cell_conc._interval+RQ_integral+Arg_ conc.+emission_CO2+interval_O2; 0.120, 0.562, tmp_cumulo+pH _cumulo+PL_cumulo+AN _cumulo+RS+RS_cumulo+Cell_conc._interva l+rab_integral+Arg_conc.+emission_CO2+interval_O2+generate-CO2; 0.120, 0.562, tmp_cumulo+PL_cumulo+AN_cumulo+RS+Feed_V ol+RS_cumulo+Cell_conc._interval+rab_integral+Arg_conc.+emi ssion_O2+interval_O2+generate_CO2; 0.120, 0.562, tmp+PL_cum ulo+AN_cumulo+RS+Feed_Vol+RS_cumulo+Cell_conc._interval+RQ_ integral+Arg_conc.+emission_O2+interval_O2+generate_CO2; 0. 120, 0.562, tmp+PL_cumulo+AN_cumulo+RS+Feed_Vol+RS_cumulo+C ell_conc._interval+Arg_conc.+emission_O2+emission_CO2+consu m_O2+generate_CO2; 0.160, 0.562, tmp_cumulo+pH+PL_cumulo+RS _cumulo+Cell_conc._interval+Arg_conc.+emission_O2+emission_ CO2+consum_O2+interval_O2; 0.141, 0.562, tmp_cumulo+PL+PL_c umulo+Feed_Vol+RS_cumulo+Cell_conc._interval+rab_integral+A rg_conc.+emission_O2+interval_O2+generate_CO2; 0.160, 0.562, tmp_cumulo+pH+PL_cumulo+AN+RS_cumulo+Cell_conc._interval+r ab_integral+Arg_conc.+emission_O2+interval_O2; 0.120, 0.562, tmp_cumulo+pH+PL_cumulo+RS+Feed_Vol+RS_cumulo+Cell_conc._i nterval+rab_integral+Arg_conc.+emission_02+consum_O2+interv al_O2; 0.141, 0.562, tmp+PL+PL_cumulo+Feed_Vol+RS_cumulo+Ce ll_conc._interval+RQ_integral+Arg_conc.+emission_O2+emissio n_CO2+interval_O2; 0.141, 0.562, tmp_cumulo+PL_cumulo+AN_cu mulo+Feed_Vol+RS_cumulo+Cell_conc._interval+RQ_integral+Arg _conc.+emission_O2+consum_O2+generate_CO2; 0.179, 0.562, tm p_cumulo+pH+PL_cumulo+RS_cumulo+Cell_conc._interval+Arg_con c.+emission_CO2+consum_O2+interval_O2; 0.120, 0.562, tmp+tm p_cumulo+pH+PL+PL_cumulo+AN_cumulo+RS_cumulo+Cell_conc._int erval+rab_integral+Arg_conc.+emission_CO2+interval_O2; 0.14 1, 0.562, tmp_cumulo+PL_cumulo+RS+Feed_Vol+RS_cumulo+Cell_c onc._interval+RQ_integral+Arg_conc.+emission_O2+interval_O2 +generate_CO2; 0.179, 0.562, tmp_cumulo+pH+PL_cumulo+RS+RS_ cumulo+Cell_conc._interval+Arg_conc.+emission_CO2+interval_ O2; 0.120, 0.562, tmp_cumulo+PL+PL_cumulo+AN_cumulo+Feed_Vo l+RS_cumulo+Cell_conc._interval+Arg_conc.+emission_O2+emiss ion_CO2+interval_O2+generate_CO2; 0.141, 0.562, tmp_cumulo+ pH_cumulo+PL_cumulo+AN_cumulo+RS_cumulo+Cell_conc._interval +rab_integral+RQ_integral+Arg_conc.+emission_O2+interval_O 2; 0.120, 0.562, tmp+tmp_cumulo+PL_cumulo+AN_cumulo+Feed_Vo l+RS_cumulo+Cell_conc._interval+Arg_conc.+emission_O2+emiss ion_CO2+interval_O2+generate_CO2; 0.120, 0.562, tmp_cumulo+ pH+pH_cumulo+PL_cumulo+RS+Feed_Vol+RS_cumulo+Cell_conc._int erval+rab_integral+Arg_conc.+emission_CO2+interval_O2; 0.16 0, 0.562, tmp_cumulo+pH+PL_cumulo+Feed_Vol+RS_cumulo+Cell_c onc._interval+Arg_conc.+emission_CO2+interval_O2+generate_C 02; 0.141, 0.562, tmp+tmp_cumulo+pH+PL_cumulo+RS_cumulo+Cel l_conc._interval+rab_integral+Arg_conc.+emission_CO2+consum _O2+interval_O2; 0.160, 0.562, tmp_cumulo+pH_cumulo+PL_cumu lo+RS+RS_cumulo+Cell_conc._interval+rab_integral+Arg_conc.+ emission_O2+interval_O2; 0.140, 0.562, tmp_cumulo+pH+pH_cum ulo+PL_cumulo+AN+RS_cumulo+Cell_conc._interval+rab_integral +Arg_conc.+emission_CO2+interval_O2; 0.120, 0.562, tmp+tmp_ cumulo+pH_cumulo+PL_cumulo+AN_cumulo+Feed_Vol+RS_cumulo+Cel l_conc._interval+Arg_conc.+emission_O2+interval_O2+generate _CO2; 0.140, 0.562, tmp+pH+PL+PL_cumulo+Feed_Vol+RS_cumulo+ Cell_conc._interval+RQ_integral+Arg_conc.+emission_CO2+inte rval_O2; 0.120, 0.562, tmp+pH_cumulo+PL_cumulo+AN_cumulo+Fe ed_Vol+RS_cumulo+Cell_conc._interval+RQ_integral+Arg_conc.+ emission_O2+consum_O2+interval_O2; 0.160, 0.562, tmp+pH_cum ulo+PL_cumulo+RS_cumulo+Cell_conc._cumulo+Cell_conc._interv al+rab_integral+Arg_conc.+emission_CO2+interval_O2; 0.179, 0.562, tmp_cumulo+pH+PL_cumulo+RS_cumulo+Cell_conc._cumulo+ Cell_conc._interval+Arg_conc.+emission_O2+interval_O2; 0.14 0, 0.562, tmp_cumulo+pH_cumulo+PL_cumulo+RS_cumulo+Cell_con c._cumulo+Cell_conc._interval+rab_integral+Arg_conc.+emissi on_CO2+interval_O2+generate_CO2; 0.140, 0.562, tmp+pH_cumul o+PL_cumulo+RS+Feed_Vol+Cell_conc._interval+Arg_conc.+emiss ion_O2+emission_CO2+consum_O2+generate_CO2; 0.120, 0.562, t mp_cumulo+pH+PL_cumulo+AN_cumulo+OD+Feed_Vol+RS_cumulo+Cell _conc._interval+RQ_integral+Arg_conc.+emission_CO2+interval _O2; 0.098, 0.562, tmp_cumulo+pH+PL_cumulo+AN_cumulo+interv al_yield+Feed_Vol+RS_cumulo+Cell_conc._interval+Arg_conc.+e mission_O2+emission_CO2+interval_O2+generate_CO2; 0.140, 0. 562, tmp_cumulo+PL_cumulo+interval_yield+Feed_Vol+RS_cumulo +Cell_conc._interval+RQ_integral+Arg_conc.+emission_O2+cons um_02+interval_02; 0.120, 0.562, tmp_cumulo+pH_cumulo+PL_cu mulo+AN_cumulo+RS+RS_cumulo+Cell_conc._interval+rab_integra l+Arg_conc.+emission_CO2+consum_O2+interval_O2; 0.120, 0.56 2, tmp_cumulo+pH_cumulo+PL_cumulo+interval_yield+Feed_Vol+R S_cumulo+Cell_conc._interval+rab_integral+Arg_conc.+emissio n_O2+consum_O2+interval_O2; 0.120, 0.562, tmp_cumulo+pH+pH_ cumulo+PL+PL_cumulo+AN_cumulo+RS_cumulo+Cell_conc._interval +rab_integral+Arg_conc.+emission_CO2+interval_O2

### [210. Linear Model that Predicts Arginine Product ion Amount in Interval 10]

0.577, 0.814, pH+PL_cumulo+AN+AN_cumulo+Feed_Vol+RS_cumulo+ rab_integral+RQ_integral+Arg_conc.+emission_O2+emission_CO 2; 0.586, 0.814, pH+AN+AN_cumulo+Feed_Vol+RS_cumulo+rab_int egral+RQ_integral+Arg_conc.+emission_O2+emission_CO2; 0.566, 0.814, pH+PL+PL_cumulo+AN+AN_cumulo+Feed_Vol+RS_cumulo+rab _integral+RQ_integral+Arg_conc.+emission_O2+emission_CO2; 0. 575, 0.813, pH+PL+AN+AN_cumulo+Feed_Vol+RS_cumulo+rab_integ ral+RQ_integral+Arg_conc.+emission_O2+emission_CO2; 0.547, 0.810, pH+PL+PL_cumulo+AN+AN_cumulo+Feed_Vol+RS_cumulo+rab_ integral+RQ_integral+Arg_conc.+emission_O2+emission_CO2+con sum_O2; 0.557, 0.810, pH+PL_cumulo+AN+AN_cumulo+Feed_Vol+RS _cumulo+rab_integral+RQ_integral+Accum._Yield+Arg_conc.+emi ssion_O2+emission_CO2; 0.567, 0.809, pH+AN+AN_cumulo+Feed_V ol+RS_cumulo+rab_integral+RQ_integral+Accum._Yield+Arg_conc. +emission_O2+emission_CO2; 0.545, 0.809, pH+PL+PL_cumulo+AN +AN_cumulo+interval_yield+Feed_Vol+RS_cumulo+rab_integral+R Q_integral+Arg_conc.+emission_O2+emission_CO2; 0.566, 0.809, tmp_cumulo+pH+AN+AN_cumulo+Feed_Vol+RS_cumulo+rab_integral +RQ_integral+Arg_conc.+emission_O2+emission_CO2; 0.556, 0.8 09, tmp_cumulo+pH+PL+AN+AN_cumulo+Feed_Vol+RS_cumulo+rab_in tegral+RQ_integral+Arg_conc.+emission_O2+emission_CO2; 0.57 4, 0.808, pH+PL+AN+Feed_Vol+RS_cumulo+rab_integral+RQ_integ ral+Arg_conc.+emission_O2+emission_CO2; 0.543, 0.808, pH+PL +PL_cumulo+AN+AN_cumulo+Feed_Vol+RS_cumulo+rab_integral+RQ_ integral+Arg_conc.+emission_O2+emission_CO2+interval_O2; 0. 563, 0.808, tmp+pH+AN+AN_cumulo+Feed_Vol+RS_cumulo+rab_inte gral+RQ_integral+Arg_conc.+emission_O2+emission_CO2; 0.553, 0.808, tmp+pH+PL+AN+AN_cumulo+Feed_Vol+RS_cumulo+rab_integ ral+RQ_integral+Arg_conc.+emission_O2+emission_CO2; 0.563, 0.808, pH+PL+PL_cumulo+AN+Feed_Vol+RS_cumulo+rab_integral+R Q_integral+Arg_conc.+emission_O2+emission_CO2; 0.542, 0.808, tmp+pH+PL+PL_cumulo+AN+AN_cumulo+Feed_Vol+RS_cumulo+rab_in tegral+RQ_integral+Arg_conc.+emission_O2+emission_CO2; 0.55 3, 0.808, pH+PL_cumulo+AN+AN_cumulo+interval_yield+Feed_Vol +RS_cumulo+rab_integral+RQ_integral+Arg_conc.+emission_O2+e mission_CO2; 0.582, 0.807, pH+AN+Feed_Vol+RS_cumulo+rab_int egral+RQ_integral+Arg_conc.+emission_O2+emission_CO2; 0.541, 0.807, tmp_cumulo+pH+PL+PL_cumulo+AN+AN_cumulo+Feed_Vol+RS _cumulo+rab_integral+RQ_integral+Arg_conc.+emission_O2+emis sion_CO2; 0.562, 0.807, pH+AN+AN_cumulo+Feed_Vol+RS_cumulo+ rab_integral+RQ_integral+Arg_conc.+emission_O2+emission_CO2 +interval_O2; 0.529, 0.807, pH+PL+PL_cumulo+AN+AN_cumulo+in terval_yield+Feed_Vol+RS_cumulo+rab_integral+RQ_integral+Ar g_conc.+emission_O2+emission_CO2+interval_O2; 0.562, 0.807, pH+AN+AN_cumulo+Feed_Vol+RS_cumulo+rab_integral+RQ_integra l+Arg_conc.+emission_O2+emission_CO2+generate_CO2; 0.551, 0. 807, tmp+pH+PL_cumulo+AN+AN_cumulo+Feed_Vol+RS_cumulo+rab_i ntegral+RQ_integral+Arg_conc.+emission_O2+emission_CO2; 0.5 51, 0.807, pH+PL+AN+AN_cumulo+Feed_Vol+RS_cumulo+rab_integr al+RQ_integral+Arg_conc.+emission_O2+emission_CO2+interval_ O2; 0.551, 0.807, tmp_cumulo+pH+PL_cumulo+AN+AN_cumulo+Feed _Vol+RS_cumulo+rab_integral+RQ_integral+Arg_conc.+emission_ O2+emission_CO2; 0.528, 0.806, pH+PL+PL_cumulo+AN+AN_cumulo +interval_yield+Feed_Vol+RS_cumulo+rab_integral+RQ_integral +Arg_conc.+emission_O2+emission_CO2+consum_O2; 0.550, 0.806, pH+PL+AN+AN_cumulo+Feed_Vol+RS_cumulo+rab_integral+RQ_inte gral+Accum._Yield+Arg_conc.+emission_O2+emission_CO2; 0.550, 0.806, pH+PL_cumulo+AN+AN_cumulo+Feed_Vol+RS_cumulo+rab_in tegral+RQ_integral+Arg_conc.+emission_O2+emission_CO2+gener ate_CO2; 0.550, 0.806, pH+PL+AN+AN_cumulo+interval_yield+Fe ed_Vol+RS_cumulo+rab_integral+RQ_integral+Arg_conc.+emissio n_O2+emission_CO2; 0.550, 0.806, pH+PL_cumulo+AN+AN_cumulo+ Feed_Vol+RS_cumulo+rab_integral+RQ_integral+Arg_conc.+emiss ion_O2+emission_CO2+interval_O2; 0.549, 0.806, pH+PL+PL_cum ulo+AN+Feed_Vol+RS_cumulo+rab_integral+RQ_integral+Arg-conc. +emission_O2+emission_CO2+consum_O2; 0.549, 0.806, pH+PL+AN +AN_cumulo+Feed_Vol+RS_cumulo+rab_integral+RQ_integral+Arg_ conc.+emission_O2+emission_CO2+generate_CO2; 0.549, 0.806, pH+PL+PL_cumulo+AN+interval_yield+Feed_Vol+RS_cumulo+rab_in tegral+RQ_integral+Arg_conc.+emission_O2+emission_CO2; 0.56 0, 0.806, pH+AN+AN_cumulo+interval_yield+Feed_Vol+RS_cumulo +rab_integral+RQ_integral+Arg_conc.+emission_O2+emission_CO 2; 0.538, 0.806, pH+PL+PL_cumulo+AN+AN_cumulo+Feed_Vol+RS_c umulo+rab_integral+RQ_integral+Arg_conc.+emission_O2+emissi on-CO2+generate-CO2; 0.560, 0.806, pH+AN+AN_cumulo+Feed_Vol +RS_cumulo+Cell_conc._interval+rab_integral+RQ_integral+Arg _conc.+emission_O2+emission_CO2; 0.526, 0.806, tmp+pH+PL+PL _cumulo+AN+interval_yield+Feed_Vol+RS_cumulo+rab_integral+R Q_integral+Arg_conc.+emission_O2+emission_CO2+consum_O2; 0. 526, 0.806, tmp_cumulo+pH+PL+PL_cumulo+AN+interval_yield+Fe ed_Vol+RS_cumulo+rab_integral+RQ_integral+Arg_conc.+emissio n_O2+emission_CO2+consum_O2; 0.549, 0.806, pH+PL_cumulo+AN+ AN_cumulo+Feed_Vol+RS_cumulo+Cell_conc._cumulo+rab_integral +RQ_integral+Arg_conc.+emission_O2+emission_CO2; 0.548, 0.8 05, pH+PL_cumulo+AN+AN_cumulo+Feed_Vol+RS_cumulo+rab_integr al+RQ_integral+Arg_conc.+emission_O2+emission_CO2+consum_O 2; 0.537, 0.805, pH+PL+PL_cumulo+AN+AN_cumulo+Feed_Vol+RS_c umulo+rab_integral+RQ_integral+Accum._Yield+Arg_conc.+emiss ion_O2+emission_CO2; 0.569, 0.805, pH+PL_cumulo+AN+Feed_Vol +RS_cumulo+rab_integral+RQ_integral+Arg_conc.+emission_O2+e mission_CO2; 0.537, 0.805, pH+PL_cumulo+AN+AN_cumulo+interv al_yield+Feed_Vol+RS_cumulo+rab_integral+RQ_integral+Accum. _Yield+Arg_conc.+emission_O2+emission_CO2; 0.537, 0.805, pH +PL+PL_cumulo+AN+AN_cumulo+Feed_Vol+RS_cumulo+Cell_conc._cu mulo+rab_integral+RQ_integral+Arg_conc.+emission_O2+emissio n_CO2; 0.537, 0.805, pH+PL+PL_cumulo+AN+interval_yield+Feed _Vol+RS_cumulo+rab_integral+RQ_integral+Arg_conc.+emission_ O2+emission_CO2+consum_O2; 0.548, 0.805, pH+AN+interval_yie ld+Feed_Vol+RS_cumulo+Cell_conc._interval+rab_integral+RQ_i ntegral+Accum._Yield+Arg_conc.+emission_O2+emission_CO2; 0. 536, 0.805, pH+AN+AN_cumulo+interval_yield+Feed_Vol+RS_cumu lo+Cell_conc._interval+rab_integral+RQ_integral+Accum._Yiel d+Arg_conc.+emission_O2+emission_CO2; 0.547, 0.805, pH+PL+A N+AN_cumulo+Feed_Vol+RS_cumulo+Cell_conc._cumulo+rab_integr al+RQ_integral+Arg_conc.+emission_O2+emission_CO2; 0.558, 0. 805, pH_cumulo+PL_cumulo+AN+AN_cumulo+Feed_Vol+RS_cumulo+ra b_integral+RQ_integral+Arg_conc.+emission_O2+emission-CO2; 0.524, 0.805, tmp_cumulo+pH+PL+PL_cumulo+AN+AN_cumulo+Feed_ Vol+RS_cumulo+rab_integral+RQ_integral+Arg_conc.+emission_O 2+emission_CO2+consum_O2; 0.512, 0.805, tmp_cumulo+pH+PL+PL _cumulo+AN+AN_cumulo+interval_yield+Feed_Vol+RS_cumulo+rab_ integral+RQ_integral+Arg_conc.+emission_O2+emission_CO2+int erval_O2; 0.524, 0.805, tmp+pH+PL+PL_cumulo+AN+AN_cumulo+Fe ed_Vol+RS_cumulo+rab_integral+RQ_integral+Arg_conc.+emissio n_02+emission_CO2+consum_O2; 0.512, 0.805, tmp_cumulo+pH+PL +PL_cumulo+AN+AN_cumulo+interval_yield+Feed_Vol+RS_cumulo+r ab_integral+RQ_integral+Arg_conc.+emission_O2+emission_CO2+ consum_O2; 0.546, 0.804, pH+pH_cumulo+PL+AN+AN_cumulo+Feed_ Vol+RS_cumulo+rab_integral+RQ_integral+Arg_conc.+emission_O 2+emission_CO2; 0.535, 0.804, pH+pH_cumulo+PL+PL_cumulo+AN+ AN_cumulo+Feed_Vol+RS_cumulo+rab_integral+RQ_integral+Arg_c onc.+emission_O2+emission_CO2; 0.535, 0.804, tmp+tmp_cumulo +pH+PL+AN+AN_cumulo+Feed_Vol+RS_cumulo+rab_integral+RQ_inte gral+Arg_conc.+emission_O2+emission_CO2; 0.556, 0.804, pH+A N+AN_cumulo+Feed_Vol+RS_cumulo+Cell_conc._cumulo+rab_integr al+RQ_integral+Arg_conc.+emission_O2+emission_CO2; 0.546, 0. 804, tmp_cumulo+pH+AN+AN_cumulo+Feed_Vol+RS_cumulo+rab_inte gral+RQ_integral+Accum._Yield+Arg_conc.+emission_O2+emissio n_CO2; 0.556, 0.804, pH+PL+AN+interval_yield+Feed_Vol+RS_cu mulo+rab_integral+RQ_integral+Arg_conc.+emission_O2+emissio n_CO2; 0.556, 0.804, pH+pH_cumulo+AN+AN_cumulo+Feed_Vol+RS_ cumulo+rab_integral+RQ_integral+Arg_conc.+emission_O2+emiss ion_CO2; 0.555, 0.804, pH+AN+interval_yield+Feed_Vol+RS_cum ulo+Cell_conc._interval+rab_integral+RQ_integral+Arg_conc.+ emission_O2+emission_CO2; 0.544, 0.803, pH+pH_cumulo+PL_cum ulo+AN+AN_cumulo+Feed_Vol+RS_cumulo+rab_integral+RQ_integra l+Arg_conc.+emission_O2+emission_CO2; 0.555, 0.803, tmp_cum ulo+pH+PL+AN+Feed_Vol+RS_cumulo+rab_integral+RQ_integral+Ar g_conc.+emission_O2+emission_CO2; 0.544, 0.803, pH+PL_cumul o+AN+AN_cumulo+Feed_Vol+RS_cumulo+Cell_conc._interval+rab_i ntegral+RQ_integral+Arg_conc.+emission_O2+emission_CO2; 0.5 44, 0.803, tmp+tmp_cumulo+pH+AN+AN_cumulo+Feed_Vol+RS_cumul o+rab_integral+RQ_integral+Arg_conc.+emission_O2+emission_C O2; 0.544, 0.803, pH+PL+AN+interval_yield+Feed_Vol+RS_cumul o+Cell_conc._interval+rab_integral+RQ_integral+Arg_conc.+em ission_O2+emission_CO2; 0.521, 0.803, tmp+pH+PL+PL_cumulo+A N+AN_cumulo+interval_yield+Feed_Vol+RS_cumulo+rab_integral+ RQ_integral+Arg_conc.+emission_O2+emission_CO2; 0.555, 0.80 3, pH+AN+AN_cumulo+Feed_Vol+RS_cumulo+rab_integral+RQ_integ ral+Arg_conc.+emission_O2+emission_CO2+consum_O2; 0.544, 0. 803, pH+PL+AN+AN_cumulo+Feed_Vol+RS_cumulo+rab_integral+RQ_ integral+Arg_conc.+emission_O2+emission_CO2+consum_O2; 0.52 1, 0.803, tmp_cumulo+pH+PL+PL_cumulo+AN+AN_cumulo+interval_ yield+Feed_Vol+RS_cumulo+rab_integral+RQ_integral+Arg-conc. +emission_O2+emission_CO2; 0.521, 0.803, pH+PL+PL_cumulo+AN +AN_cumulo+interval_yield+Feed_Vol+RS_cumulo+rab_integral+R Q_integral+Accum._Yield+Arg_conc.+emission_O2+emission_CO2; 0.574, 0.803, pH+AN_cumulo+Feed_Vol+RS_cumulo+rab_integral +RQ_integral+Arg_conc.+emission_O2+emission_CO2; 0.508, 0.8 03, tmp+pH+PL+PL_cumulo+AN+AN_cumulo+interval_yield+Feed_Vo l+RS_cumulo+rab_integral+RQ_integral+Arg_conc.+emission_O2+ emission_CO2+consum_O2; 0.508, 0.803, tmp+pH+PL+PL_cumulo+A N+AN_cumulo+interval_yield+Feed_Vol+RS_cumulo+rab_integral+ RQ_integral+Arg_conc.+emission_O2+emission_CO2+interval_O2; 0.544, 0.803, pH+AN+AN_cumulo+Feed_Vol+RS_cumulo+rab_integ ral+RQ_integral+Accum._Yield+Arg_conc.+emission_O2+emission _CO2+generate_CO2; 0.532, 0.803, tmp_cumulo+pH+PL+AN+AN_cum ulo+interval_yield+Feed_Vol+RS_cumulo+rab_integral+RQ_integ ral+Arg_conc.+emission_O2+emission_CO2; 0.532, 0.803, pH+PL +AN+interval_yield+Feed_Vol+RS_cumulo+Cell_conc._interval+r ab_integral+RQ_integral+Accum._Yield+Arg_conc.+emission_O2+ emission_CO2; 0.543, 0.803, pH+PL+AN+AN_cumulo+Feed_Vol+RS_ cumulo+Cell_conc._interval+rab_integral+RQ_integral+Arg_con c.+emission_O2+emission_CO2; 0.543, 0.803, pH+AN+AN_cumulo+ interval_yield+Feed_Vol+RS_cumulo+rab_integral+RQ_integral+ Accum._Yield+Arg_conc.+emission_O2+emission_CO2; 0.553, 0.8 03, tmp+pH+PL+AN+Feed_Vol+RS_cumulo+rab_integral+RQ_integra l+Arg_conc.+emission_O2+emission_CO2; 0.543, 0.803, tmp+pH+ AN+AN_cumulo+Feed_Vol+RS_cumulo+rab_integral+RQ_integral+Ac cum._Yield+Arg_conc.+emission_O2+emission_CO2; 0.532, 0.803, tmp+pH+PL+PL_cumulo+AN+Feed_Vol+RS_cumulo+rab_integral+RQ_ integral+Arg_conc.+emission_O2+emission_CO2+consum_O2; 0.52 0, 0.803, pH+PL+PL_cumulo+AN+AN_cumulo+Feed_Vol+RS_cumulo+C ell_conc._cumulo+rab_integral+RQ_integral+Arg_conc.+emissio n_02+emission_CO2+consum_O2; 0.563, 0.803, pH+PL_cumulo+AN_ cumulo+Feed_Vol+RS_cumulo+rab_integral+RQ_integral+Arg_conc. +emission_O2+emission_CO2; 0.519, 0.802, pH+PL_cumulo+AN+AN _cumulo+interval_yield+Feed_Vol+RS_cumulo+Cell_conc._interv al+rab_integral+RQ_integral+Accum._Yield+Arg_conc.+emission _O2+emission_CO2; 0.531, 0.802, pH+PL_cumulo+AN+AN_cumulo+F eed_Vol+RS_cumulo+rab_integral+RQ_integral+Accum._Yield+Arg _conc.+emission_O2+emission_CO2+generate_CO2; 0.542, 0.802, pH+AN+AN_cumulo+Feed_Vol+RS_cumulo+Cell_conc._interval+rab _integral+RQ_integral+Accum._Yield+Arg_conc.+emission_O2+em ission_CO2; 0.542, 0.802, pH+AN+AN_cumulo+interval_yield+Fe ed_Vol+RS_cumulo+Cell_conc._interval+rab_integral+RQ_integr al+Arg_conc.+emission_O2+emission_CO2; 0.518, 0.802, pH+PL+ PL_cumulo+AN+AN_cumulo+Feed_Vol+RS_cumulo+rab_integral+RQ_i ntegral+Arg_conc.+emission_O2+emission_CO2+consum_O2+genera te_CO2; 0.541, 0.802, pH+AN+AN_cumulo+Feed_Vol+RS_cumulo+ra b_integral+RQ_integral+Accum._Yield+Arg_conc.+emission_O2+e mission_CO2+interval_O2; 0.541, 0.802, tmp+pH+PL+PL_cumulo+ AN+Feed_Vol+RS_cumulo+rab_integral+RQ_integral+Arg_conc.+em ission_O2+emission_CO2; 0.562, 0.802, pH+AN+interval_yield+ Feed_Vol+RS_cumulo+rab_integral+RQ_integral+Arg_conc.+emiss ion_O2+emission_CO2; 0.562, 0.802, tmp_cumulo+pH+AN+Feed_Vo l+RS_cumulo+rab_integral+RQ_integral+Arg_conc.+emission_O2+ emission_CO2; 0.562, 0.802, pH+AN+Feed_Vol+RS_cumulo+Cell_c onc._interval+rab_integral+RQ_integral+Arg_conc.+emission_O 2+emission_CO2; 0.518, 0.802, tmp_cumulo+pH+PL+PL_cumulo+AN +AN_cumulo+Feed_Vol+RS_cumulo+rab_integral+RQ_integral+Arg_ conc.+emission_O2+emission_CO2+interval_O2; 0.530, 0.802, p H+PL+PL_cumulo+AN+interval_yield+Feed_Vol+RS_cumulo+rab_int egral+RQ_integral+Arg_conc.+emission_O2+emission_CO2+interv al_O2; 0.530, 0.802, tmp+pH+PL+PL_cumulo+AN+interval_yield+ Feed_Vol+RS_cumulo+rab_integral+RQ_integral+Arg_conc.+emiss ion_O2+emission_CO2; 0.541, 0.802, tmp_cumulo+pH+PL+AN+inte rval_yield+Feed_Vol+RS_cumulo+rab_integral+RQ_integral+Arg_ conc.+emission_O2+emission_CO2; 0.518, 0.802, pH+pH_cumulo+ PL+PL_cumulo+AN+AN_cumulo+interval_yield+Feed_Vol+RS_cumulo +rab_integral+RQ_integral+Arg_conc.+emission_O2+emission_CO 2; 0.540, 0.802, tmp_cumulo+pH+AN+AN_cumulo+interval_yield+ Feed_Vol+RS_cumulo+rab_integral+RQ_integral+Arg_conc.+emiss ion_O2+emission_CO2; 0.551, 0.802, pH+PL+AN+Feed_Vol+RS_cum ulo+rab_integral+RQ_integral+Accum._Yield+Arg_conc.+emissio n_O2+emission_CO2; 0.561, 0.802, pH+AN+Feed_Vol+RS_cumulo+r ab_integral+RQ_integral+Accum._Yield+Arg_conc.+emission_O2+ emission_CO2; 0.529, 0.802, tmp_cumulo+pH+PL+AN+AN_cumulo+F eed_Vol+RS_cumulo+rab_integral+RQ_integral+Accum._Yield+Arg _conc.+emission_O2+emission_CO2; 0.529, 0.801, pH+PL_cumulo +AN+AN_cumulo+interval_yield+Feed_Vol+RS_cumulo+rab_integra l+RQ_integral+Arg_conc.+emission_O2+emission_CO2+interval_O 2; 0.529, 0.801, tmp+pH+PL_cumulo+AN+AN_cumulo+Feed_Vol+RS_ cumulo+rab_integral+RQ_integral+Accum._Yield+Arg_conc.+emis sion_O2+emission_CO2; 0.517, 0.801, tmp+pH+PL+PL_cumulo+AN+ interval_yield+Feed_Vol+RS_cumulo+rab_integral+RQ_integral+ Arg_conc.+emission_O2+emission_CO2+interval_O2; 0.551, 0.80 1, pH+PL+AN+Feed_Vol+RS_cumulo+rab_integral+RQ_integral+Arg _conc.+emission_O2+emission_CO2+interval_02; 0.551, 0.801, pH+PL_cumulo+AN+interval_yield+Feed_Vol+RS_cumulo+rab_integ ral+RQ_integral+Arg_conc.+emission_O2+emission_CO2; 0.505, 0.801, pH+PL+PL_cumulo+AN+AN_cumulo+interval_yield+Feed_Vol +RS_cumulo+rab_integral+RQ_integral+Accum._Yield+Arg_conc.+ emission_O2+emission_CO2+consum_O2; 0.529, 0.801, tmp_cumul o+pH+PL_cumulo+AN+AN_cumulo+Feed_Vol+RS_cumulo+rab_integral +RQ_integral+Accum._Yield+Arg_conc.+emission_O2+emission_CO 2; 0.517, 0.801, tmp+pH+PL+PL_cumulo+AN+AN_cumulo+Feed_Vol+ RS_cumulo+rab_integral+RQ_integral+Arg_conc.+emission_O2+em ission_CO2+interval_O2; 0.529, 0.801, tmp_cumulo+pH+PL+AN+A N_cumulo+Feed_Vol+RS_cumulo+rab_integral+RQ_integral+Arg_co nc.+emission_O2+emission_CO2+interval_O2; 0.540, 0.801, pH_ cumulo+PL_cumulo+AN+AN_cumulo+interval_yield+Feed_Vol+RS_cu mulo+rab_integral+RQ_integral+Arg_conc.+emission_O2+emissio n_CO2; 0.517, 0.801, pH_cumulo+PL_cumulo+AN+AN_cumulo+inter val_yield+Feed_Vol+RS_cumulo+Cell_conc._interval+rab_integr al+RQ_integral+Accum._Yield+Arg_conc.+emission_O2+emission_ CO2; 0.517, 0.801, pH+pH_cumulo+PL+PL_cumulo+AN+AN_cumulo+F eed_Vol+RS_cumulo+rab_integral+RQ_integral+Arg_conc.+emissi on_O2+emission_CO2+consum_O2; 0.504, 0.801, tmp+pH+PL+PL_cu mulo+AN+interval_yield+Feed_Vol+RS_cumulo+rab_integral+RQ_i ntegral+Accum._Yield+Arg_conc.+emission_O2+emission_CO2+con sum_O2; 0.560, 0.801, tmp+pH+AN+Feed_Vol+RS_cumulo+rab_inte gral+RQ_integral+Arg_conc.+emission_O2+emission_CO2; 0.516, 0.801, pH+PL+PL_cumulo+AN+AN_cumulo+Feed_Vol+RS_cumulo+rab _integral+RQ_integral+Arg_conc.+emission_O2+emission_CO2+in terval_O2+generate_CO2; 0.528, 0.801, pH_cumulo+PL+PL_cumul o+AN+AN_cumulo+interval_yield+Feed_Vol+RS_cumulo+rab_integr al+RQ_integral+Arg_conc.+emission_O2+emission_CO2; 0.504, 0. 801, pH+PL+PL_cumulo+AN+AN_cumulo+interval_yield+Feed_Vol+R S_cumulo+rab_integral+RQ_integral+Accum._Yield+Arg_conc.+em ission_O2+emission_CO2+interval_O2; 0.528, 0.801, pH+PL+PL_ cumulo+AN+AN_cumulo+Feed_Vol+RS_cumulo+Cell_conc._interval+ rab_integral+RQ_integral+Arg_conc.+emission_O2+emission_CO 2; 0.528, 0.801, pH+PL_cumulo+AN+AN_cumulo+Feed_Vol+RS_cumu lo+rab_integral+RQ_integral+Accum._Yield+Arg_conc.+emission _O2+emission_CO2+interval_O2; 0.516, 0.801, tmp_cumulo+pH+P L+PL_cumulo+AN+interval_yield+Feed_Vol+RS_cumulo+rab_integr al+RQ_integral+Arg_conc.+emission_O2+emission_CO2+interval-02; 0.516, 0.801, pH+PL+PL_cumulo+AN+interval_yield+Feed_Vo l+RS_cumulo+rab_integral+RQ_integral+Accum._Yield+Arg_conc. +emission_O2+emission_CO2+consum_O2; 0.528, 0.801, tmp+pH+P L+AN+AN_cumulo+interval_yield+Feed_Vol+RS_cumulo+rab_integr al+RQ_integral+Arg_conc.+emission_O2+emission_CO2; 0.539, 0. 801, tmp_cumulo+pH+AN+AN_cumulo+Feed_Vol+RS_cumulo+rab_inte gral+RQ_integral+Arg_conc.+emission_O2+emission_CO2+generat e_CO2; 0.550, 0.801, pH+PL+AN+Feed_Vol+RS_cumulo+rab_integr al+RQ_integral+Arg_conc.+emission_O2+emission_CO2+generate-CO2; 0.539, 0.801, pH_cumulo+PL+PL_cumulo+AN+AN_cumulo+Feed _Vol+RS_cumulo+rab_integral+RQ_integral+Arg_conc.+emission_ O2+emission_CO2; 0.528, 0.801, pH+PL_cumulo+AN+AN_cumulo+Fe ed_Vol+RS_cumulo+rab_integral+RQ_integral+Accum._Yield+Arg_ conc.+emission_O2+emission_CO2+consum_O2; 0.549, 0.801, pH+ PL+AN+Feed_Vol+RS_cumulo+Cell_conc._interval+rab_integral+R Q_integral+Arg_conc.+emission_O2+emission_CO2; 0.539, 0.801, tmp_cumulo+pH+AN+AN_cumulo+Feed_Vol+RS_cumulo+rab_integral +RQ_integral+Arg_conc.+emission_O2+emission_CO2+interval_O 2; 0.527, 0.801, tmp_cumulo+pH+PL+PL_cumulo+AN+Feed_Vol+RS_ cumulo+rab_integral+RQ_integral+Arg_conc.+emission_O2+emiss ion_CO2+consum_O2; 0.516, 0.801, pH+PL+PL_cumulo+AN+AN_cumu lo+Feed_Vol+RS_cumulo+rab_integral+RQ_integral+Arg_conc.+em ission_O2+emission_CO2+consum_O2+interval_O2; 0.527, 0.801, pH+PL+AN+AN_cumulo+interval_yield+Feed_Vol+RS_cumulo+rab_i ntegral+RQ_integral+Accum._Yield+Arg_conc.+emission_02+emis sion_CO2; 0.515, 0.801, pH+PL+PL_cumulo+AN+AN_cumulo+Feed_V ol+RS_cumulo+rab_integral+RQ_integral+Accum._Yield+Arg_conc. +emission_O2+emission_CO2+consum_O2; 0.527, 0.801, pH+PL_cu mulo+AN+interval_yield+Feed_Vol+RS_cumulo+Cell_conc._interv al+rab_integral+RQ_integral+Accum._Yield+Arg_conc.+emission _O2+emission_CO2; 0.559, 0.801, pH+AN+Feed_Vol+RS_cumulo+ra b_integral+RQ_integral+Arg_conc.+emission_O2+emission-CO2+i nterval_O2; 0.527, 0.801, tmp+pH+PL+AN+AN_cumulo+Feed_Vol+R S_cumulo+rab_integral+RQ_integral+Arg_conc.+emission_O2+emi ssion_CO2+interval_O2; 0.538, 0.801, pH+PL+PL_cumulo+AN+Fee d_Vol+RS_cumulo+rab_integral+RQ_integral+Accum._Yield+Arg_c onc.+emission_O2+emission_CO2; 0.549, 0.800, pH+PL_cumulo+A N_cumulo+Feed_Vol+RS_cumulo+rab_integral+RQ_integral+Accum. _Yield+Arg_conc.+emission_O2+emission_CO2; 0.527, 0.800, pH +PL+PL_cumulo+AN+interval_yield+Feed_Vol+RS_cumulo+rab_inte gral+RQ_integral+Accum._Yield+Arg_conc.+emission_O2+emissio n_CO2; 0.503, 0.800, tmp_cumulo+pH+PL+PL_cumulo+AN+interval _yield+Feed_Vol+RS_cumulo+rab_integral+RQ_integral+Accum._Y ield+Arg_conc.+emission_O2+emission_CO2+consum_O2; 0.527, 0. 800, tmp_cumulo+pH+PL_cumulo+AN+AN_cumulo+interval_yield+Fe ed_Vol+RS_cumulo+rab_integral+RQ_integral+Arg_conc.+emissio n_O2+emission_CO2; 0.527, 0.800, tmp+pH+PL_cumulo+AN+AN_cum ulo+interval_yield+Feed_Vol+RS_cumulo+rab_integral+RQ_integ ral+Arg_conc.+emission_O2+emission_CO2; 0.527, 0.800, pH+PL +PL_cumulo+AN+interval_yield+Feed_Vol+RS_cumulo+Cell_conc._ interval+rab_integral+RQ_integral+Arg_conc.+emission_O2+emi ssion_CO2; 0.527, 0.800, tmp_cumulo+pH+PL+PL_cumulo+AN+inte rval_yield+Feed_Vol+RS_cumulo+rab_integral+RQ_integral+Arg_ conc.+emission_O2+emission_CO2; 0.559, 0.800, pH+AN_cumulo+ Feed_Vol+RS_cumulo+rab_integral+RQ_integral+Accum._Yield+Ar g_conc.+emission_O2+emission_CO2; 0.526, 0.800, tmp+pH+PL+A N+AN_cumulo+Feed_Vol+RS_cumulo+rab_integral+RQ_integral+Acc um._Yield+Arg_conc.+emission_O2+emission_CO2; 0.526, 0.800, tmp_cumulo+pH+PL+AN+AN_cumulo+Feed_Vol+RS_cumulo+rab_integ ral+RQ_integral+Arg_conc.+emission_O2+emission_CO2+generate _CO2; 0.537, 0.800, tmp+pH+AN+AN_cumulo+Feed_Vol+RS_cumulo+ rab_integral+RQ_integral+Arg_conc.+emission_O2+emission_CO2 +interval_O2; 0.537, 0.800, tmp+pH+AN+AN_cumulo+Feed_Vol+RS _cumulo+rab_integral+RQ_integral+Arg_conc.+emission_O2+emis sion_CO2+generate_CO2; 0.558, 0.800, pH+AN+Feed_Vol+RS_cumu lo+rab_integral+RQ_integral+Arg_conc.+emission_O2+emission-CO2+generate_CO2; 0.526, 0.800, pH+PL+AN+AN_cumulo+interval _yield+Feed_Vol+RS_cumulo+Cell_conc._interval+rab_integral+ RQ_integral+Arg_conc.+emission_O2+emission_CO2; 0.526, 0.80 0, pH+PL_cumulo+AN+AN_cumulo+interval_yield+Feed_Vol+RS_cum ulo+Cell_conc._interval+rab_integral+RQ_integral+Arg_conc.+ emission_O2+emission_CO2; 0.514, 0.800, pH+PL_cumulo+AN+AN_ cumulo+interval_yield+Feed_Vol+RS_cumulo+rab_integral+RQ_in tegral+Accum._Yield+Arg_conc.+emission_O2+emission_CO2+inte rval_O2; 0.537, 0.800, tmp_cumulo+pH+AN+AN_cumulo+Feed_Vol+ RS_cumulo+Cell_conc._interval+rab_integral+RQ_integral+Arg_ conc.+emission_O2+emission_CO2; 0.537, 0.800, tmp+pH+AN+AN_ cumulo+interval_yield+Feed_Vol+RS_cumulo+rab_integral+RQ_in tegral+Arg_conc.+emission_O2+emission_CO2; 0.537, 0.800, pH +PL+PL_cumulo+AN+Feed_Vol+RS_cumulo+rab_integral+RQ_integra l+Arg_conc.+emission_O2+emission_CO2+interval_O2; 0.537, 0. 800, tmp_cumulo+pH+PL+PL_cumulo+AN+Feed_Vol+RS_cumulo+rab_i ntegral+RQ_integral+Arg_conc.+emission_O2+emission_CO2; 0.5 01, 0.800, pH+pH_cumulo+PL+PL_cumulo+AN+AN_cumulo+interval_ yield+Feed_Vol+RS_cumulo+rab_integral+RQ_integral+Arg_conc. +emission_O2+emission_CO2+interval_O2; 0.537, 0.800, tmp+pH +PL+AN+interval_yield+Feed_Vol+RS_cumulo+rab_integral+RQ_in tegral+Arg_conc.+emission_O2+emission_CO2; 0.525, 0.800, tm p+pH+AN+interval_yield+Feed_Vol+RS_cumulo+Cell_conc._interv al+rab_integral+RQ_integral+Accum._Yield+Arg_conc.+emission _O2+emission_CO2; 0.525, 0.800, tmp+pH+PL+AN+AN_cumulo+Feed _Vol+RS_cumulo+rab_integral+RQ_integral+Arg_conc.+emission_ O2+emission_CO2+generate_CO2; 0.547, 0.800, tmp+tmp_cumulo+ pH+AN_cumulo+Feed_Vol+RS_cumulo+rab_integral+RQ_integral+Ar g_conc.+emission_O2+emission_CO2; 0.513, 0.800, pH+PL+PL_cu mulo+AN+AN_cumulo+Feed_Vol+RS_cumulo+Cell_conc._cumulo+rab_ integral+RQ_integral+Arg_conc.+emission_O2+emission_CO2+int erval_O2; 0.513, 0.800, pH+PL+PL_cumulo+AN+interval_yield+F eed_Vol+RS_cumulo+Cell_conc._interval+rab_integral+RQ_integ ral+Accum._Yield+Arg_conc.+emission_O2+emission_CO2; 0.536, 0.800, pH+AN+AN_cumulo+Feed_Vol+RS_cumulo+Cell_conc._inter val+rab_integral+RQ_integral+Arg_conc.+emission_O2+emission _CO2+generate_CO2; 0.547, 0.800, pH+PL+AN+Feed_Vol+RS_cumul o+Cell_conc._cumulo+rab_integral+RQ_integral+Arg_conc.+emis sion_O2+emission_CO2; 0.525, 0.800, pH+PL+AN+AN_cumulo+Feed _Vol+RS_cumulo+rab_integral+RQ_integral+Accum._Yield+Arg_co nc.+emission_O2+emission_CO2+generate_CO2; 0.513, 0.799, pH +PL+PL_cumulo+AN+AN_cumulo+interval_yield+Feed_Vol+RS_cumul o+rab_integral+RQ_integral+Arg_conc.+emission_O2+emission_C O2+generate_CO2; 0.557, 0.799, tmp+pH+AN_cumulo+Feed_Vol+RS _cumulo+rab_integral+RQ_integral+Arg_conc.+emission_O2+emis sion_CO2; 0.547, 0.799, pH+PL+AN+Feed_Vol+RS_cumulo+rab_int egral+RQ_integral+Arg_conc.+emission_O2+emission_CO2+consum _O2; 0.513, 0.799, tmp_cumulo+pH+AN+AN_cumulo+interval_yiel d+Feed_Vol+RS_cumulo+Cell_conc._interval+rab_integral+RQ_in tegral+Accum._Yield+Arg_conc.+emission_O2+emission_CO2; 0.5 46, 0.799, pH+PL_cumulo+AN+Feed_Vol+RS_cumulo+rab_integral+ RQ_integral+Accum._Yield+Arg_conc.+emission_O2+emission_CO 2; 0.536, 0.799, pH+PL+PL_cumulo+AN+Feed_Vol+RS_cumulo+rab_ integral+RQ_integral+Arg_conc.+emission_O2+emission_CO2+gen erate_CO2; 0.546, 0.799, pH+pH_cumulo+PL+AN+Feed_Vol+RS_cum ulo+rab_integral+RQ_integral+Arg_conc.+emission_O2+emission _CO2; 0.512, 0.799, tmp+tmp_cumulo+pH+PL+PL_cumulo+AN+AN_cu mulo+Feed_Vol+RS_cumulo+rab_integral+RQ_integral+Arg_conc.+ emission_O2+emission_CO2; 0.524, 0.799, tmp_cumulo+pH+pH_cu mulo+PL+AN+AN_cumulo+Feed_Vol+RS_cumulo+rab_integral+RQ_int egral+Arg_conc.+emission_O2+emission_CO2; 0.524, 0.799, tmp +pH+PL+AN+interval_yield+Feed_Vol+RS_cumulo+Cell_conc._inte rval+rab_integral+RQ_integral+Arg_conc.+emission_O2+emissio n_CO2; 0.535, 0.799, tmp+pH+AN+AN_cumulo+Feed_Vol+RS_cumulo +Cell_conc._interval+rab_integral+RQ_integral+Arg_conc.+emi ssion_O2+emission_CO2; 0.535, 0.799, pH+pH_cumulo+AN+AN_cum ulo+Feed_Vol+RS_cumulo+rab_integral+RQ_integral+Accum._Yiel d+Arg_conc.+emission_O2+emission_CO2; 0.512, 0.799, pH+PL+P L_cumulo+AN+AN_cumulo+interval_yield+Feed_Vol+RS_cumulo+Cel l_conc._interval+rab_integral+RQ_integral+Arg_conc.+emissio n_O2+emission_CO2; 0.512, 0.799, tmp+tmp_cumulo+pH+PL+AN+AN _cumulo+interval_yield+Feed_Vol+RS_cumulo+rab_integral+RQ_i ntegral+Arg_conc.+emission_O2+emission_CO2; 0.499, 0.799, p H+PL+PL_cumulo+AN+AN_cumulo+interval_yield+Feed_Vol+RS_cumu lo+rab_integral+RQ_integral+Arg_conc.+emission_O2+emission_ CO2+consum_O2+generate_CO2; 0.524, 0.799, pH+PL+AN+AN_cumul o+interval_yield+Feed_Vol+RS_cumulo+rab_integral+RQ_integra l+Arg_conc.+emission_O2+emission_CO2+interval_O2; 0.524, 0. 799, pH+PL+PL_cumulo+AN+Feed_Vol+RS_cumulo+rab_integral+RQ_ integral+Accum._Yield+Arg_conc.+emission_O2+emission_CO2+co nsum_O2; 0.524, 0.799, pH+PL+AN+AN_cumulo+Feed_Vol+RS_cumul o+rab_integral+RQ_integral+Accum._Yield+Arg_conc.+emission_ O2+emission_CO2+interval_O2; 0.524, 0.799, pH+PL_cumulo+AN+ AN_cumulo+interval_yield+Feed_Vol+RS_cumulo+rab_integral+RQ _integral+Arg_conc.+emission_O2+emission_CO2+generate-CO2; 0.556, 0.799, pH_cumulo+AN+AN_cumulo+Feed_Vol+RS_cumulo+rab _integral+RQ_integral+Arg_conc.+emission_O2+emission_CO2; 0. 523, 0.799, tmp_cumulo+pH+AN+interval_yield+Feed_Vol+RS_cum ulo+Cell_conc._interval+rab_integral+RQ_integral+Accum._Yie Id+Arg_conc.+emission_O2+emission_CO2; 0.523, 0.799, pH+PL_ cumulo+AN+AN_cumulo+Feed_Vol+RS_cumulo+Cell_conc._interval+ rab_integral+RQ_integral+Accum._Yield+Arg_conc.+emission_O2 +emission_CO2; 0.511, 0.799, tmp+pH+AN+AN_cumulo+interval_y ield+Feed_Vol+RS_cumulo+Cell_conc._interval+rab_integral+RQ _integral+Accum._Yield+Arg_conc.+emission_O2+emission-CO2; 0.523, 0.799, pH+PL_cumulo+AN+AN_cumulo+interval_yield+Feed _Vol+RS_cumulo+rab_integral+RQ_integral+Arg_conc.+emission_ O2+emission_CO2+consum_O2; 0.523, 0.799, tmp_cumulo+pH+PL+A N+interval_yield+Feed_Vol+RS_cumulo+Cell_conc._interval+rab _integral+RQ_integral+Arg_conc.+emission_O2+emission_CO2; 0. 535, 0.799, pH+PL_cumulo+AN+interval_yield+Feed_Vol+RS_cumu lo+Cell_conc._interval+rab_integral+RQ_integral+Arg_conc.+e mission_O2+emission_CO2; 0.511, 0.799, pH+AN+AN_cumulo+inte rval_yield+Feed_Vol+RS_cumulo+Cell_conc._interval+rab_integ ral+RQ_integral+Accum._Yield+Arg_conc.+emission_O2+emission _CO2+generate_CO2; 0.499, 0.799, tmp+tmp_cumulo+pH+PL+PL_cu mulo+AN+interval_yield+Feed_Vol+RS_cumulo+rab_integral+RQ_i ntegral+Arg_conc.+emission_O2+emission_CO2+consum_O2; 0.485, 0.799, tmp_cumulo+pH+PL+PL_cumulo+AN+AN_cumulo+interval_yi eld+Feed_Vol+RS_cumulo+rab_integral+RQ_integral+Accum._Yiel d+Arg_conc.+emission_O2+emission_CO2+consum _O2; 0.556, 0.79 9, tmp_cumulo+pH+AN_cumulo+Feed_Vol+RS_cumulo+rab_integral+ RQ_integral+Arg_conc.+emission_O2+emission_CO2; 0.511, 0.79 9, pH_cumulo+PL+PL_cumulo+AN+AN_cumulo+interval_yield+Feed_ Vol+RS_cumulo+rab_integral+RQ_integral+Arg_conc.+emission_O 2+emission_CO2+interval_O2; 0.511, 0.799, pH+PL+AN+AN_cumul o+interval_yield+Feed_Vol+RS_cumulo+Cell_conc._interval+rab _integral+RQ_integral+Accum._Yield+Arg_conc.+emission-O2+em ission_CO2; 0.534, 0.799, pH+AN+AN_cumulo+Feed_Vol+RS_cumul o+Cell_conc._interval+rab_integral+RQ_integral+Arg_conc.+em ission_O2+emission_CO2+interval_O2; 0.534, 0.799, tmp+pH+AN +interval_yield+Feed_Vol+RS_cumulo+Cell_conc._interval+rab_ integral+RQ_integral+Arg_conc.+emission_O2+emission_CO2; 0. 523, 0.799, pH+pH_cumulo+PL_cumulo+AN+AN_cumulo+interval_yi eld+Feed_Vol+RS_cumulo+rab_integral+RQ_integral+Arg_conc.+e mission_O2+emission_CO2; 0.523, 0.799, tmp_cumulo+pH+PL+AN+ AN_cumulo+Feed_Vol+RS_cumulo+Cell_conc._cumulo+rab_integral +RQ_integral+Arg_conc.+emission_O2+emission_CO2; 0.523, 0.7 99, tmp_cumulo+pH+PL+AN+AN_cumulo+Feed_Vol+RS_cumulo+rab_in tegral+RQ_integral+Arg_conc.+emission_O2+emission_CO2+consu m_O2; 0.498, 0.799, pH+pH_cumulo+PL+PL_cumulo+AN+AN_cumulo+ interval_yield+Feed_Vol+RS_cumulo+rab_integral+RQ_integral+ Arg_conc.+emission_O2+emission_CO2+consum_O2; 0.523, 0.799, pH+AN+interval_yield+Feed_Vol+RS_cumulo+Cell_conc._interva l+rab_integral+RQ_integral+Accum._Yield+Arg_conc.+emission_ O2+emission_CO2+interval_O2; 0.511, 0.799, pH+pH_cumulo+PL+ PL_cumulo+AN+AN_cumulo+Feed_Vol+RS_cumulo+rab_integral+RQ_i ntegral+Arg_conc.+emission_O2+emission_CO2+interval_O2; 0.5 11, 0.799, tmp+pH+PL+PL_cumulo+AN+AN_cumulo+Feed_Vol+RS_cum ulo+rab_integral+RQ_integral+Accum._Yield+Arg_conc.+emissio n_O2+emission_CO2; 0.511, 0.798, pH+AN+AN_cumulo+interval_y ield+Feed_Vol+RS_cumulo+Cell_conc._interval+rab_integral+RQ _integral+Accum._Yield+Arg_conc.+emission_O2+emission-CO2+i nterval_O2; 0.534, 0.798, pH+PL+AN+interval_yield+Feed_Vol+ RS_cumulo+rab_integral+RQ_integral+Accum._Yield+Arg_conc.+e mission_O2+emission_CO2; 0.534, 0.798, pH+AN+AN_cumulo+Feed _Vol+RS_cumulo+rab_integral+RQ_integral+Arg_conc.+emission_ O2+emission_CO2+consum_O2+interval_O2; 0.545, 0.798, tmp+pH +PL_cumulo+AN+Feed_Vol+RS_cumulo+rab_integral+RQ_integral+A rg_conc.+emission_O2+emission_CO2; 0.534, 0.798, pH+AN+AN_c umulo+interval_yield+Feed_Vol+RS_cumulo+rab_integral+RQ_int egral+Arg_conc.+emission_O2+emission_CO2+interval_O2; 0.522, 0.798, pH+pH_cumulo+PL+AN+AN_cumulo+Feed_Vol+RS_cumulo+rab _integral+RQ_integral+Arg_conc.+emission_O2+emission_CO2+in terval_O2; 0.534, 0.798, pH_cumulo+PL_cumulo+AN+AN_cumulo+F eed_Vol+RS_cumulo+rab_integral+RQ_integral+Accum._Yield+Arg _conc.+emission_O2+emission_CO2; 0.522, 0.798, tmp_cumulo+p H+PL_cumulo+AN+AN_cumulo+Feed_Vol+RS_cumulo+rab_integral+RQ _integral+Arg_conc.+emission_O2+emission_CO2+interval-O2; 0. 534, 0.798, tmp_cumulo+pH_cumulo+PL_cumulo+AN+AN_cumulo+Fee d_Vol+RS_cumulo+rab_integral+RQ_integral+Arg_conc.+emission _O2+emission_CO2; 0.510, 0.798, tmp+pH+PL+PL_cumulo+AN+AN_c umulo+Feed_Vol+RS_cumulo+rab_integral+RQ_integral+Arg_conc. +emission_O2+emission_CO2+generate_CO2; 0.522, 0.798, pH_cu mulo+PL_cumulo+AN+AN_cumulo+interval_yield+Feed_Vol+RS_cumu lo+Cell_conc._interval+rab_integral+RQ_integral+Arg_conc.+e mission_O2+emission_CO2; 0.534, 0.798, pH+AN+AN_cumulo+Feed _Vol+RS_cumulo+rab_integral+RQ_integral+Accum._Yield+Arg_co nc.+emission_O2+emission_CO2+consum _O2; 0.544, 0.798, tmp_c umulo+pH+AN+interval_yield+Feed_Vol+RS_cumulo+rab_integral+ RQ_integral+Arg_conc.+emission_O2+emission_CO2; 0.522, 0.79 8, pH_cumulo+PL_cumulo+AN+AN_cumulo+interval_yield+Feed_Vol +RS_cumulo+rab_integral+RQ_integral+Accum._Yield+Arg_conc.+ emission_O2+emission_CO2; 0.522, 0.798, tmp_cumulo+pH+AN+AN _cumulo+interval_yield+Feed_Vol+RS_cumulo+rab_integral+RQ_i ntegral+Accum._Yield+Arg_conc.+emission_O2+emission_CO2; 0. 510, 0.798, pH+PL+PL_cumulo+AN+AN_cumulo+Feed_Vol+RS_cumulo +Cell_conc._interval+rab_integral+RQ_integral+Arg_conc.+emi ssion_O2+emission_CO2+consum_O2; 0.510, 0.798, tmp+pH+PL+AN +interval_yield+Feed_Vol+RS_cumulo+Cell_conc._interval+rab_ integral+RQ_integral+Accum._Yield+Arg_conc.+emission_O2+emi ssion_CO2; 0.522, 0.798, pH+AN+interval_yield+Feed_Vol+RS_c umulo+Cell_conc._interval+rab_integral+RQ_integral+Accum._Y ield+Arg_conc.+emission_O2+emission_CO2+generate_CO2; 0.522, 0.798, pH+PL+AN+AN_cumulo+Feed_Vol+RS_cumulo+Cell_conc._cu mulo+rab_integral+RQ_integral+Arg_conc.+emission_O2+emissio n_CO2+interval_O2; 0.510, 0.798, pH+PL+PL_cumulo+AN+AN_cumu lo+Feed_Vol+RS_cumulo+rab_integral+RQ_integral+Accum._Yield +Arg_conc.+emission_O2+emission_CO2+interval_O2; 0.522, 0.7 98, tmp+tmp_cumulo+pH+AN+AN_cumulo+Feed_Vol+RS_cumulo+rab_i ntegral+RQ_integral+Accum._Yield+Arg_conc.+emission_O2+emis sion_CO2; 0.533, 0.798, pH+AN+AN_cumulo+Feed_Vol+RS_cumulo+ rab_integral+RQ_integral+Arg_conc.+emission_O2+emission_CO2 +interval_O2+generate_CO2; 0.510, 0.798, tmp_cumulo+pH+PL+P L_cumulo+AN+AN_cumulo+Feed_Vol+RS_cumulo+rab_integral+RQ_in tegral+Accum._Yield+Arg_conc.+emission_O2+emission_CO2 0.5 10, 0.798, pH+PL+PL_cumulo+AN+interval_yield+Feed_Vol+RS_cu mulo+Cell_conc._interval+rab_integral+RQ_integral+Arg_conc. +emission_O2+emission_CO2+consum_O2; 0.533, 0.798, tmp_cumu lo+pH+AN+interval_yield+Feed_Vol+RS_cumulo+Cell_conc._inter val+rab_integral+RQ_integral+Arg_conc.+emission_O2+emission _CO2; 0.522, 0.798, pH+pH_cumulo+PL+AN+AN_cumulo+interval_y ield+Feed_Vol+RS_cumulo+rab_integral+RQ_integral+Arg_conc.+ emission_O2+emission_CO2; 0.497, 0.798, tmp+pH+PL+PL_cumulo +AN+interval_yield+Feed_Vol+RS_cumulo+Cell_conc._interval+r ab_integral+RQ_integral+Arg_conc.+emission_O2+emission_CO2+ consum_O2; 0.544, 0.798, pH+PL_cumulo+AN_cumulo+Feed_Vol+RS _cumulo+Cell_conc._cumulo+rab_integral+RQ_integral+Arg_conc. +emission_O2+emission_CO2; 0.521, 0.798, pH+PL_cumulo+AN+AN _cumulo+Feed_Vol+RS_cumulo+rab_integral+RQ_integral+Arg_con c.+emission_O2+emission_CO2+consum_O2+generate_CO2; 0.521, 0.798, pH+PL_cumulo+AN+AN_cumulo+Feed_Vol+RS_cumulo+rab_int egral+RQ_integral+Arg_conc.+emission_O2+emission_CO2+consum _O2+interval_O2; 0.533, 0.798, tmp+pH_cumulo+PL_cumulo+AN+A N_cumulo+Feed_Vol+RS_cumulo+rab_integral+RQ_integral+Arg_co nc.+emission_O2+emission_CO2; 0.521, 0.798, pH+PL+AN+AN_cum ulo+Feed_Vol+RS_cumulo+rab_integral+RQ_integral+Arg_conc.+e mission_O2+emission_CO2+consum_O2+interval_02; 0.484, 0.798, tmp+pH+PL+PL_cumulo+AN+AN_cumulo+interval_yield+Feed_Vol+R S_cumulo+rab_integral+RQ_integral+Accum._Yield+Arg_conc.+em ission_O2+emission_CO2+consum_O2; 0.521, 0.798, tmp+pH+PL+A N+AN_cumulo+Feed_Vol+RS_cumulo+Cell_conc._cumulo+rab_integr al+RQ_integral+Arg_conc.+emission_O2+emission_CO2; 0.533, 0. 798, pH+AN+interval_yield+RS+Feed_Vol+Cell_conc._interval+r ab_integral+RQ_integral+Accum._Yield+Arg_conc.+emission_O2+ emission_CO2; 0.509, 0.798, pH+PL+PL_cumulo+AN+AN_cumulo+Fe ed_Vol+RS_cumulo+rab_integral+RQ_integral+Accum._Yield+Arg_ conc.+emission_O2+emission_CO2+generate_CO2; 0.521, 0.798, tmp+pH+PL_cumulo+AN+AN_cumulo+Feed_Vol+RS_cumulo+rab_integr al+RQ_integral+Arg_conc.+emission_O2+emission_CO2+generate_ CO2; 0.543, 0.798, pH+AN+Feed_Vol+RS_cumulo+Cell_conc._inte rval+rab_integral+RQ_integral+Accum._Yield+Arg_conc.+emissi on_O2+emission_CO2; 0.533, 0.798, tmp_cumulo+pH+pH_cumulo+A N+AN_cumulo+Feed_Vol+RS_cumulo+rab_integral+RQ_integral+Arg _conc.+emission_O2+emission_CO2; 0.509, 0.798, pH+PL_cumulo +AN+AN_cumulo+interval_yield+Feed_Vol+RS_cumulo+rab_integra 1+RQ_integral+Accum._Yield+Arg_conc.+emission_O2+emission_C O2+consum_O2; 0.521, 0.798, tmp_cumulo+pH+PL_cumulo+AN+AN_c umulo+Feed_Vol+RS_cumulo+rab_integral+RQ_integral+Arg-conc. +emission_O2+emission_CO2+consum_O2; 0.509, 0.798, tmp_cumu lo+pH+PL_cumulo+AN+AN_cumulo+interval_yield+Feed_Vol+RS_cum ulo+rab_integral+RQ_integral+Accum._Yield+Arg_conc.+emissio n_O2+emission_CO2; 0.521, 0.798, pH+pH_cumulo+PL_cumulo+AN+ AN_cumulo+Feed_Vol+RS_cumulo+rab_integral+RQ_integral+Accum. _Yield+Arg_conc.+emission_O2+emission_CO2; 0.509, 0.798, tm p+pH+PL_cumulo+AN+AN_cumulo+interval_yield+Feed_Vol+RS_cumu lo+rab_integral+RQ_integral+Accum._Yield+Arg_conc.+emission _O2+emission_CO2; 0.532, 0.798, pH+AN+AN_cumulo+interval_yi eld+Feed_Vol+RS_cumulo+rab_integral+RQ_integral+Arg_conc.+e mission_O2+emission_CO2+generate_CO2; 0.521, 0.798, pH+PL_c umulo+AN+AN_cumulo+Feed_Vol+RS_cumulo+rab_integral+RQ_integ ral+Arg_conc.+emission_O2+emission_CO2+interval_O2+generate _CO2; 0.521, 0.798, tmp_cumulo+pH+PL+AN+AN_cumulo+Feed_Vol+ RS_cumulo+Cell_conc._interval+rab_integral+RQ_integral+Arg_ conc.+emission_O2+emission_CO2; 0.521, 0.798, pH_cumulo+AN+ AN_cumulo+interval_yield+Feed_Vol+RS_cumulo+Cell_conc._inte rval+rab_integral+RQ_integral+Accum._Yield+Arg_conc.+emissi on_O2+emission_CO2; 0.521, 0.798, pH+PL_cumulo+AN+AN_cumulo +Feed_Vol+RS_cumulo+Cell_conc._cumulo+rab_integral+RQ_integ ral+Accum._Yield+Arg_conc.+emission_O2+emission_CO2; 0.496, 0.798, pH+PL+PL_cumulo+AN+AN_cumulo+interval_yield+Feed_Vo 1+RS_cumulo+rab_integral+RQ_integral+Arg_conc.+emission_02+ emission_CO2+consum_O2+interval_O2; 0.496, 0.798, pH+PL+PL_ cumulo+AN+interval_yield+Feed_Vol+RS_cumulo+Cell_conc._inte rval+rab_integral+RQ_integral+Accum._Yield+Arg_conc.+emissi on_O2+emission_CO2+consum_O2; 0.532, 0.798, pH+AN+AN_cumulo +Feed_Vol+RS_cumulo+Cell_conc._cumulo+rab_integral+RQ_integ ral+Accum._Yield+Arg_conc.+emission_O2+emission_CO2; 0.532, 0.798, pH+PL_cumulo+AN_cumulo+interval_yield+Feed_Vol+RS_c umulo+rab_integral+RQ_integral+Accum._Yield+Arg_conc.+emiss ion_O2+emission_CO2; 0.483, 0.798, tmp_cumulo+pH+PL+PL_cumu lo+AN+AN_cumulo+interval_yield+Feed_Vol+RS_cumulo+rab_integ ral+RQ_integral+Accum._Yield+Arg_conc.+emission_O2+emission CO2+interval_O2; 0.521, 0.798, pH+PL_cumulo+AN+AN_cumulo+F eed_Vol+RS_cumulo+Cell_conc._cumulo+rab_integral+RQ_integra 1+Arg_conc.+emission_O2+emission_CO2+interval_O2; 0.521, 0. 797, tmp+pH+PL_cumulo+AN+AN_cumulo+Feed_Vol+RS_cumulo+rab_i ntegral+RQ_integral+Arg_conc.+emission_O2+emission_CO2+inte rval_O2; 0.509, 0.797, tmp+tmp_cumulo+pH+PL+AN+AN_cumulo+Fe ed_Vol+RS_cumulo+rab_integral+RQ_integral+Arg_conc.+emissio n_O2+emission_CO2+interval_O2; 0.532, 0.797, tmp_cumulo+pH+ AN+AN_cumulo+Feed_Vol+RS_cumulo+rab_integral+RQ_integral+Ar g_conc.+emission_O2+emission_CO2+consum_O2; 0.521, 0.797, t mp+pH+pH_cumulo+PL+AN+AN_cumulo+Feed_Vol+RS_cumulo+rab_inte gral+RQ_integral+Arg_conc.+emission_O2+emission_CO2; 0.532, 0.797, pH+pH_cumulo+AN+AN_cumulo+Feed_Vol+RS_cumulo+rab_in tegral+RQ_integral+Arg_conc.+emission_O2+emission_CO2+inter val_O2; 0.508, 0.797, tmp_cumulo+pH+PL+AN+interval_yield+Fe ed_Vol+RS_cumulo+Cell_conc._interval+rab_integral+RQ_integr al+Accum._Yield+Arg_conc.+emission_O2+emission_CO2; 0.520, 0.797, pH+PL+AN+AN_cumulo+Feed_Vol+RS_cumulo+rab_integral+R Q_integral+Arg_conc.+emission_O2+emission_CO2+interval_O2+g enerate_CO2; 0.508, 0.797, pH+PL_cumulo+AN+AN_cumulo+interv al_yield+Feed_Vol+RS_cumulo+rab_integral+RQ_integral+Accum. _Yield+Arg_conc.+emission_O2+eniission_CO2+generate_CO2; 0.5 43, 0.797, pH+AN+interval_yield+Feed_Vol+RS_cumulo+rab_inte gral+RQ_integral+Accum._Yield+Arg_conc.+emission_O2+emissio n_CO2; 0.520, 0.797, pH+PL_cumulo+AN+AN_cumulo+Feed_Vol+RS_ cumulo+Cell_conc._cumulo+rab_integral+RQ_integral+Arg_conc. +emission_O2+emission_CO2+generate_CO2; 0.532, 0.797, tmp_c umulo+pH+AN+AN_cumulo+Feed_Vol+RS_cumulo+Cell_conc._cumulo+ rab_integral+RQ_integral+Arg_conc.+emission_O2+emission_CO 2; 0.520, 0.797, tmp+tmp_cumulo+pH+PL_cumulo+AN+AN_cumulo+F eed_Vol+RS_cumulo+rab_integral+RQ_integral+Arg_conc.+emissi on_O2+emission_CO2; 0.543, 0.797, pH+PL_cumulo+AN+Feed_Vol+ RS_cumulo+rab_integral+RQ_integral+Arg_conc.+emission_O2+em ission_CO2+generate_CO2; 0.532, 0.797, pH+AN+AN_cumulo+Feed _Vol+RS_cumulo+rab_integral+RQ_integral+Arg_conc.+emission_ O2+emission_CO2+consum_O2+generate_CO2; 0.532, 0.797, pH+PL _cumulo+AN+interval_yield+Feed_Vol+RS_cumulo+rab_integral+R Q_integral+Accum._Yield+Arg_conc.+emission_O2+emission_CO2; 0.542, 0.797, pH+PL_cumulo+AN+Feed_Vol+RS_cumulo+rab_integ ral+RQ_integral+Arg_conc.+emission_O2+emission_CO2+consum_O 2; 0.482, 0.797, tmp+tmp_cumulo+pH+PL+PL_cumulo+AN+AN_cumul o+interval_yield+Feed_Vol+RS_cumulo+rab_integral+RQ_integra 1+Arg_conc.+emission_O2+emission_CO2+consum_O2; 0.496, 0.79 7, tmp+tmp_cumulo+pH+PL+PL_cumulo+AN+AN_cumulo+Feed_Vol+RS_ cumulo+rab_integral+RQ_integral+Arg_conc.+emission_O2+emiss ion_CO2+consum_O2; 0.508, 0.797, tmp_cumulo+pH+PL+AN+AN_cum ulo+interval_yield+Feed_Vol+RS_cumulo+rab_integral+RQ_integ ral+Accum._Yield+Arg_conc.+emission_O2+emission_CO2; 0.508, 0.797, pH+pH_cumulo+PL+PL_cumulo+AN+interval_yield+Feed_Vo 1+RS_cumulo+rab_integral+RQ_integral+Arg_conc.+emission_O2+ emission_CO2+consum_O2; 0.520, 0.797, tmp+pH+PL_cumulo+AN+A N_cumulo+Feed_Vol+RS_cumulo+rab_integral+RQ_integral+Arg_co nc.+emission_O2+emission_CO2+consum_O2; 0.495, 0.797, pH+PL _cumulo+AN+AN_cumulo+interval_yield+Feed_Vol+RS_cumulo+Cell _conc._interval+rab_integral+RQ_integral+Accum._Yield+Arg_c onc.+emission_O2+emission_CO2+interval_O2; 0.508, 0.797, tm p+pH+PL+PL_cumulo+AN+AN_cumulo+Feed_Vol+RS_cumulo+Cell_conc. _cumulo+rab_integral+RQ_integral+Arg_conc.+emission_O2+emis sion_CO2; 0.508, 0.797, pH+pH_cumulo+AN+AN_cumulo+interval_ yield+Feed_Vol+RS_cumulo+Cell_conc._interval+rab_integral+R Q_integral+Accum._Yield+Arg_conc.+emission_O2+emission_CO2; 0.495, 0.797, pH+PL+PL_cumulo+AN+AN_cumulo+interval_yield+ Feed_Vol+RS_cumulo+rab_integral+RQ_integral+Arg_conc.+emiss ion_O2+emission_CO2+interval_O2+generate_CO2; 0.495, 0.797, tmp+pH+PL+PL_cumulo+AN+AN_cumulo+interval_yield+Feed_Vol+R S_cumulo+rab_integral+RQ_integral+Accum._Yield+Arg_conc.+em ission_O2+emission_CO2; 0.508, 0.797, pH+PL+PL_cumulo+AN+AN _cumulo+interval_yield+Feed_Vol+RS_cumulo+Cell_conc._cumulo +rab_integral+RQ_integral+Arg_conc.+emission_O2+emission_CO 2; 0.520, 0.797, tmp_cumulo+pH+PL_cumulo+AN+AN_cumulo+Feed_ Vol+RS_cumulo+rab_integral+RQ_integral+Arg_conc.+emission_O 2+emission_CO2+generate_CO2; 0.520, 0.797, pH+PL+AN+AN_cumu lo+interval_yield+Feed_Vol+RS_cumulo+rab_integral+RQ_integr al+Arg_conc.+emission_O2+emission_CO2+generate_CO2; 0.520, 0.797, pH+PL+AN+AN_cumulo+Feed_Vol+RS_cumulo+rab_integral+R Q_integral+Arg_conc.+emission_O2+emission_CO2+consum_O2+gen erate_CO2; 0.553, 0.797, pH+AN+Feed_Vol+RS_cumulo+rab_integ ral+RQ_integral+Arg_conc.+emission_O2+emission_CO2+consum_O 2; 0.520, 0.797, pH+PL_cumulo+AN+AN_cumulo+Feed_Vol+RS_cumu lo+Cell_conc._cumulo+rab_integral+RQ_integral+Arg_conc.+emi ssion_O2+emission_CO2+consum_O2; 0.520, 0.797, pH+PL+AN+AN_ cumulo+Feed_Vol+RS_cumulo+Cell_conc._cumulo+rab_integral+RQ _integral+Arg_conc.+emission_O2+eniission_CO2+generate_CO2; 0.520, 0.797, pH+pH_cumulo+PL+AN+AN_cumulo+Feed_Vol+RS_cumu lo+rab_integral+RQ_integral+Accum._Yield+Arg_conc.+emission _O2+emission_CO2; 0.508, 0.797, tmp_cumulo+pH+PL+PL_cumulo+ AN+AN_cumulo+Feed_Vol+RS_cumulo+Cell_conc._cumulo+rab_integ ral+RQ_integral+Arg_conc.+emission_O2+emission_CO2; 0.495, 0.797, pH+PL+PL_cumulo+AN+AN_cumulo+interval_yield+Feed_Vol +RS_cumulo+Cell_conc._interval+rab_integral+RQ_integral+Arg _conc.+emission_O2+emission_CO2+interval_02; 0.520, 0.797, tmp+pH+PL+AN+AN_cumulo+Feed_Vol+RS_cumulo+rab_integral+RQ_i ntegral+Arg_conc.+emission_O2+emission_CO2+consum_O2; 0.531, 0.797, tmp+tmp_cumulo+pH+PL+AN+Feed_Vol+RS_cumulo+rab_inte gral+RQ_integral+Arg_conc.+emission_O2+emission_CO2; 0.519, 0.797, pH+pH_cumulo+PL+PL_cumulo+AN+Feed_Vol+RS_cumulo+rab _integral+RQ_integral+Arg_conc.+emission_O2+emission_CO2+co nsum_O2; 0.495, 0.797, tmp_cumulo+pH+pH_cumulo+PL+PL_cumulo +AN+interval_yield+Feed_Vol+RS_cumulo+rab_integral+RQ_integ ral+Arg_conc.+emission_O2+emission_CO2+consum_O2; 0.495, 0. 797, tmp_cumulo+pH+PL+PL_cumulo+AN+AN_cumulo+interval_yield +Feed_Vol+RS_cumulo+rab_integral+RQ_integral+Accum._Yield+A rg_conc.+emission_O2+emission_CO2; 0.519, 0.797, tmp_cumulo +pH+AN+AN_cumulo+Feed_Vol+RS_cumulo+rab_integral+RQ_integra 1+Accum._Yield+Arg_conc.+emission_O2+emission_CO2+generate_ CO2; 0.552, 0.797, pH+pH_cumulo+AN+Feed_Vol+RS_cumulo+rab_i ntegral+RQ_integral+Arg_conc.+emission_O2+emission_CO2; 0.5 19, 0.797, pH+pH_cumulo+PL+PL_cumulo+AN+interval_yield+Feed _Vol+RS_cumulo+rab_integral+RQ_integral+Arg_conc.+emission_ O2+emission_CO2; 0.507, 0.797, pH+PL+PL_cumulo+AN+AN_cumulo +Feed_Vol+RS_cumulo+Cell_conc._cumulo+rab_integral+RQ_integ ral+Arg_conc.+emission_O2+emission_CO2+generate_CO2; 0.481, 0.797, tmp+tmp_cumulo+pH+PL+PL_cumulo+AN+AN_cumulo+interva 1_yield+Feed_Vol+RS_cumulo+rab_integral+RQ_integral+Arg_con c.+emission_O2+emission_CO2+interval_O2; 0.531, 0.797, pH+P L+PL_cumulo+AN+Feed_Vol+RS_cumulo+Cell_conc._interval+rab_i ntegral+RQ_integral+Arg_conc.+emission_O2+emission_CO2; 0.5 42, 0.797, pH+AN+interval_yield+RS+Feed_Vol+Cell_conc._inte rval+rab_integral+RQ_integral+Arg_conc.+emission_O2+emissio n_CO2; 0.519, 0.797, pH+AN+AN_cumulo+Feed_Vol+RS_cumulo+Cel 1_conc._interval+rab_integral+RQ_integral+Accum._Yield+Arg_ conc.+emission_O2+emission_CO2+generate_CO2; 0.542, 0.797, pH+PL_cumulo+AN+Feed_Vol+RS_cumulo+Cell_conc._interval+rab_ integral+RQ_integral+Arg_conc.+emission_O2+emission_CO2; 0. 507, 0.797, tmp_cumulo+pH+PL+PL_cumulo+AN+AN_cumulo+Feed_Vo 1+RS_cumulo+rab_integral+RQ_integral+Arg_conc.+emission_O2+ emission_CO2+generate_CO2; 0.481, 0.797, tmp+pH+PL+PL_cumul o+AN+interval_yield+Feed_Vol+RS_cumulo+Cell_conc._interval+ rab_integral+RQ_integral+Accum._Yield+Arg_conc.+emission_O2 +emission_CO2+consum_O2; 0.519, 0.797, tmp_cumulo+pH+AN+AN_ cumulo+interval_yield+Feed_Vol+RS_cumulo+Cell_conc._interva 1+rab_integral+RQ_integral+Arg_conc.+emission_O2+emission_C 02; 0.519, 0.797, tmp_cumulo+pH+PL_cumulo+AN+AN_cumulo+Feed _Vol+RS_cumulo+Cell_conc._cumulo+rab_integral+RQ_integral+A rg_conc.+emission_O2+emission_CO2; 0.541, 0.797, tmp+pH+AN+ interval_yield+Feed_Vol+RS_cumulo+rab_integral+RQ_integral+ Arg_conc.+emission_O2+emission_CO2; 0.519, 0.797, tmp+pH+PL +AN+AN_cumulo+Feed_Vol+RS_cumulo+Cell_conc._interval+rab_in tegral+RQ_integral+Arg_conc.+emission_O2+emission_CO2; 0.54 1, 0.797, tmp_cumulo+pH+PL_cumulo+AN+Feed_Vol+RS_cumulo+rab _integral+RQ_integral+Arg_conc.+emission_O2+emission_CO2; 0. 494, 0.797, pH+PL+PL_cumulo+AN+AN_cumulo+interval_yield+Fee d_Vol+RS_cumulo+Cell_conc._cumulo+rab_integral+RQ_integral+ Arg_conc.+emission_O2+emission_CO2+consum_O2; 0.530, 0.797, pH+AN+AN_cumulo+Feed_Vol+RS_cumulo+Cell_conc._cumulo+rab_i ntegral+RQ_integral+Arg_conc.+emission_O2+emission_CO2+inte rval_O2; 0.530, 0.797, tmp_cumulo+pH+PL+AN+Feed_Vol+RS_cumu lo+rab_integral+RQ_integral+Accum._Yield+Arg_conc.+emission _O2+emission_CO2; 0.494, 0.797, tmp_cumulo+pH+PL+PL_cumulo+ AN+interval_yield+Feed_Vol+RS_cumulo+Cell_conc._interval+ra b_integral+RQ_integral+Arg_conc.+emission_O2+emission-CO2+c onsum_O2; 0.481, 0.797, tmp+pH+PL+PL_cumulo+AN+AN_cumulo+in terval_yield+Feed_Vol+RS_cumulo+rab_integral+RQ_integral+Ac cum._Yield+Arg_conc.+emission_O2+emission_CO2+interval_O2; 0.494, 0.797, tmp_cumulo+pH+PL+PL_cumulo+AN+AN_cumulo+Feed_ Vol+RS_cumulo+Cell_conc._cumulo+rab_integral+RQ_integral+Ar g_conc.+emission_O2+emission_CO2+consum_O2; 0.481, 0.797, t mp_cumulo+pH+pH_cumulo+PL+PL_cumulo+AN+AN_cumulo+interval_y ield+Feed_Vol+RS_cumulo+rab_integral+RQ_integral+Arg_conc.+ emission_O2+emission_CO2+interval_O2; 0.530, 0.797, pH+AN+A N_cumulo+Feed_Vol+RS_cumulo+Cell_conc._cumulo+rab_integral+ RQ_integral+Arg_conc.+emission_O2+emission_CO2+generate_CO 2; 0.507, 0.797, tmp+tmp_cumulo+pH+PL+AN+AN_cumulo+Feed_Vol +RS_cumulo+rab_integral+RQ_integral+Accum._Yield+Arg_conc.+ emission_O2+emission_CO2; 0.519, 0.797, pH+pH_cumulo+AN+int erval_yield+Feed_Vol+RS_cumulo+Cell_conc._interval+rab_inte gral+RQ_integral+Accum._Yield+Arg_conc.+emission_O2+emissio n_CO2; 0.519, 0.797, pH+PL+AN+AN_cumulo+interval_yield+Feed _Vol+RS_cumulo+Cell_conc._cumulo+rab_integral+RQ_integral+A rg_conc.+emission_O2+emission_CO2; 0.530, 0.797, pH+AN+AN_c umulo+Feed_Vol+RS_cumulo+Cell_conc._interval+rab_integral+R Q_integral+Arg_conc.+emission_O2+emission_CO2+consum_O2; 0. 519, 0.797, tmp+pH+PL_cumulo+AN+AN_cumulo+Feed_Vol+RS_cumul o+Cell_conc._cumulo+rab_integral+RQ_integral+Arg_conc.+emis sion_O2+emission_CO2; 0.519, 0.797, pH+PL+AN+AN_cumulo+Feed _Vol+RS_cumulo+Cell_conc._interval+rab_integral+RQ_integral +Arg_conc.+emission_O2+emission_CO2+generate_CO2; 0.506, 0. 797, pH+pH_cumulo+PL+PL_cumulo+AN+AN_cumulo+Feed_Vol+RS_cum ulo+Cell_conc._cumulo+rab_integral+RQ_integral+Arg_conc.+em ission_O2+emission_CO2; 0.506, 0.797, tmp+pH+pH_cumulo+PL+P L_cumulo+AN+AN_cumulo+Feed_Vol+RS_cumulo+rab_integral+RQ_in tegral+Arg_conc.+emission_O2+emission_CO2; 0.506, 0.796, tm p_cumulo+pH+pH_cumulo+PL+PL_cumulo+AN+AN_cumulo+Feed_Vol+RS _cumulo+rab_integral+RQ_integral+Arg_conc.+emission_O2+emis sion_CO2; 0.518, 0.796, tmp+pH+AN+AN_cumulo+interval_yield+ Feed_Vol+RS_cumulo+rab_integral+RQ_integral+Accum._Yield+Ar g_conc.+emission_O2+emission_CO2; 0.530, 0.796, pH+pH_cumul o+PL+PL_cumulo+AN+Feed_Vol+RS_cumulo+rab_integral+RQ_integr al+Arg_conc.+emission_O2+emission_CO2; 0.494, 0.796, pH+PL+ PL_cumulo+AN+AN_cumulo+interval_yield+Feed_Vol+RS_cumulo+Ce ll_conc._interval+rab_integral+RQ_integral+Arg_conc.+emissi on_O2+emission_CO2+consum_O2; 0.518, 0.796, pH+PL+AN+interv al_yield+Feed_Vol+RS_cumulo+Cell_conc._interval+rab_integra l+RQ_integral+Arg_conc.+emission_O2+emission-CO2+interval-O 2; 0.530, 0.796, tmp+pH+AN+AN_cumulo+Feed_Vol+RS_cumulo+Cel l_conc._cumulo+rab_integral+RQ_integral+Arg_conc.+emission_ O2+emission_CO2; 0.541, 0.796, tmp+pH+PL_cumulo+AN_cumulo+F eed_Vol+RS_cumulo+rab_integral+RQ_integral+Arg_conc.+emissi on_O2+emission_CO2; 0.530, 0.796, pH+pH_cumulo+AN+AN_cumulo +interval_yield+Feed_Vol+RS_cumulo+rab_integral+RQ_integral +Arg_conc.+emission_O2+emission_CO2; 0.506, 0.796, pH+PL+PL _cumulo+AN+interval_yield+Feed_Vol+RS_cumulo+rab_integral+R Q_integral+Accum._Yield+Arg_conc.+emission_O2+emission_CO2+ interval_O2; 0.494, 0.796, pH+pH_cumulo+PL_cumulo+AN+AN_cum ulo+interval_yield+Feed_Vol+RS_cumulo+Cell_conc._interval+r ab_integral+RQ_integral+Accum._Yield+Arg_conc.+emission_O2+ emission_CO2; 0.518, 0.796, tmp+tmp_cumulo+pH+AN+AN_cumulo+ interval_yield+Feed_Vol+RS_cumulo+rab_integral+RQ_integral+ Arg_conc.+emission_O2+emission_CO2; 0.518, 0.796, tmp+tmp_c umulo+pH+PL+AN+interval_yield+Feed_Vol+RS_cumulo+rab_integr al+RQ_integral+Arg_conc.+emission_O2+emission_CO2; 0.530, 0. 796, pH+AN+interval_yield+Feed_Vol+RS_cumulo+Cell_conc._int erval+rab_integral+RQ_integral+Arg_conc.+emission_O2+emissi on_CO2+interval_O2; 0.530, 0.796, tmp+pH+AN+AN_cumulo+Feed_ Vol+RS_cumulo+rab_integral+RQ_integral+Arg_conc.+emission_O 2+emission_CO2+consum_O2; 0.506, 0.796, pH+PL+AN+interval_y ield+Feed_Vol+RS_cumulo+Cell_conc._interval+rab_integral+RQ _integral+Accum._Yield+Arg_conc.+emission_O2+emission_CO2+i nterval_O2; 0.551, 0.796, pH+AN+Feed_Vol+RS_cumulo+Cell_con c._cumulo+rab_integral+RQ_integral+Arg_conc.+emission_O2+em ission_CO2; 0.518, 0.796, tmp+pH+AN+AN_cumulo+interval_yiel d+Feed_Vol+RS_cumulo+Cell_conc._interval+rab_integral+RQ_in tegral+Arg_conc.+emission_O2+emission_CO2; 0.540, 0.796, tm p+pH+AN_cumulo+Feed_Vol+RS_cumulo+rab_integral+RQ_integral+ Accum._Yield+Arg_conc.+emission_O2+emission_CO2; 0.518, 0.7 96, pH+PL+AN+AN_cumulo+Feed_Vol+RS_cumulo+Cell_conc._interv al+rab_integral+RQ_integral+Arg_conc.+emission_O2+emission_ CO2+interval_O2; 0.493, 0.796, pH+PL+PL_cumulo+AN+AN_cumulo +interval_yield+Feed_Vol+RS_cumulo+Cell_conc._cumulo+rab_in tegral+RQ_integral+Arg_conc.+emission_O2+emission_CO2+inter val_O2; 0.518, 0.796, tmp_cumulo+pH+AN+AN_cumulo+Feed_Vol+R S_cumulo+Cell_conc._interval+rab_integral+RQ_integral+Accum. _Yield+Arg_conc.+emission_O2+emission_CO2; 0.540, 0.796, pH +PL_cumulo+AN_cumulo+interval_yield+Feed_Vol+RS_cumulo+rab_ integral+RQ_integral+Arg_conc.+emission_O2+emission_CO2; 0. 529, 0.796, pH+PL+AN+interval_yield+Feed_Vol+RS_cumulo+rab_ integral+RQ_integral+Arg_conc.+emission_O2+emission_CO2+int erval_O2; 0.518, 0.796, pH+pH_cumulo+PL_cumulo+AN+AN_cumulo +Feed_Vol+RS_cumulo+Cell_conc._cumulo+rab_integral+RQ_integ ral+Arg_conc.+emission_O2+emission_CO2; 0.518, 0.796, tmp+p H+AN+AN_cumulo+Feed_Vol+RS_cumulo+rab_integral+RQ_integral+ Accum._Yield+Arg_conc.+emission_O2+emission_CO2+generate_CO 2; 0.493, 0.796, pH+PL+PL_cumulo+AN+AN_cumulo+interval_yiel d+Feed_Vol+RS_cumulo+Cell_conc._interval+rab_integral+RQ_in tegral+Accum._Yield+Arg_conc.+emission_O2+emission_CO2; 0.5 05, 0.796, tmp+pH+PL+PL_cumulo+AN+interval_yield+Feed_Vol+R S_cumulo+Cell_conc._interval+rab_integral+RQ_integral+Arg_c onc.+emission_O2+emission_CO2; 0.505, 0.796, tmp_cumulo+pH+ PL+AN+AN_cumulo+interval_yield+Feed_Vol+RS_cumulo+rab_integ ral+RQ_integral+Arg_conc.+emission_O2+emission_CO2+interval _O2; 0.517, 0.796, pH+AN+interval_yield+Feed_Vol+RS_cumulo+ Cell_conc._interval+rab_integral+RQ_integral+Accum._Yield+A rg_conc.+emission_O2+emission_CO2+consum_O2; 0.529, 0.796, pH+PL+AN+interval_yield+Feed_Vol+RS_cumulo+Cell_conc._cumul o+rab_integral+RQ_integral+Arg_conc.+emission_O2+emission_C 02; 0.517, 0.796, pH_cumulo+PL_cumulo+AN+interval_yield+Fee d_Vol+RS_cumulo+Cell_conc._interval+rab_integral+RQ_integra 1+Accum._Yield+Arg_conc.+emission_O2+emission_CO2; 0.529, 0. 796, pH+AN+interval_yield+Feed_Vol+RS_cumulo+Cell_conc._int erval+rab_integral+RQ_integral+Arg_conc.+emission_O2+emissi on_CO2+generate_CO2; 0.517, 0.796, pH+PL_cumulo+AN+AN_cumul o+interval_yield+Feed_Vol+RS_cumulo+Cell_conc._cumulo+rab_i ntegral+RQ_integral+Arg_conc.+emission_O2+emission_CO2; 0.5 05, 0.796, tmp+pH+PL+PL_cumulo+AN+interval_yield+Feed_Vol+R S_cumulo+rab_integral+RQ_integral+Accum._Yield+Arg_conc.+em ission_O2+emission_CO2; 0.493, 0.796, tmp+pH+pH_cumulo+PL+P L_cumulo+AN+interval_yield+Feed_Vol+RS_cumulo+rab_integral+ RQ_integral+Arg_conc.+emission_O2+emission_CO2+consum_O2; 0. 517, 0.796, pH+PL_cumulo+AN+AN_cumulo+Feed_Vol+RS_cumulo+Ce ll_conc._interval+rab_integral+RQ_integral+Arg_conc.+emissi on_O2+emission_CO2+generate_CO2; 0.529, 0.796, tmp+pH+PL+AN +Feed_Vol+RS_cumulo+rab_integral+RQ_integral+Accum._Yield+A rg_conc.+emission_O2+emission_CO2; 0.505, 0.796, pH+PL+PL_c umulo+AN+interval_yield+Feed_Vol+RS_cumulo+Cell_conc._inter val+rab_integral+RQ_integral+Arg_conc.+emission_O2+emission _CO2+interval_O2; 0.540, 0.796, PL_cumulo+AN_cumulo+interva l_yield+Feed_Vol+RS_cumulo+rab_integral+Accum._Yield+Arg_co nc.+emission_O2+emission_CO2+consum_O2; 0.517, 0.796, pH+pH _cumulo+PL+AN+AN_cumulo+Feed_Vol+RS_cumulo+rab_integral+RQ_ integral+Arg_conc.+emission_O2+emission_CO2+generate_CO2; 0. 505, 0.796, pH_cumulo+PL+PL_cumulo+AN+AN_cumulo+interval_yi eld+Feed_Vol+RS_cumulo+Cell_conc._interval+rab_integral+RQ_ integral+Arg_conc.+emission_O2+emission_CO2; 0.539, 0.796, tmp_cumulo+pH+AN+Feed_Vol+RS_cumulo+rab_integral+RQ_integra l+Accum._Yield+Arg_conc.+emission_O2+emission_CO2; 0.492, 0. 796, pH+PL+PL_cumulo+AN+AN_cumulo+Feed_Vol+RS_cumulo+rab_in tegral+RQ_integral+Arg_conc.+emission_O2+emission_CO2+consu m_O2+interval_O2+generate_CO2; 0.517, 0.796, tmp+tmp_cumulo +pH+AN+AN_cumulo+Feed_Vol+RS_cumulo+rab_integral+RQ_integra l+Arg_conc.+emission_O2+emission_CO2+interval_O2; 0.517, 0. 796, pH+PL+AN+AN_cumulo+Feed_Vol+RS_cumulo+Cell_conc._cumul o+rab_integral+RQ_integral+Arg_conc.+emission_O2+emission_C O2+consum_O2; 0.505, 0.796, tmp+tmp_cumulo+pH+PL+AN+AN_cumu lo+Feed_Vol+RS_cumulo+rab_integral+RQ_integral+Arg_conc.+em ission_O2+emission_CO2+consum_O2; 0.517, 0.796, pH+AN+AN_cu mulo+interval_yield+Feed_Vol+RS_cumulo+Cell_conc._interval+ rab_integral+RQ_integral+Arg_conc.+emission_O2+emission_CO2 +generate_CO2; 0.492, 0.796, tmp+pH+PL+PL_cumulo+AN+AN_cumu lo+Feed_Vol+RS_cumulo+Cell_conc._cumulo+rab_integral+RQ_int egral+Arg_conc.+emission_O2+emission_CO2+consum_O2; 0.528, 0.796, tmp+pH+PL_cumulo+AN+interval_yield+Feed_Vol+RS_cumul o+rab_integral+RQ_integral+Arg_conc.+emission_02+emission_C 02; 0.505, 0.796, tmp_cumulo+pH+PL+AN+AN_cumulo+interval_yi eld+Feed_Vol+RS_cumulo+Cell_conc._interval+rab_integral+RQ_ integral+Arg_conc.+emission_O2+emission_CO2; 0.517, 0.796, pH+pH_cumulo+PL+AN+AN_cumulo+Feed_Vol+RS_cumulo+Cell_conc._ cumulo+rab_integral+RQ_integral+Arg_conc.+emission_O2+emiss ion_CO2; 0.505, 0.796, pH+AN+AN_cumulo+interval_yield+Feed_ Vol+RS_cumulo+Cell_conc._interval+rab_integral+RQ_integral+ Accum._Yield+Arg_conc.+emission_O2+emission_CO2+consum_O2; 0.505, 0.796, tmp_cumulo+pH+PL_cumulo+AN+AN_cumulo+interval _yield+Feed_Vol+RS_cumulo+rab_integral+RQ_integral+Arg_conc. +emission_O2+emission_CO2+interval_O2; 0.528, 0.796, tmp+pH +pH_cumulo+AN+AN_cumulo+Feed_Vol+RS_cumulo+rab_integral+RQ_ integral+Arg_conc.+emission_O2+emission_CO2; 0.505, 0.796, tmp_cumulo+pH_cumulo+PL+PL_cumulo+AN+AN_cumulo+interval_yie ld+Feed_Vol+RS_cumulo+rab_integral+RQ_integral+Arg_conc.+em ission_O2+emission_CO2; 0.550, 0.796, pH+AN_cumulo+interval _yield+Feed_Vol+RS_cumulo+rab_integral+RQ_integral+Arg_conc. +emission_O2+emission_CO2; 0.517, 0.796, pH+PL+PL_cumulo+AN +Feed_Vol+RS_cumulo+rab_integral+RQ_integral+Arg_conc.+emis sion _O2+emission_CO2+consum_O2+generate_CO2; 0.528, 0.796, tmp+pH+PL+AN+Feed_Vol+RS_cumulo+rab_integral+RQ_integral+Ar g_conc.+emission_O2+emission_CO2+interval_O2; 0.528, 0.796, tmp_cumulo+pH+PL+AN+Feed_Vol+RS_cumulo+rab_integral+RQ_int egral+Arg_conc.+emission_O2+emission_CO2+interval_O2; 0.539, 0.796, tmp_cumulo+pH+PL_cumulo+AN_cumulo+Feed_Vol+RS_cumul o+rab_integral+RQ_integral+Arg_conc.+emission_O2+emission_C 02; 0.528, 0.796, pH+pH_cumulo+PL+AN+interval_yield+Feed_Vo l+RS_cumulo+rab_integral+RQ_integral+Arg_conc.+emission_O2+ emission_CO2; 0.517, 0.796, tmp+pH+PL_cumulo+AN+AN_cumulo+F eed_Vol+RS_cumulo+Cell_conc._interval+rab_integral+RQ_integ ral+Arg_conc.+emission_O2+emission_CO2; 0.517, 0.796, pH+PL +AN+AN_cumulo+Feed_Vol+RS_cumulo+Cell_conc._cumulo+rab_inte gral+RQ_integral+Accum._Yield+Arg_conc.+emission_O2+emissio n_CO2; 0.517, 0.796, tmp_cumulo+pH+PL+AN+interval_yield+Fee d_Vol+RS_cumulo+rab_integral+RQ_integral+Accum._Yield+Arg_c onc.+emission_O2+emission_CO2; 0.478, 0.796, tmp_cumulo+pH+ pH_cumulo+PL+PL_cumulo+AN+AN_cumulo+interval_yield+Feed_Vol +RS_cumulo+rab_integral+RQ_integral+Arg_conc.+emission_O2+e mission_CO2+consum_O2; 0.539, 0.796, tmp_cumulo+pH+AN_cumul o+Feed_Vol+RS_cumulo+rab_integral+RQ_integral+Accum._Yield+ Arg_conc.+emission_O2+emission_CO2; 0.504, 0.796, pH+pH_cum ulo+PL_cumulo+AN+AN_cumulo+interval_yield+Feed_Vol+RS_cumul o+rab_integral+RQ_integral+Accum._Yield+Arg_conc.+emission_ O2+emission_CO2; 0.549, 0.796, pH+AN+AN_cumulo+RS+Feed_Vol+ rab_integral+RQ_integral+Arg_conc.+emission_O2+emission-CO 2; 0.516, 0.796, tmp_cumulo+pH+PL_cumulo+AN+AN_cumulo+Feed_ Vol+RS_cumulo+Cell_conc._interval+rab_integral+RQ_integral+ Arg_conc.+emission_O2+emission_CO2; 0.539, 0.796, pH+AN+Fee d_Vol+RS_cumulo+rab_integral+RQ_integral+Accum._Yield+Arg_c onc.+emission_O2+emission_CO2+generate_CO2; 0.478, 0.795, t mp_cumulo+pH+PL+PL_cumulo+AN+AN_cumulo+interval_yield+Feed_ Vol+RS_cumulo+rab_integral+RQ_integral+Arg_conc.+emission_O 2+emission_CO2+consum_O2+generate_CO2; 0.528, 0.795, tmp_cu mulo+pH+PL+AN+Feed_Vol+RS_cumulo+rab_integral+RQ_integral+A rg_conc.+emission_O2+emission_CO2+consum_O2; 0.516, 0.795, pH+pH_cumulo+PL+AN+interval_yield+Feed_Vol+RS_cumulo+Cell_c one._interval+rab_integral+RQ_integral+Arg_conc.+emission_O 2+emission_CO2; 0.539, 0.795, tmp+pH+AN+Feed_Vol+RS_cumulo+ Cell_conc._interval+rab_integral+RQ_integral+Arg_conc.+emis sion_O2+emission_CO2; 0.516, 0.795, pH+AN+AN_cumulo+interva l_yield+Feed_Vol+RS_cumulo+rab_integral+RQ_integral+Accum._ Yield+Arg_conc.+emission_O2+emission_CO2+generate_CO2; 0.49 1, 0.795, pH+PL_cumulo+AN+AN_cumulo+interval_yield+Feed_Vol +RS_cumulo+Cell_conc._interval+rab_integral+RQ_integral+Acc um._Yield+Arg_conc.+emission_O2+emission_CO2+consum_O2; 0.5 39, 0.795, pH+PL_cumulo+AN+Feed_Vol+RS_cumulo+rab_integral+ RQ_integral+Arg_conc.+emission_O2+emission_CO2+interval_O2; 0.516, 0.795, pH+pH_cumulo+PL+AN+AN_cumulo+Feed_Vol+RS_cum ulo+rab_integral+RQ_integral+Arg_conc.+emission_O2+emission _CO2+consum_O2; 0.491, 0.795, pH+PL_cumulo+AN+AN_cumulo+int erval_yield+Feed_Vol+RS_cumulo+Cell_conc._interval+rab_inte gral+RQ_integral+Accum._Yield+Arg_conc.+emission_O2+emissio n_CO2+generate_CO2; 0.516, 0.795, pH+PL_cumulo+AN+AN_cumulo +Feed_Vol+RS_cumulo+Cell_conc._cumulo+Cell_conc._interval+r ab_integral+RQ_integral+Arg_conc.+emission_O2+emission_CO2; 0.491, 0.795, tmp_cumulo+pH_cumulo+PL+PL_cumulo+AN+AN_cumu lo+interval_yield+Feed_Vol+RS_cumulo+rab_integral+RQ_integr al+Arg_conc.+emission_O2+emission_CO2+interval_O2; 0.491, 0. 795, tmp+pH+PL+PL_cumulo+AN+interval_yield+Feed_Vol+RS_cumu lo+rab_integral+RQ_integral+Accum._Yield+Arg_conc.+emission _O2+emission_CO2+interval_O2; 0.516, 0.795, pH+PL+PL_cumulo +AN+Feed_Vol+RS_cumulo+Cell_conc._cumulo+rab_integral+RQ_in tegral+Arg_conc.+emission_O2+emission_CO2+consum_O2; 0.516, 0.795, pH+PL+AN+AN_cumulo+Feed_Vol+RS_cumulo+Cell_conc._in terval+rab_integral+RQ_integral+Accum._Yield+Arg_conc.+emis sion_O2+emission_CO2; 0.528, 0.795, tmp+tmp_cumulo+pH+AN_cu mulo+Feed_Vol+RS_cumulo+rab_integral+RQ_integral+Accum._Yie ld+Arg_conc.+emission_O2+emission_CO2; 0.539, 0.795, pH+AN_ cumulo+interval_yield+Feed_Vol+RS_cumulo+rab_integral+RQ_in tegral+Accum._Yield+Arg_conc.+emission_O2+emission_CO2; 0.5 16, 0.795, pH+PL+AN+AN_cumulo+Feed_Vol+RS_cumulo+rab_integr al+RQ_integral+Accum._Yield+Arg_conc.+emission_O2+emission_ CO2+consum_O2; 0.491, 0.795, tmp+pH+PL_cumulo+AN+AN_cumulo+ interval_yield+Feed_Vol+RS_cumulo+Cell_conc._interval+rab_i ntegral+RQ_integral+Accum._Yield+Arg_conc.+emission_O2+emis sion_CO2; 0.527, 0.795, pH+PL+AN+Feed_Vol+RS_cumulo+Cell_co nc._interval+rab_integral+RQ_integral+Accum._Yield+Arg_conc. +emission_O2+emission_CO2; 0.491, 0.795, tmp+tmp_cumulo+pH+ PL+PL_cumulo+AN+AN_cumulo+interval_yield+Feed_Vol+RS_cumulo +rab_integral+RQ_integral+Arg_conc.+emission_O2+emission_CO 2; 0.491, 0.795, tmp+pH+PL+PL_cumulo+AN+AN_cumulo+Feed_Vol+ RS_cumulo+rab_integral+RQ_integral+Arg_conc.+emission_O2+em ission_CO2+consum_O2+generate_CO2; 0.538, 0.795, pH+AN+Feed _Vol+RS_cumulo+Cell_conc._interval+rab_integral+RQ_integral +Arg_conc.+emission_O2+emission_CO2+generate_CO2; 0.516, 0. 795, pH+AN+AN_cumulo+interval_yield+Feed_Vol+RS_cumulo+rab_ integral+RQ_integral+Accum._Yield+Arg_conc.+emission_O2+emi ssion_CO2+interval_O2; 0.538, 0.795, tmp_cumulo+pH+AN+Feed_ Vol+RS_cumulo+Cell_conc._interval+rab_integral+RQ_integral+ Arg_conc.+emission_O2+emission_CO2; 0.504, 0.795, pH+PL+PL_ cumulo+AN+interval_yield+Feed_Vol+RS_cumulo+Cell_conc._cumu lo+rab_integral+RQ_integral+Arg_conc.+emission_O2+emission_ CO2+consum_O2; 0.491, 0.795, tmp_cumulo+pH+pH_cumulo+PL+PL_ cumulo+AN+AN_cumulo+interval_yield+Feed_Vol+RS_cumulo+rab_i ntegral+RQ_integral+Arg_conc.+emission_O2+emission_CO2; 0.5 49, 0.795, pH+AN_cumulo+Feed_Vol+RS_cumulo+rab_integral+RQ_ integral+Arg_conc.+emission_O2+emission_CO2+generate_CO2; 0. 504, 0.795, tmp+pH+PL+AN+AN_cumulo+interval_yield+Feed_Vol+ RS_cumulo+rab_integral+RQ_integral+Accum._Yield+Arg_conc.+e mission_O2+emission_CO2; 0.527, 0.795, pH+PL+AN+Feed_Vol+RS _cumulo+rab_integral+RQ_integral+Accum._Yield+Arg_conc.+emi ssion_O2+emission_CO2+generate_CO2; 0.527, 0.795,pH_cumulo+ AN+interval_yield+Feed_Vol+RS_cumulo+Cell_conc._interval+ra b_integral+RQ_integral+Accum._Yield+Arg_conc.+emission_O2+e mission_CO2; 0.516, 0.795, tmp_cumulo+pH+AN+AN_cumulo+Feed_ Vol+RS_cumulo+rab_integral+RQ_integral+Accum._Yield+Arg_con c.+emission_O2+emission_CO2+interval_O2; 0.491, 0.795, tmp_ cumulo+pH+PL_cumulo+AN+AN_cumulo+interval_yield+Feed_Vol+RS _cumulo+Cell_conc._interval+rab_integral+RQ_integral+Accum. _Yield+Arg_conc.+emission_O2+emission_CO2; 0.516, 0.795, pH +AN+AN_cumulo+Feed_Vol+RS_cumulo+Cell_conc._interval+rab_in tegral+RQ_integral+Accum._Yield+Arg_conc.+emission_O2+emiss ion_CO2+interval_O2; 0.491, 0.795, pH_cumulo+PL_cumulo+AN+A N_cumulo+interval_yield+Feed_Vol+RS_cumulo+Cell_conc._inter val+rab_integral+RQ_integral+Accum._Yield+Arg_conc.+emissio n_O2+emission_CO2+interval_O2; 0.538, 0.795, pH+AN+Feed_Vol +RS_cumulo+Cell_conc._interval+rab_integral+RQ_integral+Arg _conc.+emission_O2+emission_CO2+interval_O2; 0.504, 0.795, pH+PL+PL_cumulo+AN+AN_cumulo+Feed_Vol+RS_cumulo+Cell_conc._ interval+rab_integral+RQ_integral+Arg_conc.+emission_O2+emi ssion_CO2+interval_O2; 0.527, 0.795, pH_cumulo+PL_cumulo+AN +AN_cumulo+Feed_Vol+RS_cumulo+rab_integral+RQ_integral+Arg_ conc.+emission_O2+emission_CO2+interval_O2; 0.527, 0.795, p H+pH_cumulo+AN+interval_yield+Feed_Vol+RS_cumulo+Cell_conc. _interval+rab_integral+RQ_integral+Arg_conc.+emission_O2+em ission_CO2; 0.491, 0.795, tmp_cumulo+pH_cumulo+PL_cumulo+AN +AN_cumulo+interval_yield+Feed_Vol+RS_cumulo+Cell_conc._int erval+rab_integral+RQ_integral+Accum._Yield+Arg_conc.+emiss ion_O2+emission_CO2; 0.503, 0.795, tmp+pH+PL+PL_cumulo+AN+F eed_Vol+RS_cumulo+rab_integral+RQ_integral+Accum._Yield+Arg _conc.+emission_O2+emission_CO2+consum_O2; 0.516, 0.795, pH +AN+AN_cumulo+interval_yield+Feed_Vol+RS_cumulo+Cell_conc._ interval+rab_integral+RQ_integral+Arg_conc.+emission_O2+emi ssion_CO2+interval_O2; 0.477, 0.795, tmp+pH+PL+PL_cumulo+AN +AN_cumulo+interval_yield+Feed_Vol+RS_cumulo+rab_integral+R Q_integral+Arg_conc.+emission_O2+emission_CO2+consum_O2+gen erate_CO2; 0.527, 0.795, tmp+pH+PL+AN+Feed_Vol+RS_cumulo+Ce ll_conc._interval+rab_integral+RQ_integral+Arg_conc.+emissi on_O2+emission_CO2; 0.515, 0.795, pH+PL+PL_cumulo+AN+interv al_yield+Feed_Vol+RS_cumulo+rab_integral+RQ_integral+Arg_co nc.+emission_O2+emission_CO2+generate_CO2; 0.515, 0.795, tm p+pH+PL+PL_cumulo+AN+Feed_Vol+RS_cumulo+rab_integral+RQ_int egral+Arg_conc.+emission_O2+emission_CO2+interval_O2; 0.515, 0.795, tmp+pH+AN+AN_cumulo+Feed_Vol+RS_cumulo+Cell_conc._i nterval+rab_integral+RQ_integral+Accum._Yield+Arg_conc.+emi ssion_O2+emission_CO2; 0.527, 0.795, tmp+pH+PL+AN+Feed_Vol+ RS_cumulo+rab_integral+RQ_integral+Arg_conc.+emission_O2+em ission_CO2+generate_CO2; 0.538, 0.795, tmp+pH+AN+Feed_Vol+R S_cumulo+rab_integral+RQ_integral+Accum._Yield+Arg_conc.+em ission_O2+emission_CO2; 0.527, 0.795, pH+PL+PL_cumulo+AN+Fe ed_Vol+RS_cumulo+Cell _conc. _cumulo+rab_integral+RQ_integral +Arg_conc.+emission_O2+emission_CO2; 0.490, 0.795, pH+pH_cu mulo+PL+PL_cumulo+AN+AN_cumulo+interval_yield+Feed_Vol+RS_c umulo+rab_integral+RQ_integral+Accum._Yield+Arg_conc.+emiss ion_O2+emission_CO2; 0.515, 0.795, tmp_cumulo+pH_cumulo+PL_ cumulo+AN+AN_cumulo+interval_yield+Feed_Vol+RS_cumulo+rab_i ntegral+RQ_integral+Arg_conc.+emission_O2+emission_CO2; 0.5 38, 0.795, pH_cumulo+PL+AN+AN_cumulo+Feed_Vol+RS_cumulo+rab _integral+RQ_integral+Arg_conc.+emission_O2+emission_CO2; 0. 515, 0.795, pH+pH_cumulo+PL_cumulo+AN+AN_cumulo+Feed_Vol+RS _cumulo+rab_integral+RQ_integral+Arg_conc.+emission_O2+emis sion_CO2+interval_O2; 0.490, 0.795, tmp+pH+pH_cumulo+PL+PL_ cumulo+AN+AN_cumulo+interval_yield+Feed_Vol+RS_cumulo+rab_i ntegral+RQ_integral+Arg_conc.+emission_O2+emission_CO2; 0.5 03, 0.795, pH+pH_cumulo+PL+PL_cumulo+AN+AN_cumulo+Feed_Vol+ RS_cumulo+rab_integral+RQ_integral+Accum._Yield+Arg_conc.+e mission_O2+emission_CO2; 0.527, 0.795, pH+AN+AN_cumulo+Feed _Vol+RS_cumulo+Cell_conc._cumulo+Cell_conc._interval+rab_in tegral+RQ_integral+Arg_conc.+emission_O2+emission_CO2; 0.54 8, 0.795, pH+AN_cumulo+Feed_Vol+RS_cumulo+rab_integral+RQ_i ntegral+Arg_conc.+emission_O2+emission_CO2+interval_O2; 0.4 90, 0.795, pH+PL+PL_cumulo+AN+interval_yield+Feed_Vol+RS_cu mulo+Cell_conc._interval+rab_integral+RQ_integral+Accum._Yi eld+Arg_conc.+emission_O2+emission_CO2+interval_O2; 0.503, 0.795, pH+PL+PL_cumulo+AN+interval_yield+Feed_Vol+RS_cumulo +rab_integral+RQ_integral+Arg_conc.+emission_O2+emission_CO 2+consum_O2+generate_CO2; 0.503, 0.795, tmp+pH+PL+AN+AN_cum ulo+interval_yield+Feed_Vol+RS_cumulo+Cell_conc._interval+r ab_integral+RQ_integral+Arg_conc.+emission_O2+emission_CO2; 0.515, 0.795, pH+PL_cumulo+AN+AN_cumulo+Feed_Vol+RS_cumulo +Cell_conc._interval+rab_integral+RQ_integral+Arg_conc.+emi ssion_O2+emission_CO2+interval_O2; 0.490, 0.795, tmp_cumulo +pH+PL+PL_cumulo+AN+AN_cumulo+Feed_Vol+RS_cumulo+rab_integr al+RQ_integral+Arg_conc.+emission_O2+emission_CO2+consum_O2 +generate_CO2; 0.537, 0.795, tmp+tmp_cumulo+pH+AN+Feed_Vol+ RS_cumulo+rab_integral+RQ_integral+Arg_conc.+emission_O2+em ission_CO2; 0.503, 0.795, pH+PL+AN+interval_yield+Feed_Vol+ RS_cumulo+Cell_conc._interval+rab_integral+RQ_integral+Accu m._Yield+Arg_conc.+emission_O2+emission_CO2+generate_CO2; 0. 548, 0.795, pH+pH_cumulo+AN_cumulo+Feed_Vol+RS_cumulo+rab_i ntegral+RQ_integral+Arg_conc.+emission_O2+emission_CO2; 0.5 26, 0.795, pH+pH_cumulo+AN+AN_cumulo+Feed_Vol+RS_cumulo+Cel l_conc._interval+rab_integral+RQ_integral+Arg_conc.+emissio n_O2+emission_CO2; 0.490, 0.795, tmp+pH+PL+PL_cumulo+AN+int erval_yield+Feed_Vol+RS_cumulo+Cell_conc._interval+rab_inte gral+RQ_integral+Arg_conc.+emission_O2+emission_CO2+interva l_O2; 0.490, 0.795, tmp_cumulo+pH+PL+PL_cumulo+AN+interval_ yield+Feed_Vol+RS_cumulo+Cell_conc._cumulo+rab_integral+RQ_ integral+Arg_conc.+emission_O2+emission_CO2+consum_O2; 0.51 5, 0.795, tmp+tmp_cumulo+pH+AN+AN_cumulo+Feed_Vol+RS_cumulo +rab_integral+RQ_integral+Arg_conc.+emission_O2+emission_CO 2+generate_CO2; 0.537, 0.795, pH+AN+Feed_Vol+RS_cumulo+rab_ integral+RQ_integral+Accum._Yield+Arg_conc.+emission_O2+emi ssion_CO2+interval_O2; 0.526, 0.795, tmp_cumulo+pH+PL+AN+Fe ed_Vol+RS_cumulo+Cell _conc. _interval+rab _integral+RQ_integr al+Arg_conc.+emission_O2+emission_CO2; 0.515, 0.795, pH+PL_ cumulo+AN+AN_cumulo+Feed_Vol+RS_cumulo+Cell_conc._interval+ rab_integral+RQ_integral+Arg_conc.+emission_O2+emission_CO2 +consum_O2; 0.515, 0.795, tmp+pH+AN+AN_cumulo+Feed_Vol+RS_c umulo+rab_integral+RQ_integral+Accum._Yield+Arg_conc.+emiss ion_O2+emission_CO2+interval_O2; 0.515, 0.795, tmp_cumulo+p H+pH_cumulo+PL_cumulo+AN+AN_cumulo+Feed_Vol+RS_cumulo+rab_i ntegral+RQ_integral+Arg_conc.+emission_O2+emission_CO2; 0.5 26, 0.795, pH_cumulo+AN+AN_cumulo+interval_yield+Feed_Vol+R S_cumulo+Cell_conc._interval+rab_integral+RQ_integral+Arg_c onc.+emission_O2+emission_CO2; 0.526, 0.795, pH+pH_cumulo+A N+AN_cumulo+Feed_Vol+RS_cumulo+rab_integral+RQ_integral+Arg _conc.+emission_O2+emission_CO2+generate_CO2; 0.503, 0.795, tmp+pH_cumulo+PL+PL_cumulo+AN+AN_cumulo+interval_yield+Fee d_Vol+RS_cumulo+rab_integral+RQ_integral+Arg_conc.+emission _O2+emission_CO2; 0.526, 0.795, pH_cumulo+PL_cumulo+AN+AN_c umulo+Feed_Vol+RS_cumulo+Cell_conc._interval+rab_integral+R Q_integral+Arg_conc.+emission_O2+emission_CO2; 0.503, 0.795, pH+pH_cumulo+PL+AN+interval_yield+Feed_Vol+RS_cumulo+Cell_ conc._interval+rab_integral+RQ_integral+Accum._Yield+Arg_co nc.+emission_O2+emission_CO2; 0.537, 0.795, pH+PL_cumulo+AN _cumulo+Feed_Vol+RS_cumulo+rab_integral+RQ_integral+Arg_con c.+emission_O2+emission_CO2+interval_O2; 0.490, 0.795, tmp+ pH+PL+PL_cumulo+AN+interval_yield+Feed_Vol+RS_cumulo+Cell_c onc._interval+rab_integral+RQ_integral+Accum._Yield+Arg_con c.+emission_O2+emission_CO2; 0.490, 0.795, tmp_cumulo+pH+pH _cumulo+PL+PL_cumulo+AN+AN_cumulo+Feed_Vol+RS_cumulo+rab_in tegral+RQ_integral+Arg_conc.+emission_O2+eniission_CO2+consu m_O2; 0.558, 0.795, pH+AN+RS+Feed_Vol+rab_integral+RQ_integ ral+Arg_conc.+emission_O2+emission_CO2; 0.548, 0.795, pH+PL +AN_cumulo+Feed_Vol+RS_cumulo+rab_integral+RQ_integral+Arg_ conc.+emission_O2+emission_CO2; 0.526, 0.795, pH+AN_cumulo+ interval_yield+Feed_Vol+RS_cumulo+Cell_conc._interval+rab_i ntegral+RQ_integral+Accum._Yield+Arg_conc.+emission_O2+emis sion_CO2; 0.526, 0.795, tmp_cumulo+pH+PL+AN+Feed_Vol+RS_cum ulo+rab_integral+RQ_integral+Arg_conc.+emission_O2+emission _CO2+generate_CO2; 0.476, 0.795, tmp+pH+pH_cumulo+PL+PL_cum ulo+AN+AN_cumulo+interval_yield+Feed_Vol+RS_cumulo+rab_inte gral+RQ_integral+Arg_conc.+emission_O2+emission_CO2+interva l_02; 0.526, 0.795, pH+PL+AN+Feed_Vol+RS_cumulo+rab_integra l+RQ_integral+Accum._Yield+Arg_conc.+emission_O2+emission_C O2+interval_O2; 0.514, 0.795, tmp_cumulo+pH+PL+AN+interval_ yield+Feed_Vol+RS_cumulo+rab_integral+RQ_integral+Arg_conc. +emission_O2+emission_CO2+interval_O2; 0.502, 0.795, pH_cum ulo+PL+PL_cumulo+AN+AN_cumulo+interval_yield+Feed_Vol+RS_cu mulo+rab_integral+RQ_integral+Accum._Yield+Arg_conc.+emissi on_O2+emission_CO2; 0.502, 0.795, tmp+pH+PL_cumulo+AN+AN_cu mulo+interval_yield+Feed_Vol+RS_cumulo+rab_integral+RQ_inte gral+Arg_conc.+emission_O2+emission_CO2+interval_O2; 0.489, 0.795, tmp_cumulo+pH+PL+PL_cumulo+AN+AN_cumulo+Feed_Vol+RS _cumulo+rab_integral+RQ_integral+Accum._Yield+Arg_conc.+emi ssion_O2+emission_CO2+consum_O2; 0.514, 0.795, pH+pH_cumulo +AN+AN_cumulo+interval_yield+Feed_Vol+RS_cumulo+Cell_conc._ interval+rab_integral+RQ_integral+Arg_conc.+emission_O2+emi ssion_CO2; 0.514, 0.794, pH+PL+AN+interval_yield+Feed_Vol+R S_cumulo+Cell_conc._interval+rab_integral+RQ_integral+Arg_c onc.+emission_O2+emission_CO2+generate_CO2; 0.514, 0.794, p H+pH_cumulo+PL_cumulo+AN+AN_cumulo+Feed_Vol+RS_cumulo+rab_i ntegral+RQ_integral+Arg_conc.+emission_O2+emission_CO2+cons um_O2; 0.502, 0.794, tmp+tmp_cumulo+pH+PL+AN+AN_cumulo+Feed _Vol+RS_cumulo+rab_integral+RQ_integral+Arg_conc.+emission_ O2+emission_CO2+generate_CO2; 0.502, 0.794, tmp+pH+PL+PL_cu mulo+AN+AN_cumulo+Feed_Vol+RS_cumulo+Cell_conc._interval+ra b_integral+RQ_integral+Arg_conc.+emission_O2+emission_CO2; 0.514, 0.794, pH+PL+AN+AN_cumulo+Feed_Vol+RS_cumulo+Cell_co nc._interval+rab_integral+RQ_integral+Arg_conc.+emission_O2 +emission_CO2+consum_O2; 0.502, 0.794, tmp+pH+PL_cumulo+AN+ interval_yield+Feed_Vol+RS_cumulo+Cell_conc._interval+rab_i ntegral+RQ_integral+Accum._Yield+Arg_conc.+emission_O2+emis sion_CO2; 0.489, 0.794, tmp+pH+PL+PL_cumulo+AN+interval_yie ld+Feed_Vol+RS_cumulo+rab_integral+RQ_integral+Arg_conc.+em ission_O2+emission_CO2+consum_O2+generate_CO2; 0.476, 0.794, tmp_cumulo+pH+PL+PL_cumulo+AN+AN_cumulo+interval_yield+Fee d_Vol+RS_cumulo+rab_integral+RQ_integral+Arg_conc.+emission _O2+emission_CO2+interval_O2+generate_CO2; 0.526, 0.794, pH +AN+AN_cumulo+interval_yield+Feed_Vol+RS_cumulo+Cell_conc._ cumulo+rab_integral+RQ_integral+Arg_conc.+emission_O2+emiss ion_CO2; 0.514, 0.794, tmp_cumulo+pH_cumulo+PL+PL_cumulo+AN +AN_cumulo+Feed_Vol+RS_cumulo+rab_integral+RQ_integral+Arg_ conc.+emission_O2+emission_CO2; 0.489, 0.794, tmp+pH+PL+PL_ cumulo+AN+AN_cumulo+Feed_Vol+RS_cumulo+rab_integral+RQ_inte gral+Accum._Yield+Arg_conc.+emission_O2+emission_CO2+consum _O2; 0.526, 0.794, pH+PL+AN+interval_yield+Feed_Vol+RS_cumu lo+rab_integral+RQ_integral+Arg_conc.+emission_O2+emission_ CO2+generate_CO2; 0.526, 0.794, pH+PL+AN+Feed_Vol+RS_cumulo +rab_integral+RQ_integral+Arg_conc.+emission_O2+emission_CO 2+consum_O2+interval_O2; 0.489, 0.794, pH+PL+PL_cumulo+AN+A N_cumulo+interval_yield+Feed_Vol+RS_cumulo+rab_integral+RQ_ integral+Accum._Yield+Arg_conc.+emission_O2+emission_CO2+ge nerate_CO2; 0.476, 0.794, tmp_cumulo+pH+PL+PL_cumulo+AN+int erval_yield+Feed_Vol+RS_cumulo+Cell_conc._interval+rab_inte gral+RQ_integral+Accum._Yield+Arg_conc.+emission_O2+emissio n_CO2+consum_O2; 0.525, 0.794, pH_cumulo+PL_cumulo+AN+AN_cu mulo+Feed_Vol+RS_cumulo+Cell_conc._cumulo+rab_integral+RQ_i ntegral+Arg_conc.+emission_O2+emission_CO2; 0.525, 0.794, t mp_cumulo+pH+PL_cumulo+AN+interval_yield+Feed_Vol+RS_cumulo +rab_integral+RQ_integral+Arg_conc.+emission_O2+emission_CO 2; 0.525, 0.794, pH+pH_cumulo+AN+AN_cumulo+Feed_Vol+RS_cumu lo+Cell_conc._cumulo+rab_integral+RQ_integral+Arg_conc.+emi ssion_O2+emission_CO2; 0.475, 0.794, tmp_cumulo+pH+PL+PL_cu mulo+AN+AN_cumulo+interval_yield+Feed_Vol+RS_cumulo+Cell_co nc._cumulo+rab_integral+RQ_integral+Arg_conc.+emission_O2+e mission_CO2+consum_O2; 0.475, 0.794, tmp_cumulo+pH+PL+PL_cu mulo+AN+AN_cumulo+interval_yield+Feed_Vol+RS_cumulo+rab_int egral+RQ_integral+Arg_conc.+emission_O2+emission_CO2+consum O2+interval O2; 0.489, 0.794, tmp_cumulo+pH+PL+PL_cumulo+A N+interval_yield+Feed_Vol+RS_cumulo+rab_integral+RQ_integra 1+Accum._Yield+Arg_conc.+emission_O2+emission_CO2+interval_ 02; 0.514, 0.794, tmp+pH+PL+PL_cumulo+AN+Feed_Vol+RS_cumulo +rab_integral+RQ_integral+Accum._Yield+Arg_conc.+emission_O 2+emission_CO2; 0.489, 0.794, tmp+pH+PL+PL_cumulo+AN+AN_cum ulo+Feed_Vol+RS_cumulo+rab_integral+RQ_integral+Arg_conc.+e mission _O2+emission_CO2+interval_O2+generate_CO2; 0.514, 0. 794, pH_cumulo+PL_cumulo+AN+AN_cumulo+interval_yield+Feed_V ol+RS_cumulo+rab_integral+RQ_integral+Arg_conc.+emission_O2 +emission_CO2+interval_O2; 0.525, 0.794, tmp+pH+PL+AN+Feed_ Vol+RS_cumulo+rab_integral+RQ_integral+Arg_conc.+emission_O 2+emission_CO2+consum_O2; 0.489, 0.794, tmp+pH_cumulo+PL_cu mulo+AN+AN_cumulo+interval_yield+Feed_Vol+RS_cumulo+Cell_co nc._interval+rab_integral+RQ_integral+Accum._Yield+Arg_conc. +emission_O2+emission_CO2; 0.514, 0.794, tmp+pH_cumulo+PL_c umulo+AN+AN_cumulo+interval_yield+Feed_Vol+RS_cumulo+rab_in tegral+RQ_integral+Arg_conc.+emission_O2+emission_CO2; 0.48 9, 0.794, tmp_cumulo+pH+PL+PL_cumulo+AN+AN_cumulo+Feed_Vol+ RS_cumulo+rab_integral+RQ_integral+Arg_conc.+emission_O2+em ission_CO2+interval_O2+generate_CO2; 0.489, 0.794, pH+PL+PL _cumulo+AN+AN_cumulo+Feed_Vol+RS_cumulo+Cell_conc._cumulo+r ab_integral+RQ_integral+Arg_conc.+emission_O2+emission_CO2+ consum_O2+generate_CO2; 0.489, 0.794, pH+pH_cumulo+PL+PL_cu mulo+AN+AN_cumulo+Feed_Vol+RS_cumulo+Cell_conc._cumulo+rab_ integral+RQ_integral+Arg_conc.+emission_O2+emission_CO2+con sum_O2; 0.536, 0.794, pH+AN+interval_yield+Feed_Vol+RS_cumu lo+rab_integral+RQ_integral+Arg_conc.+emission_O2+emission_ CO2+interval_O2; 0.501, 0.794, pH+pH_cumulo+PL_cumulo+AN+AN _cumulo+interval_yield+Feed_Vol+RS_cumulo+Cell_conc._interv al+rab integral+RQ integral+Arg conc.+emission_O2+emission CO2; 0.513, 0.794, tmp+pH_cumulo+PL+PL_cumulo+AN+AN_cumulo+ Feed Vol+RS cumulo+rab integral+RQ integral+Arg conc.+emiss ion_O2+emission_CO2; 0.501, 0.794, pH_cumulo+PL+PL_cumulo+A N+AN_cumulo+interval_yield+Feed_Vol+RS_cumulo+rab_integral+ RQ_integral+Arg_conc.+emission_O2+emission_CO2+consum_O2; 0. 525, 0.794, tmp+tmp_cumulo+pH+AN_cumulo+interval_yield+Feed _Vol+RS_cumulo+rab_integral+RQ_integral+Arg_conc.+emission_ O2+emission_CO2; 0.547, 0.794, pH+PL_cumulo+AN_cumulo+Feed_ Vol+RS_cumulo+rab_integral+Accum._Yield+Arg_conc.+emission_ O2+emission_CO2; 0.513, 0.794, pH+AN+AN_cumulo+Feed_Vol+RS_ cumulo+rab_integral+RQ_integral+Accum._Yield+Arg_conc.+emis sion_O2+emission_CO2+consum_O2+interval_O2; 0.501, 0.794, t mp_cumulo+pH+PL+PL_cumulo+AN+interval_yield+Feed_Vol+RS_cum ulo+rab_integral+RQ_integral+Accum._Yield+Arg_conc.+emissio n_O2+emission_CO2; 0.525, 0.794, pH+PL+AN+Feed_Vol+RS_cumul o+Cell_conc._interval+rab_integral+RQ_integral+Arg_conc.+em ission_O2+emission_CO2+interval_O2; 0.501, 0.794, tmp_cumul o+pH+PL+PL_cumulo+AN+AN_cumulo+Feed_Vol+RS_cumulo+Cell_conc. _interval+rab_integral+RQ_integral+Arg_conc.+emission_O2+em ission_CO2; 0.536, 0.794, tmp+pH+AN+Feed_Vol+RS_cumulo+rab_ integral+RQ_integral+Arg_conc.+emission_O2+emission_CO2+int erval_O2; 0.513, 0.794, pH+AN+AN_cumulo+Feed_Vol+RS_cumulo+ rab_integral+RQ_integral+Accum._Yield+Arg_conc.+emission_O2 +emission_CO2+interval_O2+generate_CO2; 0.501, 0.794, tmp_c umulo+pH+pH_cumulo+PL+AN+AN_cumulo+interval_yield+Feed_Vol+ RS_cumulo+rab_integral+RQ_integral+Arg_conc.+emission_O2+em ission_CO2; 0.513, 0.794, pH+PL+PL_cumulo+AN+Feed_Vol+RS_cu mulo+Cell_conc._interval+rab_integral+RQ_integral+Arg_conc. +emission_O2+emission_CO2+consum_O2; 0.488, 0.794, tmp_cumu lo+pH+PL+AN+AN_cumulo+interval_yield+Feed_Vol+RS_cumulo+Cel l_conc._interval+rab_integral+RQ_integral+Accum._Yield+Arg_ conc.+emission_O2+emission_CO2; 0.501, 0.794, pH+PL_cumulo+ AN+AN_cumulo+interval_yield+Feed_Vol+RS_cumulo+Cell_conc._i nterval+rab_integral+RQ_integral+Arg_conc.+emission_O2+emis sion_CO2+interval_O2; 0.536, 0.794, pH+PL+PL_cumulo+AN_cumu lo+Feed_Vol+RS_cumulo+rab_integral+RQ_integral+Arg_conc.+em ission_O2+emission_CO2; 0.525, 0.794, pH+PL+AN+Feed_Vol+RS_ cumulo+Cell_conc._interval+rab_integral+RQ_integral+Arg_con c.+emission_O2+emission_CO2+generate_CO2; 0.475, 0.794, tmp _cumulo+pH+PL+PL_cumulo+AN+AN_cumulo+interval_yield+Feed_Vo l+RS_cumulo+Cell_conc._interval+rab_integral+RQ_integral+Ar g_conc.+emission_O2+emission_CO2+consum_O2; 0.525, 0.794, t mp+pH+PL_cumulo+AN_cumulo+Feed_Vol+RS_cumulo+rab_integral+R Q_integral+Accum._Yield+Arg_conc.+emission_O2+emission_CO2; 0.536, 0.794, tmp_cumulo+pH+AN+Feed_Vol+RS_cumulo+rab_inte gral+RQ_integral+Arg_conc.+emission_O2+emission_CO2+generat e_CO2; 0.501, 0.794, pH+PL+PL_cumulo+AN+AN_cumulo+Feed_Vol+ RS_cumulo+Cell_conc._cumulo+rab_integral+RQ_integral+Accum. _Yield+Arg_conc.+emission_O2+emission_CO2; 0.513, 0.794, pH +AN+AN_cumulo+Feed_Vol+RS_cumulo+rab_integral+RQ_integral+A rg_conc.+emission_O2+emission_CO2+consum_O2+interval_O2+gen erate_CO2; 0.525, 0.794, pH_cumulo+PL_cumulo+AN+AN_cumulo+F eed_Vol+RS_cumulo+rab_integral+RQ_integral+Arg_conc.+emissi on_O2+emission_CO2+generate_CO2; 0.536, 0.794, tmp_cumulo+p H+AN+Feed_Vol+RS_cumulo+rab_integral+RQ_integral+Arg_conc.+ emission_O2+emission_CO2+interval_O2; 0.475, 0.794, pH+PL+P L_cumulo+AN+AN_cumulo+interval_yield+Feed_Vol+RS_cumulo+Cel l_conc._interval+rab_integral+RQ_integral+Accum._Yield+Arg_ conc.+emission_O2+emission_CO2+consum_O2; 0.525, 0.794, pH+ AN+interval_yield+Feed_Vol+RS_cumulo+Cell_conc._interval+ra b_integral+RQ_integral+Arg_conc.+emission_O2+emission_CO2+c onsum_O2; 0.513, 0.794, tmp+pH+PL+AN+interval_yield+Feed_Vo l+RS_cumulo+rab_integral+RQ_integral+Accum._Yield+Arg_conc. +emission_O2+emission_CO2; 0.488, 0.794, tmp+tmp_cumulo+pH+ PL+PL_cumulo+AN+AN_cumulo+Feed_Vol+RS_cumulo+rab_integral+R Q_integral+Arg_conc.+emission_O2+emission_CO2+interval_O2; 0.501, 0.794, pH+PL+AN+interval_yield+Feed_Vol+RS_cumulo+Ce ll_conc._interval+rab_integral+RQ_integral+Accum._Yield+Arg _conc.+emission_O2+emission_CO2+consum_O2; 0.524, 0.794, pH +PL_cumulo+AN+interval_yield+Feed_Vol+RS_cumulo+rab_integra l+RQ_integral+Arg_conc.+emission_O2+emission_CO2+consum_O2; 0.474, 0.794, tmp_cumulo+pH+PL+PL_cumulo+AN+AN_cumulo+inte rval_yield+Feed_Vol+RS_cumulo+Cell_conc._interval+rab_integ ral+RQ_integral+Arg_conc.+emission_O2+emission_CO2+interval _O2; 0.474, 0.794, pH+PL+PL_cumulo+AN+AN_cumulo+interval_yi eld+Feed_Vol+RS_cumulo+Cell_conc._interval+rab_integral+RQ_ integral+Accum._Yield+Arg_conc.+emission_O2+emission_CO2+in terval_O2; 0.513, 0.794, pH+PL_cumulo+AN_cumulo+interval_yi eld+Feed_Vol+RS_cumulo+rab_integral+RQ_integral+Accum._Yiel d+Arg_conc.+emission_O2+emission_CO2+interval_O2; 0.501, 0. 794, pH+PL_cumulo+AN+AN_cumulo+Feed_Vol+RS_cumulo+rab_integ ral+RQ_integral+Accum._Yield+Arg_conc.+emission_O2+emission _CO2+interval_O2+generate_CO2; 0.513, 0.794, tmp+pH+pH_cumu lo+PL_cumulo+AN+AN_cumulo+Feed_Vol+RS_cumulo+rab_integral+R Q_integral+Arg_conc.+emission_O2+emission_CO2; 0.524, 0.794, pH+PL+AN+interval_yield+RS+Feed_Vol+Cell_conc._interval+ra b_integral+RQ_integral+Arg_conc.+emission_O2+emission_CO2; 0.535, 0.794, tmp+pH+AN+Feed_Vol+RS_cumulo+rab_integral+RQ_ integral+Arg_conc.+emission_O2+emission_CO2+generate_CO2; 0. 500, 0.794, tmp_cumulo+pH+PL+PL_cumulo+AN+interval_yield+Fe ed_Vol+RS_cumulo+Cell _conc. _interval+rab_integral+RQ_integr al+Arg_conc.+emission_O2+emission_CO2; 0.488, 0.794, pH+pH_ cumulo+PL+PL_cumulo+AN+AN_cumulo+interval_yield+Feed_Vol+RS _cumulo+Cell_conc._interval+rab_integral+RQ_integral+Arg_co nc.+emission_O2+emission_CO2; 0.513, 0.794, pH+PL+AN+interv al_yield+Feed_Vol+RS_cumulo+Cell_conc._interval+rab_integra 1+RQ_integral+Arg_conc.+emission_O2+emission_CO2+consum_O2; 0.546, 0.794, pH+AN+interval_yield+RS+Feed_Vol+rab_integra 1+RQ_integral+Arg_conc.+emission_O2+emission_CO2; 0.500, 0. 794, pH+PL+PL_cumulo+AN+interval_yield+Feed_Vol+RS_cumulo+r ab_integral+RQ_integral+Arg_conc.+emission_O2+emission_CO2+ consum_O2+interval_O2; 0.513, 0.794, tmp_cumulo+pH+AN+AN_cu mulo+interval_yield+Feed_Vol+RS_cumulo+rab_integral+RQ_inte gral+Arg_conc.+emission_O2+emission_CO2+interval_O2; 0.513, 0.794, pH+PL+PL_cumulo+AN+Feed_Vol+RS_cumulo+rab_integral+ RQ_integral+Arg_conc.+emission_O2+emission_CO2+consum-O2+in terval_O2; 0.524, 0.794, pH+PL_cumulo+AN+interval_yield+Fee d_Vol+RS_cumulo+rab_integral+RQ_integral+Arg_conc.+emission _O2+emission_CO2+interval_O2; 0.500, 0.794, pH+PL+AN+AN_cum ulo+interval_yield+Feed_Vol+RS_cumulo+Cell_conc._interval+r ab_integral+RQ_integral+Arg_conc.+emission_O2+emission_CO2+ interval_O2; 0.500, 0.794, pH+PL+PL_cumulo+AN+AN_cumulo+Fee d_Vol+RS_cumulo+Cell_conc._interval+rab_integral+RQ_integra l+Arg_conc.+emission_O2+emission_CO2+generate_CO2; 0.488, 0. 794, tmp+pH+PL+PL_cumulo+AN+AN_cumulo+Feed_Vol+RS_cumulo+ra b_integral+RQ_integral+Arg_conc.+emission_O2+emission_CO2+c onsum_O2+interval_O2; 0.524, 0.794, pH+pH_cumulo+AN+AN_cumu lo+Feed_Vol+RS_cumulo+rab_integral+RQ_integral+Arg_conc.+em ission_O2+emission_CO2+consum_O2; 0.500, 0.794, tmp+pH+PL_c umulo+AN+AN_cumulo+Feed_Vol+RS_cumulo+rab_integral+RQ_integ ral+Accum._Yield+Arg_conc.+emission_O2+emission_CO2+generat e_CO2; 0.524, 0.794, pH_cumulo+PL_cumulo+AN+interval_yield+ Feed_Vol+RS_cumulo+Cell_conc._interval+rab_integral+RQ_inte gral+Arg_conc.+emission_O2+emission_CO2; 0.500, 0.794, tmp+ tmp_cumulo+pH+pH_cumulo+PL+AN+AN_cumulo+Feed_Vol+RS_cumulo+ rab_integral+RQ_integral+Arg_conc.+emission_O2+emission_CO 2; 0.500, 0.794, tmp+tmp_cumulo+pH+PL+AN+AN_cumulo+Feed_Vol +RS_cumulo+Cell_conc._cumulo+rab_integral+RQ_integral+Arg_c onc.+emission_O2+emission_CO2; 0.524, 0.794, pH+AN+AN_cumul o+interval_yield+Feed_Vol+RS_cumulo+rab_integral+RQ_integra l+Arg_conc.+emission_O2+emission_CO2+consum_O2; 0.500, 0.79 4, tmp+pH+PL+AN+AN_cumulo+interval_yield+Feed_Vol+RS_cumulo +rab_integral+RQ_integral+Arg_conc.+emission_O2+emission_CO 2+interval_O2; 0.487, 0.794, tmp+pH+PL+PL_cumulo+AN+interva l_yield+Feed_Vol+RS_cumulo+Cell_conc._cumulo+rab_integral+R Q_integral+Arg_conc.+emission_O2+emission_CO2+consum_O2; 0. 474, 0.794, tmp+pH+pH_cumulo+PL+PL_cumulo+AN+AN_cumulo+inte rval_yield+Feed_Vol+RS_cumulo+rab_integral+RQ_integral+Arg_ conc.+emission_O2+emission_CO2+consum_O2; 0.474, 0.794, tmp +pH+PL+PL_cumulo+AN+AN_cumulo+interval_yield+Feed_Vol+RS_cu mulo+rab_integral+RQ_integral+Arg_conc.+emission_O2+emissio n_CO2+consum_O2+interval_O2; 0.535, 0.794, pH_cumulo+AN+AN_ cumulo+interval_yield+Feed_Vol+RS_cumulo+rab_integral+RQ_in tegral+Arg_conc.+emission_O2+emission_CO2; 0.512, 0.794, pH +PL+AN+AN_cumulo+interval_yield+Feed_Vol+RS_cumulo+rab_inte gral+RQ_integral+Arg_conc.+emission_O2+emission_CO2+consum_ 02; 0.487, 0.794, pH_cumulo+PL+PL_cumulo+AN+AN_cumulo+inter val_yield+Feed_Vol+RS_cumulo+Cell_conc._interval+rab_integr al+RQ_integral+Arg_conc.+emission_O2+emission_CO2+interval-02; 0.512, 0.794, pH+PL_cumulo+AN_cumulo+interval_yield+Fee d_Vol+RS_cumulo+Cell_conc._interval+rab_integral+RQ_integra l+Accum._Yield+Arg_conc.+emission_O2+emission_CO2; 0.524, 0. 793, tmp+tmp_cumulo+pH+PL+AN_cumulo+Feed_Vol+RS_cumulo+rab_ integral+RQ_integral+Arg_conc.+emission_O2+emission_CO2; 0. 500, 0.793, tmp_cumulo+pH+PL+AN+AN_cumulo+Feed_Vol+RS_cumul o+rab_integral+RQ_integral+Accum._Yield+Arg_conc.+emission-O2+emission_CO2+generate_CO2; 0.524, 0.793, tmp_cumulo+pH+P L_cumulo+AN_cumulo+Feed_Vol+RS_cumulo+rab_integral+RQ_integ ral+Accum._Yield+Arg_conc.+emission_O2+emission_CO2; 0.473, 0.793, pH+pH_cumulo+PL+PL_cumulo+AN+AN_cumulo+interval_yie ld+Feed_Vol+RS_cumulo+rab_integral+RQ_integral+Accum._Yield +Arg_conc.+emission_O2+emission_CO2+consum_O2; 0.500, 0.793, pH+PL_cumulo+AN+AN_cumulo+Feed_Vol+RS_cumulo+rab_integral+ RQ_integral+Accum._Yield+Arg_conc.+emission_O2+emission_CO2 +consum_O2+interval_O2; 0.487, 0.793, tmp+pH+pH_cumulo+PL+P L_cumulo+AN+AN_cumulo+Feed_Vol+RS_cumulo+rab_integral+RQ_in tegral+Arg_conc.+emission_O2+emission_CO2+consum_O2; 0.487, 0.793, tmp_cumulo+pH+PL+PL_cumulo+AN+AN_cumulo+Feed_Vol+RS _cumulo+rab_integral+RQ_integral+Arg_conc.+emission_O2+emis sion_CO2+consum_O2+interval_O2; 0.535, 0.793, pH+AN+Feed_Vo l+RS_cumulo+rab_integral+RQ_integral+Arg_conc.+emission_O2+ emission_CO2+consum_O2+interval_O2; 0.535, 0.793, pH+PL_cum ulo+AN_cumulo+Feed_Vol+RS_cumulo+rab_integral+RQ_integral+A rg_conc.+emission_O2+emission_CO2+generate_CO2; 0.512, 0.79 3, pH_cumulo+PL+PL_cumulo+AN+interval_yield+Feed_Vol+RS_cum ulo+Cell_conc._interval+rab_integral+RQ_integral+Arg_conc.+ emission_O2+emission_CO2; 0.512, 0.793, pH+PL+PL_cumulo+AN+ Feed_Vol+RS_cumulo+rab_integral+RQ_integral+Arg_conc.+emiss ion_O2+emission_CO2+interval_O2+generate_CO2; 0.535, 0.793, tmp+pH_cumulo+AN+AN_cumulo+Feed_Vol+RS_cumulo+rab_integral +RQ_integral+Arg_conc.+emission_O2+emission_CO2; 0.500, 0.7 93, pH+pH_cumulo+PL+PL_cumulo+AN+interval_yield+Feed_Vol+RS _cumulo+Cell_conc._interval+rab_integral+RQ_integral+Arg_co nc.+emission_O2+emission_CO2; 0.523, 0.793, pH+pH_cumulo+PL +AN+Feed_Vol+RS_cumulo+rab_integral+RQ_integral+Arg_conc.+e mission_O2+emission_CO2+generate_CO2; 0.512, 0.793, pH+PL+A N+interval_yield+RS+Feed_Vol+Cell_conc._interval+rab_integr al+RQ_integral+Accum._Yield+Arg_conc.+emission_O2+emission_ CO2; 0.535, 0.793, pH_cumulo+AN+interval_yield+Feed_Vol+RS_ cumulo+Cell_conc._interval+rab_integral+RQ_integral+Arg_con c.+emission_O2+emission_CO2; 0.523, 0.793, tmp_cumulo+pH+pH _cumulo+PL+AN+Feed_Vol+RS_cumulo+rab_integral+RQ_integral+A rg_conc.+emission_O2+emission_CO2; 0.535, 0.793, tmp_cumulo +pH_cumulo+AN+AN_cumulo+Feed_Vol+RS_cumulo+rab_integral+RQ_ integral+Arg_conc.+emission_O2+emission_CO2; 0.500, 0.793, pH+pH_cumulo+PL+PL_cumulo+AN+AN_cumulo+Feed_Vol+RS_cumulo+r ab_integral+RQ_integral+Arg_conc.+emission_O2+emission_CO2+ generate_CO2; 0.473, 0.793, pH+pH_cumulo+PL+PL_cumulo+AN+AN _cumulo+interval_yield+Feed_Vol+RS_cumulo+rab_integral+RQ_i ntegral+Accum._Yield+Arg_conc.+emission_O2+emission_CO2+int erval_O2; 0.487, 0.793, tmp+pH+PL+AN+AN_cumulo+interval_yie ld+Feed_Vol+RS_cumulo+Cell_conc._interval+rab_integral+RQ_i ntegral+Accum._Yield+Arg_conc.+emission_O2+emission_CO2; 0. 487, 0.793, tmp+tmp_cumulo+pH+PL+AN+AN_cumulo+interval_yiel d+Feed_Vol+RS_cumulo+rab_integral+RQ_integral+Arg_conc.+emi ssion_O2+emission_CO2+interval_O2; 0.534, 0.793, pH+AN+inte rval_yield+Feed_Vol+RS_cumulo+rab_integral+RQ_integral+Arg_ conc.+emission_O2+emission_CO2+generate_CO2; 0.523, 0.793, pH_cumulo+PL_cumulo+AN+AN_cumulo+Feed_Vol+RS_cumulo+rab_int egral+RQ_integral+Arg_conc.+emission_O2+emission_CO2+consum _O2; 0.499, 0.793, tmp+pH+AN+interval_yield+Feed_Vol+RS_cum ulo+Cell_conc._interval+rab_integral+RQ_integral+Accum._Yie ld+Arg_conc.+emission_O2+emission_CO2+interval_O2; 0.487, 0. 793, tmp+pH+PL+PL_cumulo+AN+AN_cumulo+interval_yield+Feed_V ol+RS_cumulo+Cell_conc._interval+rab_integral+RQ_integral+A rg_conc.+emission_O2+emission_CO2; 0.499, 0.793, pH+PL+AN+A N_cumulo+interval_yield+Feed_Vol+RS_cumulo+rab_integral+RQ_ integral+Accum._Yield+Arg_conc.+emission_O2+emission_CO2+in terval_O2; 0.486, 0.793, tmp_cumulo+pH+PL+PL_cumulo+AN+AN_c umulo+Feed_Vol+RS_cumulo+Cell_conc._interval+rab_integral+R Q_integral+Arg_conc.+emission_O2+emission_CO2+consum_O2; 0. 486, 0.793, pH_cumulo+PL+PL_cumulo+AN+AN_cumulo+interval_yi eld+Feed_Vol+RS_cumulo+Cell_conc._interval+rab_integral+RQ_ integral+Accum._Yield+Arg_conc.+emission_O2+emission_CO2; 0. 499, 0.793, pH+PL_cumulo+AN+interval_yield+Feed_Vol+RS_cumu lo+Cell_conc._interval+rab_integral+RQ_integral+Accum._Yiel d+Arg_conc.+emission_O2+emission_CO2+consum_O2; 0.555, 0.79 3, pH+PL_cumulo+AN_cumulo+Feed_Vol+RS_cumulo+rab_integral+A rg_conc.+emission_O2+emission_CO2; 0.523, 0.793, pH+PL+AN+F eed_Vol+RS_cumulo+Cell_conc._cumulo+rab_integral+RQ_integra 1+Arg_conc.+emission_O2+emission_CO2+consum_O2; 0.545, 0.79 3, PL_cumulo+AN_cumulo+interval_yield+Feed_Vol+RS_cumulo+ra b_integral+Accum._Yield+Arg_conc.+emission_O2+emission_CO2; 0.486, 0.793, tmp_cumulo+pH+PL_cumulo+AN+AN_cumulo+interva l_yield+Feed_Vol+RS_cumulo+rab_integral+RQ_integral+Accum._ Yield+Arg_conc.+emission_O2+emission_CO2+interval_O2; 0.486, 0.793, tmp+tmp_cumulo+pH+PL+PL_cumulo+AN+interval_yield+Fe ed_Vol+RS_cumulo+rab_integral+RQ_integral+Arg_conc.+emissio n_O2+emission_CO2+interval_O2; 0.473, 0.793, tmp+pH+PL+PL_c umulo+AN+AN_cumulo+interval_yield+Feed_Vol+RS_cumulo+Cell_c onc._interval+rab_integral+RQ_integral+Arg_conc.+emission_O 2+emission_CO2+consum_O2; 0.499, 0.793, tmp_cumulo+pH+PL_cu mulo+AN+AN_cumulo+Feed_Vol+RS_cumulo+rab_integral+RQ_integr al+Accum._Yield+Arg_conc.+emission_O2+emission_CO2+generate _CO2; 0.523, 0.793, tmp_cumulo+pH+AN+interval_yield+Feed_Vo l+RS_cumulo+rab_integral+RQ_integral+Accum._Yield+Arg_conc. +emission_O2+emission_CO2; 0.473, 0.793, tmp+pH+PL+PL_cumul o+AN+AN_cumulo+interval_yield+Feed_Vol+RS_cumulo+Cell_conc. _interval+rab_integral+RQ_integral+Arg_conc.+emission-O2+em ission_CO2+interval_O2; 0.511, 0.793, pH+pH_cumulo+AN+AN_cu mulo+interval_yield+Feed_Vol+RS_cumulo+rab_integral+RQ_inte gral+Accum._Yield+Arg_conc.+emission_O2+emission_CO2; 0.511, 0.793, tmp_cumulo+pH+PL+PL_cumulo+AN+Feed_Vol+RS_cumulo+ra b_integral+RQ_integral+Arg_conc.+emission_O2+emission-CO2+i nterval_O2; 0.499, 0.793, pH+pH_cumulo+PL+AN+AN_cumulo+inte rval_yield+Feed_Vol+RS_cumulo+Cell_conc._interval+rab-integ ral+RQ_integral+Arg_conc.+emission_O2+emission_CO2; 0.486, 0.793, tmp_cumulo+pH+PL+PL_cumulo+AN+interval_yield+Feed_Vo l+RS_cumulo+rab_integral+RQ_integral+Arg_conc.+emission_O2+ emission_CO2+consum_O2+generate_CO2; 0.473, 0.793, tmp+pH+P L+PL_cumulo+AN+AN_cumulo+interval_yield+Feed_Vol+RS_cumulo+ rab_integral+RQ_integral+Arg_conc.+emission_O2+emission_CO2 +interval_O2+generate_CO2; 0.511, 0.793, tmp_cumulo+pH+AN+A N_cumulo+Feed_Vol+RS_cumulo+Cell_conc._interval+rab_integra l+RQ_integral+Arg_conc.+emission_O2+emission_CO2+generate_C 02; 0.523, 0.793, pH+PL_cumulo+AN_cumulo+interval_yield+Fee d_Vol+RS_cumulo+rab_integral+Accum._Yield+Arg_conc.+emissio n_O2+emission_CO2+consum_O2; 0.499, 0.793, pH+PL_cumulo+AN+ interval_yield+Feed_Vol+RS_cumulo+Cell_conc._interval+rab_i ntegral+RQ_integral+Accum._Yield+Arg_conc.+emission_O2+emis sion_CO2+interval_O2; 0.511, 0.793, tmp+pH+PL_cumulo+AN+int erval_yield+Feed_Vol+RS_cumulo+Cell_conc._interval+rab_inte gral+RQ_integral+Arg_conc.+emission_O2+emission_CO2; 0.523, 0.793, pH+PL+AN+Feed_Vol+RS_cumulo+Cell_conc._interval+rab _integral+RQ_integral+Arg_conc.+emission_O2+emission_CO2+co nsum_O2; 0.499, 0.793, pH+PL+AN+AN_cumulo+Feed_Vol+RS_cumul o+rab_integral+RQ_integral+Arg_conc.+emission_O2+emission_C O2+consum_O2+interval_O2+generate_CO2; 0.473, 0.793, tmp_cu mulo+pH+PL+PL_cumulo+AN+AN_cumulo+interval_yield+Feed_Vol+R S_cumulo+Cell_conc._cumulo+rab_integral+RQ_integral+Arg_con c.+emission_O2+emission_CO2+interval_O2; 0.486, 0.793, pH+P L+PL_cumulo+AN+AN_cumulo+Feed_Vol+RS_cumulo+Cell_conc._cumu lo+rab_integral+RQ_integral+Arg_conc.+emission_O2+emission_ CO2+consum_O2+interval_O2; 0.523, 0.793, pH_cumulo+PL+PL_cu mulo+AN+interval_yield+Feed_Vol+RS_cumulo+rab_integral+RQ_i ntegral+Arg_conc.+emission_O2+emission_CO2; 0.486, 0.793, p H+pH_cumulo+PL+PL_cumulo+AN+interval_yield+Feed_Vol+RS_cumu lo+rab_integral+RQ_integral+Accum._Yield+Arg_conc.+emission _O2+emission_CO2+consum_O2; 0.523, 0.793, pH+pH_cumulo+PL+A N+Feed_Vol+RS_cumulo+rab_integral+RQ_integral+Arg_conc.+emi ssion_O2+emission_CO2+interval_O2; 0.499, 0.793, tmp+pH+PL+ AN+AN_cumulo+Feed_Vol+RS_cumulo+rab_integral+RQ_integral+Ac cum._Yield+Arg_conc.+emission_O2+emission_CO2+generate_CO2; 0.534, 0.793, pH+AN+Feed_Vol+RS_cumulo+Cell_conc._interval +rab_integral+RQ_integral+Arg_conc.+emission_O2+emission_CO 2+consum_O2; 0.499, 0.793, tmp+tmp_cumulo+pH+PL+PL_cumulo+A N+Feed_Vol+RS_cumulo+rab_integral+RQ_integral+Arg_conc.+emi ssion_O2+emission_CO2+consum_O2; 0.511, 0.793, pH+AN+AN_cum ulo+Feed_Vol+RS_cumulo+Cell_conc._interval+rab_integral+RQ_ integral+Accum._Yield+Arg_conc.+emission_O2+emission_CO2+co nsum_O2; 0.486, 0.793, pH+PL+PL_cumulo+AN+AN_cumulo+Feed_Vo l+RS_cumulo+Cell_conc._cumulo+rab_integral+RQ_integral+Arg_ conc.+emission_O2+emission_CO2+interval_O2+generate_CO2; 0. 499, 0.793, tmp_cumulo+pH+PL+PL_cumulo+AN+Feed_Vol+RS_cumul o+rab_integral+RQ_integral+Accum._Yield+Arg_conc.+emission_ O2+emission_CO2+consum_O2; 0.473, 0.793, pH+PL+PL_cumulo+AN +AN_cumulo+interval_yield+Feed_Vol+RS_cumulo+rab_integral+R Q_integral+Accum._Yield+Arg_conc.+emission_O2+emission_CO2+ consum_O2+generate_CO2; 0.511, 0.793, pH+PL+PL_cumulo+AN+Fe ed_Vol+RS_cumulo+rab_integral+RQ_integral+Accum._Yield+Arg_ conc.+emission_O2+emission_CO2+generate_CO2; 0.511, 0.793, tmp_cumulo+pH+PL+AN+interval_yield+Feed_Vol+RS_cumulo+rab_i ntegral+RQ_integral+Arg_conc.+emission_O2+emission_CO2+cons um_O2; 0.523, 0.793, pH_cumulo+AN+interval_yield+RS+Feed_Vo l+Cell_conc._interval+rab_integral+RQ_integral+Accum._Yield +Arg_conc.+emission_O2+emission_CO2; 0.499, 0.793, tmp+pH+P L_cumulo+AN+AN_cumulo+interval_yield+Feed_Vol+RS_cumulo+Cel l_conc._interval+rab_integral+RQ_integral+Arg_conc.+emissio n_O2+emission_CO2; 0.499, 0.793, pH+pH_cumulo+PL+PL_cumulo+ AN+interval_yield+Feed_Vol+RS_cumulo+rab_integral+RQ_integr al+Arg_conc.+emission_O2+emission_CO2+interval_O2; 0.523, 0. 793, pH+AN+AN_cumulo+Feed_Vol+RS_cumulo+Cell_conc._cumulo+r ab_integral+RQ_integral+Arg_conc.+emission_O2+emission_CO2+ consum_O2; 0.499, 0.793, pH+PL_cumulo+AN+AN_cumulo+Feed_Vol +RS_cumulo+rab_integral+RQ_integral+Arg_conc.+emission_O2+e mission_CO2+consum_O2+interval_O2+generate_CO2; 0.534, 0.79 3, pH+pH_cumulo+PL_cumulo+AN_cumulo+Feed_Vol+RS_cumulo+rab_ integral+RQ_integral+Arg_conc.+emission_O2+emission_CO2; 0. 499, 0.793, pH_cumulo+PL+PL_cumulo+AN+interval_yield+Feed_V ol+RS_cumulo+Cell_conc._interval+rab_integral+RQ_integral+A ccum._Yield+Arg_conc.+emission_O2+emission_CO2; 0.486, 0.79 3, tmp_cumulo+pH+PL+PL_cumulo+AN+interval_yield+Feed_Vol+RS _cumulo+Cell_conc._interval+rab_integral+RQ_integral+Arg_co nc.+emission_O2+emission_CO2+interval_O2; 0.499, 0.793, pH+ pH_cumulo+PL_cumulo+AN+interval_yield+Feed_Vol+RS_cumulo+Ce ll_conc._interval+rab_integral+RQ_integral+Accum._Yield+Arg _conc.+emission_O2+emission_CO2; 0.534, 0.793, pH+AN_cumulo +Feed_Vol+RS_cumulo+rab_integral+RQ_integral+Accum._Yield+A rg_conc.+emission_O2+emission_CO2+generate_CO2; 0.486, 0.79 3, tmp_cumulo+pH+PL+PL_cumulo+AN+AN_cumulo+Feed_Vol+RS_cumu lo+Cell_conc._cumulo+rab_integral+RQ_integral+Arg_conc.+emi ssion_O2+emission_CO2+interval_O2; 0.486, 0.793, tmp+pH_cum ulo+PL+PL_cumulo+AN+AN_cumulo+interval_yield+Feed_Vol+RS_cu mulo+rab_integral+RQ_integral+Arg_conc.+emission_O2+emissio n_CO2+interval_O2; 0.499, 0.793, tmp_cumulo+pH+PL+AN+AN_cum ulo+Feed_Vol+RS_cumulo+rab_integral+RQ_integral+Accum._Yiel d+Arg_conc.+emission_O2+emission_CO2+interval_O2; 0.544, 0. 793, pH_cumulo+PL_cumulo+AN_cumulo+Feed_Vol+RS_cumulo+rab_i ntegral+RQ_integral+Arg_conc.+emission_O2+emission_CO2 0.5 34, 0.793, PL_cumulo+AN_cumulo+interval_yield+Feed_Vol+RS_c umulo+rab_integral+Accum._Yield+Arg_conc.+emission_O2+emiss ion_CO2+interval_O2; 0.499, 0.793, pH+PL_cumulo+AN+AN_cumul o+Feed_Vol+RS_cumulo+rab_integral+RQ_integral+Accum._Yield+ Arg_conc.+emission_O2+emission_CO2+consum_O2+generate-CO2; 0.486, 0.793, tmp+tmp_cumulo+pH+PL+AN+AN_cumulo+interval_yi eld+Feed_Vol+RS_cumulo+rab_integral+RQ_integral+Accum._Yiel d+Arg_conc.+emission_O2+emission_CO2; 0.472, 0.793, pH+PL+P L_cumulo+AN+AN_cumulo+interval_yield+Feed_Vol+RS_cumulo+rab _integral+RQ_integral+Accum._Yield+Arg_conc.+emission-O2+em ission_CO2+interval_O2+generate_CO2; 0.511, 0.793, pH_cumul o+PL+PL_cumulo+AN+AN_cumulo+Feed_Vol+RS_cumulo+rab_integral +RQ_integral+Arg_conc.+emission_O2+emission_CO2+interval_O 2; 0.533, 0.793, pH+PL_cumulo+AN_cumulo+interval_yield+Feed _Vol+RS_cumulo+rab_integral+Accum._Yield+Arg_conc.+emission _O2+emission_CO2; 0.498, 0.793, pH+PL_cumulo+AN+interval_yi eld+Feed_Vol+RS_cumulo+Cell_conc._interval+rab_integral+RQ_ integral+Accum._Yield+Arg_conc.+emission_O2+emission_CO2+ge nerate_CO2; 0.472, 0.793, pH+PL+PL_cumulo+AN+AN_cumulo+inte rval_yield+Feed_Vol+RS_cumulo+rab_integral+RQ_integral+Accu m._Yield+Arg_conc.+emission_O2+emission_CO2+consum_O2+inter val_O2; 0.472, 0.793, tmp+pH+PL+PL_cumulo+AN+interval_yield +Feed_Vol+RS_cumulo+Cell_conc._interval+rab_integral+RQ_int egral+Accum._Yield+Arg_conc.+emission_O2+emission_CO2+inter val_O2; 0.486, 0.793, tmp+pH+PL+PL_cumulo+AN+interval_yield +Feed_Vol+RS_cumulo+rab_integral+RQ_integral+Arg_conc.+emis sion_O2+emission_CO2+consum_O2+interval_O2; 0.522, 0.793, p H+pH_cumulo+PL+AN+Feed_Vol+RS_cumulo+rab_integral+RQ_integr al+Arg_conc.+emission_O2+emission_CO2+consum_O2; 0.533, 0.7 93, pH+PL_cumulo+AN_cumulo+Feed_Vol+RS_cumulo+rab_integral+ RQ_integral+Arg_conc.+emission_O2+emission_CO2+consum-O2; 0. 486, 0.793, tmp_cumulo+pH+AN+AN_cumulo+interval_yield+Feed_ Vol+RS_cumulo+Cell_conc._interval+rab_integral+RQ_integral+ Accum._Yield+Arg_conc.+emission_O2+emission_CO2+generate_CO 2; 0.485, 0.793, tmp_cumulo+pH+PL+PL_cumulo+AN+AN_cumulo+in terval_yield+Feed_Vol+RS_cumulo+Cell_conc._interval+rab_int egral+RQ_integral+Arg_conc.+emission_O2+emission_CO2; 0.544, 0.793, pH+AN_cumulo+Feed_Vol+RS_cumulo+Cell_conc._cumulo+r ab_integral+RQ_integral+Arg_conc.+emission_O2+emission_CO2; 0.533, 0.793, pH_cumulo+AN+AN_cumulo+Feed_Vol+RS_cumulo+ra b_integral+RQ_integral+Accum._Yield+Arg_conc.+emission_O2+e mission_CO2; 0.522, 0.793, pH+PL+AN+Feed_Vol+RS_cumulo+Cell _conc._cumulo+rab_integral+RQ_integral+Arg_conc.+emission_O 2+emission_CO2+interval_O2; 0.511, 0.793, pH+AN+AN_cumulo+F eed_Vol+RS_cumulo+rab_integral+RQ_integral+Accum._Yield+Arg _conc.+emission_O2+emission_CO2+consum_O2+generate_CO2; 0.5 11, 0.793, tmp_cumulo+pH+AN+AN_cumulo+interval_yield+Feed_V ol+RS_cumulo+rab_integral+RQ_integral+Arg_conc.+emission_O2 +emission_CO2+generate_CO2; 0.485, 0.793, pH+PL+AN+AN_cumul o+interval_yield+Feed_Vol+RS_cumulo+Cell_conc._interval+rab _integral+RQ_integral+Accum._Yield+Arg_conc.+emission_O2+em ission_CO2+interval_O2; 0.498, 0.793, tmp_cumulo+pH+PL+AN+A N_cumulo+interval_yield+Feed_Vol+RS_cumulo+rab_integral+RQ_ integral+Arg_conc.+emission_O2+emission_CO2+generate_CO2; 0. 533, 0.793, pH+pH_cumulo+PL_cumulo+AN+Feed_Vol+RS_cumulo+ra b_integral+RQ_integral+Arg_conc.+emission_O2+emission_CO2; 0.510, 0.793, pH+pH_cumulo+PL+AN+AN_cumulo+Feed_Vol+RS_cumu lo+Cell_conc._interval+rab_integral+RQ_integral+Arg_conc.+e mission_O2+emission_CO2; 0.510, 0.793, tmp+pH+AN+AN_cumulo+ Feed_Vol+RS_cumulo+Cell_conc._interval+rab_integral+RQ_inte gral+Arg_conc.+emission_O2+emission_CO2+generate_CO2; 0.485, 0.793, tmp+pH+PL+PL_cumulo+AN+AN_cumulo+Feed_Vol+RS_cumulo +Cell_conc._interval+rab_integral+RQ_integral+Arg_conc.+emi ssion_O2+emission_CO2+consum_O2; 0.485, 0.793, tmp+pH+PL+PL _cumulo+AN+AN_cumulo+interval_yield+Feed_Vol+RS_cumulo+rab_ integral+RQ_integral+Arg_conc.+emission_O2+emission_CO2+gen erate_CO2; 0.522, 0.793, pH+PL+AN+Feed_Vol+RS_cumulo+rab_in tegral+RQ_integral+Accum._Yield+Arg_conc.+emission_O2+emiss ion_CO2+consum_O2; 0.522, 0.793, pH+pH_cumulo+PL+AN+Feed_Vo l+RS_cumulo+rab_integral+RQ_integral+Accum._Yield+Arg_conc. +emission_O2+emission_CO2; 0.498, 0.793, tmp+tmp_cumulo+pH+ AN+AN_cumulo+interval_yield+Feed_Vol+RS_cumulo+rab_integral +RQ_integral+Accum._Yield+Arg_conc.+emission_O2+emission_CO 2; 0.510, 0.793, tmp+pH+PL+PL_cumulo+AN+Feed_Vol+RS_cumulo+ rab_integral+RQ_integral+Arg_conc.+emission_O2+emission_CO2 +generate_CO2; 0.498, 0.793, pH+PL+AN+AN_cumulo+interval_yi eld+Feed_Vol+RS_cumulo+rab_integral+RQ_integral+Accum._Yiel d+Arg_conc.+emission_O2+emission_CO2+generate_CO2; 0.522, 0. 793, pH+PL_cumulo+AN+Feed_Vol+RS_cumulo+rab_integral+RQ_int egral+Accum.-Yield+Arg-conc.+emission-O2+emission-CO2+gener ate_CO2; 0.498, 0.793, pH+PL+AN+AN_cumulo+interval_yield+Fe ed_Vol+RS_cumulo+Cell _conc. _interval+rab_integral+RQ_integr al+Arg_conc.+emission_O2+emission_CO2+generate_CO2; 0.498, 0.793, tmp_cumulo+pH+PL_cumulo+AN+AN_cumulo+interval_yield+ Feed_Vol+RS_cumulo+rab_integral+RQ_integral+Arg_conc.+emiss ion_O2+emission_CO2+consum_O2; 0.522, 0.793, pH+PL+AN+Feed_ Vol+RS_cumulo+rab_integral+RQ_integral+Arg_conc.+emission_O 2+emission_CO2+consum_O2+generate_CO2; 0.533, 0.793, pH+PL+ AN+AN_cumulo+RS+Feed_Vol+rab_integral+RQ_integral+Arg_conc. +emission_O2+emission_CO2; 0.498, 0.793, tmp_cumulo+pH+PL_c umulo+AN+AN_cumulo+interval_yield+Feed_Vol+RS_cumulo+Cell_c one._interval+rab_integral+RQ_integral+Arg_conc.+emission_O 2+emission_CO2; 0.510, 0.793, tmp_cumulo+pH+pH_cumulo+AN+AN _cumulo+Feed_Vol+RS_cumulo+rab_integral+RQ_integral+Accum._ Yield+Arg_conc.+emission_O2+emission_CO2; 0.498, 0.793, tmp _cumulo+pH+PL_cumulo+AN+AN_cumulo+Feed_Vol+RS_cumulo+rab_in tegral+RQ_integral+Accum._Yield+Arg_conc.+emission_O2+emiss ion_CO2+consum_O2; 0.510, 0.793, pH+AN+interval_yield+RS+Fe ed_Vol+Cell_conc._interval+rab_integral+RQ_integral+Accum._ Yield+Arg_conc.+emission_O2+emission_CO2+interval_O2; 0.510, 0.792, tmp+tmp_cumulo+pH+AN+AN_cumulo+Feed_Vol+RS_cumulo+r ab_integral+RQ_integral+Arg_conc.+emission_O2+emission_CO2+ consum_O2; 0.498, 0.792, tmp+pH+AN+interval_yield+Feed_Vol+ RS_cumulo+Cell_conc._interval+rab_integral+RQ_integral+Accu m._Yield+Arg_conc.+emission_O2+emission_CO2+generate_CO2; 0. 498, 0.792, tmp_cumulo+pH+PL_cumulo+AN+AN_cumulo+Feed_Vol+R S_cumulo+rab_integral+RQ_integral+Accum._Yield+Arg_conc.+em ission_O2+emission_CO2+interval_O2; 0.522, 0.792, pH+PL_cum ulo+AN_cumulo+Feed_Vol+RS_cumulo+rab_integral+RQ_integral+A ccum._Yield+Arg_conc.+emission_O2+emission_CO2+interval_O2; 0.498, 0.792, pH+PL_cumulo+AN+AN_cumulo+interval_yield+Fee d_Vol+RS_cumulo+Cell_conc._interval+rab_integral+RQ_integra l+Arg_conc.+emission_O2+emission_CO2+generate_CO2; 0.498, 0. 792, pH+PL_cumulo+AN+AN_cumulo+interval_yield+Feed_Vol+RS_c umulo+rab_integral+RQ_integral+Arg_conc.+emission_O2+emissi on_CO2+consum_O2+generate_CO2; 0.485, 0.792, tmp+pH+AN+AN_c umulo+interval_yield+Feed_Vol+RS_cumulo+Cell_conc._interval +rab_integral+RQ_integral+Accum._Yield+Arg_conc.+emission_O 2+emission_CO2+generate_CO2; 0.498, 0.792, tmp_cumulo+pH+PL +AN+interval_yield+Feed_Vol+RS_cumulo+Cell_conc._interval+r ab_integral+RQ_integral+Arg_conc.+emission_O2+emission_CO2+ interval_O2; 0.510, 0.792, pH+pH_cumulo+PL_cumulo+AN+AN_cum ulo+Feed_Vol+RS_cumulo+Cell_conc._interval+rab_integral+RQ_ integral+Arg_conc.+emission_O2+emission_CO2; 0.533, 0.792, pH+pH_cumulo+AN+interval_yield+Feed_Vol+RS_cumulo+rab_integ ral+RQ_integral+Arg_conc.+emission_O2+emission_CO2; 0.485, 0.792, pH+PL+PL_cumulo+AN+AN_cumulo+Feed_Vol+RS_cumulo+rab_ integral+RQ_integral+Accum._Yield+Arg_conc.+emission_O2+emi ssion_CO2+interval_O2+generate_CO2; 0.498, 0.792, tmp+pH+PL +AN+interval_yield+Feed_Vol+RS_cumulo+Cell_conc._interval+r ab_integral+RQ_integral+Arg_conc.+emission_O2+emission_CO2+ interval_O2; 0.485, 0.792, tmp_cumulo+pH+AN+AN_cumulo+inter val_yield+Feed_Vol+RS_cumulo+Cell_conc._interval+rab_integr al+RQ_integral+Accum._Yield+Arg_conc.+emission_O2+emission_ CO2+interval_O2; 0.485, 0.792, tmp+pH+PL_cumulo+AN+AN_cumul o+interval_yield+Feed_Vol+RS_cumulo+rab_integral+RQ_integra l+Accum._Yield+Arg_conc.+emission_O2+emission_CO2+interval_ 02; 0.471, 0.792, pH+PL+PL_cumulo+AN+AN_cumulo+interval_yie ld+Feed_Vol+RS_cumulo+rab_integral+RQ_integral+Arg_conc.+em ission_O2+emission_CO2+consum_O2+interval_O2+generate_CO2; 0.521, 0.792, pH+PL+AN+interval_yield+Feed_Vol+RS_cumulo+ra b_integral+RQ_integral+Arg_conc.+emission_O2+emission_CO2+c onsum_O2; 0.497, 0.792, tmp+tmp_cumulo+pH+PL+PL_cumulo+AN+i nterval_yield+Feed_Vol+RS_cumulo+rab_integral+RQ_integral+A rg_conc.+emission_O2+emission_CO2; 0.497, 0.792, pH+PL_cumu lo+AN+AN_cumulo+Feed_Vol+RS_cumulo+Cell_conc._interval+rab_ integral+RQ_integral+Accum._Yield+Arg_conc.+emission_O2+emi ssion_CO2+generate_CO2; 0.485, 0.792, pH_cumulo+PL_cumulo+A N+AN_cumulo+interval_yield+Feed_Vol+RS_cumulo+Cell_conc._in terval+rab_integral+RQ_integral+Accum._Yield+Arg_conc.+emis sion_O2+emission_CO2+generate_CO2; 0.521, 0.792, pH+AN+Feed _Vol+RS_cumulo+Cell_conc._interval+rab_integral+RQ_integral +Accum._Yield+Arg_conc.+emission_O2+emission_CO2+generate_C 02; 0.510, 0.792, tmp_cumulo+pH+pH_cumulo+PL+AN+interval_yi eld+Feed_Vol+RS_cumulo+rab_integral+RQ_integral+Arg_conc.+e mission_O2+emission_CO2; 0.521, 0.792, pH_cumulo+PL+AN+AN_c umulo+interval_yield+Feed_Vol+RS_cumulo+rab_integral+RQ_int egral+Arg_conc.+emission_O2+emission_CO2; 0.497, 0.792, pH+ PL_cumulo+AN+AN_cumulo+interval_yield+Feed_Vol+RS_cumulo+ra b_integral+RQ_integral+Arg_conc.+emission_O2+emission_CO2+c onsum_O2+interval_O2; 0.510, 0.792, pH+pH_cumulo+AN+AN_cumu lo+Feed_Vol+RS_cumulo+rab_integral+RQ_integral+Accum._Yield +Arg_conc.+emission_O2+emission_CO2+interval_O2; 0.497, 0.7 92, tmp+pH+PL+AN+AN_cumulo+Feed_Vol+RS_cumulo+rab_integral+ RQ_integral+Accum._Yield+Arg_conc.+emission_O2+emission_CO2 +interval_O2; 0.510, 0.792, pH+PL+PL_cumulo+AN+Feed_Vol+RS_ cumulo+rab_integral+RQ_integral+Accum._Yield+Arg_conc.+emis sion_O2+emission_CO2+interval_O2; 0.543, 0.792, pH+AN+RS+Fe ed_Vol+Cell_conc._interval+rab_integral+RQ_integral+Arg_con c.+emission_O2+emission_CO2; 0.471, 0.792, tmp+tmp_cumulo+p H+PL+PL_cumulo+AN+interval_yield+Feed_Vol+RS_cumulo+rab_int egral+RQ_integral+Accum._Yield+Arg_conc.+emission_O2+emissi on_CO2+consum_O2; 0.521, 0.792, tmp_cumulo+pH+PL+AN+Feed_Vo l+RS_cumulo+Cell_conc._cumulo+rab_integral+RQ_integral+Arg_ conc.+emission_O2+emission_CO2; 0.510, 0.792, pH_cumulo+PL+ PL_cumulo+AN+AN_cumulo+Feed_Vol+RS_cumulo+rab_integral+RQ_i ntegral+Arg_conc.+emission_O2+emission_CO2+consum_O2; 0.521, 0.792, pH+PL_cumulo+AN_cumulo+interval_yield+Feed_Vol+RS_c umulo+rab_integral+Accum._Yield+Arg_conc.+emission_O2+emiss ion_CO2+interval_O2; 0.532, 0.792, pH+PL+AN+interval_yield+ RS+Feed_Vol+rab_integral+RQ_integral+Arg_conc.+emission_O2+ emission_CO2; 0.497, 0.792, pH+pH_cumulo+PL+AN+AN_cumulo+in terval_yield+Feed_Vol+RS_cumulo+rab_integral+RQ_integral+Ac cum._Yield+Arg_conc.+emission_O2+emission_CO2; 0.497, 0.792, pH+PL+PL_cumulo+AN+AN_cumulo+interval_yield+Feed_Vol+RS_cu mulo+rab_integral+Accum._Yield+Arg_conc.+emission_02+emissi on_CO2+consum_O2; 0.484, 0.792, pH+pH_cumulo+PL+PL_cumulo+A N+interval_yield+Feed_Vol+RS_cumulo+Cell_conc._interval+rab _integral+RQ_integral+Accum._Yield+Arg_conc.+emission-O2+em ission_CO2; 0.510, 0.792, pH+pH_cumulo+PL_cumulo+AN+AN_cumu 10+Feed_Vol+RS_cumulo+rab_integral+RQ_integral+Arg_conc.+em ission_O2+emission_CO2+generate_CO2; 0.521, 0.792, pH+PL_cu mulo+AN+Feed_Vol+RS_cumulo+Cell_conc._interval+rab_integral +RQ_integral+Accum._Yield+Arg_conc.+emission_O2+emission_CO 2; 0.497, 0.792, pH+PL_cumulo+AN+AN_cumulo+interval_yield+F eed_Vol+RS_cumulo+rab_integral+RQ_integral+Arg_conc.+emissi on_O2+emission_CO2+interval_O2+generate_CO2; 0.543, 0.792, pH+PL+AN+RS+Feed_Vol+rab_integral+RQ_integral+Arg_conc.+emi ssion_O2+emission_CO2; 0.497, 0.792, tmp_cumulo+pH_cumulo+A N+AN_cumulo+interval_yield+Feed_Vol+RS_cumulo+Cell_conc._in terval+rab_integral+RQ_integral+Accum._Yield+Arg_conc.+emis sion_O2+emission_CO2; 0.484, 0.792, tmp_cumulo+pH+PL+PL_cum ulo+AN+interval_yield+Feed_Vol+RS_cumulo+Cell_conc._interva l+rab_integral+RQ_integral+Accum._Yield+Arg_conc.+emission_ O2+emission_CO2; 0.509, 0.792, pH+PL+PL_cumulo+AN+AN_cumulo +interval_yield+Feed_Vol+RS_cumulo+rab_integral+Arg_conc.+e mission_O2+emission_CO2+consum_O2; 0.497, 0.792, tmp_cumulo +pH+PL_cumulo+AN+interval_yield+Feed_Vol+RS_cumulo+Cell_con c._interval+rab_integral+RQ_integral+Accum._Yield+Arg_conc. +emission_O2+emission_CO2; 0.509, 0.792, pH+AN+AN_cumulo+in terval_yield+Feed_Vol+RS_cumulo+Cell_conc._interval+rab_int egral+RQ_integral+Arg_conc.+emission_O2+emission_CO2+consum _O2; 0.484, 0.792, pH_cumulo+PL+PL_cumulo+AN+AN_cumulo+inte rval_yield+Feed_Vol+RS_cumulo+rab_integral+RQ_integral+Accu m._Yield+Arg_conc.+emission_O2+emission_CO2+interval_O2; 0. 509, 0.792, tmp+pH+AN+AN_cumulo+interval_yield+Feed_Vol+RS_ cumulo+rab_integral+RQ_integral+Arg_conc.+emission_02+emiss ion_CO2+interval_O2; 0.509, 0.792, tmp+pH+PL+AN+interval_yi eld+Feed_Vol+RS_cumulo+rab_integral+RQ_integral+Arg_conc.+e mission_O2+emission_CO2+interval_O2; 0.521, 0.792, pH+PL+AN +Feed_Vol+RS_cumulo+rab_integral+RQ_integral+Arg_conc.+emis sion_O2+emission_CO2+interval_O2+generate_CO2; 0.521, 0.792, tmp+tmp_cumulo+pH+AN+interval_yield+Feed_Vol+RS_cumulo+rab _integral+RQ_integral+Arg_conc.+emission_O2+emission_CO2; 0. 484, 0.792, tmp+pH+AN+AN_cumulo+interval_yield+Feed_Vol+RS_ cumulo+Cell_conc._interval+rab_integral+RQ_integral+Accum._ Yield+Arg_conc.+emission_O2+emission_CO2+interval_O2; 0.521, 0.792, pH+PL+AN+Feed_Vol+RS_cumulo+Cell_conc._cumulo+rab_i ntegral+RQ_integral+Arg_conc.+emission_O2+emission_CO2+gene rate_CO2; 0.497, 0.792, tmp+pH+PL_cumulo+AN+AN_cumulo+Feed_ Vol+RS_cumulo+rab_integral+RQ_integral+Accum._Yield+Arg_con c.+emission_O2+emission_CO2+consum_O2; 0.497, 0.792, tmp_cu mulo+pH+AN+interval_yield+Feed_Vol+RS_cumulo+Cell_conc._int erval+rab_integral+RQ_integral+Accum._Yield+Arg_conc.+emiss ion_O2+emission_CO2+interval_O2; 0.470, 0.792, tmp+pH+PL+PL cumulo+AN+AN cumulo+interval yield+Feed Vol+RS cumulo+Cell _conc._cumulo+rab_integral+RQ_integral+Arg_conc.+emission_O 2+emission_CO2+consum_O2; 0.484, 0.792, pH+PL+PL_cumulo+AN+ AN_cumulo+Feed_Vol+RS_cumulo+rab_integral+RQ_integral+Accum. _Yield+Arg_conc.+emission_O2+emission_CO2+consum_O2+generat e_CO2; 0.497, 0.792, tmp_cumulo+pH+pH_cumulo+PL+AN+AN_cumul o+Feed_Vol+RS_cumulo+rab_integral+RQ_integral+Arg_conc.+emi ssion_O2+emission_CO2+interval_O2; 0.521, 0.792, pH+PL_cumu lo+AN_cumulo+Feed_Vol+RS_cumulo+rab_integral+RQ_integral+Ac cum._Yield+Arg_conc.+emission_O2+emission_CO2+generate_CO2; 0.509, 0.792, tmp+tmp_cumulo+pH+AN_cumulo+interval_yield+F eed_Vol+RS_cumulo+rab_integral+RQ_integral+Accum._Yield+Arg _conc.+emission_O2+emission_CO2; 0.532, 0.792, tmp_cumulo+p H+AN_cumulo+interval_yield+Feed_Vol+RS_cumulo+rab_integral+ RQ_integral+Arg_conc.+emission_O2+emission_CO2; 0.497, 0.79 2, tmp+pH_cumulo+AN+AN_cumulo+interval_yield+Feed_Vol+RS_cu mulo+Cell_conc._interval+rab_integral+RQ_integral+Accum._Yi eld+Arg_conc.+emission_O2+emission_CO2; 0.509, 0.792, tmp_c umulo+pH+AN+AN_cumulo+Feed_Vol+RS_cumulo+rab_integral+RQ_in tegral+Accum._Yield+Arg_conc.+emission_O2+emission_CO2+cons um_O2; 0.509, 0.792, tmp_cumulo+pH_cumulo+PL_cumulo+AN+AN_c umulo+Feed_Vol+RS_cumulo+rab_integral+RQ_integral+Accum._Yi eld+Arg_conc.+emission_O2+emission_CO2; 0.521, 0.792, tmp+p H+PL+AN+Feed_Vol+RS_cumulo+Cell_conc._cumulo+rab_integral+R Q_integral+Arg_conc.+emission_O2+emission_CO2; 0.509, 0.792, tmp_cumulo+pH+PL+PL_cumulo+AN+Feed_Vol+RS_cumulo+rab_integ ral+RQ_integral+Accum._Yield+Arg_conc.+emission_O2+emission _CO2; 0.497, 0.792, pH_cumulo+PL_cumulo+AN+AN_cumulo+interv al_yield+Feed_Vol+RS_cumulo+rab_integral+RQ_integral+Accum. _Yield+Arg_conc.+emission_O2+emission_CO2+interval_O2; 0.53 2, 0.792, pH+pH_cumulo+AN_cumulo+Feed_Vol+RS_cumulo+rab_int egral+RQ_integral+Accum._Yield+Arg_conc.+emission_O2+emissi on_CO2; 0.497, 0.792, pH+pH_cumulo+PL_cumulo+AN+AN_cumulo+i nterval_yield+Feed_Vol+RS_cumulo+rab_integral+RQ_integral+A rg_conc.+emission_O2+emission_CO2+interval_O2; 0.532, 0.792, tmp+pH+AN_cumulo+interval_yield+Feed_Vol+RS_cumulo+rab_int egral+RQ_integral+Arg_conc.+emission_O2+emission_CO2; 0.509, 0.792, tmp_cumulo+pH+AN+AN_cumulo+Feed_Vol+RS_cumulo+Cell_ conc._interval+rab_integral+RQ_integral+Arg_conc.+emission_ O2+emission_CO2+interval_O2; 0.521, 0.792, pH+PL_cumulo+AN+ interval_yield+Feed_Vol+RS_cumulo+rab_integral+RQ_integral+ Arg_conc.+emission_O2+emission_CO2+generate_CO2; 0.532, 0.7 92, pH_cumulo+AN+AN_cumulo+Feed_Vol+RS_cumulo+rab_integral+ RQ_integral+Arg_conc.+emission_O2+emission_CO2+interval_O2; 0.496, 0.792, tmp_cumulo+pH_cumulo+PL_cumulo+AN+AN_cumulo+ interval_yield+Feed_Vol+RS_cumulo+rab_integral+RQ_integral+ Accum. _Yield+Arg_conc.+emission_O2+emission_CO2; 0.520, 0.7 92, tmp+pH+AN+interval_yield+Feed_Vol+RS_cumulo+rab_integra l+RQ_integral+Accum._Yield+Arg_conc.+emission_O2+emission_C 02; 0.470, 0.792, pH+pH_cumulo+PL+PL_cumulo+AN+AN_cumulo+in terval_yield+Feed_Vol+RS_cumulo+Cell_conc._interval+rab_int egral+RQ_integral+Arg_conc.+emission_O2+emission_CO2+interv al_O2; 0.532, 0.792, pH_cumulo+AN+interval_yield+RS+Feed_Vo l+Cell_conc._interval+rab_integral+RQ_integral+Arg_conc.+em ission_O2+emission_CO2; 0.483, 0.792, tmp+pH+PL+AN+interval _yield+Feed_Vol+RS_cumulo+Cell_conc._interval+rab_integral+ RQ_integral+Accum._Yield+Arg_conc.+emission_O2+emission_CO2 +interval_O2; 0.509, 0.792, tmp+tmp_cumulo+pH+AN+AN_cumulo+ Feed_Vol+RS_cumulo+Cell_conc._cumulo+rab_integral+RQ_integr al+Arg_conc.+emission_O2+emission_CO2; 0.520, 0.792, pH+PL_ cumulo+AN_cumulo+interval_yield+Feed_Vol+RS_cumulo+rab_inte gral+RQ_integral+Arg_conc.+emission_O2+emission_CO2+interva l_O2; 0.496, 0.792, tmp+pH+PL_cumulo+AN+AN_cumulo+interval_ yield+Feed_Vol+RS_cumulo+rab_integral+RQ_integral+Arg_conc. +emission_O2+emission_CO2+consum_O2; 0.496, 0.792, tmp_cumu lo+pH+pH_cumulo+PL+PL_cumulo+AN+interval_yield+Feed_Vol+RS_ cumulo+Accum._Yield+Arg_conc.+emission_O2+emission_CO2+cons um_O2; 0.520, 0.792, pH+pH_cumulo+PL_cumulo+AN_cumulo+Feed_ Vol+RS_cumulo+Cell_conc._cumulo+rab_integral+RQ_integral+Ar g_conc.+emission_O2+emission_CO2; 0.496, 0.792, tmp_cumulo+ pH+PL+AN+AN_cumulo+interval_yield+Feed_Vol+RS_cumulo+rab_in tegral+RQ_integral+Arg_conc.+emission_O2+emission_CO2+consu m_O2; 0.483, 0.792, pH+PL+AN+AN_cumulo+interval_yield+Feed_ Vol+RS_cumulo+Cell_conc._interval+rab_integral+RQ_integral+ Accum._Yield+Arg_conc.+emission_O2+emission_CO2+generate_CO 2; 0.520, 0.792, tmp+pH+pH_cumulo+PL+AN+Feed_Vol+RS_cumulo+ rab_integral+RQ_integral+Arg_conc.+emission_O2+emission_CO 2; 0.520, 0.792, pH+PL+AN+Feed_Vol+RS_cumulo+Cell_conc._cum ulo+rab_integral+RQ_integral+Accum._Yield+Arg_conc.+emissio n_O2+emission_CO2; 0.470, 0.792, tmp_cumulo+pH+pH_cumulo+PL +PL_cumulo+AN+interval_yield+Feed_Vol+RS_cumulo+rab_integra l+RQ_integral+Accum._Yield+Arg_conc.+emission_O2+emission_C O2+consum_O2; 0.496, 0.792, tmp+pH+PL_cumulo+AN+AN_cumulo+F eed_Vol+RS_cumulo+rab_integral+RQ_integral+Accum._Yield+Arg _conc.+emission_O2+emission_CO2+interval_O2; 0.483, 0.792, tmp_cumulo+pH+PL+PL_cumulo+AN+AN_cumulo+interval_yield+Feed _Vol+RS_cumulo+rab_integral+RQ_integral+Arg_conc.+emission_ O2+emission_CO2+generate_CO2; 0.520, 0.792, tmp+pH+PL_cumul o+AN+Feed_Vol+RS_cumulo+rab_integral+RQ_integral+Accum._Yie ld+Arg_conc.+emission_O2+emission_CO2; 0.531, 0.792, pH+AN_ cumulo+Feed_Vol+RS_cumulo+rab_integral+RQ_integral+Accum._Y ield+Arg_conc.+emission_O2+emission_CO2+interval_O2; 0.531, 0.792, pH+PL_cumulo+AN+Feed_Vol+RS_cumulo+Cell_conc._cumul o+rab_integral+RQ_integral+Arg_conc.+emission_O2+emission_C 02; 0.496, 0.792, pH+PL_cumulo+AN+AN_cumulo+interval_yield+ Feed_Vol+RS_cumulo+Cell_conc._interval+rab_integral+RQ_inte gral+Arg_conc.+emission_O2+emission_CO2+consum_O2; 0.496, 0. 792, tmp_cumulo+pH_cumulo+PL_cumulo+AN+AN_cumulo+interval_y ield+Feed_Vol+RS_cumulo+Cell_conc._interval+rab_integral+RQ _integral+Arg_conc.+emission_O2+emission_CO2; 0.483, 0.792, pH+pH_cumulo+PL+PL_cumulo+AN+AN_cumulo+Feed_Vol+RS_cumulo+ rab_integral+RQ_integral+Accum._Yield+Arg_conc.+emission_O2 +emission_CO2+consum_O2; 0.483, 0.792, tmp+pH+PL+PL_cumulo+ AN+AN_cumulo+Feed_Vol+RS_cumulo+Cell_conc._cumulo+rab_integ ral+RQ_integral+Arg_conc.+emission_O2+emission_CO2+interval _O2; 0.496, 0.792, tmp+pH+pH_cumulo+PL+PL_cumulo+AN+interva l_yield+Feed_Vol+RS_cumulo+rab_integral+RQ_integral+Arg_con c.+emission_O2+emission_CO2; 0.508, 0.792, tmp_cumulo+pH+AN +AN_cumulo+Feed_Vol+RS_cumulo+rab_integral+RQ_integral+Arg_ conc.+emission_O2+emission_CO2+consum_O2+interval_02; 0.496, 0.792, pH+PL+PL_cumulo+AN+AN_cumulo+Feed_Vol+RS_cumulo+Cel l_conc._cumulo+Cell_conc._interval+rab_integral+RQ_integral +Arg_conc.+emission_O2+emission_CO2; 0.542, 0.792, pH+AN_cu mulo+Feed_Vol+RS_cumulo+Cell_conc._interval+rab_integral+RQ _integral+Arg_conc.+emission_O2+emission_CO2; 0.531, 0.792, pH+AN+AN_cumulo+interval_yield+RS+Feed_Vol+rab_integral+RQ _integral+Arg_conc.+emission_O2+emission_CO2; 0.496, 0.792, tmp+pH+pH_cumulo+PL+AN+AN_cumulo+interval_yield+Feed_Vol+R S_cumulo+rab_integral+RQ_integral+Arg_conc.+emission_O2+emi ssion_CO2; 0.483, 0.792, pH+pH_cumulo+PL+AN+AN_cumulo+inter val_yield+Feed_Vol+RS_cumulo+Cell_conc._interval+rab_integr al+RQ_integral+Accum._Yield+Arg_conc.+emission_O2+emission_ CO2; 0.542, 0.792, pH+AN_cumulo+Feed_Vol+RS_cumulo+rab_inte gral+RQ_integral+Arg_conc.+emission_O2+emission_CO2+consum-02; 0.508, 0.792, tmp+pH+AN+interval_yield+Feed_Vol+RS_cumu lo+Cell_conc._interval+rab_integral+RQ_integral+Arg_conc.+e mission_O2+emission_CO2+interval_O2; 0.496, 0.792, pH_cumul o+PL_cumulo+AN+AN_cumulo+interval_yield+Feed_Vol+RS_cumulo+ Cell_conc._interval+rab_integral+RQ_integral+Arg_conc.+emis sion_O2+emission_CO2+generate_CO2; 0.496, 0.792, pH_cumulo+ PL_cumulo+AN+AN_cumulo+interval_yield+Feed_Vol+RS_cumulo+Ce ll_conc._interval+rab_integral+RQ_integral+Arg_conc.+emissi on_O2+emission_CO2+interval_O2; 0.508, 0.791, tmp+pH+AN+AN_ cumulo+Feed_Vol+RS_cumulo+Cell_conc._interval+rab_integral+ RQ_integral+Arg_conc.+emission_O2+emission_CO2+interval_O2; 0.531, 0.791, tmp_cumulo+pH+AN+Feed_Vol+RS_cumulo+rab_inte gral+RQ_integral+Arg_conc.+emission_O2+emission_CO2+consum_ 02; 0.496, 0.791, tmp+tmp_cumulo+pH+AN+interval_yield+Feed_ Vol+RS_cumulo+Cell_conc._interval+rab_integral+RQ_integral+ Accum._Yield+Arg_conc.+emission_O2+emission_CO2; 0.483, 0.7 91, pH+PL_cumulo+AN+AN_cumulo+interval_yield+Feed_Vol+RS_cu mulo+rab_integral+RQ_integral+Accum._Yield+Arg_conc.+emissi on_O2+emission_CO2+interval_O2+generate_CO2; 0.496, 0.791, tmp_cumulo+pH+PL+AN+AN_cumulo+interval_yield+Feed_Vol+RS_cu mulo+Cell_conc._cumulo+rab_integral+RQ_integral+Arg_conc.+e mission_O2+emission_CO2; 0.520, 0.791, pH_cumulo+PL+AN+inte rval_yield+Feed_Vol+RS_cumulo+Cell_conc._interval+rab_integ ral+RQ_integral+Arg-conc.+emission_O2+emission_CO2; 0.496, 0.791, tmp_cumulo+pH+pH_cumulo+PL+AN+AN_cumulo+Feed_Vol+RS_ cumulo+rab_integral+RQ_integral+Accum._Yield+Arg_conc.+emis sion_O2+emission_CO2; 0.483, 0.791, tmp_cumulo+pH+PL+PL_cum ulo+AN+interval_yield+Feed_Vol+RS_cumulo+rab_integral+RQ_in tegral+Arg_conc.+emission_O2+emission_CO2+consum_O2+interva 1_02; 0.508, 0.791, pH+AN+AN_cumulo+interval_yield+RS+Feed_ Vol+Cell_conc._interval+rab_integral+RQ_integral+Accum._Yie ld+Arg_conc.+emission_O2+emission_CO2; 0.508, 0.791, pH+AN+ AN_cumulo+Feed_Vol+RS_cumulo+Cell_conc._interval+rab_integr al+RQ_integral+Arg_conc.+emission_O2+emission_CO2+consum_O2 +interval_O2; 0.508, 0.791, tmp+pH+AN+AN_cumulo+interval_yi eld+Feed_Vol+RS_cumulo+rab_integral+RQ_integral+Arg_conc.+e mission_O2+emission_CO2+generate_CO2; 0.483, 0.791, tmp_cum ulo+pH+pH_cumulo+PL+PL_cumulo+AN+AN_cumulo+Feed_Vol+RS_cumu lo+rab_integral+RQ_integral+Arg_conc.+emission_O2+emission CO2+interval_O2; 0.519, 0.791, pH+PL_cumulo+AN_cumulo+Feed_ Vol+RS_cumulo+Cell_conc._cumulo+rab_integral+RQ_integral+Ac cum._Yield+Arg_conc.+emission_O2+emission_CO2; 0.482, 0.791, pH_cumulo+PL_cumulo+AN+AN_cumulo+interval_yield+Feed_Vol+R S_cumulo+Cell_conc._interval+rab_integral+RQ_integral+Accum. _Yield+Arg_conc.+emission_O2+emission_CO2+consum _O2; 0.531, 0.791, pH+AN+Feed_Vol+RS_cumulo+rab_integral+RQ_integral+A rg_conc.+emission_O2+emission_CO2+interval_O2+generate_CO2; 0.508, 0.791, pH+AN+AN_cumulo+Feed_Vol+RS_cumulo+Cell_conc. _interval+rab_integral+RQ_integral+Arg_conc.+emission_O2+em ission_CO2+interval_O2+generate_CO2; 0.519, 0.791, tmp+pH+A N_cumulo+interval_yield+Feed_Vol+RS_cumulo+rab_integral+RQ_ integral+Accum._Yield+Arg_conc.+emission_O2+emission_CO2; 0. 508, 0.791, pH+PL+AN+AN_cumulo+Feed_Vol+RS_cumulo+Cell_conc. _cumulo+Cell_conc._interval+rab_integral+RQ_integral+Arg_co nc.+emission_O2+emission_CO2; 0.519, 0.791, pH+AN+Feed_Vol+ RS_cumulo+Cell_conc._interval+rab_integral+RQ_integral+Accu m._Yield+Arg_conc.+emission_O2+emission_CO2+interval_O2; 0. 519, 0.791, tmp_cumulo+pH+AN_cumulo+interval_yield+Feed_Vol +RS_cumulo+rab_integral+RQ_integral+Accum._Yield+Arg_conc.+ emission_O2+emission_CO2; 0.482, 0.791, tmp+tmp_cumulo+pH+A N+AN_cumulo+interval_yield+Feed_Vol+RS_cumulo+Cell_conc._in terval+rab_integral+RQ_integral+Accum._Yield+Arg_conc.+emis sion_O2+emission_CO2; 0.482, 0.791, tmp_cumulo+pH+pH_cumulo +PL+PL_cumulo+AN+interval_yield+Feed_Vol+RS_cumulo+rab_inte gral+RQ_integral+Arg_conc.+emission_02+emission_CO2+interva l_02; 0.495, 0.791, pH+PL+PL_cumulo+AN+AN_cumulo+Feed_Vol+R S_cumulo+Cell_conc._interval+rab_integral+RQ_integral+Accum. _Yield+Arg_conc.+emission_O2+emission_CO2; 0.482, 0.791, pH +PL_cumulo+AN+AN_cumulo+interval_yield+Feed_Vol+RS_cumulo+r ab_integral+RQ_integral+Accum._Yield+Arg_conc.+emission_O2+ emission_CO2+consum_O2+interval_O2; 0.531, 0.791, tmp+pH+AN _cumulo+Feed_Vol+RS_cumulo+rab_integral+RQ_integral+Arg_con c.+emission_O2+emission_CO2+generate_CO2; 0.495, 0.791, tmp +pH_cumulo+PL_cumulo+AN+AN_cumulo+interval_yield+Feed_Vol+R S_cumulo+Cell_conc._interval+rab_integral+RQ_integral+Arg_c onc.+emission_O2+emission_CO2; 0.519, 0.791, tmp+tmp_cumulo +pH+AN_cumulo+Feed_Vol+RS_cumulo+rab_integral+RQ_integral+A rg_conc.+emission_O2+emission_CO2+interval_O2; 0.495, 0.791, tmp_cumulo+pH+AN+interval_yield+Feed_Vol+RS_cumulo+Cell_co nc._interval+rab_integral+RQ_integral+Accum._Yield+Arg_conc. +emission_O2+emission_CO2+generate_CO2; 0.508, 0.791, pH_cu mulo+PL+AN+AN_cumulo+interval_yield+Feed_Vol+RS_cumulo+Cell _conc._interval+rab_integral+RQ_integral+Arg_conc.+emission _O2+emission_CO2; 0.508, 0.791, pH+PL+AN+interval_yield+Fee d_Vol+RS_cumulo+Cell_conc._cumulo+Cell_conc._interval+rab_i ntegral+RQ_integral+Arg_conc.+emission_O2+emission_CO2; 0.5 30, 0.791, pH+pH_cumulo+AN+Feed_Vol+RS_cumulo+rab_integral+ RQ_integral+Accum._Yield+Arg_conc.+emission_O2+emission_CO 2; 0.519, 0.791, pH+AN+interval_yield+RS+Feed_Vol+Cell_conc. _interval+rab_integral+RQ_integral+Arg_conc.+emission_O2+em ission_CO2+interval_O2; 0.507, 0.791, tmp+tmp_cumulo+pH+PL+ PL_cumulo+AN+Feed_Vol+RS_cumulo+rab_integral+RQ_integral+Ar g_conc.+emission_O2+emission_CO2; 0.507, 0.791, tmp+tmp_cum ulo+pH+AN+AN_cumulo+Feed_Vol+RS_cumulo+Cell_conc._interval+ rab_integral+RQ_integral+Arg_conc.+emission_O2+emission_CO 2; 0.495, 0.791, tmp+pH+PL+AN+AN_cumulo+interval_yield+Feed _Vol+RS_cumulo+rab_integral+RQ_integral+Arg_conc.+emission_ O2+emission_CO2+generate_CO2; 0.507, 0.791, tmp+pH_cumulo+P L_cumulo+AN+AN_cumulo+Feed_Vol+RS_cumulo+rab_integral+RQ_in tegral+Accum._Yield+Arg_conc.+emission_O2+emission_CO2; 0.4 95, 0.791, tmp_cumulo+pH+PL+AN+AN_cumulo+Feed_Vol+RS_cumulo +rab_integral+RQ_integral+Arg_conc.+emission_O2+emission_CO 2+consum_O2+generate_CO2; 0.482, 0.791, pH+PL+PL_cumulo+AN+ AN_cumulo+Feed_Vol+RS_cumulo+rab_integral+RQ_integral+Accum. Yield+Arg_conc.+emission_O2+emission_CO2+consum_O2+interva l_O2; 0.519, 0.791, pH+PL_cumulo+AN_cumulo+Feed_Vol+RS_cumu lo+rab_integral+RQ_integral+Accum._Yield+Arg_conc.+emission _O2+emission_CO2+consum_02; 0.519, 0.791, pH+PL_cumulo+AN+F eed_Vol+RS_cumulo+rab_integral+RQ_integral+Accum._Yield+Arg _conc.+emission_O2+emission_CO2+consum_02; 0.495, 0.791, tm p+tmp_cumulo+pH+PL+AN+interval_yield+Feed_Vol+RS_cumulo+Cel l_conc._interval+rab_integral+RQ_integral+Arg_conc.+emissio n_O2+emission_CO2; 0.530, 0.791, pH+AN+Feed_Vol+RS_cumulo+r ab_integral+RQ_integral+Accum._Yield+Arg_conc.+emission_O2+ emission_CO2+consum_O2; 0.507, 0.791, tmp_cumulo+pH+pH_cumu lo+AN+AN_cumulo+interval_yield+Feed_Vol+RS_cumulo+rab_integ ral+RQ_integral+Arg_conc.+emission_O2+emission_CO2; 0.507, 0.791, pH+pH_cumulo+PL_cumulo+AN+interval_yield+Feed_Vol+RS _cumulo+Cell_conc._interval+rab_integral+RQ_integral+Arg_co nc.+emission_O2+emission_CO2; 0.482, 0.791, pH+AN+AN_cumulo +interval_yield+Feed_Vol+RS_cumulo+Cell_conc._interval+rab-integral+RQ_integral+Accum._Yield+Arg_conc.+emission_O2+emi ssion_CO2+consum_O2+interval_O2; 0.495, 0.791, tmp_cumulo+p H+PL+AN+AN_cumulo+Feed_Vol+RS_cumulo+rab_integral+RQ_integr al+Arg_conc.+emission_O2+eniission_CO2+consum_O2+interval_O 2; 0.507, 0.791, pH+PL+PL_cumulo+AN+interval_yield+Feed_Vol +RS_cumulo+Cell_conc._cumulo+rab_integral+RQ_integral+Arg_c onc.+emission_O2+emission_CO2; 0.507, 0.791, tmp+pH+AN+AN_c umulo+Feed_Vol+RS_cumulo+rab_integral+RQ_integral+Arg_conc. +emission_O2+emission_CO2+consum_O2+interval_02; 0.530, 0.7 91, tmp+pH+PL+AN_cumulo+Feed_Vol+RS_cumulo+rab_integral+RQ_ integral+Arg_conc.+emission_O2+emission_CO2; 0.507, 0.791, tmp+pH+PL_cumulo+AN+interval_yield+Feed_Vol+RS_cumulo+rab_i ntegral+RQ_integral+Accum._Yield+Arg_conc.+emission_O2+emis sion_CO2; 0.507, 0.791, pH_cumulo+PL+AN+interval_yield+Feed _Vol+RS_cumulo+Cell_conc._interval+rab_integral+RQ_integral +Accum._Yield+Arg_conc.+emission_O2+emission_CO2; 0.530, 0. 791, pH+PL_cumulo+AN_cumulo+Feed_Vol+RS_cumulo+rab_integral +Accum._Yield+Arg conc.+emission_O2+emission_CO2+consum_O2; 0.495, 0.791, tmp+pH+pH_cumulo+PL+PL_cumulo+AN+Feed_Vol+RS cumulo+rab_integral+RQ_integral+Arg_conc.+emission_O2+emis sion_CO2+consum_O2; 0.495, 0.791, pH+AN+AN_cumulo+interval_ yield+Feed_Vol+RS_cumulo+Cell_conc._cumulo+Cell_conc._inter val+rab_integral+RQ_integral+Accum._Yield+Arg_conc.+emissio n_O2+emission_CO2; 0.495, 0.791, tmp_cumulo+pH+PL+AN+AN_cum ulo+Feed_Vol+RS_cumulo+Cell_conc._cumulo+rab_integral+RQ_in tegral+Arg_conc.+emission_O2+emission_CO2+interval_O2; 0.49 5, 0.791, tmp+tmp_cumulo+pH+PL_cumulo+AN+AN_cumulo+interval _yield+Feed_Vol+RS_cumulo+rab_integral+RQ_integral+Arg_conc. +emission_O2+emission_CO2; 0.482, 0.791, tmp_cumulo+pH+PL+P L_cumulo+AN+AN_cumulo+Feed_Vol+RS_cumulo+rab_integral+RQ_in tegral+Accum._Yield+Arg_conc.+emission_O2+emission_CO2+inte rval_O2; 0.495, 0.791, tmp_cumulo+pH+PL+AN+AN_cumulo+Feed_V ol+RS_cumulo+rab_integral+RQ_integral+Arg_conc.+emission_O2 +emission_CO2+interval_O2+generate_CO2; 0.482, 0.791, pH+pH _cumulo+PL+PL_cumulo+AN+AN_cumulo+Feed_Vol+RS_cumulo+Cell_c onc._cumulo+rab_integral+RQ_integral+Arg_conc.+emission_O2+ emission_CO2+interval_O2; 0.482, 0.791, tmp_cumulo+pH+PL+AN +interval_yield+Feed_Vol+RS_cumulo+Cell_conc._interval+rab_ integral+RQ_integral+Accum._Yield+Arg_conc.+emission_O2+emi ssion_CO2+interval_O2; 0.519, 0.791, pH+pH_cumulo+PL_cumulo +AN+interval_yield+Feed_Vol+RS_cumulo+rab_integral+RQ_integ ral+Arg_conc.+emission_O2+emission_CO2; 0.507, 0.791, tmp+p H+PL cumulo+AN cumulo+interval yield+Feed Vol+RS cumulo+rab _integral+RQ_integral+Accum._Yield+Arg_conc.+emission-O2+em ission_CO2; 0.507, 0.791, pH+pH_cumulo+AN+AN_cumulo+Feed_Vo l+RS_cumulo+rab_integral+RQ_integral+Accum._Yield+Arg_conc. +emission_O2+emission_CO2+generate_CO2; 0.495, 0.791, tmp_C umulo+pH+PL+AN+interval_yield+Feed_Vol+RS_cumulo+Cell_conc. _interval+rab_integral+RQ_integral+Arg_conc.+emission_O2+em ission_CO2+consum_O2; 0.519, 0.791, tmp+pH+PL_cumulo+AN+Fee d_Vol+RS_cumulo+rab_integral+RQ_integral+Arg_conc.+emission _O2+emission_CO2+consum_O2; 0.495, 0.791, tmp+pH+PL+AN+AN_c umulo+Feed_Vol+RS_cumulo+rab_integral+RQ_integral+Arg-conc. +emission_O2+emission_CO2+consum_O2+interval_02; 0.468, 0.7 91, tmp+pH+pH_cumulo+PL+PL_cumulo+AN+interval_yield+Feed_Vo l+RS_cumulo+rab_integral+RQ_integral+Accum._Yield+Arg-conc. +emission_O2+emission_CO2+consum_O2; 0.495, 0.791, tmp+pH+P L+PL_cumulo+AN+Feed_Vol+RS_cumulo+rab_integral+RQ_integral+ Arg_conc.+emission_O2+emission_CO2+consum_O2+generate_CO2; 0.495, 0.791, pH+pH_cumulo+PL+PL_cumulo+AN+interval_yield+F eed_Vol+RS_cumulo+rab_integral+RQ_integral+Accum._Yield+Arg _conc.+emission_O2+emission_CO2; 0.519, 0.791, pH+PL_cumulo +AN_cumulo+Feed_Vol+RS_cumulo+Cell_conc._cumulo+rab_integra l+RQ_integral+Arg_conc.+emission_O2+emission_CO2+interval_O 2; 0.507, 0.791, pH+AN+AN_cumulo+Feed_Vol+RS_cumulo+Cell_co nc._cumulo+rab_integral+RQ_integral+Accum._Yield+Arg_conc.+ emission_O2+emission_CO2+generate_CO2; 0.495, 0.791, tmp+pH +PL_cumulo+AN+AN_cumulo+interval_yield+Feed_Vol+RS_cumulo+r ab_integral+RQ_integral+Arg_conc.+emission_O2+emission_CO2+ generate_CO2; 0.495, 0.791, tmp_cumulo+pH+PL_cumulo+AN+AN_c umulo+Feed_Vol+RS_cumulo+Cell_conc._interval+rab_integral+R Q_integral+Accum._Yield+Arg_conc.+emission_O2+emission_CO2; 0.507, 0.791, pH+PL_cumulo+AN+interval_yield+Feed_Vol+RS_c umulo+Cell_conc._interval+rab_integral+RQ_integral+Arg_conc. +emission_O2+emission_CO2+interval_O2; 0.507, 0.791, pH_cum ulo+PL+PL_cumulo+AN+AN_cumulo+Feed_Vol+RS_cumulo+Cell_conc. _cumulo+rab_integral+RQ_integral+Arg_conc.+emission_O2+emis sion_CO2; 0.507, 0.791, tmp_cumulo+pH+AN+AN_cumulo+Feed_Vol +RS_cumulo+rab_integral+RQ_integral+Arg_conc.+emission_O2+e mission_CO2+interval_O2+generate_CO2; 0.482, 0.791, tmp_cum ulo+pH+pH_cumulo+AN+AN_cumulo+interval_yield+Feed_Vol+RS_cu mulo+Cell_conc._interval+rab_integral+RQ_integral+Accum._Yi eld+Arg_conc.+emission_O2+emission_CO2; 0.495, 0.791, pH+AN +interval_yield+Feed_Vol+RS_cumulo+Cell_conc._interval+rab-integral+RQ_integral+Accum._Yield+Arg_conc.+emission_O2+emi ssion_CO2+consum_O2+interval_O2; 0.495, 0.791, tmp_cumulo+p H+pH_cumulo+PL+PL_cumulo+AN+Feed_Vol+RS_cumulo+rab_integral +RQ_integral+Arg_conc.+emission_O2+emission_CO2+consum_O2; 0.507, 0.791, pH+pH_cumulo+AN+interval_yield+RS+Feed_Vol+Ce ll_conc._interval+rab_integral+RQ_integral+Accum._Yield+Arg _conc.+emission_O2+emission_CO2; 0.507, 0.791, tmp+pH+AN+AN _cumulo+Feed_Vol+RS_cumulo+rab_integral+RQ_integral+Accum._ Yield+Arg_conc.+emission_O2+emission_CO2+consum _O2; 0.507, 0.791, tmp+pH+PL+PL_cumulo+AN+Feed_Vol+RS_cumulo+Cell_conc. _interval+rab_integral+RQ_integral+Arg_conc.+emission_O2+em ission_CO2; 0.507, 0.791, pH+AN+interval_yield+Feed_Vol+RS_ cumulo+Cell_conc._cumulo+Cell_conc._interval+rab_integral+R Q_integral+Accum._Yield+Arg_conc.+emission_O2+emission_CO2; 0.507, 0.791, tmp_cumulo+pH+PL_cumulo+AN_cumulo+interval_y ield+Feed_Vol+RS_cumulo+rab_integral+RQ_integral+Accum._Yie ld+Arg_conc.+emission_O2+emission_CO2; 0.507, 0.791, tmp+pH +AN+AN_cumulo+Feed_Vol+RS_cumulo+rab_integral+RQ_integral+A rg_conc.+emission_O2+emission_CO2+interval_O2+generate_CO2; 0.494, 0.791, tmp+tmp_cumulo+pH+PL_cumulo+AN+AN_cumulo+Fee d_Vol+RS_cumulo+rab_integral+RQ_integral+Accum._Yield+Arg_c onc.+emission_O2+emission_CO2; 0.530, 0.791, pH+AN+Feed_Vol +RS_cumulo+rab_integral+RQ_integral+Arg_conc.+emission_O2+e mission_CO2+consum_O2+generate_CO2; 0.507, 0.791, tmp_cumul o+pH+PL_cumulo+AN+interval_yield+Feed_Vol+RS_cumulo+Cell_co nc._interval+rab_integral+RQ_integral+Arg_conc.+emission_O2 +emission_CO2; 0.481, 0.791, pH+AN+AN_cumulo+interval_yield +Feed_Vol+RS_cumulo+Cell_conc._interval+rab_integral+RQ_int egral+Accum._Yield+Arg_conc.+emission_O2+emission_CO2+inter val_O2+generate_CO2; 0.481, 0.791, tmp_cumulo+pH+PL_cumulo+ AN+AN_cumulo+interval_yield+Feed_Vol+RS_cumulo+rab_integral +RQ_integral+Accum._Yield+Arg_conc.+emission_O2+emission_CO 2+consum_O2; 0.530, 0.791, pH+AN+AN_cumulo+RS+Feed_Vol+RS_c umulo+rab_integral+RQ_integral+Arg_conc.+emission_O2+emissi on_CO2; 0.530, 0.791, tmp+pH+AN+Feed_Vol+RS_cumulo+rab_inte gral+RQ_integral+Arg_conc.+emission_O2+emission_CO2+consum_ 02; 0.481, 0.791, tmp+pH+pH_cumulo+PL+PL_cumulo+AN+interval _yield+Feed_Vol+RS_cumulo+rab_integral+RQ_integral+Arg_conc. +emission_O2+emission_CO2+interval_O2; 0.494, 0.791, tmp+pH _cumulo+PL_cumulo+AN+AN_cumulo+interval_yield+FeedVol+RS_c umulo+rab_integral+RQ_integral+Accum._Yield+Arg_conc.+emiss ion_O2+emission_CO2; 0.494, 0.791, tmp+pH+AN+interval_yield +Feed_Vol+RS_cumulo+Cell_conc._interval+rab_integral+RQ_int egral+Accum._Yield+Arg_conc.+emission_O2+emission_CO2+consu m_O2; 0.507, 0.791, pH_cumulo+PL_cumulo+AN+AN_cumulo+interv al_yield+Feed_Vol+RS_cumulo+rab_integral+RQ_integral+Arg_co nc.+emission_O2+emission_CO2+generate_CO2; 0.518, 0.791, pH +AN+AN_cumulo+interval_yield+RS+Feed_Vol+Cell_conc._interva l+rab_integral+RQ_integral+Arg_conc.+emission_O2+emission_C 02; 0.494, 0.791, pH+PL+PL_cumulo+AN+interval_yield+Feed_Vo l+RS_cumulo+rab_integral+RQ_integral+Accum._Yield+Arg_conc. +emission_O2+emission_CO2+generate_CO2; 0.481, 0.791, pH+pH _cumulo+PL+PL_cumulo+AN+interval_yield+Feed_Vol+RS_cumulo+C ell_conc._interval+rab_integral+RQ_integral+Arg_conc.+emiss ion_O2+emission_CO2+consum_O2; 0.494, 0.791, tmp+pH+PL+PL_c umulo+AN+Feed_Vol+RS_cumulo+Cell_conc._interval+rab_integra l+RQ_integral+Arg_conc.+emission_O2+emission_CO2+consum_O2; 0.494, 0.791, tmp+pH+PL+AN+AN_cumulo+Feed_Vol+RS_cumulo+ra b_integral+RQ integral+Arg_conc.+emission_O2+emission_CO2+i nterval_O2+generate_CO2; 0.494, 0.791, pH+PL+PL_cumulo+AN+i nterval_yield+Feed_Vol+RS_cumulo+rab_integral+RQ_integral+A rg_conc.+emission_O2+emission_CO2+interval_O2+generate_CO2; 0.506, 0.791, pH+AN+AN_cumulo+Feed_Vol+RS_cumulo+Cell_conc. _interval+rab_integral+RQ_integral+Arg_conc.+emission_O2+em ission_CO2+consum_O2+generate_CO2; 0.529, 0.791, pH+pH_cumu lo+AN+Feed_Vol+RS_cumulo+rab_integral+RQ_integral+Arg_conc. +emission_O2+emission_CO2+interval_O2; 0.506, 0.791, tmp_cu mulo+pH+AN+interval_yield+Feed_Vol+RS_cumulo+Cell_conc._int erval+rab_integral+RQ _integral+Arg _conc.+emission_O2+emissi on_CO2+interval_O2; 0.506, 0.791, tmp+pH+AN+interval_yield+ Feed_Vol+RS_cumulo+Cell_conc._interval+rab_integral+RQ_inte gral+Arg_conc.+emission_O2+emission_CO2+generate_CO2; 0.494, 0.791, tmp_cumulo+pH+pH_cumulo+PL_cumulo+AN+AN_cumulo+inte rval_yield+Feed_Vol+RS_cumulo+rab_integral+RQ_integral+Arg_ conc.+emission_O2+emission_CO2; 0.481, 0.791, tmp+pH+PL+PL_ cumulo+AN+AN_cumulo+Feed_Vol+RS_cumulo+rab_integral+RQ_inte gral+Accum._Yield+Arg_conc.+emission_O2+emission_CO2+interv al_O2; 0.494, 0.791, tmp+pH+pH_cumulo+PL+AN+AN_cumulo+Feed_ Vol+RS_cumulo+rab_integral+RQ_integral+Arg_conc.+emission_O 2+emission_CO2+interval_O2; 0.529, 0.791, tmp+pH+AN_cumulo+ Feed_Vol+RS_cumulo+rab_integral+RQ_integral+Arg_conc.+emiss ion_O2+emission_CO2+interval_O2; 0.518, 0.791, pH+PL_cumulo +AN_cumulo+interval_yield+Feed_Vol+RS_cumulo+Cell_conc._int erval+rab_integral+Accum._Yield+Arg_conc.+emission_O2+emiss ion_CO2; 0.518, 0.791, pH+AN+interval_yield+Feed_Vol+RS_cum ulo+Cell_conc._cumulo+Cell_conc._interval+rab_integral+RQ_i ntegral+Arg_conc.+emission_O2+emission_CO2; 0.506, 0.791, p H+PL_cumulo+AN+interval_yield+Feed_Vol+RS_cumulo+Cell_conc. _interval+rab_integral+RQ_integral+Arg_conc.+emission_O2+em ission_CO2+consum_O2; 0.494, 0.791, tmp_cumulo+pH+pH_cumulo +PL+PL_cumulo+AN+interval_yield+Feed_Vol+RS_cumulo+rab_inte gral+RQ_integral+Arg_conc.+emission_O2+emission_CO2; 0.506, 0.791, pH+AN+AN_cumulo+interval_yield+Feed_Vol+RS_cumulo+C ell_conc._cumulo+Cell_conc._interval+rab_integral+RQ_integr al+Arg_conc.+emission_O2+emission_CO2; 0.467, 0.791, pH_cum ulo+PL+PL_cumulo+AN+AN_cumulo+interval_yield+Feed_Vol+RS_cu mulo+Cell_conc._interval+rab_integral+RQ_integral+Accum._Yi eld+Arg_conc.+emission_O2+emission_CO2+interval_O2; 0.494, 0.791, pH+pH_cumulo+PL+AN+AN_cumulo+interval_yield+Feed_Vol +RS_cumulo+rab_integral+RQ_integral+Arg_conc.+emission_O2+e mission_CO2+interval_O2; 0.467, 0.791, tmp+pH+PL+PL_cumulo+ AN+AN_cumulo+interval_yield+Feed_Vol+RS_cumulo+Cell_conc._c umulo+rab_integral+RQ_integral+Arg_conc.+emission_O2+emissi on_CO2+interval_O2; 0.494, 0.791, pH+pH_cumulo+PL+PL_cumulo +AN+AN_cumulo+Feed_Vol+RS_cumulo+Cell_conc._interval+rab_in tegral+RQ_integral+Arg_conc.+emission_O2+emission_CO2; 0.45 3, 0.791, tmp+tmp_cumulo+pH+PL+PL_cumulo+AN+AN_cumulo+inter val_yield+Feed_Vol+RS_cumulo+rab_integral+RQ_integral+Accum. _Yield+Arg_conc.+emission_O2+emission_CO2+consum_O2; 0.518, 0.791, tmp_cumulo+pH+AN+Feed_Vol+RS_cumulo+Cell_conc._inte rval+rab_integral+RQ_integral+Accum._Yield+Arg_conc.+emissi on_O2+emission_CO2; 0.481, 0.791, pH+pH_cumulo+AN+AN_cumulo +interval_yield+Feed_Vol+RS_cumulo+Cell_conc._interval+rab_ integral+RQ_integral+Accum._Yield+Arg_conc.+emission_O2+emi ssion_CO2+interval_O2; 0.529, 0.791, tmp_cumulo+pH+PL+AN_cu mulo+Feed_Vol+RS_cumulo+rab_integral+RQ_integral+Arg_conc.+ emission_O2+emission_CO2; 0.506, 0.791, tmp_cumulo+pH+AN+AN _cumulo+Feed_Vol+RS_cumulo+rab_integral+RQ_integral+Arg_con c.+emission_O2+emission_CO2+consum_O2+generate_CO2; 0.506, 0.791, pH_cumulo+PL+PL_cumulo+AN+AN_cumulo+Feed_Vol+RS_cumu lo+rab_integral+RQ_integral+Accum._Yield+Arg_conc.+emission _O2+emission_CO2; 0.506, 0.791, tmp_cumulo+pH+PL+AN+interva l_yield+Feed_Vol+RS_cumulo+Cell_conc._cumulo+rab_integral+R Q_integral+Arg_conc.+emission_O2+emission_CO2; 0.494, 0.791, tmp+pH+PL_cumulo+AN+AN_cumulo+Feed_Vol+RS_cumulo+Cell_conc. _interval+rab_integral+RQ_integral+Accum._Yield+Arg_conc.+e mission_O2+emission_CO2; 0.506, 0.791, pH+AN+interval_yield +RS+Feed_Vol+Cell_conc._interval+rab_integral+RQ_integral+A ccum._Yield+Arg_conc.+emission_O2+emission_CO2+consum-O2; 0. 518, 0.791, tmp+pH+AN+Feed_Vol+RS_cumulo+Cell_conc._interva l+rab_integral+RQ_integral+Accum._Yield+Arg_conc.+emission_ O2+emission_CO2; 0.529, 0.791, pH_cumulo+AN+AN_cumulo+Feed_ Vol+RS_cumulo+Cell_conc._interval+rab_integral+RQ_integral+ Arg_conc.+emission_O2+emission_CO2; 0.506, 0.791, tmp+pH+AN _cumulo+interval_yield+Feed_Vol+RS_cumulo+Cell_conc._interv al+rab_integral+RQ_integral+Accum._Yield+Arg_conc.+emission _O2+emission_CO2; 0.481, 0.791, pH+PL+PL_cumulo+AN+AN_cumul o+Feed_Vol+RS_cumulo+Cell_conc._cumulo+rab_integral+RQ_inte gral+Accum._Yield+Arg_conc.+emission_O2+emission_CO2+consum _O2; 0.506, 0.791, pH+PL+PL_cumulo+AN+Feed_Vol+RS_cumulo+Ce ll_conc._interval+rab_integral+RQ_integral+Accum._Yield+Arg conc.+emission_O2+emission_CO2; 0.506, 0.791, pH+pH_cumulo +AN+AN_cumulo+Feed _Vol+RS_cumulo+Cell _conc. _interval+rab_in tegral+RQ_integral+Accum._Yield+Arg_conc.+emission_O2+emiss ion_CO2; 0.494, 0.791, tmp+pH+PL+AN+AN_cumulo+Feed_Vol+RS_c umulo+Cell_conc._cumulo+rab_integral+RQ_integral+Arg_conc.+ emission _O2+emission_CO2+interval_O2; 0.518, 0.791, tmp+tmp _cumulo+pH_cumulo+AN+AN_cumulo+Feed_Vol+RS_cumulo+rab_integ ral+RQ_integral+Arg_conc.+emission_O2+emission_CO2; 0.506, 0.791, tmp+tmp_cumulo+pH+pH_cumulo+AN+AN_cumulo+Feed_Vol+RS _cumulo+rab_integral+RQ_integral+Arg_conc.+emission_O2+emis sion_CO2; 0.529, 0.791, pH_cumulo+PL_cumulo+AN+interval_yie ld+Feed_Vol+RS_cumulo+rab_integral+RQ_integral+Arg_conc.+em ission_O2+emission_CO2; 0.506, 0.791, pH+AN+interval_yield+ RS+Feed_Vol+Cell_conc._interval+rab_integral+RQ_integral+Ac cum._Yield+Arg_conc.+emission_O2+emission_CO2+generate_CO2; 0.506, 0.791, pH+AN+AN_cumulo+interval_yield+Feed_Vol+RS_c umulo+rab_integral+RQ_integral+Accum._Yield+Arg_conc.+emiss ion_O2+emission_CO2+consum_O2; 0.467, 0.791, pH+pH_cumulo+P L_cumulo+AN+AN_cumulo+interval_yield+Feed_Vol+RS_cumulo+Cel l_conc._interval+rab_integral+RQ_integral+Accum._Yield+Arg_ conc.+emission_O2+emission_CO2+interval_O2; 0.494, 0.791, p H+PL_cumulo+AN_cumulo+interval_yield+Feed_Vol+RS_cumulo+Cel l_conc._interval+rab_integral+RQ_integral+Accum._Yield+Arg_ conc.+emission_O2+emission_CO2+interval_O2; 0.481, 0.791, p H+PL+PL_cumulo+AN+interval_yield+Feed_Vol+RS_cumulo+Cell_co nc._interval+rab_integral+RQ_integral+Accum._Yield+Arg_conc. +emission_O2+emission_CO2+generate_CO2; 0.481, 0.790, tmp_c umulo+pH_cumulo+PL+PL_cumulo+AN+AN_cumulo+interval_yield+Fe ed_Vol+RS_cumulo+rab_integral+RQ_integral+Arg_conc.+emissio n_O2+emission_CO2+consum_O2; 0.481, 0.790, pH+PL+PL_cumulo+ AN+AN_cumulo+interval_yield+Feed_Vol+RS_cumulo+Cell_conc._i nterval+rab_integral+RQ_integral+Arg_conc.+emission_O2+emis sion_CO2+generate_CO2; 0.494, 0.790, pH+PL_cumulo+AN+AN_cum ulo+Feed_Vol+RS_cumulo+Cell_conc._interval+rab_integral+RQ_ integral+Accum._Yield+Arg_conc. +emission_O2+emission_CO2+co nsum_O2; 0.506, 0.790, tmp+pH+AN+interval_yield+RS+Feed_Vol +Cell_conc._interval+rab_integral+RQ_integral+Accum._Yield+ Arg_conc.+emission_O2+emission_CO2; 0.493, 0.790, tmp_cumul o+pH+PL_cumulo+AN+AN_cumulo+interval_yield+Feed_Vol+RS_cumu lo+rab_integral+RQ_integral+Arg_conc.+emission_O2+emission-CO2+generate_CO2; 0.493, 0.790, pH+pH_cumulo+PL+AN+AN_cumul o+Feed_Vol+RS_cumulo+rab_integral+RQ_integral+Accum._Yield+ Arg_conc.+emission_O2+emission_CO2+interval_O2; 0.506, 0.79 0, pH+PL_cumulo+AN_cumulo+interval_yield+Feed_Vol+RS_cumulo +Cell_conc._interval+rab_integral+Accum._Yield+Arg_conc.+em ission_O2+emission_CO2+interval_O2; 0.506, 0.790, pH+PL_cum ulo+AN+interval_yield+Feed_Vol+RS_cumulo+rab_integral+RQ_in tegral+Accum._Yield+Arg_conc.+emission_O2+emission_CO2+cons um_O2; 0.493, 0.790, tmp_cumulo+pH+PL+AN+AN_cumulo+Feed_Vol +RS_cumulo+Cell_conc._interval+rab_integral+RQ_integral+Arg _conc.+emission_O2+emission_CO2+generate_CO2; 0.506, 0.790, pH+PL+AN+interval_yield+Feed_Vol+RS_cumulo+rab_integral+RQ integral+Accum._Yield+Arg_conc.+emission_O2+emission_CO2+i nterval_O2; 0.493, 0.790, tmp+pH+PL+AN+AN_cumulo+Feed_Vol+R S_cumulo+rab_integral+RQ_integral+Arg_conc.+emission_O2+emi ssion_CO2+consum_O2+generate_CO2; 0.506, 0.790, tmp+tmp_cum ulo+pH+AN+interval_yield+Feed_Vol+RS_cumulo+Cell_conc._inte rval+rab_integral+RQ_integral+Arg_conc.+emission_O2+emissio n_CO2; 0.480, 0.790, pH+pH_cumulo+PL+PL_cumulo+AN+AN_cumulo +Feed_Vol+RS_cumulo+rab_integral+RQ_integral+Arg_conc.+emis sion_O2+emission_CO2+consum_O2+interval_O2; 0.506, 0.790, p H_cumulo+PL_cumulo+AN_cumulo+interval_yield+Feed_Vol+RS_cum ulo+Cell_conc._interval+rab_integral+RQ_integral+Accum._Yie ld+Arg_conc.+emission_O2+emission_CO2; 0.493, 0.790, pH+PL+ PL_cumulo+AN+interval_yield+Feed_Vol+RS_cumulo+Cell_conc._i nterval+rab_integral+RQ_integral+Arg_conc.+emission_O2+emis sion_CO2+generate_CO2; 0.493, 0.790, tmp_cumulo+pH+AN+AN_cu mulo+Feed_Vol+RS_cumulo+Cell_conc._interval+rab_integral+RQ integral+Accum._Yield+Arg_conc.+emission_O2+emission_CO2+g enerate_CO2; 0.506, 0.790, tmp_cumulo+pH+PL+AN+interval_yie ld+Feed_Vol+RS_cumulo+rab_integral+RQ_integral+Arg_conc.+em ission_O2+emission_CO2+generate_CO2; 0.480, 0.790, tmp+pH+P L_cumulo+AN+AN_cumulo+interval_yield+Feed_Vol+RS_cumulo+rab integral+RQ_integral+Accum._Yield+Arg_conc.+emission_O2+em ission_CO2+consum_O2; 0.493, 0.790, tmp_cumulo+pH+PL+AN+AN_ cumulo+Feed_Vol+RS_cumulo+Cell_conc._interval+rab_integral+ RQ_integral+Arg_conc.+emission_O2+emission_CO2+interval_O2; 0.493, 0.790, tmp+tmp_cumulo+pH+AN+AN_cumulo+Feed_Vol+RS_c umulo+rab_integral+RQ_integral+Accum._Yield+Arg_conc.+emiss ion_O2+emission_CO2+generate_CO2; 0.529, 0.790, tmp+pH+pH_c umulo+AN_cumulo+Feed_Vol+RS_cumulo+rab_integral+RQ_integral +Arg_conc.+emission_O2+emission_CO2; 0.493, 0.790, pH+PL+AN +AN_cumulo+Feed_Vol+RS_cumulo+rab_integral+RQ_integral+Accu m._Yield+Arg_conc.+emission_O2+emission_CO2+interval_O2+gen erate_CO2; 0.493, 0.790, pH+PL_cumulo+AN+AN_cumulo+interval _yield+Feed_Vol+RS_cumulo+Cell_conc._cumulo+rab_integral+RQ integral+Arg_conc.+emission_O2+emission_CO2+interval_O2; 0. 539, 0.790, PL_cumulo+AN_cumulo+Feed_Vol+RS_cumulo+Cell_con c._cumulo+rab_integral+RQ_integral+Arg_conc.+emission_O2+em ission_CO2; 0.480, 0.790, pH+pH_cumulo+PL+PL_cumulo+AN+AN_c umulo+Feed_Vol+RS_cumulo+rab_integral+RQ_integral+Arg_conc. +emission_O2+emission_CO2+consum _O2+generate_CO2; 0.539, 0. 790, pH+PL_cumulo+AN_cumulo+Feed_Vol+RS_cumulo+rab_integral +Arg_conc.+emission_O2+emission_CO2+consum_O2; 0.505, 0.790, pH+PL_cumulo+AN_cumulo+interval_yield+Feed_Vol+RS_cumulo+r ab_integral+RQ_integral+Accum._Yield+Arg_conc.+emission_O2+ emission_CO2+consum_O2; 0.528, 0.790, pH+PL+AN_cumulo+Feed_ Vol+RS_cumulo+rab_integral+RQ_integral+Accum._Yield+Arg_con c.+emission_O2+emission_CO2; 0.517, 0.790, tmp_cumulo+pH+AN +interval_yield+Feed_Vol+RS_cumulo+rab_integral+RQ_integral +Arg_conc.+emission_O2+emission_CO2+interval_O2; 0.480, 0.7 90, pH+PL_cumulo+AN+AN_cumulo+interval_yield+Feed_Vol+RS_cu mulo+rab_integral+RQ_integral+Accum._Yield+Arg_conc.+emissi on_O2+emission_CO2+consum_O2+generate_CO2; 0.528, 0.790, pH +AN+AN_cumulo+RS+Feed_Vol+Cell_conc._interval+rab_integral+ RQ_integral+Arg_conc.+emission_O2+emission_CO2; 0.505, 0.79 0, tmp+tmp_cumulo+pH+PL+AN+Feed_Vol+RS_cumulo+rab_integral+ RQ_integral+Arg_conc.+emission_O2+emission_CO2+consum_O2; 0. 466, 0.790, pH+pH_cumulo+PL+PL_cumulo+AN+AN_cumulo+interval _yield+Feed_Vol+RS_cumulo+Cell_conc._interval+rab_integral+ RQ_integral+Accum._Yield+Arg_conc.+emission_O2+emission_CO 2; 0.505, 0.790, tmp_cumulo+pH+AN+interval_yield+RS+Feed_Vo l+Cell_conc._interval+rab_integral+RQ_integral+Accum.-Yield +Arg_conc.+emission_O2+emission_CO2; 0.466, 0.790, pH+pH_cu mulo+PL+PL_cumulo+AN+AN_cumulo+interval_yield+Feed_Vol+RS_c umulo+Cell_conc._interval+rab_integral+RQ_integral+Arg_conc. +emission_O2+emission_CO2+consum_O2; 0.517, 0.790, pH+PL+PL _cumulo+AN_cumulo+Feed_Vol+RS_cumulo+Cell_conc._cumulo+rab_ integral+RQ_integral+Arg_conc.+emission_O2+emission_CO2; 0. 493, 0.790, pH+PL_cumulo+AN+AN_cumulo+Feed_Vol+RS_cumulo+Ce ll_conc. _cumulo+rab_integral+RQ_integral+Accum._Yield+Arg_c onc.+emission_O2+emission_CO2+generate_CO2; 0.528, 0.790, p H+AN+interval_yield+Feed_Vol+RS_cumulo+Cell_conc._cumulo+ra b-integral+RQ-integral+Arg-conc.+emission-O2+emission-CO2; 0.517, 0.790, pH+pH_cumulo+AN+interval_yield+RS+Feed_Vol+Ce ll_conc._interval+rab_integral+RQ_integral+Arg_conc.+emissi on_O2+emission_CO2

## Claims

1. A control device that performs control of a culture condition in production of an organic compound by a fermentation method,
the control device executing processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate, and
the linear model and the target production amount being set for each time.

2. The control device according to claim 1, wherein the future time is a time when next time processing is executed.

3. The control device according to claim 1 or 2, determining a candidate corresponding to a production amount at the future time closest to the target production amount to be the optimum candidate.

4. The control device according to any one of claims 1 to 3, wherein
a plurality of the linear models are set for each time, and
the optimum candidate is determined out of the candidates using a sample statistical amount for each of the candidates based on a plurality of production amounts at the future time for each of the candidates and the target production amount.

5. The control device according to any one of claims 1 to 4, wherein the linear model is a multiple regression formula.

6. The control device according to any one of claims 1 to 5, wherein the organic compound is an amino acid.

7. The control device according to claim 6, wherein the amino acid is a basic amino acid.

8. The control device according to claim 7, wherein the basic amino acid is lysine or arginine.

9. The control device according to any one of claims 1 to 8, wherein the culture condition includes a condition on temperature, pH, or a phosphoric acid concentration.

10. A control method executed by a control device that performs control of a culture condition in production of an organic compound by a fermentation method,
the control device executing processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate, and
the linear model and the target production amount being set for each time.

11. A computer program causing a control device that performs control of a culture condition in production of an organic compound by a fermentation method to execute,
the computer program causing the control device to execute processing a plurality of times to acquire culture data, to calculate, using the acquired culture data, a plurality of candidates for a culture condition set in advance, and a linear model set in advance that outputs a production amount of an organic compound at a future time, the production amount at the future time for each of the candidates, to determine an optimum candidate out of the candidates using the calculated production amount at the future time for each of the candidates and a target production amount of the organic compound at the future time set in advance, and to change the culture condition to the determined candidate, and
the linear model and the target production amount being set for each time.

12. A method for producing an organic compound, the method comprising a step of producing an organic compound with a production system having the control device according to claim 1.

13. The method according to claim 12, wherein the organic compound is an amino acid.

14. The method according to claim 13, wherein the amino acid is a basic amino acid.

15. The method according to claim 14, wherein the basic acid is lysine or arginine.
